(19) Europäisches Patentamt
European Patent Office
Office européen des brevets

(11) EP 1 546 150 B1

(12) **EUROPEAN PATENT SPECIFICATION**

(45) Date of publication and mention of the grant of the patent:
**04.03.2009 Bulletin 2009/10**

(21) Application number: **03763972.1**

(22) Date of filing: **15.07.2003**

(51) Int Cl.:
***C07D 487/04*** *(2006.01)* ***A61K 31/407*** *(2006.01)*
***A61P 19/00*** *(2006.01)* ***A61P 35/00*** *(2006.01)*

(86) International application number:
**PCT/GB2003/002957**

(87) International publication number:
**WO 2004/007501 (22.01.2004 Gazette 2004/04)**

# (54) PYRROLE DERIVATIVES AS INHIBITORS OF CYTEINE PROTEASES

PYRROLDERIVATE ALS HEMMSTOFFE VON CYSTEIN PROTEASEN

DERIVES DE PYRROLE EN TANT QU'INHIBITEURS DE CYSTEINE PROTEASE

(84) Designated Contracting States:
**AT BE BG CH CY CZ DE DK EE ES FI FR GB GR HU IE IT LI LU MC NL PT RO SE SI SK TR**

(30) Priority: **16.07.2002 GB 0216525**
**25.07.2002 GB 0217239**
**15.10.2002 US 418524 P**

(43) Date of publication of application:
**29.06.2005 Bulletin 2005/26**

(73) Proprietor: **Amura Therapeutics Limited**
**Cambridge CB3 7AJ (GB)**

(72) Inventors:
- **QUIBELL, Martin,**
**c/o Amura Therapeutics Limited**
**Cambridge CB3 7AJ (GB)**
- **RAY, Peter Christopher**
**Port of Monteith,**
**By Kippen, FK8 3LF (GB)**
- **WATTS, John Paul,**
**c/o Amura Therapeutics Limited**
**Cambridge CB3 7AJ (GB)**

(74) Representative: **Clyde-Watson, Zöe**
**D Young & Co**
**120 Holborn**
**London EC1N 2DY (GB)**

(56) References cited:
**WO-A-00/69855** **WO-A-98/50533**
**WO-A-02/057270**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 546 150 B1

## Description

THIS INVENTION relates to compounds which are inhbitors across a broad range of cysteine proteases, to the use of these compounds, and to pharmaceutical compositions comprising them. Particular compounds of the invention are inhibitors of cathepsin K and related cysteine protesases of the CA clan. Furthermore, such compounds are useful for the *in vivo* therapeutic treatment of diseases in which participation of a cysteine protease is implicated.

Proteases form a substantial group of biological molecules which to date constitute approximately 2% of all the gene products identified following analysis of several completed genome sequencing programmes. Proteases have evolved to participate in an enormous range of biological processes, mediating their effect by cleavage of peptide amide bonds within the myriad of proteins found in nature. This hydrolytic action is performed by initially recognising, then binding to, particular three-dimensional electronic surfaces displaced by a protein, which aligns the bond for cleavage precisely within the protease catalytic site. Catalytic hydrolysis then commences through nucleophilic attack of the amide bond to be cleaved either *via* an amino acid side-chain of the protease itself, or through the action of a water molecule that is bound to and activated by the protease. Proteases in which the attacking nucleophile is the thiol side-chain of a Cys residue are known as cysteine proteases. The general classification of 'cysteine protease' contains many members found across a wide range of organisms from viruses, bacteria, protozoa, plants and fungi to mammals.

Cathepsin K and indeed many other crucial proteases belong to the papain-like CA C1 family. Cysteine proteases are classified into 'clans' based upon a similarity in the three-dimensional structure or a conserved arrangement of catalytic residues within the protease primary sequence. Additionally, 'clans' may be further classified into 'families' in which each protease shares a statistically significant relationship with other members when comparing the portions of amino acid sequence which constitute the parts responsible for the protease activity (see Barrett, A.J et al, in 'Handbook of Proteolytic Enzymes', Eds. Barrett, A. J., Rawlings, N. D., and Woessner, J. F. Publ. Academic Press, 1998**,** for a thorough discussion).

To date, cysteine proteases have been classified into five clans, CA, CB, CC, CD and CE (Barrett, A. J. *et al*, **1998).** A protease from the tropical papaya fruit 'papain' forms the foundation of clan CA, which currently contains over 80 distinct and complete entries in various sequence databases, with many more expected from the current genome sequencing efforts. Proteases of clan CA / family C1 have been implicated in a multitude of house-keeping roles and disease processes. e.g. human proteases such as cathepsin K (osteoporosis), cathepsin S (autoimmune disorders), cathepsin L (metastases), cathepsin B (metastases, arthritis), cathepsin F (antigen processing), cathepsin V (T-cell selection), dipeptidyl peptidase I (granulocyte serine protease activation) or parasitic proteases such as falcipain (malaria parasite *Plasmodium falciparum*) and cruzipain (*Trypanosoma cruzi* infection). Recently a bacterial protease, staphylopain (*S. aureus* infection) has also been tentatively assigned to clan CA. X-ray crystallographic structures are available for a range of the above mentioned proteases in complex with a range of inhibitors e.g. papain (PDB entries, 1pad, 1pe6, 1pip, 1pop, 4pad, 5pad, 6pad, 1ppp, 1the, 1csb, 1huc), cathepsin K (1au0, 1au2, 1au3, 1au4, latk, 1mem, 1bgo, 1ayw, 1ayu, 1nl6, 1nlj), cathepsin L (1cs8, 1mhw), cathepsin S (1glo, 1ms6 and currently on-hold but published McGrath, M. E. et al, Protein Science, 7, 1294-1302, 1998**),** cathepsin V (1fh0), dipeptidyl peptidase I (1jqp, 1k3b), cathepsin B (1gmy), cathepsin F (currently on-hold, but published Somoza, J. R. et al, J. Alo/. Biol., 322, 559-568, 2002**),** cruzain (a recombinant form of cruzipain see Eakin, A. E. *et al,* <u>268(9)</u>, 6115-6118, **1993)** (1ewp, 1aim, 2aim, 1F29, 1F2A, 1F2B, 1F2C), staphylopain (1cv8). Each of the structures displays a similar overall active-site topology, as would be expected by their 'clan' and 'family' classification and such structural similarity exemplifies one aspect of the difficulties involved in discovering a selective inhibitor of cathepsin K suitable for human use. However, subtle differences in terms of the depth and intricate shape of the active site groove of each CA C1 protease are evident, which may be exploited for selective inhibitor design. Additionally, many of the current substrate-based inhibitor complexes of CA C1 family proteases show a series of conserved hydrogen bonds between the inhibitor and the protease backbone, which contribute significantly to inhibitor potency. Primarily a bidentate hydrogen-bond is observed between the protease Gly66 (C=O)/ inhibitor N-H and the protease Gly66(NH)/inhibitor (C=O), where the inhibitor (C=O) and (NH) are provided by an amino acid residue <u>NH</u>-CHR<u>CO</u> that constitutes the S2 sub-site binding element within the inhibitor (see Berger, A. and Schecter, I. Philos. Trans. R. Soc. Lond. [Biol.], 257, 249-264, 1970 for a description of protease binding site nomenclature). A further hydrogen-bond between the protease main-chain (C=O) of asparagine or aspartic acid (158 to 163, residue number varies between proteases) and an inhibitor (N-H) is often observed, where the inhibitor (N-H) is provided by the S1 sub-site binding element within the inhibitor. Thus, the motif X-<u>NH</u>CHR<u>CO</u>-<u>NH</u>-Y is widely observed amongst the prior art substrate-based inhibitors of CA C1 proteases.

Cathepsin K is thought to be significant in diseases involving excessive loss of bone or cartilage. Bone consists of a protein matrix incorporating hydroxyapatite crystals. About 90% of the structural protein of the matrix is type I collagen, with the remainder comprising various non-collagenous proteins such as osteocalcin, proteoglycans, osteopontin, osteonectin, thrombospondin, fibronectin and bone sialoprotein.

Skeletal bone is not a static structure but continually undergoes a cycle of bone resorption and replacement. Bone resorption is carried out by osteoclasts, which are multinuclear cells of haematopoietic lineage. Osteoclasts adhere

to the bone surface and form a tight sealing zone. The membrane on the apical surface of the osteoclasts is folded so as to create a closed extracellular compartment between the osteoclast and the bone surface, which is acidified by proton pumps in the osteoclast membrane. Proteolytic enzymes are secreted into the compartment from the osteoclast. The high acidity in the compartment causes the hydroxyapatite at the surface of the bone to be dissolved and the proteolytic enzymes break down the protein matrix causing a resorption lacuna to be formed. Following bone resorption, osteoblasts produce a new protein matrix that is subsequently mineralised.

In disease states such as osteoporosis and Paget's disease, the bone resorption and replacement cycle is disrupted leading to a net loss of bone with each cycle. This leads to weakening of the bone and therefore to increased risk of bone fracture.

Cathepsin K is expressed at a high level in osteoclasts and is therefore thought to be essential for bone resorption. Therefore, selective inhibition of cathepsin K is likely to be effective in the treatment of diseases involving excessive bone loss. These include osteoporosis, gingival diseases such as gingivitis and periodontitis, Paget's disease, hypercalaemia of malignancy and metabolic bone disease.

In addition to osteoclasts, high levels of cathepsin K are also found in chondroclasts from the synovium of osteoarthritic patients. It therefore appears that cathepsin K inhibitors will be of use in the treatment of diseases involving matrix or cartilage degradation, in particular osteoarthritis and rheumatoid arthritis.

Elevated levels of cathepsin K are also found in metastatic neoplastic cells which suggests that cathepsin K inhibitors may also be useful for treating certain neoplastic diseases.

In the prior art, the development of cysteine protease inhibitors for human use has recently been an area of intense activity (*e.g.* see Bromme, D. and Kaleta, J., Curr. Pharm. Des., 8, 1639-1658, 2002**;** Kim, W. and Kang, K., Expert Opin. Ther. Patents, 12(3), 419-432, 2002**;** Leung-Toung, R. et al. Curr. Med. Chem., 9, 979-1002, 2002; Lecaille, F. et al., Chem. Rev., 102, 4459-4488, 2002; Hernandez, A. A. and Roush, W. R., Curr. Opin. Chem. Biol., 6, 459-465, 2002**).** Considering the CA C1 family members, particular emphasis has been placed upon the development of inhibitors of human cathepsins, primarily cathepsin K (osteoporosis), cathepsin S (autoimmune disorders), cathepsin L (metastases), cathepsin B (metastases, arthritis), cathepsin F (antigen processing), cathepsin V (T-cell selection) and dipeptidyl peptidase I (granulocyte serine protease activation), through the use of peptide and peptidomimetic nitriles (*e.g.* see WO-A-03041649, WO-A-03037892, WO-A-03029200, WO-A-02051983, WO-A-02020485, US-A-20020086996, WO-A-01096285, WO-A-0109910, WO-A-0051998, WO-A-0119816, WO-A-9924460, WO-A-0049008, WO-A-0048992, WO-A-0049007, WO-A-0130772, WO-A-0055125, WO-A-0055126, WO-A-0119808, WO-A-0149288, WO-A-0147886), linear and cyclic peptide and peptidomimetic ketones (*e.g.* see Veber, D. F. and Thompson, S. K., Curr. Opin. Drug Discovery Dev., 3(4), 362-369, 2000**,** WO-A-02092563, WO-A-02017924, WO-A-01095911, WO-A-0170232, WO-A-0178734, WO-A-0009653, WO-A-0069855, WO-A-0029408, WO-A-0134153 to WO-A-0134160, WO-A-0029408, WO-A-9964399, WO-A-9805336, WO-A-9850533), ketoheterocycles (*e.g.* see WO-A-02080920, WO-A-03042197, WO-A- WO-A-03024924, WO-A-0055144, WO-A-0055124), monobactams (*e.g.* see WO-A-0059881, WO-A-9948911, WO-A-0109169), α-ketoamides (*e.g.* see WO-A-03013518), cyanoamides (WO-A-01077073, WO-A-01068645), dihydro pyrimidines (*e.g.* see WO-A-02032879) and cyanoaminopyrimidines (*e.g.* see WO-A-03020278, WO-A-03020721). The prior art describes potent *in vitro* inhibitors, but also highlights the many difficulties in developing a human therapeutic. For example, WO-A-9850533 and WO-A-0029408 describe compounds that may be referred to as cyclic ketones and are inhibitors of cysteine proteases with a particular reference towards papain family proteases and as a most preferred embodiment, cathepsin K. WO-A-9850533 describes compounds subsequently detailed in the literature as potent inhibitors of cathepsin K with good oral bioavailability (Witherington, J., 'Tetrahydrofurans as Selective Cathepsin K Inhibitors', RSC meeting, Burlington House, London, 1999**).** The compounds of WO-A-9850533 were reported to bind to cathepsin K through the formation of a reversible covalent bond between the tetrahydrofuran carbonyl and the active site catalytic cysteine residue (Witherington, J., **1999).** Additionally, the same cyclic ketone compounds are described in WO-A-9953039 as part of a wide-ranging description of inhibitors of cysteine proteases associated with parasitic diseases, with particular reference to the treatment of malaria by inhibition of falcipain. However, subsequent literature describes the cyclic ketone compounds of WO-A-9850533 to be unsuitable for further developments or for full pharmacokinetic evaluation due to a physiochemical property of the inhibitors, the poor chiral stability of the α-aminoketone chiral centre (Marquis, R. W. et al, J. Med. Chem., 44(5), 725-736, 2001**).** WO-A-0069855 describes compounds that may also be referred to as cyclic ketones with particular reference towards inhibition of cathepsin S. The compounds of WO-A-0069855 are considered to be an advance on compounds of WO-A-9850533 due to the presence of the β-substituent on the cyclic ketone ring system that provides chiral stability to the α-carbon of the cyclic ketone ring system. However, the compounds of WO-A-0069855 and indeed those of WO-A-9850533 describe a requirement for the presence of the potential hydrogen-bonding motif X-NHCHRCO-NH-Y that is widely observed amongst the prior art substrate-based inhibitors of CA C1 proteases.

Our earlier patent application (WO-A-02057270) describes bicyclic compounds in which the chirality of the α-aminoketone is stabilised (for a review of energetic considerations within fused ring systems see Toromanoff, E. Tetrahedron Report No 96, 36, 2809-2931, 1980**).** These compounds do not contain the X-NHCHRCO-NH-Y motif and

yet the compounds are highly potent inhibitors across a broad range of CA C1 cysteine proteases. In particular, certain of the compounds are potent and selective cruzipain inhibitors.

The present invention relates to variants of the compounds described in WO-A-02057270 which are also inhibitors of a wide range of CA C1 cysteine protease. In particular, some compounds of the present invention are potent and selective inhibitors of cathepsin K.

Therefore, in the present invention, there is provided a compound of general formula (I)

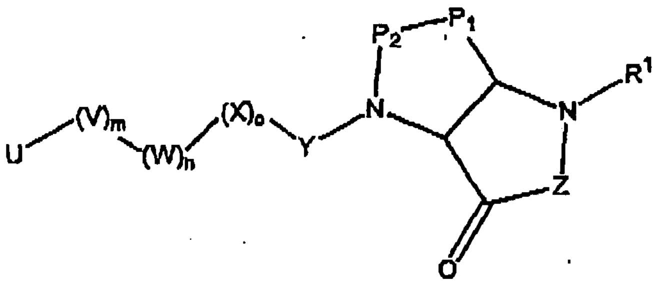

(I)

wherein:

Z = $CR^3R^4$, where $R^3$ and $R^4$ are independently chosen from $C_{0-7}$-alkyl (when C = 0, $R^3$ or $R^4$ is simply a hydrogen atom), $C_{3-6}$-cycloalkyl, $ArC_{0-7}$-alkyl (when C = 0, $R^3$ or $R^4$ is simply an aromatic moiety Ar),

$P_1$ = $CR^5R^6$, where $R^5$ and $R^6$ are independently chosen from $C_{0-7}$-alkyl, $C_{3-6}$-cycloalkyl, Ar-$C_{0-7}$-alkyl, O-$C_{0-7}$-alkyl, O-$C_{3-6}$-cycloalkyl, O-$ArC_{0-7}$-alkyl, S-$C_{0-7}$-alkyl, S-$C_{3-6}$-cycloalkyl, S-Ar-$C_{0-7}$-alkyl, NH-$C_{0-7}$-alkyl, NH-$C_{3-6}$-cycloalkyl, NH-Ar-$C_{0-7}$-alkyl, N($C_{0-7}$-alkyl)$_2$, N($C_{3-6}$-cycloalkyl)$_2$ and N(Ar-$C_{0-7}$-alkyl)$_2$;

$P_2$ = O, $CR^7R^8$ or $NR^9$, where $R^7$ and $R^8$ are independently chosen from $C_{0-7}$-alkyl, $C_{3-4}$-cycloalkyl, Ar-$C_{0-7}$-alkyl and $R^9$ is chosen from $C_{0-7}$-alkyl, $C_{3-6}$-cycloalkyl and Ar-$C_{0-7}$-alkyl;

Y = $CR^{10}R^{11}$-C(O) or $CR^{10}R^{11}$-C(S) or $CR^{10}R^{11}$-S(O) or $CR^{10}R^{11}$-$SO_2$ where $R^{10}$ and $R^{11}$ are independently chosen from $C_{0-7}$-alkyl, $C_{3-6}$-cycloalkyl and Ar-$C_{0-7}$-alkyl, or Y represents

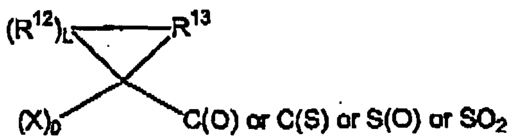

where L is a number from one to four and $R^{12}$ and $R^{13}$ are independently chosen from $CR^{14}R^{15}$ where $R^{14}$ and $R^{15}$ are independently chosen from $C_{0-7}$-alkyl, $C_{3-6}$-cycloalkyl, Ar-$C_{0-7}$-alkyl and halogen; and for each $R^{12}$ and $R^{13}$ either $R^{14}$ or $R^{15}$ (but not both $R^{14}$ and $R^{15}$) may additionally be chosen from O-$C_{0-7}$-alkyl, O-$C_{3-6}$-cycloalkyl, O-Ar-$C_{0-7}$-alkyl, S-$C_{0-7}$-alkyl, S-$C_{3-6}$-cycloalkyl, S-Ar-$C_{0-7}$-alkyl, NH-$C_{0-7}$-alkyl, NH-$C_{3-6}$-cycloalkyl, NH-Ar-$C_{0-7}$-alkyl, N-($C_{0-7}$-alkyl)$_2$, N-($C_{3-6}$-cycloalkyl)$_2$, and N-(Ar-$C_{0-7}$-alkyl)$_2$;

$(X)_o$ = $CR^{16}R^{17}$, where $R^{16}$ and $R^{17}$ are independently chosen from $C_{0-7}$-alkyl, $C_{3-6}$-cycloalkyl and Ar-$C_{0-7}$-alkyl and o is a number from zero to three;

$(W)_n$ = O, S, C(O), S(O) or S(O)$_2$ or $NR^{18}$, where $R^{18}$ is chosen from $C_{0-7}$-alkyl, $C_{3-6}$-cycloalkyl and Ar-$C_{0-7}$-alkyl and n is zero or one;

$(V)_m$ =C(O), C(S), S(O), S(O)$_2$, S(O)$_2$NH, OC(O), NHC(O), NHS(O), NHS(O)$_2$, OC(O)NH, C(O)NH or $CR^{19}R^{20}$, C=N-C(O)-$OR^{19}$ or C=N-C(O)-$NHR^{19}$, where $R^{19}$ and $R^{20}$ are independently chosen from $C_{0-7}$-alkyl, $C_{3-6}$-cycloalkyl, Ar-$C_{0-7}$-alkyl and m is a number from zero to free, provided that when m is greater than one, $(V)_m$ contains a maximum of one carbonyl or sulphonyl group;

U = a stable 5- to 7-membered monocyclic or a stable 8- to 11-membered bicyclic ring which is either saturated or unsaturated and which includes zero to four heteroatoms (as detailed below):

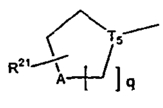 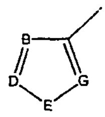 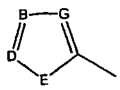 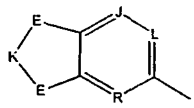 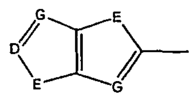

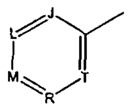 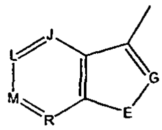 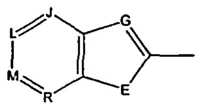 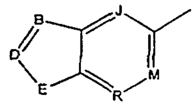 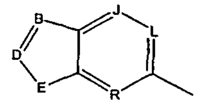

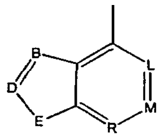 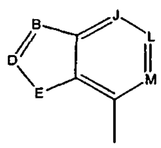 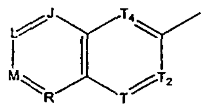 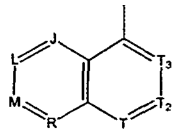 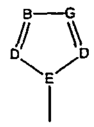

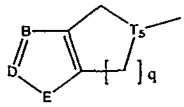 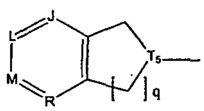 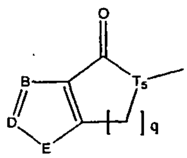 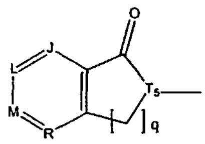

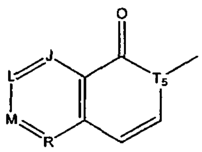 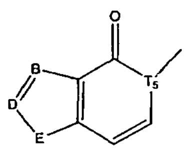 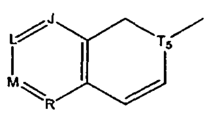 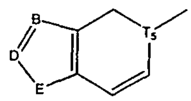

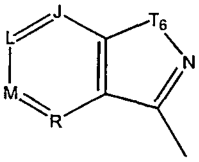 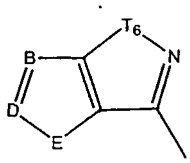 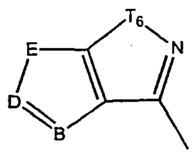

wherein $R^{21}$ is:

$C_{0-7}$-alkyl, $C_{3-6}$-cycloalhyl, Ar-$C_{0-7}$-alkyl, O-$C_{0-7}$-alkyl, O-$C_{3-6}$-cycloalkyl, O-Ar-$C_{0-7}$-alkyl, S-$C_{0-7}$-alkyl, S-$C_{3-6}$-cycloalkyl, S-Ar-$C_{0-7}$-alkyl, $SO_2$-$C_{0-7}$-alkyl, $SO_2$-$C_{3-6}$-cycloalkyl, $SO_2$-Ar-$C_{0-7}$-alkyl, NH-$C_{0-7}$-alkyl, NH-$C_{3-6}$-cycloalkyl, NH-Ar-$C_{0-7}$-alkyl, N($C_{0-7}$-alkyl)$_2$, N($C_{3-6}$-cycloalkyl)$_2$ or N(Ar-$C_{0-7}$-alkyl)$_2$; or, when part of a $CHR^{21}$ or $CR^{21}$ group, $R^{21}$ may be halogen;

A is chosen from:

$CH_2$, $CHR^{21}$, O, S, $SO_2$, $NR^{22}$ or N-oxide (N$\rightarrow$O), where $R^{21}$ is as defined above; and $R^{22}$ is chosen from $C_{0-7}$-alkyl, $C_{3-6}$-cycloalkyl and Ar-$C_{0-7}$-alkyl;

B, D and G are independently chosen from:

$CR^{21}$, where $R^{21}$ is as defined above, or N or N-oxide (N$\rightarrow$O);

E is chosen from:
$CH_2$, $CHR^{21}$, O, S, $SO_2$, $NR^{22}$ or N-oxide (N→O), where $R^{21}$ and $R^{22}$ are defined as above;
K is chosen from:
$CH_2$, $CHR^{22}$, where $R^{22}$ is defined as above;
J, L, M, R, T, $T_2$, $T_3$ and $T_4$ are independently chosen from:
$CR^{21}$ where $R^{21}$ is as defined above, or N or N-oxide (N→O);
$T_5$ is chosen from:
CH or N;
$T_6$ is chosen from:
$NR^{22}$, $SO_2$, OC(O), C(O), $NR^{22}C(O)$;
q is a number from one to three, thereby defining a 5-, 6- or 7-membered ring;
$R^1 = R^2C(O)$, $R^2OC(O)$, $R^2NQC(O)$, $R^2SO_2$, where $R^2$ is chosen from $C_{1-7}$-alkyl, $C_{3-6}$-cycloalkyl or Ar-$C_{0-7}$-alkyl (when C = 0, $R^2$ is simply an aromatic moiety Ar) and Q is $C_{0-7}$-alkyl.

The present invention includes all salts, hydrates, solvates, complexes and prodrugs of the compounds of this invention. The term "compound" is intended to include all such salts, hydrates, solvates, complexes and prodrugs, unless the context requires otherwise.

Appropriate pharmaceutically and veterinarily acceptable salts of the compounds of general formula (I) include salts of organic acids, especially carboxylic acids, including but not limited to acetate, trifluoroacetate, lactate, gluconate, citrate, tartrate, maleate, malate, pantothenate, adipate, alginate, aspartate, benzoate, butyrate, digluconate, cyclopentanate, glucoheptanate, glycerophosphate, oxalate, heptanoate, hexanoate, fumarate, nicotinate, palmoate, pectinate, 3-phenylpropionate, picrate, pivalate, proprionate, tartrate, lactobionate, pivolate, camphorate, undecanoate and succiuate, organic sulphonic acids such as methanesulphonate, ethanesulphonate, 2-hydroxyethane sulphonate, camphorsulphonate, 2-naphthalenesulphonate, benzenesulphonate, p-chlorobenzenesulphonate and p-toluenesulphonate; and inorganic acids such as hydrochloride, hydrobromide, hydroiodide, sulphate, bisulphate, hemisulphate, thiocyanate, persulphate, phosphoric and sulphonic acids. Salts which are not pharmaceutically or veterinarily acceptable may still be valuable as intermediates.

Prodrugs are any covalently bonded compounds which release the active parent drug according to general formula (I) *in vivo*. A prodrug may for example constitute a ketal or hemiketal derivative of the exocyclic ketone functionality present in the hexahydropyrrolo[3,2-*b*]pyrrol-3-one, hexahydropyrrolo[3,2-*c*]pyrazol-6-one or hexahydro-2-oxa-1,4-diazapentalen-6-one scaffold. If a chiral centre or another form of isomeric centre is present in a compound of the present invention, all forms of such isomer or isomers, including enantiomers and diastereoisomers, are intended to be covered herein. Compounds of the invention containing a chiral centre may be used as a racemic mixture, an enantiomerically enriched mixture, or the racemic mixture may be separated using well-known techniques and an individual enantiomer may be used alone.

'Halogen' as applied herein is meant to include F, Cl, Br, I;

'Heteroatom' as applied herein is meant to include O, S and N;

'$C_{0-7}$-alkyl' as applied herein is meant to include stable straight and branched chain aliphatic carbon chains containing zero (*i.e.* simply hydrogen) to seven carbon atoms such as methyl, ethyl, n-propyl, isopropyl, n-butyl, isobutyl, t-butyl, pentyl, isopentyl, hexyl, heptyl and any simple isomers thereof Additionally, where '$C_{0-7}$-alkyl' contains 2 or more contiguous carbon atoms, an alkene (-CH=CH-) may be present Additionally, any $C_{0-7}$-alkyl may optionally be substituted at any point by one, two or three halogen atoms (as defined above) for example to give a trifluoromethyl substituent. Furthermore, $C_{0-7}$-alkyl may contain one or more heteroatoms (as defined above) for example to give ethers, thioethers, sulphones, sulphonamides, substituted amines, amidines, guanidines, carboxylic acids, carboxamides. If the heteroatom is located at a chain terminus then it is appropriately substituted with one or two hydrogen atoms. A heteroatom or halogen is only present when $C_{0-7}$-alkyl contains a minimum of one carbon atom. For example, the group $CH_3$-$CH_2$-O-$CH_2$-$CH_2$- is defined within '$C_{0-7}$-alkyl' as a $C_4$ alkyl that contains a centrally positioned heteroatom whereas the group $CH_3$-$CH_2$-$CH_2$-$CH_2$- is defined within '$C_{0-7}$-alkyl' as an unsubstituted $C_4$ alkyl.

'$C_{3-6}$-cycloalkyl' as applied herein is meant to include any variation of '$C_{0-7}$-alkyl' which additionally contains a carbocyclic ring such as cyclopropyl, cyclobutyl, cyclopentyl, cyclohexyl. The carbocyclic ring may optionally be substituted at any position with one or more halogens (as defined above) or heteroatoms (as defined above) for example to give a tetrahydrofuran, pyrrolidine, piperidine, piperazine or morpholine substituent.

'Ar-$C_{0-7}$-alkyl' as applied herein is meant to include any variation of $C_{0-7}$-alkyl which additionally contains an aromatic ring moiety 'Ar'. The aromatic ring moiety Ar can be a stable 5 or 6-membered monocyclic or a stable 8 to 10 membered bicyclic ring which is unsaturated, as defined previously for U in general formula (I). The aromatic ring moiety Ar may be substituted by $R^{21}$ (as defined above for U in general formula (I)). When C = 0 in the substituent Ar-$C_{0-7}$-alkyl, the substituent is simply the aromatic ring moiety Ar.

Other expressions containing terms such as alkyl and cycloalkyl are intended to be construed according to the definitions above. For example "$C_{1-4}$ alkyl" is the same as $C_{0-7}$-alkyl except that it contains from one to four carbon atoms.

If different structural isomers are present, and/or one or more chiral centres are present, all isomeric forms are intended to be covered. Enantiomers are characterised by the absolute configuration of their chiral centres and described by the R- and *S*-sequencing rules of Cahn, Ingold and Prelog. Such conventions are well known in the art (e.g. see 'Advanced Organic Chemistry', 3rd edition, ed. March, J., John Wiley and Sons, New York, 1985). It is also intended to include compounds of general formula (I) where any hydrogen atom has been replaced by a deuterium atom.

Compounds of general formula I are inhibitors of a wide range of CA C1 cysteinyl proteases for example cathepsin K, cathepsin S, cathepsin L, cathepsin F, cathepsin B, cruzipains, falcipains and *leismania mexicana* CPB protease.

For all the above mentioned CA C1 proteases, the preferred fundamental backbone shape of inhibitor molecules is broadly similar. Therefore, the preferred compounds of general formula (I) will have similar $(V)_m$, $(W)_n$, $(X)_o$ and $R^1$ whether they act as cathepsin K cathepsin S, cathepsin L, cathepsin F, cathepsin B, cruzipains, falcipains or *leismania mexicana* CPB protease inhibitors. Within general formula (I), inhibitory potency and selectivity for each CA C1 protease is primarily determined by different preferences for the Y and U groups for each CA C1 proteases.

Preferred compounds of general formula (I) include, but are not limited to those which, independently or in any combination:

Z is $CH_2$;
$P_1$ is $CH_2$;
$P_2$ is $CH_2$, O or NH
$R^2$ is Ar-$C_{0-2}$-alkyl
or $R^2$ is $C_{3-7}$-alkyl which may include an -O- or -NH- as part of the chain and which is either unsubstituted or is substituted with one or more $NH_2$, NHMe, NHC(O)$CH_3$, NMeC(O)$CH_3$, OH or OMe groups
or $R^2$ is a $C_{3-6}$-cycloalkyl group, wherein the ring system is either connected directly to the remainder of the $R^1$ moiety or there is one intervening methylene group;
in the group $(X)_o$, each of $R^{16}$ and $R^{17}$ is selected from $C_{0-7}$-alkyl or Ar-$C_{0-7}$-alkyl, for example hydrogen, a straight or branched alkyl chain, a straight or branched heteroalkyl chain, an optionally substituted arylalkyl chain or an optionally substituted arylheteroalkyl chain;
in the group $(X)_o$, $R^{16}$ and $R^{17}$ are hydrogen and o is zero or one;
in the group $(V)_m$, V is chosen from C(O), OC(O), NHC(O), C(O)NH, $CHR^{20}$, C=N-C(O)-$OR^{19}$ or C=N-C(O)-$NHR^{19}$

where $R^{19}$ is chosen from $C_{0-7}$-alkyl, $C_{3-6}$-cycloalkyl, Ar-$C_{0-7}$-alkyl and $R^{20}$ is $C_{0-4}$-alkyl, and

As mentioned above, cysteine protease inhibitors of general formula (I), comprise an $R^2$ group chosen from $C_{1-7}$-alkyl, $C_{3-6}$-cycloalkyl and Ar-$C_{0-7}$-alkyl.

When $R^2$ composes Ar-$C_{0-7}$-alkyl, preferred $R^2$ groups comprises Ar-$C_{0-2}$-alkyl and examples include but are not limited to:

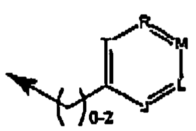
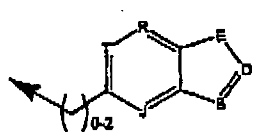
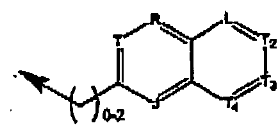

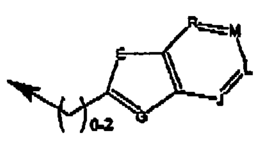
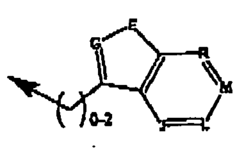
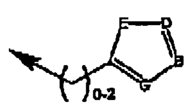
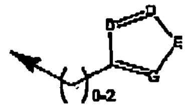

where J, L, M, R, T, $T_2$, $T_3$ and $T_4$, B, D, G and E are as previously defined.

More preferred $R^2$ comprises Ar-$C_{0-1}$-alkyl and examples of such $R^2$ groups include, but are not limited to:

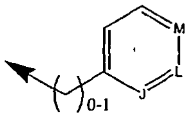 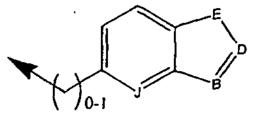 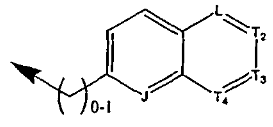

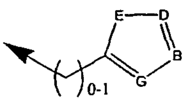 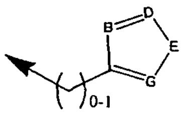 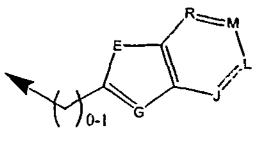 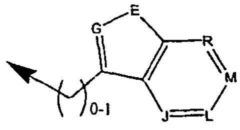

where J, L, M, $T_2$, $T_3$, $T_4$, B, D, G and E are as previously defined.

Still more active compounds of general formula (I) are those in which $R^2$ comprises a monocyclic Ar-$C_{0-1}$-alkyl and forms part of an $R^1$ group such as:

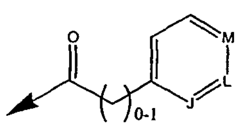 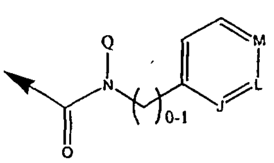 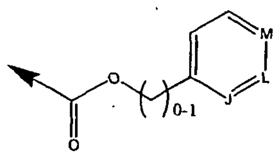

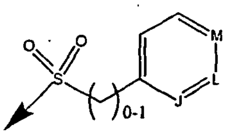 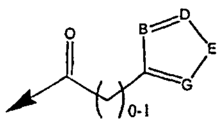 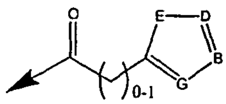

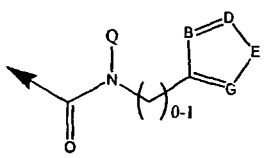 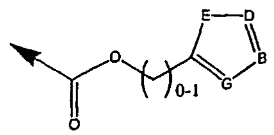

wherein:

J, L, M, B, D and G are as defined above (i.e. $CR^{21}$, N or N→O) and wherein $R^{21}$ is chosen from hydrogen, methyl, methoxy, ethyl, isopropyl, trifluoromethyl, trifluoromethoxy, F, Cl, $SO_2Me$; and

E is as previously defined; and

Q is chosen from hydrogen or methyl.

In cysteine protease inhibitors of general formula (I) when $R^2$ is $C_{1-7}$-alkyl, preferred $R^2$ groups comprise $C_{3-7}$-alkyl which may include an -O- or -NH- as part of the chain and which is either unsubstituted or is substituted with one or more $NH_2$, NHMe, $NHC(O)CH_3$, $NMeC(O)CH_3$, OH or OMe groups.

When $R^2$ is $C_{3-7}$-alkyl, more preferred groups include $C_{3-6}$-alkyl, in particular those which are branched at the α-position or which include an $NH_2$, NHMe, $NHC(O)CH_3$, $NMeC(O)CH_3$, OH or OMe substituent at the α-position.

In cysteine protease inhibitors of general formula (I) when $R^2$ is $C_{3-6}$-cycloalkyl, $R^2$ may include a heteroatom in the ring system. Examples of $R^2$ groups include cyclopropyl, cyclobutyl, cyclopentyl, cyclohexyl, pyrrolidine, piperidine, morpholine, tetrahydrofuran, cyclopentene, cyclopentadiene, cyclohexadiene and piperazine. Nitrogen-containing rings may be N-substituted with groups such as $C_{1-4}$ alkyl, phenyl or benzyl.

It is yet more preferred that when $R^2$ is a $C_{3-6}$-cycloalkyl group, the ring system is either connected directly to the remainder of the $R^1$ moiety or there is one intervening methylene group. The inventors have found that the activity

of the molecule increases with the size of the cycloalkyl ring and therefore compounds in which $R^2$ is a five- or six-membered cyclic ring are most favourable.

In compounds of general formula (I), particularly preferred $R^1$ groups therefore include:
benzoyl; pyridine-2-carbonyl; 1-oxy-pyridine-2-carbonyl; pyridine-3-carbonyl; 1-oxy-pyridine-3-carbonyl; pyridine-4-carbonyl; 1-oxy-pyridine-4-carbonyl; phenyl sulphonyl; pyridine-2-sulphonyl; 1-oxy-pyridine-2-sulphonyl; pyridine-3-sulphonyl; 1-oxy-pyridine-3-sulphonyl; pyridine-4-sulphonyl; 1-oxy-pyridine-4-sulphonyl; phenylacetyl; phenylcarbamoyl; isobutylcarbamoyl; phenyloxycarbonyl; isobutyloxycarbonyl; pyrrolidine-N-carbonyl; piperidine-N-carbonyl; morpholine-N-carbonyl; piperazine-N-carbonyl; 4-methyl-piperazine-N-carbonyl; (4-methyl-piperazin-1-yl)-acetoyl; piperazin-1-yl-acetoyl; furan-2-carbonyl; 5-chlorofuran-2-carbonyl; thiophene-2-carbonyl; 5-chlorothiophene-2-carbonyl; furan-3-carbonyl; thiophene-3-carbonyl; cyclopentoyl; cyclohexoyl; cyclopent-3-enoyl; cyclopentylmethylcarbonyl; cyclohexylmethylcarbonyl; pyrrolidine-2-carbonyl; N-acetyl-pyrrolidine-2-carbonyl; piperidine-2-carbonyl; N-acetyl-piperidine-2-carbonyl; tetrahydrofuran-2-carbonyl; 1-aminocyclobutanoyl; 1-aminocyclopentanoyl; 1-aminocyclohexanoyl; N-acetyl-1-aminocyclobutanoyl; N-acetyl-1-aminocyclopentanoyl; N-acetyl-1-aminocyclohexanoyl; 1-hydroxycyclobutanoyl; 1-hydroxycyclopentanoyl; 1-hydroxycyclohexanoyl; 1-methoxycyclobutanoyl; 1-methoxycyclopentanoyl; 1-methoxycyclohexanoyl; aminocyclopentylacetoyl; aminocyclohexylacetoyl; N-acetylaminocyclopentylacetoyl; N-acetylaminocyclohexylacetoyl; 2-acetylaminopropionoyl; 2-acetylaminoethanoyl; 2-acetyl-N-methylaminoethanoyl; N,N-dimethylaminoacetoyl; 2-aminobutanoyl; N-acetyl-2-aminobutanoyl; 2-amino-3-methylbutanoyl; N-acetyl-2-amino-3-methylbutanoyl; 2-amino-3,3-dimethylbutanoyl; N-acetyl-2-amino-3,3-dimethylbutanoyl; 2-amino-3-methylpentanoyl; N-acetyl-2-amino-3-methylpentanoyl; pentanoyl; 3-methylpentanoyl; 4-methylpentanoyl; 2-amino-4-methylpentanoyl; N-acetyl-2-amino-4-methylpentanoyl; 2-amino-4,4-dimethylpentanoyl; N-acetyl-2-amino-4,4-dimethylpentanoyl; 2-aminopentanoyl; N-acetyl-2-aminopentanoyl; 2-amino-5-methylhexanoyl; N-acetyl-2-amino-5-methylhexanoyl; 2-hydroxy-3-methylbutanoyl; 2-methoxy-3-methylbutanoyl; 2-hydroxy-3,3-dimethylbutanoyl; 2-methoxy-3,3-dimethylbutanoyl; 2-hydroxy-3-methylpentanoyl; 2-methoxy-3-methylpentanoyl; 2-hydroxy-4-methylpentanoyl; 2-methoxy-4-methylpentanoyl; 2-hydroxy-4,4-dimethylpentanoyl; 2-methoxy-4,4-dimethylpentanoyl; 2-hydroxypentanoyl; 2-methoxypentanoyl; 2-hydroxy-5-methylhexanoyl; 2-methoxy-5-methylhexanoyl.

In cysteine protease inhibitors of general formula (I), it is preferred that in the group $(X)_o$, each of $R^{16}$ and $R^{17}$ is selected from $C_{0-7}$-alkyl or Ar-$C_{0-7}$-alkyl, for example hydrogen, a straight or branched alkyl chain, a straight or branched heteroalkyl chain, an optionally substituted arylalkyl chain or an optionally substituted arylheteroalkyl chain.

More preferred $(X)_o$ groups comprise $R^{16}$ chosen from hydrogen; $R^{17}$ chosen from hydrogen or $C_{1-4}$-alkyl, which may be substituted with OH, $NR^{22}R^{22}$, $COOR^{22}$, or $CONR^{22}$; or Ar-$C_{1-4}$-alkyl, where the aryl group may be substituted with $R^{21}$, wherein each $R^{21}$ and $R^{22}$ is independently as defined previously.

Yet more preferred $(X)_o$ groups are those in which $R^{16}$ is from hydrogen and $R^{17}$ is chosen from hydrogen or simple $C_{1-4}$-alkyl groups such as methyl, ethyl, propyl, butyl.

In the most preferred $(X)_o$ groups, $R^{16}$ aud $R^{17}$ are hydrogen and o is zero or one.

Preferred compounds of general formula (I) are those in which, in the group $(W)_n$, W is chosen from O, S, $SO_2$, S(O), C(O) or $NR^{18}$, Where $R^{18}$ is chosen from $C_{0-7}$-alkyl; and n is zero or one.

In more preferred $(W)_n$ groups, W comprises O, S, $SO_2$, C(O) or NH where n is zero or one.

Still more active compounds are those in which W is C(O) or NH where n is zero or one.

It is most preferred that in the group $(W)_n$, W is NH and n is zero or one

In protease inhibitors of general formula (I), more active compounds are those in which, in the group $(V)_m$, V is chosen from C(O), OC(O), NHC(O), C(O)NK, $CHR^{20}$; C=N-C(O)-$OR^{19}$ or C=N-C(O)-$NHR^{19}$
where $R^{19}$ is chosen from $C_{0-7}$-akyl, $C_{3-6}$-cycloalkyl, Ar-$C_{0-7}$-alkyl and $R^{20}$ is $C_{0-4}$-alkyl, and

m is zero or one.

Preferably, the U-$(V)_m$-$(W)_n$-$(X)_o$-Y or U-$(V)_m$-$(W)_n$-$(X)_o$ substituent combination is selected from the following:

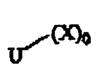
'n' = 0
'm' = 0

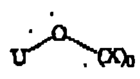
'n' = 1
'W' = O
'm' = 0

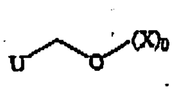
'n' = 1
'W' = O
'V' = $CH_2$
'm' = 1

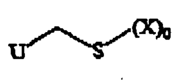
'n' = 1
'W' = S
'V' = $CH_2$
'm' = 1

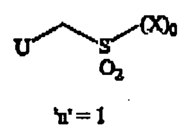
'n' = 1
'W' = $SO_2$
'V' = $CH_2$
'm' = 1

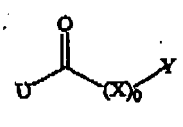
'n' = 1
'W' = C(O)
'm' = 0

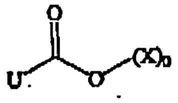

'n' = 1
'W' = O
'V' = C(O)
'm' = 1

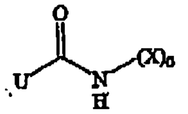

'n' = 1
'W' = $NR^{18}$, $R^{18}$ = 'H'
'V' = C(O)
'm' = 1

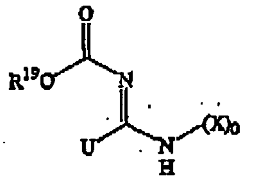

'n' = 1
'W' = $NR^{18}$, $R^{18}$ = 'H'
'V' = C=N-C(O)-$OR^{19}$
'm' = 1

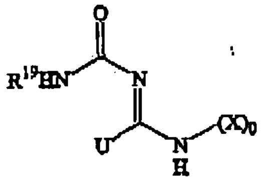

'n' = 1
'W' = $NR^{18}$, $R^{18}$ = 'H'
'V' = C=N-C(O)-$NHR^{19}$
'm' = 1

Preferably, the U-$(V)_m$-$(W)_n$-$N_o$-Y substituent combination is selected from:

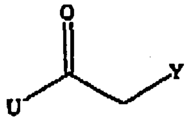

$(X)_0$ = '$CH_2$'
'n' = 1
'W' = C(O)
'm' = 0

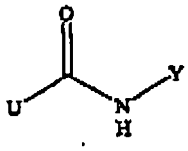

$(X)_0$ = '-'
'n' = 1
'W' = $NR^{18}$, $R^{18}$ = 'H'
'V' = C(O)
'm' = 1

As mentioned above, the substituents Y and U are important in determining the inhibitory potency and selectivity for various proteases and the preferred Y and U substituents vary depending on the target protease.

In Compounds of general formula **(I)** that are inhibitors of cathepsin K, it is preferred that the Y substituent is $CHR^{11}$ CO where $R^{11}$ is selected from $C_{0-7}$-alkyl, Ar-$C_{0-7}$-akyl and $C_{3-6}$-cycloalkyl. Examples of suitable $R^{11}$ groups include, for example, hydrogen, a straight or branched alkyl chain, a straight or branched heteroalkyl chain, an optionally substituted arylalkyl chain or an optionally substituted arylheteroalkyl chain, cyclohexylmethyl or cyclopentylmethyl. Additionally, preferred compounds of general formula **(I)** are those in which Y comprises a group:

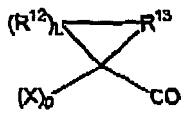

where $R^{12}$ and $R^{13}$ are each $CR^{14}R^{15}$ and each $R^{14}$ and $R^{15}$ is, independently, selected from $C_{0-7}$-alkyl and Ar-$C_{0-7}$-alkyl, for example hydrogen, a straight or branched alkyl chain, a straight or branched heteroalkyl chain, an optionally substituted arylalkyl chain or an optionally substituted arylheteroalkyl chain and L is a number from one to four; and
the group U comprises an optionally substituted 5- or 6-membered saturated or saturated heterocycle or Ar group or an optionally substituted saturated or unsaturated 8 to 10-membered heterocycle or Ar group.

Examples of preferred $(X)_o$-Y substituents in compounds of general formula (I) which are inhibitors of cathepsin K include, but are not limited to:

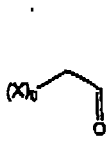 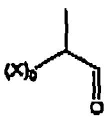 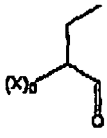 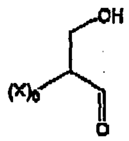 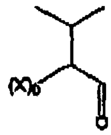 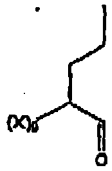 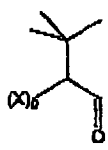

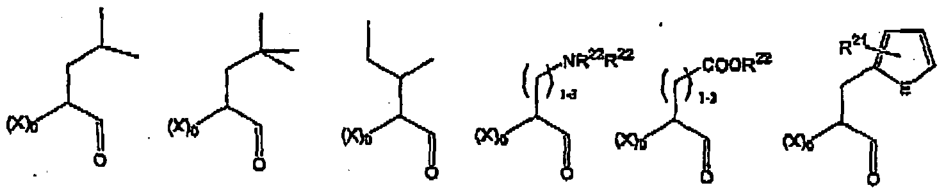

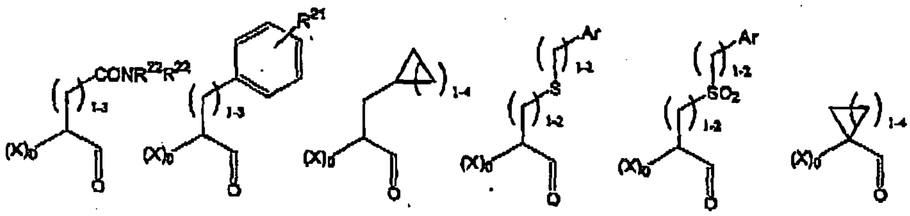

wherein E, $R^{21}$, $R^{22}$ and Ar are as defined previously; any of which may be substituted with one or more halogen, preferably fluoro, substituents.

In compounds that are inhibitors of Cathepsin K, more preferred $R^{11}$ groups include $C_{1-4}$-alkyl, which may be substituted with cycloalkylmethyl or halogen, or $R^{11}$ is chosen from cycloalkyl-1-carbonyl or $R^{11}$ is chosen from Ar-$C_{1-4}$-alkyl, where the aryl group may be substituted with $R^{21}$; where $R^{21}$ is defined above.

Increased inhibition of cathepsin K can be achieved in compounds in which the $R^{11}$ groups are simple branched alkyl groups such as isobutyl or straight alkyl chains such as n-propyl, optionally substituted with one or more halogen (preferably fluoro) substitutents. Yet more preferred $R^{11}$ groups comprise $ArCH_2$-where the aromatic ring is an optionally substituted monocyclic heterocycle and still, more preferred $R^{11}$ groups comprise cyclopropylmethyl and cyclohexyl-1-carbonyl. In compounds which are particularly active inhibitors of cathepsin K, $(X)_o$-y substituents include, but are not limited to:

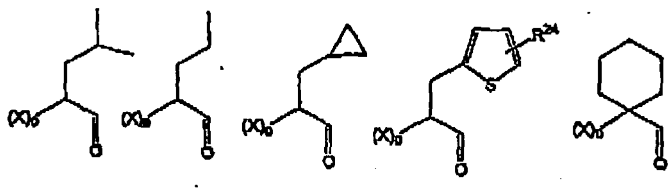

wherein $R^{24}$ is chosen from hydrogen, methyl, methoxy, ethyl, isopropyl, F, Cl and wherein any of the alkyl groups may be substituted with one or more F or Cl.

In order to maximise the inhibition of cathepsin K, the compound of formula (I) may comprise $R^{11}$ groups which are simple branched alkyl groups such as isobutyl or n-propyl or halogen substituted variants thereof such as 3,3,3-trifluoro-2-trifluoromethylpropyl.

In compounds of general formula (I) that are inhibitors of cathepsin K, it is preferred that the group U comprises an optionally substituted 5- or 6-membered saturated or unsaturated heterocycle or Ar group or an optionally substituted saturated or unsaturated 8 to 10-membered heterocycle or Ar group. Examples of such preferred U rings include, but are not limited to the following:

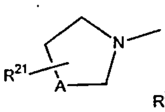 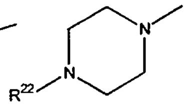 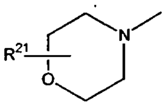 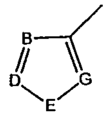 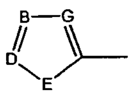 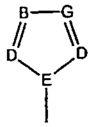

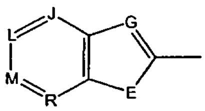 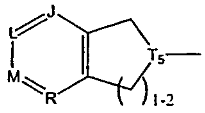 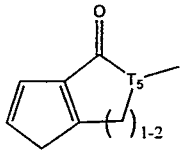 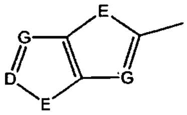 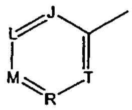

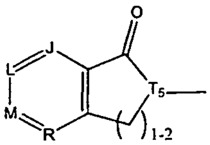 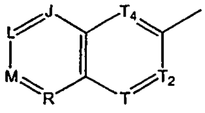 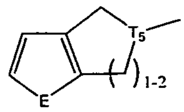 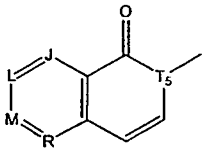

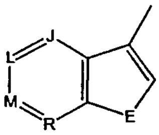 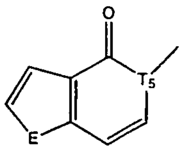 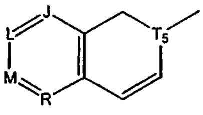 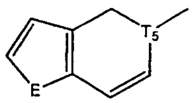

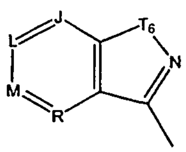 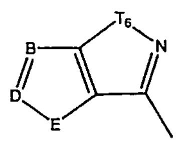 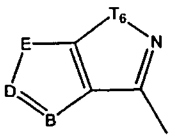

wherein $R^{21}$, $R^{22}$, A, B, D, E, G, J, L, M, R, T, $T_2$, $T_4$, $T_5$ and $T_6$ are as defined previously.

Stronger inhibition of cathepsin K can be achieved in compounds where the U groups comprise a bulky alkyl or aryl group at the para position of an aryl; a meta or para 5,6-biaryl Ar-Ar, where Ar is as previously defined; a 6,6 or 6,5 or 5,5-fused aromatic ring, where Ar is as previously defined, or a 4-substituted piperazine. Examples of more preferred U groups include but are not limited to:

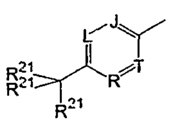 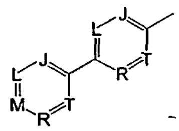 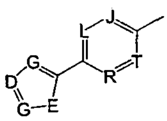 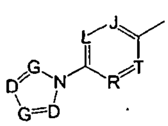 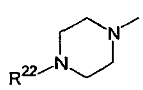

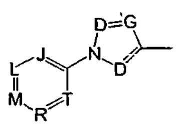 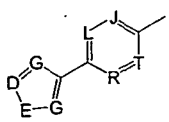 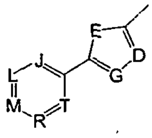 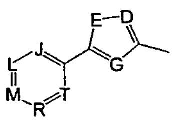 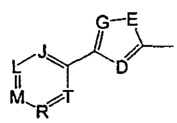

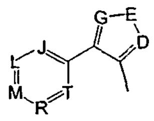 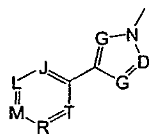 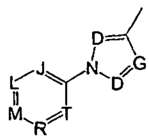 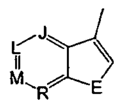 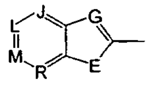

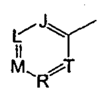 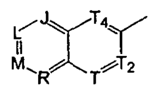 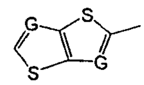

wherein $R^{21}$, $R^{22}$, D, E, G, J, L, M, R, T, $T_2$ and $T_4$ are as defined previously.

In compounds that are inhibitors of cathepsin K, even more preferred U groups comprise a 6-membered aromatic ring Ar containing a bulky alkyl or aryl group at the para position; a meta or para-biaryl Ar-Ar, where Ar is as previously defined; a 6,6 or 6,5 or 5,5-fused aromatic ring, where Ar is as previously defined; or a 4-substituted piperazine where $R^{25}$ is chosen from hydrogen, $C_{1-2}$-alkyl or Ar-$C_{0-2}$-alkyl. Examples of even more preferred U groups include but are not limited to:

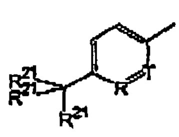 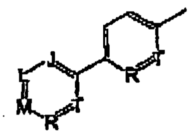 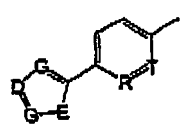 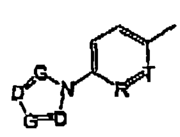 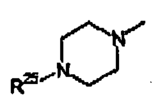

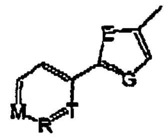 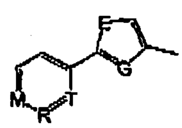 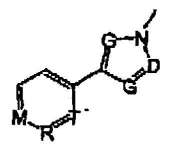 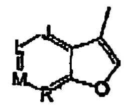 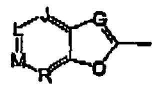

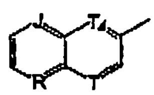 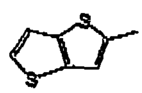 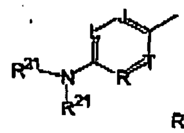 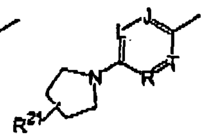 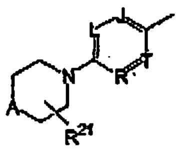

wherein $R^{21}$, $R^{25}$, D, E, G, J, L, M, R, T and $T_4$ are as defined previously.

In order to maximise inhibition of cathepsin K, compounds of general formula (I) may be selected to haveU groups chosen from the following:

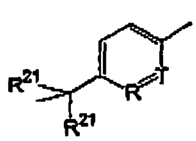 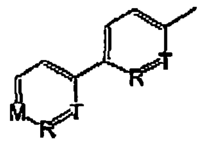 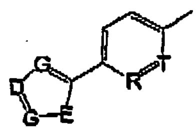 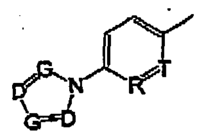

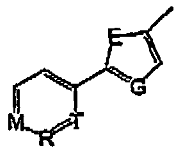 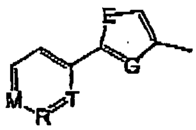 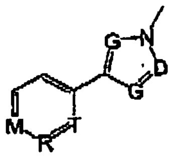

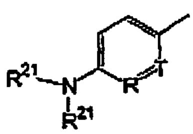 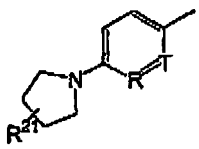 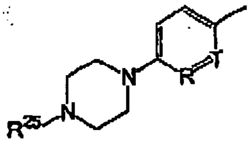

wherein $R^{21}$, $R^{25}$, D, E, G, M, R and T are as defined previously.

In order to achieve the greatest inhibitory effect against cathepsin S, it is preferred that the $(X)_o$-Y substituent is chosen from the following:

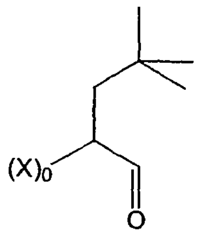 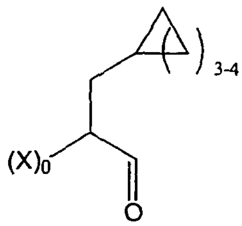 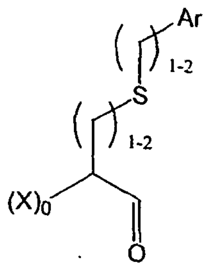 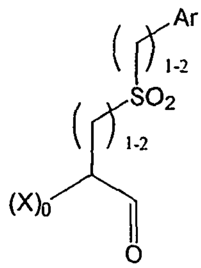

wherein $(X)_o$ and Ar are as previously defined.

In order to achieve the greatest inhibitory effect against cathepsin S, it is preferred that the group U comprises an optionally substituted 5-membered unsaturated heterocycle or a 6,5- 5,5- or 5,6-fused aromatic ring, where Ar is as previously defined or a morpholine. Examples of such preferred U rings include, but are not limited to the following:

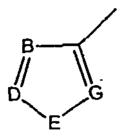 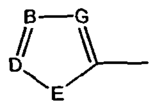 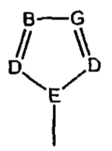 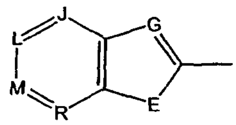 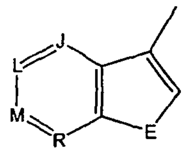

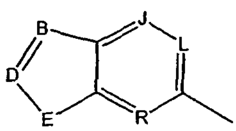 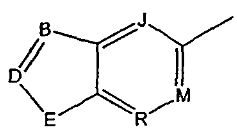 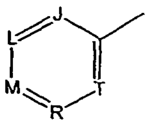 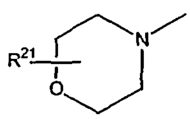

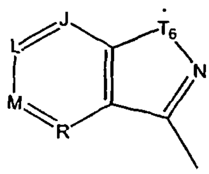
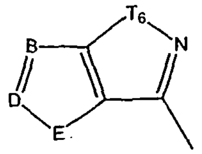
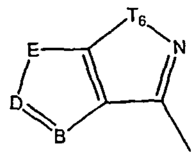

wherein $R^{21}$, B, D, E, G, J, L, M, R and $T_6$ are as defined previously.

In order to achieve the greatest inhibitory effect against cathepsin S whilst retaining selectivity against other CA C1 cysteinyl proteases, it is more preferred that the group U comprises an optionally substituted 5-membered unsaturated heterocycle or a 6,5- or 5,5-fused aromatic ring, where Ar is as previously defined. Examples of more preferred U rings include, but are not limited to the following:

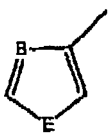
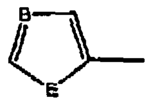
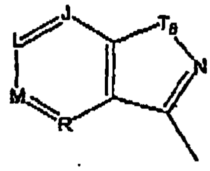
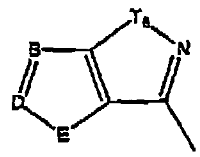
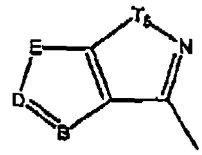

wherein B, D, E,J, L, M, R and $T_6$ are as defined previously.

In order to achieve the greatest inhibitory effect against cathepsin L, it is preferred that the $(X)_o$-Y substituent is chosen as an aromatic group as follows:

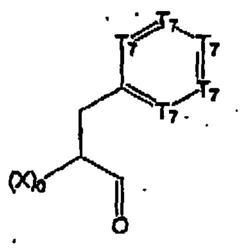

wherein $T_7$ is chosen from CH, N or $CR^{21}$ where $R^{21}$ is as defined previously.

In particular, for cathepsin L inhibition it is more preferred that within the $T_7$ substituent that the $R^{21}$ substituent is chosen from single and multiple ring substitution combinations of Me, F, C1, OH and OMe.

In order to achieve the greatest inhibitory effect against cathepsin L, it is preferred that the group U comprises an optionally substituted 5-membered unsaturated heterocycle or a 6,6- or 6,5- or 5,6-fused aromatic ring, where Ar is as previously defined or a meta-substituted Ar. Examples of such preferred U rings include, but are not limited to the following:

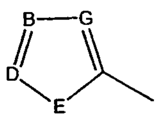
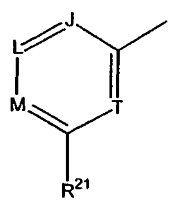
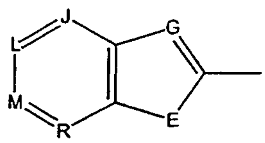

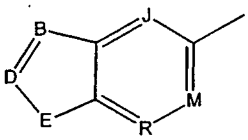 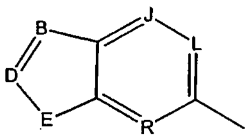 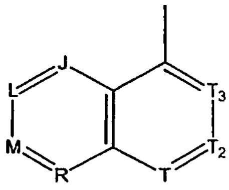

wherein $R^{21}$, B, D, E, G, J, L, M, R, T, $T_2$ and $T_3$ are as defined previously.

In order to achieve the greatest inhibitory effect against cathepsin L whilst retaining selectivity against other CA C1 cysteinyl proteases, it is more preferred that the group U comprises a substituted 5-membered unsaturated heterocycle or a 6,6-fused aromatic ring, where Ar is as previously defined or a meta-substituted Ar. Examples of such preferred U rings include, but are not limited to the following:

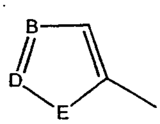 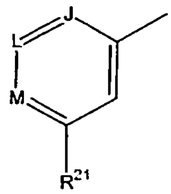 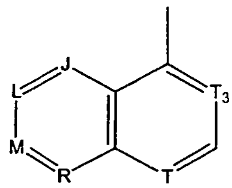

wherein E is chosen from oxygen or N-ethyl, D is chosen from nitrogen or $CCH_3$, B is chosen from nitrogen or $CCH_3$, $R^{21}$ is chosen from halogen, OMe, $CF_3$, $OCF_3$, $CH_2NH_2$ and J, L, M, R, T and $T_3$ are as previously defined.

The inventors have observed that for the cruzipains and *leismania mexicana* CPB protease, that the U and Y substituent preferences are composed of a mixture of those described earlier for cathepsin K and cathepsin L. In essence, many of the preferred cathepsin K and cathepsin L inhibitors also show potency against the cruzipains and *leismania mexicana* CPB protease as highlighted in the EXAMPLES section. Such promiscurity can be used to provide potent and selective inhibitors of the cruzipains and *leismania mexicana* CPB protease by combining a preferred U substituent as described for cathepsin K with a preferred Y substituent as described for cathepsin L or by combining a preferred U substituent as described for cathepsin L with a preferred Y substituent as described for cathepsin K. Such preferred combinations provide potent inhibitors of the cruzipains and *leismania mexicana* CPB protease with selectivity against either or both cathepsin K and cathepsin L.

Particular compounds of the invention are selected from the compounds formed by joining one of the '$U-(V)_m$' fragments herein defmed as the 'Capping group (Cgl to Cg103)' of general formula **(I)** shown in Table 1, with one of the '$(W)_n-(X)_o-Y$' fragments herein defined as the 'P2 pocket group (Pgl to Pg39)' of general formula **(I)** shown in Table 2, with a 5,5-bicyclic scaffold containing one of the $R^1$ fragments herein defined as the 'Prime-side binding group (Ps1 to Ps243)' of general formula **(I)** shown in Table 3.

Table (1) 'Capping group Cg' Fragments

* signifies the point of attachment of 'Cg groups' to 'Pg groups'.

| | U-(V)$_m$ | | U-(V)$_m$ | | U-(V)$_m$ |
|---|---|---|---|---|---|
| Cg1 | 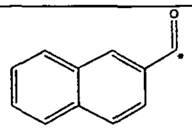 | Cg2 | 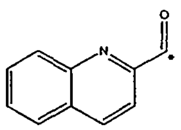 | Cg3 | 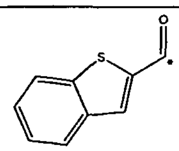 |
| Cg4 | 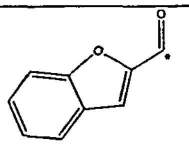 | Cg5 | 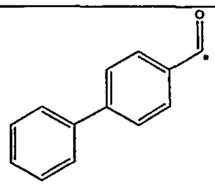 | Cg6 | 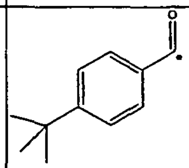 |

| | | | | | |
|---|---|---|---|---|---|
| Cg7 | 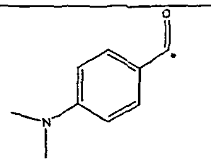 | Cg8 | 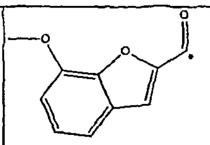 | Cg9 | 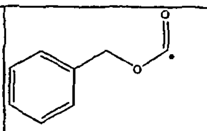 |
| Cg10 | 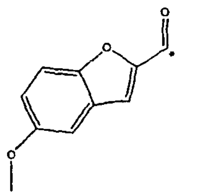 | Cg11 | 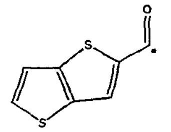 | Cg12 | 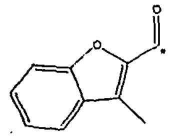 |
| Cg13 | 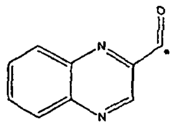 | Cg14 | 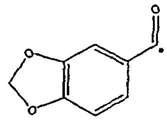 | Cg15 | 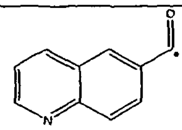 |
| Cg16 | 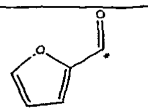 | Cg17 | 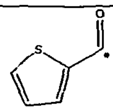 | Cg18 | 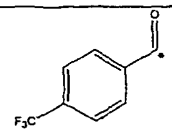 |
| Cg19 | 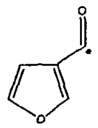 | Cg20 | 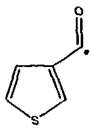 | Cg21 | 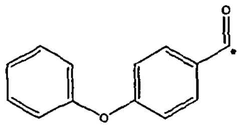 |
| Cg22 | 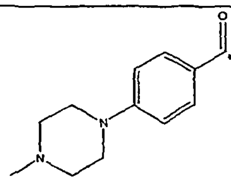 | Cg23 | 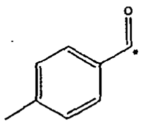 | Cg24 | 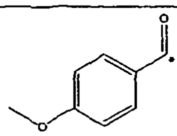 |
| Cg25 | 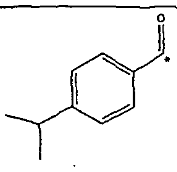 | Cg26 | 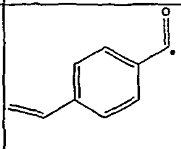 | Cg27 | 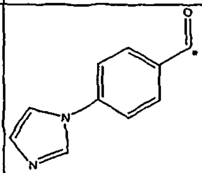 |
| Cg28 | 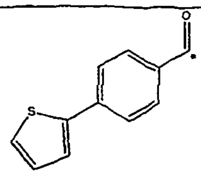 | Cg29 | 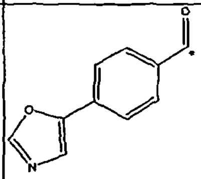 | Cg30 | 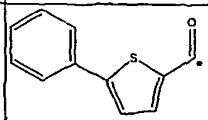 |
| Cg31 | 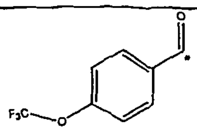 | Cg32 | 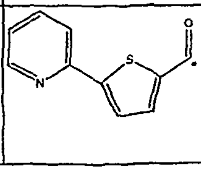 | Cg33 | 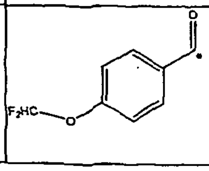 |

| | | | | | |
|---|---|---|---|---|---|
| Cg34 | 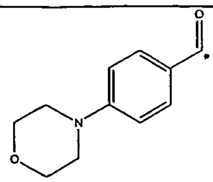 | Cg35 | 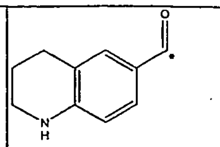 | Cg36 | 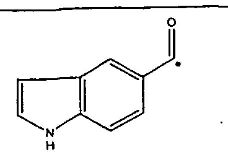 |
| Cg37 | 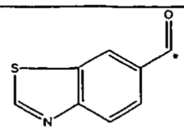 | Cg38 | 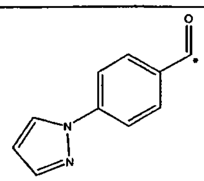 | Cg39 | 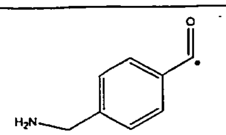 |
| Cg40 | 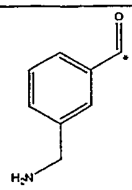 | Cg41 | 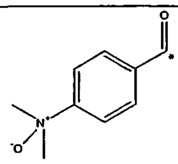 | Cg42 | 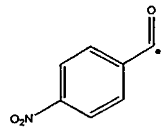 |
| Cg43 | 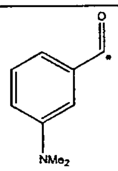 | Cg44 | 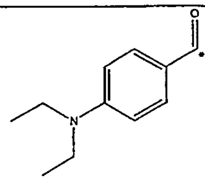 | Cg45 | 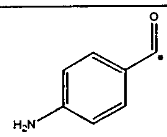 |
| Cg46 | 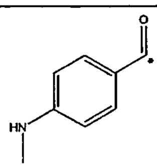 | Cg47 | 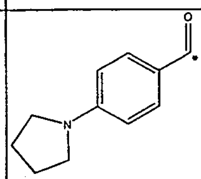 | Cg48 | 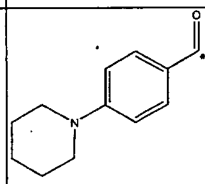 |
| Cg49 | 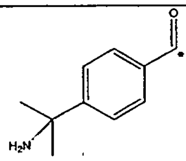 | Cg50 | 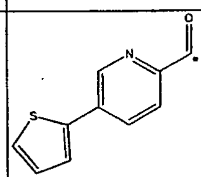 | Cg51 | 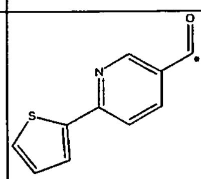 |
| Cg52 | 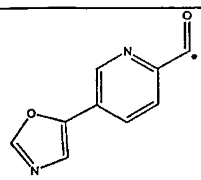 | Cg53 | 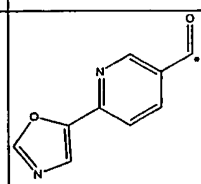 | Cg54 | 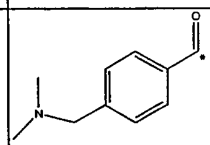 |
| Cg55 | 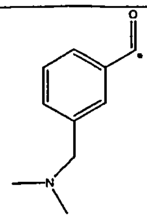 | Cg56 | 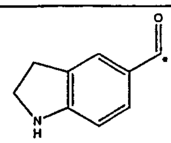 | Cg57 | 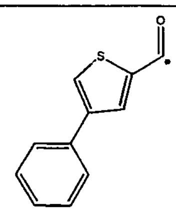 |

| | | | | | |
|---|---|---|---|---|---|
| Cg58 | 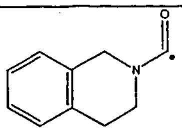 | Cg59 | 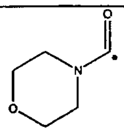 | Cg60 | 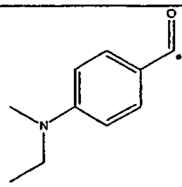 |
| Cg61 | 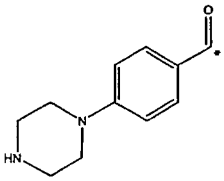 | Cg62 | 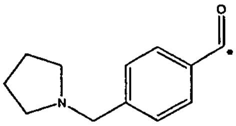 | Cg63 | 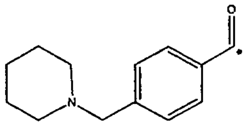 |
| Cg64 | 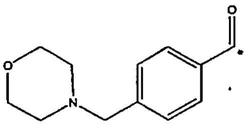 | Cg65 | 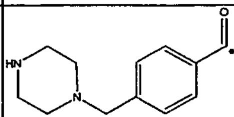 | Cg66 | 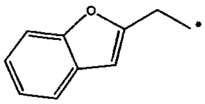 |
| Cg67 | 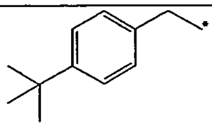 | Cg68 | 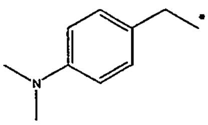 | Cg69 | 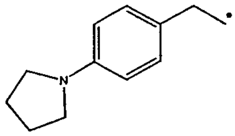 |
| Cg70 | 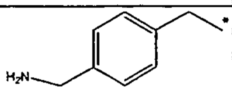 | Cg71 | 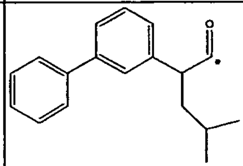 | Cg72 | 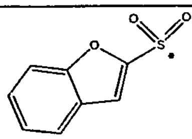 |
| Cg73 | 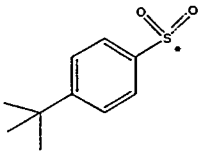 | Cg74 | 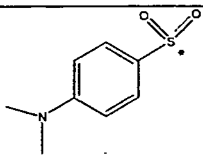 | Cg75 | 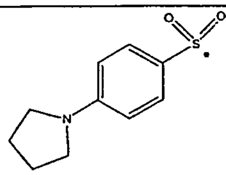 |
| Cg76 | 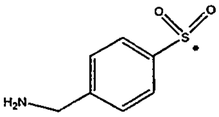 | Cg77 | 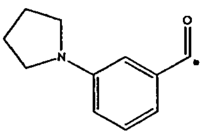 | Cg78 | 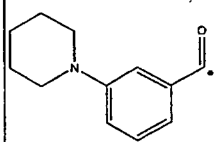 |
| Cg79 | 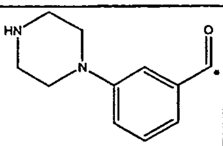 | Cg80 | 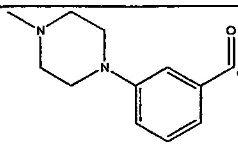 | Cg81 | 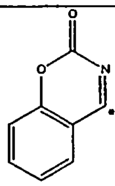 |

| | | | | | |
|---|---|---|---|---|---|
| Cg82 | 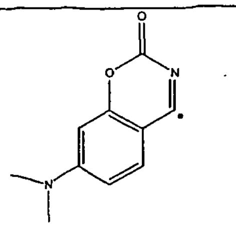 | Cg83 | 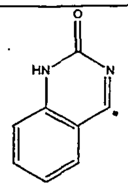 | Cg84 | 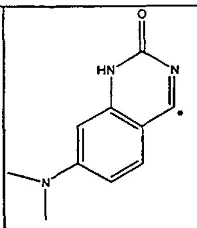 |
| Cg85 | 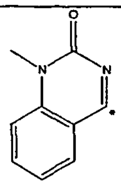 | Cg86 | 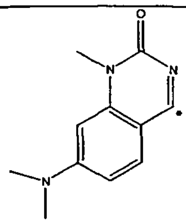 | Cg87 | 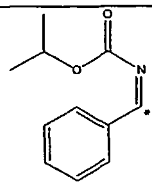 |
| Cg88 | 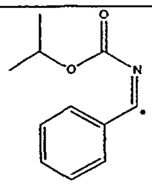 | Cg89 | 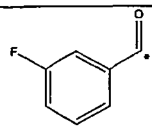 | Cg90 | 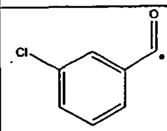 |
| Cg91 | 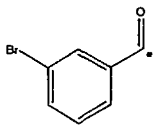 | Cg92 | 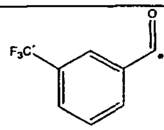 | Cg93 | 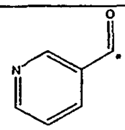 |
| Cg94 | 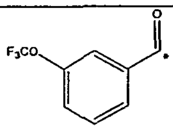 | Cg95 | 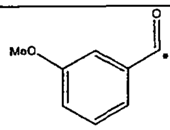 | Cg96 | 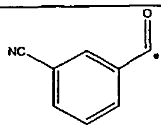 |
| Cg97 | 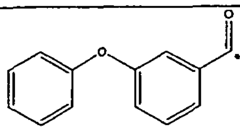 | Cg98 | 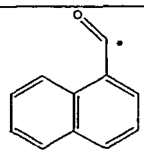 | Cg99 | 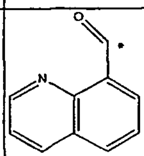 |
| Cg100 | 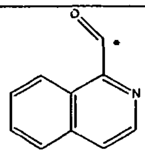 | Cg101 | 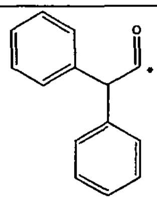 | Cg102 | 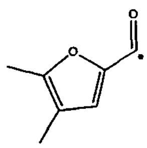 |
| Cg103 | 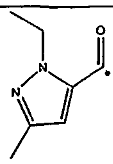 | | | | |

Table (2) 'P2 pocket group Pg' Fragments

** signifies the point of attachment of 'Pg groups' to 5,5-bicyclic scaffold.

| | $(W)_n$-$(X)_o$-Y | | $(W)_n$-$(X)_o$-Y | | $(W)_n$-$(X)_o$-Y |
|---|---|---|---|---|---|
| Pg1 | 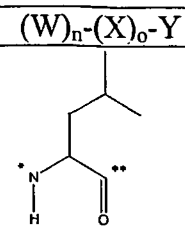 | Pg2 | 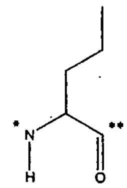 | Pg3 | 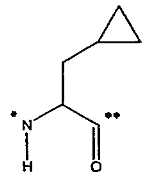 |
| Pg4 | 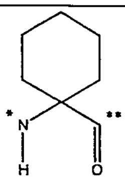 | Pg5 | 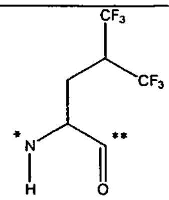 | Pg6 | 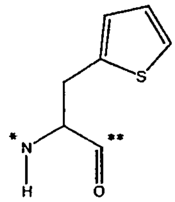 |
| Pg7 | 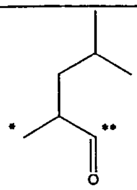 | Pg8 | 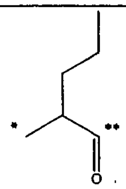 | Pg9 | 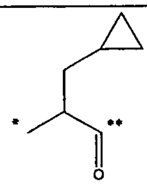 |
| Pg10 | 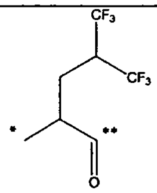 | Pg11 | 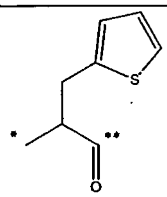 | Pg12 | 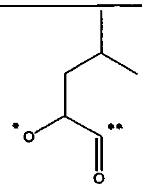 |
| Pg13 | 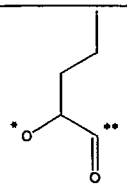 | Pg14 | 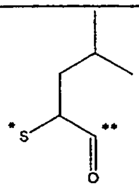 | Pg15 | 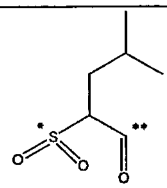 |
| Pg16 | 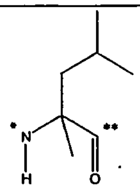 | Pg17 | 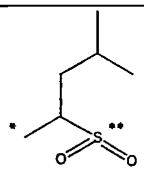 | Pg18 | 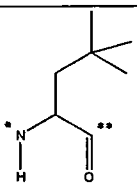 |

| | | | | | |
|---|---|---|---|---|---|
| Pg19 | 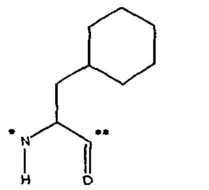 | Pg20 | 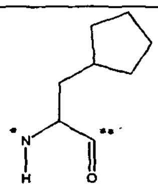 | Pg21 | 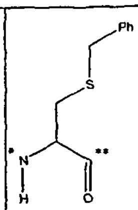 |
| Pg22 | 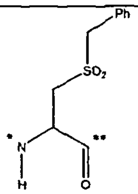 | Pg23 | 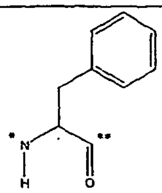 | Pg24 | 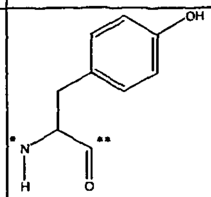 |
| Pg25 | 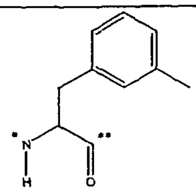 | Pg26 | 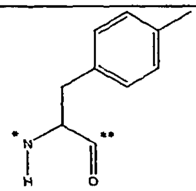 | Pg27 | 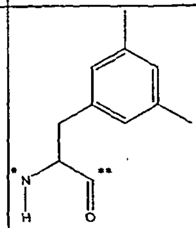 |
| Pg28 | 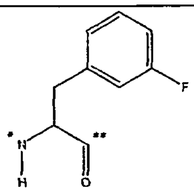 | Pg29 | 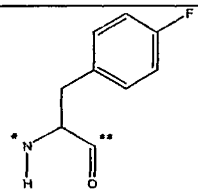 | Pg30 | 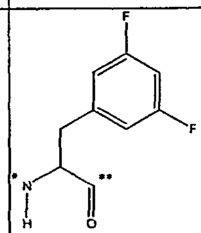 |
| Pg31 | 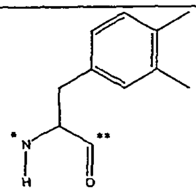 | Pg32 | 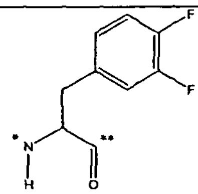 | Pg33 | 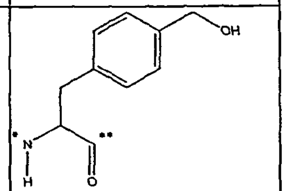 |
| Pg34 | 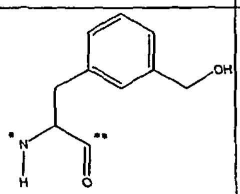 | Pg35 | 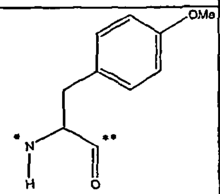 | Pg36 | 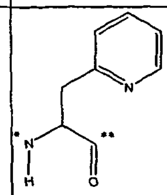 |
| Pg37 | 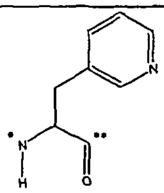 | Pg38 | 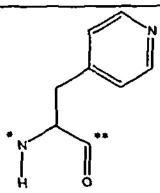 | Pg39 | 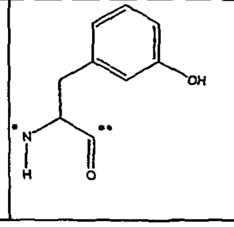 |

Table (3) 'Prime-side binding group Ps' Fragments

*** signifies the point of attachment of 'Ps groups' to 5,5-bicyclic scaffold.

| | $R_1$ | | $R_1$ | | $R_1$ |
|---|---|---|---|---|---|
| Ps1 | 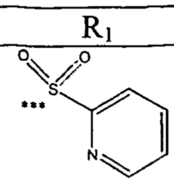 | Ps2 | 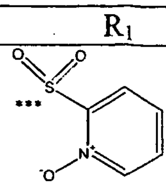 | Ps3 | 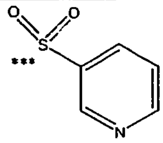 |
| Ps4 | 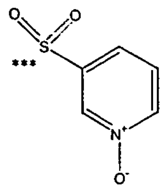 | Ps5 | 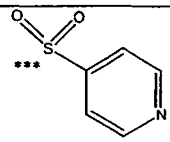 | Ps6 | 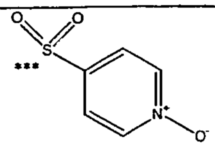 |
| Ps7 | 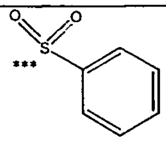 | Ps8 | 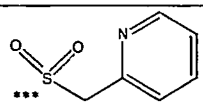 | Ps9 | 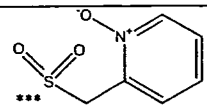 |
| Ps10 | 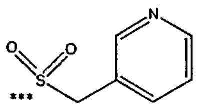 | Ps11 | 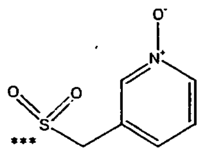 | Ps12 | 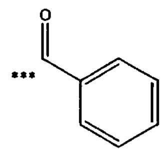 |
| Ps13 | 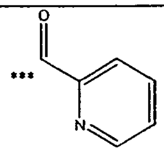 | Ps14 | 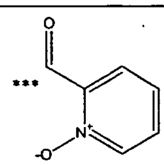 | Ps15 | 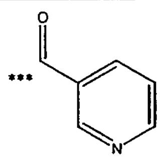 |
| Ps16 | 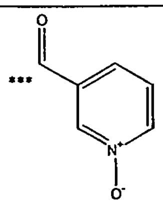 | Ps17 | 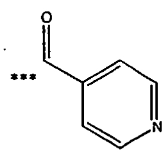 | Ps18 | 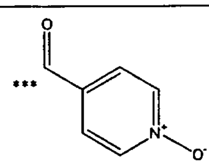 |
| Ps19 | 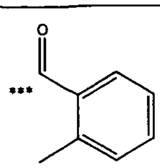 | Ps20 | 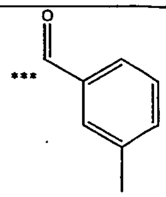 | Ps21 | 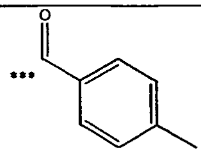 |

| | | | | | |
|---|---|---|---|---|---|
| Ps22 | 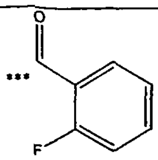 | Ps23 | 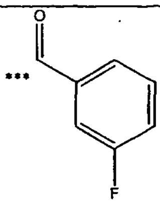 | Ps24 | 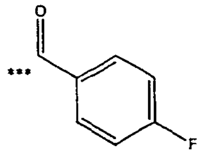 |
| Ps25 | 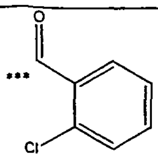 | Ps26 | 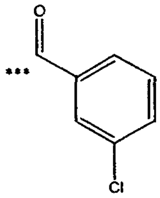 | Ps27 | 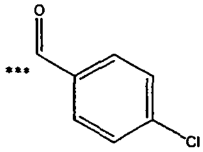 |
| Ps28 | 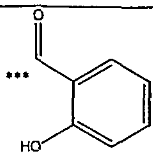 | Ps29 | 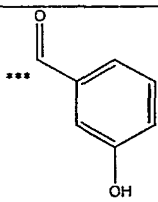 | Ps30 | 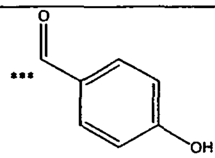 |
| Ps31 | 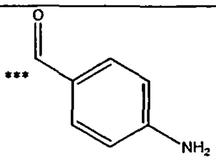 | Ps32 | 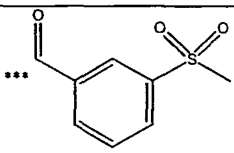 | Ps33 | 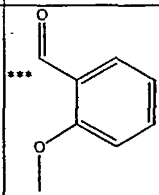 |
| Ps34 | 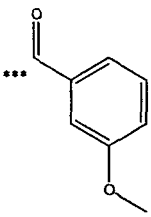 | Ps35 | 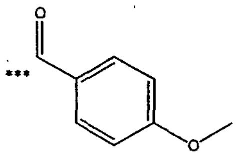 | Ps36 | 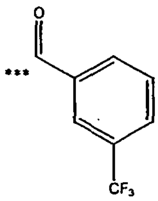 |
| Ps37 | 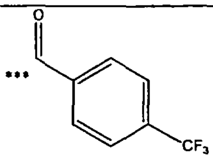 | Ps38 | 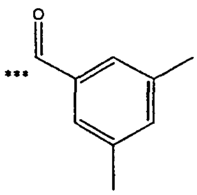 | Ps39 | 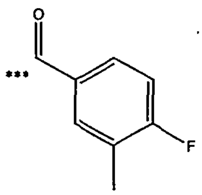 |
| Ps40 | 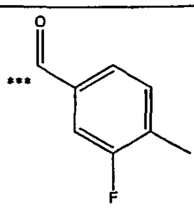 | Ps41 | 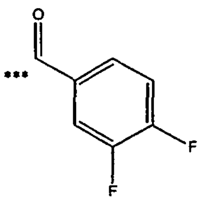 | Ps42 | 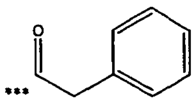 |

| | | | | | |
|---|---|---|---|---|---|
| Ps43 | 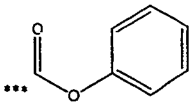 | Ps44 | 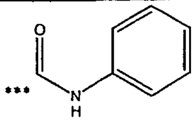 | Ps45 | 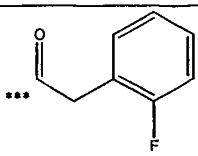 |
| Ps46 | 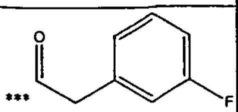 | Ps47 | 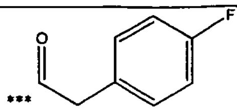 | Ps48 | 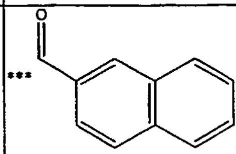 |
| Ps49 | 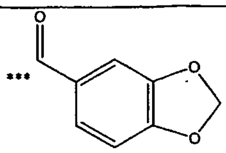 | Ps50 | 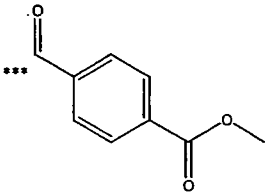 | Ps51 | 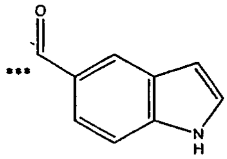 |
| Ps52 | 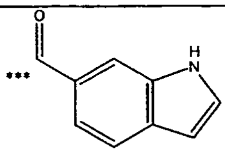 | Ps53 | 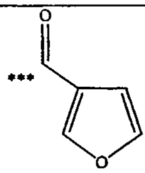 | Ps54 | 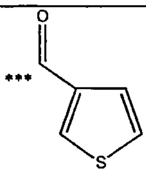 |
| Ps55 | 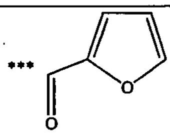 | Ps56 | 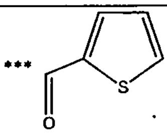 | Ps57 | 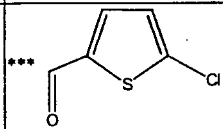 |
| Ps58 | 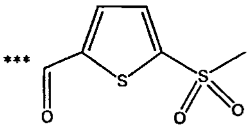 | Ps59 | 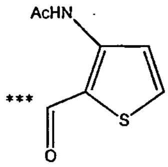 | Ps60 | 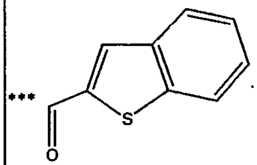 |
| Ps61 | 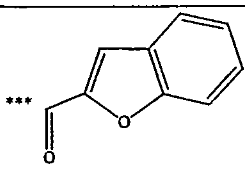 | Ps62 | 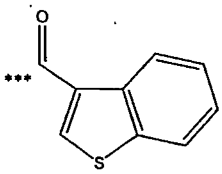 | Ps63 | 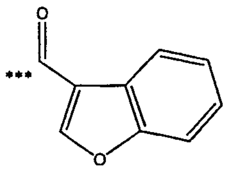 |
| Ps64 | 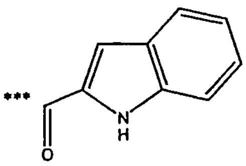 | Ps65 | 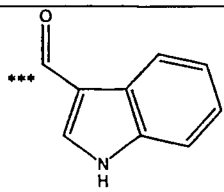 | Ps66 | 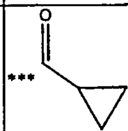 |

| | | | | | |
|---|---|---|---|---|---|
| Ps67 | 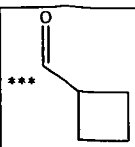 | Ps68 | 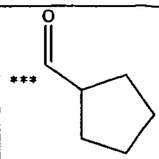 | Ps69 | 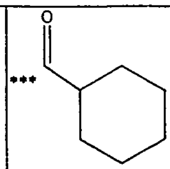 |
| Ps70 | 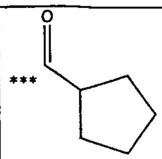 | Ps71 | 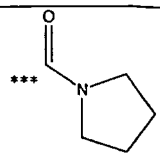 | Ps72 | 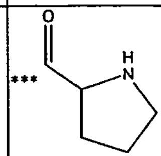 |
| Ps73 | 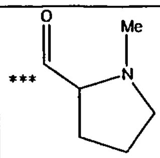 | Ps74 | 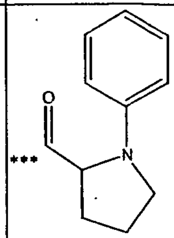 | Ps75 | 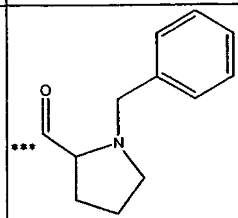 |
| Ps76 | 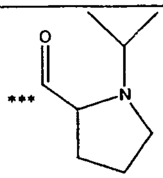 | Ps77 | 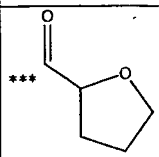 | Ps78 | 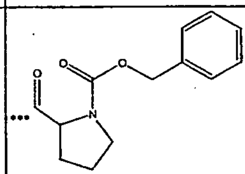 |
| Ps79 | 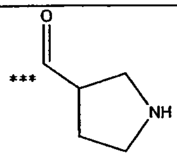 | Ps80 | 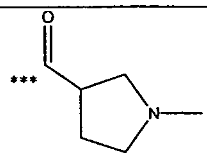 | Ps81 | 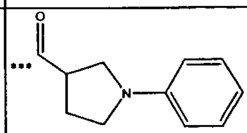 |
| Ps82 | 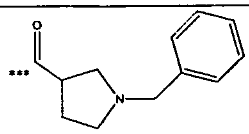 | Ps83 | 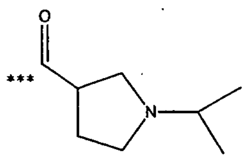 | Ps84 | 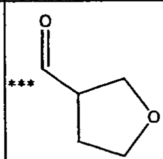 |
| Ps85 | 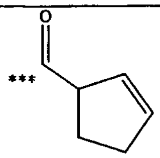 | Ps86 | 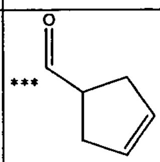 | Ps87 | 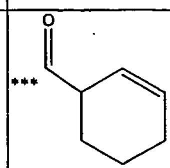 |
| Ps88 | 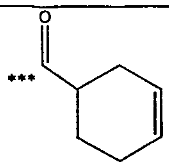 | Ps89 | 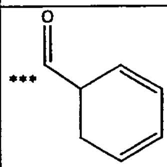 | Ps90 | 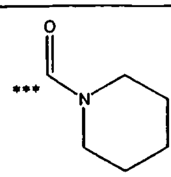 |

| | | | | | |
|---|---|---|---|---|---|
| Ps91 | 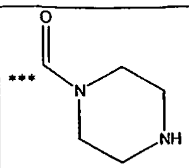 | Ps92 | 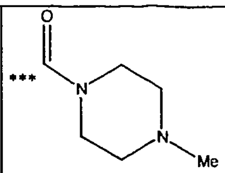 | Ps93 | 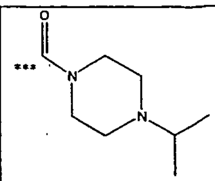 |
| Ps94 | 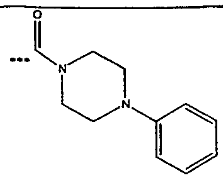 | Ps95 | 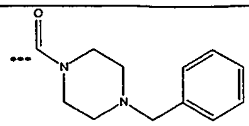 | Ps96 | 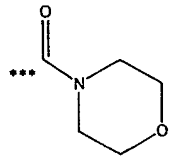 |
| Ps97 | 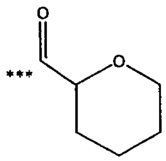 | Ps98 | 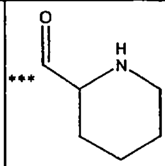 | Ps99 | 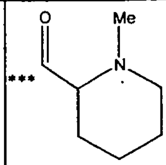 |
| Ps100 | 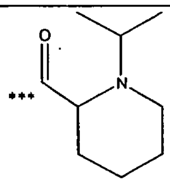 | Ps101 | 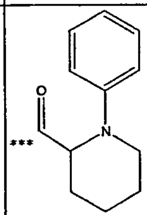 | Ps102 | 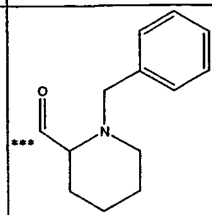 |
| Ps103 | 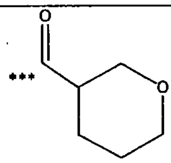 | Ps104 | 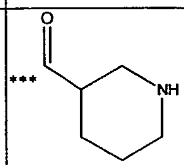 | Ps105 | 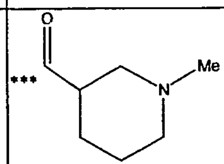 |
| Ps106 | 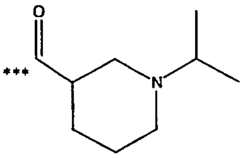 | Ps107 | 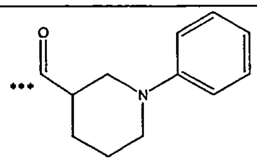 | Ps108 | 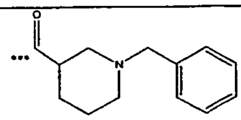 |
| Ps109 | 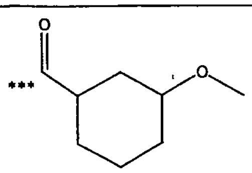 | Ps110 | 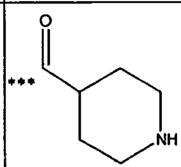 | Ps111 | 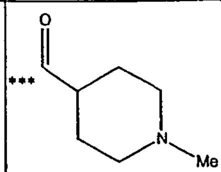 |
| Ps112 | 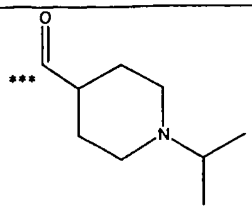 | Ps113 | 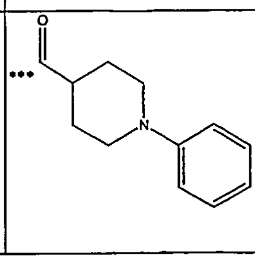 | Ps114 | 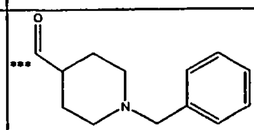 |

| | | | | | |
|---|---|---|---|---|---|
| Ps115 | 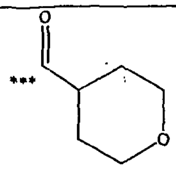 | Ps116 | 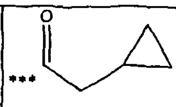 | Ps117 | 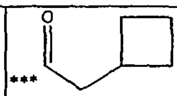 |
| Ps118 | 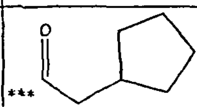 | Ps119 | 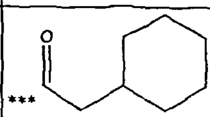 | Ps120 | 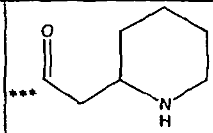 |
| Ps121 | 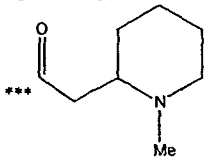 | Ps122 | 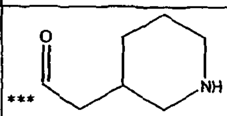 | Ps123 | 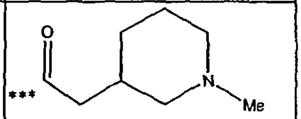 |
| Ps124 | 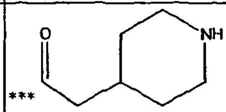 | Ps125 | 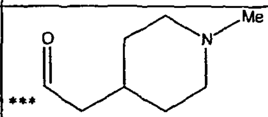 | Ps126 | 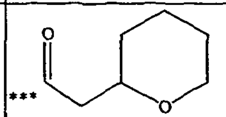 |
| Ps127 | 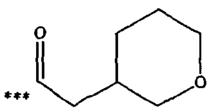 | Ps128 | 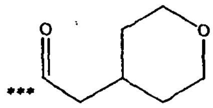 | Ps129 | 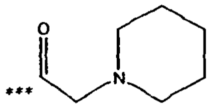 |
| Ps130 | 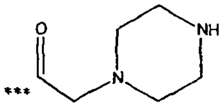 | Ps131 | 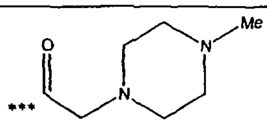 | Ps132 | 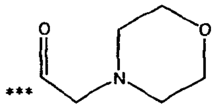 |
| Ps133 | 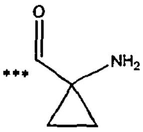 | Ps134 | 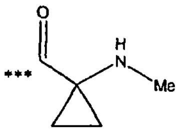 | Ps135 | 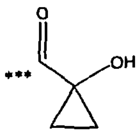 |
| Ps136 | 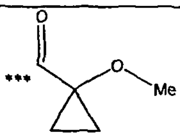 | Ps137 | 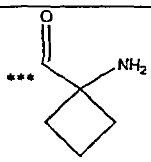 | Ps138 | 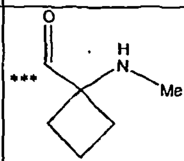 |
| Ps139 | 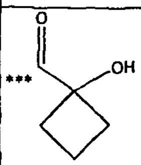 | Ps140 | 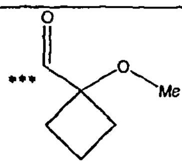 | Ps141 | 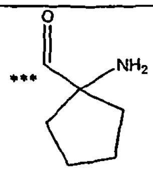 |

| | | | | | |
|---|---|---|---|---|---|
| Ps142 | 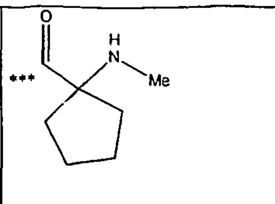 | Ps143 | 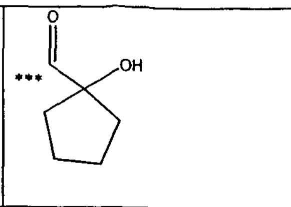 | Ps144 | 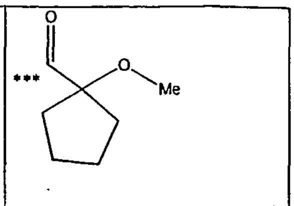 |
| Ps145 | 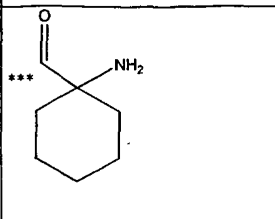 | Ps146 | 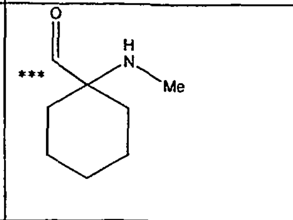 | Ps147 | 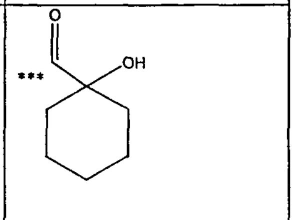 |
| Ps148 | 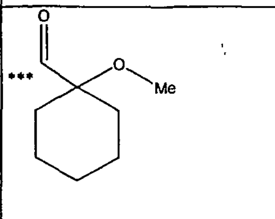 | Ps149 | 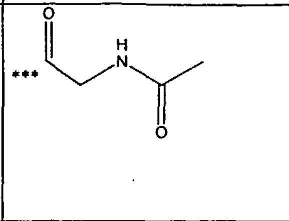 | Ps150 | 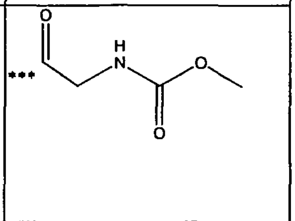 |
| Ps151 | 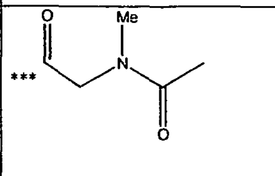 | Ps152 | 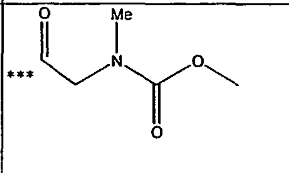 | Ps153 | 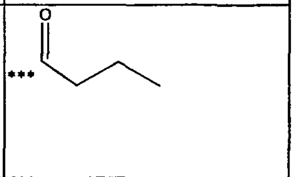 |
| Ps154 | 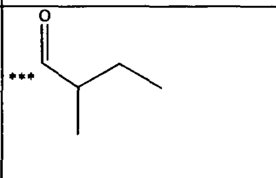 | Ps155 | 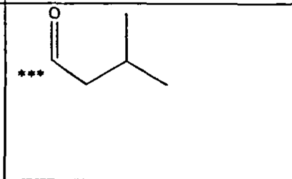 | Ps156 | 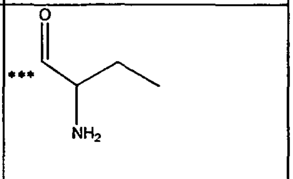 |
| Ps157 | 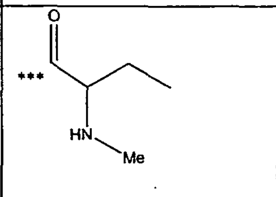 | Ps158 | 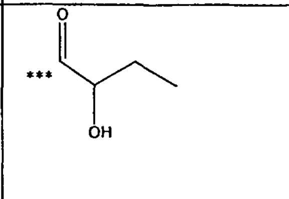 | Ps159 | 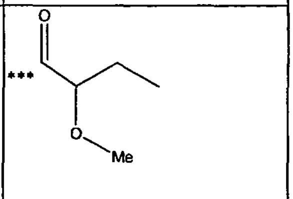 |
| Ps160 | 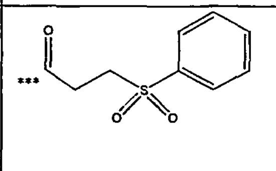 | Ps161 | 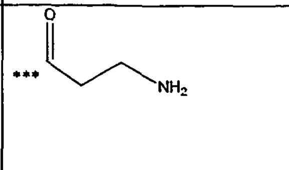 | Ps162 | 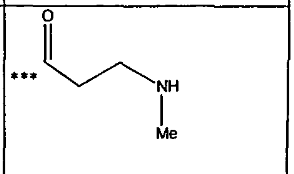 |
| Ps163 | 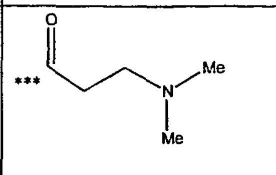 | Ps164 | 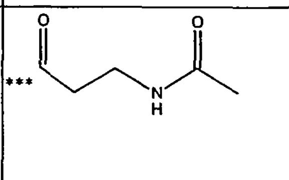 | Ps165 | 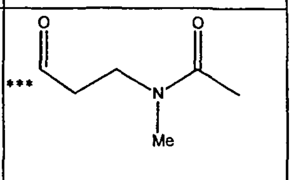 |
| Ps166 | 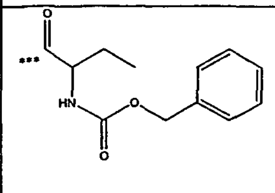 | Ps167 | 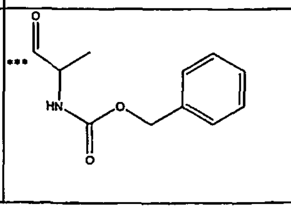 | Ps168 | 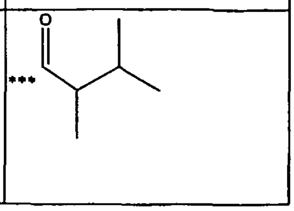 |

| | | | | | |
|---|---|---|---|---|---|
| Ps169 | 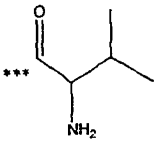 | Ps170 | 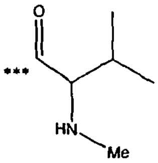 | Ps171 | 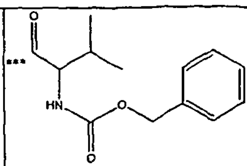 |
| Ps172 | 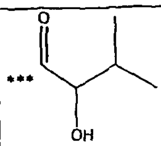 | Ps173 | 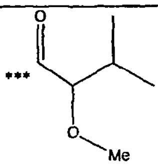 | Ps174 | 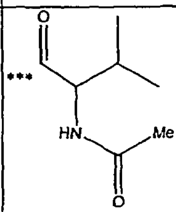 |
| Ps175 | 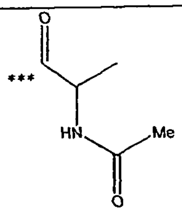 | Ps176 | 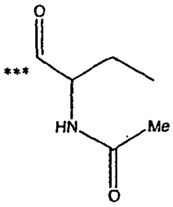 | Ps177 | 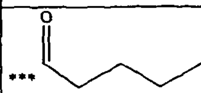 |
| Ps178 | 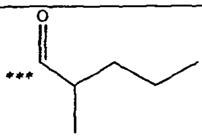 | Ps179 | 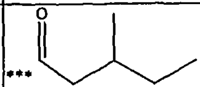 | Ps180 | 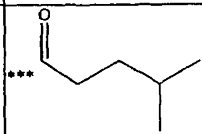 |
| Ps181 | 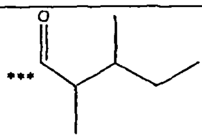 | Ps182 | 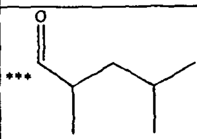 | Ps183 | 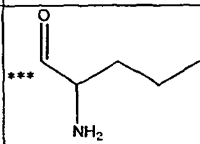 |
| Ps184 | 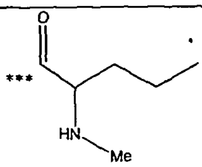 | Ps185 | 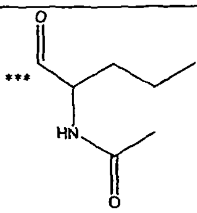 | Ps186 | 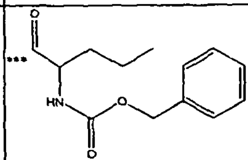 |
| Ps187 | 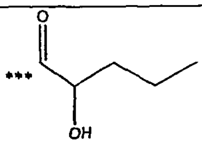 | Ps188 | 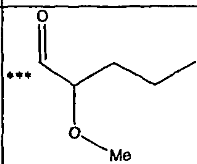 | Ps189 | 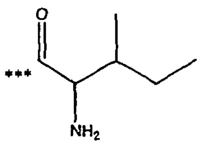 |
| Ps190 | 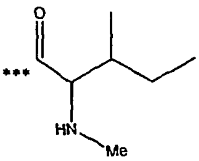 | Ps191 | 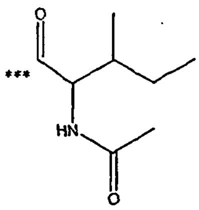 | Ps192 | 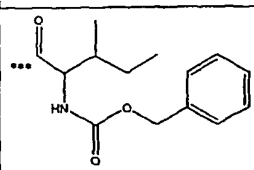 |

| | | | | | |
|---|---|---|---|---|---|
| Ps193 | 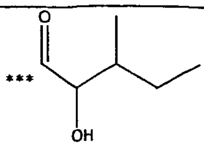 | Ps194 | 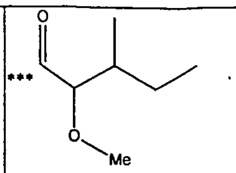 | Ps195 | 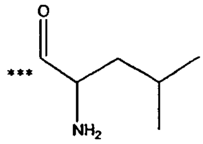 |
| Ps196 | 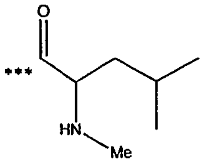 | Ps197 | 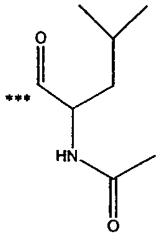 | Ps198 | 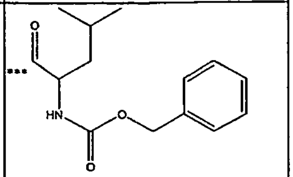 |
| Ps199 | 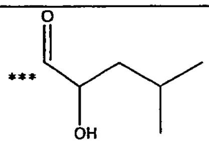 | Ps200 | 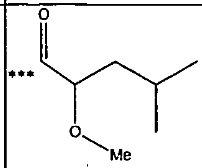 | Ps201 | 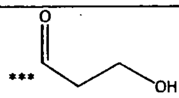 |
| Ps202 | 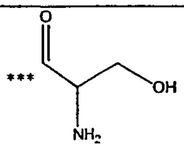 | Ps203 | 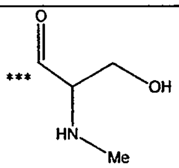 | Ps204 | 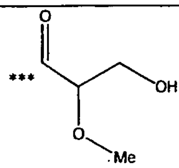 |
| Ps205 | 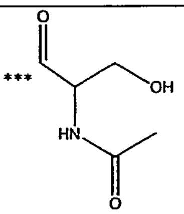 | Ps206 | 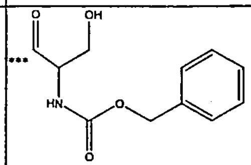 | Ps207 | 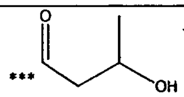 |
| Ps208 | 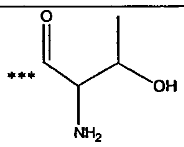 | Ps209 | 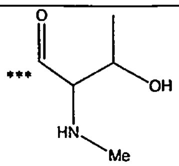 | Ps210 | 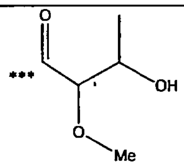 |
| Ps211 | 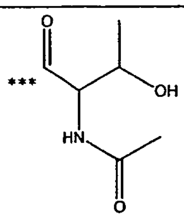 | Ps212 | 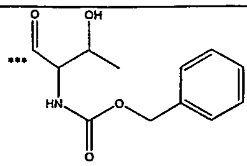 | Ps213 | 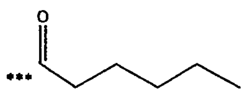 |
| Ps214 | 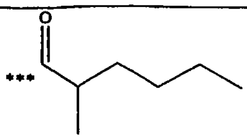 | Ps215 | 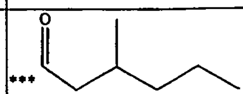 | Ps216 | 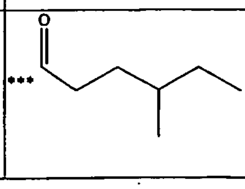 |

| | | | | | |
|---|---|---|---|---|---|
| Ps217 | 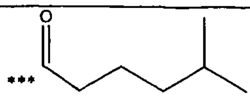 | Ps218 | 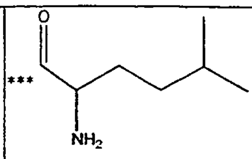 | Ps219 | 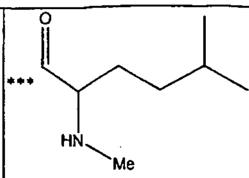 |
| Ps220 | 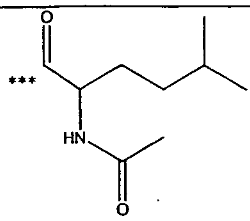 | Ps221 | 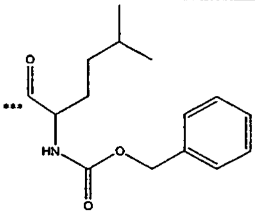 | Ps222 | 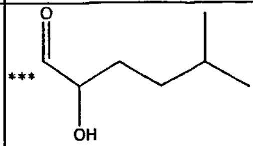 |
| Ps223 | 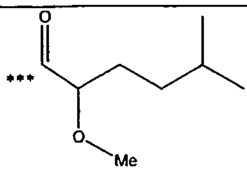 | Ps224 | 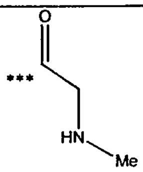 | Ps225 | 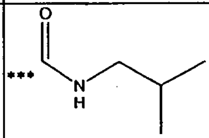 |
| Ps226 | 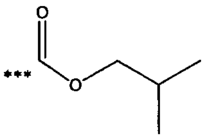 | Ps227 | 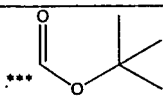 | Ps228 | 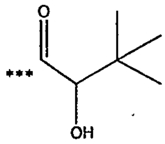 |
| Ps229 | 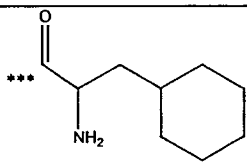 | Ps230 | 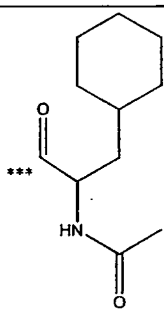 | Ps231 | 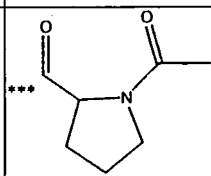 |
| Ps232 | 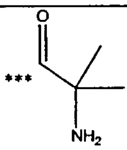 | Ps233 | 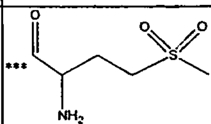 | Ps234 | 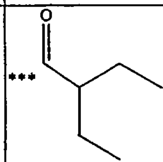 |
| Ps235 | 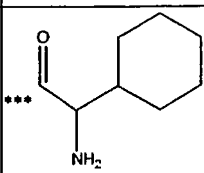 | Ps236 | 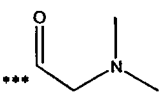 | Ps237 | 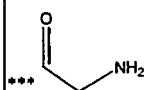 |

| | | | | | |
|---|---|---|---|---|---|
| Ps238 | 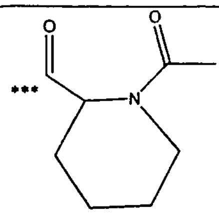 | Ps239 | 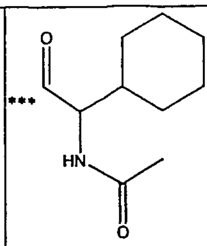 | Ps240 | 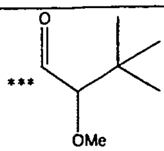 |
| Ps241 | 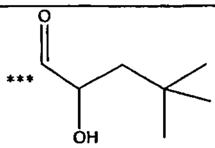 | Ps242 | 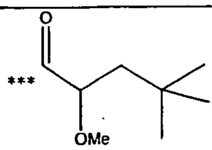 | Ps243 | 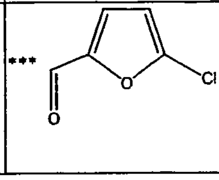 |

Particularly preferred compounds of the invention are inhibitors of cathepsin K and include but are not limited to the compounds formed by the following Cg-Pg-Ps combinations;

| | | | |
|---|---|---|---|
| Cg5–Pg1–Ps1 | Cg5–Pg1–Ps2 | Cg5–Pg1–Ps3 | Cg5–Pg1–Ps4 |
| Cg5–Pg1–Ps5 | Cg5–Pg1–Ps6 | Cg5–Pg1–Ps7 | Cg5–Pg1–Ps8 |
| Cg5–Pg1–Ps9 | Cg5–Pg1–Ps10 | Cg5–Pg1–Ps11 | Cg5–Pg1–Ps12 |
| Cg5–Pg1–Ps13 | Cg5–Pg1–Ps14 | Cg5–Pg1–Ps15 | Cg5–Pg1–Ps16 |
| Cg5–Pg1–Ps17 | Cg5–Pg1–Ps18 | Cg5–Pg1–Ps19 | Cg5–Pg1–Ps20 |
| Cg5–Pg1–Ps21 | Cg5–Pg1–Ps22 | Cg5–Pg1–Ps23 | Cg5–Pg1–Ps24 |
| Cg5–Pg1–Ps25 | Cg5–Pg1–Ps26 | Cg5–Pg1–Ps27 | Cg5–Pg1–Ps28 |
| Cg5–Pg1–Ps29 | Cg5–Pg1–Ps30 | Cg5–Pg1–Ps31 | Cg5–Pg1–Ps32 |
| Cg5–Pg1–Ps33 | Cg5–Pg1–Ps34 | Cg5–Pg1–Ps35 | Cg5–Pg1–Ps36 |
| Cg5–Pg1–Ps37 | Cg5–Pg1–Ps38 | Cg5–Pg1–Ps39 | Cg5–Pg1–Ps40 |
| Cg5–Pg1–Ps41 | Cg5–Pg1–Ps42 | Cg5–Pg1–Ps43 | Cg5–Pg1–Ps44 |
| Cg5–Pg1–Ps45 | Cg5–Pg1–Ps46 | Cg5–Pg1–Ps47 | Cg5–Pg1–Ps48 |
| Cg5–Pg1–Ps49 | Cg5–Pg1–Ps50 | Cg5–Pg1–Ps51 | Cg5–Pg1–Ps52 |
| Cg5–Pg1–Ps53 | Cg5–Pg1–Ps54 | Cg5–Pg1–Ps55 | Cg5–Pg1–Ps56 |
| Cg5–Pg1–Ps57 | Cg5–Pg1–Ps58 | Cg5–Pg1–Ps59 | Cg5–Pg1–Ps60 |
| Cg5–Pg1–Ps61 | Cg5–Pg1–Ps62 | Cg5–Pg1–Ps63 | Cg5–Pg1–Ps64 |
| Cg5–Pg1–Ps65 | Cg5–Pg1–Ps66 | Cg5–Pg1–Ps67 | Cg5–Pg1–Ps68 |
| Cg5–Pg1–Ps69 | Cg5–Pg1–Ps70 | Cg5–Pg1–Ps71 | Cg5–Pg1–Ps72 |
| Cg5–Pg1–Ps73 | Cg5–Pg1–Ps74 | Cg5–Pg1–Ps75 | Cg5–Pg1–Ps76 |
| Cg5–Pg1–Ps77 | Cg5–Pg1–Ps78 | Cg5–Pg1–Ps79 | Cg5–Pg1–Ps80 |
| Cg5–Pg1–Ps81 | Cg5–Pg1–Ps82 | Cg5–Pg1–Ps83 | Cg5–Pg1–Ps84 |
| Cg5–Pg1–Ps85 | Cg5–Pg1–Ps86 | Cg5–Pg1–Ps87 | Cg5–Pg1–Ps88 |
| Cg5–Pg1–Ps89 | Cg5–Pg1–Ps90 | Cg5–Pg1–Ps91 | Cg5–Pg1–Ps92 |
| Cg5–Pg1–Ps93 | Cg5–Pg1–Ps94 | Cg5–Pg1–Ps95 | Cg5–Pg1–Ps96 |
| Cg5–Pg1–Ps97 | Cg5–Pg1–Ps98 | Cg5–Pg1–Ps99 | Cg5–Pg1–Ps100 |
| Cg5–Pg1–Ps101 | Cg5–Pg1–Ps102 | Cg5–Pg1–Ps103 | Cg5–Pg1–Ps104 |
| Cg5–Pg1–Ps105 | Cg5–Pg1–Ps106 | Cg5–Pg1–Ps107 | Cg5–Pg1–Ps108 |
| Cg5–Pg1–Ps109 | Cg5–Pg1–Ps110 | Cg5–Pg1–Ps111 | Cg5–Pg1–Ps112 |
| Cg5–Pg1–Ps113 | Cg5–Pg1–Ps114 | Cg5–Pg1–Ps115 | Cg5–Pg1–Ps116 |
| Cg5–Pg1–Ps117 | Cg5–Pg1–Ps118 | Cg5–Pg1–Ps119 | Cg5–Pg1–Ps120 |

| | | | |
|---|---|---|---|
| Cg5–Pg1–Ps121 | Cg5–Pg1–Ps122 | Cg5–Pg1–Ps123 | Cg5–Pg1–Ps124 |
| Cg5–Pg1–Ps125 | Cg5–Pg1–Ps126 | Cg5–Pg1–Ps127 | Cg5–Pg1–Ps128 |
| Cg5–Pg1–Ps129 | Cg5–Pg1–Ps130 | Cg5–Pg1–Ps131 | Cg5–Pg1–Ps132 |
| Cg5–Pg1–Ps133 | Cg5–Pg1–Ps134 | Cg5–Pg1–Ps135 | Cg5–Pg1–Ps136 |
| Cg5–Pg1–Ps137 | Cg5–Pg1–Ps138 | Cg5–Pg1–Ps139 | Cg5–Pg1–Ps140 |
| Cg5–Pg1–Ps141 | Cg5–Pg1–Ps142 | Cg5–Pg1–Ps143 | Cg5–Pg1–Ps144 |
| Cg5–Pg1–Ps145 | Cg5–Pg1–Ps146 | Cg5–Pg1–Ps147 | Cg5–Pg1–Ps148 |
| Cg5–Pg1–Ps149 | Cg5–Pg1–Ps150 | Cg5–Pg1–Ps151 | Cg5–Pg1–Ps152 |
| Cg5–Pg1–Ps153 | Cg5–Pg1–Ps154 | Cg5–Pg1–Ps155 | Cg5–Pg1–Ps156 |
| Cg5–Pg1–Ps157 | Cg5–Pg1–Ps158 | Cg5–Pg1–Ps159 | Cg5–Pg1–Ps160 |
| Cg5–Pg1–Ps161 | Cg5–Pg1–Ps162 | Cg5–Pg1–Ps163 | Cg5–Pg1–Ps164 |
| Cg5–Pg1–Ps165 | Cg5–Pg1–Ps166 | Cg5–Pg1–Ps167 | Cg5–Pg1–Ps168 |
| Cg5–Pg1–Ps169 | Cg5–Pg1–Ps170 | Cg5–Pg1–Ps171 | Cg5–Pg1–Ps172 |
| Cg5–Pg1–Ps173 | Cg5–Pg1–Ps174 | Cg5–Pg1–Ps175 | Cg5–Pg1–Ps176 |
| Cg5–Pg1–Ps177 | Cg5–Pg1–Ps178 | Cg5–Pg1–Ps179 | Cg5–Pg1–Ps180 |
| Cg5–Pg1–Ps181 | Cg5–Pg1–Ps182 | Cg5–Pg1–Ps183 | Cg5–Pg1–Ps184 |
| Cg5–Pg1–Ps185 | Cg5–Pg1–Ps186 | Cg5–Pg1–Ps187 | Cg5–Pg1–Ps188 |
| Cg5–Pg1–Ps189 | Cg5–Pg1–Ps190 | Cg5–Pg1–Ps191 | Cg5–Pg1–Ps192 |
| Cg5–Pg1–Ps193 | Cg5–Pg1–Ps194 | Cg5–Pg1–Ps195 | Cg5–Pg1–Ps196 |
| Cg5–Pg1–Ps197 | Cg5–Pg1–Ps198 | Cg5–Pg1–Ps199 | Cg5–Pg1–Ps200 |
| Cg5–Pg1–Ps201 | Cg5–Pg1–Ps202 | Cg5–Pg1–Ps203 | Cg5–Pg1–Ps204 |
| Cg5–Pg1–Ps205 | Cg5–Pg1–Ps206 | Cg5–Pg1–Ps207 | Cg5–Pg1–Ps208 |
| Cg5–Pg1–Ps209 | Cg5–Pg1–Ps210 | Cg5–Pg1–Ps211 | Cg5–Pg1–Ps212 |
| Cg5–Pg1–Ps213 | Cg5–Pg1–Ps214 | Cg5–Pg1–Ps215 | Cg5–Pg1–Ps216 |
| Cg5–Pg1–Ps217 | Cg5–Pg1–Ps218 | Cg5–Pg1–Ps219 | Cg5–Pg1–Ps220 |
| Cg5–Pg1–Ps221 | Cg5–Pg1–Ps222 | Cg5–Pg1–Ps223 | Cg5–Pg1–Ps224 |
| Cg5–Pg1–Ps225 | Cg5–Pg1–Ps226 | Cg5–Pg1–Ps227 | Cg5–Pg1–Ps228 |
| Cg5–Pg1–Ps229 | Cg5–Pg1–Ps230 | Cg5–Pg1–Ps231 | Cg5–Pg1–Ps232 |
| Cg5–Pg1–Ps233 | Cg5–Pg1–Ps234 | Cg5–Pg1–Ps235 | Cg5–Pg1–Ps236 |
| Cg5–Pg1–Ps237 | Cg5–Pg1–Ps238 | Cg5–Pg1–Ps239 | Cg5–Pg1–Ps240 |
| Cg5–Pg1–Ps241 | Cg5–Pg1–Ps242 | Cg5–Pg1–Ps243 | |

| | | | |
|---|---|---|---|
| Cg6–Pg1–Ps1 | Cg6–Pg1–Ps2 | Cg6–Pg1–Ps3 | Cg6–Pg1–Ps4 |
| Cg6–Pg1–Ps5 | Cg6–Pg1–Ps6 | Cg6–Pg1–Ps7 | Cg6–Pg1–Ps8 |
| Cg6–Pg1–Ps9 | Cg6–Pg1–Ps10 | Cg6–Pg1–Ps11 | Cg6–Pg1–Ps12 |
| Cg6–Pg1–Ps13 | Cg6–Pg1–Ps14 | Cg6–Pg1–Ps15 | Cg6–Pg1–Ps16 |
| Cg6–Pg1–Ps17 | Cg6–Pg1–Ps18 | Cg6–Pg1–Ps19 | Cg6–Pg1–Ps20 |
| Cg6–Pg1–Ps21 | Cg6–Pg1–Ps22 | Cg6–Pg1–Ps23 | Cg6–Pg1–Ps24 |
| Cg6–Pg1–Ps25 | Cg6–Pg1–Ps26 | Cg6–Pg1–Ps27 | Cg6–Pg1–Ps28 |
| Cg6–Pg1–Ps29 | Cg6–Pg1–Ps30 | Cg6–Pg1–Ps31 | Cg6–Pg1–Ps32 |
| Cg6–Pg1–Ps33 | Cg6–Pg1–Ps34 | Cg6–Pg1–Ps35 | Cg6–Pg1–Ps36 |
| Cg6–Pg1–Ps37 | Cg6–Pg1–Ps38 | Cg6–Pg1–Ps39 | Cg6–Pg1–Ps40 |
| Cg6–Pg1–Ps41 | Cg6–Pg1–Ps42 | Cg6–Pg1–Ps43 | Cg6–Pg1–Ps44 |
| Cg6–Pg1–Ps45 | Cg6–Pg1–Ps46 | Cg6–Pg1–Ps47 | Cg6–Pg1–Ps48 |
| Cg6–Pg1–Ps49 | Cg6–Pg1–Ps50 | Cg6–Pg1–Ps51 | Cg6–Pg1–Ps52 |
| Cg6–Pg1–Ps53 | Cg6–Pg1–Ps54 | Cg6–Pg1–Ps55 | Cg6–Pg1–Ps56 |
| Cg6–Pg1–Ps57 | Cg6–Pg1–Ps58 | Cg6–Pg1–Ps59 | Cg6–Pg1–Ps60 |
| Cg6–Pg1–Ps61 | Cg6–Pg1–Ps62 | Cg6–Pg1–Ps63 | Cg6–Pg1–Ps64 |
| Cg6–Pg1–Ps65 | Cg6–Pg1–Ps66 | Cg6–Pg1–Ps67 | Cg6–Pg1–Ps68 |
| Cg6–Pg1–Ps69 | Cg6–Pg1–Ps70 | Cg6–Pg1–Ps71 | Cg6–Pg1–Ps72 |
| Cg6–Pg1–Ps73 | Cg6–Pg1–Ps74 | Cg6–Pg1–Ps75 | Cg6–Pg1–Ps76 |
| Cg6–Pg1–Ps77 | Cg6–Pg1–Ps78 | Cg6–Pg1–Ps79 | Cg6–Pg1–Ps80 |

| | | | |
|---|---|---|---|
| Cg6–Pg1–Ps81 | Cg6–Pg1–Ps82 | Cg6–Pg1–Ps83 | Cg6–Pg1–Ps84 |
| Cg6–Pg1–Ps85 | Cg6–Pg1–Ps86 | Cg6–Pg1–Ps87 | Cg6–Pg1–Ps88 |
| Cg6–Pg1–Ps89 | Cg6–Pg1–Ps90 | Cg6–Pg1–Ps91 | Cg6–Pg1–Ps92 |
| Cg6–Pg1–Ps93 | Cg6–Pg1–Ps94 | Cg6–Pg1–Ps95 | Cg6–Pg1–Ps96 |
| Cg6–Pg1–Ps97 | Cg6–Pg1–Ps98 | Cg6–Pg1–Ps99 | Cg6–Pg1–Ps100 |
| Cg6–Pg1–Ps101 | Cg6–Pg1–Ps102 | Cg6–Pg1–Ps103 | Cg6–Pg1–Ps104 |
| Cg6–Pg1–Ps105 | Cg6–Pg1–Ps106 | Cg6–Pg1–Ps107 | Cg6–Pg1–Ps108 |
| Cg6–Pg1–Ps109 | Cg6–Pg1–Ps110 | Cg6–Pg1–Ps111 | Cg6–Pg1–Ps112 |
| Cg6–Pg1–Ps113 | Cg6–Pg1–Ps114 | Cg6–Pg1–Ps115 | Cg6–Pg1–Ps116 |
| Cg6–Pg1–Ps117 | Cg6–Pg1–Ps118 | Cg6–Pg1–Ps119 | Cg6–Pg1–Ps120 |
| Cg6–Pg1–Ps121 | Cg6–Pg1–Ps122 | Cg6–Pg1–Ps123 | Cg6–Pg1–Ps124 |
| Cg6–Pg1–Ps125 | Cg6–Pg1–Ps126 | Cg6–Pg1–Ps127 | Cg6–Pg1–Ps128 |
| Cg6–Pg1–Ps129 | Cg6–Pg1–Ps130 | Cg6–Pg1–Ps131 | Cg6–Pg1–Ps132 |
| Cg6–Pg1–Ps133 | Cg6–Pg1–Ps134 | Cg6–Pg1–Ps135 | Cg6–Pg1–Ps136 |
| Cg6–Pg1–Ps137 | Cg6–Pg1–Ps138 | Cg6–Pg1–Ps139 | Cg6–Pg1–Ps140 |
| Cg6–Pg1–Ps141 | Cg6–Pg1–Ps142 | Cg6–Pg1–Ps143 | Cg6–Pg1–Ps144 |
| Cg6–Pg1–Ps145 | Cg6–Pg1–Ps146 | Cg6–Pg1–Ps147 | Cg6–Pg1–Ps148 |
| Cg6–Pg1–Ps149 | Cg6–Pg1–Ps150 | Cg6–Pg1–Ps151 | Cg6–Pg1–Ps152 |
| Cg6–Pg1–Ps153 | Cg6–Pg1–Ps154 | Cg6–Pg1–Ps155 | Cg6–Pg1–Ps156 |
| Cg6–Pg1–Ps157 | Cg6–Pg1–Ps158 | Cg6–Pg1–Ps159 | Cg6–Pg1–Ps160 |
| Cg6–Pg1–Ps161 | Cg6–Pg1–Ps162 | Cg6–Pg1–Ps163 | Cg6–Pg1–Ps164 |
| Cg6–Pg1–Ps165 | Cg6–Pg1–Ps166 | Cg6–Pg1–Ps167 | Cg6–Pg1–Ps168 |
| Cg6–Pg1–Ps169 | Cg6–Pg1–Ps170 | Cg6–Pg1–Ps171 | Cg6–Pg1–Ps172 |
| Cg6–Pg1–Ps173 | Cg6–Pg1–Ps174 | Cg6–Pg1–Ps175 | Cg6–Pg1–Ps176 |
| Cg6–Pg1–Ps177 | Cg6–Pg1–Ps178 | Cg6–Pg1–Ps179 | Cg6–Pg1–Ps180 |
| Cg6–Pg1–Ps181 | Cg6–Pg1–Ps182 | Cg6–Pg1–Ps183 | Cg6–Pg1–Ps184 |
| Cg6–Pg1–Ps185 | Cg6–Pg1–Ps186 | Cg6–Pg1–Ps187 | Cg6–Pg1–Ps188 |
| Cg6–Pg1–Ps189 | Cg6–Pg1–Ps190 | Cg6–Pg1–Ps191 | Cg6–Pg1–Ps192 |
| Cg6–Pg1–Ps193 | Cg6–Pg1–Ps194 | Cg6–Pg1–Ps195 | Cg6–Pg1–Ps196 |
| Cg6–Pg1–Ps197 | Cg6–Pg1–Ps198 | Cg6–Pg1–Ps199 | Cg6–Pg1–Ps200 |
| Cg6–Pg1–Ps201 | Cg6–Pg1–Ps202 | Cg6–Pg1–Ps203 | Cg6–Pg1–Ps204 |
| Cg6–Pg1–Ps205 | Cg6–Pg1–Ps206 | Cg6–Pg1–Ps207 | Cg6–Pg1–Ps208 |
| Cg6–Pg1–Ps209 | Cg6–Pg1–Ps210 | Cg6–Pg1–Ps211 | Cg6–Pg1–Ps212 |
| Cg6–Pg1–Ps213 | Cg6–Pg1–Ps214 | Cg6–Pg1–Ps215 | Cg6–Pg1–Ps216 |
| Cg6–Pg1–Ps217 | Cg6–Pg1–Ps218 | Cg6–Pg1–Ps219 | Cg6–Pg1–Ps220 |
| Cg6–Pg1–Ps221 | Cg6–Pg1–Ps222 | Cg6–Pg1–Ps223 | Cg6–Pg1–Ps224 |
| Cg6–Pg1–Ps225 | Cg6–Pg1–Ps226 | Cg6–Pg1–Ps227 | Cg6–Pg1–Ps228 |
| Cg6–Pg1–Ps229 | Cg6–Pg1–Ps230 | Cg6–Pg1–Ps231 | Cg6–Pg1–Ps232 |
| Cg6–Pg1–Ps233 | Cg6–Pg1–Ps234 | Cg6–Pg1–Ps235 | Cg6–Pg1–Ps236 |
| Cg6–Pg1–Ps237 | Cg6–Pg1–Ps238 | Cg6–Pg1–Ps239 | Cg6–Pg1–Ps240 |
| Cg6–Pg1–Ps241 | Cg6–Pg1–Ps242 | Cg6–Pg1–Ps243 | |

| | | | |
|---|---|---|---|
| Cg7–Pg1–Ps1 | Cg7–Pg1–Ps2 | Cg7–Pg1–Ps3 | Cg7–Pg1–Ps4 |
| Cg7–Pg1–Ps5 | Cg7–Pg1–Ps6 | Cg7–Pg1–Ps7 | Cg7–Pg1–Ps8 |
| Cg7–Pg1–Ps9 | Cg7–Pg1–Ps10 | Cg7–Pg1–Ps11 | Cg7–Pg1–Ps12 |
| Cg7–Pg1–Ps13 | Cg7–Pg1–Ps14 | Cg7–Pg1–Ps15 | Cg7–Pg1–Ps16 |
| Cg7–Pg1–Ps17 | Cg7–Pg1–Ps18 | Cg7–Pg1–Ps19 | Cg7–Pg1–Ps20 |
| Cg7–Pg1–Ps21 | Cg7–Pg1–Ps22 | Cg7–Pg1–Ps23 | Cg7–Pg1–Ps24 |
| Cg7–Pg1–Ps25 | Cg7–Pg1–Ps26 | Cg7–Pg1–Ps27 | Cg7–Pg1–Ps28 |
| Cg7–Pg1–Ps29 | Cg7–Pg1–Ps30 | Cg7–Pg1–Ps31 | Cg7–Pg1–Ps32 |
| Cg7–Pg1–Ps33 | Cg7–Pg1–Ps34 | Cg7–Pg1–Ps35 | Cg7–Pg1–Ps36 |
| Cg7–Pg1–Ps37 | Cg7–Pg1–Ps38 | Cg7–Pg1–Ps39 | Cg7–Pg1–Ps40 |

| | | | |
|---|---|---|---|
| Cg7–Pg1–Ps41 | Cg7–Pg1–Ps42 | Cg7–Pg1–Ps43 | Cg7–Pg1–Ps44 |
| Cg7–Pg1–Ps45 | Cg7–Pg1–Ps46 | Cg7–Pg1–Ps47 | Cg7–Pg1–Ps48 |
| Cg7–Pg1–Ps49 | Cg7–Pg1–Ps50 | Cg7–Pg1–Ps51 | Cg7–Pg1–Ps52 |
| Cg7–Pg1–Ps53 | Cg7–Pg1–Ps54 | Cg7–Pg1–Ps55 | Cg7–Pg1–Ps56 |
| Cg7–Pg1–Ps57 | Cg7–Pg1–Ps58 | Cg7–Pg1–Ps59 | Cg7–Pg1–Ps60 |
| Cg7–Pg1–Ps61 | Cg7–Pg1–Ps62 | Cg7–Pg1–Ps63 | Cg7–Pg1–Ps64 |
| Cg7–Pg1–Ps65 | Cg7–Pg1–Ps66 | Cg7–Pg1–Ps67 | Cg7–Pg1–Ps68 |
| Cg7–Pg1–Ps69 | Cg7–Pg1–Ps70 | Cg7–Pg1–Ps71 | Cg7–Pg1–Ps72 |
| Cg7–Pg1–Ps73 | Cg7–Pg1–Ps74 | Cg7–Pg1–Ps75 | Cg7–Pg1–Ps76 |
| Cg7–Pg1–Ps77 | Cg7–Pg1–Ps78 | Cg7–Pg1–Ps79 | Cg7–Pg1–Ps80 |
| Cg7–Pg1–Ps81 | Cg7–Pg1–Ps82 | Cg7–Pg1–Ps83 | Cg7–Pg1–Ps84 |
| Cg7–Pg1–Ps85 | Cg7–Pg1–Ps86 | Cg7–Pg1–Ps87 | Cg7–Pg1–Ps88 |
| Cg7–Pg1–Ps89 | Cg7–Pg1–Ps90 | Cg7–Pg1–Ps91 | Cg7–Pg1–Ps92 |
| Cg7–Pg1–Ps93 | Cg7–Pg1–Ps94 | Cg7–Pg1–Ps95 | Cg7–Pg1–Ps96 |
| Cg7–Pg1–Ps97 | Cg7–Pg1–Ps98 | Cg7–Pg1–Ps99 | Cg7–Pg1–Ps100 |
| Cg7–Pg1–Ps101 | Cg7–Pg1–Ps102 | Cg7–Pg1–Ps103 | Cg7–Pg1–Ps104 |
| Cg7–Pg1–Ps105 | Cg7–Pg1–Ps106 | Cg7–Pg1–Ps107 | Cg7–Pg1–Ps108 |
| Cg7–Pg1–Ps109 | Cg7–Pg1–Ps110 | Cg7–Pg1–Ps111 | Cg7–Pg1–Ps112 |
| Cg7–Pg1–Ps113 | Cg7–Pg1–Ps114 | Cg7–Pg1–Ps115 | Cg7–Pg1–Ps116 |
| Cg7–Pg1–Ps117 | Cg7–Pg1–Ps118 | Cg7–Pg1–Ps119 | Cg7–Pg1–Ps120 |
| Cg7–Pg1–Ps121 | Cg7–Pg1–Ps122 | Cg7–Pg1–Ps123 | Cg7–Pg1–Ps124 |
| Cg7–Pg1–Ps125 | Cg7–Pg1–Ps126 | Cg7–Pg1–Ps127 | Cg7–Pg1–Ps128 |
| Cg7–Pg1–Ps129 | Cg7–Pg1–Ps130 | Cg7–Pg1–Ps131 | Cg7–Pg1–Ps132 |
| Cg7–Pg1–Ps133 | Cg7–Pg1–Ps134 | Cg7–Pg1–Ps135 | Cg7–Pg1–Ps136 |
| Cg7–Pg1–Ps137 | Cg7–Pg1–Ps138 | Cg7–Pg1–Ps139 | Cg7–Pg1–Ps140 |
| Cg7–Pg1–Ps141 | Cg7–Pg1–Ps142 | Cg7–Pg1–Ps143 | Cg7–Pg1–Ps144 |
| Cg7–Pg1–Ps145 | Cg7–Pg1–Ps146 | Cg7–Pg1–Ps147 | Cg7–Pg1–Ps148 |
| Cg7–Pg1–Ps149 | Cg7–Pg1–Ps150 | Cg7–Pg1–Ps151 | Cg7–Pg1–Ps152 |
| Cg7–Pg1–Ps153 | Cg7–Pg1–Ps154 | Cg7–Pg1–Ps155 | Cg7–Pg1–Ps156 |
| Cg7–Pg1–Ps157 | Cg7–Pg1–Ps158 | Cg7–Pg1–Ps159 | Cg7–Pg1–Ps160 |
| Cg7–Pg1–Ps161 | Cg7–Pg1–Ps162 | Cg7–Pg1–Ps163 | Cg7–Pg1–Ps164 |
| Cg7–Pg1–Ps165 | Cg7–Pg1–Ps166 | Cg7–Pg1–Ps167 | Cg7–Pg1–Ps168 |
| Cg7–Pg1–Ps169 | Cg7–Pg1–Ps170 | Cg7–Pg1–Ps171 | Cg7–Pg1–Ps172 |
| Cg7–Pg1–Ps173 | Cg7–Pg1–Ps174 | Cg7–Pg1–Ps175 | Cg7–Pg1–Ps176 |
| Cg7–Pg1–Ps177 | Cg7–Pg1–Ps178 | Cg7–Pg1–Ps179 | Cg7–Pg1–Ps180 |
| Cg7–Pg1–Ps181 | Cg7–Pg1–Ps182 | Cg7–Pg1–Ps183 | Cg7–Pg1–Ps184 |
| Cg7–Pg1–Ps185 | Cg7–Pg1–Ps186 | Cg7–Pg1–Ps187 | Cg7–Pg1–Ps188 |
| Cg7–Pg1–Ps189 | Cg7–Pg1–Ps190 | Cg7–Pg1–Ps191 | Cg7–Pg1–Ps192 |
| Cg7–Pg1–Ps193 | Cg7–Pg1–Ps194 | Cg7–Pg1–Ps195 | Cg7–Pg1–Ps196 |
| Cg7–Pg1–Ps197 | Cg7–Pg1–Ps198 | Cg7–Pg1–Ps199 | Cg7–Pg1–Ps200 |
| Cg7–Pg1–Ps201 | Cg7–Pg1–Ps202 | Cg7–Pg1–Ps203 | Cg7–Pg1–Ps204 |
| Cg7–Pg1–Ps205 | Cg7–Pg1–Ps206 | Cg7–Pg1–Ps207 | Cg7–Pg1–Ps208 |
| Cg7–Pg1–Ps209 | Cg7–Pg1–Ps210 | Cg7–Pg1–Ps211 | Cg7–Pg1–Ps212 |
| Cg7–Pg1–Ps213 | Cg7–Pg1–Ps214 | Cg7–Pg1–Ps215 | Cg7–Pg1–Ps216 |
| Cg7–Pg1–Ps217 | Cg7–Pg1–Ps218 | Cg7–Pg1–Ps219 | Cg7–Pg1–Ps220 |
| Cg7–Pg1–Ps221 | Cg7–Pg1–Ps222 | Cg7–Pg1–Ps223 | Cg7–Pg1–Ps224 |
| Cg7–Pg1–Ps225 | Cg7–Pg1–Ps226 | Cg7–Pg1–Ps227 | Cg7–Pg1–Ps228 |
| Cg7–Pg1–Ps229 | Cg7–Pg1–Ps230 | Cg7–Pg1–Ps231 | Cg7–Pg1–Ps232 |
| Cg7–Pg1–Ps233 | Cg7–Pg1–Ps234 | Cg7–Pg1–Ps235 | Cg7–Pg1–Ps236 |
| Cg7–Pg1–Ps237 | Cg7–Pg1–Ps238 | Cg7–Pg1–Ps239 | Cg7–Pg1–Ps240 |
| Cg7–Pg1–Ps241 | Cg7–Pg1–Ps242 | Cg7–Pg1–Ps243 | |

| | | | |
|---|---|---|---|
| Cg18–Pg1–Ps1 | Cg18–Pg1–Ps2 | Cg18–Pg1–Ps3 | Cg18–Pg1–Ps4 |
| Cg18–Pg1–Ps5 | Cg18–Pg1–Ps6 | Cg18–Pg1–Ps7 | Cg18–Pg1–Ps8 |
| Cg18–Pg1–Ps9 | Cg18–Pg1–Ps10 | Cg18–Pg1–Ps11 | Cg18–Pg1–Ps12 |
| Cg18–Pg1–Ps13 | Cg18–Pg1–Ps14 | Cg18–Pg1–Ps15 | Cg18–Pg1–Ps16 |
| Cg18–Pg1–Ps17 | Cg18–Pg1–Ps18 | Cg18–Pg1–Ps19 | Cg18–Pg1–Ps20 |
| Cg18–Pg1–Ps21 | Cg18–Pg1–Ps22 | Cg18–Pg1–Ps23 | Cg18–Pg1–Ps24 |
| Cg18–Pg1–Ps25 | Cg18–Pg1–Ps26 | Cg18–Pg1–Ps27 | Cg18–Pg1–Ps28 |
| Cg18–Pg1–Ps29 | Cg18–Pg1–Ps30 | Cg18–Pg1–Ps31 | Cg18–Pg1–Ps32 |
| Cg18–Pg1–Ps33 | Cg18–Pg1–Ps34 | Cg18–Pg1–Ps35 | Cg18–Pg1–Ps36 |
| Cg18–Pg1–Ps37 | Cg18–Pg1–Ps38 | Cg18–Pg1–Ps39 | Cg18–Pg1–Ps40 |
| Cg18–Pg1–Ps41 | Cg18–Pg1–Ps42 | Cg18–Pg1–Ps43 | Cg18–Pg1–Ps44 |
| Cg18–Pg1–Ps45 | Cg18–Pg1–Ps46 | Cg18–Pg1–Ps47 | Cg18–Pg1–Ps48 |
| Cg18–Pg1–Ps49 | Cg18–Pg1–Ps50 | Cg18–Pg1–Ps51 | Cg18–Pg1–Ps52 |
| Cg18–Pg1–Ps53 | Cg18–Pg1–Ps54 | Cg18–Pg1–Ps55 | Cg18–Pg1–Ps56 |
| Cg18–Pg1–Ps57 | Cg18–Pg1–Ps58 | Cg18–Pg1–Ps59 | Cg18–Pg1–Ps60 |
| Cg18–Pg1–Ps61 | Cg18–Pg1–Ps62 | Cg18–Pg1–Ps63 | Cg18–Pg1–Ps64 |
| Cg18–Pg1–Ps65 | Cg18–Pg1–Ps66 | Cg18–Pg1–Ps67 | Cg18–Pg1–Ps68 |
| Cg18–Pg1–Ps69 | Cg18–Pg1–Ps70 | Cg18–Pg1–Ps71 | Cg18–Pg1–Ps72 |
| Cg18–Pg1–Ps73 | Cg18–Pg1–Ps74 | Cg18–Pg1–Ps75 | Cg18–Pg1–Ps76 |
| Cg18–Pg1–Ps77 | Cg18–Pg1–Ps78 | Cg18–Pg1–Ps79 | Cg18–Pg1–Ps80 |
| Cg18–Pg1–Ps81 | Cg18–Pg1–Ps82 | Cg18–Pg1–Ps83 | Cg18–Pg1–Ps84 |
| Cg18–Pg1–Ps85 | Cg18–Pg1–Ps86 | Cg18–Pg1–Ps87 | Cg18–Pg1–Ps88 |
| Cg18–Pg1–Ps89 | Cg18–Pg1–Ps90 | Cg18–Pg1–Ps91 | Cg18–Pg1–Ps92 |
| Cg18–Pg1–Ps93 | Cg18–Pg1–Ps94 | Cg18–Pg1–Ps95 | Cg18–Pg1–Ps96 |
| Cg18–Pg1–Ps97 | Cg18–Pg1–Ps98 | Cg18–Pg1–Ps99 | Cg18–Pg1–Ps100 |
| Cg18–Pg1–Ps101 | Cg18–Pg1–Ps102 | Cg18–Pg1–Ps103 | Cg18–Pg1–Ps104 |
| Cg18–Pg1–Ps105 | Cg18–Pg1–Ps106 | Cg18–Pg1–Ps107 | Cg18–Pg1–Ps108 |
| Cg18–Pg1–Ps109 | Cg18–Pg1–Ps110 | Cg18–Pg1–Ps111 | Cg18–Pg1–Ps112 |
| Cg18–Pg1–Ps113 | Cg18–Pg1–Ps114 | Cg18–Pg1–Ps115 | Cg18–Pg1–Ps116 |
| Cg18–Pg1–Ps117 | Cg18–Pg1–Ps118 | Cg18–Pg1–Ps119 | Cg18–Pg1–Ps120 |
| Cg18–Pg1–Ps121 | Cg18–Pg1–Ps122 | Cg18–Pg1–Ps123 | Cg18–Pg1–Ps124 |
| Cg18–Pg1–Ps125 | Cg18–Pg1–Ps126 | Cg18–Pg1–Ps127 | Cg18–Pg1–Ps128 |
| Cg18–Pg1–Ps129 | Cg18–Pg1–Ps130 | Cg18–Pg1–Ps131 | Cg18–Pg1–Ps132 |
| Cg18–Pg1–Ps133 | Cg18–Pg1–Ps134 | Cg18–Pg1–Ps135 | Cg18–Pg1–Ps136 |
| Cg18–Pg1–Ps137 | Cg18–Pg1–Ps138 | Cg18–Pg1–Ps139 | Cg18–Pg1–Ps140 |
| Cg18–Pg1–Ps141 | Cg18–Pg1–Ps142 | Cg18–Pg1–Ps143 | Cg18–Pg1–Ps144 |
| Cg18–Pg1–Ps145 | Cg18–Pg1–Ps146 | Cg18–Pg1–Ps147 | Cg18–Pg1–Ps148 |
| Cg18–Pg1–Ps149 | Cg18–Pg1–Ps150 | Cg18–Pg1–Ps151 | Cg18–Pg1–Ps152 |
| Cg18–Pg1–Ps153 | Cg18–Pg1–Ps154 | Cg18–Pg1–Ps155 | Cg18–Pg1–Ps156 |
| Cg18–Pg1–Ps157 | Cg18–Pg1–Ps158 | Cg18–Pg1–Ps159 | Cg18–Pg1–Ps160 |
| Cg18–Pg1–Ps161 | Cg18–Pg1–Ps162 | Cg18–Pg1–Ps163 | Cg18–Pg1–Ps164 |
| Cg18–Pg1–Ps165 | Cg18–Pg1–Ps166 | Cg18–Pg1–Ps167 | Cg18–Pg1–Ps168 |
| Cg18–Pg1–Ps169 | Cg18–Pg1–Ps170 | Cg18–Pg1–Ps171 | Cg18–Pg1–Ps172 |
| Cg18–Pg1–Ps173 | Cg18–Pg1–Ps174 | Cg18–Pg1–Ps175 | Cg18–Pg1–Ps176 |
| Cg18–Pg1–Ps177 | Cg18–Pg1–Ps178 | Cg18–Pg1–Ps179 | Cg18–Pg1–Ps180 |
| Cg18–Pg1–Ps181 | Cg18–Pg1–Ps182 | Cg18–Pg1–Ps183 | Cg18–Pg1–Ps184 |
| Cg18–Pg1–Ps185 | Cg18–Pg1–Ps186 | Cg18–Pg1–Ps187 | Cg18–Pg1–Ps188 |
| Cg18–Pg1–Ps189 | Cg18–Pg1–Ps190 | Cg18–Pg1–Ps191 | Cg18–Pg1–Ps192 |
| Cg18–Pg1–Ps193 | Cg18–Pg1–Ps194 | Cg18–Pg1–Ps195 | Cg18–Pg1–Ps196 |
| Cg18–Pg1–Ps197 | Cg18–Pg1–Ps198 | Cg18–Pg1–Ps199 | Cg18–Pg1–Ps200 |
| Cg18–Pg1–Ps201 | Cg18–Pg1–Ps202 | Cg18–Pg1–Ps203 | Cg18–Pg1–Ps204 |

| | | | |
|---|---|---|---|
| Cg18–Pg1–Ps205 | Cg18–Pg1–Ps206 | Cg18–Pg1–Ps207 | Cg18–Pg1–Ps208 |
| Cg18–Pg1–Ps209 | Cg18–Pg1–Ps210 | Cg18–Pg1–Ps211 | Cg18–Pg1–Ps212 |
| Cg18–Pg1–Ps213 | Cg18–Pg1–Ps214 | Cg18–Pg1–Ps215 | Cg18–Pg1–Ps216 |
| Cg18–Pg1–Ps217 | Cg18–Pg1–Ps218 | Cg18–Pg1–Ps219 | Cg18–Pg1–Ps220 |
| Cg18–Pg1–Ps221 | Cg18–Pg1–Ps222 | Cg18–Pg1–Ps223 | Cg18–Pg1–Ps224 |
| Cg18–Pg1–Ps225 | Cg18–Pg1–Ps226 | Cg18–Pg1–Ps227 | Cg18–Pg1–Ps228 |
| Cg18–Pg1–Ps229 | Cg18–Pg1–Ps230 | Cg18–Pg1–Ps231 | Cg18–Pg1–Ps232 |
| Cg18–Pg1–Ps233 | Cg18–Pg1–Ps234 | Cg18–Pg1–Ps235 | Cg18–Pg1–Ps236 |
| Cg18–Pg1–Ps237 | Cg18–Pg1–Ps238 | Cg18–Pg1–Ps239 | Cg18–Pg1–Ps240 |
| Cg18–Pg1–Ps241 | Cg18–Pg1–Ps242 | Cg18–Pg1–Ps243 | |

| | | | |
|---|---|---|---|
| Cg21–Pg1–Ps1 | Cg21–Pg1–Ps2 | Cg21–Pg1–Ps3 | Cg21–Pg1–Ps4 |
| Cg21–Pg1–Ps5 | Cg21–Pg1–Ps6 | Cg21–Pg1–Ps7 | Cg21–Pg1–Ps8 |
| Cg21–Pg1–Ps9 | Cg21–Pg1–Ps10 | Cg21–Pg1–Ps11 | Cg21–Pg1–Ps12 |
| Cg21–Pg1–Ps13 | Cg21–Pg1–Ps14 | Cg21–Pg1–Ps15 | Cg21–Pg1–Ps16 |
| Cg21–Pg1–Ps17 | Cg21–Pg1–Ps18 | Cg21–Pg1–Ps19 | Cg21–Pg1–Ps20 |
| Cg21–Pg1–Ps21 | Cg21–Pg1–Ps22 | Cg21–Pg1–Ps23 | Cg21–Pg1–Ps24 |
| Cg21–Pg1–Ps25 | Cg21–Pg1–Ps26 | Cg21–Pg1–Ps27 | Cg21–Pg1–Ps28 |
| Cg21–Pg1–Ps29 | Cg21–Pg1–Ps30 | Cg21–Pg1–Ps31 | Cg21–Pg1–Ps32 |
| Cg21–Pg1–Ps33 | Cg21–Pg1–Ps34 | Cg21–Pg1–Ps35 | Cg21–Pg1–Ps36 |
| Cg21–Pg1–Ps37 | Cg21–Pg1–Ps38 | Cg21–Pg1–Ps39 | Cg21–Pg1–Ps40 |
| Cg21–Pg1–Ps41 | Cg21–Pg1–Ps42 | Cg21–Pg1–Ps43 | Cg21–Pg1–Ps44 |
| Cg21–Pg1–Ps45 | Cg21–Pg1–Ps46 | Cg21–Pg1–Ps47 | Cg21–Pg1–Ps48 |
| Cg21–Pg1–Ps49 | Cg21–Pg1–Ps50 | Cg21–Pg1–Ps51 | Cg21–Pg1–Ps52 |
| Cg21–Pg1–Ps53 | Cg21–Pg1–Ps54 | Cg21–Pg1–Ps55 | Cg21–Pg1–Ps56 |
| Cg21–Pg1–Ps57 | Cg21–Pg1–Ps58 | Cg21–Pg1–Ps59 | Cg21–Pg1–Ps60 |
| Cg21–Pg1–Ps61 | Cg21–Pg1–Ps62 | Cg21–Pg1–Ps63 | Cg21–Pg1–Ps64 |
| Cg21–Pg1–Ps65 | Cg21–Pg1–Ps66 | Cg21–Pg1–Ps67 | Cg21–Pg1–Ps68 |
| Cg21–Pg1–Ps69 | Cg21–Pg1–Ps70 | Cg21–Pg1–Ps71 | Cg21–Pg1–Ps72 |
| Cg21–Pg1–Ps73 | Cg21–Pg1–Ps74 | Cg21–Pg1–Ps75 | Cg21–Pg1–Ps76 |
| Cg21–Pg1–Ps77 | Cg21–Pg1–Ps78 | Cg21–Pg1–Ps79 | Cg21–Pg1–Ps80 |
| Cg21–Pg1–Ps81 | Cg21–Pg1–Ps82 | Cg21–Pg1–Ps83 | Cg21–Pg1–Ps84 |
| Cg21–Pg1–Ps85 | Cg21–Pg1–Ps86 | Cg21–Pg1–Ps87 | Cg21–Pg1–Ps88 |
| Cg21–Pg1–Ps89 | Cg21–Pg1–Ps90 | Cg21–Pg1–Ps91 | Cg21–Pg1–Ps92 |
| Cg21–Pg1–Ps93 | Cg21–Pg1–Ps94 | Cg21–Pg1–Ps95 | Cg21–Pg1–Ps96 |
| Cg21–Pg1–Ps97 | Cg21–Pg1–Ps98 | Cg21–Pg1–Ps99 | Cg21–Pg1–Ps100 |
| Cg21–Pg1–Ps101 | Cg21–Pg1–Ps102 | Cg21–Pg1–Ps103 | Cg21–Pg1–Ps104 |
| Cg21–Pg1–Ps105 | Cg21–Pg1–Ps106 | Cg21–Pg1–Ps107 | Cg21–Pg1–Ps108 |
| Cg21–Pg1–Ps109 | Cg21–Pg1–Ps110 | Cg21–Pg1–Ps111 | Cg21–Pg1–Ps112 |
| Cg21–Pg1–Ps113 | Cg21–Pg1–Ps114 | Cg21–Pg1–Ps115 | Cg21–Pg1–Ps116 |
| Cg21–Pg1–Ps117 | Cg21–Pg1–Ps118 | Cg21–Pg1–Ps119 | Cg21–Pg1–Ps120 |
| Cg21–Pg1–Ps121 | Cg21–Pg1–Ps122 | Cg21–Pg1–Ps123 | Cg21–Pg1–Ps124 |
| Cg21–Pg1–Ps125 | Cg21–Pg1–Ps126 | Cg21–Pg1–Ps127 | Cg21–Pg1–Ps128 |
| Cg21–Pg1–Ps129 | Cg21–Pg1–Ps130 | Cg21–Pg1–Ps131 | Cg21–Pg1–Ps132 |
| Cg21–Pg1–Ps133 | Cg21–Pg1–Ps134 | Cg21–Pg1–Ps135 | Cg21–Pg1–Ps136 |
| Cg21–Pg1–Ps137 | Cg21–Pg1–Ps138 | Cg21–Pg1–Ps139 | Cg21–Pg1–Ps140 |
| Cg21–Pg1–Ps141 | Cg21–Pg1–Ps142 | Cg21–Pg1–Ps143 | Cg21–Pg1–Ps144 |
| Cg21–Pg1–Ps145 | Cg21–Pg1–Ps146 | Cg21–Pg1–Ps147 | Cg21–Pg1–Ps148 |
| Cg21–Pg1–Ps149 | Cg21–Pg1–Ps150 | Cg21–Pg1–Ps151 | Cg21–Pg1–Ps152 |
| Cg21–Pg1–Ps153 | Cg21–Pg1–Ps154 | Cg21–Pg1–Ps155 | Cg21–Pg1–Ps156 |
| Cg21–Pg1–Ps157 | Cg21–Pg1–Ps158 | Cg21–Pg1–Ps159 | Cg21–Pg1–Ps160 |
| Cg21–Pg1–Ps161 | Cg21–Pg1–Ps162 | Cg21–Pg1–Ps163 | Cg21–Pg1–Ps164 |

| | | | |
|---|---|---|---|
| Cg21-Pg1-Ps165 | Cg21-Pg1-Ps166 | Cg21-Pg1-Ps167 | Cg21-Pg1-Ps168 |
| Cg21-Pg1-Ps169 | Cg21-Pg1-Ps170 | Cg21-Pg1-Ps171 | Cg21-Pg1-Ps172 |
| Cg21-Pg1-Ps173 | Cg21-Pg1-Ps174 | Cg21-Pg1-Ps175 | Cg21-Pg1-Ps176 |
| Cg21-Pg1-Ps177 | Cg21-Pg1-Ps178 | Cg21-Pg1-Ps179 | Cg21-Pg1-Ps180 |
| Cg21-Pg1-Ps181 | Cg21-Pg1-Ps182 | Cg21-Pg1-Ps183 | Cg21-Pg1-Ps184 |
| Cg21-Pg1-Ps185 | Cg21-Pg1-Ps186 | Cg21-Pg1-Ps187 | Cg21-Pg1-Ps188 |
| Cg21-Pg1-Ps189 | Cg21-Pg1-Ps190 | Cg21-Pg1-Ps191 | Cg21-Pg1-Ps192 |
| Cg21-Pg1-Ps193 | Cg21-Pg1-Ps194 | Cg21-Pg1-Ps195 | Cg21-Pg1-Ps196 |
| Cg21-Pg1-Ps197 | Cg21-Pg1-Ps198 | Cg21-Pg1-Ps199 | Cg21-Pg1-Ps200 |
| Cg21-Pg1-Ps201 | Cg21-Pg1-Ps202 | Cg21-Pg1-Ps203 | Cg21-Pg1-Ps204 |
| Cg21-Pg1-Ps205 | Cg21-Pg1-Ps206 | Cg21-Pg1-Ps207 | Cg21-Pg1-Ps208 |
| Cg21-Pg1-Ps209 | Cg21-Pg1-Ps210 | Cg21-Pg1-Ps211 | Cg21-Pg1-Ps212 |
| Cg21-Pg1-Ps213 | Cg21-Pg1-Ps214 | Cg21-Pg1-Ps215 | Cg21-Pg1-Ps216 |
| Cg21-Pg1-Ps217 | Cg21-Pg1-Ps218 | Cg21-Pg1-Ps219 | Cg21-Pg1-Ps220 |
| Cg21-Pg1-Ps221 | Cg21-Pg1-Ps222 | Cg21-Pg1-Ps223 | Cg21-Pg1-Ps224 |
| Cg21-Pg1-Ps225 | Cg21-Pg1-Ps226 | Cg21-Pg1-Ps227 | Cg21-Pg1-Ps228 |
| Cg21-Pg1-Ps229 | Cg21-Pg1-Ps230 | Cg21-Pg1-Ps231 | Cg21-Pg1-Ps232 |
| Cg21-Pg1-Ps233 | Cg21-Pg1-Ps234 | Cg21-Pg1-Ps235 | Cg21-Pg1-Ps236 |
| Cg21-Pg1-Ps237 | Cg21-Pg1-Ps238 | Cg21-Pg1-Ps239 | Cg21-Pg1-Ps240 |
| Cg21-Pg1-Ps241 | Cg21-Pg1-Ps242 | Cg21-Pg1-Ps243 | |

| | | | |
|---|---|---|---|
| Cg22-Pg1-Ps1 | Cg22-Pg1-Ps2 | Cg22-Pg1-Ps3 | Cg22-Pg1-Ps4 |
| Cg22-Pg1-Ps5 | Cg22-Pg1-Ps6 | Cg22-Pg1-Ps7 | Cg22-Pg1-Ps8 |
| Cg22-Pg1-Ps9 | Cg22-Pg1-Ps10 | Cg22-Pg1-Ps11 | Cg22-Pg1-Ps12 |
| Cg22-Pg1-Ps13 | Cg22-Pg1-Ps14 | Cg22-Pg1-Ps15 | Cg22-Pg1-Ps16 |
| Cg22-Pg1-Ps17 | Cg22-Pg1-Ps18 | Cg22-Pg1-Ps19 | Cg22-Pg1-Ps20 |
| Cg22-Pg1-Ps21 | Cg22-Pg1-Ps22 | Cg22-Pg1-Ps23 | Cg22-Pg1-Ps24 |
| Cg22-Pg1-Ps25 | Cg22-Pg1-Ps26 | Cg22-Pg1-Ps27 | Cg22-Pg1-Ps28 |
| Cg22-Pg1-Ps29 | Cg22-Pg1-Ps30 | Cg22-Pg1-Ps31 | Cg22-Pg1-Ps32 |
| Cg22-Pg1-Ps33 | Cg22-Pg1-Ps34 | Cg22-Pg1-Ps35 | Cg22-Pg1-Ps36 |
| Cg22-Pg1-Ps37 | Cg22-Pg1-Ps38 | Cg22-Pg1-Ps39 | Cg22-Pg1-Ps40 |
| Cg22-Pg1-Ps41 | Cg22-Pg1-Ps42 | Cg22-Pg1-Ps43 | Cg22-Pg1-Ps44 |
| Cg22-Pg1-Ps45 | Cg22-Pg1-Ps46 | Cg22-Pg1-Ps47 | Cg22-Pg1-Ps48 |
| Cg22-Pg1-Ps49 | Cg22-Pg1-Ps50 | Cg22-Pg1-Ps51 | Cg22-Pg1-Ps52 |
| Cg22-Pg1-Ps53 | Cg22-Pg1-Ps54 | Cg22-Pg1-Ps55 | Cg22-Pg1-Ps56 |
| Cg22-Pg1-Ps57 | Cg22-Pg1-Ps58 | Cg22-Pg1-Ps59 | Cg22-Pg1-Ps60 |
| Cg22-Pg1-Ps61 | Cg22-Pg1-Ps62 | Cg22-Pg1-Ps63 | Cg22-Pg1-Ps64 |
| Cg22-Pg1-Ps65 | Cg22-Pg1-Ps66 | Cg22-Pg1-Ps67 | Cg22-Pg1-Ps68 |
| Cg22-Pg1-Ps69 | Cg22-Pg1-Ps70 | Cg22-Pg1-Ps71 | Cg22-Pg1-Ps72 |
| Cg22-Pg1-Ps73 | Cg22-Pg1-Ps74 | Cg22-Pg1-Ps75 | Cg22-Pg1-Ps76 |
| Cg22-Pg1-Ps77 | Cg22-Pg1-Ps78 | Cg22-Pg1-Ps79 | Cg22-Pg1-Ps80 |
| Cg22-Pg1-Ps81 | Cg22-Pg1-Ps82 | Cg22-Pg1-Ps83 | Cg22-Pg1-Ps84 |
| Cg22-Pg1-Ps85 | Cg22-Pg1-Ps86 | Cg22-Pg1-Ps87 | Cg22-Pg1-Ps88 |
| Cg22-Pg1-Ps89 | Cg22-Pg1-Ps90 | Cg22-Pg1-Ps91 | Cg22-Pg1-Ps92 |
| Cg22-Pg1-Ps93 | Cg22-Pg1-Ps94 | Cg22-Pg1-Ps95 | Cg22-Pg1-Ps96 |
| Cg22-Pg1-Ps97 | Cg22-Pg1-Ps98 | Cg22-Pg1-Ps99 | Cg22-Pg1-Ps100 |
| Cg22-Pg1-Ps101 | Cg22-Pg1-Ps102 | Cg22-Pg1-Ps103 | Cg22-Pg1-Ps104 |
| Cg22-Pg1-Ps105 | Cg22-Pg1-Ps106 | Cg22-Pg1-Ps107 | Cg22-Pg1-Ps108 |
| Cg22-Pg1-Ps109 | Cg22-Pg1-Ps110 | Cg22-Pg1-Ps111 | Cg22-Pg1-Ps112 |
| Cg22-Pg1-Ps113 | Cg22-Pg1-Ps114 | Cg22-Pg1-Ps115 | Cg22-Pg1-Ps116 |
| Cg22-Pg1-Ps117 | Cg22-Pg1-Ps118 | Cg22-Pg1-Ps119 | Cg22-Pg1-Ps120 |
| Cg22-Pg1-Ps121 | Cg22-Pg1-Ps122 | Cg22-Pg1-Ps123 | Cg22-Pg1-Ps124 |

| | | | |
|---|---|---|---|
| Cg22–Pg1–Ps125 | Cg22–Pg1–Ps126 | Cg22–Pg1–Ps127 | Cg22–Pg1–Ps128 |
| Cg22–Pg1–Ps129 | Cg22–Pg1–Ps130 | Cg22–Pg1–Ps131 | Cg22–Pg1–Ps132 |
| Cg22–Pg1–Ps133 | Cg22–Pg1–Ps134 | Cg22–Pg1–Ps135 | Cg22–Pg1–Ps136 |
| Cg22–Pg1–Ps137 | Cg22–Pg1–Ps138 | Cg22–Pg1–Ps139 | Cg22–Pg1–Ps140 |
| Cg22–Pg1–Ps141 | Cg22–Pg1–Ps142 | Cg22–Pg1–Ps143 | Cg22–Pg1–Ps144 |
| Cg22–Pg1–Ps145 | Cg22–Pg1–Ps146 | Cg22–Pg1–Ps147 | Cg22–Pg1–Ps148 |
| Cg22–Pg1–Ps149 | Cg22–Pg1–Ps150 | Cg22–Pg1–Ps151 | Cg22–Pg1–Ps152 |
| Cg22–Pg1–Ps153 | Cg22–Pg1–Ps154 | Cg22–Pg1–Ps155 | Cg22–Pg1–Ps156 |
| Cg22–Pg1–Ps157 | Cg22–Pg1–Ps158 | Cg22–Pg1–Ps159 | Cg22–Pg1–Ps160 |
| Cg22–Pg1–Ps161 | Cg22–Pg1–Ps162 | Cg22–Pg1–Ps163 | Cg22–Pg1–Ps164 |
| Cg22–Pg1–Ps165 | Cg22–Pg1–Ps166 | Cg22–Pg1–Ps167 | Cg22–Pg1–Ps168 |
| Cg22–Pg1–Ps169 | Cg22–Pg1–Ps170 | Cg22–Pg1–Ps171 | Cg22–Pg1–Ps172 |
| Cg22–Pg1–Ps173 | Cg22–Pg1–Ps174 | Cg22–Pg1–Ps175 | Cg22–Pg1–Ps176 |
| Cg22–Pg1–Ps177 | Cg22–Pg1–Ps178 | Cg22–Pg1–Ps179 | Cg22–Pg1–Ps180 |
| Cg22–Pg1–Ps181 | Cg22–Pg1–Ps182 | Cg22–Pg1–Ps183 | Cg22–Pg1–Ps184 |
| Cg22–Pg1–Ps185 | Cg22–Pg1–Ps186 | Cg22–Pg1–Ps187 | Cg22–Pg1–Ps188 |
| Cg22–Pg1–Ps189 | Cg22–Pg1–Ps190 | Cg22–Pg1–Ps191 | Cg22–Pg1–Ps192 |
| Cg22–Pg1–Ps193 | Cg22–Pg1–Ps194 | Cg22–Pg1–Ps195 | Cg22–Pg1–Ps196 |
| Cg22–Pg1–Ps197 | Cg22–Pg1–Ps198 | Cg22–Pg1–Ps199 | Cg22–Pg1–Ps200 |
| Cg22–Pg1–Ps201 | Cg22–Pg1–Ps202 | Cg22–Pg1–Ps203 | Cg22–Pg1–Ps204 |
| Cg22–Pg1–Ps205 | Cg22–Pg1–Ps206 | Cg22–Pg1–Ps207 | Cg22–Pg1–Ps208 |
| Cg22–Pg1–Ps209 | Cg22–Pg1–Ps210 | Cg22–Pg1–Ps211 | Cg22–Pg1–Ps212 |
| Cg22–Pg1–Ps213 | Cg22–Pg1–Ps214 | Cg22–Pg1–Ps215 | Cg22–Pg1–Ps216 |
| Cg22–Pg1–Ps217 | Cg22–Pg1–Ps218 | Cg22–Pg1–Ps219 | Cg22–Pg1–Ps220 |
| Cg22–Pg1–Ps221 | Cg22–Pg1–Ps222 | Cg22–Pg1–Ps223 | Cg22–Pg1–Ps224 |
| Cg22–Pg1–Ps225 | Cg22–Pg1–Ps226 | Cg22–Pg1–Ps227 | Cg22–Pg1–Ps228 |
| Cg22–Pg1–Ps229 | Cg22–Pg1–Ps230 | Cg22–Pg1–Ps231 | Cg22–Pg1–Ps232 |
| Cg22–Pg1–Ps233 | Cg22–Pg1–Ps234 | Cg22–Pg1–Ps235 | Cg22–Pg1–Ps236 |
| Cg22–Pg1–Ps237 | Cg22–Pg1–Ps238 | Cg22–Pg1–Ps239 | Cg22–Pg1–Ps240 |
| Cg22–Pg1–Ps241 | Cg22–Pg1–Ps242 | Cg22–Pg1–Ps243 | |

| | | | |
|---|---|---|---|
| Cg23–Pg1–Ps1 | Cg23–Pg1–Ps2 | Cg23–Pg1–Ps3 | Cg23–Pg1–Ps4 |
| Cg23–Pg1–Ps5 | Cg23–Pg1–Ps6 | Cg23–Pg1–Ps7 | Cg23–Pg1–Ps8 |
| Cg23–Pg1–Ps9 | Cg23–Pg1–Ps10 | Cg23–Pg1–Ps11 | Cg23–Pg1–Ps12 |
| Cg23–Pg1–Ps13 | Cg23–Pg1–Ps14 | Cg23–Pg1–Ps15 | Cg23–Pg1–Ps16 |
| Cg23–Pg1–Ps17 | Cg23–Pg1–Ps18 | Cg23–Pg1–Ps19 | Cg23–Pg1–Ps20 |
| Cg23–Pg1–Ps21 | Cg23–Pg1–Ps22 | Cg23–Pg1–Ps23 | Cg23–Pg1–Ps24 |
| Cg23–Pg1–Ps25 | Cg23–Pg1–Ps26 | Cg23–Pg1–Ps27 | Cg23–Pg1–Ps28 |
| Cg23–Pg1–Ps29 | Cg23–Pg1–Ps30 | Cg23–Pg1–Ps31 | Cg23–Pg1–Ps32 |
| Cg23–Pg1–Ps33 | Cg23–Pg1–Ps34 | Cg23–Pg1–Ps35 | Cg23–Pg1–Ps36 |
| Cg23–Pg1–Ps37 | Cg23–Pg1–Ps38 | Cg23–Pg1–Ps39 | Cg23–Pg1–Ps40 |
| Cg23–Pg1–Ps41 | Cg23–Pg1–Ps42 | Cg23–Pg1–Ps43 | Cg23–Pg1–Ps44 |
| Cg23–Pg1–Ps45 | Cg23–Pg1–Ps46 | Cg23–Pg1–Ps47 | Cg23–Pg1–Ps48 |
| Cg23–Pg1–Ps49 | Cg23–Pg1–Ps50 | Cg23–Pg1–Ps51 | Cg23–Pg1–Ps52 |
| Cg23–Pg1–Ps53 | Cg23–Pg1–Ps54 | Cg23–Pg1–Ps55 | Cg23–Pg1–Ps56 |
| Cg23–Pg1–Ps57 | Cg23–Pg1–Ps58 | Cg23–Pg1–Ps59 | Cg23–Pg1–Ps60 |
| Cg23–Pg1–Ps61 | Cg23–Pg1–Ps62 | Cg23–Pg1–Ps63 | Cg23–Pg1–Ps64 |
| Cg23–Pg1–Ps65 | Cg23–Pg1–Ps66 | Cg23–Pg1–Ps67 | Cg23–Pg1–Ps68 |
| Cg23–Pg1–Ps69 | Cg23–Pg1–Ps70 | Cg23–Pg1–Ps71 | Cg23–Pg1–Ps72 |
| Cg23–Pg1–Ps73 | Cg23–Pg1–Ps74 | Cg23–Pg1–Ps75 | Cg23–Pg1–Ps76 |
| Cg23–Pg1–Ps77 | Cg23–Pg1–Ps78 | Cg23–Pg1–Ps79 | Cg23–Pg1–Ps80 |
| Cg23–Pg1–Ps81 | Cg23–Pg1–Ps82 | Cg23–Pg1–Ps83 | Cg23–Pg1–Ps84 |

| | | | |
|---|---|---|---|
| Cg23–Pg1–Ps85 | Cg23–Pg1–Ps86 | Cg23–Pg1–Ps87 | Cg23–Pg1–Ps88 |
| Cg23–Pg1–Ps89 | Cg23–Pg1–Ps90 | Cg23–Pg1–Ps91 | Cg23–Pg1–Ps92 |
| Cg23–Pg1–Ps93 | Cg23–Pg1–Ps94 | Cg23–Pg1–Ps95 | Cg23–Pg1–Ps96 |
| Cg23–Pg1–Ps97 | Cg23–Pg1–Ps98 | Cg23–Pg1–Ps99 | Cg23–Pg1–Ps100 |
| Cg23–Pg1–Ps101 | Cg23–Pg1–Ps102 | Cg23–Pg1–Ps103 | Cg23–Pg1–Ps104 |
| Cg23–Pg1–Ps105 | Cg23–Pg1–Ps106 | Cg23–Pg1–Ps107 | Cg23–Pg1–Ps108 |
| Cg23–Pg1–Ps109 | Cg23–Pg1–Ps110 | Cg23–Pg1–Ps111 | Cg23–Pg1–Ps112 |
| Cg23–Pg1–Ps113 | Cg23–Pg1–Ps114 | Cg23–Pg1–Ps115 | Cg23–Pg1–Ps116 |
| Cg23–Pg1–Ps117 | Cg23–Pg1–Ps118 | Cg23–Pg1–Ps119 | Cg23–Pg1–Ps120 |
| Cg23–Pg1–Ps121 | Cg23–Pg1–Ps122 | Cg23–Pg1–Ps123 | Cg23–Pg1–Ps124 |
| Cg23–Pg1–Ps125 | Cg23–Pg1–Ps126 | Cg23–Pg1–Ps127 | Cg23–Pg1–Ps128 |
| Cg23–Pg1–Ps129 | Cg23–Pg1–Ps130 | Cg23–Pg1–Ps131 | Cg23–Pg1–Ps132 |
| Cg23–Pg1–Ps133 | Cg23–Pg1–Ps134 | Cg23–Pg1–Ps135 | Cg23–Pg1–Ps136 |
| Cg23–Pg1–Ps137 | Cg23–Pg1–Ps138 | Cg23–Pg1–Ps139 | Cg23–Pg1–Ps140 |
| Cg23–Pg1–Ps141 | Cg23–Pg1–Ps142 | Cg23–Pg1–Ps143 | Cg23–Pg1–Ps144 |
| Cg23–Pg1–Ps145 | Cg23–Pg1–Ps146 | Cg23–Pg1–Ps147 | Cg23–Pg1–Ps148 |
| Cg23–Pg1–Ps149 | Cg23–Pg1–Ps150 | Cg23–Pg1–Ps151 | Cg23–Pg1–Ps152 |
| Cg23–Pg1–Ps153 | Cg23–Pg1–Ps154 | Cg23–Pg1–Ps155 | Cg23–Pg1–Ps156 |
| Cg23–Pg1–Ps157 | Cg23–Pg1–Ps158 | Cg23–Pg1–Ps159 | Cg23–Pg1–Ps160 |
| Cg23–Pg1–Ps161 | Cg23–Pg1–Ps162 | Cg23–Pg1–Ps163 | Cg23–Pg1–Ps164 |
| Cg23–Pg1–Ps165 | Cg23–Pg1–Ps166 | Cg23–Pg1–Ps167 | Cg23–Pg1–Ps168 |
| Cg23–Pg1–Ps169 | Cg23–Pg1–Ps170 | Cg23–Pg1–Ps171 | Cg23–Pg1–Ps172 |
| Cg23–Pg1–Ps173 | Cg23–Pg1–Ps174 | Cg23–Pg1–Ps175 | Cg23–Pg1–Ps176 |
| Cg23–Pg1–Ps177 | Cg23–Pg1–Ps178 | Cg23–Pg1–Ps179 | Cg23–Pg1–Ps180 |
| Cg23–Pg1–Ps181 | Cg23–Pg1–Ps182 | Cg23–Pg1–Ps183 | Cg23–Pg1–Ps184 |
| Cg23–Pg1–Ps185 | Cg23–Pg1–Ps186 | Cg23–Pg1–Ps187 | Cg23–Pg1–Ps188 |
| Cg23–Pg1–Ps189 | Cg23–Pg1–Ps190 | Cg23–Pg1–Ps191 | Cg23–Pg1–Ps192 |
| Cg23–Pg1–Ps193 | Cg23–Pg1–Ps194 | Cg23–Pg1–Ps195 | Cg23–Pg1–Ps196 |
| Cg23–Pg1–Ps197 | Cg23–Pg1–Ps198 | Cg23–Pg1–Ps199 | Cg23–Pg1–Ps200 |
| Cg23–Pg1–Ps201 | Cg23–Pg1–Ps202 | Cg23–Pg1–Ps203 | Cg23–Pg1–Ps204 |
| Cg23–Pg1–Ps205 | Cg23–Pg1–Ps206 | Cg23–Pg1–Ps207 | Cg23–Pg1–Ps208 |
| Cg23–Pg1–Ps209 | Cg23–Pg1–Ps210 | Cg23–Pg1–Ps211 | Cg23–Pg1–Ps212 |
| Cg23–Pg1–Ps213 | Cg23–Pg1–Ps214 | Cg23–Pg1–Ps215 | Cg23–Pg1–Ps216 |
| Cg23–Pg1–Ps217 | Cg23–Pg1–Ps218 | Cg23–Pg1–Ps219 | Cg23–Pg1–Ps220 |
| Cg23–Pg1–Ps221 | Cg23–Pg1–Ps222 | Cg23–Pg1–Ps223 | Cg23–Pg1–Ps224 |
| Cg23–Pg1–Ps225 | Cg23–Pg1–Ps226 | Cg23–Pg1–Ps227 | Cg23–Pg1–Ps228 |
| Cg23–Pg1–Ps229 | Cg23–Pg1–Ps230 | Cg23–Pg1–Ps231 | Cg23–Pg1–Ps232 |
| Cg23–Pg1–Ps233 | Cg23–Pg1–Ps234 | Cg23–Pg1–Ps235 | Cg23–Pg1–Ps236 |
| Cg23–Pg1–Ps237 | Cg23–Pg1–Ps238 | Cg23–Pg1–Ps239 | Cg23–Pg1–Ps240 |
| Cg23–Pg1–Ps241 | Cg23–Pg1–Ps242 | Cg23–Pg1–Ps243 | |

| | | | |
|---|---|---|---|
| Cg24–Pg1–Ps1 | Cg24–Pg1–Ps2 | Cg24–Pg1–Ps3 | Cg24–Pg1–Ps4 |
| Cg24–Pg1–Ps5 | Cg24–Pg1–Ps6 | Cg24–Pg1–Ps7 | Cg24–Pg1–Ps8 |
| Cg24–Pg1–Ps9 | Cg24–Pg1–Ps10 | Cg24–Pg1–Ps11 | Cg24–Pg1–Ps12 |
| Cg24–Pg1–Ps13 | Cg24–Pg1–Ps14 | Cg24–Pg1–Ps15 | Cg24–Pg1–Ps16 |
| Cg24–Pg1–Ps17 | Cg24–Pg1–Ps18 | Cg24–Pg1–Ps19 | Cg24–Pg1–Ps20 |
| Cg24–Pg1–Ps21 | Cg24–Pg1–Ps22 | Cg24–Pg1–Ps23 | Cg24–Pg1–Ps24 |
| Cg24–Pg1–Ps25 | Cg24–Pg1–Ps26 | Cg24–Pg1–Ps27 | Cg24–Pg1–Ps28 |
| Cg24–Pg1–Ps29 | Cg24–Pg1–Ps30 | Cg24–Pg1–Ps31 | Cg24–Pg1–Ps32 |
| Cg24–Pg1–Ps33 | Cg24–Pg1–Ps34 | Cg24–Pg1–Ps35 | Cg24–Pg1–Ps36 |
| Cg24–Pg1–Ps37 | Cg24–Pg1–Ps38 | Cg24–Pg1–Ps39 | Cg24–Pg1–Ps40 |
| Cg24–Pg1–Ps41 | Cg24–Pg1–Ps42 | Cg24–Pg1–Ps43 | Cg24–Pg1–Ps44 |

| | | | |
|---|---|---|---|
| Cg24–Pg1–Ps45 | Cg24–Pg1–Ps46 | Cg24–Pg1–Ps47 | Cg24–Pg1–Ps48 |
| Cg24–Pg1–Ps49 | Cg24–Pg1–Ps50 | Cg24–Pg1–Ps51 | Cg24–Pg1–Ps52 |
| Cg24–Pg1–Ps53 | Cg24–Pg1–Ps54 | Cg24–Pg1–Ps55 | Cg24–Pg1–Ps56 |
| Cg24–Pg1–Ps57 | Cg24–Pg1–Ps58 | Cg24–Pg1–Ps59 | Cg24–Pg1–Ps60 |
| Cg24–Pg1–Ps61 | Cg24–Pg1–Ps62 | Cg24–Pg1–Ps63 | Cg24–Pg1–Ps64 |
| Cg24–Pg1–Ps65 | Cg24–Pg1–Ps66 | Cg24–Pg1–Ps67 | Cg24–Pg1–Ps68 |
| Cg24–Pg1–Ps69 | Cg24–Pg1–Ps70 | Cg24–Pg1–Ps71 | Cg24–Pg1–Ps72 |
| Cg24–Pg1–Ps73 | Cg24–Pg1–Ps74 | Cg24–Pg1–Ps75 | Cg24–Pg1–Ps76 |
| Cg24–Pg1–Ps77 | Cg24–Pg1–Ps78 | Cg24–Pg1–Ps79 | Cg24–Pg1–Ps80 |
| Cg24–Pg1–Ps81 | Cg24–Pg1–Ps82 | Cg24–Pg1–Ps83 | Cg24–Pg1–Ps84 |
| Cg24–Pg1–Ps85 | Cg24–Pg1–Ps86 | Cg24–Pg1–Ps87 | Cg24–Pg1–Ps88 |
| Cg24–Pg1–Ps89 | Cg24–Pg1–Ps90 | Cg24–Pg1–Ps91 | Cg24–Pg1–Ps92 |
| Cg24–Pg1–Ps93 | Cg24–Pg1–Ps94 | Cg24–Pg1–Ps95 | Cg24–Pg1–Ps96 |
| Cg24–Pg1–Ps97 | Cg24–Pg1–Ps98 | Cg24–Pg1–Ps99 | Cg24–Pg1–Ps100 |
| Cg24–Pg1–Ps101 | Cg24–Pg1–Ps102 | Cg24–Pg1–Ps103 | Cg24–Pg1–Ps104 |
| Cg24–Pg1–Ps105 | Cg24–Pg1–Ps106 | Cg24–Pg1–Ps107 | Cg24–Pg1–Ps108 |
| Cg24–Pg1–Ps109 | Cg24–Pg1–Ps110 | Cg24–Pg1–Ps111 | Cg24–Pg1–Ps112 |
| Cg24–Pg1–Ps113 | Cg24–Pg1–Ps114 | Cg24–Pg1–Ps115 | Cg24–Pg1–Ps116 |
| Cg24–Pg1–Ps117 | Cg24–Pg1–Ps118 | Cg24–Pg1–Ps119 | Cg24–Pg1–Ps120 |
| Cg24–Pg1–Ps121 | Cg24–Pg1–Ps122 | Cg24–Pg1–Ps123 | Cg24–Pg1–Ps124 |
| Cg24–Pg1–Ps125 | Cg24–Pg1–Ps126 | Cg24–Pg1–Ps127 | Cg24–Pg1–Ps128 |
| Cg24–Pg1–Ps129 | Cg24–Pg1–Ps130 | Cg24–Pg1–Ps131 | Cg24–Pg1–Ps132 |
| Cg24–Pg1–Ps133 | Cg24–Pg1–Ps134 | Cg24–Pg1–Ps135 | Cg24–Pg1–Ps136 |
| Cg24–Pg1–Ps137 | Cg24–Pg1–Ps138 | Cg24–Pg1–Ps139 | Cg24–Pg1–Ps140 |
| Cg24–Pg1–Ps141 | Cg24–Pg1–Ps142 | Cg24–Pg1–Ps143 | Cg24–Pg1–Ps144 |
| Cg24–Pg1–Ps145 | Cg24–Pg1–Ps146 | Cg24–Pg1–Ps147 | Cg24–Pg1–Ps148 |
| Cg24–Pg1–Ps149 | Cg24–Pg1–Ps150 | Cg24–Pg1–Ps151 | Cg24–Pg1–Ps152 |
| Cg24–Pg1–Ps153 | Cg24–Pg1–Ps154 | Cg24–Pg1–Ps155 | Cg24–Pg1–Ps156 |
| Cg24–Pg1–Ps157 | Cg24–Pg1–Ps158 | Cg24–Pg1–Ps159 | Cg24–Pg1–Ps160 |
| Cg24–Pg1–Ps161 | Cg24–Pg1–Ps162 | Cg24–Pg1–Ps163 | Cg24–Pg1–Ps164 |
| Cg24–Pg1–Ps165 | Cg24–Pg1–Ps166 | Cg24–Pg1–Ps167 | Cg24–Pg1–Ps168 |
| Cg24–Pg1–Ps169 | Cg24–Pg1–Ps170 | Cg24–Pg1–Ps171 | Cg24–Pg1–Ps172 |
| Cg24–Pg1–Ps173 | Cg24–Pg1–Ps174 | Cg24–Pg1–Ps175 | Cg24–Pg1–Ps176 |
| Cg24–Pg1–Ps177 | Cg24–Pg1–Ps178 | Cg24–Pg1–Ps179 | Cg24–Pg1–Ps180 |
| Cg24–Pg1–Ps181 | Cg24–Pg1–Ps182 | Cg24–Pg1–Ps183 | Cg24–Pg1–Ps184 |
| Cg24–Pg1–Ps185 | Cg24–Pg1–Ps186 | Cg24–Pg1–Ps187 | Cg24–Pg1–Ps188 |
| Cg24–Pg1–Ps189 | Cg24–Pg1–Ps190 | Cg24–Pg1–Ps191 | Cg24–Pg1–Ps192 |
| Cg24–Pg1–Ps193 | Cg24–Pg1–Ps194 | Cg24–Pg1–Ps195 | Cg24–Pg1–Ps196 |
| Cg24–Pg1–Ps197 | Cg24–Pg1–Ps198 | Cg24–Pg1–Ps199 | Cg24–Pg1–Ps200 |
| Cg24–Pg1–Ps201 | Cg24–Pg1–Ps202 | Cg24–Pg1–Ps203 | Cg24–Pg1–Ps204 |
| Cg24–Pg1–Ps205 | Cg24–Pg1–Ps206 | Cg24–Pg1–Ps207 | Cg24–Pg1–Ps208 |
| Cg24–Pg1–Ps209 | Cg24–Pg1–Ps210 | Cg24–Pg1–Ps211 | Cg24–Pg1–Ps212 |
| Cg24–Pg1–Ps213 | Cg24–Pg1–Ps214 | Cg24–Pg1–Ps215 | Cg24–Pg1–Ps216 |
| Cg24–Pg1–Ps217 | Cg24–Pg1–Ps218 | Cg24–Pg1–Ps219 | Cg24–Pg1–Ps220 |
| Cg24–Pg1–Ps221 | Cg24–Pg1–Ps222 | Cg24–Pg1–Ps223 | Cg24–Pg1–Ps224 |
| Cg24–Pg1–Ps225 | Cg24–Pg1–Ps226 | Cg24–Pg1–Ps227 | Cg24–Pg1–Ps228 |
| Cg24–Pg1–Ps229 | Cg24–Pg1–Ps230 | Cg24–Pg1–Ps231 | Cg24–Pg1–Ps232 |
| Cg24–Pg1–Ps233 | Cg24–Pg1–Ps234 | Cg24–Pg1–Ps235 | Cg24–Pg1–Ps236 |
| Cg24–Pg1–Ps237 | Cg24–Pg1–Ps238 | Cg24–Pg1–Ps239 | Cg24–Pg1–Ps240 |
| Cg24–Pg1–Ps241 | Cg24–Pg1–Ps242 | Cg24–Pg1–Ps243 | |

| | | | |
|---|---|---|---|
| Cg25–Pg1–Ps1 | Cg25–Pg1–Ps2 | Cg25–Pg1–Ps3 | Cg25–Pg1–Ps4 |

| | | | |
|---|---|---|---|
| Cg25–Pg1–Ps5 | Cg25–Pg1–Ps6 | Cg25–Pg1–Ps7 | Cg25–Pg1–Ps8 |
| Cg25–Pg1–Ps9 | Cg25–Pg1–Ps10 | Cg25–Pg1–Ps11 | Cg25–Pg1–Ps12 |
| Cg25–Pg1–Ps13 | Cg25–Pg1–Ps14 | Cg25–Pg1–Ps15 | Cg25–Pg1–Ps16 |
| Cg25–Pg1–Ps17 | Cg25–Pg1–Ps18 | Cg25–Pg1–Ps19 | Cg25–Pg1–Ps20 |
| Cg25–Pg1–Ps21 | Cg25–Pg1–Ps22 | Cg25–Pg1–Ps23 | Cg25–Pg1–Ps24 |
| Cg25–Pg1–Ps25 | Cg25–Pg1–Ps26 | Cg25–Pg1–Ps27 | Cg25–Pg1–Ps28 |
| Cg25–Pg1–Ps29 | Cg25–Pg1–Ps30 | Cg25–Pg1–Ps31 | Cg25–Pg1–Ps32 |
| Cg25–Pg1–Ps33 | Cg25–Pg1–Ps34 | Cg25–Pg1–Ps35 | Cg25–Pg1–Ps36 |
| Cg25–Pg1–Ps37 | Cg25–Pg1–Ps38 | Cg25–Pg1–Ps39 | Cg25–Pg1–Ps40 |
| Cg25–Pg1–Ps41 | Cg25–Pg1–Ps42 | Cg25–Pg1–Ps43 | Cg25–Pg1–Ps44 |
| Cg25–Pg1–Ps45 | Cg25–Pg1–Ps46 | Cg25–Pg1–Ps47 | Cg25–Pg1–Ps48 |
| Cg25–Pg1–Ps49 | Cg25–Pg1–Ps50 | Cg25–Pg1–Ps51 | Cg25–Pg1–Ps52 |
| Cg25–Pg1–Ps53 | Cg25–Pg1–Ps54 | Cg25–Pg1–Ps55 | Cg25–Pg1–Ps56 |
| Cg25–Pg1–Ps57 | Cg25–Pg1–Ps58 | Cg25–Pg1–Ps59 | Cg25–Pg1–Ps60 |
| Cg25–Pg1–Ps61 | Cg25–Pg1–Ps62 | Cg25–Pg1–Ps63 | Cg25–Pg1–Ps64 |
| Cg25–Pg1–Ps65 | Cg25–Pg1–Ps66 | Cg25–Pg1–Ps67 | Cg25–Pg1–Ps68 |
| Cg25–Pg1–Ps69 | Cg25–Pg1–Ps70 | Cg25–Pg1–Ps71 | Cg25–Pg1–Ps72 |
| Cg25–Pg1–Ps73 | Cg25–Pg1–Ps74 | Cg25–Pg1–Ps75 | Cg25–Pg1–Ps76 |
| Cg25–Pg1–Ps77 | Cg25–Pg1–Ps78 | Cg25–Pg1–Ps79 | Cg25–Pg1–Ps80 |
| Cg25–Pg1–Ps81 | Cg25–Pg1–Ps82 | Cg25–Pg1–Ps83 | Cg25–Pg1–Ps84 |
| Cg25–Pg1–Ps85 | Cg25–Pg1–Ps86 | Cg25–Pg1–Ps87 | Cg25–Pg1–Ps88 |
| Cg25–Pg1–Ps89 | Cg25–Pg1–Ps90 | Cg25–Pg1–Ps91 | Cg25–Pg1–Ps92 |
| Cg25–Pg1–Ps93 | Cg25–Pg1–Ps94 | Cg25–Pg1–Ps95 | Cg25–Pg1–Ps96 |
| Cg25–Pg1–Ps97 | Cg25–Pg1–Ps98 | Cg25–Pg1–Ps99 | Cg25–Pg1–Ps100 |
| Cg25–Pg1–Ps101 | Cg25–Pg1–Ps102 | Cg25–Pg1–Ps103 | Cg25–Pg1–Ps104 |
| Cg25–Pg1–Ps105 | Cg25–Pg1–Ps106 | Cg25–Pg1–Ps107 | Cg25–Pg1–Ps108 |
| Cg25–Pg1–Ps109 | Cg25–Pg1–Ps110 | Cg25–Pg1–Ps111 | Cg25–Pg1–Ps112 |
| Cg25–Pg1–Ps113 | Cg25–Pg1–Ps114 | Cg25–Pg1–Ps115 | Cg25–Pg1–Ps116 |
| Cg25–Pg1–Ps117 | Cg25–Pg1–Ps118 | Cg25–Pg1–Ps119 | Cg25–Pg1–Ps120 |
| Cg25–Pg1–Ps121 | Cg25–Pg1–Ps122 | Cg25–Pg1–Ps123 | Cg25–Pg1–Ps124 |
| Cg25–Pg1–Ps125 | Cg25–Pg1–Ps126 | Cg25–Pg1–Ps127 | Cg25–Pg1–Ps128 |
| Cg25–Pg1–Ps129 | Cg25–Pg1–Ps130 | Cg25–Pg1–Ps131 | Cg25–Pg1–Ps132 |
| Cg25–Pg1–Ps133 | Cg25–Pg1–Ps134 | Cg25–Pg1–Ps135 | Cg25–Pg1–Ps136 |
| Cg25–Pg1–Ps137 | Cg25–Pg1–Ps138 | Cg25–Pg1–Ps139 | Cg25–Pg1–Ps140 |
| Cg25–Pg1–Ps141 | Cg25–Pg1–Ps142 | Cg25–Pg1–Ps143 | Cg25–Pg1–Ps144 |
| Cg25–Pg1–Ps145 | Cg25–Pg1–Ps146 | Cg25–Pg1–Ps147 | Cg25–Pg1–Ps148 |
| Cg25–Pg1–Ps149 | Cg25–Pg1–Ps150 | Cg25–Pg1–Ps151 | Cg25–Pg1–Ps152 |
| Cg25–Pg1–Ps153 | Cg25–Pg1–Ps154 | Cg25–Pg1–Ps155 | Cg25–Pg1–Ps156 |
| Cg25–Pg1–Ps157 | Cg25–Pg1–Ps158 | Cg25–Pg1–Ps159 | Cg25–Pg1–Ps160 |
| Cg25–Pg1–Ps161 | Cg25–Pg1–Ps162 | Cg25–Pg1–Ps163 | Cg25–Pg1–Ps164 |
| Cg25–Pg1–Ps165 | Cg25–Pg1–Ps166 | Cg25–Pg1–Ps167 | Cg25–Pg1–Ps168 |
| Cg25–Pg1–Ps169 | Cg25–Pg1–Ps170 | Cg25–Pg1–Ps171 | Cg25–Pg1–Ps172 |
| Cg25–Pg1–Ps173 | Cg25–Pg1–Ps174 | Cg25–Pg1–Ps175 | Cg25–Pg1–Ps176 |
| Cg25–Pg1–Ps177 | Cg25–Pg1–Ps178 | Cg25–Pg1–Ps179 | Cg25–Pg1–Ps180 |
| Cg25–Pg1–Ps181 | Cg25–Pg1–Ps182 | Cg25–Pg1–Ps183 | Cg25–Pg1–Ps184 |
| Cg25–Pg1–Ps185 | Cg25–Pg1–Ps186 | Cg25–Pg1–Ps187 | Cg25–Pg1–Ps188 |
| Cg25–Pg1–Ps189 | Cg25–Pg1–Ps190 | Cg25–Pg1–Ps191 | Cg25–Pg1–Ps192 |
| Cg25–Pg1–Ps193 | Cg25–Pg1–Ps194 | Cg25–Pg1–Ps195 | Cg25–Pg1–Ps196 |
| Cg25–Pg1–Ps197 | Cg25–Pg1–Ps198 | Cg25–Pg1–Ps199 | Cg25–Pg1–Ps200 |
| Cg25–Pg1–Ps201 | Cg25–Pg1–Ps202 | Cg25–Pg1–Ps203 | Cg25–Pg1–Ps204 |
| Cg25–Pg1–Ps205 | Cg25–Pg1–Ps206 | Cg25–Pg1–Ps207 | Cg25–Pg1–Ps208 |
| Cg25–Pg1–Ps209 | Cg25–Pg1–Ps210 | Cg25–Pg1–Ps211 | Cg25–Pg1–Ps212 |

| | | | |
|---|---|---|---|
| Cg25–Pg1–Ps213 | Cg25–Pg1–Ps214 | Cg25–Pg1–Ps215 | Cg25–Pg1–Ps216 |
| Cg25–Pg1–Ps217 | Cg25–Pg1–Ps218 | Cg25–Pg1–Ps219 | Cg25–Pg1–Ps220 |
| Cg25–Pg1–Ps221 | Cg25–Pg1–Ps222 | Cg25–Pg1–Ps223 | Cg25–Pg1–Ps224 |
| Cg25–Pg1–Ps225 | Cg25–Pg1–Ps226 | Cg25–Pg1–Ps227 | Cg25–Pg1–Ps228 |
| Cg25–Pg1–Ps229 | Cg25–Pg1–Ps230 | Cg25–Pg1–Ps231 | Cg25–Pg1–Ps232 |
| Cg25–Pg1–Ps233 | Cg25–Pg1–Ps234 | Cg25–Pg1–Ps235 | Cg25–Pg1–Ps236 |
| Cg25–Pg1–Ps237 | Cg25–Pg1–Ps238 | Cg25–Pg1–Ps239 | Cg25–Pg1–Ps240 |
| Cg25–Pg1–Ps241 | Cg25–Pg1–Ps242 | Cg25–Pg1–Ps243 | |

| | | | |
|---|---|---|---|
| Cg26–Pg1–Ps1 | Cg26–Pg1–Ps2 | Cg26–Pg1–Ps3 | Cg26–Pg1–Ps4 |
| Cg26–Pg1–Ps5 | Cg26–Pg1–Ps6 | Cg26–Pg1–Ps7 | Cg26–Pg1–Ps8 |
| Cg26–Pg1–Ps9 | Cg26–Pg1–Ps10 | Cg26–Pg1–Ps11 | Cg26–Pg1–Ps12 |
| Cg26–Pg1–Ps13 | Cg26–Pg1–Ps14 | Cg26–Pg1–Ps15 | Cg26–Pg1–Ps16 |
| Cg26–Pg1–Ps17 | Cg26–Pg1–Ps18 | Cg26–Pg1–Ps19 | Cg26–Pg1–Ps20 |
| Cg26–Pg1–Ps21 | Cg26–Pg1–Ps22 | Cg26–Pg1–Ps23 | Cg26–Pg1–Ps24 |
| Cg26–Pg1–Ps25 | Cg26–Pg1–Ps26 | Cg26–Pg1–Ps27 | Cg26–Pg1–Ps28 |
| Cg26–Pg1–Ps29 | Cg26–Pg1–Ps30 | Cg26–Pg1–Ps31 | Cg26–Pg1–Ps32 |
| Cg26–Pg1–Ps33 | Cg26–Pg1–Ps34 | Cg26–Pg1–Ps35 | Cg26–Pg1–Ps36 |
| Cg26–Pg1–Ps37 | Cg26–Pg1–Ps38 | Cg26–Pg1–Ps39 | Cg26–Pg1–Ps40 |
| Cg26–Pg1–Ps41 | Cg26–Pg1–Ps42 | Cg26–Pg1–Ps43 | Cg26–Pg1–Ps44 |
| Cg26–Pg1–Ps45 | Cg26–Pg1–Ps46 | Cg26–Pg1–Ps47 | Cg26–Pg1–Ps48 |
| Cg26–Pg1–Ps49 | Cg26–Pg1–Ps50 | Cg26–Pg1–Ps51 | Cg26–Pg1–Ps52 |
| Cg26–Pg1–Ps53 | Cg26–Pg1–Ps54 | Cg26–Pg1–Ps55 | Cg26–Pg1–Ps56 |
| Cg26–Pg1–Ps57 | Cg26–Pg1–Ps58 | Cg26–Pg1–Ps59 | Cg26–Pg1–Ps60 |
| Cg26–Pg1–Ps61 | Cg26–Pg1–Ps62 | Cg26–Pg1–Ps63 | Cg26–Pg1–Ps64 |
| Cg26–Pg1–Ps65 | Cg26–Pg1–Ps66 | Cg26–Pg1–Ps67 | Cg26–Pg1–Ps68 |
| Cg26–Pg1–Ps69 | Cg26–Pg1–Ps70 | Cg26–Pg1–Ps71 | Cg26–Pg1–Ps72 |
| Cg26–Pg1–Ps73 | Cg26–Pg1–Ps74 | Cg26–Pg1–Ps75 | Cg26–Pg1–Ps76 |
| Cg26–Pg1–Ps77 | Cg26–Pg1–Ps78 | Cg26–Pg1–Ps79 | Cg26–Pg1–Ps80 |
| Cg26–Pg1–Ps81 | Cg26–Pg1–Ps82 | Cg26–Pg1–Ps83 | Cg26–Pg1–Ps84 |
| Cg26–Pg1–Ps85 | Cg26–Pg1–Ps86 | Cg26–Pg1–Ps87 | Cg26–Pg1–Ps88 |
| Cg26–Pg1–Ps89 | Cg26–Pg1–Ps90 | Cg26–Pg1–Ps91 | Cg26–Pg1–Ps92 |
| Cg26–Pg1–Ps93 | Cg26–Pg1–Ps94 | Cg26–Pg1–Ps95 | Cg26–Pg1–Ps96 |
| Cg26–Pg1–Ps97 | Cg26–Pg1–Ps98 | Cg26–Pg1–Ps99 | Cg26–Pg1–Ps100 |
| Cg26–Pg1–Ps101 | Cg26–Pg1–Ps102 | Cg26–Pg1–Ps103 | Cg26–Pg1–Ps104 |
| Cg26–Pg1–Ps105 | Cg26–Pg1–Ps106 | Cg26–Pg1–Ps107 | Cg26–Pg1–Ps108 |
| Cg26–Pg1–Ps109 | Cg26–Pg1–Ps110 | Cg26–Pg1–Ps111 | Cg26–Pg1–Ps112 |
| Cg26–Pg1–Ps113 | Cg26–Pg1–Ps114 | Cg26–Pg1–Ps115 | Cg26–Pg1–Ps116 |
| Cg26–Pg1–Ps117 | Cg26–Pg1–Ps118 | Cg26–Pg1–Ps119 | Cg26–Pg1–Ps120 |
| Cg26–Pg1–Ps121 | Cg26–Pg1–Ps122 | Cg26–Pg1–Ps123 | Cg26–Pg1–Ps124 |
| Cg26–Pg1–Ps125 | Cg26–Pg1–Ps126 | Cg26–Pg1–Ps127 | Cg26–Pg1–Ps128 |
| Cg26–Pg1–Ps129 | Cg26–Pg1–Ps130 | Cg26–Pg1–Ps131 | Cg26–Pg1–Ps132 |
| Cg26–Pg1–Ps133 | Cg26–Pg1–Ps134 | Cg26–Pg1–Ps135 | Cg26–Pg1–Ps136 |
| Cg26–Pg1–Ps137 | Cg26–Pg1–Ps138 | Cg26–Pg1–Ps139 | Cg26–Pg1–Ps140 |
| Cg26–Pg1–Ps141 | Cg26–Pg1–Ps142 | Cg26–Pg1–Ps143 | Cg26–Pg1–Ps144 |
| Cg26–Pg1–Ps145 | Cg26–Pg1–Ps146 | Cg26–Pg1–Ps147 | Cg26–Pg1–Ps148 |
| Cg26–Pg1–Ps149 | Cg26–Pg1–Ps150 | Cg26–Pg1–Ps151 | Cg26–Pg1–Ps152 |
| Cg26–Pg1–Ps153 | Cg26–Pg1–Ps154 | Cg26–Pg1–Ps155 | Cg26–Pg1–Ps156 |
| Cg26–Pg1–Ps157 | Cg26–Pg1–Ps158 | Cg26–Pg1–Ps159 | Cg26–Pg1–Ps160 |
| Cg26–Pg1–Ps161 | Cg26–Pg1–Ps162 | Cg26–Pg1–Ps163 | Cg26–Pg1–Ps164 |
| Cg26–Pg1–Ps165 | Cg26–Pg1–Ps166 | Cg26–Pg1–Ps167 | Cg26–Pg1–Ps168 |
| Cg26–Pg1–Ps169 | Cg26–Pg1–Ps170 | Cg26–Pg1–Ps171 | Cg26–Pg1–Ps172 |

| | | | |
|---|---|---|---|
| Cg26–Pg1–Ps173 | Cg26–Pg1–Ps174 | Cg26–Pg1–Ps175 | Cg26–Pg1–Ps176 |
| Cg26–Pg1–Ps177 | Cg26–Pg1–Ps178 | Cg26–Pg1–Ps179 | Cg26–Pg1–Ps180 |
| Cg26–Pg1–Ps181 | Cg26–Pg1–Ps182 | Cg26–Pg1–Ps183 | Cg26–Pg1–Ps184 |
| Cg26–Pg1–Ps185 | Cg26–Pg1–Ps186 | Cg26–Pg1–Ps187 | Cg26–Pg1–Ps188 |
| Cg26–Pg1–Ps189 | Cg26–Pg1–Ps190 | Cg26–Pg1–Ps191 | Cg26–Pg1–Ps192 |
| Cg26–Pg1–Ps193 | Cg26–Pg1–Ps194 | Cg26–Pg1–Ps195 | Cg26–Pg1–Ps196 |
| Cg26–Pg1–Ps197 | Cg26–Pg1–Ps198 | Cg26–Pg1–Ps199 | Cg26–Pg1–Ps200 |
| Cg26–Pg1–Ps201 | Cg26–Pg1–Ps202 | Cg26–Pg1–Ps203 | Cg26–Pg1–Ps204 |
| Cg26–Pg1–Ps205 | Cg26–Pg1–Ps206 | Cg26–Pg1–Ps207 | Cg26–Pg1–Ps208 |
| Cg26–Pg1–Ps209 | Cg26–Pg1–Ps210 | Cg26–Pg1–Ps211 | Cg26–Pg1–Ps212 |
| Cg26–Pg1–Ps213 | Cg26–Pg1–Ps214 | Cg26–Pg1–Ps215 | Cg26–Pg1–Ps216 |
| Cg26–Pg1–Ps217 | Cg26–Pg1–Ps218 | Cg26–Pg1–Ps219 | Cg26–Pg1–Ps220 |
| Cg26–Pg1–Ps221 | Cg26–Pg1–Ps222 | Cg26–Pg1–Ps223 | Cg26–Pg1–Ps224 |
| Cg26–Pg1–Ps225 | Cg26–Pg1–Ps226 | Cg26–Pg1–Ps227 | Cg26–Pg1–Ps228 |
| Cg26–Pg1–Ps229 | Cg26–Pg1–Ps230 | Cg26–Pg1–Ps231 | Cg26–Pg1–Ps232 |
| Cg26–Pg1–Ps233 | Cg26–Pg1–Ps234 | Cg26–Pg1–Ps235 | Cg26–Pg1–Ps236 |
| Cg26–Pg1–Ps237 | Cg26–Pg1–Ps238 | Cg26–Pg1–Ps239 | Cg26–Pg1–Ps240 |
| Cg26–Pg1–Ps241 | Cg26–Pg1–Ps242 | Cg26–Pg1–Ps243 | |

| | | | |
|---|---|---|---|
| Cg27–Pg1–Ps1 | Cg27–Pg1–Ps2 | Cg27–Pg1–Ps3 | Cg27–Pg1–Ps4 |
| Cg27–Pg1–Ps5 | Cg27–Pg1–Ps6 | Cg27–Pg1–Ps7 | Cg27–Pg1–Ps8 |
| Cg27–Pg1–Ps9 | Cg27–Pg1–Ps10 | Cg27–Pg1–Ps11 | Cg27–Pg1–Ps12 |
| Cg27–Pg1–Ps13 | Cg27–Pg1–Ps14 | Cg27–Pg1–Ps15 | Cg27–Pg1–Ps16 |
| Cg27–Pg1–Ps17 | Cg27–Pg1–Ps18 | Cg27–Pg1–Ps19 | Cg27–Pg1–Ps20 |
| Cg27–Pg1–Ps21 | Cg27–Pg1–Ps22 | Cg27–Pg1–Ps23 | Cg27–Pg1–Ps24 |
| Cg27–Pg1–Ps25 | Cg27–Pg1–Ps26 | Cg27–Pg1–Ps27 | Cg27–Pg1–Ps28 |
| Cg27–Pg1–Ps29 | Cg27–Pg1–Ps30 | Cg27–Pg1–Ps31 | Cg27–Pg1–Ps32 |
| Cg27–Pg1–Ps33 | Cg27–Pg1–Ps34 | Cg27–Pg1–Ps35 | Cg27–Pg1–Ps36 |
| Cg27–Pg1–Ps37 | Cg27–Pg1–Ps38 | Cg27–Pg1–Ps39 | Cg27–Pg1–Ps40 |
| Cg27–Pg1–Ps41 | Cg27–Pg1–Ps42 | Cg27–Pg1–Ps43 | Cg27–Pg1–Ps44 |
| Cg27–Pg1–Ps45 | Cg27–Pg1–Ps46 | Cg27–Pg1–Ps47 | Cg27–Pg1–Ps48 |
| Cg27–Pg1–Ps49 | Cg27–Pg1–Ps50 | Cg27–Pg1–Ps51 | Cg27–Pg1–Ps52 |
| Cg27–Pg1–Ps53 | Cg27–Pg1–Ps54 | Cg27–Pg1–Ps55 | Cg27–Pg1–Ps56 |
| Cg27–Pg1–Ps57 | Cg27–Pg1–Ps58 | Cg27–Pg1–Ps59 | Cg27–Pg1–Ps60 |
| Cg27–Pg1–Ps61 | Cg27–Pg1–Ps62 | Cg27–Pg1–Ps63 | Cg27–Pg1–Ps64 |
| Cg27–Pg1–Ps65 | Cg27–Pg1–Ps66 | Cg27–Pg1–Ps67 | Cg27–Pg1–Ps68 |
| Cg27–Pg1–Ps69 | Cg27–Pg1–Ps70 | Cg27–Pg1–Ps71 | Cg27–Pg1–Ps72 |
| Cg27–Pg1–Ps73 | Cg27–Pg1–Ps74 | Cg27–Pg1–Ps75 | Cg27–Pg1–Ps76 |
| Cg27–Pg1–Ps77 | Cg27–Pg1–Ps78 | Cg27–Pg1–Ps79 | Cg27–Pg1–Ps80 |
| Cg27–Pg1–Ps81 | Cg27–Pg1–Ps82 | Cg27–Pg1–Ps83 | Cg27–Pg1–Ps84 |
| Cg27–Pg1–Ps85 | Cg27–Pg1–Ps86 | Cg27–Pg1–Ps87 | Cg27–Pg1–Ps88 |
| Cg27–Pg1–Ps89 | Cg27–Pg1–Ps90 | Cg27–Pg1–Ps91 | Cg27–Pg1–Ps92 |
| Cg27–Pg1–Ps93 | Cg27–Pg1–Ps94 | Cg27–Pg1–Ps95 | Cg27–Pg1–Ps96 |
| Cg27–Pg1–Ps97 | Cg27–Pg1–Ps98 | Cg27–Pg1–Ps99 | Cg27–Pg1–Ps100 |
| Cg27–Pg1–Ps101 | Cg27–Pg1–Ps102 | Cg27–Pg1–Ps103 | Cg27–Pg1–Ps104 |
| Cg27–Pg1–Ps105 | Cg27–Pg1–Ps106 | Cg27–Pg1–Ps107 | Cg27–Pg1–Ps108 |
| Cg27–Pg1–Ps109 | Cg27–Pg1–Ps110 | Cg27–Pg1–Ps111 | Cg27–Pg1–Ps112 |
| Cg27–Pg1–Ps113 | Cg27–Pg1–Ps114 | Cg27–Pg1–Ps115 | Cg27–Pg1–Ps116 |
| Cg27–Pg1–Ps117 | Cg27–Pg1–Ps118 | Cg27–Pg1–Ps119 | Cg27–Pg1–Ps120 |
| Cg27–Pg1–Ps121 | Cg27–Pg1–Ps122 | Cg27–Pg1–Ps123 | Cg27–Pg1–Ps124 |
| Cg27–Pg1–Ps125 | Cg27–Pg1–Ps126 | Cg27–Pg1–Ps127 | Cg27–Pg1–Ps128 |
| Cg27–Pg1–Ps129 | Cg27–Pg1–Ps130 | Cg27–Pg1–Ps131 | Cg27–Pg1–Ps132 |

| | | | |
|---|---|---|---|
| Cg27–Pg1–Ps133 | Cg27–Pg1–Ps134 | Cg27–Pg1–Ps135 | Cg27–Pg1–Ps136 |
| Cg27–Pg1–Ps137 | Cg27–Pg1–Ps138 | Cg27–Pg1–Ps139 | Cg27–Pg1–Ps140 |
| Cg27–Pg1–Ps141 | Cg27–Pg1–Ps142 | Cg27–Pg1–Ps143 | Cg27–Pg1–Ps144 |
| Cg27–Pg1–Ps145 | Cg27–Pg1–Ps146 | Cg27–Pg1–Ps147 | Cg27–Pg1–Ps148 |
| Cg27–Pg1–Ps149 | Cg27–Pg1–Ps150 | Cg27–Pg1–Ps151 | Cg27–Pg1–Ps152 |
| Cg27–Pg1–Ps153 | Cg27–Pg1–Ps154 | Cg27–Pg1–Ps155 | Cg27–Pg1–Ps156 |
| Cg27–Pg1–Ps157 | Cg27–Pg1–Ps158 | Cg27–Pg1–Ps159 | Cg27–Pg1–Ps160 |
| Cg27–Pg1–Ps161 | Cg27–Pg1–Ps162 | Cg27–Pg1–Ps163 | Cg27–Pg1–Ps164 |
| Cg27–Pg1–Ps165 | Cg27–Pg1–Ps166 | Cg27–Pg1–Ps167 | Cg27–Pg1–Ps168 |
| Cg27–Pg1–Ps169 | Cg27–Pg1–Ps170 | Cg27–Pg1–Ps171 | Cg27–Pg1–Ps172 |
| Cg27–Pg1–Ps173 | Cg27–Pg1–Ps174 | Cg27–Pg1–Ps175 | Cg27–Pg1–Ps176 |
| Cg27–Pg1–Ps177 | Cg27–Pg1–Ps178 | Cg27–Pg1–Ps179 | Cg27–Pg1–Ps180 |
| Cg27–Pg1–Ps181 | Cg27–Pg1–Ps182 | Cg27–Pg1–Ps183 | Cg27–Pg1–Ps184 |
| Cg27–Pg1–Ps185 | Cg27–Pg1–Ps186 | Cg27–Pg1–Ps187 | Cg27–Pg1–Ps188 |
| Cg27–Pg1–Ps189 | Cg27–Pg1–Ps190 | Cg27–Pg1–Ps191 | Cg27–Pg1–Ps192 |
| Cg27–Pg1–Ps193 | Cg27–Pg1–Ps194 | Cg27–Pg1–Ps195 | Cg27–Pg1–Ps196 |
| Cg27–Pg1–Ps197 | Cg27–Pg1–Ps198 | Cg27–Pg1–Ps199 | Cg27–Pg1–Ps200 |
| Cg27–Pg1–Ps201 | Cg27–Pg1–Ps202 | Cg27–Pg1–Ps203 | Cg27–Pg1–Ps204 |
| Cg27–Pg1–Ps205 | Cg27–Pg1–Ps206 | Cg27–Pg1–Ps207 | Cg27–Pg1–Ps208 |
| Cg27–Pg1–Ps209 | Cg27–Pg1–Ps210 | Cg27–Pg1–Ps211 | Cg27–Pg1–Ps212 |
| Cg27–Pg1–Ps213 | Cg27–Pg1–Ps214 | Cg27–Pg1–Ps215 | Cg27–Pg1–Ps216 |
| Cg27–Pg1–Ps217 | Cg27–Pg1–Ps218 | Cg27–Pg1–Ps219 | Cg27–Pg1–Ps220 |
| Cg27–Pg1–Ps221 | Cg27–Pg1–Ps222 | Cg27–Pg1–Ps223 | Cg27–Pg1–Ps224 |
| Cg27–Pg1–Ps225 | Cg27–Pg1–Ps226 | Cg27–Pg1–Ps227 | Cg27–Pg1–Ps228 |
| Cg27–Pg1–Ps229 | Cg27–Pg1–Ps230 | Cg27–Pg1–Ps231 | Cg27–Pg1–Ps232 |
| Cg27–Pg1–Ps233 | Cg27–Pg1–Ps234 | Cg27–Pg1–Ps235 | Cg27–Pg1–Ps236 |
| Cg27–Pg1–Ps237 | Cg27–Pg1–Ps238 | Cg27–Pg1–Ps239 | Cg27–Pg1–Ps240 |
| Cg27–Pg1–Ps241 | Cg27–Pg1–Ps242 | Cg27–Pg1–Ps243 | |

| | | | |
|---|---|---|---|
| Cg28–Pg1–Ps1 | Cg28–Pg1–Ps2 | Cg28–Pg1–Ps3 | Cg28–Pg1–Ps4 |
| Cg28–Pg1–Ps5 | Cg28–Pg1–Ps6 | Cg28–Pg1–Ps7 | Cg28–Pg1–Ps8 |
| Cg28–Pg1–Ps9 | Cg28–Pg1–Ps10 | Cg28–Pg1–Ps11 | Cg28–Pg1–Ps12 |
| Cg28–Pg1–Ps13 | Cg28–Pg1–Ps14 | Cg28–Pg1–Ps15 | Cg28–Pg1–Ps16 |
| Cg28–Pg1–Ps17 | Cg28–Pg1–Ps18 | Cg28–Pg1–Ps19 | Cg28–Pg1–Ps20 |
| Cg28–Pg1–Ps21 | Cg28–Pg1–Ps22 | Cg28–Pg1–Ps23 | Cg28–Pg1–Ps24 |
| Cg28–Pg1–Ps25 | Cg28–Pg1–Ps26 | Cg28–Pg1–Ps27 | Cg28–Pg1–Ps28 |
| Cg28–Pg1–Ps29 | Cg28–Pg1–Ps30 | Cg28–Pg1–Ps31 | Cg28–Pg1–Ps32 |
| Cg28–Pg1–Ps33 | Cg28–Pg1–Ps34 | Cg28–Pg1–Ps35 | Cg28–Pg1–Ps36 |
| Cg28–Pg1–Ps37 | Cg28–Pg1–Ps38 | Cg28–Pg1–Ps39 | Cg28–Pg1–Ps40 |
| Cg28–Pg1–Ps41 | Cg28–Pg1–Ps42 | Cg28–Pg1–Ps43 | Cg28–Pg1–Ps44 |
| Cg28–Pg1–Ps45 | Cg28–Pg1–Ps46 | Cg28–Pg1–Ps47 | Cg28–Pg1–Ps48 |
| Cg28–Pg1–Ps49 | Cg28–Pg1–Ps50 | Cg28–Pg1–Ps51 | Cg28–Pg1–Ps52 |
| Cg28–Pg1–Ps53 | Cg28–Pg1–Ps54 | Cg28–Pg1–Ps55 | Cg28–Pg1–Ps56 |
| Cg28–Pg1–Ps57 | Cg28–Pg1–Ps58 | Cg28–Pg1–Ps59 | Cg28–Pg1–Ps60 |
| Cg28–Pg1–Ps61 | Cg28–Pg1–Ps62 | Cg28–Pg1–Ps63 | Cg28–Pg1–Ps64 |
| Cg28–Pg1–Ps65 | Cg28–Pg1–Ps66 | Cg28–Pg1–Ps67 | Cg28–Pg1–Ps68 |
| Cg28–Pg1–Ps69 | Cg28–Pg1–Ps70 | Cg28–Pg1–Ps71 | Cg28–Pg1–Ps72 |
| Cg28–Pg1–Ps73 | Cg28–Pg1–Ps74 | Cg28–Pg1–Ps75 | Cg28–Pg1–Ps76 |
| Cg28–Pg1–Ps77 | Cg28–Pg1–Ps78 | Cg28–Pg1–Ps79 | Cg28–Pg1–Ps80 |
| Cg28–Pg1–Ps81 | Cg28–Pg1–Ps82 | Cg28–Pg1–Ps83 | Cg28–Pg1–Ps84 |
| Cg28–Pg1–Ps85 | Cg28–Pg1–Ps86 | Cg28–Pg1–Ps87 | Cg28–Pg1–Ps88 |
| Cg28–Pg1–Ps89 | Cg28–Pg1–Ps90 | Cg28–Pg1–Ps91 | Cg28–Pg1–Ps92 |

| | | | |
|---|---|---|---|
| Cg28–Pg1–Ps93 | Cg28–Pg1–Ps94 | Cg28–Pg1–Ps95 | Cg28–Pg1–Ps96 |
| Cg28–Pg1–Ps97 | Cg28–Pg1–Ps98 | Cg28–Pg1–Ps99 | Cg28–Pg1–Ps100 |
| Cg28–Pg1–Ps101 | Cg28–Pg1–Ps102 | Cg28–Pg1–Ps103 | Cg28–Pg1–Ps104 |
| Cg28–Pg1–Ps105 | Cg28–Pg1–Ps106 | Cg28–Pg1–Ps107 | Cg28–Pg1–Ps108 |
| Cg28–Pg1–Ps109 | Cg28–Pg1–Ps110 | Cg28–Pg1–Ps111 | Cg28–Pg1–Ps112 |
| Cg28–Pg1–Ps113 | Cg28–Pg1–Ps114 | Cg28–Pg1–Ps115 | Cg28–Pg1–Ps116 |
| Cg28–Pg1–Ps117 | Cg28–Pg1–Ps118 | Cg28–Pg1–Ps119 | Cg28–Pg1–Ps120 |
| Cg28–Pg1–Ps121 | Cg28–Pg1–Ps122 | Cg28–Pg1–Ps123 | Cg28–Pg1–Ps124 |
| Cg28–Pg1–Ps125 | Cg28–Pg1–Ps126 | Cg28–Pg1–Ps127 | Cg28–Pg1–Ps128 |
| Cg28–Pg1–Ps129 | Cg28–Pg1–Ps130 | Cg28–Pg1–Ps131 | Cg28–Pg1–Ps132 |
| Cg28–Pg1–Ps133 | Cg28–Pg1–Ps134 | Cg28–Pg1–Ps135 | Cg28–Pg1–Ps136 |
| Cg28–Pg1–Ps137 | Cg28–Pg1–Ps138 | Cg28–Pg1–Ps139 | Cg28–Pg1–Ps140 |
| Cg28–Pg1–Ps141 | Cg28–Pg1–Ps142 | Cg28–Pg1–Ps143 | Cg28–Pg1–Ps144 |
| Cg28–Pg1–Ps145 | Cg28–Pg1–Ps146 | Cg28–Pg1–Ps147 | Cg28–Pg1–Ps148 |
| Cg28–Pg1–Ps149 | Cg28–Pg1–Ps150 | Cg28–Pg1–Ps151 | Cg28–Pg1–Ps152 |
| Cg28–Pg1–Ps153 | Cg28–Pg1–Ps154 | Cg28–Pg1–Ps155 | Cg28–Pg1–Ps156 |
| Cg28–Pg1–Ps157 | Cg28–Pg1–Ps158 | Cg28–Pg1–Ps159 | Cg28–Pg1–Ps160 |
| Cg28–Pg1–Ps161 | Cg28–Pg1–Ps162 | Cg28–Pg1–Ps163 | Cg28–Pg1–Ps164 |
| Cg28–Pg1–Ps165 | Cg28–Pg1–Ps166 | Cg28–Pg1–Ps167 | Cg28–Pg1–Ps168 |
| Cg28–Pg1–Ps169 | Cg28–Pg1–Ps170 | Cg28–Pg1–Ps171 | Cg28–Pg1–Ps172 |
| Cg28–Pg1–Ps173 | Cg28–Pg1–Ps174 | Cg28–Pg1–Ps175 | Cg28–Pg1–Ps176 |
| Cg28–Pg1–Ps177 | Cg28–Pg1–Ps178 | Cg28–Pg1–Ps179 | Cg28–Pg1–Ps180 |
| Cg28–Pg1–Ps181 | Cg28–Pg1–Ps182 | Cg28–Pg1–Ps183 | Cg28–Pg1–Ps184 |
| Cg28–Pg1–Ps185 | Cg28–Pg1–Ps186 | Cg28–Pg1–Ps187 | Cg28–Pg1–Ps188 |
| Cg28–Pg1–Ps189 | Cg28–Pg1–Ps190 | Cg28–Pg1–Ps191 | Cg28–Pg1–Ps192 |
| Cg28–Pg1–Ps193 | Cg28–Pg1–Ps194 | Cg28–Pg1–Ps195 | Cg28–Pg1–Ps196 |
| Cg28–Pg1–Ps197 | Cg28–Pg1–Ps198 | Cg28–Pg1–Ps199 | Cg28–Pg1–Ps200 |
| Cg28–Pg1–Ps201 | Cg28–Pg1–Ps202 | Cg28–Pg1–Ps203 | Cg28–Pg1–Ps204 |
| Cg28–Pg1–Ps205 | Cg28–Pg1–Ps206 | Cg28–Pg1–Ps207 | Cg28–Pg1–Ps208 |
| Cg28–Pg1–Ps209 | Cg28–Pg1–Ps210 | Cg28–Pg1–Ps211 | Cg28–Pg1–Ps212 |
| Cg28–Pg1–Ps213 | Cg28–Pg1–Ps214 | Cg28–Pg1–Ps215 | Cg28–Pg1–Ps216 |
| Cg28–Pg1–Ps217 | Cg28–Pg1–Ps218 | Cg28–Pg1–Ps219 | Cg28–Pg1–Ps220 |
| Cg28–Pg1–Ps221 | Cg28–Pg1–Ps222 | Cg28–Pg1–Ps223 | Cg28–Pg1–Ps224 |
| Cg28–Pg1–Ps225 | Cg28–Pg1–Ps226 | Cg28–Pg1–Ps227 | Cg28–Pg1–Ps228 |
| Cg28–Pg1–Ps229 | Cg28–Pg1–Ps230 | Cg28–Pg1–Ps231 | Cg28–Pg1–Ps232 |
| Cg28–Pg1–Ps233 | Cg28–Pg1–Ps234 | Cg28–Pg1–Ps235 | Cg28–Pg1–Ps236 |
| Cg28–Pg1–Ps237 | Cg28–Pg1–Ps238 | Cg28–Pg1–Ps239 | Cg28–Pg1–Ps240 |
| Cg28–Pg1–Ps241 | Cg28–Pg1–Ps242 | Cg28–Pg1–Ps243 | |

| | | | |
|---|---|---|---|
| Cg29–Pg1–Ps1 | Cg29–Pg1–Ps2 | Cg29–Pg1–Ps3 | Cg29–Pg1–Ps4 |
| Cg29–Pg1–Ps5 | Cg29–Pg1–Ps6 | Cg29–Pg1–Ps7 | Cg29–Pg1–Ps8 |
| Cg29–Pg1–Ps9 | Cg29–Pg1–Ps10 | Cg29–Pg1–Ps11 | Cg29–Pg1–Ps12 |
| Cg29–Pg1–Ps13 | Cg29–Pg1–Ps14 | Cg29–Pg1–Ps15 | Cg29–Pg1–Ps16 |
| Cg29–Pg1–Ps17 | Cg29–Pg1–Ps18 | Cg29–Pg1–Ps19 | Cg29–Pg1–Ps20 |
| Cg29–Pg1–Ps21 | Cg29–Pg1–Ps22 | Cg29–Pg1–Ps23 | Cg29–Pg1–Ps24 |
| Cg29–Pg1–Ps25 | Cg29–Pg1–Ps26 | Cg29–Pg1–Ps27 | Cg29–Pg1–Ps28 |
| Cg29–Pg1–Ps29 | Cg29–Pg1–Ps30 | Cg29–Pg1–Ps31 | Cg29–Pg1–Ps32 |
| Cg29–Pg1–Ps33 | Cg29–Pg1–Ps34 | Cg29–Pg1–Ps35 | Cg29–Pg1–Ps36 |
| Cg29–Pg1–Ps37 | Cg29–Pg1–Ps38 | Cg29–Pg1–Ps39 | Cg29–Pg1–Ps40 |
| Cg29–Pg1–Ps41 | Cg29–Pg1–Ps42 | Cg29–Pg1–Ps43 | Cg29–Pg1–Ps44 |
| Cg29–Pg1–Ps45 | Cg29–Pg1–Ps46 | Cg29–Pg1–Ps47 | Cg29–Pg1–Ps48 |
| Cg29–Pg1–Ps49 | Cg29–Pg1–Ps50 | Cg29–Pg1–Ps51 | Cg29–Pg1–Ps52 |

| | | | |
|---|---|---|---|
| Cg29-Pg1-Ps53 | Cg29-Pg1-Ps54 | Cg29-Pg1-Ps55 | Cg29-Pg1-Ps56 |
| Cg29-Pg1-Ps57 | Cg29-Pg1-Ps58 | Cg29-Pg1-Ps59 | Cg29-Pg1-Ps60 |
| Cg29-Pg1-Ps61 | Cg29-Pg1-Ps62 | Cg29-Pg1-Ps63 | Cg29-Pg1-Ps64 |
| Cg29-Pg1-Ps65 | Cg29-Pg1-Ps66 | Cg29-Pg1-Ps67 | Cg29-Pg1-Ps68 |
| Cg29-Pg1-Ps69 | Cg29-Pg1-Ps70 | Cg29-Pg1-Ps71 | Cg29-Pg1-Ps72 |
| Cg29-Pg1-Ps73 | Cg29-Pg1-Ps74 | Cg29-Pg1-Ps75 | Cg29-Pg1-Ps76 |
| Cg29-Pg1-Ps77 | Cg29-Pg1-Ps78 | Cg29-Pg1-Ps79 | Cg29-Pg1-Ps80 |
| Cg29-Pg1-Ps81 | Cg29-Pg1-Ps82 | Cg29-Pg1-Ps83 | Cg29-Pg1-Ps84 |
| Cg29-Pg1-Ps85 | Cg29-Pg1-Ps86 | Cg29-Pg1-Ps87 | Cg29-Pg1-Ps88 |
| Cg29-Pg1-Ps89 | Cg29-Pg1-Ps90 | Cg29-Pg1-Ps91 | Cg29-Pg1-Ps92 |
| Cg29-Pg1-Ps93 | Cg29-Pg1-Ps94 | Cg29-Pg1-Ps95 | Cg29-Pg1-Ps96 |
| Cg29-Pg1-Ps97 | Cg29-Pg1-Ps98 | Cg29-Pg1-Ps99 | Cg29-Pg1-Ps100 |
| Cg29-Pg1-Ps101 | Cg29-Pg1-Ps102 | Cg29-Pg1-Ps103 | Cg29-Pg1-Ps104 |
| Cg29-Pg1-Ps105 | Cg29-Pg1-Ps106 | Cg29-Pg1-Ps107 | Cg29-Pg1-Ps108 |
| Cg29-Pg1-Ps109 | Cg29-Pg1-Ps110 | Cg29-Pg1-Ps111 | Cg29-Pg1-Ps112 |
| Cg29-Pg1-Ps113 | Cg29-Pg1-Ps114 | Cg29-Pg1-Ps115 | Cg29-Pg1-Ps116 |
| Cg29-Pg1-Ps117 | Cg29-Pg1-Ps118 | Cg29-Pg1-Ps119 | Cg29-Pg1-Ps120 |
| Cg29-Pg1-Ps121 | Cg29-Pg1-Ps122 | Cg29-Pg1-Ps123 | Cg29-Pg1-Ps124 |
| Cg29-Pg1-Ps125 | Cg29-Pg1-Ps126 | Cg29-Pg1-Ps127 | Cg29-Pg1-Ps128 |
| Cg29-Pg1-Ps129 | Cg29-Pg1-Ps130 | Cg29-Pg1-Ps131 | Cg29-Pg1-Ps132 |
| Cg29-Pg1-Ps133 | Cg29-Pg1-Ps134 | Cg29-Pg1-Ps135 | Cg29-Pg1-Ps136 |
| Cg29-Pg1-Ps137 | Cg29-Pg1-Ps138 | Cg29-Pg1-Ps139 | Cg29-Pg1-Ps140 |
| Cg29-Pg1-Ps141 | Cg29-Pg1-Ps142 | Cg29-Pg1-Ps143 | Cg29-Pg1-Ps144 |
| Cg29-Pg1-Ps145 | Cg29-Pg1-Ps146 | Cg29-Pg1-Ps147 | Cg29-Pg1-Ps148 |
| Cg29-Pg1-Ps149 | Cg29-Pg1-Ps150 | Cg29-Pg1-Ps151 | Cg29-Pg1-Ps152 |
| Cg29-Pg1-Ps153 | Cg29-Pg1-Ps154 | Cg29-Pg1-Ps155 | Cg29-Pg1-Ps156 |
| Cg29-Pg1-Ps157 | Cg29-Pg1-Ps158 | Cg29-Pg1-Ps159 | Cg29-Pg1-Ps160 |
| Cg29-Pg1-Ps161 | Cg29-Pg1-Ps162 | Cg29-Pg1-Ps163 | Cg29-Pg1-Ps164 |
| Cg29-Pg1-Ps165 | Cg29-Pg1-Ps166 | Cg29-Pg1-Ps167 | Cg29-Pg1-Ps168 |
| Cg29-Pg1-Ps169 | Cg29-Pg1-Ps170 | Cg29-Pg1-Ps171 | Cg29-Pg1-Ps172 |
| Cg29-Pg1-Ps173 | Cg29-Pg1-Ps174 | Cg29-Pg1-Ps175 | Cg29-Pg1-Ps176 |
| Cg29-Pg1-Ps177 | Cg29-Pg1-Ps178 | Cg29-Pg1-Ps179 | Cg29-Pg1-Ps180 |
| Cg29-Pg1-Ps181 | Cg29-Pg1-Ps182 | Cg29-Pg1-Ps183 | Cg29-Pg1-Ps184 |
| Cg29-Pg1-Ps185 | Cg29-Pg1-Ps186 | Cg29-Pg1-Ps187 | Cg29-Pg1-Ps188 |
| Cg29-Pg1-Ps189 | Cg29-Pg1-Ps190 | Cg29-Pg1-Ps191 | Cg29-Pg1-Ps192 |
| Cg29-Pg1-Ps193 | Cg29-Pg1-Ps194 | Cg29-Pg1-Ps195 | Cg29-Pg1-Ps196 |
| Cg29-Pg1-Ps197 | Cg29-Pg1-Ps198 | Cg29-Pg1-Ps199 | Cg29-Pg1-Ps200 |
| Cg29-Pg1-Ps201 | Cg29-Pg1-Ps202 | Cg29-Pg1-Ps203 | Cg29-Pg1-Ps204 |
| Cg29-Pg1-Ps205 | Cg29-Pg1-Ps206 | Cg29-Pg1-Ps207 | Cg29-Pg1-Ps208 |
| Cg29-Pg1-Ps209 | Cg29-Pg1-Ps210 | Cg29-Pg1-Ps211 | Cg29-Pg1-Ps212 |
| Cg29-Pg1-Ps213 | Cg29-Pg1-Ps214 | Cg29-Pg1-Ps215 | Cg29-Pg1-Ps216 |
| Cg29-Pg1-Ps217 | Cg29-Pg1-Ps218 | Cg29-Pg1-Ps219 | Cg29-Pg1-Ps220 |
| Cg29-Pg1-Ps221 | Cg29-Pg1-Ps222 | Cg29-Pg1-Ps223 | Cg29-Pg1-Ps224 |
| Cg29-Pg1-Ps225 | Cg29-Pg1-Ps226 | Cg29-Pg1-Ps227 | Cg29-Pg1-Ps228 |
| Cg29-Pg1-Ps229 | Cg29-Pg1-Ps230 | Cg29-Pg1-Ps231 | Cg29-Pg1-Ps232 |
| Cg29-Pg1-Ps233 | Cg29-Pg1-Ps234 | Cg29-Pg1-Ps235 | Cg29-Pg1-Ps236 |
| Cg29-Pg1-Ps237 | Cg29-Pg1-Ps238 | Cg29-Pg1-Ps239 | Cg29-Pg1-Ps240 |
| Cg29-Pg1-Ps241 | Cg29-Pg1-Ps242 | Cg29-Pg1-Ps243 | |
| Cg30-Pg1-Ps1 | Cg30-Pg1-Ps2 | Cg30-Pg1-Ps3 | Cg30-Pg1-Ps4 |
| Cg30-Pg1-Ps5 | Cg30-Pg1-Ps6 | Cg30-Pg1-Ps7 | Cg30-Pg1-Ps8 |
| Cg30-Pg1-Ps9 | Cg30-Pg1-Ps10 | Cg30-Pg1-Ps11 | Cg30-Pg1-Ps12 |

| | | | |
|---|---|---|---|
| Cg30–Pg1–Ps13 | Cg30–Pg1–Ps14 | Cg30–Pg1–Ps15 | Cg30–Pg1–Ps16 |
| Cg30–Pg1–Ps17 | Cg30–Pg1–Ps18 | Cg30–Pg1–Ps19 | Cg30–Pg1–Ps20 |
| Cg30–Pg1–Ps21 | Cg30–Pg1–Ps22 | Cg30–Pg1–Ps23 | Cg30–Pg1–Ps24 |
| Cg30–Pg1–Ps25 | Cg30–Pg1–Ps26 | Cg30–Pg1–Ps27 | Cg30–Pg1–Ps28 |
| Cg30–Pg1–Ps29 | Cg30–Pg1–Ps30 | Cg30–Pg1–Ps31 | Cg30–Pg1–Ps32 |
| Cg30–Pg1–Ps33 | Cg30–Pg1–Ps34 | Cg30–Pg1–Ps35 | Cg30–Pg1–Ps36 |
| Cg30–Pg1–Ps37 | Cg30–Pg1–Ps38 | Cg30–Pg1–Ps39 | Cg30–Pg1–Ps40 |
| Cg30–Pg1–Ps41 | Cg30–Pg1–Ps42 | Cg30–Pg1–Ps43 | Cg30–Pg1–Ps44 |
| Cg30–Pg1–Ps45 | Cg30–Pg1–Ps46 | Cg30–Pg1–Ps47 | Cg30–Pg1–Ps48 |
| Cg30–Pg1–Ps49 | Cg30–Pg1–Ps50 | Cg30–Pg1–Ps51 | Cg30–Pg1–Ps52 |
| Cg30–Pg1–Ps53 | Cg30–Pg1–Ps54 | Cg30–Pg1–Ps55 | Cg30–Pg1–Ps56 |
| Cg30–Pg1–Ps57 | Cg30–Pg1–Ps58 | Cg30–Pg1–Ps59 | Cg30–Pg1–Ps60 |
| Cg30–Pg1–Ps61 | Cg30–Pg1–Ps62 | Cg30–Pg1–Ps63 | Cg30–Pg1–Ps64 |
| Cg30–Pg1–Ps65 | Cg30–Pg1–Ps66 | Cg30–Pg1–Ps67 | Cg30–Pg1–Ps68 |
| Cg30–Pg1–Ps69 | Cg30–Pg1–Ps70 | Cg30–Pg1–Ps71 | Cg30–Pg1–Ps72 |
| Cg30–Pg1–Ps73 | Cg30–Pg1–Ps74 | Cg30–Pg1–Ps75 | Cg30–Pg1–Ps76 |
| Cg30–Pg1–Ps77 | Cg30–Pg1–Ps78 | Cg30–Pg1–Ps79 | Cg30–Pg1–Ps80 |
| Cg30–Pg1–Ps81 | Cg30–Pg1–Ps82 | Cg30–Pg1–Ps83 | Cg30–Pg1–Ps84 |
| Cg30–Pg1–Ps85 | Cg30–Pg1–Ps86 | Cg30–Pg1–Ps87 | Cg30–Pg1–Ps88 |
| Cg30–Pg1–Ps89 | Cg30–Pg1–Ps90 | Cg30–Pg1–Ps91 | Cg30–Pg1–Ps92 |
| Cg30–Pg1–Ps93 | Cg30–Pg1–Ps94 | Cg30–Pg1–Ps95 | Cg30–Pg1–Ps96 |
| Cg30–Pg1–Ps97 | Cg30–Pg1–Ps98 | Cg30–Pg1–Ps99 | Cg30–Pg1–Ps100 |
| Cg30–Pg1–Ps101 | Cg30–Pg1–Ps102 | Cg30–Pg1–Ps103 | Cg30–Pg1–Ps104 |
| Cg30–Pg1–Ps105 | Cg30–Pg1–Ps106 | Cg30–Pg1–Ps107 | Cg30–Pg1–Ps108 |
| Cg30–Pg1–Ps109 | Cg30–Pg1–Ps110 | Cg30–Pg1–Ps111 | Cg30–Pg1–Ps112 |
| Cg30–Pg1–Ps113 | Cg30–Pg1–Ps114 | Cg30–Pg1–Ps115 | Cg30–Pg1–Ps116 |
| Cg30–Pg1–Ps117 | Cg30–Pg1–Ps118 | Cg30–Pg1–Ps119 | Cg30–Pg1–Ps120 |
| Cg30–Pg1–Ps121 | Cg30–Pg1–Ps122 | Cg30–Pg1–Ps123 | Cg30–Pg1–Ps124 |
| Cg30–Pg1–Ps125 | Cg30–Pg1–Ps126 | Cg30–Pg1–Ps127 | Cg30–Pg1–Ps128 |
| Cg30–Pg1–Ps129 | Cg30–Pg1–Ps130 | Cg30–Pg1–Ps131 | Cg30–Pg1–Ps132 |
| Cg30–Pg1–Ps133 | Cg30–Pg1–Ps134 | Cg30–Pg1–Ps135 | Cg30–Pg1–Ps136 |
| Cg30–Pg1–Ps137 | Cg30–Pg1–Ps138 | Cg30–Pg1–Ps139 | Cg30–Pg1–Ps140 |
| Cg30–Pg1–Ps141 | Cg30–Pg1–Ps142 | Cg30–Pg1–Ps143 | Cg30–Pg1–Ps144 |
| Cg30–Pg1–Ps145 | Cg30–Pg1–Ps146 | Cg30–Pg1–Ps147 | Cg30–Pg1–Ps148 |
| Cg30–Pg1–Ps149 | Cg30–Pg1–Ps150 | Cg30–Pg1–Ps151 | Cg30–Pg1–Ps152 |
| Cg30–Pg1–Ps153 | Cg30–Pg1–Ps154 | Cg30–Pg1–Ps155 | Cg30–Pg1–Ps156 |
| Cg30–Pg1–Ps157 | Cg30–Pg1–Ps158 | Cg30–Pg1–Ps159 | Cg30–Pg1–Ps160 |
| Cg30–Pg1–Ps161 | Cg30–Pg1–Ps162 | Cg30–Pg1–Ps163 | Cg30–Pg1–Ps164 |
| Cg30–Pg1–Ps165 | Cg30–Pg1–Ps166 | Cg30–Pg1–Ps167 | Cg30–Pg1–Ps168 |
| Cg30–Pg1–Ps169 | Cg30–Pg1–Ps170 | Cg30–Pg1–Ps171 | Cg30–Pg1–Ps172 |
| Cg30–Pg1–Ps173 | Cg30–Pg1–Ps174 | Cg30–Pg1–Ps175 | Cg30–Pg1–Ps176 |
| Cg30–Pg1–Ps177 | Cg30–Pg1–Ps178 | Cg30–Pg1–Ps179 | Cg30–Pg1–Ps180 |
| Cg30–Pg1–Ps181 | Cg30–Pg1–Ps182 | Cg30–Pg1–Ps183 | Cg30–Pg1–Ps184 |
| Cg30–Pg1–Ps185 | Cg30–Pg1–Ps186 | Cg30–Pg1–Ps187 | Cg30–Pg1–Ps188 |
| Cg30–Pg1–Ps189 | Cg30–Pg1–Ps190 | Cg30–Pg1–Ps191 | Cg30–Pg1–Ps192 |
| Cg30–Pg1–Ps193 | Cg30–Pg1–Ps194 | Cg30–Pg1–Ps195 | Cg30–Pg1–Ps196 |
| Cg30–Pg1–Ps197 | Cg30–Pg1–Ps198 | Cg30–Pg1–Ps199 | Cg30–Pg1–Ps200 |
| Cg30–Pg1–Ps201 | Cg30–Pg1–Ps202 | Cg30–Pg1–Ps203 | Cg30–Pg1–Ps204 |
| Cg30–Pg1–Ps205 | Cg30–Pg1–Ps206 | Cg30–Pg1–Ps207 | Cg30–Pg1–Ps208 |
| Cg30–Pg1–Ps209 | Cg30–Pg1–Ps210 | Cg30–Pg1–Ps211 | Cg30–Pg1–Ps212 |
| Cg30–Pg1–Ps213 | Cg30–Pg1–Ps214 | Cg30–Pg1–Ps215 | Cg30–Pg1–Ps216 |
| Cg30–Pg1–Ps217 | Cg30–Pg1–Ps218 | Cg30–Pg1–Ps219 | Cg30–Pg1–Ps220 |

| | | | |
|---|---|---|---|
| Cg30-Pg1-Ps221 | Cg30-Pg1-Ps222 | Cg30-Pg1-Ps223 | Cg30-Pg1-Ps224 |
| Cg30-Pg1-Ps225 | Cg30-Pg1-Ps226 | Cg30-Pg1-Ps227 | Cg30-Pg1-Ps228 |
| Cg30-Pg1-Ps229 | Cg30-Pg1-Ps230 | Cg30-Pg1-Ps231 | Cg30-Pg1-Ps232 |
| Cg30-Pg1-Ps233 | Cg30-Pg1-Ps234 | Cg30-Pg1-Ps235 | Cg30-Pg1-Ps236 |
| Cg30-Pg1-Ps237 | Cg30-Pg1-Ps238 | Cg30-Pg1-Ps239 | Cg30-Pg1-Ps240 |
| Cg30-Pg1-Ps241 | Cg30-Pg1-Ps242 | Cg30-Pg1-Ps243 | |

| | | | |
|---|---|---|---|
| Cg31-Pg1-Ps1 | Cg31-Pg1-Ps2 | Cg31-Pg1-Ps3 | Cg31-Pg1-Ps4 |
| Cg31-Pg1-Ps5 | Cg31-Pg1-Ps6 | Cg31-Pg1-Ps7 | Cg31-Pg1-Ps8 |
| Cg31-Pg1-Ps9 | Cg31-Pg1-Ps10 | Cg31-Pg1-Ps11 | Cg31-Pg1-Ps12 |
| Cg31-Pg1-Ps13 | Cg31-Pg1-Ps14 | Cg31-Pg1-Ps15 | Cg31-Pg1-Ps16 |
| Cg31-Pg1-Ps17 | Cg31-Pg1-Ps18 | Cg31-Pg1-Ps19 | Cg31-Pg1-Ps20 |
| Cg31-Pg1-Ps21 | Cg31-Pg1-Ps22 | Cg31-Pg1-Ps23 | Cg31-Pg1-Ps24 |
| Cg31-Pg1-Ps25 | Cg31-Pg1-Ps26 | Cg31-Pg1-Ps27 | Cg31-Pg1-Ps28 |
| Cg31-Pg1-Ps29 | Cg31-Pg1-Ps30 | Cg31-Pg1-Ps31 | Cg31-Pg1-Ps32 |
| Cg31-Pg1-Ps33 | Cg31-Pg1-Ps34 | Cg31-Pg1-Ps35 | Cg31-Pg1-Ps36 |
| Cg31-Pg1-Ps37 | Cg31-Pg1-Ps38 | Cg31-Pg1-Ps39 | Cg31-Pg1-Ps40 |
| Cg31-Pg1-Ps41 | Cg31-Pg1-Ps42 | Cg31-Pg1-Ps43 | Cg31-Pg1-Ps44 |
| Cg31-Pg1-Ps45 | Cg31-Pg1-Ps46 | Cg31-Pg1-Ps47 | Cg31-Pg1-Ps48 |
| Cg31-Pg1-Ps49 | Cg31-Pg1-Ps50 | Cg31-Pg1-Ps51 | Cg31-Pg1-Ps52 |
| Cg31-Pg1-Ps53 | Cg31-Pg1-Ps54 | Cg31-Pg1-Ps55 | Cg31-Pg1-Ps56 |
| Cg31-Pg1-Ps57 | Cg31-Pg1-Ps58 | Cg31-Pg1-Ps59 | Cg31-Pg1-Ps60 |
| Cg31-Pg1-Ps61 | Cg31-Pg1-Ps62 | Cg31-Pg1-Ps63 | Cg31-Pg1-Ps64 |
| Cg31-Pg1-Ps65 | Cg31-Pg1-Ps66 | Cg31-Pg1-Ps67 | Cg31-Pg1-Ps68 |
| Cg31-Pg1-Ps69 | Cg31-Pg1-Ps70 | Cg31-Pg1-Ps71 | Cg31-Pg1-Ps72 |
| Cg31-Pg1-Ps73 | Cg31-Pg1-Ps74 | Cg31-Pg1-Ps75 | Cg31-Pg1-Ps76 |
| Cg31-Pg1-Ps77 | Cg31-Pg1-Ps78 | Cg31-Pg1-Ps79 | Cg31-Pg1-Ps80 |
| Cg31-Pg1-Ps81 | Cg31-Pg1-Ps82 | Cg31-Pg1-Ps83 | Cg31-Pg1-Ps84 |
| Cg31-Pg1-Ps85 | Cg31-Pg1-Ps86 | Cg31-Pg1-Ps87 | Cg31-Pg1-Ps88 |
| Cg31-Pg1-Ps89 | Cg31-Pg1-Ps90 | Cg31-Pg1-Ps91 | Cg31-Pg1-Ps92 |
| Cg31-Pg1-Ps93 | Cg31-Pg1-Ps94 | Cg31-Pg1-Ps95 | Cg31-Pg1-Ps96 |
| Cg31-Pg1-Ps97 | Cg31-Pg1-Ps98 | Cg31-Pg1-Ps99 | Cg31-Pg1-Ps100 |
| Cg31-Pg1-Ps101 | Cg31-Pg1-Ps102 | Cg31-Pg1-Ps103 | Cg31-Pg1-Ps104 |
| Cg31-Pg1-Ps105 | Cg31-Pg1-Ps106 | Cg31-Pg1-Ps107 | Cg31-Pg1-Ps108 |
| Cg31-Pg1-Ps109 | Cg31-Pg1-Ps110 | Cg31-Pg1-Ps111 | Cg31-Pg1-Ps112 |
| Cg31-Pg1-Ps113 | Cg31-Pg1-Ps114 | Cg31-Pg1-Ps115 | Cg31-Pg1-Ps116 |
| Cg31-Pg1-Ps117 | Cg31-Pg1-Ps118 | Cg31-Pg1-Ps119 | Cg31-Pg1-Ps120 |
| Cg31-Pg1-Ps121 | Cg31-Pg1-Ps122 | Cg31-Pg1-Ps123 | Cg31-Pg1-Ps124 |
| Cg31-Pg1-Ps125 | Cg31-Pg1-Ps126 | Cg31-Pg1-Ps127 | Cg31-Pg1-Ps128 |
| Cg31-Pg1-Ps129 | Cg31-Pg1-Ps130 | Cg31-Pg1-Ps131 | Cg31-Pg1-Ps132 |
| Cg31-Pg1-Ps133 | Cg31-Pg1-Ps134 | Cg31-Pg1-Ps135 | Cg31-Pg1-Ps136 |
| Cg31-Pg1-Ps137 | Cg31-Pg1-Ps138 | Cg31-Pg1-Ps139 | Cg31-Pg1-Ps140 |
| Cg31-Pg1-Ps141 | Cg31-Pg1-Ps142 | Cg31-Pg1-Ps143 | Cg31-Pg1-Ps144 |
| Cg31-Pg1-Ps145 | Cg31-Pg1-Ps146 | Cg31-Pg1-Ps147 | Cg31-Pg1-Ps148 |
| Cg31-Pg1-Ps149 | Cg31-Pg1-Ps150 | Cg31-Pg1-Ps151 | Cg31-Pg1-Ps152 |
| Cg31-Pg1-Ps153 | Cg31-Pg1-Ps154 | Cg31-Pg1-Ps155 | Cg31-Pg1-Ps156 |
| Cg31-Pg1-Ps157 | Cg31-Pg1-Ps158 | Cg31-Pg1-Ps159 | Cg31-Pg1-Ps160 |
| Cg31-Pg1-Ps161 | Cg31-Pg1-Ps162 | Cg31-Pg1-Ps163 | Cg31-Pg1-Ps164 |
| Cg31-Pg1-Ps165 | Cg31-Pg1-Ps166 | Cg31-Pg1-Ps167 | Cg31-Pg1-Ps168 |
| Cg31-Pg1-Ps169 | Cg31-Pg1-Ps170 | Cg31-Pg1-Ps171 | Cg31-Pg1-Ps172 |
| Cg31-Pg1-Ps173 | Cg31-Pg1-Ps174 | Cg31-Pg1-Ps175 | Cg31-Pg1-Ps176 |
| Cg31-Pg1-Ps177 | Cg31-Pg1-Ps178 | Cg31-Pg1-Ps179 | Cg31-Pg1-Ps180 |

| | | | |
|---|---|---|---|
| Cg31–Pg1–Ps181 | Cg31–Pg1–Ps182 | Cg31–Pg1–Ps183 | Cg31–Pg1–Ps184 |
| Cg31–Pg1–Ps185 | Cg31–Pg1–Ps186 | Cg31–Pg1–Ps187 | Cg31–Pg1–Ps188 |
| Cg31–Pg1–Ps189 | Cg31–Pg1–Ps190 | Cg31–Pg1–Ps191 | Cg31–Pg1–Ps192 |
| Cg31–Pg1–Ps193 | Cg31–Pg1–Ps194 | Cg31–Pg1–Ps195 | Cg31–Pg1–Ps196 |
| Cg31–Pg1–Ps197 | Cg31–Pg1–Ps198 | Cg31–Pg1–Ps199 | Cg31–Pg1–Ps200 |
| Cg31–Pg1–Ps201 | Cg31–Pg1–Ps202 | Cg31–Pg1–Ps203 | Cg31–Pg1–Ps204 |
| Cg31–Pg1–Ps205 | Cg31–Pg1–Ps206 | Cg31–Pg1–Ps207 | Cg31–Pg1–Ps208 |
| Cg31–Pg1–Ps209 | Cg31–Pg1–Ps210 | Cg31–Pg1–Ps211 | Cg31–Pg1–Ps212 |
| Cg31–Pg1–Ps213 | Cg31–Pg1–Ps214 | Cg31–Pg1–Ps215 | Cg31–Pg1–Ps216 |
| Cg31–Pg1–Ps217 | Cg31–Pg1–Ps218 | Cg31–Pg1–Ps219 | Cg31–Pg1–Ps220 |
| Cg31–Pg1–Ps221 | Cg31–Pg1–Ps222 | Cg31–Pg1–Ps223 | Cg31–Pg1–Ps224 |
| Cg31–Pg1–Ps225 | Cg31–Pg1–Ps226 | Cg31–Pg1–Ps227 | Cg31–Pg1–Ps228 |
| Cg31–Pg1–Ps229 | Cg31–Pg1–Ps230 | Cg31–Pg1–Ps231 | Cg31–Pg1–Ps232 |
| Cg31–Pg1–Ps233 | Cg31–Pg1–Ps234 | Cg31–Pg1–Ps235 | Cg31–Pg1–Ps236 |
| Cg31–Pg1–Ps237 | Cg31–Pg1–Ps238 | Cg31–Pg1–Ps239 | Cg31–Pg1–Ps240 |
| Cg31–Pg1–Ps241 | Cg31–Pg1–Ps242 | Cg31–Pg1–Ps243 | |

| | | | |
|---|---|---|---|
| Cg32–Pg1–Ps1 | Cg32–Pg1–Ps2 | Cg32–Pg1–Ps3 | Cg32–Pg1–Ps4 |
| Cg32–Pg1–Ps5 | Cg32–Pg1–Ps6 | Cg32–Pg1–Ps7 | Cg32–Pg1–Ps8 |
| Cg32–Pg1–Ps9 | Cg32–Pg1–Ps10 | Cg32–Pg1–Ps11 | Cg32–Pg1–Ps12 |
| Cg32–Pg1–Ps13 | Cg32–Pg1–Ps14 | Cg32–Pg1–Ps15 | Cg32–Pg1–Ps16 |
| Cg32–Pg1–Ps17 | Cg32–Pg1–Ps18 | Cg32–Pg1–Ps19 | Cg32–Pg1–Ps20 |
| Cg32–Pg1–Ps21 | Cg32–Pg1–Ps22 | Cg32–Pg1–Ps23 | Cg32–Pg1–Ps24 |
| Cg32–Pg1–Ps25 | Cg32–Pg1–Ps26 | Cg32–Pg1–Ps27 | Cg32–Pg1–Ps28 |
| Cg32–Pg1–Ps29 | Cg32–Pg1–Ps30 | Cg32–Pg1–Ps31 | Cg32–Pg1–Ps32 |
| Cg32–Pg1–Ps33 | Cg32–Pg1–Ps34 | Cg32–Pg1–Ps35 | Cg32–Pg1–Ps36 |
| Cg32–Pg1–Ps37 | Cg32–Pg1–Ps38 | Cg32–Pg1–Ps39 | Cg32–Pg1–Ps40 |
| Cg32–Pg1–Ps41 | Cg32–Pg1–Ps42 | Cg32–Pg1–Ps43 | Cg32–Pg1–Ps44 |
| Cg32–Pg1–Ps45 | Cg32–Pg1–Ps46 | Cg32–Pg1–Ps47 | Cg32–Pg1–Ps48 |
| Cg32–Pg1–Ps49 | Cg32–Pg1–Ps50 | Cg32–Pg1–Ps51 | Cg32–Pg1–Ps52 |
| Cg32–Pg1–Ps53 | Cg32–Pg1–Ps54 | Cg32–Pg1–Ps55 | Cg32–Pg1–Ps56 |
| Cg32–Pg1–Ps57 | Cg32–Pg1–Ps58 | Cg32–Pg1–Ps59 | Cg32–Pg1–Ps60 |
| Cg32–Pg1–Ps61 | Cg32–Pg1–Ps62 | Cg32–Pg1–Ps63 | Cg32–Pg1–Ps64 |
| Cg32–Pg1–Ps65 | Cg32–Pg1–Ps66 | Cg32–Pg1–Ps67 | Cg32–Pg1–Ps68 |
| Cg32–Pg1–Ps69 | Cg32–Pg1–Ps70 | Cg32–Pg1–Ps71 | Cg32–Pg1–Ps72 |
| Cg32–Pg1–Ps73 | Cg32–Pg1–Ps74 | Cg32–Pg1–Ps75 | Cg32–Pg1–Ps76 |
| Cg32–Pg1–Ps77 | Cg32–Pg1–Ps78 | Cg32–Pg1–Ps79 | Cg32–Pg1–Ps80 |
| Cg32–Pg1–Ps81 | Cg32–Pg1–Ps82 | Cg32–Pg1–Ps83 | Cg32–Pg1–Ps84 |
| Cg32–Pg1–Ps85 | Cg32–Pg1–Ps86 | Cg32–Pg1–Ps87 | Cg32–Pg1–Ps88 |
| Cg32–Pg1–Ps89 | Cg32–Pg1–Ps90 | Cg32–Pg1–Ps91 | Cg32–Pg1–Ps92 |
| Cg32–Pg1–Ps93 | Cg32–Pg1–Ps94 | Cg32–Pg1–Ps95 | Cg32–Pg1–Ps96 |
| Cg32–Pg1–Ps97 | Cg32–Pg1–Ps98 | Cg32–Pg1–Ps99 | Cg32–Pg1–Ps100 |
| Cg32–Pg1–Ps101 | Cg32–Pg1–Ps102 | Cg32–Pg1–Ps103 | Cg32–Pg1–Ps104 |
| Cg32–Pg1–Ps105 | Cg32–Pg1–Ps106 | Cg32–Pg1–Ps107 | Cg32–Pg1–Ps108 |
| Cg32–Pg1–Ps109 | Cg32–Pg1–Ps110 | Cg32–Pg1–Ps111 | Cg32–Pg1–Ps112 |
| Cg32–Pg1–Ps113 | Cg32–Pg1–Ps114 | Cg32–Pg1–Ps115 | Cg32–Pg1–Ps116 |
| Cg32–Pg1–Ps117 | Cg32–Pg1–Ps118 | Cg32–Pg1–Ps119 | Cg32–Pg1–Ps120 |
| Cg32–Pg1–Ps121 | Cg32–Pg1–Ps122 | Cg32–Pg1–Ps123 | Cg32–Pg1–Ps124 |
| Cg32–Pg1–Ps125 | Cg32–Pg1–Ps126 | Cg32–Pg1–Ps127 | Cg32–Pg1–Ps128 |
| Cg32–Pg1–Ps129 | Cg32–Pg1–Ps130 | Cg32–Pg1–Ps131 | Cg32–Pg1–Ps132 |
| Cg32–Pg1–Ps133 | Cg32–Pg1–Ps134 | Cg32–Pg1–Ps135 | Cg32–Pg1–Ps136 |
| Cg32–Pg1–Ps137 | Cg32–Pg1–Ps138 | Cg32–Pg1–Ps139 | Cg32–Pg1–Ps140 |

| | | | |
|---|---|---|---|
| Cg32–Pg1–Ps141 | Cg32–Pg1–Ps142 | Cg32–Pg1–Ps143 | Cg32–Pg1–Ps144 |
| Cg32–Pg1–Ps145 | Cg32–Pg1–Ps146 | Cg32–Pg1–Ps147 | Cg32–Pg1–Ps148 |
| Cg32–Pg1–Ps149 | Cg32–Pg1–Ps150 | Cg32–Pg1–Ps151 | Cg32–Pg1–Ps152 |
| Cg32–Pg1–Ps153 | Cg32–Pg1–Ps154 | Cg32–Pg1–Ps155 | Cg32–Pg1–Ps156 |
| Cg32–Pg1–Ps157 | Cg32–Pg1–Ps158 | Cg32–Pg1–Ps159 | Cg32–Pg1–Ps160 |
| Cg32–Pg1–Ps161 | Cg32–Pg1–Ps162 | Cg32–Pg1–Ps163 | Cg32–Pg1–Ps164 |
| Cg32–Pg1–Ps165 | Cg32–Pg1–Ps166 | Cg32–Pg1–Ps167 | Cg32–Pg1–Ps168 |
| Cg32–Pg1–Ps169 | Cg32–Pg1–Ps170 | Cg32–Pg1–Ps171 | Cg32–Pg1–Ps172 |
| Cg32–Pg1–Ps173 | Cg32–Pg1–Ps174 | Cg32–Pg1–Ps175 | Cg32–Pg1–Ps176 |
| Cg32–Pg1–Ps177 | Cg32–Pg1–Ps178 | Cg32–Pg1–Ps179 | Cg32–Pg1–Ps180 |
| Cg32–Pg1–Ps181 | Cg32–Pg1–Ps182 | Cg32–Pg1–Ps183 | Cg32–Pg1–Ps184 |
| Cg32–Pg1–Ps185 | Cg32–Pg1–Ps186 | Cg32–Pg1–Ps187 | Cg32–Pg1–Ps188 |
| Cg32–Pg1–Ps189 | Cg32–Pg1–Ps190 | Cg32–Pg1–Ps191 | Cg32–Pg1–Ps192 |
| Cg32–Pg1–Ps193 | Cg32–Pg1–Ps194 | Cg32–Pg1–Ps195 | Cg32–Pg1–Ps196 |
| Cg32–Pg1–Ps197 | Cg32–Pg1–Ps198 | Cg32–Pg1–Ps199 | Cg32–Pg1–Ps200 |
| Cg32–Pg1–Ps201 | Cg32–Pg1–Ps202 | Cg32–Pg1–Ps203 | Cg32–Pg1–Ps204 |
| Cg32–Pg1–Ps205 | Cg32–Pg1–Ps206 | Cg32–Pg1–Ps207 | Cg32–Pg1–Ps208 |
| Cg32–Pg1–Ps209 | Cg32–Pg1–Ps210 | Cg32–Pg1–Ps211 | Cg32–Pg1–Ps212 |
| Cg32–Pg1–Ps213 | Cg32–Pg1–Ps214 | Cg32–Pg1–Ps215 | Cg32–Pg1–Ps216 |
| Cg32–Pg1–Ps217 | Cg32–Pg1–Ps218 | Cg32–Pg1–Ps219 | Cg32–Pg1–Ps220 |
| Cg32–Pg1–Ps221 | Cg32–Pg1–Ps222 | Cg32–Pg1–Ps223 | Cg32–Pg1–Ps224 |
| Cg32–Pg1–Ps225 | Cg32–Pg1–Ps226 | Cg32–Pg1–Ps227 | Cg32–Pg1–Ps228 |
| Cg32–Pg1–Ps229 | Cg32–Pg1–Ps230 | Cg32–Pg1–Ps231 | Cg32–Pg1–Ps232 |
| Cg32–Pg1–Ps233 | Cg32–Pg1–Ps234 | Cg32–Pg1–Ps235 | Cg32–Pg1–Ps236 |
| Cg32–Pg1–Ps237 | Cg32–Pg1–Ps238 | Cg32–Pg1–Ps239 | Cg32–Pg1–Ps240 |
| Cg32–Pg1–Ps241 | Cg32–Pg1–Ps242 | Cg32–Pg1–Ps243 | |

| | | | |
|---|---|---|---|
| Cg33–Pg1–Ps1 | Cg33–Pg1–Ps2 | Cg33–Pg1–Ps3 | Cg33–Pg1–Ps4 |
| Cg33–Pg1–Ps5 | Cg33–Pg1–Ps6 | Cg33–Pg1–Ps7 | Cg33–Pg1–Ps8 |
| Cg33–Pg1–Ps9 | Cg33–Pg1–Ps10 | Cg33–Pg1–Ps11 | Cg33–Pg1–Ps12 |
| Cg33–Pg1–Ps13 | Cg33–Pg1–Ps14 | Cg33–Pg1–Ps15 | Cg33–Pg1–Ps16 |
| Cg33–Pg1–Ps17 | Cg33–Pg1–Ps18 | Cg33–Pg1–Ps19 | Cg33–Pg1–Ps20 |
| Cg33–Pg1–Ps21 | Cg33–Pg1–Ps22 | Cg33–Pg1–Ps23 | Cg33–Pg1–Ps24 |
| Cg33–Pg1–Ps25 | Cg33–Pg1–Ps26 | Cg33–Pg1–Ps27 | Cg33–Pg1–Ps28 |
| Cg33–Pg1–Ps29 | Cg33–Pg1–Ps30 | Cg33–Pg1–Ps31 | Cg33–Pg1–Ps32 |
| Cg33–Pg1–Ps33 | Cg33–Pg1–Ps34 | Cg33–Pg1–Ps35 | Cg33–Pg1–Ps36 |
| Cg33–Pg1–Ps37 | Cg33–Pg1–Ps38 | Cg33–Pg1–Ps39 | Cg33–Pg1–Ps40 |
| Cg33–Pg1–Ps41 | Cg33–Pg1–Ps42 | Cg33–Pg1–Ps43 | Cg33–Pg1–Ps44 |
| Cg33–Pg1–Ps45 | Cg33–Pg1–Ps46 | Cg33–Pg1–Ps47 | Cg33–Pg1–Ps48 |
| Cg33–Pg1–Ps49 | Cg33–Pg1–Ps50 | Cg33–Pg1–Ps51 | Cg33–Pg1–Ps52 |
| Cg33–Pg1–Ps53 | Cg33–Pg1–Ps54 | Cg33–Pg1–Ps55 | Cg33–Pg1–Ps56 |
| Cg33–Pg1–Ps57 | Cg33–Pg1–Ps58 | Cg33–Pg1–Ps59 | Cg33–Pg1–Ps60 |
| Cg33–Pg1–Ps61 | Cg33–Pg1–Ps62 | Cg33–Pg1–Ps63 | Cg33–Pg1–Ps64 |
| Cg33–Pg1–Ps65 | Cg33–Pg1–Ps66 | Cg33–Pg1–Ps67 | Cg33–Pg1–Ps68 |
| Cg33–Pg1–Ps69 | Cg33–Pg1–Ps70 | Cg33–Pg1–Ps71 | Cg33–Pg1–Ps72 |
| Cg33–Pg1–Ps73 | Cg33–Pg1–Ps74 | Cg33–Pg1–Ps75 | Cg33–Pg1–Ps76 |
| Cg33–Pg1–Ps77 | Cg33–Pg1–Ps78 | Cg33–Pg1–Ps79 | Cg33–Pg1–Ps80 |
| Cg33–Pg1–Ps81 | Cg33–Pg1–Ps82 | Cg33–Pg1–Ps83 | Cg33–Pg1–Ps84 |
| Cg33–Pg1–Ps85 | Cg33–Pg1–Ps86 | Cg33–Pg1–Ps87 | Cg33–Pg1–Ps88 |
| Cg33–Pg1–Ps89 | Cg33–Pg1–Ps90 | Cg33–Pg1–Ps91 | Cg33–Pg1–Ps92 |
| Cg33–Pg1–Ps93 | Cg33–Pg1–Ps94 | Cg33–Pg1–Ps95 | Cg33–Pg1–Ps96 |
| Cg33–Pg1–Ps97 | Cg33–Pg1–Ps98 | Cg33–Pg1–Ps99 | Cg33–Pg1–Ps100 |

| | | | |
|---|---|---|---|
| Cg33–Pg1–Ps101 | Cg33–Pg1–Ps102 | Cg33–Pg1–Ps103 | Cg33–Pg1–Ps104 |
| Cg33–Pg1–Ps105 | Cg33–Pg1–Ps106 | Cg33–Pg1–Ps107 | Cg33–Pg1–Ps108 |
| Cg33–Pg1–Ps109 | Cg33–Pg1–Ps110 | Cg33–Pg1–Ps111 | Cg33–Pg1–Ps112 |
| Cg33–Pg1–Ps113 | Cg33–Pg1–Ps114 | Cg33–Pg1–Ps115 | Cg33–Pg1–Ps116 |
| Cg33–Pg1–Ps117 | Cg33–Pg1–Ps118 | Cg33–Pg1–Ps119 | Cg33–Pg1–Ps120 |
| Cg33–Pg1–Ps121 | Cg33–Pg1–Ps122 | Cg33–Pg1–Ps123 | Cg33–Pg1–Ps124 |
| Cg33–Pg1–Ps125 | Cg33–Pg1–Ps126 | Cg33–Pg1–Ps127 | Cg33–Pg1–Ps128 |
| Cg33–Pg1–Ps129 | Cg33–Pg1–Ps130 | Cg33–Pg1–Ps131 | Cg33–Pg1–Ps132 |
| Cg33–Pg1–Ps133 | Cg33–Pg1–Ps134 | Cg33–Pg1–Ps135 | Cg33–Pg1–Ps136 |
| Cg33–Pg1–Ps137 | Cg33–Pg1–Ps138 | Cg33–Pg1–Ps139 | Cg33–Pg1–Ps140 |
| Cg33–Pg1–Ps141 | Cg33–Pg1–Ps142 | Cg33–Pg1–Ps143 | Cg33–Pg1–Ps144 |
| Cg33–Pg1–Ps145 | Cg33–Pg1–Ps146 | Cg33–Pg1–Ps147 | Cg33–Pg1–Ps148 |
| Cg33–Pg1–Ps149 | Cg33–Pg1–Ps150 | Cg33–Pg1–Ps151 | Cg33–Pg1–Ps152 |
| Cg33–Pg1–Ps153 | Cg33–Pg1–Ps154 | Cg33–Pg1–Ps155 | Cg33–Pg1–Ps156 |
| Cg33–Pg1–Ps157 | Cg33–Pg1–Ps158 | Cg33–Pg1–Ps159 | Cg33–Pg1–Ps160 |
| Cg33–Pg1–Ps161 | Cg33–Pg1–Ps162 | Cg33–Pg1–Ps163 | Cg33–Pg1–Ps164 |
| Cg33–Pg1–Ps165 | Cg33–Pg1–Ps166 | Cg33–Pg1–Ps167 | Cg33–Pg1–Ps168 |
| Cg33–Pg1–Ps169 | Cg33–Pg1–Ps170 | Cg33–Pg1–Ps171 | Cg33–Pg1–Ps172 |
| Cg33–Pg1–Ps173 | Cg33–Pg1–Ps174 | Cg33–Pg1–Ps175 | Cg33–Pg1–Ps176 |
| Cg33–Pg1–Ps177 | Cg33–Pg1–Ps178 | Cg33–Pg1–Ps179 | Cg33–Pg1–Ps180 |
| Cg33–Pg1–Ps181 | Cg33–Pg1–Ps182 | Cg33–Pg1–Ps183 | Cg33–Pg1–Ps184 |
| Cg33–Pg1–Ps185 | Cg33–Pg1–Ps186 | Cg33–Pg1–Ps187 | Cg33–Pg1–Ps188 |
| Cg33–Pg1–Ps189 | Cg33–Pg1–Ps190 | Cg33–Pg1–Ps191 | Cg33–Pg1–Ps192 |
| Cg33–Pg1–Ps193 | Cg33–Pg1–Ps194 | Cg33–Pg1–Ps195 | Cg33–Pg1–Ps196 |
| Cg33–Pg1–Ps197 | Cg33–Pg1–Ps198 | Cg33–Pg1–Ps199 | Cg33–Pg1–Ps200 |
| Cg33–Pg1–Ps201 | Cg33–Pg1–Ps202 | Cg33–Pg1–Ps203 | Cg33–Pg1–Ps204 |
| Cg33–Pg1–Ps205 | Cg33–Pg1–Ps206 | Cg33–Pg1–Ps207 | Cg33–Pg1–Ps208 |
| Cg33–Pg1–Ps209 | Cg33–Pg1–Ps210 | Cg33–Pg1–Ps211 | Cg33–Pg1–Ps212 |
| Cg33–Pg1–Ps213 | Cg33–Pg1–Ps214 | Cg33–Pg1–Ps215 | Cg33–Pg1–Ps216 |
| Cg33–Pg1–Ps217 | Cg33–Pg1–Ps218 | Cg33–Pg1–Ps219 | Cg33–Pg1–Ps220 |
| Cg33–Pg1–Ps221 | Cg33–Pg1–Ps222 | Cg33–Pg1–Ps223 | Cg33–Pg1–Ps224 |
| Cg33–Pg1–Ps225 | Cg33–Pg1–Ps226 | Cg33–Pg1–Ps227 | Cg33–Pg1–Ps228 |
| Cg33–Pg1–Ps229 | Cg33–Pg1–Ps230 | Cg33–Pg1–Ps231 | Cg33–Pg1–Ps232 |
| Cg33–Pg1–Ps233 | Cg33–Pg1–Ps234 | Cg33–Pg1–Ps235 | Cg33–Pg1–Ps236 |
| Cg33–Pg1–Ps237 | Cg33–Pg1–Ps238 | Cg33–Pg1–Ps239 | Cg33–Pg1–Ps240 |
| Cg33–Pg1–Ps241 | Cg33–Pg1–Ps242 | Cg33–Pg1–Ps243 | |

| | | | |
|---|---|---|---|
| Cg34–Pg1–Ps1 | Cg34–Pg1–Ps2 | Cg34–Pg1–Ps3 | Cg34–Pg1–Ps4 |
| Cg34–Pg1–Ps5 | Cg34–Pg1–Ps6 | Cg34–Pg1–Ps7 | Cg34–Pg1–Ps8 |
| Cg34–Pg1–Ps9 | Cg34–Pg1–Ps10 | Cg34–Pg1–Ps11 | Cg34–Pg1–Ps12 |
| Cg34–Pg1–Ps13 | Cg34–Pg1–Ps14 | Cg34–Pg1–Ps15 | Cg34–Pg1–Ps16 |
| Cg34–Pg1–Ps17 | Cg34–Pg1–Ps18 | Cg34–Pg1–Ps19 | Cg34–Pg1–Ps20 |
| Cg34–Pg1–Ps21 | Cg34–Pg1–Ps22 | Cg34–Pg1–Ps23 | Cg34–Pg1–Ps24 |
| Cg34–Pg1–Ps25 | Cg34–Pg1–Ps26 | Cg34–Pg1–Ps27 | Cg34–Pg1–Ps28 |
| Cg34–Pg1–Ps29 | Cg34–Pg1–Ps30 | Cg34–Pg1–Ps31 | Cg34–Pg1–Ps32 |
| Cg34–Pg1–Ps33 | Cg34–Pg1–Ps34 | Cg34–Pg1–Ps35 | Cg34–Pg1–Ps36 |
| Cg34–Pg1–Ps37 | Cg34–Pg1–Ps38 | Cg34–Pg1–Ps39 | Cg34–Pg1–Ps40 |
| Cg34–Pg1–Ps41 | Cg34–Pg1–Ps42 | Cg34–Pg1–Ps43 | Cg34–Pg1–Ps44 |
| Cg34–Pg1–Ps45 | Cg34–Pg1–Ps46 | Cg34–Pg1–Ps47 | Cg34–Pg1–Ps48 |
| Cg34–Pg1–Ps49 | Cg34–Pg1–Ps50 | Cg34–Pg1–Ps51 | Cg34–Pg1–Ps52 |
| Cg34–Pg1–Ps53 | Cg34–Pg1–Ps54 | Cg34–Pg1–Ps55 | Cg34–Pg1–Ps56 |
| Cg34–Pg1–Ps57 | Cg34–Pg1–Ps58 | Cg34–Pg1–Ps59 | Cg34–Pg1–Ps60 |

| | | | |
|---|---|---|---|
| Cg34–Pg1–Ps61 | Cg34–Pg1–Ps62 | Cg34–Pg1–Ps63 | Cg34–Pg1–Ps64 |
| Cg34–Pg1–Ps65 | Cg34–Pg1–Ps66 | Cg34–Pg1–Ps67 | Cg34–Pg1–Ps68 |
| Cg34–Pg1–Ps69 | Cg34–Pg1–Ps70 | Cg34–Pg1–Ps71 | Cg34–Pg1–Ps72 |
| Cg34–Pg1–Ps73 | Cg34–Pg1–Ps74 | Cg34–Pg1–Ps75 | Cg34–Pg1–Ps76 |
| Cg34–Pg1–Ps77 | Cg34–Pg1–Ps78 | Cg34–Pg1–Ps79 | Cg34–Pg1–Ps80 |
| Cg34–Pg1–Ps81 | Cg34–Pg1–Ps82 | Cg34–Pg1–Ps83 | Cg34–Pg1–Ps84 |
| Cg34–Pg1–Ps85 | Cg34–Pg1–Ps86 | Cg34–Pg1–Ps87 | Cg34–Pg1–Ps88 |
| Cg34–Pg1–Ps89 | Cg34–Pg1–Ps90 | Cg34–Pg1–Ps91 | Cg34–Pg1–Ps92 |
| Cg34–Pg1–Ps93 | Cg34–Pg1–Ps94 | Cg34–Pg1–Ps95 | Cg34–Pg1–Ps96 |
| Cg34–Pg1–Ps97 | Cg34–Pg1–Ps98 | Cg34–Pg1–Ps99 | Cg34–Pg1–Ps100 |
| Cg34–Pg1–Ps101 | Cg34–Pg1–Ps102 | Cg34–Pg1–Ps103 | Cg34–Pg1–Ps104 |
| Cg34–Pg1–Ps105 | Cg34–Pg1–Ps106 | Cg34–Pg1–Ps107 | Cg34–Pg1–Ps108 |
| Cg34–Pg1–Ps109 | Cg34–Pg1–Ps110 | Cg34–Pg1–Ps111 | Cg34–Pg1–Ps112 |
| Cg34–Pg1–Ps113 | Cg34–Pg1–Ps114 | Cg34–Pg1–Ps115 | Cg34–Pg1–Ps116 |
| Cg34–Pg1–Ps117 | Cg34–Pg1–Ps118 | Cg34–Pg1–Ps119 | Cg34–Pg1–Ps120 |
| Cg34–Pg1–Ps121 | Cg34–Pg1–Ps122 | Cg34–Pg1–Ps123 | Cg34–Pg1–Ps124 |
| Cg34–Pg1–Ps125 | Cg34–Pg1–Ps126 | Cg34–Pg1–Ps127 | Cg34–Pg1–Ps128 |
| Cg34–Pg1–Ps129 | Cg34–Pg1–Ps130 | Cg34–Pg1–Ps131 | Cg34–Pg1–Ps132 |
| Cg34–Pg1–Ps133 | Cg34–Pg1–Ps134 | Cg34–Pg1–Ps135 | Cg34–Pg1–Ps136 |
| Cg34–Pg1–Ps137 | Cg34–Pg1–Ps138 | Cg34–Pg1–Ps139 | Cg34–Pg1–Ps140 |
| Cg34–Pg1–Ps141 | Cg34–Pg1–Ps142 | Cg34–Pg1–Ps143 | Cg34–Pg1–Ps144 |
| Cg34–Pg1–Ps145 | Cg34–Pg1–Ps146 | Cg34–Pg1–Ps147 | Cg34–Pg1–Ps148 |
| Cg34–Pg1–Ps149 | Cg34–Pg1–Ps150 | Cg34–Pg1–Ps151 | Cg34–Pg1–Ps152 |
| Cg34–Pg1–Ps153 | Cg34–Pg1–Ps154 | Cg34–Pg1–Ps155 | Cg34–Pg1–Ps156 |
| Cg34–Pg1–Ps157 | Cg34–Pg1–Ps158 | Cg34–Pg1–Ps159 | Cg34–Pg1–Ps160 |
| Cg34–Pg1–Ps161 | Cg34–Pg1–Ps162 | Cg34–Pg1–Ps163 | Cg34–Pg1–Ps164 |
| Cg34–Pg1–Ps165 | Cg34–Pg1–Ps166 | Cg34–Pg1–Ps167 | Cg34–Pg1–Ps168 |
| Cg34–Pg1–Ps169 | Cg34–Pg1–Ps170 | Cg34–Pg1–Ps171 | Cg34–Pg1–Ps172 |
| Cg34–Pg1–Ps173 | Cg34–Pg1–Ps174 | Cg34–Pg1–Ps175 | Cg34–Pg1–Ps176 |
| Cg34–Pg1–Ps177 | Cg34–Pg1–Ps178 | Cg34–Pg1–Ps179 | Cg34–Pg1–Ps180 |
| Cg34–Pg1–Ps181 | Cg34–Pg1–Ps182 | Cg34–Pg1–Ps183 | Cg34–Pg1–Ps184 |
| Cg34–Pg1–Ps185 | Cg34–Pg1–Ps186 | Cg34–Pg1–Ps187 | Cg34–Pg1–Ps188 |
| Cg34–Pg1–Ps189 | Cg34–Pg1–Ps190 | Cg34–Pg1–Ps191 | Cg34–Pg1–Ps192 |
| Cg34–Pg1–Ps193 | Cg34–Pg1–Ps194 | Cg34–Pg1–Ps195 | Cg34–Pg1–Ps196 |
| Cg34–Pg1–Ps197 | Cg34–Pg1–Ps198 | Cg34–Pg1–Ps199 | Cg34–Pg1–Ps200 |
| Cg34–Pg1–Ps201 | Cg34–Pg1–Ps202 | Cg34–Pg1–Ps203 | Cg34–Pg1–Ps204 |
| Cg34–Pg1–Ps205 | Cg34–Pg1–Ps206 | Cg34–Pg1–Ps207 | Cg34–Pg1–Ps208 |
| Cg34–Pg1–Ps209 | Cg34–Pg1–Ps210 | Cg34–Pg1–Ps211 | Cg34–Pg1–Ps212 |
| Cg34–Pg1–Ps213 | Cg34–Pg1–Ps214 | Cg34–Pg1–Ps215 | Cg34–Pg1–Ps216 |
| Cg34–Pg1–Ps217 | Cg34–Pg1–Ps218 | Cg34–Pg1–Ps219 | Cg34–Pg1–Ps220 |
| Cg34–Pg1–Ps221 | Cg34–Pg1–Ps222 | Cg34–Pg1–Ps223 | Cg34–Pg1–Ps224 |
| Cg34–Pg1–Ps225 | Cg34–Pg1–Ps226 | Cg34–Pg1–Ps227 | Cg34–Pg1–Ps228 |
| Cg34–Pg1–Ps229 | Cg34–Pg1–Ps230 | Cg34–Pg1–Ps231 | Cg34–Pg1–Ps232 |
| Cg34–Pg1–Ps233 | Cg34–Pg1–Ps234 | Cg34–Pg1–Ps235 | Cg34–Pg1–Ps236 |
| Cg34–Pg1–Ps237 | Cg34–Pg1–Ps238 | Cg34–Pg1–Ps239 | Cg34–Pg1–Ps240 |
| Cg34–Pg1–Ps241 | Cg34–Pg1–Ps242 | Cg34–Pg1–Ps243 | |

| | | | |
|---|---|---|---|
| Cg38–Pg1–Ps1 | Cg38–Pg1–Ps2 | Cg38–Pg1–Ps3 | Cg38–Pg1–Ps4 |
| Cg38–Pg1–Ps5 | Cg38–Pg1–Ps6 | Cg38–Pg1–Ps7 | Cg38–Pg1–Ps8 |
| Cg38–Pg1–Ps9 | Cg38–Pg1–Ps10 | Cg38–Pg1–Ps11 | Cg38–Pg1–Ps12 |
| Cg38–Pg1–Ps13 | Cg38–Pg1–Ps14 | Cg38–Pg1–Ps15 | Cg38–Pg1–Ps16 |
| Cg38–Pg1–Ps17 | Cg38–Pg1–Ps18 | Cg38–Pg1–Ps19 | Cg38–Pg1–Ps20 |

| | | | |
|---|---|---|---|
| Cg38-Pg1-Ps21 | Cg38-Pg1-Ps22 | Cg38-Pg1-Ps23 | Cg38-Pg1-Ps24 |
| Cg38-Pg1-Ps25 | Cg38-Pg1-Ps26 | Cg38-Pg1-Ps27 | Cg38-Pg1-Ps28 |
| Cg38-Pg1-Ps29 | Cg38-Pg1-Ps30 | Cg38-Pg1-Ps31 | Cg38-Pg1-Ps32 |
| Cg38-Pg1-Ps33 | Cg38-Pg1-Ps34 | Cg38-Pg1-Ps35 | Cg38-Pg1-Ps36 |
| Cg38-Pg1-Ps37 | Cg38-Pg1-Ps38 | Cg38-Pg1-Ps39 | Cg38-Pg1-Ps40 |
| Cg38-Pg1-Ps41 | Cg38-Pg1-Ps42 | Cg38-Pg1-Ps43 | Cg38-Pg1-Ps44 |
| Cg38-Pg1-Ps45 | Cg38-Pg1-Ps46 | Cg38-Pg1-Ps47 | Cg38-Pg1-Ps48 |
| Cg38-Pg1-Ps49 | Cg38-Pg1-Ps50 | Cg38-Pg1-Ps51 | Cg38-Pg1-Ps52 |
| Cg38-Pg1-Ps53 | Cg38-Pg1-Ps54 | Cg38-Pg1-Ps55 | Cg38-Pg1-Ps56 |
| Cg38-Pg1-Ps57 | Cg38-Pg1-Ps58 | Cg38-Pg1-Ps59 | Cg38-Pg1-Ps60 |
| Cg38-Pg1-Ps61 | Cg38-Pg1-Ps62 | Cg38-Pg1-Ps63 | Cg38-Pg1-Ps64 |
| Cg38-Pg1-Ps65 | Cg38-Pg1-Ps66 | Cg38-Pg1-Ps67 | Cg38-Pg1-Ps68 |
| Cg38-Pg1-Ps69 | Cg38-Pg1-Ps70 | Cg38-Pg1-Ps71 | Cg38-Pg1-Ps72 |
| Cg38-Pg1-Ps73 | Cg38-Pg1-Ps74 | Cg38-Pg1-Ps75 | Cg38-Pg1-Ps76 |
| Cg38-Pg1-Ps77 | Cg38-Pg1-Ps78 | Cg38-Pg1-Ps79 | Cg38-Pg1-Ps80 |
| Cg38-Pg1-Ps81 | Cg38-Pg1-Ps82 | Cg38-Pg1-Ps83 | Cg38-Pg1-Ps84 |
| Cg38-Pg1-Ps85 | Cg38-Pg1-Ps86 | Cg38-Pg1-Ps87 | Cg38-Pg1-Ps88 |
| Cg38-Pg1-Ps89 | Cg38-Pg1-Ps90 | Cg38-Pg1-Ps91 | Cg38-Pg1-Ps92 |
| Cg38-Pg1-Ps93 | Cg38-Pg1-Ps94 | Cg38-Pg1-Ps95 | Cg38-Pg1-Ps96 |
| Cg38-Pg1-Ps97 | Cg38-Pg1-Ps98 | Cg38-Pg1-Ps99 | Cg38-Pg1-Ps100 |
| Cg38-Pg1-Ps101 | Cg38-Pg1-Ps102 | Cg38-Pg1-Ps103 | Cg38-Pg1-Ps104 |
| Cg38-Pg1-Ps105 | Cg38-Pg1-Ps106 | Cg38-Pg1-Ps107 | Cg38-Pg1-Ps108 |
| Cg38-Pg1-Ps109 | Cg38-Pg1-Ps110 | Cg38-Pg1-Ps111 | Cg38-Pg1-Ps112 |
| Cg38-Pg1-Ps113 | Cg38-Pg1-Ps114 | Cg38-Pg1-Ps115 | Cg38-Pg1-Ps116 |
| Cg38-Pg1-Ps117 | Cg38-Pg1-Ps118 | Cg38-Pg1-Ps119 | Cg38-Pg1-Ps120 |
| Cg38-Pg1-Ps121 | Cg38-Pg1-Ps122 | Cg38-Pg1-Ps123 | Cg38-Pg1-Ps124 |
| Cg38-Pg1-Ps125 | Cg38-Pg1-Ps126 | Cg38-Pg1-Ps127 | Cg38-Pg1-Ps128 |
| Cg38-Pg1-Ps129 | Cg38-Pg1-Ps130 | Cg38-Pg1-Ps131 | Cg38-Pg1-Ps132 |
| Cg38-Pg1-Ps133 | Cg38-Pg1-Ps134 | Cg38-Pg1-Ps135 | Cg38-Pg1-Ps136 |
| Cg38-Pg1-Ps137 | Cg38-Pg1-Ps138 | Cg38-Pg1-Ps139 | Cg38-Pg1-Ps140 |
| Cg38-Pg1-Ps141 | Cg38-Pg1-Ps142 | Cg38-Pg1-Ps143 | Cg38-Pg1-Ps144 |
| Cg38-Pg1-Ps145 | Cg38-Pg1-Ps146 | Cg38-Pg1-Ps147 | Cg38-Pg1-Ps148 |
| Cg38-Pg1-Ps149 | Cg38-Pg1-Ps150 | Cg38-Pg1-Ps151 | Cg38-Pg1-Ps152 |
| Cg38-Pg1-Ps153 | Cg38-Pg1-Ps154 | Cg38-Pg1-Ps155 | Cg38-Pg1-Ps156 |
| Cg38-Pg1-Ps157 | Cg38-Pg1-Ps158 | Cg38-Pg1-Ps159 | Cg38-Pg1-Ps160 |
| Cg38-Pg1-Ps161 | Cg38-Pg1-Ps162 | Cg38-Pg1-Ps163 | Cg38-Pg1-Ps164 |
| Cg38-Pg1-Ps165 | Cg38-Pg1-Ps166 | Cg38-Pg1-Ps167 | Cg38-Pg1-Ps168 |
| Cg38-Pg1-Ps169 | Cg38-Pg1-Ps170 | Cg38-Pg1-Ps171 | Cg38-Pg1-Ps172 |
| Cg38-Pg1-Ps173 | Cg38-Pg1-Ps174 | Cg38-Pg1-Ps175 | Cg38-Pg1-Ps176 |
| Cg38-Pg1-Ps177 | Cg38-Pg1-Ps178 | Cg38-Pg1-Ps179 | Cg38-Pg1-Ps180 |
| Cg38-Pg1-Ps181 | Cg38-Pg1-Ps182 | Cg38-Pg1-Ps183 | Cg38-Pg1-Ps184 |
| Cg38-Pg1-Ps185 | Cg38-Pg1-Ps186 | Cg38-Pg1-Ps187 | Cg38-Pg1-Ps188 |
| Cg38-Pg1-Ps189 | Cg38-Pg1-Ps190 | Cg38-Pg1-Ps191 | Cg38-Pg1-Ps192 |
| Cg38-Pg1-Ps193 | Cg38-Pg1-Ps194 | Cg38-Pg1-Ps195 | Cg38-Pg1-Ps196 |
| Cg38-Pg1-Ps197 | Cg38-Pg1-Ps198 | Cg38-Pg1-Ps199 | Cg38-Pg1-Ps200 |
| Cg38-Pg1-Ps201 | Cg38-Pg1-Ps202 | Cg38-Pg1-Ps203 | Cg38-Pg1-Ps204 |
| Cg38-Pg1-Ps205 | Cg38-Pg1-Ps206 | Cg38-Pg1-Ps207 | Cg38-Pg1-Ps208 |
| Cg38-Pg1-Ps209 | Cg38-Pg1-Ps210 | Cg38-Pg1-Ps211 | Cg38-Pg1-Ps212 |
| Cg38-Pg1-Ps213 | Cg38-Pg1-Ps214 | Cg38-Pg1-Ps215 | Cg38-Pg1-Ps216 |
| Cg38-Pg1-Ps217 | Cg38-Pg1-Ps218 | Cg38-Pg1-Ps219 | Cg38-Pg1-Ps220 |
| Cg38-Pg1-Ps221 | Cg38-Pg1-Ps222 | Cg38-Pg1-Ps223 | Cg38-Pg1-Ps224 |
| Cg38-Pg1-Ps225 | Cg38-Pg1-Ps226 | Cg38-Pg1-Ps227 | Cg38-Pg1-Ps228 |

| | | | |
|---|---|---|---|
| Cg38–Pg1–Ps229 | Cg38–Pg1–Ps230 | Cg38–Pg1–Ps231 | Cg38–Pg1–Ps232 |
| Cg38–Pg1–Ps233 | Cg38–Pg1–Ps234 | Cg38–Pg1–Ps235 | Cg38–Pg1–Ps236 |
| Cg38–Pg1–Ps237 | Cg38–Pg1–Ps238 | Cg38–Pg1–Ps239 | Cg38–Pg1–Ps240 |
| Cg38–Pg1–Ps241 | Cg38–Pg1–Ps242 | Cg38–Pg1–Ps243 | |

| | | | |
|---|---|---|---|
| Cg39–Pg1–Ps1 | Cg39–Pg1–Ps2 | Cg39–Pg1–Ps3 | Cg39–Pg1–Ps4 |
| Cg39–Pg1–Ps5 | Cg39–Pg1–Ps6 | Cg39–Pg1–Ps7 | Cg39–Pg1–Ps8 |
| Cg39–Pg1–Ps9 | Cg39–Pg1–Ps10 | Cg39–Pg1–Ps11 | Cg39–Pg1–Ps12 |
| Cg39–Pg1–Ps13 | Cg39–Pg1–Ps14 | Cg39–Pg1–Ps15 | Cg39–Pg1–Ps16 |
| Cg39–Pg1–Ps17 | Cg39–Pg1–Ps18 | Cg39–Pg1–Ps19 | Cg39–Pg1–Ps20 |
| Cg39–Pg1–Ps21 | Cg39–Pg1–Ps22 | Cg39–Pg1–Ps23 | Cg39–Pg1–Ps24 |
| Cg39–Pg1–Ps25 | Cg39–Pg1–Ps26 | Cg39–Pg1–Ps27 | Cg39–Pg1–Ps28 |
| Cg39–Pg1–Ps29 | Cg39–Pg1–Ps30 | Cg39–Pg1–Ps31 | Cg39–Pg1–Ps32 |
| Cg39–Pg1–Ps33 | Cg39–Pg1–Ps34 | Cg39–Pg1–Ps35 | Cg39–Pg1–Ps36 |
| Cg39–Pg1–Ps37 | Cg39–Pg1–Ps38 | Cg39–Pg1–Ps39 | Cg39–Pg1–Ps40 |
| Cg39–Pg1–Ps41 | Cg39–Pg1–Ps42 | Cg39–Pg1–Ps43 | Cg39–Pg1–Ps44 |
| Cg39–Pg1–Ps45 | Cg39–Pg1–Ps46 | Cg39–Pg1–Ps47 | Cg39–Pg1–Ps48 |
| Cg39–Pg1–Ps49 | Cg39–Pg1–Ps50 | Cg39–Pg1–Ps51 | Cg39–Pg1–Ps52 |
| Cg39–Pg1–Ps53 | Cg39–Pg1–Ps54 | Cg39–Pg1–Ps55 | Cg39–Pg1–Ps56 |
| Cg39–Pg1–Ps57 | Cg39–Pg1–Ps58 | Cg39–Pg1–Ps59 | Cg39–Pg1–Ps60 |
| Cg39–Pg1–Ps61 | Cg39–Pg1–Ps62 | Cg39–Pg1–Ps63 | Cg39–Pg1–Ps64 |
| Cg39–Pg1–Ps65 | Cg39–Pg1–Ps66 | Cg39–Pg1–Ps67 | Cg39–Pg1–Ps68 |
| Cg39–Pg1–Ps69 | Cg39–Pg1–Ps70 | Cg39–Pg1–Ps71 | Cg39–Pg1–Ps72 |
| Cg39–Pg1–Ps73 | Cg39–Pg1–Ps74 | Cg39–Pg1–Ps75 | Cg39–Pg1–Ps76 |
| Cg39–Pg1–Ps77 | Cg39–Pg1–Ps78 | Cg39–Pg1–Ps79 | Cg39–Pg1–Ps80 |
| Cg39–Pg1–Ps81 | Cg39–Pg1–Ps82 | Cg39–Pg1–Ps83 | Cg39–Pg1–Ps84 |
| Cg39–Pg1–Ps85 | Cg39–Pg1–Ps86 | Cg39–Pg1–Ps87 | Cg39–Pg1–Ps88 |
| Cg39–Pg1–Ps89 | Cg39–Pg1–Ps90 | Cg39–Pg1–Ps91 | Cg39–Pg1–Ps92 |
| Cg39–Pg1–Ps93 | Cg39–Pg1–Ps94 | Cg39–Pg1–Ps95 | Cg39–Pg1–Ps96 |
| Cg39–Pg1–Ps97 | Cg39–Pg1–Ps98 | Cg39–Pg1–Ps99 | Cg39–Pg1–Ps100 |
| Cg39–Pg1–Ps101 | Cg39–Pg1–Ps102 | Cg39–Pg1–Ps103 | Cg39–Pg1–Ps104 |
| Cg39–Pg1–Ps105 | Cg39–Pg1–Ps106 | Cg39–Pg1–Ps107 | Cg39–Pg1–Ps108 |
| Cg39–Pg1–Ps109 | Cg39–Pg1–Ps110 | Cg39–Pg1–Ps111 | Cg39–Pg1–Ps112 |
| Cg39–Pg1–Ps113 | Cg39–Pg1–Ps114 | Cg39–Pg1–Ps115 | Cg39–Pg1–Ps116 |
| Cg39–Pg1–Ps117 | Cg39–Pg1–Ps118 | Cg39–Pg1–Ps119 | Cg39–Pg1–Ps120 |
| Cg39–Pg1–Ps121 | Cg39–Pg1–Ps122 | Cg39–Pg1–Ps123 | Cg39–Pg1–Ps124 |
| Cg39–Pg1–Ps125 | Cg39–Pg1–Ps126 | Cg39–Pg1–Ps127 | Cg39–Pg1–Ps128 |
| Cg39–Pg1–Ps129 | Cg39–Pg1–Ps130 | Cg39–Pg1–Ps131 | Cg39–Pg1–Ps132 |
| Cg39–Pg1–Ps133 | Cg39–Pg1–Ps134 | Cg39–Pg1–Ps135 | Cg39–Pg1–Ps136 |
| Cg39–Pg1–Ps137 | Cg39–Pg1–Ps138 | Cg39–Pg1–Ps139 | Cg39–Pg1–Ps140 |
| Cg39–Pg1–Ps141 | Cg39–Pg1–Ps142 | Cg39–Pg1–Ps143 | Cg39–Pg1–Ps144 |
| Cg39–Pg1–Ps145 | Cg39–Pg1–Ps146 | Cg39–Pg1–Ps147 | Cg39–Pg1–Ps148 |
| Cg39–Pg1–Ps149 | Cg39–Pg1–Ps150 | Cg39–Pg1–Ps151 | Cg39–Pg1–Ps152 |
| Cg39–Pg1–Ps153 | Cg39–Pg1–Ps154 | Cg39–Pg1–Ps155 | Cg39–Pg1–Ps156 |
| Cg39–Pg1–Ps157 | Cg39–Pg1–Ps158 | Cg39–Pg1–Ps159 | Cg39–Pg1–Ps160 |
| Cg39–Pg1–Ps161 | Cg39–Pg1–Ps162 | Cg39–Pg1–Ps163 | Cg39–Pg1–Ps164 |
| Cg39–Pg1–Ps165 | Cg39–Pg1–Ps166 | Cg39–Pg1–Ps167 | Cg39–Pg1–Ps168 |
| Cg39–Pg1–Ps169 | Cg39–Pg1–Ps170 | Cg39–Pg1–Ps171 | Cg39–Pg1–Ps172 |
| Cg39–Pg1–Ps173 | Cg39–Pg1–Ps174 | Cg39–Pg1–Ps175 | Cg39–Pg1–Ps176 |
| Cg39–Pg1–Ps177 | Cg39–Pg1–Ps178 | Cg39–Pg1–Ps179 | Cg39–Pg1–Ps180 |
| Cg39–Pg1–Ps181 | Cg39–Pg1–Ps182 | Cg39–Pg1–Ps183 | Cg39–Pg1–Ps184 |
| Cg39–Pg1–Ps185 | Cg39–Pg1–Ps186 | Cg39–Pg1–Ps187 | Cg39–Pg1–Ps188 |

| | | | |
|---|---|---|---|
| Cg39–Pg1–Ps189 | Cg39–Pg1–Ps190 | Cg39–Pg1–Ps191 | Cg39–Pg1–Ps192 |
| Cg39–Pg1–Ps193 | Cg39–Pg1–Ps194 | Cg39–Pg1–Ps195 | Cg39–Pg1–Ps196 |
| Cg39–Pg1–Ps197 | Cg39–Pg1–Ps198 | Cg39–Pg1–Ps199 | Cg39–Pg1–Ps200 |
| Cg39–Pg1–Ps201 | Cg39–Pg1–Ps202 | Cg39–Pg1–Ps203 | Cg39–Pg1–Ps204 |
| Cg39–Pg1–Ps205 | Cg39–Pg1–Ps206 | Cg39–Pg1–Ps207 | Cg39–Pg1–Ps208 |
| Cg39–Pg1–Ps209 | Cg39–Pg1–Ps210 | Cg39–Pg1–Ps211 | Cg39–Pg1–Ps212 |
| Cg39–Pg1–Ps213 | Cg39–Pg1–Ps214 | Cg39–Pg1–Ps215 | Cg39–Pg1–Ps216 |
| Cg39–Pg1–Ps217 | Cg39–Pg1–Ps218 | Cg39–Pg1–Ps219 | Cg39–Pg1–Ps220 |
| Cg39–Pg1–Ps221 | Cg39–Pg1–Ps222 | Cg39–Pg1–Ps223 | Cg39–Pg1–Ps224 |
| Cg39–Pg1–Ps225 | Cg39–Pg1–Ps226 | Cg39–Pg1–Ps227 | Cg39–Pg1–Ps228 |
| Cg39–Pg1–Ps229 | Cg39–Pg1–Ps230 | Cg39–Pg1–Ps231 | Cg39–Pg1–Ps232 |
| Cg39–Pg1–Ps233 | Cg39–Pg1–Ps234 | Cg39–Pg1–Ps235 | Cg39–Pg1–Ps236 |
| Cg39–Pg1–Ps237 | Cg39–Pg1–Ps238 | Cg39–Pg1–Ps239 | Cg39–Pg1–Ps240 |
| Cg39–Pg1–Ps241 | Cg39–Pg1–Ps242 | Cg39–Pg1–Ps243 | |

| | | | |
|---|---|---|---|
| Cg41–Pg1–Ps1 | Cg41–Pg1–Ps2 | Cg41–Pg1–Ps3 | Cg41–Pg1–Ps4 |
| Cg41–Pg1–Ps5 | Cg41–Pg1–Ps6 | Cg41–Pg1–Ps7 | Cg41–Pg1–Ps8 |
| Cg41–Pg1–Ps9 | Cg41–Pg1–Ps10 | Cg41–Pg1–Ps11 | Cg41–Pg1–Ps12 |
| Cg41–Pg1–Ps13 | Cg41–Pg1–Ps14 | Cg41–Pg1–Ps15 | Cg41–Pg1–Ps16 |
| Cg41–Pg1–Ps17 | Cg41–Pg1–Ps18 | Cg41–Pg1–Ps19 | Cg41–Pg1–Ps20 |
| Cg41–Pg1–Ps21 | Cg41–Pg1–Ps22 | Cg41–Pg1–Ps23 | Cg41–Pg1–Ps24 |
| Cg41–Pg1–Ps25 | Cg41–Pg1–Ps26 | Cg41–Pg1–Ps27 | Cg41–Pg1–Ps28 |
| Cg41–Pg1–Ps29 | Cg41–Pg1–Ps30 | Cg41–Pg1–Ps31 | Cg41–Pg1–Ps32 |
| Cg41–Pg1–Ps33 | Cg41–Pg1–Ps34 | Cg41–Pg1–Ps35 | Cg41–Pg1–Ps36 |
| Cg41–Pg1–Ps37 | Cg41–Pg1–Ps38 | Cg41–Pg1–Ps39 | Cg41–Pg1–Ps40 |
| Cg41–Pg1–Ps41 | Cg41–Pg1–Ps42 | Cg41–Pg1–Ps43 | Cg41–Pg1–Ps44 |
| Cg41–Pg1–Ps45 | Cg41–Pg1–Ps46 | Cg41–Pg1–Ps47 | Cg41–Pg1–Ps48 |
| Cg41–Pg1–Ps49 | Cg41–Pg1–Ps50 | Cg41–Pg1–Ps51 | Cg41–Pg1–Ps52 |
| Cg41–Pg1–Ps53 | Cg41–Pg1–Ps54 | Cg41–Pg1–Ps55 | Cg41–Pg1–Ps56 |
| Cg41–Pg1–Ps57 | Cg41–Pg1–Ps58 | Cg41–Pg1–Ps59 | Cg41–Pg1–Ps60 |
| Cg41–Pg1–Ps61 | Cg41–Pg1–Ps62 | Cg41–Pg1–Ps63 | Cg41–Pg1–Ps64 |
| Cg41–Pg1–Ps65 | Cg41–Pg1–Ps66 | Cg41–Pg1–Ps67 | Cg41–Pg1–Ps68 |
| Cg41–Pg1–Ps69 | Cg41–Pg1–Ps70 | Cg41–Pg1–Ps71 | Cg41–Pg1–Ps72 |
| Cg41–Pg1–Ps73 | Cg41–Pg1–Ps74 | Cg41–Pg1–Ps75 | Cg41–Pg1–Ps76 |
| Cg41–Pg1–Ps77 | Cg41–Pg1–Ps78 | Cg41–Pg1–Ps79 | Cg41–Pg1–Ps80 |
| Cg41–Pg1–Ps81 | Cg41–Pg1–Ps82 | Cg41–Pg1–Ps83 | Cg41–Pg1–Ps84 |
| Cg41–Pg1–Ps85 | Cg41–Pg1–Ps86 | Cg41–Pg1–Ps87 | Cg41–Pg1–Ps88 |
| Cg41–Pg1–Ps89 | Cg41–Pg1–Ps90 | Cg41–Pg1–Ps91 | Cg41–Pg1–Ps92 |
| Cg41–Pg1–Ps93 | Cg41–Pg1–Ps94 | Cg41–Pg1–Ps95 | Cg41–Pg1–Ps96 |
| Cg41–Pg1–Ps97 | Cg41–Pg1–Ps98 | Cg41–Pg1–Ps99 | Cg41–Pg1–Ps100 |
| Cg41–Pg1–Ps101 | Cg41–Pg1–Ps102 | Cg41–Pg1–Ps103 | Cg41–Pg1–Ps104 |
| Cg41–Pg1–Ps105 | Cg41–Pg1–Ps106 | Cg41–Pg1–Ps107 | Cg41–Pg1–Ps108 |
| Cg41–Pg1–Ps109 | Cg41–Pg1–Ps110 | Cg41–Pg1–Ps111 | Cg41–Pg1–Ps112 |
| Cg41–Pg1–Ps113 | Cg41–Pg1–Ps114 | Cg41–Pg1–Ps115 | Cg41–Pg1–Ps116 |
| Cg41–Pg1–Ps117 | Cg41–Pg1–Ps118 | Cg41–Pg1–Ps119 | Cg41–Pg1–Ps120 |
| Cg41–Pg1–Ps121 | Cg41–Pg1–Ps122 | Cg41–Pg1–Ps123 | Cg41–Pg1–Ps124 |
| Cg41–Pg1–Ps125 | Cg41–Pg1–Ps126 | Cg41–Pg1–Ps127 | Cg41–Pg1–Ps128 |
| Cg41–Pg1–Ps129 | Cg41–Pg1–Ps130 | Cg41–Pg1–Ps131 | Cg41–Pg1–Ps132 |
| Cg41–Pg1–Ps133 | Cg41–Pg1–Ps134 | Cg41–Pg1–Ps135 | Cg41–Pg1–Ps136 |
| Cg41–Pg1–Ps137 | Cg41–Pg1–Ps138 | Cg41–Pg1–Ps139 | Cg41–Pg1–Ps140 |
| Cg41–Pg1–Ps141 | Cg41–Pg1–Ps142 | Cg41–Pg1–Ps143 | Cg41–Pg1–Ps144 |
| Cg41–Pg1–Ps145 | Cg41–Pg1–Ps146 | Cg41–Pg1–Ps147 | Cg41–Pg1–Ps148 |

| | | | |
|---|---|---|---|
| Cg41–Pg1–Ps149 | Cg41–Pg1–Ps150 | Cg41–Pg1–Ps151 | Cg41–Pg1–Ps152 |
| Cg41–Pg1–Ps153 | Cg41–Pg1–Ps154 | Cg41–Pg1–Ps155 | Cg41–Pg1–Ps156 |
| Cg41–Pg1–Ps157 | Cg41–Pg1–Ps158 | Cg41–Pg1–Ps159 | Cg41–Pg1–Ps160 |
| Cg41–Pg1–Ps161 | Cg41–Pg1–Ps162 | Cg41–Pg1–Ps163 | Cg41–Pg1–Ps164 |
| Cg41–Pg1–Ps165 | Cg41–Pg1–Ps166 | Cg41–Pg1–Ps167 | Cg41–Pg1–Ps168 |
| Cg41–Pg1–Ps169 | Cg41–Pg1–Ps170 | Cg41–Pg1–Ps171 | Cg41–Pg1–Ps172 |
| Cg41–Pg1–Ps173 | Cg41–Pg1–Ps174 | Cg41–Pg1–Ps175 | Cg41–Pg1–Ps176 |
| Cg41–Pg1–Ps177 | Cg41–Pg1–Ps178 | Cg41–Pg1–Ps179 | Cg41–Pg1–Ps180 |
| Cg41–Pg1–Ps181 | Cg41–Pg1–Ps182 | Cg41–Pg1–Ps183 | Cg41–Pg1–Ps184 |
| Cg41–Pg1–Ps185 | Cg41–Pg1–Ps186 | Cg41–Pg1–Ps187 | Cg41–Pg1–Ps188 |
| Cg41–Pg1–Ps189 | Cg41–Pg1–Ps190 | Cg41–Pg1–Ps191 | Cg41–Pg1–Ps192 |
| Cg41–Pg1–Ps193 | Cg41–Pg1–Ps194 | Cg41–Pg1–Ps195 | Cg41–Pg1–Ps196 |
| Cg41–Pg1–Ps197 | Cg41–Pg1–Ps198 | Cg41–Pg1–Ps199 | Cg41–Pg1–Ps200 |
| Cg41–Pg1–Ps201 | Cg41–Pg1–Ps202 | Cg41–Pg1–Ps203 | Cg41–Pg1–Ps204 |
| Cg41–Pg1–Ps205 | Cg41–Pg1–Ps206 | Cg41–Pg1–Ps207 | Cg41–Pg1–Ps208 |
| Cg41–Pg1–Ps209 | Cg41–Pg1–Ps210 | Cg41–Pg1–Ps211 | Cg41–Pg1–Ps212 |
| Cg41–Pg1–Ps213 | Cg41–Pg1–Ps214 | Cg41–Pg1–Ps215 | Cg41–Pg1–Ps216 |
| Cg41–Pg1–Ps217 | Cg41–Pg1–Ps218 | Cg41–Pg1–Ps219 | Cg41–Pg1–Ps220 |
| Cg41–Pg1–Ps221 | Cg41–Pg1–Ps222 | Cg41–Pg1–Ps223 | Cg41–Pg1–Ps224 |
| Cg41–Pg1–Ps225 | Cg41–Pg1–Ps226 | Cg41–Pg1–Ps227 | Cg41–Pg1–Ps228 |
| Cg41–Pg1–Ps229 | Cg41–Pg1–Ps230 | Cg41–Pg1–Ps231 | Cg41–Pg1–Ps232 |
| Cg41–Pg1–Ps233 | Cg41–Pg1–Ps234 | Cg41–Pg1–Ps235 | Cg41–Pg1–Ps236 |
| Cg41–Pg1–Ps237 | Cg41–Pg1–Ps238 | Cg41–Pg1–Ps239 | Cg41–Pg1–Ps240 |
| Cg41–Pg1–Ps241 | Cg41–Pg1–Ps242 | Cg41–Pg1–Ps243 | |

| | | | |
|---|---|---|---|
| Cg42–Pg1–Ps1 | Cg42–Pg1–Ps2 | Cg42–Pg1–Ps3 | Cg42–Pg1–Ps4 |
| Cg42–Pg1–Ps5 | Cg42–Pg1–Ps6 | Cg42–Pg1–Ps7 | Cg42–Pg1–Ps8 |
| Cg42–Pg1–Ps9 | Cg42–Pg1–Ps10 | Cg42–Pg1–Ps11 | Cg42–Pg1–Ps12 |
| Cg42–Pg1–Ps13 | Cg42–Pg1–Ps14 | Cg42–Pg1–Ps15 | Cg42–Pg1–Ps16 |
| Cg42–Pg1–Ps17 | Cg42–Pg1–Ps18 | Cg42–Pg1–Ps19 | Cg42–Pg1–Ps20 |
| Cg42–Pg1–Ps21 | Cg42–Pg1–Ps22 | Cg42–Pg1–Ps23 | Cg42–Pg1–Ps24 |
| Cg42–Pg1–Ps25 | Cg42–Pg1–Ps26 | Cg42–Pg1–Ps27 | Cg42–Pg1–Ps28 |
| Cg42–Pg1–Ps29 | Cg42–Pg1–Ps30 | Cg42–Pg1–Ps31 | Cg42–Pg1–Ps32 |
| Cg42–Pg1–Ps33 | Cg42–Pg1–Ps34 | Cg42–Pg1–Ps35 | Cg42–Pg1–Ps36 |
| Cg42–Pg1–Ps37 | Cg42–Pg1–Ps38 | Cg42–Pg1–Ps39 | Cg42–Pg1–Ps40 |
| Cg42–Pg1–Ps41 | Cg42–Pg1–Ps42 | Cg42–Pg1–Ps43 | Cg42–Pg1–Ps44 |
| Cg42–Pg1–Ps45 | Cg42–Pg1–Ps46 | Cg42–Pg1–Ps47 | Cg42–Pg1–Ps48 |
| Cg42–Pg1–Ps49 | Cg42–Pg1–Ps50 | Cg42–Pg1–Ps51 | Cg42–Pg1–Ps52 |
| Cg42–Pg1–Ps53 | Cg42–Pg1–Ps54 | Cg42–Pg1–Ps55 | Cg42–Pg1–Ps56 |
| Cg42–Pg1–Ps57 | Cg42–Pg1–Ps58 | Cg42–Pg1–Ps59 | Cg42–Pg1–Ps60 |
| Cg42–Pg1–Ps61 | Cg42–Pg1–Ps62 | Cg42–Pg1–Ps63 | Cg42–Pg1–Ps64 |
| Cg42–Pg1–Ps65 | Cg42–Pg1–Ps66 | Cg42–Pg1–Ps67 | Cg42–Pg1–Ps68 |
| Cg42–Pg1–Ps69 | Cg42–Pg1–Ps70 | Cg42–Pg1–Ps71 | Cg42–Pg1–Ps72 |
| Cg42–Pg1–Ps73 | Cg42–Pg1–Ps74 | Cg42–Pg1–Ps75 | Cg42–Pg1–Ps76 |
| Cg42–Pg1–Ps77 | Cg42–Pg1–Ps78 | Cg42–Pg1–Ps79 | Cg42–Pg1–Ps80 |
| Cg42–Pg1–Ps81 | Cg42–Pg1–Ps82 | Cg42–Pg1–Ps83 | Cg42–Pg1–Ps84 |
| Cg42–Pg1–Ps85 | Cg42–Pg1–Ps86 | Cg42–Pg1–Ps87 | Cg42–Pg1–Ps88 |
| Cg42–Pg1–Ps89 | Cg42–Pg1–Ps90 | Cg42–Pg1–Ps91 | Cg42–Pg1–Ps92 |
| Cg42–Pg1–Ps93 | Cg42–Pg1–Ps94 | Cg42–Pg1–Ps95 | Cg42–Pg1–Ps96 |
| Cg42–Pg1–Ps97 | Cg42–Pg1–Ps98 | Cg42–Pg1–Ps99 | Cg42–Pg1–Ps100 |
| Cg42–Pg1–Ps101 | Cg42–Pg1–Ps102 | Cg42–Pg1–Ps103 | Cg42–Pg1–Ps104 |
| Cg42–Pg1–Ps105 | Cg42–Pg1–Ps106 | Cg42–Pg1–Ps107 | Cg42–Pg1–Ps108 |

| | | | |
|---|---|---|---|
| Cg42–Pg1–Ps109 | Cg42–Pg1–Ps110 | Cg42–Pg1–Ps111 | Cg42–Pg1–Ps112 |
| Cg42–Pg1–Ps113 | Cg42–Pg1–Ps114 | Cg42–Pg1–Ps115 | Cg42–Pg1–Ps116 |
| Cg42–Pg1–Ps117 | Cg42–Pg1–Ps118 | Cg42–Pg1–Ps119 | Cg42–Pg1–Ps120 |
| Cg42–Pg1–Ps121 | Cg42–Pg1–Ps122 | Cg42–Pg1–Ps123 | Cg42–Pg1–Ps124 |
| Cg42–Pg1–Ps125 | Cg42–Pg1–Ps126 | Cg42–Pg1–Ps127 | Cg42–Pg1–Ps128 |
| Cg42–Pg1–Ps129 | Cg42–Pg1–Ps130 | Cg42–Pg1–Ps131 | Cg42–Pg1–Ps132 |
| Cg42–Pg1–Ps133 | Cg42–Pg1–Ps134 | Cg42–Pg1–Ps135 | Cg42–Pg1–Ps136 |
| Cg42–Pg1–Ps137 | Cg42–Pg1–Ps138 | Cg42–Pg1–Ps139 | Cg42–Pg1–Ps140 |
| Cg42–Pg1–Ps141 | Cg42–Pg1–Ps142 | Cg42–Pg1–Ps143 | Cg42–Pg1–Ps144 |
| Cg42–Pg1–Ps145 | Cg42–Pg1–Ps146 | Cg42–Pg1–Ps147 | Cg42–Pg1–Ps148 |
| Cg42–Pg1–Ps149 | Cg42–Pg1–Ps150 | Cg42–Pg1–Ps151 | Cg42–Pg1–Ps152 |
| Cg42–Pg1–Ps153 | Cg42–Pg1–Ps154 | Cg42–Pg1–Ps155 | Cg42–Pg1–Ps156 |
| Cg42–Pg1–Ps157 | Cg42–Pg1–Ps158 | Cg42–Pg1–Ps159 | Cg42–Pg1–Ps160 |
| Cg42–Pg1–Ps161 | Cg42–Pg1–Ps162 | Cg42–Pg1–Ps163 | Cg42–Pg1–Ps164 |
| Cg42–Pg1–Ps165 | Cg42–Pg1–Ps166 | Cg42–Pg1–Ps167 | Cg42–Pg1–Ps168 |
| Cg42–Pg1–Ps169 | Cg42–Pg1–Ps170 | Cg42–Pg1–Ps171 | Cg42–Pg1–Ps172 |
| Cg42–Pg1–Ps173 | Cg42–Pg1–Ps174 | Cg42–Pg1–Ps175 | Cg42–Pg1–Ps176 |
| Cg42–Pg1–Ps177 | Cg42–Pg1–Ps178 | Cg42–Pg1–Ps179 | Cg42–Pg1–Ps180 |
| Cg42–Pg1–Ps181 | Cg42–Pg1–Ps182 | Cg42–Pg1–Ps183 | Cg42–Pg1–Ps184 |
| Cg42–Pg1–Ps185 | Cg42–Pg1–Ps186 | Cg42–Pg1–Ps187 | Cg42–Pg1–Ps188 |
| Cg42–Pg1–Ps189 | Cg42–Pg1–Ps190 | Cg42–Pg1–Ps191 | Cg42–Pg1–Ps192 |
| Cg42–Pg1–Ps193 | Cg42–Pg1–Ps194 | Cg42–Pg1–Ps195 | Cg42–Pg1–Ps196 |
| Cg42–Pg1–Ps197 | Cg42–Pg1–Ps198 | Cg42–Pg1–Ps199 | Cg42–Pg1–Ps200 |
| Cg42–Pg1–Ps201 | Cg42–Pg1–Ps202 | Cg42–Pg1–Ps203 | Cg42–Pg1–Ps204 |
| Cg42–Pg1–Ps205 | Cg42–Pg1–Ps206 | Cg42–Pg1–Ps207 | Cg42–Pg1–Ps208 |
| Cg42–Pg1–Ps209 | Cg42–Pg1–Ps210 | Cg42–Pg1–Ps211 | Cg42–Pg1–Ps212 |
| Cg42–Pg1–Ps213 | Cg42–Pg1–Ps214 | Cg42–Pg1–Ps215 | Cg42–Pg1–Ps216 |
| Cg42–Pg1–Ps217 | Cg42–Pg1–Ps218 | Cg42–Pg1–Ps219 | Cg42–Pg1–Ps220 |
| Cg42–Pg1–Ps221 | Cg42–Pg1–Ps222 | Cg42–Pg1–Ps223 | Cg42–Pg1–Ps224 |
| Cg42–Pg1–Ps225 | Cg42–Pg1–Ps226 | Cg42–Pg1–Ps227 | Cg42–Pg1–Ps228 |
| Cg42–Pg1–Ps229 | Cg42–Pg1–Ps230 | Cg42–Pg1–Ps231 | Cg42–Pg1–Ps232 |
| Cg42–Pg1–Ps233 | Cg42–Pg1–Ps234 | Cg42–Pg1–Ps235 | Cg42–Pg1–Ps236 |
| Cg42–Pg1–Ps237 | Cg42–Pg1–Ps238 | Cg42–Pg1–Ps239 | Cg42–Pg1–Ps240 |
| Cg42–Pg1–Ps241 | Cg42–Pg1–Ps242 | Cg42–Pg1–Ps243 | |

| | | | |
|---|---|---|---|
| Cg44–Pg1–Ps1 | Cg44–Pg1–Ps2 | Cg44–Pg1–Ps3 | Cg44–Pg1–Ps4 |
| Cg44–Pg1–Ps5 | Cg44–Pg1–Ps6 | Cg44–Pg1–Ps7 | Cg44–Pg1–Ps8 |
| Cg44–Pg1–Ps9 | Cg44–Pg1–Ps10 | Cg44–Pg1–Ps11 | Cg44–Pg1–Ps12 |
| Cg44–Pg1–Ps13 | Cg44–Pg1–Ps14 | Cg44–Pg1–Ps15 | Cg44–Pg1–Ps16 |
| Cg44–Pg1–Ps17 | Cg44–Pg1–Ps18 | Cg44–Pg1–Ps19 | Cg44–Pg1–Ps20 |
| Cg44–Pg1–Ps21 | Cg44–Pg1–Ps22 | Cg44–Pg1–Ps23 | Cg44–Pg1–Ps24 |
| Cg44–Pg1–Ps25 | Cg44–Pg1–Ps26 | Cg44–Pg1–Ps27 | Cg44–Pg1–Ps28 |
| Cg44–Pg1–Ps29 | Cg44–Pg1–Ps30 | Cg44–Pg1–Ps31 | Cg44–Pg1–Ps32 |
| Cg44–Pg1–Ps33 | Cg44–Pg1–Ps34 | Cg44–Pg1–Ps35 | Cg44–Pg1–Ps36 |
| Cg44–Pg1–Ps37 | Cg44–Pg1–Ps38 | Cg44–Pg1–Ps39 | Cg44–Pg1–Ps40 |
| Cg44–Pg1–Ps41 | Cg44–Pg1–Ps42 | Cg44–Pg1–Ps43 | Cg44–Pg1–Ps44 |
| Cg44–Pg1–Ps45 | Cg44–Pg1–Ps46 | Cg44–Pg1–Ps47 | Cg44–Pg1–Ps48 |
| Cg44–Pg1–Ps49 | Cg44–Pg1–Ps50 | Cg44–Pg1–Ps51 | Cg44–Pg1–Ps52 |
| Cg44–Pg1–Ps53 | Cg44–Pg1–Ps54 | Cg44–Pg1–Ps55 | Cg44–Pg1–Ps56 |
| Cg44–Pg1–Ps57 | Cg44–Pg1–Ps58 | Cg44–Pg1–Ps59 | Cg44–Pg1–Ps60 |
| Cg44–Pg1–Ps61 | Cg44–Pg1–Ps62 | Cg44–Pg1–Ps63 | Cg44–Pg1–Ps64 |
| Cg44–Pg1–Ps65 | Cg44–Pg1–Ps66 | Cg44–Pg1–Ps67 | Cg44–Pg1–Ps68 |

| | | | |
|---|---|---|---|
| Cg44–Pg1–Ps69 | Cg44–Pg1–Ps70 | Cg44–Pg1–Ps71 | Cg44–Pg1–Ps72 |
| Cg44–Pg1–Ps73 | Cg44–Pg1–Ps74 | Cg44–Pg1–Ps75 | Cg44–Pg1–Ps76 |
| Cg44–Pg1–Ps77 | Cg44–Pg1–Ps78 | Cg44–Pg1–Ps79 | Cg44–Pg1–Ps80 |
| Cg44–Pg1–Ps81 | Cg44–Pg1–Ps82 | Cg44–Pg1–Ps83 | Cg44–Pg1–Ps84 |
| Cg44–Pg1–Ps85 | Cg44–Pg1–Ps86 | Cg44–Pg1–Ps87 | Cg44–Pg1–Ps88 |
| Cg44–Pg1–Ps89 | Cg44–Pg1–Ps90 | Cg44–Pg1–Ps91 | Cg44–Pg1–Ps92 |
| Cg44–Pg1–Ps93 | Cg44–Pg1–Ps94 | Cg44–Pg1–Ps95 | Cg44–Pg1–Ps96 |
| Cg44–Pg1–Ps97 | Cg44–Pg1–Ps98 | Cg44–Pg1–Ps99 | Cg44–Pg1–Ps100 |
| Cg44–Pg1–Ps101 | Cg44–Pg1–Ps102 | Cg44–Pg1–Ps103 | Cg44–Pg1–Ps104 |
| Cg44–Pg1–Ps105 | Cg44–Pg1–Ps106 | Cg44–Pg1–Ps107 | Cg44–Pg1–Ps108 |
| Cg44–Pg1–Ps109 | Cg44–Pg1–Ps110 | Cg44–Pg1–Ps111 | Cg44–Pg1–Ps112 |
| Cg44–Pg1–Ps113 | Cg44–Pg1–Ps114 | Cg44–Pg1–Ps115 | Cg44–Pg1–Ps116 |
| Cg44–Pg1–Ps117 | Cg44–Pg1–Ps118 | Cg44–Pg1–Ps119 | Cg44–Pg1–Ps120 |
| Cg44–Pg1–Ps121 | Cg44–Pg1–Ps122 | Cg44–Pg1–Ps123 | Cg44–Pg1–Ps124 |
| Cg44–Pg1–Ps125 | Cg44–Pg1–Ps126 | Cg44–Pg1–Ps127 | Cg44–Pg1–Ps128 |
| Cg44–Pg1–Ps129 | Cg44–Pg1–Ps130 | Cg44–Pg1–Ps131 | Cg44–Pg1–Ps132 |
| Cg44–Pg1–Ps133 | Cg44–Pg1–Ps134 | Cg44–Pg1–Ps135 | Cg44–Pg1–Ps136 |
| Cg44–Pg1–Ps137 | Cg44–Pg1–Ps138 | Cg44–Pg1–Ps139 | Cg44–Pg1–Ps140 |
| Cg44–Pg1–Ps141 | Cg44–Pg1–Ps142 | Cg44–Pg1–Ps143 | Cg44–Pg1–Ps144 |
| Cg44–Pg1–Ps145 | Cg44–Pg1–Ps146 | Cg44–Pg1–Ps147 | Cg44–Pg1–Ps148 |
| Cg44–Pg1–Ps149 | Cg44–Pg1–Ps150 | Cg44–Pg1–Ps151 | Cg44–Pg1–Ps152 |
| Cg44–Pg1–Ps153 | Cg44–Pg1–Ps154 | Cg44–Pg1–Ps155 | Cg44–Pg1–Ps156 |
| Cg44–Pg1–Ps157 | Cg44–Pg1–Ps158 | Cg44–Pg1–Ps159 | Cg44–Pg1–Ps160 |
| Cg44–Pg1–Ps161 | Cg44–Pg1–Ps162 | Cg44–Pg1–Ps163 | Cg44–Pg1–Ps164 |
| Cg44–Pg1–Ps165 | Cg44–Pg1–Ps166 | Cg44–Pg1–Ps167 | Cg44–Pg1–Ps168 |
| Cg44–Pg1–Ps169 | Cg44–Pg1–Ps170 | Cg44–Pg1–Ps171 | Cg44–Pg1–Ps172 |
| Cg44–Pg1–Ps173 | Cg44–Pg1–Ps174 | Cg44–Pg1–Ps175 | Cg44–Pg1–Ps176 |
| Cg44–Pg1–Ps177 | Cg44–Pg1–Ps178 | Cg44–Pg1–Ps179 | Cg44–Pg1–Ps180 |
| Cg44–Pg1–Ps181 | Cg44–Pg1–Ps182 | Cg44–Pg1–Ps183 | Cg44–Pg1–Ps184 |
| Cg44–Pg1–Ps185 | Cg44–Pg1–Ps186 | Cg44–Pg1–Ps187 | Cg44–Pg1–Ps188 |
| Cg44–Pg1–Ps189 | Cg44–Pg1–Ps190 | Cg44–Pg1–Ps191 | Cg44–Pg1–Ps192 |
| Cg44–Pg1–Ps193 | Cg44–Pg1–Ps194 | Cg44–Pg1–Ps195 | Cg44–Pg1–Ps196 |
| Cg44–Pg1–Ps197 | Cg44–Pg1–Ps198 | Cg44–Pg1–Ps199 | Cg44–Pg1–Ps200 |
| Cg44–Pg1–Ps201 | Cg44–Pg1–Ps202 | Cg44–Pg1–Ps203 | Cg44–Pg1–Ps204 |
| Cg44–Pg1–Ps205 | Cg44–Pg1–Ps206 | Cg44–Pg1–Ps207 | Cg44–Pg1–Ps208 |
| Cg44–Pg1–Ps209 | Cg44–Pg1–Ps210 | Cg44–Pg1–Ps211 | Cg44–Pg1–Ps212 |
| Cg44–Pg1–Ps213 | Cg44–Pg1–Ps214 | Cg44–Pg1–Ps215 | Cg44–Pg1–Ps216 |
| Cg44–Pg1–Ps217 | Cg44–Pg1–Ps218 | Cg44–Pg1–Ps219 | Cg44–Pg1–Ps220 |
| Cg44–Pg1–Ps221 | Cg44–Pg1–Ps222 | Cg44–Pg1–Ps223 | Cg44–Pg1–Ps224 |
| Cg44–Pg1–Ps225 | Cg44–Pg1–Ps226 | Cg44–Pg1–Ps227 | Cg44–Pg1–Ps228 |
| Cg44–Pg1–Ps229 | Cg44–Pg1–Ps230 | Cg44–Pg1–Ps231 | Cg44–Pg1–Ps232 |
| Cg44–Pg1–Ps233 | Cg44–Pg1–Ps234 | Cg44–Pg1–Ps235 | Cg44–Pg1–Ps236 |
| Cg44–Pg1–Ps237 | Cg44–Pg1–Ps238 | Cg44–Pg1–Ps239 | Cg44–Pg1–Ps240 |
| Cg44–Pg1–Ps241 | Cg44–Pg1–Ps242 | Cg44–Pg1–Ps243 | |

| | | | |
|---|---|---|---|
| Cg45–Pg1–Ps1 | Cg45–Pg1–Ps2 | Cg45–Pg1–Ps3 | Cg45–Pg1–Ps4 |
| Cg45–Pg1–Ps5 | Cg45–Pg1–Ps6 | Cg45–Pg1–Ps7 | Cg45–Pg1–Ps8 |
| Cg45–Pg1–Ps9 | Cg45–Pg1–Ps10 | Cg45–Pg1–Ps11 | Cg45–Pg1–Ps12 |
| Cg45–Pg1–Ps13 | Cg45–Pg1–Ps14 | Cg45–Pg1–Ps15 | Cg45–Pg1–Ps16 |
| Cg45–Pg1–Ps17 | Cg45–Pg1–Ps18 | Cg45–Pg1–Ps19 | Cg45–Pg1–Ps20 |
| Cg45–Pg1–Ps21 | Cg45–Pg1–Ps22 | Cg45–Pg1–Ps23 | Cg45–Pg1–Ps24 |
| Cg45–Pg1–Ps25 | Cg45–Pg1–Ps26 | Cg45–Pg1–Ps27 | Cg45–Pg1–Ps28 |

| | | | |
|---|---|---|---|
| Cg45–Pg1–Ps29 | Cg45–Pg1–Ps30 | Cg45–Pg1–Ps31 | Cg45–Pg1–Ps32 |
| Cg45–Pg1–Ps33 | Cg45–Pg1–Ps34 | Cg45–Pg1–Ps35 | Cg45–Pg1–Ps36 |
| Cg45–Pg1–Ps37 | Cg45–Pg1–Ps38 | Cg45–Pg1–Ps39 | Cg45–Pg1–Ps40 |
| Cg45–Pg1–Ps41 | Cg45–Pg1–Ps42 | Cg45–Pg1–Ps43 | Cg45–Pg1–Ps44 |
| Cg45–Pg1–Ps45 | Cg45–Pg1–Ps46 | Cg45–Pg1–Ps47 | Cg45–Pg1–Ps48 |
| Cg45–Pg1–Ps49 | Cg45–Pg1–Ps50 | Cg45–Pg1–Ps51 | Cg45–Pg1–Ps52 |
| Cg45–Pg1–Ps53 | Cg45–Pg1–Ps54 | Cg45–Pg1–Ps55 | Cg45–Pg1–Ps56 |
| Cg45–Pg1–Ps57 | Cg45–Pg1–Ps58 | Cg45–Pg1–Ps59 | Cg45–Pg1–Ps60 |
| Cg45–Pg1–Ps61 | Cg45–Pg1–Ps62 | Cg45–Pg1–Ps63 | Cg45–Pg1–Ps64 |
| Cg45–Pg1–Ps65 | Cg45–Pg1–Ps66 | Cg45–Pg1–Ps67 | Cg45–Pg1–Ps68 |
| Cg45–Pg1–Ps69 | Cg45–Pg1–Ps70 | Cg45–Pg1–Ps71 | Cg45–Pg1–Ps72 |
| Cg45–Pg1–Ps73 | Cg45–Pg1–Ps74 | Cg45–Pg1–Ps75 | Cg45–Pg1–Ps76 |
| Cg45–Pg1–Ps77 | Cg45–Pg1–Ps78 | Cg45–Pg1–Ps79 | Cg45–Pg1–Ps80 |
| Cg45–Pg1–Ps81 | Cg45–Pg1–Ps82 | Cg45–Pg1–Ps83 | Cg45–Pg1–Ps84 |
| Cg45–Pg1–Ps85 | Cg45–Pg1–Ps86 | Cg45–Pg1–Ps87 | Cg45–Pg1–Ps88 |
| Cg45–Pg1–Ps89 | Cg45–Pg1–Ps90 | Cg45–Pg1–Ps91 | Cg45–Pg1–Ps92 |
| Cg45–Pg1–Ps93 | Cg45–Pg1–Ps94 | Cg45–Pg1–Ps95 | Cg45–Pg1–Ps96 |
| Cg45–Pg1–Ps97 | Cg45–Pg1–Ps98 | Cg45–Pg1–Ps99 | Cg45–Pg1–Ps100 |
| Cg45–Pg1–Ps101 | Cg45–Pg1–Ps102 | Cg45–Pg1–Ps103 | Cg45–Pg1–Ps104 |
| Cg45–Pg1–Ps105 | Cg45–Pg1–Ps106 | Cg45–Pg1–Ps107 | Cg45–Pg1–Ps108 |
| Cg45–Pg1–Ps109 | Cg45–Pg1–Ps110 | Cg45–Pg1–Ps111 | Cg45–Pg1–Ps112 |
| Cg45–Pg1–Ps113 | Cg45–Pg1–Ps114 | Cg45–Pg1–Ps115 | Cg45–Pg1–Ps116 |
| Cg45–Pg1–Ps117 | Cg45–Pg1–Ps118 | Cg45–Pg1–Ps119 | Cg45–Pg1–Ps120 |
| Cg45–Pg1–Ps121 | Cg45–Pg1–Ps122 | Cg45–Pg1–Ps123 | Cg45–Pg1–Ps124 |
| Cg45–Pg1–Ps125 | Cg45–Pg1–Ps126 | Cg45–Pg1–Ps127 | Cg45–Pg1–Ps128 |
| Cg45–Pg1–Ps129 | Cg45–Pg1–Ps130 | Cg45–Pg1–Ps131 | Cg45–Pg1–Ps132 |
| Cg45–Pg1–Ps133 | Cg45–Pg1–Ps134 | Cg45–Pg1–Ps135 | Cg45–Pg1–Ps136 |
| Cg45–Pg1–Ps137 | Cg45–Pg1–Ps138 | Cg45–Pg1–Ps139 | Cg45–Pg1–Ps140 |
| Cg45–Pg1–Ps141 | Cg45–Pg1–Ps142 | Cg45–Pg1–Ps143 | Cg45–Pg1–Ps144 |
| Cg45–Pg1–Ps145 | Cg45–Pg1–Ps146 | Cg45–Pg1–Ps147 | Cg45–Pg1–Ps148 |
| Cg45–Pg1–Ps149 | Cg45–Pg1–Ps150 | Cg45–Pg1–Ps151 | Cg45–Pg1–Ps152 |
| Cg45–Pg1–Ps153 | Cg45–Pg1–Ps154 | Cg45–Pg1–Ps155 | Cg45–Pg1–Ps156 |
| Cg45–Pg1–Ps157 | Cg45–Pg1–Ps158 | Cg45–Pg1–Ps159 | Cg45–Pg1–Ps160 |
| Cg45–Pg1–Ps161 | Cg45–Pg1–Ps162 | Cg45–Pg1–Ps163 | Cg45–Pg1–Ps164 |
| Cg45–Pg1–Ps165 | Cg45–Pg1–Ps166 | Cg45–Pg1–Ps167 | Cg45–Pg1–Ps168 |
| Cg45–Pg1–Ps169 | Cg45–Pg1–Ps170 | Cg45–Pg1–Ps171 | Cg45–Pg1–Ps172 |
| Cg45–Pg1–Ps173 | Cg45–Pg1–Ps174 | Cg45–Pg1–Ps175 | Cg45–Pg1–Ps176 |
| Cg45–Pg1–Ps177 | Cg45–Pg1–Ps178 | Cg45–Pg1–Ps179 | Cg45–Pg1–Ps180 |
| Cg45–Pg1–Ps181 | Cg45–Pg1–Ps182 | Cg45–Pg1–Ps183 | Cg45–Pg1–Ps184 |
| Cg45–Pg1–Ps185 | Cg45–Pg1–Ps186 | Cg45–Pg1–Ps187 | Cg45–Pg1–Ps188 |
| Cg45–Pg1–Ps189 | Cg45–Pg1–Ps190 | Cg45–Pg1–Ps191 | Cg45–Pg1–Ps192 |
| Cg45–Pg1–Ps193 | Cg45–Pg1–Ps194 | Cg45–Pg1–Ps195 | Cg45–Pg1–Ps196 |
| Cg45–Pg1–Ps197 | Cg45–Pg1–Ps198 | Cg45–Pg1–Ps199 | Cg45–Pg1–Ps200 |
| Cg45–Pg1–Ps201 | Cg45–Pg1–Ps202 | Cg45–Pg1–Ps203 | Cg45–Pg1–Ps204 |
| Cg45–Pg1–Ps205 | Cg45–Pg1–Ps206 | Cg45–Pg1–Ps207 | Cg45–Pg1–Ps208 |
| Cg45–Pg1–Ps209 | Cg45–Pg1–Ps210 | Cg45–Pg1–Ps211 | Cg45–Pg1–Ps212 |
| Cg45–Pg1–Ps213 | Cg45–Pg1–Ps214 | Cg45–Pg1–Ps215 | Cg45–Pg1–Ps216 |
| Cg45–Pg1–Ps217 | Cg45–Pg1–Ps218 | Cg45–Pg1–Ps219 | Cg45–Pg1–Ps220 |
| Cg45–Pg1–Ps221 | Cg45–Pg1–Ps222 | Cg45–Pg1–Ps223 | Cg45–Pg1–Ps224 |
| Cg45–Pg1–Ps225 | Cg45–Pg1–Ps226 | Cg45–Pg1–Ps227 | Cg45–Pg1–Ps228 |
| Cg45–Pg1–Ps229 | Cg45–Pg1–Ps230 | Cg45–Pg1–Ps231 | Cg45–Pg1–Ps232 |
| Cg45–Pg1–Ps233 | Cg45–Pg1–Ps234 | Cg45–Pg1–Ps235 | Cg45–Pg1–Ps236 |

| | | | |
|---|---|---|---|
| Cg45–Pg1–Ps237 | Cg45–Pg1–Ps238 | Cg45–Pg1–Ps239 | Cg45–Pg1–Ps240 |
| Cg45–Pg1–Ps241 | Cg45–Pg1–Ps242 | Cg45–Pg1–Ps243 | |

| | | | |
|---|---|---|---|
| Cg46–Pg1–Ps1 | Cg46–Pg1–Ps2 | Cg46–Pg1–Ps3 | Cg46–Pg1–Ps4 |
| Cg46–Pg1–Ps5 | Cg46–Pg1–Ps6 | Cg46–Pg1–Ps7 | Cg46–Pg1–Ps8 |
| Cg46–Pg1–Ps9 | Cg46–Pg1–Ps10 | Cg46–Pg1–Ps11 | Cg46–Pg1–Ps12 |
| Cg46–Pg1–Ps13 | Cg46–Pg1–Ps14 | Cg46–Pg1–Ps15 | Cg46–Pg1–Ps16 |
| Cg46–Pg1–Ps17 | Cg46–Pg1–Ps18 | Cg46–Pg1–Ps19 | Cg46–Pg1–Ps20 |
| Cg46–Pg1–Ps21 | Cg46–Pg1–Ps22 | Cg46–Pg1–Ps23 | Cg46–Pg1–Ps24 |
| Cg46–Pg1–Ps25 | Cg46–Pg1–Ps26 | Cg46–Pg1–Ps27 | Cg46–Pg1–Ps28 |
| Cg46–Pg1–Ps29 | Cg46–Pg1–Ps30 | Cg46–Pg1–Ps31 | Cg46–Pg1–Ps32 |
| Cg46–Pg1–Ps33 | Cg46–Pg1–Ps34 | Cg46–Pg1–Ps35 | Cg46–Pg1–Ps36 |
| Cg46–Pg1–Ps37 | Cg46–Pg1–Ps38 | Cg46–Pg1–Ps39 | Cg46–Pg1–Ps40 |
| Cg46–Pg1–Ps41 | Cg46–Pg1–Ps42 | Cg46–Pg1–Ps43 | Cg46–Pg1–Ps44 |
| Cg46–Pg1–Ps45 | Cg46–Pg1–Ps46 | Cg46–Pg1–Ps47 | Cg46–Pg1–Ps48 |
| Cg46–Pg1–Ps49 | Cg46–Pg1–Ps50 | Cg46–Pg1–Ps51 | Cg46–Pg1–Ps52 |
| Cg46–Pg1–Ps53 | Cg46–Pg1–Ps54 | Cg46–Pg1–Ps55 | Cg46–Pg1–Ps56 |
| Cg46–Pg1–Ps57 | Cg46–Pg1–Ps58 | Cg46–Pg1–Ps59 | Cg46–Pg1–Ps60 |
| Cg46–Pg1–Ps61 | Cg46–Pg1–Ps62 | Cg46–Pg1–Ps63 | Cg46–Pg1–Ps64 |
| Cg46–Pg1–Ps65 | Cg46–Pg1–Ps66 | Cg46–Pg1–Ps67 | Cg46–Pg1–Ps68 |
| Cg46–Pg1–Ps69 | Cg46–Pg1–Ps70 | Cg46–Pg1–Ps71 | Cg46–Pg1–Ps72 |
| Cg46–Pg1–Ps73 | Cg46–Pg1–Ps74 | Cg46–Pg1–Ps75 | Cg46–Pg1–Ps76 |
| Cg46–Pg1–Ps77 | Cg46–Pg1–Ps78 | Cg46–Pg1–Ps79 | Cg46–Pg1–Ps80 |
| Cg46–Pg1–Ps81 | Cg46–Pg1–Ps82 | Cg46–Pg1–Ps83 | Cg46–Pg1–Ps84 |
| Cg46–Pg1–Ps85 | Cg46–Pg1–Ps86 | Cg46–Pg1–Ps87 | Cg46–Pg1–Ps88 |
| Cg46–Pg1–Ps89 | Cg46–Pg1–Ps90 | Cg46–Pg1–Ps91 | Cg46–Pg1–Ps92 |
| Cg46–Pg1–Ps93 | Cg46–Pg1–Ps94 | Cg46–Pg1–Ps95 | Cg46–Pg1–Ps96 |
| Cg46–Pg1–Ps97 | Cg46–Pg1–Ps98 | Cg46–Pg1–Ps99 | Cg46–Pg1–Ps100 |
| Cg46–Pg1–Ps101 | Cg46–Pg1–Ps102 | Cg46–Pg1–Ps103 | Cg46–Pg1–Ps104 |
| Cg46–Pg1–Ps105 | Cg46–Pg1–Ps106 | Cg46–Pg1–Ps107 | Cg46–Pg1–Ps108 |
| Cg46–Pg1–Ps109 | Cg46–Pg1–Ps110 | Cg46–Pg1–Ps111 | Cg46–Pg1–Ps112 |
| Cg46–Pg1–Ps113 | Cg46–Pg1–Ps114 | Cg46–Pg1–Ps115 | Cg46–Pg1–Ps116 |
| Cg46–Pg1–Ps117 | Cg46–Pg1–Ps118 | Cg46–Pg1–Ps119 | Cg46–Pg1–Ps120 |
| Cg46–Pg1–Ps121 | Cg46–Pg1–Ps122 | Cg46–Pg1–Ps123 | Cg46–Pg1–Ps124 |
| Cg46–Pg1–Ps125 | Cg46–Pg1–Ps126 | Cg46–Pg1–Ps127 | Cg46–Pg1–Ps128 |
| Cg46–Pg1–Ps129 | Cg46–Pg1–Ps130 | Cg46–Pg1–Ps131 | Cg46–Pg1–Ps132 |
| Cg46–Pg1–Ps133 | Cg46–Pg1–Ps134 | Cg46–Pg1–Ps135 | Cg46–Pg1–Ps136 |
| Cg46–Pg1–Ps137 | Cg46–Pg1–Ps138 | Cg46–Pg1–Ps139 | Cg46–Pg1–Ps140 |
| Cg46–Pg1–Ps141 | Cg46–Pg1–Ps142 | Cg46–Pg1–Ps143 | Cg46–Pg1–Ps144 |
| Cg46–Pg1–Ps145 | Cg46–Pg1–Ps146 | Cg46–Pg1–Ps147 | Cg46–Pg1–Ps148 |
| Cg46–Pg1–Ps149 | Cg46–Pg1–Ps150 | Cg46–Pg1–Ps151 | Cg46–Pg1–Ps152 |
| Cg46–Pg1–Ps153 | Cg46–Pg1–Ps154 | Cg46–Pg1–Ps155 | Cg46–Pg1–Ps156 |
| Cg46–Pg1–Ps157 | Cg46–Pg1–Ps158 | Cg46–Pg1–Ps159 | Cg46–Pg1–Ps160 |
| Cg46–Pg1–Ps161 | Cg46–Pg1–Ps162 | Cg46–Pg1–Ps163 | Cg46–Pg1–Ps164 |
| Cg46–Pg1–Ps165 | Cg46–Pg1–Ps166 | Cg46–Pg1–Ps167 | Cg46–Pg1–Ps168 |
| Cg46–Pg1–Ps169 | Cg46–Pg1–Ps170 | Cg46–Pg1–Ps171 | Cg46–Pg1–Ps172 |
| Cg46–Pg1–Ps173 | Cg46–Pg1–Ps174 | Cg46–Pg1–Ps175 | Cg46–Pg1–Ps176 |
| Cg46–Pg1–Ps177 | Cg46–Pg1–Ps178 | Cg46–Pg1–Ps179 | Cg46–Pg1–Ps180 |
| Cg46–Pg1–Ps181 | Cg46–Pg1–Ps182 | Cg46–Pg1–Ps183 | Cg46–Pg1–Ps184 |
| Cg46–Pg1–Ps185 | Cg46–Pg1–Ps186 | Cg46–Pg1–Ps187 | Cg46–Pg1–Ps188 |
| Cg46–Pg1–Ps189 | Cg46–Pg1–Ps190 | Cg46–Pg1–Ps191 | Cg46–Pg1–Ps192 |
| Cg46–Pg1–Ps193 | Cg46–Pg1–Ps194 | Cg46–Pg1–Ps195 | Cg46–Pg1–Ps196 |

| | | | |
|---|---|---|---|
| Cg46–Pg1–Ps197 | Cg46–Pg1–Ps198 | Cg46–Pg1–Ps199 | Cg46–Pg1–Ps200 |
| Cg46–Pg1–Ps201 | Cg46–Pg1–Ps202 | Cg46–Pg1–Ps203 | Cg46–Pg1–Ps204 |
| Cg46–Pg1–Ps205 | Cg46–Pg1–Ps206 | Cg46–Pg1–Ps207 | Cg46–Pg1–Ps208 |
| Cg46–Pg1–Ps209 | Cg46–Pg1–Ps210 | Cg46–Pg1–Ps211 | Cg46–Pg1–Ps212 |
| Cg46–Pg1–Ps213 | Cg46–Pg1–Ps214 | Cg46–Pg1–Ps215 | Cg46–Pg1–Ps216 |
| Cg46–Pg1–Ps217 | Cg46–Pg1–Ps218 | Cg46–Pg1–Ps219 | Cg46–Pg1–Ps220 |
| Cg46–Pg1–Ps221 | Cg46–Pg1–Ps222 | Cg46–Pg1–Ps223 | Cg46–Pg1–Ps224 |
| Cg46–Pg1–Ps225 | Cg46–Pg1–Ps226 | Cg46–Pg1–Ps227 | Cg46–Pg1–Ps228 |
| Cg46–Pg1–Ps229 | Cg46–Pg1–Ps230 | Cg46–Pg1–Ps231 | Cg46–Pg1–Ps232 |
| Cg46–Pg1–Ps233 | Cg46–Pg1–Ps234 | Cg46–Pg1–Ps235 | Cg46–Pg1–Ps236 |
| Cg46–Pg1–Ps237 | Cg46–Pg1–Ps238 | Cg46–Pg1–Ps239 | Cg46–Pg1–Ps240 |
| Cg46–Pg1–Ps241 | Cg46–Pg1–Ps242 | Cg46–Pg1–Ps243 | |

| | | | |
|---|---|---|---|
| Cg47–Pg1–Ps1 | Cg47–Pg1–Ps2 | Cg47–Pg1–Ps3 | Cg47–Pg1–Ps4 |
| Cg47–Pg1–Ps5 | Cg47–Pg1–Ps6 | Cg47–Pg1–Ps7 | Cg47–Pg1–Ps8 |
| Cg47–Pg1–Ps9 | Cg47–Pg1–Ps10 | Cg47–Pg1–Ps11 | Cg47–Pg1–Ps12 |
| Cg47–Pg1–Ps13 | Cg47–Pg1–Ps14 | Cg47–Pg1–Ps15 | Cg47–Pg1–Ps16 |
| Cg47–Pg1–Ps17 | Cg47–Pg1–Ps18 | Cg47–Pg1–Ps19 | Cg47–Pg1–Ps20 |
| Cg47–Pg1–Ps21 | Cg47–Pg1–Ps22 | Cg47–Pg1–Ps23 | Cg47–Pg1–Ps24 |
| Cg47–Pg1–Ps25 | Cg47–Pg1–Ps26 | Cg47–Pg1–Ps27 | Cg47–Pg1–Ps28 |
| Cg47–Pg1–Ps29 | Cg47–Pg1–Ps30 | Cg47–Pg1–Ps31 | Cg47–Pg1–Ps32 |
| Cg47–Pg1–Ps33 | Cg47–Pg1–Ps34 | Cg47–Pg1–Ps35 | Cg47–Pg1–Ps36 |
| Cg47–Pg1–Ps37 | Cg47–Pg1–Ps38 | Cg47–Pg1–Ps39 | Cg47–Pg1–Ps40 |
| Cg47–Pg1–Ps41 | Cg47–Pg1–Ps42 | Cg47–Pg1–Ps43 | Cg47–Pg1–Ps44 |
| Cg47–Pg1–Ps45 | Cg47–Pg1–Ps46 | Cg47–Pg1–Ps47 | Cg47–Pg1–Ps48 |
| Cg47–Pg1–Ps49 | Cg47–Pg1–Ps50 | Cg47–Pg1–Ps51 | Cg47–Pg1–Ps52 |
| Cg47–Pg1–Ps53 | Cg47–Pg1–Ps54 | Cg47–Pg1–Ps55 | Cg47–Pg1–Ps56 |
| Cg47–Pg1–Ps57 | Cg47–Pg1–Ps58 | Cg47–Pg1–Ps59 | Cg47–Pg1–Ps60 |
| Cg47–Pg1–Ps61 | Cg47–Pg1–Ps62 | Cg47–Pg1–Ps63 | Cg47–Pg1–Ps64 |
| Cg47–Pg1–Ps65 | Cg47–Pg1–Ps66 | Cg47–Pg1–Ps67 | Cg47–Pg1–Ps68 |
| Cg47–Pg1–Ps69 | Cg47–Pg1–Ps70 | Cg47–Pg1–Ps71 | Cg47–Pg1–Ps72 |
| Cg47–Pg1–Ps73 | Cg47–Pg1–Ps74 | Cg47–Pg1–Ps75 | Cg47–Pg1–Ps76 |
| Cg47–Pg1–Ps77 | Cg47–Pg1–Ps78 | Cg47–Pg1–Ps79 | Cg47–Pg1–Ps80 |
| Cg47–Pg1–Ps81 | Cg47–Pg1–Ps82 | Cg47–Pg1–Ps83 | Cg47–Pg1–Ps84 |
| Cg47–Pg1–Ps85 | Cg47–Pg1–Ps86 | Cg47–Pg1–Ps87 | Cg47–Pg1–Ps88 |
| Cg47–Pg1–Ps89 | Cg47–Pg1–Ps90 | Cg47–Pg1–Ps91 | Cg47–Pg1–Ps92 |
| Cg47–Pg1–Ps93 | Cg47–Pg1–Ps94 | Cg47–Pg1–Ps95 | Cg47–Pg1–Ps96 |
| Cg47–Pg1–Ps97 | Cg47–Pg1–Ps98 | Cg47–Pg1–Ps99 | Cg47–Pg1–Ps100 |
| Cg47–Pg1–Ps101 | Cg47–Pg1–Ps102 | Cg47–Pg1–Ps103 | Cg47–Pg1–Ps104 |
| Cg47–Pg1–Ps105 | Cg47–Pg1–Ps106 | Cg47–Pg1–Ps107 | Cg47–Pg1–Ps108 |
| Cg47–Pg1–Ps109 | Cg47–Pg1–Ps110 | Cg47–Pg1–Ps111 | Cg47–Pg1–Ps112 |
| Cg47–Pg1–Ps113 | Cg47–Pg1–Ps114 | Cg47–Pg1–Ps115 | Cg47–Pg1–Ps116 |
| Cg47–Pg1–Ps117 | Cg47–Pg1–Ps118 | Cg47–Pg1–Ps119 | Cg47–Pg1–Ps120 |
| Cg47–Pg1–Ps121 | Cg47–Pg1–Ps122 | Cg47–Pg1–Ps123 | Cg47–Pg1–Ps124 |
| Cg47–Pg1–Ps125 | Cg47–Pg1–Ps126 | Cg47–Pg1–Ps127 | Cg47–Pg1–Ps128 |
| Cg47–Pg1–Ps129 | Cg47–Pg1–Ps130 | Cg47–Pg1–Ps131 | Cg47–Pg1–Ps132 |
| Cg47–Pg1–Ps133 | Cg47–Pg1–Ps134 | Cg47–Pg1–Ps135 | Cg47–Pg1–Ps136 |
| Cg47–Pg1–Ps137 | Cg47–Pg1–Ps138 | Cg47–Pg1–Ps139 | Cg47–Pg1–Ps140 |
| Cg47–Pg1–Ps141 | Cg47–Pg1–Ps142 | Cg47–Pg1–Ps143 | Cg47–Pg1–Ps144 |
| Cg47–Pg1–Ps145 | Cg47–Pg1–Ps146 | Cg47–Pg1–Ps147 | Cg47–Pg1–Ps148 |
| Cg47–Pg1–Ps149 | Cg47–Pg1–Ps150 | Cg47–Pg1–Ps151 | Cg47–Pg1–Ps152 |
| Cg47–Pg1–Ps153 | Cg47–Pg1–Ps154 | Cg47–Pg1–Ps155 | Cg47–Pg1–Ps156 |

| | | | |
|---|---|---|---|
| Cg47–Pg1–Ps157 | Cg47–Pg1–Ps158 | Cg47–Pg1–Ps159 | Cg47–Pg1–Ps160 |
| Cg47–Pg1–Ps161 | Cg47–Pg1–Ps162 | Cg47–Pg1–Ps163 | Cg47–Pg1–Ps164 |
| Cg47–Pg1–Ps165 | Cg47–Pg1–Ps166 | Cg47–Pg1–Ps167 | Cg47–Pg1–Ps168 |
| Cg47–Pg1–Ps169 | Cg47–Pg1–Ps170 | Cg47–Pg1–Ps171 | Cg47–Pg1–Ps172 |
| Cg47–Pg1–Ps173 | Cg47–Pg1–Ps174 | Cg47–Pg1–Ps175 | Cg47–Pg1–Ps176 |
| Cg47–Pg1–Ps177 | Cg47–Pg1–Ps178 | Cg47–Pg1–Ps179 | Cg47–Pg1–Ps180 |
| Cg47–Pg1–Ps181 | Cg47–Pg1–Ps182 | Cg47–Pg1–Ps183 | Cg47–Pg1–Ps184 |
| Cg47–Pg1–Ps185 | Cg47–Pg1–Ps186 | Cg47–Pg1–Ps187 | Cg47–Pg1–Ps188 |
| Cg47–Pg1–Ps189 | Cg47–Pg1–Ps190 | Cg47–Pg1–Ps191 | Cg47–Pg1–Ps192 |
| Cg47–Pg1–Ps193 | Cg47–Pg1–Ps194 | Cg47–Pg1–Ps195 | Cg47–Pg1–Ps196 |
| Cg47–Pg1–Ps197 | Cg47–Pg1–Ps198 | Cg47–Pg1–Ps199 | Cg47–Pg1–Ps200 |
| Cg47–Pg1–Ps201 | Cg47–Pg1–Ps202 | Cg47–Pg1–Ps203 | Cg47–Pg1–Ps204 |
| Cg47–Pg1–Ps205 | Cg47–Pg1–Ps206 | Cg47–Pg1–Ps207 | Cg47–Pg1–Ps208 |
| Cg47–Pg1–Ps209 | Cg47–Pg1–Ps210 | Cg47–Pg1–Ps211 | Cg47–Pg1–Ps212 |
| Cg47–Pg1–Ps213 | Cg47–Pg1–Ps214 | Cg47–Pg1–Ps215 | Cg47–Pg1–Ps216 |
| Cg47–Pg1–Ps217 | Cg47–Pg1–Ps218 | Cg47–Pg1–Ps219 | Cg47–Pg1–Ps220 |
| Cg47–Pg1–Ps221 | Cg47–Pg1–Ps222 | Cg47–Pg1–Ps223 | Cg47–Pg1–Ps224 |
| Cg47–Pg1–Ps225 | Cg47–Pg1–Ps226 | Cg47–Pg1–Ps227 | Cg47–Pg1–Ps228 |
| Cg47–Pg1–Ps229 | Cg47–Pg1–Ps230 | Cg47–Pg1–Ps231 | Cg47–Pg1–Ps232 |
| Cg47–Pg1–Ps233 | Cg47–Pg1–Ps234 | Cg47–Pg1–Ps235 | Cg47–Pg1–Ps236 |
| Cg47–Pg1–Ps237 | Cg47–Pg1–Ps238 | Cg47–Pg1–Ps239 | Cg47–Pg1–Ps240 |
| Cg47–Pg1–Ps241 | Cg47–Pg1–Ps242 | Cg47–Pg1–Ps243 | |

| | | | |
|---|---|---|---|
| Cg48–Pg1–Ps1 | Cg48–Pg1–Ps2 | Cg48–Pg1–Ps3 | Cg48–Pg1–Ps4 |
| Cg48–Pg1–Ps5 | Cg48–Pg1–Ps6 | Cg48–Pg1–Ps7 | Cg48–Pg1–Ps8 |
| Cg48–Pg1–Ps9 | Cg48–Pg1–Ps10 | Cg48–Pg1–Ps11 | Cg48–Pg1–Ps12 |
| Cg48–Pg1–Ps13 | Cg48–Pg1–Ps14 | Cg48–Pg1–Ps15 | Cg48–Pg1–Ps16 |
| Cg48–Pg1–Ps17 | Cg48–Pg1–Ps18 | Cg48–Pg1–Ps19 | Cg48–Pg1–Ps20 |
| Cg48–Pg1–Ps21 | Cg48–Pg1–Ps22 | Cg48–Pg1–Ps23 | Cg48–Pg1–Ps24 |
| Cg48–Pg1–Ps25 | Cg48–Pg1–Ps26 | Cg48–Pg1–Ps27 | Cg48–Pg1–Ps28 |
| Cg48–Pg1–Ps29 | Cg48–Pg1–Ps30 | Cg48–Pg1–Ps31 | Cg48–Pg1–Ps32 |
| Cg48–Pg1–Ps33 | Cg48–Pg1–Ps34 | Cg48–Pg1–Ps35 | Cg48–Pg1–Ps36 |
| Cg48–Pg1–Ps37 | Cg48–Pg1–Ps38 | Cg48–Pg1–Ps39 | Cg48–Pg1–Ps40 |
| Cg48–Pg1–Ps41 | Cg48–Pg1–Ps42 | Cg48–Pg1–Ps43 | Cg48–Pg1–Ps44 |
| Cg48–Pg1–Ps45 | Cg48–Pg1–Ps46 | Cg48–Pg1–Ps47 | Cg48–Pg1–Ps48 |
| Cg48–Pg1–Ps49 | Cg48–Pg1–Ps50 | Cg48–Pg1–Ps51 | Cg48–Pg1–Ps52 |
| Cg48–Pg1–Ps53 | Cg48–Pg1–Ps54 | Cg48–Pg1–Ps55 | Cg48–Pg1–Ps56 |
| Cg48–Pg1–Ps57 | Cg48–Pg1–Ps58 | Cg48–Pg1–Ps59 | Cg48–Pg1–Ps60 |
| Cg48–Pg1–Ps61 | Cg48–Pg1–Ps62 | Cg48–Pg1–Ps63 | Cg48–Pg1–Ps64 |
| Cg48–Pg1–Ps65 | Cg48–Pg1–Ps66 | Cg48–Pg1–Ps67 | Cg48–Pg1–Ps68 |
| Cg48–Pg1–Ps69 | Cg48–Pg1–Ps70 | Cg48–Pg1–Ps71 | Cg48–Pg1–Ps72 |
| Cg48–Pg1–Ps73 | Cg48–Pg1–Ps74 | Cg48–Pg1–Ps75 | Cg48–Pg1–Ps76 |
| Cg48–Pg1–Ps77 | Cg48–Pg1–Ps78 | Cg48–Pg1–Ps79 | Cg48–Pg1–Ps80 |
| Cg48–Pg1–Ps81 | Cg48–Pg1–Ps82 | Cg48–Pg1–Ps83 | Cg48–Pg1–Ps84 |
| Cg48–Pg1–Ps85 | Cg48–Pg1–Ps86 | Cg48–Pg1–Ps87 | Cg48–Pg1–Ps88 |
| Cg48–Pg1–Ps89 | Cg48–Pg1–Ps90 | Cg48–Pg1–Ps91 | Cg48–Pg1–Ps92 |
| Cg48–Pg1–Ps93 | Cg48–Pg1–Ps94 | Cg48–Pg1–Ps95 | Cg48–Pg1–Ps96 |
| Cg48–Pg1–Ps97 | Cg48–Pg1–Ps98 | Cg48–Pg1–Ps99 | Cg48–Pg1–Ps100 |
| Cg48–Pg1–Ps101 | Cg48–Pg1–Ps102 | Cg48–Pg1–Ps103 | Cg48–Pg1–Ps104 |
| Cg48–Pg1–Ps105 | Cg48–Pg1–Ps106 | Cg48–Pg1–Ps107 | Cg48–Pg1–Ps108 |
| Cg48–Pg1–Ps109 | Cg48–Pg1–Ps110 | Cg48–Pg1–Ps111 | Cg48–Pg1–Ps112 |
| Cg48–Pg1–Ps113 | Cg48–Pg1–Ps114 | Cg48–Pg1–Ps115 | Cg48–Pg1–Ps116 |

| | | | |
|---|---|---|---|
| Cg48–Pg1–Ps117 | Cg48–Pg1–Ps118 | Cg48–Pg1–Ps119 | Cg48–Pg1–Ps120 |
| Cg48–Pg1–Ps121 | Cg48–Pg1–Ps122 | Cg48–Pg1–Ps123 | Cg48–Pg1–Ps124 |
| Cg48–Pg1–Ps125 | Cg48–Pg1–Ps126 | Cg48–Pg1–Ps127 | Cg48–Pg1–Ps128 |
| Cg48–Pg1–Ps129 | Cg48–Pg1–Ps130 | Cg48–Pg1–Ps131 | Cg48–Pg1–Ps132 |
| Cg48–Pg1–Ps133 | Cg48–Pg1–Ps134 | Cg48–Pg1–Ps135 | Cg48–Pg1–Ps136 |
| Cg48–Pg1–Ps137 | Cg48–Pg1–Ps138 | Cg48–Pg1–Ps139 | Cg48–Pg1–Ps140 |
| Cg48–Pg1–Ps141 | Cg48–Pg1–Ps142 | Cg48–Pg1–Ps143 | Cg48–Pg1–Ps144 |
| Cg48–Pg1–Ps145 | Cg48–Pg1–Ps146 | Cg48–Pg1–Ps147 | Cg48–Pg1–Ps148 |
| Cg48–Pg1–Ps149 | Cg48–Pg1–Ps150 | Cg48–Pg1–Ps151 | Cg48–Pg1–Ps152 |
| Cg48–Pg1–Ps153 | Cg48–Pg1–Ps154 | Cg48–Pg1–Ps155 | Cg48–Pg1–Ps156 |
| Cg48–Pg1–Ps157 | Cg48–Pg1–Ps158 | Cg48–Pg1–Ps159 | Cg48–Pg1–Ps160 |
| Cg48–Pg1–Ps161 | Cg48–Pg1–Ps162 | Cg48–Pg1–Ps163 | Cg48–Pg1–Ps164 |
| Cg48–Pg1–Ps165 | Cg48–Pg1–Ps166 | Cg48–Pg1–Ps167 | Cg48–Pg1–Ps168 |
| Cg48–Pg1–Ps169 | Cg48–Pg1–Ps170 | Cg48–Pg1–Ps171 | Cg48–Pg1–Ps172 |
| Cg48–Pg1–Ps173 | Cg48–Pg1–Ps174 | Cg48–Pg1–Ps175 | Cg48–Pg1–Ps176 |
| Cg48–Pg1–Ps177 | Cg48–Pg1–Ps178 | Cg48–Pg1–Ps179 | Cg48–Pg1–Ps180 |
| Cg48–Pg1–Ps181 | Cg48–Pg1–Ps182 | Cg48–Pg1–Ps183 | Cg48–Pg1–Ps184 |
| Cg48–Pg1–Ps185 | Cg48–Pg1–Ps186 | Cg48–Pg1–Ps187 | Cg48–Pg1–Ps188 |
| Cg48–Pg1–Ps189 | Cg48–Pg1–Ps190 | Cg48–Pg1–Ps191 | Cg48–Pg1–Ps192 |
| Cg48–Pg1–Ps193 | Cg48–Pg1–Ps194 | Cg48–Pg1–Ps195 | Cg48–Pg1–Ps196 |
| Cg48–Pg1–Ps197 | Cg48–Pg1–Ps198 | Cg48–Pg1–Ps199 | Cg48–Pg1–Ps200 |
| Cg48–Pg1–Ps201 | Cg48–Pg1–Ps202 | Cg48–Pg1–Ps203 | Cg48–Pg1–Ps204 |
| Cg48–Pg1–Ps205 | Cg48–Pg1–Ps206 | Cg48–Pg1–Ps207 | Cg48–Pg1–Ps208 |
| Cg48–Pg1–Ps209 | Cg48–Pg1–Ps210 | Cg48–Pg1–Ps211 | Cg48–Pg1–Ps212 |
| Cg48–Pg1–Ps213 | Cg48–Pg1–Ps214 | Cg48–Pg1–Ps215 | Cg48–Pg1–Ps216 |
| Cg48–Pg1–Ps217 | Cg48–Pg1–Ps218 | Cg48–Pg1–Ps219 | Cg48–Pg1–Ps220 |
| Cg48–Pg1–Ps221 | Cg48–Pg1–Ps222 | Cg48–Pg1–Ps223 | Cg48–Pg1–Ps224 |
| Cg48–Pg1–Ps225 | Cg48–Pg1–Ps226 | Cg48–Pg1–Ps227 | Cg48–Pg1–Ps228 |
| Cg48–Pg1–Ps229 | Cg48–Pg1–Ps230 | Cg48–Pg1–Ps231 | Cg48–Pg1–Ps232 |
| Cg48–Pg1–Ps233 | Cg48–Pg1–Ps234 | Cg48–Pg1–Ps235 | Cg48–Pg1–Ps236 |
| Cg48–Pg1–Ps237 | Cg48–Pg1–Ps238 | Cg48–Pg1–Ps239 | Cg48–Pg1–Ps240 |
| Cg48–Pg1–Ps241 | Cg48–Pg1–Ps242 | Cg48–Pg1–Ps243 | |

| | | | |
|---|---|---|---|
| Cg49–Pg1–Ps1 | Cg49–Pg1–Ps2 | Cg49–Pg1–Ps3 | Cg49–Pg1–Ps4 |
| Cg49–Pg1–Ps5 | Cg49–Pg1–Ps6 | Cg49–Pg1–Ps7 | Cg49–Pg1–Ps8 |
| Cg49–Pg1–Ps9 | Cg49–Pg1–Ps10 | Cg49–Pg1–Ps11 | Cg49–Pg1–Ps12 |
| Cg49–Pg1–Ps13 | Cg49–Pg1–Ps14 | Cg49–Pg1–Ps15 | Cg49–Pg1–Ps16 |
| Cg49–Pg1–Ps17 | Cg49–Pg1–Ps18 | Cg49–Pg1–Ps19 | Cg49–Pg1–Ps20 |
| Cg49–Pg1–Ps21 | Cg49–Pg1–Ps22 | Cg49–Pg1–Ps23 | Cg49–Pg1–Ps24 |
| Cg49–Pg1–Ps25 | Cg49–Pg1–Ps26 | Cg49–Pg1–Ps27 | Cg49–Pg1–Ps28 |
| Cg49–Pg1–Ps29 | Cg49–Pg1–Ps30 | Cg49–Pg1–Ps31 | Cg49–Pg1–Ps32 |
| Cg49–Pg1–Ps33 | Cg49–Pg1–Ps34 | Cg49–Pg1–Ps35 | Cg49–Pg1–Ps36 |
| Cg49–Pg1–Ps37 | Cg49–Pg1–Ps38 | Cg49–Pg1–Ps39 | Cg49–Pg1–Ps40 |
| Cg49–Pg1–Ps41 | Cg49–Pg1–Ps42 | Cg49–Pg1–Ps43 | Cg49–Pg1–Ps44 |
| Cg49–Pg1–Ps45 | Cg49–Pg1–Ps46 | Cg49–Pg1–Ps47 | Cg49–Pg1–Ps48 |
| Cg49–Pg1–Ps49 | Cg49–Pg1–Ps50 | Cg49–Pg1–Ps51 | Cg49–Pg1–Ps52 |
| Cg49–Pg1–Ps53 | Cg49–Pg1–Ps54 | Cg49–Pg1–Ps55 | Cg49–Pg1–Ps56 |
| Cg49–Pg1–Ps57 | Cg49–Pg1–Ps58 | Cg49–Pg1–Ps59 | Cg49–Pg1–Ps60 |
| Cg49–Pg1–Ps61 | Cg49–Pg1–Ps62 | Cg49–Pg1–Ps63 | Cg49–Pg1–Ps64 |
| Cg49–Pg1–Ps65 | Cg49–Pg1–Ps66 | Cg49–Pg1–Ps67 | Cg49–Pg1–Ps68 |
| Cg49–Pg1–Ps69 | Cg49–Pg1–Ps70 | Cg49–Pg1–Ps71 | Cg49–Pg1–Ps72 |
| Cg49–Pg1–Ps73 | Cg49–Pg1–Ps74 | Cg49–Pg1–Ps75 | Cg49–Pg1–Ps76 |

| | | | |
|---|---|---|---|
| Cg49–Pg1–Ps77 | Cg49–Pg1–Ps78 | Cg49–Pg1–Ps79 | Cg49–Pg1–Ps80 |
| Cg49–Pg1–Ps81 | Cg49–Pg1–Ps82 | Cg49–Pg1–Ps83 | Cg49–Pg1–Ps84 |
| Cg49–Pg1–Ps85 | Cg49–Pg1–Ps86 | Cg49–Pg1–Ps87 | Cg49–Pg1–Ps88 |
| Cg49–Pg1–Ps89 | Cg49–Pg1–Ps90 | Cg49–Pg1–Ps91 | Cg49–Pg1–Ps92 |
| Cg49–Pg1–Ps93 | Cg49–Pg1–Ps94 | Cg49–Pg1–Ps95 | Cg49–Pg1–Ps96 |
| Cg49–Pg1–Ps97 | Cg49–Pg1–Ps98 | Cg49–Pg1–Ps99 | Cg49–Pg1–Ps100 |
| Cg49–Pg1–Ps101 | Cg49–Pg1–Ps102 | Cg49–Pg1–Ps103 | Cg49–Pg1–Ps104 |
| Cg49–Pg1–Ps105 | Cg49–Pg1–Ps106 | Cg49–Pg1–Ps107 | Cg49–Pg1–Ps108 |
| Cg49–Pg1–Ps109 | Cg49–Pg1–Ps110 | Cg49–Pg1–Ps111 | Cg49–Pg1–Ps112 |
| Cg49–Pg1–Ps113 | Cg49–Pg1–Ps114 | Cg49–Pg1–Ps115 | Cg49–Pg1–Ps116 |
| Cg49–Pg1–Ps117 | Cg49–Pg1–Ps118 | Cg49–Pg1–Ps119 | Cg49–Pg1–Ps120 |
| Cg49–Pg1–Ps121 | Cg49–Pg1–Ps122 | Cg49–Pg1–Ps123 | Cg49–Pg1–Ps124 |
| Cg49–Pg1–Ps125 | Cg49–Pg1–Ps126 | Cg49–Pg1–Ps127 | Cg49–Pg1–Ps128 |
| Cg49–Pg1–Ps129 | Cg49–Pg1–Ps130 | Cg49–Pg1–Ps131 | Cg49–Pg1–Ps132 |
| Cg49–Pg1–Ps133 | Cg49–Pg1–Ps134 | Cg49–Pg1–Ps135 | Cg49–Pg1–Ps136 |
| Cg49–Pg1–Ps137 | Cg49–Pg1–Ps138 | Cg49–Pg1–Ps139 | Cg49–Pg1–Ps140 |
| Cg49–Pg1–Ps141 | Cg49–Pg1–Ps142 | Cg49–Pg1–Ps143 | Cg49–Pg1–Ps144 |
| Cg49–Pg1–Ps145 | Cg49–Pg1–Ps146 | Cg49–Pg1–Ps147 | Cg49–Pg1–Ps148 |
| Cg49–Pg1–Ps149 | Cg49–Pg1–Ps150 | Cg49–Pg1–Ps151 | Cg49–Pg1–Ps152 |
| Cg49–Pg1–Ps153 | Cg49–Pg1–Ps154 | Cg49–Pg1–Ps155 | Cg49–Pg1–Ps156 |
| Cg49–Pg1–Ps157 | Cg49–Pg1–Ps158 | Cg49–Pg1–Ps159 | Cg49–Pg1–Ps160 |
| Cg49–Pg1–Ps161 | Cg49–Pg1–Ps162 | Cg49–Pg1–Ps163 | Cg49–Pg1–Ps164 |
| Cg49–Pg1–Ps165 | Cg49–Pg1–Ps166 | Cg49–Pg1–Ps167 | Cg49–Pg1–Ps168 |
| Cg49–Pg1–Ps169 | Cg49–Pg1–Ps170 | Cg49–Pg1–Ps171 | Cg49–Pg1–Ps172 |
| Cg49–Pg1–Ps173 | Cg49–Pg1–Ps174 | Cg49–Pg1–Ps175 | Cg49–Pg1–Ps176 |
| Cg49–Pg1–Ps177 | Cg49–Pg1–Ps178 | Cg49–Pg1–Ps179 | Cg49–Pg1–Ps180 |
| Cg49–Pg1–Ps181 | Cg49–Pg1–Ps182 | Cg49–Pg1–Ps183 | Cg49–Pg1–Ps184 |
| Cg49–Pg1–Ps185 | Cg49–Pg1–Ps186 | Cg49–Pg1–Ps187 | Cg49–Pg1–Ps188 |
| Cg49–Pg1–Ps189 | Cg49–Pg1–Ps190 | Cg49–Pg1–Ps191 | Cg49–Pg1–Ps192 |
| Cg49–Pg1–Ps193 | Cg49–Pg1–Ps194 | Cg49–Pg1–Ps195 | Cg49–Pg1–Ps196 |
| Cg49–Pg1–Ps197 | Cg49–Pg1–Ps198 | Cg49–Pg1–Ps199 | Cg49–Pg1–Ps200 |
| Cg49–Pg1–Ps201 | Cg49–Pg1–Ps202 | Cg49–Pg1–Ps203 | Cg49–Pg1–Ps204 |
| Cg49–Pg1–Ps205 | Cg49–Pg1–Ps206 | Cg49–Pg1–Ps207 | Cg49–Pg1–Ps208 |
| Cg49–Pg1–Ps209 | Cg49–Pg1–Ps210 | Cg49–Pg1–Ps211 | Cg49–Pg1–Ps212 |
| Cg49–Pg1–Ps213 | Cg49–Pg1–Ps214 | Cg49–Pg1–Ps215 | Cg49–Pg1–Ps216 |
| Cg49–Pg1–Ps217 | Cg49–Pg1–Ps218 | Cg49–Pg1–Ps219 | Cg49–Pg1–Ps220 |
| Cg49–Pg1–Ps221 | Cg49–Pg1–Ps222 | Cg49–Pg1–Ps223 | Cg49–Pg1–Ps224 |
| Cg49–Pg1–Ps225 | Cg49–Pg1–Ps226 | Cg49–Pg1–Ps227 | Cg49–Pg1–Ps228 |
| Cg49–Pg1–Ps229 | Cg49–Pg1–Ps230 | Cg49–Pg1–Ps231 | Cg49–Pg1–Ps232 |
| Cg49–Pg1–Ps233 | Cg49–Pg1–Ps234 | Cg49–Pg1–Ps235 | Cg49–Pg1–Ps236 |
| Cg49–Pg1–Ps237 | Cg49–Pg1–Ps238 | Cg49–Pg1–Ps239 | Cg49–Pg1–Ps240 |
| Cg49–Pg1–Ps241 | Cg49–Pg1–Ps242 | Cg49–Pg1–Ps243 | |

| | | | |
|---|---|---|---|
| Cg50–Pg1–Ps1 | Cg50–Pg1–Ps2 | Cg50–Pg1–Ps3 | Cg50–Pg1–Ps4 |
| Cg50–Pg1–Ps5 | Cg50–Pg1–Ps6 | Cg50–Pg1–Ps7 | Cg50–Pg1–Ps8 |
| Cg50–Pg1–Ps9 | Cg50–Pg1–Ps10 | Cg50–Pg1–Ps11 | Cg50–Pg1–Ps12 |
| Cg50–Pg1–Ps13 | Cg50–Pg1–Ps14 | Cg50–Pg1–Ps15 | Cg50–Pg1–Ps16 |
| Cg50–Pg1–Ps17 | Cg50–Pg1–Ps18 | Cg50–Pg1–Ps19 | Cg50–Pg1–Ps20 |
| Cg50–Pg1–Ps21 | Cg50–Pg1–Ps22 | Cg50–Pg1–Ps23 | Cg50–Pg1–Ps24 |
| Cg50–Pg1–Ps25 | Cg50–Pg1–Ps26 | Cg50–Pg1–Ps27 | Cg50–Pg1–Ps28 |
| Cg50–Pg1–Ps29 | Cg50–Pg1–Ps30 | Cg50–Pg1–Ps31 | Cg50–Pg1–Ps32 |
| Cg50–Pg1–Ps33 | Cg50–Pg1–Ps34 | Cg50–Pg1–Ps35 | Cg50–Pg1–Ps36 |

| | | | |
|---|---|---|---|
| Cg50–Pg1–Ps37 | Cg50–Pg1–Ps38 | Cg50–Pg1–Ps39 | Cg50–Pg1–Ps40 |
| Cg50–Pg1–Ps41 | Cg50–Pg1–Ps42 | Cg50–Pg1–Ps43 | Cg50–Pg1–Ps44 |
| Cg50–Pg1–Ps45 | Cg50–Pg1–Ps46 | Cg50–Pg1–Ps47 | Cg50–Pg1–Ps48 |
| Cg50–Pg1–Ps49 | Cg50–Pg1–Ps50 | Cg50–Pg1–Ps51 | Cg50–Pg1–Ps52 |
| Cg50–Pg1–Ps53 | Cg50–Pg1–Ps54 | Cg50–Pg1–Ps55 | Cg50–Pg1–Ps56 |
| Cg50–Pg1–Ps57 | Cg50–Pg1–Ps58 | Cg50–Pg1–Ps59 | Cg50–Pg1–Ps60 |
| Cg50–Pg1–Ps61 | Cg50–Pg1–Ps62 | Cg50–Pg1–Ps63 | Cg50–Pg1–Ps64 |
| Cg50–Pg1–Ps65 | Cg50–Pg1–Ps66 | Cg50–Pg1–Ps67 | Cg50–Pg1–Ps68 |
| Cg50–Pg1–Ps69 | Cg50–Pg1–Ps70 | Cg50–Pg1–Ps71 | Cg50–Pg1–Ps72 |
| Cg50–Pg1–Ps73 | Cg50–Pg1–Ps74 | Cg50–Pg1–Ps75 | Cg50–Pg1–Ps76 |
| Cg50–Pg1–Ps77 | Cg50–Pg1–Ps78 | Cg50–Pg1–Ps79 | Cg50–Pg1–Ps80 |
| Cg50–Pg1–Ps81 | Cg50–Pg1–Ps82 | Cg50–Pg1–Ps83 | Cg50–Pg1–Ps84 |
| Cg50–Pg1–Ps85 | Cg50–Pg1–Ps86 | Cg50–Pg1–Ps87 | Cg50–Pg1–Ps88 |
| Cg50–Pg1–Ps89 | Cg50–Pg1–Ps90 | Cg50–Pg1–Ps91 | Cg50–Pg1–Ps92 |
| Cg50–Pg1–Ps93 | Cg50–Pg1–Ps94 | Cg50–Pg1–Ps95 | Cg50–Pg1–Ps96 |
| Cg50–Pg1–Ps97 | Cg50–Pg1–Ps98 | Cg50–Pg1–Ps99 | Cg50–Pg1–Ps100 |
| Cg50–Pg1–Ps101 | Cg50–Pg1–Ps102 | Cg50–Pg1–Ps103 | Cg50–Pg1–Ps104 |
| Cg50–Pg1–Ps105 | Cg50–Pg1–Ps106 | Cg50–Pg1–Ps107 | Cg50–Pg1–Ps108 |
| Cg50–Pg1–Ps109 | Cg50–Pg1–Ps110 | Cg50–Pg1–Ps111 | Cg50–Pg1–Ps112 |
| Cg50–Pg1–Ps113 | Cg50–Pg1–Ps114 | Cg50–Pg1–Ps115 | Cg50–Pg1–Ps116 |
| Cg50–Pg1–Ps117 | Cg50–Pg1–Ps118 | Cg50–Pg1–Ps119 | Cg50–Pg1–Ps120 |
| Cg50–Pg1–Ps121 | Cg50–Pg1–Ps122 | Cg50–Pg1–Ps123 | Cg50–Pg1–Ps124 |
| Cg50–Pg1–Ps125 | Cg50–Pg1–Ps126 | Cg50–Pg1–Ps127 | Cg50–Pg1–Ps128 |
| Cg50–Pg1–Ps129 | Cg50–Pg1–Ps130 | Cg50–Pg1–Ps131 | Cg50–Pg1–Ps132 |
| Cg50–Pg1–Ps133 | Cg50–Pg1–Ps134 | Cg50–Pg1–Ps135 | Cg50–Pg1–Ps136 |
| Cg50–Pg1–Ps137 | Cg50–Pg1–Ps138 | Cg50–Pg1–Ps139 | Cg50–Pg1–Ps140 |
| Cg50–Pg1–Ps141 | Cg50–Pg1–Ps142 | Cg50–Pg1–Ps143 | Cg50–Pg1–Ps144 |
| Cg50–Pg1–Ps145 | Cg50–Pg1–Ps146 | Cg50–Pg1–Ps147 | Cg50–Pg1–Ps148 |
| Cg50–Pg1–Ps149 | Cg50–Pg1–Ps150 | Cg50–Pg1–Ps151 | Cg50–Pg1–Ps152 |
| Cg50–Pg1–Ps153 | Cg50–Pg1–Ps154 | Cg50–Pg1–Ps155 | Cg50–Pg1–Ps156 |
| Cg50–Pg1–Ps157 | Cg50–Pg1–Ps158 | Cg50–Pg1–Ps159 | Cg50–Pg1–Ps160 |
| Cg50–Pg1–Ps161 | Cg50–Pg1–Ps162 | Cg50–Pg1–Ps163 | Cg50–Pg1–Ps164 |
| Cg50–Pg1–Ps165 | Cg50–Pg1–Ps166 | Cg50–Pg1–Ps167 | Cg50–Pg1–Ps168 |
| Cg50–Pg1–Ps169 | Cg50–Pg1–Ps170 | Cg50–Pg1–Ps171 | Cg50–Pg1–Ps172 |
| Cg50–Pg1–Ps173 | Cg50–Pg1–Ps174 | Cg50–Pg1–Ps175 | Cg50–Pg1–Ps176 |
| Cg50–Pg1–Ps177 | Cg50–Pg1–Ps178 | Cg50–Pg1–Ps179 | Cg50–Pg1–Ps180 |
| Cg50–Pg1–Ps181 | Cg50–Pg1–Ps182 | Cg50–Pg1–Ps183 | Cg50–Pg1–Ps184 |
| Cg50–Pg1–Ps185 | Cg50–Pg1–Ps186 | Cg50–Pg1–Ps187 | Cg50–Pg1–Ps188 |
| Cg50–Pg1–Ps189 | Cg50–Pg1–Ps190 | Cg50–Pg1–Ps191 | Cg50–Pg1–Ps192 |
| Cg50–Pg1–Ps193 | Cg50–Pg1–Ps194 | Cg50–Pg1–Ps195 | Cg50–Pg1–Ps196 |
| Cg50–Pg1–Ps197 | Cg50–Pg1–Ps198 | Cg50–Pg1–Ps199 | Cg50–Pg1–Ps200 |
| Cg50–Pg1–Ps201 | Cg50–Pg1–Ps202 | Cg50–Pg1–Ps203 | Cg50–Pg1–Ps204 |
| Cg50–Pg1–Ps205 | Cg50–Pg1–Ps206 | Cg50–Pg1–Ps207 | Cg50–Pg1–Ps208 |
| Cg50–Pg1–Ps209 | Cg50–Pg1–Ps210 | Cg50–Pg1–Ps211 | Cg50–Pg1–Ps212 |
| Cg50–Pg1–Ps213 | Cg50–Pg1–Ps214 | Cg50–Pg1–Ps215 | Cg50–Pg1–Ps216 |
| Cg50–Pg1–Ps217 | Cg50–Pg1–Ps218 | Cg50–Pg1–Ps219 | Cg50–Pg1–Ps220 |
| Cg50–Pg1–Ps221 | Cg50–Pg1–Ps222 | Cg50–Pg1–Ps223 | Cg50–Pg1–Ps224 |
| Cg50–Pg1–Ps225 | Cg50–Pg1–Ps226 | Cg50–Pg1–Ps227 | Cg50–Pg1–Ps228 |
| Cg50–Pg1–Ps229 | Cg50–Pg1–Ps230 | Cg50–Pg1–Ps231 | Cg50–Pg1–Ps232 |
| Cg50–Pg1–Ps233 | Cg50–Pg1–Ps234 | Cg50–Pg1–Ps235 | Cg50–Pg1–Ps236 |
| Cg50–Pg1–Ps237 | Cg50–Pg1–Ps238 | Cg50–Pg1–Ps239 | Cg50–Pg1–Ps240 |
| Cg50–Pg1–Ps241 | Cg50–Pg1–Ps242 | Cg50–Pg1–Ps243 | |

| | | | |
|---|---|---|---|
| Cg51–Pg1–Ps1 | Cg51–Pg1–Ps2 | Cg51–Pg1–Ps3 | Cg51–Pg1–Ps4 |
| Cg51–Pg1–Ps5 | Cg51–Pg1–Ps6 | Cg51–Pg1–Ps7 | Cg51–Pg1–Ps8 |
| Cg51–Pg1–Ps9 | Cg51–Pg1–Ps10 | Cg51–Pg1–Ps11 | Cg51–Pg1–Ps12 |
| Cg51–Pg1–Ps13 | Cg51–Pg1–Ps14 | Cg51–Pg1–Ps15 | Cg51–Pg1–Ps16 |
| Cg51–Pg1–Ps17 | Cg51–Pg1–Ps18 | Cg51–Pg1–Ps19 | Cg51–Pg1–Ps20 |
| Cg51–Pg1–Ps21 | Cg51–Pg1–Ps22 | Cg51–Pg1–Ps23 | Cg51–Pg1–Ps24 |
| Cg51–Pg1–Ps25 | Cg51–Pg1–Ps26 | Cg51–Pg1–Ps27 | Cg51–Pg1–Ps28 |
| Cg51–Pg1–Ps29 | Cg51–Pg1–Ps30 | Cg51–Pg1–Ps31 | Cg51–Pg1–Ps32 |
| Cg51–Pg1–Ps33 | Cg51–Pg1–Ps34 | Cg51–Pg1–Ps35 | Cg51–Pg1–Ps36 |
| Cg51–Pg1–Ps37 | Cg51–Pg1–Ps38 | Cg51–Pg1–Ps39 | Cg51–Pg1–Ps40 |
| Cg51–Pg1–Ps41 | Cg51–Pg1–Ps42 | Cg51–Pg1–Ps43 | Cg51–Pg1–Ps44 |
| Cg51–Pg1–Ps45 | Cg51–Pg1–Ps46 | Cg51–Pg1–Ps47 | Cg51–Pg1–Ps48 |
| Cg51–Pg1–Ps49 | Cg51–Pg1–Ps50 | Cg51–Pg1–Ps51 | Cg51–Pg1–Ps52 |
| Cg51–Pg1–Ps53 | Cg51–Pg1–Ps54 | Cg51–Pg1–Ps55 | Cg51–Pg1–Ps56 |
| Cg51–Pg1–Ps57 | Cg51–Pg1–Ps58 | Cg51–Pg1–Ps59 | Cg51–Pg1–Ps60 |
| Cg51–Pg1–Ps61 | Cg51–Pg1–Ps62 | Cg51–Pg1–Ps63 | Cg51–Pg1–Ps64 |
| Cg51–Pg1–Ps65 | Cg51–Pg1–Ps66 | Cg51–Pg1–Ps67 | Cg51–Pg1–Ps68 |
| Cg51–Pg1–Ps69 | Cg51–Pg1–Ps70 | Cg51–Pg1–Ps71 | Cg51–Pg1–Ps72 |
| Cg51–Pg1–Ps73 | Cg51–Pg1–Ps74 | Cg51–Pg1–Ps75 | Cg51–Pg1–Ps76 |
| Cg51–Pg1–Ps77 | Cg51–Pg1–Ps78 | Cg51–Pg1–Ps79 | Cg51–Pg1–Ps80 |
| Cg51–Pg1–Ps81 | Cg51–Pg1–Ps82 | Cg51–Pg1–Ps83 | Cg51–Pg1–Ps84 |
| Cg51–Pg1–Ps85 | Cg51–Pg1–Ps86 | Cg51–Pg1–Ps87 | Cg51–Pg1–Ps88 |
| Cg51–Pg1–Ps89 | Cg51–Pg1–Ps90 | Cg51–Pg1–Ps91 | Cg51–Pg1–Ps92 |
| Cg51–Pg1–Ps93 | Cg51–Pg1–Ps94 | Cg51–Pg1–Ps95 | Cg51–Pg1–Ps96 |
| Cg51–Pg1–Ps97 | Cg51–Pg1–Ps98 | Cg51–Pg1–Ps99 | Cg51–Pg1–Ps100 |
| Cg51–Pg1–Ps101 | Cg51–Pg1–Ps102 | Cg51–Pg1–Ps103 | Cg51–Pg1–Ps104 |
| Cg51–Pg1–Ps105 | Cg51–Pg1–Ps106 | Cg51–Pg1–Ps107 | Cg51–Pg1–Ps108 |
| Cg51–Pg1–Ps109 | Cg51–Pg1–Ps110 | Cg51–Pg1–Ps111 | Cg51–Pg1–Ps112 |
| Cg51–Pg1–Ps113 | Cg51–Pg1–Ps114 | Cg51–Pg1–Ps115 | Cg51–Pg1–Ps116 |
| Cg51–Pg1–Ps117 | Cg51–Pg1–Ps118 | Cg51–Pg1–Ps119 | Cg51–Pg1–Ps120 |
| Cg51–Pg1–Ps121 | Cg51–Pg1–Ps122 | Cg51–Pg1–Ps123 | Cg51–Pg1–Ps124 |
| Cg51–Pg1–Ps125 | Cg51–Pg1–Ps126 | Cg51–Pg1–Ps127 | Cg51–Pg1–Ps128 |
| Cg51–Pg1–Ps129 | Cg51–Pg1–Ps130 | Cg51–Pg1–Ps131 | Cg51–Pg1–Ps132 |
| Cg51–Pg1–Ps133 | Cg51–Pg1–Ps134 | Cg51–Pg1–Ps135 | Cg51–Pg1–Ps136 |
| Cg51–Pg1–Ps137 | Cg51–Pg1–Ps138 | Cg51–Pg1–Ps139 | Cg51–Pg1–Ps140 |
| Cg51–Pg1–Ps141 | Cg51–Pg1–Ps142 | Cg51–Pg1–Ps143 | Cg51–Pg1–Ps144 |
| Cg51–Pg1–Ps145 | Cg51–Pg1–Ps146 | Cg51–Pg1–Ps147 | Cg51–Pg1–Ps148 |
| Cg51–Pg1–Ps149 | Cg51–Pg1–Ps150 | Cg51–Pg1–Ps151 | Cg51–Pg1–Ps152 |
| Cg51–Pg1–Ps153 | Cg51–Pg1–Ps154 | Cg51–Pg1–Ps155 | Cg51–Pg1–Ps156 |
| Cg51–Pg1–Ps157 | Cg51–Pg1–Ps158 | Cg51–Pg1–Ps159 | Cg51–Pg1–Ps160 |
| Cg51–Pg1–Ps161 | Cg51–Pg1–Ps162 | Cg51–Pg1–Ps163 | Cg51–Pg1–Ps164 |
| Cg51–Pg1–Ps165 | Cg51–Pg1–Ps166 | Cg51–Pg1–Ps167 | Cg51–Pg1–Ps168 |
| Cg51–Pg1–Ps169 | Cg51–Pg1–Ps170 | Cg51–Pg1–Ps171 | Cg51–Pg1–Ps172 |
| Cg51–Pg1–Ps173 | Cg51–Pg1–Ps174 | Cg51–Pg1–Ps175 | Cg51–Pg1–Ps176 |
| Cg51–Pg1–Ps177 | Cg51–Pg1–Ps178 | Cg51–Pg1–Ps179 | Cg51–Pg1–Ps180 |
| Cg51–Pg1–Ps181 | Cg51–Pg1–Ps182 | Cg51–Pg1–Ps183 | Cg51–Pg1–Ps184 |
| Cg51–Pg1–Ps185 | Cg51–Pg1–Ps186 | Cg51–Pg1–Ps187 | Cg51–Pg1–Ps188 |
| Cg51–Pg1–Ps189 | Cg51–Pg1–Ps190 | Cg51–Pg1–Ps191 | Cg51–Pg1–Ps192 |
| Cg51–Pg1–Ps193 | Cg51–Pg1–Ps194 | Cg51–Pg1–Ps195 | Cg51–Pg1–Ps196 |
| Cg51–Pg1–Ps197 | Cg51–Pg1–Ps198 | Cg51–Pg1–Ps199 | Cg51–Pg1–Ps200 |
| Cg51–Pg1–Ps201 | Cg51–Pg1–Ps202 | Cg51–Pg1–Ps203 | Cg51–Pg1–Ps204 |

| | | | |
|---|---|---|---|
| Cg51–Pg1–Ps205 | Cg51–Pg1–Ps206 | Cg51–Pg1–Ps207 | Cg51–Pg1–Ps208 |
| Cg51–Pg1–Ps209 | Cg51–Pg1–Ps210 | Cg51–Pg1–Ps211 | Cg51–Pg1–Ps212 |
| Cg51–Pg1–Ps213 | Cg51–Pg1–Ps214 | Cg51–Pg1–Ps215 | Cg51–Pg1–Ps216 |
| Cg51–Pg1–Ps217 | Cg51–Pg1–Ps218 | Cg51–Pg1–Ps219 | Cg51–Pg1–Ps220 |
| Cg51–Pg1–Ps221 | Cg51–Pg1–Ps222 | Cg51–Pg1–Ps223 | Cg51–Pg1–Ps224 |
| Cg51–Pg1–Ps225 | Cg51–Pg1–Ps226 | Cg51–Pg1–Ps227 | Cg51–Pg1–Ps228 |
| Cg51–Pg1–Ps229 | Cg51–Pg1–Ps230 | Cg51–Pg1–Ps231 | Cg51–Pg1–Ps232 |
| Cg51–Pg1–Ps233 | Cg51–Pg1–Ps234 | Cg51–Pg1–Ps235 | Cg51–Pg1–Ps236 |
| Cg51–Pg1–Ps237 | Cg51–Pg1–Ps238 | Cg51–Pg1–Ps239 | Cg51–Pg1–Ps240 |
| Cg51–Pg1–Ps241 | Cg51–Pg1–Ps242 | Cg51–Pg1–Ps243 | |

| | | | |
|---|---|---|---|
| Cg52–Pg1–Ps1 | Cg52–Pg1–Ps2 | Cg52–Pg1–Ps3 | Cg52–Pg1–Ps4 |
| Cg52–Pg1–Ps5 | Cg52–Pg1–Ps6 | Cg52–Pg1–Ps7 | Cg52–Pg1–Ps8 |
| Cg52–Pg1–Ps9 | Cg52–Pg1–Ps10 | Cg52–Pg1–Ps11 | Cg52–Pg1–Ps12 |
| Cg52–Pg1–Ps13 | Cg52–Pg1–Ps14 | Cg52–Pg1–Ps15 | Cg52–Pg1–Ps16 |
| Cg52–Pg1–Ps17 | Cg52–Pg1–Ps18 | Cg52–Pg1–Ps19 | Cg52–Pg1–Ps20 |
| Cg52–Pg1–Ps21 | Cg52–Pg1–Ps22 | Cg52–Pg1–Ps23 | Cg52–Pg1–Ps24 |
| Cg52–Pg1–Ps25 | Cg52–Pg1–Ps26 | Cg52–Pg1–Ps27 | Cg52–Pg1–Ps28 |
| Cg52–Pg1–Ps29 | Cg52–Pg1–Ps30 | Cg52–Pg1–Ps31 | Cg52–Pg1–Ps32 |
| Cg52–Pg1–Ps33 | Cg52–Pg1–Ps34 | Cg52–Pg1–Ps35 | Cg52–Pg1–Ps36 |
| Cg52–Pg1–Ps37 | Cg52–Pg1–Ps38 | Cg52–Pg1–Ps39 | Cg52–Pg1–Ps40 |
| Cg52–Pg1–Ps41 | Cg52–Pg1–Ps42 | Cg52–Pg1–Ps43 | Cg52–Pg1–Ps44 |
| Cg52–Pg1–Ps45 | Cg52–Pg1–Ps46 | Cg52–Pg1–Ps47 | Cg52–Pg1–Ps48 |
| Cg52–Pg1–Ps49 | Cg52–Pg1–Ps50 | Cg52–Pg1–Ps51 | Cg52–Pg1–Ps52 |
| Cg52–Pg1–Ps53 | Cg52–Pg1–Ps54 | Cg52–Pg1–Ps55 | Cg52–Pg1–Ps56 |
| Cg52–Pg1–Ps57 | Cg52–Pg1–Ps58 | Cg52–Pg1–Ps59 | Cg52–Pg1–Ps60 |
| Cg52–Pg1–Ps61 | Cg52–Pg1–Ps62 | Cg52–Pg1–Ps63 | Cg52–Pg1–Ps64 |
| Cg52–Pg1–Ps65 | Cg52–Pg1–Ps66 | Cg52–Pg1–Ps67 | Cg52–Pg1–Ps68 |
| Cg52–Pg1–Ps69 | Cg52–Pg1–Ps70 | Cg52–Pg1–Ps71 | Cg52–Pg1–Ps72 |
| Cg52–Pg1–Ps73 | Cg52–Pg1–Ps74 | Cg52–Pg1–Ps75 | Cg52–Pg1–Ps76 |
| Cg52–Pg1–Ps77 | Cg52–Pg1–Ps78 | Cg52–Pg1–Ps79 | Cg52–Pg1–Ps80 |
| Cg52–Pg1–Ps81 | Cg52–Pg1–Ps82 | Cg52–Pg1–Ps83 | Cg52–Pg1–Ps84 |
| Cg52–Pg1–Ps85 | Cg52–Pg1–Ps86 | Cg52–Pg1–Ps87 | Cg52–Pg1–Ps88 |
| Cg52–Pg1–Ps89 | Cg52–Pg1–Ps90 | Cg52–Pg1–Ps91 | Cg52–Pg1–Ps92 |
| Cg52–Pg1–Ps93 | Cg52–Pg1–Ps94 | Cg52–Pg1–Ps95 | Cg52–Pg1–Ps96 |
| Cg52–Pg1–Ps97 | Cg52–Pg1–Ps98 | Cg52–Pg1–Ps99 | Cg52–Pg1–Ps100 |
| Cg52–Pg1–Ps101 | Cg52–Pg1–Ps102 | Cg52–Pg1–Ps103 | Cg52–Pg1–Ps104 |
| Cg52–Pg1–Ps105 | Cg52–Pg1–Ps106 | Cg52–Pg1–Ps107 | Cg52–Pg1–Ps108 |
| Cg52–Pg1–Ps109 | Cg52–Pg1–Ps110 | Cg52–Pg1–Ps111 | Cg52–Pg1–Ps112 |
| Cg52–Pg1–Ps113 | Cg52–Pg1–Ps114 | Cg52–Pg1–Ps115 | Cg52–Pg1–Ps116 |
| Cg52–Pg1–Ps117 | Cg52–Pg1–Ps118 | Cg52–Pg1–Ps119 | Cg52–Pg1–Ps120 |
| Cg52–Pg1–Ps121 | Cg52–Pg1–Ps122 | Cg52–Pg1–Ps123 | Cg52–Pg1–Ps124 |
| Cg52–Pg1–Ps125 | Cg52–Pg1–Ps126 | Cg52–Pg1–Ps127 | Cg52–Pg1–Ps128 |
| Cg52–Pg1–Ps129 | Cg52–Pg1–Ps130 | Cg52–Pg1–Ps131 | Cg52–Pg1–Ps132 |
| Cg52–Pg1–Ps133 | Cg52–Pg1–Ps134 | Cg52–Pg1–Ps135 | Cg52–Pg1–Ps136 |
| Cg52–Pg1–Ps137 | Cg52–Pg1–Ps138 | Cg52–Pg1–Ps139 | Cg52–Pg1–Ps140 |
| Cg52–Pg1–Ps141 | Cg52–Pg1–Ps142 | Cg52–Pg1–Ps143 | Cg52–Pg1–Ps144 |
| Cg52–Pg1–Ps145 | Cg52–Pg1–Ps146 | Cg52–Pg1–Ps147 | Cg52–Pg1–Ps148 |
| Cg52–Pg1–Ps149 | Cg52–Pg1–Ps150 | Cg52–Pg1–Ps151 | Cg52–Pg1–Ps152 |
| Cg52–Pg1–Ps153 | Cg52–Pg1–Ps154 | Cg52–Pg1–Ps155 | Cg52–Pg1–Ps156 |
| Cg52–Pg1–Ps157 | Cg52–Pg1–Ps158 | Cg52–Pg1–Ps159 | Cg52–Pg1–Ps160 |
| Cg52–Pg1–Ps161 | Cg52–Pg1–Ps162 | Cg52–Pg1–Ps163 | Cg52–Pg1–Ps164 |

| | | | |
|---|---|---|---|
| Cg52-Pg1-Ps165 | Cg52-Pg1-Ps166 | Cg52-Pg1-Ps167 | Cg52-Pg1-Ps168 |
| Cg52-Pg1-Ps169 | Cg52-Pg1-Ps170 | Cg52-Pg1-Ps171 | Cg52-Pg1-Ps172 |
| Cg52-Pg1-Ps173 | Cg52-Pg1-Ps174 | Cg52-Pg1-Ps175 | Cg52-Pg1-Ps176 |
| Cg52-Pg1-Ps177 | Cg52-Pg1-Ps178 | Cg52-Pg1-Ps179 | Cg52-Pg1-Ps180 |
| Cg52-Pg1-Ps181 | Cg52-Pg1-Ps182 | Cg52-Pg1-Ps183 | Cg52-Pg1-Ps184 |
| Cg52-Pg1-Ps185 | Cg52-Pg1-Ps186 | Cg52-Pg1-Ps187 | Cg52-Pg1-Ps188 |
| Cg52-Pg1-Ps189 | Cg52-Pg1-Ps190 | Cg52-Pg1-Ps191 | Cg52-Pg1-Ps192 |
| Cg52-Pg1-Ps193 | Cg52-Pg1-Ps194 | Cg52-Pg1-Ps195 | Cg52-Pg1-Ps196 |
| Cg52-Pg1-Ps197 | Cg52-Pg1-Ps198 | Cg52-Pg1-Ps199 | Cg52-Pg1-Ps200 |
| Cg52-Pg1-Ps201 | Cg52-Pg1-Ps202 | Cg52-Pg1-Ps203 | Cg52-Pg1-Ps204 |
| Cg52-Pg1-Ps205 | Cg52-Pg1-Ps206 | Cg52-Pg1-Ps207 | Cg52-Pg1-Ps208 |
| Cg52-Pg1-Ps209 | Cg52-Pg1-Ps210 | Cg52-Pg1-Ps211 | Cg52-Pg1-Ps212 |
| Cg52-Pg1-Ps213 | Cg52-Pg1-Ps214 | Cg52-Pg1-Ps215 | Cg52-Pg1-Ps216 |
| Cg52-Pg1-Ps217 | Cg52-Pg1-Ps218 | Cg52-Pg1-Ps219 | Cg52-Pg1-Ps220 |
| Cg52-Pg1-Ps221 | Cg52-Pg1-Ps222 | Cg52-Pg1-Ps223 | Cg52-Pg1-Ps224 |
| Cg52-Pg1-Ps225 | Cg52-Pg1-Ps226 | Cg52-Pg1-Ps227 | Cg52-Pg1-Ps228 |
| Cg52-Pg1-Ps229 | Cg52-Pg1-Ps230 | Cg52-Pg1-Ps231 | Cg52-Pg1-Ps232 |
| Cg52-Pg1-Ps233 | Cg52-Pg1-Ps234 | Cg52-Pg1-Ps235 | Cg52-Pg1-Ps236 |
| Cg52-Pg1-Ps237 | Cg52-Pg1-Ps238 | Cg52-Pg1-Ps239 | Cg52-Pg1-Ps240 |
| Cg52-Pg1-Ps241 | Cg52-Pg1-Ps242 | Cg52-Pg1-Ps243 | |

| | | | |
|---|---|---|---|
| Cg53-Pg1-Ps1 | Cg53-Pg1-Ps2 | Cg53-Pg1-Ps3 | Cg53-Pg1-Ps4 |
| Cg53-Pg1-Ps5 | Cg53-Pg1-Ps6 | Cg53-Pg1-Ps7 | Cg53-Pg1-Ps8 |
| Cg53-Pg1-Ps9 | Cg53-Pg1-Ps10 | Cg53-Pg1-Ps11 | Cg53-Pg1-Ps12 |
| Cg53-Pg1-Ps13 | Cg53-Pg1-Ps14 | Cg53-Pg1-Ps15 | Cg53-Pg1-Ps16 |
| Cg53-Pg1-Ps17 | Cg53-Pg1-Ps18 | Cg53-Pg1-Ps19 | Cg53-Pg1-Ps20 |
| Cg53-Pg1-Ps21 | Cg53-Pg1-Ps22 | Cg53-Pg1-Ps23 | Cg53-Pg1-Ps24 |
| Cg53-Pg1-Ps25 | Cg53-Pg1-Ps26 | Cg53-Pg1-Ps27 | Cg53-Pg1-Ps28 |
| Cg53-Pg1-Ps29 | Cg53-Pg1-Ps30 | Cg53-Pg1-Ps31 | Cg53-Pg1-Ps32 |
| Cg53-Pg1-Ps33 | Cg53-Pg1-Ps34 | Cg53-Pg1-Ps35 | Cg53-Pg1-Ps36 |
| Cg53-Pg1-Ps37 | Cg53-Pg1-Ps38 | Cg53-Pg1-Ps39 | Cg53-Pg1-Ps40 |
| Cg53-Pg1-Ps41 | Cg53-Pg1-Ps42 | Cg53-Pg1-Ps43 | Cg53-Pg1-Ps44 |
| Cg53-Pg1-Ps45 | Cg53-Pg1-Ps46 | Cg53-Pg1-Ps47 | Cg53-Pg1-Ps48 |
| Cg53-Pg1-Ps49 | Cg53-Pg1-Ps50 | Cg53-Pg1-Ps51 | Cg53-Pg1-Ps52 |
| Cg53-Pg1-Ps53 | Cg53-Pg1-Ps54 | Cg53-Pg1-Ps55 | Cg53-Pg1-Ps56 |
| Cg53-Pg1-Ps57 | Cg53-Pg1-Ps58 | Cg53-Pg1-Ps59 | Cg53-Pg1-Ps60 |
| Cg53-Pg1-Ps61 | Cg53-Pg1-Ps62 | Cg53-Pg1-Ps63 | Cg53-Pg1-Ps64 |
| Cg53-Pg1-Ps65 | Cg53-Pg1-Ps66 | Cg53-Pg1-Ps67 | Cg53-Pg1-Ps68 |
| Cg53-Pg1-Ps69 | Cg53-Pg1-Ps70 | Cg53-Pg1-Ps71 | Cg53-Pg1-Ps72 |
| Cg53-Pg1-Ps73 | Cg53-Pg1-Ps74 | Cg53-Pg1-Ps75 | Cg53-Pg1-Ps76 |
| Cg53-Pg1-Ps77 | Cg53-Pg1-Ps78 | Cg53-Pg1-Ps79 | Cg53-Pg1-Ps80 |
| Cg53-Pg1-Ps81 | Cg53-Pg1-Ps82 | Cg53-Pg1-Ps83 | Cg53-Pg1-Ps84 |
| Cg53-Pg1-Ps85 | Cg53-Pg1-Ps86 | Cg53-Pg1-Ps87 | Cg53-Pg1-Ps88 |
| Cg53-Pg1-Ps89 | Cg53-Pg1-Ps90 | Cg53-Pg1-Ps91 | Cg53-Pg1-Ps92 |
| Cg53-Pg1-Ps93 | Cg53-Pg1-Ps94 | Cg53-Pg1-Ps95 | Cg53-Pg1-Ps96 |
| Cg53-Pg1-Ps97 | Cg53-Pg1-Ps98 | Cg53-Pg1-Ps99 | Cg53-Pg1-Ps100 |
| Cg53-Pg1-Ps101 | Cg53-Pg1-Ps102 | Cg53-Pg1-Ps103 | Cg53-Pg1-Ps104 |
| Cg53-Pg1-Ps105 | Cg53-Pg1-Ps106 | Cg53-Pg1-Ps107 | Cg53-Pg1-Ps108 |
| Cg53-Pg1-Ps109 | Cg53-Pg1-Ps110 | Cg53-Pg1-Ps111 | Cg53-Pg1-Ps112 |
| Cg53-Pg1-Ps113 | Cg53-Pg1-Ps114 | Cg53-Pg1-Ps115 | Cg53-Pg1-Ps116 |
| Cg53-Pg1-Ps117 | Cg53-Pg1-Ps118 | Cg53-Pg1-Ps119 | Cg53-Pg1-Ps120 |
| Cg53-Pg1-Ps121 | Cg53-Pg1-Ps122 | Cg53-Pg1-Ps123 | Cg53-Pg1-Ps124 |

| | | | |
|---|---|---|---|
| Cg53–Pg1–Ps125 | Cg53–Pg1–Ps126 | Cg53–Pg1–Ps127 | Cg53–Pg1–Ps128 |
| Cg53–Pg1–Ps129 | Cg53–Pg1–Ps130 | Cg53–Pg1–Ps131 | Cg53–Pg1–Ps132 |
| Cg53–Pg1–Ps133 | Cg53–Pg1–Ps134 | Cg53–Pg1–Ps135 | Cg53–Pg1–Ps136 |
| Cg53–Pg1–Ps137 | Cg53–Pg1–Ps138 | Cg53–Pg1–Ps139 | Cg53–Pg1–Ps140 |
| Cg53–Pg1–Ps141 | Cg53–Pg1–Ps142 | Cg53–Pg1–Ps143 | Cg53–Pg1–Ps144 |
| Cg53–Pg1–Ps145 | Cg53–Pg1–Ps146 | Cg53–Pg1–Ps147 | Cg53–Pg1–Ps148 |
| Cg53–Pg1–Ps149 | Cg53–Pg1–Ps150 | Cg53–Pg1–Ps151 | Cg53–Pg1–Ps152 |
| Cg53–Pg1–Ps153 | Cg53–Pg1–Ps154 | Cg53–Pg1–Ps155 | Cg53–Pg1–Ps156 |
| Cg53–Pg1–Ps157 | Cg53–Pg1–Ps158 | Cg53–Pg1–Ps159 | Cg53–Pg1–Ps160 |
| Cg53–Pg1–Ps161 | Cg53–Pg1–Ps162 | Cg53–Pg1–Ps163 | Cg53–Pg1–Ps164 |
| Cg53–Pg1–Ps165 | Cg53–Pg1–Ps166 | Cg53–Pg1–Ps167 | Cg53–Pg1–Ps168 |
| Cg53–Pg1–Ps169 | Cg53–Pg1–Ps170 | Cg53–Pg1–Ps171 | Cg53–Pg1–Ps172 |
| Cg53–Pg1–Ps173 | Cg53–Pg1–Ps174 | Cg53–Pg1–Ps175 | Cg53–Pg1–Ps176 |
| Cg53–Pg1–Ps177 | Cg53–Pg1–Ps178 | Cg53–Pg1–Ps179 | Cg53–Pg1–Ps180 |
| Cg53–Pg1–Ps181 | Cg53–Pg1–Ps182 | Cg53–Pg1–Ps183 | Cg53–Pg1–Ps184 |
| Cg53–Pg1–Ps185 | Cg53–Pg1–Ps186 | Cg53–Pg1–Ps187 | Cg53–Pg1–Ps188 |
| Cg53–Pg1–Ps189 | Cg53–Pg1–Ps190 | Cg53–Pg1–Ps191 | Cg53–Pg1–Ps192 |
| Cg53–Pg1–Ps193 | Cg53–Pg1–Ps194 | Cg53–Pg1–Ps195 | Cg53–Pg1–Ps196 |
| Cg53–Pg1–Ps197 | Cg53–Pg1–Ps198 | Cg53–Pg1–Ps199 | Cg53–Pg1–Ps200 |
| Cg53–Pg1–Ps201 | Cg53–Pg1–Ps202 | Cg53–Pg1–Ps203 | Cg53–Pg1–Ps204 |
| Cg53–Pg1–Ps205 | Cg53–Pg1–Ps206 | Cg53–Pg1–Ps207 | Cg53–Pg1–Ps208 |
| Cg53–Pg1–Ps209 | Cg53–Pg1–Ps210 | Cg53–Pg1–Ps211 | Cg53–Pg1–Ps212 |
| Cg53–Pg1–Ps213 | Cg53–Pg1–Ps214 | Cg53–Pg1–Ps215 | Cg53–Pg1–Ps216 |
| Cg53–Pg1–Ps217 | Cg53–Pg1–Ps218 | Cg53–Pg1–Ps219 | Cg53–Pg1–Ps220 |
| Cg53–Pg1–Ps221 | Cg53–Pg1–Ps222 | Cg53–Pg1–Ps223 | Cg53–Pg1–Ps224 |
| Cg53–Pg1–Ps225 | Cg53–Pg1–Ps226 | Cg53–Pg1–Ps227 | Cg53–Pg1–Ps228 |
| Cg53–Pg1–Ps229 | Cg53–Pg1–Ps230 | Cg53–Pg1–Ps231 | Cg53–Pg1–Ps232 |
| Cg53–Pg1–Ps233 | Cg53–Pg1–Ps234 | Cg53–Pg1–Ps235 | Cg53–Pg1–Ps236 |
| Cg53–Pg1–Ps237 | Cg53–Pg1–Ps238 | Cg53–Pg1–Ps239 | Cg53–Pg1–Ps240 |
| Cg53–Pg1–Ps241 | Cg53–Pg1–Ps242 | Cg53–Pg1–Ps243 | |

| | | | |
|---|---|---|---|
| Cg54–Pg1–Ps1 | Cg54–Pg1–Ps2 | Cg54–Pg1–Ps3 | Cg54–Pg1–Ps4 |
| Cg54–Pg1–Ps5 | Cg54–Pg1–Ps6 | Cg54–Pg1–Ps7 | Cg54–Pg1–Ps8 |
| Cg54–Pg1–Ps9 | Cg54–Pg1–Ps10 | Cg54–Pg1–Ps11 | Cg54–Pg1–Ps12 |
| Cg54–Pg1–Ps13 | Cg54–Pg1–Ps14 | Cg54–Pg1–Ps15 | Cg54–Pg1–Ps16 |
| Cg54–Pg1–Ps17 | Cg54–Pg1–Ps18 | Cg54–Pg1–Ps19 | Cg54–Pg1–Ps20 |
| Cg54–Pg1–Ps21 | Cg54–Pg1–Ps22 | Cg54–Pg1–Ps23 | Cg54–Pg1–Ps24 |
| Cg54–Pg1–Ps25 | Cg54–Pg1–Ps26 | Cg54–Pg1–Ps27 | Cg54–Pg1–Ps28 |
| Cg54–Pg1–Ps29 | Cg54–Pg1–Ps30 | Cg54–Pg1–Ps31 | Cg54–Pg1–Ps32 |
| Cg54–Pg1–Ps33 | Cg54–Pg1–Ps34 | Cg54–Pg1–Ps35 | Cg54–Pg1–Ps36 |
| Cg54–Pg1–Ps37 | Cg54–Pg1–Ps38 | Cg54–Pg1–Ps39 | Cg54–Pg1–Ps40 |
| Cg54–Pg1–Ps41 | Cg54–Pg1–Ps42 | Cg54–Pg1–Ps43 | Cg54–Pg1–Ps44 |
| Cg54–Pg1–Ps45 | Cg54–Pg1–Ps46 | Cg54–Pg1–Ps47 | Cg54–Pg1–Ps48 |
| Cg54–Pg1–Ps49 | Cg54–Pg1–Ps50 | Cg54–Pg1–Ps51 | Cg54–Pg1–Ps52 |
| Cg54–Pg1–Ps53 | Cg54–Pg1–Ps54 | Cg54–Pg1–Ps55 | Cg54–Pg1–Ps56 |
| Cg54–Pg1–Ps57 | Cg54–Pg1–Ps58 | Cg54–Pg1–Ps59 | Cg54–Pg1–Ps60 |
| Cg54–Pg1–Ps61 | Cg54–Pg1–Ps62 | Cg54–Pg1–Ps63 | Cg54–Pg1–Ps64 |
| Cg54–Pg1–Ps65 | Cg54–Pg1–Ps66 | Cg54–Pg1–Ps67 | Cg54–Pg1–Ps68 |
| Cg54–Pg1–Ps69 | Cg54–Pg1–Ps70 | Cg54–Pg1–Ps71 | Cg54–Pg1–Ps72 |
| Cg54–Pg1–Ps73 | Cg54–Pg1–Ps74 | Cg54–Pg1–Ps75 | Cg54–Pg1–Ps76 |
| Cg54–Pg1–Ps77 | Cg54–Pg1–Ps78 | Cg54–Pg1–Ps79 | Cg54–Pg1–Ps80 |
| Cg54–Pg1–Ps81 | Cg54–Pg1–Ps82 | Cg54–Pg1–Ps83 | Cg54–Pg1–Ps84 |

| | | | |
|---|---|---|---|
| Cg54–Pg1–Ps85 | Cg54–Pg1–Ps86 | Cg54–Pg1–Ps87 | Cg54–Pg1–Ps88 |
| Cg54–Pg1–Ps89 | Cg54–Pg1–Ps90 | Cg54–Pg1–Ps91 | Cg54–Pg1–Ps92 |
| Cg54–Pg1–Ps93 | Cg54–Pg1–Ps94 | Cg54–Pg1–Ps95 | Cg54–Pg1–Ps96 |
| Cg54–Pg1–Ps97 | Cg54–Pg1–Ps98 | Cg54–Pg1–Ps99 | Cg54–Pg1–Ps100 |
| Cg54–Pg1–Ps101 | Cg54–Pg1–Ps102 | Cg54–Pg1–Ps103 | Cg54–Pg1–Ps104 |
| Cg54–Pg1–Ps105 | Cg54–Pg1–Ps106 | Cg54–Pg1–Ps107 | Cg54–Pg1–Ps108 |
| Cg54–Pg1–Ps109 | Cg54–Pg1–Ps110 | Cg54–Pg1–Ps111 | Cg54–Pg1–Ps112 |
| Cg54–Pg1–Ps113 | Cg54–Pg1–Ps114 | Cg54–Pg1–Ps115 | Cg54–Pg1–Ps116 |
| Cg54–Pg1–Ps117 | Cg54–Pg1–Ps118 | Cg54–Pg1–Ps119 | Cg54–Pg1–Ps120 |
| Cg54–Pg1–Ps121 | Cg54–Pg1–Ps122 | Cg54–Pg1–Ps123 | Cg54–Pg1–Ps124 |
| Cg54–Pg1–Ps125 | Cg54–Pg1–Ps126 | Cg54–Pg1–Ps127 | Cg54–Pg1–Ps128 |
| Cg54–Pg1–Ps129 | Cg54–Pg1–Ps130 | Cg54–Pg1–Ps131 | Cg54–Pg1–Ps132 |
| Cg54–Pg1–Ps133 | Cg54–Pg1–Ps134 | Cg54–Pg1–Ps135 | Cg54–Pg1–Ps136 |
| Cg54–Pg1–Ps137 | Cg54–Pg1–Ps138 | Cg54–Pg1–Ps139 | Cg54–Pg1–Ps140 |
| Cg54–Pg1–Ps141 | Cg54–Pg1–Ps142 | Cg54–Pg1–Ps143 | Cg54–Pg1–Ps144 |
| Cg54–Pg1–Ps145 | Cg54–Pg1–Ps146 | Cg54–Pg1–Ps147 | Cg54–Pg1–Ps148 |
| Cg54–Pg1–Ps149 | Cg54–Pg1–Ps150 | Cg54–Pg1–Ps151 | Cg54–Pg1–Ps152 |
| Cg54–Pg1–Ps153 | Cg54–Pg1–Ps154 | Cg54–Pg1–Ps155 | Cg54–Pg1–Ps156 |
| Cg54–Pg1–Ps157 | Cg54–Pg1–Ps158 | Cg54–Pg1–Ps159 | Cg54–Pg1–Ps160 |
| Cg54–Pg1–Ps161 | Cg54–Pg1–Ps162 | Cg54–Pg1–Ps163 | Cg54–Pg1–Ps164 |
| Cg54–Pg1–Ps165 | Cg54–Pg1–Ps166 | Cg54–Pg1–Ps167 | Cg54–Pg1–Ps168 |
| Cg54–Pg1–Ps169 | Cg54–Pg1–Ps170 | Cg54–Pg1–Ps171 | Cg54–Pg1–Ps172 |
| Cg54–Pg1–Ps173 | Cg54–Pg1–Ps174 | Cg54–Pg1–Ps175 | Cg54–Pg1–Ps176 |
| Cg54–Pg1–Ps177 | Cg54–Pg1–Ps178 | Cg54–Pg1–Ps179 | Cg54–Pg1–Ps180 |
| Cg54–Pg1–Ps181 | Cg54–Pg1–Ps182 | Cg54–Pg1–Ps183 | Cg54–Pg1–Ps184 |
| Cg54–Pg1–Ps185 | Cg54–Pg1–Ps186 | Cg54–Pg1–Ps187 | Cg54–Pg1–Ps188 |
| Cg54–Pg1–Ps189 | Cg54–Pg1–Ps190 | Cg54–Pg1–Ps191 | Cg54–Pg1–Ps192 |
| Cg54–Pg1–Ps193 | Cg54–Pg1–Ps194 | Cg54–Pg1–Ps195 | Cg54–Pg1–Ps196 |
| Cg54–Pg1–Ps197 | Cg54–Pg1–Ps198 | Cg54–Pg1–Ps199 | Cg54–Pg1–Ps200 |
| Cg54–Pg1–Ps201 | Cg54–Pg1–Ps202 | Cg54–Pg1–Ps203 | Cg54–Pg1–Ps204 |
| Cg54–Pg1–Ps205 | Cg54–Pg1–Ps206 | Cg54–Pg1–Ps207 | Cg54–Pg1–Ps208 |
| Cg54–Pg1–Ps209 | Cg54–Pg1–Ps210 | Cg54–Pg1–Ps211 | Cg54–Pg1–Ps212 |
| Cg54–Pg1–Ps213 | Cg54–Pg1–Ps214 | Cg54–Pg1–Ps215 | Cg54–Pg1–Ps216 |
| Cg54–Pg1–Ps217 | Cg54–Pg1–Ps218 | Cg54–Pg1–Ps219 | Cg54–Pg1–Ps220 |
| Cg54–Pg1–Ps221 | Cg54–Pg1–Ps222 | Cg54–Pg1–Ps223 | Cg54–Pg1–Ps224 |
| Cg54–Pg1–Ps225 | Cg54–Pg1–Ps226 | Cg54–Pg1–Ps227 | Cg54–Pg1–Ps228 |
| Cg54–Pg1–Ps229 | Cg54–Pg1–Ps230 | Cg54–Pg1–Ps231 | Cg54–Pg1–Ps232 |
| Cg54–Pg1–Ps233 | Cg54–Pg1–Ps234 | Cg54–Pg1–Ps235 | Cg54–Pg1–Ps236 |
| Cg54–Pg1–Ps237 | Cg54–Pg1–Ps238 | Cg54–Pg1–Ps239 | Cg54–Pg1–Ps240 |
| Cg54–Pg1–Ps241 | Cg54–Pg1–Ps242 | Cg54–Pg1–Ps243 | |

| | | | |
|---|---|---|---|
| Cg57–Pg1–Ps1 | Cg57–Pg1–Ps2 | Cg57–Pg1–Ps3 | Cg57–Pg1–Ps4 |
| Cg57–Pg1–Ps5 | Cg57–Pg1–Ps6 | Cg57–Pg1–Ps7 | Cg57–Pg1–Ps8 |
| Cg57–Pg1–Ps9 | Cg57–Pg1–Ps10 | Cg57–Pg1–Ps11 | Cg57–Pg1–Ps12 |
| Cg57–Pg1–Ps13 | Cg57–Pg1–Ps14 | Cg57–Pg1–Ps15 | Cg57–Pg1–Ps16 |
| Cg57–Pg1–Ps17 | Cg57–Pg1–Ps18 | Cg57–Pg1–Ps19 | Cg57–Pg1–Ps20 |
| Cg57–Pg1–Ps21 | Cg57–Pg1–Ps22 | Cg57–Pg1–Ps23 | Cg57–Pg1–Ps24 |
| Cg57–Pg1–Ps25 | Cg57–Pg1–Ps26 | Cg57–Pg1–Ps27 | Cg57–Pg1–Ps28 |
| Cg57–Pg1–Ps29 | Cg57–Pg1–Ps30 | Cg57–Pg1–Ps31 | Cg57–Pg1–Ps32 |
| Cg57–Pg1–Ps33 | Cg57–Pg1–Ps34 | Cg57–Pg1–Ps35 | Cg57–Pg1–Ps36 |
| Cg57–Pg1–Ps37 | Cg57–Pg1–Ps38 | Cg57–Pg1–Ps39 | Cg57–Pg1–Ps40 |
| Cg57–Pg1–Ps41 | Cg57–Pg1–Ps42 | Cg57–Pg1–Ps43 | Cg57–Pg1–Ps44 |

| | | | |
|---|---|---|---|
| Cg57–Pg1–Ps45 | Cg57–Pg1–Ps46 | Cg57–Pg1–Ps47 | Cg57–Pg1–Ps48 |
| Cg57–Pg1–Ps49 | Cg57–Pg1–Ps50 | Cg57–Pg1–Ps51 | Cg57–Pg1–Ps52 |
| Cg57–Pg1–Ps53 | Cg57–Pg1–Ps54 | Cg57–Pg1–Ps55 | Cg57–Pg1–Ps56 |
| Cg57–Pg1–Ps57 | Cg57–Pg1–Ps58 | Cg57–Pg1–Ps59 | Cg57–Pg1–Ps60 |
| Cg57–Pg1–Ps61 | Cg57–Pg1–Ps62 | Cg57–Pg1–Ps63 | Cg57–Pg1–Ps64 |
| Cg57–Pg1–Ps65 | Cg57–Pg1–Ps66 | Cg57–Pg1–Ps67 | Cg57–Pg1–Ps68 |
| Cg57–Pg1–Ps69 | Cg57–Pg1–Ps70 | Cg57–Pg1–Ps71 | Cg57–Pg1–Ps72 |
| Cg57–Pg1–Ps73 | Cg57–Pg1–Ps74 | Cg57–Pg1–Ps75 | Cg57–Pg1–Ps76 |
| Cg57–Pg1–Ps77 | Cg57–Pg1–Ps78 | Cg57–Pg1–Ps79 | Cg57–Pg1–Ps80 |
| Cg57–Pg1–Ps81 | Cg57–Pg1–Ps82 | Cg57–Pg1–Ps83 | Cg57–Pg1–Ps84 |
| Cg57–Pg1–Ps85 | Cg57–Pg1–Ps86 | Cg57–Pg1–Ps87 | Cg57–Pg1–Ps88 |
| Cg57–Pg1–Ps89 | Cg57–Pg1–Ps90 | Cg57–Pg1–Ps91 | Cg57–Pg1–Ps92 |
| Cg57–Pg1–Ps93 | Cg57–Pg1–Ps94 | Cg57–Pg1–Ps95 | Cg57–Pg1–Ps96 |
| Cg57–Pg1–Ps97 | Cg57–Pg1–Ps98 | Cg57–Pg1–Ps99 | Cg57–Pg1–Ps100 |
| Cg57–Pg1–Ps101 | Cg57–Pg1–Ps102 | Cg57–Pg1–Ps103 | Cg57–Pg1–Ps104 |
| Cg57–Pg1–Ps105 | Cg57–Pg1–Ps106 | Cg57–Pg1–Ps107 | Cg57–Pg1–Ps108 |
| Cg57–Pg1–Ps109 | Cg57–Pg1–Ps110 | Cg57–Pg1–Ps111 | Cg57–Pg1–Ps112 |
| Cg57–Pg1–Ps113 | Cg57–Pg1–Ps114 | Cg57–Pg1–Ps115 | Cg57–Pg1–Ps116 |
| Cg57–Pg1–Ps117 | Cg57–Pg1–Ps118 | Cg57–Pg1–Ps119 | Cg57–Pg1–Ps120 |
| Cg57–Pg1–Ps121 | Cg57–Pg1–Ps122 | Cg57–Pg1–Ps123 | Cg57–Pg1–Ps124 |
| Cg57–Pg1–Ps125 | Cg57–Pg1–Ps126 | Cg57–Pg1–Ps127 | Cg57–Pg1–Ps128 |
| Cg57–Pg1–Ps129 | Cg57–Pg1–Ps130 | Cg57–Pg1–Ps131 | Cg57–Pg1–Ps132 |
| Cg57–Pg1–Ps133 | Cg57–Pg1–Ps134 | Cg57–Pg1–Ps135 | Cg57–Pg1–Ps136 |
| Cg57–Pg1–Ps137 | Cg57–Pg1–Ps138 | Cg57–Pg1–Ps139 | Cg57–Pg1–Ps140 |
| Cg57–Pg1–Ps141 | Cg57–Pg1–Ps142 | Cg57–Pg1–Ps143 | Cg57–Pg1–Ps144 |
| Cg57–Pg1–Ps145 | Cg57–Pg1–Ps146 | Cg57–Pg1–Ps147 | Cg57–Pg1–Ps148 |
| Cg57–Pg1–Ps149 | Cg57–Pg1–Ps150 | Cg57–Pg1–Ps151 | Cg57–Pg1–Ps152 |
| Cg57–Pg1–Ps153 | Cg57–Pg1–Ps154 | Cg57–Pg1–Ps155 | Cg57–Pg1–Ps156 |
| Cg57–Pg1–Ps157 | Cg57–Pg1–Ps158 | Cg57–Pg1–Ps159 | Cg57–Pg1–Ps160 |
| Cg57–Pg1–Ps161 | Cg57–Pg1–Ps162 | Cg57–Pg1–Ps163 | Cg57–Pg1–Ps164 |
| Cg57–Pg1–Ps165 | Cg57–Pg1–Ps166 | Cg57–Pg1–Ps167 | Cg57–Pg1–Ps168 |
| Cg57–Pg1–Ps169 | Cg57–Pg1–Ps170 | Cg57–Pg1–Ps171 | Cg57–Pg1–Ps172 |
| Cg57–Pg1–Ps173 | Cg57–Pg1–Ps174 | Cg57–Pg1–Ps175 | Cg57–Pg1–Ps176 |
| Cg57–Pg1–Ps177 | Cg57–Pg1–Ps178 | Cg57–Pg1–Ps179 | Cg57–Pg1–Ps180 |
| Cg57–Pg1–Ps181 | Cg57–Pg1–Ps182 | Cg57–Pg1–Ps183 | Cg57–Pg1–Ps184 |
| Cg57–Pg1–Ps185 | Cg57–Pg1–Ps186 | Cg57–Pg1–Ps187 | Cg57–Pg1–Ps188 |
| Cg57–Pg1–Ps189 | Cg57–Pg1–Ps190 | Cg57–Pg1–Ps191 | Cg57–Pg1–Ps192 |
| Cg57–Pg1–Ps193 | Cg57–Pg1–Ps194 | Cg57–Pg1–Ps195 | Cg57–Pg1–Ps196 |
| Cg57–Pg1–Ps197 | Cg57–Pg1–Ps198 | Cg57–Pg1–Ps199 | Cg57–Pg1–Ps200 |
| Cg57–Pg1–Ps201 | Cg57–Pg1–Ps202 | Cg57–Pg1–Ps203 | Cg57–Pg1–Ps204 |
| Cg57–Pg1–Ps205 | Cg57–Pg1–Ps206 | Cg57–Pg1–Ps207 | Cg57–Pg1–Ps208 |
| Cg57–Pg1–Ps209 | Cg57–Pg1–Ps210 | Cg57–Pg1–Ps211 | Cg57–Pg1–Ps212 |
| Cg57–Pg1–Ps213 | Cg57–Pg1–Ps214 | Cg57–Pg1–Ps215 | Cg57–Pg1–Ps216 |
| Cg57–Pg1–Ps217 | Cg57–Pg1–Ps218 | Cg57–Pg1–Ps219 | Cg57–Pg1–Ps220 |
| Cg57–Pg1–Ps221 | Cg57–Pg1–Ps222 | Cg57–Pg1–Ps223 | Cg57–Pg1–Ps224 |
| Cg57–Pg1–Ps225 | Cg57–Pg1–Ps226 | Cg57–Pg1–Ps227 | Cg57–Pg1–Ps228 |
| Cg57–Pg1–Ps229 | Cg57–Pg1–Ps230 | Cg57–Pg1–Ps231 | Cg57–Pg1–Ps232 |
| Cg57–Pg1–Ps233 | Cg57–Pg1–Ps234 | Cg57–Pg1–Ps235 | Cg57–Pg1–Ps236 |
| Cg57–Pg1–Ps237 | Cg57–Pg1–Ps238 | Cg57–Pg1–Ps239 | Cg57–Pg1–Ps240 |
| Cg57–Pg1–Ps241 | Cg57–Pg1–Ps242 | Cg57–Pg1–Ps243 | |

| | | | |
|---|---|---|---|
| Cg60–Pg1–Ps1 | Cg60–Pg1–Ps2 | Cg60–Pg1–Ps3 | Cg60–Pg1–Ps4 |

| | | | |
|---|---|---|---|
| Cg60-Pg1-Ps5 | Cg60-Pg1-Ps6 | Cg60-Pg1-Ps7 | Cg60-Pg1-Ps8 |
| Cg60-Pg1-Ps9 | Cg60-Pg1-Ps10 | Cg60-Pg1-Ps11 | Cg60-Pg1-Ps12 |
| Cg60-Pg1-Ps13 | Cg60-Pg1-Ps14 | Cg60-Pg1-Ps15 | Cg60-Pg1-Ps16 |
| Cg60-Pg1-Ps17 | Cg60-Pg1-Ps18 | Cg60-Pg1-Ps19 | Cg60-Pg1-Ps20 |
| Cg60-Pg1-Ps21 | Cg60-Pg1-Ps22 | Cg60-Pg1-Ps23 | Cg60-Pg1-Ps24 |
| Cg60-Pg1-Ps25 | Cg60-Pg1-Ps26 | Cg60-Pg1-Ps27 | Cg60-Pg1-Ps28 |
| Cg60-Pg1-Ps29 | Cg60-Pg1-Ps30 | Cg60-Pg1-Ps31 | Cg60-Pg1-Ps32 |
| Cg60-Pg1-Ps33 | Cg60-Pg1-Ps34 | Cg60-Pg1-Ps35 | Cg60-Pg1-Ps36 |
| Cg60-Pg1-Ps37 | Cg60-Pg1-Ps38 | Cg60-Pg1-Ps39 | Cg60-Pg1-Ps40 |
| Cg60-Pg1-Ps41 | Cg60-Pg1-Ps42 | Cg60-Pg1-Ps43 | Cg60-Pg1-Ps44 |
| Cg60-Pg1-Ps45 | Cg60-Pg1-Ps46 | Cg60-Pg1-Ps47 | Cg60-Pg1-Ps48 |
| Cg60-Pg1-Ps49 | Cg60-Pg1-Ps50 | Cg60-Pg1-Ps51 | Cg60-Pg1-Ps52 |
| Cg60-Pg1-Ps53 | Cg60-Pg1-Ps54 | Cg60-Pg1-Ps55 | Cg60-Pg1-Ps56 |
| Cg60-Pg1-Ps57 | Cg60-Pg1-Ps58 | Cg60-Pg1-Ps59 | Cg60-Pg1-Ps60 |
| Cg60-Pg1-Ps61 | Cg60-Pg1-Ps62 | Cg60-Pg1-Ps63 | Cg60-Pg1-Ps64 |
| Cg60-Pg1-Ps65 | Cg60-Pg1-Ps66 | Cg60-Pg1-Ps67 | Cg60-Pg1-Ps68 |
| Cg60-Pg1-Ps69 | Cg60-Pg1-Ps70 | Cg60-Pg1-Ps71 | Cg60-Pg1-Ps72 |
| Cg60-Pg1-Ps73 | Cg60-Pg1-Ps74 | Cg60-Pg1-Ps75 | Cg60-Pg1-Ps76 |
| Cg60-Pg1-Ps77 | Cg60-Pg1-Ps78 | Cg60-Pg1-Ps79 | Cg60-Pg1-Ps80 |
| Cg60-Pg1-Ps81 | Cg60-Pg1-Ps82 | Cg60-Pg1-Ps83 | Cg60-Pg1-Ps84 |
| Cg60-Pg1-Ps85 | Cg60-Pg1-Ps86 | Cg60-Pg1-Ps87 | Cg60-Pg1-Ps88 |
| Cg60-Pg1-Ps89 | Cg60-Pg1-Ps90 | Cg60-Pg1-Ps91 | Cg60-Pg1-Ps92 |
| Cg60-Pg1-Ps93 | Cg60-Pg1-Ps94 | Cg60-Pg1-Ps95 | Cg60-Pg1-Ps96 |
| Cg60-Pg1-Ps97 | Cg60-Pg1-Ps98 | Cg60-Pg1-Ps99 | Cg60-Pg1-Ps100 |
| Cg60-Pg1-Ps101 | Cg60-Pg1-Ps102 | Cg60-Pg1-Ps103 | Cg60-Pg1-Ps104 |
| Cg60-Pg1-Ps105 | Cg60-Pg1-Ps106 | Cg60-Pg1-Ps107 | Cg60-Pg1-Ps108 |
| Cg60-Pg1-Ps109 | Cg60-Pg1-Ps110 | Cg60-Pg1-Ps111 | Cg60-Pg1-Ps112 |
| Cg60-Pg1-Ps113 | Cg60-Pg1-Ps114 | Cg60-Pg1-Ps115 | Cg60-Pg1-Ps116 |
| Cg60-Pg1-Ps117 | Cg60-Pg1-Ps118 | Cg60-Pg1-Ps119 | Cg60-Pg1-Ps120 |
| Cg60-Pg1-Ps121 | Cg60-Pg1-Ps122 | Cg60-Pg1-Ps123 | Cg60-Pg1-Ps124 |
| Cg60-Pg1-Ps125 | Cg60-Pg1-Ps126 | Cg60-Pg1-Ps127 | Cg60-Pg1-Ps128 |
| Cg60-Pg1-Ps129 | Cg60-Pg1-Ps130 | Cg60-Pg1-Ps131 | Cg60-Pg1-Ps132 |
| Cg60-Pg1-Ps133 | Cg60-Pg1-Ps134 | Cg60-Pg1-Ps135 | Cg60-Pg1-Ps136 |
| Cg60-Pg1-Ps137 | Cg60-Pg1-Ps138 | Cg60-Pg1-Ps139 | Cg60-Pg1-Ps140 |
| Cg60-Pg1-Ps141 | Cg60-Pg1-Ps142 | Cg60-Pg1-Ps143 | Cg60-Pg1-Ps144 |
| Cg60-Pg1-Ps145 | Cg60-Pg1-Ps146 | Cg60-Pg1-Ps147 | Cg60-Pg1-Ps148 |
| Cg60-Pg1-Ps149 | Cg60-Pg1-Ps150 | Cg60-Pg1-Ps151 | Cg60-Pg1-Ps152 |
| Cg60-Pg1-Ps153 | Cg60-Pg1-Ps154 | Cg60-Pg1-Ps155 | Cg60-Pg1-Ps156 |
| Cg60-Pg1-Ps157 | Cg60-Pg1-Ps158 | Cg60-Pg1-Ps159 | Cg60-Pg1-Ps160 |
| Cg60-Pg1-Ps161 | Cg60-Pg1-Ps162 | Cg60-Pg1-Ps163 | Cg60-Pg1-Ps164 |
| Cg60-Pg1-Ps165 | Cg60-Pg1-Ps166 | Cg60-Pg1-Ps167 | Cg60-Pg1-Ps168 |
| Cg60-Pg1-Ps169 | Cg60-Pg1-Ps170 | Cg60-Pg1-Ps171 | Cg60-Pg1-Ps172 |
| Cg60-Pg1-Ps173 | Cg60-Pg1-Ps174 | Cg60-Pg1-Ps175 | Cg60-Pg1-Ps176 |
| Cg60-Pg1-Ps177 | Cg60-Pg1-Ps178 | Cg60-Pg1-Ps179 | Cg60-Pg1-Ps180 |
| Cg60-Pg1-Ps181 | Cg60-Pg1-Ps182 | Cg60-Pg1-Ps183 | Cg60-Pg1-Ps184 |
| Cg60-Pg1-Ps185 | Cg60-Pg1-Ps186 | Cg60-Pg1-Ps187 | Cg60-Pg1-Ps188 |
| Cg60-Pg1-Ps189 | Cg60-Pg1-Ps190 | Cg60-Pg1-Ps191 | Cg60-Pg1-Ps192 |
| Cg60-Pg1-Ps193 | Cg60-Pg1-Ps194 | Cg60-Pg1-Ps195 | Cg60-Pg1-Ps196 |
| Cg60-Pg1-Ps197 | Cg60-Pg1-Ps198 | Cg60-Pg1-Ps199 | Cg60-Pg1-Ps200 |
| Cg60-Pg1-Ps201 | Cg60-Pg1-Ps202 | Cg60-Pg1-Ps203 | Cg60-Pg1-Ps204 |
| Cg60-Pg1-Ps205 | Cg60-Pg1-Ps206 | Cg60-Pg1-Ps207 | Cg60-Pg1-Ps208 |
| Cg60-Pg1-Ps209 | Cg60-Pg1-Ps210 | Cg60-Pg1-Ps211 | Cg60-Pg1-Ps212 |

| | | | |
|---|---|---|---|
| Cg60–Pg1–Ps213 | Cg60–Pg1–Ps214 | Cg60–Pg1–Ps215 | Cg60–Pg1–Ps216 |
| Cg60–Pg1–Ps217 | Cg60–Pg1–Ps218 | Cg60–Pg1–Ps219 | Cg60–Pg1–Ps220 |
| Cg60–Pg1–Ps221 | Cg60–Pg1–Ps222 | Cg60–Pg1–Ps223 | Cg60–Pg1–Ps224 |
| Cg60–Pg1–Ps225 | Cg60–Pg1–Ps226 | Cg60–Pg1–Ps227 | Cg60–Pg1–Ps228 |
| Cg60–Pg1–Ps229 | Cg60–Pg1–Ps230 | Cg60–Pg1–Ps231 | Cg60–Pg1–Ps232 |
| Cg60–Pg1–Ps233 | Cg60–Pg1–Ps234 | Cg60–Pg1–Ps235 | Cg60–Pg1–Ps236 |
| Cg60–Pg1–Ps237 | Cg60–Pg1–Ps238 | Cg60–Pg1–Ps239 | Cg60–Pg1–Ps240 |
| Cg60–Pg1–Ps241 | Cg60–Pg1–Ps242 | Cg60–Pg1–Ps243 | |

| | | | |
|---|---|---|---|
| Cg61–Pg1–Ps1 | Cg61–Pg1–Ps2 | Cg61–Pg1–Ps3 | Cg61–Pg1–Ps4 |
| Cg61–Pg1–Ps5 | Cg61–Pg1–Ps6 | Cg61–Pg1–Ps7 | Cg61–Pg1–Ps8 |
| Cg61–Pg1–Ps9 | Cg61–Pg1–Ps10 | Cg61–Pg1–Ps11 | Cg61–Pg1–Ps12 |
| Cg61–Pg1–Ps13 | Cg61–Pg1–Ps14 | Cg61–Pg1–Ps15 | Cg61–Pg1–Ps16 |
| Cg61–Pg1–Ps17 | Cg61–Pg1–Ps18 | Cg61–Pg1–Ps19 | Cg61–Pg1–Ps20 |
| Cg61–Pg1–Ps21 | Cg61–Pg1–Ps22 | Cg61–Pg1–Ps23 | Cg61–Pg1–Ps24 |
| Cg61–Pg1–Ps25 | Cg61–Pg1–Ps26 | Cg61–Pg1–Ps27 | Cg61–Pg1–Ps28 |
| Cg61–Pg1–Ps29 | Cg61–Pg1–Ps30 | Cg61–Pg1–Ps31 | Cg61–Pg1–Ps32 |
| Cg61–Pg1–Ps33 | Cg61–Pg1–Ps34 | Cg61–Pg1–Ps35 | Cg61–Pg1–Ps36 |
| Cg61–Pg1–Ps37 | Cg61–Pg1–Ps38 | Cg61–Pg1–Ps39 | Cg61–Pg1–Ps40 |
| Cg61–Pg1–Ps41 | Cg61–Pg1–Ps42 | Cg61–Pg1–Ps43 | Cg61–Pg1–Ps44 |
| Cg61–Pg1–Ps45 | Cg61–Pg1–Ps46 | Cg61–Pg1–Ps47 | Cg61–Pg1–Ps48 |
| Cg61–Pg1–Ps49 | Cg61–Pg1–Ps50 | Cg61–Pg1–Ps51 | Cg61–Pg1–Ps52 |
| Cg61–Pg1–Ps53 | Cg61–Pg1–Ps54 | Cg61–Pg1–Ps55 | Cg61–Pg1–Ps56 |
| Cg61–Pg1–Ps57 | Cg61–Pg1–Ps58 | Cg61–Pg1–Ps59 | Cg61–Pg1–Ps60 |
| Cg61–Pg1–Ps61 | Cg61–Pg1–Ps62 | Cg61–Pg1–Ps63 | Cg61–Pg1–Ps64 |
| Cg61–Pg1–Ps65 | Cg61–Pg1–Ps66 | Cg61–Pg1–Ps67 | Cg61–Pg1–Ps68 |
| Cg61–Pg1–Ps69 | Cg61–Pg1–Ps70 | Cg61–Pg1–Ps71 | Cg61–Pg1–Ps72 |
| Cg61–Pg1–Ps73 | Cg61–Pg1–Ps74 | Cg61–Pg1–Ps75 | Cg61–Pg1–Ps76 |
| Cg61–Pg1–Ps77 | Cg61–Pg1–Ps78 | Cg61–Pg1–Ps79 | Cg61–Pg1–Ps80 |
| Cg61–Pg1–Ps81 | Cg61–Pg1–Ps82 | Cg61–Pg1–Ps83 | Cg61–Pg1–Ps84 |
| Cg61–Pg1–Ps85 | Cg61–Pg1–Ps86 | Cg61–Pg1–Ps87 | Cg61–Pg1–Ps88 |
| Cg61–Pg1–Ps89 | Cg61–Pg1–Ps90 | Cg61–Pg1–Ps91 | Cg61–Pg1–Ps92 |
| Cg61–Pg1–Ps93 | Cg61–Pg1–Ps94 | Cg61–Pg1–Ps95 | Cg61–Pg1–Ps96 |
| Cg61–Pg1–Ps97 | Cg61–Pg1–Ps98 | Cg61–Pg1–Ps99 | Cg61–Pg1–Ps100 |
| Cg61–Pg1–Ps101 | Cg61–Pg1–Ps102 | Cg61–Pg1–Ps103 | Cg61–Pg1–Ps104 |
| Cg61–Pg1–Ps105 | Cg61–Pg1–Ps106 | Cg61–Pg1–Ps107 | Cg61–Pg1–Ps108 |
| Cg61–Pg1–Ps109 | Cg61–Pg1–Ps110 | Cg61–Pg1–Ps111 | Cg61–Pg1–Ps112 |
| Cg61–Pg1–Ps113 | Cg61–Pg1–Ps114 | Cg61–Pg1–Ps115 | Cg61–Pg1–Ps116 |
| Cg61–Pg1–Ps117 | Cg61–Pg1–Ps118 | Cg61–Pg1–Ps119 | Cg61–Pg1–Ps120 |
| Cg61–Pg1–Ps121 | Cg61–Pg1–Ps122 | Cg61–Pg1–Ps123 | Cg61–Pg1–Ps124 |
| Cg61–Pg1–Ps125 | Cg61–Pg1–Ps126 | Cg61–Pg1–Ps127 | Cg61–Pg1–Ps128 |
| Cg61–Pg1–Ps129 | Cg61–Pg1–Ps130 | Cg61–Pg1–Ps131 | Cg61–Pg1–Ps132 |
| Cg61–Pg1–Ps133 | Cg61–Pg1–Ps134 | Cg61–Pg1–Ps135 | Cg61–Pg1–Ps136 |
| Cg61–Pg1–Ps137 | Cg61–Pg1–Ps138 | Cg61–Pg1–Ps139 | Cg61–Pg1–Ps140 |
| Cg61–Pg1–Ps141 | Cg61–Pg1–Ps142 | Cg61–Pg1–Ps143 | Cg61–Pg1–Ps144 |
| Cg61–Pg1–Ps145 | Cg61–Pg1–Ps146 | Cg61–Pg1–Ps147 | Cg61–Pg1–Ps148 |
| Cg61–Pg1–Ps149 | Cg61–Pg1–Ps150 | Cg61–Pg1–Ps151 | Cg61–Pg1–Ps152 |
| Cg61–Pg1–Ps153 | Cg61–Pg1–Ps154 | Cg61–Pg1–Ps155 | Cg61–Pg1–Ps156 |
| Cg61–Pg1–Ps157 | Cg61–Pg1–Ps158 | Cg61–Pg1–Ps159 | Cg61–Pg1–Ps160 |
| Cg61–Pg1–Ps161 | Cg61–Pg1–Ps162 | Cg61–Pg1–Ps163 | Cg61–Pg1–Ps164 |
| Cg61–Pg1–Ps165 | Cg61–Pg1–Ps166 | Cg61–Pg1–Ps167 | Cg61–Pg1–Ps168 |
| Cg61–Pg1–Ps169 | Cg61–Pg1–Ps170 | Cg61–Pg1–Ps171 | Cg61–Pg1–Ps172 |

| | | | |
|---|---|---|---|
| Cg61–Pg1–Ps173 | Cg61–Pg1–Ps174 | Cg61–Pg1–Ps175 | Cg61–Pg1–Ps176 |
| Cg61–Pg1–Ps177 | Cg61–Pg1–Ps178 | Cg61–Pg1–Ps179 | Cg61–Pg1–Ps180 |
| Cg61–Pg1–Ps181 | Cg61–Pg1–Ps182 | Cg61–Pg1–Ps183 | Cg61–Pg1–Ps184 |
| Cg61–Pg1–Ps185 | Cg61–Pg1–Ps186 | Cg61–Pg1–Ps187 | Cg61–Pg1–Ps188 |
| Cg61–Pg1–Ps189 | Cg61–Pg1–Ps190 | Cg61–Pg1–Ps191 | Cg61–Pg1–Ps192 |
| Cg61–Pg1–Ps193 | Cg61–Pg1–Ps194 | Cg61–Pg1–Ps195 | Cg61–Pg1–Ps196 |
| Cg61–Pg1–Ps197 | Cg61–Pg1–Ps198 | Cg61–Pg1–Ps199 | Cg61–Pg1–Ps200 |
| Cg61–Pg1–Ps201 | Cg61–Pg1–Ps202 | Cg61–Pg1–Ps203 | Cg61–Pg1–Ps204 |
| Cg61–Pg1–Ps205 | Cg61–Pg1–Ps206 | Cg61–Pg1–Ps207 | Cg61–Pg1–Ps208 |
| Cg61–Pg1–Ps209 | Cg61–Pg1–Ps210 | Cg61–Pg1–Ps211 | Cg61–Pg1–Ps212 |
| Cg61–Pg1–Ps213 | Cg61–Pg1–Ps214 | Cg61–Pg1–Ps215 | Cg61–Pg1–Ps216 |
| Cg61–Pg1–Ps217 | Cg61–Pg1–Ps218 | Cg61–Pg1–Ps219 | Cg61–Pg1–Ps220 |
| Cg61–Pg1–Ps221 | Cg61–Pg1–Ps222 | Cg61–Pg1–Ps223 | Cg61–Pg1–Ps224 |
| Cg61–Pg1–Ps225 | Cg61–Pg1–Ps226 | Cg61–Pg1–Ps227 | Cg61–Pg1–Ps228 |
| Cg61–Pg1–Ps229 | Cg61–Pg1–Ps230 | Cg61–Pg1–Ps231 | Cg61–Pg1–Ps232 |
| Cg61–Pg1–Ps233 | Cg61–Pg1–Ps234 | Cg61–Pg1–Ps235 | Cg61–Pg1–Ps236 |
| Cg61–Pg1–Ps237 | Cg61–Pg1–Ps238 | Cg61–Pg1–Ps239 | Cg61–Pg1–Ps240 |
| Cg61–Pg1–Ps241 | Cg61–Pg1–Ps242 | Cg61–Pg1–Ps243 | |

| | | | |
|---|---|---|---|
| Cg62–Pg1–Ps1 | Cg62–Pg1–Ps2 | Cg62–Pg1–Ps3 | Cg62–Pg1–Ps4 |
| Cg62–Pg1–Ps5 | Cg62–Pg1–Ps6 | Cg62–Pg1–Ps7 | Cg62–Pg1–Ps8 |
| Cg62–Pg1–Ps9 | Cg62–Pg1–Ps10 | Cg62–Pg1–Ps11 | Cg62–Pg1–Ps12 |
| Cg62–Pg1–Ps13 | Cg62–Pg1–Ps14 | Cg62–Pg1–Ps15 | Cg62–Pg1–Ps16 |
| Cg62–Pg1–Ps17 | Cg62–Pg1–Ps18 | Cg62–Pg1–Ps19 | Cg62–Pg1–Ps20 |
| Cg62–Pg1–Ps21 | Cg62–Pg1–Ps22 | Cg62–Pg1–Ps23 | Cg62–Pg1–Ps24 |
| Cg62–Pg1–Ps25 | Cg62–Pg1–Ps26 | Cg62–Pg1–Ps27 | Cg62–Pg1–Ps28 |
| Cg62–Pg1–Ps29 | Cg62–Pg1–Ps30 | Cg62–Pg1–Ps31 | Cg62–Pg1–Ps32 |
| Cg62–Pg1–Ps33 | Cg62–Pg1–Ps34 | Cg62–Pg1–Ps35 | Cg62–Pg1–Ps36 |
| Cg62–Pg1–Ps37 | Cg62–Pg1–Ps38 | Cg62–Pg1–Ps39 | Cg62–Pg1–Ps40 |
| Cg62–Pg1–Ps41 | Cg62–Pg1–Ps42 | Cg62–Pg1–Ps43 | Cg62–Pg1–Ps44 |
| Cg62–Pg1–Ps45 | Cg62–Pg1–Ps46 | Cg62–Pg1–Ps47 | Cg62–Pg1–Ps48 |
| Cg62–Pg1–Ps49 | Cg62–Pg1–Ps50 | Cg62–Pg1–Ps51 | Cg62–Pg1–Ps52 |
| Cg62–Pg1–Ps53 | Cg62–Pg1–Ps54 | Cg62–Pg1–Ps55 | Cg62–Pg1–Ps56 |
| Cg62–Pg1–Ps57 | Cg62–Pg1–Ps58 | Cg62–Pg1–Ps59 | Cg62–Pg1–Ps60 |
| Cg62–Pg1–Ps61 | Cg62–Pg1–Ps62 | Cg62–Pg1–Ps63 | Cg62–Pg1–Ps64 |
| Cg62–Pg1–Ps65 | Cg62–Pg1–Ps66 | Cg62–Pg1–Ps67 | Cg62–Pg1–Ps68 |
| Cg62–Pg1–Ps69 | Cg62–Pg1–Ps70 | Cg62–Pg1–Ps71 | Cg62–Pg1–Ps72 |
| Cg62–Pg1–Ps73 | Cg62–Pg1–Ps74 | Cg62–Pg1–Ps75 | Cg62–Pg1–Ps76 |
| Cg62–Pg1–Ps77 | Cg62–Pg1–Ps78 | Cg62–Pg1–Ps79 | Cg62–Pg1–Ps80 |
| Cg62–Pg1–Ps81 | Cg62–Pg1–Ps82 | Cg62–Pg1–Ps83 | Cg62–Pg1–Ps84 |
| Cg62–Pg1–Ps85 | Cg62–Pg1–Ps86 | Cg62–Pg1–Ps87 | Cg62–Pg1–Ps88 |
| Cg62–Pg1–Ps89 | Cg62–Pg1–Ps90 | Cg62–Pg1–Ps91 | Cg62–Pg1–Ps92 |
| Cg62–Pg1–Ps93 | Cg62–Pg1–Ps94 | Cg62–Pg1–Ps95 | Cg62–Pg1–Ps96 |
| Cg62–Pg1–Ps97 | Cg62–Pg1–Ps98 | Cg62–Pg1–Ps99 | Cg62–Pg1–Ps100 |
| Cg62–Pg1–Ps101 | Cg62–Pg1–Ps102 | Cg62–Pg1–Ps103 | Cg62–Pg1–Ps104 |
| Cg62–Pg1–Ps105 | Cg62–Pg1–Ps106 | Cg62–Pg1–Ps107 | Cg62–Pg1–Ps108 |
| Cg62–Pg1–Ps109 | Cg62–Pg1–Ps110 | Cg62–Pg1–Ps111 | Cg62–Pg1–Ps112 |
| Cg62–Pg1–Ps113 | Cg62–Pg1–Ps114 | Cg62–Pg1–Ps115 | Cg62–Pg1–Ps116 |
| Cg62–Pg1–Ps117 | Cg62–Pg1–Ps118 | Cg62–Pg1–Ps119 | Cg62–Pg1–Ps120 |
| Cg62–Pg1–Ps121 | Cg62–Pg1–Ps122 | Cg62–Pg1–Ps123 | Cg62–Pg1–Ps124 |
| Cg62–Pg1–Ps125 | Cg62–Pg1–Ps126 | Cg62–Pg1–Ps127 | Cg62–Pg1–Ps128 |
| Cg62–Pg1–Ps129 | Cg62–Pg1–Ps130 | Cg62–Pg1–Ps131 | Cg62–Pg1–Ps132 |

| | | | |
|---|---|---|---|
| Cg62–Pg1–Ps133 | Cg62–Pg1–Ps134 | Cg62–Pg1–Ps135 | Cg62–Pg1–Ps136 |
| Cg62–Pg1–Ps137 | Cg62–Pg1–Ps138 | Cg62–Pg1–Ps139 | Cg62–Pg1–Ps140 |
| Cg62–Pg1–Ps141 | Cg62–Pg1–Ps142 | Cg62–Pg1–Ps143 | Cg62–Pg1–Ps144 |
| Cg62–Pg1–Ps145 | Cg62–Pg1–Ps146 | Cg62–Pg1–Ps147 | Cg62–Pg1–Ps148 |
| Cg62–Pg1–Ps149 | Cg62–Pg1–Ps150 | Cg62–Pg1–Ps151 | Cg62–Pg1–Ps152 |
| Cg62–Pg1–Ps153 | Cg62–Pg1–Ps154 | Cg62–Pg1–Ps155 | Cg62–Pg1–Ps156 |
| Cg62–Pg1–Ps157 | Cg62–Pg1–Ps158 | Cg62–Pg1–Ps159 | Cg62–Pg1–Ps160 |
| Cg62–Pg1–Ps161 | Cg62–Pg1–Ps162 | Cg62–Pg1–Ps163 | Cg62–Pg1–Ps164 |
| Cg62–Pg1–Ps165 | Cg62–Pg1–Ps166 | Cg62–Pg1–Ps167 | Cg62–Pg1–Ps168 |
| Cg62–Pg1–Ps169 | Cg62–Pg1–Ps170 | Cg62–Pg1–Ps171 | Cg62–Pg1–Ps172 |
| Cg62–Pg1–Ps173 | Cg62–Pg1–Ps174 | Cg62–Pg1–Ps175 | Cg62–Pg1–Ps176 |
| Cg62–Pg1–Ps177 | Cg62–Pg1–Ps178 | Cg62–Pg1–Ps179 | Cg62–Pg1–Ps180 |
| Cg62–Pg1–Ps181 | Cg62–Pg1–Ps182 | Cg62–Pg1–Ps183 | Cg62–Pg1–Ps184 |
| Cg62–Pg1–Ps185 | Cg62–Pg1–Ps186 | Cg62–Pg1–Ps187 | Cg62–Pg1–Ps188 |
| Cg62–Pg1–Ps189 | Cg62–Pg1–Ps190 | Cg62–Pg1–Ps191 | Cg62–Pg1–Ps192 |
| Cg62–Pg1–Ps193 | Cg62–Pg1–Ps194 | Cg62–Pg1–Ps195 | Cg62–Pg1–Ps196 |
| Cg62–Pg1–Ps197 | Cg62–Pg1–Ps198 | Cg62–Pg1–Ps199 | Cg62–Pg1–Ps200 |
| Cg62–Pg1–Ps201 | Cg62–Pg1–Ps202 | Cg62–Pg1–Ps203 | Cg62–Pg1–Ps204 |
| Cg62–Pg1–Ps205 | Cg62–Pg1–Ps206 | Cg62–Pg1–Ps207 | Cg62–Pg1–Ps208 |
| Cg62–Pg1–Ps209 | Cg62–Pg1–Ps210 | Cg62–Pg1–Ps211 | Cg62–Pg1–Ps212 |
| Cg62–Pg1–Ps213 | Cg62–Pg1–Ps214 | Cg62–Pg1–Ps215 | Cg62–Pg1–Ps216 |
| Cg62–Pg1–Ps217 | Cg62–Pg1–Ps218 | Cg62–Pg1–Ps219 | Cg62–Pg1–Ps220 |
| Cg62–Pg1–Ps221 | Cg62–Pg1–Ps222 | Cg62–Pg1–Ps223 | Cg62–Pg1–Ps224 |
| Cg62–Pg1–Ps225 | Cg62–Pg1–Ps226 | Cg62–Pg1–Ps227 | Cg62–Pg1–Ps228 |
| Cg62–Pg1–Ps229 | Cg62–Pg1–Ps230 | Cg62–Pg1–Ps231 | Cg62–Pg1–Ps232 |
| Cg62–Pg1–Ps233 | Cg62–Pg1–Ps234 | Cg62–Pg1–Ps235 | Cg62–Pg1–Ps236 |
| Cg62–Pg1–Ps237 | Cg62–Pg1–Ps238 | Cg62–Pg1–Ps239 | Cg62–Pg1–Ps240 |
| Cg62–Pg1–Ps241 | Cg62–Pg1–Ps242 | Cg62–Pg1–Ps243 | |

| | | | |
|---|---|---|---|
| Cg63–Pg1–Ps1 | Cg63–Pg1–Ps2 | Cg63–Pg1–Ps3 | Cg63–Pg1–Ps4 |
| Cg63–Pg1–Ps5 | Cg63–Pg1–Ps6 | Cg63–Pg1–Ps7 | Cg63–Pg1–Ps8 |
| Cg63–Pg1–Ps9 | Cg63–Pg1–Ps10 | Cg63–Pg1–Ps11 | Cg63–Pg1–Ps12 |
| Cg63–Pg1–Ps13 | Cg63–Pg1–Ps14 | Cg63–Pg1–Ps15 | Cg63–Pg1–Ps16 |
| Cg63–Pg1–Ps17 | Cg63–Pg1–Ps18 | Cg63–Pg1–Ps19 | Cg63–Pg1–Ps20 |
| Cg63–Pg1–Ps21 | Cg63–Pg1–Ps22 | Cg63–Pg1–Ps23 | Cg63–Pg1–Ps24 |
| Cg63–Pg1–Ps25 | Cg63–Pg1–Ps26 | Cg63–Pg1–Ps27 | Cg63–Pg1–Ps28 |
| Cg63–Pg1–Ps29 | Cg63–Pg1–Ps30 | Cg63–Pg1–Ps31 | Cg63–Pg1–Ps32 |
| Cg63–Pg1–Ps33 | Cg63–Pg1–Ps34 | Cg63–Pg1–Ps35 | Cg63–Pg1–Ps36 |
| Cg63–Pg1–Ps37 | Cg63–Pg1–Ps38 | Cg63–Pg1–Ps39 | Cg63–Pg1–Ps40 |
| Cg63–Pg1–Ps41 | Cg63–Pg1–Ps42 | Cg63–Pg1–Ps43 | Cg63–Pg1–Ps44 |
| Cg63–Pg1–Ps45 | Cg63–Pg1–Ps46 | Cg63–Pg1–Ps47 | Cg63–Pg1–Ps48 |
| Cg63–Pg1–Ps49 | Cg63–Pg1–Ps50 | Cg63–Pg1–Ps51 | Cg63–Pg1–Ps52 |
| Cg63–Pg1–Ps53 | Cg63–Pg1–Ps54 | Cg63–Pg1–Ps55 | Cg63–Pg1–Ps56 |
| Cg63–Pg1–Ps57 | Cg63–Pg1–Ps58 | Cg63–Pg1–Ps59 | Cg63–Pg1–Ps60 |
| Cg63–Pg1–Ps61 | Cg63–Pg1–Ps62 | Cg63–Pg1–Ps63 | Cg63–Pg1–Ps64 |
| Cg63–Pg1–Ps65 | Cg63–Pg1–Ps66 | Cg63–Pg1–Ps67 | Cg63–Pg1–Ps68 |
| Cg63–Pg1–Ps69 | Cg63–Pg1–Ps70 | Cg63–Pg1–Ps71 | Cg63–Pg1–Ps72 |
| Cg63–Pg1–Ps73 | Cg63–Pg1–Ps74 | Cg63–Pg1–Ps75 | Cg63–Pg1–Ps76 |
| Cg63–Pg1–Ps77 | Cg63–Pg1–Ps78 | Cg63–Pg1–Ps79 | Cg63–Pg1–Ps80 |
| Cg63–Pg1–Ps81 | Cg63–Pg1–Ps82 | Cg63–Pg1–Ps83 | Cg63–Pg1–Ps84 |
| Cg63–Pg1–Ps85 | Cg63–Pg1–Ps86 | Cg63–Pg1–Ps87 | Cg63–Pg1–Ps88 |
| Cg63–Pg1–Ps89 | Cg63–Pg1–Ps90 | Cg63–Pg1–Ps91 | Cg63–Pg1–Ps92 |

| | | | |
|---|---|---|---|
| Cg63-Pg1-Ps93 | Cg63-Pg1-Ps94 | Cg63-Pg1-Ps95 | Cg63-Pg1-Ps96 |
| Cg63-Pg1-Ps97 | Cg63-Pg1-Ps98 | Cg63-Pg1-Ps99 | Cg63-Pg1-Ps100 |
| Cg63-Pg1-Ps101 | Cg63-Pg1-Ps102 | Cg63-Pg1-Ps103 | Cg63-Pg1-Ps104 |
| Cg63-Pg1-Ps105 | Cg63-Pg1-Ps106 | Cg63-Pg1-Ps107 | Cg63-Pg1-Ps108 |
| Cg63-Pg1-Ps109 | Cg63-Pg1-Ps110 | Cg63-Pg1-Ps111 | Cg63-Pg1-Ps112 |
| Cg63-Pg1-Ps113 | Cg63-Pg1-Ps114 | Cg63-Pg1-Ps115 | Cg63-Pg1-Ps116 |
| Cg63-Pg1-Ps117 | Cg63-Pg1-Ps118 | Cg63-Pg1-Ps119 | Cg63-Pg1-Ps120 |
| Cg63-Pg1-Ps121 | Cg63-Pg1-Ps122 | Cg63-Pg1-Ps123 | Cg63-Pg1-Ps124 |
| Cg63-Pg1-Ps125 | Cg63-Pg1-Ps126 | Cg63-Pg1-Ps127 | Cg63-Pg1-Ps128 |
| Cg63-Pg1-Ps129 | Cg63-Pg1-Ps130 | Cg63-Pg1-Ps131 | Cg63-Pg1-Ps132 |
| Cg63-Pg1-Ps133 | Cg63-Pg1-Ps134 | Cg63-Pg1-Ps135 | Cg63-Pg1-Ps136 |
| Cg63-Pg1-Ps137 | Cg63-Pg1-Ps138 | Cg63-Pg1-Ps139 | Cg63-Pg1-Ps140 |
| Cg63-Pg1-Ps141 | Cg63-Pg1-Ps142 | Cg63-Pg1-Ps143 | Cg63-Pg1-Ps144 |
| Cg63-Pg1-Ps145 | Cg63-Pg1-Ps146 | Cg63-Pg1-Ps147 | Cg63-Pg1-Ps148 |
| Cg63-Pg1-Ps149 | Cg63-Pg1-Ps150 | Cg63-Pg1-Ps151 | Cg63-Pg1-Ps152 |
| Cg63-Pg1-Ps153 | Cg63-Pg1-Ps154 | Cg63-Pg1-Ps155 | Cg63-Pg1-Ps156 |
| Cg63-Pg1-Ps157 | Cg63-Pg1-Ps158 | Cg63-Pg1-Ps159 | Cg63-Pg1-Ps160 |
| Cg63-Pg1-Ps161 | Cg63-Pg1-Ps162 | Cg63-Pg1-Ps163 | Cg63-Pg1-Ps164 |
| Cg63-Pg1-Ps165 | Cg63-Pg1-Ps166 | Cg63-Pg1-Ps167 | Cg63-Pg1-Ps168 |
| Cg63-Pg1-Ps169 | Cg63-Pg1-Ps170 | Cg63-Pg1-Ps171 | Cg63-Pg1-Ps172 |
| Cg63-Pg1-Ps173 | Cg63-Pg1-Ps174 | Cg63-Pg1-Ps175 | Cg63-Pg1-Ps176 |
| Cg63-Pg1-Ps177 | Cg63-Pg1-Ps178 | Cg63-Pg1-Ps179 | Cg63-Pg1-Ps180 |
| Cg63-Pg1-Ps181 | Cg63-Pg1-Ps182 | Cg63-Pg1-Ps183 | Cg63-Pg1-Ps184 |
| Cg63-Pg1-Ps185 | Cg63-Pg1-Ps186 | Cg63-Pg1-Ps187 | Cg63-Pg1-Ps188 |
| Cg63-Pg1-Ps189 | Cg63-Pg1-Ps190 | Cg63-Pg1-Ps191 | Cg63-Pg1-Ps192 |
| Cg63-Pg1-Ps193 | Cg63-Pg1-Ps194 | Cg63-Pg1-Ps195 | Cg63-Pg1-Ps196 |
| Cg63-Pg1-Ps197 | Cg63-Pg1-Ps198 | Cg63-Pg1-Ps199 | Cg63-Pg1-Ps200 |
| Cg63-Pg1-Ps201 | Cg63-Pg1-Ps202 | Cg63-Pg1-Ps203 | Cg63-Pg1-Ps204 |
| Cg63-Pg1-Ps205 | Cg63-Pg1-Ps206 | Cg63-Pg1-Ps207 | Cg63-Pg1-Ps208 |
| Cg63-Pg1-Ps209 | Cg63-Pg1-Ps210 | Cg63-Pg1-Ps211 | Cg63-Pg1-Ps212 |
| Cg63-Pg1-Ps213 | Cg63-Pg1-Ps214 | Cg63-Pg1-Ps215 | Cg63-Pg1-Ps216 |
| Cg63-Pg1-Ps217 | Cg63-Pg1-Ps218 | Cg63-Pg1-Ps219 | Cg63-Pg1-Ps220 |
| Cg63-Pg1-Ps221 | Cg63-Pg1-Ps222 | Cg63-Pg1-Ps223 | Cg63-Pg1-Ps224 |
| Cg63-Pg1-Ps225 | Cg63-Pg1-Ps226 | Cg63-Pg1-Ps227 | Cg63-Pg1-Ps228 |
| Cg63-Pg1-Ps229 | Cg63-Pg1-Ps230 | Cg63-Pg1-Ps231 | Cg63-Pg1-Ps232 |
| Cg63-Pg1-Ps233 | Cg63-Pg1-Ps234 | Cg63-Pg1-Ps235 | Cg63-Pg1-Ps236 |
| Cg63-Pg1-Ps237 | Cg63-Pg1-Ps238 | Cg63-Pg1-Ps239 | Cg63-Pg1-Ps240 |
| Cg63-Pg1-Ps241 | Cg63-Pg1-Ps242 | Cg63-Pg1-Ps243 | |

| | | | |
|---|---|---|---|
| Cg64-Pg1-Ps1 | Cg64-Pg1-Ps2 | Cg64-Pg1-Ps3 | Cg64-Pg1-Ps4 |
| Cg64-Pg1-Ps5 | Cg64-Pg1-Ps6 | Cg64-Pg1-Ps7 | Cg64-Pg1-Ps8 |
| Cg64-Pg1-Ps9 | Cg64-Pg1-Ps10 | Cg64-Pg1-Ps11 | Cg64-Pg1-Ps12 |
| Cg64-Pg1-Ps13 | Cg64-Pg1-Ps14 | Cg64-Pg1-Ps15 | Cg64-Pg1-Ps16 |
| Cg64-Pg1-Ps17 | Cg64-Pg1-Ps18 | Cg64-Pg1-Ps19 | Cg64-Pg1-Ps20 |
| Cg64-Pg1-Ps21 | Cg64-Pg1-Ps22 | Cg64-Pg1-Ps23 | Cg64-Pg1-Ps24 |
| Cg64-Pg1-Ps25 | Cg64-Pg1-Ps26 | Cg64-Pg1-Ps27 | Cg64-Pg1-Ps28 |
| Cg64-Pg1-Ps29 | Cg64-Pg1-Ps30 | Cg64-Pg1-Ps31 | Cg64-Pg1-Ps32 |
| Cg64-Pg1-Ps33 | Cg64-Pg1-Ps34 | Cg64-Pg1-Ps35 | Cg64-Pg1-Ps36 |
| Cg64-Pg1-Ps37 | Cg64-Pg1-Ps38 | Cg64-Pg1-Ps39 | Cg64-Pg1-Ps40 |
| Cg64-Pg1-Ps41 | Cg64-Pg1-Ps42 | Cg64-Pg1-Ps43 | Cg64-Pg1-Ps44 |
| Cg64-Pg1-Ps45 | Cg64-Pg1-Ps46 | Cg64-Pg1-Ps47 | Cg64-Pg1-Ps48 |
| Cg64-Pg1-Ps49 | Cg64-Pg1-Ps50 | Cg64-Pg1-Ps51 | Cg64-Pg1-Ps52 |

| | | | |
|---|---|---|---|
| Cg64–Pg1–Ps53 | Cg64–Pg1–Ps54 | Cg64–Pg1–Ps55 | Cg64–Pg1–Ps56 |
| Cg64–Pg1–Ps57 | Cg64–Pg1–Ps58 | Cg64–Pg1–Ps59 | Cg64–Pg1–Ps60 |
| Cg64–Pg1–Ps61 | Cg64–Pg1–Ps62 | Cg64–Pg1–Ps63 | Cg64–Pg1–Ps64 |
| Cg64–Pg1–Ps65 | Cg64–Pg1–Ps66 | Cg64–Pg1–Ps67 | Cg64–Pg1–Ps68 |
| Cg64–Pg1–Ps69 | Cg64–Pg1–Ps70 | Cg64–Pg1–Ps71 | Cg64–Pg1–Ps72 |
| Cg64–Pg1–Ps73 | Cg64–Pg1–Ps74 | Cg64–Pg1–Ps75 | Cg64–Pg1–Ps76 |
| Cg64–Pg1–Ps77 | Cg64–Pg1–Ps78 | Cg64–Pg1–Ps79 | Cg64–Pg1–Ps80 |
| Cg64–Pg1–Ps81 | Cg64–Pg1–Ps82 | Cg64–Pg1–Ps83 | Cg64–Pg1–Ps84 |
| Cg64–Pg1–Ps85 | Cg64–Pg1–Ps86 | Cg64–Pg1–Ps87 | Cg64–Pg1–Ps88 |
| Cg64–Pg1–Ps89 | Cg64–Pg1–Ps90 | Cg64–Pg1–Ps91 | Cg64–Pg1–Ps92 |
| Cg64–Pg1–Ps93 | Cg64–Pg1–Ps94 | Cg64–Pg1–Ps95 | Cg64–Pg1–Ps96 |
| Cg64–Pg1–Ps97 | Cg64–Pg1–Ps98 | Cg64–Pg1–Ps99 | Cg64–Pg1–Ps100 |
| Cg64–Pg1–Ps101 | Cg64–Pg1–Ps102 | Cg64–Pg1–Ps103 | Cg64–Pg1–Ps104 |
| Cg64–Pg1–Ps105 | Cg64–Pg1–Ps106 | Cg64–Pg1–Ps107 | Cg64–Pg1–Ps108 |
| Cg64–Pg1–Ps109 | Cg64–Pg1–Ps110 | Cg64–Pg1–Ps111 | Cg64–Pg1–Ps112 |
| Cg64–Pg1–Ps113 | Cg64–Pg1–Ps114 | Cg64–Pg1–Ps115 | Cg64–Pg1–Ps116 |
| Cg64–Pg1–Ps117 | Cg64–Pg1–Ps118 | Cg64–Pg1–Ps119 | Cg64–Pg1–Ps120 |
| Cg64–Pg1–Ps121 | Cg64–Pg1–Ps122 | Cg64–Pg1–Ps123 | Cg64–Pg1–Ps124 |
| Cg64–Pg1–Ps125 | Cg64–Pg1–Ps126 | Cg64–Pg1–Ps127 | Cg64–Pg1–Ps128 |
| Cg64–Pg1–Ps129 | Cg64–Pg1–Ps130 | Cg64–Pg1–Ps131 | Cg64–Pg1–Ps132 |
| Cg64–Pg1–Ps133 | Cg64–Pg1–Ps134 | Cg64–Pg1–Ps135 | Cg64–Pg1–Ps136 |
| Cg64–Pg1–Ps137 | Cg64–Pg1–Ps138 | Cg64–Pg1–Ps139 | Cg64–Pg1–Ps140 |
| Cg64–Pg1–Ps141 | Cg64–Pg1–Ps142 | Cg64–Pg1–Ps143 | Cg64–Pg1–Ps144 |
| Cg64–Pg1–Ps145 | Cg64–Pg1–Ps146 | Cg64–Pg1–Ps147 | Cg64–Pg1–Ps148 |
| Cg64–Pg1–Ps149 | Cg64–Pg1–Ps150 | Cg64–Pg1–Ps151 | Cg64–Pg1–Ps152 |
| Cg64–Pg1–Ps153 | Cg64–Pg1–Ps154 | Cg64–Pg1–Ps155 | Cg64–Pg1–Ps156 |
| Cg64–Pg1–Ps157 | Cg64–Pg1–Ps158 | Cg64–Pg1–Ps159 | Cg64–Pg1–Ps160 |
| Cg64–Pg1–Ps161 | Cg64–Pg1–Ps162 | Cg64–Pg1–Ps163 | Cg64–Pg1–Ps164 |
| Cg64–Pg1–Ps165 | Cg64–Pg1–Ps166 | Cg64–Pg1–Ps167 | Cg64–Pg1–Ps168 |
| Cg64–Pg1–Ps169 | Cg64–Pg1–Ps170 | Cg64–Pg1–Ps171 | Cg64–Pg1–Ps172 |
| Cg64–Pg1–Ps173 | Cg64–Pg1–Ps174 | Cg64–Pg1–Ps175 | Cg64–Pg1–Ps176 |
| Cg64–Pg1–Ps177 | Cg64–Pg1–Ps178 | Cg64–Pg1–Ps179 | Cg64–Pg1–Ps180 |
| Cg64–Pg1–Ps181 | Cg64–Pg1–Ps182 | Cg64–Pg1–Ps183 | Cg64–Pg1–Ps184 |
| Cg64–Pg1–Ps185 | Cg64–Pg1–Ps186 | Cg64–Pg1–Ps187 | Cg64–Pg1–Ps188 |
| Cg64–Pg1–Ps189 | Cg64–Pg1–Ps190 | Cg64–Pg1–Ps191 | Cg64–Pg1–Ps192 |
| Cg64–Pg1–Ps193 | Cg64–Pg1–Ps194 | Cg64–Pg1–Ps195 | Cg64–Pg1–Ps196 |
| Cg64–Pg1–Ps197 | Cg64–Pg1–Ps198 | Cg64–Pg1–Ps199 | Cg64–Pg1–Ps200 |
| Cg64–Pg1–Ps201 | Cg64–Pg1–Ps202 | Cg64–Pg1–Ps203 | Cg64–Pg1–Ps204 |
| Cg64–Pg1–Ps205 | Cg64–Pg1–Ps206 | Cg64–Pg1–Ps207 | Cg64–Pg1–Ps208 |
| Cg64–Pg1–Ps209 | Cg64–Pg1–Ps210 | Cg64–Pg1–Ps211 | Cg64–Pg1–Ps212 |
| Cg64–Pg1–Ps213 | Cg64–Pg1–Ps214 | Cg64–Pg1–Ps215 | Cg64–Pg1–Ps216 |
| Cg64–Pg1–Ps217 | Cg64–Pg1–Ps218 | Cg64–Pg1–Ps219 | Cg64–Pg1–Ps220 |
| Cg64–Pg1–Ps221 | Cg64–Pg1–Ps222 | Cg64–Pg1–Ps223 | Cg64–Pg1–Ps224 |
| Cg64–Pg1–Ps225 | Cg64–Pg1–Ps226 | Cg64–Pg1–Ps227 | Cg64–Pg1–Ps228 |
| Cg64–Pg1–Ps229 | Cg64–Pg1–Ps230 | Cg64–Pg1–Ps231 | Cg64–Pg1–Ps232 |
| Cg64–Pg1–Ps233 | Cg64–Pg1–Ps234 | Cg64–Pg1–Ps235 | Cg64–Pg1–Ps236 |
| Cg64–Pg1–Ps237 | Cg64–Pg1–Ps238 | Cg64–Pg1–Ps239 | Cg64–Pg1–Ps240 |
| Cg64–Pg1–Ps241 | Cg64–Pg1–Ps242 | Cg64–Pg1–Ps243 | |

| | | | |
|---|---|---|---|
| Cg65–Pg1–Ps1 | Cg65–Pg1–Ps2 | Cg65–Pg1–Ps3 | Cg65–Pg1–Ps4 |
| Cg65–Pg1–Ps5 | Cg65–Pg1–Ps6 | Cg65–Pg1–Ps7 | Cg65–Pg1–Ps8 |
| Cg65–Pg1–Ps9 | Cg65–Pg1–Ps10 | Cg65–Pg1–Ps11 | Cg65–Pg1–Ps12 |

| | | | |
|---|---|---|---|
| Cg65–Pg1–Ps13 | Cg65–Pg1–Ps14 | Cg65–Pg1–Ps15 | Cg65–Pg1–Ps16 |
| Cg65–Pg1–Ps17 | Cg65–Pg1–Ps18 | Cg65–Pg1–Ps19 | Cg65–Pg1–Ps20 |
| Cg65–Pg1–Ps21 | Cg65–Pg1–Ps22 | Cg65–Pg1–Ps23 | Cg65–Pg1–Ps24 |
| Cg65–Pg1–Ps25 | Cg65–Pg1–Ps26 | Cg65–Pg1–Ps27 | Cg65–Pg1–Ps28 |
| Cg65–Pg1–Ps29 | Cg65–Pg1–Ps30 | Cg65–Pg1–Ps31 | Cg65–Pg1–Ps32 |
| Cg65–Pg1–Ps33 | Cg65–Pg1–Ps34 | Cg65–Pg1–Ps35 | Cg65–Pg1–Ps36 |
| Cg65–Pg1–Ps37 | Cg65–Pg1–Ps38 | Cg65–Pg1–Ps39 | Cg65–Pg1–Ps40 |
| Cg65–Pg1–Ps41 | Cg65–Pg1–Ps42 | Cg65–Pg1–Ps43 | Cg65–Pg1–Ps44 |
| Cg65–Pg1–Ps45 | Cg65–Pg1–Ps46 | Cg65–Pg1–Ps47 | Cg65–Pg1–Ps48 |
| Cg65–Pg1–Ps49 | Cg65–Pg1–Ps50 | Cg65–Pg1–Ps51 | Cg65–Pg1–Ps52 |
| Cg65–Pg1–Ps53 | Cg65–Pg1–Ps54 | Cg65–Pg1–Ps55 | Cg65–Pg1–Ps56 |
| Cg65–Pg1–Ps57 | Cg65–Pg1–Ps58 | Cg65–Pg1–Ps59 | Cg65–Pg1–Ps60 |
| Cg65–Pg1–Ps61 | Cg65–Pg1–Ps62 | Cg65–Pg1–Ps63 | Cg65–Pg1–Ps64 |
| Cg65–Pg1–Ps65 | Cg65–Pg1–Ps66 | Cg65–Pg1–Ps67 | Cg65–Pg1–Ps68 |
| Cg65–Pg1–Ps69 | Cg65–Pg1–Ps70 | Cg65–Pg1–Ps71 | Cg65–Pg1–Ps72 |
| Cg65–Pg1–Ps73 | Cg65–Pg1–Ps74 | Cg65–Pg1–Ps75 | Cg65–Pg1–Ps76 |
| Cg65–Pg1–Ps77 | Cg65–Pg1–Ps78 | Cg65–Pg1–Ps79 | Cg65–Pg1–Ps80 |
| Cg65–Pg1–Ps81 | Cg65–Pg1–Ps82 | Cg65–Pg1–Ps83 | Cg65–Pg1–Ps84 |
| Cg65–Pg1–Ps85 | Cg65–Pg1–Ps86 | Cg65–Pg1–Ps87 | Cg65–Pg1–Ps88 |
| Cg65–Pg1–Ps89 | Cg65–Pg1–Ps90 | Cg65–Pg1–Ps91 | Cg65–Pg1–Ps92 |
| Cg65–Pg1–Ps93 | Cg65–Pg1–Ps94 | Cg65–Pg1–Ps95 | Cg65–Pg1–Ps96 |
| Cg65–Pg1–Ps97 | Cg65–Pg1–Ps98 | Cg65–Pg1–Ps99 | Cg65–Pg1–Ps100 |
| Cg65–Pg1–Ps101 | Cg65–Pg1–Ps102 | Cg65–Pg1–Ps103 | Cg65–Pg1–Ps104 |
| Cg65–Pg1–Ps105 | Cg65–Pg1–Ps106 | Cg65–Pg1–Ps107 | Cg65–Pg1–Ps108 |
| Cg65–Pg1–Ps109 | Cg65–Pg1–Ps110 | Cg65–Pg1–Ps111 | Cg65–Pg1–Ps112 |
| Cg65–Pg1–Ps113 | Cg65–Pg1–Ps114 | Cg65–Pg1–Ps115 | Cg65–Pg1–Ps116 |
| Cg65–Pg1–Ps117 | Cg65–Pg1–Ps118 | Cg65–Pg1–Ps119 | Cg65–Pg1–Ps120 |
| Cg65–Pg1–Ps121 | Cg65–Pg1–Ps122 | Cg65–Pg1–Ps123 | Cg65–Pg1–Ps124 |
| Cg65–Pg1–Ps125 | Cg65–Pg1–Ps126 | Cg65–Pg1–Ps127 | Cg65–Pg1–Ps128 |
| Cg65–Pg1–Ps129 | Cg65–Pg1–Ps130 | Cg65–Pg1–Ps131 | Cg65–Pg1–Ps132 |
| Cg65–Pg1–Ps133 | Cg65–Pg1–Ps134 | Cg65–Pg1–Ps135 | Cg65–Pg1–Ps136 |
| Cg65–Pg1–Ps137 | Cg65–Pg1–Ps138 | Cg65–Pg1–Ps139 | Cg65–Pg1–Ps140 |
| Cg65–Pg1–Ps141 | Cg65–Pg1–Ps142 | Cg65–Pg1–Ps143 | Cg65–Pg1–Ps144 |
| Cg65–Pg1–Ps145 | Cg65–Pg1–Ps146 | Cg65–Pg1–Ps147 | Cg65–Pg1–Ps148 |
| Cg65–Pg1–Ps149 | Cg65–Pg1–Ps150 | Cg65–Pg1–Ps151 | Cg65–Pg1–Ps152 |
| Cg65–Pg1–Ps153 | Cg65–Pg1–Ps154 | Cg65–Pg1–Ps155 | Cg65–Pg1–Ps156 |
| Cg65–Pg1–Ps157 | Cg65–Pg1–Ps158 | Cg65–Pg1–Ps159 | Cg65–Pg1–Ps160 |
| Cg65–Pg1–Ps161 | Cg65–Pg1–Ps162 | Cg65–Pg1–Ps163 | Cg65–Pg1–Ps164 |
| Cg65–Pg1–Ps165 | Cg65–Pg1–Ps166 | Cg65–Pg1–Ps167 | Cg65–Pg1–Ps168 |
| Cg65–Pg1–Ps169 | Cg65–Pg1–Ps170 | Cg65–Pg1–Ps171 | Cg65–Pg1–Ps172 |
| Cg65–Pg1–Ps173 | Cg65–Pg1–Ps174 | Cg65–Pg1–Ps175 | Cg65–Pg1–Ps176 |
| Cg65–Pg1–Ps177 | Cg65–Pg1–Ps178 | Cg65–Pg1–Ps179 | Cg65–Pg1–Ps180 |
| Cg65–Pg1–Ps181 | Cg65–Pg1–Ps182 | Cg65–Pg1–Ps183 | Cg65–Pg1–Ps184 |
| Cg65–Pg1–Ps185 | Cg65–Pg1–Ps186 | Cg65–Pg1–Ps187 | Cg65–Pg1–Ps188 |
| Cg65–Pg1–Ps189 | Cg65–Pg1–Ps190 | Cg65–Pg1–Ps191 | Cg65–Pg1–Ps192 |
| Cg65–Pg1–Ps193 | Cg65–Pg1–Ps194 | Cg65–Pg1–Ps195 | Cg65–Pg1–Ps196 |
| Cg65–Pg1–Ps197 | Cg65–Pg1–Ps198 | Cg65–Pg1–Ps199 | Cg65–Pg1–Ps200 |
| Cg65–Pg1–Ps201 | Cg65–Pg1–Ps202 | Cg65–Pg1–Ps203 | Cg65–Pg1–Ps204 |
| Cg65–Pg1–Ps205 | Cg65–Pg1–Ps206 | Cg65–Pg1–Ps207 | Cg65–Pg1–Ps208 |
| Cg65–Pg1–Ps209 | Cg65–Pg1–Ps210 | Cg65–Pg1–Ps211 | Cg65–Pg1–Ps212 |
| Cg65–Pg1–Ps213 | Cg65–Pg1–Ps214 | Cg65–Pg1–Ps215 | Cg65–Pg1–Ps216 |
| Cg65–Pg1–Ps217 | Cg65–Pg1–Ps218 | Cg65–Pg1–Ps219 | Cg65–Pg1–Ps220 |

| | | | |
|---|---|---|---|
| Cg65–Pgl–Ps221 | Cg65–Pgl–Ps222 | Cg65–Pgl–Ps223 | Cg65–Pgl–Ps224 |
| Cg65–Pgl–Ps225 | Cg65–Pgl–Ps226 | Cg65–Pgl–Ps227 | Cg65–Pgl–Ps228 |
| Cg65–Pgl–Ps229 | Cg65–Pgl–Ps230 | Cg65–Pgl–Ps231 | Cg65–Pgl–Ps232 |
| Cg65–Pgl–Ps233 | Cg65–Pgl–Ps234 | Cg65–Pgl–Ps235 | Cg65–Pgl–Ps236 |
| Cg65–Pgl–Ps237 | Cg65–Pgl–Ps238 | Cg65–Pgl–Ps239 | Cg65–Pgl–Ps240 |
| Cg65–Pgl–Ps241 | Cg65–Pgl–Ps242 | Cg65–Pgl–Ps243 | |

| | | | |
|---|---|---|---|
| Cg67–Pgl–Ps1 | Cg67–Pgl–Ps2 | Cg67–Pgl–Ps3 | Cg67–Pgl–Ps4 |
| Cg67–Pgl–Ps5 | Cg67–Pgl–Ps6 | Cg67–Pgl–Ps7 | Cg67–Pgl–Ps8 |
| Cg67–Pgl–Ps9 | Cg67–Pgl–Ps10 | Cg67–Pgl–Ps11 | Cg67–Pgl–Ps12 |
| Cg67–Pgl–Ps13 | Cg67–Pgl–Ps14 | Cg67–Pgl–Ps15 | Cg67–Pgl–Ps16 |
| Cg67–Pgl–Ps17 | Cg67–Pgl–Ps18 | Cg67–Pgl–Ps19 | Cg67–Pgl–Ps20 |
| Cg67–Pgl–Ps21 | Cg67–Pgl–Ps22 | Cg67–Pgl–Ps23 | Cg67–Pgl–Ps24 |
| Cg67–Pgl–Ps25 | Cg67–Pgl–Ps26 | Cg67–Pgl–Ps27 | Cg67–Pgl–Ps28 |
| Cg67–Pgl–Ps29 | Cg67–Pgl–Ps30 | Cg67–Pgl–Ps31 | Cg67–Pgl–Ps32 |
| Cg67–Pgl–Ps33 | Cg67–Pgl–Ps34 | Cg67–Pgl–Ps35 | Cg67–Pgl–Ps36 |
| Cg67–Pgl–Ps37 | Cg67–Pgl–Ps38 | Cg67–Pgl–Ps39 | Cg67–Pgl–Ps40 |
| Cg67–Pgl–Ps41 | Cg67–Pgl–Ps42 | Cg67–Pgl–Ps43 | Cg67–Pgl–Ps44 |
| Cg67–Pgl–Ps45 | Cg67–Pgl–Ps46 | Cg67–Pgl–Ps47 | Cg67–Pgl–Ps48 |
| Cg67–Pgl–Ps49 | Cg67–Pgl–Ps50 | Cg67–Pgl–Ps51 | Cg67–Pgl–Ps52 |
| Cg67–Pgl–Ps53 | Cg67–Pgl–Ps54 | Cg67–Pgl–Ps55 | Cg67–Pgl–Ps56 |
| Cg67–Pgl–Ps57 | Cg67–Pgl–Ps58 | Cg67–Pgl–Ps59 | Cg67–Pgl–Ps60 |
| Cg67–Pgl–Ps61 | Cg67–Pgl–Ps62 | Cg67–Pgl–Ps63 | Cg67–Pgl–Ps64 |
| Cg67–Pgl–Ps65 | Cg67–Pgl–Ps66 | Cg67–Pgl–Ps67 | Cg67–Pgl–Ps68 |
| Cg67–Pgl–Ps69 | Cg67–Pgl–Ps70 | Cg67–Pgl–Ps71 | Cg67–Pgl–Ps72 |
| Cg67–Pgl–Ps73 | Cg67–Pgl–Ps74 | Cg67–Pgl–Ps75 | Cg67–Pgl–Ps76 |
| Cg67–Pgl–Ps77 | Cg67–Pgl–Ps78 | Cg67–Pgl–Ps79 | Cg67–Pgl–Ps80 |
| Cg67–Pgl–Ps81 | Cg67–Pgl–Ps82 | Cg67–Pgl–Ps83 | Cg67–Pgl–Ps84 |
| Cg67–Pgl–Ps85 | Cg67–Pgl–Ps86 | Cg67–Pgl–Ps87 | Cg67–Pgl–Ps88 |
| Cg67–Pgl–Ps89 | Cg67–Pgl–Ps90 | Cg67–Pgl–Ps91 | Cg67–Pgl–Ps92 |
| Cg67–Pgl–Ps93 | Cg67–Pgl–Ps94 | Cg67–Pgl–Ps95 | Cg67–Pgl–Ps96 |
| Cg67–Pgl–Ps97 | Cg67–Pgl–Ps98 | Cg67–Pgl–Ps99 | Cg67–Pgl–Ps100 |
| Cg67–Pgl–Ps101 | Cg67–Pgl–Ps102 | Cg67–Pgl–Ps103 | Cg67–Pgl–Ps104 |
| Cg67–Pgl–Ps105 | Cg67–Pgl–Ps106 | Cg67–Pgl–Ps107 | Cg67–Pgl–Ps108 |
| Cg67–Pgl–Ps109 | Cg67–Pgl–Ps110 | Cg67–Pgl–Ps111 | Cg67–Pgl–Ps112 |
| Cg67–Pgl–Ps113 | Cg67–Pgl–Ps114 | Cg67–Pgl–Ps115 | Cg67–Pgl–Ps116 |
| Cg67–Pgl–Ps117 | Cg67–Pgl–Ps118 | Cg67–Pgl–Ps119 | Cg67–Pgl–Ps120 |
| Cg67–Pgl–Ps121 | Cg67–Pgl–Ps122 | Cg67–Pgl–Ps123 | Cg67–Pgl–Ps124 |
| Cg67–Pgl–Ps125 | Cg67–Pgl–Ps126 | Cg67–Pgl–Ps127 | Cg67–Pgl–Ps128 |
| Cg67–Pgl–Ps129 | Cg67–Pgl–Ps130 | Cg67–Pgl–Ps131 | Cg67–Pgl–Ps132 |
| Cg67–Pgl–Ps133 | Cg67–Pgl–Ps134 | Cg67–Pgl–Ps135 | Cg67–Pgl–Ps136 |
| Cg67–Pgl–Ps137 | Cg67–Pgl–Ps138 | Cg67–Pgl–Ps139 | Cg67–Pgl–Ps140 |
| Cg67–Pgl–Ps141 | Cg67–Pgl–Ps142 | Cg67–Pgl–Ps143 | Cg67–Pgl–Ps144 |
| Cg67–Pgl–Ps145 | Cg67–Pgl–Ps146 | Cg67–Pgl–Ps147 | Cg67–Pgl–Ps148 |
| Cg67–Pgl–Ps149 | Cg67–Pgl–Ps150 | Cg67–Pgl–Ps151 | Cg67–Pgl–Ps152 |
| Cg67–Pgl–Ps153 | Cg67–Pgl–Ps154 | Cg67–Pgl–Ps155 | Cg67–Pgl–Ps156 |
| Cg67–Pgl–Ps157 | Cg67–Pgl–Ps158 | Cg67–Pgl–Ps159 | Cg67–Pgl–Ps160 |
| Cg67–Pgl–Ps161 | Cg67–Pgl–Ps162 | Cg67–Pgl–Ps163 | Cg67–Pgl–Ps164 |
| Cg67–Pgl–Ps165 | Cg67–Pgl–Ps166 | Cg67–Pgl–Ps167 | Cg67–Pgl–Ps168 |
| Cg67–Pgl–Ps169 | Cg67–Pgl–Ps170 | Cg67–Pgl–Ps171 | Cg67–Pgl–Ps172 |
| Cg67–Pgl–Ps173 | Cg67–Pgl–Ps174 | Cg67–Pgl–Ps175 | Cg67–Pgl–Ps176 |
| Cg67–Pgl–Ps177 | Cg67–Pgl–Ps178 | Cg67–Pgl–Ps179 | Cg67–Pgl–Ps180 |

| | | | |
|---|---|---|---|
| Cg67–Pg1–Ps181 | Cg67–Pg1–Ps182 | Cg67–Pg1–Ps183 | Cg67–Pg1–Ps184 |
| Cg67–Pg1–Ps185 | Cg67–Pg1–Ps186 | Cg67–Pg1–Ps187 | Cg67–Pg1–Ps188 |
| Cg67–Pg1–Ps189 | Cg67–Pg1–Ps190 | Cg67–Pg1–Ps191 | Cg67–Pg1–Ps192 |
| Cg67–Pg1–Ps193 | Cg67–Pg1–Ps194 | Cg67–Pg1–Ps195 | Cg67–Pg1–Ps196 |
| Cg67–Pg1–Ps197 | Cg67–Pg1–Ps198 | Cg67–Pg1–Ps199 | Cg67–Pg1–Ps200 |
| Cg67–Pg1–Ps201 | Cg67–Pg1–Ps202 | Cg67–Pg1–Ps203 | Cg67–Pg1–Ps204 |
| Cg67–Pg1–Ps205 | Cg67–Pg1–Ps206 | Cg67–Pg1–Ps207 | Cg67–Pg1–Ps208 |
| Cg67–Pg1–Ps209 | Cg67–Pg1–Ps210 | Cg67–Pg1–Ps211 | Cg67–Pg1–Ps212 |
| Cg67–Pg1–Ps213 | Cg67–Pg1–Ps214 | Cg67–Pg1–Ps215 | Cg67–Pg1–Ps216 |
| Cg67–Pg1–Ps217 | Cg67–Pg1–Ps218 | Cg67–Pg1–Ps219 | Cg67–Pg1–Ps220 |
| Cg67–Pg1–Ps221 | Cg67–Pg1–Ps222 | Cg67–Pg1–Ps223 | Cg67–Pg1–Ps224 |
| Cg67–Pg1–Ps225 | Cg67–Pg1–Ps226 | Cg67–Pg1–Ps227 | Cg67–Pg1–Ps228 |
| Cg67–Pg1–Ps229 | Cg67–Pg1–Ps230 | Cg67–Pg1–Ps231 | Cg67–Pg1–Ps232 |
| Cg67–Pg1–Ps233 | Cg67–Pg1–Ps234 | Cg67–Pg1–Ps235 | Cg67–Pg1–Ps236 |
| Cg67–Pg1–Ps237 | Cg67–Pg1–Ps238 | Cg67–Pg1–Ps239 | Cg67–Pg1–Ps240 |
| Cg67–Pg1–Ps241 | Cg67–Pg1–Ps242 | Cg67–Pg1–Ps243 | |

| | | | |
|---|---|---|---|
| Cg68–Pg1–Ps1 | Cg68–Pg1–Ps2 | Cg68–Pg1–Ps3 | Cg68–Pg1–Ps4 |
| Cg68–Pg1–Ps5 | Cg68–Pg1–Ps6 | Cg68–Pg1–Ps7 | Cg68–Pg1–Ps8 |
| Cg68–Pg1–Ps9 | Cg68–Pg1–Ps10 | Cg68–Pg1–Ps11 | Cg68–Pg1–Ps12 |
| Cg68–Pg1–Ps13 | Cg68–Pg1–Ps14 | Cg68–Pg1–Ps15 | Cg68–Pg1–Ps16 |
| Cg68–Pg1–Ps17 | Cg68–Pg1–Ps18 | Cg68–Pg1–Ps19 | Cg68–Pg1–Ps20 |
| Cg68–Pg1–Ps21 | Cg68–Pg1–Ps22 | Cg68–Pg1–Ps23 | Cg68–Pg1–Ps24 |
| Cg68–Pg1–Ps25 | Cg68–Pg1–Ps26 | Cg68–Pg1–Ps27 | Cg68–Pg1–Ps28 |
| Cg68–Pg1–Ps29 | Cg68–Pg1–Ps30 | Cg68–Pg1–Ps31 | Cg68–Pg1–Ps32 |
| Cg68–Pg1–Ps33 | Cg68–Pg1–Ps34 | Cg68–Pg1–Ps35 | Cg68–Pg1–Ps36 |
| Cg68–Pg1–Ps37 | Cg68–Pg1–Ps38 | Cg68–Pg1–Ps39 | Cg68–Pg1–Ps40 |
| Cg68–Pg1–Ps41 | Cg68–Pg1–Ps42 | Cg68–Pg1–Ps43 | Cg68–Pg1–Ps44 |
| Cg68–Pg1–Ps45 | Cg68–Pg1–Ps46 | Cg68–Pg1–Ps47 | Cg68–Pg1–Ps48 |
| Cg68–Pg1–Ps49 | Cg68–Pg1–Ps50 | Cg68–Pg1–Ps51 | Cg68–Pg1–Ps52 |
| Cg68–Pg1–Ps53 | Cg68–Pg1–Ps54 | Cg68–Pg1–Ps55 | Cg68–Pg1–Ps56 |
| Cg68–Pg1–Ps57 | Cg68–Pg1–Ps58 | Cg68–Pg1–Ps59 | Cg68–Pg1–Ps60 |
| Cg68–Pg1–Ps61 | Cg68–Pg1–Ps62 | Cg68–Pg1–Ps63 | Cg68–Pg1–Ps64 |
| Cg68–Pg1–Ps65 | Cg68–Pg1–Ps66 | Cg68–Pg1–Ps67 | Cg68–Pg1–Ps68 |
| Cg68–Pg1–Ps69 | Cg68–Pg1–Ps70 | Cg68–Pg1–Ps71 | Cg68–Pg1–Ps72 |
| Cg68–Pg1–Ps73 | Cg68–Pg1–Ps74 | Cg68–Pg1–Ps75 | Cg68–Pg1–Ps76 |
| Cg68–Pg1–Ps77 | Cg68–Pg1–Ps78 | Cg68–Pg1–Ps79 | Cg68–Pg1–Ps80 |
| Cg68–Pg1–Ps81 | Cg68–Pg1–Ps82 | Cg68–Pg1–Ps83 | Cg68–Pg1–Ps84 |
| Cg68–Pg1–Ps85 | Cg68–Pg1–Ps86 | Cg68–Pg1–Ps87 | Cg68–Pg1–Ps88 |
| Cg68–Pg1–Ps89 | Cg68–Pg1–Ps90 | Cg68–Pg1–Ps91 | Cg68–Pg1–Ps92 |
| Cg68–Pg1–Ps93 | Cg68–Pg1–Ps94 | Cg68–Pg1–Ps95 | Cg68–Pg1–Ps96 |
| Cg68–Pg1–Ps97 | Cg68–Pg1–Ps98 | Cg68–Pg1–Ps99 | Cg68–Pg1–Ps100 |
| Cg68–Pg1–Ps101 | Cg68–Pg1–Ps102 | Cg68–Pg1–Ps103 | Cg68–Pg1–Ps104 |
| Cg68–Pg1–Ps105 | Cg68–Pg1–Ps106 | Cg68–Pg1–Ps107 | Cg68–Pg1–Ps108 |
| Cg68–Pg1–Ps109 | Cg68–Pg1–Ps110 | Cg68–Pg1–Ps111 | Cg68–Pg1–Ps112 |
| Cg68–Pg1–Ps113 | Cg68–Pg1–Ps114 | Cg68–Pg1–Ps115 | Cg68–Pg1–Ps116 |
| Cg68–Pg1–Ps117 | Cg68–Pg1–Ps118 | Cg68–Pg1–Ps119 | Cg68–Pg1–Ps120 |
| Cg68–Pg1–Ps121 | Cg68–Pg1–Ps122 | Cg68–Pg1–Ps123 | Cg68–Pg1–Ps124 |
| Cg68–Pg1–Ps125 | Cg68–Pg1–Ps126 | Cg68–Pg1–Ps127 | Cg68–Pg1–Ps128 |
| Cg68–Pg1–Ps129 | Cg68–Pg1–Ps130 | Cg68–Pg1–Ps131 | Cg68–Pg1–Ps132 |
| Cg68–Pg1–Ps133 | Cg68–Pg1–Ps134 | Cg68–Pg1–Ps135 | Cg68–Pg1–Ps136 |
| Cg68–Pg1–Ps137 | Cg68–Pg1–Ps138 | Cg68–Pg1–Ps139 | Cg68–Pg1–Ps140 |

| | | | |
|---|---|---|---|
| Cg68–Pg1–Ps141 | Cg68–Pg1–Ps142 | Cg68–Pg1–Ps143 | Cg68–Pg1–Ps144 |
| Cg68–Pg1–Ps145 | Cg68–Pg1–Ps146 | Cg68–Pg1–Ps147 | Cg68–Pg1–Ps148 |
| Cg68–Pg1–Ps149 | Cg68–Pg1–Ps150 | Cg68–Pg1–Ps151 | Cg68–Pg1–Ps152 |
| Cg68–Pg1–Ps153 | Cg68–Pg1–Ps154 | Cg68–Pg1–Ps155 | Cg68–Pg1–Ps156 |
| Cg68–Pg1–Ps157 | Cg68–Pg1–Ps158 | Cg68–Pg1–Ps159 | Cg68–Pg1–Ps160 |
| Cg68–Pg1–Ps161 | Cg68–Pg1–Ps162 | Cg68–Pg1–Ps163 | Cg68–Pg1–Ps164 |
| Cg68–Pg1–Ps165 | Cg68–Pg1–Ps166 | Cg68–Pg1–Ps167 | Cg68–Pg1–Ps168 |
| Cg68–Pg1–Ps169 | Cg68–Pg1–Ps170 | Cg68–Pg1–Ps171 | Cg68–Pg1–Ps172 |
| Cg68–Pg1–Ps173 | Cg68–Pg1–Ps174 | Cg68–Pg1–Ps175 | Cg68–Pg1–Ps176 |
| Cg68–Pg1–Ps177 | Cg68–Pg1–Ps178 | Cg68–Pg1–Ps179 | Cg68–Pg1–Ps180 |
| Cg68–Pg1–Ps181 | Cg68–Pg1–Ps182 | Cg68–Pg1–Ps183 | Cg68–Pg1–Ps184 |
| Cg68–Pg1–Ps185 | Cg68–Pg1–Ps186 | Cg68–Pg1–Ps187 | Cg68–Pg1–Ps188 |
| Cg68–Pg1–Ps189 | Cg68–Pg1–Ps190 | Cg68–Pg1–Ps191 | Cg68–Pg1–Ps192 |
| Cg68–Pg1–Ps193 | Cg68–Pg1–Ps194 | Cg68–Pg1–Ps195 | Cg68–Pg1–Ps196 |
| Cg68–Pg1–Ps197 | Cg68–Pg1–Ps198 | Cg68–Pg1–Ps199 | Cg68–Pg1–Ps200 |
| Cg68–Pg1–Ps201 | Cg68–Pg1–Ps202 | Cg68–Pg1–Ps203 | Cg68–Pg1–Ps204 |
| Cg68–Pg1–Ps205 | Cg68–Pg1–Ps206 | Cg68–Pg1–Ps207 | Cg68–Pg1–Ps208 |
| Cg68–Pg1–Ps209 | Cg68–Pg1–Ps210 | Cg68–Pg1–Ps211 | Cg68–Pg1–Ps212 |
| Cg68–Pg1–Ps213 | Cg68–Pg1–Ps214 | Cg68–Pg1–Ps215 | Cg68–Pg1–Ps216 |
| Cg68–Pg1–Ps217 | Cg68–Pg1–Ps218 | Cg68–Pg1–Ps219 | Cg68–Pg1–Ps220 |
| Cg68–Pg1–Ps221 | Cg68–Pg1–Ps222 | Cg68–Pg1–Ps223 | Cg68–Pg1–Ps224 |
| Cg68–Pg1–Ps225 | Cg68–Pg1–Ps226 | Cg68–Pg1–Ps227 | Cg68–Pg1–Ps228 |
| Cg68–Pg1–Ps229 | Cg68–Pg1–Ps230 | Cg68–Pg1–Ps231 | Cg68–Pg1–Ps232 |
| Cg68–Pg1–Ps233 | Cg68–Pg1–Ps234 | Cg68–Pg1–Ps235 | Cg68–Pg1–Ps236 |
| Cg68–Pg1–Ps237 | Cg68–Pg1–Ps238 | Cg68–Pg1–Ps239 | Cg68–Pg1–Ps240 |
| Cg68–Pg1–Ps241 | Cg68–Pg1–Ps242 | Cg68–Pg1–Ps243 | |

| | | | |
|---|---|---|---|
| Cg69–Pg1–Ps1 | Cg69–Pg1–Ps2 | Cg69–Pg1–Ps3 | Cg69–Pg1–Ps4 |
| Cg69–Pg1–Ps5 | Cg69–Pg1–Ps6 | Cg69–Pg1–Ps7 | Cg69–Pg1–Ps8 |
| Cg69–Pg1–Ps9 | Cg69–Pg1–Ps10 | Cg69–Pg1–Ps11 | Cg69–Pg1–Ps12 |
| Cg69–Pg1–Ps13 | Cg69–Pg1–Ps14 | Cg69–Pg1–Ps15 | Cg69–Pg1–Ps16 |
| Cg69–Pg1–Ps17 | Cg69–Pg1–Ps18 | Cg69–Pg1–Ps19 | Cg69–Pg1–Ps20 |
| Cg69–Pg1–Ps21 | Cg69–Pg1–Ps22 | Cg69–Pg1–Ps23 | Cg69–Pg1–Ps24 |
| Cg69–Pg1–Ps25 | Cg69–Pg1–Ps26 | Cg69–Pg1–Ps27 | Cg69–Pg1–Ps28 |
| Cg69–Pg1–Ps29 | Cg69–Pg1–Ps30 | Cg69–Pg1–Ps31 | Cg69–Pg1–Ps32 |
| Cg69–Pg1–Ps33 | Cg69–Pg1–Ps34 | Cg69–Pg1–Ps35 | Cg69–Pg1–Ps36 |
| Cg69–Pg1–Ps37 | Cg69–Pg1–Ps38 | Cg69–Pg1–Ps39 | Cg69–Pg1–Ps40 |
| Cg69–Pg1–Ps41 | Cg69–Pg1–Ps42 | Cg69–Pg1–Ps43 | Cg69–Pg1–Ps44 |
| Cg69–Pg1–Ps45 | Cg69–Pg1–Ps46 | Cg69–Pg1–Ps47 | Cg69–Pg1–Ps48 |
| Cg69–Pg1–Ps49 | Cg69–Pg1–Ps50 | Cg69–Pg1–Ps51 | Cg69–Pg1–Ps52 |
| Cg69–Pg1–Ps53 | Cg69–Pg1–Ps54 | Cg69–Pg1–Ps55 | Cg69–Pg1–Ps56 |
| Cg69–Pg1–Ps57 | Cg69–Pg1–Ps58 | Cg69–Pg1–Ps59 | Cg69–Pg1–Ps60 |
| Cg69–Pg1–Ps61 | Cg69–Pg1–Ps62 | Cg69–Pg1–Ps63 | Cg69–Pg1–Ps64 |
| Cg69–Pg1–Ps65 | Cg69–Pg1–Ps66 | Cg69–Pg1–Ps67 | Cg69–Pg1–Ps68 |
| Cg69–Pg1–Ps69 | Cg69–Pg1–Ps70 | Cg69–Pg1–Ps71 | Cg69–Pg1–Ps72 |
| Cg69–Pg1–Ps73 | Cg69–Pg1–Ps74 | Cg69–Pg1–Ps75 | Cg69–Pg1–Ps76 |
| Cg69–Pg1–Ps77 | Cg69–Pg1–Ps78 | Cg69–Pg1–Ps79 | Cg69–Pg1–Ps80 |
| Cg69–Pg1–Ps81 | Cg69–Pg1–Ps82 | Cg69–Pg1–Ps83 | Cg69–Pg1–Ps84 |
| Cg69–Pg1–Ps85 | Cg69–Pg1–Ps86 | Cg69–Pg1–Ps87 | Cg69–Pg1–Ps88 |
| Cg69–Pg1–Ps89 | Cg69–Pg1–Ps90 | Cg69–Pg1–Ps91 | Cg69–Pg1–Ps92 |
| Cg69–Pg1–Ps93 | Cg69–Pg1–Ps94 | Cg69–Pg1–Ps95 | Cg69–Pg1–Ps96 |
| Cg69–Pg1–Ps97 | Cg69–Pg1–Ps98 | Cg69–Pg1–Ps99 | Cg69–Pg1–Ps100 |

| | | | |
|---|---|---|---|
| Cg69-Pg1-Ps101 | Cg69-Pg1-Ps102 | Cg69-Pg1-Ps103 | Cg69-Pg1-Ps104 |
| Cg69-Pg1-Ps105 | Cg69-Pg1-Ps106 | Cg69-Pg1-Ps107 | Cg69-Pg1-Ps108 |
| Cg69-Pg1-Ps109 | Cg69-Pg1-Ps110 | Cg69-Pg1-Ps111 | Cg69-Pg1-Ps112 |
| Cg69-Pg1-Ps113 | Cg69-Pg1-Ps114 | Cg69-Pg1-Ps115 | Cg69-Pg1-Ps116 |
| Cg69-Pg1-Ps117 | Cg69-Pg1-Ps118 | Cg69-Pg1-Ps119 | Cg69-Pg1-Ps120 |
| Cg69-Pg1-Ps121 | Cg69-Pg1-Ps122 | Cg69-Pg1-Ps123 | Cg69-Pg1-Ps124 |
| Cg69-Pg1-Ps125 | Cg69-Pg1-Ps126 | Cg69-Pg1-Ps127 | Cg69-Pg1-Ps128 |
| Cg69-Pg1-Ps129 | Cg69-Pg1-Ps130 | Cg69-Pg1-Ps131 | Cg69-Pg1-Ps132 |
| Cg69-Pg1-Ps133 | Cg69-Pg1-Ps134 | Cg69-Pg1-Ps135 | Cg69-Pg1-Ps136 |
| Cg69-Pg1-Ps137 | Cg69-Pg1-Ps138 | Cg69-Pg1-Ps139 | Cg69-Pg1-Ps140 |
| Cg69-Pg1-Ps141 | Cg69-Pg1-Ps142 | Cg69-Pg1-Ps143 | Cg69-Pg1-Ps144 |
| Cg69-Pg1-Ps145 | Cg69-Pg1-Ps146 | Cg69-Pg1-Ps147 | Cg69-Pg1-Ps148 |
| Cg69-Pg1-Ps149 | Cg69-Pg1-Ps150 | Cg69-Pg1-Ps151 | Cg69-Pg1-Ps152 |
| Cg69-Pg1-Ps153 | Cg69-Pg1-Ps154 | Cg69-Pg1-Ps155 | Cg69-Pg1-Ps156 |
| Cg69-Pg1-Ps157 | Cg69-Pg1-Ps158 | Cg69-Pg1-Ps159 | Cg69-Pg1-Ps160 |
| Cg69-Pg1-Ps161 | Cg69-Pg1-Ps162 | Cg69-Pg1-Ps163 | Cg69-Pg1-Ps164 |
| Cg69-Pg1-Ps165 | Cg69-Pg1-Ps166 | Cg69-Pg1-Ps167 | Cg69-Pg1-Ps168 |
| Cg69-Pg1-Ps169 | Cg69-Pg1-Ps170 | Cg69-Pg1-Ps171 | Cg69-Pg1-Ps172 |
| Cg69-Pg1-Ps173 | Cg69-Pg1-Ps174 | Cg69-Pg1-Ps175 | Cg69-Pg1-Ps176 |
| Cg69-Pg1-Ps177 | Cg69-Pg1-Ps178 | Cg69-Pg1-Ps179 | Cg69-Pg1-Ps180 |
| Cg69-Pg1-Ps181 | Cg69-Pg1-Ps182 | Cg69-Pg1-Ps183 | Cg69-Pg1-Ps184 |
| Cg69-Pg1-Ps185 | Cg69-Pg1-Ps186 | Cg69-Pg1-Ps187 | Cg69-Pg1-Ps188 |
| Cg69-Pg1-Ps189 | Cg69-Pg1-Ps190 | Cg69-Pg1-Ps191 | Cg69-Pg1-Ps192 |
| Cg69-Pg1-Ps193 | Cg69-Pg1-Ps194 | Cg69-Pg1-Ps195 | Cg69-Pg1-Ps196 |
| Cg69-Pg1-Ps197 | Cg69-Pg1-Ps198 | Cg69-Pg1-Ps199 | Cg69-Pg1-Ps200 |
| Cg69-Pg1-Ps201 | Cg69-Pg1-Ps202 | Cg69-Pg1-Ps203 | Cg69-Pg1-Ps204 |
| Cg69-Pg1-Ps205 | Cg69-Pg1-Ps206 | Cg69-Pg1-Ps207 | Cg69-Pg1-Ps208 |
| Cg69-Pg1-Ps209 | Cg69-Pg1-Ps210 | Cg69-Pg1-Ps211 | Cg69-Pg1-Ps212 |
| Cg69-Pg1-Ps213 | Cg69-Pg1-Ps214 | Cg69-Pg1-Ps215 | Cg69-Pg1-Ps216 |
| Cg69-Pg1-Ps217 | Cg69-Pg1-Ps218 | Cg69-Pg1-Ps219 | Cg69-Pg1-Ps220 |
| Cg69-Pg1-Ps221 | Cg69-Pg1-Ps222 | Cg69-Pg1-Ps223 | Cg69-Pg1-Ps224 |
| Cg69-Pg1-Ps225 | Cg69-Pg1-Ps226 | Cg69-Pg1-Ps227 | Cg69-Pg1-Ps228 |
| Cg69-Pg1-Ps229 | Cg69-Pg1-Ps230 | Cg69-Pg1-Ps231 | Cg69-Pg1-Ps232 |
| Cg69-Pg1-Ps233 | Cg69-Pg1-Ps234 | Cg69-Pg1-Ps235 | Cg69-Pg1-Ps236 |
| Cg69-Pg1-Ps237 | Cg69-Pg1-Ps238 | Cg69-Pg1-Ps239 | Cg69-Pg1-Ps240 |
| Cg69-Pg1-Ps241 | Cg69-Pg1-Ps242 | Cg69-Pg1-Ps243 | |

| | | | |
|---|---|---|---|
| Cg70-Pg1-Ps1 | Cg70-Pg1-Ps2 | Cg70-Pg1-Ps3 | Cg70-Pg1-Ps4 |
| Cg70-Pg1-Ps5 | Cg70-Pg1-Ps6 | Cg70-Pg1-Ps7 | Cg70-Pg1-Ps8 |
| Cg70-Pg1-Ps9 | Cg70-Pg1-Ps10 | Cg70-Pg1-Ps11 | Cg70-Pg1-Ps12 |
| Cg70-Pg1-Ps13 | Cg70-Pg1-Ps14 | Cg70-Pg1-Ps15 | Cg70-Pg1-Ps16 |
| Cg70-Pg1-Ps17 | Cg70-Pg1-Ps18 | Cg70-Pg1-Ps19 | Cg70-Pg1-Ps20 |
| Cg70-Pg1-Ps21 | Cg70-Pg1-Ps22 | Cg70-Pg1-Ps23 | Cg70-Pg1-Ps24 |
| Cg70-Pg1-Ps25 | Cg70-Pg1-Ps26 | Cg70-Pg1-Ps27 | Cg70-Pg1-Ps28 |
| Cg70-Pg1-Ps29 | Cg70-Pg1-Ps30 | Cg70-Pg1-Ps31 | Cg70-Pg1-Ps32 |
| Cg70-Pg1-Ps33 | Cg70-Pg1-Ps34 | Cg70-Pg1-Ps35 | Cg70-Pg1-Ps36 |
| Cg70-Pg1-Ps37 | Cg70-Pg1-Ps38 | Cg70-Pg1-Ps39 | Cg70-Pg1-Ps40 |
| Cg70-Pg1-Ps41 | Cg70-Pg1-Ps42 | Cg70-Pg1-Ps43 | Cg70-Pg1-Ps44 |
| Cg70-Pg1-Ps45 | Cg70-Pg1-Ps46 | Cg70-Pg1-Ps47 | Cg70-Pg1-Ps48 |
| Cg70-Pg1-Ps49 | Cg70-Pg1-Ps50 | Cg70-Pg1-Ps51 | Cg70-Pg1-Ps52 |
| Cg70-Pg1-Ps53 | Cg70-Pg1-Ps54 | Cg70-Pg1-Ps55 | Cg70-Pg1-Ps56 |
| Cg70-Pg1-Ps57 | Cg70-Pg1-Ps58 | Cg70-Pg1-Ps59 | Cg70-Pg1-Ps60 |

| | | | |
|---|---|---|---|
| Cg70–Pg1–Ps61 | Cg70–Pg1–Ps62 | Cg70–Pg1–Ps63 | Cg70–Pg1–Ps64 |
| Cg70–Pg1–Ps65 | Cg70–Pg1–Ps66 | Cg70–Pg1–Ps67 | Cg70–Pg1–Ps68 |
| Cg70–Pg1–Ps69 | Cg70–Pg1–Ps70 | Cg70–Pg1–Ps71 | Cg70–Pg1–Ps72 |
| Cg70–Pg1–Ps73 | Cg70–Pg1–Ps74 | Cg70–Pg1–Ps75 | Cg70–Pg1–Ps76 |
| Cg70–Pg1–Ps77 | Cg70–Pg1–Ps78 | Cg70–Pg1–Ps79 | Cg70–Pg1–Ps80 |
| Cg70–Pg1–Ps81 | Cg70–Pg1–Ps82 | Cg70–Pg1–Ps83 | Cg70–Pg1–Ps84 |
| Cg70–Pg1–Ps85 | Cg70–Pg1–Ps86 | Cg70–Pg1–Ps87 | Cg70–Pg1–Ps88 |
| Cg70–Pg1–Ps89 | Cg70–Pg1–Ps90 | Cg70–Pg1–Ps91 | Cg70–Pg1–Ps92 |
| Cg70–Pg1–Ps93 | Cg70–Pg1–Ps94 | Cg70–Pg1–Ps95 | Cg70–Pg1–Ps96 |
| Cg70–Pg1–Ps97 | Cg70–Pg1–Ps98 | Cg70–Pg1–Ps99 | Cg70–Pg1–Ps100 |
| Cg70–Pg1–Ps101 | Cg70–Pg1–Ps102 | Cg70–Pg1–Ps103 | Cg70–Pg1–Ps104 |
| Cg70–Pg1–Ps105 | Cg70–Pg1–Ps106 | Cg70–Pg1–Ps107 | Cg70–Pg1–Ps108 |
| Cg70–Pg1–Ps109 | Cg70–Pg1–Ps110 | Cg70–Pg1–Ps111 | Cg70–Pg1–Ps112 |
| Cg70–Pg1–Ps113 | Cg70–Pg1–Ps114 | Cg70–Pg1–Ps115 | Cg70–Pg1–Ps116 |
| Cg70–Pg1–Ps117 | Cg70–Pg1–Ps118 | Cg70–Pg1–Ps119 | Cg70–Pg1–Ps120 |
| Cg70–Pg1–Ps121 | Cg70–Pg1–Ps122 | Cg70–Pg1–Ps123 | Cg70–Pg1–Ps124 |
| Cg70–Pg1–Ps125 | Cg70–Pg1–Ps126 | Cg70–Pg1–Ps127 | Cg70–Pg1–Ps128 |
| Cg70–Pg1–Ps129 | Cg70–Pg1–Ps130 | Cg70–Pg1–Ps131 | Cg70–Pg1–Ps132 |
| Cg70–Pg1–Ps133 | Cg70–Pg1–Ps134 | Cg70–Pg1–Ps135 | Cg70–Pg1–Ps136 |
| Cg70–Pg1–Ps137 | Cg70–Pg1–Ps138 | Cg70–Pg1–Ps139 | Cg70–Pg1–Ps140 |
| Cg70–Pg1–Ps141 | Cg70–Pg1–Ps142 | Cg70–Pg1–Ps143 | Cg70–Pg1–Ps144 |
| Cg70–Pg1–Ps145 | Cg70–Pg1–Ps146 | Cg70–Pg1–Ps147 | Cg70–Pg1–Ps148 |
| Cg70–Pg1–Ps149 | Cg70–Pg1–Ps150 | Cg70–Pg1–Ps151 | Cg70–Pg1–Ps152 |
| Cg70–Pg1–Ps153 | Cg70–Pg1–Ps154 | Cg70–Pg1–Ps155 | Cg70–Pg1–Ps156 |
| Cg70–Pg1–Ps157 | Cg70–Pg1–Ps158 | Cg70–Pg1–Ps159 | Cg70–Pg1–Ps160 |
| Cg70–Pg1–Ps161 | Cg70–Pg1–Ps162 | Cg70–Pg1–Ps163 | Cg70–Pg1–Ps164 |
| Cg70–Pg1–Ps165 | Cg70–Pg1–Ps166 | Cg70–Pg1–Ps167 | Cg70–Pg1–Ps168 |
| Cg70–Pg1–Ps169 | Cg70–Pg1–Ps170 | Cg70–Pg1–Ps171 | Cg70–Pg1–Ps172 |
| Cg70–Pg1–Ps173 | Cg70–Pg1–Ps174 | Cg70–Pg1–Ps175 | Cg70–Pg1–Ps176 |
| Cg70–Pg1–Ps177 | Cg70–Pg1–Ps178 | Cg70–Pg1–Ps179 | Cg70–Pg1–Ps180 |
| Cg70–Pg1–Ps181 | Cg70–Pg1–Ps182 | Cg70–Pg1–Ps183 | Cg70–Pg1–Ps184 |
| Cg70–Pg1–Ps185 | Cg70–Pg1–Ps186 | Cg70–Pg1–Ps187 | Cg70–Pg1–Ps188 |
| Cg70–Pg1–Ps189 | Cg70–Pg1–Ps190 | Cg70–Pg1–Ps191 | Cg70–Pg1–Ps192 |
| Cg70–Pg1–Ps193 | Cg70–Pg1–Ps194 | Cg70–Pg1–Ps195 | Cg70–Pg1–Ps196 |
| Cg70–Pg1–Ps197 | Cg70–Pg1–Ps198 | Cg70–Pg1–Ps199 | Cg70–Pg1–Ps200 |
| Cg70–Pg1–Ps201 | Cg70–Pg1–Ps202 | Cg70–Pg1–Ps203 | Cg70–Pg1–Ps204 |
| Cg70–Pg1–Ps205 | Cg70–Pg1–Ps206 | Cg70–Pg1–Ps207 | Cg70–Pg1–Ps208 |
| Cg70–Pg1–Ps209 | Cg70–Pg1–Ps210 | Cg70–Pg1–Ps211 | Cg70–Pg1–Ps212 |
| Cg70–Pg1–Ps213 | Cg70–Pg1–Ps214 | Cg70–Pg1–Ps215 | Cg70–Pg1–Ps216 |
| Cg70–Pg1–Ps217 | Cg70–Pg1–Ps218 | Cg70–Pg1–Ps219 | Cg70–Pg1–Ps220 |
| Cg70–Pg1–Ps221 | Cg70–Pg1–Ps222 | Cg70–Pg1–Ps223 | Cg70–Pg1–Ps224 |
| Cg70–Pg1–Ps225 | Cg70–Pg1–Ps226 | Cg70–Pg1–Ps227 | Cg70–Pg1–Ps228 |
| Cg70–Pg1–Ps229 | Cg70–Pg1–Ps230 | Cg70–Pg1–Ps231 | Cg70–Pg1–Ps232 |
| Cg70–Pg1–Ps233 | Cg70–Pg1–Ps234 | Cg70–Pg1–Ps235 | Cg70–Pg1–Ps236 |
| Cg70–Pg1–Ps237 | Cg70–Pg1–Ps238 | Cg70–Pg1–Ps239 | Cg70–Pg1–Ps240 |
| Cg70–Pg1–Ps241 | Cg70–Pg1–Ps242 | Cg70–Pg1–Ps243 | |

| | | | |
|---|---|---|---|
| Cg73–Pg1–Ps1 | Cg73–Pg1–Ps2 | Cg73–Pg1–Ps3 | Cg73–Pg1–Ps4 |
| Cg73–Pg1–Ps5 | Cg73–Pg1–Ps6 | Cg73–Pg1–Ps7 | Cg73–Pg1–Ps8 |
| Cg73–Pg1–Ps9 | Cg73–Pg1–Ps10 | Cg73–Pg1–Ps11 | Cg73–Pg1–Ps12 |
| Cg73–Pg1–Ps13 | Cg73–Pg1–Ps14 | Cg73–Pg1–Ps15 | Cg73–Pg1–Ps16 |
| Cg73–Pg1–Ps17 | Cg73–Pg1–Ps18 | Cg73–Pg1–Ps19 | Cg73–Pg1–Ps20 |

| | | | |
|---|---|---|---|
| Cg73–Pg1–Ps21 | Cg73–Pg1–Ps22 | Cg73–Pg1–Ps23 | Cg73–Pg1–Ps24 |
| Cg73–Pg1–Ps25 | Cg73–Pg1–Ps26 | Cg73–Pg1–Ps27 | Cg73–Pg1–Ps28 |
| Cg73–Pg1–Ps29 | Cg73–Pg1–Ps30 | Cg73–Pg1–Ps31 | Cg73–Pg1–Ps32 |
| Cg73–Pg1–Ps33 | Cg73–Pg1–Ps34 | Cg73–Pg1–Ps35 | Cg73–Pg1–Ps36 |
| Cg73–Pg1–Ps37 | Cg73–Pg1–Ps38 | Cg73–Pg1–Ps39 | Cg73–Pg1–Ps40 |
| Cg73–Pg1–Ps41 | Cg73–Pg1–Ps42 | Cg73–Pg1–Ps43 | Cg73–Pg1–Ps44 |
| Cg73–Pg1–Ps45 | Cg73–Pg1–Ps46 | Cg73–Pg1–Ps47 | Cg73–Pg1–Ps48 |
| Cg73–Pg1–Ps49 | Cg73–Pg1–Ps50 | Cg73–Pg1–Ps51 | Cg73–Pg1–Ps52 |
| Cg73–Pg1–Ps53 | Cg73–Pg1–Ps54 | Cg73–Pg1–Ps55 | Cg73–Pg1–Ps56 |
| Cg73–Pg1–Ps57 | Cg73–Pg1–Ps58 | Cg73–Pg1–Ps59 | Cg73–Pg1–Ps60 |
| Cg73–Pg1–Ps61 | Cg73–Pg1–Ps62 | Cg73–Pg1–Ps63 | Cg73–Pg1–Ps64 |
| Cg73–Pg1–Ps65 | Cg73–Pg1–Ps66 | Cg73–Pg1–Ps67 | Cg73–Pg1–Ps68 |
| Cg73–Pg1–Ps69 | Cg73–Pg1–Ps70 | Cg73–Pg1–Ps71 | Cg73–Pg1–Ps72 |
| Cg73–Pg1–Ps73 | Cg73–Pg1–Ps74 | Cg73–Pg1–Ps75 | Cg73–Pg1–Ps76 |
| Cg73–Pg1–Ps77 | Cg73–Pg1–Ps78 | Cg73–Pg1–Ps79 | Cg73–Pg1–Ps80 |
| Cg73–Pg1–Ps81 | Cg73–Pg1–Ps82 | Cg73–Pg1–Ps83 | Cg73–Pg1–Ps84 |
| Cg73–Pg1–Ps85 | Cg73–Pg1–Ps86 | Cg73–Pg1–Ps87 | Cg73–Pg1–Ps88 |
| Cg73–Pg1–Ps89 | Cg73–Pg1–Ps90 | Cg73–Pg1–Ps91 | Cg73–Pg1–Ps92 |
| Cg73–Pg1–Ps93 | Cg73–Pg1–Ps94 | Cg73–Pg1–Ps95 | Cg73–Pg1–Ps96 |
| Cg73–Pg1–Ps97 | Cg73–Pg1–Ps98 | Cg73–Pg1–Ps99 | Cg73–Pg1–Ps100 |
| Cg73–Pg1–Ps101 | Cg73–Pg1–Ps102 | Cg73–Pg1–Ps103 | Cg73–Pg1–Ps104 |
| Cg73–Pg1–Ps105 | Cg73–Pg1–Ps106 | Cg73–Pg1–Ps107 | Cg73–Pg1–Ps108 |
| Cg73–Pg1–Ps109 | Cg73–Pg1–Ps110 | Cg73–Pg1–Ps111 | Cg73–Pg1–Ps112 |
| Cg73–Pg1–Ps113 | Cg73–Pg1–Ps114 | Cg73–Pg1–Ps115 | Cg73–Pg1–Ps116 |
| Cg73–Pg1–Ps117 | Cg73–Pg1–Ps118 | Cg73–Pg1–Ps119 | Cg73–Pg1–Ps120 |
| Cg73–Pg1–Ps121 | Cg73–Pg1–Ps122 | Cg73–Pg1–Ps123 | Cg73–Pg1–Ps124 |
| Cg73–Pg1–Ps125 | Cg73–Pg1–Ps126 | Cg73–Pg1–Ps127 | Cg73–Pg1–Ps128 |
| Cg73–Pg1–Ps129 | Cg73–Pg1–Ps130 | Cg73–Pg1–Ps131 | Cg73–Pg1–Ps132 |
| Cg73–Pg1–Ps133 | Cg73–Pg1–Ps134 | Cg73–Pg1–Ps135 | Cg73–Pg1–Ps136 |
| Cg73–Pg1–Ps137 | Cg73–Pg1–Ps138 | Cg73–Pg1–Ps139 | Cg73–Pg1–Ps140 |
| Cg73–Pg1–Ps141 | Cg73–Pg1–Ps142 | Cg73–Pg1–Ps143 | Cg73–Pg1–Ps144 |
| Cg73–Pg1–Ps145 | Cg73–Pg1–Ps146 | Cg73–Pg1–Ps147 | Cg73–Pg1–Ps148 |
| Cg73–Pg1–Ps149 | Cg73–Pg1–Ps150 | Cg73–Pg1–Ps151 | Cg73–Pg1–Ps152 |
| Cg73–Pg1–Ps153 | Cg73–Pg1–Ps154 | Cg73–Pg1–Ps155 | Cg73–Pg1–Ps156 |
| Cg73–Pg1–Ps157 | Cg73–Pg1–Ps158 | Cg73–Pg1–Ps159 | Cg73–Pg1–Ps160 |
| Cg73–Pg1–Ps161 | Cg73–Pg1–Ps162 | Cg73–Pg1–Ps163 | Cg73–Pg1–Ps164 |
| Cg73–Pg1–Ps165 | Cg73–Pg1–Ps166 | Cg73–Pg1–Ps167 | Cg73–Pg1–Ps168 |
| Cg73–Pg1–Ps169 | Cg73–Pg1–Ps170 | Cg73–Pg1–Ps171 | Cg73–Pg1–Ps172 |
| Cg73–Pg1–Ps173 | Cg73–Pg1–Ps174 | Cg73–Pg1–Ps175 | Cg73–Pg1–Ps176 |
| Cg73–Pg1–Ps177 | Cg73–Pg1–Ps178 | Cg73–Pg1–Ps179 | Cg73–Pg1–Ps180 |
| Cg73–Pg1–Ps181 | Cg73–Pg1–Ps182 | Cg73–Pg1–Ps183 | Cg73–Pg1–Ps184 |
| Cg73–Pg1–Ps185 | Cg73–Pg1–Ps186 | Cg73–Pg1–Ps187 | Cg73–Pg1–Ps188 |
| Cg73–Pg1–Ps189 | Cg73–Pg1–Ps190 | Cg73–Pg1–Ps191 | Cg73–Pg1–Ps192 |
| Cg73–Pg1–Ps193 | Cg73–Pg1–Ps194 | Cg73–Pg1–Ps195 | Cg73–Pg1–Ps196 |
| Cg73–Pg1–Ps197 | Cg73–Pg1–Ps198 | Cg73–Pg1–Ps199 | Cg73–Pg1–Ps200 |
| Cg73–Pg1–Ps201 | Cg73–Pg1–Ps202 | Cg73–Pg1–Ps203 | Cg73–Pg1–Ps204 |
| Cg73–Pg1–Ps205 | Cg73–Pg1–Ps206 | Cg73–Pg1–Ps207 | Cg73–Pg1–Ps208 |
| Cg73–Pg1–Ps209 | Cg73–Pg1–Ps210 | Cg73–Pg1–Ps211 | Cg73–Pg1–Ps212 |
| Cg73–Pg1–Ps213 | Cg73–Pg1–Ps214 | Cg73–Pg1–Ps215 | Cg73–Pg1–Ps216 |
| Cg73–Pg1–Ps217 | Cg73–Pg1–Ps218 | Cg73–Pg1–Ps219 | Cg73–Pg1–Ps220 |
| Cg73–Pg1–Ps221 | Cg73–Pg1–Ps222 | Cg73–Pg1–Ps223 | Cg73–Pg1–Ps224 |
| Cg73–Pg1–Ps225 | Cg73–Pg1–Ps226 | Cg73–Pg1–Ps227 | Cg73–Pg1–Ps228 |

| | | | |
|---|---|---|---|
| Cg73–Pg1–Ps229 | Cg73–Pg1–Ps230 | Cg73–Pg1–Ps231 | Cg73–Pg1–Ps232 |
| Cg73–Pg1–Ps233 | Cg73–Pg1–Ps234 | Cg73–Pg1–Ps235 | Cg73–Pg1–Ps236 |
| Cg73–Pg1–Ps237 | Cg73–Pg1–Ps238 | Cg73–Pg1–Ps239 | Cg73–Pg1–Ps240 |
| Cg73–Pg1–Ps241 | Cg73–Pg1–Ps242 | Cg73–Pg1–Ps243 | |

| | | | |
|---|---|---|---|
| Cg74–Pg1–Ps1 | Cg74–Pg1–Ps2 | Cg74–Pg1–Ps3 | Cg74–Pg1–Ps4 |
| Cg74–Pg1–Ps5 | Cg74–Pg1–Ps6 | Cg74–Pg1–Ps7 | Cg74–Pg1–Ps8 |
| Cg74–Pg1–Ps9 | Cg74–Pg1–Ps10 | Cg74–Pg1–Ps11 | Cg74–Pg1–Ps12 |
| Cg74–Pg1–Ps13 | Cg74–Pg1–Ps14 | Cg74–Pg1–Ps15 | Cg74–Pg1–Ps16 |
| Cg74–Pg1–Ps17 | Cg74–Pg1–Ps18 | Cg74–Pg1–Ps19 | Cg74–Pg1–Ps20 |
| Cg74–Pg1–Ps21 | Cg74–Pg1–Ps22 | Cg74–Pg1–Ps23 | Cg74–Pg1–Ps24 |
| Cg74–Pg1–Ps25 | Cg74–Pg1–Ps26 | Cg74–Pg1–Ps27 | Cg74–Pg1–Ps28 |
| Cg74–Pg1–Ps29 | Cg74–Pg1–Ps30 | Cg74–Pg1–Ps31 | Cg74–Pg1–Ps32 |
| Cg74–Pg1–Ps33 | Cg74–Pg1–Ps34 | Cg74–Pg1–Ps35 | Cg74–Pg1–Ps36 |
| Cg74–Pg1–Ps37 | Cg74–Pg1–Ps38 | Cg74–Pg1–Ps39 | Cg74–Pg1–Ps40 |
| Cg74–Pg1–Ps41 | Cg74–Pg1–Ps42 | Cg74–Pg1–Ps43 | Cg74–Pg1–Ps44 |
| Cg74–Pg1–Ps45 | Cg74–Pg1–Ps46 | Cg74–Pg1–Ps47 | Cg74–Pg1–Ps48 |
| Cg74–Pg1–Ps49 | Cg74–Pg1–Ps50 | Cg74–Pg1–Ps51 | Cg74–Pg1–Ps52 |
| Cg74–Pg1–Ps53 | Cg74–Pg1–Ps54 | Cg74–Pg1–Ps55 | Cg74–Pg1–Ps56 |
| Cg74–Pg1–Ps57 | Cg74–Pg1–Ps58 | Cg74–Pg1–Ps59 | Cg74–Pg1–Ps60 |
| Cg74–Pg1–Ps61 | Cg74–Pg1–Ps62 | Cg74–Pg1–Ps63 | Cg74–Pg1–Ps64 |
| Cg74–Pg1–Ps65 | Cg74–Pg1–Ps66 | Cg74–Pg1–Ps67 | Cg74–Pg1–Ps68 |
| Cg74–Pg1–Ps69 | Cg74–Pg1–Ps70 | Cg74–Pg1–Ps71 | Cg74–Pg1–Ps72 |
| Cg74–Pg1–Ps73 | Cg74–Pg1–Ps74 | Cg74–Pg1–Ps75 | Cg74–Pg1–Ps76 |
| Cg74–Pg1–Ps77 | Cg74–Pg1–Ps78 | Cg74–Pg1–Ps79 | Cg74–Pg1–Ps80 |
| Cg74–Pg1–Ps81 | Cg74–Pg1–Ps82 | Cg74–Pg1–Ps83 | Cg74–Pg1–Ps84 |
| Cg74–Pg1–Ps85 | Cg74–Pg1–Ps86 | Cg74–Pg1–Ps87 | Cg74–Pg1–Ps88 |
| Cg74–Pg1–Ps89 | Cg74–Pg1–Ps90 | Cg74–Pg1–Ps91 | Cg74–Pg1–Ps92 |
| Cg74–Pg1–Ps93 | Cg74–Pg1–Ps94 | Cg74–Pg1–Ps95 | Cg74–Pg1–Ps96 |
| Cg74–Pg1–Ps97 | Cg74–Pg1–Ps98 | Cg74–Pg1–Ps99 | Cg74–Pg1–Ps100 |
| Cg74–Pg1–Ps101 | Cg74–Pg1–Ps102 | Cg74–Pg1–Ps103 | Cg74–Pg1–Ps104 |
| Cg74–Pg1–Ps105 | Cg74–Pg1–Ps106 | Cg74–Pg1–Ps107 | Cg74–Pg1–Ps108 |
| Cg74–Pg1–Ps109 | Cg74–Pg1–Ps110 | Cg74–Pg1–Ps111 | Cg74–Pg1–Ps112 |
| Cg74–Pg1–Ps113 | Cg74–Pg1–Ps114 | Cg74–Pg1–Ps115 | Cg74–Pg1–Ps116 |
| Cg74–Pg1–Ps117 | Cg74–Pg1–Ps118 | Cg74–Pg1–Ps119 | Cg74–Pg1–Ps120 |
| Cg74–Pg1–Ps121 | Cg74–Pg1–Ps122 | Cg74–Pg1–Ps123 | Cg74–Pg1–Ps124 |
| Cg74–Pg1–Ps125 | Cg74–Pg1–Ps126 | Cg74–Pg1–Ps127 | Cg74–Pg1–Ps128 |
| Cg74–Pg1–Ps129 | Cg74–Pg1–Ps130 | Cg74–Pg1–Ps131 | Cg74–Pg1–Ps132 |
| Cg74–Pg1–Ps133 | Cg74–Pg1–Ps134 | Cg74–Pg1–Ps135 | Cg74–Pg1–Ps136 |
| Cg74–Pg1–Ps137 | Cg74–Pg1–Ps138 | Cg74–Pg1–Ps139 | Cg74–Pg1–Ps140 |
| Cg74–Pg1–Ps141 | Cg74–Pg1–Ps142 | Cg74–Pg1–Ps143 | Cg74–Pg1–Ps144 |
| Cg74–Pg1–Ps145 | Cg74–Pg1–Ps146 | Cg74–Pg1–Ps147 | Cg74–Pg1–Ps148 |
| Cg74–Pg1–Ps149 | Cg74–Pg1–Ps150 | Cg74–Pg1–Ps151 | Cg74–Pg1–Ps152 |
| Cg74–Pg1–Ps153 | Cg74–Pg1–Ps154 | Cg74–Pg1–Ps155 | Cg74–Pg1–Ps156 |
| Cg74–Pg1–Ps157 | Cg74–Pg1–Ps158 | Cg74–Pg1–Ps159 | Cg74–Pg1–Ps160 |
| Cg74–Pg1–Ps161 | Cg74–Pg1–Ps162 | Cg74–Pg1–Ps163 | Cg74–Pg1–Ps164 |
| Cg74–Pg1–Ps165 | Cg74–Pg1–Ps166 | Cg74–Pg1–Ps167 | Cg74–Pg1–Ps168 |
| Cg74–Pg1–Ps169 | Cg74–Pg1–Ps170 | Cg74–Pg1–Ps171 | Cg74–Pg1–Ps172 |
| Cg74–Pg1–Ps173 | Cg74–Pg1–Ps174 | Cg74–Pg1–Ps175 | Cg74–Pg1–Ps176 |
| Cg74–Pg1–Ps177 | Cg74–Pg1–Ps178 | Cg74–Pg1–Ps179 | Cg74–Pg1–Ps180 |
| Cg74–Pg1–Ps181 | Cg74–Pg1–Ps182 | Cg74–Pg1–Ps183 | Cg74–Pg1–Ps184 |
| Cg74–Pg1–Ps185 | Cg74–Pg1–Ps186 | Cg74–Pg1–Ps187 | Cg74–Pg1–Ps188 |

| | | | |
|---|---|---|---|
| Cg74–Pg1–Ps189 | Cg74–Pg1–Ps190 | Cg74–Pg1–Ps191 | Cg74–Pg1–Ps192 |
| Cg74–Pg1–Ps193 | Cg74–Pg1–Ps194 | Cg74–Pg1–Ps195 | Cg74–Pg1–Ps196 |
| Cg74–Pg1–Ps197 | Cg74–Pg1–Ps198 | Cg74–Pg1–Ps199 | Cg74–Pg1–Ps200 |
| Cg74–Pg1–Ps201 | Cg74–Pg1–Ps202 | Cg74–Pg1–Ps203 | Cg74–Pg1–Ps204 |
| Cg74–Pg1–Ps205 | Cg74–Pg1–Ps206 | Cg74–Pg1–Ps207 | Cg74–Pg1–Ps208 |
| Cg74–Pg1–Ps209 | Cg74–Pg1–Ps210 | Cg74–Pg1–Ps211 | Cg74–Pg1–Ps212 |
| Cg74–Pg1–Ps213 | Cg74–Pg1–Ps214 | Cg74–Pg1–Ps215 | Cg74–Pg1–Ps216 |
| Cg74–Pg1–Ps217 | Cg74–Pg1–Ps218 | Cg74–Pg1–Ps219 | Cg74–Pg1–Ps220 |
| Cg74–Pg1–Ps221 | Cg74–Pg1–Ps222 | Cg74–Pg1–Ps223 | Cg74–Pg1–Ps224 |
| Cg74–Pg1–Ps225 | Cg74–Pg1–Ps226 | Cg74–Pg1–Ps227 | Cg74–Pg1–Ps228 |
| Cg74–Pg1–Ps229 | Cg74–Pg1–Ps230 | Cg74–Pg1–Ps231 | Cg74–Pg1–Ps232 |
| Cg74–Pg1–Ps233 | Cg74–Pg1–Ps234 | Cg74–Pg1–Ps235 | Cg74–Pg1–Ps236 |
| Cg74–Pg1–Ps237 | Cg74–Pg1–Ps238 | Cg74–Pg1–Ps239 | Cg74–Pg1–Ps240 |
| Cg74–Pg1–Ps241 | Cg74–Pg1–Ps242 | Cg74–Pg1–Ps243 | |

| | | | |
|---|---|---|---|
| Cg75–Pg1–Ps1 | Cg75–Pg1–Ps2 | Cg75–Pg1–Ps3 | Cg75–Pg1–Ps4 |
| Cg75–Pg1–Ps5 | Cg75–Pg1–Ps6 | Cg75–Pg1–Ps7 | Cg75–Pg1–Ps8 |
| Cg75–Pg1–Ps9 | Cg75–Pg1–Ps10 | Cg75–Pg1–Ps11 | Cg75–Pg1–Ps12 |
| Cg75–Pg1–Ps13 | Cg75–Pg1–Ps14 | Cg75–Pg1–Ps15 | Cg75–Pg1–Ps16 |
| Cg75–Pg1–Ps17 | Cg75–Pg1–Ps18 | Cg75–Pg1–Ps19 | Cg75–Pg1–Ps20 |
| Cg75–Pg1–Ps21 | Cg75–Pg1–Ps22 | Cg75–Pg1–Ps23 | Cg75–Pg1–Ps24 |
| Cg75–Pg1–Ps25 | Cg75–Pg1–Ps26 | Cg75–Pg1–Ps27 | Cg75–Pg1–Ps28 |
| Cg75–Pg1–Ps29 | Cg75–Pg1–Ps30 | Cg75–Pg1–Ps31 | Cg75–Pg1–Ps32 |
| Cg75–Pg1–Ps33 | Cg75–Pg1–Ps34 | Cg75–Pg1–Ps35 | Cg75–Pg1–Ps36 |
| Cg75–Pg1–Ps37 | Cg75–Pg1–Ps38 | Cg75–Pg1–Ps39 | Cg75–Pg1–Ps40 |
| Cg75–Pg1–Ps41 | Cg75–Pg1–Ps42 | Cg75–Pg1–Ps43 | Cg75–Pg1–Ps44 |
| Cg75–Pg1–Ps45 | Cg75–Pg1–Ps46 | Cg75–Pg1–Ps47 | Cg75–Pg1–Ps48 |
| Cg75–Pg1–Ps49 | Cg75–Pg1–Ps50 | Cg75–Pg1–Ps51 | Cg75–Pg1–Ps52 |
| Cg75–Pg1–Ps53 | Cg75–Pg1–Ps54 | Cg75–Pg1–Ps55 | Cg75–Pg1–Ps56 |
| Cg75–Pg1–Ps57 | Cg75–Pg1–Ps58 | Cg75–Pg1–Ps59 | Cg75–Pg1–Ps60 |
| Cg75–Pg1–Ps61 | Cg75–Pg1–Ps62 | Cg75–Pg1–Ps63 | Cg75–Pg1–Ps64 |
| Cg75–Pg1–Ps65 | Cg75–Pg1–Ps66 | Cg75–Pg1–Ps67 | Cg75–Pg1–Ps68 |
| Cg75–Pg1–Ps69 | Cg75–Pg1–Ps70 | Cg75–Pg1–Ps71 | Cg75–Pg1–Ps72 |
| Cg75–Pg1–Ps73 | Cg75–Pg1–Ps74 | Cg75–Pg1–Ps75 | Cg75–Pg1–Ps76 |
| Cg75–Pg1–Ps77 | Cg75–Pg1–Ps78 | Cg75–Pg1–Ps79 | Cg75–Pg1–Ps80 |
| Cg75–Pg1–Ps81 | Cg75–Pg1–Ps82 | Cg75–Pg1–Ps83 | Cg75–Pg1–Ps84 |
| Cg75–Pg1–Ps85 | Cg75–Pg1–Ps86 | Cg75–Pg1–Ps87 | Cg75–Pg1–Ps88 |
| Cg75–Pg1–Ps89 | Cg75–Pg1–Ps90 | Cg75–Pg1–Ps91 | Cg75–Pg1–Ps92 |
| Cg75–Pg1–Ps93 | Cg75–Pg1–Ps94 | Cg75–Pg1–Ps95 | Cg75–Pg1–Ps96 |
| Cg75–Pg1–Ps97 | Cg75–Pg1–Ps98 | Cg75–Pg1–Ps99 | Cg75–Pg1–Ps100 |
| Cg75–Pg1–Ps101 | Cg75–Pg1–Ps102 | Cg75–Pg1–Ps103 | Cg75–Pg1–Ps104 |
| Cg75–Pg1–Ps105 | Cg75–Pg1–Ps106 | Cg75–Pg1–Ps107 | Cg75–Pg1–Ps108 |
| Cg75–Pg1–Ps109 | Cg75–Pg1–Ps110 | Cg75–Pg1–Ps111 | Cg75–Pg1–Ps112 |
| Cg75–Pg1–Ps113 | Cg75–Pg1–Ps114 | Cg75–Pg1–Ps115 | Cg75–Pg1–Ps116 |
| Cg75–Pg1–Ps117 | Cg75–Pg1–Ps118 | Cg75–Pg1–Ps119 | Cg75–Pg1–Ps120 |
| Cg75–Pg1–Ps121 | Cg75–Pg1–Ps122 | Cg75–Pg1–Ps123 | Cg75–Pg1–Ps124 |
| Cg75–Pg1–Ps125 | Cg75–Pg1–Ps126 | Cg75–Pg1–Ps127 | Cg75–Pg1–Ps128 |
| Cg75–Pg1–Ps129 | Cg75–Pg1–Ps130 | Cg75–Pg1–Ps131 | Cg75–Pg1–Ps132 |
| Cg75–Pg1–Ps133 | Cg75–Pg1–Ps134 | Cg75–Pg1–Ps135 | Cg75–Pg1–Ps136 |
| Cg75–Pg1–Ps137 | Cg75–Pg1–Ps138 | Cg75–Pg1–Ps139 | Cg75–Pg1–Ps140 |
| Cg75–Pg1–Ps141 | Cg75–Pg1–Ps142 | Cg75–Pg1–Ps143 | Cg75–Pg1–Ps144 |
| Cg75–Pg1–Ps145 | Cg75–Pg1–Ps146 | Cg75–Pg1–Ps147 | Cg75–Pg1–Ps148 |

| | | | |
|---|---|---|---|
| Cg75–Pg1–Ps149 | Cg75–Pg1–Ps150 | Cg75–Pg1–Ps151 | Cg75–Pg1–Ps152 |
| Cg75–Pg1–Ps153 | Cg75–Pg1–Ps154 | Cg75–Pg1–Ps155 | Cg75–Pg1–Ps156 |
| Cg75–Pg1–Ps157 | Cg75–Pg1–Ps158 | Cg75–Pg1–Ps159 | Cg75–Pg1–Ps160 |
| Cg75–Pg1–Ps161 | Cg75–Pg1–Ps162 | Cg75–Pg1–Ps163 | Cg75–Pg1–Ps164 |
| Cg75–Pg1–Ps165 | Cg75–Pg1–Ps166 | Cg75–Pg1–Ps167 | Cg75–Pg1–Ps168 |
| Cg75–Pg1–Ps169 | Cg75–Pg1–Ps170 | Cg75–Pg1–Ps171 | Cg75–Pg1–Ps172 |
| Cg75–Pg1–Ps173 | Cg75–Pg1–Ps174 | Cg75–Pg1–Ps175 | Cg75–Pg1–Ps176 |
| Cg75–Pg1–Ps177 | Cg75–Pg1–Ps178 | Cg75–Pg1–Ps179 | Cg75–Pg1–Ps180 |
| Cg75–Pg1–Ps181 | Cg75–Pg1–Ps182 | Cg75–Pg1–Ps183 | Cg75–Pg1–Ps184 |
| Cg75–Pg1–Ps185 | Cg75–Pg1–Ps186 | Cg75–Pg1–Ps187 | Cg75–Pg1–Ps188 |
| Cg75–Pg1–Ps189 | Cg75–Pg1–Ps190 | Cg75–Pg1–Ps191 | Cg75–Pg1–Ps192 |
| Cg75–Pg1–Ps193 | Cg75–Pg1–Ps194 | Cg75–Pg1–Ps195 | Cg75–Pg1–Ps196 |
| Cg75–Pg1–Ps197 | Cg75–Pg1–Ps198 | Cg75–Pg1–Ps199 | Cg75–Pg1–Ps200 |
| Cg75–Pg1–Ps201 | Cg75–Pg1–Ps202 | Cg75–Pg1–Ps203 | Cg75–Pg1–Ps204 |
| Cg75–Pg1–Ps205 | Cg75–Pg1–Ps206 | Cg75–Pg1–Ps207 | Cg75–Pg1–Ps208 |
| Cg75–Pg1–Ps209 | Cg75–Pg1–Ps210 | Cg75–Pg1–Ps211 | Cg75–Pg1–Ps212 |
| Cg75–Pg1–Ps213 | Cg75–Pg1–Ps214 | Cg75–Pg1–Ps215 | Cg75–Pg1–Ps216 |
| Cg75–Pg1–Ps217 | Cg75–Pg1–Ps218 | Cg75–Pg1–Ps219 | Cg75–Pg1–Ps220 |
| Cg75–Pg1–Ps221 | Cg75–Pg1–Ps222 | Cg75–Pg1–Ps223 | Cg75–Pg1–Ps224 |
| Cg75–Pg1–Ps225 | Cg75–Pg1–Ps226 | Cg75–Pg1–Ps227 | Cg75–Pg1–Ps228 |
| Cg75–Pg1–Ps229 | Cg75–Pg1–Ps230 | Cg75–Pg1–Ps231 | Cg75–Pg1–Ps232 |
| Cg75–Pg1–Ps233 | Cg75–Pg1–Ps234 | Cg75–Pg1–Ps235 | Cg75–Pg1–Ps236 |
| Cg75–Pg1–Ps237 | Cg75–Pg1–Ps238 | Cg75–Pg1–Ps239 | Cg75–Pg1–Ps240 |
| Cg75–Pg1–Ps241 | Cg75–Pg1–Ps242 | Cg75–Pg1–Ps243 | |

| | | | |
|---|---|---|---|
| Cg76–Pg1–Ps1 | Cg76–Pg1–Ps2 | Cg76–Pg1–Ps3 | Cg76–Pg1–Ps4 |
| Cg76–Pg1–Ps5 | Cg76–Pg1–Ps6 | Cg76–Pg1–Ps7 | Cg76–Pg1–Ps8 |
| Cg76–Pg1–Ps9 | Cg76–Pg1–Ps10 | Cg76–Pg1–Ps11 | Cg76–Pg1–Ps12 |
| Cg76–Pg1–Ps13 | Cg76–Pg1–Ps14 | Cg76–Pg1–Ps15 | Cg76–Pg1–Ps16 |
| Cg76–Pg1–Ps17 | Cg76–Pg1–Ps18 | Cg76–Pg1–Ps19 | Cg76–Pg1–Ps20 |
| Cg76–Pg1–Ps21 | Cg76–Pg1–Ps22 | Cg76–Pg1–Ps23 | Cg76–Pg1–Ps24 |
| Cg76–Pg1–Ps25 | Cg76–Pg1–Ps26 | Cg76–Pg1–Ps27 | Cg76–Pg1–Ps28 |
| Cg76–Pg1–Ps29 | Cg76–Pg1–Ps30 | Cg76–Pg1–Ps31 | Cg76–Pg1–Ps32 |
| Cg76–Pg1–Ps33 | Cg76–Pg1–Ps34 | Cg76–Pg1–Ps35 | Cg76–Pg1–Ps36 |
| Cg76–Pg1–Ps37 | Cg76–Pg1–Ps38 | Cg76–Pg1–Ps39 | Cg76–Pg1–Ps40 |
| Cg76–Pg1–Ps41 | Cg76–Pg1–Ps42 | Cg76–Pg1–Ps43 | Cg76–Pg1–Ps44 |
| Cg76–Pg1–Ps45 | Cg76–Pg1–Ps46 | Cg76–Pg1–Ps47 | Cg76–Pg1–Ps48 |
| Cg76–Pg1–Ps49 | Cg76–Pg1–Ps50 | Cg76–Pg1–Ps51 | Cg76–Pg1–Ps52 |
| Cg76–Pg1–Ps53 | Cg76–Pg1–Ps54 | Cg76–Pg1–Ps55 | Cg76–Pg1–Ps56 |
| Cg76–Pg1–Ps57 | Cg76–Pg1–Ps58 | Cg76–Pg1–Ps59 | Cg76–Pg1–Ps60 |
| Cg76–Pg1–Ps61 | Cg76–Pg1–Ps62 | Cg76–Pg1–Ps63 | Cg76–Pg1–Ps64 |
| Cg76–Pg1–Ps65 | Cg76–Pg1–Ps66 | Cg76–Pg1–Ps67 | Cg76–Pg1–Ps68 |
| Cg76–Pg1–Ps69 | Cg76–Pg1–Ps70 | Cg76–Pg1–Ps71 | Cg76–Pg1–Ps72 |
| Cg76–Pg1–Ps73 | Cg76–Pg1–Ps74 | Cg76–Pg1–Ps75 | Cg76–Pg1–Ps76 |
| Cg76–Pg1–Ps77 | Cg76–Pg1–Ps78 | Cg76–Pg1–Ps79 | Cg76–Pg1–Ps80 |
| Cg76–Pg1–Ps81 | Cg76–Pg1–Ps82 | Cg76–Pg1–Ps83 | Cg76–Pg1–Ps84 |
| Cg76–Pg1–Ps85 | Cg76–Pg1–Ps86 | Cg76–Pg1–Ps87 | Cg76–Pg1–Ps88 |
| Cg76–Pg1–Ps89 | Cg76–Pg1–Ps90 | Cg76–Pg1–Ps91 | Cg76–Pg1–Ps92 |
| Cg76–Pg1–Ps93 | Cg76–Pg1–Ps94 | Cg76–Pg1–Ps95 | Cg76–Pg1–Ps96 |
| Cg76–Pg1–Ps97 | Cg76–Pg1–Ps98 | Cg76–Pg1–Ps99 | Cg76–Pg1–Ps100 |
| Cg76–Pg1–Ps101 | Cg76–Pg1–Ps102 | Cg76–Pg1–Ps103 | Cg76–Pg1–Ps104 |
| Cg76–Pg1–Ps105 | Cg76–Pg1–Ps106 | Cg76–Pg1–Ps107 | Cg76–Pg1–Ps108 |

| | | | |
|---|---|---|---|
| Cg76–Pg1–Ps109 | Cg76–Pg1–Ps110 | Cg76–Pg1–Ps111 | Cg76–Pg1–Ps112 |
| Cg76–Pg1–Ps113 | Cg76–Pg1–Ps114 | Cg76–Pg1–Ps115 | Cg76–Pg1–Ps116 |
| Cg76–Pg1–Ps117 | Cg76–Pg1–Ps118 | Cg76–Pg1–Ps119 | Cg76–Pg1–Ps120 |
| Cg76–Pg1–Ps121 | Cg76–Pg1–Ps122 | Cg76–Pg1–Ps123 | Cg76–Pg1–Ps124 |
| Cg76–Pg1–Ps125 | Cg76–Pg1–Ps126 | Cg76–Pg1–Ps127 | Cg76–Pg1–Ps128 |
| Cg76–Pg1–Ps129 | Cg76–Pg1–Ps130 | Cg76–Pg1–Ps131 | Cg76–Pg1–Ps132 |
| Cg76–Pg1–Ps133 | Cg76–Pg1–Ps134 | Cg76–Pg1–Ps135 | Cg76–Pg1–Ps136 |
| Cg76–Pg1–Ps137 | Cg76–Pg1–Ps138 | Cg76–Pg1–Ps139 | Cg76–Pg1–Ps140 |
| Cg76–Pg1–Ps141 | Cg76–Pg1–Ps142 | Cg76–Pg1–Ps143 | Cg76–Pg1–Ps144 |
| Cg76–Pg1–Ps145 | Cg76–Pg1–Ps146 | Cg76–Pg1–Ps147 | Cg76–Pg1–Ps148 |
| Cg76–Pg1–Ps149 | Cg76–Pg1–Ps150 | Cg76–Pg1–Ps151 | Cg76–Pg1–Ps152 |
| Cg76–Pg1–Ps153 | Cg76–Pg1–Ps154 | Cg76–Pg1–Ps155 | Cg76–Pg1–Ps156 |
| Cg76–Pg1–Ps157 | Cg76–Pg1–Ps158 | Cg76–Pg1–Ps159 | Cg76–Pg1–Ps160 |
| Cg76–Pg1–Ps161 | Cg76–Pg1–Ps162 | Cg76–Pg1–Ps163 | Cg76–Pg1–Ps164 |
| Cg76–Pg1–Ps165 | Cg76–Pg1–Ps166 | Cg76–Pg1–Ps167 | Cg76–Pg1–Ps168 |
| Cg76–Pg1–Ps169 | Cg76–Pg1–Ps170 | Cg76–Pg1–Ps171 | Cg76–Pg1–Ps172 |
| Cg76–Pg1–Ps173 | Cg76–Pg1–Ps174 | Cg76–Pg1–Ps175 | Cg76–Pg1–Ps176 |
| Cg76–Pg1–Ps177 | Cg76–Pg1–Ps178 | Cg76–Pg1–Ps179 | Cg76–Pg1–Ps180 |
| Cg76–Pg1–Ps181 | Cg76–Pg1–Ps182 | Cg76–Pg1–Ps183 | Cg76–Pg1–Ps184 |
| Cg76–Pg1–Ps185 | Cg76–Pg1–Ps186 | Cg76–Pg1–Ps187 | Cg76–Pg1–Ps188 |
| Cg76–Pg1–Ps189 | Cg76–Pg1–Ps190 | Cg76–Pg1–Ps191 | Cg76–Pg1–Ps192 |
| Cg76–Pg1–Ps193 | Cg76–Pg1–Ps194 | Cg76–Pg1–Ps195 | Cg76–Pg1–Ps196 |
| Cg76–Pg1–Ps197 | Cg76–Pg1–Ps198 | Cg76–Pg1–Ps199 | Cg76–Pg1–Ps200 |
| Cg76–Pg1–Ps201 | Cg76–Pg1–Ps202 | Cg76–Pg1–Ps203 | Cg76–Pg1–Ps204 |
| Cg76–Pg1–Ps205 | Cg76–Pg1–Ps206 | Cg76–Pg1–Ps207 | Cg76–Pg1–Ps208 |
| Cg76–Pg1–Ps209 | Cg76–Pg1–Ps210 | Cg76–Pg1–Ps211 | Cg76–Pg1–Ps212 |
| Cg76–Pg1–Ps213 | Cg76–Pg1–Ps214 | Cg76–Pg1–Ps215 | Cg76–Pg1–Ps216 |
| Cg76–Pg1–Ps217 | Cg76–Pg1–Ps218 | Cg76–Pg1–Ps219 | Cg76–Pg1–Ps220 |
| Cg76–Pg1–Ps221 | Cg76–Pg1–Ps222 | Cg76–Pg1–Ps223 | Cg76–Pg1–Ps224 |
| Cg76–Pg1–Ps225 | Cg76–Pg1–Ps226 | Cg76–Pg1–Ps227 | Cg76–Pg1–Ps228 |
| Cg76–Pg1–Ps229 | Cg76–Pg1–Ps230 | Cg76–Pg1–Ps231 | Cg76–Pg1–Ps232 |
| Cg76–Pg1–Ps233 | Cg76–Pg1–Ps234 | Cg76–Pg1–Ps235 | Cg76–Pg1–Ps236 |
| Cg76–Pg1–Ps237 | Cg76–Pg1–Ps238 | Cg76–Pg1–Ps239 | Cg76–Pg1–Ps240 |
| Cg76–Pg1–Ps241 | Cg76–Pg1–Ps242 | Cg76–Pg1–Ps243 | |

| | | | |
|---|---|---|---|
| Cg81–Pg1–Ps1 | Cg81–Pg1–Ps2 | Cg81–Pg1–Ps3 | Cg81–Pg1–Ps4 |
| Cg81–Pg1–Ps5 | Cg81–Pg1–Ps6 | Cg81–Pg1–Ps7 | Cg81–Pg1–Ps8 |
| Cg81–Pg1–Ps9 | Cg81–Pg1–Ps10 | Cg81–Pg1–Ps11 | Cg81–Pg1–Ps12 |
| Cg81–Pg1–Ps13 | Cg81–Pg1–Ps14 | Cg81–Pg1–Ps15 | Cg81–Pg1–Ps16 |
| Cg81–Pg1–Ps17 | Cg81–Pg1–Ps18 | Cg81–Pg1–Ps19 | Cg81–Pg1–Ps20 |
| Cg81–Pg1–Ps21 | Cg81–Pg1–Ps22 | Cg81–Pg1–Ps23 | Cg81–Pg1–Ps24 |
| Cg81–Pg1–Ps25 | Cg81–Pg1–Ps26 | Cg81–Pg1–Ps27 | Cg81–Pg1–Ps28 |
| Cg81–Pg1–Ps29 | Cg81–Pg1–Ps30 | Cg81–Pg1–Ps31 | Cg81–Pg1–Ps32 |
| Cg81–Pg1–Ps33 | Cg81–Pg1–Ps34 | Cg81–Pg1–Ps35 | Cg81–Pg1–Ps36 |
| Cg81–Pg1–Ps37 | Cg81–Pg1–Ps38 | Cg81–Pg1–Ps39 | Cg81–Pg1–Ps40 |
| Cg81–Pg1–Ps41 | Cg81–Pg1–Ps42 | Cg81–Pg1–Ps43 | Cg81–Pg1–Ps44 |
| Cg81–Pg1–Ps45 | Cg81–Pg1–Ps46 | Cg81–Pg1–Ps47 | Cg81–Pg1–Ps48 |
| Cg81–Pg1–Ps49 | Cg81–Pg1–Ps50 | Cg81–Pg1–Ps51 | Cg81–Pg1–Ps52 |
| Cg81–Pg1–Ps53 | Cg81–Pg1–Ps54 | Cg81–Pg1–Ps55 | Cg81–Pg1–Ps56 |
| Cg81–Pg1–Ps57 | Cg81–Pg1–Ps58 | Cg81–Pg1–Ps59 | Cg81–Pg1–Ps60 |
| Cg81–Pg1–Ps61 | Cg81–Pg1–Ps62 | Cg81–Pg1–Ps63 | Cg81–Pg1–Ps64 |
| Cg81–Pg1–Ps65 | Cg81–Pg1–Ps66 | Cg81–Pg1–Ps67 | Cg81–Pg1–Ps68 |

| | | | |
|---|---|---|---|
| Cg81–Pg1–Ps69 | Cg81–Pg1–Ps70 | Cg81–Pg1–Ps71 | Cg81–Pg1–Ps72 |
| Cg81–Pg1–Ps73 | Cg81–Pg1–Ps74 | Cg81–Pg1–Ps75 | Cg81–Pg1–Ps76 |
| Cg81–Pg1–Ps77 | Cg81–Pg1–Ps78 | Cg81–Pg1–Ps79 | Cg81–Pg1–Ps80 |
| Cg81–Pg1–Ps81 | Cg81–Pg1–Ps82 | Cg81–Pg1–Ps83 | Cg81–Pg1–Ps84 |
| Cg81–Pg1–Ps85 | Cg81–Pg1–Ps86 | Cg81–Pg1–Ps87 | Cg81–Pg1–Ps88 |
| Cg81–Pg1–Ps89 | Cg81–Pg1–Ps90 | Cg81–Pg1–Ps91 | Cg81–Pg1–Ps92 |
| Cg81–Pg1–Ps93 | Cg81–Pg1–Ps94 | Cg81–Pg1–Ps95 | Cg81–Pg1–Ps96 |
| Cg81–Pg1–Ps97 | Cg81–Pg1–Ps98 | Cg81–Pg1–Ps99 | Cg81–Pg1–Ps100 |
| Cg81–Pg1–Ps101 | Cg81–Pg1–Ps102 | Cg81–Pg1–Ps103 | Cg81–Pg1–Ps104 |
| Cg81–Pg1–Ps105 | Cg81–Pg1–Ps106 | Cg81–Pg1–Ps107 | Cg81–Pg1–Ps108 |
| Cg81–Pg1–Ps109 | Cg81–Pg1–Ps110 | Cg81–Pg1–Ps111 | Cg81–Pg1–Ps112 |
| Cg81–Pg1–Ps113 | Cg81–Pg1–Ps114 | Cg81–Pg1–Ps115 | Cg81–Pg1–Ps116 |
| Cg81–Pg1–Ps117 | Cg81–Pg1–Ps118 | Cg81–Pg1–Ps119 | Cg81–Pg1–Ps120 |
| Cg81–Pg1–Ps121 | Cg81–Pg1–Ps122 | Cg81–Pg1–Ps123 | Cg81–Pg1–Ps124 |
| Cg81–Pg1–Ps125 | Cg81–Pg1–Ps126 | Cg81–Pg1–Ps127 | Cg81–Pg1–Ps128 |
| Cg81–Pg1–Ps129 | Cg81–Pg1–Ps130 | Cg81–Pg1–Ps131 | Cg81–Pg1–Ps132 |
| Cg81–Pg1–Ps133 | Cg81–Pg1–Ps134 | Cg81–Pg1–Ps135 | Cg81–Pg1–Ps136 |
| Cg81–Pg1–Ps137 | Cg81–Pg1–Ps138 | Cg81–Pg1–Ps139 | Cg81–Pg1–Ps140 |
| Cg81–Pg1–Ps141 | Cg81–Pg1–Ps142 | Cg81–Pg1–Ps143 | Cg81–Pg1–Ps144 |
| Cg81–Pg1–Ps145 | Cg81–Pg1–Ps146 | Cg81–Pg1–Ps147 | Cg81–Pg1–Ps148 |
| Cg81–Pg1–Ps149 | Cg81–Pg1–Ps150 | Cg81–Pg1–Ps151 | Cg81–Pg1–Ps152 |
| Cg81–Pg1–Ps153 | Cg81–Pg1–Ps154 | Cg81–Pg1–Ps155 | Cg81–Pg1–Ps156 |
| Cg81–Pg1–Ps157 | Cg81–Pg1–Ps158 | Cg81–Pg1–Ps159 | Cg81–Pg1–Ps160 |
| Cg81–Pg1–Ps161 | Cg81–Pg1–Ps162 | Cg81–Pg1–Ps163 | Cg81–Pg1–Ps164 |
| Cg81–Pg1–Ps165 | Cg81–Pg1–Ps166 | Cg81–Pg1–Ps167 | Cg81–Pg1–Ps168 |
| Cg81–Pg1–Ps169 | Cg81–Pg1–Ps170 | Cg81–Pg1–Ps171 | Cg81–Pg1–Ps172 |
| Cg81–Pg1–Ps173 | Cg81–Pg1–Ps174 | Cg81–Pg1–Ps175 | Cg81–Pg1–Ps176 |
| Cg81–Pg1–Ps177 | Cg81–Pg1–Ps178 | Cg81–Pg1–Ps179 | Cg81–Pg1–Ps180 |
| Cg81–Pg1–Ps181 | Cg81–Pg1–Ps182 | Cg81–Pg1–Ps183 | Cg81–Pg1–Ps184 |
| Cg81–Pg1–Ps185 | Cg81–Pg1–Ps186 | Cg81–Pg1–Ps187 | Cg81–Pg1–Ps188 |
| Cg81–Pg1–Ps189 | Cg81–Pg1–Ps190 | Cg81–Pg1–Ps191 | Cg81–Pg1–Ps192 |
| Cg81–Pg1–Ps193 | Cg81–Pg1–Ps194 | Cg81–Pg1–Ps195 | Cg81–Pg1–Ps196 |
| Cg81–Pg1–Ps197 | Cg81–Pg1–Ps198 | Cg81–Pg1–Ps199 | Cg81–Pg1–Ps200 |
| Cg81–Pg1–Ps201 | Cg81–Pg1–Ps202 | Cg81–Pg1–Ps203 | Cg81–Pg1–Ps204 |
| Cg81–Pg1–Ps205 | Cg81–Pg1–Ps206 | Cg81–Pg1–Ps207 | Cg81–Pg1–Ps208 |
| Cg81–Pg1–Ps209 | Cg81–Pg1–Ps210 | Cg81–Pg1–Ps211 | Cg81–Pg1–Ps212 |
| Cg81–Pg1–Ps213 | Cg81–Pg1–Ps214 | Cg81–Pg1–Ps215 | Cg81–Pg1–Ps216 |
| Cg81–Pg1–Ps217 | Cg81–Pg1–Ps218 | Cg81–Pg1–Ps219 | Cg81–Pg1–Ps220 |
| Cg81–Pg1–Ps221 | Cg81–Pg1–Ps222 | Cg81–Pg1–Ps223 | Cg81–Pg1–Ps224 |
| Cg81–Pg1–Ps225 | Cg81–Pg1–Ps226 | Cg81–Pg1–Ps227 | Cg81–Pg1–Ps228 |
| Cg81–Pg1–Ps229 | Cg81–Pg1–Ps230 | Cg81–Pg1–Ps231 | Cg81–Pg1–Ps232 |
| Cg81–Pg1–Ps233 | Cg81–Pg1–Ps234 | Cg81–Pg1–Ps235 | Cg81–Pg1–Ps236 |
| Cg81–Pg1–Ps237 | Cg81–Pg1–Ps238 | Cg81–Pg1–Ps239 | Cg81–Pg1–Ps240 |
| Cg81–Pg1–Ps241 | Cg81–Pg1–Ps242 | Cg81–Pg1–Ps243 | |

| | | | |
|---|---|---|---|
| Cg82–Pg1–Ps1 | Cg82–Pg1–Ps2 | Cg82–Pg1–Ps3 | Cg82–Pg1–Ps4 |
| Cg82–Pg1–Ps5 | Cg82–Pg1–Ps6 | Cg82–Pg1–Ps7 | Cg82–Pg1–Ps8 |
| Cg82–Pg1–Ps9 | Cg82–Pg1–Ps10 | Cg82–Pg1–Ps11 | Cg82–Pg1–Ps12 |
| Cg82–Pg1–Ps13 | Cg82–Pg1–Ps14 | Cg82–Pg1–Ps15 | Cg82–Pg1–Ps16 |
| Cg82–Pg1–Ps17 | Cg82–Pg1–Ps18 | Cg82–Pg1–Ps19 | Cg82–Pg1–Ps20 |
| Cg82–Pg1–Ps21 | Cg82–Pg1–Ps22 | Cg82–Pg1–Ps23 | Cg82–Pg1–Ps24 |
| Cg82–Pg1–Ps25 | Cg82–Pg1–Ps26 | Cg82–Pg1–Ps27 | Cg82–Pg1–Ps28 |

| | | | |
|---|---|---|---|
| Cg82–Pg1–Ps29 | Cg82–Pg1–Ps30 | Cg82–Pg1–Ps31 | Cg82–Pg1–Ps32 |
| Cg82–Pg1–Ps33 | Cg82–Pg1–Ps34 | Cg82–Pg1–Ps35 | Cg82–Pg1–Ps36 |
| Cg82–Pg1–Ps37 | Cg82–Pg1–Ps38 | Cg82–Pg1–Ps39 | Cg82–Pg1–Ps40 |
| Cg82–Pg1–Ps41 | Cg82–Pg1–Ps42 | Cg82–Pg1–Ps43 | Cg82–Pg1–Ps44 |
| Cg82–Pg1–Ps45 | Cg82–Pg1–Ps46 | Cg82–Pg1–Ps47 | Cg82–Pg1–Ps48 |
| Cg82–Pg1–Ps49 | Cg82–Pg1–Ps50 | Cg82–Pg1–Ps51 | Cg82–Pg1–Ps52 |
| Cg82–Pg1–Ps53 | Cg82–Pg1–Ps54 | Cg82–Pg1–Ps55 | Cg82–Pg1–Ps56 |
| Cg82–Pg1–Ps57 | Cg82–Pg1–Ps58 | Cg82–Pg1–Ps59 | Cg82–Pg1–Ps60 |
| Cg82–Pg1–Ps61 | Cg82–Pg1–Ps62 | Cg82–Pg1–Ps63 | Cg82–Pg1–Ps64 |
| Cg82–Pg1–Ps65 | Cg82–Pg1–Ps66 | Cg82–Pg1–Ps67 | Cg82–Pg1–Ps68 |
| Cg82–Pg1–Ps69 | Cg82–Pg1–Ps70 | Cg82–Pg1–Ps71 | Cg82–Pg1–Ps72 |
| Cg82–Pg1–Ps73 | Cg82–Pg1–Ps74 | Cg82–Pg1–Ps75 | Cg82–Pg1–Ps76 |
| Cg82–Pg1–Ps77 | Cg82–Pg1–Ps78 | Cg82–Pg1–Ps79 | Cg82–Pg1–Ps80 |
| Cg82–Pg1–Ps81 | Cg82–Pg1–Ps82 | Cg82–Pg1–Ps83 | Cg82–Pg1–Ps84 |
| Cg82–Pg1–Ps85 | Cg82–Pg1–Ps86 | Cg82–Pg1–Ps87 | Cg82–Pg1–Ps88 |
| Cg82–Pg1–Ps89 | Cg82–Pg1–Ps90 | Cg82–Pg1–Ps91 | Cg82–Pg1–Ps92 |
| Cg82–Pg1–Ps93 | Cg82–Pg1–Ps94 | Cg82–Pg1–Ps95 | Cg82–Pg1–Ps96 |
| Cg82–Pg1–Ps97 | Cg82–Pg1–Ps98 | Cg82–Pg1–Ps99 | Cg82–Pg1–Ps100 |
| Cg82–Pg1–Ps101 | Cg82–Pg1–Ps102 | Cg82–Pg1–Ps103 | Cg82–Pg1–Ps104 |
| Cg82–Pg1–Ps105 | Cg82–Pg1–Ps106 | Cg82–Pg1–Ps107 | Cg82–Pg1–Ps108 |
| Cg82–Pg1–Ps109 | Cg82–Pg1–Ps110 | Cg82–Pg1–Ps111 | Cg82–Pg1–Ps112 |
| Cg82–Pg1–Ps113 | Cg82–Pg1–Ps114 | Cg82–Pg1–Ps115 | Cg82–Pg1–Ps116 |
| Cg82–Pg1–Ps117 | Cg82–Pg1–Ps118 | Cg82–Pg1–Ps119 | Cg82–Pg1–Ps120 |
| Cg82–Pg1–Ps121 | Cg82–Pg1–Ps122 | Cg82–Pg1–Ps123 | Cg82–Pg1–Ps124 |
| Cg82–Pg1–Ps125 | Cg82–Pg1–Ps126 | Cg82–Pg1–Ps127 | Cg82–Pg1–Ps128 |
| Cg82–Pg1–Ps129 | Cg82–Pg1–Ps130 | Cg82–Pg1–Ps131 | Cg82–Pg1–Ps132 |
| Cg82–Pg1–Ps133 | Cg82–Pg1–Ps134 | Cg82–Pg1–Ps135 | Cg82–Pg1–Ps136 |
| Cg82–Pg1–Ps137 | Cg82–Pg1–Ps138 | Cg82–Pg1–Ps139 | Cg82–Pg1–Ps140 |
| Cg82–Pg1–Ps141 | Cg82–Pg1–Ps142 | Cg82–Pg1–Ps143 | Cg82–Pg1–Ps144 |
| Cg82–Pg1–Ps145 | Cg82–Pg1–Ps146 | Cg82–Pg1–Ps147 | Cg82–Pg1–Ps148 |
| Cg82–Pg1–Ps149 | Cg82–Pg1–Ps150 | Cg82–Pg1–Ps151 | Cg82–Pg1–Ps152 |
| Cg82–Pg1–Ps153 | Cg82–Pg1–Ps154 | Cg82–Pg1–Ps155 | Cg82–Pg1–Ps156 |
| Cg82–Pg1–Ps157 | Cg82–Pg1–Ps158 | Cg82–Pg1–Ps159 | Cg82–Pg1–Ps160 |
| Cg82–Pg1–Ps161 | Cg82–Pg1–Ps162 | Cg82–Pg1–Ps163 | Cg82–Pg1–Ps164 |
| Cg82–Pg1–Ps165 | Cg82–Pg1–Ps166 | Cg82–Pg1–Ps167 | Cg82–Pg1–Ps168 |
| Cg82–Pg1–Ps169 | Cg82–Pg1–Ps170 | Cg82–Pg1–Ps171 | Cg82–Pg1–Ps172 |
| Cg82–Pg1–Ps173 | Cg82–Pg1–Ps174 | Cg82–Pg1–Ps175 | Cg82–Pg1–Ps176 |
| Cg82–Pg1–Ps177 | Cg82–Pg1–Ps178 | Cg82–Pg1–Ps179 | Cg82–Pg1–Ps180 |
| Cg82–Pg1–Ps181 | Cg82–Pg1–Ps182 | Cg82–Pg1–Ps183 | Cg82–Pg1–Ps184 |
| Cg82–Pg1–Ps185 | Cg82–Pg1–Ps186 | Cg82–Pg1–Ps187 | Cg82–Pg1–Ps188 |
| Cg82–Pg1–Ps189 | Cg82–Pg1–Ps190 | Cg82–Pg1–Ps191 | Cg82–Pg1–Ps192 |
| Cg82–Pg1–Ps193 | Cg82–Pg1–Ps194 | Cg82–Pg1–Ps195 | Cg82–Pg1–Ps196 |
| Cg82–Pg1–Ps197 | Cg82–Pg1–Ps198 | Cg82–Pg1–Ps199 | Cg82–Pg1–Ps200 |
| Cg82–Pg1–Ps201 | Cg82–Pg1–Ps202 | Cg82–Pg1–Ps203 | Cg82–Pg1–Ps204 |
| Cg82–Pg1–Ps205 | Cg82–Pg1–Ps206 | Cg82–Pg1–Ps207 | Cg82–Pg1–Ps208 |
| Cg82–Pg1–Ps209 | Cg82–Pg1–Ps210 | Cg82–Pg1–Ps211 | Cg82–Pg1–Ps212 |
| Cg82–Pg1–Ps213 | Cg82–Pg1–Ps214 | Cg82–Pg1–Ps215 | Cg82–Pg1–Ps216 |
| Cg82–Pg1–Ps217 | Cg82–Pg1–Ps218 | Cg82–Pg1–Ps219 | Cg82–Pg1–Ps220 |
| Cg82–Pg1–Ps221 | Cg82–Pg1–Ps222 | Cg82–Pg1–Ps223 | Cg82–Pg1–Ps224 |
| Cg82–Pg1–Ps225 | Cg82–Pg1–Ps226 | Cg82–Pg1–Ps227 | Cg82–Pg1–Ps228 |
| Cg82–Pg1–Ps229 | Cg82–Pg1–Ps230 | Cg82–Pg1–Ps231 | Cg82–Pg1–Ps232 |
| Cg82–Pg1–Ps233 | Cg82–Pg1–Ps234 | Cg82–Pg1–Ps235 | Cg82–Pg1–Ps236 |

| | | | |
|---|---|---|---|
| Cg82–Pg1–Ps237 | Cg82–Pg1–Ps238 | Cg82–Pg1–Ps239 | Cg82–Pg1–Ps240 |
| Cg82–Pg1–Ps241 | Cg82–Pg1–Ps242 | Cg82–Pg1–Ps243 | |

| | | | |
|---|---|---|---|
| Cg83–Pg1–Ps1 | Cg83–Pg1–Ps2 | Cg83–Pg1–Ps3 | Cg83–Pg1–Ps4 |
| Cg83–Pg1–Ps5 | Cg83–Pg1–Ps6 | Cg83–Pg1–Ps7 | Cg83–Pg1–Ps8 |
| Cg83–Pg1–Ps9 | Cg83–Pg1–Ps10 | Cg83–Pg1–Ps11 | Cg83–Pg1–Ps12 |
| Cg83–Pg1–Ps13 | Cg83–Pg1–Ps14 | Cg83–Pg1–Ps15 | Cg83–Pg1–Ps16 |
| Cg83–Pg1–Ps17 | Cg83–Pg1–Ps18 | Cg83–Pg1–Ps19 | Cg83–Pg1–Ps20 |
| Cg83–Pg1–Ps21 | Cg83–Pg1–Ps22 | Cg83–Pg1–Ps23 | Cg83–Pg1–Ps24 |
| Cg83–Pg1–Ps25 | Cg83–Pg1–Ps26 | Cg83–Pg1–Ps27 | Cg83–Pg1–Ps28 |
| Cg83–Pg1–Ps29 | Cg83–Pg1–Ps30 | Cg83–Pg1–Ps31 | Cg83–Pg1–Ps32 |
| Cg83–Pg1–Ps33 | Cg83–Pg1–Ps34 | Cg83–Pg1–Ps35 | Cg83–Pg1–Ps36 |
| Cg83–Pg1–Ps37 | Cg83–Pg1–Ps38 | Cg83–Pg1–Ps39 | Cg83–Pg1–Ps40 |
| Cg83–Pg1–Ps41 | Cg83–Pg1–Ps42 | Cg83–Pg1–Ps43 | Cg83–Pg1–Ps44 |
| Cg83–Pg1–Ps45 | Cg83–Pg1–Ps46 | Cg83–Pg1–Ps47 | Cg83–Pg1–Ps48 |
| Cg83–Pg1–Ps49 | Cg83–Pg1–Ps50 | Cg83–Pg1–Ps51 | Cg83–Pg1–Ps52 |
| Cg83–Pg1–Ps53 | Cg83–Pg1–Ps54 | Cg83–Pg1–Ps55 | Cg83–Pg1–Ps56 |
| Cg83–Pg1–Ps57 | Cg83–Pg1–Ps58 | Cg83–Pg1–Ps59 | Cg83–Pg1–Ps60 |
| Cg83–Pg1–Ps61 | Cg83–Pg1–Ps62 | Cg83–Pg1–Ps63 | Cg83–Pg1–Ps64 |
| Cg83–Pg1–Ps65 | Cg83–Pg1–Ps66 | Cg83–Pg1–Ps67 | Cg83–Pg1–Ps68 |
| Cg83–Pg1–Ps69 | Cg83–Pg1–Ps70 | Cg83–Pg1–Ps71 | Cg83–Pg1–Ps72 |
| Cg83–Pg1–Ps73 | Cg83–Pg1–Ps74 | Cg83–Pg1–Ps75 | Cg83–Pg1–Ps76 |
| Cg83–Pg1–Ps77 | Cg83–Pg1–Ps78 | Cg83–Pg1–Ps79 | Cg83–Pg1–Ps80 |
| Cg83–Pg1–Ps81 | Cg83–Pg1–Ps82 | Cg83–Pg1–Ps83 | Cg83–Pg1–Ps84 |
| Cg83–Pg1–Ps85 | Cg83–Pg1–Ps86 | Cg83–Pg1–Ps87 | Cg83–Pg1–Ps88 |
| Cg83–Pg1–Ps89 | Cg83–Pg1–Ps90 | Cg83–Pg1–Ps91 | Cg83–Pg1–Ps92 |
| Cg83–Pg1–Ps93 | Cg83–Pg1–Ps94 | Cg83–Pg1–Ps95 | Cg83–Pg1–Ps96 |
| Cg83–Pg1–Ps97 | Cg83–Pg1–Ps98 | Cg83–Pg1–Ps99 | Cg83–Pg1–Ps100 |
| Cg83–Pg1–Ps101 | Cg83–Pg1–Ps102 | Cg83–Pg1–Ps103 | Cg83–Pg1–Ps104 |
| Cg83–Pg1–Ps105 | Cg83–Pg1–Ps106 | Cg83–Pg1–Ps107 | Cg83–Pg1–Ps108 |
| Cg83–Pg1–Ps109 | Cg83–Pg1–Ps110 | Cg83–Pg1–Ps111 | Cg83–Pg1–Ps112 |
| Cg83–Pg1–Ps113 | Cg83–Pg1–Ps114 | Cg83–Pg1–Ps115 | Cg83–Pg1–Ps116 |
| Cg83–Pg1–Ps117 | Cg83–Pg1–Ps118 | Cg83–Pg1–Ps119 | Cg83–Pg1–Ps120 |
| Cg83–Pg1–Ps121 | Cg83–Pg1–Ps122 | Cg83–Pg1–Ps123 | Cg83–Pg1–Ps124 |
| Cg83–Pg1–Ps125 | Cg83–Pg1–Ps126 | Cg83–Pg1–Ps127 | Cg83–Pg1–Ps128 |
| Cg83–Pg1–Ps129 | Cg83–Pg1–Ps130 | Cg83–Pg1–Ps131 | Cg83–Pg1–Ps132 |
| Cg83–Pg1–Ps133 | Cg83–Pg1–Ps134 | Cg83–Pg1–Ps135 | Cg83–Pg1–Ps136 |
| Cg83–Pg1–Ps137 | Cg83–Pg1–Ps138 | Cg83–Pg1–Ps139 | Cg83–Pg1–Ps140 |
| Cg83–Pg1–Ps141 | Cg83–Pg1–Ps142 | Cg83–Pg1–Ps143 | Cg83–Pg1–Ps144 |
| Cg83–Pg1–Ps145 | Cg83–Pg1–Ps146 | Cg83–Pg1–Ps147 | Cg83–Pg1–Ps148 |
| Cg83–Pg1–Ps149 | Cg83–Pg1–Ps150 | Cg83–Pg1–Ps151 | Cg83–Pg1–Ps152 |
| Cg83–Pg1–Ps153 | Cg83–Pg1–Ps154 | Cg83–Pg1–Ps155 | Cg83–Pg1–Ps156 |
| Cg83–Pg1–Ps157 | Cg83–Pg1–Ps158 | Cg83–Pg1–Ps159 | Cg83–Pg1–Ps160 |
| Cg83–Pg1–Ps161 | Cg83–Pg1–Ps162 | Cg83–Pg1–Ps163 | Cg83–Pg1–Ps164 |
| Cg83–Pg1–Ps165 | Cg83–Pg1–Ps166 | Cg83–Pg1–Ps167 | Cg83–Pg1–Ps168 |
| Cg83–Pg1–Ps169 | Cg83–Pg1–Ps170 | Cg83–Pg1–Ps171 | Cg83–Pg1–Ps172 |
| Cg83–Pg1–Ps173 | Cg83–Pg1–Ps174 | Cg83–Pg1–Ps175 | Cg83–Pg1–Ps176 |
| Cg83–Pg1–Ps177 | Cg83–Pg1–Ps178 | Cg83–Pg1–Ps179 | Cg83–Pg1–Ps180 |
| Cg83–Pg1–Ps181 | Cg83–Pg1–Ps182 | Cg83–Pg1–Ps183 | Cg83–Pg1–Ps184 |
| Cg83–Pg1–Ps185 | Cg83–Pg1–Ps186 | Cg83–Pg1–Ps187 | Cg83–Pg1–Ps188 |
| Cg83–Pg1–Ps189 | Cg83–Pg1–Ps190 | Cg83–Pg1–Ps191 | Cg83–Pg1–Ps192 |

| | | | |
|---|---|---|---|
| Cg83–Pg1–Ps193 | Cg83–Pg1–Ps194 | Cg83–Pg1–Ps195 | Cg83–Pg1–Ps196 |
| Cg83–Pg1–Ps197 | Cg83–Pg1–Ps198 | Cg83–Pg1–Ps199 | Cg83–Pg1–Ps200 |
| Cg83–Pg1–Ps201 | Cg83–Pg1–Ps202 | Cg83–Pg1–Ps203 | Cg83–Pg1–Ps204 |
| Cg83–Pg1–Ps205 | Cg83–Pg1–Ps206 | Cg83–Pg1–Ps207 | Cg83–Pg1–Ps208 |
| Cg83–Pg1–Ps209 | Cg83–Pg1–Ps210 | Cg83–Pg1–Ps211 | Cg83–Pg1–Ps212 |
| Cg83–Pg1–Ps213 | Cg83–Pg1–Ps214 | Cg83–Pg1–Ps215 | Cg83–Pg1–Ps216 |
| Cg83–Pg1–Ps217 | Cg83–Pg1–Ps218 | Cg83–Pg1–Ps219 | Cg83–Pg1–Ps220 |
| Cg83–Pg1–Ps221 | Cg83–Pg1–Ps222 | Cg83–Pg1–Ps223 | Cg83–Pg1–Ps224 |
| Cg83–Pg1–Ps225 | Cg83–Pg1–Ps226 | Cg83–Pg1–Ps227 | Cg83–Pg1–Ps228 |
| Cg83–Pg1–Ps229 | Cg83–Pg1–Ps230 | Cg83–Pg1–Ps231 | Cg83–Pg1–Ps232 |
| Cg83–Pg1–Ps233 | Cg83–Pg1–Ps234 | Cg83–Pg1–Ps235 | Cg83–Pg1–Ps236 |
| Cg83–Pg1–Ps237 | Cg83–Pg1–Ps238 | Cg83–Pg1–Ps239 | Cg83–Pg1–Ps240 |
| Cg83–Pg1–Ps241 | Cg83–Pg1–Ps242 | Cg83–Pg1–Ps243 | |

| | | | |
|---|---|---|---|
| Cg84–Pg1–Ps1 | Cg84–Pg1–Ps2 | Cg84–Pg1–Ps3 | Cg84–Pg1–Ps4 |
| Cg84–Pg1–Ps5 | Cg84–Pg1–Ps6 | Cg84–Pg1–Ps7 | Cg84–Pg1–Ps8 |
| Cg84–Pg1–Ps9 | Cg84–Pg1–Ps10 | Cg84–Pg1–Ps11 | Cg84–Pg1–Ps12 |
| Cg84–Pg1–Ps13 | Cg84–Pg1–Ps14 | Cg84–Pg1–Ps15 | Cg84–Pg1–Ps16 |
| Cg84–Pg1–Ps17 | Cg84–Pg1–Ps18 | Cg84–Pg1–Ps19 | Cg84–Pg1–Ps20 |
| Cg84–Pg1–Ps21 | Cg84–Pg1–Ps22 | Cg84–Pg1–Ps23 | Cg84–Pg1–Ps24 |
| Cg84–Pg1–Ps25 | Cg84–Pg1–Ps26 | Cg84–Pg1–Ps27 | Cg84–Pg1–Ps28 |
| Cg84–Pg1–Ps29 | Cg84–Pg1–Ps30 | Cg84–Pg1–Ps31 | Cg84–Pg1–Ps32 |
| Cg84–Pg1–Ps33 | Cg84–Pg1–Ps34 | Cg84–Pg1–Ps35 | Cg84–Pg1–Ps36 |
| Cg84–Pg1–Ps37 | Cg84–Pg1–Ps38 | Cg84–Pg1–Ps39 | Cg84–Pg1–Ps40 |
| Cg84–Pg1–Ps41 | Cg84–Pg1–Ps42 | Cg84–Pg1–Ps43 | Cg84–Pg1–Ps44 |
| Cg84–Pg1–Ps45 | Cg84–Pg1–Ps46 | Cg84–Pg1–Ps47 | Cg84–Pg1–Ps48 |
| Cg84–Pg1–Ps49 | Cg84–Pg1–Ps50 | Cg84–Pg1–Ps51 | Cg84–Pg1–Ps52 |
| Cg84–Pg1–Ps53 | Cg84–Pg1–Ps54 | Cg84–Pg1–Ps55 | Cg84–Pg1–Ps56 |
| Cg84–Pg1–Ps57 | Cg84–Pg1–Ps58 | Cg84–Pg1–Ps59 | Cg84–Pg1–Ps60 |
| Cg84–Pg1–Ps61 | Cg84–Pg1–Ps62 | Cg84–Pg1–Ps63 | Cg84–Pg1–Ps64 |
| Cg84–Pg1–Ps65 | Cg84–Pg1–Ps66 | Cg84–Pg1–Ps67 | Cg84–Pg1–Ps68 |
| Cg84–Pg1–Ps69 | Cg84–Pg1–Ps70 | Cg84–Pg1–Ps71 | Cg84–Pg1–Ps72 |
| Cg84–Pg1–Ps73 | Cg84–Pg1–Ps74 | Cg84–Pg1–Ps75 | Cg84–Pg1–Ps76 |
| Cg84–Pg1–Ps77 | Cg84–Pg1–Ps78 | Cg84–Pg1–Ps79 | Cg84–Pg1–Ps80 |
| Cg84–Pg1–Ps81 | Cg84–Pg1–Ps82 | Cg84–Pg1–Ps83 | Cg84–Pg1–Ps84 |
| Cg84–Pg1–Ps85 | Cg84–Pg1–Ps86 | Cg84–Pg1–Ps87 | Cg84–Pg1–Ps88 |
| Cg84–Pg1–Ps89 | Cg84–Pg1–Ps90 | Cg84–Pg1–Ps91 | Cg84–Pg1–Ps92 |
| Cg84–Pg1–Ps93 | Cg84–Pg1–Ps94 | Cg84–Pg1–Ps95 | Cg84–Pg1–Ps96 |
| Cg84–Pg1–Ps97 | Cg84–Pg1–Ps98 | Cg84–Pg1–Ps99 | Cg84–Pg1–Ps100 |
| Cg84–Pg1–Ps101 | Cg84–Pg1–Ps102 | Cg84–Pg1–Ps103 | Cg84–Pg1–Ps104 |
| Cg84–Pg1–Ps105 | Cg84–Pg1–Ps106 | Cg84–Pg1–Ps107 | Cg84–Pg1–Ps108 |
| Cg84–Pg1–Ps109 | Cg84–Pg1–Ps110 | Cg84–Pg1–Ps111 | Cg84–Pg1–Ps112 |
| Cg84–Pg1–Ps113 | Cg84–Pg1–Ps114 | Cg84–Pg1–Ps115 | Cg84–Pg1–Ps116 |
| Cg84–Pg1–Ps117 | Cg84–Pg1–Ps118 | Cg84–Pg1–Ps119 | Cg84–Pg1–Ps120 |
| Cg84–Pg1–Ps121 | Cg84–Pg1–Ps122 | Cg84–Pg1–Ps123 | Cg84–Pg1–Ps124 |
| Cg84–Pg1–Ps125 | Cg84–Pg1–Ps126 | Cg84–Pg1–Ps127 | Cg84–Pg1–Ps128 |
| Cg84–Pg1–Ps129 | Cg84–Pg1–Ps130 | Cg84–Pg1–Ps131 | Cg84–Pg1–Ps132 |
| Cg84–Pg1–Ps133 | Cg84–Pg1–Ps134 | Cg84–Pg1–Ps135 | Cg84–Pg1–Ps136 |
| Cg84–Pg1–Ps137 | Cg84–Pg1–Ps138 | Cg84–Pg1–Ps139 | Cg84–Pg1–Ps140 |
| Cg84–Pg1–Ps141 | Cg84–Pg1–Ps142 | Cg84–Pg1–Ps143 | Cg84–Pg1–Ps144 |
| Cg84–Pg1–Ps145 | Cg84–Pg1–Ps146 | Cg84–Pg1–Ps147 | Cg84–Pg1–Ps148 |
| Cg84–Pg1–Ps149 | Cg84–Pg1–Ps150 | Cg84–Pg1–Ps151 | Cg84–Pg1–Ps152 |

| | | | |
|---|---|---|---|
| Cg84–Pg1–Ps153 | Cg84–Pg1–Ps154 | Cg84–Pg1–Ps155 | Cg84–Pg1–Ps156 |
| Cg84–Pg1–Ps157 | Cg84–Pg1–Ps158 | Cg84–Pg1–Ps159 | Cg84–Pg1–Ps160 |
| Cg84–Pg1–Ps161 | Cg84–Pg1–Ps162 | Cg84–Pg1–Ps163 | Cg84–Pg1–Ps164 |
| Cg84–Pg1–Ps165 | Cg84–Pg1–Ps166 | Cg84–Pg1–Ps167 | Cg84–Pg1–Ps168 |
| Cg84–Pg1–Ps169 | Cg84–Pg1–Ps170 | Cg84–Pg1–Ps171 | Cg84–Pg1–Ps172 |
| Cg84–Pg1–Ps173 | Cg84–Pg1–Ps174 | Cg84–Pg1–Ps175 | Cg84–Pg1–Ps176 |
| Cg84–Pg1–Ps177 | Cg84–Pg1–Ps178 | Cg84–Pg1–Ps179 | Cg84–Pg1–Ps180 |
| Cg84–Pg1–Ps181 | Cg84–Pg1–Ps182 | Cg84–Pg1–Ps183 | Cg84–Pg1–Ps184 |
| Cg84–Pg1–Ps185 | Cg84–Pg1–Ps186 | Cg84–Pg1–Ps187 | Cg84–Pg1–Ps188 |
| Cg84–Pg1–Ps189 | Cg84–Pg1–Ps190 | Cg84–Pg1–Ps191 | Cg84–Pg1–Ps192 |
| Cg84–Pg1–Ps193 | Cg84–Pg1–Ps194 | Cg84–Pg1–Ps195 | Cg84–Pg1–Ps196 |
| Cg84–Pg1–Ps197 | Cg84–Pg1–Ps198 | Cg84–Pg1–Ps199 | Cg84–Pg1–Ps200 |
| Cg84–Pg1–Ps201 | Cg84–Pg1–Ps202 | Cg84–Pg1–Ps203 | Cg84–Pg1–Ps204 |
| Cg84–Pg1–Ps205 | Cg84–Pg1–Ps206 | Cg84–Pg1–Ps207 | Cg84–Pg1–Ps208 |
| Cg84–Pg1–Ps209 | Cg84–Pg1–Ps210 | Cg84–Pg1–Ps211 | Cg84–Pg1–Ps212 |
| Cg84–Pg1–Ps213 | Cg84–Pg1–Ps214 | Cg84–Pg1–Ps215 | Cg84–Pg1–Ps216 |
| Cg84–Pg1–Ps217 | Cg84–Pg1–Ps218 | Cg84–Pg1–Ps219 | Cg84–Pg1–Ps220 |
| Cg84–Pg1–Ps221 | Cg84–Pg1–Ps222 | Cg84–Pg1–Ps223 | Cg84–Pg1–Ps224 |
| Cg84–Pg1–Ps225 | Cg84–Pg1–Ps226 | Cg84–Pg1–Ps227 | Cg84–Pg1–Ps228 |
| Cg84–Pg1–Ps229 | Cg84–Pg1–Ps230 | Cg84–Pg1–Ps231 | Cg84–Pg1–Ps232 |
| Cg84–Pg1–Ps233 | Cg84–Pg1–Ps234 | Cg84–Pg1–Ps235 | Cg84–Pg1–Ps236 |
| Cg84–Pg1–Ps237 | Cg84–Pg1–Ps238 | Cg84–Pg1–Ps239 | Cg84–Pg1–Ps240 |
| Cg84–Pg1–Ps241 | Cg84–Pg1–Ps242 | Cg84–Pg1–Ps243 | |

| | | | |
|---|---|---|---|
| Cg85–Pg1–Ps1 | Cg85–Pg1–Ps2 | Cg85–Pg1–Ps3 | Cg85–Pg1–Ps4 |
| Cg85–Pg1–Ps5 | Cg85–Pg1–Ps6 | Cg85–Pg1–Ps7 | Cg85–Pg1–Ps8 |
| Cg85–Pg1–Ps9 | Cg85–Pg1–Ps10 | Cg85–Pg1–Ps11 | Cg85–Pg1–Ps12 |
| Cg85–Pg1–Ps13 | Cg85–Pg1–Ps14 | Cg85–Pg1–Ps15 | Cg85–Pg1–Ps16 |
| Cg85–Pg1–Ps17 | Cg85–Pg1–Ps18 | Cg85–Pg1–Ps19 | Cg85–Pg1–Ps20 |
| Cg85–Pg1–Ps21 | Cg85–Pg1–Ps22 | Cg85–Pg1–Ps23 | Cg85–Pg1–Ps24 |
| Cg85–Pg1–Ps25 | Cg85–Pg1–Ps26 | Cg85–Pg1–Ps27 | Cg85–Pg1–Ps28 |
| Cg85–Pg1–Ps29 | Cg85–Pg1–Ps30 | Cg85–Pg1–Ps31 | Cg85–Pg1–Ps32 |
| Cg85–Pg1–Ps33 | Cg85–Pg1–Ps34 | Cg85–Pg1–Ps35 | Cg85–Pg1–Ps36 |
| Cg85–Pg1–Ps37 | Cg85–Pg1–Ps38 | Cg85–Pg1–Ps39 | Cg85–Pg1–Ps40 |
| Cg85–Pg1–Ps41 | Cg85–Pg1–Ps42 | Cg85–Pg1–Ps43 | Cg85–Pg1–Ps44 |
| Cg85–Pg1–Ps45 | Cg85–Pg1–Ps46 | Cg85–Pg1–Ps47 | Cg85–Pg1–Ps48 |
| Cg85–Pg1–Ps49 | Cg85–Pg1–Ps50 | Cg85–Pg1–Ps51 | Cg85–Pg1–Ps52 |
| Cg85–Pg1–Ps53 | Cg85–Pg1–Ps54 | Cg85–Pg1–Ps55 | Cg85–Pg1–Ps56 |
| Cg85–Pg1–Ps57 | Cg85–Pg1–Ps58 | Cg85–Pg1–Ps59 | Cg85–Pg1–Ps60 |
| Cg85–Pg1–Ps61 | Cg85–Pg1–Ps62 | Cg85–Pg1–Ps63 | Cg85–Pg1–Ps64 |
| Cg85–Pg1–Ps65 | Cg85–Pg1–Ps66 | Cg85–Pg1–Ps67 | Cg85–Pg1–Ps68 |
| Cg85–Pg1–Ps69 | Cg85–Pg1–Ps70 | Cg85–Pg1–Ps71 | Cg85–Pg1–Ps72 |
| Cg85–Pg1–Ps73 | Cg85–Pg1–Ps74 | Cg85–Pg1–Ps75 | Cg85–Pg1–Ps76 |
| Cg85–Pg1–Ps77 | Cg85–Pg1–Ps78 | Cg85–Pg1–Ps79 | Cg85–Pg1–Ps80 |
| Cg85–Pg1–Ps81 | Cg85–Pg1–Ps82 | Cg85–Pg1–Ps83 | Cg85–Pg1–Ps84 |
| Cg85–Pg1–Ps85 | Cg85–Pg1–Ps86 | Cg85–Pg1–Ps87 | Cg85–Pg1–Ps88 |
| Cg85–Pg1–Ps89 | Cg85–Pg1–Ps90 | Cg85–Pg1–Ps91 | Cg85–Pg1–Ps92 |
| Cg85–Pg1–Ps93 | Cg85–Pg1–Ps94 | Cg85–Pg1–Ps95 | Cg85–Pg1–Ps96 |
| Cg85–Pg1–Ps97 | Cg85–Pg1–Ps98 | Cg85–Pg1–Ps99 | Cg85–Pg1–Ps100 |
| Cg85–Pg1–Ps101 | Cg85–Pg1–Ps102 | Cg85–Pg1–Ps103 | Cg85–Pg1–Ps104 |
| Cg85–Pg1–Ps105 | Cg85–Pg1–Ps106 | Cg85–Pg1–Ps107 | Cg85–Pg1–Ps108 |
| Cg85–Pg1–Ps109 | Cg85–Pg1–Ps110 | Cg85–Pg1–Ps111 | Cg85–Pg1–Ps112 |

| | | | |
|---|---|---|---|
| Cg85–Pg1–Ps113 | Cg85–Pg1–Ps114 | Cg85–Pg1–Ps115 | Cg85–Pg1–Ps116 |
| Cg85–Pg1–Ps117 | Cg85–Pg1–Ps118 | Cg85–Pg1–Ps119 | Cg85–Pg1–Ps120 |
| Cg85–Pg1–Ps121 | Cg85–Pg1–Ps122 | Cg85–Pg1–Ps123 | Cg85–Pg1–Ps124 |
| Cg85–Pg1–Ps125 | Cg85–Pg1–Ps126 | Cg85–Pg1–Ps127 | Cg85–Pg1–Ps128 |
| Cg85–Pg1–Ps129 | Cg85–Pg1–Ps130 | Cg85–Pg1–Ps131 | Cg85–Pg1–Ps132 |
| Cg85–Pg1–Ps133 | Cg85–Pg1–Ps134 | Cg85–Pg1–Ps135 | Cg85–Pg1–Ps136 |
| Cg85–Pg1–Ps137 | Cg85–Pg1–Ps138 | Cg85–Pg1–Ps139 | Cg85–Pg1–Ps140 |
| Cg85–Pg1–Ps141 | Cg85–Pg1–Ps142 | Cg85–Pg1–Ps143 | Cg85–Pg1–Ps144 |
| Cg85–Pg1–Ps145 | Cg85–Pg1–Ps146 | Cg85–Pg1–Ps147 | Cg85–Pg1–Ps148 |
| Cg85–Pg1–Ps149 | Cg85–Pg1–Ps150 | Cg85–Pg1–Ps151 | Cg85–Pg1–Ps152 |
| Cg85–Pg1–Ps153 | Cg85–Pg1–Ps154 | Cg85–Pg1–Ps155 | Cg85–Pg1–Ps156 |
| Cg85–Pg1–Ps157 | Cg85–Pg1–Ps158 | Cg85–Pg1–Ps159 | Cg85–Pg1–Ps160 |
| Cg85–Pg1–Ps161 | Cg85–Pg1–Ps162 | Cg85–Pg1–Ps163 | Cg85–Pg1–Ps164 |
| Cg85–Pg1–Ps165 | Cg85–Pg1–Ps166 | Cg85–Pg1–Ps167 | Cg85–Pg1–Ps168 |
| Cg85–Pg1–Ps169 | Cg85–Pg1–Ps170 | Cg85–Pg1–Ps171 | Cg85–Pg1–Ps172 |
| Cg85–Pg1–Ps173 | Cg85–Pg1–Ps174 | Cg85–Pg1–Ps175 | Cg85–Pg1–Ps176 |
| Cg85–Pg1–Ps177 | Cg85–Pg1–Ps178 | Cg85–Pg1–Ps179 | Cg85–Pg1–Ps180 |
| Cg85–Pg1–Ps181 | Cg85–Pg1–Ps182 | Cg85–Pg1–Ps183 | Cg85–Pg1–Ps184 |
| Cg85–Pg1–Ps185 | Cg85–Pg1–Ps186 | Cg85–Pg1–Ps187 | Cg85–Pg1–Ps188 |
| Cg85–Pg1–Ps189 | Cg85–Pg1–Ps190 | Cg85–Pg1–Ps191 | Cg85–Pg1–Ps192 |
| Cg85–Pg1–Ps193 | Cg85–Pg1–Ps194 | Cg85–Pg1–Ps195 | Cg85–Pg1–Ps196 |
| Cg85–Pg1–Ps197 | Cg85–Pg1–Ps198 | Cg85–Pg1–Ps199 | Cg85–Pg1–Ps200 |
| Cg85–Pg1–Ps201 | Cg85–Pg1–Ps202 | Cg85–Pg1–Ps203 | Cg85–Pg1–Ps204 |
| Cg85–Pg1–Ps205 | Cg85–Pg1–Ps206 | Cg85–Pg1–Ps207 | Cg85–Pg1–Ps208 |
| Cg85–Pg1–Ps209 | Cg85–Pg1–Ps210 | Cg85–Pg1–Ps211 | Cg85–Pg1–Ps212 |
| Cg85–Pg1–Ps213 | Cg85–Pg1–Ps214 | Cg85–Pg1–Ps215 | Cg85–Pg1–Ps216 |
| Cg85–Pg1–Ps217 | Cg85–Pg1–Ps218 | Cg85–Pg1–Ps219 | Cg85–Pg1–Ps220 |
| Cg85–Pg1–Ps221 | Cg85–Pg1–Ps222 | Cg85–Pg1–Ps223 | Cg85–Pg1–Ps224 |
| Cg85–Pg1–Ps225 | Cg85–Pg1–Ps226 | Cg85–Pg1–Ps227 | Cg85–Pg1–Ps228 |
| Cg85–Pg1–Ps229 | Cg85–Pg1–Ps230 | Cg85–Pg1–Ps231 | Cg85–Pg1–Ps232 |
| Cg85–Pg1–Ps233 | Cg85–Pg1–Ps234 | Cg85–Pg1–Ps235 | Cg85–Pg1–Ps236 |
| Cg85–Pg1–Ps237 | Cg85–Pg1–Ps238 | Cg85–Pg1–Ps239 | Cg85–Pg1–Ps240 |
| Cg85–Pg1–Ps241 | Cg85–Pg1–Ps242 | Cg85–Pg1–Ps243 | |

| | | | |
|---|---|---|---|
| Cg86–Pg1–Ps1 | Cg86–Pg1–Ps2 | Cg86–Pg1–Ps3 | Cg86–Pg1–Ps4 |
| Cg86–Pg1–Ps5 | Cg86–Pg1–Ps6 | Cg86–Pg1–Ps7 | Cg86–Pg1–Ps8 |
| Cg86–Pg1–Ps9 | Cg86–Pg1–Ps10 | Cg86–Pg1–Ps11 | Cg86–Pg1–Ps12 |
| Cg86–Pg1–Ps13 | Cg86–Pg1–Ps14 | Cg86–Pg1–Ps15 | Cg86–Pg1–Ps16 |
| Cg86–Pg1–Ps17 | Cg86–Pg1–Ps18 | Cg86–Pg1–Ps19 | Cg86–Pg1–Ps20 |
| Cg86–Pg1–Ps21 | Cg86–Pg1–Ps22 | Cg86–Pg1–Ps23 | Cg86–Pg1–Ps24 |
| Cg86–Pg1–Ps25 | Cg86–Pg1–Ps26 | Cg86–Pg1–Ps27 | Cg86–Pg1–Ps28 |
| Cg86–Pg1–Ps29 | Cg86–Pg1–Ps30 | Cg86–Pg1–Ps31 | Cg86–Pg1–Ps32 |
| Cg86–Pg1–Ps33 | Cg86–Pg1–Ps34 | Cg86–Pg1–Ps35 | Cg86–Pg1–Ps36 |
| Cg86–Pg1–Ps37 | Cg86–Pg1–Ps38 | Cg86–Pg1–Ps39 | Cg86–Pg1–Ps40 |
| Cg86–Pg1–Ps41 | Cg86–Pg1–Ps42 | Cg86–Pg1–Ps43 | Cg86–Pg1–Ps44 |
| Cg86–Pg1–Ps45 | Cg86–Pg1–Ps46 | Cg86–Pg1–Ps47 | Cg86–Pg1–Ps48 |
| Cg86–Pg1–Ps49 | Cg86–Pg1–Ps50 | Cg86–Pg1–Ps51 | Cg86–Pg1–Ps52 |
| Cg86–Pg1–Ps53 | Cg86–Pg1–Ps54 | Cg86–Pg1–Ps55 | Cg86–Pg1–Ps56 |
| Cg86–Pg1–Ps57 | Cg86–Pg1–Ps58 | Cg86–Pg1–Ps59 | Cg86–Pg1–Ps60 |
| Cg86–Pg1–Ps61 | Cg86–Pg1–Ps62 | Cg86–Pg1–Ps63 | Cg86–Pg1–Ps64 |
| Cg86–Pg1–Ps65 | Cg86–Pg1–Ps66 | Cg86–Pg1–Ps67 | Cg86–Pg1–Ps68 |
| Cg86–Pg1–Ps69 | Cg86–Pg1–Ps70 | Cg86–Pg1–Ps71 | Cg86–Pg1–Ps72 |

| | | | |
|---|---|---|---|
| Cg86–Pg1–Ps73 | Cg86–Pg1–Ps74 | Cg86–Pg1–Ps75 | Cg86–Pg1–Ps76 |
| Cg86–Pg1–Ps77 | Cg86–Pg1–Ps78 | Cg86–Pg1–Ps79 | Cg86–Pg1–Ps80 |
| Cg86–Pg1–Ps81 | Cg86–Pg1–Ps82 | Cg86–Pg1–Ps83 | Cg86–Pg1–Ps84 |
| Cg86–Pg1–Ps85 | Cg86–Pg1–Ps86 | Cg86–Pg1–Ps87 | Cg86–Pg1–Ps88 |
| Cg86–Pg1–Ps89 | Cg86–Pg1–Ps90 | Cg86–Pg1–Ps91 | Cg86–Pg1–Ps92 |
| Cg86–Pg1–Ps93 | Cg86–Pg1–Ps94 | Cg86–Pg1–Ps95 | Cg86–Pg1–Ps96 |
| Cg86–Pg1–Ps97 | Cg86–Pg1–Ps98 | Cg86–Pg1–Ps99 | Cg86–Pg1–Ps100 |
| Cg86–Pg1–Ps101 | Cg86–Pg1–Ps102 | Cg86–Pg1–Ps103 | Cg86–Pg1–Ps104 |
| Cg86–Pg1–Ps105 | Cg86–Pg1–Ps106 | Cg86–Pg1–Ps107 | Cg86–Pg1–Ps108 |
| Cg86–Pg1–Ps109 | Cg86–Pg1–Ps110 | Cg86–Pg1–Ps111 | Cg86–Pg1–Ps112 |
| Cg86–Pg1–Ps113 | Cg86–Pg1–Ps114 | Cg86–Pg1–Ps115 | Cg86–Pg1–Ps116 |
| Cg86–Pg1–Ps117 | Cg86–Pg1–Ps118 | Cg86–Pg1–Ps119 | Cg86–Pg1–Ps120 |
| Cg86–Pg1–Ps121 | Cg86–Pg1–Ps122 | Cg86–Pg1–Ps123 | Cg86–Pg1–Ps124 |
| Cg86–Pg1–Ps125 | Cg86–Pg1–Ps126 | Cg86–Pg1–Ps127 | Cg86–Pg1–Ps128 |
| Cg86–Pg1–Ps129 | Cg86–Pg1–Ps130 | Cg86–Pg1–Ps131 | Cg86–Pg1–Ps132 |
| Cg86–Pg1–Ps133 | Cg86–Pg1–Ps134 | Cg86–Pg1–Ps135 | Cg86–Pg1–Ps136 |
| Cg86–Pg1–Ps137 | Cg86–Pg1–Ps138 | Cg86–Pg1–Ps139 | Cg86–Pg1–Ps140 |
| Cg86–Pg1–Ps141 | Cg86–Pg1–Ps142 | Cg86–Pg1–Ps143 | Cg86–Pg1–Ps144 |
| Cg86–Pg1–Ps145 | Cg86–Pg1–Ps146 | Cg86–Pg1–Ps147 | Cg86–Pg1–Ps148 |
| Cg86–Pg1–Ps149 | Cg86–Pg1–Ps150 | Cg86–Pg1–Ps151 | Cg86–Pg1–Ps152 |
| Cg86–Pg1–Ps153 | Cg86–Pg1–Ps154 | Cg86–Pg1–Ps155 | Cg86–Pg1–Ps156 |
| Cg86–Pg1–Ps157 | Cg86–Pg1–Ps158 | Cg86–Pg1–Ps159 | Cg86–Pg1–Ps160 |
| Cg86–Pg1–Ps161 | Cg86–Pg1–Ps162 | Cg86–Pg1–Ps163 | Cg86–Pg1–Ps164 |
| Cg86–Pg1–Ps165 | Cg86–Pg1–Ps166 | Cg86–Pg1–Ps167 | Cg86–Pg1–Ps168 |
| Cg86–Pg1–Ps169 | Cg86–Pg1–Ps170 | Cg86–Pg1–Ps171 | Cg86–Pg1–Ps172 |
| Cg86–Pg1–Ps173 | Cg86–Pg1–Ps174 | Cg86–Pg1–Ps175 | Cg86–Pg1–Ps176 |
| Cg86–Pg1–Ps177 | Cg86–Pg1–Ps178 | Cg86–Pg1–Ps179 | Cg86–Pg1–Ps180 |
| Cg86–Pg1–Ps181 | Cg86–Pg1–Ps182 | Cg86–Pg1–Ps183 | Cg86–Pg1–Ps184 |
| Cg86–Pg1–Ps185 | Cg86–Pg1–Ps186 | Cg86–Pg1–Ps187 | Cg86–Pg1–Ps188 |
| Cg86–Pg1–Ps189 | Cg86–Pg1–Ps190 | Cg86–Pg1–Ps191 | Cg86–Pg1–Ps192 |
| Cg86–Pg1–Ps193 | Cg86–Pg1–Ps194 | Cg86–Pg1–Ps195 | Cg86–Pg1–Ps196 |
| Cg86–Pg1–Ps197 | Cg86–Pg1–Ps198 | Cg86–Pg1–Ps199 | Cg86–Pg1–Ps200 |
| Cg86–Pg1–Ps201 | Cg86–Pg1–Ps202 | Cg86–Pg1–Ps203 | Cg86–Pg1–Ps204 |
| Cg86–Pg1–Ps205 | Cg86–Pg1–Ps206 | Cg86–Pg1–Ps207 | Cg86–Pg1–Ps208 |
| Cg86–Pg1–Ps209 | Cg86–Pg1–Ps210 | Cg86–Pg1–Ps211 | Cg86–Pg1–Ps212 |
| Cg86–Pg1–Ps213 | Cg86–Pg1–Ps214 | Cg86–Pg1–Ps215 | Cg86–Pg1–Ps216 |
| Cg86–Pg1–Ps217 | Cg86–Pg1–Ps218 | Cg86–Pg1–Ps219 | Cg86–Pg1–Ps220 |
| Cg86–Pg1–Ps221 | Cg86–Pg1–Ps222 | Cg86–Pg1–Ps223 | Cg86–Pg1–Ps224 |
| Cg86–Pg1–Ps225 | Cg86–Pg1–Ps226 | Cg86–Pg1–Ps227 | Cg86–Pg1–Ps228 |
| Cg86–Pg1–Ps229 | Cg86–Pg1–Ps230 | Cg86–Pg1–Ps231 | Cg86–Pg1–Ps232 |
| Cg86–Pg1–Ps233 | Cg86–Pg1–Ps234 | Cg86–Pg1–Ps235 | Cg86–Pg1–Ps236 |
| Cg86–Pg1–Ps237 | Cg86–Pg1–Ps238 | Cg86–Pg1–Ps239 | Cg86–Pg1–Ps240 |
| Cg86–Pg1–Ps241 | Cg86–Pg1–Ps242 | Cg86–Pg1–Ps243 | |

| | | | |
|---|---|---|---|
| Cg87–Pg1–Ps1 | Cg87–Pg1–Ps2 | Cg87–Pg1–Ps3 | Cg87–Pg1–Ps4 |
| Cg87–Pg1–Ps5 | Cg87–Pg1–Ps6 | Cg87–Pg1–Ps7 | Cg87–Pg1–Ps8 |
| Cg87–Pg1–Ps9 | Cg87–Pg1–Ps10 | Cg87–Pg1–Ps11 | Cg87–Pg1–Ps12 |
| Cg87–Pg1–Ps13 | Cg87–Pg1–Ps14 | Cg87–Pg1–Ps15 | Cg87–Pg1–Ps16 |
| Cg87–Pg1–Ps17 | Cg87–Pg1–Ps18 | Cg87–Pg1–Ps19 | Cg87–Pg1–Ps20 |
| Cg87–Pg1–Ps21 | Cg87–Pg1–Ps22 | Cg87–Pg1–Ps23 | Cg87–Pg1–Ps24 |
| Cg87–Pg1–Ps25 | Cg87–Pg1–Ps26 | Cg87–Pg1–Ps27 | Cg87–Pg1–Ps28 |
| Cg87–Pg1–Ps29 | Cg87–Pg1–Ps30 | Cg87–Pg1–Ps31 | Cg87–Pg1–Ps32 |

| | | | |
|---|---|---|---|
| Cg87–Pg1–Ps33 | Cg87–Pg1–Ps34 | Cg87–Pg1–Ps35 | Cg87–Pg1–Ps36 |
| Cg87–Pg1–Ps37 | Cg87–Pg1–Ps38 | Cg87–Pg1–Ps39 | Cg87–Pg1–Ps40 |
| Cg87–Pg1–Ps41 | Cg87–Pg1–Ps42 | Cg87–Pg1–Ps43 | Cg87–Pg1–Ps44 |
| Cg87–Pg1–Ps45 | Cg87–Pg1–Ps46 | Cg87–Pg1–Ps47 | Cg87–Pg1–Ps48 |
| Cg87–Pg1–Ps49 | Cg87–Pg1–Ps50 | Cg87–Pg1–Ps51 | Cg87–Pg1–Ps52 |
| Cg87–Pg1–Ps53 | Cg87–Pg1–Ps54 | Cg87–Pg1–Ps55 | Cg87–Pg1–Ps56 |
| Cg87–Pg1–Ps57 | Cg87–Pg1–Ps58 | Cg87–Pg1–Ps59 | Cg87–Pg1–Ps60 |
| Cg87–Pg1–Ps61 | Cg87–Pg1–Ps62 | Cg87–Pg1–Ps63 | Cg87–Pg1–Ps64 |
| Cg87–Pg1–Ps65 | Cg87–Pg1–Ps66 | Cg87–Pg1–Ps67 | Cg87–Pg1–Ps68 |
| Cg87–Pg1–Ps69 | Cg87–Pg1–Ps70 | Cg87–Pg1–Ps71 | Cg87–Pg1–Ps72 |
| Cg87–Pg1–Ps73 | Cg87–Pg1–Ps74 | Cg87–Pg1–Ps75 | Cg87–Pg1–Ps76 |
| Cg87–Pg1–Ps77 | Cg87–Pg1–Ps78 | Cg87–Pg1–Ps79 | Cg87–Pg1–Ps80 |
| Cg87–Pg1–Ps81 | Cg87–Pg1–Ps82 | Cg87–Pg1–Ps83 | Cg87–Pg1–Ps84 |
| Cg87–Pg1–Ps85 | Cg87–Pg1–Ps86 | Cg87–Pg1–Ps87 | Cg87–Pg1–Ps88 |
| Cg87–Pg1–Ps89 | Cg87–Pg1–Ps90 | Cg87–Pg1–Ps91 | Cg87–Pg1–Ps92 |
| Cg87–Pg1–Ps93 | Cg87–Pg1–Ps94 | Cg87–Pg1–Ps95 | Cg87–Pg1–Ps96 |
| Cg87–Pg1–Ps97 | Cg87–Pg1–Ps98 | Cg87–Pg1–Ps99 | Cg87–Pg1–Ps100 |
| Cg87–Pg1–Ps101 | Cg87–Pg1–Ps102 | Cg87–Pg1–Ps103 | Cg87–Pg1–Ps104 |
| Cg87–Pg1–Ps105 | Cg87–Pg1–Ps106 | Cg87–Pg1–Ps107 | Cg87–Pg1–Ps108 |
| Cg87–Pg1–Ps109 | Cg87–Pg1–Ps110 | Cg87–Pg1–Ps111 | Cg87–Pg1–Ps112 |
| Cg87–Pg1–Ps113 | Cg87–Pg1–Ps114 | Cg87–Pg1–Ps115 | Cg87–Pg1–Ps116 |
| Cg87–Pg1–Ps117 | Cg87–Pg1–Ps118 | Cg87–Pg1–Ps119 | Cg87–Pg1–Ps120 |
| Cg87–Pg1–Ps121 | Cg87–Pg1–Ps122 | Cg87–Pg1–Ps123 | Cg87–Pg1–Ps124 |
| Cg87–Pg1–Ps125 | Cg87–Pg1–Ps126 | Cg87–Pg1–Ps127 | Cg87–Pg1–Ps128 |
| Cg87–Pg1–Ps129 | Cg87–Pg1–Ps130 | Cg87–Pg1–Ps131 | Cg87–Pg1–Ps132 |
| Cg87–Pg1–Ps133 | Cg87–Pg1–Ps134 | Cg87–Pg1–Ps135 | Cg87–Pg1–Ps136 |
| Cg87–Pg1–Ps137 | Cg87–Pg1–Ps138 | Cg87–Pg1–Ps139 | Cg87–Pg1–Ps140 |
| Cg87–Pg1–Ps141 | Cg87–Pg1–Ps142 | Cg87–Pg1–Ps143 | Cg87–Pg1–Ps144 |
| Cg87–Pg1–Ps145 | Cg87–Pg1–Ps146 | Cg87–Pg1–Ps147 | Cg87–Pg1–Ps148 |
| Cg87–Pg1–Ps149 | Cg87–Pg1–Ps150 | Cg87–Pg1–Ps151 | Cg87–Pg1–Ps152 |
| Cg87–Pg1–Ps153 | Cg87–Pg1–Ps154 | Cg87–Pg1–Ps155 | Cg87–Pg1–Ps156 |
| Cg87–Pg1–Ps157 | Cg87–Pg1–Ps158 | Cg87–Pg1–Ps159 | Cg87–Pg1–Ps160 |
| Cg87–Pg1–Ps161 | Cg87–Pg1–Ps162 | Cg87–Pg1–Ps163 | Cg87–Pg1–Ps164 |
| Cg87–Pg1–Ps165 | Cg87–Pg1–Ps166 | Cg87–Pg1–Ps167 | Cg87–Pg1–Ps168 |
| Cg87–Pg1–Ps169 | Cg87–Pg1–Ps170 | Cg87–Pg1–Ps171 | Cg87–Pg1–Ps172 |
| Cg87–Pg1–Ps173 | Cg87–Pg1–Ps174 | Cg87–Pg1–Ps175 | Cg87–Pg1–Ps176 |
| Cg87–Pg1–Ps177 | Cg87–Pg1–Ps178 | Cg87–Pg1–Ps179 | Cg87–Pg1–Ps180 |
| Cg87–Pg1–Ps181 | Cg87–Pg1–Ps182 | Cg87–Pg1–Ps183 | Cg87–Pg1–Ps184 |
| Cg87–Pg1–Ps185 | Cg87–Pg1–Ps186 | Cg87–Pg1–Ps187 | Cg87–Pg1–Ps188 |
| Cg87–Pg1–Ps189 | Cg87–Pg1–Ps190 | Cg87–Pg1–Ps191 | Cg87–Pg1–Ps192 |
| Cg87–Pg1–Ps193 | Cg87–Pg1–Ps194 | Cg87–Pg1–Ps195 | Cg87–Pg1–Ps196 |
| Cg87–Pg1–Ps197 | Cg87–Pg1–Ps198 | Cg87–Pg1–Ps199 | Cg87–Pg1–Ps200 |
| Cg87–Pg1–Ps201 | Cg87–Pg1–Ps202 | Cg87–Pg1–Ps203 | Cg87–Pg1–Ps204 |
| Cg87–Pg1–Ps205 | Cg87–Pg1–Ps206 | Cg87–Pg1–Ps207 | Cg87–Pg1–Ps208 |
| Cg87–Pg1–Ps209 | Cg87–Pg1–Ps210 | Cg87–Pg1–Ps211 | Cg87–Pg1–Ps212 |
| Cg87–Pg1–Ps213 | Cg87–Pg1–Ps214 | Cg87–Pg1–Ps215 | Cg87–Pg1–Ps216 |
| Cg87–Pg1–Ps217 | Cg87–Pg1–Ps218 | Cg87–Pg1–Ps219 | Cg87–Pg1–Ps220 |
| Cg87–Pg1–Ps221 | Cg87–Pg1–Ps222 | Cg87–Pg1–Ps223 | Cg87–Pg1–Ps224 |
| Cg87–Pg1–Ps225 | Cg87–Pg1–Ps226 | Cg87–Pg1–Ps227 | Cg87–Pg1–Ps228 |
| Cg87–Pg1–Ps229 | Cg87–Pg1–Ps230 | Cg87–Pg1–Ps231 | Cg87–Pg1–Ps232 |
| Cg87–Pg1–Ps233 | Cg87–Pg1–Ps234 | Cg87–Pg1–Ps235 | Cg87–Pg1–Ps236 |
| Cg87–Pg1–Ps237 | Cg87–Pg1–Ps238 | Cg87–Pg1–Ps239 | Cg87–Pg1–Ps240 |

| | | | |
|---|---|---|---|
| Cg87–Pg1–Ps241 | Cg87–Pg1–Ps242 | Cg87–Pg1–Ps243 | |

| | | | |
|---|---|---|---|
| Cg88–Pg1–Ps1 | Cg88–Pg1–Ps2 | Cg88–Pg1–Ps3 | Cg88–Pg1–Ps4 |
| Cg88–Pg1–Ps5 | Cg88–Pg1–Ps6 | Cg88–Pg1–Ps7 | Cg88–Pg1–Ps8 |
| Cg88–Pg1–Ps9 | Cg88–Pg1–Ps10 | Cg88–Pg1–Ps11 | Cg88–Pg1–Ps12 |
| Cg88–Pg1–Ps13 | Cg88–Pg1–Ps14 | Cg88–Pg1–Ps15 | Cg88–Pg1–Ps16 |
| Cg88–Pg1–Ps17 | Cg88–Pg1–Ps18 | Cg88–Pg1–Ps19 | Cg88–Pg1–Ps20 |
| Cg88–Pg1–Ps21 | Cg88–Pg1–Ps22 | Cg88–Pg1–Ps23 | Cg88–Pg1–Ps24 |
| Cg88–Pg1–Ps25 | Cg88–Pg1–Ps26 | Cg88–Pg1–Ps27 | Cg88–Pg1–Ps28 |
| Cg88–Pg1–Ps29 | Cg88–Pg1–Ps30 | Cg88–Pg1–Ps31 | Cg88–Pg1–Ps32 |
| Cg88–Pg1–Ps33 | Cg88–Pg1–Ps34 | Cg88–Pg1–Ps35 | Cg88–Pg1–Ps36 |
| Cg88–Pg1–Ps37 | Cg88–Pg1–Ps38 | Cg88–Pg1–Ps39 | Cg88–Pg1–Ps40 |
| Cg88–Pg1–Ps41 | Cg88–Pg1–Ps42 | Cg88–Pg1–Ps43 | Cg88–Pg1–Ps44 |
| Cg88–Pg1–Ps45 | Cg88–Pg1–Ps46 | Cg88–Pg1–Ps47 | Cg88–Pg1–Ps48 |
| Cg88–Pg1–Ps49 | Cg88–Pg1–Ps50 | Cg88–Pg1–Ps51 | Cg88–Pg1–Ps52 |
| Cg88–Pg1–Ps53 | Cg88–Pg1–Ps54 | Cg88–Pg1–Ps55 | Cg88–Pg1–Ps56 |
| Cg88–Pg1–Ps57 | Cg88–Pg1–Ps58 | Cg88–Pg1–Ps59 | Cg88–Pg1–Ps60 |
| Cg88–Pg1–Ps61 | Cg88–Pg1–Ps62 | Cg88–Pg1–Ps63 | Cg88–Pg1–Ps64 |
| Cg88–Pg1–Ps65 | Cg88–Pg1–Ps66 | Cg88–Pg1–Ps67 | Cg88–Pg1–Ps68 |
| Cg88–Pg1–Ps69 | Cg88–Pg1–Ps70 | Cg88–Pg1–Ps71 | Cg88–Pg1–Ps72 |
| Cg88–Pg1–Ps73 | Cg88–Pg1–Ps74 | Cg88–Pg1–Ps75 | Cg88–Pg1–Ps76 |
| Cg88–Pg1–Ps77 | Cg88–Pg1–Ps78 | Cg88–Pg1–Ps79 | Cg88–Pg1–Ps80 |
| Cg88–Pg1–Ps81 | Cg88–Pg1–Ps82 | Cg88–Pg1–Ps83 | Cg88–Pg1–Ps84 |
| Cg88–Pg1–Ps85 | Cg88–Pg1–Ps86 | Cg88–Pg1–Ps87 | Cg88–Pg1–Ps88 |
| Cg88–Pg1–Ps89 | Cg88–Pg1–Ps90 | Cg88–Pg1–Ps91 | Cg88–Pg1–Ps92 |
| Cg88–Pg1–Ps93 | Cg88–Pg1–Ps94 | Cg88–Pg1–Ps95 | Cg88–Pg1–Ps96 |
| Cg88–Pg1–Ps97 | Cg88–Pg1–Ps98 | Cg88–Pg1–Ps99 | Cg88–Pg1–Ps100 |
| Cg88–Pg1–Ps101 | Cg88–Pg1–Ps102 | Cg88–Pg1–Ps103 | Cg88–Pg1–Ps104 |
| Cg88–Pg1–Ps105 | Cg88–Pg1–Ps106 | Cg88–Pg1–Ps107 | Cg88–Pg1–Ps108 |
| Cg88–Pg1–Ps109 | Cg88–Pg1–Ps110 | Cg88–Pg1–Ps111 | Cg88–Pg1–Ps112 |
| Cg88–Pg1–Ps113 | Cg88–Pg1–Ps114 | Cg88–Pg1–Ps115 | Cg88–Pg1–Ps116 |
| Cg88–Pg1–Ps117 | Cg88–Pg1–Ps118 | Cg88–Pg1–Ps119 | Cg88–Pg1–Ps120 |
| Cg88–Pg1–Ps121 | Cg88–Pg1–Ps122 | Cg88–Pg1–Ps123 | Cg88–Pg1–Ps124 |
| Cg88–Pg1–Ps125 | Cg88–Pg1–Ps126 | Cg88–Pg1–Ps127 | Cg88–Pg1–Ps128 |
| Cg88–Pg1–Ps129 | Cg88–Pg1–Ps130 | Cg88–Pg1–Ps131 | Cg88–Pg1–Ps132 |
| Cg88–Pg1–Ps133 | Cg88–Pg1–Ps134 | Cg88–Pg1–Ps135 | Cg88–Pg1–Ps136 |
| Cg88–Pg1–Ps137 | Cg88–Pg1–Ps138 | Cg88–Pg1–Ps139 | Cg88–Pg1–Ps140 |
| Cg88–Pg1–Ps141 | Cg88–Pg1–Ps142 | Cg88–Pg1–Ps143 | Cg88–Pg1–Ps144 |
| Cg88–Pg1–Ps145 | Cg88–Pg1–Ps146 | Cg88–Pg1–Ps147 | Cg88–Pg1–Ps148 |
| Cg88–Pg1–Ps149 | Cg88–Pg1–Ps150 | Cg88–Pg1–Ps151 | Cg88–Pg1–Ps152 |
| Cg88–Pg1–Ps153 | Cg88–Pg1–Ps154 | Cg88–Pg1–Ps155 | Cg88–Pg1–Ps156 |
| Cg88–Pg1–Ps157 | Cg88–Pg1–Ps158 | Cg88–Pg1–Ps159 | Cg88–Pg1–Ps160 |
| Cg88–Pg1–Ps161 | Cg88–Pg1–Ps162 | Cg88–Pg1–Ps163 | Cg88–Pg1–Ps164 |
| Cg88–Pg1–Ps165 | Cg88–Pg1–Ps166 | Cg88–Pg1–Ps167 | Cg88–Pg1–Ps168 |
| Cg88–Pg1–Ps169 | Cg88–Pg1–Ps170 | Cg88–Pg1–Ps171 | Cg88–Pg1–Ps172 |
| Cg88–Pg1–Ps173 | Cg88–Pg1–Ps174 | Cg88–Pg1–Ps175 | Cg88–Pg1–Ps176 |
| Cg88–Pg1–Ps177 | Cg88–Pg1–Ps178 | Cg88–Pg1–Ps179 | Cg88–Pg1–Ps180 |
| Cg88–Pg1–Ps181 | Cg88–Pg1–Ps182 | Cg88–Pg1–Ps183 | Cg88–Pg1–Ps184 |
| Cg88–Pg1–Ps185 | Cg88–Pg1–Ps186 | Cg88–Pg1–Ps187 | Cg88–Pg1–Ps188 |
| Cg88–Pg1–Ps189 | Cg88–Pg1–Ps190 | Cg88–Pg1–Ps191 | Cg88–Pg1–Ps192 |
| Cg88–Pg1–Ps193 | Cg88–Pg1–Ps194 | Cg88–Pg1–Ps195 | Cg88–Pg1–Ps196 |
| Cg88–Pg1–Ps197 | Cg88–Pg1–Ps198 | Cg88–Pg1–Ps199 | Cg88–Pg1–Ps200 |

| | | | |
|---|---|---|---|
| Cg88-Pg1-Ps201 | Cg88-Pg1-Ps202 | Cg88-Pg1-Ps203 | Cg88-Pg1-Ps204 |
| Cg88-Pg1-Ps205 | Cg88-Pg1-Ps206 | Cg88-Pg1-Ps207 | Cg88-Pg1-Ps208 |
| Cg88-Pg1-Ps209 | Cg88-Pg1-Ps210 | Cg88-Pg1-Ps211 | Cg88-Pg1-Ps212 |
| Cg88-Pg1-Ps213 | Cg88-Pg1-Ps214 | Cg88-Pg1-Ps215 | Cg88-Pg1-Ps216 |
| Cg88-Pg1-Ps217 | Cg88-Pg1-Ps218 | Cg88-Pg1-Ps219 | Cg88-Pg1-Ps220 |
| Cg88-Pg1-Ps221 | Cg88-Pg1-Ps222 | Cg88-Pg1-Ps223 | Cg88-Pg1-Ps224 |
| Cg88-Pg1-Ps225 | Cg88-Pg1-Ps226 | Cg88-Pg1-Ps227 | Cg88-Pg1-Ps228 |
| Cg88-Pg1-Ps229 | Cg88-Pg1-Ps230 | Cg88-Pg1-Ps231 | Cg88-Pg1-Ps232 |
| Cg88-Pg1-Ps233 | Cg88-Pg1-Ps234 | Cg88-Pg1-Ps235 | Cg88-Pg1-Ps236 |
| Cg88-Pg1-Ps237 | Cg88-Pg1-Ps238 | Cg88-Pg1-Ps239 | Cg88-Pg1-Ps240 |
| Cg88-Pg1-Ps241 | Cg88-Pg1-Ps242 | Cg88-Pg1-Ps243 | |

Abbreviations and symbols commonly used in the peptide and chemical arts are used herein to describe compounds of the present invention, following the general guidelines presented by the ICTPAC-IUB Joint Commission on Biochemical Nomenclature as described in Eur. J. Biochem., 158, 9-, 1984. Compounds of formula (I) and the intermediates and starting materials used in their preparation are named in accordance with the IUPAC rules of nomenclature in which the characteristic groups have decreasing priority for citation as the principle group. An example compound of formula (I), compound (1) in which Z is $CH_2$, $R^1$ is $R^2C(O)$, where $R^2$ is 2-pyridyl, $P_1$, $P_2$ are methylene, Y is 4-methylpentoyl, $(X)_0$ is zero, $(W)_n$ is NH, $(V)_m$ is C(O) and U is phenyl is thus named:-

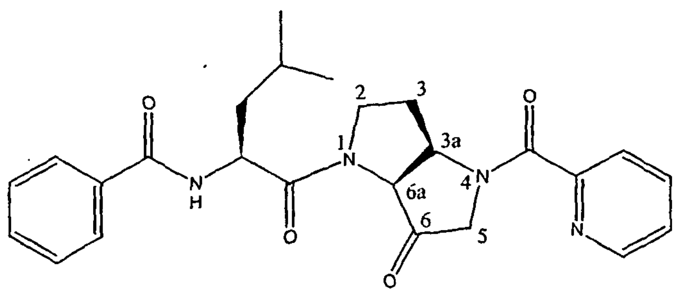

**(1)**

(3a*R*,6a*S*)-*N*-{(1*S*)-3-Methyl-1-(6-oxo-4-(pyridine-2-carbonyl)-hexahydropyrrolo [3,2-b]pyrrole-1-carbonyl]-butyl}-benzamide;

A second example compound of formula (I), compound (2) in which Z is $CH_2$, $R^1$ is $R^2C(O)$, where $R^2$ is 2-pyridyl, $P_1$, is methylene, $P_2$ is NH, Y is 4-methylpentoyl, $(X)_0$ is zero, $(W)_n$ is NH, $(V)_m$ is C(O) and U is phenyl is thus named:-

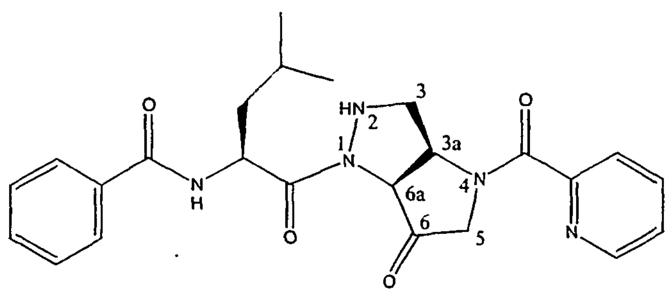

**(2)**

(3a*R*,6a*S*)-*N*-{(1*S*)-3-Methyl-1-[6-oxo-4-(pyridine-2-carbonyl)-hexahydropyrrolo [3,2-c]pyrazole-1-carbonyl]-butyl}-benzamide;

A third example compound of formula (I), compound **(3)** in which Z is $CH_2$, $R^1$ is $R^2C(O)$, where $R^2$ is 2-pyridyl, $P_1$, is methylene, $P_2$ is O, Y is 4-methylpentoyl, $(X)_0$ is zero, $(W)_n$ is NH, $(V)_m$ is C(O) and U is phenyl is thus named:-

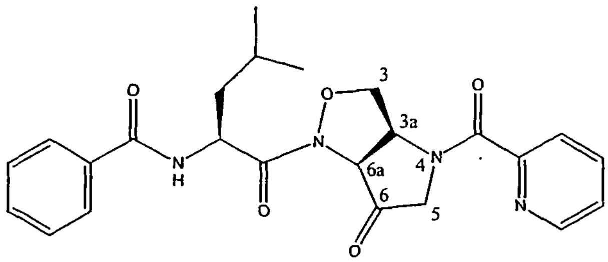

**(3)**

(3a*S*,6a*S*)-N-{(1*S*)-3-Methyl-1-[6-oxo-4-(pyridine-2-carbonyl)-hexahydro-2-oxa-1,4-diaza-pentalene-1-carbonyl]-butyl}-benzamide.

Compounds of the invention include, but are not limited to, the following examples where all 4 stereoisomeric combinations of the bicyclic ketone are included where $P_2$ is $CH_2$, i.e. (3a*S*, 6a*S*), (3a*R*, 6a*S*), (3a*S*, 6a*R*), (3a*R*, 6a*R*) and also included are the equivalent analogues where $P_2$ is O and NH. More preferred compounds consist of the cis-bicyclic isomers which, when $P_2$ is $CH_2$, are designated as (3a*R*, 6a*S*) and (3a*S*, 6a*R*) and also more preferred are the equivalent cis-bicyclic analogues where $P_2$ is O and NH.

4. Naphthalene-2-carboxylic acid {3-methyl-1-[6-oxo-4-(pyridine-2-sulfonyl)hexahydro-pyrrolo[3,2-*b*]pyrrole-1-carbonyl]-butyl}-amide;

5. Quinoline-2-carboxylic acid {3-methyl-1-[6-oxo-4-(pyridine-2-sulfonyl)-hexahydro-pyrrolo[3,2-*b*]pyrrole-1-carbonyl]-butyl}-amide;

6. Benzo[b]thiophene-2-carboxylic acid {3-methyl-1-[6-oxo-4-(pyridine-2-sulfonyl)-hexahydro-pyrrolo[3,2-*b*]pyrrole-1-carbonyl]-butyl}-amide;

7. Benzofuran-2-carboxylic acid {3-methyl-1-[6-oxo-4-(pyridine-2-sulfonyl)-hexahydro-pyrrolo[3,2-*b*]pyrrole-1-carbonyl]-butyl}-amide;

8. Biphenyl-4-carboxylic acid {3-methyl-1-[6-oxo-4-(pyridine-2-sulfonyl)-hexahydro-pyrrolo[3,2-*b*]pyrrole-1-carbonyl]-butyl}-amide;

9. 4-*tert*-Butyl-N-{3-methyl-1-[6-oxo-4-(pyridine-2-sulfonyl)-hexahydro-pyrrolo[3,2-*b*]pyrrole-1-carbonyl]-butyl}-benzamide;

10. 4-Dimethylamino-N-{3-methyl-1-[6-oxo-4-(pyrdine-2-sulfonyl)-hexahydro-pyrrolo[3,2-*b*]pyrrole-1-carbonyl]-butyl}-benzamide;

11. 7-Methoxy-benzofuran-2-carboxylic acid {3-methyl-1-[6-oxo-4-(pyridine-2-sulfonyl)-hexahydro-pyrrolo[3,2-*b*]pyrrole-1-carbonyl]-butyl}-amide;

12. {3-Methyl-1-[6-oxo-4-(pyridine-2-sulfonyl)-hexahydro-pyrrolo[3,2-*b*]pyrrole-1-carbonyl]-butyl}-carbamic acid benzyl ester;

13. 5-Methoxy-benzofuran-2-carboxylic acid {3-methyl-1-[6-oxo-4-(pyridine-2-sulfonyl)-hexahydro-pyrrolo[3,2-*b*]-pyrrole-1-carbonyl]-butyl}-amide;

14. Thieno[3,2-*b*]thiophene-2-carboxylic acid {3-methyl-1-[6-oxo-4-(pyridine-2-sulfonyl)-hexahydro-pyrrolo[3,2-*b*]-pyrrole-1-carbonyl]-butyl}-amide;

15. 3-Methyl-benzofuran-2-carboxylic acid {3-methyl-1-[6-oxo-4-(pyridine-2-sulfonyl)-hexahydro-pyrrolo[3,2-*b*]pyrrole-1-carbonyl]-butyl}-amide;

16. Quinoxaline-2-carboxylic acid {3-methyl-1-[6-oxo-4-(pyridine-2-sulfonyl)-hexahydro-pyrrolo[3,2-*b*]pyrrole-1-carbonyl]-butyl}-amide;

17. Benzo[1,3]dioxole-5-carboxylic acid {3-methyl-1-[6-oxo-4-(pyridine-2-sulfonyl)-hexahydro-pyrrolo[3,2-*b*]pyrrole-1-carbonyl]-butyl}-amide;

18. Quinoline-6-carboxylic acid {3-methyl-1-[6-oxo-4-(pyridine-2-sulfonyl)-hexahydro-pyrrolo[3,2-*b*]pyrrole-1-carbonyl]-butyl}-amide;

19. Furan-2-carboxylic acid {3-methyl-1-[6-oxo-4-(pyridine-2-sulfonyl)-hexahydro-pyrrolo[3,2-*b*]pyrrole-1-carbonyl]-butyl}-amide;

20. Thiophene-2-carboxylic acid {3-methyl-1-[6-oxo-4-(pyridine-2-sulfonyl)-hexahydro-pyrrolo[3,2-*b*]pyrrole-1-carbonyl]-butyl}-amide;

21. N-{3-Methyl-1-[6-oxo-4-(pyridine-2-sulfonyl)-hexahydro-pyrrolo[3,2-*b*]pyrrole-1-carbonyl]-butyl}-4-trifluoromethyl-benzamide;

22. Furan-3-carboxylic acid {3-methyl-1-[6-oxo-4-(pyridine-2-sulfonyl)-hexahydro-pyrrolo[3,2- *b*]pyrrole-1-carbonyl]-butyl}-amide;

23. Thiophene-3-carboxylic acid {3-methyl-1-[6-oxo-4-(pyridine-2-sulfonyl)-hexahydro-pyrrolo[3,2-*b*]pyrrole-1-carbonyl-butyl}-amide;

24. N-{3-Methyl-1-[6-oxo-4-(pyridine-2-sulfonyl)-hexahydro-pyrrolo[3,2-*b*]pyrrole-1-carbonyl]-butyl}-4-phenoxy-benzamide;

25. N-{3-Methyl-1-[6-oxo-4-(pyridine-2-sulfonyl)-hexahydro-pyrrolo[3,2-*b*]pyrrole-1-carbonyl]-butyl}-4-(4-methyl-piperazin-1-yl)-benzamide;

26. 4-Methyl-N-{3-methyl-1-[6-oxo-4-(pyridine-2-sulfonyl)-hexahydro-pyrrolo[3,2-*b*]pyrrole-1-carbonyl]-butyl}-benzamide;

27. 4-Methoxy-N-{3-methyl-1-[6-oxo-4-(pyridine-2-sulfonyl)-hexahydro-pyrrolo[3,2-*b*]pyrrole-1-carbonyl]-butyl}-benzamide;

28. 4-Isopropyl-N- {3-methyl-1-[6-oxo-4-(pyridine-2-sulfonyl)-hexahydro-pyrrolo[3,2-*b*]pyrrole-1-carbonyl]-butyl}-benzamide;

29. N-{3-Methyl-1-[6-oxo-4-(pyridine-2-sulfonyl)-hexahydro-pyrrolo[3,2-*b*]pyrrole-1-carbonyl]-butyl}-4-vinyl-benzamide;

30. 4-Imidazol-1-yl-N-{3-methyl-1-[6-oxo-4-(pyridine-2-sulfonyl)-hexahydro-pyrrolo[3,2-*b*]pyrrole-1-carbonyl]-butyl}-benzamide;

31. N-{3-Methyl-1-[6-oxo-4-(pyridine-2-sulfonyl)-hexahydro-pyrrolo[3,2-*b*]pyrrole-1-carbonyl]-butyl}-4-thiophen-2-yl-benzamide;

32. N-{3-Methyl-1-[6-oxo-4-(pyridine-2-sulfonyl)-hexahydro-pyrrolo[3,2-*b*]pyrrole-1-carbonyl]-butyl}-4-oxazol-5-yl-benzamide;

33. 5-Phenyl-thiophene-2-carboxylic acid {3-methyl-1-[6-oxo-4-(pyridine-2-sulfonyl)-hexahydro-pyrrolo[3,2-*b*]pyrrole-1-carbonyl]-butyl}-amide;

34. N-{3-Methyl-1-[6-oxo-4-(pyridine-2-sulfonyl)-hexahydro-pyrrolo[3,2-*b*]pyrrole-1-carbonyl]-butyl}-4-trifluoromethoxy-benzamide;

35. 5-Pyridin-2-yl-thiophene-2-carboxylic acid {3-methyl-1-[6-oxo-4-(pyridine-2-sulfonyl)-hexahydro-pyrrolo[3,2-*b*]-pyrrole-1-carbonyl]-butyl}-amide;

36. 4-Difluoromethoxy-N- {3-methyl-1-[6-oxo-4-(pyridine-2-sulfonyl)-hexallydro-pyrrolo[3,2-*b*]pyrrole-1-carbonyl]-butyl}-benzamide;

37. N-{3-Methyl-1-[6-oxo-4-(pyridine-2-sulfonyl)-hexahydro-pyrrolo[3,2-*b*]pyrrole-1-carbonyl]-butyl}-4-morpholin-4-yl-benzamide;

38. Naphthalene-2-carboxylic acid {1-[6-oxo-4-(pyridine-2-sulfonyl)hexahydro-pyrrolo[3,2- *b*]pyrrole-1-carbonyl]-butyl}-amide;

39. Quinoline-2-carboxylic acid {1-[6-oxo-4-(pyridine-2-sulfonyl)-hexahydro-pyrrolo[3,2-*b*]pyrrole-1-carbonyl]-butyl}-amide;

40. Benzo[b]thiophene-2-carboxylic acid {1-[6-oxo-4-(pyridine-2-sulfonyl)-hexahydro-pyrrolo[3,2-*b*]pyrrole-1-carbonyl]-butyl}-amide;

41. Benzofuran-2-carboxylic acid {1-[6-oxo-4-(pyridine-2-sulfonyl)-hexahydro-pyrrolo[3,2-*b*]pyrrole-1-carbonyl]-butyl}-amide;

42. Biphenyl-4-carboxylic acid {1-[6-oxo-4-(pyridine-2-sulfonyl)-hexahydro-pyrrolo[3,2-*b*]pyrrole-1-carbonyl]-butyl}-amide;

43. 4-*tert*-Butyl-N-{1-[6-oxo-4-(pyridine-2-sulfonyl)-hexahydro-pyrrolo[3,2-*b*]pyrrole-1-carbonyl]-butyl}-benzamide;

44. 4-Dimethylamino-N-{1-[6-oxo-4-(pyridine-2-sulfonyl)-hexahydro-pyrrolo[3,2-*b*]pyrrole-1-carbonyl]-butyl}-benzamide;

45. 7-Methoxy-benzofuran-2-carboxylic acid {1-[6-oxo-4-(pyridine-2-sulfonyl)-hexahydro-pyrrolo[3,2-*b*]pyrrole-1-carbonyl]-butyl}-amide;

46. {1-[6-oxo-4-(pyridine-2-sulfonyl)-hexahydro-pyrrolo[3,2-*b*]pyrrole-1-carbonyl]-butyl}-carbamic acid benzyl ester;

47. 5-Methoxy-benzofuran-2-carboxylic acid {1-[6-oxo-4-(pyridine-2-sulfonyl)-hexahydro-pyrrolo[3,2-*b*]pyrrole-1-carbonyl]-butyl}-amide;

48. Thieno[3,2-*b*]thiophene-2-carboxylic acid {1-[6-oxo-4-(pyridine-2-sulfonyl)-hexahydro-pyrrolo[3,2-*b*]pyrrole-1-carbonyl]-butyl}-amide;

49. 3-Methyl-benzofuran-2-carboxylic acid {1-[6-oxo-4-(pyridine-2-sulfonyl)-hexahydro-pyrrolo[3,2-*b*]pyrrole-1-carbonyl]-butyl}-amide;

50. Quinoxaline-2-carboxylic acid {1-[6-oxo-4-(pyridine-2-sulfonyl)-hexahydro-pyrrolo[3,2-*b*]pyrrole-1-carbonyl]-butyl}-amide;

51. Benzo[1,3]dioxole-5-carboxylic acid {1-[6-oxo-4-(pyridine-2-sulfonyl)-hexahydro-pyrrolo[3,2-*b*]pyrrole-1-carbonyl]-butyl}-amide;

52. Quinoline-6-carboxylic acid {1-[6-oxo-4-(pyridine-2-sulfonyl)-hexahydro-pyrrolo[3,2-*b*]pyrrole-1-carbonyl]-butyl}-amide;

53. Furan-2-carboxylic acid {1-[6-oxo-4-(pyridine-2-sulfonyl)-hexahydro-pyrrolo[3,2-*b*]pyrrole-1-carbonyl]-butyl}-amide;

54. Thiophene-2-carboxylic acid {1-[6-oxo-4-(pyridine-2-sulfonyl)-hexahydro-pyrrolo[3,2-*b*]pyrrole-1-carbonyl]-butyl}-amide;

55. N-{1-[6-oxo-4-(pyridine-2-sulfonyl)-hexahydro-pyrrolo[3,2-*b*]pyrrole-1-carbonyl]-butyl}-4-trifluoromethyl-benzamide;

56. Furan-3-carboxylic acid {1-[6-oxo-4-(pyridine-2-sulfonyl)-hexahydro-pyrrolo[3,2-*b*]pyrrole-1-carbonyl]-butyl}-amide;

57. Thiophene-3-carboxylic acid {1-[6-oxo-4-(pyridine-2-sulfonyl)-hexahydro-pyrrolo[3,2-*b*]pyrrole-1-carbonyl]-butyl}-amide;

58. N- {1-[6-oxo-4-(pyridine-2-sulfonyl)-hexahydro-pyrrolo[3,2-*b*]pyrrole-1-carbonyl]-butyl}-4-phenoxy-benzamide;

59. N-{1-[6-oxo-4-(pyridine-2-sulfonyl)-hexahydro-pyrrolo[3,2-*b*]pyrrole-1-carbonyl]-butyl}-4-(4-methyl-piperazin-1-yl)-benzamide;

60. 4-Methyl-N- {1-[6-oxo-4-(pyridine-2-sulfonyl)-hexahydro-pyrrolo[3,2-*b*]pyrrole-1-carbonyl]-butyl}-benzamide;

61. 4-Methoxy-N-{1-[6-oxo-4-(pyridine-2-sulfonyl)-hexahydro-pyrrolo[3,2-*b*]pyrrole-1-carbonyl]-butyl}-benzamide;

62. 4-Isopropyl-N-{1-[6-oxo-4-(pyridine-2-sulfonyl)-hexahydro-pyrrolo[3,2-*b*]pyrrole-1-carbonyl]-butyl}-benzamide;

63. N-{1-[6-oxo-4-(pyridine-2-s-ulfonyl)-hexahydro-pyrrolo[3,2-*b*]pyrrole-1-carbonyl]-butyl}-4-vinyl-benzamide;

64. 4-Imidazol-1-yl-N-{1-[6-oxo-4-(pyridine-2-sulfonyl)-hexahydro-pyrrolo[3,2-*b*]pyrrole-1-carbonyl]-butyl}-benzamide;

65. *N*-{1-[6-oxo-4-(pyridine-2-sulfonyl)-hexahydro-pyrrolo[3,2-*b*]pyrrole-1-carbonyl]-butyl}-4-thiophen-2-yl-benzamide;

66. N-{1-[6-oxo-4-(pyridine-2-sulfonyl)-hexahydro-pyrrolo[3,2-*b*]pyrrole-1-carbonyl]-butyl}-4-oxazol-5-yl-benzamide;

67. 5-Phenyl-thiophene-2-carboxylic acid {1-[6-oxo-4-(pyridine-2-sulfonyl)-bexahydro-pyrrolo[3,2-*b*]pyrrole-1-carbonyl]-butyl}-amide;

68. N-{1-[6-oxo-4-(pyridine-2-sulfonyl)-hexahydro-pyrrolo[3,2-*b*]pyrrole-1-carbonyl]-butyl}-4-trifluoromethoxy-benzamide;

69. 5-Pyridin-2-yl-thiophene-2-carboxylic acid {1-[6-oxo-4-(pyridine-2-sulfonyl)-hexahydro-pyrrolo[3,2-*b*]pyrrole-1-carbonyl]-butyl}-amide;

70. 4-Difluoromethoxy-N- {1-[6-oxo-4-(pyridine-2-sulfonyl)-hexahydro-pyrrolo[3,2-*b*]pyrrole-1-carbonyl]-butyl}-benzamide;

71. N-{1-[6-oxo-4-(pyridine-2-sulfonyl)-hexahydro-pyrrolo[3,2-*b*]pyrrole-1-carbonyl]-butyl} -4-morpholin-4-yl-benzamide;

72. Naphthalene-2-carboxylic acid {3-methyl-1-[6-oxo-4-(1-oxy-pyridine-2-sulfonyl)hexahydro-pyrrolo[3,2-*b*]pyrrole-1-carbonyl]-butyl}-amide;

73. Quinoline-2-carboxylic acid {3-methyl-1-[6-oxo-4-(1-oxy-pyridine-2-sulfonyl)-hexahydro-pyrrolo[3,2-*b*]pyrrole-1-carbonyl]-butyl}-amide;

74. Benzo[b]thiophene-2-carboxylic acid {3-methyl-1-[6-oxo-4-(1-oxy-pyridine-2-sulfonyl)-hexahydro-pyrrolo[3,2-

*b*]pyrrole-1-carbonyl]-butyl}-amide;

75. Benzofuran-2-carboxylic acid {3-methyl-1-[6-oxo-4-(1-oxy-pyridine-2-sulfonyl)-hexahydro-pyrrolo[3,2-*b*]pyrrole-1-carbonyl]-butyl}-amide;

76. Biphenyl-4-carboxylic acid {3-methyl-1-[6-oxo-4-(1-oxy-pyridine-2-sulfonyl)-hexallydro-pyrrolo[3,2-*b*]pyrrole-1-carbonyl]-butyl}-amide;

77. 4-*tert*-Butyl-N-{3-methyl-1-[6-oxo-4-(1-oxy-pyridine-2-sulfonyl)-hexahydro-pyrrolo[3,2-*b*]pyrrole-1-carbonyl]-butyl}-benzamide;

78. 4-Dimethylamino-N-{3-methyl-1-[6-oxo-4-(1-oxy-pyridine-2-sulfonyl)-hexahydro-pyrrolo[3,2-*b*]pyrrole-1-carbonyl]-butyl}-benzamide;

79. 7-Methoxy-benzofuran-2-carboxylic acid {3-methyl-1-[6-oxo-4-(1-oxy-pyridine-2-sulfonyl)-hexahydro-pyrrolo[3,2-*b*]pyrrole-1-carbonyl]-butyl}-amide;

80. {3-Methyl-1-[6-oxo-4-(1-oxy-pyridine-2-sulfonyl)-hexahydro-pyrrolo[3,2-*b*]pyrrole-1-carbonyl]-butyl}-carbamic acid benzyl ester;

81. 5-Methoxy-benzofuran-2-carboxylic acid {3-methyl-1-[6-oxo-4-(1-oxy-pyridine-2-sulfonyl)-hexahydro-pyrrolo[3,2-*b*]pyrrole-1-carbonyl]-butyl}-amide;

82. Thieno[3,2-*b*]thiophene-2-carboxylic acid {3-methyl-1-[6-oxo-4-(1-oxy-pyridine-2-sulfonyl)-hexahydro-pyrrolo[3,2-*b*]pyrrole-1-carbonyl]-butyl}-amide;

83. 3-Methyl-benzofuran-2-carboxylic acid {3-methyl-1-[6-oxo-4-(1-oxy-pyridine-2-sulfonyl)-hexahydro-pyrrolo[3,2-*b*]pyrrole-1-carbonyl]-butyl}-amide;

84. Quinoxaline-2-carboxylic acid {3-methyl-1-[6-oxo-4-(1-oxy-pyridine-2-sulfonyl)-hexahydro-pyrrolo[3,2-*b*]pyrrole-1-carbonyl]-butyl}-amide;

85. Benzo[1,3]dioxole-5-carboxylic acid {3-methyl-1-[6-oxo-4-(1-oxy-pyridine-2-sulfonyl)-hexahydro-pyrrolo[3,2-*b*]pyrrole-1-carbonyl]-butyl}-amide;

86. Quinoline-6-carboxylic acid {3-methyl-1-[6-oxo-4-(1-oxy-pyridine-2-s-ulfonyl)-hexahydro-pyrrolo[3,2-*b*]pyrrole-1-carbonyl]-butyl}-amide;

87. Furan-2-carboxylic acid {3-methyl-1-[6-oxo-4-(1-oxy-pyridine-2-sulfonyl)-hexahydro-pyrrolo[3,2-*b*]pyrrole-1-carbonyl]-butyl}-amide;

88. Thiophene-2-carboxylic acid {3-methyl-1-[6-oxo-4-(1-oxy-pyridine-2-sulfonyl)-hexahydro-pyrrolo[3,2-*b*]pyrrole-1-carbonyl]-butyl}-amide;

89. N-{3-Methyl-1-[6-oxo-4-(1-oxy-pyridine-2-sulfonyl)-hexahydro-pyrrolo[3,2-*b*]pyrrole-1-carbonyl]-butyl}-4-trifluoromethyl-benzamide;

90. Furan-3-carboxylic acid {3-methyl-1-[6-oxo-4-(1-oxy-pyridine-2-sulfonyl)-hexahydro-pyrrolo[3,2-*b*]pyrrole-1-carbonyl]-butyl}-amide;

91. Thiophene-3-carboxylic acid {3-methyl-1-[6-oxo-4-(1-oxy-pyridine-2-sulfonyl)-hexahydro-pyrrolo[3,2-*b*]pyrrole-1-carbonyl]-butyl}-amide;

92. N-{3-Methyl-1-[6-oxo-4-(1-oxy-pyridine-2-sulfonyl)-hexahydro-pyrrolo[3,2-*b*]pyrrole-1-carbonyl]-butyl}-4-phenoxy-benzamide;

93. N-{3-Methyl-1-[6-oxo-4-(1-oxy-pyridine-2-sulfonyl)-hexahydro-pyrrolo[3,2-*b*]pyrrole-1-carbonyl]-butyl}-4-(4-methyl-piperazin-1-yl)-benzamide;

94. 4-Methyl-N- {3-methyl-1-[6-oxo-4-(1-oxy-pyridine-2-sulfonyl)-hexahydro-pyrrolo[3,2-*b*]pyrrole-1-carbonyl]-butyl}-benzamide;

95. 4-Methoxy-N-{3-methyl-1-[6-oxo-4-(1-oxy-pyridine-2-sulfonyl)-hexahydro-pyrrolo[3,2-*b*]pyrrole-1-carbonyl]-butyl}-benzamide;

96. 4-Isopropyl-N-{3-methyl-1-[6-oxo-4-(1-oxy-pyridine-2-sulfonyl)-hexahydro-pyrrolo[3,2-*b*]pyrrole-1-carbonyl]-butyl}-benzamide;

97. N-{3-Methyl-1-[6-oxo-4-(1-oxy-pyridine-2-sulfonyl)-hexahydro-pyrrolo[3,2-*b*]pyrrole-1-carbonyl]-butyl}-4-vinyl-benzamide;

98. 4-Imidazol-1-yl-N-{3-methyl-1-[6-oxo-4-(1-oxy-pyridine-2-sulfonyl)-hexahydro-pyrrolo[3,2-*b*]pyrrole-1-carbonyl]-butyl}-benzamide;

99. N-{3-Methyl-1-[6-oxo-4-(1-oxy-pyridine-2-sulfonyl)-hexahydro-pyrrolo[3,2-*b*]pyrrole-1-carbonyl]-butyl}-4-thiophen-2-yl-benzamide;

100. N-{3-Methyl-1-[6-oxo-4-(1-oxy-pyridine-2-sulfonyl)-hexahydro-pyrrolo[3,2-*b*]pyrrole-1-carbonyl]-butyl}-4-oxazol-5-yl-benzamide;

101. 5-Phenyl-thiophene-2-carboxylic acid {3-methyl-1-[6-oxo-4-(1-oxy-pyridine-2-sulfonyl)-hexahydro-pyrrolo[3,2-*b*]pyrrole-1-carbonyl]-butyl}-amide;

102. N-{3-Methyl-1-[6-oxo-4-(1-oxy-pyridine-2-sulfonyl)-hexahydro-pyrrolo[3,2-*b*]pyrrole-1-carbonyl]-butyl}-4-trifluoromethoxy-benzamide;

103. 5-Pyridin-2-yl-thiophene-2-carboxylic acid {3-methyl-1-[6-oxo-4-(1-oxy-pyridine-2-sulfonyl)-hexahydro-pyrrolo[3,2-*b*]pyrrole-1-carbonyl]-butyl}-amide;

104. 4-Difluoromethoxy-N-{3-methyl-1-[6-oxo-4-(1-oxy-pyridine-2-sulfonyl)-hexahydro-pyrrolo[3,2-*b*]pyrrole-1-carbonyl]-butyl}-benzamide;

105. N-{3-Methyl-1-[6-oxo-4-(1-oxy-pyridine-2-sulfonyl)-hexahydro-pyrrolo[3,2-*b*]pyrrole-1-carbonyl]-butyl}-4-morpholin-4-yl-benzamide;

106. Naphthalene-2-carboxylic acid {1-[6-oxo-4-(1-oxy-pyridine-2-sulfonyl)hexahydro-pyrrolo[3,2-*b*]pyrrole-1-carbonyl]-butyl}-amide;

107. Quinoline-2-carboxylic acid {1-[6-oxo-4-(1-oxy-pyridine-2-sulfonyl)-hexahydro-pyrrolo[3,2-*b*]pyrrole-1-carbonyl]-butyl}-amide;

108. Benzo[b]thiophene-2-carboxylic acid {1-[6-oxo-4-(1-oxy-pyridine-2-sulfonyl)-hexahydro-pyrrolo[3,2-*b*]pyrrole-1-carbonyl]-butyl}-amide;

109. Benzofuran-2-carboxylic acid {1-[6-oxo-4-(1-oxy-pyridine-2-sulfonyl)-hexahydro-pyrrolo[3,2-*b*]pyrrole-1-carbonyl]-butyl}-amide;

110. Biphenyl-4-carboxylic acid {1-[6-oxo-4-(1-oxy-pyridine-2-sulfonyl)-hexahydro-pyrrolo[3,2-*b*]pyrrole-1-carbonyl]-butyl}-amide;

111. 4-*tert*-Butyl-N-{1-[6-oxo-4-(1-oxy-pyridine-2-sulfonyl)-hexahydro-pyrrolo[3,2-*b*]pyrrole-1-carbonyl]-butyl}-benzamide;

112. 4-Dimethylamino-N- {1-[6-oxo-4-(1-oxy-pyridine-2-sulfonyl)-hexahydro-pyrrolo[3,2-*b*]pyrrole-1-carbonyl]-butyl}-benzamide;

113. 7-Methoxy-benzofuran-2-carboxylic acid {1-[6-oxo-4-(1-oxy-pyridine-2-sulfonyl)-hexahydro-pyrrolo[3,2-*b*]pyrrole-1-carbonyl]-butyl}-amide;

114. {1-[6-oxo-4-(1-oxy-pyridine-2-sulfonyl)-hexahydro-pyrrolo[3,2-*b*]pyrrole-1-carbonyl]-butyl}-carbamic acid benzyl ester;

115. 5-Methoxy-benzofuran-2-carboxylic acid {1-[6-oxo-4-(1-oxy-pyridine-2-sulfonyl)-hexahydro-pyrralo[3,2-*b*]pyrrole-1-carbonyl]-butyl}-amide;

116. Thieno[3,2-*b*]thiophene-2-carboxylic acid {1-[6-oxo-4-(1-oxy-pyridine-2-sulfonyl)-hexahydro-pyrrolo[3,2-*b*]pyrrole-1-carbonyl]-butyl}-amide;

117. 3-Methyl-benzofuran-2-carboxylic acid {1-[6-oxo-4-(1-oxy-pyridine-2-sulfonyl)-hexahydro-pyrrolo[3,2-*b*]pyrrole-1-carbonyl]-butyl}-amide;

118. Quinoxaline-2-carboxylic acid {1-[6-oxo-4-(1-oxy-pyridine-2-sulfonyl)-hexahydro-pyrrolo[3,2-*b*]pyrrole-1-carbonyl]-butyl}-amide;

119. Benzo[1,3]dioxole-5-carboxylic acid {1-[6-oxo-4-(1-oxy-pyridine-2-sulfonyl)-hexahydro-pyrrolo[3,2-*b*]pyrrole-1-carbonyl]-butyl}-amide;

120. Quinoline-6-carboxylic acid {1-[6-oxo-4-(1-oxy-pyridine-2-sulfonyl)-hexahydro-pyrrolo[3,2-*b*]pyrrole-1-carbonyl]-butyl}-amide;

121. Furan-2-carboxylic acid {1-[6-oxo-4-(1-oxy-pyidine-2-sulfonyl)-hexahydro-pyrrolo[3,2-*b*]pyrrole-1-carbonyl]-butyl}-amide;

122. Thiophene-2-carboxylic acid {1-[6-oxo-4-(1-oxy-pyridine-2-sulfonyl)-hexabydro-pyrrolo[3,2-*b*]pyrrole-1-carbonyl]-butyl}-amide;

123. N-{1-[6-oxo-4-(1-oxy-pyridine-2-sulfonyl)-hexahydro-pyrrolo[3,2-*b*]pyrrole-1-carbonyl]-butyl}-4-trifluoromethyl-benzamide;

124. Furan-3-carboxylic acid {1-[6-oxo-4-(1-oxy-pyridine-2-sulfonyl)-hexahydro-pyrrolo[3,2-*b*]pyrrole-1-carbonyl]-butyl}-amide;

125. Thiophene-3-carboxylic acid {1-[6-oxo-4-(1-oxy-pyridine-2-sulfonyl)-hexahydro-pyrrolo[3,2-*b*]pyrrole-1-carbonyl]-butyl}-amide;

126. N-{1-[6-oxo-4-(1-oxy-pyridine-2-sulfonyl)-hexahydro-pyrrolo[3,2-*b*]pyrrole-1-carbonyl]-butyl}-4-phenoxy-benzamide;

127. N-{1-[6-oxo-4-(1-oxy-pyridine-2-sulfonyl)-hexahydro-pyrrolo[3,2-*b*]pyrrole-1-carbonyl]-butyl}-4-(4-methyl-piperazin-1-yl)-benzamide;

128. 4-Methyl-N- {1-[6-oxo-4-(1-oxy-pyridine-2-sulfonyl)-hexahydro-pyrrolo[3,2- *b*]pyrrole-1-carbonyl]-butyl}-benzamide;

129. 4-Methohy-N-{1-[6-oxo-4-(1-oxy-pyridine-2-sulfonyl)-hexahydro-pyrrolo[3,2-*b*]pyrrole-1-carbonyl]-butyl}-benzamide;

130. 4-Isopropyl-N- {1-[6-oxo-4-(1-oxy-pyridine-2-sulfonyl)-hexahydro-pyrrolo[3,2-*b*]pyrrole-1-carbonyl]-butyl}-benzamide;

131. N-{1-[6-oxo-4-(1-oxy-pyridine-2-sulfonyl)-hexahydro-pyrrolo[3,2-*b*]pyrrole-1-carbonyl]-butyl}-4-vinyl-benzamide;

132. 4-Imidazol-1-yl-N-{1-[6-oxo-4-(1-oxy-pyridine-2-sulfonyl)-hexahydro-pyrrolo[3,2-*b*]pyrrole-1-carbonyl]-butyl}-benzamide;

133. N-{1-[6-oxo-4-(1-oxy-pyridine-2-sulfonyl)-hexahydro-pyrrolo[3,2-*b*]pyrrole-1-carbonyl]-butyl}-4-thiophen-2-yl-benzamide;

134. N-{1-[6-oxo-4-(1-oxy-pyridine-2-sulfonyl)-hexahydro-pyrrolo[3,2-*b*]pyrrole-1-carbonyl]-butyl}-4-oxazol-5-yl-benzamide;

135. 5-Phenyl-thiophene-2-carboxylic acid {1-[6-oxo-4-(1-oxy-pyridine-2-sulfonyl)-hexahydro-pyrrolo[3,2-*b*]pyrrole-1-carbonyl]-butyl}-amide;

136. N-{1-[6-oxo-4-(1-oxy-pyridine-2-sulfonyl)-hexahydro-pyrrolo[3,2-*b*]pyrrole-1-carbonyl]-butyl}-4-trifluoromethoxy-benzamide;

137. 5-Pyridin-2-yl-thiophene-2-carboxylic acid {1-[6-oxo-4-(1-oxy-pyridine-2-sulfonyl)-hexahydro-pyrrolo[3,2-*b*]-pyrrole-1-carbonyl]-butyl}-amide;

138. 4-Difluoromethoxy-N- {1-[6-oxo-4-(1-oxy-pyridine-2-sulfonyl)-hexahydro-pyrrolo[3,2-*b*]pyrrole-1-carbonyl]-butyl}-benzamide;

139. N-{1-[6-oxo-4-(1-oxy-pyridine-2-sulfonyl)-hexahydro-pyrrolo[3,2-*b*]pyrrole-1-carbonyl]-butyl}-4-morpholin-4-yl-benzamide;

140. Naphthalene-2-carboxylic acid {3-methyl-1-[6-oxo-4-(pyridine-3-sulfonyl)hexahydro-pyrrolo[3,2- *b*]pyrrole-1-carbonyl]-butyl}-amide;

141. Quinoline-2-carboxylic acid {3-methyl-1-[6-oxo-4-(pyridine-3-sulfonyl)-hexahydro-pyrrolo[3,2-*b*]pyrrole-1-carbonyl]-butyl}-amide;

142. Benzo[b]thiophene-2-carboxylic acid {3-methyl-1-[6-oxo-4-(pyridine-3-sulfonyl)-hexahydro-pyrrolo[3,2-*b*]pyrrole-1-carbonyl]-butyl}-amide;

143. Benzofuran-2-carboxylic acid {3-methyl-1-[6-oxo-4-(pyridine-3-sulfonyl)-hexahydro-pyrrolo[3,2-*b*]pyrrole-1-carbonyl]-butyl}-amide;

144. Biphenyl-4-carboxylic acid {3-methyl-1-[6-oxo-4-(pyridine-3-sulfonyl)-hexahydro-pyrrolo[3,2-*b*]pyrrole-1-carbonyl]-butyl}-amide;

145. 4-*tert*-Butyl-N-{3-methyl-1-[6-oxo-4-(pyridine-3-sulfonyl)-hexahydro-pyrrolo[3,2- *b*]pyrrole-1-carbonyl]-butyl}-benzamide;

146. 4-Dimethylamino-N-{3-methyl-1-[6-oxo-4-(pyridine-3-sulfonyl)-hexahydro-pyrrolo[3,2-*b*]pyrrole-1-carbonyl]-butyl}-benzamide;

147. 7-Methoxy-benzofuran-2-carboxylic acid {3-methyl-1-[6-oxo-4-(pyridine-3-sulfonyl)-hexahydro-pyrrolo[3,2-*b*]-pyrrole-1-carbonyl]-butyl}-amide;

148. {3-Methyl-1-[6-oxo-4-(pyridine-3-sulfonyl)-hexahydro-pyrrolo[3,2-*b*]pyrrole-1-carbonyl]-butyl}-carbamic acid benzyl ester;

149. 5-Methoxy-benzofuran-2-carboxylic acid {3-methyl-1-[6-oxo-4-(pyridine-3-sulfonyl)-hexahydro-pyrrolo[3,2-*b*]pyrrole-1-carbonyl]-butyl}-amide;

150. Thieno[3,2-*b*]thiophene-2-carboxylic acid {3-methyl-1-[6-oxo-4-(pyridine-3-sulfonyl)-hexahydro-pyrrolo[3,2-*b*]-pyrrole-1-carbonyl]-butyl}-amide;

151. 3-Methyl-benzofuran-2-carboxylic acid {3-methyl-1-[6-oxo-4-(pyridine-3-sulfonyl)-hexahydro-pyrrolo[3,2-*b*]-pyrrole-1-carbonyl]-butyl}-amide;

152. Quinoxaline-2-carboxylic acid {3-methyl-1-[6-oxo-4-(pyridine-3-sulfonyl)-hexahydro-pyrrolo[3,2-*b*]pyrrole-1-carbonyl]-butyl}-amide;

153. Benzo[1,3]dioxole-5-carboxylic acid {3-methyl-1-[6-oxo-4-(pyridine-3-sulfonyl)-hexahydro-pyrrolo[3,2-*b*]pyrrole-1-carbonyl]-butyl}-amide;

154. Quinoline-6-carboxylic acid {3-methyl-1-[6-oxo-4-(pyridine-3-sulfonyl)-hexahydro-pyrrolo[3,2-*b*]pyrrole-1-carbonyl]-butyl}-amide;

155. Furan-2-carboxylic acid {3-methyl-1-[6-oxo-4-(pyridine-3-sulfonyl)-hexahydro-pyrrolo[3,2-*b*]pyrrole-1-carbonyl]-butyl}-amide;

156. Thiophene-2-carboxylic acid {3-methyl-1-[6-oxo-4-(pyridine-3-sulfonyl)-hexahydro-pyrrolo[3,2-*b*]pyrrole-1-carbonyl]-butyl}-amide;

157. N-{3-Methyl-1-[6-oxo-4-(pyridine-3-sulfonyl)-hexahydro-pyrrolo[3,2-*b*]pyrrole-1-carbonyl]-butyl}-4-trifluoromethyl-benzamide;

158. Furan-3-carboxylic acid {3-methyl-1-[6-oxo-4-(pyridine-3-sulfonyl)-hexahydro-pyrrolo[3,2- *b*]pyrrole-1-carbonyl]-butyl}-amide;

159. Thiophene-3-carboxylic acid {3-methyl-1-[6-oxo-4-(pyridine-3-sulfonyl)-hexahydro-pyrrolo[3,2-*b*]pyrrole-1-carbonyl]-butyl}-amide;

160. N-{3-Methyl-1-[6-oxo-4-(pyridine-3-sulfonyl)-hexahydro-pyrrolo[3,2-*b*]pyrrole-1-carbonyl]-butyl}-4-phenoxy-benzamide;

161. N-{3-Methyl-1-[6-oxo-4-(pyridine-3-sulfonyl)-hexahydro-pyrrolo[3,2-*b*]pyrrole-1-carbonyl]-butyl}-4-(4-methyl-piperazin-1-yl)-benzamide;

162. 4-Methyl-N-{3-methyl-1-[6-oxo-4-(pyridine-3-sulfonyl)-hexahydro-pyrrolo[3,2-*b*]pyrrole-1-carbonyl]-butyl}-benzamide;

163. 4-Methoxy-N-{3-methyl-1-[6-oxo-4-(pyridine-3-sulfonyl)-hexahydro-pyrrolo[3,2-*b*]pyrrole-1-carbonyl]-butyl}-benzamide;

164. 4-Isopropyl-N-{3-methyl-1-[6-oxo-4-(pyridine-3-sulfonyl)-hexahydro-pyrrolo[3,2-*b*]pyrrole-1-carbonyl]-butyl}-benzamide;

165. N-{3-Methyl-1-[6-oxo-4-(pyridine-3-sulfonyl)-hexahydro-pyrrolo[3,2-*b*]pyrrole-1-carbonyl]-butyl}-4-vinyl-benzamide;

166. 4-Imidazol-1-yl-N-{3-methyl-1-[6-oxo-4-(pyridine-3-sulfonyl)-hexahydro-pyrrolo[3,2-*b*]pyrrole-1-carbonyl]-butyl}-benzamide;

167. N-{3-Methyl-1-[6-oxo-4-(pyridine-3-sulfonyl)-hexahydro-pyrrolo[3,2-*b*]pyrrole-1-carbonyl]-butyl}-4-thiophen-2-yl-benzamide;

168. N-{3-Methyl-1-[6-oxo-4-(pyridine-3-sulfonyl)-hexahydro-pyrrolo[3,2-*b*]pyrrole-1-carbonyl]-butyl}-4-oxazol-5-yl-benzamide;

169. 5-Phenyl-thiophene-2-carboxylic acid {3-methyl-1-[6-oxo-4-(pyridine-3-sulfonyl)-hexahydro-pyrrolo[3,2-*b*]pyrrole-1-carbonyl]-butyl}-amide;

170: N-{3-Methyl-1-[6-oxo-4-(pyridine-3-sulfonyl)-hexahydro-pyrrolo[3,2-*b*]pyrrole-1-carbonyl]-butyl}-4-trifluoromethoxy-benzamide;

171. 5-Pyridin-2-yl-thiophene-2-carboxylic acid {3-methyl-1-[6-oxo-4-(pyridine-3-sulfonyl)-hexahydro-pyrrolo[3,2-*b*]pyrrole-1-carbonyl]-butyl}-amide;

172. 4-Difluoromethoxy-N-{3-methyl-1-[6-oxo-4-(pyridine-3-sulfonyl)-hexahydro-pyrrolo[3,2-*b*]pyrrole-1-carbonyl]-butyl}-benzamide;

173. N-{3-Methyl-1-[6-oxo-4-(pyridine-3-sulfonyl)-hexahydro-pyrrolo[3,2-*b*]pyrrole-1-carbonyl]-butyl}-4-morpholin-4-yl-benzamide;

174. Naphthalene-2-carboxylic acid {1-[6-oxo-4-(pyridine-3-sulfonyl)hexahydro-pyrrolo[3,2-*b*]pyrrole-1-carbonyl]-butyl}-amide;

175. Quinoline-2-carboxylic acid {1-[6-oxo-4-(pyridine-3-sulfonyl)-hexahydro-pyrrolo[3,2-*b*]pyrrole-1-carbonyl]-butyl}-amide;

176. Benzo[b]thiophene-2-carboxylic acid {1-[6-oxo-4-(pyridine-3-sulfonyl)-hexahydro-pyrrolo[3,2-*b*]pyrrole-1-carbonyl]-butyl}-amide;

177. Benzofuran-2-carboxylic acid {1-[6-oxo-4-(pyridine-3-sulfonyl)-hexahydro-pyrrolo[3,2-*b*]pyrrole-1-carbonyl]-butyl}-amide;

178. Biphenyl-4-carboxylic acid {1-[6-oxo-4-(pyridine-3-sulfonyl)-hexahydro-pyrolo[3,2-*b*]pyrrole-1-carbonyl]-butyl}-amide;

179. 4-*tert*-Butyl-N-{1-[6-oxo-4-(pyridine-3-sulfonyl)-hexahydro-pyrrolo[3,2-*b*]pyrrole-1-carbonyl]-butyl}-benzamide;

180. 4-Dimethylamino-N- {1-[6-oxo-4-(pyridine-3-sulfonyl)-hexahydro-pyrrolo[3,2-*b*]pyrrole-1-carbonyl]-butyl}-benzamide;

181. 7-Methoxy-benzofuran-2-carboxylic acid {1-[6-oxo-4-(pyridine-3-sulfonyl)-hexahydro-pyrrolo[3,2-*b*]pyrrole-1-carbonyl]-butyl}-amide;

182. {1-[6-oxo-4-(pyridine-3-sulfonyl)-hexahydro-pyrrolo[3,2-*b*]pyrrole-1-carbonyl]-butyl}-carbamic acid benzyl ester;

183. 5-Methoxy-benzofuran-2-carboxylic acid {1-[6-oxo-4-(pyridine-3-sulfonyl)-hexahydro-pyrrolo[3,2-*b*]pyrrole-1-carbonyl]-butyl}-amide;

184. Thieno[3,2-*b*]thiophene-2-carboxylic acid {1-[6-oxo-4-(pyridine-3-sulfonyl)-hexahydro-pyrrolo[3,2-*b*]pyrrole-1-carbonyl]-butyl}-amide;

185. 3-Methyl-benzofuran-2-carboxylic acid {1-[6-oxo-4-(pyridine-3-sulfonyl)-hexahydro-pyrrolo[3,2-*b*]pyrrole-1-carbonyl]-butyl}-amide;

186. Quinoxaline-2-carboxylic acid {1-[6-oxo-4-(pyridine-3-sulfonyl)-hexahydro-pyrrolo[3,2-*b*]pyrrole-1-carbonyl]-butyl}-amide;

187. Benzo[1,3]dioxole-5-carboxylic acid {1-[6-oxo-4-(pyridine-3-sulfonyl)-hexahydro-pyrrolo[3,2-*b*]pyrrole-1-carbonyl]-butyl}-amide;

188. Quinoline-6-carboxylic acid {1-[6-oxo-4-(pyridine-3-sulfonyl)-hexahydro-pyrrolo[3,2-*b*]pyrrole-1-carbonyl]-butyl}-amide;

189. Furan-2-carboxylic acid {1-[6-oxo-4-(pyridine-3-sulfonyl)-hexahydro-pyrrolo[3,2-*b*]pyrrole-1-carbonyl]-butyl}-amide;

190. Thiophene-2-carboxylic acid {1-[6-oxo-4-(pyridine-3-sulfonyl)-hexahydro-pyrrolo[3,2-*b*]pyrrole-1-carbonyl]-butyl}-amide;

191. N-{1-[6-oxo-4-(pyridine-3-sulfonyl)-hexahydro-pyrrolo[3,2-*b*]pyrrole-1-carbonyl]-butyl}-4-trifluoromethyl-benzamide;

192. Furan-3-carboxylic acid {1-[6-oxo-4-(pyridine-3-sulfonyl)-hexahydro-pyrrolo[3,2-*b*]pyrrole-1-carbonyl]-butyl}-amide;

193. Thiophene-3-carboxylic acid {1-[6-oxo-4-(pyridine-3-sulfonyl)-hexahydro-pyrrolo[3,2-*b*]pyrrole-1-carbonyl]-butyl}-amide;

194. N-{1-[6-oxo-4-(pyridine-3-sulfonyl)-hexahydro-pyrrolo[3,2-*b*]pyrrole-1-carbonyl]-butyl}-4-phenoxy-benzamide;

195. N-{1-[6-oxo-4-(pyridine-3-sulfonyl)-hexahydro-pyrrolo[3,2-*b*]pyrrole-1-carbonyl]-butyl}-4-(4-methyl-piperazin-1-yl)-benzamide;

196. 4-Methyl-N-{1-[6-oxo-4-(pyridine-3-sulfonyl)-hexahydro-pyrrolo[3,2-*b*]pyrrole-1-carbonyl]-butyl}-benzamide;

197. 4-Methoxy-N-{1-[6-oxo-4-(pyridine-3-sulfonyl)-hexahydro-pyrrolo[3,2-*b*]pyrrole-1-carbonyl]-butyl}-benzamide;

198. 4-Isopropyl-N-{1-[6-oxo-4-(pyridine-3-sulfonyl)-hexahydro-pyrrolo[3,2-*b*]pyrrole-1-carbonyl]-butyl}-benzamide;

199. N-{1-[6-oxo-4-(pyridine-3-sulfonyl)-hexahydro-pyrrolo[3,2-*b*]pyrrole-1-carbonyl]-butyl}-4-vinyl-benzamide;

200. 4-Imidazol-1-yl-N-{1-[6-oxo-4-(pyridine-3-sulfonyl)-hexahydro-pyrrolo[3,2-*b*]pyrrole-1-carbonyl]-butyl}-benzamide;

201. N-{1-[6-oxo-4-(pyridine-3-sulfonyl)-hexahydro-pyrrolo[3,2-*b*]pyrrole-1-carbonyl]-butyl}-4-thiophen-2-yl-benzamide;

202. N-{1-[6-oxo-4-(pyridine-3-sulfonyl)-hexahydro-pyrrolo[3,2-*b*]pyrrole-1-carbonyl]-butyl}-4-oxazol-5-yl-benzamide;

203. 5-Phenyl-thiophene-2-carboxylic acid {1-[6-oxo-4-(pyridine-3-sulfonyl)-hexahydro-pyrrolo[3,2-*b*]pyrrole-1-carbonyl]-butyl}-amide;

204. N-{1-[6-oxo-4-(pyridine-3-sulfonyl)-hexahydro-pyrrolo[3,2-*b*]pyrrole-1-carbonyl]-butyl}-4-trifluoromethoxy-benzamide;

205. 5-Pyridin-2-yl-thiophene-2-carboxylic acid {1-[6-oxo-4-(pyridine-3-sulfonyl)-hexahydro-pyrrolo[3,2-*b*]pyrrole-1-carbonyl]-butyl}-amide;

206. 4-Difluoromethoxy-N-{1-[6-oxo-4-(pyridine-3-sulfonyl)-hexahydro-pyrrolo[3,2-*b*]pyrrole-1-carbonyl]-butyl}-benzamide;

207. N-{1-[6-oxo-4-(pyridine-3-sulfonyl)-hexahydro-pyrrolo[3,2-*b*]pyrrole-1-carbonyl]-butyl}-4-morpholin-4-yl-benzamide;

208. Naphthalene-2-carboxylic acid {3-methyl-1-[6-oxo-4-(1-oxy-pyridine-3-sulfonyl)hexahydro-pyrrolo[3,2-*b*]pyrrole-1-carbonyl]-butyl}-amide;

209. Quinoline-2-carboxylic acid {3-methyl-1-[6-oxo-4-(1-oxy-pyridine-3-sulfonyl)-hexahydro-pyrrolo[3,2-*b*]pyrrole-1-carbonyl]-butyl}-amide;

210. Benzo[b]thiophene-2-carboxylic acid {3-methyl-1-[6-oxo-4-(1-oxy-pyridine-3-sulfonyl)-hexahydro-pyrrolo[3,2-*b*]pyrrole-1-carbonyl]-butyl}-amide;

211. Benzofuran-2-carboxylic acid {3-methyl-1-[6-oxo-4-(1-oxy-pyridine-3-sulfonyl)-hexahydro-pyrrolo[3,2-*b*]pyrrole-1-carbonyl]-butyl}-amide;

212. Biphenyl-4-carboxylic acid {3-methyl-1-[6-oxo-4-(1-oxy-pyridine-3-sulfonyl)-hexahydro-pyrrolo[3,2-*b*]pyrrole-1-carbonyl]-butyl}-amide;

213. 4-*tert*-Butyl-N-{3-methyl-1-[6-oxo-4-(1-oxy-pyridine-3-sulfonyl)-hexahydro-pyrrolo[3,2-*b*]pyrrole-1-carbonyl]-butyl}-benzamide;

214. 4-Dimethylamino-N-{3-methyl-1-[6-oxo-4-(1-oxy-pyridine-3-sulfonyl)-hexahydro-pyrrolo[3,2-*b*]pyrrole-1-carbonyl]-butyl}-benzamide;

215. 7-Methoxy-benzofuran-2-carboxylic acid {3-methyl-1-[6-oxo-4-(1-oxy-pyridine-3-sulfonyl)-hexahydro-pyrrolo[3,2-*b*]pyrrole-1-carbonyl]-butyl}-amide;

216. {3-Methyl-1-[6-oxo-4-(1-oxy-pyridine-3-sulfonyl)-hexahydro-pyrrolo[3,2-*b*]pyrrole-1-carbonyl]-butyl}-carbamic acid benzyl ester;

217. 5-Methoxy-benzofuran-2-carboxylic acid {3-methyl-1-[6-oxo-4-(1-oxy-pyridine-3-sulfonyl)-hexahydro-pyrrolo[3,2-*b*]pyrrole-1-carbonyl]-butyl}-amide;

218. Thieno[3,2-*b*]thiophene-2-carboxylic acid {3-methyl-1-[6-oxo-4-(1-oxy-pyridine-3-sulfonyl)-hexahydro-pyrrolo[3,2-*b*]pyrrole-1-carbonyl]-butyl}-amide;

219. 3-Methyl-benzofuran-2-carboxylic acid {3-methyl-1-[6-oxo-4-(1-oxy-pyridine-3-sulfonyl)-hexahydro-pyrrolo[3,2-*b*]pyrrole-1-carbonyl]-butyl}-amide;

220. Quinoxaline-2-carboxylic acid {3-methyl-1-[6-oxo-4-(1-oxy-pyridine-3-sulfonyl)-hexahydro-pyrrolo[3,2-*b*]pyrrole-1-carbonyl]-butyl}-amide;

221. Benzo[1,3]dioxole-5-carboxylic acid {3-methyl-1-[6-oxo-4-(1-oxy-pyridine-3-sulfonyl)-hexahydro-pynolo[3,2-*b*]pyrrole-1-carbonyl]-butyl}-amide;

222. Quinoline-6-carboxylic acid {3-methyl-1-[6-oxo-4-(1-oxy-pyridine-3-sulfonyl)-hexahydro-pyrrolo[3,2-*b*]pyrrole-1-carbonyl]-butyl}-amide;

223. Furan-2-carboxylic acid {3-methyl-1-[6-oxo-4-(1-oxy-pyridine-3-sulfonyl)-hexahydro-pyuolo[3,2-*b*]pyrrole-1-carbonyl]-butyl}-amide;

224. Thiophene-2-carboxylic acid {3-methyl-1-[6-oxo-4-(1-oxy-pyridine-3-sulfonyl)-hexahydro-pyrrolo[3,2-*b*]pyrrole-1-carbonyl]-butyl}-amide;

225. N-{3-Methyl-1-[6-oxo-4-(1-oxy-pyridine-3-sulfonyl)-hexahydro-pyrrolo[3,2-*b*]pyrrole-1-carbonyl]-butyl}-4-trifluoromethyl-benzamide;

226. Furan-3-carboxylic acid {3-methyl-1-[6-oxo-4-(1-oxy-pyridine-3-sulfonyl)-hexahydro-pyrrolo[3,2-*b*]pyrrole-1-carbonyl]-butyl}-amide;

227. Thiophene-3-carboxylic acid {3-methyl-1-[6-oxo-4-(1-oxy-pyridine-3-sulfonyl)-hexahydro-pyrrolo[3,2-*b*]pyrrole-1-carbonyl]-butyl}-amide;

228. N-{3-Methyl-1-[6-oxo-4-(1-oxy-pyridine-3-sulfonyl)-hexahydro-pyrrolo[3,2-*b*]pyrrole-1-carbonyl]-butyl}-4-phenoxy-benzamide;

229. N-(3-Methyl-1-[6-oxo-4-(1-oxy-pyridine-3-sulfonyl)-hexahydro-pyrrolo[3,2-*b*]pyrrole-1-carbonyl]-butyl}-4-(4-methyl-piperazin-1-yl)-benzamide;

230. 4-Methyl-N-{3-methyl-1-[6-oxo-4-(1-oxy-pyridine-3-sulfonyl)-hexahydro-pyrrolo[3,2-*b*]pyrrole-1-carbonyl]-butyl}-benzamide;

231. 4-Methoxy-N-{3-methyl-1-[6-oxo-4-(1-oxy-pyridine-3-sulfonyl)-hexahydro-prrolo[3,2-*b*]pyrrole-1-carbonyl]-butyl}-benzamide;

232. 4-Isopropyl-N- {3-methyl-1-[6-oxo-4-(1-oxy-pyridine-3-sulfonyl)-hexahydro-pyrrolo[3,2-*b*]pyrrole-1-carbonyl]-butyl}-benzamide;

233. N-{3-Methyl-1-[6-oxo-4-(1-oxy-pyridine-3-sulfonyl)-hexahydro-pyrrolo[3,2-*b*]pyrrole-1-carbonyl]-butyl}-4-vinyl-benzamide;

234. 4-Imidazol-1-yl-N-{3-methyl-1-[6-oxo-4-(1-oxy-pyridine-3-sulfonyl)-hexahydro-pyrrolo[3,2-*b*]pyrrole-1-carbonyl]-butyl}-benzamide;

235. N- {3-Methyl-1-[6-oxo-4-(1-oxy-pyridine-3-sulfonyl)-hexahydro-pyrrolo[3,2-*b*]pyrrole-1-carbonyl]-butyl}-4-thiophen-2-yl-benzamide;

236. N-{3-Methyl-1-[6-oxo-4-(1-oxy-pyridine-3-sulfonyl)-hexahydro-pyrrolo[3,2-*b*]pyrrole-1-carbonyl]-butyl}-4-oxazol-5-yl-benzamide;

237. 5-Phenyl-thiophene-2-carboxylic acid {3-methyl-1-[6-oxo-4-(1-oxy-pyridine-3-sulfonyl)-hexahydro-pyrrolo[3,2-*b*]pyrrole-1-carbonyl]-butyl}-amide;

238. N-{3-Methyl-1-[6-oxo-4-(1-oxy-pyridine-3-sulfonyl)-hexahydro-pyrrolo[3,2-*b*]pyrrole-1-carbonyl]-butyl}-4-trifluoromethoxy-benzamide;

239. 5-Pyridin-2-yl-thiophene-2-carboxylic acid {3-methyl-1-[6-oxo-4-(1-oxy-pyridine-3-sulfonyl)-hexahydro-pyrrolo[3,2-*b*]pyrrole-1-carbonyl]-butyl}-amide;

240. 4-Difluoromethoxy-N- {3-methyl-1-[6-oxo-4-(1-oxy-pyridine-3-sulfonyl)-hexahydro-pyrrolo[3,2-*b*]pyrrole-1-carbonyl]-butyl}-benzamide;

241. N-{3-Methyl-1-[6-oxo-4-(1-oxy-pyridine-3-sulfonyl)-hexahydro-pyrrolo[3,2-*b*]pyrrole-1-carbonyl]-butyl}-4-morpholin-4-yl-benzamide;

242. Naphthalene-2-carboxylic acid {1-[6-oxo-4-(1-oxy-pyridine-3-sulfonyl)hexahydro-pyrrolo[3,2-*b*]pyrrole-1-carbonyl]-butyl}-amide;

243. Quinoline-2-carboxylic acid {1-[6-oxo-4-(1-oxy-pyridine-3-sulfonyl)-hexahydro-pyrrolo[3,2-*b*]pyrrole-1-carbonyl]-butyl}-amide;

244. Benzo[b]thiophene-2-carboxylic acid {1-[6-oxo-4-(1-oxy-pyridine-3-sulfonyl)-hexahydro-pyrrolo[3,2-*b*]pyrrole-1-carbonyl]-butyl}-amide;

245. Benzofuran-2-carboxylic acid {1-[6-oxo-4-(1-oxy-pyridine-3-sulfonyl)-hexahydro-pyrrolo[3,2-*b*]pyrrole-1-carbonyl]-butyl}-amide;

246. Biphenyl-4-carboxylic acid {1-[6-oxo-4-(1-oxy-pyridine-3-sulfonyl)-hexahydro-pyrrolo[3,2-*b*]pyrrole-1-carbonyl]-butyl}-amide;

247. 4-*tert*-Butyl-N-{1-[6-oxo-4-(1-oxy-pyridine-3-sulfonyl)-hexahydro-pyrrolo[3,2-*b*]pyrrole-1-carbonyl]-butyl}-benzamide;

248. 4-Dimethylamino-N-{1-[6-oxo-4-(1-oxy-pyridine-3-sulfonyl)-hexahydro-pyrrolo[3,2-*b*]pyrrole-1-carbonyl]-butyl}-benzamide;

249. 7-Methoxy-benzofuran-2-carboxylic acid {1-[6-oxo-4-(1-oxy-pyridine-3-sulfonyl)-hexahydro-pyrrolo[3,2-*b*]pyrrole-1-carbonyl]-butyl}-amide;

250. {1-[6-oxo-4-(1-oxy-pyridine-3-sulfonyl)-hexahydro-pyrrolo[3,2-*b*]pyrrole-1-carbonyl]-butyl}-carbamic acid benzyl ester;

251. 5-Methoxy-benzofuran-2-carboxylic acid {1-[6-oxo-4-(1-oxy-pyridine-3-sulfonyl)-hexahydro-pyrrolo[3,2-*b*]pyrrole-1-carbonyl]-butyl}-amide;

252. Thieno[3,2-*b*]thiophene-2-carboxylic acid {1-[6-oxo-4-(1-oxy-pyridine-3-sulfonyl)-hexahydro-pyrrolo[3,2-*b*]pyrrole-1-carbonyl]-butyl}-amide;

253. 3-Methyl-benzofuran-2-carboxylic acid {1-[6-oxo-4-(1-oxy-pyridine-3-sulfonyl)-hexahydro-pyrrolo[3,2-*b*]pyrrole-1-carbonyl]-butyl}-amide;

254. Quinoxaline-2-carboxylic acid {1-[6-oxo-4-(1-oxy-pyridine-3-sulfonyl)-hexahydro-pyrrolo[3,2-*b*]pyrrole-1-carbonyl]-butyl}-amide;

255. Benzo[1,3]dioxole-5-carboxylic acid {1-[6-oxo-4-(1-oxy-pyridine-3-sulfonyl)-hexahydro-pyrrolo[3,2-*b*]pyrrole-1-carbonyl]-butyl}-amide;

256. Quinoline-6-carboxylic acid {1-[6-oxo-4-(1-oxy-pyridine-3-sulfonyl)-hexahydro-pyrrolo[3,2-*b*]pyrrole-1-carbonyl]-butyl}-amide;

257. Furan-2-carboxylic acid {1-[6-oxo-4-(1-oxy-pyridine-3-sulfonyl)-hexahydro-pyrrolo[3,2-*b*]pyrrole-1-carbonyl]-butyl}-amide;

258. Thiophene-2-carboxylic acid {1-[6-oxo-4-(1-oxy-pyridine-3-sulfonyl)-hexahydro-pyrrolo[3,2-*b*]pyrrole-1-carbonyl]-butyl}-amide;

259. N-{1-[6-oxo-4-(1-oxy-pyridine-3-sulfonyl)-hexahydro-pyrrolo[3,2-*b*]pyrrole-1-carbonyl]-butyl}-4-trifluoromethyl-benzamide;

260. Furan-3-carboxylic acid {1-[6-oxo-4-(1-oxy-pyridine-3-sulfonyl)-hexahydro-pyrrolo[3,2-*b*]pyrrole-1-carbonyl]-butyl}-amide;

261. Thiophene-3-carboxylic acid {1-[6-oxo-4-(1-oxy-pyridine-3-sulfonyl)-hexahydro-pyrrolo[3,2-*b*]pyrrole-1-carbonyl]-butyl}-amide;

262. N-{1-[6-oxo-4-(1-oxy-pyridine-3-sulfonyl)-hexahydro-pyrrolo[3,2-*b*]pyrrole-1-carbonyl]-butyl}-4-phenoxy-benzamide;

263. N-{1-[6-oxo-4-(1-oxy-pyridine-3-sulfonyl)-hexahydro-pyrrolo[3,2-*b*]pyrrole-1-carbonyl]-butyl}-4-(4-methyl-piperazin-1-yl)-benzamide;

264. 4-Methyl-N-{1-[6-oxo-4-(1-oxy-pyridine-3-sulfonyl)-hexahydro-pyrrolo[3,2-*b*]pyrrole-1-carbonyl]-butyl}-benzamide;

265. 4-Methoxy-N-{1-[6-oxo-4-(1-oxy-pyridine-3-sulfonyl)-hexahydro-pyrrolo[3,2-*b*]pyrrole-1-carbonyl]-butyl}-benzamide;

266. 4-Isopropyl-N- {1-[6-oxo-4-(1-oxy-pyridine-3-sulfonyl)-hexahydro-pyrrolo[3,2-*b*]pyrrole-1-carbonyl]-butyl}-benzamide;

267. N- {1-[6-oxo-4-(1-oxy-pyridine-3-sulfonyl)-hexahydro-pyrrolo[3,2-*b*]pyrrole-1-carbonyl]-butyl}-4-vinyl-benzamide;

268. 4-Imidazol-1-yl-N-{1-[6-oxo-4-(1-oxy-pyridine-3-sulfonyl)-hexahydro-pyrrolo[3,2-*b*]pyrrole-1-carbonyl]-butyl}-benzamide;

269. N-{1-[6-oxo-4-(1-oxy-pyridine-3-sulfonyl)-hexahydro-pyrrolo[3,2-*b*]pyrrole-1-carbonyl]-butyl}-4-thiophen-2-yl-benzamide;

270. N-{1-[6-oxo-4-(1-oxy-pyridine-3-sulfonyl)-hexahydro-pyrolo[3,2-*b*]pyrrole-1-carbonyl]-butyl}-4-oxazol-5-yl-benzamide;

271. 5-Phenyl-thiophene-2-carboxylic acid {1-[6-oxo-4-(1-oxy-pyridine-3-sulfonyl)-hexahydro-pyrrolo[3,2-*b*]pyrrole-1-carbonyl]-butyl}-amide;

272. N-{1-[6-oxo-4-(1-oxy-pyridine-3-sulfonyl)-hexahydro-pyrrolo[3,2-*b*]pyrrole-1-carbonyl]-butyl}-4-trifluoromethoxy-benzamide;

273. 5-Pyridin-2-yl-thiophene-2-carboxylic acid {1-[6-oxo-4-(1-oxy-pyridine-3-sulfonyl)-hexahydro-pyrrolo[3,2-*b*]-pyrrole-1-carbonyl]-butyl}-amide;

274. 4-Difluoromethoxy-N- {1-[6-oxo-4-(1-oxy-pyridine-3-sulfonyl)-hexahydro-pyrrolo[3,2-*b*]pyrrole-1-carbonyl]-butyl}-benzamide;

275. N-{1-[6-oxo-4-(1-oxy-pyridine-3-sulfonyl)-hexahydro-pyrrolo[3,2-*b*]pyrrole-1-carbonyl]-butyl}-4-morpholin-4-yl-benzamide;

276. Naphthalene-2-carboxylic acid {3-methyl-1-[6-oxo-4-(pyridine-2-carbonyl)hexahydro-pyrrolo[3,2-*b*]pyrrole-1-carbonyl]-butyl}-amide;

277. Quinoline-2-carboxylic acid {3-methyl-1-[6-oxo-4-(pyridine-2-carbonyl)-hexahydro-pyrrolo[3,2- *b*]pyrrole-1-carbonyl]-butyl}-amide;

278. Benzo[b]thiophene-2-carboxylic acid {3-methyl-1-[6-oxo-4-(pyridine-2-carbonyl)-hexahydro-pyrrolo[3,2-*b*]pyrrole-1-carbonyl]-butyl}-amide;

279. Benzofuran-2-carboxylic acid {3-methyl-1-[6-oxo-4-(pyridine-2-carbonyl)-hexahydro-pyrrolo[3,2-*b*]pyrrole-1-carbonyl]-butyl}-amide;

280. Biphenyl-4-carboxylic acid {3-methyl-1-[6-oxo-4-(pyridine-2-carbonyl)-hexahydro-pyrrolo[3,2-*b*]pyrrole-1-carbonyl]-butyl}-amide;

281. 4-*tert*-Butyl-N-{3-methyl-1-[6-oxo-4-(pyridine-2-carbonyl)-hexahydro-pyrrolo[3,2- *b*]pyrrole-1-carbonyl]-butyl}-benzamide;

282. 4-Dimethylamino-N-{3-methyl-1-[6-oxo-4-(pyridine-2-carbonyl)-hexahydro-pyrrolo[3,2-*b*]pyrrole-1-carbonyl]-butyl}-benzamide;

283. 7-Methoxy-benzofuran-2-carboxylic acid {3-methyl-1-[6-oxo-4-(pyridine-2-carbonyl)-hexahydro-pyrrolo[3,2-*b*]-pyrrole-1-carbonyl]-butyl}-amide;

284. {3-Methyl-1-[6-oxo-4-(pyridine-2-carbonyl)-hexahydro-pyrrolo[3,2-*b*]pyrrole-1-carbonyl]-butyl}-carbamic acid benzyl ester;

285. 5-Methoxy-benzofuran-2-carboxylic acid {3-methyl-1-[6-oxo-4-(pyridine-2-carbonyl)-hexahydro-pyrrolo[3,2-*b*]-pyrrole-1-carbonyl]-butyl}-amide;

286. Thieno[3,2-*b*]thiophene-2-carboxylic acid {3-methyl-1-[6-oxo-4-(pyridine-2-carbonyl)-hexahydro-pyrrolo[3,2-*b*]pyrrole-1-carbonyl]-butyl}-amide;

287. 3-Methyl-benzofuran-2-carboxylic acid {3-methyl-1-[6-oxo-4-(pyridine-2-carbonyl)-hexahydro-pyrrolo[3,2-*b*]-pyrrole-1-carbonyl]-butyl}-amide;

288. Quinoxaline-2-carboxylic acid {3-methyl-1-[6-oxo-4-(pyridine-2-carbonyl)-hexahydro-pyrrolo[3,2-*b*]pyrrole-1-carbonyl]-butyl}-amide;

289. Benzo[1,3]dioxole-5-carboxylic acid {3-methyl-1-[6-oxo-4-(pyridine-2-carbonyl)-hexahydro-pyrrolo[3,2-*b*]pyrrole-1-carbonyl]-butyl}-amide;

290. Quinoline-6-carboxylic acid {3-methyl-1-[6-oxo-4-(pyridine-2-carbonyl)-hexahydro-pyrrolo[3,2-*b*]pyrrole-1-carbonyl]-butyl}-amide;

291. Furan-2-carboxylic acid {3-methyl-1-[6-oxo-4-(pyridine-2-carbonyl)-hexahydro-pyrrolo[3,2-*b*]pyrrole-1-carbonyl]-butyl}-amide;

292. Thiophene-2-carboxylic acid {3-methyl-1-[6-oxo-4-(pyridine-2-carbonyl)-hexahydro-pyrrolo[3,2-*b*]pyrrole-1-carbonyl]-butyl}-amide;

293. N-{3-Methyl-1-[6-oxo-4-(pyridine-2-carbonyl)-hexahydro-pyrrolo[3,2-*b*]pyrrole-1-carbonyl]-butyl}-4-trifluoromethyl-benzamide;

294. Furan-3-carboxylic acid {3-methyl-1-[6-oxo-4-(pyridine-2-carbonyl)-hexahydro-pyrrolo[3,2-*b*]pyrrole-1-carbonyl]-butyl}-amide;

295. Thiophene-3-carboxylic acid {3-methyl-1-[6-oxo-4-(pyridine-2-carbonyl)-hexahydro-pyrrolo[3,2-*b*]pyrrole-1-carbonyl]-butyl}-amide;

296. N-{3-Methyl-1-[6-oxo-4-(pyridine-2-carbonyl)-hexahydro-pyrrolo[3,2-*b*]pyrrole-1-carbonyl]-butyl}-4-phenoxy-benzamide;

297. N-{3-Methyl-1-[6-oxo-4-(pyridine-2-carbonyl)-hexahydro-pyrrolo[3,2-*b*]pyrrole-1-carbonyl]-butyl}-4-(4-methyl-piperazin-1-yl)-benzamide;

298. 4-Methyl-N-{3-methyl-1-[6-oxo-4-(pyridine-2-carbonyl)-hexahydro-pyrrolo[3,2-*b*]pyrrole-1-carbonyl]-butyl}-benzamide;

299. 4-Methoxy-N-{3-methyl-1-[6-oxo-4-(pyridine-2-carbonyl)-hexahydro-pyrrolo[3,2-*b*]pyrrole-1-carbonyl]-butyl}-benzamide;

300. 4-Isopropyl-N-{3-methyl-1-[6-oxo-4-(pyridine-2-carbonyl)-hexahydro-pyrrolo[3,2-*b*]pyrrole-1-carbonyl]-butyl}-benzamide;

301. N-{3-Methyl-1-[6-oxo-4-(pyridine-2-carbonyl)-hexahydro-pyrrolo[3,2-*b*]pyrrole-1-carbonyl]-butyl}-4-vinyl-benzamide;

302. 4-Imidazol-1-yl-N-{3-methyl-1-[6-oxo-4-(pyridine-2-carbonyl)-hexahydro-pyrrolo[3,2-*b*]pyrrole-1-carbonyl]-butyl}-benzamide;

303. N-{3-Methyl-1-[6-oxo-4-(pyridine-2-carbonyl)-hexahydro-pyrrolo[3,2-*b*]pyrrole-1-carbonyl]-butyl}-4-thiophen-2-yl-benzamide;

304. N-{3-Methyl-1-[6-oxo-4-(pyridine-2-carbonyl)-hexahydro-pyrrolo[3,2-*b*]pyrrole-1-carbonyl]-butyl}-4-oxazol-5-yl-benzamide;

305. 5-Phenyl-thiophene-2-carboxylic acid {3-methyl-1-[6-oxo-4-(pyridine-2-carbonyl)-hexahydro-pyrrolo[3,2-*b*]pyrrole-1-carbonyl]-butyl}-amide;

306. N-{3-Methyl-1-[6-oxo-4-(pyridine-2-carbonyl)-hexahydro-pyrrolo[3,2-*b*]pyrrole-1-carbonyl]-butyl}-4-trifluoromethoxy-benzamide;

307. 5-Pyridin-2-yl-thiophene-2-carboxylic acid {3-methyl-1-[6-oxo-4-(pyridine-2-carbonyl)-hexahydro-pyrrolo[3,2-*b*]pyrrole-1-carbonyl]-butyl}-amide;

308. 4-Difluoromethoxy-N-{3-methyl-1-[6-oxo-4-(pyridine-2-carbonyl)-hexahydro-pyrrolo[3,2-*b*]pyrrole-1-carbonyl]-butyl}-benzamide;

309. N-{3-Methyl-1-[6-oxo-4-(pyridine-2-carbonyl)-hexahydro-pyrrolo[3,2-*b*]pyrrole-1-carbonyl]-butyl}-4-morpholin-4-yl-benzamide;

310. Naphthalene-2-carboxylic acid {1-[6-oxo-4-(pyridine-2-carbonyl)hexahydro-pyrrolo[3,2-*b*]pyrrole-1-carbonyl]-butyl}-amide;

311. Quinoline-2-carboxylic acid {1-[6-oxo-4-(pyridine-2-carbonyl)-hexahydro-pyrrolo[3,2-*b*]pyrrole-1-carbonyl]-butyl}-amide;

312. Benzo[b]thiophene-2-carboxylic acid {1-[6-oxo-4-(pyridine-2-carbonyl)-hexahydro-pyrrolo[3,2-*b*]pyrrole-1-carbonyl]-butyl}-amide;

313. Benzofuran-2-carboxylic acid {1-[6-oxo-4-(pyridine-2-carbonyl)-hexahydro-pyrrolo[3,2-*b*]pyrrole-1-carbonyl]-butyl}-amide;

314. Biphenyl-4-carboxylic acid {1-[6-oxo-4-(pyridine-2-carbonyl)-hexahydro-pyrrolo[3,2-*b*]pyrrole-1-carbonyl]-butyl}-amide;

315. 4-*tert*-Butyl-N-{1-[6-oxo-4-(pyridine-2-carbonyl)-hexahydro-pyrrolo[3,2-*b*]pyrrole-1-carbonyl]-butyl}-benzamide;

316. 4-Dimethylamino-N-{1-[6-oxo-4-(pyridine-2-carbonyl)-hexahydro-pyrrolo[3,2-*b*]pyrrole-1-carbonyl]-butyl}-benzamide;

317. 7-Methoxy-benzofuran-2-carboxylic acid {1-[6-oxo-4-(pyridine-2-carbonyl)-hexahydro-pyrrolo[3,2-*b*]pyrrole-1-carbonyl]-butyl}-amide;

318. {1-[6-oxo-4-(pyridine-2-carbonyl)-hexahydro-pyrrolo[3,2-*b*]pyrrole-1-carbonyl]-butyl}-carbamic acid benzyl ester;

319. 5-Methoxy-benzofuran-2-carboxylic acid {1-[6-oxo-4-(pyridine-2-carbonyl)-hexahydro-pyrrolo[3,2-*b*]pyrrole-1-carbonyl]-butyl}-amide;

320. Thieno[3,2-*b*]thiophene-2-carboxylic acid {1-[6-oxo-4-(pyridine-2-carbonyl)-hexahydro-pyrrolo[3,2-*b*]pyrrole-1-carbonyl]-butyl}-amide;

321. 3-Methyl-benzofuran-2-carboxylic acid {1-[6-oxo-4-(pyridine-2-carbonyl)-hexahydro-pyrrolo[3,2-*b*]pyrrole-1-carbonyl]-butyl}-amide;

322. Quinoxaline-2-carboxylic acid {1-[6-oxo-4-(pyridine-2-carbonyl)-hexahydro-pyrrolo[3,2-*b*]pyrrole-1-carbonyl]-butyl}-amide;

323. Benzo[1,3]dioxole-5-carboxylic acid {1-[6-oxo-4-(pyridine-2-carbonyl)-hexahydro-pyrrolo[3,2-*b*]pyrrole-1-carbonyl]-butyl}-amide;

324. Quinoline-6-carboxylic acid {1-[6-oxo-4-(pyridine-2-carbonyl)-hexahydro-pyrrolo[3,2-*b*]pyrrole-1-carbonyl]-butyl}-amide;

325. Furan-2-carboxylic acid {1-[6-oxo-4-(pyridine-2-carbonyl)-hexahydro-pyrrolo[3,2-*b*]pyrrole-1-carbonyl]-butyl}-amide;

326. Thiophene-2-carboxylic acid {1-[6-oxo-4-(pyridine-2-carbonyl)-hexahydro-pyrrolo[3,2-*b*]pyrrole-1-carbonyl]-butyl}-amide;

327. N-{1-[6-oxo-4-(pyridine-2-carbonyl)-hexahydro-pyrrolo[3,2-*b*]pyrrole-1-carbonyl]-butyl}-4-trifluoromethyl-benzamide;

328. Furan-3-carboxylic acid {1-[6-oxo-4-(pyridine-2-carbonyl)-hexahydro-pyrrolo[3,2-*b*]pyrrole-1-carbonyl]-butyl}-amide;

329. Thiophene-3-carboxylic acid {1-[6-oxo-4-(pyridine-2-carbonyl)-hexahydro-pyrrolo[3,2-*b*]pyrrole-1-carbonyl]-butyl}-amide;

330. N-{1-[6-oxo-4-(pyridine-2-carbonyl)-hexahydro-pyrrolo[3,2-*b*]pyrrole-1-carbonyl]-butyl}-4-phenoxy-benzamide;

331. N-{1-[6-oxo-4-(pyridine-2-carbonyl)-hexahydro-pyrrolo[3,2-*b*]pyrrole-1-carbonyl]-butyl}-4-(4-methyl-piperazin-1-yl)-benzamide;

332. 4-Methyl-N-{1-[6-oxo-4-(pyridine-2-carbonyl)-hexahydro-pyrrolo[3,2-*b*]pyrrole-1-carbonyl]-butyl}-benzamide;

333. 4-Methoxy-N-{1-[6-oxo-4-(pyridine-2-carbonyl)-hexahydro-pyrrolo[3,2-*b*]pyrrole-1-carbonyl]-butyl}-benzamide;

334. 4-Isopropyl-N-{1-[6-oxo-4-(pyridine-2-carbonyl)-hexahydro-pyrrolo[3,2-*b*]pyrrole-1-carbonyl]-butyl}-benzamide;

335. N-{1-[6-oxo-4-(pyridine-2-carbonyl)-hexahydro-pyrrolo[3,2-*b*]pyrrole-1-carbonyl]-butyl}-4-vinyl-benzamide;

336. 4-Imidazol-1-yl-N-{1-[6-oxo-4-(pyridine-2-carbonyl)-hexahydro-pyrrolo[3,2-*b*]pyrrole-1-carbonyl]-butyl}-benzamide;

337. N-{1-[6-oxo-4-(pyridine-2-carbonyl)-hexahydro-pyrrolo[3,2-*b*]pyrrole-1-carbonyl]-butyl}-4-thiophen-2-yl-benzamide;

338. N-{1-[6-oxo-4-(pyridine-2-carbonyl)-hexahydro-pyrrolo[3,2-*b*]pyrrole-1-carbonyl]-butyl}-4-oxazol-5-yl-benzamide;

339. 5-Phenyl-thiophene-2-carboxylic acid {1-[6-oxo-4-(pyridine-2-carbonyl)-hexahydro-pyrrolo[3,2-*b*]pyrrole-1-carbonyl]-butyl}-amide;

340. N-{1-[6-oxo-4-(pyridine-2-carbonyl)-hexahydro-pyrrolo[3,2-*b*]pyrrole-1-carbonyl]-butyl}-4-trifluoromethoxy-benzamide;

341. 5-Pyridin-2-yl-thiophene-2-carboxylic acid {1-[6-oxo-4-(pyridine-2-carbonyl)-hexahydro-pyrrolo[3,2-*b*]pyrrole-1-carbonyl]-butyl}-amide;

342. 4-Difluoromethoxy-N- {1-[6-oxo-4-(pyridine-2-carbonyl)-hexahydro-pyrrolo[3,2-*b*]pyrrole-1-carbonyl]-butyl}-benzamide;

343. N-{1-[6-oxo-4-(pyridine-2-carbonyl)-hexahydro-pyrrolo[3,2-*b*]pyrrole-1-carbonyl]-butyl}-4-morpholin-4-yl-benzamide;

344. Naphthalene-2-carboxylic acid {3-methyl-1-[6-oxo-4-(2-pyridin-3-yl-acetyl)hexahydro-pyrrolo[3,2-*b*]pyrrole-1-carbonyl]-butyl}-amide;

345. Quinoline-2-carboxylic acid {3-methyl-1-[6-oxo-4-(2-pyridin-3-yl-acetyl)-hexahydro-pyrrolo[3,2-*b*]pyrrole-1-carbonyl]-butyl}-amide;

346. Benzo[b]thiophene-2-carboxylic acid {3-methyl-1-[6-oxo-4-(2-pyridin-3-yl-acetyl)-hexahydro-pyrrolo[3,2-*b*]pyrrole-1-carbonyl]-butyl}-amide;

347. Benzofuran-2-carboxylic acid {3-methyl-1-[6-oxo-4-(2-pyridin-3-yl-acetyl)-hexahydro-pyrrolo[3,2-*b*]pyrrole-1-carbonyl]-butyl}-amide;

348. Biphenyl-4-carboxylic acid {3-methyl-1-[6-oxo-4-(2-pyridin-3-yl-acetyl)-hexahydro-pyrrolo[3,2-*b*]pyrrole-1-carbonyl]-butyl}-amide;

349. 4-*tert*-Butyl-N-{3-methyl-1-[6-oxo-4-(2-pyridin-3-yl-acetyl)-hexahydro-pyrrolo[3,2-*b*]pyrrole-1-carbonyl]-butyl}-benzamide;

350. 4-Dimethylamino-N-{3-methyl-1-[6-oxo-4-(2-pyridin-3-yl-acetyl)-hexahydro-pyrrolo[3,2-*b*]pyrrole-1-carbonyl]-butyl}-benzamide;

351. 7-Methoxy-benzofuran-2-carboxylic acid {3-methyl-1-[6-oxo-4-(2-pyridin-3-yl-acetyl)-hexahydro-pyrrolo[3,2-*b*]pyrrole-1-carbonyl]-butyl}-amide;

352. {3-Methyl-1-[6-oxo-4-(2-pyridin-3-yl-acetyl)-hexahydro-pyrrolo[3,2-*b*]pyrrole-1-carbonyl]-butyl}-carbamic acid benzyl ester;

353. 5-Methoxy-benzofuran-2-carboxylic acid {3-methyl-1-[6-oxo-4-(2-pyridin-3-yl-acetyl)-hexahydro-pyrrolo[3,2-*b*]pyrrole-1-carbonyl]-butyl}-amide;

354. Thieno[3,2-*b*]thiophene-2-carboxylic acid {3-methyl-1-[6-oxo-4-(2-pyridin-3-yl-acetyl)-hexahydro-pyrrolo[3,2-*b*]pyrrole-1-carbonyl]-butyl}-amide;

355. 3-Methyl-benzofuran-2-carboxylic acid {3-methyl-1-[6-oxo-4-(2-pyridin-3-yl-acetyl)-hexahydro-pyrrolo[3,2-*b*]pyrrole-1-carbonyl]-butyl}-amide;

356. Quinoxaline-2-carboxylic acid {3-methyl-1-[6-oxo-4-(2-pyridin-3-yl-acetyl)-hexahydro-pyrrolo[3,2-*b*]pyrrole-1-carbonyl]-butyl}-amide;

357. Benzo[1,3]dioxole-5-carboxylic acid {3-methyl-1-[6-oxo-4-(2-pyridin-3-yl-acetyl)-hexahydro-pyrrolo[3,2-*b*]pyrrole-1-carbonyl]-butyl}-amide;

358. Quinoline-6-carboxylic acid {3-methyl-1-[6-oxo-4-(2-pyridin-3-yl-acetyl)-hexahydro-pyrrolo[3,2-*b*]pyrrole-1-carbonyl]-butyl}-amide;

359. Furan-2-carboxylic acid {3-methyl-1-[6-oxo-4-(2-pyridin-3-yl-acetyl)-hexahydro-pyrrolo[3,2-*b*]pyrrole-1-carbonyl]-butyl}-amide;

360. Thiophene-2-carboxylic acid {3-methyl-1-[6-oxo-4-(2-pyridin-3-yl-acetyl)-hexahydro-pyrrolo[3,2-*b*]pyrrole-1-carbonyl]-butyl}-amide;

361. N-{3-Methyl-1-[6-oxo-4-(2-pyridin-3-yl-acetyl)-hexahydro-pyrrolo[3,2-*b*]pyrrole-1-carbonyl]-butyl}-4-trifluoromethyl-benzamide;

362. Furan-3-carboxylic acid {3-methyl-1-[6-oxo-4-(2-pyridin-3-yl-acetyl)-hexahydro-pyrrolo[3,2-*b*]pyrrole-1-carbonyl]-butyl}-amide;

363. Thiophene-3-carboxylic acid {3-methyl-1-[6-oxo-4-(2-pyridin-3-yl-acetyl)-hexahydro-pyrrolo[3,2- *b*]pyrrole-1-carbonyl]-butyl}-amide;

364. N-{3-Methyl-1-[6-oxo-4-(2-pyridin-3-yl-acetyl)-hexahydro-pyrrolo[3,2-*b*]pyrrole-1-carbonyl]-butyl}-4-phenoxy-benzamide;

365. N-{3-Methyl-1-[6-oxo-4-(2-pyridin-3-yl-acetyl)-hexahydro-pyrrolo[3,2-*b*]pyrrole-1-carbonyl]-butyl}-4-(4-methyl-piperazin-1-yl)-benzamide;

366. 4-Methyl-N- {3-methyl-1-[6-oxo-4-(2-pyridin-3-yl-acetyl)-hexahydro-pyrrolo[3,2-*b*]pyrrole-1-carbonyl]-butyl}- benzamide;

367. 4-Methoxy-N-{3-methyl-1-[6-oxo-4-(2-pyridin-3-yl-acetyl)-hexahydro-pyrrolo[3,2-*b*]pyrrole-1-carbonyl]-butyl}-benzamide;

368. 4-Isopropyl-N- {3-methyl-1-[6-oxo-4-(2-pyridin-3-yl-acetyl)-hexahydro-pyrrolo[3,2-*b*]pyrrole-1-carbonyl]-butyl}-benzamide;

369. N- {3-Methyl-1-[6-oxo-4-(2-pyridin-3-yl-acetyl)-hexahydro-pyrrolo[3,2-*b*]pyrrole-1-carbonyl]-butyl}-4-vinyl-benzamide;

370. 4-Imidazol-1-yl-N-{3-methyl-1-[6-oxo-4-(2-pyridin-3-yl-acetyl)-hexahydro-pyrrolo[3,2-*b*]pyrrole-1-carbonyl]-butyl}-benzamide;

371. N- {3-Methyl-1-[6-oxo-4-(2-pyridin-3-yl-acetyl)-hexahydro-pyrrolo[3,2-*b*]pyrrole-1-carbonyl]-butyl}-4-thiophen-2-yl-benzamide;

372. N-{3-Methyl-1-[6-oxo-4-(2-pyridin-3-yl-acetyl)-hexahydro-pyrrolo[3,2-*b*]pyrrole-1-carbonyl]-butyl}-4-oxazol-5-yl-benzamide;

373. 5-Phenyl-thiophene-2-carboxylic acid {3-methyl-1-[6-oxo-4-(2-pyridin-3-yl-acetyl)-hexahydro-pyrrolo[3,2-*b*]-pyrrole-1-carbonyl]-butyl}-amide;

374. N-{3-Methyl-1-[6-oxo-4-(2-pyridin-3-yl-acetyl)-hexahydro-pyrrolo[3,2-*b*]pyrrole-1-carbonyl]-butyl}-4-trifluoromethoxy-benzamide;

375. 5-Pyridin-2-yl-thiophene-2-carboxylic acid {3-methyl-1-[6-oxo-4-(2-pyridin-3-yl-acetyl)-hexahydro-pyrrolo[3,2-*b*]pyrrole-1-carbonyl]-butyl}-amide;

376. 4-Difluoromethoxy-N-{3-methyl-1-[6-oxo-4-(2-pyridin-3-yl-acetyl)-hexahydro-pyrrolo[3,2-*b*]pyrrole-1-carbonyl]-butyl}-benzamide;

377. N-{3-Methyl-1-[6-oxo-4-(2-pyridin-3-yl-acetyl)-hexahydro-pyrrolo[3,2-*b*]pyrrole-1-carbonyl]-butyl}-4-morpholin-4-yl-benzamide;

378. Naphthalene-2-carboxylic acid {1-[6-oxo-4-(2-pyridin-3-yl-acetyl)hexahydro-pyrrolo[3,2-*b*]pyrrole-1-carbonyl]-butyl -amide;

379. Quinoline-2-carboxylic acid {1-[6-oxo-4-(2-pyridin-3-yl-acetyl)-hexahydro-pyrrolo[3,2-*b*]pyrrole-1-carbonyl]-butyl}-amide;

380. Benzo[b]thiophene-2-carboxylic acid {1-[6-oxo-4-(2-pyridin-3-yl-acetyl)-hexahydro-pyrrolo[3,2-*b*]pyrrole-1-carbonyl]-butyl}-amide;

351. Benzofuran-2-carboxylic acid {1-[6-oxo-4-(2-pyridin-3-yl-acetyl)-hexahydro-pyrrolo[3,2-*b*]pyrrole-1-carbonyl]-butyl}-amide;

382. Biphenyl-4-carboxylic acid {1-[6-oxo-4-(2-pyridin-3-yl-acetyl)-hexahydro-pyrrolo[3,2-*b*]pyrrole-1-carbonyl]-butyl}-amide;

383. 4-*tert*-Butyl-N-{1-[6-oxo-4-(2-pyridin-3-yl-acetyl)-hexahydro-pyrrolo[3,2-*b*]pyrrole-1-carbonyl]-butyl}-benzamide;

384. 4-Dimethylamino-N-{1-[6-oxo-4-(2-pyridin-3-yl-acetyl)-hexahydro-pyrrolo[3,2-*b*]pyrrole-1-carbonyl]-butyl}-benzamide;

385. 7-Methoxy-benzofuran-2-carboxylic acid {1-[6-oxo-4-(2-pyridin-3-yl-acetyl)-hexahydro-pyrrolo[3,2-*b*]pyrrole-1-carbonyl]-butyl}-amide;

386. {1-[6-oxo-4-(2-pyridin-3-yl-acetyl)-hexahydro-pyrrolo[3,2-*b*]pyrrole-1-carbonyl]-butyl}-carbamic acid benzyl ester;

387. 5-Methoxy-benzofuran-2-carboxylic acid {1-[6-oxo-4-(2-pyridin-3-yl-acetyl)-hexahydro-pyrrolo[3,2-*b*]pyrrole-1-carbonyl]-butyl}-amide;

388. Thieno[3,2-*b*]thiophene-2-carboxylic acid {1-[6-oxo-4-(2-pyridin-3-yl-acetyl)-hexahydro-pyrrolo [3,2-*b*]pyrrole-1-carbonyl]-butyl}-amide;

389. 3-Methyl-benzofuran-2-carboxylic acid {1-[6-oxo-4-(2-pyridin-3-yl-acetyl)-hexahydro-pyrrolo[3,2-*b*]pyrrole-1-carbonyl]-butyl}-amide;

390. Quinoxaline-2-carboxylic acid {1-[6-oxo-4-(2-pyridin-3-yl-acetyl)-hexahydro-pyrrolo[3,2-*b*]pyrrole-1-carbonyl]-butyl}-amide;

391. Benzo[1,3]dioxole-5-carboxylic acid {1-[6-oxo-4-(2-pyridin-3-yl-acetyl)-hexahydro-pyrrolo[3,2-*b*]pyrrole-1-carbonyl]-butyl}-amide;

392. Quinoline-6-carboxylic acid {1-[6-oxo-4-(2-pyridin-3-yl-acetyl)-hexahydro-pyrrolo[3,2-*b*]pyrrole-1-carbonyl]-butyl}-amide;

393. Furan-2-carboxylic acid {1-[6-oxo-4-(2-pyridin-3-yl-acetyl)-hexahydro-pyrrolo[3,2-*b*]pyrrole-1-carbonyl]-butyl}-amide;

394. Thiophene-2-carboxylic acid {1-[6-oxo-4-(2-pyridin-3-yl-acetyl)-hexahydro-pyrrolo[3,2-*b*]pyrrole-1-carbonyl]-butyl}-amide;

395. N-{1-[6-oxo-4-(2-pyridin-3-yl-acetyl)-hexahydro-pyrrolo[3,2-*b*]pyrrole-1-carbonyl]-butyl}-4-trifluoromethyl-benzamide;

396. Furan-3-carboxylic acid {1-[6-oxo-4-(2-pyridin-3-yl-acetyl)-hexahydro-pyrrolo[3,2-*b*]pyrrole-1-carbonyl]-butyl}-amide;

397. Thiophene-3-carboxylic acid {1-[6-oxo-4-(2-pyridin-3-yl-acetyl)-hexahydro-pyrrolo[3,2-*b*]pyrrole-1-carbonyl]-butyl}-amide;

398. N-{1-[6-oxo-4-(2-pyridin-3-yl-acetyl)-hexahydro-pyrrolo[3,2-*b*]pyrrole-1-carbonyl]-butyl}-4-phenoxy-benzamide;

399. N-{1-[6-oxo-4-(2-pyridin-3-yl-acetyl)-hexahydro-pyrrolo[3,2-*b*]pyrrole-1-carbonyl]-butyl}-4-(4-methyl-piperazin-1-yl)-benzamide;

400. 4-Methyl-N-{1-[6-oxo-4-(2-pyridin-3-yl-acetyl)-hexahydro-pyrrolo[3,2-*b*]pyrrole-1-carbonyl]-butyl}-benzamide;

401. 4-Methoxy-N-{1-[6-oxo-4-(2-pyridin-3-yl-acetyl)-hexahydro-pyrrolo[3,2-*b*]pyrrole-1-carbonyl]-butyl}-benzamide;

402. 4-Isopropyl-N- {1-[6-oxo-4-(2-pyridin-3-yl-acetyl)-hexahydro-pyrrolo[3,2-*b*]pyrrole-1-carbonyl]-butyl}-benzamide;

403. N-{1-[6-oxo-4-(2-pyridin-3-yl-acetyl)-hexahydro-pyrrolo[3,2-*b*]pyrrole-1-carbonyl]-butyl}-4-vinyl-benzamide;

404. 4-Imidazol-1-yl-N-{1-[6-oxo-4-(2-pyridin-3-yl-acetyl)-hexahydro-pyrrolo[3,2-*b*]pyrrole-1-carbonyl]-butyl}-benzamide;

405. N-{1-[6-oxo-4-(2-pyridin-3-yl-acetyl)-hexahydro-pyrolo[3,2-*b*]pyrrole-1-carbonyl]-butyl}-4-thiophen-2-yl-benzamide;

406. N-{1-[6-oxo-4-(2-pyridin-3-yl-acetyl)-hexahydro-pyrrolo[3,2-*b*]pyrrole-1-carbonyl]-butyl}-4-oxazol-5-yl-benzamide;

407. 5-Phenyl-thiophene-2-carboxylic acid {1-[6-oxo-4-(2-pyridin-3-yl-acetyl)-hexahydro-pyrrolo[3,2-*b*]pyrrole-1-carbonyl]-butyl}-amide;

408. N-{1-[6-oxo-4-(2-pyridin-3-yl-acetyl)-hexahydro-pyrrolo[3,2-*b*]pyrrole-1-carbonyl]-butyl}-4-trifluoromethoxy-benzamide;

409. 5-Pyridin-2-yl-thiophene-2-carboxylic acid {1-[6-oxo-4-(2-pyridin-3-yl-acetyl)-hexahydro-pyrrolo[3,2-*b*]pyrrole-1-carbonyl]-butyl}-amide;

410. 4-Difluoromethoxy-N-{1-[6-oxo-4-(2-pyridin-3-yl-acetyl)-hexahydro-pyrrolo[3,2-*b*]pyrrole-1-carbonyl]-butyl}-benzamide;

411. N-{1-[6-oxo-4-(2-pyridin-3-yl-acetyl)-hexahydro-pyrrolo[3,2-*b*]pyrrole-1-carbonyl]-butyl}-4-morpholin-4-yl-benzamide;

412. Naphthalene-2-carboxylic acid {3-methyl-1-[6-oxo-4-(pyridin-2-ylmethanesulfonyl)hexahydro-pyrrolo[3,2-*b*]-pyrrole-1-carbonyl]-butyl}-amide;

413. Quinoline-2-carboxylic acid {3-methyl-1-[6-oxo-4-(pyridin-2-ylmethanesulfonyl)-hexahydro-pyrrolo[3,2-*b*]pyrrole-1-carbonyl]-butyl}-amide;

414. Benzo[b]thiophene-2-carboxylic acid {3-methyl-1-[6-oxo-4-(pyridin-2-ylmethanesulfonyl)-hexahydro-pyrrolo[3,2-*b*]pyrrole-1-carbonyl]-butyl}-amide;

415. Benzofuran-2-carboxylic acid {3-methyl-1-[6-oxo-4-(pyridin-2-ylmethanesulfonyl)-hexahydro-pyrrolo[3,2-*b*]-pyrrole-1-carbonyl]-butyl}-amide;

416. Biphenyl-4-carboxylic acid {3-methyl-1-[6-oxo-4-(pyridin-2-ylmethanesulfonyl)-hexahydro-pyrrolo[3,2-*b*]pyrrole-1-carbonyl]-butyl}-amide;

417. 4-*tert*-Butyl-N-{3-methyl-1-[6-oxo-4-(pyridin-2-ylmethanesulfonyl)-hexahydro-pyrrolo[3,2-*b*]pyrrole-1-carbonyl]-butyl}-benzamide;

418. 4-Dimethylamino-N-{3-methyl-1-[6-oxo-4-(pyridin-2-ylmethanesulfonyl)-hexahydro-pyrrolo[3,2-*b*]pyrrole-1-carbonyl]-butyl}-benzamide;

419. 7-Methoxy-benzofuran-2-carboxylic acid {3-methyl-1-[6-oxo-4-(pyridin-2-ylmethanesulfonyl)-hexahydro-pyrrolo[3,2-*b*]pyrrole-1-carbonyl]-butyl}-amide;

420. {3-Methyl-1-[6-oxo-4-(pyridin-2-ylmethanesulfonyl)-hexahydro-pyrrolo[3,2-*b*]pyrrole-1-carbonyl]-butyl}-carbamic acid benzyl ester;

421. 5-Methoxy-benzofuran-2-carboxylic acid {3-methyl-1-[6-oxo-4-(pyridin-2-ylmethanesulfonyl)-hexahydro-pyrrolo[3,2-*b*]pyrrole-1-carbonyl]-butyl}-amide;

422. Thieno[3,2-*b*]thiophene-2-carboxylic acid {3-methyl-1-[6-oxo-4-(pyridin-2-ylmethanesulfonyl)-hexahydro-pyrrolo[3,2-*b*]pyrrole-1-carbonyl]-butyl}-amide;

423. 3-Methyl-benzofuran-2-carboxylic acid {3-methyl-1-[6-oxo-4-(pyridin-2-ylmethanesulfonyl)-hexahydro-pyrrolo[3,2-*b*]pyrrole-1-carbonyl]-butyl}-amide;

424. Quinoxaline-2-carboxylic acid {3-methyl-1-[6-oxo-4-(pyridin-2-ylmethanesulfonyl)-hexahydro-pyrrolo[3,2-*b*]-pyrrole-1-carbonyl]-butyl}-amide;

425. Benzo[1,3]dioxole-5-carboxylic acid {3-methyl-1-[6-oxo-4-(pyridin-2-ylmethanesulfonyl)-hexahydro-pyrrolo[3,2-*b*]pyrrole-1-carbonyl]-butyl}-amide;

426. Quinoline-6-carboxylic acid {3-methyl-1-[6-oxo-4-(pyridin-2-ylmethanesulfonyl)-hexahydro-pyrrolo[3,2-*b*]pyrrole-1-carbonyl]-butyl}-amide;

427. Furan-2-carboxylic acid {3-methyl-1-[6-oxo-4-(pyridin-2-ylmethanesulfonyl)-hexahydro-pyrrolo[3,2-*b*]pyrrole-1-carbonyl]-butyl}-amide;

428. Thiophene-2-carboxylic acid {3-methyl-1-[6-oxo-4-(pyridin-2-ylmethanesulfonyl)-hexahydro-pyrrolo[3,2-*b*]pyrrole-1-carbonyl]-butyl}-amide;

429. N-{3-Methyl-1-[6-oxo-4-(pyridin-2-ylmethanesulfonyl)-hexahydro-pyrrolo[3,2-*b*]pyrrole-1-carbonyl]-butyl}-4-trifluoromethyl-benzamide;

430. Furan-3-carboxylic acid {3-methyl-1-[6-oxo-4-(pyridin-2-ylmethanesulfonyl)-hexahydro-pyrrolo[3,2-*b*]pyrrole-1-carbonyl]-butyl}-amide;

431. Thiophene-3-carboxylic acid {3-methyl-1-[6-oxo-4-(pyridin-2-ylmethanesulfonyl)-hexahydro-pyrrolo[3,2-*b*]pyrrole-1-carbonyl]-butyl}-amide;

432. N-{3-Methyl-1-[6-oxo-4-(pyridin-2-ylmethanesulfonyl)-hexahydro-pyrrolo[3,2-*b*]pyrrole-1-carbonyl]-butyl}-4-phenoxy-benzamide;

433. N-{3-Methyl-1-[6-oxo-4-(pyridin-2-ylmethanesulfonyl)-hexahydro-pyrrolo[3,2-*b*]pyrrole-1-carbonyl]-butyl}-4-(4-methyl-piperazin-1-yl)-benzamide;

434. 4-Methyl-N-{3-methyl-1-[6-oxo-4-(pyridin-2-ylmethanesulfonyl)-hexahydro-pyrrolo[3,2-*b*]pyrrole-1-carbonyl]-butyl}-benzamide;

435. 4-Methoxy-N-{3-methyl-1-[6-oxo-4-(pyridin-2-ylmethanesulfonyl)-hexahydro-pyrrolo[3,2-*b*]pyrrole-1-carbonyl]-butyl}-benzamide;

436. 4-Isopropyl-N-{3-methyl-1-[6-oxo-4-(pyridin-2-ylmethanesulfonyl)-hexahydro-pyrrolo[3,2-*b*]pyrrole-1-carbonyl]-butyl}-benzamide;

437. N-{3-Methyl-1-[6-oxo-4-(pyridin-2-ylmethanesulfonyl)-hexahydro-pyrrolo[3,2-*b*]pyrrole-1-carbonyl]-butyl}-4-vinyl-benzamide;

438. 4-Imidazol-1-yl-N-{3-methyl-1-[6-oxo-4-(pyridin-2-ylmethanesulfonyl)-hexahydro-pyrrolo[3,2-*b*]pyrrole-1-carbonyl]-butyl}-benzamide;

439. N-{3-Methyl-1-[6-oxo-4-(pyridin-2-ylmethanesulfonyl)-hexahydro-pyrrolo[3,2-*b*]pyrrole-1-carbonyl]-butyl}-4-thiophen-2-yl-benzamide;

440. N-{3-Methyl-1-[6-oxo-4-(pyridin-2-ylmethanesulfonyl)-hexahydro-pyrrolo[3,2-*b*]pyrrole-1-carbonyl]-butyl}-4-oxazol-5-yl-benzamide;

441. 5-Phenyl-thiophene-2-carboxylic acid {3-methyl-1-[6-oxo-4-(pyridin-2-ylmethanesulfonyl)-hexahydro-pyrrolo[3,2-*b*]pyrrole-1-carbonyl]-butyl}-amide;

442. N-{3-Methyl-1-[6-oxo-4-(pyridin-2-ylmethanesulfonyl)-hexahydro-pyrrolo[3,2-*b*]pyrrole-1-carbonyl]-butyl}-4-trifluoromethoxy-benzamide;

443. 5-Pyridin-2-yl-thiophene-2-carboxylic acid {3-methyl-1-[6-oxo-4-(pyridin-2-ylmethanesulfonyl)-hexahydro-pyrrolo[3,2-*b*]pyrrole-1-carbonyl]-butyl}-amide;

444. 4-Difluoromethoxy-N-{3-methyl-1-[6-oxo-4-(pyridin-2-ylmethanesulfonyl)-hexahydro-pyrrolo[3,2-*b*]pyrrole-1-carbonyl]-butyl}-benzamide;

445. N-{3-Methyl-1-[6-oxo-4-(pyridin-2-ylmethanesulfonyl)-hexahydro-pyrrolo[3,2-*b*]pyrrole-1-carbonyl]-butyl}-4-morpholin-4-yl-benzamide;

446. Naphthalene-2-carboxylic acid {1-[6-oxo-4-(pyridin-2-ylmethanesulfonyl)hexahydro-pyrrolo[3,2-*b*]pyrrole-1-carbonyl]-butyl}-amide;

447. Quinoline-2-carboxylic acid {1-[6-oxo-4-(pyridin-2-ylmethanesulfonyl)-hexahydro-pyrrolo[3,2-*b*]pyrrole-1-carbonyl]-butyl}-amide;

448. Benzo[b]thiophene-2-carboxylic acid {1-[6-oxo-4-(pyridin-2-ylmethanesulfonyl)-hexahydro-pyrrolo[3,2-*b*]pyrrole-1-carbonyl]-butyl}-amide;

449. Benzofuran-2-carboxylic acid {1-[6-oxo-4-(pyridin-2-ylmethanesulfonyl)-hexahydro-pyrrolo[3,2-*b*]pyrrole-1-carbonyl]-butyl}-amide;

450. Biphenyl-4-carboxylic acid {1-[6-oxo-4-(pyridin-2-ylmethanesulfonyl)-hexahydro-pyrrolo[3,2-*b*]pyrrole-1-carbonyl]-butyl}-amide;

451. 4-*tert*-Butyl-N-{1-[6-oxo-4-(pyridin-2-ylmethanesulfonyl)-hexahydro-pyrrolo[3,2-*b*]pyrrole-1-carbonyl]-butyl}-benzamide;

452. 4-Dimethylamino-N-{1-[6-oxo-4-(pyridin-2-ylmethanesulfonyl)-hexahydro-pyrrolo[3,2-*b*]pyrrole-1-carbonyl]-butyl}-benzamide;

453. 7-Methoxy-benzofuran-2-carboxylic acid {1-[6-oxo-4-(pyridin-2-ylmethanesulfonyl)-hexahydro-pyrrolo[3,2-*b*]-pyrrole-1-carbonyl]-butyl}-amide;

454. {1-[6-oxo-4-(pyridin-2-ylmethanesulfonyl)-hexahydro-pyrrolo[3,2-*b*]pyrrole-1-carbonyl]-butyl}-carbamic acid benzyl ester;

455. 5-Methoxy-benzofuran-2-carboxylic acid {1-[6-oxo-4-(pyridin-2-ylmethanesulfonyl)-hexahydro-pyrrolo[3,2-*b*]-pyrrole-1-carbonyl]-butyl}-amide;

456. Thieno[3,2-*b*]thiophene-2-carboxylic acid {1-[6-oxo-4-(pyridin-2-ylmethanesulfonyl)-hexahydro-pyrrolo[3,2-*b*]-pyrrole-1-carbonyl]-butyl}-amide;

457. 3-Methyl-benzofuran-2-carboxylic acid {1-[6-oxo-4-(pyridin-2-ylmethanesulfonyl)-hexahydro-pyrrolo[3,2-*b*]pyrrole-1-carbonyl]-butyl}-amide;

458. Quinoxaline-2-carboxylic acid {1-[6-oxo-4-(pyridin-2-ylmethanesulfonyl)-hexahydro-pyrrolo[3,2-*b*]pyrrole-1-carbonyl]-butyl}-amide;

459. Benzo[1,3]dioxole-5-carboxylic acid {1-[6-oxo-4-(pyridin-2-ylmethanesulfonyl)-hexahydro-pyrrolo[3,2-*b*]pyrrole-1-carbonyl]-butyl}-amide;

460. Quinoline-6-carboxylic acid {1-[6-oxo-4-(pyridin-2-ylmethanesulfonyl)-hexahydro-pyrrolo[3,2-*b*]pyrrole-1-carbonyl]-butyl}-amide;

461. Furan-2-carboxylic acid {1-[6-oxo-4-(pyridin-2-ylmethanesulfonyl)-hexahydro-pyrrolo[3,2-*b*]pyrrole-1-carbonyl]-butyl}-amide;

462. Thiophene-2-carboxylic acid {1-[6-oxo-4-(pyridin-2-ylmethanesulfonyl)-hexahydro-pyrrolo[3,2-*b*]pyrrole-1-carbonyl]-butyl}-amide;

463. N-{1-[6-oxo-4-(pyridin-2-ylmethanesulfonyl)-hexahydro-pyrrolo[3,2-*b*]pyrrole-1-carbonyl]-butyl}-4-trifluoromethyl-benzamide;

464. Furan-3-carboxylic acid {1-[6-oxo-4-(pyridin-2-ylmethanesulfonyl)-hexahydro-pyrrolo[3,2-*b*]pyrrole-1-carbonyl]-butyl}-amide;

465. Thiophene-3-carboxylic acid {1-[6-oxo-4-(pyridin-2-ylmethanesulfonyl)-hexahydro-pyrrolo[3,2-*b*]pyrrole-1-carbonyl]-butyl}-amide;

466. N-{1-[6-oxo-4-(pyridin-2-ylmethanesulfonyl)-hexahydro-pyrrolo[3,2-*b*]pyrrole-1-carbonyl]-butyl}-4-phenoxy-benzamide;

467. N-{1-[6-oxo-4-(pyridin-2-ylmethanesulfonyl)-hexahydro-pyrrolo[3,2-*b*]pyrrole-1-carbonyl]-butyl}-4-(4-methyl-piperazin-1-yl)-benzamide;

468. 4-Methyl-N-{1-[6-oxo-4-(pyridin-2-ylmethanesulfonyl)-hexahydro-pyrrolo[3,2-*b*]pyrrole-1-carbonyl]-butyl}-benzamide;

469. 4-Methoxy-N-{1-[6-oxo-4-(pyridin-2-ylmethanesulfonyl)-hexahydro-pyrrolo[3,2-*b*]pyrrole-1-carbonyl]-butyl}-benzamide;

470. 4-Isopropyl-N-{1-[6-oxo-4-(pyridin-2-ylmethanesulfonyl)-hexahydro-pyrrolo[3,2-*b*]pyrrole-1-carbonyl]-butyl}-benzamide;

471. N-{1-[6-oxo-4-(pyridin-2-ylmethanesulfonyl)-hexahydro-pyrrolo[3,2-*b*]pyrrole-1-carbonyl]-butyl}-4-vinyl-benzamide;

472. 4-Imidazol-1-yl-N-{1-[6-oxo-4-(pyridin-2-ylmethanesulfonyl)-hexahydro-pyrrolo[3,2-*b*]pyrrole-1-carbonyl]-butyl}-benzamide;

473. N-{1-[6-oxo-4-(pyridin-2-ylmethanesulfonyl)-hexahydro-pyrrolo[3,2-*b*]pyrrole-1-carbonyl]-butyl}-4-thiophen-2-yl-benzamide;

474. N-{1-[6-oxo-4-(pyridin-2-ylmethanesulfonyl)-hexahydro-pyrrolo[3,2-*b*]pyrrole-1-carbonyl]-butyl}-4-oxazol-5-yl-benzamide;

475. 5-Phenyl-thiophene-2-carboxylic acid {1-[6-oxo-4-(pyridin-2-ylmethanesulfonyl)-hexahydro-pyrrolo[3,2-*b*]pyrrole-1-carbonyl]-butyl}-amide;

476. N-{1-[6-oxo-4-(pyridin-2-ylmethanesulfonyl)-hexahydro-pyrrolo[3,2-*b*]pyrrole-1-carbonyl]-butyl}-4-trifluoromethoxy-benzamide;

477. 5-Pyridin-2-yl-thiophene-2-carboxylic acid {1-[6-oxo-4-(pyridin-2-ylmethanesulfonyl)-hexahydro-pyrrolo[3,2-*b*]-pyrrole-1-carbonyl]-butyl}-amide;

478. 4-Difluoromethoxy-N-{1-[6-oxo-4-(pyridin-2-ylmethanesulfonyl)-hexahydro-pyrrolo[3,2-*b*]pyrrole-1-carbonyl]-butyl}-benzamide;

479. N-{1-[6-oxo-4-(pyridin-2-ylmethanesulfonyl)-hexahydro-pyrrolo[3,2-*b*]pyrrole-1-carbonyl]-butyl}-4-morpholin-4-yl-benzamide;

480. Naphthalene-2-carboxylic acid {3-methyl-1-[6-oxo-4-(pyridin-3-ylmethanesulfonyl)hexahydro-pyrrolo[3,2-*b*]pyrrole-1-carbonyl]-butyl}-amide;

481. Quinoline-2-carboxylic acid {3-methyl-1-[6-oxo-4-(pyridin-3-ylmethanesulfonyl)-hexahydro-pyrrolo[3,2-*b*]pyrrole-1-carbonyl]-butyl}-amide;

482. Benzo[b]thiophene-2-carboxylic acid {3-methyl-1-[6-oxo-4-(pyridin-3-ylmethanesulfonyl)-hexahydro-pyrrolo[3,2-*b*]pyrrole-1-carbonyl]-butyl}-amide;

483. Benzofuran-2-carboxylic acid {3-methyl-1-[6-oxo-4-(pyridin-3-ylmethanesulfonyl)-hexahydro-pyrrolo[3,2-*b*]-pyrrole-1-carbonyl]-butyl}-amide;

484. Biphenyl-4-carboxylic acid {3-methyl-1-[6-oxo-4-(pyridin-3-ylmethanesulfonyl)-hexahydro-pyrrolo[3,2-*b*]pyrrole-1-carbonyl]-butyl}-amide;

485. 4-*tert*-Butyl-N-{3-methyl-1-[6-oxo-4-(pyridin-3-ylmethanesulfonyl)-hexahydro-pyrrolo[3,2- *b*]pyrrole-1-carbonyl]-butyl}-benzamide;

486. 4-Dimethylamino-N- {3-methyl-1-[6-oxo-4-(pyridin-3-ylmethanesulfonyl)-hexahydro-pyrrolo[3,2-*b*]pyrrole-1-carbonyl]-butyl}-benzamide;

487. 7-Methoxy-benzofuran-2-carboxylic acid {3-methyl-1-[6-oxo-4-(pyridin-3-ylmethanesulfonyl)-hexahydro-pyrrolo[3,2-*b*]pyrrole-1-carbonyl]-butyl}-amide;

488. {3-Methyl-1-[6-oxo-4-(pyridin-3-ylmethanesulfonyl)-hexahydro-pyrrolo[3,2-*b*]pyrrole-1-carbonyl]-butyl}-carbamic acid benzyl ester;

489. 5-Methoxy-benzofuran-2-carboxylic acid {3-methyl-1-[6-oxo-4-(pyridin-3-ylmethanesulfonyl)-hexahydro-pyrrolo[3,2-*b*]pyrrole-1-carbonyl]-butyl}-amide;

490. Thieno[3,2-*b*]thiophene-2-carboxylic acid {3-methyl-1-[6-oxo-4-(pyridin-3-ylmethanesulfonyl)-hexahydro-pyrrolo[3,2-*b*]pyrrole-1-carbonyl]-butyl}-amide;

491. 3-Methyl-benzofuran-2-carboxylic acid {3-methyl-1-[6-oxo-4-(pyridin-3-ylmethanesulfonyl)-hexahydro-pyrrolo[3,2-*b*]pyrrole-1-carbonyl]-butyl}-amide;

492. Quinoxaline-2-carboxylic acid {3-methyl-1-[6-oxo-4-(pyridin-3-ylmethanesulfonyl)-hexahydro-pyrrolo[3,2-*b*]-pyrrole-1-carbonyl]-butyl}-amide;

493. Benzo[1,3]dioxole-5-carboxylic acid {3-methyl-1-[6-oxo-4-(pyridin-3-ylmethanesulfonyl)-hexahydro-pyrrolo[3,2-*b*]pyrrole-1-carbonyl]-butyl}-amide;

494. Quinoline-6-carboxylic acid {3-methyl-1-[6-oxo-4-(pyridin-3-ylmethanesulfonyl)-hexahydro-pyrrolo[3,2-*b*]pyrrole-1-carbonyl]-butyl}-amide;

495. Furan-2-carboxylic acid {3-methyl-1-[6-oxo-4-(pyridin-3-ylmethanesulfonyl)-hexahydro-pyrrolo[3,2-*b*]pyrrole-1-carbonyl]-butyl}-amide;

496. Thiophene-2-carboxylic acid {3-methyl-1-[6-oxo-4-(pyridin-3-ylmethanesulfonyl)-hexahydro-pyrrolo[3,2-*b*]pyrrole-1-carbonyl]-butyl}-amide;

497. N-{3-Methyl-1-[6-oxo-4-(pyridin-3-ylmethanesulfonyl)-hexahydro-pyrrolo[3,2-*b*]pyrrole-1-carbonyl]-butyl}-4-trifluoromethyl-benzamide;

498. Furan-3-carboxylic acid {3-methyl-1-[6-oxo-4-(pyridin-3-ylmethanesulfonyl)-hexahydro-pyrrolo[3,2-*b*]pyrrole-1-carbonyl]-butyl}-amide;

499. Thiophene-3-carboxylic acid {3-methyl-1-[6-oxo-4-(pyridin-3-ylmethanesulfonyl)-hexahydro-pyrrolo[3,2-*b*]pyrrole-1-carbonyl]-butyl}-amide;

500. N-{3-Methyl-1-[6-oxo-4-(pyridin-3-ylmethanesulfonyl)-hexahydro-pyrrolo[3,2-*b*]pyrrole-1-carbonyl]-butyl}-4-phenoxy-benzamide;

501. N-{3-Methyl-1-[6-oxo-4-(pyridin-3-ylmethanesulfonyl)-hexahydro-pyrrolo[3,2-*b*]pyrrole-1-carbonyl]-butyl}-4-(4-methyl-piperazin-1-yl)-benzamide;

502. 4-Methyl-N-{3-methyl-1-[6-oxo-4-(pyridin-3-ylmethanesulfonyl)-hexahydro-pyrrolo[3,2-*b*]pyrrole-1-carbonyl]-butyl}-benzamide;

503. 4-Methoxy-N-{3-methyl-1-[6-oxo-4-(pyridin-3-ylmethanesulfonyl)-hexahydro-pyrrolo[3,2-*b*]pyrrole-1-carbonyl]-butyl}-benzamide;

504. 4-Isopropyl-N-{3-methyl-1-[6-oxo-4-(pyridin-3-ylmethanesulfonyl)-hexahydro-pyrrolo[3,2-*b*]pyrrole-1-carbonyl]-butyl}-benzamide;

505. N-{3-Methyl-1-[6-oxo-4-(pyridin-3-ylmethanesulfonyl)-hexahydro-pyrrolo[3,2-*b*]pyrrole-1-carbonyl]-butyl}-4-vinyl-benzamide;

506. 4-Imidazol-1-yl-N-{3-methyl-1-[6-oxo-4-(pyridin-3-ylmethanesulfonyl)-hexahydro-pyrrolo[3,2-*b*]pyrrole-1-carbonyl]-butyl}-benzamide;

507. N-{3-Methyl-1-[6-oxo-4-pyridin-3-ylmethanesulfonyl)-hexahydro-pyrrolo[3,2-*b*]pyrrole-1-carbonyl]-butyl}-4-thiophen-2-yl-benzamide;

508. N-{3-Methyl-1-[6-oxo-4-(pyridin-3-ylmethanesulfonyl)-hexahydro-pyrrolo[3,2-*b*]pyrrole-1-carbonyl]-butyl}-4-oxazol-5-yl-benzamide;

509. 5-Phenyl-thiophene-2-carboxylic acid {3-methyl-1-[6-oxo-4-(pyridin-3-ylmethanesulfonyl)-hexahydro-pyrrolo[3,2-*b*]pyrrole-1-carbonyl]-butyl}-amide;

510. N-{3-Methyl-1-[6-oxo-4-(pyridin-3-ylmethanesulfonyl)-hexahydro-pyrrolo[3,2-*b*]pyrrole-1-carbonyl]-butyl}-4-trifluoromethoxy-benzamide;

511. 5-Pyridin-2-yl-thiophene-2-carboxylic acid {3-methyl-1-[6-oxo-4-(pyridin-3-ylmethanesulfonyl)-hexahydro-pyrrolo[3,2-*b*]pyrrole-1-carbonyl]-butyl}-amide;

512. 4-Difluoromethoxy-N-{3-methyl-1-[6-oxo-4-(pyridin-3-ylmethanesulfonyl)-hexahydro-pyrrolo[3,2-*b*]pyrrole-1-carbonyl]-butyl}-benzamide;

513. N-{3-Methyl-1-[6-oxo-4-(pyridin-3-ylmethanesulfonyl)-hexahydro-pyrrolo[3,2-*b*]pyrrole-1-carbonyl]-butyl}-4-morpholin-4-yl-benzamide;

514. Naphthalene-2-carboxylic acid {1-[6-oxo-4-(pyridin-3-ylmethanesulfonyl)hexahydro-pyrrolo[3,2-*b*]pyrrole-1-carbonyl]-butyl}-amide;

515. Quinoline-2-carboxylic acid {1-[6-oxo-4-(pyridin-3-ylmethanesulfonyl)-hexahydro-pyrrolo[3,2-*b*]pyrrole-1-carbonyl]-butyl}-amide;

516. Benzo[b]thiophene-2-carboxylic acid {1-[6-oxo-4-(pyridin-3-ylmethanesulfonyl)-hexahydro-pyrrolo[3,2-*b*]pyrrole-1-carbonyl]-butyl}-amide;

517. Benzofuran-2-carboxylic acid {1-[6-oxo-4-(pyridin-3-ylmethanesulfonyl)-hexahydro-pyrrolo[3,2-*b*]pyrrole-1-carbonyl]-butyl}-amide;

518. Biphenyl-4-carboxylic acid {1-[6-oxo-4-(pyridin-3-ylmethanesulfonyl)-hexahydro-pyrrolo[3,2-*b*]pyrrole-1-carbonyl]-butyl}-amide;

519. 4-*tert*-Butyl-N-{1-[6-oxo-4-(pyridin-3-ylmethanesulfonyl)-hexahydro-pyrrolo[3,2-*b*]pyrrole-1-carbonyl]-butyl}-benzamide;

520. 4-Dimethylamino-N- {1-[6-oxo-4-(pyridin-3-ylmethanesulfonyl)-hexahydro-pyrrolo[3,2-*b*]pyrrole-1-carbonyl]-butyl}-benzamide;

521. 7-Methoxy-benzofuran-2-carboxylic acid {1-[6-oxo-4-(pyridin-3-ylmethanesulfonyl)-hexahydro-pyrrolo[3,2-*b*]-pyrrole-1-carbonyl]-butyl}-amide;

522. {1-[6-oxo-4-(pyridin-3-ylmethanesulfonyl)-hexahydro-pyrrolo[3,2-*b*]pyrrole-1-carbonyl]-butyl}-carbamic acid benzyl ester;

523. 5-Methoxy-benzofuran-2-carboxylic acid {1-[6-oxo-4-(pyridin-3-ylmethanesulfonyl)-hexahydro-pyrrolo[3,2-*b*]-pyrrole-1-carbonyl]-butyl}-amide;

524. Thieno[3,2-*b*]thiophene-2-carboxylic acid {1-[6-oxo-4-(pyridin-3-ylmethanesulfonyl)-hexahydro-pyrrolo[3,2-*b*]-pyrrole-1-carbonyl]-butyl}-amide;

525. 3-Methyl-benzofuran-2-carboxylic acid {1-[6-oxo-4-(pyridin-3-ylmethanesulfonyl)-hexahydro-pyrrolo[3,2- *b*]pyrrole-1-carbonyl]-butyl}-amide;

526. Quinoxaline-2-carboxylic acid {1-[6-oxo-4-(pyridin-3-ylmethanesulfonyl)-hexahydro-pyrrolo[3,2- *b*]pyrrole-1-carbonyl]-butyl}-amide;

527. Benzo[1,3]dioxole-5-carboxylic acid {1-[6-oxo-4-(pyridin-3-ylmethanesulfonyl)-hexahydro-pyrrolo[3,2-*b*]pyrrole-1-carbonyl]-butyl}-amide;

528. Quinoline-6-carboxylic acid {1-[6-oxo-4-(pyridin-3-ylinethanesulfonyl)-hexahydro-pyrrolo[3,2-*b*]pyrrole-1-carbonyl]-butyl}-amide;

529. Furan-2-carboxylic acid {1-[6-oxo-4-(pyridin-3-ylmethanesulfonyl)-hexahydro-pyrrolo[3,2-*b*]pyrrole-1-carbonyl]-butyl}-amide;

530. Thiophene-2-carboxylic acid {1-[6-oxo-4-(pyridin-3-ylmethanesulfonyl)-hexahydro-pyrrolo[3,2-*b*]pyrrole-1-carbonyl]-butyl}-amide;

531. N-{1-[6-oxo-4-(pyridin-3-ylmethanesulfonyl)-hexahydro-pyrrolo[3,2-*b*]pyrrole-1-carbonyl]-butyl}-4-trifluoromethyl-benzamide;

532. Furan-3-carboxylic acid {1-[6-oxo-4-(pyridin-3-ylmethanesulfonyl)-hexahydro-pyrrolo[3,2- *b*]pyrrole-1-carbonyl]-butyl}-amide;

533. Thiophene-3-carboxylic acid {1-[6-oxo-4-(pyridin-3-yrimethanesulfonyl)-hexahydro-pyrrolo[3,2-*b*]pyrrole-1-carbonyl]-butyl}-amide;

534. N-{1-[6-oxo-4-(pyridin-3-ylmethanesulfonyl)-hexahydro-pyrrolo[3,2-*b*]pyrrole-1-carbonyl]-butyl}-4-phenoxy-benzamide;

535. N-{1-[6-oxo-4-(pyridin-3-ylmethanesulfonyl)-hexahydro-pyrrolo[3,2-*b*]pyrrole-1-carbonyl]-butyl}-4-(4-methyl-piperazin-1-yl)-benzamide;

536. 4-Methyl-N-{1-[6-oxo-4-(pyridin-3-ylmethanesulfonyl)-hexahydro-pyrrolo[3,2-*b*]pyrrole-1-carbonyl]-butyl}-benzamide;

537. 4-Methoxy-N-{1-[6-oxo-4-(pyridin-3-ylmethanesulfonyl)-hexahydro-pyrrolo[3,2-*b*]pyrrole-1-carbonyl]-butyl}-benzamide;

538. 4-Isopropyl-N- {1-[6-oxo-4-(pyridin-3-ylmethanesulfonyl)-hexahydro-pyrrolo[3,2-*b*]pyrrole-1-carbonyl]-butyl}-benzamide;

539. N-{1-[6-oxo-4-(pyridin-3-ylmethanesulfonyl)-hexahydro-pyrrolo[3,2-*b*]pyrrole-1-carbonyl]-butyl}-4-vinyl-benzamide;

540. 4-Imidazol-1-yl-N-{1-[6-oxo-4-(pyridin-3-ylmethanesulfonyl)-hexahydro-pyrrolo[3,2-*b*]pyrrole-1-carbonyl]-butyl}-benzamide;

541. N-{1-[6-oxo-4-(pyridin-3-ylmethanesulfonyl)-hexahydro-pyrrolo[3,2-*b*]pyrrole-1-carbonyl]-butyl}-4-thiophen-2-yl-benzamide;

542. N-{1-[6-oxo-4-(pyridin-3-ylmethanesulfonyl)-hexahydro-pyrrolo[3,2-*b*]prrole-1-carbonyl]-butyl}-4-oxazol-5-yl-benzamide;

543. 5-Phenyl-thiophene-2-carboxylic acid {1-[6-oxo-4-(pyridin-3-ylmethanesulfonyl)-hexahydro-pyrrolo[3,2-*b*]pyrrole-1-carbonyl]-butyl}-amide;

544. N-{1-[6-oxo-4-(pyridin-3-ylmethanesulfonyl)-hexahydro-pyrrolo[3,2-*b*]pyrrole-1-carbonyl]-butyl}-4-trifluoromethoxy-benzamide;

545. 5-Pyridin-2-yl-thiophene-2-carboxylic acid {1-[6-oxo-4-(pyridin-3-ylmethanesulfonyl)-hexahydro-pyrrolo[3,2-*b*]pyrrole-1-carbonyl]-butyl}-amide;

546. 4-Difluoromethoxy-N- {1-[6-oxo-4-(pyridin-3-ylmethanesulfonyl)-hexahydro-pyrrolo[3,2-*b*]pyrrole-1-carbonyl]-butyl}-benzamide;

547. N-{1-[6-oxo-4-(pyridin-3-ylmethanesulfonyl)-hexahydro-pyrrolo[3,2-*b*]pyrrole-1-carbonyl]-butyl}-4-morpholin-4-yl-benzamide;

548. Naphthalene-2-carboxylic acid {3-methyl-1-[6-oxo-4-(1-oxy-pyridin-2-ylmethanesulfonyl)hexahydro-pyrrolo[3,2-*b*]pyrrole-1-carbonyl]-butyl}-amide;

549. Quinoline-2-carboxylic acid {3-methyl-1-[6-oxo-4-(1-oxy-pyridin-2-ylmethanesulfonyl)-hexahydro-pyrrolo[3,2-*b*]pyrrole-1-carbonyl]-butyl}-amide;

550. Benzo[b]thiophene-2-carboxylic acid {3-methyl-1-[6-oxo-4-(1-oxy-pyridin-2-ylmethanesulfonyl)-hexahydro-pyrrolo[3,2-*b*]pyrrole-1-carbonyl]-butyl}-amide;

551. Benzofuran-2-carboxylic acid {3-methyl-1-[6-oxo-4-(1-oxy-pyridin-2-ylmethanesulfonyl)-hexahydro-pyrrolo[3,2-*b*]pyrrole-1-carbonyl]-butyl}-amide;

552. Biphenyl-4-carboxylic acid {3-methyl-1-[6-oxo-4-(1-oxy-pyridin-2-ylmethanesulfonyl)-hexahydro-pyrrolo[3,2-*b*]pyrrole-1-carbonyl]-butyl}-amide;

553. 4-*tert*-Butyl-N-{3-methyl-1-[6-oxo-4-(1-oxy-pyridin-2-ylmethanesulfonyl)-hexahydro-pyrrolo[3,2- *b*]pyrrole-1-carbonyl]-butyl}-benzamide;

554. 4-Dimethylamino-N- {3-methyl-1-[6-oxo-4-(1-oxy-pyridin-2-ylmethanesulfonyl)-hexahydro-pyrrolo[3,2-*b*]pyrrole-1-carbonyl]-butyl}-benzamide;

555. 7-Methoxy-benzofuran-2-carboxylic acid {3-methyl-1-[6-oxo-4-(1-oxy-pyridin-2-ylmethanesulfonyl)-hexahydro-pyrrolo[3,2-*b*]pyrrole-1-carbonyl]-butyl}-amide;

556. {3-Methyl-1-[6-oxo-4-(1-oxy-pyridin-2-ylmethanesulfonyl)-hexahydro-pyrrolo[3,2-*b*]pyrrole-1-carbonyl]-butyl}-carbamic acid benzyl ester;

557. 5-Methoxy-benzofuran-2-carboxylic acid {3-methyl-1-[6-oxo-4-(1-oxy-pyridin-2-ylmethanesulfonyl)-hexahydro-pyrrolo[3,2-*b*]pyrrole-1-carbonyl]-butyl}-amide;

558. Thieno[3,2-*b*]thiophene-2-carboxylic acid {3-methyl-1-[6-oxo-4-(1-oxy-pyridin-2-ylmethanesulfonyl)-hexahydro-pyrrolo[3,2-*b*]pyrrole-1-carbonyl]-butyl}-amide;

559. 3-Methyl-benzofuran-2-carboxylic acid {3-methyl-1-[6-oxo-4-(1-oxy-pyridin-2-ylmethanesulfonyl)-hexahydro-pyrrolo[3,2-*b*]pyrrole-1-carbonyl]-butyl}-amide;

560. Quinoxaline-2-carboxylic acid {3-methyl-1-[6-oxo-4-(1-oxy-pyridin-2-ylmethanesulfonyl)-hexahydro-pyrrolo[3, 2-*b*]pyrrole-1-carbonyl]-butyl}-amide;

561. Benzo[1,3]dioxole-5-carboxylic acid {3-methyl-1-[6-oxo-4-(1-oxy-pyridin-2-ylmethanesulfonyl)-hexahydro-pyrrolo[3,2-*b*]pyrrole-1-carbonyl]-butyl}-amide;

562. Quinoline-6-carboxylic acid {3-methyl-1-[6-oxo-4-(1-oxy-pyridin-2-ylmethanesulfonyl)-hexahydro-pyrrolo[3,2-*b*]pyrrole-1-carbonyl]-butyl}-amide;

563. Furan-2-carboxylic acid {3-methyl-1-[6-oxo-4-(1-oxy-pyridin-2-ylmethanesulfonyl)-hexahydro-pyrrolo[3,2-*b*]-pyrrole-1-carbonyl]-butyl}-amide;

564. Thiophene-2-carboxylic acid {3-methyl-1-[6-oxo-4-(1-oxy-pyridin-2-ylmethanesulfonyl)-hexahydro-pyrrolo[3,2-*b*]pyrrole-1-carbonyl]-butyl}-amide;

565. N-{3-Methyl-1-[6-oxo-4-(1-oxy-pyridin-2-ylmethanesulfonyl)-hexahydro-pyrrolo[3,2-*b*]pyrrole-1-carbonyl]-butyl}-4-trifluoromethyl-benzamide;

566. Furan-3-carboxylic acid {3-methyl-1-[6-oxo-4-(1-oxy-pyridin-2-ylmethanesulfonyl)-hexahydro-pyrrolo[3,2-*b*]pyrrole-1-carbonyl]-butyl}-amide;

567. Thiophene-3-carboxylic acid {3-methyl-1-[6-oxo-4-(1-oxy-pyridin-2-ylmethanesulfonyl)-hexahydro-pyrrolo[3,2-*b*]pyrrole-1-carbonyl]-butyl}-amide;

568. N-{3-Methyl-1-[6-oxo-4-(1-oxy-pyridin-2-ylmethanesulfonyl)-hexahydro-pyrrolo[3,2-*b*]pyrrole-1-carbonyl]-butyl}-4-phenoxy-benzamide;

569. N-{3-Methyl-1-[6-oxo-4-(1-oxy-pyridin-2-ylmethanesulfonyl)-hexahydro-pyrrolo[3,2-*b*]pyrrole-1-carbonyl]-butyl}-4-(4-methyl-piperazin-1-yl)-benzamide;

570. 4-Methyl-N- {3-methyl-1-[6-oxo-4-(1-oxy-pyridin-2-ylmethanesulfonyl)-hexahydro-pyrrolo[3,2-*b*]pyrrole-1-carbonyl]-butyl}-benzamide;

571. 4-Methoxy-N-{3-methyl-1-[6-oxo-4-(1-oxy-pyridin-2-ylmethanesulfonyl)-hexahydro-pyrrolo[3,2-*b*]pyrrole-1-carbonyl]-butyl}-benzamide;

572. 4-Isopropyl-N- {3-methyl-1-[6-oxo-4-(1-oxy-pyridin-2-ylinethanesulfonyl)-hexahydro-pyrrolo[3,2-*b*]pyrrole-1-carbonyl]-butyl}-benzamide;

573. N- {3-Methyl-1-[6-oxo-4-(1-oxy-pyridin-2-ylmethanesulfonyl)-hexahydro-pyrrolo[3,2-*b*]pyrrole-1-carbonyl]-butyl}-4-vinyl-benzamide;

574. 4-Imidazol-1-yl-N-{3-methyl-1-[6-oxo-4-(1-oxy-pyridin-2-ylmethanesulfonyl)-hexahydro-pyrrolo[3,2-*b*]pyrrole-1-carbonyl]-butyl}-benzamide;

575. N-{3-Methyl-1-[6-oxo-4-(1-oxy-pyridin-2-ylmethanesulfonyl)-hexahydro-pyrrolo[3,2-*b*]pyrrole-1-carbonyl]-butyl}-4-thiophen-2-yl-benzamide;

576. N-{3-Methyl-1-[6-oxo-4-(1-oxy-pyridin-2-ylmethanesulfonyl)-hexahydro-pyrrolo[3,2-*b*]pyrrole-1-carbonyl]-butyl}-4-oxazol-5-yl-benzamide;

577. 5-Phenyl-thiophene-2-carboxylic acid {3-methyl-1-[6-oxo-4-(1-oxy-pyridin-2-ylmethanesulfonyl)-hexahydro-pyrrolo[3,2-*b*]pyrrole-1-carbonyl]-butyl}-amide;

578. N-{3-Methyl-1-[6-oxo-4-(1-oxy-pyridin-2-ylmethanesulfonyl)-hexallydro-pyrrolo[3,2-*b*]pyrrole-1-carbonyl]-butyl}-4-trifluoromethoxy-benzamide;

579. 5-Pyridin-2-yl-thiophene-2-carboxylic acid {3-methyl-1-[6-oxo-4-(1-oxy-pyridin-2-ylmethanesulfonyl)-hexahydro-pyrrolo[3,2-*b*]pyrrole-1-carbonyl]-butyl}-amide;

580. 4-Difluoromethoxy-N- {3-methyl-1-[6-oxo-4-(1-oxy-pyridin-2-ylmethanesulfonyl)-hexahydro-pyrrolo[3,2-*b*]pyrrole-1-carbonyl]-butyl}-benzamide;

581. N-{3-Methyl-1-[6-oxo-4-(1-oxy-pyridin-2-ylmethanesulfonyl)-hexahydro-pyrrolo[3,2-*b*]pyrrole-1-carbonyl]-butyl}-4-morpholin-4-yl-benzamide;

582. Naphthalene-2-carboxylic acid {1-[6-oxo-4-(1-oxy-pyridin-2-ylmethanesulfonyl)hexahydro-pyrrolo[3,2-*b*]pyrrole-1-carbonyl]-butyl}-amide;

583. Quinoline-2-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

584. Benzo[b]thiophene-2-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

585. Benzofuran-2-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

586. Biphenyl-4-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

587. 4-*tert*-Butyl-N-{1-[6-oxo-4-(1-oxy-pyridin-2-ylmethanesulfonyl)-hexahydro-pyrrolo[3,2-*b*]pyrrole-1-carbonyl]-butyl}-benzamide;

588. 4-Dimethylamino-N-{1-[6-oxo-4-(1-oxy-pyridin-2-ylmethanesulfonyl)-hexahydro-pyrrolo[3,2-*b*]pyrrole-1-carbonyl]-butyl}-benzamide;

589. 7-Methoxy-benzofuran-2-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

590. {1-[6-oxo-4-(1-oxy-pyridin-2-ylmethanesulfonyl)-hexahydro-pyrrolo[3,2-*b*]pyrrole-1-carbonyl]-butyl}-carbamic acid benzyl ester;

591. 5-Methoxy-benzofuran-2-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

592. Thieno[3,2-*b*]thiophene-2-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

593. 3-Methyl-benzofuran-2-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

594. Quinoxaline-2-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

595. Benzo[1,3]dioxole-5-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

596. Quinoline-6-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

597. Furan-2-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

598. Thiophene-2-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

599. N-{1-[6-oxo-4-(1-oxy-pyridin-2-ylmethanesulfonyl)-hexahydro-pyrrolo[3,2-*b*]pyrrole-1-carbonyl]-butyl}-4-trifluoromethyl-benzamide;

600. Furan-3-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

601. Thiophene-3-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

602. N-{1-[6-oxo-4-(1-oxy-pyridin-2-ylmethanesulfonyl)-hexahydro-pyrrolo[3,2-*b*]pyrrole-1-carbonyl]-butyl}-4-phenoxy-benzamide;

603. N-{1-[6-oxo-4-(1-oxy-pyridin-2-ylmethanesulfonyl)-hexahydro-pyrrolo[3,2-*b*]pyrrole-1-carbonyl]-butyl}-4-(4-methyl-piperazin-1-yl)-benzamide;

604. 4-Methyl-N-{1-[6-oxo-4-(1-oxy-pyridin-2-ylmethanesulfonyl)-hexahydro-pyrrolo[3,2-*b*]pyrrole-1-carbonyl]-butyl}-benzamide;

605. 4-Methoxy-N-{1-[6-oxo-4-(1-oxy-pyridin-2-ylmethanesulfonyl)-hexahydro-pyrrolo[3,2-*b*]pyrrole-1-carbonyl]-butyl}-benzamide;

606. 4-Isopropyl-N-{1-[6-oxo-4-(1-oxy-pyridin-2-ylmethanesulfonyl)-hexahydro-pyrrolo[3,2-*b*]pyrrole-1-carbonyl]-butyl}-benzamide;

607. N-{1-[6-oxo-4-(1-oxy-pyridin-2-ylmethanesulfonyl)-hexahydro-pyrrolo[3,2-*b*]pyrrole-1-carbonyl]-butyl}-4-vinyl-benzamide;

608. 4-Imidazol-1-yl-N-{1-[6-oxo-4-(1-oxy-pyridin-2-ylmethanesulfonyl)-hexahydro-pyrrolo[3,2-*b*]pyrrole-1-carbonyl]-butyl}-benzamide;

609. N-{1-[6-oxo-4-(1-oxy-pyridin-2-ylmethanesulfonyl)-hexahydro-pyrrolo[3,2-*b*]pyrrole-1-carbonyl]-butyl}-4-thiophen-2-yl-benzamide;

610. N-{1-[6-oxo-4-(1-oxy-pyridin-2-ylmethanesulfonyl)-hexahydro-pyrrolo[3,2-*b*]pyrrole-1-carbonyl]-butyl}-4-oxazol-5-yl-benzamide;

611. 5-Phenyl-thiophene-2-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

612. N- {1-[6-oxo-4-(1-oxy-pyridin-2-ylmethanesulfonyl)-hexahydro-pyrrolo[3,2-*b*]pyrrole-1-carbonyl]-butyl}-4-trifluoromethoxy-benzamide;

613. 5-Pyridin-2-yl-thiophene-2-carboxylic acid {1-[6-oxo-4-(1-oxy-pyridin-2-ylmethanesulfonyl)-hexahydro-pyrrolo[3,2-*b*]pyrrole-1-carbonyl]-butyl}-amide;

614. 4-Difluoromethoxy-N-{1-[6-oxo-4-(1-oxy-pyridin-2-ylmethanesulfonyl)-hexahydro-pyrrolo[3,2-*b*]pyrrole-1-carbonyl]-butyl}-benzamide;

615. N- {1-[6-oxo-4-(1-oxy-pyridin-2-ylmethanesulfonyl)-hexahydro-pyrrolo[3,2-*b*]pyrrole-1-carbonyl]-butyl}-4-morpholin-4-yl-benzamide;

616. Naphthalene-2-carboxylic acid {3-methyl-1-[6-oxo-4-(1-oxy-pyridin-3-ylmethanesulfonyl)hexahydro-pyrrolo[3,2-*b*]pyrrole-1-carbonyl]-butyl}-amide;

617. Quinoline-2-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

618. Benzo[b]thiophene-2-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

619. Benzofuran-2-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

620. Biphenyl-4-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

621. 4-*tert*-Butyl-N-{3-methyl-1-[6-oxo-4-(1-oxy-pyridin-3-ylmethanesulfonyl)-hexahydro-pyrrolo[3,2-*b*]pyrrole-1-carbonyl]-butyl}-benzamide;

622. 4-Dimethylamino-N-{3-methyl-1-[6-oxo-4-(1-oxy-pyridin-3-ylmethanesulfonyl)-hexahydro-pyrrolo[3,2-*b*]pyrrole-1-carbonyl]-butyl}-benzamide;

623. 7-Methoxy-benzofuran-2-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

624. {3-Methyl-1-[6-oxo-4-(1-oxy-pyridin-3-ylmethanesulfonyl)-hexahydro-pyrrolo[3,2-*b*]pyrrole-1-carbonyl]-butyl}-carbamic acid benzyl ester;

625. 5-Methoxy-benzofuran-2-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

626. Thieno[3,2-*b*]thiophene-2-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

627. 3-Methyl-benzofuran-2-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

628. Quinoxaline-2-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

629. Benzo[1,3]dioxole-5-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

630. Quinoline-6-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

631. Furan-2-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

632. Thiophene-2-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

633. N-{3-Methyl-1-[6-oxo-4-(1-oxy-pyridin-3-ylmethanesulfonyl)-hexahydro-pyrrolo[3,2-*b*]pyrrole-1-carbonyl]-butyl}-4-trifluoromethyl-benzamide;

634. Furan-3-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

635. Thiophene-3-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

636. N-{3-Methyl-1-[6-oxo-4-(1-oxy-pyridin-3-ylmethanesulfonyl)-hexahydro-pyrrolo[3,2-*b*]pyrrole-1-carbonyl]-butyl}-4-phenoxy-benzamide;

637. N-{3-Methyl-1-[6-oxo-4-(1-oxy-pyridin-3-ylmethanesulfonyl)-hexahydro-pyrrolo[3,2-*b*]pyrrole-1-carbonyl]-butyl}-4-(4-methyl-piperazin-1-yl)-benzamide;

638. 4-Methyl-N- {3-methyl-1-[6-oxo-4-(1-oxy-pyridin-3-ylmethanesulfonyl)-hexahydro-pyrrolo[3,2-*b*]pyrrole-1-carbonyl]-butyl}-benzamide;

639. 4-Methoxy-N-{3-methyl-1-[6-oxo-4-(1-oxy-pyridin-3-ylmethanesulfonyl)-hexahydro-pyrrolo[3,2-*b*]pyrrole-1-carbonyl]-butyl}-benzamide;

640. 4-Isopropyl-N-{3-methyl-1-[6-oxo-4-(1-oxy-pyridin-3-ylmethanesulfonyl)-hexahydro-pyrrolo[3,2-*b*]pyrrole-1-carbonyl]-butyl}-benzamide;

641. N-{3-Methyl-1-[6-oxo-4-(1-oxy-pyridin-3-ylmethanesulfonyl)-hexahydro-pyrrolo[3,2-*b*]pyrrole-1-carbonyl]-butyl}-4-vinyl-benzamide;

642. 4-Imidazol-1-yl-N-{3-methyl-1-[6-oxo-4-(1-oxy-pyridin-3-ylmethanesulfonyl)-hexahydro-pyrrolo[3,2-*b*]pyrrole-1-carbonyl]-butyl}-benzamide;

643. N-{3-Methyl-1-[6-oxo-4-(1-oxy-pyridin-3-ylmethanesulfonyl)-hexahydro-pyrrolo[3,2-*b*]pyrrole-1-carbonyl]-butyl}-4-thiophen-2-yl-benzamide;

644. N-{3-Methyl-1-[6-oxo-4-(1-oxy-pyridin-3-ylmethanesulfonyl)-hexahydro-pyrrolo[3,2-*b*]pyrrole-1-carbonyl]-butyl}-4-oxazol-5-yl-benzamide;

645. 5-Phenyl-thiophene-2-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

646. N-{3-Methyl-1-[6-oxo-4-(1-oxy-pyridin-3-ylmethanesulfonyl)-hexahydro-pyrrolo[3,2-*b*]pyrrole-1-carbonyl]-butyl}-4-trifluoromethoxy-benzamide;

647. 5-Pyridin-2-yl-thiophene-2-carboxylic acid {3-methyl-1-[6-oxo-4-(1-oxy-pyridin-3-ylmethanesulfonyl)-hexahydro-pyrrolo[3,2-*b*]pyrrole-1-carbonyl]-butyl}-amide;

648. 4-Difluoromethoxy-N- {3-methyl-1-[6-oxo-4-(1-oxy-pyridin-3-ylmethanesulfonyl)-hexahydro-pyrrolo[3,2- *b*]pyrrole-1-carbonyl]-butyl}-benzamide;

649. N-{3-Methyl-1-[6-oxo-4-(1-oxy-pyridin-3-ylmethanesulfonyl)-hexahydro-pyrrolo[3,2-*b*]pyrrole-1-carbonyl]-butyl}-4-morpholin-4-yl-benzamide;

650. Naphthalene-2-carboxylic acid {1-[6-oxo-4-(1-oxy-pyridin-3-ylmethanesulfonyl)hexahydro-pyrrolo[3,2- *b*]pyrrole-1-carbonyl]-butyl}-amide;

651. Quinoline-2-carboxylic acid {1-[6-oxo-4-(1-oxy-pyridin-3-ylmethanesulfonyl)-hexahydro-pyrrolo[3,2-*b*]pyrrole-1-carbonyl]-butyl}-amide;

652. Benzo[b]thiophene-2-carboxylic acid {1-[6-oxo-4-(1-oxy-pyridin-3-ylmethanesulfonyl)-hexahydro-pyrrolo[3,2-*b*]pyrrole-1-carbonyl]-butyl}-amide;

653. Benzofuran-2-carboxylic acid {1-[6-oxo-4-(1-oxy-pyridin-3-ylmethanesulfonyl)-hexahydro-pyrrolo[3,2- *b*]pyrrole-1-carbonyl]-butyl}-amide;

654. Biphenyl-4-carboxylic acid {1-[6-oxo-4-(1-oxy-pyridin-3-ylmethanesulfonyl)-hexahydro-pyrrolo[3,2- *b*]pyrrole-1-carbonyl]-butyl}-amide;

655. 4-*tert*-Butyl-N-{1-[6-oxo-4-(1-oxy-pyridin-3-ylmethanesulfonyl)-hexahydro-pyrrolo[3,2-*b*]pyrrole-1-carbonyl]-butyl}-benzamide;

656. 4-Dimethylamino-N- {1-[6-oxo-4-(1-oxy-pyridin-3-ylmethanesulfonyl)-hexahydro-pyrrolo[3,2-*b*]pyrrole-1-carbonyl]-butyl}-benzamide;

657. 7-Methoxy-benzofuran-2-carboxylic acid {1-[6-oxo-4-(1-oxy-pyridin-3-ylmethanesulfonyl)-hexahydro-pyrrolo[3,2-*b*]pyrrole-1-carbonyl]-butyl}-amide;

658. {1-[6-oxy-4-(1-oxy-pyridin-3-ylmethanesulfonyl)-hexahydro-pyrrolo[3,2-*b*]pyrrole-1-carbonyl]-butyl}-carbamic acid benzyl ester;

659. 5-Methoxy-benzofuran-2-carboxylic acid {1-[6-oxo-4-(1-oxy-pyridin-3-ylmethanesulfonyl)-hexahydro-pyrrolo-[3,2-*b*]pyrrole-1-carbonyl]-butyl}-amide;

660. Thieno[3,2-*b*]thiophene-2-carboxylic acid {1-[6-oxo-4-(1-oxy-pyridin-3-ylmethanesulfonyl)-hexahydro-pyrrolo-[3,2-*b*]pyrrole-1-carbonyl]-butyl}-amide;

661. 3-Methyl-benzofuran-2-carboxylic acid {1-[6-oxo-4-(1-oxy-pyridin-3-ylmethanesulfonyl)-hexahydro-pyrrolo-[3,2-*b*]pyrrole-1-carbonyl]-butyl}-amide;

662. Quinoxaline-2-carboxylic acid {1-[6-oxo-4-(1-oxy-pyridin-3-ylmethanesulfonyl)-hexahydro-pyrrolo[3,2-*b*]pyrrole-1-carbonyl]-butyl}-amide;

663. Benzo[1,3]dioxole-5-carboxylic acid {1-[6-oxo-4-(1-oxy-pyridin-3-ylmethanesulfonyl)-hexahydro-pyrrolo-[3,2-*b*]pyrrole-1-carbonyl]-butyl}-amide;

664. Quinoline-6-carboxylic acid {1-[6-oxo-4-(1-oxy-pyridin-3-ylmethanesulfonyl)-hexahydro-pyrrolo[3,2-*b*]pyrrole-1-carbonyl]-butyl}-amide;

665. Furan-2-carboxylic acid {1-[6-oxo-4-(1-oxy-pyridin-3-ylmethanesulfonyl)-hexahydro-pyrrolo[3,2-*b*]pyrrole-1-carbonyl]-butyl}-amide;

666. Thiophene-2-carboxylic acid {1-[6-oxo-4-(1-oxy-pyridin-3-ylmethanesulfonyl)-hexahydro-pyrrolo[3,2-*b*]pyrrole-1-carbonyl]-butyl}-amide;

667. N-{1-[6-oxo-4-(1-oxy-pyridin-3-ylmethanesulfonyl)-hexahydro-pyrrolo[3,2-*b*]pyrrole-1-carbonyl]-butyl}-4-trifluoromethyl-benzamide;

668. Furan-3-carboxylic acid {1-[6-oxo-4-(1-oxy-pyridin-3-ylmethanesulfonyl)-hexahydro-pyrrolo[3,2-*b*]pyrrole-1-carbonyl]-butyl}-amide;

669. Thiophene-3-carboxylic acid {1-[6-oxo-4-(1-oxy-pyridin-3-ylmethanesulfonyl)-hexahydro-pyrrolo[3,2-*b*]pyrrole-1-carbonyl]-butyl}-amide;

670. N-{1-[6-oxo-4-(1-oxy-pyridin-3-ylmethanesulfonyl)-hexahydro-pyrrolo[3,2-*b*]pyrrole-1-carbonyl]-butyl}-4-phenoxy-benzamide;

671. N-{1-[6-oxo-4-(1-oxy-pyridin-3-ylmethanesulfonyl)-hexahydro-pyrrolo[3,2-*b*]pyrrole-1-carbonyl]-butyl}-4-(4-methyl-piperazin-1-yl)-benzamide;

672. 4-Methyl-N- {1-[6-oxo-4-(1-oxy-pyridin-3-ylmethanesulfonyl)-hexahydro-pyrrolo[3,2-*b*]pyrrole-1-carbonyl]-butyl}-benzamide;

673. 4-Methoxy-N-{1-[6-oxo-4-(1-oxy-pyridin-3-ylmethanesulfonyl)-hexahydro-pyrrolo[3,2-*b*]pyrrole-1-carbonyl]-butyl}-benzamide;

674. 4-Isopropyl-N- {1-[6-oxo-4-(1-oxy-pyridin-3-ylmethanesulfonyl)-hexahydro-pyrrolo[3,2-*b*]pyrrole-1-carbonyl]-butyl}-benzamide;

675. N- {1-[6-oxo-4-(1-oxy-pyridin-3-ylmethanesulfonyl)-hexahydro-pyrrolo[3,2-*b*]pyrrole-1-carbonyl]-butyl}-4-vinyl-benzamide;

676. 4-Imidazol-1-yl-N- {1-[6-oxo-4-(1-oxy-pyridin-3-ylmethanesulfonyl)-hexahydro-pyrrolo[3,2-*b*]pyrrole-1-carbonyl]-butyl}-benzamide;

677. N-{1-[6-oxo-4-(1-oxy-pyridin-3-ylmethanesulfonyl)-hexahydro-pyrrolo[3,2-*b*]pyrrole-1-carbonyl]-butyl}-4-thiophen-2-yl-benzamide;

678. N-{1-[6-oxo-4-(1-oxy-pyridin-3-ylmethanesulfonyl)-hexahydro-pyrrolo[3,2-*b*]pyrrole-1-carbonyl]-butyl}-4-oxazol-5-yl-benzamide;

679. 5-Phenyl-thiophene-2-carboxylic acid {1-[6-oxo-4-(1-oxy-pyridin-3-ylmethanesulfonyl)-hexahydro-pyrrolo[3,2-*b*]pyrrole-1-carbonyl]-butyl}-amide;

680. N-{1-[6-oxo-4-(1-oxy-pyridin-3-ylmethanesulfonyl)-hexahydro-pyrrolo[3,2-*b*]pyrrole-1-carbonyl]-butyl}-4-trifluoromethoxy-benzamide;

681. 5-Pyridin-2-yl-thiophene-2-carboxylic acid {1-[6-oxo-4-(1-oxy-pyridin-3-ylmethanesulfonyl)-hexahydro-pyrrolo[3,2-*b*]pyrrole-1-carbonyl]-butyl}-amide;

682. 4-Difluoromethoxy-N- {1-[6-oxo-4-(1-oxy-pyridin-3-ylmethanesulfonyl)-hexahydro-pyrrolo[3,2-*b*]pyrrole-1-carbonyl]-butyl}-benzamide;

683. N- {1-[6-oxo-4-(1-oxy-pyridin-3-ylmethanesulfonyl)-hexahydro-pyrrolo[3,2-*b*]pyrrole-1-carbonyl]-butyl}-4-morpholin-4-yl-benzamide;

684. 4-*tert*-Butyl-N-{1-[6-oxo-4-(pyridine-2-sulfonyl)-hexahydro-pyrrolo[3,2-*b*]pyrrole-1-carbonyl]-cyclohexyl}-benzamide;

685. 5-Methoxy-benzofuran-2-carboxylic acid {1-[6-oxo-4-(pyridine-2-sulfonyl)-hexahydro-pyrrolo[3,2-*b*]pyrrole-1-carbonyl]-cyclohexyl}-amide;

686. Thieno[3,2-*b*]thiophene-2-carboxylic acid {1-[6-oxo-4-(pyridine-2-sulfonyl)-hexahydro-pyrrolo[3,2-*b*]pyrrole-1-carbonyl]-cyclohexyl}-amide;

687. 3-Methyl-benzofuran-2-carboxylic acid {1-[6-oxo-4-(pyridine-2-sulfonyl)-hexahydro-pyrrolo[3,2-*b*]pyrrole-1-carbonyl]-cyclohexyl}-amide;

688. N-{1-[6-oxo-4-(pyridine-2-sulfonyl)-hexahydro-pyrrolo[3,2-*b*]pyrrole-1-carbonyl]-cyclohexyl}-4-phenoxy-benzamide;

689. N-{1-[6-oxo-4-(pyridine-2-sulfonyl)-hexahydro-pyrrolo[3,2-*b*]pyrrole-1-carbonyl]-cyclohexyl}-4-thiophen-2-yl-benzamide;

690. 4-*tert*-Butyl-N-{1-cyclopropylmethyl-2-oxo-2-[6-oxo-4-(pyridine-2-sulfonyl)-hexahydro-pyrrolo[3,2-*b*]pyrrol-1-yl]-ethyl}-benzamide;

691. 5-Methoxy-benzofuran-2-carboxylic acid {1-cyclopropylmethyl-2-oxo-2-[6-oxo-4-(pyridine-2-sulfonyl)-hexahydro-pyrrolo[3,2-*b*]pyrrol-1-yl]-ethyl}-amide;

692. Thieno[3,2-*b*]thiophene-2-carboxylic acid {1-cyclopropylmethyl-2-oxo-2-[6-oxo-4-(pyridine-2-sulfonyl)-hexahydro-pyrrolo[3,2-*b*]pyrrol-1-yl]-ethyl}-amide;

693. 3-Methyl-benzofuran-2-carboxylic acid {1-cyclopropylmethyl-2-oxo-2-[6-oxo-4-(pyridine-2-sulfonyl)-hexahydro-pyrrolo[3,2-*b*]pyrrol-1-yl]-ethyl}-amide;

694. N-{1-cyclopropylmethyl-2-oxo-2-[6-oxo-4-(pyridine-2-sulfonyl)-hexahydro-pyrrolo[3,2-*b*]pyrrol-1-yl]-ethyl}-4-phenoxy-benzamide;

695. N-{1-cyclopropylmethyl-2-oxo-2-[6-oxo-4-(pyridine-2-sulfonyl)-hexahydro-pyrrolo[3,2-*b*]pyrrol-1-yl]-ethyl}-4-thiophen-2-yl-benzamide;

696. 4-*tert*-Butyl-N-{2-oxo-2-[6-oxo-4-(pyridine-2-sulfonyl)-hexahydro-pyrrolo[3,2-*b*]pyrrol-1-yl]-1-thiophen-2-ylmethyl-ethyl}-benzamide;

697. 5-Methoxy-benzofuran-2-carboxylic acid {2-oxo-2-[6-oxo-4-(pyridine-2-sulfonyl)-hexahydro-pyrrolo[3,2-*b*]pyrrol-1-yl]-1-thiophen-2-ylmethyl-ethyl}-amide;

698. Thieno[3,2-*b*]thiophene-2-carboxylic acid {2-oxo-2-[6-oxo-4-(pyridine-2-sulfonyl)-hexahydro-pyrrolo[3,2-*b*]pyrrol-1-yl]-1-thiophen-2-ylmethyl-ethyl}-amide;

699. 3-Methyl-benzofuran-2-carboxylic acid {2-oxo-2-[6-oxo-4-(pyridine-2-sulfonyl)-hexahydro-pyrrolo[3,2-*b*]pyrrol-1-yl]-1-thiophen-2-ylmethyl-ethyl}-amide;

700. N-{2-oxo-2-[6-oxo-4-(pyridine-2-sulfonyl)-hexahydro-pyrrolo[3,2-*b*]pyrrol-1-yl]-1-thiophen-2-ylmethyl-ethyl}-4-phenoxy-benzamide;

701. N-{2-oxo-2-[6-oxo-4-(pyridine-2-sulfonyl)-hexahydro-pyrrolo[3,2-*b*]pyrrol-1-yl]-1-thiophen-2-ylmethyl-ethyl}-4-thiophen-2-yl-benzamide;

702. 4-*tert*-Butyl-N-{1-[6-oxo-4-(pyridine-3-sulfonyl)-hexahydro-pyrrolo[3,2-*b*]pyrrole-1-carbonyl]-cyclohexyl}-benzamide;

703. 5-Methoxy-benzofuran-2-carboxylic acid {1-[6-oxo-4-(pyridine-3-sulfonyl)-hexahydro-pyrrolo[3,2-*b*]pyrrole-1-carbonyl]-cyclohexyl}-amide;

704. Thieno[3,2-*b*]thiophene-2-carboxylic acid {1-[6-oxo-4-(pyridine-3-sulfonyl)-hexahydro-pyrrolo[3,2-*b*]pyrrole-1-carbonyl]-cyclohexyl}-amide;

705. 3-Methyl-benzofuran-2-carboxylic acid {1-[6-oxo-4-(pyridine-3-sulfonyl)-hexahydro-pyrrolo[3,2-*b*]pyrrole-1-carbonyl]-cyclohexyl}-amide;

706. N-{1-[6-oxo-4-(pyridine-3-sulfonyl)-hexahydro-pyrrolo[3,2-*b*]pyrrole-1-carbonyl]-cyclohexyl}-4-phenoxy-benzamide;

707. N-{1-[6-oxo-4-(pyridine-3-sulfonyl)-hexahydro-pyrrolo[3,2-*b*]pyrrole-1-carbonyl]-cyclohexyl}-4-thiophen-2-yl-benzamide;

708. 4-*tert*-Butyl-N-{1-cyclopropylmethyl-2-oxo-2-[6-oxo-4-(pyridine-3-sulfonyl)-hexahydro-pyrrolo[3,2-*b*]pyrrol-1-yl]-ethyl}-benzamide;

709. 5-Methoxy-benzofuran-2-carboxylic acid {1-cyclopropylmethyl-2-oxo-2-[6-oxo-4-(pyridine-3-sulfonyl)-hexahydro-pyrrolo[3,2-*b*]pyrrol-1-yl]-ethyl}-amide;

710. Thieno[3,2-*b*]thiophene-2-carboxylic acid {1-cyclopropylmethyl-2-oxo-2-[6-oxo-4-(pyridine-3-sulfonyl)-hexahydro-pyrrolo[3,2-*b*]pyrrol-1-yl]-ethyl}-amide;

711. 3-Methyl-benzofuran-2-carboxylic acid {1-cyclopropylmethyl-2-oxo-2-[6-oxo-4-(pyridine-3-sulfonyl)-hexahydro-pyrrolo[3,2-*b*]pyrrol-1-yl]-ethyl}-amide;

712. N-{1-cyclopropylmethyl-2-oxo-2-[6-oxo-4-(pyridine-3-sulfonyl)-hexahydro-pyrrolo[3,2-*b*]pyrrol-1-yl]-ethyl}-4-phenoxy-benzamide;

713. N-{1-cylopropylmethyl-2-oxo-2-[6-oxo-4-(pyridine-3-sulfonyl)-hexahydro-pyrrolo[3,2-*b*]pyrrol-1-yl]-ethyl}-4-t hiophen-2-yl-benzamide;

714. 4-*tert*-Butyl-N-{2-oxo-2-[6-oxo-4-(pyridine-3-sulfonyl)-hexahydro-pyrrolo[3,2-*b*]pyrrol-1-yl]-1-thiophen-2-ylmethyl-ethyl}-benzamide;

715. 5-Methoxy-benzofuran-2-carboxylic acid {2-oxo-2-[6-oxo-4-(pyridine-3-sulfonyl)-hexahydro-pyrrolo[3,2-*b*]pyrrol-1-yl]-1-thiophen-2-ylmethyl-ethyl}-amide;

716. Thieno[3,2-*b*]thiophene-2-carboxylic acid {2-oxo-2-[6-oxo-4-(pyridine-3-sulfonyl)-hexahydro-pyrrolo[3,2-*b*]pyrrol-1-yl]-1-thiophen-2-ylmethyl-ethyl}-amide;

717. 3-Methyl-benzofuran-2-carboxylic acid {2-oxo-2-[6-oxo-4-(pyridine-3-sulfonyl)-hexahydro-pyrrolo[3,2-*b*]pyrrol-1-yl]-1-thiophen-2-ylmethyl-ethyl}-amide;

718. N-{2-oxo-2-[6-oxo-4-(pyridine-3-sulfonyl)-hexahydro-pyrrolo[3,2-*b*]pyrrol-1-yl]-1-thiophen-2-ylmethyl-ethyl}-4-phenoxy-benzamide;

719. N-{2-oxo-2-[6-oxo-4-(pyridine-3-sulfonyl)-hexahydro-pyrrolo[3,2-*b*]pyrrol-1-yl]-1-thiophen-2-ylmethyl-ethyl}-4-thiophen-2-yl-benzamide;

720. 4-*tert*-Butyl-N-{1-[6-oxo-4-(1-oxy-pyridine-2-sulfonyl)-hexahydro-pyrrolo[3,2-*b*]pyrrole-1-carbonyl]-cyclohexyl}-benzamide;

721. 5-Methoxy-benzofuran-2-carboxylic acid {1-[6-oxo-4-(1-oxy-pyridine-2-sulfonyl)-hexahydro-pyrrolo[3,2-*b*]pyrrole-1-carbonyl]-cyclohexyl}-amide;

722. Thieno[3,2-*b*]thiophene-2-carboxylic acid {1-[6-oxo-4-(1-oxy-pyridine-2-sulfonyl)-hexahydro-pyrrolo[3,2-*b*]pyrrole-1-carbonyl]-cyclohexyl}-amide;

723. 3-Methyl-benzofuran-2-carboxylic acid {1-[6-oxo-4-(1-oxy-pyridine-2-sulfonyl)-hexahydro-pyrrolo[3,2-*b*]pyrrole-1-carbonyl]-cyclohexyl}-amide;

724. N-{1-[6-oxo-4-(1-oxy-pyridine-2-sulfonyl)-hexahydro-pyrrolo[3,2-*b*]pyrrole-1-carbonyl]-cyclohexyl}-4-phenoxy-benzamide;

725. N-{1-[6-oxo-4-(1-oxy-pyridine-2-sulfonyl)-hexahydro-pyrrolo[3,2-*b*]pyrrole-1-carbonyl]-cyclohexyl}-4-thiophen-2-yl-benzamide;

726. 4-*tert*-Butyl-N-{1-cyclopropylmethyl-2-oxo-2-[6-oxo-4-(1-oxy-pyridine-2-sulfonyl)-hexahydro-pyrrolo[3,2-*b*]pyrrol-1-yl]-ethyl}-benzamide;

727. 5-Methoxy-benzofuran-2-carboxylic acid {1-cyclopropylmethyl-2-oxo-2-[6-oxo-4-(1-oxy-pyridine-2-sulfonyl)-hexahydro-pyrrolo[3,2-*b*]pyrrol-1-yl]-ethyl}-amide;

728. Thieno[3,2-*b*]thiophene-2-carboxylic acid {1-cyclopropylmethyl-2-oxo-2-[6-oxo-4-(1-oxy-pyridine-2-sulfonyl)-hexahydro-pyrrolo[3,2-*b*]pyrrol-1-yl]-ethyl}-amide;

729. 3-Methyl-benzofuran-2-carboxylic acid {1-cyclopropylmethyl-2-oxo-2-[6-oxo-4-(1-oxy-pyridine-2-sulfonyl)-hexahydro-pyrrolo[3,2-*b*]pyrrol-1-yl]-ethyl}-amide;

730. N-{1-cyclopropylmethyl-2-oxo-2-[6-oxo-4-(1-oxy-pyridine-2-sulfonyl)-hexahydro-pyrrolo[3,2-*b*]pyrrol-1-yl]-ethyl}-4-phenoxy-benzamide;

731. N-{1-cyclopropylmethyl-2-oxo-2-[6-oxo-4-(1-oxy-pyridine-2-sulfonyl)-hexahydro-pyrrolo[3,2-*b*]pyrrol-1-yl]-ethyl}-4-thiophen-2-yl-benzamide;

732. 4-*ter*t-Butyl-N-{2-oxo-2-[6-oxo-4-(1-oxy-pyridine-2-sulfonyl)-hexahydro-pyrrolo[3,2-*b*]pyrrol-1-yl]-1-thiophen-2-ylmethyl-ethyl}-benzamide;

733. 5-Methoxy-benzofuran-2-carboxylicacid {2-oxo-2-[6-oxo-4-(1-oxy-pyridine-2-sulfonyl)-hexahydro-pyrrolo[3,2-*b*]pyrrol-1-yl]-1-thiophen-2-ylmethyl-ethyl}-amide;

734. Thieno[3,2-*b*]thiophene-2-carboxylicacid {2-oxo-2-[6-oxo-4-(1-oxy-pyridine-2-sulfonyl)-hexahydro-pyrrolo[3,2-*b*]pyrrol-1-yl]-1-thiophen-2-ylmethyl-ethyl}-amide;

735. 3-Methyl-benzofuran-2-carboxylic acid {2-oxo-2-[6-oxo-4-(1-oxy-pyridine-2-sulfonyl)-hexahydro-pyrrolo[3,2-*b*]pyrrol-1-yl]-1-thiophen-2-ylmethyl-ethyl}-amide;

736. N-{2-oxo-2-[6-oxo-4-(1-oxy-pyridine-2-sulfonyl)-hexahydro-pyrrolo[3,2-*b*]pyrrol-1-yl]-1-thiophen-2-ylmethyl-ethyl}-4-phenoxy-benzamide;

737. N-{2-oxo-2-[6-oxo-4-(1-oxy-pyridine-2-sulfonyl)-hexahydro-pyrrolo[3,2-*b*]pyrrol-1-yl]-1-thiophen-2-ylmethyl-ethyl}-4-thiophen-2-yl-benzamide;

738. 4-*tert*-Butyl-N-{1-[6-oxo-4-(1-oxy-pyridine-3-sulfonyl)-hexahydro-pyrrolo[3,2-*b*]pyrrole-1-carbonyl]-cyclohexyl}-benzamide;

739. 5-Methoxy-benzofuran-2-carboxylic acid {1-[6-oxo-4-(1-oxy-pyridine-3-sulfonyl)-hexahydro-pyrrolo[3,2-*b*]pyrrole-1-carbonyl]-cyclohexyl}-amide;

740. Thieno[3,2-*b*]thiophene-2-carboxylic acid {1-[6-oxo-4-(1-oxy-pyridine-3-sulfonyl)-hexahydro-pyrrolo[3,2-*b*]pyrrole-1-carbonyl]-cyclohexyl}-amide;

741. 3-Methyl-benzofuran-2-carboxylic acid {1-[6-oxo-4-(1-oxy-pyridine-3-sulfonyl)-hexahydro-pyrrolo[3,2-*b*]pyrrole-1-carbonyl]-cyclohexyl}-amide;

742. N-{1-[6-oxo-4-(1-oxy-pyridine-3-sulfonyl)-hexahydro-pyrrolo[3,2-*b*]pyrrole-1-carbonyl]-cyclohexyl}-4-phenoxy-benzamide;

743. N-{1-[6-oxo-4-(1-oxy-pyridine-3-sulfonyl)-hexahydro-pyrrolo[3,2-*b*]pyrrole-1-carbonyl]-cyclohexyl}-4-thiophen-2-yl-benzamide;

744. 4-*tert*-Butyl-N-{1-cyclopropylmethyl-2-oxo-2-[6-oxo-4-(1-oxy-pyridine-3-sulfonyl)-hexahydro-pyrrolo[3,2-*b*]pyrrol-1-yl]-ethyl}-benzamide;

745. 5-Methoxy-benzofuran-2-carboxylic acid {1-cyclopropylmethyl-2-oxo-2-[6-oxo-4-(1-oxy-pyridine-3-sulfonyl)-hexahydro-pyrrolo[3,2-*b*]pyrrol-1-yl]-ethyl}-amide;

746. Thieno[3,2-*b*]thiophene-2-carboxylic acid {1-cyclopropylmethyl-2-oxo-2-[6-oxo-4-(1-oxy-pyridine-3-sulfonyl)-hexahydro-pyrrolo[3,2-*b*]pyrrol-1-yl]-ethyl}-amide;

747. 3-Methyl-benzofuran-2-carboxylic acid {1-cyclopropylmethyl-2-oxo-2-[6-oxo-4-(1-oxy-pyridine-3-sulfonyl)-hexahydro-pyrrolo[3,2-*b*]pyrrol-1-yl]-ethyl}-amide;

748. N-{1-cyclopropylmethyl-2-oxo-2-[6-oxo-4-(1-oxy-pyridine-3-sulfonyl)-hexahydro-pyrrolo[3,2-*b*]pyrrol-1-yl]-ethyl}-4-phenoxy-benzamide;

749. N-{1-cyclopropylmethyl-2-oxo-2-[6-oxo-4-(1-oxy-pyridine-3-sulfonyl)-hexahydro-pyrrolo[3,2-*b*]pyrrol-1-yl]-ethyl}-4-thiophen-2-yl-benzamide;

750. 4-*tert*-Butyl-N-{2-oxo-2-[6-oxo-4-(1-oxy-pyridine-3-sulfonyl)-hexahydro-pyrrolo[3,2-*b*]pyrrol-1-yl]-1-thiophen-2-ylmethyl-ethyl}-benzamide;

751. 5-Methoxy-benzofuran-2-carboxylic acid {2-oxo-2-[6-oxo-4-(1-oxy-pyridine-3-sulfonyl)-hexahydro-pyrrolo[3,2-*b*]pyrrol-1-yl]-1-thiophen-2-ylmethyl-ethyl}-amide;

752. Thieno[3,2-*b*]thiophene-2-carboxylic acid {2-oxo-2-[6-oxo-4-(1-oxy-pyridine-3-sulfonyl)-hexahydro-pyrrolo[3,2-*b*]pyrrol-1-yl]-1-thiophen-2-ylmethyl-ethyl}-amide;

753. 3-Methyl-benzofuran-2-carboxylic acid {2-oxo-2-[6-oxo-4-(1-oxy-pyridine-3-sulfonyl)-hexahydro-pyrrolo[3,2-*b*]pyrrol-1-yl]-1-thiophen-2-ylmethyl-ethyl}-amide;

754. N-{2-oxo-2-[6-oxo-4-(1-oxy-pyridine-3-sulfonyl)-hexahydro-pyrrolo[3,2-*b*]pyrrol-1-yl]-1-thiophen-2-ylmethyl-ethyl}-4-phenoxy-benzamide;

755. N-{2-oxo-2-[6-oxo-4-(1-oxy-pyridine-3-sulfonyl)-hexahydro-pyrrolo[3,2-*b*]pyrrol-1-yl]-1-thiophen-2-ylmethyl-ethyl}-4-thiophen-2-yl-benzamide;

756. 4-*tert*-Butyl-N-{1-[6-oxo-4-(pyridine-2-carbonyl)-hexahydro-pyrrolo[3,2-*b*]pyrrole-1-carbonyl]-cyclohexyl}-benzamide;

757. 5-Methoxy-benzofuran-2-carboxylic acid {1-[6-oxo-4-(pyridine-2-carbonyl)-hexahydro-pyrrolo[3,2-*b*]pyrrole-1-carbonyl]-cyclohexyl}-amide;

758. Thieno[3,2-*b*]thiophene-2-carboxylic acid {1-[6-oxo-4-(pyridine-2-carbonyl)-hexahydro-pyrrolo[3,2-*b*]pyrrole-1-carbonyl]-cyclohexyl}-amide;

759. 3-Methyl-benzofuran-2-carboxylic acid {1-[6-oxo-4-(pyridine-2-carbonyl)-hexahydro-pyrrolo[3,2-*b*]pyrrole-1-carbonyl]-cyclohexyl}-amide;

760. N-{1-[6-oxo-4-(pyridine-2-carbonyl)-hexahydro-pyrrolo[3,2-*b*]pyrrole-1-carbonyl]-cyclohexyl}-4-phenoxy-benzamide;

761. N-{1-[6-oxo-4-(pyridine-2-carbonyl)-hexahydro-pyrrolo[3,2-*b*]pyrrole-1-carbonyl]-cyclohexyl}-4-thiophen-2-yl-benzamide;

762. 4-*tert*-Butyl-N-{1-cyclopropylmethyl-2-oxo-2-[6-oxo-4-(pyridine-2-carbonyl)-hexahydro-pyrrolo[3,2-*b*]pyrrol-1-yl]-ethyl}-benzamide;

763. 5-Methoxy-benzofuran-2-carboxylic acid {1-cyclopropylmethyl-2-oxo-2-[6-oxo-4-(pyridine-2-carbonyl)-hexahydro-pyrrolo[3,2-*b*]pyrrol-1-yl]-ethyl}-amide;

. 764. Thieno[3,2-*b*]thiophene-2-carboxylic acid {1-cyclopropylmethyl-2-oxo-2-[6-oxo-4-(pyridine-2-carbonyl)-hexahydro-pyrrolo[3,2-*b*]pyrrol-1-yl]-ethyl}-amide;

. 765. 3-Methyl-benzofuran-2-carboxylic acid {1-cyclopropylmethyl-2-oxo-2-[6-oxo-4-(pyridine-2-carbonyl)-hexahydro-pyrrolo[3,2-*b*]pyrrol-1-yl]-ethyl}-amide;

. 766. N-{1-cyclopropylmethyl-2-oxo-2-[6-oxo-4-(pyridine-2-carbonyl)-hexahydro-pyrrolo[3,2-*b*]pyrrol-1-yl]-ethyl}-4-phenoxy-benzamide;

. 767. N-{1-cyclopropylmethyl-2-oxo-2-[6-oxo-4-(pyridine-2-carbonyl)-hexahydro-pyrrolo[3,2-*b*]pyrrol-1-yl]-ethyl}-4-thiophen-2-yl-benzamide;

768. 4-*tert*-Butyl-N-{2-oxo-2-[6-oxo-4-(pyridine-2-carbonyl)-hexahydro-pyrrolo[3,2-*b*]pyrrol-1-yl]-1-thiophen-2-ylmethyl-ethyl}-benzamide;

769. 5-Methoxy-benzofuran-2-carboxylic acid {2-oxo-2-[6-oxo-4-(pyridine-2-carbonyl)-hexahydro-pyrrolo[3,2-*b*]pyrrol-1-yl]-1-thiophen-2-ylmethyl-ethyl}-amide;

770. Thieno[3,2-*b*]thiophene-2-carboxylic acid {2-oxo-2-[6-oxo-4-(pyridine-2-carbonyl)-hexahydro-pyrrolo[3,2-*b*]-pyrrol-1-yl]-1-thiophen-2-ylmethyl-ethyl}-amide;

771. 3-Methyl-benzofuran-2-carboxylic acid {2-oxo-2-[6-oxo-4-(pyridine-2-carbonyl)-hexahydro-pyrrolo[3,2-*b*]pyrrol-1-yl]-1-thiophen-2-ylmethyl-ethyl}-amide;

772. N-{2-oxo-2-[6-oxo-4-(pyridine-2-carbonyl)-hexahydro-pyrrolo[3,2-*b*]pyrrol-1-yl]-1-thiophen-2-ylmethyl-ethyl}-4-phenoxy-benzamide;

. 773. N-{2-oxo-2-[6-oxo-4-(pyridine-2-carbonyl)-hexahydro-pyrrolo[3,2-*b*]pyrrol-1-yl]-1-thiophen-2-ylmethyl-ethyl}-4-thiophen-2-yl-benzamide;

774. 4-*tert*-Butyl-N-{1-[6-oxo-4-(2-pyridin-3-yl-acetyl)-hexahydro-pyrrolo[3,2-*b*]pyrrole-1-carbonyl]-cyclohexyl}-benzamide;

775. 5-Methoxy-benzofuran-2-carboxylic acid {1-[6-oxo-4-(2-pyridin-3-yl-acetyl)-hexahydro-pyrrolo[3,2-*b*]pyrrole-1-carbonyl]-cyclohexyl}-amide;

776. Thieno[3,2-*b*]thiophene-2-carboxylic acid {1-[6-oxo-4-(2-pyridin-3-yl-acetyl)-hexahydro-pyrrolo[3,2-*b*]pyrrole-1-carbonyl]-cyclohexyl}-amide;

777. 3-Methyl-benzofuran-2-carboxylic acid {1-[6-oxo-4-(2-pyridin-3-yl-acetyl)-hexahydro-pyrrolo[3,2-*b*]pyrrole-1-carbonyl]-cyclohexyl}-amide;

778. N-{1-[6-oxo-4-(2-pyridin-3-yl-acetyl)-hexahydro-pyrrolo[3,2-*b*]pyrrole-1-carbonyl]-cyclohexyl}-4-phenoxy-benzamide;

779. N-{1-[6-oxo-4-(2-pyridin-3-yl-acetyl)-hexahydro-pyrrolo[3,2-*b*]pyrrole-1-carbonyl]-cyclohexyl}-4-thiophen-2-yl-benzamide;

780. 4-*tert*-Butyl-N-{1-cyclopropylmethyl-2-oxo-2-[6-oxo-4-(2-pyridin-3-yl-acetyl)-hexahydro-pyrrolo[3,2-*b*]pyrrol-1-yl]-ethyl}-benzamide;

781. 5-Methoxy-benzofuran-2-carboxylic acid {1-cyclopropylmethyl-2-oxo-2-[6-oxo-4-(2-pyridin-3-yl-acetyl)-hexahydro-pyrrolo[3,2-*b*]pyrrol-1-yl]-ethyl}-amide;

782. Thieno[3,2-*b*]thiophene-2-carboxylic acid {1-cyclopropylmethyl-2-oxo-2-[6-oxo-4-(2-pyridin-3-yl-acetyl)-hexahydro-pyrrolo[3,2-*b*]pyrrol-1-yl]-ethyl}-amide;

783. 3-Methyl-benzofuran-2-carboxylic acid {1-cyclopropylmethyl-2-oxo-2-[6-oxo-4-(2-pyridin-3-yl-acetyl)-hexahydro-pyrrolo[3,2-*b*]pyrrol-1-yl]-ethyl}-amide;

784. N-{1-cyclopropylmethyl-2-oxo-2-[6-oxo-4-(2-pyridin-3-yl-acetyl)-hexahydro-pyrrolo[3,2-*b*]pyrrol-1-yl]-ethyl}-4-phenoxy-benzamide;

785. N-{1-cyclopropylmethyl-2-oxo-2-[6-oxo-4-(2-pyridin-3-yl-acetyl)-hexahydro-pyrrolo[3,2-*b*]pyrrol-1-yl]-ethyl}-4-thiophen-2-yl-benzamide;

786. 4-*tert*-Butyl-N-{2-oxo-2-[6-oxo-4-(2-pyridin-3-yl-acetyl)-hexahydro-pyrrolo[3,2-*b*]pyrrol-1-yl]-1-thiophen-2-ylmethyl-ethyl}-benzamide;

787. 5-Methoxy-benzofuran-2-carboxylic acid {2-oxo-2-[6-oxo-4-(2-pyridin-3-yl-acetyl)-hexahydro-pyrrolo[3,2-*b*]-pyrrol-1-yl]-1-thiophen-2-ylmethyl-ethyl}-amide;

788. Thieno[3,2-*b*]thiophene-2-carboxylic acid {2-oxo-2-[6-oxo-4-(2-pyridin-3-yl-acetyl)-hexahydro-pyrrolo[3,2-*b*]-pyrrol-1-yl]-1-thiophen-2-ylmethyl-ethyl}-amide;

789. 3-Methyl-benzofuran-2-carboxylic acid {2-oxo-2-[6-oxo-4-(2-pyridin-3-yl-acetyl)-hexahydro-pyrrolo[3,2-*b*]pyrrol-1-yl]-1-thiophen-2-ylmethyl-ethyl}-amide;

790. N-{2-oxo-2-[6-oxo-4-(2-pyridin-3-yl-acetyl)-hexahydro-pyrrolo[3,2-*b*]pyrrol-1-yl]-1-thiophen-2-ylmethyl-ethyl}-4-phenoxy-benzamide;

791. N-{2-oxo-2-[6-oxo-4-(2-pyridin-3-yl-acetyl)-hexahydro-pyrrolo[3,2-*b*]pyrrol-1-yl]-1-thiophen-2-ylmethyl-ethyl}-4-thiophen-2-yl-benzamide;

792. 4-*tert*-Butyl-N-{1-[6-oxo-4-(pyridin-2-ylmethanesulfonyl)-hexahydro-pyrrolo[3,2-*b*]pyrrole-1-carbonyl]-cyclohexyl}-benzamide;

793. 5-Methoxy-benzofuran-2-carboxylic acid {1-[6-oxo-4-(pyridin-2-ylmethanesulfonyl)-hexahydro-pyrrolo[3,2-*b*]-pyrrole-1-carbonyl]-cyclohexyl}-amide;

794. Thieno[3,2-*b*]thiophene-2-carboxylic acid {1-[6-oxo-4-(pyridin-2-ylmethanesulfonyl)-hexahydro-pyrrolo[3,2-*b*]-pyrrole-1-carbonyl]-cyclohexyl}-amide;

795. 3-Methyl-benzofuran-2-carboxylic acid {1-[6-oxo-4-(pyridin-2-ylmethanesulfonyl)-hexahydro-pyrrolo[3,2-*b*]pyrrole-1-carbonyl]-cyclohexyl}-amide;

796. N-{1-[6-oxo-4-(pyridin-2-ylmethanesulfonyl)-hexahydro-pyrrolo[3,2-*b*]pyrrole-1-carbonyl]-cyclohexyl}-4-phenoxy-benzamide;

797. N-{1-[6-oxo-4-(pyridin-2-ylmethanesulfonyl)-hexahydro-pyrrolo[3,2-*b*]pyrrole-1-carbonyl]-cyclohexyl}-4-thiophen-2-yl-benzamide;

798. 4-*tert*-Butyl-N-{1-cyclopropylmethyl-2-oxo-2-[6-oxo-4-(pyridin-2-ylmethanesulfonyl)-hexahydro-pyrrolo[3,2-*b*]-pyrrol-1-yl]-ethyl}-benzamide;

799. 5-Methoxy-benzofuran-2-carboxylic acid {1-cyclopropylmethyl-2-oxo-2-[6-oxo-4-(pyridin-2-ylmethanesulfonyl)-hexahydro-pyrrolo[3,2-*b*]pyrrol-1-yl]-ethyl}-amide;

800. Thieno[3,2-*b*]thiophene-2-carboxylic acid {1-cyclopropylmethyl-2-oxo-2-[6-oxo-4-(pyridin-2-ylmethanesulfonyl)-hexahydro-pyrrolo[3,2-*b*]pyrrol-1-yl]-ethyl}-amide;

801. 3-Methyl-benzofuran-2-carboxylic acid {1-cyclopropylmethyl-2-oxo-2-[6-oxo-4-(pyridin-2-ylmethanesulfonyl)-hexahydro-pyrrolo[3,2-*b*]pyrrol-1-yl]-ethyl}-amide;

802. N-{1-cyclopropylmethyl-2-oxo-2-[6-oxo-4-(pyridin-2-ylmethanesulfonyl)-hexahydro-pyrrolo[3,2-*b*]pyrrol-1-yl]-ethyl}-4-phenoxy-benzamide;

803. N-{1-cyclopropylmethyl-2-oxo-2-[6-oxo-4-(pyridin-2-ylmethanesulfonyl)-hexahydro-pyrrolo[3,2-*b*]pyrrol-1-yl]-ethyl}-4-thiophen-2-yl-benzamide;

804. 4-*tert*-Butyl-N-{2-oxo-2-[6-oxo-4-(pyridin-2-ylmethanesulfonyl)-hexahydro-pyrrolo[3,2-*b*]pyrrol-1-yl]-1-thiophen-2-ylmethyl-ethyl}-benzamide;

805. 5-Methoxy-benzofuran-2-carboxylic acid {2-oxo-2-[6-oxo-4-(pyridin-2-ylmethanesulfonyl)-hexahydro-pyrrolo[3,2-*b*]pyrrol-1-yl]-1-thiophen-2-ylmethyl-ethyl}-amide;

806. Thieno[3,2-*b*]thiophene-2-carboxylic acid {2-oxo-2-[6-oxo-4-(pyridin-2-ylmethanesulfonyl)-hexahydro-pyrrolo[3,2-*b*]pyrrol-1-yl]-1-thiophen-2-ylmethyl-ethyl}-amide;

807. 3-Methyl-benzofuran-2-carboxylic acid {2-oxo-2-[6-oxo-4-(pyridin-2-ylmethanesulfonyl)-hexahydro-pyrrolo[3,2-*b*]pyrrol-1-yl]-1-thiophen-2-ylmethyl-ethyl}-amide;

808. N-{2-oxo-2-[6-oxo-4-(pyridin-2-ylmethanesulfonyl)-hexahydro-pyrrolo[3,2-*b*]pyrrol-1-yl]-1-thiophen-2-ylmethyl-ethyl}-4-phenoxy-benzamide;

809. N-{2-oxo-2-[6-oxo-4-(pyridin-2-ylmethanesulfonyl)-hexahydro-pyrrolo[3,2-*b*]pyrrol-1-yl]-1-thiophen-2-ylmethyl-ethyl}-4-thiophen-2-yl-benzamide;

810. 4-*tert*-Butyl-N-{1-[6-oxo-4-(pyridin-3-ylmethanesulfonyl)-hexahydro-pyrrolo[3,2-*b*]pyrrole-1-carbonyl]-cyclohexyl}- benzamide;

811. 5-Methoxy-benzofuran-2-carboxylic acid {1-[6-oxo-4-(pyridin-3-ylmethanesulfonyl)-hexahydro-pyrrolo[3,2-*b*]-pyrrole-1-carbonyl]-cyclohexyl}-amide;

812. Thieno[3,2-*b*]thiophene-2-carboxylic acid {1-[6-oxo-4-(pyridin-3-ylmethanesulfonyl)-hexahydro-pyrrolo[3,2-*b*]-pyrrole-1-carbonyl]-cyclohexyl}-amide;

813. 3-Methyl-benzofuran-2-carboxylic acid {1-[6-oxo-4-(pyridin-3-ylmethanesulfonyl)-hexahydro-pyrrolo[3,2- *b*]pyrrole-1-carbonyl]-cyclohexyl}-amide;

814. N-{1-[6-oxo-4-(pyridin-3-ylmethanesulfonyl)-hexahydro-pyrrolo[3,2-*b*]pyrrole-1-carbonyl]-cyclohexyl}-4-phenoxy-benzamide;

815. N-{1-[6-oxo-4-(pyridin-3-ylmethanesulfonyl)-hexahydro-pyrrolo[3,2-*b*]pyrrole-1-carbonyl]-cyclohexyl}-4-thiophen-2-yl-benzamide;

816. 4-*tert*-Butyl-N- {1-cyclopropylmethyl-2-oxo-2-[6-oxo-4-(pyridin-3-ylmethanesulfonyl)-hexahydro-pyrrolo[3,2-*b*]pyrrol-1-yl]-ethyl}-benzamide;

817. 5-Methoxy-benzofuran-2-carboxylic acid {1-cyclopropylmethyl-2-oxo-2-[6-oxo-4-(pyridin-3-ylmethanesulfonyl)-hexahydro-pyrrolo[3,2-*b*]pyrrol-1-yl]-ethyl}-amide;

818. Thieno[3,2-*b*]thiophene-2-carboxylic acid {1-cyclopropylmethyl-2-oxo-2-[6-oxo-4-(pyridin-3-ylmethanesulfonyl)-hexahydro-pyrrolo[3,2-*b*]pyrrol-1-yl]-ethyl}-amide;

819. 3-Methyl-benzofuran-2-carboxylic acid {1-cyclopropylmethyl-2-oxo-2-[6-oxo-4-(pyridin-3-ylmethanesulfonyl)-hexahydro-pyrrolo[3,2-*b*]pyrrol-1-yl]-ethyl}-amide;

820. N-{1-cyclopropylmethyl-2-oxo-2-[6-oxo-4-(pyridin-3-ylmethanesulfonyl)-hexahydro-pyrrolo[3,2-*b*]pyrrol-1-yl]-ethyl}-4-phenoxy-benzamide;

821. N-{1-cyclopropylmethyl-2-oxo-2-[6-oxo-4-(pyridin-3-ylmethanesulfonyl)-hexahydro-pyrrolo[3,2-*b*]pyrrol-1-yl]-ethyl}-4-thiophen-2-yl-benzamide;

822. 4-*tert*-Butyl-N-{2-oxo-2-[6-oxo-4-(pyridin-3-ylmethanesulfonyl)-hexahydro-pyrrolo[3,2-*b*]pyrrol-1-yl]-1-thiophen-2-ylmethyl-ethyl}-benzamide;

823. 5-Methoxy-benzofuran-2-carboxylic acid {2-oxo-2-[6-oxo-4-(pyridin-3-ylmethanesulfonyl)-hexahydro-pyrrolo[3,2-*b*]pyrrol-1-yl]-1-thiophen-2-ylmethyl-ethyl}-amide;

824. Thieno[3,2-*b*]thiophene-2-carboxylic acid {2-oxo-2-[6-oxo-4-(pyridin-3-ylmethanesulfonyl)-hexahydro-pyrrolo[3,2-*b*]pyrrol-1-yl]-1-thiophen-2-ylmethyl-ethyl}-amide;

825. 3-Methyl-benzofuran-2-carboxylic acid {2-oxo-2-[6-oxo-4-(pyridin-3-ylmethanesulfonyl)-hexahydro-pyrrolo[3,2-*b*]pyrrol-1-yl]-1-thiophen-2-ylmethyl-ethyl}-amide;

826. N-{2-oxo-2-[6-oxo-4-(pyridin-3-ylmethanesulfonyl)-hexahydro-pyrrolo[3,2-*b*]pyrrol-1-yl]-1-thiophen-2-ylmethyl-ethyl}-4-phenoxy-benzamide;

827. N-{2-oxo-2-[6-oxo-4-(pyridin-3-ylmethanesulfonyl)-hexahydro-pyrrolo[3,2-*b*]pyrrol-1-yl]-1-thiophen-2-ylmethyl-ethyl}-4-thiophen-2-yl-benzamide;

828. 4-*tert*-Butyl-N-{1-[6-oxo-4-(1-oxy-pyridin-2-ylmethanesulfonyl)-hexahydro-pyrrolo[3,2-*b*]pyrrole-1-carbonyl]-cyclohexyl}-benzamide;

829. 5-Methoxy-benzofuran-2-carboxylic acid {1-[6-oxo-4-(1-oxy-pyridin-2-ylmethanesulfonyl)-hexahydro-pyrrolo[3,2-*b*]pyrrole-1-carbonyl]-cyclohexyl}-amide;

830. Thieno[3,2-*b*]thiophene-2-carboxylic acid {1-[6-oxo-4-(1-oxy-pyridin-2-ylmethanesulfonyl)-hexahydro-pyrrolo[3,2-*b*]pyrrole-1-carbonyl]-cyclohexyl}-amide;

831. 3-Methyl-benzofuran-2-carboxylic acid {1-[6-oxo-4-(1-oxy-pyridin-2-ylmethanesulfonyl)-hexahydro-pyrrolo[3,2-*b*]pyrrole-1-carbonyl]-cyclohexyl}-amide;

832. N-{1-[6-oxo-4-(1-oxy-pyridin-2-ylmethanesulfonyl)-hexahydro-pyrrolo[3,2-*b*]pyrrole-1-carbonyl]-cyclohexyl}-4-phenoxy-benzamide;

833. N-{1-[6-oxo-4-(1-oxy-pyridin-2-ylmethanesulfonyl)-hexahydro-pyrrolo[3,2-*b*]pyrrole-1-carbonyl]-cyclohexyl}-4-thiophen-2-yl-benzamide;

834. 4-*tert*-Butyl-N-{1-cyclopropylmethyl-2-oxo-2-[6-oxo-4-(1-oxy-pyridin-2-ylmethanesulfonyl)-hexahydro-pyrrolo-[3,2-*b*]pyrrol-1-yl]-ethyl}-benzamide;

835. 5-Methoxy-benzofuran-2-carboxylic acid {1-cyclopropylmethyl-2-oxo-2-[6-oxo-4-(1-oxy-pyridin-2-ylmethanesulfonyl)-hexahydro-pyrrolo[3,2-*b*]pyrrol-1-yl]-ethyl}-amide;

836. Thieno[3,2-*b*]thiophene-2-carboxylic acid {1-cyclopropylmethyl-2-oxo-2-[6-oxo-4-(1-oxy-pyridin-2-ylmethanesulfonyl)-hexahydro-pyrrolo[3,2-*b*]pyrrol-1-yl]-ethyl}-amide;

837. 3-Methyl-benzofuran-2-carboxylic acid {1-cyclopropylmethyl-2-oxo-2-[6-oxo-4-(1-oxy-pyridin-2-ylmethanesulfonyl)-hexahydro-pyrrolo[3,2-*b*]pyrrol-1-yl]-ethyl}-amide;

838. N-{1-cyclopropylmethyl-2-oxo-2-[6-oxo-4-(1-oxy-pyridin-2-ylmethanesulfonyl)-hexahydro-pyrrolo[3,2-*b*]pyrrol-1-yl]-ethyl}-4-phenoxy-benzamide;

839. N-{1-cyclopropylmethyl-2-oxo-2-[6-oxo-4-(1-oxy-pyridin-2-ylmethanesulfonyl)-hexahydro-pyrrolo[3,2-*b*]pyrrol-1-yl]-ethyl}-4-thiophen-2-yl-benzamide;

840. 4-*tert*-Butyl-N-{2-oxo-2-[6-oxo-4-(1-oxy-pyridin-2-ylmethanesulfonyl)-hexahydro-pyrrolo[3,2-*b*]pyrrol-1-yl]-1-thiophen-2-ylmethyl-ethyl}-benzamide;

841. 5-Methoxy-benzofuran-2-carboxylic acid {2-oxo-2-[6-oxo-4-(1-oxy-pyridin-2-ylmethanesulfonyl)-hexahydro-pyrrolo[3,2-*b*]pyrrol-1-yl]-1-thiophen-2-ylmethyl-ethyl}-amide;

842. Thieno[3,2-*b*]thiophene-2-carboxylic acid {2-oxo-2-[6-oxo-4-(1-oxy-pyridin-2-ylmethanesulfonyl)-hexahydro-pyrrolo[3,2-*b*]pyrrol-1-yl]-1-thiophen-2-ylmethyl-ethyl}-amide;

843. 3-Methyl-benzofuran-2-carboxylic acid {2-oxo-2-[6-oxo-4-(1-oxy-pyridin-2-ylmethanesulfonyl)-hexahydro-pyrrolo[3,2-*b*]pyrrol-1-yl]-1-thiophen-2-ylmethyl-ethyl}-amide;

844. N-{2-oxo-2-[6-oxo-4-(1-oxy-pyridin-2-ylmethanesulfonyl)-hexahydro-pyrrolo[3,2-*b*]pyrrol-1-yl]-1-thiophen-2-ylmethyl-ethyl}-4-phenoxy-benzamide;

845. N-{2-oxo-2-[6-oxo-4-(1-oxy-pyridin-2-ylmethanesulfonyl)-hexahydro-pyrrolo[3,2-*b*]pyrrol-1-yl]-1-thiophen-2-ylmethyl-ethyl}-4-thiophen-2-yl-benzamide;

846. 4-*tert*-Butyl-N-{1-[6-oxo-4-(1-oxy-pyridin-3-ylmethanesulfonyl)-hexahydro-pyrrolo[3,2-*b*]pyrrole-1-carbonyl]-cyclohexyl}-benzamide;

847. 5-Methoxy-benzofuran-2-carboxylic acid {1-[6-oxo-4-(1-oxy-pyridin-3-ylmethanesulfonyl)-hexahydro-pyrrolo[3,2-*b*]pyrrole-1-carbonyl]-cyclohexyl}-amide;

848. Thieno[3,2-*b*]thiophene-2-carboxylic acid {1-[6-oxo-4-(1-oxy-pyridin-3-ylmethanesulfonyl)-hexahydro-pyrrolo[3,2-*b*]pyrrole-1-carbonyl]-cyclohexyl}-amide;

849. 3-Methyl-benzofuran-2-carboxylic acid {1-[6-oxo-4-(1-oxy-pyridin-3-ylmethanesulfonyl)-hexahydro-pyrrolo[3,2-*b*]pyrrole-1-carbonyl]-cyclohexyl}-amide;

850. N-{1-[6-oxo-4-(1-oxy-pyridin-3-ylmethanesulfonyl)-hexahydro-pyrrolo[3,2-*b*]pyrrole-1-carbonyl]-cyclohexyl}-4-phenoxy-benzamide;

851. N-{1-[6-oxo-4-(1-oxy-pyridin-3-ylmethanesulfonyl)-hexahydro-pyrrolo[3,2-*b*]pyrrole-1-carbonyl]-cyclohexyl}-4-thiophen-2-yl-benzamide;

852. 4-*tert*-Butyl-N-{1-cyclopropylmethyl-2-oxo-2-[6-oxo-4-(1-oxy-pyridin-3-ylmethanesulfonyl)-hexahydro-pyrrolo-[3,2-*b*]pyrrol-1-yl]-ethyl}-benzamide;

853. 5-Methoxy-benzofuran-2-carboxylic acid {1-cyclopropylmethyl-2-oxo-2-[6-oxo-4-(1-oxy-pyridin-3-ylmethanesulfonyl)-hexahydro-pyrrolo[3,2-*b*]pyrrol-1-yl]-ethyl}-amide;

854. Thieno[3,2-*b*]thiophene-2-carboxylic acid {1-cyclopropylmethyl-2-oxo-2-[6-oxo-4-(1-oxy-pyridin-3-ylmethanesulfonyl)-hexahydro-pyrrolo[3,2-*b*]pyrrol-1-yl]-ethyl}-amide;

855. 3-Methyl-benzofuran-2-carboxylic acid {1-cyclopropylmethyl-2-oxo-2-[6-oxo-4-(1-oxy-pyridin-3-ylmethanesulfonyl)-hexahydro-pyrrolo[3,2-*b*]pyrrol-1-yl]-ethyl}-amide;

856. N-{1-cyclopropylmethyl-2-oxo-2-[6-oxo-4-(1-oxy-pyridin-3-ylmethanesulfonyl)-hexahydro-pyrrolo[3,2-*b*]pyrrol-1-yl]-ethyl}-4-phenoxy-benzamide;

857. N-{1-cyclopropylmethyl-2-oxo-2-[6-oxo-4-(1-oxy-pyridin-3-ylmethanesulfonyl)-hexahydro-pyrrolo[3,2-*b*]pyrrol-1-yl]-ethyl}-4-thiophen-2-yl-benzamide;

. 858. 4-*tert*-Butyl-N-{2-oxo-2-[6-oxo-4-(1-oxy-pyridin-3-ylmethanesulfonyl)-hexahydro-pyrrolo[3,2-*b*]pyrrol-1-yl]-1-thiophen-2-ylmethyl-ethyl}-benzamide;

859. 5-Methoxy-benzofuran-2-carboxylic acid {2-oxo-2-[6-oxo-4-(1-oxy-pyridin-3-ylmethanesulfonyl)-hexahydro-pyrrolo[3,2-*b*]pyrrol-1-yl]-1-thiophen-2-ylmethyl-ethyl}-amide;

860. Thieno[3,2-*b*]thiophene-2-carboxylic acid {2-oxo-2-[6-oxo-4-(1-oxy-pyridin-3-ylmethanesulfonyl)-hexahydro-p

yrrolo[3,2-*b*]pyrrol-1-yl]-1-thiophen-2-ylmethyl-ethyl}-amide;

861. 3-Methyl-benzofuran-2-carboxylic acid {2-oxo-2-[6-oxo-4-(1-oxy-pyridin-3-ylmethanesulfonyl)-hexahydro-pyrrolo[3,2-*b*]pyrrol-1-yl]-1-thiophen-2-ylmethyl-ethyl}-amide;

862. N-{2-oxo-2-[6-oxo-4-(1-oxy-pyridin-3-ylmethanesulfonyl)-hexahydro-pyrrolo[3,2-*b*]pyrrol-1-yl]-1-thiophen-2-ylmethyl-ethyl}-4-phenoxy-benzamide;

863. N-{2-oxo-2-[6-oxo-4-(1-oxy-pyridin-3-ylmethanesulfonyl)-hexahydro-pyrrolo[3,2-*b*]pyrrol-1-yl]-1-thiophen-2-ylmethyl-ethyl}-4-thiophen-2-yl-benzamide;

864. 2-Isobutyl-4-morpholin-4-yl-1-[6-oxo-4-(pyridine-2-sulfonyl)-hexahydro-pyrrolo[3,2-*b*]pyrrol-1-yl]-butane-1,4-dione;

865. 2-Isobutyl-1-[6-oxo-4-(pyridine-2-sulfonyl)-hexahydro-pyrrolo[3,2-*b*]pyrrol-1-yl]-4-piperazin-1-yl-butane-1,4-dione;

866. 2-Isobutyl-4-(4-methyl-piperazin-1-yl)-1-[6-oxo-4-(pyridine-2-sulfonyl)-hexahydro-pyrrolo[3,2-*b*]pyrrol-1-yl]-butane-1,4-dione;

867. 2-Isobutyl-1-[6-oxo-4-(pyridine-2-sulfonyl)-hexahydro-pyrrolo[3,2-*b*]pyrrol-1-yl]-4-(4-phenyl-piperazin-1-yl)-butane-1,4-dione;

868. 2-Isobutyl-1-[6-oxo-4-(pyridine-2-sulfonyl)-hexahydro-pyrrolo[3,2-*b*]pyrrol-1-yl]-4-(3,4,4a,8a-tetrahydro-1H-isoquinolin-2-yl)-butane-1,4-dione;

869. 2-Isobutyl-1-[6-oxo-4-(pyridine-2-sulfonyl)-hexahydro-pyrrolo[3,2-*b*]pyrrol-1-yl]-4-(1,3,3a,7a-tetrahydro-isoindol-2-yl)-butane-1,4-dione;

870. 4-(4-Benzyl-piperazin-1-yl)-2-isobutyl-1-[6-oxo-4-(pyridine-2-sulfonyl)-hexahydro-pyrrolo[3,2-*b*]pyrrol-1-yl]-butane-1,4-dione;

871. 2-Isobutyl-4-morpholin-4-yl-1-[6-oxo-4-(pyridine-3-sulfonyl)-hexahydro-pyrrolo[3,2-*b*]pyrrol-1-yl]-butane-1,4-dione;

872. 2-Isobutyl-1-[6-oxo-4-(pyridine-3-sulfonyl)-hexahydro-pyrrolo[3,2-*b*]pyrrol-1-yl]-4-piperazin-1-yl-butane-1,4-dione;

873. 2-Isobutyl-4-(4-methyl-piperazin-1-yl)-1-[6-oxo-4-(pyridine-3-sulfonyl)-hexahydro-pyrrolo[3,2-*b*]pyrrol-1-yl]-butane-1,4-dione;

874. 2-Isobutyl-1-[6-oxo-4-(pyridine-3-sulfonyl)-hexahydro-pyrrolo[3,2-*b*]pyrrol-1-yl]-4-(4-phenyl-piperazin-1-yl)-butane-1,4-dione;

875. 2-Isobutyl-1-[6-oxo-4-(pyridine-3-sulfonyl)-hexahydro-pyrrolo[3,2-*b*]pyrrol-1-yl]-4-(3,4,4a,8a-tetrahydro-1H-isoquinolin-2-yl)-butane-1,4-dione;

876. 2-Isobutyl-1-[6-oxo-4-(pyridine-3-sulfonyl)-hexahydro-pyrrolo[3,2-*b*]pyrrol-1-yl]-4-(1,3,3a,7a-tetrahydro-isoindol- 2-yl)-butane-1,4-dione;

877. 4-(4-Benzyl-piperazin-1-yl)-2-isobutyl-1-[6-oxo-4-(pyridine-3-sulfonyl)-hexahydro-pyrrolo[3,2-*b*]pyrrol-1-yl]-butane-1,4-dione;

878. 2-Isobutyl-4-morpholin-4-yl-1-[6-oxo-4-(1-oxy-pyridine-2-sulfonyl)-hexahydro-pyrrolo[3,2-*b*]pyrrol-1-yl]-butane-1,4-dione;

879. 2-Isobutyl-1-[6-oxo-4-(1-oxy-pyridine-2-sulfonyl)-hexahydro-pyrrolo[3,2-*b*]pyrrol-1-yl]-4-piperazin-1-yl-butane-1,4-dione;

880. 2-Isobutyl-4-(4-methyl-piperazin-1-yl)-1-[6-oxo-4-(1-oxy-pyridine-2-sulfonyl)-hexahydro-pyrrolo[3,2-*b*]pyrrol-1-yl]-butane-1,4-dione;

881. 2-Isobutyl-1-[6-oxo-4-(1-oxy-pyridine-2-sulfonyl)-hexahydro-pyrrolo[3,2-*b*]pyrrol-1-yl]-4-(4-phenyl-piperazin-1-yl)-butane-1,4-dione;

882. 2-Isobutyl-1-[6-oxo-4-(1-oxy-pyridine-2-sulfonyl)-hexahydro-pyrrolo[3,2-*b*]pyrrol-1-yl]-4-(3,4,4a,8a-tetrahydro-1H-isoquinolin-2-yl)-butane-1,4-dione;

883. 2-Isobutyl-1-[6-oxo-4-(1-bxy-pyridine-2-sulfonyl)-hexahydro-pyrrolo[3,2-*b*]pyrrol-1-yl]-4-(1,3,3a,7a-tetrahydro-isoindol-2-yl)-butane-1,4-dione;

884. 4-(4-Benzyl-piperazin-1-yl)-2-isobutyl-1-[6-oxo-4-(1-oxy-pyridine-2-sulfonyl)-hexahydro-pyrrolo[3,2-*b*]pyrrol-1-yl]-butane-1,4-dione;

885. 2-Isobutyl-4-morpholin-4-yl-1-[6-oxo-4-(1-oxy-pyridine-3-sulfonyl)-hexahydro-pyrrolo[3,2-*b*]pyrrol-1-yl]-butane-1,4-dione;

886. 2-Isobutyl-1-[6-oxo-4-(1-oxy-pyridine-3-sulfonyl)-hexahydro-pyrrolo[3,2-*b*]pyrrol-1-yl]-4-piperazin-1-yl-butane-1,4-dione;

887. 2-Isobutyl-4-(4-methyl-piperazin-1-yl)-1-[6-oxo-4-(1-oxy-pyridine-3-sulfonyl)-hexahydro-pyrrolo[3,2-*b*]pyrrol-1-yl]-butane-1,4-dione;

888. 2-Isobutyl-1-[6-oxo-4-(1-oxy-pyridine-3-sulfonyl)-hexahydro-pyrrolo[3,2-*b*]pyrrol-1-yl]-4-(4-phenyl-piperazin-1-yl)-butane-1,4-dione;

889. 2-Isobutyl-1-[6-oxo-4-(1-oxy-pyridine-3-sulfonyl)-hexahydro-pyrrolo[3,2-*b*]pyrrol-1-yl]-4-(3,4,4a,8a-tetrahydro-1H-isoquinolin-2-yl)-butane-1,4-dione;

890. 2-Isobutyl-1-[6-oxo-4-(1-oxy-pyridine-3-sulfonyl)-hexahydro-pyrrolo[3,2-*b*]pyrrol-1-yl]-4-(1,3,3a,7a-tetrahydro-isoindol-2-yl)-butane-1,4-dione;

891. 4-(4-Benzyl-piperazin-1-yl)-2-isobutyl-1-[6-oxo-4-(1-oxy-pyridine-3-sulfonyl)-hexahydro-pyrrolo[3,2-*b*]pyrrol-1-yl]-butane-1,4-dione;

892. 2-Isobutyl-4-morpholin-4-yl-1-[6-oxo-4-(pyridine-2-carbonyl)-hexahydro-pyrrolo[3,2-*b*]pyrrol-1-yl]-butane-1,4-dione;

893. 2-Isobutyl-1-[6-oxo-4-(pyridine-2-carbonyl)-hexahydro-pyrrolo[3,2-*b*]pyrrol-1-yl]-4-piperazin-1-yl-butane-1,4- dione;

. 894. 2-Isobutyl-4-(4-methyl-piperazin-1-yl)-1-[6-oxo-4-(pyridine-2-carbonyl)-hexahydro-pyrrolo[3,2-*b*]pyrrol-1-yl]- butane-1,4-dione;

. 895. 2-Isobutyl-1-[6-oxo-4-(pyridine-2-carbonyl)-hexahydro-pyrrolo[3,2-*b*]pyrrol-1-yl]-4-(4-phenyl-piperazin-1-yl)- butane-1,4-dione;

. 896. 2-Isobutyl-1-[6-oxo-4-(pyridine-2-carbonyl)-hexahydro-pyrrolo[3,2-*b*]pyrrol-1-yl]-4-(3,4,4a,8a-tetrahydro-1H- isoquinolin-2-yl)-butane-1,4-dione;

897. 2-Isobutyl-1-[6-oxo-4-(pyridine-2-carbonyl)-hexahydro-pyrrolo[3,2-*b*]pyrrol-1-yl]-4-(1,3,3a,7a-tetrahydro-isoindol-2-yl)-butane-1,4-dione;

898. 4-(4-Benzyl-piperazin-1-yl)-2-isobutyl-1-[6-oxo-4-(pyridine-2-carbonyl)-hexahydro-pyrrolo[3,2-*b*]pyrrol-1-yl]-butane-1,4-dione;

899. 2-Isobutyl-4-morpholin-4-yl-1-[6-oxo-4-(2-pyridin-3-yl-acetyl)-hexahydro-pyrrolo[3,2- *b*]pyrrol-1-yl]-butane-1,4-dione;

900. 2-Isobutyl-1-[6-oxo-4-(2-pyridin-3-yl-acetyl)-hexahydro-pyrrolo[3,2-*b*]pyrrol-1-yl]-4-piperazin-1-yl-butane-1,4-dione;

901. 2-Isobutyl-4-(4-methyl-piperazin-1-yl)-1-[6-oxo-4-(2-pyridin-3-yl-acetyl)-hexahydro-pyrrolo[3,2-*b*]pyrrol-1-yl]-butane-1,4-dione;

. 902. 2-Isobutyl-1-[6-oxo-4-(2-pyridin-3-yl-acetyl)-hexahydro-pyrrolo[3,2-*b*]pyrrol-1-yl]-4-(4-phenyl-piperazin-1-yl)-butane-1,4-dione;

. 903. 2-Isobutyl-1-[6-oxo-4-(2-pyridin-3-yl-acetyl)-hexahydro-pyrrolo[3,2-*b*]pyrrol-1-yl]-4-(3,4,4a,8a-tetrahydro-1H-isoquinolin-2-yl)-butane-1,4-dione;

. 904. 2-Isobutyl-1-[6-oxo-4-(2-pyridin-3-yl-acetyl)-hexahydro-pyrrolo[3,2-*b*]pyrrol-1-yl]-4-(1,3,3a,7a-tetrahydro-isoindol- 2-yl)-butane-1,4-dione;

. 905. 4-(4-Benzyl-piperazin-1-yl)-2-isobutyl-1-[6-oxo-4-(2-pyridin-3-yl-acetyl)-hexahydro-pyrrolo[3,2-*b*]pyrrol-1-yl]-butane-1,4-dione;

. 906. 2-Isobutyl-4-morpholin-4-yl-1-[6-oxo-4-(pyridin-2-ylmethanesulfonyl)-hexahydro-pyrrolo[3,2-*b*]pyrrol-1-yl]-butane-1,4-dione;

. 907. 2-Isobutyl-1-[6-oxo-4-(pyridin-2-ylmethanesulfonyl)-hexahydro-pyrrolo[3,2-*b*]pyrrol-1-yl]-4-piperazin-1-yl-butane-1,4-dione;

. 908. 2-Isobutyl-4-(4-methyl-piperazin-1-yl)-1-[6-oxo-4-(pyridin-2-ylmethanesulfonyl)-hexahydro-pyrrolo[3,2-*b*]pyrrol-1- yl]-butane-1,4-dione;

. 909. 2-Isobutyl-1-[6-oxo-4-(pyridin-2-ylmethanesulfonyl)-hexahydro-pyrrolo[3,2-*b*]pyrrol-1-yl]-4-(4-phenyl-piperazin-1 -yl)-butane-1,4-dione;

910. 2-Isobutyl-1-[6-oxo-4-(pyridin-2-ylmethanesulfonyl)-hexahydro-pyrrolo[3,2-*b*]pyrrol-1-yl]-4-(3,4,4a,8a-tetrahydro-1H-isoquinolin-2-yl)-butane-1,4-dione;

. 911. 2-Isobutyl-1-[6-oxo-4-(pyridin-2-ylmethanesulfonyl)-hexahydro-pyrrolo[3,2-*b*]pyrrol-1-yl]-4-(1,3,3a,7a-tetrahydro-isoindol-2-yl)-butane-1,4-dione;

912. 4-(4-Benzyl-piperazin-1-yl)-2-isobutyl-1-[6-oxo-4-(pyridin-2-ylmethanesulfonyl)-hexahydro-pyrrolo[3,2-*b*]pyrrol-1-yl]-butane-1,4-dione;

913. 2-Isobutyl-4-morpholin-4-yl-1-[6-oxo-4-(pyridin-3-ylmethanesulfonyl)-hexahydro-pyrrolo[3,2-*b*]pyrrol-1-yl]-butane-1,4-dione;

914. 2-Isobutyl-1-[6-oxo-4-(pyridin-3-ylmethanesulfonyl)-hexahydro-pyrrolo[3,2-*b*]pyrrol-1-yl]-4-piperazin-1-yl-butane-1,4-dione;

915. 2-Isobutyl-4-(4-methyl-piperazin-1-yl)-1-[6-oxo-4-(pyridin-3-ylmethanesulfonyl)-hexahydro-pyrrolo[3,2-*b*]pyrrol-1-yl]-butane-1,4-dione;

916. 2-Isobutyl-1-[6-oxo-4-(pyridin-3-ylmethanesulfonyl)-hexahydro-pyrrolo[3,2-*b*]pyrrol-1-yl]-4-(4-phenyl-piperazin-1-yl)-butane-1,4-dione;

917. 2-Isobutyl-1-[6-oxo-4-(pyridin-3-ylmethanesulfonyl)-hexahydro-pyrrolo[3,2-*b*]pyrrol-1-yl]-4-(3,4,4a,8a-tetrahydro-1H-isoquinolin-2-yl)-butane-1,4-dione;

. 918. 2-Isobutyl-1-[6-oxo-4-(pyridin-3-ylmethanesulfonyl)-hexahydro-pyrrolo[3,2-*b*]pyrrol-1-yl]-4-(1,3,3a,7a-tetrahydro-isoindol-2-yl)-butane-1,4-dione;

919. 4-(4-Benzyl-piperazin-1-yl)-2-isobutyl-1-[6-oxo-4-(pyridin-3-ylmethanesulfonyl)-hexahydro-pyrrolo[3,2-*b*]pyrrol-1-yl]-butane-1,4-dione;

920. 2-Isobutyl-4-morpholin-4-yl-1-[6-oxo-4-(1-oxy-pyridin-2-ylmethanesulfonyl)-hexahydro-pyrrolo[3,2-*b*]pyrrol-1-yl]-butane-1,4-dione;

921. 2-Isobutyl-1-[6-oxo-4-(1-oxy-pyridin-2-ylmethanesulfonyl)-hexahydro-pyrrolo[3,2-*b*]pyrrol-1-yl]-4-piperazin-1-yl-butane-1,4-dione;

922. 2-Isobutyl-4-(4-methyl-piperazin-1-yl)-1-[6-oxo-4-(1-oxy-pyridin-2-ylmethanesulfonyl)-hexahydro-pyrrolo[3,2-*b*]pyrrol-1-yl]-butane-1,4-dione;

923. 2-Isobutyl-1-[6-oxo-4-(1-oxy-pyridin-2-ylmethanesulfonyl)-hexahydro-pyrrolo[3,2-*b*]pyrrol-1-yl]-4-(4-phenyl-piperazin-1-yl)-butane-1,4-dione;

924. 2-Isobutyl-1-[6-oxo-4-(1-oxy-pyridin-2-ylmethanesulfonyl)-hexahydro-pyrrolo[3,2-*b*]pyrrol-1-yl]-4-(3,4,4a,8a-tetrahydro-1H-isoquinolin-2-yl)-butane-1,4-dione;

925. 2-Isobutyl-1-[6-oxo-4-(1-oxy-pyridin-2-ylmethanesulfonyl)-hexahydro-pyrrolo[3,2-*b*]pyrrol-1-yl]-4-(1,3,3a,7a-tetrahydro-isoindol-2-yl)-butane-1,4-dione;

926. 4-(4-Benzyl-piperazin-1-yl)-2-isobutyl-1-[6-oxo-4-(1-oxy-pyridin-2-ylmethanesulfonyl)-hexahydro-pyrrolo[3,2-*b*]pyrrol-1-yl]-butane-1,4-dione;

927. 2-Isobutyl-4-morpholin-4-yl-1-[6-oxo-4-(1-oxy-pyridin-3-ylmethanesulfonyl)-hexahydro-pyrrolo[3,2-*b*]pyrrol-1-yl]-butane-1,4-dione;

928. 2-Isobutyl-1-[6-oxo-4-(1-oxy-pyridin-3-ylmethanesulfonyl)-hexahydro-pyrrolo[3,2-*b*]pyrrol-1-yl]-4-piperazin-1-yl-butane-1,4-dione;

. 929. 2-Isobutyl-4-(4-methyl-piperazin-1-yl)-1-[6-oxo-4-(1-oxy-pyridin-3-ylmethanesulfonyl)-hexahydro-pyrrolo[3,2-*b*]pyrrol-1-yl]-butane-1,4-dione;

. 930. 2-Isobutyl-1-[6-oxo-4-(1-oxy-pyridin-3-ylmethanesulfonyl)-hexahydro-pyrrolo[3,2-*b*]pyrrol-1-yl]-4-(4-phenyl-piperazin-1-yl)-butane-1,4-dione;

. 931. 2-Isobutyl-1-[6-oxo-4-(1-oxy-pyridin-3-ylmethanesulfonyl)-hexahydro-pyrrolo[3,2-*b*]pyrrol-1-yl]-4-(3,4,4a,8a-tetrahydro-1H-isoquinolin-2-yl)-butane-1,4-dione;

. 932. 2-Isobutyl-1-[6-oxo-4-(1-oxy-pyridin-3-ylmethanesulfonyl)-hexahydro-pyrrolo[3,2-*b*]pyrrol-1-yl]-4-(1,3,3a,7a-tetrahydro-isoindol-2-yl)-butane-1,4-dione;

933. 4-(4-Benzyl-piperazin-1-yl)-2-isobutyl-1-[6-oxo-4-(1-oxy-pyridin-3-ylmethanesulfonyl)-hexahydro-pyrrolo[3,2-*b*]pyrrol-1-yl]-butane-1,4-dione;

934. 4-(2-Biphenyl-3-yl-4-methyl-pentanoyl)-1-(pyridine-2-sulfonyl)-hexahydro-pyrrolo[3,2-*b*]pyrrol-3-one;

935. 4-(2-Biphenyl-3-yl-4-methyl-pentanoyl)-1-(pyridine-3-sulfonyl)-hexahydro-pyrrolo[3,2-*b*]pyrrol-3-one;

936. 4-(2-Biphenyl-3-yl-4-methyl-pentanoyl)-1-(1-oxy-pyridine-2-sulfonyl)-hexahydro-pyrrolo[3,2-*b*]pyrrol-3-one;

937. 4-(2-Biphenyl-3-yl-4-methyl-pentanoyl)-1-(1-oxy-pyridine-3-sulfonyl)-hexahydro-pyrrolo[3,2-*b*]pyrrol-3-one;

938. 4-(2-Biphenyl-3-yl-4-methyl-pentanoyl)-1-(pyridine-2-carbonyl)-hexahydro-pyrrolo[3,2-*b*]pyrrol-3-one;

939. 4-(2-Biphenyl-3-yl-4-methyl-pentanoyl)-1-(2-pyridin-3-yl-acetyl)-hexahydro-pyrrolo[3,2-*b*]pyrrol-3-one;

940. 4-(2-Biphenyl-3-yl-4-methyl-pentanoyl)-1-(pyridin-2-ylmethanesulfonyl)-hexahydro-pyrrolo[3,2-*b*]pyrrol-3-one;

941. 4-(2-Biphenyl-3-yl-4-methyl-pentanoyl)-1-(pyridin-3-ylmethanesulfonyl)-hexahydro-pyrrolo[3,2-*b*]pyrrol-3-one;

942. 4-(2-Biphenyl-3-yl-4-methyl-pentanoyl)-1-(1-oxy-pyridin-2-ylmethanesulfonyl)-hexahydro-pyrrolo[3,2-*b*]pyrrol-3-one;

943. 4-(2-Biphenyl-3-yl-4-methyl-pentanoyl)-1-(1-oxy-pyridin-3-ylmethanesulfonyl)-hexahydro-pyrrolo[3,2-*b*]pyrrol-3-one;

944. 4-[4-Methyl-2-(3-pyridin-2-yl-phenyl)-pentanoyl]-1-(pyridine-2-sulfonyl)-hexahydro-pyrrolo[3,2-*b*]pyrrol-3-one;

945. 4-[4-Methyl-2-(3-pyridin-2-yl-phenyl)-pentanoyl]-1-(pyridine-3-sulfonyl)-hexahydro-pyrrolo[3,2-*b*]pyrrol-3-one;

946. 4-[4-Methyl-2-(3-pyridin-2-yl-phenyl)-pentanoyl]-1-(1-oxy-pyridine-2-sulfonyl)-hexahydro-pyrrolo[3,2-*b*]pyrrol-3-one;

947. 4-[4-Methyl-2-(3-pyridin-2-yl-phenyl)-pentanoyl]-1-(1-oxy-pyridine-3-sulfonyl)-hexahydro-pyrrolo[3,2-*b*]pyrrol-3-one;

948. 4-[4-Methyl-2-(3-pyridin-2-yl-phenyl)-pentanoyl]-1-(pyridine-2-carbonyl)-hexahydro-pyrrolo[3,2-*b*]pyrrol-3-one;

949. 4-[4-Methyl-2-(3-pyridin-2-yl-phenyl)-pentanoyl]-1-(2-pyridin-3-yl-acetyl)-hexahydro-pyrrolo[3,2-*b*]pyrrol-3-one;

950. 4-[4-Methyl-2-(3-pyridin-2-yl-phenyl)-pentanoyl]-1-(pyridin-2-ylmethanesulfonyl)-hexahydro-pyrrolo[3,2-*b*]pyrrol-3-one;

951. 4-[4-Methyl-2-(3-pyridin-2-yl-phenyl)-pentanoyl]-1-(pyridin-3-ylmethanesulfonyl)-hexahydro-pyrrolo[3,2-*b*]pyrrol-3-one;

952. 4-[4-Methyl-2-(3-pyridin-2-yl-phenyl)-pentanoyl]-1-(1-oxy-pyridin-2-ylmethanesulfonyl)-hexahydro-pyrrolo[3,2-*b*]pyrrol-3-one;

953. 4-[4-Methyl-2-(3-pyridin-2-yl-phenyl)-pentanoyl]-1-(1-oxy-pyridin-3-ylmethanesulfonyl)-hexahydro-pyrrolo[3,2-*b*]pyrrol-3-one;

954. 4-[4-Methyl-2-(3-pyridin-3-yl-phenyl)-pentanoyl]-1-(pyridine-2-sulfonyl)-hexahydro-pyrrolo[3,2-*b*]pyrrol-3-one;

955. 4-[4-Methyl-2-(3-pyridin-3-yl-phenyl)-pentanoyl]-1-(pyridine-3-sulfonyl)-hexahydro-pyrrolo[3,2-*b*]pyrrol-3-one;

956. 4-[4-Methyl-2-(3-pyridin-3-yl-phenyl)-pentanoyl]-1-(1-oxy-pyridine-2-sulfonyl)-hexahydro-pyrrolo[3,2-*b*]pyrrol-3-one;

957. 4-[4-Methyl-2-(3-pyridin-3-yl-phenyl)-pentanoyl]-1-(1-oxy-pyridine-3-sulfonyl)-hexahydro-pyrrolo[3,2-*b*]pyrrol-3-one;

958. 4-[4-Methyl-2-(3-pyridin-3-yl-phenyl)-pentanoyl]-1-(pyridine-2-carbonyl)-hexahydro-pyrrolo[3,2-*b*]pyrrol-3-one;

959. 4-[4-Methyl-2-(3-pyridin-3-yl-phenyl)-pentanoyl]-1-(2-pyridin-3-yl-acetyl)-hexahydro-pyrrolo[3,2-*b*]pyrrol-3-one;

960. 4-[4-Methyl-2-(3-pyridin-3-yl-phenyl)-pentanoyl]-1-(pyridin-2-ylmethanesulfonyl)-hexahydro-pyrrolo[3,2-*b*]pyrrol-3-one;

961. 4-[4-Methyl-2-(3-pyridin-3-yl-phenyl)-pentanoyl]-1-(pyridin-3-ylmethanesulfonyl)-hexahydro-pyrrolo[3,2-*b*]pyrrol-3-one;

962. 4-[4-Methyl-2-(3-pyridin-3-yl-phenyl)-pentanoyl]-1-(1-oxy-pyridin-2-ylmethanesulfonyl)-hexahydro-pyrrolo[3,2-*b*]pyrrol-3-one;

963. 4-[4-Methyl-2-(3-pyridin-3-yl-phenyl)-pentanoyl]-1-(1-oxy-pyridin-3-ylmethanesulfonyl)-hexahydro-pyrrolo[3,2-

*b*]pyrrol-3-one;

964. 4-[4-Methyl-2-(3-pyridin-4-yl-phenyl)-pentanoyl]-1-(pyridine-2-sulfonyl)-hexahydro-pyrrolo[3,2-*b*]pyrrol-3-one;

965. 4-[4-Methyl-2-(3-pyridin-4-yl-phenyl)-pentanoyl]-1-(pyridine-3-sulfonyl)-hexahydro-pyrrolo[3,2-*b*]pyrrol-3-one;

966. 4-[4-Methyl-2-(3-pyridin-4-yl-phenyl)-pentanoyl]-1-(1-oxy-pyridine-2-sulfonyl)-hexahydro-pyrrolo[3,2-*b*]pyrrol-3-one;

. 967. 4-[4-Methyl-2-(3-pyridin-4-yl-phenyl)-pentanoyl]-1-(1-oxy-pyridine-3-sulfonyl)-hexahydro-pyrrolo[3,2-*b*]pyrrol-3-one;

. 968. 4-[4-Methyl-2-(3-pyridin-4-yl-phenyl)-pentanoyl]-1-(pyridine-2-carbonyl)-hexahydro-pyrrolo[3,2-*b*]pyrrol-3-one;

. 969. 4-[4-Methyl-2-(3-pyridin-4-yl-phenyl)-pentanoyl]-1-(2-pyridin-3-yl-acetyl)-hexahydro-pyrrolo[3,2-*b*]pyrrol-3-one;

970. 4-[4-Methyl-2-(3-pyridin-4-yl-phenyl)-pentanoyl]-1-(pyridin-2-ylmethanesulfonyl)-hexahydro-pyrrolo[3,2-*b*]pyrrol-3-one;

971. 4-[4-Methyl-2-(3-pyridin-4-yl-phenyl)-pentanoyl]-1-(pyridin-3-ylmethanesulfonyl)-hexahydro-pyrrolo[3,2-*b*]pyrrol-3-one;

972. 4-[4-Methyl-2-(3-pyridin-4-yl-phenyl)-pentanoyl]-1-(1-oxy-pyridin-2-ylmethanesulfonyl)-hexahydro-pyrrolo[3,2-*b*]pyrrol-3-one;

973. 4-[4-Methyl-2-(3-pyridin-4-yl-phenyl)-pentanoyl]-1-(1-oxy-pyridin-3-ylmethanesulfonyl)-hexahydro-pyrrolo[3,2-*b*]pyrrol-3-one.

Other compounds of the invention include, but are not limited to, the following examples where all 4 stereoisomeric combinations of the bicyclic ketone are included where $P_2$ is $CH_2$, i.e. (3a*S*, 6a*S*), (3a*R*, 6a*S*), (3a*S*, 6a*R*), (3aR, 6aR) and also included are the equivalent analogues where $P_2$ is O and NH. More preferred compounds consist of the cis-bicyclic isomers which, when $P_2$ is $CH_2$, are designated as (3a*R*, 6a*S*) and (3a*S*, 6a*R*) and also more preferred are the equivalent cis-bicyclic analogues where $P_2$ is O and NH.

974. Furan-3-carboxylic acid {3,3-dimethyl-1-[6-oxo-4-(pyridine-2-sulfonyl)-hexahydro-pyrrolo[3,2-*b*]pyrrole-1-carbonyl]-butyl}-amide;

975. Thiophene-3-carboxylic acid {3,3-dimethyl-1-[6-oxo-4-(pyridine-2-sulfonyl)-hexahydro-pyrrolo[3,2-*b*]pyrrole-1-carbonyl]-butyl}-amide;

976. Benzo[b]thiophene-3-carboxylic acid {3,3-dimethyl-1-[6-oxo-4-(pyridine-2-sulfonyl)-hexahydro-pyrrolo[3,2-*b*]-pyrrole-1-carbonyl]-butyl}-amide;

977. Furan-3-carboxylic acid {1-cyclohexylmethyl-2-oxo-2-[6-oxo-4-(pyridine-2-sulfonyl)-hexahydro-pyrrolo[3,2-*b*]pyrrol-1-yl]-ethyl}-amide;

978. Thiophene-3-carboxylic acid {1-cyclohexylmethyl-2-oxo-2-[6-oxo-4-(pyridine-2-sulfonyl)-hexahydro-pyrrolo[3,2-*b*]pyrrol-1-yl]-ethyl}-amide;

979. Benzo[b]thiophene-3-carboxylic acid {1-cyclohexylmethyl-2-oxo-2-[6-oxo-4-(pyridine-2-sulfonyl)-hexahydro-pyrrolo[3,2-*b*]pyrrol-1-yl]-ethyl}-amide;

980. Naphthalene-1-carboxylic acid {1-(4-hydroxy-benzyl)-2-oxo-2-[6-oxo-4-(pyridine-2-sulfonyl)-hexahydro-pyrrolo[3,2-*b*]pyrrol-1-yl]-ethyl}-amide;

981. Quinoline-8-carboxylic acid {1-(4-hydroxy-benzyl)-2-oxo-2-[6-oxo-4-(pyridine-2-sulfonyl)-hexahydro-pyrrolo[3,2-*b*]pyrrol-1-yl]-ethyl}-amide;

982. 4-*tert*-Butyl-N-{1-(4-hydroxy-benzyl)-2-oxo-2-[6-oxo-4-(pyridine-2-sulfonyl)-hexahydro-pyrrolo[3,2-*b*]pyrrol-1-yl]-ethyl}-benzamide;

983. 4-*tert*-Butyl-N-{1-(4-fluoro-benzyl)-2-oxo-2-[6-oxo-4-(pyridine-2-sulfonyl)-hexahydro-pyrrolo[3,2-*b*]pyrrol-1-yl]-ethyl}-benzamide;

984. 4-*tert*-Butyl-N-{1-(4-methoxy-benzyl)-2-oxo-2-[6-oxo-4-(pyridine-2-sulfonyl)-hexahydro-pyrrolo[3,2-*b*]pyrrol-1-yl]-ethyl}-benzamide;

985. 4-*tert*-Butyl-N-{1-(3-hydroxy-benzyl)-2-oxo-2-[6-oxo-4-(pyridine-2-sulfonyl)-hexahydro-pyrrolo[3,2-*b*]pyrrol-1-yl]-ethyl}-benzamide;

986. Biphenyl-4-carboxylic acid {1-(4-hydroxy-benzyl)-2-oxo-2-[6-oxo-4-(pyridine-2-sulfonyl)-hexahydro-pyrrolo[3,2-*b*]pyrrol-1-yl]-ethyl}-amide;

987. Biphenyl-4-carboxylic acid {1-(4-fluoro-benzyl)-2-oxo-2-[6-oxo-4-(pyridine-2-sulfonyl)-hexahydro-pyrrolo[3,2-*b*]pyrrol-1-yl]-ethyl}-amide;

988. Biphenyl-4-carboxylic acid {1-(4-methoxy-benzyl)-2-oxo-2-[6-oxo-4-(pyridine-2-sulfonyl)-hexahydro-pyrrolo[3,2-*b*]pyrrol-1-yl]-ethyl}-amide;

989. Biphenyl-4-carboxylic acid {1-(3-hydroxy-benzyl)-2-oxo-2-[6-oxo-4-(pyridine-2-sulfonyl)-hexahydro-pyrrolo[3,2-*b*]pyrrol-1-yl]-ethyl}-amide;

990. Furan-3-carboxylic acid {3,3-dimethyl-1-[6-oxo-4-(yridine-3-sulfonyl)-hexahydro-pyrrolo[3,2-*b*]pyrrole-1-carbonyl]-butyl}-amide;

991. Thiophene-3-carboxylic acid {3,3-dimethyl-1-[6-oxo-4-(pyridine-3-sulfonyl)-hexahydro-pyrrolo[3,2-*b*]pyrtole-1-carbonyl]-butyl}-amide;

992. Benzo[b]thiophene-3-carboxylic acid {3,3-dimethyl-1-[6-oxo-4-(pyridine-3-sulfonyl)-hexahydro-pyrrolo[3,2-*b*]-pyrrole-1-carbonyl]-butyl}-amide;

993. Furan-3-carboxylic acid {1-cyclohexylmethyl-2-oxo-2-[6-oxo-4-(pyridine-3-sulfonyl)-hexahydro-pyrrolo[3,2-*b*]pyrrol-1-yl]-ethyl}-amide;

994. Thiophene-3-carboxylic acid {1-cyclohexylmethyl-2-oxo-2-[6-oxo-4-(pyridine-3-sulfonyl)-hexahydro-pyrrolo[3,2-*b*]pyrrol-1-yl]-ethyl}-amide;

995. Benzo[b]thiophene-3-carboxylic acid {1-cyclohexylmethyl-2-oxo-2-[6-oxo-4-(pyridine-3-sulfonyl)-hexahydro-pyrrolo[3,2-*b*]pyrrol-1-yl]-ethyl}-amide;

996. Naphthalene-1-carboxylic acid {1-(4-hydroxy-benzyl)-2-oxo-2-[6-oxo-4-(pyridine-3-sulfonyl)-hexahydro-pyrrolo[3,2-*b*]pyrrol-1-yl]-ethyl}-amide;

997. Quinoline-8-carboxylic acid {1-(4-hydroxy-benzyl)-2-oxo-2-[6-oxo-4-(pyridine-3-sulfonyl)-hexahydro-pyrrolo[3,2-*b*]pyrrol-1-yl]-ethyl}-amide;

998. 4-*tert*-Butyl-N-{1-(4-hydroxy-benzyl)-2-oxo-2-[6-oxo-4-(pyridine-3-sulfonyl)-hexahydro-pyrrolo[3,2-*b*]pyrrol-1-yl]-ethyl}-benzamide;

999. 4-*tert*-Butyl-N-{1-(4-fluoro-benzyl)-2-oxo-2-[6-oxo-4-(pyridine-3-sulfonyl)-hexahydro-pyrrolo[3,2-*b*]pyrrol-1-yl]-ethyl}-benzamide;

1000. 4-*tert*-Butyl-N-{1-(4-methoxy-benzyl)-2-oxo-2-[6-oxo-4-(pyridine-3-sulfonyl)-hexahydro-pyrrolo[3,2-*b*]pyrrol-1-yl]-ethyl}-benzamide;

1001. 4-*tert*-Butyl-N-{1-(3-hydroxy-benzyl)-2-oxo-2-[6-oxo-4-(pyridine-3-sulfonyl)-hexahydro-pyrrolo[3,2-*b*]pyrrol-1-yl]-ethyl}-benzamide;

1002. Biphenyl-4-carboxylic acid {1-(4-hydroxy-benzyl)-2-oxo-2-[6-oxo-4-(pyridine-3-sulfonyl)-hexahydro-pyrrolo-[3,2-*b*]pyrrol-1-yl]-ethyl}-amide;

1003. Biphenyl-4-carboxylic acid {1-(4-fluoro-benzyl)-2-oxo-2-[6-oxo-4-(pyridine-3-sulfonyl)-hexahydro-pyrrolo[3,2-*b*]pyrrol-1-yl]-ethyl}-amide;

1004. Biphenyl-4-carboxylic acid {1-(4-methoxy-benzyl)-2-oxo-2-[6-oxo-4-(pyridine-3-sulfonyl)-hexahydro-pyrrolo[3,2-*b*]pyrrol-1-yl]-ethyl}-amide;

1005. Biphenyl-4-carboxylic acid {1-(3-hydroxy-benzyl)-2-oxo-2-[6-oxo-4-(pyridine-3-sulfonyl)-hexahydro-pyrrolo[3,2-*b*]pyrrol-1-yl]-ethyl}-amide;

1006. Furan-3-carboxylic acid {3,3-dimethyl-1-[6-oxo-4-(1-oxy-pyridine-2-sulfonyl)-hexahydro-pyrrolo[3,2-*b*]pyrrole-1-carbonyl]-butyl}-amide;

1007. Thiophene-3-carboxylic acid {3,3-dimethyl-1-[6-oxo-4-(1-oxy-pyridine-2-sulfonyl)-hexahydro-pyrrolo[3,2-*b*]pyrrole-1-carbonyl]-butyl}-amide;

1008. Benzo[b]thiophene-3-carboxylic acid {3,3-dimethyl-1-[6-oxo-4-(1-oxy-pyridine-2-sulfonyl)-hexahydro-pyrrolo[3,2-*b*]pyrrole-1-carbonyl]-butyl}-amide;

1009. Furan-3-carboxylic acid {1-cyclohexylmethyl-2-oxo-2-[6-oxo-4-(1-oxy-pyridine-2-sulfonyl)-hexahydro-pyrrolo[3,2-*b*]pyrrol-1-yl]-ethyl}-amide;

1010. Thiophene-3-carboxylic acid {1-cyclohexylmethyl-2-oxo-2-[6-oxo-4-(1-oxy-pyridine-2-sulfonyl)-hexahydro-pyrrolo[3,2-*b*]pyrrol-1-yl]-ethyl}-amide;

1011. Benzo[b]thiophene-3-carboxylic acid {1-cyclohexylmethyl-2-oxo-2-[6-oxo-4-(1-oxy-pyridine-2-sulfonyl)-hexahydro-pyrrolo[3,2-*b*]pyrrol-1-yl]-ethyl}-amide;

1012. Naphthalene-1-carboxylic acid {1-(4-hydroxy-benzyl)-2-oxo-2-[6-oxo-4-(1-oxy-pyridine-2-sulfonyl)-hexahydro-pyrrolo[3,2-*b*]pyrrol-1-yl]-ethyl}-amide;

1013. Quinoline-8-carboxylic acid {1-(4-hydroxy-benzyl)-2-oxo-2-[6-oxo-4-(1-oxy-pyridine-2-sulfonyl)-hexahydro-pyrrolo[3,2-*b*]pyrrol-1-yl]-ethyl}-amide;

1014. 4-*tert*-Butyl-N-{1-(4-hydroxy-benzyl)-2-oxo-2-[6-oxo-4-(1-oxy-pyridine-2-sulfonyl)-hexahydro-pyrrolo[3,2-*b*]-pyrrol-1-yl]-ethyl}-benzamide;

1015. 4-*tert*-Butyl-N-{1-(4-fluoro-benzyl)-2-oxo-2-[6-oxo-4-(1-oxy-pyridine-2-sulfonyl)-hexahydro-pyrrolo[3,2-*b*]-pyrrol-1-yl]-ethyl}-benzamide;

1016. 4-*tert*-Butyl-N-{1-(4-methoxy-benzyl)-2-oxo-2-[6-oxo-4-(1-oxy-pyridine-2-sulfonyl)-hexahydro-pyrrolo[3,2-*b*]-pyrrol-1-yl]-ethyl}-benzamide;

1017. 4-*tert*-Butyl-N-{1-(3-hydroxy-benzyl)-2-oxo-2-[6-oxo-4-(1-oxy-pyridine-2-sulfonyl)-hexahydro-pyrrolo[3,2-*b*]-pyrrol-1-yl]-ethyl}-benzamide;

1018. Biphenyl-4-carboxylic acid {1-(4-hydroxy-benzyl)-2-oxo-2-[6-oxo-4-(1-oxy-pyridine-2-sulfonyl)-hexahydro-pyrrolo[3,2-*b*]pyrrol-1-yl]-ethyl}-amide;

1019. Biphenyl-4-carboxylic acid {1-(4-fluoro-benzyl)-2-oxo-2-[6-oxo-4-(1-oxy-pyridine-2-sulfonyl)-hexahydro-pyrrolo[3,2-*b*]pyrrol-1-yl]-ethyl}-amide;

1020. Biphenyl-4-carboxylic acid {1-(4-methoxy-benzyl)-2-oxo-2-[6-oxo-4-(1-oxy-pyridine-2-sulfonyl)-hexahydro-pyrrolo[3,2-*b*]pyrrol-1-yl]-ethyl}-amide;

1021. Biphenyl-4-carboxylic acid {1-(3-hydroxy-benzyl)-2-oxo-2-[6-oxo-4-(1-oxy-pyridine-2-sulfonyl)-hexahydro-pyrrolo[3,2-*b*]pyrrol-1-yl]-ethyl}-amide;

1022. Furan-3-carboxylic acid {3,3-dimethyl-1-[6-oxo-4-(1-oxy-pyridine-3-sulfonyl)-hexahydro-pyrrolo[3,2-*b*]pyrrole-1-carbonyl]-butyl}-amide;

1023. Thiophene-3-carboxylic acid {3,3-dimethyl-1-[6-oxo-4-(1-oxy-pyridine-3-sulfonyl)-hexahydro-pyrrolo[3,2-*b*]pyrrole-1-carbonyl]-butyl}-amide;

1024. Benzo[b]thiophene-3-carboxylic acid {3,3-dimethyl-1-[6-oxo-4-(1-oxy-pyridine-3-sulfonyl)-hexahydro-pyrrolo[3,2-*b*]pyrrole-1-carbonyl]-butyl}-amide;

1025. Furan-3-carboxylic acid {1-cyclohexylmethyl-2-oxo-2-[6-oxo-4-(1-oxy-pyridine-3-sulfonyl)-hexahydro-pyrrolo[3,2-*b*]pyrrol-1-yl]-ethyl}-amide;

1026. Thiophene-3-carboxylic acid {1-cyclohexylmethyl-2-oxo-2-[6-oxo-4-(1-oxy-pyridine-3-sulfonyl)-hexahydro-pyrrolo[3,2-*b*]pyrrol-1-yl]-ethyl}-amide;

1027. Benzo[b]thiophene-3-carboxylic acid {1-cyclohexylmethyl-2-oxo-2-[6-oxo-4-(1-oxy-pyridine-3-sulfonyl)-hexahydro-pyrrolo[3,2-*b*]pyrrol-1-yl]-ethyl}-amide;

1028. Naphthalene-1-carboxylic acid {1-(4-hydroxy-benzyl)-2-oxo-2-[6-oxo-4-(1-oxy-pyridine-3-sulfonyl)-hexahydro-pyrrolo[3,2-*b*]pyrrol-1-yl]-ethyl}-amide;

1029. Quinoline-8-carboxylic acid {1-(4-hydroxy-benzyl)-2-oxo-2-[6-oxo-4-(1-oxy-pyridine-3-sulfonyl)-hexahydro-pyrrolo[3,2-*b*]pyrrol-1-yl]-ethyl}-amide;

1030. 4-*tert*-Butyl-N-{1-(4-hydroxy-benzyl)-2-oxo-2-[6-oxo-4-(1-oxy-pyridine-3-sulfonyl)-hexahydro-pyrrolo[3,2-*b*]-pyrrol-1-yl]-ethyl}-benzamide;

1031. 4-*tert*-Butyl-N-{1-(4-fluoro-benzyl)-2-oxo-2-[6-oxo-4-(1-oxy-pyridine-3-sulfonyl)-hexahydro-pyrrolo[3,2-*b*]pyrrol-1-yl]-ethyl}-benzamide;

1032. 4-*tert*-Butyl-N-{1-(4-methoxy-benzyl)-2-oxo-2-[6-oxo-4-(1-oxy-pyridine-3-sulfonyl)-hexahydro-pyrrolo[3,2-*b*]-pyrrol-1-yl]-ethyl}-benzamide;

1033. 4-*tert*-Butyl-N-{1-(3-hydroxy-benzyl)-2-oxo-2-[6-oxo-4-(1-oxy-pyridine-3-sulfonyl)-hexahydro-pyrrolo[3,2-*b*]-pyrrol-1-yl]-ethyl}-benzamide;

1034. Biphenyl-4-carboxylic acid {1-(4-hydroxy-benzyl)-2-oxo-2-[6-oxo-4-(1-oxy-pyridine-3-sulfonyl)-hexahydro-pyrrolo[3,2-*b*]pyrrol-1-yl]-ethyl}-amide;

1035. Biphenyl-4-carboxylic acid {1-(4-fluoro-benzyl)-2-oxo-2-[6-oxo-4-(1-oxy-pyridine-3-sulfonyl)-hexahydro-pyrrolo[3,2-*b*]pyrrol-1-yl]-ethyl}-amide;

1036. Biphenyl-4-carboxylic acid {1-(4-methoxy-benzyl)-2-oxo-2-[6-oxo-4-(1-oxy-pyridine-3-sulfonyl)-hexahydro-pyrrolo[3,2-*b*]pyrrol-1-yl]-ethyl}-amide;

1037. Biphenyl-4-carboxylic acid {1-(3-hydroxy-benzyl)-2-oxo-2-[6-oxo-4-(1-oxy-pyridine-3-sulfonyl)-hexahydro-pyrrolo[3,2-*b*]pyrrol-1-yl]-ethyl}-amide;

1038. Furan-3-carboxylic acid {3,3-dimethyl-1-[6-oxo-4-(pyridine-2-carbonyl)-hexahydro-pyrrolo[3,2-*b*]pyrrole-1-carbonyl]-butyl}-amide;

1039. Thiophene-3-carboxylic acid {3,3-dimethyl-1-[6-oxo-4-(pyridine-2-carbonyl)-hexahydro-pyrrolo[3,2-*b*]pyrrole-1-carbonyl]-butyl}-amide;

1040. Benzo[b]thiophene-3-carboxylic acid {3,3-dimethyl-1-[6-oxo-4-(pyridine-2-carbonyl)-hexahydro-pyrrolo[3,2-

*b*]pyrrole-1-carbonyl]-butyl}-amide;

1041. Furan-3-carboxylic acid {1-cyclohexylmethyl-2-oxo-2-[6-oxo-4-(pyridine-2-carbonyl)-hexahydro-pyrrolo[3,2-*b*]pyrrol-1-yl]-ethyl}-amide;

1042. Thiophene-3-carboxylic acid {1-cyclohexylmethyl-2-oxo-2-[6-oxo-4-(pyridine-2-carbonyl)-hexahydro-pyrrolo[3,2-*b*]pyrrol-1-yl]-ethyl}-amide;

1043. Benzo[b]thiophene-3-carboxylic acid {1-cyclohexylmethyl-2-oxo-2-[6-oxo-4-(pyridine-2-carbonyl)-hexahydro-pyrrolo[3,2-*b*]pyrrol-1-yl]-ethyl}-amide;

1044. Naphthalene-1-carboxylic acid {1-(4-hydroxy-benzyl)-2-oxo-2-[6-oxo-4-(pyridine-2-carbonyl)-hexahydro-pyrrolo[3,2-*b*]pyrrol-1-yl]-ethyl}-amide;

1045. Quinoline-8-carboxylic acid {1-(4-hydroxy-benzyl)-2-oxo-2-[6-oxo-4-(pyridine-2-carbonyl)-hexahydro-pyrrolo[3,2-*b*]pyrrol-1-yl]-ethyl}-amide;

1046. 4-*tert*-Butyl-N-{1-(4-hydroxy-benzyl)-2-oxo-2-[6-oxo-4-(pyridine-2-carbonyl)-hexahydro-pyrrolo[3,2-*b*]pyrrol-1-yl]-ethyl}-benzamide;

1047. 4-*tert*-Butyl-N-{1-(4-fluoro-benzyl)-2-oxo-2-[6-oxo-4-(pyridine-2-carbonyl)-hexahydro-pyrrolo[3,2-*b*]pyrrol-1-yl]-ethyl}-benzamide;

1048. 4-*tert*-Butyl-N-{1-(4-methoxy-benzyl)-2-oxo-2-[6-oxo-4-(pyridine-2-carbonyl)-hexahydro-pyrrolo[3,2-*b*]pyrrol-1-yl]-ethyl}-benzamide;

1049. 4-*tert*-Butyl-N-{1-(3-hydroxy-benzyl)-2-oxo-2-[6-oxo-4-(pyridine-2-carbonyl)-hexahydro-pyrrolo[3,2-*b*]pyrrol-1-yl]-ethyl}-benzamide;

1050. Biphenyl-4-carboxylic acid {1-(4-hydroxy-benzyl)-2-oxo-2-[6-oxo-4-(pyridine-2-carbonyl)-hexahydro-pyolo-[3,2-*b*]pyrrol-1-yl]-ethyl}-amide;

1051. Biphenyl-4-carboxylic acid {1-(4-fluoro-benzyl)-2-oxo-2-[6-oxo-4-(pyridine-2-carbonyl)-hexahydro-pyrrolo[3,2-*b*]pyrrol-1-yl]-ethyl}-amide;

1052. Biphenyl-4-carboxylic acid {1-(4-methoxy-benzyl)-2-oxo-2-[6-oxo-4-(pyridine-2-carbonyl)-hexahydro-pyrrolo[3,2-*b*]pyrrol-1-yl]-ethyl}-amide;

1053. Biphenyl-4-carboxylic acid {1-(3-hydroxy-benzyl)-2-oxo-2-[6-oxo-4-(pyridine-2-carbonyl)-hexahydro-pyrrolo[3,2-*b*]pyrrol-1-yl]-ethyl}-amide;

1054. Furan-3-carboxylic acid {3,3-dimethyl-1-[6-oxo-4-(2-pyridin-3-yl-acetyl)-hexahydro-pyrrolo[3,2-*b*]pyrrole-1-carbonyl]-butyl}-amide;

1055. Thiophene-3-carboxylic acid {3,3-dimethyl-1-[6-oxo-4-(2-pyridin-3-yl-acetyl)-hexahydro-pyrrolo[3,2-*b*]pyrrole-1-carbonyl]-butyl}-amide;

1056. Benzo[b]thiophene-3-carboxylic acid {3,3-dimethyl-1-[6-oxo-4-(2-pyridin-3-yl-acetyl)-hexahydro-pyrrolo[3,2-*b*]pyrrole-1-carbonyl]-butyl}-amide;

1057. Furan-3-carboxylic acid {1-cyclohexylmethyl-2-oxo-2-[6-oxo-4-(2-pyridin-3-yl-acetyl)-hexahydro-pyrrolo[3,2-*b*]pyrrol-1-yl]-ethyl}-amide;

1058. Thiophene-3-carboxylic acid {1-cyclohexylmethyl-2-oxo-2-[6-oxo-4-(2-pyridin-3-yl-acetyl)-hexahydro-pyrrolo[3,2-*b*]pyrrol-1-yl]-ethyl}-amide;

1059. Benzo[b]thiophene-3-carboxylic acid {1-cyclohexylmethyl-2-oxo-2-[6-oxo-4-(2-pyridin-3-yl-acetyl)-hexahydro-pyrrolo[3,2-*b*]pyrrol-1-yl]-ethyl}-amide;

1060. Naphthalene-1-carboxylic acid {1-(4-hydroxy-benzyl)-2-oxo-2-[6-oxo-4-(2-pyridin-3-yl-acetyl)-hexahydro-pyrrolo[3,2-*b*]pyrrol-1-yl]-ethyl}-amide;

1061. Quinoline-8-carboxylic acid {1-(4-hydroxy-benzyl)-2-oxo-2-[6-oxo-4-(2-pyridin-3-yl-acetyl)-hexahydro-pyrrolo[3,2-*b*]pyrrol-1-yl]-ethyl}-amide;

1062. 4-*tert*-Butyl-N-{1-(4-hydroxy-benzyl)-2-oxo-2-[6-oxo-4-(2-pyridin-3-yl-acetyl)-hexahydro-pyrrolo[3,2-*b*]pyrrol-1-yl]-ethyl}-benzamide;

1063. 4-*tert*-Butyl-N-{1-(4-fluoro-benzyl)-2-oxo-2-[6-oxo-4-(2-pyridin-3-yl-acetyl)-hexahydro-pyrrolo[3,2-*b*]pyrrol-1-yl]-ethyl}-benzamide;

1064. 4-*tert*-Butyl-N-{1-(4-methoxy-benzyl)-2-oxo-2-[6-oxo-4-(2-pyridin-3-yl-acetyl)-hexahydro-pyrrolo[3,2-*b*]pyrrol-1-yl]-ethyl}-benzamide;

1065. 4-*tert*-Butyl-N-{1-(3-hydroxy-benzyl)-2-oxo-2-[6-oxo-4-(2-pyridin-3-yl-acetyl)-hexahydro-pyrrolo[3,2-*b*]pyrrol-1-yl]-ethyl}-benzamide;

1066. Biphenyl-4-carboxylic acid {1-(4-hydroxy-benzyl)-2-oxo-2-[6-oxo-4-(2-pyridin-3-yl-acetyl)-hexahydro-pyrrolo[3,2-*b*]pyrrol-1-yl]-ethyl}-amide;

1067. Biphenyl-4-carboxylic acid {1-(4-fluoro-benzyl)-2-oxo-2-[6-oxo-4-(2-pyridin-3-yl-acetyl)-hexahydro-pyrrolo[3,2-*b*]pyrrol-1-yl]-ethyl}-amide;

1068. Biphenyl-4-carboxylic acid {1-(4-methoxy-benzyl)-2-oxo-2-[6-oxo-(2-pyridin-3-yl-acetyl)-hexahydro-pyrrolo[3,2-*b*]pyrrol-1-yl]-ethyl}-amide;

1069. Biphenyl-4-carboxylic acid {1-(3-hydroxy-benzyl)-2-oxo-2-[6-oxo-4-(2-pyridin-3-yl-acetyl)-hexahydro-pyrrolo[3,2-*b*]pyrrol-1-yl]-ethyl}-amide;

1070. Furan-3-carboxylic acid {3,3-dimethyl-1-[6-oxo-4-(pyridin-2-ylmethanesulfonyl)-hexahydro-pyrrolo[3,2- *b*]pyrrole-1-carbonyl]-butyl}-amide;

1071. Thiophene-3-carboxylic acid {3,3-dimethyl-1-[6-oxo-4-(pyridin-2-ylmethanesulfonyl)-hexahydro-pyrrolo[3,2-*b*]pyrrole-1-carbonyl]-butyl}-amide;

1072. Benzo[b]thiophene-3-carboxylic acid {3,3-dimethyl-1-[6-oxo-4-(pyridin-2-ylmethanesulfonyl)-hexahydro-pyrrolo[3,2-*b*]pyrrole-1-carbonyl]-butyl}-amide;

1073. Furan-3-carboxylic acid {1-cyclohexylmethyl-2-oxo-2-[6-oxo-4-(pyridin-2-ylmethanesulfonyl)-hexahydro-pyrrolo[3,2-*b*]pyrrol-1-yl]-ethyl}-amide;

1074. Thiophene-3-carboxylic acid {1-cyclohexylmethyl-2-oxo-2-[6-oxo-4-(pyridin-2-ylmethanesulfonyl)-hexahydro-pyrrolo[3,2-*b*]pyrrol-1-yl]-ethyl}-amide;

1075. Benzo[b]thiophene-3-carboxylic acid {1-cyclohexylmethyl-2-oxo-2-[6-oxo-4-(pyridin-2-ylmethanesulfonyl)-hexahydro-pyrrolo[3,2-*b*]pyrrol-1-yl]-ethyl}-amide;

1076. Naphthalene-1-carboxylic acid {1-(4-hydroxy-benzyl)-2-oxo-2-[6-oxo-4-(pyridin-2-ylmethanesulfonyl)-hexahydro-pyrrolo[3,2-*b*]pyrrol-1-yl]-ethyl}-amide;

1077. Quinoline-8-carboxylic acid {1-(4-hydroxy-benzyl)-2-oxo-2-[6-oxo-4-(pyridin-2-ylmethanesulfonyl)-hexahydro-pyrrolo[3,2-*b*]pyrrol-1-yl]-ethyl}-amide;

1078. 4-*tert*-Butyl-N-{1-(4-hydroxy-benzyl)-2-oxo-2-[6-oxo-4-(pyridin-2-ylmethanesulfonyl)-hexahydro-pyrrolo[3,2-*b*]pyrrol-1-yl]-ethyl}-benzamide;

1079. 4-*tert*-Butyl-N-{1-(4-fluoro-benzyl)-2-oxo-2-[6-oxo-4-(pyridin-2-ylmethanesulfonyl)-hexahydro-pyrrolo[3,2-*b*]-

pyrrol-1-yl]-ethyl}-benzamide;

1080. 4-*tert*-Butyl-N-{1-(4-methoxy-benzyl)-2-oxo-2-[6-oxo-4-(pyridin-2-ylmethanesulfonyl)-hexahydro-pyrrolo[3,2-*b*]pyrrol-1-yl]-ethyl}-benzamide;

1081. 4-*tert*-Butyl-N-{1-(3-hydroxy-benzyl)-2-oxo-2-[6-oxo-4-(pyridin-2-ylmethanesulfonyl)-hexahydro-pyrrolo[3,2-*b*]pyrrol-1-yl]-ethyl}-benzamide;

1082. Biphenyl-4-carboxylic acid {1-(4-hydroxy-benzyl)-2-oxo-2-[6-oxo-4-(pyridin-2-ylmethanesulfonyl)-hexahydro-pyrrolo[3,2-*b*]pyrrol-1-yl]-ethyl}-amide;

1083. Biphenyl-4-carboxylic acid {1-(4-fluoro-benzyl)-2-oxo-2-[6-oxo-4-(pyridin-2-ylmethanesulfonyl)-hexahydro-pyrrolo[3,2-*b*]pyrrol-1-yl]-ethyl}-amide;

1084. Biphenyl-4-carboxylic acid {1-(4-methoxy-benzyl)-2-oxo-2-[6-oxo-4-(pyridin-2-ylmethanesulfonyl)-hexahydro-pyrrolo[3,2-*b*]pyrrol-1-yl]-ethyl}-amide;

1085. Biphenyl-4-carboxylic acid {1-(3-hydroxy-benzyl)-2-oxo-2-[6-oxo-4-(pyridin-2-ylmethanesulfonyl)-hexahydro-pyrrolo[3,2-*b*]pyrrol-1-yl]-ethyl}-amide;

1086. Furan-3-carboxylic acid {3,3-dimethyl-1-[6-oxo-4-(pyridin-3-ylmethanesulfonyl)-hexahydro-pyrrolo[3,2-*b*]pyrrole-1-carbonyl]-butyl}-amide;

1087. Thiophene-3-carboxylic acid {3,3-dimethyl-1-[6-oxo-4-(pyridin-3-ylmethanesulfonyl)-hexahydro-pyrrolo[3,2-*b*]pyrrole-1-carbonyl]-butyl}-amide;

1088. Benzo[b]thiophene-3-carboxylic acid {3,3-dimethyl-1-[6-oxo-4-(pyridin-3-ylmethanesulfonyl)-hexahydro-pyrrolo[3,2-*b*]pyrrole-1-carbonyl]-butyl}-amide;

1089. Furan-3-carboxylic acid {1-cyclohexylmethyl-2-oxo-2-[6-oxo-4-(pyridin-3-ylmethanesulfonyl)-hexahydro-pyrrolo[3,2-*b*]pyrrol-1-yl]-ethyl}-amide;

1090. Thiophene-3-carboxylic acid {1-cyclohexylmethyl-2-oxo-2-[6-oxo-4-(pyridin-3-ylmethanesulfonyl)-hexahydro-pyrrolo[3,2-*b*]pyrrol-1-yl]-ethyl}-amide;

1091. Benzo[b]thiophene-3-carboxylic acid {1-cyclohexylmethyl-2-oxo-2-[6-oxo-4-(pyridin-3-ylmethanesulfonyl)-hexahydro-pyrrolo[3,2-*b*]pyrrol-1-yl]-ethyl}-amide;

1092. Naphthalene-1-carboxylic acid {1-(4-hydroxy-benzyl)-2-oxo-2-[6-oxo-4-(pyridin-3-ylmethanesulfonyl)-hexahydro-pyrrolo[3,2-*b*]pyrrol-1-yl]-ethyl}-amide;

1093. Quinoline-8-carboxylic acid {1-(4-hydroxy-benzyl)-2-oxo-2-[6-oxo-4-(pyridin-3-ylmethanesulfonyl)-hexahydro-pyrrolo[3,2-*b*]pyrrol-1-yl]-ethyl}-amide;

1094. 4-*tert*-Butyl-N-{1-(4-hydroxy-benzyl)-2-oxo-2-[6-oxo-4-(pyridin-3-ylmethanesulfonyl)-hexahydro-pyrrolo[3,2-*b*]pyrrol-1-yl]-ethyl}-benzamide;

1095. 4-*tert*-Butyl-N-{1-(4-fluoro-benzyl)-2-oxo-2-[6-oxo-4-(pyridin-3-ylmethanesulfonyl)-hexahydro-pyrrolo[3,2-*b*]pyrrol-1-yl]-ethyl}-benzamide;

1096. 4-*tert*-Butyl-N-{1-(4-methoxy-benzyl)-2-oxo-2-[6-oxo-4-(pyridin-3-ylmethanesulfonyl)-hexahydro-pyrrolo[3,2-*b*]pyrrol-1-yl]-ethyl}-benzamide;

1097. 4-*tert*-Butyl-N-{1-(3-hydroxy-benzyl)-2-oxo-2-[6-oxo-4-(pyridin-3-ylmethanesulfonyl)-hexahydro-pyrrolo[3,2-*b*]pyrrol-1-yl]-ethyl}-benzamide;

1098. Biphenyl-4-carboxylic acid {1-(4-hydroxy-benzyl)-2-oxo-2-[6-oxo-4-(pyridin-3-ylmethanesulfonyl)-hexahydro-pyrrolo[3,2-*b*]pyrrol-1-yl]-ethyl}-amide;

1099. Biphenyl-4-carboxylic acid {1-(4-fluoro-benzyl)-2-oxo-2-[6-oxo-4-(pyridin-3-ylmethanesulfonyl)-hexahydro-pyrrolo[3,2-*b*]pyrrol-1-yl]-ethyl}-amide;

1100. Biphenyl-4-carboxylic acid {1-(4-methoxy-benzyl)-2-oxo-2-[6-oxo-4-(pyridin-3-ylmethanesulfonyl)-hexahydro-pyrrolo[3,2-*b*]pyrrol-1-yl]-ethyl}-amide;

1101. Biphenyl-4-carboxylic acid {1-(3-hydroxy-benzyl)-2-oxo-2-[6-oxo-4-(pyridin-3-ylmethanesulfonyl)-hexahydro-pyrrolo[3,2-*b*]pyrrol-1-yl]-ethyl}-amide;

1102. Furan-3-carboxylic acid {3,3-dimethyl-1-[6-oxo-4-(1-oxy-pyridin-2-ylmethanesulfonyl)-hexahydro-pyrrolo[3,2-*b*]pyrrole-1-carbonyl]-butyl}-amide;

1103. Thiophene-3-carboxylic acid {3,3-dimethyl-1-[6-oxo-4-(1-oxy-pyridin-2-ylmethanesulfonyl)-hexahydro-pyrrolo[3,2-*b*]pyrrole-1-carbonyl]-butyl}-amide;

1104. Benzo[b]thiophene-3-carboxylic acid {3,3-dimethyl-1-[6-oxo-4-(1-oxy-pyridin-2-ylmethanesulfonyl)-hexahydro-pyrrolo[3,2-*b*]pyrrole-1-carbonyl]-butyl}-amide;

1105. Furan-3-carboxylic acid {1-cyclohexylmethyl-2-oxo-2-[6-oxo-4-(1-oxy-pyridin-2-ylmethanesulfonyl)-hexahydro-pyrrolo[3,2-*b*]pyrrol-1-yl]-ethyl}-amide;

1106. Thiophene-3-carboxylic acid {1-cyclohexylmethyl-2-oxo-2-[6-oxo-4-(1-oxy-pyridin-2-ylmethanesulfonyl)-hexahydro-pyrrolo[3,2-*b*]pyrrol-1-yl]-ethyl}-amide;

1107. Benzo[b]thiophene-3-carboxylic acid {1-cyclohexylmethyl-2-oxo-2-[6-oxo-4-(1-oxy-pyridin-2-ylmethanesulfonyl)-hexahydro-pyrrolo[3,2-*b*]pyrrol-1-yl]-ethyl}-amide;

1108. Naphthalene-1-carboxylic acid {1-(4-hydroxy-benzyl)-2-oxo-2-[6-oxo-4-(1-oxy-pyridin-2-ylmethanesulfonyl)-hexahydro-pyrrolo[3,2-*b*]pyrrol-1-yl]-ethyl}-amide;

1109. Quinoline-8-carboxylic acid {1-(4-hydroxy-benzyl)-2-oxo-2-[6-oxo-4-(1-oxy-pyridin-2-ylmethanesulfonyl)-hexahydro-pyrrolo[3,2-*b*]pyrrol-1-yl]-ethyl}-amide;

1110. 4-*tert*-Butyl-N-{1-(4-hydroxy-benzyl)-2-oxo-2-[6-oxo-4-(1-oxy-pyridin-2-ylmethanesulfonyl)-hexahydro-pyrrolo[3,2-*b*]pyrrol-1-yl]-ethyl}-benzamide;

1111. 4-*tert*-Butyl-N-{1-(4-fluoro-benzyl)-2-oxo-2-[6-oxo-4-(1-oxy-pyridin-2-ylmethanesulfonyl)-hexahydro-pyrrolo-[3,2-*b*]pyrrol-1-yl]-ethyl}-benzamide;

1112. 4-*tert*-Butyl-N-{1-(4-methoxy-benzyl)-2-oxo-2-[6-oxo-4-(1-oxy-pyridin-2-ylmethanesulfonyl)-hexahydro-pyrrolo[3,2-*b*]pyrrol-1-yl]-ethyl}-benzamide;

1113. 4-*tert*-Butyl-N-{1-(3-hydroxy-benzyl)-2-oxo-2-[6-oxo-4-(1-oxy-pyridin-2-ylmethanesulfonyl)-hexahydro-pyrrolo[3,2-*b*]pyrrol-1-yl]-ethyl}-benzamide;

1114. Biphenyl-4-carboxylic acid {1-(4-hydroxy-benzyl)-2-oxo-2-[6-oxo-4-(1-oxy-pyridin-2-ylmethanesulfonyl)-hexahydro-pyrrolo[3,2-*b*]pyrrol-1-yl]-ethyl}-amide;

1115. Biphenyl-4-carboxylic acid {1-(4-fluoro-benzyl)-2-oxo-2-[6-oxo-4-(1-oxy-pyridin-2-ylmethanesulfonyl)-hexahydro-pyrrolo[3,2-*b*]pyrrol-1-yl]-ethyl}-amide;

1116. Biphenyl-4-carboxylic acid {1-(4-methoxy-benzyl)-2-oxo-2-[6-oxo-4-(1-oxy-pyridin-2-ylmethanesulfonyl)-hexahydro-pyrrolo[3,2-*b*]pyrrol-1-yl]-ethyl}-amide;

1117. Biphenyl-4-carboxylic acid {1-(3-hydroxy-benzyl)-2-oxo-2-[6-oxo-4-(1-oxy-pyridin-2-ylmethanesulfonyl)-hexahydro-pyrrolo[3,2-*b*]pyrrol-1-yl]-ethyl}-amide;

1118. Furan-3-carboxylic acid {3,3-dimethyl-1-[6-oxo-4-(1-oxy-pyridin-3-ylmethanesulfonyl)-hexahydro-pyrrolo[3,2-*b*]pyrrole-1-carbonyl]-butyl}-amide;

1119. Thiophene-3-carboxylic acid {3,3-dimethyl-1-[6-oxo-4-(1-oxy-pyridin-3-ylmethanesulfonyl)-hexahydro-pyrrolo[3,2-*b*]pyrrole-1-carbonyl]-butyl}-amide;

1120. Benzo[b]thiophene-3-carboxylic acid {3,3-dimethyl-1-[6-oxo-4-(1-oxy-pyridin-3-ylmethanesulfonyl)-hexahydro-pyrrolo[3,2-*b*]pyrrole-1-carbonyl]-butyl}-amide;

1121. Furan-3-carboxylic acid {1-cyclohexylmethyl-2-oxo-2-[6-oxo-4-(1-oxy-pyridin-3-ylmethanesulfonyl)-hexahydro-pyrrolo[3,2-*b*]pyrrol-1-yl]-ethyl}-amide;

1122. Thiophene-3-carboxylic acid {1-cyclohexylmethyl-2-oxo-2-[6-oxo-4-(1-oxy-pyrridin-3-ylmethanesulfonyl)-hexahydro-pyrrolo[3,2-*b*]pyrrol-1-yl]-ethyl}-amide;

1123. Benzo[b]thiophene-3-carboxylic acid {1-cyclohexylmethyl-2-oxo-2-[6-oxo-4-(1-oxy-pyridin-3-ylmethanesulfonyl)-hexahydro-pyrrolo[3,2-*b*]pyrrol-1-yl]-ethyl}-amide;

1124. Naphthalene-1-carboxylic acid {1-(4-hydroxy-benzyl)-2-oxo-2-[6-oxo-4-(1-oxy-pyridin-3-ylmethanesulfonyl)-hexahydro-pyrrolo[3,2-*b*]pyrrol-1-yl]-ethyl}-amide;

1125. Quinoline-8-carboxylic acid {1-(4-hydroxy-benzyl)-2-oxo-2-[6-oxo-4-(1-oxy-pyridin-3-ylmethanesulfonyl)-hexahydro-pyrrolo[3,2-*b*]pyrrol-1-yl]-ethyl}-amide;

1126. 4-*tert*-Butyl-N-{1-(4-hydroxy-benzyl)-2-oxo-2-[6-oxo-4-(1-oxy-pyridin-3-ylmethanesulfonyl)-hexahydro-pyrrolo[3,2-*b*]pyrrol-1-yl]-ethyl}-benzamide;

1127. 4-*tert*-Butyl-N-{1-(4-fluoro-benzyl)-2-oxo-2-[6-oxo-4-(1-oxy-pyridin-3-ylmethanesulfonyl)-hexahydro-pyrrolo[3,2-*b*]pyrrol-1-yl]-ethyl}-benzamide;

1128. 4-*tert*-Butyl-N-{1-(4-methoxy-benzyl)-2-oxo-2-[6-oxo-4-(1-oxy-pyridin-3-ylmethanesulfonyl)-hexahydro-pyrrolo[3,2-*b*]pyrrol-1-yl]-ethyl}-benzamide;

1129. 4-*tert*-Butyl-N-{1-(3-hydroxy-benzyl)-2-oxo-2-[6-oxo-4-(1-oxy-pyridin-3-ylmethanesulfonyl)-hexahydro-pyrrolo[3,2-*b*]pyrrol-1-yl]-ethyl-benzamide;

1130. Biphenyl-4-carboxylic acid {1-(4-hydroxy-benzyl)-2-oxo-2-[6-oxo-4-(1-oxy-pyridin-3-ylmethanesulfonyl)-hexahydro-pyrrolo[3,2-*b*]pyrrol-1-yl]-ethyl}-amide;

1131. Biphenyl-4-carboxylic acid {1-(4-fluoro-benzyl)-2-oxo-2-[6-oxo-4-(1-oxy-pyridin-3-ylmethanesulfonyl)-hexahydro-pyrrolo[3,2-*b*]pyrrol-1-yl]-ethyl}-amide;

1132. Biphenyl-4-carboxylic acid {1-(4-methoxy-benzyl)-2-oxo-2-[6-oxo-4-(1-oxy-pyridin-3-ylmethanesulfonyl)-hexahydro-pyrrolo[3,2-*b*]pyrrol-1-yl]-ethyl}-amide;

1133. Biphenyl-4-carboxylic acid {1-(3-hydroxy-benzyl)-2-oxo-2-[6-oxo-4-(1-oxy-pyridin-3-ylmethanesulfonyl)-hexahydro-pyrrolo[3,2-*b*]pyrrol-1-yl]-ethyl}-amide;

1134. 2-Cyclohexylmethyl-4-morpholin-4-yl-1-[6-oxo-4-(pyridine-2-sulfonyl)-hexahydro-pyrrolo[3,2-*b*]pyrrol-1-yl]-butane-1,4-dione;

1135. 2-Cyclohexylmethyl-4-morpholin-4-yl-1-[6-oxo-4-(pyridine-3-sulfonyl)-hexahydro-pyrrolo[3,2-*b*]pyrrol-1-yl]-butane-1,4-dione;

1136. 2-Cyclohexylmethyl-4-morpholin-4-yl-1-[6-oxo-4-(1-oxy-pyridine-2-sulfonyl)-hexahydro-pyrrolo[3,2-*b*]pyrrol-1-yl]-butane-1,4-dione;

1137. 2-Cyclohexylmethyl-4-morpholin-4-yl-1-[6-oxo-4-(1-oxy-pyridine-3-sulfonyl)-hexahydro-pyrrolo[3,2-*b*]pyrrol-1-yl]-butane-1,4-dione;

1138. 2-Cyclohexylmethyl-4-morpholin-4-yl-1-[6-oxo-4-(pyridine-2-carbonyl)-hexahydro-pyrrolo[3,2-*b*]pyrrol-1-yl]-butane-1,4-dione;

1139. 2-Cyclohexylmethyl-4-morpholin-4-yl-1-[6-oxo-4-(2-pyridin-3-yl-acetyl)-hexahydro-pyrrolo[3,2-*b*]pyrrol-1-yl]-butane-1,4-dione;

1140. 2-Cyclohexylmethyl-4-morpholin-4-yl-1-[6-oxo-4-(pyridin-2-ylmethanesulfonyl)-hexahydro-pyrrolo[3,2-*b*]pyrrol-1-yl]-butane-1,4-dione;

1141. 2-Cyclohexylmethyl-4-morpholin-4-yl-1-[6-oxo-4-(pyridin-3-ylmethanesulfonyl)-hexahydro-pyrrolo[3,2-*b*]pyrrol-1-yl]-butane-1,4-dione;

1142. 2-Cylohexylmethyl-4-morpholin-4-yl-1-[6-oxo-4-(1-oxy-pyridin-2-ylmethanesulfonyl)-hexahydro-pyrrolo[3,2-*b*]pynol-1-yl]-butane-1,4-dione;

1143. 2-Cyclohexylmethyl-4-morpholin-4-yl-1-[6-oxo-4-(1-oxy-pyridin-3-ylmethanesulfonyl)-hexahydro-pyrrolo[3,2-*b*]pynol-1-yl]-butane-1,4-dione;

1144. 2-(2,2-Dimethyl-propyl)-4-morpholin-4-yl-1-[6-oxo-4-(pyridine-2-sulfonyl)-hexahydro-pyrrolo[3,2-*b*]pyrrol-1-yl]-butane-1,4-dione;

. 1145. 2-(2,2-Dimethyl-propyl)-4-morpholin-4-yl-1-[6-oxo-4-(pyridine-3-sulfonyl)-hexahydro-pyrrolo[3,2-*b*]pyrrol-1-yl]-butane-1,4-dione;

1146. 2-(2,2-Dimethyl-propyl)-4-morpholin-4-yl-1-[6-oxo-4-(1-oxy-pyridine-2-sulfonyl)-hexahydro-pyrrolo[3,2-*b*]-pyrrol-1-yl]-butane-1,4-dione;

. 1147. 2-(2,2-Dimethyl-propyl)-4-morpholin-4-yl-1-[6-oxo-4-(1-oxy-pyridine-3-sulfonyl)-hexahydro-pyrrolo[3,2-*b*]-pyrrol-1-yl]-butane-1,4-dione;

1148. 2-(2,2-Dimethyl-propyl)-4-morpholin-4-yl-1-[6-oxo-4-(pyridine-2-carbonyl)-hexahydro-pyrrolo[3,2-*b*]pyrrol-1-yl]-butane-1,4-dione;

1149. 2-(2,2-Dimethyl-propyl)-4-morpholin-4-yl-1-[6-oxo-4-(2-pyridin-3-yl-acetyl)-hexahydro-pyrrolo[3,2-*b*]pyrrol-1-yl]-butane-1,4-dione;

1150. 2-(2,2-Dimethyl-propyl)-4-morpholin-4-yl-1-[6-oxo-4-(pyridin-2-ylmethanesulfonyl)-hexahydro-pyrrolo[3,2-*b*]-pyrrol-1-yl]-butane-1,4-dione;

1151. 2-(2,2-Dimethyl-propyl)-4-morpholin-4-yl-1-[6-oxo-4-(pyridin-3-ylmethanesulfonyl)-hexahydro-pyrrolo[3,2-*b*]-pyrrol-1-yl]-butane-1,4-dione;

1152. 2-(2,2-Dimethyl-propyl)-4-morpholin-4-yl-1-[6-oxo-4-(1-oxy-pyridin-2-ylmethanesulfonyl)-hexahydro-pyrrolo-[3,2-*b*]pyrrol-1-yl]-butane-1,4-dione;

1153. 2-(2,2-Dimethyl-propyl)-4-morpholin-4-yl-1-[6-oxo-4-(1-oxy-pyridin-3-ylmethanesulfonyl)-hexahydro-pyrrolo[3,2-*b*] pyrrol-1-yl]-butane-1,4-dione.

Additional compounds of the invention include, but are not limited to, the following examples where all 4 stereoisomeric combinations of the bicyclic ketone are included where $P_2$ is $CH_2$, i.e. (3a*S*, 6a*S*), (3a*R*, 6a*S*), (3a*S*, 6a*R*), (3a*R*, 6a*R*) and also included are the equivalent analogues where $P_2$ is O and NH. More preferred compounds consist of the cis-bicyclic isomers which, when $P_2$ is $CH_2$, are designated as (3a*R*, 6a*S*) and (3a*S*, 6a*R*) and also more preferred are the equivalent cis-bicyclic analogues where $P_2$ is O and NH.

1154. *N*-1-(4-Benzoyl-6-oxo-hexahydro-pyrrolo[3,2-*b*]pyrrole-1-carbonyl)-3-methyl-butyl]-4-*tert*-butylbenzamide;

1155. 4-*tert*-Butyl-*N*-{3-methyl-1-[6-oxo-4-(1-oxy-pyridine-2-carbonyl)-hexahydro-pyrrolo[3,2-*b*]pyrrole-1-carbonyl]-butyl}-benzamide;

1156. 4-*tert*-Butyl-*N*-{3-methyl-1-[6-oxo-4-(pyridine-3-carbonyl)-hexahydro-pyrrolo[3,2-*b*]pyrrole-1-carbonyl]-butyl}-benzamide;

1157. 4-*tert*-Butyl-*N*-{3-methyl-1-[6-oxo-4-(1-oxy-pyridine-3-carbonyl)-hexahydro-pyrrolo[3,2-*b*]pyrrole-1-carbonyl]-butyl}-benzamide;

1158. 4-*tert*-Butyl-*N*-{3-methyl-1-[6-oxo-4-(pyridine-4-carbonyl)-hexahydro-pyrrolo[3,2-*b*]pyrrole-1-carbonyl]-butyl}-benzamide;

1159. 4-*tert*-Butyl-*N*-{3-methyl-1-[6-oxo-4-(1-oxy-pyridine-4-carbonyl)-hexahydro-pyrrolo[3,2-*b*]pyrrole-1-carbonyl]-butyl}-benzamide;

1160. *N*-[1-(4-Benzenesulfonyl-6-oxo-hexahydro-pyrrolo[3,2-*b*]pyrrole-1-carbonyl)-3-methyl-butyl]-4-*tert*-butyl-benzamide;

1161. 4-*tert*-Butyl-*N*-{3-methyl-1-[6-oxo-4-(pyridine-4-sulfonyl)-hexahydro-pyrrolo[3,2-*b*]pyrrole-1-carbonyl]-butyl}-benzamide;

1162. 4-*tert*-Butyl-*N*-{3-methyl-1-[6-oxo-4-(1-oxy-pyridine-4-sulfonyl)-hexahydro-pyrrolo[3,2-*b*]pyrrole-1-carbonyl]-butyl}-benzamide;

1163. 4-*tert*-Butyl-*N*-[3-methyl-1-(6-oxo-4-phenylacetyl-hexahydro-pyrrolo[3,2-*b*]pyrrole-1-carbonyl)-butyl]-benzamide;

1164. 4-[2-(4-*tert*-Butyl-benzoylamino)-4-methyl-pentanoyl]-3-oxo-hexahydro-pyrrolo[3,2-*b*]pyrrole-1-carboxylic acid phenylamide;

1165. 4-[2-(4-*tert*-Butyl-benzoylamino)-4-methyl-pentanoyl]-3-oxo-hexahydro-pyrrolo[3,2-*b*]pyrrole-1-carboxylic acid isobutyl-amide;

1166. 4-[2-(4-*tert*-Butyl-benzoylamino)-4-methyl-pentanoyl]-3-oxo-hexahydro-pyrrolo[3,2-*b*]pyrrole-1-carboxylic acid phenyl ester;

1167. 4-[2-(4-*tert*-Butyl-benzoylamino)-4-methyl-pentanoyl]-3-oxo-hexahydro-pyrrolo[3,2-*b*]pyrrole-1-carboxylic acid isobutyl ester;

1168. 4-*tert*-Butyl-*N*-{3-methyl-1-[6-oxo-4-(pyrrolidine-1-carbonyl)-hexahydro-pyrrolo[3,2-*b*]pyrrole-1-carbonyl]-butyl}-benzamide;

1169. 4-*tert*-Butyl-*N*-{3-methyl-1-[6-oxo-4-(piperidine-1-carbonyl)-hexahydro-pyrrolo[3,2-*b*]pyrrole-1-carbonyl]-butyl}-benzamide;

1170. 4-*tert*-Butyl-*N*-{3-methyl-1-[4-(morpholine-4-carbonyl)-6-oxo-hexahydro-pyrrolo[3,2-*b*]pyrrole-1-carbonyl]-butyl}-benzamide;

1171. 4-*tert*-Butyl-*N*-{3-methyl-1-[6-oxo-4-(piperazine-1-carbonyl)-hexahydro-pyrrolo[3,2-*b*]pyrrole-1-carbonyl]-butyl}-benzamide;

1172. 4-*tert*-Butyl-*N*-{3-methyl-1-[4-(4-methyl-piperazine-1-carbonyl)-6-oxo-hexahydro-pyrrolo[3,2-*b*]pyrrole-1-carbonyl]-butyl}-benzamide;

1173. 4-*tert*-Butyl-*N*-(3-methyl-1-{4-[2-(4-methyl-piperazin-1-yl)-acetyl]-6-oxo-hexahydro-pyrrolo[3,2-*b*]pyrrole-1-carbonyl}-butyl)-benzamide;

1174. 4-*tert*-Butyl-*N*-{3-methyl-1-[6-oxo-4-(2-piperazin-1-yl-acetyl)-hexahydro-pyrrolo[3,2-*b*]pyrrole-1-carbonyl]-butyl}-benzamide;

1175. 4-*tert*-Butyl-*N*{1-[4-(furan-2-carbonyl)-6-oxo-hexahydro-pyrrolo[3,2-*b*]pyrrole-1-carbonyl]-3-methyl-butyl}-benzamide;

1176. 4-*tert*-Butyl-*N*-{1-[4-(5-chloro-furan-2-carbonyl)-6-oxo-hexahydro-pyrrolo[3,2-*b*]pyrrole-1-carbonyl]-3-methyl-butyl}-benzamide;

1177. 4-*tert*-Butyl-*N*-{1-[4-(thiophene-2-carbonyl)-6-oxo-hexahydro-pyrrolo[3,2-*b*]pyrrole-1-carbonyl]-3-methyl-butyl}-benzamide;

1178. 4-*tert*-Butyl-*N*-{1-[4-(5-chloro-thiophene-2-carbonyl)-6-oxo-hexahydro-pyrrolo[3,2-*b*]pyrrole-1-carbonyl]-3-methyl-butyl}-benzamide;

1179. 4-*tert*-Butyl-*N*-{1-[4-(furan-3-carbonyl)-6-oxo-hexahydro-pyrrolo[3,2-*b*]pyrrole-1-carbonyl]-3-methyl-butyl}-benzamide;

1180. 4-*tert*-Butyl-*N*-{1-[4-(thiophene-3-carbonyl)-6-oxo-hexahydro-pyrrolo[3,2-*b*]pyrrole-1-carbonyl]-3-methyl-butyl}-benzamide;

1181. 4-*tert*-Butyl-*N*-[1-(4-cyclopentanecarbonyl-6-oxo-hexahydro-pyrrolo[3,2-*b*]pyrrole-1-carbonyl)-3-methyl-butyl]-benzamide;

1182. 4-*tert*-Butyl-*N*-[1-(4-cyclohexanecarbonyl-6-oxo-hexahydro-pyrrolo[3,2-*b*]pyrrole-1-carbonyl)-3-methyl-butyl]-benzamide;

1183. 4-*tert*-Butyl-*N*-{1-[4-(cyclopent-3-enecarbonyl)-6-oxo-hexahydro-pyrrolo[3,2-*b*]pyrrole-1-carbonyl]-3-methyl-butyl}-benzamide;

1184. 4-*tert*-Butyl-*N*-{1-[4-(2-cyclopentyl-acetyl)-6-oxo-hexahydro-pyrrolo[3,2-*b*]pyrrole-1-carbonyl]-3-methyl-butyl}-benzamide;

1185. 4-*tert*-Butyl-*N*-{1-[4-(2-cyclohexyl-acetyl)-6-oxo-hexahydro-pyrrolo[3,2-*b*]pyrrole-1-carbonyl]-3-methyl-butyl}-benzamide;

1186. 4-*tert*-Butyl-*N*-{3-methyl-1-[6-oxo-4-(pyrrolidine-2-carbonyl)-hexahydro-pyrrolo[3,2-*b*]pyrrole-1-carbonyl]-butyl}-benzamide;

1187. *N*-{1-[4-(1-Acetyl-pyrrolidine-2-carbonyl)-6-oxo-hexahydro-pyrrolo[3,2-*b*]pyrrole-1-carbonyl]-3-methyl-butyl}-4-*tert*-butyl-benzamide;

1188. 4-*tert*-Butyl-*N*-{3-methyl-1-[6-oxo-4-(piperidine-2-carbonyl)-hexahydro-pyrrolo[3,2-*b*]pyrrole-1-carbonyl]-butyl}-benzamide;

1189. *N*-{1-[4-(1-Acetyl-piperidine-2-carbonyl)-6-oxo-hexahydro-pyrrolo[3,2-*b*]pyrrole-1-carbonyl]-3-methyl-butyl}-4-*tert*-butyl-benzamide;

1190. 4-*tert*-Butyl-*N*-{3-methyl-1-[6-oxo-4-(tetrahydro-furan-2-carbonyl)-hexahydro-pyrrolo[3,2-*b*]pyrrole-1-carbonyl]-butyl}-benzamide;

1191. *N*-{1-[4-(1-Amino-cyclobutanecarbonyl)-6-oxo-hexahydro-pyrrolo[3,2-*b*]pyrrole-1-carbonyl]-3-methyl-butyl}-4-*tert*-butyl-benzamide;

1192. *N*-{1-[4-(1-Amino-cyclopentanecarbonyl)-6-oxo-hexahydro-pyrrolo[3,2-*b*]pyrrole-1-carbonyl]-3-methyl-butyl}-4-*tert*-butyl-benzamide;

1193. *N*-{1-[4-(1-Amino-cyclohexanecarbonyl)-6-oxo-hexahydro-pyrrolo[3,2-*b*]pyrrole-1-carbonyl]-3-methyl-butyl}-4-*tert*-butyl-benzamide;

1194. *N*-{1-[4-(1-Acetylamino-cyclobutanecarbonyl)-6-oxo-hexahydro-pyrrolo[3,2-*b*]pyrrole-1-carbonyl]-3-methyl-butyl}-4-tert-butyl-benzamide;

1195. *N*-{1-[4-(1-Acetylamino-cyclopentanecarbonyl)-6-oxo-hexahydro-pyrrolo[3,2-*b*]pyrrole-1-carbonyl]-3-methyl-butyl}-4-tert-butyl-benzamide;

1196. *N*-{1-[4-(1-Acetylamino-cyclohexanecarbonyl)-6-oxo-hexahydro-pyrrolo[3,2-*b*]pyrrole-1-carbonyl]-3-methyl-butyl}-4-tert-butyl-benzamide;

1197. 4-*tert*-Butyl-*N*-{1-[4-(1-hydroxy-cyclobutanecarbonyl)-6-oxo-hexahydro-pyrrolo[3,2-*b*]pyrrole-1-carbonyl]-3-methyl-butyl}-benzamide;

1198. 4-*tert*-Butyl-*N*-{1-[4-(1-hydroxy-cyclopentanecarbonyl)-6-oxo-hexahydro-pyrrolo[3,2-*b*]pyrrole-1-carbonyl]-3-methyl-butyl}-benzamide;

1199. 4-*tert*-Butyl-*N*-{1-[4-(1-hydroxy-cyclohexanecarbonyl)-6-oxo-hexahydro-pyrrolo[3,2-*b*]pyrrole-1-carbonyl]-3-methyl-butyl}-benzamide;

1200. 4-*tert*-Butyl-*N*-{1-[4-(1-methoxy-cyclobutanecarbonyl)-6-oxo-hexahydro-pyrrolo[3,2-*b*]pyrrole-1-carbonyl]-3-methyl-butyl}-benzamide;

1201. 4-*tert*-Butyl-*N*-{1-[4-(1-methoxy-cyclopentanecarbonyl)-6-oxo-hexahydro-pyrrolo[3,2-*b*]pyrrole-1-carbonyl]-3-methyl-butyl}-benzamide;

1202. 4-*tert*-Butyl-*N*-{1-[4-(1-methoxy-cyclohexanecarbonyl)-6-oxo-hexahydro-pyrrolo[3,2-*b*]pyrrole-1-carbonyl]-3-methyl-butyl}-benzamide;

1203. *N*-{1-[4-(2-Amino-2-cyclopentyl-acetyl)-6-oxo-hexahydro-pyrrolo[3,2-*b*]pyrrole-1-carbonyl]-3-methyl-butyl}-4-*tert*-butyl-benzamide;

1204. *N*-{1-[4-(2-Amino-2-cyclohexyl-acetyl)-6-oxo-hexahydro-pyrrolo[3,2-*b*]pyrrole-1-carbonyl]-3-methyl-butyl}-4-*tert*-butyl-benzamide;

1205. *N*-{1-[4-(2-Acetylamino-2-cyclopentyl-acetyl)-6-oxo-hexahydro-pyrrolo[3,2-*b*]pyrrole-1-carbonyl]-3-methyl-butyl}-4-*tert*-butyl-benzamide;

. 1206. *N*-{1-[4-(2-Acetylamino-2-cyclohexyl-acetyl)-6-oxo-hexahydro-pymolo[3,2-*b*]pyrrole-1-carbonyl]-3-methyl-butyl}-4-*tert*-butyl-benzamide;

1207. *N*-{1-[4-(2-Acetylamino-propionyl)-6-oxo-hexahydro-pyrrolo[3,2-*b*]pyrrole-1-carbonyl]-3-methyl-butyl}-4-*tert*-butyl-benzamide;

1208. *N*-{1-[4-(2-Acetylamino-acetyl)-6-oxo-hexahydro-pyrrolo[3,2-*b*]pyrrole-1-carbonyl]-3-methyl-butyl}-4-*tert*-butyl-benzamide;

1209. *N*-(1-{4-[2-(Acetyl-methyl-amino)-acetyl]-6-oxo-hexahydro-pyrrolo[3,2-*b*]pyrrole-1-carbonyl}-3-methyl-butyl)-4-*tert*-butyl-benzamide;

1210. 4-*tert*-Butyl-*N*-{1-[4-(2-dimethylamino-acetyl)-6-oxo-hexahydro-pyrrolo[3,2-*b*]pyrrole-1-carbonyl]-3-methyl-butyl}-benzamide;

1211. *N*-{1-[4-(2-Amino-butyryl)-6-oxo-hexahydro-pyrrolo[3,2-*b*]pyrrole-1-carbonyl]-3-methyl-butyl}-4-*tert*-butyl-benzamide;

. 1212. *N*-{1-[4-(2-Acetylamino-butyryl)-6-oxo-hexahydro-pyrrolo[3,2-*b*]pyrrole-1-carbonyl]-3-methyl-butyl}-4-*tert*-butyl-benzamide;

1213. *N*-{1-[4-(2-amino-3-methyl-butyryl)-6-oxo-hexahydro-pyrrolo[3,2-*b*]pyrrole-1-carbonyl]-3-methyl-butyl}-4-*tert*-butyl-benzamide;

1214. *N*-{1-[4-(2-Acetylamino-3-methyl-butyryl)-6-oxo-hexahydro-pyrrolo[3,2-*b*]pyrrole-1-carbonyl]-3-methyl-butyl)-4-*tert*-butyl-benzamide;

. 1215. *N*-{1-[4-(2-amino-3,3-dimethyl-butyryl)-6-oxo-hexahydro-pyrrolo[3,2-*b*]pyrrole-1-carbonyl]-3-methyl-butyl}-4-*tert*-butyl-benzamide;

. 1216. *N*-{1-[4-(2-Acetylamino-3,3-dimethyl-butyryl)-6-oxo-hexahydro-pynolo[3,2-*b*]pyrrole-1-carbonyl]-3-methyl-butyl}-4-*tert*-butyl-benzamide;

1217. *N*-{1-[4-(2-Amino-3-methyl-pentanoyl)-6-oxo-hexahydro-pyrrolo[3,2-*b*]pyrrole-1-carbonyl]-3-methyl-butyl}-4-*tert*-butyl-benzamide;

. 1218. *N*-{1-[4-(2-Acetylamino-3-methyl-pentanoyl)-6-oxo-hexahydro-pynolo[3,2-*b*]pyrrole-1-carbonyl]-3-methyl-butyl}-4-*tert*-butyl-benzamide;

1219. 4-*tert*-Butyl-*N*-[3-methyl-1-(6-oxo-4-pentanoyl-hexahydro-pyrrolo[3,2-*b*]pyrrole-1-carbonyl)-butyl]-benzamide;

1220. 4-*tert*-Butyl-*N*-{3-methyl-1-[4-(3-methyl-pentanoyl)-6-oxo-hexahydro-pyrrolo[3,2-*b*]pyrrole-1-carbonyl]-butyl}-benzamide;

. 1221. 4-*tert*-Butyl-*N*-{3-methyl-1-[4-(4-methyl-pentanoyl)-6-oxo-hexahydro-pyrrolo[3,2-*b*]pyrrole-1-carbonyl]-butyl}-benzamide;

. 1222. *N*-{1-[4-(2-Amino-4-methyl-pentanoyl)-6-oxo-hexahydro-pyrrolo[3,2-*b*]pyrrole-1-carbonyl]-3-methyl-butyl}-4-*tert*-butyl-benzamide;

1223. *N*-{1-[4-(2-Acetylamino-4-methyl-pentanoyl)-6-oxo-hexahydro-pyrrolo[3,2-*b*]pyrrole-1-carbonyl]-3-methyl-butyl}-4-*tert*-butyl-benzamide;

. 1224. *N*-{1-[4-(2-Amino-4,4-dimethyl-pentanoyl)-6-oxo-hexahydro-pyrrolo[3,2-*b*]pyrrole-1-carbonyl]-3-methyl-butyl}-4-*tert*-butyl-benzamide;

1225. *N*-{1-[4-(2-Acetylamino-4,4-dimethyl-pentanoyl)-6-oxo-hexahydro-pyrrolo[3,2-*b*]pyrrole-1-carbonyl]-3-methyl-butyl}-4-*tert*-butyl-benzamide;

1226. *N*-{1-[4-(2-Amino-pentanoyl)-6-oxo-hexahydro-pyrrolo[3,2-*b*]pyrrole-1-carbonyl]-3-methyl-butyl}-4-*tert*-butyl-benzamide;

1227. *N*-{1-[4-(2-Acetylamino-pentanoyl)-6-oxo-hexahydro-pyrrolo[3,2-*b*]pyrrole-1-carbonyl]-3-methyl-butyl}-4-*tert*-butyl-benzamide;

1228. *N*-{1-[4-(2-Amino-5-methyl-hexanoyl)-6-oxo-hexahydro-pyrrolo[3,2-*b*]pyrrole-1-carbonyl]-3-methyl-butyl}-4-*tert*-butyl-benzamide;

1229. *N*-{1-[4-(2-Acetylamino-5-methyl-hexanoyl)-6-oxo-hexahydro-pyrrolo[3,2-*b*]pyrrole-1-carbonyl]-3-methyl-butyl}-4-*tert*-butyl-benzamide;

1230. *N*-{1-[4-(2-hydroxy-3-methyl-butyryl)-6-oxo-hexahydro-pyrrolo[3,2-*b*]pyrrole-1-carbonyl]-3-methyl-butyl}-4-*tert*-butyl-benzamide;

1231. *N*-{1-[4-(2-methoxy-3-methyl-butyryl)-6-oxo-hexahydro-pyrrolo[3,2-*b*]pyrrole-1-carbonyl]-3-methyl-butyl}-4-*tert*-butyl-benzamide;

. 1232. 4-*tert*-Butyl-*N*-{1-[4-(2-hydroxy-3,3-dimethyl-butyryl)-6-oxo-hexahydro-pyrrolo[3,2-*b*]pyrrole-1-carbonyl]-3-methyl-butyl}-benzamide;

. 1233. 4-*tert*-Butyl-*N*-{1-[4-(2-methoxy-3,3-dimethyl-butyryl)-6-oxo-hexahydro-pyrrolo[3,2-*b*]pyrrole-1-carbonyl]-3-methyl-butyl}-benzamide;

- 1234. 4-*tert*-Butyl-*N*-{1-[4-(2-hydroxy-3-methyl-pentanoyl)-6-oxo-hexahydro-pyrrolo[3,2-*b*]pyrrole-1-carbonyl]-3-methyl-butyl}-benzamide;

- 1235. 4-*tert*-Butyl-*N*-{1-[4-(2-methoxy-3-methyl-pentanoyl)-6-oxo-hexahydro-pyrrolo[3,2-*b*]pyrrole-1-carbonyl]-3-methyl-butyl}-benzamide;

- 1236. 4-*tert*-Butyl-*N*-{1-[4-(2-hydroxy-4-methyl-pentanoyl)-6-oxo-hexahydro-pyrrolo[3,2-*b*]pyrrole-1-carbonyl]-3-methyl-butyl}-benzamide;

- 1237. 4-*tert*-Butyl-*N*-{1-[4-(2-methoxy-4-methyl-pentanoyl)-6-oxo-hexahydro-pyrrolo[3,2-*b*]pyrrole-1-carbonyl]-3-methyl-butyl}-benzamide;

- 1238. 4-*tert*-Butyl-*N*-{1-[4-(2-hydroxy-4,4-dimethyl-pentanoyl)-6-oxo-hexahydro-pyrrolo[3,2-*b*]pyrrole-1-carbonyl]-3- methyl-butyl}-benzamide;

- 1239. 4-*tert*-Butyl-*N*-{1-[4-(2-methoxy-4,4-dimethyl-pentanoyl)-6-oxo-hexahydro-pyrrolo[3,2-*b*]pyrrole-1-carbonyl]-3- methyl-butyl}-benzamide;

1240. 4-*tert*-Butyl-*N*-{1-[4-(2-hydroxy-pentanoyl)-6-oxo-hexahydro-pyrrolo[3,2-*b*]pyrrole-1-carbonyl]-3-methyl-butyl}-benzamide;

1241. 4-*tert*-Butyl-*N*-{1-[4-(2-methoxy-pentanoyl)-6-oxo-hexahydro-pyrrolo[3,2-*b*]pyrrole-1-carbonyl]-3-methyl-butyl}-benzamide;

1242. 4-*tert*-Butyl-*N*-{1-[4-(2-hydroxy-5-methyl-hexanoyl)-6-oxo-hexahydro-pyrrolo[3,2-*b*]pyrrole-1-carbonyl]-3-methyl-butyl}-benzamide;

- 1243. 4-*tert*-Butyl-*N*-{1-[4-(2-methoxy-5-methyl-hexanoyl)-6-oxo-hexahydro-pyrrolo[3,2-*b*]pyrrole-1-carbonyl]-3-methyl-butyl}-benzamide;

1244. *N*-[1-(4-Benzoyl-6-oxo-hexahydro-pyrrolo[3,2-*b*]pyrrole-1-carbonyl)-3-methyl-butyl]-4-dimethylaminobenzamide;

1245. 4-Dimethylamino-*N*-{3-methyl-1-[6-oxo-4-(1-oxy-pyridine-2-carbonyl)-hexahydro-pyrrolo[3,2-*b*]pyrrole-1-carbonyl]-butyl}-benzamide;

1246. 4-Dimethylamino-*N*-{3-methyl-1-[6-oxo-4-(pyridine-3-carbonyl)-hexahydro-pyrrolo[3,2-*b*]pyrrole-1-carbonyl]-butyl}-benzamide;

1247. 4-Dimethylamino-*N*-{3-methyl-1-[6-oxo-4-(1-oxy-pyridine-3-carbonyl)-hexahydro-pyrrolo[3,2-*b*]pyrrole-1-carbonyl]-butyl}-benzamide;

1248. 4-Dimethylamino-*N*-{3-methyl-1-[6-oxo-4-(pyridine-4-carbonyl)-hexahydro-pyrrolo[3,2-*b*]pyrrole-1-carbonyl]-butyl}-benzamide;

1249. 4-Dimethylamino-*N*-{3-methyl-1-[6-oxo-4-(1-oxy-pyridine-4-carbonyl)-hexahydro-pyrrolo[3,2-*b*]pyrrole-1-carbonyl]-butyl}-benzamide;

1250. *N*-[1-(4-Benzenesulfonyl-6-oxo-hexahydro-pyrrolo[3,2-*b*]pyrrole-1-carbonyl)-3-methyl-butyl]-4-dimethylamino-benzamide;

1251. 4-Dimethylamino-*N*-{3-methyl-1-[6-oxo-4-(pyridine-4-sulfonyl)-hexahydro-pyrrolo[3,2-*b*]pyrrole-1-carbonyl]-butyl}-benzamide;

1252. 4-Dimethylamino-*N*-{3-methyl-1-[6-oxo-4-(1-oxy-pyridine-4-sulfonyl)-hexahydro-pyrrolo[3,2-*b*]pyrrole-1-carbonyl]-butyl}-benzamide;

1253. 4-Dimethylamino-*N*-[3-methyl-1-(6-oxo-4-phenylacetyl-hexahydro-pyrrolo[3,2-*b*]pyrrole-1-carbonyl)-butyl]-benzamide;

1254. 4-[2-(4-Dimethylamino-benzoylamino)-4-methyl-pentanoyl]-3-oxo-hexahydro-pyrrolo[3,2-*b*]pyrrole-1-carboxylic acid phenylamide;

1255. 4-[2-(4-Dimethylamino-benzoylamino)-4-methyl-pentanoyl]-3-oxo-hexahydro-pyrrolo[3,2-*b*]pyrrole-1-carboxylic acid isobutyl-amide;

1256. 4-[2-(4-Dimethylamino-benzoylamino)-4-methyl-pentanoyl]-3-oxo-hexahydro-pyrrolo[3,2-*b*]pyrrole-1-carboxylic acid phenyl ester;

1257. 4-[2-(4-Dimethylamino-benzoylamino)-4-methyl-pentanoyl]-3-oxo-hexahydro-pyrrolo[3,2-*b*]pyrrole-1-carboxylic acid isobutyl ester;

1258. 4-Dimethylamino-*N*-{3-methyl-1-[6-oxo-4-(pyrrolidine-1-carbonyl)-hexahydro-pyrrolo[3,2-*b*]pyrrole-1-carbonyl]-butyl}-benzamide;

1259. 4-Dimethylamino-*N*-{3-methyl-1-[6-oxo-4-(piperidine-1-carbonyl)-hexahydro-pyrrolo[3,2-*b*]pyrrole-1-carbonyl]-butyl}-benzamide;

1260. 4-Dimethylamino-*N*-{3-methyl-1-[4-(morpholine-4-carbonyl)-6-oxo-hexahydro-pyrrolo[3,2-*b*]pyrrole-1-carbonyl]-butyl}-benzamide;

1261. 4-Dimethylamino-*N*-{3-methyl-1-[6-oxo-4-(piperazine-1-carbonyl)-hexahydro-pyrrolo[3,2-*b*]pyrrole-1-carbonyl]-butyl}-benzamide;

1262. 4-Dimethylamino-*N*-{3-methyl-1-[4-(4-methyl-piperazine-1-carbonyl)-6-oxo-hexahydro-pyrrolo[3,2-*b*]pyrrole-1-carbonyl]-butyl}-benzamide;

1263. 4-Dimethylamino-*N*-(3-methyl-1-{4-[2-(4-methyl-piperazin-1-yl)-acetyl]-6-oxo-hexahydro-pyrrolo[3,2-*b*]pyrrole-1-carbonyl}-butyl)-benzamide;

1264. 4-Dimethylamino-*N*-{3-methyl-1-[6-oxo-4-(2-piperazin-1-yl-acetyl)-hexahydro-pyrrolo[3,2-*b*]pyrrole-1-carbonyl]-butyl}-benzamide;

1265. 4-Dimethylamino-*N*-{1-[4-(furan-2-carbonyl)-6-oxo-hexahydro-pyrrolo[3,2-*b*]pyrrole-1-carbonyl]-3-methyl-butyl}-benzamide;

1266. 4-Dimethylamino-*N*-{1-[4-(5-chloro-furan-2-carbonyl)-6-oxo-hexahydro-pyrrolo[3,2-*b*]pyrrole-1-carbonyl]-3-methyl-butyl}-benzamide;

1267. 4-Dimethylamino-*N*-{1-[4-(thiophene-2-carbonyl)-6-oxo-hexahydro-pyrrolo[3,2-*b*]pyrrole-1-carbonyl]-3-methyl-butyl}-benzamide;

1268. 4-Dimethylamino-*N*-{1-[4-(5-chloro-thiophene-2-carbonyl)-6-oxo-hexahydro-pyrrolo[3,2-*b*]pyrrole-1-carbonyl]-3- methyl-butyl}-benzamide;

1269. 4-Dimethylamino-*N*-{1-[4-(furan-3-carbonyl)-6-oxo-hexahydro-pyrrolo[3,2-*b*]pyrrole-1-carbonyl]-3-methyl-butyl}-benzamide;

1270. 4-Dimethylamino-*N*-{1-[4-(thiophene-3-carbonyl)-6-oxo-hexahydro-pyrrolo[3,2-*b*]pyrrole-1-carbonyl]-3-methyl-butyl}-benzamide;

1271. 4-Dimethylamino-*N*-[1-(4-cyclopentanecarbonyl-6-oxo-hexahydro-pyrrolo[3,2-*b*]pyrrole-1-carbonyl)-3-methyl-butyl]-benzamide;

1272. 4-Dimethylamino-*N*-[1-(4-cyclohexanecarbonyl-6-oxo-hexahydro-pyrrolo[3,2-*b*]pyrrole-1-carbonyl)-3-methyl-butyl]-benzamide;

1273. 4-Dimethylamino-*N*-{1-[4-(cyclopent-3-enecarbonyl)-6-oxo-hexahydro-pyrrolo[3,2-*b*]pyrrole-1-carbonyl]-3-methyl-butyl}-benzamide;

1274. 4-Dimethylamino-*N*-{1-[4-(2-cyclopentyl-acetyl)-6-oxo-hexahydro-pyrrolo[3,2-*b*]pyrrole-1-carbonyl]-3-methyl-butyl}-benzamide;

1275. 4-Dimethylamino-*N*-{1-[4-(2-cyclohexyl-acetyl)-6-oxo-hexahydro-pyrrolo[3,2-*b*]pyrrole-1-carbonyl]-3-methyl-butyl}-benzamide;

1276. 4-Dimethylamino-*N*-{3-methyl-1-[6-oxo-4-(pyrrolidine-2-carbonyl)-hexahydro-pyrrolo[3,2-*b*]pyrrole-1-carbonyl]-butyl}-benzamide;

1277. *N*-{1-[4-(1-Acetyl-pyrrolidine-2-carbonyl)-6-oxo-hexahydro-pyrrolo[3,2-*b*]pyrrole-1-carbonyl]-3-methyl-butyl}-4-dimethylamino-benzamide;

1278. 4-Dimethylamino-*N*-{3-methyl-1-[6-oxo-4-(piperidine-2-carbonyl)-hexahydro-pyrrolo[3,2-*b*]pyrrole-1-carbonyl]-butyl}-benzamide;

1279. *N*-{1-[4-(1-Acetyl-piperidine-2-carbonyl)-6-oxo-hexahydro-pyrrolo[3,2-*b*]pyrrole-1-carbonyl]-3-methyl-butyl}-4-dimethylamino-benzamide;

1280. 4-Dimethylamino-*N*-{3-methyl-1-[6-oxo-4-(tetrahydro-furan-2-carbonyl)-hexahydro-pyrrolo[3,2-*b*]pyrrole-1-carbonyl]-butyl}-benzamide;

1281. *N*-{1-[4-(1-Amino-cyclobutanecarbonyl)-6-oxo-hexahydro-pyrrolo[3,2-*b*]pyrrole-1-carbonyl]-3-methyl-butyl}-4-dimethylamino-benzamide;

1282. *N*-{1-[4-(1-Amino-cyclopentanecarbonyl)-6-oxo-hexahydro-pyrrolo[3,2-*b*]pyrrole-1-carbonyl]-3-methyl-butyl}-4-dimethylamino-benzamide;

1283. *N*-{1-[4-(1-Amino-cyclohexanecarbonyl)-6-oxo-hexahydro-pyrrolo[3,2-*b*)pyrrole-1-carbonyl]-3-methyl-butyl}-4-dimethylamino-benzamide;

1284. *N*-{1-[4-(1-Acetylamino-cyclobutanecarbonyl)-6-oxo-hexahydro-pyrrolo[3,2-*b*]pyrrole-1-carbonyl]-3-methyl-butyl}-4-dimethylamino-benzamide;

1285. *N*-{1-[4-(1-Acetylamino-cyclopentanecarbonyl)-6-oxo-hexahydro-pyrrolo[3,2-*b*]pyrrole-1-carbonyl]-3-methyl-butyl}-4-dimethylamino-benzamide;

1286. *N*-{1-[4-(1-Acetylamino-cyclohexanecarbonyl)-6-oxo-hexahydro-pyrrolo[3,2-*b*]pyrrole-1-carbonyl]-3-methyl-butyl}-4-dimethylamino-benzamide;

1287. 4-Dimethylamino-*N*-{1-[4-(1-hydroxy-cyclobutanecarbonyl)-6-oxo-hexahydro-pyrrolo[3,2-*b*]pyrrole-1-carbonyl]-3-methyl-butyl}-benzamide;

1288. 4-Dimethylamino-*N*-{1-[4-(1-hydroxy-cyclopentanecarbonyl)-6-oxo-hexahydro-pyrrolo[3,2-*b*]pyrrole-1-carbonyl]-3-methyl-butyl}-benzamide;

1289. 4-Dimethylamino-*N*-{1-[4-(1-hydroxy-cyclohexanecarbonyl)-6-oxo-hexahydro-pyrrolo[3,2-*b*]pyrrole-1-carbonyl]-3-methyl-butyl}-benzamide;

1290. 4-Dimethylamino-*N*-{1-[4-(1-methoxy-cyclobutanecarbonyl)-6-oxo-hexahydro-pyrrolo[3,2-*b*]pyrrole-1-carbonyl]-3-methyl-butyl}-benzamide;

1291. 4-Dimethylamino-*N*-{1-[4-(1-methoxy-cyclopentanecarbonyl)-6-oxo-hexahydro-pyrrolo[3,2-*b*]pyrrole-1-carbonyl]-3-methyl-butyl}-benzamide;

1292. 4-Dimethylamino-*N*-{1-[4-(1-methoxy-cyclohexanecarbonyl)-6-oxo-hexahydro-pyrrolo[3,2-*b*]pyrrole-1-carbonyl]-3-methyl-butyl}-benzamide;

1293. *N*-{1-[4-(2-Amino-2-cyclopentyl-acetyl)-6-oxo-hexahydro-pyrrolo[3,2-*b*]pyrrole-1-carbonyl]-3-methyl-butyl}-4-dimethylamino-benzamide;

1294. *N*-{1-[4-(2-Amino-2-cyclohexyl-acetyl)-6-oxo-hexahydro-pyrrolo[3,2-*b*]pyrrole-1-carbonyl]-3-methyl-butyl}-4-dimethylamino-benzamide;

1295. *N*-{1-[4-(2-Acetylamino-2-cyclopentyl-acetyl)-6-oxo-hexahydro-pyrrolo[3,2-*b*]pyrrole-1-carbonyl]-3-methyl-butyl}-4-dimethylamino-benzamide;

1296. *N*-{1-[4-(2-Acetylamino-2-cyclohexyl-acetyl)-6-oxo-hexahydro-pyrrolo[3,2-*b*]pyrrole-1-carbonyl]-3-methyl-butyl}-4-dimethylamino-benzamide;

1297. *N*-{1-[4-(2-Acetylamino-propionyl)-6-oxo-hexahydro-pyrrolo[3,2-*b*]pyrrole-1-carbonyl]-3-methyl-butyl}-4-dimethylamino-benzamide;

1298. *N*-{1-[4-(2-Acetylamino-acetyl)-6-oxo-hexahydro-pyrrolo[3,2-*b*]pyrrole-1-carbonyl]-3-methyl-butyl}-4-dimethylamino-benzamide;

1299, *N*-(1-{4-[2-(Acetyl-methyl-amino)-acetyl]-6-oxo-hexahydro-pyrrolo[3,2-*b*]pyrrole-1-carbonyl}-3-methyl-butyl)-4-dimethylamino-benzamide;

1300. 4-Dimethylamino-*N*-{1-[4-(2-dimethylamino-acetyl)-6-oxo-hexahydro-pyrrolo[3,2-*b*]pynole-1-carbonyl]-3-methyl-butyl}-benzamide;

1301. *N*-{1-[4-(2-Amino-butyryl)-6-oxo-hexahydro-pyrrolo[3,2-*b*]pyrrole-1-carbonyl]-3-methyl-butyl}-4-dimethylamino-benzamide;

1302. *N*-{1-[4-(2-Acetylamino-butyryl)-6-oxo-hexahydro-pyrrolo[3,2-*b*]pyrrole-1-carbonyl]-3-methyl-butyl}-4-dimethylamino-benzamide;

1303. *N*-{1-[4-(2-amino-3-methyl-butyryl)-6-oxo-hexahydro-pyrrolo[3,2-*b*]pyrrole-1-carbonyl]-3-methyl-butyl}-4-dimethylamino-benzamide;

1304. *N*-{1-[4-(2-Acetylamino-3-methyl-butyryl)-6-oxo-hexahydro-pyrrolo[3,2-*b*]pyrrole-1-carbonyl]-3-methyl-butyl}-4-dimethylamino-benzamide;

1305. *N*-{1-[4-(2-amino-3,3-dimethyl-butyryl)-6-oxo-hexahydro-pyrrolo[3,2-*b*]pyrrole-1-carbonyl]-3-methyl-butyl}-4-dimethylamino-benzamide;

1306. *N*-{1-[4-(2-Acetylamino-3,3-dimethyl-butyryl)-6-oxo-hexahydro-pyrrolo[3,2-*b*]pyrrole-1-carbonyl]-3-methyl-butyl}-4-dimethylamino-benzamide;

1307. *N*-{1-[4-(2-Amino-3-methyl-pentanoyl)-6-oxo-hexahydro-pyrrolo[3,2-*b*]pyrrole-1-carbonyl]-3-methyl-butyl}-4-dimethylamino-benzamide;

1308. *N*-{1-[4-(2-Acetylamino-3-methyl-pentanoyl)-6-oxo-hexahydro-pyrrolo[3,2-*b*]pyrrole-1-carbonyl]-3-methyl-butyl}-4-dimethylamino-benzamide;

1309. 4-Dimethylamino-*N*-[3-methyl-1-(6-oxo-4-pentanoyl-hexahydro-pyrrolo[3,2-*b*]pyrrole-1-carbonyl)-butyl]-benzamide;

1310. 4-Dimethylamino-*N*-{3-methyl-1-[4-(3-methyl-pentanoyl)-6-oxo-hexahydro-pyrrolo[3,2-*b*]pyrrole-1-carbonyl]-butyl}-benzamide;

1311. 4-Dimethylamino-*N*-{3-methyl-1-[4-(4-methyl-pentanoyl)-6-oxo-hexahydro-pyrrolo[3,2-*b*]pynole-1-carbonyl]-butyl}-benzamide;

1312. *N*-{1-[4-(2-Amino-4-methyl-pentanoyl)-6-oxo-hexahydro-pyrrolo[3,2-*b*]pyrrole-1-carbonyl]-3-methyl-butyl}-4-dimethylamino-benzamide;

1313. *N*-{1-[4-(2-Acetylamino-4-methyl-pentanoyl)-6-oxo-hexahydro-pyrrolo[3,2-*b*]pyrrole-1-carbonyl]-3-methyl-butyl}-4-dimethylamino-benzamide;

1314. *N*-{1-[4-(2-Amino-4,4-dimethyl-pentanoyl)-6-oxo-hexahydro-pyrrolo[3,2-*b*]pyrrole-1-carbonyl]-3-methyl-butyl}-4-dimethylamino-benzamide;

1315. *N*-{1-[4-(2-Acetylamino-4,4-dimethyl-pentanoyl)-6-oxo-hexahydro-pyrrolo[3,2-*b*]pyrrole-1-carbonyl]-3-methyl-butyl}-4-dimethylamino-benzamide;

1316. *N*-{1-[4-(2-Amino-pentanoyl)-6-oxo-hexahydro-pyrrolo[3,2-*b*]pyrrole-1-carbonyl]-3-methyl-butyl}-4-dimethylamino-benzamide;

1317. *N*-{1-[4-(2-Acetylamino-pentanoyl)-6-oxo-hexahydro-pyrrolo[3,2-*b*]pyrrole-1-carbonyl]-3-methyl-butyl}-4-dimethylamino-benzamide;

1318. *N*-{1-[4-(2-Amino-5-methyl-hexanoyl)-6-oxo-hexahydro-pyrrolo[3,2-*b*]pyrrole-1-carbonyl]-3-methyl-butyl}-4-dimethylamino-benzamide;

1319. *N*-{1-[4-(2-Acetylamino-5-methyl-hexanoyl)-6-oxo-hexahydro-pyrrolo[3,2-*b*]pyrrole-1-carbonyl]-3-methyl-butyl}-4-dimethylamino-benzamide;

1320. *N*-{1-[4-(2-hydroxy-3-methyl-butyryl)-6-oxo-hexahydro-pyrrolo[3,2-*b*]pyrrole-1-carbonyl]-3-methyl-butyl}-4-dimethylamino-benzamide;

1321. *N*-{1-[4-(2-methoxy-3-methyl-butyryl)-6-oxo-hexahydro-pyrrolo[3,2-*b*]pyrrole-1-carbonyl]-3-methyl-butyl}-4-dimethylamino-benzamide;

1322. 4-Dimethylamino-*N*-{1-[4-(2-hydroxy-3,3-dimethyl-butyryl)-6-oxo-hexahydro-pyrrolo[3,2-*b*]pyrrole-1-carbonyl]-3-methyl-butyl}-benzamide;

1323. 4-Dimethylamino-*N*-{1-[4-(2-methoxy-3,3-dimethyl-butyryl)-6-oxo-hexahydro-pyrrolo[3,2-*b*]pyrrole-1-carbonyl]-3-methyl-butyl}-benzamide;

1324. 4-Dimethylamino-*N*-{1-[4-(2-hydroxy-3-methyl-pentanoyl)-6-oxo-hexahydro-pyrrolo[3,2-*b*]pyrrole-1-carbonyl]-3-methyl-butyl}-benzamide;

1325. 4-Dimethylamino-*N*-{1-[4-(2-methoxy-3-methyl-pentanoyl)-6-oxo-hexahydro-pyrrolo[3,2-*b*]pyrrole-1-carbonyl]-3-methyl-butyl}-benzamide;

1326. 4-Dimethylamino-*N*-{1-[4-(2-hydroxy-4-methyl-pentanoyl)-6-oxo-hexahydro-pyrrolo[3,2-*b*]pyrrole-1-carbonyl]-3-methyl-butyl}-benzamide;

1327. 4-Dimethylamino-*N*-{1-[4-(2-methoxy-4-methyl-pentanoyl)-6-oxo-hexahydro-pyrrolo[3,2-*b*]pyrrole-1-carbonyl]-3-methyl-butyl}-benzamide;

1328. 4-Dimethylamino-*N*-{1-[4-(2-hydroxy-4,4-dimethyl-pentanoyl)-6-oxo-hexahydro-pyrrolo[3,2-*b*]pyrrole-1-carbonyl]-3-methyl-butyl}-benzamide;

1329. 4-Dimethylamino-*N*-{1-[4-(2-methoxy-4,4-dimethyl-pentanoyl)-6-oxo-hexahydro-pyrrolo[3,2-*b*]pyrrole-1-carbonyl]-3-methyl-butyl}-benzamide;

1330. 4-Dimethylamino-*N*-{1-[4-(2-hydroxy-pentanoyl)-6-oxo-hexahydro-pyrrolo[3,2-*b*]pyrrole-1-carbonyl]-3-methyl-butyl}-benzamide;

1331. 4-Dimethylamino-*N*-{1-[4-(2-methoxy-pentanoyl)-6-oxo-hexahydro-pyrrolo[3,2-*b*]pyrrole-1-carbonyl]-3-methyl-butyl}-benzamide;

1332. 4-Dimethylamino-*N*-{1-[4-(2-hydroxy-5-methyl-hexanoyl)-6-oxo-hexahydro-pyrrolo[3,2-*b*]pyrrole-1-carbonyl]-3-methyl-butyl}-benzamide;

1333. 4-Dimethylamino-*N*-{1-[4-(2-methoxy-5-methyl-hexanoyl)-6-oxo-hexahydro-pyrrolo[3,2-*b*]pyrrole-1-carbonyl]-3-methyl-butyl}-benzamide;

1334. *N*-[1-(4-Benzoyl-6-oxo-hexahydro-pyrrolo[3,2-*b*]pyrrole-1-carbonyl)-3-methyl-butyl]-4-thiophen-2-yl-benzamide;

1335. *N*-{3-methyl-1-[6-oxo-4-(1-oxy-pyridine-2-carbonyl)-hexahydro-pyrrolo[3,2-*b*]pyrrole-1-carbonyl]-butyl}-4-thiophen-2-yl-benzamide;

1336. *N*-{3-methyl-1-[6-oxo-4-(pyridine-3-carbonyl)-hexahydro-pyrrolo[3,2-*b*]pyrrole-1-carbonyl]-butyl}-4-thiophen-2-yl-benzamide;

1337. *N*-{3-methyl-1-[6-oxo-4-(1-oxy-pyridine-3-carbonyl)-hexahydro-pyrrolo[3,2-*b*]pyrrole-1-carbonyl]-butyl}-4-thiophen-2-yl-benzamide;

1338. *N*-{3-methyl-1-[6-oxo-4-(pyridine-4-carbonyl)-hexahydro-pyrrolo[3,2-*b*]pyrrole-1-carbonyl]-butyl}-4-thiophen-2-yl-benzamide;

1339. *N*-{3-methyl-1-[6-oxo-4-(1-oxy-pyridine-4-carbonyl)-hexahydro-pyrrolo[3,2-*b*]pyrrole-1-carbonyl]-butyl}-4-thiophen-2-yl-benzamide;

1340. *N*-[1-(4-Benzenesulfonyl-6-oxo-hexahydro-pyrrolo[3,2-*b*]pyrrole-1-carbonyl)-3-methyl-butyl]-4-thiophen-2-yl-benzamide;

1341. *N*-{3-methyl-1-[6-oxo-4-(pyridine-4-sulfonyl)-hexahydro-pyrrolo[3,2-*b*]pyrrole-1-carbonyl]-butyl}-4-thiophen-2-yl-benzamide;

1342. *N*-{3-methyl-1-[6-oxo-4-(1-oxy-pyridine-4-sulfonyl)-hexahydro-pyrrolo[3,2-*b*]pyrrole-1-carbonyl]-butyl}-4- thiophen-2-yl-benzamide;

1343. *N*-[3-methyl-1-(6-oxo-4-phenylacetyl-hexahydro-pyrrolo[3,2-*b*]pyrrole-1-carbonyl)-butyl]-4-thiophen-2-yl-benzamide;

1344. 4-[4-Methyl-2-(4-thiophen-2-yl-benzoylamino)-pentanoyl]-3-oxo-hexahydro-pyrrolo[3,2-*b*]pyrrole-1-carboxylic acid phenylamide;

1345. 4-[4-Methyl-2-(4-thiophen-2-yl-benzoylamino)-pentanoyl]-3-oxo-hexahydro-pyrrolo[3,2-*b*]pyrrole-1-carboxylic acid isobutyl-amide;

1346. 4-[4-Methyl-2-(4-thiophen-2-yl-benzoylamino)-pentanoyl]-3-oxo-hexahydro-pyrrolo[3,2-*b*]pyrrole-1-carboxylic acid phenyl ester;

1347. 4-[4-Methyl-2-(4-thiophen-2-yl-benzoylamino)-pentanoyl]-3-oxo-hexahydro-pyrrolo[3,2-*b*]pyrrole-1-carboxylic acid isobutyl ester;

1348. *N*-{3-methyl-1-[6-oxo-4-(pyrrolidine-1-carbonyl)-hexahydro-pyrrolo[3,2-*b*]pyrrole-1-carbonyl]-butyl}-4-thiophen-2-yl-benzamide;

1349. *N*-{3-methyl-1-[6-oxo-4-(piperidine-1-carbonyl)-hexahydro-pyrrolo[3,2-*b*]pyrrole-1-carbonyl]-butyl}-4-thiophen-2-yl-benzamide;

1350. *N*-{3-methyl-1-[4-(morpholine-4-carbonyl)-6-oxo-hexahydro-pyrrolo[3,2-*b*]pyrrole-1-carbonyl]-butyl}-4-thiophen-2-yl-benzamide;

1351. *N*-{3-methyl-1-[6-oxo-4-(piperazine-1-carbonyl)-hexahydro-pyrrolo[3,2-*b*]pyrrole-1-carbonyl]-butyl}-4-thiophen-2-yl-benzamide;

1352. *N*-{3-methyl-1-[4-(4-methyl-piperazine-1-carbonyl)-6-oxo-hexahydro-pyrrolo[3,2-*b*]pyrrole-1-carbonyl]-butyl}-4-thiophen-2-yl-benzamide;

1353. *N*-(3-methyl-1-{4-[2-(4-methyl-piperazin-1-yl)-acetyl]-6-oxo-hexahydro-pyrrolo[3,2-*b*]pyrrole-1-carbonyl}-butyl)-4-thiophen-2-yl-benzamide;

1354. *N*-{3-methyl-1-[6-oxo-4-(2-piperazin-1-yl-acetyl)-hexahydro-pyrrolo[3,2-*b*]pyrrole-1-carbonyl]-butyl}-4-thiophen-2-yl-benzamide;

1355. *N*-{1-[4-(furan-2-carbonyl)-6-oxo-hexahydro-pyrrolo[3,2-*b*]pyrrole-1-carbonyl]-3-methyl-butyl}-4-thiophen-2-yl-benzamide;

1356. *N*-{1-[4-(5-chloro-furan-2-carbonyl)-6-oxo-hexahydro-pyrrolo[3,2-*b*]pyrrole-1-carbonyl]-3-methyl-butyl}- 4-thiophen-2-yl-benzamide;

1357. *N*-{1-[4-(thiophene-2-carbonyl)-6-oxo-hexahydro-pyrrolo[3,2-*b*]pyrrole-1-carbonyl]-3-methyl-butyl}-4-thiophen-2-yl-benzamide;

1358. *N*-{1-[4-(5-chloro-thiophene-2-carbonyl)-6-oxo-hexahydro-pyrrolo[3,2-*b*]pyrrole-1-carbonyl]-3-methyl-butyl}-4-thiophen-2-yl-benzamide;

1359. *N*-{1-[4-(furan-3-carbonyl)-6-oxo-hexahydro-pyrrolo[3,2-*b*]pyrrole-1-carbon.yl]-3-methyl-butyl}-4-thiophen-2-yl-benzamide;

1360. *N*-{1-[4-(thiophene-3-carbonyl)-6-oxo-hexahydro-pyrrolo[3,2-*b*]pyrrole-1-carbonyl]-3-methyl-butyl}-4-thiophen-2-yl-benzamide;

1361. *N*-[1-(4-cyclopentanecarbonyl-6-oxo-hexahydro-pyrrolo[3,2-*b*]pyrrole-1-carbonyl)-3-methyl-butyl]-4-thiophen-2-yl-benzamide;

1362. *N*-[1-(4-cyclohexanecarbonyl-6-oxo-hexahydro-pyrrolo[3,2-*b*]pyrrole-1-carbonyl)-3-methyl-butyl]-4-thiophen-2-yl-benzamide;

1363. *N*-{1-[4-(cyclopent-3-enecarbonyl)-6-oxo-hexahydro-pyrrolo[3,2-*b*]pyrrole-1-carbonyl]-3-methyl-butyl}-4-thiophen-2-yl-benzamide;

1364. *N*-{1-[4-(2-cyclopentyl-acetyl)-6-oxo-hexahydro-pyrrolo[3,2-*b*]pyrrole-1-carbonyl]-3-methyl-butyl}-4-thiophen-2-yl-benzamide;

1365. *N*-{1-[4-(2-cyclohexyl-acetyl)-6-oxo-hexahydro-pyrrolo[3,2-*b*]pyrrole-1-carbonyl]-3-methyl-butyl}-4-thiophen-2-yl-benzamide;

1366. *N*-{3-methyl-1-[6-oxo-4-(pyrrolidine-2-carbonyl)-hexahydro-pyrrolo[3,2-*b*]pyrrole-1-carbonyl]-butyl}-4-thiophen-2-yl-benzamide;

1367. *N*-{1-[4-(1-Acetyl-pyrrolidine-2-carbonyl)-6-oxo-hexahydro-pyrrolo[3,2-*b*]pyrrole-1-carbonyl]-3-methyl-butyl}-4-thiophen-2-yl-benzamide;

1368. *N*-{3-methyl-1-[6-oxo-4-(piperidine-2-carbonyl)-hexahydro-pyrrolo[3,2-*b*]pyrrole-1-carbonyl]-butyl}-4-thiophen-2-yl-benzamide;

1369. *N*-{1-[4-(1-Acetyl-piperidine-2-carbonyl)-6-oxo-hexahydro-pyrrolo[3,2-*b*]pyrrole-1-carbonyl]-3-methyl-butyl}-4-thiophen-2-yl-benzamide;

1370. *N*-{3-methyl-1-[6-oxo-4-(tetrahydro-furan-2-carbonyl)-hexahydro-pyrrolo[3,2-*b*]pyrrole-1-carbonyl]-butyl}-4-thiophen-2-yl-benzamide;

1371. *N*-{1-[4-(1-Amino-cyclobutanecarbonyl)-6-oxo-hexahydro-pyrrolo[3,2-*b*]pyrrole-1-carbonyl]-3-methyl-butyl}-4-thiophen-2-yl-benzamide;

1372. *N*-{1-[4-(1-Amino-cyclopentanecarbonyl)-6-oxo-hexahydro-pyrrolo[3,2-*b*]pyrrole-1-carbonyl]-3-methyl-butyl}-4-thiophen-2-yl-benzamide;

1373. *N*-{1-[4-(1-Amino-cyclohexanecarbonyl)-6-oxo-hexahydro-pyrrolo[3,2-*b*]pyrrole-1-carbonyl]-3-methyl-butyl}-4-thiophen-2-yl-benzamide;

1374. *N*-{1-[4-(1-Acetylamino-cyclobutanecarbonyl)-6-oxo-hexahydro-pyrrolo[3,2-*b*]pyrrole-1-carbonyl]-3-methyl-butyl}-4-thiophen-2-yl-benzamide;

1375. *N*-{1-[4-(1-Acetylamino-cyclopentanecarbonyl)-6-oxo-hexahydro-pyrrolo[3,2-*b*]pyrrole-1-carbonyl]-3-methyl-butyl}-4-thiophen-2-yl-benzamide;

1376. *N*-{1-[4-(1-Acetylamino-cyclohexanecarbonyl)-6-oxo-hexahydro-pyrrolo[3,2-*b*]pyrrole-1-carbonyl]-3-methyl-butyl}-4-thiophen-2-yl-benzamide;

1377. *N*-{1-[4-(1-hydroxy-cyclobutanecarbonyl)-6-oxo-hexahydro-pyrrolo[3,2-*b*]pyrrole-1-carbonyl]-3-methyl-butyl}-4-thiophen-2-yl-benzamide;

1378. *N*-{1-[4-(1-hydroxy-cyclopentanecarbonyl)-6-oxo-hexahydro-pyrrolo[3,2-*b*]pyrrole-1-carbonyl]-3-methyl-butyl}-4-thiophen-2-yl-benzamide;

1379. *N*-{1-[4-(1-hydroxy-cyclohexanecarbonyl)-6-oxo-hexahydro-pyrrolo[3,2-*b*]pyrrole-1-carbonyl]-3-methyl-butyl}-4-thiophen-2-yl-benzamide;

1380. *N*-{1-[4-(1-methoxy-cyclobutanecarbonyl)-6-oxo-hexahydro-pyrrolo[3,2-*b*]pyrrole-1-carbonyl]-3-methyl-butyl}-4-thiophen-2-yl-benzamide;

1381. *N*-{1-[4-(1-methoxy-cyclopentanecarbonyl)-6-oxo-hexahydro-pyrrolo[3,2-*b*]pyrrole-1-carbonyl]-3-methyl-butyl}-4-thiophen-2-yl-benzamide;

1382. *N*-{1-[4-(1-methoxy-cyclohexanecarbonyl)-6-oxo-hexahydro-pyrrolo[3,2-*b*]pyrrole-1-carbonyl]-3-methyl-butyl}-4-thiophen-2-yl-benzamide;

1383. *N*-{1-[4-(2-Amino-2-cyclopentyl-acetyl)-6-oxo-hexahydro-pyrrolo[3,2-*b*]pyrrole-1-carbonyl]-3-methyl-butyl}-4-thiophen-2-yl-benzamide;

1384. *N*-{1-[4-(2-Amino-2-cyclohexyl-acetyl)-6-oxo-hexahydro-pyrrolo[3,2-*b*]pyrrole-1-carbonyl]-3-methyl-butyl}-4-thiophen-2-yl-benzamide;

1385. *N*-{1-[4-(2-Acetylamino-2-cyclopentyl-acetyl)-6-oxo-hexahydro-pyrrolo[3,2-*b*]pyrrole-1-carbonyl]-3-methyl-butyl}-4-thiophen-2-yl-benzamide;

1386. *N*-{1-[4-(2-Acetylamino-2-cyclohexyl-acetyl)-6-oxo-hexahydro-pyrrolo[3,2-*b*]pyrrole-1-carbonyl]-3-methyl-butyl}-4-thiophen-2-yl-benzamide;

1387. *N*-{1-[4-(2-Acetylamino-propionyl)-6-oxo-hexahydro-pyrrolo[3,2-*b*]pyrrole-1-carbonyl]-3-methyl-butyl}-4-thiophen-2-yl-benzamide;

1388. *N*-{1-[4-(2-Acetylamino-acetyl)-6-oxo-hexahydro-pyrrolo[3,2-*b*]pyrrole-1-carbonyl]-3-methyl-butyl}-4-thiophen-2-yl-benzamide;

1389. *N*-(1-{4-[2-(Acetyl-methyl-amino)-acetyl]-6-oxo-hexahydro-pyrrolo[3,2-*b*]pyrrole-1-carbonyl}-3-methyl-butyl)-4-thiophen-2-yl-benzamide;

1390. *N*-{1-[4-(2-dimethylamino-acetyl)-6-oxo-hexahydro-pyrrolo[3,2-*b*]pyrrole-1-carbonyl]-3-methyl-butyl}-4-thiophen-2-yl-benzamide;

1391. *N*-{1-[4-(2-Amino-butyryl)-6-oxo-hexahydro-pyrrolo[3,2-*b*]pyrrole-1-carbonyl]-3-methyl-butyl}-4-thiophen-2-yl-benzamide;

1392. *N*-{1-[4-(2-Acetylamino-butyryl)-6-oxo-hexahydro-pyrrolo[3,2-*b*]pyrrole-1-carbonyl]-3-methyl-butyl}-4-thiophen-2-yl-benzamide;

1393. *N*-{1-[4-(2-amino-3-methyl-butyryl)-6-oxo-hexahydro-pyrrolo[3,2-*b*]pyrrole-1-carbonyl]-3-methyl-butyl}-4-thiophen-2-yl-benzamide;

1394. *N*-{1-[4-(2-Acetylamino-3-methyl-butyryl)-6-oxo-hexahydro-pyrrolo[3,2-*b*]pyrrole-1-carbonyl]-3-methyl-butyl}-4-thiophen-2-yl-benzamide;

1395. *N*{1-[4-(2-amino-3,3-dimethyl-butyryl)-6-oxo-hexahydro-pyrrolo[3,2-*b*]pyrrole-1-carbonyl]-3-methyl-butyl}-4-thiophen-2-yl-benzamide;

1396. *N*-{1-[4-(2-Acetylamino-3,3-dimethyl-butyryl)-6-oxo-hexahydro-pyrrolo[3,2-*b*]pyrrole-1-carbonyl]-3-methyl-butyl}-4-thiophen-2-yl-benzamide;

1397. *N*-{1-[4-(2-Amino-3-methyl-pentanoyl)-6-oxo-hexahydro-pyrrolo[3,2-*b*]pyrrole-1-carbonyl]-3-methyl-butyl}-4-thiophen-2-yl-benzamide;

1398. *N*-{1-[4-(2-Acetylamino-3-methyl-pentanoyl)-6-oxo-hexahydro-pyrrolo[3,2-*b*]pyrrole-1-carbonyl]-3-methyl-butyl}-4-thiophen-2-yl-benzamide;

1399. *N*-[3-methyl-1-(6-oxo-4-pentanoyl-hexahydro-pyrrolo[3,2-*b*]pyrrole-1-carbonyl)-butyl]-4-thiophen-2-yl-benzamide;

1400. *N*-{3-methyl-1-[4-(3-methyl-pentanoyl)-6-oxo-hexahydro-pyrrolo[3,2-*b*]pyrrole-1-carbonyl]-butyl}-4-thiophen-2-yl-benzamide;

1401. *N*-{3-methyl-1-[4-(4-methyl-pentanoyl)-6-oxo-hexahydro-pyrrolo[3,2-*b*]pyrrole-1-carbonyl]-butyl}-4-thiophen-2-yl-benzamide;

1402. *N*-{1-[4-(2-Amino-4-methyl-pentanoyl)-6-oxo-hexahydro-pyrrolo[3,2-*b*]pyrrole-1-carbonyl]-3-methyl-butyl}-4-thiophen-2-yl-benzamide;

1403. *N*-{1-[4-(2-Acetylamino-4-methyl-pentanoyl)-6-oxo-hexahydro-pyrrolo[3,2-*b*]pyrrole-1-carbonyl]-3-methyl-butyl}-4-thiophen-2-yl-benzamide;

1404. *N*-{1-[4-(2-Amino-4,4-dimethyl-pentanoyl)-6-oxo-hexahydro-pyrrolo[3,2-*b*]pyrrole-1-carbonyl]-3-methyl-butyl}-4-thiophen-2-yl-benzamide;

1405. *N*-{1-[4-(2-Acetylamino-4,4-dimethyl-pentanoyl)-6-oxo-hexahydro-pyrrolo[3,2-*b*]pyrrole-1-carbonyl]-3-methyl-butyl}-4-thiophen-2-yl-benzamide;

1406. *N*-{1-[4-(2-Amino-pentanoyl)-6-oxo-hexahydro-pyrrolo[3,2-*b*]pyrrole-1-carbonyl]-3-methyl-butyl}-4-thiophen-2-yl-benzamide;

1407. *N*-{1-[4-(2-Acetylamino-pentanoyl)-6-oxo-hexahydro-pyrrolo[3,2-*b*]pyrrole-1-carbonyl]-3-methyl-butyl}-4-thiophen-2-yl-benzamide;

1408. *N*-{1-[4-(2-Amino-5-methyl-hexanoyl)-6-oxo-hexahydro-pyrrolo[3,2-*b*]pyrrole-1-carbonyl]-3-methyl-butyl}-4-thiophen-2-yl-benzamide;

1409. *N*-{1-[4-(2-Acetylamino-5-methyl-hexanoyl)-6-oxo-hexahydro-pyrrolo[3,2-*b*]pyrrole-1-carbonyl]-3-methyl-butyl}-4-thiophen-2-yl-benzamide;

1410. *N*-{1-[4-(2-hydroxy-3-methyl-butyryl)-6-oxo-hexahydro-pyrrolo[3,2-*b*]pyrrole-1-carbonyl]-3-methyl-butyl}-4-thiophen-2-yl-benzamide;

1411. *N*-{1-[4-(2-methoxy-3-methyl-butyryl)-6-oxo-hexahydro-pyrrolo[3,2-*b*]pyrrole-1-carbonyl]-3-methyl-butyl}-4-thiophen-2-yl-benzamide;

1412. *N*-{1-[4-(2-hydroxy-3,3-dimethyl-butyryl)-6-oxo-hexahydro-pyrrolo[3,2-*b*]pyrrole-1-carbonyl]-3-methyl-butyl}-4-thiophen-2-yl-benzamide;

1413. *N*-{1-[4-(2-methoxy-3,3-dimethyl-butyryl)-6-oxo-hexahydro-pyrrolo[3,2-*b*]pyrrole-1-carbonyl]-3-methyl-butyl}-4-thiophen-2-yl-benzamide;

1414. *N*-{1-[4-(2-hydroxy-3-methyl-pentanoyl)-6-oxo-hexahydro-pyrrolo[3,2-*b*]pyrrole-1-carbonyl]-3-methyl-butyl}-4-thiophen-2-yl-benzamide;

1415. *N*-{1-[4-(2-methoxy-3-methyl-pentanoyl)-6-oxo-hexahydro-pyrrolo[3,2-*b*]pyrrole-1-carbonyl]-3-methyl-butyl}-4-thiophen-2-yl-benzamide;

1416. *N*-{1-[4-(2-hydroxy-4-methyl-pentanoyl)-6-oxo-hexahydro-pyrrolo[3,2-*b*]pyrrole-1-carbonyl]-3-methyl-butyl}-4-thiophen-2-yl-benzamide;

1417. *N*-{1-[4-(2-methoxy-4-methyl-pentanoyl)-6-oxo-hexahydro-pyrrolo[3,2-*b*]pyrrole-1-carbonyl]-3-methyl-butyl}-4-thiophen-2-yl-benzamide;

1418. *N*-{-[4-(2-hydroxy-4,4-dimethyl-pentanoyl)-6-oxo-hexahydro-pyrrolo[3,2-*b*]pyrrole-1-carbonyl]-3-methyl-butyl}-4-thiophen-2-yl-benzamide;

1419. *N*-{1-[4-(2-methoxy-4,4-dimethyl-pentanoyl)-6-oxo-hexahydro-pyrrolo[3,2-*b*]pyrrole-1-carbonyl]-3-methyl-butyl}-4-thiophen-2-yl-benzamide;

1420. *N*-{1-[4-(2-hydroxy-pentanoyl)-6-oxo-hexahydro-pyrrolo[3,2-*b*]pyrrole-1-carbonyl]-3-methyl-butyl}-4-thiophen-2-yl-benzamide;

1421. *N*-{1-[4-(2-methoxy-pentanoyl)-6-oxo-hexahydro-pyrrolo[3,2-*b*]pyrrole-1-carbonyl]-3-methyl-butyl}-4-thiophen-2-yl-benzamide;

1422. *N*-{1-[4-(2-hydroxy-5-methyl-hexanoyl)-6-oxo-hexahydro-pyrrolo[3,2-*b*]pyrrole-1-carbonyl]-3-methyl-butyl}-4-thiophen-2-yl-benzamide;

1423. *N*-{1-[4-(2-methoxy-5-methyl-hexanoyl)-6-oxo-hexahydro-pyrrolo[3,2-*b*]pyrrole-1-carbonyl]-3-methyl-butyl}-4-thiophen-2-yl-benzamide;

1424. 5-Phenyl-thiophene-2-carboxylic acid-[1-(4-Benzoyl-6-oxo-hexahydro-pyrrolo[3,2-*b*]pyrrole-1-carbonyl)-3-methyl-butyl]-amide;

1425. 5-Phenyl-thiophene-2-carboxylic acid-{3-methyl-1-[6-oxo-4-(pyridine-2-carbonyl)-hexahydro-pyrrolo[3,2-*b*]-pyrrole-1-carbonyl]-butyl}-amide;

1426. 5-Phenyl-thiophene-2-carboxylic acid-{3-methyl-1-[6-oxo-4-(1-oxy-pyridine-2-carbonyl)-hexahydro-pyrrolo[3,2-*b*]pyrrole-1-carbonyl]-butyl}-amide;

1427. 5-Phenyl-thiophene-2-carboxylic acid- {3-methyl-1-[6-oxo-4-(pyrrdine-3-carbonyl)-hexahydro-pyrrolo[3,2-*b*]-pyrrole-1-carbonyl]-butyl}-amide;

1428. 5-Phenyl-thiophene-2-carboxylic acid- {3-methyl-1-[6-oxo-4-(1-oxy-pyridine-3-carbonyl)-hexahydro-pyrrolo[3,2-*b*]pyrrole-1-carbonyl]-butyl}-amide;

1429. 5-Phenyl-thiophene-2-carboxylic acid- {3-methyl-1-[6-oxo-4-(pyridine-4-carbonyl)-hexahydro-pyrrolo[3,2-*b*]-pyrrole-1-carbonyl]-butyl}-amide;

1430. 5-Phenyl-thiophene-2-carboxylic acid- {3-methyl-1-[6-oxo-4-(1-oxy-pyridine-4-carbonyl)-hexahydro-pyrrolo[3,2-*b*]pyrrole-1-carbonyl]-butyl}-amide;

1431. 5-Phenyl-thiophene-2-carboxylic acid-[1-(4-Benzenesulfonyl-6-oxo-hexahydro-pyrrolo[3,2-*b*]pyrrole-1-carbonyl)-3-methyl-butyl]-amide;

1432. 5-Phenyl-thiophene-2-carboxylic acid- {3-methyl-1-[6-oxo-4-(pyridine-2-sulfonyl)-hexahydro-pyrrolo[3,2-*b*]-pyrrole-1-carbonyl]-butyl}-amide;

1433. 5-Phenyl-thiophene-2-carboxylic acid-{3-methyl-1-[6-oxo-4-(1-oxy-pyridine-2-sulfonyl)-hexahydro-pyrrolo[3,2-*b*]pyrrole-1-carbonyl]-butyl}-amide;

1434. 5-Phenyl-thiophene-2-carboxylic acid- {3-methyl-1-[6-oxo-4-(pyridine-3-sulfonyl)-hexahydro-pyrrolo[3,2-*b*]-pyrrole-1-carbonyl]-butyl}-amide;

1435. 5-Phenyl-thiophene-2-carboxylic acid- {3-methyl-1-[6-oxo-4-(1-oxy-pyridine-3-sulfonyl)-hexahydro-pyrrolo[3,2-*b*]pyrrole-1-carbonyl]-butyl}-amide;

1436. 5-Phenyl-thiophene-2-carboxylic acid-{3-methyl-1-[6-oxo-4-(pyridine-4-sulfonyl)-hexahydro-pyrrolo[3,2-*b*]-pyrrole-1-carbonyl]-butyl}-amide;

1437. 5-Phenyl-thiophene-2-carboxylic acid- {3-methyl-1-[6-oxo-4-(1-oxy-pyridine-4-sulfonyl)-hexahydro-pyrrolo[3,2-*b*]pyrrole-1-carbonyl]-butyl}-amide;

1438. 5-Phenyl-thiophene-2-carboxylic acid-[3-methyl-1-(6-oxo-4-phenylacetyl-hexahydro-pyrrolo[3,2-*b*]pyrrole-1-carbonyl)-butyl]-amide;

1439. 4-{4-Methyl-2-[(5-phenyl-thiophene-2-carbonyl)-amino]-pentanoyl}-3-oxo-hexahydro-pyrrolo[3,2-*b*]pyrrole-1-carboxylic acid phenylamide;

1440. 4-{4-Methyl-2-[(5-phenyl-thiophene-2-carbonyl)-amino]-pentanoyl}-3-oxo-hexahydro-pyrrolo[3,2-*b*]pyrrole-1-carboxylic acid isobutyl-amide;

1441. 4-{4-Methyl-2-[(5-phenyl-thiophene-2-carbonyl)-amino]-pentanoyl}-3-oxo-hexahydro-pyrrolo[3,2-*b*]pyrrole-1-carboxylic acid phenyl ester;

1442. 4-{4-Methyl-2-[(5-phenyl-thiophene-2-carbonyl)-amino]-pentanoyl}-3-oxo-hexahydro-pyrrolo[3,2-*b*]pyrrole-1-carboxylic acid isobutyl ester;

1443. 5-Phenyl-thiophene-2-carboxylic acid-{3-methyl-1-[6-oxo-4-(pyrrolidine-1-carbonyl)-hexahydro-pyrrolo[3,2-*b*]pyrrole-1-carbonyl]-butyl}-amide;

1444. 5-Phenyl-thiophene-2-carboxylic acid-{3-methyl-1-[6-oxo-4-(piperidine-1-carbonyl)-hexahydro-pyrrolo[3,2-*b*]pyrrole-1-carbonyl]-butyl}-amide;

1445. 5-Phenyl-thiophene-2-carboxylic acid-{3-methyl-1-[4-(morpholine-4-carbonyl)-6-oxo-hexahydro-pyrrolo[3,2-*b*]pyrrole-1-carbonyl]-butyl}-amide;

1446. 5-Phenyl-thiophene-2-carboxylic acid-{3-methyl-1-[6-oxo-4-(piperazine-1-carbonyl)-hexahydro-pyrrolo[3,2-*b*]pyrrole-1-carbonyl]-butyl}-amide;

1447. 5-Phenyl-thiophene-2-carboxylic acid-{3-methyl-1-[4-(4-methyl-piperazine-1-carbonyl)-6-oxo-hexahydro-pyrrolo[3,2-*b*]pyrrole-1-carbonyl]-butyl}-amide;

1448. 5-Phenyl-thiophene-2-carboxylic acid-(3-methyl-1-{4-[2-(4-methylpiperazin-1-yl)-acetyl]-6-oxo-hexahydro-pyrrolo[3,2-*b*]pyrrole-1-carbonyl}-butyl)-amide;

1449. 5-Phenyl-thiophene-2-carboxylic acid-{3-methyl-1-[6-oxo-4-(2-piperazin-1-yl-acetyl)-hexahydro-pyrrolo[3,2-*b*]pyrrole-1-carbonyl]-butyl}-amide;

1450. 5-Phenyl-thiophene-2-carboxylic acid- {1-[4-(furan-2-carbonyl)-6-oxo-hexahydro-pyrrolo[3,2-*b*]pyrrole-1-carbonyl]-3-methyl-butyl}-amide;

1451. 5-Phenyl-thiophene-2-carboxylic acid- {1-[4-(5-chloro-furan-2-carbonyl)-6-oxo-hexahydro-pyrrolo[3,2-*b*]pyrrole-1-carbonyl]-3-methyl-butyl}-amide;

1452. 5-Phenyl-thiophene-2-carboxylic acid- {1-[4-(thiophene-2-carbonyl)-6-oxo-hexahydro-pyrrolo[3,2-*b*]pyrrole-1-carbonyl]-3-methyl-butyl}-amide;

1453. 5-Phenyl-thiophene-2-carboxylic acid- {1-[4-(5-chloro-thiophene-2-carbonyl)-6-oxo-hexahydro-pyrrolo[3,2-*b*]pyrrole-1-carbonyl]-3-methyl-butyl}-amide;

1454. 5-Phenyl-thiophene-2-carboxylic acid- {1-[4-(furan-3-carbonyl)-6-oxo-hexahydro-pyrrolo[3,2-*b*]pyrrole-1-carbonyl]-3-methyl-butyl}-amide;

1455. 5-Phenyl-thiophene-2-carboxylic acid- {1-[4-(thiophene-3-carbonyl)-6-oxo-hexahydro-pyrrolo[3,2-*b*]pyrrole-1-carbonyl]-3-methyl-butyl}-amide;

1456. 5-Phenyl-thiophene-2-carboxylic acid-[1-(4-cyclopentanecarbonyl-6-oxo-hexahydro-pyrrolo[3,2-*b*]pyrrole-1-carbonyl)-3-methyl-butyl]-amide;

1457. 5-Phenyl-thiophene-2-carboxylic acid-[1-(4-cyclohexanecarbonyl-6-oxo-hexahydro-pyrrolo[3,2-*b*]pyrrole-1-carbonyl)-3-methyl-butyl]-amide;

1458. 5-Phenyl-thiophene-2-carboxylic acid-{1-[4-(cyclopent-3-enecarbonyl)-6-oxo-hexahydro-pyrrolo[3,2-*b*]pyrrole-1-carbonyl]-3-methyl-butyl}-amide;

1459. 5-Phenyl-thiophene-2-carboxylic acid- {1-[4-(2-cyclopentyl-acetyl)-6-oxo-hexahydro-pyrrolo[3,2-b]pyrrole-1-carbonyl]-3-methyl-butyl}-amide;

1460. 5-Phenyl-thiophene-2-carboxylic acid- {1-[4-(2-cyclohexyl-acetyl)-6-oxo-hexahydro-pyrrolo[3,2-*b*]pyrrole-1-carbonyl]-3-methyl-butyl}-amide;

1461. 5-Phenyl-thiophene-2-carboxylic acid-{3-methyl-1-[6-oxo-4-(pyrrolidine-2-carbonyl)-hexahydro-pyrrolo[3,2-*b*]pyrrole-1-carbonyl]-butyl}-amide;

1462. 5-Phenyl-thiophene-2-carboxylic acid- {1-[4-(1-Acetyl-pyrrolidine-2-carbonyl)-6-oxo-hexahydro-pyrrolo[3,2-*b*]pyrrole-1-carbonyl]-3-methyl-butyl}-amide;

1463. 5-Phenyl-thiophene-2-carboxylic acid- {3-methyl-1-[6-oxo-4-(piperidine-2-carbonyl)-hexahydro-pyrrolo[3,2-*b*]pyrrole-1-carbonyl]-butyl}-amide;

1464. 5-Phenyl-thiophene-2-carboxylic acid- {1-[4-(1-Acetyl-piperidine-2-carbonyl)-6-oxo-hexahydro-pyrrolo[3,2-*b*]pyrrole-1-carbonyl]-3-methyl-butyl}-amide;

1465. 5-Phenyl-thiophene-2-carboxylic acid-{3-methyl-1-[6-oxo-4-(tetrahydrofuran-2-carbonyl)-hexahydro-pyrrolo[3,2-*b*]pyrrole-1-carbonyl]-butyl}-amide;

1466. 5-Phenyl-thiophene-2-carboxylic acid-{1-[4-(1-Amino-cyclobutanecarbonyl)-6-oxo-hexahydro-pyrrolo[3,2-*b*]-pyrrole-1-carbonyl]-3-methyl-butyl}-amide;

1467. 5-Phenyl-thiophene-2-carboxylic acid- {1-[4-(1-Amino-cyclopentanecarbonyl)-6-oxo-hexahydro-pyrrolo[3,2-*b*]pyrrole-1-carbonyl]-3-methyl-butyl -amide;

1468. 5-Phenyl-thiophene-2-carboxylic acid-{1-[4-(1-Amino-cyclohexanecarbonyl)-6-oxo-hexahydro-pyrrolo[3,2-*b*]pyrrole-1-carbonyl]-3-methyl-butyl}-amide;

1469. 5-Phenyl-thiophene-2-carboxylic acid-{1-4-(1-Acetylamino-cyclobutanecarbonyl)-6-oxo-hexahydro-pyrrolo[3,2-*b*]pyrrole-1-carbonyl]-3-methyl-butyl}-amide;

1470. 5-Phenyl-thiophene-2-carboxylic acid-{1-[4-(1-Acetylamino-cyclopentanecarbonyl)-6-oxo-hexahydro-pyrrolo[3,2-*b*]pyrrole-1-carbonyl]-3-methyl-butyl}-amide;

1471. 5-Phenyl-thiophene-2-carboxylic acid-{1-[4-(1-Acetylamino-cyclohexanecarbonyl)-6-oxo-hexahydro-pyrrolo[3,2-*b*]pyrrole-1-carbonyl]-3-methyl-butyl}-amide;

1472. 5-Phenyl-thiophene-2-carboxylic acid-{1-[4-(1-hydroxy-cylobutanecarbonyl)-6-oxo-hexahydro-pyrrolo[3,2,*b*]pyrrole-1-carbonyl]-3-methyl-butyl}-amide;

1473. 5-Phenyl-thiophene-2-carboxylic acid-{1-[4-(1-hydroxy-cyclopentanecarbonyl)-6-oxo-hexahydro-pyrrolo[3,2-*b*]pyrrole-1-carbonyl]-3-methyl-butyl}-amide;

1474. 5-Phenyl-thiophene-2-carboxylic acid-{1-[4-(1-hydroxy-cyclohexanecarbonyl)-6-oxo-hexahydro-pyrrolo[3,2-*b*]pyrrole-1-carbonyl]-3-methyl-butyl}-amide;

1475. 5-Phenyl-thiophene-2-carboxylic acid-{1-[4-(1-methoxy-cyclobutanecarbonyl)-6-oxo-hexahydro-pyrrolo[3,2-*b*]pyrrole-1-carbonyl]-3-methyl-butyl}-amide;

1476. 5-Phenyl-thiophene-2-carboxylic acid-{1-[4-(1-methoxy-cyclopentanecarbonyl)-6-oxo-hexahydro-pyrrolo[3,2-*b*]pyrrole-1-carbonyl]-3-methyl-butyl}-amide;

1477. 5-Phenyl-thiophene-2-carboxylic acid-{1-[4-(1-methoxy-cyclohexanecarbonyl)-6-oxo-hexahydro-pyrrolo[3,2-*b*]pyrrole-1-carbonyl]-3-methyl-butyl}-amide;

1478. 5-Phenyl-thiophene-2-carboxylic acid-{1-[4-(2-Amino-2-cyclopentyl-acetyl)-6-oxo-hexahydro-pyrrolo[3,2-*b*]-pyrrole-1-carbonyl]-3-methyl-butyl}-amide;

1479. 5-Phenyl-thiophene-2-carboxylic acid-{1-[4-(2-Amino-2-cyclohexyl-acetyl)-6-oxo-hexahydro-pyrrolo[3,2-*b*]-pyrrole-1-carbonyl]-3-methyl-butyl}-amide;

1480. 5-Phenyl-thiophene-2-carboxylic acid-{1-[4-(2-Acetylamino-2-cydopentyl-acetyl)-6-oxo-hexahydro-pyrrolo[3,2-*b*]pyrrole-1-carbonyl]-3-methyl-butyl}-amide;

1481. 5-Phenyl-thiophene-2-carboxylic acid-{1-[4-(2-Acetylamino-2-cyclohexyl-acetyl)-6-oxo-hexahydro-pyrrolo[3,2-*b*]pyrrole-1-carbonyl]-3-methyl-butyl}-amide;

1482. 5-Phenyl-thiophene-2-carboxylic acid- {1-[4-(2-Acetylamino-propionyl)-6-oxo-hexahydro-pyrrolo[3,2-*b*]pyrrole-1-carbonyl]-3-methyl-butyl}-amide;

1483. 5-Phenyl-thiophene-2-carboxylic acid-{1-[4-(2-Acetylamino-acetyl)-6-oxo-hexahydro-pyrrolo[3,2-*b*]pyrrole-1-carbonyl]-3-methyl-butyl}-amide;

1484. 5-Phenyl-thiophene-2-carboxylic acid-(1-{4-[2-(Acetyl-methyl-amino)-acetyl]-6-oxo-hexahydro-pyrrolo[3,2-*b*]pyrrole-1-carbonyl}-3-methyl-butyl)-amide;

1485. 5-Phenyl-thiophene-2-carboxylic acid-{1-[4-(2-dimethylamino-acetyl)-6-oxo-hexahydro-pyrrolo[3,2-*b*]pyrrole-1-carbonyl]-3-methyl-butyl}-amide;

1486. 5-Phenyl-thiophene-2-carboxylic acid-{1-[4-(2-Amino-butyryl)-6-oxo-hexahydro-pyrrolo[3,2-*b*]pyrrole-1-carbonyl]-3-methyl-butyl}-amide;

1487. 5-Phenyl-thiophene-2-carboxylic acid-{1-[4-(2-Acetylamino-butyryl)-6-oxo-hexahydro-pyrrolo[3,2-*b*]pyrrole-1-carbonyl]-3-methyl-butyl}-amide;

1488. 5-Phenyl-thiophene-2-carboxylic acid-{1-[4-(2-amino-3-methyl-butyryl)-6-oxo-hexahydro-pyrrolo[3,2-*b*]pyrrole-1-carbonyl]-3-methyl-butyl}-amide;

1489. 5-Phenyl-thiophene-2-carboxylic acid-{1-[4-(2-Acetylamino-3-methylbutyryl)-6-oxo-hexahydro-pyrrolo[3,2-*b*]pyrrole-1-carbonyl]-3-methyl-butyl}-amide;

1490. 5-Phenyl-thiophene-2-carboxylic acid-{1-[4-(2-amino-3,3-dimethyl-butyryl)-6-oxo-hexahydro-pyrrolo[3,2-*b*]-pyrrole-1-carbonyl]-3-methyl-butyl}-amide;

1491. 5-Phenyl-thiophene-2-carboxylic acid-{1-[4-(2-Acetylamino-3,3-dimethyl-butyryl)-6-oxo-hexahydro-pyrrolo[3,2-*b*]pyrrole-1-carbonyl]-3-methyl-butyl}-amide;

1492. 5-Phenyl-thiophene-2-carboxylic acid-{1-[4-(2-Amino-3-methylpentanoyl)-6-oxo-hexahydro-pyrrolo[3,2-*b*]-pyrrole-1-carbonyl]-3-methyl-butyl}-amide;

1493. 5-Phenyl-thiophene-2-carboxylic acid-{1-[4-(2-Acetylamino-3-methyl-pentanoyl)-6-oxo-hexahydro-pyrrolo[3,2-*b*]pyrrole-1-carbonyl]-3-methyl-butyl}-amide;

1494. 5-Phenyl-thiophene-2-carboxylic acid-[3-methyl-1-(6-oxo-4-pentanoyl-hexahydro-pyrrolo[3,2-*b*]pyrrole-1-carbonyl)-butyl]-amide;

1495. 5-Phenyl-thiophene-2-carboxylic acid-{3-methyl-1-[4-(3-methylpentanoyl)-6-oxo-hexahydro-pyrrolo[3,2-*b*]-pyrrole-1-carbonyl]-butyl}-amide;

1496. 5-Phenyl-thiophene-2-carboxylic acid-{3-methyl-1-[4-(4-methylpentanoyl)-6-oxo-hexahydro-pyrrolo[3,2-*b*]-pyrrole-1-carbonyl]-butyl}-amide;

1497. 5-Phenyl-thiophene-2-carboxylic acid-{1-[4-(2-Amino-4-methylpentanoyl)-6-oxo-hexahydro-pyrrolo[3,2-*b*]-pyrrole-1-carbonyl]-3-methyl-butyl}-amide;

1498. 5-Phenyl-thiophene-2-carboxylic acid-{1-[4-(2-Acetylamino-4-methylpentanoyl)-6-oxo-hexahydro-pyrrolo[3,2-*b*]pyrrole-1-carbonyl]-3-methyl-butyl}-amide;

1499. 5-Phenyl-thiophene-2-carboxylic acid-{1-[4-(2-Amino-4,4-dimethyl-pentanoyl)-6-oxo-hexahydro-pyrrolo[3,2-*b*]pyrrole-1-carbonyl]-3-methyl-butyl}-amide;

1500. 5-Phenyl-thiophene-2-carboxylic acid-{1-[4-(2-Acetylamino-4,4-dimethyl-pentanoyl)-6-oxo-hexahydro-pyrrolo[3,2-*b*]pyrrole-1-carbonyl]-3-methyl-butyl}-amide;

1501. 5-Phenyl-thiophene-2-carboxylic acid-{1-[4-(2-Amino-pentanoyl)-6-oxo-hexahydro-pyrrolo[3,2-*b*]pyrrole-1-carbonyl]-3-methyl-butyl}-amide;

1502. 5-Phenyl-thiophene-2-carboxylic acid {1-[4-(2-Acetylamino-pentanoyl)-6-oxo-hexahydro-pyrrolo[3,2-*b*]pyrrole-1-carbonyl]-3-methyl-butyl}-amide;

1503. 5-Phenyl-thiophene-2-carboxylic acid-{1-[4-(2-Amino-5-methyl-hexanoyl)-6-oxo-hexahydro-pyrrolo[3,2-*b*]-pyrrole-1-carbonyl]-3-methyl-butyl}-amide;

1504. 5-Phenyl-thiophene-2-carboxylic acid-{1-[4-(2-Acetylamino-5-methyl-hexanoyl)-6-oxo-hexahydro-pyrrolo[3,2-*b*]pyrrole-1-carbonyl]-3-methyl-butyl}-amide;

1505. 5-Phenyl-thiophene-2-carboxylic acid-{1-[4-(2-hydroxy-3-methylbutyryl)-6-oxo-hexahydro-pyrrolo[3,2-*b*]pyrrole-1-carbonyl]-3-methyl-butyl}-amide;

1506. 5-Phenyl-thiophene-2-carboxylic acid-{1-[4-(2-methoxy-3-methylbutyryl)-6-oxo-hexahydro-pyrrolo[3,2-*b*]pyrrole-1-carbonyl]-3-methyl-butyl}-amide;

1507. 5-Phenyl-thiophene-2-carboxylic acid-{1-[4-(2-hydroxy-3,3-dimethyl-butyryl)-6-oxo-hexahydro-pyrrolo[3,2-*b*]pyrrole-1-carbonyl]-3-methyl-butyl}-amide;

1508. 5-Phenyl-thiophene-2-carboxylic acid-{1-[4-(2-methoxy-3,3-dimethyl-butyryl)-6-oxo-hexahydro-pyrrolo[3,2-*b*]pyrrole-1-carbonyl]-3-methyl-butyl}-amide;

1509. 5-Phenyl-thiophene-2-carboxylic acid-{1-[4-(2-hydroxy-3-methyl-pentanoyl)-6-oxo-hexahydro-pyrrolo[3,2-*b*]pyrrole-1-carbonyl]-3-methyl-butyl}-amide;

1510. 5-Phenyl-thiophene-2-carboxylic acid-{1-[4-(2-methoxy-3-methyl-pentanoyl)-6-oxo-hexahydro-pyrrolo[3,2-*b*]pyrrole-1-carbonyl]-3-methyl-butyl}-amide;

1511. 5-Phenyl-thiophene-2-carboxylic acid-{1-[4-(2-hydroxy-4-methyl-pentanoyl)-6-oxo-hexahydro-pyrrolo[3,2-*b*]pyrrole-1-carbonyl]-3-methyl-butyl}-amide;

1512. 5-Phenyl-thiophene-2-carboxylic acid-{1-[4-(2-methoxy-4-methyl-pentanoyl)-6-oxo-hexahydro-pyrrolo[3,2-*b*]pyrrole-1-carbonyl]-3-methyl-butyl}-amide;

1513. 5-Phenyl-thiophene-2-carboxylic acid-{1-[4-(2-hydroxy-4,4-dimethyl-pentanoyl)-6-oxo-hexahydro-pyrrolo[3,2-*b*]pyrrole-1-carbonyl]-3-methyl-butyl}-amide;

1514. 5-Phenyl-thiophene-2-carboxylic acid-{1-[4-(2-methoxy-4,4-dimethyl-pentanoyl)-6-oxo-hexahydro-pyrrolo[3,2-*b*]pyrrole-1-carbonyl]-3-methyl-butyl}-amide;

1515. 5-Phenyl-thiophene-2-carboxylic acid =-{1-[4-(2-hydroxy-pentanoyl)-6-oxo-hexahydro-pyrrolo[3,2-*b*]pyrrole-1-carbonyl]-3-methyl-butyl}-amide;

1516. 5-Phenyl-thiophene-2-carboxylic acid-{1-[4-(2-methoxy-pentanoyl)-6-oxo-hexahydro-pyrrolo[3,2-*b*]pyrrole-1-carbonyl]-3-methyl-butyl}-amide;

1517. 5-Phenyl-thiophene-2-carboxylic acid-{1-[4-(2-hydroxy-5-methyl-hexanoyl)-6-oxo-hexahydro-pyrrolo[3,2-*b*]-pyrrole-1-carbonyl]-3-methyl-butyl}-amide;

1518. 5-Phenyl-thiophene-2-carboxylic acid-{1-[4-(2-methoxy-5-methyl-hexanoyl)-6-oxo-hexahydro-pyrrolo[3,2-*b*]-pyrrole-1-carbonyl]-3-methyl-butyl}-amide;

1519. *N*-[1-(4-Benzoyl-6-oxo-hexahydro-pyrrolo[3,2-*b*]pyrrole-1-carbonyl)-3-methyl-butyl]-4-pyrrolidin-1-ylbenzamide;

1520. *N*-{3-methyl-1-[6-oxo-4-(pyridine-2-carbonyl)-hexahydro-pyrrolo[3,2-*b*]pyrrole-1-carbonyl]-butyl}-4-pyrrolidin-1-yl-benzamide;

1521. *N*-{3-methyl-1-[6-oxo-4-(1-oxy-pyridine-2-carbonyl)-hexahydro-pyrrolo[3,2-*b*]pyrrole-1-carbonyl]-butyl}-4-pyrrolidin-1-yl-benzamide;

1522. *N*-{3-methyl-1-[6-oxo-4-(pyridine-3-carbonyl)-hexahydro-pyrrolo[3,2-*b*]pyrrole-1-carbonyl]-butyl}-4-pyrrolidin-1-yl-benzamide;

1523. *N*-{3-methyl-1-[6-oxo-4-(1-oxy-pyridine-3-carbonyl)-hexahydro-pyrrolo[3,2-*b*]pyrrole-1-carbonyl]-butyl}-4-pyrrolidin-1-yl-benzamide;

1524. *N*-{3-methyl-1-[6-oxo-4-(pyridine-4-carbonyl)-hexahydro-pyrrolo[3,2-*b*]pyrrole-1-carbonyl]-butyl}-4-pyrrolidin-1-yl-benzamide;

1525. *N*-{3-methyl-1-[6-oxo-4-(1-oxy-pyridine-4-carbonyl)-hexahydro-pyrrolo[3,2-*b*]pyrrole-1-carbonyl]-butyl}-4-pyrrolidin-1-yl-benzamide;

1526. *N*-[1-(4-Benzenesulfonyl-6-oxo-hexahydro-pyrrolo[3,2-*b*]pyrrole-1-carbonyl)-3-methyl-butyl]-4-pyrrolidin-1-yl-benzamide;

1527. *N*-{3-methyl-1-[6-oxo-4-(pyridine-2-sulfonyl)-hexahydro-pyrrolo[3,2-*b*]pyrrole-1-carbonyl]-butyl}-4-pyrrolidin-1-yl-benzamide;

1528. *N*-{3-methyl-1-[6-oxo-4-(1-oxy-pyridine-2-sulfonyl)-hexahydro-pyrrolo[3,2-*b*]pyrrole-1-carbonyl]-butyl}-4-pyrrolidin-1-yl-benzamide;

1529. *N*-{3-methyl-1-[6-oxo-4-(pyridine-3-sulfonyl)-hexahydro-pyrrolo[3,2-*b*]pyrrole-1-carbonyl]-butyl}-4-pyrrolidin-1-yl-benzamide;

1530. *N*-{3-methyl-1-[6-oxo-4-(1-oxy-pyridine-3-sulfonyl)-hexahydro-pyrrolo[3,2-*b*]pyrrole-1-carbonyl]-butyl}-4-pyrrolidin-1-yl-benzamide;

1531. *N*-{3-methyl-1-[6-oxo-4-(pyridine-4-sulfonyl)-hexahydro-pyrrolo[3,2-*b*]pyrrole-1-carbonyl]-butyl}-4-pyrrolidin-1-yl-benzamide;

1532. *N*-{3-methyl-1-[6-oxo-4-(1-oxy-pyridine-4-sulfonyl)-hexahydro-pyrrolo[3,2-*b*]pyrrole-1-carbonyl]-butyl}-4-pyrrolidin-1-yl-benzamide;

1533. *N*-[3-methyl-1-(6-oxo-4-phenylacetyl-hexahydro-pynolo[3,2-*b*]pyrrole-1-carbonyl)-butyl]-4-pyrrolidin-1-yl-benzamide;

1534. 4-[2-(4-pyrrolidin-1-yl-benzoylamino)-4-methyl-pentanoyl]-3-oxo-hexahydro-pyrrolo[3,2-*b*]pyrrole-1-carboxylic acid phenylamide;

1535. 4-[2-(4-pyrrolidin-1-yl-benzoylamino)-4-methyl-pentanoyl]-3-oxo-hexahydro-pyrrolo[3,2-*b*]pyrrole-1-carboxylic acid isobutyl-amide;

1536. 4-[2-(4-pyrrolidin-1-yl-benzoylamino)-4-methyl-pentanoyl]-3-oxo-hexahydro-pyrrolo[3,2-*b*]pyrrole-1-carboxylic acid phenyl ester;

1537. 4-[2-(4-pyrrolidin-1-yl-benzoylamino)-4-methyl-pentanoyl]-3-oxo-hexahydro-pyrrolo[3,2-*b*]pyrrole-1-carboxylic acid isobutyl ester;

1538. *N*-{3-methyl-1-[6-oxo-4-(pyrrolidine-1-carbonyl)-hexahydro-pyrrolo[3,2-*b*]pyrrole-1-carbonyl]-butyl}-4-pyrrolidin-1-yl-benzamide;

1539. *N*-{3-methyl-1-[6-oxo-4-(piperidine-1-carbonyl)-hexahydro-pyrrolo[3,2-*b*]pyrrole-1-carbonyl]-butyl}-4-pyrrolidin-1-yl-benzamide;

1540. *N*-{3-methyl-1-[4-(morpholine-4-carbonyl)-6-oxo-hexahydro-pyrrolo[3,2-*b*]pyrrole-1-carbonyl]-butyl}-4-pyrrolidin-1-yl-benzamide;

1541. *N*-{3-methyl-1-[6-oxo-4-(piperazine-1-carbonyl)-hexahydro-pyrrolo[3,2-*b*]pyrrole-1-carbonyl]-butyl}-4-pyrrolidin-1-yl-benzamide;

1542. *N*-{3-methyl-1-[4-(4-methyl-piperazine-1-carbonyl)-6-oxo-hexahydro-pyrrolo[3,2-*b*]pyrrole-1-carbonyl]-butyl}-4-pyrrolidin-1-yl-benzamide;

1543. *N*-(3-methyl-1-{4-[2-(4-methyl-piperazin-1-yl)-acetyl]-6-oxo-hexahydro-pyrrolo[3,2-*b*]pyrrole-1-carbonyl}-butyl)-4-pyrrolidin-1-yl-benzamide;

1544. *N*-{3-methyl-1-[6-oxo-4-(2-piperazin-1-yl-acetyl)-hexahydro-pyrrolo[3,2-*b*]pyrrole-1-carbonyl]-butyl}-4-pyrrolidin-1-yl-benzamide;

1545. *N*-{1-[4-(furan-2-carbonyl)-6-oxo-hexahydro-pyrrolo[3,2-*b*]pyrrole-1-carbonyl]-3-methyl-butyl}-4-pyrrolidin-1-yl-benzamide;

1546. *N*-{1-[4-(5-chloro-furan-2-carbonyl)-6-oxo-hexahydro-pyrrolo[3,2-*b*]pyrrole-1-carbonyl]-3-methyl-butyl}-4-pyrrolidin-1-yl-benzamide;

1547. *N*-{1-[4-(thiophene-2-carbonyl)-6-oxo-hexahydro-pyrrolo[3,2-*b*]pyrrole-1-carbonyl]-3-methyl-butyl}-4-pyrrolidin-1-yl-benzamide;

1548. *N*-{1-[4-(5-chloro-thiophene-2-carbonyl)-6-oxo-hexahydro-pyrrolo[3,2-*b*]pyrrole-1-carbonyl]-3-methyl-butyl}-4-pyrrolidin-1-yl-benzamide;

1549. *N*-{1-[4-(furan-3-carbonyl)-6-oxo-hexahydro-pyrrolo[3,2-*b*]pyrrole-1-carbonyl]-3-methyl-butyl}-4-pyrrolidin-1-yl-benzamide;

1550. *N*-{1-[4-(thiophene-3-carbonyl)-6-oxo-hexahydro-pyrrolo[3,2-*b*]pyrrole-1-carbonyl]-3-methyl-butyl}-4-pyrrolidin-1-yl-benzamide;

1551. *N*-[1-(4-cyclopentanecarbonyl-6-oxo-hexahydro-pyrrolo[3,2-*b*]pyrrole-1-carbonyl)-3-methyl-butyl]-4-pyrrolidin-1-yl-benzamide;

1552. *N*-[1-(4-cyclohexanecarbonyl-6-oxo-hexahydro-pyrrolo[3,2-*b*]pyrrole-1-carbonyl)-3-methyl-butyl]-4-pyrrolidin-1-yl-benzamide;

1553. *N*-{1-[4-(cyclopent-3-enecarbonyl)-6-oxo-hexahydro-pyrrolo[3,2-*b*]pyrrole-1-carbonyl]-3-methyl-butyl}-4-pyrrolidin-1-yl-benzamide;

1554. *N*-{1-[4-(2-cylopentyl-acetyl)-6-oxo-hexahydro-pyrrolo[3,2-*b*]pyrrole-1-carbonyl]-3-methyl-butyl}-4-pyrrolidin-1-yl-benzamide;

1555. *N*-{1-[4-(2-cyclohexyl-acetyl)-6-oxo-hexahydro-pyrrolo[3,2-*b*]pyrrole-1-carbonyl]-3-methyl-butyl}-4-pyrrolidin-1-yl-benzamide;

1556. 4-Pyrrolidin-1-yl-*N*-{3-methyl-1-[6-oxo-4-(pyrrolidine-2-carbonyl)-hexahydro-pyrrolo[3,2-*b*]pyrrole-1-carbonyl]-butyl}-benzamide;

1557. *N*-{1-[4-(1-Acetyl-pyrrolidine-2-carbonyl)-6-oxo-hexahydro-pyrrolo[3,2-*b*]pyrrole-1-carbonyl]-3-methyl-butyl}-4-pyrrolidin-1-yl-benzamide;

1558. *N*-{3-methyl-1-[6-oxo-4-(piperidine-2-carbonyl)-hexahydro-pyrrolo[3,2-*b*]pyrrole-1-carbonyl]-butyl}-4-pyrrolidin-1-yl-benzamide;

1559. *N*-{1-[4-(1-Acetyl-piperidine-2-carbonyl)-6-oxo-hexahydro-pyrrolo[3,2-*b*]pyrrole-1-carbonyl]-3-methyl-butyl}-4-pyrrolidin-1-yl-benzamide;

1560. *N*-{3-methyl-1-[6-oxo-4-(tetrahydro-furan-2-carbonyl)-hexahydro-pyrrolo[3,2-*b*]pyrrole-1-carbonyl]-butyl}-4-pyrrolidin-1-yl-benzamide;

1561. *N*-{1-[4-(1-Amino-cylobutanecarbonyl)-6-oxo-hexahydro-pyrrolo[3,2-*b*]pyrrole-1-carbonyl]-3-methyl-butyl}-4-pyrrolidin-1-yl-benzamide;

1562. *N*-{1-[4-(1-Amino-cyclopentanecarbonyl)-6-oxo-hexahydro-pyrrolo[3,2-*b*]pyrrole-1-carbonyl]-3-methyl-butyl}-4-pyrrolidin-1-yl-benzamide;

1563. *N*-{1-[4-(1-Amino-cylohexanecarbonyl)-6-oxo-hexahydro-pyrrolo[3,2-*b*]pyrrole-1-carbonyl]-3-methyl-butyl}-4-pyrrolidin-1-yl-benzamide;

1564. *N*-{1-[4-(1-Acetylamino-cyclobutanecarbonyl)-6-oxo-hexahydro-pyrrolo[3,2-*b*]pyrrole-1-carbonyl]-3-methyl-butyl}-4-pyrrolidin-1-yl-benzamide;

1565. *N*-{1-[4-(1-Acetylamino-cyclopentanecarbonyl)-6-oxo-hexahydro-pyrrolo[3,2-*b*]pyrrole-1-carbonyl]-3-methyl-butyl}-4-pyrrolidin-1-yl-benzamide;

1566. *N*-{1-[4-(1-Acetylamino-cyclohexanecarbonyl)-6-oxo-hexahydro-pyrrolo[3,2-*b*]pyrrole-1-carbonyl]-3-methyl-butyl}-4-pyrrolidin-1-yl-benzamide;

1567. *N*-{1-[4-(1-hydroxy-cyclobutanecarbonyl)-6-oxo-hexahydro-pyrrolo[3,2-*b*]pyrrole-1-carbonyl]-3-methyl-butyl}-4-pyrrolidin-1-yl-benzamide;

1568. *N*-{1-[4-(1-hydroxy-cyclopentanecarbonyl)-6-oxo-hexahydro-pyrrolo[3,2-*b*]pyrrole-1-carbonyl]-3-methyl-butyl}-4-pyrrolidin-1-yl-benzamide;

1569. *N*-{1-[4-(1-hydroxy-cyclohexanecarbonyl)-6-oxo-hexahydro-pyrrolo[3,2-*b*]pyrrole-1-carbonyl]-3-methyl-butyl}-4-pyrrolidin-1-yl-benzamide;

1570. *N*-{1-[4-(1-methoxy-cyclobutanecarbonyl)-6-oxo-hexahydro-pyrrolo[3,2-*b*]pyrrole-1-carbonyl]-3-methyl-butyl}-4-pyrrolidin-1-yl-benzamide;

1571. *N*-{1-[4-(1-methoxy-cyclopentanecarbonyl)-6-oxo-hexahydro-pyrrolo[3,2-*b*]pyrrole-1-carbonyl]-3-methyl-butyl}-4-pyrrolidin-1-yl-benzamide;

1572. *N*-{1-[4-(1-methoxy-cydohexanecarbonyl)-6-oxo-hexahydro-pyrrolo[3,2-*b*]pyrrole-1-carbonyl]-3-methyl-butyl}-4-pyrrolidin-1-yl-benzamide;

1573. *N*-{1-[4-(2-Amino-2-cyclopentyl-acetyl)-6-oxo-hexahydro-pyrrolo[3,2-*b*]pyrrole-1-carbonyl]-3-methyl-butyl}-4-pyrrolidin-1-yl-benzamide;

1574. *N*-{1-[4-(2-Amino-2-cyclohexyl-acetyl)-6-oxo-hexahydro-pyrrolo[3,2-*b*]pyrrole-1-carbonyl]-3-methyl-butyl}-4-pyrrolidin-1-yl-benzamide;

1575. *N*-{1-[4-(2-Acetylamino-2-cyclopentyl-acetyl)-6-oxo-hexahydro-pyrrolo[3,2-*b*]pyrrole-1-carbonyl]-3-methyl-butyl}-4-pyrrolidin-1-yl-benzamide;

1576. *N*-{1-[4-(2-Acetylamino-2-cyclohexyl-acetyl)-6-oxo-hexahydro-pyrrolo[3,2-*b*]pyrrole-1-carbonyl]-3-methyl-butyl}-4-pyrrolidin-1-yl-benzamide;

1577. *N*-{1-[4-(2-Acetylamino-propionyl)-6-oxo-hexahydro-pyrrolo[3,2-*b*]pyrrole-1-carbonyl]-3-methyl-butyl}-4-pyrrolidin-1-yl-benzamide;

1578. *N*-{1-[4-(2-Acetylamino-acetyl)-6-oxo-hexahydro-pyrrolo[3,2-*b*]pyrrole-1-carbonyl]-3-methyl-butyl}-4-pyrrolidin-1-yl-benzamide;

1579. *N*-(1-{4-[2-(Acetyl-methyl-amino)-acetyl]-6-oxo-hexahydro-pyrrolo[3,2-*b*]pyrrole-1-carbonyl}-3-methyl-butyl)-4-pyrrolidin-1-yl-benzamide;

1580. *N*-{1-[4-(2-dimethylamino-acetyl)-6-oxo-hexahydro-pyrrolo[3,2-*b*]pyrrole-1-carbonyl]-3-methyl-butyl}-4-pyrrolidin-1-yl-benzamide;

1581. *N*-{1-[4-(2-Amino-butyryl)-6-oxo-hexahydro-pyrrolo[3,2-*b*]pyrrole-1-carbonyl]-3-methyl-butyl}-4-pyrrolidin-1-yl-benzamide;

1582. *N*-{1-[4-(2-Acetylamino-butyryl)-6-oxo-hexahydro-pyrrolo[3,2-*b*]pyrrole-1-carbonyl]-3-methyl-butyl}-4-pyrrolidin-1-yl-benzamide;

1583. *N*-{1-[4-(2-amino-3-methyl-butyryl)-6-oxo-hexahydro-pyrrolo[3,2-*b*]pyrrole-1-carbonyl]-3-methyl-butyl}-4-pyrrolidin-1-yl-benzamide;

1584. *N*-{1-[4-(2-Acetylamino-3-methyl-butyryl)-6-oxo-hexahydro-pyrrolo[3,2-*b*]pyrrole-1-carbonyl]-3-methyl-butyl}-4-pyrrolidin-1-yl-benzamide;

1585. *N*-{1-[4-(2-amino-3,3-dimethyl-butyryl)-6-oxo-hexahydro-pyrrolo[3,2-*b*]-pyrrole-1-carbonyl]-3-methyl-butyl}-4-pyrrolidin-1-yl-benzamide;

1586. *N*-{1-[4-(2-Acetylamino-3,3-dimethyl-butyryl)-6-oxo-hexahydro-pyrrolo[3,2-*b*]pyrrole-1-carbonyl]-3-methyl-butyl}-4-pyrrolidin-1-yl-benzamide;

1587. *N*-{1-[4-(2-Amino-3-methyl-pentanoyl)-6-oxo-hexahydro-pyrrolo[3,2-*b*]pyrrole-1-carbonyl]-3-methyl-butyl}-4-pyrrolidin-1-yl-benzamide;

1588. *N*-{1-[4-(2-Acetylamino-3-methyl-pentanoyl)-6-oxo-hexahydro-pyrrolo[3,2-*b*]pyrrole-1-carbonyl]-3-methyl-butyl}-4-pyrrolidin-1-yl-benzamide;

1589. *N*-[3-methyl-1-(6-oxo-4-pentanoyl-hexahydro-pyrrolo[3,2-*b*]pyrrole-1-carbonyl)-butyl]-4-pyrrolidin-1-yl-benzamide;

1590. *N*-{3-methyl-1-[4-(3-methyl-pentanoyl)-6-oxo-hexahydro-pyrrolo[3,2-*b*]pyrrole-1-carbonyl]-butyl}-4-pyrrolidin-1-yl-benzamide;

1591. *N*-{3-methyl-1-[4-(4-methyl-pentanoyl)-6-oxo-hexahydro-pyrrolo[3,2-*b*]pyrrole-1-carbonyl]-butyl}-4-pyrrolidin-1-yl-benzamide;

1592. *N*-{1-[4-(2-Amino-4-methyl-pentanoyl)-6-oxo-hexahydro-pyrrolo[3,2-*b*]pyrrole-1-carbonyl]-3-methyl-butyl}-4-pyrrolidin-1-yl-benzamide;

1593. *N*-{1-[4-(2-Acetylamino-4-methyl-pentanoyl)-6-oxo-hexahydro-pyrrolo[3,2-*b*]pyrrole-1-carbonyl]-3-methyl-butyl}-4-pyrrolidin-1-yl-benzamide;

1594. *N*-{1-[4-(2-Amino-4,4-dimethyl-pentanoyl)-6-oxo-hexahydro-pyrrolo[3,2-*b*]pyrrole-1-carbonyl]-3-methyl-butyl}-4-pyrrolidin-1-yl-benzamide;

1595. *N*-{1-[4-(2-Acetylamino-4,4-dimethyl-pentanoyl)-6-oxo-hexahydro-pyrrolo[3,2-*b*]pyrrole-1-carbonyl]-3-methyl-butyl}-4-pyrrolidin-1-yl-benzamide;

1596. *N*-{1-[4-(2-Amino-pentanoyl)-6-oxo-hexahydro-pyrrolo[3,2-*b*]pyrrole-1-carbonyl]-3-methyl-butyl}-4-pyrrolidin-1-yl-benzamide;

1597. *N*-{1-[4-(2-Acetylamino-pentanoyl)-6-oxo-hexahydro-pyrrolo[3,2-*b*]pyrrole-1-carbonyl]-3-methyl-butyl}-4-pyrrolidin-1-yl-benzamide;

1598. *N*-{1-[4-(2-Amino-5-methyl-hexanoyl)-6-oxo-hexahydro-pyrrolo[3,2-*b*]pyrrole-1-carbonyl]-3-methyl-butyl}-4-pyrrolidin-1-yl-benzamide;

1599. *N*-{1-[4-(2-Acetylamino-5-methyl-hexanoyl)-6-oxo-hexahydro-pyrrolo[3,2-*b*]pyrrole-1-carbonyl]-3-methyl-butyl}-4-pyrrolidin-1-yl-benzamide;

1600. *N*-{1-[4-(2-hydroxy-3-methyl-butyryl)-6-oxo-hexahydro-pyrrolo[3,2-*b*]pyrrole-1-carbonyl]-3-methyl-butyl}-4-pyrrolidin-1-yl-benzamide;

1601. *N*-{1-[4-(2-methoxy-3-methyl-butyryl)-6-oxo-hexahydro-pyrrolo[3,2-*b*]pyrrole-1-carbonyl]-3-methyl-butyl}-4-pyrrolidin-1-yl-benzamide;

1602. *N*-{1-[4-(2-hydroxy-3,3-dimethyl-butyryl)-6-oxo-hexahydro-pyrrolo[3,2-*b*]pyrrole-1-carbonyl]-3-methyl-butyl}-4-pyrrolidin-1-yl-benzamide;

1603. *N*-{1-[4-(2-methoxy-3,3-dimethyl-butyryl)-6-oxo-hexahydro-pyrrolo[3,2-*b*]pyrrole-1-carbonyl]-3-methyl-butyl}-4-pyrrolidin-1-yl-benzamide;

1604. *N*-{1-[4-(2-hydroxy-3-methyl-pentanoyl)-6-oxo-hexahydro-pyrrolo[3,2-*b*]pyrrole-1-carbonyl]-3-methyl-butyl}-4-pyrrolidin-1-yl-benzamide;

1605. *N*-{1-[4-(2-methoxy-3-methyl-pentanoyl)-6-oxo-hexahydro-pyrrolo[3,2-*b*]pyrrole-1-carbonyl]-3-methyl-butyl}-4-pyrrolidin-1-yl-benzamide;

1606. *N*-{1-[4-(2-hydroxy-4-methyl-pentanoyl)-6-oxo-hexahydro-pyrrolo[3,2-*b*]pyrrole-1-carbonyl]-3-methyl-butyl}-4-pyrrolidin-1-yl-benzamide;

1607. *N*-{1-[4-(2-methoxy-4-methyl-pentanoyl)-6-oxo-hexahydro-pyrrolo[3,2-*b*]pyrrole-1-carbonyl]-3-methyl-butyl}-4-pyrrolidin-1-yl-benzamide;

1608. *N*-{1-[4-(2-hydroxy-4,4-dimethyl-pentanoyl)-6-oxo-hexahydro-pyrrolo[3,2-*b*]pyrrole-1-carbonyl]-3-methyl-butyl}-4-pyrrolidin-1-yl-benzamide;

1609. *N*-{1-[4-(2-methoxy-4,4-dimethyl-pentanoyl)-6-oxo-hexahydro-pyrrolo[3,2-*b*]pyrrole-1-carbonyl]-3-methyl-butyl}-4-pyrrolidin-1-yl-benzamide;

1610. *N*-{1-[4-(2-hydroxy-pentanoyl)-6-oxo-hexahydro-pyrrolo[3,2-*b*]pyrrole-1-carbonyl]-3-methyl-butyl}-4-pyrrolidin-l-yl-benzamide;

1611. *N*-{1-[4-(2-methoxy-pentanoyl)-6-oxo-hexahydro-pyrrolo[3,2-*b*]pyrrole-1-carbonyl]-3-methyl-butyl}-4-pyrrolidin-1-yl-benzamide;

1612. *N*-{1-[4-(2-hydroxy-5-methyl-hexanoyl)-6-oxo-hexahydro-pyrrolo[3,2-*b*]pyrrole-1-carbonyl]-3-methyl-butyl}-4-pyrrolidin-1-yl-benzamide;

1613. *N*-{1-[4-(2-methoxy-5-methyl-hexanoyl)-6-oxo-hexahydro-pyrrolo[3,2-*b*]pyrrole-1-carbonyl]-3-methyl-butyl}-4-pyrrolidin-1-yl-benzamide;

1614. *N*-[1-(4-Benzoyl-6-oxo-hexahydro-pyrrolo[3,2-*b*]pyrrole-1-carbonyl)-3-methyl-butyl]-4-morpholin-4-ylbenzamide;

1615. *N*-{3-methyl-1-[6-oxo-4-(1-oxy-pyridine-2-carbonyl)-hexahydro-pyrrolo[3,2-*b*]pyrrole-1-carbonyl]-butyl}-4-morpholin-4-yl-benzamide;

1616. *N*-{3-methyl-1-[6-oxo-4-(pyridine-3-carbonyl)-hexahydro-pyrrolo[3,2-*b*]pyrrole-1-carbonyl]-butyl}-4-morpholin-4-yl-benzamide;

1617. *N*-{3-methyl-1-[6-oxo-4-(1-oxy-pyridine-3-carbonyl)-hexahydro-pyrrolo[3,2-*b*]pyrrole-1-carbonyl]-butyl}-4-morpholin-4-yl-benzamide;

1618. *N*-{3-methyl-1-[6-oxo-4-(pyridine-4-carbonyl)-hexahydro-pyrrolo[3,2-*b*]pyrrole-1-carbonyl]-butyl}-4-morpholin-4-yl-benzamide;

1619. *N*-{3-methyl-1-[6-oxo-4-(1-oxy-pyridine-4-carbonyl)-hexahydro-pyrrolo[3,2-*b*]pyrrole-1-carbonyl]-butyl}-4-morpholin-4-yl-benzamide;

1620. *N*-[1-(4-Benzenesulfonyl-6-oxo-hexahydro-pyrrolo[3,2-*b*]pyrrole-1-carbonyl)-3-methyl-butyl]-4-morpholin-4-yl-benzamide;

1621. *N*-{3-methyl-1-[6-oxo-4-(pyridine-4-sulfonyl)-hexahydro-pyrrolo[3,2-*b*]pyrrole-1-carbonyl]-butyl}-4-morpholin-4-yl-benzamide;

1622. *N*-{3-methyl-1-[6-oxo-4-(1-oxy-pyridine-4-sulfonyl)-hexahydro-pyrrolo[3,2-*b*]pyrrole-1-carbonyl]-butyl}-4-morpholin-4-yl-benzamide;

1623. *N*-[3-methyl-1-(6-oxo-4-phenylacetyl-hexahydro-pyrrolo[3,2-*b*]pyrrole-1-carbonyl)-butyl]-4-morpholin-4-yl-benzamide;

1624. 4-[2-(4-morpholin-4-yl-benzoylamino)-4-methyl-pentanoyl]-3-oxo-hexahydro-pyrrolo[3,2-*b*]pyrrole-1-carboxylic acid phenylamide;

1625. 4-[2-(4-morpholin-4-yl-benzoylamino)-4-methyl-pentanoyl]-3-oxo-hexahydro-pyrrolo[3,2-*b*]pyrrole-1-carboxylic acid isobutyl-amide;

1626. 4-[2-(4-morpholin-4-yl-benzoylamino)-4-methyl-pentanoyl]-3-oxo-hexahydro-pyrrolo[3,2-*b*]pyrrole-1-carboxylic acid phenyl ester;

1627. 4-[2-(4-morpholin-4-yl-benzoylamino)-4-methyl-pentanoyl]-3-oxo-hexahydro-pyrrolo[3,2-*b*]pyrrole-1-carboxylic acid isobutyl ester;

1628. *N*-{3-methyl-1-[6-oxo-4-(pyrrolidine-1-carbonyl)-hexahydro-pyrrolo[3,2-*b*]pyrrole-1-carbonyl]-butyl}-4-morpholin-4-yl-benzamide;

. 1629. *N*-{3-methyl-1-[6-oxo-4-(piperidine-1-carbonyl)-hexahydro-pyrrolo[3,2-*b*]pyrrole-1-carbonyl]-butyl}-4-morpholin-4-yl-benzamide;

. 1630. *N*-{3-methyl-1-[4-(morpholine-4-carbonyl)-6-oxo-hexahydro-pyrrolo[3,2-*b*]pyrrole-1-carbonyl]-butyl}-4-morpholin-4-yl-benzamide;

. 1631. *N*-{3-methyl-1-[6-oxo-4-(piperazine-1-carbonyl)-hexahydro-pyrrolo[3,2-*b*]pyrrole-1-carbonyl]-butyl}-4-morpholin-4-yl-benzamide;

1632. *N*-{3-methyl-1-[4-(4-methyl-piperazine-1-carbonyl)-6-oxo-hexahydro-pyrrolo[3,2-*b*]pyrrole-1-carbonyl]-butyl}-4-morpholin-4-yl-benzamide;

1633. *N*-(3-methyl-1-{4-[2-(4-methyl-piperazin-1-yl)-acetyl]-6-oxo-hexahydro-pyrrolo[3,2-*b*]pyrrole-1-carbonyl}-butyl)-4-morpholin-4-yl-benzamide;

1634. *N*-{3-methyl-1-[6-oxo-4-(2-piperazin-1-yl-acetyl)-hexahydro-pyrrolo[3,2-*b*]pyrrole-1-carbonyl]-butyl}-4-morpholin-4-yl-benzamide;

1635. *N*-{1-[4-(furan-2-carbonyl)-6-oxo-hexahydro-pyrrolo[3,2-*b*]pyrrole-1-carbonyl]-3-methyl-butyl}-4-morpholin-4-yl-benzamide;

1636. *N*-{1-[4-(5-chloro-furan-2-carbonyl)-6-oxo-hexahydro-pyrrolo[3,2-*b*]pyrrole-1-carbonyl]-3-methyl-butyl}-4-morpholin-4-yl-benzamide;

1637. *N*-{1-[4-(thiophene-2-carbonyl)-6-oxo-hexahydro-pyrrolo[3,2-*b*]pyrrole-1-carbonyl]-3-methyl-butyl}-4-morpholin-4-yl-benzamide;

1638. *N*-{1-[4-(5-chloro-thiophene-2-carbonyl)-6-oxo-hexahydro-pyrrolo[3,2-*b*]pyrrole-1-carbonyl]-3-methyl-butyl}-4-morpholin-4-yl-benzamide;

1639. *N*-{1-[4-(furan-3-carbonyl)-6-oxo-hexahydro-pyrrolo[3,2-*b*]pyrrole-1-carbonyl]-3-methyl-butyl}-4-morpholin-4-yl-benzamide;

1640. *N*-{1-[4-(thiophene-3-carbonyl)-6-oxo-hexahydro-pyrrolo[3,2-*b*]pyrrole-1-carbonyl]-3-methyl-butyl}-4-morpholin-4-yl-benzamide;

1641. *N*-[1-(4-cyclopentanecarbonyl-6-oxo-hexahydro-pyrrolo[3,2-*b*]pyrrole-1-carbonyl)-3-methyl-butyl]-4-morpholin-4-yl-benzamide;

1642. *N*-[1-(4-cyclohexanecarbonyl-6-oxo-hexahydro-pyrrolo[3,2-*b*]pyrrole-1-carbonyl)-3-methyl-butyl]-4-morpholin-4-yl-benzamide;

1643. *N*-{1-[4-(cyclopent-3-enecarbonyl)-6-oxo-hexahydro-pyrrolo[3,2-*b*]pyrrole-1-carbonyl]-3-methyl-butyl}-4-morpholin-4-yl-benzamide;

1644. *N*-{1-[4-(2-cyclopentyl-acetyl)-6-oxo-hexahydro-pyrrolo[3,2-*b*]pyrrole-1-carbonyl]-3-methyl-butyl}-4-morpholin-4-yl-benzamide;

1645. *N*-{1-[4-(2-cyclohexyl-acetyl)-6-oxo-hexahydro-pyrrolo[3,2-*b*]pyrrole-1-carbonyl]-3-methyl-butyl}-4-morpholin-4-yl-benzamide;

1646. 4-morpholin-4-yl-*N*-{3-methyl-1-[6-oxo-4-(pyrrolidine-2-carbonyl)-hexahydro-pyrrolo[3,2-*b*]pyrrole-1-carbonyl]-butyl}-benzamide;

1647. *N*-{1-[4-(1-Acetyl-pyrrolidine-2-carbonyl)-6-oxo-hexahydro-pyrrolo[3,2-*b*]pyrrole-1-carbonyl]-3-methyl-butyl}-4-mor pholin-4-yl-benzamide;

1648. *N*-{3-methyl-1-[6-oxo-4-(piperidine-2-carbonyl)-hexahydro-pyrrolo[3,2-*b*]pyrrole-1-carbonyl]-butyl}-4-morpholin-4-yl-benzamide;

1649. *N*-{1-[4-(1-Acetyl-piperidine-2-carbonyl)-6-oxo-hexahydro-pyrrolo[3,2-*b*]pyrrole-1-carbonyl]-3-methyl-butyl}-4-morpholin-4-yl-benzamide;

1650. *N*-{3-methyl-1-[6-oxo-4-(tetrahydro-furan-2-carbonyl)-hexahydro-pyrrolo[3,2-*b*]pyrrole-1-carbonyl]-butyl}-4-morpholin-4-yl-benzamide;

1651. *N*-{1-[4-(1-Amino-cyclobutanecarbonyl)-6-oxo-hexahydro-pyrrolo[3,2-*b*]pyrrole-1-carbonyl]-3-methyl-butyl}-4-morpholin-4-yl-benzamide;

1652. *N*-{1-[4-(1-Amino-cyclopentanecarbonyl)-6-oxo-hexahydro-pyrrolo[3,2-*b*]pyrrole-1-carbonyl]-3-methyl-butyl}-4-morpholin-4-yl-benzamide;

1653. *N*-{1-[4-(1-Amino-cyclohexanecarbonyl)-6-oxo-hexahydro-pyrrolo[3,2-*b*]pyrrole-1-carbonyl]-3-methyl-butyl}-4-morpholin-4-yl-benzamide;

1654. *N*-{1-[4-(1-Acetylamino-cyclobutanecarbonyl)-6-oxo-hexahydro-pyrrolo[3,2-*b*]pyrrole-1-carbonyl]-3-methyl-butyl}-4-morpholin-4-yl-benzamide;

1655. *N*-{1-[4-(1-Acetylamino-cyclopentanecarbonyl)-6-oxo-hexahydro-pyrrolo[3,2-*b*]pyrrole-1-carbonyl]-3-methyl-butyl}-4-morpholin-4-yl-benzamide;

1656. *N*-{1-[4-(1-Acetylamino-cyclohexanecarbonyl)-6-oxo-hexahydro-pyrrolo[3,2-*b*]pyrrole-1-carbonyl]-3-methyl-butyl}-4-morpholin-4-yl-benzamide;

1657. *N*-{1-[4-(1-hydroxy-cyclobutanecarbonyl)-6-oxo-hexahydro-pyrrolo[3,2-*b*]pyrrole-1-carbonyl]-3-methyl-butyl}-4-morpholin-4-yl-benzamide;

1658. *N*-{1-[4-(1-hydroxy-cyclopentanecarbonyl)-6-oxo-hexahydro-pyrrolo[3,2-*b*]pyrrole-1-carbonyl]-3-methyl-butyl}-4-morpholin-4-yl-benzamide;

1659. *N*-{1-[4-(1-hydroxy-cyclohexanecarbonyl)-6-oxo-hexahydro-pyrrolo[3,2-*b*]pyrrole-1-carbonyl]-3-methyl-butyl}-4-morpholin-4-yl-benzamide;

1660. *N*-{1-[4-(1-methoxy-cyclobutanecarbonyl)-6-oxo-hexahydro-pyrrolo[3,2-*b*]pyrrole-1-carbonyl]-3-methyl-butyl}-4-morpholin-4-yl-benzamide;

1661. *N*-{1-[4-(1-methoxy-cyclopentanecarbonyl)-6-oxo-hexahydro-pyrrolo[3,2-*b*]pyrrole-1-carbonyl]-3-methyl-butyl}-4-morpholin-4-yl-benzamide;

1662. *N*-{1-[4-(1-methoxy-cyclohexanecarbonyl)-6-oxo-hexahydro-pyrrolo[3,2-*b*]pyrrole-1-carbonyl]-3-methyl-butyl}-4-morpholin-4-yl-benzamide;

1663. *N*-{1-[4-(2-Amino-2-cyclopentyl-acetyl)-6-oxo-hexahydro-pyrrolo[3,2-*b*]pyrrole-1-carbonyl]-3-methyl-butyl}-4-morpholin-4-yl-benzamide;

1664. *N*-{1-[4-(2-Amino-2-cyclohexyl-acetyl)-6-oxo-hexahydro-pyrrolo[3,2-*b*]pyrrole-1-carbonyl]-3-methyl-butyl}-4-morpholin-4-yl-benzamide;

1665. *N*-{1-[4-(2-Acetylamino-2-cyclopentyl-acetyl)-6-oxo-hexahydro-pyrrolo[3,2-*b*]pyrrole-1-carbonyl]-3-methyl-butyl}-4-morpholin-4-yl-benzamide;

1666. *N*-{1-[4-(2-Acetylamino-2-cyclohexyl-acetyl)-6-oxo-hexahydro-pyrrolo[3,2-*b*]pyrrole-1-carbonyl]-3-methyl-butyl}-4-morpholin-4-yl-benzamide;

1667. *N*-{1-[4-(2-Acetylamino-propionyl)-6-oxo-hexahydro-pyrrolo[3,2-*b*]pyrrole-1-carbonyl]-3-methyl-butyl}-4-morpholin-4-yl-benzamide;

1668. *N*-{1-[4-(2-Acetylamino-acetyl)-6-oxo-hexahydro-pyrrolo[3,2-*b*]pyrrole-1-carbonyl]-3-methyl-butyl}-4-morpholin-4-yl-benzamide;

1669. *N*-(1-{4-[2-(Acetyl-methyl-amino)-acetyl]-6-oxo-hexahydro-pyrrolo[3,2-*b*]pyrrole-1-carbonyl}-3-methyl-butyl)-4-morpholin-4-yl-benzamide;

1670. *N*-{1-[4-(2-dimethylamino-acetyl)-6-oxo-hexahydro-pyrrolo[3,2-*b*]pyrrole-1-carbonyl]-3-methyl-butyl}-4-morpholin-4-yl-benzamide;

1671. *N*-{1-[4-(2-Amino-butyryl)-6-oxo-hexahydro-pyrrolo[3,2-*b*]pyrrole-1-carbonyl]-3-methyl-butyl}-4-morpholin-4-yl-benzamide;

1672. *N*-{1-[4-(2-Acetylamino-butyryl)-6-oxo-hexahydro-pyrrolo[3,2-*b*]pyrrole-1-carbonyl]-3-methyl-butyl}-4-morpholin-4-yl-benzamide;

1673. *N*-{1-[4-(2-amino-3-methyl-butyryl)-6-oxo-hexahydro-pyrrolo[3,2-*b*]pyrrole-1-carbonyl]-3-methyl-butyl}-4-morpholin-4-yl-benzamide;

1674. *N*-{1-[4-(2-Acetylamino-3-methyl-butyryl)-6-oxo-hexahydro-pyrrolo[3,2-*b*]pyrrole-1-carbonyl]-3-methyl-butyl}-4-morpholin-4-yl-benzamide;

1675. *N*-{1-[4-(2-amino-3,3-dimethyl-butyryl)-6-oxo-hexahydro-pyrrolo[3,2-*b*]pyrrole-1-carbonyl]-3-methyl-butyl}-4-morpholin-4-yl-benzamide;

1676. *N*-{1-[4-(2-Acetylamino-3,3-dimethyl-butyryl)-6-oxo-hexahydro-pyrrolo[3,2-*b*]pyrrole-1-carbonyl]-3-methyl-butyl}-4-morpholin-4-yl-benzamide;

1677. *N*-{1-[4-(2-Amino-3-methyl-pentanoyl)-6-oxo-hexahydro-pyrrolo[3,2-*b*]pyrrole-1-carbonyl]-3-methyl-butyl}-4-morpholin-4-yl-benzamide;

1678. *N*-{1-[4-(2-Acetylamino-3-methyl-pentanoyl)-6-oxo-hexahydro-pyrrolo[3,2-*b*]pyrrole-1-carbonyl]-3-methyl-butyl}-4-morpholin-4-yl-benzamide;

1679. *N*-[3-methyl-1-(6-oxo-4-pentanoyl-hexahydro-pyrrolo[3,2-*b*]pyrrole-1-carbonyl)-butyl]-4-morpholin-4-yl-benzamide;

1680. *N*-{3-methyl-1-[4-(3-methyl-pentanoyl)-6-oxo-hexahydro-pyrrolo[3,2-*b*]pyrrole-1-carbonyl]-butyl}-4-morpholin-4-yl-benzamide;

1681. *N*-{3-methyl-1-[4-(4-methyl-pentanoyl)-6-oxo-hexahydro-pyrrolo[3,2-*b*]pyrrole-1-carbonyl]-butyl}-4-morpholin-4-yl-benzamide;

1682. *N*-{1-[4-(2-Amino-4-methyl-pentanoyl)-6-oxo-hexahydro-pyrrolo[3,2-*b*]pyrrole-1-carbonyl]-3-methyl-butyl}-4-morpholin-4-yl-benzamide;

1683. *N*-{1-[4-(2-Acetylamino-4-methyl-pentanoyl)-6-oxo-hexahydro-pyrrolo[3,2-*b*]pyrrole-1-carbonyl]-3-methyl-butyl}-4-morpholin-4-yl-benzamide;

1684. *N*-{1-[4-(2-Amino-4,4-dimethyl-pentanoyl)-6-oxo-hexahydro-pyrrolo[3,2-*b*]pyrrole-1-carbonyl]-3-methyl-butyl}-4-morpholin-4-yl-benzamide;

1685. *N*-{1-[4-(2-Acetylamino-4,4-dimethyl-pentanoyl)-6-oxo-hexahydro-pyrrolo[3,2-*b*]pyrrole-1-carbonyl]-3-methyl-butyl}-4-morpholin-4-yl-benzamide;

1686. *N*-{1-[4-(2-Amino-pentanoyl)-6-oxo-hexahydro-pyrrolo[3,2-*b*]pyrrole-1-carbonyl]-3-methyl-butyl}-4-morpholin-4-yl-benzamide;

1687. *N*-{1-[4-(2-Acetylamino-pentanoyl)-6-oxo-hexahydro-pyrrolo[3,2-*b*]pyrrole-1-carbonyl]-3-methyl-butyl}-4-morpholin-4-yl-benzamide;

1688. *N*-{1-[4-(2-Amino-5-methyl-hexanoyl)-6-oxo-hexahydro-pyrrolo[3,2-*b*]pyrrole-1-carbonyl]-3-methyl-butyl}-4-morpholin-4-yl-benzamide;

1689. *N*-{1-[4-(2-Acetylamino-5-methyl-hexanoyl)-6-oxo-hexahydro-pyrrolo[3,2-*b*]pyrrole-1-carbonyl]-3-methyl-butyl}-4-morpholin-4-yl-benzamide;

1690. *N*-{1-[4-(2-hydroxy-3-methyl-butyryl)-6-oxo-hexahydro-pyrrolo[3,2-*b*]pyrrole-1-carbonyl]-3-methyl-butyl}-4-morpholin-4-yl-benzamide;

1691. *N*-{1-[4-(2-methoxy-3-methyl-butyryl)-6-oxo-hexahydro-pyrrolo[3,2-*b*]pyrrole-1-carbonyl]-3-methyl-butyl}-4-morpholin-4-yl-benzamide;

1692. *N*-{1-[4-(2-hydroxy-3,3-dimethyl-butyryl)-6-oxo-hexahydro-pyrrolo[3,2-*b*]pyrrole-1-carbonyl]-3-methyl-butyl}-4-morpholin-4-yl-benzamide;

1693. *N*-{1-[4-(2-methoxy-3,3-dimethyl-butyryl)-6-oxo-hexahydro-pyrrolo[3,2-*b*]pyrrole-1-carbonyl]-3-methyl-butyl}-4-morpholin-4-yl-benzamide;

1694. *N*-{1-[4-(2-hydroxy-3-methyl-pentanoyl)-6-oxo-hexahydro-pyrrolo[3,2-*b*]pyrrole-1-carbonyl]-3-methyl-butyl}-4-morpholin-4-yl-benzamide;

1695. *N*-{1-[4-(2-methoxy-3-methyl-pentanoyl)-6-oxo-hexahydro-pyrrolo[3,2-*b*]pyrrole-1-carbonyl]-3-methyl-butyl}-4-morpholin-4-yl-benzamide;

1696. *N*-{1-[4-(2-hydroxy-4-methyl-pentanoyl)-6-oxo-hexahydro-pyrrolo[3,2-*b*]pyrrole-1-carbonyl]-3-methyl-butyl}-4-morpholin-4-yl-benzamide;

1697. *N*-{1-[4-(2-methoxy-4-methyl-pentanoyl)-6-oxo-hexahydro-pyrrolo[3,2-*b*]pyrrole-1-carbonyl]-3-methyl-butyl}-4-morpholin-4-yl-benzamide;

1698. *N*-{1-[4-(2-hydroxy-4,4-dimethyl-pentanoyl)-6-oxo-hexahydro-pyrrolo[3,2-*b*]pyrrole-1-carbonyl]-3-methyl-butyl}-4-morpholin-4-yl-benzamide;

1699. *N*-{1-[4-(2-methoxy-4,4-dimethyl-pentanoyl)-6-oxo-hexahydro-pyrrolo[3,2-*b*]pyrrole-1-carbonyl]-3-methyl-butyl}-4-morpholin-4-yl-benzamide;

1700. *N*-{1-[4-(2-hydroxy-pentanoyl)-6-oxo-hexahydro-pyrrolo[3,2-*b*]pyrrole-1-carbonyl]-3-methyl-butyl}-4-morpholin-4-yl-benzamide;

1701. *N*-{1-[4-(2-methoxy-pentanoyl)-6-oxo-hexahydro-pyrrolo[3,2-*b*]pyrrole-1-carbonyl]-3-methyl-butyl}-4-morpholin-4-yl-benzamide;

1702. *N*-{1-[4-(2-hydroxy-5-methyl-hexanoyl)-6-oxo-hexahydro-pyrrolo[3,2-*b*]pyrrole-1-carbonyl]-3-methyl-butyl}-4-morpholin-4-yl-benzamide;

1703. *N*-{1-[4-(2-methoxy-5-methyl-hexanoyl)-6-oxo-hexahydro-pyrrolo[3,2-*b*]pyrrole-1-carbonyl]-3-methyl-butyl}-4-morpholin-4-yl-benzamide;

1704. *N*-[1-(4-Benzoyl-6-oxo-hexahydro-pyrrolo[3,2-*b*]pyrrole-1-carbonyl)-3-methyl-butyl]-4-piperazin-1-ylbenzamide;

1705. *N*-{3-methyl-1-[6-oxo-4-(pyridine-2-carbonyl)-hexahydro-pyrrolo[3,2-*b*]pyrrole-1-carbonyl]-butyl}-4-piperazin-1-yl-benzamide;

1706. *N*-{3-methyl-1-[6-oxo-4-(1-oxy-pyridine-2-carbonyl)-hexahydro-pyrrolo[3,2-*b*]pyrrole-1-carbonyl]-butyl}-4-piperazin-1-yl-benzamide;

1707. *N*-{3-methyl-1-[6-oxo-4-(pyridine-3-carbonyl)-hexahydro-pyrrolo[3,2-*b*]pyrrole-1-carbonyl]-butyl}-4-piperazin-1-yl-benzamide;

1708. *N*-{3-methyl-1-[6-oxo-4-(1-oxy-pyridine-3-carbonyl)-hexahydro-pyrrolo[3,2-*b*]pyrrole-1-carbonyl]-butyl}-4-piperazin-1-yl-benzamide;

1709. *N*-{3-methyl-1-[6-oxo-4-(pyridine-4-carbonyl)-hexahydro-pyrrolo[3,2-*b*]pyrrole-1-carbonyl]-butyl}-4-piperazin-1-yl-benzamide;

1710. *N*-{3-methyl-1-[6-oxo-4-(1-oxy-pyridine-4-carbonyl)-hexahydro-pyrrolo[3,2-*b*]pyrrole-1-carbonyl]-butyl}-4-piperazin-1-yl-benzamide;

1711. *N*-[1-(4-Benzenesulfonyl-6-oxo-hexahydro-pynolo[3,2-*b*]pyrrole-1-carbonyl)-3-methyl-butyl]-4-piperazin-1-yl-benzamide;

1712. *N*-{3-methyl-1-[6-oxo-4-(pyridine-2-sulfonyl)-hexahydro-pyrrolo[3,2-*b*]pyrrole-1-carbonyl]-butyl}-4-piperazin-1-yl-benzamide;

1713. *N*-{3-methyl-1-[6-oxo-4-(1-oxy-pyridine-2-sulfonyl)-hexahydro-pyrrolo[3,2-*b*]pyrrole-1-carbonyl]-butyl}-4- piperazin-1-yl-benzamide;

1714. *N*-{3-methyl-1-[6-oxo-4-(pyridine-3-sulfonyl)-hexahydro-pyrrolo[3,2-*b*]pyrrole-1-carbonyl]-butyl}-4-piperazin-1-yl-benzamide;

1715. *N*-{3-methyl-1-[6-oxo-4-(1-oxy-pyridine-3-sulfonyl)-hexahydro-pyrrolo[3,2-*b*]pyrrole-1-carbonyl]-butyl}-4-piperazin-1-yl-benzamide;

1716. *N*-{3-methyl-1-[6-oxo-4-(pyridine-4-sulfonyl)-hexahydro-pyrrolo[3,2-*b*]pyrrole-1-carbonyl]-butyl}-4-piperazin-1-yl-benzamide;

1717. *N*-{3-methyl-1-[6-oxo-4-(1-oxy-pyridine-4-sulfonyl)-hexahydro-pyrrolo[3,2-*b*]pyrrole-1-carbonyl]-butyl}-4-piperazin-1-yl-benzamide;

1718. *N*-[3-methyl-1-(6-oxo-4-phenylacetyl-hexahydro-pyrrolo[3,2-*b*]pyrrole-1-carbonyl)-butyl]-4-piperazin-1-yl-benzamide;

1719. 4-[2-(4-piperazin-1-yl-benzoylamino)-4-methyl-pentanoyl]-3-oxo-hexahydro-pyrrolo[3,2-*b*]pyrrole-1-carboxylic acid phenylamide;

1720. 4-[2-(4-piperazin-1-yl-benzoylamino)-4-methyl-pentanoyl]-3-oxo-hexahydro-pyrrolo[3,2-*b*]pyrrole-1-carboxylic acid isobutyl-amide;

1721. 4-[2-(4-piperazin-1-yl-benzoylamino)-4-methyl-pentanoyl]-3-oxo-hexahydro-pyrrolo[3,2-*b*]pyrrole-1-carboxylic acid phenyl ester;

1722. 4-[2-(4-piperazin-1-yl-benzoylamino)-4-methyl-pentanoyl]-3-oxo-hexahydro-pyrrolo[3,2-*b*]pyrrole-1-carboxylic acid isobutyl ester;

1723. *N*-{3-methyl-1-[6-oxo-4-(pyrrolidine-1-carbonyl)-hexahydro-pyrrolo[3,2-*b*]pyrrole-1-carbonyl]-butyl}-4-piperazin-1-yl-benzamide;

1724. *N*-{3-methyl-1-[6-oxo-4-(piperidine-1-carbonyl)-hexahydro-pyrrolo[3,2-*b*]pyrrole-1-carbonyl]-butyl}-4-piperazin-1-yl-benzamide;

1725. *N*-{3-methyl-1-[4-(morpholine-4-carbonyl)-6-oxo-hexahydro-pyrrolo[3,2-*b*]pyrrole-1-carbonyl]-butyl}-4-piperazin-1-yl-benzamide;

1726. *N*-{3-methyl-1-[6-oxo-4-(piperazine-1-carbonyl)-hexahydro-pyrrolo[3,2-*b*]pyrrole-1-carbonyl]-butyl}-4-piperazin-1-yl-benzamide;

1727. *N*-{3-methyl-1-[4-(4-methyl-piperazine-1-carbonyl)-6-oxo-hexahydro-pyrrolo[3,2-*b*]pyrrole-1-carbonyl]-butyl}-4-piperazin-1-yl-benzamide;

1728. *N*-(3-methyl-1-{4-[2-(4-methyl-piperazin-1-yl)-acetyl]-6-oxo-hexahydro-pyrrolo[3,2-*b*]pyrrole-1-carbonyl}-butyl)-4-piperazin-1-yl-benzamide;

1729. *N*-{3-methyl-1-[6-oxo-4-(2-piperazin-1-yl-acetyl)-hexahydro-pyrrolo[3,2-*b*]pyrrole-1-carbonyl]-butyl}-4-piperazin-1-yl-benzamide;

1730. *N*-{1-[4-(furan-2-carbonyl)-6-oxo-hexahydro-pyrrolo[3,2-*b*]pyrrole-1-carbonyl]-3-methyl-butyl}-4-piperazin-1-yl-benzamide;

1731. *N*-{1-[4-(5-chloro-furan-2-carbonyl)-6-oxo-hexahydro-pyrrolo[3,2-*b*]pyrrole-1-carbonyl]-3-methyl-butyl}-4-piperazin-1-yl-benzamide;

1732. *N*-{1-[4-(thiophene-2-carbonyl)-6-oxo-hexahydro-pyrrolo[3,2-*b*]pyrrole-1-carbonyl]-3-methyl-butyl}-4-piperazin-1-yl-benzamide;

1733. *N*-{1-[4-(5-chloro-thiophene-2-carbonyl)-6-oxo-hexahydro-pyrrolo[3,2-*b*]pyrrole-1-carbonyl]-3-methyl-butyl}-4-piperazin-1-yl-benzamide;

1734. *N*-{1-[4-(furan-3-carbonyl)-6-oxo-hexahydro-pyrrolo[3,2-*b*]pyrrole-1-carbonyl]-3-methyl-butyl}-4-piperazin-1-yl-benzamide;

1735. *N*-{1-[4-(thiophene-3-carbonyl)-6-oxo-hexahydro-pyrrolo[3,2-*b*]pyrrole-1-carbonyl]-3-methyl-butyl}-4-piperazin-1-yl-benzamide;

1736. *N*-[1-(4-cyclopentanecarbonyl-6-oxo-hexahydro-pyrrolo[3,2-*b*]pyrrole-1-carbonyl)-3-methyl-butyl]-4-piperazin-1-yl-benzamide;

1737. *N*-[1-(4-cyclohexanecarbonyl-6-oxo-hexahydro-pyrrolo[3,2-*b*]pyrrole-1-carbonyl)-3-methyl-butyl]-4-piperazin-1-yl-benzamide;

1738. *N*-{1-[-4-(cyclopent-3-enecarbonyl)-6-oxo-hexahydro-pyrrolo[3,2-*b*]pyrrole-1-carbonyl]-3-methyl-butyl}-4-piperazin-1-yl-benzamide;

1739. *N*-{1-[4-(2-cyclopentyl-acetyl)-6-oxo-hexahydro-pyrrolo[3,2-*b*]pyrrole-1-carbonyl]-3-methyl-butyl}-4-piperazin-1-yl-benzamide;

1740. *N*-{1-[4-(2-cyclohexyl-acetyl)-6-oxo-hexahydro-pyrrolo[3,2-*b*]pyrrole-1-carbonyl]-3-methyl-butyl}-4-piperazin-1-yl-benzamide;

. 1741. 4-piperazin-1-yl-*N*-{3-methyl-1-[6-oxo-4-(pyrrolidine-2-carbonyl)-hexahydro-pyrrolo[3,2-*b*]pyrrole-1-carbonyl]-butyl}-benzamide;

1742. *N*-{1-[4-(1-Acetyl-pyrrolidine-2-carbonyl)-6-oxo-hexahydro-pyrrolo[3,2-*b*]pyrrole-1-carbonyl]-3-methyl-butyl}-4-piperazin-1-yl-benzamide;

1743. *N*-{3-methyl-1-[6-oxo-4-(piperidine-2-carbonyl)-hexahydro-pyrrolo[3,2-*b*]pyrrole-1-carbonyl]-butyl}-4-piperazin-1-yl-benzamide;

1744. *N*-{1-[4-(1-Acetyl-piperidine-2-carbonyl)-6-oxo-hexahydro-pyrrolo[3,2-*b*]pyrrole-1-carbonyl]-3-methyl-butyl}-4-piperazin-1-yl-benzamide;

1745. *N*-{3-methyl-1-[6-oxo-4-(tetrahydro-furan-2-carbonyl)-hexahydro-pyrrolo[3,2-*b*]pyrrole-1-carbonyl]-butyl}-4-piperazin-1-yl-benzamide;

1746. *N*-{1-[4-(1-Amino-cyclobutanecarbonyl)-6-oxo-hexahydro-pyrrolo[3,2-*b*]pyrrole-1-carbonyl]-3-methyl-butyl}-4-piperazin-1-yl-benzamide;

1747. *N*-{1-[4-(1-Amino-cyclopentanecarbonyl)-6-oxo-hexahydro-pyrrolo[3,2-*b*]pyrrole-1-carbonyl]-3-methyl-butyl}-4-piperazin-1-yl-benzamide;

1748. *N*-{1-[4-(1-Amino-cyclohexanecarbonyl)-6-oxo-hexahydro-pyrrolo[3,2-*b*]pyrrole-1-carbonyl]-3-methyl-butyl}-4-piperazin-1-yl-benzamide;

1749. *N*-{1-[4-(1-Acetylamino-cyclobutanecarbonyl)-6-oxo-hexahydro-pyrrolo[3,2-*b*]pyrrole-1-carbonyl]-3-methyl-butyl}-4-piperazin-1-yl-benzamide;

1750. *N*-{1-[4-(1-Acetylamino-cydopentanecarbonyl)-6-oxo-hexahydro-pyrrolo[3,2-*b*]pyrrole-1-carbonyl]-3-methyl-butyl}-4-piperazin-1-yl-benzamide;

1751. *N*-{1-[4-(1-Acetylamino-cyclohexanecarbonyl)-6-oxo-hexahydro-pyrrolo[3,2-*b*]pyrrole-1-carbonyl]-3-methyl-butyl}-4-piperazin-1-yl-benzamide;

1752. *N*-{1-[4-(1-hydroxy-cyclobutanecarbonyl)-6-oxo-hexahydro-pyrrolo[3,2-*b*]pyrrole-1-carbonyl]-3-methyl-butyl}-4-piperazin-1-yl-benzamide;

1753. *N*-{1-[4-(1-hydroxy-cyclopentanecarbonyl)-6-oxo-hexahydro-pyrrolo[3,2-*b*]pyrrole-1-carbonyl]-3-methyl-butyl}-4-piperazin-1-yl-benzamide;

1754. *N*-{1-[4-(1-hydroxy-cyclohexanecarbonyl)-6-oxo-hexahydro-pyrrolo[3,2-*b*]pyrrole-1-carbonyl]-3-methyl-butyl}-4-piperazin-1-yl-benzamide;

1755. *N*-{1-[4-(1-methoxy-cyclobutanecarbonyl)-6-oxo-hexahydro-pyrrolo[3,2-*b*]pyrrole-1-carbonyl]-3-methyl-butyl}-4-piperazin-1-yl-benzamide;

1756. *N*-{1-[4-(1-methoxy-cyclopentanecarbonyl)-6-oxo-hexahydro-pyrrolo[3,2-*b*]pyrrole-1-carbonyl]-3-methyl-butyl}-4-piperazin-1-yl-benzamide;

1757. *N*-{1-[4-(1-methoxy-cyclohexanecarbonyl)-6-oxo-hexahydro-pyrrolo[3,2-*b*]pyrrole-1-carbonyl]-3-methyl-butyl}-4-piperazin-1-yl-benzamide;

1758. *N*-{1-[4-(2-Amino-2-cyclopentyl-acetyl)-6-oxo-hexahydro-pyrrolo[3,2-*b*]pyrrole-1-carbonyl]-3-methyl-butyl}-4-piperazin-1-yl-benzamide;

1759. *N*-{1-[4-(2-Amino-2-cyclohexyl-acetyl)-6-oxo-hexahydro-pyrrolo[3,2-*b*]pyrrole-1-carbonyl]-3-methyl-butyl}-4-piperazin-1-yl-benzamide;

. 1760. *N*-{1-[4-(2-Acetylamino-2-cyclopentyl-acetyl)-6-oxo-hexahydro-pyrrolo[3,2-*b*]pyrrole-1-carbonyl]-3-methyl-butyl}-4-piperazin-1-yl-benzamide;

. 1761. *N*-{1-[4-(2-Acetylamino-2-cyclohexyl-acetyl)-6-oxo-hexahydro-pyrrolo[3,2-*b*]pyrrole-1-carbonyl]-3-methyl-butyl}-4-piperazin-1-yl-benzamide;

1762. *N*-{1-[4-(2-Acetylamino-propionyl)-6-oxo-hexahydro-pyrrolo[3,2-*b*]pyrrole-1-carbonyl]-3-methyl-butyl}-4-piperazin-1-yl-benzamide;

1763. *N*-{1-[4-(2-Acetylamino-acetyl)-6-oxo-hexahydro-pyrrolo[3,2-*b*]pyrrole-1-carbonyl]-3-methyl-butyl}-4-piperazin-1-yl-benzamide;

1764. *N*-(1-{4-[2-(Acetyl-methyl-amino)-acetyl]-6-oxo-hexahydro-pyrrolo[3,2-*b*]pyrrole-1-carbonyl}-3-methyl-butyl)-4-piperazin-1-yl-benzamide;

1765. *N*-{1-[4-(2-dimethylamino-acetyl)-6-oxo-hexahydro-pyrrolo[3,2-*b*]pyrrole-1-carbonyl]-3-methyl-butyl}-4-piperazin-1-yl-benzamide;

1766. *N*-{1-[4-(2-Amino-butyryl)-6-oxo-hexahydro-pyrrolo[3,2-*b*]pyrrole-1-carbonyl]-3-methyl-butyl}-4-piperazin-1-yl-benzamide;

1767. *N*-{1-[4-(2-Acetylamino-butyryl)-6-oxo-hexahydro-pyrrolo[3,2-*b*]pyrrole-1-carbonyl]-3-methyl-butyl}-4-piperazin-1-yl-benzamide;

1768. *N*-{1-[4-(2-amino-3-methyl-butyryl)-6-oxo-hexahydro-pyrrolo[3,2-*b*]pyrrole-1-carbonyl]-3-methyl-butyl}-4-piperazin-1-yl-benzamide;

1769. *N*-{1-[4-(2-Acetylamino-3-methyl-butyryl)-6-oxo-hexahydro-pyrrolo[3,2-*b*]pyrrole-1-carbonyl]-3-methyl-butyl}-4-piperazin-1-yl-benzamide;

1770. *N*-{1-[4-(2-amino-3,3-dimethyl-butyryl)-6-oxo-hexahydro-pyrrolo[3,2-*b*]pyrrole-1-carbonyl]-3-methyl-butyl}-4-piperazin-1-yl-benzamide;

1771. *N*-{1-[4-(2-Acetylamino-3,3-dimethyl-butyryl)-6-oxo-hexahydro-pyrrolo[3,2-*b*]pyrrole-1-carbonyl]-3-methyl-butyl}-4-piperazin-1-yl-benzamide;

1772. *N*-{1-[4-(2-Amino-3-methyl-pentanoyl)-6-oxo-hexahydro-pyrrolo[3,2-*b*]pyrrole-1-carbonyl]-3-methyl-butyl}-4-piperazin-1-yl-benzamide;

1773. *N*-{1-[4-(2-Acetylamino-3-methyl-pentanoyl)-6-oxo-hexahydro-pyrrolo[3,2-*b*]pyrrole-1-carbonyl]-3-methyl-butyl}-4-piperazin-1-yl-benzamide;

1774. *N*-[3-methyl-1-(6-oxo-4-pentanoyl-hexallydro-pynolo[3,2-*b*]pyrrole-1-carbonyl)-butyl]-4-piperazin-1-yl-benzamide;

1775. *N*-{3-methyl-1-[4-(3-methyl-pentanoyl)-6-oxo-hexahydro-pyrrolo[3,2-*b*]pyrrole-1-carbonyl]-butyl}-4-piperazin-1-yl-benzamide;

1776. *N*-{3-methyl-1-[4-(4-methyl-pentanoyl)-6-oxo-hexahydro-pyrrolo[3,2-*b*]pyrrole-1-carbonyl]-butyl}-4-piperazin-1-yl-benzamide;

1777. *N*-{1-[4-(2-Amino-4-methyl-pentanoyl)-6-oxo-hexahydro-pyrrolo[3,2-*b*]pyrrole-1-carbonyl]-3-methyl-butyl}-4-piperazin-1-yl-benzamide;

. 1778. *N*-{1-[4-(2-Acetylamino-4-methyl-pentanoyl)-6-oxo-hexahydro-pyrrolo[3,2-*b*]pyrrole-1-carbonyl]-3-methyl-butyl}-4-piperazin-1-yl-benzamide;

1779. *N*-{1-[4-(2-Amino-4,4-dimethyl-pentanoyl)-6-oxo-hexahydro-pyrrolo[3,2-*b*]pyrrole-1-carbonyl]-3-methyl-butyl}-4-piperazin-1-yl-benzamide;

1780. *N*-{1-[4-(2-Acetylamino-4,4-dimethyl-pentanoyl)-6-oxo-hexahydro-pyrrolo[3,2-*b*]pyrrole-1-carbonyl]-3-methyl-butyl}-4-piperazin-1-yl-benzamide;

1781. *N*-{1-[4-(2-Amino-pentanoyl)-6-oxo-hexahydro-pyrrolo[3,2-*b*]pyrrole-1-carbonyl]-3-methyl-butyl}-4-piperazin-1-yl-benzamide;

1782. *N*-{1-[4-(2-Acetylamino-pentanoyl)-6-oxo-hexahydro-pyrrolo[3,2-*b*]pyrrole-1-carbonyl]-3-methyl-butyl}-4-piperazin-1-yl-benzamide;

1783. *N*-{1-[4-(2-Amino-5-methyl-hexanoyl)-6-oxo-hexahydro-pyrrolo[3,2-*b*]pyrrole-1-carbonyl]-3-methyl-butyl}-4-piperazin-1-yl-benzamide;

1784. *N*-{1-[4-(2-Acetylamino-5-methyl-hexanoyl)-6-oxo-hexahydro-pyrrolo[3,2-*b*]pyrrole-1-carbonyl]-3-methyl-butyl}-4-piperazin-1-yl-benzamide;

1785. *N*-{1-[4-(2-hydroxy-3-methyl-butyryl)-6-oxo-hexahydro-pyrrolo[3,2-*b*]pyrrole-1-carbonyl]-3-methyl-butyl}-4-piperazin-1-yl-benzamide;

1786. *N*-{1-[4-(2-methoxy-3-methyl-butyryl)-6-oxo-hexahydro-pyrrolo[3,2-*b*]pyrrole-1-carbonyl]-3-methyl-butyl}-4-piperazin-1-yl-benzamide;

1787. *N*-{1-[4-(2-hydroxy-3,3-dimethyl-butyryl)-6-oxo-hexahydro-pyrrolo[3,2-*b*]pyrrole-1-carbonyl]-3-methyl-butyl}-4-piperazin-1-yl-benzamide;

1788. *N*-{1-[4-(2-methoxy-3,3-dimethyl-butyryl)-6-oxo-hexahydro-pyrrolo[3,2-*b*]pyrrole-1-carbonyl]-3-methyl-butyl}-4-piperazin-1-yl-benzamide;

1789. *N*-{1-[4-(2-hydroxy-3-methyl-pentanoyl)-6-oxo-hexahydro-pyrrolo[3,2-*b*]pyrrole-1-carbonyl]-3-methyl-butyl}-4-piperazin-1-yl-benzamide;

1790. *N*-{1-[4-(2-methoxy-3-methyl-pentanoyl)-6-oxo-hexahydro-pyrrolo[3,2-*b*]pyrrole-1-carbonyl]-3-methyl-butyl}-4-piperazin-1-yl-benzamide;

1791. *N*-{1-[4-(2-hydroxy-4-methyl-pentanoyl)-6-oxo-hexahydro-pyrrolo[3,2-*b*]pyrrole-1-carbonyl]-3-methyl-butyl}-4-piperazin-1-yl-benzamide;

1792. *N*-{1-[4-(2-methoxy-4-methyl-pentanoyl)-6-oxo-hexahydro-pyrrolo[3,2-*b*]pyrrole-1-carbonyl]-3-methyl-butyl}-4-piperazin-1-yl-benzamide;

1793. *N*-{1-[4-(2-hydroxy-4,4-dimethyl-pentanoyl)-6-oxo-hexahydro-pyrrolo[3,2-*b*]pyrrole-1-carbonyl]-3-methyl-butyl}-4-piperazin-1-yl-benzamide;

1794. *N*-{1-[4-(2-methoxy-4,4-dimethyl-pentanoyl)-6-oxo-hexahydro-pyrrolo[3,2-*b*]pyrrole-1-carbonyl]-3-methyl-butyl}-4-piperazin-1-yl-benzamide;

1795. *N*-{1-[4-(2-hydroxy-pentanoyl)-6-oxo-hexahydro-pyrrolo[3,2-*b*]pyrrole-1-carbonyl]-3-methyl-butyl}-4-piperazin-1-yl-benzamide;

1796. *N*-{1-[4-(2-methoxy-pentanoyl)-6-oxo-hexahydro-pyrrolo[3,2-*b*]pyrrole-1-carbonyl]-3-methyl-butyl}-4-piperazin-1-yl-benzamide;

1797. *N*-{1-[4-(2-hydroxy-5-methyl-hexanoyl)-6-oxo-hexahydro-pyrrolo[3,2-*b*]pyrrole-1-carbonyl]-3-methyl-butyl}-4-piperazin-1-yl-benzamide;

1798. *N*-{1-[4-(2-methoxy-5-methyl-hexanoyl)-6-oxo-hexahydro-pyrrolo[3,2-*b*]pyrrole-1-carbonyl]-3-methyl-butyl}-4-piperazin-1-yl-benzamide;

1799. *N*-[1-(4-Benzoyl-6-oxo-hexahydro-pyrrolo[3,2-*b*]pyrrole-1-carbonyl)-3-methyl-butyl]-4-(4-methyl-piperazin-1-yl)benzamide;

1800. *N*-{3-methyl-1-[6-oxo-4-(1-oxy-pyfidine-2-carbonyl)-hexahydro-pyrrolo[3,2-*b*]pyrrole-1-carbonyl]-butyl}-4-(4-methyl-piperazin-1-yl)-benzamide;

1801. *N*-{3-methyl-1-[6-oxo-4-(pyridine-3-carbonyl)-hexahydro-pyrrolo[3,2-*b*]pyrrole-1-carbonyl]-butyl}-4-(4-methyl-piperazin-1-yl)-benzamide;

1802. *N*-{3-methyl-1-[6-oxo-4-(1-oxy-pyridine-3-carbonyl)-hexahydro-pyrrolo[3,2-*b*]pyrrole-1-carbonyl]-butyl}-4-(4-methyl-piperazin-1-yl)-benzamide;

1803. *N*-{3-methyl-1-[6-oxo-4-(pyridine-4-carbonyl)-hexahydro-pyrrolo[3,2-*b*]pyrrole-1carbonyl]-butyl}4-(4-methyl-piperazin-1-yl)-benzamide;

1804. *N*-{3-methyl-1-[6-oxo-4-(1-oxy-pyridine-4-carbonyl)-hexahydro-pyrrolo[3,2-*b*]pyrrole-1-carbonyl]-butyl}-4-(4-methyl-piperazin-1-yl)-benzamide;

1805. *N*-[1-(4-Benzenesulfonyl-6-oxo-hexahydro-pyrrolo[3,2-*b*]pyrrole-1-carbonyl)-3-methyl-butyl]-4-(4-methyl-piperazin-1yl)-benzamide;

1806. *N*-{3-methyl-1-[6-oxo-4-(pyridine-4-sulfonyl)-hexahydro-pyrrolo[3,2-*b*]pyrrole-1-carbonyl]-butyl}-4-(4-methyl-piperazin-1-yl)-benzamide;

1807. *N*-{3-methyl-1-[6-oxo-4-(1-oxy-pyridine-4-sulfonyl)-hexahydro-pyrrolo[3,2-*b*]pyrrole-1-carbonyl]-butyl}-4-(4-methyl-piperazin-1-yl)-benzamide;

1808. *N*-[3-methyl-1-(6-oxo-4-phenylacetyl-hexahydro-pyrrolo[3,2-*b*]pyrrole-1-carbonyl)-butyl]-4-(4-methyl-piperazin-1-yl)-benzamide;

1809. 4-[2-(4-(4-methyl-piperazin-1-yl)-benzoylamino)-4-methyl-pentanoyl]-3-oxo-hexahydro-pyrrolo[3,2-*b*]pyrrole-1-carboxylic acid phenylamide;

1810. 4-[2-(4-(4-methyl-piperazin-1-yl)-benzoylamino)-4-methyl-pentanoyl]-3-oxo-hexahydro-pyrrolo[3,2-*b*]pyrrole-1-carboxylic acid isobutyl-amide;

1811. 4-[2-(4-(4-methyl-piperazin-1-yl)-benzoylamino)-4-methyl-pentanoyl]-3-oxo-hexahydro-pyrrolo[3,2-*b*]pyrrole-1-carboxylic acid phenyl ester;

1812. 4-[2-(4-(4-methyl-piperazin-1-yl)-benzoylamino)-4-methyl-pentanoyl]-3-oxo-hexahydro-pyrrolo[3,2-*b*]pyrrole-1-carboxylic acid isobutyl ester;

1813. *N*-{3-methyl-1-[6-oxo-4-(pyriolidine-1-carbonyl)-hexahydro-pyrrolo[3,2-*b*]pyrrole-1-carbonyl]-butyl}-4-(4- methyl-piperazin-1-yl)-benzamide;

1814. *N*-{3-methyl-1-[6-oxo-4-(piperidine-1-carbonyl)-hexahydro-pyrrolo[3,2-*b*]pyrrole-1-carbonyl]-butyl}-4-(4-methyl-piperazin-1-yl)-benzamide;

1815. *N*-{3-methyl-1-[4-(morpholine-4-carbonyl)-6-oxo-hexahydro-pyrrolo[3,2-*b*]pyrrole-1-carbonyl]-butyl}-4-(4-methyl-piperazin-1-yl)-benzamide;

1816. *N*-{3-methyl-1-[6-oxo-4-(piperazine-1-carbonyl)-hexahydro-pyrrolo[3,2-*b*]pyrrole-1-carbonyl]-butyl}-4-(4-methyl-piperazin-1-yl)-benzamide;

1817. *N*-{3-methyl-1-[4-(4-methyl-piperazine-1-carbonyl)-6-oxo-hexahydro-pyrrolo[3,2-*b*]pyrrole-1-carbonyl]-butyl}-4-(4-methyl-piperazin-1-yl)-benzamide;

1818. *N*-(3-methyl-1-{4-[2-(4-methyl-piperazin-1-yl)-acetyl]-6-oxo-hexahydro-pyrrolo[3,2-*b*]pyrrole-1-carbonyl}-butyl)-4-(4-methyl-piperazin-1-yl)-benzamide;

1819. *N*-{3-methyl-1-[6-oxo-4-(2-piperazin-1-yl-acetyl)-hexahydro-pyrrolo[3,2-*b*]pyrrole-1-carbonyl]-butyl}-4-(4-methyl-piperazin-1-yl)-benzamide;

1820. *N*-{1-[4-(furan-2-carbonyl)-6-oxo-hexahydro-pyrrolo[3,2-*b*]pyrrole-1-carbonyl]-3-methyl-butyl}-4-(4-methyl-piperazin-1-yl)-benzamide;

1821. *N*-{1-[4-(5-chloro-furan-2-carbonyl)-6-oxo-hexahydro-pyrrolo[3,2-*b*]pyrrole-1-carbonyl]-3-methyl-butyl}-4-(4-methyl-piperazin-1-yl)-benzamide;

1822. *N*-{1-[4-(thiophene-2-carbonyl)-6-oxo-hexahydro-pyrrolo[3,2-*b*]pyrrole-1-carbonyl]-3-methyl-butyl}-4-(4-methyl-piperazin-1-yl)-benzamide;

1823. *N*-{1-[4-(5-chloro-thiophene-2-carbonyl)-6-oxo-hexahydro-pyrrolo[3,2-*b*]pyrrole-1-carbonyl]-3-methyl-butyl}-4-(4-methyl-piperazin-1-yl)-benzamide;

1824. *N*-{1-[4-(furan-3-carbonyl)-6-oxo-hexahydro-pyrrolo[3,2-*b*]pyrrole-1-carbonyl]-3-methyl-butyl}-4-(4-methyl-piperazin-1-yl)-benzamide;

1825. *N*-{1-[4-(thiophene-3-carbonyl)-6-oxo-hexahydro-pyrrolo[3,2-*b*]pyrrole-1-carbonyl]-3-methyl-butyl}-4-(4-methyl-piperazin-1-yl)-benzamide;

1826. *N*-[1-(4-cyclopentanecarbonyl-6-oxo-hexahydro-pyrrolo[3,2-*b*]pyrrole-1-carbonyl)-3-methyl-butyl]-4-(4-methyl-piperazin-1-yl)-benzamide;

1827. *N*-[1-(4-cyclohexanecarbonyl-6-oxo-hexahydro-pyrrolo[3,2-*b*]pyrrole-1-carbonyl)-3-methyl-butyl]-4-(4-methyl-piperazin-1-yl)-benzamide;

1828. *N*-{1-[4-(cyclopent-3-enecarbonyl)-6-oxo-hexahydro-pyrrolo[3,2-*b*]pyrrole-1-carbonyl]-3-methyl-butyl}-4-(4-methyl-piperazin-1-yl)-benzamide;

1829. *N*-{1-[4-(2-cyclopentyl-acetyl)-6-oxo-hexahydro-pyrrolo[3,2-*b*]pyrrole-1-carbonyl]-3-methyl-butyl}-4-(4-methyl-piperazin-1-yl)-benzamide;

1830. *N*-{1-[4-(2-cyclohexyl-acetyl)-6-oxo-hexahydro-pyrrolo[3,2-*b*]pyrrole-1-carbonyl]-3-methyl-butyl}-4-(4-methyl-piperazin-1-yl)-benzamide;

1831. 4-(4-methyl-piperazin-1-yl)-*N*-{3-methyl-1-[6-oxo-4-(pyrrolidine-2-carbonyl)-hexahydro-pyrrolo[3,2-*b*]pyrrole-1-carbonyl]-butyl}-benzamide;

1832. *N*-{1-[4-(1-Acetyl-pyrrolidine-2-carbonyl)-6-oxo-hexahydro-pyrrolo[3,2-*b*]pyrrole-1-carbonyl]-3-methyl-butyl}-4-(4-methyl-piperazin-1-yl)-benzamide;

1833. *N*-{3-methyl-1-[6-oxo-4-(piperidine-2-carbonyl)-hexahydro-pyrrolo[3,2-*b*]pyrrole-1-carbonyl]-butyl}-4-(4-methyl-piperazin-1-yl)-benzamide;

1834. *N*-{1-[4-(1-Acetyl-piperidine-2-carbonyl)-6-oxo-hexahydro-pyrrolo[3,2-*b*]pyrrole-1-carbonyl]-3-methyl-butyl}-4-(4-methyl-piperazin-1-yl)-benzamide;

1835. *N*-{3-methyl-1-[6-oxo-4-(tetrahydro-furan-2-carbonyl)-hexahydro-pyrrolo[3,2-*b*]pyrrole-1-carbonyl]-butyl}-4-(4-methyl-piperazin-1-yl)-benzamide;

1836. *N*-{1-[4-(1-Amino-cyclobutanecarbonyl)-6-oxo-hexahydro-pyrrolo[3,2-*b*]pyrrole-1-carbonyl]-3-methyl-butyl}-4-(4-methyl-piperazin-1-yl)-benzamide;

1837. *N*-{1-[4-(1-Amino-cyclopentanecarbonyl)-6-oxo-hexahydro-pyrrolo[3,2]-*b*]pyrrole-1-carbonyl]-3-methyl-butyl}-4-(4-methyl-piperazin-1-yl)-benzamide;

1838. *N*-{1-[4-(1-Amino-cyclohexanecarbonyl)-6-oxo-hexahydro-pyrrolo[3,2-*b*]pyrrole-1-carbonyl]-3-methyl-butyl}-4-(4-methyl-piperazin-1-yl)-benzamide;

1839. *N*-{1-[4-(1-Acetylamino-cyclobutanecarbonyl)-6-oxo-hexahydro-pyrrolo[3,2-*b*]pyrrole-1-carbonyl]-3-methyl-butyl}-4-(4-methyl-piperazin-1-yl)-benzamide;

1840. *N*-{1-[4-(1-Acetylamino-cyclopentanecarbonyl)-6-oxo-hexahydro-pyrrolo[3,2-*b*]pyrrole-1-carbonyl]-3-methyl-butyl}-4-(4-methyl-piperazin-1-yl)-benzamide;

1841. *N*-{1-[4-(1-Acetylamino-cyclohexanecarbonyl)-6-oxo-hexahydro-pyrrolo[3,2-*b*]pyrrole-1-carbonyl]-3-methyl-butyl}-4-(4-methyl-piperazin-1-yl)-benzamide;

1842. *N*-{1-[4-(1-hydroxy-cyclobutanecarbonyl)-6-oxo-hexahydro-pyrrolo[3,2-*b*]pyrrole-1-carbonyl]-3-methyl-butyl}-4-(4-methyl-piperazin-1-yl)-benzamide;

1843. *N*-{1-[4-(1-hydroxy-cyclopentanecarbonyl)-6-oxo-hexahydro-pyrrolo[3,2-*b*]pyrrole-1-carbonyl]-3-methyl-butyl}-4-(4-methyl-piperazin-1-yl)-benzamide;

1844. *N*-{1-[4-(1-hydroxy-cyclohexanecarbonyl)-6-oxo-hexahydro-pyrrolo[3,2-*b*]pyrrole-1-carbonyl]-3-methyl-butyl}-4-(4-methyl-piperazin-1-yl)-benzamide;

1845. *N*-{1-[4-(1-methoxy-cyclobutanecarbonyl)-6-oxo-hexahydro-pyrrolo[3,2-*b*]pyrrole-1-carbonyl]-3-methyl-butyl}-4-(4-methyl-piperazin-1-yl)-benzamide;

1846. *N*-{1-[4-(1-methoxy-cyclopentanecarbonyl)-6-oxo-hexahydro-pyrrolo[3,2-*b*]pyrrole-1-carbonyl]-3-methyl-butyl}-4-(4-methyl-piperazin-1-yl)-benzamide;

1847. *N*-{1-[4-(1-methoxy-cyclohexanecarbonyl)-6-oxo-hexahydro-pyrrolo[3,2-*b*]pyrrole-1-carbonyl]-3-methyl-butyl}-4-(4-methyl-piperazin-1-yl)-benzamide;

1848. *N*-{1-[4-(2-Amino-2-cyclopentyl-acetyl)-6-oxo-hexahydro-pyrrolo[3,2-*b*]pyrrole-1-carbonyl]-3-methyl-butyl}-4-(4-methyl-piperazin-1-yl)-benzamide;

1849. *N*-{1-[4-(2-Amino-2-cyclohexyl-acetyl)-6-oxo-hexahydro-pyrrolo[3,2-*b*]pyrrole-1-carbonyl]-3-methyl-butyl}-4-(4-methyl-piperazin-1-yl)-benzamide;

1850. *N*-{1-[4-(2-Acetylamino-2-cyclopentyl-acetyl)-6-oxo-hexahydro-pyrrolo[3,2-*b*]pyrrole-1-carbonyl]-3-methyl-butyl}-4-(4-methyl-piperazin-1-yl)-benzamide;

1851. *N*-{1-[4-(2-Acetylamino-2-cyclohexyl-acetyl)-6-oxo-hexahydro-pyrrolo[3,2-*b*]pyrrole-1-carbonyl]-3-methyl-butyl}-4-(4-methyl-piperazin-1-yl)-benzamide;

1852. *N*-{1-[4-(2-Acetylamino-propionyl)-6-oxo-hexahydro-pyrrolo[3,2-*b*]pyrrole-1-carbonyl]-3-methyl-butyl}-4-(4-methyl-piperazin-1-yl)-benzamide;

1853. *N*-{1-[4-(2-Acetylamino-acetyl)-6-oxo-hexahydro-pyrrolo[3,2-*b*]pyrrole-1-carbonyl]-3-methyl-butyl}-4-(4-methyl-piperazin-1-yl)-benzamide;

1854. *N*-(1-{4-[2-(Acetyl-methyl-amino)-acetyl]-6-oxo-hexahydro-pyrrolo[3,2-*b*]pyrrole-1-carbonyl}-3-methyl-butyl)-4-(4-methyl-piperazin-1-yl)-benzamide;

1855. *N*-{1-[4-(2-dimethylamino-acetyl)-6-oxo-hexahydro-pyrrolo[3,2-*b*]pyrrole-1-carbonyl]-3-methyl-butyl}-4-(4-methyl-piperazin-1-yl)-benzamide;

1856. *N*-{1-[4-(2-Amino-butyryl)-6-oxo-hexahydro-pyrrolo[3,2-*b*]pyrrole-1-carbonyl]-3-methyl-butyl}-4-(4-methyl-piperazin-1-yl)-benzamide;

1857. *N*-{1-[4-(2-Acetylamino-butyryl)-6-oxo-hexahydro-pyrrolo[3,2-*b*]pyrrole-1-carbonyl]-3-methyl-butyl}-4-(4-methyl-piperazin-1-yl)-benzamide;

1858. *N*-{1-[4-(2-amino-3-methyl-butyryl)-6-oxo-hexahydro-pyrrolo[3,2-*b*]pyrrole-1-carbonyl]-3-methyl-butyl}-4-(4-methyl-piperazin-1-yl)-benzamide;

1859. *N*-{1-[4-(2-Acetylamino-3-methyl-butyryl)-6-oxo-hexahydro-pyrrolo[3,2-*b*]pyrrole-1-carbonyl]-3-methyl-butyl}-4-(4-methyl-piperazin-1-yl)-benzamide;

1860. *N*-{1-[4-(2-amino-3,3-dimethyl-butyryl)-6-oxo-hexahydro-pyrrolo[3,2-*b*]pyrrole-1-carbonyl]-3-methyl-butyl}-4-(4-methyl-piperazin-1-yl)-benzamide;

1861. *N*-{1-[4-(2-Acetylamino-3,3-dimethyl-butyryl)-6-oxo-hexahydro-pyrrolo[3,2-*b*]pyrrole-1-carbonyl]-3-methyl-butyl}-4-(4-methyl-piperazin-1-yl)-benzamide;

1862. *N*-{1-[4-(2-Amino-3-methyl-pentanoyl)-6-oxo-hexahydro-pyrrolo[3,2-*b*]pyrrole-1-carbonyl]-3-methyl-butyl}-4-(4-methyl-piperazin-1-yl)-benzamide;

1863. *N*-{1-[4-(2-Acetylamino-3-methyl-pentanoyl)-6-oxo-hexahydro-pyrrolo[3,2-*b*]pyrrole-1-carbonyl]-3-methyl-butyl}-4-(4-methyl-piperazin-1-yl)-benzamide;

1864. *N*-[3-methyl-1-(6-oxo-4-pentanoyl-hexahydro-pyrrolo[3,2-*b*]pyrrole-1-carbonyl)-butyl]-4-(4-methyl-piperazin-1-yl)-benzamide;

1865. *N*-{3-methyl-1-[4-(3-methyl-pentanoyl)-6-oxo-hexahydro-pyrrolo[3,2-*b*]pyrrole-1-carbonyl]-butyl}-4-(4-methyl-piperazin-1-yl)-benzamide;

1866. *N*-{3-methyl-1-[4-(4-methyl-pentanoyl)-6-oxo-hexahydro-pyrrolo[3,2-*b*]pyrrole-1-carbonyl]-butyl}-4-(4- methyl-piperazin-1-yl)-benzamide;

1867. *N*-{1-[4-(2-Amino-4-methyl-pentanoyl)-6-oxo-hexahydro-pyrrolo[3,2-*b*]pyrrole-1-carbonyl]-3-methyl-butyl}-4-(4-methyl-piperazin-1-yl)-benzamide;

1868. *N*-{1-[4-(2-Acetylamino-4-methyl-pentanoyl)-6-oxo-hexahydro-pyrrolo[3,2-*b*]pyrrole-1-carbonyl]-3-methyl-butyl}-4-(4-methyl-piperazin-1-yl)-benzamide;

1869. *N*-{1-[4-(2-Amino-4,4-dimethyl-pentanoyl)-6-oxo-hexahydro-pyrrolo[3,2-*b*]pyrrole-1-carbonyl]-3-methyl-butyl}-4-(4-methyl-piperazin-1-yl)-benzamide;

1870. *N*-{1-[4-(2-Acetylamino-4,4-dimethyl-pentanoyl)-6-oxo-hexahydro-pyrrolo[3,2-*b*]pyrrole-1-carbonyl]-3-methyl-butyl}-4-(4-methyl-piperazin-1-yl)-benzamide;

1871. *N*-{1-[4-(2-Amino-pentanoyl)-6-oxo-hexahydro-pyrrolo[3,2-*b*]pyrrole-1-carbonyl]-3-methyl-butyl}-4-(4-methyl-piperazin-1-yl)-benzamide;

1872. *N*-{1-[4-(2-Acetylamino-pentanoyl)-6-oxo-hexahydro-pyrrolo[3,2-*b*]pyrrole-1-carbonyl]-3-methyl-butyl}-4-(4-methyl-piperazin-1-yl)-benzamide;

1873. *N*-{1-[4-(2-Amino-5-methyl-hexanoyl)-6-oxo-hexahydro-pyrrolo[3,2-*b*]pyrrole-1-carbonyl]-3-methyl-butyl}-4-(4-methyl-piperazin-1-yl)-benzamide;

1874. *N*-{1-[4-(2-Acetylamino-5-methyl-hexanoyl)-6-oxo-hexahydro-pyrrolo[3,2-*b*]pyrrole-1-carbonyl]-3-methyl-butyl}-4-(4-methyl-piperazin-1-yl)-benzamide;

1875. *N*-{1-[4-(2-hydroxy-3-methyl-butyryl)-6-oxo-hexahydro-pyrrolo[3,2-*b*]pyrrole-1-carbonyl]-3-methyl-butyl}-4-(4-methyl-piperazin-1-yl)-benzamide;

1876. *N*-{1-[4-(2-methoxy-3-methyl-butyryl)-6-oxo-hexahydro-pyrrolo[3,2-*b*]pyrrole-1-carbonyl]-3-methyl-butyl}-4-(4-methyl-piperazin-1-yl)-benzamide;

1877. *N*-{1-[4-(2-hydroxy-3,3-dimethyl-butyryl)-6-oxo-hexahydro-pyrrolo[3,2-*b*]pyrrole-1-carbonyl]-3-methyl-butyl}-4-(4-methyl-piperazin-1-yl)-benzamide;

1878. *N*-{1-[4-(2-methoxy-3,3-dimethyl-butyryl)-6-oxo-hexahydro-pyrrolo[3,2-*b*]pyrrole-1-carbonyl]-3-methyl-butyl}-4-(4-methyl-piperazin-1-yl)-benzamide;

1879. *N*-{1-[4-(2-hydroxy-3-methyl-pentanoyl)-6-oxo-hexahydro-pyrrolo[3,2-*b*]pyrrole-1-carbonyl]-3-methyl-butyl}-4-(4-methyl-piperazin-1-yl)-benzamide;

1880. *N*-{1-[4-(2-methoxy-3-methyl-pentanoyl)-6-oxo-hexahydro-pyrrolo[3,2-*b*]pyrrole-1-carbonyl)-3-methyl-butyl}-4-(4-methyl-piperazin-1-yl)-benzamide;

1881. *N*-{1-[4-(2-hydroxy-4-methyl-pentanoyl)-6-oxo-hexahydro-pyrrolo[3,2-*b*]pyrrole-1-carbonyl]-3-methyl-butyl}-4-(4-methyl-piperazin-1-yl)-benzamide;

1882. *N*-{1-[4-(2-methoxy-4-methyl-pentanoyl)-6-oxo-hexahydro-pyrrolo[3,2-*b*]pyrrole-1-carbonyl]-3-methyl-butyl}-4-(4-methyl-piperazin-1-yl)-benzamide;

1883. *N*-{1-[4-(2-hydroxy-4,4-dimethyl-pentanoyl)-6-oxo-hexahydro-pyrrolo[3,2-*b*]pyrrole-1-carbonyl]-3-methyl-butyl}-4-(4-methyl-piperazin-1-yl)-benzamide;

1884. *N*-{1-[4-(2-methoxy-4,4-dimethyl-pentanoyl)-6-oxo-hexahydro-pyrrolo[3,2-*b*]pyrrole-1-carbonyl]-3-methyl-butyl}-4-(4-methyl-piperazin-1-yl)-benzamide;

1885. *N*-{1-[4-(2-hydroxy-pentanoyl)-6-oxo-hexahydro-pyrrolo[3,2-*b*]pyrrole-1-carbonyl]-3-methyl-butyl}-4-(4-methyl-piperazin-1-yl)-benzamide;

1886. *N*-{1-[4-(2-methoxy-pentanoyl)-6-oxo-hexahydro-pyrrolo[3,2-*b*]pyrrole-1-carbonyl]-3-methyl-butyl}-4-(4- methyl-piperazin-1-yl)-benzamide;

1887. *N*-{1-[4-(2-hydroxy-5-methyl-hexanoyl)-6-oxo-hexahydro-pyrrolo[3,2-*b*]pyrrole-1-carbonyl]-3-methyl-butyl}-4-(4-methyl-piperazin-1-yl)-benzamide;

1888. *N*-{1-[4-(2-methoxy-5-methyl-hexanoyl)-6-oxo-hexahydro-pyrrolo[3,2-*b*]pyrrole-1-carbonyl]-3-methyl-butyl}-4-(4-methyl-piperazin-1-yl)-benzamide;

1889. *N*-[1-(4-Benzoyl-6-oxo-hexahydro-pyrrolo[3,2-*b*]pyrrole-1-carbonyl)-3-methyl-butyl]-4-aminobenzamide;

1890. 4-Amino-*N*-{3-methyl-1-[6-oxo-4-(pyridine-2-carbonyl)-hexahydro-pyrrolo[3,2-*b*]pyrrole-1-carbonyl]-butyl}-benzamide;

1891. 4-Amino-*N*-{3-methyl-1-[6-oxo-4-(1-oxy-pyridine-2-carbonyl)-hexahydro-pyrrolo[3,2-*b*]pyrrole-1-carbonyl]-butyl}-benzamide;

1892. 4-Amino-*N*-{3-methyl-1-[6-oxo-4-(pyridine-3-carbonyl)-hexahydro-pyrrolo[3,2-*b*]pyrrole-1-carbonyl]-butyl}-benzamide;

1893. 4-Amino-*N*-{3-methyl-1-[6-oxo-4-(1-oxy-pyridine-3-carbonyl)-hexahydro-pyrrolo[3,2-*b*]pyrrole-1-carbonyl]-butyl}-benzamide;

1894. 4-Amino-*N*-{3-methyl-1-[6-oxo-4-(pyridine-4-carbonyl)-hexahydro-pyrrolo[3,2-*b*]pyrrole-1-carbonyl]-butyl}-benzamide;

1895. 4-Amino-*N*-{3-methyl-1-[6-oxo-4-(1-oxy-pyfidine-4-carbonyl)-hexahydro-pyrrolo[3,2-*b*]pyrrole-1-carbonyl]-butyl}-benzamide;

1896. *N*-[1-(4-Benzenesulfonyl-6-oxo-hexahydro-pyrrolo[3,2-*b*]pyrrole-1-carbonyl)-3-methyl-butyl]-4-amino-benzamide;

1897. 4-Amino-*N*-{3-methyl-1-[6-oxo-4-(pyridine-2-sulfonyl)-hexahydro-pyrrolo[3,2-*b*]pyrrole-1-carbonyl]-butyl}-benzamide;

1898. 4-Amino-*N*-{3-methyl-1-[6-oxo-4-(1-oxy-pyridine-2-sulfonyl)-hexahydro-pyrrolo[3,2-*b*]pyrrole-1-carbonyl]-butyl}-benzamide;

1899. 4-Amino-*N*-{3-methyl-1-[6-oxo-4-(pyridine-3-sulfonyl)-hexahydro-pyrrolo[3,2-*b*]pyrrole-1-carbonyl]-butyl}-benzamide;

1900. 4-Amino-*N*-{3-methyl-1-[6-oxo-4-(1-oxy-pyridine-3-sulfonyl)-hexahydro-pyrrolo[3,2-*b*]pyrrole-1-carbonyl]-butyl}-benzamide;

1901. 4-Amino-*N*-{3-methyl-1-[6-oxo-4-(pyridine-4-sulfonyl)-hexahydro-pyrrolo[3,2-*b*]pyrrole-1-carbonyl]-butyl}-benzamide;

1902. 4-Amino-*N*-{3-methyl-1-[6-oxo-4-(1-oxy-pyridine-4-sulfonyl)-hexahydro-pyrrolo[3,2-*b*]pyrrole-1-carbonyl]-butyl}-benzamide;

1903. 4-Amino-*N*-[3-methyl-1-(6-oxo-4-phenylacetyl-hexahydro-pyrrolo[3,2-*b*]pyrrole-1-carbonyl)-butyl]-benzamide;

1904. 4-[2-(4-amino-benzoylamino)-4-methyl-pentanoyl]-3-oxo-hexahydro-pyrrolo[3,2-*b*]pyrrole-1-carboxylic acid phenylamide;

1905. 4-[2-(4-amino-benzoylamino)-4-methyl-pentanoyl]-3-oxo-hexahydro-pyrrolo[3,2-*b*]pyrrole-1-carboxylic acid isobutyl-amide;

1906. 4-[2-(4-amino-benzoylamino)-4-methyl-pentanoyl]-3-oxo-hexahydro-pyrrolo[3,2-*b*]pyrrole-1-carboxylic acid phenyl ester;

1907. 4-[2-(4-amino-benzoylamino)-4-methyl-pentanoyl]-3-oxo-hexahydro-pyrrolo[3,2-*b*]pyrrole-1-carboxylic acid isobutyl ester;

1908. 4-Amino-*N*-{3-methyl-1-[6-oxo-4-(pyrrolidine-1-carbonyl)-hexahydro-pyrrolo[3,2-*b*]pyrrole-1-carbonyl]-butyl}-benzamide;

1909. 4-Amino-*N*-{3-methyl-1-[6-oxo-4-(piperidine-1-carbonyl)-hexahydro-pyrrolo[3,2-*b*]pyrrole-1-carbonyl]-butyl}-benzamide;

1910. 4-Amino-*N*-{3-methyl-1-[4-(morpholine-4-carbonyl)-6-oxo-hexahydro-pyrrolo[3,2-*b*]pyrrole-1-carbonyl]-butyl}-benzamide;

1911. 4-Amino-*N*-{3-methyl-1-[6-oxo-4-(piperazine-1-carbonyl)-hexahydro-pyrrolo[3,2-*b*]pyrrole-1-carbonyl]-butyl}-benzamide;

1912. 4-Amino-*N*-{3-methyl-1-[4-(4-methyl-piperazine-1-carbonyl)-6-oxo-hexahydro-pyrrolo[3,2-*b*]pyrrole-1-carbonyl]-butyl}-benzamide;

1913. 4-Amino-*N*-(3-methyl-1-{4-[2-(4-methyl-piperazin-1-yl)-acetyl]-6-oxo-hexahydro-pyrrolo[3,2-*b*]pyrrole-1-carbonyl}-butyl)-benzamide;

1914. 4-Amino-*N*-{3-methyl-1-[6-oxo-4-(2-piperazin-1-yl-acetyl)-hexahydro-pyrrolo[3,2-*b*]pyrrole-1-carbonyl]-butyl}-benzamide;

1915. 4-Amino-*N*-{1-[4-(furan-2-carbonyl)-6-oxo-hexahydro-pyrrolo[3,2-*b*]pyrrole-1-carbonyl]-3-methyl-butyl}-benzamide;

1916. 4-Amino-*N*-{1-[4-(5-chloro-furan-2-carbonyl)-6-oxo-hexahydro-pyrrolo[3,2-*b*]pyrrole-1-carbonyl]-3-methyl-butyl}-benzamide;

1917. 4-Amino-*N*-{1-[4-(thiophene-2-carbonyl)-6-oxo-hexahydro-pyrrolo[3,2-*b*]pyrrole-1-carbonyl]-3-methyl-butyl}-benzamide;

1918. 4-Amino-*N*-{1-[4-(5-chloro-thiophene-2-carbonyl)-6-oxo-hexahydro-pyrrolo[3,2-*b*]pyrrole-1-carbonyl]-3-methyl-butyl}-benzamide;

1919. 4-Amino-*N*-{1-[4-(furan-3-carbonyl)-6-oxo-hexahydro-pyrrolo[3,2-*b*]pyrrole-1-carbonyl]-3-methyl-butyl}-benzamide;

1920. 4-Amino-*N*-{1-[4-(thiophene-3-carbonyl)-6-oxo-hexahydro-pyrrolo[3,2-*b*]pyrrole-1-carbonyl]-3-methyl-butyl}-benzamide;

1921. 4-Amino-*N*-[1-(4-cyclopentanecarbonyl-6-oxo-hexahydro-pyrrolo[3,2-*b*]pyrrole-1-carbonyl)-3-methyl-butyl]-benzamide;

1922. 4-Amino-*N*-[1-(4-cyclohexanecarbonyl-6-oxo-hexahydro-pyrrolo[3,2-*b*]pyrrole-1-carbonyl)-3-methyl-butyl]-benzamide;

1923. 4-Amino-*N*-{1-[4-(cyclopent-3-enecarbonyl)-6-oxo-hexahydro-pyrrolo[3,2-*b*]pyrrole-1-carbonyl]-3-methyl-butyl}-benzamide;

1924. 4-Amino-*N*-{1-[4-(2-cyclopentyl-acetyl)-6-oxo-hexahydro-pyrrolo[3,2-*b*]pyrrole-1-carbonyl]-3-methyl-butyl}-benzamide;

1925. 4-Amino-*N*-{1-[4-(2-cyclohexyl-acetyl)-6-oxo-hexahydro-pyrrolo[3,2-*b*]pyrrole-1-carbonyl]-3-methyl-butyl}-benzamide;

1926. 4-Amino-*N*-{3-methyl-1-[6-oxo-4-(pyrrolidine-2-carbonyl)-hexahydro-pyrrolo[3,2-*b*]pyrrole-1-carbonyl]-butyl}-benzamide;

1927. *N*-{1-[4-(1-Acetyl-pyrrolidine-2-carbonyl)-6-oxo-hexahydro-pyrrolo[3,2-*b*]pyrrole-1-carbonyl]-3-methyl-butyl}-4-amino-benzamide;

1928. 4-Amino-*N*-{3-methyl-1-[6-oxo-4-(piperidine-2-carbonyl)-hexahydro-pyrrolo[3,2-*b*]pyrrole-1-carbonyl]-butyl}-benzamide;

1929. *N*-{1-[4-(1-Acetyl-piperidine-2-carbonyl)-6-oxo-hexahydro-pyrrolo[3,2-*b*]pyrrole-1-carbonyl]-3-methyl-butyl}-4-amino-benzamide;

1930. 4-Amino-*N*-{3-methyl-1-[6-oxo-4-(tetrahydro-furan-2-carbonyl)-hexahydro-pyrrolo[3,2-*b*]pyrrole-1-carbonyl]-butyl}-benzamide;

1931. *N*-{1-[4-(1-Amino-cyclobutanecarbonyl)-6-oxo-hexahydro-pyrrolo[3,2-*b*]pyrrole-1-carbonyl]-3-methyl-butyl}-4-Amino-benzamide;

1932. *N*-{1-[4-(1-Amino-cyclopentanecarbonyl)-6-oxo-hexahydro-pyrrolo[3,2-*b*]pyrrole-1-carbonyl]-3-methyl-butyl}-4-amino-benzamide;

1933. *N*-{1-[4-(1-Amino-cyclohexanecarbonyl)-6-oxo-hexahydro-pyrrolo[3,2-*b*]pyrole-1-carbonyl]-3-methyl-butyl}-4-amino-benzamide;

1934. *N*-{1-[4-(1-Acetylamino-cyclobutanecarbonyl)-6-oxo-hexahydro-pyrrolo[3,2-*b*]pyrrole-1-carbonyl]-3-methyl-butyl}-4-amino-benzamide;

1935. *N*-{1-[4-(1-Acetylamino-cyclopentanecarbonyl)-6-oxo-hexahydro-pyrrolo[3,2-*b*]pyrrole-1-carbonyl]-3-methyl-butyl}-4-amino-benzamide;

1936. *N*-{1-[4-(1-Acetylamino-cyclohexanecarbonyl)-6-oxo-hexahydro-pyrrolo[3,2-*b*]pyrrole-1-carbonyl]-3-methyl-butyl}-4-amino-benzamide;

1937. 4-Amino-*N*-{1-[4-(1-hydroxy-cyclobutanecarbonyl)-6-oxo-hexahydro-pyrrolo[3,2-*b*]pyrrole-1-carbonyl]-3-methyl-butyl}-benzamide;

1938. 4-Amino-*N*-{1-[4-(1-hydroxy-cyclopentanecarbonyl)-6-oxo-hexahydro-pyrrolo[3,2-*b*]pyrrole-1-carbonyl]-3-methyl-butyl}-benzamide;

1939. 4-Amino-*N*-{1-[4-(1-hydroxy-cyclohexanecarbonyl)-6-oxo-hexahydro-pyrrolo[3,2-*b*]pyrrole-1-carbonyl]-3-methyl-butyl}-benzamide;

1940. 4-Amino-*N*-{1-[4-(1-methoxy-cyclobutanecarbonyl)-6-oxo-hexahydro-pyrrolo[3,2-*b*]pyrrole-1-carbonyl]-3-methyl-butyl}-benzamide;

1941. 4-Amino-*N*-{1-[4-(1-methoxy-cyclopentanecarbonyl)-6-oxo-hexahydro-pyrrolo[3,2-*b*]pyrrole-1-carbonyl]-3-methyl-butyl}-benzamide;

1942. 4-Amino-*N*-{1-[4-(1-methoxy-cyclohexanecarbonyl)-6-oxo-hexahydro-pyrrolo[3,2-*b*]pyrrole-1-carbonyl]-3-methyl-butyl}-benzamide;

1943. *N*-{1-[4-(2-amino-2-cyclopentyl-acetyl)-6-oxo-hexahydro-pyrrolo[3,2-*b*]pyrrole-1-carbonyl]-3-methyl-butyl}-4-amino-benzamide;

1944. *N*-{1-[4-(2-amino-2-cyclohexyl-acetyl)-6-oxo-hexahydro-pyrrolo[3,2-*b*]pyrrole-1-carbonyl]-3-methyl-butyl}-4-amino-benzamide;

1945. *N*-{1-[4-(2-acetylamino-2-cyclopentyl-acetyl)-6-oxo-hexahydro-pyrrolo[3,2-*b*]pyrrole-1-carbonyl]-3-methyl-butyl}-4-amino-benzamide;

1946. *N*-{1-[4-(2-acetylamino-2-cylohexyl-acetyl)-6-oxo-hexahydro-pyrrolo[3,2-*b*]pyrrole-1-carbonyl]-3-methyl-butyl}-4-amino-benzamide;

1947. *N*-{1-[4-(2-acetylamino-propionyl)-6-oxo-hexahydro-pyrrolo[3,2-*b*]pyrrole-1-carbonyl]-3-methyl-butyl}-4-amino-benzamide;

1948. *N*-{1-[4-(2-acetylamino-acetyl)-6-oxo-hexahydro-pyrrolo[3,2-*b*]pyrrole-1-carbonyl]-3-methyl-butyl}-4-amino-benzamide;

1949. *N*-(1-{4-[2-(acetyl-methyl-amino)-acetyl]-6-oxo-hexahydro-pyrrolo[3,2-*b*]pyrrole-1-carbonyl}-3-methyl-butyl)-4-amino-benzamide;

1950. 4-Amino-*N*-{1-[4-(2-dimethylamino-acetyl)-6-oxo-hexahydro-pyrrolo[3,2-*b*]pyrrole-1-carbonyl]-3-methyl-butyl}-benzamide;

1951. *N*-{1-[4-(2-amino-butyryl)-6-oxo-hexahydro-pyrrolo[3,2-*b*]pyrrole-1-carbonyl]-3-methyl-butyl}-4-amino-benzamide;

1952. *N*-{1-[4-(2-acetylamino-butyryl)-6-oxo-hexahydro-pyrrolo[3,2-*b*]pyrrole-1-carbonyl]-3-methyl-butyl}-4-amino-benzamide;

1953. *N*-{1-[4-(2-amino-3-methyl-butyryl)-6-oxo-hexahydro-pyrrolo[3,2-*b*]pyrrole-1-carbonyl]-3-methyl-butyl}-4-amino-benzamide;

1954. *N*-{1-[4-(2-acetylamino-3-methyl-butyryl)-6-oxo-hexahydro-pyrrolo[3,2-*b*]pyrrole-1-carbonyl]-3-methyl-butyl}-4-amino-benzamide;

1955. *N*-{1-[4-(2-amino-3,3-dimethyl-butyryl)-6-oxo-hexahydro-pyrrolo[3,2-*b*]pyrrole-1-carbonyl]-3-methyl-butyl}-4-amino-benzamide;

1956. *N*-{1-[4-(2-acetylamino-3,3-dimethyl-butyryl)-6-oxo-hexahydro-pyrrolo[3,2-*b*]pyrrole-1-carbonyl]-3-methyl-butyl}-4-amino-benzamide;

1957. *N*-{1-[4-(2-amino-3-methyl-pentanoyl)-6-oxo-hexahydro-pyrrolo[3,2-*b*]pyrrole-1-carbonyl]-3-methyl-butyl}-4-amino-benzamide;

1958. *N*-{1-[4-(2-acetylamino-3-methyl-pentanoyl)-6-oxo-hexahydro-pyrrolo[3,2-*b*]pyrrole-1-carbonyl]-3-methyl-butyl}-4-amino-benzamide;

1959. 4-Amino-*N*-[3-methyl-1-(6-oxo-4-pentanoyl-hexahydro-pyrrolo[3,2-*b*]pyrrole-1-carbonyl)-butyl]-benzamide;

1960. 4-Amino-*N*-{3-methyl-1-[4-(3-methyl-pentanoyl)-6-oxo-hexahydro-pyrrolo[3,2-*b*]pyrrole-1-carbonyl]-butyl}-benzamide;

1961. 4-Amino-*N*-{3-methyl-1-[4-(4-methyl-pentanoyl)-6-oxo-hexahydro-pyrrolo[3,2-*b*]pyrrole-1-carbonyl]-butyl}-benzamide;

1962. *N*-{1-[4-(2-amino-4-methyl-pentanoyl)-6-oxo-hexahydro-pyrrolo[3,2-*b*]pyrrole-1-carbonyl]-3-methyl-butyl}-4-amino-benzamide;

1963. *N*-{1-[4-(2-acetylamino-4-methyl-pentanoyl)-6-oxo-hexahydro-pyrrolo[3,2-*b*]pyrrole-1-carbonyl]-3-methyl-butyl}-4-amino-benzamide;

1964. *N*-{1-[4-(2-amino-4,4-dimethyl-pentanoyl)-6-oxo-hexallydro-pyrrolo[3,2-*b*]pyrrole-1-carbonyl]-3-methyl-butyl}-4-amino-benzamide;

1965. *N*-{1-[4-(2-acetylamino-4,4-dimethyl-pentanoyl)-6-oxo-hexahydro-pyrrolo[3,2-*b*]pyrrole-1-carbonyl]-3- methyl-butyl}-4-amino-benzamide;

1966. *N*-{1-[4-(2-amino-pentanoyl)-6-oxo-hexahydro-pyrrolo[3,2-*b*]pyrrole-1-carbonyl]-3-methyl-butyl}-4-amino-benzamide;

1967. *N*-{1-[4-(2-acetylamino-pentanoyl)-6-oxo-hexahydro-pyrrolo[3,2-*b*]pyrrole-1-carbonyl]-3-methyl-butyl}-4-amino-benzamide;

1968. *N*-{1-[4-(2-amino-5-methyl-hexanoyl)-6-oxo-hexahydro-pyrrolo[3,2-*b*]pyrrole-1-carbonyl]-3-methyl-butyl}-4-amino-benzamide;

1969. *N*-{1-[4-(2-acetylamino-5-methyl-hexanoyl)-6-oxo-hexahydro-pyrrolo[3,2-*b*]pyrrole-1-carbonyl]-3-methyl-butyl}-4-amino-benzamide;

1970. *N*-{1-[4-(2-hydroxy-3-methyl-butyryl)-6-oxo-hexahydro-pyrrolo[3,2-*b*]pyrrole-1-carbonyl]-3-methyl-butyl}-4-amino-benzamide;

1971. *N*-{1-[4-(2-methoxy-3-methyl-butyryl)-6-oxo-hexahydro-pyrrolo[3,2-*b*]pyrrole-1-carbonyl]-3-methyl-butyl}-4-amino-benzamide;

1972. 4-Amino-*N*-{1-[4-(2-hydroxy-3,3-dimethyl-butyryl)-6-oxo-hexahydro-pyrrolo[3,2-*b*]pyrrole-1-carbonyl]-3-methyl-butyl}-benzamide;

1973. 4-Amino-*N*-{1-[4-(2-methoxy-3,3-dimethyl-butyryl)-6-oxo-hexahydro-pyrrolo[3,2-*b*]pyrrole-1-carbonyl]-3-methyl-butyl}-benzamide;

1974. 4-Amino-*N*-{1-[4-(2-hydroxy-3-methyl-pentanoyl)-6-oxo-hexahydro-pyrrolo[3,2-*b*]pyrrole-1-carbonyl]-3-methyl-butyl}-benzamide;

1975. 4-Amino-*N*-{1-[4-(2-methoxy-3-methyl-pentanoyl)-6-oxo-hexahydro-pyrrolo[3,2-*b*]pyrrole-1-carbonyl]-3-methyl-butyl}-benzamide;

1976. 4-Amino-*N*-{1-[4-(2-hydroxy-4-methyl-pentanoyl)-6-oxo-hexahydro-pyrrolo[3,2-*b*]pyrrole-1-carbonyl]-3-methyl-butyl}-benzamide;

1977. 4-Amino-*N*-{1-[4-(2-methoxy-4-methyl-pentanoyl)-6-oxo-hexahydro-pyrrolo[3,2-*b*]pyrrole-1-carbonyl]-3-methyl-butyl}-benzamide;

1978. 4-Amino-*N*-{1-[4-(2-hydroxy-4,4-dimethyl-pentanoyl)-6-oxo-hexahydro-pyrrolo[3,2-*b*]pyrrole-1-carbonyl]-3-methyl-butyl}-benzamide;

1979. 4-Amino-*N*-{1-[4-(2-methoxy-4,4-dimethyl-pentanoyl)-6-oxo-hexahydro-pyrrolo[3,2-*b*]pyrrole-1-carbonyl]-3-methyl-butyl}-benzamide;

1980. 4-Amino-*N*-{1-[4-(2-hydroxy-pentanoyl)-6-oxo-hexahydro-pyrrolo[3,2-*b*]pyrrole-1-carbonyl]-3-methyl-butyl}-benzamide;

1981. 4-Amino-*N*-{1-[4-(2-methoxy-pentanoyl)-6-oxo-hexahydro-pyrrolo[3,2-*b*]pyrrole-1-carbonyl]-3-methyl-butyl}-benzamide;

1982. 4-Amino-*N*-{1-[4-(2-hydroxy-5-methyl-hexanoyl)-6-oxo-hexahydro-pyrrolo[3,2-*b*]pyrrole-1-carbonyl]-3-methyl-butyl}-benzamide;

1983. 4-Amino-*N*-{1-[4-(2-methoxy-5-methyl-hexanoyl)-6-oxo-hexahydro-pyrrolo[3,2-*b*]pyrrole-1-carbonyl]-3-methyl-butyl}-benzamide;

1984. *N*-[1-(4-Benzoyl-6-oxo-hexahydro-pyrrolo[3,2-*b*]pyrrole-1-carbonyl)-3-methyl-butyl]-4-diethylaminobenzamide;

1985. 4-Diethylamino-*N*-{3-methyl-1-[6-oxo-4-(pyridine-2-carbonyl)-hexahydro-pyrrolo[3,2-*b*]pyrrole-1-carbonyl]-butyl}-benzamide;

1986. 4-Diethylamino-*N*-{3-methyl-1-[6-oxo-4-(1-oxy-pyridine-2-carbonyl)-hexahydro-pyrrolo[3,2-*b*]pyrrole-1-carbonyl]-butyl}-benzamide;

1987. 4-Diethylamino-*N*-{3-methyl-1-[6-oxo-4-(pyridine-3-carbonyl)-hexahydro-pyrrolo[3,2-*b*]pyrrole-1-carbonyl]-butyl}-benzamide;

1988. 4-Diethylamino-*N*-{3-methyl-1-[6-oxo-4-(1-oxy-pyridine-3-carbonyl)-hexahydro-pyrrolo[3,2-*b*]pyrrole-1-carbonyl]-butyl}-benzamide;

1989. 4-Diethylamino-*N*-{3-methyl-1-[6-oxo-4-(pyridine-4-carbonyl)-hexahydro-pyrrolo[3,2-*b*]pyrrole-1-carbonyl]-butyl}-benzamide;

1990. 4-Diethylamino-*N*-{3-methyl-1-[6-oxo-4-(1-oxy-pyridine-4-carbonyl)-hexahydro-pyrrolo[3,2-*b*]pyrrole-1-carbonyl]-butyl}-benzamide;

1991. *N*-[1-(4-Benzenesulfonyl-6-oxo-hexahydro-pyrrolo[3,2-*b*]pyrrole-1-carbonyl)-3-methyl-butyl]-4-diethylamino-benzamide;

1992. 4-Diethylamino-*N*-{3-methyl-1-[6-oxo-4-(pyridine-2-sulfonyl)-hexahydro-pyrrolo[3,2-*b*]pyrrole-1-carbonyl]-butyl}-benzamide;

1993. 4-Diethylamino-*N*-{3-methyl-1-[6-oxo-4-(1-oxy-pyridine-2-sulfonyl)-hexahydro-pyrrolo[3,2-*b*]pyrrole-1-carbonyl]-butyl}-benzamide;

1994. 4-Diethylamino-*N*-{3-methyl-1-[6-oxo-4-(pyridine-3-sulfonyl)-hexahydro-pyrrolo[3,2-*b*]pyrrole-1-carbonyl]-butyl}-benzamide;

1995. 4-Diethylamino-*N*-{3-methyl-1-[6-oxo-4-(1-oxy-pyridine-3-sulfonyl)-hexahydro-pyrrolo[3,2-*b*]pyrrole-1-carbonyl]-butyl} -benzamide;

1996. 4-Diethylamino-*N*-{3-methyl-1-[6-oxo-4-(pyridine-4-sulfonyl)-hexahydro-pyrrolo[3,2-*b*]pyrrole-1-carbonyl]-butyl}-benzamide;

1997. 4-Diethylainino-*N*-{3-methyl-1-[6-oxo-4-(1-oxy-pyridine-4-sulfonyl)-hexahydro-pyrrolo[3,2-*b*]pyrrole-1-carbonyl]-butyl}-benzamide;

1998. 4-Diethylamino-*N*-[3-methyl-1-(6-oxo-4-phenylacetyl-hexahydro-pyrrolo[3,2-*b*]pyrrole-1-carbonyl)-butyl]-benzamide;

1999. 4-[2-(4-diethylamino-benzoylamino)-4-methyl-pentanoyl]-3-oxo-hexahydro-pyrrolo[3,2-*b*]pyrrole-1-carboxylic acid phenylamide;

2000. 4-[2-(4-diethylamino-benzoylamino)-4-methyl-pentanoyl]-3-oxo-hexahydro-pyrrolo[3,2-*b*]pyrrole-1-carboxylic acid isobutyl-amide;

2001. 4-[2-(4-diethylamino-benzoylamino)-4-methyl-pentanoyl]-3-oxo-hexahydro-pyrrolo[3,2-*b*]pyrrole-1-carboxylic acid phenyl ester;

2002. 4-[2-(4-diethylamino-benzoylamino)-4-methyl-pentanoyl]-3-oxo-hexahydro-pyrrolo[3,2-*b*]pyrrole-1-carboxylic acid isobutyl ester;

2003. 4-Diethylamino-*N*-{3-methyl-1-[6-oxo-4-(pyrrolidine-1-carbonyl)-hexahydro-pyrrolo[3,2-*b*]pyrrole-1-carbonyl]-butyl}-benzarnide;

2004. 4-Diethylamino-*N*-{3-methyl-1-[6-oxo-4-(piperidine-1-carbonyl)-hexahydro-pyrrolo[3,2-*b*]pyrrole-1-carbonyl]-butyl}-lienzamide;

2005. 4-Diethylamino-*N*-{3-methyl-1-[4-(morpholine-4-carbonyl)-6-oxo-hexahydro-pyrrolo[3,2-*b*]pyrrole-1-carbonyl]-butyl}-benzamide;

2006. 4-Diethylamino-*N*-{3-methyl-1-[6-oxo-4-(piperazine-1-carbonyl)-hexahydro-pyrrolo[3,2-*b*]pyrrole-1-carbonyl]-butyl}-benzamide;

2007. 4-Diethylamino-*N*-{3-methyl-1-[4-(4-methyl-piperazine-1-carbonyl)-6-oxo-hexahydro-pyrrolo[3,2-*b*]pyrrole-1-carbonyl]-butyl}-benzamide;

2008. 4-Diethylamino-*N*-(3-methyl-1-{4-[2-(4-methyl-piperazin-1-yl)-acetyl]-6-oxo-hexahydro-pyrrolo[3,2-*b*]pyrrole-1-carbonyl}-butyl)-benzamide;

2009. 4-Diethylamino-*N*-(3-methyl-1-[6-oxo-4-(2-piperazin-1-yl-acetyl)-hexahydro-pyrrolo[3,2-*b*]pyrrole-1-carbonyl]-butyl}-benzamide;

2010. 4-Diethylamino-*N*-{1-[4-(furan-2-carbonyl)-6-oxo-hexahydro-pyrrolo[3,2-*b*]pyrrole-1-carbonyl]-3-methyl-butyl}-benzamide;

2011. 4-Diethylamino-*N*-{1-[4-(5-chloro-furan-2-carbonyl)-6-oxo-hexahydro-pyrrolo[3,2-*b*]pyrrole-1-carbonyl]-3-methyl-butyl}-benzamide;

2012. 4-Diethylamino-*N*-{1-[4-(thiophene-2-carbonyl)-6-oxo-hexahydro-pyrrolo[3,2-*b*]pyrrole-1-carbonyl]-3-methyl-butyl}-benzamide;

2013. 4-Diethylamino-*N*-{1-[4-(5-chloro-thiophene-2-carbonyl)-6-oxo-hexahydro-pyrrolo[3,2-*b*]pyrrole-1-carbonyl]-

3-methyl-butyl}-benzamide;

2014. 4-Diethylamino-*N*-{1-[4-(furan-3-carbonyl)-6-oxo-hexahydro-pyrrolo[3,2-*b*]pyrrole-1-carbonyl]-3-methyl-butyl}-benzamide;

2015. 4-Diethylamino-*N*-{1-[4-(thiophene-3-carbonyl)-6-oxo-hexahydro-pyrrolo[3,2-*b*]pyrrole-1-carbonyl]-3-methyl-butyl}-benzamide;

2016. 4-Diethylamino-*N*-[1-(4-cyclopentanecarbonyl-6-oxo-hexahydro-pyrrolo[3,2-*b*]pyrrole-1-carbonyl)-3-methyl-butyl]-benzamide;

2017. 4-Diethylamino-*N*-[1-(4-cydohexanecarbonyl-6-oxo-hexahydro-pyrrolo[3,2-*b*]pyrrole-1-carbonyl)-3-methyl-butyl]-benzamide;

2018. 4-Diethylamino-*N*-{1-[4-(cyclopent-3-enecarbonyl)-6-oxo-hexahydro-pyrrolo[3,2-*b*]pyrrole-1-carbonyl]-3-methyl-butyl}-benzamide;

2019. 4-Diethylamino-*N*-{1-[4-(2-cyclopentyl-acetyl)-6-oxo-hexahydro-pyrrolo[3,2-*b*]pyrrole-1-carbonyl]-3-methyl-butyl}-benzamide;

2020. 4-Diethylamino-*N*-{1-[4-(2-cyclohexyl-acetyl)-6-oxo-hexahydro-pyrrolo[3,2-*b*]pyrrole-1-carbonyl]-3-methyl-butyl}-benzamide;

2021. 4-Diethylamino-*N*-{3-methyl-1-[6-oxo-4-(pyrrolidine-2-carbonyl)-hexahydro-pyrrolo[3,2-*b*]pyrrole-1-carbonyl]-butyl}-benzamide;

2022. *N*-{1-[4-(1-Acetyl-pyrrolidine-2-carbonyl)-6-oxo-hexahydro-pyrrolo[3,2-*b*]pyrrole-1-carbonyl]-3-methyl-butyl}-4-diethylamino-benzamide;

2023. 4-Diethylamino-*N*-{3-methyl-1-[6-oxo-4-(piperidine-2-carbonyl)-hexahydro-pyrrolo[3,2-*b*]pyrrole-1-carbonyl]-butyl}-benzamide;

2024. *N*-{1-[4-(1-Acetyl-piperidine-2-carbonyl)-6-oxo-hexahydro-pyrrolo[3,2-*b*]pyrrole-1-carbonyl]-3-methyl-butyl}-4-diethylamino-benzamide;

2025. 4-Diethylamino-*N*-{3-methyl-1-[6-oxo-4-(tetrahydro-furan-2-carbonyl)-hexahydro-pyrrolo[3,2-*b*]pyrrole-1-carbonyl]-butyl}-benzamide;

2026. *N*-{1-[4-(1-Amino-cyclobutanecarbonyl)-6-oxo-hexahydro-pyrrolo[3,2-*b*]pyrrole-1-carbonyl]-3-methyl-butyl}-4-diethylamino-benzamide;

2027. *N*-{1-[4-(1-Amino-cydopentanecarbonyl)-6-oxo-hexahydro-pyrrolo[3,2-*b*]pyrrole-1-carbonyl]-3-methyl-butyl}-4-diethylamino-benzamide;

2028. *N*-{1-[4-(1-Amino-cyclohexanecarbonyl)-6-oxo-hexahydro-pyrrolo[3,2-*b*]pyrrole-1-carbonyl]-3-methyl-butyl}-4-diethylamino-benzamide;

2029. *N*-{1-[4-(1-Acetylamino-cyclobutanecarbonyl)-6-oxo-hexahydro-pyrrolo[3,2-*b*]pyrrole-1-carbonyl]-3-methyl-butyl}-4-diethylamino-benzamide;

2030. *N*-{1-[4-(1-Acetylamino-cyclopentanecarbonyl)-6-oxo-hexahydro-pyrrolo[3,2-*b*]pyrrole-1-carbonyl]-3-methyl-butyl}-4-diethylamino-benzamide;

2031. *N*-{1-[4-(1-Acetylamino-cyclohexanecarbonyl)-6-oxo-hexahydro-pyrrolo[3,2-*b*]pyrrole-1-carbonyl]-3-methyl-butyl}-4-diethylamino-benzamide;

2032. 4-Diethylamino-*N*-{1-[4-(1-hydroxy-cyclobutanecarbonyl)-6-oxo-hexahydro-pyrrolo[3,2-*b*]pyrrole-1-carbonyl]-3-methyl-butyl}-benzamide;

2033. 4-Diethylamino-*N*-{1-[4-(1-hydroxy-cyclopentanecarbonyl)-6-oxo-hexahydro-pyrrolo[3,2-*b*]pyrrole-1-carbonyl]-3-methyl-buyl}-benzamide;

2034. 4-Diethylamino-*N*-{1-[4-(1-hydroxy-cyclohexanecarbonyl)-6-oxo-hexahydro-pyrrolo[3,2-*b*]pyrrole-1-carbonyl]-3-methyl-butyl}-benzamide;

2035. 4-Diethylamino-*N*-{1-[4-(1-methoxy-cyclobutanecarbonyl)-6-oxo-hexahydro-pyrrolo-[3,2-*b*]pyrrole-1-carbonyl]-3-methyl-butyl}-benzamide;

2036. 4-Diethylamirio-*N*-{1-[4-(1-methoxy-cyclopentanecarbonyl)-6-oxo-hexahydro-pyrrolo[3,2-*b*]pyrrole-1-carbonyl]-3-methyl-butyl}-benzamide;

2037. 4-Diethylamino-*N*-{1-[4-(1-methoxy-cydohexanecarbonyl)-6-oxo-hexahydro-pyrrolo[3,2-*b*]pyrrole-1-carbonyl]-3-methyl-butyl}-benzamide;

2038. *N*-{1-[4-(2-Amino-2-cydopentyl-acetyl)-6-oxo-hexahydro-pyrrolo[3,2-*b*]pyrrole-1-carbonyl]-3-methyl-butyl}-4-diethylamino-benzamide;

2039. *N*-{1-[4-(2-Amino-2-cyclohexyl-acetyl)-6-oxo-hexahydro-pyrrolo[3,2-*b*]pyrrole-1-carbonyl]-3-methyl-butyl}-4-diethylamino-benzamide;

2040. *N*-{1-[4-(2-Acetylamino-2-cyclopentyl-acetyl)-6-oxo-hexahydro-pyrrolo[3,2-*b*]pyrrole-1-carbonyl]-3-methyl-butyl}-4-diethylamino-benzamide;

2041. *N*-{1-[4-(2-Acetylamino-2-cyclohexyl-acetyl)-6-oxo-hexahydro-pyrrolo[3,2-*b*]pyrrole-1-carbonyl]-3-methyl-butyl}-4-diethylamino-benzamide;

2042. *N*-{1-[4-(2-Acetylamino-propionyl)-6-oxo-hexahydro-pyrrolo[3,2-*b*]pyrrole-1-carbonyl]-3-methyl-butyl}-4-diethylamino-benzamide;

2043. *N*-{1-[4-(2-Acetylamino-acetyl)-6-oxo-hexahydro-pyrrolo[3,2-*b*]pyrrole-1-carbonyl]-3-methyl-butyl}-4-diethylamino-benzamide;

2044. *N*-(1-{4-[2-(Acetyl-methyl-amino)-acetyl]-6-oxo-hexahydro-pyrrolo[3,2-*b*]pyrrole-1-carbonyl}-3-methyl-butyl)-4-diethylamino-benzamide;

2045. 4-Diethylamino-*N*-{1-[4-(2-dimethylamino-acetyl)-6-oxo-hexahydro-pyrrolo[3,2-*b*]pyrrole-1-carbonyl]-3-methyl-butyl}-benzamide;

2046. *N*-{1-[4-(2-Amino-butyryl)-6-oxo-hexahydro-pyrrolo[3,2-*b*]pyrrole-1-carbonyl]-3-methyl-butyl}-4-diethylamino-benzamide;

2047. *N*-{1-[4-(2-Acetylamino-butyryl)-6-oxo-hexahydro-pyrrolo[3,2-*b*]pyrrole-1-carbonyl]-3-methyl-butyl}-4-diethylamino-benzamide;

2048. *N*-{1-[4-(2-amino-3-methyl-butyryl)-6-oxo-hexahydro-pyrrolo[3,2-*b*]pyrrole-1-carbonyl]-3-methyl-butyl}-4-diethylamino-benzamide;

2049. *N*-{1-[4-(2-Acetylamino-3-methyl-butyryl)-6-oxo-hexahydro-pyrrolo[3,2-*b*]pyrrole-1-carbonyl]-3-methyl-butyl}-4-diethylamino-benzamide;

2050. *N*-{1-[4-(2-amino-3,3-dimethyl-butyryl)-6-oxo-hexahydro-pyrrolo[3,2-*b*]pyrrole-1-carbonyl]-3-methyl-butyl}-4-diethylamino-benzamide;

2051. *N*-{1-[4-(2-Acetylamino-3,3-dimethyl-butyryl)-6-oxo-hexahydro-pyrrolo[3,2-*b*]pyrrole-1-carbonyl]-3-methyl-butyl}-4-diethylamino-benzamide;

2052. *N*-{1-[4-(2-Amino-3-methyl-pentanoyl)-6-oxo-hexahydro-pyrrolo[3,2-*b*]pyrrole-1-carbonyl]-3-methyl-butyl}-4-diethylamino-benzamide;

2053. *N*-{1-[4-(2-Acetylamino-3-methyl-pentanoyl)-6-oxo-hexahydro-pyrrolo[3,2-*b*]pyrrole-1-carbonyl]-3-methyl-butyl}-4-diethylamino-benzamide;

2054. 4-Diethylamino-*N*-[3-methyl-1-(6-oxo-4-pentanoyl-hexahydro-pyrrolo[3,2-*b*]pyrrole-1-carbonyl)-butyl]-benzamide;

2055. 4-Diethylamino-*N*-{3-methyl-1-[4-(3-methyl-pentanoyl)-6-oxo-hexahydro-pyrrolo[3,2-*b*]pyrrole-1-carbonyl]-butyl}-benzamide;

2056. 4-Diethylamino-*N*-{3-methyl-1-[4-(4-methyl-pentanoyl)-6-oxo-hexahydro-pyrrolo[3,2-*b*]pyrrole-1-carbonyl]-butyl}-benzamide;

2057. *N*-{1-[4-(2-Amino-4-methyl-pentanoyl)-6-oxo-hexahydro-pyrrolo[3,2-*b*]pyrrole-1-carbonyl]-3-methyl-butyl}-4-diethylamino-benzamide;

2058. *N*-{1-[4-(2-Acetylamino-4-methyl-pentanoyl)-6-oxo-hexahydro-pyrrolo[3,2-*b*]pyrrole-1-carbonyl]-3-methyl-butyl}-4-diethylamino-benzamide;

2059. *N*-{1-[4-(2-Amino-4,4-dimethyl-pentanoyl)-6-oxo-hexahydro-pyrrolo[3,2-*b*]pyrrole-1-carbonyl]-3-methyl-butyl}-4-diethylamino-benzamide;

2060. *N*-{1-[4-(2-Acetylamino-4,4-dimethyl-pentanoyl)-6-oxo-hexahydro-pyrrolo[3,2-*b*]pyrrole-1-carbonyl]-3-methyl-butyl}-4-diethylamino-benzamide;

2061. *N*-{1-[4-(2-Amino-pentanoyl)-6-oxo-hexahydro-pyrrolo[3,2-*b*]pyrrole-1-carbonyl]-3-methyl-butyl}-4-diethylamino-benzamide;

2062. *N*-{1-[4-(2-Acetylamino-pentanoyl)-6-oxo-hexahydro-pyrrolo[3,2-*b*]pyrrole-1-carbonyl]-3-methyl-butyl}-4-diethylamino-benzamide;

2063. *N*-{1-[4-(2-Amino-5-methyl-hexanoyl)-6-oxo-hexahydro-pyrrolo[3,2-*b*]pyrrole-1-carbonyl]-3-methyl-butyl}-4-diethylamino-benzamide;

2064. *N*-{1-[4-(2-Acetylamino-5-methyl-hexanoyl)-6-oxo-hexahydro-pyrrolo[3,2-*b*]pyrrole-1-carbonyl]-3-methyl-butyl}-4-diethylamino-benzamide;

2065. *N*-{1-[4-(2-hydroxy-3-methyl-butyryl)-6-oxo-hexahydro-pyrrolo[3,2-*b*]pyrrole-1-carbonyl]-3-methyl-butyl}-4-diethylamino-benzamide;

2066. *N*-{1-[4-(2-methoxy-3-methyl-butyryl)-6-oxo-hexahydro-pyrrolo[3,2-*b*]pyrrole-1-carbonyl]-3-methyl-butyl}-4-diethylanuno-benzamide;

2067. 4-Diethylarmino-*N*-{1-[4-(2-hydroxy-3,3-dimethyl-butyryl)-6-oxo-hexahydro-pyrrolo[3,2-*b*]pyrrole-1-carbonyl]-3-methyl-butyl}-benzamide;

2068. 4-Diethylamino-*N*-{1-[4-(2-methoxy-3,3-dimethyl-butyryl)-6-oxo-hexahydro-pyrrolo[3,2-*b*]pyrrole-1-carbonyl]-3-methyl-butyl}-benzamide;

2069. 4-Diethylamino-*N*-{1-[4-(2-hydroxy-3-methyl-pentanoyl)-6-oxo-hexahydro-pyrrolo[3,2-*b*]pycrole-1-carbonyl]-3-methyl-butyl}-benzamide;

2070. 4-Diethylamino-*N*-{1-[4-(2-methoxy-3-methyl-pentanoyl)-6-oxo-hexahydro-pyrrolo[3,2-*b*]pyrrole-1-carbonyl]-3-methyl-butyl}-benzamide;

2071. 4-Diethylamino-*N*-{1-[4-(2-hydroxy-4-methyl-pentanoyl)-6-oxo-hexahydro-pyrrolo[3,2-*b*]pyrrole-1-carbonyl]-3-methyl-butyl}-benzamide;

2072. 4-Diethylamino-*N*-{1-[4-(2-methoxy-4-methyl-pentanoyl)-6-oxo-hexahydro-pyrrolo[3,2-*b*]pyrrole-1-carbonyl]-3-methyl-butyl}-benzamide;

2073. 4-Diethylamino-*N*-{1-[4-(2-hydroxy-4,4-dimethyl-pentanoyl)-6-oxo-hexahydro-pyrrolo[3,2-*b*]pyrrole-1-carbonyl]-3-methyl-butyl}-benzamide;

2074. 4-Diethylamino-*N*-{1-[4-(2-methoxy-4,4-dimethyl-pentanoyl)-6-oxo-hexahydro-pyrrolo[3,2-*b*]pyrrole-1-carbonyl]-3-methyl-butyl}-benzamide;

2075. 4-Diethylamino-*N*-{1-[4-(2-hydroxy-pentanoyl)-6-oxo-hexahydro-pyrrolo[3,2-*b*]pyrrole-1-carbonyl]-3-methyl-butyl}-benzamide;

2076. 4-Diethylamino-*N*-{1-[4-(2-methoxy-pentanoyl)-6-oxo-hexahydro-pyrrolo[3,2-*b*]pyrrole-1-carbonyl]-3-methyl-butyl}-benzamide;

2077. 4-Diethylamino-*N*-{1-[4-(2-hydroxy-5-methyl-hexanoyl)-6-oxo-hexahydro-pyrrolo[3,2-*b*]pyrrole-1-carbonyl]-3-methyl-butyl}-benzamide;

2078. 4-Diethylamino-*N*-{1-[4-(2-methoxy-5-methyl-hexanoyl)-6-oxo-hexahydro-pyrrolo[3,2-*b*]pyrrole-1-carbonyl]-3-methyl-butyl}-benzamide;

To those skilled in the practices of organic chemistry, compounds of general formula (I) may be readily synthesised by a number of chemical strategies, performed either in solution or on the solid phase (see Atherton, E. and Sheppard, R. C. In 'Solid Phase Peptide Synthesis: A Practical Approach', Oxford University Press, Oxford, U.K. 1989**,** for a general review of solid phase synthesis principles). The solid phase strategy is attractive in being able to generate many thousands of analogues, typically on a 5-100mg scale, through established parallel synthesis methodologies (e.g. see (a) Bastos, M.; Maeji, N. J.; Abeles, R. H. Proc. Natl. Acad. Sci. USA, 92, 6738-6742, 1995**).**

Therefore, one strategy for the synthesis of compounds of general formula (I) comprises:-

(a) Preparation of an appropriately functionalised and protected bicyclic ketone building block in solution.
(b) Attachment of the building block (a) to the solid phase through a linker that is stable to the conditions of synthesis, but readily labile to cleavage at the end of a synthesis (see James, I. W., Tetrahedron, 55(Report No 489), 4855-4946, 1999**,** for examples of the 'linker' function as applied to solid phase synthesis).
(c) Solid phase organic chemistry (see Brown, R. D. J. Chem. Soc., Perkin Trans.1, 19, 3293-3320, 1998**),** to construct the remainder of the molecule.
(d) Compound cleavage from the solid phase into solution.
(e) Cleavage work-up and compound analysis.

The first stage in a synthesis of compounds of general formula (I) is the preparation in solution of a functionalised and protected building block. Typical schemes towards the hexahydropyrrolo[3,2-b]pyrrol-3-one **(6)** are detailed in Schemes 1-3, a hexahydropyrrolo[3,2-c]pyrazol-6-one **(21)** in Scheme 4 and a hexahydro-2-oxa-1,4-diazapentalen-6-one **(26)** in Scheme 5. The synthetic descriptions detailed in Schemes 6-18 could equally be applied using each of the scaffolds of general formula (I).

'Pg$_1$' and 'pug2' denotes suitable amine protecting groups such as the 9-fluorenyl methoxycarbonyl (Fmoc, see Atherton, E. and Sheppard, R. C. In 'Solid Phase Peptide Synthesis: A Practical Approach', Oxford University Press, Oxford, U.K. 1989**),** *tert*-butoxycarbonyl (Boc), benzyloxycarbonyl (Cbz) or allyloxycarbonyl (Alloc) for example.

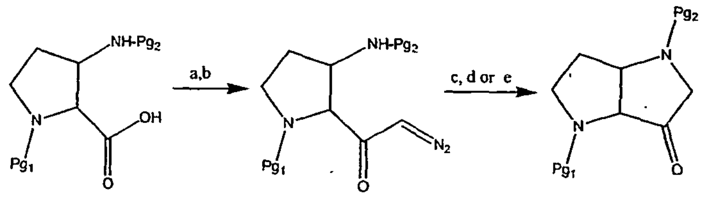


(4) (5) (6)

**Scheme 1.** (a) $^{i}$BuOCOCl, NMM, DCM, -15°C, under argon. (b) Diazomethane in diethyl ether, - 15°C, 30mins, then RT overnight. (c) LiCl (10eq) in 80%aq acetic acid RT overnight. (d) HBr / acetic acid followed by re-addition of $Pg_2$ (if '$Pg_2$' is Boc). (e) Rh(II)$(OAc)_4$, DCM, reflux.

In the illustrated case, condensation with diazomethane provides Z = $CH_2$ in general formula (I). Synthesis may commence from a suitably protected β-aminoproline **(4)** which are described in the literature e.g. Gomez-Vidal, J. A. and Silverman, R. B. Org. Lett., 3(16), 2481-2484, 2001.

Activation of the suitably protected β-aminoproline **(4)** via isobutyl chloroformate mixed anhydride, followed by condensation with diazomethane, yields the diazomethylketone intermediate **(5).** Treatment of diazomethylketone intermediate **(5)** with lithium chloride in aqueous acetic acid provides the protected hexahydropyrrolo[3,2-*b*]pyrrol-3-one **(6).** Alternatively, when $Pg_2$ is Boc, treatment with HBr in acetic acid provides an intermediate bicycle with the secondary amine. HBr salt. This intermediate may be acylated with a variety of reagents e.g. activated carboxylic acids, sulphonyl chlorides, urethane chloroformates to provide many variations of **(6)** where the nitrogen substituent is a suitable protecting group '$Pg_2$' or $R^2C(O)$, $R^2SO_2$, etc. Alternatively, treatment of diazomethylketone intermediate **(5)** with rhodium (II) tetraacetate in dichloromethane provides the hexahydropyrrolo[3,2-*b*]pyrrol-3-one **(6)** (e.g. see Lall, M. S. et al, J. Org. Chem., 67, 1536-1547, 2002. and refs cited therein).

Introduction of simple 'Z' substituents may be achieved by condensation of activated **(4)** with alternatives to diazomethane such as diazoethane (Z = $CHCH_3$, $R^3$ = H, $R^4$ = $CH_3$), or 1-phenyloxydiazoethane (Z = $CHCH_2OPh$, $R^3$ = H, $R^4$ = $CH_2OPh$).

An alternative route towards a suitably protected building block is detailed in Scheme 2. Using an Arndt-Eistert synthesis, a suitably protected 3,4-dehydroproline **(7)** may be homologated by methylene insertion between the α-carbon and carboxylic acid following standard literature methods (e.g. see Meier and Zeller, Angew. Chem. Intl. Ed. Engl., 14, 32-43, 1975 for a review). Conversion of **(7)** into the α-diazomethylketone proceeds via isobutyl chloroformate mixed anhydride, followed by condensation with diazomethane. Wolff rearrangement, e.g. with silver oxide in methanol provides the protected homologated analogue **(8),** e.g. 2-Methoxycarbonylmethyl-2,5-dihydro-pyrrole-1-carboxylic acid tert-butyl ester.

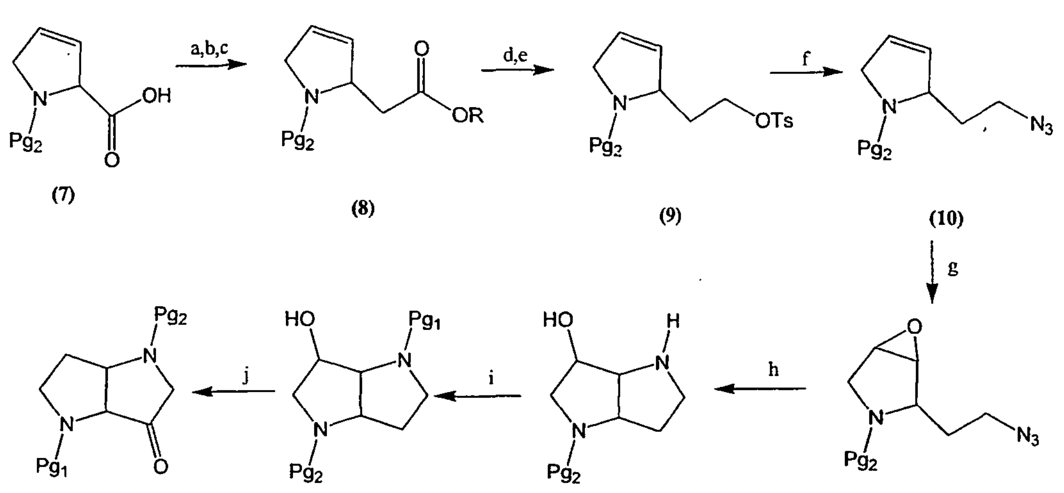


(7) (8) (9) (10) (11) (12) (13) (6)

**Scheme 2.** (a) $^{i}$BuOCOCl, NMM, DCM, -15°C, under argon. (b) Diazomethane in diethyl ether, - 15°C, 30mins, then RT

overnight. (c) Arndt-Eistert, e.g. Silver oxide in methanol (R = $CH_3$). (d) DIBAL reduction. (e) Tosylchloride, pyridine. (f) Sodium azide, DCM / DMF (g) m-chloroperbenzoic acid, DCM. (h) Azide reduction to amine, e.g. Pd-C / $H_2$ in ethanol. (i) Secondary amine protection, 'Pg$_1$' e.g. 1.05 eq Fmoc-Cl, 2.1eq $Na_2CO_3$, dioxan, water, (j) Dess-Martin periodane, DCM.

Treatment of the methyl ester **(8)** with a reducing agent such as DIBAL-H (diisobutylaluminium hydride) provides the primary alcohol, which is readily converted to tosylate **(9).** Similarly the mesylate or triflate analogues of **(9)** may be prepared. Nucleophilic displacement of the activated alcohol with sodium azide provides intermediate **(10)** e.g. 2-(2-Azido-ethyl)-2,5-dihydro-pyrrole-1-carboxylic acid tert-butyl ester. Epoxidation of **(10)** with oxidising agents common to the art such as *m*-CPBA provides the epoxide **(11)** e.g. 2-(2-Azidoethyl)-6-oxa-3-aza-bicyclo[3.1.0]hexane-3-carboxylic acid tert-butyl ester. Reduction of the azide **(11)** to the amine intermediate may be effected under a range of conditions such as Pd-C / $H_2$ or triphenylphosphine in THF and water. The amine intermediates undergo intramolecular epoxide ring opening to provide the bicyclic alcohol **(12)** e.g. 3-Hydroxy-hexahydro-pyrrolo[3,2-b]pyrrole-1-carboxylic acid tert-butyl ester. The free secondary amine **(12)** may be protected with a variety of suitable protecting groups such as Fmoc, Boc, Cbz, Alloc to provide orthogonal protection of the bicyclic scaffold. Protected alcohol **(13)** may be oxidised by reagents common to the art such as pyridine sulphur trioxide complex in DMSO and triethylamine or Dess-Martin periodane to provide ketone **(6)** e.g. 3-Oxo-hexahydro-pyrrolo[3,2-b]pyrrole-1,4-dicarboxylic acid 1-tert-butyl ester 4-(9H-fluoren-9-ylmethyl) ester.

Alternative routes towards intermediate **(9)** (ex Scheme 2) are available such as that detailed in Scheme 3. Protected alkene **(14)** is readily available following literature procedures from the protected homoserine lactol ((a) Wright, D. L. et al, Org. Lett., 2(13), 1847-1850, 2000**.** (b) Boyle, P. H. et al, Tet. Asymm., 6, 2819, 1995**.** (c) Baldwin, J. E. and Flinn, A., Org. Lett., 28, 3605, 1987**.).** N-alkyldtion of **(14)** with a base such as sodium hydride and allyl bromide provides diene **(15).** Treatment of **(15)** with the olefin metathesis catalysts developed by Grubbs such as bis(tricyclohexylphosphine)benzylidine ruthenium (IV) dichloride provides the protected primary alcohol intermediate of compound **(9)** detailed in Scheme 2.

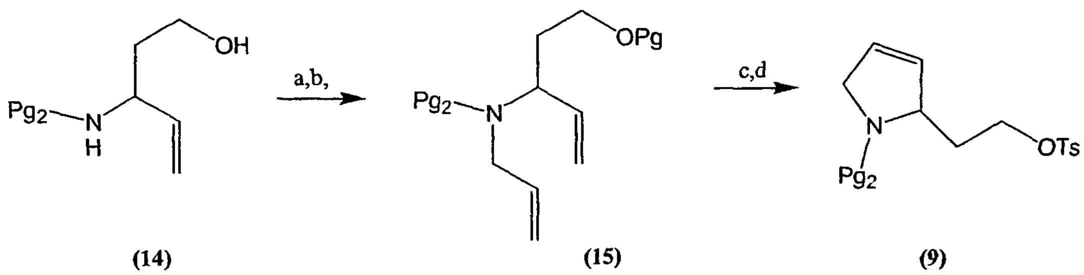


**(14)** **(15)** **(9)**

**Scheme 3.** (a) Primary alcohol protection, e.g. TBDMS-Cl, base (b) NaH, allylbromide, DMF. (c) bis(tricyclohexylphosphine)benzylidine ruthenium (IV) dichloride, DCM, reflux (d) i. TBAF, THF, ii. Tosylchloride, pyridine.

The hexahydropyrrolo[3,2-c]pyrazol-6-one **(21)** scaffold may be prepared following a similar route to that described in Scheme 2 (see Scheme 4). Treatment of the protected 3,4-dehydroproline **(7)** with HCl in methanol provides the methyl ester. Reduction of the ester with a reducing agent such as DIBAL-H (diisobutylaluminium hydride) provides the primary alcohol, which is readily converted to tosylate **(16).** Similarly the mesylate or triflate analogues of **(16)** may be prepared. Nucleophilic displacement of the activated alcohol with a protected hydrazide e.g. Hydrazinecarboxylic acid allyl ester (Alloc-$NHNH_2$) followed by Boc protection of the secondary hydrazide e.g. under standard Schotten-Baumenn conditions and removal of the alloc group e.g. $(PPh_3)_4Pd°$ / DCM / $PhSiH_3$ provides **(17).** Epoxidation of **(17)** with oxidising agents common to the art such as *m*-CPBA provides the epoxide intermediate **(18).** Intermediate **(18)** readily undergoes intramolecular epoxide ring opening to provide the bicyclic alcohol **(19).** The free secondary hydrazide **(19)** may be protected with a variety of suitable protecting groups e.g. Fmoc, Cbz, Alloc to provide orthogonal protection of the bicyclic scaffold. Protected alcohol **(20)** may be oxidised by reagents common to the art such as pyridine sulphur trioxide complex in DMSO and triethylamine or Dess-Martin periodane to provide ketone **(21).**

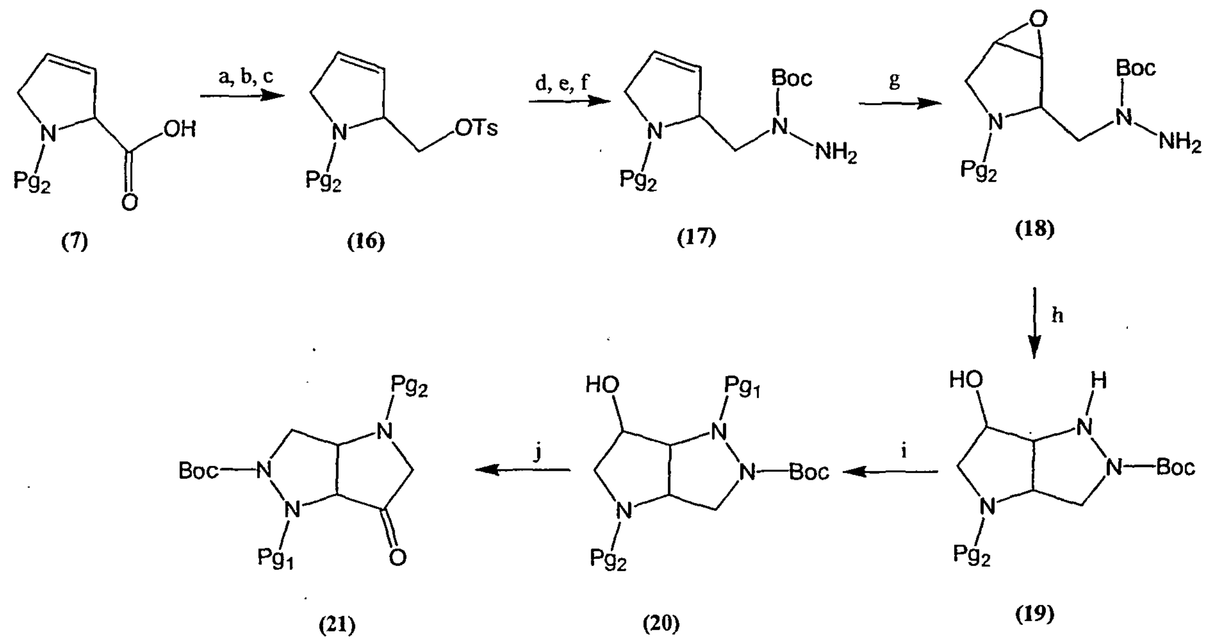


**Scheme 4.** (a) MeOH / HCl, Dean-Stark (b) DIBAL reduction. (c) Tosylchloride, pyridine (d) Alloc-$NHNH_2$ (e) $(Boc)_2O$, $Na_2CO_3$, dioxan, water (f) $(PPh_3)_4Pd°$ / DCM / $PhSiH_3$ (g) m-chloroperbenzoic acid, DCM. (h) Δ (i) $Pg_1$ protection, e.g. 1.05 eq Fmoc-Cl, 2.1eq $Na_2CO_3$, dioxan, water. (j) Dess-Martin periodane, DCM.

The hexahydro-2-oxa-1,4-diazapentalen-6-one **(26)** scaffold may be prepared following a similar route to that described in Scheme 4. Tosylate **(16)** undergoes nucleophilic displacement with a protected oxyamine e.g. N-Boc hydroxylamine to provide intermediate **(22).** Epoxidation of **(22)** with oxidising agents common to the art such as *m*-CPBA provides the epoxide intermediate, which upon acidolytic removal of the Boc group provides intermediate amine.salt **(23).** Neutralisation of the amine.salt initiates intramolecular epoxide ring opening to provide the bicyclic alcohol **(24).** The free secondary amine **(24)** may be protected with a variety of suitable protecting groups e.g. Fmoc, Cbz, Alloc, Boc to provide orthogonal protection of the bicyclic scaffold. Protected alcohol **(25)** may be oxidised by reagents common to the art such as pyridine sulphur trioxide complex in DMSO and triethylamine or Dess-Martin periodane to provide ketone **(26)** e.g. 6-Oxo-tetrahydro-2-oxa-1,4-diaza-pentalene-1,4-dicarboxylic acid 4-benzyl ester 1-(9H-fluoren-9-ylmethyl) ester.

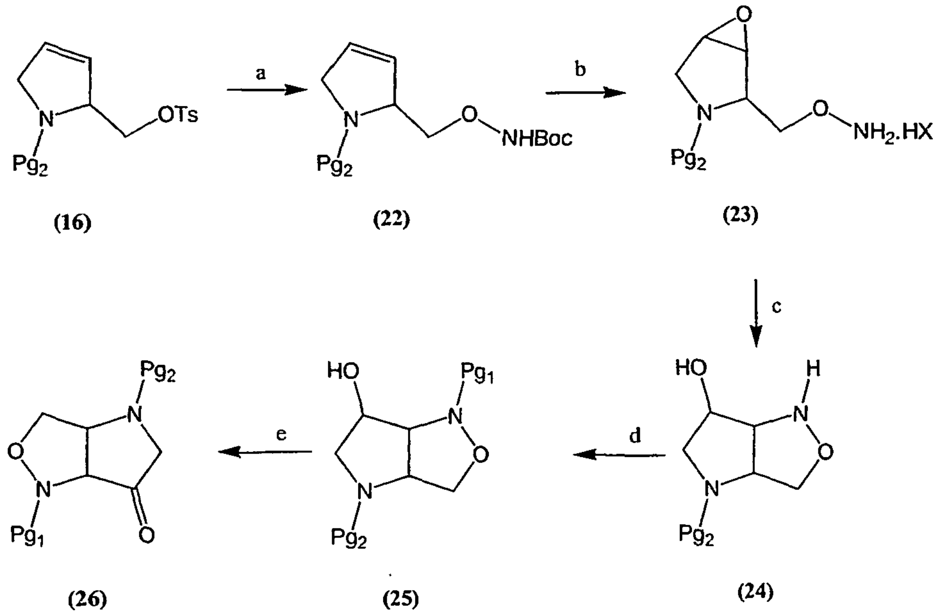


(16) (22) (23)

(26) (25) (24)

**Scheme 5.** (a)Boc-NHOH (b) i. m-chloroperbenzoic acid, DCM. ii. e.g. HCl / dioxan. (c) Base neutralisation e.g. N-methylmorpholine in DMF. (d) $Pg_1$ protection, e.g. 1.05 eq Fmoc-Cl, 2.1eq $Na_2CO_3$, dioxan, water. (e) Dess-Martin periodane, DCM.

The protected building blocks detailed in Schemes 1, 2, 4 and 5 may be utilised in a solid phase synthesis of inhibitor molecules (steps (b) to (e)). Preferred protecting group combinations include '$Pg_1$' = Fmoc / '$Pg_2$' = Boc, or '$Pg_1$' = Fmoc / '$Pg_2$' = Alloc, or '$Pg_1$' = Boc / '$Pg_2$' = Fmoc, or '$Pg_1$' = Boc / '$Pg_2$' = Alloc, or '$Pg_1$' = Alloc / '$Pg_2$' = Fmoc, or '$Pg_1$' = Alloc / '$Pg_2$' = Boc. The solid phase syntheses exemplified in Schemes 6 and 7 by the use of hexahydropyrrolo[3,2-*b*]pyrrol-3-one **(6)** and octahydropyrrolo[3,2-*b*]pyrrol-3-ol **(13)** could equally apply to ketones **(21)** and **(26)** and alcohols **(20)** and **(25).** Step (b), the solid phase linkage of an aldehyde or ketone, has previously been described by a variety of methods (e.g. see (a) James, I. W:, **1999,** (b) Lee, A., Huang, L., Ellman, J. A., J. Am. Chem. Soc, 121(43), 9907-9914, 1999**,** (c) Murphy, A. M., et al, J. Am. Chem. Soc, 114, 3156-3157, 1992**).** A suitable method amenable to the reversible linkage of an alkyl ketone functionality such as **(6)** is through a combination of the previously described chemistries. The semicarbazide, 4-[[(hydrazinocarbonyl)amino]methyl]cyclohexane carboxylic acid. trifluoroacetate (Murphy, A. M., et al, J. Am. Chem. Soc, 114, 3156-3157, 1992**),** may be utilised as illustrated in Scheme 6, where '$Pg_1$' = Fmoc and '$Pg_2$' = Boc or Alloc, exemplified by linkage of the hexahydropyrrolo[3,2-b]pyrrol-3-one **(6).**

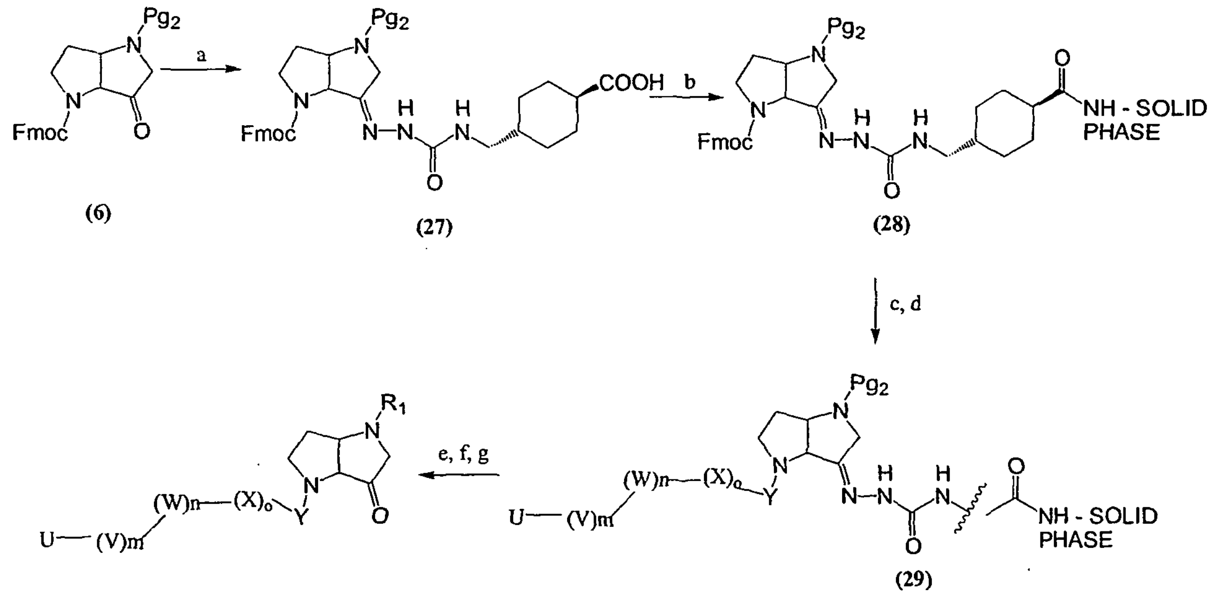


(6) (27) (28) (29)

General formula (I)

**Scheme 6.** (a) **(6)** in 90% EtOH / $H_2O$ / NaOAc / 4-[[(hydrazinocarbonyl)amino]methyl]-cyclohexane carboxylic acid.trifluoroacetate, reflux. (b) 3eq construct **(27)** / 3eq HBTU / 3eq HOBt / 6eq NMM, $NH_2$-SOLID PHASE, DMF, RT, o/n. (c) 20% piperidine / DMF, RT, 30mins. (d) Range of chemistries to add U-V-W-X-Y. (e) 'Pg$_2$' = Boc then 35%TFA in DCM, or 'Pg$_2$' = Alloc then e.g. $(PPh_3)_4Pd(0)$ catalysed deprotection /$CHCl_3$ / DMF / AcOH / NMM (f) i. RCOOH / activation e.g. HBTU / HOBT / NMM, in DMF or ii. $SO_2Cl$, pyridine in DMF. (g) 95% TFA / $H_2O$.

Construct **(28)** is prepared through reaction of the linker molecule **(27)** and the hexahydropyrrolo[3,2-*b*]pyrrol-3-one **(6)** by reflux in aqueous ethanol / sodium acetate. Standard solid phase techniques (e.g. see Atherton, E. and Sheppard, R. C. In 'Solid Phase Peptide Synthesis: A Practical Approach', Oxford University Press, Oxford, U.K. 1989**)** are used to anchor the construct to an amino-functionalised solid phase through the free carboxylic acid functionality of **(27),** providing the loaded construct **(28).** Loaded construct **(28)** may be reacted with a wide range of carboxylic acids or sulphonyl chlorides available commercially in the literature, to introduce the left-hand portion 'U-V-W-X-Y' in general formula (I), providing loaded construct **(29).** Orthogonal removal of 'Pg$_2$' then liberates the secondary amine functionality of the right-hand ring, which may be acylated with a range of carboxylic acid and sulphonyl chlorides. Finally, compounds of general formula (I) are released from the solid phase by treatment with 95% aq trifluoroacetic acid.

An alternative solid phase synthesis of compounds of general formula (I) utilises the bicyclic alcohol intermediate **(13),** Scheme 7. The secondary alcohol may be attached to the solid phase through the acid labile dihydropyran linker **(30)** that is well known in the literature (e.g. see (a) Thompson, L. A. and Ellman, J. A., Tet. Lett., 35, 9333, 1994**.** (b) Kick, E. K. and Ellman, J. A. J. Med. Chem., 38, 1427, 1995**.).** Preferred protecting group combinations include 'Pg$_1$' = Fmoc / 'Pg$_2$' = Alloc, or 'Pg$_1$' = Alloc / 'Pg$_2$' = Fmoc.

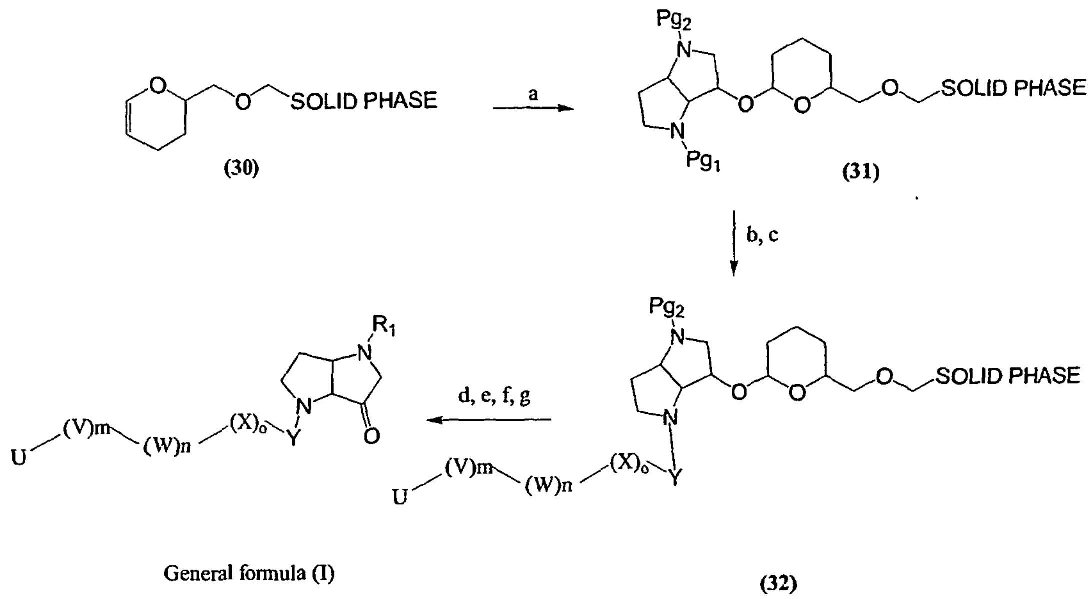


(30) (31) (32)

General formula (I)

**Scheme 7.** (a) **(13)** in dichloroethane, pyridinium p-toluenesulphonate, reflux (b) 'Pg$_1$' = Fmoc then 20% piperidine / DMF, RT, 30mins or 'Pg$_1$' = Alloc then $(PPh_3)_4Pd(0)$ catalysed deprotection /$CHCl_3$ / DMF / AcOH / NMM (c) Range of chemistries to add U-V-W-X-Y. (d) 'Pg$_2$' = Fmoc then 20% piperidine / DMF, RT, 30mins or 'Pg$_2$' = Alloc then e.g. $(PPh_3)_4Pd(0)$ catalysed deprotection /$CHCl_3$ / DMF / AcOH / NMM (e) i. RCOOH / activation e.g. HBTU / HOBT / NMM, in DMF or ii. $SO_2Cl$, pyridine in DMF. (f) 95% TFA / $H_2O$. (g) Solid supported oxidation or e.g. Dess-Martin periodane, DCM.

Loaded construct **(31)** may be reacted with a wide range of carboxylic acids or sulphonyl chlorides available commercially in the literature, to introduce the left-hand portion 'U-V-W-X-Y' in general formula (I), providing loaded construct **(32).** Orthogonal removal of 'Pg$_2$' then liberates the secondary amine functionality of the right-hand ring, which may be acylated with a range of carboxylic acid and sulphonyl chlorides. Compounds of general formula (I) are released from the solid phase by treatment with 95% aq trifluoroacetic acid and the resultant alcohols may be oxidised with a range of solution based reagents e.g. Dess-Martin periodane in DCM or solid supported oxidants (e.g. see Ley, S. V. et al, J. Chem. Soc. Perkin Trans. 1., 3815-4195, 2000.**)** to provide the ketone products.

In the simplest example, the entire left hand portion of an inhibitor of general formula (I) comprises a capped aminoacid (Scheme 8), providing for example analogues of general formula (I) where Z = 'CH$_2$', Y = CHR$^{11}$C(O), $(X)_o$ = '-', $(W)_n$ = 'NH', R$^{18}$ = 'H', n = 1, $(V)_m$ = 'CO', m = 1 and U = phenyl. Scheme 8 details chemistry utilising protected ketone construct **(33)** and the reactions could equally be applied to the protected alcohol construct **(31).**

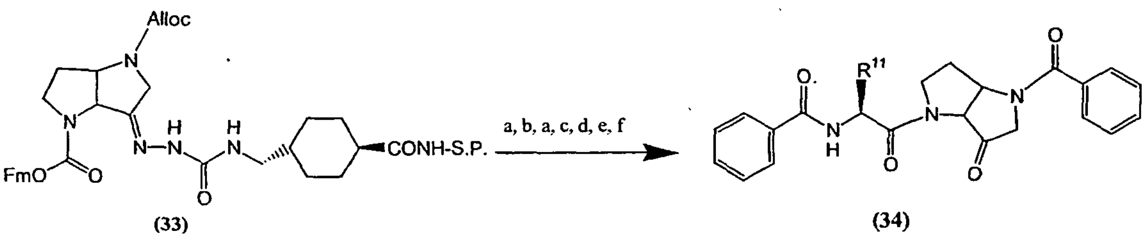


(33) (34)

General formula (I) where
Z = 'CH$_2$'
R$^1$ = PhC(O)
$(X)_o$ = '-'
$(W)_n$ = 'NH', n = 1
$(V)_m$ = 'CO', m = 1
U = phenyl

**Scheme 8.** (a) 20% piperidine / DMF, 30mins (b) 20eq Fmoc-aminoacid / 20eq HBTU / 20eq HOBt / 40eq NMM, DMF, o/n (c) 5eq carboxylic acid / 5eq HBTU / 5eq HOBt / 10eq. NMM, DMF, RT, o/n (d) Deprotection of $Pg_2$ Alloc; e.g. TMS-$N_3$ / TBAF / $(PPh_3)_4Pd°$ / under $N_2$, (e) 20eq Benzoic acid / 20eq HBTU / 20eq HOBt / 40eq NMM, DMF, RT, o/n (f) TFA / $H_2O$ (95:5, v/v), RT.

Alternatively, carboxylic acids can be prepared in solution by traditional organic chemistry methods and coupled to constructs **(28)** and **(31)** on the solid phase (Schemes 9-13). For example (Scheme 9), treatment in solution of an amino acid, exemplified by **(35)** with sodium nitrite / $H_2SO_4$, provides the α-hydroxyacid, exemplified by **(36)** (Degerbeck, F. et al, J. Chem. Soc, Perkin Trans. 1, 11-14, 1993**).** Treatment of α-hydroxyacid, **(36)** with sodium hydride in a dimethylformamide / dichloromethane mixture followed by addition of benzyl bromide, provides (*RS*) 2-benzyloxy-4-methyl-pentanoic acid (37). Coupling of **(37)** to the solid phase construct **(33)** followed by alloc deprotection, benzoylation then cleavage, provides **(38),** an example of general formula (I) where Z = 'CH$_2$', (X)o = '-', $(W)_n$ = 'O', n = 1, $(V)_m$ = 'CH$_2$', m = 1, $R^{19}$ and $R^{20}$ = H and U = phenyl. To those skilled in the practices of organic synthesis, a wide variety of aminoacids such as **(35)** may be converted to the corresponding α-hydroxyacid such as **(36)** following the general conditions detailed. Additionally, benzylbromide may be replaced by any reasonable Ar-$CR^{19}R^{20}$-halide, providing many variations of carboxylic acid **(37)** following the general conditions detailed. In certain instances, it may be advantageous to temporarily protect the carboxylic acid as the methyl ester (for example compound **(43),** Scheme 11) prior to reaction with the alkylhalide. The ester intermediate is then simply hydrolysed to acid **(37).** Analogues of **(38),** exploring a wide range of $(V)_m$ and U in general formula (I) may be prepared through the general conditions detailed in Scheme 9.

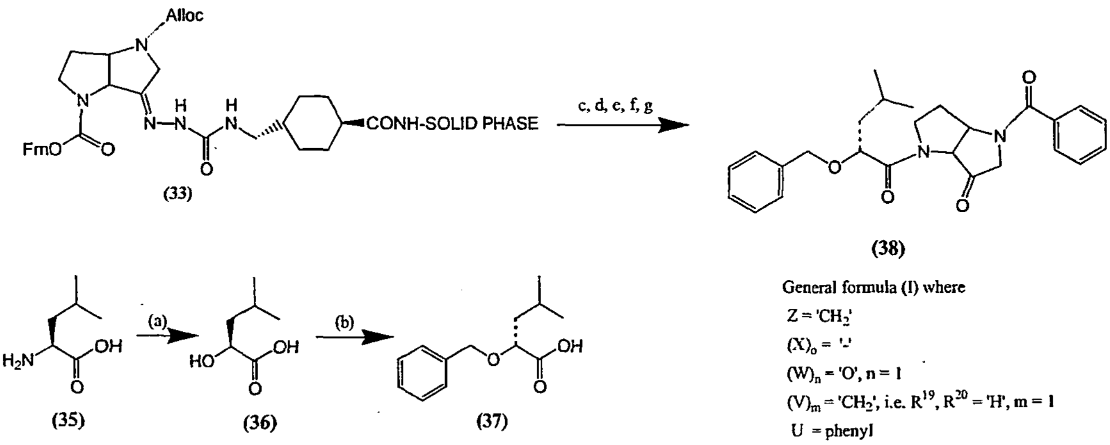

(33) (38) (35) (36) (37)

**Scheme 9.** (a)$NaNO_2$ / $H_2SO_4$, 0°C→RT, 2hr (b) 2.3eq NaH, 1:1 DMF / DCM, 1.4eq benzylbromide, o/n (c) 20% piperidine / DMF, 30mins. (d) 10eq (37) / 10eq HBTU / 10eq HOBt / 20eq NMM, DMF, RT, o/n (e) Deprotection of $Pg_2$ Alloc; e.g. TMS-$N_3$ / TBAF / $(PPh_3)_4Pd^0$ / under $N_2$. (f) 20eq Benzoic acid / 20eq HBTU / 20eq HOBt / 40eq NMM, DMF, RT, o/n (g) TFA / $H_2O$ (95:5, v/v), RT.

Since the final synthetic step involves a trifluoroacetic acid (TFA) mediated cleavage of the solid phase bound compound, analogues where the substituted ether is labile to TFA may be prepared in solution by an alternative route (see Scheme 16).

Alternatively, coupling of construct **(33)** (following removal of Fmoc) with the α-hydroxyacid **(36),** provides a versatile solid phase bound intermediate 'Y' substituent in general formula (I) that may be reacted with many reagents. For example, the α-hydroxyl can be reacted under Mitsunobu conditions (Hughes, D. L. Org. React.(N.Y), 42, 335-656, 1992**)** to give ethers (i.e. X = '-', W = 'O', in general formula (I)) (see Grabowska, U. et al, J. Comb. Chem., 2(5), 475-490, 2000**,** for an example of Mitsunobu reaction on the solid phase). Alternatively, the α-hydroxyl can be reacted with a carbamoyl chloride to give a carbamate (i.e. X = '-', W = 'O', V = 'NHC(O)', in general formula (I)).

Alternatively, (Scheme 10), treatment in solution of an amino acid, exemplified by **(35)** with sodium nitrite / $H_2SO_4$ / potassium bromide provides the α-bromoacid, exemplified by **(39)** (Souers, A. J. et al, Synthesis, 4, 583-585, 1999**)** with retention of configuration. Treatment of α-bromoacid **(39)** with an alkylthiol exemplified by 4-*tert*-butylphenylmethanethiol **(40)** in dimethylformamide / triethylamine, provides 2*R*-(4-*tert*-butylbenzylsulfanyl)-4-methylpropionic acid **(41),** with inversion of configuration. Coupling of **(41)** to the solid phase construct **(33)** followed by alloc deprotection, benzoylation, then cleavage provides **(42),** an example of general formula (I) where Z = 'CH$_2$', $(X)_o$ = '-', $(W)_n$ = 'S', n = 1, $(V)_m$ = 'CH$_2$', m = 1, $R^{19}$ and $R^{20}$ = H and U = 4-*tert*-butylphenyl. To those skilled in the practices of organic synthesis, a wide variety of aminoacids such as **(35)** may be converted to the corresponding α-bromoacid such as **(39)** following the general conditions detailed. Additionally, starting with the *R*-isomer of **(35)** gives the R-bromoacid analogue of **(39)**

and S-thioether analogue of **(41).** Additionally, (4-*tert*-butylphenyl)methanethiol **(40)** may be replaced by any reasonable Ar-$CR^{19}R^{20}$-SH, providing many variations of carboxylic acid **(41)** following the general conditions detailed. Thus analogues of **(42)** exploring a wide range of $(V)_m$ and U in general formula (I) may be prepared through the general conditions detailed in Scheme 10.

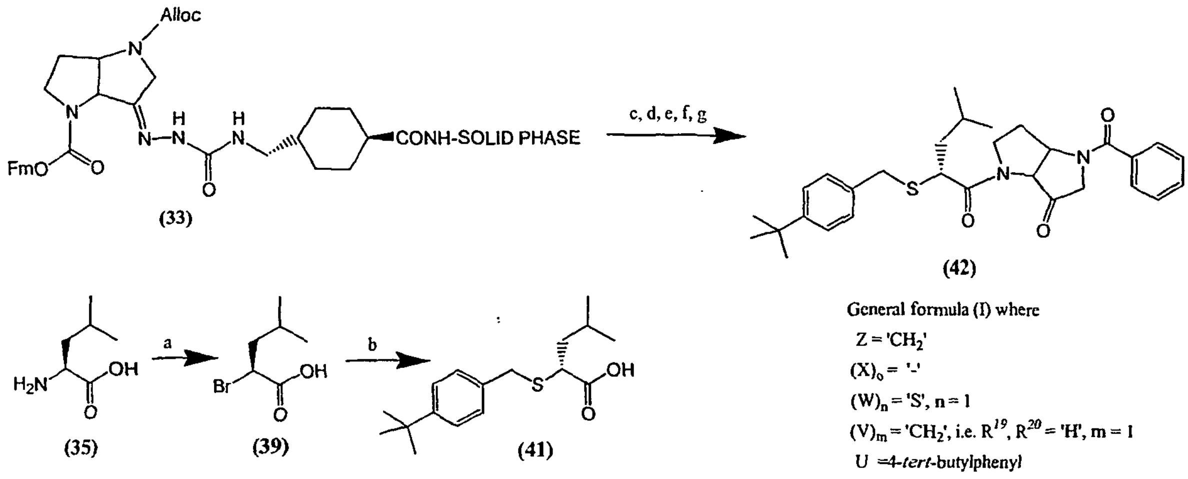

(33) (42) (35) (39) (41)

**Scheme 10.** (a)$NaNO_2$ / $H_2SO_4$, KBr 0°C→RT, 2hr (b) Alkylthiol **(40)** / DMF / $NEt_3$, o/n (c).20% piperidine / DMF, 30mins. (d) 10eq **(41)** / 10eq HBTU / 10eq HOBt / 20eq NMM, DMF, RT, o/n (e) Deprotection of $Pg_2$ Alloc; e.g. TMS-$N_3$ / TBAF / $(PPh_3)_4Pd°$ / under $N_2$. (f) 20eq Benzoic acid / 20eq HBTU / 20eq HOBt / 40eq NMM, DMF, RT, o/n (g) TFA / $H_2O$ (95:5, v/v), RT.

Alternatively, coupling of construct **(33)** (following removal of Fmoc) with an α-bromoacid e.g. **(39),** provides a versatile intermediate 'Y' substituent in general formula (I) that may be reacted with many reagents. For example, the α-bromide can be displaced with nucleophiles e.g. alcohols, thiols, carbanions etc, to give ethers (*i.e.* X = '-', W = 'O', in general formula (I)), thioethers (*i.e.* X = '-', W = 'S', in general formula (I)). The thioethers may optionally be oxidised to the sulphone (see Scheme 14, i.e. X = '-', W = '$SO_2$', in general formula (I)) (see Grabowska, U. et al, J. Comb. Chem., 2(5), 475-490, 2000**,** for an example of bromide displacement and thioether oxidation on the solid phase).

Alternatively, (Scheme 11), treatment of an α-hydroxyacid, exemplified by **(36)** with trimethylsilylchloride and methanol provides the methyl ester **(43).** Activation of the free hydroxyl to the chloroformate with phosgene in dichloromethane followed by addition of morpholine, then hydrolysis, provides morpholine-4-carboxylic acid 1-carboxy-3-methyl-butyl ester **(44).** Coupling of **(44)** to the solid phase construct **(33)** followed by alloc deprotection, benzoylation then cleavage provides **(45),** an example of general formula (I) where Z = '$CH_2$', $(X)_o$ = '-', $(W)_n$ = 'O', n = 1, $(V)_m$ = 'CO' and U = morpholino. To those skilled in the practices of organic synthesis, a wide variety of α-hydroxyacid esters such as **(36)** could be converted to the activated chloroformate following the general conditions detailed. Additionally, morpholine may be replaced by any reasonable amine, providing many variations of carboxylic acid **(44)** following the general conditions detailed. Thus analogues of **(45)** exploring a wide range of $(V)_m$ and U in general formula (I) may be prepared through the general conditions detailed in Scheme 11.

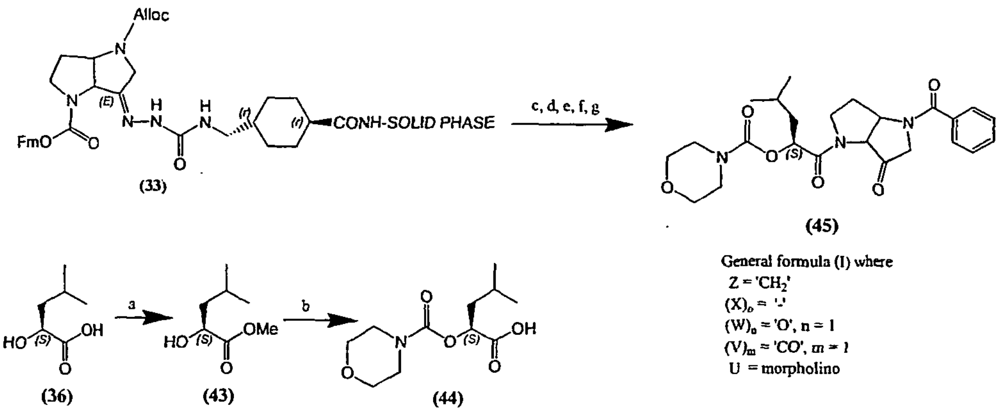


**Scheme 11.** (a) $Me_3SiCl$, MeOH, RT, o/n. (b) i. $COCl_2$ / DCM / o/n, ii. Morpholine / DCM 0°C, 2hr, iii. LiOH in $H_2O$ / dioxan, 0°C. (c) 20% piperidine / DMF, 30mins. (d) 10eq **(44)** / 10eq HBTU / 10eq HOBt / 20eq NMM, DMF, RT, o/n. (e) Deprotection of $Pg_2$ Alloc; e.g. TMS-$N_3$ / TBAF / $(PPh_3)_4Pd^0$ / under $N_2$. (f) 20eq Benzoic acid / 20eq HBTU / 20eq HOBt / 40eq NMM, DMF, RT, o/n. (g) TFA / $H_2O$ (95:5, v/v), RT.

Alternatively, (Scheme 12), a wide range of alkylsuccinate esters exemplified by 2*R*-isobutyl-succinic acid 1-methyl ester **(46)** are commercially available or readily prepared by known methods (see (a) Azam et al, J. Chem. Soc. Perkin Trans. 1, 621-, 1996**;** (b) Evans et al, J. Chem. Soc. Perkin Trans. 1, 103, 2127, 1981**;** (c) Oikawa et al, Tet. Lett, 37, 6169, 1996**).** Carboxyl activation of alkylsuccinate ester **(46)** followed by addition of morpholine in dimethylformamide and subsequent ester hydroylsis, provides 4-Methyl-2*R*-(2-morpholin-4-yl-2-oxo-ethyl)-pentanoic acid **(47).** Coupling of **(47)** to the solid phase construct **(33)** followed by alloc deprotection, benzoylation then cleavage provides **(48),** an example of general formula (I) where Z = 'CH$_2$', $(X)_o$ = 'CH$_2$', $(W)_n$ = 'CO', n = 1, $(V)_m$ = '-' and U = morpholino. To those skilled in the practices of organic synthesis, a wide variety of alkylsuccinate esters such as **(46)** may be prepared and converted to the corresponding substituted alkylsuccinate acid such as **(47)** following the general conditions detailed. Additionally, morpholine may be replaced by any reasonable amine, providing many variations of carboxylic acid **(47)** following the general conditions detailed. Thus analogues of **(48)** exploring a wide range of $(X)_o$, $(V)_m$ and U in general formula (I) may be prepared through the general conditions detailed in Scheme 12.

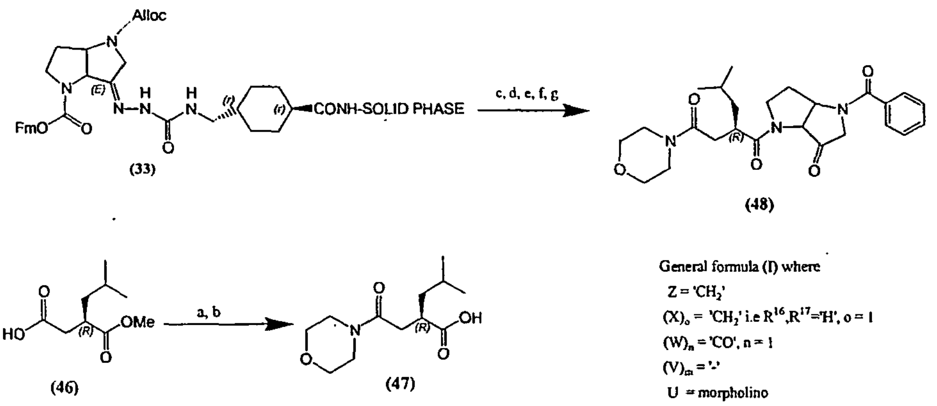


**Scheme 12.** (a) i.EDC / 1-hydroxybenzotriazole /DMF, 0°C, 30mins. ii. Morpholine, RT, o/n (b) LiOH in $H_2O$ / dioxan, 0°C (c) 20% piperidine / DMF, 30mins. (d) 10eq **(47)** / 10eq HBTU / 10eq HOBt / 20eq NMM, DMF, RT, o/n. (e) Deprotection of $Pg_2$ Alloc; e.g. TMS-$N_3$ / TBAF / $(PPh_3)_4Pd^0$ / under $N_2$. (f) 20eq Benzoic acid / 20eq HBTU / 20eq HOBt / 40eq NMM, DMF, RT, o/n. (g) TFA / $H_2O$ (95:5, v/v), RT.

Alternatively, (Scheme 13), a wide range of biarylalkylacetic acids, exemplified by 2*RS*-biphenyl-3-yl-4-methylpentanoic acid (50) are readily available by known methods (see (a) DesJarlais, R. L. et al, J. Am. Chem. Soc, 120, 9114-9115, 1998**;** (b) Oballa, R. M. et al, WO 0149288). Coupling of biarylalkylacetic acid (50) to the solid phase construct **(33)** followed by alloc deprotection, benzoylation then cleavage provides **(51),** an example of general formula (I) where Z = 'CH$_2$', $(X)_o$ = '-', $(W)_n$ = '-', $(V)_m$ = '-' and U = *m*-biphenyl. To those skilled in the practices of organic synthesis, a

wide variety of biarylalkylacetic acids such as **(50)** may be prepared by alkylation of the α-anion of the free acid analogue of **(49)**, which in turn is prepared by Suzuki coupling of phenylboronic acid and 3-bromophenylacetic acid methyl ester. Phenylboronic acid may be replaced by a wide range of arylboronic acids in the Suzuki coupling, providing many variations of carboxylic acid **(50)** following the general conditions detailed. Thus analogues of **(51)** exploring a wide range of group 'U' in general formula (I) may be prepared through the general conditions detailed in Scheme 13.

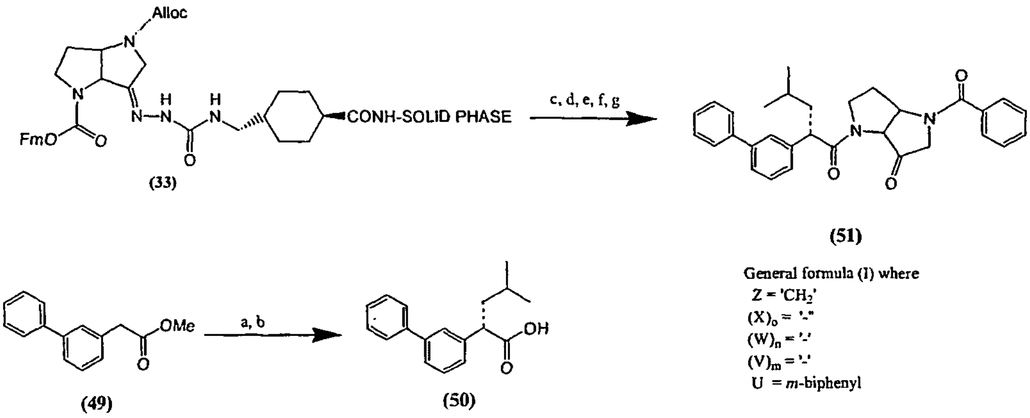


(33)

(51)

(49)

(50)

General formula (I) where
Z = 'CH$_2$'
$(X)_o$ = '-'
$(W)_n$ = '-'
$(V)_m$ = '-'
U = *m*-biphenyl

**Scheme 13.** (a) LiOH in $H_2O$ / dioxan, 0°C (b) i.LDA, THF, 2-methylpropenylbromide. ii. Pd/C, EtOH, $H_2$ (c) 20% piperidine / DMF, 30mins. (d) 10eq **(50)** / 10eq HBTU / 10eq HOBt / 20eq NMM, DMF, RT, o/n. (e) Deprotection of $Pg_2$ Alloc; e.g. TMS-$N_3$ / TBAF / $(PPh_3)_4Pd^0$ / under $N_2$. (f) 20eq Benzoic acid / 20eq HBTU / 20eq HOBt / 40eq NMM, DMF, RT, o/n. (g) TFA / $H_2O$ (95:5, v/v), RT.

Many other possibilities for solid phase organic chemistry (e.g. see Brown, R. D. J. Chem. Soc., Perkin Trans. 1, 19, 3293-3320, 1998**,** for a review of recent SPOC publications) can be used to derivatise constructs **(28)** and **(31)** towards compounds of general formula (I). For example, the left-hand portion 'U-V-W-X-Y' in general formula (I) can be partially constructed in solution, coupled to constructs **(28)** and **(31)** and further modified on the solid phase. For example (Scheme 14), a simple extension of Scheme 10 is through the oxidation of the intermediate solid phase bound species, with *m*-chloroperbenzoic acid in dichloromethane prior to cleavage, to give the sulphone analogue. Commencing from 2*R*-(4-*tert*-butylbenzylsulfanyl)-4-methylpropionic acid **(41),** sulphone **(52)** is prepared, an example of general formula (I) where Z = '$CH_2$', $(X)_o$ = '-', $(W)_n$ = '$SO_2$', n = 1, $(V)_m$ = '$CH_2$', m = 1, $R^{19}$ and $R^{20}$ = H and U = 4-*tert*-butylphenyl. As described in Scheme 10, many variations of carboxylic acid **(41)** may be prepared following the general conditions detailed. Thus analogues of **(52)** exploring a wide range of $(V)_m$ and U in general formula (I) may be prepared through the general conditions detailed in Schemes 10 and 14.

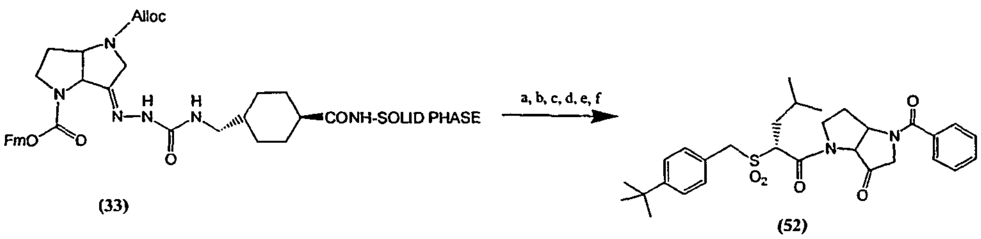


(33)

(52)

General formula (I) where
Z = 'CH$_2$'
$(X)_o$ = '-'
$(W)_n$ = 'SO$_2$', *n* = 1
$(V)_m$ = 'CH$_2$', i.e. $R^{19}$, $R^{20}$ = 'H', m = 1
U = 4-*tert*-butylphenyl

**Scheme 14.** (a) 20% piperidine / DMF, 30mins. (b) 10eq **(41)** / 10eq HBTU / 10eq HOBt / 20eq NMM, DMF, RT, o/n. (c) Deprotection of $Pg_2$ Alloc; e.g. TMS-$N_3$ / TBAF / $(PPh_3)_4Pd^0$ / under $N_2$. (d) 20eq Benzoic acid / 20eq HBTU / 20eq HOBt / 40eq NMM, DMF, RT, o/n. (e) 5eq *m*-chloroperbenzoic acid / DCM, RT, 5hr. (f) TFA / $H_2O$ (95:5, v/v), RT.

Compounds of general formula **(I)** are finally released from the solid phase by treatment with trifluoroacetic

acid / water, followed by evaporation, lyophylis and standard analytical characterisation.

A second strategy for the synthesis of compounds of general formula (I) comprises:-

(a) Preparation of an appropriately functionalised and protected hexahydropyrrolo[3,2-*b*]pyrrol-3-one, hexahydropyrrolo[3,2-*c*]pyrazol-6-one or hexahydro-2-oxa-1,4-diazapentalen-6-one building block in solution.
Preferred protecting groups for solution phase chemistry are the 9-fluorenylmethoxycarbonyl, Nα-*tert*-butoxycarbonyl, Nα-benzyloxy carbonyl and Nα-allyloxycarbonyl group.

(b) Standard organic chemistry methods for the conversion of building block (a) towards compounds of general formula (I).

In the simplest example, the entire left hand portion of an inhibitor of general formula (I) can be prepared in solution by traditional organic chemistry methods and coupled to building block (a) (see Scheme 15 exemplified by the use of 3-Oxo-hexahydro-pyrrolo[3,2-b]pyrrole-1-carboxylic acid allyl ester **(54)).**

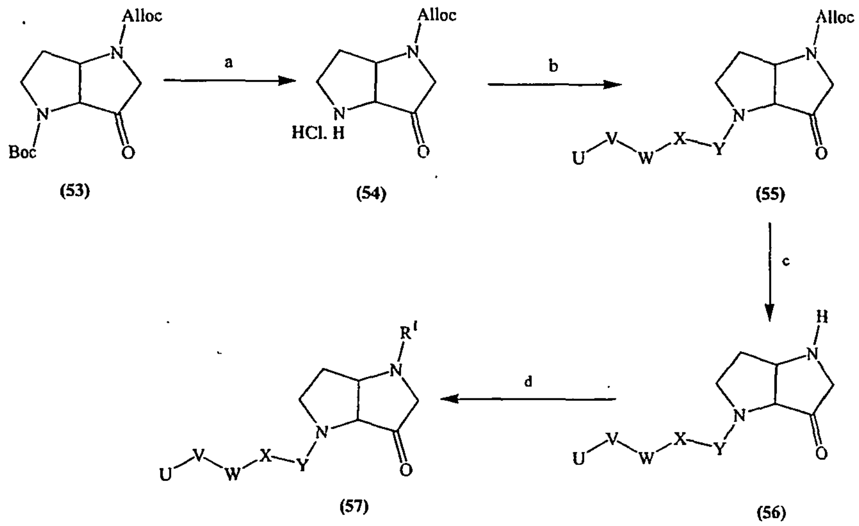


**Scheme 15.** (a) 4M HCl in dioxan, 0°C. (b) Pre-prepared U-V-W-X-Y-COOH / activation e.g. HATU / HOAt / NMM, DMF, RT, o/n. (c) Alloc deprotection e.g. $(PPh_3)_4Pd^o$ / DCM / $PhSiH_3$ (d) Acylation e.g. RCOOH, $^iBuOCOCl$, NMM, DCM, or $SO_2Cl$ / Pyridine.

The general strategy detailed in Scheme 15 is particularly useful when the compound of general formula (I) contains a substituent that is labile to trifluoroacetic acid, this being the final reagent used in each of the solid phase Schemes 6-14. For example (Scheme 16), treatment in solution of α-hydroxyacid (36) with sodium hydride in a dimethylformamide / dichloromethane mixture followed by addition of 4-*tert*-butylbenzyl bromide, provides 2*RS*-(4-*tert*-butylbenzyloxy)-4-methylpentanoic acid **(58).** Coupling of **(58)** to hydrochloride salt **(54),** followed by alloc deprotection then benzoylation provides **(59),** an example of general formula (I) where Z = 'CH$_2$', $(X)_o$ = '-', $(W)_n$ = 'O', n = 1, $(V)_m$ = 'CH$_2$', m = 1, $R^{19}$ and $R^{20}$ = H and U = 4-*tert*-butylphenyl. To those skilled in the practices of organic synthesis, 4-*tert*-butylbenzyl bromide may be replaced by any reasonable Ar-$CR^{19}R^{20}$-halide, providing many variations of carboxylic acid **(58)** under the conditions shown. Thus analogues of **(59)** exploring a wide range of $(V)_m$ and U in general formula (I) may be prepared through the conditions detailed in Scheme 16.

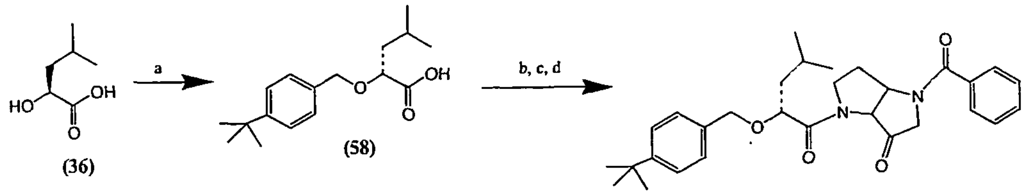


(36) (58) (59)

General formula (I) where
Z = 'CH$_2$'
$(X)_o$ = '-'
$(W)_n$ = 'O', $n = 1$
$(V)_m$ = 'CH$_2$', i.e. $R^{19}$, $R^{20}$ = 'H', $m = 1$
U = 4-*tert*-butylphenyl

**Scheme 16.** (a)2.2eq NaH, 1:1 DMF / DCM, 1.25eq 4-*tert*-benzylbromide. (b)1eq **(58),** 1eq $^i$BuOCOCl, 2eq NMM, DCM, -15°C, 1hr, under nitrogen, then leq, **(54),** *RT*, o/n. (c) Alloc deprotection e.g. $(PPh_3)_4Pd^o$ / DCM / $PhSiH_3$ (d) Acylation e.g. RCOOH, $^i$BuOCOCl, NMM, DCM, or $SO_2Cl$ / Pyridine.

A third strategy for the synthesis of compounds of general formula (I) where the addition of U-V-W-X-Y to the protected building block involves multistep organic reactions comprises:-

(a) Preparation of an appropriately functionalised and protected hexahydropyrrolo[3,2-*b*]pyrrol-3-one, hexahydropyrrolo[3,2-*c*]pyrazol-6-one or hexahydro-2-oxa-1,4-diazapentalen-6-one building block in solution. Preferred protecting groups for solution phase chemistry are the 9-fluorenylmethoxycarbonyl, Nα-*tert*-butoxycarbonyl, Nα-benzyloxy carbonyl and Nα-allyloxycarbonyl group.

(b) Protection of the ketone functionality of the hexahydropyrrolo[3,2-*b*]pyrrol-3-one, hexahydropyrrolo[3,2-*c*]pyrazol-6-one or hexahydro-2-oxa-1,4-diaza pentalen-6-one building block e.g. as a dimethylacetal. Alternatively, the reduced ketone (achiral secondary alcohols **(13), (20)** and **(25))** intermediates may be used and re-oxidised as the final synthetic step.

(c) Standard organic chemistry methods for the conversion of building block (b) towards compounds of general formula (I).

Intermediates may be prepared in solution, followed by coupling to building block (b) and further derivitisation towards compounds of general formula (I) (see Scheme 17 exemplified by preparation and use of the 3-Hydroxyhexahydro-pyrrolo[3,2-b]pyrrole-1-carboxylic acid allyl ester **(61)).**

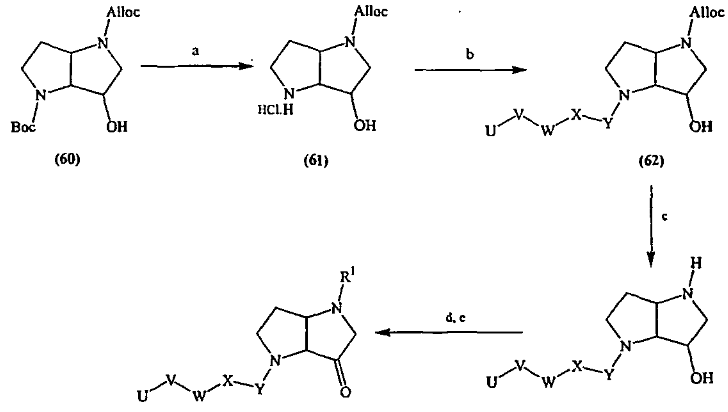


(60) (61) (62) (63) (64)

**Scheme 17.** (a) 4M HCl in dioxan, 0°C. (b) Stepwise reaction with intermediates of Y, then X, then W etc., to stepwise construct compounds **(62).** (c) Alloc deprotection e.g. $(PPh_3)_4Pd^o$ / DCM / $PhSiH_3$ (d) Acylation e.g. RCOOH, $^i$BuOCOCl,

NMM, DCM, or $SO_2Cl$ / Pyridine. (e) Oxidation, e.g. Dess-Martin periodane, $CH_2Cl_2$.

Alternatively, depending upon the types of chemistry used to construct the left hand side U-V-W-X-Y of compounds of general formula (I), the ketone may require protection e.g. as the dimethyl acetal. Such a method is detailed and exemplified in Scheme 18 by the preparation and use of 3,3-Dimethoxy-hexahydro-pyrrolo[3,2-b]pyrrole-1-carboxylic acid allyl ester **(66).**

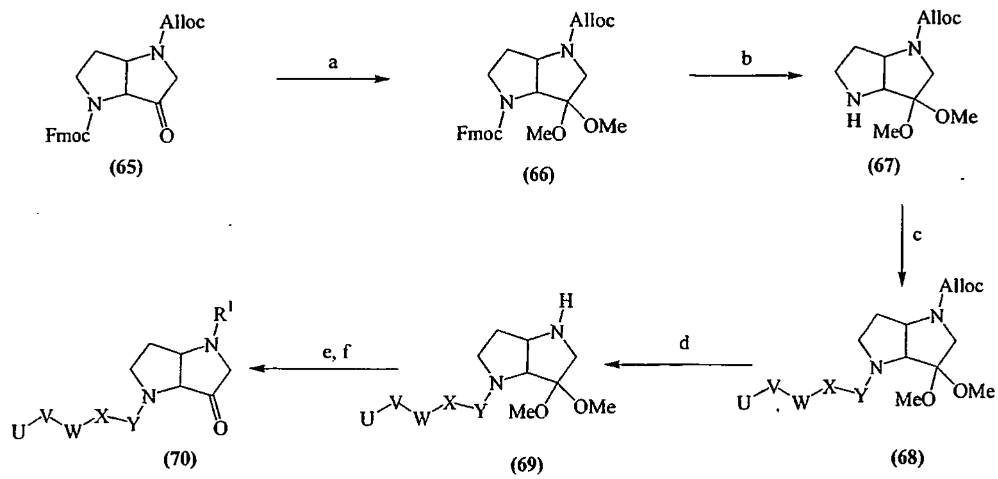


(65) (66) (67) (68) (69) (70)

**Scheme 18.** (a) Triethylorthoformate / pTSA / MeOH. (b) Fmoc deprotection, e.g. Solid supported piperidine / DMF (c) Stepwise reaction with intermediates of Y, then X, then W etc., to stepwise construct compounds **(68).** (d) Alloc deprotection e.g. $(PPh_3)_4Pd^o$ / DCM / $PhSiH_3$ (e) Acylation e.g. RCOOH, $^i$BuOCOCl, NMM, DCM, or $SO_2Cl$ / Pyridine. (f) Trifluoroacetic acid / $CH_2Cl_2$ / $H_2O$.

The invention extends to novel intermediates as described above, and to processes for preparing compounds of general formula (I) from each of their immediate precursors. In turn, processes for preparing intermediates from their immediate precursors also form part of the invention.

Compounds of general formula (I) are useful both as laboratory tools and as therapeutic agents. In the laboratory certain compounds of the invention are useful in establishing whether a known or newly discovered cysteine protease contributes a critical or at least significant biochemical function during the establishment or progression of a disease state, a process commonly referred to as 'target validation'.

According to a second aspect of the invention, there is provided a method of validating a known or putative cysteine protease as a therapeutic target, the method comprising:

(a) assessing the *in vitro* binding of a compound as described above to an isolated known or putative cysteine protease, providing a measure of potency, and optionally, one or more of the steps of:

(b) assessing the binding of the compound to closely related homologous proteases of the target and general house-keeping proteases (e.g. trypsin) to provides a measure of selectivity,

(c) monitoring a cell-based functional marker of a particular cysteine protease activity, in the presence of the compound; and

(d) monitoring an animal model-based functional marker of a particular cysteine protease activity in the presence of the compound.

The invention therefore provides a method of validating a known or putative cysteine protease inhibitor as a therapeutic target. Differing approaches and levels of complexity are appropriate to the effective inhibition and 'validation' of a particular target. In the first instance, the method comprises assessing the *in vitro* binding of a compound of general formula (I) to an isolated known or putative cysteine protease, providing a measure of 'potency'. An additional assessment of the binding of a compound of general formula (I) to closely related homologous proteases of the target and general house-keeping proteases (e.g. trypsin) provides a measure of 'selectivity'. A second level of complexity may be assessed by monitoring a cell-based functional marker of a particular cysteine protease activity, in the presence of a compound of general formula (1). For example, a 'human osteoclast resorption assay' has been utilised as a cell-based secondary

*in vitro* testing system for monitoring the activity of cathepsin K and the biochemical effect of protease inhibitors (e.g. see WO-A-9850533). An 'MHC-II processing - T-cell activation assay' has been utilised as a cell-based secondary *in vitro* testing system for monitoring the activity of cathepsin S and the biochemical effect of protease inhibitors (Shi, G-P., et al, Immunity, 10, 197-206, 1999**).** When investigating viral or bacterial infections such a marker could simply be a functional assessment of viral (e.g. count of mRNA copies) or bacterial loading and assessing the biochemical effect of protease inhibitors. A third level of complexity may be assessed by monitoring an animal model-based functional marker of a particular cysteine protease activity, in the presence of a compound of general formula (1). For example, murine models of *Leishmania* infection, *P. vinckei* infection, malaria (inhibition of falcipain) and *T. cruzi* infection (cruzipain), indicate that inhibition of cysteine proteases that play a key role in pathogen propagation is effective in arresting disease symptoms, 'validating' said targets.

The invention therefore extends to the use of a compound of general formula (I) in the validation of a known or putative cysteine protease as a therapeutic target.

Compounds of general formula (I) are useful for the *in vivo* treatment or prevention of diseases in which participation of a cysteine protease is implicated.

According to a third aspect of the invention, there is provided a compound of general formula (I) for use in medicine, especially for preventing or treating diseases in which the disease pathology may be modified by inhibiting a cysteine protease.

According to a fourth aspect of the invention, there is provided the use of a compound of general formula (I) in the preparation of a medicament for preventing or treating diseases in which the disease pathology may be modified by inhibiting a cysteine protease.

Certain cysteine proteases function in the normal physiological process of protein degradation in animals, including humans, *e.g.* in the degradation of connective tissue. However, elevated levels of these enzymes in the body can result in pathological conditions leading to disease. Thus, cysteine proteases have been implicated in various disease states, including but not limited to, infections by *Pneumocystis carinii, Trypsanoma cruzi, Trypsanonma brucei brucei* and *Crithidia fusiculata*; as well as in osteoporosis, autoimmunity, schistosomiasis, malaria, tumour metastasis, metachromatic leukodystrophy, muscular dystrophy, amytrophy, and the like. See WO-A-9404172 and EP-A-0603873 and references cited in both of them. Additionally, a secreted bacterial cysteine protease from *S. Aureus* called staphylopain has been implicated as a bacterial virulence factor (Potempa, J., et al. J. Biol. Chem, 262(6), 2664-2667, 1998**).**

The invention is useful in the prevention and/or treatment of each of the disease states mentioned or implied above. The present invention also is useful in a methods of treatment or prevention of diseases caused by pathological levels of cysteine proteases, particularly cysteine proteases of the papain superfamily, which methods comprise administering to an animal, particularly a mammal, most particularly a human, in need thereof a compound of the present invention. The present invention particularly provides methods for treating diseases in which cysteine proteases are implicated, including infections by *Pneumocystis carinii, Trypsanoma cruzi, Trypsanoma brucei, Leishmania mexicana, Clostridium histolyticum, Staphylococcus aureus*, foot-and-mouth disease virus and *Crithidia fusiculata*; as well as in osteoporosis, autoimmunity, schistosomiasis, malaria, tumour metastasis, metachromatic leukodystrophy, muscular dystrophy and amytrophy.

Inhibitors of cathepsin K, particularly cathepsin K-specific compounds, are useful for the treatment of osteoporosis, Paget's disease, gingival diseases such as gingivitis and periodontitis, hypercalaemia of malignancy, metabolic bone disease, diseases involving matrix or cartilage degradation, in particular osteoarthritis and rheumatoid arthritis and neoplastic diseases.

In accordance with this invention, an effective amount of a compound of general formula (I) may be administered to inhibit the protease implicated with a particular condition or disease. Of course, this dosage amount will further be modified according to the type of administration of the compound. For example, to achieve an "effective amount" for acute therapy, parenteral administration of a compound of general formula (I) is preferred. An intravenous infusion of the compound in 5% dextrose in water or normal saline, or a similar formulation with suitable excipients, is most effective, although an intramuscular bolus injection is also useful. Typically, the parenteral dose will be about 0.01 to about 100 mg/kg; preferably between 0.1 and 20 mg/kg, in a manner to maintain the concentration of drug in the plasma at a concentration effective to inhibit a cysteine protease. The compounds may be administered one to four times daily at a level to achieve a total daily dose of about 0.4 to about 400 mg/kg/day. The precise amount of an inventive compound which is therapeutically effective, and the route by which such compound is best administered, is readily determined by one of ordinary skill in the art by comparing the blood level of the agent to the concentration required to have a therapeutic effect. Prodrugs of compounds of the present invention may be prepared by any suitable method. For those compounds in which the prodrug moiety is a ketone functionality, specifically ketals and/or hemiacetals, the conversion may be effected in accordance with conventional methods.

The compounds of this invention may also be administered orally to the patient, in a manner such that the concentration of drug is sufficient to inhibit bone resorption or to achieve any other therapeutic indication as disclosed herein. Typically, a pharmaceutical composition containing the compound is administered at an oral dose of between

about 0.1 to about 50 mg/kg in a manner consistent with the condition of the patient. Preferably the oral dose would be about 0.5 to about 20 mg/kg.

No unacceptable toxicological effects are expected when compounds of the present invention are administered in accordance with the present invention. The compounds of this invention, which may have good bioavailability, may be tested in one of several biological assays to determine the concentration of a compound which is required to have a given pharmacological effect.

According to a fifth aspect of the invention, there is provided a pharmaceutical or veterinary composition comprising one or more compounds of general formula (I) and a pharmaceutically or veterinarily acceptable carrier. Other active materials may also be present, as may be considered appropriate or advisable for the disease or condition being treated or prevented.

The carrier, or, if more than one be present, each of the carriers, must be acceptable in the sense of being compatible with the other ingredients of the formulation and not deleterious to the recipient.

The formulations include those suitable for rectal, nasal, topical (including buccal and sublingual), vaginal or parenteral (including subcutaneous, intramuscular, intravenous and intradermal) administration, but preferably the formulation is an orally administered formulation. The formulations may conveniently be presented in unit dosage form, e.g. tablets and sustained release capsules, and may be prepared by any methods well known in the art of pharmacy.

Such methods include the step of bringing into association the above defined active agent with the carrier. In general, the formulations are prepared by uniformly and intimately bringing into association the active agent with liquid carriers or finely divided solid carriers or both, and then if necessary shaping the product. The invention extends to methods for preparing a pharmaceutical composition comprising bringing a compound of general formula (I) in conjunction or association with a pharmaceutically or veterinarily acceptable carrier or vehicle.

Formulations for oral administration in the present invention may be presented as: discrete units such as capsules, cachets or tablets each containing a predetermined amount of the active agent; as a powder or granules; as a solution or a suspension of the active agent in an aqueous liquid or a non-aqueous liquid; or as an oil-in-water liquid emulsion or a water in oil liquid emulsion; or as a bolus etc.

For compositions for oral administration (e.g. tablets and capsules), the term "acceptable carrier" includes vehicles such as common excipients e.g. binding agents, for example syrup, acacia, gelatin, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Flavouring agents such as peppermint, oil of wintergreen, cherry flavouring and the like can also be used. It may be desirable to add a colouring agent to make the dosage form readily identifiable. Tablets may also be coated by methods well known in the art.

A tablet may be made by compression or moulding, optionally with one or more accessory ingredients. Compressed tablets may be prepared by compressing in a suitable machine the active agent in a free flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may be optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerin, or sucrose and acacia; and mouthwashes comprising the active agent in a suitable liquid carrier.

Parenteral formulations will generally be sterile.

According to a sixth aspect of the invention, there is provided a process for the preparation of a pharmaceutical or veterinary composition as described above, the process comprising bringing the active compound(s) into association with the carrier, for example by admixture.

Preferred features for each aspect of the invention are as for each other aspect *mutatis mutandis*.

## Experimental Procedures

### Solution Phase Chemistry - General Methods

All solvents were purchased from ROMIL Ltd (Waterbeach, Cambridge, UK) at SpS or Hi-Dry grade unless otherwise stated. General peptide synthesis reagents were obtained from Chem-Impex Intl. Inc. (Wood Dale IL 60191. USA). Thin layer chromatography (TLC) was performed on pre-coated plates (Merck aluminium sheets silica 60 F254, part no. 5554). Visualisation of compounds was achieved under ultraviolet light (254nm) or by using an appropriate staining reagent. Flash column purification was performed on silica gel 60 (Merck 9385) or Isolute Flash silica cartridge.

All analytical HPLC were obtained on Phenomenex Jupiter $C_4$, 5μ, 300A, 250 x 4.6mm, using mixtures of solvent A = 0.1%aq trifluoroacetic acid (TFA) and solvent B = 90% acetonitrile / 10% solvent A on automated Agilent systems with 215 and / or 254nm UV detection. Unless otherwise stated a gradient of 10 - 90% B in A over 25 minutes at 1.5mL / min was performed for full analytical HPLC analysis. HPLC-MS analysis was performed on an Agilent 1100 series LC/MSD, using automated Agilent HPLC systems, with a gradient of 10 - 90% B in A over 10 minutes on Phenomenex Columbus $C_8$, 5μ, 300A, 50 x 2.0mm at 0.4mL / min. Nuclear magnetic resonance (NMR) were obtained on a Bruker DPX400 (400MHz 1H frequency; QXI probe) or Bruker DPX500 (500MHz 1H frequency) in the solvents and temperature indicated (298K unless otherwise stated). Chemical shifts are expressed in parts per million (δ) and are referenced to residual signals of the solvent. Coupling constants (*J*) are expressed in Hz. High resolution mass spectrometry was performed on a Micromass QTOF 1.

Solid Phase Chemistry - General Methods

Example inhibitors were prepared through a combination of solution and solid phase Fmoc-based chemistries (see 'Solid Phase Peptide Synthesis', Atherton, E. and Sheppard, R. C., IRL Press Ltd, Oxford, UK, 1989**,** for a general description). An appropriately protected and functionalised building block was prepared in solution (e.g. general compound **(6),** Scheme 6), then reversibly attached to the solid phase through an appropriate linker followed by rounds of coupling / deprotection / chemical modification (Scheme 6). Example inhibitors were then released (cleaved) from the solid phase, analysed, purified and assayed for inhibition verses a range of proteases.
Generally, multipins (polyamide 1.3 → 10μmole loadings, see www.mimotopes.com) were used for the solid phase synthesis, although any suitable solid phase surface could be chosen. In general, the 1.3μmole gears were used to provide small scale crude examples for preliminary screening, whilst the 10μmole crowns were used for scale-up synthesis and purification of preferred examples. Standard coupling and Fmoc deprotection methods were employed (see Grabowska, U. et al, J. Comb. Chem. 2(5), 475-490, 2000**.** for a thorough description of solid phase multipin methodologies).

Preparation of Initial Assembly

Building Block-linker constructs (e.g. **(27),** typically 10mg to 100mg) were carboxyl activated with 2-(1H-benzotriazole-1-yl)-1,1,3,3-tetramethyluronium hexafluoro phosphate (HBTU, 1 mole equivalent), 1-hydroxybenzotriazole.hydrate (HOBT, 1 mole equivalent) and N-methylmorpholine (NMM, 2 mole equivalents) in dimethylformamide (DMF, typically 1 to 10mL) for 5 minutes. Amino functionalised DA/MDA crowns or HEMA gears (10μmole per crown / 1.2μmole per gear, 0.33 mole equivalent of total surface amino functionalisation compared to activated construct) were added, followed by additional DMF to cover the solid phase surface. The loading reaction was left overnight. Following overnight loading, crowns / gears were taken through standard cycles washing, Fmoc deprotection and loading quantification (see Grabowska, U. *et al*) to provide loaded Building Block-linker constructs (e.g.(**28**)).

Coupling Cycles

The coupling of standard Fmoc-aminoacids (10 or 20 mole equivalent) were performed via carboxyl activated with 2-(1H-benzotriazole-1-yl)-1,1,3,3-tetramethyluronium hexafluoro phosphate (HBTU, 10 or 20mole equivalent), 1-hydroxybenzotriazole.hydrate (HOBT, 10 or 20mole equivalent) and N-methylmorpholine (NMM, 20 or 40mole equivalents) in dimethylformamide, with pre-activation for 5 minutes. Activated species were dispensed to the appropriate wells of a polypropylene 96-well plate (Beckman, 1mL wells, 500μL solution per well for crowns or 250μL solution per well for gears) in a pattern required for synthesis. Loaded free amino Building Block-linker constructs (e.g.(**28**)) were added and the coupling reaction left overnight. Following overnight coupling, crowns / gears were taken through standard cycles washing and Fmoc deprotection (see Grabowska, U. *et al*). Identical activation and coupling conditions were used for the coupling of a range of carboxylic acids (R-COOH). Alternatively, chloroformates e.g. morpholine-4-carbonylchloride (10mole equivalent), were coupled in DMF with the addition of NMM (10mole equivalents).

Acidolytic Cleavage Cycle

A mixture of 95% TFA / 5% water was pre-dispensed into two polystyrene 96-well plates (Beckman, 1mL wells, 600μL solution per well for crowns or 300μL solution per well for gears) in a pattern corresponding to that of the synthesis. The completed multipin assembly was added to the first plate (mother plate), the block covered in tin foil and cleaved for 2 hours. The cleaved multipin assembly was then removed from the first plate and added to the second plate (washing plate) for 15 minutes. The spent multipin assembly was then discarded and the mother / washing plates evaporated on an HT-4 GeneVac plate evaporator.

Analysis and Purification of Cleaved Examples

(a) Ex 1.2μmole Gears. 100μL dimethylsulphoxide (DMSO) was added to each post cleaved and dried washing plate well, thoroughly mixed, transferred to the corresponding post cleaved and dried mother plate well and again thoroughly mixed. 10μL of this DMSO solution was diluted to 100μL with a 90% acetonitrile / 10% 0.1 %aq TFA mixture. 20μL aliquots were analysed by HPLC-MS and full analytical HPLC. In each case the crude example molecules gave the expected $[M + H]^+$ ion and an HPLC peak at > 80% (by 215nm UV analysis). This provided an approximately 10mM DMSO stock solution of good quality crude examples for preliminary protease inhibitory screening.

(b) Ex 10μmole Crowns. 500μL of a 90% acetonitrile / 10% 0.1 %aq TFA mixture was added to each washing plate well, thoroughly mixed, transferred to the corresponding mother plate well and again thoroughly mixed. 5μL of this solution was diluted to 100μL with a 90% acetonitrile / 10% 0.1 %aq TFA mixture. 20μL aliquots were analysed by HPLC-MS and full analytical HPLC. In each case the crude example molecules gave the expected $[M + H]^+$ ion and an HPLC peak at > 80% (by 215nm UV analysis). The polystyrene blocks containing crude examples were then lyophilised.

(c) Individual examples (ex (b)) were re-dissolved in a 1 : 1 mixture of 0.1% aq TFA / acetonitrile (1mL) and purified by semi-preparative HPLC (Phenomenex Jupiter $C_4$, 5μ, 300A, 250 x 100mm, a 25-90% B in A gradient over 25mins, 4.0mL/min, 215nm UV detection). Fractions were lyophilised into pre-tarred glass sample vials to provide purified examples (typically 2 to 4mg, 40 to 80% yield).

(d) Purified examples were dissolved in an appropriate volume of DMSO to provide a 10mM stock solution, for accurate protease inhibitory screening.

EXAMPLES 1 - 248 were prepared using the general solid phase descriptions above and are inhibitors of cathepsin K with Ki ranging from 1-5000nM;

EXAMPLE 1. (3*aR*, 6*aS*)-*N*-[(1*S*)-1-(4-Benzoyl-6-oxo-hexahydro-pyrrolo[3,2-*b*] pyrrole-1-carbonyl)-3-methyl-butyl]-4-*tert*-butylbenzamide

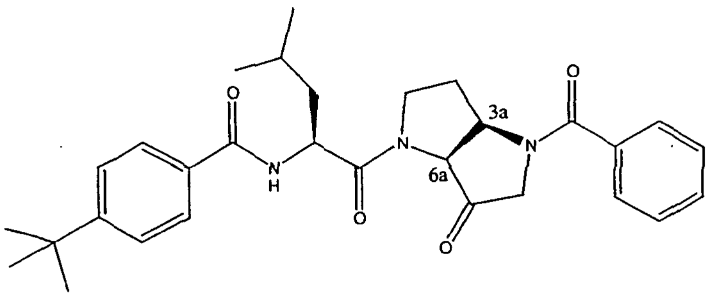

Following the general details from Scheme 1, the required bicycle building block (3a*S*,6a*R*) 3-Oxo-hexahydro-pyrrolo[3,2-b]pyrrole-1,4-dicarboxylic acid 1-tert-butyl ester 4-(9H-fluoren-9ylmethyl) ester (6) was prepared in 8 steps as follows;

(1) Preparation of (2*S*,3*S*) 3-hydroxypyrrolidine-1,2-dicarboxylic acid 1-(9H-fluoren-9-ylmethyl) ester. Trans-3-hydroxy-L-proline (10.0g, 76.3mmole) was added to a vigorously stirred, ice-cooled solution of sodium carbonate (16.90g, 160.2mmole) in water (100mL). 1,4-Dioxan (75mL) was added providing an opaque but mobile mixture. 9-Fluorenylmethyl chloroformate (20.31g, 80mmole) in 1,4-dioxan (75mL) was added over 1hr, then the ice-cooling removed and the mixture stirred at RT for an additional 2hr. Additional water (300mL) was added, the reaction mixture washed with chloroform (2 x 250mL) and the combined organic layers discarded. The aqueous phase was acidified with 1N HCl to ~ pH 2, providing a thick opaque mixture. The acidified aqueous mixture was extracted with chloroform (2 x 500mL) and the now clear aqueous phase discarded. The opaque combined chloroform layers were dried ($Na_2SO_4$), filtered and reduced in vacuo to provide batch 1 (5.70g). The residual precipitate (a mixture of product and drying agent) was triturated with hot methanol (2 x 250mL) and the combined methanol solutions

reduced in vacuo to provide batch 2 (10.25g). Batch 1 and 2 were individually analysed by TLC (single UV spot, Rf = 0.15, 20% MeOH in $CHCl_3$), and HPLC-MS (single main UV peak with Rt = 7.069mins, 354.2 $[M + H]^+$, 376.2 $[M + Na]^+$) and found to be identical, giving a combined yield of 15.95g (45.2mmole, 59.2%). Analysis by $^1H$ and $^{13}C$ NMR showed the presence of cis and trans geometrical isomers around the 3° amide bond.
$\delta_H$ (DMSO-$d_6$ at 298K); 1.80-2.02 ($2H_\gamma$, m), 3.49-3.62 ($2H_\delta$, m), 4.12-4.38 ($H_\alpha$, Hp, Fmoc H-9 and C<u>H</u>$_2$, m), 5.55/5.62 (O<u>H</u>), 7.30-7.31 (2H aromatic, Fmoc H-2 and H-7), 7.35-7.37 (2H aromatic, Fmoc H-3 and H-6), 7.43-7.45 (2H aromatic, Fmoc H-1 and H-8), 7.63-7.65 (2H aromatic, Fmoc H-4 and H-5), 12.8-13.0 (COO<u>H</u>); $\delta_C$ (DMSO-$d_6$ at 298K); 31.70/32.70 (d, $C_\gamma$), 44.68/45.32 (d, $C_\delta$), 46.94/46.97 (u, Fmoc C-9), 67.04/67.33 (d, Fmoc <u>C</u>H$_2$), 68.24/68.51 (u, $C_\alpha$), 73.12/74.23 (u, $C_\beta$), 120.49/120.52 (u, Fmoc C-4 and C-5), 125.49/125.58 (u, Fmoc C-1 and C-8), 127.50 (u, Fmoc C-2 and C-7), 128.04 (u, Fmoc C-3 and C-6), 140.99/141.09 (q, Fmoc C-4' and C-5'), 144.02/144.16 (q, Fmoc C-1' and C-8'), 154.33/154.54 (q, O<u>C</u>ON), 172.10/172.39 (<u>C</u>OOH).

(2) Preparation of (2*S*,3*R*) 3-hydroxypyrrolidine-1,2-dicarboxylic acid 2-allyl ester 1-(9H-fluoren-9-ylmethyl) ester.
(2*S*,3*S*) (3-hydroxy)pyrrolidine-1,2-dicarboxylic acid 1-(9H-fluoren-9-ylmethyl) ester (10.9g, 30.8mmole) was dissolved in toluene (75mL) in a Dean-Stark apparatus. Allyl alcohol (20mL) was added followed by *p*-toluenesulphonic acid.hydrate (6.05g, 31.4mmole). The mixture was refluxed for 1hr, cooled and $CHCl_3$ (300mL) added. The organic layer was washed with $NaHCO_3$ (300mL), 0.1N HCl (300mL) and brine (300mL), then dried ($Na_2SO_4$). Filtration and reduction in vacuo gave a pale yellow foam (13.5g). The crude foam was purified over silica gel (150g) eluting with a gradient of heptane : ethyl acetate 3:1 → 1:1. Desired fractions were combined and reduced in vacuo to a colourless gum yield 10.34g (26.3mmole, 85.4%). TLC (single UV spot, Rf = 0.30, heptane : ethyl acetate 1:1), analytical HPLC Rt = 18.849mins, HPLC-MS (single main UV peak with Rt = 8.354mins, 394.2 $[M + H]^+$, 416.2 $[M + Na]^+$). Analysis by $^1H$ and $^{13}C$ NMR showed the presence of cis and trans geometrical isomers around the 3° amide bond.
$\delta_H$ ($CDCl_3$ at 298K); 2.00-2.21 ($2H_\gamma$, m), 2.70/2.85 (O<u>H</u>, b), 3.72-3.81 ($2H_\delta$, m), 4.12-4.67 ($H_\alpha$, Hp, Fmoc H-9 and C<u>H</u>$_2$, 2 x COOC<u>H</u>$_2$CH=CH$_2$, m), 5.20-5.40 (2 x COOCH$_2$CH=C<u>H</u>$_2$, m), 5.82-5.99 (1 x COOCH$_2$C<u>H</u>=CH$_2$, m), 7.28-7.33 (2H aromatic, Fmoc H-2 and H-7), 7.34-7.41 (2H aromatic, Fmoc H-3 and H-6), 7.53-7.66 (2H aromatic, Fmoc H-1 and H-8), 7.77-7.81 (2H aromatic, Fmoc H-4 and H-5); $\delta_C$ ($CDCl_3$ at 298K); 32.28/33.04 (d, $C_\gamma$), 44.98/45.32 (d, $C_\delta$), 47.56/47.63 (u, Fmoc C-9), 66.44 (d, COO<u>C</u>H$_2$CH=CH$_2$), 68.01/68.11 (d, Fmoc <u>C</u>H$_2$), 68.32/68.72 (u, $C_\alpha$), 74.49/75.67 (u, $C_\beta$), 119.20/119.48 (d, COOCH$_2$CH=<u>C</u>H$_2$), 120.34/120.37 (u, Fmoc C-4 and C-5), 125.36/125.60 (u, Fmoc C-1 and C-8), 127.47 (u, Fmoc C-2 and C-7), 128.06/128.12 (u, Fmoc C-3 and C-6), 131.79/131.94 (u, COOCH$_2$<u>C</u>H=CH$_2$), 141.65/141.71 (q, Fmoc C-4' and C-5'), 144.12/144.34 (q, Fmoc C-1' and C-8'), 155.13/155.59 (q, O<u>C</u>ON), 170.53/170.55 (<u>C</u>OOCH$_2$CH=CH$_2$).

(3) Preparation of (2*S*,3*R*) 3-azidopyrrolidine-1,2-dicarboxylic acid 2-allyl ester 1-(9H-fluoren-9-ylmethyl) ester.
Diethyl azodicarboxylate (1.24 ml, 7.9 mmol) was added dropwise over 20 minutes to a stirred solution of triphenylphosphine (2.07 g, 7.9 mmol) in tetrahydrofuran (30 ml) at 0°C. The mixture was stirred for 5 minutes at 0°C then a solution of (2*S*, 3*S*)-3-hydroxypyrrolidine-1,2-dicarboxylic acid 2-allyl ester 1-(9*H*-fluoren-9-ylmethyl) ester (2.59 g, 6.6 mmol) and hydrazoic acid (14.3 ml of 0.7M solution in toluene) in tetrahydrofuran (30 ml) was added dropwise over 35 minutes. The mixture was stirred for 5 minutes at 0°C then at ambient temperature for 14 hours. The solvent was removed *in vacuo* and the residue purified by flash chromatography over silica gel eluting with a gradient of heptane : ethyl acetate 5:1 → 3:1.
Appropriate fractions were combined and the solvents removed *in vacuo* to obtain (2*S*, 3*R*) 3-azidopyrrolidine-1,2-dicarboxylic acid allyl ester 1-(9*H*-fluoren-9-ylmethyl) ester as a colourless oil (1.45 g, 53%). TLC (single UV spot, Rf = 0.30, heptane : ethyl acetate 3:1), analytical HPLC main UV peak with Rt = 19.896mins and HPLC-MS 419.2 $[M+H]^+$,441.2 $[M+Na]^+$.
$\delta_H$ ($CDCl_3$ at 298K); 2.08-2.25 (2H, H-4, m), 3.52-3.59 (1H, H-5, m), 3.68-3.76 (1H, H-5, m), 4.15 (0.5H, Fmoc-C<u>H</u>$_2$, t, *J* = 6.6Hz), 4.24 (0.5H, Fmoc-C<u>H</u>$_2$, t, *J* = 7.1Hz), 4.33-4.38 (2H, H-3 and Fmoc-C<u>H</u>, m), 4.44-4.48 (1.5H, 0.5H-2 and Fmoc-C<u>H</u>, m), 4.51-4.66 (1.5H, 0.5H-2 and C<u>H</u>$_2$CH=CH$_2$, m), 4.67-4.70 (1H, C<u>H</u>$_2$CH=CH$_2$, m), 5.21-5.40 (2H, CH$_2$CH=C<u>H</u>$_2$, m), 5.84-5.98 (1H, CH$_2$C<u>H</u>=CH$_2$, m), 7.26-7.32 (2H, aromatic, Fmoc H-2 and H-7), 7.37-7.40 (2H, aromatic, Fmoc H-3 and H-6), 7.51-7.60 (2H, aromatic, Fmoc H-1 and H-8), 7.74-7.77 (2H, aromatic, Fmoc H-4 and H-5); $\delta_C$ ($CDCl_3$ at 298K); 29.14/30.13 (d, C-5), 44.40/44.72 (d, C-5), 47.12/47.21 (u, Fmoc-<u>C</u>H), 61.02/61.87 (u, C-3), 61.63/62.07 (u, C-2), 66.17 (d, Fmoc-<u>C</u>H$_2$), 67.65 (d, <u>C</u>H$_2$CH=CH$_2$), 118.86/119.11 (d, CH$_2$CH=<u>C</u>H$_2$), 119.94/124.83/124.95/125.05/127.03/127.69 (u, aromatic, Fmoc-<u>C</u>H), 131.384/131.50 (u, CH$_2$<u>C</u>H=CH$_2$), 141.29 (q, aromatic Fmoc quaternary carbon b), 143.49/143.65/143.92 (q, aromatic Fmoc quaternary carbon a), 154.07/154.49 (q, Fmoc-<u>C</u>O), 168.62/168.70 (q, allyl-<u>C</u>O).

(4) Preparation of (2*S*,3*R*) 3-azidopyrrolidine-1,2-dicarboxylic acid 1-(9H-fluoren-9-ylmethyl) ester.
Dichloromethane (30 ml) then phenyltrihydrosilane (0.81 ml, 6.6 mmol) were added consecutively to a stirred mixture

of tetrakistriphenylphosphine palladium(0) (76 mg, 0.066 mmol) and (2*S*, 3*R*)-3-azidopyrrolidine-1,2-dicarboxylic acid allyl ester 1-(9*H*-fluoren-9-ylmethyl) ester (1.38 g, 3.3 mmol) under argon. The mixture was stirred for 30 minutes then diluted with chloroform (200 ml) and washed with 0.01M hydrochloric acid (200 ml). The aqueous layer was extracted with chloroform (100 ml), then the combined chloroform layers were dried ($Na_2SO_4$) and the solvent removed *in vacuo*. The brown residue was purified by flash chromatography over silica gel eluting with a gradient of heptane : ethyl acetate 3.5:1 → 0:1 followed by methanol : dichloromethane 1 : 4. Appropriate fractions were combined and the solvents removed *in vacuo* to leave *(2S,* 3*R*) 3-azidopyrrolidine-1,2-dicarboxylic acid 1-(9*H*-fluoren-9-ylmethyl) ester as a brown foam (890 mg, 71%). TLC (main UV spot, Rf = 0.20, methanol : chloroform 1:9), analytical HPLC main UV peak with Rt = 16.528mins and HPLC-MS 379.2 $[M+H]^+$, 401.1 $[M+Na]^+$, 779.3 $[2M+Na]^+$.

(5) Preparation of (2*S*,3*R*) 3-aminopyrrolidine-1,2-dicarboxylic acid 1-(9H-fluoren-9-ylmethyl) ester.
Acetic acid was added to a suspension of (2*S*, 3*R*) 3-azidopyrrolidine-1,2-dicarboxylic acid 1-(9*H*-fluoren-9-ylmethyl) ester (3.25 g, 8.6 mmol), palladium on carbon (10%, 320 mg) and ethanol (80 ml) under an atmosphere of argon. The mixture was then stirred under an atmosphere of hydrogen for 3.5 hours then the hydrogen was replaced with argon and the suspension stored at 0°C for 14 hours. A further portion of palladium on carbon (10%, 150 mg) was added then the mixture stirred at ambient temperature for 3 hours under an atmosphere of hydrogen. The catalyst was removed by filtration *in vacuo* through a pad of celite which was washed with acetic acid : water (1:1, 150 ml). The filtrate was concentrated *in vacuo* then toluene (50 ml) was added to the residue and solvents removed *in vacuo*. A further portion of toluene was added (50 ml) and the solvent removed *in vacuo* to leave an oily residue which was triturated with diethyl ether (125 ml) to obtain (2*S*, 3*R*) 3-aminopyrrolidine-1,2-dicarboxylic acid 1-(9*H*-fluoren-9-ylmethyl) ester acetate as a pale brown solid (1.05 g, 30%). Analytical HPLC single UV peak with Rt = 12.541mins and HPLC-MS 353.2 $[M+H]^+$, 705.3 $[2M+Na]^+$.

(6) Preparation of (2*S*,3*R*) 3-*tert*-Butoxycarbonylamino-pyrrolidine-1,2-dicarboxylic acid 1-(9H-fluoren-9-ylmethyl) ester
A solution of di-*tert*-butyl dicarbonate (210 mg, 0.96 mmol) in 1,4-dioxan (10 ml) was added to a stirred suspension of (2*S*, 3*R*)-3-aminopyrrolidine-1,2-dicarboxylic acid 1-(9*H*-fluoren-9-ylmethyl) ester (360 mg, 0.87 mmol) and sodium carbonate (195 mg, 1.84 mmol) in water (10 ml) and 1,4-dioxan (10 ml) over 1 hour at 0°C. The reaction mixture was stirred for 16 hours at ambient temperature then the majority of solvents were removed *in vacuo*. The residue was dissolved in dichloromethane (200 ml) and water (100 ml) then acidified to pH ~ 2.5 using 1M hydrochloric acid. The dichloromethane layer was separated then the aqueous layer extracted with dichloromethane. The combined dichloromethane layers were dried ($Na_2SO_4$) and the solvent removed *in vacuo*. The orange-brown residue was purified by flash chromatography over silica gel eluting with a gradient of dichloromethane : methanol 19:1 → 9:1. Appropriate fractions were combined and the solvents removed *in vacuo* to leave (2*S*, *3R)* 3-*tert*-butoxycarbonylaminopyrrolidine-1,2-dicarboxylic acid 1-(9*H*-fluoren-9-ylmethyl) ester as a light brown solid (235 mg, 60%). TLC (single UV spot, Rf = 0.25, methanol : chloroform 1:9), analytical HPLC single UV peak with Rt = 17.476mins and HPLC-MS 397.2 $[M-Bu+2H]^+$, 475.2 $[M+Na]^+$, 927.4 $[2M+Na]^+$.
$\delta_H$ ($CDCl_3$ at 298K); 1.35 (2H, brs $\underline{Me_3}C$), 1.48 (1H, brs $\underline{Me_3}C$), 1.75-2.20 (2H, m, H-4), 2.75-3.85 (4H, m, H-5, H-3, Fmoc-C$\underline{H}$), 3.85-4.60 (4H, m, Fmoc-C$\underline{H_2}$, H-2 and NH), 6.20-6.75 (0.5H, brs, NH), 7.05-7.90 (8H, aromatic); $\delta_C$ ($d_6$-DMSO at 298K); 1.39 and 1.46 (9H total, each s, $\underline{Me_3}C$), 1.70-1.85 (1H, m, H-4), 1.70-1.85 (1H, m, H-4), 3.24-3.35 (1H, m, H-5), 3.44-3.54 (1H, m, H-5), 4.02-4.30 (5H, m, H-2, H-3, Fmoc-C$\underline{H_2}$ and Fmoc-C$\underline{H}$), 6.80 and 7.0 (1H total, each brs, N$\underline{H}$), 7.30-7.98 (8H, aromatic)

(7) Preparation of (2*S*,3*R*) 3-*tert*-Butoxycarbonylamino-2-(2-diazo-acetyl)-pyrrolidine-1-carboxylic acid 9H-fluoren-9-ylmethyl ester.
A solution of *iso*-butyl chloroformate (68 μl, 0.52 mmol) in dichloromethane (2 ml) and a solution of 4-methylmorpholine (105 μl, 0.95 mmol) in dichloromethane (2 ml) were simultaneously added in portions to a stirred suspension of (2*S*, *3R*)-3-*tert*-butoxycarbonylaminopyrrolidine-1,2-dicarboxylic acid 1-(9*H*-fluoren-9-ylmethyl) ester (215 mg, 0.48mmol) in dichloromethane (5 ml) at -15 °C over 20 minutes under an atmosphere of nitrogen. The solution was stirred for 2 hours then additional solutions of *iso*-butyl chloroformate (15 μl, 0.115 mmol) in dichloromethane (0.5 ml) and 4-methylmorpholine (26 μl, 0.237 mmol) in dichloromethane (0.5 ml) were simultaneously added in one portion. The mixture was stirred for 30 minutes at -15°C then ethereal diazomethane [~15mmol generated from diazald (4.7 g mmol) addition in diethyl ether (75 ml) to sodium hydroxide (5.25 g) in water (7.5 ml)/ethanol (15 ml) at 65°C] was cautiously added and the resulting yellow solution stirred at room temperature for 16 hrs. Acetic acid (~1 ml) was cautiously added (until effervescence had ceased), then the mixture was diluted with diethyl ether (100 ml). The ethereal layer was washed with water (3 x 100 ml), dried ($Na_2SO_4$) and the solvents removed *in vacuo* to leave an oily residue (250 mg) which was purified by flash chromatography over silica gel eluting with a gradient of heptane : ethyl acetate 2:1 → 1:1. Appropriate fractions were combined and the solvents removed *in vacuo* to leave

(2*S*, 3*R*) 3-*tert*-butoxycarbonylamino-2-(2-diazoacetyl)pyrrolidine-1-carboxylic acid 9*H*-fluoren-9-ylmethyl ester as a pale yellow solid (91 mg, 40%). TLC (single UV spot, Rf = 0.4, heptane : ethyl acetate 1:1), analytical HPLC main UV peak with Rt = 18.363mins and HPLC-MS 449.2 $[M-N_2+H]^+$, 499.2 $[M+Na]^+$, 975.5 $[2M+Na]^+$.

(8) Cyclisation to (3aS,6aR) 3-Oxo-hexahydro-pyrrolo[3,2-*b*]pyrrole-1,4-dicarboxylic acid 1-*tert*-butyl ester 4-(9H-fluoren-9-ylmethyl) ester (6)

A solution of (2*S*, 3*R*) 3-*tert*-butoxycarbonylamino-2-(2-diazoacetyl) pyrrolidine-1-carboxylic acid 9*H*-fluoren-9-ylmethyl ester (100 mg, 0.21 mmol) in chloroform (2.5 ml) was added dropwise over 28 minutes to a stirred suspension of rhodium (II) acetate dimer (10 mg) in toluene (2.5 ml) at 75°C under an atmosphere of argon. The mixture was stirred for an additional 30 minutes at this temperature then the solvents removed in *vacuo* to leave an oily residue which was purified by flash chromatography over silica gel eluting with a gradient of hexane : ethyl acetate 3:1 → 1:1. Appropriate fractions were combined and the solvents removed *in vacuo* to leave (3a*S*, 6a*R*) 3-oxo-hexahydropyrrolo[3,2-*b*]pyrrole-1,4-dicarboxylic acid 1-*tert*-butyl ester 4-(9*H*-fluoren-9-ylmethyl) ester as a white solid (28 mg, 30%). TLC (two UV spots, major and minor Rf = 0.30 and 0.35 respectively, hexane : ethyl acetate 7:3), analytical HPLC broad group of UV peaks with Rt = 20.043-21.472mins and HPLC-MS 449.2 $[M +H]^+$, 471.2 $[M+Na]^+$, 919.4 $[2M+Na]^+$.

$\delta_H$ ($CDCl_3$ at 298K); (Spectrum poorly resolved) 1.46 (9H, s, $\underline{Me_3}C$), 1.85-2.35 (2H, m, H-6), 3.2-5.0 (9H, m, 2 x H-2, H-3a, 2 x H-5, H-6a, Fmoc-$C\underline{H_2}$, Fmoc-C$\underline{H}$), 7.2-7.85 (8H, aromatic).

Alternatively, following the general details from Scheme 2, the required bicycle building block (3a*S*,6a*R*) 3-Oxo-hexahydro-pyrrolo[3,2-*b*]pyrrole-1,4-dicarboxylic acid 1-*tert*-butyl ester 4-(9H-fluoren-9ylmethyl) ester **(6)** was prepared following Schemes 19 and 20;

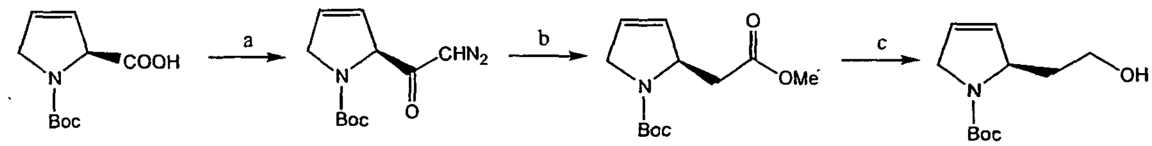


**(71)** **(72)** **(73)** **(74)**

**Scheme 19.** (a) i.$^i$BuOCOCl, NMM, $CH_2Cl_2$, -15 °C; ii. Ethereal $CH_2N_2$, -15 °C to RT. (b) MeOH, THF, $CF_3CO_2Ag$, NMM, 0 °C to RT in dark. (c) DIBAL-H / THF or $LiBH_4$ / MeOH / THF

**Preparation of (*S*)-2-(2-diazoacetyl)-2,5-dihydropyrrole-1-carboxylic acid *tert*-butyl ester (72)**

2,5-Dihydropyrrole-1,2-dicarboxylic acid 1-*tert*-butyl ester **(71)** (1.066 g, 5 mmol) was dissolved with stirring in anhydrous dichloromethane (40 ml). The reaction was flushed with nitrogen and cooled to -15 °C. *iso*-Butylchloroformate (0.713 ml, 5.5 mmol) in anhydrous dichloromethane (5 ml) and 4-methylmorpholine (1.099 ml, 10 mmol) in anhydrous dichloromethane (5 ml) were added simultaneously in 1 ml aliquots over 50 minutes. The mixture was stirred for 2.5 hours at -15 °C then ethereal diazomethane [~15 mmol generated from addition of diazald (4.7 g) in diethyl ether (75 ml) onto sodium hydroxide (5.25 g) in water (7.5 ml) / ethanol (15 ml) at 60 °C] was added to the activated amino acid solution. The mixture was allowed to warm to ambient temperature and stirred for 2.5 hours. A few drops of acetic acid were cautiously added to the mixture, followed by dichloromethane (40 ml). The ethereal layers were washed with aqueous saturated sodium hydrogen carbonate solution (2x 40 ml), dried ($Na_2SO_4$) and the solvents removed *in vacuo* to leave a yellow residue (1.4 g). Flash chromatography of the residue over silica (35 g) eluting with ethyl acetate : heptane 3 : 7 gave (*S*)-2-(2-diazoacetyl)-2,5-dihydropyrrole-1-carboxylic acid *tert*-butyl ester **(72)** (1.024 g, 86%). TLC (Single spot, $R_f$= 0.47, EtOAc : heptane 1 : 1), analytical HPLC $R_t$ = 11.537 min; HPLC-MS 497.2 $[2M + Na]^+$; $d_H$ (500 MHz, $CDCl_3$) 1.41-1.51 (9H, m, $C(CH_3)_3$), 4.11-4.35 (2H, m, $BocNCH_2$), 4.86-5.05 (1H, m, BocNC*H*), 5.25-5.50 (1H, m, $CHN_2$), 5.70-5.80 (1H, m, olefinic C*H*) and 5.88-6.03 (1H, m, olefinic C*H*); $d_C$ (125 MHz, $CDCl_3$) 28.3 and 28.4 ($C(\underline{C}H_3)_3$), 51.8 and 52.3 ($\underline{C}HN_2$), 53.65 and 54.0 ($BocN\underline{C}H_2$), 71.5 and 72.3 (BocN$\underline{C}$H), 80.6 and 80.9 ($O\underline{C}(CH_3)_3$), 126.1 and 126.3 (olefinic $\underline{C}$H), 128.35 and 128.5 (olefinic $\underline{C}$H), 153.7 and 154.15 ($N\underline{C}O_2$), 192.7 and 193.4 ($\underline{C}OCHN_2$).

**Preparation of (*R*)-2-methoxycarbonylmethyl-2,5-dihydropyrrole-1-carboxylic acid *tert*-butyl ester (73)**

(*S*)-2-(2-Diazoacetyl)-2,5-dihydropyrrole-1-carboxylic acid *tert*-butyl ester **(72)** (912 mg, 3.85 mmol) was dissolved in tetrahydrofuran (14 ml) and methanol (1.6 ml) then cooled to 0 °C. A solution of silver trifluoroacetate (94 mg)

in 4-methylmorpholine (1.06 ml) was added, and the mixture allowed to warm to ambient temperature over 6 hours in the dark. Methanol (40 ml) was added, followed by 10% aqueous citric acid solution (100 ml). The majority of the organic solvents were removed *in vacuo* then the aqueous phase extracted with ethyl acetate (3x 40 ml). The combined organic layers were washed with brine (40 ml), dried ($Na_2SO_4$) and evaporated *in vacuo* to afford a residue (1.35 g). Flash chromatography of the residue over silica (200 g) eluting with ethyl acetate : hexane 3 : 17 afforded (*R*)-2-methoxycarbonylmethyl-2,5-dihydropyrrole-1-carboxylic acid *tert*-butyl ester **(73)** as a colourless oil (670 mg, 72%). TLC (Single spot, $R_f$ = 0.27, EtOAc : hexane 1 : 4), analytical HPLC $R_t$ = 15.033 min; HPLC-MS 505.3 $[2M + Na]^+$; $d_H$ (500 MHz, $CDCl_3$) 1.44-1.53 (9H, m, $C(CH_3)_3$), 2.37-2.55 (1H, m, $CH_2CO_2Me$), 2.90-4.00 (1H, m, $CH_2CO_2Me$), 3.63-3.70 (3H, m, $OCH_3$), 3.97-4.26 (2H, m, $BocNCH_2$), 4.70-4.90 (1H, m, BocNC*H*), 5.74-5.89 (2H, m, 2x olefinic C*H*); $d_C$ (125 MHz, $CDCl_3$) 28.2, 28.3 and 28.5 ($C(\underline{C}H_3)_3$), 39.4 and 38.4 ($\underline{C}H_2CO_2Me$), 51.5 and 51.6 ($O\underline{C}H_3$), 53.3 and 53.5 ($BocN\underline{C}H_2$), 60.7 and 60.9 (BocN$\underline{C}$H), 79.6 and 80.0 ($O\underline{C}(CH_3)_3$), 126.0 and 126.1 (olefinic $\underline{C}$H), **129.3** and 129.5 (olefinic $\underline{C}$H), 153.9 ($N\underline{C}O_2$), 171.5 and 171.7 ($\underline{C}O_2Me$).

**Preparation of (*R*)-2-(2-hydroxyethyl)-2,5-dihydropyrrole-1-carboxylic acid *tert*-butyl ester (74)**

Diisobutylaluminium hydride (1M solution in tetrahydrofuran, 13.6 ml, 13.6 mmol) was added dropwise over 20 minutes to a stirred solution of (*R*)-2-methoxycarbonylmethyl-2,5-dihydropyrrole-1-carboxylic acid *tert*-butyl ester **(73)** (630 mg, 2.61 mmol) in tetrahydrofuran (20 ml) at -78 °C under a nitrogen atmosphere. The mixture was stirred for 2 hours at -78 °C then at ambient temperature for 18 hours. Methanol (11.94 ml) was slowly added to the mixture, followed by ethyl acetate (40 ml) and magnesium sulfate. The resultant slurry was vigorously stirred for 2 hours, then filtered and the solid residue washed with excess ethyl acetate. The filtrate was evaporated *in vacuo* to afford a residue (1.4 g). Flash chromatography of the residue over silica gel (150 g) eluting with ethyl acetate : hexane 7 : 13 gave (*R*)-2-(2-hydroxyethyl)-2,5-dihydropyrrole-1-carboxylic acid *tert*-butyl ester **(74)** (430 mg, 77%). TLC (Single spot, $R_f$ = 0.37, EtOAc : hexane 1 : 1), analytical HPLC $R_t$ = 12.161 min; HPLC-MS 236.1 $[M + Na]^+$, 449.3 $[2M + Na]^+$; $[a]_D^{22}$ -112° (c=1, $CHCl_3$); $d_H$ (500 MHz, $CDCl_3$) 1.42-1.55 (10H, br. s, $C(CH_3)_3$ and $NCHCH_2$), 1.84-1.95 (1H, m, $NCHCH_2$), 3.60-3.72 (2H, m, $CH_2OH$), 3.93-4.28 (2H, m, $BocNCH_2$), 4.53-4.78 (1H, m, BocNC*H*), 5.67-5.84 (2H, m, 2x olefinic C*H*); $d_C$ (125 MHz, $CDCl_3$) 28.4 ($C(\underline{C}H_3)_3$), 37.4 and 38.7 ($\underline{C}H_2CH_2OH$), 53.45 and 53.6 ($N\underline{C}H_2$), 59.2 and 59.6 ($O\underline{C}H_2$), 61.2 and 61.9 (BocN$\underline{C}$H), 79.9 and 80.1 ($O\underline{C}(CH_3)_3$), 124.4 and 125.3 (olefinic $\underline{C}$H), 130.3 and 131.1 (olefinic $\underline{C}$H), 154.4 and 156.0 ($N\underline{C}O_2$).

**Alternative preparation of (*R*)-2-(2-hydroxyethyl)-2,5-dihydropyrrole-1-carboxylic acid *tert*-butyl ester (74)**

Methanol (0.27 ml, 6.7 mmol) was added dropwise to a stirred suspension of lithium borohydride (146 mg, 6.6 mmol) in tetrahydrofuran (3.5 ml) under an atmosphere of argon over 4 minutes, followed by a solution of (*R*)-2-methoxycarbonylmethyl-2,5-dihydropyrrole-1-carboxylic acid *tert*-butyl ester **(73)** (0.8 g, 3.3 mmol) in tetrahydrofuran (8 ml) over 15 minutes. The mixture was stirred for 1 hour then poured into water (25 ml). The product was extracted into dichloromethane (3x 20 ml), dried ($Na_2SO_4$), and the solvents removed *in vacuo.* The residue was purified by flash chromatography over silica eluting with ethyl acetate : heptane mixtures 0 : 100 to 25 : 75 to give (*R*)-2-(2-hydroxyethyl)-2,5-dihydropyrrole-1-carboxylic acid *tert*-butyl ester **(74)** as a colourless oil (0.48 g, 67%), $[a]_D^{22}$ -127° (c=1, $CHCl_3$).

(74)

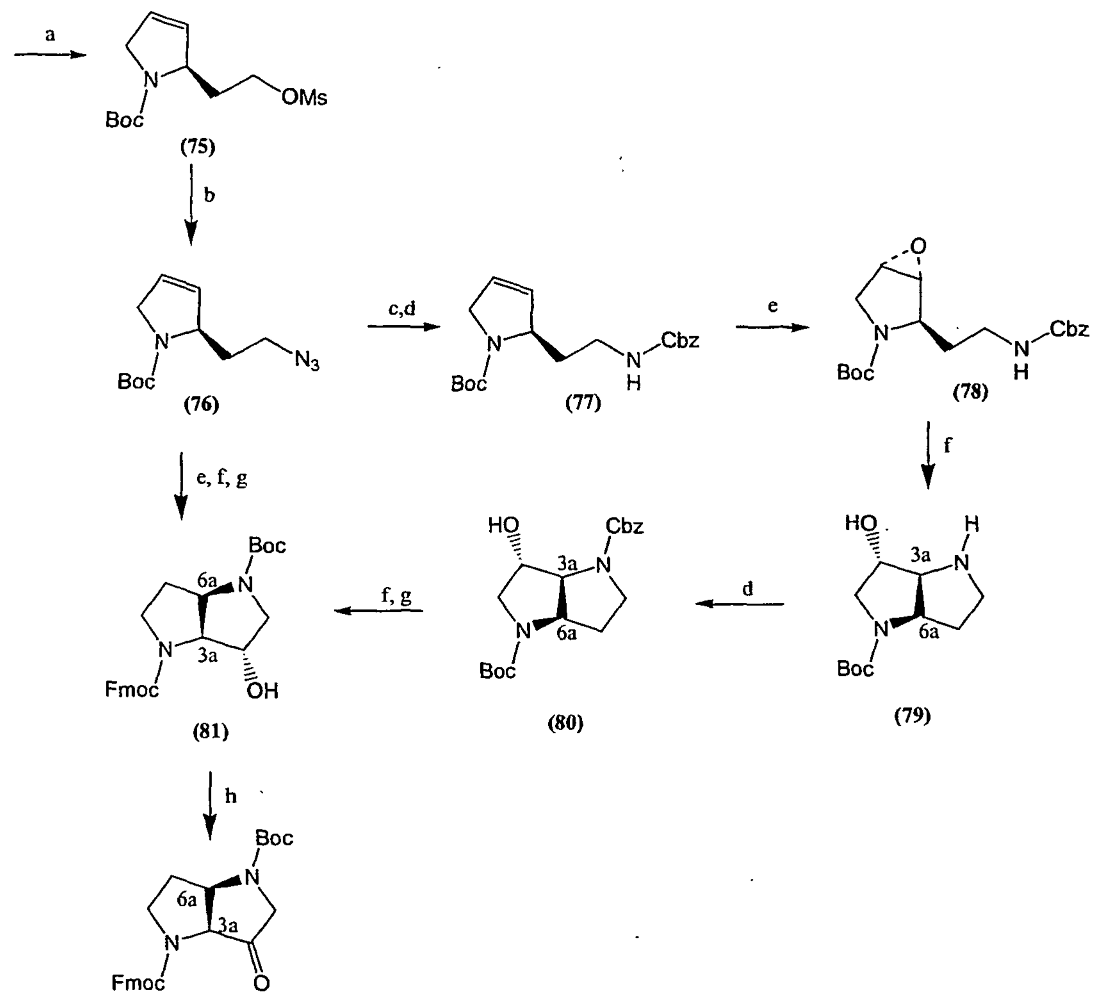


(75) (76) (77) (78) (79) (80) (81) (6)

**Scheme 20.** (a) Methanesulfonyl chloride, triethylamine, DCM. (b) Sodium azide, DMF. (c) $Ph_3P$ / $H_2O$, 1,4-dioxane. (d) 1.05 eq Cbz-Cl, 2.1eq $Na_2CO_3$, 1,4-dioxane, water. (e) *m*-Chloroperoxybenzoic acid, DCM. (f) Pd-C / $H_2$, ethanol. (g) 1.05 eq Fmoc-Cl, 2.1eq $Na_2CO_3$, 1,4-dioxane, water. (h) Dess-Martin periodinane, DCM.

**Preparation of (*R*)-2-(2-methanesulfonylethyl)-2,5-dihydropyrrole-1-carboxylic acid *tert*-butyl ester (75)**

Triethylamine (2.35 ml, 16.9 mmol) was added dropwise to a stirred solution of (*R*)-2-(2-hydroxyethyl)-2,5-dihydropyrrole-1-carboxylic acid *tert*-butyl ester **(74)** (2.33 g, 10.9 mmol) in dichloromethane (20 ml) at 0 °C over 2 minutes followed by methanesulfonyl chloride (1.27 ml, 16.4 mmol) over 4 minutes. The mixture was stirred for 1 hour at 0 °C then washed with water (170 ml) and brine (170 ml), dried ($Na_2SO_4$) and the solvents removed *in vacuo* to leave a residue (3.42 g), which was used without further purification (see below). HPLC-MS 236.0 $[M + 2H-Bu]^+$, 314.1 $[M + Na]^+$, 605.1 $[2M + Na]^+$.

**Preparation of (*R*)-2-(2-azidoethyl)-2,5-dihydropyrrole-1-carboxylic acid *tert*-butyl ester (76)**

Sodium azide (3.55 g, 54.7 mmol) was added to a stirred solution of (R)-2-(2-methanesulfonylethyl)-2,5-dihydropyrrole-1-carboxylic acid *tert*-butyl ester **(75)** (prepared as above) in dimethylformamide (45 ml) under an atmosphere of argon. The mixture was stirred at 60 °C for 1.5 hours then the majority of solvents were removed by distillation *in vacuo* and the residue partitioned between water (200 ml) and ethyl acetate (200 ml). The ethyl acetate layer was washed with brine (150 ml), dried ($Na_2SO_4$), and the solvents removed *in vacuo* to leave a residue (2.49 g) which was purified by flash chromatography over silica eluting with ethyl acetate : heptane mixtures 0: 100 to 10 : 90 to give (*R*)-2-(2-azidoethyl)-2,5-dihydropyrrole-1-carboxylic acid *tert*-butyl ester **(76)** as a colourless oil (2.05 g, 79%). TLC (Single spot, $R_f$ = 0.45, EtOAc : hexane 3 : 7), analytical HPLC $R_t$ = 15.910 min; HPLC-MS 139.1 $[M + 2H - Boc]^+$, 183.1 $[M + 2H - Bu]^+$, 499.2 $[2M + Na]^+$; $d_H$ (500 MHz, $CDCl_3$) 1.40-1.50 (9H, m, $C(CH_3)_3$), 1.90-2.10 (2H, m, $NCHCH_2$), 3.17-3.33 (2H, m, $CH_2N_3$), 3.96-4.27 (2H, m, $BocNCH_2$), 4.53-4.68 (1H, m, BocNC*H*), 5.66-5.86 (2H, m, 2x olefinic C*H*); $d_C$ (125 MHz, $CDCl_3$) 28.3 and 28.5 ($C(\underline{C}H_3)_3$), 32.5 and 33.0 ($\underline{C}H_2CH_2N_3$), 47.5 and 47.9 ($\underline{C}H_2N_3$), 53.6 and 53.8 ($BocN\underline{C}H_2$), 62.0 and 62.3 ($BocN\underline{C}H$), 79.55 and 79.9 ($O\underline{C}(CH_3)_3$), 125.6 and 126.1 (olefinic $\underline{C}H$), 128.9 and 129.4 (olefinic $\underline{C}H$), 154.2 and 154.3 ($N\underline{C}O_2$), followed by (*R*)-2-(2-hydroxyethyl)-2,5-dihydropyrrole-1-carboxylic acid *tert*-butyl ester **(74)** (22 mg, 9%).

### Preparation of (*R*)-2-(2-benzyloxycarbonylaminoethyl)-2,5-dihydropyrrole-1-carboxylic acid *tert*-butyl ester (77)

Water (2.1 ml, 118 mmol) was added to a stirred solution of (*R*)-2-(2-azidoethyl)-2,5-dihydropyrrole-1-carboxylic acid *tert*-butyl ester **(76)** (2.8 g, 11.8 mmol) and triphenylphosphine (4.6 g, 17.5 mmol) in tetrahydrofuran (350 ml) under an atmosphere of argon. The solution was heated at 45 °C for 7.5 hours then at ambient temperature for 14 hours. An aliquot (18.5 ml, ~0.63 mmol) was removed, concentrated *in vacuo* then azeotroped with toluene (3x 10 ml) and used for the preparation of (2*R*)-2-[2-((2*S*)-2-benzyloxycarbonylamino-4-methylpentanoylamino) ethyl]-2,5-dihydropyrrole-1-carboxylic acid *tert*-butyl ester **(140)** (see Scheme 28). An additional 5.0 ml aliquot was removed for analysis, then the remainder of the solution was concentrated *in vacuo* to obtain an oily residue. The residue was dissolved in 1,4-dioxane (35 ml) with stirring, ice-cooled and a solution of sodium carbonate (2.45 g, 23.1 mmol) in water (35 ml) was added. Benzyl chloroformate (2.18 g, 1.824 ml, 12.8 mmol) in 1,4-dioxane (10 ml) was then added dropwise over 30 minutes and the mixture stirred for an additional 30 minutes before adding water (250 ml). The aqueous phase was extracted with dichloromethane (2x 250 ml) and the combined organic layers were dried ($Na_2SO_4$), filtered and reduced *in vacuo* to leave a clear mobile oil (10.2 g). Flash chromatography over silica, eluting with ethyl acetate : heptane mixtures gave (*R*)-2-(2-benzyloxycarbonylaminoethyl)-2,5-dihydropyrrole-1-carboxylic acid *tert*-butyl ester **(77)** (3.58 g, 94%) as a mobile colourless oil. TLC ($R_f$= 0.32, EtOAc : heptane 1 : 1), analytical HPLC single main peak, $R_t$ = 17.39 min., HPLC-MS 247.1 $[M + 2H - Boc]^+$, 291.1 $[M + 2H - Bu]^+$, 347.1 $[M + H]^+$, 369.1 $[M + Na]^+$, 715.2 $[2M + Na]^+$; Elemental analysis $C_{19}H_{26}N_2O_4$ req.(fnd.) % C 65.87 (65.79), % H 7.56 (7.53), % N 8.09 (7.97); HRMS $C_{19}H_{26}N_2O_4Na$ req. 369.1790, fnd. 369.1803 (3.37ppm); $\delta_H$ (500 MHz, $CDCl_3$) 1.45 (9H, br. s, $C(CH_3)_3$), 1.60-1.95 (2H, m, $BocNCHCH_2$), 3.00-3.44 (2H, m, $CH_2NH$), 3.90-4.29 (2H, m, $BocNCH_2$), 4.45-4.81 (1H, m, BocNC*H*), 5.01-5.16 (2H, m, $OCH_2Ph$), 5.50-5.83 (2H, m, 2x olefinic C*H*) and 7.25-7.38 (6H, m, $C_6H_5$ and N*H*); $\delta_C$ (125 MHz, $CDCl_3$) 28.4 ($C(\underline{C}H_3)_3$), 34.4, 34.6 ($\underline{C}H_2CH_2NH$), 37.2, 37.6 ($\underline{C}H_2NH$), 53.6, 53.7 ($BocN\underline{C}H_2$), 61.7, 62.1 ($BocN\underline{C}H$), 66.4, 66.6 ($O\underline{C}H_2Ph$), 79.6, 79.9 ($O\underline{C}(CH_3)_3$), 125.2, 125.9, 127.0, 127.6, 127.9, 128.0, 128.4, 129.5, 130.2 (5x aromatic $\underline{C}H$ and 2x olefinic $\underline{C}H$), 154.3, 155.0, 156.2, 156.5 ($NH\underline{C}O_2$ and $N\underline{C}O_2$).

### Preparation of (2*R*)-2-(2-benzyloxycarbonylaminoethyl)-6-oxa-3-aza-bicyclo [3.1.0]hexane-3-carboxylic acid *tert*-butyl ester (78)

(*R*)-2-(2-Benzyloxycarbonylaminoethyl)-2,5-dihydropyrrole-1-carboxylic acid *tert*-butyl ester **(77)** (3.57 g, 10.3 mmol) was dissolved in anhydrous dichloromethane (60 ml) with stirring and *meta*-chloroperoxybenzoic acid (27.3 g, 65% reagent, 103 mmol) added. The mixture was stirred at ambient temperature under argon for 16 hours. Dichloromethane (400 ml) was added and the organic phase washed with 10% aqueous w/v solution of sodium hydroxide (2x 400 ml), then dried ($Na_2SO_4$), filtered and reduced *in vacuo* to leave a clear oil (~5 g). Flash chromatography over silica, eluting with ethyl acetate : heptane mixtures gave (2*R*)-2-(2-benzyloxycarbonyl aminoethyl)-6-oxa-3-aza-bicyclo [3.1.0]hexane-3-carboxylic acid *tert*-butyl ester **(78)** (3.57 g, 95.3%) as a mobile colourless oil. TLC ($R_f$ = 0.36 (minor) and 0.40 (major) (mixture of *anti* and *syn* epoxides), EtOAc : heptane 2 : 1), analytical HPLC single main peak, $R_t$ = 17.74 min., HPLC-MS 263.1 $[M + 2H - Boc]^+$, 307.1 $[M + 2H - Bu]^+$, 363.1 $[M + H]^+$, 385.1 $[M + Na]^+$, 747.2 $[2M + Na]^+$; Elemental analysis $C_{19}H_{26}N_2O_5$ req.(fnd.) % C 62.97 (62.93), % H 7.23 (7.22), % N 7.73 (7.61); HRMS $C_{19}H_{26}N_2O_5Na$ req. 385.1739, fnd. 385.1725 (~3.82ppm); $d_H$ (500 MHz, $CDCl_3$) 1.32-1.62 (10H, m, $C(CH_3)_3$ and $CH_2CH_2NH$), 1.67-2.00 (1H, m, $CH_2CH_2NH$), 2.90-4.21 (7H, m, $CH_2NH$, BocNC*H*C*H*, $BocNCH_2CH$), 4.70-5.17 (2H, m, $OCH_2Ph$), 5.78-6.05 (1H, m, N*H*) and 7.27-7.37 (5H, aromatics); $d_C$ (125 MHz, $CDCl_3$) 28.1, 28.3, 28.35 and 28.4 ($C(\underline{C}H_3)_3$), 30.8 and 31.2 ($\underline{C}H_2CH_2NH$), 37.4 and 37.7 ($\underline{C}H_2NH$), 46.15 and 46.6 ($BocN\underline{C}H_2$), 53.9, 54.2, 54.9 and 55.8 (2x epoxide $\underline{C}H$), 58.1 and 58.2 ($BocN\underline{C}H$), 66.5 and 66.7 ($O\underline{C}H_2Ph$), 80.3 and 80.7 ($O\underline{C}(CH_3)_3$), 128.0, 128.1, 128.2, 128.4, 128.5 (5x aromatic $\underline{C}H$), 136.7 ($OCH_2\underline{C}$), 155.1, 155.9, 156.3 and 156.6 ($NH\underline{C}O_2$ and $N\underline{C}O_2$).

### Preparation of (3*S*, 3a*S*, 6a*R*)-3-hydroxyhexahydropyrrolo[3,2-*b*]pyrrole-1-carboxylic acid *tert*-butyl ester (79)

(2*R*)-2-(2-Benzyloxycarbonylaminoethyl)-6-oxa-3-aza-bicyclo[3.1.0]hexane-3-carboxylic acid *tert*-butyl ester

**(78)** (3.57 g, 9.86 mmol) was dissolved in ethanol (60 ml), cooled to 0 °C and 10% palladium on charcoal (0.40 g) added. The mixture was stirred, then evacuated and flushed with hydrogen. The mixture was allowed to warm to ambient temperature and after 2.5 hours filtered through celite. The filter cake was washed with ethanol (3x 60 ml) and the combined organic filtrates reduced *in vacuo* to provide crude (3*S*, 3a*S*, 6a*R*)-3-hydroxyhexahydropyrrolo[3,2-*b*]pyrrole-1-carboxylic acid *tert*-butyl ester **(79)** (~ 2.4 g). HPLC-MS 173.1 $[M + 2H - Bu]^+$, 229.1 $[M + H]^+$.

### Preparation of (3*S*, 3a*S*, 6a*R*)-3-hydroxyhexahydropyrrolo[3,2-*b*]pyrrole-1,4-dicarboxylic acid 4-benzyl ester 1-*tert*-butyl ester (80)

Crude (3*S*, 3a*S*, 6a*R*)-3-hydroxyhexahydropyrrolo[3,2-*b*]pyrrole-1-carboxylic acid *tert*-butyl ester **(79)** (~2.4 g) was dissolved in 1,4-dioxane (30 ml) with stirring, ice-cooled and a solution of sodium carbonate (2.19 g, 20.7 mmol) in water (25 ml) was added. Benzyl chloroformate (1.63m, 11.4 mmol) in 1,4-dioxane (15 ml) was then added dropwise over 30 minutes and the mixture stirred for a further 30 minutes. The mixture was then reduced *in vacuo* by approximately 2/3 volume to leave a mobile pulp. Water (200 ml) was added and the aqueous phase extracted with dichloromethane (2x 100 ml). The combined organic layers were dried ($Na_2SO_4$), filtered and reduced *in vacuo* to leave a clear mobile oil (3.96 g). Flash chromatography over silica, eluting with ethyl acetate : heptane mixtures gave (3*S*, 3a*S*, 6a*R*)-3-hydroxyhexahydropyrrolo[3,2-*b*]pyrrole-1,4-dicarboxylic acid 4-benzyl ester 1-*tert*-butyl ester **(80)** (2.16 g, 60.5%) as an opaque gum. TLC ($R_f$ = 0.15, EtOAc : heptane 1 : 1), analytical HPLC single main peak, $R_t$ = 17.15 min., HPLC-MS 263.1 $[M + 2H - Boc]^+$, 307.1 $[M + 2H - Bu]^+$, 363.1 $[M + H]^+$, 385.1 $[M + Na]^+$, 747.2 $[2M + Na]^+$; Elemental analysis $C_{19}H_{26}N_2O_5$ req.(fnd.) % C 62.97 (62.82), % H 7.23 (7.39), % N 7.73 (7.57); HRMS $C_{19}H_{26}N_2O_5Na$ req. 385.1739, fnd. 385.1725 (2.15ppm); $\delta_H$ (400 MHz, $CD_3CN$, T = 75 °C) 1.46 (9H, s, $C(CH_3)_3$), 1.90-2.00 (1H, m obscured by NMR solvent peaks, BocNCHC*H*$_2$), 2.14 (1H, dd, *J* = 6.15, 13.15 Hz, BocNCHC*H*$_2$), 3.07-3.20 (2H, m, O*H* + CbzNC*H*$_2$), 3.24 (1H, dd, *J* = 3.8, 12.2 Hz, BocNC*H*$_2$), 3.51 (1H, d, *J* = 12.2 Hz, BocNC*H*$_2$), 3.68 (1H, ddd, *J* = 1.7, 8.6, 10.9 Hz, CbzNC*H*$_2$), 4.10 (1H, br. d, *J* = 5.8 Hz, CbzNC*H*), 4.27 (1H, br. s, CHOH), 4.40-4.46 (1H, m, BocNC*H*), 5.12 (1H, d, *J* = 12.7 Hz, OC*H*$_2$Ph), 5.16 (1H, d, *J* = 12.7 Hz, OC*H*$_2$Ph) and 7.42-7.29 (5H, aromatics); $\delta_C$ (120 MHz, $CDCl_3$) 28.5 ($C(CH_3)_3$), 29.7, 30.4, 31.2, 31.9, 32.0 (BocNCHC$H_2$), 45.5, 45.7 (CbzNC$H_2$), 53.1, 53.4, 53.5 (BocNC$H_2$), 60.1, 61.2 (BocNCH), 67.2, 67.6, 68.2, 68.4, 69.0 (OC$H_2$Ph + Cbz-NCH), 72.7, 73.3, 73.4 (CHOH), 79.9, 80.1 ($OC(CH_3)_3$), 127.9, 128.0, 128.2, 128.3, 128.5, 128.6, 136.3, 136.4 (aromatics), 154.1, 154.2, 155.2 (2x $NCO_2$).

### Preparation of (3*S*, 3a*S*, 6a*R*)-3-hydroxyhexahydropyrrolo[3,2-*b*]pyrrole-1,4-dicarboxylic acid 1-*tert*-butyl ester 4-(9H-fluoren-9-ylmethyl) ester (81)

(3*S*, 3a*S*, 6a*R*)-3-Hydroxyhexahydropyrrolo[3,2-*b*]pyrrole-1,4-dicarboxylic acid 4-benzyl ester 1-*tert*-butyl ester **(80)** (0.54 g, 1.5 mmol) was dissolved in ethanol (10 ml), cooled to 0 °C and 10% palladium on charcoal (0.055 g) added. The mixture was stirred, then evacuated and flushed with hydrogen. The mixture was warmed to ambient temperature and after 2.5 hours filtered through celite. The filter cake was washed with ethanol (3x 10 ml) and the combined filtrates reduced *in vacuo* to provide the crude amine (~ 0.36 g). HPLC-MS 173.1 $[M + 2H - Bu]^+$, 229.1 $[M + H]^+$. The crude amine was dissolved in 1,4-dioxane (15 ml) with stirring, ice-cooled and a solution of sodium carbonate (0.33 g, 3.15 mmol) in water (15 ml) was added. 9-Fluorenylmethyl chloroformate (0.463 g, 1.79 mmol) in 1,4-dioxane (10 ml) was added dropwise over 30 minutes and the mixture stirred for a further 30 minutes. Water (200 ml) was then added and the aqueous phase extracted with ethyl acetate (2x 100 ml). The combined organic layers were dried ($Na_2SO_4$), filtered and reduced *in vacuo* to leave a clear mobile oil (1.02 g). Flash chromatography over silica, eluting with ethyl acetate : heptane mixtures gave (3*S*, 3a*S*, 6a*R*)-3-hydroxy hexahydropyrrolo[3,2-*b*]pyrrole-1,4-dicarboxylic acid 1-*tert*-butyl ester 4-(9H-fluoren-9-ylmethyl) ester **(81)** (0.64 g, 95%) as a fine white crystalline solid. TLC ($R_f$ = 0.33, EtOAc : heptane 2 : 1), analytical HPLC single main peak, $R_t$ = 19.98 min., HPLC-MS 395.1 $[M + 2H - Bu]^+$, 451.1 $[M + H]^+$, 473.1 $[M + Na]^+$, 923.2 $[2M + Na]^+$; Elemental analysis $C_{26}H_{30}N_2O_5$ req.(fnd.) % C 69.31 (69.11), % H 6.71 (7.06), % N 6.22 (5.84); HRMS $C_{26}H_{30}N_2O_5Na$ req. 473.2052, fnd. 473.2053 (0.06ppm); $\delta_H$ (400 MHz, $CD_3CN$, T = 75 °C) 1.46 (9H, s, $C(CH_3)_3$), 1.75-1.90 (1H, m, BocNCHC*H*$_2$), 2.05-2.13 (1H, m, BocNCHC*H*$_2$), 3.02 (1H, m, FmocNC*H*$_2$), 3.08-3.20 (1H, m, BocNC*H*$_2$), 3.46 (1H, m, BocNC*H*$_2$), 3.46-3.60 (1H, m, FmocNC*H*$_2$), 3.90-4.15 (2H, m, FmocNC*H* and C*H*OH), 4.28 (1H, t, *J* = 6.1 Hz, FmocC*H*), 4.34-4.40 (1H, m, BocNC*H*), 4.49 (2H, d, *J* = 6.1 Hz, FmocC*H*$_2$), 7.31-7.45 (4H, m, Fmoc aromatics), 7.65 (2H, d, *J* = 7.3 Hz, Fmoc aromatics), 7.83 (2H, d, *J* = 7.5 Hz, Fmoc aromatics); $\delta_C$ (100 MHz, $CDCl_3$) 28.45 ($C(CH_3)_3$), 30.2, 31.2, 32.0 (BocNCHC$H_2$), 44.8, 45.3, 45.6 (FmocNC$H_2$), 47.3, 47.4 (FmocCH), 52.8, 53.1, 53.4, 53.5 (BocNC$H_2$), 60.1, 60.8 (BocNCH), 65.9, 66.2, 67.3 (FmocC$H_2$), 67.85, 68.4, 68.9 (FmocNCH), 72.5, 72.9, 73.3, 73.6 (CHOH), 79.95 ($OC(CH_3)_3$), 119.8, 120.0, 124.6, 124.9, 125.0, 127.0, 127.4, 127.8 (Fmoc CH aromatics), 141.3, 141.5, 143.7, 143.8, 144.1 (Fmoc quaternary aromatics), 154.0, 154.3, 155.0, 155.2 (2x $NCO_2$).

**Alternative preparation of (3*S*, 3a*S*, 6a*R*)-3-hydroxyhexahydropyrrolo[3,2-*b*]pyrrole-1,4-dicarboxylic acid 1-*tert*-butyl ester 4-(9H-fluoren-9-ylmethyl) ester (81)**

*meta*-Chloroperoxybenzoic acid (864 mg, 57-86%) was added to a solution of (*R*)-2-(2-azidoethyl)-2,5-dihydropyrrole-1-carboxylic acid *tert*-butyl ester **(76)** (175 mg, 0.735 mmol) in anhydrous dichloromethane (4 ml). The mixture was stirred at ambient temperature for 7 hours then saturated aqueous sodium hydrogen carbonate solution (40 ml) and dichloromethane (60 ml) were added. The phases were mixed and separated and the organic phase washed with 10% aqueous sodium hydroxide solution (40 ml), dried ($Na_2SO_4$) and evaporated *in vacuo* to afford a residue (185 mg). The residue was dissolved in ethanol (6.8 ml) and cooled to 0 °C. 10% Palladium on carbon (84 mg) was added to the mixture and the atmosphere purged with hydrogen gas. The mixture was stirred overnight under a hydrogen atmosphere at ambient temperature, filtered over celite and the filter cake washed with excess ethyl acetate. The filtrate was concentrated *in vacuo*, and the residue suspended in a solution of sodium carbonate (193 mg, 1.82 mmol) in water (4 ml). 1,4-Dioxane (2 ml) was added and the mixture cooled to 0 °C, then a solution of 9-fluorenylmethyl chloroformate (198 mg, 0.77 mmol) in 1,4-dioxane (2 ml) added in small portions over 40 minutes. The mixture was then allowed to warm to ambient temperature over 40 minutes. Water (40 ml) was added and the product extracted into dichloromethane (3x 40 ml). The combined organic layers were dried ($Na_2SO_4$) and evaporated *in vacuo* to afford a residue (335 mg). Flash chromatography of the residue over silica gel (35 g) eluting with ethyl acetate : heptane mixtures 1 : 4 followed by 1 : 1 gave (3*S*, 3a*S*, 6a*R*)-3-hydroxyhexahydropyrrolo[3,2-*b*]pyrrole-1,4-dicarboxylic acid 1-*tert*-butyl ester 4-(9H-fluoren-9-ylmethyl) **(81)** (90 mg, 27%). TLC (Single spot, $R_f$ = 0.24, EtOAc : heptane 1 : 1), analytical HPLC $R_t$ = 16.348 min; HPLC-MS 451.2 $[M + H]^+$, 473.2 $[M + Na]^+$, 923.4 $[2M + Na]^+$; $d_H$ (500 MHz, $CDCl_3$) 1.36-1.49 (9H, s, $C(CH_3)_3$), 1.65-2.25 (3H, m, BocNCHC$H_2$ and O*H*), 2.85-3.68 (5H, m, FmocNC$H_2$, BocNC$H_2$ and PmocNC*H*), 4.05-4.80 (5H, m, $OCH_2CH$, $OCH_2$, C*H*OH and BoeNC*H*), 7.26-7.45 (4H, m, Fmoc aromatic C*H*), 7.53-7.64 (2H, m, Fmoc aromatic C*H*) and 7.73-7.80 (2H, m, Fmoc aromatic C*H*);

**Preparation of (3a*S*, 6a*R*)-3-oxo-hexahydropyrrolo[3,2-*b*]pyrrole-1,4-dicarboxylic acid 1-*tert*-butyl ester 4-(9H-fluoren-9-ylmethyl) ester (6)**

(3*S*, 3a*S*, 6a*R*)-3-Hydroxyhexahydropyrrolo[3,2-*b*]pyrrole-1,4-dicarboxylic acid 1-*tert*-butyl ester 4-(9H-fluoren-9-ylmethyl) ester **(81)** (0.495 g, 1.10 mmol) was dissolved in anhydrous dichloromethane (18 ml) with stirring under argon. Dess-Martin periodinane (0.962 g, 2.27 mmol) was added and the mixture stirred for 4 hours. The mixture was concentrated *in vacuo* and the residue purified by flash chromatography over silica, eluting with ethyl acetate : heptane mixtures to give (3a*S*, 6a*R*)-3-oxo-hexahydropyrrolo[3,2-*b*]pyrrole-1,4-dicarboxylic acid 1-*tert*-butyl ester 4-(9H-fluoren-9-ylmethyl) ester (6) (0.480 g, 97%) as a white crystalline solid. TLC ($R_f$ = 0.38, EtOAc : heptane 1 : 1), analytical HPLC single broad main peak, $R_t$ = 20.27-21.79 min., HPLC-MS 393.1 $[M + 2H - Bu]^+$, 449.1 $[M + H]^+$, 471.1 $[M + Na]^+$, 919.2 $[2M + Na]^+$; Elemental analysis $C_{26}H_{28}N_2O_5$.0.25EtOAc req.(fnd.) % C 68.96 (68.88), % H 6.43 (6.61), % N 5.95 (5.95); HRMS $C_{26}H_{28}N_2O_5Na$ req. 471.1896, fnd. 471.1903 (1.44ppm); $\delta_C$ (125 MHz, $CDCl_3$) 28.36 ($C(CH_3)_3$), 30.50, 30.93, 31.20 (BocNCH$\underline{C}H_2$), 45.68 (FmocN$\underline{C}H_2$), 47.20 (Fmoc$\underline{C}$H), 51.71, 52.22 (BocN$\underline{C}H_2$), 57.58, 58.64 (BocN$\underline{C}$H), 63.03, 63.57 (FmocN$\underline{C}$H), 67.70, 68.06 (Fmoc$\underline{C}H_2$), 81.10 (O$\underline{C}(CH_3)_3$), 119.94, 124.99, 125.15, 125.29, 127.05, 127.55, 127.71, 127.85 (Fmoc $\underline{C}$H aromatics), 143.69, 143.92, 144.23 (Fmoc quaternary aromatics), 153.99, 154.74, 155.04 (2x N$\underline{C}O_2$), 206.33, 206.59 ($\underline{C}$=O).

Following the general details from Scheme 6, the required bicycle building block (3a*S*,6a*R*) 3-Oxo-hexahydro-pyrrolo[3,2-*b*]pyrrole-1,4-dicarboxylic acid 1-*tert*-butyl ester 4-(9H-fluoren-9-ylmethyl) ester **(6)** was converted to building block-linker construct **(27)** as follows:

A solution of sodium acetate trihydrate (42 mg, 0.311 mmol) in water (0.5 ml) was added to a solution of (3a*S*, 6a*R*) 3-oxo-hexahydropyrrolo[3,2-*b*]pyrrole-1,4-dicarboxylic acid 1-*tert*-butyl ester 4-(9*H*-fluoren-9-ylmethyl) ester (18.6 mg, 0.0415 mmol) and 4-[[(hydrazinocarbonyl)amino]methyl]cyclohexane carboxylic acid. trifluoroacetate (Murphy, A. M., et al, J. Am. Chem. Soc, 114, 3156-3157, 1992) (68 mg, 0.208 mmol) in ethanol (2.0 ml). Remaining traces of sodium acetate were rinsed into the mixture using a further aliquot of ethanol (1.5 ml) then the reaction heated at 75 °C in a sealed tube for 1 hour. The mixture was stood at ambient temperature for 14 hours then heated at 75°C for 2.5 hours. The product was extracted into chloroform (60 ml) then washed with hydrochloric acid (0.1M, 2 x 30 ml), saturated aqueous sodium chloride solution (30 ml) then dried ($Na_2SO_4$) and the solvent removed *in vacuo* to leave the product as a pale yellow oil (22.9 mg, 86%). Analytical HPLC has main UV peaks with Rt = 19.706 and 21.287mins and HPLC-MS (main UV peaks each with 646.3 $[M+H]^+$).

Following the general details from Scheme 6, the required building block-linker construct **(27)** was attached to the solid phase providing loaded building block-linker construct **(28)** as follows:

Building block-linker construct **(27)** (35.5μmoles mmoles), 2-(1H-benzotriazole-1-yl)-1,1,3,3-tetramethyluroniumhexafluoro phosphate (HBTU, 13.5mg, 35.5μmoles mmoles), 1-hydroxybenzotriazole.hydrate and (HOBT, 5.5mg, 35.5μmoles mmoles) were dissolved in dimethylformamide (1.5mL) and N-methylmorpholine (NMM, 7.8μL, 71μmoles mmoles) added. After pre-activation for 5 minutes, free amine gears (10 x 1.3μmole) were added and left overnight. The spent coupling solution was then added to free amine gears (6 x 1.3μmole) and left overnight. Standard washing and analyses indicated quantitative loading.

Following the general details from Scheme 6, the required loaded building block-linker construct **(28)** was elaborated on the solid phase as follows:

Loaded construct **(28)** was elaborated to EXAMPLE 1 (3*aR*, 6*aS*)-*N*-[(1*S*)-1-(4-Benzoyl-6-oxo-hexahydro-pyrrolo[3,2-*b*]pyrrole-1-carbonyl)-3-methyl-butyl]-4-*tert*-butylbenzamide by standard Fmoc deprotection and sequential rounds of coupling and washing with the appropriate reagents as follows:-

(i) Fmoc-Leu-OH (2 x 20eq, overnight and 4 hr), HBTU, HOBT, NMM activation in DMF
(ii) Standard Fmoc deprotection
(iii) 4-*tert*-butylbenzoic acid (1 x 10eq, overnight), HBTU, HOBT, NMM activation in DMF
(iv) Treatment with 35%TFA in dichloromethane for 30mins, followed by washing with 2 x DMF, 1 x 2%NMM in DMF, 4 x DMF, 4 x acetonitrile.
(v) Benzoic anhydride (20eq) and NMM (5eq) in DMF for 20hr.

The crude example was cleaved and analysed (see general techniques). HPLC Rt = 18.879-19.62mins (>90%), HPLC-MS 504.3 $[M + H]^+$, 1029.5 $[2M + Na]^+$.

The solid phase experimental detailed for EXAMPLE 1 may be followed to couple with a vast range of aminoacids, carboxylic acids, sulphonyl chlorides etc to provide a vast range of analogues of general formula I.

In certain combinations of groupings, the order of solid phase events is amended. For example, when preparing EXAMPLE 194, the U substituent contains an amine group that requires protection via the Boc group, thus the following order of events is utilised :-

(vi) Fmoc-Leu-OH (2 x 20eq, overnight and 4 hr), HBTU, HOBT, NMM activation in DMF
(vii) Treatment with 35%TFA in dichloromethane for 30mins, followed by washing with 2 x DMF, 1 x 2%NMM in DMF, 4 x DMF, 4 x acetonitrile.
(viii) Benzoic anhydride (20eq) and NMM (5eq) in DMF for 20hr.
(ix) Standard Fmoc deprotection
(x) 4-(4-Carboxyphenyl)-piperazine-1-carboxylic acid *tert*-butyl ester sodium salt (1 x 10eq, overnight), HBTU, HOBT, NMM activation in DMF.
(xi) Standard cleavage

As a further variation, when preparing EXAMPLE 151, the following order of events is utilised :-

(xii) Fmoc-Leu-OH (2 x 20eq, overnight and 4 hr), HBTU, HOBT, NMM activation in DMF
(xiii) Standard Fmoc deprotection
(xiv) 4-Dimethylaminobenzoic acid (1 x 10eq, overnight), HBTU, HOBT, NMM activation in DMF
(xv) Washing with 1 x DMF, 1 x 20% piperidine in DMF, 4 x DMF, 4 x acetonitrile.
(xvi) Treatment with 35%TFA in dichloromethane for 30mins, followed by washing with 2 x DMF, 1 x 2%NMM in DMF, 4 x DMF, 4 x acetonitrile.
(xvii) Fmoc-Leu-OH (20eq, overnight), HBTU, HOBT, NMM activation in DMF.
(xviii) Standard Fmoc deprotection
(xix) Acetic anhydride (50eq) and NMM (25eq) in DMF for 1hr.
(xx) Washing with 1 x DMF, 1 x 20% piperidine in DMF, 4 x DMF, 4 x acetonitrile.
(xxi) Standard cleavage

As a further variation, when preparing EXAMPLE 80, the following order of events is utilised :-

(xxii) Fmoc-Leu-OH (2 x 20eq, overnight and 4 hr), HBTU, HOBT, NMM activation in DMF
(xxiii) Treatment with 35%TFA in dichloromethane for 30mins, followed by washing with 2 x DMF, 1 x 2%NMM in DMF, 4 x DMF, 4 x acetonitrile.
(xxiv) Pyridine-2-carboxylic acid (20eq) and NMM (5eq) in DMF for 20hr.
(xxv) Washing with 2 x DMF, 1 x 2%NMM in DMF, 4 x DMF, 4 x acetonitrile.

(xxvi) Oxidation with m-chloroperbenzoic acid (5eq) in DCM for 8hrs.
(xxvii) Washing with 4 x DMF, 4 x acetonitrile.
(xxviii)Standard Fmoc deprotection
(xxix) 4-Dimethylaminobenzoic acid (1 x 10eq, overnight), HBTU, HOBT, NMM activation in DMF.
(xxx) Washing with 1 x DMF, I x 20% piperidine in DMF, 4 x DMF, 4 x acetonitrile.
(xxxi) Standard cleavage

The following examples **(2 - 248)** were prepared as detailed for EXAMPLE 1, coupling with the required reagents to provide the full length molecule (see i → v, or vi → xi, or xii → xxi above, or xxii → xxxi above). For step (v), (viii) and (xix) where the anhydride is not readily available, an $R^2COOH$ (20eq) / HBTU (20eq) / HOBT (20eq) / NMM (40eq) mixture in DMF with overnight coupling may be used or an RNHCOCl (20eq) / NMM (10eq) mixture in DMF with overnight coupling may be used or an RNCO (20eq) mixture in DMF with overnight coupling may be used. Following final coupling where the $R^2$ or U groups contain a protonatable nitrogen (e.g. pyridyl or 4-dimethylaminobenzoyl), the solid phase intermediate is treated with 20% piperidine in DMF for 10mins followed by standard washing protocols prior to cleavage.

EXAMPLE 2. (3*aR*, 6*aS*)-*N*-[(1*S*)-1-(4-Benzoyl-6-oxo-hexahydro-pyrrolo[3,2-*b*] pyrrole-1-carbonyl)-3-methyl-butyl]-4-thiophen-2-yl-benzamide

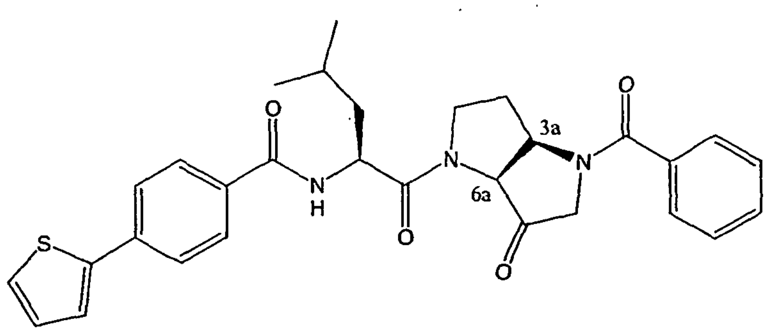

HPLC Rt = 18.0-19.2 mins (> 90%), HPLC-MS 530.2 $[M + H]^+$, 1081.4 $[2M + Na]^+$.

EXAMPLE 3. (3*aR*, 6*aS*)-4-*tert*-Butyl-*N*-[(1*S*)-3-methyl-1-(6-oxo-4-phenylacetyl-hexahydro-pyrrolo[3,2-*b*]pyrrole-1-carbonyl)-butyl]-benzamide

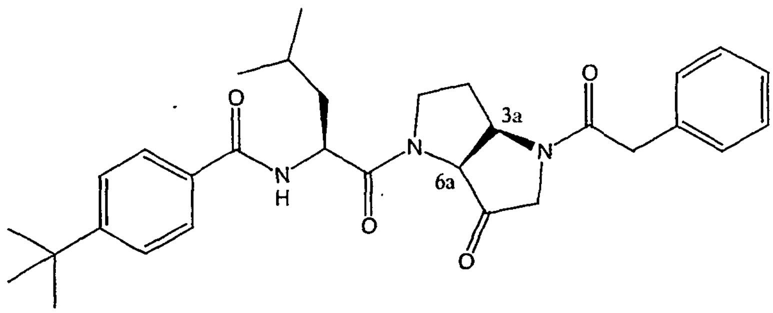

HPLC Rt = 19.1-20.2 mins (> 90%), HPLC-MS 518.3 $[M + H]^+$, 1057.6 $[2M + Na]^+$.

EXAMPLE 4. (3*aR*, 6*aS*)-4-*tert*-Butyl-*N*-{(1*S*)-3-methyl-1-[6-oxo-4-(pyridine-2-carbonyl)-hexahydro-pyrrolo[3,2-*b*]pyrrole-1-carbonyl]-butyl}-benzamide

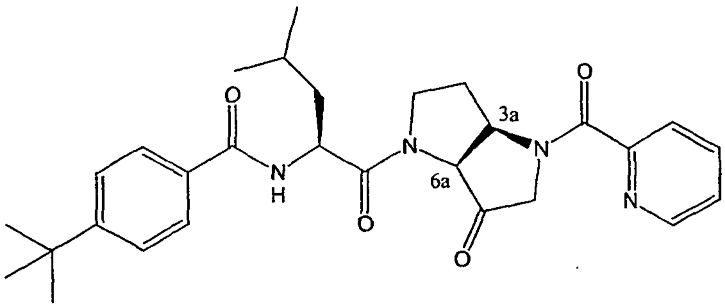

HPLC Rt = 7.2-18.1 mins (> 90%), HPLC-MS 505.3 $[M + H]^+$, 1031.5 $[2M + Na]^+$.

EXAMPLE 5. (3*aR*, 6*aS*)-4-*tert*-Butyl-*N*-{(1*S*)-3-methyl-1-[6-oxo-4-(pyridine-3-carbonyl)-hexahydro-pyrrolo[3,2-*b*]pyrrole-1-carbonyl]-butyl}-benzamide

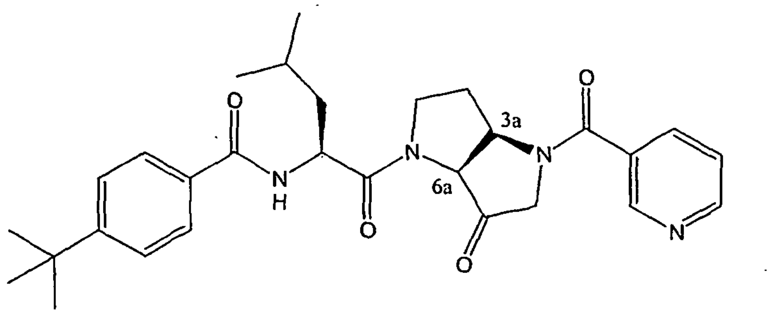

HPLC Rt = 15.2-16.4 mins (> 90%), HPLC-MS 505.3 $[M + H]^+$, 1031.5 $[2M + Na]^+$.

EXAMPLE 6. (3*aR*, 6*aS*)-4-*tert*-Butyl-*N*-{(1*S*)-3-methyl-1-[6-oxo-4-(pyridine-4-carbonyl)-hexahydro-pyrrolo[3,2-*b*]pyrrole-1-carbonyl]-butyl}-benzamide

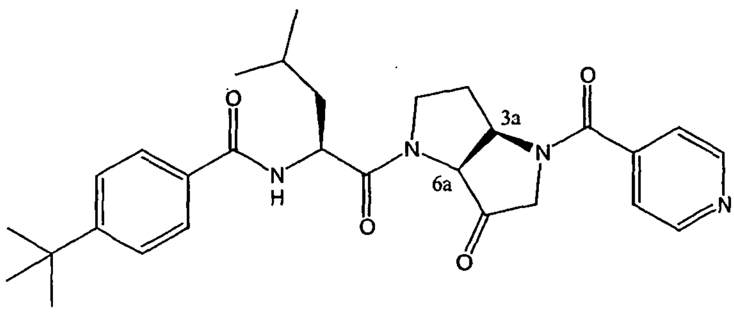

HPLC Rt = 15.3-16.4 mins (> 90%), HPLC-MS 505.3 $[M + H]^+$, 523.3 $[M + H + H_2O]^+$.

EXAMPLE 7. (3*aR*, 6*aS*)-Benzo[*b*]thiophene-2-carboxylic acid [(1*S*)-1-(4-benzoyl-6-oxo-hexahydro-pyrrolo[3,2-*b*]pyrrole-1-carbonyl)-3-methyl-butyl]-amide

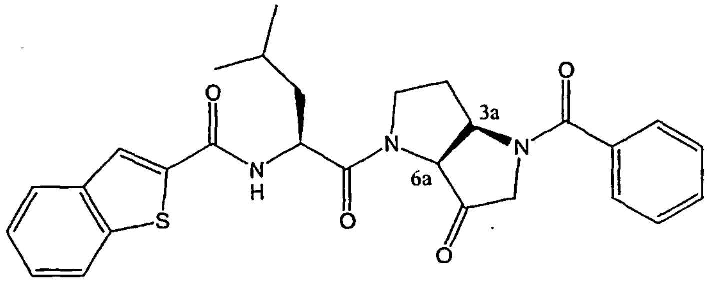

HPLC Rt = 16.8-17.9 mins (> 90%), HPLC-MS 504.2 [M + H]⁺.

EXAMPLE 8. (3a*R*, 6a*S*)-Quinoline-2-carboxylic acid [(1*S*)-1-(4-benzoyl-6-oxo-hexahydro-pyrrolo[3,2-*b*]pyrrole-1-carbonyl)-3-methyl-butyl]-amide

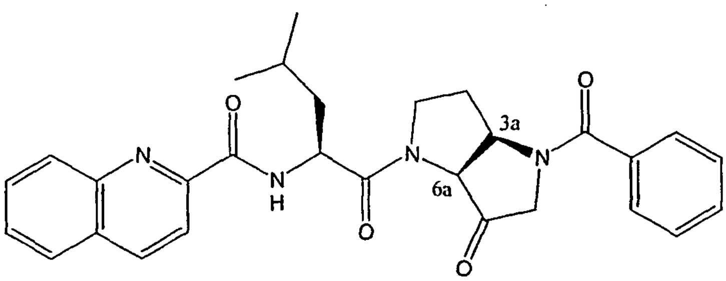

HPLC Rt = 17.0-17.8 mins (> 90%), HPLC-MS 499.1 [M + H]⁺.

EXAMPLE 9. (3a*R*, 6a*S*)-Benzofuran-2-carboxylic acid [(1*S*)-1-(4-benzoyl-6-oxo-hexahydropyrrolo[3,2-*b*]pyrrole-1-carbonyl)-3-methyl-butyl]-amide

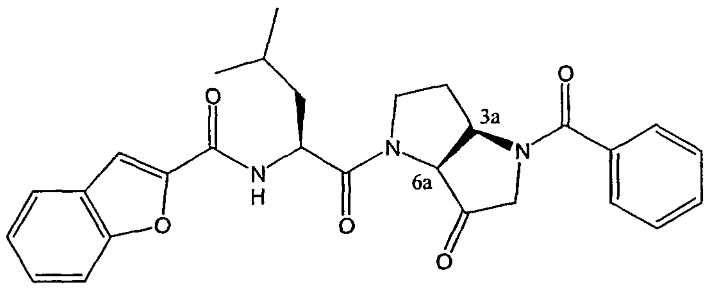

HPLC Rt = 16.2-17.7 mins (> 90%), HPLC-MS 488.1 [M + H]⁺, 997.2 [2M + Na]⁺.

EXAMPLE 10. (3a*R*, 6a*S*)-3-Methyl-benzofuran-2-carboxylic acid [(1*S*)-1-(4-benzoyl-6-oxo-hexahydro-pyrrolo[3,2-*b*]pyrrole-1-carbonyl)-3-methyl-butyl]-amide

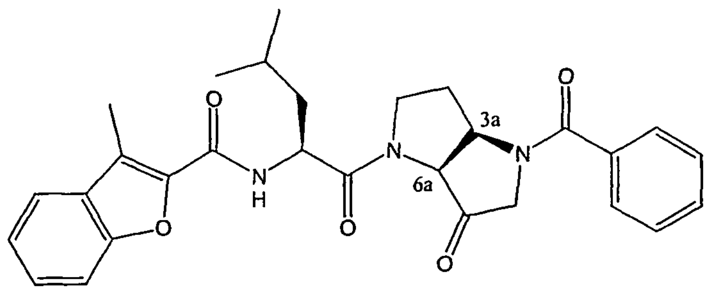

HPLC Rt = 17.8-18.9 mins (> 85%), HPLC-MS 502.1 $[M + H]^+$, 520.1 $[M + H + H_2O]^+$.

EXAMPLE 11. (3*aR*, 6*aS*)-*N*-[(1*S*)-1-(4-Benzoyl-6-oxo-hexahydro-pyrrolo[3,2-*b*] pyrrole-1-carbonyl)-butyl]-4-*tert*-butyl-benzamide

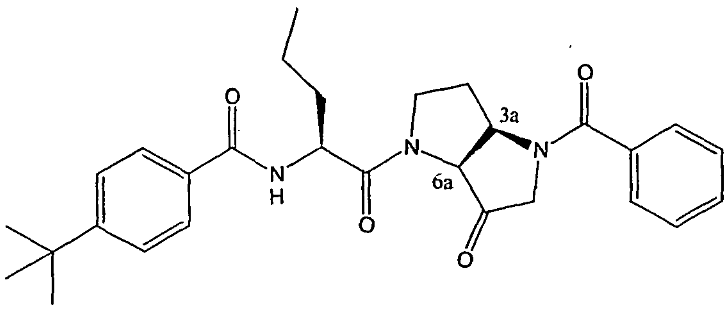

HPLC Rt = 17.4-18.2 mins (> 90%), HPLC-MS 490.2 $[M + H]^+$.

EXAMPLE 12. (3a*R*, 6a*S*)-Quinoxaline-2-carboxylic acid [(1*S*)-1-(4-benzoyl-6-oxo-hexahydro-pyrrolo[3,2-*b*]pyrrole-1-carbonyl)-3-methyl-butyl]-amide

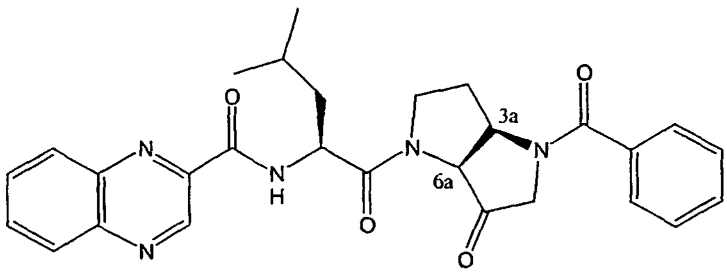

HPLC Rt = 15.6-16.7 mins (> 90%), HPLC-MS 500.2 $[M + H]^+$.

EXAMPLE 13. (3a*R*, 6a*S*)-*N*-[(1*S*)-1-(4-Benzoyl-6-oxo-hexahydro-pyrrolo[3,2-*b*] pyrrole-1-carbonyl)-3-methyl-butyl]-3-phenoxy-benzamide

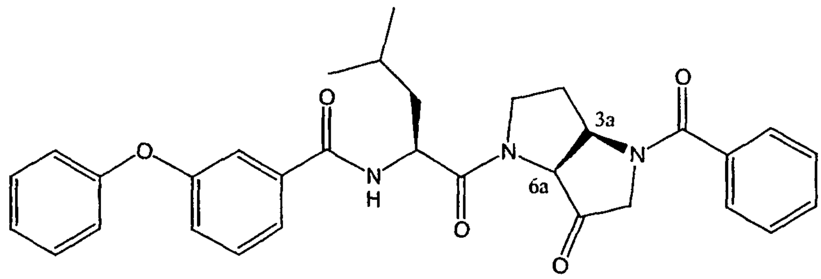

HPLC Rt = 18.5-19.7 mins (> 80%), HPLC-MS 540.1 $[M + H]^+$.

EXAMPLE 14. (3a*R*, 6a*S*)-*N*-[(1*S*)-1-(4-Benzoyl-6-oxo-hexahydro-pyrrolo[3,2-*b*] pyrrole-1-carbonyl)-3-methyl-butyl]-4-dimethylamino-benzamide

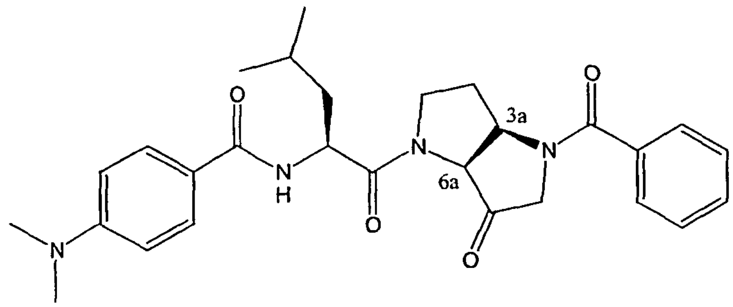

HPLC Rt = 12.16 mins (> 90%), HPLC-MS 491.2 $[M + H]^+$, 509.2 $[M + H + H_2O]^+$.

EXAMPLE 15. (3a*R*, 6a*S*)-Benzo[*b*]thiophene-2-carboxylic acid {(1*S*)-3-methyl-1-[6-oxo-4-(pyridine-3-carbonyl)-hexahydro-pyrrolo[3,2-*b*]pyrrole-1-carbonyl]-butyl}-amide

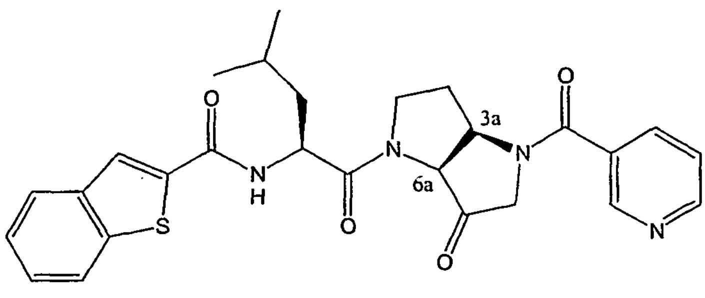

HPLC Rt = 13.93 mins (> 95%), HPLC-MS 505.2 $[M + H]^+$.

EXAMPLE 16. (3a*R*, 6a*S*)-Benzofuran-2-carboxylic acid {(1*S*)-3-methyl-1-[6-oxo-4-(pyridine-3-carbonyl)-hexahydro-pyrrolo[3,2-*b*]pyrrole-1-carbonyl]-butyl}-amide

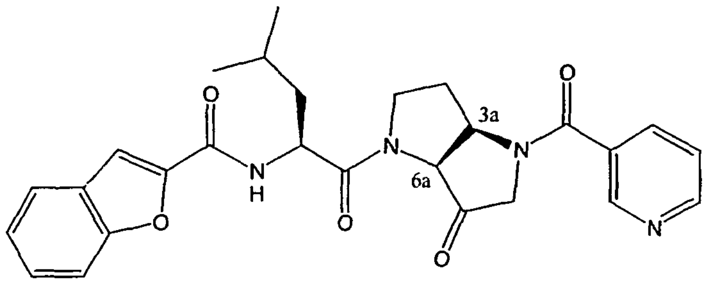

HPLC Rt = 13.42 mins (> 85%), HPLC-MS 489.2 [M + H]$^+$, 999.4 [2M + Na]$^+$.

EXAMPLE 17. (3a*R*, 6a*S*)-3-Methyl-benzofuran-2-carboxylic acid {(1*S*)-3-methyl-1-[6-oxo-4-(pyridine-3-carbonyl)-hexahydro-pyrrolo[3,2-*b*]pyrrole-1-carbonyl]-butyl}-amide

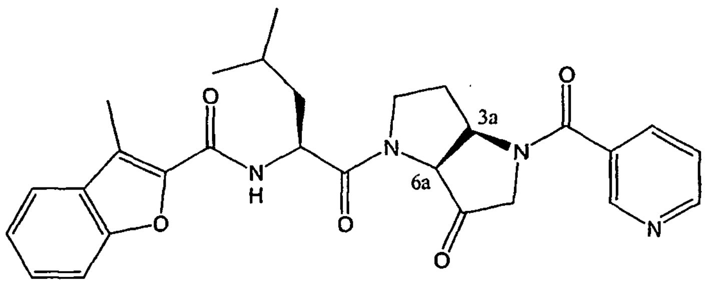

HPLC Rt = 14.58 mins (> 90%), HPLC-MS 503.2 [M + H]$^+$.

EXAMPLE 18. (3a*R*, 6a*S*)-Quinoline-2-carboxylic acid {(1*S*)-3-methyl-1-[6-oxo-4-(pyridine-3-carbonyl)-hexahydro-pyrrolo[3,2-*b*]pyrrole-1-carbonyl]-butyl}-amide

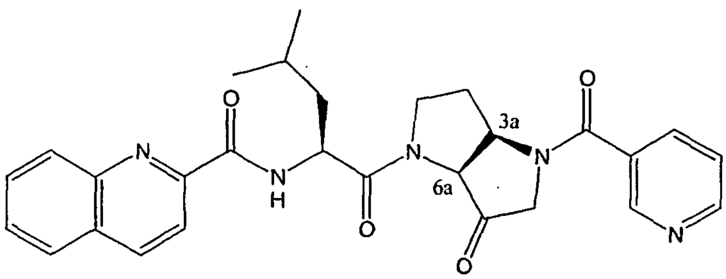

HPLC Rt = 14.00 mins (> 90%), HPLC-MS 500.2 [M + H]$^+$.

EXAMPLE 19. (3a*R*, 6a*S*)-Quinoxaline-2-carboxylic acid {(1*S*)-3-methyl-1-[6-oxo-4-(pyridine-3-carbonyl)-hexahydro-pyrrolo[3,2-*b*]pyrrole-1-carbonyl]-butyl}-amide

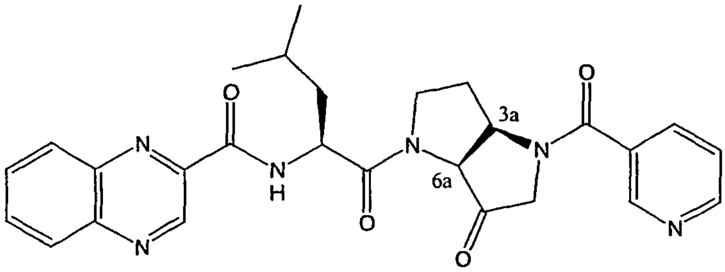

HPLC Rt = 12.77 mins (> 90%), HPLC-MS 501.2 $[M + H]^+$.

EXAMPLE 20. (3a*R*, 6a*S*)-*N*-{(1*S*)-3-Methyl-1-[6-oxo-4-(pyridine-3-carbonyl)-hexahydro-pyrrolo[3,2-*b*]pyrrole-1-carbonyl]-butyl}-3-phenoxy-benzamide

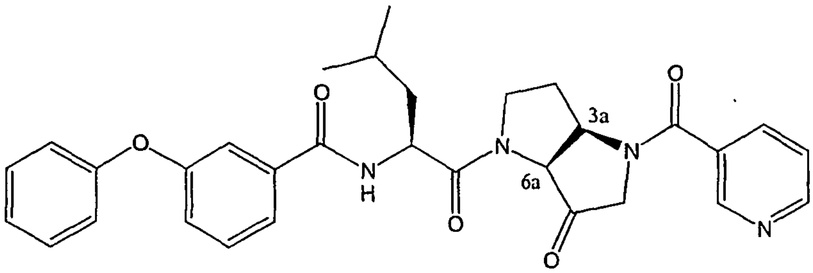

HPLC Rt = 16.06 mins (> 85%), HPLC-MS 541.2 $[M + H]^+$.

EXAMPLE 21. (3a*R*, 6a*S*)-4-Dimethylamino-*N*-{(1*S*)-3-methyl-1-[6-oxo-4-(pyridine-3-carbonyl)-hexahydro-pyrrolo[3,2-*b*]pyrrole-1-carbonyl]-butyl}-benzamide

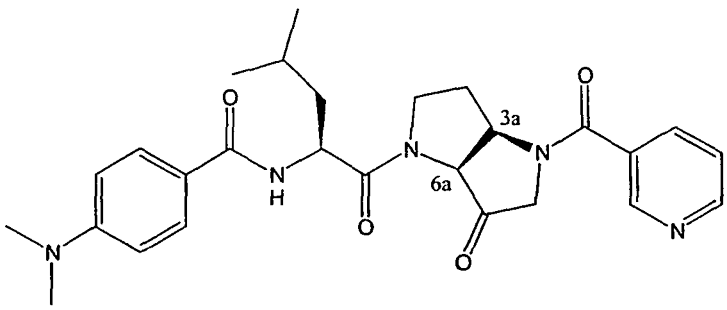

HPLC Rt = 8.70 mins (> 95%), HPLC-MS 255.6 $[M + 2H + H_2O]^{2+}$, 492.2 $[M + H]^+$, 510.2 $[M + H + H_2O]^+$.

EXAMPLE 22. (3a*R*, 6a*S*)-*N*-[(1*S*)-2-(4-Benzoyl-6-oxo-hexahydro-pyrrolo[3,2-*b* ]pyrrol-1-yl)-2-oxo-1-thiophen-2-ylmethyl-ethyl]-4-*tert*-butyl-benzamide

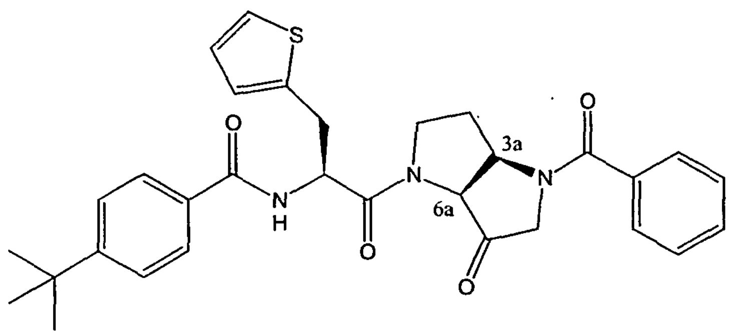

HPLC Rt = 18.0-19.0 mins (> 80%), HPLC-MS 544.2 [M + H]$^+$.

EXAMPLE 23. (3a*R*, 6a*S*)-4-*tert*-Butyl-*N*-{(1*S*)-2-oxo-2-[6-oxo-4-(pyridine-3-carbonyl)-hexahydro-pyrrolo[3,2-*b*]pyrrol-1-yl]-1-thiophen-2-ylmethyl-ethyl}-benzamide

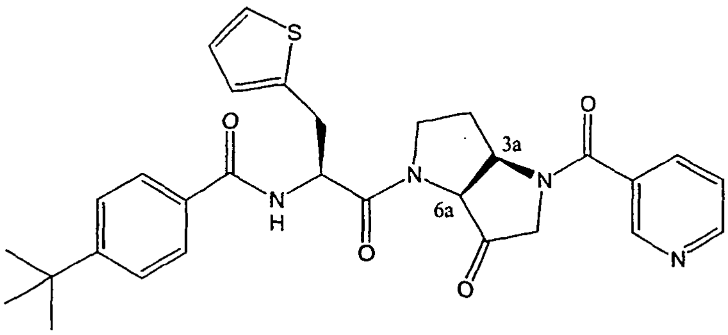

HPLC Rt = 15.626 mins (> 85%), HPLC-MS 545.2 [M + H]$^+$.

EXAMPLE 24. (3a*R*, 6a*S*)-4-*tert*-Butyl-*N*-{(1*S*)-3-methyl-1-[6-oxo-4-(3-phenoxy-benzoyl)-hexahydro-pyrrolo[3,2-*b*]pyrrole-1-carbonyl]-butyl}-benzamide

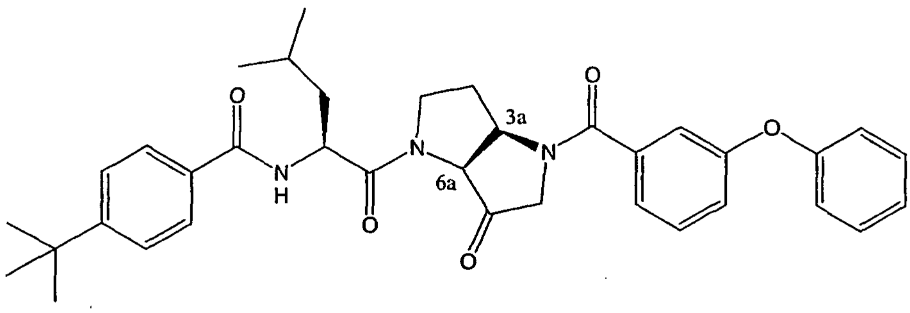

HPLC Rt = 22.0-23.2 mins (> 75%), HPLC-MS 596.1 [M + H]$^+$.

EXAMPLE 25. (3a*R*, 6a*S*)-*N*-{(1*S*)-1-[4-(3-Bromo-benzoyl)-6-oxo-hexahydro-pyrrolo[3,2-*b*]pyrrole-1-carbonyl]-3-methyl-butyl}-4-*tert*-butyl-benzamide

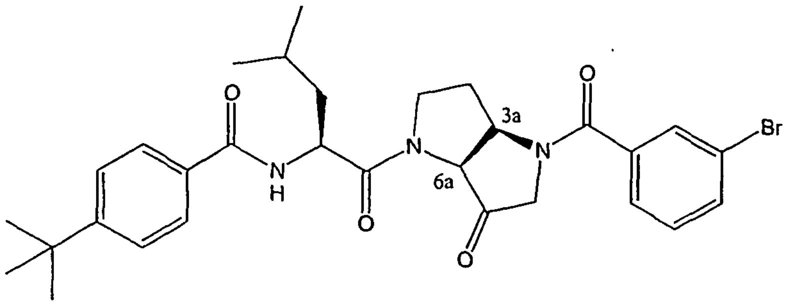

HPLC Rt = 20.3-21.5 mins (> 80%), HPLC-MS 582.1 / 584.1 [M + H]$^+$.

EXAMPLE 26. (3a*R*, 6a*S*)-4-*tert*-Butyl-*N*-{(1*S*)-3-methyl-1-[6-oxo-4-(4-[1,2,4] triazol-1-yl-benzoyl)-hexahydro-pyrrolo-[3,2-*b*]pyrrole-1-carbonyl]-butyl}-benzamide

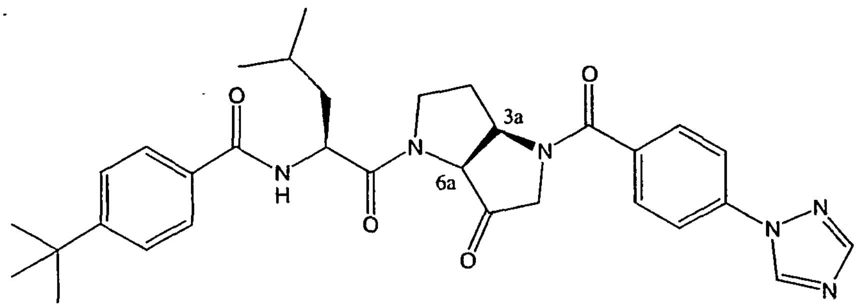

HPLC Rt = 17.4-18.7 mins (> 80%), HPLC-MS 571.1 [M + H]$^+$.

EXAMPLE 27. (3a*R*, 6a*S*)-4-*tert*-Butyl-*N*-{(1*S*)-3-methyl-1-[6-oxo-4-(2-phenyl-thiazole-4-carbonyl)-hexahydro-pyrrolo-[3,2-*b*]pyrrole-1-carbonyl]-butyl}-benzamide

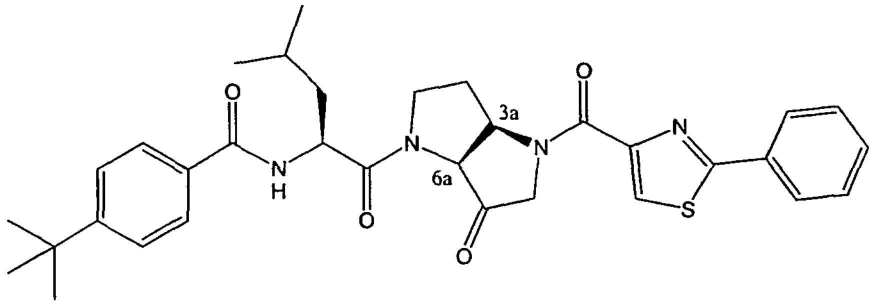

HPLC Rt = 21.4-22.7 mins (> 80%), HPLC-MS 587.1 [M + H]$^+$.

EXAMPLE 28. (3a*R*, 6a*S*)-4-*tert*-Butyl-*N*-{(1*S*)-3-methyl-1-[6-oxo-4-(5-phenyl-thiophene-2-carbonyl)-hexahydro-pyrrolo- [3,2-*b*]pyrrole-1-carbonyl]-butyl}-benzamide

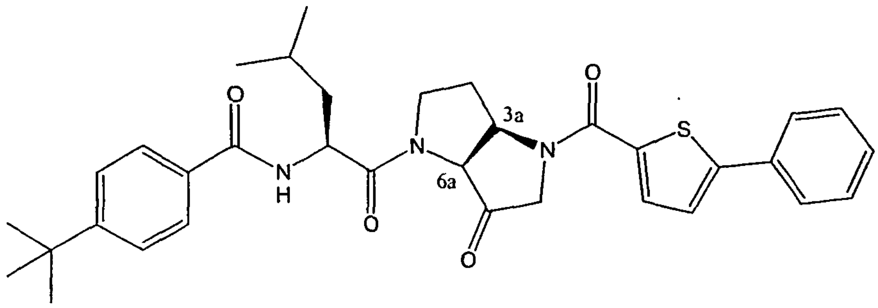

HPLC Rt = 22.0-23.0 mins (> 70%), HPLC-MS 586.1 [M + H]$^+$.

EXAMPLE 29. (3a*R*, 6a*S*)-4-*tert*-Butyl-*N*-{(1*S*)-1-[4-(2,3-dihydro-benzo[1,4]dioxine-2-carbonyl)-6-oxo-hexahydro-pyrrolo[3,2-*b*]pyrrole-1-carbonyl]-3-methyl-butyl}-benzamide

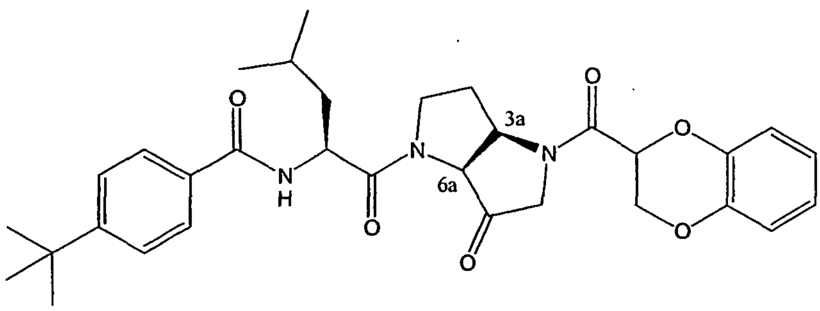

HPLC Rt = 20.1-21.3 mins (> 80%), HPLC-MS 562.1 [M + H]$^+$.

EXAMPLE 30. (3a*R*, 6a*S*)-4-*tert*-Butyl-*N*-{(1*S*)-1-[4-(2,3-dihydro-benzo[1,4]dioxine-6-carbonyl)-6-oxo-hexahydro-pyrrolo[3,2-*b*]pyrrole-1-carbonyl]-3-methyl-butyl}-benzamide

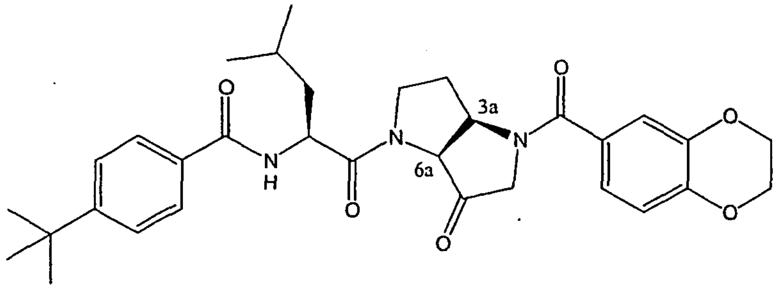

HPLC Rt = 18.3-19.1 mins (> 85%), HPLC-MS 562.1 [M + H]+.

EXAMPLE 31. (3a*R*, 6a*S*)-*N*-{(1*S*)-1-[4-(Benzo[*b*]thiophene-3-carbonyl)-6-oxo-hexahydro-pyrrolo[3,2-*b*]pyrrole-1-carbonyl]-3-methyl-butyl}-4-*tert*-butyl-benzamide

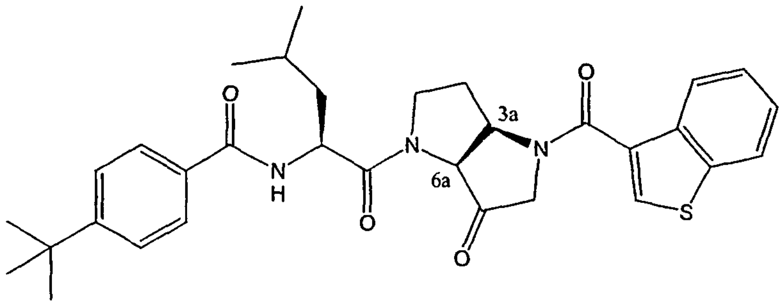

HPLC Rt = 20.5-21.8 mins (> 80%), HPLC-MS 560.2 [M + H]+.

EXAMPLE 32. (3a*R*, 6a*S*)-*N*-{(1*S*)-1-[4-(Benzothiazole-6-carbonyl)-6-oxo-hexahydro-pymolo[3,2-*b*]pyrrole-1-carbonyl]-3-methyl-butyl}-4-*tert*-butyl-benzamide

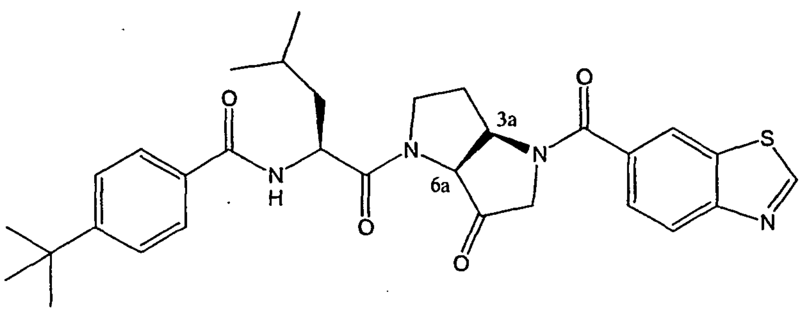

HPLC Rt = 17.9-18.9 mins (> 75%), HPLC-MS 561.2 [M + H]+.

EXAMPLE 33. (3a*R*, 6a*S*)-4-*tert*-Butyl-*N*-{(1*S*)-3-methyl-1-[4-(naphthalene-1-carbonyl)-6-oxo-hexahydro-pyrrolo[3,2-*b*]pyrrole-1-carbonyl]-butyl}-benzamide

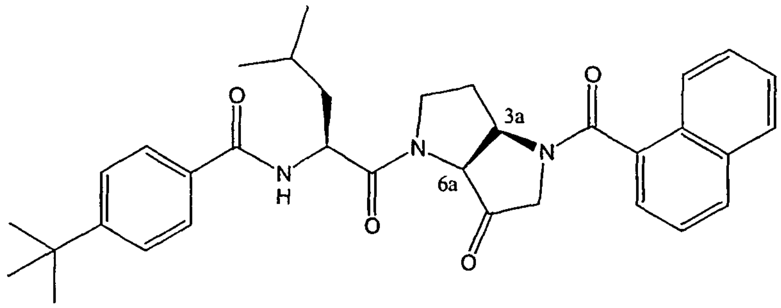

HPLC Rt = 20.5-21.7 mins (> 80%), HPLC-MS 554.1 $[M + H]^+$.

EXAMPLE 34. (3a*R*, 6a*S*)-4-*tert*-Butyl-*N*-{(1*S*)-3-methyl-1-[4-(naphthalene-2-carbonyl)-6-oxo-hexahydro-pyrrolo[3,2-*b*]pyrrole-1-carbonyl]-butyl}-benzamide

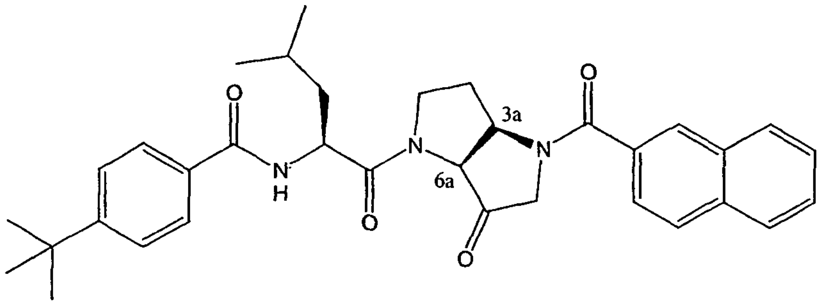

HPLC Rt = 20.7-21.8 mins (> 75%), HPLC-MS 554.2 $[M + H]^+$.

EXAMPLE 35. (3a*R*, 6a*S*)-4-*tert*-Butyl-*N*-{(1*S*)-3-methyl-1-[6-oxo-4-(5-thiophen-2-yl-pyridine-3-carbonyl)-hexahydro-pyrrolo[3,2-*b*]pyrrole-1-carbonyl]-butyl}-benzamide

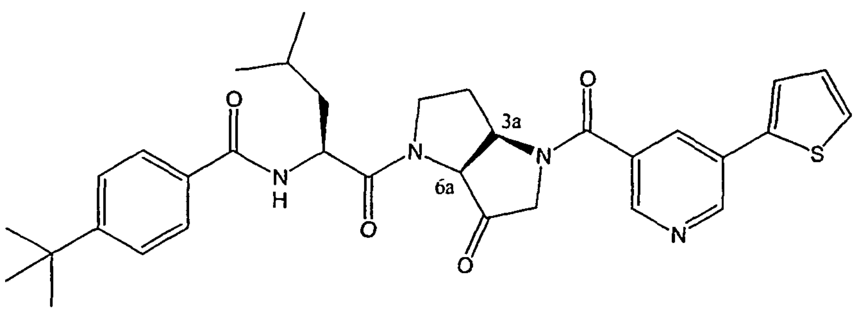

HPLC Rt = 18.9-19.8 mins (> 70%), HPLC-MS 587.1 [M + H]$^+$.

EXAMPLE 36. (3a*R*, 6a*S*)-*N*-{(1*S*)-1-[4-(2-Benzyl-benzoyl)-6-oxo-hexahydro-pyrrolo[3,2-*b*]pyrrole-1-carbonyl]-3-methyl-butyl}-4-*tert*-butyl-benzamide

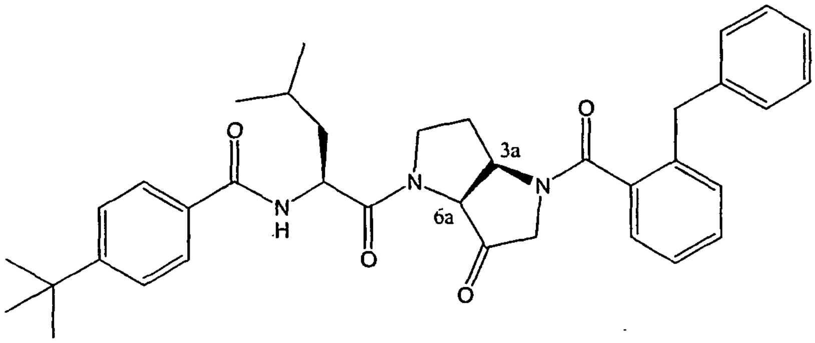

HPLC Rt = 21.8-22.9 mins (> 80%), HPLC-MS 594.1 [M + H]$^+$.

EXAMPLE 37. (3a*S*, 6a*R*)-((1*S*)-1-{4-[(2*S*)-2-(4-Dimethylamino-benzoylamino)-4-methyl-pentanoyl]-3-oxo-hexahydro-pyrrolo[3,2-*b*]pyrrole-1-carbonyl}-3-methyl-butyl)-carbamic acid benzyl ester

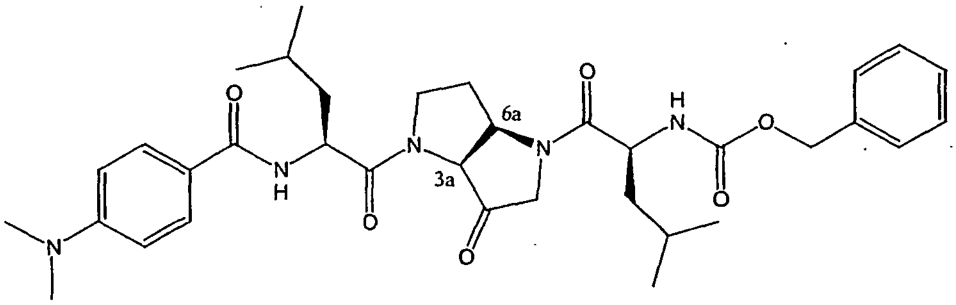

HPLC Rt = 17.15 mins (> 85%), HPLC-MS 634.3 [M + H]$^+$.

EXAMPLE 38. (3a*S*, 6a*R*)-(2-{4-[(2*S*)-2-(4-Dimethylamino-benzoylamino)-4-methyl-pentanoyl]-3-oxo-hexahydro-pyrrolo[3,2-*b*]pyrrol-1-yl}-2-oxo-ethyl)-carbamic acid benzyl ester

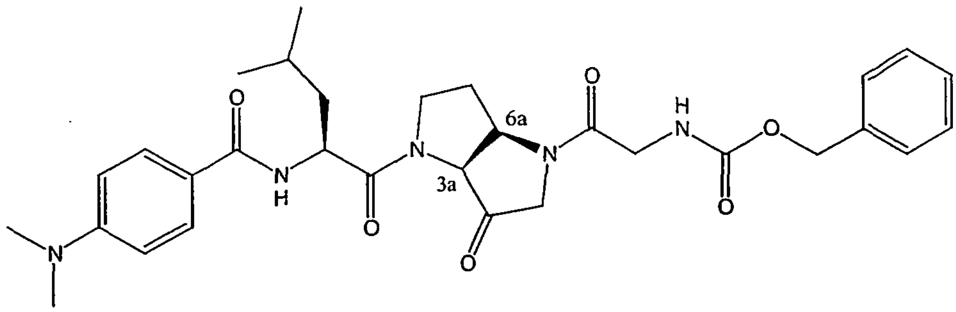

HPLC Rt = 13.79 mins (> 85%), HPLC-MS 578.3 $[M + H]^+$.

EXAMPLE 39. (3a*R*, 6a*S*)-*N*-[(1*S*)-1-(4-Benzoyl-6-oxo-hexahydro-pyrrolo[3,2-*b*] pyrrole-1-carbonyl)-3-methyl-butyl]-4-phenoxy-benzamide

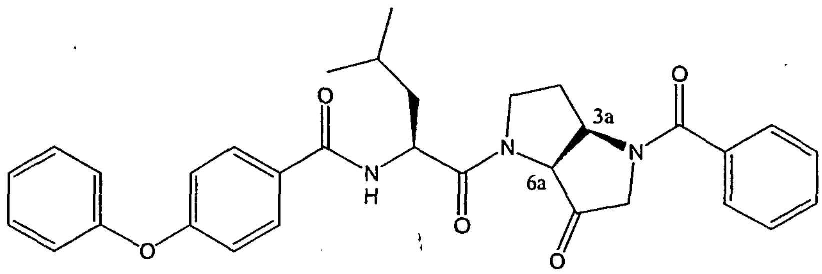

HPLC Rt = 17.5-18.2 mins (> 75%), HPLC-MS 540.2 $[M + H]^+$.

EXAMPLE 40. (3a*R*, 6a*S*)-*N*-{(1*S*)-1-[4-(2-Methanesulfonyl-benzoyl)-6-oxo-hexahydro-pyrrolo[3,2-*b*]pyrrole-1-carbonyl]-3-methyl-butyl}-4-phenoxy-benzamide

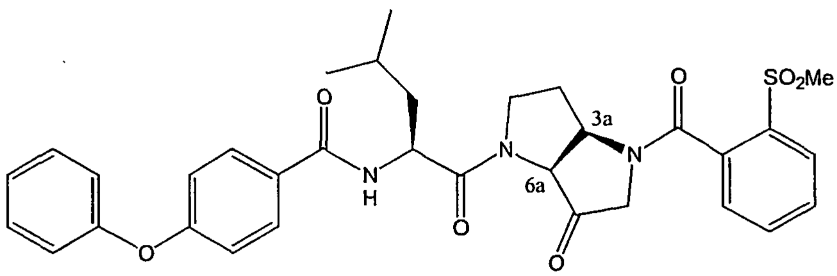

HPLC Rt = 16.9-17.9 mins (> 75%), HPLC-MS 618.2 $[M + H]^+$, 636.2 $[M + H + H_2O]^+$.

EXAMPLE 41. (3a*R*, 6a*S*)-*N*-{(1*S*)-1-[4-(3-Methanesulfonyl-benzoyl)-6-oxo-hexahydro-pyrrolo[3,2-*b*]pyrrole-1-carbonyl]-3-methyl-butyl}-4-phenoxy-benzamide

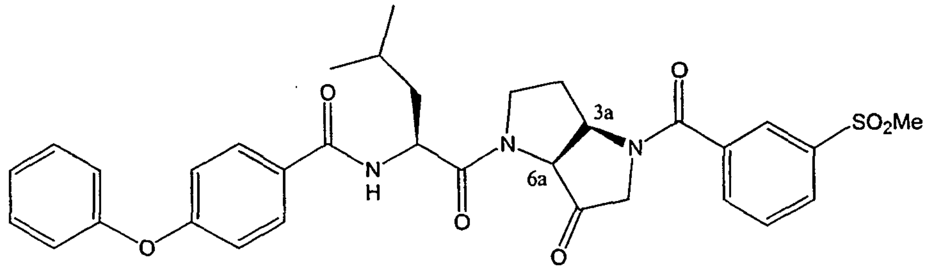

HPLC Rt = 16.6-17.7 mins (> 70%), HPLC-MS 618.2 [M + H]+.

EXAMPLE 42. (3a*R*, 6a*S*)-4-Dimethylamino-*N*-{(1*S*)-1-[4-(2-methanesulfonyl-benzoyl)-6-oxo-hexahydro-pyrrolo-[3,2-*b*]pyrrole-1-carbonyl]-3-methyl-butyl}-benzamide

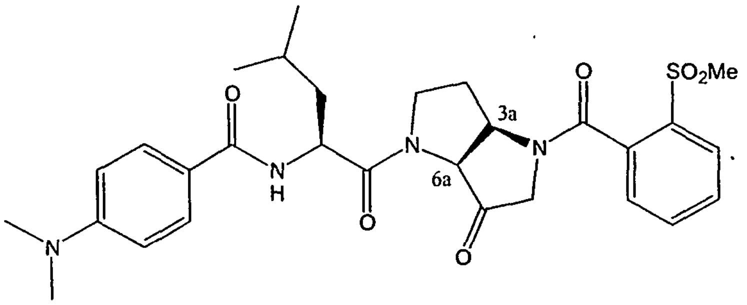

HPLC Rt = 13.10 mins (> 90%), HPLC-MS 569.3 [M + H]+, 1159.4 [2M + Na]+.

EXAMPLE 43. (3a*R*, 6a*S*)-4-Dimethylamino-*N*-{(1*S*)-1-[4-(3-methanesulfonyl-benzoyl)-6-oxo-hexahydro-pyrrolo[3,2-*b*]-pyrrole-1-carbonyl]-3-methyl-butyl}-benzamide

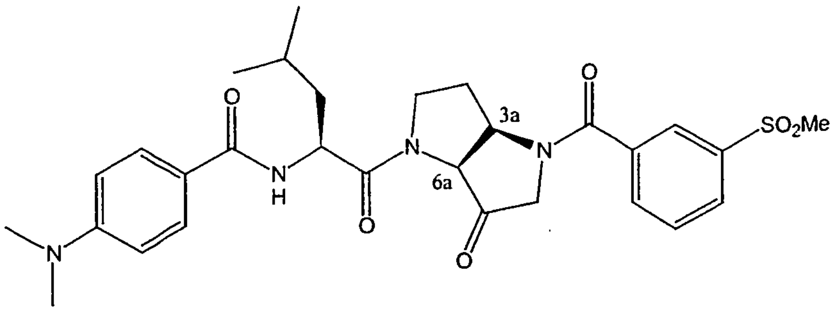

HPLC Rt = 11.59 mins (> 95%), HPLC-MS 569.2 [M + H]+, 587.2 [M + H + $H_2O$]+.

EXAMPLE 44. (3a*R*, 6a*S*)-5-Phenyl-thiophene-2-carboxylic acid [(1*S*)-1-(4-benzoyl-6-oxo-hexahydro-pyrrolo[3,2-*b*]pyrrole-1-carbonyl)-3-methyl-butyl]-amide

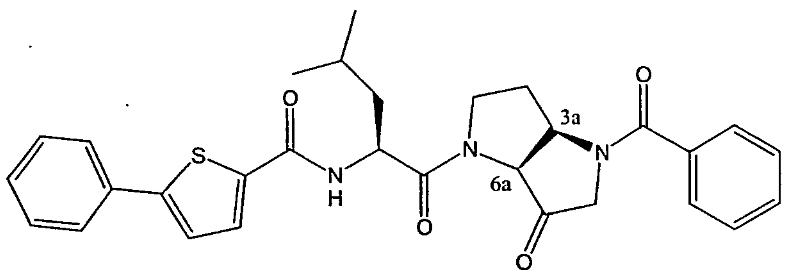

HPLC Rt = 17.2-18.1 mins (> 75%), HPLC-MS 530.2 $[M + H]^+$.

EXAMPLE 45. (3a*R*, 6a*S*)-4-Dimethylamino-*N*-{(1*S*)-3-methyl-1-[4-(2-nitrobenzoyl)-6-oxo-hexahydro-pyrrolo[3,2-*b*]pyrrole-1-carbonyl]-butyl}-benzamide

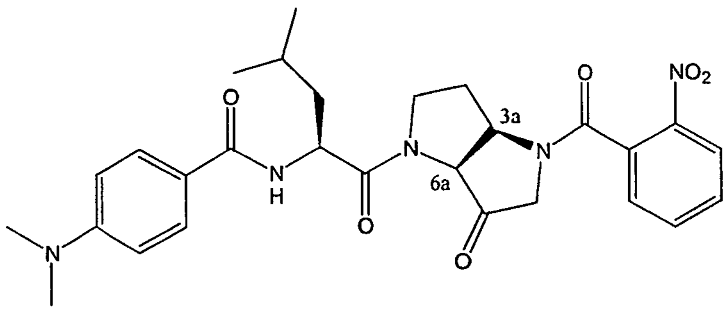

HPLC Rt = 12.47 mins (> 95%), HPLC-MS 536.2 $[M + H]^+$, 554.2 $[M + H + H_2O]^+$.

EXAMPLE 46. (3a*R*, 6a*S*)-4-Dimethylamino-*N*-{(1*S*)-3-methyl-1-[4-(3-nitrobenzoyl)-6-oxo-hexahydro-pyrrolo[3,2-*b*]pyrrole-1-carbonyl]-butyl}-benzamide

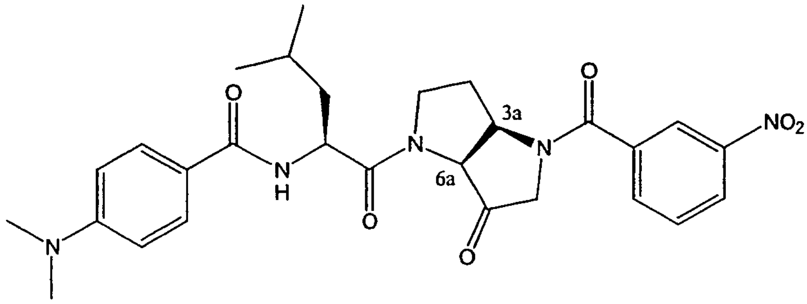

HPLC Rt = 13.17 mins (> 90%), HPLC-MS 536.2 $[M + H]^+$, 554.2 $[M + H + H_2O]^+$.

EXAMPLE 47. (3a*R*, 6a*S*)-4-Dimethylamino-*N*-{(1*S*)-3-methyl-1-[4-(4-nitrobenzoyl)-6-oxo-hexahydro-pyrrolo[3,2-*b*]pyrrole-1-carbonyl]-butyl}-benzamide

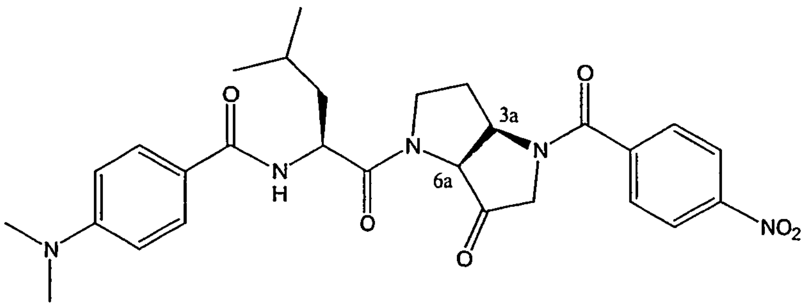

HPLC Rt = 13.19 mins (> 95%), HPLC-MS 536.2 $[M + H]^+$, 554.2 $[M + H + H_2O]^+$.

EXAMPLE 48. (3a*R*, 6a*S*)-*N*-{(1*S*)-1-[4-(Adamantane-1-carbonyl)-6-oxo-hexahydro-pyrrolo[3,2-*b*]pyrrole-1-carbonyl]-3-methyl-butyl}-4-dimethylmino-benzamide

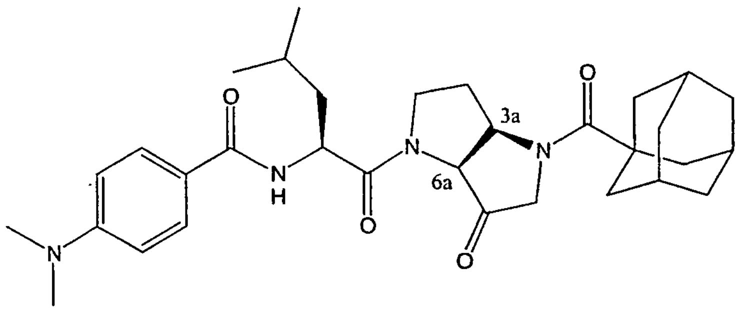

HPLC Rt = 16.4-17.1 mins (> 50%), HPLC-MS 549.3 $[M + H]^+$.

EXAMPLE 49. (3a*R*, 6a*S*)-4-Dimethylamino-*N*-{(1*S*)-1-[4-(4-hydroxy-benzoyl)-6-oxo-hexahydro-pyrrolo[3,2-*b*]pyrrole-1-carbonyl]-3-methyl-butyl}-benzamide

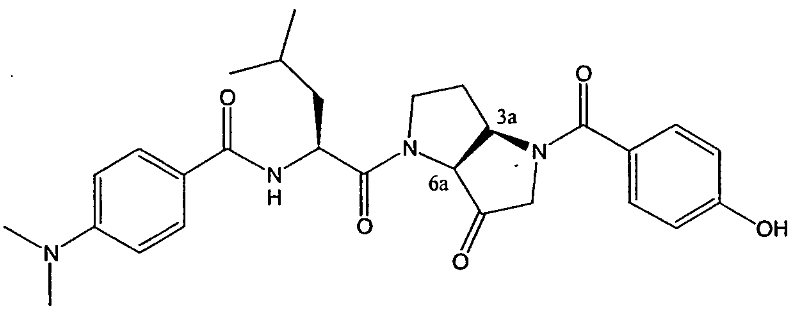

HPLC Rt = 11.05 mins (> 90%), HPLC-MS 507.2 $[M + H]^+$, 525.2 $[M + H + H_2O]^+$.

EXAMPLE 50. (3a*R*, 6a*S*)-*N*-{(1*S*)-1-[4-(4-Amino-benzoyl)-6-oxo-hexahydro-pyrrolo[3,2-*b*]pyrrole-1-carbonyl]-3-methyl-butyl}-4-dimethylamino-benzamide

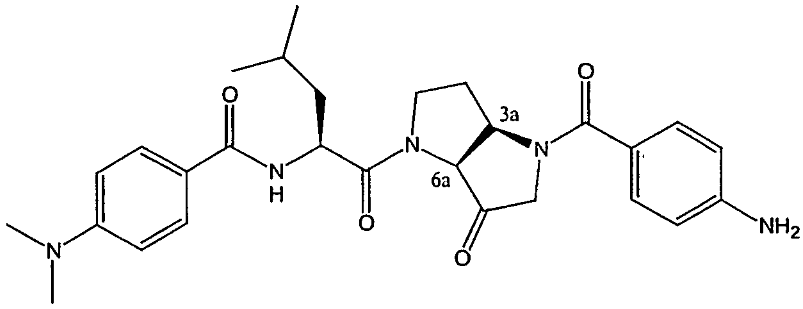

HPLC Rt = 9.33 mins (> 85%), HPLC-MS 506.2 $[M + H]^+$, 524.2 $[M + H + H_2O]^+$.

EXAMPLE 51. (3a*R*, 6a*S*)-4-Dimethylamino-*N*-{(1*S*)-1-[4-(5-methanesulfonyl-thiophene-2-carbonyl)-6-oxo-hexahydro-pyrrolo[3,2-*b*]pyrrole-1-carbonyl]-3-methyl-butyl}-benzamide

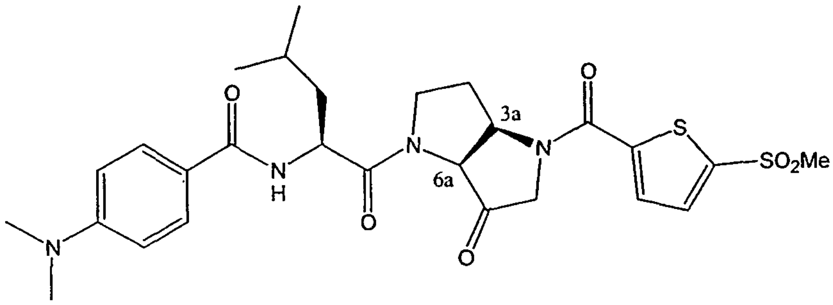

HPLC Rt = 12.10 mins (> 95%), HPLC-MS 575.1 $[M + H]^+$, 593.1 $[M + H + H_2O]^+$.

EXAMPLE 52. (3a*R*, 6a*S*)-4-Dimethylamino-*N*-{(1*S*)-3-methyl-1-[6-oxo-4-(quinoline-6-carbonyl)-hexahydro-pyrrolo[3,2-*b*]pyrrole-1-carbonyl]-butyl}-benzamide

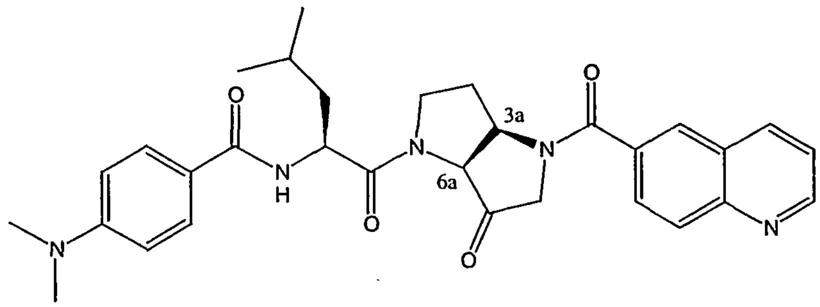

HPLC Rt = 9.99 mins (> 85%), HPLC-MS 542.2 [M + H]$^+$, 560.2 [M + H + $H_2O$]$^+$.

EXAMPLE 53. (3a*R*, 6a*S*)-4-Dimethylamino-*N*-{(1*S*)-3-methyl-1-[4-(2-methylbenzoyl)-6-oxo-hexahydro-pyrrolo[3,2-*b*]pyrrole-1-carbonyl]-butyl}-benzamide

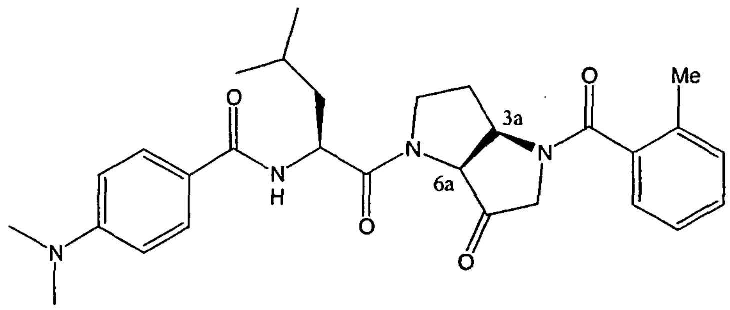

HPLC Rt = 13.08 mins (> 90%), HPLC-MS 505.2 [M + H]$^+$, 523.2 [M + H + $H_2O$]$^+$.

EXAMPLE 54. (3a*R*, 6a*S*)-4-Dimethylamino-*N*-{(1*S*)-3-methyl-1-[4-(3-methylbenzoyl)-6-oxo-hexahydro-pyrrolo[3,2-*b*]pyrrole-1-carbonyl]-butyl}-benzamide

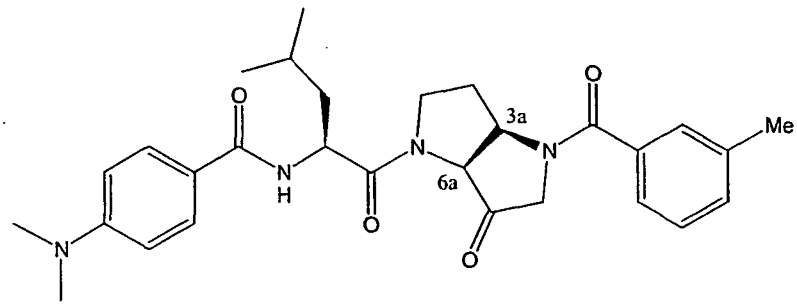

HPLC Rt = 13.12 mins (> 85%), HPLC-MS 505.2 [M + H]$^+$, 523.2 [M + H + $H_2O$]$^+$.

EXAMPLE 55. (3a*R*, 6a*S*)-4-Dimethylamino-*N*-{(1*S*)-3-methyl-1-[4-(4-methylbenzoyl)-6-oxo-hexahydro-pyrrolo[3,2-*b*]pyrrole-1-carbonyl]-butyl}-benzamide

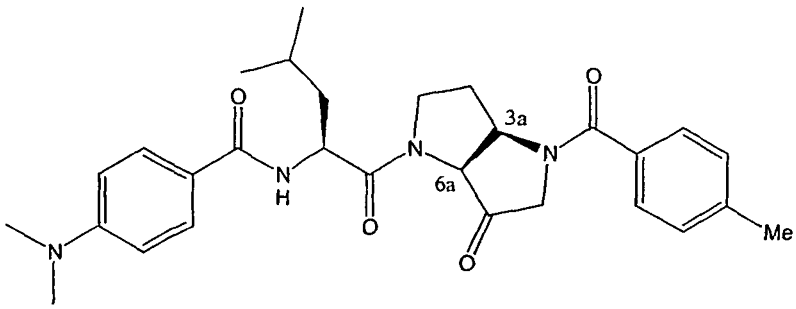

HPLC Rt = 13.65 mins (> 90%), HPLC-MS 505.2 [M + H]$^+$, 523.2 [M + H + $H_2O$]$^+$.

EXAMPLE 56. (3a*R*, 6a*S*)-4-Dimethylamino-*N*-{(1*S*)-3-methyl-1-[6-oxo-4-(4-phenoxy-benzoyl)-hexahydro-pyrrolo[3,2-*b*]pyrrole-1-carbonyl]-butyl}-benzamide

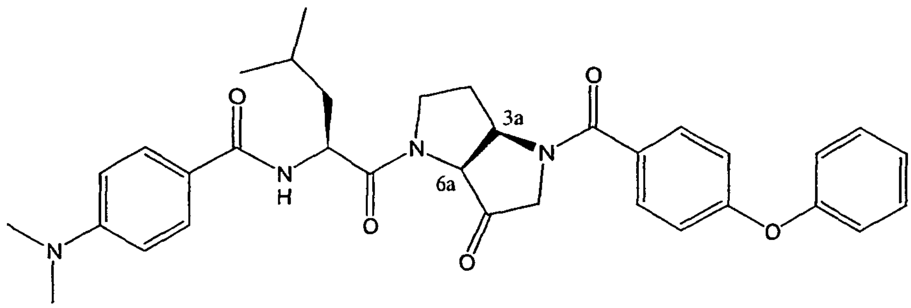

HPLC Rt = 16.38 mins (> 80%), HPLC-MS 583.2 [M + H]$^+$.

EXAMPLE 57. (3a*R*, 6a*S*)-4-Dimethylamino-*N*-{(1*S*)-1-[4-(4-fluoro-benzoyl)-6-oxo-hexahydro-pyrrolo[3,2-*b*]pyrrole-1-carbonyl]-3-methyl-butyl}-benzamide

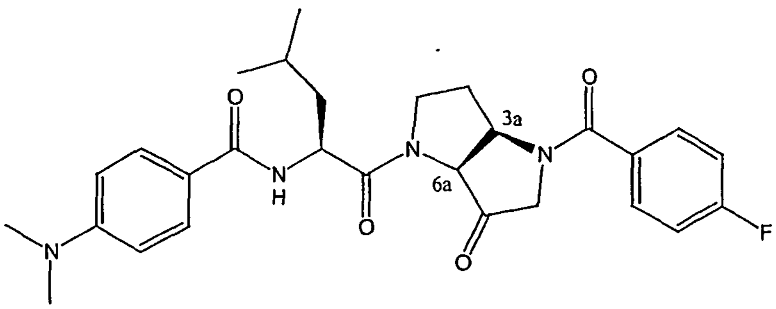

HPLC Rt = 12.83 mins (> 90%), HPLC-MS 509.1 [M + H]$^+$, 527.1 [M + H + $H_2O$]$^+$.

EXAMPLE 58. (3a*R*, 6a*S*)-*N*-{(1*S*)-1-[4-(3-Methanesulfonyl-benzoyl)-6-oxo-hexahydro-pyrrolo[3,2-*b*]pyrrole-1-carbonyl]-3-methyl-butyl}-4-nitro-benzamide

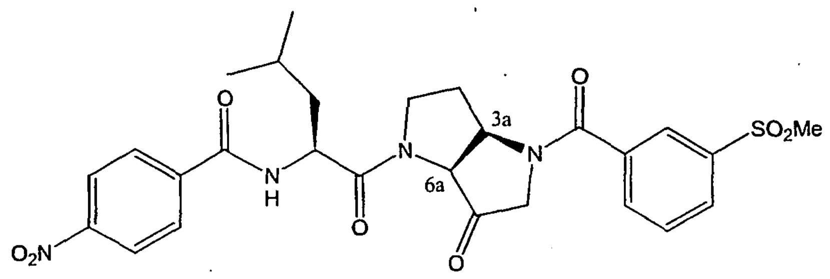

HPLC Rt =13.37 mins (> 95%), HPLC-MS 571.1 $[M + H]^+$.

EXAMPLE 59. (3a*R*, 6a*S*)-3-Dimethylamino-*N*-{(1*S*)-1-[4-(3-methanesulfonyl-benzoyl)-6-oxo-hexahydro-pyrrolo[3,2-*b*]pyrrole-1-carbonyl]-3-methyl-butyl}-benzamide

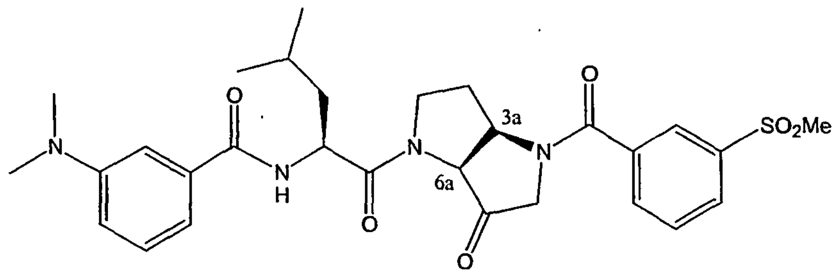

HPLC Rt = 10.29 mins (> 85%), HPLC-MS 569.1 $[M + H]^+$, 587.2 $[M + H + H_2O]^+$.

EXAMPLE 60. (3a*R*, 6a*S*)-4-Diethylamino-*N*-{(1*S*)-1-[4-(3-methanesulfonyl-benzoyl)-6-oxo-hexahydro-pyrrolo[3,2-*b*]pyrrole-1-carbonyl]-3-methyl-butyl}-benzamide

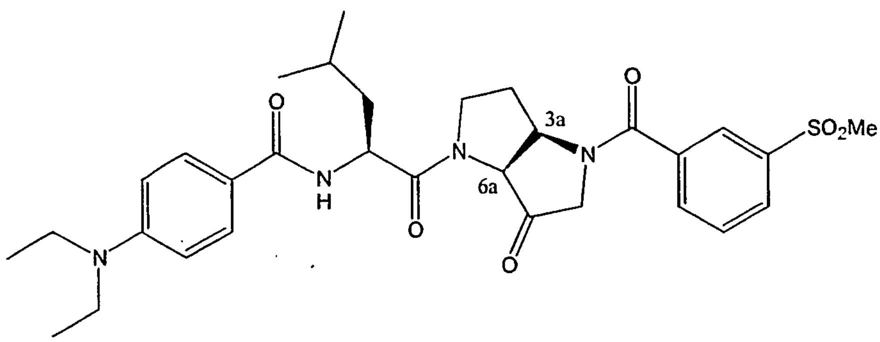

HPLC Rt = 11.13 mins (> 90%), HPLC-MS 597.2 $[M + H]^+$, 615.2 $[M + H + H_2O]^+$.

EXAMPLE 61. (3a*R*, 6a*S*)-4-Amino-*N*-{(1*S*)-1-[4-(3-methanesulfonyl-benzoyl)-6-oxo-hexahydro-pyrrolo[3,2-*b*]pyrrole-1-carbonyl]-3-methyl-butyl}-benzamide

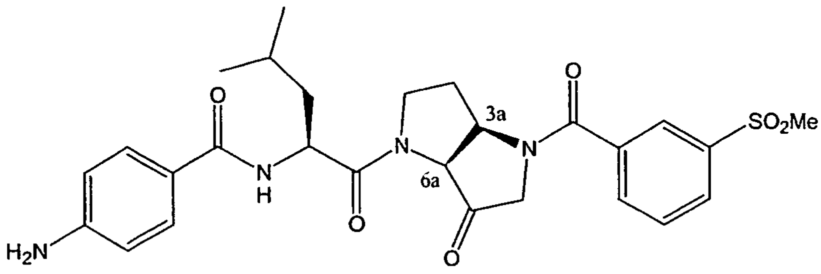

HPLC Rt = 9.69 mins (> 80%), HPLC-MS 541.1 [M + H]$^+$.

EXAMPLE 62. (3a*R*, 6a*S*)-4-Dimethylamino-*N*-{(1*S*)-1-[4-(2-fluoro-benzoyl)-6-oxo-hexahydro-pyrrolo[3,2-*b*]pyrrole-1-carbonyl]-3-methyl-butyl}-benzamide

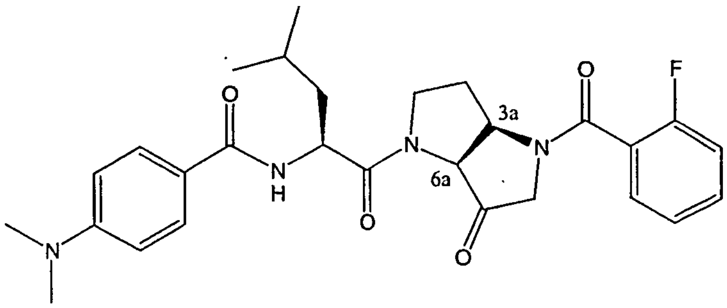

HPLC Rt = 9.18 mins (> 95%), HPLC-MS 509.2 [M + H]$^+$, 527.2 [M + H + $H_2O$]$^+$.

EXAMPLE 63. (3a*R*, 6a*S*)-4-Dimethylamino-*N*-{(1*S*)-1-[4-(3-fluoro-benzoyl)-6-oxo-hexahydro-pyrrolo[3,2-*b*]pyrrole-1-carbonyl]-3-methyl-butyl}-benzamide

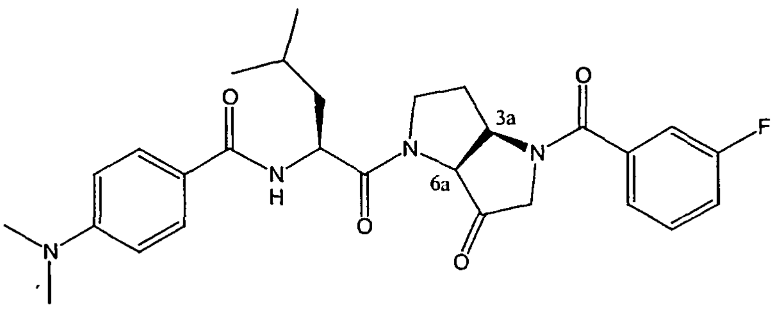

HPLC Rt = 10.59 mins (> 90%), HPLC-MS 509.2 [M + H]$^+$, 527.2 [M + H + $H_2O$]$^+$.

EXAMPLE 64. (3a*R*, 6a*S*)-4-Dimethylamino-*N*-{(1*S*)-1-[4-(2-methoxy-benzoyl)-6-oxo-hexahydro-pyrrolo[3,2-*b*]pyrrole-1-carbonyl]-3-methyl-butyl}-benzamide

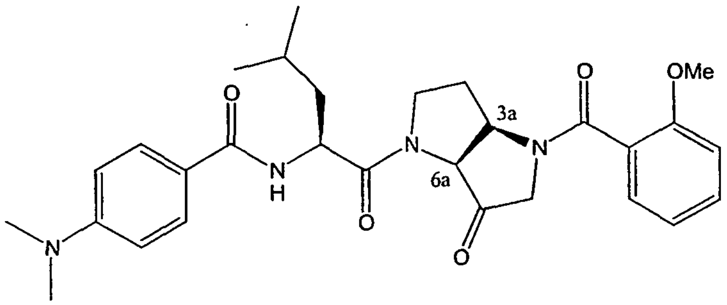

HPLC Rt = 11.20 mins (> 95%), HPLC-MS 521.2 $[M + H]^+$.

EXAMPLE 65. (3a*R*, 6a*S*)-4-Dimethylamino-*N*-{(1*S*)-1-[4-(3-methoxy-benzoyl)-6-oxo-hexahydro-pyrrolo[3,2-*b*]pyrrole-1-carbonyl]-3-methyl-butyl}-benzamide

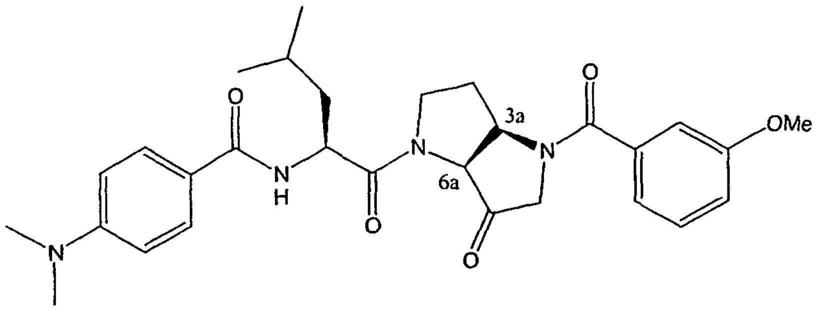

HPLC Rt = 12.5 mins (> 90%), HPLC-MS 521.2 $[M + H]^+$.

EXAMPLE 66. (3a*R*, 6a*S*)-4-Dimethylamino-*N*-{(1*S*)-1-[4-(4-methoxy-benzoyl)-6-oxo-hexahydro-pyrrolo[3,2-*b*]pyrrole-1-carbonyl]-3-methyl-butyl}-benzamide

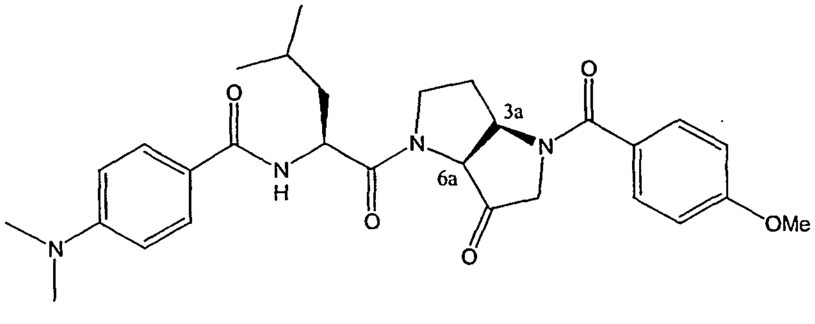

HPLC Rt = 13.33 mins (> 95%), HPLC-MS 521.1 $[M + H]^+$.

EXAMPLE 67. (3a*R*, 6a*S*)-4-Dimethylamino-*N*-{(1*S*)-3-methyl-1-[6-oxo-4-(2-trifluoromethyl-benzoyl)-hexahydro-pyrrolo[3,2-*b*]pyrrole-1-carbonyl]-butyl}-benzamide

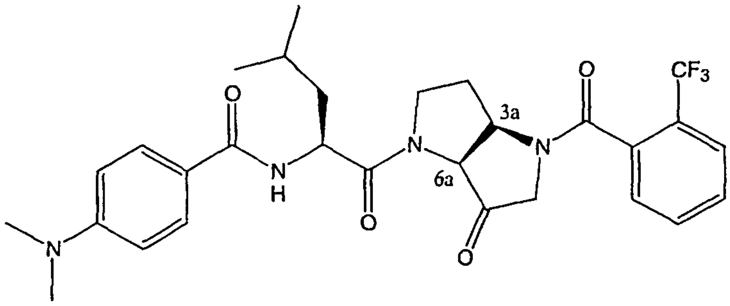

HPLC Rt = 10.98 mins (> 95%), HPLC-MS 559.2 $[M + H]^+$, 577.2 $[M + H + H_2O]^+$.

EXAMPLE 68. (3a*R*, 6a*S*)-4-Dimethylamino-*N*-{(1*S*)-3-methyl-1-[6-oxo-4-(3-trifluoromethyl-benzoyl)-hexahydro-pyrrolo[3,2-*b*]pyrrole-1-carbonyl]-butyl}-benzamide

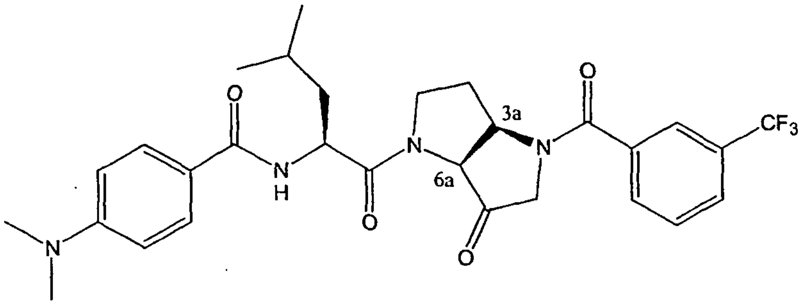

HPLC Rt = 12.11 mins (> 95%), HPLC-MS 559.2 $[M + H]^+$, 577.2 $[M + H + H_2O]^+$.

EXAMPLE 69. (3a*R*, 6a*S*)-4-Dimethylamino-*N*-{(1*S*)-3-methyl-1-[6-oxo-4-(4-trifluoromethyl-benzoyl)-hexahydro-pyrrolo[3,2-*b*]pyrrole-1-carbonyl]-butyl}-benzamide

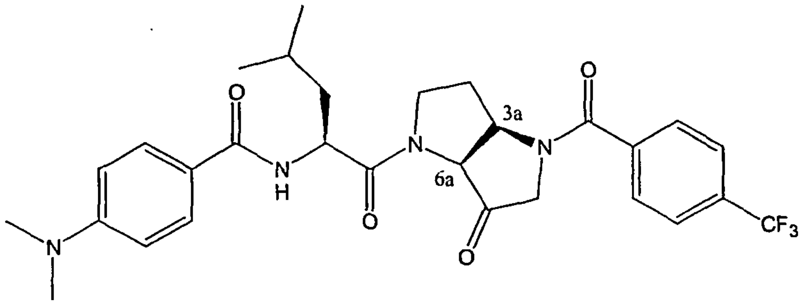

HPLC Rt = 9.76 mins (> 95%), HPLC-MS 559.2 $[M + H]^+$, 577.2 $[M + H + H_2O]^+$.

EXAMPLE 70. (3a*R*, 6a*S*)-4-Dimethylamino-*N*-{(1*S*)-3-methyl-1-[6-oxo-4-(3-trifluoromethoxy-benzoyl)-hexahydro-pyrrolo[3,2-*b*]pyrrole-1-carbonyl]-butyl}-benzamide

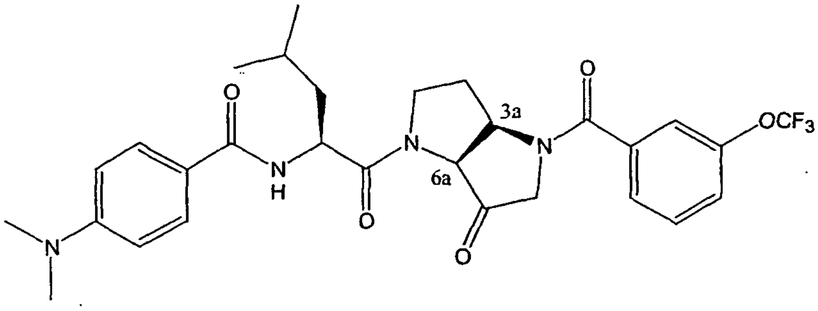

HPLC Rt = 10.42 mins (> 95%), HPLC-MS 575.2 $[M + H]^+$, 593.2 $[M + H + H_2O]^+$.

EXAMPLE 71. (3a*R*, 6a*S*)-4-Dimethylamino-*N*-{(1*S*)-3-methyl-1-[6-oxo-4-(4-trifluoromethoxy-benzoyl)-hexahydro-pyrrolo[3,2-*b*]pyrrole-1-carbonyl]-butyl}-benzamide

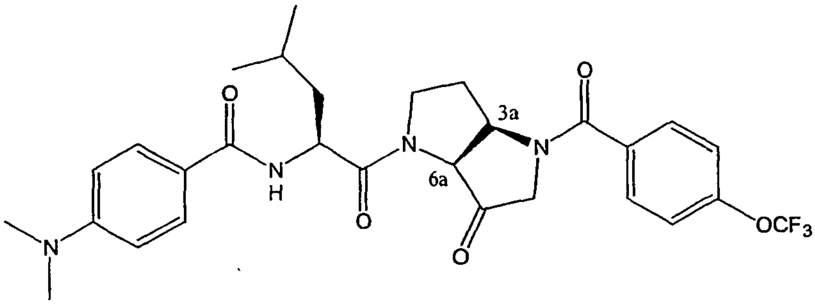

HPLC Rt = 10.48 mins (> 95%), HPLC-MS 575.2 $[M + H]^+$, 593.2 $[M + H + H_2O]^+$.

EXAMPLE 72. (3a*R*, 6a*S*)-*N*-{(1*S*)-1-[4-(2-Chloro-benzoyl)-6-oxo-hexahydro-pyrrolo[3,2-*b*]pyrrole-1-carbonyl]-3-methyl-butyl}-4-dimethylamino-benzamide

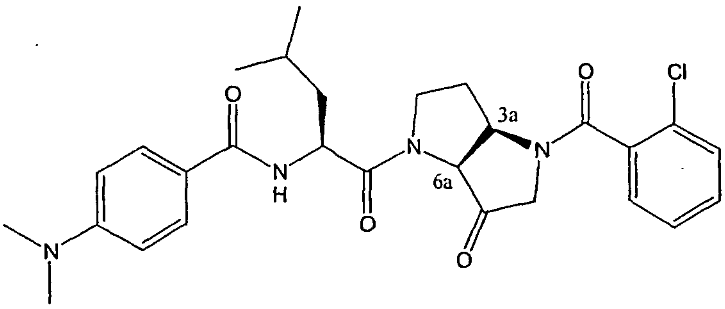

HPLC Rt = 9.99 mins (> 95%), HPLC-MS 525.2 / 527.2 $[M + H]^+$, 543.2 / 545.2 $[M + H + H_2O]^+$.

EXAMPLE 73. (3a*R*, 6a*S*)-*N*-{(1*S*)-1-[4-(3-Chloro-benzoyl)-6-oxo-hexahydro-pyrrolo[3,2-*b*]pyrrole-1-carbonyl]-3-methyl-butyl}-4-dimethylamino-benzamide

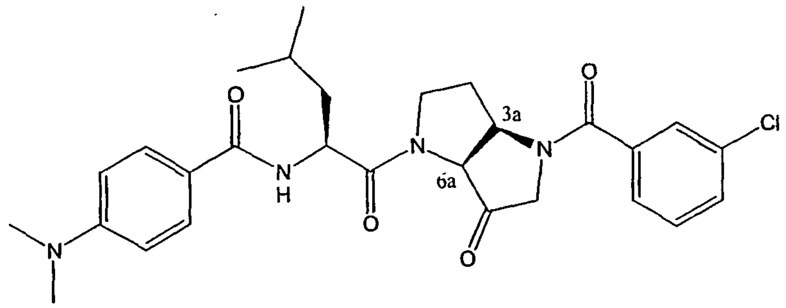

HPLC Rt = 11.08 mins (> 95%), HPLC-MS 525.2 / 527.2 $[M + H]^+$, 543.2 / 545.2 $[M + H + H_2O]^+$.

EXAMPLE 74. (3a*R*, 6a*S*)-*N*-{(1*S*)-1-[4-(4-Chloro-benzoyl)-6-oxo-hexahydro-pyrrolo[3,2-*b*]pyrrole-1-carbonyl]-3-methyl-butyl}-4-dimethylamino-benzainide

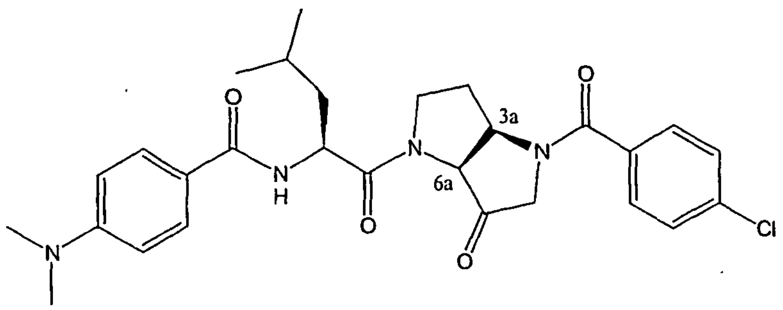

HPLC Rt = 11.03 mins (> 95%), HPLC-MS 525.2 / 527.2 $[M + H]^+$, 543.2 / 545.2 $[M + H + H_2O]^+$.

EXAMPLE 75. (3a*R*, 6a*S*)-*N*-{(1*S*)-1-[4-(4-*tert*-Butyl-benzoyl)-6-oxo-hexahydro-pyrrolo[3,2-*b*]pyrrole-1-carbonyl]-3-methyl-butyl}-4-dimethylamino-benzamide

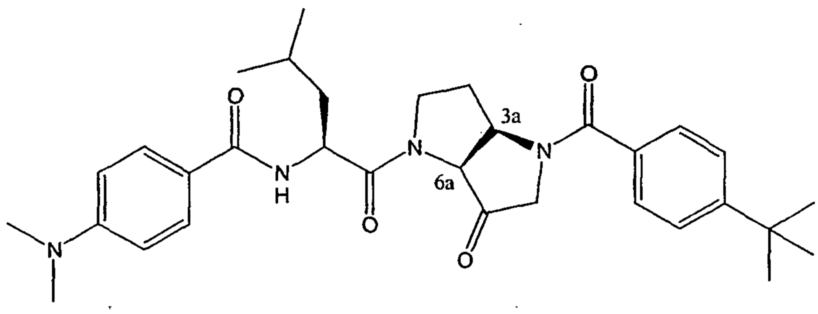

HPLC Rt = 11.51 mins (> 95%), HPLC-MS 547.3 $[M + H]^+$.

EXAMPLE 76. (3a*S*, 6a*R*)-4-{4-[(2*S*)-2-(4-Dimethylamino-benzoylamino)-4-methyl-pentanoyl]-3-oxo-hexahydro-pyrrolo[3,2-*b*]pyrrole-1-carbonyl}-benzoic acid methyl ester

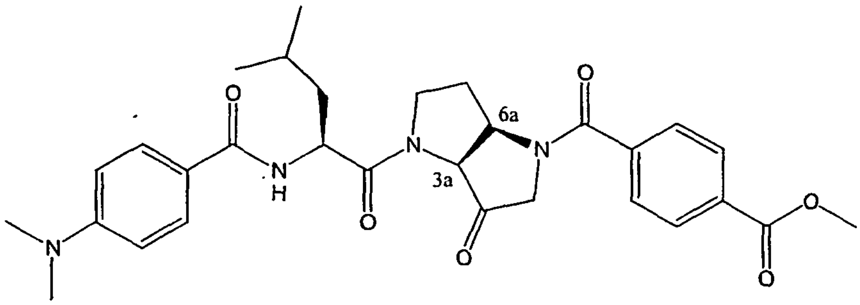

HPLC Rt = 9.76 mins (> 95%), HPLC-MS 549.2 $[M + H]^+$, 567.2 $[M + H + H_2O]^+$.

EXAMPLE 77. (3a*R*, 6a*S*)-*N*-{(1*S*)-1-[4-(Benzo[1,3]dioxole-5-carbonyl)-6-oxo-hexahydro-pyrrolo[3,2-*b*]pyrrole-1-carbonyl]-3-methyl-butyl}-4-dimethylamino-benzamide

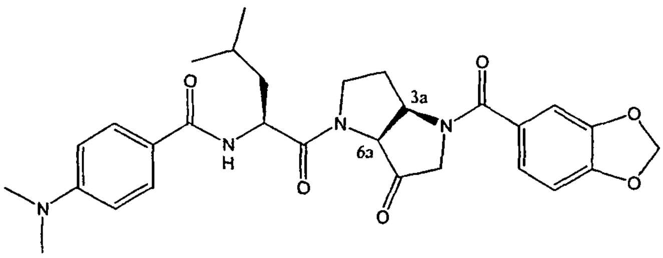

HPLC Rt =12.3 mins (> 90%), HPLC-MS 535.2 $[M + H]^+$.

EXAMPLE 78. (3a*R*, 6a*S*)-4-Dimethylamino-*N*-[(1*S*)-1-(4-diphenylacetyl-6-oxo-hexahydro-pyrrolo[3,2-*b*]pyrrole-1-carbonyl)-3-methyl-butyl]-benzamide

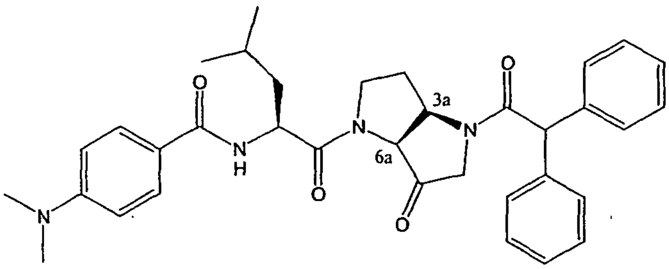

HPLC Rt = 14.0-15.1 mins (> 85%), HPLC-MS 581.2 $[M + H]^+$.

EXAMPLE 79. (3a*R*, 6a*S*)-4-Dimethylamino-*N*-{(1*S*)-3-methyl-1-[6-oxo-4-(pyridine-2-carbonyl)-hexahydro-pyrrolo[3,2-*b*]pyrrole-1-carbonyl]-butyl}-benzamide

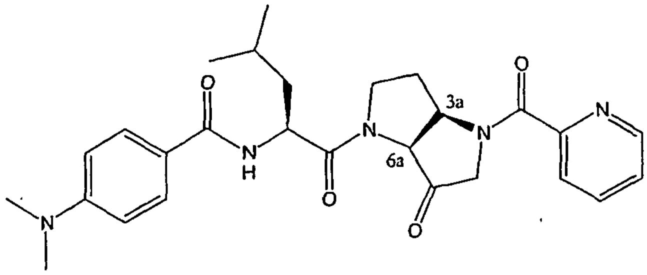

HPLC Rt = 6.17 mins (> 95%), HPLC-MS 492.2 [M + H]$^+$.

EXAMPLE 80. (3a*R*, 6a*S*)-4-Dimethylamino-*N*-{1*S*)-3-methyl-1-[6-oxo-4-(1-oxy-pyridine-2-carbonyl)-hexahydro-pyrrolo[3,2-*b*]pyrrole-1-carbonyl]-butyl}-benzamide

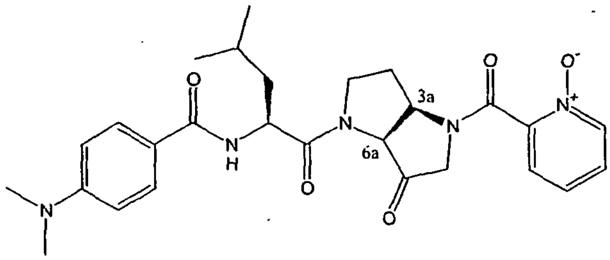

HPLC Rt = 5.82 mins (> 75%), HPLC-MS 508.2 [M + H]$^+$.

Oxidation of the intermediate was performed as detailed in the general soild phase methods prior to compound release from the solid phase.

EXAMPLE 81. (3a*R*, 6a*S*)-4-Dimethylamino-*N*-{(1*S*)-3-methyl-1-[6-oxo-4-(1-oxy-pyridine-3-carbonyl)-hexahydro-pyrrolo[3,2-*b*]pyrrole-1-carbonyl]-butyl}-benzamide

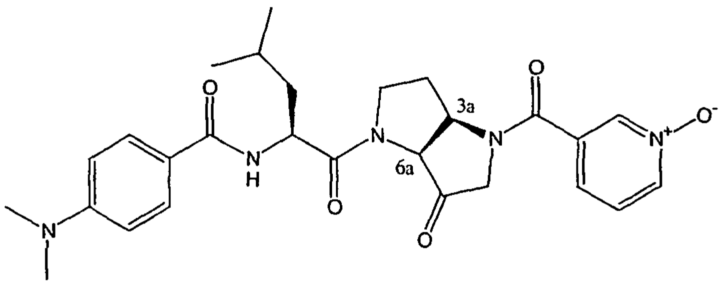

HPLC Rt = 4.7 mins (> 95%), HPLC-MS 508.2 [M + H]$^+$, 526.2 [M + H + $H_2O$]$^+$.

Oxidation of the intermediate was performed as detailed in the general soild phase methods prior to compound release from the solid phase.

EXAMPLE 82. (3a*R*, 6a*S*)-4-Dimethylamino-*N*-{(1*S*)-3-methyl-1-[6-oxo-4-(pyridine-4-carbonyl)-hexahydro-pyrrolo[3,2-*b*]pyrrole-1-carbonyl]-butyl}-benzamide

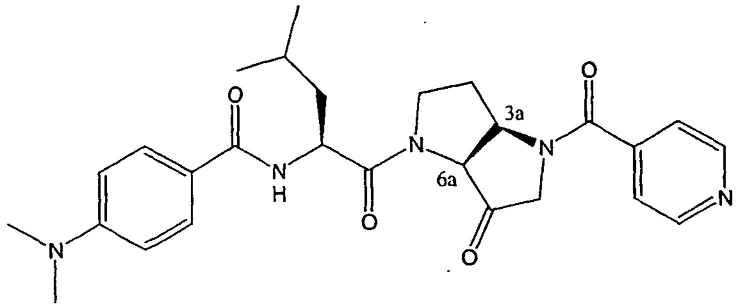

HPLC Rt = 4.5 mins (> 95%), HPLC-MS 492.2 $[M + H]^+$, 510.2 $[M + H + H_2O]^+$.

EXAMPLE 83. (3a*R*, 6a*S*)-4-Dimethylamino-*N*-{(1*S*)-3-methyl-1-[6-oxo-4-(1-oxy-pyridine-4-carbonyl)-hexahydro-pyrrolo[3,2-*b*]pyrrole-1-carbonyl]-butyl}-benzamide

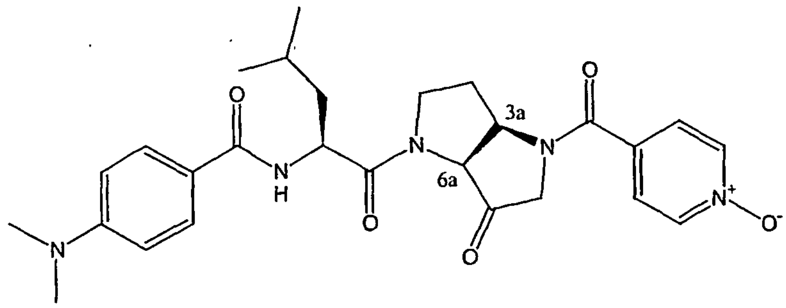

HPLC Rt = 4.9 mins (> 95%), HPLC-MS 508.2 $[M + H]^+$, 526.2 $[M + H + H_2O]^+$.

Oxidation of the intermediate was performed as detailed in the general soild phase methods prior to compound release from the solid phase.

EXAMPLE 84. (3a*R*, 6a*S*)-1-(4-Trifluoromethyl-pyrimidin-2-yl)-piperidine-4-carboxylic acid [(1*S*)-1-(4-benzoyl-6-oxo-hexahydro-pyrrolo[3,2-*b*]pyrrole-1-carbonyl)-3-methyl-butyl]-amide

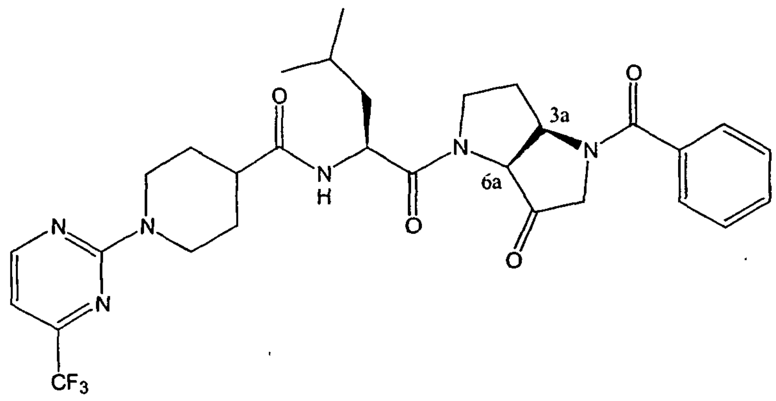

HPLC Rt = 16.49 mins (> 95%), HPLC-MS 601.2 [M + H]+.

EXAMPLE 85. (3a*R*, 6a*S*)-*N*-[(1*S*)-1-(4-Benzoyl-6-oxo-hexahydro-pyrrolo[3,2-*b*] pyrrole-1-carbonyl)-3-methyl-butyl]-4-morpholin-4-yl-benzamide

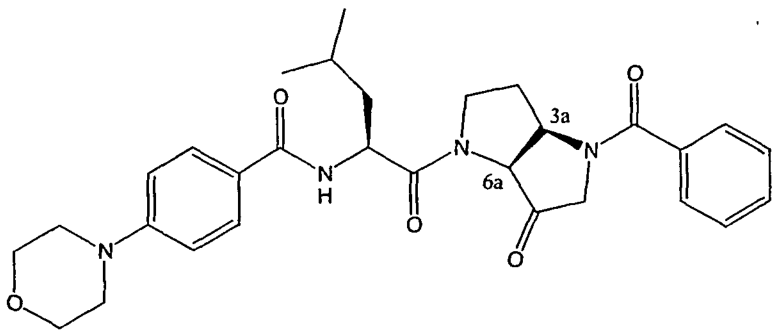

HPLC Rt = 9.37 mins (> 95%), HPLC-MS 533.2 [M + H]+.

EXAMPLE 86. (3a*R*, 6a*S*)-Quinoline-6-carboxylic acid [(1*S*)-1-(4-benzoyl-6-oxo-hexahydro-pyrrolo[3,2-*b*]pyrrole-1-carbonyl)-3-methyl-butyl]-amide

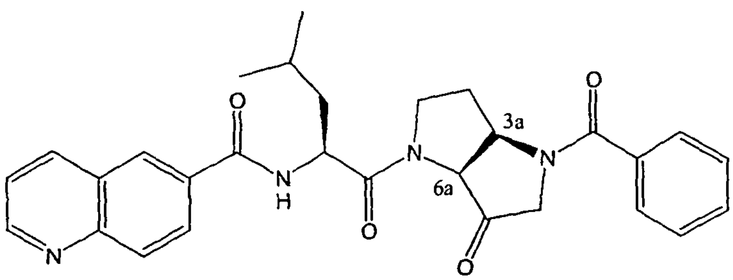

HPLC Rt = 11.73 mins (> 95%), HPLC-MS 499.2 [M + H]+.

EXAMPLE 87. (3a*R*, 6a*S*)-1,2,3,4-Tetrahydro-quinoline-6-carboxylic acid [(1*S*)-1-(4-benzoyl-6-oxo-hexahydro-pyrrolo[3,2-*b*]pyrrole-1-carbonyl)-3-methyl-butyl]-amide

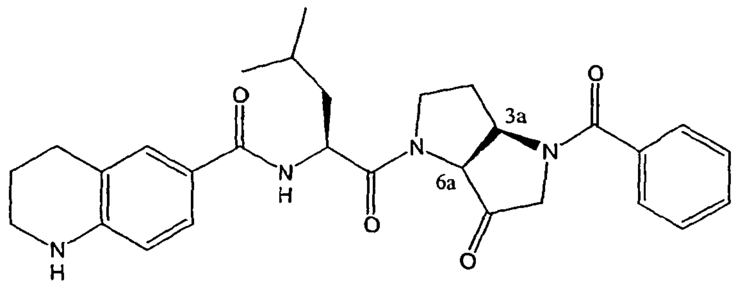

HPLC Rt = 9.06 mins (> 95%), HPLC-MS 503.2 $[M + H]^+$, 521.2 $[M + H + H_2O]^+$.

EXAMPLE 88. (3a*R*, 6a*S*)-1*H*-Indole-6-carboxylic acid [(1*S*)-1-(4-benzoyl-6-oxo-hexahydro-pyrrolo[3,2-*b*]pyrrole-1-carbonyl)-3-methyl-butyl]-amide

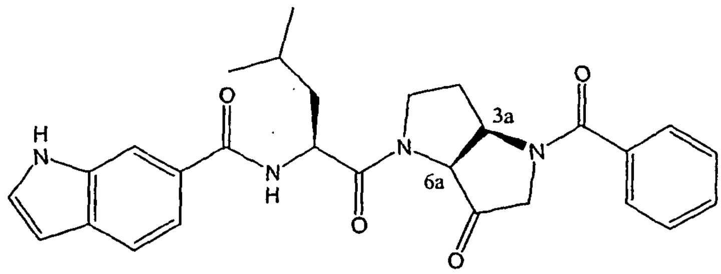

HPLC Rt = 10.13 mins (> 85%), HPLC-MS 487.1 $[M + H]^+$.

EXAMPLE 89. (3a*R*, 6a*S*)-1*H*-Indole-5-carboxylic acid [(1*S*)-1-(4-benzoyl-6-oxo-hexahydro-pyrrolo[3,2-*b*]pyrrole-1-carbonyl)-3-methyl-butyl]-amide

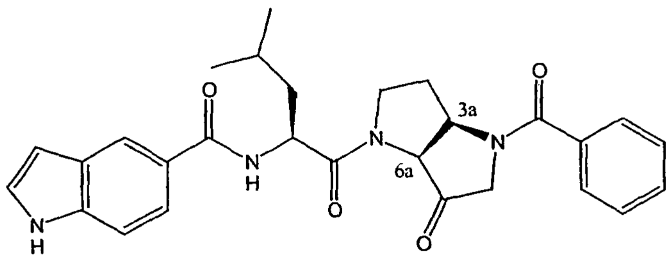

HPLC Rt = 9.58 mins (> 85%), HPLC-MS 487.2 $[M + H]^+$.

EXAMPLE 90. (3a*R*, 6a*S*)-Benzothiazole-6-carboxylic acid [(1*S*)-1-(4-benzoyl-6-oxo-hexahydro-pyrrolo[3,2-*b*]pyrrole-1-carbonyl)-3-methyl-butyl]-amide

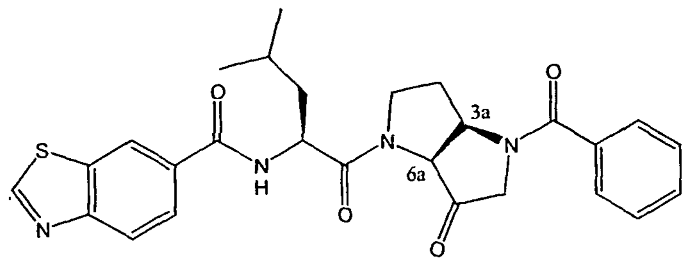

HPLC Rt = 14.15 mins (> 95%), HPLC-MS 505.1 $[M + H]^+$.

EXAMPLE 91. (3a*R*, 6a*S*)-*N*-[(1*S*)-1-(4-Benzoyl-6-oxo-hexahydro-pyrrolo[3,2-*b*] pyrrole-1-carbonyl)-3-methyl-butyl]-4-pyrazol-1-yl-benzamide

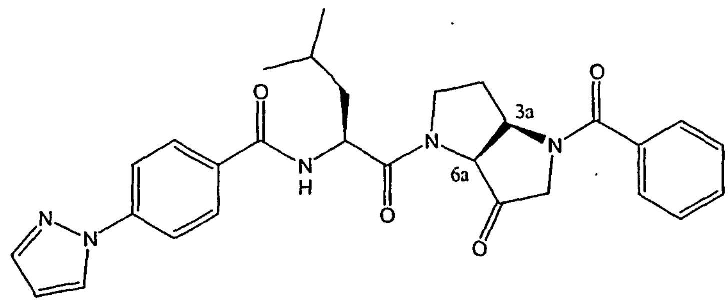

HPLC Rt = 10.25 mins (> 95%), HPLC-MS 514.2 $[M + H]^+$.

EXAMPLE 92. (3a*R*, 6a*S*)-3-Aminomethyl-*N*-[(1*S*)-1-(4-benzoyl-6-oxo-hexahydro-pyrrolo[3,2-*b*]pyrrole-1-carbonyl)-3-methyl-butyl]-benzamide

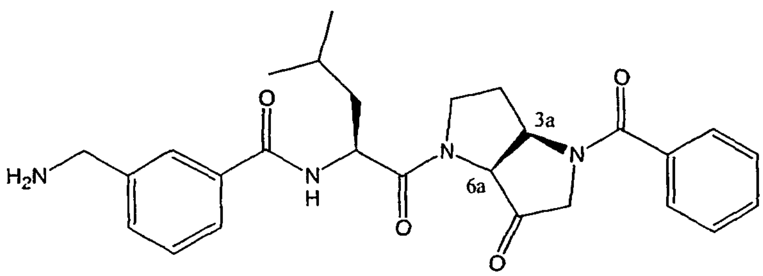

HPLC Rt = 7.42 mins (> 95%), HPLC-MS 477.2 $[M + H]^+$, 495.2 $[M + H + H_2O]^+$, 975.3 $[2M + Na]^+$.

EXAMPLE 93. (3a*R*, 6a*S*)-4-Aminomethyl-*N*-[(1*S*)-1-(4-benzoyl-6-oxo-hexahydro-pyrrolo[3,2-*b*]pyrrole-1-carbonyl)-3-methyl-butyl]-benzamide

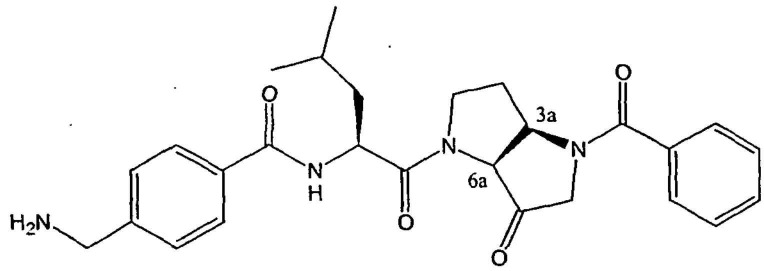

HPLC Rt = 6.86 mins (> 95%), HPLC-MS 477.2 $[M + H]^+$, 495.2 $[M + H + H_2O]^+$, 975.3 $[2M + Na]^+$.

EXAMPLE 94. (3a*R*, 6a*S*)-4-Dimethylamino-*N*-{(1*S*)-3-methyl-1-[4-(6-morpholin-4-yl-pyridine-3-carbonyl)-6-oxo-hexahydro-pyrrolo[3,2-*b*]pyrrole-1-carbonyl]-butyl}-benzamide

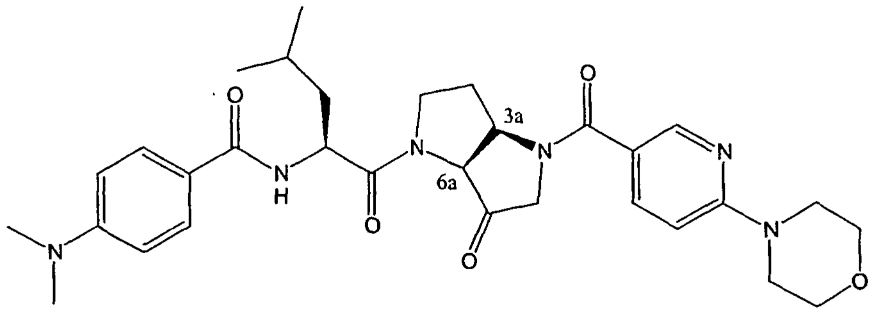

HPLC Rt = 4.4 mins (> 90%), HPLC-MS 577.2 $[M + H]^+$.

EXAMPLE 95. (3a*R*, 6a*S*)-4-Dimethylamino-*N*-{(1*S*)-3-methyl-1-[6-oxo-4-(thiophene-2-carbonyl)-hexahydro-pyrrolo[3,2-*b*]pyrrole-1-carbonyl]-butyl}-benzamide

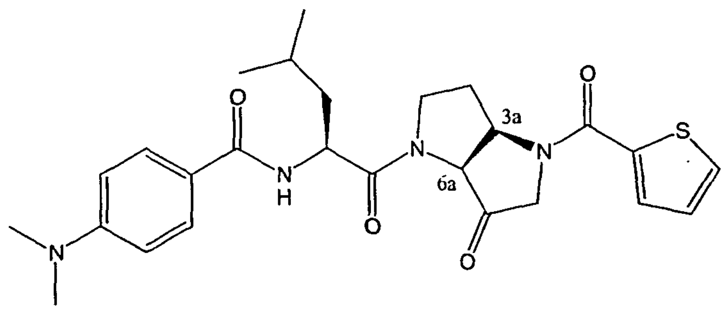

HPLC Rt = 7.7 mins (> 90%), HPLC-MS 497.1 $[M + H]^+$.

EXAMPLE 96. (3a*R*, 6a*S*)-4-Dimethylamino-*N*-{(1*S*)-1-[4-(furan-2-carbonyl)-6-oxo-hexahydro-pyrrolo[3,2-*b*]pyrrole-1-carbonyl]-3-methyl-butyl}-benzamide

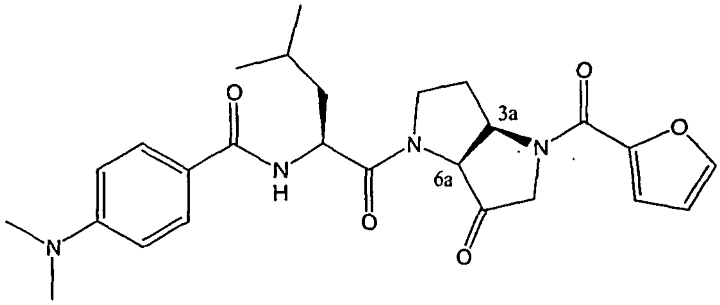

HPLC Rt = 7.3 mins (> 90%), HPLC-MS 481.2 $[M + H]^+$, 499.2 $[M + H + H_2O]^+$, 983.3 $[2M + Na]^+$.

EXAMPLE 97. (3a*R*, 6a*S*)-4-Dimethylamino-*N*-{(1*S*)-1-[4-(furan-3-carbonyl)-6-oxo-hexahydro-pyrrolo[3,2-*b*]pyrrole-1-carbonyl]-3-methyl-butyl}-benzamide

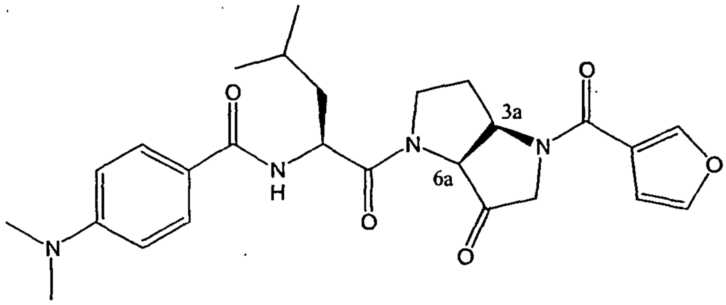

HPLC Rt = 7.30 mins (> 90%), HPLC-MS 481.2 $[M + H]^+$, 499.2 $[M + H + H_2O]^+$, 983.3 $[2M + Na]^+$.

EXAMPLE 98. (3a*R*, 6a*S*)-4-Dimethylamino-*N*-{(1*S*)-1-[4-(1*H*-indole-5-carbonyl)-6-oxo-hexahydro-pyrrolo[3,2-*b*]pyrrole-1-carbonyl]-3-methyl-butyl}-benzamide

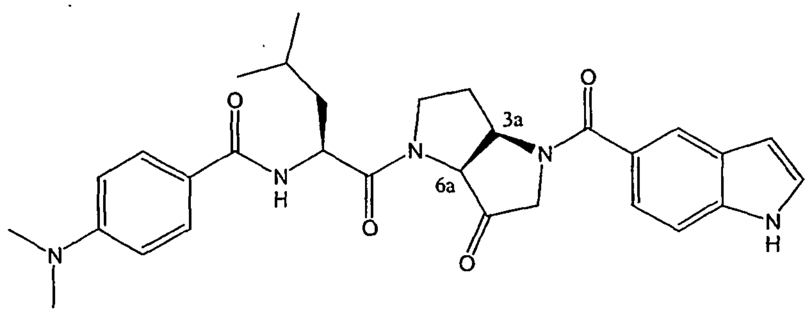

HPLC Rt = 9.0 mins (> 80%), HPLC-MS 530.2 [M + H]$^+$.

EXAMPLE 99. (3a*R*, 6a*S*)-4-Dimethylamino-*N*-{(1*S*)-1-[4-(1*H*-indole-6-carbonyl)-6-oxo-hexahydro-pyrrolo[3,2-*b*]pyrrole-1-carbonyl]-3-methyl-butyl}-benzamide

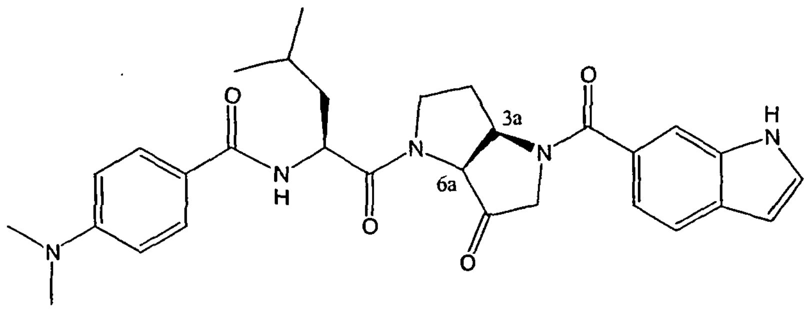

HPLC Rt = 7.8 mins (> 80%), HPLC-MS 530.2 [M + H]$^+$.

EXAMPLE 100. (3a*R*, 6a*S*)-*N*-{(1*S*)-1-[4-(Benzo[*b*]thiophene-2-carbonyl)-6-oxo-hexahydro-pyrrolo[3,2-*b*]pyrrole-1-carbonyl]-3-methyl-butyl}-4-dimethylamino-benzamide

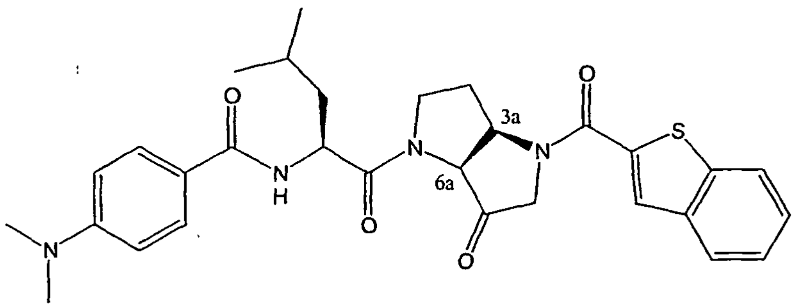

HPLC Rt = 12.85 mins (> 95%), HPLC-MS 547.2 [M + H]$^+$.

EXAMPLE 101. (3a*R*, 6a*S*)-*N*-{(1*S*)-1-[4-(Benzofuran-2-carbonyl)-6-oxo-hexahydro-pyrrolo[3,2-*b*]pyrrole-1-carbonyl]-3-methyl-butyl}-4-dimethylamino-benzamide

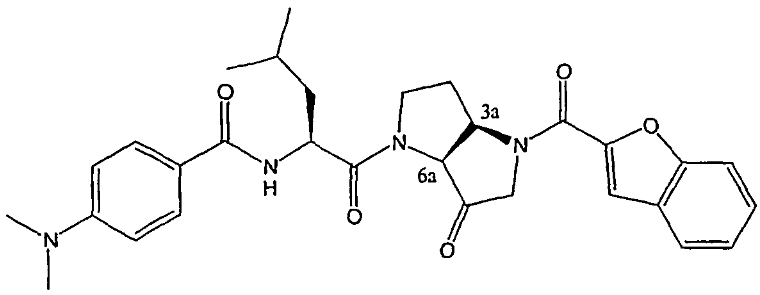

HPLC Rt = 11.9 mins (> 95%), HPLC-MS 531.2 [M + H]$^+$.

EXAMPLE 102. (3a*R*, 6a*S*)-*N*-{(1*S*)-1-[4-(Benzo[1,2,5]oxadiazole-5-carbonyl)-6-oxo-hexahydro-pyrrolo[3,2-*b*]pyrrole-1-carbonyl]-3-methyl-butyl}-4-dimethylamino-benzamide

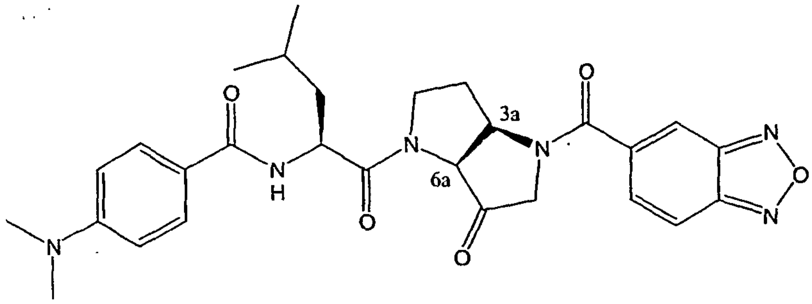

HPLC Rt = 7.06 mins (> 90%), HPLC-MS 533.2 [M + H]$^+$, 551.2 [M + H + $H_2O$]$^+$.

EXAMPLE 103. (3a*R*, 6a*S*)-*N*-{(1*S*)-1-[4-(Benzo[*b*]thiophene-3-carbonyl)-6-oxo-hexahydro-pyrrolo[3,2-*b*]pyrrole-1-carbonyl]-3-methyl-butyl}-4-dimethylamino-benzamide

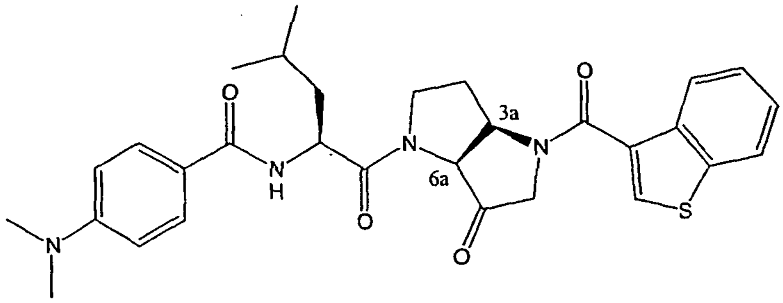

HPLC Rt = 11.8 mins (> 95%), HPLC-MS 547.2 [M + H]$^+$.

EXAMPLE 104. (3a*R*, 6a*S*)-*N*-[(1*S*)-1-(4-Cyclohexanecarbonyl-6-oxo-hexahydro-pyrrolo[3,2-*b*]pyrrole-1-carbonyl)-3-methyl-butyl]-4-dimethylamino-benzamide

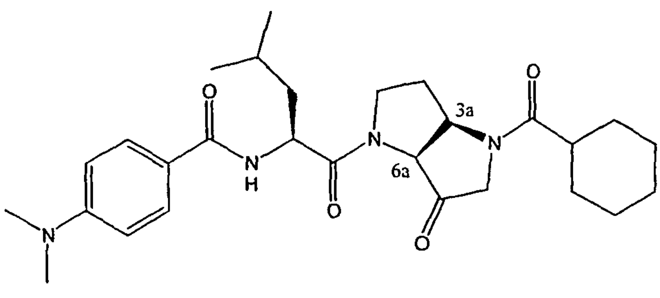

HPLC Rt = 12.9-13.9 mins (> 90%), HPLC-MS 497.2 [M + H]$^+$, 515.2 [M + H + $H_2O$]$^+$.

EXAMPLE 105. (3a*R*, 6a*S*)-4-Dimethylamino-*N*-{(1*S*)-3-methyl-1-[4-(4-methylpentanoyl)-6-oxo-hexahydro-pyrrolo-[3,2-*b*]pyrrole-1-carbonyl]-butyl}-benzamide

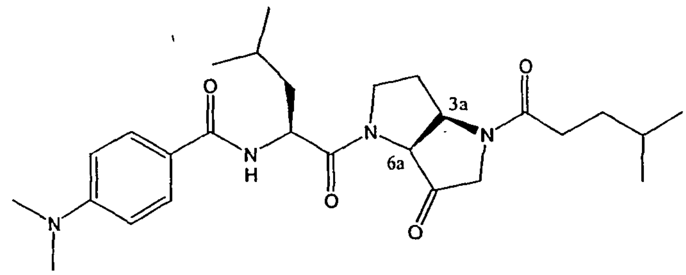

HPLC Rt = 13.08 mins (> 90%), HPLC-MS 485.2 [M + H]$^+$, 503.2 [M + H + $H_2O$]$^+$, 991.4 [2M + Na]$^+$,

EXAMPLE 106. (3a*S*, 6a*R*)-4-{4-[(2*S*)-2-(4-Dimethylamino-benzoylamino)-4-methyl-pentanoyl]-3-oxo-hexahydro-pyrrolo[3,2-*b*]pyrrol-1-yl}-4-oxo-butyric acid

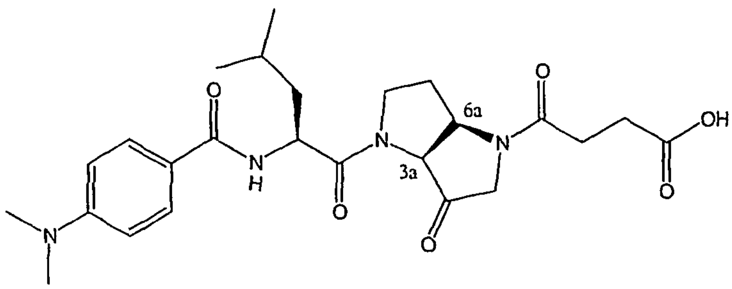

HPLC Rt = 4.35 mins (> 90%), HPLC-MS 487.2 [M + H]$^+$.

EXAMPLE 107. (3a*S*, 6a*R*)-5-{4-[(2*S*)-2-(4-Dimethylamino-benzoylamino)-4-methyl-pentanoyl]-3-oxo-hexahydro-pyrrolo[3,2-*b*]pyrrol-1-yl}-5-oxo-pentanoic acid

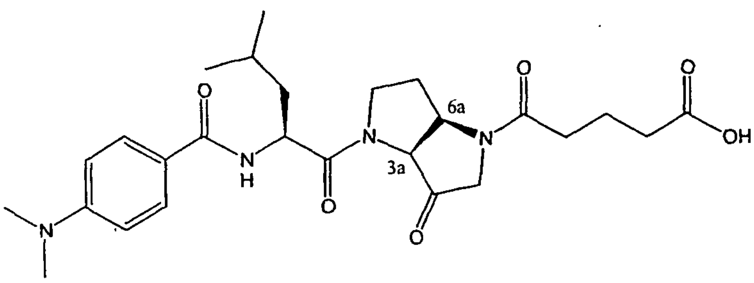

HPLC Rt = 4.99 mins (> 90%), HPLC-MS 501.2 [M + H]$^+$.

EXAMPLE 108. (3a*R*, 6a*S*)-*N*-{(1*S*)-1-[4-(3-Amino-propionyl)-6-oxo-hexahydro-pyrrolo[3,2-*b*]pyrrole-1-carbonyl]-3-methyl-butyl}-4-dimethylamino-benzamide

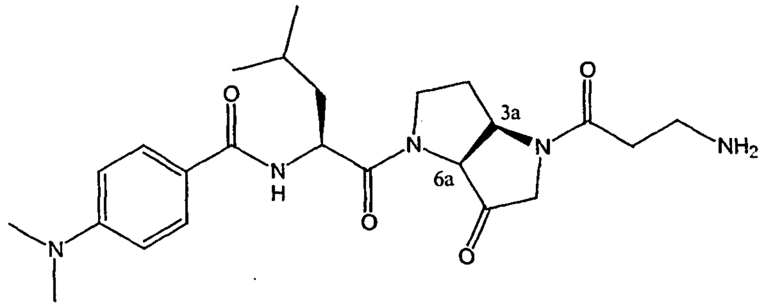

HPLC Rt = 3.9 mins (> 90%), HPLC-MS 458.2 [M + H]$^+$, 915.4 [2M + H]$^+$.

EXAMPLE 109. (3a*S*, 6a*R*)-((1*R*)-1-{4-[(2*S*)-2-(4-Dimethylamino-benzoylamino)-4-methyl-pentanoyl]-3-oxo-hexahydro-pyrrolo[3,2-*b*]pyrrole-1-carbonyl}-3-methyl-butyl)-carbamic acid benzyl ester

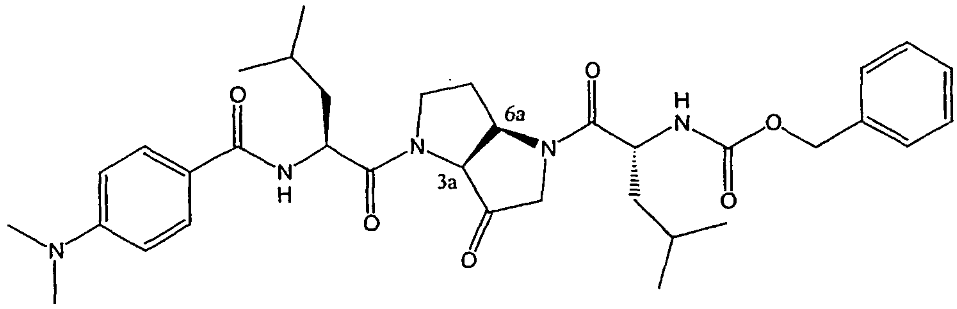

HPLC Rt = 13.0-14.2 mins (> 90%), HPLC-MS 634.3 [M + H]$^+$.

EXAMPLE 110. (3a*R*, 6a*S*)-*N*-[(1*S*)-1-(4-Acetyl-6-oxo-hexahydro-pyrrolo[3,2-*b*] pyrrole-1-carbonyl)-3-methyl-butyl]-4-dimethylamino-benzamide

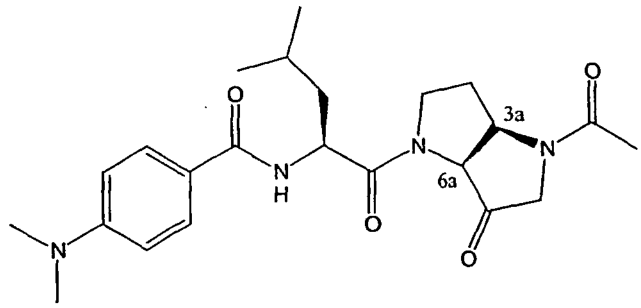

HPLC Rt = 4.58 mins (> 90%), HPLC-MS 429.2 [M + H]$^+$, 451.2 [M + Na]$^+$.

EXAMPLE 111. (3a*R*, 6a*S*)-4-Dimethylamino-*N*-{(1*S*)-1-[4-(3,5-dimethylbenzoyl)-6-oxo-hexahydro-pyrrolo[3,2-*b*]pyrrole-1-carbonyl]-3-methyl-butyl}-benzamide

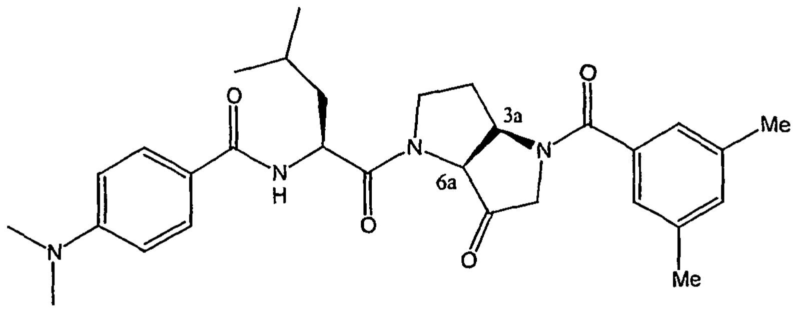

HPLC Rt = 15.53 mins (> 90%), HPLC-MS 519.3 $[M + H]^+$.

EXAMPLE 112. (3a*R*, 6a*S*)-*N*-{(1*S*)-1-[4-(3,5-Dimethoxy-benzoyl)-6-oxo-hexahydro pyrrolo[3,2-*b*]pyrrole-1-carbonyl]-3-methyl-butyl}-4-dimethylamino-benzamide

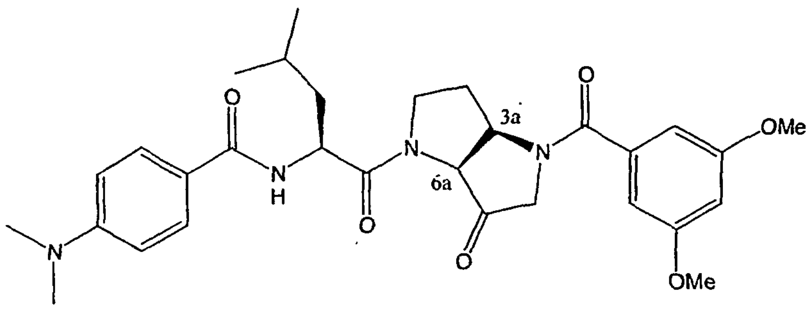

HPLC Rt = 14.43 mins (> 90%), HPLC-MS 551.2 $[M + H]^+$.

EXAMPLE 113. (3a*R*, 6a*S*)-4-Dimethylamino-*N*-{(1*S*)-1-[4-(3-fluoro-4-methylbenzoyl)-6-oxo-hexahydro-pyrrolo[3,2-*b*]pyrrole-1-carbonyl]-3-methyl-butyl}-benzamide

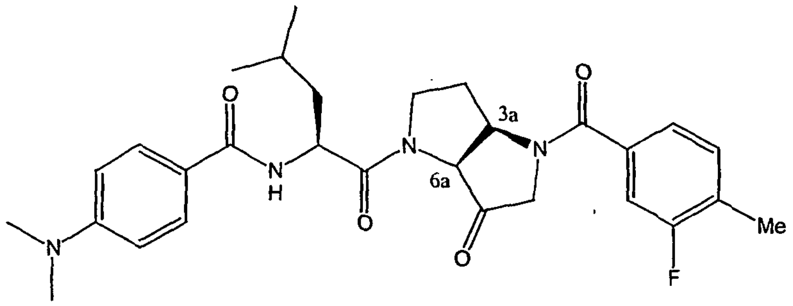

HPLC Rt = 15.04 mins (> 95%), HPLC-MS 523.2 $[M + H]^+$, 541.2 $[M + H + H_2O]^+$.

EXAMPLE 114. (3a*R*, 6a*S*)-4-Dimethylamino-*N*-{(1*S*)-1-[4-(3,4-dimethylbenzoyl)-6-oxo-hexahydro-pyrrolo[3,2-*b*]pyrrole-1-carbonyl]-3-methyl-butyl}-benzamide

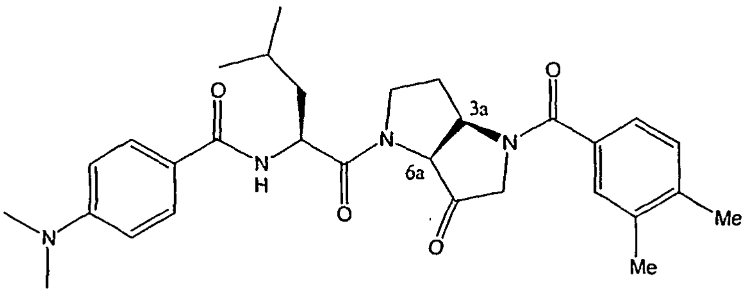

HPLC Rt = 15.70 mins (> 90%), HPLC-MS 519.3 [M + H]$^+$.

EXAMPLE 115. (3a*R*, 6a*S*)-4-Dimethylamino-*N*-{(1*S*)-1-[4-(4-fluoro-3-methylbenzoyl)-6-oxo-hexahydro-pyrrolo[3,2-*b*]pyrrole-1-carbonyl]-3-methyl-butyl}-benzamide

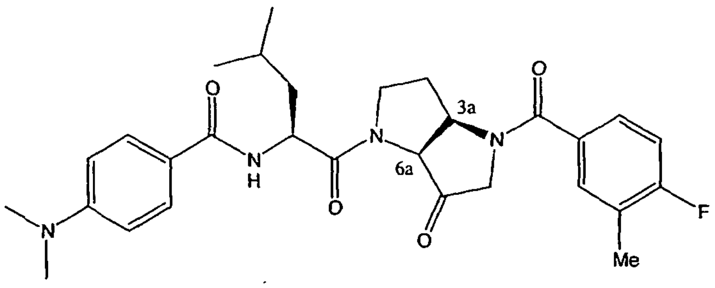

HPLC Rt = 15.00 mins (> 90%), HPLC-MS 523.2 [M + H]$^+$, 541.2 [M + H + $H_2O$]$^+$.

EXAMPLE 116. (3a*R*, 6a*S*)-*N*-{(1*S*)-1-[4-(3,4-Difluoro-benzoyl)-6-oxo-hexahydro-pyrrolo[3,2-*b*]pyrrole-1-carbonyl]-3-methyl-butyl}-4-dimethylamino-benzamide

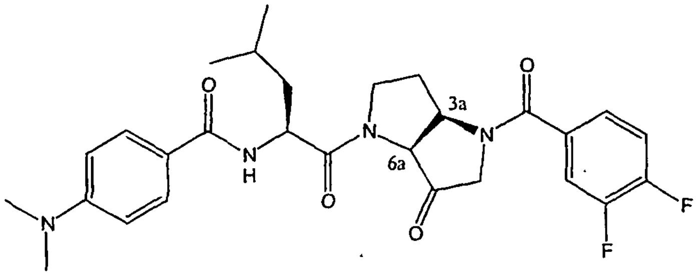

HPLC Rt = 14.47 mins (> 90%), HPLC-MS 527.2 [M + H]$^+$, 545.2 [M + H + $H_2O$]$^+$.

EXAMPLE 117. (3a*R*, 6a*S*)-*N*-[(1*S*)-1-(4-Benzoyl-6-oxo-hexahydro-pyrrolo[3,2-*b*] pyrrole-1-carbonyl)-3-methyl-butyl]-4-(*N*-oxy-dimethylamino)-benzamide

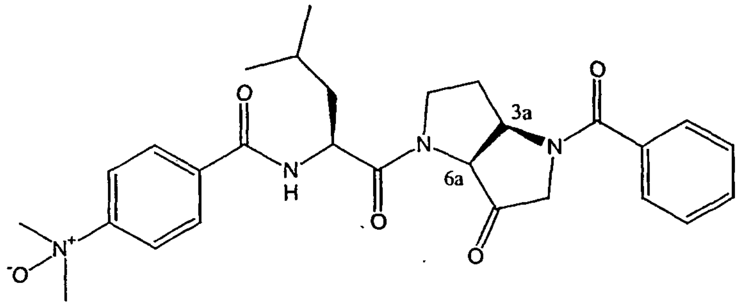

HPLC Rt = 11.64 mins (> 95%), HPLC-MS 507.2 $[M + H]^+$, 525.2 $[M + H + H_2O]^+$.

Oxidation of the intermediate was performed as detailed in the general soild phase methods prior to compound release from the solid phase.

EXAMPLE 118. (3a*R*, 6a*S*)-3-Aminomethyl-*N*-[(1*S*)-3-methyl-1-(6-oxo-4-phenylacetyl-hexahydro-pyrrolo[3,2-*b*]pyrrole-1-carbonyl)-butyl]-benzamide

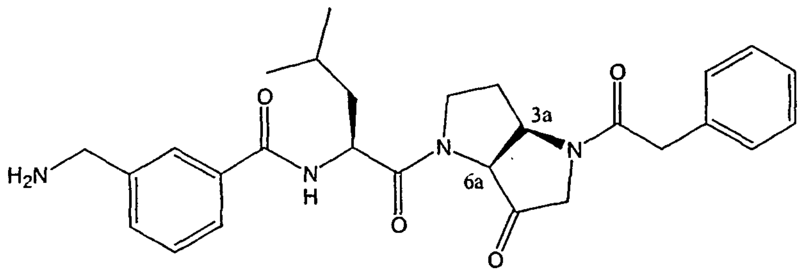

HPLC Rt = 12.06 mins (> 90%), HPLC-MS 491.2 $[M + H]^+$, 509.2 $[M + H + H_2O]^+$, 981.4 $[2M + H]^+$.

EXAMPLE 119. (3a*R*, 6a*S*)-*N*-[(1*S*)-1-(4-Benzenesulfonyl-6-oxo-hexahydro-pyrrolo[3,2-b]pyrrole-1-carbonyl)-3-methyl-butyl]-4-*tert*-butyl-benzamide

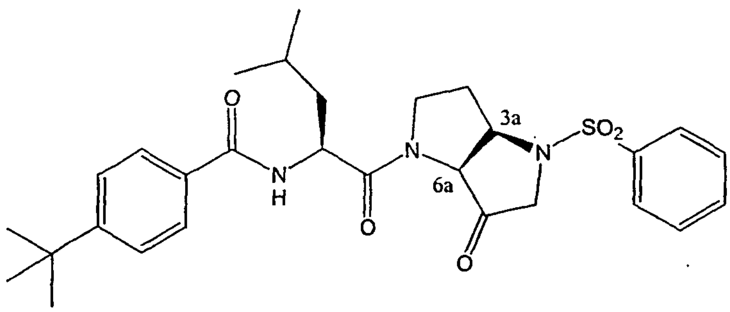

HPLC Rt = 21.0-22.4 mins (> 75%), HPLC-MS 540.1 $[M + H]^+$.

EXAMPLES 119-123 were prepared following the general methods detailed for EXAMPLE 1, but using an alternative building block (3a*R*, 6a*S*)-4-benzenesulfonyl-6-oxo-hexahydropyrrolo [3,2-*b*]pyrrole-1-carboxylic acid 9H-fluoren-9-ylmethyl ester **(84)** prepared following Scheme 21.

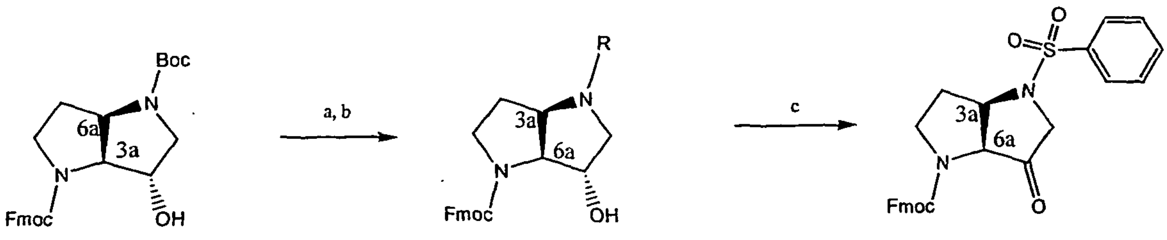


(81)

R = H (82)
R = SO2Ph (83)

(84)

**Scheme 21.** (a) 4N HCl in 1,4-dioxane, RT, 30mins, RT. (b) $PhSO_2Cl$, $Et_3N$, DCM. (c) Dess-Martin periodinane, DCM.

**Preparation of (3a*R*, 6*S*, 6a*S*)-6-hydroxyhexahydropyrrolo[3,2-*b*]pyrrole-1-carboxylic acid 9H-fluoren-9-ylmethyl ester hydrochloride (82)**

A solution of HCl in 1,4-dioxane (4.0M, 2.0 ml, 8 mmol) was added to (3*S*, 3a*S*, 6a*R*)-3-hydroxyhexahydropyrrolo[3,2-*b*]pyrrole-1,4-dicarboxylic acid 1-*tert*-butyl ester 4-(9H-fluoren-9-ylmethyl) ester **(81)** (65 mg, 0.14 mmol). The mixture was stirred in a sealed system for 50 minutes then the solvents were removed *in vacuo* to leave a residue which was azeotroped with diethyl ether (3x 10 ml) to obtain (3a*R*, 6*S*, 6a*S*)-6-hydroxyhexahydropyrrolo[3,2-*b*]pyrrole-1-carboxylic acid 9H-fluoren-9-ylmethyl ester hydrochloride **(82)** as a white solid which was used without further purification (see below). HPLC-MS 351.1 $[M + H]^+$, 373.1 $[M + Na]^+$, 723.2 $[2M + Na]^+$; HRMS $C_{21}H_{23}N_2O_3Na$ req. 351.1708, fnd. 351.1712 (0.95ppm).

**Preparation of (3aR, 6*S*, 6a*S*)-4-benzenesulfonyl-6-hydroxyhexahydropyrrolo [3,2-*b*]pyrrole-1-carboxylic acid 9H-fluoren-9-ylmethyl ester (83)**

Dichloromethane (1.5 ml), benzenesulfonyl chloride (20 µl, 0.16 mmol) then triethylamine (44 µl, 0.32 mmol) were added consecutively whilst stirring to (3a*R*, 6*S*, 6a*S*)-6-hydroxyhexahydropyrrolo[3,2-*b*]pyrrole-1-carboxylic acid 9H-fluoren-9-ylmethyl ester hydrochloride **(82)** (prepared as above, 0.14 mmol) under an atmosphere of argon. The mixture was stirred for I hour then the product extracted into ethyl acetate (40 ml), washed with aqueous saturated sodium hydrogen carbonate (40 ml), pH 3 hydrochloric acid (40 ml) and brine (40 ml) then dried ($Na_2SO_4$), and the solvents removed *in vacuo.* The residue was purified by flash chromatography over silica eluting with ethyl acetate : heptane mixtures 0 : 100 to 30 : 70 to give (3a*R*, 6*S*, 6a*S*)-4-benzenesulfonyl-6-hydroxyhexahydropyrrolo[3,2-*b*]pyrrole-1-carboxylic acid 9H-fluoren-9-ylmethyl ester **(83)** as a white solid (60 mg, 86%). TLC (Single spot, $R_f$ = 0.40, EtOAc : heptane 1 : 1), analytical HPLC single main peak $R_t$ = 20.112 min; HPLC-MS 491.0 $[M + H]^+$, 513.0 $[M + Na]^+$; Elemental analysis $C_{27}H_{26}N_2O_5S$ req.(fnd.) % C 66.10 (66.02), % H 5.34 (5.36), % N 5.71 (5.61); HRMS $C_{27}H_{26}N_2O_5SNa$ req. 513.1460, fnd. 513.1489 (5.56ppm).

**Preparation of (3a*R*, 6a*S*)-4-benzenesulfonyl-6-oxo-hexahydropyrrolo[3,2-*b*]pyrrole-1-carboxylic acid 9H-fluoren-9-ylmethyl ester (84)**

Dess-Martin periodinane (95 mg, 0.22 mmol) was added in portions to a stirred solution of (3a*R*, 6*S*, 6a*S*)-4-benzenesulfonyl-6-hydroxyhexahydropyrrolo[3,2-*b*]pyrrole-1-carboxylic acid 9H-fluoren-9-yl methyl ester **(83)** (55 mg, 0.11 mmol) in dichloromethane (1.5 ml) under an atmosphere of argon over 2 minutes. The mixture was stirred for 3.25 hours then the solvents removed *in vacuo* to obtain a residue which was purified by flash chromatography over silica eluting with ethyl acetate : heptane mixtures 0 : 100 to 25 : 75 to give (3a*R*, 6a*S*)-4-benzenesulfonyl-6-oxo-hexahydropyrrolo[3,2-*b*]pyrrole-1-carboxylic acid 9H-fluoren-9-ylmethyl ester **(84)** as a white solid (44 mg, 82%). TLC (Single spot, $R_f$ = 0.55, EtOAc : heptane 65 : 35), analytical HPLC broad peak $R_t$ = 20.0-21.5 min; HPLC-MS single broad main UV peak, 489.0 $[M + H]^+$, 511.0 $[M + Na]^+$, 529.0 $[M + H_2O + Na]^+$, 999.0 $[2M + Na]^+$; $C_{27}H_{24}N_2O_5S.0.4CDCl_3$ req.(fnd.) % C 61.36 (61.09), % H 4.51 (4.76), % N 5.22 (4.79); HRMS $C_{27}H_{24}N_2O_5SNa$ req. 511.1304, fnd. 511.1615 (2.16ppm); $d_H$ (500 MHz, $CDCl_3$) mixture of rotamers 2.10-2.28 (2H, m, $PhSO_2NCHCH_2$), 3.40-3.60 (2H, m, $FmocNCH_2$), 3.62-3.84 (2H, m, $PhSO_2NCH_2$), 4.16-4.46 (4H, m, FmocNC*H*, Fmoc-C*H* and Fmoc-$CH_2$), 4.48-4.61 (1H, m, $PhSO_2NCH$), 7.32-7.90 (13H, m, aromatic); $d_C$ (125 MHz, $CDCl_3$) 31.72, 31.86 ($PhSO_2NCH\underline{C}H_2$), 45.41 ($FmocN\underline{C}H_2$), 47.15 (Fmoc-$\underline{C}$H), 52.62 ($PhSO_2N\underline{C}H_2$), 60.17 ($PhSO_2N\underline{C}H$), 63.30, 63.52 (FmocN$\underline{C}$H), 67.79, 68.12 (Fmoc-$\underline{C}H_2$), 119.97, 120.09, 124.94, 127.07, 127.53, 127.74, 127.91, 129.65, 133.75 (aromatic $\underline{C}$H), 141.29, 143.40, 143.58, 143.81, 144.12 (quaternary aromatic), 154.93 (N$\underline{C}$=O), 203.85, 204.07 ($\underline{C}$=O).

Following the general details from Scheme 6, the required bicycle building block (3a*R*, 6a*S*)-4-benzenesulfonyl-6-oxo-hexahydropyrrolo[3,2-*b*]pyrrole-1-carboxylic acid 9H-fluoren-9-ylmethyl ester **(84)** was converted to building block-linker construct **(27)** (where $Pg_2$ is phenylsulphonyl) as follows:

A solution of sodium acetate trihydrate (30 mg, 0.221 mmol) in water (0.3 ml) was added to a solution of (3a*R*, 6a*S*)-4-benzenesulfonyl-6-oxo-hexahydropyrrolo [3,2-*b*]pyrrole-1-carboxylic acid 9H-fluoren-9-ylmethyl ester **(84)** (36 mg, 0.074mmol) and 4-[[(hydrazinocarbonyl)amino]methyl]cyclohexane carboxylic acid. trifluoroacetate (Murphy, A. M., et al, J. Am. Chem. Soc, 114, 3156-3157, 1992**)** (49 mg, 0.148 mmol) in ethanol (2.1 ml). The reaction heated at 75 °C in a sealed tube for 4.5 hour. The product was extracted into chloroform (50 ml) then washed with hydrochloric acid (0.1M, 2 x 25 ml), saturated aqueous sodium chloride solution (30 ml) then dried ($Na_2SO_4$) and the solvent removed *in vacuo* to leave the product as a white solid (46 mg, 91%). Analytical HPLC has main UV peaks with Rt = 19.624 and 21.252mins and HPLC-MS (main UV peaks each with 686.3 $[M+H]^+$).

Following the general details from Scheme 6, the required building block-linker construct **(27)** was attached to the solid phase providing loaded building block-linker construct **(28)** following standard loading protocols and indicated quantitative loading.

EXAMPLES 120 to 123 were prepared as detailed for EXAMPLE 119, substituting the appropriate carboxylic acids as required;

EXAMPLE 120. (3a*R*, 6a*S*)-*N*-[(1*S*)-1-(4-Benzenesulfonyl-6-oxo-hexahydropyrrolo [3,2-*b*]pyrrole-1-carbonyl)-3-methyl-butyl]-4-thiophen-2-yl-benzamide

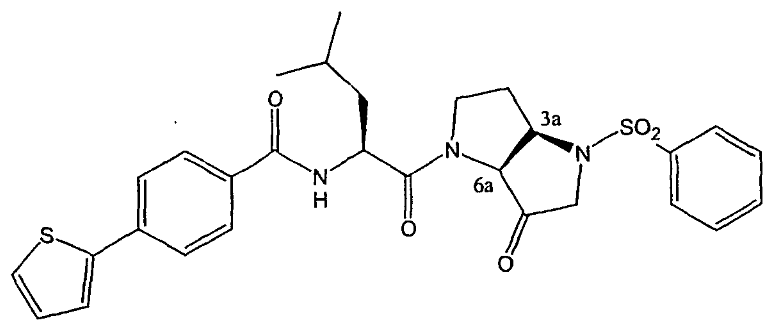

HPLC Rt = 20.3-21.7 mins (>75 %), HPLC-MS 566.1 $[M + H]^+$.

EXAMPLE 121. (3a*R*, 6a*S*)-Benzofuran-2-carboxylic acid [(1*S*)-1-(4-benzene sulfonyl-6-oxo-hexahydro-pyrrolo[3,2-*b*]pyrrole-1-carbonyl)-3-methyl-butyl]-amide

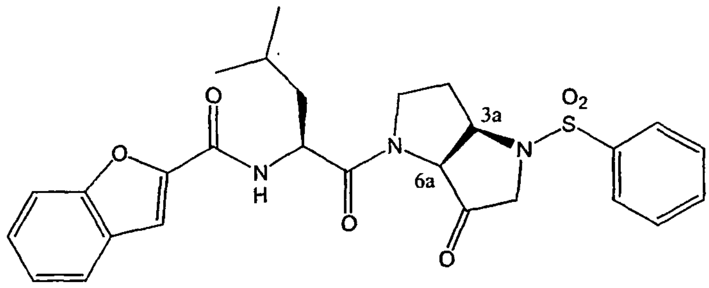

HPLC Rt = 18.3-19.9 mins (> 95%), HPLC-MS 524.1 [M + H]$^+$, 546.2 [M + Na]$^+$.

EXAMPLE 122. (3a*R*, 6a*S*)-*N*-[(1*S*)-1-(4-Benzenesulfonyl-6-oxo-hexahydropyrrolo [3,2-*b*]pyrrole-1-carbonyl)-3-methyl-butyl]-4-morpholin-4-yl-benzamide

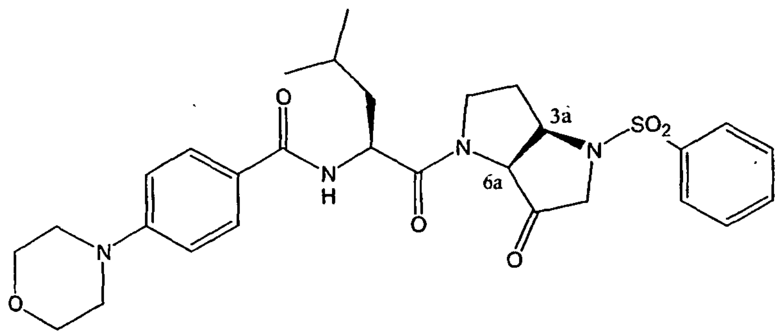

HPLC Rt = 16.4-17.8 mins (> 90%), HPLC-MS 569.1 [M + H]$^+$, 587.1 [M + H + $H_2O$]$^+$.

EXAMPLE 123. (3a*R*, 6a*S*)-3-Aminomethyl-*N*-[(1*S*)-1-(4-benzenesulfonyl-6-oxo-hexahydro-pyrrolo[3,2-*b*]pyrrole-1-carbonyl)-3-methyl-butyl]-benzamide

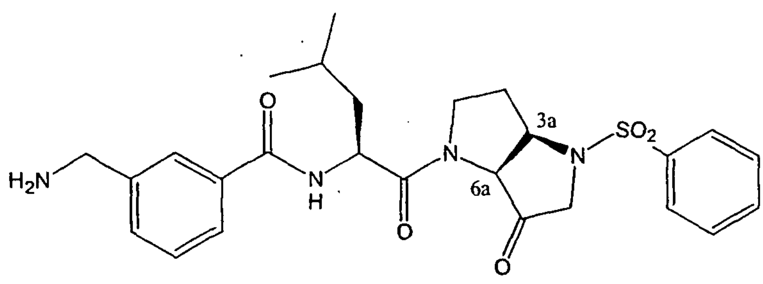

HPLC Rt = 13.0-14.1 mins (> 90%), HPLC-MS 513.1 [M + H]$^+$, 531.1 [M + H + $H_2O$]$^+$.

The following examples were prepared as detailed for EXAMPLE 1, substituting the appropriate carboxylic acids as required;

EXAMPLE 124. (3a*R*, 6a*S*)-4-Dimethylamino-*N*-{(1*S*)-3-methyl-1-[6-oxo-4-(3,3,3-trifluoro-propionyl)-hexahydro-pyrrolo[3,2-*b*]pyrrole-1-carbonyl]-butyl}-benzamide

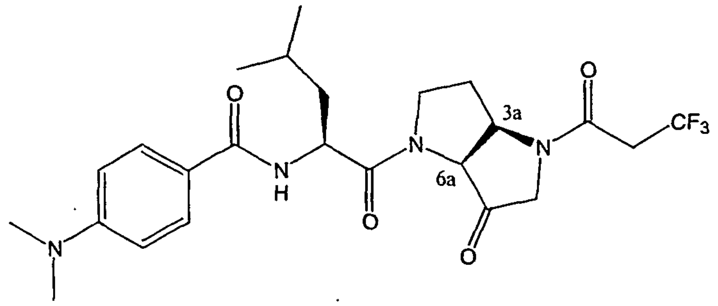

HPLC Rt = 10.46 mins (> 95%), HPLC-MS 497.2 $[M + H]^+$, 515.2 $[M + H + H_2O]^+$.

EXAMPLE 125. (3a*R*, 6a*S*)-*N*-{(1*S*)-1-[4-(2,2-Difluoro-acetyl)-6-oxo-hexahydro-pyrrolo[3,2-*b*]pyrrole-1-carbonyl]-3-methyl-butyl}-4-dimethylamino-benzamide

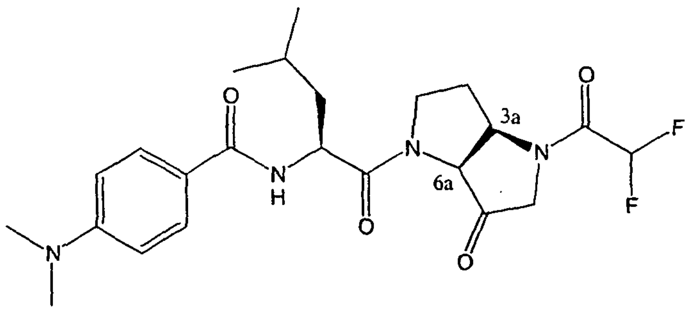

HPLC Rt = 9.31 mins (> 60%), HPLC-MS 465.1 $[M + H]^+$, 483.1 $[M + H + H_2O]^+$.

EXAMPLE 126. (3a*R*, 6a*S*)-4-Dimethylamino-*N*-[(1*S*)-3-methyl-1-(6-oxo-4-propionyl-hexahydro-pyrrolo[3,2-*b*]pyrrole-1-carbonyl)-butyl]-benzamide

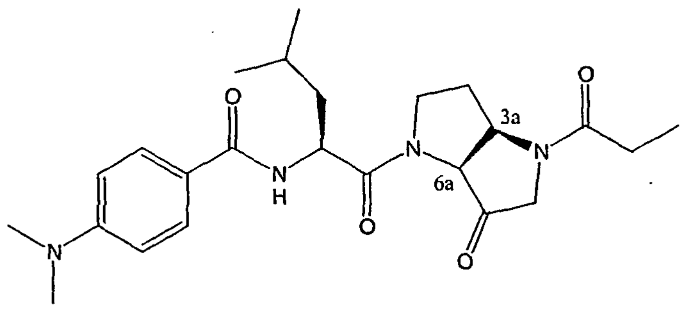

HPLC Rt = 9.90 mins (> 95%), HPLC-MS 443.2 $[M + H]^+$, 461.2 $[M + H + H_2O]^+$, 907.3 $[2M + Na]^+$.

EXAMPLE 127. (3a*R*, 6a*S*)-*N*-[(1*S*)-1-(4-Butyryl-6-oxo-hexahydro-pyrrolo[3,2-*b*] pyrrole-1-carbonyl)-3-methyl-butyl]-4-dimethylamino-benzamide

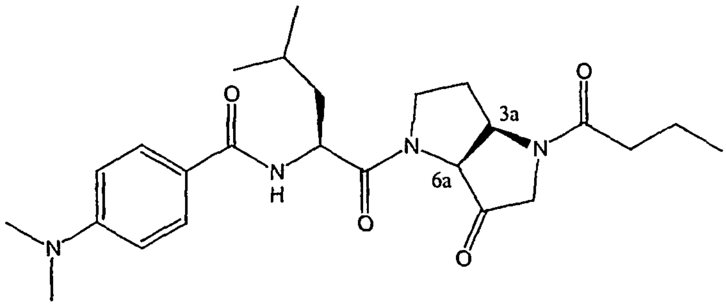

HPLC Rt = 10.95 mins (> 90%), HPLC-MS 457.2 $[M + H]^+$, 475.2 $[M + H + H_2O]^+$, 935.3 $[2M + Na]^+$.

EXAMPLE 128. (3a*R*, 6a*S*)-4-Dimethylamino-*N*-[(1*S*)-3-methyl-1-(6-oxo-4-pentanoyl-hexahydro-pyrrolo[3,2-*b*]pyrrole-1-carbonyl)-butyl]-benzamide

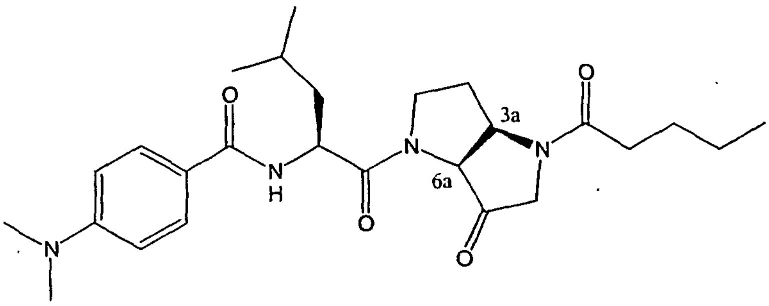

HPLC Rt = 12.0-13.1 mins (> 90%), HPLC-MS 471.2 $[M + H]^+$, 489.2 $[M + H + H_2O]^+$, 963.3 $[2M + Na]^+$.

EXAMPLE 129. (3a*R*, 6a*S*)-4-Dimethylamino-*N*-[(1*S*)-1-(4-isobutyryl-6-oxo-hexahydro-pyrrolo[3,2-*b*]pyrrole-1-carbonyl)-3-methyl-butyl]-benzamide

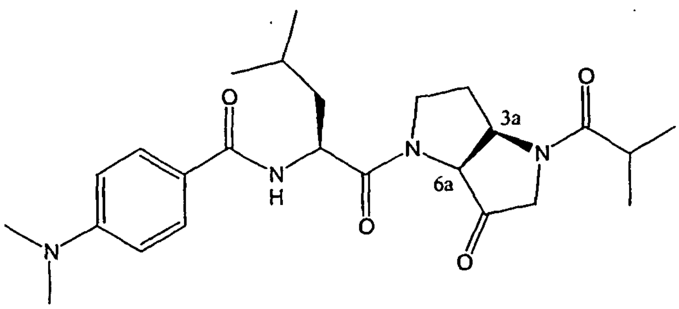

HPLC Rt = 10.55 mins (> 90%), HPLC-MS 457.2 [M + H]$^+$, 475.2 [M + H + $H_2O$]$^+$, 935.4 [2M + Na]$^+$.

EXAMPLE 130. (3a*R*, 6a*S*)-4-Dimethylamino-*N*-{(1*S*)-3-methyl-1-[4-(3-methylbutyryl)-6-oxo-hexahydro-pyrrolo[3,2-*b*]pyrrole-1-carbonyl]-butyl}-benzamide

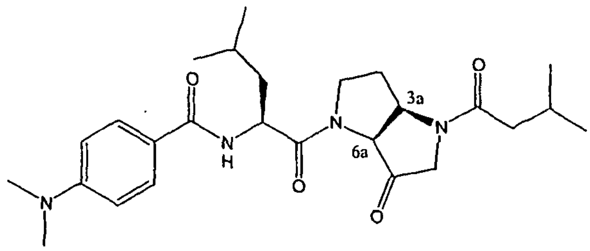

HPLC Rt = 11.81 mins (> 90%), HPLC-MS 471.2 [M + H]$^+$, 489.2 [M + H + $H_2O$]$^+$, 963.4 [2M + Na]$^+$.

EXAMPLE 131. (3a*R*, 6a*S*)-*N*-[(1*S*)-1-(4-Cyclopropanecarbonyl-6-oxo-hexahydro-pyrrolo[3,2-*b*]pyrrole-1-carbonyl)-3-methyl-butyl]-4-dimethylamino-benzamide

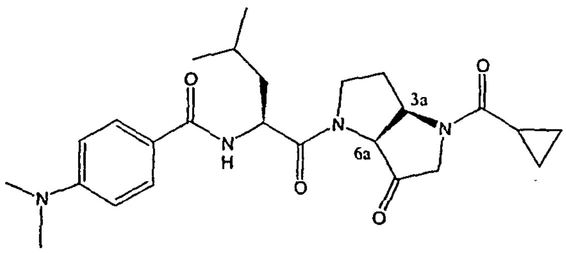

HPLC Rt = 10.10 mins (> 90%), HPLC-MS 455.2 [M + H]$^+$, 473.2 [M + H + $H_2O$]$^+$, 931.3 [2M + Na]$^+$.

EXAMPLE 132. (3a*R*, 6a*S*)-*N*-{(1*S*)-1-[4-(2-Cyclopropyl-acetyl)-6-oxo-hexahydro-pyrrolo[3,2-*b*]pyrrole-1-carbonyl]-3-methyl-butyl}-4-dimethylamino-benzamide

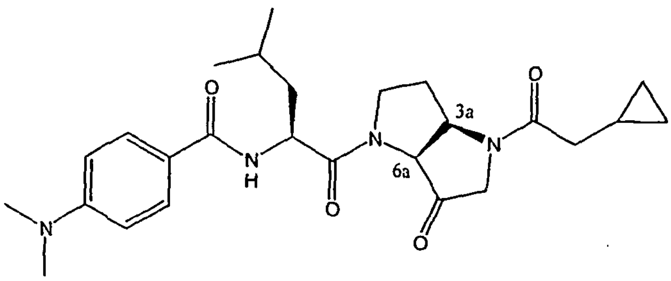

HPLC Rt = 11.13 mins (> 90%), HPLC-MS 469.2 [M + H]$^+$, 487.2 [M + H + $H_2O$]$^+$, 959.3 [2M + Na]$^+$.

EXAMPLE 133. (3a*R*, 6a*S*)-*N*-[(1*S*)-1-(4-Cyclobutanecarbonyl-6-oxo-hexahydro-pyrrolo[3,2-*b*]pyrrole-1-carbonyl)-3-methyl-butyl]-4-dimethylamino-benzamide

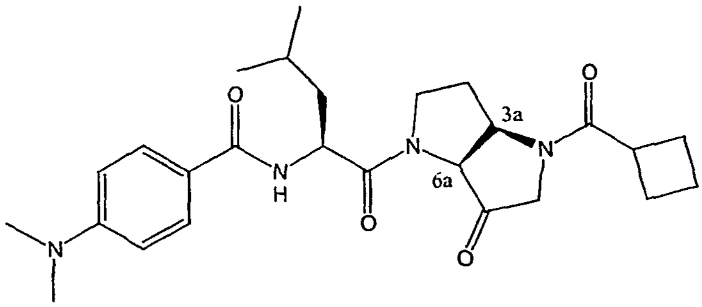

HPLC Rt = 11.41 mins (> 90%), HPLC-MS 469.2 $[M + H]^+$, 487.2 $[M + H + H_2O]^+$, 959.4 $[2M + Na]^+$.

EXAMPLE 134. (3a*R*, 6a*S*)-*N*-[(1*S*)-1-(4-Cyclopentanecarbonyl-6-oxo-hexahydro-pyrrolo[3,2-*b*]pyrrole-1-carbonyl)-3-methyl-butyl]-4-dimethylamino-benzamide

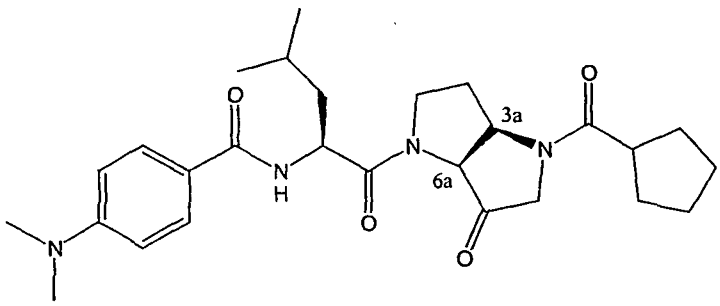

HPLC Rt = 12.2-13.1 mins (> 90%), HPLC-MS 483.2 $[M + H]^+$, 501.2 $[M + H + H_2O]^+$, 987.4 $[2M + Na]^+$.

EXAMPLE 135. (3a*R*, 6a*S*)-*N*-[(1*S*)-1-(4-Cycloheptanecarbonyl-6-oxo-hexahydro-pyrrolo[3,2-*b*]pyrrole-1-carbonyl)-3-methyl-butyl]-4-dimethylamino-benzamide

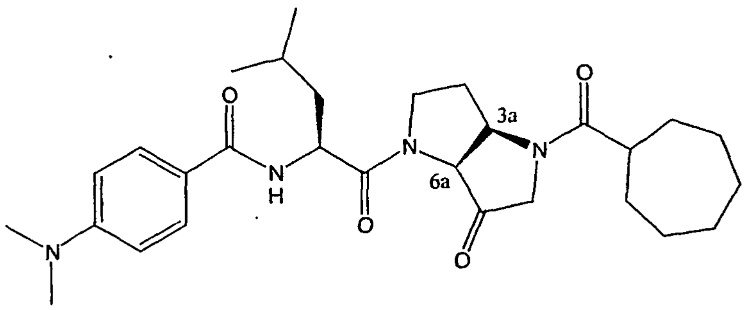

HPLC Rt = 14.0-14.9 mins (> 85%), HPLC-MS 511.2 $[M + H]^+$, 529.3 $[M + H + H_2O]^+$.

EXAMPLE 136. (3a*R*, 6a*S*)-4-Dimethylamino-*N*-[(1*S*)-1-(4-heptanoyl-6-oxo-hexahydro-pyrrolo[3,2-*b*]pyrrole-1-carbonyl)-3-methyl-butyl]-benzamide

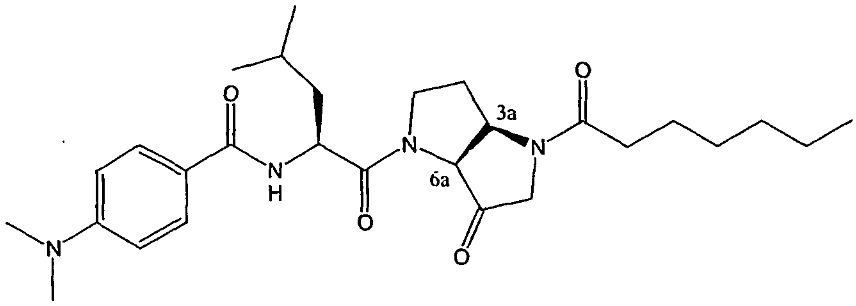

HPLC Rt = 14.3-15.2 mins (> 80%), HPLC-MS 499.2 $[M + H]^+$, 517.2 $[M + H + H_2O]^+$.

EXAMPLE 137. (3a*R*, 6a*S*)-*N*-{(1*S*)-1-[4-(2-Cyclohexyl-acetyl)-6-oxo-hexahydro-pyrrolo[3,2-*b*]pyrrole-1-carbonyl]-3-methyl-butyl}-4-dimethylamino-benzamide

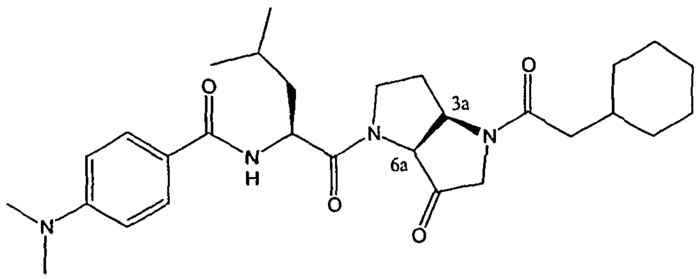

HPLC Rt = 14.27 mins (> 80%), HPLC-MS 511.2 $[M + H]^+$, 529.3 $[M + H + H_2O]^+$.

EXAMPLE 138. (3a*R*, 6a*S*)-*N*-{(1*S*)-1-[4-(Cyclohex-3-enecarbonyl)-6-oxo-hexahydro-pyrrolo[3,2-*b*]pyrrole-1-carbonyl]-3-methyl-butyl}-4-dimethylamino-benzamide

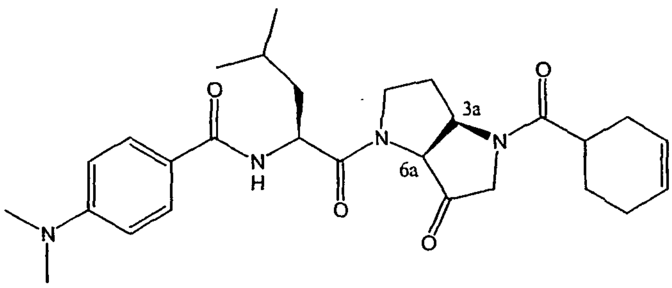

HPLC Rt = 12.2-13.3 mins (> 80%), HPLC-MS 495.2 $[M + H]^+$, 513.2 $[M + H + H_2O]^+$.

EXAMPLE 139. (3a*R*, 6a*S*)-4-Dimethylamino-*N*-{(1*S*)-1-[4-(3-methoxy-cyclohexanecarbonyl)-6-oxo-hexahydro-pyrrolo[3,2-*b*]pyrrole-1-carbonyl]-3-methyl-butyl}-benzamide

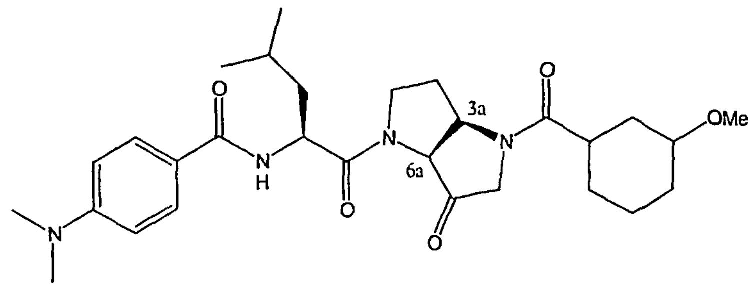

HPLC Rt = 11.1-12.8 mins (> 90%), HPLC-MS 527.2 $[M + H]^+$, 545.3 $[M + H + H_2O]^+$.

EXAMPLE 140. (3a*R*, 6a*S*)-4-Dimethylamino-*N*-{(1*S*)-3-methyl-1-[6-oxo-4-(piperidine-3-carbonyl)-hexahydro-pyrrolo[3,2-*b*]pyrrole-1-carbonyl]-butyl}-benzamide

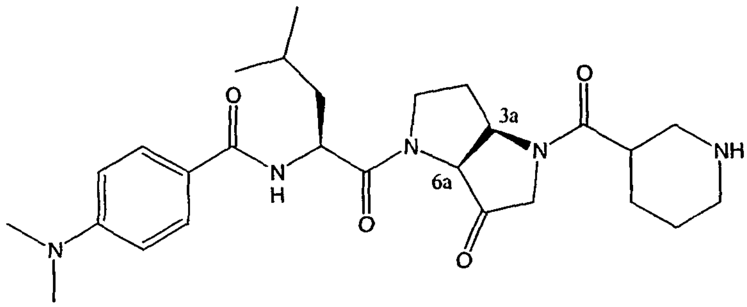

HPLC Rt = 7.8-9.3 mins (> 75%), HPLC-MS 498.2 $[M + H]^+$, 516.2 $[M + H + H_2O]^+$, 995.5 $[2M + H]^+$.

EXAMPLE 141. (3a*R*, 6a*S*)-4-Dimethylamino-*N*-{(1*S*)-3-methyl-1-[4-(3-methylpentanoyl)-6-oxo-hexahydro-pyrrolo[3,2-*b*]pyrrole-1-carbonyl]-butyl}-benzamide

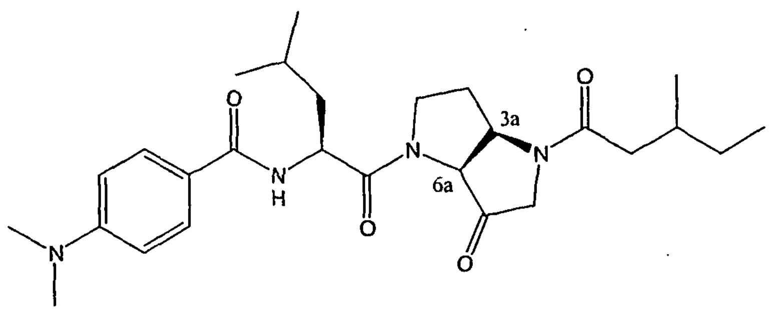

HPLC Rt = 12.9-13.8 mins (> 75%), HPLC-MS 485.2 [M + H]$^+$, 503.3 [M + H + $H_2O$]$^+$, 991.5 [2M + Na]$^+$.

EXAMPLE 142. (3a*R*, 6a*S*)-4-Dimethylamino-*N*-{(1*S*)-3-methyl-1-[4-(morpholine-4-carbonyl)-6-oxo-hexahydro-pyrrolo[3,2-*b*]pyrrole-1-carbonyl]-butyl}-benzamide

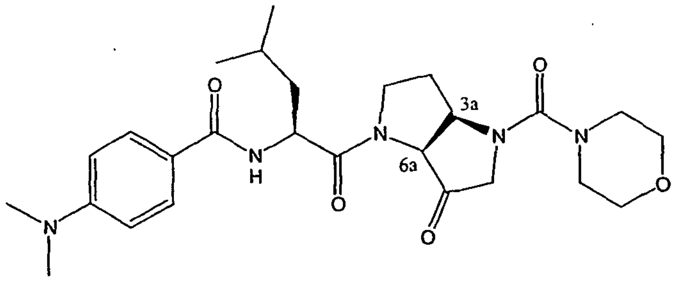

HPLC Rt = 9.82 mins (> 85%), HPLC-MS 500.2 [M + H]$^+$.

EXAMPLE 143. (3a*R*, 6a*S*)-*N*-{(1*S*)-1-[4-(3-Benzenesulfonyl-propionyl)-6-oxo-hexahydro-pyrrolo[3,2-*b*]pyrrole-1-carbonyl]-3-methyl-butyl}-4-dimethylamino-benzamide

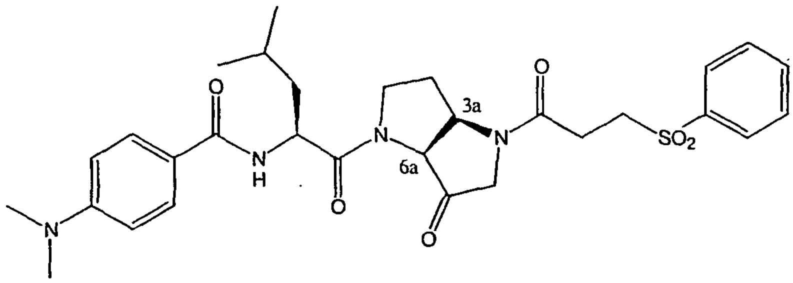

HPLC Rt = 12.27 mins (> 90%), HPLC-MS 583.2 [M + H]$^+$.

EXAMPLE 144. (3a*R*, 6a*S*)-4-Dimethylamino-*N*-{(1*S*)-1-[4-(2-methanesulfonyl-acetyl)-6-oxo-hexahydro-pyrrolo[3,2-*b*]pyrrole-1-carbonyl]-3-methyl-butyl}-benzamide

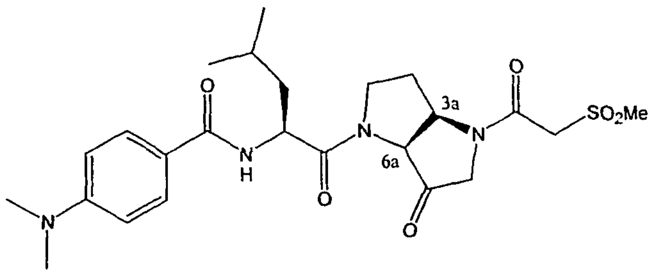

HPLC Rt = 8.00 mins (> 50%), HPLC-MS 507.1 [M + H]+.

EXAMPLE 145. (3a*S*, 6a*R*)-((1*S*)-2-{4-[(2*S*)-2-(4-Dimethylamino-benzoylamino)-4-methyl-pentanoyl]-3-oxo-hexahydro-pyrrolo[3,2-*b*]pyrrol-1-yl}-1-methyl-2-oxo-ethyl)-carbamic acid benzyl ester

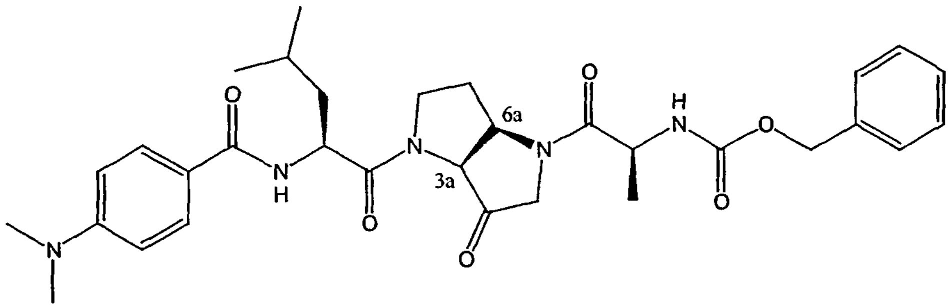

HPLC Rt = 12.4-14.0 mins (> 85%), HPLC-MS 592.2 [M + H]+.

EXAMPLE 146. (3a*S*, 6a*R*)-((1*R*)-2-{4-[(2*S*)-2-(4-Dimethylamino-benzoylamino)-4-methyl-pentanoyl]-3-oxo-hexahydro-pyrrolo[3,2-*b*]pyrrol-1-yl}-1-methyl-2-oxo-ethyl)-carbamic acid benzyl ester

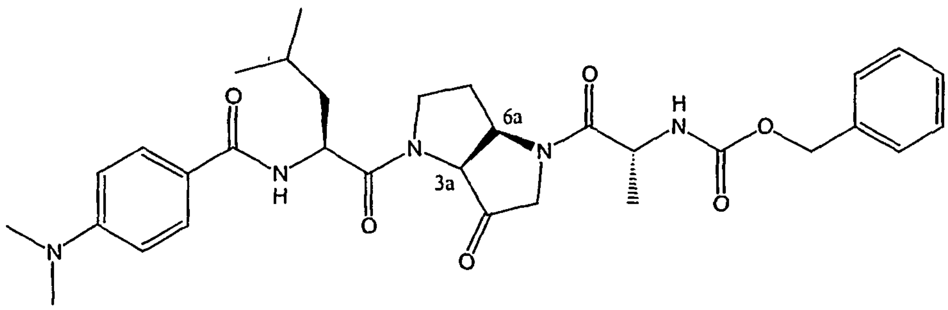

HPLC Rt = 12.5-13.9 mins (> 75%), HPLC-MS 592.2 [M + H]+.

EXAMPLE 147. (3a*S*, 6a*R*)-((1*S*)-2-{4-[(2*S*)-2-(4-Dimethylamino-benzoylamino)-4-methyl-pentanoyl]-3-oxo-hexahydro-pyrrolo[3,2-*b*]pyrrol-1-yl}-1-hydroxymethyl-2-oxo-ethyl)-carbamic acid benzyl ester

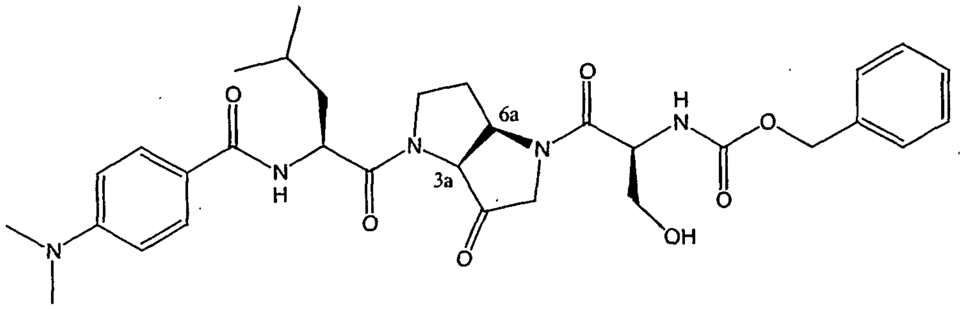

HPLC Rt = 11.8-13.3 mins (> 80%), HPLC-MS 608.3 $[M + H]^+$.

EXAMPLE 148. (3a*S*, 6a*R*)-((1*S*)-1-Carbamoylmethyl-2-{4-[(2*S*)-2-(4-dimethyl aminobenzoylamino)-4-methyl-pentanoyl]-3-oxo-hexahydro-pyrrolo[3,2-*b*] pyrrol-1-yl}-2-oxo-ethyl)-carbamic acid benzyl ester

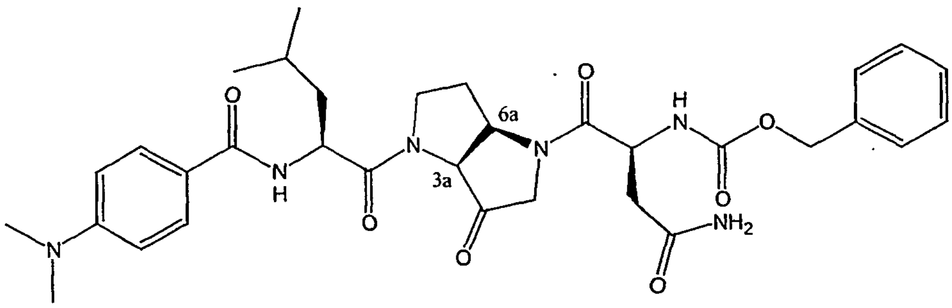

HPLC Rt = 11.4-13.2 mins (> 75%), HPLC-MS 635.2 $[M + H]^+$.

EXAMPLE 149. (3a*S*, 6a*R*)-(3*S*)-3-Benzyloxycarbonylamino-4-{4-[(2*S*)-2-(4-dimethylamino-benzoylamino)-4-methyl-pentanoyl]-3-oxo-hexahydro-pyrrolo[3,2-*b*] pyrrol-1-yl}-4-oxo-butyric acid

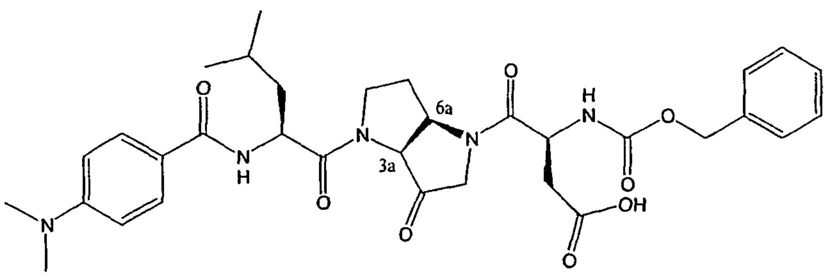

HPLC Rt = 12.1-13.5 mins (> 85%), HPLC-MS 636.2 $[M + H]^+$, 654.3 $[M + H + H_2O]^+$.

EXAMPLE 150. (3a*R*, 6a*S*)-*N*-{(1*S*)-1-[4-((2*S*)-2-Amino-4-methyl-pentanoyl)-6-oxo-hexahydro-pyrrolo[3,2-*b*]pyrrole-1-carbonyl]-3-methyl-butyl}-4-dimethylamino-benzamide

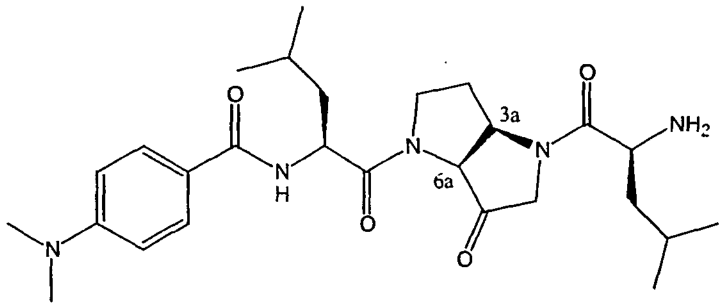

HPLC Rt = 11.00 mins (> 90%), HPLC-MS 500.2 $[M + H]^+$, 999.5 $[2M + H]^+$.

EXAMPLE 151. (3a*R*, 6a*S*)-*N*-{(1*S*)-1-[4-((2*S*)-2-Acetylamino-4-methylpentanoyl)-6-oxo-hexahydro-pyrrolo[3,2-*b*]pyrrole-1-carbonyl]-3-methyl-butyl}-4-dimethylamino-benzamide

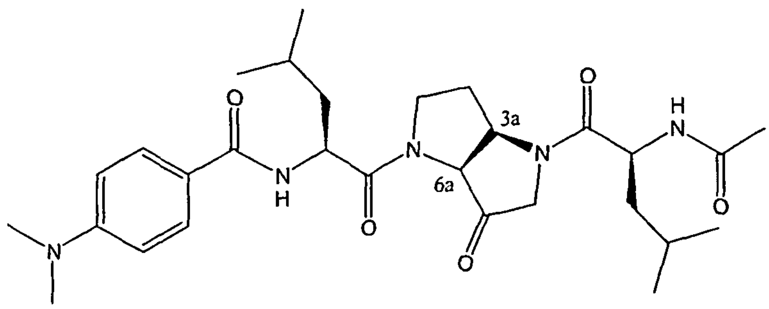

HPLC Rt = 11.5-13.0 mins (> 90%), HPLC-MS 542.2 $[M + H]^+$.

EXAMPLE 152. (3a*R*, 6a*S*)-*N*-{(1*S*)-1-[4-((2*R*)-2-Amino-4-methyl-pentanoyl)-6-oxo-hexahydro-pyrrolo[3,2-*b*]pyrrole-1-carbonyl]-3-methyl-butyl}-4-dimethylamino-benzamide

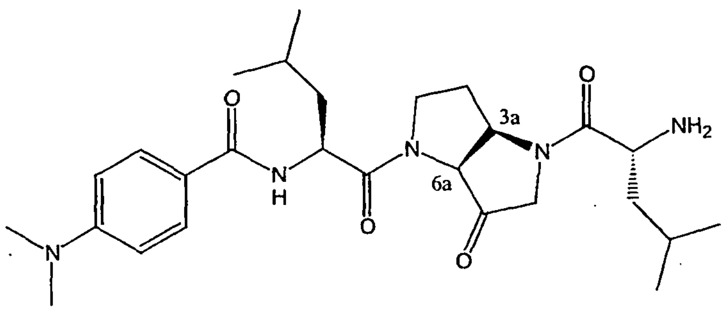

HPLC Rt = 8.9-10.8 mins (> 90%), HPLC-MS 500.2 $[M + H]^+$, 999.5 $[2M + H]^+$.

EXAMPLE 153. (3a*R*, 6a*S*)-*N*-{(1*S*)-1-[4-((2*R*)-2-Acetylamino-4-methylpentanoyl)-6-oxo-hexahydro-pyrrolo[3,2-*b*]pyrrole-1-carbonyl]-3-methyl-butyl}-4-dimethylamino-benzamide

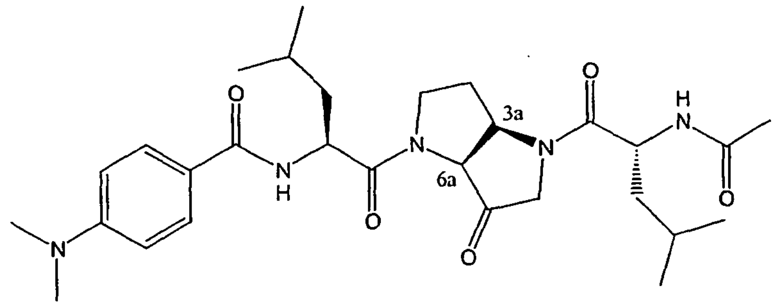

HPLC Rt = 10.4-12.2 mins (> 85%), HPLC-MS 542.3 $[M + H]^+$.

EXAMPLE 154. (3a*S*, 6a*R*)-(2*S*)-2-{4-[(2*S*)-2-(4-Dimethylamino-benzoylamino)-4-methyl-pentanoyl]-3-oxo-hexahydro-pyrrolo[3,2-*b*]pyrrole-1-carbonyl}-pyrrolidine-1-carboxylic acid benzyl ester

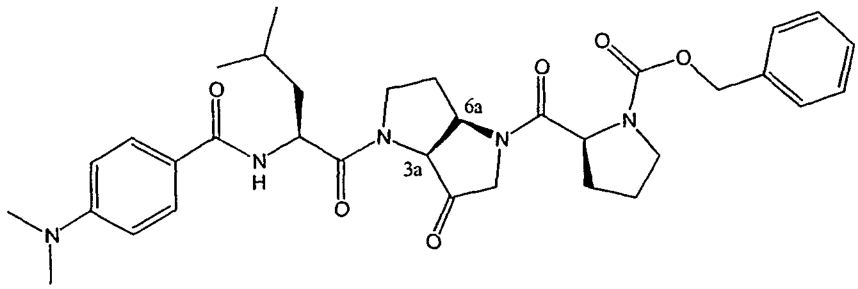

HPLC Rt = 13.2-14.3 mins (> 90%), HPLC-MS 618.2 $[M + H]^+$.

EXAMPLE 155. (3a*S*, 6a*R*)-(2*R*)-2-{4-[(2*S*)-2-(4-Dimethylamino-benzoylamino)-4-methyl-pentanoyl]-3-oxo-hexa- hydro-pyrrolo[3,2-*b*]pyrrole-1-carbonyl}-pyrrolidine-1-carboxylic acid benzyl ester

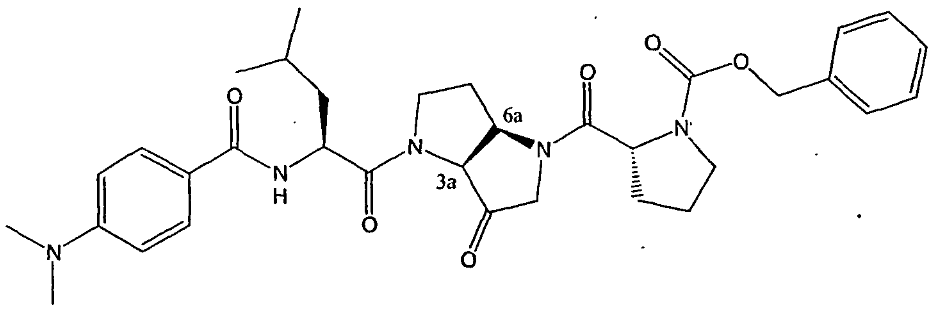

HPLC Rt = 13.2-14.2 mins (> 90%), HPLC-MS 618.2 $[M + H]^+$.

EXAMPLE 156. (3a*R*, 6a*S*)-4-Dimethylamino-*N*-{(1*S*)-3-methyl-1-[6-oxo-4-((2*S*)-pyrrolidine-2-carbonyl)-hexahydro-pyrrolo[3,2-*b*]pyrrole-1-carbonyl]-butyl}-benzamide

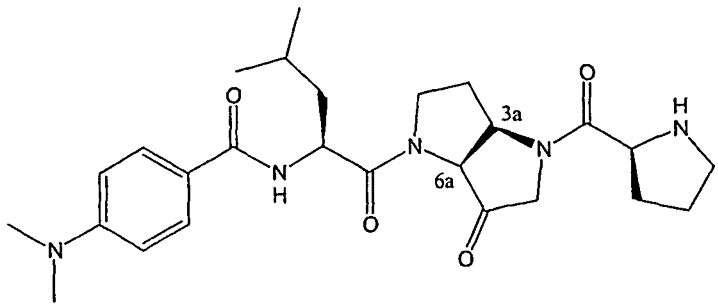

HPLC Rt = 8.82 mins (> 85%), HPLC-MS 484.2 $[M + H]^+$, 967.4 $[2M + H]^+$.

EXAMPLE 157. (3a*R*, 6a*S*)-4-Dimethylamino-*N*-{(1*S*)-3-methyl-1-[6-oxo-4-((2*R*)-pyrrolidine-2-carbonyl)-hexahydro-pyrrolo[3,2-*b*]pyrrole-1-carbonyl]-butyl-benzamide

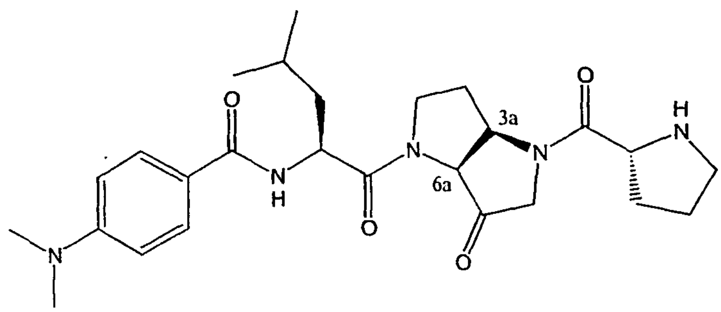

HPLC Rt = 7.3-9.1 mins (> 85%), HPLC-MS 484.2 $[M + H]^+$, 502.2 $[M + H + H_2O]^+$, 985.4 $[2M + H + H_2O]^+$.

EXAMPLE 158. (3a*S*, 6a*R*)-((1*S*)-1-{4-[(2*S*)-2-(4-Dimethylamino-benzoylamino)-4-methyl-pentanoyl]-3-oxo-hexahydro-pyrrolo[3,2-*b*]pyrrole-1-carbonyl}-2-methyl-propyl)-carbamic acid benzyl ester

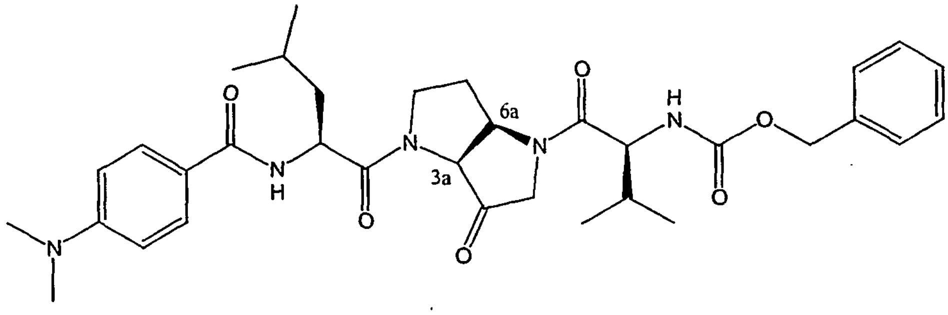

HPLC Rt = 15.18 mins (> 85%), HPLC-MS 620.3 [M + H]$^+$.

EXAMPLE 159. (3a*S*, 6a*R*)-((1*S*)-1-{4-[(2*S*)-2-(4-Dimethylamino-benzoylamino)-4-methyl-pentanoyl]-3-oxo-hexahydro-pyrrolo[3,2-*b*]pyrrole-1-carbonyl}-propyl)-carbamic acid benzyl ester

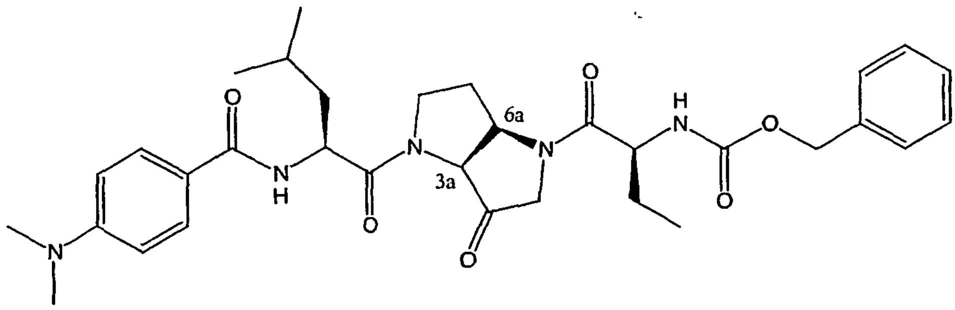

HPLC Rt = 14.22 mins (> 85%), HPLC-MS 606.2 [M + H]$^+$.

EXAMPLE 160. (3a*S*, 6a*R*)-((1*S*)-1-Benzyl-2-{4-[(2*S*)-2-(4-dimethylamino-benzoylamino)-4-methyl-pentanoyl]-3-oxo-hexahydro-pyrrolo[3,2-*b*]pyrrol-1-yl}-2-oxo-ethyl)-carbamic acid benzyl ester

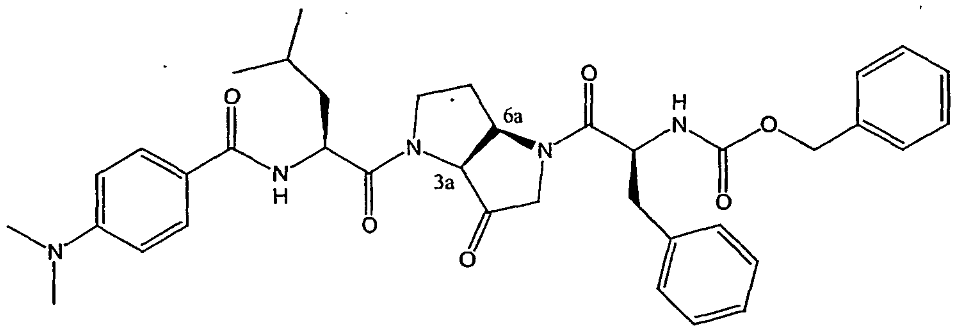

HPLC Rt = 16.23 mins (> 80%), HPLC-MS 668.2 [M + H]$^+$.

EXAMPLE 161. (3a*S*, 6a*R*)-((1*S*, 2*S*)-1-{4-[(2*S*)-2-(4-Dimethylamino-benzoylamino)-4-methyl-pentanoyl]-3-oxo-hexahydro-pyrrolo[3,2-*b*]pyrrole-1-carbonyl}-2-methyl-butyl)-carbamic acid benzyl ester

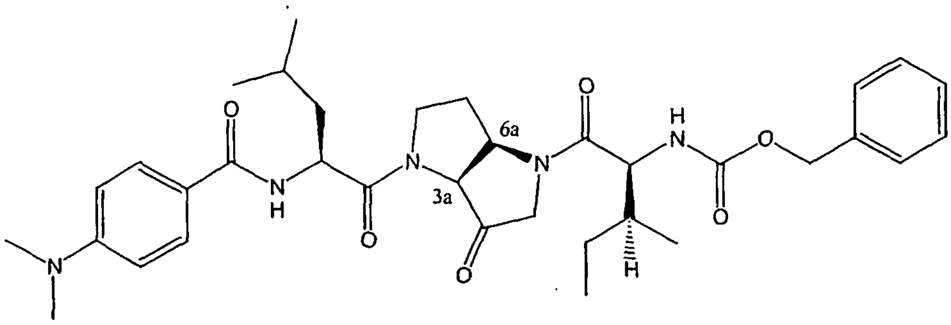

HPLC Rt = 16.17 mins (> 90%), HPLC-MS 634.3 [M + H]$^+$.

EXAMPLE 162. (3a*R*, 6a*S*)-*N*-{(1*S*)-1-[4-((2*S*)-2-Amino-propionyl)-6-oxo-hexahydro-pyrrolo[3,2-*b*]pyrrole-1-carbonyl]-3-methyl-butyl}-4-dimethylamino-benzamide

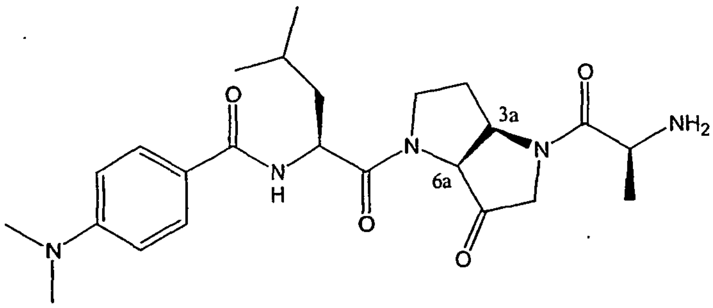

HPLC Rt = 8.01 mins (> 90%), HPLC-MS 458.2 [M + H]$^+$, 937.4 [2M + Na]$^+$.

EXAMPLE 163. (3a*R*, 6a*S*)-*N*-{(1*S*)-1-[4-((2*R*)-2-Amino-propionyl)-6-oxo-hexahydro-pyrrolo[3,2-*b*]pyrrole-1-carbonyl]-3-methyl-butyl}-4-dimethylamino-benzamide

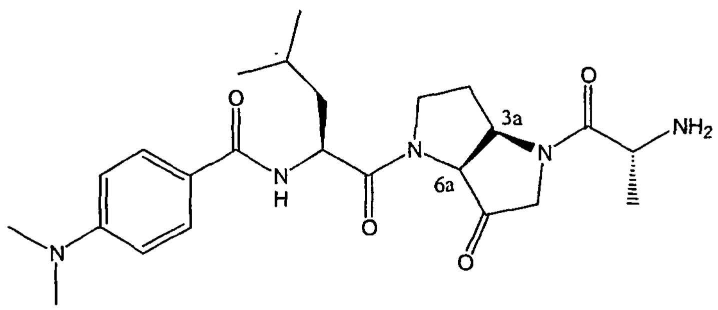

HPLC Rt = 6.9-8.3 mins (> 90%), HPLC-MS 458.2 [M + H]$^+$, 937.4 [2M + Na]$^+$.

EXAMPLE 164. (3a*R*, 6a*S*)-4-Dimethylamino-*N*-{(1*S*)-3-methyl-1-[6-oxo-4-(thiophene-3-carbonyl)-hexahydro-pyrrolo-[3,2-*b*]pyrrole-1-carbonyl]-butyl}-benzamide

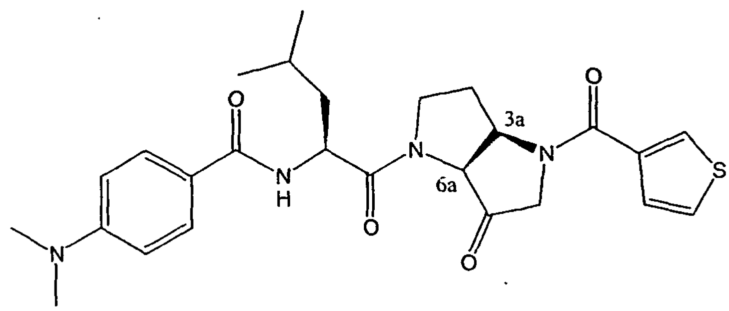

HPLC Rt = 11.0-12.0 mins (> 90%), HPLC-MS 497.2 [M + H]$^+$, 515.2 [M + H + $H_2O$]$^+$.

EXAMPLE 165. (3a*R*, 6a*S*)-*N*-{(1*S*)-1-[4-(3-Acetylamino-thiophene-2-carbonyl)-6-oxo-hexahydro-pyrrolo[3,2-*b*]pyrrole-1-carbonyl]-3-methyl-butyl}-4-dimethylamino-benzamide

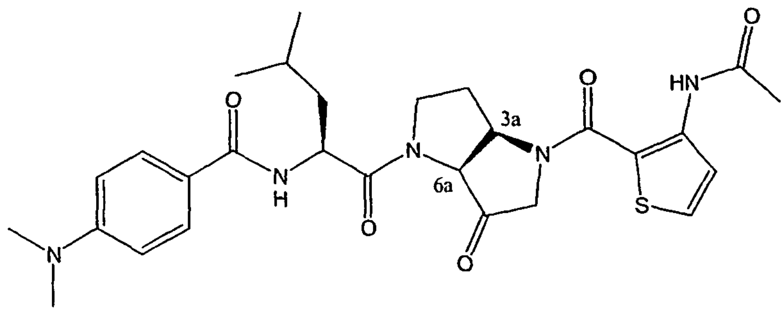

HPLC Rt = 10.94 mins (> 80%), HPLC-MS 554.2 [M + H]$^+$, 572.2 [M + H + $H_2O$]$^+$.

EXAMPLE 166. (3a*R*, 6a*S*)-*N*-{(1*S*-1-[4-(5-Chloro-thiophene-2-carbonyl)-6-oxo-hexahydro-pyrrolo[3,2-*b*]pyrrole-1-carbonyl]-3-methyl-butyl}-4-dimethylamino-benzamide

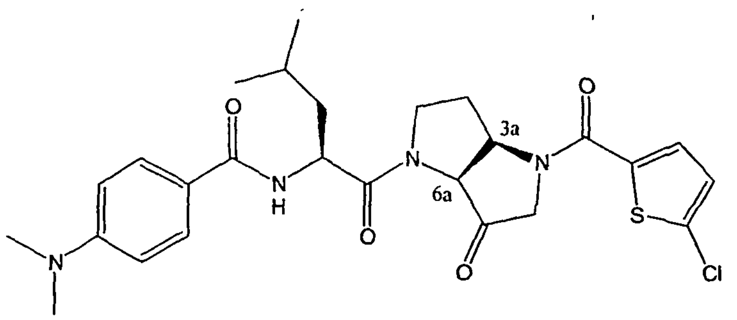

HPLC Rt = 13.83 mins (> 95%), HPLC-MS 531.1 / 533.1 $[M + H]^+$, 549.1 / 551.1 $[M + H + H_2O]^+$.

EXAMPLE 167. (3a*S*, 6a*R*)-4-[(2*S*)-2-(4-Dimethylamino-benzoylamino)-4-methyl-pentanoyl]-3-oxo-hexahydro-pyrrolo[3,2-*b*]pyrrole-1-carboxylic acid phenylamide

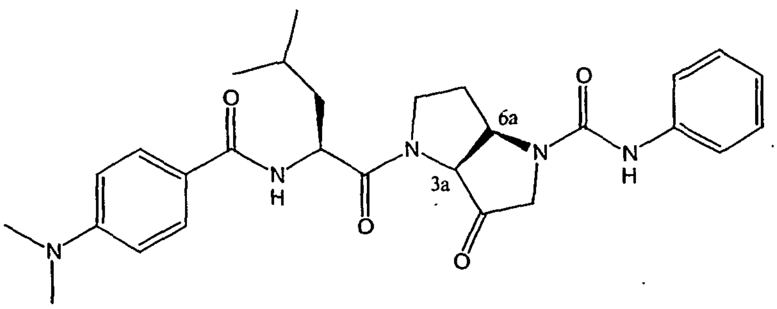

HPLC Rt = 11.4-12.1 mins (> 95%), HPLC-MS 506.2 $[M+ H]^+$.

EXAMPLE 168. (3a*R*, 6a*S*)-4-Dimethylamino-*N*-{(1*S*)-3-methyl-1-[6-oxo-4-(2-trifluoromethoxy-benzoyl)-hexahydro-pyrrolo[3,2-*b*]pyrrole-1-carbonyl]-butyl}-benzamide

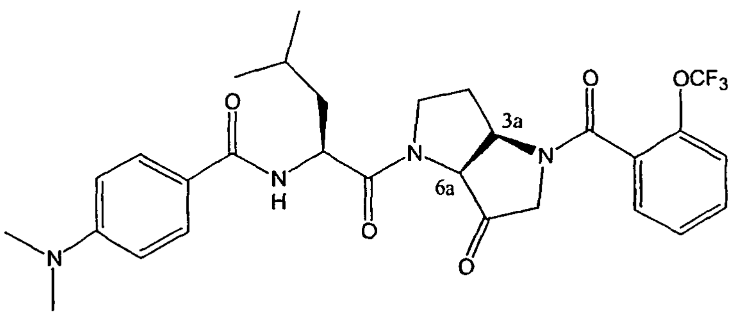

HPLC Rt = 14.42 mins (> 85%), HPLC-MS 575.2 [M + H]$^+$, 593.2 [M + H + $H_2O$]$^+$.

EXAMPLE 169. (3a*R*, 6a*S*)-4-Dimethylamino-*N*-{(1*S*)-1-[4-(2-hydroxy-benzoyl)-6-oxo-hexahydro-pyrrolo[3,2-*b*]-pyrrole-1-carbonyl]-3-methyl-butyl}-benzamide

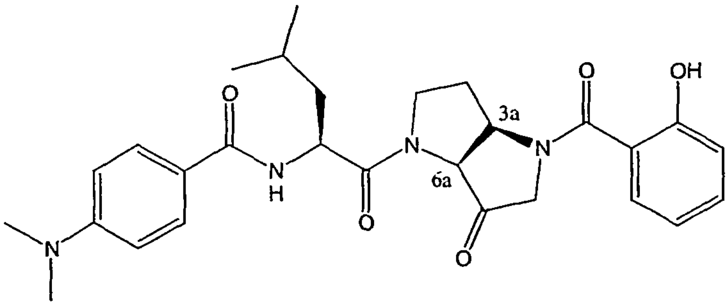

HPLC Rt = 7.77 mins (> 50%), HPLC-MS 507.2 [M + H]$^+$.

EXAMPLE 170. (3a*R*, 6a*S*)-*N*-[(1*S*)-1-(4-Benzoyl-6-oxo-hexahydro-pyrrolo[3,2-*b*] pyrrole-1-carbonyl)-3-methyl-butyl]-4-methyl-benzamide

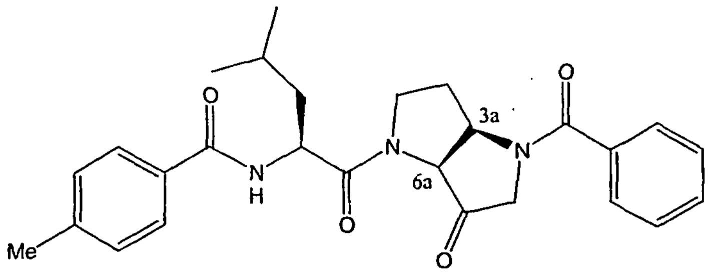

HPLC Rt = 14.35 mins (> 90%), HPLC-MS 462.2 [M + H]$^+$, 945.3 [2M + Na]$^+$.

EXAMPLE 171. (3a*R*, 6a*S*)-*N*-[(1*S*)-1-(4-Benzoyl-6-oxo-hexahydro-pyrrolo[3,2-*b*] pyrrole-1-carbonyl)-3-methyl-butyl]-4-ethyl-benzamide

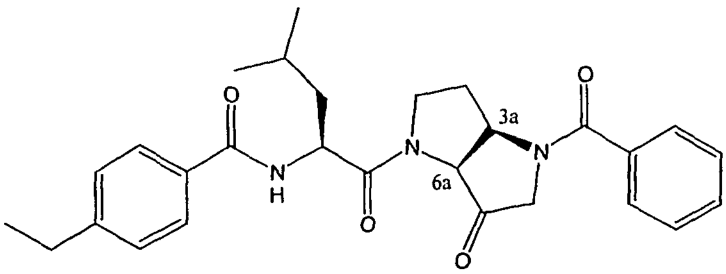

HPLC Rt = 15.40 mins (> 90%), HPLC-MS 476.2 [M + H]$^+$, 973.4 [2M + Na]$^+$.

EXAMPLE 172. (3a*R*, 6a*S*)-*N*-[(1*S*)-1-(4-Benzoyl-6-oxo-hexahydro-pyrrolo[3,2-*b*] pyrrole-1-carbonyl)-3-methyl-butyl]-4-isopropyl-benzamide

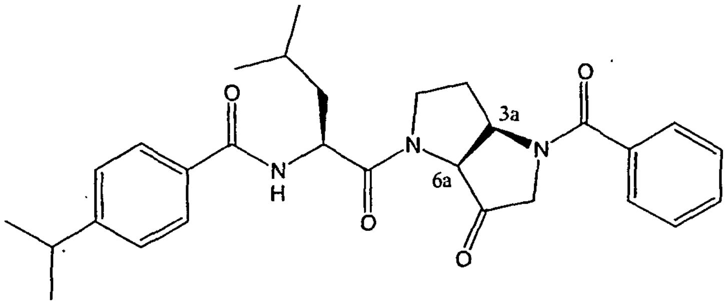

HPLC Rt = 16.41 mins (> 85%), HPLC-MS 490.2 [M + H]$^+$.

EXAMPLE 173. (3a*R*, 6a*S*)-*N*-[(1*S*)-1-(4-Benzoyl-6-oxo-hexahydro-pyrrolo[3,2-*b*] pyrrole-1-carbonyl)-3-methyl-butyl]-4-trifluoromethoxy-benzamide

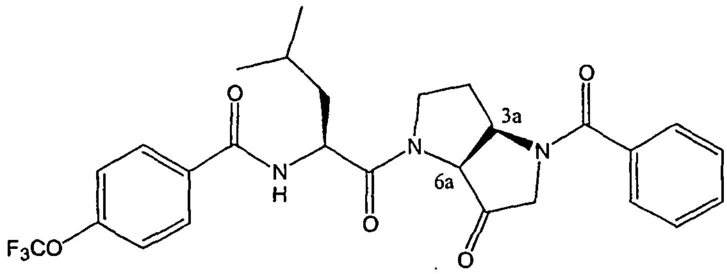

HPLC Rt =16.44 mins (> 90%), HPLC-MS 532.1 [M + H]$^+$.

EXAMPLE 174. (3a*R*, 6a*S*)-*N*-[(1*S*)-1-(4-Benzoyl-6-oxo-hexahydro-pyrrolo[3,2-*b*] pyrrole-1-carbonyl)-3-methyl-butyl]-benzamide

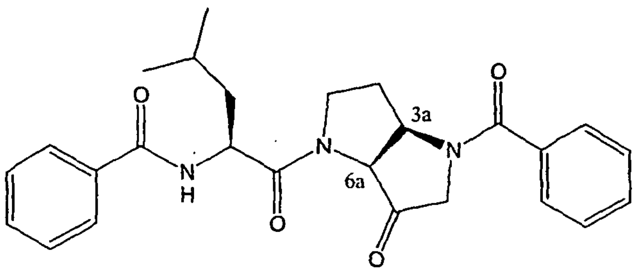

HPLC Rt = 13.30 mins (> 90%), HPLC-MS 448.2 [M + H]$^+$, 470.1 [M + Na]$^+$, 917.2 [2M + Na]$^+$.

EXAMPLE 175. (3a*S*, 6a*S*)-4-[(2*S*)-2-(3-Aminomethyl-benzoylamino)-4-methyl-pentanoyl]-3-oxo-hexahydro-pyrrolo[3,2-*b*]pyrrole-1-carboxylic acid phenylamide

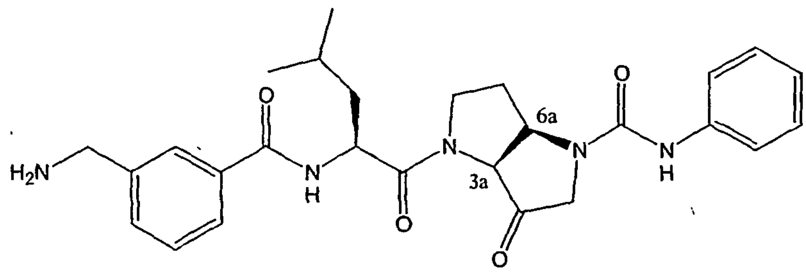

HPLC Rt =10.69 mins (> 95%), HPLC-MS 492.2 [M + H]$^+$, 983.4 [2M + H]$^+$.

EXAMPLE 176. (3a*R*, 6a*S*)-3-Aminomethyl-*N*-{(1*S*-3-methyl-1-[6-oxo-4-((2*R*)-2-phenyl-propionyl)-hexahydro-pyrrolo-[3,2-*b*]pyrrole-1-carbonyl]-butyl}-benzamide

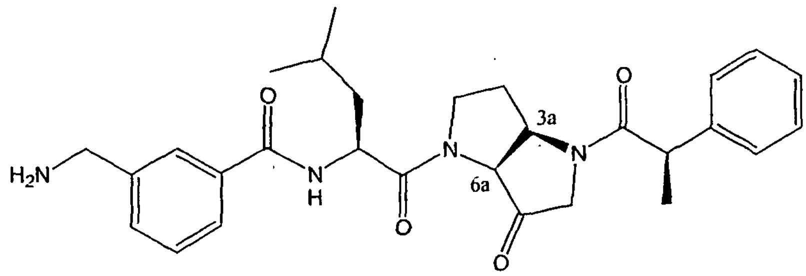

HPLC Rt = 12.2-13.8 mins (> 90%), HPLC-MS 505.2 [M + H]$^+$, 523.2 [M + H + $H_2O$]$^+$.

EXAMPLE 177. (3a*R*, 6a*S*)-3-Aminomethyl-*N*-{(1*S*)-3-methyl-1-[6-oxo-4-((2*S*)-2-phenyl-propionyl)-hexahydro-pymolo[3,2-*b*]pyrrole-1-carbonyl]-butyl}-benzamide

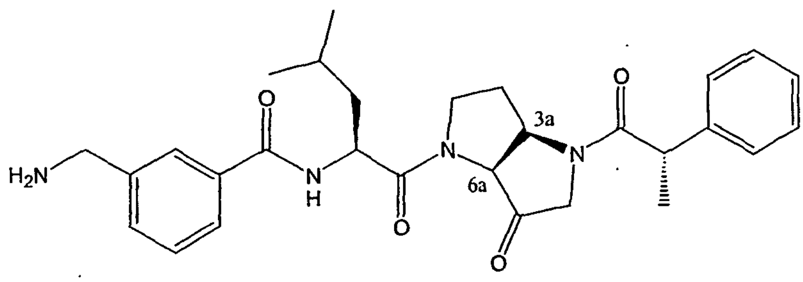

HPLC Rt = 11.9-13.5 mins (> 85%), HPLC-MS 505.2 [M + H]$^+$.

EXAMPLE 178. (3a*R*, 6a*S*)-3-Aminomethyl-*N*-((1*S*)-1-{4-[2-(2-fluoro-phenyl)-acetyl]-6-oxo-hexahydro-pyrrolo[3,2-*b*]-pyrrole-1-carbonyl}-3-methyl-butyl)-benzamide

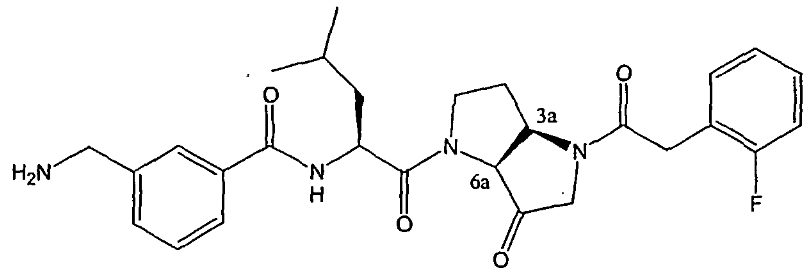

HPLC Rt = 11.65 mins (> 90%), HPLC-MS 509.2 [M + H]$^+$.

EXAMPLE 179. (3a*R*, 6a*S*)-3-Aminomethyl-*N*-((1*S*)-1-{4-[2-(3-fluoro-phenyl)-acetyl]-6-oxo-hexahydro-pyrrolo[3,2-*b*]-pyrrole-1-carbonyl}-3-methyl-butyl)-benzamide

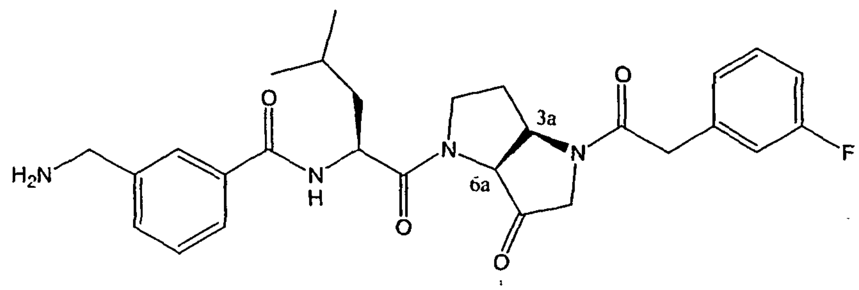

HPLC Rt = 11.70 mins (> 95%), HPLC-MS 509.2 [M + H]$^+$.

EXAMPLE 180. (3a*R*, 6a*S*)-3-Aminomethyl-*N*-((1*S*)-1-{4-[2-(4-fluoro-phenyl)-acetyl]-6-oxo-hexahydro-pyrrolo[3,2-*b*]pyrrole-1-carbonyl}-3-methyl-butyl)-benzamide

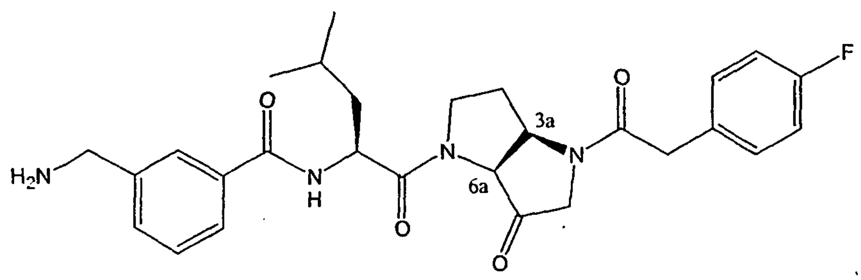

HPLC Rt = 11.74 mins (> 95%), HPLC-MS 509.2 [M + H]$^+$.

EXAMPLE 181. (3a*R*, 6a*S*)-3-Aminomethyl-*N*-{(1*S*)-1-[4-((2*R*)-2-amino-2-phenyl-acetyl)-6-oxo-hexahydro-pyrrolo[3,2-*b*]pyrrole-1-carbonyl]-3-methyl-butyl}-benzamide

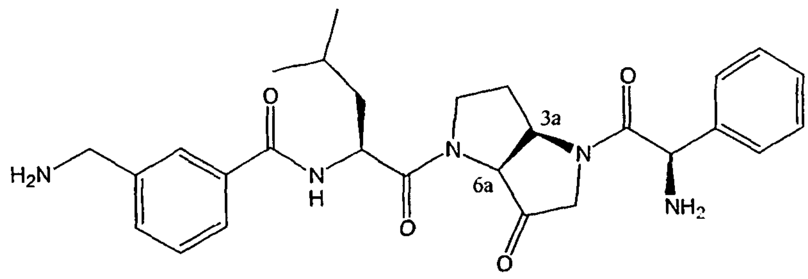

HPLC Rt = 8.93 mins (> 50%), HPLC-MS 506.2 [M + H]$^+$, 524.2 [M + H + $H_2O$]$^+$.

EXAMPLE 182. (3a*R*, 6a*S*)-3-Aminomethyl-*N*-{(1*S*-1-[4-((2*S*-2-amino-2-phenyl-acetyl)-6-oxo-hexahydro-pyrrolo[3,2-*b*]pyrrole-1-carbonyl]-3-methyl-butyl}-benzamide

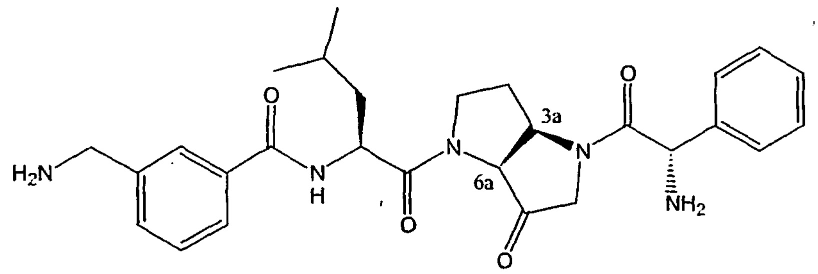

HPLC Rt = 7.2-9.3 mins (> 60%), HPLC-MS 506.2 [M + H]$^+$, 524.2 [M + H + $H_2O$]$^+$.

EXAMPLE 183. (3a*S*, 6a*R*)-(2-{4-[(2*S*)-2-(4-Dimethylamino-benzoylamino)-4-methyl-pentanoyl]-3-oxo-hexahydro-pyrrolo[3,2-*b*]pyrrol-1-yl}-2-oxo-ethyl)-carbamic acid methyl ester

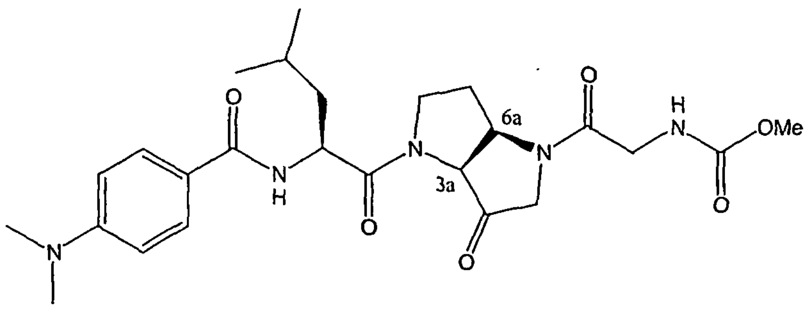

HPLC Rt = 7.96 mins (> 95%), HPLC-MS 502.2 [M + H]$^+$.

EXAMPLE 184. (3a*R*, 6a*S*)-4-Dimethylamino-*N*-((1*S*)-3-methyl-1-{6-oxo-4-[2-(toluene-4-sulfonylamino)-acetyl]-hexahydro-pyrrolo[3,2-*b*]pyrrole-1-carbonyl}-butyl)-benzamide

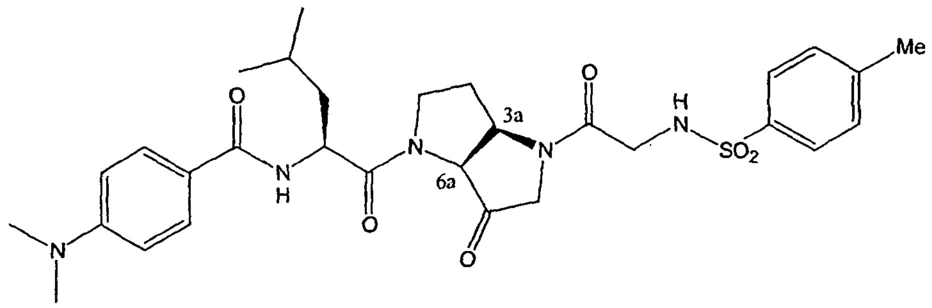

HPLC Rt = 13.40 mins (> 90%), HPLC-MS 598.2 $[M + H]^+$.

EXAMPLE 185. (3a*R*, 6a*S*)-3-Aminomethyl-*N*-{(1*S*)-1-[4-(2-methanesulfonyl-benzoyl)-6-oxo-hexahydro-pyrrolo-[3,2-*b*]pyrrole-1-carbonyl]-3-methyl-butyl}-benzamide

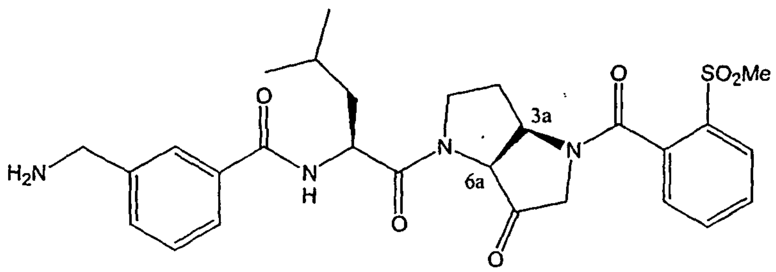

HPLC Rt = 9.4-10.4 mins (> 85%), HPLC-MS 555.2 $[M + H]^+$, 573.2 $[M + H + H_2O]^+$.

EXAMPLE 186. (3a*R*, 6a*S*)-*N*-[(1*S*)-2-(4-Benzoyl-6-oxo-hexahydro-pyrrolo[3,2-*b*] pyrrol-1-yl)-1-cyclopropylmethyl-2-oxo-ethyl]-4-dimethylamino-benzamide

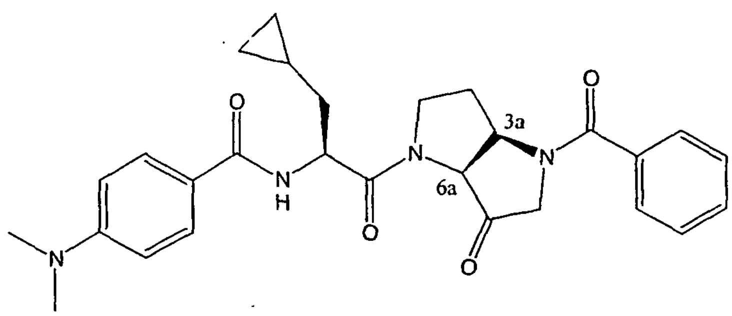

HPLC Rt = 11.0-13.0 mins (> 85%), HPLC-MS [489.2 M + H]$^+$, 507.2 $[M + H + H_2O]^+$.

EXAMPLE 187. (3a*R*, 6a*S*)-Thiophene-3-carboxylic acid [(1*S*)-2-(4-benzoyl-6-oxo-hexahydro-pyrrolo[3,2-*b*]pyrrol-1-yl)-1-cyclopropylmethyl-2-oxo-ethyl]-amide

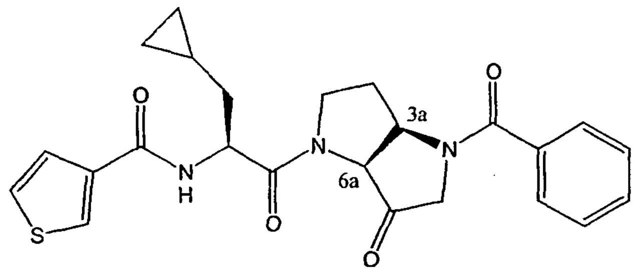

HPLC Rt = 12.10 mins (> 80%), HPLC-MS 452.1 $[M + H]^+$, 925.2 $[2M + Na]^+$.

EXAMPLE 188. (3a*R*, 6a*S*)-1-(4-Benzoyl-6-oxo-hexahydro-pyrrolo[3,2-*b*]pyrrol-1-yl)-4-(3,4-dihydro-1*H*-isoquinolin-2-yl)-2-isobutyl-butane-1,4-dione

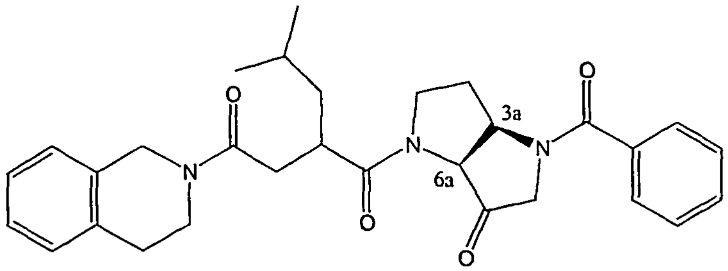

HPLC Rt = 15.1-16.5 mins (> 80%), HPLC-MS 502.1 $[M + H]^+$, 520.2 $[M + H + H_2O]^+$.

EXAMPLE 189. (3a*S*, 6a*R*)-1-Benzoyl-4-(2-biphenyl-3-yl-4-methyl-pentanoyl)-hexahydro-pyrrolo[3,2-*b*]pyrrol-3-one

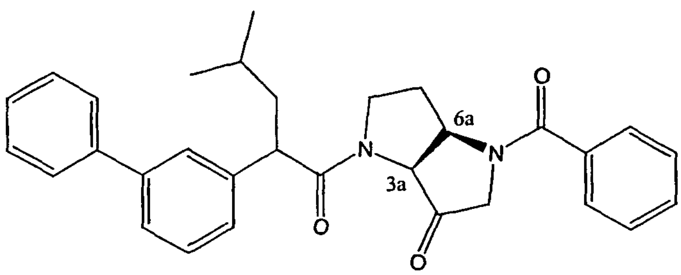

HPLC Rt = 18.1-20.1 mins (> 80%), HPLC-MS 481.2 $[M + H]^+$, 499.2 $[M + H + H_2O]^+$, 983.3 $[2M + Na]^+$.

EXAMPLE 190. (3a*S*, 6a*R*)-4-[(2*S*)-2-(4-*tert*-Butyl-benzoylamino)-4-methylpentanoyl]-3-oxo-hexahydro-pyrrolo[3,2-*b*]pyrrole-1-carboxylic acid phenylamide

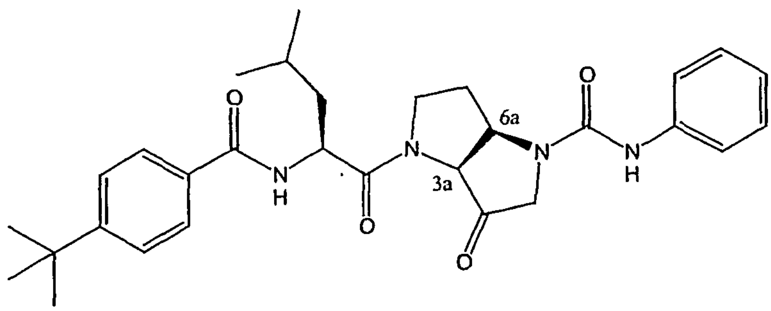

HPLC Rt = 16.9-18.4 mins (> 85%), HPLC-MS 519.2 [M + H]$^+$.

EXAMPLE 191. (3a*R*, 6a*S*)-4-*tert*-Butyl-*N*-{(1*S*)-3-methyl-1-[4-(4-methylpentanoyl)-6-oxo-hexahydro-pyrrolo[3,2-*b*]pyrrole-1-carbonyl]-butyl}-benzamide

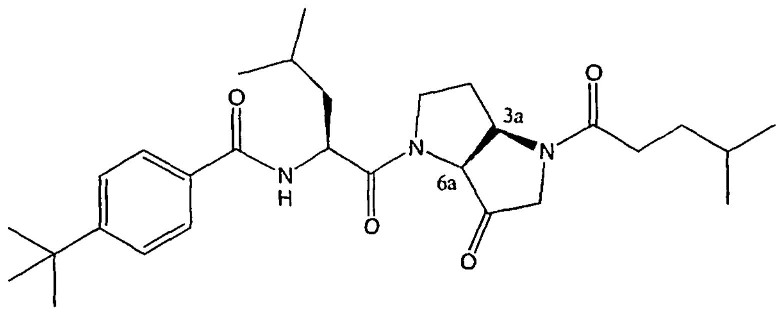

HPLC Rt = 16.3-17.5 mins (> 85%), HPLC-MS 498.2 [M + H]$^+$.

EXAMPLE 192. (3a*R*, 6a*S*)-*N*-{(1*S*)-1-[4-((2*S*)-2-Amino-4-methyl-pentanoyl)-6-oxo-hexahydro-pyrrolo[3,2-*b*]pyrrole-1-carbonyl]-3-methyl-butyl}-4-*tert*-butyl-benzamide

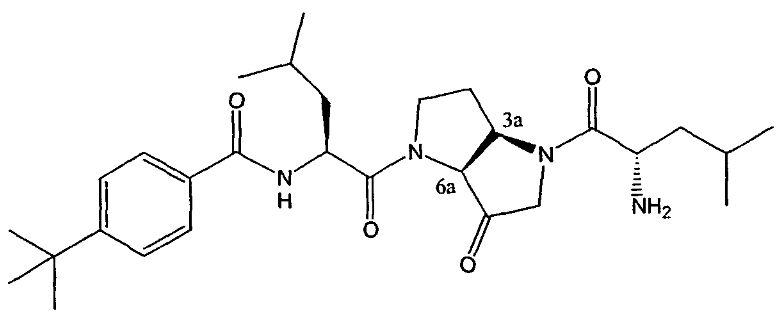

HPLC Rt =16.19 mins (> 90%), HPLC-MS 513.3 $[M + H]^+$.

EXAMPLE 193. (3a*R*, 6a*S*)-*N*-[(1*S*)-1-(4-Benzoyl-6-oxo-hexahydro-pyrrolo[3,2-*b*] pyrrole-1-carbonyl)-3-methyl-butyl]-4-pyrrolidin-1-yl-benzamide

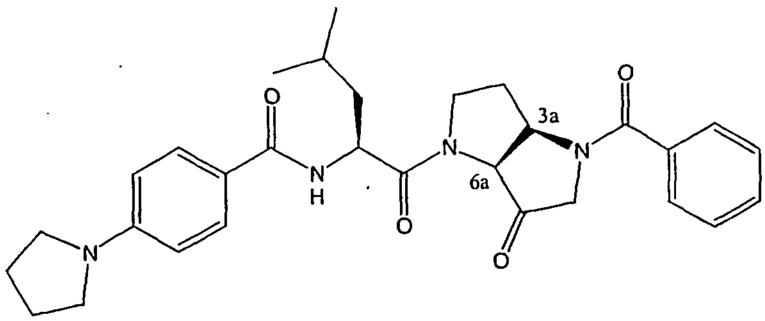

HPLC Rt = 17.45 mins (> 85%), HPLC-MS 517.1 $[M + H]^+$, 535.2 $[M + H + H_2O]^+$.

EXAMPLE 194. (3a*R*, 6a*S*)-*N*-[(1*S*)-1-(4-Benzoyl-6-oxo-hexahydro-pyrrolo[3,2-*b*] pyrrole-1-carbonyl)-3-methyl-butyl]-4-piperazin-1-yl-benzamide

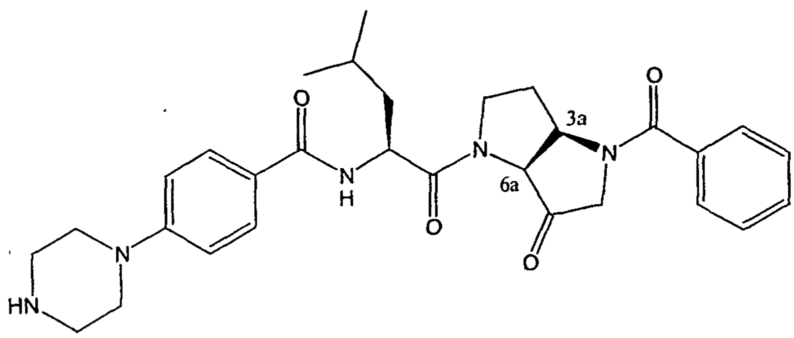

HPLC Rt = 11.50 mins (> 95%), HPLC-MS 532.1 $[M + H]^+$, 550.2 $[M + H + H_2O]^+$.

EXAMPLE 195. (3a*R*, 6a*S*)-*N*-[(1*S*)-1-(4-Benzoyl-6-oxo-herahydro-pyrrolo[3,2-*b*] pyrrole-1-carbonyl)-3-methyl-butyl]-4-dimethylamino-*N*-methyl-benzamide

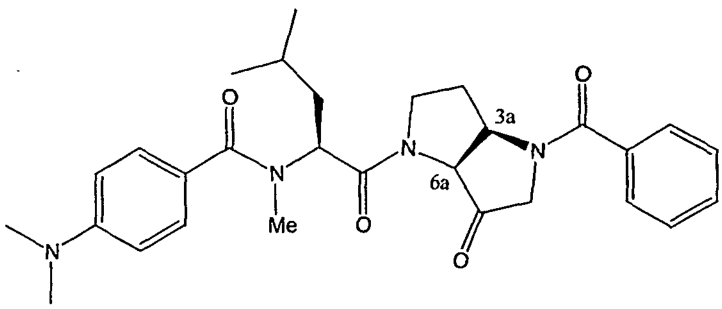

HPLC Rt = 18.29 mins (> 50%), HPLC-MS 505.1 $[M + H]^+$.

EXAMPLE 196. (3a*R*, 6a*S*)-*N*-[(1*S*)-1-(4-Benzoyl-6-oxo-hexahydro-pyrrolo[3,2-*b*] pyrrole-1-carbonyl)-butyl]-4-dimethyl-amino-benzamide

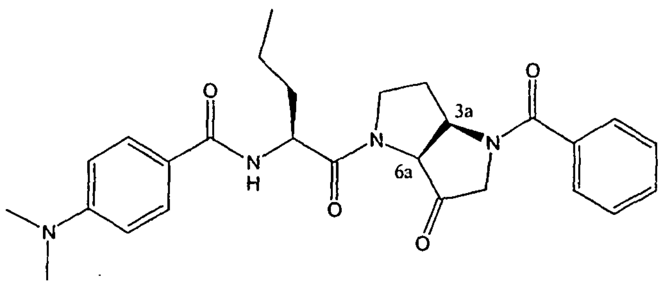

HPLC Rt = 11.09 mins (> 80%), HPLC-MS 477.1 $[M + H]^+$, 495.1 $[M + H + H_2O]^+$.

EXAMPLE 197. (3a*R*, 6a*S*)-*N*-{(1*S*)-1-[4-((2*S*)-2-Acetylamino-propionyl)-6-oxo-hexahydro-pyrrolo[3,2-*b*]pyrrole-1-carbonyl]-3-methyl-butyl}-4-dimethylamino-benzamide

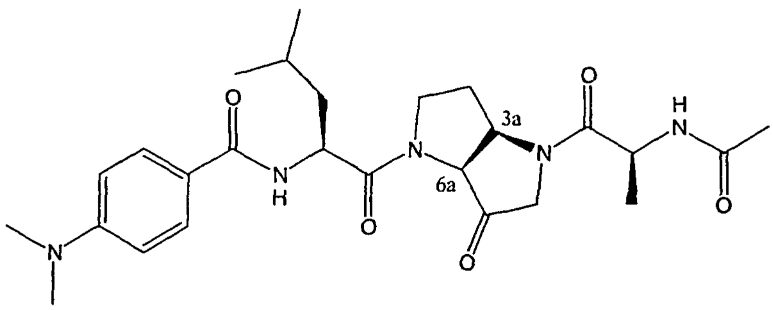

HPLC Rt = 9.06 mins (> 90%), HPLC-MS 500.1 $[M + H]^+$, 518.2 $[M + H + H_2O]^+$.

EXAMPLE 198. (3a*R*, 6a*S*)-*N*-{(1*S*)-1-[4-(2-Amino-acetyl)-6-oxo-hexahydro-pyrrolo[3,2-*b*]pyrrole-1-carbonyl]-3-methyl-butyl}-4-dimethylamino-benzamide

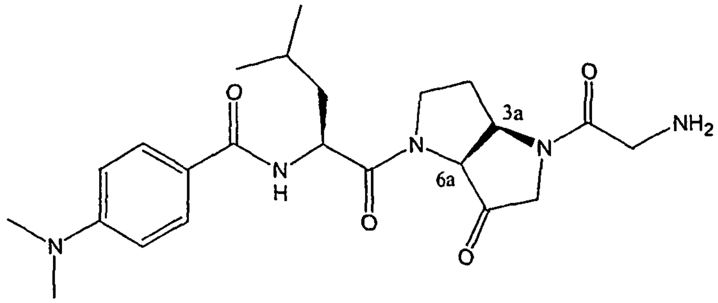

HPLC Rt = 7.93 mins (> 90%), HPLC-MS 444.1 $[M + H]^+$.

EXAMPLE 199. (3a*R*, 6a*S*)-*N*-{(1*S*)-1-[4-(2-Acetylamino-acetyl)-6-oxo-hexahydro-pyrrolo[3,2-*b*]pyrrole-1-carbonyl]-3-methyl-butyl}-4-dimethylamino-benzamide

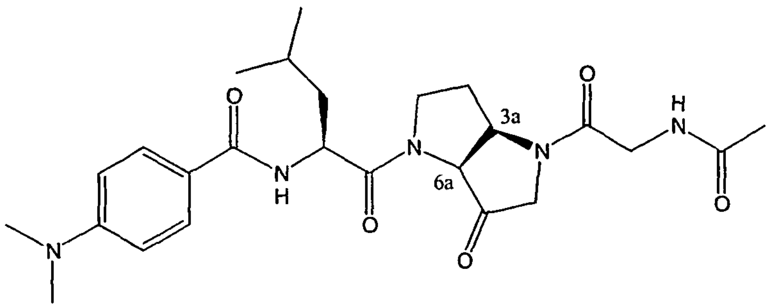

HPLC Rt = 8.1 mins (> 90%), HPLC-MS 486.1 $[M + H]^+$.

EXAMPLE 200. (3a*R*, 6a*S*)-4-Dimethylamino-*N*-{(1*S*)-3-methyl-1-[4-(2-methylamino-acetyl)-6-oxo-hexahydro-pyrrolo[3,2-*b*]pyrrole-1-carbonyl]-butyl}-benzamide

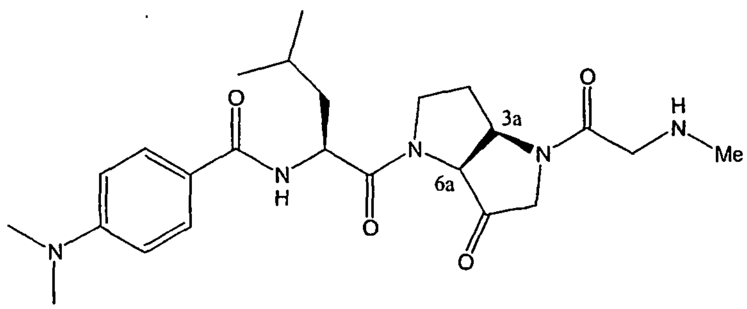

HPLC Rt = 7.89 mins (> 90%), HPLC-MS 458.2 [M + H]+.

EXAMPLE 201. (3a*R*, 6a*S*)-*N*-((1*S*)-1-{4-[2-(Acetyl-methyl-amino)-acetyl]-6-oxo-hexahydro-pyrrolo[3,2-*b*]pyrrole-1-carbonyl}-3-methyl-butyl)-4-dimethylamino-benzamide

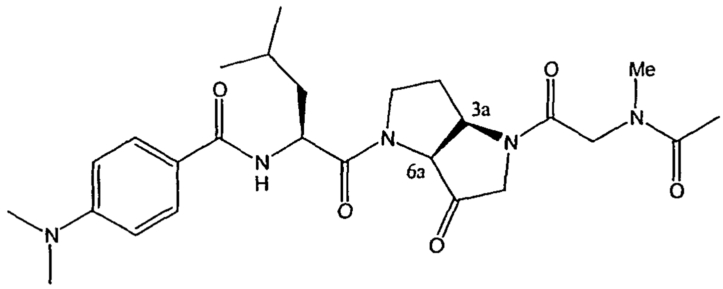

HPLC Rt = 8.89 mins (> 90%), HPLC-MS 500.2 [M + H]+.

EXAMPLE 202. (3a*R*, 6a*S*)-*N*-{(1*S*)-1-[4-(3-Acetylamino-propionyl)-6-oxo-hexahydro-pyrrolo[3,2-*b*]pyrrole-1-carbonyl]-3-methyl-butyl}-4-dimethylamino-benzamide

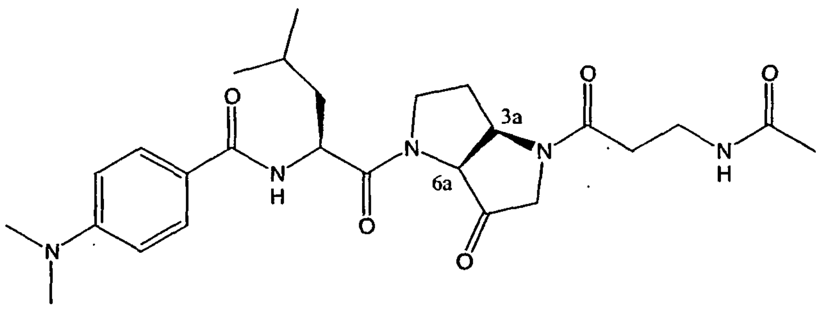

HPLC Rt = 8.57 mins (> 90%), HPLC-MS 500.1 [M + H]+.

EXAMPLE 203. (3a*R*, 6a*S*)-*N*-{(1*S*)-1-[4-((2*S*)-2-Amino-butyryl)-6-oxo-hexahydro-pyrrolo[3,2-*b*]pyrrole-1-carbonyl]-3-methyl-butyl}-4-dimethylamino-benzamide

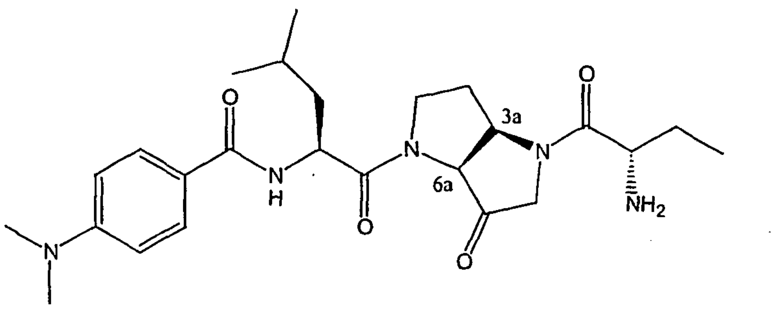

HPLC Rt = 9.29 mins (> 90%), HPLC-MS 472.2 [M + H]+.

EXAMPLE 204. (3a*R*, 6a*S*)-*N*-{(1*S*)-1-[4-((2*S*)-2-Acetylarnino-butyryl)-6-oxo-hexahydro-pyrrolo[3,2-*b*]pyrrole-1-carbonyl]-3-methyl-butyl}-4-dimethylamino-benzamide

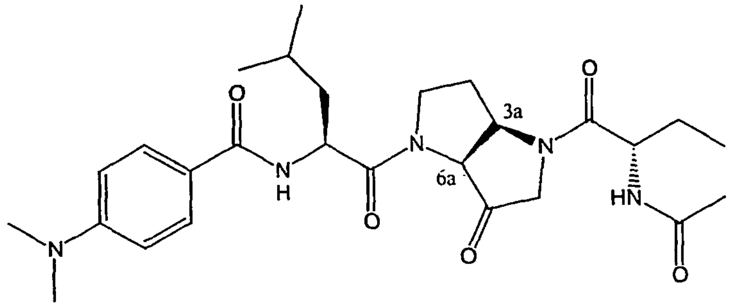

HPLC Rt = 10.45 mins (> 90%), HPLC-MS 514.2 [M + H]+.

EXAMPLE 205. (3a*R*, 6a*S*)-*N*-{(1*S*)-1-[4-((2*S*)-2-Amino-3-methyl-butyryl)-6-oxo-hexahydro-pyrrolo[3,2-*b*]pyrrole-1-carbonyl]-3-methyl-butyl}-4-dimethylamino-benzamide

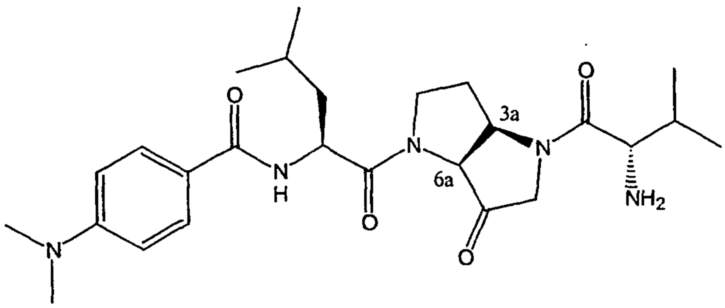

HPLC Rt = 9.92 mins (> 90%), HPLC-MS 486.2 [M + H]+.

EXAMPLE 206. (3a*R*, 6a*S*)-*N*-{(1*S*)-1-[4-((2*S*)-2-Amino-3-hydroxy-propionyl)-6-oxo-hexahydro-pyrrolo[3,2-*b*]pyrrole-1-carbonyl]-3-methyl-butyl}-4-dimethylamino-benzamide

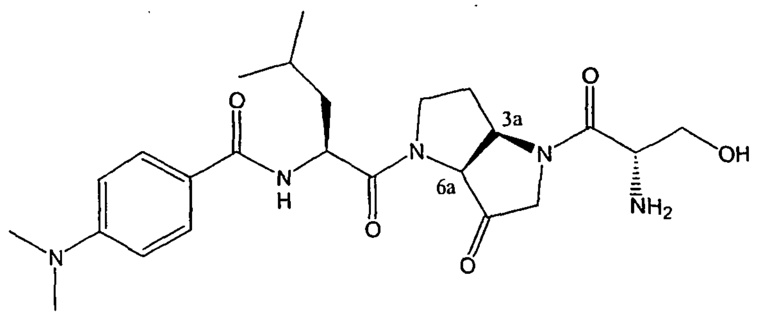

HPLC Rt = 7.56 mins (> 90%), HPLC-MS 474.1 [M + H]$^+$.

EXAMPLE 207. (3a*R*, 6a*S*)-*N*-{(1*S*)-1-[4-((2*S*,3*R*)-2-Amino-3-hydroxy-butyryl)-6-oxo-hexahydro-pyrrolo[3,2-*b*]pyrrole-1-carbonyl]-3-methyl-butyl}-4-dimethylamino-benzamide

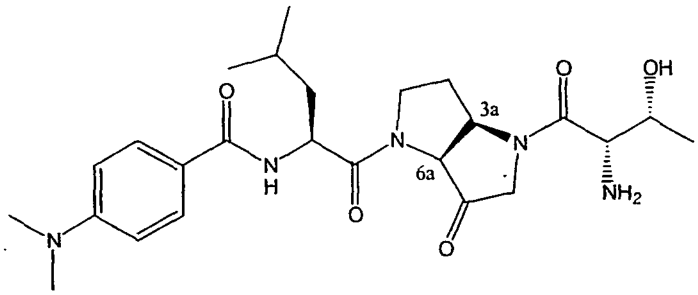

HPLC Rt = 8.00 mins (> 80%), HPLC-MS 488.1 [M + H]$^+$.

EXAMPLE 208. (3a*R*, 6a*S*)-*N*-{(1*S*)-1-[4-((2*S*,3*S*)-2-Amino-3-hydroxy-butyryl)-6-oxo-hexahydro-pyrrolo[3,2-*b*]pyrrole-1-carbonyl]-3-methyl-butyl}-4-dimethylamino-benzamide

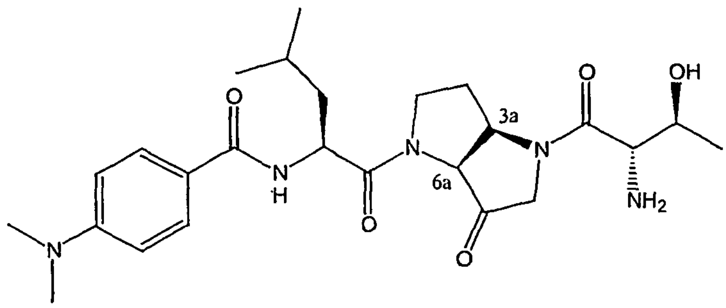

HPLC Rt = 8.13 mins (> 85%), HPLC-MS 488.1 [M + H]$^+$.

EXAMPLE 209. (3a*R*, 6a*S*)-*N*-{(1*S*)-1-[4-((2*S*, 3*S*)-2-Amino-3-methylpentanoyl)-6-oxo-hexahydro-pyrrolo[3,2-*b*]pyrrole-1-carbonyl]-3-methyl-butyl}-4-dimethylamino-benzamide

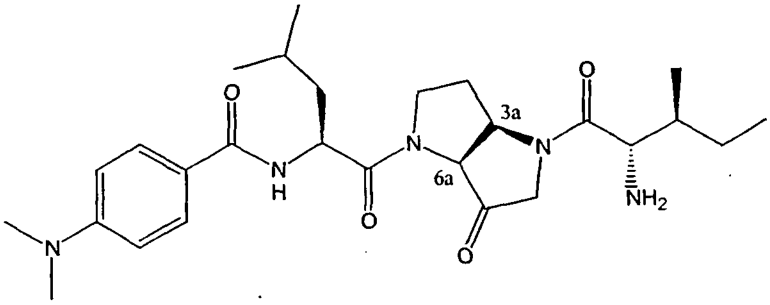

HPLC Rt = 10.97 mins (> 90%), HPLC-MS 500.2 $[M + H]^+$.

EXAMPLE 210. (3a*R*, 6a*S*)-*N*-{(1*S*)-1-[4-((2*S*)-2-Amino-pentanoyl)-6-oxo-hexahydro-pyrrolo[3,2-*b*]pyrrole-1-carbonyl]-3-methyl-butyl}-4-dimethylamino-benzamide

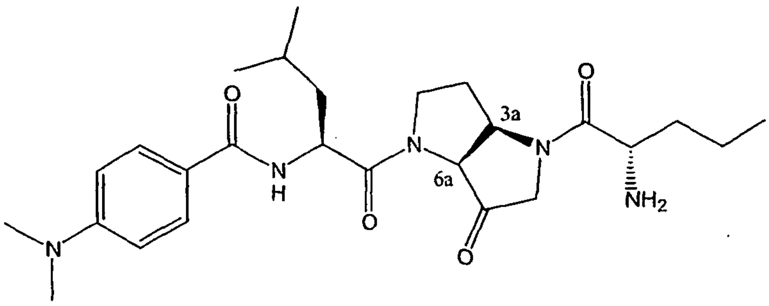

HPLC Rt = 10.32 mins (> 90%), HPLC-MS 486.2 $[M + H]^+$.

EXAMPLE 211. (3a*R*, 6a*S*)-*N*-{(1*S*)-1-[4-((2*S*)-2-Amino-3,3-dimethyl-butyryl)-6-oxo-hexahydro-pyrrolo[3,2-*b*]pyrrole-1-carbonyl]-3-methyl-butyl}-4-dimethylamino-benzamide

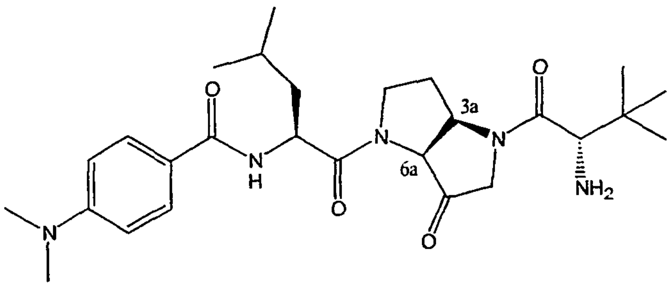

HPLC Rt =11.14 mins (> 90%), HPLC-MS 500.2 $[M + H]^+$.

EXAMPLE 212. (3a*R*, 6a*S*)-*N*-{(1*S*)-1-[4-((2*S*)-2-Amino-4,4-dimethyl-pentanoyl)-6-oxo-hexahydro-pyrrolo[3,2-*b*]pyrrole-1-carbonyl]-3-methyl-butyl}-4-dimethylamino-benzamide

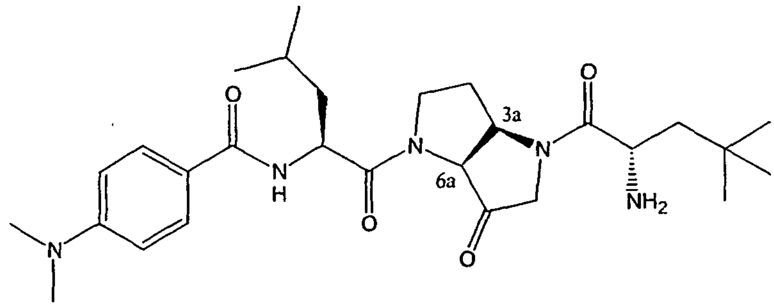

HPLC Rt = 12.01 mins (> 90%), HPLC-MS 514.2 [M + H]$^+$.

EXAMPLE 213. (3a*R*, 6a*S*)-*N*-{(1*S*)-1-[4-((2*S*)-2-Amino-5-methyl-hexanoyl)-6-oxo-hexahydro-pyrrolo[3,2-*b*]pyrrole-1-carbonyl]-3-methyl-butyl}-4-dimethylamino-benzamide

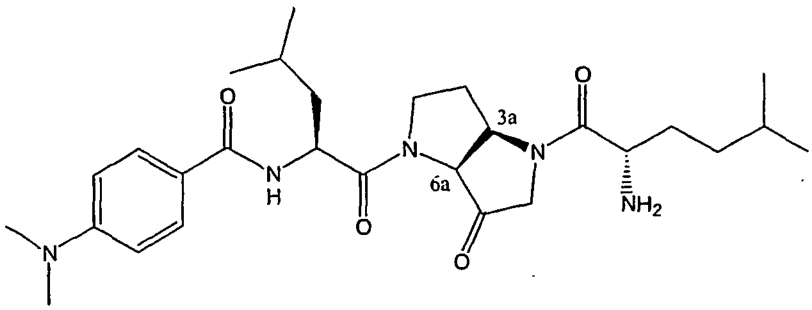

HPLC Rt = 12.83 mins (> 95%), HPLC-MS 514.2 [M + H]$^+$.

EXAMPLE 214. (3a*R*, 6a*S*)-*N*-{(1*S*)-1-[4-((2*S*)-2-Acetylamino-5-methyl-hexanoyl)-6-oxo-hexahydro-pyrrolo[3,2-*b*]pyrrole-1-carbonyl]-3-methyl-butyl}-4-dimethylamino-benzamide

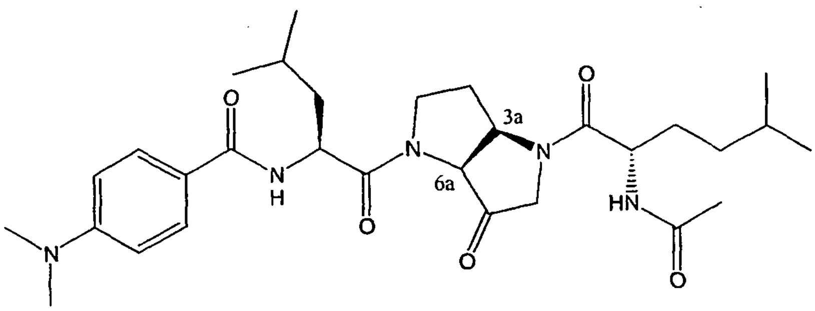

HPLC Rt =14.18 mins (> 95%), HPLC-MS 556.2 [M + H]$^+$.

EXAMPLE 215. (3a*R*,6a*S*)-*N*-{(1*S*)-1-[4-((2*S*)-2-Amino-3-cyclohexyl-propionyl)-6-oxo-hexahydro-pyrrolo[3,2-*b*]pyrrole-1-carbonyl]-3-methyl-butyl}-4-dimethylamino-benzamide

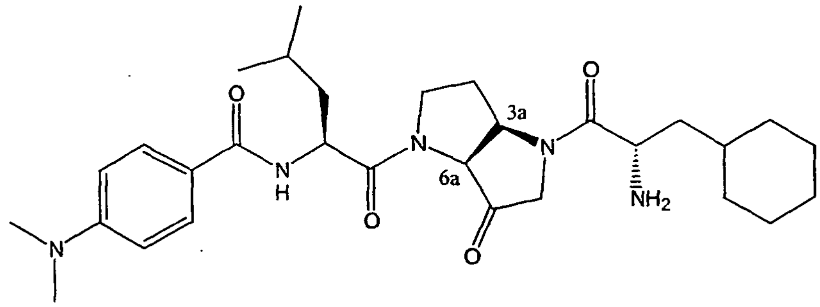

HPLC Rt = 13.54 mins (> 95%), HPLC-MS 540.2 [M + H]$^+$.

EXAMPLE 216. (3a*R*, 6a*S*)-*N*-{(1*S*)-1-[4-((2*S*)-1-Acetyl-pyrrolidine-2-carbonyl)-6-oxo-hexahydro-pyrrolo[3,2-*b*]pyrrole-1-carbonyl]-3-methyl-butyl}-4-dimethylamino-benzamide

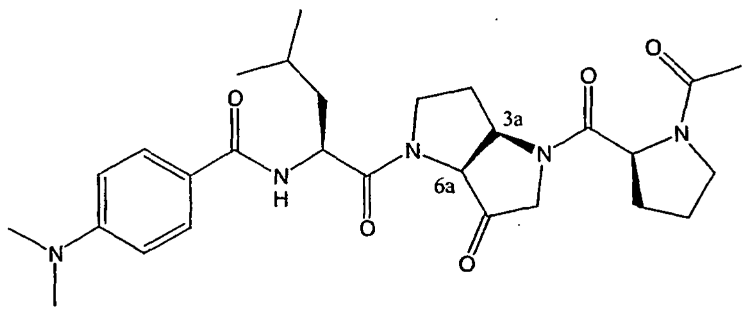

HPLC Rt = 9.88 mins (> 95%), HPLC-MS 526.2 [M + H]$^+$.

EXAMPLE 217.(3a*R*, 6a*S*)-4-Dimethylamino-*N*-{(1*S*)-3-methyl-1-[6-oxo-4-((2*S*)-piperidine-2-carbonyl)-hexahydro-pyrrolo[3,2-*b*]pyrrole-1-carbonyl]-butyl}-benzamide

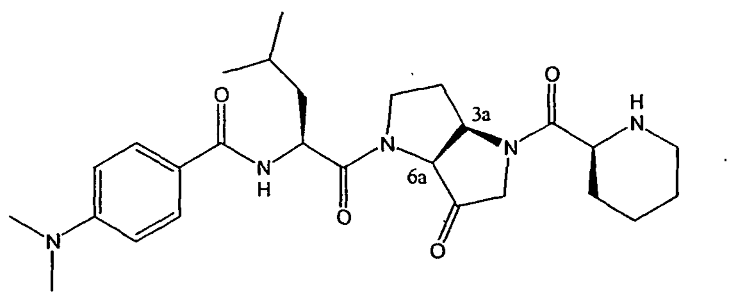

HPLC Rt = 9.12 mins (> 90%), HPLC-MS 498.2 [M + H]+, 995.3 [2M + H]+.

EXAMPLE 218. (3a*R*, 6a*S*)-4-Dimethyamino-*N*-{(1*S*)-1-[4-((2*S*,4*R*)-4-hydroxy-pyrrolidine-2-carbonyl)-6-oxo-hexahydro-pyrrolo[3,2-*b*]pyrrole-1-carbonyl]-3-methyl-butyl}-benzamide

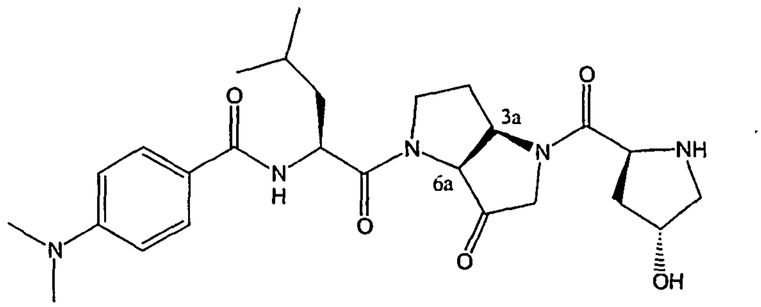

HPLC Rt = 8.00 mins (> 90%), HPLC-MS 500.2 [M + H]+, 999.3 [2M + H]+.

EXAMPLE 219. (3a*R*, 6a*S*)-*N*-{(1*S*)-1-[4-((2*S*)-2-Amino-3-benzyloxy-propionyl)-6-oxo-hexahydro-pyrrolo[3,2-*b*]pyrrole-1-carbonyl]-3-methyl-butyl}-4-dimethylamino-benzamide

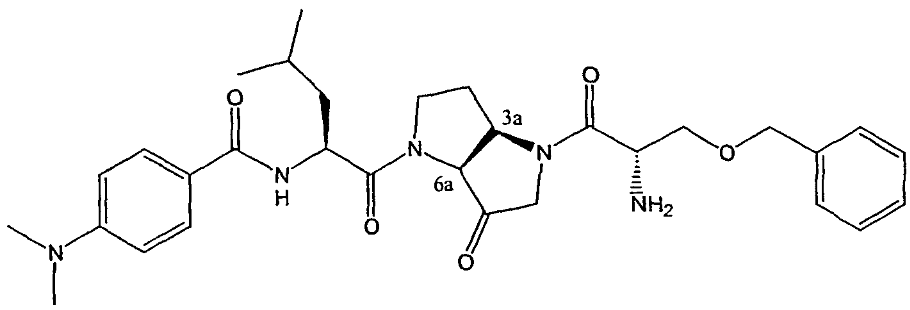

HPLC Rt = 11.6-12.7 mins (> 85%), HPLC-MS 564.2 [M + H]+.

EXAMPLE 220. (3a*R*, 6a*S*)-4-Dimethylamino-*N*-{(1*S*)-3-methyl-1-[4-((2*S*)-4-methyl-2-methylamino-pentanoyl)-6-oxo-hexahydro-pyrrolo[3,2-*b*]pyrrole-1-carbonyl]-butyl}-benzamide

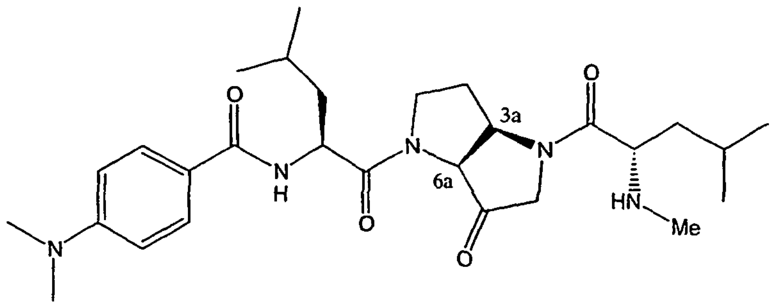

HPLC Rt =12.12 mins (> 90%), HPLC-MS 514.2 [M + H]+.

EXAMPLE 221. (3a*R*, 6a*S*)-*N*-((1*S*)-1-{4-[(2*S*)-2-(Acetyl-methyl-amino)-4-methyl-pentanoyl]-6-oxo-hexahydro-pyrrolo-[3,2-*b*]pyrrole-1-carbonyl}-3-methyl-butyl)-4-dimethylamino-benzamide

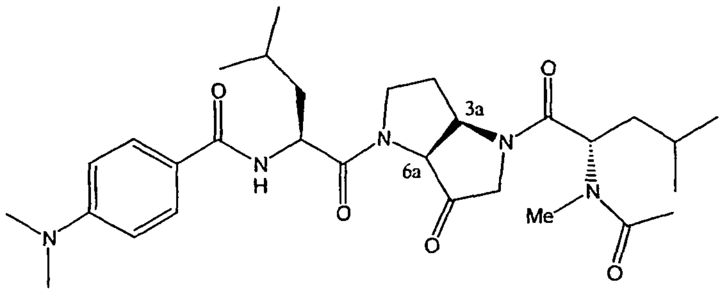

HPLC Rt = 13.37 mins (> 50%), HPLC-MS 556.2 [M + H]+.

EXAMPLE 222. (3a*R*, 6a*S*)-*N*-{(1*S*)-1-[4-(1-Amino-cyclopropanecarbonyl)-6-oxo-hexahydro-pyrrolo[3,2-*b*]pyrrole-1-carbonyl]-3-methyl-butyl}-4-dimethylamino-benzamide

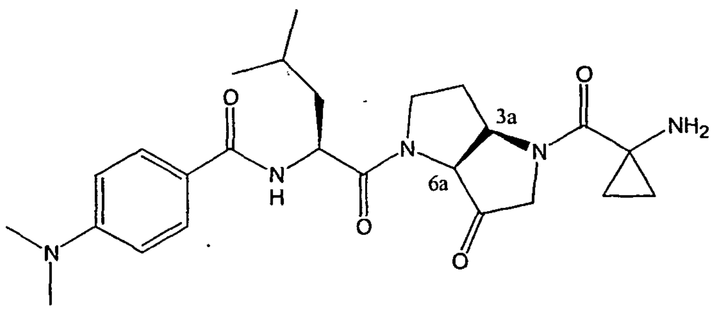

HPLC Rt = 8.16 mins (> 50%), HPLC-MS 470.2 [M + H]+.

EXAMPLE 223. (3a*R*, 6a*S*)-*N*-{(1*S*)-1-[4-(1-Amino-cyclobutanecarbonyl)-6-oxo-hexahydro-pyrrolo[3,2-*b*]pyrrole-1-carbonyl]-3-methyl-butyl}-4-dimethylamino-benzamide

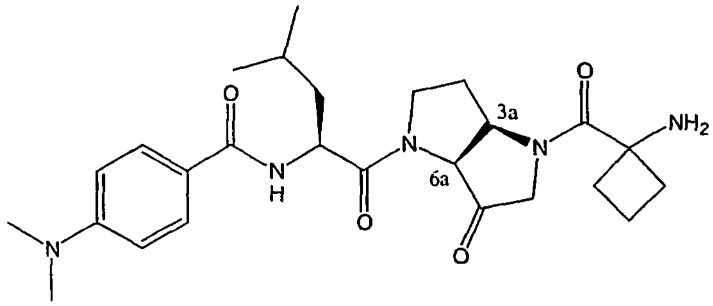

HPLC Rt = 8.4-8.9 mins (> 75%), HPLC-MS 484.2 [M + H]$^+$.

EXAMPLE 224. (3a*R*, 6a*S*)-*N*-{(1*S*)-1-[4-(1-Amino-cyclopentanecarbonyl)-6-oxo-hexahydro-pyrrolo[3,2-*b*]pyrrole-1-carbonyl]-3-methyl-butyl}-4-dimethylamino-benzamide

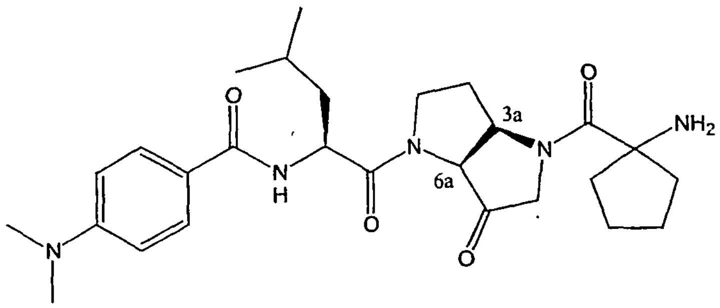

HPLC Rt = 8.5-8.9 mins (> 40%), HPLC-MS 498.2 [M + H]$^+$.

EXAMPLE 225. (3a*R*, 6a*S*)-*N*-{(1*S*)-1-[4-(1-Amino-cyclohexanecarbonyl)-6-oxo-hexahydro-pyrrolo[3,2-*b*]pyrrole-1-carbonyl]-3-methyl-butyl}-4-dimethylamino-benzamide

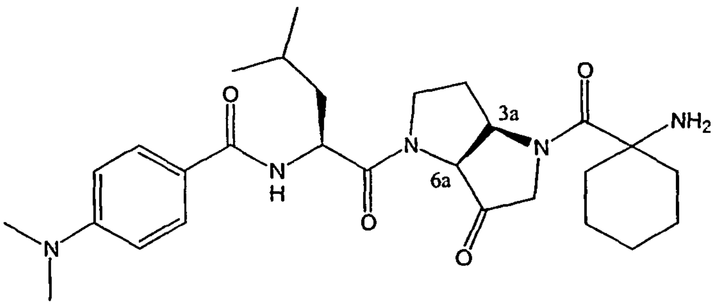

HPLC Rt = 9.1-9.5 mins (> 25%), HPLC-MS 529.9 [M + H + $H_2O$]+.

EXAMPLE 226. (3a*R*, 6a*S*)-*N*-{(1*S*)-1-[4-((2*S*)-2-Amino-2-cyclohexyl-acetyl)-6-oxo-hexahydro-pyrrolo[3,2-*b*]pyrrole-1-carbonyl]-3-methyl-butyl}-4-dimethylamino-benzamide

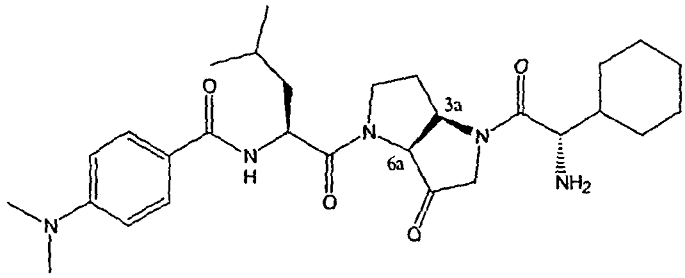

HPLC Rt = 12.17 mins (> 90%), HPLC-MS 526.2 [M + H]+.

EXAMPLE 227. (3a*R*, 6a*S*)-4-Dimethylamino-*N*-{(1*S*)-1-[4-(2-dimethylamino-acetyl)-6-oxo-hexahydro-pyrrolo[3,2-*b*]pyrrole-1-carbonyl]-3-memyl-butyl}-benzamide

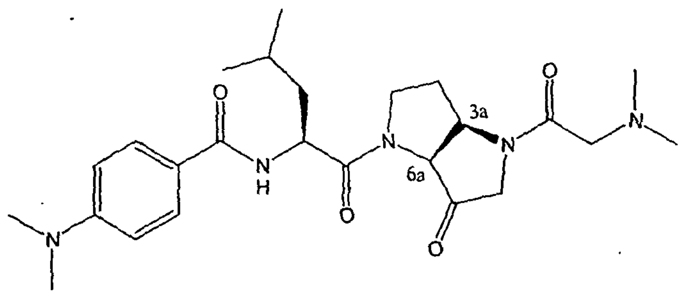

HPLC Rt = 7.7-8.1 mins (> 90%), HPLC-MS 472.2 [M + H]+, 490.2 [M + H + $H_2O$]+.

EXAMPLE 228. (3a*R*, 6a*S*)-*N*-{(1*S*)-1-[4-((2*S*)-2-Amino-4-methyl-pentanoyl)-6-oxo-hexahydro-pyrrolo[3,2-*b*]pyrrole-1-carbonyl]-3-methyl-butyl}-4-*tert*-butyl-benzamide

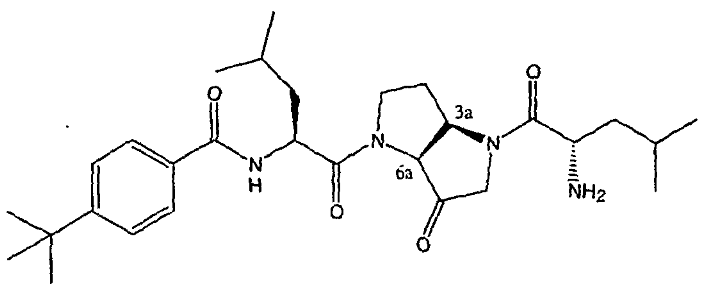

HPLC Rt = 15.47 mins (> 95%), HPLC-MS 513.2 [M + H]+.

EXAMPLE 229. (3a*R*, 6a*S*)-4-Dimethylamino-*N*-{(1*S*)-3-methyl-1-[4-(2-methylbutyryl)-6-oxo-hexahydro-pyrrolo[3,2-*b*]pyrrole-1-carbonyl]-butyl}-benzamide

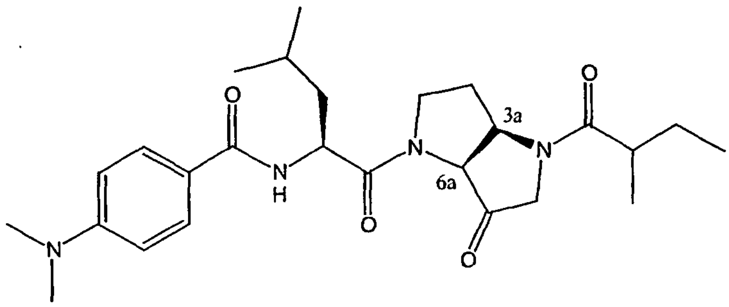

HPLC Rt = 11.4-11.5 mins (> 80%), HPLC-MS 471.2 $[M + H]^+$, 493.2 $[M + Na]^+$.

EXAMPLE 230. (3a*R*, 6a*S*)-4-Dimethylamino-*N*-{(1*S*)-3-methyl-1-[4-(2-methylpentanoyl)-6-oxo-hexahydro-pyrrolo-[3,2-*b*]pyrrole-1-carbonyl]-butyl}-benzamide

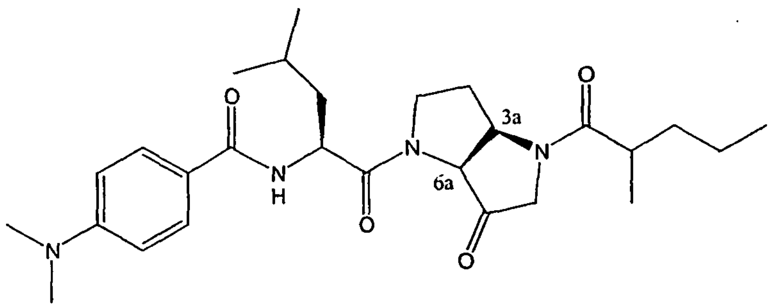

HPLC Rt = 12.4-13.3 mins (> 80%), HPLC-MS 485.2 $[M + H]^+$.

EXAMPLE 231. (3a*R*, 6a*S*)-4-Dimethylamino-*N*-{(1*S*)-1-[4-(2-ethyl-butyl)-6-oxo-hexahydro-pyrrolo[3,2-*b*]pyrrole-1-carbonyl]-3-methyl-butyl}-benzamide

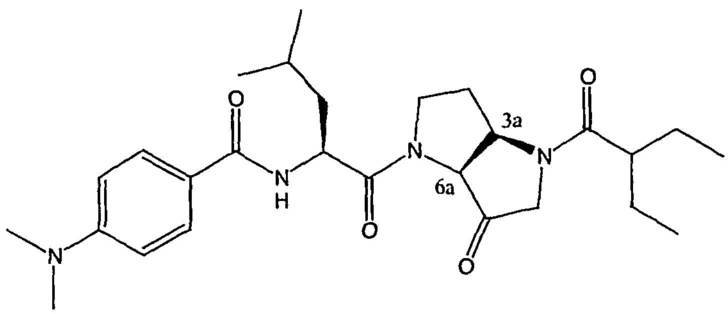

HPLC Rt = 12.34 mins (> 80%), HPLC-MS 485.2 $[M + H]^+$, 991.3 $[2M + Na]^+$.

EXAMPLE 232. (3a*R*, 6a*S*)-4-Dimethylamino-*N*-{(1*S*)-3-methyl-1-[6-oxo-4-(2-propyl-pentanoyl)-hexahydro-pyrrolo[3,2-*b*]pyrrole-1-carbonyl]-butyl}-benzamide

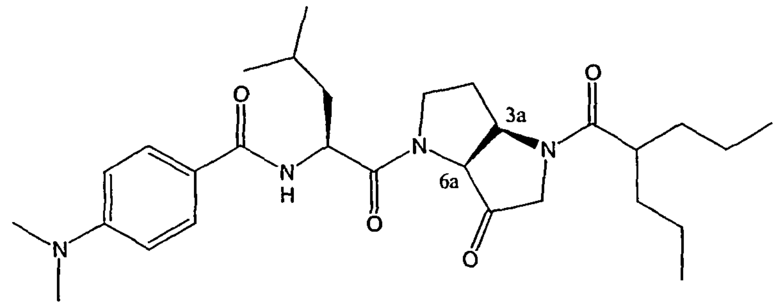

HPLC Rt = 14.75 mins (> 90%), HPLC-MS 513.2 $[M + H]^+$.

EXAMPLE 233. (3a*R*, 6a*S*)-4-Dimethylamino-*N*-{(1*S*)-1-[4-((2*S*)-2-hydroxy-4-methyl-pentanoyl)-6-oxo-hexahydro-pyrrolo[3,2-*b*]pyrrole-1-carbonyl]-3-methyl-butyl}-benzamide

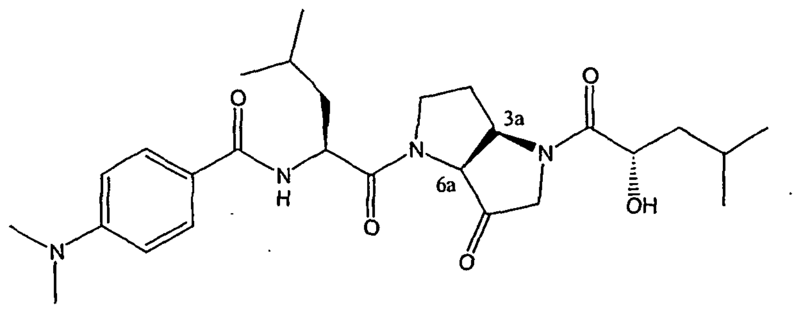

HPLC Rt = 12.04 mins (> 95%), HPLC-MS 501.2 $[M + H]^+$, 519.2 $[M + H + H_2O]^+$.

EXAMPLE 234. (3a*R*, 6a*S*)-4-Dimethylamino-*N*-[(1*S*)-1-(4-hexanoyl-6-oxo-hexahydro-pyrrolo[3,2-*b*]pyrrole-1-carbonyl)-3-methyl-butyl]-benzamide

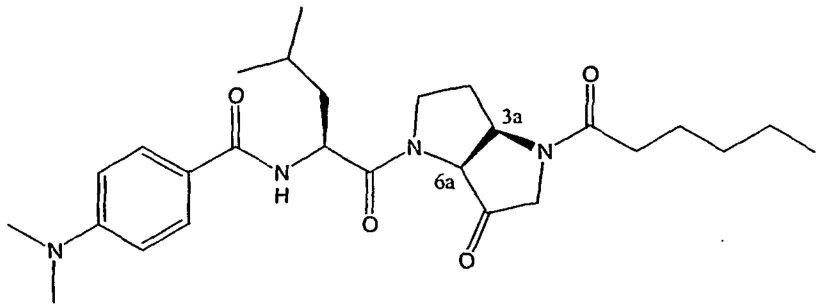

HPLC Rt = 13.24 mins (> 95%), HPLC-MS 485.2 [M + H]$^+$, 503.2 [M + H + $H_2O$]$^+$.

EXAMPLE 235. (3a*R*, 6a*S*)-4-Dimethylamino-*N*-{(1*S*)-3-methyl-1-[6-oxo-4-(pyrrolidine-1-carbonyl)-hexahydro-pyrrolo[3,2-*b*]pyrrole-1-carbonyl]-butyl}-benzamide

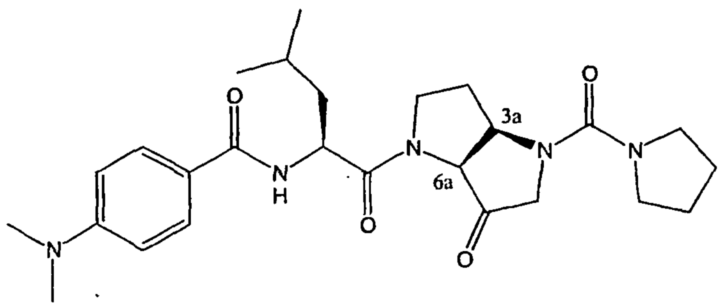

HPLC Rt = 11.06 mins (> 95%), HPLC-MS 484.1 [M + H]$^+$, 989.3 [2M + Na]$^+$.

EXAMPLE 236. (3a*R*, 6a*S*)-4-Dimethylamino-*N*-{(1*S*)-3-methyl-1-[6-oxo-4-(piperidine-1-carbonyl)-hexahydro-pyrrolo[3,2-*b*]pyrrole-1-carbonyl]-butyl}-benzamide

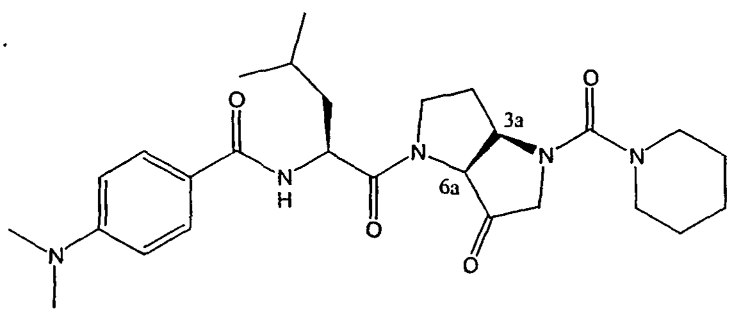

HPLC Rt = 12.21 mins (> 95%), HPLC-MS 498.2 [M + H]$^+$, 516.3 [M + H + $H_2O$]$^+$.

EXAMPLE 237. (3a*R*, 6a*S*)-4-Dimethylamino-*N*-{(1*S*)-3-methyl-1-[6-oxo-4-(pyrrolidine-3-carbonyl)-hexahydro-pyrrolo[3,2-*b*]pyrrole-1-carbonyl]-butyl}-benzamide

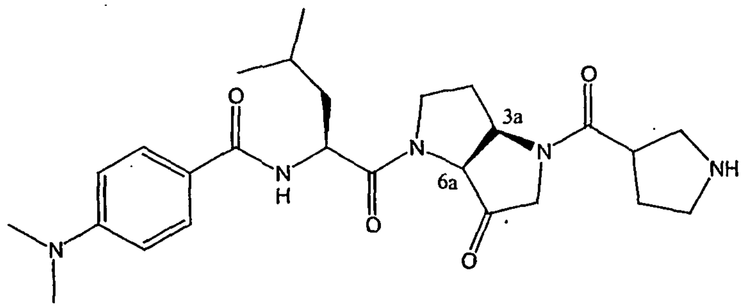

HPLC Rt = 8.13 mins (> 95%), HPLC-MS 484.1 $[M + H]^+$, 502.2 $[M + H + H_2O]^+$.

EXAMPLE 238. (3a*R*, 6a*S*)-4-Dimethylamino-*N*-{(1*S*)-3-methyl-1-[6-oxo-4-(tetrahydro-furan-2-carbonyl)-hexahydro-pyrrolo[3,2-*b*]pyrrole-1-carbonyl]-butyl}-benzamide

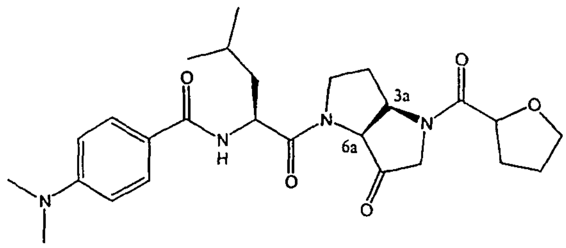

HPLC Rt = 9.89 mins (> 95%), HPLC-MS 485.1 $[M + H]^+$, 503.1 $[M + H + H_2O]^+$.

EXAMPLE 239. (3a*R*, 6a*S*)-4-Dimethylamino-*N*-{(1*S*)-3-methyl-1-[6-oxo-4-(tetrahydro-furan-3-carbonyl)-hexa-hydro-pyrrolo[3,2-*b*]pyrrole-1-carbonyl]-butyl}-benzamide

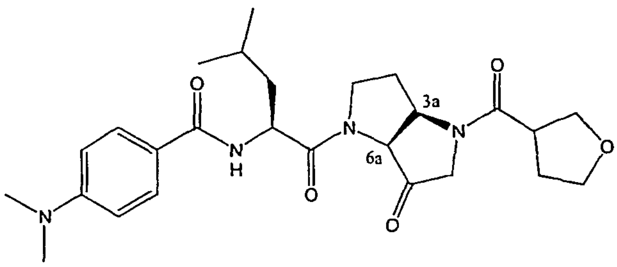

HPLC Rt = 10.40 mins (> 90%), HPLC-MS 485.1 $[M + H]^+$, 503.1 $[M + H + H_2O]^+$.

EXAMPLE 240. (3a*R*, 6a*S*)-4-Dimethylamino-*N*-{(1*S*)-3-methyl-1-[6-oxo-4-(2-piperidin-4-yl-acetyl)-hexahydro-pyrrolo[3,2-*b*]pyrrole-1-carbonyl]-butyl}-benzamide

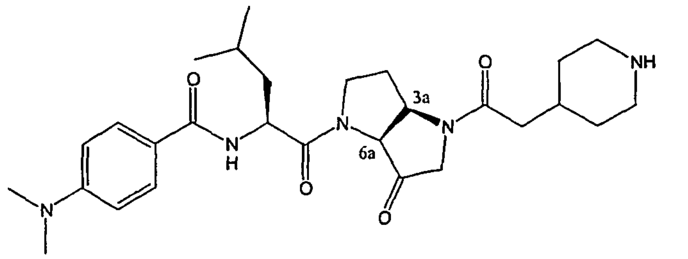

HPLC Rt = 8.8-9.6 mins (> 90%), HPLC-MS 512.2 $[M + H]^+$, 530.2 $[M + H + H_2O]^+$.

EXAMPLE 241. (3a*R*, 6a*S*)-4-Dimethylamino-*N*-{(1*S*)-3-methyl-1-[6-oxo-4-(2-piperazin-1-yl-acetyl)-hexahydro-pyrrolo[3,2-*b*]pyrrole-1-carbonyl]-butyl}-benzamide

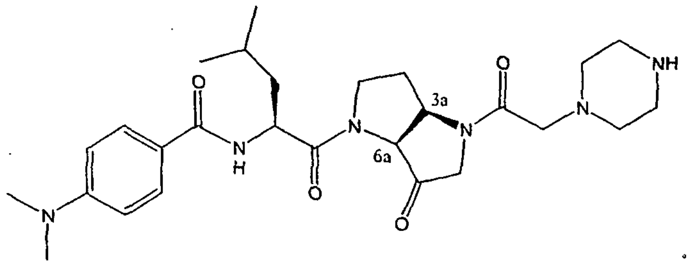

HPLC Rt = 7.3-8.2 mins (> 85%), HPLC-MS 513.1 $[M + H]^+$, 531.2 $[M + H + H_2O]^+$.

EXAMPLE 242. (3a*R*, 6a*S*)-4-Dimethylamino-*N*-{(1*S*)-3-methyl-1-[6-oxo-4-(piperdine-4-carbonyl)-hexahydro-pyrrolo[3,2-*b*]pyrrole-1-carbonyl]-butyl}-benzamide

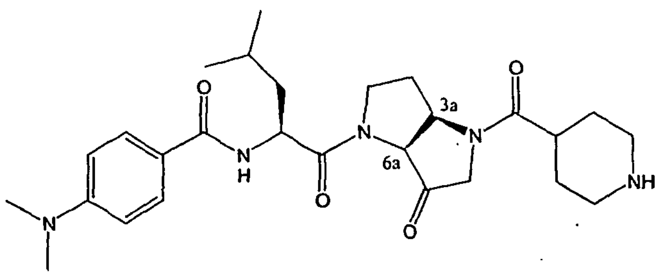

HPLC Rt = 8.2-8.9 mins (> 90%), HPLC-MS 498.2 $[M + H]^+$, 516.2 $[M + H + H_2O]^+$, 995.3 $[2M + H]^+$,

EXAMPLE 243. (3a*R*, 6a*S*)-*N*-{(1*S*)-1-[4-(Cyclopent-3-enecarbonyl)-6-oxo-hexahydro-pyrrolo[3,2-*b*]pyrrole-1-carbonyl]-3-methyl-butyl}-4-dimethylamino-benzamide

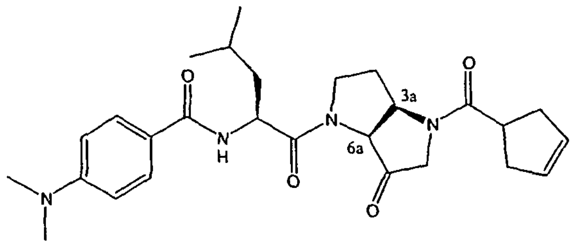

HPLC Rt = 11.39 mins (> 95%), HPLC-MS 481.2 $[M + H]^+$, 983.3 $[2M + Na]^+$.

EXAMPLE 244. (3a*R*, 6a*S*)-*N*-{(1*S*)-1-[4-(5-Chloro-furan-2-carbonyl)-6-oxo-hexahydro-pyrrolo[3,2-*b*]pyrrole-1-carbonyl]-3-methyl-butyl}-4-dimethylamino-benzamide

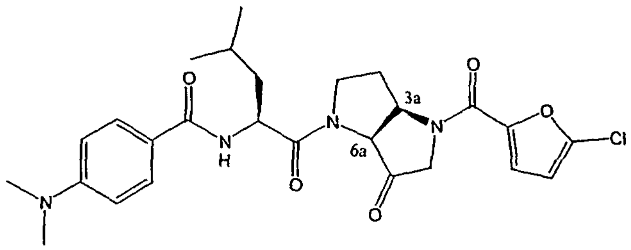

HPLC Rt = 13.17 mins (> 90%), HPLC-MS 515.1 $[M + H]^+$, 537.1 $[M + Na]^+$.

EXAMPLE 245. (3a*R*, 6a*S*)-*N*-{(1*S*)-1-[4-((2S)-2-Acetylamino-pentanoyl)-6-oxo-hexahydro-pyrrolo[3,2-*b*]pyrrole-1-carbonyl]-3-methyl-butyl}-4-dimethylamino-benzamide

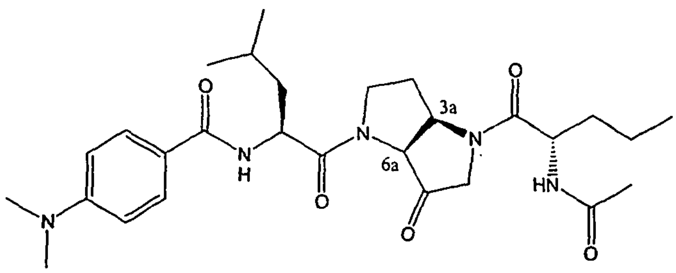

HPLC Rt = 11.18 mins (> 90%), HPLC-MS 528.2 $[M + H]^+$.

EXAMPLE 246. (3a*R*, 6a*S*)-3-Aminomethyl-*N*-{(1*S*)-1-[4-((2*S*)-2-amino-4-methyl-pentanoyl)-6-oxo-hexahydro-pyrrolo[3,2-*b*]pyrrole-1-carbonyl]-3-methyl-butyl}-benzamide

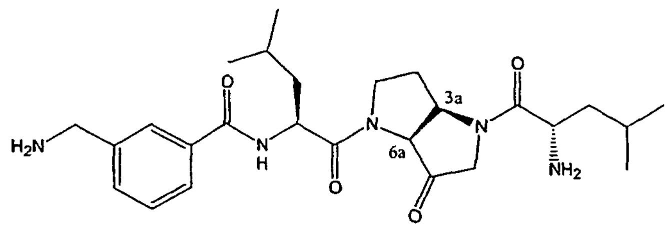

HPLC Rt = 8.87 mins (> 95%), HPLC-MS 486.2 $[M + H]^+$, 971.4 $[2M +H]^+$.

EXAMPLE 247. (3a*R*, 6a*S*)-*N*-[(1*S*)-1-(4-Benzoyl-6-oxo-hexahydro-pyrrolo[3,2-*b*] pyrrole-1-carbonyl)-3-methyl-butyl]-3-piperazin-1-yl-benzamide

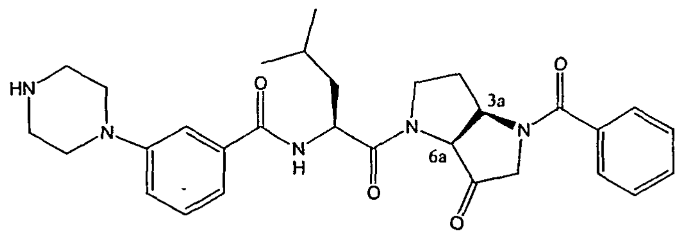

HPLC Rt = 10.92 mins (> 85%), HPLC-MS 532.1 $[M + H]^+$, 550.2 $[M + H + H_2O]^+$.

EXAMPLE 248. (3a*S*, 6a*S*)-*N*-[(1*S*)-1-(4-Benzoyl-6-oxo-hexahydro-2-oxa-1,4-diaza-pentalene-1-carbonyl)-3-methyl-butyl]-4-dimethylamino-benzamide

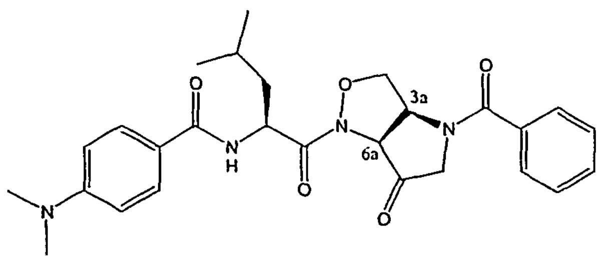

HPLC Rt =13.18 mins (> 95%), HPLC-MS 493.1 $[M + H]^+$, 511.1 $[M + H + H_2O]^+$.

EXAMPLE 248 was prepared following the general methods detailed for EXAMPLE 1, but using an alternative building block (3a*S*, 6a*S*)-6-oxo-tetrahydro-2-oxa-1,4-diazapentalene-1,4-dicarboxylic acid 4-*tert*-butyl ester 1-(9H-fluoren-9-ylmethyl) ester (91) prepared following general Scheme 5 and Scheme 22. Following solid phase synthesis, the crude product was purified by semi-preparative HPLC and lyophilised to give EXAMPLE 248.

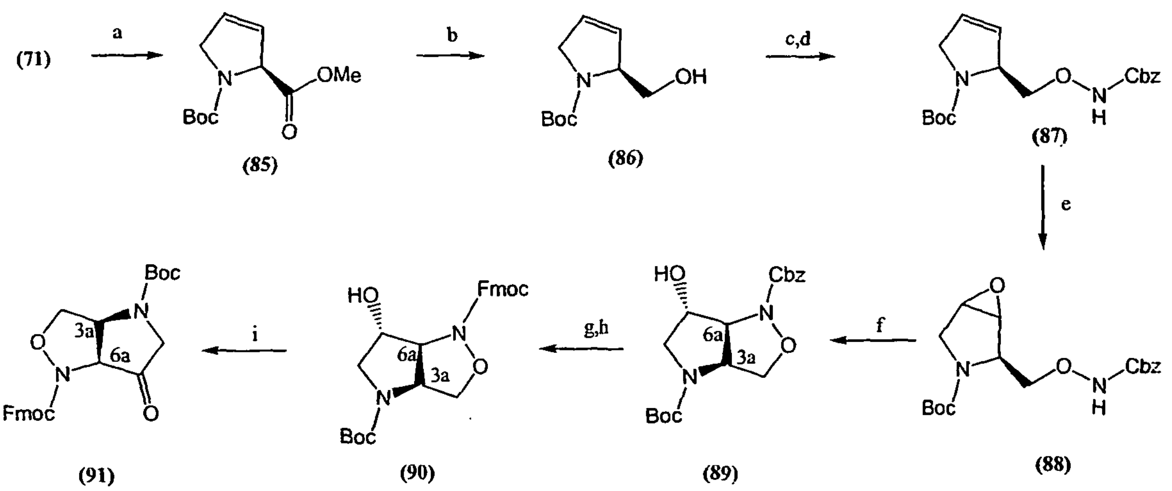


**Scheme 22.** (a) Ethereal $CH_2N_2$, -15 °C to RT. (b) $LiBR_4$, MeOH, THF or DIBAL-H, THF (c) Methanesulfonyl chloride, triethylamine, DCM (d) Cbz-NH-OH, NaH, 65 °C (e) *m*-Chloroperoxybenzoic acid, DCM. (f) Potassium carbonate, $CH_3CN$ (g) Pd-C, $H_2$, ethanol. (h) 1.05 eq Fmoc-Cl, 2.leq $Na_2CO_3$, 1,4-dioxane, water (i) Dess-Martin periodinane, DCM.

**Preparation of (*S*)-2,5-dihydropyrrole-1,2-dicarboxylic acid 1-*tert*-butyl ester 2-methyl ester (85)**

Ethereal diazomethane [~23 mmol generated from addition of diazald (7.1 g,) in diethyl ether (115 ml) onto sodium hydroxide (8.0 g) in water (14 ml) / ethanol (28 ml) at 65 °C] was added in portions to a stirred solution of (*S*)-2,5-dihydropyrrole-1,2-dicarboxylic acid 1-*tert*-butyl ester **(71)** (ex Bachem, 4.98 g, 23.4 mmol) in dichloromethane (100 ml) at 0 °C over 5 minutes. The solution was stirred for 1 hour at 0 °C then glacial acetic acid (0.5 ml) was added dropwise. The product was extracted into dichloromethane (50 ml) then washed with saturated aqueous sodium hydrogen carbonate solution (100 ml), water (100 ml) and brine (100 ml). The organic layer was dried ($Na_2SO_4$) and the solvents removed *in vacuo* to obtain (*S*)-2,5-dihydropyrrole-1,2-dicarboxylic acid 1-*tert*-butyl ester 2-methyl ester **(85)** (4.66 g, 88%), which was used without further purification. TLC (Single spot, $R_f$ = 0.25, EtOAc : heptane 1 : 4), HPLC-MS 172.1 [M + 2H - Bu]$^+$, 250.1 [M + Na]$^+$, 477.2 [2M + Na]$^+$; $C_{11}H_{17}NO_4.0.4H_2O$ req.(fnd.) % C 56.38 (56.47), % H 7.66 (7.25), % N 5.97 (5.88).

**Preparation of (*S*)-2-hydroxymethyl-2,5-dihydropyrrole-1-carboxylic acid *tert*-butyl ester (86)**

Methanol (1.66 ml, 41 mmol) was added dropwise to a stirred suspension of lithium borohydride (0.90 g, 41 mmol) in tetrahydrofuran (20 ml) over 2 minutes under an atmosphere of argon, followed by a solution of (S)-2,5-dihydropyrrole-1,2-dicarboxylic acid 1-*tert*-butyl ester 2-methyl ester **(85)** (4.65 g, 20.5 mmol) in tetrahydrofuran (50 ml) over 15 minutes. The mixture was stirred for 70 minutes then poured into water (125 ml). The product was extracted into dichloromethane (3x 100 ml), dried ($Na_2SO_4$), and the solvents removed *in vacuo*. The residue was purified by flash chromatography over silica eluting with ethyl acetate : heptane mixtures 15 : 85 to 25 : 75 to give (*S*)-2-hydroxymethyl-2,5-dihydropyrrole-1-carboxylic acid *tert*-butyl ester **(86)** as a colourless oil (3.75 g, 92%), $[a]_D^{22}$ -136° (c=1, $CHCl_3$). TLC (Single spot, $R_f$ = 0.30, EtOAc : heptane 2 : 3), HPLC-MS 222.1 [M + Na]$^+$, 421.1 [2M + Na]$^+$; $C_{10}H_{17}NO_3.0.3H_2O$ req.(fnd.) % C 58.72 (58.82), % H 8.67 (8.35), % N 6.85 (6.88); $d_H$ (500 MHz, $CDCl_3$) mixture of rotamers (major : minor = 4 : 1) 1.47 and 1.49 (9H total, each s, $(CH_3)_3C$), 3.55 (0.8H, ddd, $J$ = 11.1, 7.7, 1.1 Hz, $CH_2OH$ major), 3.61-3.66 (0.2H, m, $CH_2OH$ minor), 3.77 (1H, m, $CH_2OH$), 3.95-4.09 (1H, m, H-5), 4.14-4.19 (0.8H, m, H-5 major), 4.24-4.30 (0.2H, m, H-5 minor), 4.58 (0.2H, br. s, H-2 minor), 4.64 (1H, m, *OH*), 4.69-4.75 (0.8H, m, H-2 major), 5.57-5.63 and 5.78-5.82 (each 0.8H, m, H-3 and H-4 major), 5.65-5.70 and 5.89-5.94 (each 0.2H, m, H-3 and H-4 minor).

**Alternative preparation of (*S*)-hydroxymethyl-2,5-dihydropyrrole-1-carboxylic acid *tert*-butyl ester (86)**

A solution of diisobutylaluminium hydride (1.0M in tetrahydrofuran, 18.7 ml, 18.7 mmol) was added dropwise to a stirred solution of (*S*)-2,5-dihydropyrrole-1,2-dicarboxylic acid 1-*tert*-butyl ester 2-methyl ester **(85)** (ex Bachem, 1.06 g, 4.67 mmol) in tetrahydrofuran (15 ml) at -70 °C over 45 minutes under an atmosphere of argon. The mixture was stirred for 15 minutes at -70 °C then at ambient temperature for 3.25 hours before cooling to 0 °C and adding ethyl acetate (10 ml) dropwise followed by saturated aqueous sodium potassium tartrate solution (60 ml), ethyl acetate (65

ml) and brine (60 ml). The organic layer was separated then the aqueous layer extracted with ethyl acetate (60 ml). The organic layers were combined then washed with brine (50 ml), dried ($MgSO_4$), and solvents removed *in vacuo* to leave a residue which was purified by flash chromatography over silica eluting with ethyl acetate : heptane mixtures 15 : 85 to 25 : 75 to give (*S*)-hydroxymethyl-2,5-dihydropyrrole-1-carboxylic acid *tert*-butyl ester **(86)** as a colourless oil (0.34 g, 36%), $[a]_D^{22}$ -120.5° (c=1, $CHCl_3$). TLC (Single spot, $R_f$ = 0.30, EtOAc : heptane 2 : 3), analytical HPLC $R_t$ = 9.375 min; HPLC-MS 222.1 $[M + Na]^+$, 421.2 $[2M + Na]^+$.

**Preparation of (*S*)-(*N'*-benzyloxycarbonylaminooxymethyl)-2,5-dihydro pyrrole-1-carboxylic acid *tert*-butyl ester (87)**

i) Pyridine (7.6 ml, 94.2 mmol) was added to a solution of (*S*)-hydroxymethyl-2,5-dihydropyrrole-1-carboxylic acid *tert*-butyl ester **(86)** (3.75 g, 18.8 mmol) in dichloromethane whilst stirring at 0 °C followed by methanesulfonyl chloride (1.53 ml, 19.8 mmol) in portions over 10 minutes. The mixture was stirred for 1 hour at 0 °C then at ambient temperature for 14 hours. The product was extracted into dichloromethane (250 ml), washed with ice-chilled hydrochloric acid (1M, 2x 125 ml) and aqueous saturated sodium hydrogen carbonate solution (125 ml), dried ($Na_2SO_4$), and solvents removed *in vacuo* to leave (*S*)-methanesulfonyloxymethyl-2,5-dihydropyrrole-1-carboxylic acid *tert*-butyl ester as an oily residue (5.2 g, 100%) which was used without further purification. TLC (Single spot, $R_f$ = 0.20, EtOAc : heptane 3 : 7), analytical HPLC $R_t$ = 12.785 min; HPLC-MS 222.0 $[M + 2H - Bu]^+$, 577.1 $[2M + Na]^+$; $d_H$ (500 MHz, $CDCl_3$) mixture of rotamers (major : minor = 4 : 3) 1.45 and 1.49 (9H total, each s, $(CH_3)_3C$), 2.96 (3H, s, $SO_2CH_3$), 4.02-4.03 (0.43H, m, H-5 minor), 4.04-4.07 (0.57H, m, H-5 major), 4.12-4.57 (3H, m, H-5, $CH_2OS$), 4.67 (0.43H, br. s, H-2), 4.74 (0.57H, br. s, H-2), 5.73-5.98 (2H, m, H-3 and H-4); $d_c$ (125 MHz, $CDCl_3$) 28.41 ($C(\underline{C}H_3)_3$), 36.99, 37.46 ($S\underline{C}H_3$), 53.80, 53.95 (C-5), 62.90, 63.02 (C-2), 69.14, 69.34 ($\underline{C}H_2OS$), 80.12, 80.62 ($\underline{C}(CH_3)_3$), 126,08, 126.16 and 128.27, 128.36 (C-3 and C-4), 153.73, 154.13 (q, $N\underline{C}$=O).

ii) Sodium hydride (60% dispersion in oil, 3.0 g, 75.1 mmol) was added to a stirred solution of benzyl *N*-hydroxycarbamate (13.2 g, 78.8 mmol) in tetrahydrofuran (200 ml) at 0 °C in portions over 30 minutes under an atmosphere of argon. The mixture was stirred for 5 minutes at 0 °C then a solution of (*S*)-methanesulfonyloxymethyl-2,5-dihydropyrrole-1-carboxylic acid *tert*-butyl ester (5.2 g, 18.6 mmol, prepared as above) in tetrahydrofuran (175 ml) was added dropwise over 15 minutes. The resulting cloudy suspension was stirred for 1 hour at ambient temperature then at 65 °C for 4 hours, followed by 14 hours at ambient temperature then 7 hours at 65 °C. The product was extracted into dichloromethane (250 ml) then cautiously washed with water (250 ml). The aqueous layer was extracted with dichloromethane (250 ml) then the combined organic layers washed with water (3x 150 ml) and brine (250 ml), dried ($Na_2SO_4$), and the solvents removed *in vacuo*. The residue was purified twice by flash chromatography over silica eluting with ethyl acetate : heptane mixtures 25 : 75 to 30 : 70 then 0 : 100 to 25 : 75 to give (*S*)-(*N'*-benzyloxy carbonylaminooxymethyl)-2,5-dihydropyrrole-1-carboxylic acid *tert*-butyl ester **(87)** as a colourless oil (1.67 g, 26%) together with recovered (*S*)-methanesulfonyloxymethyl-2,5-dihydropyrrole-1-carboxylic acid *tert*-butyl ester (2.44 g, 47%). Data for (*S*)-(*N'*-benzyloxycarbonylaminooxymethyl)-2,5-dihydropyrrole-1-carboxylic acid *tert*-butyl ester **(87).** TLC (Single spot, $R_f$ = 0.35, EtOAc : heptane 2 : 3), analytical HPLC $R_t$ = 17.141 min; HPLC-MS 349.1 $[M + H]^+$, 371.1 $[M + Na]^+$, 719.2 $[2M + Na]^+$; Elemental analysis $C_{18}H_{24}N_2O_5$ req.(fnd.) % C 62.05 (62.18), % H 6.94 (7.05), % N 8.04 (7.90); HRMS $C_{18}H_{24}N_2O_5Na$ req. 371.1583, fnd. 371.1590 (1.83ppm); $d_H$ (500 MHz, $CDCl_3$) mixture of rotamers (major : minor = 2 : 1) 1.46 (9H, s, $(CH_3)_3C$), 3.67 (0.67H, dd, *J* = 11.45 and 7.6 Hz, $CH_2ONH$ major), 3.89 (0.33H, dd, *J* = 10.2 and 6.2 Hz, $CH_2ONH$ minor), 3.99 (0.67H, dd, *J* = 11.45 and 3.7 Hz, $CH_2ONH$ major), 3.95-4.10 (1H, m, H-5), 4.08-4.13 (0.33H, m, $CH_2ONH$ minor), 4.21 (0.67H, dd, *J* = 15.7 and 1.8 Hz, H-5 major), 4.20-4.26 (0.33H, m, H-5, minor), 4.63 (0.33H, br. s, H-2 minor), 4.85-4.90 (0.67H, m, H-2 m, major), 5.13-5.18 (2H, m, $OCH_2Ph$), 5.68-5.73 and 5.82-5.87 (2H, m, H-3 and H-4), 7.30-7.37 (5H, aromatics), 7.52 (0.33H, br. s, N*H*, minor), 8.69 (0.67H, br. s, N*H*, major); $d_c$ (125 MHz, $CDCl_3$) 28.40 ($C(\underline{C}H_3)_3$), 53.57, 53.78 (C-5), 62.18, 62.76 (C-2), 67.13, 67.55 ($O\underline{C}H_2Ph$), 77.27, 77.86 ($\underline{C}H_2ONH$), 80.07 ($\underline{C}(CH_3)_3$), 126.62, 126.72, 127.47 , 127.85, 128.12, 128.24, 128.50, 128.54, 128.58 (C-3, C-4, aromatic $\underline{C}H$), 135.48, 135.87, 136.18 (aromatic quaternary), 154.04, 155.24 ($CH_2N\underline{C}$=O), 156.94, 157.39 ($ON\underline{C}$=O).

**Preparation of (2*R*)-(*N'*-benzyloxycarbonylaminooxymethyl)-6-oxa-3-aza bicyclo[3.1.0]hexane-3-carboxylic acid *tert*-butyl ester (88)**

*meta*-Chloroperoxybenzoic acid (57-86%, 1.9g, ~7.7 mmol) was added in portions under an atmosphere of argon over 15 minutes to a stirred solution of (*S*)-(*N'*-benzyloxycarbonylaminooxymethyl)-2,5-dihydropyrrole-1-carboxylic acid *tert*-butyl ester **(87)** (600 mg, 1.72 mmol) in dichloromethane (12 ml). The mixture was stirred for 14 hours then the

product was extracted into dichloromethane (50 ml), washed with aqueous saturated sodium hydrogen carbonate solution (2x 30 ml), dried ($Na_2SO_4$), and the solvents removed *in vacuo*. The residue was purified by flash chromatography over silica eluting with ethyl acetate : heptane 0 : 100 to 30 : 70 to give (2*R*)-(*N'*-benzyloxycarbonylaminooxymethyl)-6-oxa-3-azabicyclo[3.1.0]hexane-3-carboxylic acid *tert*-butyl ester **(88)** as a colourless oil (390 mg, 62%). TLC (Single spot, $R_f$ = 0.35, EtOAc : heptane 2 : 3), analytical HPLC $R_t$ = 15.733 min; HPLC-MS 265.1 [M+ 2H - Boc]$^+$, 309.0 [M + 2H - Bu]$^+$, 387.1 [M + Na]$^+$, 751.2 [2M + Na]$^+$; $C_{18}H_{24}N_2O_6$.0.4$H_2O$ req.(fnd.) % C 58.21 (58.24), % H 6.73 (6.62), % N 7.54 (7.57); HRMS $C_{18}H_{24}N_2O_6Na$ req. 387.1532, fnd. 387.1534 (0.42ppm).

**Preparation of (3a*S*, *6S*, 6a*S*)-6-hydroxytetrahydro-2-oxa-1,4-diazapentalene-1,4-dicarboxylic acid 1-benzyl ester 4-*tert*-butyl ester (89)**

Potassium carbonate (1.06 g, 7.7 mmol) was added to a stirred solution of (2R)-(*N'*-benzyloxycarbonylaminooxymethyl)-6-oxa-3-azabicyclo[3.1.0]hexane-3-carboxylic acid *tert*-butyl ester **(88)** (280 mg, 0.77 mmol) in acetonitrile (4 ml) under an atmosphere of argon. The suspension was stirred for 5 hours then the product was extracted into dichloromethane (30 ml) and washed with water (10 ml). The aqueous layer was extracted with dichloromethane (10 ml) then the combined organic layers washed with water (10 ml), dried ($Na_2SO_4$), and the solvents removed *in vacuo*. The residue was purified by flash chromatography over silica eluting with ethyl acetate : heptane mixtures 0 : 100 to 30 : 70 to give (3a*S*, 6*S*, 6a*S*)-6-hydroxytetrahydro-2-oxa-1,4-diazapentalene-1,4-dicarboxylic acid 1-benzyl ester 4-*tert*-butyl ester **(89)** as a colourless oil (141 mg, 50%) together with recovered (1*S*, 2R, 5*R*)-2-(*N'*-benzyloxycarbonylaminooxymethyl)-6-oxa-3-azabicyclo[3.1.0]hexane-3-carboxylic acid *tert*-butyl ester **(88)** as a colourless oil (71 mg, 25%). Data for (3a*S*, 6*S*, 6a*S*)-6-hydroxytetrahydro-2-oxa-1,4-diazapentalene-1,4-dicarboxylic acid 1-benzyl ester 4-*tert*-butyl ester **(89).** TLC (Single spot, $R_f$ = 0.20, EtOAc : heptane 2 : 3), analytical HPLC $R_t$ = min; HPLC-MS 265.1 [M + 2H - Boc]$^+$, 309.1 [M + 2H - Bu]$^+$, 751.2 [2M + Na]$^+$; HRMS $C_{18}H_{24}N_2O_6Na$ req. 387.1532, fnd. 387.1529 (-0.87ppm); $d_H$ (500 MHz, $CDCl_3$) mixture of rotamers, *tentative proton assignment*, 1.45 (9H, s, C(C$H_3$)$_3$), 2.28 (1H, d, *J* = 3.9 Hz, O*H*), 3.45-4.81 (7H, m, BocNCHC$H_2$, BocNC*H*, BocNC$H_2$, C*H*OH, CbzNC*H*), 5.12-5.26 (2H, m, OC$H_2$Ph), 7.32-7.42 (5H, aromatics). Data for (1*S*, 2*R*, 5*R*)-2-(*N'*-benzyloxycarbonylaminooxymethyl)-6-oxa-3-azabicyclo[3.1.0]hexane-3-carboxylic acid *tert*-butyl ester **(88).** TLC (Single spot, $R_f$ = 0.35, EtOAc : heptane 40 : 60), HPLC-MS 265.1 [M + 2H - Boc]$^+$, 309.1 [M + 2H - Bu]$^+$, 387.1 [M + Na]$^+$, 751.2 [2M + Na]$^+$; $d_H$ (500 MHz, $CDCl_3$) mixture of rotamers, *tentative proton assignment*, 1.41 (9H, s, C(C$H_3$)$_3$), 3.39-3.72 (3H, m, H-2 and H-5), 3.90-4.41 (4H, m, H-3, H-4 and C$H_2$ON), 5.12-5.20 (2H, m, OC$H_2$Ph), 7.31-7.39 (5H, aromatics), 7.60 and 8.0 (0.8H total, each br. s, N*H*).

**Preparation of (3a*S*, 6*S*, 6a*S*)-6-hydroxytetrahydro-2-oxa-1,4-diazapentalene-1,4-dicarboxylic acid 4-*tert*-butyl ester 1-(9H-fluoren-9-ylmethyl) ester (90)**

Ethanol (2.5 ml) was cautiously added to a stirred mixture of (3a*S*, 6*S*, 6aS)-6-hydroxytetrahydro-2-oxa-1,4-diazapentalene-1,4-dicarboxylic acid 1-benzyl ester 4-*tert*-butyl ester **(89)** (43 mg, 0.118 mmol) and 10% palladium on charcoal (5 mg) under an atmosphere of argon at 0 °C. The argon was replaced by hydrogen then the suspension was stirred at ambient temperature for 45 minutes then the hydrogen was replaced by argon before filtering the mixture through celite in *vacuo*. The filter cake was washed with ethanol (25 ml) then solvents removed *in vacuo* from the filtrate to leave (3a*S*, 6*S*, 6a*S*)-6-hydroxyhexahydro-2-oxa-1,4-diazapentalene-4-carboxylic acid *tert*-butyl ester as an oily residue (28 mg), which was used without further purification. HPLC-MS 175.1 [M + 2H - Bu]$^+$, 483.2 [2M + Na]$^+$. A solution of sodium carbonate (31 mg, 0.295 mmol) in water (1.75 ml) was added whilst stirring to a solution of (3a *S*, 6*S*, 6a*S*)-6-hydroxyhexahydro-2-oxa-1,4-diazapentalene-4-carboxylic acid *tert*-butyl ester (28 mg) in 1,4-dioxane (1.0 ml). The mixture was cooled to 0 °C then a solution of Fmoc-Cl (34 mg, 0.132 mmol) in 1,4-dioxane (0.75 ml) was added dropwise over 40 minutes. The mixture was stirred at 0 °C for 2.25 hours then at ambient temperature for 30 minutes. Water (20 ml) was added then the product extracted into dichloromethane (3x 15 ml), dried ($Na_2SO_4$), and the solvents removed *in vacuo*. The residue was purified by flash chromatography over silica eluting with ethyl acetate : heptane mixtures 0 : 100 to 30 : 70 to give (3a*S*, 6*S*, 6a*S*)-6-hydroxytetrahydro-2-oxa-1,4-diazapentalene-1,4-dicarboxylic acid 4-*tert*-butyl ester 1-(9H-fluoren-9-ylmethyl) ester **(90)** as a white solid (40 mg, 75%). TLC (Single spot, $R_f$ = 0.20, EtOAc : heptane 3 : 7), analytical HPLC $R_t$ = 18.217 min; HPLC-MS 475.1 [M + Na]$^+$, 927.2 [2M + Na]$^+$; $C_{25}H_{28}N_2O_6$.0.5EtOAc req.(fnd.) % C 65.35 (64.85), % H 6.50 (6.21), % N 5.64 (5.66).

**Preparation of (3a*S*, 6a*S*)-6-oxo-tetrahydro-2-oxa-1,4-diazapentalene-1,4-dicarboxylic acid 4-*tert*-butyl ester 1-(9H-fluoren-9-ylmethyl) ester (91)**

Dess-Martin periodinane (73 mg, 0.170 mmol) was added to a stirred solution of (3a*S*, 6*S*, 6a*S*)-6-hydroxytetrahydro-2-oxa-1,4-diazapentalene-1,4-dicarboxylic acid 4-*tert*-butyl ester 1-(9H-fluoren-9-ylmethyl) ester **(90)** (39 mg, 0.086 mmol) in dichloromethane (1.25 ml). The mixture was stirred for 2.5 hours, stored at -80 °C for 14 hours, and then

purified by flash chromatography over silica eluting with ethyl acetate : heptane mixtures 5 : 95 to 15 : 85 to give (3a*S*, 6a*S*)-6-oxo-tetrahydro-2-oxa-1,4-diazapentalene-1,4-dicarboxylic acid 4-*tert*-butyl ester 1-(9H-fluoren-9-ylmethyl) ester **(91)** as a white solid (31 mg, 80%). TLC (Single spot, $R_f$ = 0.30, EtOAc : heptane 2 : 3), analytical HPLC broad peak $R_t$ = 19.57-22.15 min; HPLC-MS single broad main UV peak, 473.1 $[M + Na]^+$, 491.1 $[M + H_2O + Na]^+$, 923.1 $[2M + Na]^+$, 959.1 $[2M + 2H_2O + Na]^+$; HRMS $C_{25}H_{26}N_2O_6Na$ req. 473.1689, fnd. 473.1690 (0.24ppm); $d_H$ (500 MHz, $CDCl_3$) mixture of rotamers major : minor 1.5 : 1, 1.48 (5.4H, s, $C(CH_3)_3$ major), 1.50 (3.6H, s, $C(CH_3)_3$ minor), 3.49-3.58 (1H, m, BocNCHC$H_2$), 3.78-3.92 (2H, m, BocNC$H_2$), 4.13 (0.4H, d, $J$ = 9.5Hz, BocNCHC$H_2$ [minor]), 4.20-4.29 (1.6H, m, Fmoc-C*H* and BocNCHC$H_2$ [major]), 4.46-4.52 (1H, m, Fmoc-C$H_2$), 4.60-4.74 (2.4H, m, Fmoc-C$H_2$, FmocNC*H*, BocNC*H* [minor]), 4.83 (0.6H, dd, $J$ = 7.5 and 4.3Hz, BocNC*H* [major]), 7.29-7.78 (8H, aromatic); $d_C$ (125 MHz, $CDCl_3$) 28.38, 28.31 ($C(\underline{C}H_3)_3$), 46.96, 47.05 (Fmoc-$\underline{C}$H), 52.40, 52.93 (BocN$\underline{C}H_2$), 61.95 (BocN$\underline{C}$H), 64.48, 65.31 (FmocN$\underline{C}$H), 68.59, 68.76 (Fmoc-$\underline{C}$H2), 77.17, 77.31 (BocNCH$\underline{C}H_2$), 81.61 ($\underline{C}(CH_3)_3$),120.02, 125.11, 125.35, 127.21, 127.28, 127.98 (Fmoc aromatic $\underline{C}$H), 141.29, 141.33, 143.04, 143.12 (Fmoc quaternary), 153.09, 154.00 (Boc $\underline{C}$=O), 157.64 (Fmoc $\underline{C}$=O), 204.85, 205.44 ($\underline{C}$=O).

Broadly following the general details from Scheme 6, the required bicycle building block (3a*S*, 6a*S*)-6-oxo-tetrahydro-2-oxa-1,4-diazapentalene-1,4-dicarboxylic acid 4-*tert*-butyl ester 1-(9H-fluoren-9-ylmethyl) ester **(91)** was converted to the corresponding equivalent of building block-linker construct **(27)** (where $Pg_2$ is *tert*-butoxycarbonyl) as follows:

A solution of sodium acetate trihydrate (24 mg, 0.173 mmol) in water (0.25 ml) was added to a solution of (3a*S*, 6a*S*)-6-oxo-tetrahydro-2-oxa-1,4-diazapentalene-1,4-dicarboxylic acid 4-*tert*-butyl ester 1-(9H-fluoren-9-ylmethyl) ester **(91)** (26 mg, 0.058mmol) and 4-[[(hydrazinocarbonyl)amino]methyl]cyclohexane carboxylic acid. trifluoroacetate (Murphy, A. M., et al, J. Am. Chem. Soc, 114, 3156-3157, 1992**)** (38 mg, 0.116 mmol) in ethanol (1.75 ml). The reaction heated at 75 °C in a sealed tube for 1.5 hour. The product was extracted into chloroform (50 ml) then washed with hydrochloric acid (0.1M, 2 x 25 ml), saturated aqueous sodium chloride solution (30 ml) then dried ($Na_2SO_4$) and the solvent removed *in vacuo* to leave the product as a white solid (37 mg, ~100%). Analytical HPLC has main UV peaks with Rt = 20.223 and 21.596mins and HPLC-MS (main UV peaks each with 648.2 $[M+H]^+$).

Following the general details from Scheme 6, the corresponding building block-linker construct was attached to the solid phase providing loaded building block-linker construct following standard loading protocols and indicated quantitative loading.

EXAMPLES 249a to 249c were prepared entirely by solution phase synthesis methods (broadly defined by the general strategy detailed in Scheme 4) following Schemes 23 to 25 and have utility as inhibitors of cathepsin K with Ki < 1000nM.

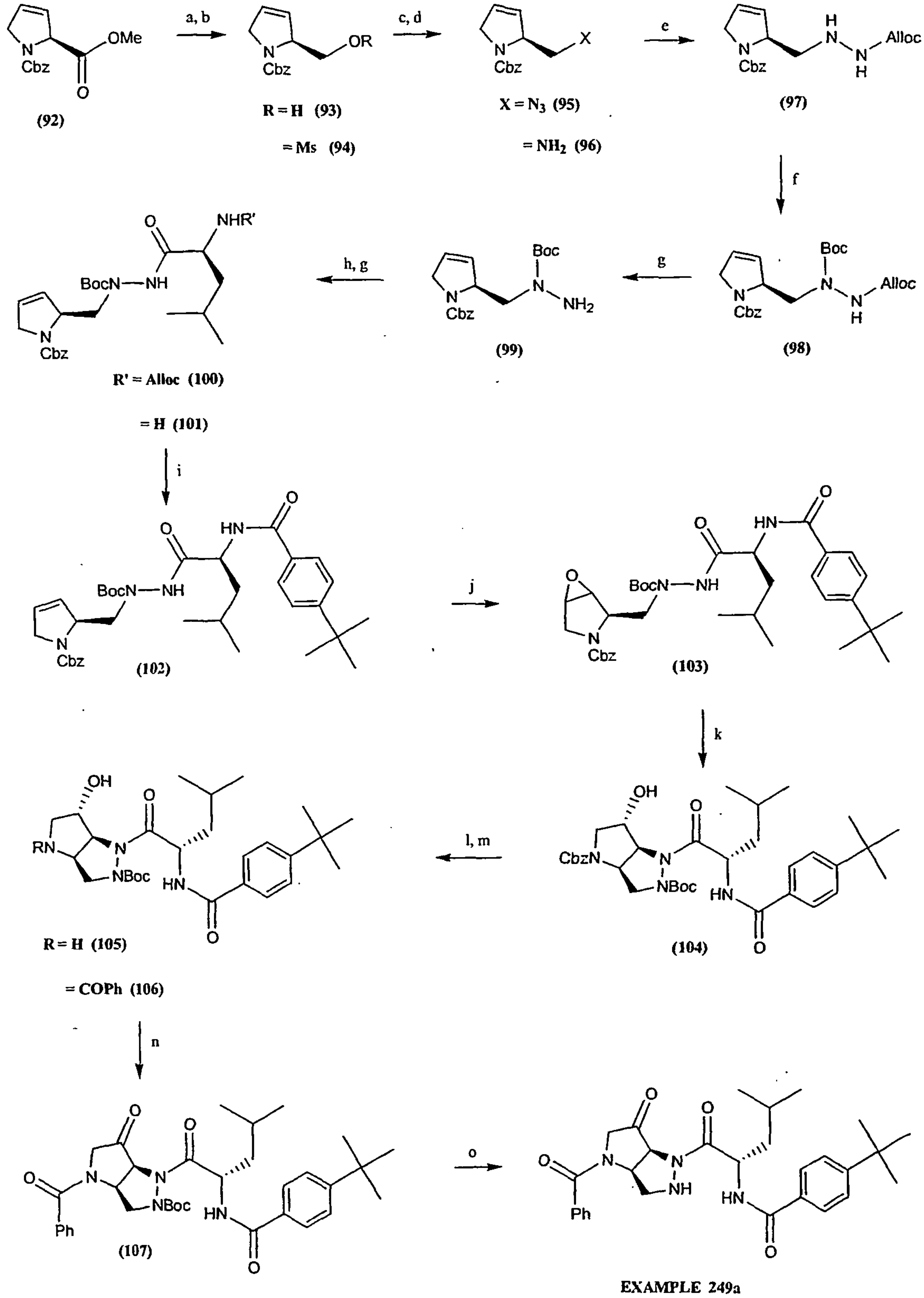


(92)

R = H (93)

= Ms (94)

X = $N_3$ (95)

= $NH_2$ (96)

(97)

(98)

(99)

R' = Alloc (100)

= H (101)

(102)

(103)

(104)

R = H (105)

= COPh (106)

(107)

EXAMPLE 249a

**Scheme 23.** (a) DIBAL-H, THF or $LiBH_4$, MeOH, THF (b) Methanesulfonyl chloride, triethylamine, DCM (c) Sodium azide, DMF, 110°C (d) $Ph_3P$ / $H_2O$, 1,4-dioxane, 50°C (e) 3-Phenyloxaziridine-2-carboxylic acid allyl ester, DCM (f) $(Boc)_2O$, DCM, 60°C (g) $Pd(PPh_3)_4$, $PhSiH_3$, DCM (h) Alloc-Leu-F, DMF (i) 4-*tert*-Butylbenzoic acid, HBTU, HOBT, NMM, DMF (j) *m*-Chloroperoxybenzoic acid, DCM. (k) Potassium carbonate, $CH_3CN$, 60°C (l) Pd-C, $H_2$, ethanol (m) $(PhCO)_2O$, DMF (n) Dess-Martin periodinane, DCM (o) TFA, DCM

**Preparation of (*S*)-2,5-dihydropyrrole-1,2-dicarboxylic acid 1-benzyl ester 2-methyl ester (92)**

Carbonic acid benzyl ester 2,5-dioxopyrrolidin-1-yl ester (8.45 g, 33.9 mmol) then triethylamine (10.8 ml, 77 mmol) were added dropwise to a stirred solution of (*S*)-2,5-dihydropyrrole-1,2-dicarboxylic acid 1-benzyl ester 2-methyl ester hydrochloride (5.0 g, 30.6 mmol) and THF : water (1 : 1, 306 ml) at 0 °C. The mixture was stirred at ambient temperature for 12 hours then half of the solvent was removed *in vacuo*. The product was extracted into *tert*-butyl methyl ether (3 x 100 ml) then the combined organic layers were washed with 5% hydrochloric acid (100 ml), 5% aqueous sodium hydrogen carbonate solution (100 ml) and brine (100 ml), dried ($MgSO_4$), and the solvents removed *in vacuo*. The residue was purified by flash chromatography over silica eluting with heptane : *tert*-butyl methyl ether 2 : 1 to give (*S*)-2,5-dihydro pyrrole-1,2-dicarboxylic acid 1-benzyl ester 2-methyl ester **(92)** as a pale yellow oil (7.9 g, 99%). TLC ($R_f$ = 0.30, heptane : *tert*-butyl methyl ether 1 : 1), analytical HPLC single peak with $R_t$ = 13.935 min, HPLC-MS 262.0 $[M + H]^+$, 284.0 $[M + Na]^+$, 545.1 $[2M + Na]^+$. $\delta_H$ (500 MHz, $CDCl_3$) approximately 1 : 1 mixture of rotamers, 3.57 and 3.64 (3H, each s, $OCH_3$), 4.22-4.36 (2H, m, H-5), 5.03-5.14 (3H, m, $OCH_2Ph$ and H-2), 5.69-5.78 and 5.92-5.99 (2H, each m, H-3 and H-4), 7.29-7.39 (5H, aromatics); $\delta_C$ (125 MHz, $CDCl_3$) 52.25 and 52.42 ($O\underline{C}H_3$), 53.36 and 53.85 (C-5), 66.25 and 66.56 (C-2), 67.09 and 67.16 ($Ph\underline{C}H_2O$), 124.66, 127.80, 127.91, 127.98, 128.03, 128.40, 128.46, 129.09 and 129.18 (C-3, C-4 and aromatic CH), 136.43 and 136.51 (aromatic quaternary), 153.91 and 154.36 ($N\underline{C}=O$), 170.38 and 170.62 ($CH\underline{C}=O$).

**Preparation of (*S*)-2-hydroxymethyl-2,5-dihydropyrrole-1-carboxylic acid benzyl ester (93)**

Diisobutylaluminium hydride (1.5M in toluene, 2.62 ml, 3.93 mmol) was added dropwise over 20 minutes to a stirred solution of (*S*)-2,5-dihydropyrrole-1,2-dicarboxylic acid 1-benzyl ester 2-methyl ester **(92)** (0.41 g, 1.57 mmol) in THF (15 ml), at -78 °C under an atmosphere of argon. The mixture was stirred for 2 hours at -78 °C then at ambient temperature for 18 hours. Saturated aqueous potassium sodium tartrate solution (40 ml) was added slowly to the mixture, followed by ethyl acetate (40ml) and magnesium sulphate ~5 *g*. The resultant slurry was vigorously stirred for 2 hours, then filtered and the filter cake washed with ethyl acetate. The filtrate was concentrated *in vacuo*. The residue was purified by flash chromatography over silica eluting with ethyl acetate : heptane 1 : 4 to give (*S*)-2-hydroxymethyl-2,5-dihydropyrrole-1-carboxylic acid benzyl ester (93) as a pale yellow oil (130 mg, 36%). TLC ($R_f$ = 0.30, heptane : ethyl acetate 1 : 1), analytical HPLC single peak with $R_t$ = 11.033 min, HPLC-MS 234.1 $[M + H]^+$, 256.0 $[M + Na]^+$, 489.1 $[2M + Na]^+$. $d_H$ (500 MHz, $CDCl_3$) approximately 4 : 1 mixture of rotamers, 3.58-3.66 (1H, m, $CH_2OH$), 3.77-3.85 (1H, m, $CH_2OH$), 4.14-4.32 (3H, m, $CH_2OH$ and H-5), 4.63-4.68 (0.2H, br. s, H-2 minor), 4.76-4.81 (0.8H, m, H-2 major), 5.14-5.21 (2H, m, $OCH_2Ph$), 5.63-5.66 and 5.81-5.85 (1.6H, m, H-3 and H-4 major), 5.69-5.73 and 5.90-5.96 (0.4H, m, H-3 and H-4 minor), 7.29-7.39 (5H, aromatics); $d_C$ (125 MHz, $CDCl_3$) 53.98 (major) and 54.59 (minor) (C-5), 64.27 (minor) and 66.65 (major) ($\underline{C}H_2OH$), 66.11 (minor) and 68.08 (major) (C-2), 67.41 ($Ph\underline{C}H_2O$), 126.67, 126.70, 126.96, 127.19, 127.40, 127.62, 127.95, 128.17, 128.54 and 128.60 (C-3, C-4 and aromatic CH), 136.30 (aromatic quaternary), 156.68 ($N\underline{C}=O$).

**Alternative preparation of (*S*)-2-hydroxymethyl-2,5-dihydropyrrole-1-carboxylic acid benzyl ester (93)**

Methanol (2.43 ml) followed by a solution of (*S*)-2,5-dihydropyrrole-1,2-dicarboxylic acid 1-benzyl ester 2-methyl ester **(92)** (7.90 g, 30.2 mmol) in THF (125 ml) were added dropwise to a stirred suspension of lithium borohydride (1.32 g, 60.5 mmol) in THF (45 ml). The mixture was stirred for 1 hour then water (10 ml) cautiously added dropwise. The product was extracted into *tert*-butyl methyl ether (3 x 100 ml) then the combined organic layers dried ($MgSO_4$), and the solvents removed *in vacuo*. The residue was purified by flash chromatography over silica eluting with heptane : ethyl acetate 4 : 1 to give (*S*)-2-hydroxymethyl-2,5-dihydropyrrole-1-carboxylic acid benzyl ester **(93)** as a pale yellow oil (6.38 g, 90%). TLC ($R_f$ = 0.30, heptane : ethyl acetate 1 : 1), analytical HPLC single peak with $R_t$ = 11.036 min, HPLC-MS 234.1 $[M + H]^+$, 256.0 $[M + Na]^+$, 489.1 $[2M + Na]^+$.

**Preparation of (*S*)-2-methanesulfonyloxymethyl-2,5-dihydropyrrole-1-carboxylic acid benzyl ester (94)**

Triethylamine (337 µl, 2.4 mmol) was added dropwise to a stirred solution of (*S*)-2-hydroxymethyl-2,5-dihydropyrrole-1-carboxylic acid benzyl ester **(93)** (0.35 g, 1.50 mmol) and methanesulfonyl chloride (174 µl, 2.25 mmol) in dichloromethane (10 ml) at 0 °C. The mixture was stirred for 30 minutes then washed with water (10 ml) and brine (10 ml), dried ($Na_2SO_4$), and the solvents removed *in vacuo* to give (*S*)-2-methanesulfonyloxymethyl-2,5-dihydropyrrole-1-carboxylic acid benzyl ester **(94)** as a pale yellow oil (443 mg, 95%) which was used without further purification. TLC ($R_f$ = 0.30, heptane : ethyl acetate 1 : 1), analytical HPLC single peak with $R_t$ = 14.115 min, HPLC-MS 312.0 $[M + H]^+$, 334.0 $[M + Na]^+$, 645.1 $[2M + Na]^+$.

**Preparation of (*S*)-2-azidomethyl-2,5-dihydropyrrole-1-carboxylic acid benzyl ester (95)**

Sodium azide (8.89 g, 137 mmol) was added to a stirred solution of (*S*)-2-methanesulfonyloxymethyl-2,5-dihydropyrrole-1-carboxylic acid benzyl ester **(94)** (8.52 g, 27.4 mmol) in DMF (150 ml). The reaction mixture was stirred at 110 °C for 1 hour. The solvent was removed *in vacuo* then the product extracted into ethyl acetate (300 ml), washed with water (300 ml), brine (200 ml), dried ($MgSO_4$), and the solvents removed *in vacuo*. The residue was purified by flash chromatography over silica eluting with heptane : ethyl acetate 9 : 1 to give (*S*)-2-azidomethyl-2,5-dihydropyrrole-1-carboxylic acid benzyl ester **(95)** as a pale yellow oil (5.05 g, 72 %). TLC ($R_f$ = 0.65, heptane : ethyl acetate 1 : 1), analytical HPLC single peak with $R_t$ = 17.855 min, HPLC-MS 259.0 $[M + H]^+$, 281.0 $[M + Na]^+$, 539.1 $[2M + Na]^+$.

**Preparation of (*S*)-2-aminomethyl-2,5-dihydropyrrole-1-carboxylic acid benzyl ester (96)**

Triphenylphosphine (3.20 g, 12.2 mmol) was added to a stirred solution of (*S*)-2-azidomethyl-2,5-dihydropyrrole-1-carboxylic acid benzyl ester **(95)** (2.10 g, 8.13 mmol) in THF (170 ml) containing water (2 ml). The mixture was stirred at 50 °C for 2.5 hours then at ambient temperature 16 hours. The were solvents removed *in vacuo* then the residue was purified by flash chromatography over silica eluting with dichloromethane : methanol 99 : 1 to 95 : 5 mixtures to give (*S*)-2-aminomethyl-2,5-dihydropyrrole-1-carboxylic acid benzyl ester **(96)** as a pale yellow oil (2.15 g) which was contaminated with triphenylphosphine oxide. The pale yellow oil was dissolved in *tert*-butyl methyl ether (15 ml) then cooled to 0 °C before adding HCl in 1,4-dioxane (4M, 5 ml) followed by iced-water (20ml). The aqueous layer was extracted with *tert*-butyl methyl ether (3 x 20 ml), then the pH adjusted to ~12 using 1M aqueous sodium hydroxide solution. The product was then extracted into dichloromethane (3 x 50 ml) and the combined dichloromethane layers were dried ($MgSO_4$), and the solvents removed *in vacuo* to give (*S*)-2-aminomethyl-2,5-dihydropyrrole-1-carboxylic acid benzyl ester **(96)** as a pale yellow oil (1.40 g, 74%). TLC ($R_f$ = 0.20, methanol : dichloromethane 1 : 9), HPLC-MS 233.1 $[M + H]^+$, 255.1 $[M + Na]^+$, 487.1 $[2M + Na]^+$. $d_H$ (500 MHz, $D_6$-DMSO) 1.30-2.10 (1H, br. s, $CH_2NH_2$), 3.00-3.70 (1H, br. s, $CH_2NH_2$), 2.64-2.75 (1H, m, $CH_2NH_2$), 2.79-2.88 (1H, m, $CH_2NH_2$), 4.00-4.21 (2H, m, H-5), 4.42-4.47 (1H, m, H-2), 5.05-5.16 (2H, m, $PhCH_2O$), 5.84-6.01 (2H, m, H-4 and H-5), 7.31-7.40 (5H, aromatics); $d_C$ (125 MHz, $D_6$-DMSO) 43.91 and 44.85 ($\underline{C}H_2NH_2$), 53.86 and 54.47 (C-5), 65.81 and 65.99 ($Ph\underline{C}H_2O$), 66.41 and 67.17 (C-2), 126.30, 126.36, 127.55, 127.58, 127.84, 127.86, 128.48, 128.51, 128.83 and 129.06 (C-3, C-4 and aromatic $\underline{C}H$), 137.13 and 137.17 (aromatic quaternary), 153.82 and 153.97 ($N\underline{C}$=O).

**Preparation of (*S*)-2-(*N'*-allyloxycarbonylhydrazinomethyl)-2,5-dihydro pyrrole-1-carboxylic acid benzyl ester (97)**

i) Preparation of 3-phenyloxaziridine-2-carboxylic acid allyl ester
Ice-chilled sodium hydroxide (2M, 50 ml, 100 mmol) was added to a vigorously stirred solution of benzaldehyde (5.3 g, 50 mmol) in diethyl ether (50 ml) at ambient temperature, then ice-chilled solutions of hydroxylamine-*O*-sulfuric acid (6.0 g, 53 mmol) in water (50 ml) and sodium hydroxide (2M, 25 ml, 50 mmol) were added simultaneously over 20 minutes. Allyl chloroformate (5.31 ml, 50 mmol) was added dropwise over 5 minutes then the mixture was stirred at 0 °C for 10 minutes before separating the ethereal layer. The aqueous phase was extracted with diethyl ether (2 x 25 ml), then the combined organic layers stirred at 0 °C for 10 minutes with a solution of hydroxylamine-*O*-sulfuric acid (2.5 g, 21 mmol) in water (25 ml). The phases were separated, then the aqueous phase extracted with diethyl ether (2 x 25 ml). The combined ethereal layers were dried ($MgSO_4$), and the solvents removed *in vacuo*. The brown oily residue was purified by flash chromatography over silica eluting with heptane : ethyl acetate 9 : 1 to give 3-phenyloxaziridine-2-carboxylic acid allyl ester as a pale yellow oil (1.44 g, 14%). TLC ($R_f$ = 0.7, heptane : ethyl acetate 1 : 1), HPLC-MS 206.0 $[M + H]^+$, 228.1 $[M + Na]^+$, 433.0 $[2M + Na]^+$.

ii) 3-Phenyloxaziridine-2-carboxylic acid allyl ester (prepared as above, 1.16 g, 5.63 mmol) was added to a stirred solution of (*S*)-2-aminomethyl-2,5-dihydropyrrole-1-carboxylic acid benzyl ester **(96)** (0.35 g, 1.50 mmol) in dichloromethane (10 ml). The mixture was stirred for 16 hours then the solvents were removed *in vacuo*. The residue was purified by flash chromatography over silica eluting with ethyl acetate : heptane mixtures 1 : 9 to 1 : 4 to give (*S*)-2-(*N'*-allyloxycarbonylhydrazinomethyl)-2,5-dihydropyrrole-1-carboxylic acid benzyl ester **(97)** as a pale yellow oil (0.18 g, 36%). TLC ($R_f$ = 0.45, heptane : ethyl acetate 1 : 1), analytical HPLC $R_t$ = 14.789 min, HPLC-MS 332.1 $[M + H]^+$, 354.1 $[M + Na]^+$, 685.2 $[2M + Na]^+$. $d_H$ (500 MHz, $CDCl_3$) mixture of rotamers, tentative assignment of spectrum 2.73-3.40 (2H, m, $CH_2NHNH$), 4.08-4.80 (5H, m, H-2, H-5 and $CH_2CH{=}CH_2$), 4.95-5.35 (4H, m, $PhCH_2O$ and $CH_2CH{=}CH_2$), 5.57-5.98 (3H, m, H-4, H-5 and $CH_2CH{=}CH_2$), 7.25-7.55 (5H, aromatics); $d_C$ (125 MHz, $CDCl_3$) 53.61, 53.65, 54.02 and 54.07 (C-5 and $\underline{C}H_2NHNH$), 65.24 (C-2), 65.87, 66.21, 66.43, 66.82 and 67.13 ($\underline{C}H_2CH{=}CH_2$

and Ph$\underline{C}H_2O$), 117.48, 117.83, 118.20 and 118.49 ($CH_2CH=\underline{C}H_2$), 127.00, 127.13, 127.70, 127.79, 127.93, 128.02, 128.13, 128.33, 128.52 and 128.54 (C-3, C-4 and aromatic $\underline{C}H$), 132.11, 132.17, 132.21, 132.46, 132.54 and 132.68 ($CH_2\underline{C}H=CH_2$), 136.65 (aromatic quaternary), 154.52, 154.82, 155.85 and 156.21 (NNH$\underline{C}$=O), 158.69 ($CH_2N\underline{C}$=O).

**Preparation of (*S*)-2-(*N-tert*-butoxycarbonyl-*N'*-allyloxycarbonylhydrazino methyl)-2,5-dihydropyrrole-1-carboxylic acid benzyl ester (98)**

Three portions of Boc anhydride (each 1.20 g, 5.55 mmol) were added at one hour intervals to a stirred solution of (*S*)-2-(*N'*-allyloxycarbonylhydrazinomethyl)-2,5-dihydropyrrole-1-carboxylic acid benzyl ester **(97)** (184 mg, 0.56 mmol) in triethylamine : methanol (1 : 9, 10 ml). The mixture was stirred at 60 °C for 3 hours then the solvents were removed *in vacuo*. The residue was purified by flash chromatography over silica eluting with ethyl acetate : heptane mixtures 1 : 9 to 1 : 4 to give (*S*)-2-(*N-tert*-butoxycarbonyl-*N'*-allyloxycarbonylhydrazinomethyl)-2,5-dihydropyrrole-1-carboxylic acid benzyl ester **(98)** as a pale yellow oil (190 mg, 79%). TLC ($R_f$ = 0.5, heptane : ethyl acetate 1 : 1), analytical HPLC $R_t$ = 19.828 min, HPLC-MS 432.2 $[M + H]^+$, 454.1 $[M + Na]^+$, 885.2 $[2M + Na]^+$. $d_H$ (500 MHz, $CDCl_3$) mixture of rotamers approximately 4 : 1, 1.38-1.45 (9H, br. s, $C(CH_3)_3$), 3.45-3.74 (2H, m, $CH_2NNH$), 4.02-4.16 (1H, m, H-5), 4.24-4.38 (1H, m, H-5), 4.54-4.68 (2H, m, $CH_2CH=CH_2$), 4.72 (minor), 4.84 (major) (1H total, each br. s, H-2), 5.03-5.20 (2H, m, $PhCH_2O$), 5.18-5.34 (2H, m, $CH_2CH=CH_2$), 5.76 (1H, br. s, H-3), 5.79-5.85 (1H, m, H-4), 5.84-5.95 (1H, m, $CH_2CH=CH_2$), 7.28-7.40 (5H, aromatics); $d_C$ (125 MHz, $CDCl_3$) 28.07 ($C(\underline{C}H_3)_3$), 53.32 ($\underline{C}H_2NNH$), 53.86 (C-5), 62.45 and 62.86 (C-2), 66.21 and 67.02 ($\underline{C}H_2CH=CH_2$ and Ph$\underline{C}H_2O$), 81.03 and 81.43 ($\underline{C}(CH_3)_3$), 117.97 and 118.26 ($CH_2CH=\underline{C}H_2$), 126.63, 128.00, 128.47 and 128.68 (C-3, C-4 and aromatic $\underline{C}H$), 132.22 and 132.42 ($CH_2\underline{C}H=CH_2$), 136.48 (aromatic quaternary), 154.96 and 155.57 (NHNH$\underline{C}$=O and $CH_2N\underline{C}$=O).

**Preparation of (*S*)-2-(*N-tert*-butoxycarbonylhydrazinomethyl)-2,5-dihydro pyrrole-1-carboxylic acid benzyl ester (99)**

Tetralcis(triphenylphosphine)palladium(0) (10.2 mg, 0.0088 mmol) was added to a stirred solution of (*S*)-2-(*N-tert*-butoxycarbonyl-*N'*-allyloxycarbonylhydrazine methyl)-2,5-dihydropyrrole-1-carboxylic acid benzyl ester **(98)** (190 mg, 0.44 mmol) in dichloromethane (10 ml) under an atmosphere of argon. Phenylsilane (0.109 ml, 0.88 mmol) was then added dropwise over two minutes. The solution was stirred for 1 hour then the solvents were removed *in vacuo.* The oily residue was purified by flash chromatography over silica eluting with heptane : *tert*-butyl methyl ether 9 : 1 to 0 : 1 mixtures to give (*S*)-2-(*N-tert*-butoxycarbonylhydrazinomethyl)-2,5-dihydropyrrole-1-carboxylic acid benzyl ester **(99)** as a pale yellow oil (148 mg, 97%). TLC ($R_f$ = 0.35, heptane : ethyl acetate 1 : 1), analytical HPLC $R_t$ = 15.669 min, HPLC-MS 248.1 $[M + 2H - Boc]^+$, 292.1 $[M + 2H - Bu]^+$, 370.1 $[M + Na]^+$, 717.3 $[2M + Na]^+$. $d_H$ (500 MHz, $CDCl_3$) 1.41-1.45 (9H, br. s, $C(CH_3)_3$), 1.45-1.70 (2H, br. s, $NH_2$), 3.53-3.95 (2H, m, $CH_2NNH_2$), 4.03-4.12 (1H, m, H-5), 4.25-4.36 (1H, m, H-5), 4.74-4.91 (1H, m, H-2), 5.04-5.26 (2H, m, $PhCH_2O$), 5.73-5.87 (2H, m, H-4 and H-5), 7.28-7.41 (5H, aromatics); $d_C$ (125 MHz, $CDCl_3$) 28.26 ($C(\underline{C}H_3)_3$), 53.23 (C-5), 53.70 ($\underline{C}H_2NNH_2$), 63.24 (C-2), 66.73 and 67.18 (Ph$\underline{C}H_2O$), 80.25 and 80.53 ($\underline{C}(CH_3)_3$), 126.01, 127.82, 127.91, 128.08, 128.22, 128.42 and 128.49 (C-3 C-4, and aromatic $\underline{C}H$), 136.69 (aromatic quaternary).

**Preparation of (2*S*)-2-[*N'*-((2*S*)-2-allyloxycarbonylamino-4-methylpentanoyl)-*N-tert*-butoxycarbonylhydrazinomethyl]-2,5-dihydropyrrole-1-carboxylic acid benzyl ester (100)**

(i) Preparation of Alloc-L-Leucine Fluoride (Alloc-Leu-F)
Alloc-L-Leucine (0.90 g, 4.2 mmol) was dissolved in dichloromethane (50 ml) with stirring under nitrogen. (Diethylamino)sulfur trifluoride (DAST, 790 µl, 6.0 mmol) was added and the mixture stirred for 1.75 hours. The mixture was added to iced-water (200 ml) and product extracted into dichloromethane (50 ml), dried ($MgSO_4$), and reduced *in vacuo* to a mobile tan oil (0.70 g, 77%). An analytical sample, pre-treated with 10% pyridine in methanol gave HPLC-MS 230.1 $[M + H]^+$, 481.1 $[2M + Na]^+$ (methyl ester).

(ii) Alloc-Leu-F (prepared as above, 47 mg, 0.21 mmol) was dissolved in dimethylformamide (1.5 ml) then added to (*S*)-2-(*N-tert*-butoxycarbonyl-1-hydrazinomethyl)-2,5-dihydropyrrole-1-carboxylic acid benzyl ester **(99)** (71 mg, 0.20 mmol) under an atmosphere of nitrogen. The solution was stirred for 19 hours then the solvents were removed *in vacuo.* The residue was purified by flash chromatography over silica eluting with ethyl acetate : heptane mixtures 1 : 9 to 3 : 7 to give (2*S*)-2-[*N'*-((2*S*)-2-allyloxycarbonylamino-4-methylpentanoyl)-*N-tert*-butoxycarbonylhydrazinomethyl]-2,5-dihydropyrrole-1-carboxylic acid benzyl ester **(100)** as a sticky white solid (69 mg, 63%). TLC (Single spot, $R_f$ = 0.75, EtOAc : heptane 2 : 1), analytical HPLC broad double peak $R_t$ = 21.214 and 21.483 min; HPLC-MS

445.2 [M + 2H - Boc]$^+$, 489.2 [M + 2H - Bu]$^+$, 545.2 [M + H]$^+$.

**Preparation of (2*S*)-2-[*N'*-((2*S*)-2-amino-4-methylpentanoyl)-*N*-*tert*-butoxy carbonylhydrazinomethyl]-2,5,dihydropyrrole-1-carboxylic acid benzyl ester (101)**

Dichloromethane (1.5 ml) followed by phenylsilane (32 μl, 0.26 mmol) were consecutively added with stirring under an atmosphere of nitrogen to a mixture of tetrakistriphenylphosphine palladium(0) (3.0 mg, 0.003 mmol) and (2*S*)-2-[*N'*-((2*S*)-2-allyloxycarbonylamino-4-methylpentanoyl)-*N*-*tert*-butoxycarbonyl hydrazinomethyl]-2,5-dihydropyrrole-1-carboxylic acid benzyl ester **(100)** (70 mg, 0.129 mmol). The solution was stirred for 80 minutes then purified by flash chromatography over silica eluting with methanol : dichloromethane mixtures 0 : 100 to 5 : 95 to give (2*S*)-2-[*N'*-((2*S*)-2-amino-4-methylpentanoyl)-*N*-*tert*-butoxycarbonylhydrazinomethyl]-2,5-dihydropyrrole-1-carboxylic acid benzyl ester **(101)** as a colourless oil (57 mg, 96%). TLC (Single spot, $R_f$ = 0.65, MeOH : dichloromethane 1 : 9), analytical HPLC $R_t$ = 16.345 min; HPLC-MS 461.2 [M + H]$^+$, 483.2 [M + Na]$^+$, 921.4 [2M + H]$^+$.

**Preparation of (2*S*)-2-{*N*-*tert*-butoxycarbonyl-*N'*-[(2*S*)-2-(4-*tert*-butylbenzoyl amino)-4-methylpentanoyl]-hydrazinoylmethyl}-2,5-dihydropyrrole-1-carboxylic acid benzyl ester (102)**

4-Methylmorpholine (26.6 μl, 0.244 mmol) was added to a solution of HBTU (46 mg, 0.122 mmol), 1-hydroxybenzotriazole monohydrate (18.6 mg, 0.122 mmol) and 4-(*tert*-butyl)benzoic acid (22 mg, 0.122 mmol) in dimethylformamide (1.5 ml). The solution was stood for 5 minutes then added to (2*S*)-2-[*N'*-((2*S*)-2-amino-4-methylpentanoyl)-*N*-*tert*-butoxycarbonylhydrazinomethyl]-2,5-dihydropyrrole-1-carboxylic acid benzyl ester **(101)** (56 mg, 0.122 mmol). The mixture was stirred for 1 hour then the solvents were removed *in vacuo* (water bath temperature < 33 °C). The residue was dissolved in dichloromethane (15 ml) then washed with pH 3 hydrochloric acid (5 ml), saturated aqueous sodium hydrogen carbonate solution (5 ml) and brine (5 ml), dried ($Na_2SO_4$), and the solvents removed *in vacuo*. The yellow residue (108 mg) was purified by flash chromatography over silica eluting with ethyl acetate : heptane mixtures 1 : 9 to 3 : 7 to give (2*S*)-2-{*N*-*tert*-butoxycarbonyl-*N'*-[(2*S*)-2-(4-*tert*-butylbenzoylamino)-4-methylpentanoyl]-hydrazinoylmethyl}-2,5-dihydropyrrole-1-carboxylic acid benzyl ester **(102)** as a sticky white solid (63 mg, 84%). TLC ($R_f$ = 0.55, EtOAc : heptane 1 : 1), analytical HPLC $R_t$ = 24.205 min; HPLC-MS 521.2 [M + 2H - Boc]$^+$, 621.3 [M + H]$^+$.

**Preparation of (2*S*)-2-{*N*-*tert*-butoxycarbonyl-*N'*-[(2*S*)-2-(4-*tert*-butylbenzoyl amino)-4-methylpentanoyl]-hydrazinomethyl}-6-oxa-3-aza-bicyclo[3.1.0]hexane-3-carboxylic acid benzyl ester (103)**

A solution of *meta*-chloroperoxybenzoic acid (57-86%, 196 mg, ~0.81 mmol) in dichloromethane (1.2 ml) was added to (2*S*)-2-{*N*-*tert*-butoxycarbonyl-*N'*-[(2*S*)-2-(4-*tert*-butylbenzoylamino)-4-methylpentanoyl]-hydrazinoylmethyl}-2,5-dihydropyrrole -1-carboxylic acid benzyl ester **(102)** (50 mg, 0.081 mmol) under an atmosphere of nitrogen. The solution was stirred for 20 hours then dichloromethane (1 ml) was added and the mixture washed with 5% aqueous sodium hydroxide solution (10 ml), then 10% aqueous sodium hydroxide solution (5 ml), dried ($Na_2SO_4$), and the solvents removed *in vacuo*. The residue was purified by flash chromatography over silica eluting with ethyl acetate : heptane mixtures 0 : 100 to 40 : 60 to give (2*S*)-2-{*N*-*tert*-butoxycarbonyl-*N'*-[(2*S*)-2-(4-*tert*-butylbenzoylamino)-4-methylpentanoyl]-hydrazino methyl}-6-oxa-3-aza-bicyclo[3.1.0]hexane-3-carboxylic acid benzyl ester **(103)** as a white solid (39 mg, 75%). TLC ($R_f$ = 0.30, EtOAc : heptane 2 : 3), analytical HPLC $R_t$ = 23.156 min; HPLC-MS 537.2 [M + 2H - Boc]$^+$, 637.2 [M + H]$^+$.

**Preparation of (3a*R*, 6*S*, 6a*S*)-1-[(2*S*)-2-(4-*tert*-butylbenzoylamino)-4-methylpentanoyl]-6-hydroxyhexahydropyrrolo[3,2-*c*]pyrazole-2,4-dicarboxylic acid 4-benzyl ester 2-*tert*-butyl ester (104)**

A solution of (2*S*)-2-{*N*-*tert*-butoxycarbonyl-*N'*-[(2*S*)-2-(4-*tert*-butyl benzoylamino)-4-methylpentanoyl]-hydrazinomethyl}-6-oxa-3-aza-bicyclo[3.1.0] hexane-3-carboxylic acid benzyl ester **(103)** (38.5 mg, 0.061 mmol) in acetonitrile (4.0 ml) was added to potassium carbonate (210 mg, 1.51 mmol). The suspension was placed under an atmosphere of nitrogen then heated at 60 °C whilst stirring for 4.75 hours before being allowed to cool to ambient temperature. The suspension was filtered then the filtrate concentrated *in vacuo*. The residue was purified by flash chromatography over silica eluting with ethyl acetate : heptane mixtures 0 : 100 to 50 : 50 to give (3a*R*, 6*S*, 6a*S*)-1-[(2*S*)-2-(4-*tert*-butylbenzoylamino)-4-methylpentanoyl]-6-hydroxyhexahydropyrrolo[3,2-*c*] pyrazole-2,4-dicarboxylic acid 4-benzyl ester 2-*tert*-butyl ester **(104)** as a white solid (16.2 mg, 42%). TLC ($R_f$ = 0.30, EtOAc : heptane 1 : 1), analytical HPLC $R_t$ = 21.762 min; HPLC-MS 537.2 [M + 2H - Boc]$^+$, 581.1 [M + 2H - Bu]$^+$, 637.2 [M + H]$^+$.

**Preparation of (3a*R*, 6*S*, 6a*S*)-4-benzoyl-1-[(2*S*)-2-(4-*tert*-butylbenzoylamino)-4-methylpentanoyl]-6-hydroxyhexahydropyrrolo[3,2-*c*]pyrazole-2-carboxylic acid *tert*-butyl ester (106)**

Under an atmosphere of nitrogen a solution of (3a*R*, 6*S*, 6a*S*)-1-[(2*S*)-2-(4-*tert*-butylbenzoylamino)-4-methylpentanoyl]-6-hydroxyhexahydropyrrolo[3,2-c] pyrazole-2,4-dicarboxylic acid 4-benzyl ester 2-*tert*-butyl ester **(104)** (16.0 mg, 0.025 mmol) in ethanol (1.5 ml) was added to 10% palladium on charcoal (10 mg) whilst stirring. The nitrogen was replaced by hydrogen then stirring continued for 30 minutes. The hydrogen was replaced by nitrogen then the mixture filtered through celite. The filter cake was washed with ethanol (40 ml) then the filtrate concentrated *in vacuo*. The residue was used without further purification. Analytical HPLC $R_t$ = 18.568 min; HPLC-MS 403.2 [M + 2H - Boc]$^+$, 503.2 [M + H]$^+$ for (3a*R*, 6*S*, 6a*S*)-1-[(2*S*)-2-(4-*tert*-butylbenzoylamino)-4-methylpentanoyl]-6-hydroxyhexahydropyrrolo[3,2-c] pyrazole-2-carboxylic acid *tert*-butyl ester **(105).**

Benzoic anhydride (6.0 mg, 0.026 mmol), dimethylformamide (0.3 ml) then 4-methylmorpholine (5.8 μl, 0.053 mmol) were added consecutively to (3a*R*, 6*S*, 6a*S*)-1-[(2*S*)-2-(4-*tert*-butylbenzoylamino)-4-methylpentanoyl]-6-hydroxyhexa hydropyrrolo[3,2-c]pyrazole-2-carboxylic acid *tert*-butyl ester **(105)** (~0.025 mmol, prepared as above). The solution was stirred for 65 minutes then the majority of solvents were removed *in vacuo*. The residue was dissolved in ethyl acetate (10 ml), then washed with saturated aqueous sodium hydrogen carbonate solution (5 ml), pH 3 hydrochloric acid (5 ml) and brine (5 ml), dried ($Na_2SO_4$), and the solvents removed *in vacuo*. The residue (15.5 mg) was purified by flash chromatography over silica eluting with ethyl acetate : heptane mixtures 5 : 95 to 50 : 50 to give (3a*R*, 6*S*, 6a*S*)-4-benzoyl-1-[(2*S*)-2-(4-*tert*-butylbenzoylamino)-4-methylpentanoyl]-6-hydroxyhexa hydropyrrolo[3,2-*c*]pyrazole-2-carboxylic acid *tert*-butyl ester **(106)** as a white solid (10.3 mg, 68%). TLC ($R_f$ = 0.25, EtOAc : heptane 1 : 1), analytical HPLC $R_t$ = 22.101 min; HPLC-MS 278.1, 507.2 [M + 2H - Boc]$^+$, 607.2 [M + H]$^+$.

**Preparation of (3a*R*, 6a*S*)-4-benzoyl-1-[(2*S*)-2-(4-*tert*-butylbenzoylamino)-4-methylpentanoyl]-6-oxo-hexahydropyrrolo[3,2-*c*]pyrazole-2-carboxylic acid *tert*-butyl ester (107)**

A solution of Dess-Martin periodinane (54 mg, 0.128 mmol) in dichloromethane (1.25 ml) was added to (3a*R*, 6*S*, 6a*S*)-4-benzoyl-1-[(2*S*)-2-(4-*tert*-butylbenzoylamino)-4-methylpentanoyl]-6-hydroxyhexahydropyrrolo[3,2-*c*] pyrazole-2-carboxylic acid *tert*-butyl ester **(106)** (15.5 mg, 0.026 mmol) under an atmosphere of nitrogen. The mixture was stirred for 4.5 hours then purified by flash chromatography over silica eluting with ethyl acetate : heptane mixtures 1 : 9 to 2 : 3 to give (3a*R*, 6a*S*)-4-benzoyl-1-[(2*S*)-2-(4-*tert*-butylbenzoylamino)-4-methylpentanoyl]-6-oxo-hexahydro pyrrolo[3,2-*c*]pyrazole-2-carboxylic acid *tert*-butyl ester **(107)** as a white solid (12.8 mg, 81%). TLC ($R_f$ = 0.65, EtOAc : heptane 3 : 2), analytical HPLC broad peak $R_t$ = 21.02-23.60 min; HPLC-MS single broad main UV peak 274.1, 505.2 [M + 2H - Boc]$^+$, 549.1 [M + 2H - Bu]$^+$, 605.2 [M + H]$^+$, 623.2 [M + $H_2O$ + H]$^+$, 627.2 [M + Na]$^+$, 645.2 [M + $H_2O$ + Na]$^+$.

**Preparation of (3a*R*, 6a*S*)-*N*-[(1*S*)-1-(4-benzoyl-6-oxo-hexahydropyrrolo[3,2-*c*] pyrazole-1-carbonyl)-3-methylbutyl]-4-*tert*-butylbenzamide** (EXAMPLE 249a)

Trifluoroacetic acid (0.15 ml) was added to (3a*R*, 6a*S*)-4-benzoyl-1-[(2*S*)-2-(4-*tert*-butylbenzoylamino)-4-methylpentanoyl]-6-oxo-hexahydropyrrolo[3,2-*c*] pyrazole-2-carboxylic acid *tert*-butyl ester **(107)** (10.3 mg, 0.017 mmol) under an atmosphere of nitrogen. The solution was stirred for 45 minutes then cautiously added to saturated aqueous sodium hydrogen carbonate solution (10 ml). The product was extracted into dichloromethane (10 ml) then washed with water (10 ml), dried ($Na_2SO_4$), and the solvents removed *in vacuo*. The residue (10 mg) was combined with a second batch of material (0.8 mg, prepared in a similar way to above from 1.14 mg of (3a*R*, 6a*S*)-4-benzoyl-1-[(2*S*)-2-(4-*tert*-butyl benzoylamino)-4-methylpentanoyl]-6-oxo-hexahydropyrrolo[3,2-*c*]pyrazole-2-carboxylic acid *tert*-butyl ester **(107),** then purified by flash chromatography over silica eluting with ethyl acetate : heptane mixtures 20 : 80 to 65 : 35 to give (3a*R*, 6a*S*)-*N*-[(1*S*)-1-(4-benzoyl-6-oxo-hexahydropyrrolo[3,2.*c*]pyrazole-1-carbonyl)-3-methylbutyl]-4-*tert*-butylbenzamide (EXAMPLE 249a) as an off-white solid (3.49 mg, 37 %), together with recovered (3a*R*, 6a*S*)-4-benzoyl-1-[(2*S*)-2-(4-*tert*-butylbenzoylamino)-4-methylpentanoyl]-6-oxo-hexahydropyrrolo[3,2-*c*]pyrazole -2-carboxylic acid *tert*-butyl ester **(107)** (1.83 mg, 16%). Data for (3a*R*, 6a*S*)-*N*-[(1*S*)-1-(4-benzoyl-6-oxo-hexahydropyrrolo[3,2-*c*]pyrazole-1-carbonyl)-3-methyl butyl]-4-*tert*-butylbenzamide (EXAMPLE 249a), TLC ($R_f$ = 0.26, EtOAc : heptane 3 : 1), analytical HPLC broad peak $R_t$ = 18.10-19.70 min; HPLC-MS single broad main UV peak 274.1, 505.1 [M + H]$^+$, 523.2 [M + $H_2O$ + H]$^+$.

An alternative preparation of (3a*R*, 6*S*, 6a*S*)-4-benzoyl-1-[(2*S*)-2-(4-*tert*-butylbenzoylamino)-4-methylpentanoyl]-6-hydroxyhexahydropyrrolo[3,2-*c*]pyrazole -2-carboxylic acid *tert*-butyl ester **(106)** is detailed in Scheme 24.

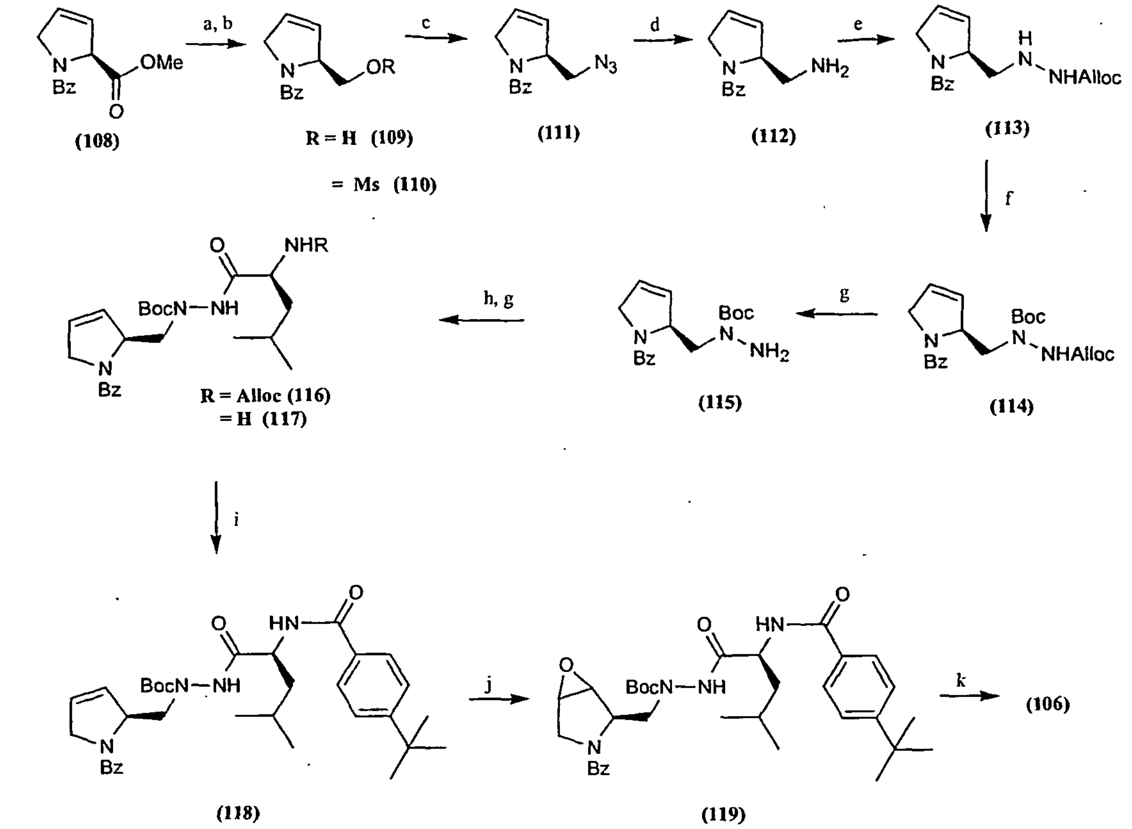


(108) R = H (109) = Ms (110) (111) (112) (113) (114) (115) R = Alloc (116) = H (117) (118) (119) (106)

**Scheme 24.** (a) $LiBH_4$, MeOH, THF (b) Methanesulfonyl chloride, triethylamine, DCM (c) Sodium azide, DMF, 110°C (d) $Ph_3P$ / $H_2O$, 1,4-dioxane, 50°C (e) 3-Phenyloxaziridine-2-carboxylic acid allyl ester, DCM (f) $(Boc)_2O$, DCM, 60°C (g) $Pd(PPh_3)_4$, $PhSiH_3$, DCM (h) Alloc-Leu-F, DMF (i) 4-*tert*-Butylbenzoic acid, HBTU, HOBT, NMM, DMF (j) *m*-Chloroperoxybenzoic acid, DCM. (k) Potassium carbonate, $CH_3CN$, 60°C

**Preparation of (*S*)-1-benzoyl-2,5-dihydro-1*H*-pyrrole-2-carboxylic acid 1-methyl ester (108)**

Benzoic anhydride (4.15 g, 18.3 mmol) followed by 4-methylmorpholine (2.82 ml, 25.7 mmol) were consecutively added to a stirred solution of (*S*)-2,5-dihydro-1*H*-pyrrole-2-carboxylic acid methyl ester hydrochloride (2.0 g, 12.2 mmol) in DMF (50 ml). The mixture was stirred for 1.5 hours then the solvents were removed *in vacuo.* The product was extracted into *tert*-butyl methyl ether (300 ml) then washed with 5% hydrochloric acid (100 ml), 5% aqueous sodium hydrogen carbonate solution (100 ml), and brine (100 ml), dried ($MgSO_4$), and the solvents removed *in vacuo.* The residue was purified by flash chromatography over silica eluting with ethyl acetate : heptane 1 : 4 to give (*S*)-1-benzoyl-2,5-dihydro-1*H*-pyrrole-2-carboxylic acid 1-methyl ester **(108)** as a colourless oil (2.05 g, 73%). TLC ($R_f$ = 0.25, EtOAc : heptane 1 : 1); HPLC-MS 232.1, $[M + H]^+$, 254.0 $[M + Na]^+$, 485.1 $[2M + Na]^+$; $\delta_H$ (500 MHz, $CDCl_3$) 3.78 (3H, s, $OCH_3$), 4.15-4.22 (1H, m, H-5), 4.41-4.47 (1H, m, H-5), 5.45-5.49 (1H, m, H-2), 5.83-5.95 (2H, m, H-3 and H-4), 7.36-7.58 (5H, aromatics); $\delta_C$ (125 MHz, $CDCl_3$) 52.50 ($O\underline{C}H_3$), 55.90 (C-5), 66.39 (C-2), 124.89, 127.01, 128.39, 128.41, 128.60 and 130.24 (C-3, C-4 and aromatic $\underline{C}$H), 135.86 (aromatic quaternary), 169.73 and 170.16 (CH$\underline{C}$=O and N$\underline{C}$=OPh).

**Preparation of (*S*)-(2-hydroxymethyl-2,5-dihydropyrrole-1-yl)phenyl methanone (109)**

Methanol (0.71 ml) followed by a solution of (*S*)-1-benzoyl-2,5-dihydro-1*H*-pyrrole-2-carboxylic acid 1-methyl ester **(108)** (2.05 g, 8.9 mmol) in THF (37 ml) were added dropwise to a stirred suspension of lithium borohydride (390 mg, 17.7 mmol) in THF (13 ml). The mixture was stirred for 1 hour then water (5 ml) was carefully added. The product was extracted into *tert*-butyl methyl ether (3 x 50 ml), then the combined organic layers dried ($MgSO_4$), and the solvents removed *in vacuo* to give (*S*)-(2-hydroxymethyl-2,5-dihydropyrrole-1-yl)phenylmethanone **(109)** as a pale yellow oil (1.68 g, 93%) which was used without further purification. TLC ($R_f$ = 0.1, EtOAc : heptane 1 : 1), HPLC-MS 204.1 $[M + H]^+$; $d_H$ (500 MHz, $CDCl_3$) 1.65-1.95 (1H, br. s, $CH_2OH$), 3.72 (1H, dd $J$ = 11.4 and 7.1 Hz, $CH_2OH$), 3.90 (1H, dd $J$ = 11.6 and 2.1 Hz, $CH_2OH$), 4.11-4.16 (1H, m, H-5), 4.27-4.38 (1H, m, H-5), 5.17-5.21 (1H, m, H-2), 5.74-5.82 (2H, m, H-3 and

H-4), 7.38-7.53 (5H, aromatics); $d_C$ (125 MHz, $CDCl_3$) 56.76 (C-5), 66.44 ($\underline{C}H_2OH$), 68.68 (C-2), 126.25, 126.67, 126.93, 126.95, 128.50 and 130.20 (C-3, C-4 and aromatic $\underline{C}H$), 136.21 (aromatic quaternary), 172.12 (N$\underline{C}$=OPh).

**Preparation of (*S*)-methanesulfonic acid 1-benzoyl-2,5-dihydro-1*H*-pyrrol-2-ylmethyl ester (110)**

Triethylamine (1.86 ml, 13.2 mmol) was added dropwise to a stirred solution of (*S*)-(2-hydroxymethyl-2,5-dihydropyrrole-1-yl)phenylmethanone **(109)** (1.68 g, 8.3 mmol) and methanesulfonyl chloride (0.96 ml, 12.4 mmol) in dichloromethane (30 ml) at 0 °C. The mixture was stirred for 30 minutes at ambient temperature then washed with water (100 ml), and brine (100 ml), dried ($Na_2SO_4$), and the solvents removed *in vacuo* to give (*S*)-methanesulfonic acid 1-benzoyl-2,5-dihydro-1*H*-pyrrol-2-ylmethyl ester **(110)** as a pale yellow oil (1.88 g, 81 %) which was used without further purification. HPLC-MS 282.0 $[M + H]^+$, 585.1 $[2M + Na]^+$.

**Preparation of (*S*)-(2-azidomethyl-2,5-dihydropyrrol-1-yl)phenylmethanone (111)**

Sodium azide (2.17 g, 33.4 mmol) was added to a stirred solution of (*S*)-methanesulfonic acid 1-benzoyl-2,5-dihydro-1*H*-pyrrol-2-ylmethyl ester **(110)** (1.88 g, 6.68 mmol) in DMF (50 ml). The reaction mixture was stirred at 110 °C for 1 hour. The solvent was removed *in vacuo* then the product was extracted into ethyl acetate (100 ml), washed with water (100 ml) and brine (100 ml), dried ($MgSO_4$), and the solvents removed *in vacuo*. The residue was purified by flash chromatography over silica eluting with heptane : ethyl acetate 9 : 1 to give (*S*)-(2-azidomethyl-2,5-dihydropyrrol-1-yl)phenylmethanone **(111)** as a pale yellow oil (0.96 g, 63%). TLC ($R_f$ = 0.40, heptane : ethyl acetate 1 : 1), analytical HPLC $R_t$ = 13.943 min, HPLC-MS 229.1 $[M + H]^+$, 251.1 $[M + Na]^+$, 479.1 $[2M + Na]^+$.

**Preparation of (*S*)-(2-aminomethyl-2,5-dihydro-pyrrol-1-yl)phenyl methanone (112)**

Triphenylphosphine (1.65 g, 6.30 mmol) was added to a stirred solution of (*S*)-(2-azidomethyl-2,5-dihydropyrrol-1-yl)phenylmethanone **(111)** (0.96 g, 4.2 mmol) in THF (87 ml) containing water (1 ml). The mixture was stirred at 50 °C for 24 hours then the solvents were removed *in vacuo*. The residue was purified by flash chromatography over silica eluting with ethyl acetate : heptane 1 : 9 then ethyl acetate : methanol 4 : 1 mixtures to give (*S*)-(2-aminomethyl-2,5-dihydro-pyrrol-1-yl)phenylmethanone **(112)** as a pale yellow oil (0.77 g, 91%). TLC ($R_f$ = 0.1, chloroform : methanol 9 : 1), analytical HPLC with UV peaks at $R_t$ = 6.705 and 7.943 min; HPLC-MS 203.1 $[M + H]^+$, 427.1 $[2M + Na]^+$. $d_H$ (500 MHz, $D_6$-DMSO) 1.30-2.10 (2H, br. s, $CH_2NH_2$), 2.84 (2H, m, $CH_2NH_2$), 3.87 (1H, dd, *J* = 14.9 and 1.0 Hz, H-5), 4.26-4.35 (1H, m, H-5), 4.84 (1H, m, H-2), 5.83-6.03 (2H, m, H-4 and H-5), 7.39-7.58 (5H, aromatics); $d_C$ (125 MHz, $D_6$-DMSO) 44.25 ($\underline{C}H_2NH_2$), 56.96 (C-5), 67.20 (C-2), 126.50, 126.62, 126.83, 127.51, 128.63, 128.84, 128.96, 129.52, 129.81 and 130.22 (C-3, C-4 and aromatic $\underline{C}H$), 137.48 (aromatic quaternary), 169.44 (N$\underline{C}$=O).

**Preparation of (*S*)-*N*-(1-benzoyl-2,5-dihydro-1*H*-pyrrol-2-ylmethyl)hydrazine carboxylic acid allyl ester (113)**

3-Phenyloxaziridine-2-carboxylic acid allyl ester (prepared as above, 1.95 g, 9.5 mmol) was added to a stirred solution of (*S*)-(2-aminomethyl-2,5-dihydro-pyrrol-1-yl)phenylmethanone **(112)** (0.64 g, 3.17 mmol) in dichloromethane (10 ml). The mixture was stirred for 16 hours then the solvents were removed *in vacuo*. The oily residue was purified by flash chromatography over silica eluting with ethyl acetate : heptane mixtures 1 : 9 to 1 : 4 to give (*S*)-*N*-(1-benzoyl-2,5-dihydro-1*H*-pyrrol-2-ylmethyl)hydrazinecarboxylic acid allyl ester **(113)** as a pale yellow oil (0.26 g, 27%). TLC ($R_f$ = 0.45, heptane : ethyl acetate 1 : 1), HPLC-MS 302.1 $[M + H]^+$, 324.1 $[M + Na]^+$, 625.1 $[2M + Na]^+$.

**Preparation of (*S*)-*N'*-(1-benzoyl-2,5-dihydro-1*H*-pyrrol-2-ylmethyl)-*N'*-*tert*-butoxycarbonyl-hydrazinecarboxylic acid allyl ester (114)**

Three portions of Boc anhydride (each 1.84 g, 8.1 mmol) were added at one hour intervals to a stirred solution of (*S*)-*N*-(1-benzoyl-2,5-dihydro-1*H*-pyrrol-2-ylmethyl)hydrazinecarboxylic acid allyl ester **(113)** (0.26 g, 0.85 mmol) in triethylamine : methanol (1 : 9, 10 ml). The mixture was stirred at 60 °C for 3 hours then the solvents were removed *in vacuo*. The oily residue was purified by flash chromatography over silica eluting with ethyl acetate : heptane mixtures 0 : 1 to 1 : 4 to give (*S*)-*N'*-(1-benzoyl-2,5-dihydro-1*H*-pyrrol-2-ylmethyl)-*N'*-*tert*-butoxycarbonyl-hydrazinecarboxylic acid allyl ester **(114)** as a pale yellow oil (96 mg, 28%). TLC ($R_f$ = 0.25, heptane : ethyl acetate 1 : 1), analytical HPLC $R_t$ = 6.025 min; HPLC-MS 402.1 $[M + H]^+$, 825.1 $[2M + Na]^+$.

### Preparation of (*S*)-*N*-(1-benzoyl-2,5-dihydro-1*H*-pyrrol-2-ylmethyl)hydrazine carboxylic acid *tert*-butyl ester (115)

Tetrahis(triphenylphosphine)palladium(0) (7.5 mg, 0.0065mmol) was added to a stirred solution of (*S*)-*N'*-(1-benzoyl-2,5-dihydro-1*H*-pyrrol-2-ylmethyl)-*N'*-*tert*-butoxycarbonyl-hydrazinecarboxylic acid allyl ester **(114)** (131 mg, 0.326 mmol) in dichloromethane (5 ml) under an atmosphere of argon. Phenylsilane (81 µl, 0.65 mmol) was then added dropwise over two minutes. The solution was stirred for 1 hour then the solvents were removed *in vacuo*. The oily residue was purified by flash chromatography over silica eluting with heptane : *tert*-butyl methyl ether 9 : 1 to 0 : 1 mixtures to give (*S*)-*N*-(1-benzoyl-2,5-dihydro-1*H*-pyrrol-2-ylmethyl)hydrazine carboxylic acid *tert*-butyl ester **(115)** as a brown oil (77 mg, 74%). TLC ($R_f$ = 0.35, heptane : ethyl acetate 1 : 1), analytical HPLC $R_t$ = 12.738 min; HPLC-MS 218.1 [M + 2H - Boc]$^+$, 262.1 [M + 2H - Bu]$^+$, 318.1 [M + H]$^+$, 635.3 [2M + H]$^+$, 657.2 [2M + Na] $^+$.

### Preparation of *N'*-((2*S*)-2-allyloxycarbonylamwo-4-methylpentanoyl)-*N*-((2*S*)-1-benzoyl-2,5-dihydro-1*H*-pyrrol-2-ylmethyl)hydrazinecarboxylic acid *tert*-butyl ester (116)

Alloc-Leu-F (prepared as above, 55 mg, 0.26 mmol) was added to a stirred solution of (S)-2-*N*-(1-benzoyl-2,5-dihydro-1*H*-pyrrol-2-ylmethyl)hydrazine carboxylic acid *tert*-butyl ester **(115)** (77 mg, 0.24 mmol) in dimethylformamide (1.5 ml) under an atmosphere of nitrogen. The solution was stirred for 4.5 hours then the solvents were removed *in vacuo.* The residue was purified by flash chromatography over silica eluting with ethyl acetate : heptane mixtures 1 : 9 to 2 : 3 to give *N'*-((2*S*)-2-allyloxycarbonylamino-4-methylpentanoyl)-*N*-((2*S*)-1-benzoyl-2,5-dihydro-1*H*-pyrrol -2-ylmethyl)hydrazinecarboxylic acid *tert*-butyl ester **(116)** as a viscous oil (83 mg, 67%). TLC (Single spot, $R_f$ = 0.45, EtOAc : heptane 1 : 1), analytical HPLC $R_t$ = 19.157 min; HPLC-MS 415.1 [M + 2H - Boc]$^+$, 459.1 [M + 2H - Bu]$^+$, 515.2 [M + H]$^+$.

### Preparation of *N'*-((2*S*)-2-amino4-methylpentanoyl)-*N*-((2*S*)-1-benzoyl-2,5-dihydro-1*H*-pyrrol-2-ylmethyl)hydrazinecarboxylic acid *tert*-butyl ester (117)

Dichloromethane (1.5 ml) followed by phenylsilane (39 µl, 0.32 mmol) were consecutively added with stirring under an atmosphere of nitrogen to a mixture of tetrakistriphenylphosphine palladium(0) (3.7 mg, 0.003 mmol) and *N'*-((2*S*)-2-allyloxycarbonylamino-4-methylpentanoyl)-*N*-((2*S*)-1-benzoyl-2,5-dihydro-1*H*-pyrrol -2-ylmethyl)hydrazinecarboxylic acid *tert*-butyl ester **(116)** (82 mg, 0.16 mmol). The solution was stirred for 1.75 hours then purified by flash chromatography over silica eluting with methanol : dichloromethane mixtures 1 : 99 to 5 : 95 to give *N'*-((2*S*)-2-amino-4-methylpentanoyl)-*N*-((2*S*)-1-benzoyl-2,5-dihydro-1*H*-pyrrol-2-ylmethyl) hydrazinecarboxylic acid *tert*-butyl ester **(117)** as a colourless oil (57 mg, 83%). TLC (Single spot, $R_f$ = 0.45, MeOH : dichloromethane 6 : 94), analytical HPLC $R_t$ = 14.217 min; HPLC-MS 431.1 [M + H]$^+$, S61.3 [2M + H]$^+$, 883.3 [2M + Na]$^+$.

### Preparation of *N*-((2*S*)-1-benzoyl-2,5-dihydro-1*H*-pyrrol-2-ylmethyl)-*N'*-[(2*S*)-2-(4-*tert*-butylbenzoylamino)-4-methylpentanoyl]hydrazinecarboxylic acid *tert*-butyl ester (118)

4-Methylmorpholine (28.5 µl, 0.26 mmol) was added to a solution of HBTU (49 mg, 0.13 mmol), 1-hydroxybenzotriazole monohydrate (20 mg, 0.13 mmol) and 4-(*tert*-butyl)benzoic acid (23 mg, 0.13 mmol) in dimethylformamide (1.5 ml). The solution was stood for 5 minutes then added to *N'*-((2*S*)-2-amino-4-methylpentanoyl)-*N*-((2*S*)-1-benzoyl-2,5-dihydro-1*H*-pyrrol-2-ylmethyl) hydrazine carboxylic acid *tert*-butyl ester **(117)** (56 mg, 0.13 mmol). The mixture was stirred for 1.5 hour then the solvents were removed *in vacuo* (water bath temperature < 33 °C). The residue was dissolved in dichloromethane (15 ml) then washed with pH 3 hydrochloric acid (5 ml), saturated aqueous sodium hydrogen carbonate solution (5 ml) and brine (5 ml), dried ($Na_2SO_4$), and the solvents removed *in vacuo*. The yellow residue (102 mg) was purified by flash chromatography over silica eluting with ethyl acetate : heptane mixtures 1 : 9 to 1 : 1 to give *N*-((2*S*)-1-benzoyl-2,5-dihydro-1*H*-pyrrol-2-ylmethyl)-*N'*-[(2*S*)-2-(4-*tert*-butylbenzoylamino)-4-methylpentanoyl]hydrazinecarboxylic acid *tert*-butyl ester **(118)** as a white solid (52 mg, 68%). TLC ($R_f$ = 0.46, EtOAc : heptane 1 : 1), analytical HPLC $R_t$ = 22.310 min; HPLC-MS 491.2 [M + 2H - Boc]$^+$, 591.2 [M + H]$^+$.

### Preparation of *N*-((2*S*)-3-benzoyl-6-oxa-3-aza-bicyclo[3.1.0]hex-2-ylmethyl)-*N'*-[(2*S*)-2-(4-*tert*-butylbenzoylamino)-4-methylpentanoyl]hydrazine carboxylic acid *tert*-butyl ester (119)

A solution of *meta*-chloroperoxybenzoic acid (57-86%, 210 mg, ~0.86 mmol) in dichloromethane (1.25 ml) was added to *N*-((2*S*)-1-benzoyl-2,5-dihydro-1*H*-pyrrol-2-ylmethyl)-*N'*-[(2*S*)-2-(4-*tert*-butylbenzoylamino)-4-methylpentanoyl]hydrazine carboxylic acid *tert*-butyl ester **(118)** (51 mg, 0.086 mmol) under an atmosphere of nitrogen. The solution was stirred for 20 hours then dichloromethane (15 ml) was added and the mixture washed with 10% aqueous sodium hydroxide solution (10 ml). The aqueous layer was extracted with dichloromethane (5 ml) then the combined organic

layers washed with 10% aqueous sodium hydroxide solution (10 ml), dried ($Na_2SO_4$), and the solvents removed *in vacuo*. The yellow oily residue was purified by flash chromatography over silica eluting with ethyl acetate : heptane mixtures 1 : 9 to 1 : 1 to give *N*-((2*S*)-3-benzoyl-6-oxa-3-aza-bicyclo[3.1.0]hex-2-ylmethyl)-*N'*-[(2*S*)-2-(4-*tert*-butylbenzoylamino)-4-methylpentanoyl]hydrazinecarboxylic acid *tert*-butyl ester **(119)** as a white solid (27.5 mg, 52%). TLC ($R_f$ = 0.25, EtOAc : heptane 1 : 1), analytical HPLC $R_t$ = 21.769 min; HPLC-MS 507.2 $[M + 2H - Boc]^+$, 607.2 $[M + H]^+$.

**Preparation of (3a*R*, 6*S*, 6a*S*)-4-benzoyl-1-[(2*S*)-2-(4-*tert*-butylbenzoylamino)-4-methylpentanoyl]-6-hydroxyhexahydropyrrolo[3,2-*c*]pyrazole-2-carboxylic acid *tert*-butyl ester (106)**

A solution of *N*-((2*S*)-3-benzoyl-6-oxa-3-aza-bicyclo[3.1.0]hex-2-ylmethyl)-*N'*-[(2*S*)-2-(4-*tert*-butylbenzoylamino)-4-methylpentanoyl]hydrazinecarboxylic acid *tert*-butyl ester **(119)** (26 mg, 0.043 mmol) in acetonitrile (2.5 ml) was added to potassium carbonate (150 mg, 1.09 mmol). The suspension was placed under an atmosphere of nitrogen then heated at 60 °C whilst stirring for 3.25 hours before being allowed to cool to ambient temperature. The suspension was filtered and the collected solid washed with acetonitrile (20 ml), then the filtrate was concentrated *in vacuo*. The residue was dissolved in dichloromethane (15 ml), washed with water (10 ml), dried ($Na_2SO_4$), and the solvents removed *in vacuo*. The residue was purified by flash chromatography over silica eluting with ethyl acetate : heptane mixtures 1 : 4 to 1 : 1 to give (3a*R*, 6*S*, 6a*S*)-4-benzoyl-1-[(2*S*)-2-(4-*tert*-butylbenzoylamino)-4-methylpentanoyl]-6-hydroxyhexahydropyrrolo[3,2-*c*] pyrazole-2-carboxylic acid *tert*-butyl ester **(106)** as a white solid (16.2 mg, 62%). TLC ($R_f$ = 0.30, EtOAc : heptane 3 : 2), analytical HPLC $R_t$ = 22.013 min; HPLC-MS 278.1, 507.2 $[M + 2H - Boc]^+$, 607.2 $[M + H]^+$.

EXAMPLES 249b and 249c were prepared from the intermediate (2*S*)-2-[*N'*-((2*S*)-2-amino-4-methylpentanoyl)-*N*-*tert*-butoxycarbonylhydrazinomethyl]-2,5-dihydro pyrrole-1-carboxylic acid benzyl ester **(101)** following Scheme 25;

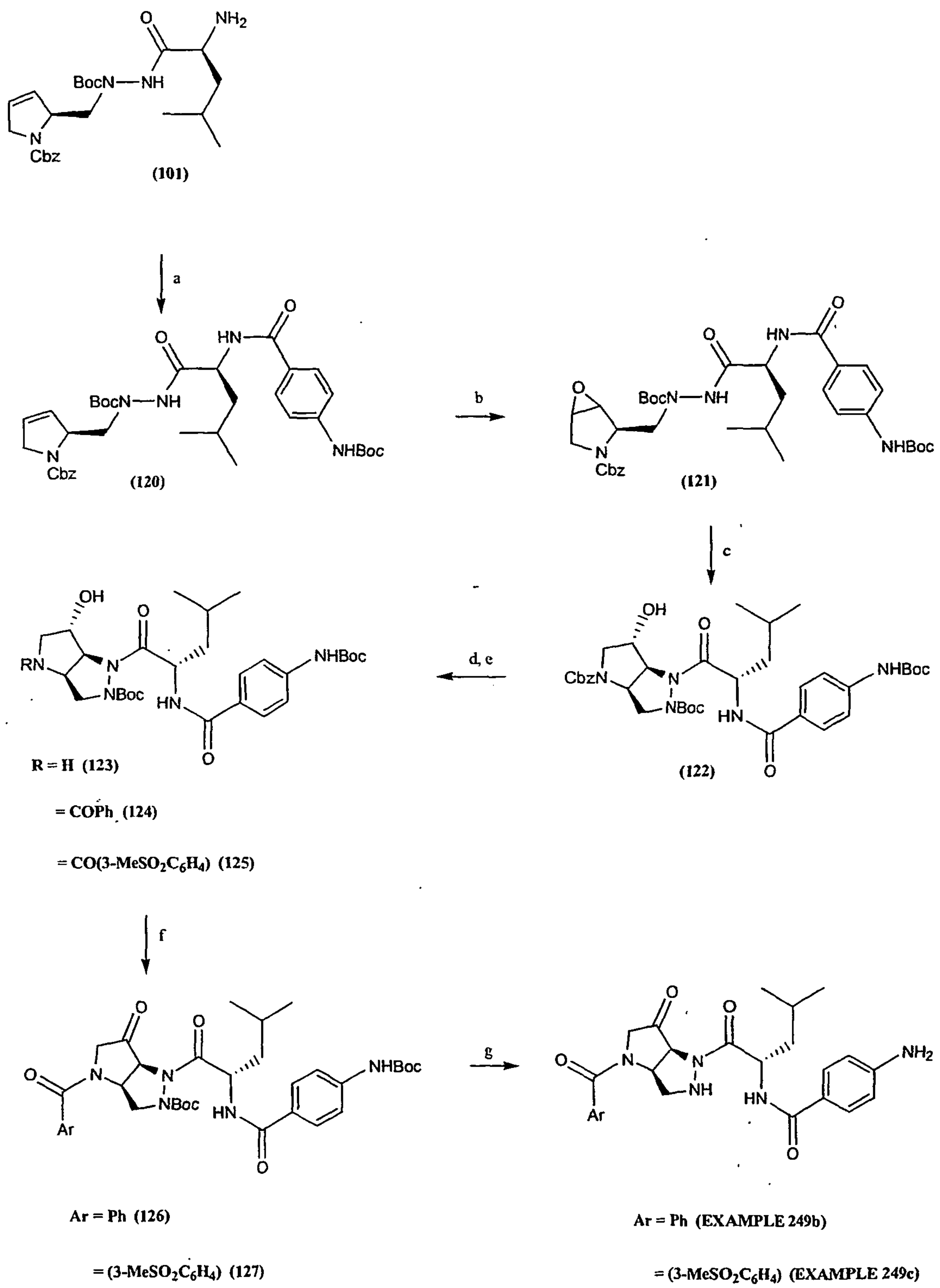


**Scheme 25.** (a) 4-*tert*-Butoxycarbonylaminobenzoic acid, HBTU, HOBT, NMM, DMF (b) *m*-Chloroperoxybenzoic acid, DCM. (c) Potassium carbonate, $CH_3CN$, 60°C (d) Pd-C, $H_2$, ethanol (e) $(PhCO)_2O$, DMF or HBTU, HOBT, NMM, DMF, 3-(methylsulfonyl)benzoic acid (f) Dess-Martin periodinane, DCM (g) TFA, DCM

**Preparation of (2*S*)-2-{*N*-*tert*-butoxycarbonyl-*N'*-[(2*S*)-2-(4-*tert*-butoxycarbonylaminobenzoylamino)-4-methyl-pentanoyl]-hydrazinoyl methyl}-2,5-dihydro pyrrole-1-carboxylic acid benzyl ester (120)**

4-Methylmorpholine (44.7 µl, 0.407 mmol) was added to a solution of HBTU (77 mg, 0.204 mmol), 1-hydroxybenzotriazole monohydrate (31 mg, 0.204 mmol) and 4-(*tert*-butoxycarbonylamino)benzoic acid (48 mg, 0.204 mmol) in dimethylformamide (2.0 ml). The solution was stood for 5 minutes then added to (2*S*)-2-[*N'*-((2*S*)-2-amino-4-methyl-pentanoyl)-*N*-*tert*-butoxycarbonylhydrazino methyl]-2,5-dihydropyrrole-1-carboxylic acid benzyl ester **(101)** (94 mg, 0.204 mmol). The mixture was stirred for 1 hour 35 minutes then the solvents were removed *in vacuo* (water bath temperature < 37 °C). The residue was dissolved in dichloromethane (15 ml) then washed with pH 3 hydrochloric acid (5 ml), saturated aqueous sodium hydrogen carbonate solution (5 ml) and brine (5 ml), dried ($Na_2SO_4$), and the solvents removed *in vacuo.* The residue (166 mg) was purified by flash chromatography over silica eluting with ethyl acetate : heptane mixtures 1 : 9 to 4 : 6 to give (2*S*)-2-{*N*-*tert*-butoxycarbonyl-*N'*-[(2*S*)- 2-(4-*tert*-butoxycarbonylaminobenzoylamino)-4-methylpentanoyl]-hydrazinoylmethyl}-2,5-dihydropyrrole-1-carboxylic acid benzyl ester **(120)** as a white solid (116 mg, 84%). TLC ($R_f$ = 0.48, EtOAc : heptane 1 : 1), analytical HPLC $R_t$ = 22.296 min; HPLC-MS 580.4 $[M + 2H - Boc]^+$, 680.4 $[M + H]^+$.

**Preparation of (2*S*)-2-{*N*-*tert*-butoxycarbonyl-*N'*-[(2*S*)-2-(4-*tert*-butoxy carbonylaminobenzoylamino)-4-methyl-pentanoyl]-hydrazinomethyl}-6-oxa-3-aza-bicyclo [3.1.0]hexane-3-carboxylic acid benzyl ester (121)**

A solution of *meta*-chloroperoxybenzoic acid (57-86%, 411 mg, ~1.7 mmol) in dichloromethane (2.5 ml) was added to (2*S*)-2-{*N*-*tert*-butoxycarbonyl-*N'*-[(2*S'*)-2-(4-*tert*-butoxycarbonylaminobenzoylamino)-4-methylpentanoyl]-hydrazinoyl methyl}-2,5-dihydropyrrole-1-carboxylic acid benzyl ester **(120)** (115 mg, 0.169 mmol) under an atmosphere of nitrogen. The solution was stirred for 20 hours then dichloromethane (15 ml) was added and the solution twice washed with a mixture of water (5 ml) and aqueous saturated sodium hydrogen carbonate solution (5 ml), dried ($Na_2SO_4$), and the solvents removed *in vacuo.* The residue (127 mg) was purified by flash chromatography over silica eluting with ethyl acetate : heptane mixtures 1 : 9 to 1 : 1 to give (2*S*)-2-{*N*-*tert*-butoxycarbonyl-*N'*-[(2*S*)-2-(4-*tert*-butoxycarbonylaminobenzoylamino)-4-methylpentanoyl]-hydrazinomethyl}-6-oxa-3-aza-bicyclo[3.1.0] hexane-3-carboxylic acid benzyl ester **(121)** as a white solid (63.7 mg, 54%). TLC ($R_f$ = 0.25, EtOAc : heptane 1 : 1), analytical HPLC $R_t$ = 21.723 min; HPLC-MS 596.4 $[M + 2H - Boc]^+$, 696.4 $[M + H]^+$, 718.4 $[M + Na]^+$.

**Preparation of (3a*R*, 6*S*, 6a*S*)-1-[(2*S*)-2-(4-*tert*-butoxycarbonylamino benzoylamino)-4-methylpentanoyl]-6-hydroxyhexahydropyrrolo[3,2-*c*] pyrazole-2,4-dicarboxylic acid 4-benzyl ester 2-*tert*-butyl ester (122)**

A solution of (2*S*)-2-{*N*-*tert*-butoxycarbonyl-*N'*-[(2*S*)-2-(4-*tert*-butoxycarbonyl aminobenzoylamino)-4-methyl-pentanoyl]-hydrazinomethyl}-6-oxa-3-aza-bicyclo [3.1.0]hexane-3-carboxylic acid benzyl ester **(121)** (62.2 mg, 0.090 mmol) in acetonitrile (3.0 ml) was added to potassium carbonate (300 mg, 2.17 mmol). The suspension was placed under an atmosphere of nitrogen then heated at 60 °C whilst stirring for 3 hours before being allowed to cool to ambient temperature. The suspension was filtered then the filtrate concentrated *in vacuo.* The product was extracted into dichloromethane (15 ml) then washed with water (5 ml), dried ($Na_2SO_4$), and the solvents removed *in vacuo*. The residue was purified by flash chromatography over silica eluting with ethyl acetate : heptane mixtures 1 : 4 to 3 : 2 to give (3a*R*, 6*S*, 6a*S*)-1-[(2*S*)-2-(4-*tert*-butoxycarbonylaminobenzoylamino)-4-methylpentanoyl]-6-hydroxyhexahydropyrrolo[3,2-*c*]pyra zole-2,4-dicarboxylic acid 4-benzyl ester 2-*tert*-butyl ester **(122)** as a white solid (36.7 mg, 59%). TLC ($R_f$ = 0.28, EtOAc : heptane 1 : 1), analytical HPLC $R_t$ = 22.528 min; HPLC-MS 333.3, 596.4 $[M + 2H - Boc]^+$, 640.4 $[M + 2H - Bu]^+$, 696.4 $[M + H]^+$.

**Preparation of (3a*R*, 6*S*, 6a*S*)-1-[(2*S*)-2-(4-*tert*-butoxycarbonylamino benzoylamino)-4-methylpentanoyl]-6-hydroxyhexahydropyrrolo[3,2-*c*] pyrazole-2-carboxylic acid *tert*-butyl ester (123)**

Under an atmosphere of nitrogen ethanol (2.6 ml) was added to a mixture of (3a*R*, 6*S*, 6a*S*)-1-[(2*S*)-2-(4-*tert*-butoxycarbonylaminobenzoylamino)-4-methyl pentanoyl]-6-hydroxyhexahydropyrrolo[3,2-*c*]pyrazole-2,4-dicarboxylic acid 4-benzyl ester 2-*tert*-butyl ester **(122)** (35.2 mg, 0.051 mmol) and 10% palladium on charcoal (20 mg) whilst stirring. The nitrogen was replaced by hydrogen then stirring continued for 1 hour. The hydrogen was replaced by nitrogen then the mixture filtered through celite. The filter cake was washed with ethanol (20 ml) then the filtrate concentrated *in vacuo*. The residue (28.3 mg, 100%) was used without further purification. Analytical HPLC $R_t$ = 17.437 min; HPLC-MS 462.3 $[M + 2H - Boc]^+$, 562.4 $[M + H]^+$ for (3a*R*, 6*S*, 6a*S*)-1-[(2*S*)-2-(4-*tert*-butoxycarbonylaminobenzoylamino)-4-methylpentanoyl]-6-hydroxyhexahydro pyrrolo[3,2-*c*]pyrazole-2-carboxylic acid *tert*-butyl ester **(123).**

**Preparation of (3a*R*, 6*S*, 6a*S*)-4-benzoyl-1-[(2*S*)-2-(4-*tert*-butoxycarbonyl aminobenzoylamino)-4-methylpentanoyl]-6-hydroxyhexahydropyrrolo[3,2-*c*] pyrazole-2-carboxylic acid *tert*-butyl ester (124)**

Benzoic anhydride (5.3 mg, 0.024 mmol), dimethylformamide (0.275 ml) then 4-methylmorpholine (5.2 μl, 0.047 mmol) were added consecutively to (3a*R*, 6*S*, 6a*S*)-1-[(2*S*)-2-(4-*tert*-butoxycarbonylaminobenzoylamino)-4-methylpentanoyl]-6-hydroxy hexahydropyrrolo[3,2-*c*]pyrazole-2-carboxylic acid *tert*-butyl ester **(123)** (12.6 mg, 0.0225 mmol, prepared as above). The solution was stirred for 90 minutes then the majority of solvents were removed *in vacuo*. The residue was dissolved in ethyl acetate (10 ml), then washed with saturated aqueous sodium hydrogen carbonate solution (5 ml), pH 3 hydrochloric acid (5 ml) and brine (5 ml), dried ($Na_2SO_4$), and the solvents removed *in vacuo*. The solid white residue (15.4 mg, 100%) was used without further purification. Data for (3a*R*, 6*S*, 6a*S*)-4-benzoyl-1-[(2*S*)-2-(4-*tert*-butoxycarbonylaminobenzoylamino)-4-methyl pentanoyl]-6-hydroxyhexahydro pyrrolo[3,2-*c*]pyrazole-2-carboxylic acid *tert*-butyl ester **(124):** TLC ($R_f$ = 0.25, EtOAc : heptane 2 : 1), analytical HPLC $R_t$ = 20.787 min; HPLC-MS 333.3, 566.4 $[M + 2H - Boc]^+$, 666.4 $[M + H]^+$.

**Preparation of (3a*R*, 6a*S*)-4-benzoyl-1-[(2*S*)-2-(4-*tert*-butoxycarbonylamino benzoylamino)4-methylpentanoyl]-6-oxo-hexahydropyrrolo[3,2-*c*]pyrazole-2-carboxylic acid *tert*-butyl ester (126)**

A solution of Dess-Martin periodinane (48 mg, 0.113 mmol) in dichloromethane (1.1 ml) was added to (3a*R*, 6*S*, 6a*S*)-4-benzoyl-1-[(2*S*)-2-(4-*tert*-butoxycarbonyl aminobenzoylamino)-4-methylpentanoyl]-6-hydroxyhexahydropyrrolo[3,2-*c*] pyrazole-2-carboxylic acid *tert*-butyl ester **(124)** (15.4 mg, 0.0225 mmol, prepared as above) under an atmosphere of nitrogen. The mixture was stirred for 3 hours then purified by flash chromatography over silica eluting with ethyl acetate : heptane mixtures 1 : 4 to 1 : 1 to give (3a*R*, 6a*S*)-4-benzoyl-1-[(2*S*)-2-(4-*tert*-butoxycarbonylaminobenzoylamino)-4-methylpentanoyl]-6-oxo-hexahydro pyrrolo[3,2-*c*]pyrazole-2-carboxylic acid *tert*-butyl ester **(126)** as an off-white solid (9.7 mg, 65%). TLC ($R_f$ = 0.28, EtOAc : heptane 2 : 1), analytical HPLC broad peak $R_t$ = 20.05-22.80 min; HPLC-MS single broad main UV peak 333.2, 664.4 $[M + H]^+$, 682.4 $[M + H_2O + H]^+$.

**Preparation of (3a*R*, 6a*S*)-4-amino-*N*-[(1*S*)-1-(4-benzoyl-6-oxo-hexahydro pyrrolo[3,2-*c*]pyrazole-1-carbonyl)-3-methylbutyl]benzamide** (EXAMPLE 249b)

Trifluoroacetic acid (0.05 ml) was added to (3a*R*, 6a*S*)-4-benzoyl-1-[(2*S*)-2-(4-*tert*-butoxycarbonylaminobenzoylamino)-4-methylpentanoyl]-6-oxo-hexahydro pyrrolo[3,2-*c*]pyrazole-2-carboxylic acid *tert*-butyl ester **(126)** (6.4 mg, 9.7 μmol) under an atmosphere of nitrogen. The solution was stirred for 2.5 hours then diluted with dichloromethane (1 ml) and cautiously added to saturated aqueous sodium hydrogen carbonate solution (1 ml). The dichloromethane was separated then washed with water (1 ml). The saturated aqueous sodium hydrogen carbonate solution was extracted with dichloromethane (0.5 ml) which was then washed with the water layer. The combined organic layers were dried ($Na_2SO_4$), and the solvents removed *in vacuo* to obtain (3a*R*, 6a*S*)-4-amino-*N*-[1-(4-benzoyl-6-oxo-hexahydropyrrolo[3,2-*c*]pyrazole-1-carbonyl)-3-methylbutyl]benzamide (EXAMPLE 249b) as a red solid (0.72 mg, 16%). Analytical HPLC broad peak $R_t$ = 11.0-12.1 min; HPLC-MS broad UV peak 464.2 $[M + H]^+$, 482.4 $[M + H_2O + H]^+$, 949.3 $[2M + Na]^+$, 967.4 $[2M + H_2O + Na]^+$, 985.3 $[2M + 2H_2O + Na]^+$.

**Preparation of (3a*R*, 6*S*, 6a*S*)-1-[(2*S*)-2-(4-*tert*-butoxycarbonyl aminobenzoy lamino)-4-methylpentanoyl]-6-hydroxy-4-(3-methanesulfonyl benzoyl)hexa hydropyrrolo[3,2-*c*]pyrazole-2-carboxylic acid *tert*-butyl ester (125)**

4-Methylmorpholine (5.7 μl, 0.052 mmol) was added to a solution of HBTU (9.9 mg, 0.026 mmol), 1-hydroxybenzotriazole monohydrate (4.0 mg, 0.026 mmol) and 3-(methylsulfonyl)benzoic acid (5.2 mg, 0.026 mmol) in dimethylformamide (0.3 ml). The solution was stood for 5 minutes then added to (3a*R*, 6*S*, 6a*S*)-1-[(2*S*)-2-(4-*tert*-butoxycarbonylaminobenzoylamino)-4-methylpentanoyl]-6-hydroxyhexahydro pyrrolo[3,2-*c*]pyrazole-2-carboxylic acid *tert*-butyl ester **(123)** (14.7 mg, 0.026 mmol, prepared as above). The mixture was stirred for 2.5 hour then the solvents were removed *in vacuo* (water bath temperature < 37 °C). The residue was dissolved in dichloromethane (10 ml) then washed with pH 3 hydrochloric acid (5 ml), saturated aqueous sodium hydrogen carbonate solution (5 ml) and brine (5 ml), dried ($Na_2SO_4$), and the solvents removed *in vacuo*. The pale yellow solid residue (19.7 mg, 100%) was used without further purification. Data for (3a*R*, 6*S*, 6a*S*)-1-[(2*S*)-2-(4-*tert*-butoxycarbonylaminobenzoylamino)-4-methylpentanoyl]-6-hydroxy-4-(3-methanesulfonylbenzoyl)hexahydropyrrolo [3,2-*c*]pyrazole-2-carboxylic acid *tert*-butyl ester **(125):** TLC ($R_f$ = 0.05, EtOAc : heptane 2 : 1), analytical HPLC $R_t$ = 19.945 min; HPLC-MS 333.3, 644.3 $[M + 2H - Boc]^+$, 688.3 $[M + 2H - Bu]^+$, 744.3 $[M + H]^+$, 766.3 $[M + Na]^+$.

**Preparation of (3a*R*, 6a*S*)-1-[(2*S*)-2-(4-*tert*-butoxycarbonylaminobenzoyl amino)-4-methylpentanoyl]-4-(3-methanesulfonylbenzoyl)-6-oxohexahydro pyrrolo[3,2-*c*] pyrazole-2-carboxylic acid *tert*-butyl ester (127)**

A solution of Dess-Martin periodinane (56 mg, 0.132 mmol) in dichloromethane (1.25 ml) was added to (3a*R*, 6*S*, 6a*S*)-1-[(2*S*)-2-(4-*tert*-butoxycarbonylamino benzoylamino)-4-methylpentanoyl]-6-hydroxy-4-(3-methanesulfonylbenzoyl) hexahydropyrrolo[3,2-*c*]pyrazole-2-carboxylic acid *tert*-butyl ester **(125)** (19.7 mg, 0.0262 mmol, prepared as above) under an atmosphere of nitrogen. The mixture was stirred for 4 hours then purified by flash chromatography over

silica eluting with ethyl acetate : heptane mixtures 3 : 7 to 4 : 1 to give (3a*R*, 6a*S*)-1-[(2*S*)-2-(4-*tert*-butoxycarbonylaminobenzoylamino)-4-methylpentanoyl]-4-(3-methanesulfonylbenzoyl)-6-oxohexahydropyrrolo[3,2-*c*] pyrazole-2-carboxylic acid *tert*-butyl ester **(127)** as an off-white solid (11.1 mg, 57%). TLC ($R_f$ = 0.10, EtOAc : heptane 2 : 1), analytical HPLC broad peak $R_t$ = 19.20-21.70 min; HPLC-MS single broad main UV peak 333.2, 642.3 [M + 2H - Boc]$^+$, 660.3 [M + 2H + $H_2O$ - Boc]$^+$, 686.3 [M + 2H - Bu]$^+$, 704.2 [M + 2H + $H_2O$ - Bu]$^+$, 742.3 [M + H]$^+$, 760.3 [M + $H_2O$ + H]$^+$.

**Preparation of (3a*R*, 6a*S*)-4-amino-*N*-{(1*S*)-1-[4-(3-methanesulfonylbenzoyl)-6-oxo-hexahydropyrrolo[3,2-*c*]pyrazole-1-carbonyl]-3-methylbutyl} benzamide** (EXAMPLE 249c)

Trifluoroacetic acid (0.05 ml) was added to (3a*R*, 6a*S*)-1-[(2*S*)-2-(4-*tert*-butoxycarbonylaminobenzoylamino)-4-methylpentanoyl]-4-(3-methanesulfonyl benzoyl)-6-oxohexahydropyrrolo[3,2-*c*]pyrazole-2-carboxylic acid *tert*-butyl ester **(127)** (4.93 mg, 6.7 μmol) under an atmosphere of nitrogen. The solution was stirred for 2.5 hours then diluted with dichloromethane (1 ml) and cautiously added to saturated aqueous sodium hydrogen carbonate solution (1 ml). The dichloromethane was separated then washed with water (1 ml). The saturated aqueous sodium hydrogen carbonate solution was extracted with dichloromethane (0.5 ml) which was then washed with the water layer. The combined organic layers were dried ($Na_2SO_4$), and the solvents removed *in vacuo*, to obtain (3a*R*, 6a*S*)-4-amino-*N*-{(1*S*)-1-[4-(3-methanesulfonylbenzoyl)-6-oxo-hexahydropyrrolo [3,2-*c*]pyrazole-1-carbonyl]-3-methylbutyllbenzamide (EXAMPLE 249c) as a red solid (0.66 mg, 18%). Analytical HPLC broad peak $R_t$ = 10.2-11.4 min; HPLC-MS broad UV peak 233.1, 542.2 [M + H]$^+$, 560.2 [M + $H_2O$ + H]$^+$.

EXAMPLES 250 to 295 were prepared as detailed for EXAMPLES 1 and 119, substituting the appropriate carboxylic acids as required and are inhibitors of cathepsin S with Ki ranging from 10-5000n;

EXAMPLE 250. (3*aR*, 6*aS*)-Thiophene-3-carboxylic acid [(1*S*)-2-(4-benzoyl-6-oxo-hexahydro-pyrrolo[3,2-*b*]pyrrol-1-yl)-1-cyclohexylmethyl-2-oxo-ethyl]-amide

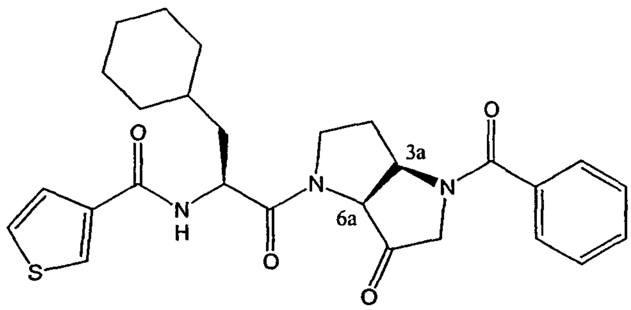

HPLC Rt = 16.4-17.2 mins (> 90%), HPLC-MS 494.2 [M + H]$^+$, 1009.4 [2M + Na]$^+$.

EXAMPLE 251. (3a*R*, 6*aS*)-Thiophene-3-carboxylic acid [(1*R*)-2-(4-benzoyl-6-oxo-hexahydro-pyrrolo[3,2-*b*]pyrrol-1-yl)-1-benzylsulfanylmethyl-2-oxo-ethyl]-amide

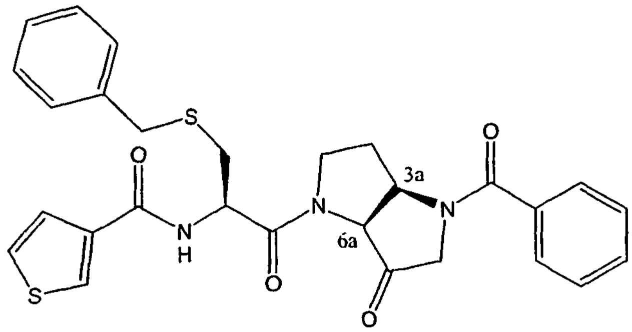

HPLC Rt = 14.78 mins (> 90%), HPLC-MS 534.1 [M + H]+.

EXAMPLE 252. (3a*R*, 6*aS*)-Benzo[*b*]thiophene-2-carboxylic acid [(1*R*)-2-(4-benzoyl-6-oxo-hexahydro-pyrrolo[3,2-*b*]pyrrol-1-yl)-1-benzylsulfanylmethyl-2-oxo-ethyl]-amide

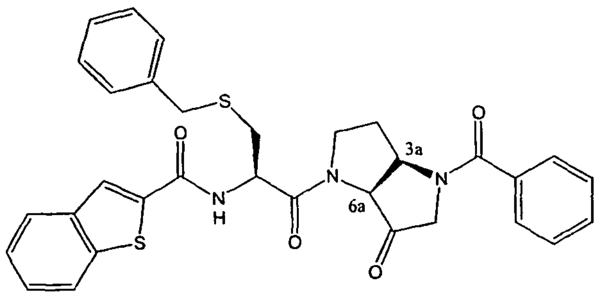

HPLC Rt = 18.5-19.8 mins (> 85%), HPLC-MS 584.1 [M + H]+.

EXAMPLE 253. (3a*R*, 6*aS*)-Benzo[*b*]thiophene-3-carboxylic acid [(1*R*)-2-(4-benzoyl-6-oxo-hexahydro-pyrrolo[3,2-*b*]pyrrol-1-yl)-1-benzylsulfanylmethyl-2-oxo-ethyl]-amide

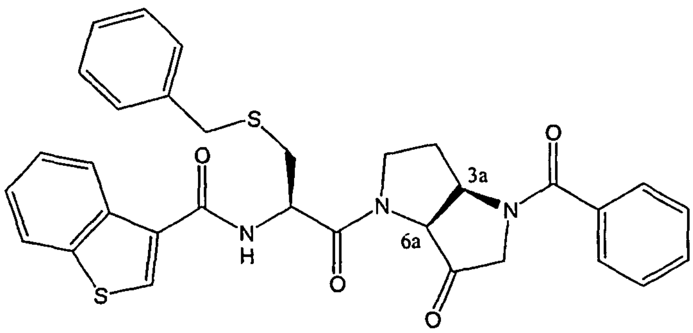

HPLC Rt = 17.8-19.0 mins (> 80%), HPLC-MS 584.2 [M + H]+.

EXAMPLE 254. (3a*R*, 6a*S*)-Benzo[*b*]thiophene-3-carboxylic acid [(1*S*)-2-(4-benzoyl-6-oxo-hexahydro-pyrrolo[3,2-*b*]pyrrol-1-yl)-1-cyclohexylmethyl-2-oxo-ethyl]-amide

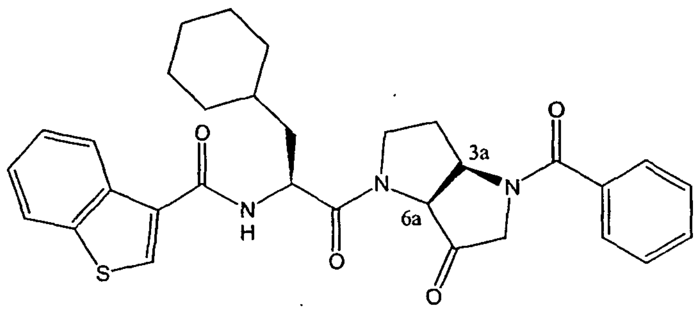

HPLC Rt = 19.5-20.5 mins (> 75%), HPLC-MS 544.1 $[M + H]^+$.

EXAMPLE 255. (3a*R*, 6a*S*)-Thiophene-3-carboxylic acid {(1*S*)-1-cyclohexylrnethyl-2-[4-(naphthalene-1-carbonyl)-6-oxo-hexahydro-pyrrolo[3,2-*b*]pyrrol-1-yl]-2-oxo-ethyl}-amide

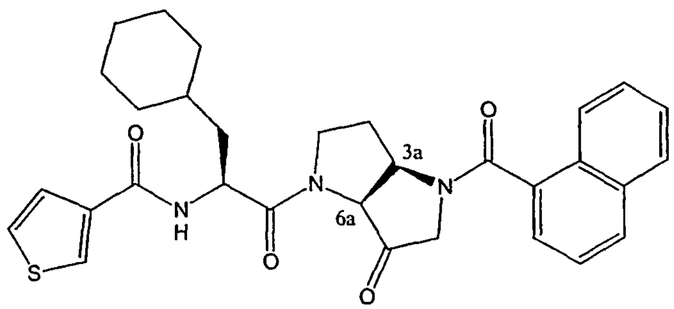

HPLC Rt =18.3-19.5 mins (> 80%), HPLC-MS 544.1 $[M + H]^+$.

EXAMPLE 256. (3a*R*, 6a*S*)-Thiophene-3-carboxylic acid {(1*S*)-1-cyclohexylmethyl-2-[4-(naphthalene-2-carbonyl)-6-oxo-hexahydro-pyrrolo[3,2-*b*]pyrrol-1-yl]-2-oxo-ethyl}-amide

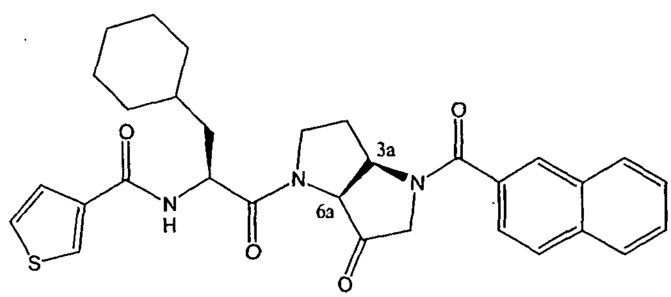

HPLC Rt = 18.9-19.7 mins (> 85%), HPLC-MS 544.1 $[M + H]^+$.

EXAMPLE 257. (3a*R*, 6*aS*)-Thiophene-3-carboxylic acid {(1*S*)-1-cyclohexylmethyl-2-oxo-2-[6-oxo-4-(4-[1,2,4]triazol-1-yl-benzoyl)-hexahydro-pyrrolo[3,2-*b*]pyrrol-1-yl]-ethyl}-amide

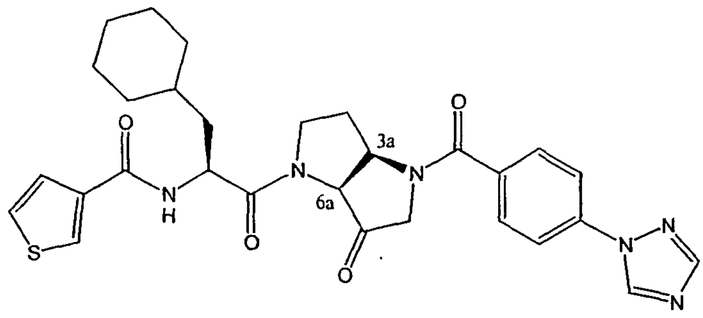

HPLC Rt = 15.5-16.5 mins (> 85%), HPLC-MS 561.2 $[M + H]^+$.

EXAMPLE 258. (3a*R*, 6*aS*)-Thiophene-3-carboxylic acid {(1*S*)-1-cyclohexylmethyl-2-oxo-2-[6-oxo-4-(5-phenyl-thiophene-2-carbonyl)-hexahydro-pyrrolo[3,2-*b*]pyrrol-1-yl]-ethyll-amide

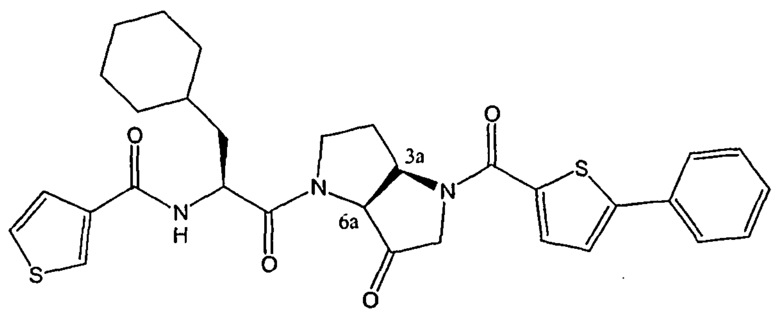

HPLC Rt = 19.5-20.2 mins (> 85%), HPLC-MS 576.1 $[M + H]^+$.

EXAMPLE 259. (3*aR*, 6*aS*)-*N*-[(1*S*)-2-(4-Benzoyl-6-oxo-hexahydro-pyrrolo[3,2-*b*]pyrrol-1-yl)-1-cyclohexylmethyl-2-oxo-ethyl]-3-phenoxy-benzamide

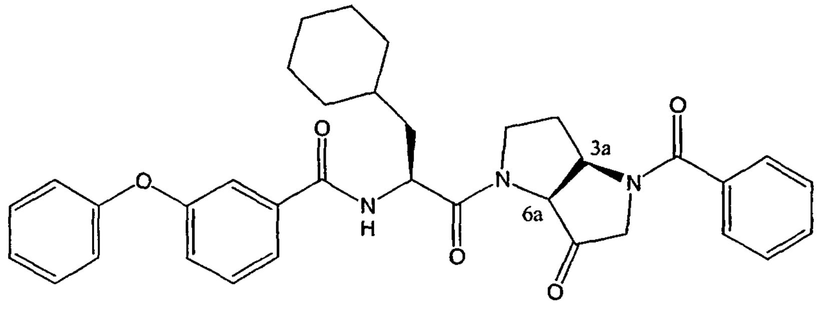

HPLC Rt = 19.4-20.3 mins (> 85%); HPLC-MS 580.2 $[M + H]^+$.

EXAMPLE 260. (3a*R*, 6a*S*)-*N*-[(1*R*)-2-(4-Benzoyl-6-oxo-hexahydro-pyrrolo[3,2-*b*]pyrrol-1-yl)-1-benzylsulfanylmethyl-2-oxo-ethyl]-3-phenoxy-benzamide

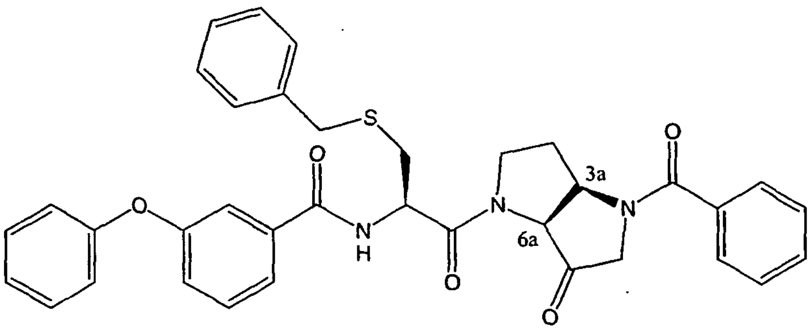

HPLC Rt =18.3-19.6 mins (> 90%), HPLC-MS 620.2 $[M + H]^+$.

EXAMPLE 261. (3a*R*, 6a*S*)-Thiophene-3-carboxylic acid {(1*R*)-1-benzylsulfanyl methyl-2-[4-(2-methanesulfonyl-benzoyl)-6-oxo-hexahydro-pyrrolo[3,2-*b*]pyrrol-1-yl]-2-oxo-ethyl}-amide

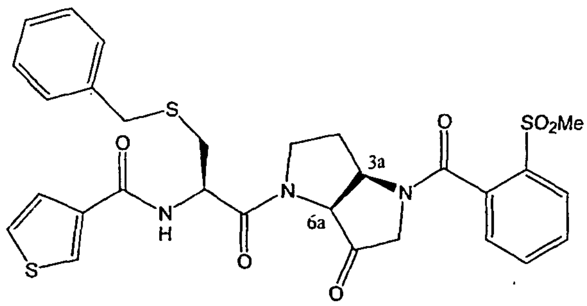

HPLC Rt = 14.5-15.2 mins (> 80%), HPLC-MS 612.0 $[M + H]^+$.

EXAMPLE 262. (3a*R*, 6a*S*)-Thiophene-3-carboxylic acid {(*1R*)-1-benzylsulfanyl methyl-2-[4-(3-methanesulfonyl-benzoyl)-6-oxo-hexahydro-pyrrolo[3,2-*b*]pyrrol-1-yl]-2-oxo-ethyl}-amide

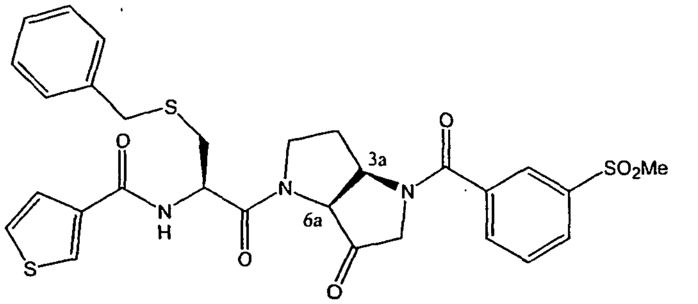

HPLC Rt = 14.09 mins (> 90%), HPLC-MS 612.1 [M + H]$^+$.

EXAMPLE 263. (3a*R*, 6a*S*)-Thiophene-3-carboxylic acid {(1*R*)-1-benzylsulfanyl methyl-2-[4-(2-nitro-benzoyl)-6-oxo-hexahydro-pyrrolo[3,2-*b*]pyrrol-1-yl]-2-oxoethyl}-amide

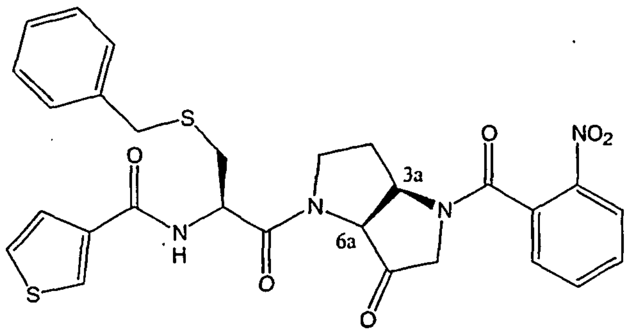

HPLC Rt = 14.93 mins (> 90%), HPLC-MS 579.0 [M + H]$^+$.

EXAMPLE 264. (3a*R*, 6a*S*)-Thiophene-3-carboxylic acid {(1*R*)-1-benzylsulfanyl methyl-2-[4-(3-nitro-benzoyl)-6-oxo-hexahydro-pyrrolo[3,2-*b*]pyrrol-1-yl]-2-oxoethyl}-amide

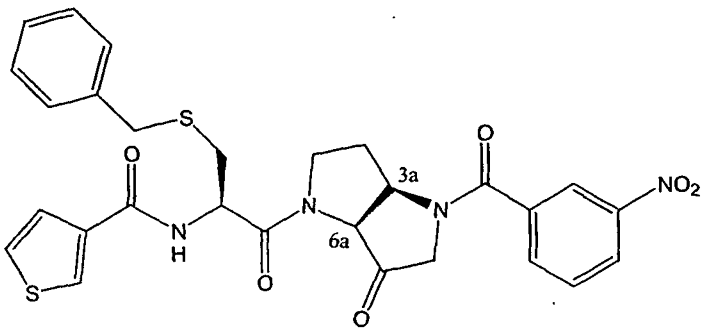

HPLC Rt = 15.53 mins (> 85%), HPLC-MS 579.0 [M + H]$^+$.

EXAMPLE 265. (3a*R*, 6a*S*)-Thiophene-3-carboxylic acid {(1*R*)-1-benzylsulfanyl methyl-2-[4-(4-nitro-benzoyl)-6-oxo-hexahydro-pyrrolo[3,2-*b*]pyrrol-1-yl]-2-oxoethyl}-amide

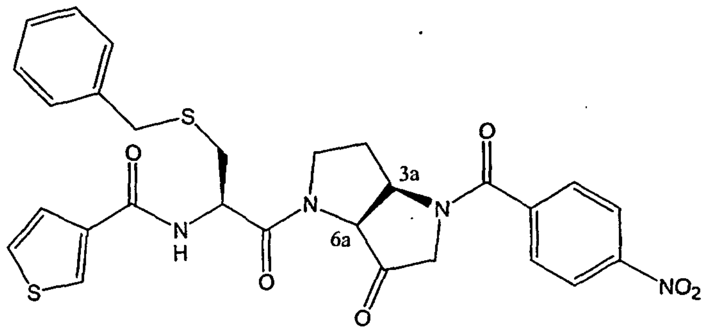

HPLC Rt = 15.41 mins (> 90%), HPLC-MS 579.1 $[M + H]^+$.

EXAMPLE 266. (3a*R*, 6a*S*)-Thiophene-3-carboxylic acid {(1*R*)-1-benzylsulfanyl methyl-2-[4-(hexahydro-2,5-methano-pentalene-3a-carbonyl)-6-oxo-hexahydro-pyrrolo[3,2-*b*]pyrrol-1-yl]-2-oxo-ethyl}-amide

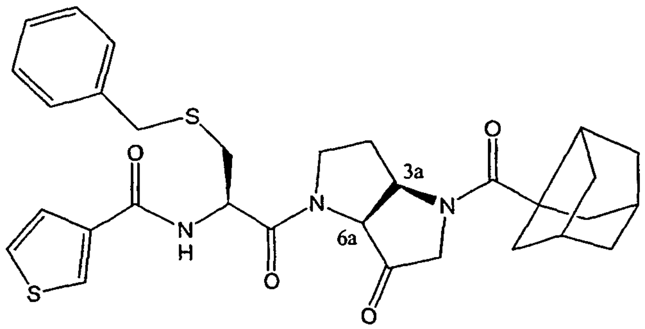

HPLC Rt = 17.0-18.2 mins (> 80%), HPLC-MS 578.1 $[M + H]^+$.

EXAMPLE 267. (3a*R*, 6a*S*)-Thiophene-3-carboxylic acid {(1*R*)-1-benzylsulfanyl methyl-2-[4-(4-hydroxy-benzoyl)-6-oxo-hexahydro-pyrrolo[3,2-*b*]pyrrol-1-yl]-2-oxo-ethyl}-amide

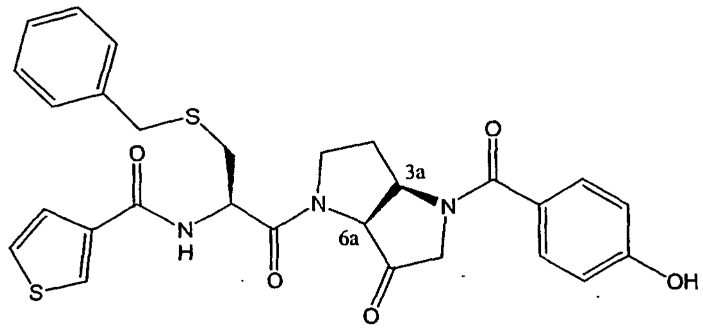

HPLC Rt = 13.43 mins (> 80%), HPLC-MS 550.0 [M + H]$^+$.

EXAMPLE 268. (3a*R*, 6*aS*)-Thiophene-3-carboxylic acid {(1*R*)-2-[4-(4-aminobenzoyl)-6-oxo-hexahydro-pyrrolo[3,2-*b*]pytrol-1-yl]-1-benzylsulfanylmethyl-2-oxo-ethyl}-amide

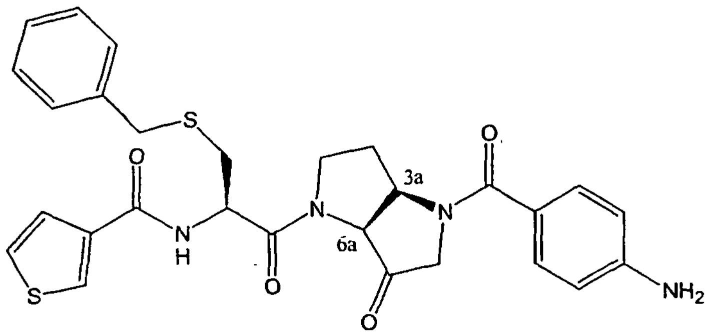

HPLC Rt =11.8-12.4 mins (> 75%), HPLC-MS 549. [M + H]$^+$.

EXAMPLE 269. (3a*R*, 6a*S*)-*N*-[(1*R*)-2-(4-Benzoyl-6-oxo-hexahydro-pyrrolo[3,2-*b*]pyrrol-1-yl)-1-benzylsulfanylmethyl-2-oxo-ethyl]-2,2-diphenyl-acetamide

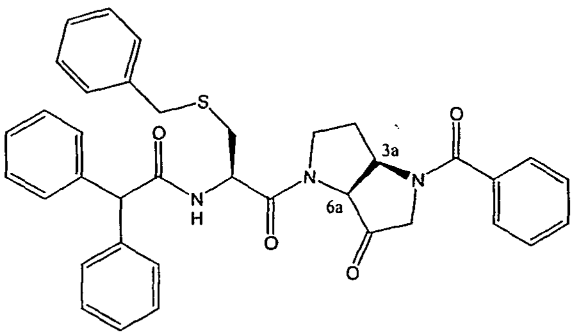

HPLC Rt = 19.1-20.3 mins (> 90%), HPLC-MS 618.2 [M + H]$^+$.

EXAMPLE 270. (3a*R*, 6a*S*)-*N*-[(1*S*)-2-(4-Benzoyl-6-oxo-hexahydro-pyrrolo[3,2-*b*]pyrrol-1-yl)-1-cyclohexylmethyl-2-oxo-ethyl]-2,2-diphenyl-acetamide

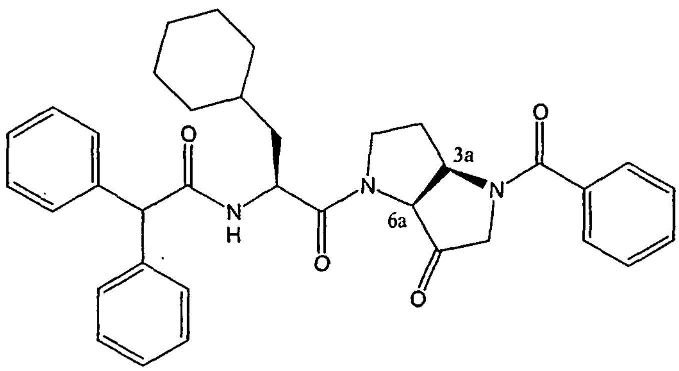

HPLC Rt = 19.9-21.0 mins (> 90%), HPLC-MS 578.3 [M + H]$^+$.

EXAMPLE 271. (3a*R*, 6a*S*)-*N*-{(1*S*)-1-[4-(Benzo[*b*]thiophene-3-carbonyl)-6-oxo-hexahydro-pyrrolo[3,2-*b*]pyrrole-1-carbonyl]-3-methyl-butyl}-2,2-diphenyl-acetamide

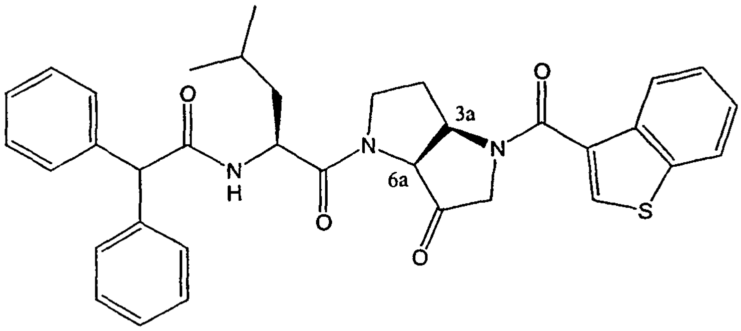

HPLC Rt = 19.4-20.7 mins (> 90%), HPLC-MS 594.2 [M + H]$^+$.

EXAMPLE 272. (3a*R*, 6a*S*)-Thiophene-3-carboxylic acid {(1*R*)-1-benzylsulfanyl methyl-2-oxo-2-[6-oxo-4-(thiophene-3-carbonyl)-hexahydro-pyrrolo[3,2-*b*]pyrrol-1-yl]-ethyl}-amide

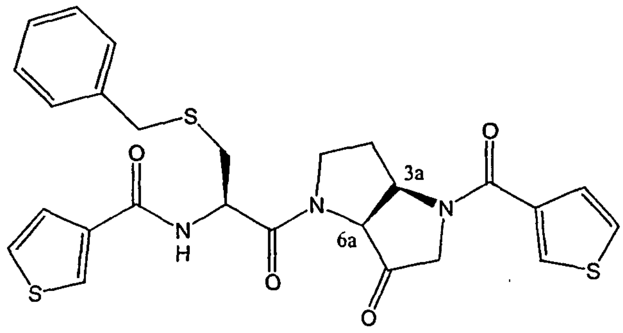

HPLC Rt = 14.56 mins (> 90%), HPLC-MS 540.0 [M + H]$^+$.

EXAMPLE 273. (3a*R*, 6a*S*)-Thiophene-3-carboxylic acid [(1*R*)-1-benzylsulfanyl methyl-2-(4-cyclohexanecarbonyl-6-oxo-hexahydro-pyrrolo[3,2-*b*]pyrrol-1-yl)-2-oxo-ethyl]-amide

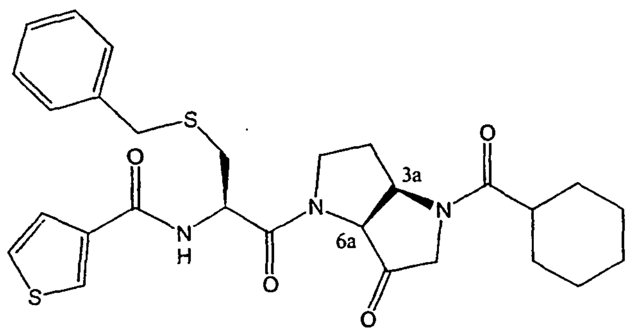

HPLC Rt = 15.5-16.4 mins (> 75%), HPLC-MS 540.1 [M + H]$^+$.

EXAMPLE 274. (3a*R*, 6a*S*)-Thiophene-3-carboxylic acid {(1*R*)-1-benzylsulfanyl methyl-2-[4-(2-methyl-benzoyl)-6-oxo-hexahydro-pyrrolo[3,2-*b*]pyrrol-1-yl]-2-oxo-ethyl}-amide

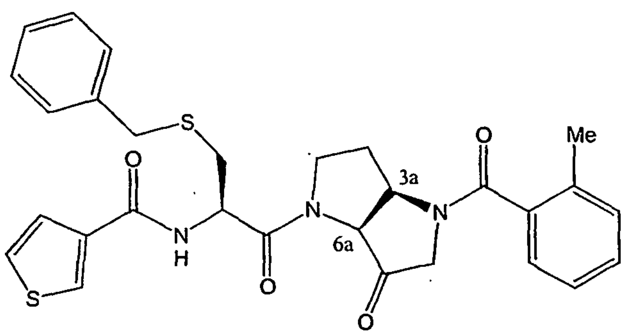

HPLC Rt = 16.0-17.9 mins (> 90%), HPLC-MS 548.1 [M + H]$^+$.

EXAMPLE 275. (3a*R*, 6a*S*)-Thiophene-3-carboxylic acid {(1*R*)-1-benzylsulfanyl methyl-2-[4-(3-methyl-benzoyl)-6-oxo-hexahydro-pyrrolo[3,2-*b*]pyrrol-1-yl]-2-oxo-ethyl}-amide

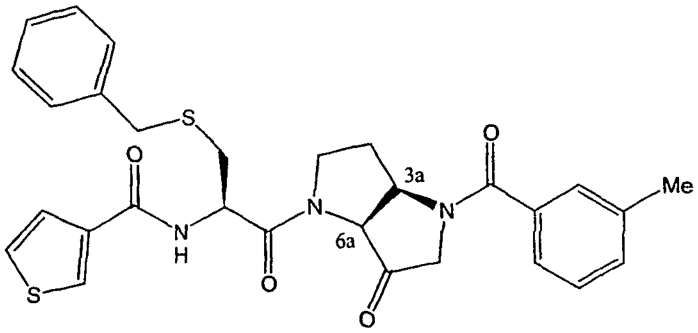

HPLC Rt = 16.3-17.8 mins (> 90%), HPLC-MS 548.1 $[M + H]^+$.

EXAMPLE 276. (3a*R*, 6a*S*)-Thiophene-3-carboxylic acid {(1*R*)-1-benzylsulfanyl methyl-2-[4-(4-methyl-benzoyl)-6-oxo-hexahydro-pyrrolo[3,2-*b*]pyrrol-1-yl]-2-oxo-ethyl}-amide

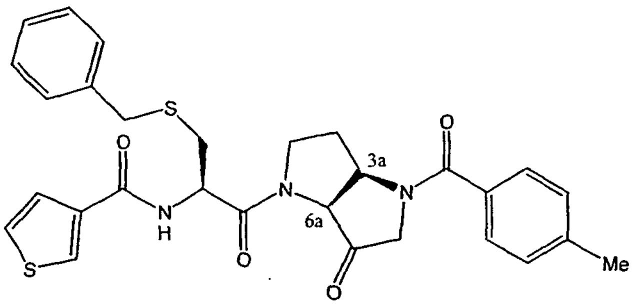

HPLC Rt = 15.5-16.8 mins (> 90%), HPLC-MS 548.1 $[M + H]^+$.

EXAMPLE 277. (3a*R*, 6a*S*)-Morpholine-4-carboxylic acid [(1*R*)-2-(4-benzoyl-6-oxo-hexahydro-pyrrolo[3,2-*b*]pyrrol-1-yl)-1-benzylsulfanylmethyl-2-oxo-ethyl]-amide

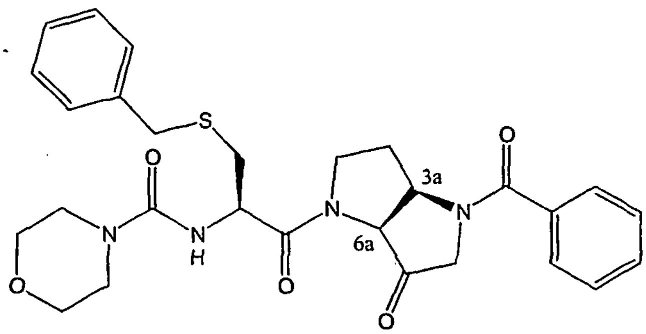

HPLC Rt = 12.5-13.8 mins (> 80%), HPLC-MS 537.1 $[M + H]^+$.

EXAMPLE 278. (3a*R*, 6a*S*)-3-Aminomethyl-*N*-[(1*R*)-2-(4-benzoyl-6-oxo-hexahydro-pyrrolo[3,2-*b*]pyrrol-1-yl)-1-benzylsulfanylmethyl-2-oxo-ethyl]-benzamide

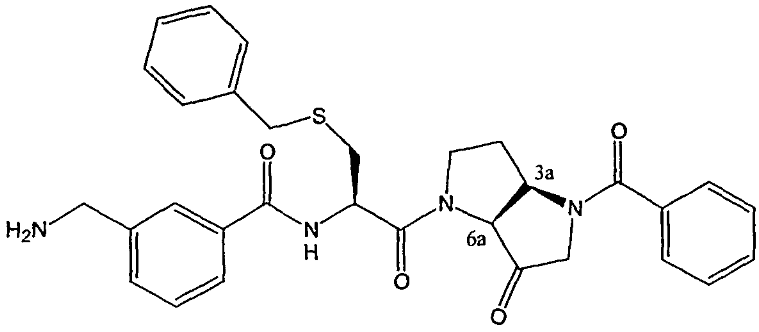

HPLC Rt = 12.0-13.4 mins (> 80%), HPLC-MS 557.2 $[M + H]^+$.

EXAMPLE 279. (3a*R*, 6a*S*)-*N*-{(1*S*)-1-Cydohexylmethyl-2-[4-(2-methanesulfonyl-benzoyl)-6-oxo-hexahydro- pyrrolo[3,2-*b*]pyrrol-1-yl]-2-oxoethyl}-2,2-diphenyl-acetamide

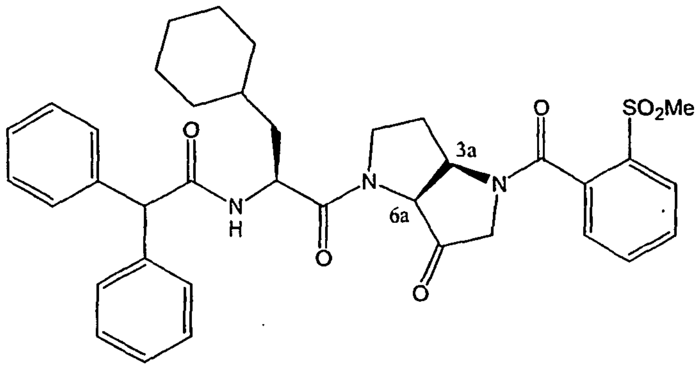

HPLC Rt = 20.1-21.4 mins (> 90%), HPLC-MS 656.2 $[M + H]^+$.

EXAMPLE 280. (3a*R*, 6a*S*)-Morpholine-4-carboxylic acid [(1*S*)-2-(4-benzoyl-6-oxo-hexahydro-pyrrolo[3,2-*b*]pyrrol-1-yl)-1-cyclohexylmethyl-2-oxo-ethyl]-amide

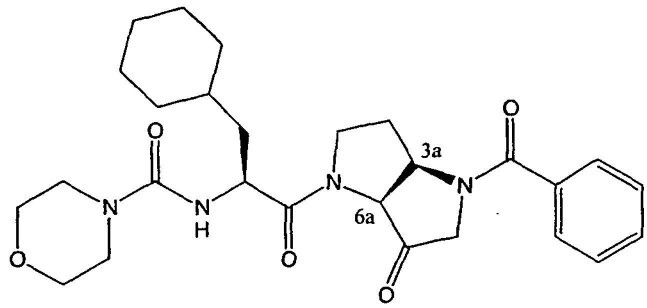

HPLC Rt = 14.96 mins (> 95%), HPLC-MS 497.2 [M + H]$^+$, 515.2 [M + H + $H_2O$]$^+$.

EXAMPLE 281. (3a*R*, 6a*S*)-Benzothiazole-6-carboxylic acid [(1*R*)-2-(4-benzoyl-6-oxo-hexahydro-pytrolo[3,2-*b*]pytrol-1-yl)-1-benzylsulfanylmethyl-2-oxoethyl]-amide

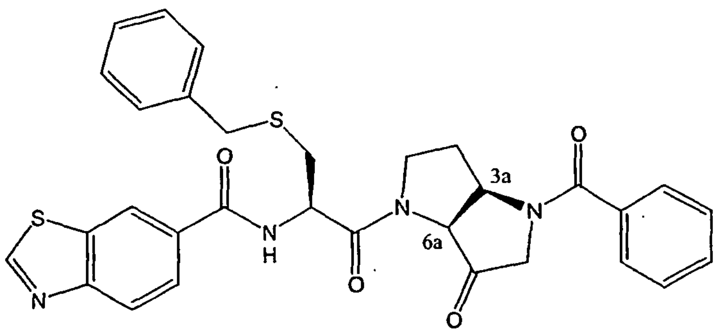

HPLC Rt = 15.15 mins (> 85%), HPLC-MS 585.1 [M + H]$^+$, 603.1 [M + H + $H_2O$]$^+$.

EXAMPLE 282. (3a*R*, 6a*S*)-Thiophene-2-carboxylic acid [(1*R*)-2-(4-benzoyl-6-oxo-hexahydro-pyrrolo[3,2-*b*]pyrrol-1-yl)-1-benzylsulfanylmethyl-2-oxo-ethyl]-amide

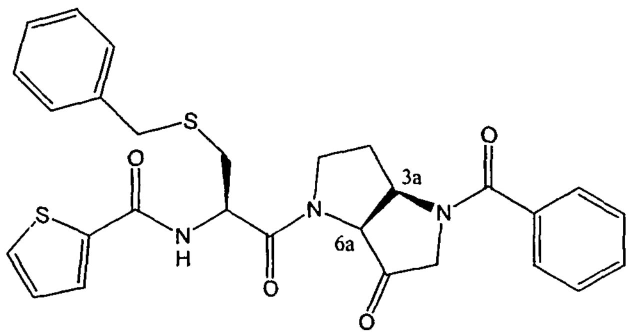

HPLC Rt = 15.06 mins (> 85%), HPLC-MS 534.0 [M + H]$^+$.

EXAMPLE 283. (3a*R*, 6a*S*)-Furan-2-carboxylic acid [(1*R*)-2-(4-benzoyl-6-oxo-hexahydro-pyrrolo[3,2-*b*]pyrrol-1-yl)-1-benzylsulfanylmethyl-2-oxo-ethyl]-amide

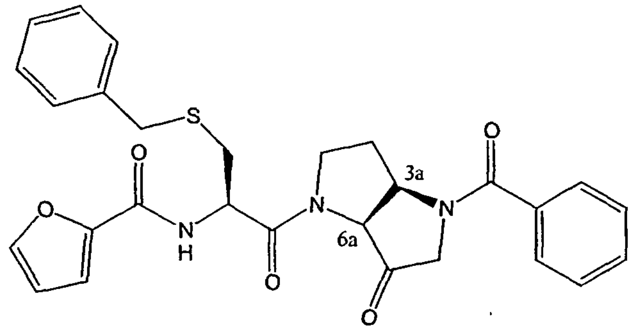

HPLC Rt = 14.1-15.4 mins (> 80%), HPLC-MS 518.1 $[M + H]^+$, 536.1 $[M + H + H_2O]^+$.

EXAMPLE 284. (3a*R*, 6a*S*)-Furan-3-carboxylic acid [(1*R*)-2-(4-benzoyl-6-oxo-hexahydro-pyrrolo[3,2-*b*]pyrrol-1-yl)-1-benzylsulfanylmethyl-2-oxo-ethyl]-arnide

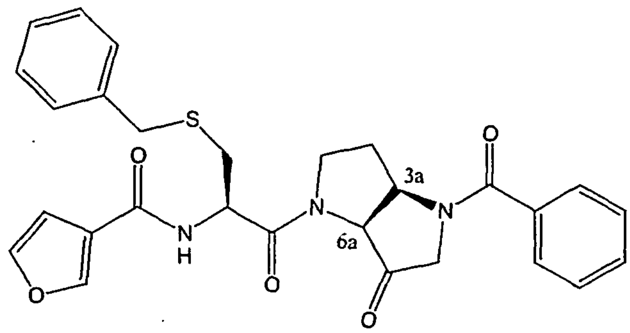

HPLC Rt = 15.7-17.3 mins (> 85%), HPLC-MS 518.1 $[M + H]^+$.

EXAMPLE 285. (3a*R*, 6a*S*)-*N*-[(1*R*)-2-(4-Benzoyl-6-oxo-hexahydro-pyrrolo[3,2-*b*]pyrrol-1-yl)-1-benzylsulfanylmethyl-2-oxo-etllyl]-benzarnide

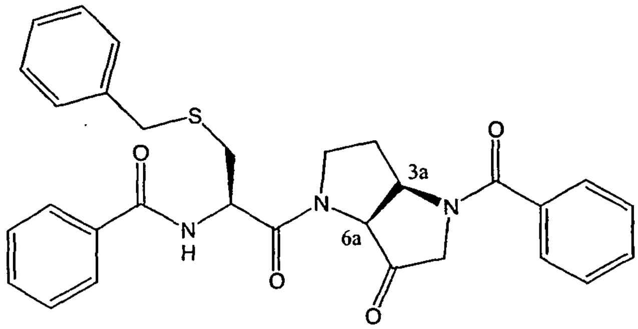

HPLC Rt = 15.34 mins (> 90%), HPLC-MS 528.1 [M + H]+.

EXAMPLE 286. (3a*R*, 6a*S*)-Benzothiazole-6-carboxylic acid [(1*S*)-2-(4-benzoyl-6-oxo-hexahydro-pyrrolo[3,2-*b*]pyrrol-1-yl)-1-cyclohexylmethyl-2-oxo-ethyl]-amide

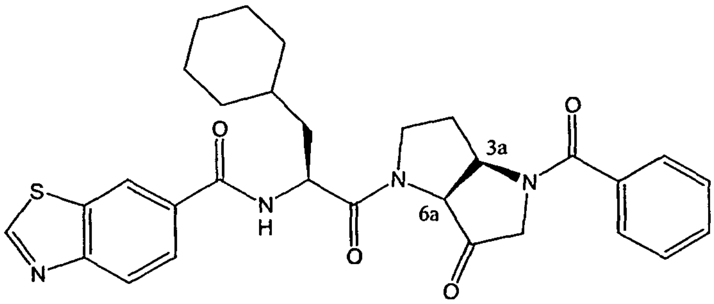

HPLC Rt = 18.25 mins (> 90%), HPLC-MS 5452 [M + H]+.

EXAMPLE 287. (3a*R*, 6a*S*)-Thiophene-2-carboxylic acid [(1*S*)-2-(4-benzoyl-6-oxo-hexahydro-pyrrolo[3,2-*b*]pyrrol-1-yl)-1-cydohexylmethyl-2-oxo-etllyl]-amide

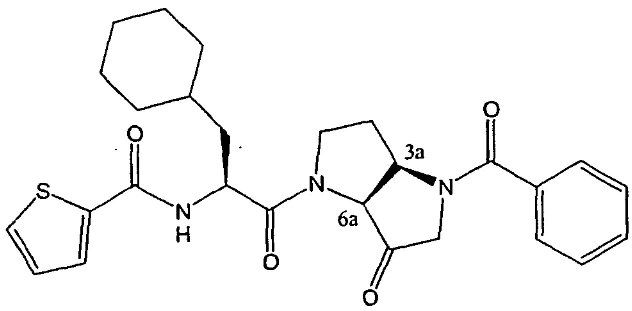

HPLC Rt = 18.28 mins (> 90%), HPLC-MS 494.1 [M + H]+.

EXAMPLE 288. (3a*R*, 6a*S*)-Furan-2-carboxylic acid [(1*S*)-2-(4-benzoyl-6-oxo-hexahydro-pyrrolo[3,2-*b*]pyrrol-1-yl)-1-cyclohexylmethyl-2-oxo-ethyl]-amide

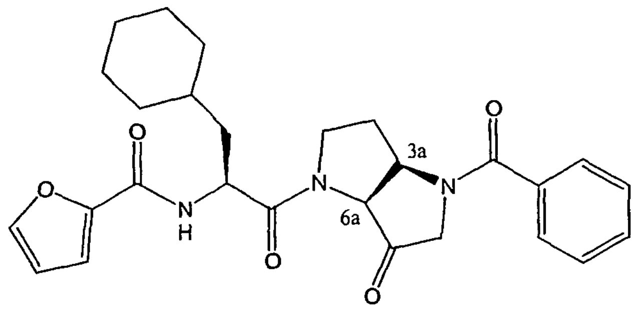

HPLC Rt = 16.66 mins (> 90%), HPLC-MS 478.1 [M + H]$^+$, 977.3 [2M + Na]$^+$.

EXAMPLE 289. (3a*R*, 6a*S*)-Furan-3-carboxylic acid [(1*S*)-2-(4-benzoyl-6-oxo-hexahydro-pyrrolo[3,2-*b*]pyrrol-1-yl)-1-cyclohexylmethyl-2-oxo-ethyl]-amide

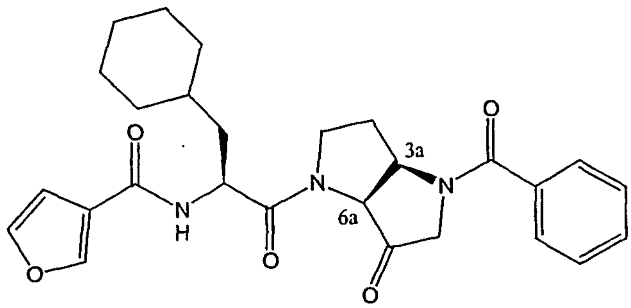

HPLC Rt = 16.37 mins (> 90%), HPLC-MS 478.1 [M + H]$^+$, 977.3 [2M + Na]$^+$.

EXAMPLE 290. (3a*R*, 6a*S*)-*N*-[(1*S*)-2-(4-Benzoyl-6-oxo-hexahydro-pyrrolo[3,2-*b*]pyrrol-1-yl)-1-cyclohexylmethyl-2-oxo-ethyl]-benzamide

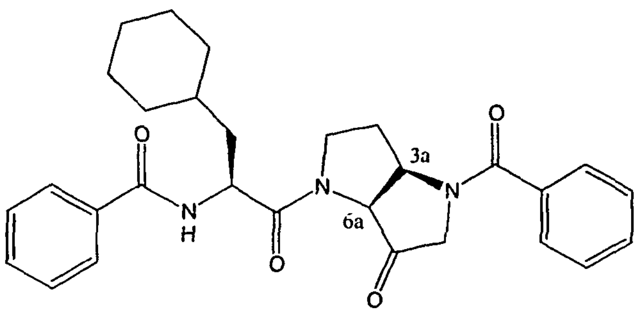

HPLC Rt = 16.71 mins (> 95%), HPLC-MS 488.2 [M + H]+, 997.3 [2M + Na]+.

EXAMPLE 291. (3a*R*, 6a*S*)-Thiophene-3-carboxylic acid {(1*S*)-1-cyclohexylmethyl-2-[4-(2-methyl-benzoyl)-6-oxo-hexahydro-pyrrolo[3,2-*b*]pyrrol-1-yl]-2-oxo-ethyl) -amide

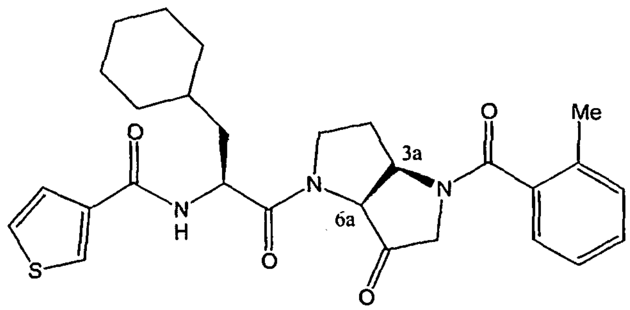

HPLC Rt = 15.65 mins (> 95%), HPLC-MS 508.2 [M + H]+, 526.2 [M + H + $H_2O$]+.

EXAMPLE 292. (3a*R*, 6a*S*)-niophene-3-carboxylic acid {(1*S*)-1-cyclohexylmethyl-2-[4-(3-methyl-benzoyl)-6-oxo-hexahydro-pyrrolo[3,2-*b*]pyrrol-1-yl]-2-oxo-ethyl}-amide

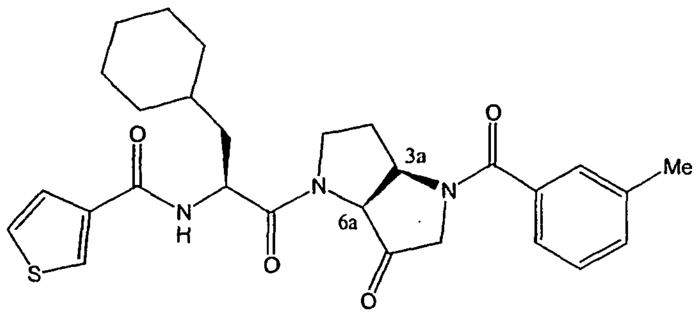

HPLC Rt = 16.10 mins (> 90%), HPLC-MS 508.1 [M + H]+.

EXAMPLE 293. (3a*R*, 6a*S*)-Thiophene-3-carboxylic acid {(1*S*)-1-cyclohexylmethyl-2-[4-(4-methyl-benzoyl)-6-oxo-hexahydro-pyrrolo[3,2-*b*]pyrrol-1-yl]-2-oxo-ethyl}-amide

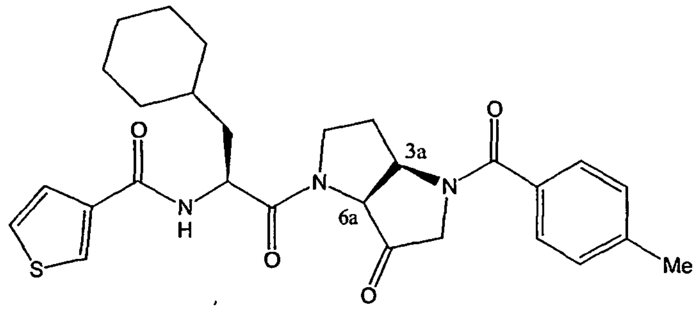

HPLC Rt = 15.88 mins (> 90%), HPLC-MS 508.1 $[M + H]^+$.

EXAMPLE 294. (3a*R*, 6a*S*)-Thiophene-3-carboxylic acid [(1*R*)-2-(4-benzoyl-6-oxo-hexahydro-pyrrolo[3,2-*b*]pyrrol-1-yl)-1-methanesulfonylmethyl-2-oxo-ethyl]-amide

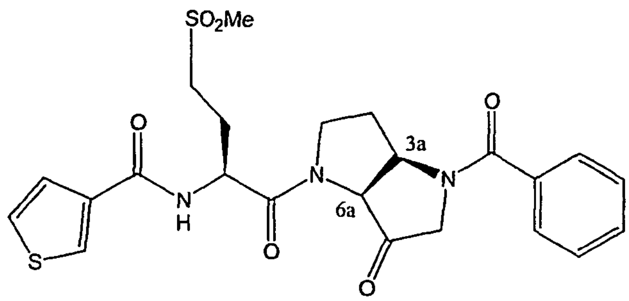

HPLC Rt = 8.61 mins (> 90%), HPLC-MS 504.1 $[M + H]^+$.

EXAMPLE 295. (3a*R*, 6a*S*)-Furan-2-carboxylic acid {(1*S*)-2-[4-((2*S*)-2-acetylamino-4-methyl-pentanoyl)-6-oxo-hexahydro-pyrrolo[3,2-*b*]pyrrol-1-yl]-1-cyclohexylmethyl-2-oxo-ethyl}-amide

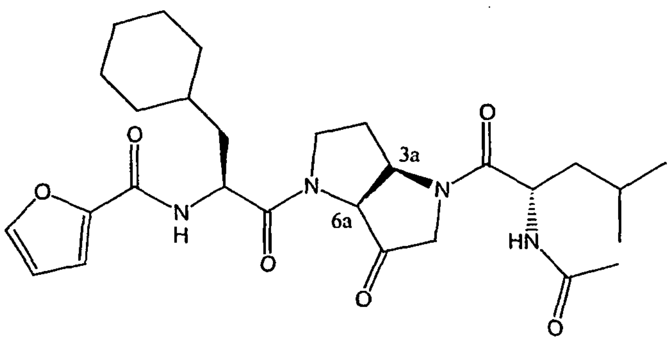

HPLC Rt = 14.35 mins (> 95%), HPLC-MS 529.2 $[M + H]^+$.

In addition, EXAMPLES 7, 8, 9, 10, 12, 13, 14, 16, 17, 19, 20, 37, 39, 59, 61, 65, 85, 86, 87, 88, 89, 90, 91, 92, 93, 98, 103, 123, 145, 151, 154, 158, 159, 161, 164, 170, 171, 172, 173, 174, 185, 187, 193, 194, 245, 247, 249a,

249b, 249c, 298, 303, 310, 314, 315, 316, 317, 323, 329, 330, 334, 335 and 340 have utility as inhibitors of cathepsin S with Ki less than 5000nM.

EXAMPLES 296 to 345 were prepared as detailed for EXAMPLES 1 and 119, substituting the appropriate carboxylic acids as required and are inhibitors of cathepsin L with Ki ranging from 10-5000nM;

EXAMPLE 296. (3a*R*, 6a*S*)-Naphthalene-1-carboxylic acid [(1*S*)-2-(4-benzoyl-6-oxo-hexahydro-pyrrolo[3,2-*b*]pyrrol-1-yl)-1-(4-hydroxybenzyl)-2-oxo-ethyl]-amide

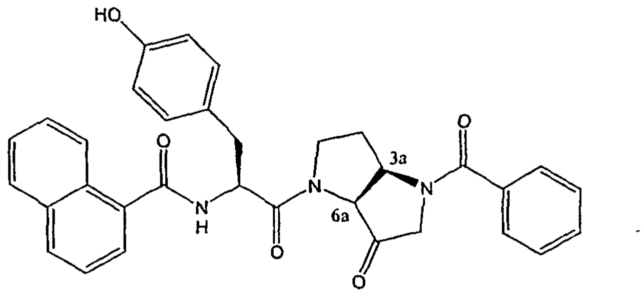

HPLC Rt = 13.5-14.4 mins (> 90%), HPLC-MS 548.2 $[M + H]^+$, 1117.4 $[2M + Na]^+$.

EXAMPLE 297. (3a*R*, 6a*S*)-Quinoline-8-carboxylic acid [(1*S*)-2-(4-benzoyl-6-oxo-hexahydro-pyrrolo[3,2-*b*]pyrrol-1-yl)-1-(4-hydroxy-benzyl)-2-oxo-ethyl]-amide

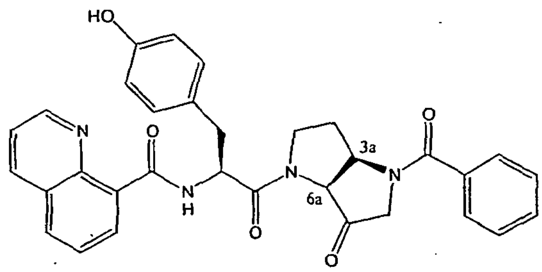

HPLC Rt = 11.79 mins (> 85%), HPLC-MS 549.2 $[M + H]^+$, 567.3 $[M + H + H_2O]^+$.

EXAMPLE 298. (3a*R*, 6a*S*)-*N*[(1*S*)-2-(4-Benzoyl-6-oxo-hexahydro-pyrrolo[3,2-*b*]pyrrol-1-yl)-1-(4-hydroxy-benzyl)-2-oxo-ethyl]-3-bromo-benzamide

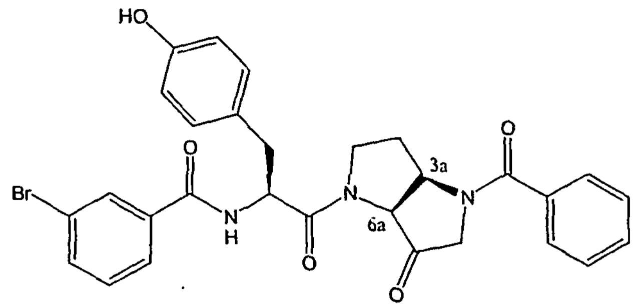

HPLC Rt = 13.21 mins (> 95%), HPLC-MS 576.1 / 578.1 $[M + H]^+$.

EXAMPLE 299. (3a*R*, 6a*S*)-Isoquinoline-1-carboxylic acid [(1*S*)-2-(4-benzoyl-6-oxo-hexahydro-pyrrolo[3,2-*b*]pyrrol-1-yl)-1-(4-hydroxy-benzyl)-2-oxo-ethyl]-amide

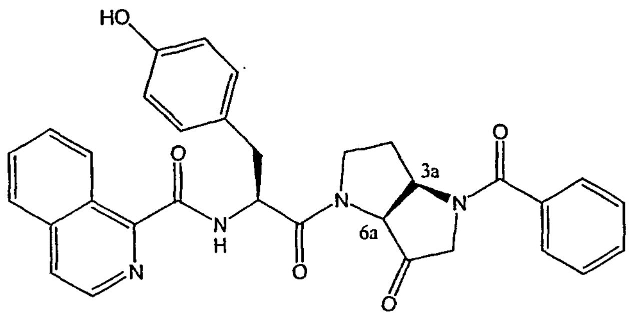

HPLC Rt = 14.1-15.1 mins (> 85%), HPLC-MS 549.2 $[M + H]^+$, 567.2 $[M + H + H_2O]^+$.

EXAMPLE 300. (3a*R*, 6a*S*)-Benzo[*b*]thiophene-2-carboxylic acid [(1*S*)-2-(4-benzoyl-6-oxo-hexahydro-pyrrolo[3,2-*b*]pyrrol-1-yl)-1-(4-hydroxy-benzyl)-2-oxo-ethyl]-amide

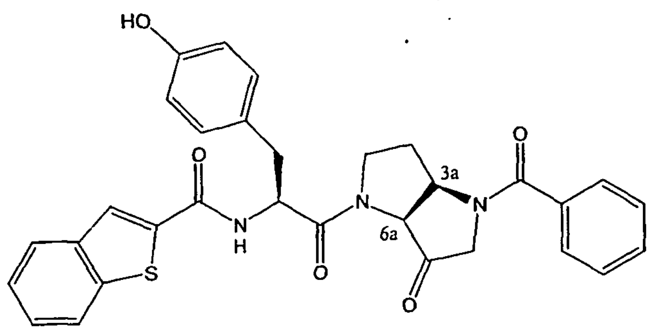

HPLC Rt = 14.8-15.6 mins (> 85%), HPLC-MS 554.2 $[M + H]^+$.

EXAMPLE 301.3a*R*, 6a*S*)-Naphthalene-1-carboxylic acid {(1*S*)-1-(4-hydroxy-bertzyl)-2-[4-(naphthalene-1-carbonyl)-6-oxo-hexahydro-pyrrolo[3,2-*b*]pyrrol-1-yl]-2-oxo-ethyl}-amide

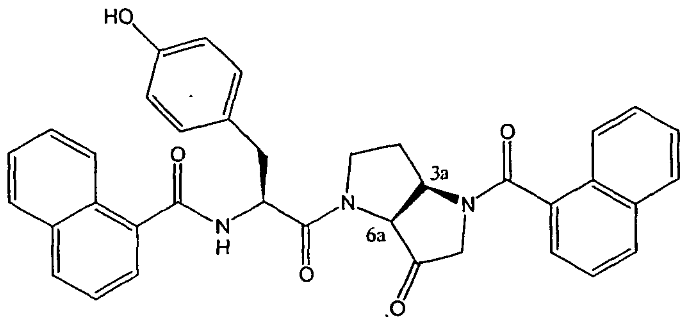

HPLC Rt = 15.5-16.8 mins (> 85%), HPLC-MS 598.2 $[M + H]^+$, 616.2 $[M + H + H_2O]^+$.

EXAMPLE 302. (3a*R*, 6a*S*)-Naphthalene-1-carboxylic acid {(1*S*)-1-(4-hydroxy-benzyl)-2-[4-(naphthalene-2-carbonyl)-6-oxo-hexahydro-pyrrolo[3,2-*b*]pyrrol-1-yl]-2-oxo-ethyl}-amide

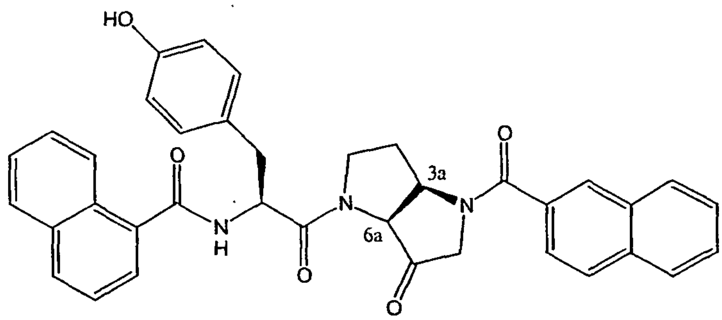

HPLC Rt = 16.0-16.9 mins (> 90%), HPLC-MS 598.2 $[M + H]^+$.

EXAMPLE 303. (3a*R*, 6a*S*)-3-Bromo-*N*-{(1*S*)-1-(4-hydroxy-benzyl)-2-[4-(2-methane sulfonyl-benzoyl)-6-oxo-hexahydro-pyrrolo[3,2-*b*]pyrrol-1-yl]-2-oxoethyl}-benzamide

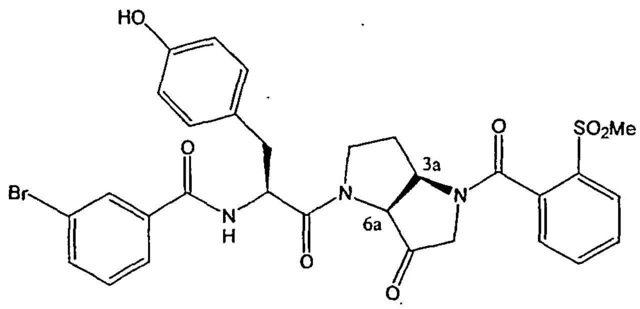

HPLC Rt = 12.66 mins (> 90%), HPLC-MS 654.0 / 656.0 [M + H]$^+$.

EXAMPLE 304. (3a*R*, 6a*S*)-3-Bromo-N-{(1*S*)-1-(4-hydroxy-benzyl)-2-[4-(3-methane sulfonyl-benzoyl)-6-oxo-hexahydro-pyrrolo[3,2-*b*]pyrrol-1-yl]-2-oxoethyl}-benzamide.

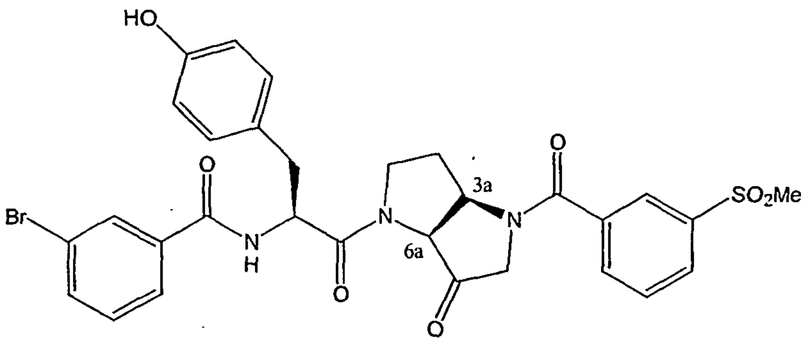

HPLC Rt =12.95 mins (> 75%), HPLC-MS 654.0 / 656.0 [M + H]$^+$.

EXAMPLE 305. (3a*R*, 6a*S*)-3-Bromo-*N*-{(1*S*)-1-(4-hydroxy-benzyl)-2-[4-(3-nitro-benzoyl)-6-oxo-hexahydro-pyrrolo[3,2-*b*]pyrrol-1-yl]-2-oxo-ethyl}-benzamide

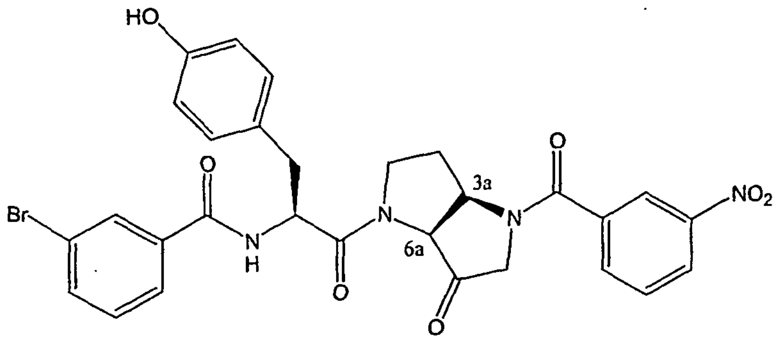

HPLC Rt = 13.52 mins (> 90%), HPLC-MS 621.0 / 623.0 [M + H]$^+$.

EXAMPLE 306. (3a*R*, 6a*S*)-3-Bromo-*N*-{(1*S*)-1-(4-hydroxy-benzyl)-2-[4-(4-nitro-benzoyl)-6-oxo-hexahydro-pyrrolo[3,2-*b*]pyrrol-1-yl]-2-oxo-ethyl}-benzamide

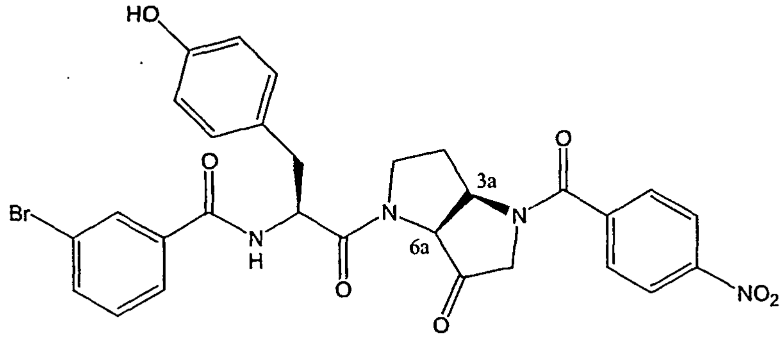

HPLC Rt = 13.50 mins (> 95%), HPLC-MS 621.0 / 623.0 [M + H]$^+$.

EXAMPLE 307. (3a*R*, 6a*S*)-*N*-[(1*S*)-2-[4-(Adamantane-1-carbonyl)-6-oxo-hexa hydro-pyrrolo[3,2-*b*]pyrrol-1-yl]-1-(4-hydroxy-benzyl)-2-oxo-ethyl]-3-bromo-benzamide

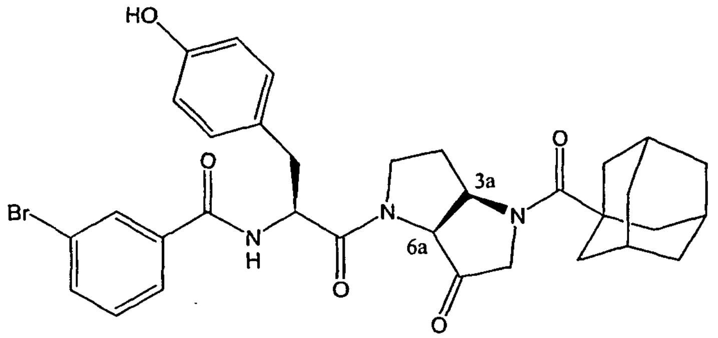

HPLC Rt = 15.9-17.3 mins (> 50%), HPLC-MS 634.0 / 636.0 [M + H]$^+$.

EXAMPLE 308. (3a*R*, 6a*S*)-3-Bromo-*N*-[(1*S*)-2-[4-(hexahydro-2,5-methano-pentalene-3a-carbonyl)-6-oxo-hexahydro-pyrrolo[3,2-*b*]pyrrol-1-yl]-1-(4-hydroxy-benzyl)-2-oxo-ethyl]-benzamide

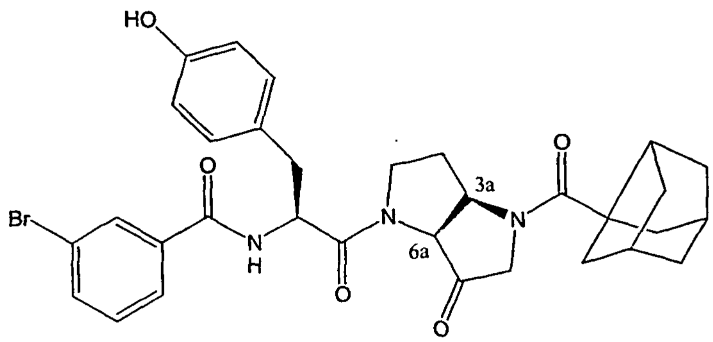

HPLC Rt = 14.7-16.2 mins (> 80%), HPLC-MS 620.0 / 622.0 [M + H]$^+$, 638.1 / 640.1 [M + H + $H_2O$]$^+$.

EXAMPLE 309. (3a*R*, 6a*S*)-3-Bromo-*N*[(1*S*)-2-[4-(4-hydroxy-benzoyl)-6-oxo-hexa hydro-pyrrolo[3,2-*b*]pyrrol-1-yl]-1-(4-hydroxy-benzyl)-2-oxo-ethyl]-benzamide

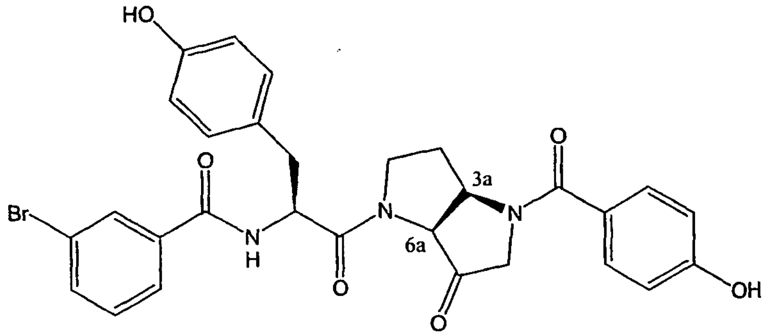

HPLC Rt = 11.92 mins (> 80%), HPLC-MS 592.0 / 594.0 $[M + H]^+$.

EXAMPLE 310. (3a*R*, 6a*S*)-*N*-[(1*S*)-2-[4-(4-Amino-benzoyl)-6-oxo-hexahydro-pyrrolo[3,2-*b*]pyrrol-1-yl]-1-(4-hydroxy-benzyl)-2-oxo-ethyl]-3-bromo-benzamide

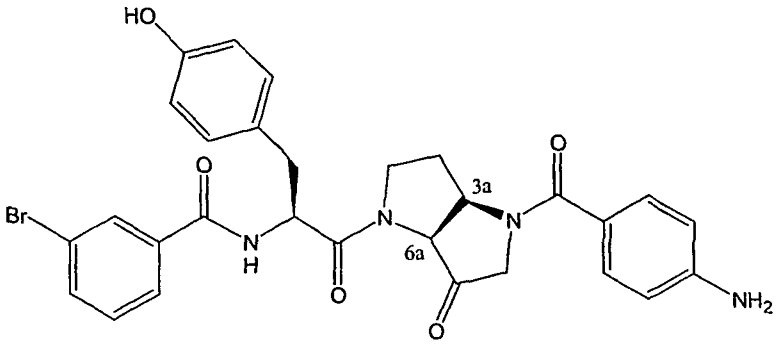

HPLC Rt = 9.9-10.6 mins (> 75%), HPLC-MS 591.0/593.0 $[M + H]^+$, 609.0 / 611.0 $[M+H+H_2O]^+$.

EXAMPLE 311. (3a*R*, 6a*S*)-3-Bromo-*N*-{(1*S*)-l-(4-hydroxy-benzyl)-2-oxo-2-[6-oxo-4-(quinoline-6-carbonyl)-hexahydro-pyrrolo[3,2-*b*]pyrrol-1-yl]-ethyl}-benzamide

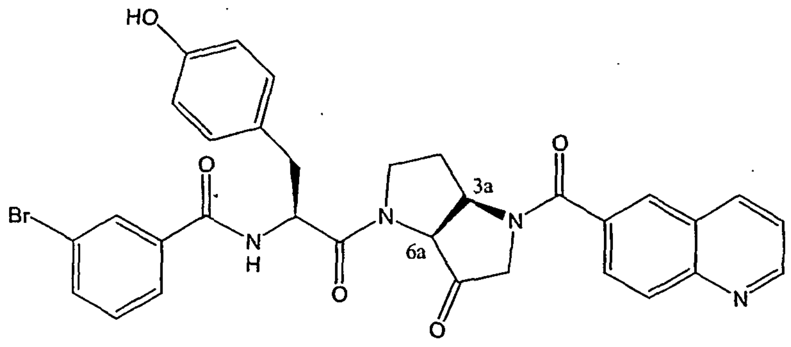

HPLC Rt = 11.13 mins (> 75%), HPLC-MS 627.0 / 629.0 [M + H]$^+$, 645.0 / 647.0 [M + H + $H_2O$]$^+$.

EXAMPLE 312. (3a*R*, 6a*S*)-4,5-Dimethyl-furan-2-carboxylic acid {(1*S*)-1-(4-hydroxy-benzyl)-2-[4-(2-methanesulfonyl-benzoyl)-6-oxo-hexahydro-pyrrolo[3,2-*b*]pyrrol-1-yl]-2-oxo-ethyl}-amide

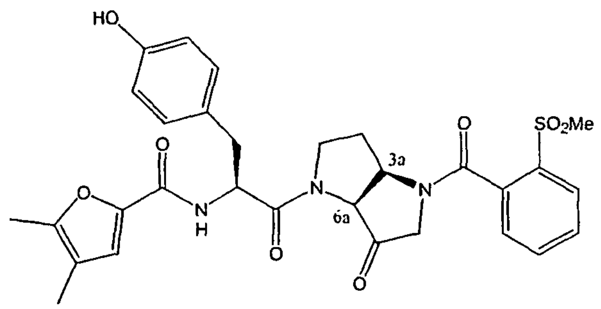

HPLC Rt =11.43 mins (> 90%), HPLC-MS 594.1 [M + H]$^+$.

EXAMPLE 313. (3a*R*, 6a*S*)-2-Ethyl-5-methyl-2*H*-pyrazole-3-carboxylic acid {(1*S*)-1-(4-hydroxy-benzyl)-2-[4-(2-methanesulfonylbenzoyl)-6-oxo-hexahydropyrrolo[3,2-*b*]pyrrol-1-yl]-2-oxo-ethyl}-amide

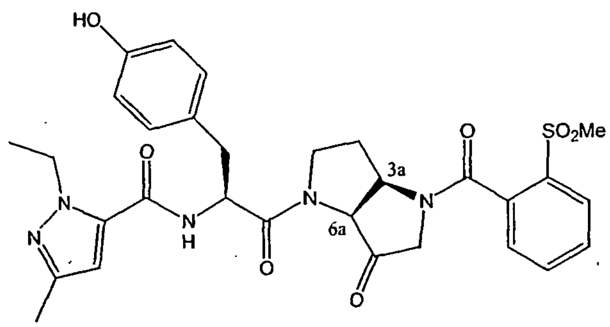

HPLC Rt = 10.03 mins (> 90%), HPLC-MS 608.1 [M + H]$^+$, 626.1 [M + H + $H_2O$]$^+$.

EXAMPLE 314. (3a*R*, 6a*S*)-*N*-[(1*S*)-2-[4-(Benzo[*b*]thiophene-3-carbonyl)-6-oxo-hexahydro-pyrrolo[3,2-*b*]pyrrol-1-yl]-1-(3-methyl-benzyl)-2-oxo-ethyl]-2,2-diphenyl-acetamide

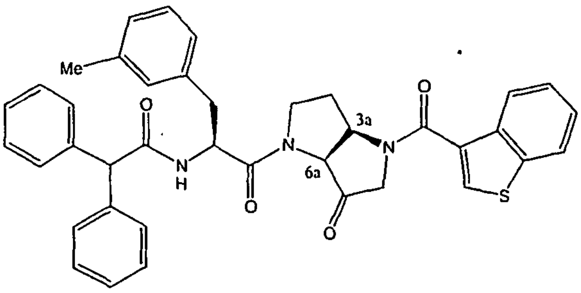

HPLC Rt = 20.2-21.4 mins (> 95%), HPLC-MS 642.2 [M + H]$^+$.

EXAMPLE 315. (3a*R*, 6a*S*)-*N*-[(1*S*)-2-[4-(Benzo[*b*]thiophene-3-carbonyl)-6-oxo-hexahydro-pyrrolo[3,2-*b*]pyrrol-1-yl]-1-(3-fluoro-benzyl)-2-oxo-etllyl]-2,2-diphenyl-acetamide

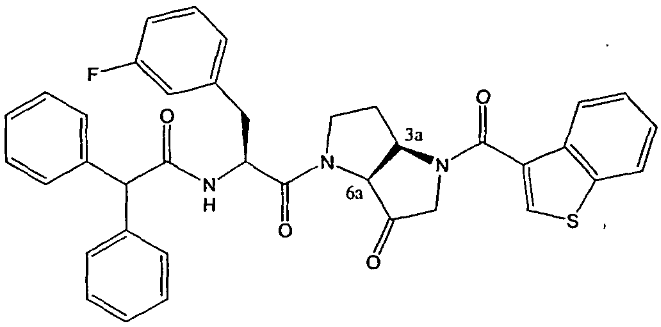

HPLC Rt = 19.7-21.0 mins (> 90%), HPLC-MS 646.2 [M + H]$^+$.

EXAMPLE 316. (3a*R*, 6a*S*)-*N*-[(1*S*)-2-[4-(Benzo[*b*]thiophene-3-carbonyl)-6-oxo-hexahydro-pyrrolo[3,2-*b*]pyrrol-1-yl]-1-(3,5-difluoro-benzyl)-2-oxo-ethyl]-2,2-diphenyl-acetamide

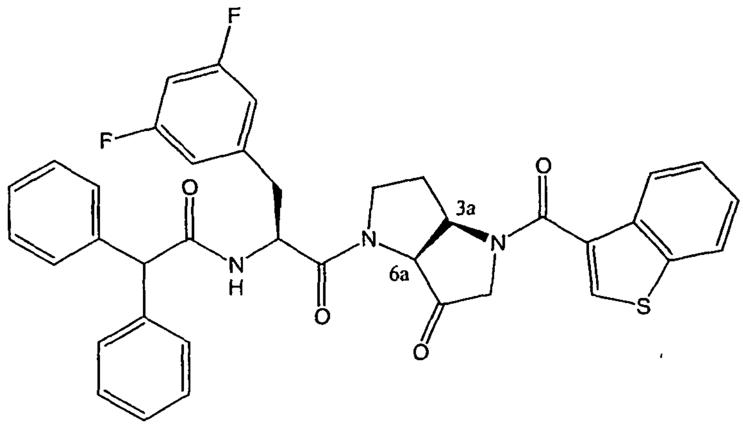

HPLC Rt = 20.1-21.3 mins (>90%), HPLC-MS 664.2 $[M+ H]^+$.

EXAMPLE 317. (3a*R*, 6a*S*)-Benzothiazole-6-carboxylic acid [(1*S*)-2-(4-benzoyl-6-oxo-hexahydro-pyrrolo[3,2-*b*]pyrrol-1-yl)-1-(4-hydroxy-benzyl)-2-oxo-ethyl]-amide

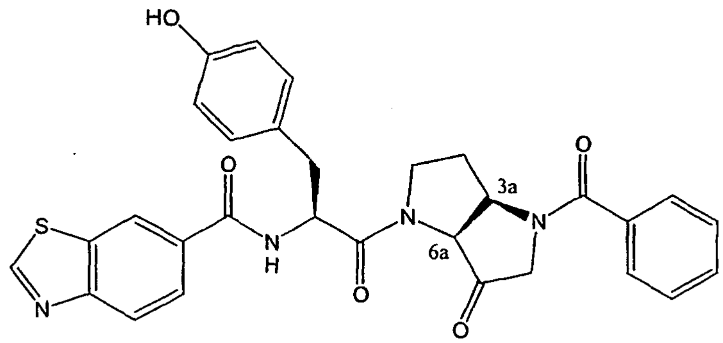

HPLC Rt = 12.02 mins (> 95%), HPLC-MS 555.1 $[M + H]^+$.

EXAMPLE 318. (3a*R*, 6a*S*)-4-Aminomethyl-N-[(1*S*)-2-(4-benzoyl-6-oxo-hexahydro-pyrrolo[3,2-*b*]pyrrol-1-yl)-1-(4-hydroxy-benzyl)-2-oxo-ethyl]-benzamide

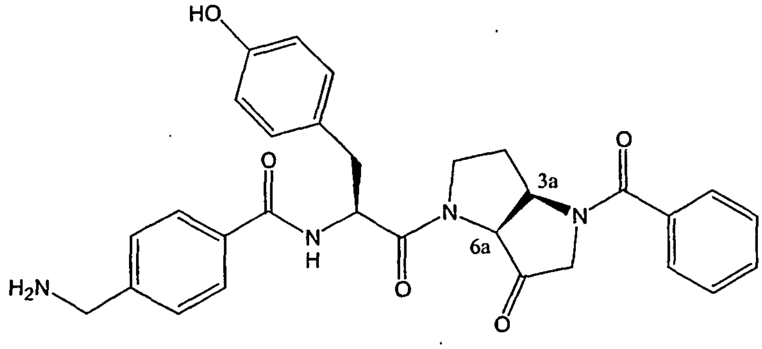

HPLC Rt = 8.92 mins (> 95%), HPLC-MS 527.2 $[M + H]^+$, 545.2 $[M + H + H_2O]^+$.

EXAMPLE 319. (3a*R*, 6a*S*)-3-Aminomethyl-*N*-[(1*S*)-2-(4-benzoyl-6-oxo-hexahydro-pyrrolo[3,2-*b*]pyrrol-1-yl)-1-(4-hydroxy-benzyl)-2-oxo-ethyl]-benzamide

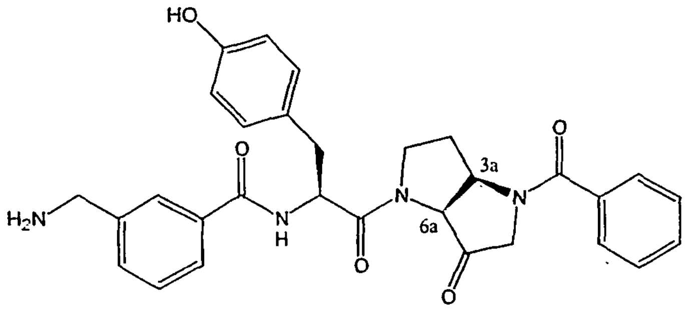

HPLC Rt = 9.14 mins (> %), HPLC-MS 527.2 $[M + H]^+$, 545.3 $[M + H + H_2O]^+$.

EXAMPLE 320. (3a*R*, 6a*S*)-*N*-[(1*S*)-2-(4-Benzenesulfonyl-6-oxo-hexahydro-pyrrolo[3,2-*b*]pyrrol-1-yl)-1-(4-hydroxy-benzyl)-2-oxo-ethyl]-3-bromo-benzamide

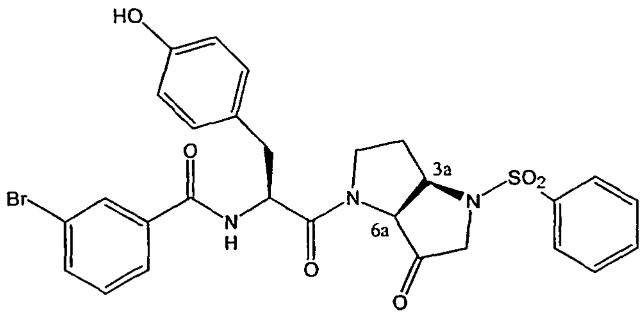

HPLC Rt = 16.1-17.9 mins (> 90%), HPLC-MS 612.0 / 614.0 $[M + H]^+$.

EXAMPLE 321. (3a*R*, 6a*S*)-4-Dimethylamino-*N*-{(1*S*)-1-(4-hydroxy-benzyl)-2-[4-(3-nitro-benzoyl)-6-oxo-hexa- hydro-pyrrolo[3,2-*b*]pyrrol-1-yl]-2-oxo-ethyl}-benzamide

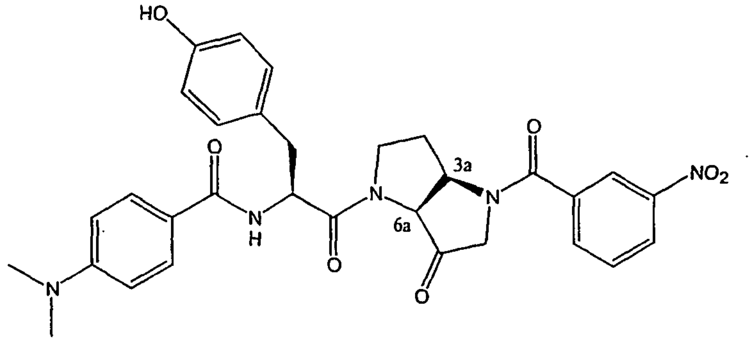

HPLC Rt = 10.75 mins (> 80%), HPLC-MS 586.2 [M + H]$^+$, 604.2 [M + H + $H_2O$]$^+$.

EXAMPLE 322. (3a*R*, 6a*S*)-4-Dimethylamino-*N*-[(1*S*)-2-[4-(3-fluoro-benzoyl)-6-oxo-hexahydro-pyrrolo[3,2-*b*]-pyrrol-1-yl]-1-(4-hydroxy-benzyl)-2-oxo-ethyl]-benzamide

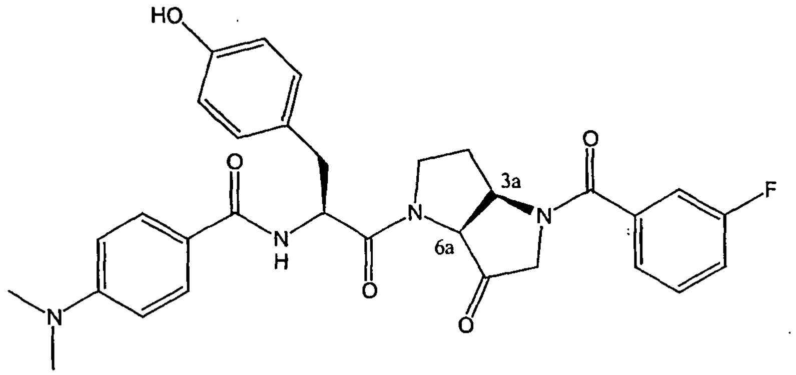

HPLC Rt = 10.59 mins (> 90%), HPLC-MS 559.2 [M + H]$^+$, 577.2 [M + H + $H_2O$]$^+$.

EXAMPLE 323. (3a*R*, 6a*S*)-*N*-[(1*S*)-2-[4-(Benzo[*b*]thiophene-3-carbonyl)-6-oxo-hexahydro-pyrrolo[3,2-*b*]pyrrol-1-yl]-1-(4-hydroxy-benzyl)-2-oxo-ethyl]-3-bromo-benzamide

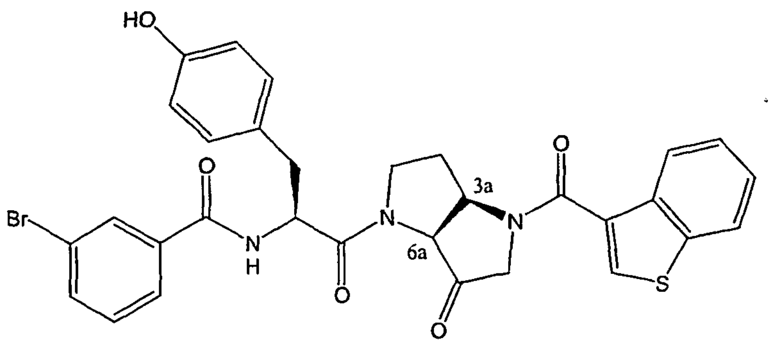

HPLC Rt = 14.61 mins (> 90%), HPLC-MS 632.0 / 634.0 [M + H]+.

EXAMPLE 324. (3a*R*, 6a*S*)-3-Bromo-*N*-{(1*S*)-1-(4-hydroxy-benzyt)-2-oxo-2-[6-oxo-4-(thiophene-2-carbonyl)-hexahydro-pyrrolo[3,2-*b*]pyrrol-1-yl]-ethyl}-benzamide

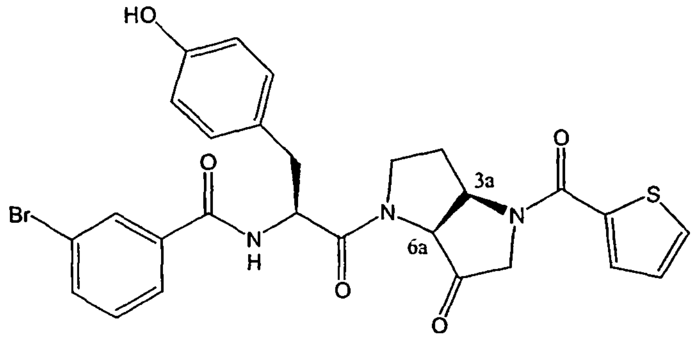

HPLC Rt = 12.93 mins (> 90%), HPLC-MS 582.0 / 584.0 [M + H]+.

EXAMPLE 325. (3a*R*, 6a*S*)-3-Bromo-*N*-[(1*S*)-2-(4-cyclohexanecarbonyl-6-oxo-hexahydro-pyrrolo[3,2-*b*]pyrrol-1-yl)-1-(4-hydroxy-benzyl)-2-oxo-ethyl]-benzamide

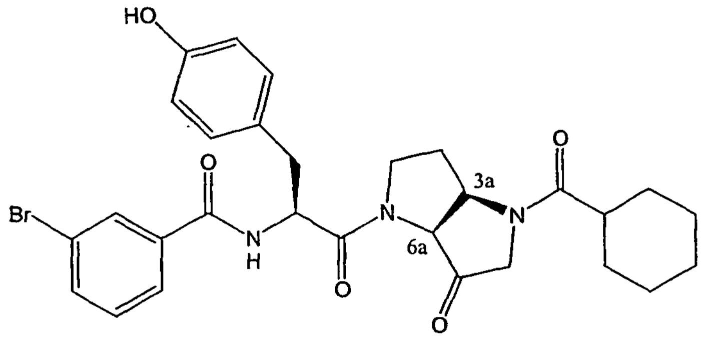

HPLC Rt = 15.0-17.0 mins (> 90%), HPLC-MS 582.1 / 584.1.[M + H]+.

EXAMPLE 326. (3a*R*, 6a*S*)-3-Bromo-*N*-{(1*S*)-1-(4-hydroxy-benzyl)-2-[4-(2-methyl-benzoyl)-6-oxo-hexahydro-pyrrolo-[3,2-*b*]pyrrol-1-yl]-2-oxo-ethyl}-benzamide

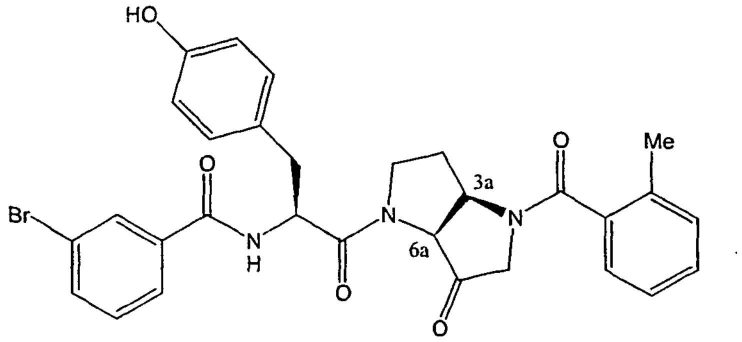

HPLC Rt = 14.4-16.1 mins (> 90%), HPLC-MS 590.0/592.0 [M + H]+.

EXAMPLE 327. (3a*R*, 6a*S*)-3-Bromo-*N*-{(1*S*)-1-(4-hydroxy-benzyl)-2-[4-(3-methyl-benzoyl)-6-oxo-hexahydro-pyrrolo[3,2-*b*]pyrrol-1-yl]-2-oxo-ethyl}-benzamide

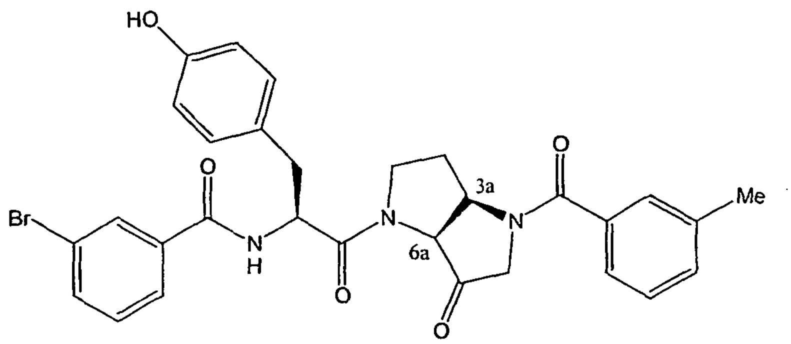

HPLC Rt = 14.4-15.8 mins (> 90%), HPLC-MS 590.0/592.0 [M + H]+.

EXAMPLE 328. (3a*R*, 6a*S*)-3-Bromo-*N*-{(1*S*)-(4-hydroxy-benzyt)-2-[4-(4-methyl-benzoyl)-6-oxo-hexahydro-pyrrolo[3,2-*b*]pyrrol-1-yl]-2-oxo-ethyl}-benzamide

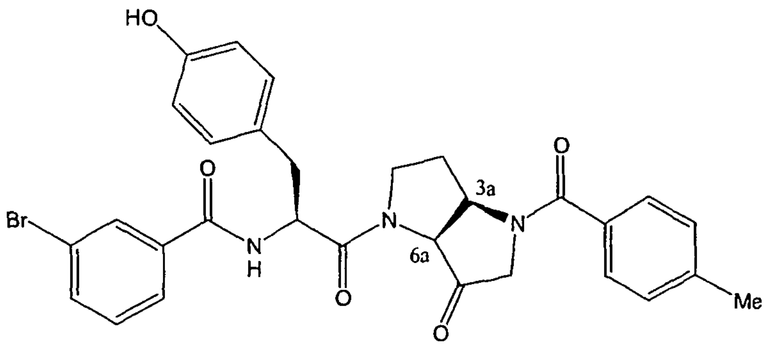

HPLC Rt =13.8-14.9 mins (> 90%), HPLC-MS 590.0/592.0 [M + H]+.

EXAMPLE 329. (3a*R*, 6a*S*)-*N*-[(1*S*)-2-[4-(Benzo[*b*]thiophene-3-carbonyl)-6-oxo-hexahydro-pyrrolo[3,2-*b*]pyrrol-1-yl]-1-(4-hydroxy-benzyl)-2-oxo-ethyl]-2,2-diphenyl-acetamide

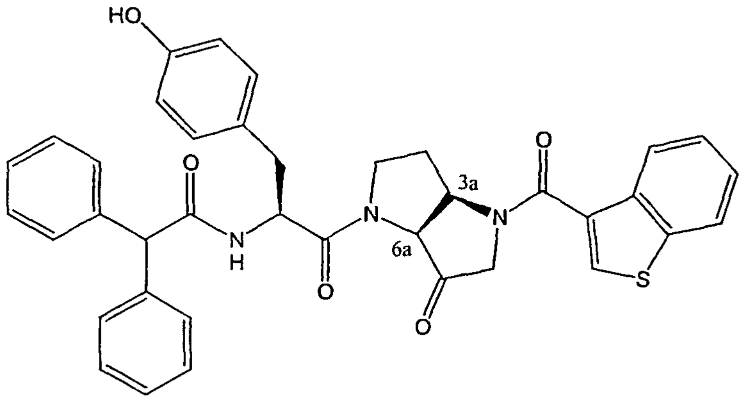

HPLC Rt = 17.0-18.5 mins (> 80%), HPLC-MS 644.1 $[M + H]^+$.

EXAMPLE 330. (3a*R*, 6a*S*)-*N*-[(1*S*)-2-(4-Benzoyl-6-oxo-hexahydro-pyrrolo[3,2-*b*]pyrrol-1-yl)-1-(4-hydroxy-benzyl)-2-oxo-ethyl]-2,2-diphenyl-acetamide

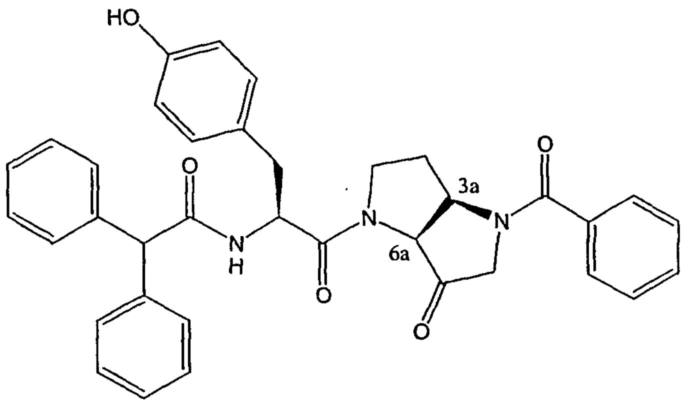

HPLC Rt = 16.4-17.5 mins (> 85%), HPLC-MS 588.2 $[M + H]^+$.

EXAMPLE 331. (3a*R*, 6a*S*)-3-Aminomethyl-*N*-[(1*S*)-2-[4-(benzo[*b*]thiophene-3-carbonyl)-6-oxo-hexahydro-pyrrolo-[3,2-*b*]pyrrol-1-yl]-1-(4-hydroxy-benzyl)-2-oxo-ethyl]-benzamide

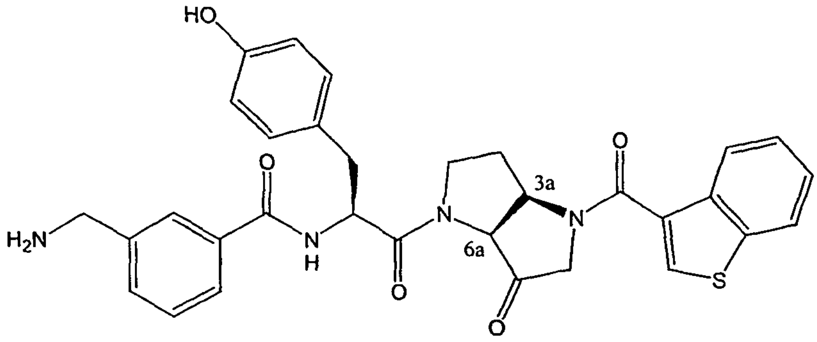

HPLC Rt = 10.82 mins (> 90%), HPLC-MS 583.1 [M + H]$^+$.

EXAMPLE 332. (3a*R*, 6a*S*)-3-Aminomethyl-*N*-{(1*S*)-1-(4-hydroxy-benzyl)-2-[4-(2-methanesulfonyl-benzoyl)-6-oxo-hexahydro-pyrrolo[3,2-*b*]pyrrol-1-yl]-2-oxo-ethyl}-benzamide

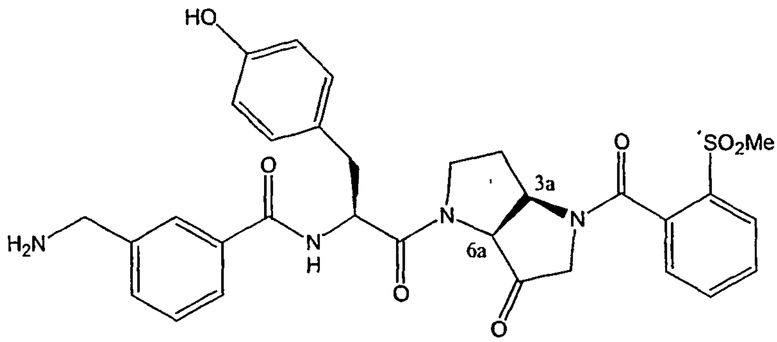

HPLC Rt = 8.16 mins (> 85%), HPLC-MS 605.1 [M + H]$^+$, 623.2 [M + H + $H_2O$]$^+$.

EXAMPLE 333. (3a*R*, 6a*S*)-*N*-[(1*S*)-2-(4-Benzoyl-6-oxo-hexahydro-pyrrolo[3,2-*b*]pyrrol-1-yl)-1-(4-hydroxy-benzyl)-2-oxo-ethyl)-benzamide

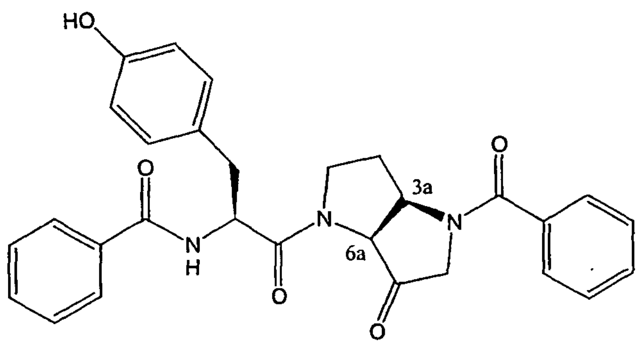

HPLC Rt = 11.79 mins (> 95%), HPLC-MS 498.1 [M + H]$^+$.

EXAMPLE 334. (3a*R*, 6a*S*)-*N*-[(1*S*)-2-(4-Benzoyl-6-oxo-hexahydro-pyrrolo[3,2-*b*]pyrrol-1-yl)-1-(4-hydroxy-benzyl)-2-oxo-ethyl]-3-chloro-benzamide

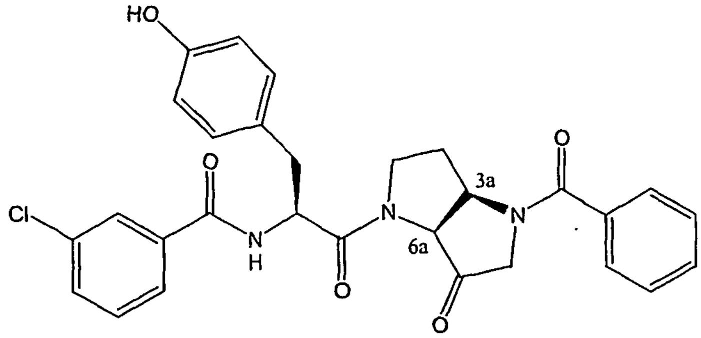

HPLC Rt = 13.64 mins (> 95 %), HPLC-MS 532.1 / 534.1 [M + H]$^+$.

EXAMPLE 335. (3a*R*, 6a*S*)-*N*-[(1*S*)-2-(4-Benzoyl-6-oxo-hexahydro-pyrrolo[3,2-*b*]pyrrol-1-yl)-1-(4-hydroxy-benzyl)-2-oxo-ethyl]-3-fluoro-benzamide

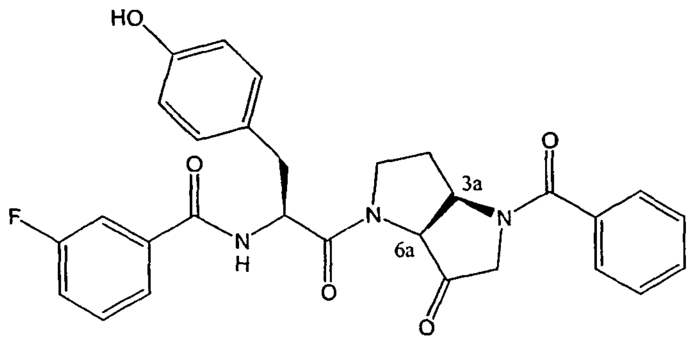

HPLC Rt = 12.10 mins (> 90%), HPLC-MS 516.1 [M + H]$^+$.

EXAMPLE 336. (3a*R*, 6a*S*)-*N*-[(1*S*)-2-(4-Benzoyl-6-oxo-hexahydro-pyrrolo[3,2-*b*]pyrrol-1-yl)-1-(4-hydroxy-benzyl)-2-oxo-ethyl]-nicotinamide

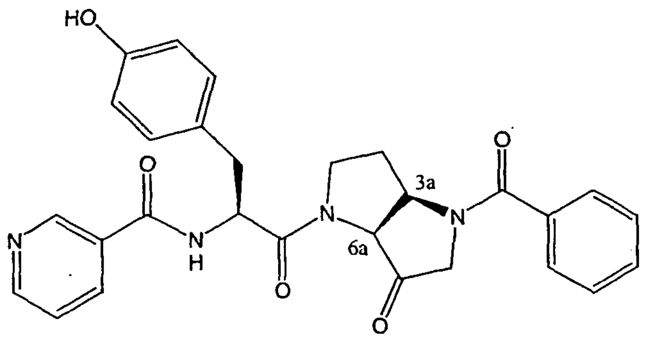

HPLC Rt = 8.16 mins (> 95%), HPLC-MS 499.1 [M + H]$^+$, 997.2 [2M + H]$^+$.

EXAMPLE 337. (3a*R*, 6a*S*)-*N*-[(1*S*)-2-(4-Benzoyl-6-oxo-hexahydro-pyrrolo[3,2-*b*]pyrrol-1-yl)-1-(4-hydroxy-benzyl)-2-oxo-ethyl]-3-bromo-4-methyl-benzamide

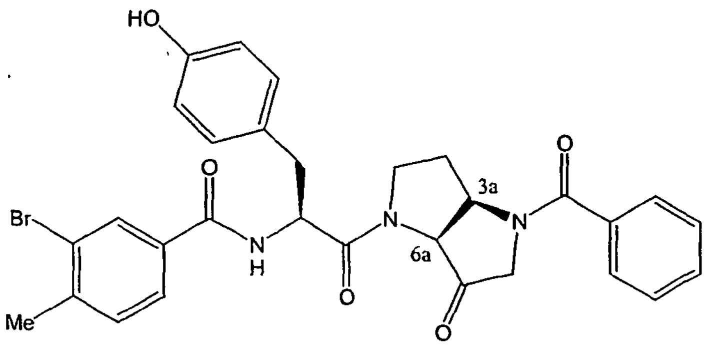

HPLC Rt = 14.0-15.1 mins (> 85%), HPLC-MS 590.0/592.0 [M + H]$^+$.

EXAMPLE 338. (3a*R*, 6a*S*)-*N*-[(1*S*)-2-(4-Benzoyl-6-oxo-hexahydro-pyrrolo[3,2-*b*]pyrrol-1-yl)-1-(4-hydroxy-benzyl)-2-oxo-ethyl]-3-methoxy-benzamide

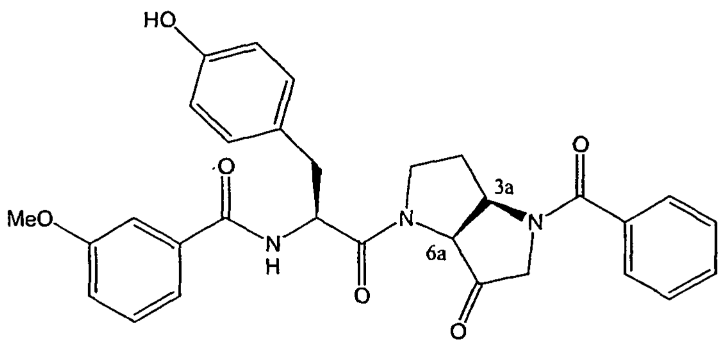

HPLC Rt = 11.81 mins (> 95%), HPLC-MS 528.1 [M + H]$^+$.

EXAMPLE 339. (3a*R*, 6a*S*)-*N*-[(1*S*)-2-(4-Benzoyl-6-oxo-hexahydro-pyrrolo[3,2-*b*]pyrrol-1-yl)-1-(4-hydroxy-benzyl)-2-oxo-ethyl]-3-trifluoromethoxy-benzamide

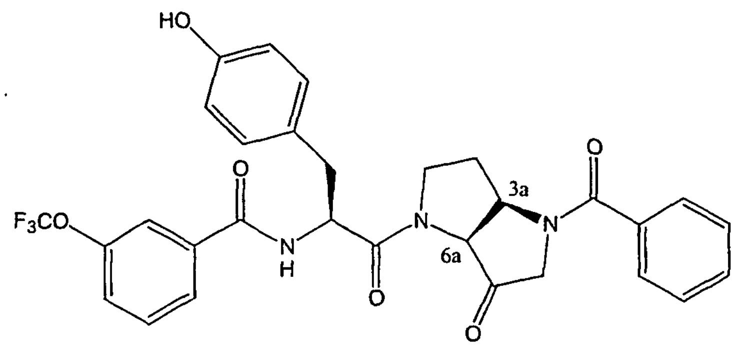

HPLC Rt = 14.21 mins (> 90%), HPLC-MS 582.1 $[M + H]^+$, 600.2 $[M + H + H_2O]^+$,

EXAMPLE 340. (3a*R*, 6a*S*)-*N*-[(1*S*)-1-(4-Benzoyl-6-oxo-hexahydro-pyrrolo[3,2-*b*]pyrrole-1-carbonyl)-3-methyl-butyl]-3-bromo-benzamide

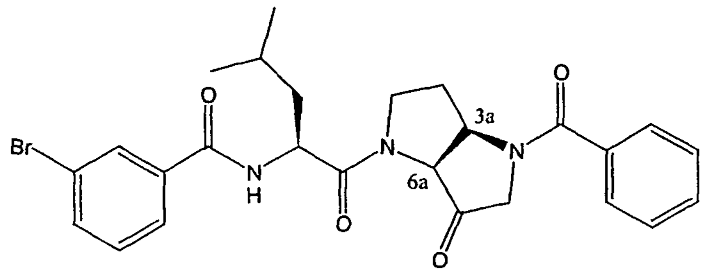

HPLC Rt = 15.0-16.1 mins (> 85%), HPLC-MS 526.1 / 528.1 $[M + H]^+$.

EXAMPLE 341. (3a*R*, 6a*S*)-*N*-[(1*S*)-2-(4-Benzoyl-6-oxo-hexahydro-pyrrolo[3,2-*b*]pyrrol-1-yl)-1-(3-methyl-benzyl)-2-oxo-ethyl]-3-bromo-benzamide

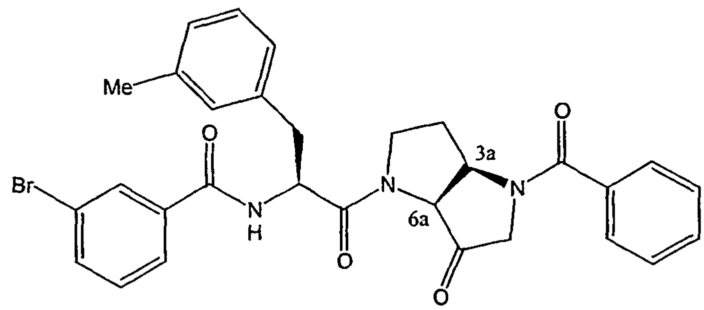

HPLC Rt = 17.0-18.3 mins (> 85%), HPLC-MS 574.0 / 576.0[M + H]$^+$.

EXAMPLE 342. (3a*R*, 6a*S*)-*N*-[(1*S*)-2-(4-Benzoyl-6-oxo-hexahydro-pyrrolo[3,2-*b*]pyrrol-1-yl)-1-(3-fluoro-benzyl)-2-oxo-ethyl]-3-bromo-benzamide

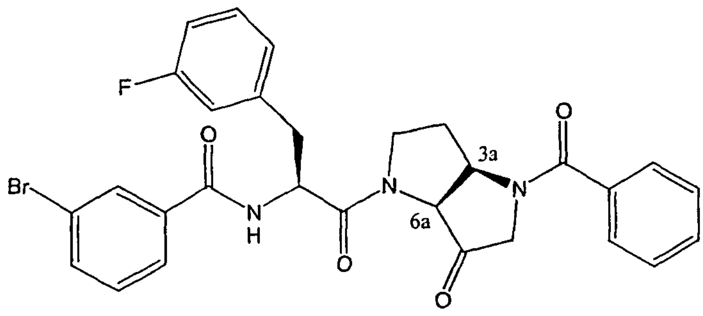

HPLC Rt = 15.8-17.1 mins (> 90%), HPLC-MS 578.0/580.0 [M + H]$^+$.

EXAMPLE 343. (3a*R*, 6a*S*)-*N*-[(1*S*)-2-(4-Benzoyl-6-oxo-hexahydro-pyrrolo[3,2-*b*]pyrrol-1-yl)-1-(3,5-difluoro-benzyl)-2-oxo-ethyl]-3-bromo-benzamide

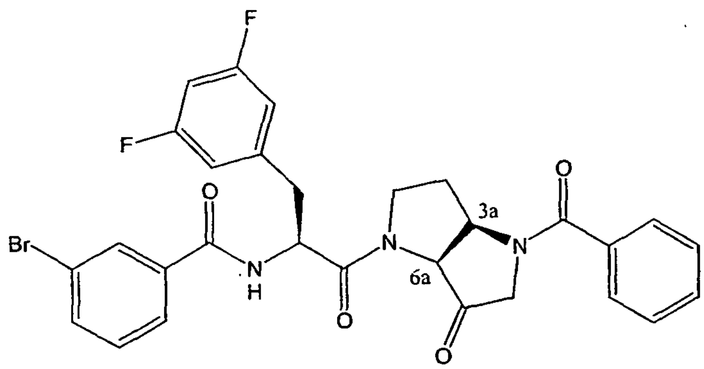

HPLC Rt = 15.9-17.3 mins (> 90%), HPLC-MS 596.0 / 598.0 [M + H]$^+$.

EXAMPLE 344. (3a*R*, 6a*S*)-*N*-[(1*S*)-2-(4-Benzoyl-6-oxo-hexahydro-pyrrolo[3,2-*b*]pyrrol-1-yl)-1-(4-fluoro-benzyl)-2-oxo-ethyl]-3-bromo-benzamide

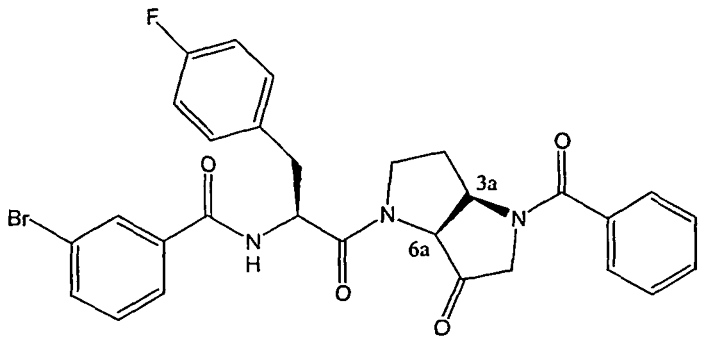

HPLC Rt = 17.1-18.2 mins (> 90%), HPLC-MS 578.0 / 580.0 [M + H]+.

EXAMPLE 345. (3a*R*, 6a*S*)-*N*-[(1*S*)-2-[4-((2*S*)-2-Acetylamino-4-methylpentanoyl)-6-oxo-hexahydro-pyrrolo[3,2-*b*]pyrrol-1-yl]-1-(4-hydroxy-benzyl)-2-oxo-ethyl]-3-bromo-benzamide

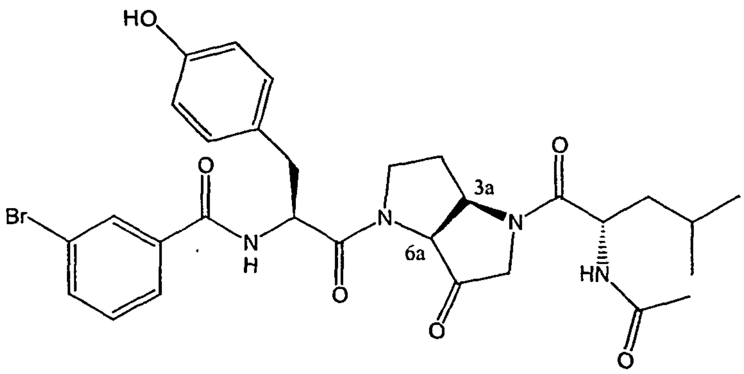

HPLC Rt = 12.73 mins (> 90%), HPLC-MS 627.0/629.0 [M + H]+.

In addition, EXAMPLES 7, 8, 9, 10, 12, 14, 15, 16, 17, 18, 28, 31, 37, 59, 63, 65, 68, 73, 85, 86, 87, 88, 89, 90, 91, 92, 93, 98, 103, 104, 111, 113, 117, 118, 145, 151, 154, 158, 159, 161, 164, 170, 171, 172, 173, 174, 175, 178, 179, 180, 181, 182, 185, 193, 194, 204, 216, 244, 245, 246, 247, 249a, 249b, 249c, 250, 251, 254, 255, 256, 258, 259, 261, 262, 268, 269, 270, 271, 272, 273, 275, 278, 280, 281, 282, 285, 286, 287, 288, 289, 290, 291, 292, 293, 295, 346, 356, 357, 358 and 359 have utility as inhibitors of cathepsin L with Ki less than 5000nM.

EXAMPLES 346 to 359 were prepared as detailed for EXAMPLES 1 and 119, substituting the appropriate carboxylic acids as required and are inhibitors of cruzain and cruzipains with Ki ranging from 10-5000nM;

EXAMPLE 346. (3a*R*, 6*aS*)-*N*-[(1*S*)-2-(4-Benzoyl-6-oxo-hexahydro-pyrrolo[3,2-*b*]pyrrol-1-yl)-1-(4-hydroxybenzyl)-2-oxo-ethyl]-4-tert-butyl-benzamide

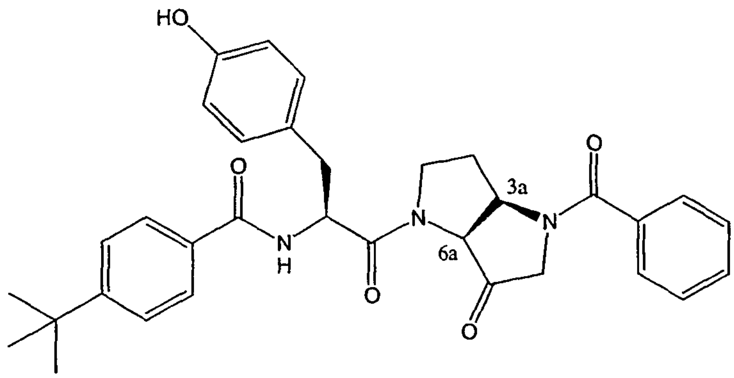

HPLC Rt = 16.0-17.1 mins (> 90%), HPLC-MS 554.3 [M + H]$^+$, 1129.5 [2M + Na]$^+$.

EXAMPLE 347. (3a*R*, 6a*S*)-4-*tert*-Butyl-*N*-{(1*S*)-1-(4-fluoro-benzyl)-2-oxo-2-[6-oxo-4-(pyridine-3-carbonyl)-hexahydro-pyrrolo[3,2-*b*]pyrrol-1-yl]-ethyl}-benzamide

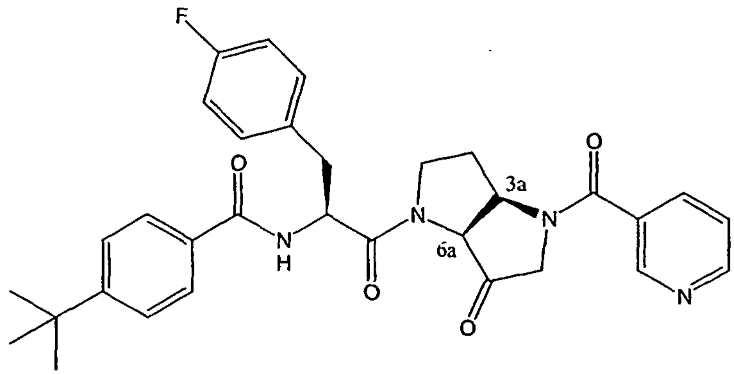

HPLC Rt = 16.82 mins (> 90%), HPLC-MS 557.2 [M + H]$^+$.

EXAMPLE 348. (3a*R*, 6a*S*)-4-*tert*-t-Butyl-*N*-{(1*S*)-1-(4-fluoro-benzyl)-2-oxo-2-[6-oxo-4-(pyridine-4-carbonyl)-hexahydro-pyrrolo[3,2-*b*]pyrrol-1-yl]-ethyl}-benzamide

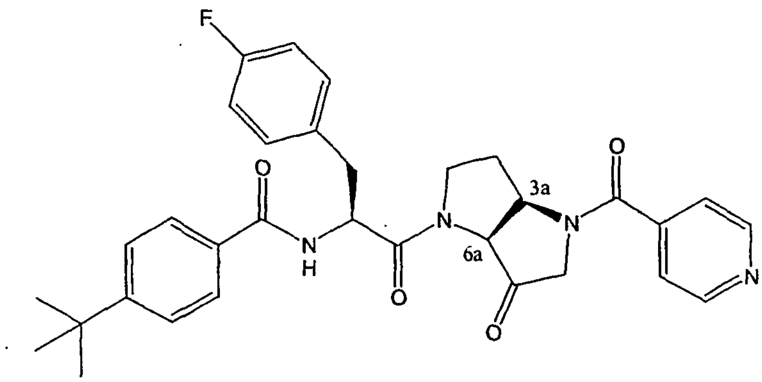

HPLC Rt = 16.44 mins (> 85%), HPLC-MS 557.2 $[M + H]^+$, 575.3 $[M + H + H_2O]^+$.

EXAMPLE 349. (3a*R*, 6a*S*)-4-*tert*-Butyl-*N*-{(1*S*)-1-(4-fluoro-benzyl)-2-[4-(naphthalene-1-carbonyl)-6-oxo-hexahydro-pyrrolo[3,2-*b*]pyrrol-1-yl]-2-oxoethyl}-benzamide

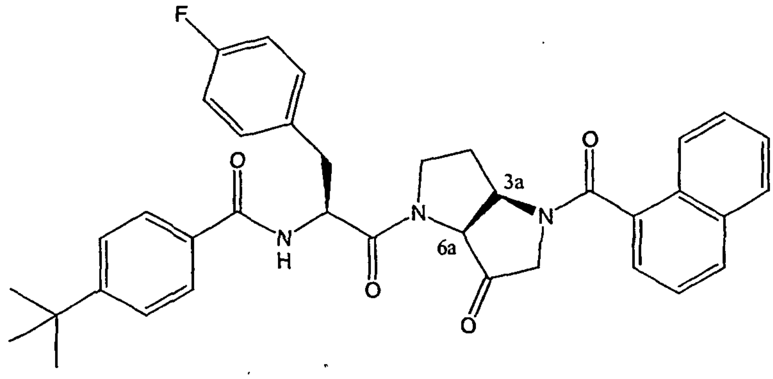

HPLC Rt = 19.6-20.8 mins (> 80%), HPLC-MS 606.1 $[M + H]^+$.

EXAMPLE 350. (3a*R*, 6a*S*)-4-*tert*-Butyl-*N*-{(1*S*)-1-(4-fluoro-benzyl)-2-[4-(naphthalene-2-carbonyl)-6-oxo-hexahydro-pyrrolo[3,2-*b*]pyrrol-1-yl]-2-oxoethyl}-benzamide

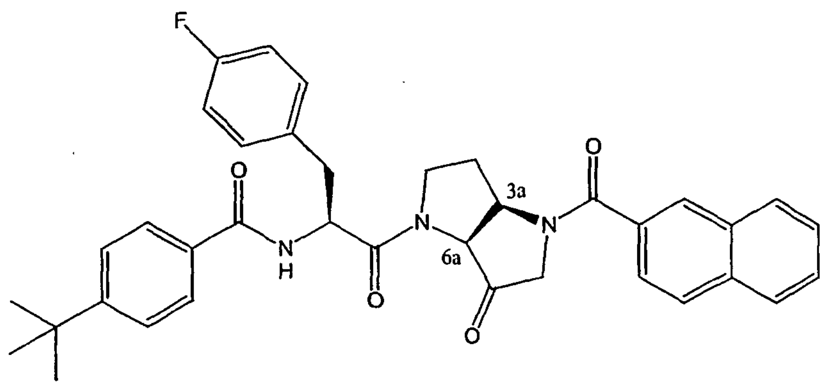

HPLC Rt = 21.0-22.0 mins (> 85%), HPLC-MS 606.1 [M + H]$^+$.

EXAMPLE 351. (3a*R*, 6a*S*)-4-*tert*-Butyl-*N*{(1*S*)-1-(4-hydroxy-benzyl)-2-oxo-2-[6-oxo-4-(pyridine-3-carbonyl)-hexahydro-pyrrolo[3,2-*b*]pyrrol-1-yl]-ethyl}-benzamide

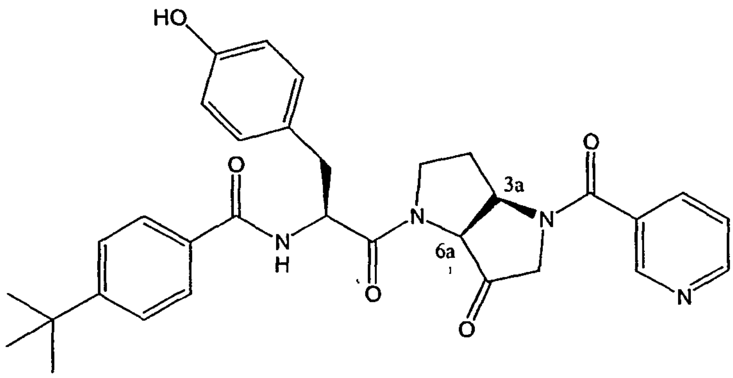

HPLC Rt = 14.27 mins (> 85%), HPLC-MS 555.3 [M + H]$^+$, 573.3 [M + H + $H_2O$]$^+$.

EXAMPLE 352. (3a*R*, 6a*S*)-4-*tert*-Butyl-*N*-{(1*S*)-1-(4-hydroxy-benzyl)-2-oxo-2-[6-oxo-4-(pyridine-4-carbonyl)-hexahydro-pyrrolo[3,2-*b*]pyrrol-1-yl]-ethyl}-benzamide

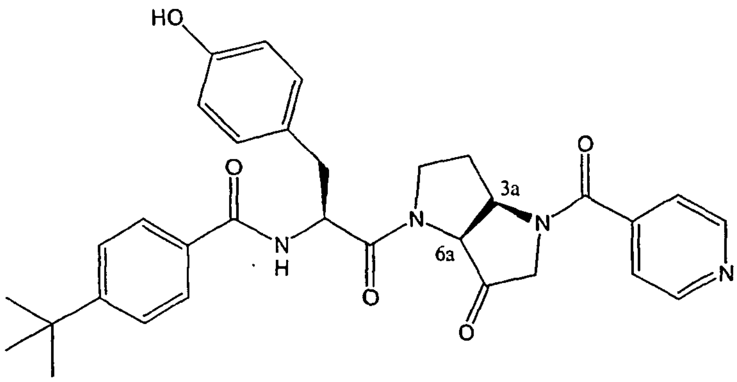

HPLC Rt = 14.35 mins (> 90%), HPLC-MS 555.2 [M + H]$^+$, 573.3 [M + H + $H_2O$]$^+$.

EXAMPLE 353. (3a*R*, 6a*S*)-4-*tert*-Butyl-*N*{(1*S*)-1-(4-hydroxy-benzyl)-2-[4-(naphthalene-1-carbonyl)-6-oxo-hexahydro-pyrrolo[3,2-*b*]pyrrol-1-yl]-2-oxoethyl}-benzamide

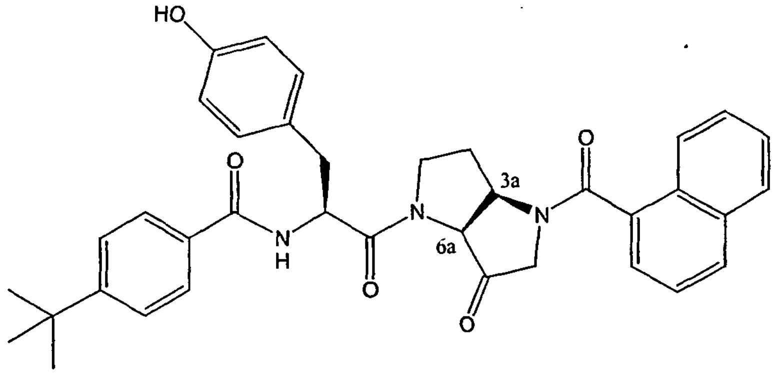

HPLC Rt = 17.8-18.7 mins (> 85%), HPLC-MS 604.2 [M + H]+.

EXAMPLE 354. (3aR, 6a*S*)-4-*tert*-Butyl-*N*-{(1*S*)-1-(4-hydroxy-benzyl)-2-[4-(naphthalene-2-carbonyl)-6-oxo-hexahydro-pyrrolo[3,2-*b*]pyrrol-1-yl]-2-oxoethyl}-benzamide

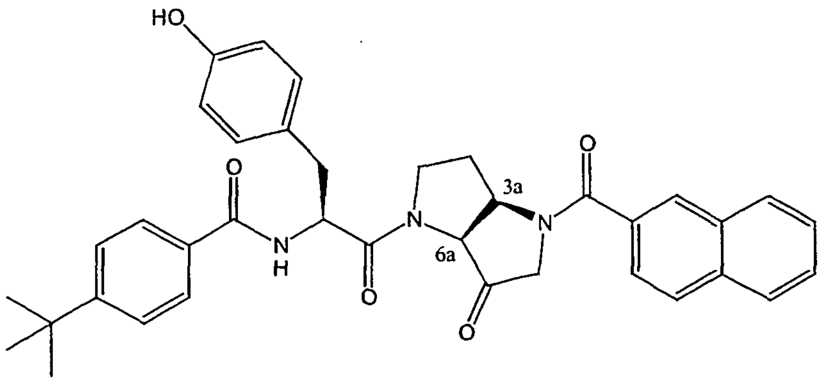

HPLC Rt =17.3-18.2 mins (> 85%), HPLC-MS 604.1 [M + H]+.

EXAMPLE 355. (3a*R*, 6a*S*)-Quinoline-4-carboxylic acid [(1*S*)-2-(4-lienzoyl-6-oxo-hexahydro-pyrrolo[3,2-*b*]pyrrol-1-yl)-1-(4-hydroxy-benzyl)-2-oxo-ethyl]-amide

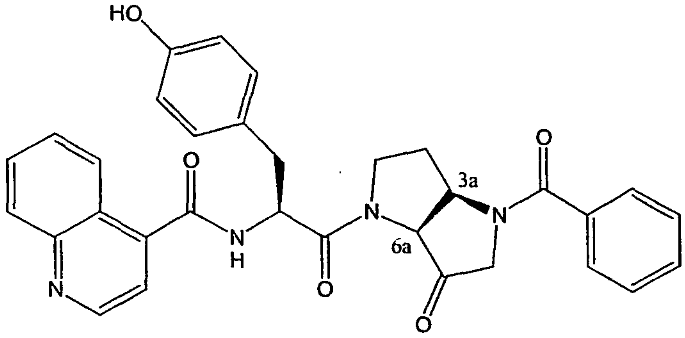

HPLC Rt =10.10 mins (> 90%), HPLC-MS 549.2 $[M + H]^+$, 567.2 $[M + H + H_2O]^+$.

EXAMPLE 356. (3aR, 6a*S*)-*N*-[(1*S*)-2-(4-Benzoyl-6-oxo-hexahydro-pyrrolo[3,2-*b*]pyrrol-1-yl)-1-(4-hydroxy-benzyl)-2-oxo-ethyl]-4-dimethylamino-benzamide

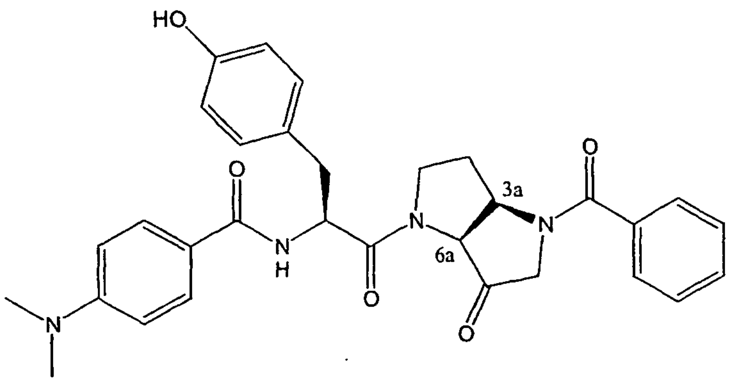

HPLC Rt = 10.34 mins (> 90%), HPLC-MS 541.2 $[M + H]^+$.

EXAMPLE 357. (3a*R*, 6a*S*)-4-Dimethylamino-*N*-{(1*S*)-1-(4-hydroxy-benzyl)-2-[4-(2-methanesulfonyl-benzoyl)-6-oxo-hexahydro-pyrrolo[3,2-*b*]pyrrol-1-yl]-2-oxo-ethyl}-benzamide

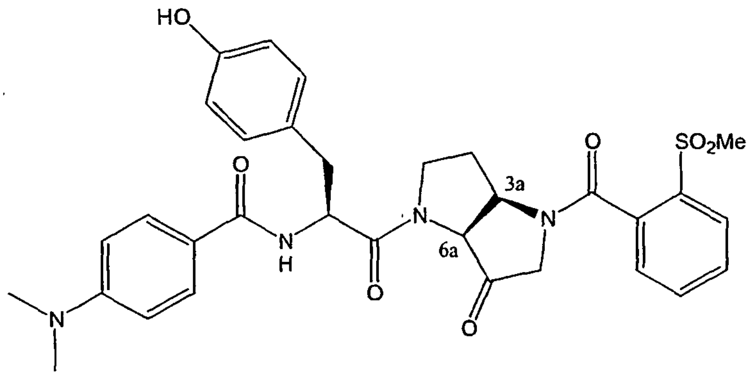

HPLC Rt = 9.78 mins (> 90%), HPLC-MS 619.2 $[M + H]^+$, 637.2 $[M + H + H_2O]^+$.

EXAMPLE 358. (3aR, 6a*S*)-4-Dimethylamino-*N*-{(1*S*)-1-(4-hydroxy-benzyl)-2-[4-(3-methanesulfonyl-benzoyl)-6-oxo-hexahydro-pyrrolo[3,2-*b*]pyrrol-1-yl]-2-oxo-ethyl}-benzamide

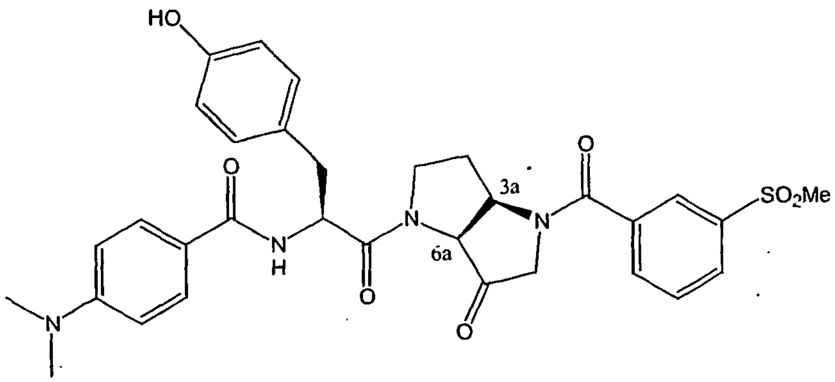

HPLC Rt = 9.95 mins (> 80%), HPLC-MS 619.2 [M + H]$^+$, 637.2 [M + H + $H_2O$]$^+$.

EXAMPLE 359. (3a*R*, 6a*S*)-4-Dimethylamino-*N*-{(1*S*)-1-(4-hydroxy-benzyl)-2-oxo-2-[6-oxo-4-(thiophene-2-carbonyl)-hexahydro-pyrrolo[3,2-*b*]pyrrol-1-yl]-ethyl}-benzamide

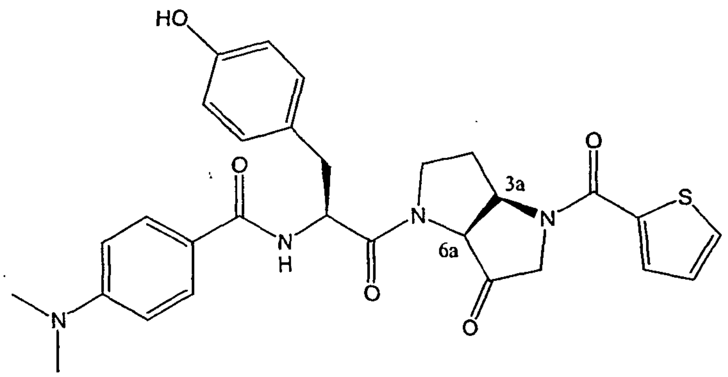

HPLC Rt = 10.69 mins (> 85%), HPLC-MS 547.2 [M + H]$^+$, 565.2 [M + H + $H_2O$]$^+$.

In addition, EXAMPLES 1, 2, 4, 5, 6, 7, 8, 9, 10, 12, 13, 14, 15, 16, 17, 18, 19, 20, 21, 22, 23, 24, 25, 26, 27, 28, 29, 30, 31, 32, 33, 34, 35, 36, 37, 39, 42, 43, 44, 46, 47, 48, 49, 50, 51, 53, 54, 55, 56, 58, 59, 60, 61, 62, 63, 64, 65, 66, 68, 69, 70, 71, 72, 73, 74, 77, 79, 80, 81, 82, 85, 86, 87, 88, 89, 90, 91, 92, 93, 94, 95, 96, 97, 98, 99, 100, 101, 102, 103, 104, 105, 111, 113, 114, 115, 116, 117, 118, 128, 130, 132, 133, 134, 135, 136, 137, 138, 139, 140, 141, 142, 145, 147, 150, 151, 154, 156, 157, 158, 159, 160, 161, 162, 164, 165, 166, 167, 168, 170, 171, 172, 173, 174, 175, 180, 183, 184, 185, 190, 191, 192, 193, 194, 196, 197, 199, 200, 203, 204, 205, 206, 207, 208, 209, 210, 212, 214, 216, 217, 218, 220, 221, 223, 224, 226, 227, 228, 233, 235, 238, 240, 241, 242, 244, 245, 246, 247, 248, 249a, 249b, 249c, 254, 255, 256, 260, 261, 271, 286, 287, 288, 290, 292, 295, 296, 297, 298, 299, 300, 301, 302, 303, 304, 305, 307, 308, 309, 313, 314, 315, 316, 317, 318, 319, 321, 322, 323, 324, 325, 327, 328, 329, 330, 331, 332, 333, 334, 335, 336, 337, 338, 339, 342, 343, 344 and 345 have utility as inhibitors of cruzain and cruzipains with Ki less than 5000nM.

EXAMPLE 360 was prepared as detailed for EXAMPLE 1 substituting the appropriate carboxylic acids as required and is an inhibitor of *Leismania mexicana* CPB protease with Ki less than 5000nM;

EXAMPLE 360. (3a*R*, 6a*S*)-4-*tert*-Butyl-*N*-[(1*S*)-1-(4-methyl-benzyl)-2-oxo-2-(6-oxo-4-phenylacetyl-hexahydro-pyrrolo[3,2-*b*]pyrrol-1-yl)-ethyl]-benzamide

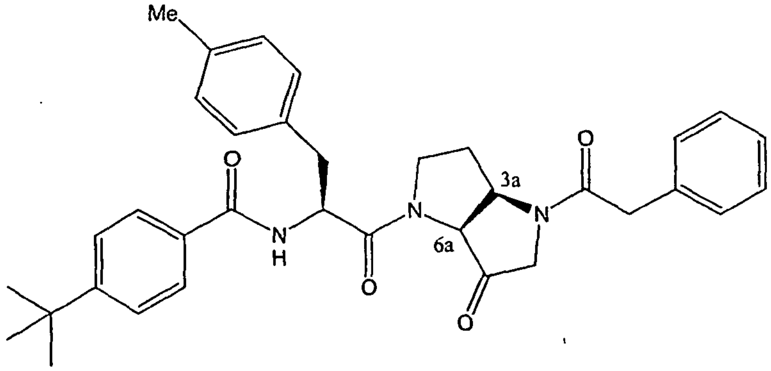

HPLC Rt =19.5-20.6 mins (> 80%), HPLC-MS 566.3 $[M + H]^+$.

In addition, EXAMPLES 1, 2, 3, 5, 6, 13, 14, 16, 17, 20, 21, 24, 25, 26, 27, 28, 29, 31, 32, 34, 35, 37, 39, 42, 43, 44, 46, 47, 48, 50, 56, 57, 59, 60, 62, 63, 64, 65, 66, 68, 69, 70, 71, 72, 73, 74, 75, 76, 77, 82, 85, 86, 87, 88, 89, 90, 91, 92, 93, 95, 96, 97, 98, 99, 100, 101, 102, 103, 104, 105, 111, 112, 113, 114, 115, 116, 117, 118, 119, 120, 128, 130, 132, 134, 135, 136, 137, 138, 139, 141, 142, 145, 147, 148, 150, 151, 154, 158, 159, 160, 161, 164, 165, 166, 167, 170, 171, 172, 173, 174, 175, 176, 177, 178, 179, 180, 181, 182, 185, 190, 191, 192, 193, 194, 201, 204, 207, 208, 209, 212, 213, 214, 215, 216, 220, 230, 233, 234, 235, 245, 246, 247, 248, 249a, 249b, 249c, 271, 278, 286, 295, 314, 315, 316, 318, 319, 329, 330, 331, 332 and 345 have utility as inhibitors of *Leismania mexicana* CPB protease with Ki less than 5000nM.

In addition, EXAMPLES 2, 7, 14, 15, 27, 28, 31, 34, 35, 39, 40, 41, 43, 46, 48, 54, 57, 58, 59, 60, 61, 73, 74, 77, 87, 90, 91, 99, 102, 103, 104, 113, 135, 141, 151, 158, 166, 173, 194, 247, 249a, 249b, 249c, 251, 252, 253, 254, 255, 258, 259, 261, 262, 264, 266, 267, 269, 273, 275, 279, 281, 286, 290, 292, 296, 298, 299, 300, 301, 304, 305, 307, 308, 309, 310, 314, 315, 316, 323, 325, 327, 329 and 330 have utility as inhibitors of cathepsin B with Ki less than 5000nM.

## Solution Phase Syntheses

Alternative strategies to the solid phase techniques described for EXAMPLES 1 → 360 above are broadly outlined in Schemes 15, 17 and 18. Here, building blocks may be prepared for solution phase syntheses for example 3-Oxo-hexahydro-pyrrolo[3,2-*b*]pyrrole-1,4-dicarboxylic acid 1-benzyl ester 4-*tert*-butyl ester (analogue of compound **53,** Scheme 15), prepared in 7 steps as follows:

(1) Preparation of (2*S*, 3*S*) 3-Hydroxypyrrolidine-1,2-dicarboxylic acid 1-*tert*-butyl ester.
Boc anhydride (2.95 g, 13.5 mmol) was added to a stirred solution of the (2*S*, 3*S*)-3-hydroxypyrrolidine-2-carboxylic acid (1.61 g, 12.3 mmol) and sodium carbonate (1.3 g, 12.3 mmol) in a mixture of dioxane (25 ml) and water (12.3 ml). The mixture was stirred for 1.5 h at ambient temperature then evaporated under reduced pressure to afford a residue (~10 ml). The residue was diluted with water (30 ml) then extracted with ethyl acetate (40 ml). The aqueous phase was acidified (pH ~ 2.5) with dilute aqueous hydrochloric acid (0.1 M) then extracted with chloroform (4 x 50 ml). The combined organic layers were dried ($Na_2SO_4$) and evaporated under reduced pressure to afford (2*S*, 3*S*)-3-hydroxypyrrolidine-1,2-dicarboxylic acid 1-*tert*-butyl ester as a white crystalline solid (2.39 g, ~85%), HPLC-MS (single main peak, 254.1 $[M + Na]^+$ and 485.2 $[M + H]^+$).

(2) Preparation of (2*S*, 3*S*) 3-Hydroxypyrrolidine-1,2-dicarboxylic acid 2-allyl ester 1-*tert*-butyl ester
A solution of allyl bromide (26 ml, 301 mmol) and tricaprylmethylammonium chloride (38.4 ml, 86.1 mmol) in dichloromethane (307 ml) was added to a stirred solution of (2*S*, 3*S*) 3-hydroxypyrrolidine-1,2-dicarboxylic acid 1-*tert*-butyl ester (19.89 g, 86.1 mmol) and sodium hydrogen carbonate (7.23 g, 86.1 mmol) in water (307 ml). The biphasic mixture was vigorously stirred overnight then diluted with water (100 ml) and the product extracted into dichloromethane (3 x 200 ml). The combined organic layers were dried ($Na_2SO_4$) and evaporated under reduced pressure to afford a residue. Flash chromatography of the residue over silica gel (400 g) using ethyl acetate : heptane (1 : 4) as the eluent afforded (2*S*, 3*S*) 3-hydroxypyrrolidine-1,2-dicarboxylic acid 2-allyl ester 1-*tert*-butyl ester (9.4 g, 40 %), TLC (single spot, $R_f$ = 0.28, 50% ethyl acetate in heptane), HPLC-MS (single main peak, 294.1 $[M + Na]^+$, 565.3 $[2M + Na]^+$); $\delta_H$ (400 MHz, $CDCl_3$) 1.41 and 1.46 (combined integration 9H, 2 x s, $C(CH_3)_3$ of geometric isomers), 1.87-1.97 (1H, m, 4-H), 2.06-2.18 (1H, m, 4-H), 2.28-2.36 (1H, m, O*H*), 3.55-3.71 (2H, m, 5-$H_2$), 4.20 and 4.32 (combined integration of 1H, 2 x s, 2-H geometric isomers), 4.46 (1H, br. s, 3-H), 4.57-4.73 (1H, m, OC$H_2$), 5.25-5.37

(2H, m, $OCH_2CHCH_2$) and 5.86-5.98 (1H, m, $OCH_2CHCH_2$); $\delta_C$ (100 MHz, $CDCl_3$) 28.6 and 28.7 ($C(CH_3)_3$), 32.5 and 32.95 (C-4), 44.5 and 44.9 (C-5), 66.2 ($OCH_2$), 68.3 (C-2), 74.6 and 75.7 (C-3), 80.6 ($OC(CH_3)_3$), 118.95 and 119.4 ($OCH_2CHCH_2$), 131.9 ($OCH_2CHCH_2$), 154.25 and 154.9 ($NCO_2$), 170.8 and 171.1 ($CO_2$Allyl).

(3) Preparation of (2*S*,3*R*) 3-Azido-pyrrolidine-1,2-dicarboxylic acid 2-allyl ester 1-*tert*-buytl ester

Diethyl azodicarboxylate (0.62 ml, 4.0 mmol) was added dropwise over 25 minutes to a stirred solution of (2*S*, 3*S*) 3-hydroxypyrrolidine-1,2-dicarboxylic acid 2-allyl ester 1-*tert*-butyl ester (895 mg, 3.3 mmol) and triphenylphosphine (1.08 g, 4.1 mmol) in tetrahydrofuran (30 ml) at 0°C under an atmosphere of argon. The solution was stirred for 5 minutes then diphenylphosphoryl azide (0.89 ml, 4.1 mmol) was added dropwise over 10 minutes. The mixture was stirred for 10 minutes at 0°C then for 24 hours at ambient temperature before removing solvents *in vacuo* to obtain a residue which was purified by flash chromatography over silica gel eluting with a gradient of heptane : ethyl acetate 3:1 $\rightarrow$ 1:1. Appropriate fractions were combined and the solvents removed *in vacuo* to obtain (2*S*, 3*R*) 3-azidopymolidine-1,2-dicarboxylic acid 2-allyl ester 1-tert-butyl ester as a colourless oil (850 mg, 88%) which was contaminated with ~5% of 4,5-dihydropyrrole-1,2-dicarboxylic acid 2-allyl ester 1-*tert*-butyl ester. TLC (main spot, Rf = 0.70, heptane : ethyl acetate 1:1), HPLC-MS 197.1 $[M\text{-}Boc+H]^+$, 241.1 $[M\text{-}Bu+2H]^+$, 319.2 $[M+Na]^+$, 615.3 $[2M + Na]^+$; $\delta_H$ ($CDCl_3$ at 298K); (Doubling up of peaks in spectrum due to restricted rotation around Fmoc amide bond) 1.45 and 1.49 (1.8 and 1.2H respectively, $\underline{Me_3}C$, each s), 2.10-2.24 (2H, H-4, m), 3.44-3.52 and 3.60-3.73 (each 1H, H-5, m), 4.31-4.40 (1H, H-3, m), 4.45 and 4.54 (0.6 and 0.4H respectively, H-2, each d, *J* = 7.6Hz), 4.66-4.78 (2H, $C\underline{H_2}CH=CH_2$, m), 5.28-5.33 (1H, $CH_2CH=C\underline{H_2}$, m), 5.42 (1H, $CH_2CH=C\underline{H_2}$, dd, *J*= 17.2 and 3.7Hz), 5.91-6.04 (1H, $CH_2C\underline{H}=CH_2$); $\delta_C$ ($CDCl_3$ at 298K); 28.40 (u, $\underline{Me_3}C$), 29.40/30.18 (d, C-4), 44.22/44.62 (d, C-5), 61.21/61.86/61.98/62.23 (u, C-2 and C-3), 65.81/66.10 (d, $\underline{C}H_2CH=CH_2$), 80.76/81.24 (q, $Me_3\underline{C}$), 118.79/119.21 (d, $CH_2CH=\underline{C}H_2$), 131.82/131.87 (u, $CH_2\underline{C}H=CH_2$), 153.53/154.11 (q, $O\underline{C}ON$), 169.19/169.38 (q, $\underline{C}O_2CH_2$).

(4) Preparation of (2*S*, 3*R*) 3-Benzyloxycarbonylaminopyrrolidine-1,2-dicarboxylic acid 1-*tert*-butyl ester 2-propyl ester

Palladium (10 wt. % on carbon powder, 300 mg) was added portion wise to a solution of (2*S*, 3*R*) 3-azidopyrrolidine-1,2-dicarboxylic acid 2-allyl ester 1-*tert*-butyl ester (790 mg, 2.66 mmol) in ethanol (20 ml) at 0 °C. The mixture was stirred for 2 h under an atmosphere of hydrogen at ambient temperature, then filtered over celite and concentrated *in vacuo* to afford a residue (540 mg). The residue was suspended in dioxane (6 ml) then a solution of sodium carbonate (529 mg, 5 mmol) in water (12 ml) added. The mixture was cooled to 0 °C then a solution of benzyloxy chloroformate (0.314 ml) in dioxane (6 ml) added portion wise over 40 min at 0 °C. The mixture was stirred for 30 min at 0 °C then at ambient temperature for 40 min. Water (150 ml) was added and the products extracted with chloroform (3 x 100 ml), dried ($Na_2SO_4$) and evaporated under reduced pressure to afford a residue. Flash chromatography of the residue over silica (100 g) using ethyl acetate : heptane (1 : 4) as the eluent afforded (2*S*, 3*R*) 3-benzyl oxycarbonylaminopyrrolidine-1,2-dicarboxylic acid 1-*tert*-butyl ester 2-propyl ester (240 mg, 22 %), TLC (single spot, $R_f$ = 0.47, 50% ethyl acetate in heptane); analytical HPLC $R_t$ = 18.06 min, HPLC-MS (single main peak, 429.2 $[M + Na]^+$, 835.4 $[2M + Na]^+$); $\delta_H$ (400 MHz, $CDCl_3$) 0.90 (3H, t, *J* = 7.3 Hz, $CH_3$), 1.41 and 1.45 (combined integration 9H, 2 x s, $C(CH_3)_3$ of geometric isomers), 1.46-1.66 ($OCH_2CH_2CH_3$), 1.89-2.00 (1H, m, 4-$H_2$), 2.12-2.25 (1H, m, 4-$H_2$), 3.32-3.46 (1H, m, 5-H), 3.55-3.72 (1H, m, 5-H), 3.99-4.07 (1H, m, 3-H), 4.46-4.55 ($OCH_2CH_2CH_3$), 4.88-5.17 (combined integration of 3H, m, 2-H and $OCH_2Ph$), and 7.29-7.42 (6H, m, $C_6H_5$ and NH); $\delta_C$ (100 MHz, $CDCl_3$) 10.5 ($CH_3$), 22.10 ($OCH_2CH_2$), 28.41 and 28.50 ($C(CH_3)_3$), 29.4 and 30.5 (C-4), 43.9 and 44.3 (C-5), 52.8 (C-3), 60.5 and 61.3 (C-2), 66.9, 67.0 and 67.2 ($OCH_2Ph$ and $OCH_2$), 80.5 ($OC(CH_3)_3$), 128.3, 128.4 and 128.7 (*o*-, *m*- and *p*-$C_6H_5$), 136.2 ($NHCOOCH_2C$), 153.8 and 155.7 ($NCO_2$ and $NHCO_2$) and 171.1 ($CO_2$Propyl).

(5) Preparation of (2*S*, 3*R*) 3-Benzyloxycarbonylaminopyrrolidine-1,2-dicarboxylic acid 1-*tert*-butyl ester

A solution of sodium hydroxide (185 mg, 4.6 mmol) in water (1.6 ml) was added to a solution of (2*S*, 3*R*) 3-benzyloxycarbonylaminopyrrolidine-1,2-dicarboxylic acid 1-*tert*-butyl ester 2-propyl ester (229 mg, 0.56 mmol) in ethanol (6 ml). The mixture was stirred for 8 h at ambient temperature. Water (50 ml) was added and the ethanol removed under reduced pressure. The aqueous residue was acidified to pH = 2 by the addition of dilute aqueous hydrochloric acid (0.1 M). The mixture was then extracted with ethyl acetate (3 x 50 ml), dried ($Na_2SO_4$) and evaporated under reduced pressure to afford (2*S*, 3*R*) 3-benzyloxycarbonylaminopyrrolidine-1,2-dicarboxylic acid 1-*tert*-butyl ester (200 mg, 98%), analytical HPLC $R_t$ = 14.03 min, HPLC-MS (single main peak, 387.2 $[M + Na]^+$, 751.4 $[2M + Na]^+$); $\delta_H$ (400 MHz, $CDCl_3$) 1.35 and 1.37 (combined integration 9H, 2 x s, $C(CH_3)_3$ of geometric isomers), 1.86-2.15 (2H, m, 4-$H_2$), 3.14-3.71 (2H, m, 5-$H_2$), 4.20-4.49 (2H, m, 2-H and 3-H), 5.02-5.22 (2H, m, $OCH_2Ph$), and 7.18-7.37 (6H, m, $C_6H_5$ and NH); $\delta_C$ (100 MHz, $CDCl_3$) 28.3, 28.4, 28.5 and 29.2 ($C(CH_3)_3$ and C-4), 43.8 and 44.3 (C-5), 52.3 and 53.1 (C-3), 61.0 and 61.4 (C-2), 67.2 and 68.4 ($OCH_2Ph$), 80.6 and 80.8 ($OC(CH_3)_3$), 128.2, 128.5 and 128.6 and 128.7 (*o*-, *m*- and *p*-$C_6H_5$), 135.5 ($NHCOOCH_2C$), 153.8 and 154.3 ($NCO_2$), 158.6 ($NHCO_2$), 175.6 and 175.9 ($CO_2H$).

(6) Preparation of (2*S*, 3*R*) 3-Benzyloxycarbonylamino-2-(2-diazoacetyl) pyrrolidine-1-carboxylic acid *tert*-butyl ester (2*S*, 3*R*) 3-benzyloxycarbonylaminopyrrolidine-1,2-dicarboxylic acid 1-*tert*-butyl ester (161 mg, 0.443 mmol) was dissolved with stirring in anhydrous dichloromethane (18 ml). The reaction was flushed with nitrogen and cooled to -15 °C. *iso*-Butylchloroformate (0.063 ml, 0.487 mmol) in anhydrous dichloromethane (1.5 ml) and *N*-methylmorpholine (0.097 ml, 0.886 mmol) in anhydrous dichloromethane (1.5 ml) were added simultaneously in 0.5 ml aliquots over 15 min. The mixture was stirred for 45 min at -15 °C then ethereal diazomethane [generated from addition of diazald (4.7 g, ~15 mmol) in diethyl ether (75 ml) onto sodium hydroxide (5.25 g) in water (7.5 ml) / ethanol (15 ml) at 60 °C] was added to the activated amino acid solution. The mixture was allowed to warm to ambient temperature and stirred for 24 h. A few drops of acetic acid were added to the mixture, followed by diethyl ether (200 ml). The ethereal layer was washed with saturated aqueous sodium hydrogen carbonate (80 ml), dried ($Na_2SO_4$) and the solvents removed under reduced pressure to give a yellow residue (300 mg). Flash chromatography of the residue over silica (35 g) using ethyl acetate : heptane (1 : 3) afforded (2*S*, 3*R*) 3-benzyloxycarbonylamino-2-(2-diazoacetyl)pyrrolidine-1-carboxylic acid *tert*-butyl ester (150 mg, 87%), TLC (single spot, $R_f$ = 0.29, 50% ethyl acetate in heptane); analytical HPLC $R_t$ = 15.15 min, HPLC-MS (single main peak, 411.2 $[M + Na]^+$, 799.4 $[2M + Na]^+$).

(7) Preparation of (2*S*, 3*R*) 3-Oxohexahydropyrrolo[3,2-*b*]pyrrole-1,4-dicarboxylic acid 1-benzyl ester 4-*tert*-butyl ester

A solution of (2*S*, 3*R*) 3-benzyloxycarbonylamino-2-(2-diazoacetyl) pyrrolidine-1-carboxylic acid *tert*-butyl ester (90 mg, 0.23 mmol) was added over 1 h to a refluxing solution of rhodium(II) acetate dimer (2 mg, 0.0046 mmol) in dichloromethane (3 ml). The mixture was heated under reflux for 2 h and the solvent then removed *in vacua*. The residue was diluted with tetrahydrofuran (40 ml) then filtered through a pad of celite. The filtrate was then concentrated *in vacuo* to afford a residue (106 mg). Flash chromatography of the residue over silica (35 g) using ethyl acetate : heptane (1 : 3) afforded (2*S*, 3*R*) 3-oxohexahydropyrrolo[3,2-*b*]pyrrole-1,4-dicarboxylic acid 1-benzyl ester 4-*tert*-butyl ester (14 mg, 17 %), TLC (two possible rotameric spots, $R_f$ = 0.30 and 0.23, 50% ethyl acetate in heptane), analytical HPLC $R_t$ = 15.479 min (~60 %), 16.393 (~10 %), 17.197 (~10 %), 18.085 (~10 %) and 21.665 (~10 %); HPLC-MS (4 main peaks, 361.0 = $[M + H]^+$, 379 = $[M + H_3O]^+$, 401.0 $[M + H_2O + Na]^+$, $[2M + H]^+$= 721.0, $[2M + Na]^+$= 743.0, $[2(M + H_2O) + Na]^+$ = 779.0); $\delta_H$ (500 MHz, $CDCl_3$) 1.40-1.50 (9H, m, $C(CH_3)_3$), 1.78-1.86 (1H, m, $NCH_3CH_2$), 2.30-2.47 (1H, m, $NCH_2CH_2$), 3.90-4.06 (2H, m, $NCH_2$), 5.05-5.20 (4H, m, $OCH_2$, BocNC*H*CO and CbzNC*H*), 5.44 (1H, d, *J* = 2.2 Hz, $CbzNCH_2$), 5.56 (1H, br. s, $CbzNCH_2$) and 7.30-7.37 (5H, m, $C_6H_5$); $\delta_C$ (126 MHz, $CDCl_3$) 27.5 ($C(CH_3)3$), 29.6 and 32.2 ($NCH_2CH_2$), 57.7 (CbzN*C*H), 59.1 ($NCH_2$), 67.0 ($OCH_2$), 77.2 (BocN*C*H), 83.5 ($OC(CH_3)_3$), 110.7 ($CbzNCH_2$), 128.0, 128.2 and 128.5 (*o*-, *m*- and *p*-$C_6H_5$), 136.1 ($OCH_2C$ of Cbz), 147.6, 151.4 155.7 and 167.1 (2x$NCO_2$ and $CbzNCH_2CO$).

(8) Preparation of (2*S*, 3*R*) 3-Oxohexahydropyrrolo[3,2-*b*]pyrrole-1-carboxylic acid benzyl ester hydrochloride salt (analogue of compound 54).

(2*S*, 3*R*) 3-oxohexahydropyrrolo[3,2-*b*]pyrrole-1,4-dicarboxylic acid 1-benzyl ester 4-*tert*-butyl ester (9 mg, 0.025 mmol) was dissolved in 4M HCl in dioxane (1.25 ml) then stirred for 1 h at room temperature. The mixture was concentrated *in vacuo* then toluene (10 ml) added. The mixture was concentrated once again *in vacuo* and the procedure repeated to afford a residue (10 mg, ~100 %), HPLC-MS (single main peak, 261.1 $[M + H]^+$ and 279.1 $[M + H_3O]^+$).

Alternatively, a useful building block for solution phase synthesis is (3*S*, 3a*S*, 6a*R*)-3-hydroxyhexahydropyrrolo[3,2-*b*]pyrrole-1,4-dicarboxylic acid 4-benzyl ester 1-*tert*-butyl ester **(80)** described earlier in Scheme 20. The utility of building block **(80)** is detailed in alternative syntheses of EXAMPLES 1 and 14, through Scheme 26, which is an example of the general synthetic strategy detailed in Scheme 18.

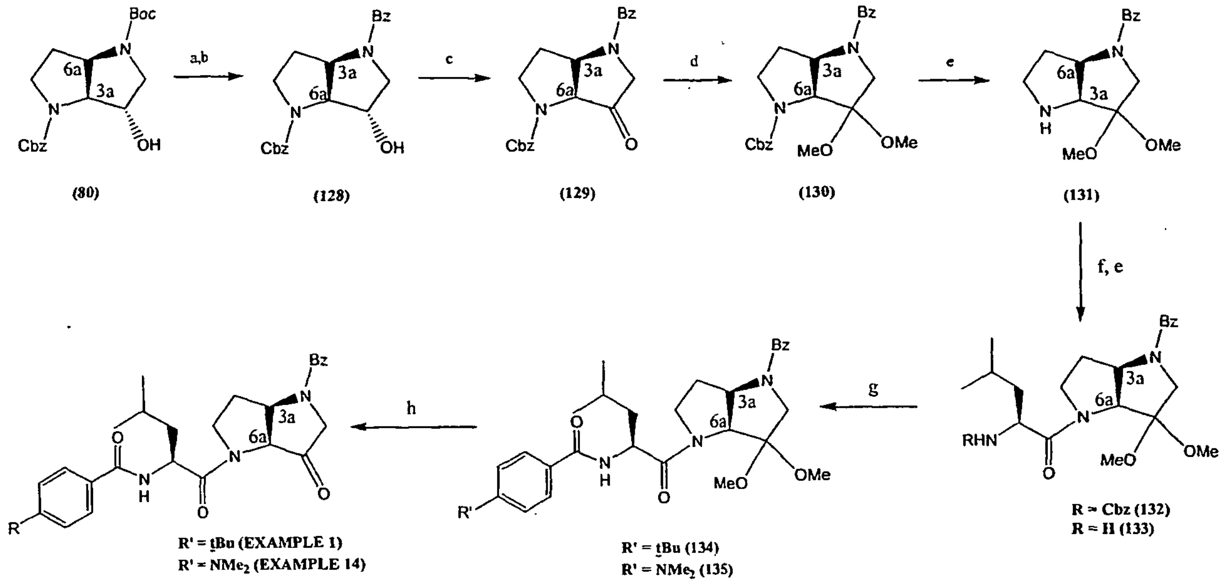


**Scheme 26.** (a) 4N HCl in 1,4-dioxane, 30mins, RT. (b) Benzoic anhydride, 4-methylmorpholine, DMF, RT, 1hr. (c) Dess-Martin periodinane, DCM. (d) Trimethylorthoformate, anhydrous MeOH, cat. *p*-TsOH, under Ar, 65°C. (e) Pd-C / $H_2$, ethanol / methanol. (f) 1eq Cbz-Leu-F, DMF, RT. (g) 1.05eq R-COOH, HBTU, HOBT, NMM, DMF, RT. (h) 95% Trifluoroacetic acid / 5% water, RT.

**Preparation of (3a*R*, 6*S*, 6a*S*)-4-benzoyl-6-hydroxyhexahydropyrrolo[3,2-*b*]pyrrole-1-carboxylic acid benzyl ester (128)**

A solution of HCl in 1,4-dioxane (4.0M, 11 ml, 44 mmol) was added to (3*S*, 3a*S*, 6a*R*)-3-hydroxyhexahydropyrrolo[3,2-*b*]pyrrole-1,4-dicarboxylic acid 4-benzyl ester 1-*tert*-butyl ester **(80)** (450 mg, 1.24 mmol). The solution was stirred for 65 minutes whereupon a white suspension formed. The solvents were removed *in vacuo* and the residue azeotroped with diethyl ether (3x 15 ml) and then dimethylformamide (10 ml) and benzoic anhydride (295 mg, 1.31 mmol) added. The solution was placed under an atmosphere of argon then 4-methylmorpholine (0.29 ml, 2.6 mmol) was added to the solution dropwise whilst stirring over 0.5 minutes. The mixture was stirred for 1.75 hours then the solvents were removed *in vacuo*. The residue was dissolved in ethyl acetate (100 ml) then washed with saturated aqueous sodium hydrogen carbonate solution (100 ml), pH 3 hydrochloric acid (100 ml) then brine (100 ml). The organic layer was dried ($Na_2SO_4$) and evaporated *in vacuo* to afford (3a*R*, 6*S*, 6a*S*)-4-benzoyl-6-hydroxyhexahydropyrrolo[3,2-*b*]pyrrole-1-carboxylic acid benzyl ester **(128)** as a pale yellow gum (465 mg), which was used without further purification. Analytical HPLC $R_t$ = 14.967 min; HPLC-MS 367.1 $[M + H]^+$, 733.1 $[2M + H]^+$; Elemental analysis $C_{21}R_{22}N_2O_{4.}$0.4EtOAc req.(fnd.) % C 67.62 (67.73), % H 6.33 (6.17), % N 6.98 (7.08); HRMS $C_{21}H_{22}N_2O_4Na$ req. 389.1477, fnd. 389.1476 (-0.40ppm); $d_H$ (500 MHz, $CDCl_3$) approximately 3 : 1 mixture of rotamers, 2.10-2.21 (1H, m, BzNCHC*H*$_2$), 2.24-2.36 (1H, m, BzNCHC*H*$_2$), 3.20-3.35 (1H, m, CbzNC*H*$_2$), 3.35-3.66 (2H, m, BzNC*H*$_2$), 3.74-3.80 (1H, m, CbzNC*H*$_2$), 4.16-4.20 (1H, m, CbzNC*H*), 4.38-4.42 (1H, br. s; C*H*OH), 4.94-5.04 (1H, m, BzNC*H*), 5.08-5.22 (2H, m, OC*H*$_2$Ph), 7.30-7.52 (10H, aromatic C*H*); $d_C$ (125 MHz, $CDCl_3$) 31.02, 30.40 (BzNCH$\underline{C}$H$_2$), 45.73, 45.86 (CbzN$\underline{C}$H$_2$), 56.43, 56.74 (BzN$\underline{C}$H$_2$), 60.58, 61.49 (BzN$\underline{C}$H), 66.68, 67.33 (O$\underline{C}$H$_2$Ph), 66.92, 67.76 (CbzN$\underline{C}$H), 73.01, 73.86 ($\underline{C}$HOH), 126.77, 127.48, 127.99, 128.23, 128.28, 128.45, 128.56, 128.87, 130.02, 130.38 and 134.53 ($\underline{C}$H aromatics), 136.16, 136.23, 136.33 (aromatic quaternary), 154.26, 154.97 (Cbz$\underline{C}$=O), 170.06, 171.19 (Bz$\underline{C}$=O).

**Preparation of (3a*R*, 6a*S*)-4-benzoyl-6-oxo-hexahydropyrrolo[3,2-*b*]pyrrole-1-carboxylic acid benzyl ester (129)**

(3a*R*, 6*S*, 6a*S*)-4-Benzoyl-6-hydroxyhexahydropyrrolo[3,2-*b*]pyrrole-1-carboxylic acid benzyl ester **(128)** (0.78 g, 2.13 mmol) was dissolved in dichloromethane (20 ml) with stirring under argon. Dess-Martin periodinane (1.804 g, 4.26 mmol) was added and the mixture stirred for 16 hours. The mixture was concentrated *in vacuo* and the residue purified by flash chromatography over silica, eluting with ethyl acetate : heptane mixtures to give (3a*R*, 6a*S*)-4-benzoyl-6-oxo-hexahydropyrrolo[3,2-*b*] pyrrole-1-carboxylic acid benzyl ester **(129)** (0.61 g, 78%) as an off-white gum. TLC ($R_f$ = 0.27, EtOAc : heptane 3 : 1), analytical HPLC single main peak, $R_t$ = 14.65-16.30 min., HPLC-MS 365.1 $[M + H]^+$,

383.1 [M + H + $H_2O$]$^+$; Elemental analysis $C_{21}H_{20}N_2O_4$.1.2$H_2O$ req.(fnd.) % C 65.38 (65.12), % H 5.85 (5.65), % N 7.26 (6.95); HRMS $C_{21}H_{20}N_2O_4$Na req. 387.1321, fnd. 387.1324 (0.76ppm); $\delta_C$ (125 MHz, $CDCl_3$) 30.41, 30.89, 31.23 (BzNCH$\underline{C}H_2$), 45.75 (CbzN$\underline{C}H_2$), 54.55, 63.04 (BzN$\underline{C}$H + CbzN$\underline{C}$H), 57.91, 58.45, 58.99, 59.73 (BzN$\underline{C}H_2$), 67.60, 68.07 (O$\underline{C}H_2$Ph), 127.00, 127.38, 127.48, 127.98, 128.11, 128.48, 128.62, 128.74, 130.48, 130.83 ($\underline{C}$H aromatics), 135.07, 136.14 (quaternary aromatics), 154.54, 155.03 (Cbz$\underline{C}O_2$), 170.58 (Bz$\underline{C}$O), 204-207 (broad, $\underline{C}$=O).

**Preparation of (3a*R*, 6a*S*)-4-benzoyl-6,6-dimethoxyhexahydropyrrolo[3,2-*b*] pyrrole-1-carboxylic acid benzyl ester (130)**

(3a*R*, 6a*S*)-4-Benzoyl-6-oxo-hexahydropyrrolo[3,2-*b*]pyrrole-1-carboxylic acid benzyl ester **(129)** (0.60 g, 1.65 mmol) was dissolved in methanol (10 ml) with stirring. Trimethylorthoformate (1.8 ml, 16.5 mmol) was added followed by *para*toluenesulfonic acid (40 mg) and the mixture heated under argon at 65 °C for 16 hours. The mixture was reduced *in vacuo* to leave a dark oil (0.8 g). Flash chromatography over silica, eluting with ethyl acetate : heptane mixtures gave (3a*R*, 6a*S*)-4-benzoyl-6,6-dimethoxyhexahydropyrrolo[3,2-*b*]pyrrole-1-carboxylic acid benzyl ester **(130)** (0.52 g, 77%) as a fine white crystalline solid. TLC ($R_f$= 0.40, EtOAc : heptane 3 : 1), analytical HPLC single main peak, $R_t$ = 18.22 min., HPLC-MS 411.1 [M + H]$^+$, 433.1 [M + Na]$^+$, 843.1 [2M + Na]$^+$; HRMS $C_{23}H_{26}N_2O_5$Na req. 433.1739, fnd. 433.1727 (-2.94ppm); $\delta_H$ (500 MHz, $CDCl_3$) 1.96-2.07, 2.15-2.22 (2H, m, BzNCHC$H_2$), 3.04-3.42 (6H, m, 2x OC$H_3$), 3.25 (1H, m, CbzNC$H_2$), 3.4 (1H, m, BzNC$H_2$), 3.58-3.67 (1H, m, BzNC$H_2$), 3.96-4.07 (1H, m, CbzNC$H_2$), 4.35-4.58 (1H, m, CbzNC*H*), 4.98-5.26 (3H, BzNC*H*+ OC$H_2$Ph), 7.28-7.49 (10H aromatics); $\delta_C$ (125 MHz, $CDCl_3$) 32.27, 32.59 (BzNCH$\underline{C}H_2$), 46.74 (CbzN$\underline{C}H_2$), 49.36, 51.10, 51.59 (2x O$\underline{C}H_3$), 54.59, 56.08 (BzN$\underline{C}H_2$), 60.77, 61.08 (BzN$\underline{C}$H), 62.47 (CbzN$\underline{C}$H), 67.28 (O$\underline{C}H_2$Ph), 106.76, 107.02 ($\underline{C}(OCH_3)_2$), 126.84, 127.35, 127.90, 128.06, 128.39, 130.05, 130.38 ($\underline{C}$H aromatics), 135.91, 136.48 (quaternary aromatics), 155.44 (Cbz$\underline{C}O_2$), 169.54 (Bz$\underline{C}$O).

**Preparation of (3a*S*, 6a*R*)-(3,3-dimethoxyhexahydropyrrolo[3,2-*b*]pyrrol-1-yl)phenylmethanone (131)**

Methanol (15 ml) was added cautiously dropwise to a stirred mixture of (3a*R*, 6a*S*)-4-benzoyl-6,6-dimethoxyhexahydropyrrolo[3,2-*b*]pyrrole-1-carboxylic acid benzyl ester **(130)** (0.48 g, 1.17 mmol) and 10% palladium on charcoal (100 mg) at 0 °C under an atmosphere of argon over 10 minutes. The argon was then replaced by an atmosphere of hydrogen and stirring continued at ambient temperature for 85 minutes before replacing the hydrogen with argon and adding ethanol (30 ml). The mixture was filtered under reduced pressure through celite and the filter cake washed with methanol (25 ml) then ethanol (70 ml). Solvents were removed from the filtrate *in vacuo* to obtain (3a*S*, 6a*R*)-(3,3-dimethoxyhexahydropyrrolo[3,2-*b*]pyrrol-1-yl) phenylmethanone **(131)** as a colourless oil (340 mg), which was used without further purification. HPLC-MS 277.1 [M + H]$^+$, 553.2 [2M + H]$^+$.

**Preparation of (3a*R*, 6a*S*)-[(1*S*)-1-(4-benzoyl-6,6-dimethoxyhexahydropyrrol [3,2-*b*]pyrrole-1-carbonyl)-3-methylbutyl]carbamic acid benzyl ester (132)**

(i) Preparation of Cbz-L-Leucine Fluoride
Cbz-L-Leucine (1.115 g, 4.2 mmol) was dissolved in dichloromethane (50 ml) with stirring under argon. (Diethylamino)sulfur trifluoride (DAST, 792 μl, 6.0 mmol) was added and the mixture stirred for 40 minutes. Ice-cooled water (200 ml) was added to the mixture and the organic layer separated, dried ($Na_2SO_4$) and reduced *in vacuo* to a mobile tan oil (1.14 g). An analytical sample, pre-treated with 10% pyridine in methanol for 15 minutes gave HPLC-MS 266.1 [M + H]$^+$ (acid, < 5%), 280.1 [M + H]$^+$, 302.1 [M + Na]$^+$, 581.1 [2M + Na]$^+$ (methyl ester, ~ 95%).

(ii) Crude (3a*S*, 6a*R*)-(3,3-dimethoxyhexahydropyrrolo[3,2-*b*]pyrrol-1-yl)phenyl methanone **(131)** (~ 1.17 mmol) was dissolved in anhydrous dimethylformamide (5 ml) with stirring. Cbz-L-Leucine fluoride (0.33 g, 1.23 mmol) was added and the mixture stirred under argon for 1 hour. The mixture was reduced *in vacuo* to a semi-mobile dark oil (~ 1.0 g). Flash chromatography over silica, eluting with ethyl acetate : heptane mixtures gave (3a*R*, 6a*S*)-[(1*S*)-1-(4-benzoyl-6,6-dimethoxyhexahydro pyrrolo[3,2-*b*]pyrrole-1-carbonyl)-3-methylbutyl]carbamic acid benzyl ester **(132)** (0.55 g, 90%) as an off-white crystalline solid. TLC ($R_f$= 0.35, EtOAc : heptane 3 : 1), analytical HPLC single main peak, $R_t$ = 19.396 min., HPLC-MS 524.1 [M + H]$^+$, 546.1 [M + Na]$^+$, 1069.2 [2M + Na]$^+$; Elemental analysis $C_{29}H_{37}N_3O_6$ req.(fnd.) % C 66.52 (66.26), % H 7.12 (7.30), % N 8.02 (7.86); HRMS $C_{29}H_{37}N_3O_6$Na req. 546.2580, fnd. 546.2584 (0.67ppm); $\delta_H$ (500 MHz, $CDCl_3$) 0.92-1.04 (6H, m, 2x Leu δC$H_3$), 1.45-1.55 (2H, m, Leu βC$H_2$), 1.73-1.84 (1H, m, Leu γC*H*), 1.92-1.99, 2.10-2.16, 2.22-2.30 (2H, 4 : 6 : 10, m, BzNCHC$H_2$), 2.94, 3.19, 3.23 and 3.40 (6H total, each s, C(OC$H_3$)$_2$), 3.14-3.38 (2H, m, 1x BzNC$H_2$ and 1x CbzLeuNC$H_2$), 3.60-3.68 (1H, 4 : 6, each d, *J* = 12 Hz, 1x BzNC$H_2$), 4.03-4.10, 4.11-4.18 (1H, 4 : 6, m, 1x CbzLeuNC$H_2$), 4.33 (0.4H, d, *J* = 6.3 Hz,

0.4x CbzLeuNC*H*), 4.5-4.65 (0.6H, m, 0.6x Leu αC*H*), 4.82 (0.6H, d, *J* = 6.45 Hz, 0.6x CbzLeuNC*H*), 4.87-4.93 (0.4H, m, 0.4x Leu αC*H*), 5.0-5.14 (3H, m, BzNC*H* + OC$H_2$Ph), 5.42 (0.6H, d, *J* = 8.3 Hz, LeuN*H*), 5.57 (0.4H, d, *J* = 8.9 Hz, LeuN*H*), 7.3-7.5 (10H, aromatics); $\delta_C$ (125 MHz, $CDCl_3$) 21.99, 22.20, 22.67, 23.06, 23.67 (2x Leu δ$\underline{C}H_3$), 24.37, 24.57 (Leu γ$\underline{C}$H), 31.58, 31.86, 33.26 (BzNCH$\underline{C}H_2$), 42.78 (Leu β$\underline{C}H_2$), 44.12, 45.79, 47.05 (CbzLeuN$\underline{C}H_2$), 49.31, 49.99 (1x O$\underline{C}H_3$), 51.18, 51.27, 51.30, 51.47 (1x O$\underline{C}H_3$ + Leu α$\underline{C}$H), 55.55, 57.03 (BzN$\underline{C}H_2$), 59.69, 61.32 (BzN$\underline{C}$H), 60.30, 61.04 (CbzLeuN$\underline{C}$H), 66.39, 66.88(O$\underline{C}H_2$Ph), 106.27, 107.11 ($\underline{C}(OCH_3)_2$), 126.84, 127.32, 127.42, 127.87, 127.94, 128.09, 128.43, 128.48, 130.18, 130.43, 130.50 ($\underline{C}$H aromatics), 135.68, 135.81, 136.28, 136.73 (quaternary aromatics), 155.58, 156.21 (Cbz$\underline{C}O_2$), 169.56, 169.62 (Bz$\underline{C}$O), 172.35, 173.36 (Leu $\underline{C}$=O).

**Preparation of (2*S*, 3a*R*, 6a*S*)-2-amino-1-(4-benzoyl-6,6-dimethoxyhexahydro pyrrolo[3,2-*b*]pyrrol-1-yl)-4-methylpentan-1-one (133)**

Methanol (15 ml) was added cautiously dropwise to 10% palladium on charcoal (75 mg) at 0 °C under an atmosphere of argon over 10 minutes whilst stirring followed by a solution of (3a*R*, 6a*S*)-[(1*S*)-1-(4-benzoyl-6,6-dimethoxy hexahydropyrrolo[3,2-*b*]pyrrole-1-carbonyl)-3-methylbutyl]carbamic acid benzyl ester **(132)** (0.52 g, 0.99 mmol) in methanol (15 ml). The argon was then replaced by an atmosphere of hydrogen and stirring continued at ambient temperature for 5.5 hours. A suspension of 10% palladium on charcoal (15 mg) in methanol (1 ml) was added and stirring continued for 2.25 hours. The hydrogen was replaced by argon then ethanol (100 ml) was added before filtering the mixture through celite. The filter cake was washed with ethanol (100 ml) then the filtrate separated into two equal portions before concentrating separately *in vacuo* to obtain two identical batches of (2*S*, 3a*R*, 6a*S*)-2-amino-1-(4-benzoyl-6,6-dimethoxy hexahydropyrrolo[3,2-*b*]pyrrol-1-yl)-4-methyl pentan-1-one **(133).** TLC (Single spot, $R_f$= 0.05, EtOAc : heptane 9 : 1), HPLC-MS 390.2 [M + H]$^+$, 801.2 [2M + Na]$^+$ as white solids which were contaminated with approximately 5% of (3a*R*, 6a*S*)-[(1*S*)-1-(4-benzoyl-6,6-dimethoxyhexahydropyrrolo[3,2-*b*]pyrrole-1-carbonyl)-3-methylbutyl]carbamic acid benzyl ester **(132)** starting material. Each batch was used without further purification (see preparations of **(134)** and **(135)** below).

**Preparation of (3a*R*, 6a*S*)-*N*-[(1*S*)-1-(4-benzoyl-6,6-dimethoxy hexa hydropyrrolo[3,2-*b*]pyrrole-1-carbonyl)-3-methylbutyl]-4-*tert*-butyl benzamide (134)**

4-Methylmorpholine (0.109 ml, 0.994 mmol) was added to a solution of HBTU (189 mg, 0.497 mmol), 1-hydroxybenzotriazole monohydrate (76 mg, 0.497 mmol) and 4-(*tert*-butyl)benzoic acid (88 mg, 0.497 mmol) in dimethylformamide (12.5 ml). The solution was stood for 5 minutes then added to (2*S*, 3a*R*, 6a*S*)-2-amino-1-(4-benzoyl-6,6-dimethoxyhexahydropyrrolo[3,2-*b*]pyrrol-1-yl)-4-methylpentan-1-one **(133)** (prepared as above, 0.497 mmol). The mixture was stirred at ambient temperature for 1 hour 50 minutes then the solvents were removed *in vacuo*. The residue was dissolved in dichloromethane (60 ml) then washed with pH 3 hydrochloric acid (40 ml), saturated aqueous sodium hydrogen carbonate solution (40 ml) and brine (40 ml), then dried ($Na_2SO_4$) and the solvents removed *in vacuo*. The residue was purified by flash chromatography over silica eluting with ethyl acetate : heptane mixtures 0 : 100 to 50 : 50 to give (3a*R*, 6a*S*)-*N*-[(1*S*)-1-(4-benzoyl-6,6-dimethoxyhexahydro pyrrolo [3,2-*b*]pyrrole-1-carbonyl)-3-methylbutyl]-4-*tert*-butyl benzamide **(134)** as a white solid (230 mg) which contained approximately 5% of (3a*R*, 6a*S*)-[(1*S*)-1-(4-benzoyl-6,6-dimethoxyhexahydropyrrolo[3,2-*b*]pyrrole-1-carbonyl)-3-methylbutyl] carbamic acid benzyl ester **(132)** The latter compound was removed prior to the hydrolysis step (i.e. preparation of EXAMPLE 1 below) by dissolving (220 mg) in methanol (11 ml) then adding to a stirred suspension of 10% palladium on charcoal (70 mg) in ethanol (11 ml) under an atmosphere of argon at 0 °C. The argon was then replaced by hydrogen and the mixture stirred at ambient temperature for 80 minutes, then water (11 ml) was added and the mixture filtered through celite. The filter cake was washed with ethanol (200 ml) then the filtrate concentrated in *vacuo* to give an oily solid (3a*R*, 6a*S*)-*N*-[(1*S*)-1-(4-benzoyl-6,6-dimethoxyhexahydropyrrolo[3,2-*b*]pyrrole-1-carbonyl)-3-methylbutyl]-4-*tert*-butylbenzamide **(134)** (203 mg) which was used without further purification. TLC (Single spot, $R_f$ = 0.65, EtOAc in heptane 9 : 1), analytical HPLC $R_t$ = 21.412 min; HPLC-MS 550.2 [M + H]$^+$; Elemental analysis $C_{32}H_{43}N_3O_5$ req.(fnd.) % C 69.92 (69.52), % H 7.88 (8.12), % N 7.64 (7.40); HRMS $C_{32}H_{44}N_3O_5$ req. 550.3281, fnd. 550.3284 (0.55ppm).

**Preparation of (3a*R*, 6a*S*)-*N*-[(1*S*)-1-(4-benzoyl-6,6-dimethoxyhexahydro pyrrolo[3,2-*b*]pyrrole-1-carbonyl)-3-methylbutyl]-4-dimethylaminobenzamide (135)**

4-Methylmorpholine (0.109 ml, 0.994 mmol) was added to a solution of HBTU (189 mg, 0.497 mmol), 1-hydroxybenzotriazole monohydrate (76 mg, 0.497 mmol) and 4-(dimethylamino)benzoic acid (82 mg, 0.497 mmol) in dimethylformamide (12.5 ml). The solution was stood for 5 minutes then added to (2*S*, 3a*R*, 6a*S*)-2-amino-1-(4-benzoyl-6,6-dimethoxyhexahydropyrrolo[3,2-*b*]pyrrol-1-yl)-4-methylpentan-1-one **(133)** (prepared as above, 0.497 mmol). The mixture was stirred at ambient temperature for 7 hours then the solvents removed *in vacuo* (water bath temperature < 26 °C) to obtain a volume of approximately 3 ml. Stirring was continued for 1.25 hours then the remaining solvent was

removed *in vacuo*. The residue was dissolved in dichloromethane (60 ml) then washed with pH 3 hydrochloric acid (40 ml), saturated aqueous sodium hydrogen carbonate solution (40 ml) and brine (40 ml), then dried ($Na_2SO_4$) and the solvents removed *in vacuo.* The residue was purified by flash chromatography over silica eluting with ethyl acetate : heptane mixtures 0 : 100 to 65 : 35 to give (3a*R*, 6a*S*)-*N*-[(1*S*)-1-(4-benzoyl-6,6-dimethoxyhexahydropyrrolo[3,2-*b*]pyrrole-1-carbonyl)-3-methylbutyl]-4-dimethylamino benzamide **(135)** as a white solid (180 mg, 68%). TLC (Single spot, $R_f$ = 0.30, EtOAc : heptane 9 : 1), analytical HPLC $R_t$ = 15.789min; HPLC-MS 537.2 [M + H]$^+$; Elemental analysis $C_{30}H_{40}N_4O_5$ .0.4EtOAc req.(fnd.) % C 66.41 (66.60), % H 7.62 (7.92), % N 9.80 (9.51); HRMS $C_{30}H_{40}N_4O_5Na$ req. 559.2896, fnd. 559.2902 (0.95ppm).

**Preparation of (3a*R*, 6a*S*)-*N*-[(1*S*)-1-(4-benzoyl-6-oxo-hexahydropyrrolo[3,2-*b*]pyrrole-1-carbonyl)-3-methylbutyl]-4-*tert*-butylbenzamide** (EXAMPLE 1).

(3a*R*, 6a*S*)-*N*-[(1*S*)-1-(4-Benzoyl-6,6-dimethoxyhexahydropyrrolo[3,2-*b*]pyrrole-1-carbonyl)-3-methyl butyl]-4-*tert*-butylbenzamide **(134)** (0.19 g, 0.345 mmol) was dissolved in ice-cooled trifluoroacetic acid / water (95 : 5 v/v, 10 ml) with stirring. The ice-bath was removed and the mixture stirred at ambient temperature for 3.5 hours. The mixture was then reduced *in vacuo* and evaporated from diethyl ether (2x 10 ml) to give a semi-mobile tan gum (0.3 g). Flash chromatography over silica, eluting with ethyl acetate : heptane mixtures gave (3a*R*, 6a*S*)-*N*-[(1*S*)-1-(4-benzoyl-6-oxo-hexahydropyrrolo[3,2-*b*]pyrrole-1-carbonyl)-3-methylbutyl]-4-*tert*-butyl benzamide (EXAMPLE 1) (0.042 g, 24%) as a white solid. TLC ($R_f$= 0.45, EtOAc : heptane 9 : 1), analytical HPLC single broad main peak, $R_t$ = 19.43-21.37 min., HPLC-MS 504.1 [M + H]$^+$; Elemental analysis $C_{30}H_{37}N_3O_4$.0.5TFA req.(fnd.) % C 66.45 (66.04), % H 6.75 (7.19), % N 7.50 (7.24); HRMS $C_{30}H_{37}N_3O_4Na$ req. 526.2682, fnd. 526.2677 (-0.96ppm); $d_H$ (500 MHz, $CDCl_3$) Tentative assignment of peaks due to presence of rotamers 0.95 (3H, d, *J* = 6.5 Hz, Leu δC$H_3$), 1.01 (3H, d, *J* = 6.2 Hz, Leu δC$H_3$), 1.31 (9H, s, C(C$H$)$_3$), 1.58-1.81 (3H, m, Leu βC$H_2$ and Leu γC*H*), 1.85-2.73 (2H, m, BzNCHC$H_2$), 3.55-3.69 (1H, m, BzNCHC$H_2$C$H_2$), 3.85-5.20 (6H, m, BzNC*H*C$H_2$C$H_2$, BzNC$H_2$C(=O)C*H* and Leu αC*H*), 6.70-6.89 (1H, m, N*H*), 7.40-7.52 (7H, m, COC$_6$$H_5$ and CHC*H*CC(C$H_3$)$_3$), 7.65-7.76 (2H, m, C*H*CHCC(C$H_3$)$_3$); $\delta_C$ (500 MHz, $CDCl_3$) 22.06, 23.28 (2x Leu δC$H_3$), 24.82 (Leu γCH), 31.11, 31.14 (C(CH$_3$)$_3$), 31.67, 31.86 (BzNCHCH$_2$), 34.89, 34.92 (C(CH$_3$)$_3$), 42.43 (Leu βCH$_2$), 46.10 (BzNCHCH$_2$CH$_2$), 48.93 (Leu αCH), 60.2 (BzNCH$_2$), 61.0 (BzNCH or BzNCH$_2$C(=O)CH), 68.2 (BzNCH or BzNCH$_2$C(=O)CH), 125.41, 125.49, 125.54, 126.91, 126.97, 127.11, 127.46, 128.33, 128.79, 130.84 (CH aromatics), 130.70, 131.16, 135.0 (quaternary aromatics), 155.35 (CC(CH$_3$)$_3$), 167.07, 170.67, 172.61 (3 x NC=O).

**Preparation of (3a*R*, 6a*S*)-*N*-[(1*S*)-1-(4-benzoyl-6-oxo-hexahydropyrrolo[3,2-*b*]pyrrole-1-carbonyl)-3-methylbutyl]-4-dimethylaminobenzamide** (EXAMPLE 14)

Water (1.75 ml) was added dropwise to a stirred solution of (3a*R*, 6a*S*)-*N*-[(1*S*)-1-(4-benzoyl-6,6-dimethoxyhexahydropyrrolo[3,2-*b*]pyrrole-1-carbonyl)-3-methylbutyl]-4-dimethylaminobenzamide **(135)** (175 mg, 0.327 mmol) in trifluoroacetic acid (17.5 ml) at 0 °C over 3 minutes. The solution was then stirred at ambient temperature for 17 hours then the solvents removed *in vacuo* (water bath < 25 °C). The residue was azeotroped with diethyl ether (25 ml) then the residue purified by flash chromatography over silica eluting with ethyl acetate : heptane mixtures 0 : 100 to 70 : 30 to give (3a*R*, 6a*S*)-*N*-[(1*S*)-1-(4-benzoyl-6-oxo-hexahydropyrrolo[3,2-*b*]pyrrole-1-carbonyl)-3-methylbutyl]-4-dimethylaminobenzamide (EXAMPLE 14) as a white solid (113.6 mg, 71%). TLC (Single spot, $R_f$ = 0.25, EtOAc : heptane 9 : 1), analytical HPLC $R_t$ = 14.241min; HPLC-MS 491.2 [M + H]$^+$; $C_{28}H_{34}N_4O_4$.0.5TFA req.(fnd.) % C 63.64 (63.07), % H 6.35 (6.76), % N 10.23 (9.91); HRMS $C_{28}H_{34}N_4O_4Na$ req. 513.2478, fnd. 513.2492 (2.72ppm); $d_H$ (500 MHz, $CDCl_3$) Tentative assignment of peaks due to presence of rotamers 0.94 (3H, d, *J* = 6.5 Hz, Leu δC$H_3$), 0.99 (3H, d, *J* = 6.2 Hz, Leu δC$H_3$), 1.46-1.83 (3H, m, Leu βC$H_2$ and Leu γC*H*), 1.90-2.70 (2H, m, BzNCHC$H_2$), 2.99 (6H, s, N(C$H_3$)$_2$), 3.45-3.69 (1H, m, BzNCHC$H_2$C$H_2$), 3.90-5.25 (6H, m, BzNC*H*C$H_2$C$H_2$, BzNC$H_2$C(=O)C*H* and Leu αC*H*), 6.58-6.75 (1H, m, N*H*), 6.60-6.68 (2H, m, C*H*CN(CH$_3$)$_2$), 7.35-7.55 (5H, m, COC$_6$$H_5$), 7.60-7.73 (2H, m, C*H*CHCN(CH$_3$)$_2$); $\delta_C$ (500 MHz, $CDCl_3$) 22.13, 23.25 (2x Leu δCH$_3$), 24.78 (Leu γCH), 31.65, 31.85 (BzNCHCH$_2$), 40.04, 40.09, 40.13 (N(CH$_3$)$_2$), 42.35 (Leu βCH$_2$), 46.12 (BzNCHCH$_2$CH$_2$), 48.79 (Leu αCH), 59.99 (BzNCH$_2$), 60.80 (BzNCH or BzNCH$_2$C(=O)CH), 67.7 (BzNCH or BzNCH$_2$C(=O)CH), 110.89, 111.00, 111.04, 111.35 (CHCN(CH$_3$)$_2$), 120.17 (CCHCHCN(CH$_3$)$_2$), 126.92, 127.12, 127.48, 128.31, 128.63, 128.67, 128.77, 129.00, 130.81, 130.96 (CH aromatics), 135.0 (quaternary aromatics), 152.61 (CN(CH$_3$)$_2$), 167.21, 170.69, 173.01 (3 x NC=O).

Alternatively, a useful building block for solution phase synthesis is (3a*R*, 6*S*, 6a*S*)-4-benzoyl-6-bydroxyhexahydropyrrolo[3,2-*b*]pyrrole-1-carboxylic acid benzyl ester **(128)** described earlier in Scheme 26. The utility of building block **(128)** is detailed in an alternative synthesis of EXAMPLE 1, through Scheme 27, which is an example of the general synthetic strategy detailed in Scheme 17.

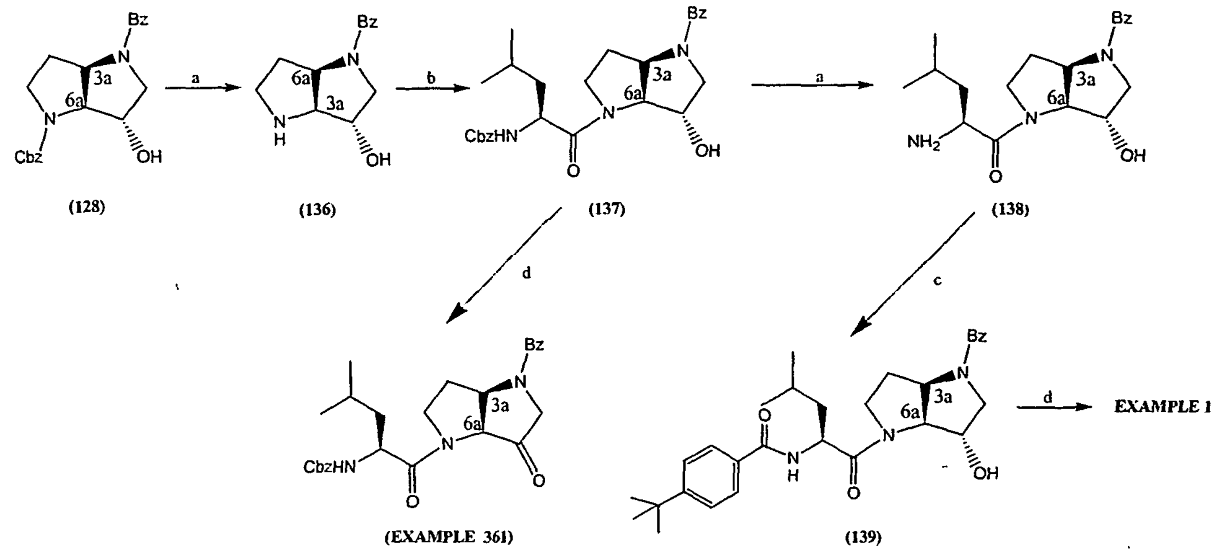


**Scheme 27.** (a) Pd-C / $H_2$, ethanol / methanol. (b) leq Cbz-Leu-F, DMF, RT. (c) 1.05eq 4-*tert*-butylbenzoic acid, HBTU, HOBT, NMM, DMF, RT (d) Dess-Martin periodinane, DCM.

**Preparation of (3*S*, 3a*S*, 6a*R*)-(3-hydroxyhexahydropyrrolo[3,2-*b*]pyrrol-1-yl)phenylmethanone (136).**

Ethanol (5 ml) was added cautiously dropwise to 10% palladium on charcoal (50 mg) at 0 °C under an atmosphere of argon over 10 minutes whilst stirring followed by a solution of (3a*R*, 6*S*, 6a*S*)-4-benzoyl-6-hydroxy hexahydropyrrolo[3,2-*b*]pyrrole-1-carboxylic acid benzyl ester **(128)** (465 mg, 1.27 mmol prepared as above) in ethanol (10 ml). The argon was then replaced by an atmosphere of hydrogen and stirring continued at ambient temperature for 4.5 hours. The hydrogen was then replaced by argon and 10% palladium on charcoal (20 mg) was added at 0 °C. The argon was then replaced with hydrogen and stirring was continued for 4 hours. The hydrogen was replaced by argon then the mixture was filtered through celite. The filter cake was washed with ethanol (75 ml) then the filtrate concentrated *in vacuo* to obtain (3*S*, 3a*S*, 6a*R*)-(3-hydroxyhexahydropyrrolo[3,2-*b*]pyrrol-1-yl)phenylmethanone **(136)** as a colourless oil (309 mg) which was used without further purification. HPLC-MS 233.1 $[M + H]^+$, 465.1 $[2M + H]^+$.

**Preparation of (3a*R*, 6a*S*)-(1*S*)-1-((6*S*)-4-benzoyl-6-hydroxyhexa hydropyrrolo[3,2-*b*]pyrrole-1-carbonyl)-3-methylbutyl]carbamic acid benzyl ester (137)**

Cbz-Leu-F (350 mg, 1.31 mmol) was dissolved in dimethylformamide (5 ml) then added to (3*S*, 3a*S*, 6a*R*)-(3-hydroxyhexahydropyrrolo[3,2-*b*]pyrrol-1-yl)phenyl methanone **(136)** (304 mg, 1.24 mmol, prepared as above) under an atmosphere of argon. The solution was stirred for 1.25 hours then the solvents removed *in vacuo*. The residue was purified by flash chromatography over silica eluting with ethyl acetate : heptane mixtures 0 : 100 to 80 : 20 to give (3a*R*, 6a*S*)-[(1*S*)-1-((6*S*)-4-benzoyl-6-hydroxyhexahydropyrrolo[3,2-*b*]pyrrole-1-carbonyl)-3-methylbutyl] carbamic acid benzyl ester **(137)** as a white solid (402 mg, 68%). TLC (Single spot, $R_f$ = 0.10, EtOAc : heptane 65 : 35), analytical HPLC $R_t$ = 16.803 min; HPLC-MS 480.2 $[M + H]^+$, 981.3 $[2M + Na]^+$; HRMS $C_{27}H_{33}N_3O_5Na$ req. 502.2318, fnd. 502.2311 (-1.44ppm).

**Alternative preparation of (3a*R*, 6a*S*)-[(1*S*)-1-((6*S*)-4-benzoyl-6-hydroxyhexa hydropyrrolo[3,2-*b*]pyrrole-1-carbonyl)-3-methylbutyl]carbamic acid benzyl ester (137)**

Dimethylformamide (1 ml) was added to a mixture of (3*S*, 3a*S*, 6a*R*)-(3-hydroxyhexahydropyrrolo[3,2-*b*]pyrrol-1-yl)phenylmethanone **(136)** (24 mg, 0.087 mmol, prepared as above) and Cbz-Leu-OSuc (32 mg, 0.088 mmol) under an atmosphere of argon. The solution was stirred for 20 hours then the solvents removed *in vacuo* to obtain a residue which was dissolved in dichloromethane (20 ml) then washed with water (10 ml), dried ($Na_2SO_4$) and the solvents removed *in vacuo*. The residue was purified by flash chromatography over silica eluting with ethyl acetate : heptane mixtures 30 : 70 to 80 : 20 to give (3a*R*, 6a*S*)-[(1*S*)-1-((6*S*)-4-benzoyl-6-hydroxyhexahydropyrrolo[3,2-*b*]pyrrole-1-carbonyl)-3-methyl butyl]carbamic acid benzyl ester **(137)** as a white solid (25 mg, 60%). TLC (Single spot, $R_f$ = 0.10, EtOAc : heptane 65 : 35), analytical HPLC $R_t$ = 17.301 min; HPLC-MS 480.2 $[M + H]^+$, 981.3 $[2M + Na]^+$; $C_{27}H_{33}N_3O_5$.0.2EtOAc req.(fnd.) %

C 67.20 (67.03), % H 7.02 (7.16), % N 8.45 (8.27); $d_H$ (500 MHz, $CDCl_3$) mixture of rotamers, *tentative assignment of proton* 1.2-2.4 (11H, m, 2x Leu $\delta CH_3$, Leu $\beta CH_2$, Leu $\gamma CH$, BzNCHC$H_2$), 3.3-4.0 (4H, m, BzNC$H_2$, CbzLeuNC$H_2$), 4.2-5.0 (4H, BzNC*H*, CbzLeuNC*H*, C*H*OH, Leu $\alpha CH$), 5.0-5.1 (2H, OC$H_2$Ph), 5.4 (1H, d, *J* = 8.3Hz, N*H*), 7.4-7.6 (10H, aromatic); $d_C$ (125 MHz, $CDCl_3$) 21.73, 21.89 and 23.22, 23.36 (2x Leu $\delta \underline{C}H_3$), 24.59, 24.67 (Leu $\gamma \underline{C}H$), 31.86 (BzNCH$\underline{C}H_2$), 42.02, 42.22 (Leu $\beta \underline{C}H_2$), 46.52 (CbzLeuN$\underline{C}H_2$), 50.94, 51.02 (Leu $\alpha \underline{C}H$), 56.58 (BzN$\underline{C}H_2$), 59.72 (BzN$\underline{C}$H), 67.00 (O$\underline{C}H_2$Ph), 67.98 (CbzLeuN$\underline{C}$H), 75.25 ($\underline{C}$HOH), 127.34, 128.02, 128.18, 128.28, 128.36, 128.52, 130.34 (aromatic $\underline{C}$H), 136.09, 136.18 (aromatic quaternary), 156.18 (NH$\underline{C}$=O), 170.08 (Ph$\underline{C}$=O), 172.32 ($CH_2N\underline{C}$=O).

**Preparation of (2*S*, 3a*R*, 6a*S*)-2-amino-1-((6S)-4-benzoyl-6-hydroxyhexahydro pyrrolo[3,2-*b*]pyrrol-1-yl)-4-methylpentan-1-one (138)**

Ethanol (15 ml) was added cautiously to a stirred mixture of (3a*R*, 6a*S*)-[(1*S*)-1-((6*S*)-4-benzoyl-6-hydroxyhexahydropyrrolo[3,2-*b*]pyrrole-1-carbonyl)-3-methylbutyl] carbamic acid benzyl ester **(137)** (370 mg, 0.77 mmol) and 10% palladium on charcoal (50 mg) at 0 °C under an atmosphere of argon. The argon was replaced by an atmosphere of hydrogen then stirring continued at ambient temperature for 1.75 hours. The hydrogen was replaced by argon then the mixture was cooled to 0 °C before adding a further portion of 10% palladium on charcoal (20 mg). The argon was replaced by hydrogen then stirring continued at ambient temperature for 5.25 hours. The hydrogen was replaced by argon then the mixture was cooled to 0 °C before adding a further portion of 10% palladium on charcoal (20 mg). The argon was replaced by hydrogen then stirring continued at ambient temperature for 14 hours. The hydrogen was replaced by argon then the mixture was cooled to 0 °C before adding a further portion of 10% palladium on charcoal (10 mg). The argon was replaced by hydrogen then stirring continued at ambient temperature for 2 hours. The hydrogen was replaced by argon then the mixture was diluted with ethanol (60 ml) and filtered through celite. The filter cake was washed with ethanol (40 ml) then the filtrate concentrated *in vacuo*. The residue was azeotroped with ethyl acetate (35 ml) to obtain (2*S*, 3a*R*, 6a*S*)-2-amino-1-((6*S*)-4-benzoyl-6-hydroxyhexahydropyrrolo[3,2-*b*]pyrrol-1-yl)-4-methylpentan-1-one **(138)** as an oily white solid (270 mg), which was used without further purification. HPLC-MS 346.2 $[M + H]^+$, 713.3 $[2M + Na]^+$.

**Preparation of (3a*R*, 6a*S*)-*N*-[(1*S*)-1-((6*S*)-4-benzoyl-6-hydroxyhexahydro-pyrrolo[3,2-*b*]pyrrole-1-carbonyl)-3-methylbutyl]-4-*tert*-butyl-benzamide (139)**

4-Methylmorpholine (0.17 ml, 1.55 mmol) was added to a solution of HBTU (293 mg, 0.77 mmol), 1-hydroxybenzotriazole monohydrate (118 mg, 0.77 mmol) and 4-(*tert*-butyl)benzoic acid (138 mg, 0.77 mmol) in dimethylformamide (7.5 ml). The solution was stood for 5 minutes then added to (2*S*, 3a*R*, 6a*S*)-2-amino-1-((6*S*)-4-benzoyl-6-hydroxyhexahydropyrrolo[3,2-*b*]pyrrol-1-yl)-4-methylpentan-1-one **(138)** (prepared as above, 0.77 mmol). The mixture was stirred at ambient temperature for 1 hour 5 minutes then the solvents removed *in vacuo* (water bath temperature < 28 °C). The residue was dissolved in dichloromethane (75 ml) then washed with pH 3 hydrochloric acid (60 ml), saturated aqueous sodium hydrogen carbonate solution (60 ml) and brine (60 ml), dried ($Na_2SO_4$) and the solvents removed *in vacuo*. The residue (512 mg) was purified by flash chromatography over silica eluting with ethyl acetate : heptane mixtures 0 : 100 to 85 : 15 to give (3a*R*, 6a*S*)-*N*-[(1*S*-1-((6*S*)-4-benzoyl-6-hydroxyhexahydro-pyrrolo[3,2-*b*]pyrrole-1-carbonyl)-3-methylbutyl]-4-*tert*-butyl-benzamide **(139)** as a white solid (263 mg, 68%). TLC (Single spot, $R_f$ = 0.15, EtOAc : heptane 9 : 1), analytical HPLC $R_t$ = 19.340 min; HPLC-MS 506.2$[M + H]^+$; $C_{30}H_{39}N_3O_4$.0.5EtOAc req.(fnd.) % C 69.97 (69.86), % H 7.89 (7.87), % N 7.65 (7.88); HRMS $C_{30}H_{39}N_3O_4Na$ req. 528.2838, fnd. 528.2818 (-3.89ppm).

**Preparation of (3a*R*, 6a*S*)-*N*-[(1*S*)-1-(4-benzoyl-6-oxo-hexahydropyrrolo[3,2-*b*] pyrrole-1-carbonyl)-3-methylbutyl]-4-*tert*-butylbenzamide** (EXAMPLE 1)

A solution of (3aR, 6a*S*)-*N*-[(1*S*)-1-((6*S*)-4-benzoyl-6-hydroxyhexahydro-pyrrolo[3,2-*b*]pyrrole-1-carbonyl)-3-methylbutyl]-4-*tert*-butyl-benzamide (139) (174 mg, 0.345 mmol) in dichloromethane (10 ml) was added to Dess-Martin periodinane (292 mg, 0.689 mmol) under an atmosphere of argon whilst stirring over 2.5 minutes. The mixture was stirred for 3 minutes then trifluoroacetic acid (53 μl, 0.689 mmol) was added. The mixture was stirred for 14 hours then solvents removed *in vacuo*. The residue was purified by flash chromatography over silica eluting with ethyl acetate : heptane mixtures 0 : 100 to 55 : 45 to give (3a*R*, 6a*S*)-*N*-[(1*S*)-1-(4-benzoyl-6-oxo-hexahydropyrrolo[3,2-*b*]pyrrole-1-carbonyl)-3-methylbutyl]-4-*tert*-butylbenzamide (EXAMPLE 1) as a white solid (128 mg, 74%). TLC (Single spot, $R_f$= 0.20, EtOAc : heptane 9 : 1), analytical HPLC broad peak $R_t$ = 19.2-20.6 min; HPLC-MS single broad UV peak, 504.1 $[M + H]^+$.

**Preparation of (3a*R*, 6a*S*)-[(1*S*)-1-(4-benzoyl-6-oxo-hexahydropyrrolo[3,2-*b*] pyrrole-1-carbonyl)-3-methyl-butyl]carbamic acid benzyl ester** (EXAMPLE 361)

A solution of (3a*R*, 6a*S*)-[(1*S*)-1-((6*S*)-4-benzoyl-6-hydroxyhexa hydropyrrolo[3,2-*b*]pyrrole-1-carbonyl)-3-methylbutyl]carbamic acid benzyl ester (137) (15.0 mg, 0.031 mmol) in dichloromethane (0.75 ml) was added dropwise to Dess-Martin periodinane (26.6 mg, 0.063 mmol) under an atmosphere of argon whilst stirring over 1 minute. The solution was stirred for 4.5 hours then purified by flash chromatography over silica eluting with ethyl acetate : heptane mixtures 0 : 100 to 80 : 20 to give (3a*R*, 6a*S*)-[(1*S*)-1-(4-benzoyl-6-oxo-hexahydropyrrolo[3,2-*b*]pyrrole-1-carbonyl)-3-methylbutyl]carbamic acid benzyl ester (EXAMPLE 361) as a white solid (8.8 mg, 58%). TLC (Single spot, $R_f$= 0.35, EtOAc : heptane 9 : 1), analytical HPLC broad peak $R_t$ = 17.7-19.5 min; HPLC-MS single broad UV peak, 478.1 $[M + H]^+$, 977.2 $[2M + Na]^+$; HRMS $C_{27}H_{31}N_3O_5Na$ req. 500.2161, fnd. 500.2168 (1.26ppm).

Alternatively, the general synthetic strategy detailed in Scheme 28 involves construction of an extended compound prior to intramolecular ring closure to the 5,5-cis bicycle as the penultimate step. As detailed in Scheme 20, the building block (*R*)-2-(2-azidoethyl)-2,5-dihydropyrrole-1-carboxylic acid *tert*-butyl ester **(76)** may be reduced to the primary amine, which in Scheme 28 is directly acylated with a protected aminoacid. Following epoxidation, then conversion of the aminoacid protecting group to a suitable capping group, with all of the potency and specificity components now in place, ring-closure and oxidation provides the final inhibitor compound.The utility of such synthetic flexibility is detailed in an alternativesynthesis of EXAMPLE 1, through Scheme 28.

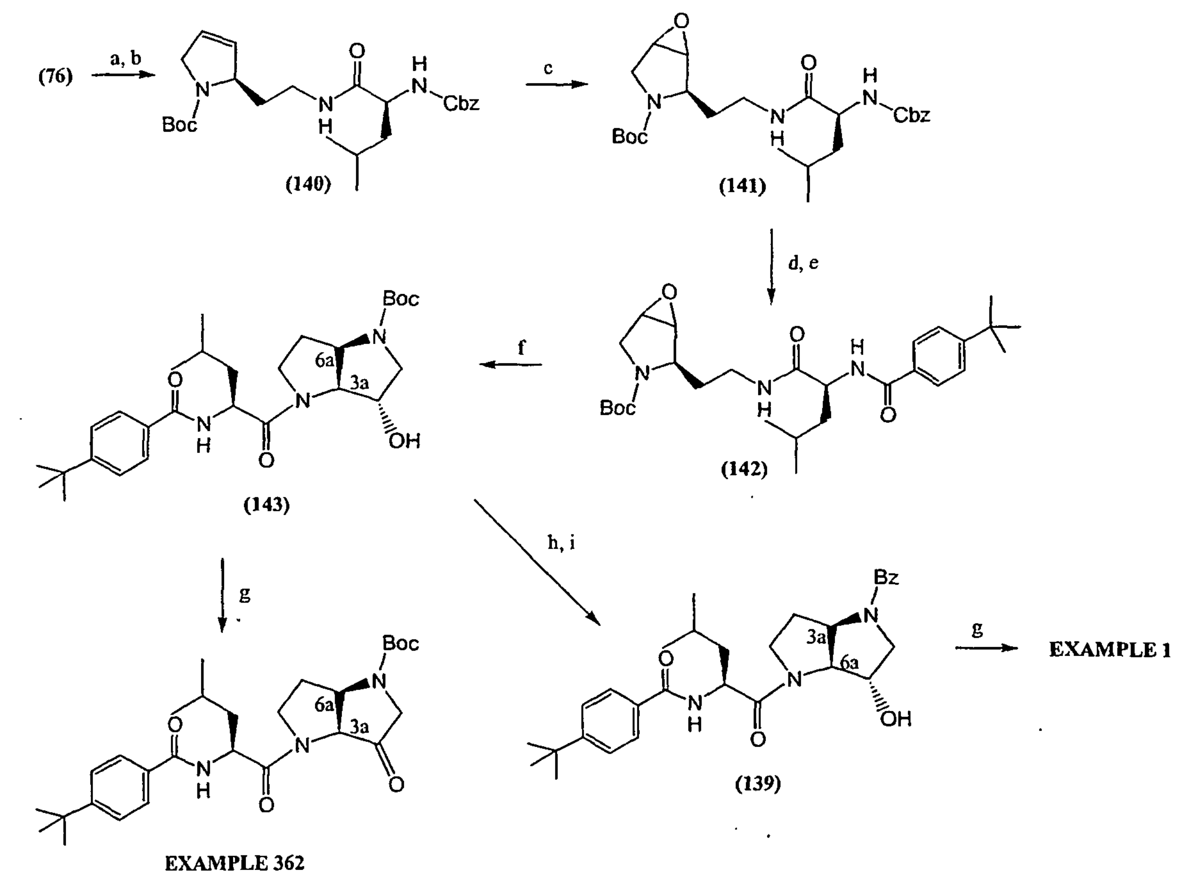


**Scheme 28.** (a) $Ph_3P$, $H_2O$, 1,4-dioxane. (b) 1eq Cbz-Leu-OSu, 2.1eq $Na_2CO_3$, 1,4-dioxane, water. (c) *m*-Chloroperoxybenzoic acid, DCM. (d) Pd-C, $H_2$, ethanol. (e) 1.05eq 4-*tert*-butylbenzoic acid, HBTU, HOBT, NMM, DMF, RT. (f) 2eq NaH, THF, RT, 16 h. (g) Dess-Martin periodinane, DCM. (h) 4N HCl in 1,4-dioxane, RT, 30mins. (i) Benzoic anhydride, 4-methylmorpholine, DMF, RT, 1hr.

**Preparation of (2*R*)-2-[2-((2*S*)-2-benzyloxycarbonylamino-4-methylpentanoyl amino)ethyl]-2,5-dihydro pyrrole-1-carboxylic acid *tert*-butyl ester (140)**

(*R*)-2-(2-Aminoethyl)-2,5-dihydropyrrole-1-carboxylic acid *tert*-butyl ester (see preparation of **(77)** above, ~ 0.63 mmol) was dissolved in 1,4-dioxane (10 ml) with stirring, ice-cooled and a solution of sodium carbonate (0.14 g, 1.32 mmol) in water (10 ml) was added. Cbz-L-Leu-OSu (0.251 g, 0.693 mmol) in 1,4-dioxane (10 ml) was added dropwise over 30 minutes, then the ice bath removed and the mixture stirred for a further 30 minutes. Water (100 ml) was then added and the aqueous phase extracted with dichloromethane (2x 100 ml). The combined organic layers were dried ($Na_2SO_4$), filtered and reduced *in vacuo* to leave a clear gum (0.54 g). Flash chromatography over silica, eluting with ethyl acetate : heptane mixtures gave (2*R*)-2-[2-((2*S*)-2-benzyloxycarbonylamino-4-methylpentanoylamino)ethyl]-2,5-dihydro pyrrole-1-carboxylic acid *tert*-butyl ester **(140)** (0.21 g, 72%) as a clear oil. TLC ($R_f$= 0.30, EtOAc : heptane 1 : 1), analytical HPLC single main peak, $R_t$ = 20.326 min., HPLC-MS 360.1 $[M + 2H - Boc]^+$, 404.1 $[M + 2H - Bu]^+$, 460.2 $[M + H]^+$, 482.1 $[M + Na]^+$, 941.2 $[2M + Na]^+$; Elemental analysis $C_{25}H_{37}N_3O_5$ req.(fnd.) % C 65.34 (65.14), % H 8.11 (8.19), % N 9.14 (9.07); HRMS $C_{25}H_{37}N_3O_5Na$ req. 482.2631, fnd. 482.2620 (-2.33ppm).

**Preparation of (2*R*)-2-[2-((2*S*)-2-benzyloxycarbonylamino-4-methylpentanoyl amino)ethyl]-6-oxa-3-azabicyclo[3.1.0]hexane-3-carboxyfic acid *tert*-butyl ester (141)**

(2*R*)-2-[2-((2*S*)-2-Benzyloxycarbonylamino-4-methylpentanoylamino)ethyl]-2,5-di hydropyrrole-1-carboxylic acid *tert*-butyl ester **(140)** (0.20 g, 0.435 mmol) was dissolved in dichloromethane (5 ml) with stirring then *meta*-chloroperoxybenzoic acid (65% reagent, 1.15 g, 4.35 mmol) added. The mixture was stirred at ambient temperature under argon for 16 hours. Dichloromethane (100 ml) was added and the organic phase washed with 10% w/v aqueous sodium hydroxide solution (2x 100 ml), then dried ($Na_2SO_4$), filtered and reduced *in vacuo* to leave an oily solid (0.19 g). Flash chromatography over silica, eluting with ethyl acetate : heptane mixtures gave (2*R*)-2-[2-((2*S*)-2-benzyloxycarbonylamino-4-methylpentanoyl amino)ethyl]-6-oxa-3-azabicyclo[3.1.0]hexane-3-carboxlic acid *tert*-butyl ester **(141)** (0.19 g, 92%) as an opaque gum. TLC ($R_f$= 0.35 (major) and 0.42 (minor) (mixture of *anti* and *syn* epoxides), EtOAc : heptane 3 : 1), analytical HPLC single main peak, $R_t$ = 19.21 min., HPLC-MS 376.1 $[M + 2H - Boc]^+$, 420.1 $[M + 2H - Bu]^+$, 476.1 $[M + H]^+$, 498.1 $[M + Na]^+$, 973.2 $[2M + Na]^+$; Elemental analysis $C_{25}H_{37}N_3O_6$ req.(fnd.) % C 63.14 (63.11), % H 7.84 (7.96), % N 8.84 (8.80); HRMS $C_{25}H_{37}N_3O_6Na$ req. 498.2580, fnd. 498.2602 (4.34ppm).

**Preparation of (2*R*)-2-{2-[(2*S*)-2-(4-*tert*-butylbenzoylamino)-4-methyl pentanoylamino]ethyl}-6-oxa-3-aza-bicyclo[3.1.0]hexane-3-carboxylic acid *tert*-butyl ester (142)**

(i) (2*R*)-2-[2-((2*S*)-2-Benzyloxycarbonylamino-4-methylpentanoylamino)ethyl]-6-oxa-3-azabicyclo [3.1.0]hexane-3-carboxlic acid *tert*-butyl ester **(141)** (0.17 g, 0.357 mmol) was dissolved in ethanol (5 ml), cooled to 0 °C and 10% palladium on charcoal (0.034 g) added. The mixture was stirred, then evacuated and flushed with hydrogen. The mixture was allowed to warm to ambient temperature, stirred for 45 minutes then filtered through celite. The filter cake was washed with ethanol (3x 10 ml) and the combined organic layers reduced *in vacuo* to provide the crude free amine, which was used without further purification. HPLC-MS 342.2 $[M + H]^+$, 683.3 $[2M + H]^+$, 705.3 $[2M + Na]^+$.

(ii) The crude free amine was dissolved in anhydrous dimethylformamide (3 ml) with stirring and 2-(1*H*-benzotriazole-1-yl)-1,1,3,3-tetramethyluroniumhexafluoro phosphate (HBTU, 0.136 g, 0.357 mmol) and 1-hydroxybenzotriazole monohydrate (HOBT, 0.0548 g, 0.357 mmol) added. 4-Methylmorpholine (78.6 μl, 0.715 mmol) was added and the mixture stirred for 1.5 hours, then reduced *in vacuo*. The residue was dissolved in dichloromethane (50 ml) and washed with pH 3 hydrochloric acid (50 ml), saturated sodium hydrogen carbonate solution (50 ml) and brine (50 ml). The organic phase was dried ($Na_2SO_4$), filtered and reduced *in vacuo* to leave a pale yellow gum (0.19 g). Flash chromatography over silica, eluting with ethyl acetate : heptane mixtures gave (2*R*)-2-{2-[(2*S*)-2-(4-*tert*-butylbenzoylamino)-4-methylpentanoylamino]ethyl}-6-oxa-3-aza-bicyclo[3.1.0] hexane-3-carboxylic acid *tert*-butyl ester **(142)** (0.08 g, 45%) as a white crystalline solid. TLC ($R_f$ = 0.26, EtOAc : heptane 3 : 1), analytical HPLC single main peak, $R_t$ = 21.195 min., HPLC-MS 446.2 $[M + 2H - Bu]^+$, 502.3 $[M + H]^+$, 524.2 $[M + Na]^+$; Elemental analysis $C_{28}H_{43}N_3O_5$ req.(fnd.) % C 67.04 (67.07), % H 8.64 (8.96), % N 8.38 (7.87); HRMS $C_{28}H_{43}N_3O_5Na$ req. 524.3100, fnd. 524.3086 (-2.81ppm).

**Preparation of (3*S*, 3a*S*, 6a*R*)-4-[(2*S*)-2-(4-*tert*-butylbenzoylamino)-4-methylpentanoyl]-3-hydroxyhexahydro-pyrrolo[3,2-*b*]pyrrole-1-carboxylic acid *tert*-butyl ester (143)**

(2*R*)-2-{2-[(2*S*)-2-(4-*tert*-Butylbenzoylamino)-4-methylpentanoylamino]ethyl}-6-oxa -3-aza-bicyclo [3.1.0]hexane-3-carboxylic acid *tert*-butyl ester **(142)** (0.06 g, 0.12 mmol) was dissolved in tetrahydrofuran (3 ml) with stirring under nitrogen and ice-cooled. Sodium hydride (60% dispersion in oil, 0.010 g, 0.25 mmol) was added over 1 minute and the mixture stirred at ambient temperature for 16 hours. Water (10 ml) was added, then saturated aqueous ammonium chloride solution (5 ml) and the product extracted into ethyl acetate (2x 25 ml). The combined organic layers were dried ($Na_2SO_4$), filtered and reduced *in vacuo* to leave a clear film (0.06 g). Flash chromatography over silica, eluting with ethyl acetate : heptane mixtures gave (3*S*, 3a*S*, 6a*R*)-4-[(2*S*)-2-(4-*tert*-butylbenzoylamino)-4-methylpentanoyl]-3-hydroxyhexa hydropyrrolo[3,2-*b*]pyrrole-1-carboxylic acid *tert*-butyl ester **(143)** (0.021 g, 35%) as a white solid. TLC ($R_f$ = 0.40, EtOAc : heptane 2 : 1), HPLC-MS 502.3 $[M + H]^+$, 524.2 $[M + Na]^+$.

A second product fraction contaminated by starting epoxide (~ 25% by UV analysis) was obtained as a white solid (0.0239 g).

**Preparation of (3a*S*, 6a*R*)-4-[(2*S*)-2-(4-*tert*-butylbenzoylamino)-4-methyl pentanoyl]-3-oxo-hexahydropyrrolo[3,2-*b*]pyrrole-1-carboxylic acid *tert*-butyl ester (EXAMPLE 362)**

(3*S*, 3a*S*, 6a*R*)-4-[(2*S*)-2-(4-*tert*-Butylbenzoylamino)-4-methylpentanoyl]-3-hydroxy hexahydropyrrolo[3,2-*b*]pyrrole-1-carboxylic acid *tert*-butyl ester **(143)** (0.021 g, 0.042 mmol) was dissolved in dichloromethane (2 ml) with stirring under argon. Dess-Martin periodinane (0.0373 g, 0.088 mmol) was added and the mixture stirred for 16 hours. The mixture was reduced *in vacuo* and the residue purified by flash chromatography over silica, eluting with ethyl acetate : heptane mixtures to give (3a*S*, 6a*R*)-4-[(2*S*)-2-(4-*tert*-butylbenzoylamino)-4-methylpentanoyl]-3-oxo-hexahydro pyrrolo[3,2-*b*]pyrrole-1-carboxylic acid *tert*-butyl ester (EXAMPLE 362) (0.015 g, 71%) as an off white gum. TLC ($R_f$= 0.53, EtOAc : heptane 2 : 1), analytical HPLC single broad main peak, $R_t$ = 20.6-22.5 min., HPLC-MS 500.1 $[M + H]^+$. HRMS $C_{28}H_{41}N_3O_5Na$ req. 522.2944, find. 522.2952 (1.49ppm); $\delta_H$ (500 MHz, $CDCl_3$) 0.85-1.03 (7H, m, 2x Leu $\delta CH_3$ + Leu $\gamma CH$), 1.32 (9H, s, $(CH_3)_3CPh$), 1.47 (9H, s, $(CH_3)_3COCO$), 1.6-1.8 (2H, m, Leu $\beta CH_2$), 2.03-2.15 / 2.33-2.45 (2H, b, BocNCHC$H_2$), 3.50-3.60 (1H, m, LeuNC$H_2$), 3.75-3.82 (1H, m, BocNC$H_2$), 3.93-4.02 (1H, m, BocNC$H_2$), 4.02-4.08 (1H, m, LeuNC$H_2$), 4.58-4.80 (1H, b, BocNC*H* or LeuNC*H*), 4.96-4.98 (1H, b, BocNC*H* or LeuNCH), 5.0-5.06 / 5.25-5.30 (1H, bm, Leu $\alpha CH$), 6.83-6.93 (1H, b, LeuN*H*), 7.42-7.45 (2H, d, *J* = 8.5 Hz, $(CH_3)_3C$-C-C*H*=CH), 7.72-7.75 (2H, d, *J* = 8.5 Hz, $(CH_3)_3C$-C-CH=C*H*); $\delta_C$ (125 MHz, $CDCl_3$) 22.27, 23.48 (2x Leu $\delta \underline{C}H_3$), 24.78 (Leu $\gamma \underline{C}H$), 28.32 ($(\underline{C}H_3)_3COCO$), 29.64, 31.82 (BocNCH$\underline{C}H_2$), 31.09 ($(\underline{C}H_3)_3CPh$), 34.88 ($(CH_3)_3\underline{C}Ph$), 42.50 (Leu $\beta \underline{C}H_2$), 45.95 (LeuN$\underline{C}H_2$), 49.09, 49.67 (Leu $\alpha \underline{C}H$), 52.37 (BocN$\underline{C}H_2$), 56.82 (BocN$\underline{C}$H), 62.92 (LeuN$\underline{C}$H), 81.16 ($(CH_3)_3\underline{C}OCO$), 125.34, 125.44 ($(CH_3)_3\underline{C}$-C-$\underline{C}$H=CH), 126.89, 126.95 ($(CH_3)_3C$-$\underline{C}$-CH=$\underline{C}$H), 130.83 (quaternary aromatics), 155.26 ($(CH_3)_3COC\underline{O}$), 167.01 $(CH_3)_3CPh\underline{C}O$), 172.28 ((Leu $\underline{C}$=O).

**Preparation of (3a*R*, 6a*S*)-*N*-[(1*S*)-1-((6*S*)-4-benzoyl-6-hydroxyhexahydro pyrrolo[3,2-*b*]pyrrole-1-carbonyl)-3-methylbuty]-4-*tert*-butylbenzamide (139)**

(i) (3*S*, 3a*S*, 6a*R*)-4-[(2*S*)-2-(4-*tert*-Butylbenzoylamino)-4-methylpentanoyl]-3-hydroxyhexahydro pyrrolo[3,2-*b*]pyrrole-1-carboxylic acid *tert*-butyl ester **(143)** (0.023 g, 0.046 mmol) was dissolved in 4.0M HCl in 1,4-dioxane (2 ml, 8 mmol) with stirring. After 45 minutes the solvents were removed *in vacuo* and the residue triturated then evaporated from diethyl ether (3x 3 ml) to leave (3a*R*, 6a*S*)-4-*tert*-butyl-*N*-[(1*S*)-1-((6*S*)-6-hydroxyhexahydropyrrolo[3,2-*b*]pyrrole-1-carbonyl)-3-methylbutyl]benzamide hydrochloride as a white solid which was used without further purification; HPLC-MS 402.2 $[M + H]^+$, 424.2 $[M + Na]^+$, 803.4 $[2M + H]^+$, 825.4 $[2M + Na]^+$.

(ii) (3a*R*, 6a*S*)-4-*tert*-Butyl-*N*-[(1*S*)-1-((6*S*)-6-hydroxyhexahydropyrrolo[3,2-*b*] pyrrole-1-carbonyl)-3-methylbutyl]benzamide hydrochloride (prepared as above, ~0.048 mmol) was dissolved in dimethylformamide (2 ml) with stirring, then benzoic anhydride (0.0114g, 0.05 mmol) added followed by 4-methylmorpholine (11.1μl, 0.0102 g, 0.101 mmol). After 1 hour, ethyl acetate (25 ml) was added and the organics washed with saturated aqueous sodium hydrogen carbonate solution (25 ml) solution, pH 3 hydrochloric acid (25 ml), and brine (25 ml). The organics were dried ($Na_2SO_4$), filtered and reduced *in vacuo* to a colourless film **(3a*R*,** 6a*S*)-*N*-[(1*S*)-1-((6*S*)-4-benzoyl-6-hydroxyhexahydropyrrolo[3,2-*b*]pyrrole-1-carbonyl)-3-methylbuty]-4-*tert*-butylbenzamide **(139)** (0.028 g). TLC ($R_f$ = 0.16, EtOAc : heptane 4 : 1), analytical HPLC single broad main peak, $R_t$ = 19.15 min., HPLC-MS 506.2 $[M + H]^+$, 528.2 $[M + Na]^+$.

Oxidation to EXAMPLE 1 is as detailed in Scheme 27.

EXAMPLE A. Assays for Cysteine Protease Activity

The compounds of this invention may be tested in one of a number of literature based biochemical assays that are designed to elucidate the characteristics of compound inhibition. The data from these types of assays enables compound potency and the rates of reaction to be measured and quantified. This information, either alone or in combination with other information, would allow the amount of compound required to produce a given pharmacological effect to be determined.

General materials and methods

Unless otherwise stated, all general chemicals and biochemicals were purchased from either the Sigma Chemical Company, Poole, Dorset, U.K. or from Fisher Scientific UK, Loughborough, Leicestershire, U.K. Absorbance assays were carried out in flat-bottomed 96-well plates (Spectra; Greiner Bio-One Ltd., Stonehouse, Gloucestershire, U.K.) using a SpectraMax PLUS384 plate reader (Molecular Devices, Crawley, U.K.). Fluorescence high throughput assays were carried out in either 384-well microtitre plates (Coming Costar 3705 plates, Fisher Scientific) or 96-well 'U' bottomed Microfluor W1 microtitre plates (Thermo Labsystems, Ashford, Middlesex, U.K.). Fluorescence assays were monitored using a SpectraMax Gemini fluorescence plate reader (Molecular Devices). For substrates employing either a 7-amino-4-methylcoumarin (AMC) or a 7-amino-4-trifluoromethylcoumarin (AFC) fluorophore, assays were monitored at an excitation wavelength of 365 nm and an emission wavelength of 450 nm and the fluorescence plate reader calibrated with AMC. For substrates employing a 3-amino-benzoyl (Abz) fluorophore, assays were monitored at an excitation wavelength of 310 nm and an emission wavelength of 445 nm; the fluorescence plate reader calibrated with 3-amino-benzamide (Fluka). Unless otherwise indicated, all the peptidase substrates were purchased from Bachem UK, St. Helens, Merseyside, UK. Substrates utilizing fluorescence resonance energy transfer methodology (i.e. FRET-based substrates) were synthesized at Incenta Limited using published methods (Atherton & Sheppard, *Solid Phase Peptide Synthesis*, IRL Press, Oxford, U.K., **1989**) and employed Abz (2-aminobenzoyl) as the fluorescence donor and 3-nitro-tyrosine [Tyr($NO_2$)] as the fluorescence quencher (Meldal, M. and Breddam, K., *Anal. Biochem.,* 195, 141-147, **1991**). Hydroxyethylpiperazine ethanesulfonate (HEPES), tris-hydroxylmethyl aminomethane (tris) base, bis-tris-propane and all the biological detergents (*e.g.* CHAPS, zwittergents, *etc.*) were purchased from CN Biosciences UK, Beeston, Nottinghamshire, U.K. Glycerol was purchased from Amersham Pharmacia Biotech, Little Chalfont, Buckinghamshire, U.K. Stock solutions of substrate or inhibitor were made up to 10 mM in 100 % dimethylsulfoxide (DMSO) (Rathburns, Glasgow, U.K.) and diluted as appropriately required. In all cases the DMSO concentration in the assays was maintained at less than 1% (vol./vol.).
Assay protocols were based on literature precedent (Table 4; Barrett, A.J., Rawlings, N.D. and Woessner, J.F., 1998, Handbook of Proteolytic Enzymes, Academic Press, London and references therein) and modified as required to suit local assay protocols. Enzyme was added as required to initiate the reaction and the activity, as judged by the change in fluorescence upon conversion of substrate to product, was monitored over time. All assays were carried out at 25±1°C.

**Table 4.** *The enzyme assays described herein were carried out according to literature precedents*.

| Enzyme | Buffer | Substrate | Reference |
|---|---|---|---|
| Cathepsin B | I | Z-Phe-Arg-AMC | a, b |
| Cathepsin H | II | Bz-Phe-Val-Arg-AMC | a, b |
| Cathepsin L | I | Ac-Phe-Arg-AMC | b, c |
| Cathepsin S | I | Boc-Val-Leu-Lys-AMC | c,d |
| Caspase 1 | III | Ac-Leu-Glu-His-Asp-AMC | e |
| Caspase 2 | III | Z-Val-Asp-Val-Ala-Asp-AFC | f |
| Caspase 3 | III | Ac-Asp-Glu-Val-Asp-AMC | g, h |
| Caspase 4 | III | Suc-Tyr-Val-Ala-Asp-AMC | f |
| Caspase 5 | III | Ac-Leu-Glu-His-Asp-AMC | |
| Caspase 6 | III | Ac-Val-Glu-Ile-Asp-AMC | i,j, k |
| Caspase 7 | III | Ac-Asp-Glu-Val- Asp-AMC | |
| Caspase 8 | III | Ac-Ile-Glu-Thr-Asp-AMC | l |
| Caspase 9 | III | Ac-Leu-Glu-His-Asp-AMC | |
| Caspase 10 | III | Ac-Ile-Glu-Thr-Asp-AMC | |

(continued)

| Enzyme | Buffer | Substrate | Reference |
|---|---|---|---|
| Cruzipain | IV | D-Val-Leu-Lys-AMC | m, n |
| CPB2.8$\Delta$CTE | XI | Pro-Phe-Arg-AMC | q |
| *S. Aureus* Extracellular cysteine peptidase | I | Abz-Ile-Ala-Ala-Pro-Tyr($NO_2$)-Glu-$NH_2$ | o |
| Clostripain | | Z-Gly-Gly-Arg-AMC | p |
| FMDV LP | V | Abz-Arg-Lys-Leu-Lys-Gly-Ala-Gly-Ser-Tyr($NO_2$)-Glu-$NH_2$ | r |
| Trypsin | VI | Z-Gly-Gly-Arg-AMC | s |
| Calpain $\mu$ | VII | Abz-Ala-Asn-Leu-Gly-Arg-Pro-Ala-Leu-Tyr($NO_2$)-Asp-$NH_2$ | t |
| Calpain m | VIII | Abz-Lys-Leu-Cys(Bzl)-Phe-Ser-Lys-Gln-Tyr($NO_2$)-Asp-$NH_2$ | t |
| Cathepsin K | IX | Z-Phe-Arg-AMC | u |
| Cathepsin X | X | | v,w |

I: 10 mM BTP, pH 6.5 containing 1 mM EDTA, 5 mM 2-mercaptoethanol and 1 mM $CaCl_2$
II: 10 mM BTP, pH 6.5 containing 1 mM EDTA, 142 mM NaCl, 1 mM DTT, 1 mM $CaCl_2$, 0.035 mM Zwittergent 3-16
III: 50mM HEPES pH 7.2, 10% Glycerol, 0.1% CHAPS, 142 mM NaCl, 1 mM EDTA, 5 mM DTT
IV: 100 mM sodium phosphate, pH 6.75 containing 1 mM EDTA and 10 mM L-cysteine
V: 50 mM tris·acetate, pH 8.4 containing 1 mM EDTA, 10 mM L-cysteine and 0.25% (w/v) CHAPS
VI: 10 mM HEPES, pH 8.0 containing 5 mM $CaCl_2$
VII: 10 mM HEPES, pH 7.5 containing 2 mM 2-mercaptoethanol and 100 $\mu$M $CaCl_2$
VIII: 10 mM HEPES, pH 7.5 containing 2 mM 2-mercaptoethanol and 200 $\mu$M $CaCl_2$
IX: 100 mM sodium acetate; pH 5.5 containing 10 mM L-cysteine and 1 mM EDTA
X: 100 mM sodium acetate; pH 5.5 containing 10 mM L-cysteine; 0.05% (w/v) Brij 35 and 1 mM EDTA
XI: 100 mM sodium acetate; pH 5.5 containing 10 mM L-cysteine; 142 mM sodium chloride and 1 mM EDTA

[a] Barrett, A.J., Biochem. J., 187, 909-912, 1980
[b] Barrett, A.J. and Kirschke, H., Methods Enzymol., 80, 535-561, 1981
[c] Quibell, M. and Taylor, S., WO0069855, 2000
[d] Bromme, D., Steinert, ., Freibe, S., Fittkau, S., Wiederanders, B., and Kirschke, H., Biochem. J., 264, 475-481, 1989
[e] Rano, T.A., et. al., Chem. Biol., 4, 149, 1997
[f] Talanian, R.V., et. al., J. Biol. Chem., 272, 9677, 1997
[g] Lazebnik, Y.A., Kaufmann, S.H., Desnoyers, S., Poirer, G.G. and Earnshaw, W.C., Nature., 371, 768-774, 1994
[h] Han, Z., et. al., J. Biol. Chem., 272, 13432,1997
[i] Takahashi, A., et. al., PNAS, 93, 8395, 1996
[j] Martins, L.M., et. al., J. Biol. Chem., 272, 7421,1997
[k] Nagata, S., Cell., 88, 355, 1997
[l] Harries, J.L., et. al., J. Biol. Chem., 273, 27364, 1998
[m] Cazzulo, J.J., Cazzulo Franke, M.C., Martinez, J. and Franke de Cazzulo, B.M., Biochim. Biophys. Acta., 1037, 186-191, 1990
[n] Cazzulo, J.J., Bravo, M., Raimondi, A., Engstrom, U., Lindeberg, G. and Hellman, U., Cell Mol. Biol., 42, 691-696, 1996
[o] Potempa, J., Dubin, A., Korzus, G. and Travis, J., Biochem. J., 263, 2664-2667, 1988
[p] Kembhavi, A.A., Buttle, D.J., Rauber, P. and Barrett, A.J., FEBS Lett., 283, 277-280,1991
[q] Alves, L.C., et. al., Mol. Biochem. Parasitol, 116, 1-9, 2001.
[r] Guamé, et.al., J. Mol. Biol., 302, 1227-1240, 2000.
[s] Halfon and Craik, (Barret, Rawlings and Woessner, eds.), in Handbook of Proteolytic Enzymes, Academic Press, London, 12-21, 1998.
[t] Sasaki, et. al., (1984), J. Biol. Chem., 259, 12489-12494, 1984.
[u] Bossard, M.J., et. al., , J. Biol. Chem., 21, 12517-12524, 1996
[v] Santamaria, I., et. al., J. Biol. Chem., 273, 16816-16823, 1998
[w] Klemencic, J, et al., Eur.J.Biochem., 267,5404-5412, 2000

*Trypanosoma cruzi* cruzipain peptidase activity assays

Wild-type cruzipain, derived from *Trypanosoma cruzi* Dm28 epimastigotes, was obtained from Dr. Julio Scharfstein (Instituto de Biofisica Carlos Chagas Filho, Universidade Federal do Rio de Janeiro, Rio de Janeiro, Brazil). Activity assays were carried out in 100 mM sodium phosphate, pH 6.75 containing 1 mM EDTA and 10 mM L-cysteine using 2.5 nM enzyme. Ac-Phe-Arg-AMC ($K_M^{app}$ ~ 12 μM) and D-Val-Leu-Lys-AMC ($K_M^{app}$ ~ 4 μM) were used as the substrates. Routinely, Ac-FR-AMC was used at a concentration equivalent to $K_M^{app}$ and D-Val-Leu-Lys-AMC was used at a concentration of 25 μM. The rate of conversion of substrate to product was derived from the slope of the increase in fluorescence monitored continuously over time.

*Leishmania mexicana* cysteine protease B (CPB) peptidase activity assays

Wild-type recombinant CPB without the C-terminal extention (*i.e.* CPB2.8ΔCTE; Sanderson, S.J., et. al., Biochem. J., 347, 383-388, 2000) was obtained from Dr. Jeremy Mottram (Wellcome Centre for Molecular Parasitology, The Anderson College, University of Glasgow, Glasgow, U.K.). Activity assays were carried out in 100 mM sodium acetate; pH 5.5 containing 1 mM EDTA; 200 mM NaCl and 10 mM DTT (Alves, L.C., et. al., Mol. Biochem. Panasitol, 116, 1-9, 2001) using 0.25 nM enzyme. Pro-Phe-Arg-AMC ($K_M^{app}$ ~ 38μM) was used as the substrate at a concentration equivalent to $K_M^{app}$. The rate of conversion of substrate to product was derived from the slope of the increase in fluorescence monitored continuously over time.

Cathepsin peptidase activity assays

Bovine cathepsin S, human cathepsin L, human cathepsin H and human cathepsin B were obtained from CN Biosciences. Recombinant human cathepsin S, human cathepsin K and human cathepsin X were obtained from Dr. Boris Turk (Josef Stefan Institute, Ljubljana, Slovenia). Unless otherwise stated, all peptidase activity assays were carried out in 10 mM bis-tris-propane (BTP), pH 6.5 containing 1 mM EDTA, 5 mM 2-mercaptoethanol and 1 mM $CaCl_2$. Human cathepsin H activity assays were carried out in 10 mM BTP pH 6.5, 142 mM $NaCl_2$, 1 mM $CaCl_2$, 1 mM EDTA, 1 mM DTT, 0.035 mM Zwittergent 3-16. Human cathepsin K assays were carried out in 100 mM sodium acetate; pH 5.5 containing 20 mM L-cysteine and 1 mM EDTA (Bossard, M.J., et. al., J. Biol. Chem., 21, 12517-12524, 1996). Human cathepsin X assays were carried out in 100 mM sodium acetate; pH 5.5 containing 20 mM L-cysteine; 0.05% (w/v) Brij 35 and 1 mM EDTA (Santamaria, I., et. al., J. Biol. Chem., 273, 16816-16823, 1998; Klemencic, J, et al., Eur. J. Biochem., 267, 5404-5412, 2000). The final enzyme concentrations used in the assays were 0.5 nM bovine cathepsin S, 1 nM cathepsin L, 0.1 nM cathepsin B, 0.25nM Cathepsin K; 1 nM cathepsin X and 10 nM cathepsin H. For the inhibition assays, the substrates used for cathepsin S, cathepsin L, cathepsin B, cathepsin K and cathepsin H were boc-Val-Leu-Lys-AMC ($K_M^{app}$ ~ 30 μM), Ac-Phe-Arg-AMC ($K_M^{app}$ ~ 20 μM), Z-Phe-Arg-AMC ($K_M^{app}$ ~ 40 μM), Z-Leu-Arg-AMC ($K_M^{app}$ ~ 2μM); Bz-Phe-Val-Arg-AMC ($K_M^{app}$ ~ 150 μM) respectively. In each case the substrate concentration used in each assay was equivalent to the $K_M^{app}$. The rate of conversion of substrate to product was derived from the slope of the increase in fluorescence monitored continuously over time.

Trypsin peptidase activity assays

Human pancreatic trypsin (iodination grade; CN Biosciences) activity assays were carried out in 10 mM HEPES, pH 8.0 containing 5 mM $CaCl_2$ using 0.1 nM trypsin. For the inhibition assays, Z-Gly-Gly-Arg-AMC ($K_M^{app}$ ~ 84 μM) was used as the substrate at a concentration equivalent to $K_M^{app}$. The rate of conversion of substrate to product was derived from the slope of the increase in fluorescence monitored continuously over time.

Clostripain peptidase activity assays

Clostripain (Sigma) activity assays were carried out in 10 mM BTP, pH 6.5 containing 1 mM EDTA, 5 mM 2-mercaptoethanol and 1mM $CaCl_2$ using 0.3 nM enzyme. For the inhibition assays, Z-Gly-Gly-Arg-AMC ($K_M^{app}$ ~ 100 μM) was used as the substrate at a concentration equivalent to $K_M^{app}$. The rate of conversion of substrate to product was derived from the slope of the increase in fluorescence monitored continuously over time.

Calpain peptidase activity assays

Calpain (human erythrocyte μ-calpain and porcine kidney m-calpain; CN Biosciences) activity assays were carried out in 10 mM HEPES, pH 7.5 containing 2 mM 2-mercaptoethanol and $CaCl_2$ using 25 nM of either enzyme (Sasaki, et. al., J. Biol. Chem., 259, 12489-12494, 1984). For μ-calpain inhibition assays, the buffer contained 100 μM

$CaCl_2$ and Abz-Ala-Asn-Leu-Gly-Arg-Pro-Ala-Leu-Tyr($NO_2$)-Asp-$NH_2$ ($K_M^{app}$~ 20 μM; Incenta Limited) was used as the substrate. For m-calpain inhibition assays, the assay buffer contained 200 μM $CaCl_2$ and Abz-Lys-Leu-Cys(Bzl)-Phe-Ser-Lys-Gln-Tyr($NO_2$)-Asp-$NH_2$ ($K_M^{app}$~ 22 μM; Incenta Limited) was used as the substrate. In both cases the substrate concentration employed in the assays was equivalent to the $K_M^{app}$. The rate of conversion of substrate to product was derived from the slope of the increase in fluorescence monitored continuously over time.

Extracellular *S. aureus* V8 cysteine peptidase (staphylopain) peptidase activity assays

*S. aureus* V8 was obtained from Prof. S. Arvidson, Karolinska Institute, Stockholm, Sweden. Extracellular *S. aureus* V8 cysteine peptidase (staphylopain) activity assays were carried out using partially purified S. *aureus* V8 culture supernatant (obtained from Dr. Peter Lambert, Aston University, Birmingham, U.K.). Activity assays were carried out in 10 mM BTP, pH 6.5 containing 1 mM EDTA, 5 mM 2-mercaptoethanol and 1mM $CaCl_2$ using two-times diluted partially purified extract. For the inhibition assays, Abz-Ile-Ala-Ala-Pro-Tyr($NO_2$)-Glu-$NH_2$ ($K_M^{app}$ ~ 117 μM; Incenta Limited) was used as the substrate at a concentration equivalent to $K_M^{app}$. The rate of conversion of substrate to product was derived from the slope of the increase in fluorescence monitored continuously over time.

Foot-and-mouth disease leader peptidase (FMDV-LP) activity assays

Recombinant wild-type FMDV-LP was obtained from Dr. Tim Skem (Institut für Medizinische Biochemie, Abteilung für Biochemie, Universtät Wien, Wien, Austria). Activity assays were carried out in 50 mM tris-acetate, pH 8.4 containing 1 mM EDTA, 10 mM L-cysteine and 0.25% (w/v) CHAPS using 10 nM enzyme. For the inhibition assays, Abz-Arg-Lys-Leu-Lys-Gly-Ala-Gly-Ser-Tyr($NO_2$)-Glu-$NH_2$ ($K_M^{app}$ ~ 51 μM, Incenta Limited) was used as the substrate at a concentration equivalent to $K_M^{app}$. The rate of conversion of substrate to product was derived from the slope of the increase in fluorescence monitored continuously over time.

Caspase peptidase activity assays

Caspases 1-10 were obtained from CN Biosciences or BioVision Inc. (Mountain View, CA, USA) and all assays were carried out in 50mM HEPES; pH 7.2, 10% (v/v) glycerol, 0.1% (w/v) CHAPS, 142 mM NaCl, 1 mM EDTA, 5 mM dithiothreitol (DTT) using 0.1-1 U per assay. For caspase 1, Ac-Leu-Glu-His-Asp-AMC was used as the substrate; for caspase 2, Z-Val-Asp-Val-Ala-Asp-AFC was used as the substrate; for caspase 3, Ac-Asp-Glu-Val-Asp-AMC was used as the substrate; for caspase 4, Suc-Tyr-Val-Ala-Asp-AMC was used as the substrate; for caspase 5, Ac-Leu-Glu-His-Asp-AMC was used as the substrate; for caspase 6, Ac-Val-Glu-Ile-Asp-AMC was used as the substrate; for caspase 7, Ac-Asp-Glu-Val-Asp-AMC was used as the substrate; for caspase 8, Ac-Ile-Glu-Thr-Asp-AMC was used as the substrate; for caspase 9, Ac-Leu-Glu-His-Asp-AMC was used as the substrate; for caspase 10, Ac-Ile-Glu-Thr-Asp-AMC was used as the substrate (Nicholson, D.W. and Thornberry, N.A., TIBS, 22, 299-306, 1997**;** Stennicke, H.R. and Salvesen, G.S., J. Biol. Chem., 272(41), 25719-25723, 1997**;** Talanian, R.V., et. al., J. Biol. Chem., 272(15), 9677-9682, 1997**;** Wolf, B.B. and Green, D.R., J. Biol. Chem., 274(29), 20049-20052, 1999**).** The rate of conversion of substrate to product was derived from the slope of the increase in fluorescence monitored continuously over time.

Measurement of the apparent macroscopic binding (Michaelis) constants ($K_M^{app}$) for substrates

The apparent macroscopic binding constant ($K_M^{app}$) for each substrate was calculated, from the dependence of enzyme activity as a function of substrate concentration. The observed rates were plotted on the ordinate against the related substrate concentration on the abscissa and the data fitted by direct regression analysis (Prism v 3.02; GraphPad, San Diego, USA) using Equation 1 (Cornish-Bowden, A. Fundamentals of enzyme kinetics Portland Press; 1995, 93-128.).

$$v_i = \frac{V_{\max}{}^{app}.[S_o]}{[S_o]+K_M{}^{app}} \qquad \textbf{(1)}$$

In Equation 1 **'$v_i$'** is the observed initial rate, **'$V_{max}^{app}$'** is the observed maximum activity at saturating substrate concentration, **'$K_m^{app}$'** is the apparent macroscopic binding (Michaelis) constant for the substrate, **'$[S_o]$'** is the initial substrate concentration.

Measurement of the inhibition constants

The apparent inhibition constant ($K_i$) for each compound was determined on the basis that inhibition was reversible and occurred by a pure-competitive mechanism. The $K_i$ values were calculated, from the dependence of enzyme activity as a function of inhibitor concentration, by direct regression analysis (Prism v 3.02) using Equation 2 (Cornish-Bowden, A., 1995.).

$$v_i = \frac{V_{max}{}^{app}.[S]}{[S] + \{K_M{}^{app}.([I]/K_i)\}} \qquad \textbf{(2)}$$

In Equation 2 **'$v_i$'** is the observed residual activity, **'$V_{max}{}^{app}$'** is the observed maximum activity (*i.e.* in the absence of inhibitor), **'$K_M{}^{app}$'** is the apparent macroscopic binding (Michaelis) constant for the substrate, **'[S]'** is the initial substrate concentration, **'$K_i$'** is the apparent dissociation constant and **'[I]'** is the inhibitor concentration.

In situations where the apparent dissociation constant ($K_i{}^{app}$) approached the enzyme concentrations, the $K_i{}^{app}$ values were calculated using a quadratic solution in the form described by Equation 3 (Morrison, J.F. Trends Biochem. Sci., 7, 102-105, 1982; Morrison, J.F. Biochim. Biophys. Acta, 185, 269-286, 1969; Stone, S.R. and Hofsteenge, J. Biochemistry, 25, 4622-4628, 1986).

$$v_i = \frac{F\{E_o - I_o - K_i^{app} + \sqrt{(E_o - I_o - K_i^{app})^2 + 4.K_i^{app}.E_o}\}}{2} \qquad \textbf{(3)}$$

$$K_i^{app} = K_i(1 + [S_o]/K_M{}^{app}) \qquad \textbf{(4)}$$

In Equation 3 **'$v_i$'** is the observed residual activity, **'F'** is the difference between the maximum activity (i.e. in the absence of inhibitor) and minimum enzyme activity, **'$E_o$'** is the total enzyme concentration, **'$K_i{}^{app}$'** is the apparent dissociation constant and **'$I_o$'** is the inhibitor concentration. Curves were fitted by non-linear regression analysis (Prism) using a fixed value for the enzyme concentration. Equation 4 was used to account for the substrate kinetics, where **'$K_i$'** is the inhibition constant, **'$[S_o]$'** is the initial substrate concentration and **'$K_M{}^{app}$'** is the apparent macroscopic binding (Michaelis) constant for the substrate (Morrison, **1982).**

The second-order rate of reaction of inhibitor with enzyme

Where applicable, the concentration dependence of the observed rate of reaction ($k_{obs}$) of each compound with enzyme was analysed by determining the rate of enzyme inactivation under pseudo-first order conditions in the presence of substrate (Morrison, J.F., TIBS, 102-105, 1982; Tian, W.X. and Tsou, C.L., Biochemistry, 21, 1028-1032, 1982; Morrison, J.F. and Walsh, C.T., from Meister (Ed.), Advances in Enzymol., 61, 201-301, 1988; Tsou, C.L., from Meister (Ed.), Advances in Enzymol., 61, 381-436, 1988;). Assays were carried out by addition of various concentrations of inhibitor to assay buffer containing substrate. Assays were initiated by the addition of enzyme to the reaction mixture and the change in fluorescence monitored over time. During the course of the assay less than 10% of the substrate was consumed.

$$F = v_s t + \frac{(v_o - v_s)\left[1 - e^{(k_{obs} \cdot t)}\right]}{k_{obs}} + D \qquad \textbf{(5)}$$

The activity fluorescence progress curves were fitted by non-linear regression analysis (Prism) using Eq. 5

(Morrison, 1969; Morrison, 1982); where **'F'** is the fluorescence response, **'t'** is time, **'$v_o$'** is the initial velocity, **'$v_s$'** is the equilibrium steady-state velocity, **'$k_{obs}$'** is the observed pseudo first-order rate constant and **'D'** is the intercept at time zero (*i.e.* the ordinate displacement of the curve). The second order rate constant was obtained from the slope of the line of a plot of $k_{obs}$ versus the inhibitor concentration (i.e. $k_{obs}$/[I]). To correct for substrate kinetics, Eq. 6 was used, where **'[$S_o$]'** is the iniitial substrate concentration and **'$K_M^{app}$'** is the apparent macroscopic binding (Michaelis) constant for the substrate.

$$k_{inact} = \frac{k_{obs}\left(1 + [S_o]/K_M{}^{app}\right)}{[I]} \qquad \textbf{(6)}$$

Compounds of the invention were tested by the above described assays and observed to exhibit cathepsin K inhibitory activity or inhibitory activity against an alternative CA C1 cysteine protease with an *in vitro* Ki inhibitory constant of less than or equal to 100μM. Exemplary inhibition data for examples of the invention are given in Table 5.

**Table 5.** Exemplary inhibition data (Ki expressed as μM).

| Example № | Human Cathepsin K | Cruzipain | Bovine Cathepsin S | Human Cathepsin L | CPB |
|---|---|---|---|---|---|
| 2 | <0.01 | >0.3 | >1 | >3 | >0.2 |
| 296 | >50 | >1 | >5 | <0.2 | >5 |
| 250 | >5 | >5 | <0.1 | >1 | >5 |
| 346 | >8 | <0.2 | >10 | >3 | >5 |

<u>**Human Osteoclast Resorption Assay**</u>

Bone resorption was studied using a model where human osteoclast precursor cells were cultured on bovine bone slices for 9 days and allowed to differentiate into bone-resorbing osteoclasts. The formed mature osteoclasts were then allowed to resorb bone. The assay was performed by Pharmatest Services Ltd, Itäinen Pitkakatu 4C, Turku, Finland. After the culture period, bone collagen degradation products were quantified from the culture medium as an index of bone resorption.

Inhibitor compounds were added into the cell cultures after the differentiation period and their effects on the resorbing activity of mature osteoclasts were determined. The studies included a baseline group without added compounds and a positive control group where a potent cathepsin K inhibitor E-64 was added.

Human peripheral blood monocytes were suspended to culture medium and allowed to attach to bovine bone slices. The bone slices were transferred into 96-well tissue culture plates containing culture medium with appropriate amounts of important growth factors favouring osteoclast differentiation, including M-CSF, RANK-ligand and TGF-β. The cells were incubated in a $CO_2$ incubator in humidified atmosphere of 95% air and 5% carbon dioxide at 37°C. At day 7 when osteoclast differentiation was complete, the culture medium was replaced with culture medium containing conditions favouring osteoclast activity. The cell culture was continued for an additional 2 days, during which the formed mature osteoclasts were allowed to resorb bone in the presence of vehicle, control inhibitor (E64) or test compounds. At the end of the culture, bone collagen degradation products released into the culture medium were determined using a commercially available ELISA method (CrossLaps® for culture, Nordic Bioscience, Herlev, Denmark) as an index of bone resorption (see Bagger, Y. Z. et al, J. Bone. Miner. Res. 14 (suppl. 1), S370).

In this assay, selected EXAMPLES of the invention exhibited more than 70% inhibition of bone resorption at a concentration of 100nM.

**Claims**

**1.** A compound of general formula (I)

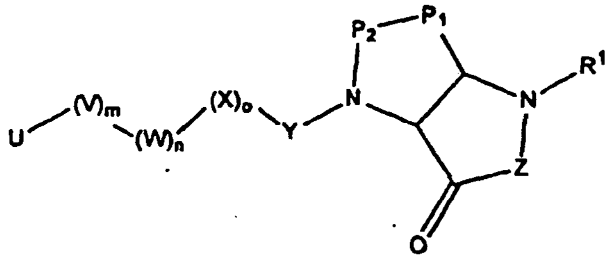

(I)

wherein:

Z = $CR^3R^4$, where $R^3$ and $R^4$ are independently chosen from $C_{0-7}$-alkyl (when C = 0, $R^3$ or $R^4$ is simply a hydrogen atom), $C_{3-6}$-cycloalkyl, Ar-$C_{0-7}$-alkyl (when C = 0, $R^3$ or $R^4$ is simply an aromatic moiety Ar),

$P_1$ = $CR^5R^6$, where $R^5$ and $R^6$ are independently chosen from $C_{0-7}$alkyl, $C_{3-6}$-cycloalkyl, Ar-$C_{0-7}$-alkyl, O-$C_{0-7}$alkyl, O-$C_{3-6}$-cycloalkyl, O-Ar-$C_{0-7}$-alkyl, S-$C_{0-7}$-alkyl, S-$C_{3-6}$-cycloalkyl, S-Ar-$C_{0-7}$-alkyl, NH-$C_{0-7}$-alkyl, NH-$C_{3-6}$-cycloalkyl, NH-Ar-$C_{0-7}$-alkyl, N($C_{0-7}$-alkyl)$_2$, N($C_{3-6}$-cycloalkyl)$_2$ and N(Ar-$C_{0-7}$-alkyl)$_2$;

$P_2$ = O, $CR^7R^8$ or $NR^9$, where $R^7$ and $R^8$ are independently chosen from $C_{0-7}$-alkyl, $C_{3-6}$-cycloalkyl, Ar-$C_{0-7}$-alkyl and $R^9$ is chosen from $C_{0-7}$alkyl, $C_{3-6}$-cycloalkyl and Ar-$C_{0-7}$-alkyl;

Y = $CR^{10}R^{11}$-C(O) or $CR^{10}R^{11}$-C(S) or $CR^{10}R^{11}$-S(O) or $CR^{10}R^{11}$-$SO_2$
where $R^{10}$ and $R^{11}$ are independently chosen from $C_{0-7}$-alkyl, $C_{3-6}$-cycloalkyl and Ar-$C_{0-7}$-alkyl, or Y represents

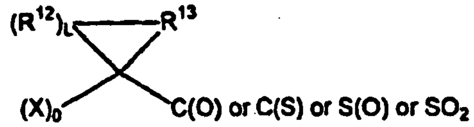

where L is a number from one to four and $R^{12}$ and $R^{13}$ are independently chosen from $CR^{14}R^{15}$ where $R^{14}$ and $R^{15}$ are independently chosen from $C_{0-7}$-alkyl, $C_{3-6}$-cycloalkyl, Ar-$C_{0-7}$-alkyl and halogen; and for each $R^{12}$ and $R^{13}$ either $R^{14}$ or $R^{15}$ (but not both $R^{14}$ and $R^{15}$) may additionally be chosen from O-$C_{0-7}$alkyl, O-$C_{3-6}$-cycloalkyl, O-Ar-$C_{0-7}$-alkyl, S-$C_{0-7}$alkyl, S-$C_{3-6}$-cycloalkyl, S-Ar-$C_{0-7}$-alkyl, NH-$C_{0-7}$-alkyl, NH-$C_{3-6}$-cycloalkyl, NH-Ar$C_{0-7}$-alkyl, N-($C_{0-7}$-alkyl)$_2$, N-($C_{3-6}$-cycloalkyl)$_2$, and N-(Ar-$C_{0-7}$-alkyl)$_2$;

$(X)_o$ = $CR^{16}R^{17}$, where $R^{16}$ and $R^{17}$ are independently chosen from $C_{0-7}$-alkyl, $C_{3-6}$-cycloalkyl and Ar-$C_{0-7}$-alkyl and o is a number from zero to three;

$(W)_n$ = O, S, C(O), S(O) or S(O)$_2$ or $NR^{18}$, where $R^{18}$ is chosen from $C_{0-7}$-alkyl, $C_{3-6}$-cycloalkyl and Ar-$C_{0-7}$-alkyl and n is zero or one;

$(V)_m$ = C(O), C(S), S(O), S(O)$_2$, S(O)$_2$NH, OC(O), NHC(O), NHS(O), NHS(O)$_2$, OC(O)NH, C(O)NH or $CR^{19}R^{20}$, C=N-C(O)-$OR^{19}$ or C=N-C(O)-$NHR^{19}$, where $R^{19}$ and $R^{20}$ are independently chosen from $C_{0-7}$-alkyl, $C_{3-6}$-cycloalkyl, Ar-$C_{0-7}$-alkyl and m is a number from zero to three, provided that when m is greater than one, $(V)_m$ contains a maximum of one carbonyl or sulphonyl group;

U = a stable 5- to 7-membered monocyclic or a stable 8- to 11-membered bicyclic ring which is either saturated or unsaturated and which includes zero to four heteroatoms (as detailed below):

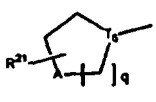
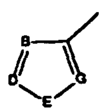
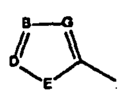
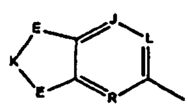
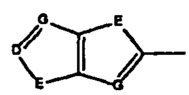

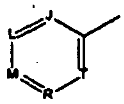 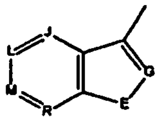 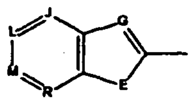 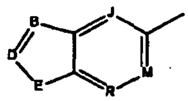 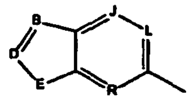

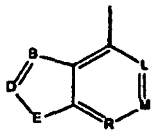 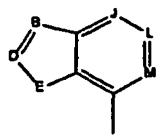 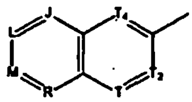 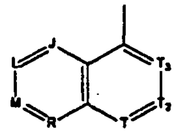 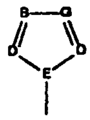

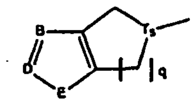 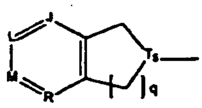 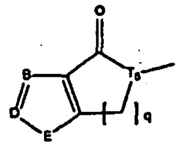 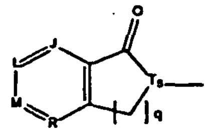

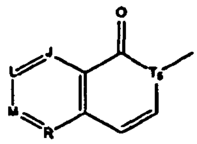 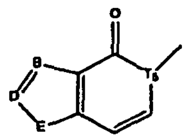 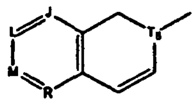 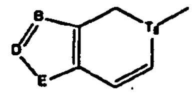

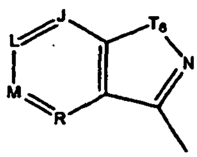 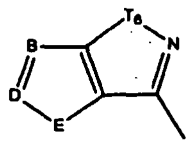 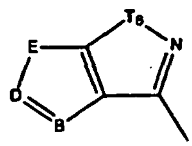

wherein $R^{21}$ is:

$C_{0-7}$-alkyl, $C_{3-6}$-cycloalkyl, Ar-$C_{0-7}$-alkyl, O-$C_{0-7}$-alkyl, O-$C_{3-6}$-cycloalkyl, O-Ar-$C_{0-7}$-alkyl, S-$C_{0-7}$-alkyl, S-$C_{3-6}$-cycloalkyl, S-Ar-$C_{0-7}$-alkyl, $SO_2$-$C_{0-7}$-alkyl, $SO_2$-$C_{3-6}$-cycloalkyl, $SO_2$-Ar-$C_{0-7}$-alkyl, NH-$C_{0-7}$-alkyl, NH-$C_{3-6}$-cycloalkyl, NH-Ar-$C_{0-7}$-alkyl, N($C_{0-7}$-alkyl)$_2$, N($C_{3-6}$-cycloalkyl)$_2$ or N(Ar-$C_{0-7}$-alkyl)$_2$; or, when part of a $CHR^{21}$ or $CR^{21}$ group, $R^{21}$ may be halogen;

A is chosen from:

$CH_2$, $CHR^{21}$, O, S, $SO_2$, $NR^{22}$ or N-oxide (N→O), where $R^{21}$ is as defined above; and $R^{22}$ is chosen from $C_{0-7}$-alkyl, $C_{3-6}$-cycloalkyl and Ar-$C_{0-7}$-alkyl;

B, D and G are independently chosen from:

$CR^{21}$, where $R^{21}$ is as defined above, or N or N-oxide (N→O);

E is chosen from:

$CH_2$, $CHR^{21}$, O, S, $SO_2$, $NR^{22}$ or N-oxide (N→O), where $R^{21}$ and $R^{22}$ are defined as above;

K is chosen from:

$CH_2$, $CHR^{22}$, where $R^{22}$ is defined as above;

J, L, M, R, T, $T_2$, $T_3$ and $T_4$ are independently chosen from:

$CR^{21}$ where $R^{21}$ is as defined above, or N or N-oxide (N→O);

$T_5$ is chosen from:

CH or N;

$T_6$ is chosen from:

$NR^{22}$, $SO_2$, OC(O), C(O), $NR^{22}$C(O);

q is a number from one to three, thereby defining a 5-, 6- or 7-membered ring;
$R^1$ = $R^2C(O)$, $R^2OC(O)$, $R^2NQC(O)$, $R^2SO_2$, where $R^2$ is chosen from $C_{1-7}$-alkyl, $C_{3-6}$-cycloalkyl or Ar-$C_{0-7}$-alkyl (when C = 0, $R^2$ is simply an aromatic moiety Ar) and Q is $C_{0-7}$-alkyl;
or a salt, hydrate, solvate or complex thereof.

**2.** A compound as claimed in claim 1 wherein independently or in any combination:

Z is $CH_2$;
$P_1$ is $CH_2$;
$P_2$ is $CH_2$, O or NH
$R^2$ is Ar-$C_{0-2}$-alkyl
or $R^2$ is $C_{3-7}$-alkyl which may include an -O- or -NH- as part of the chain and which is either unsubstituted or is substituted with one or more $NH_2$, NHMe, $NHC(O)CH_3$, $NMeC(O)CH_3$, OH or OMe groups
or $R^2$ is a $C_{3-6}$-cycloalkyl group, wherein the ring system is either connected directly to the remainder of the $R^1$ moiety or there is one intervening methylene group;
in the group $(X)_o$, each of $R^{16}$ and $R^{17}$ is selected from $C_{0-7}$-alkyl or M-$C_{0-7}$-alkyl, for example hydrogen, a straight or branched alkyl chain, a straight or
branched heteroalkyl chain, an optionally substituted arylalkyl chain or an optionally substituted arylheteroalkyl chain;
in the group $(X)_o$, $R^{16}$ and $R^{17}$ are hydrogen and o is zero or one;
in the group $(V)_m$, V is chosen from C(O), OC(O), NHC(O), C(O)NH,
$CHR^{20}$, C=N-C(O)-$OR^{19}$ or C=N-C(O)-$NHR^{19}$

where $R^{19}$ is chosen from $C_{0-7}$-alkyl, $C_{3-6}$-cycloalkyl, Ar-$C_{0-7}$-alkyl and $R^{20}$ is $C_{0-4}$-alkyl, and
m is zero or one.

**3.** A compound as claimed in claim 2, wherein $R^2$ is:

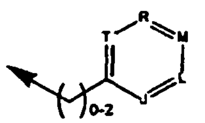
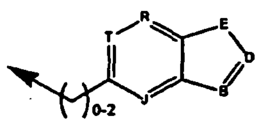
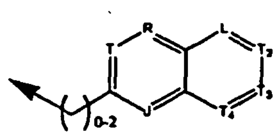
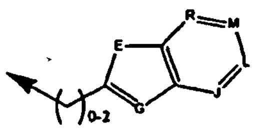
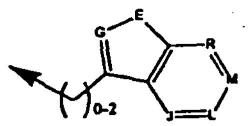
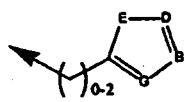
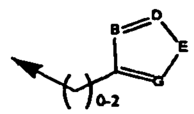

where J, L, M, R, T, $T_2$, $T_3$ and $T_4$, B, D, G and E are as previously defined.

**4.** A compound as claimed in any one of claims 1 to 3, wherein $R^2$ is Ar-$C_{0-1}$-alkyl, for example:

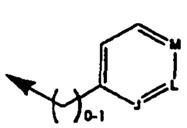
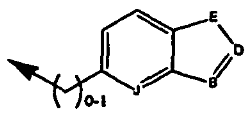
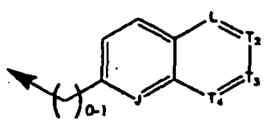
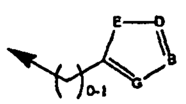
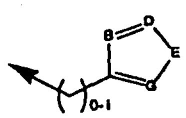
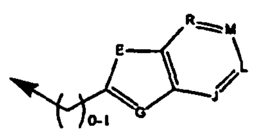
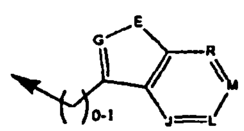

where J, L, M, $T_2$, $T_3$, $T_4$, B, D, G and E are as previously defined.

**5.** A compound as claimed in claim 4 wherein $R^2$ comprises a monocyclic $ArC_{0-1}$-alkyl and forms part of an $R^1$ group selected from:

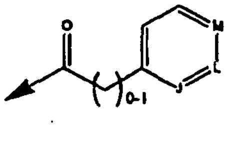
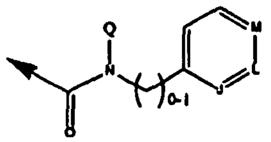
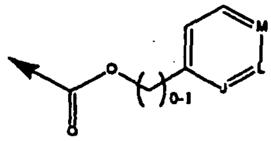
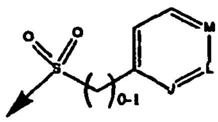
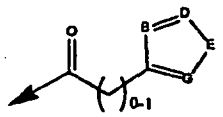
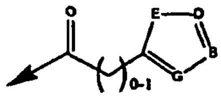
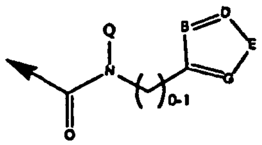
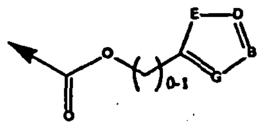

wherein:
J, L, M, B, D and G are as defined above (i.e. $CR^{21}$, N or N→O) and wherein $R^{21}$ is chosen from hydrogen, methyl, methoxy, ethyl, isopropyl, trifluoromethyl, trifluoromethoxy, F, Cl, $SO_2Me$; and
B, D, G are as defined above (i.e. $CR^{21}$, N or N→O) and where $R^{21}$ is chosen from hydrogen, methyl, methoxy, ethyl, isopropyl, trifluoromethyl, trifluoromethoxy, F, Cl, $SO_2Me$; and
E is as previously defined; and
Q is chosen from hydrogen or methyl.

**6.** A compound as claimed in claim 2 wherein When $R^2$ is a $C_{3-6}$-alkyl group which is branched at the α-position or which includes an $NH_2$, NHMe, $NHC(O)CH_3$, $NMeC(O)CH_3$, OH or OMe substituent at the α-position.

**7.** A compound as claimed in claim 1 or claim 2 wherein $R^2$ is cyclopropyl, cyclobutyl, cyclopentyl, cyclohexyl, pyrrolidine, piperidine, morpholine, tetrahydrofuran, cyclopentene, cyclopentadiene, cyclohexadiene and piperazine, wherein nitrogen-containing rings may be N-substituted with groups such as $C_{1-4}$ alkyl, phenyl or benzyl.

**8.** A compound as claimed in claim 1 or claim 2 wherein $R^1$ is:
benzoyl; pyridine-2-carbonyl; 1-oxy-pyridine-2-carbonyl; pyridine-3-carbonyl; 1-oxy-pyridine-3-carbonyl; pyridine-4-carbonyl; 1-oxy-pyridine-4-carbonyl; phenyl sulphonyl; pyridine-2-sulphonyl; 1-oxy-pyridine-2-sulphonyl; pyridine-3-sulphonyl; 1-oxy-pyridine-3-sulphonyl; pyridine-4-sulphonyl; 1-oxy-pyridine-4-sulphonyl; phenylacetyl; phenylcarbamoyl; isobutylcarbamoyl; phenyloxycarbonyl; isobutyloxycarbonyl; pyrrolidine-N-carbonyl; piperidine-N-carbonyl; morpholine-N-carbonyl; piperazine-N-carbonyl; 4-methyl-piperazine-N-carbonyl; (4-methylpiperazin-1-yl)-acetoyl; piperazin-1-yl-acetoyl; furan-2-carbonyl; 5-chlorofuran-2-carbonyl; thiophene-2-carbonyl; 5-chlorothiophene-2-carbonyl; furan-3-carbonyl; thiophene-3-carbonyl; cyclopentoyl; cyclohexoyl; cyclopent-3-enoyl; cyclopentylmethylcarbonyl; cyclohexylmethylcarbonyl; pyrrolidine-2-carbonyl; N-acetyl-pyrrolidine-2-carbonyl; piperidine-2-carbonyl; N-acetyl-piperidine-2-carbonyl; tetrahydrofuran-2-carbonyl; 1-aminocyclobutanoyl; 1-aminocyclopentanoyl; 1-aminocyclohexanoyl; N-acetyl-1-aminocyclobutanoyl; N-acetyl-1-aminocyclopentanoyl; N-acetyl-1-aminocyclohexanoyl; 1-hydroxycyclobutanoyl; 1-hydroxycyclopentanoyl; 1-hydroxycyclohexanoyl; 1-methoxycyclobutanoyl; 1-methoxycyclopentanoyl; 1-methoxycyclohexanoyl; aminocyclopentylacetoyl; aminocyclohexylacetoyl; N-acetylaminocyclopentylacetoyl; N-acetylaminocyclohexylacetoyl; 2-acetylaminopropionoyl; 2-acetylaminoethanoyl; 2-acetyl-N-methylaminoethanoyl; N,N-dimethylaminoacetoyl; 2-aminobutanoyl; N-acetyl-2-aminobutanoyl; 2-amino-3-methylbutanoyl; N-acetyl-2-amino-3-methylbutanoyl; 2-amino-3,3-dimethylbutanoyl; N-acetyl-2-amino-3,3-dimethylbutanoyl; 2-amino-3-methylpentanoyl; N-acetyl-2-amino-3-methylpentanoyl; pentanoyl; 3-methylpentanoyl; 4-methylpentanoyl; 2-amino-4-methylpentanoyl; N-acetyl-2-amino-4-methylpentanoyl; 2-amino-4,4-dimethylpentanoyl; N-acetyl-2-amino-4,4-dimethylpentanoyl; 2-aminopentanoyl; N-acetyl-2-aminopentanoyl; 2-amino-5-methylhexanoyl; N-acetyl-2-amino-5-methylhexanoyl; 2-hydroxy-3-methylbutanoyl; 2-methoxy-3-methylbutanoyl; 2-hydroxy-3,3-dimethylbutanoyl; 2-methoxy-3,3-dimethylbutanoyl; 2-hydroxy-3-methylpentanoyl; 2-methoxy-3-methylpentanoyl; 2-hydroxy-4-methylpentanoyl; 2-methoxy-4-methylpentanoyl; 2-hydroxy-4,4-dimethylpentanoyl; 2-methoxy-4,4-dimethylpentanoyl; 2-hydroxypentanoyl; 2-methoxypentanoyl; 2-hydroxy-5-methylhexanoyl; 2-methoxy-5-methylhexanoyl;

**9.** A compound as claimed in any one of claims 2 to 8, wherein, in the group $(X)_o$, $R^{16}$ is hydrogen and $R^{17}$ is hydrogen; $C_{1-4}$-alkyl, which may be substituted with OH, $NR^{22}R^{22}$, $COOR^{22}$, or $CONR^{22}$; or Ar-$C_{1-4}$-alkyl, where the aryl group may be substituted with $R^{21}$, wherein each $R^{21}$ and $R^{22}$ is independently as defined in claim 1.

**10.** A compound as claimed in claim 9 wherein, in the group $(X)_o$, $R^{16}$ is hydrogen and $R^{17}$ is chosen from hydrogen or a simple $C_{1-4}$-alkyl group.

**11.** A compound as claimed in claim 10 wherein, in the group $(X)_o$, $R^{16}$ and $R^{17}$ are hydrogen and o is zero or one.

**12.** A compound as claimed in any one of claims 2 to 11, wherein, in the group $(W)_n$, W comprises O, S, $SO_2$, C(O) or NH and n is zero or one.

**13.** A compound as claimed in claim 12 wherein, in the group $(W)_n$, W is C(O) or NH where n is zero or one.

**14.** A compound as claimed in claim 13 wherein in the group $(W)_n$, W is NH and n is zero or one.

**15.** A compound as claimed in any one of claims 1 to 14, wherein the U-$(V)_m$-$(W)_n$-$(X)_o$-Y or U-$(V)_m$-$(W)_n$-$(X)_o$ substituent combination comprises:

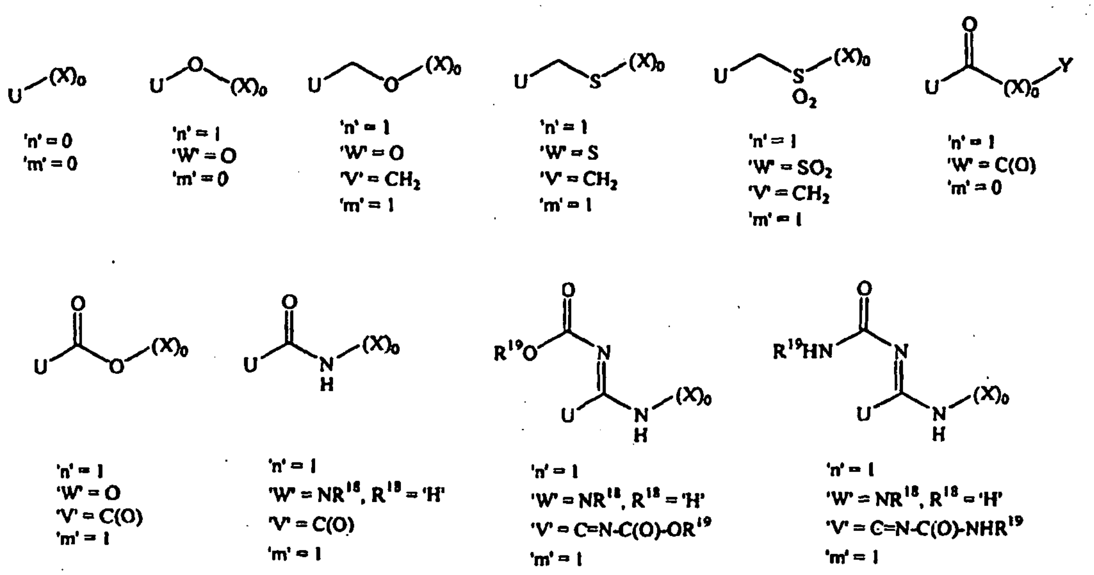


**16.** A compound as claimed in any one of claims 1 to 15 wherein the U-$(V)_m$-$(W)_n$-$(X)_o$-Y substituent combination comprises:

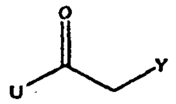

(X)0 = 'CH2'
'n' = 1
'W' = C(O)
'm' = 0

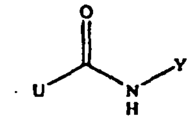

(X)0 = '-'
'n' = 1
'W' = NR18, R18 = 'H'
'V' = C(O)
'm' = 1

**17.** A compound as claimed in any one of claims 1 to 16 which is an inhibitor of cathepsin K and wherein independently or in any combination:

Y is $CHR^{11}CO$ where $R^{11}$ is selected from $C_{0-7}$-alkyl, Ar-$C_{0-7}$-alkyl and $C_{3-6}$-cycloalkyl; or Y comprises a group:

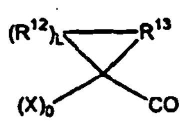

where $R^{12}$ and $R^{13}$ are each $CR^{14}R^{15}$ and each $R^{14}$ and $R^{15}$ is, independently, selected from $C_{0-7}$-alkyl and Ar-$C_{0-7}$-alkyl, for example hydrogen, a straight or branched alkyl chain, a straight or branched heteroalkyl chain, an optionally substituted arylalkyl chain or an optionally substituted arylheteroalkyl chain and L is a number from one to four; and
the group U comprises an optionally substituted 5- or 6-membered saturated or unsaturated heterocycle or Ar group or an optionally substituted saturated or unsaturated 8 to 10-membered heterocycle or Ar group.

**18.** A compound as claimed in claim 17 wherein $(X)_o$-Y is:

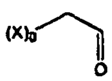 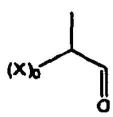 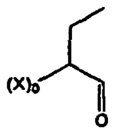 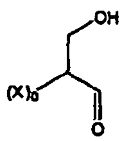 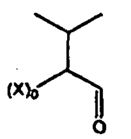 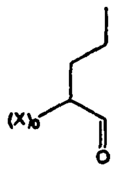 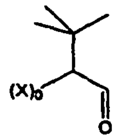

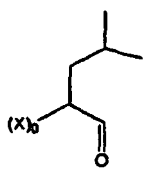 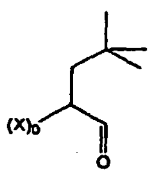 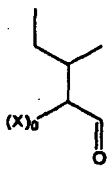 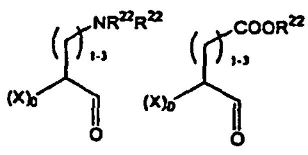 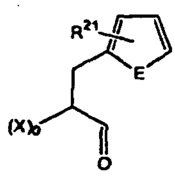

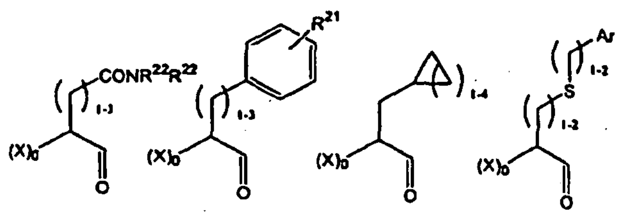 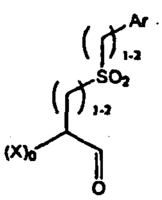 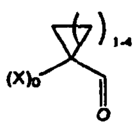

wherein E, $R^{21}$, $R^{22}$ and Ar are as defined previously; any of which may be substituted with one or more halogen, preferably fluoro, substituents.

**19.** A compound as claimed in claim 17, wherein in the group Y, $R^{11}$ is $C_{1-4}$-alkyl, which may be substituted with cycloalkylmethyl or halogen, or $R^{11}$ is chosen from cycloalkyl-1-carbonyl or $R^{11}$ is chosen from Ar-$C_{1-4}$-alkyl, where the aryl group may be substituted with $R^{21}$; where $R^{21}$ is defined in claim 1.

**20.** A compound as claimed in claim 19, wherein, in the group Y, $R^{11}$ is a simple straight or branched alkyl group, optionally substituted with one or more halogen substitutents.

**21.** A compound as claimed in claim 20, wherein in the group Y, $R^{11}$ is $ArCH_2$-, where the aromatic ring is an optionally substituted monocyclic heterocycle.

**22.** A compound as claimed in any one of claims 17 to 21 wherein the $(X)_o$-Y group comprises:

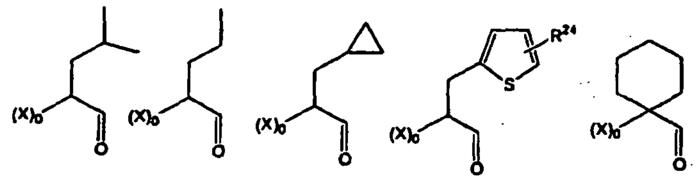

wherein $R^{24}$ is chosen from hydrogen, methyl, methoxy, ethyl, isopropyl, F, Cl and wherein any of the alkyl groups may be substituted with one or more F or Cl.

**23.** A compound as claimed in any one of claims 17 to 22, wherein the group U comprises:

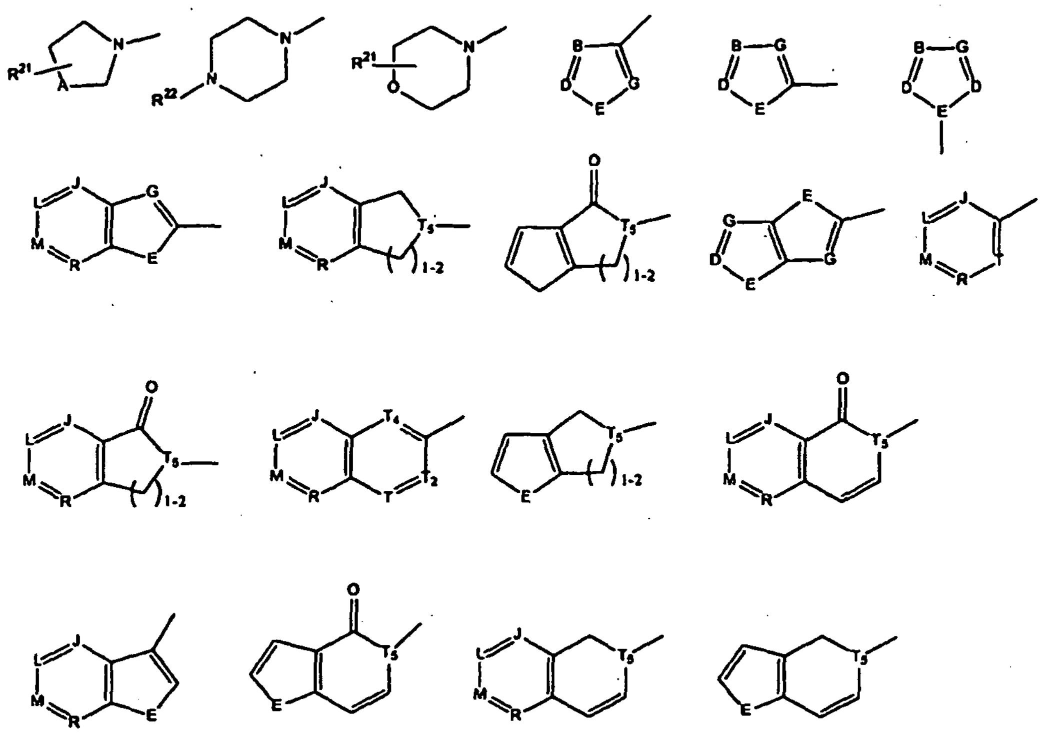

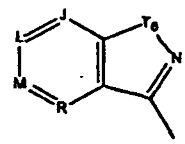 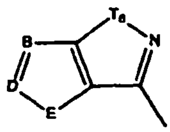 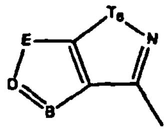

wherein $R^{21}$, $R^{22}$, A, B, D, E, G, J, L, M, R, T, $T_2$, $T_4$, $T_5$ and $T_6$ are as defined in claim 1.

**24.** A compound as claimed in claim 23, wherein U comprises:

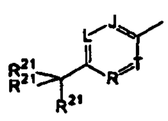 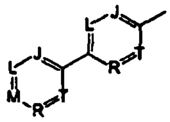 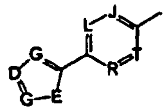 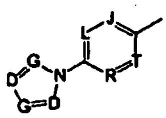 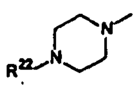

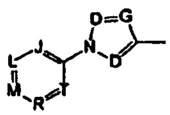 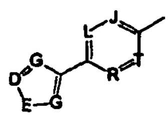 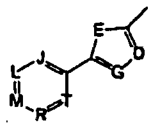 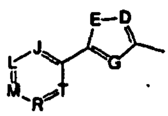 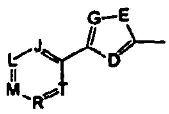

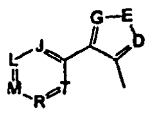 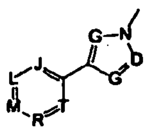 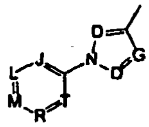 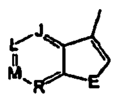 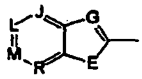

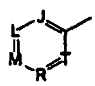 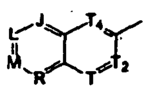 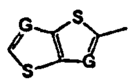

wherein $R^{21}$, $R^{22}$, D, E, G, J, L, M, R, T, $T_2$ and $T_4$ are as defined previously.

**25.** A compound as claimed in claim 24, wherein U comprises:

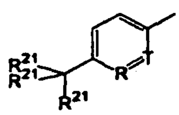 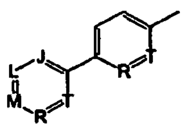 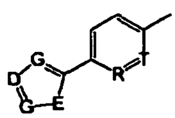 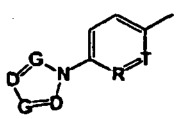 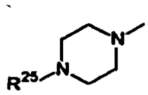

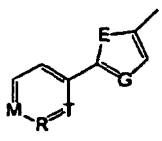 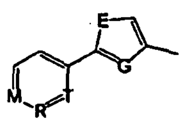 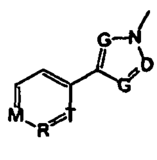 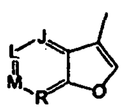 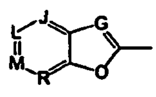

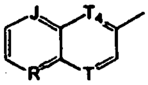 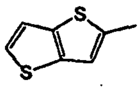 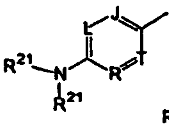 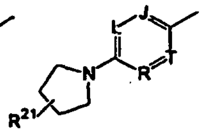 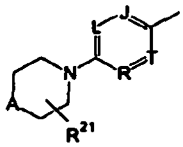

wherein $R^{21}$, $R^{25}$, D, E, G, J, L, M, R, T and $T_4$ are as defined in claim 1.

**26.** A compound as claimed in claim 25, wherein U comprises:

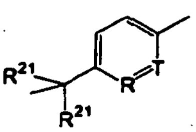 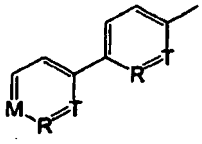 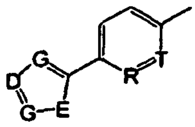 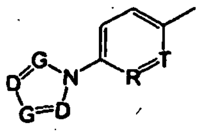

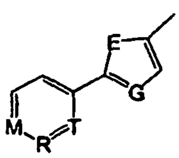 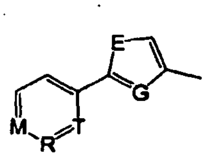 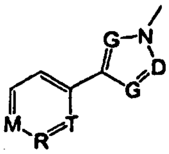

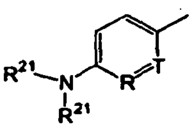 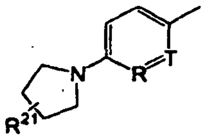 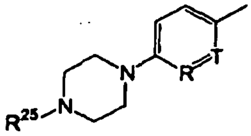

wherein $R^{21}$, $R^{25}$, D, E, G, M, R and T are as defined in claim 1.

**27.** A compound as claimed in any one of claims 1 to 16 which is an inhibitor of cathepsin S and wherein, independently or in any combination, $(X)_o$-Y comprises:

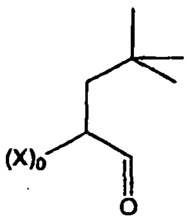 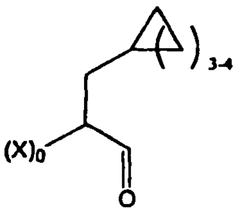 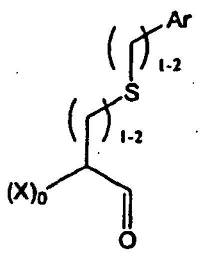 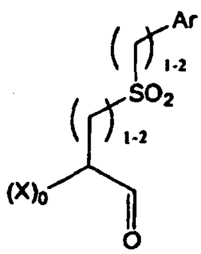

wherein $(X)_o$ is as defined in claim 1 and
U comprises:

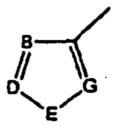 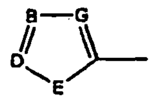 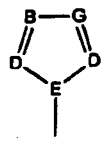 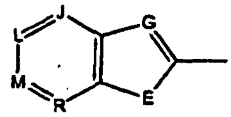 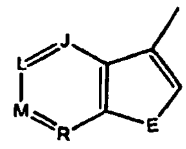

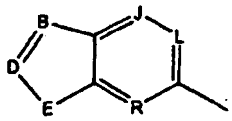 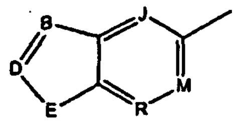 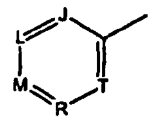 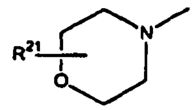

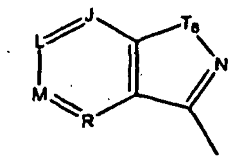 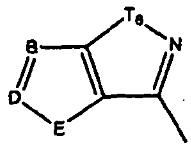 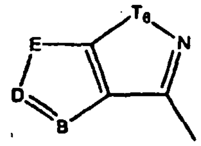

wherein $R^{21}$, B, D, E, G, J, L, M, R and $T_6$ are as defined in claim 1.

**28.** A compound as claimed in claim 27, wherein U comprises an optionally substituted 5-membered unsaturated heterocycle or a 6,5- or 5,5-fused aromatic ring.

**29.** A compound as claimed in claim 28, wherein U comprises:

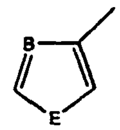 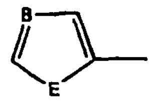 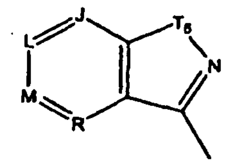 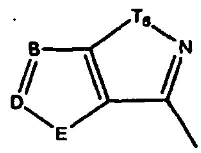 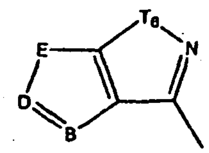

wherein B, D, E, J, L, M, R and $T_6$ are as defined in claim 1.

**30.** A compound as claimed in any one of claims 1 to 24 which is an inhibitor of cathepsin L and wherein, independently or in any combination,
$(X)_o$-Y comprises:

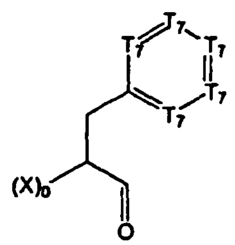

wherein $T_7$ is chosen from CH, N or $CR^{21}$ where $R^{21}$ is as defined in claim 1; and
U comprises an optionally substituted 5-membered unsaturated heterocycle or a 6,6- or 6,5- or 5,6-fused aromatic ring, or a meta-substituted Ar.

**31.** A compound as claimed in claim 30, wherein, within the $T_7$ substituent, the $R^{21}$ substituent is chosen from single and multiple ring substitution combinations of Me, F, Cl, OH and OMe.

**32.** A compound as claimed in claim 30 or claim 31, wherein U comprises:

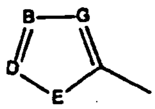
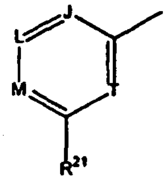
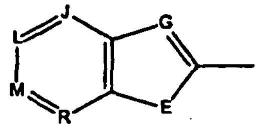

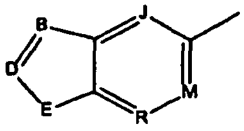
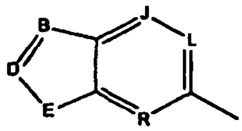
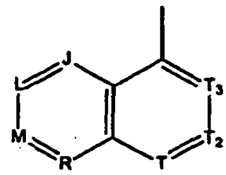

wherein $R^{21}$, B, D, E, G, J, L, M, R, T, $T_2$ and $T_3$ are as defined in claim 1.

**33.** A compound as claimed in any one of claims 30 to 32 wherein U comprises:

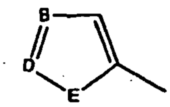
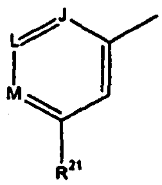
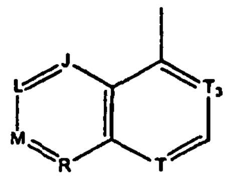

wherein E is chosen from oxygen or N-ethyl, D is chosen from nitrogen or $CCH_3$, B is chosen from nitrogen or $CCH_3$, $R^{21}$ is chosen from halogen, OMe, $CF_3$, $OCF_3$, $CH_2NH_2$ and J, L, M, R, T and $T_3$ are as defined in claim 1.

**34.** A compound as claimed in any one of claims 1 to 33 which is a cis-bicyclic isomer.

**35.** A compound selected from:
(3aR,6aS)-N-{(1S)-3-Methyl-1-[6-oxo-4-(pyridine-2-carbonyl)-hexahydropyrrolo [3,2-b]pyrrole-1-carbonyl]-butyl}-benzamide;
(3aR,6aS)-N-{(1S)-3-Methyl-1-[6-oxo-4-(pyridine-2-carbonyl)-hexahydropyrrolo [3,2-c]pyrazole-1-carbonyl]-butyl}-benzamide;
(3aS,6aS)-N-{(1S)-3-Methyl-1-[6-oxo-4-(pyridine-2-carbonyl)-hexahydro-2-oxa-1,4-diaza-pentalene-1-carbonyl]-butyl}-benzamide.
Naphthalene-2-carboxylic acid {3-methyl-1-[6-oxo-4-(pyridine-2-sulfonyl)hexahydro-pyrrolo[3,2-b]pyrrole-1-carbonyl]-butyl}-amide;
Quinoline-2-carboxylic acid {3-methyl-1-[6-oxo-4-(pyridine-2-sulfonyl)-hexahydro-pyrrolo[3,2-b]pyrrole-1-carbonyl]-butyl}-amide;
Benzo[b]thiophene-2-carboxylic acid {3-methyl-1-[6-oxo-4-(pyridine-2-sulfonyl)-hexahydro-pyrrolo[3,2-b]pyrrole-1-carbonyl]-butyl}-amide;
Benzofuran-2-carboxylic acid {3-methyl-1-[6-oxo-4-(pyridine-2-sulfonyl)-hexahydro-pyrrolo[3,2-b]pyrrole-1-carbonyl]-butyl}-amide;
Biphenyl-4-carboxylic acid {3-methyl-1-[6-oxo-4-(pyridine-2-sulfonyl)-hexahydro-pyrrolo[3,2-b]pyrrole-1-carbonyl]-butyl}-amide;
4-tert-Butyl-N-{3-methyl-1-[6-oxo-4-(pyridine-2-sulfonyl)-hexahydro-pyrrolo[3,2-b]pyrrole-1-carbonyl]-butyl}-benzamide;
4-Dimethylamino-N-{3-methyl-1-[6-oxo-4-(pyridine-2-sulfonyl)-hexahydro-pyrrolo[3,2-b]pyrrole-1-carbonyl]-butyl}-benzamide;
7-Methoxy-benzofuran-2-carboxylic acid {3-methyl-1-[6-oxo-4-(pyridine-2-sulfonyl)-hexahydro-pyrrolo[3,2-b]pyrrole-1-carbonyl]-butyl}-amide;
{3-Methyl-1-[6-oxo-4-(pyridine-2-sulfonyl)-hexahydro-pyrrolo[3,2-b]pyrrole-1-carbonyl]-butyl}-carbamic acid benzyl ester;
5-Methoxy-benzofuran-2-carboxylic acid {3-methyl-1-[6-oxo-4-(pyridine-2-sulfonyl)-hexahydro-pyrrolo[3,2-b]pyrrole-1-carbonyl]-butyl}-amide;
Thieno[3,2-b]thiophene-2-carboxylic acid {3-methyl-1-[6-oxo-4-(pyridine-2-sulfonyl)-hexahydro-pyrrolo[3,2-b]pyrrole-1-carbonyl]-butyl}-amide;
3-Methyl-benzofuran-2-carboxylic acid {3-methyl-1-[6-oxo-4-(pyridine-2-sulfonyl)-hexahydro-pyrrolo[3,2-b]pyrrole-1-carbonyl]-butyl}-amide;
Quinoxaline-2-carboxylic acid {3-methyl-1-[6-oxo-4-(pyridine-2-sulfonyl)-hexahydro-pyrrolo[3,2-b]pyrrole-1-carbonyl]-butyl}-amide;
Benzo[1,3]dioxole-5-carboxylic acid {3-methyl-1-[6-oxo-4-(pyridine-2-sulfonyl)-hexahydro-pyrrolo[3,2-b]pyrrole-1-carbonyl]-butyl}-amide;
Quinoline-6-carboxylic acid {3-methyl-1-[6-oxo-4-(pyridine-2-sulfonyl)-hexahydro-pyrrolo[3,2-b]pyrrole-1-carbonyl]-butyl}-amide;
Furan-2-carboxylic acid {3-methyl-1-[6-oxo-4-(pyridine-2-sulfonyl)-hexahydro-pyrrolo[3,2-b]pyrrole-1-carbonyl]-butyl}-amide;
Thiophene-2-carboxylic acid {3-methyl-1-[6-oxo-4-(pyridine-2-sulfonyl)-hexahydro-pyrrolo[3,2-b]pyrrole-1-carbonyl]-butyl}-amide;
N-{3-Methyl-1-[6-oxo-4-(pyridine-2-sulfonyl)-hexahydro-pyrrolo[3,2-b]pyrrole-1-carbonyl]-butyl}-4-trifluoromethyl-benzamide;
Furan-3-carboxylic acid {3-methyl-1-[6-oxo-4-(pyridine-2-sulfonyl)-hexahydro-pyrrolo[3,2-b]pyrrole-1-carbonyl]-butyl}-amide;
Thiophene-3-carboxylic acid {3-methyl-1-[6-oxo-4-(pyridine-2-sulfonyl)-hexahydro-pyrrolo[3,2-b]pyrrole-1-carbonyl]-butyl}-amide;
N-{3-Methyl-1-[6-oxo-4-(pyridine-2-sulfonyl)-hexahydro-pyrrolo[3,2-b]pyrrole-1-carbonyl]-butyl}-4-phenoxy-benzamide;
N-{3-Methyl-1-[6-oxo-4-(pyridine-2-sulfonyl)-hexahydro-pyrrolo[3,2-b]pyrrole-1-carbonyl]-butyl}-4-(4-methyl-piperazin-1-yl)-benzamide;
4-Methyl-N-{3-methyl-1-[6-oxo-4-(pyridine-2-sulfonyl)-hexahydro-pyrrolo[3,2-b]pyrrole-1-carbonyl]-butyl}-benzamide;
4-Methoxy-N-{3-methyl-1-[6-oxo-4-(pyridine-2-sulfonyl)-hexahydro-pyrrolo[3,2-b]pyrrole-1-carbonyl]-butyl}-benzamide;
4-Isopropyl-N-{3-methyl-1-[6-oxo-4-(pyridine-2-sulfonyl)-hexahydro-pyrrolo[3,2-b]pyrrole-1-carbonyl]-butyl}-benzamide;
N-{3-Methyl-1-[6-oxo-4-(pyridine-2-sulfonyl)-hexahydro-pyrrolo[3,2-b]pyrrole-1-carbonyl]-butyl}-4-vinyl-benzamide;
4-Imidazol-1-yl-N-{3-methyl-1-[6-oxo-4-(pyridine-2-sulfonyl)-hexahydro-pyrrolo[3,2-b]pyrrole-1-carbonyl]-butyl}-benzamide;
N-{3-Methyl-1-[6-oxo-4-(pyridine-2-sulfonyl)-hexahydro-pyrrolo[3,2-b]pyrrole-1-carbonyl]-butyl}-4-thiophen-2-yl-benzamide;
N-{3-Methyl-1-[6-oxo-4-(pyridine-2-sulfonyl)-hexahydro-pyrrolo[3,2-b]pyrrole-1-carbonyl]-butyl}-4-oxazol-5-yl-benzamide;
5-Phenyl-thiophene-2-carboxylic acid {3-methyl-1-[6-oxo-4-(pyridine-2-sulfonyl)-hexahydro-pyrrolo[3,2-b]pyrrole-1-carbonyl]-butyl}-amide;
N-{3-Methyl-1-[6-oxo-4-(pyridine-2-sulfonyl)-hexahydro-pyrrolo[3,2-b]pyrrole-1-carbonyl]-butyl}-4-trifluoromethoxy-benzamide;
5-Pyridin-2-yl-thiophene-2-carboxylic acid {3-methyl-1-[6-oxo-4-(pyridine-2-sulfonyl)-hexahydro-pyrrolo[3,2-b]pyrrole-1-carbonyl]-butyl}-amide;
4-Difluoromethoxy-N-{3-methyl-1-[6-oxo-4-(pyridine-2-sulfonyl)-hexahydro-pyrrolo[3,2-b]pyrrole-1-carbonyl]-butyl}-benzamide;
N-{3-Methyl-1-[6-oxo-4-(pyridine-2-sulfonyl)-hexahydro-pyrrolo[3,2-b]pyrrole-1-carbonyl]-butyl}-4-morpholin-4-yl-benzamide;

Naphthalene-2-carboxylic acid {1-[6-oxo-4-(pyridine-2-sulfonyl)hexahydro-pyrrolo[3,2-b]pyrrole-1-carbonyl]-butyl}-amide;
Quinoline-2-carboxylic acid {1-[6-oxo-4-(pyridine-2-sulfonyl)-hexahydro-pyrrolo[3,2-b]pyrrole-1-carbonyl]-butyl}-amide;
Benzo[b]thiophene-2-carboxylic acid {1-[6-oxo-4-(pyridine-2-sulfonyl)-hexahydro-pyrrolo[3,2-b]pyrrole-1-carbonyl]-butyl}-amide;
Benzofuran-2-carboxylic acid {1-[6-oxo-4-(pyridine-2-sulfonyl)-hexahydro-pyrrolo[3,2-b]pyrrole-1-carbonyl]-butyl}-amide;
Biphenyl-4-carboxylic acid {1-[6-oxo-4-(pyridine-2-sulfonyl)-hexahydro-pyrrolo[3,2-b]pyrrole-1-carbonyl]-butyl}-amide;
4-tert-Hutyl-N-{1-[6-oxo-4-(pyridine-2-sulfonyl)-hexahydro-pyrrolo[3,2-b]pyrrole-1-carbonyl]-butyl}-benzamide;
4-Dimethylamino-N-{1-[6-oxo-4-(pyridine-2-sulfonyl)-hexahydro-pyrrolo[3,2-b]pyrrole-1-carbonyl]-butyl}-benzamide;
7-Methoxy-benzofuran-2-carboxylic acid {1-[6-oxo-4-(pyridine-2-sulfonyl)-hexahydro-pyrrolo[3,2-b]pyrrole-1-carbonyl]-butyl}-amide;
{1-[6-oxo-4-(pyridine-2-sulfonyl)-hexahydro-pyrrolo[3,2-b]pyrrole-1-carbonyl]-butyl}-carbamic acid benzyl ester;
5-Methoxy-benzofuran-2-carboxylic acid {1-[6-oxo-4-(pyridine-2-sulfonyl)-hexahydro-pyrrolo[3,2-b]pyrrole-1-carbonyl]-butyl}-amide;
Thieno[3,2-b]thiophene-2-carboxylic acid {1-[6-oxo-4-(pyridine-2-sulfonyl)-hexahydro-pyrrolo[3,2-b]pyrrole-1-carbonyl]-butyl}-amide;
3-Methyl-benzofuran-2-carboxylic acid {1-[6-oxo-4-(pyridine-2-sulfonyl)-hexahydro-pyrrolo[3,2-b]pyrrole-1-carbonyl]-butyl}-amide;
Quinoxaline-2-carboxylic acid {1-[6-oxo-4-(pyridine-2-sulfonyl)-hexahydro-pyrrolo[3,2-b]pycrole-1-carbonyl]-butyl}-amide;
Benzo[1,3]dioxole-5-carboxylic acid {1-[6-oxo-4-(pyridine-2-sulfonyl)-hexahydro-pyrrolo[3,2-b]pyrrole-1-carbonyl]-butyl}-amide;
Quinoline-6-carboxylic acid {1-[6-oxo-4-(pyridine-2-sulfonyl)-hexahydro-pyrrolo[3,2-b]pyrrole-1-carbonyl]-butyl}-amide;
Furan-2-carboxylic acid {1-[6-oxo-4-(pyridine-2-sulfonyl)-hexahydro-pyrrolo[3,2-b]pyrrole-1-carbonyl]-butyl}-amide;
Thiophene-2-carboxylic acid {1-[6-oxo-4-(pyridine-2-sulfonyl)-hexahydro-pyrrolo[3,2-b]pyrrole-1-carbonyl]-butyl}-amide;
N-{1-[6-oxo-4-(pyridine-2-sulfonyl)-hexahydro-pyrrolo[3,2-b]pyrcole-1-carbonyl]-butyl}-4-trifluoromethyl-benzamide;
Furan-3-carboxylic acid {1-[6-oxo-4-(pyridine-2-sulfonyl)-hexahydro-pyrrolo[3,2-b]pyrrole-1-carbonyl]-butyl}-amide;
Thiophene-3-carboxylic acid {1-[6-oxo-4-(pyridine-2-sulfonyl)-hexahydro-pyrrolo[3,2-b]pyrrole-1-carbonyl]-butyl}-amide;
N-{1-[6-oxo-4-(pyridine-2-sulfonyl)-hexahydro-pyrrolo[3,2-b]pyrrole-1-carbonyl]-butyl}-4-phenoxy-benzamide;
N-{1-[6-oxo-4-(pyridine-2-sulfonyl)-hexahydro-pyrrolo[3,2-b]pyrrole-1-carbonyl]-butyl}-4-(4-methyl-piperazin-1-yl)-benzamide;
4-Methyl-N-{1-[6-oxo-4-(pyridine-2-sulfonyl)-hexahydro-pyrrolo[3,2-b]pyrrole-1-carbonyl]-butyl}-benzamide;
4-Methoxy-N-{1-[6-oxo-4-(pyridine-2-sulfonyl)-hexahydro-pyrrolo[3,2-b]pyrrole-1-carbonyl]-butyl}-benzamide;
4-Isopropyl-N-{1-[6-oxo-4-(pyridine-2-sulfonyl)-hexahydro-pyrrolo[3,2-b]pyrrole-1-carbonyl]-butyl}-benzamide;
N-{1-[6-oxo-4-(pyridine-2-sulfonyl)-hexahydro-pyrrolo[3,2-b]pyrrole-1-carbonyl]-butyl}-4-vinyl-benzamide;
4-Imidazol-1-yl-N-{1-[6-oxo-4-(pyridine-2-sulfonyl)-hexahydro-pyrrolo[3,2-b]pyrrole-1-carbonyl]-butyl}-benzamide;
N-{1-[6-oxo-4-(pyridine-2-sulfonyl)-hexahydro-pyrrolo[3,2-b]pyrrole-1-carbonyl]-butyl}-4-thiophen-2-yl-benzamide;
N-{1-[6-oxo-4-(pyridine-2-sulfonyl)-hexahydro-pyrrolo[3,2-b]pyrrole-1-carbonyl]-butyl}-4-oxazol-5-yl-benzamide;
5-Phenyl-thiophene-2-carboxylic acid {1-[6-oxo-4-(pyridine-2-sulfonyl)-hexahydro-pyrrolo[3,2-b]pyrrole-1-carbonyl]-butyl}-amide;
N-{1-[6-oxo-4-(pyridine-2-sulfonyl)-hexahydro-pyrrolo[3,2-b]pyrrole-1-carbonyl]-butyl}-4-trifluoromethoxy-benzamide;
5-Pyridin-2-yl-thiophene-2-carboxylic acid {1-[6-oxo-4-(pyridine-2-sulfonyl)-hexahydro-pyrrolo[3,2-b]pyrrole-1-carbonyl]-butyl}-amide;
4-Difluoromethoxy-N-{1-[6-oxo-4-(pyridine-2-sulfonyl)-hexahydro-pyrrolo[3,2-b]pyrrole-1-carbonyl]-butyl}-benzamide;
N-{1-[6-oxo-4-(pyridine-2-sulfonyl)-hexahydro-pyrrolo[3,2-b]pyrrole-1-carbonyl]-butyl}-4-morpholin-4-yl-benzamide;
Naphthalene-2-carboxylic acid {3-methyl-1-[6-oxo-4-(1-oxy-pyridine-2-sulfonyl)hexahydro-pyrrolo[3,2-b]pyrrole-1-carbonyl]-butyl}-amide;

Quinoline-2-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;
Benzo[b]thiophene-2-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;
Benzofuran-2-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;
Biphenyl-4-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;
4-tert-Butyl-N- {3-methyl-1-[6-oxo-4-(1-oxy-pyridine-2-sulfonyl)-hexahydro-pyrrolo[3,2-b]pyrrole-1-carbonyl]-butyl}-benzamide;
4-Dimethylamino-N-{3-methyl-1-[6-oxo-4-(1-oxy-pyridine-2-sulfonyl)-hexahydro-pyrrolo[3,2-b]pyrrole-1-carbonyl]-butyl}-benzamide;
7-Methoxy-benzofuran-2-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;
{3-Methyl-1-[6-oxo-4-(1-oxy-pyridine-2-sulfonyl)-hexahydro-pyrrolo[3,2-b]pyrrole-1-carbonyl]-butyl}-carbamic acid benzyl ester;
5-Methoxy-benzofuran-2-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;
Thieno[3,2-b]thiophene-2-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;
3-Methyl-benzofuran-2-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;
Quinoxaline-2-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;
Benzo[1,3]dioxole-5-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;
Quinoline-6-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;
Furan-2-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;
Thiophene-2-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;
N-{3-Methyl-1-[6-oxo-4-(1-oxy-pyridine-2-sulfonyl)-hexahydro-pyrrolo[3,2-b]pyrrole-1-carbonyl]-butyl}-4-trifluoromethyl-benzamide;
Furan-3-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;
Thiophene-3-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;
N-{3-Methyl-1-[6-oxo-4-(1-oxy-pyridine-2-sulfonyl)-hexahydro-pyrrolo[3,2-b]pyrrole-1-carbonyl]-butyl}-4-phenoxy-benzamide;
N-{3-Methyl-1-[6-oxo-4-(1-oxy-pyridine-2-sulfonyl)-hexahydro-pyrrolo[3,2-b]pyrrole-1-carbonyl]-butyl}-4-(4-methyl-piperazin-1-yl)-benzamide;
4-Methyl-N-{3-methyl-1-[6-oxo-4-(1-oxy-pyridine-2-sulfonyl)-hexahydro-pyrrolo[3,2-b]pyrrole-1-carbonyl]-butyl}-benzamide;
4-Methoxy-N-{3-methyl-1-[6-oxo-4-(1-oxy-pyridine-2-sulfonyl)-hexahydro-pyrrolo[3,2-b]pyrrole-1-carbonyl]-butyl}-benzamide;
4-Isopropyl-N-{3-methyl-1-[6-oxo-4-(1-oxy-pyridine-2-sulfonyl)-hexahydro-pyrrolo[3,2-b]pyrrole-1-carbonyl]-butyl}-benzamide;
N-{3-Methyl-1-[6-oxo-4-(1-oxy-pyridine-2-sulfonyl)-hexahydro-pyrrolo[3,2-b]pyrrole-1-carbonyl]-butyl}-4-vinyl-benzamide;
4-Imidazol-1-yl-N-{3-methyl-1-[6-oxo-4-(1-oxy-pyridine-2-sulfonyl)-hexahydro-pyrrolo[3,2-b]pyrrole-1-carbonyl]-butyl}-benzamide;
N-{3-Methyl-1-[6-oxo-4-(1-oxy-pyridine-2-sulfonyl)-hexahydro-pyrrolo[3,2-b]pyrrole-1-carbonyl]-butyl}-4-thiophen-2-yl-benzamide;
N-{3-Methyl-1-[6-oxo-4-(1-oxy-pyridine-2-sulfonyl)-hexahydro-pyrrolo[3,2-b]pyrrole-1-carbonyl]-butyl}-4-oxaol-5-yl-benzamide;
5-Phenyl-thiophene-2-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;

N-{3-Methyl-1-[6-oxo-4-(1-oxy-pyridine-2-sulfonyl)-hexahydro-pyrrolo[3,2-b]pyrrole-1-carbonyl]-butyl}-4-trifluoromethoxy-benzamide;

5-Pyridin-2-yl-thiophene-2-carboxylic acid {3-methyl-1-[6-oxo-4-(1-oxy-pyridine-2-sulfonyl)-hexahydro-pyrrolo[3,2-b]pyrrole-1-carbonyl]-butyl}-amide;

4-Difluoromethoxy-N-{3-methyl-1-[6-oxo-4-(1-oxy-pyridine-2-sulfonyl)-hexahydro-pyrrlo[3,2-b]pyrrole-1-carbonyl]-butyl}-benzmide;

N-{3-Methyl-1-[6-oxo-4-(1-oxy-pyridine-2-sulfonyl)-hexahydro-pyrrolo[3,2-b]pyrrole-1-carbonyl]-butyl}-4-morpholin-4-yl-benzamide;

Naphthalene-2-carboxylic acid {1-[6-oxo-4-(1-oxy-pyridine-2-sulfonyl)hexahydro-pyrrolo[3,2-b]pyrrole-1-carbonyl]-butyl}-amide;

Quinoline-2-carboxylic acid {1-[6-oxo-4-(1-oxy-pyridine-2-sulfonyl)-hexahydro-pyrrolo[3,2-b]pyrrole-1-carbonyl]-butyl}-amide;

Henzo[b]thiophene-2-carboxylic acid {1-[6-oxo-4-(1-oxy-pyridine-2-sulfonyl)-hexahydro-pyrrolo[3,2-b]pyrrole-1-carbonyl]-butyl}-amide;

Benzofuran-2-carboxylic acid {1-[6-oxo-4-(1-oxy-pyridine-2-sulfonyl)-hexahydro-pyrrolo[3,2-b]pyrrole-1-carbonyl]-butyl}-amide;

Biphenyl-4-carboxylic acid {1-[6-oxo-4-(1-oxy-pyridine-2-sulfonyl)-hexahydro-pyrrolo[3,2-b]pyrrole-1-carbonyl]-butyl}-amide;

4-tert-Butyl-N-{1-[6-oxo-4-(1-oxy-pyridine-2-sulfonyl)-hexahydro-pyrrolo[3,2-b]pyrrole-1-carbonyl]-butyl}-benzamide;

4-Dimethylamino-N-{1-[6-oxo-4-(1-oxy-pyridine-2-sulfonyl)-hexahydro-pyrrolo[3,2-b]pyrrole-1-carbonyl]-butyl}-benzamide;

7-Methoxy-benzofuran-2-carboxylic acid {1-[6-oxo-4-(1-oxy-pyridine-2-sulfonyl)-hexahydro-pyrrolo[3,2-b]pyrrole-1-carbonyl]-butyl}-amide;

{1-[6-oxo-4-(1-oxy-pyridine-2-sulfonyl)-hexahydro-pyrrolo[3,2-b]pyrrole-1-carbonyl]-butyl}-carbamic acid benzyl ester;

5-Methoxy-benzofuran-2-carboxylic acid {1-[6-oxo-4-(1-oxy-pyridine-2-sulfonyl)-hexahydro-pyrrolo[3,2-b]pyrrole-1-carbonyl]-butyl}-amide;

Thieno[3,2-b]thiophene-2-carboxylic acid {1-[6-oxo-4-(1-oxy-pyridine-2-sulfonyl)-hexahydro-pyrrolo[3,2-b]pyrrole-1-carbonyl]-butyl}-amide;

3-Methyl-benzofuran-2-carboxylic acid {1-[6-oxo-4-(1-oxy-pyridine-2-sulfonyl)-hexahydro-pyrrolo[3,2-b]pyrrole-1-carbonyl]-butyl}-amide;

Quinoxaline-2-carboxylic acid {1-[6-oxo-4-(1-oxy-pyridine-2-sulfonyl)-hexahydro-pyrrolo[3,2-b]pyrrole-1-carbonyl]-butyl}-amide;

Benzo[1,3]dioxole-5-carboxylic acid {1-[6-oxo-4-(1-oxy-pyridine-2-sulfonyl)-hexahydro-pyrrolo[3,2-b]pyrrole-1-carbonyl]-butyl}-amide;

Quinoline-6-carboxylic acid {1-[6-oxo-4-(1-oxy-pyridine-2-sulfonyl)-hexahydro-pyrrolo[3,2-b]pyrrole-1-carbonyl]-butyl}-amide;

Furan-2-carboxylic acid {1-[6-oxo-4-(1-oxy-pyridine-2-sulfonyl)-hexahydro-pyrrolo[3,2-b]pyrrole-1-carbonyl]-butyl}-amide;

Thiophene-2-carboxylic acid {1-[6-oxo-4-(1-oxy-pyridine-2-sulfonyl)-hexahydro-pyrrolo[3,2-b]pyrrole-1-carbonyl]-butyl}-amide;

N-{1-[6-oxo-4-(1-oxy-pyridine-2-sulfonyl)-hexahydro-pyrrolo[3,2-b]pyrrole-1-carbonyl]-butyl}-4-trifluoromethyl-benzamide;

Furan-3-carboxylic acid {1-[6-oxo-4-(1-oxy-pyridine-2-sulfonyl)-hexahydro-pyrrolo[3,2-b]pyrrole-1-carbonyl]-butyl}-amide;

Thiophene-3-carboxylic acid {1-[6-oxo-4-(1-oxy-pyridine-2-sulfonyl)-hexahydro-pyrrolo[3,2-b]pyrrole-1-carbonyl]-butyl}-amide;

N-{1-[6-oxo-4-(1-oxy-pyridine-2-sulfonyl)-hexahydro-pyrrolo[3,2-b]pyrrole-1-carbonyl]-butyl}-4-phenoxy-benzamide;

N-{1-[6-oxo-4-(1-oxy-pyridine-2-sulfonyl)-hexahydro-pyrrolo[3,2-b]pyrrole-1-carbonyl]-butyl}-4-(4-methyl-piperazin-1-yl)-benzamide;

4-Methyl-N-{1-[6-oxo-4-(1-oxy-pyridine-2-sulfonyl)-hexahydro-pyrrolo[3,2-b]pyrrole-1-carbonyl]-butyl}-beznzamide;

4-Methoxy-N-{1-[6-oxo-4-(1-oxy-pyridine-2-sulfonyl)-hexahydro-pyrrolo[3,2-b]pyrrole-1-carbonyl]-butyl}-benzamide;

4-Isopropyl-N- {1-[ 6-oxo-4-(1-oxy-pyridine-2-sulfonyl)-hexahydro-pyrrolo[3,2-b]pyrrole-1-carbonyl]-butyl}-benzamide;

N-{1-[6-oxo-4-(1-oxy-pyridine-2-sulfonyl)-hexahydro-pyrrolo[3,2-b]pyrrole-1-carbonyl]-butyl}-4-vinyl-benzamide;
4-Imidazol-1-yl-N-{1-[6-oxo-4-(1-oxy-pyridine-2-sulfonyl)-hexahydro-pyrrolo[3,2-b]pyrrole-1-carbonyl]-butyl}-benzamide;
N-{1-[6-oxo-4-(1-oxy-pyridine-2-sulfonyl)-hexahydro-pyrrolo[3,2-b]pyrrole-1-carbonyl]-butyl}-4-thiophen-2-yl-benzamide;
N-{1-[6-oxo-4-(1-oxy-pyridine-2-sulfonyl)-hexahydro-pyrrolo[3,2-b]pyrrole-1-carbonyl]-butyl}-4-oxazol-5-yl-benzamide;
5-Phenyl-thiophene-2-carboxylic acid {1-[6-oxo-4-(1-oxy-pyridine-2-sulfonyl)-hexahydro-pyrrolo[3,2-b]pyrrole-1-carbonyl]-butyl}-amide;
N-{1-[6-oxo-4-(1-oxy-pyridine-2-sulfonyl)-hexahydro-pyrrolo[3,2-b]pyrrole-1-carbonyl]-butyl}-4-trifluoromethoxy-benzamide;
5-Pyridin-2-yl-thiophene-2-carboxylic acid {1-[6-oxo-4-(1-oxy-pyridine-2-sulfonyl)-hexahydro-pyrrolo[3,2-b]pyrrole-1-carbonyl]-butyl}-amide;
4-Difluoromethoxy-N-{1-[6-oxo-4-(1-oxy-pyridine-2-sulfonyl)-hexahydro-pyrrolo[3,2-b]pyrrole-1-carbonyl]-butyl}-benzamide;
N-{1-[6-oxo-4-(1-oxy-pyridine-2-sulfonyl)-hexahydro-pyrrolo[3,2-b]pyrrole-1-carbonyl]-butyl}-4-morpholin-4-yl-benzamide;
Naphthalene-2-carboxylic acid {3-methyl-1-[6-oxo-4-(pyridine-3-sulfonyl)hexahydro-pyrrolo[3,2-b]pyrrole-1-carbonyl]-butyl}-amide;
Quinoline-2-carboxylic acid {3-methyl-1-[6-oxo-4-(pyridine-3-sulfonyl)-hexahydro-pyrrolo[3,2-b]pyrrole-1-carbonyl]-butyl}-amide;
Benzo[b]thiophene-2-carboxylic acid {3-methyl-1-[6-oxo4-(pyridine-3-sulfonyl)-hexahydro-pyrrolo[3,2-b]pyrrole-1-carbonyl]-butyl}-amide;
Benzofuran-2-carboxylic acid {3-methyl-1-[6-oxo-4-(pyridine-3-sulfonyl)-hexahydro-pyrrolo[3,2-b]pyrrole-1-carbonyl]-butyl}-amide;
Biphenyl-4-carboxylic acid {3-methyl-1-[6-oxo-4-(pyridine-3-sulfonyl)-hexahydro-pyrrolo[3,2-b]pyrrole-1-carbonyl]-butyl}-amide;
4-tert-Butyl-N-{3-methyl-1-[6-oxo-4-(pyridine-3-sulfonyl)-hexahydro-pyrrolo[3,2-b]pyrrole-1-carbonyl]-butyl}-benzamide;
4-Dimethylamino-N-{3-methyl-1-[6-oxo-4-(pyridine-3-sulfonyl)-hexahydro-pyrrolo[3,2-b]pyrrole-1-carbonyl]-butyl}-benzamide;
7-Methoxy-benzofuran-2-carboxylic acid {3-methyl-1-[6-oxo-4-(pyridine-3-sulfonyl)-hexahydro-pyrrolo[3,2-b]pyrrole-1-carbonyl]-butyl}-amide;
{3-Methyl-1-[6-oxo-4-(pyridine-3-sulfonyl)-hexahydro-pyrrolo[3,2-b]pyrrole-1-carbonyl]-butyl}-carbamic acid benzyl ester;
5-Methoxy-benzofuran-2-carboxylic acid {3-methyl-1-[6-oxo-4-(pyridine-3-sulfonyl)-hexahydro-pyrrolo[3,2-b]pyrrole-1-carbonyl]-butyl}-amide;
Thieno[3,2-b]thiophene-2-carboxylic acid {3-methyl-1-[6-oxo-4-(pyridine-3-sulfonyl)-hexahydro-pyrrolo[3,2-b]pyrrole-1-carbonyl]-butyl}-amide;
3-Methyl-benzofuran-2-carboxylic acid {3-methyl-1-[6-oxo-4-(pyridine-3-sulfonyl)-hexahydro-pyrrolo[3,2-b]pyrrole-1-carbonyl]-butyl}-amide;
Quinoxaline-2-carboxylic acid {3-methyl-1-[6-oxo-4-(pyridine-3-sulfonyl)-hexahydro-pyrrolo[3,2-b]pyrrole-1-carbonyl]-butyl}-amide;
Benzo[1,3]dioxole-5-carboxylic acid {3-methyl-1-[6-oxo4-(pyridine-3-sulfonyl)-hexahydro-pyrrolo[3,2-b]pyrrole-1-carbonyl]-butyl}-amide;
Quinoline-6-carboxylic acid {3-methyl-1-[6-oxo-4-(pyridine-3-sulfonyl)-hexahydro-pyrrolo[3,2-b]pyrrole-1-carbonyl]-butyl}-amide;
Furan-2-carboxylic acid {3-methyl-1-[6-oxo-4-(pyridine-3-sulfonyl)-hexahydro-pyrrolo[3,2-b]pyrrole-1-carbonyl]-butyl}-amide;
Thiophene-2-carboxylic acid {3-methyl-1-[6-oxo-4-(pyridine-3-sulfonyl)-hexahydro-pyrrolo[3,2-b]pyrrole-1-carbonyl]-butyl}-amide;
N-{3-Methyl-1-[6-oxo-4-(pyridine-3-sulfonyl)-hexahydro-pyrrolo[3,2-b]pyrrole-1-carbonyl]-butyl}-4-trifluoromethyl-benzamide;
Furan-3-carboxylic acid {3-methyl-1-[6-oxo-4-(pyridine-3-sulfonyl)-hexahydro-pyrrolo[3,2-b]pyrrole-1-carbonyl]-butyl}-amide;
Thiophene-3-carboxylic acid {3-methyl-1-[6-oxo-4-(pyridine-3-sulfonyl)-hexahydro-pyrrolo[3,2-b]pyrrole-1-carbonyl]-butyl}-amide;
N-{3-Methyl-1-[6-oxo-4-(pyridine-3-sulfonyl)-hexahydro-pyrrolo[3,2-b]pyrrole-1-carbonyl]-butyl}-4-phenoxy-benzamide;
N-{3-Methyl-1-[6-oxo-4-(pyridine-3-sulfonyl)-hexahydro-pyrrolo[3,2-b]pyrrole-1-carbonyl]-butyl}-4-(4-methyl-piperazin-1-yl)-benzamide;
4-Methyl-N-{3-methyl-1-[6-oxo-4-(pyridine-3-sulfonyl)-hexahydro-pyrrolo[3,2-b]pyrrole-1-carbonyl]-butyl}-benzamide;
4-Methoxy-N-{3-methyl-1-[6-oxo-4-(pyridine-3-sulfonyl)-hexahydro-pyrrolo[3,2-b]pyrrole-1-carbonyl]-butyl}-benzamide;
4-Isopropyl-N-{3-methyl-1-[6-oxo4-(pyridine-3-sulfonyl)-hexahydro-pyrrolo[3,2-b]pyrrole-1-carbonyl]-butyl}-benzamide;
N-{3-Methyl-1-[6-oxo-4-(pyridine-3-sulfonyl)-hexahydro-pyrrolo[3,2-b]pyrrole-1-carbonyl]-butyl}-4-vinyl-benzamide;
4-Imidazol-1-yl-N-{3-methyl-1-[6-oxo-4-(pyridine-3-sulfonyl)-hexahydro-pyrrolo[3,2-b]pyrrole-1-carbonyl]-butyl}-benzamide;
N-{3-Methyl-1-[6-oxo-4-(pyridine-3-sulfonyl)-hexahydro-pyrrolo[3,2-b]pyrrole-1-carbonyl]-butyl}-4-thiophen-2-yl-benzamide;
N-{3-Methyl-1-[6-oxo-4-(pyridine-3-sulfonyl)-hexahydro-pyrrolo[3,2-b]pyrrole-1-carbonyl]-butyl}-4-oxazol-5-yl-benzamide;
5-Phenyl-thiophene-2-carboxylic acid {3-methyl-1-[6-oxo-4-(pyridine-3-sulfonyl)-hexahydro-pyrrolo[3,2-b]pyrrole-1-carbonyl]-butyl}-amide;
N-{3-Methyl-1-[6-oxo-4-(pyridine-3-sulfonyl)-hexahydro-pyrrolo[3,2-b]pyrrole-1-carbonyl]-butyl}-4-trifluoromethoxy-benzamide;
5-Pyridin-2-yl-thiophene-2-carboxylic acid {3-methyl-1-[6-oxo-4-(pyridine-3-sulfonyl)-hexahydro-pyrrolo[3,2-b]pyrrole-1-carbonyl]-butyl}-amide;
4-Difluoromethoxy-N-{3-methyl-1-[6-oxo-4-(pyridine-3-sulfonyl)-hexahydro-pyrrolo[3,2-b]pyrrole-1-carbonyl]-butyl}-benzamide;
N-{3-Methyl-1-[6-oxo-4-(pyridine-3-sulfonyl)-hexahydro-pyrrolo[3,2-b]pyrrole-1-carbonyl]-butyl}-4-morpholin-4-yl-benzamide;
Naphthalene-2-carboxylic acid {1-[6-oxo-4-(pyridine-3-sulfonyl)hexahydro-pyrrolo[3,2-b]pyrrole-1-carbonyl]-butyl}-amide;
Quinoline-2-carboxylic acid {1-[6-oxo-4-(pyridine-3-sulfonyl)-hexahydro-pyrrolo[3,2-b]pyrrole-1-carbonyl]-butyl}-amide;
Benzo[b]thiophene-2-carboxylic acid {1-[6-oxo-4-(pyridine-3-sulfonyl)-hexahydro-pyrrolo[3,2-b]pyrrole-1-carbonyl]-butyl}-amide;
Benzofuran-2-carboxylic acid {1-[6-oxo-4-(pyridine-3-sulfonyl)-hexahydro-pyrrolo[3,2-b]pyrrole-1-carbonyl]-butyl}-amide;
Biphenyl-4-carboxylic acid {1-[6-oxo-4-(pyridine-3-sulfonyl)-hexahydro-pyrrolo[3,2-b]pyrrole-1-carbonyl]-butyl}-amide;
4-tert-Butyl-N-{1-[6-oxo-4-(pyridine-3-sulfonyl)-hexahydro-pyrrolo[3,2-b]pyrrole-1-carbonyl]-butyl}-benzamide;
4-Dimethylamino-N-{1-[6-oxo-4-(pyridine-3-sulfonyl)-hexahydro-pyrrolo[3,2-b]pyrrole-1-carbonyl]-butyl}-benzamide;
7-Methoxy-benzofuran-2-carboxylic acid {1-[6-oxo-4-(pyridine-3-sulfonyl)-hexahydro-pyrrolo[3,2-b]pyrrole-1-carbonyl]-butyl}-amide;
{1-[6-oxo-4-(pyridine-3-sulfonyl)-hexahydro-pyrrolo[3,2-b]pyrrole-1-carbonyl]-butyl}-carbamic acid benzyl ester;
5-Methoxy-benzofuran-2-carboxylic acid {1-[6-oxo-4-(pyridine-3-sulfonyl)-hexahydro-pyrrolo[3,2-b]pyrrole-1-carbonyl]-butyl}-amide;
Thieno[3,2-b]thiophene-2-carboxylic acid {1-[6-oxo-4-(pyridine-3-sulfonyl)-hexahydro-pyrrolo[3,2-b]pyrrole-1-carbonyl]-butyl}-amide;
3-Methyl-benzofuran-2-carboxylic acid {1-[6-oxo-4-(pyridine-3-sulfonyl)-hexahydro-pyrrolo[3,2-b]pyrrole-1-carbonyl]-butyl}-amide;
Quinoxaline-2-carboxylic acid {1-[6-oxo-4-(pyridine-3-sulfonyl)-hexahydro-pyrrolo[3,2-b]pyrrole-1-carbonyl]-butyl}-amide;
Benzo[1,3]dioxole-5-carboxylic acid {1-[6-oxo-4-(pyridine-3-sulfonyl)-hexahydro-pyrrolo[3,2-b]pyrrole-1-carbonyl]-butyl}-amide;
Quinoline-6-carboxylic acid {1-[6-oxo-4-(pyridine-3-sulfonyl)-hexahydro-pyrrolo[3,2-b]pyrrole-1-carbonyl]-butyl}-amide;
Furan-2-carboxylic acid {1-[6-oxo-4-(pyridine-3-sulfonyl)-hexahydro-pyrrolo[3,2-b]pyrrole-1-carbonyl]-butyl}-amide;
Thiophene-2-carboxylic acid {1-[6-oxo-4-(pyridine-3-sulfonyl)-hexahydro-pyrrolo[3,2-b]pyrrole-1-carbonyl]-butyl}-amide;

N-{1-[6-oxo-4-(pyridine-3-sulfonyl)-hexahydro-pyrrolo[3,2-b]pyrrole-1-carbonyl]-butyl}-4-trifluoromethyl-benzamide;
Furan-3-carboxylic acid {1-[6-oxo-4-(pyridine-3-sulfonyl)-hexahydro-pyrrolo[3,2-b]pyrrole-1-carbonyl]-butyl}-amide;
Thiophene-3-carboxylic acid {1-[6-oxo-4-(pyridine-3-sulfonyl)-hexahydro-pyrrolo[3,2-b]pyrrole-1-carbonyl]-butyl}-amide;
N-{1-[6-oxo-4-(pyridine-3-sulfonyl)-hexahydro-pyrrolo[3,2-b]pyrrole-1-carbonyl]-butyl}-4-phenoxy-benzamide;
N-{1-[6-oxo-4-(pyridine-3-sulfonyl)-hexahydro-pyrrolo[3,2-b]pyrrole-1-carbonyl]-butyl}-4-(4-methyl-piperazin-1-yl)-benzamide;
4-Methyl-N-{1-[6-oxo-4-(pyridine-3-sulfonyl)-hexahydro-pyrrolo[3,2-b]pyrrole-1-carbonyl]-butyl}-benzamide;
4-Methoxy-N-{1-[6-oxo-4-(pyridine-3-sulfony)-hexahydro-pyrrolo[3,2-b]pyrrole-1-carbonyl]-butyl}-benzamide;
4-Isopropyl-N-{1-[6-oxo-4-(pyridine-3-sulfonyl)-hexahydro-pyrrolo[3,2-b]pyrrole-1-carbonyl]-butyl}-benzamide;
N-{1-[6-oxo-4-(pyridine-3-sulfonyl)-hexahydro-pyrrolo[3,2-b]pyrrole-1-carbonyl]-butyl}-4-vinyl-benzamide;
4-Imidazol-1-yl-N-{1-[6-oxo-4-(pyridine-3-sulfonyl)-hexahydro-pyrrolo[3,2-b]pyrrole-1-carbonyl]-butyl}-benxmide;
N-{1-[6-oxo-4-(pyridine-3-sulfonyl)-hexahydro-pyrrolo[3,2-b]pyrrole-1-carbonyl]-butyl}-4-thiophen-2-yl-benzamide;
N-{1-[6-oxo-4-(pyridine-3-sulfonyl)-hexahydro-pyrrolo[3,2-b]pyrrole-1-carbonyl]-butyl}-4-oxazol-5-yl-benzamide;
5-Phenyl-thiophene-2-carboxylic acid {1-[6-oxo-4-(pyridine-3-sulfonyl)-hexahydro-pyrrolo[3,2-b]pyrrole-1-carbonyl]-butyl}-amide;
N-{1-[6-oxo-4-(pyridine-3-sulfonyl)-hexahydro-pyrrolo[3,2-b]pyrrole-1-carbonyl]-butyl}-4-trifluoromethoxy-benzamide;
5-Pyridin-2-yl-thiophene-2-carboxylic acid {1-[6-oxo-4-(pyridine-3-sulfonyl)-hexahydro-pyrrolo[3,2-b]pyrrole-1-carbonyl]-butyl}-amide;
4-Difluoromethoxy-N-{1-[6-oxo-4-(pyridine-3-sulfonyl)-hexahydro-pyrrolo[3,2-b]pyrrole-1-carbonyl]-butyl}-benzamide;
N-{1-[6-oxo-4-(pyridine-3-sulfonyl)-hexahydro-pyrrolo[3,2-b]pycrole-1-carbonyl]-butyl}-4-morpholin-4-yl-benzamide;
Naphthalene-2-carboxylic acid {3-methyl-1-[6-oxo-4-(1-oxy-pyridine-3-sulfonyl)hexahydro-pyrrolo[3,2-b]pyrrole-1-carbonyl]-butyl}-amide;
Quinoline-2-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
Benzo[b]thiophene-2-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
Benzofuran-2-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
Biphenyl-4-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
4-tert-Butyl-N-{3-methyl-1-[6-oxo-4-(1-oxy-pyridine-3-sulfonyl)-hexahydro-pyrrolo[3,2-b]pyrrole-1-carbonyl]-butyl}-benzamide;
4-Dimethylamino-N-{3-methyl-1-[6-oxo-4-(1-oxy-pyridine-3-sulfonyl)-hexahydro-pyrrolo[3,2-b]pyrrole-1-carbonyl]-butyl}-benzamide;
7-Methoxy-benzofuran-2-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
{3-Methyl-1-[6-oxo-4-(1-oxy-pyridine-3-sulfonyl)-hexahydro-pyrrolo[3,2-b]pyrrole-1-carbonyl]-butyl}-carbamic acid benzyl ester;
5-Methoxy-benzofuran-2-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
Thieno[3,2-b]thiophene-2-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
3-Methyl-benzofuran-2-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
Quinoxaline-2-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
Benzo[1,3]dioxole-5-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
Quinoline-6-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
Furan-2-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
Thiophene-2-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
N-{3-Methyl-1-[6-oxo-4-(1-oxy-pyridine-3-sulfonyl)-hexahydro-pyrrolo[3,2-b]pyrrole-1-carbonyl]-butyl}-4-trifluorom ethyl-benzamide;
Furan-3-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
Thiophene-3-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
N-{3-Methyl-1-[6-oxo-4-(1-oxy-pyridine-3-sulfonyl)-hexahydro-pyrrolo[3,2-b]pyrrole-1-carbonyl]-butyl}-4-phenoxy-benzamide;
N-{3-Methyl-1-[6-oxo-4-(1-oxy-pyridine-3-sulfonyl)-hexahydro-pyrrolo[3,2-b]pyrrole-1-carbonyl]-butyl}-4-(4-methyl-piperazin-1-yl)-benzamide;
4-Methyl-N-{3-methyl-1-[6-oxo-4-(1-oxy-pyridine-3-sulfonyl)-hexahydro-pyrrolo[3,2-b]pyrrole-1-carbonyl]-butyl}-benzamide;
4-Methoxy-N-{3-methyl-1-[6-oxo-4-(1-oxy-pyridine-3-sulfonyl)-hexahydro-pyrrolo[3,2-b]pyrrole-1-carbonyl]-butyl}-benzamide;
4-Isopropyl-N-{3-methyl-1-[6-oxo-4-(1-oxy-pyridine-3-sulfonyl)-hexahydro-pyrrolo[3,2-b]pyrrole-1-carbonyl]-butyl}-benzamide;
N-{3-Methyl-1-[6-oxo-4-(1-oxy-pyridine-3-sulfonyl)-hexahydro-pyrrolo[3,2-b]pyrrole-1-carbonyl]-butyl}-4-vinyl-benzamide;
4-Imidazol-1-yl-N-{3-methyl-1-[6-oxo-4-(1-oxy-pyridine-3-sulfonyl)-hexahydro-pyrrolo[3,2-b]pyrrole-1-carbonyl]-bu tyl}-benzamide;
N-{3-Methyl-1-[6-oxo-4-(1-oxy-pyridine-3-sulfonyl)-hexahydro-pyrrolo[3,2-b]pyrrole-1-carbonyl]-butyl}-4-thiophen-2-yl-benzamide;
N-{3-Methyl-1-[6-oxo-4-(1-oxy-pyridine-3-sulfonyl)-hexahydro-pyrrolo[3,2-b]pyrrole-1-carbonyl]-butyl}-4-oxazol-5-yl-benzamide;
5-Phenyl-thiophene-2-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
N-{3-Methyl-1-[6-oxo-4-(1-oxy-pyridine-3-sulfonyl)-hexahydro-pyrrolo[3,2-b]pyrrole-1-carbonyl]-butyl}-4-trifluorom ethoxy-benzamide;
5-Pyridin-2-yl-thiophene-2-carboxylic acid {3-methyl-1-[6-oxo-4-(1-oxy-pyridine-3-sulfonyl)-hexahydro-pyrrolo[3,2-b]pyrrole-1-carbonyl]-butyl}-amide;
4-Difluoromethoxy-N-{3-methyl-1-[6-oxo-4-(1-oxy-pyridine-3-sulfonyl)-hexahydro-pyrrolo[3,2-b]pyrrole-1-carbonyl] -butyl}-benzamide;
N-{3-Methyl-1-[6-oxo-4-(1-oxy-pyridine-3-sulfonyl)-hexahydro-pyrrolo[3,2-b]pyrrole-1-carbonyl]-butyl)-4-morpholin-4-yl-bemamide;
Naphthalene-2-carboxylic acid {1-[6-oxo-4-(1-oxy-pyridine-3-sulfonyl)hexahydro-pyrrolo[3,2-b]pyrrole-1-carbonyl]-butyl}-amide;
Quinoline-2-carboxylic acid {1-[6-oxo-4-(1-oxy-pyridine-3-sulfonyl)-hexahydro-pyrrolo[3,2-b]pyrrole-1-carbonyl]-butyl}-amide;
Benzo[b]thiophene-2-carboxylic acid {1-[6-oxo-4-(1-oxy-pyridine-3-sulfonyl)-hexahydro-pyrrolo[3,2-b]pyrrole-1-carbonyl]-butyl}-amide;
Benzofuran-2-carboxylic acid {1-[6-oxo-4-(1-oxy-pyridine-3-sulfonyl)-hexahydro-pyrrolo[3,2-b]pyrrole-1-carbonyl]-butyl}-amide;
Biphenyl-4-carboxylic acid {1-[6-oxo-4-(1-oxy-pyridine-3-sulfonyl)-hexahydro-pyrrolo[3,2-b]pyrrole-1-carbonyl]-butyl}-amide;
4-tert-Butyl-N-{1-[6-oxo-4-(1-oxy-pyridine-3-sulfonyl)-hexahydro-pyrrolo[3,2-b]pyrrole-1-carbonyl]-butyl}-benzamide;
4-Dimethylamino-N-{1-[6-oxo-4-(1-oxy-pyridine-3-sulfonyl)-hexahydro-pyrrolo[3,2-b]pyrrole-1-carbonyl]-butyl}-benzamide;
7-Methoxy-benzofuran-2-carboxylic acid {1-[6-oxo-4-(1-oxy-pyridine-3-sulfonyl)-hexahydro-pyrrolo[3,2-b]pyrrole-1-carbonyl]-butyl}-amide;
{1-[6-oxo-4-(1-oxy-pyridine-3-sulfonyl)-hexahydro-pyrrolo[3,2-b]pyrrole-1-carbonyl]-butyl}-carbamic acid benzyl ester;
5-Methoxy-benzofuran-2-carboxylic acid {1-[6-oxo-4-(1-oxy-pyridine-3-sulfonyl)-hexahydro-pyrrolo[3,2-b]pyrrole-1-carbonyl]-butyl}-amide;
Thieno[3,2-b]thiophene-2-carboxylic acid {1-[6-oxo-4-(1-oxy-pyridine-3-sulfonyl)-hexahydro-pyrrolo[3,2-b]pyrrole-1-carbonyl]-butyl}-amide;
3-Methyl-benzofuran-2-carboxylic acid {1-[6-oxo-4-(1-oxy-pyridine-3-sulfonyl)-hexahydro-pyrrolo[3,2-b]pyrrole-1-carbonyl]-butyl}-amide;
Quinoxaline-2-carboxylic acid {1-[6-oxo-4-(1-oxy-pyridine-3-sulfonyl)-hexahydro-pyrrolo[3,2-b]pyrrole-1-carbonyl]-butyl}-amide;
Benzo[1,3]dioxole-5-carboxylic acid {1-[6-oxo-4-(1-oxy-pyridine-3-sulfonyl)-hexahydro-pyrrolo[3,2-b]pyrrole-1-carbonyl]-buty}-amide;
Quinoline-6-carboxylic acid {1-[6-oxo-4-(1-oxy-pyridine-3-sulfonyl)-hexahydro-pyrrolo[3,2-b]pyrrole-1-carbonyl]-butyl}-amide;
Furan-2-carboxylic acid {1-[6-oxo-4-(1-oxy-pyridine-3-sulfonyl)-hexahydro-pyrrolo[3,2-b]pyrrole-1-carbonyl]-butyl}-amide;
Thiophene-2-carboxylic acid {1-[6-oxo-4-(1-oxy-pyridine-3-sulfonyl)-hexahydro-pyrrolo[3,2-b]pyrrole-1-carbonyl]-butyl}-amide;
N-{1-[6-oxo-4-(1-oxy-pyridine-3-sulfonyl)-hexahydro-pyrrolo[3,2-b]pyrrole-1-carbonyl]-butyl}-4-trifluoromethyl-benzamide;
Furan-3-carboxylic acid {1-[6-oxo-4-(1-oxy-pyridine-3-sulfonyl)-hexahydro-pyrrolo[3,2-b]pyrrole-1-carbonyl]-butyl}-amide;
Thiophene-3-carboxylic acid {1-[6-oxo-4-(1-oxy-pyridine-3-sulfonyl)-hexahydro-pyrrolo[3,2-b]pyrrole-1-carbonyl]-butyl}-amide;
N-{1-[6-oxo-4-(1-oxy-pyridine-3-sulfonyl)-hexahydro-pyrrolo[3,2-b]pyrrole-1-carbonyl]-butyl}-4-phenoxy-benzamide;
N-{1-[6-oxo-4-(1-oxy-pyridine-3-sulfonyl)-hexahydro-pyrrolo[3,2-b]pyrrole-1-carbonyl]-butyl}-4-(4-methyl-piperazin-1-yl)-benzamide;
4-Methyl-N- {1-[6-oxo-4-(1-oxy-pyridine-3-sulfonyl)-hexahydro-pyrrolo[3,2-b]pyrrole-1-carbonyl]-butyl}-benzamide;
4-Methoxy-N-{1-[6-oxo-4-(1-oxy-pyridine-3-sulfonyl)-hexahydro-pyrrolo[3,2-b]pyrrole-1-carbonyl]-butyl}-benzamide;
4-Isopropyl-N-{1-[6-oxo-4-(1-oxy-pyridine-3-sulfonyl)-hexahydro-pyrrolo[3,2-b]pyrrole-1-carbonyl]-butyl}-benzamide;
N-{1-[6-oxo-4-(1-oxy-pyridine-3-sulfonyl)-hexahydro-pyrrolo[3,2-b]pyrrole-1-carbonyl]-butyl}-4-vinyl-benzamide;
4-Imidazol-1-yl-N-{1-[6-oxo-4-(1-oxy-pyridine-3-sulfonyl)-hexahydro-pyrrolo[3,2-b]pyrrole-1-carbonyl]-butyl}-benzamide;
N-{1-[6-oxo-4-(1-oxy-pyridine-3-sulfonyl)-hexahydro-pyrrolo[3,2-b]pyrrole-1-carbonyl]-butyl}-4-thiophen-2-yl-benzamide;
N-{1-[6-oxo-4-(1-oxy-pyridine-3-sulfonyl)-hexahydro-pyrrolo[3,2-b]pyrrole-1-carbonyl]-butyl}-4-oxazol-5-yl-benzamide;
5-Phenyl-thiophene-2-carboxylic acid {1-[6-oxo-4-(1-oxy-pyridine-3-sulfonyl)-hexahydro-pyrrolo[3,2-b]pyrrole-1-carbonyl]-butyl}-amide;
N-{1-[6-oxo-4-(1-oxy-pyridine-3-sulfonyl)-hexahydro-pyrrolo[3,2-b]pyrrole-1-carbonyl]-butyl}-4-trifluoromethoxy-benzamide;
5-Pyridin-2-yl-thiophene-2-carboxylic acid {1-[6-oxo-4-(1-oxy-pyridine-3-sulfonyl)-hexahydro-pyrrolo[3,2-b]pyrrole-1-carbonyl]-butyl}-amide;
4-Difluoromethoxy-N-{1-[6-oxo-4-(1-oxy-pyridine-3-sulfonyl)-hexahydro-pyrrolo[3,2-b]pyrrole-1-carbonyl]-butyl}-benzamide;
N-{1-[6-oxo-4-(1-oxy-pyridine-3-sulfonyl)-hexahydro-pyrrolo[3,2-b]pyrrole-1-carbonyl]-butyl}-4-morpholin-4-yl-benzamide;
Naphthalene-2-carboxylic acid {3-methyl-1-[6-oxo-4-(pyridine-2-carbonyl)hexahydro-pyrrolo[3,2-b]pyrrole-1-carbonyl]-butyl}-amide;
Quinoline-2-carboxylic acid {3-methyl-1-[6-oxo-4-(pyridine-2-carbonyl)-hexahydro-pyrrolo[3,2-b]pyrrole-1-carbonyl]-butyl}-amide;
Benzo[b]thiophene-2-carboxylic acid {3-methyl-1-[6-oxo-4-(pyridine-2-carbonyl)-hexahydro-pyrrolo[3,2-b]pyrrole-1-carbonyl]-butyl}-amide;
Benzofuran-2-carboxylic acid {3-methyl-1-[6-oxo-4-(pyridine-2-carbonyl)-hexahydro-pyrrolo[3,2-b]pyrrole-1-carbonyl]-butyl}-amide;
Biphenyl-4-carboxylic acid {3-methyl-1-[6-oxo-4-(pyridine-2-carbonyl)-hexahydro-pyrrolo[3,2-b]pyrrole-1-carbonyl]-butyl}-amide;
4-tert-Butyl-N-{3-methyl-1-[6-oxo-4-(pyridine-2-carbonyl)-hexahydro-pyrrolo[3,2-b]pyrrole-1-carbonyl]-butyl}-benzamide;
4-Dimethylamino-N-{3-methyl-1-[6-oxo-4-(pyridine-2-carbonyl)-hexahydro-pyrrolo[3,2-b]pyrrole-1-carbonyl]-butyl}-benzamide;
7-Methoxy-benzofuran-2-carboxylic acid {3-methyl-1-[6-oxo-4-(pyridine-2-carbonyl)-hexahydro-pyrrolo[3,2-b]pyrrole-1-carbonyl]-butyl}-amide;
{3-Methyl-1-[6-oxo-4-(pyridine-2-carbonyl)-hexahydro-pyrrolo[3,2-b]pyrrole-1-carbonyl]-butyl}-carbamic acid benzyl ester;
5-Methoxy-benzofuran-2-carboxylic acid {3-methyl-1-[6-oxo-4-(pyridine-2-carbonyl)-hexahydro-pyrrolo[3,2-b]pyrrole-1-carbonyl]-butyl}-amide;
Thieno[3,2-b]thiophene-2-carboxylic acid {3-methyl-1-[6-oxo-4-(pyridine-2-carbonyl)-hexahydro-pyrrolo[3,2-b]pyrrole-1-carbonyl]-butyl}-amide;
3-Methyl-benzofuran-2-carboxylic acid {3-methyl-1-[6-oxo-4-(pyridine-2-carbonyl)-hexahydro-pyrrolo[3,2-b]pyrrole-1-carbonyl]-butyl}-amide;
Quinoxaline-2-carboxylic acid {3-methyl-1-[6-oxo-4-(pyridine-2-carbonyl)-hexahydro-pyrrolo[3,2-b]pyrrole-1-carbonyl]-butyl}-amide;
Benzo[1,3]dioxole-5-carboxylic acid {3-methyl-1-[6-oxo-4-(pyridine-2-carbonyl)-hexahydro-pyrrolo[3,2-b]pyrrole-1-carbonyl]-butyl}-amide;
Quinoline-6-carboxylic acid {3-methyl-1-[6-oxo-4-(pyridine-2-carbonyl)-hexahydro-pyrrolo[3,2-b]pyrrole-1-carbonyl]-butyl}-amide;
Furan-2-carboxylic acid {3-methyl-1-[6-oxo-4-(pyridine-2-carbonyl)-hexahydro-pyrrolo[3,2-b]pyrrole-1-carbonyl]-butyl}-amide;
Thiophene-2-carboxylic acid {3-methyl-1-[6-oxo-4-(pyridine-2-carbonyl)-hexahydro-pyrrolo[3,2-b]pyrrole-1-carbonyl]-butyl}-amide;
N-{3-Methyl-1-[6-oxo-4-(pyridine-2-carbonyl)-hexahydro-pyrrolo[3,2-b]pyrrole-1-carbonyl]-butyl}-4-trifluoromethyl-benzamide;
Furan-3-carboxylic acid {3-methyl-1-[6-oxo-4-(pyridine-2-carbonyl)-hexahydro-pyrrolo[3,2-b]pyrrole-1-carbonyl]-butyl}-amide;
Thiophene-3-carboxylic acid {3-methyl-1-[6-oxo-4-(pyridine-2-carbonyl)-hexahydro-pyrrolo[3,2-b]pyrrole-1-carbonyl]-butyl}-amide;
N-{3-Methyl-1-[6-oxo-4-(pyridine-2-carbonyl)-hexahydro-pyrrolo[3,2-b]pyrrole-1-carbonyl]-butyl}-4-phenoxy-benzamide;
N-{3-Methyl-1-[6-oxo-4-(pyridine-2-carbonyl)-hexahydro-pyrrolo[3,2-b]pyrrole-1-carbonyl]-butyl}-4-(4-methyl-piper azin-1-yl)-benzamide;
4-Methyl-N-{3-methyl-1-[6-oxo-4-(pyridine-2-carbonyl)-hexahydro-pyrrolo[3,2-b]pyrrole-1-carbonyl]-butyl}-benzamide;
4-Methoxy-N-{3-methyl-1-[6-oxo-4-(pyridine-2-carbonyl)-hexahydro-pyrrolo[3,2-b]pyrrole-1-carbonyl]-butyl}-benzamide;
4-Isopropyl-N-{3-methyl-1-[6-oxo-4-(pyridine-2-carbonyl)-hexahydro-pyrrolo[3,2-b]pyrrole-1-carbonyl]-butyl}-benzamide;
N-{3-Methyl-1-[6-oxo-4-(pyridine-2-carbonyl)-hexahydro-pyrrolo[3,2-b]pyrrole-1-carbonyl]-butyl}-4-vinyl-benzamide;
4-Imidazol-1-yl-N-{3-methyl-1-[6-oxo-4-(pyridine-2-carbonyl)-hexahydro-pyrrolo[3,2-b]pyrrole-1-carbonyl]-butyl}-benzamide;
N-{3-Methyl-1-[6-oxo-4-(pyridine-2-carbonyl)-hexahydro-pyrrolo[3,2-b]pyrrole-1-carbonyl]-butyl}-4-thiophen-2-yl-benzamide;
N-{3-Methyl-1-[6-oxo-4-(pyridine-2-carbonyl)-hexahydro-pyrrolo[3,2-b]pyrrole-1-carbonyl]-butyl}-4-oxazol-5-yl-benzamide;
5-Phenyl-thiophene-2-carboxylic acid {3-methyl-1-[6-oxo-4-(pyridine-2-carbonyl)-hexahydro-pyrrolo[3,2-b]pyrrole-1-carbonyl]-butyl}-amide;
N-{3-Methyl-1-[6-oxo-4-(pyridine-2-carbonyl)-hexahydro-pyrrolo[3,2-b]pyrrole-1-carbonyl]-butyl}-4-trifluoromethoxy-benzamide;
5-Pyridin-2-yl-thiophene-2-carboxylic acid {3-methyl-1-[6-oxo-4-(pyridine-2-carbonyl)-hexahydro-pyrrolo[3,2-b]pyrrole-1-carbonyl]-butyl}-amide;
4-Difluoromethoxy-N-{3-methyl-1-[6-oxo-4-(pyridine-2-carbonyl)-hexahydro-pyrrolo[3,2-b]pyrrole-1-carbonyl]-butyl}-benzamide;
N-{3-Methyl-1-[6-oxo-4-(pyridine-2-carbonyl)-hexahydro-pyrrolo[3,2-b]pyrrole-1-carbonyl]-butyl}-4-morpholin-4-yl-benzamide;
Naphthalene-2-carboxylic acid {1-[6-oxo-4-(pyridine-2-carbonyl)hexahydro-pyrrolo[3,2-b]pyrrole-1-carbonyl]-butyl}-amide;
Quinoline-2-carboxylic acid {1-[6-oxo-4-(pyridine-2-carbonyl)-hexahydro-pyrrolo[3,2-b]pyrrole-1-carbonyl]-butyl}-amide;
Benzo[b]thiophene-2-carboxylic acid {1-[6-oxo-4-(pyridine-2-carbonyl)-hexahydro-pyrrolo[3,2-b]pyrrole-1-carbonyl]-butyl}-amide;
Benzofuran-2-carboxylic acid {1-[6-oxo-4-(pyridine-2-carbonyl)-hexahydro-pyrrolo[3,2-b]pyrrole-1-carbonyl]-butyl}-amide;
Biphenyl-4-carboxylic acid {1-[6-oxo-4-(pyridine-2-carbonyl)-hexahydro-pyrrolo[3,2-b]pyrrole-1-carbonyl]-butyl}-amide;
4-tert-Butyl-N-{1-[6-oxo-4-(pyridine-2-carbonyl)-hexahydro-pyrrolo[3,2-b]pyrrole-1-carbonyl]-butyl}-benzamide;
4-Dimethylamino-N-{1-[6-oxo-4-(pyridine-2-carbonyl)-hexahydro-pyrrolo[3,2-b]pyrrole-1-carbonyl]-butyl}-benzamide;
7-Methoxy-benzofuran-2-carboxylic acid {1-[6-oxo-4-(pyridine-2-carbonyl)-hexahydro-pyrrolo[3,2-b]pyrrole-1-carbonyl]-butyl}-amide;
{1-[6-oxo-4-(pyridine-2-carbonyl)-hexahydro-pyrrolo[3,2-b]pyrrole-1-carbonyl]-butyl}-carbamic acid benzyl ester;
5-Methoxy-benzofuran-2-carboxylic acid {1-[6-oxo-4-(pyridine-2-carbonyl)-hexahydro-pyrrolo[3,2-b]pyrrole-1-carbonyl]-butyl}-amide;
Thieno[3,2-b]thiophene-2-carboxylic acid {1-[6-oxo-4-(pyridine-2-carbonyl)-hexahydro-pyrrolo[3,2-b]pyrrole-1-carbonyl]-butyl}-amide;
3-Methyl-benzofuran-2-carboxylic acid {1-[6-oxo-4-(pyridine-2-carbonyl)-hexahydro-pyrrolo[3,2-b]pyrrole-1-carbonyl]-butyl}-amide;
Quinoxaline-2-carboxylic acid {1-[6-oxo-4-(pyridine-2-carbonyl)-hexahydro-pyrrolo[3,2-b]pyrrole-1-carbonyl]-butyl}-amide;
Benzo[1,3]dioxole-5-carboxylic acid {1-[6-oxo-4-(pyridine-2-carbonyl)-hexahydro-pyrrolo[3,2-b]pyrrole-1-carbonyl]-butyl}-amide;
Quinoline-6-carboxylic acid {1-[6-oxo-4-(pyridine-2-carbonyl)-hexahydro-pyrrolo[3,2-b]pyrrole-1-carbonyl]-butyl}-amide;
Furan-2-carboxylic acid {1-[6-oxo-4-(pyridine-2-carbonyl)-hexahydro-pyrrolo[3,2-b]pyrrole-1-carbonyl]-butyl}-amide;
Thiophene-2-carboxylic acid {1-[6-oxo-4-(pyridine-2-carbonyl)-hexahydro-pyrrolo[3,2-b]pyrrole-1-carbonyl]-butyl}-amide;
N-{1-[6-oxo-4-(pyridine-2-carbonyl)-hexahydro-pyrrolo[3,2-b]pyrrole-1-carbonyl]-butyl}-4-trifluoromethyl-benzamide;
Furan-3-carboxylic acid {1-[6-oxo-4-(pyridine-2-carbonyl)-hexahydro-pyrrolo[3,2-b]pyrrole-1-carbonyl]-butyl}-amide;
Thiophene-3-carboxylic acid {1-[6-oxo-4-(pyridine-2-carbonyl)-hexahydro-pyrrolo[3,2-b]pyrrole-1-carbonyl]-butyl}-amide;
N-{1-[6-oxo-4-(pyridine-2-carbonyl)-hexahydro-pyrrolo[3,2-b]pyrrole-1-carbonyl]-butyl}-4-phenoxy-benzamide;
N-{1-[6-oxo-4-(pyridine-2-carbonyl)-hexahydro-pyrrolo[3,2-b]pyrrole-1-carbonyl]-butyl}-4-(4-methyl-piperazin-1-yl)-benzamide;
4-Methyl-N-{1-[6-oxo-4-(pyridine-2-carbonyl)-hexahydro-pyrrolo[3,2-b]pyrrole-1-carbonyl]-butyl}-benzamide;
4-Methoxy-N-{1-[6-oxo-4-(pyridine-2-carbonyl)-hexahydro-pyrrolo[3,2-b]pyrrole-1-carbonyl]-butyl}-benzamide;
4-Isopropyl-N-{1-[6-oxo-4-(pyridine-2-carbonyl)-hexahydro-pyrrolo[3,2-b]pyrrole-1-carbonyl]-butyl}-benzamide;
N-{1-[6-oxo-4-(pyridine-2-carbonyl)-hexahydro-pyrrolo[3,2-b]pyrrole-1-carbonyl]-butyl}-4-vinyl-benzamide;
4-Imidazol-1-yl-N-{1-[6-oxo-4-(pyridine-2-carbonyl)-hexahydro-pyrrolo[3,2-b]pyrrole-1-carbonyl]-butyl}-benzamide;
N-{1-[6-oxo-4-(pyridine-2-carbonyl)-hexahydro-pyrrolo[3,2-b]pyrrole-1-carbonyl]-butyl}-4-thiophen-2-yl-benzamide;
N-{1-[6-oxo-4-(pyridine-2-carbonyl)-hexahydro-pyrrolo[3,2-b]pyrrole-1-carbonyl]-butyl}-4-oxazol-5-yl-benzamide;
5-Phenyl-thiophene-2-carboxylic acid {1-[6-oxo-4-(pyridine-2-carbonyl)-hexahydro-pyrrolo[3,2-b]pyrrole-1-carbonyl]-butyl}-amide;
N-{1-[6-oxo-4-(pyridine-2-carbonyl)-hexahydro-pyrrolo[3,2-b]pyrrole-1-carbonyl]-butyl}-4-trifluoromethoxy-benzamide;
5-Pyridin-2-yl-thiophene-2-carboxylic acid {1-[6-oxo-4-(pyridine-2-carbonyl)-hexahydro-pyrrolo[3,2-b]pyrrole-1-carbonyl]-butyl}-amide;
4-Difluoromethoxy-N-{1-[6-oxo-4-(pyridine-2-carbonyl)-hexahydro-pyrrolo[3,2-b]pyrrole-1-carbonyl]-butyl}-benzamide;
N-{1-[6-oxo-4-(pyridine-2-carbonyl)-hexahydro-pyrrolo[3,2-b]pyrrole-1-carbonyl]-butyl}-4-morpholin-4-yl-benzamide;
Naphthalene-2-carboxylic acid {3-methyl-1-[6-oxo-4-(2-pyridin-3-yl-acetyl)hexahydro-pyrrolo[3,2-b]pyrrole-1-carbonyl]-butyl}-amide;
Quinoline-2-carboxylic acid {3-methyl-1-[6-oxo-4-(2-pyridin-3-yl-acetyl)-hexahydro-pyrrolo[3,2-b]pyrrole-1-carbonyl]-butyl}-amide;
Benzo[b]thiophene-2-carboxylic acid {3-methyl-1-[6-oxo-4-(2-pyridin-3-yl-acetyl)-hexahydro-pyrrolo[3,2-b]pyrrole-1-carbonyl]-butyl}-amide;
Benzofuran-2-carboxylic acid {3-methyl-1-[6-oxo-4-(2-pyridin-3-yl-acetyl)-hexahydro-pyrrolo[3,2-b]pyrrole-1-carbonyl]-butyl}-amide;
Biphenyl-4-carboxylic acid {3-methyl-1-[6-oxo-4-(2-pyridin-3-yl-acetyl)-hexahydro-pyrrolo[3,2-b]pyrrole-1-carbonyl]-butyl}-amide;
4-tert-Butyl-N-{3-methyl-1-[6-oxo-4-(2-pyridin-3-yl-acetyl)-hexahydro-pyrrolo[3,2-b]pyrrole-1-carbonyl]-butyl}-benzamide;
4-Dimethylamino-N-{3-methyl-1-[6-oxo-4-(2-pyridin-3-yl-acetyl)-hexahydro-pyrrolo[3,2-b]pyrrole-1-carbonyl]-butyl}-benzamide;
7-Methoxy-benzofuran-2-carboxylic acid {3-methyl-1-[6-oxo-4-(2-pyridin-3-yl-acetyl)-hexahydro-pyrrolo[3,2-b]pyrrole-1-carbonyl]-butyl}-amide;
{3-Methyl-1-[6-oxo-4-(2-pyridin-3-yl-acetyl)-hexahydro-pyrrolo[3,2-b]pyrrole-1-carbonyl]-butyl}-carbamic acid benzyl ester;
5-Methoxy-benzofuran-2-carboxylic acid {3-methyl-1-[6-oxo-4-(2-pyridin-3-yl-acetyl)-hexahydro-pyrrolo[3,2-b]pyrrole-1-carbonyl]-butyl}-amide;
Thieno[3,2-b]thiophene-2-carboxylic acid {3-methyl-1-[6-oxo-4-(2-pyridin-3-yl-acetyl)-hexahydro-pyrrolo[3,2-b]pyrrole-1-carbonyl]-butyl}-amide;
3-Methyl-benzofuran-2-carboxylic acid {3-methyl-1-[6-oxo-4-(2-pyridin-3-yl-acetyl)-hexaydro-pyrrolo[3,2-b]pyrrole-1-carbonyl]-butyl}-amide;
Quinoxaline-2-carboxylic acid {3-methyl-1-[6-oxo-4-(2-pyridin-3-yl-acetyl)-hexahydro-pyrrolo[3,2-b]pyrrole-1-carbonyl]-butyl}-amide;
Benzo[1,3]dioxole-5-carboxylic acid {3-methyl-1-[6-oxo-4-(2-pyridin-3-yl-acetyl)-hexahydro-pyrrolo[3,2-b]pyrrole-1-carbonyl]-butyl}-amide;
Quinoline-6-carboxylic acid {3-methyl-1-[6-oxo-4-(2-pyridin-3-yl-acetyl)-hexahydro-pyrrolo[3,2-b]pyrrole-1-carbonyl]-butyl}-amide;
Furan-2-carboxylic acid {3-methyl-1-[6-oxo-4-(2-pyridin-3-yl-acetyl)-hexahydro-pyrrolo[3,2-b]pyrrole-1-carbonyl]-butyl}-amide;
Thiophene-2-carboxylic acid {3-methyl-1-[6-oxo-4-(2-pyridin-3-yl-acetyl)-hexahydro-pyrrolo[3,2-b]pyrrole-1-carbonyl]-butyl}-amide;
N-{3-Methyl-1-[6-oxo-4-(2-pyridin-3-yl-acetyl)-hexahydro-pyrrolo[3,2-b]pyrrole-1-carbonyl]-butyl}-4-trifluoromethyl-benzamide;
Furan-3-carboxylic acid {3-methyl-1-[6-oxo-4-(2-pyridin-3-yl-acetyl)-hexahydro-pyrrolo[3,2-b]pyrrole-1-carbonyl]-butyl}-amide;
Thiophene-3-carboxylic acid {3-methyl-1-[6-oxo-4-(2-pyridin-3-yl-acetyl)-hexahydro-pyrrolo[3,2-b]pyrrole-1-carbonyl]-butyl}-amide;
N-{3-Methyl-1-[6-oxo-4-(2-pyridin-3-yl-acetyl)-hexahydro-pyrrolo[3,2-b]pyrrole-1-carbonyl]-butyl}-4-phenoxy-benzamide;
N-{3-Methyl-1-[6-oxo-4-(2-pyridin-3-yl-acetyl)-hexahydro-pyrrolo [3,2-b]pyrrole-1-carbonyl]-butyl}-4-(4-methyl-piperazin-1-yl)-benzamide;
4-Methyl-N-{3-methyl-1-[6-oxo-4-(2-pyridin-3-yl-acetyl)-hexahydro-pyrrolo[3,2-b]pyrrole-1-carbonyl]-butyl}-benzamide;
4-Methoxy-N-{3-methyl-1-[6-oxo-4-(2-pyridin-3-yl-acetyl)-hexahydro-pyrrolo[3,2-b]pyrrole-1-carbonyl]-butyl}-benzamide;
4-Isopropyl-N-{3-methyl-1-[6-oxo-4-(2-pyridin-3-yl-acetyl)-hexahydro-pyrrolo[3,2-b]pyrrole-1-carbonyl]-butyl}-benzamide;
N-{3-Methyl-1-[6-oxo-4-(2-pyridin-3-yl-acetyl)-hexahydro-pyrrolo[3,2-b]pyrrole-1-carbonyl]-butyl}-4-vinyl-benzamide;
4-Imidazol-1-yl-N-{3-methyl-1-[6-oxo-4-(2-pyridin-3-yl-acetyl)-hexahydro-pyrrolo[3,2-b]pyrrole-1-carbonyl]-butyl}-benzamide;
N-{3-Methyl-1-[6-oxo-4-(2-pyridin-3-yl-acetyl)-hexahydro-pyrrolo[3,2-b]pyrrole-1-carbonyl]-butyl}-4-thiophen-2-yl-benzamide;
N-{3-Methyl-1-[6-oxo-4-(2-pyridin-3-yl-acetyl)-hexahydro-pyrrolo[3,2-b]pyrrole-1-carbonyl]-butyl}-4-oxazol-5-yl-benzamide;
5-Phenyl-thiophene-2-carboxylic acid {3-methyl-1-[6-oxo-4-(2-pyridin-3-yl-acetyl)-hexahydro-pyrrolo[3,2-b]pyrrole-1-carbonyl]-butyl}-amide;
N-{3-Methyl-1-[6-oxo-4-(2-pyridin-3-yl-acetyl)-hexahydro-pyrrolo[3,2-b]pyrrole-1-carbonyl]-butyl}-4-trifluoromethoxy-benzamide;
5-Pyridin-2-yl-thiophene-2-carboxylic acid {3-methyl-1-[6-oxo-4-(2-pyridin-3-yl-acetyl)-hexahydro-pyrrolo[3,2-b]pyrrole-1-carbonyl]-butyl}-amide;
4-Difluoromethoxy-N-{3-methyl-1-[6-oxo-4-(2-pyridin-3-yl-acetyl)-hexahydro-pyrrolo[3,2-b]pyrrole-1-carbonyl]-butyl}-benzmide;
N-{3-Methyl-1-[6-oxo-4-(2-pyridin-3-yl-acetyl)-hexahydro-pyrrolo[3,2-b]pyrrole-1-carbonyl]-butyl}-4-morpholin-4-yl-benzamide;
Naphthalene-2-carboxylic acid {1-[6-oxo-4-(2-pyridin-3-yl-acetyl)hexahydro-pyrrolo[3,2-b]pyrrole-1-carbonyl]-butyl}-amide;
Quinoline-2-carboxylic acid {1-[6-oxo-4-(2-pyridin-3-yl-acetyl)-hexahydro-pyrrolo[3,2-b]pyrrole-1-carbonyl]-butyl}-amide;
Benzo[b]thiophene-2-calboxylic acid {1-[6-oxo-4-(2-pyridin-3-yl-acetyl)-hexahydro-pyrrolo[3,2-b]pyrrole-1-carbonyl]-butyl}-amide;
Benzofuran-2-carboxylic acid {1-[6-oxo-4-(2-pyridin-3-yl-acetyl)-hexahydro-pyrrolo[3,2-b]pyrrole-1-carbonyl]-butyl}-amide;
Biphenyl-4-carboxylic acid {1-[6-oxo-4-(2-pyridin-3-yl-acetyl)-hexahydro-pyrrolo[3,2-b]pyrrole-1-carbonyl]-butyl}-amide;
4-tert-Butyl-N-{1-[6-oxo-4-(2-pyridin-3-yl-acetyl)-hexahydro-pyrrolo[3,2-b]pyrrole-1-carbonyl]-butyl}-benzamide;
4-Dimethylamino-N-{1-[6-oxo-4-(2-pyridin-3-yl-acetyl)-hexahydro-pyrrolo[3,2-b]pyrrole-1-carbonyl]-butyl}-benzamide;
7-Methoxy-benzofuran-2-carboxylic acid {1-[6-oxo-4-(2-pyridin-3-yl-acetyl)-hexahydro-pyrrolo[3,2-b]pyrrole-1-carbonyl]-butyl}-amide;
{1-[6-oxo-4-(2-pyridin-3-yl-acetyl)-hexahydro-pyrrolo[3,2-b]pyrrole-1-carbonyl]-butyl}-carbamic acid benzyl ester;
5-Methoxy-benzofuran-2-carboxylic acid {1-[6-oxo-4-(2-pyridin-3-yl-acetyl)-hexahydro-pyrrolo[3,2-b]pyrrole-1-carbonyl]-butyl}-amide;
Thieno[3,2-b]thiophene-2-carboxylic acid {1-[6-oxo-4-(2-pyridin-3-yl-acetyl)-hexahydro-pyrrolo[3,2-b]pyrrole-1-carbonyl]-butyl}-amide;
3-Methyl-benzofuran-2-carboxylic acid {1-[6-oxo-4-(2-pyridin-3-yl-acetyl)-hexahydro-pyrrolo[3,2-b]pyrrole-1-carbonyl]-butyl}-amide;
Quinoxaline-2-carboxylic acid {1-[6-oxo-4-(2-pyridin-3-yl-acetyl)-hexahydro-pyrrolo[3,2-b]pyrrole-1-carbonyl]-butyl}-amide;
Benzo[1,3]dioxole-5-carboxylic acid {1-[6-oxo-4-(2-pyridin-3-yl-acetyl)-hexahydro-pyrrolo[3,2-b]pyrrole-1-carbonyl]-butyl}-amide;
Quinoline-6-carboxylic acid {1-[6-oxo-4-(2-pyridin-3-yl-acetyl)-hexahydro-pyrrolo[3,2-b]pyrrole-1-carbonyl]-butyl}-amide;
Furan-2-carboxylic acid {1-[6-oxo-4-(2-pyridin-3-yl-acetyl)-hexahydro-pyrrolo[3,2-b]pyrrole-1-carbonyl]-butyl}-amide;
Thiophene-2-carboxylic acid {1-[6-oxo-4-(2-pyridin-3-yl-acetyl)-hexahydro-pyrrolo[3,2-b]pyrrole-1-carbonyl]-butyl}-amide;
N-{1-[6-oxo-4-(2-pyridin-3-yl-acetyl)-hexahydro-pyrrolo[3,2-b]pyrrole-1-carbonyl]-butyl}-4-trifluoromethyl-benzamide;
Furan-3-carboxylic acid {1-[6-oxo-4-(2-pyridin-3-yl-acetyl)-hexahydro-pyrrolo[3,2-b]pyrrole-1-carbonyl]-butyl}-amide;
Thiophene-3-carboxylic acid {1-[6-oxo-4-(2-pyridin-3-yl-acetyl)-hexahydro-pyrrolo[3,2-b]pyrrole-1-carbonyl]-butyl}-amide;
N-{1-[6-oxo-4-(2-pyridin-3-yl-acetyl)-hexahydro-pyrrolo[3,2-b]pyrrole-1-carbonyl]-butyl}-4-phenoxy-benzamide;
N-{1-[6-oxo-4-(2-pyridin-3-yl-acetyl)-hexahydro-pyrrolo[3,2-b]pyrrole-1-carbonyl]-butyl}-4-(4-methyl-piperazin-1-yl)-benzamide;
4-Methyl-N-{1-[6-oxo-4-(2-pyridin-3-yl-acetyl)-hexahydro-pyrrolo[3,2-b]pyrrole-1-carbonyl]-butyl}-benzamide;
4-Methoxy-N-{1-[6-oxo-4-(2-pyridin-3-yl-acetyl)-hexahydro-pyrrolo[3,2-b]pyrrole-1-carbonyl]-butyl}-benzamide;
4-Isopropyl-N- {1-[6-oxo-4-(2-pyridin-3-yl-acetyl)-hexahydro-pyrrolo[3,2-b]pyrrole-1-carbonyl]-butyl}-benzamide;
N-{1-[6-oxo-4-(2-pyridin-3-yl-acetyl)-hexahydro-pyrrolo[3,2-b]pyrrole-1-carbonyl]-butyl}-4-vinyl-benzamide;
4-Imidazol-1-yl-N-{1-[6-oxo-4-(2-pyridin-3-yl-acetyl)-hexahydro-pyrrolo[3,2-b]pyrrole-1-carbonyl]-butyl}-benzamide;
N-{1-[6-oxo-4-(2-pyridin-3-yl-acetyl)-hexahydro-pyrrolo[3,2-b]pyrrole-1-carbonyl]-butyl}-4-thiophen-2-yl-benzamide;
N-{1-[6-oxo-4-(2-pyridin-3-yl-acetyl)-hexahydro-pyrrolo[3,2-b]pyrrole-1-carbonyl]-butyl}-4-oxazol-5-yl-benzamide;
5-Phenyl-thiophene-2-carboxylic acid {1-[6-oxo-4-(2-pyridin-3-yl-acetyl)-hexahydro-pyrrolo[3,2-b]pyrrole-1-carbonyl]-butyl}-amide;
N-{1-[6-oxo-4-(2-pyridin-3-yl-acetyl)-hexahydro-pyrrolo[3,2-b]pyrrole-1-carbonyl]-butyl}-4-trifluoromethoxy-benzamide;
5-Pyridin-2-yl-thiophene-2-carboxylic acid {1-[6-oxo-4-(2-pyridin-3-yl-acetyl)-hexahydro-pyrrolo[3,2-b]pyrrole-1-carbonyl]-butyl}-amide;
4-Difluoromethoxy-N-{1-[6-oxo-4-(2-pyridin-3-yl-acetyl)-hexahydro-pyrrolo[3,2-b]pyrrole-1-carbonyl]-butyl}-benzamide;
N-{1-[6-oxo-4-(2-pyridin-3-yl-acetyl)-hexahydro-pyrrolo[3,2-b]pyrrole-1-carbonyl]-butyl}-4-morpholin-4-yl-benzamide;
Naphthalene-2-carboxylic acid {3-methyl-1-[6-oxo-4-(pyridin-2-ylmethanesulfonyl)hexahydro-pyrrolo[3,2-b]pyrrole-1-carbonyl]-buty}-amide;
Quinoline-2-carboxylic acid {3-methyl-1-[6-oxo-4-(pyridin-2-ylmethanesulfonyl)-hexahydro-pyrrolo[3,2-b]pyrrole-1-carbonyl]-butyl}-amide;
Benzo[b]thiophene-2-carboxylic acid {3-methyl-1-[6-oxo-4-(pyridin-2-ylmethanesulfonyl)-hexahydro-pyrrolo[3,2-b]pyrrole-1-carbonyl]-butyl}-amide;
Benzofuran-2-carboxylic acid {3-methyl-1-[6-oxo-4-(pyridin-2-ylmethanesulfonyl)-hexahydro-pyrrolo[3,2-b]pyrrole-1-carbonyl]-butyl}-amide;
Biphenyl-4-carboxylic acid {3-methyl-1-[6-oxo-4-(pyridin-2-ylmethanesulfonyl)-hexahydro-pyrrolo[3,2-b]pyrrole-1-carbonyl]-butyl}-amide;
4-tert-Butyl-N-{3-methyl-1-[6-oxo-4-(pyridin-2-ylmethanesulfonyl)-hexahydro-pyrrolo[3,2-b]pyrrole-1-carbonyl]-butyl}-benzamide;
4-Dimethylamino-N-{3-methyl-1-[6-oxo-4-(pyridin-2-ylmethanesulfonyl)-hexahydro-pyrrolo[3,2-b]pyrrole-1-carbon yl]-butyl}-benzamide;
7-Methoxy-benzofiuan-2-carboxylic acid {3-methyl-1-[6-oxo-4-(pyridin-2-ylmethanesulfonyl)-hexahydro-pyrrolo[3,2-b]pyrrole-1-carbonyl]-butyl}-amide;
{3-Methyl-1-[6-oxo-4-(pyridin-2-ylmethanesulfonyl)-hexahydro-pyrrolo[3,2-b]pyrrole-1-carbonyl]-butyl}-carbamic acid benzyl ester;
5-Methoxy-benzofuran-2-carboxylic acid {3-methyl-1-[6-oxo-4-(pyridin-2-ylmethanesulfonyl)-hexahydro-pyrrolo[3,2-b]pyrrole-1-carbonyl]-butyl}-amide;
Thieno[3,2-b]thiophene-2-carboxylic acid {3-methyl-1-[6-oxo-4-(pyridin-2-ylmethanesulfonyl)-hexahydro-pyrrolo[3,2-b]pyrrole-1-carbonyl]-butyl}-amide;
3-Methyl-benzofuran-2-carboxylic acid {3-methyl-1-[6-oxo-4-(pyridin-2-ylmethanesulfonyl)-hexahydro-pyrrolo[3,2-b]pyrrole-1-carbonyl]-butyl}-amide;
Quinoxaline-2-carboxylic acid {3-methyl-1-[6-oxo-4-(pyridin-2-ylmethanesulfonyl)-hexahydro-pyrrolo[3,2-b]pyrrole-1-carbonyl]-butyl}-amide;
Benzo[1,3]dioxole-5-carboxylic acid {3-methyl-1-[6-oxo-4-(pyridin-2-ylmethanesulfonyl)-hexahydro-pyrrolo[3,2-b]pyrrole-1-carbonyl]-butyl}-amide;
Quinoline-6-carboxylic acid {3-methyl-1-[6-oxo-4-(pyridin-2-ylmethanesulfonyl)-hexahydro-pyrrolo[3,2-b]pyrrole-1-carbonyl]-butyl}amide;
Furan-2-carboxylic acid {3-methyl-1-[6-oxo-4-(pyridin-2-ylmethanesulfonyl)-hexahydro-pyrrolo[3,2-b]pyrrole-1-carbonyl]-butyl}-amide;
Thiophene-2-carboxylic acid {3-methyl-1-[6-oxo-4-(pyridin-2-ylmethanesulfonyl)-hexahydro-pyrrolo[3,2-b]pyrrole-1-carbonyl]-butyl}-amide;
N-{3-Methyl-1-[6-oxo-4-(pyridin-2-ylmethanesulfonyl)-hexahydro-pyrrolo[3,2-b]pyrrole-1-carbonyl]-butyl}-4-trifluor omethyl-benzamide;
Furan-3-carboxylic acid {3-methyl-1-[6-oxo-4-(pyridin-2-ylmethanesulfonyl)-hexahydro-pyrrolo[3,2-b]pyrrole-1-carbonyl]-butyl}-amide;
Thiophene-3-carboxylic acid {3-methyl-1-[6-oxo-4-(pyridin-2-ylmethanesulfonyl)-hexahydro-pyrrolo[3,2-b]pyrrole-1-carbonyl]-butyl}-amide;
N-{3-Methyl-1-[6-oxo-4-(pyridin-2-ylmethanesulfonyl)-hexahydro-pyrrolo[3,2-b]pyrrole-1-carbonyl]-butyl}-4-phenoxy-benzamide;
N-{3-Methyl-1-[6-oxo-4-(pyridin-2-ylmethanesulfonyl)-hexahydro-pyrrolo[3,2-b]pyrrole-1-carbonyl]-butyl}-4-(4-met hyl-piperazin-1-yl)-benzamide;
4-Methyl-N-{3-methyl-1-[6-oxo-4-(pyridin-2-ylmethanesulfonyl)-hexahydro-pyrrolo[3,2-b]pyrrole-1-carbonyl]-butyl}-benzamide;
4-Methoxy-N-{3-methyl-1-[6-oxo-4-(pyridin-2-ylmethanesulfonyl)-hexahydro-pyrrolo[3,2-b]pyrrole-1-carbonyl]-butyl}-benzamide;
4-Isopropyl-N-{3-methyl-1-[6-oxo-4-(pyridin-2-ylmethanesulfonyl)-hexahydro-pyrrolo[3,2-b]pyrrole-1-carbonyl]-butyl}-benzamide;
N- {3-Methyl-1-[6-oxo-4-(pyridin-2-ylmethanesulfonyl)-hexahydro-pyrrolo[3,2-b]pyrrole-1-carbonyl]-butyl}-4-vinyl-benzamide;
4-Imidazol-1-yl-N-{3-methyl-1-[6-oxo-4-(pyridin-2-ylmethanesulfonyl)-hexahydro-pyrrolo[3,2-b]pyrrole-1-carbonyl]-butyl}-benzamide;
N-{3-Methyl-1-[6-oxo-4-(pyridin-2-ylmethanesulfonyl)-hexahydro-pyrrolo[3,2-b]pyrrole-1-carbonyl]-butyl}-4-thiophen-2-yl-benzamide;
N-{3-Methyl-1-[6-oxo-4-(pyridin-2-ylmethanesulfonyl)-hexahydro-pyrrolo[3,2-b]pyrrole-1-carbonyl]-butyl}-4-oxazol-5-yl-benzamide;
5-Phenyl-thiophene-2-carboxylic acid {3-methyl-1-[6-oxo-4-(pyridin-2-ylmethanesulfonyl)-hexahydro-pyrrolo[3,2-b]pyrrole-1-carbonyl]-butyl}-amide;
N-{3-Methyl-1-[6-oxo-4-(pyridin-2-ylmethanesulfonyl)-hexahydro-pyrrolo[3,2-b]pyrrole-1-carbonyl]-butyl}-4-trifluoromethoxy-benzamide;
5-Pyridin-2-yl-thiophene-2-carboxylic acid {3-methyl-1-[6-oxo-4-(pyridin-2-ylmethanesulfonyl)-hexahydro-pyrrolo[3,2-b]pyrrole-1-carbonyl]-butyl}-amide;
4-Difluoromethoxy-N-{3-methyl-1-[6-oxo-4-(pyridin-2-ylmethanesulfonyl)-hexahydro-pyrrolo[3,2-b]pyrrole-1-carbonyl]-butyl}-benzamide;
N-{3-Methyl-1-[6-oxo-4-(pyridin-2-ylmethanesulfonyl)-hexahydro-pyrrolo[3,2-b]pyrrole-1-carbonyl]-butyl}-4-morpholin-4-yl-benzamide;
Naphthalene-2-carboxylic acid {1-[6-oxo-4-(pyridin-2-ylmethanesulfonyl)hexahydro-pyrrolo[3,2-b]pyrrole-1-carbonyl]-butyl}-amide;
Quinoline-2-carboxylic acid {1-[6-oxo-4-(pyridin-2-ylmethanesulfonyl)-hexahydro-pyrrolo[3,2-b]pyrrole-1-carbonyl]-butyl}-amide;
Benzo[b]thiophene-2-carboxylic acid {1-[6-oxo-4-(pyridin-2-ylmethanesulfonyl)-hexahydro-pyrrolo[3,2-b]pyrrole-1-carbonyl]-butyl}-amide;
Benzofuran-2-carboxylic acid {1-[6-oxo-4-(pyridin-2-ylmethanesulfonyl)-hexahydro-pyrrolo[3,2-b]pyrrole-1-carbonyl]-butyl}-amide;
Biphenyl-4-carboxylic acid {1-[6-oxo-4-(pyridin-2-ylmethanesulfonyl)-hexahydro-pyrrolo[3,2-b]pyrrole-1-carbonyl]-butyl}-amide;
4-tert-Butyl-N-{1-[6-oxo-4-(pyridin-2-ylmethanesulfonyl)-hexahydro-pyrrolo[3,2-b]pyrrole-1-carbonyl]-butyl}-benzamide;
4-Dimethylamino-N-{1-[6-oxo-4-(pyridin-2-ylmethanesulfonyl)-hexahydro-pyrrolo[3,2-b]pyrrole-1-carbonyl]-butyl}-benzamide;
7-Methoxy-benzofuran-2-carboxylic acid {1-[6-oxo-4-(pyridin-2-ylmethanesulfonyl)-hexahydro-pyrrolo[3,2-b]pyrrole-1-carbonyl]-butyl}-amide;
{1-[6-oxo-4-(pyridin-2-ylmethanesulfonyl)-hexahydro-pyrrolo[3,2-b]pyrrole-1-carbonyl]-butyl}-carbamic acid benzyl ester;
5-Methoxy-benzofuran-2-carboxylic acid {1-[6-oxo-4-(pyridin-2-ylmethanesulfonyl)-hexahydro-pyrrolo[3,2-b]pyrrole-1-carbonyl]-butyl}-amide;
Thieno[3,2-b]thiophene-2-carboxylic acid {1-[6-oxo-4-(pyridin-2-ylmethanesulfonyl)-hexahydro-pyrrolo[3,2-b]pyrrole-1-carbonyl]-butyl}-amide;
3-Methyl-benzofuran-2-carboxylic acid {1-[6-oxo4-(pyridin-2-ylmethanesulfonyl)-hexahydro-pyrrolo[3,2-b]pyrrole-1-carbonyl]-butyl}-amide;
Quinoxaline-2-carboxylic acid {1-[6-oxo-4-(pyridin-2-ylmethanesulfonyl)-hexahydro-pyrrolo[3,2-b]pyrrole-1-carbonyl]-butyl}-amide;
Benzo[1,3]dioxole-5-carboxylic acid {1-[6-oxo-4-(pyridin-2-ylmethanesulfonyl)-hexahydro-pyrrolo[3,2-b]pyrrole-1-carbonyl]-butyl}-amide;
Quinoline-6-carboxylic acid {1-[6-oxo-4-(pyridin-2-ylmethanesulfonyl)-hexahydro-pyrrolo[3,2-b]pyrrole-1-carbonyl]-butyl}-amide;
Furan-2-carboxylic acid {1-[6-oxo-4-(pyridin-2-ylmethanesulfony)-hexahydro-pyrrolo[3,2-b]pyrrole-1-carbonyl]-butyl}-amide;
Thiophene-2-carboxylic acid {1-[6-oxo-4-(pyridin-2-ylmethanesulfonyl)-hexahydro-pyrrolo[3,2-b]pyrrole-1-carbonyl]-butyl}-amide;
N-{1-[6-oxo-4-(pyridin-2-ylmethanesulfonyl)-hexahydro-pyrrolo[3,2-b]pyrrole-1-carbonyl]-butyl}-4-trifluoromethyl-benzamide;
Furan-3-carboxylic acid {1-[6-oxo-4-(pyridin-2-ylmethanesulfonyl)-hexahydro-pyrrolo[3,2-b]pyrrole-1-carbonyl]-butyl}-amide;
Thiophene-3-carboxylic acid {1-[6-oxo-(pyridin-2-ylmethanesulfonyl)-hexahydro-pyrrolo[3,2-b]pyrrole-1-carbonyl]-butyl}-amide;
N-{1-[6-oxo-4-(pyridin-2-ylmethanesulfonyl)-hexahydro-pyrrolo[3,2-b]pyrrole-1-carbonyl]-butyl}-4-phenoxy-benzamide;
N-{1-[6-oxo-4-(pyridin-2-ylmethanesulfonyl)-hexahydro-pyrrolo[3,2-b]pyrrole-1-carbonyl]-butyl}-4-(4-methyl-piperazin-1-yl)-benzamide;
4-Methyl-N-{1-[6-oxo-4-(pyridin-2-ylmethanesulfonyl)-hexahydro-pyrrolo[3,2-b]pyrrole-1-carbonyl]-butyl}-benzamide;
4-Methoxy-N-{1-[6-oxo-4-(pyridin-2-ylmethanesulfonyl)-hexahydro-pyrrolo[3,2-b]pyrrole-1-carbonyl]-butyl}-benzamide;
4-Isopropyl-N-{1-[6-oxo-4-(pyridin-2-ylmethanesulfonyl)-hexahydro-pyrrolo[3,2-b]pyrrole-1-carbonyl]-butyl}-benzamide;
N-{1-[6-oxo-4-(pyridin-2-ylmethanesulfonyl)-hexahydro-pyrrolo[3,2-b]pyrrole-1-carbonyl]-butyl}-4-vinyl-benzamide;
4-Imidazol-1-yl-N-{1-[6-oxo-4-(pyridin-2-ylmethanesulfonyl)-hexahydro-pyrrolo[3,2-b]pyrrole-1-carbonyl]-butyl}-benzamide;
N-{1-[6-oxo-4-(pyridin-2-ylmethanesulfonyl)-hexahydro-pyrrolo[3,2-b]pyrrole-1-carbonyl]-butyl}-4-thiophen-2-yl-benzamide;
N-{1-[6-oxo-4-(pyridin-2-ylmethanesulfonyl)-hexahydro-pyrrolo[3,2-b]pyrrole-1-carbonyl]-butyl}-4-oxazol-5-yl-benzamide;
5-Phenyl-thiophene-2-carboxylic acid {1-[6-oxo-4-(pyridin-2-ylmethanesulfonyl)-hexahydro-pyrrolo[3,2-b]pyrrole-1-carbonyl]-butyl}-amide;
N-{1-[6-oxo-4-(pyridin-2-ylmethanesulfonyl)-hexahydro-pyrrolo[3,2-b]pyrrole-1-carbonyl]-butyl}-4-trifluoromethoxy-benzamide;
5-Pyridin-2-yl-thiophene-2-carboxylic acid {1-[6-oxo-4-(pyridin-2-ylmethanesulfonyl)-hexahydro-pyrrolo[3,2-b]pyrrole-1-carbonyl]-butyl}-amide;
4-Difluoromethoxy-N-{1-[6-oxo-4-(pyridin-2-ylmethanesulfonyl)-hexahydro-pyrrolo[3,2-b]pyrrole-1-carbonyl]-butyl}-benzamide;
N-{1-[6-oxo-4-(pyridin-2-ylmethanesulfonyl)-hexahydro-pyrrolo[3,2-b]pyrrole-1-carbonyl]-butyl}-4-morpholin-4-yl-benzamide;
Naphthalene-2-carboxylic acid {3-methyl-1-[6-oxo-4-(pyridin-3-ylmethanesulfonyl)hexahydro-pyrrolo[3,2-b]pyrrole-1-carbonyl]-butyl}-amide;
Quinoline-2-carboxylic acid {3-methyl-1-[6-oxo-4-(pyridin-3-ylmethanesulfonyl)-hexahydro-pyrrolo[3,2-b]pyrrole-1-carbonyl]-butyl}-amide;
Benzo[b]thiophene-2-carboxylic acid {3-methyt-1-[6-oxo-4-(pyridin-3-ylmethanesulfonyl)-hexahydro-pyrrolo[3,2-b]pyrrole-1-carbonyl]-butyl}-amide;
Benzofuran-2-carboxylic acid {3-methyl-1-[6-oxo-4-(pyridin-3-ylmethanesulfonyl)-hexahydro-pyrrolo[3,2-b]pyrrole-1-carbonyl]-butyl}-amide;
Biphenyl-4-carboxylic acid {3-methyl-1-[6-oxo-4-(pyridin-3-ylmethanesulfonyl)-hexahydro-pyrrolo[3,2-b]pyrrole-1-carbonyl]-butyl}-amide;
4-tert-Butyl-N-{3-methyl-1-[6-oxo-4-(pyridin-3-ylmethanesulfonyl)-hexahydro-pyrrolo[3,2-b]pyrrole-1-carbonyl]-butyl}-benzamide;
4-Dimethylamino-N-{3-methyl-1-[6-oxo-4-(pyridin-3-ylmethanesulfonyl)-hexahydro-pyrrolo[3,2-b]pyrrole-1-carbon yl]-butyl}-benzamide;
7-Methoxy-benzofuran-2-carboxylic acid {3-methyl-1-[6-oxo-4-(pyridin-3-ylmethanesulfonyl)-hexahydro-pyrrolo[3,2-b]pyrrole-1-carbonyl]-butyl}-amide;
{3-Methyl-1-[6-oxo-4-(pyridin-3-ylmethanesulfonyl)-hexahydro-pyrrolo[3,2-b]pyrrole-1-carbonyl]-butyl}-carbamic acid benzyl ester;
5-Methoxy-benzofuran-2-carboxylic acid {3-methyl-1-[6-oxo-4-(pyridin-3-ylmethanesulfonyl)-hexahydro-pyrrolo[3, 2-b]pyrrole-1-carbonyl]-butyl}-amide;
Thieno[3,2-b]thiophene-2-carboxylic acid {3-methyl-1-[6-oxo-4-(pyridin-3-ylmethanesulfonyl)-hexahydro-pyrrolo[3, 2-b]pyrrole-1-carbonyl]-butyl}-amide;
3-Methyl-benzofuran-2-carboxylic acid {3-methyl-1-[6-oxo-4-(pyridin-3-ylmethanesulfonyl)-hexahydro-pyrrolo[3,2-b]pyrrole-1-carbonyl]-butyl}-amide;
Quinoxaline-2-carboxylic acid {3-methyl-1-[6-oxo-4-(pyridin-3-ylmethanesulfonyl)-hexahydro-pyrrolo[3,2-b]pyrrole-1-carbonyl]-butyl}-amide;
Benzo[1,3]dioxole-5-carboxylic acid {3-methyl-1-[6-oxo-4-(pyridin-3-ylmethanesulfonyl)-hexahydro-pyrrolo[3,2-b]pyrrole-1-carbonyl]-butyl}-amide;
Quinoline-6-carboxylic acid {3-methyl-1-[6-oxo-4-(pyridin-3-ylmethanesulfonyl)-hexahydro-pyrrolo[3,2-b]pyrrole-1-carbonyl]-butyl}-amide;
Furan-2-carboxylic acid {3-methyl-1-[6-oxo-4-(pyridin-3-ylmethanesulfonyl)-hexahydro-pyrrolo[3,2-b]pyrrole-1-carbonyl]-butyl}-amide;
Thiophene-2-carboxylic acid {3-methyl-1-[6-oxo-4-(pyridin-3-ylmethanesulfonyl)-hexahydro-pyrrolo[3,2-b]pyrrole-1-carbonyl]-butyl}-amide;
N- {3-Methyl-1-[6-oxo-4-(pyridin-3-ylmethanesulfonyl)-hexahydro-pyrrolo[3,2-b]pyrrole-1-carbonyl]-butyl}-4-trifluoromethyl-benzamide;
Furan-3-carboxylic acid {3-methyl-1-[6-oxo-4-(pyridin-3-ylmethanesulfonyl)-hexahydro-pyrrolo[3,2-b]pyrrole-1-carbonyl]-butyl}-amide;
Thiophene-3-carboxylic acid {3-methyl-1-[6-oxo-4-(pyridin-3-ylmethanesulfonyl)-hexahydro-pyrrolo[3,2-b]pyrrole-1-carbonyl]-butyl}-amide;
N-{3-Methyl-1-[6-oxo-4-(pyridin-3-ylmethanesulfonyl)-hexahydro-pyrrolo[3,2-b]pyrrole-1-carbonyl]-butyl}-4-phenoxy-benzamide;
N-{3-Methyl-1-[6-oxo-4-(pyridin-3-ylmethanesulfonyl)-hexahydro-pyrrolo[3,2-b]pyrrole-1-carbonyl]-butyl}-4-(4-met hyl-piperazin-1-yl)-benzamide;
4-Methyl-N-{3-methyl-1-[6-oxo-4-(pyridin-3-ylmethanesulfonyl)-hexahydro-pyrrolo[3,2-b]pyrrole-1-carbonyl]-butyl}-benzamide;
4-Methoxy-N-{3-methyl-1-[6-oxo-4-(pyridin-3-ylmethanesulfonyl)-hexahydro-pyrrolo[3,2-b]pyrrole-1-carbonyl]-butyl}-benzamide;
4-Isopropyl-N-{3-methyl-1-[6-oxo-4-(pyridin-3-ylmethanesulfonyl)-hexahydro-pyrrolo[3,2-b]pyrrole-1-carbonyl]-butyl}-benzamide;
N-{3-Methyl-1-[6-oxo-4-(pyridin-3-ylmethanesulfonyl)-hexahydro-pyrrolo[3,2-b]pyrrole-1-arbonyl]-butyl}-4-vinyl-benzamide;
4-Imidazol-1-yl-N-{3-methyl-1-[6-oxo-4-(pyridin-3-ylmethanesulfonyl)-hexahydro-pyrrolo[3,2-b]pyrrole-1-carbonyl]-butyl}-benzamide;
N-{3-Methyl-1-[6-oxo-4-(pyridin-3-ylmethanesulfonyl)-hexahydro-pyrrolo[3,2-b]pyrrole-1-carbonyl]-butyl}-4-thiophe n-2-yl-benzamide;
N-{3-Methyl-1-[6-oxo-4-(pyridin-3-ylmethanesulfonyl)-hexahydro-pyrrolo[3,2-b]pyrrole-1-carbonyl]-butyl}-4-oxazol-5-yl-benzamide;
5-Phenyl-thiophene-2-carboxylic acid {3-methyl-1-[6-oxo-4-(pyridin-3-ylmethanesulfonyl)-hexahydro-pyrrolo[3,2-b]pyrrole-1-carbonyl]-butyl}-amide;
N-{3-Methyl-1-[6-oxo-4-(pyridin-3-ylmethanesulfonyl)-hexahydro-pyrrolo[3,2-b]pyrrole-1-carbonyl]-butyl}4-trifluoro methoxy-benzamide;
5-Pyridin-2-yl-thiophene-2-carboxylic acid {3-methyl-1-[6-oxo-4-(pyridin-3-ylmethanesulfonyl)-hexahydro-pyrrolo[3,2-b]pyrrole-1-carbonyl]-butyl}-amide;
4-Difluoromethoxy-N- {3-methyl-1-[6-oxo-4-(pyridin-3-ylmethanesulfonyl)-hexahydro-pyrrolo[3,2-b]pyrrole-1-carbonyl]-butyl}-benzamide;
N-{3-Methyl-1-[6-oxo-4-(pyridin-3-ylmethanesulfonyl)-hexahydro-pyrrolo[3,2-b]pyrrole-1-carbonyl]-butyl}-4-morph olin-4-yl-benzamide;
Naphthalene-2-carboxylic acid {1-[6-oxo-4-(pyridin-3-ylmethanesulfonyl)hexahydro-pyrrolo[3,2-b]pyrrole-1-carbonyl]-butyl}-amide;
Quinoline-2-carboxylic acid {1-[6-oxo-4-(pyridin-3-ylmethanesulfonyl)-hexahydro-pyrrolo[3,2-b]pyrrole-1-carbonyl]-butyl}-amide;
Benzo[b]thiophene-2-carboxylic acid {1-[6-oxo-4-(pyridin-3-ylmethanesulfonyl)-hexahydro-pyrrolo[3,2-b]pyrrole-1-carbonyl]-butyl}-amide;
Benzofuran-2-carboxylic acid {1-[6-oxo-4-(pyridin-3-ylmethanesulfonyl)-hexahydro-pyrrolo[3,2-b]pyrrole-1-carbonyl]-butyl}-amide;
Biphenyl-4-carboxylic acid {1-[6-oxo-4-(pyridin-3-ylmethanesulfonyl)-hexahydro-pyrrolo[3,2-b]pyrrole-1-carbonyl]-butyl}-amide;
4-tert-Butyl-N-{1-[6-oxo-4-(pyridin-3-ylmethanesulfonyl)-hexahydro-pyrrolo[3,2-b]pyrrole-1-carbonyl]-butyl}-benzamide;
4-Dimethylamino-N-{1-[6-oxo-4-(pyridin-3-ylmethanesulfonyl)-hexahydro-pyrrolo[3,2-b]pyrrole-1-carbonyl]-butyl}-benzamide;
7-Methoxy-benzofuran-2-carboxylic acid {1-[6-oxo-4-(pyridin-3-ylmethanesulfonyl)-hexahydro-pyrrolo[3,2-b]pyrrole-1-carbonyl]-butyl}-amide;
{1-[6-oxo-4-(pyridin-3-ylmethanesulfonyl)-hexahydro-pyrrolo[3,2-b]pyrrole-1-carbonyl]-butyl}-carbamic acid benzyl ester;
5-Methoxy-benzofuran-2-carboxylic acid {1-[6-oxo-4-(pyridin-3-ylmethanesulfonyl)-hexahydro-pyrrolo [3,2-b]pyrrole-1-carbonyl]-butyl}-amide;
Thieno[3,2-b]thiophene-2-carboxylic acid {1-[6-oxo-4-(pyridin-3-ylmethanesulfonyl)-hexahydro-pyrrolo[3,2-b]pyrrole-1-carbonyl]-butyl}-amide;
3-Methyl-benzofuran-2-carboxylic acid {1-[6-oxo-4-(pyridin-3-ylmethanesulfonyl)-hexahydro-pyrrolo[3,2-b]pyrrole-1-carbonyl]-butyl}-amide;
Quinoxaline-2-carboxylic acid {1-[6-oxo-4-(pyridin-3-ylmethanesulfonyl)-hexahydro-pyrrolo[3,2-b]pyrrole-1-carbonyl]-butyl}-amide;
Benzo[1,3]dioxole-5-carboxylic acid {1-[6-oxo-4-(pyridin-3-ylmethanesulfonyl)-hexahydro-pyrrolo[3,2-b]pyrrole-1-carbonyl]-butyl}-amide;
Quinoline-6-carboxylic acid {1-[6-oxo-4-(pyridin-3-ylmethanesulfonyl)-hexahydro-pyrrolo[3,2-b]pyrrole-1-carbonyl]-butyl}-amide;
Furan-2-carboxylic acid {1-[6-oxo-4-(pyridin-3-ylmethanesulfonyl)-hexahydro-pyrrolo[3,2-b]pyrrole-1-carbonyl]-butyl}-amide;
Thiophene-2-carboxylic acid {1-[6-oxo-4-(pyridin-3-ylmethanesulfonyl)-hexahydro-pyaolo[3,2-b]pyuole-1-carbonyl]-butyl}-amide;
N-{1-[6-oxo-4-(pyridin-3-ylmethanesulfonyl)-hexahydro-pyrrolo[3,2-b]pyrrole-1-carbonyl]-butyl}-4-trifluoromethyl-benzamide;
Furan-3-carboxylic acid {1-[6-oxo-4-(pyridin-3-ylmethanesulfonyl)-hexahydro-pyrrolo[3,2-b]pyrrole-1-carbonyl]-butyl}-amide;
Thiophene-3-carboxylic acid {1-[6-oxo-4-(pyridin-3-ylmethanesulfonyl)-hexahydro-pyrrolo[3,2-b]pyrrole-1-carbonyl]-butyl}-anude;
N-{1-[6-oxo-4-(pyridin-3-ylmethanesulfonyl)-hexahydro-pyrrolo[3,2-b]pyrrole-1-carbonyl]-butyl}-4-phenoxy-benzamide;
N-{1-[6-oxo-4-(pyridin-3-ylmethanesulfonyl)-hexahydro-pyrrolo[3,2-b]pyrrole-1-carbonyl]-butyl}-4-(4-methyl-pipera zin-1-yl)-benzamide;
4-Methyl-N-{1-[6-ox0-4-(pyridin-3-ylmethanesulfonyl)-hexahydro-pyrrolo[3,2-b]pyrrote-1-carbonyl]-butyl}-benzamide;
4-Methoxy-N-{1-[6-oxo-4-(pyridin-3-ylmethanesulfonyl)-hexahydro-pyrrolo[3,2-b]pyrrole-1-carbonyt]-butyt}-benzamide;
4-Isopropyl-N-{1-[6-oxo-4-(pyridin-3-ylnlethanesulfonyl)-hexahydro-pyrrolo[3,2-b]pyrrole-1-carbonyl]-butyl}-benzamide;
N-{1-[6-oxo-4-(pyridin-3-ylmethanesulfonyl)-hexahydro-pyrrolo[3,2-b]pyrrole-1-carbonyl]-butyl}-4-vinyl-benzamide;
4-Imidazol-1-yl-N-{1-[6-oxo-4-(pyridin-3-ylmethanesulfonyl)-hexahydro-pyrrolo[3,2-b]pyrrole-1-carbonyl]-butyl}-benzamide;
N-{1-[6-oxo-4-(pyridin-3-ylmethanesulfonyl)-hexahydro-pyrrolo[3,2-b]pyrrole-1-carbonyl]-butyl}-4-thiophen-2-yl-benzamide;
N-{1-[6-oxo-4-(pyridin-3-ylmethanesulfonyl)-hexahydro-pyrrolo[3,2-b]pyrrole-1-carbonyl]-butyl}-4-oxazol-5-yl-benzamide;
5-Phenyl-thiophene-2-carboxylic acid {1-[6-oxo-4-(pyridin-3-ylmethanesulfonyl)-hexahydro-pyrrolo[3,2-b]pyrrole-1-carbonyl]-butyl}-amide;
N-{1-[b-oxo-4-(pyridin-3-ylmethanesulfonyl)-hexahydro-pyrrolo[3,2-b]pyrrole-1-carbonyl]-butyl}-4-trifluoromethoxy-benzamide;
5-Pyridin-2-yl-thiophene-2-carboxylic acid {1-[6-oxo-4-(pyridin-3-ylmethanesulfonyl)-hexahydro-pyrrolo[3,2-b]pyrrole-1-carbonyl]-butyl}-amide;
4-Difluoromethoxy-N-{1-[6-oxo-4-(pyridin-3-ylmethanesulfonyl)-hexahydro-pyrrolo[3,2-b]pyrrole-1-carbonyl]-butyl}-benzamide;
N-{1-[6-oxo-4-(pyridin-3-ylmethanesulfonyl)-hexahydro-pyrrolo[3,2-b]pyrrole-1-carbonyl]-butyl}-4-morpholin-4-yi-benzainide;
Naphthalene-2-carboxylic acid {3-methyl-1-[6-oxo-4-(1-oxy-pyridin-2-ylmethanesulfonyl)hexahydro-pyrrolo[3,2-b]pyrrole-1-carbonyl]-butyl}-amide;
Quinoline-2-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-amide;
Benzo[b]thiophene-2-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-amide;
Benzofuran-2-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-amide;
Biphenyl-4-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-amide;
4-tert-Butyl-N-{3-methyl-1-[6-oxo-4-(1-oxy-pyridin-2-ylmethanesulfonyl)-hexahydro-pyrrolo[3,2-b]pyrrole-1-carbonyl]-butyl}-benzamide;
4-Dimethylamino-N-{3-methyl-1-[6-oxo-4-(1-oxy-pyridin-2-ylmethanesulfonyl)-hexahydro-pyrrolo[3,2-b]pyrrole-1-carbonyl]-butyl}-benzamide;
7-Methoxy-benzofuran-2-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-wnide;
{3-Methyl-1-[6-oxo-4-(1-oxy-pyridin-2-ylmethanesulfonyl)-hexahydro-pyrrolo[3,2-b]pyrrole-1-carbonyl]-butyl}-carbamic acid benzyl ester;
5-Methoxy-benzofuran-2-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-amide;
Thieno[3,2-b]thiophene-2-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-amide;
3-Methyl-benzofuran-2-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-amide;
Quinoxaline-2-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-amide;
Benzo[1,3]dioxole-5-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-amide;
Quinoline-6-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-amide;
Furan-2-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-amide;
Thiophene-2-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-amide;
N-{3-Methyl-1-[6-oxo-4-(1-oxy-pyridin-2-ylmethanesulfonyl)-hexahydro-pyrrolo[3,2-b]pyrrole-1-carbonyl]-butyl}-4-trifluoromethyl-benzamide;
Furan-3-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-amide;
Thiophene-3-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-amide;
N-{3-Methyl-1-[6-oxo-4-(1-oxy-pyridin-2-ylmethanesulfonyl)-hexahydro-pyrrolo[3,2-b]pyrrole-1-carbonyl]-butyl}-4-phenoxy-benzamide;
N-{3-Methyl-1-[6-oxo-4-(1-oxy-pyridin-2-ylmethanesulfonyl)-hexahydro-pyrrolo[3,2-b]pyrrole-1-carbonyl]-butyl}-4-(4-methyl-piperazin-1-yl)-benzamide;
4-Methyl-N-{3-methyl-1-[6-oxo-4-(1-oxy-pyridin-2-ylmethanesulfonyl)-hexahydro-pyrrolo[3,2-b]pyrrole-1-carbonyl]-butyl}-benzamide;
4-Methoxy-N-{3-methyl-1-[6-oxo-4-(1-oxy-pyridin-2-ylmethanesulfonyl)-hexahydro-pyrrolo[3,2-b]pyrrole-1-carbonyl]-butyl}-benzamide;
4-Isopropyl-N-{3-methyl-1-[6-oxo-4-(1-oxy-pyridin-2-ylmethanesulfonyl)-hexahydro-pyrrolo[3,2-b]pyrrole-1-carbonyl]-butyl}-benzamide;
N-{3-Methyl-1-[6-oxo-4-(1-oxy-pyridin-2-ylmethanesulfonyl)-hexahydro-pyrrolo[3,2-b]pyrrole-1-carbonyl]-butyl}-4-vinyl-benzamide;
4-Imidazol-1-yl-N-{3-methyl-1-[6-oxo-4-(1-oxy-pyfidin-2-ylmethanesulfonyl)-hexahydro-pyrrolo[3,2-b]pyrrole-1-carbonyl]-butyl}-benzamide;
N-{3-Methyl-1-[6-oxo-4(1-oxy-pyridin-2-ylmethanesulfonyl)-hexahydro-pyrrolo[3,2-b]pyrrole-1-carbonyl]-butyl}-4-thiophen-2-yl-benzwnide;
N-{3-Methyl-1-[6-oxo-4-(1-oxy-pyridin-2-ylmethanesulfonyl)-hexahydro-pyrrolo[3,2-b]pyrrole-1-carbonyl]-butyl}-4-oxazol-5-yl-benzamide;
5-Phenyl-thiophene-2-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-amide;
N-{3-Methyl-1-[6-oxo-4-(1-oxy-pyridin-2-ylmethanesulfonyl)-hexahydro-pyrrolo[3,2-b]pyrrole-1-carbonyl]-butyl}-4-trifluoromethoxy-benzamide;
5-Pyridin-2-yl-thiophene-2-carboxylic acid {3-methyl-1-[6-oxo-4-(1-oxy-pyridin-2-ylmethanesulfonyl)-hexahydro-pyrrolo[3,2-b]pyrrole-1-carbonyl]-butyl}-amide;
4-Difluoromethoxy-N-{3-methyl-1-[6-oxo-4-(1-oxy-pyridin-2-ylmethanesulfonyl)-hexahydro-pyrrolo[3,2-b]pyrrole-1-carbonyl]-butyl}-benzamide;
N-{3-Methyl-1-[6-oxo-4-(1-oxy-pyridin-2-ylmethanesulfonyl)-hexahydro-pyrrolo[3,2-b]pyrrole-1-carbonyl]-butyl}-4-morpholin-4-yl-benzamide;
Naphthalene-2-carboxylic acid {1-[6-oxo-4-(1-oxy-pyridin-2-ylmethanesulfonyl)hexahydro-pyrrolo[3,2-b]pyrrole-1-carbonyl]-butyl}-amide;
Quinoline-2-carboxylic acid {1-[6-oxo-4-(1-oxy-pyridin-2-ylmethanesulfonyl)-hexahydro-pyrrolo[3,2-b]pyrrole-1-carbonyl]-butyl}-amide;
Benzo[b]thiophene-2-carboxylic acid {1-[6-oxo-4-(1-oxy-pyridin-2-ylmethanesulfonyl)-hexahydro-pyrrolo[3,2-b]pyrrole-1-carbonyl]-butyl}-amide;
Benzokran-2-carboxylic acid {1-[6-oxo-4-(1-oxy-pyridin-2-ylmethanesulfonyl)-hexahydro-pyrrolo[3,2-b]pyrrole-1-carbonyl]-butyl} -amide;
Biphenyl-4-carboxylic acid {1-[6-oxo-4(1-oxy-pyridin-2-ylmethanesulfonyl)-hexahydro-pyrrolo[3,2-b]pyrrole-1-carbonyl]-butyl}-amide;
4-tert-Butyl-N-{1-[6-oxo-4-(1-oxy-pyridin-2-ylmethanesulfonyl)-hexahydro-pyrrolo[3,2-b]pyrrole-1-carbonyl]-butyl}-bemamide;
4-Dimethylamino-N-{1-[6-oxo-4-(1-oxy-pyridin-2-ylmethanesulfonyl)-hexahydro-pyrrolo[3,2-b]pyrrole-1-carbonyl]-butyl}-benzamide;
7-Methoxy-benzofuran-2-carboxylic acid {1-[6-oxo-4-(1-oxy-pyridin-2-ylmethanesulfonyl)-hexahydro-pyrmlo[3,2-b]pyrrole-1-carbonyl]-butyl}-amide;
{1-[6-oxo-4-(1-oxy-pyridin-2-ylmethanesulfonyl)-hexahydro-pyrrolo[3,2-b]pyrrole-1-carbonyl]-butyl}-carbamic acid benzyl ester;
5-Methoxy-benzofuran-2-carboxylic acid {1-[6-oxo-4-(1-oxy-pyridin-2-ylmethanesulfonyl)-hexahydro-pyrrolo[3,2-b]pyrrole-1-carbonyl]-butyl}-amide;
Thieno[3,2-b]thiophene-2-carboxylic acid {1-[6-oxo-4-(1-oxy-pyridin-2-ylmethanesulfonyl)-hexahydro-pyrrolo[3,2-b]pyrrole-1-carbonyl]-butyl}-amide;
3-Methyl-benzofuran-2-carboxylic acid {1-[6-oxo-4-(1-oxy-pyridin-2-ylmethanesulfonyl)-hexahydro-pyrrolo[3,2-b]pyrrole-1-carbonyl]-butyl}-amide;
Quinoxaline-2-carboxylic acid {1-[6-oxo-4-(1-oxy-pyridin-2-ylmethanesulfonyl)-hexahydro-pyrrolo[3,2-b]pyrrole-1-carbonyl]-butyl}-amide;
Benzo[1,3]dioxole-5-carboxylic acid {1-[6-oxo-4-(1-oxy-pyridin-2-ylmethanesulfonyl)-hexahydro-pyrrolo[3,2-b]pyrrole-1-carbonyl]-butyl}-amide;
Quinoline-6-carboxylic acid {1-[6-oxo-4-(1-oxy-pyridin-2-ylinethanesulfonyl)-hexahydro-pyrrolo[3,2-b]pyrrole-1-carbonyl]-butyl}-amide;
Furan-2-carboxylic acid {1-[6-oxo-4-(1-oxy-pyridin-2-ylmethanesulfonyl)-hexahydro-pyrrolo[3,2-b]pyrrole-1-carbonyl]-butyl}-amide;
Thiophene-2-carboxylic acid {1-[6-oxo-4-(1-oxy-pyridin-2-ylmethanesulfonyl)-hexahydro-pyaolo[3,2-b]pyrrole-1-carbonyl]-butyl}-amide;
N-{1-[6-oxo-4-(1-oxy-pyridin-2-ylmethanesulfonyl)-hexahydro-pyrrolo[3,2-b]pyrrole-1-carbonyl]-butyl}-4-trifluorom ethyl-benzamide;
Furan-3-carboxylic acid {1-[6-oxo-4-(1-oxy-pyridin-2-ylinethanesulfonyl)-hexahydro-pyrrolo[3,2-b]pyrrole-1-carbonyl]-butyl}-amide;
Thiophene-3-carboxylic acid {1-[6-oxo-4-(1-oxy-pyridin-2-ylmethanesulfonyl)-hexahydro-pyrrolo[3,2-b]pyrrole-1-carbonyl]-butyl}-amide;
N-{1-[6-oxo-4-(1-oxy-pyridin-2-ylmethanesulfonyl)-hexahydro-pyrrolo[3,2-b]pyrrole-1-carbonyl]-butyl}-4-phenoxy-benzamide;
N-{1-[6-oxo-4-(1-oxy-pyridin-2-ylmethanesulfonyl)-hexahydro-pyrrolo[3,2-b]pyrrole-1-carbonyl]-butyl}-4-(4-methyl-piperazn-1-yl)-benzamide;
4-Methyl-N-{1-[6-oxo-4-(1-oxy-pyridin-2-ylmethanesulfonyl)-hexahydro-pyrrolo[3,2-b]pyrrole-1-carbonyl]-butyl -benzamide;
4-Methoxy-N-{1-[6-oxo-4-(1-oxy-pyridin-2-ylmetbanesulfonyl)-hexahydro-pyrrolo[3,2-b]pyrrole-1-carbonyl]-butyl}-benzamide;
4-Isopropyl-N-{1-[6-oxo-4-(1-oxy-pyridin-2-ylmethanesulfonyl)-hexahydro-pyrrolo[3,2-b]pyrrole-1-carbonyl]-butyl}-benzamide;
N-{1-[6-oxo-4-(1-oxy-pyridin-2-ylmethanesulfonyl)-hexahydro-pyrrolo[3,2-b]pyrrole-1-carbonyl]-butyl}-4-vinyl-benzamide;
4-Imidazol-1-yl-N-{1-[6-oxo-4-(1-oxy-pyridin-2-ylmethanesulfonyl)-hexahydro-pyrrolo[3,2-b]pyrrole-1-carbonyl]-butyl}-benzamide;
N-{1-[6-oxo-4-(1-oxy-pyridin-2-ylmethanesulfonyl)-hexahydro-pyrrolo[3,2-b]pyrrole-1-carbonyl]-butyl}-4-thiophen-2-yl-benzamide;
N-{1-[6-oxo-4-(1-oxy-pyridin-2-ylmethanesulfonyl)-hexahydro-pyrrolo[3,2-b]pyrrole-1-carbonyl]-butyl}-4-oxazol-5-yl-benzamide;
5-Phenyl-thiophene-2-carboxylic acid {1-[6-oxo-4-(1-oxy-pyridin-2-ylmethanesulfonyl)-hexahydro-pyrrolo[3,2-b]pyrrole-1-carbonyl]-butyl}-amide;
N-{1-[6-oxo-4-(1-oxy-pyridin-2-ylmethanesulfonyl)-hexahydro-pyrrolo[3,2-b]pyrrole-1-carbonyl]-butyl}-4-trifluoromethoxy-benzamide;
5-Pyridin-2-yl-thiophene-2-carboxylic acid {1-[6-oxo-4-(1-oxy-pyridin-2-ylmethanesulfonyl)-hexahydro-pyrrolo[3,2-b]pyrrole-1-carbonyl]-butyl}-amide;
4-Difluorornethoxy-N-{1-[6-oxo-4-(1-oxy-pyridin-2-ylmethanesulfonyl)-hexahydro-pyrrolo[3,2-b]pyrrole-1-carbonyl]-butyl}-benzamide;
N-{1-[6-oxo-4-(1-axy-pyridin-2-ylmethanesulfonyl)-hexahydro-pyrrolo[3,2-b]pyrrole-1-carbonyl]-butyl}-4-morpholin-4-yl-benzamide;
Naphthalene-2-carboxylic acid {3-methyl-1-[6-oxo-4-(1-oxy-pyridin-3-ylmethanesulfonyl)hexahydro-pyrrolo[3,2-b]pyrrole-1-carbonyl]-butyl}-amide;
Quinoline-2-carboxylic acid {3-metixyl-1-[6-oxo4-(1-oxy-pyridin-3-ylmethanesulfony)-hexahydro-pyrrolo[3,2-b]pyrrole-1-carbonyl]-butyl}-amide;
Benzo[b]thiophene-2-carboxylic acid {3-methyl-1-[6-oxo-4-(1-oxy-pyridin-3-ylmethanesulfonyl)-hexahydro-pyrrolo[3,2-b]pyrrole-1-carbonyl]-butyl}-amide;
Benzofuran-2-carboxylic acid {3-methyl-1-[6-oxo-4-(1-oxy-pyridin-3-ylmethanesulfonyl)-hexahydro-pyrrolo[3,2-b]pyrrole-1-carbonyl]-butyl}-amide;
Biphenyl-4-carboxylic acid {3-methyl-1-[6-oxo-4-(1-oxy-pyridin-3-ylmethanesulfonyl)-hexahydro-pyrrolo[3,2-b]pyrrole-1-carbonyl]-butyl}-amide;
4-tert-Butyl-N-{3-methyl-1-[6-oxo-4-(1-oxy-pyridin-3-ylmethanesulfonyl)-hexahydro-pyrrolo[3,2-b]pyrrole-1-carbonyl]-butyl}-benzamide;
4-Dimethylamino-N-{3-methyl-1-[6-oxo-4-(1-oxy-pyridin-3-ylmethanesulfonyl)-hexahydro-pyrrolo[3,2-b]pyrrole-1-carbonyl]-butyl}-benzamide;
7-Methoxy-benzofuran-2-carboxylic acid {3-methyl-1-[6-oxo-4-(1-oxy-pyridin-3-ylmethanesulfonyl)-hexahydro-pyaolo[3,2-b]pyrrole-1-carbonyl]-butyl}-amide;
{3-Methyl-1-[6-oxo-4(1-oxy-pyridin-3-ylmethanesulfonyl)-hexahydm-pyrrolo[3,2-b]pyrrole-1-bonyl]-butyl}-carbamic acid benzyl ester;
5-Methoxy-benzofuran-2-carboxylic acid {3-methyl-1-[6-oxo-4-(1-oxy-pyridin-3-ylmethanesulfonyl)-hexahydro-pyrrolo[3,2-b]pyrrole-1-carbonyl]-butyl}-amide;
Thieno[3,2-b]thiophene-2-carboxylic acid {3-methyl-1-[6-oxo-4-(1-oxy-pyridin-3-ylmethanesulfonyl)-hexahydro-pyrrolo[3,2-b]pyrrole-1-carbonyl]-butyl}-amide;
3-Methyl-bemofiuan-2-carboxylic acid {3-methyl-1-[6-oxo-4-(1-oxy-pyridin-3-ylmethanesulfonyl)-hexahydro-pyrrolo[3,2-b]pyrrole-1-carbonyl]-butyl}-amide;
Quinoxaline-2-carboxylic acid {3-methyl-1-[6-oxo-4-(1-oxy-pyridin-3-ylmethanesulfonyl)-hexahydro-pyrrolo[3,2-b]pyrrole-1-carbonyl]-butyl}-amide;
Benzo[1,3]dioxole-5-carboxylic acid {3-methyl-1-[6-oxo-4-(1-oxy-pyridin-3-ylmethanesulfonyl)-hexahydro-pyrrolo[3,2-b]pyrrole-1-carbonyl]-butyl}-amide;
Quinoline-6-carboxylic acid {3-methyl-1-[6-oxo-4-(1-oxy-pyridin-3-ylmethanesulfonyl)-hexahydro-pyrrolo[3,2-b]pyrrole-1-carbonyl]-butyl}-amide;
Furan-2-carboxylic acid {3-methyl-1-[6-oxo-4-(1-oxy-pyridin-3-ylmethanesulfonyl)-hexahydro-pyrrolo[3,2-b]pyrrole-1-carbonyl]-butyl}-amide;
Thiophene-2-carboxylic acid {3-methyl-1-[6-oxo-4-(1-oxy-pyridin-3-ylmethanesulfonyl)-hexahydro-pyrrolo[3,2-b]pyrrole-1-carbonyl]-butyl}-amide;
N-{3-Methyl-1-[6-oxo-4-(1-oxy-pyridin-3-ylmethanesulfonyl)-hexahydro-pyrrolo[3,2-b]pyrrole-1-carbonyl]-butyl}-4-trifluoromethyl-benzamide;
Furan-3-carboxylic acid {3-methyl-1-[6-oxo-4-(1-oxy-pyridin-3-ylmethanesulfonyl)-hexahydro-pyrrolo[3,2-b]pyrrole-1-carbonyl]-butyl}-amide;
Thiophene-3-carboxylic acid {3-methyl-1-[6-oxo-4-(1-oxy-pyridin-3-ylmethanesulfonyl)-hexahydro-pyrrolo[3,2-b]pyrrole-1-carbonyl]-butyl}-anfde;
N- {3-Methyl-1-[6-oxo-4-(1-oxy-pyridin-3-ylmethanesulfonyl)-hexahydro-pyrrolo[3,2-b]pyrrole-1-carbonyl]-butyl}-4-phenoxy-benzamide;
N-{3-Methyl-1-[6-oxo-4-(1-oxy-pyridin-3-ylmethanesulfonyl)-hexahydro-pyrrolo[3,2-b]pyrrole-1-carbonyl]-butyl}-4-(4-methyl-piperazin-1-yl)-benzamide;
4-Methyl-N-{3-methyl-1-[6-oxo-4-(1-oxy-pyridin-3-ylmethanesulfonyl)-hexahydro-pyrrolo[3,2-b]pyrrole-1-carbonyl]-butyl}-benzamide;
4-Methoxy-N-{3-methyl-1-[6-oxo-4-(1-oxy-pyridin-3-ylmethanesulfonyl)-hexahydro-pyrrolo[3,2-b]pyrrole-1-carbony

l]-butyl}-benzamide;
4-Isopropyl-N-{3-methyl-1-[6-oxo-4-(1-oxy-pyridin-3-ylmethanesulfonyl)-hexahydro-pyaolo[3,2-b]pyrrole-1-carbon yl]-butyl}-benzamide;
N-{3-Methyl-1-[6-oxo-4-(1-oxy-pyridin-3-ylmethanesulfonyl)-hexahydro-pyrrolo[3,2-b]pyrrole-1-carbonyl]-butyl}-4-v inyl-benzamide;
4-Imidazol-1-yl-N-{3-methyl-1-[6-oxo-4-(1-oxy-pyridin-3-ylmethanesulfonyl)-hexahydro-pyrrolo[3,2-b]pyrrole-1-car bonyl]-butyl}-benzamide;
N-{3-Methyl-1-[6-oxo-4-(1-oxy-pyridin-3-ylmethanesulfonyl)-hexahydro-pyrrolo[3,2-b]pyrrole-1-carbonyl]-butyl}-4-t hiophen-2-yl-benzamide;
N-{3-Methyl-1-[6-oxo-4-(1-oxy-pyridin-3-ylmethanesulfonyl)-hexahydro-pyrrolo[3,2-b]pyrrole-1-carbonyl]-butyl}-4-oxazol-5-yl-benzamide;
5-Phenyl-thiophene-2-carboxylic acid {3-methyl-1-[6-oxo-4-(1-oxy-pyridin-3-ylmethanesulfonyl)-hexahydro-pyrrolo[3,2-b]pyrrole-1-carbonyl]-butyl}-amide;
N-{3-Methyl-1-[6-oxo-4-(1-oxy-pyridin-3-ylmethanesulfonyl)-hexahydro-pyrroio[3,2-b]pyrrole-1-carbonyl]-butyl}-4-trifluoromethoxy-benzanide;
5-Pyridin-2-yl-thiophene-2-carboxylic acid {3-methyl-1-[6-oxo-4-(1-oxy-pyridin-3-ylme+anesulfonyl)-hexahydro-pyrrolo[3,2-b]pyrrole-1-carbonyl]-butyl}-amide;
4-Difluoromethoxy-N-{3-methyl-1-[6-oxo-4-(1-oxy-pyridin-3-ylmethanesulfonyl)-hexahydro-pyrrolo[3,2-b]pyrrole-1-carbonyl]-butyl}-benzamide;
N-{3-Methyl-1-[6-oxo-4-(1-oxy-pyridin-3-ylmethanesulfonyl)-hexahydro-pyrrolo[3,2-b]pyrrole-1-carbonyl]-butyl}-4-morpholin-4-yl-benzamide;
Naphthalene-2-carboxylic acid {1-[6-oxo-4-(1-oxy-pyridin-3-ylmethanesulfonyl)hexahydro-pyrrolo[3,2-b]pyrrole-1-carbonyl]-butyl}-amide;
Quinoline-2-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
Benzo[b]thiophene-2-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
Benzofuran-2-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
Biphenyl-4-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
4-tert-Butyl-N-{1-[6-oxo-4-(1-oxy-pyridin-3-ylmethanesulfonyl)-hexahydro-pyrrolo[3,2-b]pyrrole-1-carbonyl]-butyl}-benzamide;
4-Dimethylamino-N-{1-[6-oxo-4-(1-oxy-pyridin-3-ylmethanesulfonyl)-hexahydro-pyrrolo[3,2-b]pyrrole-1-carbonyl]-butyl}-benzamide;
7-Methoxy-benzofuran-2-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
{1-[6-oxo-4-(1-oxy-pyridin-3-ylmethanesulfonyl)-hexahydro-pyrrolo[3,2-b]pyrrole-1-carbonyl]-butyl}-carbamic acid benzyl ester;
5-Methoxy-benzofuran-2-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
Thieno[3,2-b]thiophene-2-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
3-Methyl-benzofuran-2-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
Quinoxaline-2-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
Benzo[1,3]dioxole-5-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
Quinoline-6-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
Furan-2-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
Thiophene-2-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
N-{1-[6-oxo-4-(1-oxy-pyridin-3-ylmethanesulfonyl)-hexahydro-pyrrolo[3,2-b]pyrrole-1-carbonyl]-butyl}-4-trifluorom ethyl-benzamide;
Furan-3-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
Thiophene-3-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
N-{1-[6-oxo-4-(1-oxy-pyridin-3-ylmethanesulfonyl)-hexahydro-pyrrolo[3,2-b]pyrrole-1-carbonyl]-butyl}-4-phenoxy-benzamide;
N-{1-[6-oxo-4-(1-oxy-pyridin-3-ylmethanesulfonyl)-hexahydro-pyrrolo[3,2-b]pyrrole-1-carbonyl]-butyl}-4-(4-methyl-piperazin-1-yl)-benzamide;
4-Methyl-N-{1-[6-oxo-4-(1-oxy-pyridin-3-ylmethanesulfonyl)-hexahydro-pyrrolo[3,2-b]pyrrole-1-carbonyl]-butyl}-benzamide;
4-Methoxy-N-{1-[6-oxo-4-(1-oxy-pyridin-3-ylmethanesulfonyl)-hexahydro-pyrrolo[3,2-b]pyrrole-1-carbonyl]-butyl}-benzamide;
4-Isopropyl-N-{1-[6-oxo-4-(1-oxy-pyridin-3-ylmethanesulfonyl)-hexahydro-pyrrolo[3,2-b]pyrrole-1-carbonyl]-butyl}-benzamide;
N-{1-[6-oxo-4-(1-oxy-pyridin-3-ylmethanesulfonyl)-hexahydro-pyrrolo[3,2-b]pyrrole-1-carbonyl]-butyl}-4-vinyl-benzamide;
4-Imidazol-1-yl-N-{1-[6-oxo-4-(1-oxy-pyridin-3-ylmethanesulfonyl)-hexahydro-pyrrolo[3,2-b]pyrrole-1-carbonyl]-butyl}-benzamide;
N-{1-[6-oxo-4-(1-oxy-pyridin-3-ylmethanesulfonyl)-hexahydro-pyrrolo[3,2-b]pyrrole-1-carbonyl]-butyl}-4-thiophen-2-yl-benzamide;
N-{1-[6-oxo-4-(1-oxy-pyridin-3-ylmethanesulfonyl)-hexahydro-pyrrolo[3,2-b]pyrrole-1-carbonyl]-butyl}-4-oxazol-5-yl-benzamide;
5-Phenyl-thiophene-2-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
N-{-[6-oxo-4-(l-oxy-pyridin-3-ylmethanesulfonyl)-hexahydro-pyrrolo[3,2-b]pyrrole-1-carbonyl]-butyl}-4-trifluoromethoxy-benwmide;
5-Pyridin-2-yl-thiophene-2-carboxylic acid {1-[6-oxo-4-(1-oxy-pyridin-3-ylmethanesulfonyl)-hexahydro-pyrrolo[3,2-b]pyrrole-1-carbonyl]-butyl}-amide;
4-Difluoromethoxy-N-{1-[6-oxo-4-(1-oxy-pyridin-3-ylmethanesulfonyl)-hexahydro-pyrrolo[3,2-b]pyrrole-1-carbonyl]-butyl}-benzamide;
N-{1-[6-oxo-4-(1-oxy-pyridin-3-ylmethanesulfonyl)-hexahydro-pyrrolo[3,2-b]pyrrole-1-carbonyl]-butyl}-4-morpholin-4-yl-benzamide;
4-tert-Butyl-N-{1-[6-oxo-4-(pyridine-2-sulfonyl)-hexahydro-pyrrolo[3,2-b]pyrrole-1-carbonyl]-cyclohexyl}-benzamide;
5-Methoxy-benzofuran-2-carboxylic acid {1-[6-oxo-4-(pyridine-2-sulfonyl)-hexahydro-pyrrolo[3,2-b]pyrrole-1-carbonyl]-cyclohexyl}-amide;
Thieno[3,2-b]thiophene-2-carboxylic acid {1-[6-oxo-4-(pyridine-2-sulfonyl)-hexahydro-pyrrolo[3,2-b]pyrrole-1-carbonyl]-cydohexyl}-amide;
3-Methyl-benzofuran-2-carboxylic acid {1-[6-oxo-4-(pyridine-2-sulfonyl)-hexahydro-pyrrolo[3,2-b]pyrrole-1-carbonyl]-cyclohexyl}-amide;
N-{1-[6-oxo-4-(pyridine-2-sulfonyl)-hexahydro-pyrrolo[3,2-b]pyrrole-1-carbonyl]-cyclohexyl}-4-phenoxy-benzamide;
N-{1-[6-oxo-4-(pyridine-2-sulfonyl)-hexahydro-pyrrolo[3,2-b]pyrrole-1-carbonyl]-cyclohexyl}-4-thiophen-2-yl-benzamide;
4-tert-Butyl-N-{1-cyclopropylmethyl-2-oxo-2-[6-oxo-4-(pyridine-2-sulfonyl)-hexahydro-pyrrolo[3,2-b]pyrrol-1-yl]-ethyl}-benzamide;
5-Methoxy-benzofuran-2-carboxylic acid {1-cyclopropylmethyl-2-oxo-2-[6-oxo-4-(pyridine-2-sulfonyl)-hexahydro-pyrrolo[3,2-b]pyrrol-1-yl]-ethyl}-amide;
Thieno[3,2-b]thiophene-2-carboxylic acid {1-cyclopropylmethyl-2-oxo-2-[6-oxo-4-(pyridine-2-sulfonyl)-hexahydro-pyrrolo[3,2-b]pyrrol-1-yl]-ethyl}-amide;
3-Methyl-benzofuran-2-carboxylic acid {1-cyclopropylmethyl-2-oxo-2-[6-oxo-4-(pyridine-2-sulfonyl)-hexahydro-pyrrolo[3,2-b]pyrrol-1-yl]-ethyl}-amide;
N-{1-cyclopropylmethyl-2-oxo-2-[6-oxo-4-(pyridine-2-sulfonyl)-hexahydro-pyrrolo[3,2-b]pyrrol-1-yl]-ethyl}-4-phenoxy-benzamide;
N-{1-cyclopropylmethyl-2-oxo-2-[6-oxo-4-(pyridine-2-sulfonyl)-hexahydro-pyrrolo[3,2-b]pyrrol-1-yl]-ethyl}-4-thiophen-2-yl-benzamide;
4-tert-Butyl-N-{2-oxo-2-[6-oxo-4-(pyridine-2-sulfonyl)-hexahydm-pyrrolo[3,2-b]pyrrol-1-yl]-1-Riophen-2-ylmethyl-ethyl}-benzamide;
5-Methoxy-benzofuran-2-carboxylic acid {2-oxo-2-[6-oxo-4-(pyridine-2-sulfonyl)-hexahydro-pyrrolo[3,2-b]pyrrol-1-yl]-1-thiophen-2-ylmethyl-ethyl}-amide;
Thieno[3,2-b]thiophene-2-carboxylic acid {2-oxo-2-[6-oxo-4-(pyridine-2-sulfonyl)-hexahydro-pyrrolo[3,2-b]pyrrol-1-yl]-1-thiophen-2-ylmethyl-ethyl}-amide;
3-Methyl-benzofuran-2-carboxylic acid {2-oxo-2-[6-oxo-4-(pyridine-2-sulfonyl)-hexahydro-pyrrolo[3,2-b]pyrrol-1-yl]-1-thiophen-2-ylmethyl-ethyl} -amide;
N-{2-oxo-2-[6-oxo-4-(pyridine-2-sulfonyl)-hexahydro-pyrrolo[3,2-b]pyrrol-1-yl]-1-thiophen-2-ylmethyl-ethyl}-4-phenoxy-benzamide;
N-{2-oxo-2-[6-oxo-4-(pyridine-2-sulfonyl)-hexahydro-pyrrolo[3,2-b]pyrrol-1-yl]-1-thiophen-2-ylmethyl-ethyl}-4-thiophen-2-yl-benzamide;
4-tert-Butyl-N-{1-[6-oxo-4-(pyridine-3-sulfonyl)-hexahydro-pyrrolo[3,2-b]pyrrole-1-carbonyl]-cyclohexyl}-benzamide;
5-Methoxy-benzofuran-2-carboxylic acid {1-[6-oxo-4-(pyridine-3-sulfonyl)-hexahydro-pyrrolo[3,2-b]pyrrole-1-carbonyl]-cyclohexyl}-amide;
Thieno[3,2-b]thiophene-2-carboxylic acid {1-[6-oxo-4-(pyridine-3-sulfonyl)-hexahydro-pyrrolo[3,2-b]pyrrole-1-carbonyl]-cyclohexyl}-amide;
3-Methyl-benzofuran-2-carboxylic acid {1-[6-oxo-4-(pyridine-3-sulfonyl)-hexahydro-pyrrolo[3,2-b]pyrrole-1-carbonyl]-cyclohexyl}-amide;
N-{1-[6-oxo-4-(pyridine-3-sulfonyl)-hexahydro-pyrrolo[3,2-b]pyrrole-1-carbonyl]-cyclohexyl}-4-phenoxy-benzamide;
N-{1-[6-oxo-4-(pyridine-3-sulfonyl)-hexahydro-pyrrolo[3,2-b]pyrrole-1-carbonyl]-cyclohexyl}-4-thiophen-2-yl-benzgnide;
4-tert-Butyl-N-{1-cyclopropylmethyl-2-oxo-2-[6-oxo-4-(pyridine-3-sulfonyl)-hexahydro-pyrrolo[3,2-b]pyrrol-1-yl]-eth yl}-benzamide;
5-Methoxy-benzofuran-2-carboxylic acid {1-cyclopropylmethyl-2-oxo-2-[6-oxo-4-(pyridine-3-sulfonyl)-hexahydro-pyrrolo[3,2-b]pyrrol-1-yl]-ethyl}-amide;
Thieno[3,2-b]thiophene-2-carboxylic acid {1-cyclopropylmethyl-2-oxo-2-[6-oxo-4-(pyridine-3-sulfonyl)-hexahydro-pyrrolo[3,2-b]pyrrol-1-yl]-ethyl}-amide;
3-Methyl-benzofuran-2-carboxylic acid {1-cyclopropylmethyl-2-oxo-2-[6-oxo-4-(pyridine-3-sulfonyl)-hexahydro-pyrrolo[3,2-b]pyrrol-1-yl]-ethyl}-amide;
N-{1-cyclopropylmethyl-2-oxo-2-[6-oxo-4-(pyridine-3-sulfonyl)-hexahydro-pyrrolo[3,2-b]pyrrol-1-yl]-ethyl}-4-phenoxy-benzamide;
N-{1-cyclopropylmethyl-2-oxo-2-[6-oxo-4-(pyridine-3-sulfonyl)-hexahydro-pyrrolo[3,2-b]pyrrol-1-yl]-ethyl}-4-thioph en-2-yl-benzamide;
4-tert-Butyl-N-{2-oxo-2-[6-oxo-4-(pyridine-3-sulfonyl)-hexahydro-pyrrolo(3,2-b]pyrrol-1-yl]-1-thiophen-2-ylmethyl-e thyl}-benzamide;
5-Methoxy-benzofuran-2-carboxylic acid {2-oxo-2-[6-oxo-4-(pyridine-3-sulfonyl)-hexahydro-pyrrolo[3,2-b]pyrrol-1-yl]-1-thiophen-2-ylmethyl-ethyl}-amide;
Thieno[3,2-b]thiophene-2-carboxylic acid {2-oxo-2-[6-oxo-4-(pyridine-3-sulfonyl)-hexahydro-pyrrolo[3,2-b]pyrrol-1-yl]-1-thiophen-2-ylmethyl-ethyl}-amide;
3-Methyl-benzofuran-2-carboxylic acid {2-oxo-2-[6-oxo-4-(pyridine-3-sulfonyl)-hexahydro-pyrrolo[3,2-b]pyrrol-1-yl]-1-thiophen-2-ylmethyl-ethyl}-amide;
N-{2-oxo-2-[6-oxo-4-(pyridine-3-sulfonyl)-hexahydro-pyrrolo[3,2-b]pyrrol-1-yl]-1-thiophen-2-ylmethyl-ethyl}-4-phen oxy-benzamide;
N-{2-oxo-2-[6-oxo-4-(pyridine-3-sulfonyl)-hexahydro-pyrrolo[3,2-b]pyrrol-1-yl]-1-thiophen-2-ylmethyl-ethyl}-4-thiop hen-2-yl-benzamide;
4-tert-Butyl-N-{1-[6-oxo-4-(1-oxy-pyridine-2-sulfonyl)-hexahydro-pyrrolo[3,2-b]pyrrole-1-carbonyl]-cyclohexyl}-benzamide;
5-Methoxy-benzofuran-2-carboxylic acid {1-[6-oxo-4-(1-oxy-pyridine-2-sulfonyl)-hexahydro-pyrrolo[3,2-b]pyrrole-1-carbonyl]-cyclohexyl}-amide;
Thieno[3,2-b]thiophene-2-carboxylic acid {1-[6-oxo-4-(1-oxy-pyridine-2-sulfonyl)-hexahydro-pyrrolo[3,2-b]pyrrole-1-carbonyl]-cyclohexyl}-amide;
3-Methyl-benzofuran-2-carboxylic acid {1-[6-oxo-4-(1-oxy-pyridine-2-sulfonyl)-hexahydro-pyrrolo[3,2-b]pyrrole-1-carbonyl]-cyclohexyl}-amide;
N-{1-[6-oxo-4-(1-oxy-pyridine-2-sulfonyl)-hexahydro-pyrrolo[3,2-b]pyrrole-1-carbonyl]-cyclohexyl}-4-phenoxy-benzamide;
N-{1-[6-oxo-4-(1-oxy-pyridine-2-sulfonyl)-hexahydro-pyrrolo[3,2-b]pyrrole-1-carbonyl]-cyclohexyl}-4-thiophen-2-yl-benzamide;
4-tert-Butyl-N-{1-cyclopropylmethyl-2-oxo-2-[6-oxo-4-(1-oxy-pyridine-2-sulfonyl)-hexahydro-pyrrolo[3,2-b]pyrrol-1-yl]-ethyl}-benzamide;
5-Methoxy-benzofiuran-2-carboxylic acid {1-cyclopropylmethyl-2-oxo-2-[6-oxo-4-(1-oxy-pyridine-2-sulfonyl)-hexahydro-pyrrolo[3,2-b]pyrrol-1-yl]-ethyl}-amide;
Thieno[3,2-b]thiophene-2-carboxylic acid {1-cyclopropylmethyl-2-oxo-2-[6-oxo-4-(1-oxy-pyridine-2-sulfonyl)-hexahydro-pyrrolo[3,2-b]pyrrol-1-yl]-ethyl}-amide;
3-Methyl-benzofuran-2-carboxylic acid {1-cyclopropylmethyl-2-oxo-2-[6-oxo-4-(1-oxy-pyridine-2-sulfonyl)-hexahydro-pyrrolo[3,2-b]pyrrol-1-yl]-ethyl}-amide;
N-{1-cyclopropylmethyl-2-oxo-2-[6-oxo-4-(1-oxy-pyridine-2-sulfonyl)-hexahydro-pyrrolo[3,2-b]pyrrol-1-yl]-ethyl}-4-phenoxy-benzamide;
N-{1-cyclopropylmethyl-2-oxo-2-[6-oxo-4-(1-oxy-pyridine-2-sulfonyl)-hexahydro-pyrrolo[3,2-b]pyrrol-1-yl]-ethyl}-4-thiophen-2-yl-benzamide;
4-tert-Butyl-N-{2-oxo-2-[6-oxo-4-(1-oxy-pyridine-2-sulfonyl)-hexahydro-pyrrolo[3,2-b]pyrrol-1-yl]-1-thiophen-2-ylmethyl-ethyl}-benzamide;
5-Methoxy-benzofuran-2-carboxylic acid {2-oxo-2-[6-oxo-4-(1-oxy-pyridine-2-sulfonyl)-hexahydro-pyrrolo[3,2-b]pyrrol-1-yl]-1-thiophen-2-ylmethyl-ethyl}-amide;
Thieno[3,2-b]thiophene-2-carboxylic acid {2-oxo-2-[6-oxo-4-(1-oxy-pyridine-2-sulfonyl)-hexahydro-pyrrolo[3,2-b]pyrrol-1-yl]-1-thiophen-2-ylmethyl-ethyl}-amide;
3-Methyl-benzofuran-2-carboxylic acid {2-oxo-2-[6-oxo-4-(1-oxy-pyridine-2-sulfonyl)-hexahydro-pyrrolo[3,2-b]pyrrol-1-yl]-1-thiophen-2-ylmethyl-ethyl}-amide;
N-{2-oxo-2-[6-oxo-4-(1-oxy-pyridine-2-sulfonyl)-hexahydro-pyrrolo[3,2-b]pyrrol-1-yl]-1-thiophen-2-ylmethyl-ethyl}-4-phenoxy-benzamide;
N-{2-oxo-2-[6-oxo-4-(1-oxy-pyridine-2-sulfonyl)-hexahydro-pyrrolo[3,2-b]pyrrol-1-yl]-1-thiophen-2-ylmethyl-ethyl}-4-thiophen-2-yl-benzamide;
4-tert-Butyl-N-{1-[6-oxo-4-(1-oxy-pyridine-3-sulfonyl)-hexahydro-pyrrolo[3,2-b]pyrrole-1-carbonyl]-cyclohexyl}-benzamide;
5-Methoxy-benzofuran-2-carboxylic acid {1-[6-oxo-4-(1-oxy-pyridine-3-sulfonyl)-hexabydro-pyrrolo[3,2-b]pyrrole-1-carbonyl]-cyclohexyl}-amide;
Thieno[3,2-b]thiophene-2-carboxylic acid {1-[6-oxo-4-(1-oxy-pyridine-3-sulfonyl)-hexahydro-pyrrolo[3,2-b]pyrrole-1-carbonyl]-cyclohexyl}-amide;
3-Methyl-benzofuran-2-carboxylic acid {1-[6-oxo-4-(1-oxy-pyridine-3-sulfonyl)-hexahydro-pyrrolo[3,2-b]pyrrole-1-carbonyl]-cyclohexyl}-amide;
N-{1-[6-oxo-4-(1-oxy-pyridine-3-sulfonyl)-hexahydro-pyrrolo[3,2-b]pyrrole-1-carbonyl]-cyclohexyl}-4-phenoxy-benzamide;
N-{1-[6-oxo-4-(1-oxy-pyridine-3-sulfonyl)-hexahydro-pyrrolo[3,2-b]pyrrole-1-carbonyl]-cyclohexyl}-4-thiophen-2-yl-benzamide;
4-tert-Butyl-N-{1-cyclopropylmethyl-2-oxo-2-[6-oxo-4-(1-oxy-pyridine-3-sulfonyl)-hexahydro-pyrrolo[3,2-b]pyrrol-1-yl]-ethyl}-benzamide;
5-Methoxy-benzofuran-2-carboxylic acid {1-cyclopropylmethyl-2-oxo-2-[6-oxo-4-(1-oxy-pyridine-3-sulfonyl)-hexahydro-pyrrolo[3,2-b]pyrrol-1-yl]-ethyl}-amide;
Thieno[3,2-b]thiophene-2-carboxylic acid {1-cyclopropylmethyl-2-oxo-2-[6-oxo-4-(1-oxy-pyridine-3-sulfonyl)-hexahydro-pyrrolo[3,2-b]pyrrol-1-yl]-ethyl}-amide;
3-Methyl-benzofuran-2-carboxylic acid {1-cyclopropylmethyl-2-oxo-2-[6-oxo-4-(1-oxy-pyridine-3-sulfonyl)-hexahydro-pyrrolo[3,2-b]pyrrol-1-yl]-ethyl}-amide;
N-{1-cyclopropylinethyl-2-oxo-2-[6-oxo-4-(1-oxy-pyridine-3-sulfonyl)-hexahydro-pyrrolo[3,2-b]pyrrol-1-yl]-ethyl}-4-phenoxy-benzamide;
N-{1-cyclopropylmethyl-2-oxo-2-[6-oxo-4-(1-oxy-pyridine-3-sulfonyl)-hexahydro-pyrrolo[3,2-b]pyrrol-1-yl]-ethyl}-4-t hiophen-2-yl-benzamide;
4-tert-Butyl-N-{2-oxo-2-[6-oxo-4-(1-oxy-pyridine-3-sulfonyl)-hexahydro-pyrrolo[3,2-b]pyrrol-1-yl]-1-thiophen-2-ylm ethyl-ethyl}-benzamide;
5-Methoxy-benzofuran-2-carboxylic acid {2-oxo-2-[6-oxo-4-(1-oxy-pyridine-3-sulfonyl)-hexahydro-pyrrolo[3,2-b]pyrrol-1-yl]-1-thiophen-2-ylmethyl-ethyl}-amide;
Thieno[3,2-b]thiophene-2-carboxylic acid {2-oxo-2-[6-oxo-4-(1-oxy-pyridine-3-sulfonyl)-hexahydro-pyrrolo[3,2-b]pyrrol-1-yl]-1-thiophen-2-ylmethyl-ethyl}-amide;
3-Methyl-benzofiuan-2-carboxylic acid {2-oxo-2-[6-oxo-4-(1-oxy-pyridine-3-sulfonyl)-hexahydro-pyrrolo[3,2-b]pyrrol-1-yl]-1-thiophen-2-ylmethyl-ethyl}-amide;
N-{2-oxo-2-[6-oxo-4-(1-oxy-pyridine-3-sulfonyl)-hexahydro-pyrrolo[3,2-b]pyrrol-1-yl]-1-thiophen-2-ylmethyl-ethyl}-4-phenoxy-benzamide;
N-{2-oxo-2-[6-oxo-4-(1-oxy-pyridine-3-sulfonyl)-hexahydro-pyrrolo[3,2-b]pyrrol-1-yl]-1-thiophen-2-ylmethyl-ethyl}-4-thiophen-2-yl-benzamide;
4-tert-Butyl-N-{1-[6-oxo-4-(pyridine-2-carbonyl)-hexahydro-pyrrolo[3,2-b]pyrrole-1-carbonyl]-cyclohexyl}-benzamide;
5-Methoxy-benzofuran-2-carboxylic acid {1-[6-oxo-4-(pyridine-2-carbonyl)-hexahydro-pyrrolo[3,2-b]pyrrole-1-carbonyl]-cyclohexyl}-amide;
Thieno[3,2-b]thiophene-2-carboxylic acid {1-[6-oxo-4-(pyridine-2-carbonyl)-hexahydro-pyrrolo[3,2-b]pyrrole-1-carbonyl]-cyclohexyl}-amide;
3-Methyl-benzofuran-2-carboxylic acid {1-[6-oxo-4-(pyridine-2-carbonyl)-hexahydro-pyrrolo[3,2-b]pyrrole-1-carbonyl]-cyclohexyl}-amide;
N-{1-[6-oxo-4-(pyridine-2-carbonyl)-hexahydro-pyrrolo[3,2-b]pyrrole-1-carbonyl]-cyclohexyl}-4-phenoxy-benzamide;
N-{1-[6-oxo-4-(pyridine-2-carbonyl)-hexahydro-pyrrolo[3,2-b]pyrrole-1-carbonyl]-cyclohexyl}-4-thiophen-2-yl-benzamide;
4-tert-Butyl-N-{1-cyclopropylmethyl-2-oxo-2-[6-oxo-4-(pyridine-2-carbonyl)-hexahydro-pyrrolo[3,2-b]pyrrol-1-yl]-ethyl}-benzamide;
5-Methoxy-benzofuran-2-carboxylic acid {1-cyclopropylmethyl-2-oxo-2-[6-oxo-4-(pyridine-2-carbonyl)-hexahydro-pyrrolo[3,2-b]pyrrol-1-yl]-ethyl}-amide;
Thieno[3,2-b]thiophene-2-carboxylic acid {1-cyclopropylmethyl-2-oxo-2-[6-oxo-4-(pyridine-2-carbonyl)-hexahydro-pyrrolo[3,2-b]pyrrol-1-yl]-ethyl}-amide;
3-Methyl-benzofuran-2-carboxylic acid {1-cyclopropylmethyl-2-oxo-2-[6-oxo-4-(pyridine-2-carbonyl)-hexahydro-pyrrolo[3,2-b]pyrrol-1-yl]-ethyl}-amide;
N-{1-cyclopropylmethyl-2-oxo-2-[6-oxo-4-(pyridine-2-carbonyl)-hexahydro-pyrrolo[3,2-b]pyrrol-1-yl]-ethyl}-4-phenoxy-benzamide;
N-{1-cyclopropylmethyl-2-oxo-2-[6-oxo-4-(pyridine-2-carbonyl)-hexahydro-pyrrolo[3,2-b]pyrrol-1-yl]-ethyl}-4-thiophen-2-yl-benzamide;
4-tert-Butyl-N-{2-oxo-2-[6-oxo-4-(pyridine-2-carbonyl)-hexahydro-pyrrolo(3,2-b]pyrrol-1-yl]-1-thiophen-2-ylmethyl-ethyl}-benzamide;
5-Methoxy-benzofuran-2-carboxylic acid {2-oxo-2-[6-oxo-4-(pyridine-2-carbonyl)-hexahydro-pyrrolo[3,2-b]pyrrol-1-yl]-1-thiophen-2-ylmethyl-ethyl}-amide;
Thieno[3,2-b]thiophene-2-carboxylic acid {2-oxo-2-[6-oxo-4-(pyridine-2-carbonyl)-hexahydro-pyrrolo[3,2-b]pyrrol-1-yl]-1-thiophen-2-ylmethyl-ethyl}-amide;
3-Methyl-benzofuran-2-carboxylic acid {2-oxo-2-[6-oxo-4-(pyridine-2-carbonyl)-hexahydro-pyrrolo[3,2-b]pyrrol-1-yl]-1-thiophen-2-ylmethyl-ethyl}-amide;
N-{2-oxo-2-[6-oxo-4-(pyridine-2-carbonyl)-hexahydro-pyrrolo[3,2-b]pyrrol-1-yl]-1-thiophen-2-ylmethyl-ethyl}-4-phenoxy-benzamide;
N-{2-oxo-2-[6-oxo-4-(pyridine-2-carbonyl)-hexahydro-pyrrolo[3,2-b]pyrrol-1-yl]-1-thiophen-2-ylmethyl-ethyl}-4-thiophen-2-yl-benzamide;
4-tert-Butyl-N-{1-[6-oxo-4-(2-pyridin-3-yl-acetyl)-hexahydro-pyrrolo[3,2-b]pyrrole-1-carbonyl]-cyclohexyl}-benzamide;
5-Methoxy-benzofuran-2-carboxylic acid {1-[6-oxo-4-(2-pyridin-3-yl-acetyl)-hexahydro-pyrrolo[3,2-b]pyrrole-1-carbonyl]-cyclohexyl}-amide;
Thieno[3,2-b]thiophene-2-carboxylic acid {1-[6-oxo-4-(2-pyridin-3-yl-acetyl)-hexahydro-pyrrolo[3,2-b]pyrrole-1-carbonyl]-cyclohexyl}-amide;
3-Methyl-benzofuran-2-carboxylic acid {1-[6-oxo-4-(2-pyridin-3-yl-acetyl)-hexahydro-pyrrolo[3,2-b]pyrrole-1-carbonyl]-cyclohexyl}-amide;
N-{1-[6-oxo-4-(2-pyridin-3-yl-acetyl)-hexahydro-pyrrolo[3,2-b]pycrole-1-carbonyl]-cyclohexyl}-4-phenoxy-benzamide;
N-{1-[6-oxo-4-(2-pyridin-3-yl-acetyl)-hexahydm-pyrrolo[3,2-b]pyrrole-1-carbonyl]-cyclohexyl}-4-thiophen-2-yl-benzamide;
4-tert-Butyl-N-{1-cyclopropylmethyl-2-oxo-2-[6-oxo-4-(2-pyridin-3-yl-acetyl)-hexahydro-pyrrolo[3,2-b]pyrrol-1-yl]-ethyl}-benzamide;
5-Methoxy-benzofuran-2-carboxylic acid {1-cyclopropylmethyl-2-oxo-2-[6-oxo-4-(2-pyridin-3-yl-acetyl)-hexahydro-pyrrolo[3,2-b]pyrrol-1-yl]-ethyl}-amide;
Thieno[3,2-b]thiophene-2-carboxylic acid {1-cyclopropylmethyl-2-oxo-2-[6-oxo-4-(2-pyridin-3-yl-acetyl)-hexahydro-pyrrolo[3,2-b]pyrrol-1-yl]-ethyl}-amide;
3-Methyl-benzofuran-2-carboxylic acid {1-cyclopropylmethyl-2-oxo-2-[6-oxo-4-(2-pyridin-3-yl-acetyl)-hexahydro-pyrrolo[3,2-b]pyrrol-1-yl]-ethyl}-amide;
N-{1-cyclopropylmethyl-2-oxo-2-[6-oxo-4-(2-pyridin-3-yl-acetyl)-hexahydro-pyrrolo[3,2-b]pyrrol-1-yl]-ethyl}-4-phenoxy-benzmnide;
N-{1-cyclopropylmethyl-2-oxo-2-[6-oxo-4-(2-pyridin-3-yl-acetyl)-hexahydro-pyrrolo[3,2-b]pyrrol-1-yl]-ethyl}-4-thiophen-2-yl-benzamide;
4-tert-Butyl-N-{2-oxo-2-[6-oxo-4-(2-pyridin-3-yl-acetyl)-hexahydro-pyrrolo[3,2-b]pyrrol-1-yl]-1-thiophen-2-ylmethyl-ethyl}-benzamide;
5-Methoxy-benzofuran-2-carboxylic acid {2-oxo-2-[6-oxo-4-(2-pyridin-3-yl-acetyl)-hexahydro-pyrrolo[3,2-b]pyrrol-1-yl]-1-thiophen-2-ylmethyl-ethyl}-amide;
Thieno[3,2-b]thiophene-2-carboxylic acid {2-oxo-2-[6-oxo-4-(2-pyridin-3-yl-acetyl)-hexahydro-pyrrolo[3,2-b]pyrrol-1-yl]-1-thiophen-2-ylmethyl-ethyl}-amide;
3-Methyl-benzofuran-2-carboxylic acid {2-oxo-2-[6-oxo-4-(2-pyridin-3-yl-acetyl)-hexahydro-pyrrolo[3,2-b]pyrrol-1-yl]-1-thiophen-2-ylmethyl-ethyl}-amide;
N-{2-oxo-2-[6-oxo-4-(2-pyridin-3-yl-acetyl)-hexahydro-pyrrolo[3,2-b]pyrrol-1-yl]-1-thiophen-2-ylmethyl-ethyl}-4-phenoxy-benzamide;
N-{2-oxo-2-[6-oxo-4-(2-pyridin-3-yl-acetyl)-hexahydro-pyrrolo[3,2-b]pyrrol-1-yl]-1-thiophen-2-ylinethyl-ethyl}-4-t hiophen-2-yl-benzamide;
4-tert-Butyl-N-{1-[6-oxo-4-(pyridin-2-ylmethanesulfonyl)-hexahydro-pyrrolo[3,2-b]pyrrole-1-carbonyl]-cyclohexyl}-benzamide;
5-Methoxy-benzofuran-2-carboxylic acid {1-[6-oxo-4-(pyridin-2-ylmethanesulfonyl)-hexahydro-pyrrolo[3,2-b]pyrrole-1-carbonyl]-cyclohexyl}-amide;
Thieno[3,2-b]thiophene-2-carboxylic acid {1-[6-oxo-4-(pyridin-2-ylmethanesulfonyl)-hexahydro-pyrrolo[3,2-b]pyrrole-1-carbonyl]-cyclohexyl}-amide;
3-Methyl-benzofuran-2-carboxylic acid {1-[6-oxo-4-(pyridin-2-ylmethanesulfonyl)-hexahydro-pyrrolo[3,2-b]pyrrole-1-carbonyl]-cyclohexyl}-amide;
N-{1-[6-oxo-4-(pyridin-2-ylmethanesulfonyl)-hexahydro-pyrrolo[3,2-b]pyrrole-1-carbonyl]-cyclohexyl}-4-phenoxy-benzamide;
N-{1-[6-oxo-4-(pyridin-2-ylmeoanesulfonyl)-hexahydro-pyrrolo[3,2-b]pyrrole-1-carbonyl]-cyclohexyl}-4-thiophen-2-yl-benzamide;
4-tert-Butyl-N-{1-cyclopropylmethyl-2-oxo-2-[6-oxo-4-(pyridin-2-ylmethanesulfonyl)-hexahydro-pyrrolo[3,2-b]pyrrol -1-yl]-ethyl}-benzamide;
5-Methoxy-benzofuran-2-carboxylic acid {1-cyclopropyhnethyl-2-oxo-2-[6-oxo-4-(pyridin-2-ylmethanesulfonyl)-hexahydro-pyrrolo[3,2-b]pyrrol-1-yl]-ethyl}-amide;
Thieno[3,2-b]thiophene-2-carboxylic acid {1-cyclopropylmethyl-2-oxo-2-[6-oxo-4-(pyridin-2-ylmethanesulfonyl)-hexahydro-pyrrolo[3,2-b]pyrrol-1-yl]-ethyl}-amide;
3-Methyl-benzofuran-2-carboxylic acid {1-cyclopropylmethyl-2-oxo-2-[6-oxo-4-(pyridin-2-ylmemanesulfonyl)-hexahydro-pyrrolo[3,2-b]pyrrol-1-yl]-ethyl}-amide;
N-{1-cyclopropylmethyl-2-oxo-2-[6-oxo-4-(pyridin-2-ylmethanesulfonyl)-hexahydro-pyrrolo[3,2-b]pyrrol-1-yl]-ethyl} -4-phenoxy-benzamide;
N-{1-cyclopropylmethyl-2-oxo-2-[6-oxo-4-(pyridin-2-ylmethanesulfonyl)-hexahydro-pyrrolo[3,2-b]pyrrol-1-yl]-ethyl} -4-thiophen-2-yl-benzamide;
4-tert-Butyl-N-{2-oxo-2-[6-oxo-4-(pyridin-2-ylmethanesulfonyl)-hexahydro-pyrrolo[3,2-b]pyrrol-1-yl]-1-thiophen-2-ylmethyl-ethyl}-benzamide;
5-Methoxy-benzofuran-2-carboxylic acid {2-oxo-2-[6-oxo-4-(pyridin-2-ylmethanesulfonyl)-hexahydro-pyrrolo[3,2-b]pyrrol-1-yl]-1-thiophen-2-ylmethyl-ethyl}-amide;
Thieno[3,2-b]thiophene-2-carboxylic acid {2-oxo-2-[6-oxo-4-(pyridin-2-ylmethanesulfonyl)-hexahydro-pyrrolo[3,2-b]pyrrol-1-yl]-1-thiophen-2-ylmethyl-ethyl}-amide;
3-Methyl-benzofuran-2-carboxylic acid {2-oxo-2-[6-oxo-4-(pyridin-2-ylmethanesulfonyl)-hexahydro-pyrrolo[3,2-b]pyrrol-1-yl]-1-thiophen-2-ylmethyl-ethyl}-amide;
N-{2-oxo-2-[6-oxo-4-(pyridin-2-ylmethanesulfonyl)-hexahydm-pyrrolo[3,2-b]pyrrol-1-yl]-1-thiophen-2-ylmethyl-ethyl}-4-phenoxy-benzamide;
N-{2-oxo-2-[6-oxo-4-(pyridin-2-ylmethanesulfonyl)-hexahydro-pyrrolo[3,2-b]pyrrol-1-yl]-1-thiophen-2-ylmethyl-ethyl}-4-thiophen-2-yl-benzamide;
4-tert-Butyl-N-{1-[6-oxo-4-(pyridin-3-ylmethanesulfonyl)-hexahydro-pyrrolo[3,2-b]pyrrole-1-carbonyl]-cyclohexyl}-benzamide;
5-Methoxy-benzofuran-2-carboxylic acid {1-[6-oxo-4-(pyridin-3-ylmethanesulfonyl)-hexahydro-pyrrolo[3,2-b]pyrrole-1-carbonyl]-cyclohexyl}-amide;
Thieno[3,2-b]thiophene-2-carboxylic acid {1-[6-oxo-4-(pyridin-3-ylmethanesulfonyl)-hexahydro-pyrrolo[3,2-b]pyrrole-1-carbonyl]-cyclohexyl}-amide;
3-Methyl-benzofuran-2-carboxylic acid {1-[6-oxo-4-(pyridin-3-ylmethanesulfonyl)-hexahydro-pyrrolo[3,2-

b]pyrrole-1-carbonyl]-cyclohexyl}-amide;
N-{1-[6-oxo-4-(pyridin-3-ylmethanesulfonyl)-hexahydro-pyrrolo[3,2-b]pyrrole-1-carbonyl]-cyclohexyl}-4-phenoxy-benzamide;
N-{1-[6-oxo-4-(pyridin-3-ylmethanesulfonyl)-hexahydro-pyrrolo[3,2-b]pyrrole-1-carbonyl]-cyclohexyl}-4-thiophen-2-yl-benzamide;
4-tert-Butyl-N-{1-cyclopropylmethyl-2-oxo-2-[6-oxo-4-(pyridin-3-ylmethanesulfonyl)-hexahydro-pyrrolo[3,2-b]pyrrol-1-yl]-ethyl}-benzamide;
5-Methoxy-benzofuran-2-carboxylic acid {1-cyclopropylmethyl-2-oxo-2-[6-oxo-4-(pyridin-3-ylmethanesulfonyl)-hexahydro-pyrrolo[3,2-b]pyrrol-1-yl]-ethyl}-amide;
Thieno[3,2-b]thiophene-2-carboxylic acid {1-cyclopropylmethyl-2-oxo-2-[6-oxo-4-(pyridin-3-ylmethanesulfonyl)-hexahydro-pyrrolo[3,2-b]pyrrol-1-yl]-ethyl}-amide;
3-Methyl-benzofuran-2-carboxylic acid {1-cyclopropylmethyl-2-oxo-2-[6-oxo-4-(pyridin-3-ylmethanesulfonyl)-hexahydro-pyrrolo[3,2-b]pyrrol-1-yl]-ethyl}-amide;
N-{1-cyclopropylmethyl-2-oxo-2-[6-oxo-4-(pyridin-3-ylmethanesulfonyl)-hexahydro-pyrrolo[3,2-b]pyrrol-1-yl]-ethyl}-4-phenoxy-benzamide;
N-{1-cyclopropylmethyl-2-oxo-2-[6-oxo-4-(pyridin-3-ylmethanesulfonyl)-hexahydro-pyrrolo[3,2-b]pyrrol-1-yl]-ethyl}-4-thiophen-2-yl-benzamide;
4-tert-Butyl-N-{2-oxo-2-[6-oxo-4-(pyridin-3-ylmethanesulfonyl)-hexahydro-pymolo[3,2-b]pyrrol-1-yl]-1-thiophen-2-ylmethyl-ethyl}-benzamide;
5-Methoxy-benzofuran-2-carboxylic acid {2-oxo-2-[6-oxo-4-(pyridin-3-ylmethanesulfonyl)-hexahydro-pyrrolo[3,2-b]pyrrol-1-yl]-1-thiophen-2-ylmethyl-ethyl}-amide;
Thieno[3,2-b]thiophene-2-carboxylic acid {2-oxo-2-[6-oxo-4-(pyridin-3-ylmethanesulfonyl)-hexahydro-pyrrolo[3,2-b]pyrrol-1-yl]-1-thiophen-2-ylmethyl-ethyl}-amide;
3-Methyl-benzofiuan-2-carboxylic acid {2-oxo-2-[6-oxo-4-(pyridin-3-ylmethanesulfonyl)-hexahydro-pyrrolo[3,2-b]pyrrol-1-yl]-1-thiophen-2-ylmethyl-ethyl}-amide;
N-{2-oxo-2-[6-oxo-4-(pyridin-3-ylmethanesulfonyl)-hexahydro-pyrrolo[3,2-b]pyrrol-1-yl]-1-thiophen-2-ylmethyl-ethyl}-4-phenoxy-benzamide;
N-12-oxo-2-[6-oxo-4-(pyridin-3-ylmethanesulfonyl)-hexahydro-pyrrolb[3,2-b]pyrrol-1-yl]-1-thiophen-2-ylmethyl-ethyl}-4-thiophen-2-yl-benzamide;
4-tert-Butyl-N-{1-[6-oxo-4-(1-oxy-pyridin-2-ylmetbanesulfonyl)-hexahydro-pyrrolo[3,2-b]pyrrole-1-carbonyl]-cyclohexyl}-benzamide;
5-Methoxy-benzofuran-2-carboxylic acid {1-[6-oxo-4-(1-oxy-pyridin-2-ylmethanesulfonyl)-hexahydro-pyrrolo[3,2-b]pyrrole-1-carbonyl]-cyclohexyl}-amide;
Thieno[3,2-b]thiophene-2-carboxylic acid {1-[6-oxo-4-(1-oxy-pyridin-2-ylmethanesulfonyl)-hexahydro-pyrrolo[3,2-b]pyrrole-1-carbonyl]-cyclohexyl}-amide;
3-Methyl-benzofuran-2-carboxylic acid {1-[6-oxo-4-(1-oxy-pyridin-2-ylmethanesulfonyl)-hexahydro-pyrrolo[3,2-b]pyrrole-1-carbonyl]-cyclohexyl}-amide;
N-{1-[6-oxo-4-(1-oxy-pyridin-2-ylmethanesulfonyl)-hexahydro-pyrrolo[3,2-b]pyrrole-1-carbonyl]-cyclohexyl}-4-phenoxy-benzamide;
N-{1-[6-oxo-4-(-oxy-pyridin-2-ylmethanesulfonyl)-hexahydro-pyrrolo[3,2-b]pyrrole-1-carbonyl]-cyclohexyl}-4-thiophen-2-yl-benzamide;
4-tert-Butyl-N-{1-cyclopropylmethyl-2-oxo-2-[6-oxo-4-(1-oxy-pyridin-2-ylmethanesulfonyl)-hexahydro-pyrrolo[3,2-b]pyrrol-1-yl]-ethyl}-benzamide;
5-Methoxy-benzofuran-2-carboxylic acid {1-cyclopropylmethyl-2-oxo-2-[6-oxo-4-(1-oxy-pyridin-2-ylmethanesulfonyl)-hexahydro-pyrrolo[3,2-b]pyrrol-1-yl]-ethyl}-amide;
Thieno[3,2-b]thiophene-2-carboxylic acid {1-cyclopropylmethyl-2-oxo-2-[6-oxo-4-(1-oxy-pyridin-2-ylmethanesulfonyl)-hexahydro-pyrrolo[3,2-b]pyrrol-1-yl]-ethyl}-amide;
3-Methyl-benzofuran-2-carboxylic acid {1-cyclopmpylmethyl-2-oxo-2-[6-oxo-4-(1-oxy-pyridin-2-ytmethanesulfonyl)-hexahydro-pyrrolo[3,2-b]pyrrol-1-yl]-ethyl}-amide;
N-{1-cyclopropylmethyl-2-oxo-2-[6-oxo-4-(1-oxy-pyridin-2-ylmethanesulfonyl)-hexahydro-pyrrolo[3,2-b]pyrrol-1-yl]-ethyl}-4-phenoxy-benzamide;
N-{1-cyclopropylmethyl-2-oxo-2-[6-oxo-4-(1-oxy-pyridin-2-ylmethanesulfonyl)-hexahydro-pyrrolo[3,2-b]pyrrol-1-yl]-ethyl}-4-thiophen-2-yl-benzamide;
4-tert-Butyl-N-{2-oxo-2-[6-oxo-4-(1-oxy-pyridin-2-ylmethanesulfonyl)-hexahydro-pyrrolo[3,2-b]pyrrol-1-yl]-1-thiophen-2-ylmethyl-ethyl}-benzamide;
5-Methoxy-benzofuran-2-carboxylic acid {2-oxo-2-[6-oxo-4-(1-oxy-pyridin-2-ylmethanesulfonyl)-hexahydro-pyrrolo[3,2-b]pyrrol-1-yl]-1-thiophen-2-ylmethyl-ethyl}-amide;
Thieno[3,2-b]thiophene-2-carboxylic acid {2-oxo-2-[6-oxo-4-(1-oxy-pyridin-2-ylmethanesulfonyl)-hexahydro-pyrrolo[3,2-b]pyrrol-1-yl]-1-thiophen-2-ylmethyl-ethyl}-amide;
3-Methyl-benzofuran-2-carboxylic acid {2-oxo-2-[6-oxo-4-(1-oxy-pyridin-2-ylmethanesulfonyl)-hexahydro-pyrrolo[3,2-b]pyrrol-1-yl]-1-thiophen-2-ylmethyl-ethyl}-amide;
N-{2-oxo-2-[6-oxo-4-(1-oxy-pyridin-2-ylmethanesulfonyl)-hexahydro-pyrrolo[3,2-b]pyrrol-1-yl]-1-thiophen-2-ylmethyl-ethyl}-4-phenoxy-benzamide;
N-{2-oxo-2-[6-oxo-4-(1-oxy-pyridin-2-ylmethanesulfonyl)-hexahydro-pyrrolo[3,2-b]pyrrol-1-yl]-1-thiophen-2-ylmethyl-ethyl}-4-thiophen-2-yl-benzamide;
4-tert-Butyl-N-{1-[6-oxo-4-(1-oxy-pyridin-3-ylmethanesulfonyl)-hexahydro-pyrrolo[3,2-b]pyrrole-1-carbonyl]-cyclohexyl}-benzamide;
5-Methoxy-benzofuran-2-carboxylic acid {1-[6-oxo-4-(1-oxy-pyridin-3-ylmethanesulfonyl)-hexahydro-pyrrolo[3,2-b]pyrrole-1-carbonyl]-cyclohexyl}-amide;
Thieno[3,2-b]thiophene-2-carboxylic acid {1-[6-oxo-4-(1-oxy-pyridin-3-ylmethanesulfonyl)-hexahydro-pyrrolo[3,2-b]pyrrole-1-carbonyl]-cyclohexyl}-amide;
3-Methyl-benzofuran-2-carboxylic acid {1-[6-oxo-4-(1-oxy-pyridin-3-ylmethanesulfonyl)-hexahydro-pyrrolo[3,2-b]pyrrole-1-carbonyl]-cyclohexyl}-amide;
N-{1-[6-oxo-4-(1-oxy-pyridin-3-ylmethanesulfonyl)-hexahydro-pyrrolo[3,2-b]pyrrole-1-carbonyl]-cyclohexyl}-4-phenoxy-benzamide;
N-{1-[6-oxo-4-(1-oxy-pyridin-3-ylmethanesulfonyl)-hexahydro-pyrrolo[3,2-b]pyrrole-1-carbonyl]-cyclohexyl}-4-thiophen-2-yl-benzamide;
4-tert-Butyl-N-{1-cyclopropylmethyl-2-oxo-2-[6-oxo-4-(1-oxy-pyridin-3-ylmethanesulfonyl)-hexahydro-pyrrolo[3,2-b]pyrrol-1-yl]-ethyl}-benzamide;
5-Methoxy-benzofuran-2-carboxylic acid {1-cyclopropylmethyl-2-oxo-2-[6-oxo-4-(1-oxy-pyridin-3-ylmethanesulfonyl)-hexahydro-pyrrolo[3,2-b]pyrrol-1-yl]-ethyl}-amide;
Thieno[3,2-b]thiophene-2-carboxylic acid {1-cyclopropylmethyl-2-oxo-2-[6-oxo-4-(1-oxy-pyridin-3-ylmethanesulfonyl)-hexahydro-pyrrolo[3,2-b]pyrrol-1-yl]-ethyl}-amide;
3-Methyl-benzofuran-2-carboxylic acid {1-cyclopropylmethyl-2-oxo-2-[6-oxo-4-(1-oxy-pyridin-3-ylmethanesulfonyl)-hexahydro-pyrrolo[3,2-b]pyrrol-1-yl]-ethyl}-amide;
N-{1-cyclopropylmethyl-2-oxo-2-[6-oxo-4-(1-oxy-pyridin-3-ylmethanesulfonyl)-hexahydro-pyrrolo[3,2-b]pyrrol-1-yl]-ethyl}-4-phenoxy-benzamide;
N-{1-cyclopropylmethyl-2-oxo-2-[6-oxo-4-(1-oxy-pyridin-3-ylmethanesulfonyl)-hexahydro-pyrrolo[3,2-b]pyrrol-1-yl]-ethyl}-4-thiophen-2-yl-benzamide;
4-tert-Butyl-N-{2-oxo-2-[6-oxo-4-(1-oxy-pyridin-3-ylmethanesulfonyl)-hexahydro-pyrrolo[3,2-b]pyrrol-1-yl]-1-thiophen-2-ylmethyl-ethyl}-benzamide;
5-Methoxy-benzofuran-2-carboxylic acid {2-oxo-2-[6-oxo-4-(1-oxy-pyridin-3-ylmethanesulfonyl)-hexahydro-pyrrolo[3,2-b]pyrrol-1-yl]-1-thiophen-2-ylmethyl-ethyl}-amide;
Thieno[3,2-b]thiophene-2-carboxylic acid {2-oxo-2-[6-oxo-4-(1-oxy-pyridin-3-ylmethanesulfonyl)-hexahydro-pyrrolo[3,2-b]pyrrrol-1-yl]-1-thiophen-2-ylmethyl-ethyl}-amide;
3-Methyl-benzofuran-2-carboxylic acid {2-oxo-2-[6-oxo-4-(1-oxy-pyridin-3-ylmethanesulfonyl)-hexahydro-pyrrolo[3,2-b]pyrrol-1-yl]-1-thiophen-2-ylmethyl-ethyl}-amide;
N-{2-oxo-2-[6-oxo-4-(1-oxy-pyridin-3-ylmethanesulfonyl)-hexahydro-pyrrolo[3,2-b]pyrrol-1-yl]-1-thiophen-2-ylmethyl-ethyl}-4-phenoxy-benzamide;
N-{2-oxo-2-[6-oxo-4-(1-oxy-pyridin-3-ylmethanesulfonyl)-hexahydro-pyrrolo[3,2-b]pyrrol-1-yl]-1-thiophen-2-ylmethyl-ethyl}-4-thiophen-2-yl-benzamide;
2-Isobutyl-4-morpholin-4-yl-1-[6-oxo-4-(pyridine-2-sulfonyl)-hexahydro-pyrrolo[3,2-b]pyrrol-1-yl]-butane-1,4-dione;
2-Isobutyl-1-[6-oxo-4-(pyridine-2-sulfonyl)-hexahydro-pyrrolo[3,2-b]pyrrol-1-yl]-4-piperazin-1-yl-butane-1,4-dione;
2-Isobutyl-4-(4-methyl-piperazin-1-yl)-1-[6-oxo-4-(pyridine-2-sulfonyl)-hexahydro-pyrrolo[3,2-b]pyrrol-1-yl]-butane-1,4-dione;
2-Isobutyl-1-[6-oxo-4-(pyridine-2-sulfonyl)-hexahydro-pyrrolo[3,2-b]pyrrol-1-yl]-4-(4-phenyl-piperazin-1-yl)-butane-1,4-dione;
2-Isobutyl-1-[6-oxo-4-(pyridine-2-sulfonyl)-hexahydro-pyrrolo[3,2-b]pyrrol-1-yl]-4-(3,4,4a,8a-tetrahydro-1H-isoquinolin-2-yl)-butane-1,4-dione;
2-Isobutyl-1-[6-oxo-4-(pyridine-2-sulfonyl)-hexahydro-pyrrolo[3,2-b]pyrrol-1-yl]-4-(1,3,3a,7a-tetrahydro-isoindol-2-yl)-butane-1,4-dione;
4-(4-Benzyl-piperazin-1-yl)-2-isobutyl-1-[6-oxo-4-(pyridine-2-sulfonyl)-hexahydro-pyrrolo[3,2-b]pyrrol-1-yl]-butane-1,4-dione;
2-Isobutyl-4-morpholin-4-yl-1-[6-oxo-4-(pyridine-3-sulfonyl)-hexahydro-pyrmlo[3,2-b]pyrrol-1-yl]-butane-1,4-dione;
2-Isobutyl-1-[6-oxo-4-(pyridine-3-sulfonyl)-hexahydro-pyrrolo[3,2-b]pyrrol-1-yl]-4-piperazin-1-yl-butane-1,4-dione;
2-Isobutyl-4-(4-methyl-piperazin-1-yl)-1-[6-oxo-4-(pyridine-3-sulfonyl)-hexahydro-pyrrolo[3,2-b]pyrrol-1-yl]-butane-1,4-dione;
2-Isobutyl-1-[6-oxo-4-(pyridine-3-sulfonyl)-hexahydro-pyrrolo[3,2-b]pyrrol-1-yl]-4-(4-phenyl-piperazin-1-yl)-butane-1,4-dione;
2-Isobutyl-1-[6-oxo-4-(pyridine-3-sulfonyl)-hexahydro-pyrrolo[3,2-b]pyrrol-1-yl]-4-(3,4,4a,8a-tetrahydro-1H-isoquinolin-2-yl)-butane-1,4-dione;
2-Isobutyl-1-[6-oxo-4-(pyridine-3-sulfonyl)-hexahydro-pyrrolo[3,2-b]pyrrol-1-yl]-4-(1,3,3a,7a-tetrahydro-isoindol-2-yl)-butane-1,4-dione;
4-(4-Benzyl-piperazin-1-yl)-2-isobutyl-1-[6-oxo-4-(pyridine-3-sulfonyl)-hexahydro-pyrrolo[3,2-b]pyrrol-1-yl]-butane-1,4-dione;
2-Isobutyl-4-morpholin-4-yl-1-[6-oxo-4-(1-oxy-pyridine-2-sulfonyl)-hexahydro-pyrrolo[3,2-b]pyrrol-1-yl]-butane-1,4-dione;
2-Isobutyl-1-[6-oxo4-(1-oxy-pyridine-2-sulfonyl)-hexahydro-pyrrolo[3,2-b]pyrrol-1-yl]-4-piperazin-1-yl-butane-1,4-dione;
2-Isobutyl-4-(4-methyl-piperain-1-yl)-1-[6-oxo-4-(1-oxy-pyridine-2-sulfonyl)-hexahydro-pyrrolo[3,2-b]pyrrol-1-yl]-butane-1,4-dione;
2-Isobutyl-1-[6-oxo-4-(1-oxy-pyridine-2-sulfonyl)-hexahydro-pyrrolo[3,2-b]pyrrol-1-yl]-4-(4-phenyl-piperazin-1-yl)-butane-1,4-dione;
2-Isobutyl-1-[6-oxo-4-(1-oxy-pyridine-2-sulfonyl)-hexahydro-pyrrolo[3,2-b]pyrrol-1-yl]-4-(3,4,4a,8a-tetrahydro-1H-isoquinolin-2-yl)-butane-1,4-dione;
2-Isobutyl-1-[6-oxo-4-(1-oxy-pyridine-2-sulfonyl)-hexahydro-pyrrolo[3,2-b]pyrrol-1-yl]-4-(1,3,3a,7a-tetrahydro-isoindol-2-yl)-butane-1,4-dione;
4-(4-Benzyl-piperazin-1-yl)-2-isobutyl-1-[6-oxo-4-(1-oxy-pyridine-2-sulfonyl)-hexahydro-pyrrolo[3,2-b]pyrrol-1-yl]-butane-1,4-dione;
2-Isobutyl-4-morpholin-4-yl-1-[6-oxo-4-(1-oxy-pyridine-3-sulfonyl)-hexahydro-pyrrolo[3,2-b]pyrrol-1-yl]-butane-1,4-dione;
2-Isobutyl-1-[6-oxo-4-(1-oxy-pyridine-3-sulfonyl)-hexahydro-pyrrolo[3,2-b]pyrrol-1-yl]-4-piperazin-1-yl-butane-1,4-dione;
2-Isobutyl-4-(4-methyl-piperazin-1-yl)-1-[6-oxo-4-(1-oxy-pyridine-3-sulfonyl)-hexahydro-pyrrolo[3,2-b]pyrrol-1-yl]-butane-1,4-dione;
2-Isobutyl-1-[6-oxo-4-(1-oxy-pyridine-3-sulfonyl)-hexahydro-pyrrolo[3,2-b]pyrrol-1-yl]-4-(4-phenyl-piperazin-1-yl)-butane-1,4-dione;
2-Isobutyl-1-[6-oxo-4-(1-oxy-pyridine-3-sulfonyl)-hexahydro-pyrrolo[3,2-b]pyrrol-1-yl]-4-(3,4,4a,8a-tetrahydro-1H-isoquinolin-2-yl)-butane-1,4-dione;
2-Isobutyl-1-[6-oxo-4-(1-oxy-pyridine-3-sulfonyl)-hexahydro-pyrrolo[3,2-b]pyrrol-1-yl]-4-(1,3,3a,7a-tetrahydro-isoindol-2-yl)-butane-1,4-dione;
4-(4-Benzyl-piperazin-1-yl)-2-isobutyl-1-[6-oxo-4-(1-oxy-pyridine-3-sulfonyl)-hexahydro-pyrrolo[3,2-b]pyrrol-1-yl]-butane-1,4-dione;
2-Isobutyl-4-morpholin-4-yl-1-[6-oxo-4-(pyridine-2-carbonyl)-hexahydro-pyrrolo[3,2-b]pyrrol-1-yl]-butane-1,4-dione;
2-Isobutyl-1-[6-oxo-4-(pyridine-2-carbonyl)-hexahydro-pyrrolo[3,2-b]pyrrol-1-yl]-4-piperazin-1-yl-butane-1,4-dione;
2-Isobutyl-4-(4-methyl-piperazin-1-yl)-1-[6-oxo-4-(pyridine-2-carbonyl)-hexahydro-pymolo[3,2-b]pyrrol-1-yl]-butane-1,4-dione;
2-Isobutyl-1-[6-oxo-4-(pyridine-2-carbonyl)-hexahydro-pyrrolo[3,2-b]pyrrol-1-yl]-4-(4-phenyl-piperazin-1-yl)-butane-1,4-dione;
2-Isobutyl-1-[6-oxo-4-(pyridine-2-carbonyl)-hexahydro-pyrrolo[3,2-b]pyrrol-1-yl]-4-(3,4,4a,8a-tetrahydro-1H-isoquinolin-2-yl)-butane-1,4-dione;
2-Isobutyl-1-[6-oxo-4-(pyridine-2-carbonyl)-hexahydro-pyirolo[3,2-b]pyrrol-1-yl]-4-(1,3,3a,7a-tetrahydro-isoindol-2-yl)-butane-1,4-dione;
4-(4-Benzyl-piperazin-1-yl)-2-isobutyl-1-[6-oxo-4-(pyridine-2-carbonyl)-hexahydro-pyrrolo[3,2-b]pyrrol-1-yl]-butane-1,4-dione;
2-Isobutyl-4-morpholin-4-yl-1-[6-oxo-4-(2-pyridin-3-yl-acetyl)-hexahydro-pyrrolo[3,2-b]pyrrol-1-yl]-butane-1,4-dione;
2-Isobutyl-1-[6-oxo-4-(2-pyridin-3-yl-acetyl)-hexahydro-pyrrolo[3,2-b]pyrrol-1-yl]-4-piperazin-1-yl-butane-1,4-dione;
2-Isobutyl-4-(4-methyl-pipenzin-1-yl)-1-[6-oxo-4-(2-pyridin-3-yl-acetyl)-hexahydro-pyrrolo[3,2-b]pyrrol-1-yl]-butane-1,4-dione;
2-Isobutyl-1-[6-oxo-4-(2-pyridin-3-yl-acetyl)-hexahydro-pyrrolo[3,2-b]pyrrol-1-yl]-4-(4-phenyl-piperazin-1-yl)-butane-1,4-dione;

2-Isobutyl-1-[6-oxo-4-(2-pyridin-3-yl-acetyl)-hexahydro-pyrrolo[3,2-b]pyrrol-1-yl]-4-(3,4,4a,8a-tetrahydro-1H-isoquinolin-2-yl)-butane-1,4-dione;
2-Isobutyl-1-[6-oxo-4-(2-pyridin-3-yl-acetyl)-hexahydro-pyrrolo[3,2-b]pyrrol-1-yl]-4-(1,3,3a,7a-tetrahydro-isoindol-2-yl)-butane-1,4-dione;
4-(4-Benzyl-piperazin-1-yl)-2-isobutyl-1-[6-oxo-4-(2-pyridin-3-yl-acetyl)-hexahydro-pyrrolo[3,2-b]pyrrol-1-yl]-butane-1,4-dione;
2-Isobutyl-4-morpholin-4-yl-1-[6-oxo-4-(pyridin-2-ylmethanesulfonyl)-hexahydro-pyrrolo[3,2-b]pyrrol-1-yl]-butane-1,4-dione;
2-Isobutyl-1-[6-oxo-4-(pyridin-2-ylmethanesulfonyl)-hexahydro-pyrrolo[3,2-b]pyrrol-1-yl]-4-piperazin-1-yl-butane-1,4-dione;
2-Isobutyl-4-(4-methyl-piperazin-1-yl)-1-[6-oxo-4-(pyridin-2-ylmethanesulfonyl)-hexahydro-pyrrolo[3,2-b]pyrrol-1-yl]-butane-1,4-dione;
2-Isobutyl-1-[6-oxo-4-(pyridin-2-ylmethanesulfonyl)-hexahydro-pyrrolo[3,2-b]pyrrol-1-yl]-4-(4-phenyl-piperazin-1-yl)-butane-1,4-dione;
2-Isobutyl-1-[6-oxo-4-(pyridin-2-ylmethanesulfonyl)-hexahydro-pyrrolo[3,2-b]pyrrol-1-yl]-4-(3,4,4a,8a-tetrahydro-1H-isoquinolin-2-yl)-butane-1,4-dione;
2-Isobutyl-1-[6-oxo-4-(pyridin-2-ylmethanesulfonyl)-hexahydro-pyrrolo[3,2-b]pyrrol-1-yl]-4-(1,3,3a,7a-tetrahydro-isoindol-2-yl)-butane-1,4-dione;
4-(4-Benzyl-piperazin-1-yl)-2-isobutyl-1-[6-oxo-4-(pyridin-2-ylmethanesulfonyl)-hexahydro-pyrrolo[3,2-b]pyrrol-1-yl]-butane-1,4-dione;
2-Isobutyl-4-morpholin-4-yl-1-[6-oxo-4-(pyridin-3-ylmethanesulfonyl)-hexahydro-pyrrolo[3,2-b]pyrrol-1-yl]-butane-1,4-dione;
2-Isobutyl-1-[6-oxo-4-(pyridin-3-ylmethanesulfonyl)-hexahydro-pyrrolo[3,2-b]pyrrol-1-yl]-4-piperazin-1-yl-butane-1,4-dione;
2-Isobutyl-4-(4-methyl-piperazin-1-yl)-1-[6-oxo-4-(pyridin-3-ylmethanesulfonyl)-hexahydro-pyrrolo[3,2-b]pyrrol-1-yl]-butane-1,4-dione;
2-Isobutyl-1-[6-oxo-4-(pyridin-3-ylmethanesulfonyl)-hexahydro-pyrrolo[3,2-b]pyrrol-1-yl]-4-(4-phenyl-piperazin-1-yl)-butane-1,4-dione;
2-Isobutyl-1-[6-oxo-4-(pyridin-3-ylmethanesulfonyl)-hexahydro-pyrrolo[3,2-b]pyrrol-1-yl]-4-(3,4,4a,8a-tetrahydro-1H-isoquinolin-2-yl)-butane-1,4-dione;
2-Isobutyl-1-[6-oxo-4-(pyridin-3-ylmethanesulfonyl)-hexahyro-pyrrolo[3,2-b]pyrrol-1-yl]-4-(1,3,3a,7a-tetrahydro-isoindol-2-yl)-butane-1,4-dione;
4-(4-Benzyl-piperazin-1-yl)-2-isobutyl-1-[6-oxo-4-(pyridin-3-ylmethanesulfonyl)-hexahydro-pyrrolo[3,2-b]pyrrol-1-yl]-butane-1,4-dione;
2-Isobutyl-4-morpholin-4-yl-1-[6-oxo-4-(1-oxy-pyridin-2-ylmethanesulfonyl)-hexahydro-pyrrolo[3,2-b]pyrrol-1-yl]-butane-1,4-dione;
2-Isobutyl-1-[6-oxo-4-(1-oxy-pyridin-2-ylmethanesulfonyl)-hexahydro-pyrrolo[3,2-b]pyrrol-1-yl]-4-piperazin-1-yl-butane-1,4-dione;
2-Isobutyl-4-(4-methyl-piperazin-1-yl)-1-[6-oxo-4-(1-oxy-pyridin-2-ylmethanesulfonyl)-hexahydro-pyrrolo[3,2-b]-pyrrol-1-yl]-butane-1,4-dione;
2-Isobutyl-1-[6-oxo-4-(1-oxy-pyridin-2-ylmethanesulfonyl)-hexahydro-pyrrolo[3,2-b]pyrrol-1-yl]-4-(4-phenyl-piperazin-1-yl)-butane-1,4-dione;
2-Isobutyl-1-[6-oxo-4-(1-oxy-pyridin-2-ylmethanesulfonyl)-hexahydro-pyrrolo[3,2-b]pyrrol-1-yl]-4-(3,4,4a,8a-tetrahydro-1H-isoquinolin-2-yl)-butane-1,4-dione;
2-Isobutyl-1-[6-oxo-4-(1-oxy-pyridin-2-ylmethanesulfonyl)-hexahydro-pyrrolo[3,2-b]pyrrol-1-yl]-4-(1,3,3a,7a-tetrahydro-isoindol-2-yl)-butane-1,4-dione;
4-(4-Benzyl-piperazin-1-yl)-2-isobutyl-1-[6-oxo-4-(1-oxy-pyridin-2-ylmethanesulfonyl)-hexahydro-pyrrolo[3,2-b]-pyrrol-1-yl]-butane-1,4-dione;
2-Isobutyl-4-morpholin-4-yl-1-[6-oxo-4-(1-oxy-pyridin-3-ylmethanesulfonyl)-hexahydro-pyrrolo[3,2-b]pyrrol-1-yl]-butane-1,4-dione;
2-Isobutyl-1-[6-oxo-4-(1-oxy-pyridin-3-ylmethanesulfonyl)-hexahydro-pyrrolo[3,2-b]pyrrol-1-yl]-4-piperazin-1-yl-butane-1,4-dione;
2-Isobutyl-4-(4-methyl-piperazin-1-yl)-1-[6-oxo-4-(1-oxy-pyridin-3-ylmethanesulfonyl)-hexahydro-pyrrolo[3,2-b]-pyrrol-1-yl]-butane-1,4-dione;
2-Isobutyl-1-[6-oxo-4-(1-oxy-pyridin-3-ylmethimesulfonyl)-hexahydro-pyrrolo[3,2-b]pyrrol-1-yl]-4-(4-phenyl-piperazin-1-yl)-butane-1,4-dione;
2-Isobutyl-1-[6-oxo-4-(1-oxy-pyridin-3-ylmethanesulfonyl)-hexahydro-pyrrolo[3,2-b]pyrrol-1-yl]-4-(3,4,4a,8a-tetrahydro-1H-isoquinolin-2-yl)-butane-1,4-dione;

2-Isobutyl-1-[6-oxo-4-(1-oxy-pyridin-3-ylmethanesulfonyl)-hexahydro-pyrrolo[3,2-b]pyrrol-1-yl]-4-(1,3,3a,7a-tetrahydro-isoindol-2-yl)-butane-1,4-dione;
4-(4-Benzyl-piperazin-1-yl)-2-isobutyl-1-[6-oxo-4-(1-oxy-pyridin-3-ylmethanesulfonyl)-hexahydro-pyrrolo[3,2-b]-pyrrol-1-yl]-butane-1,4-dione;
4-(2-Biphenyl-3-yl-4-methyl-pentanoyl)-1-(pyridine-2-sulfonyl)-hexahydro-pyrrolo[3,2-b]pyrrol-3-one;
4-(2-Biphenyl-3-yl-4-methyl-pentanoyl)-1-(pyridine-3-sulfonyl)-hexahydro-pyrrolo[3,2-b]pyrrol-3-one;
4-(2-Biphenyl-3-yl-4-methyl-pentanoyl)-1-(1-oxy-pyridine-2-sulfonyl)-hexahydro-pyrrolo[3,2-b]pyrrol-3-one;
4-(2-Biphenyl-3-yl-4-methyl-pentanoyl)-1-(1-oxy-pyridine-3-sulfonyl)-hexahydro-pyrrolo[3,2-b]pyrrol-3-one;
4-(2-Biphenyl-3-yl-4-methyl-pentanoyl)-1-(pyridine-2-carbonyl)-hexahydro-pyrrolo[3,2-b]pyrrol-3-one;
4-(2-Biphenyl-3-yl-4-methyl-pentanoyl)-1-(2-pyridin-3-yl-acetyl)-hexahydro-pyrrolo[3,2-b]pyrrol-3-one;
4-(2-Biphenyl-3-yl-4-methyl-pentanoyl)-1-(pyridin-2-ylmethanesulfonyl)-hexahydro-pyrrolo[3,2-b]pyrrol-3-one;
4-(2-Biphenyl-3-yl-4-methyl-pentanoyl)-1-(pyridin-3-ylmethanesulfonyl)-hexahydro-pyrrolo[3,2-b]pyrrol-3-one;
4-(2-Biphenyl-3-yl-4-methyl-pentanoyl)-1-(1-oxy-pyridin-2-ylmethanesulfonyl)-hexahydro-pyrrolo[3,2-b]pyrrol-3-one;
4-(2-Biphenyl-3-yl-4-methyl-pentanoyl)-1-(1-oxy-pyridin-3-ylmethanesulfonyl)-hexahydro-pyrrolo[3,2-b]pyrrol-3-one;
4-[4-Methyl-2-(3-pyridin-2-yl-phenyl)-pentanoyl]-1-(pyridine-2-sulfonyl)-hexahydro-pyrrolo[3,2-b]pyrrol-3-one;
4-[4-Methyl-2-(3-pyridin-2-yl-phenyl)-pentanoyl]-1-(pyridine-3-sulfonyl)-hexahydro-pyrrolo[3,2-b]pyrrol-3-one;
4-[4-Methyl-2-(3-pyridin-2-yl-phenyl)-pentanoyl]-1-(1-oxy-pyridine-2-sulfonyl)-hexahydro-pyrrolo[3,2-b]pyrrol-3-one;
4-[4-Methyl-2-(3-pyridin-2-yl-phenyl)-pentanoyl]-1-(1-oxy-pyridine-3-sulfonyl)-hexahydro-pyrrolo[3,2-b]pyrrol-3-one;
4-[4-Methyl-2-(3-pyridin-2-yl-phenyl)-pentanoyl]-1-(pyridine-2-carbonyl)-hexahydro-pyrrolo[3,2-b]pyrrol-3-one;
4-[4-Methyl-2-(3-pyridin-2-yl-phenyl)-pentanoyl]-1-(2-pyridin-3-yl-acetyl)-hexahydro-pyrrolo[3,2-b]pyrrol-3-one;
4-[4-Methyl-2-(3-pyridin-2-yl-phenyl)-pentanoyl]-1-(pyridin-2-ylmethanesulfonyl)-hexahydio-pyrrolo[3,2-b]pyrrol-3-one;
4-[4-Methyl-2-(3-pyridin-2-yl-phenyl)-pentanoyl]-1-(pyridin-3-ylmethanesulfonyl)-hexahydro-pyrrolo[3,2-b]pyrrol-3-one;
4-[4-Methyl-2-(3-pyridin-2-yl-phenyl)-pentanoyl]-1-(1-oxy-pyridin-2-ylmethanesulfonyl)-hexahydro-pyrrolo[3,2-b]pyrrol-3-one;
4-[4-Methyl-2-(3-pyridin-2-yl-phenyl)-pentanoyl]-1-(1-oxy-pyridin-3-ylmethanesulfonyl)-hexahydro-pyrrolo[3,2-b]pyrrol-3-one;
4-[4-Methyl-2-(3-pyridin-3-yl-phenyl)-pentanoyl]-1-(pyridine-2-sulfonyl)-hexahydro-pyrrolo[3,2-b]pyrrol-3-one;
4-[4-Methyl-2-(3-pyridin-3-yl-phenyl)-pentanoyl]-1-(pyridine-3-sulfonyl)-hexahydro-pyrrolo[3,2-b]pyrrol-3-one;
4-[4-Methyl-2-(3-pyridin-3-yl-phenyl)-pentanoyl]-1-(1-oxy-pyridine-2-sulfonyl)-hexahydro-pyrrolo[3,2-b]pyrrol-3-one;
4-[4-Methyl-2-(3-pyridin-3-yl-phenyl)-pentanoyl]-1-(1-oxy-pyridine-3-sulfonyl)-hexahydro-pyrrolo[3,2-b]pyrrol-3-one;
4-[4-Methyl-2-(3-pyridin-3-yl-phenyl)-pentanoyl]-1-(pyridine-2-carbonyl)-hexahydro-pyrrlo[3,2-b]pyrrol-3-one;
4-[4-Methyl-2-(3-pyridin-3-yl-phenyl)-pentanoyl]-1-(2-pyridin-3-yl-acetyl)-hexahydro-pyrrolo[3,2-b]pyrrol-3-one;
4-[4-Methyl-2-(3-pyridin-3-yl-phenyl)-pentanoyl]-1-(pyridin-2-ylmethanesulfonyl)-hexahydro-pyrrolo[3,2-b]pyrrol-3-one;
4-[4-Methyl-2-(3-pyridin-3-yl-phenyl)-pentanoyl]-1-(pyridin-3-ylmethanesulfonyl)-hexahydro-pyrrolo[3,2-b]pyrrol-3-one;
4-[4-Methyl-2-(3-pyridin-3-yl-phenyl)-pentanoyl]-1-(1-oxy-pyridin-2-ylmethanesulfonyl)-hexahydro-pyrrolo[3,2-b]pyrrol-3-one;
4-[4-Methyl-2-(3-pyridin-3-yl-phenyl)-pentanoyl]-1-(1-oxy-pyridin-3-ylmethanesulfonyl)-hexahydro-pyrrolo[3,2-b]pyrrol-3-one;
4-[4-Methyl-2-(3-pyridin-4-yl-phenyl)-pentanoyl]-1-(pyridine-2-sulfonyl)-hexahydro-pyrrolo[3,2-b]pyrrol-3-one;
4-[4-Methyl-2-(3-pyridin-4-yl-phenyl)-pentanoyl]-1-(pyridine-3-sulfonyl)-hexahydro-pyrrolo[3,2-b]pyrrol-3-one;
4-[4-Methyl-2-(3-pyridin-4-yl-phenyl)-pentanoyl]-1-(1-oxy-pyridine-2-sulfonyl)-hexahydro-pyrrolo[3,2-b]pyrrol-3-one;
4-[4-Methyl-2-(3-pyridin-4-yl-phenyl)-pentanoyl]-1-(1-oxy-pyridine-3-sulfonyl)-hexahydro-pyrrolo[3,2-b]pyrrol-3-one;
4-[4-Methyl-2-(3-pyridin-4-yl-phenyl)-pentanoyl]-1-(pyridine-2-carbonyl)-hexahydro-pyrrolo[3,2-b]pyrrol-3-one;
4-[4-Methyl-2-(3-pyridin-4-yl-phenyl)-pentanoyl]-1-(2-pyridin-3-yl-acetyl)-hexahydro-pyrrolo[3,2-b]pyrrol-3-one;
4-[4-Methyl-2-(3-pyridin-4-yl-phenyl)-pentanoyl]-1-(pyridin-2-ylmethanesulfonyl)-hexahydro-pyrrolo[3,2-b]pyrrol-3-one;

4-[4-Methyl-2-(3-pyridin-4-yl-phenyl)-pentanoyl]-1-(pyridin-3-ylmethanesulfonyl)-hexahydro-pyrrolo[3,2-b]pyrrol-3-one;
4-[4-Methyl-2-(3-pyridin-4-yl-phenyl)-pentanoyl]-1-(1-oxy-pyridin-2-ylmethanesulfonyl)-hexahydro-pyrrolo[3,2-b]pyrrol-3-one;
4-[4-Methyl-2-(3-pyridin-4-yl-phenyl)-pentanoyl]-1-(1-oxy-pyridin-3-ylmethanesulfonyl)-hexahydro-pyrrolo[3,2-b]pyrrol-3-one;
Furan-3-carboxylic acid {3,3-dimethyl-1-[6-oxo-4-(pyridine-2-sulfonyl)-hexahydro-pyrrolo[3,2-b]pyrrole-1-carbonyl]-butyl}-amide;
Thiophene-3-carboxylic acid {3,3-dimethyl-1-[6-oxo-4-(pyridine-2-sulfonyl)-hexahydro-pyrrolo[3,2-b]pyrrole-1-carbonyl]-butyl}-amide;
Benzo[b]thiophene-3-carboxylic acid {3,3-dimethyl-1-[6-oxo-4-(pyridine-2-sulfonyl)-hexahydro-pyrrolo[3,2-b]pyrrole-1-carbonyl]-butyl}-amide;
Furan-3-carboxylic acid {1-cyclohexylmethyl-2-oxo-2-[6-oxo-4-(pyridine-2-sulfonyl)-hexahydro-pyrrolo[3,2-b]pyrrol-1-yl]-ethyl}-amide;
Thiophene-3-carboxylic acid {1-cyclohexylmethyl-2-oxo-2-[6-oxo-4-(pyridine-2-sulfonyl)-hexahydro-pyrrolo[3,2-b]pyrrol-1-yl]-ethyl}-amide;
Benzo[b]thiophene-3-carboxylic acid {1-cyclohexylmethyl-2-oxo-2-[6-oxo-4-(pyridine-2-sulfonyl)-hexahydro-pyrrolo[3,2-b]pyrrol-1-yl]-ethyl}-amide;
Naphthalene-1-carboxylic acid {1-(4-hydroxy-benzyl)-2-oxo-2-[6-oxo-4-(pyridine-2-sulfonyl)-hexahydro-pyrrolo[3,2-b]pyrrol-1-yl]-ethyl}-amide;
Quinoline-8-carboxylic acid {1-(4-hydroxy-benzyl)-2-oxo-2-[6-oxo-4-(pyridine-2-sulfonyl)-hexahyro-pyrrolo[3,2-b]pyrrol-1-yl]-ethyl}-amide;
4-tert-Butyl-N-{1-(4-hydroxy-benzyl)-2-oxo-2-[6-oxo-4-(pyridine-2-sulfonyl)-hexahydro-pyrrolo[3,2-b]pyrrol-1-yl]-ethyl}-benzamide;
4-tert-Butyl-N-{1-(4-fluoro-benlyl)-2-oxo-2-[6-oxo-4-(pyridine-2-sulfonyl)-hexahydro-pyrrolo[3,2-b]pyrrol-1-yl]-ethyl}-benzamide;
4-tert-Butyl-N-{1-(4-methoxy-benzyl)-2-oxo-2-[6-oxo-4-(pyridine-2-sulfonyl)-hexahydro-pyrrolo[3,2-b]pyrrol-1-yl]-ethyl}-benzamide;
4-tert-Butyl-N-{1-(3-hydroxy-benzyl)-2-oxo-2-[6-oxo-4-(pyridine-2-sulfonyl)-hexahydro-pyrrolo[3,2-b]pyrrol-1-yl]-ethyl}-benzamide;
Biphenyl-4-carboxylic acid {1-(4-hydroxy-benzyl)-2-oxo-2-[6-oxo-4-(pyridine-2-sulfonyl)-hexahydro-pyrrolo[3,2-b]pyrrol-1-yl]-ethyl}-amide;
Biphenyl-4-carboxylic acid {1-(4-fluoro-benzyl)-2-oxo-2-[6-oxo-4-(pyridine-2-sulfonyl)-hexahydro-pyrrolo[3,2-b]pyrrol-1-yl]-ethyl}-amide;
Biphenyl-4-carboxylic acid {1-(4-methoxy-benzyl)-2-oxo-2-[6-oxo-4-(pyridine-2-sulfonyl)-hexahydro-pyrrolo[3,2-b]pyrrol-1-yl]-ethyl}-amide;
Biphenyl-4-carboxylic acid {1-(3-hydroxy-benzyl)-2-oxo-2-[6-oxo-4-(pyridine-2-sulfonyl)-hexahydro-pyrrolo[3,2-b]pyrrol-1-yl]-ethyl}-amide;
Furan-3-carboxylic acid {3,3-dimethyl-1-[6-oxo-4-(pyridine-3-sulfonyl)-hexahydro-pyrrolo[3,2-b]pyrrole-1-carbonyl]-butyl}-amide;
Thiophene-3-carboxylic acid {3,3-dimethyl-1-[6-oxo-4-(pyridine-3-sulfonyl)-hexahydro-pyrrolo[3,2-b]pyrrole-1-carbonyl]-butyl}-amide;
Benzo[b]thiophene-3-carboxylic acid {3,3-dimethyl-1-[6-oxo-4-(pyridine-3-sulfonyl)-hexahydro-pyrrolo[3,2-b]pyrrole-1-carbonyl]-butyl}-amide;
Furan-3-carboxylic acid {1-cyclohexylmethyl-2-oxo-2-[6-oxo-4-(pyridine-3-sulfonyl)-hexahydro-pyrrolo[3,2-b]pyrrol-1-yl]-ethyl}-amide;
Thiophene-3-carboxylic acid {1-cyclohexylmethyl-2-oxo-2-[6-oxo-4-(pyridine-3-sulfonyl)-hexahydro-pyrrolo[3,2-b]pyrrol-1-yl]-ethyl}-amide;
Benzo[b]thiophene-3-carboxylic acid {1-cyclohexylmethyl-2-oxo-2-[6-oxo-4-(pyridine-3-sulfonyl)-hexahydro-pyrrolo[3,2-b]pyrrol-1-yl]-ethyl}-amide;
Naphthalene-1-carboxylic acid {1-(4-hydroxy-benzyl)-2-oxo-2-[6-oxo-4-(pyridine-3-sulfonyl)-hexahydro-pyrrolo[3,2-b]pyrrol-1-yl]-ethyl}-amide;
Quinoline-8-carboxylic acid {1-(4-hydroxy-benzyl)-2-oxo-2-[6-oxo-4-(pyridine-3-sulfonyl)-hexahydro-pyrrolo[3,2-b]pyrrol-1-yl]-ethyl}-amide;
4-tert-Butyl-N-{1-(4-hydroxy-benzyl)-2-oxo-2-[6-oxo-4-(pyridine-3-sulfonyl)-hexahydro-pyrrolo[3,2-b]pyrrol-1-yl]-ethyl}-benzamide;
4-tert-Butyl-N-{1-(4-fluoro-benzyl)-2-oxo-2-[6-oxo-4-(pyridine-3-sulfonyl)-hexahydro-pyrrolo[3,2-b]pyrrol-1-yl]-ethyl}-benzamide;

4-tert-Butyl-N-{1-(4-methoxy-benzyl)-2-oxo-2-[6-oxo-4-(pyridine-3-sulfonyl)-hexahydro-pyrrolo[3,2-b]pyrrol-1-yl]-ethyl}-benzamide;
4-tert-Butyl-N-{1-(3-hydroxy-benzyl)-2-oxo-2-[6-oxo-4-(pyridine-3-sulfonyl)-hexahydro-pyrrolo[3,2-b]pyrrol-1-yl]-ethyl}-benzamide;
Biphenyl-4-carboxylic acid {1-(4-hydroxy-benzyl)-2-oxo-2-[6-oxo-4-(pyridine-3-sulfonyl)-hexahydro-pyrrolo[3,2-b]pyrrol-1-yl]-ethyl}-amide;
Biphenyl-4-carboxylic acid {1-(4-fluoro-benzyl)-2-oxo-2-[6-oxo-4-(pyridine-3-sulfonyl)-hexahydro-pyrrolo[3,2-b]pyrrol-1-yl]-ethyl}-amide;
Biphenyl-4-carboxylic acid {1-(4-methoxy-benzyl)-2-oxo-2-[6-oxo-4-(pyridine-3-sulfonyl)-hexahydro-pyrrolo[3,2-b]pyrrol-1-yl]-ethyl}-amide;
Biphenyl-4-carboxylic acid {1-(3-hydroxy-benzyl)-2-oxo-2-[6-oxo-4-(pyridine-3-sulfonyl)-hexahydro-pyrrolo[3,2-b]pyrrol-1-yl]-ethyl}-amide;
Furan-3-carboxylic acid {3,3-dimethyl-1-[6-oxo-4-(1-oxy-pyridine-2-sulfonyl)-hexahydro-pyrrolo[3,2-b]pyrrole-1-carbonyl]-butyl}-amide;
Thiophene-3-carboxylic acid {3,3-dimethyl-1-[6-oxo-4-(1-oxy-pyridine-2-sulfonyl)-hexahydro-pyrrolo[3,2-b]pyrrole-1-carbonyl]-butyl}-amide;
Benzo[b]thiophene-3-carboxylic acid {3,3-dimethyl-1-[6-oxo-4-(1-oxy-pyridine-2-sulfonyl)-hexahydro-pyrrolo[3,2-b]pyrrole-1-carbonyl]-butyl}-amide;
Furan-3-carboxylic acid {1-cyclohexylmethyl-2-oxo-2-[6-oxo-4-(1-oxy-pyridine-2-sulfonyl)-hexahydro-pyrrolo[3,2-b]pyrrol-1-yl]-ethyl}-amide;
Thiophene-3-carboxylic acid {1-cyclohexylmethyl-2-oxo-2-[6-oxo-4-(1-oxy-pyridine-2-sulfonyl)-hexahydro-pyrrolo[3,2-b]pyrrol-1-yl]-ethyl}-amide;
Benzo[b]thiophene-3-carboxylic acid {1-cyclohexylmethyl-2-oxo-2-[6-oxo-4-(1-oxy-pyridine-2-sulfonyl)-hexahydro-pyrrolo[3,2-b]pyrrol-1-yl]-ethyl}-amide;
Naphthalene-1-carboxylic acid {1-(4-hydroxy-benzyl)-2-oxo-2-[6-oxo-4-(1-oxy-pyridine-2-sulfonyl)-hexahydro-pyrrolo[3,2-b]pyrrol-1-yl]-ethyl}-amide;
Quinoline-8-carboxylic acid {1-(4-hydroxy-benzyl)-2-oxo-2-[6-oxo-4-(1-oxy-pyridine-2-sulfonyl)-hexahydro-pyrrolo[3,2-b]pyrrol-1-yl]-ethyl}-amide;
4-tert-Butyl-N-{1-(4-hydroxy-benzyl)-2-oxo-2-[6-oxo-4-(1-oxy-pyridine-2-sulfonyl)-hexahydro-pyrrolo[3,2-b]pyrrol-1-yl]-ethyl}-benzamide;
4-tert-Butyl-N-{1-(4-fluoro-benzyl)-2-oxo-2-[6-oxo-4-(1-oxy-pyridine-2-sulfonyl)-hexahydro-pyrrolo[3,2-b]pyrrol-1-yl]-ethyl}-benzamide;
4-tert-Butyl-N-{1-(4-methoxy-benzyl)-2-oxo-2-[6-oxo-4-(1-oxy-pyridine-2-sulfonyl)-hexahydro-pyrrolo[3,2-b]pyrrol-1-yl]-ethyl}-benzamide;
4-tert-Butyl-N-{1-(3-hydroxy-benzyl)-2-oxo-2-[6-oxo-4-(1-oxy-pyridine-2-sulfonyl)-hexahydro-pyrrolo[3,2-b]pyrrol-1-yl]-ethyl}-benzamide;
Biphenyl-4-carboxylic acid {1-(4-hydroxy-benzyl)-2-oxo-2-[6-oxo-4-(1-oxy-pyridine-2-sulfonyl)-hexahydro-pyrrolo[3,2-b]pyrrol-1-yl]-ethyl}-amide;
Biphenyl-4-carboxylic acid {1-(4-fluoro-benzyl)-2-oxo-2-[6-oxo-4-(1-oxy-pyridine-2-sulfonyl)-hexahydro-pyrrolo[3,2-b]pyrrol-1-yl]-ethyl}-amide;
Biphenyl-4-carboxylic acid {1-(4-methoxy-benzyl)-2-oxo-2-[6-oxo-4-(1-oxy-pyridine-2-sulfonyl)-hexahydro-pyrrolo[3,2-b]pyrrol-1-yl]-ethyl}-amide;
Biphenyl-4-carboxylic acid {1-(3-hydroxy-benzyl)-2-oxo-2-[6-oxo-4-(1-oxy-pyridine-2-sulfonyl)-hexahydro-pyrrolo[3,2-b]pyrrol-1-yl]-ethyl}-amide;
Furan-3-carboxylic acid {3,3-dimethyl-1-[6-oxo-4-(1-oxy-pyridine-3-sulfonyl)-hexahydro-pyrrolo[3,2-b]pyrrole-1-carbonyl]-butyl}-amide;
Thiophene-3-carboxylic acid {3,3-dimethyl-1-[6-oxo-4-(1-oxy-pyridine-3-sulfonyl)-hexahydro-pyrrolo[3,2-b]pyrrole-1-carbonyl]-butyl}-amide;
Benzo[b]thiophene-3-carboxylic acid {3,3-dimethyl-1-[6-oxo-4-(1-oxy-pyridine-3-sulfonyl)-hexahydro-pyrrolo[3,2-b]pyrrole-1-carbonyl]-butyl}-amide;
Furan-3-carboxylic acid {1-cyclohexylmethyl-2-oxo-2-[6-oxo-4-(1-oxy-pyridine-3-sulfonyl)-hexahydro-pyrrolo[3,2-b]pyrrol-1-yl]-ethyl}-amide;
Thiophene-3-carboxylic acid {1-cyclohexylmethyl-2-oxo-2-[6-oxo-4-(1-oxy-pyridine-3-sulfonyl)-hexahydro-pyrrolo[3,2-b]pyrrol-1-yl]-ethyl}-amide;
Benzo[b]thiophene-3-carboxylic acid {1-cyclohexylmethyl-2-oxo-2-[6-oxo-4-(1-oxy-pyridine-3-sulfonyl)-hexahydro-pyrrolo[3,2-b]pyrrol-1-yl]-ethyl}-amide;
Naphthalene-1-carboxylic acid {1-(4-hydroxy-benzyl)-2-oxo-2-[6-oxo-4-(1-oxy-pyridine-3-sulfonyl)-hexahydro-pyrrolo[3,2-b]pyrrol-1-yl]-ethyl}-amide;

Quinoline-8-carboxylic acid {1-(4-hydroxy-benzyl)-2-oxo-2-[6-oxo-4-(1-oxy-pyridine-3-sulfonyl)-hexahydro-pyrrolo[3,2-b]pyrrol-1-yl]-ethyl}-amide;
4-tert-Butyl-N-{1-(4-hydroxy-benzyl)-2-oxo-2-[6-oxo-4-(1-oxy-pyridine-3-sulfonyl)-hexahydro-pyrrolo[3,2-b]pyrrol-μ1-yl]-ethyl}-benzamide;
4-tert-Butyl-N-{1-(4-fluoro-benzyl)-2-oxo-2-[6-oxo-4-(1-oxy-pyridine-3-sulfonyl)-hexahydro-pyrrolo[3,2-b]pyrrol-1-yl]-ethyl}-benzamide;
4-tert-Butyl-N-{1-(4-methoxy-benzyl)-2-oxo-2-[6-oxo-4-(1-oxy-pyridine-3-sulfonyl)-hexahydro-pyrrolo[3,2-b]pyrrol-1-yl]-ethyl}-benzamide;
4-tert-Butyl-N-{1-(3-hydroxy-benzyl)-2-oxo-2-[6-oxo-4-(1-oxy-pyridine-3-sulfonyl)-hexahydro-pyrrolo[3,2-b]pyrrol-1-yl]-ethyl}-benzamide;
Biphenyl-4-carboxylic acid {1-(4-hydroxy-benzyl)-2-oxo-2-[6-oxo-4-(1-oxy-pyridine-3-sulfonyl)-hexahydro-pyrrolo[3,2-b]pyrrol-1-yl]-ethyl}-amide;
Biphenyl-4-carboxylic acid {1-(4-fluoro-benzyl)-2-oxo-2-[6-oxo-4-(1-oxy-pyridine-3-sulfonyl)-hexahydro-pyrrolo[3,2-b]pyrrol-1-yl]ethyl}-amide;
Biphenyl-4-carboxylic acid {1-(4-methoxy-benzyl)-2-oxo-2-[6-oxo-4-(1-oxy-pyridine-3-sulfonyl)-hexahydro-pyrrolo[3,2-b]pyrrol-1-yl]-ethyl}-amide;
Biphenyl-4-carboxylic acid {1-(3-hydroxy-benzyl)-2-oxo-2-[6-oxo-4-(1-oxy-pyridine-3-sulfonyl)-hexahydro-pyrrolo[3,2-b]pyrrol-1-yl]-ethyl}-amide;
Furan-3-carboxylic acid {3,3-dimethyl-1-[6-oxo-4-(pyridine-2-carbonyl)-hexahydro-pyrrolo[3,2-b]pyrrole-1-carbonyl]-butyl}-amide;
Thiophene-3-carboxylic acid {3,3-dimethyl-1-[6-oxo-4-(pyridine-2-carbonyl)-hexahydro-pyrrolo[3,2-b]pyrrole-1-carbonyl]-butyl}-amid;
Benzo[b]thiophene-3-carboxylic acid {3,3-dimethyl-1-[6-oxo-4-(pyridine-2-carbonyl)-hexahydro-pyrrolo[3,2-b]pyrrole-1-carbonyl]-butyl}-amide;
Furan-3-carboxylic acid {1-cyclohexylmethyl-2-oxo-2-[6-oxo-4-(pyridine-2-carbonyl)-hexahydro-pyrrolo[3,2-b]pyrrol-1-yl]-ethyl}-amide;
Thiophene-3-carboxylic acid {1-cyclohexylmethyl-2-oxo-2-[6-oxo-4-(pyridine-2-carbonyl)-hexahydro-pyrrolo[3,2-b]pyrrol-1-yl]-ethyl}-amide;
Benzo[b]thiophene-3-carboxylic acid {1-cyclohexylmethyl-2-oxo-2-[6-oxo-4-(pyridine-2-carbonyl)-hexahydro-pyrrolo[3,2-b]pyrrol-1-yl]-ethyl}-amide;
Naphthalene-1-carboxylic acid {1-(4-hydroxy-benzyl)-2-oxo-2-[6-oxo-4-(pyridine-2-carbonyl)-hexahydro-pyrrolo[3,2-b]pyrrol-1-yl]-ethyl}-amide;
Quinoline-8-carboxylic acid {1-(4-hydroxy-benzyl)-2-oxo-2-[6-oxo-4-(pyridine-2-carbonyl)-hexahydro-pyrrolo[3,2-b]pyrrol-1-yl]-ethyl}-amide;
4-tert-Butyl-N-{1-(4-hydroxy-benzyl)-2-oxo-2-[6-oxo-4-(pyridine-2-carbonyl)-hexahydro-pyrrolo[3,2-b]pyrrol-1-yl]-ethyl}-benzamide;
4-tert-Butyl-N-{1-(4-fluoro-benzyl)-2-oxo-2-[6-oxo-4-(pyridine-2-carbonyl)-hexahydro-pyrrolo[3,2-b]pyrrol-1-yl]-ethyl}-benzamide;
4-tert-Butyl-N-{1-(4-methoxy-benzyl)-2-oxo-2-[6-oxo-4-(pyridine-2-carbonyl)-hexahydro-pyrrolo[3,2-b]pyrrol-1-yl]-ethyl}-benzamide;
4-tert-Butyl-N-{1-(3-hydroxy-benzyl)-2-oxo-2-[6-oxo-4-(pyridine-2-carbonyl)-hexahydro-pyrrolo-[3,2-b]pyrrol-1-yl]-ethyl}-benzamide;
Biphenyl-4-carboxylic acid {1-(4-hydroxy-benzyl)-2-oxo-2-[6-oxo-4-(pyridine-2-carbonyl)-hexahydro-pyrrolo[3,2-b]pyrrol-1-yl]-ethyl}-amide;
Biphenyl-4-carboxylic acid {1-(4-fluoro-benzyl)-2-oxo-2-[6-oxo-4-(pyridine-2-carbonyl)-hexahydro-pyrrolo[3,2-b]pyrrol-1-yl]-ethyl}-amide;
Biphenyl-4-carboxylic acid {1-(4-methoxy-benzyl)-2-oxo-2-[6-oxo-4-(pyridine-2-carbonyl)-hexahydro-pyrrolo[3,2-b]pyrrol-1-yl]-ethyl}-amide;
Biphenyl-4-carboxylic acid {1-(3-hydroxy-benzyl)-2-oxo-2-[6-oxo-4-(pyridine-2-carbonyl)-hexahydro-pyrrolo[3,2-b]pyrrol-1-yl]-ethyl}-amide;
Furan-3-carboxylic acid {3,3-dimethyl-1-[6-oxo-4-(2-pyridin-3-yl-acetyl)-hexahydro-pyrrolo[3,2-b]pyrrole-1-carbonyl]-butyl}-amide;
Thiophene-3-carboxylic acid {3,3-dimethyl-1-[6-oxo-4-(2-pyridin-3-yl-acetyl)-hexahydro-pyrrolo[3,2-b]pyrrole-1-carbonyl]-butyl}-amide;
Benzo[b]thiophene-3-carboxylic acid {3,3-dimethyl-1-[6-oxo-4-(2-pyridin-3-yl-acetyl)-hexahydro-pyrrolo[3,2-b]pyrrole-1-carbonyl]-butyl}-amide;
Furan-3-carboxylic acid {1-cyclohexylmethyl-2-oxo-2-[6-oxo-4-(2-pyridin-3-yl-acetyl)-hexahydro-pyrrolo[3,2-b]pyrrol-1-yl]-ethyl}-amide;

Thiophene-3-carboxylic acid {1-cyclohexylmethyl-2-oxo-2-[6-oxo-4-(2-pyridin-3-yl-acetyl)-hexahydro-pyrrolo[3,2-b]pyrrol-1-yl]-ethyl}-amide;
Benzo[b]thiophene-3-carboxylic acid {1-cyclohexylmethyl-2-oxo-2-[6-oxo-4-(2-pyridin-3-yl-acetyl)-hexahydro-pyrrolo[3,2-b]pyrrol-1-yl]-ethyl}-amide;
Naphthalene-1-carboxylic acid {1-(4-hydroxy-benzyl)-2-oxo-2-[6-oxo-4-(2-pyridin-3-yl-acetyl)-hexahydro-pyrrolo[3,2-b]pyrrol-1-yl]-ethyl}-amide;
Quinoline-8-carboxylic acid {1-(4-hydroxy-benzyl)-2-oxo-2-[6-oxo-4-(2-pyridin-3-yl-acetyl)-hexahydro-pyrrolo[3,2-b]pyrrol-1-yl]-ethyl}-amide;
4-tert-Butyl-N-{1-(4-hydroxy-benzyl)-2-oxo-2-[6-oxo-4-(2-pyridin-3-yl-acetyl)-hexahydro-pyrrolo[3,2-b]pyrrol-1-yl]-ethyl}-benzamide;
4-tert-Butyl-N-{1-(4-fluoro-benzyl)-2-oxo-2-[6-oxo-4-(2-pyridin-3-yl-acetyl)-hexahydro-pyrrolo[3,2-b]pyrrol-1-yl]-ethyl}-benzamide;
4-tert-Butyl-N-{1-(4-methoxy-benzyl)-2-oxo-2-[6-oxo-4-(2-pyridin-3-yl-acetyl)-hexahydro-pyrrolo[3,2-b]pyrrol-1-yl]-ethyl}-benzamide;
4-tert-Butyl-N-{1-(3-hydroxy-benzyl)-2-oxo-2-[6-oxo-4-(2-pyridin-3-yl-acetyl)-hexahydro-pyrrolo[3,2-b]pyrrol-1-yl]-ethyl}-benzamide;
Biphenyl-4-carboxylic acid {1-(4-hydroxy-benzyl)-2-oxo-2-[6-oxo-4-(2-pyridin-3-yl-acetyl)-hexahydro-pyrrolo[3,2-b]pyrrol-1-yl]-ethyl}-amide;
Biphenyl-4-carboxylic acid {1-(4-fluoro-benzyl)-2-oxo-2-[6-oxo-4-(2-pyridin-3-yl-acetyl)-hexahydro-pyrrolo[3,2-b]pyrrol-1-yl]-ethyl}-amide;
Biphenyl-4-carboxylic acid {1-(4-methoxy-benzyl)-2-oxo-2-[6-oxo-4-(2-pyridin-3-yl-acetyl)-hexahydro-pyrrolo[3,2-b]pyrrol-1-yl]-ethyl}-amide;
Biphenyl-4-carboxylic acid {1-(3-hydroxy-benzyl)-2-oxo-2-[6-oxo-4-(2-pyridin-3-yl-acetyl)-hexahydro-pyrrolo[3,2-b]pyrrol-1-yl]-ethyl}-amide;
Furan-3-carboxylic acid {3,3-dimethyl-1-[6-oxo-4-(pyridin-2-ylmethanesulfonyl)-hexahydro-pyrrolo[3,2-b]pyrrole-1-carbonyl]-butyl}-amide;
Thiophene-3-carboxylic acid {3,3-dimethyl-1-[6-oxo-4-(pyridin-2-ylmethanesulfonyl)-hexahydro-pyrrolo[3,2-b]pyrrole-1-carbonyl]-butyl}-amide;
Benzo[b]thiophene-3-carboxylic acid {3,3-dimethyl-1-[6-oxo-4-(pyridin-2-ylmethanesulfonyl)-hexahydro-pyrrolo[3,2-b]pyrrole-1-carbonyl]-butyl}-amide;
Furan-3-carboxylic acid {1-cyclohexylmethyl-2-oxo-2-[6-oxo-4-(pyridin-2-ylmethanesulfonyl)-hexahydro-pyrrolo[3,2-b]pyrrol-1-yl]-ethyl}-amide;
Thiophene-3-carboxylic acid {1-cyclohexylmethyl-2-oxo-2-[6-oxo-4-(pyridin-2-ylmethanesulfonyl)-hexahydro-pyrrolo[3,2-b]pyrrol-1-yl]-ethyl}-amide;
Benzo[b]thiophene-3-carboxylic acid {1-cyclohexylmethyl-2-oxo-2-[6-oxo-4-(pyridin-2-ylmethanesulfonyl)-hexahydropyrrolo[3,2-b]pyrrol-1-yl]-ethyl}-amide;
Naphthalene-1-carboxylic acid {1-(4-hydroxy-benzyl)-2-oxo-2-[6-oxo-4-(pyridin-2-ylmethanesulfonyl)-hexahydro-pyrrolo[3,2-b]pyrrol-1-yl]-ethyl}-amide;
Quinoline-8-carboxylic acid {1-(4-hydroxy-benzyl)-2-oxo-2-[6-oxo-4-(pyridin-2-ylmethanesulfonyl)-hexahydro-pyrrolo[3,2-b]pyrrol-1-yl]-ethyl}-amide;
4-tert-Butyl-N-{1-(4-hydroxy-benzyl)-2-oxo-2-[6-oxo-4-(pyridin-2-ylmethanesulfonyl)-hexahydro-pyrrolo[3,2-b]-pyrrol-1-yl]-ethyl}-benzamide;
4-tert-Butyl-N-{1-(4-fluoro-benzyl)-2-oxo-2-[6-oxo-4-(pyridin-2-ylmethanesulfonyl)-hexahydro-pyrrolo[3,2-b]pyrrol-1-yl]-ethyl}-benzamide;
4-tert-Butyl-N-{1-(4-methoxy-benzyl)-2-oxo-2-[6-oxo-4-(pyridin-2-ylmethanesulfonyl)-hexahydro-pyrrolo[3,2-b]-pyrrol-1-yl]-ethyl}-benzamide;
4-tert-Butyl-N-{1-(3-hydroxy-benzyl)-2-oxo-2-[6-oxo-4-(pyridin-2-ylmethanesulfonyl)-hexahydro-pyrrolo[3,2-b]-pyrrol-1-yl]-ethyl}-benzamide;
Biphenyl-4-carboxylic acid {1-(4-hydroxy-benzyl)-2-oxo-2-[6-oxo-4-(pyridin-2-ylmethanesulfonyl)-hexahydro-pyrrolo[3,2-b]pyrrol-1-yl]-ethyl}-amide;
Biphenyl-4-carboxylic acid {1-(4-fluoro-benzyl)-2-oxo-2-[6-oxo-4-(pyridin-2-ylmethanesulfonyl)-hexahydro-pyrrolo[3,2-b]pyrrol-1-yl]-ethyl}-amide;
Biphenyl-4-carboxylic acid {1-(4-methoxy-benzyl)-2-oxo-2-[6-oxo-4-(pyridin-2-ylmethanesulfonyl)-hexahydro-pyrrolo[3,2-b]pyrrol-1-yl]-ethyl}-amide;
Biphenyl-4-carboxylic acid {1-(3-hydroxy-benzyl)-2-oxo-2-[6-oxo-4-(pyridin-2-ylmethanesulfonyl)-hexahydro-pyrrolo[3,2-b]pyrrol-1-yl]-ethyl}-amide;
Furan-3-carboxylic acid {3,3-dimethyl-1-[6-oxo-4-(pyridin-3-ylmethanesulfonyl)-hexahydro-pyrrolo[3,2-b]pyrrole-1-carbonyl]-butyl}-amide;

Thiophene-3-carboxylic acid {3,3-dimethyl-1-[6-oxo4-(pyridin-3-ylmethanesulfonyl)-hexahydro-pyrrclo[3,2-b]pyrrole-1-carbonyl]-butyl}-amide;
Benzo[b]thiophene-3-carboxylic acid {3,3-dimethyl-1-[6-oxo-4-(pyridin-3-ylmethanesulfonyl)-hexahydro-pyrcolo[3,2-b]pyrrole-1-carbonyl]-butyl}-amide;
Furan-3-carboxylic acid {1-cyclohexylmethyl-2-oxo-2-[6-oxo-4-(pyridin-3-ylmethanesulfonyl)-hexahydro-pyrrolo[3,2-b]pyrrol-1-yl]-ethyl}-amide;
Thiophene-3-carboxylic acid {1-cyclohexylmethyl-2-oxo-2-[6-oxo-4-(pyridin-3-ylmethanesulfonyl)-hexahydro-pyrrolo[3,2-b]pyrrol-1-yl]-ethyl}-amide;
Benzo[b]thiophene-3-carboxylic acid {1-cyclohexylmethyl-2-oxo-2-[6-oxo-4-(pyridin-3-ylmethanesulfonyl)-hexahydro-pyrrolo[3,2-b]pyrrol-1-yl]-ethyl}-amide;
Naphthalene-1-carboxylic acid {1-(4-hydroxy-benzyl)-2-oxo-2-[6-oxo-4-(pyridin-3-ylmethanesulfonyl)-hexahydro-pyrrolo[3,2-b]pyrrol-1-yl]-ethyl}-amide;
Quinoline-8-carboxylic acid {1-(4-hydroxy-benzyl)-2-oxo-2-[6-oxo-4-(pyridin-3-ylmethanesulfonyl)-hexahydro-pyrrolo[3,2-b]pyrrol-1-yl]-ethyl}-amide;
4-tert-Butyl-N-{1-(4-hydroxy-benzyl)-2-oxo-2-[6-oxo-4-(pyridin-3-ylmethanesulfonyl)-hexahydro-pyrrolo[3,2-b]-pyrrol-1-yl]-ethyl}-benzamide;
4-tert-Butyl-N-{1-(4-fluoro-benzyl)-2-oxo-2-[6-oxo-4-(pyridin-3-ylmethanesulfonyl)-hexahydro-pyrrolo[3,2-b]pyrrol-1-yl]-ethyl}-benzamide;
4-tert-Butyl-N-{1-(4-methoxy-benzyl)-2-oxo-2-[6-oxo-4-(pyridin-3-ylmethanesulfonyl)-hexahydro-pyrrolo[3,2-b]-pyrrol-1-yl]-ethyl}-benzamide;
4-tert-Butyl-N-{1-(3-hydroxy-benzyl)-2-oxo-2-[6-oxo-4-(pyridin-3-ylmethanesulfonyl)-hexahyro-pyrrolo[3,2-b]-pyrrol-1-yl]-ethyl}-benzmide;
Biphenyl-4-carboxylic acid {1-(4-hydroxy-benzyl)-2-oxo-2-[6-oxo-4-(pyridin-3-ylmethanesulfonyl)-hexahydro-pyrrolo[3,2-b]pyrrol-1-yl]-ethyl}-amide;
Biphenyl-4-carboxylic acid {1-(4-fluoro-benzyl)-2-oxo-2-[6-oxo-4-(pyridin-3-ylmethanesulfonyl)-hexahydro-pyrrolo[3,2-b]pyrrol-1-yl]-ethyl}-amide;
Biphenyl-4-carboxylic acid {1-(4-methoxy-benzyl)-2-oxo-2-[6-oxo-4-(pyridin-3-ylmethanesulfonyl)-hexahydro-pyrrolo[3,2-b]pyrrol-1-yl]-ethyl}-amide;
Biphenyl-4-carboxylic acid {1-(3-hydroxy-benzyl)-2-oxo-2-[6-oxo-4-(pyridin-3-ylmethanesulfonyl)-hexahydro-pyrrolo[3,2-b]pyrrol-1-yl]-ethyl}-amide;
Furan-3-carboxylic acid {3,3-dimethyl-1-[6-oxo-4-(1-oxy-pyridin-2-ylmethanesulfonyl)-hexahydro-pyrrolo[3,2-b]pyrrole-1-carbonyl]-butyl}-amide;
Thiophene-3-carboxylic acid {3,3-dimethyl-1-[6-oxo-4-(1-oxy-pyridin-2-ylmethanesulfonyl)-hexahydro-pyrrolo[3,2-b]pyrrole-1-carbonyl]-butyl}-amide;
Benzo[b]thiophene-3-carboxylic acid {3,3-dimethyl-1-[6-oxo-4-(1-oxy-pyridin-2-ylmethanesulfonyl)-hexahydro-pyrrolo[3,2-b]pyrrole-1-carbonyl]-butyl}-amide;
Furan-3-carboxylic acid {1-cyclohexylmethyl-2-oxo-2-[6-oxo-4-(1-oxy-pyridin-2-ylmethanesulfonyl)-hexahydro-pyrrolo[3,2-b]pyrrol-1-yl]-ethyl}-amide;
Thiophene-3-carboxylic acid {1-cyclohexylmethyl-2-oxo-2-[6-oxo-4-(1-oxy-pyridin-2-ylmethanesulfonyl)-hexahydro-pyrrolo[3,2-b]pyrrol-1-yl]-ethyl}-amide;
Benzo[b]thiophene-3-carboxylic acid {1-cyclohexylmethyl-2-oxo-2-[6-oxo-4-(1-oxy-pyridin-2-ylmethanesulfonyl)-hexahydro-pyrrolo[3,2-b]pyrrol-1-yl]-ethyl}-amide;
Naphthalene-1-carboxylic acid {1-(4-hydroxy-benzyl)-2-oxo-2-[6-oxo-4-(1-oxy-pyridin-2-ylmethanesulfonyl)-hexahydro-pyrrolo[3,2-b]pyrrol-1-yl]-ethyl}-amide;
Quinoline-8-carboxylic acid {1-(4-hydroxy-benzyl)-2-oxo-2-[6-oxo-4-(1-oxy-pyridin-2-ylmethanesulfonyl)-hexahydro-pyrrolo[3,2-b]pyrrol-1-yl]-ethyl}-amide;
4-tert-Butyl-N-{1-(4-hydroxy-benzyl)-2-oxo-2-[6-oxo-4-(1-oxy-pyridin-2-ylmethanesulfonyl)-hexahydro-pyrrolo[3,2-b]pyrrol-1-yl]-ethyl}-benzamide;
4-tert-Butyl-N-{1-(4-fluoro-benzyl)-2-oxo-2-[6-oxo-4-(1-oxy-pyridin-2-ylmethanesulfonyl)-hexahydro-pyrrolo[3,2-b]pyrrol-1-yl]-ethyl}-benzamide;
4-tert-Butyl-N-{1-(4-methoxy-benzyl)-2-oxo-2-[6-oxo-4-(1-oxy-pyridin-2-ylmethanesulfonyl)-hexahydro-pyrrolo[3,2-b]pyrrol-1-yl]-ethyl}-benzamide;
4-tert-Butyl-N-{1-(3-hydroxy-benzyl)-2-oxo-2-[6-oxo-4-(1-oxy-pyridin-2-ylmethanesulfonyl)-hexahydro-pyrrolo[3,2-b]pyrrol-1-yl]-ethyl}-benzamide;
Biphenyl-4-carboxylic acid {1-(4-hydroxy-benzyl)-2-oxo-2-[6-oxo-4-(1-oxy-pyridin-2-ylmethanesulfonyl)-hexahydro-pyrrolo[3,2-b]pyrrol-1-yl]-ethyl}-amide;
Biphenyl-4-carboxylic acid {1-(4-fluom-benzyl)-2-oxo-2-[6-oxo-4-(1-oxy-pyridin-2-ylmethanesulfonyl)-hexahydro-pyrrolo[3,2-b]pyrrol-1-yl]-ethyl}-amide;

Biphenyl-4-carboxylic acid {1-(4-methoxy-benzyl)-2-oxo-2-[6-oxo-4-(1-oxy-pyridin-2-ylmethanesulfonyl)-hexahydro-pyrrolo[3,2-b]pyrrol-1-yl]-ethyl}-amide;
Biphenyl-4-carboxylic acid {1-(3-hydroxy-benzyl)-2-oxo-2-[6-oxo-4-(1-oxy-pyridin-2-ylmethanesulfonyl)-hexahydro-pyrrolo[3,2-b]pyrrol-1-yl]-ethyl}-amide;
Furan-3-carboxylic acid {3,3-dimethyl-1-[6-oxo-4-(1-oxy-pyridin-3-ylmethanesulfonyl)-hexahydro-pyrrolo[3,2-b]pyrrole-1-carbonyl]-butyl}-amide;
Thiophene-3-carboxylic acid {3,3-dimethyl-1-[6-oxo-4-(1-oxy-pyridin-3-ylmethanesulfonyl)-hexahydro-pyrrolo[3,2-b]pyrrole-1-carbonyl]-butyl}-amide;
Benzo[b]thiophene-3-carboxylic acid {3,3-dimethyl-1-[6-oxo-4-(1-oxy-pyridin-3-ylmethanesulfonyl)-hexahydro-pyrrolo[3,2-b]pyrrole-1-carbonyl]-butyl}-amide;
Furan-3-carboxylic acid {1-cyclohexylmethyl-2-oxo-2-[6-oxo-4-(1-oxy-pyridin-3-ylmethanesulfonyl)-hexahydro-pyrrolo[3,2-b]pyrrol-1-yl]-ethyl}-amide;
Thiophene-3-carboxylic acid {1-cyclohexylmethyl-2-oxo-2-[6-oxo-4-(1-oxy-pyridin-3-ylmethanesulfonyl)-hexahydro-pyrrolo[3,2-b]pyrrol-1-yl]-ethyl}-amide;
Benzo[b]thiophene-3-carboxylic acid {1-cyclohexylmethyl-2-oxo-2-[6-oxo-4-(1-oxy-pyridin-3-ylmethanesulfonyl)-hexahydro-pyrrolo[3,2-b]pyrrol-1-yl]-ethyl}-amide;
Naphthalene-1-carboxylic acid {1-(4-hydroxy-benzyl)-2-oxo-2-[6-oxo-4-(1-oxy-pyridin-3-ylmethanesulfonyl)-hexahydro-pyrrolo[3,2-b]pyrrol-1-yl]-ethyl}-amide;
Quinoline-8-carboxylic acid {1-(4-hydroxy-benzyl)-2-oxo-2-[6-oxo-4-(1-oxy-pyridin-3-ylmethanesulfonyl)-hexahydro-pyrrolo[3,2-b]pyrrol-1-yl]-ethyl}-amide;
4-tert-Butyl-N-{1-(4-hydroxy-benzyl)-2-oxo-2-[6-oxo-4-(1-oxy-pyridin-3-ylmethanesulfonyl)-hexahydro-pyrrolo[3,2-b]pyrrol-1-yl]-ethyl}-benzamide;
4-tert-Butyl-N-{1-(4-fluoro-benzyl)-2-oxo-2-[6-oxo-4-(1-oxy-pyridin-3-ylmethanesulfonyl)-hexahydro-pyrrolo[3,2-b]pyrrol-1-yl]-ethyl}-benzamide;
4-tert-Butyl-N-{1-(4-methoxy-benzyl)-2-oxo-2-[6-oxo-4-(1-oxy-pyridin-3-ylmethanesulfonyl)-hexahydro-pyrrolo[3,2-b]pyrrol-1-yl]-ethyl}-benzamide;
4-tert-Butyl-N-{1-(3-hydroxy-benzyl)-2-oxo-2-[6-oxo-4-(1-oxy-pyridin-3-ylmethanesulfonyl)-hexahydro-pyrrolo[3,2-b]pyrrol-1-yl]-ethyl}-benzamide;
Biphenyl-4-carboxylic acid {1-(4-hydroxy-benzyl)-2-oxo-2-[6-oxo-4-(1-oxy-pyridin-3-ylmethanesulfonyl)-hexahydro-pyrrolo[3,2-b]pyrrol-1-yl]-ethyl}-amide;
Biphenyl-4-carboxylic acid {1-(4-fluoro-benzyl)-2-oxo-2-[6-oxo-4-(1-oxy-pyridin-3-ylmethanesulfonyl)-hexahydro-pyrrolo[3,2-b]pyrrol-1-yl]-ethyl}-amide;
Biphenyl-4-carboxylic acid {1-(4-methoxy-benzyl)-2-oxo-2-[6-oxo-4-(1-oxy-pyridin-3-ylmethanesulfonyl)-hexahydro-pyrrolo[3,2-b]pyrrol-1-yl]-ethyl}-amide;
Biphenyl-4-carboxylic acid {1-(3-hydroxy-benzyl)-2-oxo-2-[6-oxo-4-(1-oxy-pyridin-3-ylmethanesulfonyl)-hexahydro-pyrrolo[3,2-b]pyrrol-1-yl]-ethyl}-amide;
2-Cyclohexylmethyl-4-morpholin-4-yl-1-[6-oxo-4-(pyridine-2-sulfonyl)-hexahydro-pyrrolo[3,2-b]pyrrol-1-yl]-butane-1,4-dione;
2-Cyclohexylmethyl-4-morpholin-4-yl-1-[6-oxo-4-(pyridine-3-sulfonyl)-hexahydro-pyrrolo[3,2-b]pyrrol-1-yl]-butane-1,4-dione;
2-Cyclohexylmethyl-4-morpholin-4-yl-1-[6-oxo-4-(1-oxy-pyridine-2-sulfonyl)-hexahydro-pyrrolo[3,2-b]pyrrol-1-yl]-butane-1,4-dione;
2-Cyclohexylmethyl-4-morpholin4-yl-1-[6-oxo-4-(1-oxy-pyridine-3-sulfonyl)-hexahydro-pyrrolo[3,2-b]pyrrol-1-yl]-butane-1,4-dione;
2-Cyclohexylmethyl-4-morpholin-4-yl-1-[6-oxo-4-(pyridine-2-carbonyl)-hexahydro-pyrrolo[3,2-b]pyrrol-1-yl]-butane-1,4-dione;
2-Cyclohexylmethyl-4-morpholin-4-yl-1-[6-oxo-4-(2-pyridin-3-yl-acetyl)-hexahydro-pyrrolo[3,2-b]pyrrol-1-yl]-butane-1,4-dione;
2-Cyclohexylmethyl-4-morpholin-4-yl-1-[6-oxo-4-(pyridin-2-ylmethanesulfonyl)-hexahydro-pyrrolo[3,2-b]pyrrol-1-yl]-butane-1,4-dione;
2-Cyclohexylmethyl-4-morpholin-4-yl-1-[6-oxo-4-(pyridin-3-ylmethanesulfonyl)-hexahydro-pyrrolo[3,2-b]pyrrol-1-yl]-butane-1,4-dione;
2-Cyclohexylmethyl-4-morpholin-4-yl-1-[6-oxo-4-(1-oxy-pyridin-2-ylmethanesulfonyl)-hexahydro-pyrrolo[3,2-b]pyrrol-1-yl]-butane-1,4-dione;
2-Cyclohexylmethyl-4-morpholin-4-yl-1-[6-oxo-4-(1-oxy-pyridin-3-ylmethanesulfonyl)-hexahydro-pyrrolo[3,2-b]pyrrol-1-yl]-butane-1,4-dione;
2-(2,2-Dimethyl-propyl)-4-morpholin-4-yl-1-[6-oxo-4-(pyridine-2-sulfonyl)-hexahydro-pyrrolo[3,2-b]pyrrol-1-yl]-butane-1,4-dione;

2-(2,2-Dimethyl-propyl)-4-morpholin-4-yl-1-[6-oxo-4-(pyridine-3-sulfonyl)-hexahydro-pyrrolo[3,2-b]pyrrol-1-yl]-butane-1,4-dione;
2-(2,2-Dimethyl-propyl)-4-morpholin-4-yl-1-[6-oxo-4-(1-oxy-pyridine-2-sulfonyl)-hexahydro-pyrrolo[3,2-b]pyrrol-1-yl]-butane-1,4-dione;
2-(2,2-Dimethyl-propyl)-4-morpholin-4-yl-1-[6-oxo-4-(1-oxy-pyridine-3-sulfonyl)-hexahydro-pyrrolo[3,2-b]pyrrol-1-yl]-butane-1,4-dione;
2-(2,2-Dimethyl-propyl)-4-morpholin-4-yl-1-[6-oxo-4-(pyridine-2-carbonyl)-hexahydro-pymolo[3,2-b]pyrrol-1-yl]-butane-1,4-dione;
2-(2,2-Dimethyl-propyl)-4-morpholin-4-yl-1-[6-oxo-4-(2-pyridin-3-yl-acetyl)-hexahydro-pyrrolo[3,2-b]pyrrol-1-yl]-butane-1,4-dione;
2-(2,2-Dimethyl-propyl)-4-morpholin-4-yl-1-[6-oxo-4-(pyridin-2-ylmethanesulfonyl)-hexahydro-pyrrolo[3,2-b]pyrrol-1-yl]-butane-1,4-dione;
2-(2,2-Dimethyl-propyl)-4-morpholin-4-yl-1-[6-oxo-4-(pyridin-3-ylmethanesulfonyl)-hexahydro-pyrrolo[3,2-b]pyrrol-1-yl]-butane-1,4-dione;
2-(2,2-Dimethyl-propyl)-4-morpholin-4-yl-1-[6-oxo-4-(1-oxy-pyridin-2-ylmethanesulfonyl)-hexahydro-pyrrolo[3,2-b]pyrrol-1-yl]-butane-1,4-dione;
2-(2,2-Dimethyl-propyl)-4-morpholin-4-yl-1-[6-oxo-4-(1-oxy-pyridin-3-ylmethanesulfonyl)-hexahydro-pyrrolo[3,2-b]pyrrol-1-yl]-butane-1,4-dione;
N-[1-(4-Benzoyl-6-oxo-hexahydro-pyrrolo[3,2-b]pyrrole-1-carbonyl)-3-methyl-butyl]-4-tert-butylbenzamide;
4-tert-Butyl-N-{3-methyl-1-[6-oxo-4-(1-oxy-pyridine-2-carbonyl)-hexahyro-pyrrolo[3,2-b]pyrrole-1-carbonyl]-butyl}-benzamide;
4-tert-Butyl-N-{3-methyl-1-[6-oxo-4-(pyridine-3-carbonyl)-hexahydro-pyrrolo[3,2-b]pyrrole-1-carbonyl]-butyl}-benzamide;
4-tert-Butyl-N-{3-methyl-1-[6-oxo-4-(1-oxy-pyridine-3-carbonyl)-hexahydro-pyrrolo[3,2-b]pyrrole-1-carbonyl]-butyl}-benzamide;
4-tert-Butyl-N-{3-methyl-1-[6-oxo-4-(pyridine-4-carbonyl)-hexahydro-pyrrolo[3,2-b]pyrrole-1-carbonyl]-butyl}-benzamide;
4-tert-Butyl-N-{3-methyl-1-[6-oxo-4-(1-oxy-pyridine-4-carbonyl)-hexahydro-pyrrolo[3,2-b]pyrrole-1-carbonyl]-butyl}-benzamide;
N-[1-(4-Benzenesulfonyl-6-oxo-hexahydro-pyrrolo[3,2-b]pyrrole-1-carbonyl)-3-methyl-butyl]-4-tert-butyl-benzamide;
4-tert-Butyl-N-{3-methyl-1-[6-oxo-4-(pyridine-4-sulfonyl)-hexahydro-pyrrolo[3,2-b]pyrrole-1-carbonyl]-butyl}-benzamide;
4-tert-Butyl-N-{3-methyl-1-[6-oxo-4-(1-oxy-pyridine-4-sulfonyl)-hexahydro-pyrrolo[3,2-b]pyrrole-1-carbonyl]-butyl}-benzamide;
4-tert-Butyl-N-[3-methyl-1-(6-oxo-4-phenylacetyl-hexahydro-pyrrolo[3,2-b]pyrrole-1-carbonyl)-butyl]-benzamide;
4-[2-(4-tert-Butyl-benzoylamino)-4-methyl-pentanoyl]-3-oxo-hexahydro-pyrrolo[3,2-b]pyrrole-1-carboxylic acid phenylamide;
4-[2-(4-tert-Butyl-benzoylamino)-4-methyl-pentanoyl]-3-oxo-hexahydro-pyrrolo[3,2-b]pyrrole-1-carboxylic acid isobutyl-amide;
4-[2-(4-tert-Butyl-benzoylamino)-4-methyl-pentanoyl]-3-oxo-hexahydro-pyrrolo[3,2-b]pyrrole-1-carboxylic acid phenyl ester;
4-[2-(4-tert-Butyl-benzoylamino)-4-methyl-pentanoyl]-3-oxo-hexahydro-pyrrolo[3,2-b]pyrrole-1-carboxylic acid isobutyl ester;
4-tert-Butyl-N-{3-methyl-1-[6-oxo-4-(pyrrolidine-1-carbonyl)-hexahydro-pyrrolo[3,2-b]pyrrole-1-carbonyl]-butyl}-benzamide;
4-tert-Butyl-N-{3-methyl-1-[6-oxo-4-(piperidine-1-carbonyl)-hexahydro-pyrrolo[3,2-b]pyrrole-1-carbonyl]-butyl}-benzamide;
4-tert-Butyl-N-{3-methyl-1-[4-(morpholine-4-carbonyl)-6-oxo-hexahydro-pyrrolo[3,2-b]pyrrole-1-carbonyl]-butyl}-benzamide;
4-tert-Butyl-N-{3-methyl-1-[6-oxo-4-(piperazine-1-carbonyl)-hexahydro-pyrrolo[3,2-b]pyrrole-1-carbonyl]-butyl}-benzamide;
4-tert-Butyl-N-{3-methyl-1-[4-(4-methyl-piperazine-1-carbonyl)-6-oxo-hexahydro-pyrrolo[3,2-b]pyrrole-1-carbonyl]-butyl}-benzamide;
4-tert-Butyl-N-(3-methyl-1-{4-[2-(4-methyl-piperazin-1-yl)-acetyl]-6-oxo-hexahydro-pyrrolo[3,2-b]pyrrole-1-carbonyl}-butyl)-benzamide;
4-tert-Butyl-N-{3-methyl-1-[6-oxo-4-(2-piperazin-1-yl-acetyl)-hexahydro-pyrrolo[3,2-b]pyrrole-1-carbonyl]-butyl}-benzamide;

4-tert-Butyl-N-{1-[4-(furan-2-carbonyl)-6-oxo-hexahydro-pyrrolo[3,2-b]pyrrole-1-carbonyl]-3-methyl-butyl}-benzamide;
4-tert-Butyl-N-{1-[4-(5-chloro-furan-2-carbonyl)-6-oxo-hexahydro-pyrrolo[3,2-b]pyrrole-1-carbonyl]-3-methyl-butyl}-benzamide;
4-tert-Butyl-N-{1-[4-(thiophene-2-carbonyl)-6-oxo-hexahydro-pyrrolo[3,2-b]pyrrole-1-carbonyl]-3-methyl-butyl}-benzamide;
4-tert-Butyl-N-{1-[4-(5-chloro-thiophene-2-carbonyl)-6-oxo-hexahydro-pyrrolo[3,2-b]pyrrole-1-carbonyl]-3-methyl-butyl}-benzamide;
4-tert-Butyl-N-{1-[4-(furan-3-carbonyl)-6-oxo-hexahydro-pyrrolo[3,2-b]pyrrole-1-carbonyl]-3-methyl-butyl}-benzamide;
4-tert-Butyl-N-{1-[4-(thiophene-3-carbonyl)-6-oxo-hexahydro-pyrrolo[3,2-b]pyrrole-1-carbonyl]-3-methyl-butyl}-benzmide;
4-tert-Butyl-N-[1-(4-cyclopentanecarbonyl-6-oxo-hexahyro-pyrrolo[3,2-b]pyrrole-1-carbonyl)-3-methyl-butyl]-benzamide;
4-tert-Butyl-N-[1-(4-cyclohexanecarbonyl-6-oxo-hexahydro-pyrrolo[3,2-b]pyrrole-1-carbonyl)-3-methyl-butyl]-benzamide;
4-tert-Butyl-N-{1-[4-(cyclopent-3-enecarbonyl)-6-oxo-hexahydro-pyrrolo[3,2-b]pyrrole-1-carbonyl]-3-methyl-butyl}-benzamide;
4-tert-Butyl-N-{1-[4-(2-cyclopentyl-acetyl)-6-oxo-hexahydro-pyrrolo[3,2-b]pyrrole-1-carbonyl]-3-methyl-butyl}-benzamide;
4-tert-Butyl-N-{1-[4-(2-cyclohexyl-acetyl)-6-oxo-hexahydro-pyrrolo[3,2-b]pyrrole-1-carbonyl]-3-methyl-butyl}-benzamide;
4-tert-Butyl-N-{3-methyl-1-[6-oxo-4-(pyrrolidine-2-carbonyl)-hexahydro-pyrrolo[3,2-b]pyrrole-1-carbonyl]-butyl}-benzamide;
N-{1-[4-(1-Acetyl-pyrrolidine-2-carbonyl)-6-oxo-hexahydro-pyrrolo[3,2-b]pyrrole-1-carbonyl]-3-methyl-butyl}-4-tert-butyl-benzamide;
4-tert-Butyl-N-{3-methyl-1-[6-oxo-4-(piperidine-2-carbonyl)-hexahydro-pyrrolo[3,2-b]pyrrole-1-carbonyl]-butyl}-benzamide;
N-{1-[4-(1-Acetyl-piperidine-2-carbonyl)-6-oxo-hexahydro-pyrrolo[3,2-b]pyrrole-1-carbonyl]-3-methyl-butyl}-4-tert-butyl-benzamide;
4-tert-Butyl-N-{3-methyl-1-[6-oxo-4-(tetrahydro-furan-2-carbonyl)-hexahydro-pyrrolo[3,2-b]pyrrole-1-carbonyl]-butyl}-benzamide;
N-{1-[4-(1-Amino-cyclobutanecarbonyl)-6-oxo-hexahydro-pyrrolo[3,2-b]pyrrole-1-carbonyl]-3-methyl-butyl}-4-tert-butyl-benzamide;
N-{1-[4-(1-Amino-cyclopentanecarbonyl)-6-oxo-hexahydro-pyrrolo[3,2-b]pyrrole-1-carbonyl]-3-methyl-butyl}-4-tert -butyl-benzamide;
N-{1-[4-(1-Amino-cyclohexanecarbonyl)-6-oxo-hexahydro-pyrrolo[3,2-b]pyrrole-1-carbonyl]-3-methyl-butyl}-4-tert-butyl-benzamide;
N-{1-[4-(1-Acetylamino-cyclobutanecarbonyl)-6-oxo-hexahydro-pyrrolo[3,2-b]pyrrole-1-carbonyl]-3-methyl-butyl}-4-tert-butyl-benzamide;
N-{1-[4-(1-Acetylamino-cyclopentanecarbonyl)-6-oxo-hexahydro-pyrrolo[3,2-b]pyrrole-1-carbonyl]-3-methyl-butyl}-4-tert-butyl-benzamide;
N-{1-[4-(1-Acetylamino-cyclohexanecarbonyl)-6-oxo-hexahydro-pyrrolo[3,2-b]pyrrole-1-carbonyl]-3-methyl-butyl}-4-tert-butyl-benzamide;
4-tert-Butyl-N-{1-[4-(1-hydroxy-cyclobutanecarbonyl)-6-oxo-hexahydro-pyrrolo[3,2-b]pyrrole-1-carbonyl]-3-methyl-butyl}-benzamide;
4-tert-Butyl-N-{1-[4-(1-hydroxy-cyclopentanecarbonyl)-6-oxo-hexahydro-pyrrolo[3,2-b]pyrrole-1-carbonyl]-3-methyl-butyl}-benzamide;
4-tert-Butyl-N-{1-[4-(1-hydroxy-cyclohexanecarbonyl)-6-oxo-hexahydro-pyrrolo[3,2-b]pyrrole-1-carbonyl]-3-methyl-butyl}-benzamide;
4-tert-Butyl-N-{1-[4-(1-methoxy-cyclobutanecarbonyl)-6-oxo-hexahydro-pyrrolo[3,2-b]pyrrole-1-carbonyl]-3-methyl-butyl}-benzamide;
4-tert-Butyl-N-{1-[4-(1-methoxy-cyclopentanecarbonyl)-6-oxo-hexahydro-pyrrolo[3,2-b]pyrrole-1-carbonyl]-3 -methylbutyl}-benzamide;
4-tert-Butyl-N-{1-[4-(1-methoxy-cyclohexanecarbonyl)-6-oxo-hexahydro-pyrrolo[3,2-b]pyrrole-1-carbonyl]-3-methyl-butyl}-benzamide;
N-{1-[4-(2-Amino-2-cyclopentyl-acetyl)-6-oxo-hexahydro-pyrrolo[3,2-b]pyrrole-1-carbonyl]-3-methyl-butyl}-4-tert-butyl-benzamide;

N-1-[4-(2-Amino-2-cyclohexyl-acetyl)-6-oxo-hexahydro-pyrrolo[3,2-b]pyrrole-1-carbonyl]-3-methyl-butyl}-4-tert-butyl-benzamide;
N-{1-[4-(2-Acetylamino-2-cyclopentyl-acetyl)-6-oxo-hexahydro-pyrrolo[3,2-b]pyrrole-1-carbonyl]-3-methyl-butyl}-4-tert-butyl-benzamide;
N-{1-[4-(2-Acetylamino-2-cyclohexyl-acetyl)-6-oxo-hexahydro-pyrrolo[3,2-b]pyrrole-1-carbonyl]-3-methyl-butyl}-4-tert-butyl-benzamide;
N-{1-[4-(2-Acetylamino-propionyl)-6-oxo-hexahydro-pyrrolo[3,2-b]pyrrole-1-carbonyl]-3-methyl-butyl}-4-tert-butyl-benzamide;
N-{1-[4-(2-Acetylamino-acetyl)-6-oxo-hexahydro-pyrrolo[3,2-b]pyrrole-1-carbonyl]-3-methyl-butyl}-4-tert-butyl-benzamide;
N-(1-{4-[2-(Acetyl-methyl-amino)-acetyl]-6-oxo-hexahydro-pyrrolo[3,2-b]pyrrole-1-carbonyl}-3-methyl-butyl)-4-tert-butyl-benzamide;
4-tert-Butyl-N-{1-[4-(2-dimethylamino-acetyl)-6-oxo-hexahydro-pyrrolo[3,2-b]pyrrole-1-carbonyl]-3-methyl-butyl}-benzamide;
N-{1-[4-(2-Amino-butyryl)-6-oxo-hexahydro-pyrrolo[3,2-b]pyrrole-1-carbonyl]-3-methyl-butyl}-4-tert-butyl-benzamide;
N-{1-[4-(2-Acetylamino-butyryl)-6-oxo-hexahydro-pyrrolo[3,2-b]pyrrole-1-carbonyl]-3-methyl-butyl}-4-tert-butyl-benzamide;
N-{1-[4-(2-amino-3-methyl-butyryl)-6-oxo-hexahydro-pyrrolo[3,2-b]pyrrole-1-carbonyl]-3-methyl-butyl}-4-tert-butyl-benzamide;
N-{1-[4-(2-Acetylamino-3-methyl-butyryl)-6-oxo-hexahydro-pyrrolo[3,2-b]pyrrole-1-carbonyl]-3-methyl-butyl}-4-tert-butyl-benzamide;
N-{1-[4-(2-amino-3,3-dimethyl-butyryl)-6-oxo-hexahydro-pyrrolo[3,2-b]pyrrole-1-carbonyl]-3-methyl-butyl}-4-tert-b utyl-benzamide;
N-{1-[4-(2-Acetylamino-3,3-dimethyl-butyryl)-6-oxo-hexahydro-pyrrolo[3,2-b]pyrrole-1-carbonyl]-3-methyl-butyl}-4-tert-butyl-benzamide;
N-{1-[4-(2-Amino-3-methyl-pentanoyl)-6-oxo-hexahydro-pyrrolo[3,2-b]pyrrole-1-carbonyl]-3-methyl-butyl}-4-tert-butyl-benzamide;
N-{1-[4-(2-Acetylamino-3-methyl-pentanoyl)-6-oxo-hexahydro-pyrrolo[3,2-b]pyrrole-1-carbonyl]-3-methyl-butyl}-4-tert-butyl-benzamide;
4-tert-Butyl-N-[3-methyl-1-(6-oxo-4-pentanoyl-hexahydro-pyrrolo[3,2-b]pyrrole-1-carbonyl)-butyl]-benzamide;
4-tert-Butyl-N-{3-methyl-1-[4-(3-methyl-pentanoyl)-6-oxo-hexahydro-pyrrolo[3,2-b]pyrrole-1-carbonyl]-butyl}-benzamide;
4-tert-Butyl-N-{3-methyl-1-[4-(4-methyl-pentanoyl)-6-oxo-hexahydro-pyrrolo[3,2-b]pyrrole-1-carbonyl]-butyl}-benzamide;
N-{1-[4-(2-Amino-4-methyl-pentnoyl)-6-oxo-hexahydro-pymolo[3,2-b]pyrrole-1-carbonyl]-3-methyl-butyl}-4-tert-butyl-benzamide;
N-{1-[4-(2-Acetylamino-4-methyl-pentanoyl)-6-oxo-hexahydro-pyrrolo[3,2-b]pyrrole-1-carbonyl]-3-methyl-butyl}-4-tert-butyl-benzamide;
N-{1-[4-(2-Amino-4,4-dimethyl-pentanoyl)-6-oxo-hexahydro-pyrrolo[3,2-b]pyrrole-1-carbonyl]-3-methyl-butyl}-4-tert-butyl-benzamide;
N-{1-[4-(2-Acetylamino-4,4-dimethyl-pentanoyl)-6-oxo-hexahydro-pyrrolo[3,2-b]pyrrole-1-carbonyl)-3-methyl-butyl}-4-tert-butyl-benzamide;
N-{1-[4-(2-Amino-pentanoyl)-6-oxo-hexahydro-pyrrolo[3,2-b]pyrrole-1-carbonyl]-3-methyl-butyl}-4-tert-butyl-benzamide;
N-{1-[4-(2-Acetylamino-pentanoyl)-6-oxo-hexahydro-pyrrolo[3,2-b]pyrrole-1-carbonyl]-3-methyl-butyl}-4-tert-butyl-benzamide;
N-{1-[4-(2-Amino-5-methyl-hexanoyl)-6-oxo-hexahydro-pyrrolo[3,2-b]pyrrole-1-carbonyl]-3-methyl-butyl}-4-tert-butyl-benzamide;
N-{1-[4-(2-Acetylamino-5-methyl-hexanoyl)-6-oxo-hexahydro-pyrrolo[3,2-b]pyrrole-1-carbonyl]-3-methyl-butyl}-4-t ert-butyl-benzamide;
N-{1-[4-(2-hydroxy-3-methyl-butyryl)-6-oxo-hexahydro-pyrrolo[3,2-b]pyrrole-1-carbonyl]-3-methyl-butyl}-4-tert-butyl-benzamide;
N-{1-[4-(2-methoxy-3-methyl-butyryl)-6-oxo-hexahydro-pyrrolo[3,2-b]pyrrole-1-carbonyl]-3-methyl-butyl}-4-tert-butyl-benzamide;
4-tert-Butyl-N-{1-[4-(2-hydroxy-3,3-dimethyl-butyryl)-6-oxo-hexahydro-pyrrolo[3,2-b]pyrrole-1-carbonyl]-3-methyl-butyl}-benzamide;
4-tert-Butyl-N-{1-[4-(2-methoxy-3,3-dimethyl-butyryl)-6-oxo-hexahydro-pyrrolo[3,2-b]pyrrole-1-carbonyl]-3-methyl-butyl}-benzamide;
4-tert-Butyl-N-{1-[4-(2-hydroxy-3-methyl-pentanoyl)-6-oxo-hexahydro-pyrrolo[3,2-b]pyrrole-1-carbonyl]-3-methyl-butyl}-benzamide;
4-tert-Butyl-N-{1-[4-(2-methoxy-3-methyl-pentanoyl)-6-oxo-hexahydro-pyrrolo[3,2-b]pyrrole-1-carbonyl]-3-methyl-butyl}-benzamide;
4-tert-Butyl-N-{1-[4-(2-hydroxy-4-methyl-pentanoyl)-6-oxo-hexahydro-pyrrolo[3,2-b]pyrrole-1-carbonyl]-3-methyl-butyl}-benzamide;
4-tert-Butyl-N-{1-[4-(2-methoxy-4-methyl-pentanoyl)-6-oxo-hexahydro-pyrrolo[3,2-b]pyrrole-1-carbonyl]-3-methyl-butyl}-benzamide;
4-tert-Butyl-N-{1-[4-(2-hydroxy-4,4-dimethyl-pentanoyl)-6-oxo-hexahydro-pyrrolo[3,2-b]pyrrole-1-carbonyl]-3-methyl-butyl}-benzamide;
4-tert-Butyl-N-{1-[4-(2-methoxy-4,4-dimethyl-pentanoyl)-6-oxo-hexahydro-pyrrolo[3,2-b]pyrrole-1-carbonyl]-3-methyl-butyl}-benzamide;
4-tert-Butyl-N-{1-[4-(2-hydroxy-pentanoyl)-6-oxo-hexahydro-pyrrolo[3,2-b]pyrrole-1-carbonyl]-3-methyl-butyl}-benzamide;
4-tert-Butyl-N-{1-[4-(2-methoxy-pentanoyl)-6-oxo-hexahydro-pyrrolo[3,2, b]pyrrole-1-carbonyl]-3-methyl-butyl}-benzamide;
4-tert-Butyl-N-{1-[4-(2-hydroxy-5-methyl-hexanoyl)-6-oxo-hexahydro-pyrrolo[3,2-b]pyrrole-1-carbonyl]-3-methyl-butyl}-benzamide;
4-tert-Butyl-N-{1-[4-(2-methoxy-5-methyl-hexanoyl)-6-oxo-hexahydro-pyrrolo[3,2-b]pyrrole-1-carbonyl]-3-methyl-butyl}-benzamide;
N-[1-(4-Benzoyl-6-oxo-hexahydro-pyrrolo[3,2-b]pyrrole-1-carbonyl)-3-methyl-butyl]-4-dimethylaminobenzamide;
4-Dimethylamino-N-{3-methyl-1-[6-oxo-4-(1-oxy-pyridine-2-carbonyl)-hexahydro-pyrrolo[3,2-b]pyrrole-1-carbonyl]-butyl}-benzadde;
4-Dimethylamino-N-{3-methyl-1-[6-oxo-4-(pyridine-3-carbonyl)-hexahydro-pyrrolo[3,2-b]pyrrole-1-carbonyl]-butyl}-benzamide;
4-Dimethylamino-N-{3-methyl-1-[6-oxo-4-(1-oxy-pyridine-3-carbonyl)-hexahydro-pyrrolo[3,2-b]pyrrole-1-carbonyl]-butyl}-benzamide;
4-Dimethylamino-N-{3-methyl-1-[6-oxo-4-(pyridine-4-carbonyl)-hexahydro-pyrrolo[3,2-b]pyrrole-1-carbonyl]-butyl}-benzamide;
4-Dimethylamino-N-{3-methyl-1-[6-oxo-4-(1-oxy-pyridine-4-carbonyl)-hexahydro-pyrrolo[3,2-b]pyrrole-1-carbonyl]-butyl}-benzamide;
N-[1-(4-Benzenesulfonyl-6-oxo-hexahydro-pyrrolo[3,2-b]pyrrole-1-carbonyl)-3-methyl-butyl]-4-dimethylamino-benzamide;
4-Dimethylamino-N-{3-methyl-1-[6-oxo-4-(pyridine-4-sulfonyl)-hexahydro-pyrrolo[3,2-b]pyrrole-1-carbonyl]-butyl}-benzamide;
4-Dimethylamino-N-{3-methyl-1-[6-oxo-4-(1-oxy-pyridine-4-sulfonyl)-hexahydro-pyrrolo[3,2-b]pyrrole-1-carbonyl]-butyl}-benzamide;
4-Dimethylamino-N-[3-methyl-1-(6-oxo-4-phenylacetyl-hexahydro-pyrrolo[3,2-b]pyrrole-1-carbonyl)-butyl]-benzamide;
4-[2-(4-Dimethylamino-benzoylamino)-4-methyl-pentanoyl]-3-oxo-hexahydro-pyrrolo[3,2-b]pyrrole-1-carboxylic acid phenylamide;
4-[2-(4-Dimethylamino-benzoylamino)-4-methyl-pentanoyl]-3-oxo-hexahydro-pyrrolo[3,2-b]pyrrole-1-carboxylic acid isobutyl-amide;
4-[2-(4-Dimethylamino-benzoylamino)-4-methyl-pentanoyl]-3-oxo-hexahydro-pyrrolo[3,2-b]pyaole-1-carboxylic acid phenyl ester;
4-[2-(4-Dimethylamino-benzoylamino)-4-methyl-pentanoyl]-3-oxo-hexahydro-pyrrolo[3,2-b]pyrrole-1-carboxylic acid isobutyl ester,
4-Dimethylamino-N-{3-methyl-1-[6-oxo-4-(pyrrolidine-1-carbonyl)-hexahydro-pyrrolo[3,2-b]pyrrole-1-carbonyl]-butyl}-benzamide;
4-Dimethylamino-N-{3-methyl-1-[6-oxo-4-(piperidine-1-carbonyl)-hexahydro-pyrrolo[3,2-b]pyrrole-1-carbonyl]-butyl}-benzamide;
4-Dimethylamino-N-{3-methyl-1-[4-(morpholine-4-carbonyl)-6-oxo-hexahydro-pyrrolo[3,2-b]pyrrole-1-carbonyl]-butyl}-benzamide;
4-Dimethylamino-N-{3-methyl-1-[6-oxo-4-(piperazine-1-carbonyl)-hexahydro-pyrrolo[3,2-b]pyrrole-1-carbonyl]-butyl}-benzamide;
4-Dimethylamino-N-{3-methyl-1-[4-(4-methyl-piperazine-1-carbonyl)-6-oxo-hexahydro-pyrrolo[3,2-b]pyrrole-1-car bonyl]-butyl}-benzamide;

4-Dimethylamino-N-(3-methyl-1-{4-[2-(4-methyl-piperazin-1-yl)-acetyl]-6-oxo-hexahydro-pyrrolo[3,2-b]pyrrole-1-carbonyl}-butyl)-benzamide;
4-Dimethylamino-N-{3-methyl-1-[6-oxo-4-(2-piperazin-1-yl-acetyl)-hexahydro-pyrrolo[3,2-b]pyrrole-1-carbonyl]-butyl}-benzamide;
4-Dimethylamino-N-{1-[4-(furan-2-carbonyl)-6-oxo-hexahydro-pyrrolo[3,2-b]pyrrole-1-carbonyl]-3-methyl-butyl}-benzamide;
4-Dimethylamino-N-{1-[4-(5-chloro-furan-2-carbonyl)-6-oxo-hexahydro-pyrrolo[3,2-b]pyrrole-1-carbonyl]-3-methyl-butyl}-benzamide;
4-Dimethylamino-N-{1-[4-(thiophene-2-carbonyl)-6-oxo-hexahydro-pyrrolo[3,2-b]pyrrole-1-carbonyl]-3-methyl-butyl}-benzamide;
4-Dimethylamino-N-{1-[4-(5-chloro-thiophene-2-carbonyl)-6-oxo-hexahydro-pyrrolo[3,2-b]pyrrole-1-carbonyl]-3-methyl-butyl}-benzamide;
4-Dimethylamino-N-{1-[4-(furan-3-carbonyl)-6-oxo-hexahydro-pyrrolo[3,2-b]pyrrole-1-carbonyl]-3-methyl-butyl}-benzamide;
4-Dimethylamino-N-{1-[4-(thiophene-3-mbonyl)-6-oxo-hexahydro-pyrrolo[3,2-b]pyrrole-1-carbonyl]-3-methyl-butyl}-benzamide;
4-Dimethylamino-N-[1-(4-cyclopentanecarbonyl-6-oxo-hexahydro-pyrrolo[3,2-b]pyrrole-1-carbonyl)-3-methyl-butyl]-benzamide;
4-Dimethylamino-N-[1-4-cyclohexanecarbonyl-6-oxo-hexahydro-pyrrolo[3,2-b]pyrrole-1-carbonyl)-3-methyl-butyl]-benzamide;
4-Dimethylamino-N-{1-[4-(cyclopent-3-enecarbonyl)-6-oxo-hexahydro-pyrrolo[3,2-b]pyrrole-1-carbonyl]-3-methyl-butyl}-benzamide;
4-Dimethylamino-N-{1-[4-(2-cyclopentyl-acetyl)-6-oxo-hexahydro-pyrrolo[3,2-b]pyrrole-1-carbonyl]-3-methyl-butyl}-benzamide;
4-Dimethylamino-N-{1-[4-(2-cyclohexyl-acetyl)-6-oxo-hexahydro-pyrrolo[3,2-b]pyrrole-1-carbonyl]-3-methyl-butyl}-benzamide;
4-Dimethylamino-N-{3-methyl-1-[6-oxo-4-(pyrrolidine-2-carbonyl)-hexahydro-pyrrolo[3,2-b]pyrrole-1-carbonyl]-butyl}-benzmide;
N-{1-[4-(1-Acetyl-pyrrolidine-2-carbonyl)-6-oxo-hexahydro-pyrrolo[3,2-b]pyrrole-1-carbonyl]-3-methyl-butyl}-4-dimethylamino-benzamide;
4-Dimethylamino-N-{3-methyl-1-[6-oxo-4-(pipendine-2-carbonyl)-hexahydro-pyrrolo[3,2-b]pyrrole-1-carbonyl]-butyl}-benzamide;
N-{1-[4-(1-Acetyl-piperidine-2-carbonyl)-6-oxo-hexahydro-pyrrolo[3,2-b]pyrrole-1-carbonyl]-3-methyl-butyl}-4-dimethylamino-benzamide;
4-Dimethylamino-N-{3-methyl-1-[6-oxo-4-(tetrahydro-furan-2-carbonyl)-hexahydro-pyrrolo[3,2-b]pyrrole-1-carbonyl]-butyl}-benzamide;
N-{1-[4-(1-Amino-cyclobutanecarbonyl)-6-oxo-hexahydro-pyrrolo[3,2-b]pyrrole-1-carbonyl]-3-methyl-butyl}-4-dimethylamino-benzamide;
N-{1-[4(1-Amino-cyclopentanecarbonyl)-6-oxo-hexahydro-pyrrolo[3,2-b]pyrrole-1-carbonyl]-3-methyl-butyl}-4-dim ethylamino-benzamide;
N-{1-[4-(1-Amino-cyclohexanecarbonyl)-6-oxo-hexahydro-pyrrolo[3,2-b]pyrrole- - carbonyl]-3-methyl-butyl}-4-dimethylamino-benzamide;
N-{1-[4-(1-Acetylamino-cyclobutanecarbonyl)-6-oxo-hexahydro-pyrrolo[3,2-b]pyrrole-1-carbonyl]-3-methyl-butyl}-4-dimethylamino-benzamide;
N-{1-[4-(1-Acetylamino-cyclopentanecarbonyl)-6-oxo-hexahydro-pyrrolo[3,2-b]pyrrole-1-carbonyl]-3-methyl-butyl}-4-dimethylamino-benzamide;
N-{1-[4-(1-Acetylamino-cyclohexanecarbonyt)-6-oxo-hexahydro-pyrrolo[3,2-b]pyrrole-1-carbonyl]-3-methyl-butyl}-4-dimethylamino-benzamide;
4-Dimethylamino-N-{1-[4-(1-hydroxy-cyclobutanecarbonyl)-6-oxo-hexahydro-pyrrolo[3,2-b]pyrrole-1-carbonyl]-3-methyl-butyl}-benzamide;
4-Dimethylamino-N-{1-[4-(1-hydroxy-cyclopentanecarbonyl)-6-oxo-hexahydro-pyrrolo[3,2-b]pyrrole-1-carbonyl]-3-methyl-butyl}-benzamide;
4-Dimethylamino-N-{1-[4-(1-hydroxy-cyclohexanecarbonyl)-6-oxo-hexahydro-pyrrolo[3,2-b]pyrrole-1-carbonyl]-3-methyl-butyl}-benzamide;
4-Dimethylamino-N-{1-[4-(1-methoxy-cyclobutanecarbonyl)-6-oxo-hexahydro-pyrrolo[3,2-b]pyrrole-1-carbonyl]-3-methyl-butyl}-benzamide;
4-Dimethylamino-N-{1-[4-(1-methoxy-cyclopentanecarbonyl)-6-oxo-hexahydro-pyrrolo[3,2-b]pyrrole-1-carbonyl]-3-methyl-butyl}-benzamide;

4-Dimethylamino-N-{1-[4-(1-methoxy-cyclohexanecarbonyl)-6-oxo-hexahydro-pyrrolo[3,2-b]pyrrole-1-carbonyl]-3-methyl-butyl}-benzamide;
N-{1-[4-(2-Amino-2-cyclopentyl-acetyl)6-oxo-hexahydro-pyrrolo[3,2-b]pyrrole-1-carbonyl]-3-methyl-butyl}-4-dimethylamino-benzamide;
N-{1-[4-(2-Amino-2-cyclohexyl-acetyl)-6-oxo-hexahydro-pyrrolo[3,2-b]pyrrole-1-carbonyl]-3-methyl-butyl}-4-dimethylamino-benzamide;
N-{1-[4-(2-Acetylamino-2-cyclopentyl-acetyl)-6-oxo-hexahydro-pyrrolo[3,2-b]pyrrole-1-carbonyl]-3-methyl-butyl}-4-dimethylamino-benzamide;
N-{1-[4-(2-Acetylamino-2-cyclohexyl-acetyl)-6-oxo-hexahydro-pyrrolo[3,2-b]pyrrole-1-carbonyl]-3-methyl-butyl}-4-dimethylamino-benzamide;
N-{1-[4-(2-Acetylamino-propionyl)-6-oxo-hexahydro-pyrrolo[3,2-b]pyrrole-1-carbonyl]-3-methyl-butyl}-4-dimethylamino-benzamide;
N-{1-[4-(2-Acetylamino-acetyl)-6-oxo-hexahydro-pyrrolo[3,2-b]pyrrole-1-carbonyl]-3-methyl-butyl}-4-dimethylamino-benzamide;
N-(1-{4-[2-(Acetyl-methyl-amino)-acetyl]-6-oxo-hexahydro-pyrrolo[3,2-b]pyrrole-1-carbonyl}-3-methyl-butyl)-4-dimethylamino-benzamide;
4-Dimethylamino-N-{1-[4-(2-dimethylamino-acetyl)-6-oxo-hexahydro-pyrrolo[3,2-b]pyrrole-1-carbonyl]-3-methyl-butyl}-benzamide;
N-{1-[4-(2-Amino-butyryl)-6-oxo-hexahydro-pyrrolo[3,2-b]pyrrole-1-carbonyl]-3-methyl-butyl}-4-dimethylamino-benzamide;
N-{1-[4-(2-Acetylamino-butyryl)-6-oxo-hexahydro-pyrrolo[3,2-b]pyrrole-1-carbonyl]-3-methyl-butyl}-4-dimethylamino-benzamide;
N-{1-[4-(2-amino-3-methyl-buwryl)-6-oxo-hexthydro-pyrrolo[3,2-b]pyrrole-1-carbonyl]-3-methyl-butyl}-4-dimethylamino-benzamide;
N-{1-[4-(2-Acetylamino-3-methyl-butyryl)-6-oxo-hexahydro-pyrrolo[3,2-b]pyrrole-1-carbonyl]-3-methyl-butyl}-4-dim ethylamino-benzamide;
N-{1-[4-(2-amino-3,3-dimethyl-butyryl)-6-oxo-hexahydro-pyrrolo[3,2-b]pyrrole-1-carbonyl]-3-methyl-butyl}-4-dimet hylamino-benzamide;
N-{1-[4-(2-Acetylamino-3,3-dimethyl-butyryl)-6-oxo-hexahydro-pyrrolo[3,2-b]pyrrole-1-carbonyl]-3-methyl-butyl}-4-dimethylamino-benzamide;
N-{1-[4-(2-Amino-3-methyl-pentanoyl)-6-oxo-hexahydro-pyrrolo[3,2-b]pyrrole-1-carbonyl]-3-methyl-butyl}-4-dimet hylamino-benzamide;
N-{1-[4-(2-Acetylamino-3-methyl-pentanoyl)-6-oxo-hexahydro-pyrrolo[3,2-b]pyrrole-1-carbonyl]-3-methyl-butyl}-4-dimethylamino-benzamide;
4-Dimethylamino-N-[3-methyl-1-(6-oxo-4-pentanoyl-hexahydro-pyrrolo[3,2-b]pyrrole-1-carbonyl)-butyl]-benzamide;
4-Dimethylamino-N-{3-methyl-1-[4-(3-methyl-pentanoyl)-6-oxo-hexahydro-pyrrolo[3,2-b]pyrrole-1-carbonyl]-butyl}-benzamide;
4-Dimethylamino-N-{3-methyl-1-[4-(4-methyl-pentanoyl)-6-oxo-hexahydro-pyrrolo[3,2-b]pyrrole-1-carbonyl]-butyl}-benzamide;
N-{1-[4-(2-Amino-4-methyl-pentanoyl)-6-oxo-hexahydro-pyrrolo[3,2-b]pyrrole-1-carbonyl]-3-methyl-butyl}-4-dimethylamino-benzamide;
N-{1-[4-(2-Acetylamino-4-methyl-pentanoyl)-6-oxo-hexahydro-pyrrolo[3,2-b]pyrrole-1-carbonyl]-3-methyl-butyl}-4-dimethylamino-benzamide;
N-{1-[4-(2-Amino-4,4-dimethyl-pentanoyl)-6-oxo-hexahydro-pyrrolo[3,2-b]pyrrole-1-carbonyl]-3-methyl-butyl}-4-dimethylamino-benzamide;
N-{1-[4-(2-Acetylamino-4,4-dimethyl-pentanoyl)-6-oxo-hexthydro-pyrrolo[3,2-b]pyrrole-1-carbonyl]-3-methyl-butyl}-4-dimethylamino-benzamide;
N-{1-[4-(2-Amino-pentanoyl)-6-oxo-hexahydro-pyrrolo[3,2-b]pyrrole-1-carbonyl]-3-methyl-butyl}-4-dimethylamino-benzamide;
N-{1-[4-(2-Acetylamino-pentanoyl)-6-oxo-hexahydro-pyrrolo[3,2-b]pyrrole-1-carbonyl]-3-methyl-butyl}-4-dimethylamino-benzamide;
N-{1-[4-(2-Amino-5-methyl-hexanoyl)-6-oxo-hexahydro-pyrrolo[3,2-b]pyrrole-1-carbonyl]-3-methyl-butyl}-4-dimethylamino-benzamide;
N-{1-[4-(2-Acetylamino-5-methyl-hexanoyl)-6-oxo-hexahydro-pyrrolo[3,2-b]pyrrole-1-carbonyl]-3-methyl-butyl}-4-dimethylamino-benzamide;
N-{1-[4-(2-hydroxy-3-methyl-butyryl)-6-oxo-hexahydro-pyrrolo[3,2-b]pyrrole-1-carbonyl]-3-methyl-butyl}-4-dimethylamino-benzamide;

N-{1-[4-(2-methoxy-3-methyl-butyryl)-6-oxo-hexahydro-pyrrolo[3,2-b]pyrrole-1-carbonyl]-3-methyl-butyl}-4-dimethylamino-benzamide;
4-Dimethylamino-N-{1-[4-(2-hydroxy-3,3-dimethyl-butyryl)-6-oxo-hexahydro-pyrrolo[3,2-b]pyrrole-1-carbonyl]-3-methyl-butyl}-benzamide;
4-Dimethylamino-N-{1-[4-(2-methoxy-3,3-dimethyl-butyryl)-6-oxo-hexahydro-pyrrolo[3,2-b]pyrrole-1-carbonyl]-3-methyl-butyl}-benzamide;
4-Dimethylamino-N-{1-[4-(2-hydroxy-3-methyl-pentanoyl)-6-oxo-hexahydro-pyrrolo[3,2-b]pyrrole-1-carbonyl]-3-methyl-butyl}-benzamide;
4-Dimethylamino-N-{1-[4-(2-methoky-3-methyl-pentanoyl)-6-oxo-hexahydro-pyrrolo[3,2-b]pyrrole-1-carbonyl]-3-methyl-butyl}-benzamide;
4-Dimethylamino-N-{1-[4-(2-hydroxy-4-methyl-pentanoyl)-6-oxo-hexahydro-pyrrolo[3,2-b]pyrrole-1-carbonyl]-3-methyl-butyl}-benzamide;
4-Dimethylamino-N-{1-[4-(2-methoxy-4-methyl-pentanoyl)-6-oxo-hexahydro-pyrrolo[3,2-b]pyrrole-1-carbonyl]-3-methyl-butyl}-benzamide;
4-Dimethylamino-N-{1-[4-(2-hydroxy-4,4-dimethyl-pentanoyl)-6-oxo-hexahydro-pyrrolo[3,2-b]pyrrole-1-carbonyl]-3-methyl-butyl}-benzamide;
4-Dimethylamino-N-{1-[4-(2-methoxy-4,4-dimethyl-pentanoyl)-6-oxo-hexahydro-pyrrolo[3,2-b]pyrrole-1-carbonyl]-3-methyl-butyl}-benzamide;
4-Dimethylamino-N-{1-[4-(2-hydroxy-pentanoyl)-6-oxo-hexahydro-pyrrolo[3,2-b]pyrrole-1-carbonyl]-3-methyl-butyl}-benzamide;
4-Dimethylamino-N-{1-[4-(2-methoxy-pentanoyl)-6-oxo-hexahydro-pyrrolo[3,2-b]pyrrole-1-carbonyl]-3-methyl-butyl}-benzamide;
4-Dimethylamino-N-{1-[4-(2-hydroxy-5-methyl-hexanoyl)-6-oxo-hexahydro-pyrrolo[3,2-b]pyrrole-1-carbonyl]-3-methyl-butyl}-benzamide;
4-Dimethylamino-N-{1-[4-(2-methoxy-5-methyl-hexanoyl)-6-oxo-hexahydro-pyrrolo[3,2-b]pyrrole-1-carbonyl]-3-methyl-butyl}-benzamide;
N-[1-(4-Benzoyl-6-oxo-hexahydro-pyrtolo[3,2-b]pyrrole-1-carbonyl)-3-methyl-butyl]-4-thiophen-2-yl-benzamide;
N-{3-methyl-1-[6-oxo-4-(1-oxy-pyridine-2-carbonyl)-hexahydro-pyrrolo[3,2-b]pyrrole-1-carbonyl]-butyl}-4-thiophen-2-yl-bemamide;
N-{3-methyl-1-[6-oxo-4-(pyridine-3-carbonyl)-hexahydro-pyrrolo[3,2-b]pyrrole-1-carbonyl]-butyl}-4-thiophen-2-yl-benzamide;
N-{3-methyl-1-[6-oxo-4-(1-oxy-pyridine-3-carbonyl)-hexahydro-pyrrolo[3,2-b]pyrrole-1-carbonyl]-butyl}-4-thiophen-2-yl-benzamide;
N-{3-methyl-1-[6-oxo-4-(pyridine-4-carbonyl)-hexahydro-pyrrolo[3,2-b]pyrrole-1-carbonyl]-butyl}-4-thiophen-2-yl-benzamide;
N-{3-methyl-1-[6-oxo-4-(1-oxy-pxidine-4-carbonyl)-hexahydro-pyrrolo[3,2-b]pyrrole-1-carbonyl]-butyl}-4-thiophen-2-yl-benzamide;
N-[1-(4-Benzenesulfonyl-6-oxo-hexahydro-pyrrolo[3,2-b]pyrrole-1-carbonyl)-3-methyl-butyl]-4-thiophen-2-yl-benzamide;
N-{3-methyl-1-[6-oxo-4-(pyridine-4-sulfonyl)-hexahydro-pyrrolo[3,2-b]pyrrole-1-carbonyl]-butyl}-4-thiophen-2-yl-benzamide;
N-{3-methyl-1-[6-oxo-4-(1-oxy-pyridine-4-sulfonyl)-hexahydro-pyrrolo[3,2-b]pyrrole-1-carbonyl]-butyl}-4-thiophen-2-yl-benzamide;
N-[3-methyl-1-(6-oxo-4-phenylacetyl-hexahydro-pyrrolo[3,2-b]pyrrole-1-carbonyl)-butyl]-4-thiophen-2-yl-ben2amide;
4-[4-Methyl-2-(4-thiophen-2-yl-benzoylamino)-pentanoyl]-3-oxo-hexahydro-pyrrolo[3,2-b]pyrrole-1-carboxylic acid phenylamide;
4-[4-Methyl-2-(4-thiophen-2-yl-benzoylamino)-pentanoyl]-3-oxo-hexahydro-pyrrolo[3,2-b]pyrrole-1-carboxylic acid isobutyl-amide;
4-[4-Methyl-2-(4-thiophen-2-yl-benzoylamino)-pentanoyl]-3-oxo-hexahydro-pyrrolo[3,2-b]pyrrole-1-carboxylic acid phenyl ester;
4-[4-Methyl-2-(4-thiophen-2-yl-benzoylamino)-pentanoyl]-3-oxo-hexahydro-pyrrolo[3,2-b]pyrrole-1-carboxylic acid isobutyl ester;
N-{3-methyl-1-[6-oxo-4-(pyrrolidine-1-carbonyl)-hexahydro-pyrrolo[3,2-b]pyrrole-1-carbonyl]-butyl}-4-thiophen-2-yl-benzamide;
N-{3-methyl-1-[6-oxo-4-(piperidine-1-carbonyl)-hexahydro-pyrrolo[3,2-b]pyrrole-1-carbonyl]-butyl}-4-thiophen-2-yl-benzamide;
N-{3-methyl-1-[4-(morpholine-4-carbonyl)-6-oxo-hexahydro-pyrrolo[3,2-b]pyrrole-1-carbonyl]-butyl}-4-thiophen-2-yl-benzamide;
N-[3-methyl-1-[6-oxo-4-(piperazine-1-carbonyl)-hexahydro-pyrrolo[3,2-b]pyrrrole-1-carbonyl]-butyl}-4-thiophen-2-yl-benzamide;
N-{3-methyl-1-[4-(4-methyl-piperazine-1-carbonyl)-6-oxo-hexahydro-pyrrolo[3,2-b]pyrrole-1-carbonyl]-butyl}-4-thiophen-2-yl-benzamide;
N-(3-methyl-1-{4-[2-(4-methyl-piperazin-1-yl)-acetyl]-6-oxo-hexahydro-pyrrolo[3,2-b]pyrrole-1-carbonyl}-butyl)-4-thiophen-2-yl-benzamide;
N-{3-methyl-1-[6-oxo-4-(2-piperazin-1-yl-acetyl)-hexahydro-pyrrolo[3,2-b]pyrrole-1-carbonyl]-butyl}-4-thiophen-2-yl-benzamide;
N-{1-[4-(furan-2-carbonyl)-6-oxo-hexahydro-pyrrolo[3,2-b]pyrrole-1-carbonyl]-3-methyl-butyl}-4-thiophen-2-yl-benzamide;
N-{1-[4-(5-chloro-furan-2-carbonyl)-6-oxo-hexahydro-pyrrolo[3,2-b]pyrrole-1-carbonyl]-3-methyl-butyl}-4-thiophen-2-yl-benzamide;
N-{1-[4-(thiophene-2-carbonyl)-6-oxo-hexahydro-pyrrolo[3,2-b]pyrrole-1-carbonyl]-3-methyl-butyl}-4-thiophen-2-yl-benzamide;
N-{1-[4-(5-chloro-thiophene-2-carbonyl)-6-oxo-hexahydro-pyrrolo[3,2-b]pyrrole-1-carbonyl]-3-methyl-butyl}-4-thiophen-2-yl-benzamide;
N-{1-[4-(fuan-3-carbonyl)-6-oxo-hexahydro-pyrrolo[3,2-b]pyrrole-1-carbonyl]-3-methyl-butyl}-4-thiophen-2-yl-benzamide;
N-{1-[4-(thiophene-3-carbonyl)-6-oxo-hexahydro-pyrrolo[3,2-b]pyrrole-1-carbonyl]-3-methyl-butyl}-4-thiophen-2-yl-benzamide;
N-[1-(4-cyclopentanecarbonyl-6-oxo-hexahydro-pyrrolo[3,2-b]pyrrole-1-carbonyl)-3-methyl-butyl]-4-thiophen-2-yl-benzamide;
N-[1-(4-cyclohexanecarbonyl-6-oxo-hexahydro-pyrrolo[3,2-b]pyrrole-1-carbonyl)-3-methyl-butyl]-4-thiophen-2-yl-benzamide;
N-{1-[4-(cyclopent-3-enecarbonyl)-6-oxo-hexahydro-pyrrolo[3,2-b]pyrrole-1-carbonyl]-3-methyl-butyl}-4-thiophen-2-yl-benzamide;
N-{1-[4-(2-cyclopentyl-acetyl)-6-oxo-hexahydro-pyrrolo[3,2-b]pyrrole-1-carbonyl]-3-methyl-butyl}-4-thiophen-2-yl-benzamide;
N-{1-[4-(2-cyclohexyl-acetyl)-6-oxo-hexahydro-pyrrolo[3,2-b]pyrrole-1-carbonyl]-3-methyl-butyl}-4-thiophen-2-yl-benzamide;
N-{3-methyl-1-[6-oxo-4-(pyrrolidine-2-carbonyl)-hexahydro-pyrrolo[3,2-b]pyrrole-1-carbonyl]-butyl}-4-thiophen-2-yl-benzamide;
N-{1-[4-(1-Acetyl-pyrrolidine-2-carbonyl)-6-oxo-hexahydro-pyrrolo[3,2-b]pyrrole-1-carbonyl]-3-methyl-butyl}-4-thiophen-2-yl-benzamide;
N-{3-methyl-1-[6-oxo-4-(piperidine-2-carbonyl)-hexahydro-pyrrolo[3,2-b]pyrrole-1-carbonyl]-butyl}-4-thiophen-2-yl-benzamide;
N-{1-[4-(1-Acetyl-piperidine-2-carbonyl)-6-oxo-hexahydro-pyrrolo[3,2-b]pyrrole-1-carbonyl]-3-methyl-butyl}-4-thiophen-2-yl-benzamide;
N-{3-methyl-1-[6-oxo-4-(tetrahydro-furan-2-carbonyl)-hexahydro-pyrrolo[3,2-b]pyrrole-1-carbonyl]-butyl}-4-thiophen-2-yl-benzamide;
N-{1-[4-(1-Amino-cyclobutanecarbonyl)-6-oxo-hexahydro-pyrrolo[3,2-b]pyrrole-1-carbonyl]-3-methyl-butyl}-4-thiophen-2-yl-benzamide;
N-{1-[4-(1-Amino-cyclopentanecarbonyl)-6-oxo-hexahydro-pyrrolo[3,2-b]pyrrole-1-carbonyl]-3-methyl-butyl}-4-thiophen-2-yl-benzamide;
N-{1-[4-(1-Amino-cyclohexanecarbonyl)-b-oxo-hexahydro-pyrrolo[3,2-b]pyrrole-1-carbonyl]-3-methyl-butyl}-4-thiophen-2-yl-benzamide;
N-{1-[4-(1-Acetylamino-cydobutanecarbonyl)-6-oxo-hexahydro-pyrrolo[3,2-b]pyrrole-1-carbonyl]-3-methyl-butyl}-4-thiophen-2-yl-benzamide;
N-{1-[4-(1-Acetylamino-cyclopentanecarbonyl)-6-oxo-hexahydro-pyrrolo[3,2-b]pyrrole-1-carbonyl]-3-methyl-butyl}-4-thiophen-2-yl-benzamide;
N-{1-[4-(1-Acetylamino-cyclohexanecarbonyl)-6-oxo-hexahydro-pyrrolo[3,2-b]pyrrole-1-carbonyl]-3-methyl-butyl}-4-thiophen-2-yl-benzamide;
N-{1-[4-(1-hydroxy-cyclobutanecarbonyl)-6-oxo-hexahydro-pyrrolo[3,2-b]pyrrole-1-carbonyl]-3-methyl-butyl}-4-thiophen-2-yl-benzamide;
N-{1-[4-(1-hydroxy-cyclopentanecarbonyl)-6-oxo-hexahydro-pyrrolo[3,2-b]pyrrole-1-carbonyl]-3-methyl-butyl}-4-thiophen-2-yl-benzamide;
N-{1-[4-(1-hydroxy-cyclohexanecarbonyl)-6-oxo-hexahydro-pyrrolo[3,2-b]pyrrole-1-carbonyl]-3-methyl-butyl}-4-thiophen-2-yl-benzamide;
N-{1-[4-(1-methoxy-cyclobutanecarbonyl)-6-oxo-hexahydro-pyrrolo[3,2-b]pyrrole-1-carbonyl]-3-methyl-butyl}-4-thiophen-2-yl-benzamide;
N-{1-[4-(1-methoxy-cyclopentanecarbonyl)-6-oxo-hexahydro-pyrrolo[3,2-b]pyrrole-1-carbonyl]-3-methyl-butyl}-4-thiophen-2-yl-benzamide;
N-{1-[4-(1-methoxy-cyclohexanecarbonyl)-6-oxo-hexahydro-pyrrolo[3,2-b]pyrrole-1-carbonyl]-3-methyl-butyl}-4-thiophen-2-yl-benzamide;
N-{1-[4-(2-Amino-2-cyclopentyl-acetyl)-6-oxo-hexahydro-pyrrolo[3,2-b]pyrrole-1-carbonyl]-3-methyl-butyl}-4-thiophen-2-yl-benzamide;
N-{1-[4-(2-Amino-2-cyclohexyl-acetyl)-6-oxo-hexahydro-pyrrolo[3,2-b]pyrrole-1-carbonyl]-3-methyl-butyl}-4-thiophen-2-yl-benzamide;
N-{1-[4-(2-Acetylamino-2-cyclopentyl-acetyl)-6-oxo-hexahydro-pyrrolo[3,2-b]pyrrole-1-carbonyl]-3-methyl-butyl}-4-thiophen-2-yl-benzamide;
N-{1-[4-(2-Acetylamino-2-cyclohexyl-acetyl)-6-oxo-hexahydro-pyaolo[3,2-b]pyrrole-1-carbonyl]-3-methyl-butyl}-4-thiophen-2-yl-benzamide;
N-{1-[4-(2-Acetylamino-propionyl)-6-oxo-hexahydro-pyrrolo[3,2-b]pyrrole-1-carbonyl]-3-methyl-butyl}-4-thiophen-2-yl-benzamide;
N-{1-[4-(2-Acetylamino-acetyl)-6-oxo-hexahydro-pyrrolo[3,2-b]pyrrole-1-carbonyl]-3-methyl-butyl}-4-thiophen-2-yl-benzamide;
N-(1-{4-[2-(Acetyl-methyl-amino)-acetyl]-6-oxo-hexahydro-pyrrolo[3,2-b]pyrrole-1-carbonyl}-3-methyl-butyl)-4-thiophen-2-yl-benzamide;
N-{1-[4-(2-dimethylamino-acetyl)-6-oxo-hexahydro-pyrrolo[3,2-b]pyrrole-1-carbonyl]-3-methyl-butyl}-4-thiophen-2 -yl-benzamide;
N-{1-[4-(2-Amino-butyryl)-6-oxo-hexahydro-pyrrolo[3,2-b]pyrrole-1-carbonyl]-3-methyl-butyl}-4-thiophen-2-yl-benzamide;
N-{1-[4-(2-Acetylmnino-buwryl)-6-oxo-hexahydro-pyrrolo[3,2.b]pyrrole-1-carbonyl]-3-methyl-butyl}-4-thiophen-2-yl-benzamide;
N-{1-[4-(2-amino-3-methyl-butyryl)-6-oxo-hexahydro-pyrrolo[3,2-b]pyrrole-1-carbonyl]-3-methyl-butyl}-4-thiophen-2-yl-benzamide;
N-{1-[4-(2-Acetylamino-3-methyl-butyryl)-6-oxo-hexahydro-pyrrolo[3,2-b]pyrrole-1-carbonyl]-3-methyl-butyl}-4-thiophen-2-yl-benzamide;
N-{1-[4-(2-amino-3,3-dimethyl-butyryl)-6-oxo-hexahydro-pyrrolo[3,2-b]pyrrole-1-carbonyl]-3-methyl-butyl}-4-thiophen-2-yl-benzamide;
N-{1-[4-(2-Acetylamino-3,3-dimethyl-butyryl)-6-oxo-hexahydro-pyrrolo[3,2-b]pyrrole-1-carbonyl]-3-methyl-butyl}-4-thiophen-2-yl-benzamide;
N-{1-[4-(2-Amino-3-methyl-pentanoyl)-6-oxo-hexahydro-pyrrolo[3,2-b]pyrrole-1-carbonyl]-3-methyl-butyl}-4-thiophen-2-yl-benzamide;
N-{1-[4-(2-Acetylamino-3-methyl-pentanoyl)-6-oxo-hexahydro-pyrrolo[3,2-b]pyrrole-1-carbonyl]-3-methyl-butyl}-4-thiophen-2-yl-benzamide;
N-[3-methyl-1-(6-oxo-4-pentanoyl-hexahydro-pyrrolo[3,2-b]pyrrole-1-carbonyl)-butyl]-4-thiophen-2-yl-benzamide;
N-{3-methyl-1-[4-(3-methyl-pentanoyl)-6-oxo-hexahydro-pyrrolo[3,2-b]pyrrole-1-carbonyl]-butyl}-4-thiophen-2-yl-benzamide;
N-{3-methyl-1-[4-(4-methyl-pentanoyl)-6-oxo-hexahydro-pyrrolo[3,2-b]pyrrole-1-carbonyl]-butyl}-4-thiophen-2-yl-benzamide;
N-{1-[4-(2-Amino-4-methyl-pentanoyl)-6-oxo-hexahydro-pyrrolo[3,2-b]pyrrole-1-carbonyl]-3-methyl-butyl}-4-thiophen-2-yl-benzamide;
N-{1-[4-(2-Acetylamino-4-methyl-pentanoyl)-6-oxo-hexahydro-pyrrolo[3,2-b]pyrrole-1-carbonyl]-3-methyl-butyl}-4-thiophen-2-yl-benzamide;
N-{1-[4-(2-Amino-4,4-dimethyl-pentanoyl)-6-oxo-hexahydro-pyrrolo[3,2-b]pyrrole-1-carbonyl]-3-methyl-butyl}-4-thiophen-2-yl-benzamide;
N-{1-[4-(2-Acetylamino-4,4-dimethyl-pentanoyl)-6-oxo-hexahydro-pyrrolo[3,2 b]pyrrole-1-carbonyl]-3-methyl-butyl}-4-thiophen-2-yl-benzamide;
N-{1-[4-(2-Amino-pentanoyl)-6-oxo-hexahydro-pyrrolo[3,2-b]pyrrole-1-carbonyl]-3-methyl-butyl}-4-thiophen-2-yl-benzamide;
N-{1-[4-(2-Acetylamino-pentanoyl)-6-oxo-hexahydro-pyrrolo[3,2-b]pyrrole-1-carbonyl]-3-methyl-butyl}-4-thiophen-2-yl-benzamide;
N-{1-[4-(2-Amino-5-methyl-hexanoyl)-6-oxo-hexahydro-pyrrolo[3,2-b]pyrrole-1-carbonyl]-3-methyl-butyl}-4-thiophen- 2-yl-benzamide;

N-{1-[4-(2-Acetylamino-5-methyl-hexanoyl)-6-oxo-hexahydro-pyrrolo[3,2-b]pyrrole-1-carbonyl]-3-methyl-butyl}-4-thiophen-2-yl-benzamide;
N-{1-[4-(2-hydroxy-3-methyl-butyryl)-6-oxo-hexahydro-pyrrolo[3,2-b]pyrrole-1-carbonyl]-3-methyl-butyl}-4-thiophen-2-yl-benzamide;
N-{1-[4-(2-methoxy-3-methyl-butyryl)-6-oxo-hexahydro-pyrrolo[3,2-b]pyrrole-1-carbonyl]-3-methyl-butyl}-4-thiophen-2-yl-benzamide;
N-{1-[4-(2-hydroxy-3,3-dimethyl-butyryl)-6-oxo-hexahydro-pyrrolo[3,2-b]pyrrole-1-carbonyl]-3-methyl-butyl}-4-thiophen-2-yl-benzamide;
N-{1-[4-(2-methoxy-3,3-dimethyl-butyryl)-6-oxo-hexahydro-pyrrolo[3,2-b]pyrrole-1-carbonyl]-3-methyl-butyl}-4-thiophen-2-yl-benzamide;
N-{1-[4-(2-hydroxy-3-methyl-pentanoyl)-6-oxo-hexahydro-pyrrolo[3,2-b]pyrrole-1-carbonyl]-3-methyl-butyl}-4-thiophen-2-yl-benzamide;
N-{1-[4-(2-methoxy-3-methyl-pentanoyl)-6-oxo-hexahydro-pyrrolo[3,2-b]pyrrole-1-carbonyl]-3-methyl-butyl}-4-thiophen-2-yl-benzamide;
N-{1-[4-(2-hydroxy-4-methyl-pentanoyl)-6-oxo-hexahydro-pyrrolo[3,2-b]pyrrole-1-carbonyl]-3-methyl-butyl}-4-thiophen-2-yl-benzamide;
N-{1-[4-(2-methoxy-4-methyl-pentanoyl)-6-oxo-hexahydro-pyrrolo[3,2-b]pyrrole-1-carbonyl]-3-methyl-butyl}-4-thiophen-2-yl-benzamide;
N-{1-[4-(2-hydroxy-4,4-dimethyl-pentanoyl)-6-oxo-hexahydro-pyrrolo[3,2-b]pyrrole-1-carbonyl]-3-methyl-butyl}-4-thiophen-2-yl-benzamde;
N-{1-[4-(2-methoxy-4,4-dimethyl-pentanoyl)-6-oxo-hexahydro-pyrrolo[3,2-b]pyrrole-1-carbonyl]-3-methyl-butyl}-4-thiophen-2-yl-benzamide;
N-{1-[4-(2-hydroxy-pentanoyl)-6-oxo-hexahydro-pyrrolo[3,2-b]pyrrole-1-carbonyl]-3-methyl-butyl}-4-thiophen-2-yl-benzamide;
N-{1-[4-(2-methoxy-pentanoyl)-6-oxo-hexahydro-pyrrolo[3,2-b]pyrrole-1-carbonyl]-3-methyl-butyl}-4-thiophen-2-yl-benzamide;
N-{1-[4-(2-hydroxy-5-methyl-hexanoyl)-6-oxo-hexahydro-pyrrolo[3,2-b]pyrrole-1-carbonyl]-3-methyl-butyl}-4-thiophen-2-yl-benzamide;
N-{1-[4-(2-methoxy-5-methyl-hexanoyl)-6-oxo-hexahydro-pyrrolo[3,2-b]pyrrole-1-carbonyl]-3-methyl-butyl}-4-thiophen-2-yl-benzamide;
5-Phenyl-thiophene-2-carboxylic acid-[1-(4-Benzoyl-6-oxo-hexahydro-pyrrolo[3,2-b]pyrrole-1-carbonyl)-3-methyl-butyl]-amide;
5-Phenyl-thiophene-2-carboxylic acid-{3-methyl-1-[6-oxo-4-(pyridine-2-carbonyl)-hexahydro-pyrrolo[3,2-b]pyrrole-1-carbonyl]-butyl}-amide;
5-Phenyl-thiophene-2-carboxylic acid-{3-methyl-1-[6-oxo-4-(1-oxy-pyridine-2-carbonyl)-hexahydro-pyrrolo[3,2-b]pyrrole-1-carbonyl]-butyl}-amide;
5-Phenyl-thiophene-2-carboxylic acid-{3-methyl-1-[6-oxo-4-(pyridine-3-carbonyl)-hexahydro-pyrrolo[3,2-b]pyrrole-1-carbonyl]-butyl}-amide;
5-Phenyl-thiophene-2-carboxylic acid-{3-methyl-1-[6-oxo-4-(1-oxy-pyridine-3-carbonyl)-hexahydro-pyrrolo[3,2-b]pyrrole-1-carbonyl]-butyl}-amide;
5-Phenyl-thiophene-2-carboxylic acid-{3-methyl-1-[6-oxo-4-(pyridine-4-carbonyl)-hexahydro-pyrrolo[3,2-b]pyrrole-1-carbonyl]-butyl}-amide;
5-Phenyl-thiophene-2-carboxylic acid-{3-methyl-1-[6-oxo-4-(1-oxy-pyridine-4-carbonyl)-hexahydm-pyrrolo[3,2-b]pyrrole-1carbonyl]-butyl}-amide;
5-Phenyl-thiophene-2-carboxylic acid-[1-(4-Benzenesulfonyl-6-oxo-hexahydro-pyrrolo[3,2-b]pyrrole-1-carbonyl)-3-methyl-butyl]-amide;
5-Phenyl-thiophene-2-carboxylic acid-{3-methyl-1-[6-oxo-4-(pyridine-2-sulfonyl)-hexahydro-pyrrolo[3,2-b]pyrrole-1-carbonyl]-butyl}-amide;
5-Phenyl-thiophene-2-carboxylic acid-{3-methyl-1-[6-oxo-4-(1-oxy-pyridine-2-sulfonyl)-hexahydro-pyrrolo[3,2-b]pyrrole-1-carbonyl]-butyl}-amide;
5-Phenyl-thiophene-2-carboxylic acid-{3-methyl-1-[6-oxo-4-(pyridine-3-sulfonyl)-hexahydro-pyrrolo[3,2-b]pyrrole-1-carbonyl]-butyl}-amide;
5-Phenyl-thiophene-2-carboxylic acid-{3-methyl-1-[6-oxo-4-(1-oxy-pyridine-3-sulfonyl)-hexahydro-pyrrolo[3,2-b]pyrrole-1-carbonyl]-butyl}-amide;
5-Phenyl-thiophene-2-carboxylic acid-{3-methyl-1-[6-oxo-4-(pyridine-4-sulfonyl)-hexahydro-pyrrolo[3,2-b]pyrrole-1-carbonyl]-butyl}-amide;
5-Phenyl-thiophene-2-carboxylic acid-{3-methyl-1-[6-oxo-4-(1-oxy-pyridine-4-sulfonyl)-hexahydro-pyrrolo[3,2-b]pyrrole-1-carbonyl]-butyl}-amide;

5-Phenyl-thiophene-2-carboxylic acid-[3-methyl-1-(6-oxo-4-phenylacetyl-hexahydro-pyrrolo[3,2-b]pyrrole-1-carbony)-butyl]-amide;
4-{4-Methyl-2-[(5-phenyl-thiophene-2-carbonyl)-amino]-pentanoyl}-3-oxo-hexahydro-pyrrolo[3,2-b]pyrrole-1-carboxylic acid phenylamide;
4-{4-Methyl-2-[(5-phenyl-thiophene-2-carbonyl)-amino]-pentanoyl}-3-oxo-hexahydro-pyrrolo[3,2-b]pyrrole-1-carboxylic acid isobutyl-amide;
4-{4-Methyl-2-[(5-phenyl-thiophene-2-carbonyl)-amino]-pentanoyl}-3-oxo-hexahydro-pyrrolo[3,2-b]pyrrole-1-carboxylic acid phenyl ester;
4-{4-Methyl-2-[(5-phenyl-thiophene-2-carbonyl)-amino]-pentanoyl}-3-oxo-hexahydro-pyrrolo[3,2-b]pyrrole-1-carboxylic acid isobutyl ester;
5-Phenyl-thiophene-2-carboxylic acid-{3-methyl-1-[6-oxo-4-(pyrrolidine-1-carbonyl)-hexahydro-pyrrolo[3,2-b]pyrrole-1-carbonyl]-butyl}-amide;
5-Phenyl-thiophene-2-carboxylic acid-{3-methyl-1-[6-oxo-4-(piperidine-1-carbonyl)-hexahydro-pyrrolo[3,2-b]pyrrole-1-carbonyl]-butyl}-amide;
5-Phenyl-thiophene-2-carboxylic acid-{3-methyl-1-[4-(morpholine-4-carbonyl)-6-oxo-hexahydro-pyrrolo[3,2-b]pyrrole-1-carbonyl]-butyl}-amide;
5-Phenyl-thiophene-2-carboxylic acid-{3-methyl-1-[6-oxo-4-(piperazine-1-carbonyl)-hexahydro-pyrrolo[3,2-b]pyrrole-1-carbonyl]-butyl}-amide;
5-Phenyl-thiophene-2-carboxylic acid- {3-methyl-1-[4-(4-methyl-piperazine-1-carbonyl)-6-oxo-hexahydro-pyrrolo[3,2-b]pyrrole-1-carbonyl]-butyl}-amide;
5-Phenyl-thiophene-2-carboxylic acid-(3-methyl-1-{4-[2-(4-methyl-piperazin-1-yl)-acetyl]-6-oxo-hexahydro-pyrrolo[3,2-b]pyrrole-1-carbonyl}-butyl)-amide;
5-Phenyl-thiophene-2-carboxylic acid-{3-methyl-1-[6-oxo-4-(2-piperazin-1-yl-acetyl)-hexahydro-pyrrolo[3,2-b]pyrrole-1-carbonyl]-butyl}-amide;
5-Phenyl-thiophene-2-carboxylic acid-{1-[4-(fumn-2-carbonyl)-6-oxo-hexahydro-pyrrolo[3,2-b]pyrrole-1-carbonyl]-3-methyl-butyl}-amide;
5-Phenyl-thiophene-2-carboxylic acid-{1-[4-(5-chloro-furan-2-carbonyl)-6-oxo-hexahydro-pyrrolo[3,2-b]pyrrole-1-carbonyl]-3-methyl-butyl}-amide;
5-Phenyl-thiophene-2-carboxylic acid-{1-[4-(thiophene-2-carbonyl)-6-oxo-hexahydro-pyrrolo[3,2-b]pyrrole-1-carbonyl]-3-methyl-butyl}-amide;
5-Phenyl-thiophene-2-carboxylic acid-{1-[4-(5-chloro-thiophene-2-carbonyl)-6-oxo-hexahydro-pyrrolo[3,2-b]pyrrole-1-carbonyl]-3-methyl-butyl}-amide;
5-Phenyl-thiophene-2-carboxylic acid-{1-[4-(furan-3-carbonyl)-6-oxo-hexahydro-pyrrolo[3,2-b]pyrrole-1-carbonyl]-3-methyl-butyl}-amide;
5-Phenyl-thiophene-2-carboxylic acid-{1-[4-(thiophene-3-carbonyl)-6-oxo-hexahydro-pyrrolo[3,2-b]pyrrole-1-carbonyl]-3-methyl-butyl}-amide;
5-Phenyl-thiophene-2-carboxylic acid-[1-(4-cyclopentanecarbonyl-6-oxo-hexahydro-pyrrolo[3,2-b]pyrrole-1-carbonyl)-3-methyl-butyl]-amide;
5-Phenyl-thiophene-2-carboxylic acid-[1-(4-cyclohexanecarbonyl-6-oxo-hexahydro-pyrrolo[3,2-b]pyrrole-1-carbonyl)-3-methyl-butyl]-amide;
5-Phenyl-thiophene-2-carboxylic acid-{1-[4-(cyclopent-3-enecarbonyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-cyclopentyl-acetyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-cyclohexyl-acetyl)-6-oxo-hexahydro-pyrrolo[3,2-b]pyrrole-1-carbonyl]-3-methyl-butyl}-amide;
5-Phenyl-thiophene-2-carboxylic acid-{3-methyl-1-[6-oxo-4-(pyrrolidine-2-carbonyl)-hexahydro-pyrrolo[3,2-b]pyrrole-1-carbonyl]-butyl}-amide;
5-Phenyl-thiophene-2-carboxylic acid-{1-[4-(1-Acetyl-pyrrolidine-2-carbonyl)-6-oxo-hexahydro-pyrrolo[3,2-b]pyrrole-1-carbonyl]-3-methyl-butyl}-amide;
5-Phenyl-thiophene-2-carboxylic acid-{3-methyl-1-[6-oxo-4-(piperidine-2-carbonyl)-hexahydro-pyrrolo[3,2-b]pyrrole-1-carbonyl]-butyl}-amide;
5-Phenyl-thiophene-2-carboxylic acid-{1-[4-(1-Acetyl-piperidille-2-carbonyl)-6-oxo-hexahydro-pyrrolo[3,2-b]pyrrole-1-carbonyl]-3-methyl-butyl}-amide;
5-Phenyl-thiophene-2-carboxylic acid-{3-methyl-1-[6-oxo-4-(tetrahydro-furan-2-carbonyl)-hexahydro-pyrrolo[3,2-b]pyrrole-1-carbonyl]-butyl}-amide;
5-Phenyl-thiophene-2-carboxylic acid-{1-[4-(1-Amino-cyclobutanecarbonyl)-6-oxo-hexahydro-pyrrolo[3,2-b]pyrrole-1-carbonyl]-3-methyl-butyl}-amide;

5-Phenyl-thiophene-2-carboxylic acid-{1-[4-(1-Amino-cyclopentanecarbonyl)-6-oxo-hexahydro-pyrrolo[3,2-b]pyrrole-1-carbonyl]-3-methyl-butyl}-amide;
5-Phenyl-thiophene-2-carboxylic acid-{1-[4-(1-Amino-cyclohexanecarbonyl)-6-oxo-hexahydro-pyrrolo[3,2-b]pyrrole-1-carbonyl]-3-methyl-butyl}-amide;
5-Phenyl-thiophene-2-carboxylic acid-{1-[4-(-Acetylarnino-cydobutanecarbonyl)-6-oxo-hexahydm-pyrrolo[3,2-b]-pyrrole-1-carbonyl]-3-methyl-butyl}-amide;
5-Phenyl-thiophene-2-carboxylic acid-{1-[4-(1-Acetylamino-cyclopentanecarbonyl)-6-oxo-hexahydro-pyrrolo[3,2-b]pyrrole-1-carbonyl]-3-methyl-butyl}-amide;
5-Phenyl-thiophene-2-carboxylic acid-{1-[4-(1-Acetylamino-cyclohexauecarbonyl)-6-oxo-hexahydro-pyrrolo[3,2-b]-pyrrole-1-carbonyl]-3-methyl-buty}-amide;
5-Phenyl-thiophene-2-carboxylic acid-{1-[4-(1-hydroxy-cyclobutanecarbonyl)-6-oxo-hexahydro-pyrrolo[3,2-b]pyrrole-1-carbcnyl]-3-methyl-butyl}-amide;
5-Phenyl-thiophene-2-carboxylic acid-{-[4-(1-hydroxy-cyclopentanecarbonyl)-6-oxo-hexahydro-pyrrolo[3,2-b]pyrrole-1-carbonyl]-3-methyl-butyl}-amide;
5-Phenyl-thiophene-2-carboxylic acid-{1-[4-(1-hydroxy-cyclohexanecarbonyl)-6-oxo-hexahydro-pyrrolo[3,2-b]pyrrole-1-carbonyl]-3-methyl-butyl}-amide;
5-Phenyl-thiophene-2-cuboxylic acid-{1-[4-(1-methoxy-cyclobutanecarbonyl)-6-oxo-hexahydro-pyrrolo[3,2-b]pyrrole-1-carbonyl]-3-methyl-butyl}-amide;
5-Phenyl-thiophene-2-carboxylic acid-{1-[4-(1-methoxy-cyclopentanecarbonyl)-6-oxo-hexahydro-pyrrolo[3,2-b]-pyrrole-1-carbonyl]-3-methyl-butyl}-amide;
5-Phenyl-thiophene-2-carboxylic acid-{1-[4-(1-methoxy-cyclohexanecarbonyl)-6-oxo-hexahydro-pyrrolo[3,2-b]pyrrole-1-carbonyl]-3-methyl-butyl}-amide;
5-Phenyl-thiophene-2-carboxylic acid- {1-[4-(2-Amino-2-cyclopentyl-acetyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-Amino-2-cyclohexyl-acetyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-Acetylamino-2-cyclopentyl-acetyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-Acetylamino-2-cyclohexyl-acetyl)-6-oxo-hexahydro-pyrrolo[3,2-b-]pyrrole-1-carbonyl]-3-methyl-butyl}-amide;
5-Phenyl-thiophene-2-carboxylic acid-{1-[4-(2-Acetylamino-propionyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-Acetylamino-acetyl)-6-oxo-hexahydro-pyrrolo[3,2-b]pyrrole-1-carbonyl]-3-methyl-butyl}-amide;
5-Phenyl-thiophene-2-carboxylic acid-(1-{4-[2-(Acetyl-methyl-amino)-acetyl]-6-oxo-hexahydro-pyrrolo[3,2-b]pyrrole-1-carbonyl}-3-methyl-butyl}-amide;
5-Phenyl-thiophene-2-carboxylic acid-{1-[4-(2-dimethylamino-acetyl)-6-oxo-hexahydro-pyrrolo[3,2-b]pyrrole-1-carbonyl]-3-methyl-butyl}-amide;
5-Phenyl-thiophene-2-carboxylic acid- {1-[4-(2-Amino-butyryl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-Acetylamino-butyryl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-amino-3-methyl-butyryl)-6-oxohexahydro-pyrrolo[3,2-b]pyrrole-1-carbonyl]-3-methyl-butyl}-amide;
5-Phenyl-thiophene-2-carboxylic acid-{1-[4-(2-Acetylamino-3-methyl-butyryl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-amino-3,3-dimethyl-butyryl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-Acetylamino-3,3-dimethyl-butyryl)-6-oxo-hexahydro-pyrrolo[3,2-b]-pyrrole-1-carbonyl]-3-methyl-butyl}-amide;
5-Phenyl-thiophene-2-carboxylic acid-{1-[4-(2-Amino-3-methyl-pentanoyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-Acetylamino-3-methyl-pentanoyl)-6-oxo-hexahydro-pyrrolo[3,2-b]-pyrrole-1-carbonyl]-3-methyl-butyl-amide;
5-Phenyl-thiophene-2-carboxylic acid-[3-methyl-1-(6-oxo-4-pentanoyl-hexahydro-pyrrolo[3,2-b]pyrrole-1-carbonyl)-butyl]-amide;
5-Phenyl-thiophene-2-carboxylic acid-{3-methyl-1-[4-(3-methyl-pentanoyl)-6-oxo-hexahydro-pyrrolo[3,2-b]pyrrole-1-carbonyl]-butyl}-amide;

5-Phenyl-thiophene-2-carboxylic acid-{3-methyl-1-[4-(4-methyl-pentanoyl)-6-oxo-hexahydro-pyrrolo[3,2-b]pyrrole-1-carbonyl]-butyl}-amide;
5-Phenyl-thiophene-2-carboxylic acid-{1-[4-(2-Amino-4-methyl-pentanoyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-Acetylamino-4-methyl-pentanoyl)-6-oxo-hexahydro-pyrrolo[3,2-b]-pyrrole-1-carbonyl]-3-methyl-butyl}-amide;
5-Phenyl-thiophene-2-carboxylic acid-{1-[4-(2-Amino-4,4-dimethyl-pentanoyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-Acetylamino-4,4-dimethyl-pentanoyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-Amino-pentanoyl)-6-oxo-hexahydro-pyrrolo[3,2-b]pyrrole-1-carbonyl]-3-methyl-butyl}-amide;
5-Phenyl-thiophene-2-carboxylic acid {1-[4-(2-Acetylamino-pentanoyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-Amino-5-methyl-hexanoyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-Acetylamino-5-methyl-hexanoyl)-6-oxo-hexahydro-pyrrolo[3,2-b]-pyrrole-1-carbonyl]-3-methyl-butyl}-amide;
5-Phenyl-thiophene-2-carboxylic acid-{1-[4-(2-hydroxy-3-methyl-butyryl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-methoxy-3-methyl-butyryl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-hydroxy-3,3-dimethyl-butyryl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-methoxy-3,3-dimethyl-butyryl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-hydroxy-3-methyl-pentanoyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-methoxy-3-methyl-pentanoyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-hydroxy-4-methyl-pentnoyl)-6-oxo hexahydro-pyrrolo[3,2-b]pyrrole-1-carbonyl]-3-methyl-butyl}-amide;
5-Phenyl-thiophene-2-carboxylic acid-{1-[4-(2-methoxy-4-methyl-pentanoyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-hydroxy-4,4-dimethyl-pentanoyl)-6-oxo-hexahydro-pyrrolo[3,2-b]-pyrrole-1-carbonyl]-3-methyl-butyl}-amide;
5-Phenyl-thiophene-2-carboxylic acid-{1-[4-(2-methoxy-4,4-dimethyl-pentanoyl)-6-oxo-hexahydro-pyrrolo[3,2-b]-pyrrole-1-carbonyl]-3-methyl-butyl}-amide;
5-Phenyl-thiophene-2-carboxylic acid =-{1-[4-(2-hydroxy-pentanoyl)-6-oxo-hexahydro-pyrrolo[3,2-b]pyrrole-1-carbonyl]-3-methyl-butyl}-amide;
5-Phenyl-thiophene-2-carboxylic acid- {1-[4-(2-methoxy-pentanoyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-hydroxy-5-methyl-hexanoyl)-6-oxo-hexahydro-pyrrolo[3,2-b]pyrrole-1-carbonyl]-3-methyl-butyl}-amide;
5-Phenyl-thiophene-2-carboxylic acid-{1-[4-(2-methoxy-5-methyl-hexanoyl)-6-oxo-hexahydro-pyrrolo[3,2-b]pyrrole- 1-carbonyl]-3-methyl-butyl}-amide;
N-[1-(4-Benzoyl-6-oxo-hexahydro-pyrrolo[3,2-b]pyrrole-1-carbonyl)-3-methyl-butyl]-4-pyrrolidin-1-ylbenzamide;
N-{3-methyl-1-[6-oxo-4-(pyridine-2-carbonyl)-hexahydro-pyrrolo[3,2-b]pyrrole-1-carbonyl]-butyl}-4-pyrrolidin-1-yl-benzamide;
N-{3-methyl-1-[6-oxo-4-(1-oxy-pyridine-2-carbonyl)-hexahydro-pyrrolo[3,2-b]pyrrole-1-carbonyl]-butyl}-4-pyrrolidin-1-yl-benzamide;
N-{3-methyl-1-[6-oxo-4-(pyridine-3-carbonyl)-hexahydro-pyrrolo[3,2-b]pyrrole-1-carbonyl]-butyl}-4-pyrrolidin-1-yl-benzamide;
N-{3-methyl-1-[6-oxo-4-(1-oxy-pyridine-3-carbonyl)-hexahydro-pyrrolo[3,2-b]pyrrole-1-carbonyl]-butyl}-4-pyrrolidin-1-yl-benzamide;
N-{3-methyl-1-[6-oxo-4-(pyridine-4-carbonyl)-hexahydro-pyrrolo[3,2-b]pyrrole-1-carbonyl]-butyl}-4-pyrrolidin-1-yl-benzamide;
N-{3-methyl-1-[6-oxo-4-(1-oxy-pyridine-4-carbonyl)-hexahydro-pyrrolo[3,2-b]pyrrole-1-carbonyl]-butyl}-4-pyrrolidin-1-yl-benzamide;
N-[1-(4-Benzenesulfonyl-6-oxo-hexahydro-pyrrolo[3,2-b]pyrrole-1-carbonyl)-3-methyl-butyl]-4-pyrrolidin-1-yl-benzamide;
N-{3-methyl-1-[6-oxo-4-(pyridine-2-sulfonyl)-hexahydro-pyrrolo[3,2-b]pyrrole-1-carbonyl]-butyl}-4-pyrrolidin-1-yl-benzamide;
N-{3-methyl-1-[6-oxo-4-(1-oxy-pyridine-2-sulfonyl)-hexahydro-pyrrolo[3,2-b]pyrrole-1-carbonyl]-butyl}-4-pyrrolidin-1-yl-benzamide;
N-{3-methyl-1-[6-oxo-4-(pyridine-3-sulfonyl)-hexahydro-pyrrolo[3,2-b]pyrrole-1-carbonyl]-butyl}-4-pyrrolidin-1yl-benzamide;
N-{3-methyl-1-[6-oxo-4-(1-oxy-pyridine-3-sulfonyl)-hexahydro-pyrrolo[3,2-b]pyrrole-1-carbonyl]-butyl}-4-pyrrolidin-1-yl-benzamide;
N-{3-methyl-1-[6-oxo-4-(pyridine-4-sulfonyl)-hexahydro-pyrrolo[3,2-b]pyrrole-1-carbonyl]-butyl}-4-pyrrolidin-1-yl-benzamide;
N-{3-methyl-1-[6-oxo-4-(1-oxy-pyridine-4-sulfonyl)-hexahydro-pyrrolo[3,2-b]pyrrole-1-carbonyl]-butyl}-4-pyrrolidin-1-yl-benzamide;
N-[3-methyl-1-(6-oxo-4-phenylacetyl-hexahydro-pyrrolo[3,2-b]pyrrole-1-carbonyl)-butyl]-4-pyrrolidin-1-yl-benzamide;
4-[2-(4-pyrrolidin-1-yl-benzoylamino)-4-methyl-pentanoyl]-3-oxo-hexahydro-pyrrolo[3,2-b]pyrrole-1-carboxylic acid phenylamide;
4-[2-(4-pyrrolidin-1-yl-benzoylamino)-4-methyl-pentanoyl]-3-oxo-hexahydro-pyrrolo[3,2-b]pyrrole-1-carboxylic acid isobutyl-amide;
4-[2-(4-pyrrolidin-1-yl-benzoylamino)-4-methyl-pentanoyl]-3-oxo-hexahydro-pyrrolo[3,2-b]pyrrole-1-carboxylic acid phenyl ester,
4-[2-(4-pyrrolidin-1-yl-benzoylamino)-4-methyl-pentanoyl]-3-oxo-hexahydro-pyrrolo[3,2-b]pyrrole-1-carboxylic acid isobutyl ester;
N-{3-methyl-1-[6-oxo-4-(pyrrolidine-1-carbonyl)-hexahydro-pyrrolo[3,2-b]pyrrole-1-carbonyl]-butyl}-4-pyrrolidin-1-yl-benzamide;
N-{3-methyl-1-[6-oxo-4-(piperidine-1-carbonyl)-hexahydro-pyrrolo[3,2-b]pyrrole-1-carbonyl]-butyl}-4-pyrrolidin-1-yl-benzamide;
N-{3-methyl-1-[4-(morpholine-4-carbonyl)-6-oxo-hexahydro-pyrrolo[3,2-b]pyrrole-1-carbonyl]-butyl}-4-pyrrolidin-1-yl-benzamide;
N-{3-methyl-1-[6-oxo-4-(piperazine-1-carbonyl)-hexahydro-pyrrolo[3,2-b]pyrrole-1-carbonyl]-butyl}-4-pyrrolidin-1-yl-benzamide;
N-{3-methyl-1-[4-(4-methyl-piperazine-1-carbonyl)-6-oxo-hexahydro-pyrrolo[3,2-b]pyrrole-1-carbonyl]-butyl}-4-pyrrolidin-1-yl-benzamide;
N-(3-methyl-1-{4-[2-(4-methyl-piperazin-1-yl)-acetyl]-6-oxo-hexahydro-pyrrolo[3,2-b]pyrrole-1-carbonyl}-butyl)-4-pyrrolidin-1-yl-benzamide;
N-{3-methyl-1-[6-oxo-4-(2-piperazin-1-yl-acetyl)-hexahydro-pyrrolo[3,2-b]pyrrole-1-carbonyl]-butyl}-4-pyrrolidin-1-yl-benzamide;
N-{1-[4-(furan-2-carbonyl)-6-oxo-hexahydro-pyrrolo[3,2-b]pyrrole-1-carbonyl]-3-methyl-butyl}-4-pyrrolidin-1-yl-benzamide;
N-{1-[4-(5-chloro-furan-2-carbonyl)-6-oxo-hexahydro-pyrrolo[3,2-b]pyrrole-1-carbonyl]-3-methyl-butyl}-4-pyrrolidin-1-yl-benzamide;
N-{1-[4-(thiophene-2-carbonyl)-6-oxo-hexahydro-pyrrolo[3,2-b]pyrrole-1-carbonyl]-3-methyl-butyl}-4-pyrrolidin-1-yl-benzamide;
N-{1-[4-(5-chloro-thiophene-2-carbonyl)-6-oxo-hexahydro-pyrrolo[3,2-b]pyrrole-1-carbonyl]-3-methyl-butyl}-4-pyrrolidin-1-yl-benzamide;
N-{1-[4-(furan-3-carbonyl)-6-oxo-hexahydro-pyrrolo[3,2-b]pyrrole-1-carbonyl]-3-methyl-butyl}-4-pyrrolidin-1-yl-benzamide;
N-{1-[4-(thiophene-3-carbonyl)-6-oxo-hexahydro-pyrrolo[3,2-b]pyrrole-1-carbonyl]-3-methyl-butyl}-4-pyrrolidin-1-yl-benzamide;
N-[1-(4-cyclopentanecarbonyl-6-oxo-hexahydro-pyrrolo[3,2-b]pyrrole-1-carbonyl)-3-methyl-butyl]-4-pyrrolidin-1-yl-benzamide;
N-[1-(4-cyclohexanecarbonyl-6-oxo-hexahydro-pyrrolo[3,2-b]pyrrole-1-carbonyl)-3-methyl-butyl]-4-pyrrolidin-1-yl-benzamide;
N-{1-[4-(cyclopent-3-enecarbonyl)-6-oxo-hexahydro-pyrrolo[3,2-b]pyrrole-1-carbonyl]-3-methyl-butyl}-4-pyrrolidin-1-yl-benzamide;
N-{1-[4-(2-cyclopentyl-acetyl)-6-oxo-hexahydro-pyrrolo[3,2-b]pyrrole-1-carbonyl]-3-methyl-butyl}-4-pyrrolidin-1-yl-benzamide;
N-{1-[4-(2-cyclohexyl-acetyl)-6-oxo-hexahydro-pyrrolo[3,2-b]pyrrole-1-carbonyl]-3-methyl-butyl}-4-pyrrolidin-1-yl-benzamide;
4-Pyrrolidin-1-yl-N-{3-methyl-1-[6-oxo-4-(pyrrolidine-2-carbonyl)-hexahydro-pyrrolo[3,2-b]pyrrole-1-carbonyl]-butyl}-benzamide;
N-{1-[4-(1-Acetyl-pyrrolidine-2-carbonyl)-6-oxo-hexahydro-pyrrolo[3,2-b]pyrrole-1-carbonyl]-3-methyl-butyl}-4-pyrrolidin-1-yl-benzamide;
N-{3-methyl-1-[6-oxo-4-(piperidine-2-carbonyl)-hexahydro-pyrrolo[3,2-b]pyrrole-1-carbonyl]-butyl}-4-pyrrolidin-1-yl-benzamide;
N-{1-[4-(1-Acetyl-piperidine-2-carbonyl)-6-oxo-hexahydro-pyrrolo[3,2-b]pyrrole-1-carbonyl]-3-methyl-butyl}-4-pyrrolidin-1-yl-benzamide;
N-{3-methyl-1-[6-oxo-4-(tetrahydro-furan-2-carbonyl)-hexahydro-pyrrolo[3,2-b]pyrrole-1-carbonyl]-butyl}-4-pyrrolidin-1-yl-benzamide;
N-{1-[4-(1-Amino-cyclobutanecarbonyl)-6-oxo-hexahydro-pyrrolo[3,2-b]pyrrole-1-carbonyl]-3-methyl-butyl}-4-pyrrolidin-1-yl-benzamide;
N-{1-[4-(1-Amino-cyclopentanecarbonyl)-6-oxo-hexahydro-pyrrolo[3,2-b]pyrrole-1-carbonyl]-3-methyl-butyl}-4-pyrrolidin-1-yl-benzamide;
N-{1-[4-(1-Amino-cyclohexanecarbonyl)-6-oxo-hexahydro-pyrrolo[3,2-b]pyrrole-1-carbonyl]-3-methyl-butyl}-4-pyrrolidin-1-yl-benzamide;
N-{1-[4-(1-Acetylamino-cyclobutanecarbonyl)-6-oxo-hexahydro-pyrrolo[3,2-b]pyrrole-1-carbonyl]-3-methyl-butyl}-4-pyrrolidin-1-yl-benzamide;
N-{1-[4-(1-Acetylamino-cyclopentanecarbonyl)-6-oxo-hexahydro-pyrrolo[3,2-b]pyrrole-1-carbonyl]-3-methyl-butyl}-4-pyrrolidin-1-yl-benzamide;
N-{1-[4-(1-Acetylamino-cyclohexanecarbonyl)-6-oxo-hexahyro-pyrrolo[3,2-b]pyrrole-1-carbonyl]-3-methyl-butyl}-4-pyrrolidin-1-yl-benzamide;
N-{1-[4-(1-hydroxy-cyclobutanecarbonyl)-6-oxo-hexahydro-pyrrolo[3,2-b]pyrrole-1-carbonyl]-3-methyl-butyl}-4-pyrrolidin-1-yl-benzamide;
N-{1-[4-(1-hydroxy-cyclopentanecarbonyl)-6-oxo-hexahydro-pyrrolo[3,2-b]pyrrole-1-carbonyl]-3-methyl-butyl}-4-pyrrolidin-1-yl-benzamide;
N-{1-[4-(1-hydroxy-cyclohexanecarbonyl)-6-oxo-hexahydro-pyrrolo[3,2-b]pyrrole-1-carbonyl]-3-methyl-butyl}-4-pyrrolidin-1-yl-benzamide;
N-{1-[4-(1-methoxy-cyclobutanecarbonyl)-6-oxo-hexahydro-pyrrolo[3,2-b]pyrrole-1-carbonyl]-3-methyl-butyl}-4-pyrrolidin-1-yl-benzamide;
N-{1-[4-(1-methoxy-cyclopentanecarbonyl)-6-oxo-hexahydro-pyrrolo[3,2-b]pyrrole-1-carbonyl]-3-methyl-butyl}-4-pyrrolidin-1-yl-benzamide;
N-{1-[4-(1-methoxy-cyclohexanecarbonyl)-6-oxo-hexahydro-pyrrolo[3,2-b]pyrrole-1-carbonyl]-3-methyl-butyl}-4-pyrrolidin-1-yl-benzamide;
N-{1-[4-(2-Amino-2-cyclopentyl-acetyl)-6-oxo-hexahydro-pyrrolo[3,2-b]pyrrole-1-carbonyl]-3-methyl-butyl}-4-pyrrolidin-1-yl-benzamide;
N-{1-[4-(2-Amino-2-cyclohexyl-acetyl)-6-oxo-hexahydro-pyrrolo[3,2-b]pyrrole-1-carbonyl]-3-methyl-butyl}-4-pyrrolidin-1-yl-benzamide;
N-{1-[4-(2-Acetylamino-2-cyclopentyl-acetyl)-6-oxo-hexahydro-pyrrolo[3,2-b]pyrrole-1-carbonyl]-3-methyl-butyl}-4-pyrrolidin-1-yl-benzamide;
N-{1-[4-(2-Acetylamino-2-cyclohexyl-acetyl)-6-oxo-hexahydro-pyrrolo[3,2-b]pyrrole-1-carbonyl]-3-methyl-butyl}-4-pyrrolidin-1-yl-benzamide;
N-{1-[4-(2-Acetylamino-propionyl)-6-oxo-hexahydro-pyrrolo[3,2-b]pyrrole-1-carbonyl]-3-methyl-butyl}-4-pyrrolidin-1-yl-benzamide;
N-{1-[4-(2-Acetylamino-acetyl)-6-oxo-hexahydro-pyrrolo[3,2-b]pyrrole-1-carbonyl]-3-methyl-butyl}-4-pyrrolidin-1-yl-benzamide;
N-(1-{4-[2-(Acetyl-methyl-amino)-acetyl]-6-oxo-hexahydro-pyrrolo[3,2-b]pyrrole-1-carbonyl}-3-methyl-butyl)-4-pyrrolidin-1-yl-benzamide;
N-{1-[4-(2-dimethylamino-acetyl)-6-oxo-hexahydro-pyrrolo[3,2-b]pyrrole-1-carbonyl]-3-methyl-butyl}-4-pyrrolidin-1-yl-benzamide;
N-{1-[4-(2-Amino-butyryl)-6-oxo-hexahydro-pyrrolo[3,2-b]pyrrole-1-carbonyl]-3-methyl-butyl}-4-pyrrolidin-1-yl-benzmide;
N-{1-[4-(2-Acetylamino-butyryl)-6-oxo-hexahydro-pyrrolo[3,2-b]pyrrole-1-carbonyl]-3-methyl-butyl}-4-pyrrolidin-1-yl-benzamide;
N-{1-[4-(2-amino-3-methyl-butyryl)-6-oxo-hexahydro-pyrrolo[3,2-b]pyrrole-1-carbonyl]-3-methyl-butyl}-4-pyrrolidin-1-yl-benzamide;
N-{1-[4-(2-Acetylamino-3-methyl-butyryl)-6-oxo-hexahydro-pyrrolo[3,2-b]pyrrole-1-carbonyl]-3-methyl-butyl}-4-pyrrolidin-1-yl-benzamide;
N-{1-[4-(2-amino-3,3-dimethyl-butyryl)-6-oxo-hexahydro-pyrrolo[3,2-b]pyrrole-1-carbonyl]-3-methyl-butyl}-4-pyrrolidin-1-yl-benzamide;
N-{1-[4-(2-Acetylamino-3,3-dimethyl-butyryl)-6-oxo-hexahydro-pyrrolo[3,2-b]pyrrole-1-carbonyl]-3-methyl-butyl}-4-pyrrolidin-1-yl-benzamide;
N-{1-[4-(2-Amino-3-methyl-pentanoyl)-6-oxo-hexahydro-pyrrolo[3,2-b]pyrrole-1-carbonyl]-3-methyl-butyl}-4-pyrrolidin-1-yl-benzamide;
N-{1-[4-(2-Acetylamino-3-methyl-pentanoyl)-6-oxo-hexahydro-pyrrolo[3,2-b]pyrrole-1-carbonyl]-3-methyl-butyl}-4-pyrrolidin-1-yl-benzamide;
N-[3-methyl-1-(6-oxo-4-pentanoyl-hexahydro-pyrrolo[3,2-b]pyrrole-1-carbonyl)-butyl]-4-pyrrolidin-1-yl-benzamide;
N-{3-methyl-1-[4-(3-methyl-pentanoyl)-6-oxo-hexahydro-pyrrolo[3,2-b]pyrrole-1-carbonyl]-butyl}-4-pyrrolidin-1-yl-benzamide;
N-{3-methyl-1-[4-(4-methyl-pentanoyl)-6-oxo-hexahydro-pyrrolo[3,2-b]pyrrole-1-carbonyl]-butyl}-4-pyrrolidin-1-yl-benzamide;
N-{1-[4-(2-Amino-4-methyl-pentanoyl)-6-oxo-hexahydro-pyrrolo[3,2-b]pyrrole-1-carbonyl]-3-methyl-butyl}-4-pyrrolidin-1-yl-benzamide;
N-{1-[4-(2-Acetylamino-4-methyl-pentanoyl)-6-oxo-hexahydro-pyrrolo[3,2-b]pyrrole-1-carbonyl]-3-methyl-butyl}-4-pyrrolidin-1-yl-benzamide;
N-{1-[4-(2-Amino-4,4-dimethyl-pentanoyl)-6-oxo-hexahydro-pyrrolo[3,2-b]pyrrole-1-carbonyl]-3-methyl-butyl}-4-pyrrolidin-1-yl-benzamide;
N-{1-[4-(2-Acetylamino-4,4-dimethyl-pentanoyl)-6-oxo-hexahydro-pyrrolo[3,2-b]pyrrole-1-carbonyl]-3-methyl-butyl}-4-pyrrolidin-1-yl-benzamide;
N-{1-[4-(2-Amino-pentanoyl)-6-oxo-hexahydro-pyrrolo[3,2-b]pyrrole-1-carbonyl]-3-methyl-butyl}-4-pyrrolidin-1-yl-benzamide;
N-{1-[4-(2-Acetylamino-pentanoyl)-6-oxo-hexahydro-pyrrolo[3,2-b]pyrrole-1-carbonyl]-3-methyl-butyl}-4-pyrrolidin-1-yl-benzamide;
N-{1-[4-(2-Amino-5-methyl-hexanoyl)-6-oxo-hexahydro-pyrrolo[3,2-b]pyrrole-1-carbonyl]-3-methyl-butyl}-4-pyrrolidin-1-yl-benzamide;
N-{1-[4-(2-Acetylamino-5-methyl-hexanoyl)-6-oxo-hexahydro-pyrrolo[3,2-b]pyrrole-1-carbonyl]-3-methyl-butyl}-4-pyrrolidin-1-yl-benzamide;
N-{1-[4-(2-hydroxy-3-methyl-butyryl)-6-oxo-hexahydro-pyrrolo[3,2-b]pyrrole-1-carbonyl]-3-methyl-butyl}-4-pyrrolidin-1-yl-benzamide;
N-{1-[4-(2-methoxy-3-methyl-butyryl)-6-oxo-hexahydro-pyrrolo[3,2-b]pyrrole-1-carbonyl]-3-methyl-butyl}-4-pyrrolidin-1-yl-benzamide;
N-{1-[4-(2-hydroxy-3,3-dimethyl-butyryl)-6-oxo-hexahydro-pyrrolo[3,2-b]pyrrole-1-carbonyl]-3-methyl-butyl}-4-pyrrolidin-1-yl-benzamide;
N-{1-[4-(2-methoxy-3,3-dimethyl-butyryl)-6-oxo-hexahydro-pyrrolo[3,2-b]pyrrole-1-carbonyl]-3-methyl-butyl}-4-pyrrolidin-1-yl-benzamide;
N-{1-[4-(2-hydroxy-3-methyl-pentanoyl)-6-oxo-hexahydro-pyrrolo[3,2-b]pyrrole-1-carbonyl]-3-methyl-butyl}-4-pyrrolidin-1-yl-benzamide;
N-{1-[4-(2-methoxy-3-methyl-pentanoyl)-6-oxo-hexahydro-pyrrolo[3,2-b]pyrrole-1-carbonyl]-3-methyl-butyl}-4-pyrrolidin-1-yl-benzamide;
N-{1-[4-(2-hydroxy-4-methyl-pentanoyl)-6-oxo-hexahydro-pyrrolo[3,2-b]pyrrole-1-carbonyl]-3-methyl-butyl}-4-pyrrolidin-1-yl-benzamide;
N-{1-[4-(2-methoxy-4-methyl-pentanoyl)-6-oxo-hexahydro-pyrrolo[3,2-b]pyrrole-1-carbonyl]-3-methyl-butyl}-4-pyrrolidin-1-yl-benzamide;
N-{1-[4-(2-hydroxy-4,4-dimethyl-pentanoyl)-6-oxo-hexahydro-pyrrolo[3,2-b]pyrrole-1-carbonyl]-3-methyl-butyl}-4-pyrrolidin-1-yl-benzamide;
N-{1-[4-(2-methoxy-4,4-dimethyl-pentanoyl)-6-oxo-hexahydro-pyrrolo[3,2-b]pyrrole-1-carbonyl]-3-methyl-butyl}-4-pyrrolidin-1-yl-benzamide;
N-{1-[4-(2-hydroxy-pentanoyl)-6-oxo-hexahydro-pyrrolo[3,2-b]pyrrole-1-carbonyl]-3-methyl-butyl}-4-pyrrolidin-1-yl-benzamide;
N-{1-[4-(2-methoxy-pentanoyl)-6-oxo-hexahydro-pyrrolo[3,2-b]pyrrole-1-carbonyl]-3-methyl-butyl}-4-pyrrolidin-1-yl-benzamide;
N-{1-[4-(2-hydroxy-5-methyl-hexanoyl)-6-oxo-hexahydro-pyrrolo[3,2-b]pyrrole-1-carbonyl]-3-methyl-butyl}-4-pyrrolidin-1-yl-benzamide;

N-{1-[4-(2-methoxy-5-methyl-hexanoyl)-6-oxo-hexahydro-pyrrolo[3,2-b]pyrrole-1-carbonyl]-3-methyl-butyl}-4-pyrrolidin-1-yl-benzamide;
N-[1-(4-Benzoyl-6-oxo-hexahydro-pyrrolo[3,2-b]pyrrole-1-carbonyl)-3-methyl-butyl]-4-morpholin-4-ylbenzamide;
N-{3-methyl-1-[6-oxo-4-(1-oxy-pyridine-2-carbonyl)-hexahydro-pyrrolo[3,2-b]pyrrole-1-carbonyl]-butyl}-4-morpholin-4-yl-benzamide;
N-{3-methyl-1-[6-oxo4-(pyridine-3-carbonyl)-hexahydro-pyrrolo[3,2-b]pyrrole-1-carbonyl]-butyl}-4-morpholin-4-yl-benzamide;
N-{3-methyl-1-[6-oxo-4-(1-oxy-pyridine-3-carbonyl)-hexahydro-pyrrolo[3,2-b]pyrrole-1-carbonyl]-butyl}-4-morpholin-4-yl-benzamide;
N-{3-methyl-1-[6-oxo-4-(pyridine-4-carbonyl)-hexahydro-pyrrolo[3,2-b]pyrrole-1-carbonyl]-butyl}-4-morpholin-4-yl-benzamide;
N-{3-methyl-1-[6-oxo-4-(1-oxy-pyridine-4-carbonyl)-hexahydro-pyrrolo[3,2-b]pyrrole-1-carbonyl]-butyl}-4-morpholin-4-yl-benzamide;
N-[1-(4-Benzenesulfonyl-6-oxo-hexahydro-pyrrolo[3,2-b]pyrrole-1-carbonyl)-3-methyl-butyl]-4-morpholin-4-yl-benzamide;
N-{3-methyl-1-[6-oxo-4-(pyridine-4-sulfonyl)-hexahydro-pyrrolo[3,2-b]pyrrole-1-carbonyl]-butyl}-4-morpholin-4-yl-benzamide;
N-{3-methyl-1-[6-oxo-4-(1-oxy-pyridine-4-sulfonyl)-hexahydro-pyrrolo[3,2-b]pyrrole-1-carbonyl]-butyl}-4-morpholin-4-yl-benzamide;
N-[3-methyl-1-(6-oxo4-phenylacetyl-hexahydro-pyrrolo[3,2-b]pyrrole-1-carbonyl)-butyl]-4-morpholin-4-yl-benzamide;
4-[2-(4-morpholin-4-yl-benzoylamino)-4-methyl-pentanoyl]-3-oxo-hexahydro-pyrrolo[3,2-b]pyrrole-1-carboxylic acid phenylamide;
4-[2-(4-morpholin-4-yl-benzoylamino)-4-methyl-pentanoyl]-3-oxo-hexahydro-pyrrolo[3,2-b]pyrrole-1-carboxylic acid isobutyl-amide;
4-[2-(4-morpholin-4-yl-benzoylamino)-4-methyl-pentanoyl]-3-oxo-hexahydro-pyrrolo[3,2-b]pyrrole-1-carboxylic acid phenyl ester;
4-[2-(4-morpholin-4-yl-benzoylamino)-4-methyl-pentanoyl]-3-oxo-hexahydro-pyrrolo[3,2-b]pyrrole-1-carboxylic acid isobutyl ester;
N-{3-methyl-1-[6-oxo-4-(pyrrolidine-1-carbonyl)-hexahydro-pyrrolo[3,2-b]pyrrole-1-carbonyl]-butyl}-4-morpholin-4-yl-benzamide;
N-{3-methyl-1-[6-oxo-4-(piperidine-1-carbonyl)-hexahydropyrrolo[3,2-b]pyrrole-1-carbonyl]-butyl}-4-morpholin-4-yl-benzamide;
N-{3-methyl-1-[4-(morpholine-4-carbonyl)-6-oxo-hexahydro-pyrrolo[3,2-b]pyrrole-1-carbonyl]-butyl}-4-morpholin-4-yl-benzamide;
N-{3-methyl-1-[6-oxo-4-(piperazine-1-carbonyl)-hexahydro-pyrrolo[3,2-b]pyrrole-1-carbonyl]-butyl}-4-morpholin-4-yl-benzamide;
N-{3-methyl-1-[4-(4-methyl-piperazine-1-carbonyl)-6-oxo-hexahydro-pyrrolo[3,2-b]pyrrole-1-carbonyl]-butyl}-4-morpholin-4-yl-benzamide;
N-{3-methyl-1-{4-[2-(4-methyl-piperazin-1-yl)-acetyl]-6-oxo-hexahydro-pyrrolo[3,2-b]pyrrole-1-carbonyl}-butyl)-4-morpholin-4-yl-benzamide;
N-{3-methyl-1-[6-oxo-4-(2-piperazin-1-yl-acetyl)-hexahydro-pyrrolo[3,2-b]pyrrole-1-carbonyl]-butyl}-4-morpholin-4-yl-benzamide;
N-{1-[4-(furan-2-carbonyl)-6-oxo-hexahydro-pyrrolo[3,2-b]pyrrole-1-carbonyl]-3-methyl-butyl}-4-morpholin-4-yl-benzamide;
N-{1-[4-(5-chloro-furan-2-carbonyl)-6-oxo-hexahydro-pyrrolo[3,2-b]pyrrole-1-carbonyl]-3-methyl-butyl}-4-morpholin-4-yl-benzamide;
N-{1-[4-(thiophene-2-carbonyl)-6-oxo-hexahydro-pyrrolo[3,2-b]pyrrole-1-carbonyl]-3-methyl-butyl}-4-morpholin-4-yl-benzamide;
N-{1-[4-(5-chloro-thiophene-2-carbonyl)-6-oxo-hexahydro-pyrrolo[3,2-b]pyrrole-1-carbonyl]-3-methyl-butyl}-4-morpholin-4-yl-benzamide;
N-{1-[4-(furan-3-carbonyl)-6-oxo-hexahydro-pyrrolo[3,2-b]pyrrole-1-carbonyl]-3-methyl-butyl}-4-morpholin-4-yl-benzamide;
N-{1-[4-(thiophene-3-carbonyl)-6-oxo-hexahydro-pyrrolo[3,2-b]pyrrole-1-carbonyl]-3-methyl-butyl}-4-morpholin-4-yl-benzamide;
N-[1-(4-cyclopentanecarbonyl-6-oxo-hexahydro-pyrrolo[3,2-b]pyrrole-1-carbonyl)-3-methyl-butyl]-4-morpholin-4-yl-benzamide;
N-[1-(4-cyclohexanecarbonyl-6-oxo-hexahydro-pyrrolo[3,2-b]pyrrole-1-carbonyl)-3-methyl-butyl]-4-morpholin-4-yl-benzamide;
N-{1-[4-(cyclopent-3-enecarbonyl)-6-oxo-hexahydro-pyrrolo[3,2-b]pyrrole-1-carbonyl]-3-methyl-butyl}-4-morpholin-4-yl-benzamide;
N-{1-[4-(2-cyclopentyl-acetyl)-6-oxo-hexahydro-pyrrolo[3,2-b]pyrrole-1-carbonyl]-3-methyl-butyl}-4-morpholin-4-yl-benzamide;
N-{1-[4-(2-cyclohexyl-acetyl)-6-oxo-hexahydro-pyrrolo[3,2-b]pyrrole-1-carbonyl]-3-methyl-butyl}-4-morpholin-4-yl-benzamide;
4-morpholin-4-yl-N-{3-methyl-1-[6-oxo-4-(pyrrolidine-2-carbonyl)-hexahydro-pyrrolo[3,2-b]pyrrole-1-carbonyl]-butyl}-benzamide;
N-{1-[4-(1-Acetyl-pyrrolidine-2-carbonyl)-6-oxo-hexahydro-pyrrolo[3,2-b]pyrrole-1-carbonyl]-3-methyl-butyl}-4-morpholin-4-yl-benzamide;
N-{3-methyl-1-[6-oxo-4-(piperidine-2-carbonyl)-hexahydro-pyrrolo[3,2-b]pyrrole-1-carbonyl]-butyl}-4-morpholin-4-yl-benzamide;
N-{1-[4-(1-Acetyl-piperidine-2-carbonyl)-6-oxo-hexahydro-pyrrolo[3,2-b]pyrrole-1-carbonyl]-3-methyl-butyl}-4-morpholin-4-yl-benzamide;
N-{3-methyl-1-[6-oxo-4-(tetrahydro-furan-2-carbonyl)-hexahydro-pyrrolo[3,2-b]pyrrole-1-carbonyl]-butyl}-4-morpholin-4-yl-benzamide;
N-{1-[4-(1-Amino-cyclobutanecarbonyl)-6-oxo-hexahydro-pyrrolo[3,2-b]pyrrole-1-carbonyl]-3-methyl-butyl}-4-morpholin-4-yl-bezamide;
N-{1-[4-(1-Amino-cyclopentanecarbonyl)-6-oxo-hexahydro-pyrrolo[3,2-b]pyrrole-1-carbonyl]-3-methyl-butyl}-4-morpholin-4-yl-benzamide;
N-{1-[4-(1-Amino-cyclohexanecarbonyl)-6-oxo-hexahydro-pymolo[3,2-b]pyrrole-1-carbonyl]-3-methyl-butyl}-4-morpholin-4-yl-benzamide;
N-{1-[4-(1-Acetylamino-cyclobutanecarbonyl)-6-oxo-hexahydro-pyrrolo[3,2-b]pyrrole-1-carbonyl]-3-methyl-butyl}-4-morpholin-4-yl-benzamide;
N-{1-[4-(1-Acetylamino-cyclopentanecarbonyl)-6-oxo-hexahydro-pyrrolo[3,2-b]pyrrole-1-carbonyl]-3-methyl-butyl}-4-morpholin-4-yl-benzamide;
N-{1-[4-(1-Acetylamino-cyclohexanecarbonyl)-6-oxo-hexahydro-pyrrolo[3,2-b]pyrrole-1-carbonyl]-3-methyl-butyl}-4-morpholin-4-yl-benzamide;
N-{1-[4-(1-hydroxy-cyclobutanecarbonyl)-6-oxo-hexahydro-pyrrolo[3,2-b]pyrrole-1-carbonyl]-3-methyl-butyl}-4-morpholin-4-yl-benzamide;
N-{1-[4-(1-hydroxy-cyclopentariecarbonyl)-6-oxo-hexahydro-pyrrolo[3,2-b]pyrrole-1-carbonyl]-3-methyl-butyl}-4-morpholin-4-yl-benzamide;
N-{1-[4-(1-hydroxy-cyclohexanecarbonyl)-6-oxo-hexahydro-pyrrolo[3,2-b]pyrrole-1-carbonyl]-3-methyl-butyl}-4-morpholin-4-yl-benzamide;
N-{1-[4-(1-methoxy-cyclobutanecarbonyl)-6-oxo-hexahydro-pyrrolo[3,2-b]pyrrole-1-carbonyl]-3-methyl-butyl}-4-morpholin-4-yl-benzamide;
N-{1-[4-(1-methoxy-yclopentanecarbonyl)-6-oxo-hexahydro-pyrrolo[3,2-b]pyrrole-1-carbonyl]-3-methyl-butyl}-4-morpholin-4-yl-benzamide;
N-{1-[4-(1-methoxy-cyclohexanecarbonyl)-6-oxo-hexahydro-pyrrolo[3,2-b]pyrrole-1-carbonyl]-3-methyl-butyl}-4-morpholin-4-yl-benzamide;
N-{1-[4-(2-Amino-2-cyclopentyl-acetyl)-6-oxo-hexahydro-pyrrolo[3,2-b]pyrrole-1-carbonyl]-3-methyl-butyl}-4-morpholin-4-yl-benzamide;
N-{1-[4-(2-Amino-2-cyclohexyl-acetyl)-6-oxo-hexahydro-pyrrolo[3,2-b]pyrrole-1-carbonyl]-3-methyl-butyl}-4-morpholin-4-yl-benzamide;
N-{1-[4-(2-Acetylamino-2-cyclopentyl-acetyl)-6-oxo-hexahydro-pyrrolo[3,2-b]pyrrole-1-carbonyl]-3-methyl-butyl}-4-morpholin-4-yl-benzamide;
N-{1-[4-(2-Acetylamino-2-cyclohexyl-acetyl)-6-oxo-hexahydro-pyrrolo[3,2-b]pyrrole-1-carbonyl]-3-methyl-butyl}-4-morpholin-4-yl-benzamide;
N-{1-[4-(2-Acetylamino-propionyl)-6-oxo-hexahydro-pyrrolo[3,2-b]pyrrole-1-carbonyl]-3-methyl-butyl}-4-morpholin-4-yl-benzamide;
N-{1-[4-(2-Acetylamino-acetyl)-6-oxo-hexahydro-pyrrolo[3,2-b]pyrrole-1-carbonyl]-3-methyl-butyl}-4-morpholin-4-yl-benzamide;
N-(1-{4-[2-(Acetyl-methyl-amino)-acetyl]-6-oxo-hexahydro-pyrrolo[3,2-b]pyrrole-1-carbonyl}-3-methyl-butyl)-4-morpholin-4-yl-benzamide;
N-{1-[4-(2-dimethylamino-acetyl)-6-oxo-hexahydro-pyrrolo[3,2-b]pyrrole-1-carbonyl]-3-methyl-butyl}-4-morpholin-4-yl-benzamide;
N-{1-[4-(2-Amino-butyryl)-6-oxo-hexahydro-pyrrolo[3,2-b]pyrrole-1-carbonyl]-3-methyl-butyl}-4-morpholin-4-yl-benzamide;
N-{1-[4-(2-Acetylamino-butyryl)-6-oxo-hexahydro-pyrrolo[3,2-b]pyrrole-1-carbonyl]-3-methyl-butyl}-4-morpholin-4-yl-benzamide;
N-{1-[4-(2-amino-3-methyl-butyryl)-6-oxo-hexahydro-pyrrolo[3,2-b]pyrrole-1-carbonyl]-3-methyl-butyl}-4-morpholin-4-yl-benzamide;
N-{1-[4-(2-Acetylamino-3-methyl-butyryl)-6-oxo-hexahydro-pyrrolo[3,2-b]pyrrole-1-carbonyl]-3-methyl-butyl}-4-morpholin-4-yl-benzamide;
N-{1-[4-(2-amino-3,3-dimethyl-butyryl)-6-oxo-hexhydro-pyrrolo[3,2-b]pyrrole-1-carbonyl]-3-methyl-butyl}-4-morpholin-4-yl-benzamide;
N-{1-[4-(2-Acetylamino-3,3-dimethyl-butyryl)-6-oxo-hexahydro-pyrrolo[3,2-b]pyrrole-1-carbonyl]-3-methyl-butyl}-4-morpholin-4-yl-benzamide;
N-{1-[4-(2-Amino-3-methyl-pentanoyl)-6-oxo-hexahydro-pyrrolo[3,2-b]pyrrole-1-carbonyl]-3-methyl-butyl}-4-morpholin-4-yl-benzamide;
N-{1-[4-(2-Acetylamino-3-methyl-pentanoyl)-6-oxo-hexahydro-pyrrolo[3,2-b]pyrrole-1-carbonyl]-3-methyl-butyl}-4-morpholin-4-yl-benzamide;
N-[3-methyl-1-(6-oxo-4-pentanoyl-hexahydro-pyrrolo[3,2-b]pyrrole-1-carbonyl)-butyl]-4-morpholin-4-yl-benzamide;
N-{3-methyl-1-[4-(3-methyl-pentanoyl)-6-oxo-hexahydro-pyrrolo[3,2-b]pyrrole-1-carbonyl]-butyl}-4-morpholin-4-yl-benzamide;
N-{3-methyl-1-[4-(4-methyl-pentanoyl)-6-oxo-hexahydro-pyrrolo[3,2-b]pyrrole-1-carbonyl]-butyl}-4-morpholin-4-yl-benzamide;
N-{1-[4-(2-Amino-4-methyl-pentanoyl)-6-oxo-hexahydro-pyrrolo[3,2-b]pyrrole-1-carbonyl]-3-methyl-butyl}-4-morpholin-4-yl-benzamide;
N-{1-[4-(2-Acetylamino-4-methyl-pentanoyl)-6-oxo-hexahydro-pyrrolo[3,2-b]pyrrole-1-carbonyl]-3-methyl-butyl}-4-morpholin-4-yl-benzamide;
N-{1-[4-(2-Amino-4,4-dimethyl-pentanoyl)-6-oxo-hexahydro-pyrrolo[3,2-b]pyrrole-1-carbonyl]-3-methyl-butyl}-4-morpholin-4-yl-benzamide;
N-{1-[4-(2-Acetylamino-4,4-dimethyl-pentanoyl)-6-oxo-hexahydro-pyrrolo[3,2-b]pyrrole-1-carbonyl]-3-methyl-butyl}-4-morpholin-4-yl-benzamide;
N-{1-[4-(2-Amino-pentanoyl)-6-oxo-hexahydro-pyrrolo[3,2-b]pyrrole-1-carbonyl]-3-methyl-butyl}-4-morpholin-4-yl-benzamide;
N-{1-[4-(2-Acetylamino-pentanoyl)-6-oxo-hexahydro-pyrrolo[3,2-b]pyrrole-1-carbonyl]-3-methyl-butyl}-4-morpholin-4-yl-benzamide;
N-{1-[4-(2-Amino-5-methyl-hexanoyl)-6-oxo-hexahydro-pyrrolo[3,2-b]pyrrole-1-carbonyl]-3-methyl-butyl}-4-morpholin-4-yl-benzamide;
N-{1-[4-(2-Acetylamino-5-methyl-hexanoyl)-6-oxo-hexahydro-pyrrolo[3,2-b]pyrrole-1-carbonyl]-3-methyl-butyl}-4-morpholin-4-yl-benzamide;
N-{1-[4-(2-hydroxy-3-methyl-butyryl)-6-oxo-hexahydro-pyrrolo[3,2-b]pyrrole-1-carbonyl]-3-methyl-butyl}-4-morpholin-4-yl-benzamide;
N-{1-[4-(2-methoxy-3-methyl-butyryl)-6-oxo-hexahydro-pyrrolo[3,2-b]pyrrole-1-carbonyl]-3-methyl-butyl}-4-morpholin-4-yl-benzamide;
N-{1-[4-(2-hydroxy-3,3-dimethyl-butryl)-6-oxo-hexahydro-pyrrolo[3,2-b]pyrrole-1-carbonyl]-3-methyl-butyl}-4-morpholin-4-yl-benzamide;
N-{1-[4-(2-methoxy-3,3-dimethyl-butyryl)-6-oxo-hexahydro-pyrrolo[3,2-b]pyrrole-1-carbonyl]-3-methyl-butyl}-4-morpholin-4-yl-benzamide;
N-{1-[4-(2-hydroxy-3-methyl-pentanoyl)-6-oxo-hexahydro-pyrrolo[3,2-b]pyrrole-1-carbonyl]-3-methyl-butyl}-4-morpholin-4-yl-benzmide;
N-{1-[4-(2-methoxy-3-methyl-pentanoyl)-6-oxo-hexahydro-pyrrolo[3,2-b]pyrrole-1-carbonyl]-3-methyl-butyl}-4-morpholin-4-yl-benzamide;
N-{1-[4-(2-hydroxy-4-methyl-pentanoyl)-6-oxo-hexahydro-pyrrolo[1,2-b]pyrrole-1-carbonyl]-3-methyl-butyl}-4-morpholin-4-yl-benzamide;
N-{1-[4-(2-methoxy-4-methyl-pentanoyl)-6-oxo-hexahydro-pyrrolo[3,2-b]pyrrole-1-carbonyl]-3-methyl-butyl}-4-morpholin-4-yl-benzamide;
N-{1-[4-(2-hydroxy-4,4-dimethyl-pentanoyl)-6-oxo-hexahydro-pyrrolo[3,2-b]pyrrole-1-carbonyl]-3-methyl-butyl}-4-morpholin-4-yl-benzamide;
N-{1-[4-(2-methoxy-4,4-dimethyl-pentanoyl)-6-oxo-hexahydro-pyrrolo[3,2-b]pyrrole-1-carbonyl]-3-methyl-butyl}-4-morpholin-4-yl-benzamide;
N-{1-[4-(2-hydroxy-pentanoyl)-6-oxo-hexahydro-pyrrolo[3,2-b]pyrrole-1-carbonyl]-3-methyl-butyl}-4-morpholin-4-yl-benzamide;
N-{1-[4-(2-methoxy-pentanoyl)-6-oxo-hexahydro-pyrrolo[3,2-b]pyrrole-1-carbonyl]-3-methyl-butyl}-4-morpholin-4-yl-benzamide;
N-{1-[4-(2-hydroxy-5-methyl-hexanoyl)-6-oxo-hexahydro-pyrrolo[3,2-b]pyrrole-1-carbonyl]-3-methyl-butyl}-4-morpholin-4-yl-benzamide;
N-{1-[4-(2-methoxy-5-methyl-hexanoyl)-6-oxo-hexahyro-pyrrolo[3,2-b]pyrrole-1-carbonyl]-3-methyl-butyl}-4-morpholin-4-yl-benzamide;
N-[1-(4-Benzoyl-6-oxo-hexahydro-pyrrolo[3,2-b]pyrrole-1-carbonyl)-3-methyl-butyl]-4-piperazin-1-ylbenzamide;
N-{3-methyl-1-[6-oxo-4-(pyridine-2-carbonyl)-hexahydro-pyrrolo[3,2-b]pyrrole-1-carbonyl]-butyl}-4-piperazin-1-yl-benzamide;
N-{3-methyl-1-[6-oxo-4-(1-oxy-pyridine-2-carbonyl)-hexahydro-pyrrolo[3,2-b]pyrrole-1-carbonyl]-butyl}-4-piperazin-1-yl-benzamide;
N-{3-methyl-1-[6-oxo-4-(pyridine-3-carbonyl)-hexahydro-pyrrolo[3,2-b]pyrrole-1-carbonyl]-butyl}-4-piperazin-1-yl-benzamide;
N-{3-methyl-1-[6-oxo-4-(1-oxy-pyridine-3-carbonyl)-hexahydro-pyrrolo[3,2-b]pyrrole-1-carbonyl]-butyl}-4-piperazin-1-yl-benzamide;
N-{3-methyl-1-[6-oxo-4-(pyridine-4-carbonyl)-hexahydro-pyrrolo[3,2-b]pyrrole-1-carbonyl]-butyl}-4-piperazin-1-yl-benzamide;
N-{3-methyl-1-[6-oxo-4-(1-oxy-pyridine-4-carbonyl)-hexahydro-pyrrolo[3,2-b]pyrrole-1-carbonyl]-butyl}-4-piperazin-1-yl-benzamide;
N-[1-(4-Benzenesulfonyl-6-oxo-hexahydro-pyrrolo[3,2-b]pyrrole-1-carbonyl)-3-methyl-butyl]-4-piperazin-1-yl-benzamide;
N-{3-methyl-1-[6-oxo-4-(pyridine-2-sulfonyl)-hexahydro-pyrrolo[3,2-b]pyrrole-1-carbonyl]-butyl}-4-piperazin-1-yl-benzamide;
N-{3-methyl-1-[6-oxo-4-(1-oxy-pyridine-2-sulfonyl)-hexahydro-pyrrolo[3,2-b]pyrrole-1-carbonyl]-butyl}-4-piperazin-1-yl-benzamide;
N-{3-methyl-1-[6-oxo-4-(pyridine-3-sulfonyl)-hexahydro-pyrrolo[3,2-b]pyrrole-1-carbonyl]-butyl}-4-piperazin-1-yl-benzamide;
N-{3-methyl-1-[6-oxo-4-(1-oxy-pyridine-3-sulfonyl)-hexahydro-pyrrolo[3,2-b]pyrrole-1-carbonyl]-butyl}-4-piperazin-1-yl-benzamide;
N-3-methyl-1-[6-oxo-4-(pyridine-4-sulfonyl)-hexahydro-pyrrolo[3,2-b]pyrrole-1-carbonyl]-butyl}-4-piperazin-1-yl-benzamide;
N-{3-methyl-1-[6-oxo-4-(1-oxy-pyridine-4-sulfonyl)-hexhydro-pyrrolo[3,2-b]pyrrole-1-carbonyl]-butyl}-4-piperazzin-1-yl-benzamide;
N-[3-methyl-1-(6-oxo-4-phenylacetyl-hexahydro-pyrrolo[3,2-b]pyrrole-1-carbonyl)-butyl]-4-piperazin-1-yl-benzamide;
4-[2-(4-piperazin-1-yl-benzoylamino)-4-methyl-pentanoyl]-3-oxo-hexahydro-pyrrolo[3,2-b]pyrrole-1-carboxylic acid phenylamide;
4-[2-(4-piperazin-1-yl-benzoylamino)-4-methyl-pentanoyl]-3-oxo-hexahydro-pyrrolo[3,2-b]pyrrole-1-carboxylic acid isobutyl-amide;
4-[2-(4-piperazin-1-yl-benzoylamino)-4-methyl-pentanoyl]-3-oxo-hexahydro-pyrrolo[3,2-b]pyrrole-1-carboxylic acid phenyl ester;
4-[2-(4-piperazin-1-yl-benzoylamino)-4-methyl-pentanoyl]-3-oxo-hexahydro-pyuolo[3,2-b]pyrrole-1-carboxylic acid isobutyl ester;
N-{3-methyl-1-[6-oxo-4-(pyrrolidine-1-carbonyl)-hexahydro-pyrrolo[3,2-b]pyrrole-1-carbonyl]-butyl}-4-piperazin-1 -yl-benzamide;
N-{3-methyl-1-[6-oxo-4-(piperidine-1-carbonyl)-hexahydro-pyrrolo[3,2-b]pyrrole-1-carbonyl]-butyl}-4-piperazin-1-yl-benzmide;
N-{3-methyl-1-[4-(morpholine-4-carbonyl)-6-oxo-hexahydro-pyrrolo[3,2-b]pyrrole-1-carbonyl]-butyl}-4-piperazin-1-yl-benzamide;
N-{3-methyl-1-[6-oxo-4-(piperazine-1-carbonyl)-hexahydro-pyrrolo[3,2-b]pyrrole-1-carbonyl]-butyl}-4-piperazin-1-yl-benzamide;
N-{3-methyl-1-[4-(4-methyl-piperazine-1-carbonyl)-6-oxo-hexahydro-pyrrolo[3,2-b]pyrrole-1-parbonyl]-butyl}-4-piperazin-1-yl-benzamide;
N-(3-methyl-1-{4-[2-(4-methyl-piperazin-1-yl)-acetyl]-6-oxo-hexahydro-pyrrolo[3,2-b]pyrrole-1-carbonyl}-butyl)-4-piperazin-1-yl-benzamide;
N-{3-methyl-1-[6-oxo-4-(2-piperazin-1-yl-acetyl)-hexahydro-pyrrolo[3,2-b]pyrrole-1-carbonyl]-butyl}-4-piperazin-1-yl-benzamide;

N-{1-[4-(furan-2-carbonyl)-6-oxo-hexahydro-pyrrolo[3,2-b]pyrrole-1-carbonyl]-3-methyl-butyl}-4-piperazin-1-yl-benzamide;
N-{1-[4-(5-chloro-furan-2-carbonyl)-6-oxo-hexahydro-pyrrolo[3,2-b]pyrrole-1-carbonyl]-3-methyl-butyl}-4-piperazin-1-yl-benzamide;
N-{1-[4-(thiophene-2-carbonyl)-6-oxo-hexahydro-pyrrolo[3,2-b]pyrrole-1-carbonyl]-3-methyl-butyl}-4-piperazin-1-yl-benzamide;
N-{1-[4-(5-chloro-thiophene-2-carbonyl)-6-oxo-hexahydro-pyrrolo[3,2-b]pyrrole-1-carbonyl]-3-methyl-butyl}-4-piperazin-1-yl-benzamide;
N-{1-[4-(furan-3-carbonyl)-6-oxo-hexahydro-pyrrolo[3,2-b]pyrrole-1-carbonyl]-3-methyl-butyl}-4-piperazin-1-yl-benzamide;
N-{1-[4-(thiophene-3-carbonyl)-6-oxo-hexahydro-pyrrolo[3,2-b]pyrrole-1-carbonyl]-3-methyl-butyl}-4-piperazin-1-yl-benzamide;
N-[1-(4-cyclopentanecarbonyl-6-oxo-hexahydro-pyrrolo[3,2-b]pyrrole-1-carbonyl)-3-methyl-butyl]-4-piperazin-1-yl-benzamide;
N-[1-(4-cyclohexanecarbonyl-6-oxo-hexahydro-pyrrolo[3,2-b]pyrrole-1-carbonyl)-3-methyl-butyl]-4-piperazin-1-yl-benzamide;
N-{1-[4-(cyclopent-3-enecarbonyl)-6-oxo-hexahydro-pyrrolo[3,2-b]pyrrole-1-carbonyl]-3-methyl-butyl}-4-piperazin-1-yl-benzamide;
N-{1-[4-(2-cyclopentyl-acetyl)-6-oxo-hexahydro-pyrrolo[3,2-b]pyrrole-1-carbonyl]-3-methyl-butyl}-4-piperazin-1-yl-benzamide;
N-{1-[4-(2-cyclohexyl-acetyl)-6-oxo-hexahydro-pyrrolo[3,2-b]pyrrole-1-carbonyl]-3-methyl-butyl}-4-piperazin-1-yl-benzamide;
4-piperazin-1-yl-N-{3-methyl-1-[6-oxo-4-(pyrrolidine-2-carbonyl)-hexahydro-pyrrolo[3,2-b]pyrrole-1-carbonyl]-butyl}-benzamide;
N-{1-[4-(1-Acetyl-pyrrolidine-2-carbonyl)-6-oxo-hexahydro-pyrrolo[3,2-b]pyrrole-1-carbonyl]-3-methyl-butyl}-4-pipe razin-1-yl-benzamide;
N-{3-methyl-1-[6-oxo-4-(piperidine-2-carbonyl)-hexahydro-pyrrolo[3,2-b]pyrrole-1-carbonyl]-butyl}-4-piperazin-1-yl-benzamide;
N-{1-[4-(1-Acetyl-piperidine-2-carbonyl)-6-oxo-hexahydro-pyrrolo[3,2-b]pyrrole-1-carbonyl]-3-methyl-butyl}-4-piperazin-1-yl-benzamide;
N-{3-methyl-1-[6-oxo-4-(tetrahydro-furan-2-carbonyl)-hexahydro-pyrrolo[3,2-b]pyrrole-1-carbonyl]-butyl}-4-piperazin-1-yl-benzamide;
N-{1-[4-(1-Amino-cyclobutanecarbonyl)-6-oxo-hexahydro-pyrrolo[3,2-b]pyrrole-1-carbonyl]-3-methyl-butyl}-4-piperazin-1-yl-benzamide;
N-{1-[4-(1-Amino-cyclopentanecarbonyl)-6-oxo-hexahydro-pyrrolo[3,2-b]pyrrole-1-carbonyl]-3-methyl-butyl}-4-piperazin-1-yl-benzamide;
N-{1-[4-(1-Amino-cyclohexanecarbonyl)-6-oxo-hexahydro-pyrrolo[3,2-b]pyrrole-1-carbonyl]-3-methyl-butyl}-4-piperazin-1-yl-benzamide;
N-{1-[4-(1-Acetylamino-cyclobutanecarbonyl)-6-oxo-hexahydro-pyrrolo[3,2-b]pyrrole-1-carbonyl]-3-methyl-butyl}-4-piperazin-1-yl-benzamide;
N-{1-[4-(1-Acetylamino-cyclopentanecarbonyl)-6-oxo-hexahydro-pyrrolo[3,2-b]pyrrole-1-carbonyl]-3-methyl-butyl}-4-piperazin-1-yl-benzamide;
N-{1-[4-(1-Acetylamino-cyclohexanecarbonyl)-6-oxo-hexahydro-pyrrolo[3,2-b]pyrrole-1-carbonyl]-3-methyl-butyl}-4-piperazin-1-yl-benzamide;
N-{1-[4-(1-hydroxy-cyclobutanecarbonyl)-6-oxo-hexahydro-pyrrolo[3,2-b]pyrrole-1-carbonyl]-3-methyl-butyl}-4-piperazin-1-yl-benzamide;
N-{1-[4-(1-hydroxy-cyclopentanecarbonyl)-6-oxo-hexahydro-pyrrolo[3,2-b]pyrrole-1-carbonyl]-3-methyl-butyl}-4-piperazin-1-yl-benzamide;
N-{1-[4-(1-hydroxy-cyclohexanecarbonyl)-6-oxo-hexahydro-pyrrolo[3,2-b]pyrrole-1-carbonyl]-3-methyl-butyl}-4-piperazin-1-yl-benzamide;
N-{1-[4-(1-methoxy-cyclobutanecarbonyl)-6-oxo-hexahydro-pyrrolo[3,2-b]pyrrole-1-carbonyl]-3-methyl-butyl}-4-piperazin-1-yl-benzamide;
N-{1-[4-(1-methoxy-cyclopentanecarbonyl)-6-oxo-hexahydro-pyrrolo[3,2-b]pyrrole-1-carbonyl]-3-methyl-butyl}-4-piperazin-1-yl-benzamide;
N-{1-[4-(1-methoxy-cyclohexanecarbonyl)-6-oxo-hexahydro-pyrrolo[3,2-b]pyrrole-1-carbonyl]-3-methyl-butyl}-4-piperazin-1-yl-benzamide;
N-{1-[4-(2-Amino-2-cyclopentyl-acetyl)-6-oxo-hexahydro-pyrrolo[3,2-b]pyrrole-1-carbonyl]-3-methyl-butyl}-4-piperazin-1-yl-benzamide;

N-{1-[4-(2-Amino-2-cyclohexyl-acetyl)-6-oxo-hexahydro-pyrrolo[3,2-b]pyrrole-1-carbonyl]-3-methyl-butyl}-4-piperazin-1-yl-benzamide;
N-{1-[4-(2-Acetylamino-2-cyclopentyl-acetyl)-6-oxo-hexahydro-pyrrolo[3,2-b]pyrrole-1-carbonyl]-3-methyl-butyl}-4-piperazin-1-yl-benzamide;
N-{1-[4-(2-Acetylamino-2-cyclohexyl-acetyl)-6-oxo-hexahydro-pyrrolo[3,2-b]pyrrole-1-carbonyl]-3-methyl-butyl}-4-piperazin-1-yl-benzamide;
N-{1-[4-(2-Acetylmino-propionyl)-6-oxo-hexahydro-pyrrolo[3,2-b]pyrrole-1-carbonyl]-3-methyl-butyl}-4-piperazin-1-yl-benzamide;
N-{1-[4-(2-Acetylamino-acetyl)-6-oxo-hexahydro-pyrrolo[3,2-b]pyrrole-1-carbonyl]-3-methyl-butyl}-4-piperazin-1-yl-benzamide;
N-(1-{4-[2-(Acetyl-methylamino)-acetyl]-6-oxo-hexahydro-pyrrolo[3,2-b]pyrrole-1-carbonyl}-3-methyl-butyl)-4-piperazin-1-yl-benzamide;
N-{1-[4-(2-dimethylamino-acetyl)-6-oxo-hexahydro-pyrrolo[3,2-b]pyrrole-1-carbonyl]-3-methyl-butyl}-4-piperazin-1-yl-benzamide;
N-{1-[4-(2-Amino-butyryl)-6-oxo-hexahydro-pyrrolo[3,2-b]pyrrole-1-carbonyl]-3-methyl-butyl}-4-piperazin-1-yl-benzamide;
N-{1-[4-(2-Acetylamino-butyryl)-6-oxo-hexahydro-pyrrolo[3,2-b]pyrrole-1-carbonyl]-3-methyl-butyl}-4-piperazin-1-yl-benzamide;
N-{1-[4-(2-amino-3-methyl-butyryl)-6-oxo-hexahydro-pyrrolo[3,2-b]pyrrole-1-carbonyl]-3-methyl-butyl}-4-piperazin-1-yl-benzamide;
N-{1-[4-(2-Acetylamino-3-methyl-butyryl)-6-oxo-hexahydro-pyrrolo[3,2-b]pyrrole-1-carbonyl]-3-methyl-butyl}-4-piperazin-1-yl-benzamide;
N-{1-[4-(2-amino-3,3-dimethyl-butyryl)-6-oxo-hexahydro-pyrrolo[3,2-b]pyrrole-1-carbonyl]-3-methyl-butyl}-4-piperazin-1-yl-benzamide;
N-{1-[4-(2-Acetylamino-3,3-dimethyl-butyryl)-6-oxo-hexahydro-pyrrolo[3,2-b]pyrrole-1-carbonyl]-3-methyl-butyl}-4-piperazin-1-yl-benzamide;
N-{1-[4-(2-Amino-3-methyl-pentanoyl)-6-oxo-hexahydro-pyrrolo[3,2-b]pyrrole-1-carbonyl]-3-methyl-butyl}-4-piperazin-1-yl-benzamide;
N-{1-[4-(2-Acetylamino-3-methyl-pentanoyl)-6-oxo-hexahydro-pyrrolo[3,2-b]pyrrole-1-carbonyl]-3-methyl-butyl}-4-piperazin-1-yl-benzamide;
N-[3-methyl-1-(6-oxo-4-pentanoyl-hexahydro-pyrrolo[3,2-b]pyrrole-1-carbonyl)-butyl]-4-piperein-1-yl-benzamide;
N-{3-methyl-1-[4-(3-methyl-pentanoyl)-6-oxo-hexahydro-pyrrolo[3,2-b]pyrrole-1-carbonyl]-butyl}-4-piperazin-1-yl-benzamide;
N-{3-methyl-1-[4-(4-methyl-pentanoyl)-6-oxo-hexahydro-pyrrolo[3,2-b]pyrrole-1-carbonyl]-butyl}-4-piperazin-1-yl-benzamide;
N-{1-[4-(2-Amino-4-methyl-pentanoyl)-6-oxo-hexahydro-pyrrolo[3,2-b]pyrrole-1-carbonyl]-3-methyl-butyl}-4-piperazin-1-yl-benzamide;
N-{1-[4-(2-Acetylamino-4-methyl-pentanoyl)-6-oxo-hexahydro-pyrrolo[3,2-b]pyrrole-1-carbonyl]-3-methyl-butyl}-4-piperein-1-yl-benzamide;
N-{1-[4-(2-Amino-4,4-dimethyl-pentanoyl)-6-oxo-hexahydro-pyrrolo[3,2-b]pyrrole-1-carbonyl]-3-methyl-butyl}-4-piperazin-1-yl-benzamide;
N-{1-[4-(2-Acetylamino-4,4-dimethyl-pentanoyl)-6-oxo-hexahydro-pyrrolo[3,2-b]pyrrole-1-carbonyl]-3-methyl-butyl}-4-piperazin-1-yl-benzamide;
N-{1-[4-(2-Amino-pentanoyl)-6-oxo-hexahydro-pyrrolo[3,2-b]pyrrole-1-carbonyl]-3-methyl-butyl}-4-piperazin-1-yl-benzamide;
N-{1-[4-(2-Acetylamino-pentanoyl)-6-oxo-hexahydro-pyrrolo[3,2-b]pyrrole-1-carbonyl]-3-methyl-butyl}-4-piperazin-1-yl-benzamide;
N-{1-[4-(2-Amino-5-methyl-hexanoyl)-6-oxo-hexahydro-pyrrolo[3,2-b]pyrrole-1-carbonyl]-3-methyl-butyl}-4-piperazin-1-yl-benzamide;
N-{1-[4-(2-Acetylamino-5-methyl-hexanoyl)-6-oxo-hexahydro-pyrrolo[3,2-b]pyrrole-1-carbonyl]-3-methyl-butyl}-4-piperazin-1-yl-benzamide;
N-{1-[4-(2-hydroxy-3-methyl-butyryl)-6-oxo-hexahydro-pyrrolo[3,2-b]pyrrole-1-carbonyl]-3-methyl-butyl}-4-piperazin-1-yl-benzamide;
N-{1-[4-(2-methoxy-3-methyl-butyryl)-6-oxo-hexahydro-pyrrolo[3,2-b]pyrrole-1-carbonyl]-3-methyl-butyl}-4-piperazin-1-yl-benzamide;
N-{1-[4-(2-hydroxy-3,3-dimethyl-butyryl)-6-oxo-hexahydro-pyrrolo[3,2-b]pyrrole-1-carbonyl]-3-methyl-butyl}-4-piperazin-1-yl-benzamide;
N-{1-[4-(2-methoxy-3,3-dimethyl-butyryl)-6-oxo-hexahydro-pyrrolo[3,2-b]pyrrole-1-carbonyl]-3-methyl-butyl}-4-piperazin-1-yl-benzamide;
N-{1-[4-(2-hydroxy-3-methyl-pentanoyl)-6-oxo-hexahydro-pyrrolo[3,2-b]pyrrole-1-carbonyl]-3-methyl-butyl}-4-piperazin-1-yl-benzamide;
N-{1-[4-(2-methoxy-3-methyl-pentanoyl)-6-oxo-hexahydro-pyrrolo[3,2-b]pyrrole-1-carbonyl]-3-methyl-butyl}-4-piperazin-1-yl-benzamide;
N-{1-[4-(2-hydroxy-4-methyl-pentanoyl)-6-oxo-hexahydro-pyrrolo[3,2-b]pyrrole-1-carbonyl]-3-methyl-butyl}-4-piperazin-1-yl-benzamide;
N-{1-[4-(2-methoxy-4-methyl-pentanoyl)-6-oxo-hexahydro-pyrrolo[3,2-b]pyrrole-1-carbonyl]-3-methyl-butyl}-4-piperazin-1-yl-benzamide;
N-{-[4-(2-hydroxy-4,4-dimethyl-pentanoyl)-6-oxo-hexahydro-pyrrolo[3,2-b]pyrrole-1-carbonyl]-3-methyl-butyl}-4-piperazin-1-yl-benzamide;
N-{1-[4-(2-methoxy-4,4-dimethyl-pentanoyl)-6-oxo-hexahydro-pyrrolo[3,2-b]pyrrole-1-carbonyl]-3-methyl-butyl}-4-piperazin-1-yl-benzamide;
N-{1-[4-(2-hydroxy-pentanoyl)-6-oxo-hexahydro-pyrrolo[3,2-b]pyrrole-1-carbonyl]-3-methyl-butyl}-4-piperazin-1-yl-benzamide;
N-{1-[4-(2-methoxy-pentanoyl)-6-oxo-hexahydro-pyrrolo[3,2-b]pyrrole-1-carbonyl]-3-methyl-butyl}-4-piperazin-1-yl-benzamide;
N-{1-[4-(2-hydroxy-5-methyl-hexanoyl)-6-oxo-hexahydro-pyrrolo[3,2-b]pyrrole-1-carbonyl]-3-methyl-butyl}-4-piperazin-1-yl-benzamide;
N-{1-[4-(2-methoxy-5-methyl-hexanoyl)-6-oxo-hexahydro-pyrrolo[3,2-b]pyrrole-1-carbonyl]-3-methyl-butyl}-4-piperazin-1-yl-benzamide;
N-[1-(4-Benzoyl-6-oxo-hexahydro-pyrrolo[3,2-b]pyrrole-1-carbonyl)-3-methyl-butyl]-4-(4-methyl-piperazin-1-yl)benzamide;
N-{3-methyl-1-[6-oxo-4-(1-oxy-pyridine-2-carbonyl)-hexahydro-pyrrolo[3,2-b]pyrrole-1-carbonyl]-butyl}-4-(4-methyl-piperazin-1-yl)-benzamide;
N-{3-methyl-1-[6-oxo-4-(pyridine-3-carbonyl)-hexahydro-pyrrolo[3,2-b]pyrrole-1-carbonyl]-butyl}-4-(4-methyl-piperazin-1-yl)-benzamide;
N-{3-methyl-1-[6-oxo-4-(1-oxy-pyridine-3-carbonyl)-hexahydro-pyrrolo[3,2-b]pyrrole-1-carbonyl]-butyl}-4-(4-methyl-piperazin-1-yl)-benzamide;
N-{3-methyl-1-[6-oxo-4-(pyridine-4-carbonyl)-hexahydro-pyrrolo[3,2-b]pyrrole-1-carbonyl]-butyl}-4-(4-methyl-piperazin-1-yl)-benzamide;
N-{3-methyl-1-[6-oxo-4-(1-oxy-pyridine-4-carbonyl)-hexahydro-pyrrolo[3,2-b]pyrrole-1-carbonyl]-butyl}-4-(4-methyl-piperazin-1-yl)-benzamide;
N-[1-(4-Benzenesulfonyl-6-oxo-hexahydro-pyrrolo[3,2-b]pyrrole-1-carbonyl)-3-methyl-butyl]-4-(4-methyl-piperazin-1-yl)-benzamide;
N-{3-methyl-1-[6-oxo-4-(pyridine-4-sulfonyl)-hexahydro-pyrrolo[3,2-b]pyrrole-1-carbonyl]-butyl}-4-(4-methyl-piperazin-1-yl)-benzamide;
N-{3-methyl-1-[6-oxo-4-(1-oxy-pyridine-4-sulfonyl)-hexahydro-pyrrolo[3,2-b]pyrrole-1-carbonyl]-butyl}-4-(4-methyl-piperain-1-yl)-benzamide;
N-[3-methyl-1-(6-oxo-4-phenylacetyl-hexahydro-pyrrolo[3,2-b]pyrrole-1-carbonyl)-butyl]-4-(4-methyl-piperazin-1-yl)-benzamide;
4-[2-(4-(4-methyl-piperazin-1-yl)-benzoylamino)-4-methyl-pentanoyl]-3-oxo-hexahydro-pyrrolo[3,2-b]pyrrole-1-carboxylic acid phenylamide;
4-[2-(4-(4-methyl-piperazin-1-yl)-benzoylamino)-4-methyl-pentanoyl]-3-oxo-hexahydro-pyrrolo[3,2-b]pyrrole-1-carboxylic acid isobutyl-amide;
4-[2-(4-(4-methyl-piperazin-1-yl)-benzoylamino)-4-methyl-pentanoyl]-3-oxo-hexahydro-pyrrolo[3,2-b]pyrrole-1-carboxylic acid phenyl ester;
4-[2-(4-(4-methyl-piperazin-1-yl)-benzoylamino)-4-methyl-pentanoyl]-3-oxo-hexahydro-pyrrolo[3,2-b]pyrrole-1-carboxylic acid isobutyl ester;
N-{3-methyl-1-[6-oxo-4-(pyrrolidine-1-carbonyl)-hexahydro-pyrrolo[3,2-b]pyrrole-1-carbonyl]-butyl}-4-(4-methyl-piperazin-1-yl)-benzamide;
N-{3-methyl-1-[6-oxo-4-(piperidine-1-carbonyl)-hexahydro-pyrrolo[3,2-b]pyrrole-1-carbonyl]-butyl}-4-(4-methyl-piperazin-1-yl)-benzamide;
N-{3-methyl-1-[4-(morpholine-4-carbonyl)-6-oxo-hexahydro-pyrrolo[3,2-b]pyrrole-1-carbonyl]-butyl}-4-(4-methyl-piperazin-1-yl)-benzamide;
N-{3-methyl-1-[6-oxo-4-(piperazine-1-carbonyl)-hexahydro-pyrrolo[3,2-b]pyrrole-1-carbonyl]-butyl}-4-(4-methyl-piperazin-1-yl)-benzamide;
N-{3-methyl-1-[4-(4-methyl-piperazine-1-carbonyl)-6-oxo-hexahydro-pyrrolo[3,2-b]pyrrole-1-carbonyl]-butyl}-4-(4-methyl-piperazin-1-yl)-benzamide;
N-(3-methyl-1-{4-[2-(4-methyl-piperazin-1-yl)-acetyl]-6-oxo-hexahydro-pyrrolo[3,2-b]pyrrole-1-carbonyl}-butyl)-4-(4-methyl-piperazin-1-yl)-benzamide;
N-{3-methyl-1-[6-oxo-4-(2-piperazin-1-yl-acetyl)-hexahydro-pyrrolo[3,2-b]pyrrole-1-carbonyl]-butyl}-4-(4-methyl-piperein-1-yl)-benzamide;
N-{1-[4-(furan-2-carbonyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(5-chloro-furan-2-carbonyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(thiophene-2-carbonyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(5-chloro-thiophene-2-carbonyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(furan-3-carbonyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(thiophene-3-carbonyl)-6-oxo-hexahydro-pyrrolo[3,2-b]pyrrole-1-carbonyl]-3-methyl-butyl}-4-(4-methyl-piperazin-1-yl)-benzamide;
N-[1-(4-cyclopentanecarbonyl-6-oxo-hexahydro-pyrrolo[3,2-b]pyrrole-1-carbonyl)-3-methyl-butyl]-4-(4-methyl-piperazin-1-yl)-benzamide;
N-[1-(4-cyclohexanecarbonyl-6-oxo-hexahydro-pyrrolo[3,2-b]pyrrole-1-carbonyl)-3-methyl-butyl]-4-(4-methyl-piperazin-1-yl)-benzamide;
N-{1-[4-(cyclopent-3-enecarbonyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-cyclopentyl-acetyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-cyclohexyl-acetyl)-6-oxo-hexahydro-pyrrolo[3,2-b]pyrrole-1-carbonyl]-3-methyl-butyl}-4-(4-methyl-piperazin-1-yl)-benzamide;
4-(4-methyl-piperazk-1-yl)-N-{3-methyl-1-[6-oxo-4-(pyrrolidine-2-carbonyl)-hexahydro-pyrrolo[3,2-b]pyrrole-1-carbonyl]-butyl}-benzamide;
N-{1-[4-(1-Acetyl-pyrrolidine-2-carbonyl)-6-oxo-hexahydro-pyrrolo[3,2-b]pyrrole-1-carbonyl]-3-methyl-butyl}-4-(4-methyl-piperazin-1-yl)-benzamide;
N-{3-methyl-1-[6-oxo-4-(piperidine-2-carbonyl)-hexahydro-pyrrolo[3,2-b]pyrrole-1-carbonyl]-butyl}-4-(4-methyl-piperazin-1-yl)-benzmide;
N-{1-[4-(1-Acetyl-piperidine-2-carbonyl)-6-oxo-hexahydro-pyrrolo[3,2-b]pyrrole-1-carbonyl]-3-methyl-butyl}-4-(4-methyl-piperazin-1-yl)-benzamide;
N-{3-methyl-1-[6-oxo-4-(tetrahydro-furan-2-carbonyl)-hexahydro-pyrrolo[3,2-b]pyrrole-1-carbonyl]-butyl}-4-(4-methyl-piperazin-1-yl)-benzamide;
N-{1-[4-(1-Amino-cyclobutanecarbonyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(1-Amino-cyclopentanecarbonyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(1-Amino-cyclohexanecarbonyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(1-Acetylamino-cyclobutanecarbonyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(1-Acetylamino-cyclopentanecarbonyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(1-Acetylamino-cyclohexanecarbonyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(1-hydroxy-cyclobutanecarbonyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(1-hydroxy-cyclopentanecarbonyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(1-hydroxy-cyclohexanecarbonyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(1-methoxy-cyclobutanecarbonyl)-6-oxo-hexahydro-pyrrolo[3,2-b]pyrrole-1-carbonyl]-3-methyl-butyl}-4-(4-methyl-piperein-1-yl)-benzamide;
N-{1-[4-(1-methoxy-cyclopentanecarbonyl)-6-oxo-hexahydro-pyrrolo[3,2-b]pyrrole-1-carbonyl]-3-methyl-butyl}-4-(4-methyl-piperein-1-yl)-benzamide;
N-{1-[4-(1-methoxy-cyclohexanecarbonyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-Amino-2-cyclopentyl-acetyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-Amino-2-cyclohexyl-acetyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-Acetylamino-2-cyclopentyl-acetyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-Acetylamino-2-cyclohexyl-acetyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-Acetylamino-propionyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-Acetylamino-acetyl)-6-oxo-hexahydro-pyrrolo[3,2-b]pyrrole-1-carbonyl]-3-methyl-butyl}-4-(4-methyl-piperazin-1-yl)-benzamide;
N-(1-{4-[2-(Acetyl-methyl-amino)-acetyl]-6-oxo-hexahydro-pyrrolo[3,2-b]pyrrole-1-carbonyl}-3-methyl-butyl)-4-(4-methyl-piperazin-1-yl)-benzamide;
N-{1-[4-(2-dimethylamino-acetyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-Amino-butyryl)-6-oxo-hexahydro-pyrrolo[3,2-b]pyrrole-1-carbonyl]-3-methyl-butyl}-4-(4-methyl-piperein-1-yl)-benzamide;
N-{1-[4-(2-Acetylamino-butyryl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-amino-3-methyl-butyryl)-6-oxo-hexahydro-pyrrolo[3,2-b]pyrrole-1-carbonyl]-3-methyl-butyl}-4-(4-methyl-piperein-1-yl)-benzamide;
N-{1-[4-(2-Acetylamino-3-methyl-butyryl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-amino-3,3-dimethyl-butyryl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-Acetylamino-3,3-dimethyl-butyryl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-Amino-3-methyl-pentanoyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-Acetylamino-3-methyl-pentanoyl)-6-oxo-hexahydro-pyrrolo[3,2-b]pyrrole-1-carbonyl]-3-methyl-butyl}-4-(4-methyl-piperein-1-yl)-benzamide;
N-[3-methyl-1-(6-oxo-4-pentanoyl-hexahydro-pyrrolo[3,2-b]pyrrole-1-carbonyl)-butyl]-4-(4-methyl-piperazin-1-yl)-benzamide;
N-{3-methyl-1-[4-(3-methyl-pentanoyl)-6-oxo-hexahydro-pyrrolo[3,2-b]pyrrole-1-carbonyl]-butyl}-4-(4-methyl-piperazin-1-yl)-benzamide;
N-{3-methyl-1-[4-(4-methyl-pentanoyl)-6-oxo-hexahydro-pyrrolo[3,2-b]pyrrole-1-carbonyl]-butyl}-4-(4-methyl-piperazin-1-yl)-benzamide;
N-{1-[4-(2-Amino-4-methyl-pentanoyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-Acetylamino-4-methyl-pentanoyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-Amino-4,4-dimethyl-pentanoyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-Acetylamino-4,4-dimethyl-pentanoyl)-6-oxo-hexahydro-pyrrolo[3,2-b]pyrrole-1-carbonyl]-3-methyl-butyl}-4-(4-methylpiperazin-1-yl)-benzamide;
N-{1-[4-(2-Amino-pentanoyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-Acetylamino-pentanoyl)-6-oxo-hexahydro-pyrrolo[3,2-b]pyrrole-1-carbonyl]-3-methyl-butyl}-4-(4-methyl-piperazk-1-yl)-benzamide;
N-{1-[4-(2-Amino-5-methyl-hexanoyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-Acetylamino-5-methyl-hexanoyl)-6-oxo-hexahydro-pyrrolo[3,2-b]pyrrole-1-carbonyl]-3-methyl-butyl}-4-( 4-methyl-piperazin-1-yl)-benxamide;
N-{1-[4-(2-hydroxy-3-methyl-butyryl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-methoxy-3-methyl-butyryl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-hydroxy-3,3-dimethyl-butyryl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-methoxy-3,3-dimethyl-butyryl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-hydroxy-3-methyl-pentanoyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-methoxy-3-methyl-pentanoyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-hydroxy-4-methyl-pentanoyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-methoxy-4-methyl-pentanoyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-hydroxy-4,4-dimethyl-pentanoyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-methoxy-4,4-dimethyl-pentanoyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-hydroxy-pentanoyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-methoxy-pentanoyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-hydroxy-5-methyl-hexanoyl)-6-oxo-hexahydro-pyrrolo[3,2-b]pyrrole-1-carbonyl]-3-methyl-butyl}-4-(4-methyl-piperazin-1-yl)-benzamide;
N-{1-[4-(2-methoxy-5-methyl-hexanoyl)-6-oxo-hexahydro-pyrrolo[3,2-b]pyrrole-1-carbonyl]-3-methyl-butyl}-4-(4-methyl-piperazin-1-yl)-benzamide;
N-[1-(4-Benzoyl-6-oxo-hexahydro-pyrrolo[3,2-b]pyrrole-1-carbonyl)-3-methyl-butyl]-4-aminobenzamide;
4-Amino-N-{3-methyl-1-[6-oxo-4-(pyridine-2-carbonyl)-hexahydro-pyrrolo[3,2-b]pyrrole-1-carbonyl]-butyl}-benzamide;
4-Amino-N-{3-methyl-1-[6-oxo-4-(1-oxy-pyridine-2-carbonyl)-hexahydro-pyrrolo[3,2-b]pyrrole-1-carbonyl]-butyl}-benzamide;
4-Amino-N-{3-methyl-1-[6-oxo-4-(pyridine-3-carbonyl)-hexahydro-pyrrolo[3,2-b]pyrrole-1-carbonyl]-butyl}-benzamide;
4-Amino-N-{3-methyl-1-[6-oxo-4-(1-oxy-pyridine-3-carbonyl)-hexahydro-pyrrolo[3,2-b]pyrrole-1-carbonyl]-butyl}-benzamide;
4-Amino-N-{3-methyl-1-[6-oxo-4-(pyridine-4-carbonyl)-hexahydro-pyrrolo[3,2-b]pyrrole-1-carbonyl]-butyl}-benzamide;
4-Amino-N-{3-methyl-1-[6-oxo-4-(1-oxy-pyridine-4-carbonyl)-hexahydro-pyrrolo[3,2-b]pyrrole-1-carbonyl]-butyl}-benzamide;
N-[1-(4-Benzenesulfonyl-6-oxo-hexahydro-pyrrolo[3,2-b]pyrrole-1-carbonyl)-3-methyl-butyl]-4-amino-benzamide;
4-Amino-N-{3-methyl-1-[6-oxo-4-(pyridine-2-sulfonyl)-hexahydro-pyrrolo[3,2-b]pyrrole-1-carbonyl]-butyl}-benzamide;
4-Amino-N-{3-methyl-1-[6-oxo-4-(1-oxy-pyridine-2-sulfonyl)-hexahydro-pyrrolo[3,2-b]pyrrole-1-carbonyl]-butyl}-benzamide;
4-Amino-N-{3-methyl-1-[6-oxo-4-(pyridine-3-sulfonyl)-hexahydro-pyrrolo[3,2-b]pyrrole-1-carbonyl]-butyl}-benzamide;
4-Amino-N-{3-methyl-1-[6-oxo4-(1-oxy-pyridine-3-sulfonyl)-hexahydro-pyrrolo[3,2-b]pyrrole-1-carbonyl]-butyl}-benzamide;
4-Amino-N-{3-methyl-1-[6-oxo-4-(pyridine-4-sulfonyl)-hexahydro-pyrrolo[3,2-b]pyrrole-1-carbonyl]-butyl}-benzamide;
4-Amino-N-{3-methyl-1-[6-oxo-4-(1-oxy-pyridine-4-sulfonyl)-hexahydro-pyrrolo[3,2-b]pyrrole-1-carbonyl]-butyl}-benzamide;
4-Amino-N-[3-methyl-1-(6-oxo-4-phenylacetyl-hexahydro-pyrrolo[3,2-b]pyrrole-1-carbonyl)-butyl]-benzamide;
4-[2-(4-amino-benzoylamino)-4-methyl-pentanoyl]-3-oxo-hexahydro-pyrrolo[3,2-b]pyrrole-1-carboxylic acid phenylamide;
4-[2-(4-amino-benzoylamino)-4-methyl-pentanoyl]-3-oxo-hexahydro-pyrrolo[3,2-b]pyrrole-1-carboxylic acid isobutyl-amide;

4-[2-(4-amino-benzoylamino)-4-methyl-pentanoyl]-3-oxo-hexahydro-pyrrolo[3,2-b]pyrrole-1-carboxylic acid phenyl ester;
4-[2-(4-amino-benzoylamino)-4-methyl-pentanoyl]-3-oxo-hexahydro-pyrrolo[3,2-b]pyrrole-1-carboxylic acid isobutyl ester;
4-Amino-N-{3-methyl-1-[6-oxo-4-(pyrrolidine-1-carbonyl)-hexahydro-pyrrolo[3,2-b]pyrrole-1-carbonyl]-butyl}-benzamide;
4-Amino-N-{3-methyl-1-[6-oxo-4-(piperidine-1-carbonyl)-hexahydro-pyrrolo[3,2-b]pyrrole-1-carbonyl]-butyl}-benzamide;
4-Amino-N-{3-methyl-1-[4-(morpholine-4-carbonyl)-6-oxo-hexahydro-pyrrolo[3,2-b]pyrrole-1-carbonyl]-butyl}-benzamide;
4-Amino-N-{3-methyl-1-[6-oxo-4-(piperazine-1-carbonyl)-hexahydro-pyrrolo[3,2-b]pyrrote-1-carbonyl]-butyl}-benzamide;
4-Amino-N-{3-methyl-1-[4-(4-methyl-piperazine-1-carbonyl)-6-oxo-hexahydro-pyrrolo[3,2-b]pyrrole-1-carbonyl]-butyl}-benzamide;
4-Amino-N-(3-methyl-1-{4-[2-(4-methyl-piperazin-1-yl)-acetyl]-6-oxo-hexahydro-pyrrolo[3,2-b]pyrrole-1-carbonyl}-butyl)-benzamide;
4-Amino-N-{3-methyl-1-[6-oxo4-(2-piperazin-1-yl-acetyl)-hexahydro-pyrrolo[3,2-b]pyrrole-1-carbonyl]-butyl}-benzamide;
4-Amino-N-{1-[4-(furan-2-carbonyl)-6-oxo-hexahydro-pyrrolo[3,2-b]pyrrole-1-carbonyl]-3-methyl-butyl}-benzamide;
4-Amino-N-{1-[4-(5-chloro-furan-2-carbonyl)-6-oxo-hexahydro-pyrrolo[3,2-b]pyrrole-1-carbonyl]-3-methyl-butyl}-benzamide;
4-Amino-N-{1-[4-(thiophene-2-carbonyl)-6-oxo-hexahydro-pyrrolo[3,2-b]pyrrole-1-carbonyl]-3-methyl-butyl}-benzamide;
4-Amino-N-{1-[4-(5-chloro-thiophene-2-carbonyl)-6-oxo-hexahydro-pyrrolo[3,2-b]pyrrole-1-carbonyl]-3-methyl-butyl}-benzamide;
4-Amino-N-{1-[4-(furan-3-carbonyl)-6-oxo-hexahydro-pyrrolo[3,2-b]pyrrole-1-carbonyl]-3-methyl-butyl}-benzamide;
4-Amino-N-{1-[4-(thiophene-3-carbonyl)-6-oxo-hexahydro-pyrrolo[3,2-b]pyrrole-1-carbonyl]-3-methyl-butyl}-benzamide;
4-Amino-N-[1-(4-cyclopentanecarbonyl-6-oxo-hexahydro-pyrrolo[3,2-b]pyrrole-1-carbonyl)-3-methyl-butyl]-benzamide;
4-Amino-N-[1-(4-cyclohexanecarbonyl-6-oxo-hexahydro-pyrrolo[3,2-b]pyrrole-1-carbonyl)-3-methyl-butyl]-benzamide;
4-Amino-N-{1-[4-(cyclopent-3-enecarbonyl)-6-oxo-hexahydro-pyrrolo[3,2-b]pyrrole-1-carbonyl]-3-methyl-butyl}-benzamide;
4-Amino-N-{1-[4-(2-cyclopentyl-acetyl)-6-oxo-hexahydro-pyrrolo[3,2-b]pyrrole-1-carbonyl]-3-methyl-butyl}-benzamide;
4-Amino-N-{1-[4-(2-cyclohexyl-acetyl)-6-oxo-hexahydro-pyrrolo[3,2-b]pyrrole-1-carbonyl]-3-methyl-butyl}-benzamide;
4-Amino-N-{3-methyl-1-[6-oxo-4-(pyrrolidine-2-carbonyl)-hexahydro-pyrrolo[3,2-b]pyrrole-1-carbonyl]-butyl}-benzamide;
N-{1-[4-(1-Acetyl-pyrrolidine-2-carbonyl)-6-oxo-hexahydro-pyrrolo[3,2-b]pyrrole-1-carbonyl]-3-methyl-butyl}-4-amino-benzamide;
4-Amino-N-{3-methyl-1-[6-oxo-4-(piperidine-2-carbonyl)-hexahydro-pyrrolo[3,2-b]pyrrole-1-carbonyl]-butyl}-benzamide;
N-{1-[4-(1-Acetyl-piperidine-2-carbonyl)-6-oxo-hexahydro-pyrrolo[3,2-b]pyrrole-1-carbonyl]-3-methyl-butyl}-4-amino-benzamide;
4-Amino-N-{3-methyl-1-[6-oxo-4-(tetrahydro-furan-2-carbonyl)-hexahydro-pyrrolo[3,2-b]pyrrole-1-carbonyl]-butyl}-benzamide;
N-{1-[4-(1-Amino-cyclobutanecarbonyl)-6-oxo-hexahydro-pyrrolo[3,2-b]pyrrole-1-carbonyl]-3-methyl-butyl}-4-Amino-benzamide;.
N-{1-[4-(1-Amino-cyclopentanecarbonyl)-6-oxo-hexahydro-pyrrolo[3,2-b]pyrrole-1-carbonyl]-3-methyl-butyl}-4-amino-benzamide;
N-{1-[4-(1-Amino-cyclohexanecarbonyl)-6-oxo-hexahydro-pyrrolo[3,2-b]pyrrole-1-carbonyl]-3-methyl-butyl}-4-amino-benzamide;
N-{1-[4-(1-Acetylamino-cyclobutanecarbonyl)-6-oxo-hexahydro-pyrrolo[3,2-b]pyrrole-1-carbonyl]-3-methyl-butyl}-4-amino-benzamide;

N-{1-[4-(1-Acetylamino-cyclopentanecarbonyl)-6-oxo-hexahydro-pyrrolo[3,2-b]pyrrole-1-carbonyl]-3-methyl-butyl}-4-amino-benzamide;
N-{1-[4-(1-Acetylamino-cyclohexanecarbonyl)6-oxo-hexahydro-pyrrolo[3,2-b]pyrrole-1-carbonyl]-3-methyl-butyl}-4-amino-benzamide;
4-Amino-N-{1-[4-(1-hydroxy-cyclobutanecarbonyl)-6-oxo-hexahydro-pyrrolo[3,2-b]pyrrole-1-carbonyl]-3-methyl-butyl}-benzamide;
4-Amino-N-{1-[4-(1-hydroxy-cyclopentanecarbonyl)-6-oxo-hexahydro-pyrrolo[3,2-b]pyrrole-1-carbonyl]-3-methyl-butyl}-benzamide;
4-Amino-N-{1-[4-(1-hydroxy-cyclohexanecarbonyl)-6-oxo-hexahydro-pyrrolo[3,2-b]pyrrole-1-carbonyl]-3-methyl-butyl}-benzamide;
4-Amino-N-{1-[4-(1-methoxy-cyclobutanecarbonyl)-6-oxo-hexahydro-pyrrolo[3,2-b]pyrrole-1-carbonyl]-3-methyl-butyl}-benzamide;
4-Amino-N-{1-[4-(1-methoxy-cyclopentanecarbonyl)-6-oxo-hexahydro-pyrrolo[3,2-b]pyrrole-1-carbonyl]-3-methyl-butyl}-benzamide;
4-Amino-N-{1-[4-(1-methoxy-cyclohexanecarbonyl)-6-oxo-hexahydro-pyrrolo[3,2-b]pyrrole-1-carbonyl]-3-methyl-butyl}-benzamide;
N-{1-[4-(2-amino-2-cyclopentyl-acetyl)-6-oxo-hexahydro-pyrrolo[3,2-b]pyrrole-1-carbonyl]-3-methyl-butyl}-4-amino-benzamide;
N-{1-[4-(2-amino-2-cyclohexyl-acetyl)-6-oxo-hexahydro-pyrrolo[3,2-b]pyrrole-1-carbonyl]-3-methyl-butyl}-4-amino-benzamide;
N-{1-[4-(2-acetylamino-2-cyclopentyl-acetyl)-6-oxo-hexahydro-pyrrolo[3,2-b]pyrrole-1-carbonyl]-3-methyl-butyl}-4-amino-benzamide;
N-{1-[4-(2-acetylamino-2-cyclohexyl-acetyl)-6-oxo-hexahydro-pyrrolo[3,2-b]pyrrole-1-carbonyl]-3-methyl-butyl}-4-amino-benzamide;
N-{1-[4-(2-acetylamino-propionyl)-6-oxo-hexahydro-pyrrolo[3,2-b]pyrrole-1-carbonyl]-3-methyl-butyl}-4-amino-benzamide;
N-{1-[4-(2-acetylamino-acetyl)-6-oxo-hexahydro-pyrrolo[3,2-b]pyrrole-1-carbonyl]-3-methyl-butyl}-4-amino-benzamide;
N-(1-{4-[2-(acetyl-methyl-amino)-acetyl]-6-oxo-hexahydro-pyrrolo[3,2-b]pyrrole-1-carbonyl}-3-methyl-butyl)-4-amino-benzamide;
4-Amino-N-{1-[4-(2-dimethylamino-acetyl)-6-oxo-hexahydro-pyrrolo[3,2-b]pyrrole-1-carbonyl]-3-methyl-butyl}-benzamide;
N-{1-[4-(2-amino-butyryl)-6-oxo-hexahydro-pyrrolo[3,2-b]pyrrole-1-carbonyl]-3-methyl-butyl}-4-amino-benzamide;
N-{1-[4-(2-acetylamino-butyryl)-6-oxo-hexahydro-pyrrolo[3,2-b]pyrrole-1-carbonyl]-3-methyl-butyl}-4-amino-benzamide;
N-{1-[4-(2-amino-3-methyl-butyryl)-6-oxo-hexahydro-prrolo[3,2-b]pyrrole-1-carbonyl]-3-methyl-butyl}-4-amino-benzamide;
N-{1-[4-(2-acetylamino-3-methyl-butyryl)-6-oxo-hexahydro-pyrrolo[3,2-b]pyrrole-1-carbonyl]-3-methyl-butyl}-4-amino-benzamide;
N-{1-[4-(2-amino-3,3-dimethyl-butyryl)-6-oxo-hexahydro-pyrrolo[3,2-b]pyrrole-1-carbonyl]-3-methyl-butyl}-4-amino-benzamide;
N-{1-[4-(2-acetylamino-3,3-dimethyl-butyryl)-6-oxo-hexahydro-pyrrolo[3,2-b]pyrrole-1-carbonyl]-3-methyl-butyl}-4-amino-benzamide;
N-{1-[4-(2-amino-3-methyl-pentanoyl)-6-oxo-hexahydro-pyrrolo[3,2-b]pyrrole-1-carbonyl]-3-methyl-butyl}-4-amino-benzamide;
N-{1-[4-(2-acetylamino-3-methyl-pentanoyl)-6-oxo-hexahydro-pyrrolo[3,2-b]pyrrole-1-carbonyl]-3-methyl-butyl}-4-amino-benzamide;
4-Amino-N-[3-methyl-1-(6-oxo-4-pentanoyl-hexahydro-pyrrolo[3,2-b]pyrrole-1-carbonyl)-butyl]-benzamide;
4-Amino-N-{3-methyl-1-[4-(3-methyl-pentanoyl)-6-oxo-hexahydro-pyrrolo[3,2-b]pyrrole-1-carbonyl]-butyl}-benzamide;
4-Amino-N-{3-methyl-1-[4-(4-methyl-pentanoyl)-6-oxo-hexahydro-pyrrolo[3,2-b]pyrrole-1-carbonyl]-butyl}-benzamide;
N-{1-[4-(2-amino-4-methyl-pentanoyl)-6-oxo-hexahydro-pyrrolo[3,2-b]pyrrole-1-carbonyl]-3-methyl-butyl}-4-amino-benzamide;
N-{1-[4-(2-acetylamino-4-methyl-pentanoyl)-6-oxo-hexahydro-pyrrolo[3,2-b]pyrrole-1-carbonyl]-3-methyl-butyl}-4-amino-benzamide;
N-{1-[4-(2-amino-4,4-dimethyl-pentanoyl)-6-oxo-hexahydro-pyrrolo[3,2-b]pyrrole-1-carbonyl]-3-methyl-butyl}-4-amino-benzamide;

N-{1-[4-(2-acetylamino-4,4-dimethyl-pentanoyl)-6-oxo-hexahydro-pyrrolo[3,2-b]pyrrole-1-carbonyl]-3-methyl-butyl}-4-amino-benzamide;
N-{1-[4-(2-amino-pentanoyl)-6-oxo-hexahydro-pyrrolo[3,2-b]pyrrole-1-carbonyl]-3-methyl-butyl}-4-amino-benzamide;
N-{1-[4-(2-acetylamino-pentanoyl)-6-oxo-hexahydro-pyrrolo[3,2-b]pyrrole-1-carbonyl]-3-methyl-butyl}-4-amino-benzamide;
N-{1-[4-(2-amino-5-methyl-hexanoyl)-6-oxo-hexahydro-pyrrolo[3,2-b]pyrrole-1-carbonyl]-3-methyl-butyl}-4-amino-benzamide;
N-{1-[4-(2-acetylamino-5-methyl-hexanoyl)-6-oxo-hexahydro-pyrrolo[3,2-b]pyrrole-1-carbonyl]-3-methyl-butyl}-4-amino-benzamide;
N-{1-[4-(2-hydroxy-3-methyl-butyryl)-6-oxo-hexahydro-pyrrolo[3,2-b]pyrrole-1-carbonyl]-3-methyl-butyl}-4-amino-benzamide;
N-{1-[4-(2-methoxy-3-methyl-butyryl)-6-oxo-hexahydro-pyrrolo[3,2-b]pyrrole-1-carbonyl]-3-methyl-butyl}-4-amino-benzamide;
4-Amino-N-{1-[4-(2-hydroxy-3,3-dimethyl-butyryl)-6-oxo-hexahydro-pyrrolo[3,2-b]pyrrole-1-carbonyl]-3-methyl-butyl}-benzamide;
4-Amino-N-{1-[4-(2-methoxy-3,3-dimethyl-butyryl)-6-oxo-hexahydro-pyrrolo[3,2-b]pyrrole-1-carbonyl]-3-methyl-butyl}-benzamide;
4-Amino-N-{1-[4-(2-hydroxy-3-methyl-pentanoyl)-6-oxo-hexahydro-pyrrolo[3,2-b]pyrrole-1-carbonyl]-3-methyl-butyl}-benzamide;
4-Amino-N-{1-[4-(2-methoxy-3-methyl-pentanoyl)-6-oxo-hexahydro-pyrrolo[3,2-b]pyrrole-1-carbonyl]-3-methyl-butyl}-benzamide;
4-Amino-N-{1-[4-(2-hydroxy-4-methyl-pentanoyl)-6-oxo-hexahydro-pyrrolo[3,2-b]pyrrole-1-carbonyl]-3-methyl-butyl}-benzamide;
4-Amino-N-{1-[4-(2-methoxy-4-methyl-pentanoyl)-6-oxo-hexahydro-pyrrolo[3,2-b]pyrrole-1-carbonyl]-3-methyl-butyl}-benzamide;
4-Amino-N-{1-[4-(2-hydroxy-4,4-dimethyl-pentanoyl)-6-oxo-hexahydro-pyrrolo[3,2-b]pyrrole-1-carbonyl]-3-methyl-butyl}-benzamide;
4-Amino-N-{1-[4-(2-methoxy-4,4-dimethyl-pentanoyl)-6-oxo-hexahydro-pyrrolo[3,2-b]pyrrole-1-carbonyl]-3-methyl-butyl}-benzamide;
4-Amino-N-{1-[4-(2-hydroxy-pentanoyl)-6-oxo-hexahydro-pyrrolo[3,2-b]pyrrole-1-carbonyl]-3-methyl-butyl}-benzamide;
4-Amino-N-{1-[4-(2-methoxy-pentanoyl)-6-oxo-hexahydro-pyrrolo[3,2-b]pyrrole-1-carbonyl]-3-methyl-butyl}-benzamide;
4-Amino-N-{1-[4-(2-hydroxy-5-methyl-hexanoyl)-6-oxo-hexahydro-pyrrolo[3,2-b]pyrrole-1-carbonyl]-3-methyl-butyl}-benzamide;
4-Amino-N-{1-[4-(2-methoxy-5-methyl-hexanoyl)-6-oxo-hexahydro-pyrrolo[3,2-b]pyrrole-1-carbonyl]-3-methyl-butyl}-benzamide;
N-[1-(4-Benzoyl-6-oxo-hexahydro-pyrrolo[3,2-b]pyrrole-1-carbonyl)-3-methyl-butyl]-4-diethylaminobenzamide;
4-Diethylamino-N-{3-methyl-1-[6-oxo-4-(pyridine-2-carbonyl)-hexahydro-pyrrolo[3,2-b]pyrrole-1-carbonyl]-butyl}-benzamide;
4-Diethylamino-N-{3-methyl-1-[6-oxo-4-(1-oxy-pyridine-2-carbonyl)-hexahydro-pyrrolo[3,2-b]pyrrole-1-carbonyl]-butyl}-benzamide;
4-Diethylamino-N-{3-methyl-1-[6-oxo-4-(pyridine-3-carbonyl)-hexahydro-pyrrolo[3,2-b]pyrrole-1-carbonyl]-butyl}-benzamide;
4-Diethylamino-N-{3-methyl-1-[6-oxo-4-(1-oxy-pyridine-3-carbonyl)-hexahydro-pyrrolo[3,2-b]pyrrole-1-carbonyl]-butyl}-benzamide;
4-Diethylamino-N-{3-methyl-1-[6-oxo4-(pyridine-4-carbonyl)-hexahydro-pyrrolo[3,2-b]pyrrole-1-carbonyl]-butyl}-benzamide;
4-Diethylamino-N-{3-methyl-1-[6-oxo-4-(1-oxy-pyridine-4-carbonyl)-hexahydro-pyrrolo[3,2-b]pyrrole-1-carbonyl]-butyl}-benzamide;
N-[1-(4-Benzenesulfonyl-6-oxo-hexahydro-pyrrolo[3,2-b]pyrrole-1-carbonyl)-3-methyl-butyl]-4-diethylamino-benzamide;
4-Diethylamino-N-{3-methyl-1-[6-oxo-4-(pyridine-2-sulfonyl)-hexahydro-pyrrolo[3,2-b]pyrrole-1-carbonyl]-butyl}-benzamide;
4-Diethylamino-N-{3-methyl-1-[6-oxo-4-(1-oxy-pyridine-2-sulfonyl)-hexahydro-pyrrolo[3,2-b]pyrrole-1-carbonyl]-butyl}-benzamide;
4-Diethylamino-N-{3-methyl-1-[6-oxo-4-(pyridine-3-sulfonyl)-hexahydro-pyrrolo[3,2-b]pyrrole-1-carbonyl]-butyl}-

benzamide;
4-Diethylamino-N-{3-methyl-1-[6-oxo-4-(1-oxy-pyridine-3-sulfonyl)-hexahydro-pyrrolo[3,2-b]pyrrole-1-carbonyl]-butyl}-benzamide;
4-Diethylamino-N-{3-methyl-1-[6-oxo-4-(pyridine4-sulfonyl)-hexahydro-pyrrolo[3,2-b]pyrrole-1-carbonyl]-butyl}-benzamide;
4-Diethylamino-N-{3-methyl-1-[6-oxo-4-(1-oxy-pyridine-4-sulfonyl)-hexahydro-pyrrolo[3,2-b]pyrrole-1-carbonyl]-butyl}-benzamide;
4-Diethylamino-N-[3-methyl-1-(6-oxo-4-phenylacetyl-hexahydro-pyrrolo[3,2-b]pyrrole-1-carbonyl)-butyl]-benzamide;
4-[2-(4-diethylamino-benzoylamino)-4-methyl-pentanoyl]-3-oxo-hexahydro-pyrrolo[3,2-b]pyrrole-1-carboxylic acid phenylamide;
4-[2-(4-diethylamino-benzoylamino)-4-methyl-pentanoyl]-3-oxo-hexahydro-pyrrolo[3,2-b]pyrrole-1-carboxylic acid isobutyl-amide;
4-[2-(4-diethylamino-benzoylamino)-4-methyl-pentanoyl]-3-oxo-hexahydro-pyrrolo[3,2-b]pyrrole-1-carboxylic acid phenyl ester;
4-[2-(4-diethylamino-benzoylamino)-4-methyl-pentanoyl]-3-oxo-hexahydro-pyrrolo[3,2-b]pyrrole-1-carboxylic acid isobutyl ester;
4-Diethylamino-N-{3-methyl-1-[6-oxo-4-(pyrrolidine-1-carbonyl)-hexahydro-pyrrolo[3,2-b]pyrrole-1-carbonyl]-butyl}-benzamide;
4-Diethylamino-N-{3-methyl-1-[6-oxo-4-(piperidine-1-carbonyl)-hexahydro-pyrrolo[3,2-b]pyrrole-1-carbonyl]-butyl}-benzamide;
4-Diethylamino-N-{3-methyl-1-[4-(morpholine-4-carbonyl)-6-oxo-hexahydro-pyrrolo[3,2-b]pyrrole-1-carbonyl]-butyl}-benzamide;
4-Diethylamino-N-{3-methyl-1-[6-oxo-4-(piperazine-1-carbonyl)-hexahydro-pyrrolo[3,2-b]pyrrole-1-carbonyl]-butyl}-benzamide;
4-Diethylamino-N-{3-methyl-1-[4-(4-methyl-piperazine-1-carbonyl)-6-oxo-hexahydro-pyrrolo[3,2-b]pyrrole-1-carbonyl]-butyl}-benzamide;
4-Diethylamino-N-(3-methyl-1-{4-[2-(4-methyl-piperazin-1-yl)-acetyl]-6-oxo-hexahydro-pyrrolo[3,2-b]pyrrole-1-carbonyl}-butyl)-benzamide;
4-Diethylamino-N-{3-methyl-1-[6-oxo-4-(2-piperazin-1-yl-acetyl)-hexahydro-pyrrolo[3,2-b]pyrrole-1-carbonyl]-butyl}-benzamide;
4-Diethylamino-N-{1-[4-(furan-2-carbonyl)-6-oxo-hexahydro-pyrrolo[3,2-b]pyrrole-1-carbonyl]-3-methyl-butyl}-benzamide;
4-Diethylamino-N-{1-[4-(5-chloro-furan-2-carbonyl)-6-oxo-hexahydro-pyrrolo[3,2-b]pyrrole-1-carbonyl]-3-methyl-butyl}-benzamide;
4-Diethylamino-N-{1-[4-(thiophene-2-carbonyl)-6-oxo-hexahydro-pyrrolo[3,2-b]pyrrole-1-carbonyl]-3-methyl-butyl}-benzarnide;
4-Diethylamino-N-{1-[4-(5-chloro-thiophene-2-carbonyl)-6-oxo-hexahydro-pyrrolo[3,2-b]pyrrole-1-carbonyl]-3-methyl-butyl}-benzamide;
4-Diethylamino-N-{1-[4-(furan-3-carbonyl)-6-oxo-hexahydro-pyrrolo[3,2-b]pyrrole-1-carbonyl]-3-methyl-butyl}-benzamide;
4-Diethylamino-N-{1-[4-(thiophene-3-carbonyl)-6-oxo-hexahydro-pyrrolo[3,2-b]pyrrole-1-carbonyl]-3-methyl-butyl}-benzamide;
4-Diethylamino-N-[1-(4-cyclopentanecarbonyl-6-oxo-hexahydro-pyrrolo[3,2-b]pyrrole-1-carbonyl)-3-methyl-butyl]-benzamide;
4-Diethylamino-N-[1-(4-cyclohexanecarbonyl-6-oxo-hexahydro-pyrrolo[3,2-b]pyrrole-1-carbonyl)-3-methyl-butyl]-benzamide;
4-Diethylamino-N-{1-[4-(cyclopent-3-enecarbonyl)-6-oxo-hexahydro-pyrrolo[3,2-b]pyrrole-1-carbonyl]-3-methyl-butyl}-benzamide;
4-Diethylamino-N-{1-[4-(2-cyclopentyl-acetyl)-6-oxo-hexahydro-pyrrolo[3,2-b]pyrrole-1-carbonyl]-3-methyl-butyl}-benzamide;
4-Diethylamino-N-{1-[4-(2-cyclohexyl-acetyl)-6-oxo-hexahydro-pyrrolo[3,2-b]pyrrole-1-carbonyl]-3-methyl-butyl}-benzamide;
4-Diethylamino-N-{3-methyl-1-[6-oxo-(pyrrolidine-2-carbonyl)-hexahydro-pyrrolo[3,2-b]pyrrole-1-carbonyl]-butyl}-benzamide;
N-{1-[4-(1-Acetyl-pyrrolidine-2-carbonyl)-6-oxo-hexahydro-pyrrolo[3,2-b]pyrrole-1-carbonyl]-3-methyl-butyl}-4-diethylamino-benzamide;
4-Diethylamino-N-{3-methyl-1-[6-oxo-4-(piperidine-2-carbonyl)-hexahydro-pyrrolo[3,2-b]pyrrole-1-carbonyl]-butyl}-benzamide;
N-{1-[4-(1-Acetyl-piperidine-2-carbonyl)-6-oxo-hexahydro-pyrrolo[3,2-b]pyrrole-1-carbonyl]-3-methyl-butyl}-4-diethylamino-benzamide;
4-Diethylamino-N-{3-methyl-1-[6-oxo-4-(tetrahydro-furan-2-carbonyl)-hexahydro-pyrrolo[3,2-b]pyrrole-1-carbonyl]-butyl}-benzamide;
N-{1-[4-(1-Amino-cyclobutanecarbonyl)-6-oxo-hexahydro-pyrrolo[3,2-b]pyrrole-1-carbonyl]-3-methyl-butyl}-4-diethylamino-benzamide;
N-{1-[4-(1-Amino-cyclopentanecarbonyl)-6-oxo-hexahydro-pyrrolo[3,2-b]pyrrole-1-carbonyl]-3-methyl-butyl}-4-diethylamino-benzamide;
N-{1-[4-(1-Amino-cyclohexanecarbonyl)-6-oxo-hexahydro-pyrrolo[3,2-b]pyrrole-1-carbonyl]-3-methyl-butyl}-4-diethylamino-benzamide;
N-{1-[4-(1-Acetylamino-cyclobutanecarbonyl)-6-oxo-hexahydro-pyrrolo[3,2-b]pyrrole-1-carbonyl]-3-methyl-butyl}-4-diethylamino-benzamide;
N-{1-[4-(1-Acetylamino-cyclopentanecarbonyl)-6-oxo-hexahydro-pyrrolo[3,2-b]pyrrole-1-carbonyl]-3-methyl-butyl}-4-diethylamino-benzamide;
N-{1-[4-(1-Acetylamino-cyclohexanecarbonyl)-6-oxo-hexahydro-pyrrolo[3,2-b]pyrrole-1-carbonyl]-3-methyl-butyl}-4-diethylamino-benzamide;
4-Diethylamino-N-{1-[4-(1-hydroxy-cyclobutanecarbonyl)-6-oxo-hexahydro-pyrrolo[3,2-b]pyrrole-1-carbonyl]-3-methyl-butyl}-benzamide;
4-Diethylamino-N-{1-[4-(1-hydroxy-cyclopentanecarbonyl)-6-oxo-hexahydro-pyrrolo[3,2-b]pyrrole-1-carbonyl]-3-methyl-butyl}-benzamide;
4-Diethylamino-N-{1-[4-(1-hydroxy-cyclohexanecarbonyl)-6-oxo-hexahydro-pyrrolo[3,2-b]pyrrole-1-carbonyl]-3-methyl-butyl}-benzamide;
4-Diethylamino-N-{1-[4-(1-methoxy-cyclobutanecarbonyl)-6-oxo-hexahydro-pyrrolo[3,2-b]pyrrole-1-carbonyl]-3-methyl-butyl}-benzamide;
4-Diethylamino-N-{1-[4-(1-methoxy-cyclopentanecarbonyl)-6-oxo-hexahydro-pyrrolo[3,2-b]pyrrole-1-carbonyl]-3-methyl-butyl}-benzamide;
4-Diethylamino-N-{1-[4-(1-methoxy-cyclohexanecarbonyl)-6-oxo-hexahydro-pyrrolo[3,2-b]pyrrole-1-carbonyl]-3-methyl-butyl}-benzamide;
N-{1-[4-(2-Amino-2-cyclopentyl-acetyl)-6-oxo-hexahydro-pyrrolo[3,2-b]pyrrole-1-carbonyl]-3-methyl-butyl}-4-diethylamino-benzamide;
N-{1-[4-(2-Amino-2-cyclohexyl-acetyl)-6-oxo-hexahydro-pyrrolo[3,2-b]pyrrole-1-carbonyl]-3-methyl-butyl}-4-diethylamino-benzamide;
N-{1-[4-(2-Acetylamino-2-cyclopentyl-acetyl)-6-oxo-hexahydro-pyrrolo[3,2-b]pyrrole-1-carbonyl]-3-methyl-butyl}-4-diethylamino-benzamide;
N-{1-[4-(2-Acetylamino-2-cyclohexyl-acetyl)-6-oxo-hexahydro-pyrrolo[3,2-b]pyrrole-1-carbonyl]-3-methyl-butyl}-4-diethylamino-benzamide;
N-{1-[4-(2-Acetylamino-propionyl)-6-oxo-hexahydro-pyrrolo[3,2-b]pyrrole-1-carbonyl]-3-methyl-butyl}-4-diethylamino-benzamide;
N-{1-[4-(2-Acetylamino-acetyl)-6-oxo-hexahydro-pyrrolo[3,2-b]pyrrole-1-carbonyl]-3-methyl-butyl}-4-diethylamino-benzamide;
N-(1-{4-[2-(Acetyl-methyl-amino)-acetyl]-6-oxo-hexahydro-pyrrolo[3,2-b]pyrrole-1-carbonyl}-3-methyl-butyl)-4-diethylamino-benzamide;
4-Diethylamino-N-{1-[4-(2-dimethylamino-acetyl)-6-oxo-hexahydro-pyrrolo[3,2-b]pyrrole-1-carbonyl]-3-methyl-butyl}-benzamide;
N-{1-[4-(2-Amino-butyryl)-6-oxo-hexahydro-pyrrolo[3,2-b]pyrrole-1-carbonyl]-3-methyl-butyl}-4-diethylamino-benzamide;
N-{1-[4-(2-Acetylamino-butyryl)-6-oxo-hexahydro-pyrrolo[3,2-b]pyrrole-1-carbonyl]-3-methyl-butyl}-4-diethylamino-benzamide;
N-{1-[4-(2-amino-3-methyl-butyryl)-6-oxo-hexahydro-pyrrolo[3,2-b]pyrrole-1-carbonyl]-3-methyl-butyl}-4-diethylamino-benzamide;
N-{1-[4-(2-Acetylamino-3-methyl-butyryl)-6-oxo-hexahydro-pyrrolo[3,2-b]pyrrole-1-carbonyl]-3-methyl-butyl}-4-diethylamino-benzamide;
N-{1-[4-(2-amino-3,3-dimethyl-butyryl)-6-oxo-hexahydro-pyrrolo[3,2-b]pyrrole-1-carbonyl]-3-methyl-butyl}-4-diethylamino-benzamide;
N-{1-[4-(2-Acetylamino-3,3-dimethyl-butyryl)-6-oxo-hexahydro-pyrrolo[3,2-b]pyrrole-1-carbonyl]-3-methyl-butyl}-4-diethylamino-benzamide;
N-{1-[4-(2-Amino-3-methyl-pentanoyl)-6-oxo-hexahydro-pyrrolo[3,2-b]pyrrole-1-carbonyl]-3-methyl-butyl}-4-diethylamino-benzamide;
N-{1-[4-(2-Acetylamino-3-methyl-pentanoyl)-6-oxo-hexahydro-pyrrolo[3,2-b]pyrrole-1-carbonyl]-3-methyl-butyl}-4-diethylamino-benzamide;
4-Diethylamino-N-[3-methyl-1-(6-oxo-4-pentanoyl-hexahydro-pyrrolo[3,2-b]pyrrole-1-carbonyl)-butyl]-benzamide;
4-Diethylamino-N-{3-methyl-1-[4-(3-methyl-pentanoyl)-6-oxo-hexahydro-pyrrolo[3,2-b]pyrrole-1-carbonyl]-butyl}-benzamide;
4-Diethylamino-N-{3-methyl-1-[4-(4-methyl-pentanoyl)-6-oxo-hexahydro-pyrrolo[3,2-b]pyrrole-1-carbonyl]-butyl}-benzamide;
N-{1-[4-(2-Amino-4-methyl-pentanoyl)-6-oxo-hexahydro-pyrrolo[3,2-b]pyrrole-1-carbonyl]-3-methyl-butyl}-4-diethylamino-benzamide;
N-{1-[4-(2-Acetylamino-4-methyl-pentanoyl)-6-oxo-hexahydro-pyrrolo[3,2-b]pyrrole-1-carbonyl]-3-methyl-butyl}-4-diethylamino-benzamide;
N-{1-[4-(2-Amino-4,4-dimethyl-pentanoyl)-6-oxo-hexahydro-pyrrolo[3,2-b]pyrrole-1-carbonyl]-3-methyl-butyl}-4-diethylamino-benzamide;
N-{1-[4-(2-Acetylamino-4,4-dimethyl-pentanoyl)-6-oxo-hexahydro-pyrrolo[3,2-b]pyrrole-1-carbonyl]-3-methyl-butyl}-4-diethylamino-benzamide;
N-{1-[4-(2-Amino-pentanoyl)-6-oxo-hexahydro-pyrrolo[3,2-b]pyrrole-1-carbonyl]-3-methyl-butyl}-4-diethylamino-benzamide;
N-{1-[4-(2-Acetylamino-pentanoyl)-6-oxo-hexahydro-pyrrolo[3,2-b]pyrrole-1-carbonyl]-3-methyl-butyl}-4-diethylamino-benzamide;
N-{1-[4-(2-Amino-5-methyl-hexanoyl)-6-oxo-hexahydro-pyrrolo[3,2-b]pyrrole-1-carbonyl]-3-methyl-butyl}-4-diethylamino-benzamide;
N-{1-[4-(2-Acetylamino-5-methyl-hexanoyl)-6-oxo-hexahydro-pyrrolo[3,2-b]pyrrole-1-carbonyl]-3-methyl-butyl}-4-diethylamino-benzamide;
N-{1-[4-(2-hydroxy-3-methyl-butyryl)-6-oxo-hexahydro-pyrrolo[3,2-b]pyrrole-1-carbonyl]-3-methyl-butyl}-4-diethylamino-benzamide;
N-{1-[4-(2-methoxy-3-methyl-butyryl)-6-oxo-hexahydro-pyrrolo[3,2-b]pyrrole-1-carbonyl]-3-methyl-butyl}-4-diethylamino-benzamide;
4-Diethylamino-N-{1-[4-(2-hydroxy-3,3-dimethyl-butyryl)-6-oxo-hexahydro-pyrrolo[3,2-b]pyrrole-1-carbonyl]-3-methyl-butyl}-benzamide;
4-Diethylamino-N-{1-[4-(2-methoxy-3,3-dimethyl-butyryl)-6-oxo-hexahydro-pyrrolo[3,2-b]pyrrole-1-carbonyl]-3-methyl-butyl}-benzmide;
4-Diethylamino-N-{1-[4-(2-hydroxy-3-methyl-pentanoyl)-6-oxo-hexahydro-pyrrolo[3,2-b]pyrrole-1-carbonyl]-3-methyl-butyl}-benzamide;
4-Diethylamino-N-{1-[4-(2-methoxy-3-methyl-pentanoyl)-6-oxo-hexahydro-pyrrolo[3,2-b]pyrrole-1-carbonyl]-3-methyl-butyl}-benzamide;
4-Diethylamino-N-{1-[4-(2-hydroxy-4-methyl-pentanoyl)-6-oxo-hexahydro-pyrrolo[3,2-b]pyrrole-1-carbonyl]-3-methyl-butyl}-benzamide;
4-Diethylamino-N-{1-[4-(2-methoxy-4-methyl-pentanoyl)-6-oxo-hexahydro-pyrrolo[3,2-b]pyrrole-1-carbonyl]-3-methyl-butyl}-benzamide;
4-Diethylamino-N-{1-[4-(2-hydroxy-4,4-dimethyl-pentanoyl)-6-oxo-hexahydro-pyrrolo[3,2-b]pyrrole-1-carbonyl]-3-methyl-butyl}-benzamide;
4-Diethylamino-N-{1-[4-(2-methoxy-4,4-dimethyl-pentanoyl)-6-oxo-hexahydro-pyrrolo[3,2-b]pyrrole-1-carbonyl]-3-methyl-butyl}-benzamide;
4-Diethylamino-N-{1-[4-(2-hydroxy-pentanoyl)-6-oxo-hexahydro-pyrrolo[3,2-b]pyrrole-1-carbonyl]-3-methyl-butyl}-benzamide;
4-Diethylamino-N-{1-[4-(2-methoxy-pentanoyl)-6-oxo-hexahydro-pyrrolo[3,2-b]pyrrole-1-carbonyl]-3-methyl-butyl}-benzamide;
4-Diethylamino-N-{1-[4-(2-hydroxy-5-methyl-hexanoyl)-6-oxo-hexahydro-pyrrolo[3,2-b]pyrrole-1-carbonyl]-3-meth yl-butyl}-benzamide;
4-Diethylamino-N-{1-[4-(2-methoxy-5-methyl-hexanoyl)-6-oxo-hexahydro-pyrrolo[3,2-b]pyrrole-1-carbonyl]-3-meth yl-butyl}-benzamide;

**36.** A compound as claimed in any one of claims 1 to 35 for use in medicine.

**37.** A compound as claimed in any one of claims 1 to 35 for use in the treatment of osteoporosis, Paget's disease, gingival diseases such as gingivitis and periodontitis, hypercalaemia of malignancy, metabolic bone disease, diseases involving matrix or cartilage degradation, in particular osteoarthritis or rheumatoid arthritis and neoplastic

diseases.

**38.** The use of a compound as claimed in any one of claims 1 to 35 in the preparation of a medicament for the treatment of osteoporosis, Paget's disease, gingival diseases such as gingivitis and periodontitis, hypercalaemia of malignancy, metabolic bone disease, diseases involving matrix or cartilage degradation, in particular osteoarthritis or rheumatoid arthritis and neoplastic diseases.

**39.** A pharmaceutical or veterinary composition comprising one or more compounds as claimed in any one of claims 1 to 35 and a pharmaceutically or veterinarily acceptable carrier.

**40.** A process for the preparation of a pharmaceutical or veterinary composition as claimed in claim 39, the process comprising bringing the active compound(s) into association with the carrier.

## Patentansprüche

**1.** Verbindung der allgemeinen Formel (I)

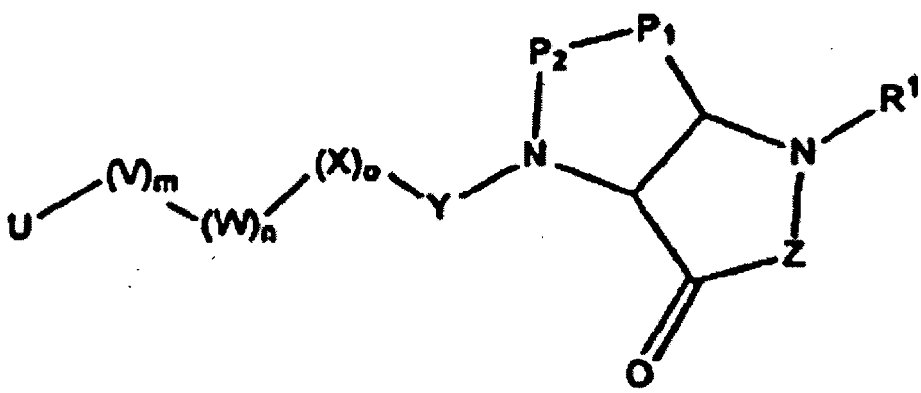

(I)

worin:

Z = $CR^3R^4$, wobei $R^3$ und $R^4$ unabhängig voneinander ausgewählt sind unter $C_{0-7}$-Alkyl (wenn C = 0 ist, ist $R^3$ oder $R^4$ einfach ein Wasserstoffatom), $C_{3-6}$-Cycloalkyl, Ar-$C_{0-7}$-Alkyl (wenn C = 0 ist, ist $R^3$ oder $R^4$ einfach ein aromatischer Rest Ar),

$P_1$ = $CR^5R^6$, wobei $R^5$ und $R^6$ unabhängig voneinander ausgewählt sind unter $C_{0-7}$-Alkyl, $C_{3-6}$-Cycloalkyl, Ar-$C_{0-7}$-Alkyl, O-$C_{0-7}$-Alkyl, O-$C_{3-6}$-Cycloalkyl, O-Ar-$C_{0-7}$-Alkyl, S-$C_{0-7}$-Alkyl, S-$C_{3-6}$-Cycloalkyl, S-Ar-$C_{0-7}$-Alkyl, NH-$C_{0-7}$-Alkyl, NH-$C_{3-6}$-Cycloalkyl, NH-Ar-$C_{0-7}$-Alkyl, N($C_{0-7}$-Alkyl)$_2$, N($C_{3-6}$-Cycloalkyl)$_2$ und N(Ar-$C_{0-7}$-Alkyl)$_2$,

$P_2$ = O, $CR^7R^8$ oder $NR^9$, wobei $R^7$ und $R^8$ unabhängig voneinander ausgewählt sind unter $C_{0-7}$-Alkyl, $C_{3-6}$-Cycloalkyl und Ar-$C_{0-7}$-Alkyl und $R^9$ ausgewählt ist unter $C_{0-7}$-Alkyl, $C_{3-6}$-Cycloalkyl und Ar-$C_{0-7}$-Alkyl,

Y = $CR^{10}R^{11}$-C(O) oder $CR^{10}R^{11}$-C(S) oder $CR^{10}R^{11}$-S(O) oder $CR^{10}R^{11}$-$SO_2$, wobei $R^{10}$ und $R^{11}$ unabhängig voneinander ausgewählt sind unter $C_{0-7}$-Alkyl, $C_{3-6}$-Cycloalkyl und Ar-$C_{0-7}$-Alkyl, oder Y

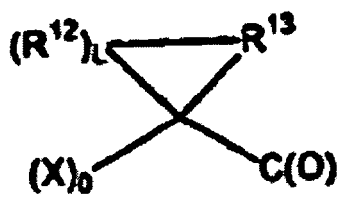

oder C(S) oder S(O) oder $SO_2$ repräsentiert, wobei L eine Zahl von eins bis vier ist und $R^{12}$ und $R^{13}$ unabhängig voneinander ausgewählt sind unter $CR^{14}R^{15}$, wobei $R^{14}$ und $R^{15}$ unabhängig voneinander ausgewählt sind unter $C_{0-7}$-Alkyl, $C_{3-6}$-Cycloalkyl, Ar-$C_{0-7}$-Alkyl und Halogen und für jeden $R^{12}$ und $R^{13}$ entweder $R^{14}$ oder $R^{15}$ (aber nicht sowohl $R^{14}$ als auch $R^{15}$) zusätzlich unter O-$C_{0-7}$-Alkyl, O-$C_{3-6}$-Cycloalkyl, O-Ar-$C_{0-7}$-Alkyl, S-$C_{0-7}$Alkyl, S-$C_{3-6}$-Cycloalkyl, S-Ar-$C_{0-7}$-Alkyl, NH-$C_{0-7}$-Alkyl, NH-$C_{3-6}$-Cycloalkyl, NH-Ar-$C_{0-7}$-Alkyl, N-($C_{0-7}$-Alkyl)$_2$, N-($C_{3-6}$-Cycloalkyl)$_2$ und N-(Ar-$C_{0-7}$-Alkyl)$_2$ ausgewählt sein können,

$(X)_o$ = $CR^{16}R^{17}$, wobei $R^{16}$ und $R^{17}$ unabhängig voneinander ausgewählt sind unter $C_{0-7}$-Alkyl, $C_{3-6}$-Cycloalkyl und Ar-$C_{0-7}$-Alkyl und o eine Zahl von null bis drei ist,

$(W)_n$ = O, S, C(O), S(O) oder $S(O)_2$ oder $NR^{18}$, wobei $R^{18}$ ausgewählt ist unter $C_{0-7}$-Alkyl, $C_{3-6}$-Cycloalkyl und Ar-$C_{0-7}$-Alkyl und n null oder eins ist,

$(V)_m$ = C(O), C(S), S(O), $S(O)_2$, $S(O)_2NH$, OC(O), NHC(O), NHS(O), $NHS(O)_2$, OC(O)NH, C(O)NH oder $CR^{19}R^{20}$, C=N-C(O)-$OR^{19}$ oder C=N-C(O)-$NHR^{19}$, wobei $R^{19}$ und $R^{20}$ unabhängig voneinander ausgewählt sind unter $C_{0-7}$-Alkyl, $C_{3-6}$-Cycloalkyl, Ar-$C_{0-7}$-Alkyl und m eine Zahl von null bis drei ist, mit der Maßgabe, daß, wenn m größer als eins ist, $(V)_m$ maximal eine Carbonyl- oder Sulfonylgruppe enthält,

U = ein stabiler 5- bis 7-gliedriger monozyklischer oder ein stabiler 8- bis 11-gliedriger bizyklischer Ring ist, welcher entweder gesättigt oder ungesättigt ist und welcher null bis vier Heteroatome enthält (wie unten aufgeführt):

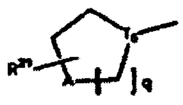
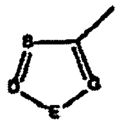
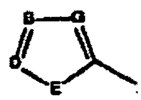
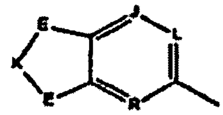
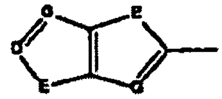

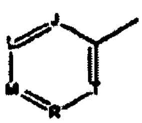
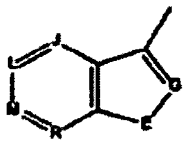
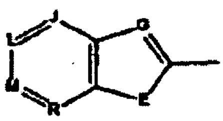
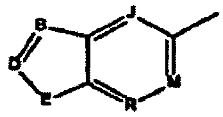
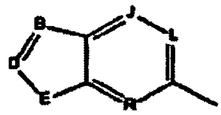

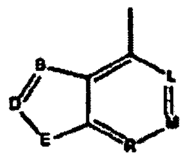
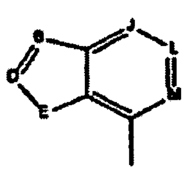
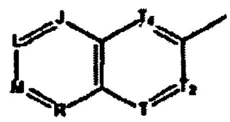
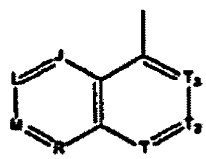
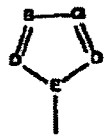

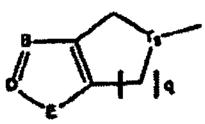
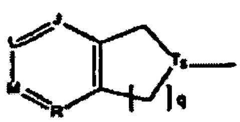
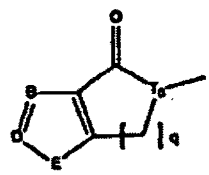
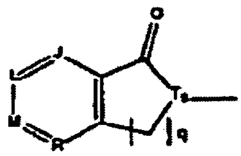

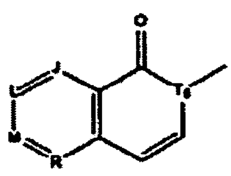
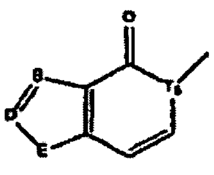
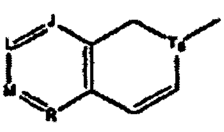
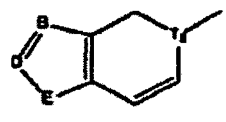

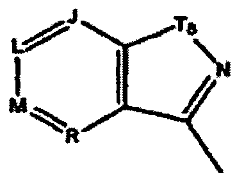 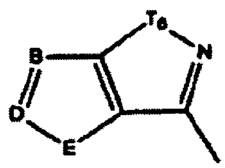 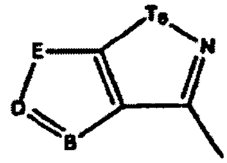

wobei $R^{21}$ folgendes ist:
$C_{0-7}$-Alkyl, $C_{3-6}$-Cycloalkyl, Ar-$C_{0-7}$-Alkyl, O-$C_{0-7}$-Alkyl, O-$C_{3-6}$-Cycloalkyl, O-Ar-$C_{0-7}$-Alkyl, S-$C_{0-7}$-Alkyl, S-$C_{3-6}$-Cycloalkyl, S-Ar-$C_{0-7}$-Alkyl, $SO_2$-$C_{0-7}$-Alkyl, $SO_2$-$C_{3-6}$-Cycloalkyl, $SO_2$-Ar-$C_{0-7}$-Alkyl, NH-$C_{0-7}$-Alkyl, NH-$C_{3-6}$-Cycloalkyl, NH-Ar-$C_{0-7}$-Alkyl, N($C_{0-7}$-Alkyl)$_2$, N($C_{3-6}$-Cycloalkyl)$_2$ oder N(Ar-$C_{0-7}$-Alkyl)$_2$, oder, wenn er Teil einer $CHR^{21}$- oder $CR^{21}$-Gruppe ist, $R^{21}$ Halogen sein kann,
A ausgewählt ist unter:

$CH_2$, $CHR^{21}$, O, S, $SO_2$, $NR^{22}$ oder N-Oxid (N$\rightarrow$O), wobei $R^{21}$ wie oben definiert ist, und $R^{22}$ ausgewählt ist unter $C_{0-7}$-Alkyl, $C_{3-6}$-Cycloalkyl und Ar-$C_{0-7}$-Alkyl,

B, D und G unabhängig voneinander ausgewählt sind unter:

$CR^{21}$, wobei $R^{21}$ wie oben definiert oder N oder N-Oxid (N$\rightarrow$O) ist,

E ausgewählt ist unter:

$CH_2$, $CHR^{21}$, O, S, $SO_2$, $NR^{22}$ oder N-Oxid (N$\rightarrow$O), wobei $R^{21}$ und $R^{22}$ wie oben definiert sind,

K ausgewählt ist unter:

$CH_2$, $CHR^{22}$, wobei $R^{22}$ wie oben definiert ist,

J, L, M, R, T, $T_2$, $T_3$ und $T_4$ unabhängig voneinander ausgewählt sind unter:

$CR^{21}$, wobei $R^{21}$ wie oben definiert ist, oder N oder N-Oxid (N$\rightarrow$O),

$T_5$ ausgewählt ist unter:

CH oder N,

$T_6$ ausgewählt ist unter:

$NR^{22}$, $SO_2$, OC(O), C(O), $NR^{22}$C(O),

q eine Zahl von eins bis drei ist, wodurch ein 5-, 6- oder 7-gliedriger Ring definiert wird,
$R^1$ = $R^2$C(O), $R^2$OC(O), $R^2$NQC(O), $R^2SO_2$, wobei $R^2$ unter $C_{1-7}$-Alkyl, $C_{3-6}$-Cycloalkyl oder Ar-$C_{0-7}$-Akyl (wenn C = 0 ist, ist $R^2$ einfach ein aromatischer Rest Ar) ausgewählt ist und Q $C_{0-7}$-Alkyl ist,
oder ein Salz, Hydrat, Solvat oder Komplex davon.

**2.** Verbindung nach Anspruch 1, wobei unabhängig voneinander oder in irgendeiner beliebigen Kombination
Z $CH_2$ ist,
$P_1$ $CH_2$ ist,
$P_2$ $CH_2$, O oder NH ist,
$R^2$ Ar-$C_{0-2}$-Alkyl ist
oder $R^2$ $C_{3-7}$-Alkyl ist, welches ein -O- oder -NH- als Teil der Kette enthalten kann und welches entweder unsubstituiert oder mit einer oder mehreren von $NH_2$-, NHMe-, NHC(O)$CH_3$-, NMeC(O)$CH_3$-, OH- oder OMe-Gruppe(n) substituiert ist,
oder $R^2$ eine $C_{3-6}$-Cycloalkylgruppe ist, wobei das Ringsystem entweder direkt mit dem Rest des $R^1$-Rests verbunden ist oder es eine dazwischenliegende Methylengruppe gibt,

in der Gruppe $(X)_o$ jeder von $R^{16}$ und $R^{17}$ ausgewählt ist unter $C_{0-7}$-Alkyl oder Ar-$C_{0-7}$-Alkyl, beispielsweise Wasserstoff, einer geraden oder verzweigten Alkylkette, einer geraden oder verzweigten Heteroalkylkette, einer optional substituierten Arylalkylkette
oder einer optional substituierten Arylheteroalkylkette,
in der Gruppe $(X)_o$ $R^{16}$ und $R^{17}$ Wasserstoff sind und o null oder eins ist,
in der Gruppe $(V)_m$ V ausgewählt ist unter C(O), OC(O), NHC(O), C(O)NH, $CHR^{20}$,
C=N-C(O)-$OR^{19}$ oder C=N-C(O)-$NHR^{19}$,
wobei $R^{19}$ ausgewählt ist unter $C_{0-7}$-Alkyl, $C_{3-6}$-Cycloalkyl, Ar-$C_{0-7}$-Alkyl und
$R^{20}$ $C_{0-4}$-Alkyl ist und
m null oder eins ist.

**3.** Verbindung nach Anspruch 2, wobei $R^2$ folgendes ist:

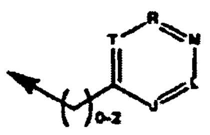
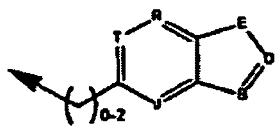
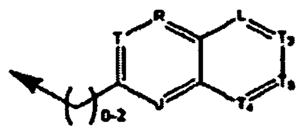

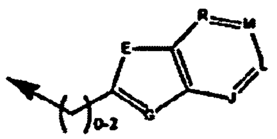
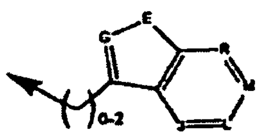
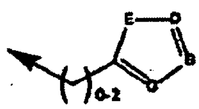
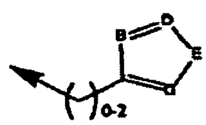

wobei J, L, M, R, T, $T_2$, $T_3$ und $T_4$, B, D, G und E wie zuvor definiert sind.

**4.** Verbindung nach einem der Ansprüche 1 bis 3, wobei $R^2$ Ar-$C_{0-1}$-Alkyl ist, beispielsweise:

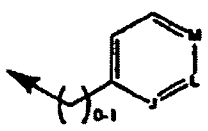
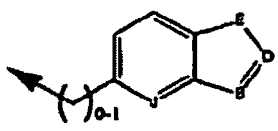
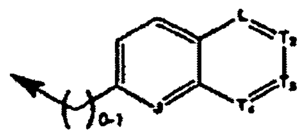

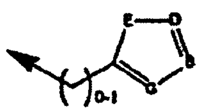
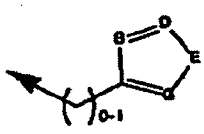
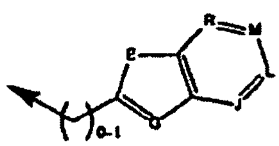
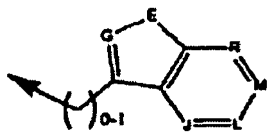

wobei J, L, M, $T_2$, $T_3$, $T_4$, B, D, G und E wie zuvor definiert sind.

**5.** Verbindung nach Anspruch 4, wobei $R^2$ ein monozyklisches Ar-$C_{0-1}$-Alkyl umfaßt und Teil einer $R^1$-Gruppe ist, ausgewählt unter:

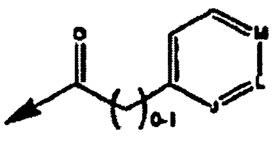
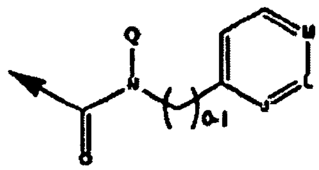
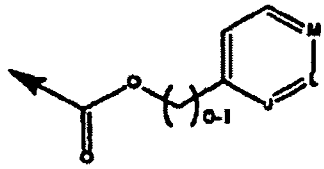

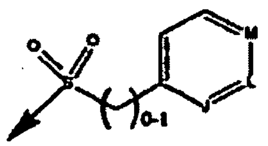
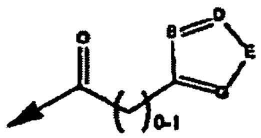
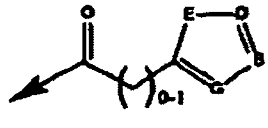

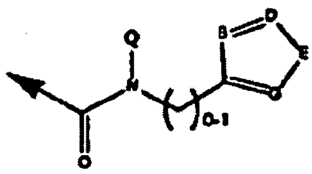
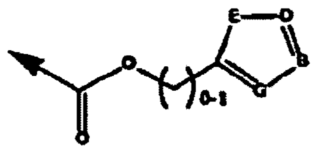

wobei:

J, L, M, B, D und G wie oben definiert sind (d.h. $CR^{21}$, N oder N→O) und wobei $R^{21}$ ausgewählt ist unter Wasserstoff, Methyl, Methoxy, Ethyl, Isopropyl,
Trifluormethyl, Trifluormethoxy, F, Cl, $SO_2Me$, und
B, D, G wie oben definiert sind, (d.h. $CR^{21}$, N oder N→O) und wobei $R^{21}$ ausgewählt ist unter Wasserstoff, Methyl, Methoxy, Ethyl, Isopropyl,
Trifluormethyl, Trifluormethoxy, F, Cl, $SO_2Me$, und
E wie zuvor definiert ist und
Q unter Wasserstoff oder Methyl ausgewählt ist.

**6.** Verbindung nach Anspruch 2, wobei $R^2$ eine $C_{3-6}$-Alkylgruppe ist, die an der α-Position verzweigt ist oder die einen $NH_2$-, NHMe-, $NHC(O)CH_3$-, $NMeC(O)CH_3$-, OH- oder OMe-Substituenten an der α-Position enthält.

**7.** Verbindung nach Anspruch 1 oder Anspruch 2, wobei $R^2$ Cyclopropyl, Cyclobutyl, Cyclopentyl, Cyclohexyl, Pyrrolidin, Piperidin, Morpholin, Tetrahydrofuran, Cyclopenten, Cyclopentadien, Cyclohexadien und Piperazin ist, wobei Stickstoff enthaltende Ringe mit Gruppen, wie $C_{1-4}$-Alkyl, Phenyl oder Benzyl, N-substituiert sein können.

**8.** Verbindung nach Anspruch 1 oder Anspruch 2, wobei $R^1$ folgendes ist:

Benzoyl, Pyridin-2-carbonyl, 1-Oxy-pyridin-2-carbonyl, Pyridin-3-carbonyl, 1-Oxy-pyridin-3-carbonyl, Pyridin-4-carbonyl, 1-Oxy-pyridin-4-carbonyl, Phenylsulfonyl, Pyridin-2-sulfonyl, 1-Oxy-pyridin-2-sulfonyl, Pyridin-3-sulfonyl, 1-Oxy-pyridin-3-sulfonyl, Pyridin-4-sulfonyl, 1-Oxy-pyridin-4-sulfonyl, Phenylacetyl, Phenylcarbamoyl, Isobutylcarbamoyl, Phenyloxycarbonyl, Isobutyloxycarbonyl, Pyrrolidin-N-carbonyl, Piperidin-N-carbonyl, Morpholin-N-carbonyl, Piperazin-N-carbonyl, 4-Methylpiperazin-N-carbonyl, (4-Methylpiperazin-1-yl)-acetoyl, Piperazin-1-yl-acetoyl, Furan-2-carbonyl, 5-Chlorfuran-2-carbonyl, Thiophen-2-carbonyl, 5-Chlorthiophen-2-carbonyl, Furan-3-carbonyl, Thiophen-3-carbonyl, Cyclopentoyl, Cyclohexoyl, Cyclopent-3-enoyl, Cyclopentylmethylcarbonyl, Cyclohexylmethylcarbonyl, Pyrrolidin-2-carbonyl, N-Acetylpyrrolidin-2-carbonyl, Piperidin-2-carbonyl, N-Acetylpiperidin-2-carbonyl, Tetrahydrofuran-2-carbonyl, 1-Aminocyclobutanoyl, 1-Aminocyclopentanoyl, 1-Aminocyclohexanoyl, N-Acetyl-1-aminocyclobutanoyl, N-Acetyl-1-aminocyclopentanoyl, N- Acetyl-1-aminocyclohexanoyl, 1-Hydroxycyclobutanoyl, 1-Hydroxycyclopentanoyl, 1-Hydroxycyclohexanoyl, 1-Methoxycyclobutanoyl, 1-Methoxycyclopentanoyl, 1-Methoxycyclohexanoyl, Aminocyclopentylacetoyl, Aminocyclohexylacetoyl, N-Acetylaminocyclopentylacetoyl, N-Acetylaminocyclohexylacetoyl, 2-Acetylaminopropionoyl, 2-Acetylaminoethanoyl, 2-Acetyl-N-methylaminoethanoyl, N,N-Dimethylaminoacetoyl, 2-Aminobutanoyl, N-Acetyl-2-aminobutanoyl, 2-Amino-3-methylbutanoyl, N-Acetyl-2-amino-3-methylbutanoyl, 2-Amino- 3,3-dimethylbutanoyl, N-Acetyl-2-amino-3,3-dimethylbutanoyl, 2-Amino-3-methylpentanoyl, N-Acetyl-2-amino-3-methylpentanoyl, Pentanoyl, 3-Methylpentanoyl, 4-Methylpentanoyl, 2-Amino-4-methylpentanoyl, N-Acetyl-2-amino-4-methylpentanoyl, 2-Amino-4,4-dimethylpentanoyl, N-Acetyl-2-amino-4,4-dimethylpentanoyl, 2- minopentanoyl, N-Acetyl-2-aminopentanoyl, 2-Amino-5-methylhexanoyl, N-Acetyl-2-amino-5-methylhexanoyl, 2-Hydroxy-3-methylbutanoyl, 2-Methoxy-3-methylbutanoyl, 2-Hydroxy-3,3-dimethylbutanoyl, 2-Methoxy- 3,3-dimethylbutanoyl, 2-Hydroxy-3-methylpentanoyl, 2-Methoxy-3-methylpentanoyl, 2-Hydroxy-4-methylpentanoyl, 2-Methoxy-4-methylpentanoyl, 2-Hydroxy-4,4-dimethylpentanoyl, 2-Methoxy-4,4-dimethylpentanoyl, 2-Hydroxypentanoyl, 2-Methoxypentanoyl, 2-Hydroxy-5-methylhexanoyl, 2-Methoxy-5-methylhexanoyl.

**9.** Verbindung nach einem der Ansprüche 2 bis 8, wobei in der Gruppe $(X)_o$ $R^{16}$ Wasserstoff ist und $R^{17}$ Wasserstoff, $C_{1-4}$-Alkyl, welches mit OH, $NR^{22}R^{22}$, $COOR^{22}$ oder $CONR^{22}$ substituiert sein kann, oder Ar-$C_{1-4}$-Alkyl ist, wobei die Arylgruppe mit $R^{21}$ substituiert sein kann, wobei jeder $R^{21}$ und $R^{22}$ unabhängig voneinander wie in Anspruch 1 definiert ist.

**10.** Verbindung nach Anspruch 9, wobei in der Gruppe $(X)_o$ $R^{16}$ Wasserstoff ist und $R^{17}$ unter Wasserstoff oder einer einfachen $C_{1-4}$-Alkylgruppe ausgewählt ist.

**11.** Verbindung nach Anspruch 10, wobei in der Gruppe $(X)_o$ $R^{16}$ und $R^{17}$ Wasserstoff sind und o null oder eins ist.

**12.** Verbindung nach einem der Ansprüche 2 bis 11, wobei in der Gruppe $(W)_n$ W O, S, $SO_2$, C(O) oder NH umfaßt und n null oder eins ist.

**13.** Verbindung nach Anspruch 12, wobei in der Gruppe $(W)_n$ W C(O) oder NH ist, wobei n null oder eins ist.

**14.** Verbindung nach Anspruch 13, wobei in der Gruppe $(W)_n$ W NH ist und n null oder eins ist.

**15.** Verbindung nach einem der Ansprüche 1 bis 14, wobei die U-$(V)_m$-$(W)_n$-$(X)_o$-Y- oder die U-$(V)_m$-$(W)_n$-$(X)_o$-Substituenten-Kombination folgendes umfaßt:

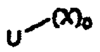

'n' = 0
'm' = 0

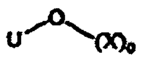

'n' = 1
'W' = O
'm' = 0

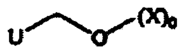

'n' = 1
'W' = O
'V' = $CH_2$
'm' = 1

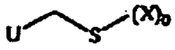

'n' = 1
'W' = S
'V' = $CH_2$
'm' = 1

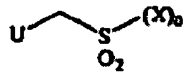

'n' = 1
'W' = $SO_2$
'V' = $CH_2$
'm' = 1

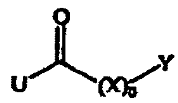

'n' = 1
'W' = C(O)
'm' = 0

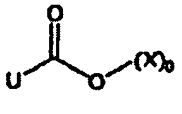

'n' = 1
'W' = O
'V' = C(O)
'm' = 1

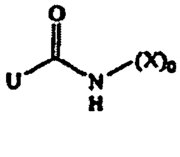

'n' = 1
'W' = $NR^{18}$, $R^{18}$ = 'H'
'V' = C(O)
'm' = 1

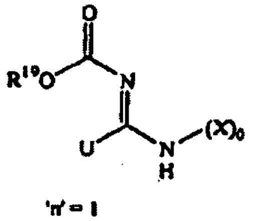

'n' = 1
'W' = $NR^{18}$, $R^{18}$ = 'H'
'V' = C=N-C(O)-$OR^{19}$
'm' = 1

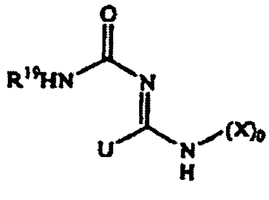

'n' = 1
'W' = $NR^{18}$, $R^{18}$ = 'H'
'V' = C=N-C(O)-$NHR^{19}$
'm' = 1

**16.** Verbindung nach einem der Ansprüche 1 bis 15, wobei die U-$(V)_m$-$(W)_n$-$(X)_o$-Y-Substituenten-Kombination folgendes umfaßt:

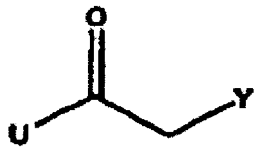

$(X)_0$ = '$CH_2$'
'n' = 1
'W' = C(O)
'm' = 0

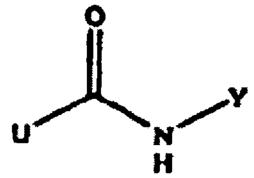

$(X)_0$ = '-'
'n' = 1
'W' = $NR^{18}$, $R^{18}$ = 'H'
'V' = C(O)
'm' = 1

**17.** Verbindung nach einem der Ansprüche 1 bis 16, welche ein Inhibitor von Cathepsin K ist und wobei unabhängig voneinander oder in irgendeiner beliebigen Kombination
Y $CHR^{11}CO$ ist, wobei $R^{11}$ ausgewählt ist unter $C_{0-7}$-Alkyl, Ar-$C_{0-7}$-Alkyl und $C_{3-6}$-Cycloalkyl oder Y eine Gruppe

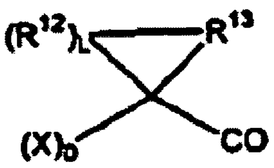

umfaßt, wobei $R^{12}$ und $R^{13}$ jeweils $CR^{14}R^{15}$ sind und jeder $R^{14}$ und $R^{15}$ unabhängig voneinander ausgewählt ist unter $C_{0-7}$-Alkyl und Ar-$C_{0-7}$Alkyl, beispielsweise Wasserstoff, einer geraden oder verzweigten Alkylkette, einer geraden oder verzweigten Heteroalkylkette, einer optional substituierten Arylalkylkette oder einer optional substituierten Arylheteroalkylkette, und L eine Zahl von eins bis vier ist und
die Gruppe U einen optional substituierten 5- oder 6-gliedrigen gesättigten oder ungesättigten Heterozyklus oder eine Ar-Gruppe oder eine optional substituierte gesättigte oder ungesättigte 8- bis 10-gliedrige heterozyklische oder Ar-Gruppe umfaßt.

**18.** Verbindung nach Anspruch 17, wobei $(X)_o$-Y folgendes ist:

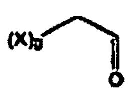 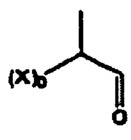 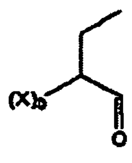 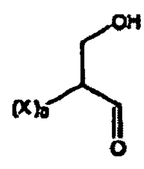 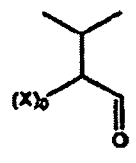 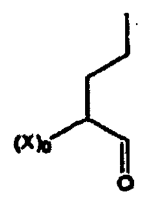 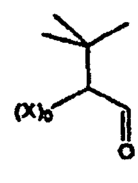

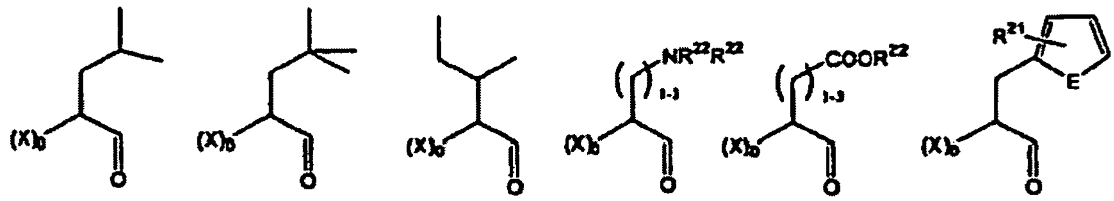

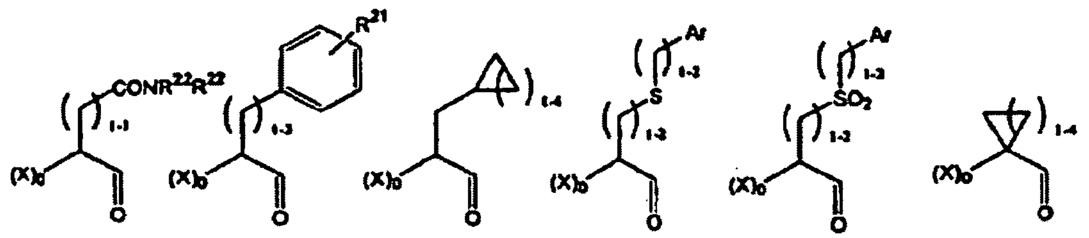

wobei E, $R^{21}$, $R^{22}$ und Ar wie zuvor definiert sind und jeder davon mit einem oder mehreren Halogen-, bevorzugt Fluor-, Substituenten substituiert sein kann.

**19.** Verbindung nach Anspruch 17, wobei in der Gruppe Y $R^{11}$ $C_{1-4}$-Alkyl ist, welches mit Cycloalkylmethyl oder Halogen substituiert sein kann, oder $R^{11}$ unter Cycloalkyl-1-carbonyl ausgewählt ist oder $R^{11}$ unter Ar-$C_{1-4}$-Alkyl ausgewählt ist, wobei die Arylgruppe mit $R^{21}$ substituiert sein kann, wobei $R^{21}$ wie in Anspruch 1 definiert ist.

**20.** Verbindung nach Anspruch 19, wobei in der Gruppe Y $R^{11}$ eine einfache gerade- oder verzweigtkettige Alkylgruppe ist, optional substituiert mit einem oder mehreren Halogensubstituenten.

**21.** Verbindung nach Anspruch 20, wobei in der Gruppe Y $R^{11}$ $ArCH_2$- ist, wobei der aromatische Ring ein optional substituierter monozyklischer Heterozyklus ist.

**22.** Verbindung nach einem der Ansprüche 17 bis 21, wobei die $(X)_o$-Y-Gruppe folgendes umfaßt:

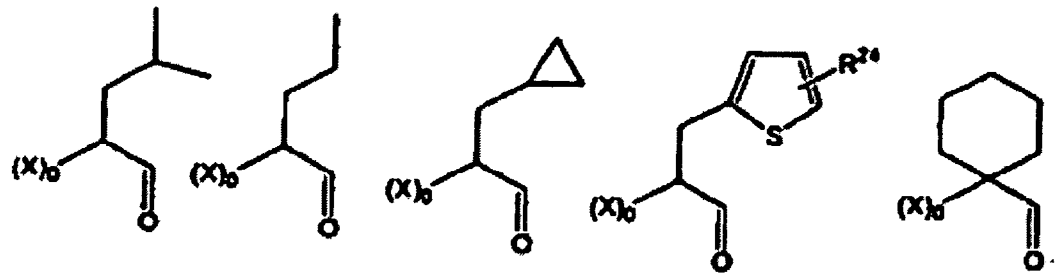

wobei $R^{24}$ unter Wasserstoff, Methyl, Methoxy, Ethyl, Isopropyl, F und Cl ausgewählt ist und wobei jede beliebige der Alkylgruppen mit einem oder mehreren F oder Cl substituiert sein kann.

**23.** Verbindung nach einem der Ansprüche 17 bis 22, wobei die Gruppe U folgendes umfaßt:

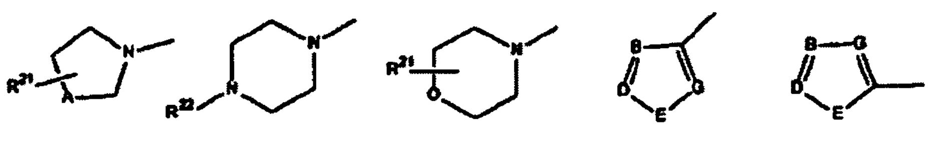

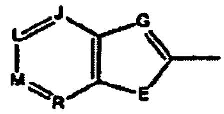 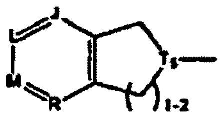 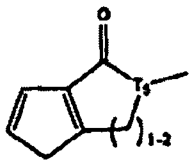 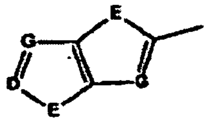 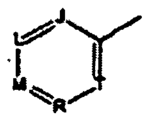

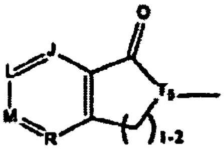 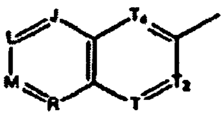 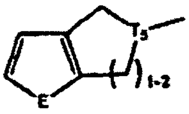 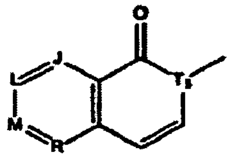

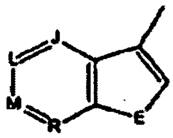 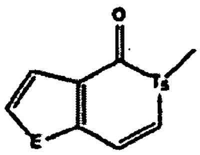 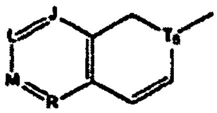 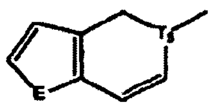

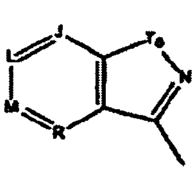 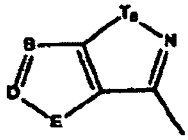 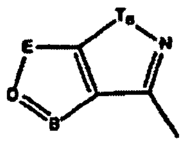

wobei $R^{21}$, $R^{22}$, A, B, D, E, G, J, L, M, R, T, $T_2$, $T_4$, $T_5$ und $T_6$ wie in Anspruch 1 definiert sind.

**24.** Verbindung nach Anspruch 23, wobei U folgendes umfaßt:

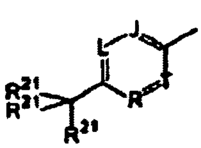 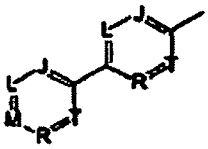 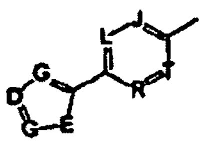 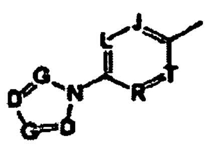 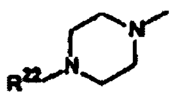

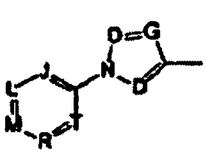 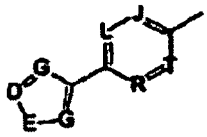 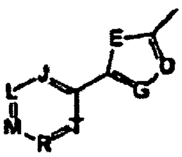 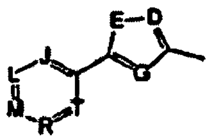 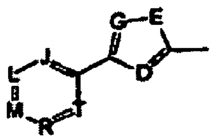

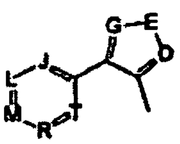 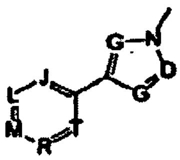 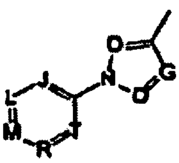 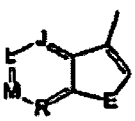 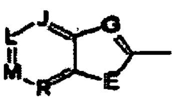

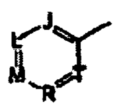
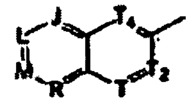
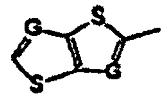

wobei $R^{21}$, $R^{22}$, D, E, G, J, L, M, R, T, $T_2$ und $T_4$ wie zuvor definiert sind.

**25.** Verbindung nach Anspruch 24, wobei U folgendes umfaßt:

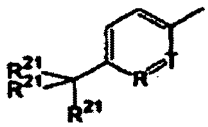
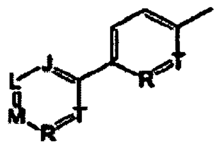
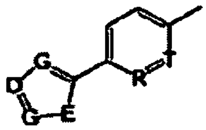
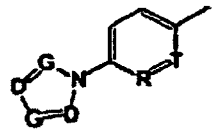
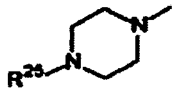

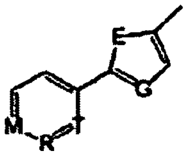
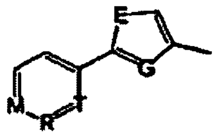
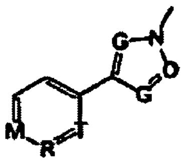
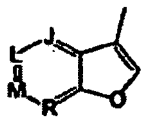
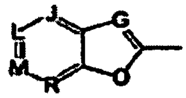

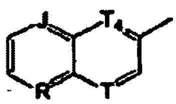
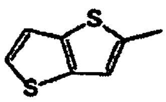
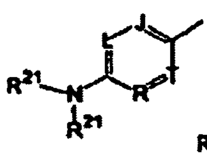
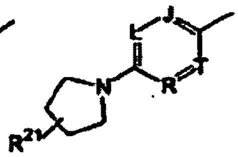
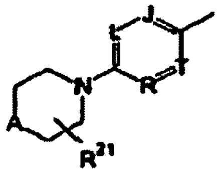

wobei $R^{21}$, $R^{25}$, D, E, G, J, L, M, R, T und $T_4$ wie in Anspruch 1 definiert sind.

**26.** Verbindung nach Anspruch 25, wobei U folgendes umfaßt:

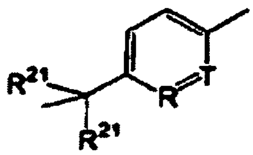
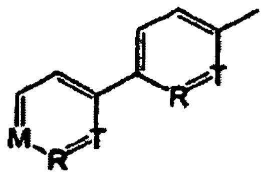
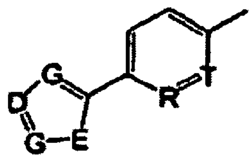
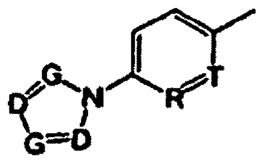

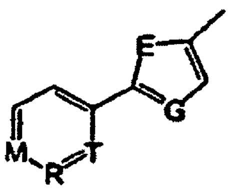
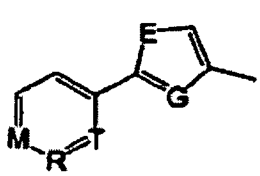
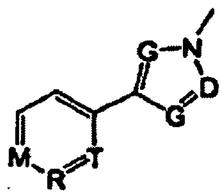

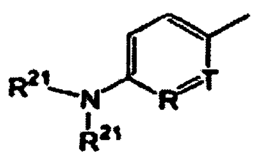

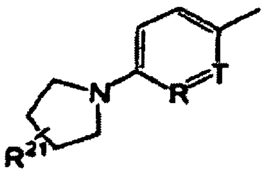

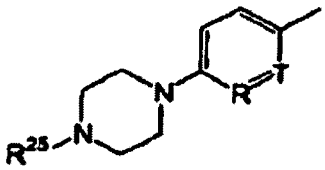

wobei $R^{21}$, $R^{25}$, D, E, G, M, R und T wie in Anspruch 1 definiert sind.

**27.** Verbindung nach einem der Ansprüche 1 bis 16, welche ein Inhibitor von Cathepsin S ist und wobei unabhängig voneinander oder in irgendeiner beliebigen Kombination $(X)_o$-Y folgendes umfaßt:

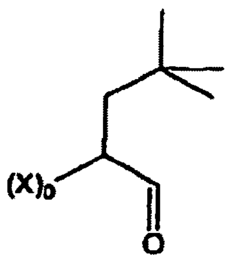

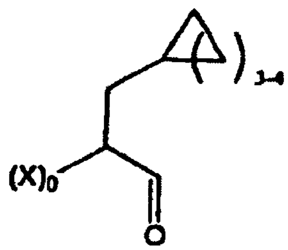

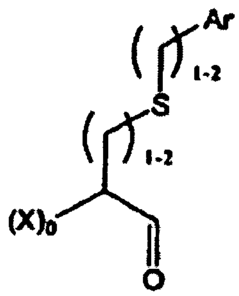

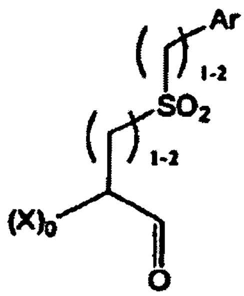

wobei $(X)_o$ wie in Anspruch 1 definiert ist und
U folgendes umfaßt:

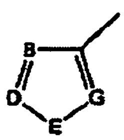

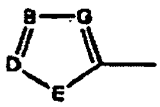

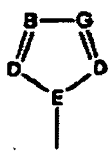

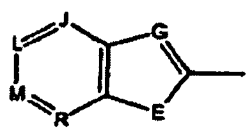

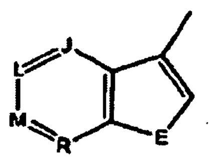

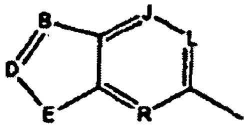

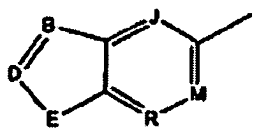

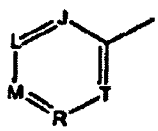

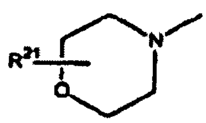

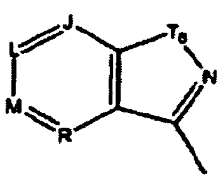

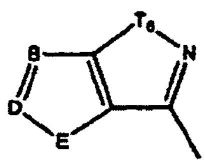

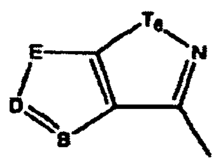

wobei $R^{21}$, B, D, E, G, J, L, M, R und $T_6$ wie in Anspruch 1 definiert sind.

**28.** Verbindung nach Anspruch 27, wobei U einen optional substituierten 5-gliedrigen ungesättigten Heterozyklus oder einen 6,5- oder 5,5-fusionierten aromatischen Ring umfaßt.

**29.** Verbindung nach Anspruch 28, wobei U folgendes umfaßt:

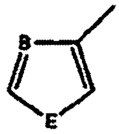
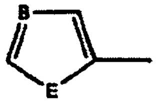
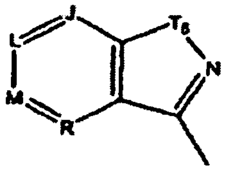
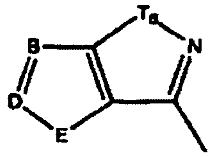
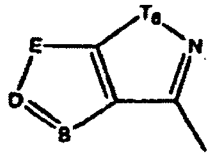

wobei B, D, E, J, L, M, R und $T_6$ wie in Anspruch 1 definiert sind.

**30.** Verbindung nach einem der Ansprüche 1 bis 24, welche ein Inhibitor von Cathepsin L ist und wobei unabhängig voneinander oder in irgendeiner beliebigen Kombination $(X)_o$-Y folgendes umfaßt:

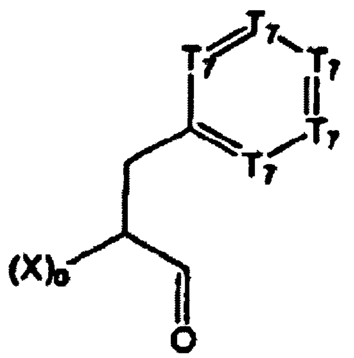

wobei $T_7$ unter CH, N oder $CR^{21}$ ausgewählt ist, wobei $R^{21}$ wie in Anspruch 1 definiert ist, und U einen optional substituierten 5-gliedrigen ungesättigten Heterozyklus oder einen 6,6-oder 6,5- oder 5,5-fusionierten aromatischen Ring oder einen meta-substituierten Ar umfaßt.

**31.** Verbindung nach Anspruch 30, wobei innerhalb des $T_7$-Substituenten der $R^{21}$-Substituent unter einzelnen und mehrfachen Ringsubstitutionskombinationen von Me, F, Cl, OH und OMe ausgewählt ist.

**32.** Verbindung nach Anspruch 30 oder Anspruch 31, wobei U folgendes umfaßt:

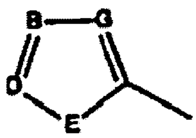
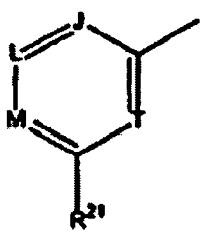
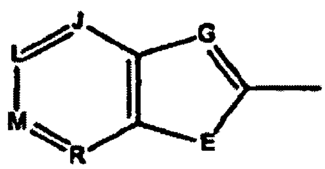

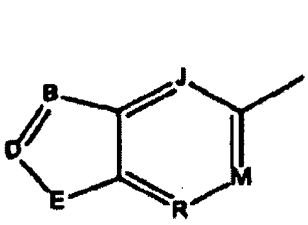 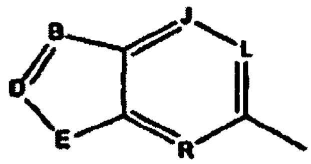 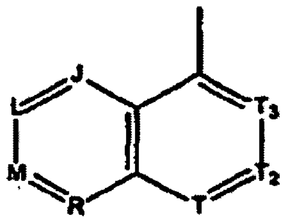

wobei $R^{21}$, B, D, E, G, J, L, M, R, T, $T_2$ und $T_3$ wie in Anspruch 1 definiert sind.

**33.** Verbindung nach einem der Ansprüche 30 bis 32, wobei U folgendes umfaßt:

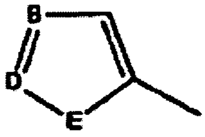 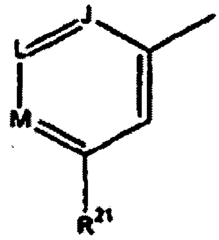 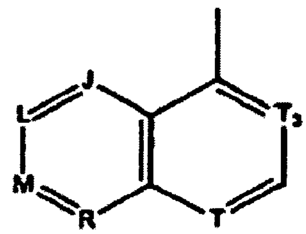

wobei E unter Sauerstoff oder N-Ethyl ausgewählt ist, D unter Stickstoff oder $CCH_3$ ausgewählt ist, B unter Stickstoff oder $CCH_3$ ausgewählt ist, $R^{21}$ unter Halogen, OMe, $CF_3$, $OCF_3$, $CH_2NH_2$ ausgewählt ist und J, L, M, R, T und $T_3$ wie in Anspruch 1 definiert sind.

**34.** Verbindung nach einem der Ansprüche 1 bis 33, welche ein bizyklisches cis-Isomer ist.

**35.** Verbindung, ausgewählt unter:
(3aR,6aS)-N-{(1S)-3-Methyl-1-[6-oxo-4-(pyridin-2-carbonyl)-hexahydro-pyrrolo[3,2-b]pyrrol-1-carbonyl]-butyl}-benzamid,
(3aR,6aS)-N-{(1S)-3-Methyl-1-[6-oxo-4-(pyridin-2-carbonyl)-hexahydro-pyrrolo[3,2-c]pyrazol-1-carbonyl]-butyl}-benzamid,
(3aS,6aS)-N-{(1S)-3-Methyl-1-[6-oxo-4-(pyridin-2-carbonyl)-hexahydro-2-oxa-1,4-diaza-pentalen-1-carbonyl]-butyl}-benzamid,
Naphthalen-2-carbonsäure-{3-methyl-1-[6-oxo-4-(pyridin-2-sulfonyl)-hexahydro-pyrrolo[3,2-b]pyrrol-1-carbonyl]-butylfamid,
Chinolin-2-carbonsäure-{3-methyl-1-[6-oxo-4-(pyridin-2-sulfonyl)-hexahydro-pyrrolo[3,2-b]pyrrol-1-carbonyl]-butyl}-amid,
Benzo[b]thiophen-2-carbonsäure-{3-methyl-1-[6-oxo-4-(pyridin-2-sulfonyl)-hexahydro-pyrrolo[3,2-b]pyrrol-1-carbonyl]-butyl}-amid,
Benzofuran-2-carbonsäure-{3-methyl-1-[6-oxo-4-(pyridin-2-sulfonyl)-hexahydro-pyrrolo[3,2-b]pyrrol-1-carbonyl]-butyl}-amid,
Biphenyl-4-carbonsäure-{3-methyl-1-[6-oxo-4-(pyridin-2-sulfonyl)-hexahydro-pyrrolo[3,2-b]pyrrol-1-carbonyl]-butyl}-amid,
4-tert-Butyl-N-{3-methyl-1-[6-oxo-4-(pyridin-2-sulfonyl)-hexahydro-pyrrolo[3,2-b]pyrrol-1-carbonyl]-butyl}-benzamid,
4-Dimethylamino-N-{3-methyl-1-[6-oxo-4-(pyridin-2-sulfonyl)-hexahydro-pyrrolo[3,2-b]pyrrol-1-carbonyl]-butyl}-benzamid,
7-Methoxy-benzofuran-2-carbonsäure-{3-methyl-1-[6-oxo-4-(pyridin-2-sulfonyl)-hexahydro-pyrrolo[3,2-b]pyrrol-1-carbonyl]-butyl}-amid,
{3-Methyl-1-[6-oxo-4-(pyridin-2-sulfonyl)-hexahydro-pyrrolo[3,2-b]pyrrol-1-carbonyl]-butyl}-carbaminsäurebenzylester,
5-Methoxy-benzofuran-2-carbonsäure-{3-methyl-1-[6-oxo-4-(pyridin-2-sulfonyl)-hexahydro-pyrrolo[3,2-b]pyrrol-1-carbonyl]-butyl}-amid,

Thieno[3,2-b]thiophen-2-carbonsäure-{3-methyl-1-[6-oxo-4-(pyridin-2-sulfonyl)-hexahydro-pyrrolo[3,2-b]pyrrol-1-carbonyl]-butyl}-amid,
3-Methyl-benzofuran-2-carbonsäure-{3-methyl-1-[6-oxo-4-(pyridin-2-sulfonyl)-hexahydro-pyrrolo[3,2-b]pyrrol-1-carbonyl]-butyl}-amid,
Chinoxalin-2-carbonsäure-{3-methyl-1-[6-oxo-4-(pyridin-2-sulfonyl)-hexahydro-pyrrolo[3,2-b]pyrrol-1-carbonyl]-butyl}-amid,
Benzo[1,3]dioxol-5-carbonsäure-{3-methyl-1-[6-oxo-4-(pyridin-2-sulfonyl)-hexahydro-pyrrolo[3,2-b]pyrrol-1-carbonyl]-butyl}-amid,
Chinolin-6-carbonsäure-{3-methyl-1-[6-oxo-4-(pyridin-2-sulfonyl)-hexahydro-pyrrolo[3,2-b]pyrrol-1-carbonyl]-butyl}-amid,
Furan-2-carbonsäure-{3-methyl-1-[6-oxo-4-(pyridin-2-sulfonyl)-hexahydro-pyrrolo[3,2-b]pyrrol-1-carbonyl]-butyl}-amid,
Thiophen-2-carbonsäure-{3-methyl-1-[6-oxo-4-(pyridin-2-sulfonyl)-hexahydro-pyrrolo[3,2-b]pyrrol-1-carbonyl]-butyl}-amid,
N-{3-Methyl-1-[6-oxo-4-(pyridin-2-sulfonyl)-hexahydro-pyrrolo[3,2-b]pyrrol-1-carbonyl]-butyl}-4-trifluormethyl-benzamid,
Furan-3-carbonsäure-{3-methyl-1-[6-oxo-4-(pyridin-2-sulfonyl)-hexahydro-pyrrolo[3,2-b]pyrrol-1-carbonyl]-butyl}-amid,
Thiophen-3-carbonsäure-{3-methyl-1-[6-oxo-4-(pyridin-2-sulfonyl)-hexahydro-pyrrolo[3,2-b]pyrrol-1-carbonyl]-butyl}-amid,
N-{3-Methyl-1-[6-oxo-4-(pyridin-2-sulfonyl)-hexahydro-pyrrolo[3,2-b]pyrrol-1-carbonyl]-butyl}-4-phenoxy-benzamid,
N-{3-Methyl-1-[6-oxo-4-(pyridin-2-sulfonyl)-hexahydro-pyrrolo[3,2-b]pyrrol-1-carbonyl]-butyl}-4-(4-methyl-piperazin-1-yl)-benzamid,
4-Methyl-N-{3-methyl-1-[6-oxo-4-(pyridin-2-sulfonyl)-hexahydro-pyrrolo[3,2-b]pyrrol-1-carbonyl]-butyl}-benzamid,
4-Methoxy-N-{3-methyl-1-[6-oxo-4-(pyridin-2-sulfonyl)-hexahydro-pyrrolo[3,2-b]pyrrol-1-carbonyl]-butyl}-benzamid,
4-Isopropyl-N-{3-methyl-1-[6-oxo-4-(pyridin-2-sulfonyl)-hexahydro-pyrrolo[3,2-b]pyrrol-1-carbonyl]-butyl}-benzamid,
N-{3-Methyl-1-[6-oxo-4-(pyridin-2-sulfonyl)-hexahydro-pyrrolo[3,2-b]pyrrol-1-carbonyl]-butyl}-4-vinyl-benzamid,
4-Imidazol-1-yl-N-{3-methyl-1-[6-oxo-4-(pyridin-2-sulfonyl)-hexahydro-pyrrolo[3,2-b]pyrrol-1-carbonyl]-butyl}-benzamid,
N-{3-Methyl-1-[6-oxo-4-(pyridin-2-sulfonyl)-hexahydro-pyrrolo[3,2-b]pyrrol-1-carbonyl]-butyl}4-thiophen-2-yl-benzamid,
N-{3-Methyl-1-[6-oxo-4-(pyridin-2-sulfonyl)-hexahydro-pyrrolo[3,2-b]pyrrol-1-carbonyl]-butyl}-4-oxazol-5-yl-benzamid,
5-Phenyl-thiophen-2-carbonsäure-{3-methyl-1-[6-oxo-4-(pyridin-2-sulfonyl)-hexahydro-pyrrolo[3,2-b]pyrrol-1-carbonyl]-butyl}-amid,
N-{3-Methyl-1-[6-oxo-4-(pyridin-2-sulfonyl)-hexahydro-pyrrolo[3,2-b]pyrrol-1-carbonyl]-butyl}-4-trifluormethoxy-benzamid,
5-Pyridin-2-yl-thiophen-2-carbonsäure-{3-methyl-1-[6-oxo-4-(pyridin-2-sulfonyl)-hexahydro-pyrrolo[3,2-b]pyrrol-1-carbonyl]-butyl}-amid,
4-Difluormethoxy-N-{3-methyl-1-[6-oxo-4-(pyridin-2-sulfonyl)-hexahydro-pyrrolo[3,2-b]pyrrol-1-carbonyl]-butyl}-benzamid,
N-{3-Methyl-1-[6-oxo-4-(pyridin-2-sulfonyl)-hexahydro-pyrrolo[3,2-b]pyrrol-1-carbonyl]-butyl}-4-morpholin-4-yl-benzamid,
Naphthalen-2-carbonsäure-{1-[6-oxo-4-(pyridin-2-sulfonyl)-hexahydro-pyrrolo[3,2-b]pyrrol-1-carbonyl]-butyl}-amid,
Chinolin-2-carbonsäure-{1-[6-oxo-4-(pyridin-2-sulfonyl)-hexahydro-pyrrolo[3,2-b]pyrrol-1-carbonyl]-butyl}-amid,
Benzo[b]thiophen-2-carbonsäure-{1-[6-oxo-4-(pyridin-2-sulfonyl)-hexahydro-pyrrolo[3,2-b]pyrrol-1-carbonyl]-butyl}-amid,
Benzofuran-2-carbonsäure-{1-[6-oxo-4-(pyridin-2-sulfonyl)-hexahydro-pyrrolo[3,2-b]pyrrol-1-carbonyl]-butyl}-amid,
Biphenyl-4-carbonsäure-{1-[6-oxo-4-(pyridin-2-sulfonyl)-hexahydro-pyrrolo[3,2-b]pyrrol-1-carbonyl]-butyl}-amid,
4-tert-Butyl-N-{1-[6-oxo-4-(pyridin-2-sulfonyl)-hexahydro-pyrrolo[3,2-b]pyrrol-1-carbonyl]-butyl}-benzamid,
4-Dimethylamino-N-{1-[6-oxo-4-(pyridin-2-sulfonyl)-hexahydro-pyrrolo[3,2-b]pyrrol-1-carbonyl]-butyl}-benzamid,
7-Methoxy-benzofuran-2-carbonsäure-{1-[6-oxo-4-(pyridin-2-sulfonyl)-hexahydro-pyrrolo[3,2-b]pyrrol-1-carbonyl]-butyl}-amid,

{1-[6-oxo-4-(pyridin-2-sulfonyl)-hexahydro-pyrrolo[3,2-b]pyrrol-1-carbonyl]-butyl}-carbaminsäurebenzylester,
5-Methoxy-benzofuran-2-carbonsäure-{1-[6-oxo-4-(pyridin-2-sulfonyl)-hexahydro-pyrrolo[3,2-b]pyrrol-1-carbonyl]-butyl}-amid,
Thieno[3,2-b]thiophen-2-carbonsäure-{1-[6-oxo-4-(pyridin-2-sulfonyl)-hexahydro-pyrrolo[3,2-b]pyrrol-1-carbonyl]-butyl}-amid,
3-Methyl-benzofuran-2-carbonsäure-{1-[6-oxo-4-(pyridin-2-sulfonyl)-hexahydro-pyrrolo[3,2-b]pyrrol-1-carbonyl]-butyl}-amid,
Chinoxalin-2-carbonsäure-{1-[6-oxo-4-(pyridin-2-sulfonyl)-hexahydro-pyrrolo[3,2-b]pyrrol-1-carbonyl]-butyl}-amid,
Benzo[1,3]dioxol-5-carbonsäure-{1-[6-oxo-4-(pyridin-2-sulfonyl)-hexahydro-pyrrolo[3,2-b]pyrrol-1-carbonyl]-butyl}-amid,
Chinolin-6-carbonsäure-{1-[6-oxo-4-(pyridin-2-sulfonyl)-hexahydro-pyrrolo[3,2-b]pyrrol-1-carbonyl]-butyl}-amid,
Furan-2-carbonsäure-{1-[6-oxo-4-(pyridin-2-sulfonyl)-hexahydro-pyrrolo[3,2-b]pyrrol-1-carbonyl]-butyl}-amid,
Thiophen-2-carbonsäure-{1-[6-oxo-4-(pyridin-2-sulfonyl)-hexahydro-pyrrolo[3,2-b]pyrrol-1-carbonyl]-butyl}-amid,
N-{1-[6-oxo-4-(pyridin-2-sulfonyl)-hexahydro-pyrrolo[3,2-b]pyrrol-1-carbonyl]-butyl}-4-trifluormethyl-benzamid,
Furan-3-carbonsäure-{1-[6-oxo-4-(pyridin-2-sulfonyl)-hexahydro-pyrrolo[3,2-b]pyrrol-1-carbonyl]-butyl}-amid,
Thiophen-3-carbonsäure-{1-[6-oxo-4-(pyridin-2-sulfonyl)-hexahydro-pyrrolo[3,2-b]pyrrol-1-carbonyl]-butyl}-amid,
N-{1-[6-oxo-4-(pyridin-2-sulfonyl)-hexahydro-pyrrolo[3,2-b]pyrrol-1-carbonyl]-butyl}-4-phenoxy-benzamid,
N-{1-[6-oxo-4-(pyridin-2-sulfonyl)-hexahydro-pyrrolo[3,2-b]pyrrol-1-carbonyl]-butyl}-4-(4-methylpiperazin-1-yl)-benzamid,
4-Methyl-N-{1-[6-oxo-4-(pyridin-2-sulfonyl)-hexahydro-pyrrolo[3,2-b]pyrrol-1-carbonyl]-butyl}-benzamid,
4-Methoxy-N-{1-[6-oxo-4-(pyridin-2-sulfonyl)-hexahydro-pyrrolo[3,2-b]pyrrol-1-carbonyl]-butyl}-benzamid,
4-Isopropyl-N-{1-[6-oxo-4-(pyridin-2-sulfonyl)-hexahydro-pyrrolo[3,2-b]pyrrol-1-carbonyl]-butyl}-benzamid,
N-{1-[6-oxo-4-(pyridin-2-sulfonyl)-hexahydro-pyrrolo[3,2-b]pyrrol-1-carbonyl]-butyl}-4-vinyl-benzamid,
4-Imidazol-1-yl-N-{1-[6-oxo-4-(pyridin-2-sulfonyl)-hexahydro-pyrrolo[3,2-b]pyrrol-1-carbonyl]-butyl}-benzamid,
N-{1-[6-oxo-4-(pyridin-2-sulfonyl)-hexahydro-pyrrolo[3,2-b]pyrrol-1-carbonyl]-buty}-4-thiophen-2-yl-benzamid,
N-{1-[6-oxo-4-(pyridin-2-sulfonyl)-hexahydro-pyrrolo[3,2-b]pyrrol-1-carbonyl]-butyl}-4-oxazol-5-yl-benzamid,
5-Phenyl-thiophen-2-carbonsäure-{1-[6-oxo-4-(pyridin-2-sulfonyl)-hexahydro-pyrrolo[3,2-b]pyrrol-1-carbonyl]-butyl}-amid,
N-{1-[6-oxo-4-(pyridin-2-sulfonyl)-hexahydro-pyrrolo[3,2-b]pyrrol-1-carbonyl]-butyl}-4-trifluormethoxy-benzamid,
5-Pyridin-2-yl-thiophen-2-carbonsäure-{1-[6-oxo-4-(pyridin-2-sulfonyl)-hexahydro-pyrrolo[3,2-b]pyrrol-1-carbonyl]-butyl}-amid,
4-Difluormethoxy-N-{1-[6-oxo-4-(pyridin-2-sulfonyl)-hexahydro-pyrrolo[3,2-b]pyrrol-1-carbonyl]-butyl}-benzamid,
N-{1-[6-Oxo-4-(pyridin-2-sulfonyl)-hexahydro-pyrrolo[3,2-b]pyrrol-1-carbonyl]-butyl}-4-morpholin-4-yl-benzamid,
Naphthalen-2-carbonsäure-{3-methyl-1-[6-oxo-4-(1-oxy-pyridin-2-sulfonyl)-hexahydro-pyrrolo[3,2-b]pyrrol-1-carbonyl]-butyl)-amid,
Chinolin-2-carbonsäure-{3-methyl-1-[6-oxo-4-(1-oxy-pyridin-2-sulfonyl)-hexahydro-pyrrolo[3,2-b]pyrrol-1-carbonyl]-butyl}-amid,
Benzo[b]thiophen-2-carbonsäure-{3-methyl-1-[6-oxo-4-(1-oxy-pyridin-2-sulfonyl)-hexahydro-pyrrolo[3,2-b]pyrrol-1-carbonyl]-butyl}-amid,
Benzofuran-2-carbonsäure-{3-methyl-1-[6-oxo-4-(1-oxy-pyridin-2-sulfonyl)-hexahydro-pyrrolo[3,2-b]pyrrol-1-carbonyl]-butyl}-amid,
Biphenyl-4-carbonsäure-{3-methyl-1-[6-oxo-4-(1-oxy-pyridin-2-sulfonyl)-hexahydro-pyrrolo[3,2-b]pyrrol-1-carbonyl]-butyl}-amid,
4-tert-Butyl-N-{3-methyl-1-[6-oxo-4-(1-oxy-pyridin-2-sulfonyl)-hexahydro-pyrrolo[3,2-b]pyrrol-1-carbonyl]-butyl}-benzamid,
4-Dimethylamino-N-{3-methyl-1-[6-oxo-4-(1-oxy-pyridin-2-sulfonyl)-hexahydro-pyrrolo[3,2-b]pyrrol-1-carbonyl]-butyl}-benzamid,
7-Methoxy-benzofuran-2-carbonsäure-{3-methyl-1-[6-oxo-4-(1-oxy-pyridin-2-sulfonyl)-hexahydro-pyrrolo[3,2-b]pyrrol-1-carbonyl]-butyl}-amid,
{3-Methyl-1-[6-oxo-4-(1-oxy-pyridin-2-sulfonyl)-hexahydro-pyrrolo[3,2-b]pyrrol-1-carbonyl]-butyl}-carbaminsäurebenzylester,
5-Methoxy-benzofuran-2-carbonsäure-{3-methyl-1-[6-oxo-4-(1-oxy-pyridin-2-sulfonyl)-hexahydro-pyrrolo[3,2-b]pyrrol-1-carbonyl]-butyl}-amid,
Thieno[3,2-b]thiophen-2-carbonsäure-{3-methyl-1-[6-oxo-4-(1-oxy-pyridin-2-sulfonyl)-hexahydro-pyrrolo[3,2-b]pyrrol-1-carbonyl]-butyl}-amid,
3-Methyl-benzofuran-2-carbonsäure-{3-methyl-1-[6-oxo-4-(1-oxy-pyridin-2-sulfonyl)-hexahydro-pyrrolo[3,2-b]pyrrol-1-carbonyl]-butyl}-amid,
Chinoxalin-2-carbonsäure-{3-methyl-1-[6-oxo-4-(1-oxy-pyridin-2-sulfonyl)-hexahydro-pyrrolo[3,2-b]pyrrol-1-carbonyl]-butyl]-amid,
Benzo[1,3]dioxol-5-carbonsäure-{3-methyl-1-[6-oxo-4-(1-oxy-pyridin-2-sulfonyl)-hexahydro-pyrrolo[3,2-b]pyrrol-1-carbonyl]-butyl}-amid,
Chinolin-6-carbonsäure-{3-methyl-1-[6-oxo-4-(1-oxy-pyridin-2-sulfonyl)-hexahydro-pyrrolo[3,2-b]pyrrol-1-carbonyl]-butyl}-amid,
Furan-2-carbonsäure-{3-methyl-1-[6-oxo-4-(1-oxy-pyridin-2-sulfonyl)-hexahydro-pyrrolo[3,2-b]pyrrol-1-carbonyl]-butyl}-amid,
Thiophen-2-carbonsäure-{3-methyl-1-[6-oxo-4-(1-oxy-pyridin-2-sulfonyl)-hexahydro-pyrrolo[3,2-b]pyrrol-1-carbonyl]-butyl}-amid,
N-{3-Methyl-1-[6-oxo-4-(1-oxy-pyridin-2-sulfonyl)-hexahydro-pyrrolo[3,2-b]pyrrol-1-carbonyl]-butyl}-4-trifluormethyl-benzamid,
Furan-3-carbonsäure-{3-methyl-1-[6-oxo-4-(1-oxy-pyridin-2-sulfonyl)-hexahydro-pyrrolo[3,2-b]pyrrol-1-carbonyl]-butyl}-amid,
Thiophen-3-carbonsäureg3-methyl-1-[6-oxo-4-(1-oxy-pyridin-2-sulfonyl)-hexahydro-pyrrolo[3,2-b]pyrrol-1-carbonyl]-butyl}-amid,
N-{3-Methyl-1-[6-oxo-4-(1-oxy-pyridin-2-sulfonyl)-hexahydro-pyrrolo[3,2-b]pyrrol-1-carbonyl]-butyl}-4-phenoxy-benzamid,
N-{3-Methyl-1-[6-oxo-4-(1-oxy-pyridin-2-sulfonyl)-hexahydro-pyrrolo[3,2-b]pyrrol-1-carbonyl]-butyl}-4-(4-methyl-piperazin-1-yl)-benzamid,
4-Methyl-N-{3-methyl-1-[6-oxo-4-(1-oxy-pyridin-2-sulfonyl)-hexahydro-pyrrolo[3,2-b]pyrrol-1-carbonyl]-butyl}-benzamid,
4-Methoxy-N-{3-methyl-1-[6-oxo-4-(1-oxy-pyridin-2-sulfonyl)-hexahydro-pyrrolo[3,2-b]pyrrol-1-carbonyl]-butyl}-benzamid,
4-Isopropyl-N-{3-methyl-1-[6-oxo-4-(1-oxy-pyridin-2-sulfonyl)-hexahydro-pyrrolo[3,2-b]pyrrol-1-carbonyl]-butyl}-benzamid,
N-{3-Methyl-1-[6-oxo-4-(1-oxy-pyridin-2-sulfonyl)-hexahydro-pyrrolo[3,2-b]pyrrol-1-carbonyl]-butyl}-4-vinyl-benzamid,
4-Imidazol-1-yl-N-{3-methyl-1-[6-oxo-4-(1-oxy-pyridin-2-sulfonyl)-hexahydro-pyrrolo[3,2-b]pyrrol-1-carbonyl]-butyl}-benzamid,
N-{3-Methyl-1-[6-oxo-4-(1-oxy-pyridin-2-sulfonyl)-hexahydro-pyrrolo[3,2-b]pyrrol-1-carbonyl]-butyl}-4-thiophen-2-yl-benzamid,
N-{3-Methyl-1-[6-oxo-4-(1-oxy-pyridin-2-sulfonyl)-hexahydro-pyrrolo[3,2-b]pyrrol-1-carbonyl]-butyl}-4-oxazol-5-yl-benzamid,
5-Phenyl-thiophen-2-carbonsäure-{3-methyl-1-[6-oxo-4-(1-oxy-pyridin-2-sulfonyl)-hexahydro-pyrrolo[3,2-b]pyrrol-1-carbonyl]-butyl}-amid,
N-{3-Methyl-1-[6-oxo-4-(1-oxy-pyridin-2-sulfonyl)-hexahydro-pyrrolo[3,2-b]pyrrol-1-carbonyl]-butyl}-4-trifluormethoxy-benzamid,
5-Pyridin-2-yl-thiophen-2-carbonsäure-{3-methyl-1-[6-oxo-4-(1-oxy-pyridin-2-sulfonyl)-hexahydro-pyrrolo[3,2-b]pyrrol-1-carbonyl]-butyl}-amid,
4-Difluormethoxy-N-{3-methyl-1-[6-oxo-4-(1-oxy-pyridin-2-sulfonyl)-hexahydro-pyrrolo[3,2-b]pyrrol-1-carbonyl]-butyl}-benzamid,
N-{3-Methyl-1-[6-oxo-4-(1-oxy-pyridin-2-sulfonyl)-hexahydro-pyrrolo[3,2-b]pyrrol-1-carbonyl]-butyl}-4-morpholin-4-yl-benzamid,
Naphthalen-2-carbonsäure-{1-[6-oxo-4-(1-oxy-pyridin-2-sulfonyl)-hexahydro-pyrrolo[3,2-b]pyrrol-1-carbonyl]-butyl}-amid,
Chinolin-2-carbonsäure-{1-[6-oxo-4-(1-oxy-pyridin-2-sulfonyl)-hexahydro-pyrrolo[3,2-b]pyrrol-1-carbonyl]-butyl}-amid,
Benzo[b]thiophen-2-carbonsäure-{1-[6-oxo-4-(1-oxy-pyridin-2-sulfonyl)-hexahydro-pyrrolo[3,2-b]pyrrol-1-carbonyl]-butyl}-amid,
Benzofuran-2-carbonsäure-{1-[6-oxo-4-(1-oxy-pyridin-2-sulfonyl)-hexahydro-pyrrolo[3,2-b]pyrrol-1-carbonyl]-butyl}-amid,
Biphenyl-4-carbonsäure-{1-[6-oxo-4-(1-oxy-pyridin-2-sulfonyl)-hexahydro-pyrrolo[3,2-b]pyrrol-1-carbonyl]-butyl}-amid,
4-tert-Butyl-N-{1-[6-oxo-4-(1-oxy-pyridin-2-sulfonyl)-hexahydro-pyrrolo[3,2-b]pyrrol-1-carbonyl]-butyl}-benzamid,
4-Dimethylamino-N-{1-[6-oxo-4-(1-oxy-pyridin-2-sulfonyl)-hexahydro-pyrrolo[3,2-b]pyrrol-1-carbonyl]-butyl}-benzamid,
7-Methoxy-benzofuran-2-carbonsäure-{1-[6-oxo-4-(1-oxy-pyridin-2-sulfonyl)-hexahydro-pyrrolo[3,2-b]pyrrol-1-carbonyl]-butyl}-amid,

{1-[6-oxo-4-(1-oxy-pyridin-2-sulfonyl)-hexahydro-pyrrolo[3,2-b]pyrrol-1-carbonyl]-butyl}-carbaminsäurebenzylester,
5-Methoxy-benzofuran-2-carbonsäure-{1-[6-oxo-4-(1-oxy-pyridin-2-sulfonyl)-hexahydro-pyrrolo[3,2-b]pyrrol-1-carbonyl]-butyl}-amid,
Thieno[3,2-b]thiophen-2-carbonsäure-{1-[6-oxo-4-(1-oxy-pyridin-2-sulfonyl)-hexahydro-pyrrolo[3,2-b]pyrrol-1-carbonyl]-butyl}-amid,
3-Methyl-benzofuran-2-carbonsäure-{1-[6-oxo-4-(1-oxy-pyridin-2-sulfonyl)-hexahydro-pyrrolo[3,2-b]pyrrol-1-carbonyl]-butyl}-amid,
Chinoxalin-2-carbonsäure-{1-[6-oxo-4-(1-oxy-pyridin-2-sulfonyl)-hexahydro-pyrrolo[3,2-b]pyrrol-1-carbonyl]-butyl}-amid,
Benzo[1,3]dioxol-5-carbonsäure-{1-[6-oxo-4-(1-oxy-pyridin-2-sulfonyl)-hexahydro-pyrrolo[3,2-b]pyrrol-1-carbonyl]-butyl}-amid,
Chinolin-6-carbonsäure-{1-[6-oxo-4-(1-oxy-pyridin-2-sulfonyl)-hexahydro-pyrrolo[3,2-b]pyrrol-1-carbonyl]-butyl}-amid,
Furan-2-carbonsäure-{1-[6-oxo-4-(1-oxy-pyridin-2-sulfonyl)-hexahydro-pyrrolo[3,2-b]pyrrol-1-carbonyl]-butyl}-amid,
Thiophen-2-carbonsäure-{1-[6-oxo-4-(1-oxy-pyridin-2-sulfonyl)-hexahydro-pyrrolo[3,2-b]pyrrol-1-carbonyl]-butyl}-amid,
N-{1-[6-oxo-4-(1-oxy-pyridin-2-sulfonyl)-hexahydro-pyrrolo[3,2-b]pyrrol-1-carbonyl]-butyl}-4-trifluormethyl-benzamid,
Furan-3-carbonsäure-{1-[6-oxo-4-(1-oxy-pyridin-2-sulfonyl)-hexahydro-pyrrolo[3,2-b]pyrrol-1-carbonyl]-butyl}-amid,
Thiophen-3-carbonsäure-{1-[6-oxo-4-(1-oxy-pyridin-2-sulfonyl)-hexahydro-pyrrolo[3,2-b]pyrrol-1-carbonyl]-butyl}-amid,
N-{1-[6-oxo-4-(1-oxy-pyridin-2-sulfonyl)-hexahydro-pyrrolo[3,2-b]pyrrol-1-carbonyl]-butyl}-4-phenoxy-benzamid,
N-{1-[6-Oxo-4-(1-oxy-pyridin-2-sulfonyl)-hexahydro-pyrrolo[3,2-b]pyrrol-1-carbonyl]-butyl}-4-(4-methyl-piperazin-1-yl)-benzamid,
4-Methyl-N-{1-[6-oxo-4-(1-oxy-pyridin-2-sulfonyl)-hexahydro-pyrrolo[3,2-b]pyrrol-1-carbonyl]-butyl}-benzamid,
4-Methoxy-N-{1-[6-oxo-4-(1-oxy-pyridin-2-sulfonyl)-hexahydro-pyrrolo[3,2-b]pyrrol-1-carbonyl]-butyl}-benzamid,
4-Isopropyl-N-{1-[6-oxo-4-(1-oxy-pyridin-2-sulfonyl)-hexahydro-pyrrolo[3,2-b]pyrrol-1-carbonyl]-butyl}-benzamid,
N-{1-[6-Oxo-4-(1-oxy-pyridin-2-sulfonyl)-hexahydro-pyrrolo[3,2-b]pyrrol-1-carbonyl]-butyl}-4-vinyl-benzamid,
4-Imidazol-1-yl-N-{1-[6-oxo-4-(1-oxy-pyridin-2-sulfonyl)-hexahydro-pyrrolo[3,2-b]pyrrol-1-carbonyl-butyl]-benzamid,
N-{1-[6-Oxo-4-(1-oxy-pyridin-2-sulfonyl)-hexahydro-pyrrolo[3,2-b]pyrrol-1-carbonyl]-butyl}-4-thiophen-2-yl-benzamid,
N-{1-[6-Oxo-4-(1-oxy-pyridin-2-sulfonyl)-hexahydro-pyrrolo[3,2-b]pyrrol-1-carbonyl]-butyl}-4-oxazol-5-yl-benzamid,
5-Phenyl-thiophen-2-carbonsäure-{1-[6-oxo-4-(1-oxy-pyridin-2-sulfonyl)-hexahydro-pyrrolo[3,2-b]pyrrol-1-carbonyl]-butyl}-amid,
N-{1-[6-Oxo-5-(1-oxy-pyridin-2-sulfonyl)-hexahydro-pyrrolo[3,2-b]pyrrol-1-carbonyl]-butyl}-4-trifluormethoxy-benzamid,
5-Pyridin-2-yl-thiophen-2-carbonsäure-{1-[6-oxo-4-(1-oxy-pyridin-2-sulfonyl)-hexahydro-pyrrolo[3,2-b]pyrrol-1-carbonyl]-butyl}-amid,
4-Difluormethoxy-N-{1-[6-oxo-4-(1-oxy-pyridin-2-sulfonyl)-hexahydro-pyrrolo[3,2-b]pyrrol-1-carbonyl]-butyl}-benzamid,
N-{1-[6-Oxo-4-(1-oxy-pyridin-2-sulfonyl)-hexahydro-pyrrolo[3,2-b]pyrrol-1-carbonyl]-butyl}-4-morpholin-4-yl-benzamid,
Naphthalen-2-carbonsäure-{3-methyl-1-[6-oxo-4-(pyridin-3-sulfonyl)-hexahydro-pyrrolo[3,2-b]pyrrol-1-carbonyl]-butyl}-amid,
Chinolin-2-carbonsäure-{3-methyl-1-[6-oxo-4-(pyridin-3-sulfonyl)-hexahydro-pyrrolo[3,2-b]pyrrol-1-carbonyl]-butyl}-amid,
Benzo[b]thiophen-2-carbonsäure-{3-methyl-1-[6-oxo-4-(pyridin-3-sulfonyl)-hexahydro-pyrrolo[3,2-b]pyrrol-1-carbonyl]-butyl}-amid,
Benzofuran-2-carbonsäure-{3-methyl-1-[6-oxo-4-(pyridin-3-sulfonyl)-hexahydro-pyrrolo[3,2-b]pyrrol-1-carbonyl]-butyl}-amid,
Biphenyl-4-carbonsäure-{3-methyl-1-[6-oxo-4-(pyridin-3-sulfonyl)-hexahydro-pyrrolo[3,2-b]pyrrol-1-carbonyl]-butyl}-amid,
4-tert-Butyl-N-{3-methyl-1-[6-oxo-4-(pyridin-3-sulfonyl)-hexahydro-pyrrolo[3,2-b]pyrrol-1-carbonyl]-butyl}-benzamid,
4-Dimethylamino-N-{3-methyl-1-[6-oxo-4-(pyridin-3-sulfonyl)-hexahydro-pyrrolo[3,2-b]pyrrol-1-carbonyl]-butyl}-benzamid,
7-Methoxy-benzofuran-2-carbonsäure-{3-methyl-1-[6-oxo-4-(pyridin-3-sulfonyl)-hexahydro-pyrrolo[3,2-b]pyrrol-1-carbonyl]-butyl}-amid,
{3-Methyl-1-[6-oxo-4-(pyridin-3-sulfonyl)-hexahydro-pyrrolo[3,2-b]pyrrol-1-carbonyl]-butyl}-carbaminsäurebenzylester,
5-Methoxy-benzofuran-2-carbonsäure-{3-methyl-1-[6-oxo-4-(pyridin-3-sulfonyl)-hexahydro-pyrrolo[3,2-b]pyrrol-1-carbonyl]-butyl}-amid,
Thieno[3,2-b]thiophen-2-carbonsäure-{3-methyl-1-[6-oxo-4-(pyridin-3-sulfonyl)-hexahydro-pyrrolo[3,2-b]pyrrol-1-carbonyl]-butyl}-amid,
3-Methyl-benzofuran-2-carbonsäure-{3-methyl-1-[6-oxo-4-(pyridin-3-sulfonyl)-hexahydro-pyrrolo[3,2-b]pyrrol-1-carbonyl]-butyl}-amid,
Chinoxalin-2-carbonsäure-{3-methyl-1-[6-oxo-4-(pyridin-3-sulfonyl)-hexahydro-pyrrolo[3,2-b]pyrrol-1-carbonyl]-butyl}-amid,
Benzo[1,3]-dioxol-5-carbonsäure-{3-methyl-1-[6-oxo-4-(pyridin-3-sulfonyl)-hexahydro-pyrrolo[3,2-b]pyrrol-1-carbonyl]-butyl}-amid,
Chinolin-6-carbonsäure-{3-methyl-1-[6-oxo-4-(pyridin-3-sulfonyl)-hexahydro-pyrrolo[3,2-b]pyrrol-1-carbonyl]-butyl}-amid,
Furan-2-carbonsäure-{3-methyl-1-[6-oxo-4-(pyridin-3-sulfonyl)-hexahydro-pyrrolo[3,2-b]pyrrol-1-carbonyl]-butyl}-amid,
Thiophen-2-carbonsäure-{3-methyl-1-[6-oxo-4-(pyridin-3-sulfonyl)-hexahydro-pyrrolo[3,2-b]pyrrol-1-carbonyl]-butyl}-amid,
N-{3-Methyl-1-[6-oxo-4-(pyridin-3-sulfonyl)-hexahydro-pyrrolo[3,2-b]pyrrol-1-carbonyl]-butyl}-4-trifluormethyl-benzamid,
Furan-3-carbonsäure-{3-methyl-1-[6-oxo-4-(pyridin-3-sulfonyl)-hexahydro-pyrrolo[3,2-b]pyrrol-1-carbonyl]-butyl}-amid,
Thiophen-3-carbonsäure-{3-methyl-1-[6-öxo-4-(pyridin-3-sulfonyl)-hexahydro-pyrrolo[3,2-b]pyrrol-1-carbonyl]-butyl}-amid,
N-{3-Methyl-1-[6-oxo-4-(pyridin-3-sulfonyl)-hexahydro-pyrrolo[3,2-b]pyrrol-1-carbonyl]-butyl}-4-phenoxy-benzamid,
N-{3-Methyl-1-[6-oxo-4-(pyridin-3-sulfonyl)-hexahydro-pyrrolo[3,2-b]pyrrol-1-carbonyl]-butyl}-4-(4-methyl-piperazin-1-yl)-benzamid,
4-Methyl-N-{3-methyl-1-[6-oxo-4-(pyridin-3-sulfonyl)-hexahydro-pyrrolo[3,2-b]pyrrol-1-carbonyl]-butyl}-benzamid,
4-Methoxy-N-{3-methyl-1-[6-oxo-4-(pyridin-3-sulfonyl)-hexahydro-pyrrolo[3,2-b]pyrrol-1-carbonyl]-butyl}-benzamid,
4-Isopropyl-N-{3-methyl-1-[6-oxo-4-(pyridin-3-sulfonyl)-hexahydro-pyrrolo[3,2-b]pyrrol-1-carbonyl]-butyl}-benzamid,
N-{3-Methyl-1-[6-oxo-4-(pyridin-3-sulfonyl)-hexahydro-pyrrolo[3,2-b]pyrrol-1-carbonyl]-butyl}-4-vinyl-benzamid,
4-Imidazol-1-yl-N-{3-methyl-1-[6-oxo-4-(pyridin-3-sulfonyl)-hexahydro-pyrrolo[3,2-b]pyrrol-1-carbonyl]-butyl}-benzamid,
N-{3-Methyl-1-[6-oxo-4-(pyridin-3-sulfonyl)-hexahydro-pyrrolo[3,2-b]pyrrol-1-carbonyl]-butyl}-4-thiophen-2-yl-benzamid,
N-{3-Methyl-1-[6-oxo-4-(pyridin-3-sulfonyl)-hexahydro-pyrrolo[3,2-b]pyrrol-1-carbonyl]-butyl}-4-oxazol-5-yl-benzamid,
5-Phenyl-thiophen-2-carbonsäure-{3-methyl-1-[6-oxo-4-(pyridin-3-sulfonyl)-hexahydro-pyrrolo[3,2-b]pyrrol-1-carbonyl]-butyl}-amid,
N-{3-Methyl-1-[6-oxo-4-(pyridin-3-sulfonyl)-hexahydro-pyrrolo[3,2-b]pyrrol-1-carbonyl]-butyl}-4-trifluormethoxy-benzamid,
5-Pyridin-2-yl-thiophen-2-carbonsäure-{3-methyl-1-[6-oxo-4-(pyridin-3-sulfonyl)-hexahydro-pyrrolo[3,2-b]pyrrol-1-carbonyl]-butyl}-amid,
4-Difluormethoxy-N-{3-methyl-1-[6-oxo-4-(pyridin-3-sulfonyl)-hexahydro-pyrrolo[3,2-b]pyrrol-1-carbonyl]-butyl}-benzamid,
N-{3-Methyl-1-[6-oxo-4-(pyridin-3-sulfonyl)-hexahydro-pyrrolo[3,2-b]pyrrol-1-carbonyl]-butyl}-4-morpholin-4-yl-benzamid,
Naphthalen-2-carbonsäure-{1-[6-oxo-4-(pyridin-3-sulfonyl)-hexahydro-pyrrolo[3,2-b]pyrrol-1-carbonyl]-butyl}-amid,
Chinolin-2-carbonsäure-{1-[6-oxo-4-(pyridin-3-sulfonyl)-hexahydro-pyrrolo[3,2-b]pyrrol-1-carbonyl]-butyl}-amid,

Benzo[b]thiophen-2-carbonsäure-{1-[6-oxo-4-(pyridin-3-sulfonyl)-hexahydro-pyrrolo[3,2-b]pyrrol-1-carbonyl]-butyl}-amid,
Benzofuran-2-carbonsäure-{1-[6-oxo-4-(pyridin-3-sulfonyl)-hexahydro-pyrrolo[3,2-b]pyrrol-1-carbonyl]-butyl}-amid,
Biphenyl-4-carbonsäure-{1-[6-oxo-4-(pyridin-3-sulfonyl)-hexahydro-pyrrolo[3,2-b]pyrrol-1-carbonyl]-butyl}-amid,
4-tert-Butyl-N-{1-[6-oxo-4-(pyridin-3-sulfonyl)-hexahydro-pyrrolo[3,2-b]pyrrol-1-carbonyl]-butyl}-benzamid,
4-Dimethylamino-N-{1-[6-oxo-4-(pyridin-3-sulfonyl)-hexahydro-pyrrolo[3,2-b]pyrrol-1-carbonyl]-butyl}-benzamid,
7-Methoxy-benzofuran-2-carbonsäure-{1-[6-oxo-4-(pyridin-3-sulfonyl)-hexahydro-pyrrolo[3,2-b]pyrrol-1-carbonyl]-butyl}-amid,
{1-[6-oxo-4-(pyridin-3-sulfonyl)-hexahydro-pyrrolo[3,2-b]pyrrol-1-carbonyl]-butyl}-carbaminsäurebenzylester,
5-Methoxy-benzofuran-2-carbonsäure-{1-[6-oxo-4-(pyridin-3-sulfonyl)-hexahydro-pyrrolo[3,2-b]pyrrol-1-carbonyl]-butyl}-amid,
Thieno[3,2-b]thiophen-2-carbonsäure-{1-[6-oxo-4-(pyridin-3-sulfonyl)-hexahydro-pyrrolo[3,2-b]pyrrol-1-carbonyl]-butyl}-amid,
3-Methyl-benzofuran-2-carbonsäure-{1-[6-oxo-4-(pyridin-3-sulfonyl)-hexahydro-pyrrolo[3,2-b]pyrrol-1-carbonyl]-butyl}-amid,
Chinoxalin-2-carbonsäure-{1-[6-oxo-4-(pyridin-3-sulfonyl)-hexahydro-pyrrolo[3,2-b]pyrrol-1-carbonyl]-butyl}-amid,
Benzo[1,3]dioxol-5-carbonsäure-{1-[6-oxo-4-(pyridin-3-sulfonyl)-hexahydro-pyrrolo[3,2-b]pyrrol-1-carbonyl]-butyl}-amid,
Chinolin-6-carbonsäure-{1-[6-oxo-4-(pyridin-3-sulfonyl)-hexahydro-pyrrolo[3,2-b]pyrrol-1-carbonyl]-butyl}-amid,
Furan-2-carbonsäure-{1-[6-oxo-4-(pyridin-3-sulfonyl)-hexahydro-pyrrolo[3,2-b]pyrrol-1-carbonyl]-butyl}-amid,
Thiophen-2-carbonsäure-{1-[6-oxo-4-(pyridin-3-sulfonyl)-hexahydro-pyrrolo[3,2-b]pyrrol-1-carbonyl]-butyl}-amid,
N-{1-[6-Oxo-4-(pyridin-3-sulfonyl)-hexahydro-pyrrolo[3,2-b]pyrrol-1-carbonyl]-butyl}-4-trifluormethyl-benzamid,
Furan-3-carbonsäure-{1-[6-oxo-4-(pyridin-3-sulfonyl)-hexahydro-pyrrolo[3,2-b]pyrrol-1-carbonyl]-butyl}-amid,
Thiophen-3-carbonsäure-{1-[6-oxo-4-(pyridin-3-sulfonyl)-hexahydro-pyrrolo[3,2-b]pyrrol-1-carbonyl]-butyl}-amid,
N-{1-[6-Oxo-4-(pyridin-3-sulfonyl)-hexahydro-pyrrolo[3,2-b]pyrrol-1-carbonyl]-butyl}-4-phenoxy-benzamid,
N-{1-[6-oxo-4-(pyridin-3-sulfonyl)-hexahydro-pyrrolo[3,2-b]pyrrol-1-carbonyl]-butyl}-4-(4-methylpiperazin-1-yl)-benzamid,
4-Methyl-N-1-[6-oxo-4-(pyridin-3-sulfonyl)-hexahydro-pyrrolo[3,2-b]pyrrol-1-carbonyl]-butyl}-benzamid,
4-Methoxy-N-{1-[6-oxo-4-(pyridin-3-sulfonyl)-hexahydro-pyrrolo[3,2-b]pyrrol-1-carbonyl]-butyl}-benzamid,
4-Isopropyl-N-1-[6-oxo-4-(pyridin-3-sulfonyl)-hexahydro-pyrrolo[3,2-b]pyrrol-1-carbonyl]-butyl}-benzamid,
N-{1-[6-oxo-4-(pyridin-3-sulfonyl)-hexahydro-pyrrolo[3,2-b]pyrrol-1-carbonyl]-butyl}-4-vinyl-benzamid,
4-Imidazol-1-yl-N-{1-[6-oxo-4-(pyridin-3-sulfonyl)-hexahydro-pyrrolo[3,2-b]pyrrol-1-carbonyl]-butyl}-benzamid,
N-{1-[6-Oxo-4-(pyridin-3-sulfonyl)-hexahydro-pyrrolo[3,2-b]pyrrol-1-carbonyl]-butyl}-4-thiophen-2-yl-benzamid,
N-{1-[6-Oxo-4-(pyridin-3-sulfonyl)-hexahydro-pyrrolo[3,2-b]pyrrol-1-carbonyl]-butyl}-4-oxazol-5-yl-benzamid,
5-Phenyl-thiophen-2-carbonsäure-{1-[6-oxo-4-(pyridin-3-sulfonyl)-hexahydro-pyrrolo[3,2-b]pyrrol-1-carbonyl]-butyl}-amid,
N-{1-[6-Oxo-4-(pyridin-3-sulfonyl)-hexahydro-pyrrolo[3,2-b]pyrrol-1-carbonyl]-butyl}-4-trifluormethoxy-benzamid,
5-Pyridin-2-yl-thiophen-2-carbonsäure-{1-[6-oxo-4-(pyridin-3-sulfonyl)-hexahydro-pyrrolo[3,2-b]pyrrol-1-carbonyl]-butyl}-amid,
4-Difluormethoxy-N-{1-[6-oxo-4-(pyridin-3-sulfonyl)-hexahydro-pyrrolo[3,2-b]pyrrol-1-carbonyl]-butyl}-benzamid,
N-{1-[6-Oxo-4-(pyridin-3-sulfonyl)-hexahydro-pyrrolo[3,2-b]pyrrol-1-carbonyl]-butyl}-4-morpholin-4-yl-benzamid,
Naphthalen-2-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,
Chinolin-2-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,
Benzo[b]thiophen-2-carbonsäure-{3-methyl-1-[6-oxo-4-(1-oxy-pyridin-3-sulfonyl)-hexahydro-pyrrolo[3,2-b]pyrrol-1 -carbonyl]-butyl}-amid,
Benzofuran-2-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,
Biphenyl-4-carbonsäure-{3-methyl-1-[6-oxo-4-(1-oxy-pyridin-3-sulfonyl)-hexahydro-pyrrolo[3,2-b)pyrrol-1-carbonyl]-butyl}-amid,
4-tert-Butyl-N-{3-methyl-1-[6-oxo-4-(1-oxy-pyridin-3-sulfonyl)-hexahydro-pyrrolo[3,2-b]pyrrol-1-carbonyl]-butyl}-benzamid,
4-Dimethylamino-N-{3-methyl-1-[6-oxo-4-(1-oxy-pyridin-3-sulfonyl)-hexahydro-pyrrolo[3,2-b]pyrrol-1-carbonyl]-butyl}-benzamid,
7-Methoxy-benzofuran-2-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,

{3-Methyl-1-[6-oxo-4-(1-oxy-pyridin-3-sulfonyl)-hexahydro-pyrrolo[3,2-b]pyrrol-1-carbonyl]-butyl}-carbaminsäurebenzylester,
5-Methoxy-benzofuran-2-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,
Thieno[3,2-b]thiophen-2-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,
3-Methyl-benzofuran-2-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,
Chinoxalin-2-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,
Benzo[1,3]dioxol-5-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,
Chinolin-6-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,
Furan-2-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,
Thiophen-2-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,
N-{3-Methyl-1-[6-oxo-4-(1-oxy-pyridin-3-sulfonyl)-hexahydro-pyrrolo[3,2-b]pyrrol-1-carbonyl]-butyl}-4-trifluormethyl-benzamid,
Furan-3-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,
Thiophen-3-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,
N-{3-Methyl-1-[6-oxo-4-(1-oxy-pyridin-3-sulfonyl)-hexahydro-pyrrolo[3,2-b]pyrrol-1-carbonyl]-butyl}-4-phenoxy-benzamid,
N-{3-Methyl-1-[6-oxo-4-(1-oxy-pyridin-3-sulfonyl)-hexahydro-pyrrolo[3,2-b]pyrrol-1-carbonyl]-butyl}-4-(4-methyl-piperazin-1-yl)-benzamid,
4-Methyl-N-{3-methyl-1-[6-oxo-4-(1-oxy-pyridin-3-sulfonyl)-hexahydro-pyrrolo[3,2-b]pyrrol-1-carbonyl]-butyl}-benzamid,
4-Methoxy-N-{3-methyl-1-[6-oxo-4-(1-oxy-pyridin-3-sulfonyl)-hexahydro-pyrrolo[3,2-b]pyrrol-1-carbonyl]-butyl}-benzamid,
4-Isopropyl-N-{3-methyl-1-[6-oxo-4-(1-oxy-pyridin-3-sulfonyl)-hexahydro-pyrrolo[3,2-b]pyrrol-1-carbonyl]-butyl}-benzamid,
N-{3-Methyl-1-[6-oxo-4-(1-oxy-pyridin-3-sulfonyl)-hexahydro-pyrrolo[3,2-b]pyrrol-1-carbonyl]-butyl}-4-vinyl-benzamid,
4-Imidazol-1-yl-N-{3-methyl-1-[6-oxo-4-(1-oxy-pyridin-3-sulfonyl)-hexahydro-pyrrolo[3,2-b]pyrrol-1-carbonyl]-butyl}-benzamid,
N-{3-Methyl-1-[6-oxo-4-(1-oxy-pyridin-3-sulfonyl)-hexahydro-pyrrolo[3,2-b]pyrrol-1-carbonyl]-butyl}-4-thiophen-2-yl-benzamid,
N-{3-Methyl-1-[6-oxo-4-(1-oxy-pyridin-3-sulfonyl)-hexahydro-pyrrolo[3,2-b]pyrrol-1-carbonyl]-butyl}-4-oxazol-5-yl-benzamid,
5-Phenyl-thiophen-2-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,
N-{3-Methyl-1-[6-oxo-4-(1-oxy-pyridin-3-sulfonyl)-hexahydro-pyrrolo[3,2-b]pyrrol-1-carbonyl]-butyl}-4-trifluormethoxy-benzamid,
5-Pyridin-2-yl-thiophen-2-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,
4-Difluormethoxy-N-{3-methyl-1-[6-oxo-4-(1-oxy-pyridin-3-sulfonyl)-hexahydro-pyrrolo[3,2-b]pyrrol-1-carbonyl]-butyl}-benzamid,
N-{3-Methyl-1-[6-oxo-4-(1-oxy-pyridin-3-sulfonyl)-hexahydro-pyrrolo[3,2-b]pyrrol-1-carbonyl]-butyl}-4-morpholin-4-yl-benzamid,
Naphthalen-2-carbonsäure-{1-[6-oxo-4-(1-oxy-pyridin-3-sulfonyl)-hexahydro-pyrrolo[3,2-b]pyrrol-1-carbonyl]-butyl}-amid,
Chinolin-2-carbonsäure-{1-[6-oxo-4-(1-oxy-pyridin-3-sulfonyl)-hexahydro-pyrrolo[3,2-b]pyrrol-1-carbonyl]-butyl}-amid,
Benzo[b]thiophen-2-carbonsäure-{1-[6-oxo-4-(1-oxy-pyridin-3-sulfonyl)-hexahydro-pyrrolo[3,2-b]pyrrol-1-carbonyl]-butyl}-amid,

Benzofuran-2-carbonsäure-{1-[6-oxo-4-(1-oxy-pyridin-3-sulfonyl)-hexahydro-pyrrolo[3,2-b]pyrrol-1-carbonyl]-butyl}-amid,
Biphenyl-4-carbonsäure-{1-[6-oxo-4-(1-oxy-pyridin-3-sulfonyl)-hexahydro-pyrrolo[3,2-b]pyrrol-1-carbonyl]-butyl}-amid,
4-tert-Butyl-N-{1-[6-oxo-4-(1-oxy-pyridin-3-sulfonyl)-hexahydro-pyrrolo[3,2-b]pyrrol-1-carbonyl]-butyl}-benzamid,
4-Dimethylamino-N-{1-[6-oxo-4-(1-oxy-pyridin-3-sulfonyl)-hexahydro-pyrrolo[3,2-b]pyrrol-1-carbonyl]-butyl}-benzamid,
7-Methoxy-benzofuran-2-carbonsäure-{1-[6-oxo-4-(1-oxy-pyridin-3-sulfonyl)-hexahydro-pyrrolo[3,2-b]pyrrol-1-carbonyl]-butyl}-amid,
{1-[6-Oxo-4-(1-oxy-pyridin-3-sulfonyl)-hexahydro-pyrrolo[3,2-b]pyrrol-1-carbonyl]-butyl}-carbaminsäurebenzylester,
5-Methoxy-benzofuran-2-carbonsäure-{1-[6-oxo-4-(1-oxy-pyridin-3-sulfonyl)-hexahydro-pyrrolo[3,2-b]pyrrol-1-carbonyl]-butyl}-amid,
Thieno[3,2-b]thiophen-2-carbonsäure-{1-[6-oxo-4-(1-oxy-pyridin-3-sulfonyl)-hexahydro-pyrrolo[3,2-b]pyrrol-1-carbonyl]-butyl}-amid,
3-Methyl-benzofuran-2-carbonsäure-{1-[6-oxo-4-(1-oxy-pyridin-3-sulfonyl)-hexahydro-pyrrolo[3,2-b]pyrrol-1-carbonyl]-butyl}-amid,
Chinoxalin-2-carbonsäure-{1-[6-oxo-4-(1-oxy-pyridin-3-sulfonyl)-hexahydro-pyrrolo[3,2-b]pyrrol-1-carbonyl]-butyl}-amid,
Benzo[1,3]dioxol-5-carbonsäure-{1-[6-oxo-4-(1-oxy-pyridin-3-sulfonyl)-hexahydro-pyrrolo[3,2-b]pyrrol-1-carbonyl]-butyl}-amid,
Chinolin-6-carbonsäure-{1-[6-oxo-4-(1-oxy-pyridin-3-sulfonyl)-hexahydro-pyrrolo[3,2-b]pyrrol-1-carbonyl]-butyl}-amid,
Furan-2-carbonsäure-{1-[6-oxo-4-(1-oxy-pyridin-3-sulfonyl)-hexahydro-pyrrolo[3,2-b]pyrrol-1-carbonyl]-butyl}-amid,
Thiophen-2-carbonsäure-{1-[6-oxo-4-(1-oxy-pyridin-3-sulfonyl)-hexahydro-pyrrolo[3,2-b]pyrrol-1-carbonyl]-butyl)-amid,
N-{1-[6-oxo-4-(1-oxy-pyridin-3-sulfonyl)-hexahydro-pyrrolo[3,2-b]pyrrol-1-carbonyl]-butyl}-4-trifluormethyl-benzamid,
Furan-3-carbonsäure-{1-[6-oxo-4-(1-oxy-pyridin-3-sulfonyl)-hexahydro-pyrrolo[3,2-b]pyrrol-1-carbonyl]-butyl}-amid,
Thiophen-3-carbonsäure-{1-[6-oxo-4-(1-oxy-pyridin-3-sulfonyl)-hexahydro-pyrrolo[3,2-b]pyrrol-1-carbonyl]-butyl}-amid,
N-{1-[6-Oxo-4-(1-oxy-pyridin-3-sulfonyl)-hexahydro-pyrrolo[3,2-b]pyrrol-1-carbonyl]-butyl}-4-phenoxy-benzamid,
N-{1-[6-Oxo-4-(1-oxy-pyridin-3-sulfonyl)-hexahydro-pyrrolo[3,2-b]pyrrol-1-carbonyl]-butyl}-4-(4-methyl-piperazin-1-yl)-benzamid,
4-Methyl-N-{1-[6-oxo-4-(1-oxy-pyridin-3-sulfonyl)-hexahydro-pyrrolo[3,2-b]pyrrol-1-carbonyl]-butyl}-benzamid,
4-Methoxy-N-{1-[6-oxo-4-(1-oxy-pyridin-3-sulfonyl)-hexahydro-pyrrolo[3,2-b]pyrrol-1-carbonyl]-butyl}-benzamid,
4-Isopropyl-N-{1-[6-oxo-4-(1-oxy-pyridin-3-sulfonyl)-hexahydro-pyrrolo[3,2-b]pyrrol-1-carbonyl]-butyl}-benzamid,
N-{1-[6-Oxo-4-(1-oxy-pyridin-3-sulfonyl)-hexahydro-pyrrolo[3,2-b]pyrrol-1-carbonyl]-butyl}-4-vinyl-benzamid,
4-Imidazol-1-yl-N-{1-[6-oxo-4-(1-oxy-pyridin-3-sulfonyl)-hexahydro-pyrrolo[3,2-b]pyrrol-1-carbonyl]-butyl}-benzamid,
N-{1-[6-Oxo-4-(1-oxy-pyridin-3-sulfonyl)-hexahydro-pyrrolo[3,2-b]pyrrol-1-carbonyl]-butyl}-4-thiophen-2-yl-benzamid,
N-{1-[6-Oxo-4-(1-oxy-pyridin-3-sulfonyl)-hexahydro-pyrrolo[3,2-b]pyrrol-1-carbonyl]-butyl}-4-oxazol-5-yl-benzamid,
5-Phenyl-thiophen-2-carbonsäure-{1-[6-oxo-4-(1-oxy-pyridin-3-sulfonyl)-hexahydro-pyrrolo[3,2-b]pyrrol-1-carbonyl]-butyl}-amid,
N-{1-[6-Oxo-4-(1-oxy-pyridin-3-sulfonyl)-hexahydro-pyrrolo[3,2-b]pyrrol-1-carbonyl]-butyl}-4-trifluormethoxy-benzamid,
5-Pyridin-2-yl-thiophen-2-carbonsäure-{1-[6-oxo-4-(1-oxy-pyridin-3-sulfonyl)-hexahydro-pyrrolo[3,2-b]pyrrol-1-carbonyl]-butyl}-amid,
4-Difluormethoxy-N-{1-[6-oxo-4-(1-oxy-pyridin-3-sulfonyl)-hexahydro-pyrrolo[3,2-b]pyrrol-1-carbonyl]-butyl}-benzamid,
N-{1-[6-Oxo-4-(1-oxy-pyridin-3-sulfonyl)-hexahydro-pyrrolo[3,2-b]pyrrol-1-carbonyl]-butyl}-4-morpholin-4-yl-benzamid,
Naphthalen-2-carbonsäure-{3-methyl-1-[6-oxo-4-(pyridin-2-carbonyl)-hexahydro-pyrrolo[3,2-b]pyrrol-1-carbonyl]-butyl}-amid,

Chinolin-2-carbonsäure-{3-methyl-1-[6-oxo-4-(pyridin-2-carbonyl)-hexahydro-pyrrolo[3,2-b]pyrrol-1-carbonyl]-butyl}-amid,
Benzo[b]thiophen-2-carbonsäure-{3-methyl-1-[6-oxo-4-(pyridin-2-carbonyl)-hexahydro-pyrrolo[3,2-b]pyrrol-1-carbonyl]-butyl}-amid,
Benzofuran-2-carbonsäure-{3-methyl-1-[6-oxo-4-(pyridin-2-carbonyl)-hexahydro-pyrrolo[3,2-b]pyrrol-1-carbonyl]-butyl}-amid,
Biphenyl-4-carbonsäure-{3-methyl-1-[6-oxo-4-(pyridin-2-carbonyl)-hexahydro-pyrrolo[3,2-b]pyrrol-1-carbonyl]-butyl}-amid,
4-tert-Butyl-N-{3-methyl-1-[6-oxo-4-(pyridin-2-carbonyl)-hexahydro-pyrrolo[3,2-b]pyrrol-1-carbonyl]-butyl}-benzamid,
4-Dimethylamino-N-{3-methyl-1-[6-oxo-4-(pyridin-2-carbonyl)-hexahydro-pyrrolo[3,2-b]pyrrol-1-carbonyl]-butyl}-benzamid,
7-Methoxy-benzofuran-2-carbonsäure-{3-methyl-1-[6-oxo-4-(pyridin-2-carbonyl)-hexahydro-pyrrolo[3,2-b]pyrrol-1-carbonyl]-butyl}-amid,
{3-Methyl-1-[6-oxo-4-(pyridin-2-carbonyl)-hexahydro-pyrrolo[3,2-b]pyrrol-1-carbonyl]-butyl}-carbaminsäurebenzylester,
5-Methoxy-benzofuran-2-carbonsäure-{3-methyl-1-[6-oxo-4-(pyridin-2-carbonyl)-hexahydro-pyrrolo[3,2-b]pyrrol-1-carbonyl]-butyl}-amid,
Thieno[3,2-b]thiophen-2-carbonsäure-{3-methyl-1-[6-oxo-4-(pyridin-2-carbonyl)-hexahydro-pyrrolo[3,2-b]pyrrol-1-carbonyl]-butyl}-amid,
3-Methyl-benzofuran-2-carbonsäure-{3-methyl-1-[6-oxo-4-(pyridin-2-carbonyl)-hexahydro-pyrrolo[3,2-b]pyrrol-1-carbonyl]-butyl}-amid,
Chinoxalin-2-carbonsäure-{3-methyl-1-[6-oxo-4-(pyridin-2-carbonyl)-hexahydro-pyrrolo[3,2-b]pyrrol-1-carbonyl]-butyl}-amid,
Benzo[1,3]dioxol-5-carbonsäure-{3-methyl-1-[6-oxo-4-(pyridin-2-carbonyl)-hexahydro-pyrrolo[3,2-b]pyrrol-1-carbonyl]-butyl}-amid,
Chinolin-6-carbonsäure-{3-methyl-1-[6-oxo-4-(pyridin-2-carbonyl)-hexahydro-pyrrolo[3,2-b]pyrrol-1-carbonyl]-butyl}-amid,
Furan-2-carbonsäure-{3-methyl-1-[6-oxo-4-(pyridin-2-carbonyl)-hexahydro-pyrrolo[3,2-b]pyrrol-1-carbonyl]-butyl}-amid,
Thiophen-2-carbonsäure-{3-methyl-1-[6-oxo-4-(pyridin-2-carbonyl)-hexahydro-pyrrolo[3,2-b]pyrrol-1-carbonyl]-butyl}-amid,
N-{3-Methyl-1-[6-oxo-4-(pyridin-2-carbonyl)-hexahydro-pyrrolo[3,2-b]pyrrol-1-carbonyl]-butyl}-4-trifluormethyl-benzamid,
Furan-3-carbonsäure-{3-methyl-1-[6-oxo-4-(pyridin-2-carbonyl)-hexahydro-pyrrolo[3,2-b]pyrrol-1-carbonyl]-butyl}-amid,
Thiophen-3-carbonsäure-{3-methyl-1-[6-oxo-4-(pyridin-2-carbonyl)-hexahydro-pyrrolo[3,2-b]pyrrol-1-carbonyl]-butyl}-amid,
N-{3-Methyl-1-[6-oxo-4-(pyridin-2-carbonyl)-hexahydro-pyrrolo[3,2-b]pyrrol-1-carbonyl]-butyl}-4-phenoxy-benzamid,
N-{3-Methyl-1-[6-oxo-4-(pyridin-2-carbonyl)-hexahydro-pyrrolo[3,2-b]pyrrol-1-carbonyl]-butyl}-4-(4-methyl-piperazin-1-yl)-benzamid,
4-Methyl-N-{3-methyl-1-[6-oxo-4-(pyridin-2-carbonyl)-hexahydro-pyrrolo[3,2-b]pyrrol-1-carbonyl]-butyl}-benzamid,
4-Methoxy-N-{3-methyl-1-[6-oxo-4-(pyridin-2-carbonyl)-hexahydro-pyrrolo[3,2-b]pyrrol-1-carbonyl]-butyl}-benzamid,
4-Isopropyl-N-{3-methyl-1-[6-oxo-4-(pyridin-2-carbonyl)-hexahydro-pyrrolo[3,2-b]pyrrol-1-carbonyl]-butyl}-benzamid,
N-{3-Methyl-1-[6-oxo-4-(pyridin-2-carbonyl)-hexahydro-pyrrolo[3,2-b]pyrrol-1-carbonyl]-butyl}-4-vinyl-benzamid,
4-Imidazol-1-yl-N-{3-methyl-1-[6-oxo-4-(pyridin-2-carbonyl)-hexahydro-pyrrolo[3,2-b]pyrrol-1-carbonyl]-butyl}-benzamid,
N-{3-Methyl-1-[6-oxo-4-(pyridin-2-carbonyl)-hexahydro-pyrrolo[3,2-b]pyrrol-1-carbonyl]-butyl}-4-thiophen-2-yl-benzamid,
N-{3-Methyl-1-[6-oxo-4-(pyridin-2-carbonyl)-hexahydro-pyrrolo[3,2-b]pyrrol-1-carbonyl]-butyl}-4-oxazol-5-yl-benzamid,
5-Phenyl-thiophen-2-carbonsäure-{3-methyl-1-[6-oxo-4-(pyridin-2-carbonyl)-hexahydro-pyrrolo[3,2-b]pyrrol-1-carbonyl]-butyl}-amid,
N-{3-Methyl-1-[6-oxo-4-(pyridin-2-carbonyl)-hexahydro-pyrrolo[3,2-b]pyrrol-1-carbonyl]-butyl}-trifluormethoxy-benzamid,

5-Pyridin-2-yl-thiophen-2-carbonsäure-{3-methyl-1-[6-oxo-4-(pyridin-2-carbonyl)-hexahydro-pyrrolo[3,2-b]pyrrol-1-carbonyl]-butyl}-amid,
4-Difluorrnethoxy-N-[3-methyl-1-[6-oxo-4-(pyridin-2-carbonyl)-hexahydro-pyrrolo[3,2-b]pyrrol-1-carbonyl]-butyl}-benzamid,
N-{3-Methyl-1-[6-oxo-4-(pyridin-2-carbonyl)-hexahydro-pyrrolo[3,2-b]pyrrol-1-carbonyl]-butyl}-4-morpholin-4-yl-benzamid,
Naphthalen-2-carbonsäure-{1-[6-oxo-4-(pyridin-2-carbonyl)-hexahydro-pyrrolo[3,2-b]pyrrol-1-carbonyl]-butyl}-amid,
Chinolin-2-carbonsäure-{1-[6-oxo-4-(pyridin-2-carbonyl)-hexahydro-pyrrolo[3,2-b]pyrrol-1-carbonyl]-butyl}-amid,
Benzo[b]thiophen-2-carbonsäure-{1-[6-oxo-4-(pyridin-2-carbonyl)-hexahydro-pyrrolo[3,2-b]pyrrol-1-carbonyl]-butyl}-amid,
Benzofuran-2-carbonsäure-{1-[6-oxo-4-(pyridin-2-carbonyl)-hexahydro-pyrrolo[3,2-b]pyrrol-1-carbonyl]-butyl}-amid,
Biphenyl-4-carbonsäure-{1-[6-oxo-4-(pyridin-2-carbonyl)-hexahydro-pyrrolo[3,2-b]pyrrol-1-carbonyl]-butyl}-amid,
4-tert-Butyl-N-{1-[6-oxo-4-(pyridin-2-carbonyl)-hexahydro-pyrrolo[3,2-b]pyrrol-1-carbonyl]-butyl}-benzamid,
4-Dimethylamino-N-{1-[6-oxo-4-(pyridin-2-carbonyl)-hexahydro-pyrrolo[3,2-b]pyrrol-1-carbonyl]-butyl}-benzamid,
7-Methoxy-benzofuran-2-carbonsäure-{1-[6-oxo-4-(pyridin-2-carbonyl)-hexahydro-pyrrolo[3,2-b]pyrrol-1-carbonyl]-butyl}-amid,
{1-[6-Oxo-4-(pyridin-2-carbonyl)-hexahydro-pyrrolo[3,2-b]pyrrol-1-carbonyl]-butyl}-carbaminsäurebenzylester,
5-Methoxy-benzofuran-2-carbonsäure-{1-[6-oxo-4-(pyridin-2-carbonyl)-hexahydro-pyrrolo[3,2-b]pyrrol-1-carbonyl]-butyl}-amid,
Thieno[3,2-b]thiophen-2-carbonsäure-{1-[6-oxo-4-(pyridin-2-carbonyl)-hexahydro-pyrrolo[3,2-b]pyrrol-1-carbonyl]-butyl}-amid,
3-Methyl-benzofuran-2-carbonsäure-{1-[6-oxo-4-(pyridin-2-carbonyl)-hexahydro-pyrrolo[3,2-b]pyrrol-1-carbonyl]-butyl}-amid,
Chinoxalin-2-carbonsäure-{1-[6-oxo-4-(pyridin-2-carbonyl)-hexahydro-pyrrolo[3,2-b]pyrrol-1-carbonyl]-butyl}-amid,
Benzo[1,3]dioxol-5-carbonsäure-{1-[6-oxo-4-(pyridin-2-carbonyl)-hexahydro-pyrrolo[3,2-b]pyrrol-1-carbonyl]-butyl}-amid,
Chinolin-6-carbonsäure-{1-[6-oxo-4-(pyridin-2-carbonyl)-hexahydro-pyrrolo[3,2-b]pyrrol-1-carbonyl]-butyl}-amid,
Furan-2-carbonsäure-{1-[6-oxo-4-(pyridin-2-carbonyl)-hexahydro-pyrrolo[3,2-b]pyrrol-1-carbonyl]-butyl}-amid,
Thiophen-2-carbonsäure-{1-[6-oxo-4-(pyridin-2-carbonyl)-hexahydro-pyrrolo[3,2-b]pyrrol-1-carbonyl]-butyl}-amid,
N-{1-[6-Oxo-4-(pyridin-2-carbonyl)-hexahydro-pyrrolo[3,2-b]pyrrol-1-carbonyl]-butyl}-4-trifluormethyl-benzamid,
Furan-3-carbonsäure-{1-[6-oxo-4-(pyridin-2-carbonyl)-hexahydro-pyrrolo[3,2-b]pyrrol-1-carbonyl]-butyl}-amid,
Thiophen-3-carbonsäure-{1-[6-oxo-4-(pyridin-2-carbonyl)-hexahydro-pyrrolo[3,2-b]pyrrol-1-carbonyl]-butyl}-amid,
N-{1-[6-Oxo-4-(pyridin-2-carbonyl)-hexahydro-pyrrolo[3,2-b]pyrrol-1-carbonyl]-butyl}-4-phenoxy-benzamid,
N-{1-[6-Oxo-4-(pyridin-2-carbonyl)-hexahydro-pyrrolo[3,2-b]pyrrol-1-carbonyl]-butyl}-4-(4-methylpiperazin-1-yl)-benzamid,
4-Methyl-N-{1-[6-oxo-4-(pyridin-2-carbonyl)-hexahydro-pyrrolo[3,2-b]pyrrol-1-carbonyl]-butyl}-benzamid,
4-Methoxy-N-{1-[6-oxo-4-(pyridin-2-carbonyl)-hexahydro-pyrrolo[3,2-b]pyrrol-1-carbonyl]-butyl}-benzamid,
4-Isopropyl-N-{1-[6-oxo-4-(pyridin-2-carbonyl)-hexahydro-pyrrolo[3,2-b]pyrrol-1-carbonyl]-butyl}-benzamid,
N-{1-[6-Oxo-4-(pyridin-2-carbonyl)-hexahydro-pyrrolo[3,2-b]pyrrol-1-carbonyl]-butyl}-4-vinyl-benzamid,
4-Imidazol-1-yl-N-{1-[6-oxo-4-(pyridin-2-carbonyl)-hexahydro-pyrrolo[3,2-b]pyrrol-1-carbonyl]-butyl}-benzamid,
N-{1-[6-Oxo-4-(pyridin-2-carbonyl)-hexahydro-pyrrolo[3,2-b]pyrrol-1-carbonyl]-butyl}-4-thiophen-2-yl-benzamid,
N-{1-[6-oxo-4-(pyridin-2-carbonyl)-hexahydro-pyrrolo[3,2-b]pyrrol-1-carbonyl]-butyl}-4-oxazol-5-yl-benzamid,
5-Phenyl-thiophen-2-carbonsäure-{1-[6-oxo-4-(pyridin-2-carbonyl)-hexahydro-pyrrolo[3,2-b]pyrrol-1-carbonyl]-butyl}-amid,
N-{1-[6-Oxo-4-(pyridin-2-carbonyl)-hexahydro-pyrrolo[3,2-b]pyrrol-1-carbonyl]-butyl}-4-trifluormethoxy-benzamid,
5-Pyridin-2-yl-thiophen-2-carbonsäuregl-[6-oxo-4-(pyridin-2-carbonyl)-hexahydro-pyrrolo[3,2-b]pyrrol-1-carbonyl]-butyl}-amid,
4-Difluormethoxy-N-{1-[6-oxo-4-(pyridin-2-carbonyl)-hexahydro-pyrrolo[3,2-b]pyrrol-1-carbonyl]-butyl}-benzamid,
N-{1-[6-Oxo-4-(pyridin-2-carbonyl)-hexahydro-pyrrolo[3,2-b]pyrrol-1-carbonyl]-butyl}-4-morpholin-4-yl-benzamid,
Naphthalen-2-carbonsäure-{3-methyl-1-[6-oxo-4-(2-pyridin-3-yl-acetyl)-hexahydro-pyrrolo[3,2-b]pyrrol-1-carbonyl]-butyl}-amid,
Chinolin-2-carbonsäure-{3-methyl-1-[6-oxo-4-(2-pyridin-3-yl-acetyl)-hexahydro-pyrrolo[3,2-b]pyrrol-1-carbonyl]-butyl}-amid,
Benzo[b]thiophen-2-carbonsäure-{3-methyl-1-[6-oxo-4-(2-pyridin-3-yl-acetyl)-hexahydro-pyrrolo[3,2-b]pyrrol-1-carbonyl]-butyl}-amid,

Benzofuran-2-carbonsäure-{3-methyl-1-[6-oxo-4-(2-pyridin-3-yl-acetyl)-hexahydro-pyrrolo[3,2-b]pyrrol-1-carbonyl]-butyl}-amid,
Biphenyl-4-carbonsäure-{3-methyl-1-[6-oxo-4-(2-pyridin-3-yl-acetyl)-hexahydro-pyrrolo[3,2-b]pyrrol-1-carbonyl]-butyl}-amid,
4-tert-Butyl-N-{3-methyl-1-[6-oxo-4-(2-pyridin-3-yl-acetyl)-hexahydro-pyrrolo[3,2-b]pyrrol-1-carbonyl]-butyl}-benzamid,
4-Dimethylamino-N-{3-methyl-1-[6-oxo-4-(2-pyridin-3-yl-acetyl)-hexahydro-pyrrolo[3,2-b]pyrrol-1-carbonyl]-butyl}-benzamid,
7-Methoxy-benzofuran-2-carbonsäure-{3-methyl-1-[6-oxo-4-(2-pyridin-3-yl-acetyl)-hexahydro-pyrrolo[3,2-b]pyrrol-1-carbonyl]-butyl}-amid,
{3-Methyl-1-[6-oxo-4-(2-pyridin-3-yl-acetyl)-hexahydro-pyrrolo[3,2-b]pyrrol-1-carbonyl]-butyl}-carbaminsäurebenzylester,
5-Methoxy-benzofuran-2-carbonsäure-{3-methyl-1-[6-oxo-4-(2-pyridin-3-yl-acetyl)-hexahydro-pyrrolo[3,2-b]pyrrol-1-carbonyl]-butyl}-amid,
Thieno[3,2-b]thiophen-2-carbonsäure-{3-methyl-1-[6-oxo-4-(2-pyridin-3-yl-acetyl)-hexahydro-pyrrolo[3,2-b]pyrrol-1-carbonyl]-butyl}-amid,
3-Methyl-benzofuran-2-carbonsäure-{3-methyl-1-[6-oxo-4-(2-pyridin-3-yl-acetyl)-hexahydro-pyrrolo[3,2-b]pyrrol-1-carbonyl]-butyl}-amid,
Chinoxalin-2-carbonsäure-{3-methyl-1-[6-oxo-4-(2-pyridin-3-yl-acetyl)-hexahydro-pyrrolo[3,2-b]pyrrol-1-carbonyl]-butyl}-amid,
Benzo[1,3]dioxol-5-carbonsäure-{3-methyl-1-[6-oxo-4-(2-pyridin-3-yl-acetyl)-hexahydro-pyrrolo[3,2-b]pyrrol-1-carbonyl]-butyl}-amid,
Chinolin-6-carbonsäure-{3-methyl-1-[6-oxo-4-(2-pyridin-3-yl-acetyl)-hexahydro-pyrrolo[3,2-b]pyrrol-1-carbonyl]-butyl}-amid,
Furan-2-carbonsäure-{3-methyl-1-[6-oxo-4-(2-pyridin-3-yl-acetyl)-hexahydro-pyrrolo[3,2-b]pyrrol-1-carbonyl]-butyl}-amid,
Thiophen-2-carbonsäure-{3-methyl-1-[6-oxo-4-(2-pyridin-3-yl-acetyl)-hexahydro-pyrrolo[3,2-b]pyrrol-1-carbonyl]-butyl}-amid,
N-{3-Methyl-1-[6-oxo-4-(2-pyridin-3-yl-acetyl)-hexahydro-pyrrolo[3,2-b]pyrrol-1-carbonyl]-butyl}-4-trifluormethyl-benzamid,
Furan-3-carbonsäure-{3-methyl-1-[6-oxo-4-(2-pyridin-3-yl-acetyl)-hexahydro-pyrrolo[3,2-b]pyrrol-1-carbonyl]-butyl}-amid,
Thiophen-3-carbonsäure-{3-methyl-1-[6-oxo-4-(2-pyridin-3-yl-acetyl)-hexahydro-pyrrolo[3,2-b]pyrrol-1-carbonyl]-butyl}-amid,
N-{3-Methyl-1-[6-oxo-4-(2-pyridin-3-yl-acetyl)-hexahydro-pyrrolo[3,2-b]pyrrol-1-carbonyl]-butyl}-4-phenoxy-benzamid,
N-{3-Methyl-1-[6-oxo-4-(2-pyridin-3-yl-acetyl)-hexahydro-pyrrolo[3,2-b]pyrrol-1-carbonyl]-butyl}-4-(4-methyl-piperazin-1-yl)-benzamid,
4-Methyl-N-{3-methyl-1-[6-oxo-4-(2-pyridin-3-yl-acetyl)-hexahydro-pyrrolo[3,2-b]pyrrol-1-carbonyl]-butyl}-benzamid,
4-Methoxy-N-{3-methyl-1-[6-oxo-4-(2-pyridin-3-yl-acetyl)-hexahydro-pyrrolo[3,2-b]pyrrol-1-carbonyl]-butyl}-benzamid,
4-Isopropyl-N-{3-methyl-1-[6-oxo-4-(2-pyridin-3-yl-acetyl)-hexahydro-pyrrolo[3,2-b]pyrrol-1-carbonyl]-butyl}-benzamid,
N-{3-Methyl-1-[6-oxo-4-(2-pyridin-3-yl-acetyl)-hexahydro-pyrrolo[3,2-b]pyrrol-1-carbonyl]-butyl}-4-vinyl-benzamid,
4-Imidazol-1-yl-N-{3-methyl-1-[6-oxo-4-(2-pyridin-3-yl-acetyl)-hexahydro-pyrrolo[3,2-b]pyrrol-1-carbonyl]-butyl}-benzamid,
N-{3-Methyl-1-[6-oxo-4-(2-pyridin-3-yl-acetyl)-hexahydro-pyrrolo[3,2-b]pyrrol-1-carbonyl]-butyl}-4-thiophen-2-yl-benzamid,
N-{3-Methyl-1-[6-oxo-4-(2-pyridin-3-yl-acetyl)-hexahydro-pyrrolo[3,2-b]pyrrol-1-carbonyl]-butyl}-4-oxazol-5-yl-benzamid,
5-Phenyl-thiophen-2-carbonsäure-{3-methyl-1-[6-oxo-4-(2-pyridin-3-yl-acetyl)-hexahydro-pyrrolo[3,2-b]pyrrol-1-carbonyl]-butyl}-amid,
N-{3-Methyl-1-[6-oxo-4-(2-pyridin-3-yl-acetyl)-hexahydro-pyrrolo[3,2-b]pyrrol-1-carbonyl]-butyl}-4-trifluormethoxy-benzamid,
5-Pyridin-2-yl-thiophen-2-carbonsäure-{3-methyl-1-[6-oxo-4-(2-pyridin-3-yl-acetyl)-hexahydro-pyrrolo[3,2-b]pyrrol-1-carbonyl]-butyl}-amid,
4-Difluormethoxy-N-{3-methyl-1-[6-oxo-4-(2-pyridin-3-yl-acetyl)-hexahydro-pyrrolo[3,2-b]pyrrol-1-carbonyl]-butyl}-benzamid,
N-{3-Methyl-1-[6-oxo-4-(2-pyridin-3-yl-acetyl)-hexahydro-pyrrolo[3,2-b]pyrrol-1-carbonyl]-butyl}-4-morpholin-4-yl-benzamid,
Naphthalen-2-carbonsäure-{1-[6-oxo-4-(2-pyridin-3-yl-acetyl)-hexahydro-pyrrolo[3,2-b]pyrrol-1-carbonyl]-butyl}-amid,
Chinolin-2-carbonsäure-{1-[6-oxo-4-(2-pyridin-3-yl-acetyl)-hexahydro-pyrrolo[3,2-b]pyrrol-1-carbonyl]-butyl}-amid,
Benzo[b]thiophen-2-carbonsäure-{1-[6-oxo-4-(2-pyridin-3-yl-acetyl)-hexahydro-pyrrolo[3,2-b]pyrrol-1-carbonyl]-butyl}-amid,
Benzofuran-2-carbonsäure-{1-[6-oxo-4-(2-pyridin-3-yl-acetyl)-hexahydro-pyrrolo[3,2-b]pyrrol-1-carbonyl]-butyl}-amid,
Biphenyl-4-carbonsäure-{1-[6-oxo-4-(2-pyridin-3-yl-acetyl)-hexahydro-pyrrolo[3,2-b]pyrrol-1-carbonyl]-butyl}-amid,
4-tert-Butyl-N-{1-[6-oxo-4-(2-pyridin-3-yl-acetyl)-hexahydro-pyrrolo[3,2-b]pyrrol-1-carbonyl]-butyl}-benzamid,
4-Dimethylamino-N-{1-[6-oxo-4-(2-pyridin-3-yl-acetyl)-hexahydro-pyrrolo[3,2-b]pyrrol-1-carbonyl]-butyl}-benzamid,
7-Methoxy-benzofuran-2-carbonsäure-{1-[6-oxo-4-(2-pyridin-3-yl-acetyl)-hexahydro-pyrrolo[3,2-b]pyrrol-1-carbonyl]-butyl}-amid,
{1-[6-Oxo-4-(2-pyridin-3-yl-acetyl)-hexahydro-pyrrolo[3,2-b]pyrrol-1-carbonyl]-butyl}-carbaminsäurebenzylester,
5-Methoxy-benzofuran-2-carbonsäure-{1-[6-oxo-4-(2-pyridin-3-yl-acetyl)-hexahydro-pyrrolo[3,2-b]pyrrol-1-carbonyl]-butyl}-amid,
Thieno[3,2-b]thiophen-2-carbonsäure-{1-[6-oxo-4-(2-pyridin-3-yl-acetyl)-hexahydro-pyrrolo[3,2-b]pyrrol-1-carbonyl]-butyl}-amid,
3-Methyl-benzofuran-2-carbonsäure-{1-[6-oxo-4-(2-pyridin-3-yl-acetyl)-hexahydro-pyrrolo[3,2-b]pyrrol-1-carbonyl]-butyl}amid,
Chinoxalin-2-carbonsäure-{1-[6-oxo-4-(2-pyridin-3-yl-acetyl)-hexahydro-pyrrolo[3,2-b]pyrrol-1-carbonyl]-butyl}-amid,
Benzo[1,3]dioxol-5-carbonsäure-{1-[6-oxo-4-(2-pyridin-3-yl-acetyl)-hexahydro-pyrrolo[3,2-b]pyrrol-1-carbonyl]-butyl}-amid,
Chinolin-6-carbonsäure-{1-[6-oxo-4-(2-pyridin-3-yl-acetyl)-hexahydro-pyrrolo[3,2-b]pyrrol-1-carbonyl]-butyl}-amid,
Furan-2-carbonsäure-{1-[6-oxo-4-(2-pyridin-3-yl-acetyl)-hexahydro-pyrrolo[3,2-b]pyrrol-1-carbonyl]-butyl}-amid,
Thiophen-2-carbonsäure-{1-[6-oxo-4-(2-pyridin-3-yl-acetyl)-hexahydro-pyrrolo[3,2-b]pyrrol-1-carbonyl]-butyl}-amid,
N-{1-[6-Oxo-4-(2-pyridin-3-yl-acetyl)-hexahydro-pyrrolo[3,2-b]pyrrol-1-carbonyl]-butyl}-4-trifluormethyl-benzamid,
Furan-3-carbonsäure-{1-[6-oxo-4-(2-pyridin-3-yl-acetyl)-hexahydro-pyrrolo[3,2-b]pyrrol-1-carbonyl]-butyl}-amid,
Thiophen-3-carbonsäure-{1-[6-oxo-4-(2-pyridin-3-yl-acetyl)-hexahydro-pyrrolo[3,2-b]pyrrol-1-carbonyl]-butyl}-amid,
N-{1-[6-Oxo-4-(2-pyridin-3-yl-acetyl)-hexahydro-pyrrolo[3,2-b]pyrrol-1-carbonyl]-butyl}-4-phenoxy-benzamid,
N-{1-[6-Oxo-4-(2-pyridin-3-yl-acetyl)-hexahydro-pyrrolo[3,2-b]pyrrol-1-carbonyl]-butyl}-4-(4-methyl-piperazin-1-yl)-benzamid,
4-Methyl-N-{1-[6-oxo-4-(2-pyridin-3-yl-acetyl)-hexahydro-pyrrolo[3,2-b]pyrrol-1-carbonyl]-butyl}-benzamid,
4-Methoxy-N-{1-[6-oxo-4-(2-pyridin-3-yl-acetyl)-hexahydro-pyrrolo[3,2-b]pyrrol-1-carbonyl]-butyl}-benzamid,
4-Isopropyl-N-{1-[6-oxo-4-(2-pyridin-3-yl-acetyl)-hexahydro-pyrrolo[3,2-b]pyrrol-1-carbonyl]-butyl}-benzamid,
N-{1-[6-Oxo-4-(2-pyridin-3-yl-acetyl)-hexahydro-pyrrolo[3,2-b]pyrrol-1-carbonyl]-butyl}4-vinyl-benzamid,
4-Imidazol-1-yl-N-{1-[6-oxo-4-(2-pyridin-3-yl-acetyl)-hexahydro-pyrrolo[3,2-b]pyrrol-1-carbonyl]-butyl}-benzamid,
N-{1-[6-Oxo-4-(2-pyridin-3-yl-acetyl)-hexahydro-pyrrolo[3,2-b]pyrrol-1-carbonyl]-butyl}-4-thiophen-2-yl-benzamid,
N-{1-[6-Oxo-4-(2-pyridin-3-yl-acetyl)-hexahydro-pyrrolo[3,2-b]pyrrol-1-carbonyl]-butyl}-4-oxazol-5-yl-benzamid,
5-Phenyl-thiophen-2-carbonsäure-{1-[6-oxo-4-(2-pyridin-3-yl-acetyl)-hexahydro-pyrrolo[3,2-b]pyrrol-1-carbonyl]-butyl}-amid,
N-{1-[6-Oxo-4-(2-pyridin-3-yl-acetyl)-hexahydro-pyrrolo[3,2-b]pyrrol-1-carbonyl]-butyl}-4-trifluormethoxy-benzamid,
5-Pyridin-2-yl-thiophen-2-carbonsäure-{1-[6-oxo-4-(2-pyridin-3-yl-acetyl)-hexahydro-pyrrolo[3,2-b]pyrrol-1-carbonyl]-butyl}-amid,
4-Difluormethoxy-N-{1-[6-oxo-4-(2-pyridin-3-yl-acetyl)-hexahydro-pyrrolo[3,2-b]pyrrol-1-carbonyl]-butyl}-benzamid,
N-{1-[6-Oxo-4-(2-pyridin-3-yl-acetyl)-hexahydro-pyrrolo[3,2-b]pyrrol-1-carbonyl]-butyl}-4-morpholin-4-yl-benzamid,
Naphthalen-2-carbonsäure-{3-methyl-1-[6-oxo-4-(pyridin-2-ylmethansulfonyl)-hexahydro-pyrrolo[3,2-b]pyrrol-1-carbonyl]-butyl}-amid,
Chinolin-2-carbonsäure-{3-methyl-1-[6-oxo-4-(pyridin-2-ylmethansulfonyl)-hexahydro-pyrrolo[3,2-b]pyrrol-1-carbonyl]-butyl}-amid,
Benzo[b]thiophen-2-carbonsäure-{3-methyl-1-[6-oxo-4-(pyridin-2-ylmethansulfonyl)-hexahydro-pyrrolo[3,2-b]pyrrol-1-carbonyl]-butyl}-amid,
Benzofuran-2-carbonsäure-{3-methyl-1-[6-oxo-4-(pyridin-2-ylmethansulfonyl)-hexahydro-pyrrolo[3,2-b]pyrrol-1-carbonyl]-butyl}-amid,
Biphenyl-4-carbonsäure-{3-methyl-1-[6-oxo-4-(pyridin-2-ylmethansulfonyl)-hexahydro-pyrrolo[3,2-b]pyrrol-1-carbonyl]-butyl}-amid,
4-tert-Butyl-N-{3-methyl-1-[6-oxo-4-(pyridin-2-ylmethansulfonyl)-hexahydro-pyrrolo[3,2-b]pyrrol-1-carbonyl]-butyl}-benzamid,
4-Dimethylamino-N-{3-methyl-1-[6-oxo-4-(pyridin-2-ylmethansulfonyl)-hexahydro-pyrrolo[3,2-b]pyrrol-1-carbonyl]-butyl}-benzamid,
7-Methoxy-benzofuran-2-carbonsäure-{3-methyl-1-[6-oxo-4-(pyridin-2-ylmethansulfonyl)-hexahydro-pyrrolo[3,2-b]pyrrol-1-carbonyl]-butyl}-amid,
{3-Methyl-1-[6-oxo-4-(pyridin-2-ylmethansulfonyl)-hexahydro-pyrrolo[3,2-b]pyrrol-1-carbonyl]-butyl}-carbaminsäurebenzylester,
5-Methoxy-benzofuran-2-carbonsäure-{3-methyl-1-[6-oxo-4-(pyridin-2-ylmethansulfonyl)-hexahydro-pyrrolo[3,2-b]pyrrol-1-carbonyl]-butyl}-amid,
Thieno[3,2-b]thiophen-2-carbonsäure-{3-methyl-1-[6-oxo-4-(pyridin-2-ylmethansulfonyl)-hexahydro-pyrrolo[3,2-b]pyrrol-1-carbonyl]-butyl}-amid,
3-Methyl-benzofuran-2-carbonsäure-{3-methyl-1-[6-oxo-4-(pyridin-2-ylmethansulfonyl)-hexahydro-pyrrolo[3,2-b]pyrrol-1-carbonyl]-butyl}-amid,
Chinoxalin-2-carbonsäure-{3-methyl-1-[6-oxo-4-(pyridin-2-ylmethansulfonyl)-hexahydro-pyrrolo[3,2-b]pyrrol-1-carbonyl]-butyl}-amid,
Benzo[1,3]dioxol-5-carbonsäure-{3-methyl-1-[6-oxo-4-(pyridin-2-ylmethansulfonyl)-hexahydro-pyrrolo[3,2-b]pyrrol-1-carbonyl]-butyl}-amid,
Chinolin-6-carbonsäure-{3-methyl-1-[6-oxo-4-(pyridin-2-ylmethansulfonyl)-hexahydro-pyrrolo[3,2-b]pyrrol-1-carbonyl]-butyl}-amid,
Furan-2-carbonsäure-{3-methyl-1-[6-oxo-4-(pyridin-2-ylmethansulfonyl)-hexahydro-pyrrolo[3,2-b]pyrrol-1-carbonyl]-butyl}-amid,
Thiophen-2-carbonsäure-{3-methyl-1-[6-oxo-4-(pyridin-2-ylmethansulfonyl)-hexahydro-pyrrolo[3,2-b]pyrrol-1-carbonyl]-butyl}-amid,
N-{3-Methyl-1-[6-oxo-4-(pyridin-2-ylmethansulfonyl)-hexahydro-pyrrolo[3,2-b]pyrrol-1-carbonyl]-butyl}-4-trifluormethyl-benzamid,
Furan-3-carbonsäure-{3-methyl-1-[6-oxo-4-(pyridin-2-ylmethansulfonyl)-hexahydro-pyrrolo[3,2-b]pyrrol-1-carbonyl]-butyl}-amid,
Thiophen-3-carbonsäure-{3-methyl-1-[6-oxo-4-(pyridin-2-ylmethansulfonyl)-hexahydro-pyrrolo[3,2-b]pyrrol-1-carbonyl]-butyl}-amid,
N-{3-Methyl-1-[6-oxo-4-(pyridin-2-ylmethansulfonyl)-hexahydro-pyrrolo[3,2-b]pyrrol-1-carbonyl]-butyl}-4-phenoxy-benzamid,
N-{3-Methyl-1-[6-oxo-4-(pyridin-2-ylmethansulfonyl)-hexahydro-pyrrolo[3,2-b]pyrrol-1-carbonyl]-butyl}-4-(4-methyl-piperazin-1-yl)-benzamid,
4-Methyl-N-{3-methyl-1-[6-oxo-4-(pyridin-2-ylmethansulfonyl)-hexahydro-pyrrolo[3,2-b]pyrrol-1-carbonyl]-butyl}-benzamid,
4-Methoxy-N-{3-methyl-1-[6-oxo-4-(pyridin-2-ylmethansulfonyl)-hexahydro-pyrrolo[3,2-b]pyrrol-1-carbonyl]-butyl}-benzamid,
4-Isopropyl-N-{3-methyl-1-[6-oxo-4-(pyridin-2-ylmethansulfonyl)-hexahydro-pyrrolo[3,2-b]pyrrol-1-carbonyl]-butyl}-benzamid,
N-{3-Methyl-1-[6-oxo-4-(pyridin-2-ylmethansulfonyl)-hexahydro-pyrrolo[3,2-b]pyrrol-1-carbonyl]-butyl}-4-vinyl-benzamid,
4-Imidazol-1-yl-N-{3-methyl-1-[6-oxo-4-(pyridin-2-ylmethansulfonyl)-hexahydro-pyrrolo[3,2-b]pyrrol-1-carbonyl]-butyl}-benzamid,
N-{3-Methyl-1-[6-oxo-4-(pyridin-2-ylmethansulfonyl)-hexahydro-pyrrolo[3,2-b]pyrrol-1-carbonyl]-butyl}-4-thiophen-2-yl-benzamid,
N-{3-Methyl-1-[6-oxo-4-(pyridin-2-ylmethansulfonyl)-hexahydro-pyrrolo[3,2-b]pyrrol-1-carbonyl]-butyl}-4-oxazol-5-yl-benzamid,
5-Phenyl-thiophen-2-carbonsäure-{3-methyl-1-[6-oxo-4-(pyridin-2-ylmethansulfonyl)-hexahydro-pyrrolo[3,2-b]pyrrol-1-carbonyl]-butyl}-amid,
N-{3-Methyl-1-[6-oxo-4-(pyridin-2-ylmethansulfonyl)-hexahydro-pyrrolo[3,2-b]pyrrol-1-carbonyl]-butyl}-4-trifluormethoxy-benzamid,
5-Pyridin-2-yl-thiophen-2-carbonsäure-{3-methyl-1-[6-oxo-4-(pyridin-2-ylmethansulfonyl)-hexahydro-pyrrolo[3,2-b]pyrrol-1-carbonyl]-butyl}-amid,
4-Difluormethoxy-N-{3-methyl-1-[6-oxo-4-(pyridin-2-ylmethansulfonyl)-hexahydro-pyrrolo[3,2-b]pyrrol-1-carbonyl]-butyl}-benzamid,
N-{3-Methyl-1-[6-oxo-4-(pyridin-2-ylmethansulfonyl)-hexahydro-pyrrolo[3,2-b]pyrrol-1-carbonyl]-butyl}-4-morpholin-4-yl-benzamid,
Naphthalen-2-carbonsäure-{1-[6-oxo-4-(pyridin-2-ylmethansulfonyl)-hexahydro-pyrrolo[3,2-b]pyrrol-1-carbonyl]-butyl}-amid,
Chinolin-2-carbonsäure-{1-[6-oxo-4-(pyridin-2-ylmethansulfonyl)-hexahydro-pyrrolo[3,2-b]pyrrol-1-carbonyl]-butyl}-amid,
Benzo[b]thiophen-2-carbonsäure-{1-[6-oxo-4-(pyridin-2-ylmethansulfonyl)-hexahydro-pyrrolo[3,2-b]pyrrol-1-carbonyl]-butyl}-amid,
Benzofuran-2-carbonsäure-{1-[6-oxo-4-(pyridin-2-ylmethansulfonyl)-hexahydro-pyrrolo[3,2-b]pyrrol-1-carbonyl]-butyl}-amid,
Biphenyl-4-carbonsäure-{1-[6-oxo-4-(pyridin-2-ylmethansulfonyl)-hexahydro-pyrrolo[3,2-b]pyrrol-1-carbonyl]-butyl}-amid,
4-tert-Butyl-N-{1-[6-oxo-4-(pyridin-2-ylmethansulfonyl)-hexahydro-pyrrolo[3,2-b]pyrrol-1-carbonyl]-butyl}-benzamid,
4-Dimethylamino-N-{1-[6-oxo-4-(pyridin-2-ylmethansulfonyl)-hexahydro-pyrrolo[3,2-b]pyrrol-1-carbonyl]-butyl}-benzamid,
7-Methoxy-benzofuran-2-carbonsäure-{1-[6-oxo-4-(pyridin-2-ylmethansulfonyl)-hexahydro-pyrrolo[3,2-b]pyrrol-1-carbonyl]-butyl}-amid,
{1-[6-Oxo-4-(pyridin-2-ylmethansulfonyl)-hexahydro-pyrrolo[3,2-b]pyrrol-1-carbonyl]-butyl}-carbaminsäurebenzylester,
5-Methoxy-benzofuran-2-carbonsäure-{1-[6-oxo-4-(pyridin-2-ylmethansulfonyl)-hexahydro-pyrrolo[3,2-b]pyrrol-1-carbonyl]-butyl}-amid,
Thieno[3,2-b]thiophen-2-carbonsäure-{1-[6-oxo-4-(pyridin-2-ylmethansulfonyl)-hexahydro-pyrrolo[3,2-b]pyrrol-1-carbonyl]-butyl}-amid,
3-Methyl-benzofuran-2-carbonsäure-{1-[6-oxo-4-(pyridin-2-ylmethansulfonyl)-hexahydro-pyrrolo[3,2-b]pyrrol-1-carbonyl]-butyl}-amid,
Chinoxalin-2-carbonsäure-{1-[6-oxo-4-(pyridin-2-ylmethansulfonyl)-hexahydro-pyrrolo[3,2-b]pyrrol-1-carbonyl]-butyl}-amid,
Benzo[1,3]dioxol-5-carbonsäure-{1-[6-oxo-4-(pyridin-2-ylmethansulfonyl)-hexahydro-pyrrolo[3,2-b]pyrrol-1-carbonyl]-butyl}-amid,
Chinolin-6-carbonsäure-{1-[6-oxo-4-(pyridin-2-ylmethansulfonyl)-hexahydro-pyrrolo[3,2-b]pyrrol-1-carbonyl]-butyl}-amid,
Furan-2-carbonsäure-{1-[6-oxo-4-(pyridin-2-ylmethansulfonyl)-hexahydro-pyrrolo[3,2-b]pyrrol-1-carbonyl]-butyl}-amid,
Thiophen-2-carbonsäure-{1-[6-oxo-4-(pyridin-2-ylmethansulfonyl)-hexahydro-pyrrolo[3,2-b]pyrrol-1-carbonyl]-butyl}-amid,
N-{1-[6-Oxo-4-(pyridin-2-ylmethansulfonyl)-hexahydro-pyrrolo[3,2-b]pyrrol-1-carbonyl]-butyl}-4-trifluormethyl-benzamid,
Furan-3-carbonsäure-{1-[6-oxo-4-(pyridin-2-ylmethansulfonyl)-hexahydro-pyrrolo[3,2-b]pyrrol-1-carbonyl]-butyl}-amid,
Thiophen-3-carbonsäure-{1-[6-oxo-4-(pyridin-2-ylmethansulfonyl)-hexahydro-pyrrolo[3,2-b]pyrrol-1-carbonyl]-butyl}-amid,
N-{1-[6-oxo-4-(pyridin-2-ylmethansulfonyl)-hexahydro-pyrrolo[3,2-b]pyrrol-1-carbonyl]-butyl}-4-phenoxy-benzamid,
N-{1-[6-Oxo-4-(pyridin-2-ylmethansulfonyl)-hexahydro-pyrrolo[3,2-b]pyrrol-1-carbonyl]-butyl}-4-(4-methyl-piperazin-1-yl)-benzamid,
4-Methyl-N-{1-[6-oxo-4-(pyridin-2-ylmethansulfonyl)-hexahydro-pyrrolo[3,2-b]pyrrol-1-carbonyl]-butyl}-benzamid,
4-Methoxy-N-{1-[6-oxo-4-(pyridin-2-ylmethansulfonyl)-hexahydro-pyrrolo[3,2-b]pyrrol-1-carbonyl]-butyl}-benzamid,
4-Isopropyl-N-{1-[6-oxo-4-(pyridin-2-ylmethansulfonyl)-hexahydro-pyrrolo[3,2-b]pyrrol-1-carbonyl]-butyl}-benzamid,
N-{1-[6-Oxo-4-(pyridin-2-ylmethansulfonyl)-hexahydro-pyrrolo[3,2-b]pyrrol-1-carbonyl]-butyl}-4-vinyl-benzamid,
4-Imidazol-1-yl-N-{1-[6-oxo-4-(pyridin-2-ylmethansulfonyl)-hexahydro-pyrrolo[3,2-b]pyrrol-1-carbonyl]-butyl}-benzamid,
N-{1-[6-Oxo-4-(pyridin-2-ylmethansulfonyl)-hexahydro-pyrrolo[3,2-b]pyrrol-1-carbonyl]-butyl}-4-thiophen-2-yl-benzamid,
N-{1-[6-Oxo-4-(pyridin-2-ylmethansulfonyl)-hexahydro-pyrrolo[3,2-b]pyrrol-1-carbonyl]-butyl}-4-oxazol-5-yl-benzamid,
5-Phenyl-thiophen-2-carbonsäure-{1-[6-oxo-4-(pyridin-2-ylmethansulfonyl)-hexahydro-pyrrolo[3,2-b]pyrrol-1-carb onyl]-butyl}-amid,
N-{1-[6-Oxo-4-(pyridin-2-ylmethansulfonyl)-hexahydro-pyrrolo[3,2-b]pyrrol-1-carbonyl]-butyl}-4-trifluormethoxy-benzamid,
5-Pyridin-2-yl-thiophen-2-carbonsäure-{1-[6-oxo-4-(pyridin-2-ylmethansulfonyl)-hexahydro-pyrrolo[3,2-b]pyrrol-1-carbonyl]-butyl}-amid,
4-Difluormethoxy-N-{1-[6-oxo-4-(pyridin-2-ylmethansulfonyl)-hexahydro-pyrrolo[3,2-b]pyrrol-1-carbonyl]-butyl}-benzamid,
N-{1-[6-Oxo-4-(pyridin-2-ylmethansulfonyl)-hexahydro-pyrrolo[3,2-b]pyrrol-1-carbonyl]-butyl}-4-morpholin-4-yl-benzamid,
Naphthalen-2-carbonsäure-{3-methyl-1-[6-oxo-4-(pyridin-3-ylmethansulfonyl)-hexahydro-pyrrolo[3,2-b]pyrrol-1-carbonyl]-butyl}-amid,
Chinolin-2-carbonsäure-{3-methyl-1-[6-oxo-4-(pyridin-3-ylmethansulfonyl)-hexahydro-pyrrolo[3,2-b]pyrrol-1-carbonyl]-butyl}-amid,
Benzo[b]thiophen-2-carbonsäure-{3-methyl-1-[6-oxo-4-(pyridin-3-ylmethansulfonyl)-hexahydro-pyrrolo[3,2-b]pyrrol-1-carbonyl]-butyl}-amid,
Benzofuran-2-carbonsäure-{3-methyl-1-[6-oxo-4-(pyridin-3-ylmethansulfonyl)-hexahydro-pyrrolo[3,2-b]pyrrol-1-carbonyl]-butyl}-amid,
Biphenyl-4-carbonsäure-{3-methyl-1-[6-oxo-4-(pyridin-3-ylmethansulfonyl)-hexahydro-pyrrolo[3,2-b]pyrrol-1-carbonyl]-butyl}-amid,
4-tert-Butyl-N-{3-methyl-1-[6-oxo-4-(pyridin-3-ylmethansulfonyl)-hexahydro-pyrrolo[3,2-b]pyrrol-1-carbonyl]-butyl}-benzamid,
4-Dimethylamino-N-{3-methyl-1-[6-oxo-4-(pyridin-3-ylmethansulfonyl)-hexahydro-pyrrolo[3,2-b]pyrrol-1-carbonyl]-butyl}-benzamid,
7-Methoxy-benzofuran-2-carbonsäure-{3-methyl-1-[6-oxo-4-(pyridin-3-ylmethansulfonyl)-hexahydro-pyrrolo[3,2-b]pyrrol-1-carbonyl]-butyl}-amid,
{3-Methyl-1-[6-oxo-4-(pyridin-3-ylmethansulfonyl)-hexahydro-pyrrolo[3,2-b]pyrrol-1-carbonyl]-butyl}-carbaminsäurebenzylester,
5-Methoxy-benzofuran-2-carbonsäure-{3-methyl-1-[6-oxo-4-(pyridin-3-ylmethansulfonyl)-hexahydro-pyrrolo[3,2-b]pyrrol-1-carbonyl]-butyl}-amid,
Thieno[3,2-b]thiophen-2-carbonsäure-{3-methyl-1-[6-oxo-4-(pyridin-3-ylmethansulfonyl)-hexahydro-pyrrolo[3,2-b]pyrrol-1-carbonyl]-butyl}-amid,
3-Methyl-benzofuran-2-carbonsäure-{3-methyl-1-[6-oxo-4-(pyridin-3-ylmethansulfonyl)-hexahydro-pyrrolo[3,2-b]pyrrol-1-carbonyl]-butyl}-amid,
Chinoxalin-2-carbonsäure-{3-methyl-1-[6-oxo-4-(pyridin-3-ylmethansulfonyl)-hexahydro-pyrrolo[3,2-b]pyrrol-1-carbonyl]-butyl}-amid,
Benzo[1,3]dioxol-5-carbonsäure-{3-methyl-1-[6-oxo-4-(pyridin-3-ylmethansulfonyl)-hexahydro-pyrrolo[3,2-b]pyrrol-1-carbonyl]-butyl}-amid,
Chinolin-6-carbonsäure-{3-methyl-1-[6-oxo-4-(pyridin-3-ylmethansulfonyl)-hexahydro-pyrrolo[3,2-b]pyrrol-1-carbonyl]-butyl}-amid,
Furan-2-carbonsäure-{3-methyl-1-[6-oxo-4-(pyridin-3-ylmethansulfonyl)-hexahydro-pyrrolo[3,2-b]pyrrol-1-carbonyl]-butyl}-amid,
Thiophen-2-carbonsäure-{3-methyl-1-[6-oxo-4-(pyridin-3-ylmethansulfonyl)-hexahydro-pyrrolo[3,2-b]pyrrol-1-carbonyl]-butyl}-amid,
N-{3-Methyl-1-[6-oxo-4-(pyridin-3-ylmethansulfonyl)-hexahydro-pyrrolo[3,2-b]pyrrol-1-carbonyl]-butyl}-4-trifluormethyl-benzamid,
Furan-3-carbonsäure-{3-methyl-1-[6-oxo-4-(pyridin-3-ylmethansulfonyl)-hexahydro-pyrrolo[3,2-b]pyrrol-1-carbonyl]-butyl}-amid,
Thiophen-3-carbonsäure-{3-methyl-1-[6-oxo-4-(pyridin-3-ylmethansulfonyl)-hexahydro-pyrrolo[3,2-b]pyrrol-1-carbonyl]-butyl}-amid,
N-{3-Methyl-1-[6-oxo-4-(pyridin-3-ylmethansulfonyl)-hexahydro-pyrrolo[3,2-b]pyrrol-1-carbonyl]-butyl}-4-phenoxy-benzamid,
N-{3-Methyl-1-[6-oxo-4-(pyridin-3-ylmethansulfonyl)-hexahydro-pyrrolo[3,2-b]pyrrol-1-carbonyl]-butyl}-4-(4-methyl-piperazin-1-yl)-benzamid,
4-Methyl-N-{3-methyl-1-[6-oxo-4-(pyridin-3-ylmethansulfonyl)-hexahydro-pyrrolo[3,2-b]pyrrol-1-carbonyl]-butyl}-benzamid,
4-Methoxy-N-{3-methyl-1-[6-oxo-4-(pyridin-3-ylmethansulfonyl)-hexahydro-pyrrolo[3,2-b]pyrrol-1-carbonyl]-butyl}-benzamid,
4-Isopropyl-N-{3-methyl-1-[6-oxo-4-(pyridin-3-ylmethansulfonyl)-hexahydro-pyrrolo[3,2-b]pyrrol-1-carbonyl]-butyl}-benzamid,
N-{3-Methyl-1-[6-oxo-4-(pyridin-3-ylmethansulfonyl)-hexahydro-pyrrolo[3,2-b]pyrrol-1-carbonyl]-butyl}-4-vinyl-benzamid,
4-Imidazol-1-yl-N-[3-methyl-1-[6-oxo-4-(pyridin-3-ylmethansulfonyl)-hexahydro-pyrrolo[3,2-b]pyrrol-1-carbonyl]-butyl}-benzamid,
N-{3-Methyl-1-[6-oxo-4-(pyridin-3-ylmethansulfonyl)-hexahydro-pyrrolo[3,2-b]pyrrol-1-carbonyl]-butyl}-4-thiophen-2-yl-benzamid,
N-{3-Methyl-1-[6-oxo-4-(pyridin-3-ylmethansulfonyl)-hexahydro-pyrrolo[3,2-b]pyrrol-1-carbonyl]-butyl}-4-oxazol-5-yl-benzamid,
5-Phenyl-thiophen-2-carbonsäure-{3-methyl-1-[6-oxo-4-(pyridin-3-ylmethansulfonyl)-hexahydro-pyrrolo[3,2-b]pyrrol-1-carbonyl]-butyl}-amid,
N-{3-Methyl-1-[6-oxo-4-(pyridin-3-ylmethansulfonyl)-hexahydro-pyrrolo[3,2-b]pyrrol-1-carbonyl]-butyl}-4-trifluormethoxy-benzamid,
5-Pyridin-2-yl-thiophen-2-carbonsäure-{3-methyl-1-[6-oxo-4-(pyridin-3-ylmethansulfonyl)-hexahydro-pyrrolo[3,2-b]pyrrol-1-carbonyl}-butyl}-amid,
4-Difluormethoxy-N-{3-methyl-1-[6-oxo-4-(pyridin-3-ylmethansulfonyl)-hexahydro-pyrrolo[3,2-b]pyrrol-1-carbonyl]-butyl}-benzamid,
N-{3-Methyl-1-[6-oxo-4-(pyridin-3-ylmethansulfonyl)-hexahydro-pyrrolo[3,2-b]pyrrol-1-carbonyl]-butyl}-4-morpholin-4-yl-benzamid,
Naphthalen-2-carbonsäure-{1-[6-oxo-4-(pyridin-3-ylmethansulfonyl)-hexahydro-pyrrolo[3,2-b]pyrrol-1-carbonyl]-butyl}-amid,
Chinolin-2-carbonsäure-{1-[6-oxo-4-(pyridin-3-ylmethansulfonyl)-hexahydro-pyrrolo[3,2-b]pyrrol-1-carbonyl]-butyl}-amid,
Benzo[b]thiophen-2-carbonsäure-{1-[6-oxo-4-(pyridin-3-ylmethansulfonyl)-hexahydro-pyrrolo[3,2-b]pyrrol-1-carbonyl]-butyl}-amid,
Benzofuran-2-carbonsäure-{1-[6-oxo-4-(pyridin-3-ylmethansulfonyl)-hexahydro-pyrrolo[3,2-b]pyrrol-1-carbonyl]-butyl}-amid,
Biphenyl-4-carbonsäure-{1-[6-oxo-4-(pyridin-3-ylmethansulfonyl)-hexahydro-pyrrolo[3,2-b]pyrrol-1-carbonyl]-butyl}-amid,
4-tert-Butyl-N-{1-[6-oxo-4-(pyridin-3-ylmethansulfonyl)-hexahydro-pyrrolo[3,2-b]pyrrol-1-carbonyl]-butyl}-benzamid,
4-Dimethylamino-N-{1-[6-oxo-4-(pyridin-3-ylmethansulfonyl)-hexahydro-pyrrolo[3,2-b]pyrrol-1-carbonyl]-butyl}-benzamid,
7-Methoxy-benzofuran-2-carbonsäure-{1-[6-oxo-4-(pyridin-3-ylmethansulfonyl)-hexahydro-pyrrolo[3,2-b]pyrrol-1-carbonyl]-butyl}-amid,
{1-[6-Oxo-4-(pyridin-3-ylmethansulfonyl)-hexahydro-pyrrolo[3,2-b]pyrrol-1-carbonyl]-butyl}-carbaminsäurebenzylester,
5-Methoxy-benzofuran-2-carbonsäure-{1-[6-oxo-4-(pyridin-3-ylmethansulfonyl)-hexahydro-pyrrolo[3,2-b]pyrrol-1-carbonyl]-butyl}-amid,
Thieno[3,2-b]thiophen-2-carbonsäure-{1-[6-oxo-4-(pyridin-3-ylmethansulfonyl)-hexahydro-pyrrolo[3,2-b]pyrrol-1-carbonyl]-butyl}-amid,
3-Methyl-benzofuran-2-carbonsäure-{1-[6-oxo-4-(pyridin-3-ylmethansulfonyl)-hexahydro-pyrrolo[3,2-b]pyrrol-1-carbonyl]-butyl}-amid,
Chinoxalin-2-carbonsäure-{1-[6-oxo-4-(pyridin-3-ylmethansulfonyl)-hexahydro-pyrrolo[3,2-b]pyrrol-1-carbonyl]-butyl}-amid,
Benzo[1,3]dioxol-5-carbonsäure-{1-[6-oxo-4-(pyridin-3-ylmethansulfonyl)-hexahydro-pyrrolo[3,2-b]pyrrol-1-carbonyl]-butyl}-amid,
Chinolin-6-carbonsäure-{1-[6-oxo-4-(pyridin-3-ylmethansulfonyl)-hexahydro-pyrrolo[3,2-b]pyrrol-1-carbonyl]-butyl}-amid,
Furan-2-carbonsäure-{1-[6-oxo-4-(pyridin-3-ylmethansulfonyl)-hexahydro-pyrrolo[3,2-b]pyrrol-1-carbonyl]-butyl}-amid,
Thiophen-2-carbonsäure-{1-[6-oxo-4-(pyridin-3-ylmethansulfonyl)-hexahydro-pyrrolo[3,2-b]pyrrol-1-carbonyl]-butyl}-amid,
N-{1-[6-Oxo-4-(pyridin-3-ylmethansulfonyl)-hexahydro-pyrrolo[3,2-b]pyrrol-1-carbonyl]-butyl}-4-trifluormethyl-benzamid,
Furan-3-carbonsäure-{1-[6-oxo-4-(pyridin-3-ylmethansulfonyl)-hexahydro-pyrrolo[3,2-b]pyrrol-1-carbonyl]-butyl}-amid,
Thiophen-3-carbonsäure-{1-[6-oxo-4-(pyridin-3-ylmethansulfonyl)-hexahydro-pyrrolo[3,2-b]pyrrol-1-carbonyl]-butyl}-amid,
N-{1-[6-Oxo-4-(pyridin-3-ylmethansulfonyl)-hexahydro-pyrrolo[3,2-b]pyrrol-1-carbonyl]-butyl}-4-phenoxy-benzamid,
N-{1-[6-Oxo-4-(pyridin-3-ylmethansulfonyl)-hexahydro-pyrrolo[3,2-b]pyrrol-1-carbonyl]-butyl}-4-(4-methyl-piperazin-1-yl)-benzamid,
4-Methyl-N-{1-[6-oxo-4-(pyridin-3-ylmethansulfonyl)-hexahydro-pyrrolo[3,2-b]pyrrol-1-carbonyl]-butyl}-benzamid,
4-Methoxy-N-{1-[6-oxo-4-(pyridin-3-ylmethansulfonyl)-hexahydro-pyrrolo[3,2-b]pyrrol-1-carbonyl]-butyl}-benzamid,
4-Isopropyl-N-{1-[6-oxo-4-(pyridin-3-ylmethansulfonyl)-hexahydro-pyrrolo[3,2-b]pyrrol-1-carbonyl]-butyl}-benzamid,
N-{1-[6-Oxo-4-(pyridin-3-ylmethansulfonyl)-hexahydro-pyrrolo[3,2-b]pyrrol-1-carbonyl]-butyl}-4-vinyl-benzamid,
4-Imidazol-1-yl-N-{1-[6-oxo-4-(pyridin-3-ylmethansulfonyl)-hexahydro-pyrrolo[3,2-b]pyrrol-1-carbonyl]-butyl}-benzamid,
N-{1-[6-Oxo-4-(pyridin-3-ylmethansulfonyl)-hexahydro-pyrrolo[3,2-b]pyrrol-1-carbonyl]-butyl}-4-thiophen-2-yl-benzamid,
N-{1-[6-Oxo-4-(pyridin-3-ylmethansulfonyl)-hexahydro-pyrrolo[3,2-b]pyrrol-1-carbonyl]-butyl}-4-oxazol-5-yl-benzamid,
5-Phenyl-thiophen-2-carbonsäure-{1-[6-oxo-4-(pyridin-3-ylmethansulfonyl)-hexahydro-pyrrolo[3,2-b]pyrrol-1-carbonyl]-butyl}-amid,
N-{1-[6-Oxo-4-(pyridin-3-ylmethansulfonyl)-hexahydro-pyrrolo[3,2-b]pyrrol-1-carbonyl]-butyl}-4-trifluormethoxy-benzamid,
5-Pyridin-2-yl-thiophen-2-carbonsäure-{1-[6-oxo-4-(pyridin-3-ylmethansulfonyl)-hexahydro-pyrrolo[3,2-b]pyrrol-1-carbonyl]-butyl}-amid,
4-Difluormethoxy-N-{1-[6-oxo-4-(pyridin-3-ylmethansulfonyl)-hexahydro-pyrrolo[3,2-b]pyrrol-1-carbonyl]-butyl}-benzamid,
N-{1-[6-Oxo-4-(pyridin-3-ylmethansulfonyl)-hexahydro-pyrrolo[3,2-b]pyrrol-1-carbonyl]-butyl}-4-morpholin-4-yl-benzamid,
Naphthalen-2-carbonsäure-{3-methyl-1-[6-oxo-4-(1-oxy-pyridin-2-ylmethansulfonyl)-hexahydro-pyrrolo[3,2-b]pyrrol-1-carbonyl]-butyl}-amid,
Chinolin-2-carbonsäure-{3-methyl-1-[6-oxo-4-(1-oxy-pyridin-2-ylmethansulfonyl)-hexahydro-pyrrolo[3,2-b]pyrrol-1-carbonyl]-butyl}-amid,
Benzo[b]thiophen-2-carbonsäure-{3-methyl-1-[6-oxo-4-(1-oxy-pyridin-2-ylmethansulfonyl)-hexahydro-pyrrolo[3,2-b]pyrrol-1-carbonyl]-butyl}-amid,
Benzofuran-2-carbonsäure-{3-methyl-1-[6-oxo-4-(1-oxy-pyridin-2-ylmethansulfonyl)-hexahydro-pyrrolo[3,2-b]pyrrol-1-carbonyl]-butyl}-amid,
Biphenyl-4-carbonsäure-{3-methyl-1-[6-oxo-4-(1-oxy-pyridin-2-ylmethansulfonyl)-hexahydro-pyrrolo[3,2-b]pyrrol-1-carbonyl]-butyl}-amid,
4-tert-Butyl-N-{3-methyl-1-[6-oxo-4-(1-oxy-pyridin-2-ylmethansulfonyl)-hexahydro-pyrrolo[3,2-b]pyrrol-1-carbonyl]-butyl}-benzamid,
4-Dimethylamino-N-{3-methyl-1-[6-oxo-4-(1-oxy-pyridin-2-ylmethansulfonyl)-hexahydro-pyrrolo[3,2-b]pyrrol-1-carbonyl]-butyl}-benzamid,
7-Methoxy-benzofuran-2-carbonsäure-{3-methyl-1-[6-oxo-4-(1-oxy-pyridin-2-ylmethansulfonyl)-hexahydro-pyrrolo[3,2-b]pyrrol-1-carbonyl]-butyl}-amid,
{3-Methyl-1-[6-oxo-4-(1-oxy-pyridin-2-ylmethansulfonyl)-hexahydro-pyrrolo[3,2-b]pyrrol-1-carbonyl]-butyl}-carbaminsäurebenzylester,
5-Methoxy-benzofuran-2-carbonsäure-{3-methyl-1-[6-oxo-4-(1-oxy-pyridin-2-ylmethansulfonyl)-hexahydro-pyrrolo[3,2-b]pyrrol-1-carbonyl]-butyl}-amid,
Thieno[3,2-b]thiophen-2-carbonsäure-{3-methyl-1-[6-oxo-4-(1-oxy-pyridin-2-ylmethansulfonyl)-hexahydro-pyrrolo[3,2-b]pyrrol-1-carbonyl]-butyl}-amid,
3-Methyl-benzofuran-2-carbonsäure-{3-methyl-1-[6-oxo-4-(1-oxy-pyridin-2-ylmethansulfonyl)-hexahydro-pyrrolo[3,2-b]pyrrol-1-carbonyl]-butyl}-amid,
Chinoxalin-2-carbonsäure-{3-methyl-1-[6-oxo-4-(1-oxy-pyridin-2-ylmethansulfonyl)-hexahydro-pyrrolo[3,2-

b]pyrrol-1-carbonyl]-butyl}-amid,
Benzo[1,3]dioxol-5-carbonsäure-{3-methyl-1-[6-oxo-4-(1-oxy-pyridin-2-ylmethansulfonyl)-hexahydro-pyrrolo[3,2-b ]pyrrol-1-carbonyl]-butyl}-amid,
Chinolin-6-carbonsäure-{3-methyl-1-[6-oxo-4-(1-oxy-pyridin-2-ylmethansulfonyl)-hexahydro-pyrrolo[3,2-b]pyrrol-1-carbonyl]-butyl}-amid,
Furan-2-carbonsäure-{3-methyl-1-[6-oxo-4-(1-oxy-pyridin-2-ylmethansulfonyl)-hexahydro-pyrrolo[3,2-b]pyrrol-1-carbonyl]-butyl}-amid,
Thiophen-2-carbonsäure-{3-methyl-1-[6-oxo-4-(1-oxy-pyridin-2-ylmethansulfonyl)-hexahydro-pyrrolo[3,2-b]pyrrol-1-carbonyl]-butyl}-amid,
N-{3-Methyl-1-[6-oxo-4-(1-oxy-pyridin-2-ylmethansulfonyl)-hexahydro-pyrrolo[3,2-b]pyrrol-1-carbonyl]-butyl}-4-trifluormethyl-benzamid,
Furan-3-carbonsäure-{3-methyl-1-[6-oxo-4-(1-oxy-pyridin-2-ylmethansulfonyl)-hexahydro-pyrrolo[3,2-b]pyrrol-1-carbonyl]-butyl}-amid,
Thiophen-3-carbonsäure-{3-methyl-1-[6-oxo-4-(1-oxy-pyridin-2-ylmethansulfonyl)-hexahydro-pyrrolo[3,2-b]pyrrol-1-carbonyl]-butyl}-amid,
N-{3-Methyl-1-[6-oxo-4-(1-oxy-pyridin-2-ylmethansulfonyl)-hexahydro-pyrrolo[3,2-b]pyrrol-1-carbonyl]-butyl}-4-phenoxy-benzamid,
N-{3-Methyl-1-[6-oxo-4-(1-oxy-pyridin-2-ylmethansulfonyl)-hexahydro-pyrrolo[3,2-b]pyrrol-1-carbonyl]-butyl}-4-(4-methyl-piperazin-1-yl)-benzamid,
4-Methyl-N-{3-methyl-1-[6-oxo-4-(1-oxy-pyridin-2-ylmethansulfonyl)-hexahydro-pyrrolo[3,2-b]pyrrol-1-carbonyl]-butyl}benzamid,
4-Methoxy-N-{3-methyl-1-[6-oxo-4-(I-oxy-pyridin-2-ylmethansulfonyl)-hexahydro-pyrrolo[3,2-b]pyrrol-1-carbonyl]-butyl}-benzamid,
4-Isopropyl-N-{3-methyl-1-[6-oxo-4-(1-oxy-pyridin-2-ylmethansulfonyl)-hexahydro-pyrrolo[3,2-b]pyrrol-1-carbonyl]-butyl}-benzamid,
N-{3-Methyl-1-[6-oxo-4-(1-oxy-pyridin-2-ylmethansulfonyl)-hexahydro-pyrrolo[3,2-b]pyrrol-1-carbonyl]-butyl}-4-vinyl-benzamid,
4-Imidazol-1-yl-N-{3-methyl-1-[6-oxo-4-(1-oxy-pyridin-2-ylmethansulfonyl)-hexahydro-pyrrolo[3,2-b]pyrrol-1-carbonyl]-butyl}-benzamid,
N-{3-Methyl-1-[6-oxo-4-(1-oxy-pyridin-2-ylmethansulfonyl)-hexahydro-pyrrolo[3,2-b]pyrrol-1-carbonyl]-butyl}-2-yl-benzamid,
N-{3-Methyl-1-[6-oxo-4-(1-oxy-pyridin-2-ylmethansulfonyl)-hexahydro-pyrrolo[3,2-b]pyrrol-1-carbonyl]-butyl}-4-oxazol-5-yl-benzamid,
5-Phenyl-thiophen-2-carbonsäure-[3-methyl-1-[6-oxo-4-(1-oxy-pyridin-2-ylmethansulfonyl)-hexahydro-pyrrolo[3,2-b]pyrrol-1-carbonyl]-butyl}-amid,
N-{3-Methyl-1-[6-oxo-4-(1-oxy-pyridin-2-ylmethansulfonyl)-hexahydro-pyrrolo[3,2-b]pyrrol-1-carbonyl]-butyl}-4-trifluormethoxy-benzamid
5-Pyridin-2-yl-thiophen-2-carbonsäure-{3-methyl-1-[6-oxo-4-(1-oxy-pyridin-2-ylmethansulfonyl)-hexahydro-pyrrolo[3,2-b]pyrrol-1-carbonyl]-butyl}-amid,
4-Difluormethoxy-N-{3-methyl-1-[6-oxo-4-(1-oxy-pyridin-2-ylmethansulfonyl)-hexahydro-pyrrolo[3,2-b]pyrrol-1-carbonyl]-butyl}-benzamid,
N-{3-Methyl-1-[6-oxo-4-(1-oxy-pyridin-2-ylmethansulfonyl)-hexahydro-pyrrolo[3,2-b]pyrrol-1-carbonyl]-butyl}-4-morpholin-4-yl-benzamid,
Naphthalen-2-carbonsäure-{1-[6-oxo-4-(1-oxy-pyridin-2-ylmethansulfonyl)-hexahydro-pyrrolo[3,2-b]pyrrol-1-carbonyl]-butyl}-amid,
Chinolin-2-carbonsäure-{1-[6-oxo-4-(1-oxy-pyridin-2-ylmethansulfonyl)-hexahydro-pyrrolo[3,2-b]pyrrol-1-carbonyl]-butyl}-amid,
Benzo[b]thiophen-2-carbonsäure-{1-[6-oxo-4-(1-oxy-pyridin-2-ylmethansulfonyl)-hexahydro-pyrrolo[3,2-b]pyrrol-1-carbonyl]-butyl}-amid,
Benzofuran-2-carbonsäure-{1-[6-oxo-4-(1-oxy-pyridin-2-ylmethansulfonyl)-hexahydro-pyrrolo[3,2-b]pyrrol-1-carbonyl]-butyl}-amid,
Biphenyl-4-carbonsäure-{1-[6-oxo-4-(1-oxy-pyridin-2-ylmethansulfonyl)-hexahydro-pyrrolo[3,2-b]pyrrol-1-carbonyl]-butyl}-amid,
4-tert-Butyl-N-{1-[6-oxo-4-(1-oxy-pyridin-2-ylmethansulfonyl)-hexahydro-pyrrolo[3,2-b]pyrrol-1-carbonyl]-butyl}-benzamid,
4-Dimethylamino-N-{1-[6-oxo-4-(1-oxy-pyridin-2-ylmethansulfonyl)-hexahydro-pyrrolo[3,2-b]pyrrol-1-carbonyl]-butyl}-benzamid,
7-Methoxy-benzofuran-2-carbonsäure-{1-[6-oxo-4-(1-oxy-pyridin-2-ylmethansulfonyl)-hexahydro-pyrrolo[3,2-

b]pyrrol-1-carbonyl]-butyl}-amid,
{1-[6-Oxo-4-(1-oxy-pyridin-2-ylmethansulfonyl)-hexahydro-pyrrolo[3,2-b]pyrrol-1-carbonyl]-butyl}-carbaminsäurebenzylester,
5-Methoxy-benzofuran-2-carbonsäure-{1-[6-oxo-4-(1-oxy-pyridin-2-ylmethansulfonyl)-hexahydro-pyrrolo[3,2-b]pyrrol-1-carbonyl]-butyl}-amid,
Thieno[3,2-b]thiophen-2-carbonsäure-{1-[6-oxo-4-(1-oxy-pyridin-2-ylmethansulfonyl)-hexahydro-pyrrolo[3,2-b]pyrrol-1-carbonyl]-butyl}-amid,
3-Methyl-benzofuran-2-carbonsäure-{1-[6-oxo-4-(1-oxy-pyridin-2-ylmethansulfonyl)-hexahydro-pyrrolo[3,2-b]pyrrol-1-carbonyl]-butyl}-amid,
Chinoxalin-2-carbonsäure-{1-[6-oxo-4-(1-oxy-pyridin-2-ylmethansulfonyl)-hexahydro-pyrrolo[3,2-b]pyrrol-1-carbonyl]-butyl}-amid,
Benzo[1,3]dioxol-5-carbonsäure-{1-[6-oxo-4-(1-oxy-pyridin-2-ylmethansulfonyl)-hexahydro-pyrrolo[3,2-b]pyrrol-1-carbonyl]-butyl}-amid,
Chinolin-6-carbonsäure-{1-[6-oxo-4-(1-oxy-pyridin-2-ylmethansulfonyl)-hexahydro-pyrrolo[3,2-b]pyrrol-1-carbonyl]-butyl}-amid,
Furan-2-carbonsäure-{1-[6-oxo-4-(1-oxy-pyridin-2-ylmethansulfonyl)-hexahydro-pyrrolo[3,2-b]pyrrol-1-carbonyl]-butyl}-amid,
Thiophen-2-carbonsäure-{1-[6-oxo-4-(1-oxy-pyridin-2-ylmethansulfonyl)-hexahydro-pyrrolo[3,2-b]pyrrol-1-carbonyl]-butyl}-amid,
N-{1-[6-Oxo-4-(1-oxy-pyridin-2-ylmethansulfonyl)-hexahydro-pyrrolo[3,2-b]pyrrol-1-carbonyl]-butyl}-4-trifluormethyl-benzamid,
Furan-3-carbonsäure-{1-[6-oxo-4-(1-oxy-pyridin-2-ylmethansulfonyl)-hexahydro-pyrrolo[3,2-b]pyrrol-1-carbonyl]-butyl}-amid,
Thiophen-3-carbonsäure-{1-[6-oxo-4-(1-oxy-pyridin-2-ylmethansulfonyl)-hexahydro-pyrrolo[3,2-b]pyrrol-1-carbonyl]-butyl}-amid,
N-{1-[6-Oxo-4-(1-oxy-pyridin-2-ylmethansulfonyl)-hexahydro-pyrrolo[3,2-b]pyrrol-1-carbonyl]-butyl}-4-phenoxy-benzamid,
N-{1-[6-Oxo-4-(1-oxy-pyridin-2-ylmethansulfonyl)-hexahydro-pyrrolo[3,2-b]pyrrol-1-carbonyl]-butyl}-4-(4-methyl-piperazin-1-yl)-benzamid,
4-Methyl-N-{1-[6-oxo-4-(1-oxy-pyridin-2-ylmethansulfonyl)-hexahydro-pyrrolo[3,2-b]pyrrol-1-carbonyl]-butyl}-benzamid,
4-Methoxy-N-{1-[6-oxo-4-(1-oxy-pyridin-2-ylmethansulfonyl)-hexahydro-pyrrolo[3,2-b]pyrrol-1-carbonyl]-butyl}-benzamid,
4-Isopropyl-N-{1-[6-oxo-4-(1-oxy-pyridin-2-ylmethansulfonyl)-hexahydro-pyrrolo[3,2-b]pyrrol-1-carbonyl]-butyl}-benzamid,
N-{1-[6-Oxo-4-(1-oxy-pyridin-2-ylmethansulfonyl)-hexahydro-pyrrolo[3,2-b]pyrrol-1-carbonyl]-butyl}-4-vinyl-benzamid,
4-Imidazol-1-yl-N-{1-[6-oxo-4-(1-oxy-pyridin-2-ylmethansulfonyl)-hexahydro-pyrrolo[3,2-b]pyrrol-1-carbonyl]-butyl}-benzamid,
N-{1-[6-Oxo-4-(1-oxy-pyridin-2-ylmethansulfonyl)-hexahydro-pyrrolo[3,2-b]pyrrol-1-carbonyl]-butyl}-4-thiophen-2-yl-benzamid,
N-{1-[6-Oxo-4-(1-oxy-pyridin-2-ylmethansulfonyl)-hexahydro-pyrrolo[3,2-b]pyrrol-1-carbonyl]-butyl}-4-oxazol-5-yl-benzamid,
5-Phenyl-thiophen-2-carbonsäure-{1-[6-oxo-4-(1-oxy-pyridin-2-ylmethansulfonyl)-hexahydro-pyrrolo[3,2-b]pyrrol-1-carbonyl]-butyl}-amid,
N-{1-[6-Oxo-4-(1-oxy-pyridin-2-ylmethansulfonyl)-hexahydro-pyrrolo[3,2-b]pyrrol-1-carbonyl]-butyl}-4-trifluormethoxy-benzamid,
5-Pyridin-2-yl-thiophen-2-carbonsäure-{1-[6-oxo-4-(1-oxy-pyridin-2-ylmethansulfonyl)-hexahydro-pyrrolo[3,2-b]pyrrol-1-carbonyl]-butyl}-amid,
4-Difluormethoxy-N-{1-[6-oxo-4-(1-oxy-pyridin-2-ylmethansulfonyl)-hexahydro-pyrrolo[3,2-b]pyrrol-1-carbonyl]-butyl}-benzamid,
N-{1-[6-Oxo-4-(1-oxy-pyridin-2-ylmethansulfonyl)-hexahydro-pyrrolo[3,2-b]pyrrol-1-carbonyl]-butyl}-4-morpholin-4-yl-benzamid,
Naphthalen-2-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
Chinolin-2-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
Benzo[b]thiophen-2-carbonsäure-{3-methyl-1-[6-oxo-4-(1-oxy-pyridin-3-ylmethansulfonyl)-hexahydro-pyrrolo[3,2-

b]pyrrol-1-carbonyl]-butyl}-amid,
Benzofuran-2-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
Biphenyl-4-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
4-tert-Butyl-N-{3-methyl-1-[6-oxo-4-(1-oxy-pyridin-3-ylmethansulfonyl)-hexahydro-pyrrolo[3,2-b]pyrrol-1-carbonyl]-butyl}-benzamid,
4-Dimethylamino-N-{3-methyl-1-[6-oxo-4-(1-oxy-pyridin-3-ylmethansulfonyl)-hexahydro-pyrrolo[3,2-b]pyrrol-1-carbonyl]-butyl}-benzamid,
7-Methoxy-benzofuran-2-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
{3-Methyl-1-[6-oxo-4-(1-oxy-pyridin-3-ylmethansulfonyl)-hexahydro-pyrrolo[3,2-b]pyrrol-1-carbonyl]-butyl}-carbaminsäurebenzylester,
5-Methoxy-benzofuran-2-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
Thieno[3,2-b]thiophen-2-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
3-Methyl-benzofuran-2-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
Chinoxalin-2-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
Benzo[1,3]dioxol-5-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
Chinolin-6-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
Furan-2-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
Thiophen-2-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
N-{3-Methyl-1-[6-oxo-4-(1-oxy-pyridin-3-ylmethansulfonyl)-hexahydro-pyrrolo[3,2-b]pyrrol-1-carbonyl]-butyl}-4-trifluormethyl-benzamid,
Furan-3-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
Thiophen-3-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
N-{3-Methyl-1-[6-oxo-4-(1-oxy-pyridin-3-ylmethansulfonyl)-hexahydro-pyrrolo[3,2-b]pyrrol-1-carbonyl]-butyl}-4-phenoxy-benzamid,
N-{3-Methyl-1-[6-oxo-4-(1-oxy-pyridin-3-ylmethansulfonyl)-hexahydro-pyrrolo[3,2-b]pyrrol-1-carbonyl]-butyl}-4-(4-methyl-piperazin-1-yl)-benzamid,
4-Methyl-N-{3-methyl-1-[6-oxo-4-(1-oxy-pyridin-3-ylmethansulfonyl)-hexahydro-pyrrolo[3,2-b]pyrrol-1-carbonyl]-butyl}-benzamid,
4-Methoxy-N-{3-methyl-1-[6-oxo-4-(1-oxy-pyridin-3-ylmethansulfonyl)-hexahydro-pyrrolo[3,2-b]pyrrol-1-carbonyl]-butyl}-benzamid,
4-Isopropyl-N-{3-methyl-1-[6-oxo-4-(1-oxy-pyridin-3-ylmethansulfonyl)-hexahydro-pyrrolo[3,2-b]pyrrol-1-carbonyl]-butyl}-benzamid,
N-{3-Methyl-1-[6-oxo-4-(1-oxy-pyridin-3-ylmethansulfonyl)-hexahydro-pyrrolo[3,2-b]pyrrol-1-carbonyl]-butyl}-4-vinyl-benzamid,
4-Imidazol-1-yl-N-{3-methyl-1-[6-oxo-4-(1-oxy-pyridin-3-ylmethansulfonyl)-hexahydro-pyrrolo[3,2-b]pyrrol-1-carbonyl]-butyl}-benzamid,
N-{3-Methyl-1-[6-oxo-4-(1-oxy-pyridin-3-ylmethansulfonyl)-hexahydro-pyrrolo[3,2-b]pyrrol-1-carbonyl]-butyl}-4-thiophen-2-yl-benzamid,
N-{3-Methyl-1-[6-oxo-4-(1-oxy-pyridin-3-ylmethansulforiyl)-hexahydro-pyrrolo[3,2-b]pyrrol-1-carbonyl]-butyl}-4-oxazol-5-yl-benzamid,
5-Phenyl-thiophen-2-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
N-{3-Methyl-1-[6-oxo-4-(1-oxy-pyridin-3-ylmethansulfonyl)-hexahydro-pyrrolo[3,2-b]pyrrol-1-carbonyl]-butyl}-4-trifluormethoxy-benzamid,
5-Pyridin-2-yl-thiophen-2-carbonsäure-{3-methyl-1-[6-oxo-4-(1-oxy-pyridin-3-ylmethansulfonyl)-hexahydro-pyrrolo[3,2-b]pyrrol-1-carbonyl]-butyl}-amid,
4-Difluormethoxy-N-{3-methyl-1-[6-oxo-4-(1-oxy-pyridin-3-ylmethansulfonyl)-hexahydro-pyrrolo[3,2-b]pyrrol-1-carbonyl]-butyl}-benzamid,
N-{3-Methyl-1-[6-oxo-4-(1-oxy-pyridin-3-ylmethansulfonyl)-hexahydro-pyrrolo[3,2-b]pyrrol-1-carbonyl]-butyl}-4-morpholin-4-yl-benzamid,
Naphthalen-2-carbonsäure-{1-[6-oxo-4-(1-oxy-pyridin-3-ylmethansulfonyl)-hexahydro-pyrrolo[3,2-b]pyrrol-1-carbonyl]-butyl}-amid,
Chinolin-2-carbonsäure-{1-[6-oxo-4-(1-oxy-pyridin-3-ylmethansulfonyl)-hexahydro-pyrrolo[3,2-b]pyrrol-1-carbonyl]-butyl}-amid,
Benzo[b]thiophen-2-carbonsäure-{1-[6-oxo-4-(1-oxy-pyridin-3-ylmethansulfonyl)-hexahydro-pyrrolo[3,2-b]pyrrol-1-carbonyl]-butyl}-amid,
Benzofuran-2-carbonsäure-{1-[6-oxo-4-(1-oxy-pyridin-3-ylmethansulfonyl)-hexahydro-pyrrolo[3,2-b]pyrrol-1-carbonyl]-butyl}-amid,
Biphenyl-4-carbonsäure-{1-[6-oxo-4-(1-oxy-pyridin-3-ylmethansulfonyl)-hexahydro-pyrrolo[3,2-b]pyrrol-1-carbonyl]-butyl}-amid,
4-tert-Butyl-N-{1-[6-oxo-4-(1-oxy-pyridin-3-ylmethansulfonyl)-hexahydro-pyrrolo[3,2-b]pyrrol-1-carbonyl]-butyl}-benzamid,
4-Dimethylamino-N-{1-[6-oxo-4-(1-oxy-pyridin-3-ylmethansulfonyl)-hexahydro-pyrrolo[3,2-b]pyrrol-1-carbonyl]-butyl}-benzamid,
7-Methoxy-benzofuran-2-carbonsäure-{1-[6-oxo-4-(1-oxy-pyridin-3-ylmethansulfonyl)-hexahydro-pyrrolo[3,2-b]pyrrol-1-carbonyl]-butyl}-amid,
{1-[6-Oxo-4-(1-oxy-pyridin-3-ylmethansulfonyl)-hexahydro-pyrrolo[3,2-b]pyrrol-1-carbonyl]-butyl}-carbaminsäurebenzylester,
5-Methoxy-benzofuran-2-carbonsäure-{1-[6-oxo-4-(1-oxy-pyridin-3-ylmethansulfonyl)-hexahydro-pyrrolo[3,2-b]pyrrol-1-carbonyl]-butyl}-amid,
Thieno[3,2-b]thiophen-2-carbonsäure-{1-[6-oxo-4-(1-oxy-pyridin-3-ylmethansulfonyl)-hexahydro-pyrrolo[3,2-b]pyrrol-1-carbonyl]-butyl}-amid,
3-Methyl-benzofuran-2-carbonsäure-{1-[6-oxo-4-(1-oxy-pyridin-3-ylmethansulfonyl)-hexahydro-pyrrolo[3,2-b]pyrrol-1-carbonyl]-butyl}-amid,
Chinoxalin-2-carbonsäure-{1-[6-oxo-4-(1-oxy-pyridin-3-ylmethansulfonyl)-hexahydro-pyrrolo[3,2-b]pyrrol-1-carbonyl]-butyl}-amid,
Benzo[1,3]dioxol-5-carbonsäure-{1-[6-oxo-4-(1-oxy-pyridin-3-ylmethansulfonyl)-hexahydro-pyrrolo[3,2-b]pyrrol-1-carbonyl]-butyl}-amid,
Chinolin-6-carbonsäure-{1-[6-oxo-4-(1-oxy-pyridin-3-ylmethansulfonyl)-hexahydro-pyrrolo[3,2-b]pyrrol-1-carbonyl]-butyl}-amid,
Furan-2-carbonsäure-{1-[6-oxo-4-(1-oxy-pyridin-3-ylmethansulfonyl)-hexahydro-pyrrolo[3,2-b]pyrrol-1-carbonyl]-butyl}-amid,
Thiophen-2-carbonsäure-{1-[6-oxo-4-(1-oxy-pyridin-3-ylmethansulfonyl)-hexahydro-pyrrolo[3,2-b]pyrrol-1-carbonyl]-butyl}-amid,
N-{1-[6-Oxo-4-(1-oxy-pyridin-3-ylmethansulfonyl)-hexahydro-pyrrolo[3,2-b]pyrrol-1-carbonyl]-butyl}-4-trifluormethyl-benzamid,
Furan-3-carbonsäure-{1-[6-oxo-4-(1-oxy-pyridin-3-ylmethansulfonyl)-hexahydro-pyrrolo[3,2-b]pyrrol-1-carbonyl]-butyl}-amid,
Thiophen-3-carbonsäure-{1-[6-oxo-4-(1-oxy-pyridin-3-ylmethansulfonyl)-hexahydro-pyrrolo[3,2-b]pyrrol-1-carbonyl]-butyl}-amid,
N-{1-[6-Oxo-4-(1-oxy-pyridin-3-ylmethansulfonyl)-hexahydro-pyrrolo[3,2-b]pyrrol-1-carbonyl]-butyl}-4-phenoxy-benzamid,
N-{1-[6-Oxo-4-(1-oxy-pyridin-3-ylmethansulfonyl)-hexahydro-pyrrolo[3,2-b]pyrrol-1-carbonyl]-butyl}-4-(4-methyl-piperazin-1-yl)-benzamid,
4-Methyl-N-{1-[6-oxo-4-(1-oxy-pyridin-3-ylmethansulfonyl)-hexahydro-pyrrolo[3,2-b]pyrrol-1-carbonyl]-butyl}-benzamid,
4-Methoxy-N-{1-[6-oxo-4-(1-oxy-pyridin-3-ylmethansulfonyl)-hexahydro-pyrrolo[3,2-b]pyrrol-1-carbonyl]-butyl}-benzamid,
4-Isopropyl-N-{1-[6-oxo-4-(1-oxy-pyridin-3-ylmethansulfonyl)-hexahydro-pyrrolo[3,2-b]pyrrol-1-carbonyl]-butyl}-benzamid,
N-{1-[6-Oxo-4-(1-oxy-pyridin-3-ylmethansulfonyl)-hexahydro-pyrrolo[3,2-b]pyrrol-1-carbonyl]-butyl}-4-vinyl-benzamid,
4-Imidazol-1-yl-N-{1-[6-oxo-4-(1-oxy-pyridin-3-ylmethansulfbnyl)-hexahydro-pyrrolo[3,2-b]pyrrol-1-carbonyl]-butyl}-benzamid,
N-{1-[6-Oxo-4-(1-oxy-pyridin-3-ylmethansulfonyl)-hexahydro-pyrrolo[3,2-b]pyrrol-1-carbonyl]-butyl}-4-thiophen-2-yl-benzamid,
N-{1-[6-Oxo-4-(1-oxy-pyridin-3-ylmethansulfonyl)-hexahydro-pyrrolo[3,2-b]pyrrol-1-carbonyl]-butyl}-4-oxazol-5-yl-benzamid,
5-Phenyl-thiophen-2-carbonsäure-{1-[6-oxo-4-(1-oxy-pyridin-3-ylmethansulfonyl)-hexahydro-pyrrolo[3,2-b]pyrrol-1-carbonyl]-butyl}-amid,
N-{1-[6-Oxo-4-(1-oxy-pyridin-3-ylmethansulfonyl)-hexahydro-pyrrolo[3,2-b]pyrrol-1-carbonyl]-butyl}-4-trifluormethoxy-benzamid,
5-Pyridin-2-yl-thiophen-2-carbonsäure-{1-[6-oxo-4-(1-oxy-pyridin-3-ylmethansulfonyl)-hexahydro-pyrrolo[3,2-b]pyrrol-1-carbonyl]-butyl}-amid,
4-Difluormethoxy-N-{1-[6-oxo-4-(1-oxy-pyridin-3-ylmethansulfonyl)-hexahydro-pyrrolo[3,2-b]pyrrol-1-carbonyl]-butyl}-benzamid,
N-{1-[6-Oxo-4-(1-oxy-pyridin-3-ylmethansulfonyl)-hexahydro-pyrrolo[3,2-b]pyrrol-1-carbonyl]-butyl}-4-morpholin-4-yl-benzamid,
4-tert-Butyl-N-{1-[6-oxo-4-(pyridin-2-sulfonyl)-hexahydro-pyrrolo[3,2-b]pyrrol-1-carbonyl]-cyclohexyl}-benzamid,
5-Methoxy-benzofuran-2-carbonsäure-{1-[6-oxo-4-(pyridin-2-sulfonyl)-hexahydro-pyrrolo[3,2-b]pyrrol-1-carbonyl]-cyclohexyl}-amid,
Thieno[3,2-b]thiophen-2-carbonsäure-{1-[6-oxo-4-(pyridin-2-sulfonyl)-hexahydro-pyrrolo[3,2-b]pyrrol-1-carbonyl]-cyclohexyl}-amid,
3-Methyl-benzofuran-2-carbonsäure-{1-[6-oxo-4-(pyridin-2-sulfonyl)-hexahydro-pyrrolo[3,2-b]pyrrol-1-carbonyl]-cyclohexyl}-amid,
N-{1-[6-Oxo-4-(pyridin-2-sulfonyl)-hexahydro-pyrrolo[3,2-b]pyrrol-1-carbonyl]-cyclohexyl}-4-phenoxy-benzamid,
N-{1-[6-Oxo-4-(pyridin-2-sulfonyl)-hexahydro-pyrrolo[3,2-b]pyrrol-1-carbonyl]-cyclohexyl}-4-thiophen-2-yl-benzamid,
4-tert-Butyl-N-{1-cyclopropylmethyl-2-oxo-2-[6-oxo-4-(pyridin-2-sulfonyl)-hexahydro-pyrrolo[3,2-b]pyrrol-1-yl]-ethyl}-benzamid,
5-Methoxy-benzofuran-2-carbonsäure-{1-cyclopropylmethyl-2-oxo-2-[6-oxo-4-(pyridin-2-sulfonyl)-hexahydro-pyrrolo[3,2-b]pyrrol-1-yl]-ethyl}-amid,
Thieno[3,2-b]thiophen-2-carbonsäure-{1-cyclopropylmethyl-2-oxo-2-[6-oxo-4-(pyridin-2-sulfonyl)-hexahydro-pyrrolo[3,2-b]pyrrol-1-yl]-ethyl}-amid,
3-Methyl-benzofuran-2-carbonsäure-{1-cyclopropylmethyl-2-oxo-2-[6-oxo-4-(pyridin-2-sulfonyl)-hexahydro-pyrrolo[3,2-b]pyrrol-1-yl]-ethyl}-amid,
N-{1-Cyclopropylmethyl-2-oxo-2-[6-oxo-4-(pyridin-2-sulfonyl)-hexahydro-pyrrolo[3,2-b]pyrrol-1-yl]-ethyl}-4-phenoxy-benzamid,
N-{1-Cyclopropylmethyl-2-oxo-2-[6-oxo-4-(pyridin-2-sulfonyl)-hexahydro-pyrrolo[3,2-b]pyrrol-1-yl]-ethyl}-4-thiophen-2-yl-benzamid,
4-tert-Butyl-N-{2-oxo-2-[6-oxo-4-(pyridin-2-sulfonyl)-hexahydro-pyrrolo[3,2-b]pyrrol-1-yl]-1-thiophen-2-ylmethyl-ethyl}-benzamid,
5-Methoxy-benzofuran-2-carbonsäure-{2-oxo-2-[6-oxo-4-(pyridin-2-sulfonyl)-hexahydro-pyrrolo[3,2-b]pyrrol-1-yl]-1-thiophen-2-ylmethyl-ethyl}-amid,
Thieno[3,2-b]thiophen-2-carbonsäure-{2-oxo-2-[6-oxo-4-(pyridin-2-sulfonyl)-hexahydro-pyrrolo[3,2-b]pyrrol-1-yl]-1-thiophen-2-ylmethyl-ethyl}-amid,
3-Methyl-benzofuran-2-carbonsäure-{2-oxo-2-[6-oxo-4-(pyridin-2-sulfonyl)-hexahydro-pyrrolo[3,2-b]pyrrol-1-yl]-1-thiophen-2-ylmethyl-ethyl}-amid,
N-{2-Oxo-2-[6-oxo-4-(pyridin-2-sulfonyl)-hexahydro-pyrrolo[3,2-b]pyrrol-1-yl]-1-thiophen-2-ylmethyl-ethyl}-4-phenoxy-benzamid,
N-{2-Oxo-2-[6-oxo-4-(pyridin-2-sulfonyl)-hexahydro-pyrrolo[3,2-b]pyrrol-1-yl]-1-thiophen-2-ylmethyl-ethyl}-4-thiophen-2-yl-benzamid,
4-tert-Butyl-N-{1-[6-oxo-4-(pyridin-3-sulfonyl)-hexahydro-pyrrolo[3,2-b]pyrrol-1-carbonyl]-cyclohexyl}-benzamid,
5-Methoxy-benzofuran-2-carbonsäure-{1-[6-oxo-4-(pyridin-3-sulfonyl)-hexahydro-pyrrolo[3,2-b]pyrrol-1-carbonyl]-cyclohexyl}-amid,
Thieno[3,2-b]thiophen-2-carbonsäure-{1-[6-oxo-4-(pyridin-3-sulfonyl)-hexahydro-pyrrolo[3,2-b]pyrrol-1-carbonyl]-cyclohexyl}-amid,
3-Methyl-benzofuran-2-carbonsäure-{1-[6-oxo-4-(pyridin-3-sulfonyl)-hexahydro-pyrrolo[3,2-b]pyrrol-1-carbonyl]-cyclohexyl}-amid,
N-{1-[6-Oxo-4-(pyridin-3-sulfonyl)-hexahydro-pyrrolo[3,2-b]pyrrol-1-carbonyl]-cyclohexyl}-4-phenoxy-benzamid,
N-{1-[6-Oxo-4-(pyridin-3-sulfonyl)-hexahydro-pyrrolo[3,2-b]pyrrol-1-carbonyl]-cyclohexyl}-4-thiophen-2-yl-benzamid,
4-tert-Butyl-N-{1-cyclopropylmethyl-2-oxo-2-[6-oxo-4-(pyridin-3-sulfonyl)-hexahydro-pyrrolo[3,2-b]pyrrol-1-yl]-ethyl}-benzamid,
5-Methoxy-benzofuran-2-carbonsäure-{1-cyclopropylmethyl-2-oxo-2-[6-oxo-4-(pyridin-3-sulfonyl)-hexahydro-pyrrolo[3,2-b]pyrrol-1-yl]-ethyl}-amid,
Thieno[3,2-b]thiophen-2-carbonsäure-{1-cyclopropylmethyl-2-oxo-2-[6-oxo-4-(pyridin-3-sulfonyl)-hexahydro-pyrrolo[3,2-b]pyrrol-1-yl]-ethyl}-amid,
3-Methyl-benzofuran-2-carbonsäure-{1-cydopropylmethyl-2-oxo-2-[6-oxo-4-(pyridin-3-sulfonyl)-hexahydro-pyrrolo[3,2-b]pyrrol-1-yl]-ethyl}-amid,
N-{1-Cyclopropylmethyl-2-oxo-2-[6-oxo-4-(pyridin-3-sulfonyl)-hexahydro-pyrrolo[3,2-b]pyrrol-1-yl]-ethyl}-4-phenoxy-benzamid,
N-{1-Cyclopropylmethyl-2-oxo-2-[6-oxo-4-(pyridin-3-sulfonyl)-hexahydro-pyrrolo[3,2-b]pyrrol-1-yl]-ethyl}-4-thiophen-2-yl-benzamid,
4-tert-Butyl-N-{2-oxo-2-[6-oxo-4-(pyridin-3-sulfonyl)-hexahydro-pyrrolo[3,2-b]pyrrol-1-yl]-1-thiophen-2-ylmethyl-ethyl}-benzamid,
5-Methoxy-benzofuran-2-carbonsäure-{2-oxo-2-[6-oxo-4-(pyridin-3-sulfonyl)-hexahydro-pyrrolo[3,2-b]pyrrol-1-yl]-1-thiophen-2-ylmethyl-ethyl}-amid,
Thieno[3,2-b]thiophen-2-carbonsäure-{2-oxo-2-[6-oxo-4-(pyridin-3-sulfonyl)-hexahydro-pyrrolo[3,2-b]pyrrol-1-yl]-1-thiophen-2-ylmethyl-ethyl}-amid,
3-Methyl-benzofuran-2-carbonsäure-{2-oxo-2-[6-oxo-4-(pyridin-3-sulfonyl)-hexahydro-pyrrolo[3,2-b]pyrrol-1-yl]-1-thiophen-2-ylmethyl-ethyl}-amid,
N-{2-Oxo-2-[6-oxo-4-(pyridin-3-sulfonyl)-hexahydro-pyrrolo[3,2-b]pyrrol-1-yl]-1-thiophen-2-ylmethyl-ethyl}-4-phenoxy-benzamid,
N-{2-Oxo-2-[6-oxo-4-(pyridin-3-sulfonyl)-hexahydro-pyrrolo[3,2-b]pyrrol-1-yl]-1-thiophen-2-ylmethyl-ethyl}-4-thiophen-2-yl-benzamid,
4-tert-Butyl-N-{1-[6-oxo-4-(1-oxy-pyridin-2-sulfonyl)-hexahydro-pyrrolo[3,2-b]pyrrol-1-carbonyl]-cyclohexyl}-benzamid,
5-Methoxy-benzofuran-2-carbonsäure-{1-[6-oxo-4-(1-oxy-pyridin-2-sulfonyl)-hexahydro-pyrrolo[3,2-b]pyrrol-1-carbonyl]-cyclohexyl}-amid,
Thieno[3,2-b]thiophen-2-carbonsäure-{1-[6-oxo-4-(1-oxy-pyridin-2-sulfonyl)-hexahydro-pyrrolo[3,2-b]pyrrol-1-carbonyl]-cyclohexyl}-amid,
3-Methyl-benzofuran-2-carbonsäure-{1-[6-oxo-4-(1-oxy-pyridin-2-sulfonyl)-hexahydro-pyrrolo[3,2-b]pyrrol-1-carbonyl]-cyclohexyl}-amid,
N-{1-[6-Oxo-4-(1-oxy-pyridin-2-sulfonyl)-hexahydro-pyrrolo[3,2-b]pyrrol-1-carbonyl]-cyclohexyl}-4-phenoxy-benzamid,
N-{1-[6-Oxo-4-(1-oxy-pyridin-2-sulfonyl)-hexahydro-pyrrolo[3,2-b]pyrrol-1-carbonyl]-cyclohexyl}-4-thiophen-2-yl-benzamid,
4-tert-Butyl-N-{1-cyclopropylmethyl-2-oxo-2-[6-oxo-4-(1-oxy-pyridin-2-sulfonyl)-hexahydro-pyrrolo[3,2-b]pyrrol-1-yl]-ethyl}-benzamid,
5-Methoxy-benzofuran-2-carbonsäure-{1-cyclopropylmethyl-2-oxo-2-[6-oxo-4-(1-oxy-pyridin-2-sulfonyl)-hexahydro-pyrrolo[3,2-b]pyrrol-1-yl]-ethyl}-amid,
Thieno[3,2-b]thiophen-2-carbonsäure-{1-cyclopropylmethyl-2-oxo-2-[6-oxo-4-(1-oxy-pyridin-2-sulfonyl)-hexahydro-pyrrolo[3,2-b]pyrrol-1-yl]-ethyl}-amid,
3-Methyl-benzofuran-2-carbonsäure-{1-cyclopropylmethyl-2-oxo-2-[6-oxo-4-(1-oxy-pyridin-2-sulfonyl)-hexahydro-pyrrolo[3,2-b]pyrrol-1-yl]-ethyl}-amid,
N-{1-Cyclopropylmethyl-2-oxo-2-[6-oxo-4-(1-oxy-pyridin-2-sulfonyl)-hexahydro-pyrrolo[3,2-b]pyrrol-1-yl]-ethyl}-4-phenoxy-benzamid,
N-{1-Cyclopropylmethyl-2-oxo-2-[6-oxo-4-(1-oxy-pyridin-2-sulfonyl)-hexahydro-pyrrolo[3,2-b]pyrrol-1-yl]-ethyl}-4-thiophen-2-yl-benzamid,
4-tert-Butyl-N-{2-oxo-2-[6-oxo-4-(1-oxy-pyridin-2-sulfonyl)-hexahydro-pyrrolo[3,2-b]pyrrol-1-yl]-1-thiophen-2-ylmethyl-ethyl}-benzamid,
5-Methoxy-benzofuran-2-carbonsäure-{2-oxo-2-[6-oxo-4-(1-oxy-pyridin-2-sulfonyl)-hexahydro-pyrrolo[3,2-b]pyrrol-1-yl]-1-thiophen-2-ylmethyl-ethyl}-amid,
Thieno[3,2-b]thiophen-2-carbonsäure-{2-oxo-2-[6-oxo-4-(1-oxy-pyridin-2-sulfonyl)-hexahydro-pyrrolo[3,2-b]pyrrol-1-yl]-1-thiophen-2-ylmethyl-ethyl}-amid,
3-Methyl-benzofuran-2-carbonsäure-{2-oxo-2-[6-oxo-4-(1-oxy-pyridin-2-sulfonyl)-hexahydro-pyrrolo[3,2-b]pyrrol-1-yl]-1-thiophen-2-ylmethyl-ethyl}-amid,
N-{2-Oxo-2-[6-oxo-4-(1-oxy-pyridin-2-sulfonyl)-hexahydro-pyrrolo[3,2-b]pyrrol-1-yl]-1-thiophen-2-ylmethyl-ethyl}-4-phenoxy-benzamid,
N-{2-Oxo-2-[6-oxo-4-(1-oxy-pyridin-2-sulfonyl)-hexahydro-pyrrolo[3,2-b]pyrrol-1-yl]-1-thiophen-2-ylmethyl-ethyl}-4-thiophen-2-yl-benzamid,
4-tert-Butyl-N-{1-[6-oxo-4-(1-oxy-pyridin-3-sulfonyl)-hexahydro-pyrrolo[3,2-b]pyrrol-1-carbonyl]-cyclohexyl}-benzamid,
5-Methoxy-benzofuran-2-carbonsäure-{1-[6-oxo-4-(1-oxy-pyridin-3-sulfonyl)-hexahydro-pyrrolo[3,2-b]pyrrol-1-carbonyl]-cyclohexyl}-amid,
Thieno[3,2-b]thiophen-2-carbonsäure-{1-[6-oxo-4-(1-oxy-pyridin-3-sulfonyl)-hexahydro-pyrrolo[3,2-b]pyrrol-1-carbonyl]-cyclohexyl}-amid,
3-Methyl-benzofuran-2-carbonsäure-{1-[6-oxo-4-(1-oxy-pyridin-3-sulfonyl)-hexahydro-pyrrolo[3,2-b]pyrrol-1-carbonyl]-cyclohexyl}-amid,
N-{1-[6-Oxo-4-(1-oxy-pyridin-3-sulfonyl)-hexahydro-pyrrolo[3,2-b]pyrrol-1-carbonyl]-cyclohexyl}-4-phenoxy-benzamid,
N-{1-[6-Oxo-4-(1-oxy-pyridin-3-sulfonyl)-hexahydro-pyrrolo[3,2-b]pyrrol-1-carbonyl]-cyclohexyl}-4-thiophen-2-yl-benzamid,
4-tert-Butyl-N-{1-cyclopropylmethyl-2-oxo-2-[6-oxo-4-(1-oxy-pyridin-3-sulfonyl)-hexahydro-pyrrolo[3,2-b]pyrrol-1-yl]-ethyl}-benzamid,
5-Methoxy-benzofuran-2-carbonsäure-{1-cyclopropylmethyl-2-oxo-2-[6-oxo-4-(1-oxy-pyridin-3-sulfonyl)-hexahydro-pyrrolo[3,2-b]pyrrol-1-yl]-ethyl}-amid,
Thieno[3,2-b]thiophen-2-carbonsäure-{1-cyclopropylmethyl-2-oxo-2-[6-oxo-4-(1-oxy-pyridin-3-sulfonyl)-hexahydro-pyrrolo[3,2-b]pyrrol-1-yl]-ethyl}-amid,
3-Methyl-benzofuran-2-carbonsäure-{1-cyclopropylmethyl-2-oxo-2-[6-oxo-4-(1-oxy-pyridin-3-sulfonyl)-hexahydro-pyrrolo[3,2-b]pyrrol-1-yl]-ethyl}-amid,
N-{1-Cyclopropylmethyl-2-oxo-2-[6-oxo-4-(1-oxy-pyridin-3-sulfonyl)-hexahydro-pyrrolo[3,2-b]pyrrol-1-yl]-ethyl}-4-phenoxy-benzamid,
N-{1-Cyclopropylmethyl-2-oxo-2-[6-oxo-4-(1-oxy-pyridin-3-sulfonyl)-hexahydro-pyrrolo[3,2-b]pyrrol-1-yl]-ethyl}-4-thiophen-2-yl-benzamid,
4-tert-Butyl-N-{2-oxo-2-[6-oxo-4-(1-oxy-pyridin-3-sulfonyl)-hexahydro-pyrrolo[3,2-b]pyrrol-1-yl]-1-thiophen-2-ylmethyl-ethyl}-benzamid,
5-Methoxy-benzofuran-2-carbonsäure{2-oxo-2-[6-oxo-4-(1-oxy-pyridin-3-sulfonyl)-hexahydro-pyrrolo[3,2-b]pyrrol-1-yl]-1-thiophen-2-ylmethyl-ethyl}-amid,
Thieno[3,2-b]thiophen-2-carbonsäure-{2-oxo-2-[6-oxo-4-(1-oxy-pyridin-3-sulfonyl)-hexahydro-pyrrolo[3,2-b]pyrrol-1-yl]-1-thiophen-2-ylmethyl-ethyl}-amid,
3-Methyl-benzofuran-2-carbonsäure-{2-oxo-2-[6-oxo-4-(1-oxy-pyridin-3-sulfonyl)-hexahydro-pyrrolo[3,2-b]pyrrol-1-yl]-1-thiophen-2-ylmethyl-ethyl}-amid,
N-{2-Oxo-2-[6-oxo-4-(1-oxy-pyridin-3-sulfonyl)-hexahydro-pyrrolo[3,2-b]pyrrol-1-yl]-1-thiophen-2-ylmethyl-ethyl}-4-phenoxy-benzamid,
N-{2-Oxo-2-[6-oxo-4-(1-oxy-pyridin-3-sulfonyl)-hexahydro-pyrrolo[3,2-b]pyrrol-1-yl]-1-thiophen-2-ylmethyl-ethyl}-4-thiophen-2-yl-benzamid,
4-tert-Butyl-N-{1-[6-oxo-4-(pyridin-2-carbonyl)-hexahydro-pyrrolo[3,2-b]pyrrol-1-carbonyl]-cyclohexyl}-benzamid,
5-Methoxy-benzofuran-2-carbonsäure-{1-[6-oxo-4-(pyridin-2-carbonyl)-hexahydro-pyrrolo[3,2-b]pyrrol-1-carbonyl]-cyclohexyl}-amid,
Thieno[3,2-b]thiophen-2-carbonsäure-{1-[6-oxo-4-(pyridin-2-carbonyl)-hexahydro-pyrrolo[3,2-b]pyrrol-1-carbonyl]-cyclohexyl}-amid,
3-Methyl-benzofuran-2-carbonsäure-{1-[6-oxo-4-(pyridin-2-carbonyl)-hexahydro-pyrrolo[3,2-b]pyrrol-1-carbonyl]-cyclohexyl}-amid,
N-{1-[6-Oxo-4-(pyridin-2-carbonyl)-hexahydro-pyrrolo[3,2-b]pyrrol-1-carbonyl]-cyclohexyl}-4-phenoxy-benzamid,
N-{1-[6-Oxo-4-(pyridin-2-carbonyl)-hexahydro-pyrrolo[3,2-b]pyrrol-1-carbonyl]-cyclohexyl}-4-thiophen-2-yl-benzamid,
4-tert-Butyl-N-{1-cyclopropylmethyl-2-oxo-2-[6-oxo-4-(pyridin-2-carbonyl)-hexahydro-pyrrolo[3,2-b]pyrrol-1-yl]-ethyl}-benzamid,
5-Methoxy-benzofuran-2-carbonsäure-{1-cyclopropylmethyl-2-oxo-2-[6-oxo-4-(pyridin-2-carbonyl)-hexahydro-pyrrolo[3,2-b]pyrrol-1-yl]-ethyl}-amid,
Thieno[3,2-b]thiophen-2-carbonsäure-{1-cyclopropylmethyl-2-oxo-2-[6-oxo-4-(pyridin-2-carbonyl)-hexahydro-pyrrolo[3,2-b]pyrrol-1-yl]-ethyl}-amid,
3-Methyl-benzofuran-2-carbonsäure-{1-cyclopropylmethyl-2-oxo-2-[6-oxo-4-(pyridin-2-carbonyl)-hexahydro-pyrrolo[3,2-b]pyrrol-1-yl]-ethyl}-amid,
N-{1-Cyclopropylmethyl-2-oxo-2-[6-oxo-4-(pyridin-2-carbonyl)-hexahydro-pyrrolo[3,2-b]pyrrol-1-yl]-ethyl}-4-phenoxy-benzamid,
N-{1-Cyclopropylmethyl-2-oxo-2-[6-oxo-4-(pyridin-2-carbonyl)-hexahydro-pyrrolo[3,2-b]pyrrol-1-yl]-ethyl}-4-thiophen-2-yl-benzamid,
4-tert-Butyl-N-{2-oxo-2-[6-oxo-4-(pyridin-2-carbonyl)-hexahydro-pyrrolo[3,2-b]pyrrol-1-yl]-1-thiophen-2-ylmethyl-ethyl}-benzamid,
5-Methoxy-benzofuran-2-carbonsäure-{2-oxo-2-[6-oxo-4-(pyridin-2-carbonyl)-hexahydro-pyrrolo[3,2-b]pyrrol-1-yl]-1-thiophen-2-ylmethyl-ethyl}-amid,
Thieno[3,2-b]thiophen-2-carbonsäure-{2-oxo-2-[6-oxo-4-(pyridin-2-carbonyl)-hexahydro-pyrrolo[3,2-b]pyrrol-1-yl]-1-thiophen-2-ylmethyl-ethyl}-amid,
3-Methyl-benzofuran-2-carbonsäure-{2-oxo-2-[6-oxo-4-(pyridin-2-carbonyl)-hexahydro-pyrrolo[3,2-b]pyrrol-1-yl]-1-thiophen-2-ylmethyl-ethyl}-amid,
N-{2-Oxo-2-[6-oxo-4-(pyridin-2-carbonyl)-hexahydro-pyrrolo[3,2-b]pyrrol-1-yl]-1-thiophen-2-ylmethyl-ethyl}-4-phenoxy-benzamid,
N-{2-Oxo-2-[6-oxo-4-(pyridin-2-carbonyl)-hexahydro-pyrrolo[3,2-b]pyrrol-1-yl]-1-thiophen-2-ylmethyl-ethyl}-4-thiophen-2-yl-benzamid,
4-tert-Butyl-N-{1-[6-oxo-4-(2-pyridin-3-yl-acetyl)-hexahydro-pyrrolo[3,2-b]pyrrol-1-carbonyl]-cyclohexyl}-benzamid,
5-Methoxy-benzofuran-2-carbonsäure-{1-[6-oxo-4-(2-pyridin-3-yl-acetyl)-hexahydro-pyrrolo[3,2-b]pyrrol-1-carbonyl]-cyclohexyl}-amid,
Thieno[3,2-b]thiophen-2-carbonsäure-{1-[6-oxo-4-(2-pyridin-3-yl-acetyl)-hexahydro-pyrrolo[3,2-b]pyrrol-1-carbonyl]-cyclohexyl}-amid,
3-Methyl-benzofuran-2-carbonsäure-{1-[6-oxo-4-(2-pyridin-3-yl-acetyl)-hexahydro-pyrrolo[3,2-b]pyrrol-1-carbonyl]-cyclohexyl}-amid,
N-{1-[6-Oxo-4-(2-pyridin-3-yl-acetyl)-hexahydro-pyrrolo[3,2-b]pyrrol-1-carbonyl]-cyclohexyl}-4-phenoxy-benzamid,
N-{1-[6-Oxo-4-(2-pyridin-2-yl-acetyl)-hexahydro-pyrrolo[3,2-b]pyrrol-1-carbonyl]-cyclohexyl}-4-thiophen-2-yl-benzamid,
4-tert-Butyl-N-{1-cyclopropylmethyl-2-oxo-2-[6-oxo-4-(2-pyridin-3-yl-acetyl)-hexahydro-pyrrolo[3,2-b]pyrrol-1-yl]-ethyl}-benzamid,
5-Methoxy-benzofuran-2-carbonsäure-{1-cyclopropylmethyl-2-oxo-2-[6-oxo-4-(2-pyridin-3-yl-acetyl)-hexahydro-pyrrolo[3,2-b]pyrrol-1-yl]-ethyl}-amid,
Thieno[3,2-b]thiophen-2-carbonsäure-{1-cyclopropylmethyl-2-oxo-2-[6-oxo-4-(2-pyridin-3-yl-acetyl)-hexahydro-pyrrolo[3,2-b]pyrrol-1-yl]-ethyl}-amid,
3-Methyl-benzofuran-2-carbonsäure-{1-cyclopropylmethyl-2-oxo-2-[6-oxo-4-(2-pyridin-3-yl-acetyl)-hexahydro-pyrrolo[3,2-b]pyrrol-1-yl]-ethyl}-amid,
N-{1-Cyclopropylmethyl-2-oxo-2-[6-oxo-4-(2-pyridin-3-yl-acetyl)-hexahydro-pyrrolo[3,2-b]pyrrol-1-yl]-ethyl}-4-phenoxy-benzamid,
N-{1-Cyclopropylmethyl-2-oxo-2-[6-oxo-4-(2-pyridin-3-yl-acetyl)-hexahydro-pyrrolo[3,2-b]pyrrol-1-yl]-ethyl}-4-thiophen-2-yl-benzamid,
4-tert-Butyl-N-{2-oxo-2-[6-oxo-4-(2-pyridin-3-yl-acetyl)-hexahydro-pyrrolo[3,2-b]pyrrol-1-yl]-1-thiphen-2-ylmethyl-ethyl}-benzamid,
5-Methoxy-benzofuran-2-carbonsäure-{2-oxo-2-[6-oxo-4-(2-pyridin-3-yl-acetyl)-hexahydro-pyrrolo[3,2-b]pyrrol-1-yl]-1-thiophen-2-ylmethyl-ethyl}-amid,
Thieno[3,2-b]thiophen-2-carbonsäure-{2-oxo-2-[6-oxo-4-(2-pyridin-3-yl-acetyl)-hexahydro-pyrrolo[3,2-b]pyrrol-1-yl]-1-thiophen-2-ylmethyl-ethyl}-amid,
3-Methyl-benzofuran-2-carbonsäure-{2-oxo-2-[6-oxo-4-(2-pyridin-3-yl-acetyl)-hexahydro-pyrrolo[3,2-b]pyrrol-1-yl]-1-thiophen-2-ylmethyl-ethyl}-amid,
N-{2-Oxo-2-[6-oxo-4-(2-pyridin-3-yl-acetyl)-hexahydro-pyrrolo[3,2-b]pyrrol-1-yl]-1-thiophen-2-ylmethyl-ethyl}-4-phenoxy-benzamid,
N-{2-Oxo-2-[6-oxo-4-(2-pyridin-3-yl-acetyl)-hexahydro-pyrrolo[3,2-b]pyrrol-1-yl]-1-thiophen-2-ylmethyl-ethyl}-4-thiophen-2-yl-benzamid,
4-tert-Butyl-N-{1-[6-oxo-4-(pyridin-2-ylmethansulfonyl)-hexahydro-pyrrolo[3,2-b]pyrrol-1-carbonyl]-cyclohexyl}-benzamid,
5-Methoxy-benzofuran-2-carbonsäure-{1-[6-oxo-4-(pyridin-2-ylmethansulfonyl)-hexahydro-pyrrolo[3,2-b]pyrrol-1-carbonyl]-cyclohexyl}-amid,
Thieno[3,2-b]thiophen-2-carbonsäure-{1-[6-oxo-4-(pyridin-2-ylmethansulfonyl)-hexahydro-pyrrolo[3,2-b]pyrrol-1-carbonyl]-cyclohexyl}-amid,
3-Methyl-benzofuran-2-carbonsäure-{1-[6-oxo-4-(pyridin-2-ylmethansulfonyl)-hexahydro-pyrrolo[3,2-b]pyrrol-1-carbonyl]-cyclohexyl}-amid,
N-{1-[6-Oxo-4-(pyridin-2-ylmethansulfonyl)-hexahydro-pyrrolo[3,2-b]pyrrol-1-carbonyl]-cyclohexyl}-4-phenoxy-benzamid,
N-{1-[6-Oxo-4-(pyridin-2-ylmethansulfonyl)-hexahydro-pyrrolo[3,2-b]pyrrol-1-carbonyl]-cyclohexyl}-4-thiophen-2-yl-benzamid,
4-tert-Butyl-N-{1-cyclopropylmethyl-2-oxo-2-[6-oxo-4-(pyridin-2-ylmethansulfonyl)-hexahydro-pyrrolo[3,2-b]pyrrol-1-yl]-ethyl}-benzamid,
5-Methoxy-benzofuran-2-carbonsäure-{1-cyclopropylmethyl-2-oxo-2-[6-oxo-4-(pyridin-2-ylmethansulfonyl)-hexahydro-pyrrolo[3,2-b]pyrrol-1-yl]-ethyl}-amid,
Thieno[3,2-b]thiophen-2-carbonsäure-{1-cyclopropylmethyl-2-oxo-2-[6-oxo-4-(pyridin-2-ylmethansulfonyl)-hexahydro-pyrrolo[3,2-b]pyrrol-1-yl]-ethyl}-amid,
3-Methyl-benzofuran-2-carbonsäure-{1-cyclopropylmethyl-2-oxo-2-[6-oxo-4-(pyridin-2-ylmethansulfonyl)-hexahydro-pyrrolo[3,2-b]pyrrol-1-yl]-ethyl}-amid,
N-{1-Cyclopropylmethyl-2-oxo-2-[6-oxo-4-(pyridin-2-ylmethansulfonyl)-hexahydro-pyrrolo[3,2-b]pyrrol-1-yl]-ethyl}-4-phenoxy-benzamid,
N-{1-Cyclopropylmethyl-2-oxo-2-[6-oxo-4-(pyridin-2-ylmethansulfonyl)-hexahydro-pyrrolo[3,2-b]pyrrol-1-yl]-ethyl}-4-thiophen-2-yl-benzamid,
4-tert-Butyl-N-{2-oxo-2-[6-oxo-4-(pyridin-2-ylmethansulfonyl)-hexahydro-pyrrolo[3,2-b]pyrrol-1-yl]-1-thiophen-2-ylmethyl-ethyl}-benzamid,
5-Methoxy-benzofuran-2-carbonsäure-{2-oxo-2-[6-oxo-4-(pyridin-2-ylmethansulfonyl)-hexahydro-pyrrolo[3,2-b]pyrrol-1-yl]-1-thiophen-2-ylmethyl-ethyl}-amid,
Thieno[3,2-b]thiophen-2-carbonsäure-{2-oxo-2-[6-oxo-4-(pyridin-2-ylmethansulfonyl)-hexahydro-pyrrolo[3,2-b]pyrrol-1-yl]-1-thiophen-2-ylmethyl-ethyl}-amid,
3-Methyl-benzofuran-2-carbonsäure-{2-oxo-2-[6-oxo-4-(pyridin-2-ylmethansulfonyl)-hexahydro-pyrrolo[3,2-b]pyrrol-1-yl]-1-thiophen-2-ylmethyl-ethyl}-amid,
N-{2-Oxo-2-[6-oxo-4-(pyridin-2-ylmethansulfonyl)-hexahydro-pyrrolo[3,2-b]pyrrol-1-yl]-1-thiophen-2-ylmethyl-ethyl}-4-phenoxy-benzamid,
N-{2-Oxo-2-[6-oxo-4-(pyridin-2-ylmethansulfonyl)-hexahydro-pyrrolo[3,2-b]pyrrol-1-yl]-1-thiophen-2-ylmethyl-ethyl}-4-thiophen-2-yl-benzamid,
4-tert-Butyl-N-{1-[6-oxo-4-(pyridin-3-ylmethansulfonyl)-hexahydro-pyrrolo[3,2-b]pyrrol-1-carbonyl]-cyclohexyl}-benzamid,
5-Methoxy-benzofuran-2-carbonsäure-{1-[6-oxo-4-(pyridin-3-ylmethansulfonyl)-hexahydro-pyrrolo[3,2-b]pyrrol-1-carbonyl]-cyclohexyl}-amid,
Thieno[3,2-b]thiophen-2-carbonsäure-{1-[6-oxo-4-(pyridin-3-ylmethansulfonyl)-hexahydro-pyrrolo[3,2-b]pyrrol-1-carbonyl]-cyclohexyl}-amid,
3-Methyl-benzofuran-2-carbonsäure-{1-[6-oxo-4-(pyridin-3-ylmethansulfonyl)-hexahydro-pyrrolo[3,2-b]pyrrol-1-carbonyl]-cyclohexyl}-amid,
N-{1-[6-Oxo-4-(pyridin-3-ylmethansulfonyl)-hexahydro-pyrrolo[3,2-b]pyrrol-1-carbonyl]-cyclohexyl}-4-phenoxy-benzamid,
N-{1-[6-Oxo-4-(pyridin-3-ylmethansulfonyl)-hexahydro-pyrrolo[3,2-b]pyrrol-1-carbonyl]-cyclohexyl}-4-thiophen-2-yl-benzamid,
4-tert-Butyl-N-{1-cyclopropylmethyl-2-oxo-2-[6-oxo-4-(pyridin-3-ylmethansulfonyl)-hexahydro-pyrrolo[3,2-b]pyrrol-1-yl]-ethyl}-benzamid,
5-Methoxy-benzofuran-2-carbonsäure-{1-cyclopropylmethyl-2-oxo-2-[6-oxo-4-(pyridin-3-ylmethansulfonyl)-hexahydro-pyrrolo[3,2-b]pyrrol-1-yl]-ethyl}-amid,
Thieno[3,2-b]thiophen-2-carbonsäure-{1-cyclopropylmethyl-2-oxo-2-[6-oxo-4-(pyridin-3-ylmethansulfonyl)-hexahydro-pyrrolo[3,2-b]pyrrol-1-yl]-ethyl}-amid,
3-Methyl-benzofuran-2-carbonsäure-{1-cyclopropylmethyl-2-oxo-2-[6-oxo-4-(pyridin-3-ylmethansulfonyl)-hexahydro-pyrrolo[3,2-b]pyrrol-1-yl]-ethyl}-amid,
N-{1-Cyclopropylmethyl-2-oxo-2-[6-oxo-4-(pyridin-3-ylmethansulfonyl)-hexahydro-pyrrolo[3,2-b]pyrrol-1-yl]-ethy}-4-phenoxy-benzamid,
N-{1-Cyclopropylmethyl-2-oxo-2-[6-oxo-4-(pyridin-3-ylmethansulfonyl)-hexahydro-pyrrolo[3,2-b]pyrrol-1-yl]-ethyl}-4-thiophen-2-yl-benzamid,
4-tert-Butyl-N-{2-oxo-2-[6-oxo-4-(pyridin-3-ylmethansulfonyl)-hexahydro-pyrrolo[3,2-b]pyrrol-1-yl]-1-thiophen-2-ylmethyl-ethyl}-benzamid,
5-Methoxy-benzofuran-2-carbonsäure-{2-oxo-2-[6-oxo-4-(pyridin-3-ylmethansulfonyl)-hexahydro-pyrrolo[3,2-b]pyrrol-1-yl]-1-thiophen-2-ylmethyl-ethyl}-amid,
Thieno[3,2-b]thiophen-2-carbonsäure-{2-oxo-2-[6-oxo-4-(pyridin-3-ylmethansulfonyl)-hexahydro-pyrrolo[3,2-b]pyrrol-1-yl]-1-thiophen-2-ylmethyl-ethyl}-amid,
3-Methyl-benzofuran-2-carbonsäure-{2-oxo-2-[6-oxo-4-(pyridin-3-ylmethansulfonyl)-hexahydro-pyrrolo[3,2-

b]pyrrol-1-yl]-1-thiophen-2-ylmethyl-ethyl}-amid,
N-{2-Oxo-2-[6-oxo-4-(pyridin-3-ylmethansulfonyl)-hexahydro-pyrrolo[3,2-b]pyrrol-1-yl]-1-thiophen-2-ylmethyl-ethyl}-4-phenoxy-benzamid,
N-{2-Oxo-2-[6-oxo-4-(pyridin-3-ylmethansulfonyl)-hexahydro-pyrrolo[3,2-b]pyrrol-1-yl]-1-thiophen-2-ylmethyl-ethyl}-4-thiophen-2-yl-benzamid,
4-tert-Butyl-N-{1-[6-oxo-4-(1-oxy-pyridin-2-ylmethansulfonyl)-hexahydro-pyrrolo[3,2-b]pyrrol-1-carbonyl]-cyclohexyl}-benzamid,
5-Methoxy-benzofuran-2-carbonsäure-{1-[6-oxo-4-(1-oxy-pyridin-2-ylmethansulfonyl)-hexahydro-pyrrolo[3,2-b]pyrrol-1-carbonyl]-cyclohexyl}-amid,
Thieno[3,2-b]thiophen-2-carbonsäure-{1-[6-oxo-4-(1-oxy-pyridin-2-ylmethansulfonyl)-hexahydro-pyrrolo[3,2-b]pyrrol-1-carbonyl]-cyclohexyl}-amid,
3-Methyl-benzofuran-2-carbonsäure-{1-[6-oxo-4-(1-oxy-pyridin-2-ylmethansulfonyl)-hexahydro-pyrrolo[3,2-b]pyrrol-1-carbonyl]-cyclohexyl}-amid,
N-{1-[6-Oxo-4-(1-oxy-pyridin-2-ylmethansulfonyl)-hexahydro-pyrrolo[3,2-b]pyrrol-1-carbonyl]-cyclohexyl}-4-phenoxy-benzamid,
N-{1-[6-Oxo-4-(1-oxy-pyridin-2-ylmethansulfonyl)-hexahydro-pyrrolo[3,2-b]pyrrol-1-carbonyl]-cyclohexyl}-4-thiophen-2-yl-benzamid,
4-tert-Butyl-N-{1-cyclopropylmethyl-2-oxo-2-[6-oxo-4-(1-oxy-pyridin-2-ylmethansulfonyl)-hexahydro-pyrrolo[3,2-b]pyrrol-1-yl]-ethyl}-benzamid,
5-Methoxy-benzofuran-2-carbonsäure-{1-cyclopropylmethyl-2-oxo-2-[6-oxo-4-(1-oxy-pyridin-2-ylmethansulfonyl)-hexahydro-pyrrolo[3,2-b]pyrrol-1-yl]-ethyl}-amid,
Thieno[3,2-b]thiophen-2-carbonsäure-{1-cyclopropylmethyl-2-oxo-2-[6-oxo-4-(1-oxy-pyridin-2-ylmethansulfonyl)-hexahydro-pyrrolo[3,2-b]pyrrol-1-yl]-ethyl}-amid,
3-Methyl-benzofuran-2-carbonsäure-{1-cyclopropylmethyl-2-oxo-2-[6-oxo-4-(1-oxy-pyridin-2-ylmethansulfonyl)-hexahydro-pyrrolo[3,2-b]pyrrol-1-yl]-ethyl}-amid,
N-{1-Cyclopropylmethyl-2-oxo-2-[6-oxo-4-(1-oxy-pyridin-2-ylmethansulfonyl)-hexahydro-pyrrolo[3,2-b]pyrrol-1-yl]-ethyl}-4-phenoxy-benzamid,
N-{1-Cyclopropylmethyl-2-oxo-2-[6-oxo-4-(1-oxy-pyridin-2-ylmethansulfonyl)-hexahydro-pyrrolo[3,2-b]pyrrol-1-yl]-ethyl}-4-thiophen-2-yl-benzamid,
4-tert-Butyl-N-{2-oxo-2-[6-oxo-4-(1-oxy-pyridin-2-ylmethansulfonyl)-hexahydro-pyrrolo[3,2-b]pyrrol-1-yl]-1-thiophen-2-ylmethyl-ethyl}-benzamid,
5-Methoxy-benzofuran-2-carbonsäure-{2-oxo-2-[6-oxo-4-(1-oxy-pyridin-2-ylmethansulfonyl)-hexahydro-pyrrolo[3,2-b]pyrrol-1-yl]-1-thiophen-2-ylmethyl-ethyl}-amid,
Thieno[3,2-b]thiophen-2-carbonsäure-{2-oxo-2-[6-oxo-4-(1-oxy-pyridin-2-ylmethansulfonyl)-hexahydro-pyrrolo[3,2-b]pyrrol-1-yl]-1-thiophen-2-ylmethyl-ethyl}-amid,
3-Methyl-benzofuran-2-carbonsäure-{2-oxo-2-[6-oxo-4-(1-oxy-pyridin-2-ylmethansulfonyl)-hexahydro-pyrrolo[3,2-b]pyrrol-1-yl]-1-thiophen-2-ylmethyl-ethyl}-amid,
N-{2-Oxo-2-[6-oxo-4-(1-oxy-pyridin-2-ylmethansulfonyl)-hexahydro-pyrrolo[3,2-b]pyrrol-1-yl]-1-thiophen-2-ylmethyl-ethyl}-4-phenoxy-benzamid,
N-{2-Oxo-2-[6-oxo-4-(1-oxy-pyridin-2-ylmethansulfonyl)-hexahydro-pyrrolo[3,2-b]pyrrol-1-yl]-1-thiophen-2-ylmethyl-ethyl}-4-thiophen-2-yl-benzamid,
4-tert-Butyl-N-{1-[6-oxo-4-(1-oxy-pyridin-3-ylmethansulfbnyl)-hexahydro-pyrrolo[3,2-b]pyrrol-1-carbonyl]-cyclohexyl}-benzamid,
5-Methoxy-benzofuran-2-carbonsäure-{1-[6-oxo-4-(1-oxy-pyridin-3-ylmethansulfonyl)-hexahydro-pyrrolo[3,2-b]pyrrol-1-carbonyl]-cyclohexyl}-amid,
Thieno[3,2-b]thiophen-2-carbonsäure-{1-[6-oxo-4-(1-oxy-pyridin-3-ylmethansulfonyl)-hexahydro-pyrrolo[3,2-b]pyrrol-1-carbonyl]-cyclohexyl}-amid,
3-Methyl-benzofuran-2-carbonsäure-{1-[6-oxo-4-(1-oxy-pyddin-3-ylmethansulfonyl)-hexahydro-pyrrolo[3,2-b]pyrrol-1-carbonyl]-cyclohexyl}-amid,
N-{1-[6-Oxo-4-(1-oxy-pyridin-3-ylmethansulfonyl)-hexahydro-pyrrolo[3,2-b]pyrrol-1-carbonyl]-cyclohexyl}-4-phenoxy-benzamid,
N-{1-[6-Oxo-4-(1-oxy-pyridin-3-ylmethansulfonyl)-hexahydro-pyrrolo[3,2-b]pyrrol-1-carbonyl]-cyclohexyl}-4-thiophen-2-yl-benzamid,
4-tert-Butyl-N-{1-cyclopropylmethyl-2-oxo-2-[6-oxo-4-(1-oxy-pyridin-3-ylmethansulfonyl)-hexahydro-pyrrolo[3,2-b]pyrrol-1-yl]-ethyl}-benzamid,
5-Methoxy-benzofuran-2-carbonsäure-{1-cyclopropylmethyl-2-oxo-2-[6-oxo-4-(1-oxy-pyridin-3-ylmethansulfonyl)-hexahydro-pyrrolo[3,2-b]pyrrol-1-yl]-ethyl}-amid,
Thieno[3,2-b]thiophen-2-carbonsäure-{1-cyclopropylmethyl-2-oxo-2-[6-oxo-4-(1-oxy-pyridin-3-ylmethansulfonyl)-hexahydro-pyrrolo[3,2-b]pyrrol-1-yl]-ethyl}-amid,
3-Methyl-benzofuran-2-carbonsäure-{1-cyclopropylmethyl-2-oxo-2-[6-oxo-4-(1-oxy-pyridin-3-ylmethansulfonyl)-hexahydro-pyrrolo[3,2-b]pyrrol-1-yl]-ethyl}-amid,
N-{1-Cyclopropylmethyl-2-oxo-2-[6-oxo-4-(1-oxy-pyridin-3-ylmethansulfonyl)-hexahydro-pyrrolo[3,2-b]pyrrol-1-yl]-ethyl}-4-phenoxy-benzamid,
N-{1-Cyclopropylmethyl-2-oxo-2-[6-oxo-4-(1-oxy-pyridin-3-ylmethansulfonyl)-hexahydro-pyrrolo[3,2-b]pyrrol-1-yl]-ethyl}-4-thiophen-2-yl-benzamid,
4-tert-Butyl-N-{2-oxo-2-[6-oxo-4-(1-oxy-pyridin-3-ylmethansulfonyl)-hexahydro-pyrrolo[3,2-b]pyrrol-1-yl]-1-thiophen-2-ylmethyl-ethyl}-benzamid,
5-Methoxy-benzofuran-2-carbonsäure-{2-oxo-2-[6-oxo-4-(1-oxy-pyridin-3-ylmethansulfonyl)-hexahydro-pyrrolo[3,2-b]pyrrol-1-yl]-1-thiophen-2-ylmethyl-ethyl}-amid,
Thieno[3,2-b]thiophen-2-carbonsäure-{2-oxo-2-[6-oxo-4-(1-oxy-pyridin-3-ylmethansulfonyl)-hexahydro-pyrrolo[3,2-b]pyrrol-1-yl]-1-thiophen-2-ylmethyl-ethyl}-amid,
3-Methyl-benzofuran-2-carbonsäure-{2-oxo-2-[6-oxo-4-(1-oxy-pyridin-3-ylmethansulfonyl)-hexahydro-pyrrolo[3,2-b]pyrrol-1-yl]-1-thiophen-2-ylmethyl-ethyl}-amid,
N-{2-Oxo-2-[6-oxo-4-(1-oxy-pyridin-3-ylmethansulfonyl)-hexahydro-pyrrolo[3,2-b]pyrrol-1-yl]-1-thiophen-2-ylmethyl-ethyl}-4-phenoxy-benzamid,
N-{2-Oxo-2-[6-oxo-4-(1-oxy-pyridin-3-ylmethansulfonyl)-hexahydro-pyrrolo[3,2-b]pyrrol-1-yl]-1-thiophen-2-ylmethyl-ethyl}-4-thiophen-2-yl-benzamid,
2-Isobutyl-4-morpholin-4-yl-1-[6-oxo-4-(pyridin-2-sulfonyl)-hexahydro-pyrrolo[3,2-b]pyrrol-1-yl]-butan-1,4-dion,
2-Isobutyl-1-[6-oxo-4-(pyridin-2-sulfonyl)-hexahydro-pyrrolo[3,2-b]pyrrol-1-yl]-4-piperazin-1-yl-butan-1,4-dion,
2-Isobutyl-4-(4-methyl-piperazin-1-yl)-1-[6-oxo-4-(pyridin-2-sulfonyl)-hexahydro-pyrrolo[3,2-b]pyrrol-1-yl]-butan-1,4-dion,
2-Isobutyl-1-[6-oxo-4-(pyridin-2-sulfonyl)-hexahydro-pyrrolo[3,2-b]pyrrol-1-yl]-4-(4-phenyl-piperazin-1-yl)-butan-1,4-dion,
2-Isobutyl-1-[6-oxo-4-(pyridin-2-sulfonyl)-hexahydro-pyrrolo[3,2-b]pyrrol-1-yl]-4-(3,4,4a,8a-tetrahydro-1H-isochinolin-2-yl)-butan-1,4-dion,
2-Isobutyl-1-[6-oxo-4-(pyridin-2-sulfonyl)-hexahydro-pyrrolo[3,2-b]pyrrol-1-yl]-4-(1,3,3a,7a-tetrahydro-isoindol-2-yl)-butan-1,4-dion,
4-(4-Benzyl-piperazin-1-yl)-2-isobutyl-1-[6-oxo-4-(pyridin-2-sulfonyl)-hexahydro-pyrrolo[3,2-b]pyrrol-1-yl]-butan-1,4-dion,
2-Isobutyl-4-morpholin-4-yl-1-[6-oxo-4-(pyridin-3-sulfonyl)-hexahydro-pyrrolo[3,2-b]pyrrol-1-yl]-butan-1,4-dion,
2-Isobutyl-1-[6-oxo-4-(pyridin-3-sulfonyl)-hexahydro-pyrrolo[3,2-b]pyrrol-1-yl]-4-piperazin-1-yl-butan-1,4-dion,
2-Isobutyl-4-(4-methyl-piperazin-1-yl)-1-[6-oxo-4-(pyridin-3-sulfonyl)-hexahydro-pyrrolo[3,2-b]pyrrol-1-yl]-butan-1,4-dion,
2-Isobutyl-1-[6-oxo-4-(pyridin-3-sulfonyl)-hexahydro-pyrrolo[3,2-b]pyrrol-1-yl]-4-(4-phenyl-piperazin-1-yl)-butan-1,4-dion,
2-Isobutyl-1-[6-oxo-4-(pyridin-3-sulfonyl)-hexahydro-pyrrolo[3,2-b]pyrrol-1-yl]-4-(3,4,4a,8a-tetrahydro-1H-isochinolin-2-yl)-butan-1,4-dion,
2-Isobutyl-1-[6-oxo-4-(pyridin-3-sulfonyl)-hexahydro-pyrrolo[3,2-b]pyrrol-1-yl]-4-(1,3,3a,7a-tetrahydro-isoindol-2-yl)-butan-1,4-dion,
4-(4-Benzyl-piperazin-1-yl)-2-isobutyl-1-[6-oxo-4-(pyridin-3-sulfonyl)-hexahydro-pyrrolo[3,2-b]pyrrol-1-yl]-butan-1,4-dion,
2-Isobutyl-4-morpholin-4-yl-1-[6-oxo-4-(1-oxy-pyridin-2-sulfonyl)-hexahydro-pyrrolo[3,2-b]pyrrol-1-yl]-butan-1,4-dion,
2-Isobutyl-1-[6-oxo-4-(1-oxy-pyridin-2-sulfonyl)-hexahydro-pyrrolo[3,2-b]pyrrol-1-yl]-4-piperazin-1-yl-butan-1,4-dion,
2-Isobutyl-4-(4-methyl-piperazin-1-yl)-1-[6-oxo-4-(1-oxy-pyridin-2-sulfonyl)-hexahydro-pyrrolo[3,2-b]pyrrol-1-yl]-butan-1,4-dion,
2-Isobutyl-1-[6-oxo-4-(1-oxy-pyridin-2-sulfonyl)-hexahydro-pyrrolo[3,2-b]pyrrol-1-yl]-4-(4-phenyl-piperazin-1-yl)-butan-1,4-dion,
2-Isobutyl-1-[6-oxo-4-(1-oxy-pyridin-2-sulfonyl)-hexahydro-pyrrolo[3,2-b]pyrrol-1-yl]-4-(3,4,4a,8a-tetrahydro-1H-isochinolin-2-yl)-butan-1,4-dion,
2-Isobutyl-1-[6-oxo-4-(1-oxy-pyridin-2-sulfonyl)-hexahydro-pyrrolo[3,2-b]pyrrol-1-yl]-4-(1,3,3a,7a-tetrahydro-isoindol-2-yl)-butan-1,4-dion,
4-(4-Benzyl-piperazin-1-yl)-2-isobutyl-1-[6-oxo-4-(1-oxy-pyridin-2-sulfonyl)-hexahydro-pyrrolo[3,2-b]pyrrol-1-yl]-butan-1,4-dion,
2-Isobutyl-4-morpholin-4-yl-1-[6-oxo-4-(1-oxy-pyridin-3-sulfonyl)-hexahydro-pyrrolo[3,2-b]pyrrol-1-yl]-butan-1,4-dion,
2-Isobutyl-1-[6-oxo-4-(1-oxy-pyridin-3-sulfonyl)-hexahydro-pyrrolo[3,2-b]pyrrol-1-yl]-4-piperazin-1-yl-butan-1,4-dion,
2-Isobutyl-4-(4-methyl-piperazin-1-yl)-1-[6-oxo-4-(1-oxy-pyridin-3-sulfonyl)-hexahydro-pyrrolo[3,2-b]pyrrol-1-yl]-butan-1,4-dion,
2-Isobutyl-1-[6-oxo-4-(1-oxy-pyridin-3-sulfonyl)-hexahydro-pyrrolo[3,2-b]pyrrol-1-yl]-4-(4-phenyl-piperazin-1-yl)-butan-1,4-dion,
2-Isobutyl-1-[6-oxo-4-(1-oxy-pyridin-3-sulfonyl)-hexahydro-pyrrolo[3,2-b]pyrrol-1-yl]-4-(3,4,4a,8a-tetrahydro-1H-isochinolin-2-yl)-butan-1,4-dion,
2-Isobutyl-1-[6-oxo-4-(1-oxy-pyridin-3-sulfonyl)-hexahydro-pyrrolo[3,2-b]pyrrol-1-yl]-4-(1,3,3a,7a-tetrahydro-isoindol-2-yl)-butan-1,4-dion,
4-(4-Benzyl-piperazin-1-yl)-2-isobutyl-1-[6-oxo-4-(1-oxy-pyridin-3-sulfonyl)-hexahydro-pyrrolo[3,2-b]pyrrol-1-yl]-butan-1,4-dion,
2-Isobutyl-4-morpholin-4-yl-1-[6-oxo-4-(pyridin-2-carbonyl)-hexahydro-pyrrolo[3,2-b]pyrrol-1-yl]-butan-1,4-dion,
2-Isobutyl-1-[6-oxo-4-(pyridin-2-carbonyl)-hexahydro-pyrrolo[3,2-b]pyrrol-1-yl]-4-piperazin-1-yl-butan-1,4-dion,
2-Isobutyl-4-(4-methyl-piperazin-1-yl)-1-[6-oxo-4-(pyridin-2-carbonyl)-hexahydro-pyrrolo[3,2-b]pyrrol-1-yl]-butan-1,4-dion,
2-Isobutyl-1-[6-oxo-4-(pyridin-2-carbonyl)-hexahydro-pyrrolo[3,2-b]pyrrol-1-yl]-4-(4-phenyl-piperazin-1-yl)-butan-1,4-dion,
2-Isobutyl-1-[6-oxo-4-(pyridin-2-carbonyl)-hexahydro-pyrrolo[3,2-b]pyrrol-1-yl]-4-(3,4,4a,8a-tetrahydro-1H-isochinolin-2-yl)-butan-1,4-dion,
2-lsobutyl-1-[6-oxo-4-(pyridin-2-carbonyl)-hexahydro-pyrrolo[3,2-b]pyrrol-1-yl]-4-(1,3,3a,7a-tetrahydro-isoindol-2-yl)-butan-1,4-dion,
4-(4-Benzyl-piperazin-1-yl)-2-isobutyl-1-[6-oxo-4-(pyridin-2-carbonyl)-hexahydro-pyrrolo[3,2-b]pyrrol-1-yl]-butan-1,4-dion,
2-Isobutyl-4-morpholin-4-yl-1-[6-oxo-4-(2-pyridin-3-yl-acetyl)-hexahydro-pyrrolo[3,2-b]pyrrol-1-yl]-butan-1,4-dion,
2-Isobutyl-1-[6-oxo-4-(2-pyridin-3-yl-acetyl)-hexahydro-pyrrolo[3,2-b]pyrrol-1-yl]-4-piperazin-1-yl-butan-1,4-dion,
2-Isobutyl-4-(4-methyl-piperazin-1-yl)-1-[6-oxo-4-(2-pyridin-3-yl-acetyl)-hexahydro-pyrrolo[3,2-b]pyrrol-1-yl]-butan-1,4-dion,
2-Isobutyl-1-[6-oxo-4-(2-pyridin-3-yl-acetyl)-hexahydro-pyrrolo[3,2-b]pyrrol-1-yl]-4-(4-phenyl-piperazin-1-yl)-butan-1,4-dion,
2-Isobutyl-1-[6-oxo-4-(2-pyridin-3-yl-acetyl)-hexahydro-pyrrolo[3,2-b]pyrrol-1-yl]-4-(3,4,4a,8a-tetrahydro-1H-isochinolin-2-yl)-butan-1,4-dion,
2-Isobutyl-1-[6-oxo-4-(2-pyridin-3-yl-acetyl)-hexahydro-pyrrolo[3,2-b]pyrrol-1-yl]-4-(1,3,3a,7a-tetrahydro-isoindol-2-yl)-butan-1,4-dion,
4-(4-Benzyl-piperazin-1-yl)-2-isobutyl-1-[6-oxo-4-(2-pyridin-3-yl-acetyl)-hexahydro-pyrrolo[3,2-b]pyrrol-1-yl]-butan-1,4-dion,
2-Isobutyl-4-morpholin-4-yl-1-[6-oxo-4-(pyridin-2-ylmethansulfonyl)-hexahydro-pyrrolo[3,2-b]pyrrol-1-yl]-butan-1,4-dion,
2-Isobutyl-1-[6-oxo-4-(pyridin-2-ylmethansulfonyl)-hexahydro-pyrrolo[3,2-b]pyrrol-1-yl]-4-piperazin-1-yl-butan-1,4-dion,
2-Isobutyl-4-(4-methyl-piperazin-1-yl)-1-[6-oxo-4-(pyridin-2-ylmethansulfonyl)-hexahydro-pyrrolo[3,2-b]pyrrol-1-yl]-butan-1,4-dion,
2-Isobutyl-1-[6-oxo-4-(pyridin-2-ylmethansulfonyl)-hexahydro-pyrrolo[3,2-b]pyrrol-1-yl]-4-(4-phenyl-piperazin-1-yl)-butan-1,4-dion,
2-Isobutyl-1-[6-oxo-4-(pyridin-2-ylmethansulfonyl)-hexahydro-pyrrolo[3,2-b]pyrrol-1-yl]-4-(3,4,4a,8a-tetrahydro-1 H-isochinolin-2-yl)-butan-1,4-dion,
2-Isobutyl-1-[6-oxo-4-(pyridin-2-ylmethansulfonyl)-hexahydro-pyrrolo[3,2-b]pyrrol-1-yl]-4-(1,3,3a,7a-tetrahydro-isoindol-2-yl)-butan-1,4-dion,
4-(4-Benzyl-piperazin-1-yl)-2-isobutyl-1-[6-oxo-4-(pyridin-2-ylmethansulfonyl)-hexahydro-pyrrolo[3,2-b]pyrrol-1-yl]-butan-1,4-dion,
2-Isobutyl-4-morpholin-4-yl-1-[6-oxo-4-(pyridin-3-ylmethansulfonyl)-hexahydro-pyrrolo[3,2-b]pyrrol-1-yl]-butan-1,4-dion,
2-Isobutyl-1-[6-oxo-4-(pyridin-3-ylmethansulfonyl)-hexahydro-pyrrolo[3,2-b]pyrrol-1-yl]-4-piperazin-1-yl-butan-1,4-dion,
2-Isobutyl-4-(4-methyl-piperazin-1-yl)-1-[6-oxo-4-(pyridin-3-ylmethansulfonyl)-hexahydro-pyrrolo[3,2-b]pyrrol-1-yl]-butan-1,4-dion,
2-Isobutyl-1-[6-oxo-4-(pyridin-3-ylmethansulfonyl)-hexahydro-pyrrolo[3,2-b]pyrrol-1-yl]-4-(4-phenyl-piperazin-1-yl)-butan-1,4-dion,
2-Isobutyl-1-[6-oxo-4-(pyridin-3-ylmethansulfonyl)-hexahydro-pyrrolo[3,2-b]pyrrol-1-yl]-4-(3,4,4a,8a-tetrahydro-1H-isochinolin-2-yl)-butan-1,4-dion,
2-Isobutyl-1-[6-oxo-4-(pyridin-3-ylmethansulfonyl)-hexahydro-pyrrolo[3,2-b]pyrrol-1-yl]-4-(1,3,3a,7a-tetrahydro-isoindol-2-yl)-butan-1,4-dion,
4-(4-Benzyl-piperazin-1-yl)-2-isobutyl-1-[6-oxo-4-(pyridin-3-ylmethansulfonyl)-hexahydro-pyrrolo[3,2-b]pyrrol-1-yl]-butan-1,4-dion,
2-Isobutyl-4-morpholin-4-yl-1-[6-oxo-4-(1-oxy-pyridin-2-ylmethansulfonyl)-hexahydro-pyrrolo[3,2-b]pyrrol-1-yl]-butan-1,4-dion,
2-Isobutyl-1-[6-oxo-4-(1-oxy-pyridin-2-ylmethansulfonyl)-hexahydro-pyrrolo[3,2-b]pyrrol-1-yl]-4-piperazin-1-yl-butan-1,4-dion,
2-Isobutyl-4-(4-methyl-piperazin-1-yl)-1-[6-oxo-4-(1-oxy-pyridin-2-ylmethansulfonyl)-hexahydro-pyrrolo[3,2-b]pyrrol-1-yl]-butan-1,4-dion,
2-Isobutyl-1-[6-oxo-4-(1-oxy-pyridin-2-ylmethansulfonyl)-hexahydro-pyrrolo[3,2-b]pyrrol-1-yl]-4-(4-phenyl-piperazin-1-yl)-butan-1,4-dion,
2-Isobutyl-1-[6-oxo-4-(1-oxy-pyridin-2-ylmethansulfonyl)-hexahydro-pyrrolo[3,2-b]pyrrol-1-yl]-4-(3,4,4a,8a-tetrahydro-1H-isochinolin-2-yl)-butan-1,4-dion,
2-Isobutyl-1-[6-oxo-4-(1-oxy-pyridin-2-ylmethansulfonyl)-hexahydro-pyrrolo[3,2-b]pyrrol-1-yl]-4-(1,3,3a,7a-tetrahydro-isoindol-2-yl)-butan-1,4-dion,
4-(4-Benzyl-piperazin-1-yl)-2-isobutyl-1-[6-oxo-4-(1-oxy-pyridin-2-ylmethansulfonyl)-hexahydro-pyrrolo[3,2-b]pyrrol-1-yl]-butan-1,4-dion,
2-Isobutyl-4-morpholin-4-yl-1-[6-oxo-4-(1-oxy-pyridin-3-ylmethansulfonyl)-hexahydro-pyrrolo[3,2-b]pyrrol-1-yl]-butan-1,4-dion,
2-Isobutyl-1-[6-oxo-4-(1-oxy-pyridin-3-ylmethansulfonyl)-hexahydro-pyrrolo[3,2-b]pyrrol-1-yl]-4-piperazin-1-yl-butan-1,4-dion,
2-Isobutyl-4-(4-methyl-piperazin-1-yl)-1-[6-oxo-4-(1-oxy-pyridin-3-ylmethansulfonyl)-hexahydro-pyrrolo[3,2-b]pyrrol-1-yl]-butan-1,4-dion,
2-Isobutyl-1-[6-oxo-4-(1-oxy-pyridin-3-ylmethanslfonyl)-hexahydro-pyrrolo[3,2-b]pyrrol-1-yl]-4-(4-phenyl-piperazin-1-yl)-butan-1,4-dion,
2-Isobutyl-1-[6-oxo-4-(1-oxy-pyridin-3-ylmethansulfonyl)-hexahydro-pyrrolo[3,2-b]pyrrol-1-yl]-4-(3,4,4a,8a-tetrahydro-1H-isochinolin-2-yl)-butan-1,4-dion,
2-Isobutyl-1-[6-oxo-4-(1-oxy-pyridin-3-ylmethansulfonyl)-hexahydro-pyrrolo[3,2-b]pyrrol-1-yl]-4-(1,3,3a,7a-tetrahydro-isoindol-2-yl)-butan-1,4-dion,
4-(4-Benzyl-piperazin-1-yl)-2-isobutyl-1-[6-oxo-4-(1-oxy-pyridin-3-ylmethansulfonyl)-hexahydro-pyrrolo[3,2-b]pyrrol-1-yl]-butan-1,4-dion,
4-(2-Biphenyl-3-yl-4-methyl-pentanoyl)-1-(pyridin-2-sulfonyl)-hexahydro-pyrrolo[3,2-b]pyrrol-3-on,
4-(2-Biphenyl-3-yl-4-methyl-pentanoyl)-1-(pyridin-3-sulfonyl)-hexahydro-pyrrolo[3,2-b]pyrrol-3-on,
4-(2-Biphenyl-3-yl-4-methyl-pentanoyl)-1-(1-oxy-pyridin-2-sulfonyl)-hexahydro-pyrrolo[3,2-b]pyrrol-3-on,
4-(2-Biphenyl-3-yl-4-methyl-pentanoyl)-1-(1-oxy-pyridin-3-sulfonyl)-hexahydro-pyrrolo[3,2-b]pyrrol-3-on,
4-(2-Biphenyl-3-yl-4-methyl-pentanoyl)-1-(pyridin-2-carbonyl)-hexahydro-pyrrolo[3,2-b]pyrrol-3-on,
4-(2-Biphenyl-3-yl-4-methyl-pentanoyl)-1-(2-pyridin-3-yl-acetyl)-hexahydro-pyrrolo[3,2-b]pyrrol-3-on,
4-(2-Biphenyl-3-yl-4-methyl-pentanoyl)-1-(pyridin-2-ylmethansulfonyl)-hexahydro-pyrrolo[3,2-b]pyrrol-3-on,
4-(2-Biphenyl-3-yl-4-methyl-pentanoyl)-1-(pyridin-3-ylmethansulfonyl)-hexahydro-pyrrolo[3,2-b]pyrrol-3-on,
4-(2-Biphenyl-3-yl-4-methyl-pentanoyl)-1-(1-oxy-pyridin-2-ylmethansulfonyl)-hexahydro-pyrrolo[3,2-b]pyrrol-3-on,
4-(2-Biphenyl-3-yl-4-methyl-pentanoyl)-1-(1-oxy-pyridin-3-ylmethansulfonyl)-hexahydro-pyrrolo[3,2-b]pyrrol-3-on,
4-[4-Methyl-2-(3-pyridin-2-yl-phenyl)-pentanoyl]-1-(pyridin-2-sulfonyl)-hexahydro-pyrrolo[3,2-b]pyrrol-3-on,
4-[4-Methyl-2-(3-pyridin-2-yl-phenyl)-pentanoyl]-1-(pyridin-3-sulfonyl)-hexahydro-pyrrolo[3,2-b]pyrrol-3-on,
4-[4-Methyl-2-(3-pyridin-2-yl-phenyl)-pentanoyl]-1-(1-oxy-pyridin-2-sulfonyl)-hexahydro-pyrrolo[3,2-b]pyrrol-3-on,
4-[4-Methyl-2-(3-pyridin-2-yl-phenyl)-pentanoyl]-1-(1-oxy-pyridin-3-sulfonyl)-hexahydro-pyrrolo[3,2-b]pyrrol-3-on,
4-[4-Methyl-2-(3-pyridin-2-yl-phenyl)-pentanoyl]-1-(pyridin-2-carbonyl)-hexahydro-pyrrolo[3,2-b]pyrrol-3-on,
4-[4-Methyl-2-(3-pyridin-2-yl-phenyl)-pentanoyl]-1-(2-pyridin-3-yl-acetyl)-hexahydro-pyrrolo[3,2-b]pyrrol-3-on,
4-[4-Methyl-2-(3-pyridin-2-yl-phenyl)-pentanoyl]-1-(pyridin-2-ylmethansulfonyl)-hexahydro-pyrrolo[3,2-b]pyrrol-3-on,
4-[4-Methyl-2-(3-pyridin-2-yl-phenyl)-pentanoyl]-1-(pyridin-3-ylmethansulfonyl)-hexahydro-pyrrolo[3,2-b]pyrrol-3-on,
4-[4-Methyl-2-(3-pyridin-2-yl-phenyl)-pentanoyl]-1-(1-oxy-pyridin-2-ylmethansulfonyl)-hexahydro-pyrrolo[3,2-b]pyrrol-3-on,
4-[4-Methyl-2-(3-pyridin-2-yl-phenyl)-pentanoyl]-1-(1-oxy-pyridin-3-ylmethansulfonyl)-hexahydro-pyrrolo[3,2-b]pyrrol-3-on,
4-[4-Methyl-2-(3-pyridin-3-yl-phenyl)-pentanoyl]-1-(pyridin-2-sulfonyl)-hexahydro-pyrrolo[3,2-b]pyrrol-3-on,
4-[4-Methyl-2-(3-pyridin-3-yl-phenyl)-pentanoyl]-1-(pyridin-3-sulfonyl)-hexahydro-pyrrolo[3,2-b]pyrrol-3-on,
4-[4-Methyl-2-(3-pyridin-3-yl-phenyl)-pentanoyl]-1-(1-oxy-pyridin-2-sulfonyl)-hexahydro-pyrrolo[3,2-b]pyrrol-3-on,
4-[4-Methyl-2-(3-pyridin-3-yl-phenyl)-pentanoyl]-1-(1-oxy-pyridin-3-sulfonyl)-hexahydro-pyrrolo[3,2-b]pyrrol-3-on,
4-[4-Methyl-2-(3-pyridin-3-yl-phenyl)-pentanoyl]-1-(pyridin-2-carbonyl)-hexahydro-pyrrolo[3,2-b]pyrrol-3-on,

4-[4-Methyl-2-(3-pyridin-3-yl-phenyl)-pentanoyl]-1-(2-pyridin-3-yl-acetyl)-hexahydro-pyrrolo[3,2-b]pyrrol-3-on,
4-[4-Methyl-2-(3-pyridin-3-yl-phenyl)-pentanoyl]-1-(pyridin-2-ylmethansulfonyl)-hexahydro-pyrrolo[3,2-b]pyrrol-3-on,
4-[4-Methyl-2-(3-pyridm-3-yl-phenyl)-pentanoyl]-1-(pyridin-3-ylmethansulfonyl)-hexahydro-pyrrolo[3,2-b]pyrrol-3-on,
4-[4-Methyl-2-(3-pyridin-3-yl-phenyl)-pentanoyl]-1-(1-oxy-pyridin-2-ylmethansulfonyl)-hexahydro-pyrrolo[3,2-b]pyrrol-3-on,
4-[4-Methyl-2-(3-pyridin-3-yl-phenyl)-pentanoyl]-1-(1-oxy-pyridin-3-ylmethansulfonyl)-hexahydro-pyrrolo[3,2-b]pyrrol-3-on,
4-[4-Methyl-2-(3-pyridin-4-yl-phenyl)-pentanoyl]-1-(pyridin-2-sulfonyl)-hexahydro-pyrrolo[3,2-b]pyrrol-3-on,
4-[4-Methyl-2-(3-pyridin-4-yl-phenyl)-pentanoyl]-1-(pyridin-3-sulfonyl)-hexahydro-pyrrolo[3,2-b]pyrrol-3-on,
4-[4-Methyl-2-(3-pyridin-4-yl-phenyl)-pentanoyl]-1-(1-oxy-pyridin-2-sulfonyl)-hexahydro-pyrrolo[3,2-b]pyrrol-3-on,
4-[4-Methyl-2-(3-pyridin-4-yl-phenyl)-pentanoyl]-1-(1-oxy-pyridin-3-sulfonyl)-hexahydro-pyrrolo[3,2-b]pyrrol-3-on,
4-[4-Methyl-2-(3-pyridin-4-yl-phenyl)-pentanoyl]-1-(pyridin-2-carbonyl)-hexahydro-pyrrolo[3,2-b]pyrrol-3-on,
4-[4-Methyl-2-(3-pyridin-4-yl-phenyl)-pentanoyl]-1-(2-pyridin-3-yl-acetyl)-hexahydro-pyrrolo[3,2-b]pyrrol-3-on,
4-[4-Methyl-2-(3-pyridin-4-yl-phenyl)-pentanoyl]-1-(pyridin-2-ylmethansulfonyl)-hexahydro-pyrrolo[3,2-b]pyrrol-3-on,
4-[4-Methyl-2-(3-pyridin-4-yl-phenyl)-pentanoyl]-1-(pyridin-3-ylmethansulfonyl)-hexahydro-pyrrolo[3,2-b]pyrrol-3-on,
4-[4-Methyl-2-(3-pyridin-4-yl-phenyl)-pentanoyl]-1-(1-oxy-pyridin-2-ylmethansulfonyl)-hexahydro-pyrrolo[3,2-b]pyrrol-3-on,
4-[4-Methyl-2-(3-pyridin-4-yl-phenyl)-pentanoyl]-1-(1-oxy-pyridin-3-ylmethansulfonyl)-hexahydro-pyrrolo[3,2-b]pyrrol-3-on,
Furan-3-carbonsäure-{3,3-dimethyl-1-[6-oxo-4-(pyridin-2-sulfonyl)-hexahydro-pyrrolo[3,2-b]pyrrol-1-carbonyl]-butyl}-amid,
Thiophen-3-carbonsäure-{3,3-dimethyl-1-[6-oxo-4-(pyridin-2-sulfonyl)-hexahydro-pyrrolo[3,2-b]pyrrol-1-carbonyl]-butyl}-amid,
Benzo[b]thiophen-3-carbonsäure-{3,3-dimethyl-1-[6-oxo-4-(pyridin-2-sulfonyl)-hexahydro-pyrrolo[3,2-b]pyrrol-1-carbonyl]-butyl}-amid,
Furan-3-carbonsäure-{1-cyclohexylmethyl-2-oxo-2-[6-oxo-4-(pyridin-2-sulfonyl)-hexahydro-pyrrolo[3,2-b]pyrrol-1-yl]-ethyl}-amid,
Thiophen-3-carbonsäure-{1-cyclohexylmethyl-2-oxo-2-[6-oxo-4-(pyridin-2-sulfonyl)-hexahydro-pyrrolo[3,2-b]pyrrol-1-yl]-ethyl}-amid,
Benzo[b]thiophen-3-carbonsäure-{1-cyclohexylmethyl-2-oxo-2-[6-oxo-4-(pyridin-2-sulfonyl)-hexahydro-pyrrolo[3,2-b]pyrrol-1-yl]-ethyl}-amid,
Naphthalen-1-carbonsäure-{1-(4-hydroxy-benzyl)-2-oxo-2-[6-oxo-4-(pyridin-2-sulfonyl)-hexahydro-pyrrolo[3,2-b]pyrrol-1-yl]-ethyl}-amid,
Chinolin-8-carbonsäure-{1-(4-hydroxy-benzyl)-2-oxo-2-[6-oxo-4-(pyridin-2-sulfonyl)-hexahydro-pyrrolo[3,2-b]pyrrol-1-yl]-ethyl}-amid,
4-tert-Butyl-N-1-(4-hydroxy-benzyl)-2-oxo-2-[6-oxo-4-(pyridin-2-sulfonyl)-hexahydro-pyrrolo[3,2-b]pyrrol-1-yl]-ethyl}-benzamid,
4-tert-Butyl-N-{1-(4-fluor-benzyl)-2-oxo-2-[6-oxo-4-(pyridin-2-sulfonyl)-hexahydro-pyrrolo[3,2-b]pyrrol-1-yl]-ethyl}-benzamid,
4-tert-Butyl-N-{1-(4-methoxy-benzyl)-2-oxo-2-[6-oxo-4-(pyridin-2-sulfonyl)-hexahydro-pyrrolo[3,2-b]pyrrol-1-yl]-ethyl}-benzamid,
4-tert-Butyl-N-1-(3-hydroxy-benzyl)-2-oxo-2-[6-oxo-4-(pyridin-2-sulfonyl)-hexahydro-pyrrolo[3,2-b]pyrrol-1-yl]-ethyl}-benzamid,
Biphenyl-4-carbonsäure-{1-(4-hydroxy-benzyl)-2-oxo-2-[6-oxo-4-(pyridin-2-sulfonyl)-hexahydro-pyrrolo[3,2-b]pyrrol-1-yl]-ethyl}-amid,
Biphenyl-4-carbonsäuregl-(4-fluor-benzyl)-2-oxo-2-[6-oxo-4-(pyridin-2-sulfonyl)-hexahydro-pyrrolo[3,2-b]pyrrol-1-yl]-ethyl}-amid,
Biphenyl-4-carbonsäure-{1-(4-methoxy-benzyl)-2-oxo-2-[6-oxo-4-(pyüdin-2-sulfonyl)-hexahydro-pyrrolo[3,2-b]pyrrol-1-yl]-ethyl}-amid,
Biphenyl-4-carbonsäure-{1-(3-hydroxy-benzyl)-2-oxo-2-[6-oxo-4-(pyridin-2-sulfonyl)-hexahydro-pyrrolo[3,2-b]pyrrol-1-yl]-ethyl}-amid,
Furan-3-carbonsäure-{3,3-dimethyl-1-[6-oxo-4-(pyridin-3-sulfonyl)-hexahydro-pyrrolo[3,2-b]pyrrol-1-carbonyl]-butyl}-amid,
Thiophen-3-carbonsäure-{3,3-dimethyl-1-[6-oxo-4-(pyridin-3-sulfonyl)-hexahydro-pyrrolo[3,2-b]pyrrol-1-carbonyl]-butyl}-amid,
Benzo[b]thiophen-3-carbonsäure-{3,3-dimethyl-1-[6-oxo-4-(pyridin-3-sulfonyl)-hexahydro-pyrrolo[3,2-b]pyrrol-1-carbonyl]-butyl}-amid,
Furan-3-carbonsäure-{1-cyclohexylmethyl-2-oxo-2-[6-oxo-4-(pyridin-3-sulfonyl)-hexahydro-pyrrolo[3,2-b]pyrrol-1-yl]-ethyl}-amid,
Thiophen-3-carbonsäure-{1-cyclohexylmethyl-2-oxo-2-[6-oxo-4-(pyridin-3-sulfonyl)-hexahydro-pyrrolo[3,2-b]pyrrol-1-yl]-ethyl}-amid,
Benzo[b]thiophen-3-carbonsäure-{1-cyclohexylmethyl-2-oxo-2-[6-oxo-4-(pyridin-3-sulfonyl)-hexahydro-pyrrolo[3,2-b]pyrrol-1-yl]-ethyl}-amid,
Naphthalen-1-carbonsäure-{1-(4-hydroxy-benzyl)-2-oxo-2-[6-oxo-4-(pyridin-3-sulfonyl)-hexahydro-pyrrolo[3,2-b]pyrrol-1-yl]-ethyl}-amid,
Chinolin-8-carbonsäure-{1-(4-hydroxy-benzyl)-2-oxo-2-[6-oxo-4-(pyridin-3-sulfonyl)-hexahydro-pyrrolo[3,2-b]pyrrol-1-yl]-ethyl}-amid,
4-tert-Butyl-N-{1-(4-hydroxy-benzyl)-2-oxo-2-[6-oxo-4-(pyridin-3-sulfonyl)-hexahydro-pyrrolo[3,2-b]pyrrol-1-yl]-ethyl}-benzamid,
4-tert-Butyl-N-{1-(4-fluor-benzyl)-2-oxo-2-[6-oxo-4-(pyridin-3-sulfonyl)-hexahydro-pyrrolo[3,2-b]pyrrol-1-yl]-ethyl}-benzamid,
4-tert-Butyl-N-{1-(4-methoxy-benzyl)-2-oxo-2-[6-oxo-4-(pyridin-3-sulfonyl)-hexahydro-pyrrolo[3,2-b]pyrrol-1-yl]-ethyl}-benzamid,
4-tert-Butyl-N-{1-(3-hydroxy-benzyl)-2-oxo-2-[6-oxo-4-(pyridin-3-sulfonyl)-hexahydro-pyrrolo[3,2-b]pyrrol-1-yl]-ethyl}-benzamid,
Biphenyl-4-carbonsäure-{1-(4-hydroxy-benzyl)-2-oxo-2-[6-oxo-4-(pyridin-3-sulfonyl)-hexahydro-pyrrolo[3,2-b]pyrrol-1-yl]-ethyl}-amid,
Biphenyl-4-carbonsäure-{1-(4-fluor-benzyl)-2-oxo-2-[6-oxo-4-(pyridin-3-sulfonyl)-hexahydro-pyrrolo[3,2-b]pyrrol-1-yl]-ethyl}-amid,
Biphenyl-4-carbonsäuregl-(4-methoxy-benzyl)-2-oxo-2-[6-oxo-4-(pyridin-3-sulfonyl)-hexahydro-pyrrolo[3,2-b]pyrrol-1-yl]-ethyl}-amid,
Biphenyl-4-carbonsäure-{1-(3-hydroxy-benzyl)-2-oxo-2-[6-oxo-4-(pyridin-3-sulfonyl)-hexahydro-pyrrolo[3,2-b]pyrrol-1-yl]-ethyl}-amid,
Furan-3-carbonsäure-{3,3-dimethyl-1-[6-oxo-4-(1-oxy-pyridin-2-sulfonyl)-hexahydro-pyrrolo[3,2-b]pyrrol-1-carbonyl]-butyl}-amid,
Thiophen-3-carbonsäure-{3,3-dimethyl-1-[6-oxo-4-(1-oxy-pyridin-2-sulfonyl)-hexahydro-pyrrolo[3,2-b]pyrrol-1-carbonyl]-butyl}-amid,
Benzo[b]thiophen-3-carbonsäure-{3,3-dimethyl-1-[6-oxo-4-(1-oxy-pyridin-2-sulfonyl)-hexahydro-pyrrolo[3,2-b]pyrrol-1-carbonyl]-butyl}-amid,
Furan-3-carbonsäure-{1-cyclohexylmethyl-2-oxo-2-[6-oxo-4-(1-oxy-pyridin-2-sulfonyl)-hexahydro-pyrrolo[3,2-b]pyrrol-1-yl]-ethyl}-amid,
Thiophen-3-carbonsäure-{1-cyclohexylmethyl-2-oxo-2-[6-oxo-4-(1-oxy-pyridin-2-sulfonyl)-hexahydro-pyrrolo[3,2-b]pyrrol-1-yl]-ethyl}-amid,
Benzo[b]thiophen-3-carbonsäure-{1-cyclohexylmethyl-2-oxo-2-[6-oxo-4-(1-oxy-pyridin-2-sulfonyl)-hexahydro-pyrrolo[3,2-b]pyrrol-1-yl]-ethyl}-amid,
Naphthalen-1-carbonsäure-{1-(4-hydroxy-benzyl)-2-oxo-2-[6-oxo-4-(1-oxy-pyridin-2-sulfonyl)-hexahydro-pyrrolo[3,2-b]pyrrol-1-yl]-ethyl}-amid,
Chinolin-8-carbonsäure-{1-(4-hydroxy-benzyl)-2-oxo-2-[6-oxo-4-(1-oxy-pyridin-2-sulfonyl)-hexahydro-pyrrolo[3,2-b]pyrrol-1-yl]-ethyl}-amid,
4-tert-Butyl-N-{1-(4-hydroxy-benzyl)-2-oxo-2-[6-oxo-4-(1-oxy-pyridin-2-sulfonyl)-hexahydro-pyrrolo[3,2-b]pyrrol-1-yl]-ethyl}-benzamid,
4-tert-Butyl-N-{1-(4-fluor-benzyl)-2-oxo-2-[6-oxo-4-(1-oxy-pyridin-2-sulfonyl)-hexahydro-pyrrolo[3,2-b]pyrrol-1-yl]-ethyl}-benzamid,
4-tert-Butyl-N-{1-(4-methoxy-benzyl)-2-oxo-2-[6-oxo-4-(1-oxy-pyridin-2-sulfonyl)-hexahydro-pyrrolo[3,2-b]pyrrol-1-yl]-ethyl}-benzamid,
4-tert-Butyl-N-{1-(3-hydroxy-benzyl)-2-oxo-2-[6-oxo-4-(1-oxy-pyridin-2-sulfonyl)-hexahydro-pyrrolo[3,2-b]pyrrol-1-yl]-ethyl}-benzamid,
Biphenyl-4-carbonsäure-{1-(4-hydroxy-benzyl)-2-oxo-2-[6-oxo-4-(1-oxy-pyridin-2-sulfonyl)-hexahydro-pyrrolo[3,2-b]pyrrol-1-yl]-ethyl}-amid,
Biphenyl-4-carbonsäure-{1-(4-fluor-benzyl)-2-oxo-2-[6-oxo-4-(1-oxy-pyridin-2-sulfonyl)-hexahydro-pyrrolo[3,2-b]pyrrol-1-yl]-ethyl}-amid,
Biphenyl-4-carbonsäure-{1-(4-methoxy-benzyl)-2-oxo-2-[6-oxo-4-(1-oxy-pyridin-2-sulfonyl)-hexahydro-pyrrolo[3,2-b]pyrrol-1-yl]-ethyl}-amid,
Biphenyl-4-carbonsäure-{1-(3-hydroxy-benzyl)-2-oxo-2-[6-oxo-4-(1-oxy-pyridin-2-sulfonyl)-hexahydro-pyrrolo[3,2-b]pyrrol-1-yl]-ethyl}-amid,
Furan-3-carbonsäure-{3,3-dimethyl-1-[6-oxo-4-(1-oxy-pyridin-3-sulfonyl)-hexahydro-pyrrolo[3,2-b]pyrrol-1-carbonyl]-butyl}-amid,
Thiophen-3-carbonsäure-{3,3-dimethyl-1-[6-oxo-4-(1-oxy-pyridin-3-sulfonyl)-hexahydro-pyrrolo[3,2-b]pyrrol-1-carbonyl]-butyl}-amid,
Benzo[b]thiophen-3-carbonsäure-{3,3-dimethyl-1-[6-oxo-4-(1-oxy-pyridin-3-sulfonyl)-hexahydro-pyrrolo[3,2-b]pyrrol-1-carbonyl]-butyl}-amid,
Furan-3-carbonsäure-{1-cyclohexylmethyl-2-oxo-2-[6-oxo-4-(1-oxy-pyridin-3-sulfonyl)-hexahydro-pyrrolo[3,2-b]pyrrol-1-yl]-ethyl}-amid,
Thiophen-3-carbonsäure-{1-cyclohexylmethyl-2-oxo-2-[6-oxo-4-(1-oxy-pyridin-3-sulfonyl)-hexahydro-pyrrolo[3,2-b]pyrrol-1-yl]-ethy}-amid,
Benzo[b]thiophen-3-carbonsäure-{1-cyclohexylmethyl-2-oxo-2-[6-oxo-4-(1-oxy-pyridin-3-sulfonyl)-hexahydro-pyrrolo[3,2-b]pyrrol-1-yl]-ethyl}-amid,
Naphthalen-1-carbonsäure-{1-(4-hydroxy-benzyl)-2-oxo-2-[6-oxo-4-(1-oxy-pyridin-3-sulfonyl)-hexahydro-pyrrolo[3,2-b]pyrrol-1-yl]-ethyl}-amid,
Chinolin-8-carbonsäure-{1-(4-hydroxy-benzyl)-2-oxo-2-[6-oxo-4-(1-oxy-pyridin-3-sulfonyl)-hexahydro-pyrrolo[3,2-b]pyrrol-1-yl]-ethyl}-amid,
4-tert-Butyl-N-{1-(4-hydroxy-benzyl)-2-oxo-2-[6-oxo-4-(1-oxy-pyridin-3-sulfonyl)-hexahydro-pyrrolo[3,2-b]pyrrol-1-yl]-ethyl}-benzamid,
4-tert-Butyl-N-{1-(4-fluor-benzyl)-2-oxo-2-[6-oxo-4-(1-oxy-pyridin-3-sulfonyl)-hexahydro-pyrrolo[3,2-b]pyrrol-1-yl]-ethyl}-benzamid,
4-tert-Butyl-N-{1-(4-methoxy-benzyl)-2-oxo-2-[6-oxo-4-(1-oxy-pyridin-3-sulfonyl)-hexahydro-pyrrolo[3,2-b]pyrrol-1-yl]-ethyl}-benzamid,
4-tert-Butyl-N-{1-(3-hydroxy-benzyl)-2-oxo-2-[6-oxo-4-(1-oxy-pyridin-3-sulfonyl)-hexahydro-pyrrolo[3,2-b]pyrrol-1-yl]-ethyl}-benzamid,
Biphenyl-4-carbonsäure-{1-(4-hydroxy-benzyl)-2-oxo-2-[6-oxo-4-(1-oxy-pyridin-3-sulfonyl)-hexahydro-pyrrolo[3,2-b]pyrrol-1-yl]-ethyl}-amid,
Biphenyl-4-carbonsäure-{1-(4-fluor-benzyl)-2-oxo-2-[6-oxo-4-(1-oxy-pyridin-3-sulfonyl)-hexahydro-pyrrolo[3,2-b]pyrrol-1-yl]-ethyl}-amid,
Biphenyl-4-carbonsäure-{1-(4-methoxy-benzyl)-2-oxo-2-[6-oxo-4-(1-oxy-pyridin-3-sulfonyl)-hexahydro-pyrrolo[3,2-b]pyrrol-1-yl]-ethyl}-amid,
Biphenyl-4-carbonsäure-{1-(3-hydroxy-benzyl)-2-oxo-2-[6-oxo-4-(1-oxy-pyridin-3-sulfonyl)-hexahydro-pyrrolo[3,2-b]pyrrol-1-yl]-ethyl}-amid,
Furan-3-carbonsäure-{3,3-dimethyl-1-[6-oxo-4-(pyridin-2-carbonyl)-hexahydro-pyrrolo[3,2-b]pyrrol-1-carbonyl]-butyl}-amid,
Thiophen-3-carbonsäure-{3,3-dimethyl-1-[6-oxo-4-(pyridin-2-carbonyl)-hexahydro-pyrrolo[3,2-b]pyrrol-1-carbonyl]-butyl}-amid,
Benzo[b]thiophen-3-carbonsäure-{3,3-dimethyl-1-[6-oxo-4-(pyridin-2-carbonyl)-hexahydro-pyrrolo[3,2-b]pyrrol-1-carbonyl]-butyl}-amid,
Furan-3-carbonsäure-{1-cyclohexylmethyl-2-oxo-2-[6-oxo-4-(pyridin-2-carbonyl)-hexahydro-pyrrolo[3,2-b]pyrrol-1-yl]-ethyl}-amid,
Thiophen-3-carbonsäure-{1-cyclohexylmethyl-2-oxo-2-[6-oxo-4-(pyridin-2-carbonyl)-hexahydro-pyrrolo[3,2-b]pyrrol-1-yl]-ethyl}-amid,
Benzo[b]thiophen-3-carbonsäure-{1-cyclohexylmethyl-2-oxo-2-[6-oxo-4-(pyridin-2-carbonyl)-hexahydro-pyrrolo[3,2-b]pyrrol-1-yl]-ethyl}-amid,
Naphthalen-1-carbonsäure-{1-(4-hydroxy-benzyl)-2-oxo-2-[6-oxo-4-(pyridin-2-carbonyl)-hexahydro-pyrrolo[3,2-b]pyrrol-1-yl]-ethyl}-amid,
Chinolin-8-carbonsäure-{1-(4-hydroxy-benzyl)-2-oxo-2-[6-oxo-4-(pyridin-2-carbonyl)-hexahydro-pyrrolo[3,2-b]pyrrol-1-yl]-ethyl}-amid,
4-tert-Butyl-N-{1-(4-hydroxy-benzyl)-2-oxo-2-[6-oxo-4-(pyridin-2-carbonyl)-hexahydro-pyrrolo[3,2-b]pyrrol-1-yl]-ethyl}-benzamid,
4-tert-Butyl-N-{1-(4-fluor-benzyl)-2-oxo-2-[6-oxo-4-(pyridin-2-carbonyl)-hexahydro-pyrrolo[3,2-b]pyrrol-1-yl]-ethyl}-benzamid,
4-tert-Butyl-N-{1-(4-methoxy-benzyl)-2-oxo-2-[6-oxo-4-(pyridin-2-carbonyl)-hexahydro-pyrrolo[3,2-b]pyrrol-1-yl]-ethyl}-benzamid,
4-tert-Butyl-N-{1-(3-hydroxy-benzyl)-2-oxo-2-[6-oxo-4-(pyridin-2-carbonyl)-hexahydro-pyrrolo[3,2-b]pyrrol-1-yl]-ethyl}-benzamid,
Biphenyl-4-carbonsäure-{1-(4-hydroxy-benzyl)-2-oxo-2-[6-oxo-4-(pyridin-2-carbonyl)-hexahydro-pyrrolo[3,2-b]pyrrol-1-yl]-ethyl}-amid,
Biphenyl-4-carbonsäure-{1-(4-fluor-benzyl)-2-oxo-2-[6-oxo-4-(pyridin-2-carbonyl)-hexahydro-pyrrolo[3,2-b]pyrrol-1-yl]-ethyl}-amid,
Biphenyl-4-carbonsäure-{1-(4-methoxy-benzyl)-2-oxo-2-[6-oxo-4-(pyridin-2-carbonyl)-hexahydro-pyrrolo[3,2-b]pyrrol-1-yl]-ethyl}-amid,
Biphenyl-4-carbonsäure-{1-(3-hydroxy-benzyl)-2-oxo-2-[6-oxo-4-(pyridin-2-carbonyl)-hexahydro-pyrrolo[3,2-b]pyrrol-1-yl]-ethyl}-amid,
Furan-3-carbonsäure-{3,3-dimethyl-1-[6-oxo-4-(2-pyridin-3-yl-acetyl)-hexahydro-pyrrolo[3,2-b]pyrrol-1-carbonyl]-butyl}-amid,
Thiophen-3-carbonsäure-{3,3-dimethyl-1-[6-oxo-4-(2-pyridin-3-yl-acetyl)-hexahydro-pyrrolo[3,2-b]pyrrol-1-carbonyl]-butyl}-amid,
Benzo[b]thiophen-3-carbonsäure-{3,3-dimethyl-1-[6-oxo-4-(2-pyridin-3-yl-acetyl)-hexahydro-pyrrolo[3,2-b]pyrrol-1-carbonyl]-butyl}-amid,
Furan-3-carbonsäure-{1-cyclohexylmethyl-2-oxo-2-[6-oxo-4-(2-pyridin-3-yl-acetyl)-hexahydro-pyrrolo[3,2-b]pyrrol-1-yl]-ethyl}-amid,
Thiophen-3-carbonsäure-{1-cyclohexylmethyl-2-oxo-2-[6-oxo-4-(2-pyridin-3-yl-acetyl)-hexahydro-pyrrolo[3,2-b]pyrrol-1-yl]-ethyl}-amid,
Benzo[b]thiophen-3-carbonsäure-{1-cyclohexylmethyl-2-oxo-2-[6-oxo-4-(2-pyridin-3-yl-acetyl)-hexahydro-pyrrolo[3,2-b]pyrrol-1-yl]-ethyl}-amid,
Naphthalen-1-carbonsäure-{1-(4-hydroxy-benzyl)-2-oxo-2-[6-oxo-4-(2-pyridin-3-yl-acetyl)-hexahydro-pyrrolo[3,2-b]pyrrol-1-yl]-ethyl}-amid,
Chinolin-8-carbonsäure-{1-(4-hydroxy-benzyl)-2-oxo-2-[6-oxo-4-(2-pyridin-3-yl-acetyl)-hexahydro-pyrrolo[3,2-b]pynrol-1-yl]-ethy}-amid,
4-tert-Butyl-N-{1-(4-hydroxy-benzyl)-2-oxo-2-[6-oxo-4-(2-pyridin-3-yl-acetyl)-hexahydro-pyrrolo[3,2-b]pyrrol-1-yl]-ethyl}-benzamid,
4-tert-Butyl-N-{1-(4-fluor-benzyl)-2-oxo-2-[6-oxo-4-(2-pyridin-3-yl-acetyl)-hexahydro-pyrrolo[3,2-b]pyrrol-1-yl]-ethyl}-benzamid,
4-tert-Butyl-N-{1-(4-methoxy-benzyl)-2-oxo-2-[6-oxo-4-(2-pyridin-3-yl-acetyl)-hexahydro-pyrrolo[3,2-b]pyrrol-1-yl]-ethyl}-benzamid,
4-tert-Butyl-N-{1-(3-hydroxy-benzyl)-2-oxo-2-[6-oxo-4-(2-pyridin-3-yl-acetyl)-hexahydro-pyrrolo[3,2-b]pyrrol-1-yl]-ethyl}-benzamid,
Biphenyl-4-carbonsäure-{1-(4-hydroxy-benzyl)-2-oxo-2-[6-oxo-4-(2-pyridin-3-yl-acetyl)-hexahydro-pyrrolo[3,2-b]pyrrol-1-yl]-ethyl}-amid,
Biphenyl-4-carbonsäure-{1-(4-fluor-benzyl)-2-oxo-2-[6-oxo-4-(2-pyridin-3-yl-acetyl)-hexahydro-pyrrolo[3,2-b]pyrrol-1-yl]-ethyl}-amid,
Biphenyl-4-carbonsäure-{1-(4-methoxy-benzyl)-2-oxo-2-[6-oxo-4-(2-pyridin-3-yl-acetyl)-hexahydro-pyrrolo[3,2-b]pyrrol-1-yl]-ethyl}-amid,
Biphenyl-4-carbonsäure-{1-(3-hydroxy-benzyl)-2-oxo-2-[6-oxo-4-(2-pyridin-3-yl-acetyl)-hexahydro-pyrrolo[3,2-b]pyrrol-1-yl]-ethyl}-amid,
Furan-3-carbonsäure-{3,3-dimethyl-1-[6-oxo-4-(pyridin-2-ylmethansulfonyl)-hexahydro-pyrrolo[3,2-b]pyrrol-1-carbonyl]-butyl}-amid,
Thiophen-3-carbonsäure-{3,3-dimethyl-1-[6-oxo-4-(pyridin-2-ylmethansulfonyl)-hexahydro-pyrrolo[3,2-b]pyrrol-1-carbonyl]-butyl}-amid,
Benzo[b]thiophen-3-carbonsäure-{3,3-dimethyl-1-[6-oxo-4-(pyridin-2-ylmethansulfonyl)-hexahydro-pyrrolo[3,2-b]pyrrol-1-carbonyl]-butyl}-amid,
Furan-3-carbonsäure-{1-cyclohexylmethyl-2-oxo-[6-oxo-4-(pyridin-2-ylmethansulfonyl)-hexahydro-pyrrolo[3,2-b]pyrrol-1-yl]-ethyl}-amid,
Thiophen-3-carbonsäure-{1-cyclohexylmethyl-2-oxo-2-[6-oxo-4-(pyridin-2-ylmethansulfonyl)-hexahydro-pyrrolo[3,2-b]pyrrol-1-yl]-ethyl}-amid,
Benzo[b]thiophen-3-carbonsäure-{1-cyclohexylmethyl-2-oxo-2-[6-oxo-4-(pyridin-2-ylmethansulfonyl)-hexahydro-pyrrolo[3,2-b]pyrrol-1-yl]-ethyl}-amid,
Naphthalen-1-carbonsäure-{1-(4-hydroxy-benzyl)-2-oxo-2-[6-oxo-4-(pyridin-2-ylmethansulfonyl)-hexahydro-pyrrolo[3,2-b]pyrrol-1-yl]-ethyl}-amid,
Chinolin-8-carbonsäure-{1-(4-hydroxy-benzyl)-2-oxo-2-[6-oxo-4-(pyridin-2-ylmethansulfonyl)-hexahydro-pyrrolo[3,2-b]pyrrol-1-yl]-ethyl}-amid,
4-tert-Butyl-N-{1-(4-hydroxy-benzyl)-2-oxo-2-[6-oxo-4-(pyridin-2-ylmethansulfonyl)-hexahydro-pyrrolo[3,2-b]pyrrol-1-yl]-ethyl}-benzamid,
4-tert-Butyl-N-{1-(4-fluor-benzyl)-2-oxo-2-[6-oxo-4-(pyridin-2-ylmethansulfonyl)-hexahydro-pyrrolo[3,2-b]pyrrol-1-yl]-ethyl}-benzamid,
4-tert-Butyl-N-{1-(4-methoxy-benzyl)-2-oxo-2-[6-oxo-4-(pyridin-2-ylmethansulfonyl)-hexahydro-pyrrolo[3,2-b]pyrrol-1-yl]-ethyl}-benzamid,
4-tert-Butyl-N-{1-(3-hydroxy-benzyl)-2-oxo-2-[6-oxo-4-(pyridin-2-ylmethansulfonyl)-hexahydro-pyrrolo[3,2-b]pyrrol-1-yl]-ethyl}-benzamid,
Biphenyl-4-carbonsäure-{1-(4-hydroxy-benzyl)-2-oxo-2-[6-oxo-4-(pyridin-2-ylmethansulfonyl)-hexahydro-pyrrolo[3,2-b]pyrrol-1-yl]-ethyl}-amid,
Biphenyl-4-carbonsäure-{1-(4-fluor-benzyl)-2-oxo-2-[6-oxo-4-(pyridin-2-ylmethansulfonyl)-hexahydro-pyrrolo[3,2-b]pyrrol-1-yl]-ethyl}-amid,
Biphenyl-4-carbonsäure-{1-(4-methoxy-benzyl)-2-oxo-2-[6-oxo-4-(pyridin-2-ylmethansulfonyl)-hexahydro-pyrrolo[3,2-b]pyrrol-1-yl]-ethyl}-amid,
Biphenyl-4-carbonsäure-{1-(3-hydroxy-benzyl)-2-oxo-2-[6-oxo-4-(pyridin-2-ylmethansulfonyl)-hexahydro-pyrrolo[3,2-b]pyrrol-1-yl]-ethyl}-amid,
Furan-3-carbonsäure-{3,3-dimethyl-1-[6-oxo-4-(pyridin-3-ylmethansulfonyl)-hexahydro-pyrrolo[3,2-b]pyrrol-1-carbonyl]-butyl}-amid,
Thiophen-3-carbonsäure-{3,3-dimethyl-1-[6-oxo-4-(pyridin-3-ylmethansulfonyl)-hexahydro-pyrrolo[3,2-b]pyrrol-1-carbonyl]-butyl}-amid,
Benzo[b]thiophen-3-carbonsäure-{3,3-dimethyl-1-[6-oxo-4-(pyridin-3-ylmethansulfonyl)-hexahydro-pyrrolo[3,2-b]pyrrol-1-carbonyl]-butyl}-amid,
Furan-3-carbonsäure-{1-cyclohexylmethyl-2-oxo-2-[6-oxo-4-(pyridin-3-ylmethansulfonyl)-hexahydro-pyrrolo[3,2-b]pyrrol-1-yl]-ethyl}-amid,
Thiophen-3-carbonsäure-{1-cyclohexylmethyl-2-oxo-2-[6-oxo-4-(pyridin-3-ylmethansulfonyl)-hexahydro-pyrrolo[3,2-b]pyrrol-1-yl]-ethyl}-amid,
Benzo[b]thiophen-3-carbonsäure-{1-cyclohexylmethyl-2-oxo-2-[6-oxo-4-(pyridin-3-ylmethansulfonyl)-hexahydro-pyrrolo[3,2-b]pyrrol-1-yl]-ethyl}-amid,
Naphthalen-1-carbonsäure-{1-(4-hydroxy-benzyl)-2-oxo-2-[6-oxo-4-(pyridin-3-ylmethansulfonyl)-hexahydro-pyrrolo[3,2-b]pyrrol-1-yl]-ethyl}-amid,
Chinolin-8-carbonsäure-{1-(4-hydroxy-benzyl)-2-oxo-2-[6-oxo-4-(pyridin-3-ylmethansulfonyl)-hexahydro-pyrrolo[3,2-b]pyrrol-1-yl]-ethyl}-amid,
4-tert-Butyl-N-{1-(4-hydroxy-benzyl)-2-oxo-2-[6-oxo-4-(pyridin-3-ylmethansulfonyl)-hexahydro-pyrrolo[3,2-b]pyrrol-1-yl]-ethyl}-benzamid,
4-tert-Butyl-N-{1-(4-fluor-benzyl)-2-oxo-2-[6-oxo-4-(pyridin-3-ylmethansulfonyl)-hexahydro-pyrrolo[3,2-b]pyrrol-1-yl]-ethyl}-benzamid,
4-tert-Butyl-N-{1-(4-methoxy-benzyl)-2-oxo-2-[6-oxo-4-(pyridin-3-ylmethansulfonyl)-hexahydro-pyrrolo[3,2-b]pyrrol-1-yl]-ethyl}-benzamid,
4-tert-Butyl-N-{1-(3-hydroxy-benzyl)-2-oxo-2-[6-oxo-4-(pyridin-3-ylmethansulfonyl)-hexahydro-pyrrolo[3,2-b]pyrrol-1-yl]-ethyl}-benzamid,
Biphenyl-4-carbonsäure-{1-(4-hydroxy-benzyl)-2-oxo-2-[6-oxo-4-(pyridin-3-ylmethansulfonyl)-hexahydro-pyrrolo[3,2-b]pyrrol-1-yl]-ethyl}-amid,
Biphenyl-4-carbonsäure-{1-(4-fluor-benzyl)-2-oxo-2-[6-oxo-4-(pyridin-3-ylmethansulfonyl)-hexahydro-pyrrolo[3,2-b]pyrrol-1-yl]-ethyl}-amid,
Biphenyl-4-carbonsäure-{1-(4-methoxy-benzyl)-2-oxo-2-[6-oxo-4-(pyridin-3-ylmethansulfonyl)-hexahydro-pyrrolo[3,2-b]pyrrol-1-yl]-ethyl}-amid,
Biphenyl-4-carbonsäure-{1-(3-hydroxy-benzyl)-2-oxo-2-[6-oxo-4-(pyridin-3-ylmethansulfonyl)-hexahydro-pyrrolo[3,2-b]pyrrol-1-yl]-ethyl}-amid,
Furan-3-carbonsäure-{3,3-dimethyl-1-[6-oxo-4-(1-oxy-pyridin-2-ylmethansulfonyl)-hexahydro-pyrrolo[3,2-b]pyrrol-1-carbonyl]-butyl}-amid,
Thiophen-3-carbonsäure-{3,3-dimethyl-1-[6-oxo-4-(1-oxy-pyridin-2-ylmethansulfonyl)-hexahydro-pyrrolo[3,2-b]pyrrol-1-carbonyl]-butyl}-amid,
Benzo[b]thiophen-3-carbonsäure-{3,3-dimethyl-1-[6-oxo-4-(1-oxy-pyridin-2-ylmethansulfonyl)-hexahydro-pyrrolo[3,2-b]pyrrol-1-carbonyl]-butyl}-amid,
Furan-3-carbonsäure-{1-cyclohexylmethyl-2-oxo-2-[6-oxo-4-(1-oxy-pyridin-2-ylmethansulfonyl)-hexahydro-pyrrolo[3,2-b]pyrrol-1-yl]-ethyl}-amid,
Thiophen-3-carbonsäure-{1-cyclohexylmethyl-2-oxo-2-[6-oxo-4-(1-oxy-pyridin-2-ylmethansulfonyl)-hexahydro-pyrrolo[3,2-b]pyrrol-1-yl]-ethyl}-amid,
Benzo[b]thiophen-3-carbonsäure-{1-cyclohexylmethyl-2-oxo-2-[6-oxo-4-(1-oxy-pyridin-2-ylmethansulfonyl)-hexahydro-pyrrolo[3,2-b]pyrrol-1-yl]-ethyl}-amid,
Naphthalen-1-carbonsäure-{1-(4-hydroxy-benzyl)-2-oxo-2-[6-oxo-4-(1-oxy-pyridin-2-ylmethansulfonyl)-hexahydro-pyrrolo[3,2-b]pyrrol-1-yl]-ethyl}-amid,
Chinolin-8-carbonsäure-{1-(4-hydroxy-benzyl)-2-oxo-2-[6-oxo-4-(1-oxy-pyridin-2-ylmethansulfonyl)-hexahydro-pyrrolo[3,2-b]pyrrol-1-yl]-ethyl}-amid,
4-tert-Butyl-N-{1-(4-hydroxy-benzyl)-2-oxo-2-[6-oxo-4-(1-oxy-pyridin-2-ylmethansulfonyl)-hexahydro-pyrrolo[3,2-b]pyrrol-1-yl]-ethyl}-benzamid,
4-tert-Butyl-N-{1-(4-fluor-benzyl)-2-oxo-2-[6-oxo-4-(1-oxy-pyridin-2-ylmethansulfonyl)-hexahydro-pyrrolo[3,2-b]pyrrol-1-yl]-ethyl}-benzamid,
4-tert-Butyl-N-{1-(4-methoxy-benzyl)-2-oxo-2-[6-oxo-4-(1-oxy-pyridin-2-ylmethansulfonyl)-hexahydro-pyrrolo[3,2-b]pyrrol-1-yl]-ethyl}-benzamid,
4-tert-Butyl-N-{1-(3-hydroxy-benzyl)-2-oxo-2-[6-oxo-4-(1-oxy-pyridin-2-ylmethansulfonyl)-hexahydro-pyrrolo[3,2-b]pyrrol-1-yl]-ethyl}-benzamid,
Biphenyl-4-carbonsäure-{1-(4-hydroxy-benzyl)-2-oxo-2-[6-oxo-4-(1-oxy-pyridin-2-ylmethansulfonyl)-hexahydro-pyrrolo[3,2-b]pyrrol-1-yl]-ethyl}-amid,
Biphenyl-4-carbonsäure-{1-(4-fluor-benzyl)-2-oxo-2-[6-oxo-4-(1-oxy-pyridin-2-ylmethansulfonyl)-hexahydro-pyrrolo[3,2-b]pyrrol-1-yl]-ethyl}-amid,
Biphenyl-4-carbonsäure-{1-(4-methoxy-benzyl)-2-oxo-2-[6-oxo-4-(1-oxy-pyridin-2-ylmethansulfonyl)-hexahydro-pyrrolo[3,2-b]pyrrol-1-yl]-ethyl}-amid,
Biphenyl-4-carbonsäure-{1-(3-hydroxy-benzyl)-2-oxo-2-[6-oxo-4-(1-oxy-pyridin-2-ylmethansulfonyl)-hexahydro-pyrrolo[3,2-b]pyrrol-1-yl]-ethyl}-amid,
Furan-3-carbonsäure-{3,3-dimethyl-1-[6-oxo-4-(1-oxy-pyridin-3-ylmethansulfonyl)-hexahydro-pyrrolo[3,2-b]pyrrol-1-carbonyl]-butyl}-amid,
Thiophen-3-carbonsäure-{3,3-dimethyl-1-[6-oxo-4-(1-oxy-pyridin-3-ylmethansulfonyl)-hexahydro-pyrrolo[3,2-b]pyrrol-1-carbonyl]-butyl}-amid,
Benzo[b]thiophen-3-carbonsäure-{3,3-dimethyl-1-[6-oxo-4-(1-oxy-pyridin-3-ylmethansulfonyl)-hexahydro-pyrrolo[3,2-b]pyrrol-1-carbonyl]-butyl}-amid,
Furan-3-carbonsäure-{1-cyclohexylmethyl-2-oxo-2-[6-oxo-4-(1-oxy-pyridin-3-ylmethansulfonyl)-hexahydro-pyrrolo[3,2-b]pyrrol-1-yl]-ethyl}-amid,
Thiophen-3-carbonsäure-{1-cyclohexylmethyl-2-oxo-2-[6-oxo-4-(1-oxy-pyridin-3-ylmethansulfonyl)-hexahydro-pyrrolo[3,2-b]pyrrol-1-yl]-ethyl}-amid,
Benzo[b]thiophen-3-carbonsäure-{1-cyclohexylmethyl-2-oxo-2-[6-oxo-4-(1-oxy-pyridin-3-ylmethansulfonyl)-hexahydro-pyrrolo[3,2-b]pyrrol-1-yl]-ethyl}-amid,
Naphthalen-1-carbonsäure-{1-(4-hydroxy-benzyl)-2-oxo-2-[6-oxo-4-(1-oxy-pyridin-3-ylmethansulfonyl)-hexahydro-pyrrolo[3,2-b]pyrrol-1-yl]-ethyl}-amid,
Chinolin-8-carbonsäure-{1-(4-hydroxy-benzyl)-2-oxo-2-[6-oxo-4-(1-oxy-pyridin-3-ylmethansulfonyl)-hexahydro-pyrrolo[3,2-b]pyrrol-1-yl]-ethyl}-amid,
4-tert-Butyl-N-{1-(4-hydroxy-benzyl)-2-oxo-2-[6-oxo-4-(1-oxy-pyridin-3-ylmethansulfonyl)-hexahydro-pyrrolo[3,2-b]pyrrol-1-yl]-ethyl}-benzamid,
4-tert-Butyl-N-{1-(4-fluor-benzyl)-2-oxo-2-[6-oxo-4-(1-oxy-pyridin-3-ylmethansulfonyl)-hexahydro-pyrrolo[3,2-b]pyrrol-1-yl]-ethyl}-benzamid,
4-tert-Butyl-N-{1-(4-methoxy-benzyl)-2-oxo-2-[6-oxo-4-(1-oxy-pyridin-3-ylmethansulfonyl)-hexahydro-pyrrolo[3,2-b]pyrrol-1-yl]-ethyl}-benzamid,
4-tert-Butyl-N-{1-(3-hydroxy-benzyl)-2-oxo-2-[6-oxo-4-(1-oxy-pyridin-3-ylmethansulfonyl)-hexahydro-pyrrolo[3,2-b]pyrrol-1-yl]-ethyl}-benzamid,
Biphenyl-4-carbonsäure-{1-(4-hydroxy-benzyl)-2-oxo-2-[6-oxo-4-(1-oxy-pyridin-3-ylmethansulfonyl)-hexahydro-pyrrolo[3,2-b]pyrrol-1-yl]-ethyl}-amid,
Biphenyl-4-carbonsäure-{1-(4-fluor-benzyl)-2-oxo-2-[6-oxo-4-(1-oxy-pyridin-3-ylmethansulfonyl)-hexahydro-pyrrolo[3,2-b]pyrrol-1-yl]-ethyl}-amid,
Biphenyl-4-carbonsäure-{1-(4-methoxy-benzyl)-2-oxo-2-[6-oxo-4-(1-oxy-pyridin-3-ylmethansulfonyl)-hexahydro-pyrrolo[3,2-b]pyrrol-1-yl]-ethyl}-amid,
Biphenyl-4-carbonsäure-{1-(3-hydroxy-benzyl)-2-oxo-2-[6-oxo-4-(1-oxy-pyridin-3-ylmethansulfonyl)-hexahydro-pyrrolo[3,2-b]pyrrol-1-yl]-ethyl}-amid,
2-Cyclohexylmethyl-4-morpholin-4-yl-1-[6-oxo-4-(pyridin-2-sulfonyl)-hexahydro-pyrrolo[3,2-b]pyrrol-1-yl]-butan-1,4-dion,
2-Cyclohexylmethyl-4-morpholin-4-yl-1-[6-oxo-4-(pyridin-3-sulfonyl)-hexahydro-pyrrolo[3,2-b]pyrrol-1-yl]-butan-1,4-dion,
2-Cyclohexylmethyl-4-morpholin-4-yl-1-[6-oxo-4-(1-oxy-pyridin-2-sulfonyl)-hexahydro-pyrrolo[3,2-b]pyrrol-1-yl]-butan-1,4-dion,
2-Cyclohexylmethyl-4-morpholin-4-yl-1-[6-oxo-4-(1-oxy-pyridin-3-sulfonyl)-hexahydro-pyrrolo[3,2-b]pyrrol-1-yl]-butan-1,4-dion,
2-Cyclohexylmethyl-4-morpholin-4-yl-1-[6-oxo-4-(pyridin-2-carbonyl)-hexahydro-pyrrolo[3,2-b]pyrrol-1-yl]-butan-1,4-dion,
2-Cyclohexylmethyl-4-morpholin-4-yl-1-[6-oxo-4-(2-pyridin-3-yl-acetyl)-hexahydro-pyrrolo[3,2-b]pyrrol-1-yl]-butan-1,4-dion,
2-Cyclohexylmethyl-4-morpholin-4-yl-1-[6-oxo-4-(pyridin-2-ylmethansulfonyl)-hexahydro-pyrrolo[3,2-b]pyrrol-1-yl]-butan-1,4-dion,
2-Cyclohexylmethyl-4-morpholin-4-yl-1-[6-oxo-4-(pyridin-3-ylmethansulfonyl)-hexahydro-pyrrolo[3,2-b]pyrrol-1-yl]-butan-1,4-dion,
2-Cyclohexylmethyl-4-morpholin-4-yl-1-[6-oxo-4-(1-oxy-pyridin-2-ylmethansulfonyl)-hexahydro-pyrrolo[3,2-b]pyrrol-1-yl]-butan-1,4-dion,
2-Cyclohexylmethyl-4-morpholin-4-yl-1-[6-oxo-4-(1-oxy-pyridin-3-ylmethansulfonyl)-hexahydro-pyrrolo[3,2-b]pyrrol-1-yl]-butan-1,4-dion,
2-(2,2-Dimethyl-propyl)-4-morpholin-4-yl-1-[6-oxo-4-(pyridin-2-sulfonyl)-hexahydro-pyrrolo[3,2-b]pyrrol-1-yl]-butan-1,4-dion,
2-(2,2-Dimethyl-propyl)-4-morpholin-4-yl-1-[6-oxo-4-(pyridin-3-sulfonyl)-hexahydro-pyrrolo[3,2-b]pyrrol-1-yl]-butan-1,4-dion,
2-(2,2-Dimethyl-propyl)-4-morpholin-4-yl-1-[6-oxo-4-(1-oxy-pyridin-2-sulfonyl)-hexahydro-pyrrolo[3,2-b]pyrrol-1-yl]-butan-1,4-dion,
2-(2,2-Dimethyl-propyl)-4-morpholin-4-yl-1-[6-oxo-4-(1-oxy-pyridin-3-sulfonyl)-hexahydro-pyrrolo[3,2-b]pyrrol-1-yl]-butan-1,4-dion,
2-(2,2-Dimethyl-propyl)-4-morpholin-4-yl-1-[6-oxo-4-(pyridin-2-carbonyl)-hexahydro-pyrrolo[3,2-b]pyrrol-1-yl]-butan-1,4-dion,
2-(2,2-Dimethyl-propyl)-4-morpholin-4-yl-1-[6-oxo-4-(2-pyridin-3-yl-acetyl)-hexahydro-pyrrolo[3,2-b]pyrrol-1-yl]-butan-1,4-dion,
2-(2,2-Dimethyl-propyl)-4-morpholin-4-yl-1-[6-oxo-4-(pyridin-2-ylmethansulfonyl)-hexahydro-pyrrolo[3,2-b]pyrrol-1-yl]-butan-1,4-dion,
2-(2,2-Dimethyl-propyl)-4-morpholin-4-yl-1-[6-oxo-4-(pyridin-3-ylmethansulfonyl)-hexahydro-pyrrolo[3,2-b]pyrrol-1-yl]-butan-1,4-dion,
2-(2,2-Dimethyl-propyl)-4-morpholin-4-yl-1-[6-oxo-4-(1-oxy-pyridin-2-ylmethansulfonyl)-hexahydro-pyrrolo[3,2-b]pyrrol-1-yl]-butan-1,4-dion,
2-(2,2-Dimethyl-propyl)-4-morpholin-4-yl-1-[6-oxo-4-(1-oxy-pyridin-3-ylmethansulfonyl)-hexahydro-pyrrolo[3,2-b]pyrrol-1-yl]-butan-1,4-dion,
N-[1-(4-Benzoyl-6-oxo-hexahydro-pyrrolo[3,2-b]pyrrol-1-carbonyl)-3-methyl-butyl]-4-tert-butylbenzamid,
4-tert-Butyl-N-{3-methyl-1-[6-oxo-4-(1-oxy-pyridin-2-carbonyl)-hexahydro-pyrrolo[3,2-b]pyrrol-1-carbonyl]-butyl}-benzamid,
4-tert-Butyl-N-{3-methyl-1-[6-oxo-4-(pyridin-3-carbonyl)-hexahydro-pyrrolo[3,2-b]pyrrol-1-carbonyl]-butyl}-benzamid,
4-tert-Butyl-N-{3-methyl-1-[6-oxo-4-(1-oxy-pyridin-3-carbonyl)-hexahydro-pyrrolo[3,2-b]pyrrol-1-carbonyl]-butyl}-benzamid,
4-tert-Butyl-N-{3-methyl-1-[6-oxo-4-(pyridin-4-carbonyl)-hexahydro-pyrrolo[3,2-b]pyrrol-1-carbonyl]-butyl}-benzamid,
4-tert-Butyl-N-{3-methyl-1-[6-oxo-4-(1-oxy-pyridin-4-carbonyl)-hexahydro-pyrrolo[3,2-b]pyrrol-1-carbonyl]-butyl}-benzamid,
N-[1-(4-Benzensulfonyl-6-oxo-hexahydro-pyrrolo[3,2-b]pyrrol-1-carbonyl)-3-methyl-butyl]-4-tert-butyl-benzamid,
4-tert-Butyl-N-{3-methyl-1-[6-oxo-4-(pyridin-4-sulfonyl)-hexahydro-pyrrolo[3,2-b]pyrrol-1-carbonyl]-butyl}-benzamid,
4-tert-Butyl-N-{3-methyl-1-[6-oxo-4-(1-oxy-pyridin-4-sulfonyl)-hexahydro-pyrrolo[3,2-b]pyrrol-1-carbonyl]-butyl}-benzamid,
4-tert-Butyl-N-[3-methyl-1-(6-oxo-4-phenylacetyl-hexahydro-pyrrolo[3,2-b]pyrrol-1-carbonyl)-butyl]-benzamid,
4-[2-(4-tert-Butyl-benzoylamino)-4-methyl-pentanoyl]-3-oxo-hexahydro-pyrrolo[3,2-b]pyrrol-1-carbonsäurephenylamid,
4-[2-(4-tert-Butyl-benzoylamino)-4-methyl-pentanoyl]-3-oxo-hexahydro-pyrrolo[3,2-b]pyrrol-1-carbonsäure-isobutyl-amid,
4-[2-(4-tert-Butyl-benzoylamino)-4-methyl-pentanoyl]-3-oxo-hexahydro-pyrrolo[3,2-b]pyrrol-1-carbonsäurephenylester,
4-[2-(4-tert-Butyl-benzoylamino)-4-methyl-pentanoyl]-3-oxo-hexahydro-pyrrolo[3,2-b]pyrrol-1-carbonsäureisobutylester,
4-tert-Butyl-N-{3-methyl-1-[6-oxo-4-(pyrrolidin-1-carbonyl)-hexahydro-pyrrolo[3,2-b]pyrrol-1-carbonyl]-butyl}-benzamid,
4-tert-Butyl-N-{3-methyl-1-[6-oxo-4-(piperidin-1-carbonyl)-hexahydro-pyrrolo[3,2-b]pyrrol-1-carbonyl]-butyl}-benzamid,
4-tert-Butyl-N-{3-methyl-1-[4-(morpholine-4-carbonyl)-6-oxo-hexahydro-pyrrolo[3,2-b]pyrrol-1-carbonyl]-butyl}-benzamid,
4-tert-Butyl-N-{3-methyl-1-[6-oxo-4-(piperazin-1-carbonyl)-hexahydro-pyrrolo[3,2-b]pyrrol-1-carbonyl]-butyl}-benzamid,

4-tert-Butyl-N-{3-methyl-1-[4-(4-methyl-piperazin-1-carbonyl)-6-oxo-hexahydro-pyrrolo[3,2-b]pyrrol-1-carbonyl]-butyl}-benzamid,
4-tert-Butyl-N-(3-methyl-1-{4-[2-(4-methyl-piperazin-1-yl)-acetyl]-6-oxo-hexahydro-pyrrolo[3,2-b]pyrrol-1-carbonyl}-butyl)-benzamid,
4-tert-Butyl-N-{3-methyl-1-[6-oxo-4-(2-piperazin-1-yl-acetyl)-hexahydro-pyrrolo[3,2-b]pyrrol-1-carbonyl]-butyl}-benzamid,
4-tert-Butyl-N-{1-[4-(furan-2-carbonyl)-6-oxo-hexahydro-pyrrolo[3,2-b]pyrrol-1-carbonyl]-3-methyl-butyl}-benzamid,
4-tert-Butyl-N-{1-[4-(5-chlor-furan-2-carbonyl)-6-oxo-hexahydro-pyrrolo[3,2-b]pyrrol-1-carbonyl]-3-methyl-butyl}-benzamid,
4-tert-Butyl-N-{1-[4-(thiophen-2-carbonyl)-6-oxo-hexahydro-pyrrolo[3,2-b]pyrrol-1-carbonyl]-3-methyl-butyl}-benzamid,
4-tert-Butyl-N-{1-[4-(5-chlor-thiophen-2-carbonyl)-6-oxo-hexahydro-pyrrolo[3,2-b]pyrrol-1-carbonyl]-3-methyl-butyl}-benzamid,
4-tert-Butyl-N-{1-[4-(furan-3-carbonyl)-6-oxo-hexahydro-pyrrolo[3,2-b]pyrrol-1-carbonyl]-3-methyl-butyl}-benzamid,
4-tert-Butyl-N-{1-[4-(thiophen-3-carbonyl)-6-oxo-hexahydro-pyrrolo[3,2-b]pyrrol-1-carbonyl]-3-methyl-butyl}-benzamid,
4-tert-Butyl-N-[1-(4-cyclopentancarbonyl-6-oxo-hexahydro-pyrrolo[3,2-b]pyrrol-1-carbonyl)-3-methyl-butyl]-benzamid,
4-tert-Butyl-N-[1-(4-cyclohexancarbonyl-6-oxo-hexahydro-pyrrolo[3,2-b]pyrrol-1-carbonyl)-3-methyl-butyl]-benzamid,
4-tert-Butyl-N-{1-[4-(cyclopent-3-encarbonyl)-6-oxo-hexahydro-pyrrolo[3,2-b]pyrrol-1-carbonyl]-3-methyl-butyl}-benzamid,
4-tert-Butyl-N-{1-[4-(2-cyclopentyl-acetyl)-6-oxo-hexahydro-pyrrolo[3,2-b]pyrrol-1-carbonyl]-3-methyl-butyl}-benzamid,
4-tert-Butyl-N-{1-[4-(2-cyclohexyl-acetyl)-6-oxo-hexahydro-pyrrolo[3,2-b]pyrrol-1-carbonyl]-3-methyl-butyl}-benzamid,
4-tert-Butyl-N-{3-methyl-1-[6-oxo-4-(pyrrolidin-2-carbonyl)-hexahydro-pyrrolo[3,2-b]pyrrol-1-carbonyl]-butyl}-benzamid,
N-{1-[4-(1-Acetyl-pyrrolidin-2-carbonyl)-6-oxo-hexahydro-pyrrolo[3,2-b]pyrrol-1-carbonyl]-3-methyl-butyl}-4-tert-butyl-benzamid,
4-tert-Butyl-N-{3-methyl-1-[6-oxo-4-(piperidin-2-carbonyl)-hexahydro-pyrrolo[3,2-b]pyrrol-1-carbonyl]-butyl}-benzamid,
N-{1-[4-(1-Acetyl-piperidin-2-carbonyl)-6-oxo-hexahydro-pyrrolo[3,2-b]pyrrol-1-carbonyl]-3-methyl-butyl}-4-tert-butyl-benzamid,
4-tert-Butyl-N-{3-methyl-1-[6-oxo-4-(tetrahydro-furan-2-carbonyl)-hexahydro-pyrrolo[3,2-b]pyrrol-1-carbonyl]-butyl}-benzamid,
N-{1-[4-(1-Amino-cyclobutancarbonyl)-6-oxo-hexahydro-pyrrolo[3,2-b]pyrrol-1-carbonyl]-3-methyl-butyl}-4-tert-butyl-benzamid,
N-{1-[4-(1-Amino-cyclopentancarbonyl)-6-oxo-hexahydro-pyrrolo[3,2-b]pyrrol-1-carbonyl]-3-methyl-butyl}-4-tert-butyl-benzamid,
N-{1-[4-(1-Amino-cyclohexancarbonyl)-6-oxo-hexahydro-pyrrolo[3,2-b]pyrrol-1-carbonyl]-3-methyl-butyl}-4-tert-butyl-benzamid,
N-{1-[4-(1-Acetylamino-cyclobutancarbonyl)-6-oxo-hexahydro-pyrrolo[3,2-b]pyrrol-1-carbonyl]-3-methyl-butyl}-4-tert-butyl-benzamid,
N-{1-[4-(1-Acetylamino-cyclopentancarbonyl)-6-oxo-hexahydro-pyrrolo[3,2-b]pyrrol-1-carbonyl]-3-methyl-butyl}-4-tert-butyl-benzamid,
N-{1-[4-(1-Acetylamino-cyclohexancarbonyl)-6-oxo-hexahydro-pyrrolo[3,2-b]pyrrol-1-carbonyl]-3-methyl-butyl}-4-tert-butyl-benzamid,
4-tert-Butyl-N-{1-[4-(1-hydroxy-cyclobutancarbonyl)-6-oxo-hexahydro-pyrrolo[3,2-b]pyrrol-1-carbonyl]-3-methyl-butyl}-benzamid,
4-tert-Butyl-N-{1-[4-(1-hydroxy-cyclopentancarbonyl)-6-oxo-hexahydro-pyrrolo[3,2-b]pyrrol-1-carbonyl]-3-methyl-butyl}-benzamid,
4-tert-Butyl-N-{1-[4-(1-hydroxy-cyclohexancarbonyl)-6-oxo-hexahydro-pyrrolo[3,2-b]pyrrol-1-carbonyl]-3-methyl-butyl}-benzamid,
4-tert-Butyl-N-{1-[4-(1-methoxy-cyclobutancarbonyl)-6-oxo-hexahydro-pyrrolo[3,2-b]pyrrol-1-carbonyl]-3-methyl-butyl}-benzamid,
4-tert-Butyl-N-{1-[4-(1-methoxy-cyclopentancarbonyl)-6-oxo-hexahydro-pyrrolo[3,2-b]pyrrol-1-carbonyl]-3-methyl-butyl}-benzamid,
4-tert-Butyl-N-{1-[4-(1-methoxy-cyclohexancarbonyl)-6-oxo-hexahydro-pyrrolo[3,2-b]pyrrol-1-carbonyl]-3-methyl-butyl}-benzamid,
N-{1-[4-(2-Amino-2-cyclopentyl-acetyl)-6-oxo-hexahydro-pyrrolo[3,2-b]pyrrol-1-carbonyl]-3-methyl-butyl}-4-tert-butyl-benzamid,
N-{1-[4-(2-Amino-2-cyclohexyl-acetyl)-6-oxo-hexahydro-pyrrolo[3,2-b]pyrrol-1-carbonyl]-3-methyl-butyl}-4-tert-butyl-benzamid,
N-{1-[4-(2-Acetylamino-2-cyclopentyl-acetyl)-6-oxo-hexahydro-pyrrolo[3,2-b]pyrrol-1-carbonyl]-3-methyl-butyl}-4-tert-

butyl-benzamid,
N-{1-[4-(2-Acetylamino-2-cyclohexyl-acetyl)-6-oxo-hexahydro-pyrrolo[3,2-b]pyrrol-1-carbonyl]-3-methyl-butyl}-4-tert-butyl-benzamid,
N-{1-[4-(2-Acetylamino-propionyl)-6-oxo-hexahydro-pyrrolo[3,2-b]pyrrol-1-carbonyl]-3-methyl-butyl}-4-tert-butyl-benzamid,
N-{1-[4-(2-Acetylamino-acetyl)-6-oxo-hexahydro-pyrrolo[3,2-b]pyrrol-1-carbonyl]-3-methyl-butyl}-4-tert-butyl-benzamid,
N-{1-[4-(2-Acetyl-methyl-amino)-acetyl]-6-oxo-hexahydro-pyrrolo[3,2-b]pyrrol-1-carbonyl}-3-methyl-butyl)-4-tert-butyl-benzamid,
4-tert-Butyl-N-{1-[4-(2-dimethylamino-acetyl)-6-oxo-hexahydro-pyrrolo[3,2-b]pyrrol-1-carbonyl]-3-methyl-butyl}-benzamid,
N-{1-[4-(2-Amino-butyryl)-6-oxo-hexahydro-pyrrolo[3,2-b]pyrrol-1-carbonyl]-3-methyl-butyl}-4-tert-butyl-benzamid,
N-{1-[4-(2-Acetylamino-butyryl)-6-oxo-hexahydro-pyrrolo[3,2-b]pyrrol-1-carbonyl]-3-methyl-butyl}-4-tert-butyl-benzamid,
N-{1-[4-(2-Amino-3-methyl-butyryl)-6-oxo-hexahydro-pyrrolo[3,2-b]pyrrol-1-carbonyl]-3-methyl-butyl}-4-tert-butyl-benzamid,
N-{1-[4-(2-Acetylamino-3-methyl-butyryl)-6-oxo-hexahydro-pyrrolo[3,2-b]pyrrol-1-carbonyl]-3-methyl-butyl}-4-tert-butyl-benzamid,
N-{1-[4-(2-Amino-3,3-dimethyl-butyryl)-6-oxo-hexahydro-pyrrolo[3,2-b]pyrrol-1-carbonyl]-3-methyl-butyl}-4-tert-butyl-benzamid,
N-{1-[4-(2-Acetylamino-3,3-dimethyl-butyryl)-6-oxo-hexahydro-pyrrolo[3,2-b]pyrrol-1-carbonyl]-3-methyl-butyl}-4-tert-butyl-benzamid,
N-{1-[4-(2-Amino-3-methyl-pentanoyl)-6-oxo-hexahydro-pyrrolo[3,2-b]pyrrol-1-carbonyl]-3-methyl-butyl}-4-tert-butyl-benzamid,
N-{1-[4-(2-Acetylamino-3-methyl-pentanoyl)-6-oxo-hexahydro-pyrrolo[3,2-b]pyrrol-1-carbonyl]-3-methyl-butyl}-4-tert-butyl-benzamid,
4-tert-Butyl-N-[3-methyl-1-(6-oxo-4-pentanoyl-hexahydro-pyrrolo[3,2-b]pyrrol-1-carbonyl)-butyl]-benzamid,
4-tert-Butyl-N-{3-methyl-1-[4-(3-methyl-pentanoyl)-6-oxo-hexahydro-pyrrolo[3,2-b]pyrrol-1-carbonyl]-butyl}-benzamid,
4-tert-Butyl-N-{3-methyl-1-[4-(4-methyl-pentanoyl)-6-oxo-hexahydro-pyrrolo[3,2-b]pyrrol-1-carbonyl]-butyl}-benzamid,
N-{1-[4-(2-Amino-4-methyl-pentanoyl)-6-oxo-hexahydro-pyrrolo[3,2-b]pyrrol-1-carbonyl]-3-methyl-butyl}-4-tert-butyl-benzamid,
N-{1-[4-(2-Acetylamino-4-methyl-pentanoyl)-6-oxo-hexahydro-pyrrolo[3,2-b]pyrrol-1-carbonyl]-3-methyl-butyl}-4-tert-butyl-benzamid,
N-{1-[4-(2-Amino-4,4-dimethyl-pentanoyl)-6-oxo-hexahydro-pyrrolo[3,2-b]pyrrol-1-carbonyl]-3-methyl-butyl}-4-tert-butyl-benzamid,
N-{1-[4-(2-Acetylamino-4,4-dimethyl-pentanoyl)-6-oxo-hexahydro-pyrrolo[3,2-b]pyrrol-1-carbonyl]-3-methyl-butyl}-4-tert-butyl-benzamid,
N-{1-[4-(2-Amino-pentanoyl)-6-oxo-hexahydro-pyrrolo[3,2-b]pyrrol-1-carbonyl]-3-methyl-butyl}-4-tert-butyl-benzamid,
N-{1-[4-(2-Acetylamino-pentanoyl)-6-oxo-hexahydro-pyrrolo[3,2-b]pyrrol-1-carbonyl]-3-methyl-butyl}-4-tert-butyl-benzamid,
N-{1-[4-(2-Amino-5-methyl-hexanoyl)-6-oxo-hexahydro-pyrrolo[3,2-b]pyrrol-1-carbonyl]-3-methyl-butyl}-4-tert-butyl-benzamid,
N-{1-[4-(2-Amino-5-methyl-hexanoyl)-6-oxo-hexahydro-pyrrolo[3,2-b]pyrrol-1-carbonyl]-3-methyl-butyl}-4-tert-butyl-benzamid,
N-{1-[4-(2-Acetylamino-5-methyl-hexanoyl)-6-oxo-hexahydro-pyrrolo[3,2-b]pyrrol-1-carbonyl]-3-methyl-butyl}-4-tert-butyl-benzamid,
N-{1-[4-(2-Hydroxy-3-methyl-butyryl)-6-oxo-hexahydro-pyrrolo[3,2-b]pyrrol-1-carbonyl]-3-methyl-butyl}-4-tert-butyl-benzamid,
N-{1-[4-(2-Methoxy-3-methyl-butyryl)-6-oxo-hexahydro-pyrrolo[3,2-b]pyrrol-1-carbonyl]-3-methyl-butyl}-4-tert-butyl-benzamid,
4-tert-Butyl-N-{1-[4-(2-hydroxy-3,3-dimethyl-butyryl)-6-oxo-hexahydro-pyrrolo[3,2-b]pyrrol-1-carbonyl]-3-methyl-butyl}-benzamid,
4-tert-Butyl-N-{1-[4-(2-methoxy-3,3-dimethyl-butyryl)-6-oxo-hexahydro-pyrrolo[3,2-b]pyrrol-1-carbonyl]-3-methyl-butyl}-benzamid,
4-tert-Butyl-N-{1-[4-(2-hydroxy-3-methyl-pentanoyl)-6-oxo-hexahydro-pyrrolo[3,2-b]pyrrol-1-carbonyl]-3-methyl-butyl}-benzamid,
4-tert-Butyl-N-{1-[4-(2-methoxy-3-methyl-pentanoyl)-6-oxo-hexahydro-pyrrolo[3,2-b]pyrrol-1-carbonyl]-3-methyl-butyl}-benzamid,

4-tert-Butyl-N-{1-[4-(2-hydroxy-4-methyl-pentanoyl)-6-oxo-hexahydro-pyrrolo[3,2-b]pyrrol-1-carbonyl]-3-methyl-butyl}-benzamid,
4-tert-Butyl-N-{1-[4-(2-methoxy-4-methyl-pentanoyl)-6-oxo-hexahydro-pyrrolo[3,2-b]pyrrol-1-carbonyl]-3-methyl-butyl}-benzamid,
4-tert-Butyl-N-{1-[4-(2-hydroxy-4,4-dimethyl-pentanoyl)-6-oxo-hexahydro-pyrrolo[3,2-b]pyrrol-1-carbonyl]-3-methyl-butyl}-benzamid,
4-tert-Butyl-N-{1-[4-(2-methoxy-4,4-dimethyl-pentanoyl)-6-oxo-hexahydro-pyrrolo[3,2-b]pyrrol-1-carbonyl]-3-methyl-butyl}-benzamid,
4-tert-Butyl-N-{1-[4-(2-hydroxy-pentanoyl)-6-oxo-hexahydro-pyrrolo[3,2-b]pyrrol-1-carbonyl]-3-methyl-butyl}-benzamid,
4-tert-Butyl-N-{1-[4-(2-methoxy-pentanoyl)-6-oxo-hexahydro-pyrrolo[3,2-b]pyrrol-1-carbonyl]-3-methyl-butyl}-benzamid,
4-tert-Butyl-N-{1-[4-(2-hydroxy-5-methyl-hexanoyl)-6-oxo-hexahydro-pyrrolo[3,2-b]pyrrol-1-carbonyl]-3-methyl-butyl}-benzamid,
4-tert-Butyl-N-{1-[4-(2-methoxy-5-methyl-hexanoyl)-6-oxo-hexahydro-pyrrolo[3,2-b]pyrrol-1-carbonyl]-3-methyl-butyl}-benzamid,
N-[1-(4-Benzoyl-6-oxo-hexahydro-pyrrolo[3,2-b]pyrrol-1-carbonyl)-3-methyl-butyl]-4-dimethylamino-benzamid,
4-Dimethylamino-N-{3-methyl-1-[6-oxo-4-(1-oxy-pyridin-2-carbonyl)-hexahydro-pyrrolo[3,2-b]pyrrol-1-carbonyl]-butyl}-benzamid,
4-Dimethylamino-N-{3-methyl-1-[6-oxo-4-(pyridin-3-carbonyl)-hexahydro-pyrrolo[3,2-b]pyrrol-1-carbonyl]-butyl}-benzamid,
4-Dimethylamino-N-{3-methyl-1-[6-oxo-4-(1-oxy-pyridin-3-carbonyl)-hexahydro-pyrrolo[3,2-b]pyrrol-1-carbonyl]-butyl}-benzamid,
4-Dimethylamino-N-{3-methyl-1-[6-oxo-4-(pyridin-4-carbonyl)-hexahydro-pyrrolo[3,2-b]pyrrol-1-carbonyl]-butyl}-benzamid,
4-Dimethylamino-N-{3-methyl-1-[6-oxo-4-(1-oxy-pyridin-4-carbonyl)-hexahydro-pyrrolo[3,2-b]pyrrol-1-carbonyl]-butyl}-benzamid,
N-[1-(4-Benzensulfonyl-6-oxo-hexahydro-pyrrolo[3,2-b]pyrrol-1-carbonyl)-3-methyl-butyl]-4-dimethylamino-benzamid,
4-Dimethylamino-N-{3-methyl-1-[6-oxo-4-(pyridin-4-sulfonyl)-hexahydro-pyrrolo[3,2-b]pyrrol-1-carbonyl]-butyl}-benzamid,
4-Dimethylamino-N-{3-methyl-1-[6-oxo-4-(1-oxy-pyridin-4-sulfonyl)-hexahydro-pyrrolo[3,2-b]pyrrol-1-carbonyl]-butyl}-benzamid,
4-Dimethylamino-N-[3-methyl-1-[6-oxo-4-phenylacetyl-hexahydro-pyrrolo[3,2-b]pyrrol-1-carbonyl]-butyl}-benzamid,
4-[2-(4-Dimethylamino-benzoylamino)-4-methyl-pentanoyl]-3-oxo-hexahydro-pyrrolo[3,2-b]pyrrol-1-carbonsäurephenylamid,
4-[2-(4-Dimethylamino-benzoylamino)-4-methyl-pentanoyl]-3-oxo-hexahydro-pyrrolo[3,2-b]pyrrol-1-carbonsäureisobutylamid,
4-[2-(4-Dimethylamino-benzoylamino)-4-methyl-pentanoyl]-3-oxo-hexahydro-pyrrolo[3,2-b]pyrrol-1-carbonsäurephenylester,
4-[2-(4-Dimethylamino-benzoylamino)-4-methyl-pentanoyl]-3-oxo-hexahydro-pyrrolo[3,2-b]pyrrol-1-carbonsäure-isobutylester,
4-Dimethylamino-N-{3-methyl-1-[6-oxo-4-(pyrrolidin-1-carbonyl)-hexahydro-pyrrolo[3,2-b]pyrrol-1-carbonyl]-butyl}-benzamid,
4-Dimethylamino-N-{3-methyl-1-[6-oxo-4-(piperidin-1-carbonyl)-hexahydro-pyrrolo[3,2-b]pyrrol-1-carbonyl]-butyl}-benzamid,
4-Dimethylamino-N-{3-methyl-1-[4-(morpholin-1-carbonyl)-6-oxo-hexahydro-pyrrolo[3,2-b]pyrrol-1-carbonyl]-butyl}-benzamid,
4-Dimethylamino-N-{3-methyl-1-[6-oxo-4-(piperazin-1-carbonyl)-hexahydro-pyrrolo[3,2-b]pyrrol-1-carbonyl]-butyl}-benzamid,
4-Dimethylamino-N-{3-methyl-1-[4-(4-methyl-piperazin-1-carbonyl)-6-oxo-hexahydro-pyrrolo[3,2-b]pyrrol-1-carbonyl]-butyl}-benzamid,
4-Dimethylamino-N-(3-methyl-1-{4-[2-(4-methyl-piperazin-1-yl)-acetyl]-6-oxo-hexahydro-pyrrolo[3,2-b]pyrrol-1-carbonyl}-butyl)-benzamid,
4-Dimethylamino-N-{3-methyl-1-[6-oxo-4-(2-piperazin-1-yl-acetyl)-hexahydro-pyrrolo[3,2-b]-pyrrol-1-carbonyl]-butyl}-benzamid,
4-Dimethylamino-N-{1-[4-(furan-2-carbonyl)-6-oxo-hexahydro-pyrrolo[3,2-b]pyrrol-1-carbonyl]-3-methyl-butyl}-benzamid,
4-Dimethylamino-N-{1-[4-(5-chlor-furan-2-carbonyl)-6-oxo-hexahydro-pyrrolo[3,2-b]pyrrol-1-carbonyl]-3-methyl-butyl}-benzamid,
4-Dimethylamino-N-{1-[4-(thiophen-2-carbonyl)-6-oxo-hexahydro-pyrrolo[3,2-b]pyrrol-1-carbonyl]-3-methyl-butyl}-benzamid,
4-Dimethylamino-N-{1-[4-(5-chlor-thiophen-2-carbonyl)-6-oxo-hexahydro-pyrrolo[3,2-b]pyrrol-1-carbonyl]-3-methyl-butyl}-benzamid,
4-Dimethylamino-N-{1-[4-(furan-3-carbonyl)-6-oxo-hexahydro-pyrrolo[3,2-b]pyrrol-1-carbonyl]-3-methyl-butyl}-benzamid,
4-Dimethylamino-N-{1-[4-(thiophen-3-carbonyl)-6-oxo-hexahydro-pyrrolo[3,2-b]pyrrol-1-carbonyl]-3-methyl-butyl}-benzamid,
4-Dimethylamino-N-[1-(4-cyclopentancarbonyl-6-oxo-hexahydro-pyrrolo[3,2-b]pyrrol-1-carbonyl)-3-methyl-butyl]-benzamid,
4-Dimethylamino-N-[1-(4-cyclohexancarbonyl-6-oxo-hexahydro-pyrrolo[3,2-b]pyrrol-1-carbonyl)-3-methyl-butyl]-benzamid,
4-Dimethylamino-N-{1-[4-(cyclopent-3-encarbonyl)-6-oxo-hexahydro-pyrrolo[3,2-b]pyrrol-1-carbonyl]-3-methyl-butyl}-benzamid,
4-Dimethylamino-N-{1-[4-(2-cyclopentyl-acetyl)-6-oxo-hexahydro-pyrrolo[3,2-b]pyrrol-1-carbonyl]-3-methyl-butyl}-benzamid,
4-Dimethylamino-N-{1-[4-(2-cyclohexyl-acetyl)-6-oxo-hexahydro-pyrrolo[3,2-b]pyrrol-1-carbonyl]-3-methyl-butyl}-benzamid,
4-Dimethylamino-N-{3-methyl-1-[6-oxo-4-(pyrrolidin-2-carbonyl)-hexahydro-pyrrolo[3,2-b]pyrrol-1-carbonyl]-butyl}-benzamid,
N-{1-[4-(1-Acetyl-pyrrolidin-2-carbonyl)-6-oxo-hexahydro-pyrrolo[3,2-b]pyrrol-1-carbonyl]-3-methyl-butyl}-4-dimethylamino-benzamid,
4-Dimethylamino-N-{3-methyl-1-[6-oxo-4-(piperidin-2-carbonyl)-hexahydro-pyrrolo[3,2-b]pyrrol-1-carbonyl]-butyl}-benzamid,
N-1-[4-(1-Acetyl-piperidin-2-carbonyl)-6-oxo-hexahydro-pyrrolo[3,2-b]pyrrol-1-carbonyl]-3-methyl-butyl}-4-dimethylamino-benzamid,
4-Dimethylamino-N-{3-methyl-1-[6-oxo-4-(tetrahydro-furan-2-carbonyl)-hexahydro-pyrrolo[3,2-b]pyrrol-1-carbonyl]-butyl}-benzamid,
N-{1-[4-(1-Amino-cyclobutancarbonyl)-6-oxo-hexahydro-pyrrolo[3,2-b]pyrrol-1-carbonyl]-3-methyl-butyl}-4-dimethylamino-benzamid,
N-{1-[4-(1-Amino-cyclopentancarbonyl)-6-oxo-hexahydro-pyrrolo[3,2-b]pyrrol-1-carbonyl]-3-methyl-butyl}-4-dimethylamino-benzamid,
N-{-1-[4-(1-Amino-cyclohexancarbonyl)-6-oxo-hexahydro-pyrrolo[3,2-b]pyrrol-1-carbonyl]-3-methyl-butyl}-4-dimethylamino-benzamid,
N-{1-[4-(1-Acetylamino-cyclobutancarbonyl)-6-oxo-hexahydro-pyrrolo[3,2-b]pyrrol-1-carbonyl]-3-methyl-butyl}-4-dimethylamino-benzamid,
N-{1-[4-(1-Acetylamino-cyclopentancarbonyl)-6-oxo-hexahydro-pyrrolo[3,2-b]pyrrol-1-carbonyl]-3-methyl-butyl}-4-dimethylamino-benzamid,
N-{1-[4-(1-Acetylamino-cyclohexancarbonyl)-6-oxo-hexahydro-pyrrolo[3,2-b]pyrrol-1-carbonyl]-3-methyl-butyl}-4-dimethylamino-benzamid,
4-Dimethylamino-N-{1-[4-(1-hydroxy-cyclobutancarbonyl)-6-oxo-hexahydro-pyrrolo[3,2-b]pyrrol-1-carbonyl]-3-methyl-butyl}-benzamid,
4-Dimethylamino-N-{1-[4-(1-hydroxy-cyclopentancarbonyl)-6-oxo-hexahydro-pyrrolo[3,2-b]pyrrol-1-carbonyl]-3-methyl-butyl}-benzamid,
4-Dimethylamino-N-{1-[4-(1-hydroxy-cyclohexancarbonyl)-6-oxo-hexahydro-pyrrolo[3,2-b]pyrrol-1-carbonyl]-3-methyl-butyl}-benzamid,
4-Dimethylamino-N-{1-[4-(1-methoxy-cyclobutancarbonyl)-6-oxo-hexahydro-pyrrolo[3,2-b]pyrrol-1-carbonyl]-3-methyl-butyl}-benzamid,
4-Dimethylamino-N-{1-[4-(1-methoxy-cyclopentancarbonyl)-6-oxo-hexahydro-pyrrolo[3,2-b]pyrrol-1-carbonyl]-3-methyl-butyl}-benzamid,
4-Dimethylamino-N-{1-[4-(1-methoxy-cyclohexancarbonyl)-6-oxo-hexahydro-pyrrolo[3,2-b]pyrrol-1-carbonyl]-3-methyl-butyl}-benzamid,
N-{1-[4-(2-Amino-2-cyclopentyl-acetyl)-6-oxo-hexahydro-pyrrolo[3,2-b]pyrrol-1-carbonyl]-3-methyl-butyl}-4-dimethylamino-benzamid,
N-{1-[4-(2-Amino-2-cyclohexyl-acetyl)-6-oxo-hexahydro-pyrrolo[3,2-b]pyrrol-1-carbonyl]-3-methyl-butyl}-4-dimethylamino-benzamid,
N-{1-[4-(2-Acetylamino-2-cyclopentyl-acetyl)-6-oxo-hexahydro-pyrrolo[3,2-b]pyrrol-1-carbonyl]-3-methyl-butyl}-4-dimethylamino-benzamid,
N-{1-[4-(2-Acetylamino-2-cyclohexyl-acetyl)-6-oxo-hexahydro-pyrrolo[3,2-b]pyrrol-1-carbonyl]-3-methyl-butyl}-4-dimethylamino-benzamid,
N-{1-[4-(2-Acetylamino-propionyl)-6-oxo-hexahydro-pyrrolo[3,2-b]pyrrol-1-carbonyl]-3-methyl-butyl}-4-dimethylamino-benzamid,
N-{1-[4-(2-Acetylamino-acetyl)-6-oxo-hexahydro-pyrrolo[3,2-b]pyrrol-1-carbonyl]-3-methyl-butyl}-4-dimethylamino-benzamid,
N-(1-{4-[2-(Acetyl-methyl-amino)-acetyl]-6-oxo-hexahydro-pyrrolo[3,2-b]pyrrol-1-carbonyl}-3-methyl-butyl)-4-dimethylamino-benzamid,
4-Dimethylamino-N-{1-[4-(2-dimethylamino-acetyl)-6-oxo-hexahydro-pyrrolo[3,2-b]pyrrol-1-carbonyl]-3-methyl-butyl}-benzamid,
N-{1-[4-(2-Amino-butyryl)-6-oxo-hexahydro-pyrrolo[3,2-b]pyrrol-1-carbonyl]-3-methyl-butyl}-4-dimethylamino-benzamid,
N-{1-[4-(2-Acetylamino-butyryl)-6-oxo-hexahydro-pyrrolo[3,2-b]pyrrol-1-carbonyl]-3-methyl-butyl}-4-dimethylamino-benzamid,
N-{1-[4-(2-Amino-3-methyl-butyryl)-6-oxo-hexahydro-pyrrolo[3,2-b]pyrrol-1-carbonyl]-3-methyl-butyl}-4-dimethylamino-benzamid,
N-{1-[4-(2-Acetylamino-3-methyl-butyryl)-6-oxo-hexahydro-pyrrolo[3,2-b]pyrrol-1-carbonyl]-3-methyl-butyl}-4-dimethylamino-benzamid,
N-{1-[4-(2-Amino-3,3-dimethyl-butyryl)-6-oxo-hexahydro-pyrrolo[3,2-b]pyrrol-1-carbonyl]-3-methyl-butyl}-4-dimethylamino-benzamid,
N-{1-[4-(2-Acetylamino-3,3-dimethyl-butyryl)-6-oxo-hexahydro-pyrrolo[3,2-b]pyrrol-1-carbonyl]-3-methyl-butyl}-4-dimethylamino-benzamid,
N-{1-[4-(2-Amino-3-methyl-pentanoyl)-6-oxo-hexahydro-pyrrolo[3,2-b]pyrrol-1-carbonyl]-3-methyl-butyl}-4-dimethylamino-benzamid,
N-{1-[4-(2-Acetylamino-3-methyl-pentanoyl)-6-oxo-hexahydro-pyrrolo[3,2-b]pyrrol-1-carbonyl]-3-methyl-butyl}-4-dimethylamino-benzamid,
4-Dimethylamino-N-[3-methyl-1-(6-oxo-4-pentanoyl-hexahydro-pyrrolo[3,2-b]pyrrol-1-carbonyl)-butyl]-benzamid,
4-Dimethylamino-N-{3-methyl-1-[4-(3-methyl-pentanoyl)-6-oxo-hexahydro-pyrrolo[3,2-b]pyrrol-1-carbonyl]-butyl}-benzamid,
4-Dimethylamino-N-{3-methyl-1-[4-(4-methyl-pentanoyl)-6-oxo-hexahydro-pyrrolo[3,2-b]pyrrol-1-carbonyl]-butyl}-benzamid,
N-{1-[4-(2-Amino-4-methyl-pentanoyl)-6-oxo-hexahydro-pyrrolo[3,2-b]pyrrol-1-carbonyl]-3-methyl-butyl}-4-dimethylamino-benzamid,
N-{1-[4-(2-Acetylamino-4-methyl-pentanoyl)-6-oxo-hexahydro-pyrrolo[3,2-b]pyrrol-1-carbonyl]-3-methyl-butyl}-4-dimethylamino-benzamid,
N-{1-[4-(2-Amino-4,4-dimethyl-pentanoyl)-6-oxo-hexahydro-pyrrolo[3,2-b]pyrrol-1-carbonyl]-3-methyl-butyl}-4-dimethylamino-benzamid,
N-{1-[4-(2-Acetylamino-4,4-dimethyl-pentanoyl)-6-oxo-hexahydro-pyrrolo[3,2-b]pyrrol-1-carbonyl]-3-methyl-butyl}-4-dimethylamino-benzamid,
N-{1-[4-(2-Amino-pentanoyl)-6-oxo-hexahydro-pyrrolo[3,2-b]pyrrol-1-carbonyl]-3-methyl-butyl}-4-dimethylamino-benzamid,
N-{1-[4-(2-Acetylamino-pentanoyl)-6-oxo-hexahydro-pyrrolo[3,2-b]pyrrol-1-carbonyl]-3-methyl-butyl}-4-dimethylamino-benzamid,
N-{1-[4-(2-Amino-5-methyl-hexanoyl)-6-oxo-hexahydro-pyrrolo[3,2-b]pyrrol-1-carbonyl]-3-methyl-butyl}-4-dimethylamino-benzamid,
N-{1-[4-(2-Acetylamino-5-methyl-hexanoyl)-6-oxo-hexahydro-pyrrolo[3,2-b]pyrrol-1-carbonyl]-3-methyl-butyl}-4-dimethylamino-benzamid,
N-{1-[4-(Hydroxy-3-methyl-butyryl)-6-oxo-hexahydro-pyrrolo[3,2-b]pyrrol-1-carbonyl]-3-methyl-butyl}-4-dimethylamino-benzamid,
N-{1-[4-(2-Methoxy-3-methyl-butyryl)-6-oxo-hexahydro-pyrrolo[3,2-b]pyrrol-1-carbonyl]-3-methyl-butyl}-4-dimethylamino-benzamid,
4-Dimethylamino-N-{-[4-(2-hydroxy-3,3-dimethyl-butyryl)-6-oxo-hexahydro-pyrrolo[3,2-b]pyrrol-1-carbonyl]-3-methyl-butyl}-benzamid,
4-Dimethylamino-N-{1-[4-(2-methoxy-3,3-dimethyl-butyryl)-6-oxo-hexahydro-pyrrolo[3,2-b]pyrrol-1-carbonyl]-3-methyl-butyl}-benzamid,
4-Dimethylamino-N-{1-[4-(2-hydroxy-3-methyl-pentanoyl)-6-oxo-hexahydro-pyrrolo[3,2-b]pyrrol-1-carbonyl]-3-methyl-butyl}-benzamid,

4-Dimethylamino-N-{-[4-(2-methoxy-3-methyl-pentanoyl)-6-oxo-hexahydro-pyrrolo[3,2-b]pyrrol-1-carbonyl]-3-methyl-butyl}-benzamid,
4-Dimethylamino-N-{1-[4-(2-hydroxy-4-methyl-pentanoyl)-6-oxo-hexahydro-pyrrolo[3,2-b]pyrrol-1-carbonyl]-3-methyl-butyl}-benzamid,
4-Dimethylamino-N-{1-[4-(2-methoxy-4-methyl-pentanoyl)-6-oxo-hexahydro-pyrrolo[3,2-b]pyrrol-1-carbonyl]-3-methyl-butyl}-benzamid,
4-Dimethylamino-N-{1-[4-(2-hydroxy-4,4-dimethyl-pentanoyl)-6-oxo-hexahydro-pyrrolo[3,2-b]pyrrol-1-carbonyl]-3-methyl-butyl}-benzamid,
4-Dimethylamino-N-{1-[4-(2-methoxy-4,4-dimethyl-pentanoyl)-6-oxo-hexahydro-pyrrolo[3,2-b]pyrrol-1-carbonyl]-3-methyl-butyl}-benzamid,
4-Dimethylamino-N-{1-[4-(2-hydroxy-pentanoyl)-6-oxo-hexahydro-pyrrolo[3,2-b]pyrrol-1-carbonyl]-3-methyl-butyl}-benzamid,
4-Dimethylamino-N-{1-[4-(2-methoxy-pentanoyl)-6-oxo-hexahydro-pyrrolo[3,2-b]pyrrol-1-carbonyl]-3-methyl-butyl}-benzamid,
4-Dimethylamino-N-{1-[4-(2-hydroxy-5-methyl-hexanoyl)-6-oxo-hexahydro-pyrrolo[3,2-b]pyrrol-1-carbonyl]-3-methyl-butyl}-benzamid,
4-Dimethylamino-N-{1-[4-(2-methoxy-5-methyl-hexanoyl)-6-oxo-hexahydro-pyrrolo[3,2-b]pyrrol-1-carbonyl]-3-methyl-butyl}-benzamid,
N-[1-(4-Benzoyl-6-oxo-hexahydro-pyrrolo[3,2-b]pyrrol-1-carbonyl)-3-methyl-butyl]-4-thiophen-2-yl-benzamid,
N-{3-Methyl-1-[6-oxo-4-(1-oxy-pyridin-2-carbonyl)-hexahydro-pyrrolo[3,2-b]pyrrol-1-carbonyl]-butyl}-4-thiophen-2-yl-benzamid,
N-{3-Methyl-1-[6-oxo-4-(pyridin-3-carbonyl)-hexahydro-pyrrolo[3,2-b]pyrrol-1-carbonyl]-butyl}-4-thiophen-2-yl-benzamid,
N-{3-Methyl-1-[6-oxo-4-(1-oxy-pyridin-3-carbonyl)-hexahydro-pyrrolo[3,2-b]pyrrol-1-carbonyl]-butyl}-4-thiophen-2-yl-benzamid,
N-{3-Methyl-1-[6-oxo-4-(pyridin-4-carbonyl)-hexahydro-pyrrolo[3,2-b]pyrrol-1-carbonyl]-butyl}-4-thiophen-2-yl-benzamid,
N-{3-Methyl-1-[6-oxo-4-(1-oxy-pyridin-4-carbonyl)-hexahydro-pyrrolo[3,2-b]pyrrol-1-carbonyl]-butyl}-4-thiophen-2-yl-benzamid,
N[1-(4-Benzensulfonyl-6-oxo-hexahydro-pyrrolo[3,2-b]pyrrol-1-carbonyl)-3-methyl-butyl]-4-thiophen-2-yl-benzamid,
N-{3-Methyl-1-[6-oxo-4-(pyridin-4-sulfonyl)-hexahydro-pyrrolo[3,2-b]pyrrol-1-carbonyl]-butyl}-4-thiophen-2-yl-benzamid,
N-{3-Methyl-1-[6-oxo-4-(1-oxy-pyridin-4-sulfonyl)-hexahydro-pyrrolo[3,2-b]pyrrol-1-carbonyl]-butyl}-4-thiophen-2-yl-benzamid,
N-[3-Methyl-1-(6-oxo-4-phenylacetyl-hexahydro-pyrrolo[3,2-b]pyrrol-1-carbonyl)-butyl]-4-thiophen-2-yl-benzamid,
4-[4-Methyl-2-(4-thiophen-2-yl-benzoylamino)-pentanoyl]-3-oxo-hexahydro-pyrrolo[3,2-b]pyrrol-1-carbonsäure-phenylamid,
4-[4-Methyl-2-(4-thiophen-2-yl-benzoylamino)-pentanoyl]-3-oxo-hexahydro-pyrrolo[3,2-b]pyrrol-1-carbonsäure-isobutyl-amid,
4-[4-Methyl-2-(4-thiophen-2-yl-benzoylamino)-pentanoyl]-3-oxo-hexahydro-pyrrolo[3,2-b]pyrrol-1-carbonsäure-phenylester,
4-[4-Methyl-2-(4-thiophen-2-yl-benzoylamino)-pentanoyl]-3-oxo-hexahydro-pyrrolo[3,2-b]pyrrol-1-carbonsäure-isobutylester,
N-{3-Methyl-1-[6-oxo-4-(pyrrolidin-1-carbonyl)-hexahydro-pyrrolo[3,2-b]pyrrol-1-carbonyl]-butyl}-4-thiophen-2-yl-benzamid,
N-{3-Methyl-1-[6-oxo-4-(piperidin-1-carbonyl)-hexahydro-pyrrolo[3,2-b]pyrrol-1-carbonyl]-butyl}-4-thiophen-2-yl-benzamid,
N-{3-Methyl-1-[4-(morpholin-4-carbonyl)-6-oxo-hexahydro-pyrrolo[3,2-b]pyrrol-1-carbonyl]-butyl}-4-thiophen-2-yl-benzamid,
N-{3-Methyl-1-[6-oxo-4-(piperazin-1-carbonyl)-hexahydro-pyrrolo[3,2-b]pyrrol-1-carbonyl]-butyl}-4-thiophen-2-yl-benzamid,
N-{3-Methyl-1-[4-(4-methyl-piperazin-1-carbonyl)-6-oxo-hexahydro-pyrrolo[3,2-b]pyrrol-1-carbonyl]-butyl}-4-thiophen-2-yl-benzamid,
N-(3-Methyl-1-{-4-[2-(4-methyl-piperazin-1-yl)-acetyl]-6-oxo-hexahydro-pyrrolo[3,2-b]pyrrol-1-carbonyl}-butyl)-4-thiophen-2-yl-benzamid,
N-{3-Methyl-1-[6-oxo-4-(2-piperazin-1-yl-acetyl)-hexahydro-pyrrolo[3,2-b]pyrrol-1-carbonyl]-butyl}-4-thiophen-2-yl-benzamid,
N-{1-[4-(Furan-2-carbonyl)-6-oxo-hexahydro-pyrrolo[3,2-b]pyrrol-1-carbonyl]-3-methyl-butyl}-4-thiophen-2-yl-benzamid,
N-{1-[-(5-Chlor-furan-2-carbonyl)-6-oxo-hexahydro-pyrrolo[3,2-b]pyrrol-1-carbonyl]-3-methyl-butyl}-4-thiophen-2-yl-benzamid,
N-{1-[4-(Thiophen-2-carbonyl)-6-oxo-hexahydro-pyrrolo[3,2-b]pyrrol-1-carbonyl]-3-methyl-butyl}-4-thiophen-2-yl-benzamid,
N-{1-[4-(5-Chlor-thiophen-2-carbonyl)-6-oxo-hexahydro-pyrrolo[3,2-b]pyrrol-1-carbonyl]-3-methyl-butyl}-4-thiophen-2-yl-benzamid,
N-{1-[4-(Furan-3-carbonyl)-6-oxo-hexahydro-pyrrolo[3,2-b]pyrrol-1-carbonyl]-3-methyl-butyl}-4-thiophen-2-yl-benzamid,
N-{1-[4-(Thiophen-3-carbonyl)-6-oxo-hexahydro-pyrrolo[3,2-b]pyrrol-1-carbonyl]-3-methyl-butyl}-4-thiophen-2-yl-benzamid,
N-[1-(4-Cyclopentancarbonyl-6-oxo-hexahydro-pyrrolo[3,2-b]pyrrol-1-carbonyl)-3-methyl-butyl]-4-thiophen-2-yl-benzamid,
N-{1-[4-(Cyclopent-3-encarbonyl)-6-oxo-hexahydro-pyrrolo[3,2-b]pyrrol-1-carbonyl]-3-methyl-butyl}-4-thiophen-2-yl-benzamid,
N-{1-[4-(2-Cyclopentyl-acetyl)-6-oxo-hexahydro-pyrrolo[3,2-b]pyrrol-1-carbonyl]-3-methyl-butyl}-4-thiophen-2-yl-benzamid,
N-{1-[4-(2-Cyclohexyl-acetyl)-6-oxo-hexahydro-pyrrolo[3,2-b]pyrrol-1-carbonyl]-3-methyl-butyl}-4-thiophen-2-yl-benzamid,
N-{3-Methyl-1-[6-oxo-4-(pyrrolidin-2-carbonyl)-hexahydro-pyrrolo[3,2-b]pyrrol-1-carbonyl]-butyl}-4-thiophen-2-yl-benzamid,
N-{1-[-(1-Acetyl-pyrrolidin-2-carbonyl)-6-oxo-hexahydro-pyrrolo[3,2-b]pyrrol-1-carbonyl]-3-methyl-butyl}-4-thiophen-2-yl-benzamid,
N-{3-Methyl-1-[6-oxo-4-(piperidin-2-carbonyl)-hexahydro-pyrrolo[3,2-b]pyrrol-1-carbonyl]-butyl}-4-thiophen-2-yl-benzamid,
N-{1-[4-(1-Acetyl-piperidin-2-carbonyl)-6-oxo-hexahydro-pyrrolo[3,2-b]pyrrol-1-carbonyl]-3-methyl-butyl}-4-thiophen-2-yl-benzamid,
N-{3-Methyl-1-[6-oxo-4-(tetrahydro-furan-2-carbonyl)-hexahydro-pyrrolo[3,2-b]pyrrol-1-carbonyl]-butyl}-4-thiophen-2-yl-benzamid,
N-{1-[4-(1-Amino-cyclobutancarbonyl)-6-oxo-hexahydro-pyrrolo[3,2-b]pyrrol-1-carbonyl]-3-methyl-butyl}-4-thiophen-2-yl-benzamid,
N-{1-[4-(1-Amino-cyclopentancarbonyl)-6-oxo-hexahydro-pyrrolo[3,2-b]pyrrol-1-carbonyl]-3-methyl-butyl}-4-thiophen-2-yl-benzamid,
N-{1-[4-(1-Amino-cyclohexancarbonyl)-6-oxo-hexahydro-pyrrolo[3,2-b]pyrrol-1-carbonyl]-3-methyl-butyl}-4-thiophen-2-yl-benzamid,
N-{1-[4-(1-Acetylamino-cyclobutancarbonyl)-6-oxo-hexahydro-pyrrolo[3,2-b]pyrrol-1-carbonyl]-3-methyl-butyl}-4-thiophen-2-yl-benzamid,
N-{1-[4-(1-Acetylamino-cyclopentancarbonyl)-6-oxo-hexahydro-pyrrolo[3,2-b]pyrrol-1-carbonyl]-3-methyl-butyl}-4-thiophen-2-yl-benzamid,
N-{1-[4-(1-Acetylamino-cyclohexancarbonyl)-6-oxo-hexahydro-pyrrolo[3,2-b]pyrrol-1-carbonyl]-3-methyl-butyl}-4-thiophen-2-yl-benzamid,
N-{1-[4-(1-Hydroxy-cyclobutancarbonyl)-6-oxo-hexahydro-pyrrolo[3,2-b]pyrrol-1-carbonyl]-3-methyl-butyl}-4-thiophen-2-yl-benzamid,
N-{1-[4-(1-Hydroxy-cyclopentancarbonyl)-6-oxo-hexahydro-pyrrolo[3,2-b]pyrrol-1-carbonyl]-3-methyl-butyl}-4-thiophen-2-yl-benzamid,
N-{1-[4-(1-Hydroxy-cyclohexancarbonyl)-6-oxo-hexahydro-pyrrolo[3,2-b]pyrrol-1-carbonyl]-3-methyl-butyl}-4-thiophen-2-yl-benzamid,
N-{1-[4-(1-Methoxy-cyclobutancarbonyl)-6-oxo-hexahydro-pyrrolo[3,2-b]pyrrol-1-carbonyl]-3-methyl-butyl}-4-thiophen-2-yl-benzamid,
N-{1-[4-(1Methoxy-cydopentancarbonyl)-6-oxo-hexahydro-pyrrolo[3,2-b]pyrrol-1-carbonyl]-3-methyl-butyl}-4-thiophen-2-yl-benzamid,
N-{1-[4-(1-Methoxy-cyclohexancarbonyl)-6-oxo-hexahydro-pyrrolo[3,2-b]pyrrol-1-carbonyl]-3-methyl-butyl}-4-thiophen-2-yl-benzamid,
N-{1-[4-(2-Amino-2-cyclopentyl-acetyl)-6-oxo-hexahydro-pyrrolo[3,2-b]pyrrol-1-carbonyl]-3-methyl-butyl}-4-thiophen-2-yl-benzamid,
N-{1-[4-(2-Amino-cyclohexyl-acetyl)-6-oxo-hexahydro-pyrrolo[3,2-b]pyrrol-1-carbonyl]-3-methyl-butyl}-4-thiophen-2-yl-benzamid,
N-{1-[4-(2-Acetylamino-2-cyclopentyl-acetyl)-6-oxo-hexahydro-pyrrolo[3,2-b]pyrrol-1-carbonyl]-3-methyl-butyl}-4-thiophen-2-yl-benzamid,
N-{1-[4-(2-Acetylamino-2-cyclohexyl-acetyl)-6-oxo-hexahydro-pyrrolo[3,2-b]pyrrol-1-carbonyl]-3-methyl-butyl}-4-thiophen-2-yl-benzamid,
N-{1-[4-(2-Acetylamino-propionyl)-6-oxo-hexahydro-pyrrolo[3,2-b]pyrrol-1-carbonyl]-3-methyl-butyl}-4-thiophen-2-yl-benzamid,
N-{1-[4-(2-Acetylamino-acetyl)-6-oxo-hexahydro-pyrrolo[3,2-b]pyrrol-1-carbonyl]-3-methyl-butyl}-4-thiophen-2-yl-benzamid,
N-(1-{4-[2-(Acetyl-methyl-amino)-acetyl]-6-oxo-hexahydro-pyrrolo[3,2-b]pyrrol-1-carbonyl]-3-methyl-butyl}-4-thiophen-2-yl-benzamid,
N-{1-[4-(2-Dimethylamino-acetyl)-6-oxo-hexahydro-pyrrolo[3,2-b]pyrrol-1-carbonyl]-3-methyl-butyl}-4-thiophen-2-yl-benzamid,
N-{1-[4-(2-Amino-butyryl)-6-oxo-hexahydro-pyrrolo[3,2-b]pyrrol-1-carbonyl]-3-methyl-butyl}-4-thiophen-2-yl-benzamid,
N-{1-[4-(2-Acetylamino-butyryl)-6-oxo-hexahydro-pyrrolo[3,2-b]pyrrol-1-carbonyl]-3-methyl-butyl}-4-thiophen-2-yl-benzamid,
N-{1-[4-(2-Amino-3-methyl-butyryl)-6-oxo-hexahydro-pyrrolo[3,2-b]pyrrol-1-carbonyl]-3-methyl-butyl}-4-thiophen-2-yl-benzamid,
N-{1-[4-(2-Acetylamino-3-methyl-butyryl)-6-oxo-hexahydro-pyrrolo[3,2-b]pyrrol-1-carbonyl]-3-methyl-butyl}-4-thiophen-2-yl-benzamid,
N-{1-[4-(2-Amino-3,3-dimethylbutyryl)-6-oxo-hexahydro-pyrrolo[3,2-b]pyrrol-1-carbonyl]-3-methyl-butyl}-4-thiophen-2-yl-benzamid,
N-{1-[4-(2-Acetylamino-3,3-dimethyl-butyryl)-6-oxo-hexahydro-pyrrolo[3,2-b]pyrrol-1-carbonyl]-3-methyl-butyl}-4-thiophen-2-yl-benzamid,
N-{1-[4-(2-Amino-3-methyl-pentanoyl)-6-oxo-hexahydro-pyrrolo[3,2-b]pyrrol-1-carbonyl]-3-methyl-butyl}-4-thiophen-2-yl-benzamid,
N-{1-[4-(2-Acetylamino-3-methyl-pentanoyl)-6-oxo-hexahydro-pyrrolo[3,2-b]pyrrol-1-carbonyl]-3-methyl-butyl}-4-thiophen-2-yl-benzamid,
N-[3-Methyl-1-(6-oxo-4-pentanoyl-hexahydro-pyrrolo[3,2-b]pyrrol-1-carbonyl)-butyl]-4-thiophen-2-yl-benzamid,
N-{3-Methyl-1-[4-(3-methyl-pentanoyl)-6-oxo-hexahydro-pyrrolo[3,2-b]pyrrol-1-carbonyl]-butyl}-4-thiophen-2-yl-benzamid,
N-{3-Methyl-1-[4-(4-methyl-pentanoyl)-6-oxo-hexahydro-pyrrolo[3,2-b]pyrrol-1-carbonyl]-butyl}-4-thiophen-2-yl-benzamid,
N-{1-[4-(2-Amino-4-methyl-pentanoyl)-6-oxo-hexahydro-pyrrolo[3,2-b]pyrrol-1-carbonyl]-3-methyl-butyl}-4-thiophen-2-yl-benzamid,
N-{1-[4-(2-Acetylamino-4-methyl-pentanoyl)-6-oxo-hexahydro-pyrrolo[3,2-b]pyrrol-1-carbonyl]-3-methyl-butyl}-4-thiophen-2-yl-benzamid,
N-{1-[4-(2-Amino-4,4-dimethyl-pentanoyl)-6-oxo-hexahydro-pyrrolo[3,2-b]pyrrol-1-carbonyl]-3-methyl-butyl}-4-thiophen-2-yl-benzamid,
N-{1-[4-(2-Amino-pentanoyl)-6-oxo-hexahydro-pyrrolo[3,2-b]pyrrol-1-carbonyl]-3-methyl-butyl}-4-thiophen-2-yl-benzamid,
N-{1-[4-(2-Acetylamino-pentanoyl)-6-oxo-hexahydro-pyrrolo[3,2-b]pyrrol-1-carbonyl]-3-methyl-butyl}-4-thiophen-2-yl-benzamid,
N-{1-[4-(2-Amino-5-methyl-hexanoyl)-6-oxo-hexahydro-pyrrolo[3,2-b]pyrrol-1-carbonyl]-3-methyl-butyl}-4-thiophen-2-yl-benzamid,
N-{1-[4-(2-Acetylamino-5-methyl-hexanoyl)-6-oxo-hexahydro-pyrrolo[3,2-b]pyrrol-1-carbonyl]-3-methyl-butyl}-4-thiophen-2-yl-benzamid,
N-{1-[4-(2-Hydroxy-3-methyl-butyryl)-6-oxo-hexahydro-pyrrolo[3,2-b]pyrrol-1-carbonyl]-3-methyl-butyl}-4-thiophen-2-yl-benzamid,
N-{1-[4-(2-Methoxy-3-methyl-butyryl)-6-oxo-hexahydro-pyrrolo[3,2-b]pyrrol-1-carbonyl]-3-methyl-butyl}-4-thiophen-2-yl-benzamid,
N-{1-[4-(2-Hydroxy-3,3-dimethyl-butyryl)-6-oxo-hexahydro-pyrrolo[3,2-b]pyrrol-1-carbonyl]-3-methyl-butyl}-4-thiophen-2-yl-benzamid,
N-{1-[4-(2-Methoxy-3,3-dimethylbutyryl)-6-oxo-hexahydro-pyrrolo[3,2-b]pyrrol-1-carbonyl]-3-methyl-butyl}-4-thiophen-2-yl-benzamid,
N-{1-[4-(2-Hydroxy-3-methyl-pentanoyl)-6-oxo-hexahydro-pyrrolo[3,2-b]pyrrol-1-carbonyl]-3-methyl-butyl}-4-thiophen-2-yl-benzamid,
N-{1-[4-(2-Methoxy-3-methyl-pentanoyl)-6-oxo-hexahydro-pyrrolo[3,2-b]pyrrol-1-carbonyl]-3-methyl-butyl}-4-thiophen-2-yl-benzamid,
N-{1-[4-(2-Hydroxy-4-methyl-pentanoyl)-6-oxo-hexahydro-pyrrolo[3,2-b]pyrrol-1-carbonyl]-3-methyl-butyl}-4-thiophen-2-yl-benzamid,
N-{1-[4-(2-Methoxy-4-methyl-pentanoyl)-6-oxo-hexahydro-pyrrolo[3,2-b]pyrrol-1-carbonyl]-3-methyl-butyl}-4-thiophen-2-yl-benzamid,
N-{1-[4-(2-Hydroxy-4,4-dimethyl-pentanoyl)-6-oxo-hexahydro-pyrrolo[3,2-b]pyrrol-1-carbonyl]-3-methyl-butyl}-4-thiophen-2-yl-benzamid,
N-{1-[4-(2-Methoxy-4,4-dimethyl-pentanoyl)-6-oxo-hexahydro-pyrrolo[3,2-b]pyrrol-1-carbonyl]-3-methyl-butyl}-4-thiophen-2-yl-benzamid,
N-{1-[4-(2-hydroxy-pentanoyl)-6-oxo-hexahydro-pyrrolo[3,2-b]pyrrol-1-carbonyl]-3-methyl-butyl}-4-thiophen-2-yl-benzamid,
N-{1-[4-(2-Methoxy-pentanoyl)-6-oxo-hexahydro-pyrrolo[3,2-b]pyrrol-1-carbonyl]-3-methyl-butyl}-4-thiophen-2-yl-benzamid,
N-{1-[4-(2-Hydroxy-5-methyl-hexanoyl)-6-oxo-hexahydro-pyrrolo[3,2-b]pyrrol-1-carbonyl]-3-methyl-butyl}-4-thiophen-2-yl-benzamid,
N-1-[4-(2-Methoxy-5-methyl-hexanoyl)-6-oxo-hexahydro-pyrrolo[3,2-b]pyrrol-1-carbonyl]-3-methyl-butyl}-4-thiophen-2-yl-benzamid,
5-Phenyl-thiophen-2-carbonsäure-[1-(4-Benzoyl-6-oxo-hexahydro-pyrrolo[3,2-b]pyrrol-1-carbonyl)-3-methyl-butyl]-amid,
5-Phenyl-thiophen-2-carbonsäure-{3-methyl-1-[6-oxo-4-(pyridin-2-carbonyl)-hexahydro-pyrrolo[3,2-b]pyrrol-1-carbonyl]-butyl}-amid,
5-Phenyl-thiophen-2-carbonsäure-{3-methyl-1-[6-oxo-4-(1-oxy-pyridin-2-carbonyl)-hexahydro-pyrrolo[3,2-b]pyrrol-1-carbonyl]-butyl}-amid,
5-Phenyl-thiophen-2-carbonsäure-{3-methyl-1-[6-oxo-4-(pyridin-3-carbonyl)-hexahydro-pyrrolo[3,2-b]pyrrol-1-carbonyl]-butyl}-amid,
5-Phenyl-thiophen-2-carbonsäure-{3-methyl-1-[6-oxo-4-(1-oxy-pyridin-3-carbonyl)-hexahydro-pyrrolo[3,2-b]pyrrol-1-carbonyl]-butyl}-amid,
5-Phenyl-thiophen-2-carbonsäure-{3-methyl-1-[6-oxo-4-(pyridin-4-carbonyl)-hexahydro-pyrrolo[3,2-b]pyrrol-1-carbonyl]-butyl}-amid,
5-Phenyl-thiophen-2-carbonsäure-{3-methyl-1-[6-oxo-4-(1-oxy-pyridin-4-carbonyl)-hexahydro-pyrrolo[3,2-b]pyrrol-1-carbonyl]-butyl}-amid,
5-Phenyl-thiophen-2-carbonsäure-[1-(4-benzensulfonyl-6-oxo-hexahydro-pyrrolo[3,2-b]pyrrol-1-carbonyl)-3-methyl-butyl]-amid,
5-Phenyl-thiophen-2-carbonsäure-{3-methyl-1-[6-oxo-4-(pyridin-2-sulfonyl)-hexahydro-pyrrolo[3,2-b]pyrrol-1-carbonyl]-butyl}-amid,
5-Phenyl-thiophen-2-carbonsäure-{3-methyl-1-[6-oxo-4-(1-oxy-pyridin-2-sulfonyl)-hexahydro-pyrrolo[3,2-b]pyrrol-1-carbonyl]-butyl}-amid,
5-Phenyl-thiophen-2-carbonsäure-{3-methyl-1-[6-oxo-4-(pyridin-3-sulfonyl)-hexahydro-pyrrolo[3,2-b]pyrrol-1-carbonyl]-butyl}-amid,
5-Phenyl-thiophen-2-carbonsäure-{3-methyl-1-[6-oxo-4-(1-oxy-pyridin-3-sulfonyl)-hexahydro-pyrrolo[3,2-b]pyrrol-1-carbonyl]-butyl}-amid,
5-Phenyl-thiophen-2-carbonsäure-{3-methyl-1-[6-oxo-4-(pyridin-4-sulfonyl)-hexahydro-pyrrolo[3,2-b]pyrrol-1-carbonyl]-butyl}-amid,
5-Phenyl-thiophen-2-carbonsäure-{3-methyl-1-[6-oxo-4-(1-oxy-pyridin-4-sulfonyl)-hexahydro-pyrrolo[3,2-b]pyrrol-1-carbonyl]-butyl}-amid,
5-Phenyl-thiophen-2-carbonsäure-[3-methyl-1-(6-oxo-4-phenylacetyl-hexahydro-pyrrolo[3,2-b]pyrrol-1-carbonyl)-butyl]-amid,
4-{4-Methyl-2-[(5-phenyl-thiophen-2-carbonyl)-amino]-pentanoyl}-3-oxo-hexahydro-pyrrolo[3,2-b]pyrrol-1-carbonsäure-phenylamid,
4-{4-Methyl-2-[(5-phenyl-thiophen-2-carbonyl)-amino]-pentanoyl}-3-oxo-hexahydro-pyrrolo[3,2-b]pyrrol-1-carbonsäure-isobutyl-amid,
4-{4-Methyl-2-[(5-phenyl-thiophen-2-carbonyl)-amino]-pentanoyl}-3-oxo-hexahydro-pyrrolo[3,2-b]pyrrol-1-carbonsäurephenylester,
4-{4-Methyl-2-[(5-phenyl-thiophen-2-carbonyl)-amino]-pentanoyl}-3-oxo-hexahydro-pyrrolo[3,2-b]pyrrol-1-carbonsäureisobutylester,
5-Phenyl-thiophen-2-carbonsäure-{3-methyl-1-[6-oxo-4-(pyrrolidin-1-carbonyl)-hexahydro-pyrrolo[3,2-b]pyrrol-1-carbonyl]-butyl}-amid,
5-Phenyl-thiophen-2-carbonsäure-{3-methyl-1-[6-oxo-4-(piperidin-1-carbonyl)-hexahydro-pyrrolo[3,2-b]pyrrol-1-carbonyl]-butyl}-amid,
5-Phenyl-thiophen-2-carbonsäure-{3-methyl-1-[4-(morpholine-4-carbonyl)-6-oxo-hexahydro-pyrrolo[3,2-b]pyrrol-1-carbonyl]-butyl}-amid,
5-Phenyl-thiophen-2-carbonsäure-{3-methyl-1-[6-oxo-4-(piperazin-1-carbonyl)-hexahydro-pyrrolo[3,2-b]pyrrol-1-carbonyl]-butyl}-amid,
5-Phenyl-thiophen-2-carbonsäure-{3-methyl-1-[4-(4-methyl-piperazin-1-carbonyl)-6-oxo-hexahydro-pyrrolo[3,2-b]pyrrol-1-carbonyl]-butyl} -amid,
5-Phenyl-thiophen-2-carbonsäure-(3-methyl-1-{4-[2-(4-methyl-piperazin-1-yl)-acetyl]-6-oxo-hexahydro-pyrrolo[3,2-b]pyrrol-1-carbonyl}-butyl)-amid,
5-Phenyl-thiophen-2-carbonsäure-{3-methyl-1-[6-oxo-4-(2-piperazin-1-yl-acetyl)-hexahydro-pyrrolo[3,2-b]pyrrol-1-carbonyl]-butyl}-amid,
5-Phenyl-thiophen-2-carbonsäure-{1-[4-(furan-2-carbonyl)-6-oxo-hexahydro-pyrrolo[3,2-b]pyrrol-1-carbonyl]-3-methyl-butyl}-amid,
5-Phenyl-thiophen-2-carbonsäure{1-[4-(5-chlor-furan-2-carbonyl)-6-oxo-hexahydro-pyrrolo[3,2-b]pyrrol-1-carbonyl]-3-methyl-butyl}-amid,
5-Phenyl-thiophen-2-carbonsäure-{1-[-(thiophen-2-carbonyl)-6-oxo-hexahydro-pyrrolo[3,2-b]pyrrol-1-carbonyl]-3-methyl-butyl}-amid,
5-Phenyl-thiophen-2-carbonsäure-{-[4-(5-chlor-thiophen-2-carbonyl)-6-oxo-hexahydro-pyrrolo[3,2-b]pyrrol-1-carbonyl]-3-methyl-butyl}-amid,
5-Phenyl-thiophen-2-carbonsäure-{-[4-(furan-3-carbonyl)-6-oxo-hexahydro-pyrrolo[3,2-b]pyrrol-1-carbonyl]-3-methyl-butyl}-amid,
5-Phenyl-thiophen-2-carbonsäure-{1-[4-(thiophen-3-carbonyl)-6-oxo-hexahydro-pyrrolo[3,2-b]pyrrol-1-carbonyl]-3-methyl-butyl}-amid,
5-Phenyl-thiophen-2-carbonsäure-[1-(4-cyclopentancarbonyl-6-oxo-hexahydro-pyrrolo[3,2-b]pyrrol-1-carbonyl)-3-methyl-butyl]-amid,
5-Phenyl-thiophen-2-carbonsäure-[1-(4-cyclohexancarbonyl-6-oxo-hexahydro-pyrrolo[3,2-b]pyrrol-1-carbonyl)-3-methyl-butyl]-amid,
5-Phenyl-thiophen-2-carbonsäure-{1-[4-(cyclopent-3-encarbonyl)-6-oxo-hexahydro-pyrrolo[3,2-b]pyrrol-1-carbonyl]-3-methyl-butyl}-amid,
5-Phenyl-thiophen-2-carbonsäuregl-[4-(2-cyclopentyl-acetyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-cyclohexyl-acetyl)-6-oxo-hexahydro-pyrrolo[3,2-b]pyrrol-1-carbonyl]-3-methyl-butyl}-amid,
5-Phenyl-thiophen-2-carbonsäure-{3-methyl-1-[6-oxo-4-(pyrrolidin-2-carbonyl)-hexahydro-pyrrolo[3,2-b]pyrrol-1-carbonyl]-butyl}-amid,
5-Phenyl-thiophen-2-carbonsäure-{1-[4-(1-acetyl-pyrrolidin-2-carbonyl)-6-oxo-hexahydro-pyrrolo[3,2-b]pyrrol-1-carbonyl]-3-methyl-butyl}-amid,
5-Phenyl-thiophen-2-carbonsäure-{3-methyl-1-[6-oxo-4-(piperidin-2-carbonyl)-hexahydro-pyrrolo[3,2-b]pyrrol-1-carbonyl]-butyl}-amid,
5-Phenyl-thiophen-2-carbonsäure-{1-[4-(1-acetyl-piperidin-2-carbonyl)-6-oxo-hexahydro-pyrrolo[3,2-b]pyrrol-1-c arbonyl]-3-methyl-butyl}-amid,
5-Phenyl-thiophen-2-carbonsäure-{3-methyl-1-[6-oxo-4-(tetrahydro-furan-2-carbonyl)-hexahydro-pyrrolo[3,2-b]pyrrol-1-carbonyl]-butyl}-amid,
5-Phenyl-thiophen-2-carbonsäure-{1-[4-(1-amino-cyclobutancarbonyl)-6-oxo-hexahydro-pyrrolo[3,2-b]pyrrol-1-carbonyl]-3-methyl-butyl}-amid,
5-Phenyl-thiophen-2-carbonsäure-{1-[4-(1-amino-cyclopentancarbonyl)-6-oxo-hexahydro-pyrrolo[3,2-b]pyrrol-1-carbonyl]-3-methyl-butyl}-amid,
5-Phenyl-thiophen-2-carbonsäure-{1-[4-(1-amino-cyclohexancarbonyl)-6-oxo-hexahydro-pyrrolo[3,2-b]pyrrol-1-carbonyl]-3-methyl-butyl}-amid,
5-Phenyl-thiophen-2-carbonsäure-{1-[4-(1-acetylamino-cyclobutancarbonyl)-6-oxo-hexahydro-pyrrolo[3,2-b]pyrrol-1-carbonyl]-3-methyl-butyl}-amid,
5-Phenyl-thiophen-2-carbonsäure-{1-[4-(1-acetylamino-cyclopentancarbonyl)-6-oxo-hexahydro-pyrrolo[3,2-b]pyrrol-1-carbonyl]-3-methyl-butyl}-amid,
5-Phenyl-thiophen-2-carbonsäure-{1-[4-(1-acetylamino-cyclohexancarbonyl)-6-oxo-hexahydro-pyrrolo[3,2-b]pyrrol-1-carbonyl]-3-methyl-butyl}-amid,
5-Phenyl-thiophen-2-carbonsäure-{1-[4-(1-hydroxy-cyclobutancarbonyl)-6-oxo-hexahydro-pyrrolo[3,2-b]pyrrol-1-carbonyl]-3-methyl-butyl}-amid,
5-Phenyl-thiophen-2-carbonsäure-{1-[4-(1-hydroxy-cyclopentancarbonyl)-6-oxo-hexahydro-pyrrolo[3,2-b]pyrrol-1-carbonyl]-3-methyl-butyl}-amid,
5-Phenyl-thiophen-2-carbonsäure-{1-[4-(1-hydroxy-cyclohexancarbonyl)-6-oxo-hexahydro-pyrrolo[3,2-b]pyrrol-1-carbonyl]-3-methyl-butyl}-amid,
5-Phenyl-thiophen-2-carbonsäure-{1-[4-(1-methoxy-cyclobutancarbonyl)-6-oxo-hexahydro-pyrrolo[3,2-b]pyrrol-1-carbonyl]-3-methyl-butyl}-amid,
5-Phenyl-thiophen-2-carbonsäure-{1-[4-(1-methoxy-cyclopentancarbonyl)-6-oxo-hexahydro-pyrrolo[3,2-b]pyrrol-1-carbonyl]-3-methyl-butyl}-amid,
5-Phenyl-thiophen-2-carbonsäure-{1-[4-(1-methoxy-cyclohexancarbonyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-amino-2-cyclopentyl-acetyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-amino-2-cyclohexyl-acetyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-acetylamino-2-cyclopentyl-acetyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-acetylamino-2-cyclohexyl-acetyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-acetylamino-propionyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-acetylamino-acetyl)-6-oxo-hexahydro-pyrrolo[3,2-b]pyrrol-1-carbonyl]-3-methyl-butyl}-amid,
5-Phenyl-thiophen-2-carbonsäure-(1-{4-[2-(acetyl-methyl-amino)-acetyl]-6-oxo-hexahydro-pyrrolo[3,2-b]pyrrol-1-carbonyl}-3-methyl-butyl)-amid,
5-Phenyl-thiophen-2-carbonsäure-{1-[4-(2-dimethylamino-acetyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-amino-butyryl)-6-oxo-hexahydro-pyrrolo[3,2-b]pyrrol-1-carbonyl]-3-methyl-butyl}-amid,
5-Phenyl-thiophen-2-carbonsäure-{1-[4-(2-acetylamino-butyryl)-6-oxo-hexahydro-pyrrolo[3,2-b]pyrrol-1-carbonyl]-3-methyl-butyl}-amid,
5-Phenyl-thiophen-2-carbonsäure-{1-[4-(2-amino-3-methyl-butyryl)-6-oxo-hexahydro-pyrrolo[3,2-b]pyrrol-1-carbonyl]-3-methyl-butyl}-amid,
5-Phenyl-thiophen-2-carbonsäure-{1-[4-(2-acetylamino-3-methyl-butyryl)-6-oxo-hexahydro-pyrrolo[3,2-b]pyrrol-1-carbonyl]-3-methyl-butyl}-amid,
5-Phenyl-thiophen-2-carbonsäure-{1-[4-(2-amino-3,3-dimethyl-butyryl)-6-oxo-hexahydro-pyrrolo[3,2-b]pyrrol-1-carbonyl]-3-methyl-butyl}-amid,
5-Phenyl-thiophen-2-carbonsäure-{1-[4-(2-acetylamino-3,3-dimethyl-butyryl)-6-oxo-hexahydro-pyrrolo[3,2-b]pyrrol-1-carbonyl]-3methyl-butyl}-amid,
5-Phenyl-thiophen-2-carbonsäure-{1-[4-(2-amino-3-methyl-pentanoyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-acetylamino-3-methyl-pentanoyl)-6-oxo-hexahydro-pyrrolo[3,2-b]pyrrol-1-carbonyl]-3-methyl-butyl}-amid,
5-Phenyl-thiophen-2-carbonsäure-[3-methyl-1-(6-oxo-4-pentanoyl-hexahydro-pyrrolo[3,2-b]pyrrol-1-carbonyl)-butyl]-amid,
5-Phenyl-thiophen-2-carbonsäure-{3-methyl-1-[4-(3-methyl-pentanoyl)-6-oxo-hexahydro-pyrrolo[3,2-b]pyrrol-1-carbonyl]-butyl}-amid,
5-Phenyl-thiophen-2-carbonsäure-{3-methyl-1-[4-(4-methyl-pentanoyl)-6-oxo-hexahydro-pyrrolo[3,2-b]pyrrol-1-carbonyl]-butyl}-amid,
5-Phenyl-thiophen-2-carbonsäure-{1-[4-(2-amino-4-methyl-pentanoyl)-6-oxo-hexahydro-pyrrolo[3,2-b]pyrrol-1-carbonyl]-3-methyl-butyl}amid,
5-Phenyl-thiophen-2-carbonsäure-{1-[4-(2-acetylamino-4-methyl-pentanoyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-amino-4,4-dimethyl-pentanoyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-acetylamino-4,4-dimethyl-pentanoyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-amino-pentanoyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-acetylamino-pentanoyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-amino-5-methyl-hexanoyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-acetylamino-5-methyl-hexanoyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-hydroxy-3-methyl-butyryl)-6-oxo-hexahydro-pyrrolo[3,2-b]pyrrol-1-carbonyl]-3-methyl-butyl}-amid,
5-Phenyl-thiophen-2-carbonsäure-{1-[4-(2-methoxy-3-methyl-butyryl)-6-oxo-hexahydro-pyrrolo[3,2-b]pyrrol-1-carbonyl]-3-methyl-butylfamid,
5-Phenyl-thiophen-2-carbonsäure-1-[4-(2-hydroxy-3,3-dimethyl-butyryl)-6-oxo-hexahydro-pyrrolo[3,2-b]pyrrol-1-carbonyl]-3-methyl-butyl}-amid,
5-Phenyl-thiophen-2-carbonsäure-{1-[4-(2-methoxy-3,3-dimethyl-butyryl)-6-oxo-hexahydro-pyrrolo[3,2-b]pyrrol-1-carbonyl]-3-methyl-butyl}-amid,
5-Phenyl-thiophen-2-carbonsäure-{1-[4-(2-hydroxy-3-methyl-pentanoyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-methoxy-3-methyl-pentanoyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-hydroxy-4-methyl-pentanoyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-methoxy-4-methyl-pentanoyl)-6-oxo-hexahydro-pyrrolo[3,2-b]pyrrol-1-carbonyl]-3-methyl-butyl}-amid,
5-Phenyl-thiophen-2-carbonsäure-1-[4-(2-hydroxy-4,4-dimethyl-pentanoyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-methoxy-4,4-dimethyl-pentanoyl)-6-oxo-hexahydro-pyrrolo[3,2-b]pyrrol-1-carbonyl]-3-methyl-butyl}-amid,
5-Phenyl-thiophen-2-carbonsäure=-{1-[4-(2-hydroxy-pentanoyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-methoxy-pentanoyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-hydroxy-5-methyl-hexanoyl)-6-oxo-hexahydro-pyrrolo[3,2-b]pyrrol-1-carbonyl]-3-methyl-butyl}-amid,
5-Phenyl-thiophen-2-carbonsäure-{1-[4-(2-methoxy-5-methyl-hexanoyl)-6-oxo-hexahydro-pyrrolo[3,2-b]pyrrol-1-carbonyl]-3-methyl-butyl}-amid,
N-[1-(4-Benzoyl-6-oxo-hexahydro-pyrrolo[3,2-b]pyrrol-1-carbonyl)-3-methyl-butyl]-4-pyrrolidin-1-ylbenzamid,
N-{3-Methyl-1-[6-oxo-4-(pyridin-2-carbonyl)-hexahydro-pyrrolo[3,2-b]pyrrol-1-carbonyl]-butyl}-4-pyrrolidin-1-yl-benzamid,
N-{3-Methyl-1-[6-oxo-4-(1-oxy-pyridin-2-carbonyl)-hexahydro-pyrrolo[3,2-b]pyrrol-1-carbonyl]-butyl}-4-pyrrolidin-1-yl-benzamid,
N-{3-Methyl-1-[6-oxo-4-(pyridin-3-carbonyl)-hexahydro-pyrrolo[3,2-b]pyrrol-1-carbonyl]-butyl}-4-pyrrolidin-1-yl-benzamid,
N-{3-Methyl-1-[-oxo-4-(1-oxy-pyridin-3-carbonyl)-hexahydro-pyrrolo[3,2-b]pyrrol-1-carbonyl]-butyl}-4-pyrrolidin-1-yl-benzamid,
N-{3-Methyl-1-[6-oxo-4-(pyridin-4-carbonyl)-hexahydro-pyrrolo[3,2-b]pyrrol-1-carbonyl]-butyl}-4-pyrrolidin-1-yl-benzamid,
N-{3-Methyl-1-[6-oxo-4-(1-oxy-pyridin-4-carbonyl)-hexahydro-pyrrolo[3,2-b]pyrrol-1-carbonyl]-butyl}-4-pyrrolidin-1-yl-benzamid,
N-[1-(4-Benzensulfonyl-6-oxo-hexahydro-pyrrolo[3,2-b]pyrrol-1-carbonyl)-3-methyl-butyl]-4-pyrrolidin-1-yl-benzamid,
N-{3-Methyl-1-[6-oxo-4-(pyridin-2-sulfonyl)-hexahydro-pyrrolo[3,2-b]pyrrol-1-carbonyl]-butyl}-4-pyrrolidin-1-yl-benzamid,
N-{3-Methyl-1-[6-oxo-4-(1-oxy-pyridin-2-sulfonyl)-hexahydro-pyrrolo[3,2-b]pyrrol-1-carbonyl]-butyl}-4-pyrrolidin-1-yl-benzamid,
N-{3-Methyl-1-[6-oxo-4-(pyridin-3-sulfonyl)-hexahydro-pyrrolo[3,2-b]pyrrol-1-carbonyl]-butyl}-4-pyrrolidin-1-yl-benzamid,
N-{3-Methyl-1-[6-oxo-4-(1-oxy-pyridin-3-sulfonyl)-hexahydro-pyrrolo[3,2-b]pyrrol-1-carbonyl]-butyl}-4-pyrrolidin-1-yl-benzamid,
N-{3-Methyl-1-[6-oxo-4-(pyridin-4-sulfonyl)-hexahydro-pyrrolo[3,2-b]pyrrol-1-carbonyl]-butyl}-4-pyrrolidin-1-yl-benzamid,
N-{3-Methyl-1-[6-oxo-4-(1-oxy-pyridin-4-sulfonyl)-hexahydro-pyrrolo[3,2-b]pyrrol-1-carbonyl]-butyl}-4-pyrrolidin-1-yl-benzamid,
N-[3-Methyl-1-(6-oxo-4-phenylacetyl-hexahydro-pyrrolo[3,2-b]-pyrrol-1-carbonyl)-butyl]-4-pyrrolidin-1-yl-benzamid,

4-[2-(4-Pyrrolidin-1-yl-benzoylamino)-4-methyl-pentanoyl]-3-oxo-hexahydro-pyrrolo[3,2-b]pyrrol-1-carbonsäurephenylamid,
4-[2-(4-Pyrrolidin-1-yl-benzoylamino)-4-methyl-pentanoyl]-3-oxo-hexahydro-pyrrolo[3,2-b]pyrrol-1-carbonsärueisobutylamid,
4-[2-(4-Pyrrolidin-1-yl-benzoylamino)-4-methyl-pentanoyl]-3-oxo-hexahydro-pyrrolo[3,2-b]pyrrol-1-carbonsäurephenylester,
4-[2-(4-Pyrrolidin-1-yl-benzoylamino)-4-methyl-pentanoyl]-3-oxo-hexahydro-pyrrolo[3,2-b]pyrrol-1-carbonsäure-isobutylester,
N-{3-Methyl-1-[6-oxo-4-(pyrrolidin-1-carbonyl)-hexahydro-pyrrolo[3,2-b]pyrrol-1-carbonyl]-butyl}-4-pyrrolidin-1-yl-benzamid,
N-{3-Methyl-1-[6-oxo-4-(piperidin-1-carbonyl)-hexahydro-pyrrolo[3,2-b]pyrrol-1-carbonyl]-butyl}-4-pyrrolidin-1-yl-benzamid,
N-{3-Methyl-1-[4-(morpholin-4-carbonyl)-6-oxo-hexahydro-pyrrolo[3,2-b]pyrrol-1-carbonyl]-butyl}-4-pyrrolidin-1-yl-benzamid,
N-{3-Methyl-1-[6-oxo-4-(piperazin-1-carbonyl)-hexahydro-pyrrolo[3,2-b]pyrrol-1-carbonyl]-butyl}-4-pyrrolidin-1-yl-benzamid,
N-{3-Methyl-1-[4-(4-methyl-piperazin-1-carbonyl)-6-oxo-hexahydro-pyrrolo[3,2-b]pyrrol-1-carbonyl]-butyl}-4-pyrrolidin-1-yl-benzamid,
N-(3-Methyl-1-{4-[4-(4-methyl-piperazin-1-yl)-acetyl]-6-oxo-hexahydro-pyrrolo[3,2-b]pyrrol-1-carbonyl}-butyl)-4-pyrrolidin-1-yl-benzamid,
N-{3-Methyl-1-[6-oxo-4-(2-piperazin-1-yl-acetyl)-hexahydro-pyrrolo[3,2-b]pyrrol-1-carbonyl]-butyl}-4-pyrrolidin-1-yl-benzamid,
N-1-[4-(Furan-2-carbonyl)-6-oxo-hexahydro-pyrrolo[3,2-b]pyrrol-1-carbonyl]-3-methyl-butyl}-4-pyrrolidin-1-yl-benzamid,
N-{1-[4-(5-Chlor-furan-2-carbonyl)-6-oxo-hexahydro-pyrrolo[3,2-b]pyrrol-1-carbonyl]-3-methyl-butyl}-4-pyrrolidin-1-yl-benzamid,
N-1-[4-(Thiophen-2-carbonyl)-6-oxo-hexahydro-pyrrolo[3,2-b]pyrrol-1-carbonyl]-3-methyl-butyl}-4-pyrrolidin-1-yl-benzamid,
N-{1-[4-(5-Chlor-thiophen-2-carbonyl)-6-oxo-hexahydro-pyrrolo[3,2-b]pyrrol-1-carbonyl]-3-methyl-butyl}-4-pyrrolidin-1-yl-benzamid,
N-{1-[4-(Furan-3-carbonyl)-6-oxo-hexahydro-pyrrolo[3,2-b]pyrrol-1-carbonyl]-3-methyl-butyl}-4-pyrrolidin-1-yl-benzamid,
N-1-[4-(Thiophen-3-carbonyl)-6-oxo-hexahydro-pyrrolo[3,2-b]pyrrol-1-carbonyl]-3-methyl-butyl}-4-pyrrolidin-1-yl-benzamid,
N-[1-(4-Cyclopentancarbonyl-6-oxo-hexahydro-pyrrolo[3,2-b]pyrrol-1-carbonyl)-3-methyl-butyl]-4-pyrrolidin-1-yl-benzamid,
N-[1-(4-Cyclohexancarbonyl-6-oxo-hexahydro-pyrrolo[3,2-b]pyrrol-1-carbonyl)-3-methyl-butyl]-4-pyrrolidin-1-yl-benzamid,
N-{1-[4-(Cyclopent-3-encarbonyl)-6-oxo-hexahydro-pyrrolo[3,2-b]pyrrol-1-carbonyl]-3-methyl-butyl}-4-pyrrolidin-1-yl-benzamid,
N-{1-[4-(2-Cyclopentyl-acetyl)-6-oxo-hexahydro-pyrrolo[3,2-b]pyrrol-1-carbonyl]-3-methyl-butyl}-4-pyrrolidin-1-yl-benzamid,
N-{1-[4-(2-Cyclohexyl-acetyl)-6-oxo-hexahydro-pyrrolo[3,2-b]pyrrol-1-carbonyl]-3-methyl-butyl}-4-pyrrolidin-1-yl-benzamid,
4-Pyrrolidin-1-yl-N-{3-methyl-1-[6-oxo-4-(pyrrolidin-2-carbonyl)-hexahydro-pyrrolo[3,2-b]pyrrol-1-carbonyl]-butyl}-benzamid,
N-{1-[4-(1-Acetyl-pyrrolidin-2-carbonyl)-6-oxo-hexahydro-pyrrolo[3,2-b]pyrrol-1-carbonyl]-3-methyl-butyl}-4-pyrrolidin-1-yl-benzamid,
N-{3-Methyl-1-[6-oxo-4-(piperidin-2-carbonyl)-hexahydro-pyrrolo[3,2-b]pyrrol-1-carbonyl]-butyl}-4-pyrrolidin-1-yl-benzamid,
N-{1-[4-(1-Acetyl-piperidin-2-carbonyl)-6-oxo-hexahydro-pyrrolo[3,2-b]pyrrol-1-carbonyl]-3-methyl-butyl}-4-pyrrolidin-1-yl-benzamid,
N-{3-Methyl-1-[6-oxo-4-(tetrahydro-furan-2-carbonyl)-hexahydro-pyrrolo[3,2-b]pyrrol-1-carbonyl]-butyl}-4-pyrrolidin-1-yl-benzamid,
N-{1-[4-(1-Amino-cyclobutancarbonyl)-6-oxo-hexahydro-pyrrolo[3,2-b]pyrrol-1-carbonyl]-3-methyl-butyl}-4-pyrrolidin-1-yl-benzamid,
N-1-[4-(1-Amino-cyclopentancarbonyl)-6-oxo-hexahydro-pyrrolo[3,2-b]pyrrol-1-carbonyl]-3-methyl-butyl}-4-pyrrolidin-1-yl-benzamid,

N-1-[4-(1-Amino-cyclohexancarbonyl)-6-oxo-hexahydro-pyrrolo[3,2-b]pyrrol-1-carbonyl]-3-methyl-butyl}-4-pyrrolidin-1-yl-benzamid,
N-1-[4-(1-Acetylamino-cyclobutancarbonyl)-6-oxo-hexahydro-pyrrolo[3,2-b]pyrrol-1-carbonyl]-3-methyl-butyl}-4-pyrrolidin-1-yl-benzamid,
N-{1-[4-(1-Acetylamino-cyclopentancarbonyl)-6-oxo-hexahydro-pyrrolo[3,2-b]pyrrol-1-carbonyl]-3-methyl-butyl}-4-pyrrolidin-1-yl-benzamid,
N-1-[4-(1-Acetylamino-cyclohexancarbonyl)-6-oxo-hexahydro-pyrrolo[3,2-b]pyrrol-1-carbonyl]-3-methyl-butyl}-4-pyrrolidin-1-yl-benzamid,
N-{1-[4-(1-Hydroxy-cyclobutancarbonyl)-6-oxo-hexahydro-pyrrolo[3,2-b]pyrrol-1-carbonyl]-3-methyl-butyl}-4-pyrrolidin-1-yl-benzamid,
N-{1-[4-(1-Hydroxy-cyclopentancarbonyl)-6-oxo-hexahydro-pyrrolo[3,2-b]pyrrol-1-carbonyl]-3-methyl-butyl}-4-pyrrolidin-1-yl-benzamid,
N-{1-[4-(1-Hydroxy-cyclohexancarbonyl)-6-oxo-hexahydro-pyrrolo[3,2-b]pyrrol-1-carbonyl]-3-methyl-butyl}-4-pyrrolidin-1-yl-benzamid,
N-{1-[4-(1-Methoxy-cyclobutancarbonyl)-6-oxo-hexahydro-pyrrolo[3,2-b]pyrrol-1-carbonyl]-3-methyl-butyl}-4-pyrrolidin-1-yl-benzamid,
N-{1-[4-(1-Methoxy-cyclopentancarbonyl)-6-oxo-hexahydro-pyrrolo[3,2-b]pyrrol-1-carbonyl]-3-methyl-butyl}-4-pyrrolidin-1-yl-benzamid,
N-{1-[4-(1-Methoxy-cyclohexancarbonyl)-6-oxo-hexahydro-pyrrolo[3,2-b]pyrrol-1-carbonyl]-3-methyl-butyl}-4-pyrrolidin-1-yl-benzamid,
N-{1-[4-(2-Amino-2-cyclopentyl-acetyl)-6-oxo-hexahydro-pyrrolo[3,2-b]pyrrol-1-carbonyl]-3-methyl-butyl}-4-pyrrolidin-1-yl-benzamid,
N-{1-[4-(2-Amino-2-cyclohexyl-acetyl)-6-oxo-hexahydro-pyrrolo[3,2-b]pyrrol-1-carbonyl]-3-methyl-butyl}-4-pyrrolidin-1-yl-benzamid,
N-{1-[4-(2-Acetylamino-2-cyclopentyl-acetyl)-6-oxo-hexahydro-pyrrolo[3,2-b]pyrrol-1-carbonyl]-3-methyl-butyl}-4-pyrrolidin-1-yl-benzamid,
N-{1-[4-(2-Acetylamino-2-cyclohexyl-acetyl)-6-oxo-hexahydro-pyrrolo[3,2-b]pyrrol-1-carbonyl]-3-methyl-butyl}-4-pyrrolidin-1-yl-benzamid,
N-{1-[4-(2-Acetylamino-propionyl)-6-oxo-hexahydro-pyrrolo[3,2b]pyrrol-1-carbonyl]-3-methyl-butyl}-4-pyrrolidin-1-yl-benzamid,
N-{1-[4-(2-Acetylamino-acetyl)-6-oxo-hexahydro-pyrrolo[3,2-b]pyrrol-1-carbonyl]-3-methyl-butyl}-4-pyrrolidin-1-yl-benzamid,
N-(1-{4-[2-(Acetyl-methyl-amino)-acetyl]-6-oxo-hexahydro-pyrrolo[3,2-b]pyrrol-1-carbonyl}-3-methyl-butyl)-4-pyrrolidin-1-yl-benzamid,
N-{1-[4-(2-Dimethylamino-acetyl)-6-oxo-hexahydro-pyrrolo[3,2-b]pyrrol-1-carbonyl]-3-methyl-butyl}-4-pyrrolidin-1-yl-benzamid,
N-{1-[4-(2-Amino-butyryl)-6-oxo-hexahydro-pyrrolo[3,2-b]pyrrol-1-carbonyl]-3-methyl-butyl}-4-pyrrolidin-1-yl-benzamid,
N-{1-[4-(2-Acetylamino-butyryl)-6-oxo-hexahydro-pyrrolo[3,2-b]pyrrol-1-carbonyl]-3-methyl-butyl}-4-pyrrolidin-1-yl-benzamid,
N-{1-[4-(2-Amino-3-methyl-butyryl)-6-oxo-hexahydro-pyrrolo[3,2-b]pyrrol-1-carbonyl]-3-methyl-butyl}-4-pyrrolidin-1-yl-benzamid,
N-{1-[4-(2-Acetylamino-3-methyl-butyryl)-6-oxo-hexahydro-pyrrolo[3,2-b]pyrrol-1-carbonyl]-3-methyl-butyl}-4-pyrrolidin-1-yl-benzamid,
N-{1-[4-(2-Amino-3,3-dimethyl-butyryl)-6-oxo-hexahydro-pyrrolo[3,2-b]pyrrol-1-carbonyl]-3-methyl-butyl}-4-pyrrolidin-1-yl-benzamid,
N-{1-[4-(2-Acetylamino-3,3-dimethyl-butyryl)-6-oxo-hexahydro-pyrrolo[3,2-b]pyrrol-1-carbonyl]-3-methyl-butyl}-4-pyrrolidin-1-yl-benzamid,
N-{1-[4-(2-Amino-3-methyl-pentanoyl)-6-oxo-hexahydro-pyrrolo[3,2-b]pyrrol-1-carbonyl]-3-methyl-butyl}-4-pyrrolidin-1-yl-benzamid,
N-1-[4-(2-Acetylamino-3-methyl-pentanoyl)-6-oxo-hexahydro-pyrrolo[3,2-b]pyrrol-1-carbonyl]-3-methyl-butyl}-4-pyrrolidin-1-yl-benzamid,
N-[3-Methyl-1-(6-oxo-4-pentanoyl-hexahydro-pyrrolo[3,2-b]pyrrol-1-carbonyl)-butyl]-4-pyrrolidin-1-yl-benzamid,
N-{3-Methyl-1-[4-(3-methyl-pentanoyl)-6-oxo-hexahydro-pyrrolo[3,2-b]pyrrol-1-carbonyl]-butyl}-4-pyrrolidin-1-yl-benzamid,
N-{3-Methyl-1-[4-(4-methyl-pentanoyl)-6-oxo-hexahydro-pyrrolo[3,2-b]pyrrol-1-carbonyl]-butyl}-4-pyrrolidin-1-yl-benzamid,
N-1-[4-(2-Amino-4-methyl-pentanoyl)-6-oxo-hexahydro-pyrrolo[3,2-b]pyrrol-1-carbonyl]-3-methyl-butyl}-4-pyrrolidin-1-yl-benzamid,
N-1-[4-(2-Acetylamino-4-methyl-pentanoyl)-6-oxo-hexahydro-pyrrolo[3,2-b]pyrrol-1-carbonyl]-3-methyl-butyl}-4-pyrrolidin-1-yl-benzamid,
N-{1-[4-(2-Amino-4,4-dimethyl-pentanoyl)-6-oxo-hexahydro-pyrrolo[3,2-b]pyrrol-1-carbonyl]-3-methyl-butyl}-4-pyrrolidin-1-yl-benzamid,
N-{1-[4-(2-Acetylamino-4,4-dimethyl-pentanoyl)-6-oxo-hexahydro-pyrrolo[3,2-b]pyrrol-1-carbonyl]-3-methyl-butyl}-4-pyrrolidin-1-yl-benzamid,
N-{1-[4-(2-Acetylamino-4,4-dimethyl-pentanoyl)-6-oxo-hexahydro-pyrrolo[3,2-b]pyrrol-1-carbonyl]-3-methyl-butyl}-4-pyrrolidin-1-yl-benzamid,
N-{1-[4-(2-Amino-pentanoyl)-6-oxo-hexahydro-pyrrolo[3,2-b]pyrrol-1-carbonyl]-3-methyl-butyl}-4-pyrrolidin-1-yl-benzamid,
N-{1-[4-(2-Acetylamino-pentanoyl)-6-oxo-hexahydro-pyrrolo[3,2-b]pyrrol-1-carbonyl]-3-methyl-butyl}-4-pyrrolidin-1-yl-benzamid,
N-{1-[4-(2-Amino-5-methyl-hexanoyl)-6-oxo-hexahydro-pyrrolo[3,2-b]pyrrol-1-carbonyl]-3-methyl-butyl}-4-pyrrolidin-1-yl-benzamid,
N-{1-[4-(2-Acetylamino-5-methyl-hexanoyl)-6-oxo-hexahydro-pyrrolo[3,2-b]pyrrol-1-carbonyl]-3-methyl-butyl}-4-pyrrolidin-1-yl-benzamid,
N-{1-[4-(2-Hydroxy-3-methyl-butyryl)-6-oxo-hexahydro-pyrrolo[3,2-b]pyrrol-1-carbonyl]-3-methyl-butyl}-4-pyrrolidin-1-yl-benzamid,
N-{1-[4-(2-Methoxy-3-methyl-butyryl)-6-oxo-hexahydro-pyrrolo[3,2-b]pyrrol-1-carbonyl]-3-methyl-butyl}-4-pyrrolidin-1-yl-benzamid,
N-{1-[4-(2-Hydroxy-3,3-dimethyl-butyryl)-6-oxo-hexahydro-pyrrolo[3,2-b]pyrrol-1-carbonyl]-3-methyl-butyl}-4-pyrrolidin-1-yl-benzamid,
N-{1-[4-(2-Methoxy-3,3-dimethyl-butyryl)-6-oxo-hexahydro-pyrrolo[3,2-b]pyrrol-1-carbonyl]-3-methyl-butyi}-4-pyrrolidin-1-yl-benzamid,
N-{1-[4-(2-Hydroxy-3-methyl-pentanoyl)-6-oxo-hexahydro-pyrrolo[3,2-b]pyrrol-1-carbonyl]-3-methyl-butyl}-4-pyrrolidin-1-yl-benzamid,
N-1-[4-(2-Methoxy-3-methyl-pentanoyl)-6-oxo-hexahydro-pyrrolo[3,2-b]pyrrol-1-carbonyl]-3-methyl-butyl}-4-pyrrolidin-1-yl-benzamid,
N-{1-[4-(2-Hydroxy-4-methyl-pentanoyl)-6-oxo-hexahydro-pyrrolo[3,2-b]pyrrol-1-carbonyl]-3-methyl-butyl}-4-pyrrolidin-1-yl-benzamid,
N-{1-[4-(2-Methoxy-4-methyl-pentanoyl)-6-oxo-hexahydro-pyrrolo[3,2-b]pyrrol-1-carbonyl]-3-methyl-butyl}-4-pyrrolidin-1-yl-benzamid,
N-{1-[4-(2-Hydroxy-4,4-dimethyl-pentanoyl)-6-oxo-hexahydro-pyrrolo[3,2-b]pyrrol-1-carbonyl]-3-methyl-butyl}-4-pyrrolidin-1-yl-benzamid,
N-{1-[4-(2-Methoxy-4,4-dimethyl-pentanoyl)-6-oxo-hexahydro-pyrrolo[3,2-b]pyrrol-1-carbonyl]-3-methyl-butyl}-4-pyrrolidin-1-yl-benzamid,
N-{1-[4-(2-Hydroxy-pentanoyl)-6-oxo-hexahydro-pyrrolo[3,2-b]pyrrol-1-carbonyl]-3-methyl-butyl}-4-pyrrolidin-1-yl-benzamid,
N-{1-[4-(2-Methoxy-pentanoyl)-6-oxo-hexahydro-pyrrolo[3,2-b]pyrrol-1-carbonyl]-3-methyl-butyl}-4-pyrrolidin-1-yl-benzamid,
N-{1-[4-(2-Hydroxy-5-methyl-hexanoyl)-6-oxo-hexahydro-pyrrolo[3,2-b]pyrrol-1-carbonyl]-3-methyl-butyl}-4-pyrrolidin-1-yl-benzamid,
N-{1-[4-(2-Methoxy-5-methyl-hexanoyl)-6-oxo-hexahydro-pyrrolo[3,2-b]pyrrol-1-carbonyl]-3-methyl-butyl}-4-pyrrolidin-1-yl-benzamid,
N-[1-(4-Benzoyl-6-oxo-hexahydro-pyrrolo[3,2-b]pyrrol-1-carbonyl)-3-methyl-butyl]-4-morpholin-4-yl-benzamid,
N-{3-Methyl-1-[-oxo-4-(1-oxy-pyridin-2-carbonyl)-hexahydro-pyrrolo[3,2-b]pyrrol-1-carbonyl]-butyl}-4-morpholin-4-yl-benzamid,
N-{3-Methyl-1-[6-oxo-4-(pyridin-3-carbonyl)-hexahydro-pyrrolo[3,2-b]pyrrol-1-carbonyl]-butyl}-4-morpholin-4-yl-benzamid,
N-{3-Methyl-1-[6-oxo-4-(1-oxy-pyridin-3-carbonyl)-hexahydro-pyrrolo[3,2-b]pyrrol-1-carbonyl]-butyl}-4-morpholin-4-yl-benzamid,
N-{3-Methyl-1-[6-oxo-4-(pyridin-4-carbonyl)-hexahydro-pyrrolo[3,2-b]pyrrol-1-carbonyl]-butyl}-4-morpholin-4-yl-benzamid,
N-{3-Methyl-1-[6-oxo-4-(1-oxy-pyridin-4-carbonyl)-hexahydro-pyrrolo[3,2-b]pyrrol-1-carbonyl]-butyl}-4-morpholin-4-yl-benzamid,
N-[1-(4-Benzensulfonyl-6-oxo-hexahydro-pyrrolo[3,2-b]pyrrol-1-carbonyl)-3-methyl-butyl]-4-morpholin-4-yl-benzamid,
N-{3-Methyl-1-[6-oxo-4-(pyridin-4-sulfonyl)-hexahydro-pyrrolo[3,2-b]pyrrol-1-carbonyl]-butyl}-4-morpholin-4-yl-benzamid,
N-{3-Methyl-1-[6-oxo-4-(1-oxy-pyridin-4-sulfonyl)-hexahydro-pyrrolo[3,2-b]pyrrol-1-carbonyl]-butyl}-4-morpholin-4-yl-benzamid,
N-[3-Methyl-1-(6-oxo-4-phenylacetyl-hexahydro-pyrrolo[3,2-b]pyrrol-1-carbonyl)-butyl]-4-morpholin-4-yl-benzamid,
4-[2-(4-Morpholin-4-yl-benzoylamino)-4-methyl-pentanoyl]-3-oxo-hexahydro-pyrrolo[3,2-b]pyrrol-1-carbonsäurephenylamid,
4-[2-(4-Morpholin-4-yl-benzoylamino)-4-methyl-pentanoyl]-3-oxo-hexahydro-pyrrolo[3,2-b]pyrrol-1-carbonsäureisobutylamid,
4-[2-(4-Morpholin-4-yl-benzoylamino)-4-methyl-pentanoyl]-3-oxo-hexahydro-pyrrolo[3,2-b]pyrrol-1-carbonsäurephenylester,
4-[2-(4-Morpholin-4-yl-benzoylamino)-4-methyl-pentanoyl]-3-oxo-hexahydro-pyrrolo[3,2-b]pyrrol-1-carbonsäure-isobutylester,
N-{3-Methyl-1-[6-oxo-4-(pyrrolidin-1-carbonyl)-hexahydro-pyrrolo[3,2-b]pyrrol-1-carbonyl]-butyl}-4-morpholin-4-yl-benzamid,
N-{3-Methyl-1-[6-oxo-4-(piperidin-1-carbonyl)-hexahydro-pyrrolo[3,2-b]pyrrol-1-carbonyl]-butyl}-4-morpholin-4-yl-benzamid,
N-{3-Methyl-1-[4-(morpholin-4-carbonyl)-6-oxo-hexahydro-pyrrolo[3,2-b]pyrrol-1-carbonyl]-butyl}-4-morpholin-4-yl-benzamid,
N-{3-Methyl-1-[6-oxo-4-(piperazin-1-carbonyl)-hexahydro-pyrrolo[3,2-b]pyrrol-1-carbonyl]-butyl}-4-morpholin-4-yl-benzamid,
N-{3-Methyl-1-[4-(4-methyl-piperazin-1-carbonyl)-6-oxo-hexahydro-pyrrolo[3,2-b]pyrrol-1-carbonyl]-butyl}-4-morpholin-4-yl-benzamid,
N-(3-Methyl-1-{4-[2-(4-methyl-piperazin-1-yl)-acetyl]-6-oxo-hexahydro-pyrrolo[3,2-b]pyrrol-1-carbonyl}-butyl)-4-morpholin-4-yl-benzamid,
N-{3-Methyl-1-[6-oxo-4-(2-piperazin-1-yl-acetyl)-hexahydro-pyrrolo[3,2-b]pyrrol-1-carbonyl]-butyl}-4-morpholin-4-yl-benzamid,
N-{1-[4-(Furan-2-carbonyl)-6-oxo-hexahydro-pyrrolo[3,2-b]pyrrol-1-carbonyl]-3-methyl-butyl}-4-morpholin-4-yl-benzamid,
N-1-[4-(5-Chlor-furan-2-carbonyl)-6-oxo-hexahydro-pyrrolo[3,2-b]pyrrol-1-carbonyl]-3-methyl-butyl}-4-morpholin-4-yl-benzamid,
N-{1-[4-(Thiophen-2-carbonyl)-6-oxo-hexahydro-pyrrolo[3,2-b]pyrrol-1-carbonyl]-3-methyl-butyl}-4-morpholin-4-yl-benzamid,
N-{1-[4-(5-Chlor-thiophen-2-carbonyl)-6-oxo-hexahydro-pyrrolo[3,2-b]pyrrol-1-carbonyl]-3-methyl-butyl}-4-morpholin-4-yl-benzamid,
N-{1-[4-(Furan-3-carbonyl)-6-oxo-hexahydro-pyrrolo[3,2-b]pyrrol-1-carbonyl]-3-methyl-butyl}-4-morpholin-4-yl-benzamid,
N-{1-[4-(Thiophen-3-carbonyl)-6-oxo-hexahydro-pyrrolo[3,2-b]pyrrol-1-carbonyl]-3-methyl-butyl}-4-morpholin-4-yl-benzamid,
N-[1-(4-Cyclopentancarbonyl-6-oxo-hexahydro-pyrrolo[3,2-b]pyrrol-1-carbonyl)-3-methyl-butyl]-4-morpholin-4-yl-benzamid,
N-[1-(4-Cyclohexancarbonyl-6-oxo-hexahydro-pyrrolo[3,2-b]pyrrol-1-carbonyl)-3-methyl-butyl}]4-morpholin-4-yl-benzamid,
N-{1-[4-(Cyclopent-3-encarbonyl)-6-oxo-hexahydro-pyrrolo[3,2-b]pyrrol-1-carbonyl]-3-methyl-butyl}-4-morpholin-4-yl-benzamid,
N-{1-[4-(2-Cyclopentyl-acetyl)-6-oxo-hexahydro-pyrrolo[3,2-b]pyrrol-1-carbonyl]-3-methyl-butyl}-4-morpholin-4-yl-benzamid,
N-{1-[4-(2-Cyclohexyl-acetyl)-6-oxo-hexahydro-pyrrolo[3,2-b]pyrrol-1-carbonyl]-3-methyl-butyl}-4-morpholin-4-yl-benzamid,
4-Morpholin-4-yl-N-{3-methyl-1-[6-oxo-4-(pyrrolidin-2-carbonyl)-hexahydro-pyrrolo[3,2-b]pyrrol-1-carbonyl]-butyl}-benzamid,
N-{1-[4-(1-Acetyl-pyrrolidin-2-carbonyl)-6-oxo-hexahydro-pyrrolo[3,2-b]pyrrol-1-carbonyl]-3-methyl-butyl}-4-morpholin-4-yl-benzamid,
N-{3-Methyl-1-[6-oxo-4-(piperidin-2-carbonyl)-hexahydro-pyrrolo[3,2-b]pyrrol-1-carbonyl]-butyl}-4-morpholin-4-yl-benzamid,
N-{1-[4-(1-Acetyl-piperidin-2-carbonyl)-6-oxo-hexahydro-pyrrolo[3,2-b]pyrrol-1-carbonyl]-3-methyl-butyl}-4-morpholin-4-yl-benzamid,
N-{3-Methyl-1-[6-oxo-4-(tetrahydro-furan-2-carbonyl)-hexahydro-pyrrolo[3,2-b]pyrrol-1-carbonyl]-butyl}-4-morpholin-4-yl-benzamid,

N-{1-[4-(1-Amino-cyclobutancarbonyl)-6-oxo-hexahydro-pyrrolo[3,2-b]pyrrol-1-carbonyl]-3-methyl-butyl}-4-morpholin-4-yl-benzamid,
N-{1-[4-(1-Amino-cyclopentancarbonyl)-6-oxo-hexahydro-pyrrolo[3,2-b]pyrrol-1-carbonyl]-3-methyl-butyl}-4-morpholin-4-yl-benzamid,
N-{1-[4-(1-Amino-cyclohexancarbonyl)-6-oxo-hexahydro-pyrrolo[3,2-b]pyrrol-1-carbonyl]-3-methyl-butyl}-4-morpholin-4-yl-benzamid,
N-{1-[4-(1-Acetylamino-cyclobutancarbonyl)-6-oxo-hexahydro-pyrrolo[3,2-b]pyrrol-1-carbonyl]-3-methyl-butyl}-4-morpholin-4-yl-benzamid,
N-{1-[4-(1-Acetylamino-cyclopentancarbonyl)-6-oxo-hexahydro-pyrrolo[3,2-b]pyrrol-1-carbonyl]-3-methyl-butyl}-4-morpholin-4-yl-benzamid,
N-{1-[4-(1-Acetylamino-cyclohexancarbonyl)-6-oxo-hexahydro-pyrrolo[3,2-b]pyrrol-1-carbonyl]-3-methyl-butyl}-4-morpholin-4-yl-benzamid,
N-{1-[4-(1-Hydroxy-cyclobutancarbonyl)-6-oxo-hexahydro-pyrrolo[3,2-b]pyrrol-1-carbonyl]-3-methyl-butyl}-4-morpholin-4-yl-benzamid,
N-{1-[4-(1-Hydroxy-cyclopentancarbonyl)-6-oxo-hexahydro-pyrrolo[3,2-b]pyrrol-1-carbonyl]-3-methyl-butyl}-4-morpholin-4-yl-benzamid,
N-{1-[4-(1-Hydroxy-cyclohexancarbonyl)-6-oxo-hexahydro-pyrrolo[3,2-b]pyrrol-1-carbonyl]-3-methyl-butyl}-4-morpholin-4-yl-benzamid,
N-{1-[4-(1-Methoxy-cyclobutancarbonyl)-6-oxo-hexahydro-pyrrolo[3,2-b]pyrrol-1-carbonyl]-3-methyl-butyl}-4-morpholin-4-yl-benzamid,
N-{1-[4-(1-Methoxy-cyclopentancarbonyl)-6-oxo-hexahydro-pyrrolo[3,2-b]pyrrol-1-carbonyl]-3-methyl-butyl}-4-morpholin-4-yl-benzamid,
N-{1-[4-(1-Methoxy-cyclohexancarbonyl)-6-oxo-hexahydro-pyrrolo[3,2-b]pyrrol-1-carbonyl]-3-methyl-butyl}-4-morpholin-4-yl-benzamid,
N-{1-[4-(2-Amino-2-cyclopentyl-acetyl)-6-oxo-hexahydro-pyrrolo[3,2-b]pyrrol-1-carbonyl]-3-methyl-butyl}-4-morpholin-4-yl-benzamid,
N-{1-[4-(2-Amino-2-cyclohexyl-acetyl)-6-oxo-hexahydro-pyrrolo[3,2-b]pyrrol-1-carbonyl]-3-methyl-butyl}-4-morpholin-4-yl-benzamid,
N-1-[4-(2-Acetylamino-2-cyclopentyl-acetyl)-6-oxo-hexahydro-pyrrolo[3,2-b]pyrrol-1-carbonyl]-3-methyl-butyl}-4-morpholin-4-yl-benzamid,
N-1-[4-(2-Acetylamino-2-cyclohexyl-acetyl)-6-oxo-hexahydro-pyrrolo[3,2-b]pyrrol-1-carbonyl]-3-methyl-butyl}-4-morpholin-4-yl-benzamid,
N-{1-[4-(2-Acetylamino-propionyl)-6-oxo-hexahydro-pyrrolo[3,2-b]pyrrol-1-carbonyl]-3-methyl-butyl}-4-morpholin-4-yl-benzamid,
N-{1-[4-(2-Acetylamino-acetyl)-6-oxo-hexahydro-pyrrolo[3,2-b]pyrrol-1-carbonyl]-3-methyl-butyl}-4-morpholin-4-yl-benzamid,
N-(1-{4-[2-(Acetyl-methyl-amino)-acetyl]-6-oxo-hexahydro-pyrrolo[3,2-b]pyrrol-1-carbonyl}-3-methyl-butyl)-4-morpholin-4-yl-benzamid,
N-1-[4-(2-Dimethylamino-acetyl)-6-oxo-hexahydro-pyrrolo[3,2-b]pyrrol-1-carbonyl]-3-methyl-butyl}-4-morpholin-4-yl-benzamid,
N-{1-[4-(2-Amino-butyryl)-6-oxo-hexahydro-pyrrolo[3,2-b]pyrrol-1-carbonyl]-3-methyl-butyl}-4-morpholin-4-yl-benzamid,
N-{1-[4-(2-Acetylamino-butyryl)-6-oxo-hexahydro-pyrrolo[3,2-b]pyrrol-1-carbonyl]-3-methyl-butyl}-4-morpholin-4-yl-benzamid,
N-1-[4-(2-Amino-3-methyl-butyryl)-6-oxo-hexahydro-pyrrolo[3,2-b]pyrrol-1-carbonyl]-3-methyl-butyl}-4-morpholin-4-yl-benzamid,
N-{1-[4-(2-Acetylamino-3-methyl-butyryl)-6-oxo-hexahydro-pyrrolo[3,2-b]pyrrol-1-carbonyl]-3-methyl-butyl}-4-morpholin-4-yl-benzamid,
N-{1-[4-(2-Amino-3,3-dimethyl-butyryl)-6-oxo-hexahydro-pyrrolo[3,2-b]pyrrol-1-carbonyl]-3-methyl-butyl}-4-morpholin-4-yl-benzamid,
N-{1-[4-(2-Acetylamino-3,3-dimethyl-butyryl)-6-oxo-hexahydro-pyrrolo[3,2-b]pyrrol-1-carbonyl]-3-methyl-butyl}-4-morpholin-4-yl-benzamid,
N-{1-[4-(2-Amino-3-methyl-pentanoyl)-6-oxo-hexahydro-pyrrolo[3,2-b]pyrrol-1-carbonyl]-3-methyl-butyl}-4-morpholin-4-yl-benzamid,
N-{1-[4-(2-Acetylamino-3-methyl-pentanoyl)-6-oxo-hexahydro-pyrrolo[3,2-b]pyrrol-1-carbonyl]-3-methyl-butyl}-4-morpholin-4-yl-benzamid,
N-[3-Methyl-1-(6-oxo-4-pentanoyl-hexahydro-pyrrolo[3,2-b]pyrrol-1-carbonyl)-butyl]-4-morpholin-4-yl-benzamid,
N-{3-Methyl-1-[4-(3-methyl-pentanoyl)-6-oxo-hexahydro-pyrrolo[3,2-b]pyrrol-1-carbonyl]-butyl}-4-morpholin-4-yl-benzamid,
N-{3-Methyl-1-[4-(4-methyl-pentanoyl)-6-oxo-hexahydro-pyrrolo[3,2-b]pyrrol-1-carbonyl]-butyl}-4-morpholin-4-yl-benzamid,
N-{1-[4-(2-Amino-4-methyl-pentanoyl)-6-oxo-hexahydro-pyrrolo[3,2-b]pyrrol-1-carbonyl]-3-methyl-butyl}-4-morpholin-4-yl-benzamid,
N-{1-[4-(2-Acetylamino-4-methyl-pentanoyl)-6-oxo-hexahydro-pyrrolo[3,2-b]pyrrol-1-carbonyl]-3-methyl-butyl}-4-morpholin-4-yl-benzamid,
N-{1-[4-(2-Amino-4,4-dimethyl-pentanoyl)-6-oxo-hexahydro-pyrrolo[3,2-b]pyrrol-1-carbonyl]-3-methyl-butyl}-4-morpholin-4-yl-benzamid,
N-{1-[4-(2-Acetylamino-4,4-dimethyl-pentanoyl)-6-oxo-hexahydro-pyrrolo[3,2-b]pyrrol-1-carbonyl]-3-methyl-butyl}-4-morpholin-4-yl-benzamid,
N-{1-[4-(2-Amino-pentanoyl)-6-oxo-hexahydro-pyrrolo[3,2-b]pyrrol-1-carbonyl]-3-methyl-butyl}-4-morpholin-4-yl-benzamid,
N-{1-[4-(2-Acetylamino-pentanoyl)-6-oxo-hexahydro-pyrrolo[3,2-b]pyrrol-1-carbonyl]-3-methyl-butyl}-4-morpholin-4-yl-benzamid,
N-{1-[4-(2-Amino-5-methyl-hexanoyl)-6-oxo-hexahydro-pyrrolo[3,2-b]pyrrol-1-carbonyl]-3-methyl-butyl}-4-morpholin-4-yl-benzamid,
N-{1-[4-(2-Acetylamino-5-methyl-hexanoyl)-6-oxo-hexahydro-pyrrolo[3,2-b]pyrrol-1-carbonyl]-3-methyl-butyl}-4-morpholin-4-yl-benzamid,
N-{1-[4-(2-Hydroxy-3-methyl-butyryl)-6-oxo-hexahydro-pyrrolo[3,2-b]pyrrol-1-carbonyl]-3-methyl-butyl}-4-morpholin-4-yl-benzamid,
N-{1-[4-(2-Methoxy-3-methyl-butyryl)-6-oxo-hexahydro-pyrrolo[3,2-b]pyrrol-1-carbonyl]-3-methyl-butyl}-4-morpholin-4-yl-benzamid,
N-{1-[4-(2-Hydroxy-3,3-dimethyl-butyryl)-6-oxo-hexahydro-pyrrolo[3,2-b]pyrrol-1-carbonyl]-3-methyl-butyl}-4-morpholin-4-yl-benzamid,
N-{1-[4-(2-Methoxy-3,3-dimethyl-butyryl)-6-oxo-hexahydro-pyrrolo[3,2-b]pyrrol-1-carbonyl]-3-methyl-butyl}-4-morpholin-4-yl-benzamid,
N-{1-[4-(2-Hydroxy-3-methyl-pentanoyl)-6-oxo-hexahydro-pyrrolo[3,2-b]pyrrol-1-carbonyl]-3-methyl-butyl}-4-morpholin-4-yl-benzamid,
N-1-[4-(2-Methoxy-3-methyl-pentanoyl)-6-oxo-hexahydro-pyrrolo[3,2-b]pyrrol-1-carbonyl]-3-methyl-butyl}-4-morpholin-4-yl-benzamid,
N-{1-[4-(2-Hydroxy-4-methyl-pentanoyl)-6-oxo-hexahydro-pyrrolo[3,2-b]pyrrol-1-carbonyl]-3-methyl-butyl}-4-morpholin-4-yl-benzamid,
N-{1-[4-(2-Methoxy-4-methyl-pentanoyl)-6-oxo-hexahydro-pyrrolo[3,2-b]pyrrol-1-carbonyl]-3-methyl-butyl}-4-morpholin-4-yl-benzamid,
N-{1-[4-(2-Hydroxy-4,4-dimethyl-pentanoyl)-6-oxo-hexahydro-pyrrolo[3,2-b]pyrrol-1-carbonyl]-3-methyl-butyl}-4-morpholin-4-yl-benzamid,
N-{1-[4-(2-Methoxy-4,4-dimethyl-pentanoyl)-6-oxo-hexahydro-pyrrolo[3,2-b]pyrrol-1-carbonyl]-3-methyl-butyl}-4-morpholin-4-yl-benzamid,
N-{1-[4-(2-Hydroxy-pentanoyl)-6-oxo-hexahydro-pyrrolo[3,2-b]pyrrol-1-carbonyl]-3-methyl-butyl}-4-morpholin-4-yl-benzamid,
N-{1-[4-(2-Methoxy-pentanoyl)-6-oxo-hexahydro-pyrrolo[3,2-b]pyrrol-1-carbonyl]-3-methyl-butyl}-4-morpholin-4-yl-benzamid,
N-{1-[4-(2-Hydroxy-5-methyl-hexanoyl)-6-oxo-hexahydro-pyrrolo[3,2-b]pyrrol-1-carbonyl]-3-methyl-butyl}-4-morpholin-4-yl-benzamid,
N-{1-[4-(2-Methoxy-5-methyl-hexanoyl)-6-oxo-hexahydro-pyrrolo[3,2-b]pyrrol-1-carbonyl]-3-methyl-butyl}-4-morpholin-4-yl-benzamid,
N-[1-(4-Benzoyl-6-oxo-hexahydro-pyrrolo[3,2-b]pyrrol-1-carbonyl)-3-methyl-butyl]-4-piperazin-1-yl-benzamid,
N-{3-Methyl-1-[6-oxo-4-(pyridin-2-carbonyl)-hexahydro-pyrrolo[3,2-b]pyrrol-1-carbonyl]-butyl}-4-piperazin-1-yl-benzamid,
N-{3-Methyl-1-[6-oxo-(1-oxy-pyridin-2-carbonyl)-hexahydro-pyrrolo[3,2-b]pyrrol-1-carbonyl]-butyl}-4-piperazin-1-yl-benzamid,
N-{3-Methyl-1-[6-oxo-4-(pyridin-3-carbonyl)-hexahydro-pyrrolo[3,2-b]pyrrol-1-carbonyl]-butyl}-4-piperazin-1-yl-benzamid,
N-{3-Methyl-1-[6-oxo-4-(1-oxy-pyridin-3-carbonyl)-hexahydro-pyrrolo[3,2-b]pyrrol-1-carbonyl]-butyl}-4-piperazin-1-yl-benzamid,
N-{3-Methyl-1-[6-oxo-4-(pyridin-4-carbonyl)-hexahydro-pyrrolo[3,2-b]pyrrol-1-carbonyl]-butyl}-4-piperazin-1-yl-benzamid,

N-{3-Methyl-1-[6-oxo-4-(1-oxy-pyridin-4-carbonyl)-hexahydro-pyrrolo[3,2-b]pyrrol-1-carbonyl]-butyl}-4-piperazin-1-yl-benzamid,
N-[1-(4-Benzensulfonyl-6-oxo-hexahydro-pyrrolo[3,2-b]pyrrol-1-carbonyl)-3-methyl-butyl]-4-piperazin-1-yl-benzamid,
N-{3-Methyl-1-[6-oxo-4-(pyridin-2-sulfonyl)-hexahydro-pyrrolo[3,2-b]pyrrol-1-carbonyl]-butyl}-4-piperazin-1-yl-benzamid,
N-{3-Methyl-1-[6-oxo-4-(1-oxy-pyridin-2-sulfonyl)-hexahydro-pyrrolo[3,2-b]pyrrol-1-carbonyl]-butyl}-4-piperazin-1-yl-benzamid,
N-{3-Methyl-1-[6-oxo-4-(pyridin-3-sulfonyl)-hexahydro-pyrrolo[3,2-b]pyrrol-1-carbonyl]-butyl}-4-piperazin-1-yl-benzamid,
N-{3-Methyl-1-[-oxo-4-(1-oxy-pyridin-3-sulfonyl)-hexahydro-pyrrolo[3,2-b]pyrrol-1-carbonyl]-butyl}-4-piperazin-1-yl-benzamid,
N-{3-Methyl-1-[6-oxo-4-(pyridin-4-sulfonyl)-hexahydro-pyrrolo[3,2-b]pyrrol-1-carbonyl]-butyl}-4-piperazin-1-yl-benzamid,
N-{3-Methyl-1-[6-oxo-4-(1-oxy-pyridin-4-sulfonyl)-hexahydro-pyrrolo[3,2-b]pyrrol-1-carbonyl]-butyl}-4-piperazin-1-yl-benzamid,
N-[3-Methyl-1-(6-oxo-4-phenylacetyl-hexahydro-pyrrolo[3,2-b]pyrrol-1-carbonyl)-butyl]-4-piperazin-1-yl-benzamid,
4-[2-(4-Piperazin-1-yl-benzoylamino)-4-methyl-pentanoyl]-3-oxo-hexahydro-pyrrolo[3,2-b]pyrrol-1-carbonsäurephenylamid,
4-[2-(4-Piperazin-1-yl-benzoylamino)-4-methyl-pentanoyl]-3-oxo-hexahydro-pyrrolo[3,2-b]pyrrol-1-carbonsäureisobutylamid,
4-[2-(4-Piperazin-1-yl-benzoylamino)-4-methyl-pentanoyl]-3-oxo-hexahydro-pyrrolo[3,2-b]pyrrol-1-carbonsäurephenylester,
4-[2-(4-Piperazin-1-yl-benzoylamino)-4-methyl-pentanoyl]-3-oxo-hexahydro-pyrrolo[3,2-b]pyrrol-1-carbonsäure-isobutylester,
N-{3-Methyl-1-[6-oxo-4-(pyrrolidin-1-carbonyl)-hexahydro-pyrrolo[3,2-b]pyrrol-1-carbonyl]-butyl}-4-piperazin-1-yl-benzamid,
N-{3-Methyl-1-[6-oxo-4-(piperidin-1-carbonyl)-hexahydro-pyrrolo[3,2-b]pyrrol-1-carbonyl]-butyl}-4-piperazin-1-yl-benzamid,
N-{3-Methyl-1-[4-(morpholin-4-carbonyl)-6-oxo-hexahydro-pyrrolo[3,2-b]pyrrol-1-carbonyl]-butyl}-4-piperazin-1-yl-benzamid,
N-{3-Methyl-1-[6-oxo-4-(piperazin-1-carbonyl)-hexahydro-pyrrolo[3,2-b]pyrrol-1-carbonyl]-butyl}-4-piperazin-1-yl-benzamid,
N-[3-Methyl-1-[4-(4-methyl-piperazin-1-carbonyl)-6-oxo-hexahydro-pyrrolo[3,2-b]pyrrol-1-carbonyl]-butyl}-4-piperazin-1-yl-benzamid,
N-(3-Methyl-1-{4-[2-(4-methyl-piperazin-1-yl)-acetyl]-6-oxo-hexahydro-pyrrolo[3,2-b]pyrrol-1-carbonyl}-butyl)-4-piperazin-1-yl-benzamid,
N-{3-Methyl-1-[6-oxo-4-(2-piperazin-1-yl-acetyl)-hexahydro-pyrrolo[3,2-b]pyrrol-1-carbonyl]-butyl}-4-piperazin-1-yl-benzamid,
N-{1-[4-(Furan-2-carbonyl)-6-oxo-hexahydro-pyrrolo[3,2-b]pyrrol-1-carbonyl]-butyl}-4-piperazin-1-yl-benzamid,
N-{1-[4-(5-Chlor-furan-2-carbonyl)-6-oxo-hexahydro-pyrrolo[3,2-b]pyrrol-1-carbonyl]-3-methyl-butyl}-4-piperazin-1-yl-benzamid,
N-{1-[4-(Thiophen-2-carbonyl)-6-oxo-hexahydro-pyrrolo[3,2-b]pyrrol-1-carbonyl]-3-methyl-butyl}-4-piperazin-1-yl-benzamid,
N-{1-[4-(5-Chlor-thiophen-2-carbonyl)-6-oxo-hexahydro-pyrrolo[3,2-b]pyrrol-1-carbonyl]-3-methyl-butyl}-4-piperazin-1-yl-benzamid,
N-{1-[4-(Furan-3-carbonyl)-6-oxo-hexahydro-pyrrolo[3,2-b]pyrrol-1-carbonyl]-3-methyl-butyl}-4-piperazin-1-yl-benzamid,
N-{1-[4-(Thiophen-3-carbonyl)-6-oxo-hexahydro-pyrrolo[3,2-b]pyrrol-1-carbonyl]-3-methyl-butyl}-4-piperazin-1-yl-benzamid,
N-[1-(4-Cyclopentancarbonyl-6-oxo-hexahydro-pyrrolo[3,2-b]pyrrol-1-carbonyl)-3-methyl-butyl}]4-piperazin-1-yl-benzamid,
N-[1-(4-Cyclohexancarbonyl-6-oxo-hexahydro-pyrrolo[3,2-b]pyrrol-1-carbonyl)-3-methyl-butyl]-4-piperazin-1-yl-benzamid,
N-{1-[4-(Cyclopent-3-encarbonyl)-6-oxo-hexahydro-pyrrolo[3,2-b]pyrrol-1-carbonyl]-3-methyl-butyl}-4-piperazin-1-yl-benzamid,
N-{1-[4-(2-Cyclopentyl-acetyl)-6-oxo-hexahydro-pyrrolo[3,2-b]pyrrol-1-carbonyl]-3-methyl-butyl}-4-piperazin-1-yl-benzamid,
N-{1-[4-(2-Cyclohexyl-acetyl)-6-oxo-hexahydro-pyrrolo[3,2-b]pyrrol-1-carbonyl]-3-methyl-butyl}-4-piperazin-1-yl-benzamid,
4-Piperazin-1-yl-N-{3-methyl-1-[-oxo-4-(pyrrolidin-2-carbonyl)-hexahydro-pyrrolo[3,2-b]pyrrol-1-carbonyl]-butyl}-benzamid,
N-{1-[4-(1-Acetyl-pyrrolidin-2-carbonyl)-6-oxo-hexahydro-pyrrolo[3,2-b]pyrrol-1-carbonyl]-3-methyl-butyl}-4-piperazin-1-yl-benzamid,
N-{3-Methyl-1-[6-oxo-4-(piperidin-2-carbonyl)-hexahydro-pyrrolo[3,2-b]pyrrol-1-carbonyl]-butyl}-4-piperazin-1-yl-benzamid,
N-{1-[4-(1-Acetyl-piperidin-2-carbonyl)-6-oxo-hexahydro-pyrrolo[3,2-b]pyrrol-1-carbonyl]-3-methyl-butyl}-4-piperazin-1-yl-benzamid,
N-{3-Methyl-1-[6-oxo-4-(tetrahydro-furan-2-carbonyl)-hexahydro-pyrrolo[3,2-b]pyrrol-1-carbonyl]-butyl}-4-piperazin-1-yl-benzamid,
N-{1-[4-(1-Amino-cyclobutancarbonyl)-6-oxo-hexahydro-pyrrolo[3,2-b]pyrrol-1-carbonyl]-3-methyl-butyl}-4-piperazin-1-yl-benzamid,
N-{1-[4-(1-Amino-cyclopentancarbonyl)-6-oxo-hexahydro-pyrrolo[3,2-b]pyrrol-1-carbonyl]-3-methyl-butyl}-4-piperazin-1-yl-benzamid,
N-{1-[4-(1-Amino-cyclohexancarbonyl)-6-oxo-hexahydro-pyrrolo[3,2-b]pyrrol-1-carbonyl]-3-methyl-butyl}-4-piperazin-1-yl-benzamid,
N-{1-[4-(1-Acetylamino-cyclobutancarbonyl)-6-oxo-hexahydro-pyrrolo[3,2-b]pyrrol-1-carbonyl]-3-methyl-butyl}-4-piperazin-1-yl-benzamid,
N-{1-[4-(1-Acetylamino-cyclopentancarbonyl)-6-oxo-hexahydro-pyrrolo[3,2-b]pyrrol-1-carbonyl]-3-methyl-butyl}-4-piperazin-1-yl-benzamid,
N-{1-[4-(1-Acetylamino-cyclohexancarbonyl)-6-oxo-hexahydro-pyrrolo[3,2-b]pyrrol-1-carbonyl]-3-methyl-butyl}-4-piperazin-1-yl-benzamid,
N-{1-[4-(1-Hydroxy-cyclobutancarbonyl)-6-oxo-hexahydro-pyrrolo[3,2-b]pyrrol-1-carbonyl]-3-methyl-butyl}-4-piperazin-1-yl-benzamid,
N-{1-[4-(1-Hydroxy-cyclopentancarbonyl)-6-oxo-hexahydro-pyrrolo[3,2-b]pyrrol-1-carbonyl]-3-methyl-butyl}-4-piperazin-1-yl-benzamid,
N-{1-[4-(1-Hydroxy-cyclohexancarbonyl)-6-oxo-hexahydro-pyrrolo[3,2-b]pyrrol-1-carbonyl]-3-methyl-butyl}-4-piperazin-1-yl-benzamid,
N-{1-[4-(1-Methoxy-cyclobutancarbonyl)-6-oxo-hexahydro-pyrrolo[3,2-b]pyrrol-1-carbonyl]-3-methyl-butyl}-4-piperazin-1-yl-benzamid,
N-{1-[4-(1-Methoxy-cyclopentancarbonyl)-6-oxo-hexahydro-pyrrolo[3,2-b]pyrrol-1-carbonyl]-3-methyl-butyl}-4-piperazin-1-yl-benzamid,
N-{1-[4-(1-Methoxy-cyclohexancarbonyl)-6-oxo-hexahydro-pyrrolo[3,2-b]pyrrol-1-carbonyl]-3-methyl-butyl}-4-piperazin-1-yl-benzamid,
N-{1-[4-(2-Amino-2-cyclopentyl-acetyl)-6-oxo-hexahydro-pyrrolo[3,2-b]pyrrol-1-carbonyl]-3-methyl-butyl}-4-piperazin-1-yl-benzamid,
N-{1-[4-(2-Amino-2-cyclohexyl-acetyl)-6-oxo-hexahydro-pyrrolo[3,2-b]pyrrol-1-carbonyl]-3-methyl-butyl}-4-piperazin-1-yl-benzamid,
N-{1-[4-(2-Acetylamino-2-cyclopentyl-acetyl)-6-oxo-hexahydro-pyrrolo[3,2-b]pyrrol-1-carbonyl]-3-methyl-butyl}-4-piperazin-1-yl-benzamid,
N-{1-[4-(2-Acetylamino-2-cyclohexyl-acetyl)-6-oxo-hexahydro-pyrrolo[3,2-b]pyrrol-1-carbonyl]-3-methyl-butyl}-4-piperazin-1-yl-benzamid,
N-{1-[4-(2-Acetylamino-propionyl)-6-oxo-hexahydro-pyrrolo[3,2-b]pyrrol-1-carbonyl]-3-methyl-butyl}-4-piperazin-1-yl-benzamid,
N-{1-[4-(2-Acetylamino-acetyl)-6-oxo-hexahydro-pyrrolo[3,2-b]pyrrol-1-carbonyl]-3-methyl-butyl}-4-piperazin-1-yl-benzamid,
N-(1-{4-[2-(Acetyl-methyl-amino)-acetyl]-6-oxo-hexahydro-pyrrolo[3,2-b]pyrrol-1-carbonyl}-3-methyl-butyl)-4-piperazin-1-yl-benzamid,
N-{1-[4-(2-Dimethylamino-acetyl)-6-oxo-hexahydro-pyrrolo[3,2-b]pyrrol-1-carbonyl]-3-methyl-butyl}-4-piperazin-1-yl-benzamid,
N-1-[4-(2-Amino-butyryl)-6-oxo-hexahydro-pyrrolo[3,2-b]pyrrol-1-carbonyl]-3-methyl-butyl}-4-piperazin-1-yl-benzamid,
N-{1-[4-(2-Acetylamino-butyryl)-6-oxo-hexahydro-pyrrolo[3,2-b]pyrrol-1-carbonyl]-3-methyl-butyl}-4-piperazin-1-yl-benzamid,
N-1-[4-(2-Amino-3-methyl-butyryl)-6-oxo-hexahydro-pyrrolo[3,2-b]pyrrol-1-carbonyl]-3-methyl-butyl}-4-piperazin-1-yl-benzamid,
N-{1-[4-(2-Acetylamino-3-methyl-butyryl)-6-oxo-hexahydro-pyrrolo[3,2-b]pyrrol-1-carbonyl]-3-methyl-butyl}-4-piperazin-1-yl-benzamid,

N-{1-[4-(2-Amino-3,3-dimethyl-butyryl)-6-oxo-hexahydro-pyrrolo[3,2-b]pyrrol-1-carbonyl]-3-methyl-butyl}-4-piperazin-1-yl-benzamid,
N-{1-[4-(2-Acetylamino-3,3-dimethyl-butyryl)-6-oxo-hexahydro-pyrrolo[3,2-b]pyrrol-1-carbonyl]-3-methyl-butyl}-4-piperazin-1-yl-benzamid,
N-{1-[4-(2-Amino-3-methyl-pentanoyl)-6-oxo-hexahydro-pyrrolo[3,2-b]pyrrol-1-carbonyl]-3-methyl-butyl}-4-piperazin-1-yl-benzamid,
N-{1-[4-(2-Acetylamino-3-methyl-pentanoyl)-6-oxo-hexahydro-pyrrolo[3,2-b]pyrrol-1-carbonyl]-3-methyl-butyl}-4-piperazin-1-yl-benzamid,
N-[3-Methyl-1-(6-oxo-4-pentanoyl-hexahydro-pyrrolo[3,2-b]pyrrol-1-carbonyl)-butyl]-4-piperazin-1-yl-benzamid,
N-{3-Methyl-1-[4-(3-methyl-pentanoyl)-6-oxo-hexahydro-pyrrolo[3,2-b]pyrrol-1-carbonyl]-butyl}-4-piperazin-1-yl-benzamid,
N-{3-Methyl-1-[4-(4-methyl-pentanoyl)-6-oxo-hexahydro-pyrrolo[3,2-b]pyrrol-1-carbonyl]-butyl}-4-piperazin-1-yl-benzamid,
N-{1-[4-(2-Amino-4-methyl-pentanoyl)-6-oxo-hexahydro-pyrrolo[3,2-b]pyrrol-1-carbonyl]-3-methyl-butyl}-4-piperazin-1-yl-benzamid,
N-{1-[4-(2-Acetylamino-4-methyl-pentanoyl)-6-oxo-hexahydro-pyrrolo[3,2-b]pyrrol-1-carbonyl]-3-methyl-butyl}-4-piperazin-1-yl-benzamid,
N-{1-[4-(2-Amino-4,4-dimethyl-pentanoyl)-6-oxo-hexahydro-pyrrolo[3,2-b]pyrrol-1-carbonyl]-3-methyl-butyl}-4-piperazin-1-yl-benzamid,
N-{1-[4-(2-Acetylamino-4,4-dimethyl-pentanoyl)-6-oxo-hexahydro-pyrrolo[3,2-b]pyrrol-1-carbonyl]-3-methyl-butyl}-4-piperazin-1-yl-benzamid,
N-{1-[4-(2-Amino-pentanoyl)-6-oxo-hexahydro-pyrrolo[3,2-b]pyrrol-1-carbonyl]-3-methyl-butyl}-4-piperazin-1-yl-benzamid,
N-{1-[4-(2-Acetylamino-pentanoyl)-6-oxo-hexahydro-pyrrolo[3,2-b]pyrrol-1-carbonyl]-3-methyl-butyl}-4-piperazin-1-yl-benzamid,
N-{1-[4-(2-Amino-5-methyl-hexanoyl)-6-oxo-hexahydro-pyrrolo[3,2-b]pyrrol-1-carbonyl]-3-methyl-butyl}-4-piperazin-1-yl-benzamid,
N-{1-[4-(2-Acetylamino-5-methyl-hexanoyl)-6-oxo-hexahydro-pyrrolo[3,2-b]pyrrol-1-carbonyl]-3-methyl-butyl}-4-piperazin-1-yl-benzamid,
N-{1-[4-(2-Hydroxy-3-methyl-butyryl)-6-oxo-hexahydro-pyrrolo[3,2-b]pyrrol-1-carbonyl]-3-methyl-butyl}-4-piperazin-1-yl-benzamid,
N-{1-[4-(2-Methoxy-3-methyl-butyryl)-6-oxo-hexahydro-pyrrolo[3,2-b]pyrrol-1-carbonyl]-3-methyl-butyl}-4-piperazin-1-yl-benzamid,
N-{1-[4-(2-Hydroxy-3,3-dimethyl-butyryl)-6-oxo-hexahydro-pyrrolo[3,2-b]pyrrol-1-carbonyl]-3-methyl-butyl}-4-piperazin-1-yl-benzamid,
N-{1-[4-(2-Methoxy-3,3-dimethyl-butyryl)-6-oxo-hexahydro-pyrrolo[3,2-b]pyrrol-1-carbonyl]-3-methyl-butyl}-4-piperazin-1-yl-benzamid,
N-{1-[4-(2-Hydroxy-3-methyl-pentanoyl)-6-oxo-hexahydro-pyrrolo[3,2-b]pyrrol-1-carbonyl]-3-methyl-butyl}-4-piperazin-1-yl-benzamid,
N-{1-[4-(2-Methoxy-3-methyl-pentanoyl)-6-oxo-hexahydro-pyrrolo[3,2-b]pyrrol-1-carbonyl]-3-methyl-butyl}-4-piperazin-1-yl-benzamid,
N-{1-[4-(2-Hydroxy-4-methyl-pentanoyl)-6-oxo-hexahydro-pyrrolo[3,2-b]pyrrol-1-carbonyl]-3-methyl-butyl}-4-piperazin-1-yl-benzamid,
N-{1-[4-(2-Methoxy-4-methyl-pentanoyl)-6-oxo-hexahydro-pyrrolo[3,2-b]pyrrol-1-carbonyl]-3-methyl-butyl}-4-piperazin-1-yl-benzamid,
N-{1-[4-(2-Hydroxy-4,4-dimethyl-pentanoyl)-6-oxo-hexahydro-pyrrolo[3,2-b]pyrrol-1-carbonyl]-3-methyl-butyl}-4-piperazin-1-yl-benzamid,
N-{1-[4-(2-Methoxy-4,4-dimethyl-pentanoyl)-6-oxo-hexahydro-pyrrolo[3,2-b]pyrrol-1-carbonyl]-3-methyl-butyl}-4-piperazin-1-yl-benzamid,
N-{1-[4-(2-Hydroxy-pentanoyl)-6-oxo-hexahydro-pyrrolo[3,2-b]pyrrol-1-carbonyl]-3-methyl-butyl}-4-piperazin-1-yl-benzamid,
N-{1-[4-(2-Methoxy-pentanoyl)-6-oxo-hexahydro-pyrrolo[3,2-b]pyrrol-1-carbonyl]-3-methyl-butyl}-4-piperazin-1-yl-benzamid,
N-{1-[4-(2-Hydroxy-5-methyl-hexanoyl)-6-oxo-hexahydro-pyrrolo[3,2-b]pyrrol-1-carbonyl]-3-methyl-butyl}-4-piperazin-1-yl-benzamid,
N-{1-[4-(2-Methoxy-5-methyl-hexanoyl)-6-oxo-hexahydro-pyrrolo[3,2-b]pyrrol-1-carbonyl]-3-methyl-butyl}-4-piperazin-1-yl-benzamid,
N-[1-(4-Benzoyl-6-oxo-hexahydro-pyrrolo[3,2-b]pyrrol-1-carbonyl)-3-methyl-butyl]-4-(4-methylpiperazin-1-yl)-benzamid,
N-{3-Methyl-1-[6-oxo-4-(1-oxy-pyridin-2-carbonyl)-hexahydro-pyrrolo[3,2-b]pyrrol-1-carbonyl]-butyl}-4-(4-methyl-piperazin-1-yl)-benzamid,
N-{3-Methyl-1-[6-oxo-4-(pyridin-3-carbonyl)-hexahydro-pyrrolo[3,2-b]pyrrol-1-carbonyl]-butyl}-4-(4-methyl-piperazin-1-yl)-benzamid,
N-{3-Methyl-1-[6-oxo-4-(1-oxy-pyridin-3-carbonyl)-hexahydro-pyrrolo[3,2-b]pyrrol-1-carbonyl]-butyl}-4-(4-methyl-piperazin-1-yl)-benzamid,
N-{3-Methyl-1-[6-oxo-4-(pyridin-4-carbonyl)-hexahydro-pyrrolo[3,2-b]pyrrol-1-carbonyl]-butyl}-4-(4-methyl-piperazin-1-yl)-benzamid,
N-{3-Methyl-1-[6-oxo-4-(1-oxy-pyridin-4-carbonyl)-hexahydro-pyrrolo[3,2-b]pyrrol-1-carbonyl]-butyl}-4-(4-methyl-piperazin-1-yl)-benzamid,
N-[1-(4-Benzensulfonyl-6-oxo-hexahydro-pyrrolo[3,2-b]pyrrol-1-carbonyl)-3-methyl-butyl]-4-(4-methyl-piperazin-1-yl)-benzamid,
N-{3-Methyl-1-[6-oxo-4-(pyridin-4-sulfonyl)-hexahydro-pyrrolo[3,2-b]pyrrol-1-carbonyl]-butyl}-4-(4-methyl-piperazin-1-yl)-benzamid,
N-{3-Methyl-1-[6-oxo-4-(1-oxy-pyridin-4-sulfonyl)-hexahydro-pyrrolo[3,2-b]pyrrol-1-carbonyl]-butyl}-4-(4-methyl-piperazin-1-yl)-benzamid,
N-[3-Methyl-1-(6-oxo-4-phenylacetyl-hexahydro-pyrrolo[3,2-b]pyrrol-1-carbonyl)-butyl]-4-(4-methyl-piperazin-1-yl)-benzamid,
4-[2-(4-(4-Methyl-piperazin-1-yl)-benzoylamino)-4-methyl-pentanoyl]-3-oxo-hexahydro-pyrrolo[3,2-b]pyrrol-1-carbonsäurephenylamid,
4-[2-(4-(4-Methyl-piperazin-1-yl)-benzoylamino)-4-methyl-pentanoyl]-3-oxo-hexahydro-pyrrolo[3,2-b]pyrrol-1-carbonsäureisobutylamid,
4-[2-(4-(4-Methyl-piperazin-1-yl)-benzoylamino)-4-methyl-pentanoyl]-3-oxo-hexahydro-pyrrolo[3,2-b]pyrrol-1-carbonsäurephenylester,
4-[2-(4-(4-Methyl-piperazin-1-yl)-benzoylamino)-4-methyl-pentanoyl]-3-oxo-hexahydro-pyrrolo[3,2-b]pyrrol-1-carbonsäureisobutylester,
N-{3-Methyl-1-[6-oxo-4-(pyrrolidin-1-carbonyl)-hexahydro-pyrrolo[3,2-b]pyrrol-1-carbonyl]-butyl}-4-(4-methyl-piperazin-1-yl)-benzamid,
N-{3-Methyl-1-[6-oxo-4-(piperidin-1-carbonyl)-hexahydro-pyrrolo[3,2-b]pyrrol-1-carbonyl]-butyl}-4-(4-methyl-piperazin-1-yl)-benzamid,
N-{3-Methyl-1-[4-(morpholin-4-carbonyl)-6-oxo-hexahydro-pyrrolo[3,2-b]pyrrol-1-carbonyl]-butyl}-4-(4-methyl-piperazin-1-yl)-benzamid,
N-{3-Methyl-1-[6-oxo-4-(piperazin-1-carbonyl)-hexahydro-pyrrolo[3,2-b]pyrrol-1-carbonyl]-butyl}-4-(4-methyl-piperazin-1-yl)-benzamid,
N-{3-Methyl-1-[4-(4-methyl-piperazin-1-carbonyl)-6-oxo-hexahydro-pyrrolo[3,2-b]pyrrol-1-carbonyl]-butyl}-4-(4-methyl-piperazin-1-yl)-benzamid,
N-(3-Methyl-1-{4-[2-(4-methyl-piperazin-1-yl)-acetyl]-6-oxo-hexahydro-pyrrolo[3,2-b]pyrrol-1-carbonyl}-butyl)-4-(4-methyl-piperazin-1-yl)-benzamid,
N-{3-Methyl-1-[6-oxo-4-(2-piperazin-1-yl-acetyl)-hexahydro-pyrrolo[3,2-b]pyrrol-1-carbonyl]-butyl}-4-(4-methyl-piperazin-1-yl)-benzamid,
N-{1-[4-(Furan-2-carbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(5-Chlor-furan-2-carbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(Thiophen-2-carbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(5-Chlor-thiophen-2-carbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(Furan-3-carbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(Thiophen-3-carbonyl)-6-oxo-hexahydro-pyrrolo[3,2-b]pyrrol-1-carbonyl]-3-methyl-butyl}-4-(4-methyl-piperazin-1-yl)-benzamid,
N-[1-(4-Cyclopentancarbonyl-6-oxo-hexahydro-pyrrolo[3,2-b]pyrrol-1-carbonyl)-3-methyl-butyl]-4-(4-methyl-piperazin-1-yl)-benzamid,
N-[1-(4-Cyclohexancarbonyl-6-oxo-hexahydro-pyrrolo[3,2-b]pyrrol-1-carbonyl)-3-methyl-butyl]-4-(4-methyl-piperazin-1-yl)-benzamid,
N-{1-[4-(Cyclopent-3-encarbonyl)-6-oxo-hexahydro-pyrrolo[3,2-b]pyrrol-1-carbonyl]-3-methyl-butyl}--4-(4-methyl-piperazin-1-yl)-benzamid,
N-{1-[4-(2-Cyclopentyl-acetyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Cyclohexyl-acetyl)-6-oxo-hexahydro-pyrrolo[3,2-b]pyrrol-1-carbonyl]-3-methyl-butyl}-4-(4-methyl-piperazin-1-yl)-benzamid,
4-(4-Methyl-piperazin-1-yl)-N-{3-methyl-1-[6-oxo-4-(pyrrolidin-2-carbonyl)-hexahydro-pyrrolo[3,2-b]pyrrol-1-carbonyl]-butyl}-benzamid,
N-{1-[4-(1-Acetyl-pyrrolidin-2-carbonyl)-6-oxo-hexahydro-pyrrolo[3,2-b]pyrrol-1-carbonyl]-3-methyl-butyl}-4-(4-methyl-piperazin-1-yl)-benzamid,
N-{3-Methyl-1-[6-oxo-4-(piperidin-2-carbonyl)-hexahydro-pyrrolo[3,2-b]pyrrol-1-carbonyl]-butyl}-4-(4-methyl-piperazin-1-yl)-benzamid,
N-{1-[4-(1-Acetyl-piperidin-2-carbonyl)-6-oxo-hexahydro-pyrrolo[3,2-b]pyrrol-1-carbonyl]-3-methyl-butyl}-4-(4-methyl-piperazin-1-yl)-benzamid,
N-{3-Methyl-1-[6-oxo-4-(tetrahydro-furan-2-carbonyl)-hexahydro-pyrrolo[3,2-b]pyrrol-1-carbonyl]-butyl}-4-(4-methyl-piperazin-1-yl)-benzamid,
N-{1-[4-(1-Amino-cyclobutancarbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(1-Amino-cyclopentancarbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(1-Amino-cyclohexancarbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(1-Acetylamino-cyclobutancarbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(1-Acetylamino-cyclopentancarbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(1-Acetylamino-cyclohexancarbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(1-Hydroxy-cyclobutancarbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(1-Hydroxy-cyclopentancarbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(1-Hydroxy-cyclohexancarbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(1-Methoxy-cyclobutancarbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(1-Methoxy-cyclopentancarbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(1-Methoxy-cyclohexancarbonyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Amino-2-cyclopentyl-acetyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Amino-2-cyclohexyl-acetyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Acetylamino-2-cyclopentyl-acetyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Acetylamino-2-cyclohexyl-acetyl)-6-oxo-hexahydro-pyrrolo[3,2-b]pyrrol-1-carbonyl]-3-methyl-buty}-4-(4-methyl-piperazin-1-yl)-benzamid,
N-{1-[4-(2-Acetylamino-propionyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Acetylamino-acetyl)-6-oxo-hexahydro-pyrrolo[3,2-b]pyrrol-1-carbonyl]-3-methyl-butyl}-4-(4-methyl-piperazin-1-yl)-benzamid,
N-(1-{4-[2-(Acetyl-methyl-amino)-acetyl]-6-oxo-hexahydro-pyrrolo[3,2-b]pyrrol-1-carbonyl}-3-methyl-butyl)-4-(4-methyl-piperazin-1-yl)-benzamid,
N-{1-[4-(2-Dimethylamino-acetyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Amino-butyryl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Acetylamino-butyryl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Amino-3-methyl-butyryl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Acetylamino-3-methyl-butyryl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Amino-3,3-dimethyl-butyryl)-6-oxo-hexahydro-pyrrolo[3,2b]pyrrol-1-carbonyl]-3-methyl-butyl}-4-(4-methyl-piperazin-1-yl)-benzamid,
N-{1-[4-(2-Acetylamino-3,3-dimethyl-butyryl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Amino-3-methyl-pentanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Acetylamino-3-methyl-pentanoyl)-6-oxo-hexahydro-pyrrolo[3,2-b]pyrrol-1-carbonyl]-3-methyl-butyl}-4-(4-methyl-piperazin-1-yl)-benzamid,
N-[3-Methyl-1-(6-oxo-4-pentanoyl-hexahydro-pyrrolo[3,2-b]pyrrol-1-carbonyl)-butyl]-4-(4-methylpiperazin-1-yl)-benzamid,
N-{3-Methyl-1-[4-(3-methyl-pentanoyl)-6-oxo-hexahydro-pyrrolo[3,2-b]pyrrol-1-carbonyl]-butyl}-4-(4-methyl-piperazin-1-yl)-benzamid,
N-{3-Methyl-1-[4-(4-methyl-pentanoyl)-6-oxo-hexahydro-pyrrolo[3,2-b]pyrrol-1-carbonyl]-butyl}-4-(4-methyl-piperazin-1-yl)-benzamid,
N-{1-[4-(2-Amino-4-methyl-pentanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Acetylamino-4-methyl-pentanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Amino-4,4-dimethyl-pentanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Acetylamino-4,4-dimethyl-pentanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Amino-pentanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Acetylamino-pentanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Amino-5-methyl-hexanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Acetylamino-5-methyl-hexanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Hydroxy-3-methyl-butyryl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Methoxy-3-methyl-butyryl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Hydroxy-3,3-dimethyl-butyryl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Methoxy-3,3-dimethyl-butyryl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Hydroxy-3-methyl-pentanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Methoxy-3-methyl-pentanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Hydroxy-4-methyl-pentanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Methoy-4-methyl-pentanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Hydroxy-4,4-dimethyl-pentanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Methoxy-4,4-dimethyl-pentanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Hydroxy-pentanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Methoxy-pentanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Hydroxy-5-methyl-hexanoyl)-6-oxo-hexahydro-pyrrolo[3,2-b]pyrrol-1-carbonyl]-3-methyl-butyl}-4-(4-methyl-piperazin-1-yl)-benzamid,
N-{1-[4-(2-Methoxy-5-methyl-hexanoyl)-6-oxo-hexahydro-pyrrolo[3,2-b]pyrrol-1-carbonyl]-3-methyl-butyl}-4-(4-methyl-piperazin-1-yl)-benzamid,
N-[1-(4-Benzoyl-6-oxo-hexahydro-pyrrolo[3,2-b]pyrrol-1-carbonyl)-3-methyl-butyl]-4-(4-methylpiperazin-1-yl)-benzamid,
4-Amino-N-{3-methyl-1-[-oxo-4-(pyridin-2-carbonyl)-hexahydro-pyrrolo[3,2-b]pyrrol-1-carbonyl]-butyl}-benzamid,
4-Amino-N-{3-methyl-1-[6-oxo-4-(1-oxy-pyridin-2-carbonyl)-hexahydro-pyrrolo[3,2-b]pyrrol-1-carbonyl]-butyl}-benzamid,
4-Amino-N-{3-methyl-1-[6-oxo-4-(pyridin-3-carbonyl)-hexahydro-pyrrolo[3,2-b]pyrrol-1-carbonyl]-butyl}-benzamid,
4-Amino-N-{3-methyl-1-[6-oxo-4-(1-oxy-pyridin-3-carbonyl)-hexahydro-pyrrolo[3,2-b]pyrrol-1-carbonyl]-butyl}-benzamid,
4-Amino-N-{3-methyl-1-[6-oxo-4-(pyridin-4-carbonyl)-hexahydro-pyrrolo[3,2-b]pyrrol-1-carbonyl]-butyl}-benzamid,
4-Amino-N-{3-methyl-1-[6-oxo-4-(1-oxy-pyridin-4-carbonyl)-hexahydro-pyrrolo[3,2-b]pyrrol-1-carbonyl]-butyl}-benzamid,
N-[1-(4-Benzensulfonyl-6-oxo-hexahydro-pyrrolo[3,2-b]pyrrol-1-carbonyl)-3-methyl-butyl]-4-amino-benzamid,
4-Amino-N-{3-methyl-1-[6-oxo-4-(pyridin-2-sulfonyl)-hexahydro-pyrrolo[3,2-b]pyrrol-1-carbonyl]-butyl}-benzamid,
4-Amino-N-{3-methyl-1-[6-oxo-4-(1-oxy-pyridin-2-sulfonyl)-hexahydro-pyrrolo[3,2-b]pyrrol-1-carbonyl]-butyl}-benzamid,
4-Amino-N-{3-methyl-1-[6-oxo-4-(pyridin-3-sulfonyl)-hexahydro-pyrrolo[3,2-b]pyrrol-1-carbonyl]-butyl}-benzamid,
4-Amino-N-{3-methyl-1-[6-oxo-4-(1-oxy-pyridin-3-sulfonyl)-hexahydro-pyrrolo[3,2-b]pyrrol-1-carbonyl]-butyl}-benzamid,
4-Amino-N-{3-methyl-1-[6-oxo-4-(pyridin-4-sulfonyl)-hexahydro-pyrrolo[3,2-b]pyrrol-1-carbonyl]-butyl}-benzamid,
4-Amino-N-{3-methyl-1-[6-oxo-4-(1-oxy-pyridin-4-sulfonyl)-hexahydro-pyrrolo[3,2-b]pyrrol-1-carbonyl]-butyl}-benzamid,
4-Amino-N-[3-methyl-1-(6-oxo-4-phenylacetyl-hexahydro-pyrrolo[3,2-b]pyrrol-1-carbonyl)-butyl]-benzamid,
4-[2-(4-Amino-benzoylamino)-4-methyl-pentanoyl]-3-oxo-hexahydro-pyrrolo[3,2-b]pyrrol-1-carbonsäurephenylamid,
4-[2-(4-Amino-benzoylamino)-4-methyl-pentanoyl]-3-oxo-hexahydro-pyrrolo[3,2-b]pyrrol-1-carbonsäureisobutylamid,
4-[2-(4-Amino-benzoylamino)-4-methyl-pentanoyl]-3-oxo-hexahydro-pyrrolo[3,2-b]pyrrol-1-carbonsäurephenylester,
4-[2-(4-Amino-benzoylamino)-4-methyl-pentanoyl]-3-oxo-hexahydro-pyrrolo[3,2-b]pyrrol-1-carbonsäureisobutylester,
4-Amino-N-{3-methyl-1-[6-oxo-4-(pyrrolidin-1-carbonyl)-hexahydro-pyrrolo[3,2-b]pyrrol-1-carbonyl]-butyl}-benzamid,
4-Amino-N-{3-methyl-1-[6-oxo-4-(piperidin-1-carbonyl)-hexahydro-pyrrolo[3,2-b]pyrrol-1-carbonyl]-butyl}-benzamid,
4-Amino-N-{3-methyl-1-[4-(morpholin-4-carbonyl)-6-oxo-hexahydro-pyrrolo[3,2-b]pyrrol-1-carbonyl]-butyl}-benzamid,
4-Amino-N-{3-methyl-1-[6-oxo-4-(piperazin-1-carbonyl)-hexahydro-pyrrolo[3,2-b]pyrrol-1-carbonyl]-butyl}-benzamid,
4-Amino-N-{3-methyl-1-[4-(4-methyl-piperazin-1-carbonyl)-6-oxo-hexahydro-pyrrolo[3,2-b]pyrrol-1-carbonyl]-butyl}-benzamid,
4-Amino-N-(3-methyl-1-{4-[2-(4-methyl-piperazin-1-yl)-acetyl]-6-oxo-hexahydro-pyrrolo[3,2-b]pyrrol-1-carbonyl}-butyl)-benzamid,
4-Amino-N-{3-methyl-1-[6-oxo-4-(2-piperazin-1-yl-acetyl)-hexahydro-pyrrolo[3,2-b]pyrrol-1-carbonyl]-butyl}-benzamid,
4-Amino-N-{1-[4-(furan-2-carbonyl)-6-oxo-hexahydro-pyrrolo[3,2-b]pyrrol-1-carbonyl]-3-methyl-butyl}-benzamid,
4-Amino-N-1-[4-(5-chlor-furan-2-carbonyl)-6-oxo-hexahydro-pyrrolo[3,2-b]pyrrol-1-carbonyl]-3-methyl-butyl}-benzamid,
4-Amino-N-{1-[4-(thiophen-2-carbonyl)-6-oxo-hexahydro-pyrrolo[3,2-b]pyrrol-1-carbonyl]-3-methyl-butyl}-benzamid,
4-Amino-N-{1-[4-(5-chlor-thiophen-2-carbonyl)-6-oxo-hexahydro-pyrrolo[3,2-b]pyrrol-1-carbonyl]-3-methyl-butyl}-benzamid,
4-Amino-N-{1-[4-(furan-3-carbonyl)-6-oxo-hexahydro-pyrrolo[3,2-b]pyrrol-1-carbonyl]-3-methyl-butyl}-benzamid,
4-Amino-N-{1-[4-(thiophen-3-carbonyl)-6-oxo-hexahydro-pyrrolo[3,2-b]pyrrol-1-carbonyl]-3-methyl-butyl}-benzamid,
4-Amino-N-[1-(4-cyclopentancarbonyl-6-oxo-hexahydro-pyrrolo[3,2-b]pyrrol-1-carbonyl)-3-methyl-butyl]-benzamid,
4-Amino-N-[1-(4-cyclohexancarbonyl-6-oxo-hexahydro-pyrrolo[3,2-b]pyrrol-1-carbonyl)-3-methyl-butyl]-benzamid,
4-Amino-N-{1-[4-(cyclopent-3-encarbonyl)-6-oxo-hexahydro-pyrrolo[3,2-b]pyrrol-1-carbonyl]-3-methyl-butyl}-benzamid,
4-Amino-N-{1-[4-(2-cyclopentyl-acetyl)-6-oxo-hexahydro-pyrrolo[3,2-b]pyrrol-1-carbonyl]-3-methyl-butyl}-benzamid,
4-Amino-N-{1-[4-(2-cyclohexyl-acetyl)-6-oxo-hexahydro-pyrrolo[3,2-b]pyrrol-1-carbonyl]-3-methyl-butyl}-benzamid,
4-Amino-N-{3-methyl-1-[6-oxo-4-(pyrrolidin-2-carbonyl)-hexahydro-pyrrolo[3,2-b]pyrrol-1-carbonyl]-butyl}-benzamid,
N-{1-[4-(1-Acetyl-pyrrolidin-2-carbonyl)-6-oxo-hexahydro-pyrrolo[3,2-b]pyrrol-1-carbonyl]-3-methyl-butyl}-4-amino-benzamid,
4-Amino-N-{3-methyl-1-[6-oxo-4-(piperidin-2-carbonyl)-hexahydro-pyrrolo[3,2-b]pyrrol-1-carbonyl]-butyl}-benzamid,

N-{1-[4-(1-Acetyl-piperidin-2-carbonyl)-6-oxo-hexahydro-pyrrolo[3,2-b]pyrrol-1-carbonyl]-3-methyl-butyl}-4-amino-benzamid,
4-Amino-N-{3-methyl-1-[6-oxo-4-(tetrahydro-furan-2-carbonyl)-hexahydro-pyrrolo[3,2-b]pyrrol-1-carbonyl]-butyl}-benzamid,
N-{1-[4-(1-Amino-cyclobutancarbonyl)-6-oxo-hexahydro-pyrrolo[3,2-b]pyrrol-1-carbonyl]-3-methyl-butyl}-4-Amino-benzamid,
N-{1-[4-(1-Amino-cyclopentancarbonyl)-6-oxo-hexahydro-pyrrolo[3,2-b]pyrrol-1-carbonyl]-3-methyl-butyl}-4-amino-benzamid,
N-{1-[4-(1-Amino-cyclohexancarbonyl)-6-oxo-hexahydro-pyrrolo[3,2-b]pyrrol-1-carbonyl]-3-methyl-butyl}-4-amino-benzamid,
N-{1-[4-(1-Acetylamino-cyclobutancarbonyl)-6-oxo-hexahydro-pyrrolo[3,2-b]pyrrol-1-carbonyl]-3-methyl-butyl}-4-amino-benzamid,
N-{1-[4-(1-Acetylamino-cyclopentancarbonyl)-6-oxo-hexahydro-pyrrolo[3,2-b]pyrrol-1-carbonyl]-3-methyl-butyl}-4-amino-benzamid,
N-{1-[4-(1-Acetylamino-cyclohexancarbonyl)-6-oxo-hexahydro-pyrrolo[3,2-b]pyrrol-1-carbonyl]-3-methyl-butyl}-4-amino-benzamid,
4-Amino-N-{1-[4-(1-hydroxy-cyclobutancarbonyl)-6-oxo-hexahydro-pyrrolo[3,2-b]pyrrol-1-carbonyl]-3-methyl-butyl}-benzamid,
4-Amino-N-{1-[4-(1-hydroxy-cyclopentancarbonyl)-6-oxo-hexahydro-pyrrolo[3,2-b]pyrrol-1-carbonyl]-3-methyl-butyl}-benzamid,
4-Amino-N-{1-[4-(1-hydroxy-cyclohexancarbonyl)-6-oxo-hexahydro-pyrrolo[3,2-b]pyrrol-1-carbonyl]-3-methyl-butyl}-benzamid,
4-Amino-N-{1-[4-(1-methoxy-cyclobutancarbonyl)-6-oxo-hexahydro-pyrrolo[3,2-b]pyrrol-1-carbonyl]-3-methyl-butyl}-benzamid,
4-Amino-N-{1-[4-(1-methoxy-cyclopentancarbonyl)-6-oxo-hexahydro-pyrrolo[3,2-b]pyrrol-1-carbonyl]-3-methyl-butyl}-benzamid,
4-Amino-N-{1-[4-(1-methoxy-cyclohexancarbonyl)-6-oxo-hexahydro-pyrrolo[3,2-b]pyrrol-1-carbonyl]-3-methyl-butyl}-benzamid,
N-{1-[4-(2-Amino-2-cyclopentyl-acetyl)-6-oxo-hexahydro-pyrrolo[3,2-b]pyrrol-1-carbonyl]-3-methyl-butyl}-4-amino-benzamid,
N-{1-[4-(2-Amino-2-cyclohexyl-acetyl)-6-oxo-hexahydro-pyrrolo[3,2-b]pyrrol-1-carbonyl]-3-methyl-butyl}-4-amino-benzamid,
N-{1-[4-(2-Acetylamino-2-cyclopentyl-acetyl)-6-oxo-hexahydro-pyrrolo[3,2-b]pyrrol-1-carbonyl]-3-methyl-butyl}-4-amino-benzamid,
N-{1-[4-(2-Acetylamino-2-cyclohexyl-acetyl)-6-oxo-hexahydro-pyrrolo[3,2-b]pyrrol-1-carbonyl]-3-methyl-butyl}-4-amino-benzamid,
N-{1-[4-(2-Acetylamino-propionyl)-6-oxo-hexahydro-pyrrolo[3,2-b]pyrrol-1-carbonyl]-3-methyl-butyl}-4-amino-benzamid,
N-{1-[4-(2-Acetylamino-acetyl)-6-oxo-hexahydro-pyrrolo[3,2-b]pyrrol-1-carbonyl]-3-methyl-butyl}-4-amino-benzamid,
N-(1-{4-[2-(Acetyl-methyl-amino)-acetyl]-6-oxo-hexahydro-pyrrolo[3,2-b]pyrrol-1-carbonyl}-3-methyl-butyl)-4-amino-benzamid,
4-Amino-N-1-[4-(2-dimethylamino-acetyl)-6-oxo-hexahydro-pyrrolo[3,2-b]pyrrol-1-carbonyl]-3-methyl-butyl}-benzamid,
N-{1-[4-(2-Amino-butyryl)-6-oxo-hexahydro-pyrrolo[3,2-b]pyrrol-1-carbonyl]-3-methyl-butyl}-4-amino-benzamid,
N-{1-[4-(2-Acetylamino-butyryl)-6-oxo-hexahydro-pyrrolo[3,2-b]pyrrol-1-carbonyl]-3-methyl-butyl}-4-amino-benzamid,
N-{1-[4-(2-Amino-3-methyl-butyryl)-6-oxo-hexahydro-pyrrolo[3,2-b]pyrrol-1-carbonyl]-3-methyl-butyl}-4-amino-benzamid,
N-{1-[4-(2-Acetylamino-3-methyl-butyryl)-6-oxo-hexahydro-pyrrolo[3,2-b]pyrrol-1-carbonyl]-3-methyl-butyl}-4-amino-benzamid,
N-{1-[4-(2-Amino-3,3-dimethyl-butyryl)-6-oxo-hexahydro-pyrrolo[3,2-b]pyrrol-1-carbonyl]-3-methyl-butyl}-4-amino-benzamid,
N-{1-[4-(2-Acetylamino-3,3-dimethyl-butyryl)-6-oxo-hexahydro-pyrrolo[3,2-b]pyrrol-1-carbonyl]-3-methyl-butyl}-4-amino-benzamid,
N-{1-[4-(2-Amino-3-methyl-pentanoyl)-6-oxo-hexahydro-pyrrolo[3,2-b]pyrrol-1-carbonyl]-3-methyl-butyl}-4-amino-benzamid,
N-{1-[4-(2-Acetylamino-3-methyl-pentanoyl)-6-oxo-hexahydro-pyrrolo[3,2-b]pyrrol-1-carbonyl]-3-methyl-butyl}-4-amino-benzamid,
4-Amino-N-[3-methyl-1-(6-oxo-4-pentanoyl-hexahydro-pyrrolo[3,2-b]pyrrol-1-carbonyl)-butyl]-benzamid,
4-Amino-N-{3-methyl-1-[4-(3-methyl-pentanoyl)-6-oxo-hexahydro-pyrrolo[3,2-b]pyrrol-1-carbonyl]-butyl}-benzamid,

4-Amino-N-{3-methyl-1-[4-(4-methyl-pentanoyl)-6-oxo-hexahydro-pyrrolo[3,2-b]pyrrol-1-carbonyl]-butyl}-benzamid,
N-{1-[4-(2-amino-4-methyl-pentanoyl)-6-oxo-hexahydro-pyrrolo[3,2-b]pyrrol-1-carbonyl]-3-methyl-butyl}-4-amino-benzamid,
N-{1-[4-(2-Acetylamino-4-methyl-pentanoyl)-6-oxo-hexahydro-pyrrolo[3,2-b]pyrrol-1-carbonyl]-3-methyl-butyl}-4-amino-benzamid,
N-{1-[4-(2-Amino-4,4-dimethyl-pentanoyl)-6-oxo-hexahydro-pyrrolo[3,2-b]pyrrol-1-carbonyl]-3-methyl-butyl}-4-amino-benzamid,
N-{1-[4-(2-Acetylamino-4,4-dimethyl-pentanoyl)-6-oxo-hexahydro-pyrrolo[3,2-b]pyrrol-1-carbonyl]-3-methyl-butyl}-4-amino-benzamid,
N-{1-[4-(2-Amino-pentanoyl)-6-oxo-hexahydro-pyrrolo[3,2-b]pyrrol-1-carbonyl]-3-methyl-butyl}-4-amino-benzamid,
N-{1-[4-(2-Acetylamino-pentanoyl)-6-oxo-hexahydro-pyrrolo[3,2-b]pyrrol-1-carbonyl]-3-methyl-butyl}-4-amino-benzamid,
N-{1-[4-(2-Amino-5-methyl-hexanoyl)-6-oxo-hexahydro-pyrrolo[3,2-b]pyrrol-1-carbonyl]-3-methyl-butyl}-4-amino-benzamid,
N-{1-[4-(2-Acetylainino-5-methyl-hexanoyl)-6-oxo-hexahydro-pyrrolo[3,2-b]pyrrol-1-carbonyl]-3-methyl-butyl}-4-amino-benzamid,
N-{1-[4-(2-Hydroxy-3-methyl-butyryl)-6-oxo-hexahydro-pyrrolo[3,2-b]pyrrol-1-carbonyl]-3-methyl-butyl}-4-amino-benzamid,
N-{1-[4-(2-Methoxy-3-methyl-butyryl)-6-oxo-hexahydro-pyrrolo[3,2-b]pyrrol-1-carbonyl]-3-methyl-butyl}-4-amino-benzamid,
4-Amino-N-{1-[4-(2-hydroxy-3,3-dimethyl-butyryl)-6-oxo-hexahydro-pyrrolo[3,2-b]pyrrol-1-carbonyl]-3-methyl-butyl}-benzamid,
4-Amino-N-{1-[4-(2-methoxy-3,3-dimethyl-butyryl)-6-oxo-hexahydro-pyrrolo[3,2-b]pyrrol-1-carbonyl]-3-methyl-butyl}-4-amino-benzamid,
4-Amino-N-{1-[4-(2-hydroxy-3-methyl-pentanoyl)-6-oxo-hexahydro-pyrrolop3,2-b]pyrrol-1-carbonyl]-3-methyl-butyl}-benzamid,
4-Amino-N-{1-[4-(2-methoxy-3-methyl-pentanoyl)-6-oxo-hexahydro-pyrrolo[3,2-b]pyrrol-1-carbonyl]-3-methyl-butyl}-benzamid,
4-Amino-N-{1-[4-(2-hydroxy-4-methyl-pentanoyl)-6-oxo-hexahydro-pyrrolo[3,2-b]pyrrol-1-carbonyl]-3-methyl-butyl}-benzamid,
4-Amino-N-{1-[4-(2-methoxy-4-methyl-pentanoyl)-6-oxo-hexahydro-pyrrolo[3,2-b]pyrrol-1-carbonyl]-3-methyl-butyl}-benzamid,
4-Amino-N-{1-[4-(2-hydroxy-4,4-dimethyl-pentanoyl)-6-oxo-hexahydro-pyrrolo[3,2-b]pyrrol-1-carbonyl]-3-methyl-butyl}-benzamid,
4-Amino-N-{1-[4-(2-methoxy-4,4-dimethyl-pentanoyl)-6-oxo-hexahydro-pyrrolo[3,2-b]pyrrol-1-carbonyl]-3-methyl-butyl}-benzamid,
4-Amino-N-{1-[4-(2-hydroxy-pentanoyl)-6-oxo-hexahydro-pyrrolo[3,2-b]pyrrol-1-carbonyl]-3-methyl-butyl}-benzamid,
4-Amino-N-{1-[4-(2-methoxy-pentanoyl)-6-oxo-hexahydro-pyrrolo[3,2-b]pyrrol-1-carbonyl]-3-methyl-butyl}-benzamid,
4-Amino-N-{1-[4-(2-hydroxy-5-methyl-hexanoyl)-6-oxo-hexahydro-pyrrolo[3,2-b]pyrrol-1-carbonyl]-3-methyl-butyl}-benzamid,
4-Amino-N-{1-[4-(2-methoxy-5-methyl-hexanoyl)-6-oxo-hexahydro-pyrrolo[3,2-b]pyrrol-1-carbonyl]-3-methyl-butyl}-benzamid,
N-[1-(4-Benzoyl-6-oxo-hexahydro-pyrrolo[3,2-b]pyrrol-1-carbonyl)-3-methyl-butyl]-4-diethylaminobenzamid,
4-Diethylamino-N-{3-methyl-1-[6-oxo-4-(pyridin-2-carbonyl)-hexahydro-pyrrolo[3,2-b]pyrrol-1-carbonyl]-butyl}-benzamid,
4-Diethylamino-N-{3-methyl-1-[6-oxo-4-(1-oxy-pyridin-2-carbonyl)-hexahydro-pyrrolo[3,2-b]pyrrol-1-carbonyl]-butyl}-benzamid,
4-Diethylamino-N-{3-methyl-1-[6-oxo-4-(pyridin-3-carbonyl)-hexahydro-pyrrolo[3,2-b]pyrrol-1-carbonyl]-butyl}-benzamid,
4-Diethylamino-N-{3-methyl-1-[6-oxo-4-(1-oxy-pyridin-3-carbonyl)-hexahydro-pyrrolo[3,2-b]pyrrol-1-carbonyl]-butyl}-benzamid,
4-Diethylamino-N-{3-methyl-1-[6-oxo-4-(pyridin-4-carbonyl)-hexahydro-pyrrolo[3,2-b]pyrrol-1-carbonyl]-butyl}-benzamid,
4-Diethylamino-N-{3-methyl-1-[6-oxo-4-(1-oxy-pyridin-4-carbonyl)-hexahydro-pyrrolo[3,2-b]pyrrol-1-carbonyl]-butyl}-benzamid,
N-[1-(4-Benzensulfonyl-6-oxo-hexahydro-pyrrolo[3,2-b]pyrrol-1-carbonyl)-3-methyl-butyl]-4-diethylamino-benzamid,
4-Diethylamino-N-{3-methyl-1-[6-oxo-4-(pyridin-2-sulfonyl)-hexahydro-pyrrolo[3,2-b]pyrrol-1-carbonyl]-butyl}-benzamid,

4-Diethylamino-N-{3-methyl-1-[6-oxo-4-(1-oxy-pyridin-2-sulfonyl)-hexahydro-pyrrolo[3,2-b]pyrrol-1-carbonyl]-butyl}-benzamid,
4-Diethylamino-N-{3-methyl-1-[6-oxo-4-(pyridin-3-sulfonyl)-hexahydro-pyrrolo[3,2-b]pyrrol-1-carbonyl]-butyl}-benzamid,
4-Diethylamino-N-{3-methyl-1-[6-oxo-4-(1-oxy-pyridin-3-sulfonyl)-hexahydro-pyrrolo[3,2-b]pyrrol-1-carbonyl]-butyl}-benzamid,
4-Diethylamino-N-{3-methyl-1-[6-oxo-4-(pyridin-4-sulfonyl)-hexahydro-pyrrolo[3,2-b]pyrrol-1-carbonyl]-butyl}-benzamid,
4-Diethylamino-N-3-methyl-1-[6-oxo-4-(1-oxy-pyridin-4-sulfonyl)-hexahydro-pyrrolo[3,2-b]pyrrol-1-carbonyl]-butyl}-benzamid,
4-Diethylamino-N-[3-methyl-1-(6-oxo-4-phenylacetyl-hexahydro-pyrrolo[3,2-b]pyrrol-1-carbonyl)-butyl]-benzamid,
4-[2-(4-Diethylamino-benzoylamino)-4-methyl-pentanoyl]-3-oxo-hexahydro-pyrrolo[3,2-b]pyrrol-1-carbonsäure-phenylamid,
4-[2-(4-Diethylamino-benzoylamino)-4-methyl-pentanoyl]-3-oxo-hexahydro-pyrrolo[3,2-b]pyrrol-1-carbonsäureisobutylamid,
4-[2-(4-Diethylamino-benzoylamino)-4-methyl-pentanoyl]-3-oxo-hexahydro-pyrrolo[3,2-b]pyrrol-1-carbonsäurephenylester,
4-[2-(4-Diethylamino-benzoylamino)-4-methyl-pentanoyl]-3-oxo-hexahydro-pyrrolo[3,2-b]pyrrol-1-carbonsäureisobutylester,
4-Diethylamino-N-{3-methyl-1-[6-oxo-4-(pyrrolidin-1-carbonyl)-hexahydro-pyrrolo[3,2-b]pyrrol-1-carbonyl]-butyl}-benzamid,
4-Diethylamino-N-{3-methyl-1-[6-oxo-4-(piperidin-1-carbonyl)-hexahydro-pyrrolo[3,2-b]pyrrol-1-carbonyl]-butyl}-benzamid,
4-Diethylamino-N-{3-methyl-1-[4-(morpholin-4-carbonyl)-6-oxo-hexahydro-pyrrolo[3,2-b]pyrrol-1-carbonyl]-butyl}-benzamid,
4-Diethylamino-N-{3-methyl-1-[6-oxo-4-(piperazin-1-carbonyl)-hexahydro-pyrrolo[3,2-b]pyrrol-1-carbonyl]-butyl}-benzamid,
4-Diethylamino-N-{3-methyl-1-[4-(4-methyl-piperazin-1-carbonyl)-6-oxo-hexahydro-pyrrolo[3,2-b]pyrrol-1-carbonyl]-butyl}-benzamid,
4-Diethylamino-N-(3-methyl-1-{4-[2-(4-methyl-piperazin-1-yl)-acetyl]-6-oxo-hexahydro-pyrrolo[3,2-b]pyrrol-1-carbonyl}-butyl)-benzamid,
4-Diethylamino-N-{3-methyl-1-[6-oxo-4-(2-piperazin-1-yl-acetyl)-hexahydro-pyrrolo[3,2-b]pyrrol-1-carbonyl]-butyl}-benzamid,
4-Diethylamino-N-{1-[4-(furan-2-carbonyl)-6-oxo-hexahydro-pyrrolo[3,2-b]pyrrol-1-carbonyl]-3-methyl-butyl}-benzamid,
4-Diethylamino-N-{1-[4-(5-chlor-furan-2-carbonyl)-6-oxo-hexahydro-pyrrolo[3,2-b]pyrrol-1-carbonyl]-3-methyl-butyl}-benzamid,
4-Diethylamino-N-{1-[4-(thlophen-2-carbonyl)-6-oxo-hexahydro-pyrrolo[3,2-b]pyrrol-1-carbonyl]-3-methyl-butyl}-benzamid,
4-Diethylamino-N-{1-[4-(5-chlor-thiophen-2-carbonyl)-6-oxo-hexahydro-pyrrolo[3,2-b]pyrrol-1-carbonyl]-3-methyl-butyl}-benzamid,
4-Diethylamino-N-{1-[4-(furan-3-carbonyl)-6-oxo-hexahydro-pyrrolo[3,2-b]pyrrol-1-carbonyl]-3-methyl-butyl}-benzamid,
4-Diethylamino-N-{1-[4-(thiophen-3-carbonyl)-6-oxo-hexahydro-pyrrolo[3,2-b]pyrrol-1-carbonyl]-3-methyl-butyl}-benzamid,
4-Diethylamino-N-[1-(4-cyclopentancarbonyl-6-oxo-hexahydro-pyrrolo[3,2-b]pyrrol-1-carbonyl)-3-methyl-butyl]-benzamid,
4-Diethylamino-N-[1-(4-cyclohexancarbonyl-6-oxo-hexahydro-pyrrolo[3,2-b]pyrrol-1-carbonyl)-3-methyl-butyl]-benzamid,
4-Diethylamino-N-{1-[4-(cyclopent-3-encarbonyl)-6-oxo-hexahydro-pyrrolo[3,2-b]pyrrol-1-carbonyl]-3-methyl-butyl}-benzamid,
4-Diethylamino-N-{1-[4-(2-cyclopentyl-acetyl)-6-oxo-hexahydro-pyrrolo[3,2-b]pyrrol-1-carbonyl]-3-methyl-butyl}-benzamid,
4-Diethylamino-N-{1-[4-(2-cyclohexyl-acetyl)-6-oxo-hexahydro-pyrrolo[3,2-b]pyrrol-1-carbonyl]-3-methyl-butyl}-benzamid,
4-Diethylamino-N-{3-methyl-1-[6-oxo-4-(pyrrolidin-2-carbonyl)-hexahydro-pyrrolo[3,2-b]pyrrol-1-carbonyl]-butyl}-benzamid,
N-{1-[4-(1-Acetyl-pyrrolidin-2-carbonyl)-6-oxo-hexahydro-pyrrolo[3,2-b]pyrrol-1-carbonyl]-3-methyl-butyl}-4-diethylamino-benzamid,
4-Diethylamino-N-{3-methyl-1-[6-oxo-4-(piperidin-2-carbonyl)-hexahydro-pyrrolo[3,2-b]pyrrol-1-carbonyl]-butyl}-benzamid,
N-{1-[4-(1-Acetyl-piperidin-2-carbonyl)-6-oxo-hexahydro-pyrrolo[3,2-b]pyrrol-1-carbonyl]-3-methyl-butyl}-4-diethylamino-benzamid,
4-Diethylamino-N-{3-methyl-1-[6-oxo-4-(tetrahydro-furan-2-carbonyl)-hexahydro-pyrrolo[3,2-b]pyrrol-1-carbonyl]-butyl}-benzamid,
N-{1-[4-(1-Amino-cyclobutancarbonyl)-6-oxo-hexahydro-pyrrolo[3,2-b]pyrrol-1-carbonyl]-3-methyl-butyl}-4-diethylamino-benzamid,
N-{1-[4-(1-Amino-cyclopentancarbonyl)-6-oxo-hexahydro-pyrrolo[3,2-b]pyrrol-1-carbonyl]-3-methyl-butyl}-4-diethylamino-benzamid,
N-{1-[4-(1-Amino-cyclohexancarbonyl)-6-oxo-hexahydro-pyrrolo[3,2-b]pyrrol-1-carbonyl]-3-methyl-butyl}-4-diethylamino-benzamid,
N-{1-[4-(1-Acetylamino-cyclobutancarbonyl)-6-oxo-hexahydro-pyrrolo[3,2-b]pyrrol-1-carbonyl]-3-methyl-butyl}-4-diethylamino-benzamid,
N-{1-[4-(1-Acetylamino-cyclopentancarbonyl)-6-oxo-hexahydro-pyrrolo[3,2-b]pyrrol-1-carbonyl]-3-methyl-butyl}-4-diethylamino-benzamid,
N-{1-[4-(1-Acetylamino-cyclohexancarbonyl)-6-oxo-hexahydro-pyrrolo[3,2-b]pyrrol-1-carbonyl]-3-methyl-butyl}-4-diethylamino-benzamid,
4-Diethylamino-N-{1-[4-(1-hydroxy-cyclobutancarbonyl)-6-oxo-hexahydro-pyrrolo[3,2-b]pyrrol-1-carbonyl]-3-methyl-butyl}-benzamid,
4-Diethylamino-N-{1-[4-(1-hydroxy-cyclopentancarbonyl)-6-oxo-hexahydro-pyrrolo[3,2-b]pyrrol-1-carbonyl]-3-methyl-butyl}-benzamid,
4-Diethylamino-N-{1-[4-(1-hydroxy-cyclohexancarbonyl)-6-oxo-hexahydro-pyrrolo[3,2-b]pyrrol-1-carbonyl]-3-methyl-butyl}-benzamid,
4-Diethylamino-N-{1-[4-(1-methoxy-cyclobutancarbonyl)-6-oxo-hexahydro-pyrrolo[3,2-b]pyrrol-1-carbonyl]-3-methyl-butyl}-benzamid,
4-Diethylamino-N-{1-[4-(1-methoxy-cyclopentancarbonyl)-6-oxo-hexahydro-pyrrolo[3,2-b]pyrrol-1-carbonyl]-3-methyl-butyl}-benzamid,
4-Diethylamino-N-{1-[4-(1-methoxy-cyclohexancarbonyl)-6-oxo-hexahydro-pyrrolo[3,2-b]pyrrol-1-carbonyl]-3-methyl-butyl}-benzamid,
N-{1-[4-(2-Amino-2-cyclopentyl-acetyl)-6-oxo-hexahydro-pyrrolo[3,2-b]pyrrol-1-carbonyl]-3-methyl-butyl}-4-diethylamino-benzamid,
N-{1-[4-(2-Amino-2-cyclohexyl-acetyl)-6-oxo-hexahydro-pyrrolo[3,2-b]pyrrol-1-carbonyl]-3-methyl-butyl}-4-diethylamino-benzamid,
N-{1-[4-(2-Acetylamino-2-cyclopentyl-acetyl)-6-oxo-hexahydro-pyrrolo[3,2-b]pyrrol-1-carbonyl]-3-methyl-butyl}-4-diethylamino-benzamid,
N-{1-[4-(2-Acetylamino-2-cyclohexyl-acetyl)-6-oxo-hexahydro-pyrrolo[3,2-b]pyrrol-1-carbonyl]-3-methyl-butyl}-4-diethylamino-benzamid,
N-{1-[4-(2-Acetylamino-propionyl)-6-oxo-hexahydro-pyrrolo[3,2-b]pyrrol-1-carbonyl]-3-methyl-butyl}-4-diethylamino-benzamid,
N-{1-[4-(2-Acetylamino-acetyl)-6-oxo-hexahydro-pyrrolo[3,2-b]pyrrol-1-carbonyl]-3-methyl-butyl} 4-diethylamino-benzamid,
N-(1-{4-(2-Acetyl-methyl-amino)-acetyl]-6-oxo-hexahydro-pyrrolo[3,2-b]pyrrol-1-carbonyl}-3-methyl-butyl)-4-diethylamino-benzamid,
4-Diethylamino-N-{1-[4-(2-dimethylamino-acetyl)-6-oxo-hexahydro-pyrrolo[3,2-b]pyrrol-1-carbonyl]-3-methyl-butyl}-benzamid,
N-{1-[4-(2-Amino-butyryl)-6-oxo-hexahydro-pyrrolo[3,2-b]pyrrol-1-carbonyl]-3-methyl-butyl}-4-diethylamino-benzamid,
N-{1-[4-(2-Acetylamino-butyryl)-6-oxo-hexahydro-pyrrolo[3,2-b]pyrrol-1-carbonyl]-3-methyl-butyl}-4-diethylamino-benzamid,
N-{1-[4-(2-Amino-3-methyl-butyryl)-6-oxo-hexahydro-pyrrolo[3,2-b]pyrrol-1-carbonyl]-3-methyl-butyl}-4-diethylamino-benzamid,
N-{1-[4-(2-Acetylamino-3-methyl-butyryl)-6-oxo-hexahydro-pyrrolo[3,2-b]pyrrol-1-carbonyl]-3-methyl-butyl}-4-diethylamino-benzamid,
N-{1-[4-(2-Amino-3,3-dimethyl-butyryl)-6-oxo-hexahydro-pyrrolo[3,2-b]pyrrol-1-carbonyl]-3-methyl-butyl}-4-diethylamino-benzamid,
N-{1-[4-(2-Acetylamino-3,3-dimethyl-butyryl)-6-oxo-hexahydro-pyrrolo[3,2-b]pyrrol-1-carbonyl]-3-methyl-butyl}-4-diethylamino-benzamid,

N-{1-[4-(2-Amino-3-methyl-pentanoyl)-6-oxo-hexahydro-pyrrolo[3,2-b]pyrrol-1-carbonyl]-3-methyl-butyl}-4-diethylamino-benzamid,
N-{1-[4-(2-Acetylamino-3-methyl-pentanoyl)-6-oxo-hexahydro-pyrrolo[3,2-b]pyrrol-1-carbonyl]-3-methyl-butyl}-4-diethylamino-benzamid,
4-Diethylamino-N-[3-methyl-1-(6-oxo-4-pentanoyl-hexahydro-pyrrolo[3,2-b]pyrrol-1-carbonyl)-butyl]-benzamid,
4-Diethylamino-N-{3-methyl-1-[4-(3-mcthyl-pentanoyl)-6-oxo-hexahydro-pyrrolo[3,2-b]pyrrol-1-carbonyl]-butyl}-benzamid,
4-Diethylamino-N-3-methyl-1-[4-(4-methyl-pentanoyl)-6-oxo-hexahydro-pyrrolo[3,2-b]pyrrol-1-carbonyl]-butyl}-benzamid,
N-{1-[4-(2-Amino-4-methyl-pentanoyl)-6-oxo-hexahydro-pyrrolo[3,2-b]pyrrol-1-carbonyl]-3-methyl-butyl}-4-diethylamino-benzamid,
N-{1-[4-(2-Acetylamino-4-methyl-pentanoyl)-6-oxo-hexahydro-pyrrolo[3,2-b]pyrrol-1-carbonyl]-3-methyl-butyl}-4-diethylamino-benzamid,
N-{1-[4-(2-Amino-4,4-dimethyl-pentanoyl)-6-oxo-hexahydro-pyrrolo[3,2-b]pyrrol-1-carbonyl]-3-methyl-butyl}-4-diethylamino-benzamid,
N-{1-[4-(2-Acetylamino-4,4-dimethyl-pentanoyl)-6-oxo-hexahydro-pyrrolo[3,2-b]pyrrol-1-carbonyl]-3-methyl-butyl}-4-diethylamino-benzamid,
N-{1-[4-(2-Amino-pentanoyl)-6-oxo-hexahydro-pyrrolo[3,2-b]pyrrol-1-carbonyl]-3-methyl-butyl}-4-diethylamino-benzamid,
N-{1-[4-(2-Acetylamino-pentanoyl)-6-oxo-hexahydro-pyrrolo[3,2-b]pyrrol-1-carbonyl]-3-methyl-butyl}-4-diethylamino-benzamid,
N-{1-[4-(2-Amino-5-methyl-hexanoyl)-6-oxo-hexahydro-pyrrolo[3,2-b]pyrrol-1-carbonyl]-3-methyl-butyl}-4-diethylamino-benzamid,
N-{1-[4-(2-Acetylamino-5-methyl-hexanoyl)-6-oxo-hexahydro-pyrrolo[3,2-b]pyrrol-1-carbonyl]-3-methyl-butyl}-4-diethylamino-benzamid,
N-{1-[4-(2-Hydroxy-3-methyl-butyryl)-6-oxo-hexahydro-pyrrolo[3,2-b]pyrrol-1-carbonyl]-3-methyl-butyl}-4-diethylamino-benzamid,
N-{1-[4-(2-Methoxy-3-methyl-butyryl)-6-oxo-hexahydro-pyrrolo[3,2-b]pyrrol-1-carbonyl]-3-methyl-butyl}-4-diethylamino-benzamid,
4-Diethylamino-N-{1-[4-(2-hydroxy-3,3-dimethyl-butyryl)-6-oxo-hexahydro-pyrrolo[3,2-b]pyrrol-1-carbonyl]-3-methyl-butyl}-benzamid,
4-Diethylamino-N-{1-[4-(2-methoxy-3,3-dimethyl-butyryl)-6-oxo-hexahydro-pyrrolo[3,2-b]pyrrol-1-carbonyl]-3-methyl-butyl}-benzamid,
4-Diethylamino-N-{1-[4-(2-hydroxy-3-methyl-pentanoyl)-6-oxo-hexahydro-pyrrolo[3,2-b]pyrrol-1-carbonyl]-3-methyl-butyl}-benzamid,
4-Diethylamino-N-{1-[4-(2-methoxy-3-methyl-pentanoyl)-6-oxo-hexahydro-pyrrolo[3,2-b]pyrrol-1-carbonyl]-3-methyl-butyl}-benzamid,
4-Diethylamino-N-{1-[4-(2-hydroxy-4-methyl-pentanoyl)-6-oxo-hexahydro-pyrrolo[3,2-b]pyrrol-1-carbonyl]-3-methyl-butyl}-benzamid,
4-Diethylamino-N-{1-[4-(2-methoxy-4-methyl-pentanoyl)-6-oxo-hexahydro-pyrrolo[3,2-b]pyrrol-1-carbonyl]-3-methyl-butyl}-benzamid,
4-Diethylamino-N-{1-[4-(2-hydroxy-4,4-dimethyl-pentanoyl)-6-oxo-hexahydro-pyrrolo[3,2-b]pyrrol-1-carbonyl]-3-methyl-butyl}-benzamid,
4-Diethylamino-N-{1-[4-(2-methoxy-4,4-dimethyl-pentanoyl)-6-oxo-hexahydro-pyrrolo[3,2-b]pyrrol-1-carbonyl]-3-methyl-butyl}-benzamid,
4-Diethylamino-N-{1-[4-(2-hydroxy-pentanoyl)-6-oxo-hexahydro-pyrrolo[3,2-b]pyrrol-1-carbonyl]-3-methyl-butyl}-benzamid,
4-Diethylamino-N-{1-[4-(2-methoxy-pentanoyl)-6-oxo-hexahydro-pyrrolo[3,2-b]pyrrol-1-carbonyl]-3-methyl-butyl}-benzamid,
4-Diethylamino-N-1-[4-(2-hydroxy-5-methyl-hexanoyl)-6-oxo-hexahydro-pyrrolo[3,2-b]pyrrol-1-carbonyl]-3-methyl-butyl}-benzamid,
4-Diethylamino-N-{1-[4-(2-methoxy-5-methyl-hexanoyl)-6-oxo-hexahydro-pyrrolo[3,2-b]pyrrol-1-carbonyl]-3-methyl-butyl}-benzamid.

**36.** Verbindung nach einem der Ansprüche 1 bis 35 zur Verwendung in der Medizin.

**37.** Verbindung nach einem der Ansprüche 1 bis 35 zur Verwendung bei der Behandlung von Osteoporose, Paget’scher Krankheit, Zahnfleischerkrankungen, wie Gingivitis und Periodontitis, Hyperkalziämie von Malignität, metabolischer

Knochenerkrankung, Erkrankungen, die mit Matrix- oder Knorpelabbau einhergehen, insbesondere Osteoarthritis oder rheumatoide Arthritis, und neoplastischen Erkrankungen.

**38.** Verwendung einer Verbindung nach einem der Ansprüche 1 bis 35 bei der Herstellung eines Medikaments zur Behandlung von Osteoporose, Paget'scher Krankheit, Zahnfleischerkrankungen, wie Gingivitis und Periodontitis, Hyperkalziämie von Malignität, metabolischer Knochenerkrankung, Erkrankungen, die mit Matrix- oder Knorpelabbau einhergehen, insbesondere Osteoarthritis oder rheumatoide Arthritis, und neoplastischen Erkrankungen.

**39.** Pharmazeutische oder veterinärmedizinische Zusammensetzung, welche eine oder mehrere Verbindungen nach einem der Ansprüche 1 bis 35 und einen pharmazeutisch oder veterinärmedizinisch verträglichen Träger umfaßt.

**40.** Verfahren zur Herstellung einer pharmazeutischen oder veterinärmedizinischen Zusammensetzung nach Anspruch 39, wobei das Verfahren das Assoziieren der aktiven Verbindung(en) mit dem Träger umfaßt.

## Revendications

**1.** Composé de formule générale (I)

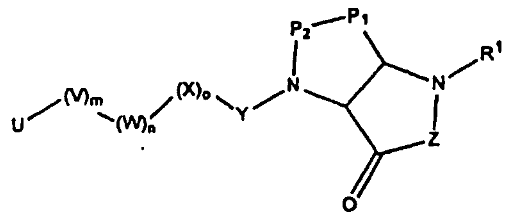

(I)

dans laquelle :

Z = $CR^3R^4$, dans lequel $R^3$ et $R^4$ sont choisis indépendamment entre des groupes alkyle en $C_0$ à $C_7$ (lorsque C = 0, $R^3$ ou $R^4$ représente simplement un atome d'hydrogène), cycloalkyle en $C_3$ à $C_6$, Ar-(alkyle en $C_0$ à $C_7$) (lorsque C = 0, $R^3$ ou $R^4$ représente simplement un groupement aromatique Ar),

$P_1$ = $CR^5R^6$, dans lequel $R^5$ et $R^6$ sont choisis indépendamment entre des groupes alkyle en $C_0$ à $C_7$, cycloalkyle en $C_3$ à $C_6$, Ar-(alkyle en $C_0$ à $C_7$) O-(alkyle en $C_0$ à $C_7$) O-(cycloalkyle en $C_3$ à $C_6$), O-Ar-(alkyle en $C_0$ à $C_7$), S-(alkyle en $C_0$ à $C_7$), S-(cycloalkyle en $C_3$ à $C_6$), S-Ar- (alkyle en $C_0$ à $C_7$) NH-(alkyle en $C_0$ à $C_7$), NH- (cycloalkyle en $C_3$ à $C_6$), NH-Ar-(alkyle en $C_0$ à $C_7$), N-(alkyle en $C_0$ à $C_7)_2$, N-(cycloalkyle en $C_3$ à $C_6)_2$, et N- (Ar- (alkyle en $C_0$ à $C_7))_2$ ;

$P_2$ = O, $CR^7R^8$ ou $NR^9$, dans lequel $R^7$ et $R^8$ sont choisis indépendamment entre des groupes alkyle en $C_0$ à $C_7$, cycloalkyle en $C_3$ à $C_6$, Ar-(alkyle en $C_0$ à $C_7$) et $R^9$ est choisi entre des groupes alkyle en $C_0$ à $C_7$, cycloalkyle en $C_3$ à $C_6$ et Ar-(alkyle en $C_0$ à $C_7$);

Y = $CR^{10}R^{11}$-C(O) ou $CR^{10}R^{11}$-C(S) ou $CR^{10}R^{11}$-S(O) ou $CR^{10}R^{11}$-$SO_2$ dans lequel $R^{10}$ et $R^{11}$ sont choisis indépendamment entre des groupes alkyle en $C_0$ à $C_7$, cycloalkyle en $C_3$ à $C_6$ et Ar-(alkyle en $C_0$ à $C_7$), ou bien Y représente un groupe

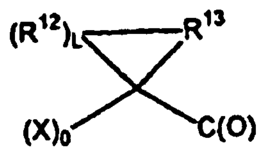

ou C(S) ou S(O) ou $SO_2$

dans lequel L représente un nombre de un à quatre et $R^{12}$ et $R^{13}$ sont choisis indépendamment parmi des groupes $CR^{14}R^{15}$ dans lesquels $R^{14}$ et $R^{15}$ sont choisis indépendamment entre des groupes alkyle en $C_0$ à $C_7$, cycloalkyle

en $C_3$ à $C_6$, Ar-(alkyle en $C_0$ à $C_7$) et halogéno ; et, pour chacun des groupes $R^{12}$ et $R^{13}$, $R^{14}$ ou bien $R^{15}$ (mais non à la fois $R^{14}$ et $R^{15}$) peut être choisi en outre entre des groupes O-(alkyle en $C_0$ à $C_7$), O-(cycloalkyl en $C_3$ à $C_6$), O-Ar-(alkyle en $C_0$ à $C_7$), S-(alkyle en $C_0$ à $C_7$), S-(cycloalkyle en $C_3$ à $C_6$), S-Ar- (alkyle en $C_0$ à $C_7$), NH-(alkyle en $C_0$ à $C_7$), NH-(cycloalkyle en $C_3$ à $C_6$), NH-Ar-(alkyle en $C_0$ à $C_7$), N-(alkyle en $C_0$ à $C_7)_2$, N-(cycloalkyle en $C_3$ à $C_6)_2$ et N-(Ar- (alkyle en $C_0$ à $C_7))_2$ ;

$(X)_o$ = $CR^{16}R^{17}$, dans lequel $R^{16}$ et $R^{17}$ sont choisis indépendamment entre des groupes alkyle en $C_0$ à $C_7$, cycloalkyle en $C_3$ à $C_6$, Ar-(alkyle en $C_0$ à $C_7$) et o représente un nombre de zéro à trois ;

$(W)_n$ = O, S, C(O), S(O) ou $S(O)_2$ ou $NR^{18}$, dans lequel $R^{18}$ est choisi entre des groupes alkyle en $C_0$ à $C_7$, cycloalkyle en $C_3$ à $C_6$ et Ar-(alkyle en $C_0$ à $C_7$) et n est égal à zéro ou un ;

$(V)_m$ = C(O), C(S), S(O), $S(O)_2$, $S(O)_2NH$, OC(O), NHC(O), NHS(O), $NHS(O)_2$, OC(O)NH, C(O)NH ou $CR^{19}R^{20}$, C=N-C(O)-$OR^{19}$ ou C=N-C(O)-$NHR^{19}$, dans lequel $R^{19}$ et $R^{20}$ sont choisis indépendamment entre des groupes alkyle en $C_0$ à $C_7$, cycloalkyle en $C_3$ à $C_6$, Ar-(alkyle en $C_0$ à $C_7$) et m est un nombre de zéro à trois, sous réserve que, lorsque m est supérieur à un, $(V)_m$ contient un maximum d'un groupe carbonyle ou sulfonyle ;

U = un noyau monocyclique penta- à heptagonal stable ou noyau bicyclique octo- à undécagonal stable qui est saturé ou insaturé et qui comprend zéro à quatre hétéroatomes (de la manière décrite en détail ci-dessous) :

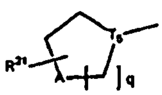
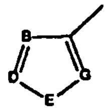
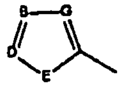
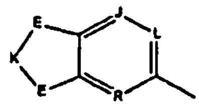
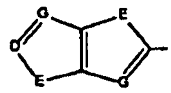

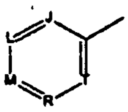
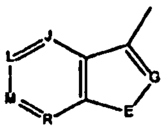
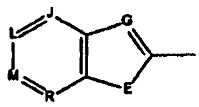
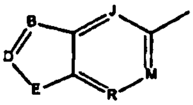
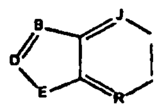

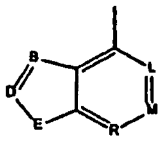
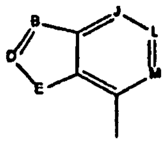
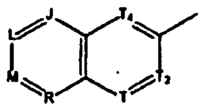
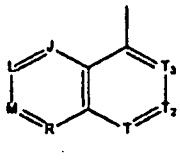
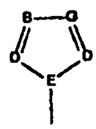

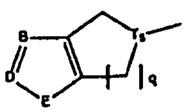
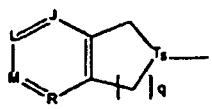
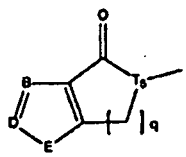
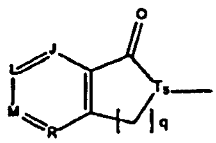

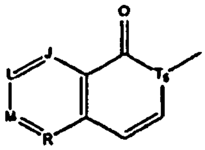
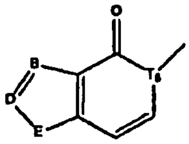
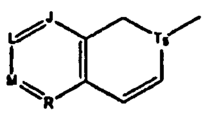
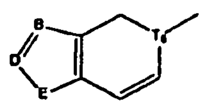

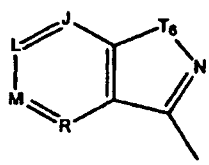 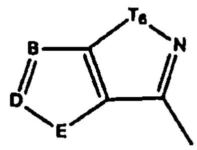 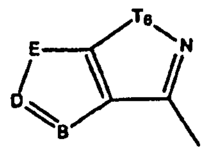

où $R^{21}$ représente un groupe :

alkyle en $C_0$ à $C_7$, cycloalkyle en $C_3$ à $C_6$, Ar-(alkyle en $C_0$ à $C_7$), O-(alkyle en $C_0$ à $C_7$), O-(cycloalkyle en $C_3$ à $C_6$), O-Ar-(alkyle en $C_0$ à $C_7$), S-(alkyle en $C_0$ à $C_7$), S-(cycloalkyle en $C_3$ à $C_6$), S-Ar- (alkyle en $C_0$ à $C_7$), $SO_2$-(alkyle en $C_0$ à $C_7$), $SO_2$-(cycloalkyle en $C_3$ à $C_6$), $SO_2$-Ar-(alkyle en $C_0$ à $C_7$), NH-(alkyle en $C_0$ à $C_7$), NH-(cycloalkyle en $C_3$ à $C_6$), NH-Ar-(alkyle en $C_0$ à $C_7$), N-(alkyle en $C_0$ à $C_7)_2$, N-(cycloalkyle en $C_3$ à $C_6)_2$ ou N-(Ar-(alkyle en $C_0$ à $C_7$) ; ou bien, lorsqu'il fait partie d'un groupe $CHR^{21}$ ou $CR^{21}$, $R^{21}$ peut représenter un groupe halogéno ;

A est choisi entre des groupes :

$CH_2$, $CHR^{21}$, O, S, $SO_2$, $NR^{22}$ ou N-oxyde ($N\rightarrow O$), où $R^{21}$ répond à la définition précitée ; et $R^{22}$ est choisi entre des groupes alkyle en $C_0$ à $C_7$, cycloalkyle en $C_3$ à $C_6$ et Ar-(alkyle en $C_0$ à $C_7$) ;

B, D et G sont choisis indépendamment parmi des groupes :

$CR^{21}$, où $R^{21}$ répond à la définition précitée, ou N ou N-oxyde ($N\rightarrow O$) ;

E est choisi entre des groupes :

$CH_2$, $CHR^{21}$, O, S, $SO_2$, $NR^{22}$ ou N-oxyde ($N\rightarrow O$), où $R^{21}$ et $R^{22}$ répondent aux définitions précitées ;

K est choisi entre des groupes :

$CH_2$, $CHR^{22}$, où $R^{22}$ répond à la définition précitée ;

J, L, M, R, T, $T_2$, $T_3$ et $T_4$ sont choisis indépendamment parmi des groupes :

$CR^{21}$ où $R^{21}$ répond à la définition précitée, ou N ou N-oxyde ($N\rightarrow O$) ;

$T_5$ est choisi entre des groupes :

CH ou N ;

$T_6$ est choisi entre des groupes :

$NR^{22}$, $SO_2$, OC(O), C(O), $NR^{22}C(O)$ ;

q représente un nombre de un à trois, définissant ainsi un noyau penta-, hexa- ou heptagonal ;

$R^1 = R^2C(O)$, $R^2OC(O)$, $R^2NQC(O)$, $R^2SO_2$, dans lequel $R^2$ est choisi entre des groupes alkyle en $C_1$ à $C_7$, cycloalkyle en $C_3$ à $C_6$ et Ar-(alkyle en $C_0$ à $C_7$) (lorsque C = 0, $R^2$ représente simplement un groupement aromatique Ar) et Q représente un groupe alkyle en $C_0$ à $C_7$ ;

ou un de ses sels, hydrates, produits de solvatation ou complexes.

**2.** Composé suivant la revendication 1, dans lequel, indépendamment ou dans n'importe quelle combinaison :

Z représente un groupe $CH_2$ ;

$P_1$ représente un groupe $CH_2$ ;
$P_2$ représente un groupe $CH_2$, O ou NH ;

$R^2$ représente un groupe Ar, alkyle en $C_0$ à $C_2$
ou bien $R^2$ représente un groupe alkyle en $C_1$ à $C_7$ qui peut comprendre un groupe -O- ou -NH- en tant qu'une partie de la chaîne et qui est non substitué ou qui est substitué avec un ou plusieurs groupes $NH_2$, NHMe, $NHC(O)CH_3$, $NMeC(O)CH_3$ OH ou OMe
ou bien $R^2$ représente un groupe cycloalkyle en $C_3$ à $C_6$, dans lequel le système cyclique est connecté directement au reste du groupement $R^1$ ou bien il existe un groupe méthylène intermédiaire ;
dans le groupe $(X)_0$, chacun des groupes $R^{16}$ et $R^{17}$ est choisi entre des groupes alkyle en $C_0$ à $C_7$ et Ar-(alkyle en $C_0$ à $C_7$), par exemple un atome d'hydrogène, une chaîne alkyle droite ou ramifiée, une chaîne hétéroalkyle droite ou ramifiée, une chaîne arylalkyle facultativement substituée ou une chaîne arylhétéroalkyle facultativement substituée ;
dans le groupe $(X)_0$, $R^{16}$ et $R^{17}$ représentent des atomes d'hydrogène et o est égal à zéro ou un ;
dans le groupe $(V)_m$, V est choisi entre des groupes C(O), OC(O), NHC(O), C(O)NH, $CHR^{20}$, C=N-C(O)-$OR^{19}$ ou C=N-C(O)-$NHR^{19}$
dans lesquels $R^{19}$ est choisi entre des groupes alkyle en $C_8$ à $C_7$, cycloalkyle en $C_3$ à $C_6$, Ar-(alkyle en $C_0$ à $C_7$) et $R^{20}$ représente un groupe alkyle en $C_0$ à $C_4$, et
m est égal à zéro ou un.

**3.** Composé suivant la revendication 2, dans lequel $R^2$ représente un groupe :

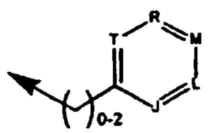
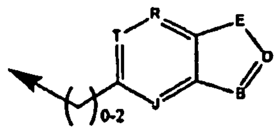
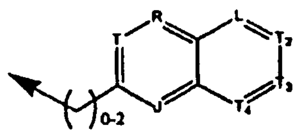

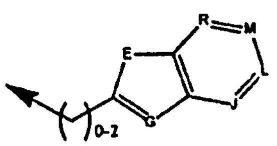
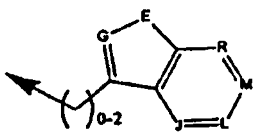
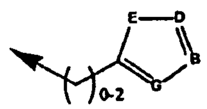
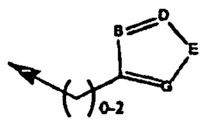

dans lequel J, L, M, T, $T_2$, $T_3$ et $T_4$, B, D, G et E répondent aux définitions précitées.

**4.** Composé suivant l'une quelconque des revendications 1 à 3, dans lequel $R^2$ représente un groupe Ar-(alkyle en $C_0$ ou $C_1$), par exemple :

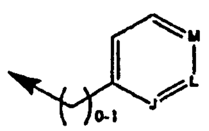
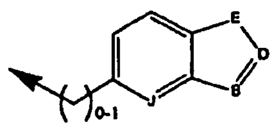
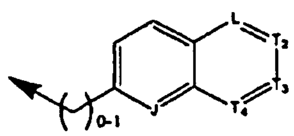

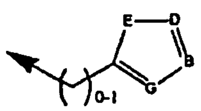
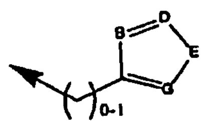
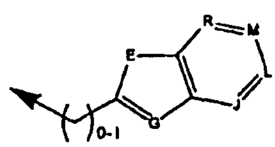
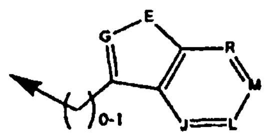

où J, L, M, $T_2$, $T_3$, $T_4$, B, D, G et E répondent aux définitions précitées.

**5.** Composé suivant la revendication 4, dans lequel $R^2$ comprend un Ar-(alkyle en $C_0$ ou $C_1$) monocyclique et fait partie d'un groupe $R^1$ choisi entre :

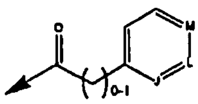
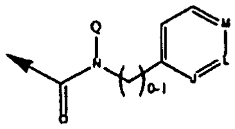
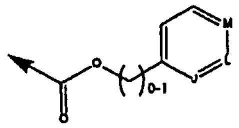

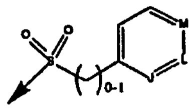
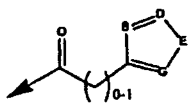
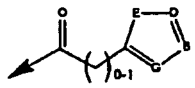

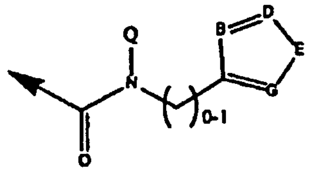
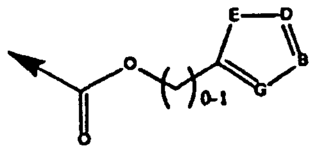

où :

J, L, M, B, D et G répondent aux définitions précitées (c'est-à-dire $CR^{21}$, N ou N→O) et où
$R^{21}$ est choisi entre un atome d'hydrogène, des groupes méthyle, méthoxy, éthyle, isopropyle, trifluorométhyle, trifluorométhoxy, F, Cl, $SO_2Me$ ; et
B, D et G répondent aux définitions précitées (c'est-à-dire $CR^{21}$, N ou N→O) et où $R^{21}$ est choisi entre un atome d'hydrogène, des groupes méthyle, méthoxy, éthyle, isopropyle, trifluorométhyle, trifluorométhoxy, F, Cl, et $SO_2Me$ ; et
E répond à la définition précitée ; et
Q est choisi entre un atome d'hydrogène ou un groupe méthyle.

**6.** Composé suivant la revendication 2, dans lequel $R^2$ représente un groupe alkyle en $C_3$ à $C_6$ qui est ramifié en position $\alpha$ ou qui comprend un substituant $NH_2$, NHMe, $NHC(O)CH_3$, $NMeC(O)CH_3$, OH ou OMe en position $\alpha$.

**7.** Composé suivant la revendication 1 ou la revendication 2, dans lequel $R^2$ représente un groupe cyclopropyle, cyclobutyle, cyclopentyle, cyclohexyle, pyrrolidine, pipéridine, morpholine, tétrahydrofuranne, cyclopentène, cyclopentadiène, cyclohexadiène ou pipérazine, où les noyaux contenant de l'azote peuvent être N-substitués avec des groupes tels que des groupes alkyle en $C_1$ à $C_4$, phényle et benzyle.

**8.** Composé suivant la revendication 1 ou la revendication 2, dans lequel $R^1$ représente un groupe :

benzoyle ; pyridine-2-carbonyle ; 1-oxypyridine-2-carbonyle ; pyridine-3-carbonyle ; 1-oxypyridine-3-carbonyle ; pyridine-4-carbonyle ; 1-oxypyridine-4-carbonyle ; phénylsulfonyle ; pyridine-2-sulfonyle ; 1-oxypyridine-2-sulfonyle ; pyridine-3-sulfonyle ; 1-oxy-pyridine-3-sulfonyle ; pyridine-4-sulfonyle ; 1-oxypyridine-4-sulfonyle ; phénylacétyle ; phényl-carbamoyle ; isobutylcarbamoyle ; phényloxycarbonyle ; isobutyloxycarbonyle ; pyrrolidine-N-carbonyle ; pipéridine-N-carbonyle ; morpholine-N-carbonyle ; pipérazine-N-carbonyle ; 4-méthyl-pipérazine-N-carbonyle ; (4-méthyl-pipérazine-1-yl)-acétoyle ; pipérazine-1-yl-acétoyle ; furanne-2-carbonyle ; 5-chlorofuranne-2-carbonyle ; thiophène-2-carbonyle ; 5-chlorothiophène-2-carbonyle ; furanne-3-carbonyle ; thiophène-3-carbonyle ; cyclopentoyle ; cyclohexoyle ; cyclopent-3-énoyle ; cyclopentylméthylcarbonyle ; cyclohexylméthylcarbonyle ; pyrrolidine-2-carbonyle ; N-acétyl-pipéridine-2-carbonyle ; tétrahydrofuranne-2-carbonyle ; 1-aminocyclobutanol ; 1-aminocyclopentanoyle ; 1-aminocyclohexanoyle ; N-acétyl-1-aminocyclobutanoyle ; N-acétyl-1-aminocyclopentanoyle ; N-acétyl-1-aminocyclohexanoyle ; 1-hydroxycyclobutanoyle ; 1-hydroxycyclopentanoyle ; 1-hydroxycyclohexanoyle ; 1-méthoxycyclobutanoyle ; 1-méthoxycyclopentanoyle ; 1-méthoxycyclohexanoyle ; aminocyclopentylacétyle ; aminocyclohexylacétoyle ; N-acétylaminocyclopentyl-acétoyle ; N-acétylaminocyclohexylacétoyle ; 2-acétylaminopropionoyle ; 2-acétylaminoéthanoyle ; 2-acétyl-N-méthyl-aminoéthanoyle ; N,N-diméthylaminoacétoyle ; 2-aminobutanoyle ; N-acétyl-2-aminobutanoyle ; 2-amino-3-méthylbutanoyl ; N-acétyl-2-amino-3-méthylbutanoyle ; 2-amino-3,3-diméthylbutanoyle ; N-acétyl-2-amino-3,3-diméthylbutanoyle ; 2-amino-3-méthylpentanoyle ; N-acétyl-2-amino-3-méthylpentanoyle ; pentanoyle ; 3-méthylpentanoyle ; 4-méthyl-pentanoyle ; 2-amino-4-méthyl-pentanoyle ; N-acétyl-2-amino-4-méthylpentanoyle ; 2-amino-4,4-diméthylpentanoyle ; N-acétyl-2-amino-4,4-diméthylpentanoyle ; 2-aminopentanoyle ; N-acétyl-2-aminopentanoyle ; 2-amino-5-méthylhexanoyle ; N-acétyl-2-amino-5-méthylhexanoyle ; 2-hydroxy-3-méthyl-butanoyle ; 2-méthoxy-3-méthylbutanoyle ; 2-hydroxy-3,3-diméthylbutanoyle ; 2-méthoxy-3,3-diméthylbutanoyle ; 2-hydroxy-3-méthylpentanoyle ; 2-méthoxy-3-méthylpentanoyle ; 2-hydroxy-4-méthylpentanoyle ; 2-méthoxy-4-méthylpentanoyle ; 2-hydroxy-4,4-diméthylpentanoyle ; 2-méthoxy-4,4-diméthyl-pentanoyle ; 2-hydroxypentanoyle ; 2-méthoxypentanoyle ; 2-hydroxy-5-méthylhexanoyle ; 2-méthoxy-5-méthylhexanoyle.

**9.** Composé suivant l'une quelconque des revendications 2 à 8, dans lequel, dans le groupe $(X)_0$, $R^{16}$ représente un atome d'hydrogène et $R^{17}$ représente un atome d'hydrogène ; un groupe alkyle en $C_1$ à $C_4$ qui peut être substitué avec un substituant OH, $NR^{22}R^{22}$, $COOR^{22}$ ou $CONR^{22}$ ; ou un groupe Ar-(alkyle en $C_1$ à $C_4$), dans lequel le groupe aryle peut être substitué avec un substituant $R^{21}$, où chacun des groupes $R^{21}$ et $R^{22}$ est indépendamment tel que défini dans la revendication 1.

**10.** Composé suivant la revendication 9, dans lequel, dans le groupe $(X)_0$, $R^{16}$ représente un atome d'hydrogène et $R^{17}$ est choisi entre un atome d'hydrogène ou un groupe alkyle en $C_1$ à $C_4$ simple.

**11.** Composé suivant la revendication 10, dans lequel, dans le groupe $(X)_0$, $R^{16}$ et $R^{17}$ représentent des atomes d'hydrogène et o est égal à zéro ou un.

**12.** Composé suivant l'une quelconque des revendications 2 à 11, dans lequel , dans le groupe $(W)_n$, W comprend un groupe O, S, $SO_2$, C(O) ou NH et n est égal à zéro ou un.

**13.** Composé suivant la revendication 12, dans lequel, dans le groupe $(W)_n$, W représente un groupe C(O) ou NH et n est égal à zéro ou un.

**14.** Composé suivant la revendication 13, dans lequel, dans le groupe $(W)_n$, W représente un groupe NH et n est égal à zéro ou un.

**15.** Composé suivant l'une quelconque des revendications 1 à 14, dans lequel la combinaison de substituants $U-(V)_m-(W)_n-(X)_o-Y$ ou $U-(V)_m-(W)_n-(X)_o$ comprend :

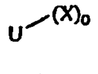
'n' = 0
'm' = 0

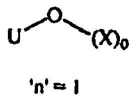
'n' = 1
'W' = O
'm' = 0

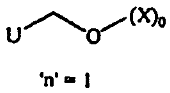
'n' = 1
'W' = O
'V' = $CH_2$
'm' = 1

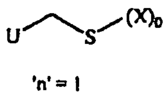
'n' = 1
'W' = S
'V' = $CH_2$
'm' = 1

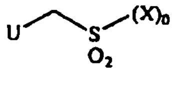
'n' = 1
'W' = $SO_2$
'V' = $CH_2$
'm' = 1

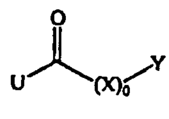
'n' = 1
'W' = C(O)
'm' = 0

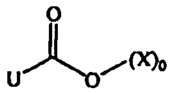

'n' = 1
'W' = O
'V' = C(O)
'm' = 1

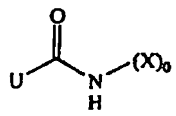

'n' = 1
'W' = $NR^{18}$, $R^{18}$ = 'H'
'V' = C(O)
'm' = 1

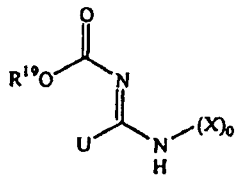

'n' = 1
'W' = $NR^{18}$, $R^{18}$ = 'H'
'V' = C=N-C(O)-$OR^{19}$
'm' = 1

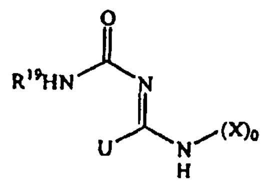

'n' = 1
'W' = $NR^{18}$, $R^{18}$ = 'H'
'V' = C=N-C(O)-$NHR^{19}$
'm' = 1

**16.** Composé suivant l'une quelconque des revendications 1 à 15, dans lequel la combinaison de substituants $U-(V)_m-(W)_n-(X)_o-Y$ comprend :

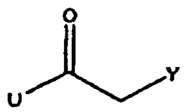

$(X)_0$ = '$CH_2$'
'n' = 1
'W' = C(O)
'm' = 0

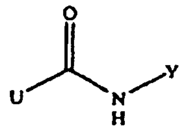

$(X)_0$ = '-'
'n' = 1
'W' = $NR^{18}$, $R^{18}$ = 'H'
'V' = C(O)
'm' = 1

**17.** Composé suivant l'une quelconque des revendications 1 à 16, qui est un inhibiteur de la cathepsine K et dans lequel, indépendamment ou dans n'importe quelle combinaison :

Y représente un groupe $CHR^{11}CO$ dans lequel $R^{11}$ est choisi entre des groupes alkyle en $C_0$ à $C_7$, Ar-(alkyle en $C_0$ à $C_7$) et cycloalkyle en $C_3$ à $C_6$ ; ou bien Y comprend un groupe :

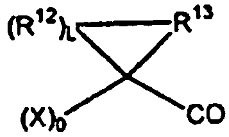

dans lequel $R^{12}$ et $R^{13}$ représentent chacun un groupe $CR^{14}R^{15}$ et chacun des groupes $R^{14}$ et $R^{15}$ est choisi, indépendamment, entre des groupes alkyle en $C_0$ à $C_7$ et Ar-(alkyle en $C_0$ à $C_7$), par exemple un atome d'hydrogène, une chaîne alkyle droite ou ramifiée, une chaîne hétéroalkyle droite ou ramifiée, une chaîne arylalkyle facultativement substituée ou une chaîne aryle hétéroalkyle facultativement substituée et L représente un nombre de un à quatre ; et le groupe U comprend un hétérocycle ou groupe Ar penta- ou hexagonal saturé ou insaturé facultativement substitué ou un hétérocycle ou groupe Ar octo- à décagonal saturé ou insaturé facultativement substitué.

**18.** Composé suivant la revendication 17, dans lequel $(X)_0$-Y représente un groupe :

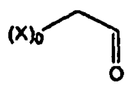 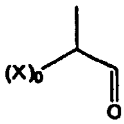 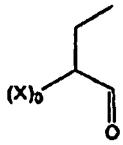 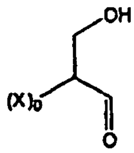 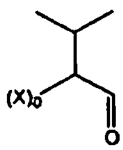 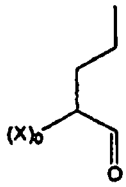 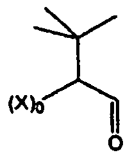

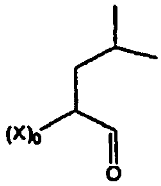 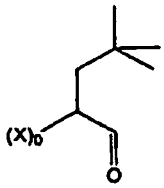 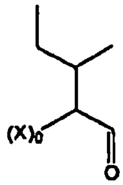 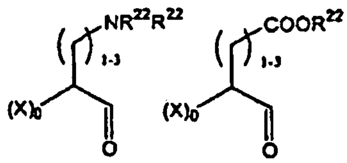 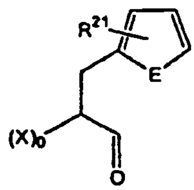

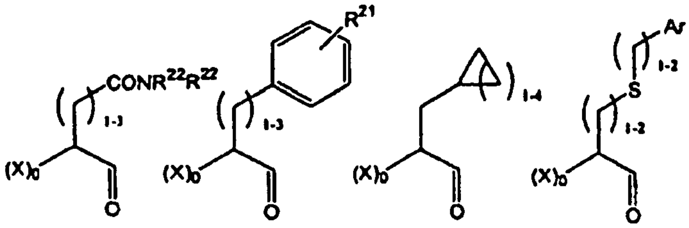 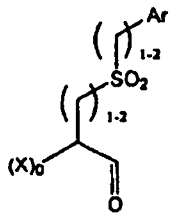 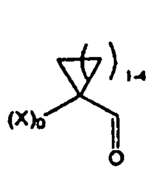

dans lequel E, $R^{21}$, $R^{22}$ et Ar répondent aux définitions précitées ; n'importe lequel de ces groupes pouvant être substitué avec un ou plusieurs substituants halogéno, de préférence fluoro.

**19.** Composé suivant la revendication 17, dans lequel, dans le groupe Y, $R^{11}$ représente un groupe alkyle en $C_1$ à $C_4$, qui peut être substitué avec un substituant cycloalkylméthyle ou halogéno, ou bien $R^{11}$ est choisi parmi des groupes cycloalkyl-1-carbonyle ou bien $R^{11}$ est choisi parmi des groupes Ar-(alkyle en $C_1$ à $C_4$), dans lesquels le groupe aryle peut être substitué avec $R^{21}$ ; où $R^{21}$ répond à la définition figurant dans la revendication 1.

**20.** Composé suivant la revendication 19, dans lequel, dans le groupe Y, $R^{11}$ représente un groupe alkyle simple droit ou ramifié, facultativement substitué avec un ou plusieurs substituants halogéno.

**21.** Composé suivant la revendication 20, dans lequel, dans le groupe Y, $R^{11}$ représente un groupe $ArCH_2$-, dans lequel le noyau aromatique est un hétérocycle monocyclique facultativement substitué.

**22.** Composé suivant l'une quelconque des revendications 17 à 21, dans lequel le groupe $(X)_0$-Y comprend un groupe :

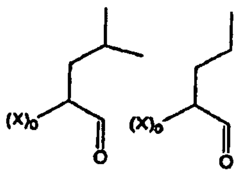 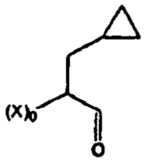 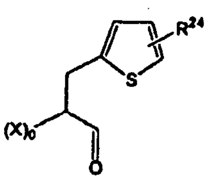 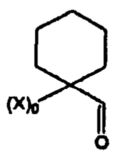

dans lequel $R^{24}$ est choisi entre un atome d'hydrogène, des groupes méthyle, méthoxy, éthyle, isopropyle, F, Cl et dans lequel n'importe lequel des groupes alkyle peut être substitué avec un ou plusieurs substituants F ou Cl.

**23.** Composé suivant l'une quelconque des revendications 17 à 22, dans lequel le groupe U comprend un groupe :

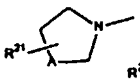 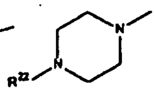 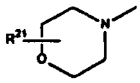 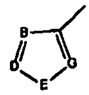 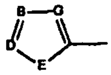 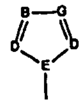

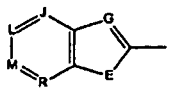 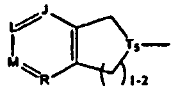 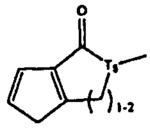 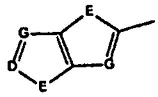 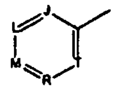

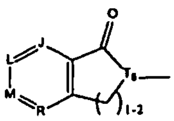 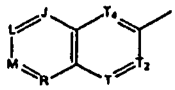 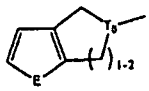 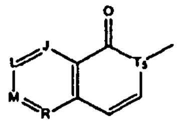

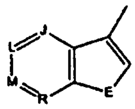 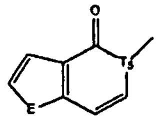 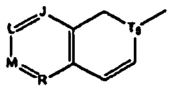 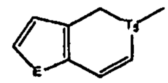

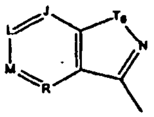 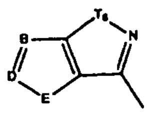 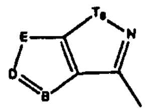

où $R^{21}$, $R^{22}$, A, B, D, E, G, J, L, M, R, T, $T_2$, $T_4$, $T_5$ et $T_6$ sont tels que définis dans la revendication 1.

**24.** Composé suivant la revendication 23, dans lequel U comprend un groupe :

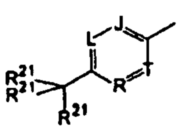 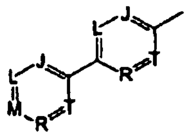 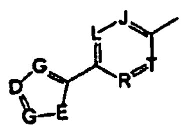 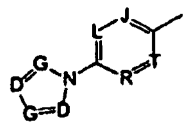 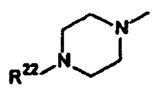

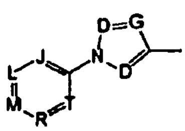 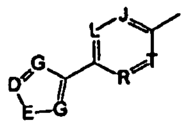 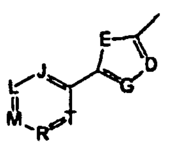 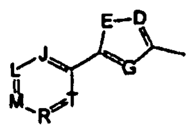 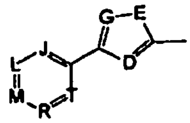

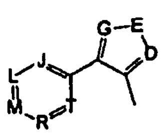 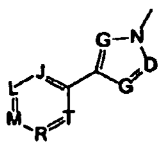 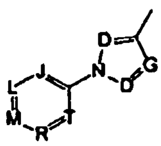 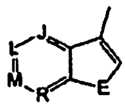 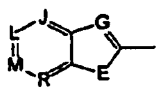

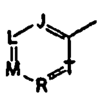 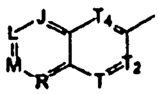 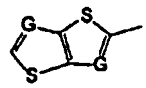

où $R^{21}$, $R^{22}$, D, E, G, J, L, M, R, T, $T_2$ et $T_4$ répondent aux définition précitées.

**25.** Composé suivant la revendication 24, dans lequel U comprend un groupe :

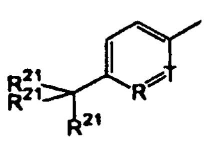 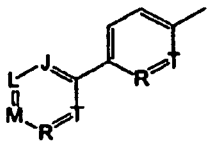 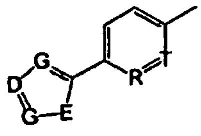 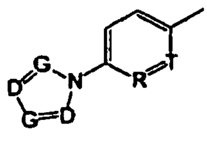 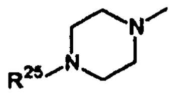

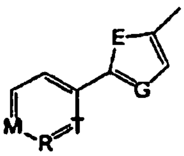 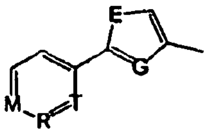 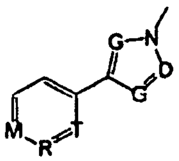 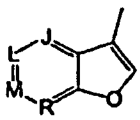 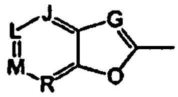

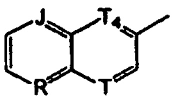 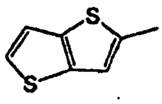 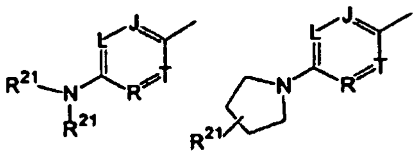 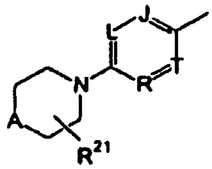

où $R^{21}$, $R^{25}$, D, E, G, J, L, M, R, T, et $T_4$ sont tels que définis dans la revendication 1.

**26.** Composé suivant la revendication 25, dans lequel U comprend un groupe :

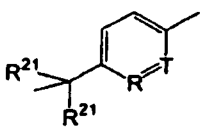 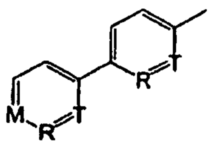 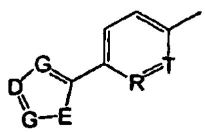 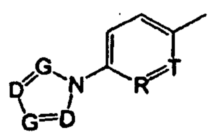

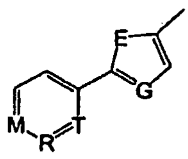 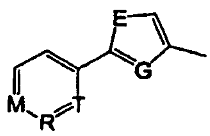 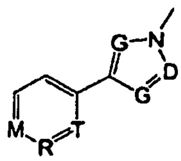

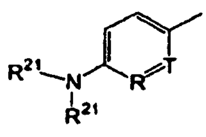 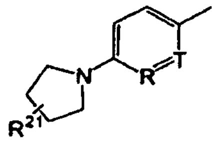 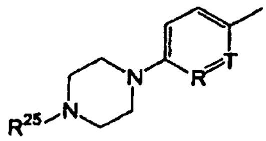

où $R^{21}$, $R^{25}$, D, E, G, M, R et T sont tels que définis dans la revendication 1.

**27.** Composé suivant l'une quelconque des revendications 1 à 16, qui est un inhibiteur de la cathepsine S et dans lequel, indépendamment ou dans n'importe quelle combinaison, $(X)_0$-Y comprend un groupe :

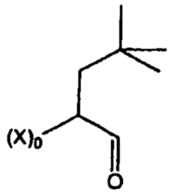 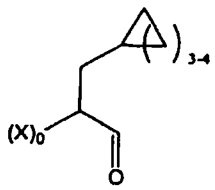 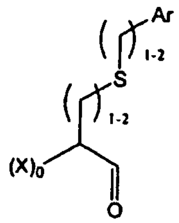 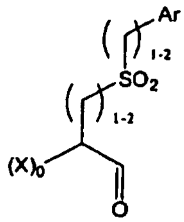

dans lequel $(X)_0$ est tel que défini dans la revendication 1, et
U comprend un groupe :

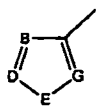 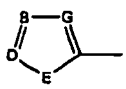 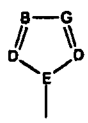 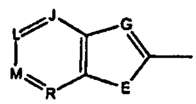 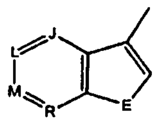

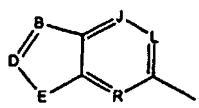 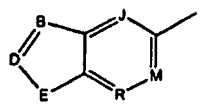 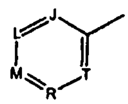 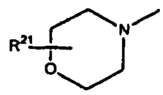

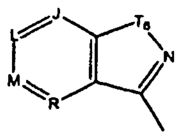 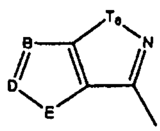 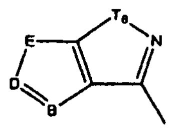

où $R^{21}$, B, D, E, G, J, L, M, R et $T_6$ sont tels que définis dans la revendication 1.

**28.** Composé suivant la revendication 27, dans lequel U comprend un hétérocycle pentagonal insaturé facultativement substitué ou un noyau aromatique condensé en position 6,5 ou 5,5.

**29.** Composé suivant la revendication 28, dans lequel U comprend un groupe :

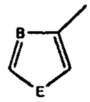 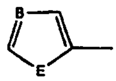 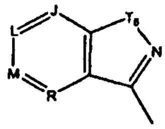 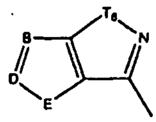 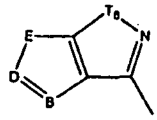

où B, D, E, J, L, M, R et $T_6$ sont tels que définis dans la revendication 1.

**30.** Composé suivant l'une quelconque des revendications 1 à 24, qui est un inhibiteur de la cathepsine L et dans lequel, indépendamment ou dans n'importe quelle combinaison,
$(X)_0$-Y comprend un groupe :

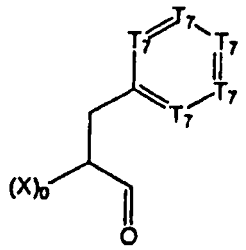

dans lequel $T_7$ est choisi entre des groupes CH, N ou $CR^{21}$ dans lequel $R^{21}$ est tel que défini dans la revendication 1 ; et U comprend un hétérocycle pentagonal insaturé facultativement substitué ou un noyau aromatique condensé en position 6,6, 6,5 ou 5,6, ou un groupe Ar méta-substitué.

**31.** Composé suivant la revendication 30, dans lequel, dans le substituant $T_7$, le substituant $R^{21}$ est choisi parmi des combinaisons de substitution de noyaux uniques et multiples de groupes Me, F, Cl, OH et OMe.

**32.** Composé suivant la revendication 30 ou la revendication 31, dans lequel U comprend un groupe :

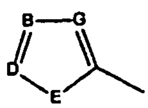 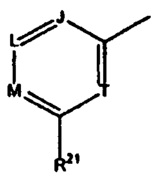 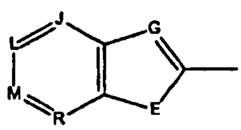

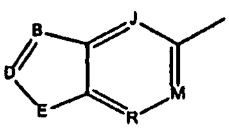 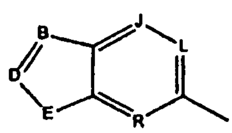 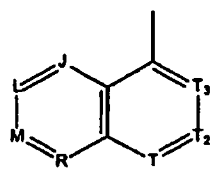

où $R^{21}$, B, D, E, G, J, L, M, R, T, $T_2$ et $T_3$ sont tels que définis dans la revendication 1.

**33.** Composé suivant l'une quelconque des revendications 30 à 32, dans lequel U comprend un groupe :

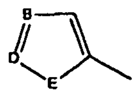 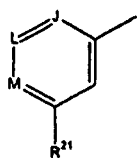 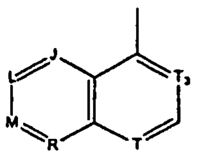

dans lequel E est choisi entre un atome d'oxygène et un groupe N-éthyle, D est choisi entre un atome d'azote et un groupe $CCH_3$, B est choisi entre un atome d'azote et un groupe $CCH_3$, $R^{21}$ est choisi entre des groupes halogéno, OMe, $CF_3$, $OCF_3$, $CH_3NH_2$ et J, L, M, R, T et $T_3$ sont tels que définis dans la revendication 1.

**34.** Composé suivant l'une quelconque des revendications 1 à 33, qui est un isomère cis-bicyclique.

**35.** Composé choisi entre les suivants :
(3aR,6aS)-N-{(1S)-3-méthyl-1-[6-oxo-4-(pyridine-2-carbonyl)-hexahydropyrrolo[3,2-b]pyrrole-1-carbonyl]-butyl}-benzamide ;
(3aR,6aS)-N-{(1S)-3-méthyl-1-[6-oxo-4-(pyridine-2-carbonyl)-hexahydropyrrolo[3,2-C]pyrrole-1-carbonyl]-butyl}-benzamide ;
(3aR,6aS)-N-{(1S)-3-méthyl-1-[6-oxo-4-(pyridine-2-carbonyl)-hexahydropyrrolo-2-oxa-1,4-diaza-pentalène-1-carbonyl]-butyl}-benzamide ;
{3-méthyl-1-[6-oxo-4-(pyridine-2-sulfonyl)-hexahydro-pyrrolo[3,2-b]pyrrole-1-carbonyl]-butyl}-amide d'acide naphtalène-2-carboxylique ;
{3-méthyl-1-[6-oxo-4-(pyridine-2-sulfonyl)-hexahydro-pyrrolo[3,2-b]pyrrole-1-carbonyl]-butyl}-amide d'acide quinoléine-[b]thiophène-2-carboxylique ;
{3-méthyl-1-[6-oxo-4-(pyridine-2-sulfonyl)-hexahydro-pyrrolo[3,2-b]pyrrole-1-carbonyl]-butyl}amide d'acide benzo-[b]thiophène-2-carboxylique ;
{3-méthyl-1-[6-oxo-4-(pyridine-2-sulfonyl)-hexahydro-pyrrolo[3,2-b]pyrrole-1-carbonyl]-butyl}-amide d'acide benzofuranne-2-carboxylique ;
{3-méthyl-1-[6-oxo-4-(pyridine-2-sulfonyl)-hexahydro-pyrrolo[3,2-b]pyrrole-1-carbonyl]-butyl}-amide d'acide biphényl-4-carboxylique ;
4-tertiobutyl-N-{3-méthyl-1-[6-oxo-4-(pyridine-2-sulfonyl)-hexahydro-pyrrolo[3,2-b]pyrrole-1-carbonyl]-butyl}-benzamide ;
4-diméthylamino-N-{3-méthyl-1-[6-oxo-4-(pyridine-2-sulfonyl)-hexahydro-pyrrolo[3,2-b]pyrrole-1-carbonyl]-butyl}-benzamide ;
{3-méthyl-1-[6-oxo-4-(pyridine-2-sulfonyl)-hexahydro-pyrrolo[3,2-b]pyrrole-1-carbonyl]-butyl}-amide d'acide 7-méthoxy-benzofuranne-2-carboxylique ;
ester benzylique d'acide {3-méthyl-1-[6-oxo-4-(pyridine-2-sulfonyl)-hexahydro-pyrrolo[3,2-b]pyrrole-1-carbonyl]-butyl}-carbamique ;
{3-méthyl-1-[6-oxo-4-(pyridine-2-sulfonyl)-hexahydro-pyrrolo[3,2-b]pyrrole-1-carbonyl]-butyl}-amide d'acide 5-méthoxy-benzofuranne-2-carboxylique ;
{3-méthyl-1-[6-oxo-4-(pyridine-2-sulfonyl)-hexahydro-pyrrolo[3,2-b]pyrrole-1-carbonyl]-butyl}-amide d'acide thiéno-[3,2-b]thiophène-2-carboxylique ;
{3-méthyl-1-[6-oxo-4-(pyridine-2-sulfonyl)-hexahydro-pyrrolo[3,2-b]pyrrole-1-carbonyl]-butyl}-amide d'acide 3-méthyl- benzofuranne-2-carboxylique ;
{3-méthyl-1-[6-oxo-4-(pyridine-2-sulfonyl)-hexahydro-pyrrolo[3,2-b]pyrrole-1-carbonyl]-butyl}-amide d'acide quinoxaline-2-carboxylique ;
{3-méthyl-1-[6-oxo-4-(pyridine-2-sulfonyl)-hexahydro-pyrrolo[3,2-b]pyrrole-1-carbonyl]-butyl}-amide d'acide benzo-[1,3]dioxole-5-carboxylique ;
{3-méthyl-1-[6-oxo-4-(pyridine-2-sulfonyl)-hexahydro-pyrrolo[3,2-b]pyrrole-1-carbonyl]-butyl}-amide d'acide quinoléine-6-carboxylique ;
{3-méthyl-1-[6-oxo-4-(pyridine-2-sulfonyl)-hexahydro-pyrrolo[3,2-b]pyrrole-1-carbonyl]-butyl}-amide d'acide furanne-2-carboxylique ;
{3-méthyl-1-[6-oxo-4-(pyridine-2-sulfonyl)-hexahydro-pyrrolo[3,2-b]pyrrole-1-carbonyl]-butyl}-amide d'acide thiophène-2-carboxylique ;
N-{3-méthyl-1-[6-oxo-4-(pyridine-2-sulfonyl)-hexahydro-pyrrolo[3,2-b]pyrrole-1-carbonyl]-butyl}-4-trifluorométhyl -benzamide ;
{3-méthyl-1-[6-oxo-4-(pyridine-2-sulfonyl)-hexahydro-pyrrolo[3,2-b]pyrrole-1-carbonyl]-butyl}-amide d'acide furanne-3-carboxylique ;
{3-méthyl-1-[6-oxo-4-(pyridine-2-sulfonyl)-hexahydro-pyrrolo[3,2-b]pyrrole-1-carbonyl]-butyl}-amide d'acide thiophène-3-carboxylique ;
N-{3-méthyl-1-[6-oxo-4-(pyridine-2-sulfonyl)-hexahydro-pyrrolo[3,2-b]pyrrole-1-carbonyl]-butyl}-4-phénoxy-benzamide ;
N-{3-méthyl-1-[6-oxo-4-(pyridine-2-sulfonyl)-hexahydro-pyrrolo[3,2-b]pyrrole-1-carbonyl]-butyl}-4-(4-méthyl-pipérazine-1-yl)-benzamide ;
4-méthyl-N-{3-méthyl-1-[6-oxo-4-(pyridine-2-sulfonyl)-hexahydro-pyrrolo[3,2-b]pyrrole-1-carbonyl]-butyl}-benzamide ;
4-méthoxy-N-{3-méthyl-1-[6-oxo-4-(pyridine-2-sulfonyl)-hexahydro-pyrrolo[3,2-b]pyrrole-1-carbonyl]-butyl}-benzamide ;
4-isopropyl-N-{3-méthyl-1-[6-oxo-4-(pyridine-2-sulfonyl)-hexahydro-pyrrolo[3,2-b]pyrrole-1-carbonyl]-butyl}-benzamide ;
N-{3-méthyl-1-[6-oxo-4-(pyridine-2-sulfonyl)-hexahydro-pyrrolo[3,2-b]pyrrole-1-carbonyl]-butyl}-4-vinyl-benzamide ;
4-imidazole-1-yl-N-{3-méthyl-1-[6-oxo-4-(pyridine-2-sulfonyl)-hexahydro-pyrrolo[3,2-b]pyrrole-1-carbonyl]-butyl}-benzamide ;
N-{3-méthyl-1-[6-oxo-4-(pyridine-2-sulfonyl)-hexahydro-pyrrolo[3,2-b]pyrrole-1-carbonyl]-butyl}-4-thiophène-2-yl-benzamide ;
N-{3-méthyl-1-[6-oxo-4-(pyridine-2-sulfonyl)-hexahydro-pyrrolo[3,2-b]pyrrole-1-carbonyl]-butyl}-4-oxazole-5-yl-benzamide ;
{3-méthyl-1-[6-oxo-4-(pyridine-2-sulfonyl)-hexahydro-pyrrolo[3,2-b]pyrrole-1-carbonyl]-butyl}-amide d'acide 5-phényl-thiophène-2-carboxylique ;
N-{3-méthyl-1-[6-oxo-4-(pyridine-2-sulfonyl)-hexahydro-pyrrolo[3,2-b]pyrrole-1-carbonyl]-butyl}-4-trifluorométhoxy-benzamide ;
{3-méthyl-1-[6-oxo-4-(pyridine-2-sulfonyl)-hexahydro-pyrrolo[3,2-b]pyrrole-1-carbonyl]-butyl}-amide d'acide 5-pyridine-2-yl-thiophène-2-carboxylique ;
4-difluorométhoxy-N-{3-méthyl-1-[6-oxo-4-(pyridine-2-sulfonyl)-hexahydro-pyrrolo[3,2-b]pyrrole-1-carbonyl]-butyl}-benzamide ;
N-{3-méthyl-1-[6-oxo-4-(pyridine-2-sulfonyl)-hexahydro-pyrrolo[3,2-b]pyrrole-1-carbonyl]-butyl}-4-morpholine-4-yl-benzamide ;
{1-[6-oxo-4-(pyridine-2-sulfonyl)-hexahydro-pyrrolo-[3,2-b]pyrrole-1-carbonyl]-butyl}-amide d'acide naphtalène-2-carboxylique ;
{1-[6-oxo-4-(pyridine-2-sulfonyl)-hexahydro-pyrrolo-[3,2-b]pyrrole-1-carbonyl]-butyl}-amide d'acide quinoléine-2-carboxylique ;
{1-[6-oxo-4-(pyridine-2-sulfonyl)-hexahydro-pyrrolo-[3,2-b]pyrrole-1-carbonyl]-butyl}-amide d'acide benzo[b]-thiophène-2-carboxylique ;
{1-[6-oxo-4-(pyridine-2-sulfonyl)-hexahydro-pyrrolo-[3,2-b]pyrrole-1-carbonyl]-butyl}-amide d'acide benzo-furanne-2-carboxylique ;
{1-[6-oxo-4-(pyridine-2-sulfonyl)-hexahydro-pyrrolo-[3,2-b]pyrrole-1-carbonyl]-butyl}-amide d'acide biphényl-4-carboxylique ;
4-tertiobutyl-N-{1-[6-oxo-4-(pyridine-2-sulfonyl)-hexahydro-pyrrolo[3,2-b]pyrrole-1-carbonyl]-butyl}-benzamide ;
4-diméthylamino-N-{1-[6-oxo-4-(pyridine-2-sulfonyl)-hexahydro-pyrrolo[3,2-b]pyrrole-1-carbonyl]-butyl}-benzamide ;
{1-[6-oxo-4-(pyridine-2-sulfonyl)-hexahydro-pyrrolo-[3,2-b]pyrrole-1-carbonyl]-butyl}-amide d'acide 7-méthoxy-benzofuranne-2-carboxylique ;
ester benzylique d'acide {1-[6-oxo-4-(pyridine-2-sulfonyl)-hexahydro-pyrrolo[3,2-b]pyrrole-1-carbonyl]-butyl}-carbamique ;
{1-[6-oxo-4-(pyridine-2-sulfonyl)-hexahydro-pyrrolo-[3,2-b]pyrrole-1-carbonyl]-butyl}-amide d'acide 5-méthoxy-benzofuranne-2-carboxylique ;
{1-[6-oxo-4-(pyridine-2-sulfonyl)-hexahydro-pyrrolo-[3,2-b]pyrrole-1-carbonyl]-butyl}-amide d'acide thiéno-[3,2-b]thiophène-2-carboxylique ;
{1-[6-oxo-4-(pyridine-2-sulfonyl)-hexahydro-pyrrolo-[3,2-b]pyrrole-1-carbonyl]-butyl}-amide d'acide 3-méthyl-benzofuranne-2-carboxylique ;
{1-[6-oxo-4-(pyridine-2-sulfonyl)-hexahydro-pyrrolo-[3,2-b]pyrrole-1-carbonyl]-butyl}-amide d'acide quinoxaline-2-carboxylique ;
{1-[6-oxo-4-(pyridine-2-sulfonyl)-hexahydro-pyrrolo-[3,2-b]pyrrole-1-carbonyl]-butyl}-amide d'acide benzo[1,3]-dioxole-5-carboxylique ;

{1-[6-oxo-4-(pyridine-2-sulfonyl)-hexahydro-pyrrolo-[3,2-b]pyrrole-1-carbonyl]-butyl}-amide d'acide quinoléine-6-carboxylique ;
{1-[6-oxo-4-(pyridine-2-sulfonyl)-hexahydro-pyrrolo-[3,2-b]pyrrole-1-carbonyl]-butyl}-amide d'acide furanne-2-carboxylique ;
{1-[6-oxo-4-(pyridine-2-sulfonyl)-hexahydro-pyrrolo-[3,2-b]pyrrole-1-carbonyl]-butyl}-amide d'acide thiophène-2-carboxylique ;
N-{1-[6-oxo-4-(pyridine-2-sulfonyl)-hexahydro-pyrrolo-[3,2-b]pyrrole-1-carbonyl)-butyl}-4-trifluorométhyl-benzamide ;
{1-[6-oxo-4-(pyridine-2-sulfonyl)-hexahydro-pyrrolo-[3,2-b]pyrrole-1-carbonyl]-butyl}-amide d'acide furanne-3-carboxylique ; .
{1-[6-oxo-4-(pyridine-2-sulfonyl)-hexahydro-pyrrolo-[3,2-b]pyrrole-1-carbonyl]-butyl}-amide d'acide thiophène-3-carboxylique ;
N-{1-[6-oxo-4-(pyridine-2-sulfonyl)-hexahydro-pyrrolo-[3,2-b]pyrrole-1-carbonyl]-butyl}-4-phénoxy-benzamide ;
N-{1-[6-oxo-4-(pyridine-2-sulfonyl)-hexahydro-pyrrolo-[3,2-b]pyrrole-1-carbonyl]-butyl}-4-(4-méthyl-pipérazine-1-yl)-benzamide ;
4-méthyl-N-{1-[6-oxo-4-(pyridine-2-sulfonyl)-hexahydro-pyrrolo[3,2-b]pyrrole-1-carbonyl]-butyl}-benzamide ;
4-méthoxy-N-{1-[6-oxo-4-(pyridine-2-sulfonyl)-hexahydro-pyrrolo[3,2-b]pyrrole-1-carbonyl]-butyl}-benzamide ;
4-isopropyl-N-{1-[6-oxo-4-(pyridine-2-sulfonyl)-hexahydro-pyrrolo[3,2-b]pyrrole-1-carbonyl]-butyl}-benzamide ;
N-{1-[6-oxo-4-(pyridine-2-sulfonyl)-hexahydro-pyrrolo-[3,2-b]pyrrole-1-carbonyl]-butyl}-4-vinyl-benzamide ;
4-imidazole-1-yl-N-{1-[6-oxo-4-(pyridine-2-sulfonyl)-hexahydro-pyrrolo[3,2-b]pyrrole-1-carbonyl]-butyl}-benzamide ;
N-{1-[6-oxo-4-(pyridine-2-sulfonyl)-hexahydro-pyrrolo-[3,2-b]pyrrole-1-carbonyl]-butyl}-4-thiophène-2-yl-benzamide ;
N-{1-[6-oxo-4-(pyridine-2-sulfonyl)-hexahydro-pyrrolo-[3,2-b]pyrrole-1-carbonyl]-butyl}-4-oxazole-5-yl-benzamide ;
{1-[6-oxo-4-(pyridine-2-sulfonyl)-hexahydro-pyrrolo-[3,2-b]pyrrole-1-carbonyl]-butyl}-amide d'acide 5-phényl-thiophène-2-carboxylique ;
N-{1-[6-oxo-4-(pyridine-2-sulfonyl)-hexahydro-pyrrolo-[3,2-b]pyrrole-1-carbonyl]-butyl}-4-trifluorométhoxy-benzam ide ;
{1-[6-oxo-4-(pyridine-2-sulfonyl)-hexahydro-pyrrolo-[3,2-b]pyrrole-1-carbonyl]-butyl}-amide d'acide 5-pyridine-2-yl-thiophène-2-carboxylique ;
4-difluorométhoxy-N-{1-[6-oxo-4-(pyridine-2-sulfonyl)-hexahydro-pyrrolo-[3,2-b]pyrrole-1-carbonyl]-butyl}-benzamide ;
N-{1-[6-oxo-4-(pyridine-2-sulfonyl)-hexahydro-pyrrolo-[3,2-b]pyrrole-1-carbonyl]-butyl}-4-morpholine-4-yl-benzamide ;
{3-méthyl-1-[6-oxo-4-(1-oxypyridine-2-sulfonyl)-hexahydro-pyrrolo[3,2-b]pyrrole-1-carbonyl]-butyl}-amide d'acide naphtalène-2-carboxylique ;
{3-méthyl-1-[6-oxo-4-(1-oxypyridine-2-sulfonyl)-hexahydro-pyrrolo[3,2-b]pyrrole-1-carbonyl]-butyl}-amide d'acide quinoléine-2-carboxylique ;
{3-méthyl-1-[6-oxo-4-(1-oxypyridine-2-sulfonyl)-hexahydro-pyrrolo[3,2-b]pyrrole-1-carbonyl]-butyl}-amide d'acide benzo[b]thiophène-2-carboxylique ;
{3-méthyl-1-[6-oxo-4-(1-oxypyridine-2-sulfonyl)-hexahydro-pyrrolo[3,2-b]pyrrole-1-carbonyl]-butyl}-amide d'acide benzofuranne-2-carboxylique ;
{3-méthyl-1-[6-oxo-4-(1-oxypyridine-2-sulfonyl)-hexahydro-pyrrolo[3,2-b]pyrrole-1-carbonyl]-butyl}-amide d'acide biphényl-4-carboxylique ;
4-tertiobutyl-N-{3-méthyl-1-[6-oxo-4-(1-oxypyridine-2-sulfonyl)-hexahydro-pyrrolo[3,2-b]pyrrole-1-carbonyl]-butyl}-benzamide ;
4-diméthylamino-N-{3-méthyl-1-[6-oxo-4-(1-oxypyridine-2-sulfonyl)-hexahydro-pyrrolo[3,2-b]pyrrole-1-carbonyl]-butyl}-benzamide ;
{3-méthyl-1-[6-oxo-4-(1-oxypyridine-2-sulfonyl)-hexahydro-pyrrolo[3,2-b]pyrrole-1-carbonyl]-butyl}-amide d'acide 7-méthoxy-benzofuranne-2-carboxylique ;
ester benzylique d'acide {3-méthyl-1-[6-oxo-4-(1-oxy-pyridine-2-sulfonyl)-hexahydro-pyrrolo[3,2-b]pyrrole-1-carbonyl]-butyl}-carbamique ;
{3-méthyl-1-[6-oxo-4-(1-oxypyridine-2-sulfonyl)-hexahydro-pyrrolo[3,2-b]pyrrole-1-carbonyl]-butyl}-amide d'acide 5-méthoxybenzofuranne-2-carboxylique ;
{3-méthyl-1-[6-oxo-4-(1-oxypyridine-2-sulfonyl)-hexahydro-pyrrolo[3,2-b]pyrrole-1-carbonyl]-butyl}-amide d'acide thiéno[3,2-b]thiophène-2-carboxylique ;
{3-méthyl-1-[6-oxo-4-(1-oxypyridine-2-sulfonyl)-hexahydro-pyrrolo[3,2-b]pyrrole-1-carbonyl]-butyl}-amide d'acide 3-méthyl-benzofuranne-2-carboxylique ;

{3-méthyl-1-[6-oxo-4-(1-oxypyridine-2-sulfonyl)-hexahydro-pyrrolo[3,2-b]pyrrole-1-carbonyl]-butyl}-amide d'acide quinoxaline-2-carboxylique ;
{3-méthyl-1-[6-oxo-4-(1-oxypyridine-2-sulfonyl)-hexahydro-pyrrolo[3,2-b]pyrrole-1-carbonyl]-butyl}-amide d'acide benzo[1,3]dioxole-5-carboxylique ;
{3-méthyl-1-[6-oxo-4-(1-oxypyridine-2-sulfonyl)-hexahydro-pyrrolo[3,2-b]pyrrole-1-carbonyl]-butyl}-amide d'acide quinoléine-6-carboxylique ;
{3-méthyl-1-[6-oxo-4-(1-oxypyridine-2-sulfonyl)-hexahydro-pyrrolo[3,2-b]pyrrole-1-carbonyl]-butyl}-amide d'acide furanne-2-carboxylique ;
{3-méthyl-1-[6-oxo-4-(1-oxypyridine-2-sulfonyl)-hexahydro-pyrrolo[3,2-b]pyrrole-1-carbonyl]-butyl}-amide d'acide thiophène-2-carboxylique ;
N-{3-méthyl-1-[6-oxo-4-(1-oxypyridine-2-sulfonyl)-hexahydro-pyrrolo[3,2-b]pyrrole-1-carbonyl]-butyl}-4-trifluorométhyl-benzamide ;
{3-méthyl-1-[6-oxo-4-(1-oxypyridine-2-sulfonyl)-hexahydro-pyrrolo[3,2-b]pyrrole-1-carbonyl]-butyl}-amide d'acide furanne-3-carboxylique ;
{3-méthyl-1-[6-oxo-4-(1-oxypyridine-2-sulfonyl)-hexahydro-pyrrolo[3,2-b]pyrrole-1-carbonyl]-butyl}-amide d'acide thiophène-3-carboxylique ;
N-{3-méthyl-1-[6-oxo-4-(1-oxypyridine-2-sulfonyl)-hexahydro-pyrrolo[3,2-b]pyrrole-1-carbonyl]-butyl}-4-phénoxy-benzamide ;
N-{3-méthyl-1-[6-oxo-4-(1-oxypyridine-2-sulfonyl)-hexahydro-pyrrolo[3,2-b]pyrrole-1-carbonyl]-butyl}-4-(4-méthyl-pipérazine-1-yl)-benzamide ;
4-méthyl-N-{3-méthyl-1-[6-oxo-4-(1-oxypyridine-2-sulfonyl)-hexahydro-pyrrolo[3,2-b]pyrrole-1-carbonyl]-butyl}-benzamide ;
4-méthoxy-N-{3-méthyl-1-[6-oxo-4-(1-oxypyridine-2-sulfonyl)-hexahydro-pyrrolo[3,2-b]pyrrole-1-carbonyl]-butyl}-benzamide ;
4-isopropyl-N-{3-méthyl-1-[6-oxo-4-(1-oxypyridine-2-sulfonyl)-hexahydro-pyrrolo[3,2-b]pyrrole-1-carbonyl]-butyl}-benzamide ;
N-{3-méthyl-1-[6-oxo-4-(1-oxypyridine-2-sulfonyl)-hexahydro-pyrrolo[3,2-b]pyrrole-1-carbonyl]-butyl}-4-vinyl-benzamide ;
4-imidazole-1-yl-N-{3-méthyl-1-[6-oxo-4-(1-oxypyridine-2-sulfonyl)-hexahydro-pyrrolo[3,2-b]pyrrole-1-carbonyl]-butyl}-benzamide ;
N-{3-méthyl-1-[6-oxo-4-(1-oxypyridine-2-sulfonyl)-hexa-hydro-pyrrolo[3,2-b]pyrrole-1-carbonyl]-butyl}-4-thiophène -2-yl-benzamide ;
N-{3-méthyl-1-[6-oxo-4-(1-oxypyridine-2-sulfonyl)-hexa-hydro-pyrrolo[3,2-b]pyrrole-1-carbonyl]-butyl}-4-oxazole-5-yl-benzamide ;
{3-méthyl-1-[6-oxo-4-(1-oxypyridine-2-sulfonyl)-hexa-hydro-pyrrolo[3,2-b]pyrrole-1-carbonyl]-butyl}-amide d'acide 5-phényl-thiophène-2-carboxylique ;
N-{3-méthyl-1-[6-oxo-4-(1-oxypyridine-2-sulfonyl)-hexa-hydro-pyrrolo[3,2-b]pyrrole-1-carbonyl]-butyl}-4-trifluorométhoxy-benzamide ;
{3-méthyl-1-[6-oxo-4-(1-oxypyridine-2-sulfonyl)-hexa-hydro-pyrrolo[3,2-b]pyrrole-1-carbonyl]-butyl}-amide d'acide 5-pyridine-2-yl-thiophène-2-carboxylique ;
4-difluorométhoxy-N-{3-méthyl-1-[6-oxo-4-(1-oxypyridine-2-sulfonyl)-hexa-hydro-pyrrolo[3,2-b]pyrrole-1-carbonyl] -butyl}-benzamide ;
N-{3-méthyl-1-[6-oxo-4-(1-oxypyridine-2-sulfonyl)-hexa-hydro-pyrrolo[3,2-b]pyrrole-1-carbonyl]-butyl}-4-morpholin e-4-yl-benzamide ;
{1-[6-oxo-4-(1-oxypyridine-2-sulfonyl)-hexahydro-pyrrolo-[3,2-b]pyrrole-1-carbonyl]-butyl}-amide d'acide naphtalène-2-carboxylique ;
{1-[6-oxo-4-(1-oxypyridine-2-sulfonyl)-hexahydro-pyrrolo-[3,2-b]pyrrole-1-carbonyl]-butyl}-amide d'acide quinoléine-2-carboxylique ;
{1-[6-oxo-4-(1-oxypyridine-2-sulfonyl)-hexahydro-pyrrolo-[3,2-b]pyrrole-1-carbonyl]-butyl}-amide d'acide benzo[b]-thiophène-2-carboxylique ;
{1-[6-oxo-4-(1-oxypyridine-2-sulfonyl)-hexahydro-pyrrolo-[3,2-b]pyrrole-1-carbonyl]-butyl}-amide d'acide benzo-furanne-2-carboxylique ;
{1-[6-oxo-4-(1-oxypyridine-2-sulfonyl)-hexahydro-pyrrolo-[3,2-b]pyrrole-1-carbonyl]-butyl}-amide d'acide biphényl-4-carboxylique ;
4-tertiobutyl-N-{1-[6-oxo-4-(1-oxypyridine-2-sulfonyl)-hexahydro-pyrrolo-[3,2-b]pyrrole-1-carbonyl]-butyl}-benzamide ;
4-diméthylamino-N-{1-[6-oxo-4-(1-oxypyridine-2-sulfonyl)-hexahydro-pyrrolo-[3,2-b]pyrrole-1-carbonyl]-butyl}-benzamide ;

{1-[6-oxo-4-(1-oxypyridine-2-sulfonyl)-hexahydro-pyrrolo-[3,2-b]pyrrole-1-carbonyl]-butyl}-amide d'acide 7-méthoxy- benzofuranne-2-carboxylique ;
ester benzylique d'acide {1-[6-oxo-4-(1-oxypyridine-2-sulfonyl)-hexahydro-pyrrolo-[3,2-b]pyrrole-1-carbonyl]-butyl}-carbamique ;
{1-[6-oxo-4-(1-oxypyridine-2-sulfonyl)-hexahydro-pyrrolo-[3,2-b]pyrrole-1-carbonyl]-butyl}-amide d'acide 5-méthoxy- benzofuranne-2-carboxylique ;
{1-[6-oxo-4-(1-oxypyridine-2-sulfonyl)-hexahydro-pyrrolo-[3,2-b]pyrrole-1-carbonyl]-butyl}-amide d'acide thiéno-[3,2- b]thiophène-2-carboxylique ;
{1-[6-oxo-4-(1-oxypyridine-2-sulfonyl)-hexahydro-pyrrolo-[3,2-b]pyrrole-1-carbonyl]-butyl}-amide d'acide 3-méthyl-benzofuranne-2-carboxylique ;
{1-[6-oxo-4-(1-oxypyridine-2-sulfonyl)-hexahydro-pyrrolo-[3,2-b]pyrrole-1-carbonyl]-butyl}-amide d'acide quinoxaline- 2-carboxylique ;
{1-[6-oxo-4-(1-oxypyridine-2-sulfonyl)-hexahydro-pyrrolo-[3,2-b]pyrrole-1-carbonyl]-butyl}-amide d'acide benzo[1,3]- dioxole-5-carboxylique ;
{1-[6-oxo-4-(1-oxypyridine-2-sulfonyl)-hexahydro-pyrrolo-[3,2-b]pyrrole-1-carbonyl]-butyl}-amide d'acide quinoléine-6-carboxylique ;
{1-[6-oxo-4-(1-oxypyridine-2-sulfonyl)-hexahydro-pyrrolo-[3,2-b]pyrrole-1-carbonyl]-butyl}-amide d'acide furanne-2- carboxylique ;
{1-[6-oxo-4-(1-oxypyridine-2-sulfonyl)-hexahydro-pyrrolo-[3,2-b]pyrrole-1-carbonyl]-butyl}-amide d'acide thiophène-2- carboxylique ;
N-{1-[6-oxo-4-(1-oxypyridine-2-sulfonyl)-hexahydro-pyrrolo-[3,2-b]pyrrole-1-carbonyl]-butyl}-4-trifluorométhyl-ben zamide ;
{1-[6-oxo-4-(1-oxypyridine-2-sulfonyl)-hexahydro-pyrrolo-[3,2-b]pyrrole-1-carbonyl]-butyl}-amide d'acide furanne-3- carboxylique ;
{1-[6-oxo-4-(1-oxypyridine-2-sulfonyl)-hexahydro-pyrrolo-[3,2-b]pyrrole-1-carbonyl]-butyl}-amide d'acide thiophène-3- carboxylique ;
N-{1-[6-oxo-4-(1-oxypyridine-2-sulfonyl)-hexahydro-pyrrolo-[3,2-b]pyrrole-1-carbonyl]-butyl}-4-phénoxy-benzamide ;
N-{1-[6-oxo-4-(1-oxypyridine-2-sulfonyl)-hexahydro-pyrrolo-[3,2-b]pyrrole-1-carbonyl]-butyl}-4-(4-méthyl-pipérazine-1-yl)-benzamide ;
4-méthyl-N-{1-[6-oxo-4-(1-oxypyridine-2-sulfonyl)-hexa-hydro-pyrrolo[3,2-b]pyrrole-1-carbonyl]-butyl}-benzamide ;
4-méthoxy-N-{1-[6-oxo-4-(1-oxypyridine-2-sulfonyl)-hexa-hydro-pyrrolo[3,2-b]pyrrole-1-carbonyl]-butyl}-benzamide ;
4-isopropyl-N-{1-[6-oxo-4-(1-oxypyridine-2-sulfonyl)-hexa-hydro-pyrrolo[3,2-b]pyrrole-1-carbonyl]-butyl}-benzamide ;
N-{1-[6-oxo-4-(1-oxypyridine-2-sulfonyl)-hexahydro-pyrrolo-[3,2-b]pyrrole-1-carbonyl]-butyl}-4-vinyl-benzamide ;
4-imidazole-1-yl-N-{1-[6-oxo-4-(1-oxypyridine-2-sulfonyl)-hexahydro-pyrrolo[3,2-b]pyrrole-1-carbonyl]-butyl}-benzamide ;
N-{1-[6-oxo-4-(1-oxypyridine-2-sulfonyl)-hexahydro-pyrrolo-[3,2-b]pyrrole-1-carbonyl]-butyl}-4-thiophène-2-yl-benzamide ;
N-{1-[6-oxo-4-(1-oxypyridine-2-sulfonyl)-hexa-hydro-pyrrolo[3,2-b]pyrrole-1-carbonyl]-butyl}-4-oxazole-5-yl-benzamide ;
{1-[6-oxo-4-(1-oxypyridine-2-sulfonyl)-hexahydro-pyrrolo-[3,2-b]pyrrole-1-carbonyl]-butyl}-amide d'acide 5-phényl-thiophène-2-carboxylique ;
N-{1-[6-oxo-4-(1-oxypyridine-2-sulfonyl)-hexahydro-pyrrolo-[3,2-b]pyrrole-1-carbonyl]-butyl}-4-trifluorométhoxy-benzamide ;
{1-[6-oxo-4-(1-oxypyridine-2-sulfonyl)-hexahydro-pyrrolo-[3,2-b]pyrrole-1-carbonyl]-butyl}-amide d'acide 5-pyridine-2-yl- thiophène-2-carboxylique ;
4-difluorométhoxy-N-{1-[6-oxo-4-(1-oxypyridine-2-sulfonyl)-hexa-hydro-pyrrolo[3,2-b]pyrrole-1-carbonyl]-butyl}-benzamide ;
N-{1-[6-oxo-4-(1-oxypyridine-2-sulfonyl)-hexahydro-pyrrolo-[3,2-b]pyrrole-1-carbonyl]-butyl}-4-morpholine-4-yl-benzamide ;
{3-méthyl-1-[6-oxo-4-(pyridine-3-sulfonyl)-hexahydro-pyrrolo[3,2-b]pyrrole-1-carbonyl]-butyl}-amide d'acide naphtalène-2-carboxylique ;
{3-méthyl-1-[6-oxo-4-(pyridine-3-sulfonyl)-hexahydro-pyrrolo[3,2-b]pyrrole-1-carbonyl]-butyl}-amide d'acide quinoléine-2-carboxylique ;
{3-méthyl-1-[6-oxo-4-(pyridine-3-sulfonyl)-hexahydro-pyrrolo[3,2-b]pyrrole-1-carbonyl]-butyl}-amide d'acide benzo-[b]thiophène-2-carboxylique ;

{3-méthyl-1-[6-oxo-4-(pyridine-3-sulfonyl)-hexahydro-pyrrolo[3,2-b]pyrrole-1-carbonyl]-butyl}-amide d'acide benzofuranne-2-carboxylique ;
{3-méthyl-1-[6-oxo-4-(pyridine-3-sulfonyl)-hexahydro-pyrrolo[3,2-b]pyrrole-1-carbonyl]-butyl}-amide d'acide biphényl-4-carboxylique ;
4-tertiobutyl-N-{3-méthyl-1-[6-oxo-4-(pyridine-3-sulfonyl)-hexahydro-pyrrolo[3,2-b]pyrrole-1-carbonyl]-butyl}-benzamide ;
4-diméthylamino-N-{3-méthyl-1-[6-oxo-4-(pyridine-3-sulfonyl)-hexahydro-pyrrolo[3,2-b]pyrrole-1-carbonyl]-butyl}-benzamide ;
{3-méthyl-1-[6-oxo-4-(pyridine-3-sulfonyl)-hexahydro-pyrrolo[3,2-b]pyrrole-1-carbonyl]-butyl}-amide d'acide 7-méthoxybenzofuranne-2-carboxylique ;
ester benzylique d'acide {3-méthyl-1-[6-oxo-4-(pyridine-3-sulfonyl)-hexahydro-pyrrolo[3,2-b]pyrrole-1-carbonyl]-butyl}-carbamique ;
{3-méthyl-1-[6-oxo-4-(pyridine-3-sulfonyl)-hexahydro-pyrrolo[3,2-b]pyrrole-1-carbonyl]-butyl}-amide d'acide 5-méthoxybenzofuranne-2-carboxylique ;
{3-méthyl-1-[6-oxo-4-(pyridine-3-sulfonyl)-hexahydro-pyrrolo[3,2-b]pyrrole-1-carbonyl]-butyl}-amide thiéno-[3,2-b]-thiophène-2-carboxylique ;
{3-méthyl-1-[6-oxo-4-(pyridine-3-sulfonyl)-hexahydro-pyrrolo[3,2-b]pyrrole-1-carbonyl]-butyl}-amide d'acide 3-méthyl-benzofuranne-2-carboxylique ;
{3-méthyl-1-[6-oxo-4-(pyridine-3-sulfonyl)-hexahydro-pyrrolo[3,2-b]pyrrole-1-carbonyl]-butyl}-amide d'acide quinoxaline-2-carboxylique ;
{3-méthyl-1-[6-oxo-4-(pyridine-3-sulfonyl)-hexahydro-pyrrolo[3,2-b]pyrrole-1-carbonyl]-butyl}-amide d'acide benzo[1,3]-dioxole-5-carboxylique ;
{3-méthyl-1-[6-oxo-4-(pyridine-3-sulfonyl)-hexahydro-pyrrolo[3,2-b]pyrrole-1-carbonyl]-butyl}-amide d'acide quinoléine-6-carboxylique ;
{3-méthyl-1-[6-oxo-4-(pyridine-3-sulfonyl)-hexahydro-pyrrolo[3,2-b]pyrrole-1-carbonyl]-butyl}-amide d'acide furanne-2-carboxylique ;
{3-méthyl-1-[6-oxo-4-(pyridine-3-sulfonyl)-hexahydro-pyrrolo[3,2-b]pyrrole-1-carbonyl]-butyl}-amide d'acide thiophène-2-carboxylique ;
N-{3-méthyl-1-[6-oxo-4-(pyridine-3-sulfonyl)-hexahydro-pyrrolo[3,2-b]pyrrole-1-carbonyl]-butyl}-4-fluorométhyl-benzamide ;
{3-méthyl-1-[6-oxo-4-(pyridine-3-sulfonyl)-hexahydro-pyrrolo[3,2-b]pyrrole-1-carbonyl]-butyl}-amide d'acide furanne-3-carboxylique ;
{3-méthyl-1-[6-oxo-4-(pyridine-3-sulfonyl)-hexahydro-pyrrolo[3,2-b]pyrrole-1-carbonyl]-butyl}-amide d'acide thiophène-3-carboxylique ;
N-{3-méthyl-1-[6-oxo-4-(pyridine-3-sulfonyl)-hexahydro-pyrrolo[3,2-b]pyrrole-1-carbonyl]-butyl}-4-phénoxy-benzamide ;
N-{3-méthyl-1-[6-oxo-4-(pyridine-3-sulfonyl)-hexahydro-pyrrolo[3,2-b]pyrrole-1-carbonyl]-butyl}-4-(4-méthyl-pipérazine-1-yl)-benzamide ;
4-méthyl-N-{3-méthyl-1-[6-oxo-4-(pyridine-3-sulfonyl)-hexahydro-pyrrolo[3,2-b]pyrrole-1-carbonyl]-butyl}-benzamide ;
4-méthoxy-N-{3-méthyl-1-[6-oxo-4-(pyridine-3-sulfonyl)-hexahydro-pyrrolo[3,2-b]pyrrole-1-carbonyl]-butyl}-benzamide ;
4-isopropyl-N-{3-méthyl-1-[6-oxo-4-(pyridine-3-sulfonyl)-hexahydro-pyrrolo[3,2-b]pyrrole-1-carbonyl]-butyl}-benzamide ;
N-{3-méthyl-1-[6-oxo-4-(pyridine-3-sulfonyl)-hexahydro-pyrrolo[3,2-b]pyrrole-1-carbonyl]-butyl}-4-vinyl-benzamide ;
4-imidazole-1-yl-N-{3-méthyl-1-[6-oxo-4-(pyridine-3-sulfonyl)-hexahydro-pyrrolo[3,2-b]pyrrole-1-carbonyl]-butyl}-benzamide ;
N-{3-méthyl-1-[6-oxo-4-(pyridine-3-sulfonyl)-hexahydro-pyrrolo[3,2-b]pyrrole-1-carbonyl]-butyl}-4-thiophène-2-yl-benzamide ;
N-{3-méthyl-1-[6-oxo-4-(pyridine-3-sulfonyl)-hexahydro-pyrrolo[3,2-b]pyrrole-1-carbonyl]-butyl}-4-oxazole-5-yl-benzamide ;
{3-méthyl-1-[6-oxo-4-(pyridine-3-sulfonyl)-hexahydro-pyrrolo[3,2-b]pyrrole-1-carbonyl]-butyl}-amide d'acide 5-phényl-thiophène-2-carboxylique ;
N-{3-méthyl-1-[6-oxo-4-(pyridine-3-sulfonyl)-hexahydro-pyrrolo[3,2-b]pyrrole-1-carbonyl]-butyl}-4-trifluorométhoxy-benzamide ;
{3-méthyl-1-[6-oxo-4-(pyridine-3-sulfonyl)-hexahydro-pyrrolo[3,2-b]pyrrole-1-carbonyl]-butyl}-amide d'acide 5-pyridine-2-yl-thiophène-2-carboxylique ;

4-difluorométhoxy-N-{3-méthyl-1-[6-oxo-4-(pyridine-3-sulfonyl)-hexahydro-pyrrolo[3,2-b]pyrrole-1-carbonyl]-butyl}-benzamide ;
N-{3-méthyl-1-[6-oxo-4-(pyridine-3-sulfonyl)-hexahydro-pyrrolo[3,2-b]pyrrole-1-carbonyl]-butyl}-4-morpholine-4-yl-benzamide ;
{1-[6-oxo-4-(pyridine-3-sulfonyl)-hexahydro-pyrrolo[3,2-b]-pyrrole-1-carbonyl]-butyl}-amide d'acide naphtalène-2-carboxylique ;
{1-[6-oxo-4-(pyridine-3-sulfonyl)-hexahydro-pyrrolo[3,2-b]-pyrrole-1-carbonyl]-butyl}-amide d'acide quinoléine-2-carboxylique ;
{1-[6-oxo-4-(pyridine-3-sulfonyl)-hexahydro-pyrrolo[3,2-b]-pyrrole-1-carbonyl]-butyl}-amide d'acide benzo[b]thiophène-2-carboxylique ;
{1-[6-oxo-4-(pyridine-3-sulfonyl)-hexahydro-pyrrolo[3,2-b]-pyrrole-1-carbonyl]-butyl}-amide d'acide benzofuranne-2-c arboxylique ;
{1-[6-oxo-4-(pyridine-3-sulfonyl)-hexahydro-pyrrolo[3,2-b]-pyrrole-1-carbonyl]-butyl}-amide d'acide biphényl-4-carboxylique ;
4-tertiobutyl-N-{1-[6-oxo-4-(pyridine-3-sulfonyl)-hexa-hydro-pyrrolo[3,2-b]-pyrrole-1-carbonyl]-butyl}-benzamide ;
4-diméthylamino-N-{1-[6-oxo-4-(pyridine-3-sulfonyl)-hexa-hydro-pyrrolo[3,2-b]-pyrrole-1-carbonyl]-butyl}-benzamide ;
{1-[6-oxo-4-(pyridine-3-sulfonyl)-hexahydro-pyrrolo[3,2-b]-pyrrole-1-carbonyl]-butyl}-amide d'acide 7-méthoxy-benzo-furanne-2-carboxylique ;
ester benzylique d'acide {1-[6-oxo-4-(pyridine-3-sulfonyl)-hexahydro-pyrrolo[3,2-b]-pyrrole-1-carbonyl]-butyl}-carbamique ;
{1-[6-oxo-4-(pyridine-3-sulfonyl)-hexahydro-pyrrolo[3,2-b]-pyrrole-1-carbonyl]-butyl}-amide d'acide 5-méthoxy-benzofuranne-2-carboxylique ;
{1-[6-oxo-4-(pyridine-3-sulfonyl)-hexahydro-pyrrolo[3,2-b]-pyrrole-1-carbonyl]-butyl}-amide d'acide thiéno[3,2-b]-hiophène-2-carboxylique ;
{1-[6-oxo-4-(pyridine-3-sulfonyl)-hexahydro-pyrrolo[3,2-b]-pyrrole-1-carbonyl]-butyl}-amide d'acide 3-méthyl-benzo- furanne-2-carboxylique ;
{1-[6-oxo-4-(pyridine-3-sulfonyl)-hexahydro-pyrrolo[3,2-b]-pyrrole-1-carbonyl]-butyl}-amide d'acide quinoxaline-2-carboxylique ;
{1-[6-oxo-4-(pyridine-3-sulfonyl)-hexahydro-pyrrolo[3,2-b]-pyrrole-1-carbonyl]-butyl}-amide d'acide benzo[1,3]-dioxole-5-carboxylique ;
{1-[6-oxo-4-(pyridine-3-sulfonyl)-hexahydro-pyrrolo[3,2-b]-pyrrole-1-carbonyl]-butyl}-amide d'acide quinoléine-6-carboxylique ;
{1-[6-oxo-4-(pyridine-3-sulfonyl)-hexahydro-pyrrolo[3,2-b]-pyrrole-1-carbonyl]-butyl}-amide d'acide furanne-2-carboxylique ;
{1-[6-oxo-4-(pyridine-3-sulfonyl)-hexahydro-pyrrolo[3,2-b]-pyrrole-1-carbonyl]-butyl}-amide d'acide thiophène-2-carboxylique ;
N-{1-[6-oxo-4-(pyridine-3-sulfonyl)-hexahydro-pyrrolo[3,2-b]-pyrrole-1-carbonyl]-butyl}-4-trifluorométhyl-benzamide
{1-[6-oxo-4-(pyridine-3-sulfonyl)-hexahydro-pyrrolo[3,2-b]-pyrrole-1-carbonyl]-butyl}-amide d'acide furanne-3-carboxylique ;
{1-[6-oxo-4-(pyridine-3-sulfonyl)-hexahydro-pyrrolo[3,2-b]-pyrrole-1-carbonyl]-butyl}-amide d'acide thiophène-3-carboxylique ;
N-{1-[6-oxo-4-(pyridine-3-sulfonyl)-hexahydro-pyrrolo[3,2-b]-pyrrole-1-carbonyl]-butyl}-4-phénoxybenzamide ;
N-{1-[6-oxo-4-(pyridine-3-sulfonyl)-hexahydro-pyrrolo-[3,2-b]-pyrrole-1-carbonyl]-butyl}-4-(4-méthyl-pipérazine-1yl)-yl)-benzamide ;
4-méthyl-N-{1-[6-oxo-4-(pyridine-3-sulfonyl)-hexahydro-pyrrolo[3,2-b]-pyrrole-1-carbonyl]-butyl}-benzamide ;
4-méthoxy-N-{1-[6-oxo-4-(pyridine-3-sulfonyl)-hexahydro-pyrrolo[3,2-b]-pyrrole-1-carbonyl]-butyl}-benzamide ;
4-isopropyl-N-{1-[6-oxo-4-(pyridine-3-sulfonyl)-hexahydro-pyrrolo[3,2-b]-pyrrole-1-carbonyl]-butyl}-benzamide ;
N-{1-[6-oxo-4-(pyridine-3-sulfonyl)-hexahydro-pyrrolo-[3,2-b]-pyrrole-1-carbonyl]-butyl}-4-vinyl-benzamide ;
4-imidazole-1-yl-N-{1-[6-oxo-4-(pyridine-3-sulfonyl)-hexa-hydro-pyrrolo[3,2-b]pyrrole-1-carbonyl]-butyl}-benzamide ;
N-{1-[6-oxo-4-(pyridine-3-sulfonyl)-hexahydro-pyrrolo[3,2-b]-pyrrole-1-carbonyl]-butyl}-4-thiophène-2-yl-benzamide ;
N-{1-[6-oxo-4-(pyridine-3-sulfonyl)-hexahydro-pyrrolo-[3,2-b]-pyrrole-1-carbonyl]-butyl}-4-oxazole-5-yl-benzamide ;
{1-[6-oxo-4-(pyridine-3-sulfonyl)-hexahydro-pyrrolo[3,2-b]-pyrrole-1-carbonyl]-butyl}-amide d'acide 5-phényl-thiophène-2-carboxylique ;

N-{1-[6-oxo-4-(pyridine-3-sulfonyl)-hexahydro-pyrrolo-[3,2-b]-pyrrole-1-carbonyl]-butyl}-4-trifluorométhoxy-benzamide ;
{1-[6-oxo-4-(pyridine-3-sulfonyl)-hexahydro-pyrrolo[3,2-b]-pyrrole-1-carbonyl]-butyl}-amide d'acide 5-pyridine-2-yl-thiophène-2-carboxylique ;
4-difluorométhoxy-N-{1-[6-oxo-4-(pyridine-3-sulfonyl)-hexa-hydro-pyrrolo[3,2-b]-pyrrole-1-carbonyl]-butyl}-benzamide ;
N-{1-[6-oxo-4-(pyridine-3-sulfonyl)-hexahydro-pyrrolo-[3,2-b]-pyrrole-1-carbonyl]-butyl}-4-morpholine-4-yl-benzamide ;
{3-méthyl-1-[6-oxo-4-(1-oxypyridine-3-sulfonyl)-hexahydro-pyrrolo[3,2-b]-pyrrole-1-carbonyl]-butyl}-amide d'acide naphtalène-2-carboxylique ;
{3-méthyl-1-[6-oxo-4-(1-oxypyridine-3-sulfonyl)-hexahydro-pyrrolo[3,2-b]-pyrrole-1-carbonyl]-butyl}-amide d'acide quinoléine-2-carboxylique ;
{3-méthyl-1-[6-oxo-4-(1-oxypyridine-3-sulfonyl)-hexahydro-pyrrolo[3,2-b]-pyrrole-1-carbonyl]-butyl}-amide d'acide benzo[b]thiophène-2-carboxylique ;
{3-méthyl-1-[6-oxo-4-(1-oxypyridine-3-sulfonyl)-hexahydro-pyrrolo[3,2-b]-pyrrole-1-carbonyl]-butyl}-amide d'acide benzofuranne-2-carboxylique ;
{3-méthyl-1-[6-oxo-4-(1-oxypyridine-3-sulfonyl)-hexahydro-pyrrolo[3,2-b]-pyrrole-1-carbonyl]-butyl}-amide d'acide biphényl-4-carboxylique ;
4-tertiobutyl-N-{3-méthyl-1-[6-oxo-4-(1-oxypyridine-3-sulfonyl)-hexahydro-pyrrolo[3,2-b]-pyrrole-1-carbonyl]-butyl}-benzamide ;
4-diméthylamino-N-{3-méthyl-1-[6-oxo-4-(1-oxypyridine-3-sulfonyl)-hexahydro-pyrrolo[3,2-b]-pyrrole-1-carbonyl]-butyl}-benzamide ;
{3-méthyl-1-[6-oxo-4-(1-oxypyridine-3-sulfonyl)-hexahydro-pyrrolo[3,2-b]-pyrrole-1-carbonyl]-butyl}-amide d'acide 7-méthoxy-benzofuranne-2-carboxylique ;
ester benzylique d'acide {3-méthyl-1-[6-oxo-4-(1-oxy-pyridine-3-sulfonyl)-hexahydro-pyrrolo[3,2-b]-pyrrole-1-carbonyl]-butyl}-carbamique ;
{3-méthyl-1-[6-oxo-4-(1-oxypyridine-3-sulfonyl)-hexahydro-pyrrolo[3,2-b]-pyrrole-1-carbonyl]-butyl}-amide d'acide 5-méthoxybenzofuranne-2-carboxylique ;
{3-méthyl-1-[6-oxo-4-(1-oxypyridine-3-sulfonyl)-hexahydro-pyrrolo[3,2-b]-pyrrole-1-carbonyl]-butyl}-amide d'acide thiéno[3,2-b]thiophène-2-carboxylique ;
{3-méthyl-1-[6-oxo-4-(1-oxypyridine-3-sulfonyl)-hexahydro-pyrrolo[3,2-b]-pyrrole-1-carbonyl]-butyl}-amide d'acide 3-méthylbenzofuranne-2-carboxylique ;
{3-méthyl-1-[6-oxo-4-(1-oxypyridine-3-sulfonyl)-hexahydro-pyrrolo[3,2-b]-pyrrole-1-carbonyl]-butyl}-amide d'acide quinoxaline-2-carboxylique ;
{3-méthyl-1-[6-oxo-4-(1-oxypyridine-3-sulfonyl)-hexahydro-pyrrolo[3,2-b]-pyrrole-1-carbonyl]-butyl}-amide d'acide benzo[1,3]dioxole-5-carboxylique ;
{3-méthyl-1-[6-oxo-4-(1-oxypyridine-3-sulfonyl)-hexahydro-pyrrolo[3,2-b]-pyrrole-1-carbonyl]-butyl}-amide d'acide quinoléine-6-carboxylique ;
{3-méthyl-1-[6-oxo-4-(1-oxypyridine-3-sulfonyl)-hexahydro-pyrrolo[3,2-b]-pyrrole-1-carbonyl]-butyl}-amide d'acide furanne-2-carboxylique ;
{3-méthyl-1-[6-oxo-4-(1-oxypyridine-3-sulfonyl)-hexahydro-pyrrolo[3,2-b]-pyrrole-1-carbonyl]-butyl}-amide d'acide thiophène-2-carboxylique ;
N-{3-méthyl-1-[6-oxo-4-(1-oxypyridine-3-sulfonyl)-hexa-hydro-pyrrolo[3,2-b]-pyrrole-1-carbonyl]-butyl}-4-trifluorométhyl-benzamide ;
{3-méthyl-1-[6-oxo-4-(1-oxypyridine-3-sulfonyl)-hexahydro-pyrrolo[3,2-b]-pyrrole-1-carbonyl]-butyl}-amide d'acide furanne-3-carboxylique ;
{3-méthyl-1-[6-oxo-4-(1-oxypyridine-3-sulfonyl)-hexahydro-pyrrolo[3,2-b]-pyrrole-1-carbonyl]-butyl}-amide d'acide thiophène-3-carboxylique ;
N-{3-méthyl-1-[6-oxo-4-(1-oxypyridine-3-sulfonyl)-hexahydro-pyrrolo[3,2-b]-pyrrole-1-carbonyl]-butyl}-4-phénoxy-benzamide ;
N-{3-méthyl-1-[6-oxo-4-(1-oxypyridine-3-sulfonyl)-hexa-hydro-pyrrolo[3,2-b]-pyrrole-1-carbonyl]-butyl}-4-(4-méthyl-pipérazine-1-yl)-benzamide ;
4-méthyl-N-{3-méthyl-1-[6-oxo-4-(1-oxypyridine-3-sulfonyl)-hexahydro-pyrrolo[3,2-b]-pyrrole-1-carbonyl]-butyl}-benzamide ;
4-méthoxy-N-{3-méthyl-1-[6-oxo-4-(1-oxypyridine-3-sulfonyl)-hexahydro-pyrrolo[3,2-b]-pyrrole-1-carbonyl]-butyl}-benzamide ;
4-isopropyl-N-{3-méthyl-1-[6-oxo-4-(1-oxypyridine-3-sulfonyl)-hexahydro-pyrrolo[3,2-b]-pyrrole-1-carbonyl]-butyl}-benzamide ;

N-{3-méthyl-1-[6-oxo-4-(1-oxypyridine-3-sulfonyl)-hexa-hydro-pyrrolo[3,2-b]-pyrrole-1-carbonyl]-butyl}-4-vinyl-benzamide ;
4-imidazole-1-yl-N-{3-méthyl-1-[6-oxo-4-(1-oxypyridine-3-sulfonyl)-hexahydro-pyrrolo[3,2-b]-pyrrole-1-carbonyl]-butyl}-benzamide ;
N-{3-méthyl-1-[6-oxo-4-(1-oxypyridine-3-sulfonyl)-hexahydro-pyrrolo[3,2-b]-pyrrole-1-carbonyl]-butyl}-4-thiophène-2-yl-benzamide ;
N-{3-méthyl-1-[6-oxo-4-(1-oxypyridine-3-sulfonyl)-hexahydro-pyrrolo[3,2-b]-pyrrole-1-carbonyl]-butyl}-4-oxazole-5-yl-benzamide ;
{3-méthyl-1-[6-oxo-4-(1-oxypyridine-3-sulfonyl)-hexahydro-pyrrolo[3,2-b]-pyrrole-1-carbonyl]-butyl}-amide d'acide 5-phényl-thiophène-2-carboxylique ;
N-{3-méthyl-1-[6-oxo-4-(1-oxypyridine-3-sulfonyl)-hexahydro-pyrrolo[3,2-b]-pyrrole-1-carbonyl]-butyl}-4-trifluorométhoxy-benzamide ;
{3-méthyl-1-[6-oxo-4-(1-oxypyridine-3-sulfonyl)-hexahydro-pyrrolo[3,2-b]-pyrrole-1-carbonyl]-butyl}-amide d'acide 5-pyridine-2-yl-thiophène-2-carboxylique ;
4-difluorométhoxy-N-{3-méthyl-1-[6-oxo-4-(1-oxypyridine-3-sulfonyl)-hexahydro-pyrrolo[3,2-b]-pyrrole-1-carbonyl]-butyl}-benzamide ;
N-{3-méthyl-1-[6-oxo-4-(1-oxypyridine-3-sulfonyl)-hexahydro-pyrrolo[3,2-b]-pyrrole-1-carbonyl]-butyl}-4-morpholine-4-yl-benzamide ;
{1-[6-oxo-4-(1-oxypyridine-3-sulfonyl)-hexahydro-pyrrolo-[3,2-b]-pyrrole-1-carbonyl]-butyl}-amide d'acide naphtalène-2-carboxylique ;
{1-[6-oxo-4-(1-oxypyridine-3-sulfonyl)-hexahydro-pyrrolo-[3,2-b]-pyrrole-1-carbonyl]-butyl}-amide d'acide quinoléine-2-carboxylique ;
{1-[6-oxo-4-(1-oxypyridine-3-sulfonyl)-hexahydro-pyrrolo-[3,2-b]-pyrrole-1-carbonyl]-butyl}-amide d'acide benzo[b]-thiophène-2-carboxylique ;
{1-[6-oxo-4-(1-oxypyridine-3-sulfonyl)-hexahydro-pyrrolo-[3,2-b]-pyrrole-1-carbonyl]-butyl}-amide d'acide benzofuranne-2-carboxylique ;
{1-[6-oxo-4-(1-oxypyridine-3-sulfonyl)-hexahydro-pyrrolo-[3,2-b]-pyrrole-1-carbonyl]-butyl}-amide d'acide biphényl-4-carboxylique ;
4-tertiobutyl-N-{1-[6-oxo-4-(1-oxypyridine-3-sulfonyl)-hexahydro-pyrrolo-[3,2-b]-pyrrole-1-carbonyl]-butyl}-benzamide ;
4-diméthylamino-N-{1-[6-oxo-4-(1-oxypyridine-3-sulfonyl)-hexahydro-pyrrolo-[3,2-b]-pyrrole-1-carbonyl]-butyl}-benzamide ;
{1-[6-oxo-4-(1-oxypyridine-3-sulfonyl)-hexahydro-pyrrolo-[3,2-b]-pyrrole-1-carbonyl]-butyl}-amide d'acide 7-méthoxy-benzofuranne-2-carboxylique ;
ester benzylique d'acide {1-[6-oxo-4-(1-oxypyridine-3-sulfonyl)-hexahydro-pyrrolo-[3,2-b]-pyrrole-1-carbonyl]-butyl}-carbamique ;
{1-[6-oxo-4-(1-oxypyridine-3-sulfonyl)-hexahydro-pyrrolo-[3,2-b]-pyrrole-1-carbonyl]-butyl}-amide d'acide 5-méthoxy-benzofuranne-2-carboxylique ;
{1-[6-oxo-4-(1-oxypyridine-3-sulfonyl)-hexahydro-pyrrolo-[3,2-b]-pyrrole-1-carbonyl]-butyl}-amide d'acide thiéno-[3,2-b]thiophène-2-carboxylique ;
{1-[6-oxo-4-(1-oxypyridine-3-sulfonyl)-hexahydro-pyrrolo-[3,2-b]-pyrrole-1-carbonyl]-butyl}-amide d'acide 3-méthyl-benzofuranne-2-carboxylique ;
{1-[6-oxo-4-(1-oxypyridine-3-sulfonyl)-hexahydro-pyrrolo-[3,2-b]-pyrrole-1-carbonyl]-butyl}-amide d'acide quinoxaline-2-carboxylique ;
{1-[6-oxo-4-(1-oxypyridine-3-sulfonyl)-hexahydro-pyrrolo-[3,2-b]-pyrrole-1-carbonyl]-butyl}-amide d'acide benzo-[1,3]dioxole-5-carboxylique ;
{1-[6-oxo-4-(1-oxypyridine-3-sulfonyl)-hexahydro-pyrrolo-[3,2-b]-pyrrole-1-carbonyl]-butyl}-amide d'acide quinoléine-6-carboxylique ;
{1-[6-oxo-4-(1-oxypyridine-3-sulfonyl)-hexahydro-pyrrolo-[3,2-b]-pyrrole-1-carbonyl]-butyl}-amide d'acide furanne-2-carboxylique ;
{1-[6-oxo-4-(1-oxypyridine-3-sulfonyl)-hexahydro-pyrrolo-[3,2-b]-pyrrole-1-carbonyl]-butyl}-amide d'acide thiophène-2-carboxylique ;
N-{1-[6-oxo-4-(1-oxypyridine-3-sulfonyl)-hexahydro-pyrrolo-[3,2-b]-pyrrole-1-carbonyl]-butyl}-4-trifluorométhyl-benzamide ;
{1-[6-oxo-4-(1-oxypyridine-3-sulfonyl)-hexahydro-pyrrolo-[3,2-b]-pyrrole-1-carbonyl]-butyl}-amide d'acide furanne-3-carboxylique ;
{1-[6-oxo-4-(1-oxypyridine-3-sulfonyl)-hexahydro-pyrrolo-[3,2-b]-pyrrole-1-carbonyl]-butyl}-amide d'acide thiophène-3-carboxylique ;

N-{1-[6-oxo-4-(1-oxypyridine-3-sulfonyl)-hexahydro-pyrrolo-[3,2-b]-pyrrole-1-carbonyl]-butyl}-4-phénoxybenzamide ;
N-{1-[6-oxo-4-(1-oxypyridine-3-sulfonyl)-hexahydro-pyrrolo-[3,2-b]-pyrrole-1-carbonyl]-butyl}-4-(4-méthyl-pipérazine-1-yl)-benzamide ;
4-méthyl-N-{1-[6-oxo-4-(1-oxypyridine-3-sulfonyl)-hexa-hydro-pyrrolo[3,2-b]pyrrole-1-carbonyl]-butyl}-benzamide ;
4-méthoxy-N-{1-[6-oxo-4-(1-oxypyridine-3-sulfonyl)-hexahydro-pyrrolo-[3,2-b]-pyrrole-1-carbonyl]-butyl}-benzamide ;
4-isopropyl-N-{1-[6-oxo-4-(1-oxypyridine-3-sulfonyl)-hexa-hydro-pyrrolo-[3,2-b]-pyrrole-1-carbonyl]-butyl}-benzamide ;
N-{1-[6-oxo-4-(1-oxypyridine-3-sulfonyl)-hexahydro-pyrrolo-[3,2-b]-pyrrole-1-carbonyl]-butyl}-4-vinyl-benzamide ;
4-imidazole-1-yl-N-{1-[6-oxo-4-(1-oxypyridine-3-sulfonyl)-hexahydro-pyrrolo-[3,2-b]-pyrrole-1-carbonyl]-butyl}-benzamide ;
N-{1-[6-oxo-4-(1-oxypyridine-3-sulfonyl)-hexahydro-pyrrolo-[3,2-b]-pyrrole-1-carbonyl]-butyl}-4-thiophène-2-yl-benzamide ;
N-{1-[6-oxo-4-(1-oxypyridine-3-sulfonyl)-hexahydro-pyrrolo-[3,2-b]-pyrrole-1-carbonyl]-butyl}-4-oxazole-5-yl-benz amide ;
{1-[6-oxo-4-(1-oxypyridine-3-sulfonyl)-hexahydro-pyrrolo-[3,2-b]-pyrrole-1-carbonyl]-butyl}-amide d'acide 5-phényl-thiophène-2-carboxylique ;
N-{1-[6-oxo-4-(1-oxypyridine-3-sulfonyl)-hexahydro-pyrrolo-[3,2-b]-pyrrole-1-carbonyl]-butyl}-4-trifluorométhoxy-benzamide ;
{1-[6-oxo-4-(1-oxypyridine-3-sulfonyl)-hexahydro-pyrrolo-[3,2-b]-pyrrole-1-carbonyl]-butyl}-amide d'acide 5-pyridine-2-yl-thiophène-2-carboxylique ;
4-difluorométhoxy-N-{1-[6-oxo-4-(1-oxypyridine-3-sulfonyl)-hexahydro-pyrrolo-[3,2-b]-pyrrole-1-carbonyl]-butyl}-benzamide ;
N-{1-[6-oxo-4-(1-oxypyridine-3-sulfonyl)-hexahydro-pyrrolo-[3,2-b]-pyrrole-1-carbonyl]-butyl}-4-morpholine-4-yl-benzamide ;
{3-méthyl-1-[6-oxo-4-(pyridine-2-carbonyl)-hexahydro-pyrrolo[3,2-b]pyrrole-1-carbonyl]-butyl}-amide d'acide naphtalène-2-carboxylique ;
{3-méthyl-1-[6-oxo-4-(pyridine-2-carbonyl)-hexahydro-pyrrolo[3,2-b]pyrrole-1-carbonyl]-butyl}-amide d'acide quinoléine-2-carboxylique ;
{3-méthyl-1-[6-oxo-4-(pyridine-2-carbonyl)-hexahydro-pyrrolo[3,2-b]pyrrole-1-carbonyl]-butyl}-amide d'acide benzo[b]thiophène-2-carboxylique ;
{3-méthyl-1-[6-oxo-4-(pyridine-2-carbonyl)-hexahydro-pyrrolo[3,2-b]pyrrole-1-carbonyl]-butyl}-amide d'acide benzofuranne-2-carboxylique ;
{3-méthyl-1-[6-oxo-4-(pyridine-2-carbonyl)-hexahydro-pyrrolo[3,2-b]pyrrole-1-carbonyl]-butyl}-amide d'acide biphényl-4-carboxylique ;
4-tertiobutyl-N-{3-méthyl-1-[6-oxo-4-(pyridine-2-carbonyl)-hexahydro-pyrrolo[3,2-b]pyrrole-1-carbonyl]-butyl}-benzamide ;
4-diméthylamino-N-{3-méthyl-1-[6-oxo-4-(pyridine-2-carbonyl)-hexahydro-pyrrolo[3,2-b]pyrrole-1-carbonyl]-butyl}-benzamide ;
{3-méthyl-1-[6-oxo-4-(pyridine-2-carbonyl)-hexahydro-pyrrolo[3,2-b]pyrrole-1-carbonyl]-butyl}-amide d'acide 7-méthoxy-benzofuranne-2-carboxylique ;
ester benzylique d'acide {3-méthyl-1-[6-oxo-4-(pyridine-2-carbonyl)-hexahydro-pyrrolo[3,2-b]pyrrole-1-carbonyl]-butyl}-carbamique ;
{3-méthyl-1-[6-oxo-4-(pyridine-2-carbonyl)-hexahydro-pyrrolo[3,2-b]pyrrole-1-carbonyl]-butyl}-amide d'acide 5-méthoxybenzofuranne-2-carboxylique ;
{3-méthyl-1-[6-oxo-4-(pyridine-2-carbonyl)-hexahydro-pyrrolo[3,2-b]pyrrole-1-carbonyl]-butyl}-amide d'acide thiéno[3,2-b]thiophène-2-carboxylique ;
{3-méthyl-1-[6-oxo-4-(pyridine-2-carbonyl)-hexahydro-pyrrolo[3,2-b]pyrrole-1-carbonyl]-butyl}-amide d'acide 3-méthyl-benzofuranne-2-carboxylique ;
{3-méthyl-1-[6-oxo-4-(pyridine-2-carbonyl)-hexahydro-pyrrolo[3,2-b]pyrrole-1-carbonyl]-butyl}-amide d'acide quinoxaline-2-carboxylique ;
{3-méthyl-1-[6-oxo-4-(pyridine-2-carbonyl)-hexahydro-pyrrolo[3,2-b]pyrrole-1-carbonyl]-butyl}-amide d'acide benzo[1,3]dioxole-5-carboxylique ;
{3-méthyl-1-[6-oxo-4-(pyridine-2-carbonyl)-hexahydro-pyrrolo[3,2-b]pyrrole-1-carbonyl]-butyl}-amide d'acide quinoléine-6-carboxylique ;
{3-méthyl-1-[6-oxo-4-(pyridine-2-carbonyl)-hexahydro-pyrrolo[3,2-b]pyrrole-1-carbonyl]-butyl}-amide d'acide furanne-2-carboxylique ;

{3-méthyl-1-[6-oxo-4-(pyridine-2-carbonyl)-hexahydro-pyrrolo[3,2-b]pyrrole-1-carbonyl]-butyl}-amide d'acide thiophène-2-carboxylique ;
N-{3-méthyl-1-[6-oxo-4-(pyridine-2-carbonyl)-hexahydro-pyrrolo[3,2-b]pyrrole-1-carbonyl]-butyl}-4-trifluorométhyl-benzamide ;
{3-méthyl-1-[6-oxo-4-(pyridine-2-carbonyl)-hexahydro-pyrrolo[3,2-b]pyrrole-1-carbonyl]-butyl}-amide d'acide furanne-3-carboxylique ;
{3-méthyl-1-[6-oxo-4-(pyridine-2-carbonyl)-hexahydro-pyrrolo[3,2-b]pyrrole-1-carbonyl]-butyl}-amide d'acide thiophène-3-carboxylique ;
N-{3-méthyl-1-[6-oxo-4-(pyridine-2-carbonyl)-hexahydro-pyrrolo[3,2-b]pyrrole-1-carbonyl]-butyl}-4-phénoxy-benzamide ;
N-{3-méthyl-1-[6-oxo-4-(pyridine-2-carbonyl)-hexahydro-pyrrolo[3,2-b]pyrrole-1-carbonyl]-butyl}-4-(4-méthyl-pipérazine-1-yl)-benzamide ;
4-méthyl-N-{3-méthyl-1-[6-oxo-4-(pyridine-2-carbonyl)-hexahydro-pyrrolo[3,2-b]pyrrole-1-carbonyl]-butyl}-benzamide ;
4-méthoxy-N-{3-méthyl-1-[6-oxo-4-(pyridine-2-carbonyl)-hexahydro-pyrrolo[3,2-b]pyrrole-1-carbonyl]-butyl}-benzamide ;
4-isopropyl-N-{3-méthyl-1-[6-oxo-4-(pyridine-2-carbonyl)-hexahydro-pyrrolo[3,2-b]pyrrole-1-carbonyl]-butyl}-benzamide ;
N-{3-méthyl-1-[6-oxo-4-(pyridine-2-carbonyl)-hexahydro-pyrrolo[3,2-b]pyrrole-1-carbonyl]-butyl}-4-vinyl-benzamide ;
4-imidazole-1-yl-N-{3-méthyl-1-[6-oxo-4-(pyridine-2-carbonyl)-hexahydro-pyrrolo[3,2-b]pyrrole-1-carbonyl]-butyl}-benzamide ;
N-{3-méthyl-1-[6-oxo-4-(pyridine-2-carbonyl)-hexahydro-pyrrolo[3,2-b]pyrrole-1-carbonyl]-butyl}-4-thiophène-2-yl-benzamide ;
N-{3-méthyl-1-[6-oxo-4-(pyridine-2-carbonyl)-hexahydro-pyrrolo[3,2-b]pyrrole-1-carbonyl]-butyl}-4-oxazole-5-yl-benzamide ;
{3-méthyl-1-[6-oxo-4-(pyridine-2-carbonyl)-hexahydro-pyrrolo[3,2-b]pyrrole-1-carbonyl]-butyl}-amide d'acide 5-phényl-thiophène-2-carboxylique ;
N-{3-méthyl-1-[6-oxo-4-(pyridine-2-carbonyl)-hexahydro-pyrrolo[3,2-b]pyrrole-1-carbonyl]-butyl}-4-trifluorométhoxy-benzamide ;
{3-méthyl-1-[6-oxo-4-(pyridine-2-carbonyl)-hexahydro-pyrrolo[3,2-b]pyrrole-1-carbonyl]-butyl}-amide d'acide 5-pyridine-2-yl-thiophène-2-carboxylique ;
4-difluorométhoxy-N-{3-méthyl-1-[6-oxo-4-(pyridine-2-carbonyl)-hexahydro-pyrrolo[3,2-b]pyrrole-1-carbonyl]-butyl}-benzamide :
N-{3-méthyl-1-[6-oxo-4-(pyridine-2-carbonyl)-hexahydro-pyrrolo[3,2-b]pyrrole-1-carbonyl]-butyl}-4-morpholine-4-yl-benzamide ;
{1-[6-oxo-4-(pyridine-2-carbonyl)-hexahydro-pyrrolo[3,2-b]-pyrrole-1-carbonyl]-butyl}-amide d'acide naphtalène-2-carboxylique ;
{1-[6-oxo-4-(pyridine-2-carbonyl)-hexahydro-pyrrolo[3,2-b]-pyrrole-1-carbonyl]-butyl}-amide d'acide quinoléine-2-carboxylique ;
{1-[6-oxo-4-(pyridine-2-carbonyl)-hexahydro-pyrrolo[3,2-b]pyrrole-1-carbonyl]-butyl}-amide d'acide benzo[b]thiophène-2-carboxylique ;
{1-[6-oxo-4-(pyridine-2-carbonyl)-hexahydro-pyrrolo[3,2-b]pyrrole-1-carbonyl]-butyl}-amide d'acide benzofuranne-2-carboxylique ;
{1-[6-oxo-4-(pyridine-2-carbonyl)-hexahydro-pyrrolo[3,2-b]-pyrrole-1-carbonyl]-butyl}-amide d'acide biphényl-4-carboxylique ;
4-tertiobutyl-N-{1-[6-oxo-4-(pyridine-2-carbonyl)-hexa-hydro-pyrrolo[3,2-b]-pyrrole-1-carbonyl]-butyl}-benzamide ;
4-diméthylamino-N-{1-[6-oxo-4-(pyridine-2-carbonyl)-hexahydro-pyrrolo[3,2-b]-pyrrole-1-carbonyl]-butyl}-benzamide ;
{1-[6-oxo-4-(pyridine-2-carbonyl)-hexahydro-pyrrolo[3,2-b]-pyrrole-1-carbonyl]-butyl}-amide d'acide 7-méthoxy-benzofuranne-2-carboxylique ;
ester benzylique d'acide {1-[6-oxo-4-(pyridine-2-carbonyl)-hexahydro-pyrrolo[3,2-b]-pyrrole-1-carbonyl]-butyl}-carbamique ;
{1-[6-oxo-4-(pyridine-2-carbonyl)-hexahydro-pyrrolo[3,2-b]-pyrrole-1-carbonyl]-butyl}-amide d'acide 5-méthoxy-benzo-furanne-2-carboxylique ;
{1-[6-oxo-4-(pyridine-2-carbonyl)-hexahydro-pyrrolo[3,2-b]-pyrrole-1-carbonyl]-butyl}-amide d'acide thiéno[3,2-b]-thiophène-2-carboxylique ;
{1-[6-oxo-4-(pyridine-2-carbonyl)-hexahydro-pyrrolo[3,2-b]-pyrrole-1-carbonyl]-butyl}-amide d'acide 3-méthyl-benzo-furanne-2-carboxylique ;
{1-[6-oxo-4-(pyridine-2-carbonyl)-hexahydro-pyrrolo[3,2-b]-pyrrole-1-carbonyl]-butyl}-amide d'acide quinoxaline-2-carboxylique ;
{1-[6-oxo-4-(pyridine-2-carbonyl)-hexahydro-pyrrolo[3,2-b]-pyrrole-1-carbonyl]-butyl}-amide d'acide benzo[1,3]-dioxole-5-carboxylique ;
{1-[6-oxo-4-(pyridine-2-carbonyl)-hexahydro-pyrrolo[3,2-b]-pyrrole-1-carbonyl]-butyl}-amide d'acide quinoléine-6-carboxylique ;
{1-[6-oxo-4-(pyridine-2-carbonyl)-hexahydro-pyrrolo[3,2-b]-pyrrole-1-carbonyl]-butyl}-amide d'acide furanne-2-carboxylique ;
{1-[6-oxo-4-(pyridine-2-carbonyl)-hexahydro-pyrrolo[3,2-b]-pyrrole-1-carbonyl]-butyl}-amide d'acide thiophène-2-carboxylique ;
N-{1-[6-oxo-4-(pyridine-2-carbonyl)-hexahydro-pyrrolo-[3,2-b]-pyrrole-1-carbonyl]-butyl}-4-trifluorométhyl-benzamide ;
{1-[6-oxo-4-(pyridine-2-carbonyl)-hexahydro-pyrrolo[3,2-b]-pyrrole-1-carbonyl]-butyl}-amide d'acide furanne-3-carboxylique ;
{1-[6-oxo-4-(pyridine-2-carbonyl)-hexahydro-pyrrolo[3,2-b]-pyrrole-1-carbonyl]-butyl}-amide d'acide thiophène-3-carboxylique ;
N-{1-[6-oxo-4-(pyridine-2-carbonyl)-hexahydro-pyrrolo-[3,2-b]pyrrole-1-carbonyl]-butyl}-4-phénoxy-benzamide ;
N-{1-[6-oxo-4-(pyridine-2-carbonyl)-hexahydro-pyrrolo[3,2-b]-pyrrole-1-carbonyl]-butyl}-4-(4-méthyl-pipérazine-1-yl)-benzamide ;
4-méthyl-N-{1-[6-oxo-4-(pyridine-2-carbonyl)-hexahydro-pyrrolo[3,2-b]-pyrrole-1-carbonyl]-butyl}-benzamide ;
4-méthoxy-N-{1-[6-oxo-4-(pyridine-2-carbonyl)-hexahydro-pyrrolo[3,2-b]-pyrrole-1-carbonyl]-butyl}-benzamide ;
4-isopropyl-N-{1-[6-oxo-4-(pyridine-2-carbonyl)-hexahydro-pyrrolo[3,2-b]-pyrrole-1-carbonyl]-butyl}-benzamide ;
N-{1-[6-oxo-4-(pyridine-2-carbonyl)-hexahydro-pyrrolo-[3,2-b]pyrrole-1-carbonyl]-butyl}-benzamide ;
4-imidazole-1-yl-N-{1-[6-oxo-4-(pyridine-2-carbonyl)-hexahydro-pyrrolo[3,2-b]-pyrrole-1-carbonyl]-butyl}-benzamide ;
N-{1-[6-oxo-4-(pyridine-2-carbonyl)-hexahydro-pyrrolo[3,2-b]-pyrrole-1-carbonyl]-butyl}-4-thiophène-2-yl-benzamide ;
N-{1-[6-oxo-4-(pyridine-2-carbonyl)-hexahydro-pyrrolo[3,2-b]-pyrrole-1-carbonyl]-butyl}-4-oxazole-5-yl-benzamide ;
{1-[6-oxo-4-(pyridine-2-carbonyl)-hexahydro-pyrrolo[3,2-b]-pyrrole-1-carbonyl]-butyl}-amide d'acide 5-phényl-thiophène-2-carboxylique ;
N-{1-[6-oxo-4-(pyridine-2-carbonyl)-hexahydro-pyrrolo[3,2-b]-pyrrole-1-carbonyl]-butyl}-4-trifluorométhoxy-benzamide ;
{1-[6-oxo-4-(pyridine-2-carbonyl)-hexahydro-pyrrolo[3,2-b]-pyrrole-1-carbonyl]-butyl}-amide d'acide 5-pyridine-2-yl-thiophène-2-carboxylique ;
4-difluorométhoxy-N-{1-[6-oxo-4-(pyridine-2-carbonyl)-hexahydro-pyrrolo[3,2-b]-pyrrole-1-carbonyl]-butyl}-benzamide ;
N-{1-[6-oxo-4-(pyridine-2-carbonyl)-hexahydro-pyrrolo[3,2-b]-pyrrole-1-carbonyl]-butyl}-4-morpholine-4-yl-benzamide ;
{3-méthyl-1-[6-oxo-4-(2-pyridine-3-ylacétyl)-hexahydro-pyrrolo[3,2-b]-pyrrole-1-carbonyl]-butyl}-amide d'acide naphtalène-2-carboxylique ;
{3-méthyl-1-[6-oxo-4-(2-pyridine-3-ylacétyl)-hexahydro-pyrrolo[3,2-b]-pyrrole-1-carbonyl]-butyl}-amide d'acide quinoléine-2-carboxylique ;
{3-méthyl-1-[6-oxo-4-(2-pyridine-3-ylacétyl)-hexahydro-pyrrolo[3,2-b]-pyrrole-1-carbonyl]-butyl}-amide d'acide benzo[b]thiophène-2-carboxylique ;
{3-méthyl-1-[6-oxo-4-(2-pyridine-3-ylacétyl)-hexahydro-pyrrolo[3,2-b]-pyrrole-1-carbonyl]-butyl}-amide d'acide benzofuranne-2-carboxylique ;
{3-méthyl-1-[6-oxo-4-(2-pyridine-3-ylacétyl)-hexahydro-pyrrolo[3,2-b]-pyrrole-1-carbonyl]-butyl}-amide d'acide biphényl-4-carboxylique ;
4-tertiobutyl-N-{3-méthyl-1-[6-oxo-4-(2-pyridine-3-ylacétyl)-hexahydro-pyrrolo[3,2-b]-pyrrole-1-carbonyl]-butyl}-benzamide ;
4-diméthylamino-N-{3-méthyl-1-[6-oxo-4-(2-pyridine-3-ylacétyl)-hexahydro-pyrrolo[3,2-b]-pyrrole-1-carbonyl]-butyl }-benzamide ;
{3-méthyl-1-[6-oxo-4-(2-pyridine-3-ylacétyl)-hexahydro-pyrrolo[3,2-b]-pyrrole-1-carbonyl]-butyl}-amide d'acide 7-méthoxybenzofuranne-2-carboxylique ;
ester benzylique d'acide {3-méthyl-1-[6-oxo-4-(2-pyridine-3-ylacétyl)-hexahydro-pyrrolo[3,2-b]-pyrrole-1-carbonyl]-butyl}-carbamique ;

{3-méthyl-1-[6-oxo-4-(2-pyridine-3-ylacétyl)-hexahydro-pyrrolo[3,2-b]-pyrrole-1-carbonyl]-butyl}-amide d'acide 5-méthoxybenzofuranne-2-carboxylique ;

{3-méthyl-1-[6-oxo-4-(2-pyridine-3-ylacétyl)-hexahydro-pyrrolo[3,2-b]-pyrrole-1-carbonyl]-butyl}-amide d'acide thiéno[3,2-b]thiophène-2-carboxylique ;

{3-méthyl-1-[6-oxo-4-(2-pyridine-3-ylacétyl)-hexahydro-pyrrolo[3,2-b]-pyrrole-1-carbonyl]-butyl}-amide d'acide 3-méthyl-benzofuranne-2-carboxylique ;

{3-méthyl-1-[6-oxo-4-(2-pyridine-3-ylacétyl)-hexahydro-pyrrolo[3,2-b]-pyrrole-1-carbonyl]-butyl}-amide d'acide quinoxaline-2-carboxylique ;

{3-méthyl-1-[6-oxo-4-(2-pyridine-3-ylacétyl)-hexahydro-pyrrolo[3,2-b]-pyrrole-1-carbonyl]-butyl}-amide d'acide benzo[1,3]dioxole-5-carboxylique ;

{3-méthyl-1-[6-oxo-4-(2-pyridine-3-ylacétyl)-hexahydro-pyrrolo[3,2-b]-pyrrole-1-carbonyl]-butyl}-amide d'acide quinoléine-6-carboxylique ;

{3-méthyl-1-[6-oxo-4-(2-pyridine-3-ylacétyl)-hexahydro-pyrrolo[3,2-b]-pyrrole-1-carbonyl]-butyl}-amide d'acide furanne-2-carboxylique ;

{3-méthyl-1-[6-oxo-4-(2-pyridine-3-ylacétyl)-hexahydro-pyrrolo[3,2-b]-pyrrole-1-carbonyl]-butyl}-amide d'acide thiophène-2-carboxylique ;

N-{3-méthyl-1-[6-oxo-4-(2-pyridine-3-ylacétyl)-hexahydro-pyrrolo[3,2-b]-pyrrole-1-carbonyl]-butyl}-4-trifluorométhyl-benzamide ;

{3-méthyl-1-[6-oxo-4-(2-pyridine-3-ylacétyl)-hexahydro-pyrrolo[3,2-b]-pyrrole-1-carbonyl]-butyl}-amide d'acide furanne-3-carboxylique ;

{3-méthyl-1-[6-oxo-4-(2-pyridine-3-ylacétyl)-hexahydro-pyrrolo[3,2-b]-pyrrole-1-carbonyl]-butyl}-amide d'acide thiophène-3-carboxylique ;

N-{3-méthyl-1-[6-oxo-4-(2-pyridine-3-ylacétyl)-hexahydro-pyrrolo[3,2-b]-pyrrole-1-carbonyl]-butyl}-4-phénoxy-benzamide ;

N-{3-méthyl-1-[6-oxo-4-(2-pyridine-3-ylacétyl)-hexahydro-pyrrolo[3,2-b]-pyrrole-1-carbonyl]-butyl}-4-(4-méthyl-pipérazine-1-y1)-benzamide ;

4-méthyl-N-{3-méthyl-1-[6-oxo-4-(2-pyridine-3-ylacétyl)-hexahydro-pyrrolo[3,2-b]-pyrrole-1-carbonyl]-butyl}-benzamide ;

4-méthoxy-N-{3-méthyl-1-[6-oxo-4-(2-pyridine-3-ylacétyl)-hexahydro-pyrrolo[3,2-b]-pyrrole-1-carbonyl]-butyl}-benzamide ;

4-isopropyl-N-{3-méthyl-1-[6-oxo-4-(2-pyridine-3-ylacétyl)-hexahydro-pyrrolo[3,2-b]-pyrrole-1-carbonyl]-butyl}-benzamide ;

N-{3-méthyl-1-[6-oxo-4-(2-pyridine-3-ylacétyl)-hexahydro-pyrrolo[3,2-b]-pyrrole-1-carbonyl]-butyl}-4-vinyl-benzamide ;

N-{3-méthyl-1-[6-oxo-4-(2-pyridine-3-ylacétyl)-hexahydro-pyrrolo[3,2-b]-pyrrole-1-carbonyl]-butyl}-benzamide ;

N-{3-méthyl-1-[6-oxo-4-(2-pyridine-3-ylacétyl)-hexahydro-pyrrolo[3,2-b]-pyrrole-1-carbonyl]-butyl}-4-thiophène-2-yl-benzamide ;

N-{3-méthyl-1-[6-oxo-4-(2-pyridine-3-ylacétyl)-hexahydro-pyrrolo[3,2-b]-pyrrole-1-carbonyl]-butyl}-4-oxazole-5-yl-benzamide ;

{3-méthyl-1-[6-oxo-4-(2-pyridine-3-ylacétyl)-hexahydro-pyrrolo[3,2-b]-pyrrole-1-carbonyl]-butyl}-amide d'acide 5-phényl-thiophène-2-carboxylique ;

N-{3-méthyl-1-[6-oxo-4-(2-pyridine-3-ylacétyl)-hexahydro-pyrrolo[3,2-b]-pyrrole-1-carbonyl]-butyl}-4-trifluorométhoxy-benzamide ;

{3-méthyl-1-[6-oxo-4-(2-pyridine-3-ylacétyl)-hexahydro-pyrrolo[3,2-b]-pyrrole-1-carbonyl]-butyl}-amide d'acide 5-pyridine-2-yl-thiophène-2-carboxylique ;

4-difluorométhoxy-N-{3-méthyl-1-[6-oxo-4-(2-pyridine-3-ylacétyl)-hexahydro-pyrrolo[3,2-b]-pyrrole-1-carbonyl]-butyl}-benzamide ;

N-{3-méthyl-1-[6-oxo-4-(2-pyridine-3-ylacétyl)-hexahydro-pyrrolo[3,2-b]-pyrrole-1-carbonyl]-butyl}-4-morpholine-4-yl-benzamide ;

{1-[6-oxo-4-(2-pyridine-3-ylacétyl)-hexahydro-pyrrolo-[3,2-b]-pyrrole-1-carbonyl]-butyl}-amide d'acide naphtalène-2-carboxylique ;

{1-[6-oxo-4-(2-pyridine-3-ylacétyl)-hexahydro-pyrrolo-[3,2-b]-pyrrole-1-carbonyl]-butyl}-amide d'acide quinoléine-2-carboxylique ;

{1-[6-oxo-4-(2-pyridine-3-ylacétyl)-hexahydro-pyrrolo-[3,2-b]-pyrrole-1-carbonyl]-butyl}-amide d'acide benzo[b]-thiophène-2-carboxylique ;

{1-[6-oxo-4-(2-pyridine-3-ylacétyl)-hexahydro-pyrrolo-[3,2-b]-pyrrole-1-carbonyl]-butyl}-amide d'acide benzofuranne-2-carboxylique ;

{1-[6-oxo-4-(2-pyridine-3-ylacétyl)-hexahydro-pyrrolo-[3,2-b]-pyrrole-1-carbonyl]-butyl}-amide d'acide biphényl-4-carboxylique ;
4-tertiobutyl-N-{1-[6-oxo-4-(2-pyridine-3-ylacétyl)-hexahydro-pyrrolo-[3,2-b]-pyrrole-1-carbonyl]-butyl}-benzamide ;
4-diméthylamino-N-{1-[6-oxo-4-(2-pyridine-3-ylacétyl)-hexahydro-pyrrolo-[3,2-b]-pyrrole-1-carbonyl]-butyl}-benzamide ;
{1-[6-oxo-4-(2-pyridine-3-ylacétyl)-hexahydro-pyrrolo-[3,2-b]-pyrrole-1-carbonyl]-butyl}-amide d'acide 7-méthoxy-benzofuranne-2-carboxylique ;
ester benzylique d'acide {1-[6-oxo-4-(2-pyridine-3-ylacétyl)-hexahydro-pyrrolo-[3,2-b]-pyrrole-1-carbonyl]-butyl}-carbamique ;
{1-[6-oxo-4-(2-pyridine-3-ylacétyl)-hexahydro-pyrrolo-[3,2-b]-pyrrole-1-carbonyl]-butyl}-amide d'acide 5-méthoxy-benzofuranne-2-carboxylique ;
{1-[6-oxo-4-(2-pyridine-3-ylacétyl)-hexahydro-pyrrolo-[3,2-b]-pyrrole-1-carbonyl]-butyl}-amide d'acide thiéno-[3,2-b]thiophène-2-carboxylique ;
{1-[6-oxo-4-(2-pyridine-3-ylacétyl)-hexahydro-pyrrolo-[3,2-b]-pyrrole-1-carbonyl]-butyl}-amide d'acide 3-méthyl-benzofuranne-2-carboxylique ;
{1-[6-oxo-4-(2-pyridine-3-ylacétyl)-hexahydro-pyrrolo-[3,2-b]-pyrrole-1-carbonyl]-butyl}-amide d'acide quinoxaline-2- carboxylique ;
{1-[6-oxo-4-(2-pyridine-3-ylacétyl)-hexahydro-pyrrolo-[3,2-b]-pyrrole-1-carbonyl]-butyl}-amide d'acide benzo-[1,3]dioxole-5-carboxylique ;
{1-[6-oxo-4-(2-pyridine-3-ylacétyl)-hexàhydro-pyrrolo-[3,2-b]-pyrrole-1-carbonyl]-butyl}-amide d'acide quinoléine-6-carboxylique ;
{1-[6-oxo-4-(2-pyridine-3-ylacétyl)-hexahydro-pyrrolo-[3,2-b]-pyrrole-1-carbonyl]-butyl}-amide d'acide furanne-2-carboxylique ;
{1-[6-oxo-4-(2-pyridine-3-ylacétyl)-hexahydro-pyrrolo-[3,2-b]-pyrrole-1-carbonyl]-butyl}-amide d'acide thiophène-2-carboxylique ;
N-{1-[6-oxo-4-(2-pyridine-3-ylacétyl)-hexahydro-pyrrolo-[3,2-b]-pyrrole-1-carbonyl]-butyl}-4-trifluorométhyl-benzamide ;
{1-[6-oxo-4-(2-pyridine-3-ylacétyl)-hexahydro-pyrrolo-[3,2-b]-pyrrole-1-carbonyl]-butyl}-amide d'acide furanne-3-carboxylique ;
{1-[6-oxo-4-(2-pyridine-3-ylacétyl)-hexahydro-pyrrolo-[3,2-b]-pyrrole-1-carbonyl]-butyl}-amide d'acide thiophène-3-carboxylique ;
N-{1-[6-oxo-4-(2-pyridine-3-ylacétyl)-hexahydro-pyrrolo-[3,2-b]-pyrrole-1-carbonyl]-butyl}-4-phénoxy-benzamide ;
N-{1-[6-oxo-4-(2-pyridine-3-ylacétyl)-hexahydro-pyrrolo-[3,2-b]-pyrrole-1-carbonyl]-butyl}-4-(4-méthyl-pipérazine-1-yl)-benzamide ;
4-méthyl-N-{1-[6-oxo-4-(2-pyridine-3-ylacétyl)-hexahydro-pyrrolo-[3,2-b]-pyrrole-1-carbonyl]-butyl}-benzamide ;
4-méthoxy-N-{1-[6-oxo-4-(2-pyridine-3-ylacétyl)-hexahydro-pyrrolo-[3,2-b]-pyrrole-1-carbonyl]-butyl}-benzamide ;
4-isopropyl-N-{1-[6-oxo-4-(2-pyridine-3-ylacétyl)-hexahydro-pyrrolo-[3,2-b]-pyrrole-1-carbonyl]-butyl}-benzamide ;
N-{1-[6-oxo-4-(2-pyridine-3-ylacétyl)-hexahydro-pyrrolo-[3,2-b]-pyrrole-1-carbonyl]-butyl}-4-vinylbenzamide ;
4-imidazole-1-yl-N-{1-[6-oxo-4-(2-pyridine-3-ylacétyl)-hexahydro-pyrrolo-[3,2-b]-pyrrole-1-carbonyl]-butyl}-benzamide ;
N-{1-[6-oxo-4-(2-pyridine-3-ylacétyl)-hexahydro-pyrrolo-[3,2-b]-pyrrole-1-carbonyl]-butyl}-4-thiophène-2-yl-benzamide ;
N-{1-[6-oxo-4-(2-pyridine-3-ylacétyl)-hexahydro-pyrrolo-[3,2-b]-pyrrole-1-carbonyl]-butyl}-4-oxazole-5-yl-benzamide ;
{1-[6-oxo-4-(2-pyridine-3-ylacétyl)-hexahydro-pyrrolo-[3,2-b]-pyrrole-1-carbonyl]-butyl}-amide d'acide 5-phényl-thiophène-2-carboxylique ;
N-{1-[6-oxo-4-(2-pyridine-3-ylacétyl)-hexahydro-pyrrolo-[3,2-b]-pyrrole-1-carbonyl]-butyl}-4-trifluorométhoxy-benzamide ;
{1-[6-oxo-4-(2-pyridine-3-ylacétyl)-hexahydro-pyrrolo-[3,2-b]-pyrrole-1-carbonyl]-butyl}-amide d'acide 5-pyridine-2-yl-thiophène-2-carboxylique ;
4-difluorométhoxy-N-{1-[6-oxo-4-(2-pyridine-3-ylacétyl)-hexahydro-pyrrolo-[3,2-b]-pyrrole-1-carbonyl]-butyl}-benzamide ;
N-{1-[6-oxo-4-(2-pyridine-3-ylacétyl)-hexahydro-pyrrolo-[3,2-b]-pyrrole-1-carbonyl]-butyl}-4-morpholine-4-yl-benzamide ;
{3-méthyl-1-[6-oxo-4-(pyridine-2-ylméthanesulfonyl)-hexa-hydropyrrolo[3,2-b]pyrrole-1-carbonyl]-butyl}-amide d'acide naphtalène-2-carboxylique ;
{3-méthyl-1-[6-oxo-4-(pyridine-2-ylméthanesulfonyl)-hexa-hydropyrrolo[3,2-b]pyrrole-1-carbonyl]-butyl}-amide d'acide quinoléine-2-carboxylique ;

{3-méthyl-1-[6-oxo-4-(pyridine-2-ylméthanesulfonyl)-hexa-hydropyrrolo[3,2-b]pyrrole-1-carbonyl]-butyl}-amide d'acide benzo[b]thiophène-2-carboxylique ;
{3-méthyl-1-[6-oxo-4-(pyridine-2-ylméthanesulfonyl)-hexa-hydropyrrolo[3,2-b]pyrrole-1-carbonyl]-butyl}-amide d'acide benzofuranne-2-carboxylique ;
{3-méthyl-1-[6-oxo-4-(pyridine-2-ylméthanesulfonyl)-hexa-hydropyrrolo[3,2-b]pyrrole-1-carbonyl]-butyl}-amide d'acide biphényl-4-carboxylique ;
4-tertiobutyl-N-{3-méthyl-1-[6-oxo-4-(pyridine-2-ylméthanesulfonyl)-hexa-hydropyrrolo[3,2-b]pyrrole-1-carbonyl]-butyl}-benzamide ;
4-diméthylamino-N-{3-méthyl-1-[6-oxo-4-(pyridine-2-ylméthanesulfonyl)-hexa-hydropyrrolo[3,2-b]pyrrole-1-carbonyl]-butyl}-benzamide ;
{3-méthyl-1-[6-oxo-4-(pyridine-2-ylméthanesulfonyl)-hexa-hydropyrrolo[3,2-b]pyrrole-1-carbonyl]-butyl}-amide d'acide 7-méthoxybenzofuranne-2-carboxylique ;
ester benzylique d'acide {3-méthyl-1-[6-oxo-4-(pyridine-2-ylméthanesulfonyl)-hexa-hydropyrrolo[3,2-b]pyrrole-1-carbonyl]-butyl}-carbamique ;
{3-méthyl-1-[6-oxo-4-(pyridine-2-ylméthanesulfonyl)-hexa-hydropyrrolo[3,2-b]pyrrole-1-carbonyl]-butyl}-amide d'acide 5-méthoxybenzofuranne-2-carboxylique ;
{3-méthyl-1-[6-oxo-4-(pyridine-2-ylméthanesulfonyl)-hexa-hydropyrrolo[3,2-b]pyrrole-1-carbonyl]-butyl}-amide d'acide thiéno[3,2-b]thiophène-2-carboxylique ;
{3-méthyl-1-[6-oxo-4-(pyridine-2-ylméthanesulfonyl)-hexa-hydropyrrolo[3,2-b]pyrrole-1-carbonyl]-butyl}-amide d'acide 3-méthyl-benzofuranne-2-carboxylique ;
{3-méthyl-1-[6-oxo-4-(pyridine-2-ylméthanesulfonyl)-hexa-hydropyrrolo[3,2-b]pyrrole-1-carbonyl]-butyl}-amide d'acide quinoxaline-2-carboxylique ;
{3-méthyl-1-[6-oxo-4-(pyridine-2-ylméthanesulfonyl)-hexa-hydropyrrolo[3,2-b]pyrrole-1-carbonyl]-butyl}-amide d'acide benzo[1,3]dioxole-5-carboxylique ;
{3-méthyl-1-[6-oxo-4-(pyridine-2-ylméthanesulfonyl)-hexa-hydropyrrolo[3,2-b]pyrrole-1-carbonyl]-butyl}-amide d'acide quinoléine-6-carboxylique ;
{3-méthyl-1-[6-oxo-4-(pyridine-2-ylméthanesulfonyl)-hexa-hydropyrrolo[3,2-b]pyrrole-1-carbonyl]-butyl}-amide d'acide furanne-2-carboxylique ;
{3-méthyl-1-[6-oxo-4-(pyridine-2-ylméthanesulfonyl)-hexa-hydropyrrolo[3,2-b]pyrrole-1-carbonyl]-butyl}-amide d'acide thiophène-2-carboxylique ;
N-{3-méthyl-1-[6-oxo-4-(pyridine-2-ylméthanesulfonyl)-hexa-hydropyrrolo[3,2-b]pyrrole-1-carbonyl]-butyl}-4-trifluorométhyl-benzamide ;
{3-méthyl-1-[6-oxo-4-(pyridine-2-ylméthanesulfonyl)-hexa-hydropyrrolo[3,2-b]pyrrole-1-carbonyl]-butyl}-amide d'acide furanne-3-carboxylique ;
{3-méthyl-1-[6-oxo-4-(pyridine-2-ylméthanesulfonyl)-hexa-hydropyrrolo[3,2-b]pyrrole-1-carbonyl]-butyl}-amide d'acide thiophène-3-carboxylique ;
N-{3-méthyl-1-[6-oxo-4-(pyridine-2-ylméthanesulfonyl)-hexa-hydropyrrolo[3,2-b]pyrrole-1-carbonyl]-butyl}-4-phénoxy-benzamide ;
N-{3-méthyl-1-[6-oxo-4-(pyridine-2-ylméthanesulfonyl)-hexa-hydropyrrolo[3,2-b]pyrrole-1-carbonyl]-butyl}-benzamide ;
4-méthyl-N-{3-méthyl-1-[6-oxo-4-(pyridine-2-ylméthane-sulfonyl)-hexa-hydropyrrolo[3,2-b]pyrrole-1-carbonyl]-butyl}-benzamide ;
4-méthoxy-N-{3-méthyl-1-[6-oxo-4-(pyridine-2-ylméthane-sulfonyl)-hexa-hydropyrrolo[3,2-b]pyrrole-1-carbonyl]-butyl}-benzamide ;
4-isopropyl-N-{3-méthyl-1-[6-oxo-4-(pyridine-2-ylméthane-sulfonyl)-hexa-hydropyrrolo[3,2-b]pyrrole-1-carbonyl]-butyl}-benzamide ;
N-{3-méthyl-1-[6-oxo-4-(pyridine-2-ylméthanesulfonyl)-hexa-hydropyrrolo[3,2-b]pyrrole-1-carbonyl]-butyl}-4-vinyl-benzamide ;
4-imidazole-1-yl-N-{3-méthyl-1-[6-oxo-4-(pyridine-2-ylméthanesulfonyl)-hexa-hydropyrrolo[3,2-b]pyrrole-1-carbonyl]-butyl}-benzamide ;
N-{3-méthyl-1-[6-oxo-4-(pyridine-2-ylméthanesulfonyl)-hexa-hydropyrrolo[3,2-b]pyrrole-1-carbonyl]-butyl}-4-thiophène-2-ylbenzamide ;
N-{3-méthyl-1-[6-oxo-4-(pyridine-2-ylméthanesulfonyl)-hexa-hydropyrrolo[3,2-b]pyrrole-1-carbonyl]-butyl}-4-oxazole-5-yl-benzamide ;
{3-méthyl-1-[6-oxo-4-(pyridine-2-ylméthanesulfonyl)-hexa-hydropyrrolo[3,2-b]pyrrole-1-carbonyl]-butyl}-amide d'acide 5-phényl-thiophène-2-carboxylique ;
N-{3-méthyl-1-[6-oxo-4-(pyridine-2-ylméthanesulfonyl)-hexa-hydropyrrolo[3,2-b]pyrrole-1-carbonyl]-butyl}-4-trifluorométhoxy-benzamide ;

{3-méthyl-1-[6-oxo-4-(pyridine-2-ylméthanesulfonyl)-hexa-hydropyrrolo[3,2-b]pyrrole-1-carbonyl]-butyl}-amide d'acide 5-pyridine-2-yl-thiophène-2-carboxylique ;
4-difluorométhoxy-N-{3-méthyl-1-[6-oxo-4-(pyridine-2-ylméthanesulfonyl)-hexa-hydropyrrolo[3,2-b]pyrrole-1-carbonyl]-butyl}-benzamide ;
N-{3-méthyl-1-[6-oxo-4-(pyridine-2-ylméthanesulfonyl)-hexa-hydropyrrolo[3,2-b]pyrrole-1-carbonyl]-butyl}-4-morpholine-4-yl-benzamide
{1-[6-oxo-4-(pyridine-2-ylméthanesulfonyl)-hexahydro-pyrrolo[3,2-b]pyrrole-1-carbonyl]-butyl}-amide d'acide naphtalène-2-carboxylique ;
{1-[6-oxo-4-(pyridine-2-ylméthanesulfonyl)-hexahydro-pyrrolo[3,2-b]pyrrole-1-carbonyl]-butyl}-amide d'acide quinoléine-2-carboxylique ;
{1-[6-oxo-4-(pyridine-2-ylméthanesulfonyl)-hexahydro-pyrrolo[3,2-b]pyrrole-1-carbonyl]-butyl}-amide d'acide benzo[b]thiophène-2-carboxylique ;
{1-[6-oxo-4-(pyridine-2-ylméthanesulfonyl)-hexahydro-pyrrolo[3,2-b]pyrrole-1-carbonyl]-butyl}-amide d'acide benzofuranne-2-carboxylique ;
{1-[6-oxo-4-(pyridine-2-ylméthanesulfonyl)-hexahydro-pyrrolo[3,2-b]pyrrole-1-carbonyl]-butyl}-amide d'acide biphényl-4-carboxylique ;
4-tertiobutyl-N-{1-[6-oxo-4-(pyridine-2-ylméthanesulfonyl)-hexahydro-pyrrolo[3,2-b]pyrrole-1-carbonyl]-butyl}-benzamide ;
4-diméthylamino-N-{1-[6-oxo-4-(pyridine-2-ylméthane-sulfonyl)-hexahydro-pyrrolo[3,2-b]pyrrole-1-carbonyl]-butyl}-benzamide ;
{1-[6-oxo-4-(pyridine-2-ylméthanesulfonyl)-hexahydro-pyrrolo[3,2-b]pyrrole-1-carbonyl]-butyl}-amide d'acide 7-méthoxybenzofuranne-2-carboxylique ;
ester benzylique d'acide {1-[6-oxo-4-(pyridine-2-ylméthanesulfonyl)-hexahydro-pyrrolo[3,2-b]pyrrole-1-carbonyl]-butyl}-carbamique ;
{1-[6-oxo-4-(pyridine-2-ylméthanesulfonyl)-hexahydro-pyrrolo[3,2-b]pyrrole-1-carbonyl]-butyl}-amide d'acide 5-méthoxybenzofuranne-2-carboxylique ;
{1-[6-oxo-4-(pyridine-2-ylméthanesulfonyl)-hexahydro-pyrrolo[3,2-b]pyrrole-1-carbonyl]-butyl}-amide d'acide thiéno[3,2-b]thiophène-2-carboxylique ;
{1-[6-oxo-4-(pyridine-2-ylméthanesulfonyl)-hexahydro-pyrrolo[3,2-b]pyrrole-1-carbonyl]-butyl}-amide d'acide 3-méthyl-benzofuranne-2-carboxylique ;
{1-[6-oxo-4-(pyridine-2-ylméthanesulfonyl)-hexahydro-pyrrolo[3,2-b]pyrrole-1-carbonyl]-butyl}-amide d'acide quinoxaline-2-carboxylique ;
{1-[6-oxo-4-(pyridine-2-ylméthanesulfonyl)-hexahydro-pyrrolo[3,2-b]pyrrole-1-carbonyl]-butyl}-amide d'acide ben zo[1,3]dioxole-5-carboxylique ;
{1-[6-oxo-4-(pyridine-2-ylméthanesulfonyl)-hexahydro-pyrrolo[3,2-b]pyrrole-1-carbonyl]-butyl}-amide d'acide quinoléine-6-carboxylique ;
{1-[6-oxo-4-(pyridine-2-ylméthanesulfonyl)-hexahydro-pyrrolo[3,2-b]pyrrole-1-carbonyl]butyl}-amide d'acide furanne-2-carboxylique ;
{1-[6-oxo-4-(pyridine-2-ylméthanesulfonyl)-hexahydro-pyrrolo[3,2-b]pyrrole-1-carbonyl]-butyl}-amide d'acide thiophène-2-carboxylique ;
N-{1-[6-oxo-4-(pyridine-2-ylméthanesulfonyl)-hexahydro-pyrrolo[3,2-b]pyrrole-1-carbonyl]-butyl}-4-trifluorométhyl-benzamide ;
{1-[6-oxo-4-(pyridine-2-ylméthanesulfonyl)-hexahydro-pyrrolo[3,2-b]pyrrole-1-carbonyl]-butyl}-amide d'acide furanne-3-carboxylique ;
{1-[6-oxo-4-(pyridine-2-ylméthanesulfonyl)-hexahydro-pyrrolo[3,2-b]pyrrole-1-carbonyl]-butyl}-amide d'acide thiophène-3-carboxylique ;
N-{1-[6-oxo-4-(pyridine-2-ylméthanesulfonyl)-hexahydro-pyrrolo[3,2-b]pyrrole-1-carbonyl]-butyl}-4-phénoxy-benzamide ;
N-{1-[6-oxo-4-(pyridine-2-ylméthanesulfonyl)-hexahydro-pyrrolo[3,2-b]pyrrole-1-carbonyl]-butyl}-4-(4-méthyl-pipérazine-1-yl)-benzamide ;
4-méthyl-N-{1-[6-oxo-4-(pyridine-2-ylméthanesulfonyl)-hexahydro-pyrrolo[3,2-b]pyrrole-1-carbonyl]-butyl}-benzamide ;
4-méthoxy-N-{1-[6-oxo-4-(pyridine-2-ylméthanesulfonyl)-hexahydro-pyrrolo[3,2-b]pyrrole-1-carbonyl]-butyl}-benzamide ;
4-isopropyl-N-{1-[6-oxo-4-(pyridine-2-ylméthanesulfonyl)-hexahydro-pyrrolo[3,2-b]pyrrole-1-carbonyl]-butyl}-benzamide ;
N-{1-[6-oxo-4-(pyridine-2-ylméthanesulfonyl)-hexahydro-pyrrolo[3,2-b]pyrrole-1-carbonyl]-butyl}-4-vinyl-benzamide ;

4-imidazole-1-yl-N-{1-[6-oxo-4-(pyridine-2-ylméthane-sulfonyl)-hexahydro-pyrrolo[3,2-b]pyrrole-1-carbonyl]-butyl}-benzamide ;

N-{1-[6-oxo-4-(pyridine-2-ylméthanesulfonyl)-hexahydro-pyrrolo[3,2-b]pyrrole-1-carbonyl]-butyl}-4-thiophène-2-yl-benzamide ;

N-{1-[6-oxo-4-(pyridine-2-ylméthanesulfonyl)-hexahydro-pyrrolo[3,2-b]pyrrole-1-carbonyl]-butyl}-4-oxazole-5-yl-be nzamide ;

{1-[6-oxo-4-(pyridine-2-ylméthanesulfonyl)-hexahydro-pyrrolo[3,2-b]pyrrole-1-carbonyl]-butyl}-amide d'acide 5-phényl-thiophène-2-carboxylique ;

N-{1-[6-oxo-4-(pyridine-2-ylméthanesulfonyl)-hexahydro-pyrrolo[3,2-b]pyrrole-1-carbonyl]-butyl}-4-trifluoro-méthoxybenzamide ;

{1-[6-oxo-4-(pyridine-2-ylméthanesulfonyl)-hexahydro-pyrrolo[3,2-b]pyrrole-1-carbonyl]-butyl}-amide d'acide 5-pyridine-2-yl-thiophène-2-carboxylique ;

4-difluorométhoxy-N-{1-[6-oxo-4-(pyridine-2-ylméthane-sulfonyl)-hexahydro-pyrrolo[3,2-b]pyrrole-1-carbonyl]-butyl}-benzamide ;

N-{1-[6-oxo-4-(pyridine-2-ylméthanesulfonyl)-hexahydro-pyrrolo[3,2-b]pyrrole-1-carbonyl]-butyl}-benzamide ;

{3-méthyl-1-[6-oxo-4-(pyridine-3-ylméthanesulfonyl)-hexahydro-pyrrolo[3,2-b]pyrrole-1-carbonyl]-butyl}-amide d'acide naphtalène-2-carboxylique ;

{3-méthyl-1-[6-oxo-4-(pyridine-3-ylméthanesulfonyl)-hexahydro-pyrrolo[3,2-b]pyrrole-1-carbonyl]-butyl}-amide d'acide quinoléine-2-carboxylique ;

{3-méthyl-1-[6-oxo-4-(pyridine-3-ylméthanesulfonyl)-hexahydro-pyrrolo[3,2-b]pyrrole-1-carbonyl]-butyl}-amide d'acide benzo[b]thiophène-2-carboxylique ;

{3-méthyl-1-[6-oxo-4-(pyridine-3-ylméthanesulfonyl)-hexahydro-pyrrolo[3,2-b]pyrrole-1-carbonyl]-butyl}-amide d'acide benzofuranne-2-carboxylique ;

{3-méthyl-1-[6-oxo-4-(pyridine-3-ylméthanesulfonyl)-hexahydro-pyrrolo[3,2-b]pyrrole-1-carbonyl]-butyl}-amide d'acide biphényl-4-carboxylique ;

4-tertiobutyl-N-{3-méthyl-1-[6-oxo-4-(pyridine-3-ylméthane-sulfonyl)-hexahydro-pyrrolo[3,2-b]pyrrole-1-carbonyl]-butyl}-benzamide ;

4-diméthylamino-N-{3-méthyl-1-[6-oxo-4-(pyridine-3-ylméthanesulfonyl)-hexahydro-pyrrolo[3,2-b]pyrrole-1-carbonyl]-butyl}-benzamide ;

{3-méthyl-1-[6-oxo-4-(pyridïne-3-ylméthanesulfonyl)-hexahydro-pyrrolo[3,2-b]pyrrole-1-carbonyl]-butyl}-amide d'acide 7-méthoxybenzofuranne-2-carboxylique ;

ester benzylique d'acide {3-méthyl-1-[6-oxo-4-(pyridine-3-ylméthanesulfonyl)-hexahydro-pyrrolo[3,2-b]pyrrole-1-carbonyl]-butyl}-carbamique ;

{3-méthyl-1-[6-oxo-4-(pyridine-3-ylméthanesulfonyl)-hexahydro-pyrrolo[3,2-b]pyrrole-1-carbonyl]-butyl}-amide d'acide 5-méthoxybenzofuranne-2-carboxylique ;

{3-méthyl-1-[6-oxo-4-(pyridine-3-ylméthanesulfonyl)-hexahydro-pyrrolo[3,2-b]pyrrole-1-carbonyl]-butyl}-amide d'acide thiéno[3,2-b]thiophène-2-carboxylique ;

{3-méthyl-1-[6-oxo-4-(pyridine-3-ylméthanesulfonyl)-hexahydro-pyrrolo[3,2-b]pyrrole-1-carbonyl]-butyl}-amide d'acide 3-méthyl-benzofuranne-2-carboxylique ;

{3-méthyl-1-[6-oxo-4-(pyridine-3-ylméthanesulfonyl)-hexahydro-pyrrolo[3,2-b]pyrrole-1-carbonyl]-butyl}-amide d'acide quinoxaline-2-carboxylique ;

{3-méthyl-1-[6-oxo-4-(pyridine-3-ylméthanesulfonyl)-hexahydro-pyrrolo[3,2-b]pyrrole-1-carbonyl]-butyl}-amide d'acide benzo[1,3]dioxole-5-carboxylique ;

{3-méthyl-1-[6-oxo-4-(pyridine-3-ylméthanesulfonyl)-hexahydro-pyrrolo[3,2-b]pyrrole-1-carbonyl]-butyl}-amide d'acide quinoléine-6-carboxylique ;

{3-méthyl-1-[6-oxo-4-(pyridine-3-ylméthanesulfonyl)-hexahydro-pyrrolo[3,2-b]pyrrole-1-carbonyl]-butyl}-amide d'acide furanne-2-carboxylique ;

{3-méthyl-1-[6-oxo-4-(pyridine-3-ylméthanesulfonyl)-hexahydro-pyrrolo[3,2-b]pyrrole-1-carbonyl]-butyl}-amide d'acide thiophène-2-carboxylique ;

N-{3-méthyl-1-[6-oxo-4-(pyridine-3-ylméthanesulfonyl)-hexahydro-pyrrolo[3,2-b]pyrrole-1-carbonyl]-butyl}-4-trifluo rométhyl-benzamide ;

{3-méthyl-1-[6-oxo-4-(pyridine-3-ylméthanesulfonyl)-hexahydro-pyrrolo[3,2-b]pyrrole-1-carbonyl]-butyl}-amide d'acide furanne-3-carboxylique ;

{3-méthyl-1-[6-oxo-4-(pyridine-3-ylméthanesulfonyl)-hexahydro-pyrrolo[3,2-b]pyrrole-1-carbonyl]-butyl}-amide d'acide thiophène-3-carboxylique ;

N-{3-méthyl-1- [6-oxo-4-(pyridine-3-ylméthanesulfonyl)-hexahydro-pyrrolo[3,2-b]pyrrole-1-carbonyl]-butyl}-4-phénoxy-benzamide ;

N-{3-méthyl-1-[6-oxo-4-(pyridine-3-ylméthanesulfonyl)-hexahydro-pyrrolo[3,2-b]pyrrole-1-carbonyl]-butyl}-4-(4-méthyl-pipérazine-1-yl)-benzamide ;
4-méthyl-N-{3-méthyl-1-[6-oxo-4-(pyridine-3-ylméthane-sulfonyl)-hexahydro-pyrrolo[3,2-b]pyrrole-1-carbonyl]-butyl}-benzamide ;
4-méthoxy-N-{3-méthyl-1-[6-oxo-4-(pyridine-3-ylméthanesulfonyl)-hexahydro-pyrrolo[3,2-b]pyrrole-1-carbonyl]-butyl}-benzamide ;
4-isopropyl-N-{3-méthyl-1-[6-oxo-4-(pyridine-3-ylméthane-sulfonyl)-hexahydro-pyrrolo[3,2-b]pyrrole-1-carbonyl]-butyl}-benzamide ;
N-{3-méthyl-1-[6-oxo-4-(pyridine-3-ylméthanesulfonyl)-hexahydro-pyrrolo[3,2-b]pyrrole-1-carbonyl]-butyl}-4-vinyl-benzamide ;
4-imidazole-1-yl-N-{3-méthyl-1-[6-oxo-4-(pyridine-3-ylméthanesulfonyl)-hexahydro-pyrrolo[3,2-b]pyrrole-1-carbonyl]-butyl}-benzamide ;
N-{3-méthyl-1-[6-oxo-4-(pyridine-3-ylméthanesulfonyl)-hexahydro-pyrrolo[3,2-b]pyrrole-1-carbonyl]-butyl}-4-thiophène-2-yl-benzamide ;
N-{3-méthyl-1-[6-oxo-4-(pyridine-3-ylméthanesulfonyl)-hexahydro-pyrrolo[3,2-b]pyrrole-1-carbonyl]-butyl}-4-oxazole-5-yl-benzamide ;
{3-méthyl-1-[6-oxo-4-(pyridine-3-ylméthanesulfonyl)-hexahydro-pyrrolo[3,2-b]pyrrole-1-carbonyl]-butyl}-amide d'acide 5-phényl-thiophène-2-carboxylique ;
N-{3-méthyl-1-[6-oxo-4-(pyridine-3-ylméthanesulfonyl)-hexahydro-pyrrolo[3,2-b]pyrrole-1-carbonyl]-butyl}-4-t rifluorométhoxy-benzamide ;
{3-méthyl-1-[6-oxo-4-(pyridine-3-ylméthanesulfonyl)-hexahydro-pyrrolo[3,2-b]pyrrole-1-carbonyl]-butyl}-amide d'acide 5-pyridine-2-yl-thiophène-2-carboxylique ;
4-difluorométhoxy-N-{3-méthyl-1-[6-oxo-4-(pyridine-3-ylméthanesulfonyl)-hexahydro-pyrrolo[3,2-b]pyrrole-1-carbonyl]-butyl}-benzamide ;
N-{3-méthyl-1-[6-oxo-4-(pyridine-3-ylméthanesulfonyl)-hexahydro-pyrrolo[3,2-b]pyrrole-1-carbonyl]-butyl}-4-morpholine-4-yl-benzamide ;
{1-[6-oxo-4-(pyridine-3-ylméthanesulfonyl)-hexahydro-pyrrolo[3,2-b]pyrrole-1-carbonyl]-butyl}-amide d'acide naphtalène-2-carboxylique ;
{1-[6-oxo-4-(pyridine-3-ylméthanesulfonyl)-hexahydro-pyrrolo[3,2-b]pyrrole-1-carbonyl]-butyl}-amide d'acide quinoléine-2-carboxylique ;
{1-[6-oxo-4-(pyridine-3-ylméthanesulfonyl)-hexahydro-pyrrolo[3,2-b]pyrrole-1-carbonyl]-butyl}-amide d'acide benzo[b]thiophène-2-carboxylique ;
{1-[6-oxo-4-(pyridine-3-ylméthanesulfonyl)-hexahydro-pyrrolo[3,2-b]pyrrole-1-carbonyl]-butyl}-amide d'acide benzofuranne-2-carboxylique ;
{1-[6-oxo-4-(pyridine-3-ylméthanesulfonyl)-hexahydro-pyrrolo[3,2-b]pyrrole-1-carbonyl]-butyl}-amide d'acide biphényl-4-carboxylique ;
4-tertiobutyl-N-{1-[6-oxo-4-(pyridine-3-ylméthanesulfonyl)-hexahydro-pyrrolo[3,2-b]pyrrole-1-carbonyl]-butyl}-benzamide ;
4-diméthylamino-N-{1-[6-oxo-4-(pyridine-3-ylméthane-sulfonyl)-hexahydro-pyrrolo[3,2-b]pyrrole-1-carbonyl]-butyl}-benzamide ;
{1-[6-oxo-4-(pyridine-3-ylméthanesulfonyl)-hexahydro-pyrrolo[3,2-b]pyrrole-1-carbonyl]-butyl}-amide d'acide 7-méthoxybenzofuranne-2-carboxylique ;
ester benzylique d'acide {1-[6-oxo-4-(pyridine-3-ylméthanesulfonyl)-hexahydro-pyrrolo[3,2-b]pyrrole-1-carbonyl]-butyl}-carbamique ;
{1-[6-oxo-4-(pyridine-3-ylméthanesulfonyl)-hexahydro-pyrrolo[3,2-b]pyrrole-1-carbonyl]-butyl}-amide d'acide 5-méthoxybenzofuranne-2-carboxylique ;
{1-[6-oxo-4-(pyridine-3-ylméthanesulfonyl)-hexahydro-pyrrolo[3,2-b]pyrrole-1-carbonyl]-butyl}-amide d'acide thiéno[3,2-b]thiophène-2-carboxylique ;
{1-[6-oxo-4-(pyridine-3-ylméthanesulfonyl)-hexahydro-pyrrolo[3,2-b]pyrrole-1-carbonyl]-butyl}-amide d'acide 3-méthyl-benzofuranne-2-carboxylique ;
{1-[6-oxo-4-(pyridine-3-ylméthanesulfonyl)-hexahydro-pyrrolo[3,2-b]pyrrole-1-carbonyl]-butyl}-amide d'acide quinoxaline-2-carboxylique ;
{1-[6-oxo-4-(pyridine-3-ylméthanesulfonyl)-hexahydro-pyrrolo[3,2-b]pyrrole-1-carbonyl]-butyl}-amide d'acide benzo|1,3]dioxole-5-carboxylique ;
{1-[6-oxo-4-(pyridine-3-ylméthanesulfonyl)-hexahydro-pyrrolo[3,2-b]pyrrole-1-carbonyl]-butyl}-amide d'acide quinoléine-6-carboxylique ;
{1-[6-oxo-4-(pyridine-3-ylméthanesulfonyl)-hexahydro-pyrrolo[3,2-b]pyrrole-1-carbonyl]-butyl}-amide d'acide furanne-2-carboxylique ;
{1-[6-oxo-4-(pyridine-3-ylméthanesulfonyl)-hexahydro-pyrrolo[3,2-b]pyrrole-1-carbonyl]-butyl}-amide d'acide thio-

phène-2-carboxylique ;
N-{1-[6-oxo-4-(pyridine-3-ylméthanesulfonyl)-hexahydro-pyrrolo[3,2-b]pyrrole-1-carbonyl]-butyl}-4-trifluorométhyl-benzamide ;
{1-[6-oxo-4-(pyridine-3-ylméthanesulfonyl)-hexahydro-pyrrolo[3,2-b]pyrrole-1-carbonyl]-butyl}-amide d'acide furanne-3-carboxylique ;
{1-[6-oxo-4-(pyridine-3-ylméthanesulfonyl)-hexahydro-pyrrolo[3,2-b]pyrrole-1-carbonyl]-butyl}-amide d'acide thiophène-3-carboxylique ;
N-{1-[6-oxo-4-(pyridine-3-ylméthanesulfonyl)-hexahydro-pyrrolo[3,2-b]pyrrole-1-carbonyl]-butyl}-4-phénoxy-benzamide ;
N-{1-[6-oxo-4-(pyridine-3-ylméthanesulfonyl)-hexahydro-pyrrolo[3,2-b]pyrrole-1-carbonyl]-butyl}-4-(méthyl-pipérazine-1-yl)-benzamide ;
4-méthyl-N-{1-[6-oxo-4-(pyridine-3-ylméthanesulfonyl)-hexahydro-pyrrolo[3,2-b]pyrrole-1-carbonyl]-butyl}-benzamide ;
4-méthoxy-N-{1-[6-oxo-4-(pyridine-3-ylméthanesulfonyl)-hexahydro-pyrrolo[3,2-b]pyrrole-1-carbonyl]-butyl}-benzamide ;
4-isopropyl-N-{1-[6-oxo-4-(pyridine-3-ylméthanesulfonyl)-hexahydro-pyrrolo[3,2-b]pyrrole-1-carbonyl]-butyl}-benzamide ;
N-{1-[6-oxo-4-(pyridine-3-ylméthanesulfonyl)-hexahydro-pyrrolo[3,2-b]pyrrole-1-carbonyl]-butyl}-4-vinyl-benzamide ;
4-imidazole-1-yl-N-{1-[6-oxo-4-(pyridine-3-ylméthane-sulfonyl)-hexahydro-pyrrolo[3,2-b]pyrrole-1-carbonyl]-butyl}-benzamide ;
N-{1-[6-oxo-4-(pyridine-3-ylméthanesulfonyl)-hexahydro-pyrrolo[3,2-b]pyrrole-l-carbonyl]-butyl}-4-thiophène-2-yl-benzamide ;
N-{1-[6-oxo-4-(pyridine-3-ylméthanesulfonyl)-hexahydro-pyrrolo[3,2-b]pyrrole-1-carbonyl]-butyl}-4-oxazole-5-yl-benzamide ;
{1-[6-oxo-4-(pyridine-3-ylméthanesulfonyl)-hexahydro-pyrrolo[3,2-b]pyrrole-1-carbonyl]-butyl}-amide d'acide 5-phényl-thiophène-2-carboxylique ;
N-{1-[6-oxo-4-(pyridine-3-ylméthanesulfonyl)-hexahydro-pyrrolo[3,2-b]pyrrole-1-carbonyl]-butyl}-4-trifluorométhoxy-benzamide ;
{1-[6-oxo-4-(pyridine-3-ylméthanesulfonyl)-hexahydro-pyrrolo[3,2-b]pyrrole-1-carbonyl]-butyl}-amide d'acide 5-pyridine-2-yl-thiophène-2-carboxylique ;
4-difluorométhoxy-N-{1-[6-oxo-4-(pyridine-3-ylméthane-sulfonyl)-hexahydro-pyrrolo[3,2-b]pyrrole-1-carbonyl]-butyl}-benzamide ;
N-{1-[6-oxo-4-(pyridine-3-ylméthanesulfonyl)-hexahydro-pyrrolo[3,2-b]pyrrole-1-carbonyl]-butyl}-4-morpholine-4-yl-benzamide ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide naphtalène-2-carboxylique ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide quinoléine-2-carboxylique ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide benzo[b]thiophène-2-carboxylique ;
{3-mé-thyl-1-[6-oxo-4-(1-oxypyridine-2-ylméthanesulfonyl)-hexahydro-pyrrolo[3,2-b]pyrrole-1-carbonyl]-butyl}-amide d'acide benzofuranne-2-carboxylique ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide biphényl-4-carboxylique ;
4-tertiobutyl-N-{3-méthyl-1-[6-oxo-4-(1-oxypyridine-2-ylméthanesulfonyl)-hexahydro-pyrrolo[3,2-b]pyrrole-1-carbonyl]-butyl}-benzamide ;
4-diméthylamino-N-{3-méthyl-1-[6-oxo-4-(1-oxypyridine-2-ylméthanesulfonyl)-hexahydro-pyrrolo[3,2-b]pyrrole-1-carbonyl]-butyl}-benzamide ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide 7-méthoxybenzofuranne-2-carboxylique ;
ester benzylique d'acide {3-méthyl-1-[6-oxo-4-(1-oxypyridine-2-ylméthanesulfonyl)-hexahydro-pyrrolo[3,2-b]pyrrole-1-carbonyl]-butyl}-carbamique ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide 5-méthoxybenzofuranne-2-carboxylique ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide thiéno[3,2-b]thiophène-2-carboxylique ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide 3-méthyl-benzofuranne-2-carboxylique ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide quinoxaline-2-carboxylique ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide benzo[1,3]dioxole-5-carboxylique ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide quinoléine-6-carboxylique ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide furanne-2-carboxylque ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide thiophène-2-carboxylique ;
N-{3-méthyl-1-[6-oxo-4-(1-oxypyridine-2-ylméthanesulfonyl)-hexahydro-pyrrolo[3,2-b]pyrrole-1-carbonyl]-butyl}-4-trifluorométhyl-benzamide ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide furanne-3-carboxylique ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide thiophène-3-carboxylique ;
N-{3-méthyl-1-[6-oxo-4-(1-oxypyridine-2-ylméthanesulfonyl)-hexahydro-pyrrolo[3,2-b]pyrrole-1-carbonyl]-butyl}-4-phénoxy-benzamide ;
N-{3-méthyl-1-[6-oxo-4-(1-oxypyridine-2-ylméthanesulfonyl)-hexahydro-pyrrolo[3,2-b]pyrrole-1-carbonyl]-butyl}-4-(4-méthyl-pipérazine-1-yl)-benzamide ;
4-méthyl-N-{3-méthyl-1-[6-oxo-4-(1-oxypyridine-2-ylméthane-sulfonyl)-hexahydro-pyrrolo[3,2-b]pyrrole-1-carbonyl]-butyl}-benzamide ;
4-méthoxy-N-{3-méthyl-1-[6-oxo-4-(1-oxypyridine-2-ylméthanesulfonyl)-hexahydro-pyrrolo[3,2-b]pyrrole-1-carbonyl]-butyl}-benzamide ;
4-isopropyl-N-{3-méthyl-1-[6-oxo-4-(1-oxypyridine-2-ylméthanesulfonyl)-hexahydro-pyrrolo[3,2-b]pyrrole-1-carbonyl]-butyl}-benzamide ;
N-{3-méthyl-1-[6-oxo-4-(1-oxypyridine-2-ylméthanesulfonyl)-hexahydro-pyrrolo[3,2-b]pyrrole-1-carbonyl]-butyl}-4-vinyl-benzamide ;
4-imidazole-1-yl-N-{3-méthyl-1-[6-oxo-4-(1-oxypyridine-2-ylméthanesulfonyl)-hexahydro-pyrrolo[3,2-b]pyrrole-1-carbonyl]-butyl}-benzamide ;
N-{3-méthyl-1-[6-oxo-4-(1-oxypyridine-2-ylméthanesulfonyl)-hexahydro-pyrrolo[3,2-b]pyrrole-1-carbonyl]-butyl}-4-thiophène-2-yl-benzamide ;
N-{3-méthyl-1-[6-oxo-4-(1-oxypyridine-2-ylméthanesulfonyl)-hexahydro-pyrrolo[3,2-b]pyrrole-1-carbonyl]-butyl}-4-oxazole-5-yl-benzamide ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide 5-phényl-thiophène-2-carboxylique ;
N-{3-méthyl-1-[6-oxo-4-(1-oxypyridine-2-ylméthanesulfonyl)-hexahydro-pyrrolo[3,2-b]pyrrole-1-carbonyl]-butyl}-4-trifluorométhoxy-benzamide ;
{3-méthyl-1-[6-oxo-4-(1-oxypyridine-2-ylméthanesulfonyl)-hexahydro-pyrrolo[3,2-b]pyrrole-1-carbonyl]-butyl}-amide d'acide 5-pyridine-2-yl-thiophène-2-carboxylique ;
4-difluorométhoxy-N-{3-méthyl-1-[6-oxo-4-(1-oxypyridine-2-ylméthanesulfonyl)-hexahydro-pyrrolo[3,2-b]pyrrole-1-carbonyl]-butyl}-benzamide ;
N-{3-méthyl-1-[6-oxo-4-(1-oxypyridine-2-ylméthanesulfonyl)-hexahydro-pyrrolo[3,2-b]pyrrole-1-carbonyl]-butyl}-4-morpholine-4-yl-benzamide ;
{1-[6-oxo-4-(1-oxypyridine-2-ylméthanesulfonyl)-hexahydro-pyrrolo[3,2-b]pyrrole-1-carbonyl]-butyl}-amide d'acide naphtalène-2-carboxylique ;
{1-[6-oxo-4-(1-oxypyridine-2-ylméthanesulfonyl)-hexahydro-pyrrolo[3,2-b]pyrrole-1-carbonyl]-butyl}-amide d'acide quinoléine-2-carboxylique ;
{1-[6-oxo-4-(1-oxypyridine-2-ylméthanesulfonyl)-hexahydro-pyrrolo[3,2-b]pyrrole-1-carbonyl]-butyl}-amide d'acide benzo[b]thiophène-2-carboxylique ;
{1-[6-oxo-4-(1-oxypyridine-2-ylméthanesulfonyl)-hexahydro-pyrrolo[3,2-b]pyrrole-1-carbonyl]-butyl}-amide d'acide benzofuranne-2-carboxylique ;
{1-[6-oxo-4-(1-oxypyridine-2-ylméthanesulfonyl)-hexahydro-pyrrolo[3,2-b]pyrrole-1-carbonyl]-butyl}-amide d'acide biphényl-4-carboxylique ;
4-tertiobutyl-N-{1-[6-oxo-4-(1-oxypyridine-2-ylméthane-sulfonyl)-hexahydro-pyrrolo[3,2-b]pyrrole-1-carbonyl]-butyl}-benzamide ;
4-diméthylamino-N-{1-[6-oxo-4-(1-oxypyridine-2-ylméthanesulfonyl)-hexahydro-pyrrolo[3,2-b]pyrrole-1-carbonyl]-butyl}-benzamide ;
{1-[6-oxo-4-(1-oxypyridine-2-ylméthanesulfonyl)-hexahydro-pyrrolo[3,2-b]pyrrole-1-carbonyl]-butyl}-amide d'acide 7-méthoxybenzofuranne-2-carboxylique ; ester benzylique d'acide {1-[6-oxo-4-(1-oxypyridine-2-ylméthanesulfonyl)-hexahydro-pyrrolo[3,2-b]pyrrole-1-carbonyl]-butyl}-carbamique ;
{1-[6-oxo-4-(1-oxypyridine-2-ylméthanesulfonyl)-hexahydro-pyrrolo[3,2-b]pyrrole-1-carbonyl]-butyl}-amide d'acide 5-méthoxybenzofuranne-2-carboxylique ;
{1-[6-oxo-4-(1-oxypyridine-2-ylméthanesulfonyl)-hexahydro-pyrrolo[3,2-b]pyrrole-1-carbonyl]-butyl}-amide d'acide thiéno[3,2-b]thiophène-2-carboxylique ;
{1-[6-oxo-4-(1-oxypyridine-2-ylméthanesulfonyl)-hexahydro-pyrrolo[3,2-b]pyrrole-1-carbonyl]-butyl}-amide d'acide 3-méthyl-benzofuranne-2-carboxylique ;
{1-[6-oxo-4-(1-oxypyridine-2-ylméthanesulfonyl)-hexahydro-pyrrolo[3,2-b]pyrrole-1-carbonyl]-butyl}-amide d'acide quinoxaline-2-carboxylique ;
{1-[6-oxo-4-(1-oxypyridine-2-ylméthanesulfonyl)-hexahydro-pyrrolo[3,2-b]pyrrole-1-carbonyl]-butyl}-amide d'acide benzo[1,3]dioxole-5-carboxylique ;
{1-[6-oxo-4-(1-oxypyridine-2-ylméthanesulfonyl)-hexahydro-pyrrolo[3,2-b]pyrrole-1-carbonyl]-butyl}-amide d'acide quinoléine-6-carboxylique ;
{1-[6-oxo-4-(1-oxypyridine-2-ylméthanesulfonyl)-hexahydro-pyrrolo[3,2-b]pyrrole-1-carbonyl]-butyl}-amide d'acide furanne-2-carboxylique ;
{1-[6-oxo-4-(1-oxypyridine-2-ylméthanesulfonyl)-hexahydro-pyrrolo[3,2-b]pyrrole-1-carbonyl]-butyl}-amide d'acide thiophène-2-carboxylique ;
N-{1-[6-oxo-4-(1-oxypyridine-2-ylméthanesulfonyl)-hexahydro-pyrrolo[3,2-b]pyrrole-1-carbonyl]-butyl}-4-trifluorom éthyl-benzamide ;
{1-[6-oxo-4-(1-oxypyridine-2-ylméthanesulfonyl)-hexahydro-pyrrolo[3,2-b]pyrrole-1-carbonyl]-butyl}-amide d'acide furanne-3-carboxylique ;
{1-[6-oxo-4-(1-oxypyridine-2-ylméthanesulfonyl)-hexahydro-pyrrolo[3,2-b]pyrrole-1-carbonyl]-butyl}-amide d'acide thiophène-3-carboxylique ;
N-{1-[6-oxo-4-(1-oxypyridine-2-ylméthanesulfonyl)-hexahydro-pyrrolo[3,2-b]pyrrole-1-carbonyl]-butyl}-4-phénoxy-benzamide ;
N-{1-[6-oxo-4-(1-oxypyridine-2-ylméthanesulfonyl)-hexahydro-pyrrolo[3,2-b]pyrrole-1-carbonyl]-butyl}-4-(4-méthyl-pipérazine-1-yl)-benzamide ;
4-méthyl-N-{1-[6-oxo-4-(1-oxypyridine-2-ylméthanesulfonyl)-hexahydro-pyrrolo[3,2-b]pyrrole-1-carbonyl]-butyl}-benzamide ;
4-méthoxy-N-{1-[6-oxo-4-(1-oxypyridine-2-ylméthane-sulfonyl)-hexahydro-pyrrolo[3,2-b]pyrrole-1-carbonyl]-butyl}-benzamide ;
4-isopropyl-N-{1-[6-oxo-4-(1-oxypyridine-2-ylméthanesulfonyl)-hexahydro-pyrrolo[3,2-b]pyrrole-1-carbonyl]-butyl}-benzamide ;
N-{1-[6-oxo-4-(1-oxypyridine-2-ylméthanesulfonyl)-hexahydro-pyrrolo[3,2-b]pyrrole-1-carbonyl]-butyl}-4-vinyl-benzamide ;
4-imidazole-1-yl-N-{1-[6-oxo-4-(1-oxypyridine-2-ylméthanesulfonyl)-hexahydro-pyrrolo[3,2-b]pyrrole-1-carbonyl]-butyl}-benzamide ;
N-{1-[6-oxo-4-(1-oxypyridine-2-ylméthanesulfonyl)-hexahydro-pyrrolo[3,2-b]pyrrole-1-carbonyl]-butyl}-4-thiophène-2-yl-benzamide ;
N-{1-[6-oxo-4-(1-oxypyridine-2-ylméthanesulfonyl)-hexahydro-pyrrolo[3,2-b]pyrrole-1-carbonyl]-butyl}-4-oxazole-5-yl-benzamide ;
{1-[6-oxo-4-(1-oxypyridine-2-ylméthanesulfonyl)-hexahydro-pyrrolo[3,2-b]pyrrole-1-carbonyl]-butyl}-amide d'acide 5-phényl-thiophène-2-carboxylique ;
N-{1-[6-oxo-4-(1-oxypyridine-2-ylméthanesulfonyl)-hexahydro-pyrrolo[3,2-b]pyrrole-1-carbonyl]-butyl}-4-trifluorométhoxy-benzamide ;
{1-[6-oxo-4-(1-oxypyridine-2-ylméthanesulfonyl)-hexahydro-pyrrolo[3,2-b]pyrrole-1-carbonyl]-butyl}-amide d'acide 5-pyridine-2-yl-thiophène-2-carboxylique ;
4-difluorométhoxy-N-{1-[6-oxo-4-(1-oxypyridine-2-ylméthanesulfonyl)-hexahydro-pyrrolo[3,2-b]pyrrole-1-carbonyl]-butyl}-benzamide ;
N-{1-[6-oxo-4-(1-oxypyridine-2-ylméthanesulfonyl)-hexahydro-pyrrolo[3,2-b]pyrrole-1-carbonyl]-butyl}-4-morpholine-4-yl-benzamide ;
{3-méthyl-1-[6-oxo-4-(1-oxypyridine-3-ylméthanesulfonyl)-hexahydro-pyrrolo[3,2-b]pyrrole-1-carbonyl]-butyl}-amide d'acide naphtalène-2-carboxylique ;
{3-méthyl-1-[6-oxo-4-(1-oxypyridine-3-ylméthanesulfonyl)-hexahydro-pyrrolo[3,2-b]pyrrole-1-carbonyl]-butyl}-amide d'acide quinoléine-2-carboxylique ;

{3-méthyl-1-[6-oxo-4-(1-oxypyridine-3-ylméthanesulfonyl)-hexahydro-pyrrolo[3,2-b]pyrrole-1-carbonyl]-butyl}-amide d'acide benzo[b]thiophène-2-carboxylique ;
{3-méthyl-1-[6-oxo-4-(1-oxypyridine-3-ylméthanesulfonyl)-hexahydro-pyrrolo[3,2-b]pyrrole-1-carbonyl]-butyl}-amide d'acide benzofuranne-2-carboxylique ;
{3-méthyl-1-[6-oxo-4-(1-oxypyridine-3-ylméthanesulfonyl)-hexahydro-pyrrolo[3,2-b]pyrrole-1-carbonyl]-butyl}-amide d'acide biphényl-4-carboxylique ;
4-tertiobutyl-N-{3-méthyl-1-[6-oxo-4-(1-oxypyridine-3-ylméthanesulfonyl)-hexahydro-pyrrolo[3,2-b]pyrrole-1-carbo nyl]-butyl}-benzamide ;
4-diméthylamino-N-{3-méthyl-1-[6-oxo-4-(1-oxypyridine-3-ylméthanesulfonyl)-hexahydro-pyrrolo[3,2-b]pyrrole-1-carbonyl]-butyl}-benzamide ;
{3-méthyl-1-[6-oxo-4-(1-oxypyridine-3-ylméthanesulfonyl)-hexahydro-pyrrolo[3,2-b]pyrrole-1-carbonyl]-butyl}-amide d'acide 7-méthoxybenzofuranne-2-carboxylique ;
ester benzylique d'acide {3-méthyl-1-[6-oxo-4-(1-oxypyridine-3-ylméthanesulfonyl)-hexahydro-pyrrolo[3,2-b]pyrrole-1-carbonyl]-butyl}-carbamique ;
{3-méthyl-1-[6-oxo-4-(1-oxypyridine-3-ylméthanesulfonyl)-hexahydro-pyrrolo[3,2-b]pyrrole-1-carbonyl]-butyl}-amide d'acide 5-méthoxybenzofuranne-2-carboxylique ;
{3-méthyl-1-[6-oxo-4-(1-oxypyridine-3-ylméthanesulfonyl)-hexahydro-pyrrolo[3,2-b]pyrrole-1-carbonyl]-butyl}-amide d'acide thiéno[3,2-b]thiophène-2-carboxylique ;
{3-méthyl-1-[6-oxo-4-(1-oxypyridine-3-ylméthanesulfonyl)-hexahydro-pyrrolo[3,2-b]pyrrole-1-carbonyl]-butyl}-amide d'acide 3-méthyl-benzofuranne-2-carboxylique ;
{3-méthyl-1-[6-oxo-4-(1-oxypyridine-3-ylméthanesulfonyl)-hexahydro-pyrrolo[3,2-b]pyrrole-1-carbonyl]-butyl}-amide d'acide quinoxaline-2-carboxylique ;
{3-méthyl-1-[6-oxo-4-(1-oxypyridine-3-ylméthanesulfonyl)-hexahydro-pyrrolo[3,2-b]pyrrole-1-carbonyl]-butyl}-amide d'acide benzo[1,3]dioxole-5-carboxylique ;
{3-méthyl-1-[6-oxo-4-(1-oxypyridine-3-ylméthanesulfonyl)-hexahydro-pyrrolo[3,2-b]pyrrole-1-carbonyl]-butyl}-amide d'acide quinoléine-6-carboxylique ;
{3-méthyl-1-[6-oxo-4-(1-oxypyridine-3-ylméthanesulfonyl)-hexahydro-pyrrolo[3,2-b]pyrrole-1-carbonyl]-butyl}-amide d'acide furanne-2-carboxylique ;
{3-méthyl-1-[6-oxo-4-(1-oxypyridine-3-ylméthanesulfonyl)-hexahydro-pyrrolo[3,2-b]pyrrole-1-carbonyl]-butyl}-amide d'acide thiophène-2-carboxylique ;
N-{3-méthyl-1-[6-oxo-4-(1-oxypyridine-3-ylméthanesulfonyl)-hexahydro-pyrrolo[3,2-b]pyrrole-1-carbonyl]-butyl}-4-trifluorométhyl-benzamide ;
{3-méthyl-1-[6-oxo-4-(1-oxypyridine-3-ylméthanesulfonyl)-hexahydro-pyrrolo[3,2-b]pyrrole-1-carbonyl]-butyl}-amide d'acide furanne-3-carboxylique ;
{3-méthyl-1-[6-oxo-4-(1-oxypyridine-3-ylméthanesulfonyl)-hexahydro-pyrrolo[3,2-b]pyrrole-1-carbonyl]-butyl}-amide d'acide thiophène-3-carboxylique ;
N-{3-méthyl-1-[6-oxo-4-(1-oxypyridine-3-ylméthanesulfonyl)-hexahydro-pyrrolo[3,2-b]pyrrole-1-carbonyl]-butyl}-4-phénoxy-benzamide ;
N-{3-méthyl-1-[6-oxo-4-(1-oxypyridine-3-ylméthanesulfonyl)-hexahydro-pyrrolo[3,2-b]pyrrole-1-carbonyl]-butyl}-4-(4-méthyl-pipérazine-1-yl)-benzamide ;
4-méthyl-N-{3-méthyl-1-[6-oxo-4-(1-oxypyridine-3-ylméthane-sulfonyl)-hexahydro-pyrrolo[3,2-b]pyrrole-1-carbonyl]-butyl}-benzamide ;
4-méthoxy-N-{3-méthyl-1-[6-oxo-4-(1-oxypyridine-3-ylméthanesulfonyl)-hexahydro-pyrrolo[3,2-b]pyrrole-1-carbonyl]-butyl}-benzamide ;
4-isopropyl-N-{3-méthyl-1-[6-oxo-4-(1-oxypyridine-3-ylméthanesulfonyl)-hexahydro-pyrrolo[3,2-b]pyrrole-1-carbonyl]-butyl}-benzamide ;
N-{3-méthyl-1-[6-oxo-4-(1-oxypyridine-3-ylméthanesulfonyl)-hexahydro-pyrrolo[3,2-b]pyrrole-1-carbonyl]-butyl}-4-vinyl-benzamide ;
4-imidazole-1-yl-N-{3-méthyl-1-[6-oxo-4-(1-oxypyridine-3-ylméthanesulfonyl)-hexahydro-pyrrolo[3,2-b]pyrrole-1--carbonyl]-butyl}-benzamide ;
N-{3-méthyl-1-[6-oxo-4-(1-oxypyridine-3-ylméthanesulfonyl)-hexahydro-pyrrolo[3,2-b]pyrrole-1-carbonyl]-butyl}-4-thiophène-2-yl-benzamide ;
N-{3-méthyl-1-[6-oxo-4-(1-oxypyridine-3-ylméthanesulfonyl)-hexahydro-pyrrolo[3,2-b]pyrrole-1-carbonyl]-butyl}-4-oxazole-5-benzamide ;
{3-méthyl-1-[6-oxo-4-(1-oxypyridine-3-ylméthanesulfonyl)-hexahydro-pyrrolo[3,2-b]pyrrole-1-carbonyl]-butyl}-amide d'acide 5-phényl-thiophène-2-carboxylique ;
N-{3-méthyl-1-[6-oxo-4-(1-oxypyridine-3-ylméthanesulfonyl)-hexahydro-pyrrolo[3,2-b]pyrrole-1-carbonyl]-butyl}-4-trifluorométhoxy-benzamide ;

{3-méthyl-1-[6-oxo-4-(1-oxypyridine-3-ylméthanesulfonyl)-hexahydro-pyrrolo[3,2-b]pyrrole-1-carbonyl]-butyl}-amide d'acide 5-pyridine-2-yl-thiophène-2-carboxylique ;
4-difluorométhoxy-N-{3-méthyl-1-[6-oxo-4-(1-oxypyridine-3-ylméthanesulfonyl)-hexahydro-pyrrolo[3,2-b]pyrrole-1-carbonyl]-butyl}-benzamide ;
N-{3-méthyl-1-[6-oxo-4-(1-oxypyridine-3-ylméthanesulfonyl)-hexahydro-pyrrolo[3,2-b]pyrrole-1-carbonyl]-butyl}-4-morpholine-4-yl-benzamide ;
{1-[6-oxo-4-(1-oxypyridine-3-ylméthanesulfonyl)-hexahydro-pyrrolo[3,2-b]pyrrole-1-carbonyl]-butyl}-amide d'acide naphtalène-2-carboxylique ;
{1-[6-oxo-4-(1-oxypyridine-3-ylméthanesulfonyl)-hexahydro-pyrrolo[3,2-b]pyrrole-1-carbonyl]-butyl}-amide d'acide quinoléine-2-carboxylique ;
{1-[6-oxo-4-(1-oxypyridine-3-ylméthanesulfonyl)-hexahydro-pyrrolo[3,2-b]pyrrole-1-carbonyl]-butyl}-amide d'acide benzo[b]thiophène-2-carboxylique ;
{1-[6-oxo-4-(1-oxypyridine-3-ylméthanesulfonyl)-hexahydro-pyrrolo[3,2-b]pyrrole-1-carbonyl]-butyl}-amide d'acide benzofuranne-2-carboxylique ;
{1-[6-oxo-4-(1-oxypyridine-3-ylméthanesulfonyl)-hexahydro-pyrrolo[3,2-b]pyrrole-1-carbonyl]-butyl}-amide d'acide biphényl-4-carboxylique ;
4-tertiobutyl-N-{1-[6-oxo-4-(1-oxypyridine-3-ylméthane-sulfonyl)-hexahydro-pyrrolo[3,2-b]pyrrole-1-carbonyl]-butyl}-benzamide ;
4-diméthylamino-N-{1-[6-oxo-4-(1-oxypyridine-3-ylméthane-sulfonyl)-hexahydro-pyrrolo[3,2-b]pyrrole-1-carbonyl]-butyl}-benzamide ;
{1-[6-oxo-4-(1-oxypyridine-3-ylméthanesulfonyl)-hexahydro-pyrrolo[3,2-b]pyrrole-1-carbonyl]-butyl}-amide d'acide 7-méthoxybenzofuranne-2-carboxylique ;
ester benzylique d'acide {1-[6-oxo-4-(1-oxypyridine-3-ylméthanesulfonyl)-hexahydro-pyrrolo[3,2-b]pyrrole-1-carbonyl]-butyl}-carbamique ;
{1-[6-oxo-4-(1-oxypyridine-3-ylméthanesulfonyl)-hexahydro-pyrrolo[3,2-b]pyrrole-1-carbonyl]-butyl}-amide d'acide 5-méthoxybenzofuranne-2-carboxylique ;
{1-[6-oxo-4-(1-oxypyridine-3-ylméthanesulfonyl)-hexahydro-pyrrolo[3,2-b]pyrrole-1-carbonyl]-butyl}-amide d'acide thiéno[3,2-b]thiophène-2-carboxylique ;
{1-[6-oxo-4-(1-oxypyridine-3-ylméthanesulfonyl)-hexahydro-pyrrolo[3,2-b]pyrrole-1-carbonyl]-butyl}-amide d'acide 3-méthyl-benzofuranne-2-carboxylique ;
{1-[6-oxo-4-(1-oxypyridine-3-ylméthanesulfonyl)-hexahydro-pyrrolo[3,2-b]pyrrole-1-carbonyl]-butyl}-amide d'acide quinoxaline-2-carboxylique ;
{1-[6-oxo-4-(1-oxypyridine-3-ylméthanesulfonyl)-hexahydro-pyrrolo[3,2-b]pyrrole-1-carbonyl]-butyl}-amide d'acide benzo[1,3]dioxole-5-carboxylique ;
{1-[6-oxo-4-(1-oxypyridine-3-ylméthanesulfonyl)-hexahydro-pyrrolo[3,2-b]pyrrole-1-carbonyl]-butyl}-amide d'acide quinoléine-6-carboxylique ;
{1-[6-oxo-4-(1-oxypyridine-3-ylméthanesulfonyl)-hexahydro-pyrrolo[3,2-b]pyrrole-1-carbonyl]-butyl}-amide d'acide furanne-2-carboxylique ;
{1-[6-oxo-4-(1-oxypyridine-3-ylméthanesulfonyl)-hexahydro-pyrrolo[3,2-b]pyrrole-1-carbonyl]-butyl}-amide d'acide thiophène-2-carboxylique ;
N-{1-[6-oxo-4-(1-oxypyridine-3-ylméthanesulfonyl)-hexahydro-pyrrolo[3,2-b]pyrrole-1-carbonyl]-butyl}-4-trifluorométhyl-benzamide ;
{1-[6-oxo-4-(1-oxypyridine-3-ylméthanesulfonyl)-hexahydro-pyrrolo[3,2-b]pyrrole-1-carbonyl]-butyl}-amide d'acide furanne-3-carboxylique ;
{1-[6-oxo-4-(1-oxypyridine-3-ylméthanesulfonyl)-hexahydro-pyrrolo[3,2-b]pyrrole-1-carbonyl]-butyl}-amide d'acide thiophène-3-carboxylique ;
N-{1-[6-oxo-4-(1-oxypyridine-3-ylméthanesulfonyl)-hexahydro-pyrrolo[3,2-b]pyrrole-1-carbonyl]-butyl}-4-phénoxy-benzamide ;
N-{1-[6-oxo-4-(1-oxypyridine-3-ylméthanesulfonyl)-hexahydro-pyrrolo [3,2-b]pyrrole-1-carbonyl]-butyl}-4-(4-méthyl-pipérazine-1-yl)-benzamide ;
4-méthyl-N-{1-[6-oxo-4-(1-oxypyridine-3-ylméthanesulfonyl)-hexahydro-pyrrolo[3,2-b]pyrrole-1-carbonyl]-butyl}-benzamide ;
4-méthoxy-N-{1-[6-oxo-4-(1-oxypyridine-3-ylméthane-sulfonyl)-hexahydro-pyrrolo[3,2-b]pyrrole-1-carbonyl]-butyl}-benzamide ;
4-isopropyl-N-{1-[6-oxo-4-(1-oxypyridine-3-ylméthane-sulfonyl)-hexahydro-pyrrolo[3,2-b]pyrrole-1-carbonyl]-butyl}-benzamide ;
N-{1-[6-oxo-4-(1-oxypyridine-3-ylméthanesulfonyl)-hexahydro-pyrrolo[3,2-b]pyrrole-1-carbonyl]-butyl}-4-vinyl-benzamide ;

4-imidazole-1-yl-N-{1-[6-oxo-4-(1-oxypyridine-3-ylméthanesulfonyl)-hexahydro-pyrrolo[3,2-b]pyrrole-1-carbonyl]-butyl}-benzamide ;
N-{1-[6-oxo-4-(1-oxypyridine-3-ylméthanesulfonyl)-hexahydro-pyrrolo[3,2-b]pyrrole-1-carbonyl]-butyl}-4-thiophène-2-yl-benzamide ;
N-{1-[6-oxo-4-(1-oxypyridine-3-ylméthanesulfonyl)-hexahydro-pyrrolo[3,2-b]pyrrole-1-carbonyl]-butyl}-4-oxazole-5-yl-benzamide ;
{1-[6-oxo-4-(1-oxypyridine-3-ylméthanesulfonyl)-hexahydro-pyrrolo[3,2-b]pyrrole-1-carbonyl]-butyl}-amide d'acide 5-phényl-thiophène-2-carboxylique ;
N-{1-[6-oxo-4-(1-oxypyridine-3-ylméthanesulfonyl)-hexahydro-pyrrolo[3,2-b]pyrrole-1-carbonyl]-butyl}-4-trifluorométhoxy-benzamide ;
{1-[6-oxo-4-(1-oxypyridine-3-ylméthanesulfonyl)-hexahydro-pyrrolo[3,2-b]pyrrole-1-carbonyl]-butyl}-amide d'acide 5-pyridine-2-yl-thiophène-2-carboxylique ;
4-difluorométhoxy-N-{1-[6-oxo-4-(1-oxypyridine-3-ylméthane-sulfonyl)-hexahydro-pyrrolo[3,2-b]pyrrole-1-carbonyl]-butyl}-benzamide ;
N-{1-[6-oxo-4-(1-oxypyridine-3-ylméthanesulfonyl)-hexahydro-pyrrolo[3,2-b]pyrrole-1-carbonyl]-butyl}-4-morpholine-4-yl-benzamide ;
4-tertiobutyl-N-{1-[6-oxo-4-(pyridine-2-sulfonyl)-hexahydro-pyrrolo[3,2-b]pyrrole-1-carbonyl]-cyclohexyl}-benzamide ;
{1-[6-oxo-4-(pyridine-2-sulfonyl)-hexahydro-pyrrolo-[3,2-b]pyrrole-1-carbonyl]-butyl}-amide d'acide 5-méthoxy-benzofuranne-2-carboxylique ;
{1-[6-oxo-4-(pyridine-2-sulfonyl)-hexahydro-pyrrolo-[3,2-b]pyrrole-1-carbonyl]-cyclohexyl}-amide d'acide thiéno-[3,2-b]thiophène-2-carboxylique ;
{1-[6-oxo-4-(pyridine-2-sulfonyl)-hexahydro-pyrrolo-[3,2-b]pyrrole-1-carbonyl]-cyclohexyl}-amide d'acide 3-méthyl-benzofuranne-2-carboxylique ;
N-{1-[6-oxo-4-(pyridine-2-sulfonyl)-hexahydro-pyrrolo-[3,2-b]pyrrole-1-carbonyl]-cyclohexyl}-4-phénoxy-benzamide ;
N-{1-[6-oxo-4-(pyridine-2-sulfonyl)-hexahydro-pyrrolo-[3,2-b]pyrrole-1-carbonyl]-cyclohexyl}-4-thiophène-2-yl-benzamide ;
4-tertiobutyl-N-{1-cyclopropylméthyl-2-oxo-2-[6-oxo-4-(pyridine-2-sulfonyl)-hexahydro-pyrrolo[3,2-b]pyrrole-1-yl}-éthyl]-benzamide ;
{1-cyclopropylméthyl-2-oxo-2-[6-oxo-4-(pyridine-2-sulfonyl)-hexahydro-pyrrolo[3,2-b]pyrrole-1-yl}-éthyl]-amide d'acide 5-méthoxy-benzofuranne-2-carboxylique ;
{1-cyclopropylméthyl-2-oxo-2-[6-oxo-4-(pyridine-2-sulfonyl)-hexahydro-pyrrolo[3,2-b]pyrrole-1-yl}-éthyl]-amide d'acide thiéno[3,2-b]thiophène-2-carboxylique ;
{1-cyclopropylméthyl-2-oxo-2-[6-oxo-4-(pyridine-2-sulfonyl)-hexahydro-pyrrolo[3,2-b]pyrrole-1-yl}-éthyl]-amide d'acide 3-méthyl-benzofuranne-2-carboxylique ;
N-{1-cyclopropylméthyl-2-oxo-2-[6-oxo-4-(pyridine-2-sulfonyl)-hexahydro-pyrrolo[3,2-b]pyrrole-1-yl}-éthyl]-4-phénoxy-benzamide ;
N-{1-cyclopropylméthyl-2-oxo-2-[6-oxo-4-(pyridine-2-sulfonyl)-hexahydro-pyrrolo[3,2-b]pyrrole-1-yl}-éthyl]-4-thiophène-2-yl-benzamide ;
4-tertiobutyl-N-{2-oxo-2-[6-oxo-4-(pyridine-2-sulfonyl)-hexahydro-pyrrolo[3,2-b]pyrrole-1-yl]-1-thiophène-2-ylméthyléthyl}-benzamide ;
{2-oxo-2-[6-oxo-4-(pyridine-2-sulfonyl)-hexahydro-pyrrolo[3,2-b]pyrrole-1-yl]-1-thiophène-2-ylméthyléthyl}-amide d'acide 5-méthoxy-benzofuranne-2-carboxylique ;
{2-oxo-2-[6-oxo-4-(pyridine-2-sulfonyl)-hexahydro-pyrrolo[3,2-b]pyrrole-1-yl]-1-thiophène-2-ylméthyléthyl}-amide d'acide thiéno[3,2-b]thiophène-2-carboxylique ;
{2-oxo-2-[6-oxo-4-(pyridine-2-sulfonyl)-hexahydro-pyrrolo[3,2-b]pyrrole-1-yl]-1-thiophène-2-ylméthyléthyl}-amide d'acide 3-méthyl-benzofuranne-2-carboxylique ;
N-{2-oxo-2-[6-oxo-4-(pyridine-2-sulfonyl)-hexahydro-pyrrolo[3,2-b]pyrrole-1-yl]-1-thiophène-2-ylméthyléthyl}-4-phénoxy-benzamide ;
N-{2-oxo-2-[6-oxo-4-(pyridine-2-sulfonyl)-hexahydro-pyrrolo[3,2-b]pyrrole-1-yl]-1-thiophène-2-ylméthyléthyl}-4-thiophène-2-yl-benzamide ;
4-tertiobutyl-N-{1-[6-oxo-4-(pyridine-3-sulfonyl)-hexa-hydro-pyrrolo[3,2-b]pyrrole-1-carbonyl]-cyclohexyl}-benzamide ;
{1-[6-oxo-4-(pyridine-3-sulfonyl)-hexa-hydro-pyrrolo[3,2-b]pyrrole-1-carbonyl]-cyclohexyl}-amide d'acide 5-méthoxy-benzofuranne-2-carboxylique ;
{1-[6-oxo-4-(pyridine-3-sulfonyl)-hexa-hydro-pyrrolo[3,2-b]pyrrole-1-carbonyl]-cyclohexyl}-amide d'acide thiéno-[3,2-b]thiophène-2-carboxylique ;

{1-[6-oxo-4-(pyridine-3-sulfonyl)-hexa-hydro-pyrrolo[3,2'-b]pyrrole-1-carbonyl]-cyclohexyl}-amide d'acide 3-méthyl-benzofuranne-2-carboxylique ;

N-{1-[6-oxo-4-(pyridine-3-sulfonyl)-hexa-hydro-pyrrolo[3,2-b]pyrrole-1-carbonyl]-cyclohexyl}-4-phénoxy-benzamide ;

N-{1-[6-oxo-4-(pyridine-3-sulfonyl)-hexa-hydro-pyrrolo[3,2-b]pyrrole-1-carbonyl]-cyclohexyl}-4-thiophène-2-yl-benzamide ;

4-tertiobutyl-N-{1-cyclopropylméthyl-2-oxo-2-(6-oxo-4-(pyridine-3-sulfonyl)-hexahydro-pyrrolo[3,2-b]pyrrole-1-yl]-éthyl}-benzamide ;

{1-cyclopropylméthyl-2-oxo-2-(6-oxo-4-(pyridine-3-sulfonyl)-hexahydro-pyrrolo[3,2-b]pyrrole-1-yl]-éthyl}-amide d'acide 5-méthoxy-benzofuranne-2-carboxylique ;

{1-cyclopropylméthyl-2-oxo-2-(6-oxo-4-(pyridine-3-sulfonyl)-hexahydro-pyrrolo[3,2-b]pyrrole-1-yl]-éthyl}-amide d'acide thiéno[3,2-b]thiophène-2-carboxylique ;

{1-cyclopropylméthyl-2-oxo-2-(6-oxo-4-(pyridine-3-sulfonyl)-hexahydro-pyrrolo[3,2-b]pyrrole-1-yl]-éthyl}-amide d'acide 3-méthyl-benzofuranne-2-carboxylique ;

N-{1-cyclopropylméthyl-2-oxo-2-(6-oxo-4-(pyridine-3-sulfonyl)-hexahydro-pyrrolo[3,2-b]pyrrole-1-yl]-éthyl}-4-phénoxy-benzamide ;

N-{1-cyclopropylméthyl-2-oxo-2-(6-oxo-4-(pyridine-3-sulfonyl)-hexahydro-pyrrolo[3,2-b]pyrrole-1-yl]-éthyl}-4-thiophène-2-yl-benzamide ;

4-tertiobutyl-N-{2-oxo-2-[6-oxo-4-(pyridine-3-sulfonyl)-hexahydro-pyrrolo[3,2-b]pyrrole-1-yl]-1-thiophène-2-yl-méthyléthyl}-benzamide ;

{2-oxo-2-[6-oxo-4-(pyridine-3-sulfonyl)-hexahydro-pyrrolo[3,2-b]pyrrole-1-yl]-1-thiophène-2-yl-méthyléthyl}-amide d'acide 5-mêthoxy-benzofuranne-2-carboxylique ;

{2-oxo-2-[6-oxo-4-(pyridine-3-sulfonyl)-hexahydro-pyrrolo[3,2-b]pyrrole-1-yl]-1-thiophène-2-yl-méthyléthyl}-amide d'acide thiéno[3,2-b]thiophène-2-carboxylique ;

{2-oxo-2-[6-oxo-4-(pyridine-3-sulfonyl)-hexahydro-pyrrolo[3,2-b]pyrrole-1-yl]-1-thiophène-2-yl-méthyléthyl}-amide d'acide 3-méthyl-benzofuranne-2-carboxylique ;

N-{2-oxo-2-[6-oxo-4-(pyridine-3-sulfonyl)-hexahydro-pyrrolo[3,2-b]pyrrole-1-yl]-1-thiophène-2-yl-méthyléthyl}-4-phénoxy-benzamide ;

N-{2-oxo-2-[6-oxo-4-(pyridine-3-sulfonyl)-hexahydro-pyrrolo[3,2-b]pyrrole-1-yl]-1-thiophène-2-yl-méthyléthyl}-4-thiophène-2-yl-benzamide ;

4-tertiobutyl-N-{1-[6-oxo-4-(1-oxypyridine-2-sulfonyl)-hexahydro-pyrrolo[3,2-b]pyrrole-1-carbonyl]-cyclohexyl}-benzamide ;

{1-[6-oxo-4-(1-oxypyridine-2-sulfonyl)-hexahydro-pyrrolo-[3,2-b]pyrrole-1-carbonyl]-cyclohexyl}-amide d'acide 5-méthoxy-benzofuranne-2-carboxylique ;

{1-[6-oxo-4-(1-oxypyridine-2-sulfonyl)-hexahydro-pyrrolo-[3,2-b]pyrrole-1-carbonyl]-cyclohexyl}-amide d'acide thiéno[3,2-b]thiophène-2-carboxylique ;

{1-[6-oxo-4-(1-oxypyridine-2-sulfonyl)-hexahydro-pyrrolo-[3,2-b]pyrrole-1-carbonyl]-cyclohexyl}-amide d'acide 3-méthyl-benzofuranne-2-carboxylique ;

N-{1-[6-oxo-4-(1-oxypyridine-2-sulfonyl)-hexahydro-pyrrolo-[3,2-b]pyrrole-1-carbonyl]-cyclohexyl}-4-phénoxy-benzamide ;

N-{1-[6-oxo-4-(1-oxypyridine-2-sulfonyl)-hexahydro-pyrrolo-[3,2-b]pyrrole-1-carbonyl]-cyclohexyl}-4-thiophène-2-yl-benzamide ;

4-tertiobutyl-N-{1-cyclopropylméthyl-2-oxo-2-[6-oxo-4-(1-oxypyridine-2-sulfonyl)-hexahydro-pyrrolo[3,2-b]pyrrole-1-yl]-éthyl}-benzamide ;

{1-cyclopropylméthyl-2-oxo-2-[6-oxo-4-(1-oxypyridine-2-sulfonyl)-hexahydro-pyrrolo[3,2-b]pyrrole-1-yl]-éthyl}-amide d'acide 5-méthoxybenzofuranne-2-carboxylique ;

{1-cyclopropylméthyl-2-oxo-2-[6-oxo-4-(1-oxypyridine-2-sulfonyl)-hexahydro-pyrrolo[3,2-b]pyrrole-1-yl]-éthyl}-amide d'acide thiéno[3,2-b]thiophène-2-carboxylique ;

{1-cyclopropylméthyl-2-oxo-2-[6-oxo-4-(1-oxypyridine-2-sulfonyl)-hexahydro-pyrrolo[3,2-b]pyrrole-1-yl]-éthyl}-amide d'acide 3-méthyl-benzofuranne-2-carboxylique ;

N-{1-cyclopropylméthyl-2-oxo-2-[6-oxo-4-(1-oxypyridine-2-sulfonyl)-hexahydro-pyrrolo[3,2-b]pyrrole-1-yl]-éthyl}-4-phénoxy-benzamide ;

N-{1-cyclopropylméthyl-2-oxo-2-[6-oxo-4-(1-oxypyridine-2-sulfonyl)-hexahydro-pyrrolo[3,2-b]pyrrole-1-yl]-éthyl}-4-thiophène-2-yl-benzamide ;

4-tertiobutyl-N-{2-oxo-2-[6-oxo-4-(1-oxypyridine-2-sulfonyl)-hexahydro-pyrrolo[3,2-b]pyrrole-1-yl]-thiophène-2-ylméthyléthyl}-benzamide ;

{2-oxo-2-[6-oxo-4-(1-oxypyridine-2-sulfonyl)-hexahydro-pyrrolo[3,2-b]pyrrole-1-yl]-thiophène-2-ylméthyléthyl}-amide d'acide 5-méthoxy-benzofuranne-2-carboxylique ;

{2-oxo-2-[6-oxo-4-(1-oxypyridine-2-sulfonyl)-hexahydro-pyrrolo[3,2-b]pyrrole-1-yl]-thiophène-2-ylméthyléthyl}-amide d'acide thiéno[3,2-b]thiophène-2-carboxylique ;
{2-oxo-2-[6-oxo-4-(1-oxypyridine-2-sulfonyl)-hexahydro-pyrrolo[3,2-b]pyrrole-1-yl]-thiophène-2-ylméthyléthyl}-amide d'acide 3-méthyl-benzofuranne-2-carboxylique ;
N-{2-oxo-2-[6-oxo-4-(1-oxypyridine-2-sulfonyl)-hexahydro-pyrrolo[3,2-b]pyrrole-1-yl]-thiophène-2-ylméthyléthyl}-4-phénoxy-benzamide ;
N-{2-oxo-2-[6-oxo-4-(1-oxypyridine-2-sulfonyl)-hexahydro-pyrrolo[3,2-b]pyrrole-1-yl]-thiophène-2-ylméthyléthyl}-4-thiophène-2-yl-benzamide ;
4-tertiobutyl-N-{1-[6-oxo-4-(1-oxypyridine-3-sulfonyl)-hexahydro-pyrrolo[3,2-b]pyrrole-1-carbonyl]-cyclohexyl}-ben zamide ;
{1-[6-oxo-4-(1-oxypyridine-3-sulfonyl)-hexahydro-pyrrolo-[3,2-b]pyrrole-1-carbonyl]-cyclohexyl}-amide d'acide 5-méthoxy-benzofuranne-2-carboxylique ;
{1-[6-oxo-4-(1-oxypyridine-3-sulfonyl)-hexahydro-pyrrolo-[3,2-b]pyrrole-1-carbonyl]-cyclohexyl}-amide d'acide thiéno[3,2-b]thiophène-2-carboxylique ;
{1-[6-oxo-4-(1-oxypyridine-3-sulfonyl)-hexahydro-pyrrolo-[3,2-b]pyrrole-1-carbonyl]-cyclohexyl}-amide d'acide 3-méthyl-benzofuranne-2-carboxylique ;
N-{1-[6-oxo-4-(1-oxypyridine-3-sulfonyl)-hexahydro-pyrrolo[3,2-b]pyrrole-1-carbonyl]-cyclohexyl}-4-phénoxy-benz amide ;
N-{1-[6-oxo-4-(1-oxypyridine-3-sulfonyl)-hexahydro-pyrrolo[3,2-b]pyrrole-1-carbonyl]-cyclohexyl}-4-thiophéne-2-yl-benzamide ;
4-tertiobutyl-N-{1-cyclopropylméthyl-2-oxo-2-[6-oxo-4-(1-oxy-pyridine-3-sulfonyl)-hexahydro-pyrrolo[3,2-b]pyrrole-1-yl]-éthyl}-benzamide ;
{1-cyclopropylméthyl-2-oxo-2-[6-oxo-4-(1-oxy-pyridine-3-sulfonyl)-hexahydro-pyrrolo[3,2-b]pyrrole-1-yl]-éthyl}-amide d'acide 5-méthoxy-benzofuranne-2-carboxylique ;
{1-cyclopropylméthyl-2-oxo-2-[6-oxo-4-(1-oxy-pyridine-3-sulfonyl)-hexahydro-pyrrolo[3,2-b]pyrrole-1-yl]-éthyl}-amide d'acide thiéno[3,2-b]thiophène-2-carboxylique ;
{1-cyclopropylméthyl-2-oxo-2-[6-oxo-4-(1-oxy-pyridine-3-sulfonyl)-hexahydro-pyrrolo[3,2-b]pyrrole-1-yl]-éthyl}-amide d'acide 3-méthyl-benzofuranne-2-carboxylique ;
N-{1-cyclopropylméthyl-2-oxo-2-[6-oxo-4-(1-oxy-pyridine-3-sulfonyl)-hexahydro-pyrrolo[3,2-b]pyrrole-1-yl]-éthyl}-4-phénoxy-benzamide ;
N-{1-cyclopropylméthyl-2-oxo-2-[6-oxo-4-(1-oxy-pyridine-3-sulfonyl)-hexahydro-pyrrolo[3,2-b]pyrrole-1-yl]-éthyl}-4-thiophène-2-yl-benzamide ;
4-tertiobutyl-N-{2-oxo-2-[6-oxo-4-(1-oxy-pyridine-3-sulfonyl)-hexahydro-pyrrolo[3,2-b]pyrrole-1-yl]-1-thio-phène-2-ylméthyl-éthyl}-benzamide ;
{2-oxo-2-[6-oxo-4-(1-oxy-pyridine-3-sulfonyl)-hexahydro-pyrrolo[3,2-b]pyrrole-1-yl]-1-thiophène-2-ylméthyl-éthyl}-amide d'acide 5-méthoxy-benzofuranne-2-carboxylique ;
{2-oxo-2-[6-oxo-4-(1-oxy-pyridine-3-sulfonyl)-hexahydro-pyrrolo[3,2-b]pyrrole-1-yl]-1-thiophène-2-ylméthyl-éthyl}-amide d'acide thiéno[3,2-b]thiophène-2-carboxylique ;
{2-oxo-2-[6-oxo-4-(1-oxy-pyridine-3-sulfonyl)-hexahydro-pyrrolo[3,2-b]pyrrole-1-yl]-1-thiophène-2-ylméthyl-éthyl}-amide d'acide 3-méthyl-benzofuranne-2-carboxylique ;
N-{2-oxo-2-[6-oxo-4-(1-oxy-pyridine-3-sulfonyl)-hexahydro-pyrrolo[3,2-b]pyrrole-1-yl]-1-thiophène-2-ylméthyl-éthyl}-4-phénoxy-benzamide ;
N-{2-oxo-2-[6-oxo-4-(1-oxy-pyridine-3-sulfonyl)-hexahydro-pyrrolo[3,2-b]pyrrole-1-yl]-1-thiophène-2-ylméthyl-éthyl}-4-thiophène-2-yl-benzamide ;
4-tertiobutyl-N-{1-[6-oxo-4-(pyridine-2-carbonyl)-hexa-hydro-pyrrolo[3,2-b]pyrrole-1-carbonyl]-cyclohexyl}-benzam ide ;
{1-[6-oxo-4-(pyridine-2-carbonyl)-hexahydro-pyrrolo[3,2-b]pyrrole-1-carbonyl]-cyclohexyl}-amide d'acide 5-méthoxy-benzofuranne-2-carboxylique ;
{1-[6-oxo-4-(pyridine-2-carbonyl)-hexahydro-pyrrolo[3,2-b]pyrrole-1-carbonyl]-cyclohexyl}-amide d'acide thiéno-[3,2-b]thiophène-2-carboxylique ;
{1-[6-oxo-4-(pyridine-2-carbonyl)-hexahydro-pyrrolo[3,2-b]pyrrole-1-carbonyl]-cyclohexyl}-amide d'acide 3-méthyl-benzofuranne-2-carboxylique ;
N-{1-[6-oxo-4-(pyridine-2-carbonyl)-hexahydro-pyrrolo[3,2-b]pyrrole-1-carbonyl]-cyclohexyl}-4-phénoxy-benzamide ;
N-{1-[6-oxo-4-(pyridine-2-carbonyl)-hexahydro-pyrrolo[3,2-b]pyrrole-1-carbonyl]-cyclohexyl}-4-thiophène-2-yl-benzamide ;
4-tertiobutyl-N-{1-cyclopropylméthyl-2-oxo-2-[6-oxo-4-(pyridine-2-carbonyl)-hexahydro-pyrrolo[3,2-b]pyrrole-1-yl]-éthyl}-benzamide ;

{1-cyclopropylmêthyl-2-oxo-2-[6-oxo-4-(pyridine-2-carbonyl)-hexahydro-pyrrolo[3,2-b]pyrrole-1-yl]-éthyl}-amide d'acide 5-méthoxy-benzofuranne-2-carboxylique ;
{1-cyclopropylméthyl-2-oxo-2-[6-oxo-4-(pyridine-2-carbonyl)-hexahydro-pyrrolo[3,2-b]pyrrole-1-yl]-éthyl}-amide d'acide thiéno[3,2-b]thiophène-2-carboxylique ;
{1-cyclopropylméthyl-2-oxo-2-[6-oxo-4-(pyridine-2-carbonyl)-hexahydro-pyrrolo[3,2-b]pyrrole-1-yl]-éthyl}-amide d'acide 3-méthyl-benzofuranne-2-carboxylique ;
N-{1-cyclopropylméthyl-2-oxo-2-[6-oxo-4-(pyridine-2-carbonyl)-hexahydro-pyrrolo[3,2-b]pyrrole-1-yl]-éthyl}-4-phénoxy-benzamide ;
N-{1-cyclopropylméthyl-2-oxo-2-[6-oxo-4-(pyridine-2-carbonyl)-hexahydro-pyrrolo[3,2-b]pyrrole-1-yl]-éthyl}-4-thiophène-2-yl-benzamide ;
4-tertiobutyl-N-{2-oxo-2-[6-oxo-4-(pyridine-2-carbonyl)-hexahydro-pyrrolo[3,2-b]pyrrole-1-yl]-1-thiophène-2-ylméthyléthyl}-benzamide ;
{2-oxo-2-[6-oxo-4-(pyridine-2-carbonyl)-hexahydro-pyrrolo[3,2-b]pyrrole-1-yl]-1-thiophène-2-ylméthyl-éthyl}-amide d'acide 5-méthoxy-benzofuranne-2-carboxylique ;
{2-oxo-2-[6-oxo-4-(pyridine-2-carbonyl)-hexahydro-pyrrolo[3,2-b]pyrrole-1-yl]-1-thiophène-2-ylméthyl-éthyl}-amide d'acide thiéno[3,2-b]thiophène-2-carboxylique ;
{2-oxo-2-[6-oxo-4-(pyridine-2-carbonyl)-hexahydro-pyrrolo[3,2-b]pyrrole-1-yl]-1-thiophène-2-ylmêthyl-éthyl}-amide d'acide 3-méthyl-benzofuranne-2-carboxylique ;
N-{2-oxo-2-[6-oxo-4-(pyridine-2-carbonyl)-hexahydro-pyrrolo[3,2-b]pyrrole-1-yl]-1-thiophène-2-ylméthyl-éthyl}-4-phénoxy- benzamide ;
N-{2-oxo-2-[6-oxo-4-(pyridine-2-carbonyl)-hexahydro-pyrrolo[3,2-b]pyrrole-1-yl]-1-thiophène-2-ylméthyl-éthyl}-4-thiophène-2-yl-benzamide ;
4-tertiobutyl-N-{1-[6-oxo-4-(2-pyridine-3-ylacétyl)-hexahydro-pyrrolo[3,2-b]pyrrole-1-carbonyl]-cyclohexyl}-benza mide ;
{1-[6-oxo-4-(2-pyridine-3-ylacétyl)-hexahydro-pyrrolo[3,2-b]pyrrole-1-carbonyl]-cyclohexyl}-amide d'acide 5-méthoxy-benzofuranne-2-carboxylique ;
{1-[6-oxo-4-(2-pyridine-3-ylacétyl)-hexahydro-pyrrolo[3,2-b]pyrrole-1-carbonyl]-cyclohexyl}-amide d'acide thiéno-[3,2-b]thiophène-2-carboxylique ;
{1-[6-oxo-4-(2-pyridine-3-ylacétyl)-hexahydro-pyrrolo[3,2-b]pyrrole-1-carbonyl]-cyclohexyl}-amide d'acide 3-méthyl-benzofuranne-2-carboxylique ;
N-{1-[6-oxo-4-(2-pyridine-3-ylacétyl)-hexahydro-pyrrolo[3,2-b]pyrrole-1-carbonyl]-cyclohexyl}-4-phénoxy-benzamide ;
N-{1-[6-oxo-4- (2-pyriàine-3-ylacétyl)-hexahydro-pyrrolo[3,2-b]pyrrole-1-carbonyl]-cyclohexyl}-4-thiophène-2-yl-benzamide ;
4-tertiobutyl-N-{1-cyclopropylméthyl-2-oxo-2-[6-oxo-4-(2-pyridine-3-ylacétyl)-hexahydro-pyrrolo[3,2-b]pyrrole-1-yl]-éthyl}-benzamide ;
{1-cyclopropylméthyl-2-oxo-2-[6-oxo-4-(2-pyridine-3-ylacétyl)-hexahydro-pyrrolo[3,2-b]pyrrole-1-yl]-éthyl}-amide d'acide 5-méthoxy-benzofuranne-2-carboxylique ;
{1-cyclopropylméthyl-2-oxo-2-[6-oxo-4-(2-pyridine-3-ylacétyl)-hexahydro-pyrrolo[3,2-b]pyrrole-1-yl]-éthyl}-amide d'acide thiéno[3,2-b]thiophène-2-carboxylique ;
{1-cyclopropylméthyl-2-oxo-2-[6-oxo-4-(2-pyridine-3-ylacétyl)-hexahydro-pyrrolo[3,2-b]pyrrole-1-yl]-éthyl}-amide d'acide 3-méthyl-benzofuranne-2-carboxylique ;
N-{1-cyclopropylméthyl-2-oxo-2-[6-oxo-4-(2-pyridine-3-ylacétyl)-hexahydro-pyrrolo[3,2-b]pyrrole-1-yl]-éthyl}-4-phénoxy-benzamide ;
N-{1-cyclopropylméthyl-2-oxo-2-[6-oxo-4-(2-pyridine-3-ylacétyl)-hexahydro-pyrrolo[3,2-b]pyrrole-1-yl]-éthyl}-4-thiophène-2-yl-benzamide ;
4-tertiobutyl-N-{2-oxo-2-[6-oxo-4-(2-pyridine-3-ylacétyl)-hexahydro-pyrrolo[3,2-b]pyrrole-1-yl]-1-thiophène-2-ylméthyl-éthyl]-benzamide ;
{2-oxo-2-[6-oxo-4-(2-pyridine-3-ylacétyl)-hexahydro-pyrrolo[3,2-b]pyrrole-1-yl]-1-thiophène-2-ylméthyl-éthyl]-amide d'acide 5-méthoxy-benzofuranne-2-carboxylique ;
{2-oxo-2-[6-oxo-4-(2-pyridine-3-ylacétyl)-hexahydro-pyrrolo[3,2-b]pyrrole-1-yl]-1-thiophène-2-ylméthyl-éthyl]-amide d'acide thiéno[3,2-b]thiophène-2-carboxylique ;
{2-oxo-2-[6-oxo-4-(2-pyridine-3-ylacétyl)-hexahydro-pyrrolo[3,2-b]pyrrole-1-yl]-1-thiophène-2-ylméthyl-éthyl]-amide d'acide 3-mêthyl-benzofuranne-2-carboxylique ;
N-{2-oxo-2-[6-oxo-4-(2-pyridine-3-ylacétyl)-hexahydro-pyrrolo[3,2-b]pyrrole-1-yl]-1-thiophène-2-ylméthyl-éthyl]-4-phénoxy-benzamide ;
N-{2-oxo-2-[6-oxo-4-(2-pyridine-3-ylacétyl)-hexahydro-pyrrolo[3,2-b]pyrrole-1-yl]-1-thiophène-2-ylméthyl-éthyl]-4-thiophène-2-yl-benzamide ;

4-tertiobutyl-N-{1-[6-oxo-4-(pyridine-2-ylméthanesulfonyl)-hexahydro-pyrrolo[3,2-b]pyrrole-1-carbonyl]-cyclohexyl}-benzamide ;
{1-[6-oxo-4-(pyridine-2-ylméthanesulfonyl)-hexahydro-pyrrolo[3,2-b]pyrrole-1-carbonyl]-cyclohexyl}-amide d'acide 5-méthoxy-benzofuranne-2-carboxylique ;
{1-[6-oxo-4-(pyridine-2-ylméthanesulfonyl)-hexahydro-pyrrolo[3,2-b]pyrrole-1-carbonyl]-cyclohexyl}-amide d'acide thiéno[3,2-b]thiophène-2-carboxylique ;
{1-[6-oxo-4-(pyridine-2-ylméthanesulfonyl)-hexahydro-pyrrolo[3,2-b]pyrrole-1-carbonyl]-cyclohexyl}-amide d'acide 3-méthyl-benzofuranne-2-carboxylique ;
N-{1-[6-oxo-4-(pyridine-2-ylméthanesulfonyl)-hexahydro-pyrrolo[3,2-b]pyrrole-1-carbonyl]-cyclohexyl}-4-phénoxy-benzamide ;
N-{1-[6-oxo-4-(pyridine-2-ylméthanesulfonyl)-hexahydro-pyrrolo[3,2-b]pyrrole-1-carbonyl]-cyclohexyl}-4-thiophène-2-yl-benzamide ;
4-tertiobutyl-N-{1-cyclopropylméthyl-2-oxo-2-[6-oxo-4-(pyridine-2-ylméthanesulfonyl)-hexahydro-pyrrolo[3,2-b]-pyrrole-1-yl] -éthyl}-benzamide ;
{1-cyclopropylméthyl-2-oxo-2-[6-oxo-4-(pyridine-2-ylméthanesulfonyl)-hexahydro-pyrrolo[3,2-b]-pyrrole-1-yl]-éthyl}-amide d'acide 5-méthoxy-benzofuranne-2-carboxylique ;
{1-cyclopropylméthyl-2-oxo-2-[6-oxo-4-(pyridine-2-ylméthanesulfonyl)-hexahydro-pyrrolo[3,2-b]-pyrrole-1-yl]-éthyl}-amide d'acide thiéno[3,2-b]thiophène-2-carboxylique ;
{1-cyclopropylméthyl-2-oxo-2-[6-oxo-4-(pyridine-2-ylméthanesulfonyl)-hexahydro-pyrrolo[3,2-b]-pyrrole-1-yl]-éthyl}-amide d'acide 3-mtéhyl-benzofuranne-2-carboxylique ;
N-{1-cyclopropylméthyl-2-oxo-2-[6-oxo-4-(pyridine-2-ylméthanesulfonyl)-hexahydro-pyrrolo[3,2-b]-pyrrole-1-yl]-éthyl}-4-phénoxy-benzamide ;
N-{1-cyclopropylméthyl-2-oxo-2-[6-oxo-4-(pyridine-2-ylméthanesulfonyl)-hexahydro-pyrrolo[3,2-b]-pyrrole-1-yl]-éthyl}-4-thiophène-2-yl-benzamide ;
4-tertiobutyl-N-{2-[6-oxo-4-(pyridine-2-ylméthanesulfonyl)-hexahydro-pyrrolo[3,2-b]pyrrole-1-yl]-1-thiophène-2-ylméthyléthyl}-benzamide ;
{2-oxo-[6-oxo-4-(pyridine-2-ylméthanesulfonyl)-hexahydro-pyrrolo[3,2-b]pyrrole-1-yl]-1-thiophène-2-ylméthyléthyl}-amide d'acide 5-méthoxy-benzofuranne-2-carboxylique ;
{2-oxo-[6-oxo-4-(pyridine-2-ylméthanesulfonyl)-hexahydro-pyrrolo[3,2-b]pyrrole-1-yl]-1-thiophène-2-ylméthyléthyl}-amide d'acide thiéno[3,2-b]thiophène-2-carboxylique ;
{2-oxo-2-[6-oxo-4-(pyridine-2-ylméthanesulfonyl)-hexahydro-pyrrolo[3,2-b]pyrrole-1-yl]-1-thiophène-2-ylmêthyléthyl}-amide d'acide 3-méthyl-benzofuranne-2-carboxylique ;
N-{2-oxo-2-[6-oxo-4-(pyridine-2-ylméthanesulfonyl)-hexahydro-pyrrolo[3,2-b]pyrrole-1-yl]-1-thiophène-2-ylméthyléthyll-4-phénoxy-benzamide ;
N-{2-oxo-[6-oxo-4-(pyridine-2-ylméthanesulfonyl)-hexahydro-pyrrolo[3,2-b]pyrrole-1-yl]-1-thiophène-2-ylméthyléthyl}-4-thiophène-2-yl-benzamide ;
4-tertiobutyl-N-{1-[6-oxo-4-(pyridine-3-ylméthanesulfonyl)-hexahydro-pyrrolo[3,2-b]pyrrole-1-carbonyl]-cyclohexyl}-benzamide ;
{1-[6-oxo-4-(pyridine-3-ylméthanesulfonyl)-hexahydro-pyrrolo[3,2-b]pyrrole-1-carbonyl]-cyclohexyl}-amide d'acide 5-méthoxy-benzofuranne-2-carboxylique ;
{1-[6-oxo-4-(pyridine-3-ylméthanesulfonyl)-hexahydro-pyrrolo[3,2-b]pyrrole-1-carbonyl]-cyclohexyl}-amide d'acide thiéno[3,2-b]thiophène-2-carboxylique ;
{1-[6-oxo-4-(pyridine-3-ylméthanesulfonyl)-hexahydro-pyrrolo[3,2-b]pyrrole-1-carbonyl]-cyclohexyl}-amide d'acide 3-méthyl-benzofuranne-2-carboxylique ;
N-{1-[6-oxo-4-(pyridine-3-ylméthanesulfonyl)-hexahydro-pyrrolo[3,2-b]pyrrole-1-carbonyl]-cyclohexyl}-4-phénoxy-benzamide ;
N-{1-[6-oxo-4-(pyridine-3-ylméthanesulfonyl)-hexahydro-pyrrolo[3,2-b]pyrrole-1-carbonyl]-cyclohexyl}-4-thiophène-2-yl-benzamide ;
4-tertiobutyl-N-{1-cyclopropylméthyl-2-oxo-2-[6-oxo-4-(pyridine-3-ylméthanesulfonyl)-hexahydro-pyrrolo[3,2-b]-pyrrole-1-yll-éthyll-benzamide ;
{1-cyclopropylméthyl-2-oxo-2-[6-oxo-4-(pyridine-3-ylméthanesulfonyl)-hexahydro-pyrrolo[3,2-b]-pyrrole-1-yl]-éthyl}-amide d'acide 5-méthoxybenzofuranne-2-carboxylique ;
{1-cyclopropylméthyl-2-oxo-2-[6-oxo-4-(pyridine-3-ylméthanesulfonyl)-hexahydro-pyrrolo[3,2-b]-pyrrole-1-yl]-éthyl}-amide d'acide thiéno[3,2-b]thiophène-2-carboxylique ;
{1-cyclopropylméthyl-2-oxo-2-[6-oxo-4-(pyridine-3-ylméthanesulfonyl)-hexahydro-pyrrolo[3,2-b]-pyrrole-1-yl]-éthyl}-amide d'acide 3-méthyl-benzofuranne-2-carboxylique ;
N-{1-cyclopropylméthyl-2-oxo-2-[6-oxo-4-(pyridine-3-ylméthanesulfonyl)-hexahydro-pyrrolo[3,2-b]-pyrrole-1-yl]-éthyl}-4-phénoxy-benzamide ;

N-{1-cyclopropylméthyl-2-oxo-2-[6-oxo-4-(pyridine-3-ylméthanesulfonyl)-hexahydro-pyrrolo[3,2-b]-pyrrole-1-yl]-éthyl}-4-thiophène-2-yl-benzamide ;
4-tertiobutyl-N-{2-oxo-2-[6-oxo-4-(pyridine-3-ylméthane-sulfonyl)-hexahydro-pyrrolo[3,2-b]pyrrole-1-yl]-1-thiophène-2-ylméthyléthyl}-benzamide ;
{2-oxo-2-[6-oxo-4-(pyridine-3-ylméthane-sulfonyl)-hexahydro-pyrrolo[3,2-b]pyrrole-1-yl]-1-thiophène-2-ylméthyléthyl}-amide d'acide 5-méthoxy-benzofuranne-2-carboxylique ;
{2-oxo-2-[6-oxo-4-(pyridine-3-ylméthane-sulfonyl)-hexahydro-pyrrolo[3,2-b]pyrrole-1-yl]-1-thiophène-2-ylméthyléthyl}-amide d'acide thiéno[3,2-b]thiophène-2-carboxylique ;
{2-oxo-2-[6-oxo-4-(pyridine-3-ylméthane-sulfonyl)-hexahydro-pyrrolo[3,2-b]pyrrole-1-yl]-1-thiophène-2-ylméthyléthyl}-amide d'acide 3-méthyl-benzofuranne-2-carboxylique ;
N-{2-oxo-2-[6-oxo-4-(pyridine-3-ylméthane-sulfonyl)-hexahydro-pyrrolo[3,2-b]pyrrole-1-yl]-1-thiophène-2-ylméthyléthyl}-4-phénoxy-benzamide ;
N-{2-oxo-2-[6-oxo-4-(pyridine-3-ylméthane-sulfonyl)-hexahydro-pyrrolo[3,2-b]pyrrole-1-yl]-1-thiophène-2-ylméthyléthyl}-4-thiophène-2-yl-benzamide ;
4-tertiobutyl-N-{1-[6-oxo-4-(1-oxy-pyridine-2-ylméthane-sulfonyl)-hexahydro-pyrrolo[3,2-b]pyrrole-1-carbonyl]-cyclohexyl}-benzamide ;
{1-[6-oxo-4-(1-oxy-pyridine-2-ylméthane-sulfonyl)-hexahydro-pyrrolo[3,2-b]pyrrole-1-carbonyl]-cyclohexyl}-amide d'acide 5-méthoxy-benzofuranne-2-carboxylique ;
{1-[6-oxo-4-(1-oxy-pyridine-2-ylméthane-sulfonyl)-hexahydro-pyrrolo[3,2-b]pyrrole-1-carbonyl]-cyclohexyl}-amide d'acide thiéno[3,2-b]thiophène-2-carboxylique ;
{1-[6-oxo-4-(1-oxy-pyridine-2-ylméthane-sulfonyl)-hexahydro-pyrrolo[3,2-b]pyrrole-1-carbonyll-cyclohexyl}-amide d'acide 3-méthyl-benzofuranne-2-carboxylique ;
N-{1-[6-oxo-4-(1-oxy-pyridine-2-ylméthane-sulfonyl)-hexahydro-pyrrolo[3,2-b]pyrrole-1-carbonyl]-cyclohexyl}-4-phénoxy-benzamide ;
N-{1-[6-oxo-4-(1-oxy-pyridine-2-ylméthane-sulfonyl)-hexahydro-pyrrolo[3,2-b]pyrrole-1-carbonyl]-cyclohexyl}-4-t hiophène-2-yl-benzamide ;
4-tertiobutyl-N-{1-cyclopropylméthyl-2-oxo-2-[6-oxo-4-(1-oxy-pyridine-2-ylméthanesulfonyl)-hexahydro-pyrrolo[3,2-b]-pyrrole-1-yl]-éthyl}-benzamide ;
{1-cyclopropylméthyl-2-oxo-2-[6-oxo-4-(1-oxy-pyridine-2-ylméthanesulfonyl)-hexahydro-pyrrolo[3,2-b]-pyrrole-1-yl]-éthyl}-amide d'acide 5-méthoxy-benzofuranne-2-carboxylique ;
{1-cyclopropylméthyl-2-oxo-2-[6-oxo-4-(1-oxy-pyridine-2-ylméthanesulfonyl)-hexahydro-pyrrolo[3,2-b]-pyrrole-1-yl]-éthyl}-amide d'acide thiéno[3,2-b]thiophène-2-carboxylique ;
{1-cyclopropylméthyl-2-oxo-2-[6-oxo-4-(1-oxy-pyridine-2-ylméthanesulfonyl)-hexahydro-pyrrolo[3,2-b]-pyrrole-1-yl]-éthyl}-amide d'acide 3-mêthyl-benzofuranne-2-carboxylique ; N-{1-cyclopropylméthyl-2-oxo-2-[6-oxo-4-(1-oxy-pyridine-2-ylméthanesulfonyl)-hexahydro-pyrrolo[3,2-b]-pyrrole-1-yl]-éthyl}-4-phénoxy-benzamide ;
N-{1-cyclopropylméthyl-2-oxo-2-[6-oxo-4-(1-oxy-pyridine-2-ylméthanesulfonyl)-hexahydro-pyrrolo[3,2-b]-pyrrole-1-yl]-éthyl}-4-thiophène-2-yl-benzamide ;
4-tertiobutyl-N-{2-oxo-2-[6-oxo-4-(1-oxy-pyridine-2-ylméthanesulfonyl)-hexahydro-pyrrolo[3,2-b]pyrrole-1-yl]-1-thiophène-2-ylméthyléthyl}-benzamide ;
{2-oxo-2-[6-oxo-4-(1-oxy-pyridine-2-ylméthanesulfonyl)-hexahydro-pyrrolo[3,2-b]pyrrole-1-yl]-1-thiophène-2-ylméthyléthyl}-amide d'acide 5-méthoxy-benzofuranne-2-carboxylique ;
{2-oxo-2-[6-oxo-4-(1-oxy-pyridine-2-ylméthanesulfonyl)-hexahydro-pyrrolo[3,2-b]pyrrole-1-yl]-1-thiophène-2-ylméthyléthyl}-amide d'acide thiéno[3,2-b]thiophène-2-carboxylique ;
{2-oxo-2-[6-oxo-4-(1-oxy-pyridine-2-ylméthanesulfonyl)-hexahydro-pyrrolo[3,2-b]pyrrole-1-yl]-1-thiophène-2-ylméthyléthyl}-amide d'acide 3-méthyl-benzofuranne-2-carboxylique ;
N-{2-oxo-2-[6-oxo-4-(1-oxy-pyridine-2-ylméthanesulfonyl)-hexahydro-pyrrolo[3,2-b]pyrrole-1-yl]-1-thiophène-2-ylméthyléthyl}-4-phénoxy-benzamide ;
N-{2-oxo-2-[6-oxo-4-(1-oxy-pyridine-2-ylméthanesulfonyl)-hexahydro-pyrrolo[3,2-b]pyrrole-1-yl]-1-thiophène-2-ylméthyléthyl}-4-thiophène-2-yl-benzamide ;
4-tertiobutyl-N-{1-[6-oxo-4-(1-oxypyridine-3-ylméthane-sulfonyl)-hexahydro-pyrrolo[3,2-b]pyrrole-1-carbonyl]-cyclohexyl}-benzamide ;
{1-[6-oxo-4-(1-oxypyridine-3-ylméthane-sulfonyl)-hexahydro-pyrrolo[3,2-b]pyrrole-1-carbonyl]-cyclohexyl}-amide d'acide 5-méthoxy-benzofuranne-2-carboxylique ;
{1-[6-oxo-4-(1-oxypyridine-3-ylméthane-sulfonyl)-hexahydro-pyrrolo[3,2-b]pyrrole-1-carbonyl]-cyclohexyl}-amide d'acide thiéno[3,2-b]thiophène-2-carboxylique ;
{1-[6-oxo-4-(1-oxypyridine-3-ylméthane-sulfonyl)-hexahydro-pyrrolo[3,2-b]pyrrole-1-carbonyl]-cyclohexyl}-amide d'acide 3-méthyl-benzofuranne-2-carboxylique ;
N-{1-[6-oxo-4-(1-oxypyridine-3-ylméthane-sulfonyl)-hexahydro-pyrrolo[3,2-b]pyrrole-1-carbonyl]-cyclohexyl}-4-phénoxy-benzamide ;
N-{1-[6-oxo-4-(1-oxypyridine-3-ylméthane-sulfonyl)-hexahydro-pyrrolo[3,2-b]pyrrole-1-carbonyl]-cyclohexyl}-4-thiophène-2-yl-benzamide ;
4-tertiobutyl-N-{1-cyclopropylméthyl-2-oxo-2-[6-oxo-4-(1-oxy-pyridine-3-ylméthanesulfonyl)-hexahydro-pyrrolo-[3,2-b]-pyrrole-1-yll-éthyl}-benzamide ;
{1-cyclopropylméthyl-2-oxo-2-[6-oxo-4-(1-oxy-pyridine-3-ylméthanesulfonyl)-hexahydro-pyrrolo[3,2-b]pyrrole-1-yl]-éthyl}-amide d'acide 5-méthoxy-benzofuranne-2-carboxylique ;
{1-cyclopropylméthyl-2-oxo-2-[6-oxo-4-(1-oxy-pyridine-3-ylméthanesulfonyl)-hexahydro-pyrrolo[3,2-b]pyrrole-1-yl]-éthyl}-amide d'acide thiéno[3,2-b]thiophène-2-carboxylique ;
{1-cyclopropylméthyl-2-oxo-2-[6-oxo-4-(1-oxy-pyridine-3-ylméthanesulfonyl)-hexahydro-pyrrolo[3,2-b]pyrrole-1-yl]-éthyl}-amide d'acide 3-méthyl-benzofuranne-2-carboxylique ;
N-{1-cyclopropylméthyl-2-oxo-2-[6-oxo-4-(1-oxy-pyridine-3-ylméthanesulfonyl)-hexahydro-pyrrolo[3,2-b]pyrrole-1-yl]-éthyl}-4-phénoxy-benzamide ;
N-{1-cyclopropylméthyl-2-oxo-2-[6-oxo-4-(1-oxy-pyridine-3-ylméthanesulfonyl)-hexahydro-pyrrolo[3,2-b]pyrrole-1-yl]-éthyl}-4-thiophène-2-yl-benzamide ;
4-tertiobutyl-N-{2-oxo-2-[6-oxo-4-(1-oxy-pyridine-3-yl-méthanesulfonyl)-hexahydro-pyrrolo[3,2-b]pyrrole-1-yl]-1-thiophène-2-ylméthyl-éthyl}-benzamide ;
{2-oxo-2-[6-oxo-4-(1-oxy-pyridine-3-yl-méthanesulfonyl)-hexahydro-pyrrolo[3,2-b]pyrrole-1-yl]-1-thiophène-2-ylméthyl-éthyl}-amide d'acide 5-méthoxy-benzofuranne-2-carboxylique ;
{2-oxo-2-[6-oxo-4-(1-oxy-pyridine-3-yl-méthanesulfonyl)-hexahydro-pyrrolo[3,2-b]pyrrole-1-yl]-1-thiophêne-2-ylméthyl-éthyl}-amide d'acide thiéno[3,2-b]thiophène-2-carboxylique ;
{2-oxo-2-[6-oxo-4-(1-oxy-pyridine-3-yl-méthanesulfonyl)-hexahydro-pyrrolo[3,2-b]pyrrole-1-yl]-1-thiophène-2-ylméthyl-éthyl}-amide d'acide 3-méthyl-benzofuranne-2-carboxylique ;
N-{2-oxo-2-[6-oxo-4-(1-oxy-pyridine-3-yl-mêthanesulfonyl)-hexahydro-pyrrolo[3,2-b]pyrrole-1-yl]-1-thiophène-2-ylméthyl-éthyl}-4-phénoxy-benzamide ;
N-{2-oxo-2-[6-oxo-4-(1-oxy-pyridine-3-yl-méthanesulfonyl)-hexahydro-pyrrolo[3,2-b]pyrrole-1-yl]-1-thiophène-2-ylméthyl-éthyl}-4-thiophène-2-yl-benzamide ;
2-isobutyl-4-morpholine-4-yl-1-[6-oxo-4-(pyridine-2-sulfonyl)-hexahydro-pyrrolo[3,2-b]pyrrole-1-yl]-butane-1,4-dione ;
2-isobutyl-1-[6-oxo-4-(pyridine-2-sulfonyl)-hexahydro-pyrrolo[3,2-b]pyrrole-1-yl]-4-pipérazine-1-yl-butane-1,4-dione ;
2-isobutyl-4-(4-méthyl-pipérazine-1-yl)-1-[6-oxo-4-(pyridine-2-sulfonyl)-hexahydro-pyrrolo[3,2-b]pyrrole-1-yll-butane-1,4-dione ;
2-isobutyl-1-[6-oxo-4-(pyridine-2-sulfonyl)-hexahydro-pyrrolo[3,2-b]pyrrole-1-yl]-4-(4-phényl-pipérazine-1-yl)-butane-1,4-dione ;
2-isobutyl-1-[6-oxo-4-(pyridine-2-sulfonyl)-hexahydro-pyrrolo[3,2-b]pyrrole-1-yl]-4-(3,4,4a,8a-tétrahydro-1H-isoquinoléine-2-yl)-butane-1,4-dione ;
2-isobutyl-1-[6-oxo-4-(pyridine-2-sulfonyl)-hexahydro-pyrrolo[3,2-b]pyrrole-1-yl]-4-(1,3,3a,7a-tétrahydro-iso-indole-2-yl)-butane-1,4-dione ;
4-(4-benzyl-pipérazine-1-yl)-2-isobutyl-1-[6-oxo-4-(pyridine-2-sulfonyl)-hexahydro-pyrrolo[3,2-b]pyrrole-1-yl]-butane-1,4-dione ;
2-isobutyl-4-morpholine-4-yl-1-[6-oxo-4-(pyridine-3-sulfonyl)-hexahydro-pyrrolo[3,2-b]pyrrole-1-yl]-butane-1,4-dione ;
2-isobutyl-1-[6-oxo-4-(pyridine-3-sulfonyl)-hexahydro-pyrrolo[3,2-b]pyrrole-1-yl]-4-pipérazine-1-yl-butane-1,4-dione ;
2-isobutyl-4-(4-méthyl-pipérazine-1-yl)-1-[6-oxo-4-(pyridine-2-sulfonyl)-hexahydro-pyrrolo[3,2-b]pyrrole-1-yll-butane-1,4-dione ;
2-isobutyl-1-[6-oxo-4-(pyridine-3-sulfonyl)-hexahydro-pyrrolo[3,2-b]pyrrole-1-yl]-4-(4-phényl-pipérazine-1-yl)-butane-1,4-dione ;
2-isobutyl-1-[6-oxo-4-(pyridine-3-sulfonyl)-hexahydro-pyrrolo[3,2-b]pyrrole-1-yl]-4-(3,4,4a,8a-tétrahydro-1H-isoquinoléine-2-yl)-butane-1,4-dione ;
2-isobutyl-1-[6-oxo-4-(pyridine-3-sulfonyl)-hexahydro-pyrrolo[3,2-b]pyrrole-1-yl]-4-(1,3,3a,7a-tétrahydro-iso-indole-2-yl)-butane-1,4-dione ;
4-(4-benzyl-pipérazine-1-yl)-2-isobutyl-1-[6-oxo-4-(pyridine-3-sulfonyl)-hexahydro-pyrrolo[3,2-b]pyrrole-1-yll-butane-1,4-dione ;
2-isobutyl-4-morpholine-4-yl-1-[6-oxo-4-(1-oxy-pyridine-2-sulfonyl)-hexahydro-pyrrolo[3,2-b]pyrrole-1-yl]-butane-1,4-dione ;
2-isobutyl-1-[6-oxo-4-(1-oxy-pyridine-2-sulfonyl)-hexahydro-pyrrolo[3,2-b]pyrrole-1-yl]-4-pipérazine-1-yl-butane-1,4-dione ;
2-isobutyl-4-(4-méthyl-pipérazine-1-yl)-1-[6-oxo-4-(1-oxy-pyridine-2-sulfonyl)-hexahydro-pyrrolo[3,2-b]pyrrole-1-yl]-butane-1,4-dione ;
2-isobutyl-1-[6-oxo-4-(1-oxy-pyridine-2-sulfonyl)-hexahydro-pyrrolo[3,2-b]pyrrole-1-yl]-4-(4-phényl-pipérazine-1-yl)-butane-1,4-dione ;
2-isobutyl-1-[6-oxo-4-(1-oxy-pyridine-2-sulfonyl)-hexahydro-pyrrolo[3,2-b]pyrrole-1-yl]-4-(3,4,4a,8a-tétrahydro-1H-isoquinoléine-2-yl)-butane-1,4-dione ;
2-isobutyl-1-[6-oxo-4-(1-oxy-pyridine-2-sulfonyl)-hexahydro-pyrrolo[3,2-b]pyrrole-1-yl]-4-(1,3,3a,7a-tétrahydro-iso-indole-2-yl)-butane-1,4-dione ;
4-(4-benzyl-pipérazine-1-yl)-2-isobutyl-1-[6-oxo-4-(1-oxy-pyridine-2-sulfonyl)-hexahydro-pyrrolo[3,2-b]pyrrole-1-yl]-butane-1,4-dione ;
2-isobutyl-4-morpholine-4-yl-1-[6-oxo-4-(1-oxy-pyridine-3-sulfonyl)-hexahydro-pyrrolo[3,2-b]pyrrole-1-yl]-butane-1,4-dione ;
2-isobutyl-1-[6-oxo-4-(1-oxy-pyridine-3-sulfonyl)-hexahydro-pyrrolo[3,2-b]pyrrole-1-yl]-4-pipérazine-1-yl-butane-1,4-dione ;
2-isobutyl-4-(4-méthyl-pipérazine-1-yl)-1-[6-oxo-4-(1-oxy-pyridine-3-sulfonyl)-hexahydro-pyrrolo[3,2-b]pyrrole-1-yl]-butane-1,4-dione ;
2-isobutyl-1-[6-oxo-4-(1-oxy-pyridine-3-sulfonyl)-hexahydro-pyrrolo[3,2-b]pyrrole-1-yl]-4-(4-phényl-pipérazine-1-yl)-butane-1,4-dione ;
2-isobutyl-1-[6-oxo-4-(1-oxy-pyridine-3-sulfonyl)-hexahydro-pyrrolo[3,2-b]pyrrole-1-yl]-4-(3,4,4a,8a-tétrahydro-1H-isoquinoléine-2-yl)-butane-1,4-dione ;
2-isobutyl-1-[6-oxo-4-(1-oxy-pyridine-3-sulfonyl)-hexahydro-pyrrolo[3,2-b]pyrrole-1-yl]-4-(1,3,3a,7a-tétrahydro-iso-indole-2-yl)-butane-1,4-dione ;
4-(4-benzyl-pipérazine-1-yl)-2-isobutyl-1-[6-oxo-4-(1-oxy-pyridine-3-sulfonyl)-hexahydro-pyrrolo[3,2-b]pyrrole-1-yl]-butane-1,4-dione ;
2-isobutyl-4-morpholine-4-yl-1-[6-oxo-4-(1-oxy-pyridine-2-carbonyl)-hexahydro-pyrrolo[3,2-b]pyrrole-1-yl]-butane-1,4-dione ;
2-isobutyl-1-[6-oxo-4-(pyridine-2-carbonyl)-hexahydro-pyrrolo[3,2-b]pyrrole-1-yl]-4-pipérazine-1-yl-butane-1,4-dione ;
2-isobutyl-4-(4-méthyl-pipérazine-1-yl)-1-[6-oxo-4-(pyridine-2-carbonyl)-hexahydro-pyrrolo[3,2-b]pyrrole-1-yll-butane-1,4-dione ;
2-isobutyl-1-[6-oxo-4-(pyridine-2-carbonyl)-hexahydro-pyrrolo[3,2-b]pyrrole-1-yl]-4-(4-phényl-pipérazine-1-yl)-butane-1,4-dione ;
2-isobutyl-1-[6-oxo-4-(pyridine-2-carbonyl)-hexahydro-pyrrolo[3,2-b]pyrrole-1-yl]-4-(3,4,4a,8a-tétrahydro-1H-isoquinoléine-2-yl)-butane-1,4-dione ;
2-isobutyl-1-[6-oxo-4-(pyridine-2-carbonyl)-hexahydro-pyrrolo[3,2-b]pyrrole-1-yl]-4-(1,3,3a,7a-tétrahydro-iso-indole-2-yl)-butane-1,4-dione ;
4-(4-benzyl-pipérazine-1-yl)-2-isobutyl-1-[6-oxo-4-(pyridine-2-carbonyl)-hexahydro-pyrrolo[3,2-b]pyrrole-1-yl]-butane-1,4-dione ;
2-isobutyl-4-morpholine-4-yl-1-[6-oxo-4-(2-pyridine-3-yl-acétyl)-hexahydro-pyrrolo[3,2-b]pyrrole-1-yl]-butane-1,4-dione ;
2-isobutyl-1-[6-oxo-4-(2-pyridine-3-yl-acétyl)-hexahydro-pyrrolo[3,2-b]pyrrole-1-yl]-4-pipérazine-1-yl-butane-1,4-dione ;
2-isobutyl-4-(4-méthyl-pipérazine-1-yl)-1-[6-oxo-4-(2-pyridine-3-yl-acétyl)-hexahydro-pyrrolo[3,2-b]pyrrole-1-yl]-butane-1,4-dione ;
2-isobutyl-1-[6-oxo-4-(2-pyridine-3-yl-acétyl)-hexahydro-pyrrolo[3,2-b]pyrrole-1-yl]-4-(4-phényl-pipérazine-1-yl)-butane-1,4-dione ;
2-isobutyl-1-[6-oxo-4-(2-pyridine-3-yl-acétyl)-hexahydro-pyrrolo[3,2-b]pyrrole-1-yl]-4-(3,4,4a,8a-tétrahydro-1H-isoquinoléine-2-yl)-butane-1,4-dione ;
2-isobutyl-1-[6-oxo-4-(2-pyridine-3-yl-acétyl)-hexahydro-pyrrolo[3,2-b]pyrrole-1-yl]-4-(1,3,3a,7a-tétrahydro-iso-indole-2-yl)-butane-1,4-dione ;
4-(4-benzyl-pipérazine-1-yl)-2-isobutyl-1-[6-oxo-4-(2-pyridine-3-yl-acétyl)-hexahydro-pyrrolo[3,2-b]pyrrole-1-yll-butane-1,4-dione ;
2-isobutyl-4-morpholine-4-yl-1-[6-oxo-4-(pyridine-3-yl-méthanesulfonyl)-hexahydro-pyrrolo[3,2-b]pyrrole-1-yl]-butane-1,4-dione ;
2-isobutyl-1-[6-oxo-4-(pyridine-2-ylméthanesulfonyl)-hexahydro-pyrrolo[3,2-b]pyrrole-1-yl]-4-pipérazine-1-yl-butane-1,4-dione ;
2-isobutyl-4-(4-méthyl-pipérazine-1-yl)-1-[6-oxo-4-(pyridine-2-yl-méthanesulfonyl)-hexahydro-pyrrolo[3,2-b]-

pyrrole-1-yl]-butane-1,4-dione ;
2-isobutyl-1-[6-oxo-4-(pyridine-2-yl-méthanesulfonyl)-hexahydro-pyrrolo[3,2-b]pyrrole-1-yl]-4-(4-phényl-pipérazine-1-yl)-butane-1,4-dione ;
2-isobutyl-1-[6-oxo-4-(pyridine-2-yl-méthanesulfonyl)-hexahydro-pyrrolo[3,2-b]pyrrole-1-yl]-4-(3,4,4a,8a-tétrahydro-1H-isoquinoléine-2-yl)-butane-1,4-dione ;
2-isobutyl-1-[6-oxo-4-(pyridine-2-yl-méthanesulfonyl)-hexahydro-pyrrolo[3,2-b]pyrrole-1-yl]-4-(1,3,3a,7a-tétrahydro-iso-indole-2-yl)-butane-1,4-dione ;
4-(4-benzyl-pipérazine-1-yl)-2-isobutyl-1-[6-oxo-4-(pyridine-2-ylméthanesulfonyl)-hexahydro-pyrrolo[3,2-b]-pyrrole-1-yl]-butane-1,4-dione ;
2-isobutyl-4-morpholine-4-yl-1-[6-oxo-4-(pyridine-3-yl-méthanesulfonyl)-hexahydro-pyrrolo[3,2-b]pyrrole-1-yl]-butane-1,4-dione ;
2-isobutyl-1-[6-oxo-4-(pyridine-3-ylméthanesulfonyl)-hexahydro-pyrrolo[3,2-b]pyrrole-1-yl]-4-pipérazine-1-yl-butane-1,4-dione ;
2-isobutyl-4-(4-méthyl-pipérazine-1-yl)-1-[6-oxo-4-(pyridine-3-ylméthanesulfonyl)-hexahydro-pyrrolo[3,2-b]-pyrrole-1-yll-butane-1,4-dione ;
2-isobutyl-1-[6-oxo-4-(pyridine-3-ylméthanesulfonyl)-hexahydro-pyrrolo[3,2-b]pyrrole-1-yl]-4-(4-phényl-pipérazine-1-yl)-butane-1,4-dione ;
2-isobutyl-1-[6-oxo-4-(pyridine-3-ylméthanesulfonyl)-hexahydro-pyrrolo[3,2-b]pyrrole-1-yl]-4-(3,4,4a,8a-tétrahyàro-1H-isoquinoléine-2-yl)-butane-1,4-dione ;
2-isobutyl-1-[6-oxo-4-(pyridine-3-ylméthanesulfonyl)-hexahydro-pyrrolo[3,2-b]pyrrole-1-yl]-4-(1,3,3a,7a-tétrahydro-iso-indole-2-yl)-butane-1,4-dione ;
4-(4-benzyl-pipérazine-1-yl)-2-isobutyl-1-[6-oxo-4-(pyridine-3-ylméthanesulfonyl)-hexahydro-pyrrolo[3,2-b]-pyrrole -1-yll-butane-1,4-dione ;
2-isobutyl-4-morpholine-4-yl-1-[6-oxo-4-(pyridine-2-yl-méthanesulfonyl)-hexahydro-pyrrolo[3,2-b]pyrrole-1-yl]-butane-1,4-dione ;
2-isobutyl-1-[6-oxo-4-(1-oxy-pyridine-2-ylméthanesulfonyl)-hexahydro-pyrrolo[3,2-b]pyrrole-1-yl]-4-pipérazine-1-yl-butane-1,4-dione ;
2-isobutyl-4-(4-méthyl-pipérazine-1-yl)-1-[6-oxo-4-(1-oxy-pyridine-2-ylméthanesulfonyl)-hexahydro-pyrrolo[3,2-b]-pyrrole-1-yl]-butane-1,4-dione ;
2-isobutyl-1-[6-oxo-4-(1-oxy-pyridine-2-ylméthanesulfonyl)-hexahydro-pyrrolo[3,2-b]pyrrole-1-yl]-4-(4-phényl-pipérazine-1-yl)-butane-1,4-dione ;
2-isobutyl-1-[6-oxo-4-(1-oxy-pyridine-2-ylméthanesulfonyl)-hexahydro-pyrrolo[3,2-b]pyrrole-1-yl]-4-(3,4,4a,8a-tétrahydro-1H-isoquinoléine-2-yl)-butane-1,4-dione ;
2-isobutyl-1-[6-oxo-4-(1-oxy-pyridine-2-ylméthanesulfonyl)-hexahydro-pyrrolo[3,2-b]pyrrole-1-yl]-4-(1,3,3a,7a-tétrahydro-iso-indole-2-yl)-butane-1,4-dione ;
4-(4-benzyl-pipérazine-1-yl)-2-isobutyl-1-[6-oxo-4-(1-oxy-pyridine-2-ylméthanesulfonyl)-hexahydro-pyrrolo[3,2-b]-pyrrole-1-yl]-butane-1,4-dione ;
2-isobutyl-4-morpholine-4-yl-1-[6-oxo-4-(1-oxy-pyridine-3-yl-méthanesulfonyl)-hexahydro-pyrrolo[3,2-b]pyrrole-1-yl]-butane-1,4-dione ;
2-isobutyl-1-[6-oxo-4-(1-oxy-pyridine-3-ylméthanesulfonyl)-hexahydro-pyrrolo[3,2-b]pyrrole-1-yl]-4-pipérazine-1-yl-butane-1,4-dione ;
2-isobutyl-4-(4-méthyl-pipérazine-1-yl)-1-[6-oxo-4-(1-oxy-pyridine-3-ylméthanesulfonyl)-hexahydro-pyrrolo[3,2-b]-pyrrole-1-yl]-butane-1,4-dione ;
2-isobutyl-1-[6-oxo-4-(1-oxy-pyridine-3-ylméthanesulfonyl)-hexahydro-pyrrolo[3,2-b]pyrrole-1-yl]-4-(4-phényl-pipérazine-1-yl)-butane-1,4-dione ;
2-isobutyl-1-[6-oxo-4-(1-oxy-pyridine-3-ylméthanesulfonyl)-hexahydro-pyrrolo[3,2-b]pyrrole-1-yl]-4-(3,4,4a,8a-tétrahydro-1H-isoquinoléine-2-yl)-butane-1,4-dione ;
2-isobutyl-1-[6-oxo-4-(1-oxy-pyridine-3-ylméthanesulfonyl)-hexahydro-pyrrolo[3,2-b]pyrrole-1-yl]-4-(1,3,3a,7a-tétrahydro-iso-indole-2-yl)-butane-1,4-dione ;
4-(4-benzyl-pipérazine-1-yl)-2-isobutyl-1-[6-oxo-4-(1-oxy-pyridine-3-ylméthanesulfonyl)-hexahydro-pyrrolo[3,2-b]-pyrrole-1-yl]-butane-1,4-dione ;
4-(2-biphényl-3-yl-4-méthyl-pentanoyl)-1-(pyridine-2-sulfonyl)-hexahydro-pyrrolo[3,2-b]pyrrole-3-one ;
4-(2-biphényl-3-yl-4-méthyl-pentanoyl)-1-(pyridine-3-sulfonyl)-hexahydro-pyrrolo[3,2-b]pyrrole-3-one ;
4-(2-biphényl-3-yl-4-méthyl-pentanoyl)-1-(1-oxy-pyridine-2-sulfonyl)-hexahydro-pyrrolo[3,2-b]pyrrole-3-one ;
4-(2-biphényl-3-yl-4-méthyl-pentanoyl)-1-(1-oxy-pyridine-3-sulfonyl)-hexahydro-pyrrolo[3,2-b]pyrrole-3-one ;
4-(2-biphényl-3-yl-4-méthyl-pentanoyl)-1-(pyridine-2-carbonyl)-hexahydro-pyrrolo[3,2-b]pyrrole-3-one ;
4-(2-biphényl-3-yl-4-méthyl-pentanoyl)-1-(2-pyridine-3-yl-acétyl)-hexahydro-pyrrolo[3,2-b]pyrrole-3-one ;
4-(2-biphényl-3-yl-4-méthyl-pentanoyl)-1-(pyridine-2-yl-méthanesulfonyl)-hexahydro-pyrrolo[3,2-b]pyrrole-3-one ;

4-(2-biphényl-3-yl-4-méthyl-pentanoyl)-1-(pyridine-3-yl-méthanesulfonyl)-hexahydro-pyrrolo[3,2-b]pyrrole-3-one ;
4-(2-biphényl-3-yl-4-méthyl-pentanoyl)-1-(1-oxy-pyridine-2-yl-méthanesulfonyl)-hexahydro-pyrrolo[3,2-b]pyrrole-3-one ;
4-(2-biphényl-3-yl-4-méthyl-pentanoyl)-1-(1-oxy-pyridine-3-yl-méthanesulfonyl)-hexahydro-pyrrolo[3,2-b]pyrrole-3-one ;
4-[4-méthyl-2-(3-pyridine-2-y1-phény1)-pentanoy1]-1-(pyridine-2-sulfonyl)-hexahydro-pyrrolo[3,2-b]pyrrole-3-one ;
4-[4-méthyl-2-(3-pyridine-2-yl-phényl)-pentanoyl]-1-(pyridine-3-sulfonyl)-hexahydro-pyrrolo[3,2-b]pyrrole-3-one ;
4-[4-méthyl-2-(3-pyridine-2-yl-phényl)-pentanoyl]-1-(1-oxy-pyridine-2-sulfonyl)-hexahydro-pyrrolo[3,2-b]pyrrole-3-one ;
4-[4-méthyl-2-(3-pyridine-2-yl-phényl)-pentanoyl]-1-(1-oxy-pyridine-3-sulfonyl)-hexahydro-pyrrolo[3,2-b]pyrrole-3-one ;
4-[4-méthyl-2-(3-pyridine-2-yl-phényl)-pentanoyl]-1-(pyridine-2-carbonyl)-hexahydro-pyrrolo[3,2-b]pyrrole-3-one ;
4-[4-méthyl-2-(3-pyridine-2-yl-phényl)-pentanoyl]-1-(2-pyridine-3-yl-acétyl)-hexahydro-pyrrolo[3,2-b]pyrrole-3-one ;
4-[4-méthyl-2-(3-pyridine-2-yl-phényl)-pentanoyl]-1-(pyridine-2-ylméthanesulfonyl)-hexahydro-pyrrolo[3,2-b]-pyrrole-3-one ;
4-[4-méthyl-2-(3-pyridine-2-yl-phényl)-pentanoyl]-1-(pyridine-3-ylméthanesulfonyl)-hexahydro-pyrrolo[3,2-b]-pyrrole-3-one ;
4-[4-méthyl-2-(3-pyridine-2-yl-phényl)-pentanoyl]-1-(1-oxy-pyridine-2-ylméthanesulfonyl)-hexahydro-pyrrolo[3,2-b]-pyrrole-3-one ;
4-[4-méthyl-2-(3-pyridine-2-yl-phényl)-pentanoyl]-1-(1-oxy-pyridine-3-ylméthanesulfonyl)-hexahydro-pyrrolo[3,2-b]-pyrrole-3-one ;
4-[4-méthyl-2-(3-pyridine-3-yl-phényl)-pentanoyl]-1-(pyridine-2-sulfonyl)-hexahydro-pyrrolo[3,2-b]pyrrole-3-one ;
4-[4-méthyl-2-(3-pyridine-3-yl-phényl)-pentanoyl]-1-(pyridine-3-sulfonyl)-hexahydro-pyrrolo[3,2-b]pyrrole-3-one ;
4-[4-méthyl-2-(3-pyridine-3-yl-phényl)-pentanoyl]-1-(1-oxy-pyridine-2-sulfonyl)-hexahydro-pyrrolo[3,2-b]pyrrole-3-one ;
4-[4-méthyl-2-(3-pyridine-3-yl-phényl)-pentanoyl]-1-(1-oxy-pyridine-3-sulfonyl)-hexahydro-pyrrolo[3,2-b]pyrrole-3-one ;
4-[4-méthyl-2-(3-pyridine-3-yl-phényl)-pentanoyl]-1-(pyridine-2-carbonyl)-hexahydro-pyrrolo[3,2-b]pyrrole-3-one ;
4-[4-méthyl-2-(3-pyridine-3-yl-phényl)-pentanoyl]-1-(2-pyridine-3-yl-acétyl)-hexahydro-pyrrolo[3,2-b]pyrrole-3-one ;
4-[4-méthyl-2-(3-pyridine-3-yl-phényl)-pentanoyl]-1-(pyridine-2-ylméthanesulfonyl)-hexahydro-pyrrolo[3,2-b]-pyrrole-3-one ;
4-[4-méthyl-2-(3-pyridine-3-yl-phényl)-pentanoyl]-1-(pyridine-3-ylméthanesulfonyl)-hexahydro-pyrrolo[3,2-b]pyrrole-3-one ;
4-[4-méthyl-2-(3-pyridine-3-yl-phényl)-pentanoyl]-1-(1-oxy-pyridine-2-ylméthanesulfonyl)-hexahydro-pyrrolo[3,2-b]-pyrrole-3-one ;
4-[4-méthyl-2-(3-pyridine-3-yl-phényl)-pentanoyl]-1-(1-oxy-pyridine-3-ylméthanesulfonyl)-hexahydro-pyrrolo[3,2-b] pyrrole-3-one ;
4-[4-méthyl-2-(3-pyridine-4-yl-phényl)-pentanoyl]-1-(pyridine-2-sulfonyl)-hexahydro-pyrrolo[3,2-b]pyrrole-3-one ;
4-[4-méthyl-2-(3-pyridine-4-yl-phényl)-pentanoyl]-1-(pyridine-3-sulfonyl)-hexahydro-pyrrolo[3,2-b]pyrrole-3-one ;
4-[4-méthyl-2-(3-pyridine-4-yl-phényl)-pentanoyl]-1-(1-oxy-pyridine-2-sulfonyl)-hexahydro-pyrrolo[3,2-b]pyrrole-3-one ;
4-[4-méthyl-2-(3-pyridine-4-yl-phényl)-pentanoyl]-1-(1-oxy-pyridine-3-sulfonyl)-hexahydro-pyrrolo[3,2-b]pyrrole-3-one ;
4-[4-méthyl-2-(3-pyridine-4-yl-phényl)pentanoyl]-1-(pyridine-2-carbonyl)-hexahydro-pyrrolo[3,2-b]pyrrole-3-one ;
4-[4-méthyl-2-(3-pyridine-4-yl-phényl)-pentanoyl]-1-(2-pyridine-3-yl-acétyl)-hexahydro-pyrrolo[3,2-b]pyrrole-3-one ;
4-[4-méthyl-2-(3-pyridine-4-yl-phényl)-pentanoyl]-1-(pyridine-2-ylméthanesulfonyl)-hexahydro-pyrrolo[3,2-b]pyr role-3-one ;
4-[4-méthyl-2-(3-pyridine-4-yl-phényl)-pentanoyl]-1-(pyridine-3-ylméthanesulfonyl)-hexahydro-pyrrolo[3,2-b]-pyrrole-3-one ;
4-[4-méthyl-2-(3-pyridine-4-yl-phényl)-pentanoyl]-1-(1-oxy-pyridine-2-ylméthanesulfonyl)-hexahydro-pyrrolo[3,2-b]-pyrrole-3-one ;
4-[4-méthyl-2-(3-pyridine-4-yl-phényl)-pentanoyl]-1-(1-oxy-pyridine-3-ylméthanesulfonyl)-hexahydro-pyrrolo[3,2-b]-pyrrole-3-one ;
{3,3-diméthyl-1-[6-oxo-4-(pyridine-2-sulfonyl)-hexahydro-pyrrolo[3,2-b]pyrrole-1-carbonyl]-butyl}-amide d'acide

furanne-3-carboxylique ;
{3,3-diméthyl-1-[6-oxo-4-(pyridine-2-sulfonyl)-hexahydro-pyrrolo[3,2-b]pyrrole-1-carbonyl]-butyl}-amide d'acide thiophène-3-carboxylique ;
{3,3-diméthyl-1-[6-oxo-4-(pyridine-2-sulfonyl)-hexahydro-pyrrolo[3,2-b]pyrrole-1-carbonyl]-butyl}-amide d'acide benzo[b]thiophène-3-carboxylique ;
{1-cyclohexylméthyl-2-oxo-2-[6-oxo-4-(pyridine-2-sulfonyl)-hexahydro-pyrrolo[3,2-b]pyrrole-1-yl]-éthyl}-amide d'acide furanne-3-carboxylique ;
{1-cyclohexylméthyl-2-oxo-2-[6-oxo-4-(pyridine-2-sulfonyl)-hexahydro-pyrrolo[3,2-b]pyrrole-1-yl]-éthyl}-amide d'acide thiophène-3-carboxylique ;
{1-cyclohexylméthyl-2-oxo-2-[6-oxo-4-(pyridine-2-sulfonyl)-hexahydro-pyrrolo[3,2-b]pyrrole-1-yl]-éthyl}-amide d'acide benzo[b]thiophène-3-carboxylique ;
{1-(4-hydroxy-benzyl)-2-oxo-2-[6-oxo-4-(pyridine-2-sulfonyl)-hexahydro-pyrrolo[3,2-b]pyrrole-1-yl]-éthyl}-amide d'acide naphtalène-1-carboxylique ;
{1-(4-hydroxy-benzyl)-2-oxo-2-[6-oxo-4-(pyridine-2-sulfonyl)-hexahydro-pyrrolo[3,2-b]pyrrole-1-yl]-éthyl}-amide d'acide quinoléine-8-carboxylique ;
4-tertiobutyl-N-{1-(4-hydroxy-benzyl)-2-oxo-2-(6-oxo-4-(pyridine-2-sulfonyl)-hexahydro-pyrrolo[3,2-b]pyrrole-1-yl]-éthyl}-benzamide ;
4-tertiobutyl-N-{1-(4-fluoro-benzyl)-2-oxo-2-[6-oxo-4-(pyridine-2-sulfonyl)-hexahydro-pyrrolo[3,2-b]pyrrole-1-yl]-éthyl}-benzamide ;
4-tertiobutyl-N-{1-(4-méthoxy-benzyl)-2-oxo-2-[6-oxo-4-(pyridine-2-sulfonyl)-hexahydro-pyrrolo[3,2-b]pyrrole-1-yl]-éthyl}-benzamide ;
4-tertiobutyl-N-{1-(3-hydroxy-benzyl)-2-oxo-2-[6-oxo-4-(pyridine-2-sulfonyl)-hexahydro-pyrrolo[3,2-b]pyrrole-1-yl]-éthyl}-benzamide ;
{1-(4-hydroxy-benzyl)-2-oxo-2-[6-oxo-4-(pyridine-2-sulfonyl)-hexahydro-pyrrolo[3,2-b]pyrrole-1-yl]-éthyl}-amide d'acide biphényl-4-carboxylique ;
{1-(4-fluoro-benzyl)-2-oxo-2-[6-oxo-4-(pyridine-2-sulfonyl)-hexahydro-pyrrolo[3,2-b]pyrrole-1-yl]-éthyl}-amide d'acide biphényl-4-carboxylique ;
{1-(4-méthoxy-benzyl)-2-oxo-2-[6-oxo-4-(pyridine-2-sulfonyl)-hexahydro-pyrrolo[3,2-b]pyrrole-1-yl]-éthyl}-amide d'acide biphényl-4-carboxylique ;
{1-(3-hydroxy-benzyl)-2-oxo-2-[6-oxo-4-(pyridine-2-sulfonyl)-hexahydro-pyrrolo[3,2-b]pyrrole-1-yl]-éthyl}-amide d'acide biphényl-4-carboxylique ;
{3,3-diméthyl-1-[6-oxo-4-(pyridine-3-sulfonyl)-hexahydro-pyrrolo[3,2-b]pyrrole-1-carbonyl]-butyl}-amide d'acide furanne-3-carboxylique ;
{3,3-diméthyl-1-[6-oxo-4-(pyridine-3-sulfonyl)-hexahydro-pyrrolo[3,2-b]pyrrole-1-carbonyl]-butyl}-amide d'acide thiophène-3-carboxylique ;
{3,3-diméthyl-1-[6-oxo-4-(pyridine-3-sulfonyl)-hexahydro-pyrrolo[3,2-b]pyrrole-1-carbonyl]-butyl}-amide d'acide benzo[b]thiophène-3-carboxylique ;
{1-cyclohexylméthyl-2-oxo-2-[6-oxo-4-(pyridine-3-sulfonyl)-hexahydro-pyrrolo[3,2-b]pyrrole-1-yl]-éthyl}-amide d'acide furanne-3-carboxylique ;
{1-cyclohexylméthyl-2-oxo-2-[6-oxo-4-(pyridine-3-sulfonyl)-hexahydro-pyrrolo[3,2-b]pyrrole-1-yl]-éthyl}-amide d'acide thiophène-3-carboxylique ;
{1-cyclohexylméthyl-2-oxo-2-[6-oxo-4-(pyridine-3-sulfonyl)-hexahydro-pyrrolo[3,2-b]pyrrole-1-yl]-éthyl}-amide d'acide benzo[b]thiophène-3-carboxylique ;
{1-(4-hydroxy-benzyl)-2-oxo-2-[6-oxo-4-(pyridine-3-sulfonyl)-hexahydro-pyrrolo[3,2-b]pyrrole-1-yl]-éthyl}-amide d'acide naphtalène-1-carboxylique ;
{1-(4-hydroxy-benzyl)-2-oxo-2-[6-oxo-4-(pyridine-3-sulfonyl)-hexahydro-pyrrolo[3,2-b]pyrrole-1-yl]-éthyl}-amide d'acide quinoléine-8-carboxylique ;
4-tertiobutyl-N-{1-(4-hydroxy-benzyl)-2-oxo-2-[6-oxo-4-(pyridine-3-sulfonyl)-hexahydro-pyrrolo[3,2-b]pyrrole-1-yl]-éthyl}-benzamide ;
4-tertiobutyl-N-{1-(4-fluoro-benzyl)-2-oxo-2-[6-oxo-4-(pyridine-3-sulfonyl)-hexahydro-pyrrolo[3,2-b]pyrrole-1-yl]-ét hyl}-benzamide ;
4-tertiobutyl-N-{1-(4-méthoxy-benzyl)-2-oxo-2-[6-oxo-4-(pyridine-3-sulfonyl)-hexahydro-pyrrolo[3,2-b]pyrrole-1-yl]-éthyl}-benzamide ;
4-tertiobutyl-N-{1-(3-hydroxy-benzyl)-2-oxo-2-[6-oxo-4-(pyridine-3-sulfonyl)-hexahydro-pyrrolo[3,2-b]pyrrole-1-yl]-éthyl}-benzamide ;
{1-(4-hydroxy-benzyl)-2-oxo-2-[6-oxo-4-(pyridine-3-sulfonyl)-hexahydro-pyrrolo[3,2-b]pyrrole-1-yl]-éthyl}-amide d'acide biphényl-4-carboxylique ;
{1-(4-fluoro-benzyl)-2-oxo-2-[6-oxo-4-(pyridine-3-sulfonyl)-hexahydro-pyrrolo[3,2-b]pyrrole-1-yl]-éthyl}-amide

d'acide biphényl-4-carboxylique ;
{1-(4-méthoxy-benzyl)-2-oxo-2-[6-oxo-4-(pyridine-3-sulfonyl)-hexahydro-pyrrolo[3,2-b]pyrrole-1-yl]-éthyl}-amide d'acide biphényl-4-carboxylique ;
{1-(3-hydroxy-benzyl)-2-oxo-2-[6-oxo-4-(pyridine-3-sulfonyl)-hexahydro-pyrrolo[3,2-b]pyrrole-1-yl]-éthyl}-amide d'acide biphényl-4-carboxylique ;
{3,3-diméthyl-1-[6-oxo-4-(1-oxy-pyridine-2-sulfonyl)-hexahydro-pyrrolo[3,2-b]pyrrole-1-carbonyl]-butyl}-amide d'acide furanne-3-carboxylique ;
{3,3-diméthyl-1-[6-oxo-4-(1-oxy-pyridine-2-sulfonyl)-hexahydro-pyrrolo[3,2-b]pyrrole-1-carbonyl]-butyl}-amide d'acide thiophène-3-carboxylique ;
{3,3-diméthyl-1-[6-oxo-4-(1-oxy-pyridine-2-sulfonyl)-hexahydro-pyrrolo[3,2-b]pyrrole-1-carbonyl]-butyl}-amide d'acide benzo[b]thiophène-3-carboxylique ;
{1-cyclohexylméthyl-2-oxo-2-[6-oxo-4-(1-oxy-pyridine-2-sulfonyl)-hexahydro-pyrrolo[3,2-b]pyrrole-1-yl]-éthyl}-amide d'acide furanne-3-carboxylique ;
{1-cyclohexylméthyl-2-oxo-2-[6-oxo-4-(1-oxy-pyridine-2-sulfonyl)-hexahydro-pyrrolo[3,2-b]pyrrole-1-yl]-éthyl}-amide d'acide thiophène-3-carboxylique ;
{1-cyclohexylméthyl-2-oxo-2-[6-oxo-4-(1-oxy-pyridine-2-sulfonyl)-hexahydro-pyrrolo[3,2-b]pyrrole-1-yl]-éthyl}-amide d'acide benzo[b]thiophène-3-carboxylique ;
{1-(4-hydroxy-benzyl)-2-oxo-2-[6-oxo-4-(1-oxy-pyridine-2-sulfonyl)-hexahydro-pyrrolo[3,2-b]pyrrole-l-yl]-éthyl}-amide d'acide naphtalène-1-carboxylique ;
{1-(4-hydroxy-benzyl)-2-oxo-2-[6-oxo-4-(1-oxy-pyridine-2-sulfonyl)-hexahydro-pyrrolo[3,2-b]pyrrole-1-yl]-éthyl}-amide d'acide quinoléine-8-carboxylique ;
4-tertiobutyl-N-{1-(4-hydroxy-benzyl)-2-oxo-2-[6-oxo-4-(1-oxy-pyridine-2-sulfonyl)-hexahydro-pyrrolo[3,2-b]pyrrole-1-yl]-éthyl}-benzamide ;
4-tertiobutyl-N-{1-(4-fluoro-benzyl)-2-oxo-2-[6-oxo-4-(1-oxy-pyridine-2-sulfonyl)-hexahydro-pyrrolo[3,2-b]pyrrole-1-yl]-éthyl}-benzamide ;
4-tertiobutyl-N-{1-(4-méthoxy-benzyl)-2-oxo-2-[6-oxo-4-(1-oxy-pyridine-2-sulfonyl)-hexahydro-pyrrolo[3,2-b]pyrrole-1-yl]-éthyl}-benzamide ;
4-tertiobutyl-N-{1-(3-hydroxy-benzyl)-2-oxo-2-[6-oxo-4-(1-oxy-pyridine-2-sulfonyl)-hexahydro-pyrrolo[3,2-b]pyrrole-1-yl]-éthyl}-benzamide ;
{1-(4-hydroxy-benzyl)-2-oxo-2-[6-oxo-4-(1-oxy-pyridine-2-sulfonyl)-hexahydro-pyrrolo[3,2-b]pyrrole-1-yl]-éthyl}-amide d'acide biphényl-4-carboxylique ;
{1-(4-fluoro-benzyl)-2-oxo-2-[6-oxo-4-(1-oxy-pyridine-2-sulfonyl)-hexahydro-pyrrolo[3,2-b]pyrrole-1-yl]-éthyl}-amide d'acide biphényl-4-carboxylique ;
{1-(4-méthoxy-benzyl)-2-oxo-2-[6-oxo-4-(1-oxy-pyridine-2-sulfonyl)-hexahydro-pyrrolo[3,2-b]pyrrole-1-yl]-éthyl}-amide d'acide biphényl-4-carboxylique ;
{1-(3-hydroxy-benzyl)-2-oxo-2-[6-oxo-4-(1-oxy-pyridine-2-sulfonyl)-hexahydro-pyrrolo[3,2-b]pyrrole-1-yl]-éthyl}-amide d'acide biphényl-4-carboxylique ;
{3,3-diméthyl-1-[6-oxo-4-(1-oxy-pyridine-3-sulfonyl)-hexahydro-pyrrolo[3,2-b]pyrrole-1-carbonyl]-butyl}-amide d'acide furanne-3-carboxylique ;
{3,3-diméthyl-1-[6-oxo-4-(1-oxy-pyridine-3-sulfonyl)-hexahydro-pyrrolo[3,2-b]pyrrole-1-carbonyl]-butyl}-amide d'acide thiophène-3-carboxylique ;
{3,3-diméthyl-1-[6-oxo-4-(1-oxy-pyridine-3-sulfonyl)-hexahydro-pyrrolo[3,2-b]pyrrole-1-carbonyl]-butyl}-amide d'acide benzo[b]thiophène-3-carboxylique ;
{1-cyclohexylméthyl-2-oxo-2-[6-oxo-4-(1-oxy-pyridine-3-sulfonyl)-hexahydro-pyrrolo[3,2-b]pyrrole-1-yl]-éthyl}-amide d'acide furanne-3-carboxylique ;
{1-cyclohexylméthyl-2-oxo-2-[6-oxo-4-(1-oxy-pyridine-3-sulfonyl)-hexahydro-pyrrolo[3,2-b]pyrrole-1-yl]-éthyl}-amide d'acide thiophène-3-carboxylique ;
{1-cyclohexylméthyl-2-oxo-2-[6-oxo-4-(1-oxy-pyridine-3-sulfonyl)-hexahydro-pyrrolo[3,2-b]pyrrole-1-yl]-éthyl}-amide d'acide benzo[b]thiophène-3-carboxylique ;
{1-(4-hydroxy-benzyl)-2-oxo-2-[6-oxo-4-(1-oxy-pyridine-3-sulfonyl)-hexahydro-pyrrolo[3,2-b]pyrrole-1-yl]-éthyl}-amide d'acide naphtalène-1-carboxylique ;
{1-(4-hydroxy-benzyl)-2-oxo-2-[6-oxo-4-(1-oxy-pyridine-3-sulfonyl)-hexahydro-pyrrolo[3,2-b]pyrrole-1-yl]-éthyl}-amide d'acide quinoléine-8-carboxylique ;
4-tertiobutyl-N-{1-(4-hydroxy-benzyl)-2-oxo-2-[6-oxo-4-(1-oxy-pyridine-3-sulfonyl)-hexahydro-pyrrolo[3,2-b]pyrrole-1-yl]-éthyl}-benzamide ;
4-tertiobutyl-N-{1-(4-fluoro-benzyl)-2-oxo-2-[6-oxo-4-(1-oxy-pyridine-3-sulfonyl)-hexahydro-pyrrolo[3,2-b]pyrrole-1-yl]-éthyl}-benzamide ;
4-tertiobutyl-N-{1-(4-méthoxy-benzyl)-2-oxo-2-[6-oxo-4-(1-oxy-pyridine-3-sulfonyl)-hexahydro-pyrrolo[3,2-b]-

pyrrole-1-yl]-éthyl}-benzamide ;
4-tertiobutyl-N-{1-(3-hydroxy-benzyl)-2-oxo-2-[6-oxo-4-(1-oxy-pyridine-3-sulfonyl)-hexahydro-pyrrolo[3,2-b]pyrrole-1-yl]-éthyl}-benzamide ;
{1-(4-hydroxy-benzyl)-2-oxo-2-[6-oxo-4-(1-oxy-pyridine-3-sulfonyl)-hexahydro-pyrrolo[3,2-b]pyrrole-1-yl]-éthyl}-amide d'acide biphényl-4-carboxylique ;
{1-(4-fluoro-benzyl)-2-oxo-2-[6-oxo-4-(1-oxy-pyridine-3-sulfonyl)-hexahydro-pyrrolo[3,2-b]pyrrole-1-yl]-éthyl}-amide d'acide biphényl-4-carboxylique ;
{1-(4-méthoxy-benzyl)-2-oxo-2-[6-oxo-4-(1-oxy-pyridine-3-sulfonyl)-hexahydro-pyrrolo[3,2-b]pyrrole-1-yl]-éthyl}-amide d'acide biphényl-4-carboxylique ;
{1-(3-hydroxy-benzyl)-2-oxo-2-[6-oxo-4-(1-oxy-pyridine-3-sulfonyl)-hexahydro-pyrrolo[3,2-b]pyrrole-1-yl]-éthyl}-amide d'acide biphényl-4-carboxylique ;
{3,3-diméthyl-1-[6-oxo-4-(pyridine-2-carbonyl)-hexahydro-pyrrolo[3,2-b]pyrrole-1-carbonyl]-butyl}-amide d'acide furanne-3-carboxylique ;
{3,3-diméthyl-1-[6-oxo-4-(pyridine-2-carbonyl)-hexahydro-pyrrolo[3,2-b]pyrrole-1-carbonyl]-butyl}-amide d'acide thiophène-3-carboxylique ;
{3,3-diméthyl-1-[6-oxo-4-(pyridine-2-carbonyl)-hexahydro-pyrrolo[3,2-b]pyrrole-1-carbonyl]-butyl}-amide d'acide benzo[b]thiophène-3-carboxylique ;
{1-cyclohexylméthyl-2-oxo-2-[6-oxo-4-(pyridine-2-carbonyl)-hexahydro-pyrrolo[3,2-b]pyrrole-1-yl]-éthyl}-amide d'acide furanne-3-carboxylique ;
{1-cyclohexylméthyl-2-oxo-2-[6-oxo-4-(pyridine-2-carbonyl)-hexahydro-pyrrolo[3,2-b]pyrrole-1-yl]-éthyl}-amide d'acide thiophène-3-carboxylique ;
{1-cyclohexylméthyl-2-oxo-2-[6-oxo-4-(pyridine-2-carbonyl)-hexahydro-pyrrolo[3,2-b]pyrrole-1-yl]-éthyl}-amide d'acide benzo[b]thiophène-3-carboxylique ;
{1-(4-hydroxy-benzyl)-2-oxo-2-[6-oxo-4-(pyridine-2-carbonyl)-hexahydro-pyrrolo[3,2-b]pyrrole-1-yl]-éthyl}-amide d'acide naphtalène-1-carboxylique ;
{1-(4-hydroxy-benzyl)-2-oxo-2-[6-oxo-4-(pyridine-2-carbonyl)-hexahydro-pyrrolo[3,2-b]pyrrole-1-yl]-éthyl}-amide d'acide quinoléine-8-carboxylique ;
4-tertiobutyl-N-{1-(4-hydroxy-benzyl)-2-oxo-2-[6-oxo-4-(pyridine-2-carbonyl)-hexahydro-pyrrolo[3,2-b]pyrrole-1-yl]-éthyl}-benzamide ;
4-tertiobutyl-N-{1-(4-fluoro-benzyl)-2-oxo-2-[6-oxo-4-(pyridine-2-carbonyl)-hexahydro-pyrrolo[3,2-b]pyrrole-1-yl]-éthyl}-benzamide ;
4-tertiobutyl-N-{1-(4-méthoxy-benzyl)-2-oxo-2-[6-oxo-4-(pyridine-2-carbonyl)-hexahydro-pyrrolo[3,2-b]pyrrole-1-yl]-éthyl}-benzamide ;
4-tertiobutyl-N-{1-(3-hydroxy-benzyl)-2-oxo-2-[6-oxo-4-(pyridine-2-carbonyl)-hexahydro-pyrrolo[3,2-b]pyrrole-1-yl]-éthyl}-benzamide ;
{1-(4-hydroxy-benzyl)-2-oxo-2-[6-oxo-4-(pyridine-2-carbonyl)-hexahydro-pyrrolo[3,2-b]pyrrole-1-yl]-éthyl}-amide d'acide biphényl-4-carboxylique ;
{1-(4-fluoro-benzyl)-2-oxo-2-[6-oxo-4-(pyridine-2-carbonyl)-hexahydro-pyrrolo[3,2-b]pyrrole-1-yl]-éthyl}-amide d'acide biphényl-4-carboxylique ;
{1-(4-méthoxy-benzyl)-2-oxo-2-[6-oxo-4-(pyridine-2-carbonyl)-hexahydro-pyrrolo[3,2-b]pyrrole-1-yl]-éthyl}-amide d'acide biphényl-4-carboxylique ;
{1-(3-hydroxy-benzyl)-2-oxo-2-[6-oxo-4-(pyridine-2-carbonyl)-hexahydro-pyrrolo[3,2-b]pyrrole-1-yl]-éthyl}-amide d'acide biphényl-4-carboxylique ;
{3,3-diméthyl-1-[6-oxo-4-(2-pyridine-3-yl-acétyl)-hexahydro-pyrrolo[3,2-b]pyrrole-1-carbonyl]-butyl}-amide d'acide furanne-3-carboxylique ;
{3,3-diméthyl-1-[6-oxo-4-(2-pyridine-3-yl-acétyl)-hexahydro-pyrrolo[3,2-b]pyrrole-1-carbonyl]-butyl}-amide d'acide thiophène-3-carboxylique ;
{3,3-diméthyl-1-[6-oxo-4-(2-pyridine-3-yl-acétyl)-hexahydro-pyrrolo[3,2-b]pyrrole-1-carbonyl]-butyl}-amide d'acide benzo[b]thiophène-3-carboxylique ;
{1-cyclohexylméthyl-2-oxo-2-[6-oxo-4-(2-pyridine-3-yl-acétyl)-hexahydro-pyrrolo[3,2-b]pyrrole-1-yl]-éthyl}-amide d'acide furanne-3-carboxylique ;
{1-cyclohexylméthyl-2-oxo-2-[6-oxo-4-(2-pyridine-3-yl-acétyl)-hexahydro-pyrrolo[3,2-b]pyrrole-1-yl]-éthyl}-amide d'acide thiophène-3-carboxylique ;
{1-cyclohexylméthyl-2-oxo-2-[6-oxo-4-(2-pyridine-3-yl-acétyl)-hexahydro-pyrrolo[3,2-b]pyrrole-1-yl]-éthyl}-amide d'acide benzo[b]thiophène-3-carboxylique ;
{1-(4-hydroxybenzyl)-2-oxo-2-[6-oxo-4-(2-pyridine-3-yl-acétyl)-hexahydro-pyrrolo[3,2-b]pyrrole-1-yl]-éthyl}-amide d'acide naphtalène-1-carboxylique ;
{1-(4-hydroxybenzyl)-2-oxo-2-[6-oxo-4-(2-pyridine-3-yl-acétyl)-hexahydro-pyrrolo[3,2-b]pyrrole-1-yl]-éthyl}-amide

d'acide quinoléine-8-carboxylique ;
4-tertiobutyl-N-{1-(4-hydroxy-benzyl)-2-oxo-2-[6-oxo-4-(2-pyridine-3-yl-acétyl)-hexahydro-pyrrolo[3,2-b]pyrrole-1-yl]-éthyl}-benzamide ;
4-tertiobutyl-N-{1-(4-fluoro-benzyl)-2-oxo-2-[6-oxo-4-(2-pyridine-3-yl-acétyl)-hexahydro-pyrrolo[3,2-b]pyrrole-1-yll-éthyl}-benzamide ;
4-tertiobutyl-N-{1-(4-méthoxy-benzyl)-2-oxo-2-[6-oxo-4-(2-pyridine-3-yl-acétyl)-hexahydro-pyrrolo[3,2-b]pyrrole-1-yll-éthyl}-benzamide ;
4-tertiobutyl-N-{1-(3-hydroxy-benzyl)-2-oxo-2-[6-oxo-4-(2-pyridine-3-yl-acétyl)-hexahydro-pyrrolo[3,2-b]pyrrole-1-yll-éthyl}-benzamide ;
{1-(4-hydroxy-benzyl)-2-oxo-2-[6-oxo-4-(2-pyridine-3-yl-acétyl)-hexahydro-pyrrolo[3,2-b]pyrrole-1-yl]-éthyl}-amide d'acide biphényl-4-carboxylique ;
{1-(4-fluoro-benzyl)-2-oxo-2-[6-oxo-4-(2-pyridine-3-yl-acétyl)-hexahydro-pyrrolo[3,2-b]pyrrole-1-yl]-éthyl}-amide d'acide biphényl-4-carboxylique ;
{1-(4-méthoxy-benzyl)-2-oxo-2-[6-oxo-4-(2-pyridine-3-yl-acétyl)-hexahydro-pyrrolo[3,2-b]pyrrole-1-yl]-éthyl}-amide d'acide biphényl-4-carboxylique ;
{1-(3-hydroxy-benzyl)-2-oxo-2-[6-oxo-4-(2-pyridine-3-yl-acétyl)-hexahydro-pyrrolo[3,2-b]pyrrole-1-yl]-éthyl}-amide d'acide biphényl-4-carboxylique ;
{3,3-diméthyl-1-[6-oxo-4-(pyridine-2-ylméthanesulfonyl)-hexahydro-pyrrolo[3,2-b]pyrrole-1-carbonyl]-butyl}-amide d'acide furanne 3-carboxylique ;
{3,3-diméthyl-1-[6-oxo-4-(pyridine-2-ylméthanesulfonyl)-hexahydro-pyrrolo[3,2-b]pyrrole-1-carbonyl]-butyl}-amide d'acide thiophène-3-carboxylique ;
{3,3-diméthyl-1-[6-oxo-4-(pyridine-2-ylméthanesulfonyl)-hexahydro-pyrrolo[3,2-b]pyrrole-1-carbonyl]-butyl}-amide d'acide benzo[b]thiophène-3-carboxylique ;
{1-cyclohexylméthyl-2-oxo-2-[6-oxo-4-(pyridine-2-ylméthanesulfonyl)-hexahydro-pyrrolo[3,2-b]pyrrole-1-yl]-éthyl}-amide d'acide furanne-3-carboxylique ;
{1-cyclohexylméthyl-2-oxo-2-[6-oxo-4-(pyridine-2-ylméthanesulfonyl)-hexahydro-pyrrolo[3,2-b]pyrrole-1-yl]-éthyl}-amide d'acide thiophène-3-carboxylique ;
{1-cyclohexylméthyl-2-oxo-2-[6-oxo-4-(pyridine-2-ylméthanesulfonyl)-hexahydro-pyrrolo[3,2-b]pyrrole-1-yl]-éthyl}-amide d'acide benzo[b]thiophène-3-carboxylique ;
{1-(4-hydroxy-benzyl)-2-oxo-2-[6-oxo-4-(pyridine-2-ylméthanesulfonyl)-hexahydro-pyrrolo[3,2-b]pyrrole-1-yl]-éthy1}-amide d'acide naphtalène-1-carboxylique ;
{1-(4-hydroxy-benzyl)-2-oxo-2-[6-oxo-4-(pyridine-2-ylméthanesulfonyl)-hexahydro-pyrrolo [3,2-b]pyrrole-1-yl]-éthyl}-amide d'acide quinoléine-8-carboxylique ;
4-tertiobutyl-N-{1-(4-hydroxy-benzyl)-2-oxo-2-[6-oxo-4-(pyridine-2-ylméthanesulfonyl)-hexahydro-pyrrolo[3,2-b]py rrole-1-yl]-éthyl}-benzamide ;
4-tertiobutyl-N-{1-(4-fluoro-benzyl)-2-oxo-2-[6-oxo-4-(pyridine-2-ylméthanesulfonyl)-hexahydro-pyrrolo[3,2-b]pyrro le-1-yl]-éthyl}-benzamide ;
4-tertiobutyl-N-{1-(4-méthoxy-benzyl)-2-oxo-2-[6-oxo-4-(pyridine-2-ylméthanesulfonyl)-hexahydro-pyrrolo[3,2-b]py rrole-1-yl]-éthyl}-benzamide ;
4-tertiobutyl-N-{1-(3-hydroxy-benzyl)-2-oxo-2-[6-oxo-4-(pyridine-2-ylméthanesulfonyl)-hexahydro-pyrrolo[3,2-b]py rrole-1-yl]-éthyl}-benzamide ;
{1-(4-hydroxy-benzyl)-2-oxo-2-[6-oxo-4-(pyridine-2-ylméthanesulfonyl)-hexahydro-pyrrolo[3,2-b]pyrrole-1-yl]-éthyl}-amide d'acide biphényl-4-carboxylique ;
{1-(4-fluoro-benzyl)-2-oxo-2-[6-oxo-4-(pyridine-2-ylméthanesulfonyl)-hexahydro-pyrrolo[3,2-b]pyrrole-1-yl]-éthyl}-amide d'acide biphényl-4-carboxylique ;
{1-(4-méthoxy-benzyl)-2-oxo-2-[6-oxo-4-(pyridine-2-ylméthanesulfonyl)-hexahydro-pyrrolo[3,2-b]pyrrole-1-yl]-éthyl}-amide d'acide biphényl-4-carboxylique ;
{1-(3-hydroxy-benzyl)-2-oxo-2-[6-oxo-4-(pyridine-2-ylméthanesulfonyl)-hexahydro-pyrrolo[3,2-b]pyrrole-1-yl]-éthyl}-amide d'acide biphényl-4-carboxylique ;
{3,3-diméthyl-1-[6-oxo-4-(pyridine-3-ylméthanesulfonyl)-hexahydro-pyrrolo[3,2-b]pyrrole-1-carbonyl]-butyl}-amide d'acide furanne-3-carboxylique ;
{3,3-diméthyl-1-[6-oxo-4-(pyridine-3-ylméthanesulfonyl)-hexahydro-pyrrolo[3,2-b]pyrrole-1-carbonyl]-butyl}-amide d'acide thiophène-3-carboxylique ;
{3,3-diméthyl-1-[6-oxo-4-(pyridine-3-ylméthanesulfonyl)-hexahydro-pyrrolo[3,2-b]pyrrole-1-carbonyl]-butyl}-amide d'acide benzo[b]thiophène-3-carboxylique ;
{1-cyclohexylméthyl-2-oxo-2-[6-oxo-4-(pyridine-3-ylméthanesulfonyl)-hexahydro-pyrrolo[3,2-b]pyrrole-1-yl]-éthyll-amide d'acide furanne-3-carboxylique ;
{1-cyclohexylméthyl-2-oxo-2-[6-oxo-4-(pyridine-3-ylméthanesulfonyl)-hexahydro-pyrrolo[3,2-b]pyrrole-1-yl]-éthyl}-amide d'acide thiophène-3-carboxylique ;
{1-cyclohexylméthyl-2-oxo-2-[6-oxo-4-(pyridine-3-ylméthanesulfonyl)-hexahydro-pyrrolo[3,2-b]pyrrole-1-yl]-éthyl}-amide d'acide benzo[b]thiophène-3-carboxylique ;
{1-(4-hydroxy-benzyl)-2-oxo-2-[6-oxo-4-(pyridine-3-ylméthanesulfonyl)-hexahydro-pyrrolo[3,2-b]pyrrole-1-yl]-éthyl}-amide d'acide naphtalène-1-carboxylique ;
{1-(4-hydroxy-benzyl)-2-oxo-2-[6-oxo-4-(pyridine-3-ylméthanesulfonyl)-hexahydro-pyrrolo[3,2-b]pyrrole-1-yl]-éthyl}-amide d'acide quinoléine-8-carboxylique ;
4-tertiobutyl-N-{1-(4-hydroxy-benzyl)-2-oxo-2-[6-oxo-4-(pyridine-3-ylméthanesulfonyl)-hexahydro-pyrrolo[3,2-b]pyrrole-1-yl]-éthyl}-benzamide ;
4-tertiobutyl-N-{1-(4-fluoro-benzyl)-2-oxo-2-[6-oxo-4-(pyridine-3-ylméthanesulfonyl)-hexahydro-pyrrolo[3,2-b]pyrrole-1-yl]-éthyl}-benzamide ;
4-tertiobutyl-N-{1-(4-méthoxy-benzyl)-2-oxo-2-[6-oxo-4-(pyridine-3-ylméthanesulfonyl)-hexahydro-pyrrolo[3,2-b]pyrrole-1-yl]-éthyl}-benzamide ;
4-tertiobutyl-N-{1-(3-hydroxy-benzyl)-2-oxo-2-[6-oxo-4-(pyridine-3-ylméthanesulfonyl)-hexahydro-pyrrolo[3,2-b]pyrrole-1-yl]-éthyl}-benzamide ;
{1-(4-hydroxy-benzyl)-2-oxo-2-[6-oxo-4-(pyridine-3-ylméthanesulfonyl)-hexahydro-pyrrolo[3,2-b]pyrrole-1-yl]-éthyl}-amide d'acide biphényl-4-carboxylique ;
{1-(4-fluoro-benzyl)-2-oxo-2-[6-oxo-4-(pyridine-3-ylméthanesulfonyl)-hexahydro-pyrrolo[3,2-b]pyrrole-1-yl]-éthyl}-amide d'acide biphényl-4-carboxylique ;
{1-(4-méthoxy-benzyl)-2-oxo-2-[6-oxo-4-(pyridine-3-ylméthanesulfonyl)-hexahydro-pyrrolo[3,2-b]pyrrole-1-yl]-éthyl}-amide d'acide biphényl-4-carboxylique ;
{1-(3-hydroxy-benzyl)-2-oxo-2-[6-oxo-4-(pyridine-3-yl-méthanesulfonyl)-hexahydro-pyrrolo[3,2-b]pyrrole-1-yl]-éthyl}-amide d'acide biphényl-4-carboxylique ;
{3,3-diméthyl-1-[6-oxo-4-(1-oxy-pyridine-2-ylméthane-sulfonyl)-hexahydro-pyrrolo[3,2-b]pyrrole-1-carbonyl]-butyl}-amide d'acide furanne-3-carboxylique ;
{3,3-diméthyl-1-[6-oxo-4-(1-oxy-pyridine-2-ylméthane-sulfonyl)-hexahydro-pyrrolo[3,2-b]pyrrole-1-carbonyl]-butyl}-amide d'acide thiophène-3-carboxylique ;
{3,3-diméthyl-1-[6-oxo-4-(1-oxy-pyridine-2-ylméthane-sulfonyl)-hexahydro-pyrrolo[3,2-b]pyrrole-1-carbonyl]-butyl}-amide d'acide benzo[b]thiophène-3-carboxylique ;
{1-cyclohexylméthyl-2-oxo-2-[6-oxo-4-(1-oxy-pyridine-2-ylméthane-sulfonyl)-hexahydro-pyrrolo[3,2-b]pyrrole-1-yl]-éthyl}-amide d'acide furanne-3-carboxylique ;
{1-cyclohexylméthyl-2-oxo-2-[6-oxo-4-(1-oxy-pyridine-2-ylméthane-sulfonyl)-hexahydro-pyrrolo[3,2-b]pyrrole-1-yl]-éthyl}-amide d'acide thiophène-3-carboxylique ;
{1-cyclohexylméthyl-2-oxo-2-[6-oxo-4-(1-oxy-pyridine-2-ylméthane-sulfonyl)-hexahydro-pyrrolo[3,2-b]pyrrole-1-yl]-éthyl}-amide d'acide benzo[b]thiophène-3-carboxylique ;
{1-(4-hydroxy-benzyl)-2-oxo-2-[6-oxo-4-(1-oxy-pyridine-2-ylméthane-sulfonyl)-hexahydro-pyrrolo[3,2-b]pyrrole-1-yl]-éthyl}-amide d'acide naphtalène-1-carboxylique ;
{1-(4-hydroxy-benzyl)-2-oxo-2-[6-oxo-4-(1-oxy-pyridine-2-ylméthane-sulfonyl)-hexahydro-pyrrolo[3,2-b]pyrrole-1-yl]-éthyl}-amide d'acide quinoléine-8-carboxylique ;
4-tertiobutyl-N-{1-(4-hydroxy-benzyl)-2-oxo-2-[6-oxo-4-(1-oxy-pyridine-2-ylméthane-sulfonyl)-hexahydro-pyrrolo-[3,2-b]pyrrole-1-yl]-éthyl}-benzamide ;
4-tertiobutyl-N-{1-(4-fluoro-benzyl)-2-oxo-2-[6-oxo-4-(1-oxy-pyridine-2-ylméthane-sulfonyl)-hexahydro-pyrrolo-[3,2-b]pyrrole-1-yl]-éthyl}-benzamide ;
4-tertiobutyl-N-{1-(4-méthoxy-benzyl)-2-oxo-2-[6-oxo-4-(1-oxy-pyridine-2-ylméthane-sulfonyl)-hexahydro-pyrrolo-[3,2-b]pyrrole-1-yl]-éthyl}-benzamide ;
4-tertiobutyl-N-{1-(3-hydroxy-benzyl)-2-oxo-2-[6-oxo-4-(1-oxy-pyridine-2-ylméthane-sulfonyl)-hexahydro-pyrrolo-[3,2-b]pyrrole-1-yl]-éthyl}-benzamide ;
{1-(4-hydroxy-benzyl)-2-oxo-2-[6-oxo-4-(1-oxy-pyridine-2-ylméthane-sulfonyl)-hexahydro-pyrrolo[3,2-b]pyrrole-1-yl]-éthyl}-amide d'acide biphényl-4-carboxylique ;
{1-(4-fluoro-benzyl)-2-oxo-2-[6-oxo-4-(1-oxy-pyridine-2-ylméthane-sulfonyl)-hexahydro-pyrrolo[3,2-b]pyrrole-1-yl]-éthyl}-amide d'acide biphényl-4-carboxylique ;
{1-(4-méthoxy-benzyl)-2-oxo-2-[6-oxo-4-(1-oxy-pyridine-2-ylméthane-sulfonyl)-hexahydro-pyrrolo[3,2-b]pyrrole-1-yl]-éthyl}-amide d'acide biphényl-4-carboxylique ;
{1-(3-hydroxy-benzyl)-2-oxo-2-[6-oxo-4-(1-oxy-pyridine-2-ylméthane-sulfonyl)-hexahydro-pyrrolo[3,2-b]pyrrole-1-yl]-éthyl}-amide d'acide biphényl-4-carboxylique ;
{3,3-diméthyl-1-[6-oxo-4-(1-oxy-pyridine-3-ylméthane-sulfonyl)-hexahydro-pyrrolo[3,2-b]pyrrole-1-carbonyl]-butyl}-amide d'acide furanne-3-carboxylique ;
{3,3-diméthyl-1-[6-oxo-4-(1-oxy-pyridine-3-ylméthane-sulfonyl)-hexahydro-pyrrolo[3,2-b]pyrrole-1-carbonyl]-butyl}-amide d'acide thiophène-3-carboxylique ;
{3,3-diméthyl-1-[6-oxo-4-(1-oxy-pyridine-3-ylméthane-sulfonyl)-hexahydro-pyrrolo[3,2-b]pyrrole-1-carbonyl]-butyl}-amide d'acide benzo[b]thiophène-3-carboxylique ;
{1-cyclohexylméthyl-2-oxo-2-[6-oxo-4-(1-oxy-pyridine-3-ylméthane-sulfonyl)-hexahydro-pyrrolo[3,2-b]pyrrole-1-yl]-éthyl}-amide d'acide furanne-3-carboxylique ;
{1-cyclohexylméthyl-2-oxo-2-[6-oxo-4-(1-oxy-pyridine-3-ylméthane-sulfonyl)-hexahydro-pyrrolo[3,2-b]pyrrole-1-yl]-éthyl}-amide d'acide thiophène-3-carboxylique ;
{1-cyclohexylméthyl-2-oxo-2-[6-oxo-4-(1-oxy-pyridine-3-ylméthane-sulfonyl)-hexahydro-pyrrolo[3,2-b]pyrrole-1-yl]-éthyl}-amide d'acide benzo[b]thiophène-3-carboxylique ;
{1-(4-hydroxy-benzyl)-2-oxo-2-[6-oxo-4-(1-oxy-pyridine-3-ylméthane-sulfonyl)-hexahydro-pyrrolo[3,2-b]pyrrole-1-yl]-éthyl}-amide d'acide naphtalène-1-carboxylique ;
{1-(4-hydroxy-benzyl)-2-oxo-2-[6-oxo-4-(1-oxy-pyridine-3-ylméthane-sulfonyl)-hexahydro-pyrrolo[3,2-b]pyrrole-1-yl]-éthyl}-amide d'acide quinoléine-8-carboxylique ;
4-tertiobutyl-N-{1-(4-hydroxy-benzyl)-2-oxo-2-[6-oxo-4-(1-oxy-pyridine-3-ylméthane-sulfonyl)-hexahydro-pyrrolo-[3,2-b]pyrrole-1-yl]-éthyl}-benzamide ;
4-tertiobutyl-N-{1-(4-fluoro-benzyl)-2-oxo-2-[6-oxo-4-(1-oxy-pyridine-3-ylméthane-sulfonyl)-hexahydro-pyrrolo[3,2-b]pyrrole-1-yl]-éthyl}-benzamide ;
4-tertiobutyl-N-{1-(4-méthoxy-benzyl)-2-oxo-2-[6-oxo-4-(1-oxy-pyridine-3-ylméthane-sulfonyl)-hexahydro-pyrrolo[3,2-b]pyrrole-1-yl]-éthyl}-benzamide ;
4-tertiobutyl-N-{1-(3-hydroxy-benzyl)-2-oxo-2-[6-oxo-4-(1-oxy-pyridine-3-ylméthane-sulfonyl)-hexahydro-pyrrolo[3,2-b]pyrrole-1-yl]-éthyl}-benzamide ;
{1-(4-hydroxy-benzyl)-2-oxo-2-[6-oxo-4-(1-oxy-pyridine-3-ylméthane-sulfonyl)-hexahydro-pyrrolo[3,2-b]pyrrole-1-yl]-éthyl}-amide d'acide biphényl-4-carboxylique ;
{1-(4-fluoro-benzyl)-2-oxo-2-[6-oxo-4-(1-oxy-pyridine-3-ylméthane-sulfonyl)-hexahydro-pyrrolo[3,2-b]pyrrole-1-yl]-éthyl}-amide d'acide biphényl-4-carboxylique ;
{1-(4-méthoxy-benzyl)-2-oxo-2-[6-oxo-4-(1-oxy-pyridine-3-ylméthane-sulfonyl)-hexahydro-pyrrolo[3,2-b]pyrrole-1-yl]-éthyl}-amide d'acide biphényl-4-carboxylique ;
{1-(3-hydroxy-benzyl)-2-oxo-2-[6-oxo-4-(1-oxy-pyridine-3-ylméthane-sulfonyl)-hexahydro-pyrrolo[3,2-b]pyrrole-1-yl]-éthyl}-amide d'acide biphényl-4-carboxylique ;
2-cyclohexylméthyl-4-morpholine-4-yl-1-[6-oxo-4-(pyridine-2-sulfonyl)-hexahydro-pyrrolo[3,2-b]pyrrole-1-yl]-butane-1,4-dione ;
2-cyclohexylméthyl-4-morpholine-4-yl-1-[6-oxo-4-(pyridine-3-sulfonyl)-hexahydro-pyrrolo[3,2-b]pyrrole-1-yl]-butane-1,4-dione ;
2-cyclohexylméthyl-4-morpholine-4-yl-1-[6-oxo-4-(1-oxy-pyridine-2-sulfonyl)-hexahydro-pyrrolo[3,2-b]pyrrole-1-yl]-butane-1,4-dione ;
2-cyclohexylméthyl-4-morpholine-4-yl-1-[6-oxo-4-(1-oxy-pyridine-3-sulfonyl)-hexahydro-pyrrolo[3,2-b]pyrrole-1-yl]-butane-1,4-dione ;
2-cyclohexylméthyl-4-morpholine-4-yl-1-[6-oxo-4-(pyridine-2-carbonyl)-hexahydro-pyrrolo[3,2-b]pyrrole-1-yl]-butane-1,4-dione ;
2-cyclohexylméthyl-4-morpholine-4-yl-1-[6-oxo-4-(2-pyridine-3-yl-acétyl)-hexahydro-pyrrolo[3,2-b]pyrrole-1-yl]-butane-1,4-dione ;
2-cyclohexylméthyl-4-morpholine-4-yl-1-[6-oxo-4-(pyridine-2-ylméthanesulfonyl)-hexahydro-pyrrolo[3,2-b]pyrrole-1-yl]-butane-1,4-dione ;
2-cyclohexylméthyl-4-morpholine-4-yl-1-[6-oxo-4-(pyridine-3-ylméthanesulfonyl)-hexahydro-pyrrolo[3,2-b]pyrrole-1-yl]-butane-1,4-dione ;
2-cyclohexylméthyl-4-morpholine-4-yl-1-[6-oxo-4-(1-oxy-pyridine-2-ylméthanesulfonyl)-hexahydro-pyrrolo[3,2-b]pyrrole-1-yl]-butane-1,4-dione ;
2-cyclohexylméthyl-4-morpholine-4-yl-1-[6-oxo-4-(1-oxy-pyridine-3-ylméthanesulfonyl)-hexahydro-pyrrolo[3,2-b]-pyrrole-1-yl]-butane-1,4-dione ;
2-(2,2-diméthyl-propyl)-4-morpholine-4-yl-1-[6-oxo-4-(pyridine-2-sulfonyl)-hexahydro-pyrrolo[3,2-b]pyrrole-1-yl]-butane-1,4-dione ;
2-(2,2-diméthyl-propyl)-4-morpholine-4-yl-1-[6-oxo-4-(pyridine-3-sulfonyl)-hexahydro-pyrrolo[3,2-b]pyrrole-1-yl]-butane-1,4-dione ;
2-(2,2-diméthyl-propyl)-4-morpholine-4-yl-1-[6-oxo-4-(1-oxy-pyridine-2-sulfonyl)-hexahydro-pyrrolo[3,2-b]pyrrole-1-yl]-butane-1,4-dione ;
2-(2,2-diméthyl-propyl)-4-morpholine-4-yl-1-[6-oxo-4-(1-oxy-pyridine-3-sulfonyl)-hexahydro-pyrrolo[3,2-b]pyrrole-1-yl]-butane-1,4-dione ;
2-(2,2-diméthyl-propyl)-4-morpholine-4-yl-1-[6-oxo-4-(pyridine-2-carbonyl)-hexahydro-pyrrolo[3,2-b]pyrrole-1-yl]-butane-1,4-dione ;
2-(2,2-diméthyl-propyl)-4-morpholine-4-yl-1-[6-oxo-4-(2-pyridine-3-yl-acétyl)-hexahydro-pyrrolo[3,2-b]pyrrole-1-yl]-butane-1,4-dione ;
2-(2,2-diméthyl-propyl)-4-morpholine-4-yl-1-[6-oxo-4-(pyridine-2-ylméthanesulfonyl)-hexahydro-pyrrolo[3,2-b]-pyrrole-1-yl]-butane-1,4-dione ;
2-(2,2-diméthyl-propyl)-4-morpholine-4-yl-1-[6-oxo-4-(pyridine-3-ylméthanesulfonyl)-hexahydro-pyrrolo[3,2-b]-pyrrole-1-yl]-butane-1,4-dione ;
2-(2,2-diméthyl-propyl)-4-morpholine-4-yl-1-[6-oxo-4-(1-oxy-pyridine-2-ylméthanesulfonyl)-hexahydro-pyrrolo[3,2-b]-pyrrole-1-yl]-butane-1,4-dione ;
2-(2,2-diméthyl-propyl)-4-morpholine-4-yl-1-[6-oxo-4-(1-oxy-pyridine-3-ylméthanesulfonyl)-hexahydro-pyrrolo[3,2-b]-pyrrole-1-yl]-butane-1,4-dione ;
N-[1-(4-benzoyl-6-oxo-hexahydro-pyrrolo[3,2-b]pyrrole-1-carbonyl)-3-méthyl-butyl]-4-tertiobutylbenzamide ;
4-tertiobutyl-N-(3-méthyl-1-[6-oxo-4-(1-oxy-pyridine-2-carbonyl)-hexahydro-pyrrolo[3,2-b]pyrrole-1-carbonyl]-butyl}-benzamide ;
4-tertiobutyl-N-(3-méthyl-1-[6-oxo-4-(pyridine-3-carbonyl)-hexahydro-pyrrolo[3,2-b]pyrrole-1-carbonyl]-butyl}-benzamide ;
4-tertiobutyl-N-(3-méthyl-1-[6-oxo-4-(1-oxy-pyridine-3-carbonyl)-hexahydro-pyrrolo[3,2-b]pyrrole-1-carbonyl]-butyl}-benzamide ;
4-tertiobutyl-N-(3-méthyl-1-[6-oxo-4-(pyridine-4-carbonyl)-hexahydro-pyrrolo[3,2-b]pyrrole-1-carbonyl]-butyl}-benzamide ;
4-tertiobutyl-N-(3-méthyl-1-[6-oxo-4-(1-oxy-pyridine-4-carbonyl)-hexahydro-pyrrolo[3,2-b]pyrrole-1-carbonyl]-butyl}-benzamide ;
N-[1-(4-benzènesulfonyl-6-oxo-hexahydro-pyrrolo[3,2-b]-pyrrole-1-carbonyl)-3-méthyl-butyl]-4-tertiobutyl-benzamide ;
4-tertiobutyl-N-(3-méthyl-1-[6-oxo-4-(pyridine-4-sulfonyl)-hexahydro-pyrrolo[3,2-b]pyrrole-1-carbonyl]-butyl}-benzamide ;
4-tertiobutyl-N-(3-méthyl-1-[6-oxo-4-(1-oxy-pyridine-4-sulfonyl)-hexahydro-pyrrolo[3,2-b]pyrrole-1-carbonyl]-butyl}-benzamide ;
4-tertiobutyl-N-[3-méthyl-1-(6-oxo-4-phénylacétyl-hexahydro-pyrrolo[3,2-b]pyrrole-1-carbonyl)-butyl]-benzamide ;
phénylamide d'acide 4-[2-(4-tertiobutyl-benzoylamino)-4-méthyl-pentanoyl]-3-oxo-hexahydro-pyrrolo[3,2-b]pyrrole-1-carboxylique ;
isobutylamide d'acide 4-[2-(4-tertiobutyl-benzoylamino)-4-méthyl-pentanoyl]-3-oxo-hexahydro-pyrrolo[3,2-b]pyrrole-1-carboxylique ;
ester phénylique d'acide 4-[2-(4-tertiobutyl-benzoylamino)-4-méthyl-pentanoyl]-3-oxo-hexahydro-pyrrolo[3,2-b]pyrrole-1-carboxylique ;
ester isobutylique d'acide 4-[2-(4-tertiobutyl-benzoylamino)-4-méthyl-pentanoyl]-3-oxo-hexahydro-pyrrolo[3,2-b]pyrrole-1-carboxylique ;
4-tertiobutyl-N-(3-méthyl-1-[6-oxo-4-(pyrrolidine-1-carbonyl)-hexahydro-pyrrolo[3,2-b]pyrrole-1-carbonyl]-butyl}-benzamide ;
4-tertiobutyl-N-(3-méthyl-1-[6-oxo-4-(pipéridine-1-carbonyl)-hexahydro-pyrrolo[3,2-b]pyrrole-1-carbonyl]-butyl}-benzamide ;
4-tertiobutyl-N-(3-méthyl-1-[6-oxo-4-(morpholine-4-carbonyl)-hexahydro-pyrrolo[3,2-b]pyrrole-1-carbonyl]-butyl}-benzamide ;
4-tertiobutyl-N-(3-méthyl-1-[6-oxo-4-(pipérazine-1-carbonyl)-hexahydro-pyrrolo[3,2-b]pyrrole-1-carbonyl]-butyl}-benzamide ;
4-tertiobutyl-N-(3-méthyl-1-[4-(méthyl-pipérazine-1-carbonyl)-hexahydro-pyrrolo[3,2-b]pyrrole-1-carbonyl]-butyl}-benzamide ;
4-tertiobutyl-N-(3-méthyl-1-{4-[2-(4-mêthylpipérazine-1-yl)-acétyl]-6-oxo-hexahydro-pyrrolo[3,2-b]pyrrole-1-carbonyl]-butyl}-benzamide ;
4-tertiobutyl-N-(3-méthyl-1-[6-oxo-4-(2-pipérazine-1-yl-acétique)-hexahydro-pyrrolo[3,2-b]pyrrole-1-carbonyl]-butyl}-benzamide ;
4-tertiobutyl-N-{1-[4-(furanne-2-carbonyl)-6-oxo-hexahydro-pyrrolo[3,2-b]pyrrole-1-carbonyl]-3-méthyl-butyl}-benzamide ;
4-tertiobutyl-N-{1-[4-(5-chloro-furanne-2-carbonyl)-6-oxo-hexahydro-pyrrolo[3,2-b]pyrrole-1-carbonyl]-3-méthyl-butyl}-benzamide ;
4-tertiobutyl-N-{1-[4-(thiophène-2-carbonyl)-6-oxo-hexahydro-pyrrolo[3,2-b]pyrrole-1-carbonyl]-3-méthyl-butyl}-benzamide ;
4-tertiobutyl-N-{1-[4-(5-chloro-thiophène-2-carbonyl)-6-oxo-hexahydro-pyrrolo[3,2-b]pyrrole-1-carbonyl]-3-méthyl-butyl}-benzamide ;
4-tertiobutyl-N-{1-[4-(furanne-3-carbonyl)-6-oxo-hexahydro-pyrrolo[3,2-b]pyrrole-1-carbonyl]-3-méthyl-butyl}-benzamide ;
4-tertiobutyl-N-{1-[4-(thiophène-3-carbonyl)-6-oxo-hexahydro-pyrrolo[3,2-b]pyrrole-1-carbonyl]-3-méthyl-butyl}-benzamide ;
4-tertiobutyl-N-[1-(4-cyclopentanecarbonyl-6-oxo-hexahydro-pyrrolo[3,2-b]pyrrole-1-carbonyl)-3-méthyl-butyl]-benzamide ;
4-tertiobutyl-N-[1-(4-cyclohexanecarbonyl-6-oxo-hexahydro-pyrrolo[3,2-b]pyrrole-1-carbonyl)-3-méthyl-butyl]-benzamide ;
4-tertiobutyl-N-{1-[4-(cyclopent-3-ènecarbonyl)-6-oxo-hexahydro-pyrrolo[3,2-b]pyrrole-1-carbonyl]-3-méthyl-butyl}-benzamide ;
4-tertiobutyl-N-{1-[4-(2-cyclopentyl-acétyl)-6-oxo-hexahydro-pyrrolo[3,2-b]pyrrole-1-carbonyl]-3-méthyl-butyl}-benzamide ;
4-tertiobutyl-N-{1-[4-(2-cyclohexyl-acétyl)-6-oxo-hexahydro-pyrrolo[3,2-b]pyrrole-1-carbonyl]-3-méthyl-butyl}-benzamide ;
4-tertiobutyl-N-{3-méthyl-1-[6-oxo-4-(pyrrolidine-2-carbonyl)-hexahydro-pyrrolo[3,2-b]pyrrole-1-carbonyl]-butyl}-benzamide ;
N-{1-[4-(1-acétyl-pyrrolidine-2-carbonyl)-6-oxo-hexahydro-pyrrolo[3,2-b]pyrrole-1-carbonyl]-3-méthyl-butyl}-4-tertiobutyl-benzamide ;
4-tertiobutyl-N-{3-méthyl-1-[6-oxo-4-(pipéridine-2-carbonyl)-hexahydro-pyrrolo[3,2-b]pyrrole-1-carbonyl]-butyl}-benzamide ;
N-{1-[4-(1-acétyl-pipéridine-2-carbonyl)-6-oxo-hexahydro-pyrrolo[3,2-b]pyrrole-1-carbonyl]-3-méthyl-butyl}-4-tertiobutyl-benzamide ;
4-tertiobutyl-N-{3-méthyl-1-[6-oxo-4-(tétrahydro-furanne-2-carbonyl)-hexahydro-pyrrolo[3,2-b]pyrrole-1-carbonyl]-butyl}-benzamide ;
N-{1-[4-(1-amino-cyclobutanecarbonyl)-6-oxo-hexahydro-pyrrolo[3,2-b]pyrrole-1-carbonyl]-3-méthyl-butyl}-4-tertiobutyl-benzamide ;
N-{1-[4-(1-amino-cyclopentanecarbonyl)-6-oxo-hexahydro-pyrrolo[3,2-b]pyrrole-1-carbonyl]-3-méthyl-butyl}-4-tertiobutyl-benzamide ;
N-{1-[4-(1-amino-cyclohexanecarbonyl)-6-oxo-hexahydro-pyrrolo[3,2-b]pyrrole-1-carbonyl]-3-méthyl-butyl}-4-tertiobutyl-benzamide ;
N-{1-[4-(1-acétylamino-cyclobutanecarbonyl)-6-oxo-hexahydro-pyrrolo[3,2-b]pyrrole-1-carbonyl]-3-méthyl-butyl}-4-tertiobutyl-benzamide ;
N-{1-[4-(1-acétylamino-cyclobutanecarbonyl)-6-oxo-hexahydro-pyrrolo[3,2-b]pyrrole-1-carbonyl]-3-méthyl-butyl}-4-tertiobutyl-benzamide ;
N-{1-[4-(1-acétylamino-cyclohexanecarbonyl)-6-oxo-hexahydro-pyrrolo[3,2-b]pyrrole-1-carbonyl]-3-méthyl-butyl}-4-tertiobutyl-benzamide ;
4-tertiobutyl-N-{1-[4-(1-hydroxy-cyclobutanecarbonyl)-6-oxo-hexahydro-pyrrolo[3,2-b]pyrrole-1-carbonyl]-3-méthyl-butyl}-benzamide ;
4-tertiobutyl-N-{1-[4-(1-hydroxy-cyclopentanecarbonyl)-6-oxo-hexahydro-pyrrolo[3,2-b]pyrrole-1-carbonyl]-3-méthyl-butyl}-benzamide ;
4-tertiobutyl-N-{1-[4-(1-hydroxy-cyclohexanecarbonyl)-6-oxo-hexahydro-pyrrolo[3,2-b]pyrrole-1-carbonyl]-3-méthyl-butyl}-benzamide ;
4-tertiobutyl-N-{1-[4-(1-méthoxy-cyclobutanecarbonyl)-6-oxo-hexahydro-pyrrolo[3,2-b]pyrrole-1-carbonyl]-3-méthyl-butyl}-benzamide ;
4-tertiobutyl-N-{1-[4-(1-méthoxy-cyclobutanecarbonyl)-6-oxo-hexahydro-pyrrolo[3,2-b]pyrrole-1-carbonyl]-3-méthyl-butyl}-benzamide ;
4-tertiobutyl-N-{1-[4-(1-méthoxy-cyclohexanecarbonyl)-6-oxo-hexahydro-pyrrolo[3,2-b]pyrrole-1-carbonyl]-3-méthyl-butyl}-benzamide ;
N-{1-[4-(2-amino-2-cyclopentylacétyl)-6-oxo-hexahydro-pyrrolo[3,2-b]pyrrole-1-carbonyl]-3-méthyl-butyl}-4-tertiobutyl-benzamide ;
N-{1-[4-(2-amino-2-cyclohexylacétyl)-6-oxo-hexahydro-pyrrolo[3,2-b]pyrrole-1-carbonyl]-3-méthyl-butyl}-4-tertiobutyl-benzamide ;
N-{1-[4-(2-acétylamino-2-cyclopentylacétyl)-6-oxo-hexahydro-pyrrolo[3,2-b]pyrrole-1-carbonyl]-3-méthyl-butyl}-4-tertiobutyl-benzamide ;
N-{1-[4-(2-acétylamino-2-cyclopentylacétyl)-6-oxo-hexahydro-pyrrolo[3,2-b]pyrrole-1-carbonyl]-3-méthyl-butyl}-4-tertiobutyl-benzamide ;
N-{1-[4-(2-acétylamino-propionyl)-6-oxo-hexahydro-pyrrolo[3,2-b]pyrrole-1-carbonyl]-3-méthyl-butyl}-4-tertiobutyl-benzamide ;
N-{1-[4-(2-acétylamino-acétyl)-6-oxo-hexahydro-pyrrolo-[3,2-b]pyrrole-1-carbonyl]-3-méthyl-butyl}-4-tertiobutyl-benzamide ;
N-(1-{4-[2-(acétyl-méthyl-amino)-acétyl]-6-oxo-hexahydro-pyrrolo[3,2-b]pyrrole-1-carbonyl}-3-méthyl-butyl)-4-tertiobutyl-benzamide ;
4-tertiobutyl-N-{1-[4-(2-dimethylamino-acétyl)-6-oxo-hexahydro-pyrrolo[3,2-b]pyrrole-1-carbonyl]-3-méthyl-butyl}-benzamide ;
N-{1-[4-(2-amino-butyryl)-6-oxo-hexahydro-pyrrolo[3,2-b]-pyrrole-1-carbonyl]-3-méthyl-butyl}-4-tertiobutyl-benzamide ;
N-{1-[4-(2-acétylamino-butyryl)-6-oxo-hexahydro-pyrrolo-[3,2-b]pyrrole-1-carbonyl]-3-méthyl-butyl}-4-tertiobutyl-benzamide ;
N-{1-[4-(2-amino-3-méthyl-butyryl)-6-oxo-hexahydro-pyrrolo-[3,2-b]pyrrole-1-carbonyl]-3-méthyl-butyl}-4-tertiobutyl-benzamide ;
N-{1-[4-(2-acétylamino-3-méthyl-butyryl)-6-oxo-hexahydro-pyrrolo[3,2-b]pyrrole-1-carbonyl]-3-méthyl-butyl}-4-tertiobutyl-benzamide ;
N-{1-[4-(2-amino-3,3-diméthyl-butyryl)-6-oxo-hexahydro-pyrrolo[3,2-b]pyrrole-1-carbonyl]-3-méthyl-butyl}-4-tertiobutyl-benzamide ;
N-{1-[4-(2-acétylamino-3,3-diméthyl-butyryl)-6-oxo-hexahydro-pyrrolo[3,2-b]pyrrole-1-carbonyl]-3-méthyl-butyl}-4-tertiobutyl-benzamide ;
N-{1-[4-(2-amino-3-méthyl-pentanoyl)-6-oxo-hexahydro-pyrrolo[3,2-b]pyrrole-1-carbonyl]-3-méthyl-butyl}-4-tertiobutyl-benzamide ;
N-{1-[4-(2-acétylamino-3-méthyl-pentanoyl)-6-oxo-hexahydro-pyrrolo[3,2-b]pyrrole-1-carbonyl]-3-méthyl-butyl}-4-tertiobutyl-benzamide ;
4-tertiobutyl-N-[3-méthyl-1-(6-oxo-4-pentanoyl-hexahydro-pyrrolo[3,2-b]pyrrole-1-carbonyl)-butyl]-benzamide ;
4-tertiobutyl-N-{3-méthyl-1-[4-(3-méthyl-pentanoyl)-6-oxo-hexahydro-pyrrolo[3,2-b]pyrrole-1-carbonyl]-butyl}-benzamide ;
4-tertiobutyl-N-{3-méthyl-1-[4-(4-méthyl-pentanoyl)-6-oxo-hexahydro-pyrrolo[3,2-b]pyrrole-1-carbonyl]-butyl}-benzamide ;
N-{1-[4-(2-amino-4-méthyl-pentanoyl)-6-oxo-hexahydro-pyrrolo[3,2-b]pyrrole-1-carbonyl]-3-méthyl-butyl}-4-tertiobutyl-benzamide ;
N-{1-[4-(2-acétylamino-3-méthyl-pentanoyl)-6-oxo-hexahydro-pyrrolo[3,2-b]pyrrole-1-carbonyl]-3-méthyl-butyl}-4-tertiobutyl-benzamide ;
N-{1-[4-(2-amino-4,4-diméthyl-pentanoyl)-6-oxo-hexahydro-pyrrolo[3,2-b]pyrrole-1-carbonyl]-3-méthyl-butyl}-4-tertiobutyl-benzamide ;
N-{1-[4-(2-acétylamino-4,4-diméthyl-pentanoyl)-6-oxo-hexahydro-pyrrolo[3,2-b]pyrrole-1-carbonyl]-3-méthyl-butyl}-4-tertiobutyl-benzamide ;
N-{1-[4-(2-amino-pentanoyl)-6-oxo-hexahydro-pyrrolo[3,2-b]-pyrrole-1-carbonyl]-3-méthyl-butyl}-4-tertiobutyl-benzamide ;
N-{1-[4-(2-acétylamino-pentanoyl)-6-oxo-hexahydro-pyrrolo-[3,2-b]pyrrole-1-carbonyl]-3-méthyl-butyl}-4-tertiobutyl-benzamide ;
N-{1-[4-(2-amino-5-méthyl-hexanoyl)-6-oxo-hexahydro-pyrrolo[3,2-b]pyrrole-1-carbonyl]-3-méthyl-butyl}-4-tertiobutyl-benzamide ;
N-{1-[4-(2-acétylamino-5-méthyl-hexanoyl)-6-oxo-hexahydro-pyrrolo[3,2-b]pyrrole-1-carbonyl]-3-méthyl-butyl}-4-tertiobutyl-benzamide ;
N-{1-[4-(2-hydroxy-3-méthyl-butyryl)-6-oxo-hexahydro-pyrrolo[3,2-b]pyrrole-1-carbonyl]-3-méthyl-butyl}-4-tertiobutyl-benzamide ;
N-{1-[4-(2-méthoxy-3-méthyl-butyryl)-6-oxo-hexahydro-pyrrolo[3,2-b]pyrrole-1-carbonyl]-3-méthyl-butyl}-4-tertiobutyl-benzamide ;
4-tertiobutyl-N-{1-[4-(2-hydroxy-3,3-diméthyl-butyryl)-6-oxo-hexahydro-pyrrolo[3,2-b]pyrrole-1-carbonyl]-3-méthyl-butyl}-benzamide ;
4-tertiobutyl-N-{1-[4-(2-méthoxy-3,3-diméthyl-butyryl)-6-oxo-hexahydro-pyrrolo[3,2-b]pyrrole-1-carbonyl]-3-méthyl-butyl}-benzamide ;
4-tertiobutyl-N-{1-[4-(2-hydroxy-3-méthyl-pentanoyl)-6-oxo-hexahydro-pyrrolo[3,2-b]pyrrole-1-carbonyl]-3-méthyl-butyl}-benzamide ;
4-tertiobutyl-N-{1-[4-(2-méthoxy-3-méthyl-pentanoyl)-6-oxo-hexahydro-pyrrolo[3,2-b]pyrrole-1-carbonyl]-3-méthyl-butyl}-benzamide ;
4-tertiobutyl-N-{1-[4-(2-hydroxy-4-méthyl-pentanoyl)-6-oxo-hexahydro-pyrrolo[3,2-b]pyrrole-1-carbonyl]-3-méthyl-butyl}-benzamide ;

4-tertiobutyl-N-{1-[4-(2-méthoxy-4-méthyl-pentanoyl)-6-oxo-hexahydro-pyrrolo[3,2-b]pyrrole-1-carbonyl]-3-méthyl-butyl}-benzamide ;
4-tertiobutyl-N-{1-[4-(2-hydroxy-4,4-diméthyl-pentanoyl)-6-oxo-hexahydro-pyrrolo[3,2-b]pyrrole-1-carbonyl]-3-méthyl-butyl}-benzamide ;
4-tertiobutyl-N-{1-[4-(2-méthoxy-4,4-diméthyl-pentanoyl)-6-oxo-hexahydro-pyrrolo[3,2-b]pyrrole-1-carbonyl]-3-méthyl-butyl}-benzamide ;
4-tertiobutyl-N-{1-[4-(2-hydroxy-pentanoyl)-6-oxo-hexahydro-pyrrolo[3,2-b]pyrrole-1-carbonyl]-3-méthyl-butyl}-benzamide ;
4-tertiobutyl-N-{1-[4-(2-méthoxy-pentanoyl)-6-oxo-hexahydro-pyrrolo[3,2-b]pyrrole-1-carbonyl]-3-méthyl-butyl}-benzamide ;
4-tertiobutyl-N-{1-[4-(2-hydroxy-5-méthyl-hexanoyl)-6-oxo-hexahydro-pyrrolo[3,2-b]pyrrole-1-carbonyl]-3-méthyl-butyl}-benzamide ;
4-tertiobutyl-N-{1-[4-(2-méthoxy-5-méthyl-hexanoyl)-6-oxo-hexahydro-pyrrolo[3,2-b]pyrrole-1-carbonyl]-3-méthyl-butyl}-benzamide ;
N-[1-(4-benzoyl-6-oxo-hexahydro-pyrrolo[3,2-b]pyrrole-1-carbonyl)-3-méthyl-butyl]-4-diméthylamino-benzamide ;
4-diméthylamino-N-{3-méthyl-1-[6-oxo-4-(1-oxy-pyridine-2-carbonyl)-hexahydro-pyrrolo[3,2-b]pyrrole-1-carbonyl]-butyl}-benzamide ;
4-diméthylamino-N-{3-méthyl-1-[6-oxo-4-(pyridine-3-carbonyl)-hexahydro-pyrrolo[3,2-b]pyrrole-1-carbonyl]-butyl}-benzamide ;
4-diméthylamino-N-{3-méthyl-1-[6-oxo-4-(1-oxy-pyridine-3-carbonyl)-hexahydro-pyrrolo[3,2-b]pyrrole-1-carbonyl]-butyl}-benzamide ;
4-diméthylamino-N-{3-méthyl-1-[6-oxo-4-(pyridine-4-carbonyl)-hexahydro-pyrrolo[3,2-b]pyrrole-1-carbonyl]-butyl}-benzamide ;
4-diméthylamino-N-{3-méthyl-1-[6-oxo-4-(1-oxy-pyridine-4-carbonyl)-hexahydro-pyrrolo[3,2-b]pyrrole-1-carbonyl]-butyl}-benzamide ;
N-[1-(4-benzènesulfonyl-6-oxo-hexahydro-pyrrolo[3,2-b]-pyrrole-1-carbonyl)-3-méthyl-butyl]-4-diméthylamino-benzamide ;
4-dimêthylamino-N-{3-méthyl-1-[6-oxo-4-(pyridine-4-sulfonyl)-hexahydro-pyrrolo[3,2-b]pyrrole-1-carbonyl]-butyl}-benzamide ;
4-diméthylamino-N-{3-méthyl-1-[6-oxo-4-(1-oxy-pyridine-4-sulfonyl)-hexahydro-pyrrolo[3,2-b]pyrrole-1-carbonyl]-butyl}-benzamide ;
4-diméthylamino-N-[3-méthyl-1-(6-oxo-4-phénylacétyl-hexahydro-pyrrolo[3,2-b]pyrrole-1-carbonyl)-butyl]-benzamide ;
phénylamide d'acide 4-[2-(4-diméthylamino-benzoylamino)-4-méthyl-pentanoyl]-3-oxo-hexahydro-pyrrolo[3,2-b]pyrrole-1-carboxylique ;
isobutylamide d'acide 4-[2-(4-diméthylamino-benzoylamino)-4-méthyl-pentanoyl]-3-oxo-hexahydro-pyrrolo[3,2-b]pyrrole-1-carboxylique ;
ester phénylique d'acide 4-[2-(4-diméthylamino-benzoylamino)-4-méthyl-pentanoyl]-3-oxo-hexahydro-pyrrolo[3,2-b]-pyrrole-1-carboxylique ;
ester isobutylique d'acide 4-[2-(4-diméthylamino-benzoylamino)-4-méthyl-pentanoyl]-3-oxo-hexahydro-pyrrolo[3,2-b]-pyrrole-1-carboxylique ;
4-diméthylamino-N-{3-méthyl-1-[6-oxo-4-(pyrrolidine-1-carbonyl)-hexahydro-pyrrolo[3,2-b]pyrrole-1-carbonyl]-butyl}-benzamide ;
4-diméthylamino-N-{3-méthyl-1-[6-oxo-4-(pipéridine-1-carbonyl)-hexahydro-pyrrolo[3,2-b]pyrrole-1-carbonyl]-butyl}-benzamide ;
4-diméthylamino-N-{3-méthyl-1-[4-(morpholine-4-carbonyl)-6-oxo-hexahydro-pyrrolo[3,2-b]pyrrole-1-carbonyl]-butyl}-benzamide ;
4-diméthylamino-N-{3-méthyl-1-[6-oxo-4-(pipérazine-1-carbonyl)-hexahydro-pyrrolo[3,2-b]pyrrole-1-carbonyl]-butyl}-benzamide ;
4-diméthylamino-N-{3-méthyl-1-[4-(4-méthyl-pipérazine-1-carbonyl)-6-oxo-hexahydro-pyrrolo[3,2-b]pyrrole-1-carbonyl]-butyl}-benzamide ;
4-diméthylamino-N-(3-méthyl-1-{4-[2-(4-méthyl-pipérazine-1-yl)-acétyl]-6-oxo-hexahydro-pyrrolo[3,2-b]pyrrole-1-carbonyl}-butyl)-benzamide ;
4-diméthylamino-N-{3-méthyl-1-[6-oxo-4-(2-pipérazine-1-yl-acétyl)-hexahydro-pyrrolo[3,2-b]pyrrole-1-carbonyl]-butyl}-benzamide ;
4-diméthylamino-N-{1-[4-(furanne-2-carbonyl)-6-oxo-hexahydro-pyrrolo[3,2-b]pyrrole-1-carbonyl]-3-méthyl-butyl}-benzamide ;
4-diméthylamino-N-{1-[4-(5-chlorofuranne-2-carbonyl)-6-oxo-hexahydro-pyrrolo[3,2-b]pyrrole-1-carbonyl]-3-méthyl-butyl}-benzamide ;
4-diméthylamino-N-{1-[4-(thiophène-2-carbonyl)-6-oxo-hexahydro-pyrrolo[3,2-b]pyrrole-1-carbonyl]-3-méthyl-butyl}-benzamide ;
4-diméthylamino-N-{1-[4-(5-chlorothiophène-2-carbonyl)-6-oxo-hexahydro-pyrrolo[3,2-b]pyrrole-1-carbonyl]-3-méthyl-butyl}-benzamide ;
4-diméthylamino-N-{1-[4-(furanne-3-carbonyl)-6-oxo-hexahydro-pyrrolo[3,2-b]pyrrole-1-carbonyl]-3-méthyl-butyl}-benzamide ;
4-diméthylamino-N-{1-[4-(thiophène-3-carbonyl)-6-oxo-hexahydro-pyrrolo[3,2-b]pyrrole-1-carbonyl]-3-méthyl-butyl}-benzamide ;
4-diméthylamino-N-[1-(4-cyclopentanecarbonyl-6-oxo-hexahydro-pyrrolo[3,2-b]pyrrole-1-carbonyl)-3-méthyl-butyl]-benzamide ;
4-diméthylamino-N-[1-(4-cyclohexanecarbonyl-6-oxo-hexahydro-pyrrolo[3,2-b]pyrrole-1-carbonyl)-3-méthyl-butyl]-benzamide ;
4-diméthylamino-N-{1-[4-(cyclopent-3-ènecarbonyl)-6-oxo-hexahydro-pyrrolo[3,2-b]pyrrole-1-carbonyl]-3-méthyl-butyl}-benzamide ;
4-diméthylamino-N-{1-[4-(2-cyclopentyl-acétyl)-6-oxo-hexahydro-pyrrolo[3,2-b]pyrrole-1-carbonyl]-3-méthyl-butyl}-benzamide ;
4-diméthylamino-N-{1-[4-(2-cyclohexyl-acétyl)-6-oxo-hexahydro-pyrrolo[3,2-b]pyrrole-1-carbonyl]-3-méthyl-butyl}-benzamide ;
4-diméthylamino-N-{3-méthyl-1-[6-oxo-4-(pyrrolidine-2-carbonyl)-hexahydro-pyrrolo[3,2-b]pyrrole-1-carbonyl]-butyl}-benzamide ;
N-{1-[4-(1-acétyl-pyrrolidone-2-carbonyl)-6-oxo-hexahydro-pyrrolo[3,2-b]pyrrole-1-carbonyl]-3-méthyl-butyl}-4-diméthylamino-benzamide ;
4-diméthylamino-N-{3-méthyl-1-[6-oxo-4-(pipéridine-2-carbonyl)-hexahydro-pyrrolo[3,2-b]pyrrole-1-carbonyl]-butyl}-benzamide ;
N-{1-[4-(1-acétyl-pipéridine-2-carbonyl)-6-oxo-hexahydro-pyrrolo[3,2-b]pyrrole-1-carbonyl]-3-méthyl-butyl}-4-diméthylamino-benzamide ;
4-diméthylamino-N-{3-méthyl-1-[6-oxo-4-(tétrahydro-furanne-2-carbonyl)-hexahydro-pyrrolo[3,2-b]pyrrole-1-carbonyl]-butyl}-benzamide ;
N-{1-[4-(1-amino-cyclobutanecarbonyl)-6-oxo-hexahydro-pyrrolo[3,2-b]pyrrole-1-carbonyl]-3-méthyl-butyl}-4-diméthylamino-benzamide ;
N-{1-[4-(1-amino-cyclopentanecarbonyl)-6-oxo-hexahydro-pyrrolo[3,2-b]pyrrole-1-carbonyl]-3-méthyl-butyl}-4-diméthylamino-benzamide ;
N-{1-[4-(1-amino-cyclohexanecarbonyl)-6-oxo-hexahydro-pyrrolo[3,2-b]pyrrole-1-carbonyl]-3-méthyl-butyl}-4-diméthylamino-benzamide ;
N-{1-[4-(1-acétylamino-cyclobutanecarbonyl)-6-oxo-hexahydro-pyrrolo[3,2-b]pyrrole-1-carbonyl]-3-méthyl-butyl}-4-diméthylamino-benzamide ;
N-{1-[4-(1-acétylamino-cyclopentanecarbonyl)-6-oxo-hexahydro-pyrrolo[3,2-b]pyrrole-1-carbonyl]-3-méthyl-butyl}-4-diméthylamino-benzamide ;
N-{1-[4-(1-acétylamino-cyclohexanecarbonyl)-6-oxo-hexahydro-pyrrolo[3,2-b]pyrrole-1-carbonyl]-3-méthyl-butyl}-4-diméthylamino-benzamide ;
4-diméthylamino-N-{1-[4-(1-hydroxy-cyclobutanecarbonyl)-6-oxo-hexahydro-pyrrolo[3,2-b]pyrrole-1-carbonyl]-3-méthyl-butyl}-benzamide ;
4-diméthylamino-N-{1-[4-(1-hydroxy-cyclopentanecarbonyl)-6-oxo-hexahydro-pyrrolo[3,2-b]pyrrole-1-carbonyl]-3-méthyl-butyl}-benzamide ;
4-diméthylamino-N-{1-[4-(1-hydroxy-cyclohexanecarbonyl)-6-oxo-hexahydro-pyrrolo[3,2-b]pyrrole-1-carbonyl]-3-méthyl-butyl}-benzamide ;
4-diméthylamino-N-{1-[4-(1-méthoxy-cyclobutanecarbonyl)-6-oxo-hexahydro-pyrrolo[3,2-b]pyrrole-1-carbonyl]-3-méthyl-butyl}-benzamide ;
4-diméthylamino-N-{1-[4-(1-méthoxy-cyclopentanecarbonyl)-6-oxo-hexahydro-pyrrolo[3,2-b]pyrrole-1-carbonyl]-3-méthyl-butyl}-benzamide ;
4-diméthylamino-N-{1-[4-(1-méthoxy-cyclohexanecarbonyl)-6-oxo-hexahydro-pyrrolo[3,2-b]pyrrole-1-carbonyl]-3-méthyl-butyl}-benzamide ;
N-{1-[4-(2-amino-2-cyclopentyl-acétyl)-6-oxo-hexahydro-pyrrolo[3,2-b]pyrrole-1-carbonyl]-3-méthyl-butyl}-4-diméthylamino-benzamide ;
N-{1-[4-(2-amino-2-cyclohexyl-acétyl)-6-oxo-hexahydro-pyrrolo[3,2-b]pyrrole-1-carbonyl]-3-méthyl-butyl}-4-diméthylamino-benzamide ;
N-{1-[4-(2-acétylamino-2-cyclopentyl-acétyl)-6-oxo-hexahydro-pyrrolo[3,2-b]pyrrole-1-carbonyl]-3-méthyl-butyl}-4-diméthylamino-benzamide ;
N-{1-[4-(2-acétylamino-2-cyclohexyl-acétyl)-6-oxo-hexahydro-pyrrolo[3,2-b]pyrrole-1-carbonyl]-3-méthyl-butyl}-4-diméthylamino-benzamide ;
N-{1-[4-(2-acétylaminopropionyl)-6-oxo-hexahydro-pyrrolo[3,2-b]pyrrole-1-carbonyl]-3-méthyl-butyl}-4-diméthylamino-benzamide ;
N-{1-[4-(2-acétylamino-acétyl)-6-oxo-hexahydro-pyrrolo-[3,2-b]pyrrole-1-carbonyl]-3-méthyl-butyl}-4-diméthylamino-benzamide ;
N-(1-{4-[2-(acétyl-méthyl-amino)-acétyl]-6-oxo-hexahydro-pyrrolo[3,2-b]pyrrole-1-carbonyl}-3-méthyl-butyl)-4-diméthylamino-benzamide ;
4-diméthylamino-N-{1-[4-(2-diméthylamino-acétyl)-6-oxo-hexahydro-pyrrolo[3,2-b]pyrrole-1-carbonyl]-3-méthyl-butyl}-benzamide ;
N-{1-[4-(2-amino-butyryl)-6-oxo-hexahydro-pyrrolo-[3,2-b]-pyrrole-1-carbonyl]-3-méthyl-butyl}-4-diméthylamino-benzamide ;
N-{1-[4-(2-acétylamino-butyryl)-6-oxo-hexahydro-pyrrolo-[3,2-b]pyrrole-1-carbonyl]-3-méthyl-butyl}-4-diméthylamino-benzamide ;
N-{1-[4-(2-amino-3-méthyl-butyryl)-6-oxo-hexahydro-pyrrolo-[3,2-b]pyrrole-1-carbonyl]-3-méthyl-butyl}-4-diméthylamino-benzamide ;
N-{1-[4-(2-acétylamino-3-méthyl-butyryl)-6-oxo-hexahydro-pyrrolo[3,2-b]pyrrole-1-carbonyl]-3-méthyl-butyl}-4-diméthylamino-benzamide ;
N-{1-[4-(2-amino-3,3-diméthyl-butyryl)-6-oxo-hexahydro-pyrrolo[3,2-b]pyrrole-1-carbonyl]-3-méthyl-butyl}-4-diméthylamino-benzamide ;
N-{1-[4-(2-acétylamino-3,3-diméthyl-butyryl)-6-oxo-hexahydro-pyrrolo[3,2-b]pyrrole-1-carbonyl]-3-méthyl-butyl}-4-diméthylamino-benzamide ;
N-{1-[4-(2-amino-3-méthyl-pentanoyl)-6-oxo-hexahydro-pyrrolo[3,2-b]pyrrole-1-carbonyl]-3-méthyl-butyl}-4-diméthylamino-benzamide ;
N-{1-[4-(2-acétylamino-3-méthyl-pentanoyl)-6-oxo-hexahydro-pyrrolo[3,2-b]pyrrole-1-carbonyl]-3-méthyl-butyl}-4-diméthylamino-benzamide ;
4-diméthylamino-N-[3-méthyl-1-(6-oxo-4-pentanoyl-hexahydro-pyrrolo[3,2-b]pyrrole-1-carbonyl)-butyl]-benzamide ;
4-diméthylamino-N-{3-méthyl-1-[4-(3-méthyl-pentanoyl)-6-oxo-hexahydro-pyrrolo[3,2-b]pyrrole-1-carbonyl]-butyl}-benzamide ;
4-diméthylamino-N-{3-méthyl-1-[4-(4-méthyl-pentanoyl)-6-oxo-hexahydro-pyrrolo[3,2-b]pyrrole-1-carbonyl]-butyl}-benzamide ;
N-{1-[4-(2-amino-4-méthyl-pentanoyl)-6-oxo-hexahydro-pyrrolo[3,2-b]pyrrole-1-carbonyl]-3-méthyl-butyl}-4-diméthylamino-benzamide ;
N-{1-[4-(2-acétylamino-4-méthyl-pentanoyl)-6-oxo-hexahydro-pyrrolo[3,2-b]pyrrole-1-carbonyl]-3-méthyl-butyl}-4-diméthylamino-benzamide ;
N-{1-[4-(2-amino-4,4-diméthyl-pentanoyl)-6-oxo-hexahydro-pyrrolo[3,2-b]pyrrole-1-carbonyl]-3-méthyl-butyl}-4-diméthylamino-benzamide ;
N-{1-[4-(2-acétylamino-4,4-diméthyl-pentanoyl)-6-oxo-hexahydro-pyrrolo[3,2-b]pyrrole-1-carbonyl]-3-méthyl-butyl}-4-diméthylamino-benzamide ;
N-{1-[4-(2-amino-pentanoyl)-6-oxo-hexahydro-pyrrolo[3,2-b]-pyrrole-1-carbonyl]-3-méthyl-butyl}-4-diméthylamino-benzamide ;
N-{1-[4-(2-acétylamino-pentanoyl)-6-oxo-hexahydro-pyrrolo[3,2-b]pyrrole-1-carbonyl]-3-méthyl-butyl}-4-diméthylamino-benzamide ;
N-{1-[4-(2-amino-5-méthyl-hexanoyl)-6-oxo-hexahydro-pyrrolo[3,2-b]pyrrole-1-carbonyl]-3-méthyl-butyl}-4-diméthylamino-benzamide ;
N-{1-[4-(2-acétylamino-5-méthyl-hexanoyl)-6-oxo-hexahydro-pyrrolo[3,2-b]pyrrole-1-carbonyl]-3-méthyl-butyl}-4-diméthylamino-benzamide ;
N-{1-[4-(2-hydroxy-3-méthyl-butyryl)-6-oxo-hexahydro-pyrrolo[3,2-b]pyrrole-1-carbonyl]-3-méthyl-butyl}-4-diméthylamino-benzamide ;
N-{1-[4-(2-méthoxy-3-méthyl-butyryl)-6-oxo-hexahydro-pyrrolo[3,2-b]pyrrole-1-carbonyl]-3-méthyl-butyl}-4-diméthylamino-benzamide ;
4-diméthylamino-N-{1-[4-(2-hydroxy-3,3-diméthyl-butyryl)-6-oxo-hexahydro-pyrrolo[3,2-b]pyrrole-1-carbonyl]-3-méthyl-butyl}-benzamide ;
4-diméthylamino-N-{1-[4-(2-méthoxy-3,3-diméthyl-butyryl)-6-oxo-hexahydro-pyrrolo[3,2-b]pyrrole-1-carbonyl]-3-méthyl-butyl}-benzamide ;
4-diméthylamino-N-{1-[4-(2-hydroxy-3-méthyl-pentanoyl)-6-oxo-hexahydro-pyrrolo[3,2-b]pyrrole-1-carbonyl]-3-méthyl-butyl}-benzamide ;
4-diméthylamino-N-{1-[4-(2-méthoxy-3-méthyl-pentanoyl)-6-oxo-hexahydro-pyrrolo[3,2-b]pyrrole-1-carbonyl]-3-méthyl-butyl}-benzamide ;
4-diméthylamino-N-{1-[4-(2-hydroxy-4-méthyl-pentanoyl)-6-oxo-hexahydro-pyrrolo[3,2-b]pyrrole-1-carbonyl]-3-méthyl-butyl}-benzamide ;
4-diméthylamino-N-{1-[4-(2-méthoxy-4-méthyl-pentanoyl)-6-oxo-hexahydro-pyrrolo[3,2-b]pyrrole-1-carbonyl]-3-méthyl-butyl}-benzamide ;
4-diméthylamino-N-{1-[4-(2-hydroxy-4,4-diméthyl-pentanoyl)-6-oxo-hexahydro-pyrrolo[3,2-b]pyrrole-1-carbonyl]-3-méthyl-butyl}-benzamide ;
4-diméthylamino-N-{1-[4-(2-méthoxy-4,4-diméthyl-pentanoyl)-6-oxo-hexahydro-pyrrolo[3,2-b]pyrrole-1-carbonyl]-3-méthyl-butyl}-benzamide ;
4-diméthylamino-N-{1-[4-(2-hydroxy-pentanoyl)-6-oxo-hexahydro-pyrrolo[3,2-b]pyrrole-1-carbonyl]-3-méthyl-butyl}-benzamide ;
4-diméthylamino-N-{1-[4-(2-méthoxy-pentanoyl)-6-oxo-hexahydro-pyrrolo[3,2-b]pyrrole-1-carbonyl]-3-méthyl-butyl}-benzamide ;
4-diméthylamino-N-{1-[4-(2-hydroxy-5-méthyl-hexanoyl)-6-oxo-hexahydro-pyrrolo[3,2-b]pyrrole-1-carbonyl]-3-méthyl-butyl}-benzamide ;
4-diméthylamino-N-{1-[4-(2-méthoxy-5-méthyl-hexanoyl)-6-oxo-hexahydro-pyrrolo[3,2-b]pyrrole-1-carbonyl]-3-méthyl-butyl}-benzamide ;
N-[1-(4-benzoyl-6-oxo-hexahydro-pyrrolo[3,2-b]pyrrole-1-carbonyl)-3-méthyl-butyl]-4-thiophène-2-yl-benzamide ;
N-{3-méthyl-1-[6-oxo-4-(1-oxy-pyridine-2-carbonyl)-hexahydro-pyrrolo[3,2-b]pyrrole-1-carbonyl]-butyl}-4-thiophène-2-yl-benzamide ;
N-{3-méthyl-1-[6-oxo-4-(pyridine-3-carbonyl)-hexahydro-pyrrolo[3,2-b]pyrrole-1-carbonyl]-butyl}-4-thiophène-2-yl-benzamide ;
N-{3-méthyl-1-[6-oxo-4-(1-oxy-pyridine-3-carbonyl)-hexahydro-pyrrolo[3,2-b]pyrrole-1-carbonyl]-butyl}-4-thiophène-2-yl-benzamide ;
N-{3-méthyl-1-[6-oxo-4-(pyridine-4-carbonyl)-hexahydro-pyrrolo[3,2-b]pyrrole-1-carbonyl]-butyl}-4-thiophène-2-yl-benzamide ;
N-{3-méthyl-1-[6-oxo-4-(1-oxy-pyridine-4-carbonyl)-hexahydro-pyrrolo[3,2-b]pyrrole-1-carbonyl]-butyl}-4-thiophène-2-yl-benzamide ;
N-[1-(4-benzènesulfonyl-6-oxo-hexahydro-pyrrolo[3,2-b]-pyrrole-1-carbonyl)-3-méthyl-butyl]-4-thiophène-2-yl-benzamide ;
N-{3-méthyl-1-[6-oxo-4-(pyridine-4-sulfonyl)-hexahydro-pyrrolo[3,2-b]pyrrole-1-carbonyl]-butyl}-4-thiophène-2-yl-benzamide ;
N-{3-méthyl-1-[6-oxo-4-(1-oxy-pyridine-4-sulfonyl)-hexahydro-pyrrolo[3,2-b]pyrrole-1-carbonyl]-butyl}-4-thiophène-2-yl-benzamide ;
N-[3-méthyl-1-(6-oxo-4-phénylacétyl-hexahydro-pyrrolo-[3,2-b]-pyrrole-1-carbonyl)-butyl]-4-thiophène-2-yl-benzamide ;
phénylamide d’acide 4-[4-méthyl-2-(4-thiophène-2-yl-benzoylamino)-pentanoyl]-3-oxo-hexahydro-pyrrolo[3,2-b]-pyrrole-1-carboxylique ;
isobutylamide d’acide 4-[4-méthyl-2-(4-thiophène-2-yl-benzoylamino)-pentanoyl]-3-oxo-hexahydro-pyrrolo[3,2-b]-pyrrole-1-carboxylique ;
ester phénylique d’acide 4-[4-méthyl-2-(4-thiophène-2-yl-benzoylamino)-pentanoyl]-3-oxo-hexahydro-pyrrolo[3,2-b]- pyrrole-1-carboxylique ;
ester isobutylique d’acide 4-[4-méthyl-2-(4-thiophène-2-yl-benzoylamino)-pentanoyl]-3-oxo-hexahydro-pyrrolo[3,2-b]-pyrrole-1-carboxylique ;
N-{3-méthyl-1-[6-oxo-4-(pyrrolidine-1-carbonyl)-hexahydro-pyrrolo[3,2-b]pyrrole-1-carbonyl]-butyl}-4-thiophène-2-yl-benzamide ;
N-{3-méthyl-1-[6-oxo-4-(pipéridine-1-carbonyl)-hexahydro-pyrrolo[3,2-b]pyrrole-1-carbonyl]-butyl}-4-thiophène-2-yl-benzamide ;
N-{3-méthyl-1-[4-(morpholine-4-carbonyl)-6-oxo-hexahydro-pyrrolo[3,2-b]pyrrole-1-carbonyl]-butyl}-4-thiophène-2-yl-benzamide ;
N-{3-méthyl-1-[6-oxo-4-(pipérazine-1-carbonyl)-hexahydro-pyrrolo[3,2-b]pyrrole-1-carbonyl]-butyl}-4-thiophène-2-yl-benzamide ;
N-{3-méthyl-1-[4-(4-méthyl-pipérazine-1-carbonyl)-6-oxo-hexahydro-pyrrolo[3,2-b]pyrrole-1-carbonyl]-butyl}-4-thiophène-2-yl-benzamide ;
N-(3-méthyl-1-{4-[2-(4-méthyl-pipérazine-1-yl)-acétyl]-6-oxo-hexahydro-pyrrolo[3,2-b]pyrrole-1-carbonyl}-butyl)-4-thiophène-2-yl-benzamide ;

N-{3-méthyl-1-[6-oxo-4-(2-pipéridine-1-yl-acétyl)-hexahydro-pyrrolo[3,2-b]pyrrole-1-carbonyl]-butyl}-4-thiophène-2-yl-benzamide ;
N-{1-[4-(furanne-2-carbonyl)-6-oxo-hexahydro-pyrrolo-[3,2-b]pyrrole-1-carbonyl]-méthyl-butyl}-4-thiophène-2-yl-benzamide ;
N-{1-[4-(5-chloro-furanne-2-carbonyl)-6-oxo-hexahydro-pyrrolo[3,2-b]pyrrole-1-carbonyl]-3-méthyl-butyl}-4-thiophène-2-yl-benzamide ;
N-{1-[4-(thiophène-2-carbonyl)-6-oxo-hexahydro-pyrrolo-[3,2-b]pyrrole-1-carbonyl]-3-méthyl-butyl}-4-thiophène-2-yl-benzamide ;
N-{1-[4-(5-chloro-thiophène-2-carbonyl)-6-oxo-hexahydro-pyrrolo[3,2-b]pyrrole-1-carbonyl]-3-méthyl-butyl}-4-thiophène-2-yl-benzamide ;
N-{1-[4-(furanne-3-carbonyl)-6-oxo-hexahydro-pyrrolo-[3,2-b]pyrrole-1-carbonyl]-3-méthyl-butyl}-4-thiophène-2-yl-benzamide ;
N-{1-[4-(thiophène-3-carbonyl)-6-oxo-hexahydro-pyrrolo-[3,2-b]pyrrole-1-carbonyl]-3-méthyl-butyl}-4-thiophène-2-yl-benzamide ;
N-[1-(4-cyclopentanecarbonyl-6-oxo-hexahydro-pyrrolo-[3,2-b]pyrrole-1-carbonyl)-3-méthyl-butyl]-4-thiophène-2-yl-benzamide ;
N-[1-(4-cyclohexanecarbonyl-6-oxo-hexahydro-pyrrolo-[3,2-b]pyrrole-1-carbonyl)-3-méthyl-butyl]-4-thiophène-2-yl-benzamide ;
N-{1-[4-(cyclopent-3-ènecarbonyl)-6-oxo-hexahydro-pyrrolo-[3,2-b]pyrrole-1-carbonyl]-3-méthyl-butyl}-4-thiophène-2-yl-benzamide ;
N-{1-[4-(cyclopentyl-acétyl)-6-oxo-hexahydro-pyrrolo-[3,2-b]pyrrole-1-carbonyl]-3-méthyl-butyl}-4-thiophène-2-yl-benzamide ;
N-{1-[4-(2-cyclohexyl-acétyl)-6-oxo-hexahydro-pyrrolo-[3,2-b]pyrrole-1-carbonyl]-3-méthyl-butyl}-4-thiophène-2-yl-benzamide ;
N-{3-méthyl-1-[6-oxo-4-(pyrrolidine-2-carbonyl)-hexahydro-pyrrolo[3,2-b]pyrrole-1-carbonyl]-butyl}-4-thiophène-2-yl-benzamide ;
N-{1-[4-(1-acétyl-pyrrolidine-2-carbonyl)-6-oxo-hexahydro-pyrrolo[3,2-b]pyrrole-1-carbonyl]-3-méthyl-butyl}-4-thiophène-2-yl-benzamide ;
N-{3-méthyl-1-[6-oxo-4-(pipéridine-2-carbonyl)-hexahydro-pyrrolo[3,2-b]pyrrole-1-carbonyl]-butyl}-4-thiophène-2-yl-benzamide ;
N-{1-[4-(1-acétyl-pipéridine-2-carbonyl)-6-oxo-hexahydro-pyrrolo[3,2-b]pyrrole-1-carbonyl]-3-méthyl-butyl}-4-thiophène-2-yl-benzamide ;
N-{3-méthyl-1-[6-oxo-4-(tétrahydro-furanne-2-carbonyl)-hexahydro-pyrrolo[3,2-b]pyrrole-1-carbonyl]-butyl}-4-thiophène-2-yl-benzamide ;
N-{1-[4-(1-amino-cyclobutanecarbonyl)-6-oxo-hexahydro-pyrrolo[3,2-b]pyrrole-1-carbonyl]-3-méthyl-butyl}-4-thiophène-2-yl-benzamide ;
N-{1-[4-(1-amino-cyclopentanecarbonyl)-6-oxo-hexahydro-pyrrolo[3,2-b]pyrrole-1-carbonyl]-3-mêthyl-butyl}-4-thiophène-2-yl-benzamide ;
N-{1-[4-(1-amino-cyclohexanecarbonyl)-6-oxo-hexahydro-pyrrolo[3,2-b]pyrrole-1-carbonyl]-3-méthyl-butyl}-4-thiophène-2-yl-benzamide ;
N-{1-[4-(1-acétylamino-cyclobutanecarbonyl)-6-oxo-hexahydro-pyrrolo[3,2-b]pyrrole-1-carbonyl]-3-méthyl-butyl}-4-thiophène-2-yl-benzamide ;
N-{1-[4-(1-acétylamino-cyclopentanecarbonyl)-6-oxo-hexahydro-pyrrolo[3,2-b]pyrrole-1-carbonyl]-3-méthyl-butyl}-4-thiophène-2-yl-benzamide ;
N-{1-[4-(1-acétylamino-cyclohexanecarbonyl)-6-oxo-hexahydro-pyrrolo[3,2-b]pyrrole-1-carbonyl]-3-méthyl-butyl}-4-thiophène-2-yl-benzamide ;
N-{1-[4-(1-hydroxy-cyclobutanecarbonyl)-6-oxo-hexahydro-pyrrolo[3,2-b]pyrrole-1-carbonyl]-3-méthyl-butyl}-4-thiophène-2-yl-benzamide ;
N-{1-[4-(1-hydroxy-cyclopentanecarbonyl)-6-oxo-hexahydro-pyrrolo[3,2-b]pyrrole-1-carbonyl]-3-méthyl-butyl}-4-thiophène-2-yl-benzamide ;
N-{1-[4-(1-hydroxy-cyclohexanecarbonyl)-6-oxo-hexahydro-pyrrolo[3,2-b]pyrrole-1-carbonyl]-3-méthyl-butyl}-4-thiophène-2-yl-benzamide ;
N-{1-[4-(1-méthoxy-cyclobutanecarbonyl)-6-oxo-hexahydro-pyrrolo[3,2-b]pyrrole-1-carbonyl]-3-méthyl-butyl}-4-thiophène-2-yl-benzamide ;
N-{1-[4-(1-méthoxy-cyclopentanecarbonyl)-6-oxo-hexahydro-pyrrolo[3,2-b]pyrrole-1-carbonyl]-3-méthyl-butyl}-4-thiophène-2-yl-benzamide ;
N-{1-[4-(1-méthoxy-cyclohexanecarbonyl)-6-oxo-hexahydro-pyrrolo[3,2-b]pyrrole-1-carbonyl]-3-méthyl-butyl}-4-thiophène-2-yl-benzamide ;

N-{1-[4-(2-amino-2-cyclopentyl-acétyl)-6-oxo-hexahydro-pyrrolo[3,2-b]pyrrole-1-carbonyl]-3-méthyl-butyl}-4-thiophène-2-yl-benzamide ;
N-{1-[4-(2-amino-2-cyclohexyl-acétyl)-6-oxo-hexahydro-pyrrolo[3,2-b]pyrrole-1-carbonyl]-3-méthyl-butyl}-4-thiophène_2-yl-benzamide ;
N-{1-[4-(2-acétylamino-2-cyclopentyl-acétyl)-6-oxo-hexahydro-pyrrolo[3,2-b]pyrrole-1-carbonyl]-3-méthyl-butyl}-4-thiophène-2-yl-benzamide ;
N-{1-[4-(2-acétylamino-2-cyclohexyl-acétyl)-6-oxo-hexahydro-pyrrolo[3,2-b]pyrrole-1-carbonyl]-3-méthyl-butyl}-4-thiophène-2-yl-benzamide ;
N-{1-[4-(2-acétylamino-propionyl)-6-oxo-hexahydro-pyrrolo[3,2-b]pyrrole-1-carbonyl]-3-méthyl-butyl}-4-thiophène-2-yl-benzamide ;
N-{1-[4-(2-acétylamino-acétyl)-6-oxo-hexahydro-pyrrolo-[3,2-b]pyrrole-1-carbonyl]-3-méthyl-butyl}-4-thiophène-2-yl-benzamide ;
N-(1-{4-[2-(acétyl-méthyl-amino)-acétyl]-6-oxo-hexahydro-pyrrolo[3,2-b]pyrrole-1-carbonyl}-3-méthyl-butyl)-4-thiophène-2-yl-benzamide ;
N-{1-[4-(2-dimétylamino-acétyl)-6-oxo-hexahydro-pyrrolo-[3,2-b]pyrrole-1-carbonyl]-3-méthyl-butyl}-4-thiophène-2-yl-benzamide ;
N-{1-[4-(2-amino-butyryl)-6-oxo-hexahydro-pyrrolo[3,2-b]-pyrrole-1-carbonyl]-3-méthyl-butyl}-4-thiophène-2-yl-benzamide ;
N-{1-[4-(2-acétylamino-butyryl)-6-oxo-hexahydro-pyrrolo[3,2-b]pyrrole-1-carbonyl]-3-méthyl-butyl}-4-thiophène-2-yl-benzamide ;
N-{1-[4-(2-amino-3-méthyl-butyryl)-6-oxo-hexahydro-pyrrolo[3,2-b]pyrrole-1-carbonyl]-3-méthyl-butyl}-4-thiophène-2-yl-benzamide ;
N-{1-[4-(2-acétylamino-3-méthyl-butyryl)-6-oxo-hexahydro-pyrrolo[3,2-b]pyrrole-1-carbonyl]-3-méthyl-butyl}-4-thiophène-2-yl-benzamide ;
N-{1-[4-(2-amino-3,3-diméthyl-butyryl)-6-oxo-hexahydro-pyrrolo[3,2-b]pyrrole-1-carbonyl]-3-méthyl-butyl}-4-thiophène-2-yl-benzamide ;
N-{1-[4-(2-acétylamino-3,3-diméthyl-butyryl)-6-oxo-hexahydro-pyrrolo[3,2-b]pyrrole-1-carbonyl]-3-méthyl-butyl}-4-thiophène-2-yl-benzamide ;
N-{1-[4-(2-amino-3-méthyl-pentanoyl)-6-oxo-hexahydro-pyrrolo[3,2-,b]pyrrole-1-carbonyl]-3-méthyl-butyl}-4-thiophène-2-yl-benzamide ;
N-{1-[4-(2-acétylamino-3-méthyl-pentanoyl)-6-oxo-hexahydro-pyrrolo[3,2-b]pyrrole-1-carbonyl]-3-méthyl-butyl}-4-thiophène-2-yl-benzamide ;
N-[3-méthyl-1-(6-oxo-4-pentanoyl-hexahydro-pyrrolo-[3,2-b]pyrrole-1-carbonyl)-butyl]-4-thiophène-2-yl-benzamide ;
N-{3-méthyl-1-[4-(3-méthyl-pentanoyl)-6-oxo-hexahydro-pyrrolo[3,2-b]pyrrole-1-carbonyl]-butyl}-4-thiophène-2-yl-benzamide ;
N-{3-méthyl-1-[4-(4-méthyl-pentanoyl)-6-oxo-hexahydro-pyrrolo[3,2-b]pyrrole-1-carbonyl]-butyl}-4-thiophène-2-yl-benzamide ;
N-{1-[4-(2-amino-4-méthyl-pentanoyl)-6-oxo-hexahydro-pyrrolo[3,2-b]pyrrole-1-carbonyl]-3-méthyl-butyl}-4-thiophène-2-yl-benzamide ;
N-{1-[4-(2-acétylamino-4-méthyl-pentanoyl)-6-oxo-hexahydro-pyrrolo[3,2-b]pyrrole-1-carbonyl]-3-méthyl-butyl}-4-thiophène-2-yl-benzamide ;
N-{1-[4-(2-amino-4,4-diméthyl-pentanoyl)-6-oxo-hexahydro-pyrrolo[3,2-b]pyrrole-1-carbonyl]-3-méthyl-butyl}-4-thiophène-2-yl-benzamide ;
N-{1-[4-(2-acétylamino-4,4-diméthyl-pentanoyl)-6-oxo-hexahydro-pyrrolo[3,2-b]pyrrole-1-carbonyl]-3-méthyl-butyl}-4-thiophène-2-yl-benzamide ;
N-{1-[4-(2-amino-pentanoyl)-6-oxo-hexahydro-pyrrolo[3,2-jb]-pyrrole-1-carbonyl]-3-méthyl-butyl}-4-thiophène-2-yl-benzamide ;
N-{1-[4-(2-acétylamino-pentanoyl)-6-oxo-hexahydro-pyrrolo-[3,2-b]pyrrole-1-carbonyl]-3-méthyl-butyl}-4-thiophène-2-yl-benzamide ;
N-{1-[4-(2-amino-5-méthyl-hexanoyl)-6-oxo-hexahydro-pyrrolo[3,2-b]pyrrole-1-carbonyl]-3-méthyl-butyl}-4-thiophène-2-yl-benzamide ;
N-{1-[4-(2-acétylamino-5-mêthyl-hexanoyl)-6-oxo-hexahydro-pyrrolo[3,2-b]pyrrole-1-carbonyl]-3-méthyl-butyl}-4-thiophène-2-yl-benzamide ;
N-{1-[4-(2-hydroxy-3-méthyl-butyryl)-6-oxo-hexahydro-pyrrolo[3,2-b]pyrrole-1-carbonyl]-3-méthyl-butyl}-4-thiophène-2-yl-benzamide ;
N-{1-[4-(2-méthoxy-3-méthyl-butyryl)-6-oxo-hexahydro-pyrrolo[3,2-b]pyrrole-1-carbonyl]-3-méthyl-butyl}-4-thiophène-2-yl-benzamide ;

N-{1-[4-(2-hydroxy-3,3-diméthyl-butyryl)-6-oxo-hexahydro-pyrrolo[3,2-b]pyrrole-1-carbonyl]-3-méthyl-butyl}-4-thiophène-2-yl-benzamide ;
N-{1-[4-(2-méthoxy-3,3-diméthyl-butyryl)-6-oxo-hexahydro-pyrrolo[3,2-b]pyrrole-1-carbonyl]-3-méthyl-butyl}-4-thiophène-2-yl-benzamide ;
N-{1-[4-(2-hydroxy-3-méthyl-pentanoyl)-6-oxo-hexahydro-pyrrolo[3,2-b]pyrrole-1-carbonyl]-3-méthyl-butyl}-4-thiophène-2-yl-benzamide ;
N-{1-[4-(2-méthoxy-3-méthyl-pentanoyl)-6-oxo-hexahydro-pyrrolo[3,2-b]pyrrole-1-carbonyl]-3-méthyl-butyl}-4-thiophène-2-yl-benzamide ;
N-{1-[4-(2-hydroxy-4-méthyl-pentanoyl)-6-oxo-hexahydro-pyrrolo[3,2-b]pyrrole-1-carbonyl]-3-méthyl-butyl}-4-thiophène-2-yl-benzamide ;
N-{1-[4-(2-méthoxy-4-méthyl-pentanoyl)-6-oxo-hexahydro-pyrrolo[3,2-b]pyrrole-1-carbonyl]-3-méthyl-butyl}-4-thiophène-2-yl-benzamide ;
N-{1-[4-(2-hydroxy-4,4-diméthyl-pentanoyl)-6-oxo-hexahydro-pyrrolo[3,2-b]pyrrole-1-carbonyl]-3-méthyl-butyl}-4-thiophène-2-yl-benzamide ;
N-{1-[4-(2-méthoxy-4,4-diméthyl-pentanoyl)-6-oxo-hexahydro-pyrrolo[3,2-b]pyrrole-1-carbonyl]-3-méthyl-butyl}-4-thiophène-2-yl-benzamide ;
N-{1-[4-(2-hydroxy-pentanoyl)-6-oxo-hexahydro-pyrrolo-[3,2-b]pyrrole-1-carbonyl]-3-méthyl-butyl}-4-thiophêne-2-yl-benzamide ;
N-{1-[4-(2-méthoxy-pentanoyl)-6-oxo-hexahydro-pyrrolo-[3,2-b]pyrrole-1-carbonyl]-3-méthyl-butyl}-4-thiophène-2-yl-benzamide ;
N-{1-[4-(2-hydroxy-5-méthyl-hexanoyl)-6-oxo-hexahydro-pyrrolo[3,2-b]pyrrole-1-carbonyl]-3-méthyl-butyl}-4-thiophène-2-yl-benzamide ;
N-{1-[4-(2-méthoxy-5-méthyl-hexanoyl)-6-oxo-hexahydro-pyrrolo[3,2-b]pyrrole-1-carbonyl]-3-méthyl-butyl}-4-thiophène-2-yl-benzamide ;
[1-(4-benzoyl-6-oxo-hexahydro-pyrrolo[3,2-b]pyrrole-1-carbonyl)-3-méthyl-butyl]-amide d'acide 5-phényl-thiophène-2-carboxylique ;
{3-méthyl-1-[6-oxo-4-(pyridine-2-carbonyl)-hexahydro-pyrrolo[3,2-b]pyrrole-1-carbonyl]-butyl}-amide d'acide 5-phényl-thiophène-2-carboxylique ;
{3-méthyl-1-[6-oxo-4-(1-oxy-pyridine-2-carbonyl)-hexahydro-pyrrolo[3,2-b]pyrrole-1-carbonyl]-butyl}-amide d'acide 5-phényl-thiophène-2-carboxylique ;
{3-méthyl-1-[6-oxo-4-(pyridine-3-carbonyl)-hexahydro-pyrrolo[3,2-b]pyrrole-1-carbonyl]-butyl}-amide d'acide 5-phényl-thiophène-2-carboxylique ;
{3-méthyl-1-[6-oxo-4-(1-oxy-pyridine-3-carbonyl)-hexahydro-pyrrolo[3,2-b]pyrrole-1-carbonyl]-butyl}-amide d'acide 5-phényl-thiophène-2-carboxylique ;
{3-méthyl-1-[6-oxo-4-(pyridine-4-carbonyl)-hexahydro-pyrrolo[3,2-b]pyrrole-1-carbonyl]-butyl}-amide d'acide 5-phényl-thiophène-2-carboxylique ;
{3-méthyl-1-[6-oxo-4-(1-oxy-pyridine-4-carbonyl)-hexahydro-pyrrolo[3,2-b]pyrrole-1-carbonyl]-butyl}-amide d'acide 5-phényl-thiophène-2-carboxylique ;
[1-(4-benzènesulfonyl-6-oxo-hexahydro-pyrrolo[3,2-b]-pyrrole-1-carbonyl)-3-méthyl-butyl]-amide d'acide 5-phényl-thiophène-2-carboxylique ;
{3-méthyl-1-[6-oxo-4-(pyridine-2-sulfonyl)-hexahydro-pyrrolo[3,2-b]pyrrole-1-carbonyl]-butyl}-amide d'acide 5-phényl-thiophène-2-carboxylique ;
{3-méthyl-1-[6-oxo-4-(1-oxy-pyridine-2-sulfonyl)-hexahydro-pyrrolo[3,2-b]pyrrole-1-carbonyl]-butyl}-amide d'acide 5-phényl-thiophène-2-carboxylique ;
{3-méthyl-1-[6-oxo-4-(pyridine-3-sulfonyl)-hexahydro-pyrrolo[3,2-b]pyrrole-1-carbonyl]-butyl}-amide d'acide 5-phényl-thiophène-2-carboxylique ;
{3-méthyl-1-[6-oxo-4-(1-oxy-pyridine-3-sulfonyl)-hexahydro-pyrrolo[3,2-b]pyrrole-1-carbonyl]-butyl}-amide d'acide 5-phényl-thiophène-2-carboxylique ;
{3-méthyl-1-[6-oxo-4-(pyridine-4-sulfonyl)-hexahydro-pyrrolo[3,2-b]pyrrole-1-carbonyl]-butyl}-amide d'acide 5-phényl-thiophène-2-carboxylique ;
{3-méthyl-1-[6-oxo-4-(1-oxy-pyridine-4-sulfonyl)-hexahydro-pyrrolo[3,2-b]pyrrole-1-carbonyl]-butyl}-amide d'acide 5-phényl-thiophène-2-carboxylique ;
[3-méthyl-1-(6-oxo-4-phénylacétyl-hexahydro-pyrrolo[3,2-b]-pyrrole-1-carbonyl)-butyl]-amide d'acide 5-phényl-thiophène-2-carboxylique ;
phénylamide d'acide 4-{4-méthyl-2-[(5-phényl-thiophène-2-carbonyl)-amino]-pentanoyl}-3-oxo-hexahydro-pyrrolo[3,2-b]-pyrrole-1-carboxylique ;
isobutyl-amide d'acide 4-{4-méthyl-2-[(5-phényl-thiophène-2-carbonyl)-amino]-pentanoyl}-3-oxo-hexahydro-pyrrolo[3,2-b]-pyrrole-1-carboxylique ;

ester phénylique d'acide 4-{4-méthyl-2-[(5-phényl-thiophène-2-carbonyl)-amino]-pentanoyl}-3-oxo-hexahydro-pyrrolo[3,2-b]-pyrrole-1-carboxylique ;
ester isobutylique d'acide 4-{4-méthyl-2-[(5-phényl-thiophène-2-carbonyl)-amino]-pentanoyl}-3-oxo-hexahydro-pyrrolo[3,2-b]-pyrrole-1-carboxylique ;
{3-méthyl-1-[6-oxo-4-(pyrrolidine-1-carbonyl)-hexahydro-pyrrolo[3,2-b]pyrrole-1-carbonyl]-butyl}-amide d'acide 5-phényl-thiophène-2-carboxylique ;
{3-méthyl-1-[6-oxo-4-(pipéridine-1-carbonyl)-hexahydro-pyrrolo[3,2-b]pyrrole-1-carbonyl]-butyl}-amide d'acide 5-phényl-thiophène-2-carboxylique ;
{3-méthyl-1-[4-(morpholine-4-carbonyl)-6-oxo-hexahydro-pyrrolo[3,2-b]pyrrole-1-carbonyl]-butyl}-amide d'acide 5-phényl-thiophène-2-carboxylique ;
{3-méthyl-1-[6-oxo-4-(pipérazine-1-carbonyl)-hexahydro-pyrrolo[3,2-b]pyrrole-1-carbonyl]-butyl}-amide d'acide 5-phényl-thiophène-2-carboxylique ;
{3-méthyl-1-[4-(4-méthyl-pipérazine-1-carbonyl)-6-oxo-hexahydro-pyrrolo[3,2-b]pyrrole-1-carbonyl]-butyl}-amide d'acide 5-phényl-thiophène-2-carboxylique ;
(3-méthyl-1-{4-[2-(4-méthyl-pipérazine-1-yl)-acétyl]-6-oxo-hexahydro-pyrrolo[3,2-b]pyrrole-1-carbonyl}-butyl)-amide d'acide 5-phényl-thiophène-2-carboxylique ;
{3-méthyl-1-[6-oxo-4-(2-pipérazine-1-yl-acétyl)-hexahydro-pyrrolo[3,2-b]pyrrole-1-carbonyl]-butyl}-amide d'acide 5-phényl-thiophène-2-carboxylique ;
{1-[4-(furanne-2-carbonyl)-6-oxo-hexahydro-pyrrolo[3,2-b]-pyrrole-1-carbonyl]-3-méthyl-butyl}-amide d'acide 5-phényl-thiophène-2-carboxylique ;
{1-[4-(5-chloro-furanne-2-carbonyl)-6-oxo-hexahydro-pyrrolo[3,2-b]-pyrrole-1-carbonyl]-3-méthyl-butyl}-amide d'acide 5-phényl-thiophène-2-carboxylique ;
{1-[4-(thiophène-2-carbonyl)-6-oxo-hexahydro-pyrrolo-[3,2-b]pyrrole-1-carbonyl]-3-méthyl-butyl}-amide d'acide 5-phényl-thiophène-2-carboxylique ;
{1-[4-(5-chloro-thiophène-2-carbonyl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(furanne-3-carbonyl)-6-oxo-hexahydro-pyrrolo[3,2-b]-pyrrole-1-carbonyl]-3-méthyl-butyl}-amide d'acide 5-phényl-thiophène-2-carboxylique ;
{1-[4-(thiophène-3-carbonyl)-6-oxo-hexahydro-pyrrolo-[3,2-b]pyrrole-1-carbonyl]-3-méthyl-butyl}-amide d'acide 5-phényl-thiophène-2-carboxylique ;
[1-(4-cyclopentanecarbonyl-6-oxo-hexahydro-pyrrolo[3,2-b]-pyrrole-1-carbonyl)-3-méthyl-butyl]-amide d'acide 5-phényl-thiophène-2-carboxylique ;
[1-(4-cyclohexanecarbonyl-6-oxo-hexahydro-pyrrolo[3,2-b]-pyrrole-1-carbonyl)-3-méthyl-butyl]-amide d'acide 5-phényl-thiophène-2-carboxylique ;
{1-[4-(cyclopent-3-ènecarbonyl)-6-oxo-hexahydro-pyrrolo-[3,2-b]pyrrole-1-carbonyl]-3-méthyl-butyl}-amide d'acide 5-phényl-thiophène-2-carboxylique ;
{1-[4-(2-cyclopentyl-acétyl)-6-oxo-hexahydro-pyrrolo-[3,2-b]pyrrole-1-carbonyl]-3-méthyl-butyl}-amide d'acide 5-phényl-thiophène-2-carboxylique ;
{1-[4-(2-cyclohexyl-acétyl)-6-oxo-hexahydro-pyrrolo-[3,2-b]pyrrole-1-carbonyl]-3-méthyl-butyl}-amide d'acide 5-phényl-thiophène-2-carboxylique ;
{3-méthyl-1-[6-oxo-4-(pyrrolidine-2-carbonyl)-hexahydro-pyrrolo[3,2-b]pyrrole-1-carbonyl]-butyl}-amide d'acide 5-phényl-thiophène-2-carboxylique ;
{1-[4-(1-acétyl-pyrrolidine-2-carbonyl)-6-oxo-hexahydro-pyrrolo[3,2-b]pyrrole-1-carbonyl]-3-méthyl-butyl}-amide d'acide 5-phényl-thiophène-2-carboxylique ;
{3-méthyl-1-[6-oxo-4-(pipéridine-2-carbonyl)-hexahydro-pyrrolo[3,2-b]pyrrole-1-carbonyl]-butyl}-amide d'acide 5-phényl-thiophène-2-carboxylique ;
{1-[4-(1-acétyl-pipéridine-2-carbonyl)-6-oxo-hexahydro-pyrrolo[3,2-b]pyrrole-1-carbonyl]-3-méthyl-butyl}-amide d'acide 5-phényl-thiophène-2-carboxylique ;
{3-méthyl-1-[6-oxo-4-(tétrahydro-furanne-2-carbonyl)-hexahydro-pyrrolo[3,2-b]pyrrole-1-carbonyl]-butyl}-amide d'acide 5-phényl-thiophène-2-carboxylique ;
{1-[4-(1-amino-cyclobutanecarbonyl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(1-amino-cyclopentanecarbonyl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(1-amino-cyclohexanecarbonyl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(1-acétylamino-cyclobutanecarbonyl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(1-acétylamino-cyclopentanecarbonyl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(1-acétylamino-cyclohexanecarbonyl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(1-hydroxy-cyclobutanecarbonyl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(1-hydroxy-cyclopentanecarbonyl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(1-hydroxy-cyclohexanecarbonyl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(1-méthoxy-cyclobutanecarbonyl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(1-méthoxy-cyclopentanecarbonyl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(1-méthoxy-cyclohexanecarbonyl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(2-amino-2-cyclopentyl-acétyl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(2-amino-2-cyclohexyl-acétyl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(2-acétylamino-2-cyclopentyl-acétyl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(2-acétylamino-2-cyclohexyl-acétyl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(2-acétylamino-propionyl)-6-oxo-hexahydro-pyrrolo-[3,2-b]pyrrole-1-carbonyl]-3-méthyl-butyl}-amide d'acide 5-phényl-thiophène-2-carboxylique ;

{1-[4-(2-acétylamino-acétyl)-6-oxo-hexahydro-pyrrolo-[3,2-b]pyrrole-1-carbonyl]-3-méthyl-butyl}-amide d'acide 5-phényl-thiophène-2-carboxylique ;

(1-{4-[2-(acétyl-méthyl-amino)-acétyl]-6-oxo-hexahydro-pyrrolo[3,2-b]pyrrole-1-carbonyl}-3-méthyl-butyl)-amide d'acide 5-phényl-thiophène-2-carboxylique ;

{1-[4-(2-diméthylamino-acétyl)-6-oxo-hexahydro-pyrrolo-[3,2-b]pyrrole-1-carbonyl]-3-méthyl-butyl}-amide d'acide 5-phényl-thiophène-2-carboxylique ;

{1-[4-(2-amino-butyryl)-6-oxo-hexahydro-pyrrolo[3,2-b]-pyrrole-1-carbonyl]-3-méthyl-butyl}-amide d'acide 5-phényl-thiophène-2-carboxylique ;

{1-[4-(2-acétylamino-butyryl)-6-oxo-hexahydro-pyrrolo-[3,2-b]pyrrole-1-carbonyl]-3-méthyl-butyl}-amide d'acide 5-phényl-thiophène-2-carboxylique ;

{1-[4-(2-amino-3-méthyl-butyryl)-6-oxo-hexahydro-pyrrolo[3,2-b]-pyrrole-1-carbonyl]-3-méthyl-butyl}-amide d'acide 5-phényl-thiophène-2-carboxylique ;

{1-[4-(2-acétylamino-3-méthyl-butyryl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(2-amino-3,3-diméthyl-butyryl)-6-oxo-hexahydro-pyrrolo[3,2-b]-pyrrole-1-carbonyl]-3-méthyl-butyl}-amide d'acide 5-phényl-thiophène-2-carboxylique ;

{1-[4-(2-acétylamino-3,3-diméthyl-butyryl)-6-oxo-hexahydro-pyrrolo[3,2-b]-pyrrole-1-carbonyl]-3-méthyl-butyl}-amide d'acide 5-phényl-thiophène-2-carboxylique ;

{1-[4-(2-amino-3-méthyl-pentanoyl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(2-acétylamino-3-méthyl-pentanoyl)-6-oxo-hexahydro-pyrrolo[3,2-b]pyrrole-1-carbonyl]-3-méthyl-butyl}-amide d'acide 5-phényl-thiophène-2-carboxylique ;

[3-méthyl-1-(6-oxo-4-pentanoyl-hexahydro-pyrrolo[3,2-b]-pyrrole-1-carbonyl)-butyl]-amide d'acide 5-phényl-thiophène-2-carboxylique ;

{3-méthyl-1-[4-(3-méthyl-pentanoyl)-6-oxo-hexahydro-pyrrolo[3,2-b]pyrrole-1-carbonyl]-butyl}-amide d'acide 5-phényl-thiophène-2-carboxylique ;

{3-méthyl-1-[4-(4-méthyl-pentanoyl)-6-oxo-hexahydro-pyrrolo[3,2-b]pyrrole-1-carbonyl]-butyl}-amide d'acide 5-phényl-thiophène-2-carboxylique ;

{1-[4-(2-amino-4-méthyl-pentanoyl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(2-acétylamino-4-méthyl-pentanoyl)-6-oxo-hexahydro-pyrrolo[3,2-b]pyrrole-1-carbonyl]-3-méthyl-butyl}-amide d'acide 5-phényl-thiophène-2-carboxylique ;

{1-[4-(2-amino-4,4-diméthyl-pentanoyl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(2-acétylamino-4,4-diméthyl-pentanoyl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(2-amino-pentanoyl)-6-oxo-hexahydro-pyrrolo[3,2-b]-pyrrole-1-carbonyl]-3-méthyl-butyl}-amide d'acide 5-phényl-thiophène-2-carboxylique ;
{1-[4-(2-acétylamino-pentanoyl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(2-amino-5-méthyl-hexanoyl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(2-acétylamino-5-méthyl-hexanoyl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(2-hydroxy-3-méthyl-butyryl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(2-méthoxy-3-méthyl-butyryl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(2-hydroxy-3,3-diméthyl-butyryl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(2-méthoxy-3,3-diméthyl-butyryl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(2-hydroxy-3-méthyl-pentanoyl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(2-méthoxy-3-méthyl-pentanoyl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(2-hydroxy-4-méthyl-pentanoyl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(2-méthoxy-4-méthyl-pentanoyl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(2-hydroxy-4,4-diméthyl-pentanoyl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(2-méthoxy-4,4-diméthyl-pentanoyl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(2-hydroxy-pentanoyl)-6-oxo-hexahydro-pyrrolo[3,2-b]-pyrrole-1-carbonyl]-3-méthyl-butyl}-amide d'acide 5-phényl-thiophène-2-carboxylique ;
{1-[4-(2-méthoxy-pentanoyl)-6-oxo-hexahydro-pyrrolo[3,2-b]-pyrrole-1-carbonyl]-3-méthyl-butyl}-amide d'acide 5-phényl-thiophène-2-carboxylique ;
{1-[4-(2-hydroxy-5-méthyl-hexanoyl)-6-oxo-hexahydro-pyrrolo[3,2-b]pyrrole-1-carbonyl]-3-méthyl-butyl}-amide d'acide 5-phényl-thiophène-2-carboxylique ;
{1-[4-(2-méthoxy-5-méthyl-hexanoyl)-6-oxo-hexahydro-pyrrolo[3,2-b]pyrrole-1-carbonyl]-3-méthyl-butyl}-amide d'acide 5-phényl-thiophène-2-carboxylique ;
N-[1-(4-benzoyl-6-oxo-hexahydro-pyrrolo[3,2-b]pyrrole-1-carbonyl)-3-méthyl-butyl]-4-pyrrolidine-1-yl-benzamide ;
N-{3-méthyl-1-[6-oxo-4-(pyridine-2-carbonyl)-hexahydro-pyrrolo[3,2-b]pyrrole-1-carbonyl]-butyl}-4-pyrrolidine-1-yl-benzamide ;
N-{3-méthyl-1-[6-oxo-4-(1-oxy-pyridine-2-carbonyl)-hexahydro-pyrrolo[3,2-b]pyrrole-1-carbonyl]-butyl}-4-pyrrolidine-1-yl-benzamide ;
N-{3-méthyl-1-[6-oxo-4-(pyridine-3-carbonyl)-hexahydro-pyrrolo[3,2-b]pyrrole-1-carbonyl]-butyl}-4-pyrrolidine-1-yl-benzamide ;
N-{3-méthyl-1-[6-oxo-4-(1-oxy-pyridine-3-carbonyl)-hexahydro-pyrrolo[3,2-b]pyrrole-1-carbonyl]-butyl}-4-pyrrolidine-1-yl-benzamide ;
N-{3-méthyl-1-[6-oxo-4-(pyridine-4-carbonyl)-hexahydro-pyrrolo[3,2-b]pyrrole-1-carbonyl]-butyl}-4-pyrrolidine-1-yl-benzamide ;
N-{3-méthyl-1-[6-oxo-4-(1-oxy-pyridine-4-carbonyl)-hexahydro-pyrrolo[3,2-b]pyrrole-1-carbonyl]-butyl}-4-pyrrolidine-1-yl-benzamide ;
N-[1-(4-benzènesulfonyl-6-oxo-hexahydro-pyrrolo[3,2-b]-pyrrole-1-carbonyl)-3-méthyl-butyl]-4-pyrrolidine-1-yl-benzamide ;
N-{3-méthyl-1-[6-oxo-4-(pyridine-2-sulfonyl)-hexahydro-pyrrolo[3,2-b]pyrrole-1-carbonyl]-butyl}-4-pyrrolidine-1-yl-benzamide ;
N-{3-méthyl-1-[6-oxo-4-(1-oxy-pyridine-2-sulfonyl)-hexahydro-pyrrolo[3,2-b]pyrrole-1-carbonyl]-butyl}-4-pyrrolidine-1-yl-benzamide ;
N-{3-méthyl-1-[6-oxo-4-(pyridine-3-sulfonyl)-hexahydro-pyrrolo[3,2-b]pyrrole-1-carbonyl]-butyl}-4-pyrrolidine-1-yl-benzamide ;
N-{3-méthyl-1-[6-oxo-4-(1-oxy-pyridine-3-sulfonyl)-hexahydro-pyrrolo[3,2-b]pyrrole-1-carbonyl]-butyl}-4-pyrrolidine-1-yl-benzamide ;
N-{3-méthyl-1-[6-oxo-4-(pyridine-4-sulfonyl)-hexahydro-pyrrolo[3,2-b]pyrrole-1-carbonyl]-butyl}-4-pyrrolidine-1-yl-benzamide ;
N-{3-méthyl-1-[6-oxo-4-(1-oxy-pyridine-4-sulfonyl)-hexahydro-pyrrolo[3,2-b]pyrrole-1-carbonyl]-butyl}-4-pyrrolidine-1-yl-benzamide ;
N-[3-méthyl-1-(6-oxo-4-phénylacétyl-hexahydro-pyrrolo-[3,2-b]pyrrole-1-carbonyl)-butyl]-4-pyrrolidine-1-yl-benzamide ;
phénylamide d'acide 4-[2-(4-pyrrolidine-1-yl-benzoylamino)-4-méthyl-pentanoyl]-3-oxo-hexahydropyrrolo[3,2-b]pyrrole-1-carboxylique ;
isobutylamide d'acide 4-[2-(4-pyrrolidine-1-yl-benzoylamino)-4-méthyl-pentanoyl]-3-oxo-hexahydropyrrolo[3,2-b]-pyrrole-1-carboxylique ;
ester phénylique d'acide 4-[2-(4-pyrrolidine-1-yl-benzoylamino)-4-méthyl-pentanoyl]-3-oxo-hexahydro-pyrrolo[3,2-b]pyrrole-1-carboxylique ;
ester isobutylique d'acide 4-[2-(4-pyrrolidine-1-yl-benzoylamino)-4-méthyl-pentanoyl]-3-oxo-hexahydropyrrolo-[3,2-b]pyrrole-1-carboxylique ;
N-{3-méthyl-1-[6-oxo-4-(pyrrolidine-1-carbonyl)-hexahydro-pyrrolo[3,2-b]pyrrole-1-carbonyl]-butyl}-4-pyrrolidine-1-yl-benzamide ;
N-{3-méthyl-1-[6-oxo-4-(pipéridine-1-carbonyl)-hexahydro-pyrrolo[3,2-b]pyrrole-1-carbonyl]-butyl}-4-pyrrolidine-1-yl-benzamide ;
N-{3-méthyl-1-[4-(morpholine-4-carbonyl)-6-oxo-hexahydro-pyrrolo[3,2-b]pyrrole-1-carbonyl]-butyl}-4-pyrrolidine-1-yl-benzamide ;
N-{3-méthyl-1-[6-oxo-4-(pipérazine-1-carbonyl)-hexahydro-pyrrolo[3,2-b]pyrrole-1-carbonyl]-butyl}-4-pyrrolidine-1-yl-benzamide ;
N-{3-méthyl-1-[4-(4-méthyl-pipérazine-1-carbonyl)-6-oxo-hexahydro-pyrrolo[3,2-b]pyrrole-1-carbonyl]-butyl}-4-pyrrolidine-1-yl-benzamide ;
N-(3-méthyl-1-{4-[2-(4-méthyl-pipérazine-1-yl)-acétyl]-6-oxo-hexahydro-pyrrolo[3,2-b]pyrrole-1-carbonyl}-butyl)-4-pyrrolidine-1-yl-benzamide ;
N-{3-méthyl-1-[6-oxo-4-(2-pipérazine-1-yl-acétyl)-hexahydro-pyrrolo[3,2-b]pyrrole-1-carbonyl]-butyl}-4-pyrrolidine-1-yl-benzamide ;
N-{1-[4-(furanne-2-carbonyl)-6-oxo-hexahydro-pyrrolo[3,2-b]pyrrole-1-carbonyl]-3-méthyl-butyl}-4-pyrrolidine-1-yl-benzamide ;
N-{1-[4-(5-chloro-furanne-2-carbonyl)-6-oxo-hexahydro-pyrrolo[3,2-b]pyrrole-1-carbonyl]-3-méthyl-butyl}-4-pyrrolidine-1-yl-benzamide ;
N-{1-[4-(thiophène-2-carbonyl)-6-oxo-hexahydro-pyrrolo-[3,2-b]pyrrole-1-carbonyl]-3-méthyl-butyl}-4-pyrrolidine-1-yl-benzamide ;
N-{1-[4-(5-chloro-thiophène-2-carbonyl)-6-oxo-hexahydro-pyrrolo[3,2-b]pyrrole-1-carbonyl]-3-méthyl-butyl}-4-pyrrolidine-1-yl-benzamide ;
N-{1-[4-(furanne-3-carbonyl)-6-oxo-hexahydro-pyrrolo-[3,2-b]pyrrole-1-carbonyl]-3-méthyl-butyl}-4-pyrrolidine-1-yl-benzamide ;
N-{1-[4-(thiophène-3-carbonyl)-6-oxo-hexahydro-pyrrolo-[3,2-b]pyrrole-1-carbonyl]-3-méthyl-butyl}-4-pyrrolidine-1-yl-benzamide ;
N-[1-(4-cyclopentanecarbonyl-6-oxo-hexahydro-pyrrolo-[3,2-b]pyrrole-1-carbonyl)-3-méthyl-butyl]-4-pyrrolidine-1-yl-benzamide ;
N-[1-(4-cyclohexanecarbonyl-6-oxo-hexahydro-pyrrolo-[3,2-b]pyrrole-1-carbonyl)-3-méthyl-butyl]-4-pyrrolidine-1-yl-benzamide ;
N-{1-[4-(cyclopent-3-ènecarbonyl)-6-oxo-hexahydro-pyrrolo-[3,2-b]pyrrole-1-carbonyl]-3-méthyl-butyl}-4-pyrrolidine-1-yl-benzamide ;
N-{1-[4-(2-cyclopentyl-acétyl)-6-oxo-hexahydro-pyrrolo-[3,2-b]pyrrole-1-carbonyl]-3-méthyl-butyl}-4-pyrrolidine-1-yl-benzamide ;
N-{1-[4-(2-cyclohexyl-acétyl)-6-oxo-hexahydro-pyrrolo-[3,2-b]pyrrole-1-carbonyl]-3-méthyl-butyl}-4-pyrrolidine-1-yl-benzamide ;
4-pyrrolidine-1-yl-
N-{3-méthyl-1-[6-oxo-4-(pyrrolidine-2-carbonyl)-hexahydro-pyrrolo[3,2-b]pyrrole-1-carbonyl]-butyl}-benzamide ;
N-{1-[4-(1-acétyl-pyrrolidine-2-carbonyl)-6-oxo-hexahydro-pyrrolo[3,2-b]pyrrole-1-carbonyl]-3-méthyl-butyl}-4-pyrrolidine-1-yl-benzamide ;
N-{3-méthyl-1-[6-oxo-4-(pipéridine-2-carbonyl)-hexahydro-pyrrolo[3,2-b]pyrrole-1-carbonyl]-butyl}-4-pyrrolidine-1-yl-benzamide ;
N-{1-[4-(1-acétyl-pipéridine-2-carbonyl)-6-oxo-hexahydro-pyrrolo[3,2-b]pyrrole-1-carbonyl]-3-méthyl-butyl}-4-pyrrolidine-1-yl-benzamide ;
N-{3-méthyl-1-[6-oxo-4-(tétrahydro-furanne-2-carbonyl)-hexahydro-pyrrolo[3,2-b]pyrrole-1-carbonyl]-butyl}-4-pyrrolidine-1-yl-benzamide ;
N-{1-[4-(1-amino-cyclobutanecarbonyl)-6-oxo-hexahydro-pyrrolo[3,2-b]pyrrole-1-carbonyl]-3-méthyl-butyl}-4-pyrrolidine-1-yl-benzamide ;
N-{1-[4-(1-amino-cyclopentanecarbonyl)-6-oxo-hexahydro-pyrrolo[3,2-b]pyrrole-1-carbonyl]-3-méthyl-butyl}-4-pyrrolidine-1-yl-benzamide ;
N-{1-[4-(1-amino-cyclohexanecarbonyl)-6-oxo-hexahydro-pyrrolo[3,2-b]pyrrole-1-carbonyl]-3-méthyl-butyl}-4-pyrrolidine-1-yl-benzamide ;
N-{1-[4-(1-acétylamino-cyclobutanecarbonyl)-6-oxo-hexahydro-pyrrolo[3,2-b]pyrrole-1-carbonyl]-3-méthyl-butyl}-4-pyrrolidine-1-yl-benzamide ;
N-{1-[4-(1-acétylamino-cyclopentanecarbonyl)-6-oxo-hexahydro-pyrrolo[3,2-b]pyrrole-1-carbonyl]-3-méthyl-butyl}-4-pyrrolidine-1-yl-benzamide ;
N-{1-[4-(1-acétylamino-cyclohexanecarbonyl)-6-oxo-hexahydro-pyrrolo[3,2-b]pyrrole-1-carbonyl]-3-méthyl-butyl}-4-pyrrolidine-1-yl-benzamide ;
N-{1-[4-(1-hydroxy-cyclobutanecarbonyl)-6-oxo-hexahydro-pyrrolo[3,2-b]pyrrole-1-carbonyl]-3-méthyl-butyl}-4-pyrrolidine-1-yl-benzamide ;
N-{1-[4-(1-hydroxy-cyclopentanecarbonyl)-6-oxo-hexahydro-pyrrolo[3,2-b]pyrrole-1-carbonyl]-3-méthyl-butyl}-4-pyrrolidine-1-yl-benzamide ;
N-{1-[4-(1-hydroxy-cyclohexanecarbonyl)-6-oxo-hexahydro-pyrrolo[3,2-b]pyrrole-1-carbonyl]-3-méthyl-butyl}-4-pyrrolidine-1-yl-benzamide ;
N-{1-[4-(1-méthoxy-cyclobutanecarbonyl)-6-oxo-hexahydro-pyrrolo[3,2-b]pyrrole-1-carbonyl]-3-méthyl-butyl}-4-pyrrolidine-1-yl-benzamide ;
N-{1-[4-(1-méthoxy-cyclopentanecarbonyl)-6-oxo-hexahydro-pyrrolo[3,2-b]pyrrole-1-carbonyl]-3-méthyl-butyl}-4-pyrrolidine-1-yl-benzamide ;
N-{1-[4-(1-méthoxy-cyclohexanecarbonyl)-6-oxo-hexahydro-pyrrolo[3,2-b]pyrrole-1-carbonyl]-3-méthyl-butyl}-4-pyrrolidine-1-yl-benzamide ;
N-{1-[4-(2-amino-2-cyclopentyl-acétyl)-6-oxo-hexahydro-pyrrolo[3,2-b]pyrrole-1-carbonyl]-3-méthyl-butyl}-4-pyrrolidine-1-yl-benzamide ;
N-{1-[4-(2-amino-2-cyclohexyl-acétyl)-6-oxo-hexahydro-pyrrolo[3,2-b]pyrrole-1-carbonyl]-3-méthyl-butyl}-4-pyrrolidine-1-yl-benzamide ;
N-{1-[4-(2-acétylamino-2-cyclopentyl-acétyl)-6-oxo-hexahydro-pyrrolo[3,2-b]pyrrole-1-carbonyl]-3-méthyl-butyl}-4-pyrrolidine-1-yl-benzamide ;
N-{1-[4-(2-acétylamino-2-cyclohexyl-acétyl)-6-oxo-hexahydro-pyrrolo[3,2-b]pyrrole-1-carbonyl]-3-méthyl-butyl}-4-pyrrolidine-1-yl-benzamide ;
N-{1-[4-(2-acétylamino-propionyl)-6-oxo-hexahydro-pyrrolo-[3,2-b]pyrrole-1-carbonyl]-3-méthyl-butyl}-4-pyrrolidine-1-yl-benzamide ;
N-{1-[4-(2-acétylamino-acétyl)-6-oxo-hexahydro-pyrrolo-[3,2-b]pyrrole-1-carbonyl]-3-méthyl-butyl}-4-pyrrolidine-1-yl-benzamide ;
N-(1-{4-[2-(acétyl-méthyl-amino)-acétyl]-6-oxo-hexahydro-pyrrolo[3,2-b]pyrrole-1-carbonyl}-3-méthyl-butyl)-4-pyrrolidine-1-yl-benzamide ;
N-{1-[4-(2-diméthylamino-acétyl)-6-oxo-hexahydro-pyrrolo-[3,2-b]pyrrole-1-carbonyl]-3-méthyl-butyl}-4-pyrrolidine-1-yl-benzamide ;
N-{1-[4-(2-amino-butyryl)-6-oxo-hexahydro-pyrrolo[3,2-b]-pyrrole-1-carbonyl]-3-méthyl-butyl}-4-pyrrolidine-1-yl-benzamide ;
N-{1-[4-(2-acétylamino-butyryl)-6-oxo-hexahydro-pyrrolo-[3,2-b]pyrrole-1-carbonyl]-3-méthyl-butyl}-4-pyrrolidine-1-yl-benzamide ;
N-{1-[4-(2-amino-3-méthyl-butyryl)-6-oxo-hexahydro-pyrrolo-[3,2-b]pyrrole-1-carbonyl]-3-méthyl-butyl}-4-pyrrolidine-1-yl-benzamide ;
N-{1-[4-(2-acétylamino-3-méthyl-butyryl)-6-oxo-hexahydro-pyrrolo[3,2-b]pyrrole-1-carbonyl]-3-méthyl-butyl}-4-pyrrolidine-1-yl-benzamide ;
N-{1-[4-(2-amino-3,3-diméthyl-butyryl)-6-oxo-hexahydro-pyrrolo[3,2-b]pyrrole-1-carbonyl]-3-méthyl-butyl}-4-pyrrolidine-1-yl-benzamide ;
N-{1-[4-(2-acétylamino-3,3-diméthyl-butyryl)-6-oxo-hexahydro-pyrrolo[3,2-b]pyrrole-1-carbonyl]-3-méthyl-butyl}-4-pyrrolidine-1-yl-benzamide ;
N-{1-[4-(2-amino-3-méthyl-pentanoyl)-6-oxo-hexahydro-pyrrolo[3,2-b]pyrrole-1-carbonyl]-3-méthyl-butyl}-4-pyrrolidine-1-yl-benzamide ;
N-{1-[4-(2-acétylamino-3-méthyl-pentanoyl)-6-oxo-hexahydro-pyrrolo[3,2-b]pyrrole-1-carbonyl]-3-méthyl-butyl}-4-pyrrolidine-1-yl-benzamide ;
N-[3-méthyl-1-(6-oxo-4-pentanoyl-hexahydro-pyrrolo[3,2-b]-pyrrole-1-carbonyl)-butyl]-4-pyrrolidine-1-yl-benzamide ;
N-{3-méthyl-1-[4-(3-méthyl-pentanoyl)-6-oxo-hexahydro-pyrrolo[3,2-b]pyrrole-1-carbonyl]-butyl}-4-pyrrolidine-1-yl-benzamide ;
N-{3-méthyl-1-[4-(4-méthyl-pentanoyl)-6-oxo-hexahydro-pyrrolo[3,2-b]pyrrole-1-carbonyl]-butyl}-4-pyrrolidine-1-yl-benzamide ;
N-{1-[4-(2-amino-4-méthyl-pentanoyl)-6-oxo-hexahydro-pyrrolo[3,2-b]pyrrole-1-carbonyl]-3-méthyl-butyl}-4-pyrrolidine-1-yl-benzamide ;
N-{1-[4-(2-acétylamino-4-méthyl-pentanoyl)-6-oxo-hexahydro-pyrrolo[3,2-b]pyrrole-1-carbonyl]-3-méthyl-butyl}-4-pyrrolidine-1-yl-benzamide ;
N-{1-[4-(2-amino-4,4-diméthyl-pentanoyl)-6-oxo-hexahydro-pyrrolo[3,2-b]pyrrole-1-carbonyl]-3-méthyl-butyl}-4-pyrrolidine-1-yl-benzamide ;
N-{1-[4-(2-acétylamino-4,4-diméthyl-pentanoyl)-6-oxo-hexahydro-pyrrolo[3,2-b]pyrrole-1-carbonyl]-3-méthyl-butyl}-4-pyrrolidine-1-yl-benzamide ;
N-{1-[4-(2-amino-pentanoyl)-6-oxo-hexahydro-pyrrolo-[3,2-b]pyrrole-1-carbonyl]-3-méthyl-butyl}-4-pyrrolidine-1-yl -benzamide ;
N-{1-[4-(2-acétylamino-pentanoyl)-6-oxo-hexahydro-pyrrolo-[3,2-b]pyrrole-1-carbonyl]-3-méthyl-butyl}-4-pyrrolidine-1-yl-benzamide ;
N-{1-[4-(2-amino-5-méthyl-hexanoyl)-6-oxo-hexahydro-pyrrolo[3,2-b]pyrrole-1-carbonyl]-3-méthyl-butyl}-4-pyrrolidine-1-yl-benzamide ;
N-{1-[4-(2-acétylamino-5-méthyl-hexanoyl)-6-oxo-hexahydro-pyrrolo[3,2-b]pyrrole-1-carbonyl]-3-méthyl-butyl}-4-pyrrolidine-1-yl-benzamide ;
N-{1-[4-(2-hydroxy-3-méthyl-butyryl)-6-oxo-hexahydro-pyrrolo[3,2-b]pyrrole-1-carbonyl]-3-méthyl-butyl}-4-pyrrolidine-1-yl-benzamide ;
N-{1-[4-(2-méthoxy-3-méthyl-butyryl)-6-oxo-hexahydro-pyrrolo[3,2-b]pyrrole-1-carbonyl]-3-méthyl-butyl}-4-pyrrolidine-1-yl-benzamide ;
N-{1-[4-(2-hydroxy-3,3-diméthyl-butyryl)-6-oxo-hexahydro-pyrrolo[3,2-b]pyrrole-1-carbonyl]-3-méthyl-butyl}-4-pyrrolidine-1-yl-benzamide ;
N-{1-[4-(2-méthoxy-3,3-diméthyl-butyryl)-6-oxo-hexahydro-pyrrolo[3,2-b]pyrrole-1-carbonyl]-3-méthyl-butyl}-4-pyrrolidine-1-yl-benzamide ;
N-{1-[4-(2-hydroxy-3-méthyl-pentanoyl)-6-oxo-hexahydro-pyrrolo[3,2-b]pyrrole-1-carbonyl]-3-méthyl-butyl}-4-pyrrolidine-1-yl-benzamide ;
N-{1-[4-(2-méthoxy-3-méthyl-pentanoyl)-6-oxo-hexahydro-pyrrolo[3,2-b]pyrrole-1-carbonyl]-3-méthyl-butyl}-4-pyrrolidine-1-yl-benzamide ;
N-{1-[4-(2-hydroxy-3-méthyl-pentanoyl)-6-oxo-hexahydro-pyrrolo[3,2-b]pyrrole-1-carbonyl]-3-méthyl-butyl}-4-pyrrolidine-1-yl-benzamide ;
N-{1-[4-(2-méthoxy-4-méthyl-pentanoyl)-6-oxo-hexahydro-pyrrolo[3,2-b]pyrrole-1-carbonyl]-3-méthyl-butyl}-4-pyrrolidine-1-yl-benzamide ;
N-{1-[4-(2-hydroxy-4,4-diméthyl-pentanoyl)-6-oxo-hexahydro-pyrrolo[3,2-b]pyrrole-1-carbonyl]-3-méthyl-butyl}-4-pyrrolidine-1-yl-benzamide ;
N-{1-[4-(2-méthoxy-4,4-diméthyl-pentanoyl)-6-oxo-hexahydro-pyrrolo[3,2-b]pyrrole-1-carbonyl]-3-méthyl-butyl}-4-pyrrolidine-1-yl-benzamide ;
N-{1-[4-(2-hydroxy-pentanoyl)-6-oxo-hexahydro-pyrrolo-[3,2-b]pyrrole-1-carbonyl]-3-méthyl-butyl}-4-pyrrolidine-1-yl-benzamide ;
N-{1-[4-(2-méthoxy-pentanoyl)-6-oxo-hexahydro-pyrrolo-[3,2-b]pyrrole-1-carbonyl]-3-méthyl-butyl}-4-pyrrolidine-1-yl-benzamide ;
N-{1-[4-(2-hydroxy-5-méthyl-hexanoyl)-6-oxo-hexahydro-pyrrolo[3,2-b]pyrrole-1-carbonyl]-3-méthyl-butyl}-4-pyrrolidine-1-yl-benzamide ;
N-{1-[4-(2-méthoxy-5-méthyl-hexanoyl)-6-oxo-hexahydro-pyrrolo[3,2-b]pyrrole-1-carbonyl]-3-méthyl-butyl}-4-pyrrolidine-1-yl-benzamide ;
N-[1-(4-benzoyl-6-oxo-hexahydro-pyrrolo[3,2-b]pyrrole-1-carbonyl)-3-méthyl-butyl]-4-morpholine-4-yl-benzamide ;
N-{3-méthyl-1-[6-oxo-4-(1-oxy-pyridine-2-carbonyl)-hexahydro-pyrrolo[3,2-b]pyrrole-1-carbonyl]-butyl}-4-morpholine-4-yl-benzamide ;

N-{3-méthyl-1-[6-oxo-4-(pyridine-3-carbonyl)-hexahydro-pyrrolo[3,2-b]pyrrole-1-carbonyl]-butyl}-4-morpholine-4-yl-benzamide ;
N-{3-méthyl-1-[6-oxo-4-(1-oxy-pyridine-3-carbonyl)-hexahydro-pyrrolo[3,2-b]pyrrole-1-carbonyl]-butyl}-4-morpholine-4-yl-benzamide ;
N-{3-méthyl-1-[6-oxo-4-(pyridine-4-carbonyl)-hexahydro-pyrrolo[3,2-b]pyrrole-1-carbonyl]-butyl}-4-morpholine-4-yl-benzamide ;
N-{3-méthyl-1-[6-oxo-4-(1-oxy-pyridine-4-carbonyl)-hexahydro-pyrrolo[3,2-b]pyrrole-1-carbonyl]-butyl}-4-morpholine-4-yl-benzamide ;
N-[1-(4-benzènesulfonyl-6-oxo-hexahydro-pyrrolo[3,2-b]-pyrrole-1-carbonyl)-3-méthyl-butyl]-4-morpholine-4-yl-benzamide ;
N-{3-méthyl-1-[6-oxo-4-(pyridine-4-sulfonyl)-hexahydro-pyrrolo[3,2-b]pyrrole-1-carbonyl]-butyl}-4-morpholine-4-yl-benzamide ;
N-{3-méthyl-1-[6-oxo-4-(1-oxy-pyridine-4-sulfonyl)-hexahydro-pyrrolo[3,2-b]pyrrole-1-carbonyl]-butyl}-4-morpholine-4-yl-benzamide ;
N-[3-méthyl-1-(6-oxo-4-phénylacétyl-hexahydro-pyrrolo-[3,2-b]pyrrole-1-carbonyl)-butyl]-4-morpholine-4-yl-benzamide ;
phénylamide d'acide 4-[2-(4-morpholine-4-yl-benzoylamino)-4-méthyl-pentanoyl]-3-oxo-hexahydro-pyrrolo[3,2-b]pyrrole-1-carboxylique ;
isobutyl-amide d'acide 4-[2-(4-morpholine-4-yl-benzoylamino)-4-méthyl-pentanoyl]-3-oxo-hexahydro-pyrrolo[3,2-b]-pyrrole-1-carboxylique ;
ester phénylique d'acide 4-[2-(4-morpholine-4-yl-benzoylamino)-4-méthyl-pentanoyl]-3-oxo-hexahydro-pyrrolo[3,2-b]-pyrrole-1-carboxylique ;
ester isobutylique d'acide 4-[2-(4-morpholine-4-yl-benzoylamino)-4-méthyl-pentanoyl]-3-oxo-hexahydro-pyrrolo[3,2-b]-pyrrole-1-carboxylique ;
N-{3-méthyl-1-[6-oxo-4-(pyrrolidine-1-carbonyl)-hexahydro-pyrrolo[3,2-b]pyrrole-1-carbonyl]-butyl}-4-morpholine-4-yl-benzamide ;
N-{3-méthyl-1-[6-oxo-4-(pipéridine-1-carbonyl)-hexahydro-pyrrolo[3,2-b]pyrrole-1-carbonyl]-butyl}-4-morpholine-4-yl-benzamide ;
N-{3-méthyl-1-[4-(morpholine-4-carbonyl)-6-oxo-hexahydro-pyrrolo-[3,2-b]pyrrole-1-carbonyl]-butyl}-4-morpholine-4-yl-benzamide ;
N-{3-méthyl-1-[6-oxo-4-(pipérazine-1-carbonyl)-hexahydro-pyrrolo[3,2-b]pyrrole-1-carbonyl]-butyl}-4-morpholine-4-yl-benzamide ;
N-{3-méthyl-1-[4-(4-méthyl-pipérazine-1-carbonyl)-6-oxo-hexahydro-pyrrolo[3,2-b]pyrrole-1-carbonyl]-butyl}-4-morpholine-4-yl-benzamide ;
N-(3-méthyl-1-{4-[2-(4-méthyl-pipérazine-1-yl)-acétyl]-6-oxo-hexahydro-pyrrolo[3,2-b]pyrrole-1-carbonyl}-butyl)-4-morpholine-4-yl-benzamide ;
N-{3-méthyl-1-[6-oxo-4-(2-pipérazine-1-yl-acétyl)-hexahydro-pyrrolo[3,2-b]pyrrole-1-carbonyl]-butyl}-4-morpholine-4-yl-benzamide ;
N-{1-[4-(furanne-2-carbonyl)-6-oxo-hexahydro-pyrrolo-[3,2-b]pyrrole-1-carbonyl]-3-méthyl-butyl}-4-morpholine-4-yl-benzamide ;
N-{1-[4-(5-chloro-furanne-2-carbonyl)-6-oxo-hexahydro-pyrrolo[3,2-b]pyrrole-1-carbonyl]-3-méthyl-butyl}-4-morpholine-4-yl-benzamide ;
N-{1-[4-(thiophène-2-carbonyl)-6-oxo-hexahydro-pyrrolo-[3,2-b]pyrrole-1-carbonyl]-3-méthyl-butyl}-4-morpholine-4-yl-benzamide ;
N-{1-[4-(5-chloro-thiophène-2-carbonyl)-6-oxo-hexahydro-pyrrolo[3,2-b]pyrrole-1-carbonyl]-3-méthyl-butyl}-4-morpholine-4-yl-benzamide ;
N-{1-[4-(furanne-3-carbonyl)-6-oxo-hexahydro-pyrrolo-[3,2-b]pyrrole-1-carbonyl]-3-méthyl-butyl}-4-morpholine-4-yl-benzamide ;
N-{1-[4-(thiophène-3-carbonyl)-6-oxo-hexahydro-pyrrolo-[3,2-b]pyrrole-1-carbonyl]-3-méthyl-butyl}-4-morpholine-4-yl-benzamide ;
N-[1-(4-cyclopentanecarbonyl-6-oxo-hexahydro-pyrrolo-[3,2-b]pyrrole-1-carbonyl)-3-méthyl-butyl]-4-morpholine-4-yl-benzamide ;
N-[1-(4-cyclohexanecarbonyl-6-oxo-hexahydro-pyrrolo-[3,2-b]pyrrole-1-carbonyl)-3-méthyl-butyl]-4-morpholine-4-yl-benzamide ;
N-{1-[4-(cyclopent-3-ènecarbonyl)-6-oxo-hexahydro-pyrrolo-[3,2-b]pyrrole-1-carbonyl]-3-méthyl-butyl}-4-morpholine-4-yl-benzamide ;
N-{1-[4-(2-cyclopentyl-acétyl)-6-oxo-hexahydro-pyrrolo-[3,2-b]pyrrole-1-carbonyl]-3-méthyl-butyl}-4-morpholine-4-yl-benzamide ;

N-{1-[4-(2-cyclohexyl-acétyl)-6-oxo-hexahydro-pyrrolo-[3,2-b]pyrrole-1-carbonyl]-3-méthyl-butyl}-4-morpholine-4-yl-benzamide ;
4-morpholine-4-yl-N-{3-méthyl-1-[6-oxo-4-(pyrrolidine-2-carbonyl)-hexahydro-pyrrolo-[3,2-b]pyrrole-1-carbonyl]-butyl}-benzamide ;
N-{1-[4-(4-acétyl-pyrrolidine-2-carbonyl)-6-oxo-hexahydro-pyrrolo[3,2-b]pyrrole-1-carbonyl]-3-méthyl-butyl}-4-morpholine-4-yl-benzamide ;
N-{3-méthyl-1-[6-oxo-4-(pipéridine-2-carbonyl)-hexahydro-pyrrolo[3,2-b]pyrrole-1-carbonyl]-butyl}-4-morpholine-4-yl-benzamide ;
N-{1-[4-(1-acétyl-pipéridine-2-carbonyl)-6-oxo-hexahydro-pyrrolo[3,2-b]pyrrole-1-carbonyl]-3-méthyl-butyl}-4-morpholine-4-yl-benzamide ;
N-{3-méthyl-1-[6-oxo-4-(tétrahydro-furanne-2-carbonyl)-hexahydro-pyrrolo[3,2-b]pyrrole-1-carbonyl]-butyl}-4-morpholine-4-yl-benzamide ;
N-{1-[4-(1-amino-cyclobutanecarbonyl)-6-oxo-hexahydro-pyrrolo[3,2-b]pyrrole-1-carbonyl]-3-méthyl-butyl}-4-morpholine-4-yl-benzamide ;
N-{1-[4-(1-amino-cyclopentanecarbonyl)-6-oxo-hexahydro-pyrrolo[3,2-b]pyrrole-1-carbonyl]-3-méthyl-butyl}-4-morpholine-4-yl-benzamide ;
N-{1-[4-(1-amino-cyclohexanecarbonyl)-6-oxo-hexahydro-pyrrolo[3,2-b]pyrrole-1-carbonyl]-3-méthyl-butyl}-4-morpholine-4-yl-benzamide ;
N-{1-[4-(1-acétylamino-cyclobutanecarbonyl)-6-oxo-hexahydro-pyrrolo[3,2-b]pyrrole-1-carbonyl]-3-méthyl-butyl}-4-morpholine-4-yl-benzamide ;
N-{1-[4-(1-acétylamino-cyclopentanecarbonyl)-6-oxo-hexahydro-pyrrolo[3,2-b]pyrrole-1-carbonyl]-3-méthyl-butyl}-4-morpholine-4-yl-benzamide ;
N-{1-[4-(1-acétylamino-cyclohexanecarbonyl)-6-oxo-hexahydro-pyrrolo[3,2-b]pyrrole-1-carbonyl]-3-méthyl-butyl}-4-morpholine-4-yl-benzamide ;
N-{1-[4-(1-hydroxy-cyclobutanecarbonyl)-6-oxo-hexahydro-pyrrolo[3,2-b]pyrrole-1-carbonyl]-3-méthyl-butyl}-4-morpholine-4-yl-benzamide ;
N-{1-[4-(1-hydroxy-cyclopentanecarbonyl)-6-oxo-hexahydro-pyrrolo[3,2-b]pyrrole-1-carbonyl]-3-méthyl-butyl}-4-morpholine-4-yl-benzamide ;
N-{1-[4-(1-hydroxy-cyclohexanecarbonyl)-6-oxo-hexahydro-pyrrolo[3,2-b]pyrrole-1-carbonyl]-3-méthyl-butyl}-4-morpholine-4-yl-benzamide ;
N-{1-[4-(1-méthoxy-cyclobutanecarbonyl)-6-oxo-hexahydro-pyrrolo[3,2-b]pyrrole-1-carbonyl]-3-méthyl-butyl}-4-morpholine-4-yl-benzamide ;
N-{1-[4-(1-méthoxy-cyclopentanecarbonyl)-6-oxo-hexahydro-pyrrolo[3,2-b]pyrrole-1-carbonyl]-3-méthyl-butyl}-4-morpholine-4-yl-benzamide ;
N-{1-[4-(1-méthoxy-cyclohexanecarbonyl)-6-oxo-hexahydro-pyrrolo[3,2-b]pyrrole-1-carbonyl]-3-méthyl-butyl}-4-morpholine-4-yl-benzamide ;
N-{1-[4-(2-amino-2-cyclopentyl-acétyl)-6-oxo-hexahydro-pyrrolo[3,2-b]pyrrole-1-carbonyl]-3-méthyl-butyl}-4-morpholine-4-yl-benzamide ;
N-{1-[4-(2-amino-2-cyclohexyl-acétyl)-6-oxo-hexahydro-pyrrolo[3,2-b]pyrrole-1-carbonyl]-3-méthyl-butyl}-4-morpholine-4-yl-benzamide ;
N-{1-[4-(2-acétylamino-2-cyclopentyl-acétyl)-6-oxo-hexahydro-pyrrolo[3,2-b]pyrrole-1-carbonyl]-3-méthyl-butyl}-4-morpholine-4-yl-benzamide ;
N-{1-[4-(2-acétylamino-2-cyclohexyl-acétyl)-6-oxo-hexahydro-pyrrolo[3,2-b]pyrrole-1-carbonyl]-3-méthyl-butyl}-4-morpholine-4-yl-benzamide ;
N-{1-[4-(2-acétylamino-propionyl)-6-oxo-hexahydro-pyrrolo-[3,2-b]pyrrole-1-carbonyl]-3-méthyl-butyl}-4-morpholine-4-yl-benzamide ;
N-{1-[4-(2-acétylamino-acétyl)-6-oxo-hexahydro-pyrrolo-[3,2-b]pyrrole-1-carbonyl]-3-méthyl-butyl}-4-morpholine-4-yl-benzamide ;
N-(1-{4-[2-(acétyl-méthyl-amino)-acétyl]-6-oxo-hexahydro-pyrrolo[3,2-b]pyrrole-1-carbonyl}-3-méthyl-butyl)-4-morpholine-4-yl-benzamide ;
N-{1-[4-(2-diméthylamino-acétyl)-6-oxo-hexahydro-pyrrolo-[3,2-b]pyrrole-1-carbonyl]-3-méthyl-butyl}-4-morpholine-4-yl-benzamide ;
N-{1-[4-(2-amino-butyryl)-6-oxo-hexahydro-pyrrolo-[3,2-b]pyrrole-1-carbonyl]-3-méthyl-butyl}-4-morpholine-4-yl-benzamide ;
N-{1-[4-(2-acétylamino-butyryl)-6-oxo-hexahydro-pyrrolo-[3,2-b]pyrrole-1-carbonyl]-3-méthyl-butyl}-4-morpholine-4-yl-benzamide ;
N-{1-[4-(2-amino-3-méthyl-butyryl)-6-oxo-hexahydro-pyrrolo-[3,2-b]pyrrole-1-carbonyl]-3-méthyl-butyl}-4-morpholine-4-yl-benzamide ;

N-{1-[4-(2-acétylamino-3-méthyl-butyryl)-6-oxo-hexahydro-pyrrolo[3,2-b]pyrrole-1-carbonyl]-3-méthyl-butyl}-4-morpholine-4-yl-benzamide ;
N-{1-[4-(2-amino-3,3-diméthyl-butyryl)-6-oxo-hexahydro-pyrrolo-[3,2-b]pyrrole-1-carbonyl]-3-méthyl-butyl}-4-morpholine-4-yl-benzamide ;
N-{1-[4-(2-acétylamino-3,3-diméthyl-butyryl)-6-oxo-hexahydro-pyrrolo-[3,2-b]pyrrole-1-carbonyl]-3-méthyl-butyl}-4-morpholine-4-yl-benzamide ;
N-{1-[4-(2-amino-3-méthyl-pentanoyl)-6-oxo-hexahydro-pyrrolo[3,2-b]pyrrole-1-carbonyl]-3-méthyl-butyl}-4-morpholine-4-yl-benzamide ;
N-{1-[4-(2-acétylamino-3-méthyl-pentanoyl)-6-oxo-hexahydro-pyrrolo[3,2-b]pyrrole-1-carbonyl]-3-méthyl-butyl}-4-morpholine-4-yl-benzamide ;
N-[3-méthyl-1-(6-oxo-4-pentanoyl-hexahydro-pyrrolo[3,2-b]-pyrrole-1-carbonyl)-butyl]-4-morpholine-4-yl-benzamide ;
N-{3-méthyl-1-[4-(3-méthyl-pentanoyl)-6-oxo-hexahydro-pyrrolo[3,2-b]pyrrole-1-carbonyl]-butyl}-4-morpholine-4-yl-benzamide ;
N-{3-méthyl-1-[4-(4-méthyl-pentanoyl)-6-oxo-hexahydro-pyrrolo[3,2-b]pyrrole-1-carbonyl]-butyl}-4-morpholine-4-yl-benzamide ;
N-{1-[4-(2-amino-4-méthyl-pentanoyl)-6-oxo-hexahydro-pyrrolo[3,2-b]pyrrole-1-carbonyl]-3-méthyl-butyl}-4-morpholine-4-yl-benzamide ;
N-{1-[4-(2-acétylamino-4-méthyl-pentanoyl)-6-oxo-hexahydro-pyrrolo[3,2-b]pyrrole-1-carbonyl]-3-méthyl-butyl}-4-morpholine-4-yl-benzamide ;
N-{1-[4-(2-amino-4,4-diméthyl-pentanoyl)-6-oxo-hexahydro-pyrrolo[3,2-b]pyrrole-1-carbonyl]-3-méthyl-butyl}-4-morpholine-4-yl-benzamide ;
N-{1-[4-(2-acétylamino-4,4-diméthyl-pentanoyl)-6-oxo-hexahydro-pyrrolo[3,2-b]pyrrole-1-carbonyl]-3-méthyl-butyl}-4-morpholine-4-yl-benzamide ;
N-{1-[4-(2-amino-pentanoyl)-6-oxo-hexahydro-pyrrolo[3,2-b]-pyrrole-1-carbonyl]-3-méthyl-butyl}-4-morpholine-4-yl-benzamide ;
N-{1-[4-(2-acétylamino-pentanoyl)-6-oxo-hexahydro-pyrrolo[3,2-b]-pyrrole-1-carbonyl]-3-méthyl-butyl}-4-morpholine-4-yl-benzamide ;
N-{1-[4-(2-amino-5-méthyl-hexanoyl)-6-oxo-hexahydro-pyrrolo[3,2-b]pyrrole-1-carbonyl]-3-méthyl-butyl}-4-morpholine-4-yl-benzamide ;
N-{1-[4-(2-acétylamino-5-méthyl-hexanoyl)-6-oxo-hexahydro-pyrrolo[3,2-b]pyrrole-1-carbonyl]-3-méthyl-butyl}-4-morpholine-4-yl-benzamide ;
N-{1-[4-(2-hydroxy-3-méthyl-butyryl)-6-oxo-hexahydro-pyrrolo[3,2-b]pyrrole-1-carbonyl]-3-méthyl-butyl}-4-morpholine-4-yl-benzamide ;
N-{1-[4-(2-méthoxy-3-méthyl-butyryl)-6-oxo-hexahydro-pyrrolo[3,2-b]pyrrole-1-carbonyl]-3-méthyl-butyl}-4-morpholine-4-yl-benzamide ;
N-{1-[4-(2-hydroxy-3,3-diméthyl-butyryl)-6-oxo-hexahydro-pyrrolo[3,2-b]pyrrole-1-carbonyl]-3-méthyl-butyl}-4-morpholine-4-yl-benzamide ;
N-{1-[4-(2-méthoxy-3,3-diméthyl-butyryl)-6-oxo-hexahydro-pyrrolo[3,2-b]pyrrole-1-carbonyl]-3-méthyl-butyl}-4-morpholine-4-yl-benzamide ;
N-{1-[4-(2-hydroxy-3-méthyl-pentanoyl)-6-oxo-hexahydro-pyrrolo[3,2-b]pyrrole-1-carbonyl]-3-méthyl-butyl}-4-morpholine-4-yl-benzamide ;
N-{1-[4-(2-méthoxy-3-méthyl-pentanoyl)-6-oxo-hexahydro-pyrrolo[3,2-b]pyrrole-1-carbonyl]-3-méthyl-butyl}-4-morpholine-4-yl-benzamide ;
N-{1-[4-(2-hydroxy-4-méthyl-pentanoyl)-6-oxo-hexahydro-pyrrolo[3,2-b]pyrrole-1-carbonyl]-3-méthyl-butyl}-4-morpholine-4-yl-benzamide ;
N-{1-[4-(2-méthoxy-4-méthyl-pentanoyl)-6-oxo-hexahydro-pyrrolo[3,2-b]pyrrole-1-carbonyl]-3-méthyl-butyl}-4-morpholine-4-yl-benzamide ;
N-{1-[4-(2-hydroxy-4,4-diméthyl-pentanoyl)-6-oxo-hexahydro-pyrrolo[3,2-b]pyrrole-1-carbonyl]-3-méthyl-butyl}-4-morpholine-4-yl-benzamide ;
N-{1-[4-(2-méthoxy-4,4-diméthyl-pentanoyl)-6-oxo-hexahydro-pyrrolo[3,2-b]pyrrole-1-carbonyl]-3-méthyl-butyl}-4-morpholine-4-yl-benzamide ;
N-{1-[4-(2-hydroxy-pentanoyl)-6-oxo-hexahydro-pyrrolo-[3,2-b]pyrrole-1-carbonyl]-3-méthyl-butyl}-4-morpholine-4-yl-benzamide ;
N-{1-[4-(2-méthoxy-pentanoyl)-6-oxo-hexahydro-pyrrolo-[3,2-b]pyrrole-1-carbonyl]-3-méthyl-butyl}-4-morpholine-4-yl-benzamide ;
N-{1-[4-(2-hydroxy-5-méthyl-hexanoyl)-6-oxo-hexahydro-pyrrolo[3,2-b]pyrrole-1-carbonyl]-3-méthyl-butyl}-4-morpholine-4-yl-benzamide ;

N-{1-[4-(2-méthoxy-5-méthyl-hexanoyl)-6-oxo-hexahydro-pyrrolo[3,2-b]pyrrole-1-carbonyl]-3-méthyl-butyl}-4-morpholine-4-yl-benzamide ;
N-[1-(4-benzoyl-6-oxo-hexahydro-pyrrolo[3,2-b]pyrrole-1-carbonyl)-3-méthyl-butyl]-4-pipérazine-1-yl-benzamide ;
N-{3-méthyl-1-[6-oxo-4-(pyridine-2-carbonyl)-hexahydro-pyrrolo[3,2-b]pyrrole-1-carbonyl]-butyl}-4-pipérazine-1-yl-benzamide ;
N-{3-méthyl-1-[6-oxo-4-(1-oxy-pyridine-2-carbonyl)-hexahydro-pyrrolo[3,2-b]pyrrole-1-carbonyl]-butyl}-4-pipérazine-1-yl-benzamide ;
N-{3-méthyl-1-[6-oxo-4-(pyridine-4-carbonyl)-hexahydro-pyrrolo[3,2-b]pyrrole-1-carbonyl]-butyl}-4-pipérazine-1-yl-benzamide ;
N-{3-méthyl-1-[6-oxo-4-(1-oxy-pyridine-3-carbonyl)-hexahydro-pyrrolo[3,2-b]pyrrole-1-carbonyl]-butyl}-4-pipérazine-1-yl-benzamide ;
N-{3-méthyl-1-[6-oxo-4-(pyridine-3-carbonyl)-hexahydro-pyrrolo[3,2-b]pyrrole-1-carbonyl]-butyl}-4-pipérazine-1-yl-benzamide ;
N-{3-méthyl-1-[6-oxo-4-(1-oxy-pyridine-4-carbonyl)-hexahydro-pyrrolo[3,2-b]pyrrole-1-carbonyl]-butyl}-4-pipérazine-1-yl-benzamide ;
N-[1-(4-benzènesulfonyl-6-oxo-hexahydro-pyrrolo[3,2-b]-pyrrole-1-carbonyl)-3-méthyl-butyl]-4-pipérazine-1-yl-benzamide ;
N-{3-méthyl-1-[6-oxo-4-(pyridine-2-sulfonyl)-hexahydro-pyrrolo[3,2-b]pyrrole-1-carbonyl]-butyl}-4-pipérazine-1-yl-benzamide ;
N-{3-méthyl-1-[6-oxo-4-(1-oxy-pyridine-2-sulfonyl)-hexahydro-pyrrolo[3,2-b]pyrrole-1-carbonyl]-butyl}-4-pipérazine-1-yl-benzamide ;
N-{3-méthyl-1-[6-oxo-4-(pyridine-3-sulfonyl)-hexahydro-pyrrolo[3,2-b]pyrrole-1-carbonyl]-butyl}-4-pipérazine-1-yl-benzamide ;
N-{3-méthyl-1-[6-oxo-4-(1-oxy-pyridine-3-sulfonyl)-hexahydro-pyrrolo[3,2-b]pyrrole-1-carbonyl]-butyl}-4-pipérazine-1-yl-benzamide ;
N-{3-méthyl-1-[6-oxo-4-(pyridine-4-sulfonyl)-hexahydro-pyrrolo[3,2-b]pyrrole-1-carbonyl]-butyl}-4-pipérazine-1-yl-benzamide ;
N-{3-méthyl-1-[6-oxo-4-(1-oxy-pyridine-4-sulfonyl)-hexahydro-pyrrolo[3,2-b]pyrrole-1-carbonyl]-butyl}-4-pipérazine-1-yl-benzamide ;
N-[3-méthyl-1-(6-oxo-4-phénylacétyl-hexahydro-pyrrolo-[3,2-b]pyrrole-1-carbonyl)-butyl]-4-pipérazine-1-yl-benzamide ;
phénylamide d'acide 4-[2-(4-pipérazine-1-yl-benzoylamino)-4-méthyl-pentanoyl]-3-oxo-hexahydro-pyrrolo[3,2-b]pyrrole-1-carboxylique ;
isobutylamide d'acide 4-[2-(4-pipérazine-1-yl-benzoylamino)-4-méthyl-pentanoyl]-3-oxo-hexahydro-pyrrolo[3,2-b]-pyrrole-1-carboxylique ;
ester phénylique d'acide 4-[2-(4-pipérazine-1-yl-benzoylamino)-4-méthyl-pentanoyl]-3-oxo-hexahydro-pyrrolo[3,2-b]-pyrrole-1-carboxylique ;
ester isobutylique d'acide 4-[2-(4-pipérazine-1-yl-benzoylamino)-4-méthyl-pentanoyl]-3-oxo-hexahydro-pyrrolo[3,2-b]pyrrole-1-carboxylique ;
N-{3-méthyl-1-[6-oxo-4-(pyrrolidine-1-carbonyl)-hexahydro-pyrrolo[3,2-b]pyrrole-1-carbonyl]-butyl}-4-pipérazine-1-yl-benzamide ;
N-{3-méthyl-1-[6-oxo-4-(pipéridine-1-carbonyl)-hexahydro-pyrrolo[3,2-b]pyrrole-1-carbonyl]-butyl}-4-pipérazine-1-yl-benzamide ;
N-{3-méthyl-1-[4-(morpholine-4-carbonyl)-6-oxo-hexahydro-pyrrolo[3,2-b]pyrrole-1-carbonyl]-butyl}-4-pipérazine-1-yl-benzamide ;
N-{3-méthyl-1-[6-oxo-4-(pipérazine-1-carbonyl)-hexahydro-pyrrolo[3,2-b]pyrrole-1-carbonyl]-butyl}-4-pipérazine-1-yl-benzamide ;
N-{3-méthyl-1-[4-(4-méthyl-pipérazine-1-carbonyl)-6-oxo-hexahydro-pyrrolo[3,2-b]pyrrole-1-carbonyl]-butyl}-4-pipérazine-1-yl-benzamide ;
N-(3-méthyl-1-{4-[2-(4-méthyl-pipérazine-1-yl)-acétyl]-6-oxo-hexahydro-pyrrolo[3,2-b]pyrrole-1-carbonyl}-butyl)-4-pipérazine-1-yl-benzamide ;
N-{3-méthyl-1-[6-oxo-4-(2-pipérazine-1-yl-acétyl)-hexahydro-pyrrolo[3,2-b]pyrrole-1-carbonyl]-butyl}-4-pipérazine-1-yl-benzamide ;
N-{1-[4-(furanne-2-carbonyl)-hexahydro-pyrrolo[3,2-b]-pyrrole-1-carbonyl]-3-méthyl-butyl}-4-pipérazine-1-yl-benzamide ;
N-{1-[4-(5-chloro-furanne-2-carbonyl)-6-oxo-hexahydro-pyrrolo[3,2-b]pyrrole-1-carbonyl]-3-méthyl-butyl}-4-pipérazine-1-yl-benzamide ;
N-{1-[4-(thiophène-2-carbonyl)-6-oxo-hexahydro-pyrrolo-[3,2-b]pyrrole-1-carbonyl]-3-méthyl-butyl}-4-pipérazine-1-yl-benzamide ;
N-{1-[4-(5-chloro-thiophène-2-carbonyl)-6-oxo-hexahydro-pyrrolo[3,2-b]pyrrole-1-carbonyl]-3-méthyl-butyl}-4-pipérazine-1-yl-benzamide ;
N-{1-[4-(furanne-3-carbonyl)-6-oxo-hexahydro-pyrrolo-[3,2-b]pyrrole-1-carbonyl]-3-méthyl-butyl}-4-pipérazine-1-yl-benzamide ;
N-{1-[4-(thiophène-3-carbonyl)-6-oxo-hexahydro-pyrrolo-[3,2-b]pyrrole-1-carbonyl]-3-méthyl-butyl}-4-pipérazine-1-yl-benzamide ;
N-[1-(4-cyclopentanecarbonyl-6-oxo-hexahydro-pyrrolo-[3,2-b]pyrrole-1-carbonyl)-3-méthyl-butyl]-4-pipérazine-1-yl-benzamide ;
N-[1-(4-cyclohexanecarbonyl-6-oxo-hexahydro-pyrrolo-[3,2-b]pyrrole-1-carbonyl)-3-méthyl-butyl]-4-pipérazine-1-yl-benzamide ;
N-{1-[4-(cyclopent-3-ènecarbonyl)-6-oxo-hexahydro-pyrrolo-[3,2-b]pyrrole-1-carbonyl]-3-méthyl-butyl}-4-pipérazine-1-yl-benzamide ;
N-{1-[4-(2-cyclopentyl-acétyl)-6-oxo-hexahydro-pyrrolo-[3,2-b]pyrrole-1-carbonyl]-3-méthyl-butyl}-4-pipérazine-1-yl-benzamide ;
N-{1-[4-(2-cyclohexyl-acétyl)-6-oxo-hexahydro-pyrrolo-[3,2-b]pyrrole-1-carbonyl]-3-méthyl-butyl}-4-pipérazine-1-yl-benzamide ;
4-pipérazine-1-yl-N-{3-méthyl-1-[6-oxo-4-(pyrrolidine-2-carbonyl)-hexahydro-pyrrolo[3,2-b]pyrrole-1-carbonyl]-butyl}-benzamide ;
N-{1-[4-(1-acétyl-pyrrolidine-2-carbonyl)-6-oxo-hexahydro-pyrrolo[3,2-b]pyrrole-1-carbonyl]-3-méthyl-butyl}-4-pipérazine-1-yl-benzamide ;
N-{3-méthyl-1-[6-oxo-4-(pipéridine-2-carbonyl)-hexahydro-pyrrolo[3,2-b]pyrrole-1-carbonyl]-butyl}-4-pipérazine-1-yl-benzamide ;
N-{1-[4-(1-acétyl-pipéridine-2-carbonyl)-6-oxo-hexahydro-pyrrolo[3,2-b]pyrrole-1-carbonyl]-3-méthyl-butyl}-4-pipérazine-1-yl-benzamide ;
N-{3-méthyl-1-[6-oxo-4-(tétrahydrofuranne-2-carbonyl)-hexahydro-pyrrolo[3,2-b]pyrrole-1-carbonyl]-butyl}-4-pipérazine-1-yl-benzamide ;
N-{1-[4-(1-amino-cyclobutanecarbonyl)-6-oxo-hexahydro-pyrrolo[3,2-b]pyrrole-1-carbonyl]-3-méthyl-butyl}-4-pipérazine-1-yl-benzamide ;
N-{1-[4-(1-amino-cyclopentanecarbonyl)-6-oxo-hexahydro-pyrrolo[3,2-b]pyrrole-1-carbonyl]-3-méthyl-butyl}-4-pipérazine-1-yl-benzamide ;
N-{1-[4-(1-amino-cyclohexanecarbonyl)-6-oxo-hexahydro-pyrrolo[3,2-b]pyrrole-1-carbonyl]-3-méthyl-butyl}-4-pipérazine-1-yl-benzamide ;
N-{1-[4-(1-acétylamino-cyclobutanecarbonyl)-6-oxo-hexahydro-pyrrolo[3,2-b]pyrrole-1-carbonyl]-3-méthyl-butyl}-4-pipérazine-1-yl-benzamide ;
N-{1-[4-(1-acétylamino-cyclopentanecarbonyl)-6-oxo-hexahydro-pyrrolo[3,2-b]pyrrole-1-carbonyl]-3-méthyl-butyl}-4-pipérazine-1-yl-benzamide ;
N-{1-[4-(1-acétylamino-cyclohexanecarbonyl)-6-oxo-hexahydro-pyrrolo[3,2-b]pyrrole-1-carbonyl]-3-méthyl-butyl}-4-pipérazine-l-yl-benzamide ;
N-{1-[4-(1-hydroxy-cyclobutanecarbonyl)-6-oxo-hexahydro-pyrrolo[3,2-b]pyrrole-1-carbonyl]-3-méthyl-butyl}-4-pipérazine-1-yl-benzamide ;
N-{1-[4-(1-hydroxy-cyclopentanecarbonyl)-6-oxo-hexahydro-pyrrolo[3,2-b]pyrrole-1-carbonyl]-3-méthyl-butyl}-4-pipérazine-1-yl-benzamide ;
N-{1-[4-(1-hydroxy-cyclohexanecarbonyl)-6-oxo-hexahydro-pyrrolo[3,2-b]pyrrole-1-carbonyl]-3-méthyl-butyl}-4-pipérazine-1-yl-benzamide ;
N-{1-[4-(1-méthoxy-cyclobutanecarbonyl)-6-oxo-hexahydro-pyrrolo[3,2-b]pyrrole-1-carbonyl]-3-méthyl-butyl}-4-pipérazine-1-yl-benzamide ;
N-{1-[4-(1-méthoxy-cyclopentanecarbonyl)-6-oxo-hexahydro-pyrrolo[3,2-b]pyrrole-1-carbonyl]-3-méthyl-butyl}-4-pipérazine-1-yl-benzamide ;
N-{1-[4-(1-méthoxy-cyclohexanecarbonyl)-6-oxo-hexahydro-pyrrolo[3,2-b]pyrrole-1-carbonyl]-3-méthyl-butyl}-4-pipérazine-1-yl-benzamide ;
N-{1-[4-(2-amino-2-cyclopentyl-acétyl)-6-oxo-hexahydro-pyrrolo[3,2-b]pyrrole-1-carbonyl]-3-méthyl-butyl}-4-pipérazine-1-yl-benzamide ;
N-{1-[4-(2-amino-2-cyclohexyl-acétyl)-6-oxo-hexahydro-pyrrolo[3,2-b]pyrrole-1-carbonyl]-3-méthyl-butyl}-4-pipérazine-1-yl-benzamide ;
N-{1-[4-(2-acétylamino-2-cyclopentyl-acétyl)-6-oxo-hexahydro-pyrrolo[3,2-b]pyrrole-1-carbonyl]-3-méthyl-butyl}-4-pipérazine-l-yl-benzamide ;
N-{1-[4-(2-acétylamino-2-cyclohexyl-acétyl)-6-oxo-hexahydro-pyrrolo[3,2-b]pyrrole-1-carbonyl]-3-méthyl-butyl}-4-pipérazine-1-yl-benzamide ;
N-{1-[4-(2-acétylamino-propionyl)-6-oxo-hexahydro-pyrrolo[3,2-b]pyrrole-1-carbonyl]-3-méthyl-butyl}-4-pipérazine-1-yl-benzamide ;
N-{1-[4-(2-acétylamino-acétyl)-6-oxo-hexahydro-pyrrolo-[3,2-b]pyrrole-1-carbonyl]-3-méthyl-butyl}-4-pipérazine-1-yl-benzamide ;
N-(1-{4-[2-(acétyl-méthyl-amino)-acétyl]-6-oxo-hexahydro-pyrrolo[3,2-b]pyrrole-1-carbonyl}-3-méthyl-butyl)-4-pipérazine-1-yl-benzamide ;
N-{1-[4-(2-diméthylamino-acétyl)-6-oxo-hexahydro-pyrrolo-[3,2-b]pyrrole-1-carbonyl]-3-méthyl-butyl}-4-pipérazine-1-yl-benzamide ;
N-{1-[4-(2-amino-butyryl)-6-oxo-hexahydro-pyrrolo[3,2-b]-pyrrole-1-carbonyl]-3-méthyl-butyl}-4-pipérazine-1-yl-benzamide ;
N-{1-[4-(2-acétylamino-butyryl)-6-oxo-hexahydro-pyrrolo-[3,2-b]-pyrrole-1-carbonyl]-3-méthyl-butyl}-4-pipérazine-1-yl-benzamide ;
N-{1-[4-(2-amino-3-méthyl-butyryl)-6-oxo-hexahydro-pyrrolo[3,2-b]pyrrole-1-carbonyl]-3-méthyl-butyl}-4-pipérazine-1-yl-benzamide ;
N-{1-[4-(2-acétylamino-3-méthyl-butyryl)-6-oxo-hexahydro-pyrrolo[3,2-b]pyrrole-1-carbonyl]-3-méthyl-butyl}-4-pipérazine-1-yl-benzamide ;
N-{1-[4-(2-amino-3,3-diméthyl-butyryl)-6-oxo-hexahydro-pyrrolo[3,2-b]pyrrole-1-carbonyl]-3-méthyl-butyl}-4-pipérazine-1-yl-benzamide ;
N-{1-[4-(2-acétylamino-3,3-diméthyl-butyryl)-6-oxo-hexahydro-pyrrolo[3,2-b]pyrrole-1-carbonyl]-3-méthyl-butyl}-4-pipérazine-1-yl-benzamide ;
N-{1-[4-(2-amino-3-méthyl-pentanoyl)-6-oxo-hexahydro-pyrrolo[3,2-b]pyrrole-1-carbonyl]-3-méthyl-butyl}-4-pipérazine-1-yl-benzamide ;
N-{1-[4-(2-acétylamino-3-méthyl-pentanoyl)-6-oxo-hexahydro-pyrrolo[3,2-b]pyrrole-1-carbonyl]-3-méthyl-butyl}-4-pipérazine-1-yl-benzamide ;
N-[3-méthyl-1-(6-oxo-4-pentanoyl-hexahydro-pyrrolo[3,2-b]-pyrrole-1-carbonyl)-butyl]-4-pipérazine-1-yl-benzamide ;
N-{3-méthyl-1-[4-(3-méthyl-pentanoyl)-6-oxo-hexahydro-pyrrolo[3,2-b]pyrrole-1-carbonyl]-butyl}-4-pipérazine-1-yl-benzamide ;
N-{3-méthyl-1-[4-(4-méthyl-pentanoyl)-6-oxo-hexahydro-pyrrolo[3,2-b]pyrrole-1-carbonyl]-butyl}-4-pipérazine-1-yl-benzamide ;
N-{1-[4-(2-amino-4-méthyl-pentanoyl)-6-oxo-hexahydro-pyrrolo[3,2-b]pyrrole-1-carbonyl]-3-méthyl-butyl}-4-pipérazine-1-yl-benzamide ;
N-{1-[4-(2-acétylamino-4-méthyl-pentanoyl)-6-oxo-hexahydro-pyrrolo[3,2-b]pyrrole-1-carbonyl]-3-méthyl-butyl}-4-pipérazine-1-yl-benzamide ;
N-{1-[4-(2-amino-4,4-diméthyl-pentanoyl)-6-oxo-hexahydro-pyrrolo[3,2-b]pyrrole-1-carbonyl]-3-méthyl-butyl}-4-pipérazine-1-yl-benzamide ;
N-{1-[4-(2-acétylamino-4,4-diméthyl-pentanoyl)-6-oxo-hexahydro-pyrrolo[3,2-b]pyrrole-1-carbonyl]-3-méthyl-butyl}-4-pipérazine-1-yl-benzamide ;
N-{1-[4-(2-amino-pentanoyl)-6-oxo-hexahydro-pyrrolo-[3,2-b]pyrrole-1-carbonyl]-3-méthyl-butyl}-4-pipérazine-1-yl-benzamide ;
N-{1-[4-(2-acétylamino-pentanoyl)-6-oxo-hexahydro-pyrrolo-[3,2-b]pyrrole-1-carbonyl]-3-méthyl-butyl}-4-pipérazine-1-yl-benzamide ;
N-{1-[4-(2-amino-5-méthyl-hexanoyl)-6-oxo-hexahydro-pyrrolo[3,2-b]pyrrole-1-carbonyl]-3-méthyl-butyl}-4-pipérazine-1-yl-benzamide ;
N-{1-[4-(2-acétylamino-5-méthyl-hexanoyl)-6-oxo-hexahydro-pyrrolo[3,2-b]pyrrole-1-carbonyl]-3-méthyl-butyl}-4-pipérazine-1-yl-benzamide ;
N-{1-[4-(2-hydroxy-3-méthyl-butyryl)-6-oxo-hexahydro-pyrrolo[3,2-b]pyrrole-1-carbonyl]-3-méthyl-butyl}-4-pipérazine-1-yl-benzamide ;
N-{1-[4-(2-méthoxy-3-méthyl-butyryl)-6-oxo-hexahydro-pyrrolo[3,2-b]pyrrole-1-carbonyl]-3-méthyl-butyl}-4-pipérazine-1-yl-benzamide ;
N-{1-[4-(2-hydroxy-3,3-diméthyl-butyryl)-6-oxo-hexahydro-pyrrolo[3,2-b]pyrrole-1-carbonyl]-3-méthyl-butyl}-4-pipérazine-1-yl-benzamide ;
N-{1-[4-(2-méthoxy-3,3-diméthyl-butyryl)-6-oxo-hexahydro-pyrrolo[3,2-b]pyrrole-1-carbonyl]-3-méthyl-butyl}-4-pipérazine-1-yl-benzamide ;
N-{1-[4-(2-hydroxy-3-méthyl-pentanoyl)-6-oxo-hexahydro-pyrrolo[3,2-b]pyrrole-1-carbonyl]-3-méthyl-butyl}-4-pipérazine-1-yl-benzamide ;
N-{1-[4-(2-méthoxy-3-méthyl-pentanoyl)-6-oxo-hexahydro-pyrrolo[3,2-b]pyrrole-1-carbonyl]-3-méthyl-butyl}-4-pipérazine-1-yl-benzamide ;
N-{1-[4-(2-hydroxy-4-méthyl-pentanoyl)-6-oxo-hexahydro-pyrrolo[3,2-b]pyrrole-1-carbonyl]-3-méthyl-butyl}-4-pipérazine-1-yl-benzamide ;
N-{1-[4-(2-méthoxy-4-méthyl-pentanoyl)-6-oxo-hexahydro-pyrrolo[3,2-b]pyrrole-1-carbonyl]-3-méthyl-butyl}-4-pipérazine-1-yl-benzamide ;
N-{1-[4-(2-hydroxy-4,4-diméthyl-pentanoyl)-6-oxo-hexahydro-pyrrolo[3,2-b]pyrrole-1-carbonyl]-3-méthyl-butyl}-4-pipérazine-1-yl-benzamide ;
N-{1-[4-(2-méthoxy-4,4-diméthyl-pentanoyl)-6-oxo-hexahydro-pyrrolo[3,2-b]pyrrole-1-carbonyl]-3-méthyl-butyl}-4-pipérazine-1-yl-benzamide ;
N-{1-[4-(2-hydroxy-pentanoyl)-6-oxo-hexahydro-pyrrolo-[3,2-b]pyrrole-1-carbonyl]-3-méthyl-butyl}-4-pipérazine-1-yl-benzamide ;
N-{1-[4-(2-méthoxy-pentanoyl)-6-oxo-hexahydro-pyrrolo-[3,2-b]pyrrole-1-carbonyl]-3-méthyl-butyl}-4-pipérazine-1-yl-benzamide ;
N-{1-[4-(2-hydroxy-5-méthyl-hexanoyl)-6-oxo-hexahydro-pyrrolo[3,2-b]pyrrole-1-carbonyl]-3-méthyl-butyl}-4-pipérazine-1-yl-benzamide ;
N-{1-[4-(2-méthoxy-5-méthyl-hexanoyl)-6-oxo-hexahydro-pyrrolo[3,2-b]pyrrole-1-carbonyl]-3-méthyl-butyl}-4-pipérazine-1-yl-benzamide ;
N-[1-(4-benzoyl-6-oxo-hexahydro-pyrrolo[3,2-b]-pyrrole-1-carbonyl)-3-méthyl-butyl]-4-(4-méthyl-pipérazine-1-yl)-benzamide ;
N-{3-méthyl-1-[6-oxo-4-(1-oxy-pyridine-2-carbonyl)-hexahydro-pyrrolo[3,2-b]pyrrole-1-carbonyl]-butyl}-4-(4-méthyl-pipérazine-1-yl)-benzamide ;
N-{3-méthyl-1-[6-oxo-4-(pyridine-3-carbonyl)-hexahydro-pyrrolo[3,2-b]pyrrole-1-carbonyl]-butyl}-4-(4-méthyl-pipérazine-1-yl)-benzamide ;
N-{3-méthyl-1-[6-oxo-4-(1-oxy-pyridine-3-carbonyl)-hexahydro-pyrrolo[3,2-b]pyrrole-1-carbonyl]-butyl}-4-(4-méthyl-pipérazine-1-yl)-benzamide ;
N-{3-méthyl-1-[6-oxo-4-(pyridine-4-carbonyl)-hexahydro-pyrrolo[3,2-b]pyrrole-1-carbonyl]-butyl}-4-(4-méthyl-pipérazine-1-yl)-benzamide ;
N-{3-méthyl-1-[6-oxo-4-(1-oxy-pyridine-4-carbonyl)-hexahydro-pyrrolo[3,2-b]pyrrole-1-carbonyl]-butyl}-4-(4-méthyl-pipérazine-1-yl)-benzamide ;
N-[1-(4-benzènesulfonyl-6-oxo-hexahydro-pyrrolo[3,2-b]-pyrrole-1-carbonyl)-3-méthyl-butyl]-4-(4-méthyl-pipérazine-1-yl)-benzamide ;
N-{3-méthyl-1-[6-oxo-4-(pyridine-4-sulfonyl)-hexahydro-pyrrolo[3,2-b]pyrrole-1-carbonyl]-butyl}-4-(4-méthyl-pipérazine-1-yl)-benzamide ;
N-{3-méthyl-1-[6-oxo-4-(1-oxy-pyridine-4-sulfonyl)-hexahydro-pyrrolo[3,2-b]pyrrole-1-carbonyl]-butyl}-4-(4-méthyl-pipérazine-1-yl)-benzamide ;
N-[3-méthyl-1-(6-oxo-4-phénylacétyl-hexahydro-pyrrolo-[3,2-b]pyrrole-1-carbonyl)-butyl]-4-(4-méthyl-pipérazine-1-yl)-benzamide ; phénylamide d'acide 4-[2-(4-(4-méthyl-pipérazine-1-yl)-benzoylamino)-4-méthyl-pentanoyl)-3-oxo-hexahydro-pyrrolo[3,2- b]pyrrole-1-carboxylique ;
isobutylamide d'acide 4-[2-(4-(4-méthyl-pipérazine-1-yl)-benzoylamino)-4-méthyl-pentanoyl]-3-oxo-hexahydro-pyrrolo[3,2-b]pyrrole-1-carboxylique ;
ester phénylique d'acide 4-[2-(4-(4-méthyl-pipérazine-1-yl)-benzoylamino)-4-méthyl-pentanoyl)-3-oxo-hexahydro-pyrrolo[3,2-b]pyrrole-1-carboxylique ;
ester isobutylique d'acide 4-[2-(4-(4-méthyl-pipérazine-1-yl)-benzoylamino)-4-méthyl-pentanoyl)-3-oxo-hexahydro-pyrrolo[3,2-b]pyrrole-1-carboxylique ;
N-{3-méthyl-1-[6-oxo-4-(pyrrolidine-1-carbonyl)-hexahydro-pyrrolo[3,2-b]pyrrole-1-carbonyl]-butyl}-4-(4-méthyl-pipérazine-1-yl)-benzamide ;
N-{3-méthyl-1-[6-oxo-4-(pipéridine-1-carbonyl)-hexahydro-pyrrolo[3,2-b]pyrrole-1-carbonyl]-butyl}-4-(4-méthyl-pipérazine-1-yl)-benzamide ;
N-{3-méthyl-1-[4-(morpholine-4-carbonyl)-4-oxo-hexahydro-pyrrolo[3,2-b]pyrrole-1-carbonyl]-butyl}-4-(4-méthyl-pipérazine-1-yl)-benzamide ;
N-{3-méthyl-1-[6-oxo-4-(pipérazine-1-carbonyl)-hexahydro-pyrrolo[3,2-b]pyrrole-1-carbonyl]-butyl}-4-(4-méthyl-pipérazine-1-yl)-benzamide ;
N-{3-méthyl-1-[4-(4-méthyl-pipérazine-1-carbonyl)-6-oxo-hexahydro-pyrrolo[3,2-b]pyrrole-1-carbonyl]-butyl}-4-(4-méthyl-pipérazine-1-yl)-benzamide ;
N-(3-méthyl-1-{4-[2-(4-méthyl-pipérazine-1-yl)-acétyl]-6-oxo-hexahydro-pyrrolo[3,2-b]pyrrole-1-carbonyl}-butyl)-4-(4-méthyl-pipérazine-1-yl)-benzamide ;
N-{3-méthyl-1-[6-oxo-4-(2-pipérazine-1-yl-acétyl)-hexahydro-pyrrolo[3,2-b]pyrrole-1-carbonyl]-butyl}-4-(4-méthyl-pipérazine-1-yl)-benzamide ;

N-{1-[4-(furanne-2-carbonyl)-6-oxo-hexahydro-pyrrolo-[3,2-b]pyrrole-1-carbonyl]-3-méthyl-butyl}-4-(4-méthyl-pipérazine-1-yl)-benzamide ;
N-{1-[4-(5-chloro-furanne-2-carbonyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(thiophène-2-carbonyl)-6-oxo-hexahydro-pyrrolo-[3,2-b]pyrrole-1-carbonyl]-3-méthyl-butyl}-4-(4-méthyl-pipérazine-1-yl)-benzamide ;
N-{1-[4-(5-chloro-thiophène-2-carbonyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(furanne-3-carbonyl)-6-oxo-hexahydro-pyrrolo-[3,2-b]pyrrole-1-carbonyl]-3-méthyl-butyl}-4-(4-méthyl-pipérazine-1-yl)-benzamide ;
N-{1-[4-(thiophène-3-carbonyl)-6-oxo-hexahydro-pyrrolo-[3,2-b]pyrrole-1-carbonyl]-3-méthyl-butyl}-4-(4-méthyl-pipérazine-1-yl)-benzamide ;
N-[1-(4-cyclopentanecarbonyl-6-oxo-hexahydro-pyrrolo-[3,2-b]pyrrole-1-carbonyl)-3-méthyl-butyl]-4-(4-méthyl-pipérazine-1-yl)-benzamide ;
N-[1-(4-cyclohexanecarbonyl-6-oxo-hexahydro-pyrrolo-[3,2-b]pyrrole-1-carbonyl)-3-méthyl-butyl]-4-(4-méthyl-pipérazine-1-yl)-benzamide ;
N-{1-[4-(cyclopent-3-ènecarbonyl)-6-oxo-hexahydro-pyrrolo-[3,2-b]pyrrole-1-carbonyl]-3-méthyl-butyl}-4-(4-méthyl-pipérazine-1-yl)-benzamide ;
N-{1-[4-(2-cyclopentyl-acétyl)-6-oxo-hexahydro-pyrrolo-[3,2-b]pyrrole-1-carbonyl]-3-méthyl-butyl}-4-(4-méthyl-pipérazine-1-yl)-benzamide ;
N-{1-[4-(2-cyclohexyl-acétyl)-6-oxo-hexahydro-pyrrolo-[3,2-b]pyrrole-1-carbonyl]-3-méthyl-butyl}-4-(4-méthyl-pipérazine-1-yl)-benzamide ;
4-(4-méthyl-pipérazine-1-yl)-N-(3-méthyl-1-[6-oxo-4-(pyrrolidine-2-carbonyl)-hexahydro-pyrrolo[3,2-b]pyrrole-1-carbonyl]-butyl}-benzamide ;
N-{1-[4-(1-acétyl-pyrrolidine-2-carbonyl)-6-oxo-hexahydro-pyrrolo-[3,2-b]pyrrole-1-carbonyl]-3-méthyl-butyl}-4-(4-méthyl-pipérazine-1-yl)-benzamide ;
N-{3-méthyl-1-[6-oxo-4-(pipéridine-2-carbonyl)-hexahydro-pyrrolo[3,2-b]pyrrole-1-carbonyl]-butyl}-4-(4-méthyl-pipérazine-1-yl)-benzamide ;
N-{1-[4-(1-acétyl-pipéridine-2-carbonyl)-6-oxo-hexahydro-pyrrolo[3,2-b]pyrrole-1-carbonyl]-3-méthyl-butyl}-4-(4-méthyl-pipérazine-1-yl)-benzamide ;
N-{3-méthyl-1-[6-oxo-4-(tétrahydrofuranne-2-carbonyl)-hexahydro-pyrrolo[3,2-b]pyrrole-1-carbonyl]-butyl}-4-(4-méthyl-pipérazine-1-yl)-benzamide ;
N-{1-[4-(1-amino-cyclobutanecarbonyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(1-amino-cyclopentanecarbonyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(1-amino-cyclohexanecarbonyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(1-acétylamino-cyclobutanecarbonyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(1-acétylamino-cyclopentanecarbonyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(1-acétylamino-cyclohexanecarbonyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(1-hydroxy-cyclobutanecarbonyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(1-hydroxy-cyclopentanecarbonyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(1-hydroxy-cyclohexanecarbonyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(1-méthoxy-cyclobutanecarbonyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(1-méthoxy-cyclopentanecarbonyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(1-méthoxy-cyclohexanecarbonyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-amino-2-cyclopentyl-acétyl)-6-oxo-hexahydro-pyrrolo[3,2-b]pyrrole-1-carbonyl]-3-méthyl-butyl}-4-(4-méthyl-pipérazine-1-yl)-benzamide ;

N-{1-[4-(2-amino-2-cyclohexyl-acétyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-acétylamino-2-cyclopentyl-acétyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-acétylamino-2-cyclohexyl-acétyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-acétylamino-propionyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-acétylamino-acétyl)-6-oxo-hexahydro-pyrrolo-[3,2-b]pyrrole-1-carbonyl]-3-méthyl-butyl}-4-(4-méthyl-pipérazine-1-yl)-benzamide ;
N-(1-{4-[2-(acétyl-méthyl-amino)-acétyl]-6-oxo-hexahydro-pyrrolo[3,2-b]pyrrole-1-carbonyl}-3-méthyl-butyl)-4-(4-méthyl-pipérazine-1-yl)-benzamide ;
N-{1-[4-(2-diméthylamino-acétyl)-6-oxo-hexahydro-pyrrolo-[3,2-b]pyrrole-1-carbonyl]-3-méthyl-butyl}-4-(4-méthyl-pipérazine-1-yl)-benzamide ;
N-{1-[4-(2-amino-butyryl)-6-oxo-hexahydro-pyrrolo[3,2-b]-pyrrole-1-carbonyl]-3-méthyl-butyl}-4-(4-méthyl-pipérazine-1-yl)-benzamide ;
N-{1-[4-(2-acétylamino-butyryl)-6-oxo-hexahydro-pyrrolo-[3,2-b]pyrrole-1-carbonyl]-3-méthyl-butyl}-4-(4-méthyl-pipérazine-1-yl)-benzamide ;
N-{1-[4-(2-amino-3-méthyl-butyryl)-6-oxo-hexahydro-pyrrolo-[3,2-b]pyrrole-1-carbonyl]-3-méthyl-butyl}-4-(4-méthyl-pipérazine-1-yl)-benzamide ;
N-{1-[4-(2-acétylamino-3-méthyl-butyryl)-6-oxo-hexahydro-pyrrolo-[3,2-b]pyrrole-1-carbonyl]-3-méthyl-butyl}-4-(4-méthyl-pipérazine-1-yl)-benzamide ;
N-{1-[4-(2-amino-3,3-diméthyl-butyryl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-acétylamino-3,3-diméthyl-butyryl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-amino-3-méthyl-pentanoyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-acétylamino-3-méthyl-pentanoyl)-6-oxo-hexahydro-pyrrolo[3,2-b]pyrrole-1-carbonyl]-3-méthyl-butyl}-4-(4-méthyl-pipérazine-1-yl)-benzamide ;
N-[3-méthyl-1-(6-oxo-4-pentanoyl-hexahydro-pyrrolo[3,2-b]-pyrrole-1-carbonyl)-butyl]-4-(4-méthyl-pipérazine-1-yl)-benzamide ;
N-{3-méthyl-1-[4-(3-méthyl-pentanoyl)-6-oxo-hexahydro-pyrrolo[3,2-b]pyrrole-1-carbonyl]-butyl}-4-(4-méthyl-pipérazine-1-yl)-benzamide ;
N-{3-méthyl-1-[4-(4-méthyl-pentanoyl)-6-oxo-hexahydro-pyrrolo[3,2-b]pyrrole-1-carbonyl]-butyl}-4-(4-méthyl-pipérazine-1-yl)-benzamide ;
N-{1-[4-(2-amino-4-méthyl-pentanoyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-acétylamino-4-méthyl-pentanoyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-amino-4,4-diméthyl-pentanoyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-acétylamino-4,4-diméthyl-pentanoyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-amino-pentanoyl)-6-oxo-hexahydro-pyrrolo-[3,2-b]pyrrole-1-carbonyl]-3-méthyl-butyl}-4-(4-méthyl-pipérazine-1-yl)-benzamide ;
N-{1-[4-(2-acétylamino-pentanoyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-amino-5-méthyl-hexanoyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-acétylamino-5-méthyl-hexanoyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-hydroxy-3-méthyl-butyryl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-méthoxy-3-méthyl-butyryl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-hydroxy-3,3-diméthyl-butyryl)-6-oxo-hexahydro-pyrrolo[3,2-b]pyrrole-1-carbonyl]-3-méthyl-butyl}-4-(4-méthyl-pipérazine-1-yl)-benzamide ;

N-{1-[4-(2-méthoxy-3,3-diméthyl-butyryl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-hydroxy-3-méthyl-pentanoyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-méthoxy-3-méthyl-pentanoyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-hydroxy-4-méthyl-pentanoyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-méthoxy-4-méthyl-pentanoyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-hydroxy-4,4-diméthyl-pentanoyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-méthoxy-4,4-diméthyl-pentanoyl)-6-oxo-hexahydro-pyrrolo[3,2-b]pyrrole-1-carbonyl]-3-méthyl-butyl}-4-(4-méthyl-pipérazine-1-yl)-benzamide ;
N-{1-[4-(2-hydroxy-pentanoyl)-6-oxo-hexahydro-pyrrolo-[3,2-b]pyrrole-1-carbonyl]-3-méthyl-butyl}-4-(4-méthyl-pipérazine-1-yl)-benzamide ;
N-{1-[4-(2-méthoxy-pentanoyl)-6-oxo-hexahydro-pyrrolo-[3,2-b]pyrrole-1-carbonyl]-3-méthyl-butyl}-4-(4-méthyl-pipérazine-1-yl)-benzamide ;
N-{1-[4-(2-hydroxy-5-méthyl-hexanoyl)-6-oxo-hexahydro-pyrrolo-[3,2-b]pyrrole-1-carbonyl]-3-méthyl-butyl}-4-(4-méthyl-pipérazine-1-yl)-benzamide ;
N-{1-[4-(2-méthoxy-5-méthyl-hexanoyl)-6-oxo-hexahydro-pyrrolo-[3,2-b]pyrrole-1-carbonyl]-3-méthyl-butyl}-4-(4-méthyl-pipérazine-1-yl)-benzamide ;
N-[1-(4-benzoyl-6-oxo-hexahydro-pyrrolo[3,2-b]-pyrrole-1-carbonyl)-3-méthyl-butyl]-4-aminobenzamide ;
4-amino-N-{3-méthyl-1-[6-oxo-4-(pyridine-2-carbonyl)-hexahydro-pyrrolo[3,2-b]pyrrole-1-carbonyl]-butyl}-benzamide ;
4-amino-N-{3-méthyl-1-[6-oxo-4-(1-oxy-pyridine-2-carbonyl)-hexahydro-pyrrolo[3,2-b]pyrrole-1-carbonyl]-butyl}-benzamide ;
4-amino-N-{3-méthyl-1-[6-oxo-4-(pyridine-3-carbonyl)-hexahydro-pyrrolo[3,2-b]pyrrole-1-carbonyl]-butyl}-benzamide ;
4-amino-N-{3-méthyl-1-[6-oxo-4-(1-oxy-pyridine-3-carbonyl)-hexahydro-pyrrolo[3,2-b]pyrrole-1-carbonyl]-butyl}-benzamide ;
4-amino-N-{3-méthyl-1-[6-oxo-4-(pyridine-4-carbonyl)-hexahydro-pyrrolo[3,2-b]pyrrole-1-carbonyl]-butyl}-benzamide ;
4-amino-N-{3-méthyl-1-[6-oxo-4-(1-oxy-pyridine-4-carbonyl)-hexahydro-pyrrolo[3,2-b]pyrrole-1-carbonyl]-butyl}-benzamide ;
N-[1-(4-benzènesulfonyl-6-oxo-hexahydro-pyrrolo[3,2-b]-pyrrole-1-carbonyl)-3-méthyl-butyl]-4-aminobenzamide ;
4-amino-N-{3-méthyl-1-[6-oxo-4-(pyridine-2-sulfonyl)-hexahydro-pyrrolo[3,2-b]pyrrole-1-carbonyl]-butyl}-benzamide ;
4-amino-N-{3-méthyl-1-[6-oxo-4-(1-oxy-pyridine-2-sulfonyl)-hexahydro-pyrrolo[3,2-b]pyrrole-1-carbonyl]-butyl}-benzamide ;
4-amino-N-{3-méthyl-1-[6-oxo-4-(pyridine-3-sulfonyl)-hexahydro-pyrrolo[3,2-b]pyrrole-1-carbonyl]-butyl}-benzamide ;
4-amino-N-{3-méthyl-1-[6-oxo-4-(1-oxy-pyridine-3-sulfonyl)-hexahydro-pyrrolo[3,2-b]pyrrole-1-carbonyl]-butyl}-benzamide ;
4-amino-N-{3-méthyl-1-[6-oxo-4-(pyridine-4-sulfonyl)-hexahydro-pyrrolo[3,2-b]pyrrole-1-carbonyl]-butyl}-benzamide ;
4-amino-N-{3-méthyl-1-[6-oxo-4-(1-oxy-pyridine-4-sulfonyl)-hexahydro-pyrrolo[3,2-b]pyrrole-1-carbonyl]-butyl}-benzamide ;
4-amino-N-[3-méthyl-1(6-oxo-4-phéylacétyl-hexahydro-pyrrolo[3,2-b]pyrrole-1-carbonyl)-butyl]-benzamide ;
phénylamide d'acide 4-[2-(4-amino-benzoylamino)-4-méthyl-pentanoyl]-3-oxo-hexahydro-pyrrolo[3,2-b]pyrrole-1-carboxylique ;
isobutylamide d'acide 4-[2-(4-amino-benzoylamino)-4-méthyl-pentanoyl]-3-oxo-hexahydro-pyrrolo[3,2-b]pyrrole-1-carboxylique ;
ester phénylique d'acide 4-[2-(4-amino-benzoylamino)-4-méthyl-pentanoyl]-3-oxo-hexahydro-pyrrolo[3,2-b]pyrrole-1- carboxylique ;
ester isobutyliue d'acide 4-[2-(4-amino-benzoylamino)-4-méthyl-pentanoyl]-3-oxo-hexahydro-pyrrolo[3,2-b]pyrrole-1- carboxylique ;
4-amino-N-{3-méthyl-1-[6-oxo-4-(pyrrolidine-1-carbonyl)-hexahydro-pyrrolo[3,2-b]pyrrole-1-carbonyl]-butyl}-

benzamide ;
4-amino-N-{3-méthyl-1-[6-oxo-4-(pipéridine-1-carbonyl)-hexahydro-pyrrolo[3,2-b]pyrrole-1-carbonyl]-butyl}-benzamide ;
4-amino-N-{3-méthyl-1-[4-(morpholine-4-carbonyl)-6-oxo-hexahydro-pyrrolo[3,2-b]pyrrole-1-carbonyl]-butyl}-benzamide ;
4-amino-N-{3-méthyl-1-[6-oxo-4-(pipérazine-1-carbonyl)-hexahydro-pyrrolo[3,2-b]pyrrole-1-carbonyl]-butyl}-benzamide ;
4-amino-N-{3-méthyl-1-[4-(4-méthyl-pipérazine-1carbonyl)-6-oxo-hexahydro-pyrrolo[3,2-b]pyrrole-1-carbonyl]-butyl}-benzamide ;
4-amino-N-{3-méthyl-1-{4-[2-(4-méthyl-pipérazine-1-yl)-acétyl]-6-oxo-hexahydro-pyrrolo[3,2-b]pyrrole-1-carbonyl}-butyl)-benzamide ;
4-amino-N-{3-méthyl-1-[6-oxo-4-(2-pipérazine-1-yl-acétyl)-hexahydro-pyrrolo[3,2-b]pyrrole-1-carbonyl]-butyl}-benzamide ;
4-amino-N-{1-[4-(furanne-2-carbonyl)-6-oxo-hexahydro-pyrrolo[3,2-b]pyrrole-1-carbonyl]-3-méthyl-butyl}-benzamide ;
4-amino-N-{1-[4-(5-chloro-furanne-2-carbonyl)-6-oxo-hexahydro-pyrrolo[3,2-b]pyrrole-1-carbonyl]-3-méthyl-butyl}-benzamide ;
4-amino-N-{1-[4-(thiophène-2-carbonyl)-6-oxo-hexahydro-pyrrolo[3,2-b]pyrrole-1-carbonyl]-3-méthyl-butyl}-benzamide ;
4-amino-N-{1-[4-(5-chloro-thiophène-2-carbonyl)-6-oxo-hexahydro-pyrrolo[3,2-b]pyrrole-1carbonyl]-3-méthyl-butyl}-benzamide ;
4-amino-N-{1-[4-(furanne-3-carbonyl)-6-oxo-hexahydro-pyrrolo[3,2-b]pyrrole-1-carbonyl]-3-méthyl-butyl}-benzamide ;
4-amino-N-{1-[4-(thiophène-3-carbonyl)-6-oxo-hexahydro-pyrrolo[3,2-b]pyrrole-1-carbonyl]-3-méthyl-butyl}-benzamide ;
4-amino-N-[1-(4-cyclopentanecarbonyl-6-oxo-hexahydro-pyrrolo[3,2-b]pyrrole-1-carbonyl)-3-méthyl-butyl]-benzamide ;
4-amino-N-[1-(4-cyclohexanecarbonyl-6-oxo-hexahydro-pyrrolo[3,2-b]pyrrole-1-carbonyl)-3-méthyl-butyl]-benzamide ;
4-amino-N-{1-[4-(cyclopent-3-ènecarbonyl)-6-oxo-hexahydro-pyrrolo[3,2-b]pyrrole-1-carbonyl]-3-méthyl-butyl}-benzamide ;
4-amino-N-{1-[4-(2-cyclopentyl-acétyl)-6-oxo-hexahydro-pyrrolo[3,2-b]pyrrole-1-carbonyl]-3-méthyl-butyl}-benzamide ;
4-amino-N-{1-[4-(2-cyclohexyl-acétyl)-6-oxo-hexahydro-pyrrolo[3,2-b]pyrrole-1-carbonyl]-3-méthyl-butyl}-benzamide ;
4-amino-N-{3-méthyl-1-[6-oxo-4-(pyrrolidine-2-carbonyl)-hexahydro-pyrrolo[3,2-b]pyrrole-1-carbonyl]-butyl}-benzamide ;
N-{1-[4-(1-acétyl-pyrrolidine-2-carbonyl)-6-oxo-hexahydro-pyrrolo[3,2-b]pyrrole-1-carbonyl]-3-méthyl-butyl}-4-amino-benzamide ;
4-amino-N-{3-méthyl-1-[6-oxo-4-(pipéridine-2-carbonyl)-hexahydro-pyrrolo[3,2-b]pyrrole-1-carbonyl]-butyl}-benzamide ;
N-{1-[4-(1-acétyl-pipéridine-2-carbonyl)-6-oxo-hexahydro-pyrrolo[3,2-b]pyrrole-1-carbonyl]-3-méthyl-butyl}-4-amino-benzamide ;
4-amino-N-{3-méthyl-1-[6-oxo-4-(tétrahydro-furanne-2-carbonyl)-hexahydro-pyrrolo[3,2-b]pyrrole-1-carbonyl]-butyl}-benzamide ;
N-{1-[4-(1-amino-cyclobutanecarbonyl)-6-oxo-hexahydro-pyrrolo[3,2-b]pyrrole-1-carbonyl]-3-méthyl-butyl}-4-amino-benzamide ;
N-{1-[4-(1-amino-cyclopentanecarbonyl)-6-oxo-hexahydro-pyrrolo[3,2-b]pyrrole-1-carbonyl]-3-méthyl-butyl}-4-amino-benzamide ;
N-{1-[4-(1-amino-cyclohexanecarbonyl)-6-oxo-hexahydro-pyrrolo[3,2-b]pyrrole-1-carbonyl]-3-méthyl-butyl}-4-amino-benzamide ;
N-{1-[4-(1-acétylamino-cyclobutanecarbonyl)-6-oxo-hexahydro-pyrrolo[3,2-b]pyrrole-1-carbonyl]-3-méthyl-butyl}-4-amino-benzamide ;
N-{1-[4-(1-acétylamino-cyclopentanecarbonyl)-6-oxo-hexahydro-pyrrolo[3,2-b]pyrrole-1-carbonyl]-3-méhyl-butyl}-4-amino-benzamide ;
N-{1-[4-(1-acétylamino-cyclohexanecarbonyl)-6-oxo-hexahydro-pyrrolo[3,2-b]pyrrole-1-carbonyl]-3-méthyl-butyl}-4-amino-benzamide ;
4-amino-N-{1-[4-(1-hydroxy-cyclobutanecarbonyl)-6-oxo-hexahydro-pyrrolo[3,2-b]pyrrole-1-carbonyl]-3-méthyl-butyl}-benzamide ;
4-amino-N-{1-[4-(1-hydroxy-cyclopentanecarbonyl)-6-oxo-hexahydro-pyrrolo[3,2-b]pyrrole-1-carbonyl]-3-méthyl-butyl}-benzamide ;
4-amino-N-{1-[4-(1-hydroxy-cyclohexanecarbonyl)-6-oxo-hexahydro-pyrrolo[3,2-b]pyrrole-1-carbonyl]-3-méthyl-butyl}-benzamide ;
4-amino-N-{1-[4-(1-méthoxy-cyclobutanecarbonyl)-6-oxo-hexahydro-pyrrolo[3,2-b]pyrrole-1-carbonyl]-3-méthyl-butyl}-benzamide ;
4-amino-N-{1-[4-(1-méthoxy-cyclopentanecarbonyl)-6-oxo-hexahydro-pyrrolo[3,2-b]pyrrole-1-carbonyl]-3-méthyl-butyl}-benzamide ;
4-amino-N-{1-[4-(1-méthoxy-cyclohexanecarbonyl)-6-oxo-hexahydro-pyrrolo[3,2-b]pyrrole-1-carbonyl]-3-méthyl-butyl}-benzamide ;
N-{1-[4-(2-amino-2-cyclopentyl-acétyl)-6-oxo-hexahydro-pyrrolo[3,2-b]pyrrole-1-carbonyl]-3-méthyl-butyl}-4-amino-benzamide ;
N-{1-[4-(2-amino-2-cyclohexyl-acétyl)-6-oxo-hexahydro-pyrrolo[3,2-b]pyrrole-1-carbonyl]-3-méthyl-butyl}-4-amino-benzamide ;
N-{1-[4-(2-acétylamino-2-cyclopentyl-acétyl)-6-oxo-hexahydro-pyrrolo[3,2-b]pyrrole-1-carbonyl]-3-méthyl-butyl}-4-amino-benzamide ;
N-{1-[4-(2-acétylamino-2-cyclohexyl-acétyl)-6-oxo-hexahydro-pyrrolo[3,2-b]pyrrole-1-carbonyl]-3-méthyl-butyl}-4-amino-benzamide ;
N-{1-[4-(2-acétylamino-propionyl)-6-oxo-hexahydro-pyrrolo[3,2-b]pyrrole-1-carbonyl]-3-méthyl-butyl}-4-amino-benzamide ;
N-{1-[4-(2-acétylamino-acétyl)-6-oxo-hexahydro-pyrrolo-[3,2-b]pyrrole-1-carbonyl]-3-méthyl-butyl}-4-amino-benzamide ;
N-(1-{4-[2-(acétyl-méthyl-amino)-acétyl]-6-oxo-hexahydro-pyrrolo-[3,2-b]pyrrole-1-carbonyl}-3-méthyl-butyl)-4-amino-benzamide ;
4-amino-N-{1-[4-(2-diméthylamino-acétyl)-6-oxo-hexahydro-pyrrolo[3,2-b]pyrrole-1-carbonyl]-3-méthyl-butyl}-benzamide ;
N-{1-[4-(2-amino-butyryl)-6-oxo-hexahydro-pyrrolo[3,2-b]-pyrrole-1-carbonyl]-3-méthyl-butyl}-4-amino-benzamide ;
N-{1-[4-(2-acétylamino-butyryl)-6-oxo-hexahydro-pyrrolo-[3,2-b]-pyrrole-1-carbonyl]-3-méthyl-butyl}-4-amino-benzamide ;
N-{1-[4-(2-amino-3-méthyl-butyryl)-6-oxo-hexahydro-pyrrolo[3,2-b]pyrrole-1-carbonyl]-3-méthyl-butyl}-4-amino-benzamide ;
N-{1-[4-(2-acétylamino-3-méthyl-butyryl)-6-oxo-hexahydro-pyrrolo[3,2-b]pyrrole-1-carbonyl]-3-méthyl-butyl}-4-amino-benzamide ;
N-{1-[4-(2-amino-3,3-diméthyl-butyryl)-6-oxo-hexahydro-pyrrolo[3,2-b]pyrrole-1-carbonyl]-3-méthyl-butyl}-4-amino-benzamide ;
N-{1-[4-(2-acétylamino-3,3-diméthyl-butyryl)-6-oxo-hexahydro-pyrrolo[3,2-b]pyrrole-1-carbonyl]-3-méthyl-butyl}-4-amino-benzamide ;
N-{1-[4-(2-amino-3-méthyl-pentanoyl)-6-oxo-hexahydro-pyrrolo[3,2-b]pyrrole-1-carbonyl]-3-méthyl-butyl}-4-amino-benzamide ;
N-{1-[4-(2-acétylamino-3-méthyl-pentanoyl)-6-oxo-hexahydro-pyrrolo[3,2-b]pyrrole-1-carbonyl]-3-méthyl-butyl}-4-amino-benzamide ;
4-amino-N-[3-méthyl-1-(6-oxo-4-pentanoyl-hexahydro-pyrrolo[3,2-b]pyrrole-1-carbonyl)-butyl]-benzamide ;
4-amino-N-{3-méthyl-1-[4-(3-méthyl-pentanoyl)-6-oxo-hexahydro-pyrrolo[3,2-b]pyrrole-1-carbonyl]-butyl}-benzamide ;
4-amino-N-{3-méthyl-1-[4-(4-méthyl-pentanoyl)-6-oxo-hexahydro-pyrrolo[3,2-b]pyrrole-1-carbonyl]-butyl}-benzamide ;
N-{1-[4-(2-amino-4-méthyl-pentanoyl)-6-oxo-hexahydro-pyrrolo[3,2-b]pyrrole-1-carbonyl]-3-méthyl-butyl}-4-amino-benzamide ;
N-{1-[4-(2-acétylamino-4-méthyl-pentanoyl)-6-oxo-hexahydro-pyrrolo[3,2-b]pyrrole-1-carbonyl]-3-méthyl-butyl}-4-amino-benzamide ;
N-{1-[4-(2-amino-4,4-diméthyl-pentanoyl)-6-oxo-hexahydro-pyrrolo[3,2-b]pyrrole-1-carbonyl]-3-méthyl-butyl}-4-amino-benzamide ;
N-{1-[4-(2-acétylamino-4,4-diméthyl-pentanoyl)-6-oxo-hexahydro-pyrrolo[3,2-b]pyrrole-1-carbonyl]-3-méthyl-butyl}-4-amino-benzamide ;
N-{1-[4-(2-amino-pentanoyl)-6-oxo-hexahydro-pyrrolo[3,2-b]-pyrrole-1-carbonyl]-3-méthyl-butyl}-4-amino-benzamide ;

N-{1-[4-(2-acétylamino-pentanoyl)-6-oxo-hexahydro-pyrrolo-[3,2-b]pyrrole-1-carbonyl]-3-méthyl-butyl}-4-amino-benzamide ;
N-{1-[4-(2-amino-5-méthyl-hexanoyl)-6-oxo-hexahydro-pyrrolo[3,2-b]pyrrole-1-carbonyl]-3-méthyl-butyl}-4-amino-benzamide ;
N-{1-[4-(2-acétylamino-5-méthyl-hexanoyl)-6-oxo-hexahydro-pyrrolo[3,2-b]pyrrole-1-carbonyl]-3-méthyl-butyl}-4-amino-benzamide ;
N-{1-[4-(2-hydroxy-3-méthyl-butyryl)-6-oxo-hexahydro-pyrrolo[3,2-b]pyrrole-1-carbonyl]-3-méthyl-butyl}-4-amino-benzamide ;
N-{1-[4-(2-méthoxy-3-méthyl-butyryl)-6-oxo-hexahydro-pyrrolo[3,2-b]pyrrole-1-carbonyl]-3-méthyl-butyl}-4-amino-benzamide ;
4-amino-N-{1-[4-(2-hydroxy-3,3-diméthyl-butyryl)-6-oxo-hexahydro-pyrrolo[3,2-b]pyrrole-1-carbonyl]-3-méthyl-butyl}-benzamide ;
4-amino-N-{1-[4-(2-méthoxy-3,3-diméthyl-butyryl)-6-oxo-hexahydro-pyrro1o[3,2-b]pyrrole-1-carbonyl]-3-méthyl-butyl}-benzamide ;
4-amino-N-{1-[4-(2-hydroxy-3-méthyl-pentanoyl)-6-oxo-hexahydro-pyrrolo[3,2-b]pyrrole-1-carbonyl]-3-méthyl-butyl}-benzamide ;
4-amino-N-{1-[4-(2-méthoxy-3-méthyl-pentanoyl)-6-oxo-hexahydro-pyrrolo[3,2-b]pyrrole-1-carbonyl]-3-méthyl-butyl}-benzamide ;
4-amino-N-{1-[4-(2-hydroxy-4-méthyl-pentanoyl)-6-oxo-hexahydro-pyrrolo[3,2-b]pyrrole-1-carbonyl]-3-méthyl-butyl}-benzamide ;
4-amino-N-{1-[4-(2-méthoxy-4-méthyl-pentanoyl)-6-oxo-hexahydro-pyrrolo[3,2-b]pyrrole-1-carbonyl]-3-méthyl-butyl}-benzamide ;
4-amino-N-{1-[4-(2-hydroxy-4,4-diméthyl-pentanoyl)-6-oxo-hexahydro-pyrrolo[3,2-b]pyrrole-1-carbonyl]-3-méthyl-butyl}-benzamide ;
4-amino-N-{1-[4-(2-méthoxy-4,4-diméthyl-pentanoyl)-6-oxo-hexahydro-pyrrolo[3,2-b]pyrrole-1-carbonyl]-3-méthyl-butyl}-benzamide ;
4-amino-N-{1-[4-(2-hydroxy-pentanoyl)-6-oxo-hexahydro-pyrrolo[3,2-b]pyrrole-1-carbonyl]-3-méthyl-butyl}-benzamide ;
4-amino-N-{1-[4-(2-méthoxy-pentanoyl)-6-oxo-hexahydro-pyrrolo[3,2-b]pyrrole-1-carbonyl]-3-méthyl-butyl}-benzamide ;
4-amino-N-{1-[4-(2-hydroxy-5-méthyl-hexanoyl)-6-oxo-hexahydro-pyrrolo[3,2-b]pyrrole-1-carbonyl]-3-méthyl-butyl}-benzamide ;
4-amino-N-{1-[4-(2-méthoxy-5-méthyl-hexanoyl)-6-oxo-hexahydro-pyrrolo[3,2-b]pyrrole-1-carbonyl]-3-méthyl-butyl}-benzamide ;
N-[1-(4-benzoyl-6-oxo-hexahydro-pyrrolo[3,2-b]pyrrole-1-carbonyl)-3-méthyl-butyl]-4-diéthylaminobenzamide ;
4-diéthylamino-N-{3-méthyl-1-[6-oxo-4-(pyridine-2-carbonyl)-hexahydro-pyrrolo[3,2-b]pyrrole-1-carbonyl]-butyl}-benzamide ;
4-diéthylamino-N-{3-méthyl-1-[6-oxo-4-(1-oxy-pyridine-2-carbonyl)-hexahydro-pyrrolo[3,2-b]pyrrole-1-carbonyl]-butyl}-benzamide ;
4-diéthylamino-N-{3-méthyl-1-[6-oxo-4-(pyridine-3-carbonyl)-hexahydro-pyrrolo[3,2-b]pyrrole-1-carbonyl]-butyl}-benzamide ;
4-diéthylamino-N-{3-méthyl-1-[6-oxo-4-(1-oxy-pyridine-3-carbonyl)-hexahydro-pyrrolo[3,2-b]pyrrole-1-carbonyl]-butyl}-benzamide ;
4-diéthylamino-N-{3-méthyl-1-[6-oxo-4-(pyridine-4-carbonyl)-hexahydro-pyrrolo[3,2-b]pyrrole-1-carbonyl]-butyl}-benzamide ;
4-diéthylamino-N-{3-méthyl-1-[6-oxo-4-(1-oxy-pyridine-4-carbonyl)-hexahydro-pyrrolo[3,2-b]pyrrole-1-carbonyl]-b utyl}-benzamide ;
N-[1-(4-benzènesulfonyl-6-oxo-hexahydro-pyrrolo[3,2-b]-pyrrole-1-carbonyl)-3-méthyl-butyl]-4-diéthylamino-benzamide ;
4-diéthylamino-N-{3-méthyl-1-[6-oxo-4-(pyridine-2-sulfonyl)-hexahydro-pyrrolo[3,2-b]pyrrole-1-carbonyl]-butyl}-benzamide ;
4-diéthylamino-N-{3-méthyl-1-[6-oxo-4-(1-oxy-pyridine-2-sulfonyl)-hexahydro-pyrrolo[3,2-b]pyrrole-1-carbonyl]-butyl}-benzamide ;
4-diéthylamino-N-{3-méthyl-1-[6-oxo-4-(pyridine-3-sulfonyl)-hexahydro-pyrrolo[3,2-b]pyrrole-1-carbonyl]-butyl}-benzamide ;
4-diéthylamino-N-{3-méthyl-1-[6-oxo-4-(1-oxy-pyridine-3-sulfonyl)-hexahydro-pyrrolo[3,2-b]pyrrole-1-carbonyl]-butyl}-benzamide ;
4-diéthylamino-N-{3-méthyl-1-[6-oxo-4-(pyridine-4-sulfonyl)-hexahydro-pyrrolo[3,2-b]pyrrole-1-carbonyl]-butyl}-

benzamide ;
4-diéthylamino-N-{3-méthyl-1-[6-oxo-4-(1-oxy-pyridine-4-sulfonyl)-hexahydro-pyrrolo[3,2-b]pyrrole-1-carbonyl]-butyl}-benzamide ;
4-diéthylamino-N-[3-méthyl-1-(6-oxo-4-phénylacétyl-hexahydro-pyrrolo[3,2-b]pyrrole-1-carbonyl)-butyl]-benzamide ;
phénylamide d'acide 4-[2-(4-diéthylamino-benzoylamino)-4-méthyl-pentanoyl]-3-oxo-hexahydro-pyrrolo[3,2-b]pyrrole-1-carboxylique ;
isobutylamide d'acide 4-[2-(4-diéthylamino-benzoylamino)-4-méthyl-pentanoyl]-3-oxo-hexahydro-pyrrolo[3,2-b]pyrrole-1-carboxylique ;
ester phénylique d'acide 4-[2-(4-diéthylamino-benzoylamino)-4-méthyl-pentanoyl]-3-oxo-hexahydro-pyrrolo[3,2-b]-pyrrole-1-carboxylique ;
ester isobutylique d'acide 4-[2-(4-diéthylamino-benzoylamino)-4-méthyl-pentanoyl]-3-oxo-hexahydro-pyrrolo[3,2-b]-pyrrole-1-carboxylique ;
4-diéthylamino-N-{3-méthyl-1-[6-oxo-4-(pyrrolidine-1-carbonyl)-hexahydro-pyrrolo[3,2-b]pyrrole-1-carbonyl]-butyl}-benzamide ;
4-diéthylamino-N-{3-méthyl-1-[6-oxo-4-(pipéridine-1-carbonyl)-hexahydro-pyrrolo[3,2-b]pyrrole-1-carbonyl]-butyl}-benzamide ;
4-diéthylamino-N-{3-méthyl-1-[4-(morpholine-1-carbonyl)-6-oxo-hexahydro-pyrrolo[3,2-b]pyrrole-1-carbonyl]-butyl}-benzamide ;
4-diéthylamino-N-{3-méthyl-1-[6-oxo-4-(pipérazine-1-carbonyl)-hexahydro-pyrrolo[3,2-b]pyrrole-1-carbonyll-butyl}-benzamide ;
4-diéthylamino-N-{3-méthyl-1-[4-(4-méthyl-pipérazine-1-carbonyl)-6-oxo-hexahydro-pyrrolo[3,2-b]pyrrole-1-carbonyl]-butyl}-benzamide ;
4-diéthylamino-N-(3-méthyl-1-{4-[2-(4-méthyl-pipérazine-1-yl)-acétyl]-6-oxo-hexahydro-pyrrolo[3,2-b]pyrrole-1-carbonyl]-butyl}-benzamide ;
4-diéthylamino-N-{3-méthyl-1-[6-oxo-4-(2-pipérazine-1-yl-acétyl)-hexahydro-pyrrolo[3,2-b]pyrrole-1-carbonyl]-butyl}-benzamide ;
4-diéthylamino-N-{1-[4-(furanne-2-carbonyl)-6-oxo-hexahydro-pyrrolo[3,2-b]pyrrole-1-carbonyl]-3-méthyl-butyl}-benzamide ;
4-diéthylamino-N-{1-[4-(5-chloro-furanne-2-carbonyl)-6-oxo-hexahydro-pyrrolo[3,2-b]pyrrole-1-carbonyl]-3-méthyl-butyl}-benzamide ;
4-diéthylamino-N-{1-[4-(thiophène-2-carbonyl)-6-oxo-hexahydro-pyrrolo[3,2-b]pyrrole-1-carbonyl]-3-méthyl-butyl}-benzamide ;
4-diéthylamino-N-{1-[4-(5-chloro-thiophéne-2-carbonyl)-6-oxo-hexahydro-pyrrolo[3,2-b]pyrrole-1-carbonyl]-3-méthyl-butyl}-benzamide ;
4-diéthylamino-N-{1-[4-(furanne-3-carbonyl)-6-oxo-hexahydro-pyrrolo[3,2-b]pyrrole-1-carbonyl]-3-méthyl-butyl}-benzamide ;
4-diéthylamino-N-{1-[4-(thiophène-3-carbonyl)-6-oxo-hexahydro-pyrrolo[3,2-b]pyrrole-1-carbonyl]-3-méthyl-butyl}-benzamide ;
4-diéthylamino-N-[1-(4-cyclopentanecarbonyl-6-oxo-hexahydro-pyrrolo[3,2-b]pyrrole-1-carbonyl)-3-méthyl-butyl]-benzamide ;
4-diéthylamino-N-[1-(4-cyclohexanecarbonyl-6-oxo-hexahydro-pyrrolo[3,2-b]pyrrole-1-carbonyl)-3-méthyl-butyl]-benzamide ;
4-diéthylamino-N-{1-[4-(cyclopent-3-ènecarbonyl)-6-oxo-hexahydro-pyrrolo[3,2-b]pyrrole-1-carbonyl]-3-méthyl-butyl}-benzamide ;
4-diéthylamino-N-{1-[4-(2-cyclopentyl-acétyl)-6-oxo-hexahydro-pyrrolo[3,2-b]pyrrole-1-carbonyl]-3-méthyl-butyl}-benzamide ;
4-diéthylamino-N-{1-[4-(2-cyclohexyl-acétyl)-6-oxo-hexahydro-pyrrolo[3,2-b]pyrrole-1-carbonyl]-3-méthyl-butyl}-benzamide ;
4-diéthylamino-N-{3-méthyl-1-[6-oxo-4-(pyrrolidine-2-carbonyl)-hexahydro-pyrrolo[3,2-b]pyrrole-1-carbonyl]-butyl}-benzamide ;
N-{1-[4-(1-acétyl-pyrrolidine-2-carbonyl)-6-oxo-hexahydro-pyrrolo[3,2-b]pyrrole-1-carbonyl]-3-méthyl-butyl}-4-diéthylamino-benzamide ;
4-diéthylamino-N-{3-méthyl-1-[6-oxo-4-(pipéridine-2-carbonyl)-hexahydro-pyrrolo[3,2-b]pyrrole-1-carbonyl]-butyl}-benzamide ;
N-{1-[4-(1-acétyl-pipéridine-2-carbonyl)-6-oxo-hexahydro-pyrrolo[3,2-b]pyrrole-1-carbonyl]-3-méthyl-butyl}-4-diéthylamino-benzamide ;
4-diéthylamino-N-{3-méthyl-1-[6-oxo-4-(tétrahydro-furanne-2-carbonyl)-hexahydro-pyrrolo[3,2-b]pyrrole-1-carbonyl]-butyl}-benzamide ;
N-{1-[4-(1-amino-cyclobutanecarbonyl)-6-oxo-hexahydro-pyrrolo[3,2-b]pyrrole-1-carbonyl]-3-méthyl-butyl}-4-diéthylamino-benzamide ;
N-{1-[4-(1-amino-cyclopentanecarbonyl)-6-oxo-hexahydro-pyrrolo[3,2-b]pyrrole-1-carbonyl]-3-méthyl-butyl}-4-diéthylamino-benzamide ;
N-{1-[4-(1-amino-cyclohexanecarbonyl)-6-oxo-hexahydro-pyrrolo[3,2-b]pyrrole-1-carbonyl]-3-méthyl-butyl}-4-diéthylamino-benzamide ;
N-{1-[4-(1-acétylamino-cyclobutanecarbonyl)-6-oxo-hexahydro-pyrrolo[3,2-b]pyrrole-1-carbonyl]-3-méthyl-butyl}-4-diéthylamino-benzamide ;
N-{1-[4-(1-acétylamino-cyclopentanecarbonyl)-6-oxo-hexahydro-pyrrolo[3,2-b]pyrrole-1-carbonyl]-3-méthyl-butyl}-4-diéthylamino-benzamide ;
N-{1-[4-(1-acétylamino-cyclohexanecarbonyl)-6-oxo-hexahydro-pyrrolo[3,2-b]pyrrole-1-carbonyl]-3-méthyl-butyl}-4-diéthylamino-benzamide ;
4-diéthylamino-N-{1-[4-(1-hydroxy-cyclobutanecarbonyl)-6-oxo-hexahydro-pyrrolo[3,2-b]pyrrole-1-carbonyl]-3-méthyl-butyl}-benzamide ;
4-diéthylamino-N-{1-[4-(1-hydroxy-cyclopentanecarbonyl)-6-oxo-hexahydro-pyrrolo[3,2-b]pyrrole-1-carbonyl]-3-méthyl-butyl}-benzamide ;
4-diéthylamino-N-{1-[4-(1-hydroxy-cyclohexanecarbonyl)-6-oxo-hexahydro-pyrrolo[3,2-b]pyrrole-1-carbonyl]-3-méthyl-butyl}-benzamide ;
4-diéthylamino-N-{1-[4-(1-méthoxy-cyclobutanecarbonyl)-6-oxo-hexahydro-pyrrolo[3,2-b]pyrrole-1-carbonyl]-3-méthyl-butyl}-benzamide ;
4-diéthylamino-N-{1-[4-(1-méthoxy-cyclopentanecarbonyl)-6-oxo-hexahydro-pyrrolo[3,2-b]pyrrole-1-carbonyl]-3-méthyl-butyl}-benzamide ;
4-diéthylamino-N-{1-[4-(1-méthoxy-cyclohexanecarbonyl)-6-oxo-hexahydro-pyrrolo[3,2-b]pyrrole-1-carbonyl]-3-méthyl-butyl}-benzamide ;
N-{1-[4-(2-amino-2-cyclopentyl-acétyl)-6-oxo-hexahydro-pyrrolo[3,2-b]pyrrole-1-carbonyl]-3-méthyl-butyl}-4-diéthylamino-benzamide ;
N-{1-[4-(2-amino-2-cyclohexyl-acétyl)-6-oxo-hexahydro-pyrrolo[3,2-b]pyrrole-1-carbonyl]-3-méthyl-butyl}-4-diéthylamino-benzamide ;
N-{1-[4-(2-acétylamino-2-cyclopentyl-acétyl)-6-oxo-hexahydro-pyrrolo[3,2-b]pyrrole-1-carbonyl]-3-méthyl-butyl}-4-diéthylamino-benzamide ;
N-{1-[4-(2-acétylamino-2-cyclohexyl-acétyl)-6-oxo-hexahydro-pyrrolo[3,2-b]pyrrole-1-carbonyl]-3-méthyl-butyl}-4-diéthylamino-benzamide ;
N-{1-[4-(2-acétylamino-propionyl)-6-oxo-hexahydro-pyrrolo-[3,2-b]pyrrole-1-carbonyl]-3-méthyl-butyl}-4-diéthylamino-benzamide ;
N-{1-[4-(2-acétylamino-acétyl)-6-oxo-hexahydro-pyrrolo-[3,2-b]pyrrole-1-carbonyl]-3-méthyl-butyl}-4-diéthylamino-benzamide ;
N-(1-{4-[2-(acétyl-méthyl-amino)-acétyl]-6-oxo-hexahydro-pyrrolo[3,2-b]pyrrole-1-carbonyl}-3-méthyl-butyl)-4-diéthylamino-benzamide ;
4-diéthylamino-N-{1-[4-(2-diméthylamino-acétyl)-6-oxo-hexahydro-pyrrolo[3,2-b]pyrrole-1-carbonyl]-3-méthyl-butyl}-benzamide ;
N-{1-[4-(2-amino-butyryl)-6-oxo-hexahydro-pyrrolo-[3,2-b]-pyrrole-1-carbonyl]-3-méthyl-butyl}-4-diéthylamino-benzamide ;
N-{1-[4-(2-acétylamino-butyryl)-6-oxo-hexahydro-pyrrolo-[3,2-b]pyrrole-1-carbonyl]-3-méthyl-butyl}-4-diéthylamino-benzamide ;
N-{1-[4-(2-amino-3-méthyl-butyryl)-6-oxo-hexahydro-pyrrolo-[3,2-b]pyrrole-1-carbonyl]-3-mêthyl-butyl}-4-diéthylamino-benzamide ;
N-{1-[4-(2-acétylamino-3-méthyl-butyryl)-6-oxo-hexahydro-pyrrolo[3,2-b]pyrrole-1-carbonyl]-3-méthyl-butyl}-4-diéthylamino-benzamide ;
N-{1-[4-(2-amino-3,3-diméthyl-butyryl)-6-oxo-hexahydro-pyrrolo[3,2-b]pyrrole-1-carbonyl]-3-méthyl-butyl}-4-diéthylamino-benzamide ;
N-{1-[4-(2-acétylamino-3,3-diméthyl-butyryl)-6-oxo-hexahydro-pyrrolo[3,2-b]pyrrole-1-carbonyl]-3-méthyl-butyl}-4-diéthylamino-benzamide ;
N-{1-[4-(2-amino-3-méthyl-pentanoyl)-6-oxo-hexahydro-pyrrolo[3,2-b]pyrrole-1-carbonyl]-3-méthyl-butyl}-4-diéthylamino-benzamide ;
N-{1-[4-(2-acétylamino-3-méthyl-pentanoyl)-6-oxo-hexahydro-pyrrolo[3,2-b]pyrrole-1-carbonyl]-3-méthyl-butyl}-4-diéthylamino-benzamide ;
4-diéthylamino-N-[3-méthyl-1-(6-oxo-4-pentanoyl-hexahydro-pyrrolo[3,2-b]pyrrole-1-carbonyl)-butyl]-benzamide ;

4-diéthylamino-N-{3-méthyl-1-[4-(3-méthyl-pentanoyl)-6-oxo-hexahydro-pyrrolo[3,2-b]pyrrole-1-carbonyl]-butyl}-benzamide ;
4-diéthylamino-N-{3-méthyl-1-[4-(4-méthyl-pentanoyl)-6-oxo-hexahydro-pyrrolo[3,2-b]pyrrole-1-carbonyl]-butyl}-benzamide ;
N-{1-[4-(2-amino-4-méthyl-pentanoyl)-6-oxo-hexahydro-pyrrolo[3,2-b]pyrrole-1-carbonyl]-3-méthyl-butyl}-4-diéthylamino-benzamide ;
N-{1-[4-(2-acétylamino-4-méthyl-pentanoyl)-6-oxo-hexahydro-pyrrolo[3,2-b]pyrrole-1-carbonyl]-3-méthyl-butyl}-4-diéthylamino-benzamide ;
N-{1-[4-(2-amino-4,4-diméthyl-pentanoyl)-6-oxo-hexahydro-pyrrolo[3,2-b]pyrrole-1-carbonyl]-3-méthyl-butyl}-4-diéthylamino-benzamide ;
N-{1-[4-(2-acétylamino-4,4-diméthyl-pentanoyl)-6-oxo-hexahydro-pyrrolo[3,2-b]pyrrole-1-carbonyl]-3-méthyl-butyl}-4-diéthylamino-benzamide ;
N-{1-[4-(2-amino-pentanoyl)-6-oxo-hexahydro-pyrrolo[3,2-b]-pyrrole-1-carbonyl]-3-méthyl-butyl}-4-diéthylamino-benzamide ;
N-{1-[4-(2-acétylamino-pentanoyl)-6-oxo-hexahydro-pyrrolo[3,2-b]-pyrrole-1-carbonyl]-3-méthyl-butyl}-4-diéthylamino-benzamide ;
N-{1-[4-(2-amino-5-méthyl-hexanoyl)-6-oxo-hexahydro-pyrrolo[3,2-b]-pyrrole-1-carbonyl]-3-méthyl-butyl}-4-diéthylamino-benzamide ;
N-{1-[4-(2-acétylamino-5-méthyl-hexanoyl)-6-oxo-hexahydro-pyrrolo[3,2-b]-pyrrole-1-carbonyl]-3-méthyl-butyl}-4-diéthylamino-benzamide ;
N-{1-[4-(2-hydroxy-3-méthyl-butyryl)-6-oxo-hexahydro-pyrrolo[3,2-b]-pyrrole-1-carbonyl]-3-méthyl-butyl}-4-diéthylamino-benzamide ;
N-{1-[4-(2-méthoxy-3-méthyl-butyryl)-6-oxo-hexahydro-pyrrolo[3,2-b]-pyrrole-1-carbonyl]-3-méthyl-butyl}-4-diéthylamino-benzamide ;
4-diéthylamino-N-{1-[4-(2-hydroxy-3,3-diméthyl-butyryl)-6-oxo-hexahydro-pyrrolo[3,2-b]pyrrole-1-carbonyl]-3-méthyl-butyl}-benzamide ;
4-diéthylamino-N-{1-[4-(2-méthoxy-3,3-diméthyl-butyryl)-6-oxo-hexahydro-pyrrolo[3,2-b]pyrrole-1-carbonyl]-3-méthyl-butyl}-benzamide ;
4-diéthylamino-N-{1-[4-(2-hydroxy-3-méthyl-pentanoyl)-6-oxo-hexahydro-pyrrolo[3,2-b]pyrrole-1-carbonyl]-3-méthyl-butyl}-benzamide ;
4-diéthylamino-N-{1-[4-(2-méthoxy-3-méthyl-pentanoyl)-6-oxo-hexahydro-pyrrolo[3,2-b]pyrrole-1-carbonyl]-3-méthyl-butyl}-benzamide ;
4-diéthylamino-N-{1-[4-(2-hydroxy-4-méthyl-pentanoyl)-6-oxo-hexahydro-pyrrolo[3,2-b]pyrrole-1-carbonyl]-3-méthyl-butyl}-benzamide ;
4-diéthylamino-N-{1-[4-(2-méthoxy-4-méthyl-pentanoyl)-6-oxo-hexahydro-pyrrolo[3,2-b]pyrrole-1-carbonyl]-3-méthyl-butyl}-benzamide ;
4-diéthylamino-N-{1-[4-(2-hydroxy-4,4-diméthyl-pentanoyl)-6-oxo-hexahydro-pyrrolo[3,2-b]pyrrole-1-carbonyl]-3-méthyl-butyl}-benzamide ;
4-diéthylamino-N-{1-[4-(2-méthoxy-4,4-diméthyl-pentanoyl)-6-oxo-hexahydro-pyrrolo[3,2-b]pyrrole-1-carbonyl]-3-méthyl-butyl}-benzamide ;
4-diéthylamino-N-{1-[4-(2-hydroxy-pentanoyl)-6-oxo-hexahydro-pyrrolo[3,2-b]pyrrole-1-carbonyl]-3-méthyl-butyl}-benzamide ;
4-diéthylamino-N-{1-[4-(2-méthoxy-pentanoyl)-6-oxo-hexahydro-pyrrolo[3,2-b]pyrrole-1-carbonyl]-3-méthyl-butyl}-benzamide ;
4-diéthylamino-N-{1-[4-(2-hydroxy-5-méthyl-hexanoyl)-6-oxo-hexahydro-pyrrolo[3,2-b]pyrrole-1-carbonyl]-3-méthyl-butyl}-benzamide ;
4-diéthylamino-N-{1-[4-(2-méthoxy-5-méthyl-hexanoyl)-6-oxo-hexahydro-pyrrolo[3,2-b]pyrrole-1-carbonyl]-3-méthyl-butyl}-benzamide.

**36.** Composé suivant l'une quelconque des revendications 1 à 35, destiné à être utilisé en médecine.

**37.** Composé suivant l'une quelconque des revendications 1 à 35, destiné à être utilisé dans le traitement de l'ostéoporose, de la maladie de Paget, de maladies gingivales telles que la gingivite et la périodontite, de l'hypercalcémie dans le cas d'une affection maligne, d'une maladie osseuse métabolique, de maladies comportant une dégradation de la matrice ou du cartilage, en particulier de l'arthrose ou de la polyarthrite rhumatoïde, et de maladies néoplasiques.

**38.** Utilisation d'un composé suivant l'une quelconque des revendications 1 à 35 dans la préparation d'un médicament destiné au traitement de l'ostéoporose, de la maladie de Paget, de maladies gingivales telles que la gingivite et la

périodontite, de l'hypercalcémie dans le cas d'une affection maligne, d'une maladie osseuse métabolique, de maladies comportant une dégradation de la matrice ou du cartilage, en particulier de l'arthrose ou de la polyarthrite rhumatoïde, et de maladies néoplasiques.

**39.** Composition pharmaceutique ou vétérinaire, comprenant un ou plusieurs composés suivant l'une quelconque des revendications 1 à 35 et un support acceptable du point de vue pharmaceutique ou vétérinaire.

**40.** Procédé pour la préparation d'une composition pharmaceutique ou vétérinaire suivant la revendication 39, procédé comprenant l'étape consistant à associer le ou les composés actifs au support.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03041649 A
- WO 03037892 A
- WO 03029200 A
- WO 02051983 A
- WO 02020485 A
- US 20020086996 A
- WO 01096285 A
- WO 0109910 A
- WO 0051998 A
- WO 0119816 A
- WO 9924460 A
- WO 0049008 A
- WO 0048992 A
- WO 0049007 A
- WO 0130772 A
- WO 0055125 A
- WO 0055126 A
- WO 0119808 A
- WO 0149288 A
- WO 0147886 A
- WO 02092563 A
- WO 02017924 A
- WO 01095911 A
- WO 0170232 A
- WO 0178734 A
- WO 0009653 A
- WO 0069855 A
- WO 0029408 A
- WO 0134153 A
- WO 0134160 A
- WO 9964399 A
- WO 9805336 A
- WO 9850533 A
- WO 02080920 A
- WO 03042197 A
- WO 03024924 A
- WO 0055144 A
- WO 0055124 A
- WO 0059881 A
- WO 9948911 A
- WO 0109169 A
- WO 03013518 A
- WO 01077073 A
- WO 01068645 A
- WO 02032879 A
- WO 03020278 A
- WO 03020721 A
- WO 9953039 A
- WO 02057270 A
- WO 9404172 A
- EP 0603873 A
- WO 2000 A


### Non-patent literature cited in the description


- **BARRETT, A.J et al.** Handbook of Proteolytic Enzymes. Academic Press, 1998
- **MCGRATH, M. E. et al.** *Protein Science,* 1998, vol. 7, 1294-1302
- **SOMOZA, J. R. et al.** *J. Alo/. Biol.,* 2002, vol. 322, 559-568
- **BERGER, A. ; SCHECTER, I.** *Philos. Trans. R. Soc. Lond. [Biol.],* 1970, vol. 257, 249-264
- **BROMME, D. ; KALETA, J.** *Curr. Pharm. Des.,* 2002, vol. 8, 1639-1658
- **KIM, W. ; KANG, K.** *Expert Opin. Ther. Patents,* 2002, vol. 12 (3), 419-432
- **LEUNG-TOUNG, R. et al.** *Curr. Med. Chem.,* 2002, vol. 9, 979-1002
- **LECAILLE, F. et al.** *Chem. Rev.,* 2002, vol. 102, 4459-4488
- **HERNANDEZ, A. A. ; ROUSH, W. R.** *Curr. Opin. Chem. Biol.,* 2002, vol. 6, 459-465
- **VEBER, D. F. ; THOMPSON, S. K.** *Curr. Opin. Drug Discovery Dev,* 2000, vol. 3 (4), 362-369
- **WITHERINGTON, J.** *Tetrahydrofurans as Selective Cathepsin K Inhibitors,* 1999
- **MARQUIS, R. W. et al.** *J. Med. Chem.,* 2001, vol. 44 (5), 725-736
- **TOROMANOFF, E.** *Tetrahedron Report,* 1980, vol. 36 (96), 2809-2931
- Advanced Organic Chemistry. John Wiley and Sons, 1985
- *Eur. J. Biochem.,* 1984, vol. 158, 9
- **ATHERTON, E. ; SHEPPARD, R. C.** Solid Phase Peptide Synthesis: A Practical Approach. Oxford University Press, 1989
- **BASTOS, M. ; MAEJI, N. J. ; ABELES, R. H.** *Proc. Natl. Acad. Sci. USA,* 1995, vol. 92, 6738-6742

- **JAMES, I. W.** *Tetrahedron,* 1999, vol. 55 (489), 4855-4946
- **BROWN, R. D.** *J. Chem. Soc., Perkin Trans.,* 1998, vol. 1 (19), 3293-3320
- **GOMEZ-VIDAL, J. A. ; SILVERMAN, R. B.** *Org. Lett.,* 2001, vol. 3 (16), 2481-2484
- **LALL, M. S. et al.** *J. Org. Chem.,* 2002, vol. 67, 1536-1547
- **MEIER ; ZELLER.** *Angew. Chem. Intl. Ed. Engl.,* 1975, vol. 14, 32-43
- **WRIGHT, D. L. et al.** *Org. Lett.,* 2000, vol. 2 (13), 1847-1850
- **BOYLE, P. H. et al.** *Tet. Asymm.,* 1995, vol. 6 (2819
- **BALDWIN, J. E. ; FLINN, A.** *Org. Lett.,* 1987, vol. 28, 3605
- **LEE, A. ; HUANG, L. ; ELLMAN, J. A.** *J. Am. Chem. Soc,* 1999, vol. 121 (43), 9907-9914
- **MURPHY, A. M. et al.** *J. Am. Chem. Soc,* 1992, vol. 114, 3156-3157
- **THOMPSON, L. A. ; ELLMAN, J. A.** *Tet. Lett.,* 1994, vol. 35, 9333
- **KICK, E. K. ; ELLMAN, J. A.** *J. Med. Chem.,* 1995, vol. 38, 1427
- **LEY, S. V. et al.** *J. Chem. Soc. Perkin Trans.,* 2000, vol. 1, 3815-4195
- **DEGERBECK, F. et al.** *J. Chem. Soc, Perkin Trans.,* 1993, vol. 1, 11-14
- **HUGHES, D. L.** *Org. React.(N.Y),* 1992, vol. 42, 335-656
- **GRABOWSKA, U. et al.** *J. Comb. Chem.,* 2000, vol. 2 (5), 475-490
- **SOUERS, A. J. et al.** *Synthesis,* 1999, vol. 4, 583-585
- **AZAM et al.** *J. Chem. Soc. Perkin Trans.,* 1996, vol. 1, 621
- **EVANS et al.** *J. Chem. Soc. Perkin Trans.,* 1981, vol. 1 (103), 2127
- **OIKAWA et al.** *Tet. Lett,* 1996, vol. 37, 6169
- **DESJARLAIS, R. L et al.** *J. Am. Chem. Soc,* 1998, vol. 120, 9114-9115
- **SHI, G-P. et al.** *Immunity,* 1999, vol. 10, 197-206
- **POTEMPA, J. et al.** *J. Biol. Chem,* 1998, vol. 262 (6), 2664-2667
- **ATHERTON, E. ; SHEPPARD, R. C.** Solid Phase Peptide Synthesis. IRL Press Ltd, 1989
- **BARRETT, A.J. ; RAWLINGS, N.D. ; WOESSNER, J.F.** Handbook of Proteolytic Enzymes. Academic Press, 1998
- **BARRETT, A.J.** *Biochem. J.,* 1980, vol. 187, 909-912
- **BARRETT, A.J. ; KIRSCHKE, H.** *Methods Enzymol.,* 1981, vol. 80, 535-561
- **BROMME, D. ; STEINERT, . ; FREIBE, S. ; FITTKAU, S. ; WIEDERANDERS, B. ; KIRSCHKE, H.** *Biochem. J.,* 1989, vol. 264, 475-481
- **RANO, T.A.** *Chem. Biol.,* 1997, vol. 4, 149
- **TALANIAN, R.V.** *J. Biol. Chem.,* 1997, vol. 272, 9677
- **LAZEBNIK, Y.A. ; KAUFMANN, S.H. ; DESNOYERS, S. ; POIRER, G.G. ; EARNSHAW, W.C.** *Nature,* 1994, vol. 371, 768-774
- **HAN, Z.** *J. Biol. Chem.,* 1997, vol. 272, 13432
- **TAKAHASHI, A.** *PNAS,* 1996, vol. 93, 8395
- **MARTINS, L.M.** *J. Biol. Chem.,* 1997, vol. 272, 7421
- **NAGATA, S.** *Cell.,* 1997, vol. 88, 355
- **HARRIES, J.L.** *J. Biol. Chem.,* 1998, vol. 273, 27364
- **CAZZULO, J.J. ; CAZZULO FRANKE, M.C. ; MARTINEZ, J. ; FRANKE DE CAZZULO, B.M.** *Biochim. Biophys. Acta.,* 1990, vol. 1037, 186-191
- **CAZZULO, J.J. ; BRAVO, M. ; RAIMONDI, A. ; ENGSTROM, U. ; LINDEBERG, G. ; HELLMAN, U.** *Cell Mol. Biol.,* 1996, vol. 42, 691-696
- **POTEMPA, J. ; DUBIN, A. ; KORZUS, G. ; TRAVIS, J.** *Biochem. J.,* 1988, vol. 263, 2664-2667
- **KEMBHAVI, A.A. ; BUTTLE, D.J. ; RAUBER, P. ; BARRETT, A.J.** *FEBS Lett.,* 1991, vol. 283, 277-280
- **ALVES, L.C.** *Mol. Biochem. Parasitol,* 2001, vol. 116, 1-9
- **GUAMÉ.** *J. Mol. Biol.,* 2000, vol. 302, 1227-1240
- **HALFON ; CRAIK.** Handbook of Proteolytic Enzymes. Academic Press, 1998, 12-21
- **SASAKI.** *J. Biol. Chem.,* 1984, vol. 259, 12489-12494
- **BOSSARD, M.J.** *J. Biol. Chem.,* 1996, vol. 21, 12517-12524
- **SANTAMARIA, I.** *J. Biol. Chem.,* 1998, vol. 273, 16816-16823
- **KLEMENCIC, J et al.** *Eur.J.Biochem.,* 2000, vol. 267, 5404-5412
- **SANDERSON, S.J.** *Biochem. J.,* 2000, vol. 347, 383-388
- **ALVES, L.C.** *Mol. Biochem. Panasitol,* 2001, vol. 116, 1-9
- **KLEMENCIC, J et al.** *Eur. J. Biochem.,* 2000, vol. 267, 5404-5412
- **NICHOLSON, D.W. ; THORNBERRY, N.A.** *TIBS,* 1997, vol. 22, 299-306
- **STENNICKE, H.R. ; SALVESEN, G.S.** *J. Biol. Chem.,* 1997, vol. 272 (41), 25719-25723
- **TALANIAN, R.V.** *J. Biol. Chem.,* 1997, vol. 272 (15), 9677-9682
- **WOLF, B.B. ; GREEN, D.R.** *J. Biol. Chem.,* 1999, vol. 274 (29), 20049-20052
- **CORNISH-BOWDEN, A.** Fundamentals of enzyme kinetics. Portland Press, 1995, 93-128
- **MORRISON, J.F.** *Trends Biochem. Sci.,* 1982, vol. 7, 102-105
- **MORRISON, J.F.** *Biochim. Biophys. Acta,* 1969, vol. 185, 269-286

- **STONE, S.R. ; HOFSTEENGE, J.** *Biochemistry,* 1986, vol. 25, 4622-4628
- **MORRISON, J.F.** *TIBS,* 1982, 102-105
- **TIAN, W.X. ; TSOU, C.L.** *Biochemistry,* 1982, vol. 21, 1028-1032
- Advances in Enzymol.,. 1988, vol. 61, 201-301
- Advances in Enzymol.,. 1988, vol. 61, 381-436